# EUROPEAN PATENT APPLICATION

(11) **EP 1 686 175 A2**
(43) Date of publication of application: **02.08.2006**
(21) Application number: 06004078.9
(22) Date of filing: 26.02.2002
(51) Int. Cl.: C12N 15/12, C07K 14/47, C07K 16/18, C12Q 1/68, A61K 38/17, A61P 3/00, A61P 21/00, A61P 3/10, A61K 39/395, G01N 33/68

(54) **Proteins and nucleic acids encoding same**

(30) Priority: 26.02.2001 US 271646 P; 27.02.2001 US 271840 P; 28.02.2001 US 272405 P; 28.02.2001 US 272414 P; 28.02.2001 US 272404 P; 28.02.2001 US 272410 P; 02.03.2001 US 273300 P; 02.03.2001 US 273048 P; 02.03.2001 US 272787 P; 02.03.2001 US 272922 P; 16.03.2001 US 276401 P; 20.03.2001 US 277324 P; 20.03.2001 US 278660 P; 30.03.2001 US 280234 P; 30.03.2001 US 280039 P; 02.04.2001 US 280818 P; 12.04.2001 US 283443 P; 23.04.2001 US 285754 P; 24.04.2001 US 286096 P; 03.05.2001 US 288353 P; 17.05.2001 US 291703 P; 31.05.2001 US 294834 P; 20.06.2001 US 299695 P; 21.06.2001 US 299845 P; 05.07.2001 US 303242 P; 13.08.2001 US 311981 P; 16.08.2001 US 312858 P; 17.08.2001 US 313280 P; 29.08.2001 US 315614 P; 17.09.2001 US 322818 P; 25.02.2002 US 85198 P
(62) Divisional of application: 02715003.6
(71) Applicant: Curagen Corporation, New Haven, CT 06511 (US)
(72) Inventor: Alsobrook, John P. II, Madison, CT 06443 (US); Anderson, David W., New Haven, CT 06511 (US); Ballinger, Robert A., Newington, CT 06111 (US); Boldog, Ferenc L., North Haven, CT 06473 (US); Burgess, Catherine E., Wethersfield, CT 06109-4102 (US); Casman, Stacie J., North Haven, CT 06473 (US); Ellerman, Karen E., Branford, CT 06405 (US); Gangolli, Esha A., Madison, CT 06443 (US); Gerlach, Valerie L., Branford, CT 06405 (US); Gilbert, Jennifer A., Madison, CT 06443 (US); Gorman, Linda, Branford, CT 06405 (US); Guo, Xiaojia, Branford, CT 06405 (US); Gusev, Vladimir Y., Madison, CT 06443 (US); Kekuda, Ramesh, Stamford, CT 06902 (US); Li, Li, Branford, CT 06405 (US); Liu, Xiaohong, Branford, CT 06405 (US); Malyankar, Uriel M., Branford, CT 06405 (US); Miller, Charles E., Guilford, CT 06437 (US); Millett, Isabelle, Milford, CT 06460 (US); Padigaru, Muralidhara, Malad Mumbai, 400-064 (IN); Patturajan, Meera, Branford, CT 06405 (US); Pena, Carol E. A., New Haven, CT 06511 (US); Peyman, John A., New Haven, CT 06515 (US); Rastelli, Luca, Guilford, CT 06437 (US); Shenoy, Suresh G., Branford, CT 06405 (US); Shimkets, Richard A., Guilford, CT 06437 (US); Smithson, Glennda, Guilford, CT 06435 (US); Spytek, Kimberly A., New Haven, CT 06511 (US); Stone, David J., Guilford, CT 06437 (US); Taupier, Raymond J., Jr., East Haven, CT 06512 (US); Tchernev, Velizar T., Branford, CT 06405 (US); Vernet, Corine A. M., North Branford CT 06471 (US); Zerhusen, Bryan D., Branford, CT 06405 (US)
(74) Representative: Naylor, Kathryn May

(57) **Abstract**

Disclosed herein are nucleic acid sequences that encode novel polypeptides. Also disclosed are polypeptides encoded by these nucleic acid sequences, and antibodies, which immunospecifically-bind to the polypeptide, as well as derivatives, variants, mutants, or fragments of the aforementioned polypeptide, polynucleotide, or antibody. The invention further discloses therapeutic, diagnostic and research methods for diagnosis, treatment, and prevention of disorders involving any one of these novel human nucleic acids and proteins.

### Annex to the application documents - subsequently filed sequences listing

SEQUENCE LISTING

<110> CuraGen Corp. et al.

<120> Proteins and Nucleic Acids Encoding Same

<130> P25928EP-D1-PCT-KMN/ARC

<140> EP06004078.9
<141> 2002-02-26

<150> 10/085,198
<151> 2002-02-25

<150> 60/271,646
<151> 2001-02-26

<150> 60/276,401
<151> 2001-03-16

<150> 60/311,981
<151> 2001-08-13

<150> 60/312,858
<151> 2001-08-16

<150> 60/271,840
<151> 2001-02-27

<150> 60/277,324
<151> 2001-03-20

<150> 60/286,096
<151> 2001-04-21

<150> 60/299,695
<151> 2001-06-20

<150> 60/315,614
<151> 2001-08-29

<150> 60/272,405
<151> 2001-02-28

<150> 60/272,410
<151> 2001-02-28

<150> 60/278,660
<151> 2001-03-20

<150> 60/272,414
<151> 2001-02-28

<150> 60/280,234
<151> 2001-03-30

<150> 60/272,404
<151> 2001-02-28

<150> 60/280,039
<151> 2001-03-30

<150> 60/313,280
<151> 2001-08-17

<160> 655

<170> PatentIn Ver. 2.1

<210> 1
<211> 12660
<212> DNA
<213> Homo sapiens

<400> 1
atgctactcc ctgccctcct ctttgggatg gcgtgggccc tggctgacgg gcggtggtgt 60
gagtggacag agaccatccg tgtggaggag gaagtggcac cccgtcagga ggacctggta 120
ccctgtgcca gcctcgacca ttacagccgc ctgggctggc ggctggacct gccctggagt 180
ggccgctcgg ggcttacccg gtccccagcg cctgggctct gtcctatcta caaacctcca 240
gaaacccggc ctgccaagtg gaaccggaca gtgaggactt gttgcccagg ctgggggggc 300
gcccactgca ctgaggccct tgccaaagcc agtcctgaag gccactgctt tgccatgtgg 360
cagtgccagc tacaggcagg ctcagctaat gcctcagcag gaagcctgga ggagtgctgc 420
gcccggccct ggggacgaag ctggtgggat ggcagctccc aggcctgccg cagctgctcc 480
agccgacacc tgccaggcag tgcctcttct ccagccctcc tgcagcccct ggcaggggct 540
gtgggccagc tctggagcca gcaccagcgt ccctcggcca cctgtgcctc ctggtcgggc 600
ttccactacc gcacctttga tggccgccac tatcacttcc tgggccgctg cacctacctg 660
ctggcgggtg ctgcggactc cacctgggct gtccacctaa cacccgggga ccgctgcccc 720
cagcctggac actgtcagcg ggtccaggtg actatgggac ccgaggaggt gctgatccag 780
gctggaaatg tgtctgtgaa ggggcagctg gtacctgaag ggcagtcttg gctgctccac 840
gggctgagcc tgcaatggct gggggactgg ctggtgctgt caggaggcct gggggtcgtg 900
gtgcggctgg acaggactgg ctccatctcc atctctgtgg accacgagct ctggggacag 960
acacaaggcc tctgtgggct ctacaatggc tggccagagg atgacttcat ggagccaggc 1020
ggagggctgg ccatgttagc agccaccttt ggaaattcct ggaggctccc tggctcggag 1080
gtttcccccg ctgagtacca cgaggcctgt ctctttgcct actgcgcagg ggccatggca 1140
ggcagtgggc aagaggggcg gcagcaggct gtttgtgcca cctttgccag ctatgtccag 1200
gcctgtgcca ggcggcacat ccacattcgc tggaggaagc ctggcttctg cgagcgcctg 1260
tgccccgggg gccagctcta ctccgactgc gtctccctct gcccacccag ctgcgaggcg 1320
gtgggtcagg gagaggagga gtcctgcagg gaagagtgtg tgagtggctg tgagtgcccg 1380
cgaggcctct tctggaatgg caccctctgt gtgcctgctg cccactgccc ctgctactac 1440
tgccgccagc gctatgtacc cggtgacacc gtgcgccagc tgtgtaaccc ctgcgtgtgc 1500
agggatggcc gctggcactg tgcccaggca ctgtgccccg ccgagtgtgc agtgggtggg 1560
gacgggcact acctcacctt cgatgggcgg agctactcct tctggggtgg tcaaggttgc 1620
cgctacagcc tggtgcagcc ctatccttcc acgtgcccca cccccaccat cagacccccct 1680
gttccaggag ctgtgctggt caatgggcag gatgtgggct tgccctggat tggcgctgag 1740
ggcctcagtg tgcgccgagc ttcctctgcc tttctgctgc tgcgctggcc tggggcccag 1800
gtgctctggg gactgtctga ccctgtagcc tacatcaccc tggacccccg ccatgcccac 1860
caggtgcagg gtctgtgtgg caccttcacc cagaaccagc aggacgactt cctgacacca 1920
gccggagatg tggaaactag cattgctgcc tttgctagca agttccaggt ggccggcaag 1980
ggaagatgcc cctctgagga cagtgccctg ctgtctccct gcaccaccca ctcccagcgc 2040
cacgccttcg cagaggcggc ctgtgccatc ctgcacagct ctgtcttcca ggaatgccac 2100
aggctggtgg acaaagagcc attctatctg cgctgcctgg cagccgtgtg tggctgtgat 2160
cccggcagtg actgcctgtg cccggtgctg tctgcctatg cgcgtcgctg tgcccaggaa 2220
ggtgcctcac ctccctggag gaaccagacc ctctgccctg ttatgtgtcc tggtggccag 2280
gagtaccgag agtgtgcccc agcatgcggt caacactgcg ggaaaccaga ggactgtgga 2340
gagctgggca gctgtgtggc tggttgtaac tgtcctctgg ggctgctgtg ggaccctgag 2400
ggccagtgtg tgccccccag cttgtgcccc tgccagctcg gagcccgtcg ctatgcccct 2460
ggcagtgcca ccatgaagga gtgcaaccgc tgggagcttg tctatgcccc tggtgcctgt 2520
ctcctcacct gtgacagccc cagcgccaat cactcctgcc ctgcaggcag tactgatggc 2580
tgtgtctgtc caccaggcac ggtgctgctg gacgagcgct gtgtgcctcc tgacctctgt 2640
ccctgccgtc acagtgggca gtggtacctg cccaacgcca ccatccagga agactgcaac 2700
gtttgcgtgt gccggggccg gcagtggcac tgcacaggcc agcggcgcag tgggcggtgc 2760
caggcatcag gcgcccccca ctatgtgaca tttgacggac tggccttcac ctatcctggg 2820
gcctgcgagt atctgctggt gcgagaggcc agtggcctat tcacagtctc tgcccagaac 2880
ctgccctgtg gggccagcgg tctcacctgc accaaagcgc tggccgtgcg tctggagggc 2940
actgttgtgc acatgctcag agggactcgg gtcctggtgc aactgtcccc tcagttccgt 3000
ggtcgcgtgg ctgggctgtg tggtgacttt gatggagatg ccagtaatga tctgcggagc 3060
cgccagggcg tcctggagcc cacagctgaa ctggctgccc actcctggcg cctcagcccc 3120
ctctgccctg agccaggaga cctgccacac ccctgcacga tgaacacaca ccgggctggt 3180
tgggctcggg cccgctgtgg ggcgctgctg cagccgctct tcacattatg ccacgcggag 3240
gtcccccgc agcagcacta tgagtggtgc ctgtatgacg cctgcggctg cgactcgggg 3300
ggtgactgtg agtgcctctg ctcggccatt gccacctatg cagatgagtg tgcccggcat 3360
gggcaccacg tgcgctggcg tagccaggag ctctgctgtc tacaccagac accctgtgcc 3420
ctccatggtg gtcaccttgg ccagccagcc tggtgtggtt gcatcctttt gcccttgtgc 3480
ctcagtgatc caaggctctc tcctctccac ccagccctgc agtgtgaagg gggacaggta 3540
tatgaggcct gtggccccac gtgtcccccc acctgccatg agcagcatcc tgagcccggg 3600
tggcactgcc aggtggtggc ctgtgtggag ggctgcttct gccccgaggg gactctgctg 3660
cacggacgct gtgtgctcag cacgtgccag gaaggtcaat ggcattgtgg gggtgacggt 3720
ggccactgtg aggagcttgt gcctgcctgt gcagagggag aggccctgtg ccaagagaat 3780

gggcactgtg tgccccatgg gtggctttgt gacaaccagg acgactgtgg cgatggctct 3840
gatgaggagg gtgagtgtct ttgcccatgc gtggaagcga cagggttggt cagtccttgc 3900
acatgttgtg ccgccccagg ctgtggggag gggcagatga cttgcagctc cggccactgc 3960
ctgcccctgg ccctgctctg tgaccgccag gatgactgtg gagatggcac ggatgagccg 4020
agctatccgt gcccccaggg cttgctggcc tgtgccgatg gacgctgcct gccgccggcc 4080
ctgctctgcg atgggcatcc tgactgtctg gatgccgccg acgaggagtc ctgtctgggg 4140
caggtgacct gcgtccccgg ggaggtgtcc tgtgttgatg gcacctgcct gggggccatc 4200
cagctgtgtg acggagtctg ggactgccca gatggagccg atgaggggcc gggacactgc 4260
cccctacctt ctctgcccac acctcctgcc agcaccttgc ctggcccctc cccaggctcc 4320
ctggacactg cgtcaagtcc cctggccagc gccagccctg cgccaccctg cggccccttc 4380
gagtttcggt gcggcagcgg cgagtgcacc ccgcggggct ggcgctgcga ccaggaggaa 4440
gactgcgccg acggcagcga cgagcgcggc tgcggagggc cctgcgcgcc gcaccacgcg 4500
ccctgcgccc gcggccctca ctgcgtgtcc cccgagcagc tgtgcgacgg cgtgcggcag 4560
tgtcccgacg gctcggacga gggccccgac gcctgcgttg aggctcccgc gcccccggcc 4620
atgcgcggcc cccctggcca agccggcggg cccacctctt cccgagcgcc atccccacct 4680
tcgcctcctg aggcacaggg agagggcagg aagggacagg agcggagcag gacacatctc 4740
acagtgcccg caggctccac ccagctgcct ctgtgccctg gcctctttcc ctgtggtgtg 4800
gctccggggc tgtgcctgac ccctgagcag ctctgtgatg ggatcccaga ctgtccccag 4860
ggcgaggacg agctggactg cgggggggctg ccagccctgg gaggccccaa caggacaggg 4920
cttccctgcc cagaatacac ctgccccaat ggcacctgca taggcttcca gctggtgtgt 4980
gatgggcagc ctgactgtgg aaggccaggg caggtgggcc cctccccaga agagcagggt 5040
tgtggggcct ggggcccctg gagcccatgg gggccctgca gccggacgtg tgggccctgg 5100
ggccagggcc ggagccgccg ctgctcccca ctcggcctcc tggtgctaca gaactgccca 5160
gggcctgagc accagtctca ggcctgcttc acggcagcct gcccagtgga cggtgaatgg 5220
agcacctggt cccccctggtc tgtgtgctct gagccgtgca ggggcaccat gacgcggcaa 5280
cggcagtgcc actcacccca gaatgggggc cgcacctgtg ctgcactgcc cggaggcctg 5340
cacagcaccc gccagaccaa gccttgccct caggacggct gccccaatgc cacttgctct 5400
ggggagctga tgttccagcc ctgtgccccc tgcccactga cctgtgatga catctctggc 5460
caggtcacgt gcccacctga ttggccctgc ggcagcccgg gctgctggtg cccagaaggg 5520
caggtgctgg gcagcgaggg gtggtgtgtg tggccccggc agtgcccctg cctggtggac 5580
ggtgcccgct actggcctgg gcaacgcatc aaggccgact gccagctctg catctgccaa 5640
gacggacggc cccgacgctg ccgactcaac ccggactgcg ctggtgaggc ccttccctcg 5700
gggtccctag tcctctccct ggaccgccca gctgcacatc caccacctcc ttcaggctct 5760
gactgttggc cctccctcag tggactgtgg ctggtcctcc tggtcaccct gggccaagtg 5820
cctgggcccc tgtggaagcc agagcatcca gtggtccttc cggagctcca acaaccccg 5880
cccctccggc cgaggtcgcc agtgccgtgg catccaccgc aaggcacgca gacggagccc 5940
tgtgaggggt gtgagcatca gggccaggtc caccgtgtcg ggaacgctgg gcatgggggc 6000
ccctgcaggg tgtgccagtg tctgcacaac ctcaccgcac actgctcacc ctactgcccg 6060
ctcggcagct gcccccaggg ctgggtcttg gtggagggga cgggagaatc atgctgccac 6120
tgtgccctac ctggagagaa ccagacggtc cagcccatgg ccactcctgc cgcagctccg 6180
gctcccagtc cccagatcag attccctttg gccacttaca ttctgcctcc gtcaggaggc 6240
tcctgccgcc ctctgtcctc ccctactcca gcctgtctct ctcttctgca cccagacccc 6300
tgctattctc ccctggggct ggccggactg gctgagggga gtctgcatgc atcgtcccag 6360
cagctggaac accccaccca ggctgccctc ctgggggctc ccacccaggg gcccagccct 6420
cagggatggc acgctggagg ggatgcttat gccaagtggc acactcggcc ccattacctg 6480
cagctggacc tgcttcagcc tcggaacctc actggcatcc tagtgccgga gactggctcc 6540
tccaacgcat atgccagcag cttctcactc cagttcagca gcaatggtct acactggcat 6600
gactatcgtg acctcctgcc tggcatcttg cccctgccca aggtatcacc cgcccaaggc 6660
cgatggggcc agcagcccac catgcccttt tgtgggttcc atagtctttg tccccaaggg 6720
ccttccagtg tccccgaggg gcatggcctg cattcgatgc ttgttgaata cctgcttttc 6780
cccagaaact gggatgacct ggaccctgcc gtatggactt tcggccgcat ggtgcaggcg 6840
aggtttgtca gggtgtggcc ccacgatgtc caccacagcg atgtccccct gcaggtggag 6900
ctgctgggct gcgagccagg ggttggactc cgctgtgcca gtggtgagtg tgtcctgaga 6960
gggggccctt gtgacggtgt tctggactgc gaggatggct cggatgagga gggctgtgtg 7020
ttgctgcctg agggcactgg caggtatact gtggccggcc gtgcagctca cgcccttggc 7080
ctggcctttg aggggacagc catgtgggag ggcccggca ctgccttcac ccccaaggtg 7140
cccagaccct gcatgctgag gagctgcagc cggggcctgg cagagactga gcactggccc 7200
cctgggcagg aatcccccac gtccccgaca gaagcctggg ataccctctc gagggccccc 7260
accttccttt cctgggaagg ggagctgggc aagcctcacc tccctctacc tacacccaca 7320
gagacaaggc ccgtgagtcc tggcccagcc tccggggtgc ctcaccatgg ggaatctgtg 7380
cagatggtga ccaccacccc catacccag atggaggcca ggaccctgcc accaggtatg 7440
gcagctgtga cggtggtgcc cccacaccct gtgactccag cgacccctgc tggtaagatg 7500
ccaagccctt ctacctgtgt gcaaggcctc tgccagagcg tcgccccagg acccttccca 7560
cctgtgcagt gtggccccgg ccagacgccc tgtgaggtgc tgggctgcgt ggaacaggcg 7620
caggtgtgtg atggcaggga ggattgcctc gacggctccg acgagaggca ctgcggtgag 7680
ctgctggagg gcctgctgtc ctgtggggcc ctctgttccc cgagccagct gagctgtggc 7740
agcggggagt gtctgtctgc tgagcggcgc tgtgacctgc ggcctgactg ccaggatggc 7800
tcggacgagg atggctgtgt ggactgcgtg ctggccccct ggtctgtctg gagcagctgc 7860
agccgcagct gtggcctggg cctcaccttc cagcgccagg agctgctgcg gcctcctctg 7920

ccaggggca gctgcccgcg tgaccggttc cgaagccagt cctgctttgt gcaggcctgc 7980
ccagtggctg ggcatgggc catgtgggag gcctggggac cctgcagcgt ctcctgcggg 8040
ggtggccatc agagtcgcca gagaagctgt gtggaccccc cacccaagaa tggcggtgcc 8100
ccctgccccg ggcctccca agagagggca ccctgcggct tgcagccctg ctcaggtggc 8160
acaggtaagg gggtgctggg ctggggacac ggagggagca ctgtgggcac ggggcgattg 8220
ggcctcccag cacctaggct cacctggtgc ccatccccca ccagactgcg agctgggccg 8280
tgtgtatgtg agtgccgatc tgtgccagaa ggggctggtg cccccatgcc caccctcctg 8340
cctggatccc aaggccaaca gaagctgcag tgggcattgt gtggaagcct cccctccctg 8400
ctgtgcccct tggggctgag tgccctcttc cacatactcc caggatgccg ctgtcccccg 8460
gggctccttc tgcatgacac tcgctgcctg cccctctctg agtgcccctg cctggtgggc 8520
gaagagctga agtggccagg ggtgtccttc ctcctgggca actgcagcca atgcgtgtgt 8580
gagaaggggg agttgctgtg ccaaccaggg ggctgccccc tgccctgcgg ctggtcagcc 8640
tggtcctcct gggctccctg cgaccgctcc tgtggctctg gagtgagggc caggttcagg 8700
tctccctcca accctccggc agcctggggg ggtgccccgt gtgaaggtga ccggcaggaa 8760
ctgcagggct gccacacagt gtgtgggaca ggtatagccg ggagcctggg tgcaggagtc 8820
cccccttcct cctcccaatt ctgtaccctg agaacacatg ggatgggacc cactgaccac 8880
tctacgtggg ggattgaggt gttcggctgg acgccctgga cttcctggtc ctcctgctcc 8940
caaagctgcc ttgccccggg agggggccct ggctggcgca gtcgttcccg actctgcccc 9000
agccctgggg attcatcctg cccaggagat gccacccagg aggagccctg cagcccccct 9060
atagagtgta cgggcttctg cgcccccggc tgcacctgcc cccctggtct tttcctgcac 9120
aatgctagct gcctgccccg cagccagtgc ccctgccagc tgcacgggca gctctatgca 9180
tcaggagcaa tggctcgcct ggactcctgc aacaactgca cctgtgtctc tggtaagatg 9240
gcatgcacct cggagcgctg cccagtggcc tgtggctgga gtccctggac cctgtggagt 9300
ctctgtagct gcagctgcaa cgtgggcatt cggcgccgct tccgggcagg cactgcaccc 9360
ccagctgcct ttgggggtgc tgagtgccaa ggccccacca tggaggctga attctgcagc 9420
ctgcggccat gtccagggcc agtgcctggc atgtgtccca gggacaagca gtggctggac 9480
tgtgcccagg gccctgcctc ttgtgcagag ctcagcgccc caagagggac taaccagacc 9540
tgccaccctg gctgccactg cccctctggg atgcttctgc tggtgagccc acgtggtcac 9600
cctggacccc ttggagccag tgttcagcct cctgtggccc tgcccggtgc catcggcacc 9660
ggttctgtgc caggagctgg gggatggggt ccatgggggc cctggtccca ctgtagccgg 9720
agctgtgggg gaggcctgcg gagccggacc cgggcctgtg accagccccc accccagggc 9780
ctgggggatt actgcgaggg gccacgggca cagggggagg tctgccaggc tctgccctgc 9840
ccagtgacca actgcactgc cattgaaggg gccgagtata gcccctgtgg ccctccgtgc 9900
cctcgctcct gtgatgacct agtgcactgc gtgtggcgct gccagcctgg ctgctactgc 9960
ccaccaggcc aggtactgag ttccaacggg gccatctgcg tgcagccggg tcactgcagc 10020
tgcctggacc tgctgaccgg gcagcggcac catccgggtg ctcggctggc aaggcctgac 10080
ggctgcaacc actgcacctg cctggagggg aggctgaact gcacagacct gccctgccca 10140
gactgcgggg gtggccagag tctgcatccc tgtgggcagc cctgcccccg ctcctgccag 10200
gacctgtccc ctgggagtgt gtgccagcca ggctctgtgg gctgccagcc cacttgtggg 10260
tgcccccctgg gccagctctc ccaggacggg ctgtgcgtgc cccagcccca ctgccgctgc 10320
cagtaccagc ctggagccat ggccccctcc tttgtcccca gcacctgtgt ggcaggcatt 10380
ctgcaatgcc aggaggtgcc tgactgcccg gaccctgggg tgtggagctc ttggggccct 10440
tgggaagact gcagtgtttc gtgtgggggc ggggagcagc tgcgctcccg gcgctgtgct 10500
cgtcctccct gcccagggcc tgcccgccag agccgcacat gcagcacaca ggtctgcaga 10560
gaggcaggct gcccggctgg ccgcctgtac cgtgaatgcc agcccggcga gggatgcccc 10620
ttctcctgcg cccacgtcac gcagcaggtg ggctgcttct ctgagggctg cgaggagggc 10680
tgccactgcc ccgagggcac cttccagcac cgcctggcct gtgtgcagga gtgcccttgt 10740
gtgctgacag cctggctgct gcaggagctg ggagccacca taggtgaccc tggtcagccc 10800
ctcgggcctg gagatgagct ggactcaggc cagacacttc gtacaagctg tggcaactgc 10860
tcgtgtgcac acgggaagct gtcttgctcc ctggacgact gcttcgaggc cgatggtggt 10920
ttcggtccct ggagcccgtg gggcccgtgc tcccgctcct gtggagggct gggcacccgt 10980
acccgcagcc gccagtgtgt gctcaccatg cccaccctca gtgagctgcc cgtgtgccct 11040
ggccctggct gtggggctgg gaactgttcc tggacctcct gggccccgtg ggaaccttgc 11100
tcccgcagct gcggagtggg ccagcagcgc cgcctgcggg cataccgtcc ccctgggccc 11160
ggcgggcact ggtgccccaa catccttact gcctaccaag agcgccgctt ctgcaacctg 11220
cgagcctgcc cagaggccgg ctgcccagca ggcatggagg tggtcacctg tgccaaccgc 11280
tgcccccgcc gctgctcaga cctccaggag ggaattgtgt gtcaggacga ccaggtctgc 11340
cagaagggct gccgctgccc aaaggggtcc ctggagcagg atggtggctg cgtgccaatt 11400
gggcactgtg actgcaccga tgcccagggc cacagctggg ccccggggag ccagcaccag 11460
gatgcctgca acaactgctc atgccaagct gggcagctct cctgcacggc tcagccctgc 11520
ccgcctccca cccactgtgc ctggagccac tggtcggcct ggagtccctg cagccactca 11580
tgcgggccca gagggcagca gagccgcttc cgctgcggcc cgggcctggc ctctcgctct 11640
gggtcctgcc cctgcctgat ggccaaggcc gaccccacct gcaacagcac cttcctccac 11700
ctggacaccc agggctgcta ctcagggccc tgcccagact catgccagtg gagtctgtgg 11760
gggccatgga gcccctgcca ggtgccctgc agtgggggt tcaggctacg ctggagagag 11820
gcagaggccc tctgtggagg aggctgccgg gagccatggg ctcaagacag aaagctgcaa 11880
cggagggccc tgcccagcac ctgtgtcaac gagtccctgg tgtgcccaca ccaggagtgt 11940
ccagtccttg ggccttggtc agcctggagc agttgctcgg ccccctgtgg tgggggcact 12000
atggagcgac atcggacttg tgaggggggt cctggggtgg caccatgcca ggcccaggac 12060

acagagcaac ggcaggagtg taacctgcag ccctgccctg agtgcccccc tggccaggtg 12120
cttagtgcct gtgccacctc atgcccgtgc ctctgctggc atctgcagcc tggtgccatc 12180
tgtgtgcagg agccctgcca gcctggctgt ggctgccctg gagggcagca ttctctgccc 12240
tggggcctca ccctgaccct ggaagagcag gcccaggagc tgcccccagg gactgtgctc 12300
acccggaact gcacccgctg tgtctgccac ggtggagcct tcagctgctc cctcgttgac 12360
tgtcagggtg agatagtgcc ccctggggaa acgtggcagc aggtggcccc gggggagctg 12420
gggctctgcg agcagacgtg cctggagatg aacgccacaa agacccagag taactgcagt 12480
tcagctcgag cctcgggctg cgtgtgccag cccgggcact tccgcagcca ggcaggcccc 12540
tgcgtccccg aagaccactg cgagtgctgg caccttgggc gtccccacct ggtgagacac 12600
cgaacccccct ctgctaccac tcacccattc ctgaccccaa gcctccccat ctgtctgtaa 12660

<210> 2
<211> 4219
<212> PRT
<213> Homo sapiens

<400> 2
   Met Leu Leu Pro Ala Leu Leu Phe Gly Met Ala Trp Ala Leu Ala Asp
   1               5                   10                  15
   Gly Arg Trp Cys Glu Trp Thr Glu Thr Ile Arg Val Glu Glu Glu Val
               20                  25                  30
   Ala Pro Arg Gln Glu Asp Leu Val Pro Cys Ala Ser Leu Asp His Tyr
           35                  40                  45
   Ser Arg Leu Gly Trp Arg Leu Asp Leu Pro Trp Ser Gly Arg Ser Gly
       50                  55                  60
   Leu Thr Arg Ser Pro Ala Pro Gly Leu Cys Pro Ile Tyr Lys Pro Pro
   65                  70                  75                  80
   Glu Thr Arg Pro Ala Lys Trp Asn Arg Thr Val Arg Thr Cys Cys Pro
                   85                  90                  95
   Gly Trp Gly Gly Ala His Cys Thr Glu Ala Leu Ala Lys Ala Ser Pro
               100                 105                 110
   Glu Gly His Cys Phe Ala Met Trp Gln Cys Gln Leu Gln Ala Gly Ser
           115                 120                 125
   Ala Asn Ala Ser Ala Gly Ser Leu Glu Glu Cys Cys Ala Arg Pro Trp
       130                 135                 140
   Gly Arg Ser Trp Trp Asp Gly Ser Ser Gln Ala Cys Arg Ser Cys Ser
   145                 150                 155                 160
   Ser Arg His Leu Pro Gly Ser Ala Ser Ser Pro Ala Leu Leu Gln Pro
                   165                 170                 175
   Leu Ala Gly Ala Val Gly Gln Leu Trp Ser Gln His Gln Arg Pro Ser
               180                 185                 190
   Ala Thr Cys Ala Ser Trp Ser Gly Phe His Tyr Arg Thr Phe Asp Gly
           195                 200                 205
   Arg His Tyr His Phe Leu Gly Arg Cys Thr Tyr Leu Leu Ala Gly Ala
       210                 215                 220
   Ala Asp Ser Thr Trp Ala Val His Leu Thr Pro Gly Asp Arg Cys Pro
   225                 230                 235                 240
   Gln Pro Gly His Cys Gln Arg Val Gln Val Thr Met Gly Pro Glu Glu
                   245                 250                 255
   Val Leu Ile Gln Ala Gly Asn Val Ser Val Lys Gly Gln Leu Val Pro
               260                 265                 270
   Glu Gly Gln Ser Trp Leu Leu His Gly Leu Ser Leu Gln Trp Leu Gly
           275                 280                 285
   Asp Trp Leu Val Leu Ser Gly Gly Leu Gly Val Val Val Arg Leu Asp
       290                 295                 300
   Arg Thr Gly Ser Ile Ser Ile Ser Val Asp His Glu Leu Trp Gly Gln
   305                 310                 315                 320
   Thr Gln Gly Leu Cys Gly Leu Tyr Asn Gly Trp Pro Glu Asp Asp Phe
                   325                 330                 335
   Met Glu Pro Gly Gly Gly Leu Ala Met Leu Ala Ala Thr Phe Gly Asn
               340                 345                 350
   Ser Trp Arg Leu Pro Gly Ser Glu Val Ser Pro Ala Glu Tyr His Glu
           355                 360                 365
   Ala Cys Leu Phe Ala Tyr Cys Ala Gly Ala Met Ala Gly Ser Gly Gln
       370                 375                 380
   Glu Gly Arg Gln Gln Ala Val Cys Ala Thr Phe Ala Ser Tyr Val Gln
   385                 390                 395                 400
   Ala Cys Ala Arg Arg His Ile His Ile Arg Trp Arg Lys Pro Gly Phe
                   405                 410                 415
   Cys Glu Arg Leu Cys Pro Gly Gly Gln Leu Tyr Ser Asp Cys Val Ser
               420                 425                 430
   Leu Cys Pro Pro Ser Cys Glu Ala Val Gly Gln Gly Glu Glu Glu Ser
           435                 440                 445
   Cys Arg Glu Glu Cys Val Ser Gly Cys Glu Cys Pro Arg Gly Leu Phe
       450                 455                 460
   Trp Asn Gly Thr Leu Cys Val Pro Ala Ala His Cys Pro Cys Tyr Tyr
   465                 470                 475                 480
   Cys Arg Gln Arg Tyr Val Pro Gly Asp Thr Val Arg Gln Leu Cys Asn
                   485                 490                 495
   Pro Cys Val Cys Arg Asp Gly Arg Trp His Cys Ala Gln Ala Leu Cys
               500                 505                 510
   Pro Ala Glu Cys Ala Val Gly Gly Asp Gly His Tyr Leu Thr Phe Asp
           515                 520                 525
   Gly Arg Ser Tyr Ser Phe Trp Gly Gly Gln Gly Cys Arg Tyr Ser Leu
       530                 535                 540
   Val Gln Pro Tyr Pro Ser Thr Cys Pro Thr Pro Thr Ile Arg Pro Pro
   545                 550                 555                 560
   Val Pro Gly Ala Val Leu Val Asn Gly Gln Asp Val Gly Leu Pro Trp
                   565                 570                 575
   Ile Gly Ala Glu Gly Leu Ser Val Arg Arg Ala Ser Ser Ala Phe Leu
               580                 585                 590
   Leu Leu Arg Trp Pro Gly Ala Gln Val Leu Trp Gly Leu Ser Asp Pro
           595                 600                 605
   Val Ala Tyr Ile Thr Leu Asp Pro Arg His Ala His Gln Val Gln Gly
       610                 615                 620
   Leu Cys Gly Thr Phe Thr Gln Asn Gln Gln Asp Asp Phe Leu Thr Pro
   625                 630                 635                 640
   Ala Gly Asp Val Glu Thr Ser Ile Ala Ala Phe Ala Ser Lys Phe Gln
                   645                 650                 655
   Val Ala Gly Lys Gly Arg Cys Pro Ser Glu Asp Ser Ala Leu Leu Ser
               660                 665                 670
   Pro Cys Thr Thr His Ser Gln Arg His Ala Phe Ala Glu Ala Ala Cys
           675                 680                 685
   Ala Ile Leu His Ser Ser Val Phe Gln Glu Cys His Arg Leu Val Asp
       690                 695                 700
   Lys Glu Pro Phe Tyr Leu Arg Cys Leu Ala Ala Val Cys Gly Cys Asp
   705                 710                 715                 720
   Pro Gly Ser Asp Cys Leu Cys Pro Val Leu Ser Ala Tyr Ala Arg Arg
                   725                 730                 735
   Cys Ala Gln Glu Gly Ala Ser Pro Pro Trp Arg Asn Gln Thr Leu Cys
               740                 745                 750
   Pro Val Met Cys Pro Gly Gly Gln Glu Tyr Arg Glu Cys Ala Pro Ala
           755                 760                 765
   Cys Gly Gln His Cys Gly Lys Pro Glu Asp Cys Gly Glu Leu Gly Ser
       770                 775                 780
   Cys Val Ala Gly Cys Asn Cys Pro Leu Gly Leu Leu Trp Asp Pro Glu
   785                 790                 795                 800
   Gly Gln Cys Val Pro Pro Ser Leu Cys Pro Cys Gln Leu Gly Ala Arg
                   805                 810                 815
   Arg Tyr Ala Pro Gly Ser Ala Thr Met Lys Glu Cys Asn Arg Trp Glu
               820                 825                 830
   Leu Val Tyr Ala Pro Gly Ala Cys Leu Leu Thr Cys Asp Ser Pro Ser
           835                 840                 845
   Ala Asn His Ser Cys Pro Ala Gly Ser Thr Asp Gly Cys Val Cys Pro
       850                 855                 860
   Pro Gly Thr Val Leu Leu Asp Glu Arg Cys Val Pro Pro Asp Leu Cys
   865                 870                 875                 880
   Pro Cys Arg His Ser Gly Gln Trp Tyr Leu Pro Asn Ala Thr Ile Gln
                   885                 890                 895
   Glu Asp Cys Asn Val Cys Val Cys Arg Gly Arg Gln Trp His Cys Thr
               900                 905                 910
   Gly Gln Arg Arg Ser Gly Arg Cys Gln Ala Ser Gly Ala Pro His Tyr
           915                 920                 925
   Val Thr Phe Asp Gly Leu Ala Phe Thr Tyr Pro Gly Ala Cys Glu Tyr
       930                 935                 940
   Leu Leu Val Arg Glu Ala Ser Gly Leu Phe Thr Val Ser Ala Gln Asn
   945                 950                 955                 960
   Leu Pro Cys Gly Ala Ser Gly Leu Thr Cys Thr Lys Ala Leu Ala Val
                   965                 970                 975
   Arg Leu Glu Gly Thr Val Val His Met Leu Arg Gly Thr Arg Val Leu
               980                 985                 990
   Val Gln Leu Ser Pro Gln Phe Arg Gly Arg Val Ala Gly Leu Cys Gly
           995                 1000                1005
   Asp Phe Asp Gly Asp Ala Ser Asn Asp Leu Arg Ser Arg Gln Gly Val
       1010                1015                1020
   Leu Glu Pro Thr Ala Glu Leu Ala Ala His Ser Trp Arg Leu Ser Pro
   1025                1030                1035                1040
   Leu Cys Pro Glu Pro Gly Asp Leu Pro His Pro Cys Thr Met Asn Thr
                   1045                1050                1055
   His Arg Ala Gly Trp Ala Arg Ala Arg Cys Gly Ala Leu Leu Gln Pro
               1060                1065                1070
   Leu Phe Thr Leu Cys His Ala Glu Val Pro Pro Gln Gln His Tyr Glu
           1075                1080                1085
   Trp Cys Leu Tyr Asp Ala Cys Gly Cys Asp Ser Gly Gly Asp Cys Glu
       1090                1095                1100
   Cys Leu Cys Ser Ala Ile Ala Thr Tyr Ala Asp Glu Cys Ala Arg His
   1105                1110                1115                1120
   Gly His His Val Arg Trp Arg Ser Gln Glu Leu Cys Cys Leu His Gln
                   1125                1130                1135
   Thr Pro Cys Ala Leu His Gly Gly His Leu Gly Gln Pro Ala Trp Cys
               1140                1145                1150
   Gly Cys Ile Leu Leu Pro Leu Cys Leu Ser Asp Pro Arg Leu Ser Pro
           1155                1160                1165
   Leu His Pro Ala Leu Gln Cys Glu Gly Gly Gln Val Tyr Glu Ala Cys
       1170                1175                1180
   Gly Pro Thr Cys Pro Pro Thr Cys His Glu Gln His Pro Glu Pro Gly
   1185                1190                1195                1200
   Trp His Cys Gln Val Val Ala Cys Val Glu Gly Cys Phe Cys Pro Glu
                   1205                1210                1215
   Gly Thr Leu Leu His Gly Arg Cys Val Leu Ser Thr Cys Gln Glu Gly
               1220                1225                1230
   Gln Trp His Cys Gly Gly Asp Gly Gly His Cys Glu Glu Leu Val Pro
           1235                1240                1245
   Ala Cys Ala Glu Gly Glu Ala Leu Cys Gln Glu Asn Gly His Cys Val
       1250                1255                1260
   Pro His Gly Trp Leu Cys Asp Asn Gln Asp Asp Cys Gly Asp Gly Ser
   1265                1270                1275                1280
   Asp Glu Glu Gly Glu Cys Leu Cys Pro Cys Val Glu Ala Thr Gly Leu
                   1285                1290                1295
   Val Ser Pro Cys Thr Cys Cys Ala Ala Pro Gly Cys Gly Glu Gly Gln
               1300                1305                1310
   Met Thr Cys Ser Ser Gly His Cys Leu Pro Leu Ala Leu Leu Cys Asp
           1315                1320                1325
   Arg Gln Asp Asp Cys Gly Asp Gly Thr Asp Glu Pro Ser Tyr Pro Cys
       1330                1335                1340
   Pro Gln Gly Leu Leu Ala Cys Ala Asp Gly Arg Cys Leu Pro Pro Ala
   1345                1350                1355                1360
   Leu Leu Cys Asp Gly His Pro Asp Cys Leu Asp Ala Ala Asp Glu Glu
                   1365                1370                1375
   Ser Cys Leu Gly Gln Val Thr Cys Val Pro Gly Glu Val Ser Cys Val
               1380                1385                1390
   Asp Gly Thr Cys Leu Gly Ala Ile Gln Leu Cys Asp Gly Val Trp Asp
           1395                1400                1405
   Cys Pro Asp Gly Ala Asp Glu Gly Pro Gly His Cys Pro Leu Pro Ser
       1410                1415                1420
   Leu Pro Thr Pro Pro Ala Ser Thr Leu Pro Gly Pro Ser Pro Gly Ser
   1425                1430                1435                1440
   Leu Asp Thr Ala Ser Ser Pro Leu Ala Ser Ala Ser Pro Ala Pro Pro
                   1445                1450                1455
   Cys Gly Pro Phe Glu Phe Arg Cys Gly Ser Gly Glu Cys Thr Pro Arg
               1460                1465                1470
   Gly Trp Arg Cys Asp Gln Glu Glu Asp Cys Ala Asp Gly Ser Asp Glu
           1475                1480                1485
   Arg Gly Cys Gly Gly Pro Cys Ala Pro His His Ala Pro Cys Ala Arg
       1490                1495                1500
   Gly Pro His Cys Val Ser Pro Glu Gln Leu Cys Asp Gly Val Arg Gln
   1505                1510                1515                1520
   Cys Pro Asp Gly Ser Asp Glu Gly Pro Asp Ala Cys Val Glu Ala Pro
                   1525                1530                1535
   Ala Pro Pro Ala Met Arg Gly Pro Pro Gly Gln Ala Gly Gly Pro Thr
               1540                1545                1550
   Ser Ser Arg Ala Pro Ser Pro Pro Ser Pro Pro Glu Ala Gln Gly Glu
           1555                1560                1565
   Gly Arg Lys Gly Gln Glu Arg Ser Arg Thr His Leu Thr Val Pro Ala
       1570                1575                1580
   Gly Ser Thr Gln Leu Pro Leu Cys Pro Gly Leu Phe Pro Cys Gly Val
   1585                1590                1595                1600
   Ala Pro Gly Leu Cys Leu Thr Pro Glu Gln Leu Cys Asp Gly Ile Pro
                   1605                1610                1615
   Asp Cys Pro Gln Gly Glu Asp Glu Leu Asp Cys Gly Gly Leu Pro Ala
               1620                1625                1630
   Leu Gly Gly Pro Asn Arg Thr Gly Leu Pro Cys Pro Glu Tyr Thr Cys
           1635                1640                1645
   Pro Asn Gly Thr Cys Ile Gly Phe Gln Leu Val Cys Asp Gly Gln Pro
       1650                1655                1660
   Asp Cys Gly Arg Pro Gly Gln Val Gly Pro Ser Pro Glu Glu Gln Gly
   1665                1670                1675                1680
   Cys Gly Ala Trp Gly Pro Trp Ser Pro Trp Gly Pro Cys Ser Arg Thr
                   1685                1690                1695
   Cys Gly Pro Trp Gly Gln Gly Arg Ser Arg Arg Cys Ser Pro Leu Gly
               1700                1705                1710
   Leu Leu Val Leu Gln Asn Cys Pro Gly Pro Glu His Gln Ser Gln Ala
           1715                1720                1725
   Cys Phe Thr Ala Ala Cys Pro Val Asp Gly Glu Trp Ser Thr Trp Ser
       1730                1735                1740
   Pro Trp Ser Val Cys Ser Glu Pro Cys Arg Gly Thr Met Thr Arg Gln
   1745                1750                1755                1760
   Arg Gln Cys His Ser Pro Gln Asn Gly Gly Arg Thr Cys Ala Ala Leu
                   1765                1770                1775
   Pro Gly Gly Leu His Ser Thr Arg Gln Thr Lys Pro Cys Pro Gln Asp
               1780                1785                1790
   Gly Cys Pro Asn Ala Thr Cys Ser Gly Glu Leu Met Phe Gln Pro Cys
           1795                1800                1805
   Ala Pro Cys Pro Leu Thr Cys Asp Asp Ile Ser Gly Gln Val Thr Cys
       1810                1815                1820
   Pro Pro Asp Trp Pro Cys Gly Ser Pro Gly Cys Trp Cys Pro Glu Gly
   1825                1830                1835                1840
   Gln Val Leu Gly Ser Glu Gly Trp Cys Val Trp Pro Arg Gln Cys Pro
                   1845                1850                1855
   Cys Leu Val Asp Gly Ala Arg Tyr Trp Pro Gly Gln Arg Ile Lys Ala
               1860                1865                1870
   Asp Cys Gln Leu Cys Ile Cys Gln Asp Gly Arg Pro Arg Arg Cys Arg
           1875                1880                1885
   Leu Asn Pro Asp Cys Ala Gly Glu Ala Leu Pro Ser Gly Ser Leu Val
       1890                1895                1900
   Leu Ser Leu Asp Arg Pro Ala Ala His Pro Pro Pro Pro Ser Gly Ser
   1905                1910                1915                1920
   Asp Cys Trp Pro Ser Leu Ser Gly Leu Trp Leu Val Leu Leu Val Thr
                   1925                1930                1935
   Leu Gly Gln Val Pro Gly Pro Leu Trp Lys Pro Glu His Pro Val Val
               1940                1945                1950
   Leu Pro Glu Leu Gln Gln Pro Pro Pro Leu Arg Pro Arg Ser Pro Val
           1955                1960                1965
   Pro Trp His Pro Pro Gln Gly Thr Gln Thr Glu Pro Cys Glu Gly Cys
       1970                1975                1980
   Glu His Gln Gly Gln Val His Arg Val Gly Glu Arg Trp His Gly Gly
   1985                1990                1995                2000
   Pro Cys Arg Val Cys Gln Cys Leu His Asn Leu Thr Ala His Cys Ser
                   2005                2010                2015
   Pro Tyr Cys Pro Leu Gly Ser Cys Pro Gln Gly Trp Val Leu Val Glu
               2020                2025                2030
   Gly Thr Gly Glu Ser Cys Cys His Cys Ala Leu Pro Gly Glu Asn Gln
           2035                2040                2045
   Thr Val Gln Pro Met Ala Thr Pro Ala Ala Ala Pro Ala Pro Ser Pro
       2050                2055                2060
   Gln Ile Arg Phe Pro Leu Ala Thr Tyr Ile Leu Pro Pro Ser Gly Gly
   2065                2070                2075                2080
   Ser Cys Arg Pro Leu Ser Ser Pro Thr Pro Ala Cys Leu Ser Leu Leu
                   2085                2090                2095
   His Pro Asp Pro Cys Tyr Ser Pro Leu Gly Leu Ala Gly Leu Ala Glu
               2100                2105                2110
   Gly Ser Leu His Ala Ser Ser Gln Gln Leu Glu His Pro Thr Gln Ala
           2115                2120                2125
   Ala Leu Leu Gly Ala Pro Thr Gln Gly Pro Ser Pro Gln Gly Trp His
       2130                2135                2140
   Ala Gly Gly Asp Ala Tyr Ala Lys Trp His Thr Arg Pro His Tyr Leu
   2145                2150                2155                2160
   Gln Leu Asp Leu Leu Gln Pro Arg Asn Leu Thr Gly Ile Leu Val Pro
                   2165                2170                2175
   Glu Thr Gly Ser Ser Asn Ala Tyr Ala Ser Ser Phe Ser Leu Gln Phe
               2180                2185                2190
   Ser Ser Asn Gly Leu His Trp His Asp Tyr Arg Asp Leu Leu Pro Gly
           2195                2200                2205
   Ile Leu Pro Leu Pro Lys Val Ser Pro Ala Gln Gly Arg Trp Gly Gln
       2210                2215                2220
   Gln Pro Thr Met Pro Phe Cys Gly Phe His Ser Leu Cys Pro Gln Gly
   2225                2230                2235                2240
   Pro Ser Ser Val Pro Glu Gly His Gly Leu His Ser Met Leu Val Glu
                   2245                2250                2255
   Tyr Leu Leu Phe Pro Arg Asn Trp Asp Asp Leu Asp Pro Ala Val Trp
               2260                2265                2270
   Thr Phe Gly Arg Met Val Gln Ala Arg Phe Val Arg Val Trp Pro His
           2275                2280                2285
   Asp Val His His Ser Asp Val Pro Leu Gln Val Glu Leu Leu Gly Cys
       2290                2295                2300
   Glu Pro Gly Val Gly Leu Arg Cys Ala Ser Gly Glu Cys Val Leu Arg
   2305                2310                2315                2320
   Gly Gly Pro Cys Asp Gly Val Leu Asp Cys Glu Asp Gly Ser Asp Glu
                   2325                2330                2335
   Glu Gly Cys Val Leu Leu Pro Glu Gly Thr Gly Arg Tyr Thr Val Ala
               2340                2345                2350
   Gly Arg Ala Ala His Ala Leu Gly Leu Ala Phe Glu Gly Thr Ala Met
           2355                2360                2365
   Trp Glu Gly Pro Gly Thr Ala Phe Thr Pro Lys Val Pro Arg Pro Cys
       2370                2375                2380
   Met Leu Arg Ser Cys Ser Arg Gly Leu Ala Glu Thr Glu His Trp Pro
   2385                2390                2395                2400
   Pro Gly Gln Glu Ser Pro Thr Ser Pro Thr Glu Ala Trp Asp Thr Leu
                   2405                2410                2415
   Ser Arg Ala Pro Thr Phe Leu Ser Trp Glu Gly Glu Leu Gly Lys Pro
               2420                2425                2430
   His Leu Pro Leu Pro Thr Pro Thr Glu Thr Arg Pro Val Ser Pro Gly
           2435                2440                2445
   Pro Ala Ser Gly Val Pro His His Gly Glu Ser Val Gln Met Val Thr
       2450                2455                2460
   Thr Thr Pro Ile Pro Gln Met Glu Ala Arg Thr Leu Pro Pro Gly Met
   2465                2470                2475                2480
   Ala Ala Val Thr Val Val Pro Pro His Pro Val Thr Pro Ala Thr Pro
                   2485                2490                2495
   Ala Gly Lys Met Pro Ser Pro Ser Thr Cys Val Gln Gly Leu Cys Gln
               2500                2505                2510
   Ser Val Ala Pro Gly Pro Phe Pro Pro Val Gln Cys Gly Pro Gly Gln
           2515                2520                2525
   Thr Pro Cys Glu Val Leu Gly Cys Val Glu Gln Ala Gln Val Cys Asp
       2530                2535                2540
   Gly Arg Glu Asp Cys Leu Asp Gly Ser Asp Glu Arg His Cys Gly Glu
   2545                2550                2555                2560
   Leu Leu Glu Gly Leu Leu Ser Cys Gly Ala Leu Cys Ser Pro Ser Gln
                   2565                2570                2575
   Leu Ser Cys Gly Ser Gly Glu Cys Leu Ser Ala Glu Arg Arg Cys Asp
               2580                2585                2590
   Leu Arg Pro Asp Cys Gln Asp Gly Ser Asp Glu Asp Gly Cys Val Asp
           2595                2600                2605
   Cys Val Leu Ala Pro Trp Ser Val Trp Ser Ser Cys Ser Arg Ser Cys
       2610                2615                2620
   Gly Leu Gly Leu Thr Phe Gln Arg Gln Glu Leu Leu Arg Pro Pro Leu
   2625                2630                2635                2640
   Pro Gly Gly Ser Cys Pro Arg Asp Arg Phe Arg Ser Gln Ser Cys Phe
                   2645                2650                2655
   Val Gln Ala Cys Pro Val Ala Gly Ala Trp Ala Met Trp Glu Ala Trp
               2660                2665                2670
   Gly Pro Cys Ser Val Ser Cys Gly Gly Gly His Gln Ser Arg Gln Arg
           2675                2680                2685
   Ser Cys Val Asp Pro Pro Pro Lys Asn Gly Gly Ala Pro Cys Pro Gly
       2690                2695                2700
   Ala Ser Gln Glu Arg Ala Pro Cys Gly Leu Gln Pro Cys Ser Gly Gly
   2705                2710                2715                2720
   Thr Gly Lys Gly Val Leu Gly Trp Gly His Gly Gly Ser Thr Val Gly
                   2725                2730                2735
   Thr Gly Arg Leu Gly Leu Pro Ala Pro Arg Leu Thr Trp Cys Pro Ser
               2740                2745                2750
   Pro Thr Arg Leu Arg Ala Gly Pro Cys Val Cys Glu Cys Arg Ser Val
           2755                2760                2765
   Pro Glu Gly Ala Gly Ala Pro Met Pro Thr Leu Leu Pro Gly Ser Gln
       2770                2775                2780
   Gly Gln Gln Lys Leu Gln Trp Ala Leu Cys Gly Ser Leu Pro Ser Leu
   2785                2790                2795                2800
   Leu Cys Pro Leu Gly Leu Ser Ala Leu Phe His Ile Leu Pro Gly Cys
                   2805                2810                2815
   Arg Cys Pro Pro Gly Leu Leu Leu His Asp Thr Arg Cys Leu Pro Leu
               2820                2825                2830
   Ser Glu Cys Pro Cys Leu Val Gly Glu Glu Leu Lys Trp Pro Gly Val
           2835                2840                2845
   Ser Phe Leu Leu Gly Asn Cys Ser Gln Cys Val Cys Glu Lys Gly Glu
       2850                    2855                    2860
   Leu Leu Cys Gln Pro Gly Gly Cys Pro Leu Pro Cys Gly Trp Ser Ala
   2865                2870                    2875                    2880
   Trp Ser Ser Trp Ala Pro Cys Asp Arg Ser Cys Gly Ser Gly Val Arg
                   2885                    2890                    2895
   Ala Arg Phe Arg Ser Pro Ser Asn Pro Pro Ala Ala Trp Gly Gly Ala
               2900                    2905                    2910
   Pro Cys Glu Gly Asp Arg Gln Glu Leu Gln Gly Cys His Thr Val Cys
           2915                    2920                    2925
   Gly Thr Gly Ile Ala Gly Ser Leu Gly Ala Gly Val Pro Pro Ser Ser
       2930                    2935                    2940
   Ser Gln Phe Cys Thr Leu Arg Thr His Gly Met Gly Pro Thr Asp His
   2945                2950                    2955                    2960
   Ser Thr Trp Gly Ile Glu Val Phe Gly Trp Thr Pro Trp Thr Ser Trp
                   2965                    2970                    2975
   Ser Ser Cys Ser Gln Ser Cys Leu Ala Pro Gly Gly Gly Pro Gly Trp
               2980                    2985                    2990
   Arg Ser Arg Ser Arg Leu Cys Pro Ser Pro Gly Asp Ser Ser Cys Pro
           2995                    3000                    3005
   Gly Asp Ala Thr Gln Glu Glu Pro Cys Ser Pro Pro Ile Glu Cys Thr
       3010                    3015                    3020
   Gly Phe Cys Ala Pro Gly Cys Thr Cys Pro Pro Gly Leu Phe Leu His
   3025                3030                    3035                    3040
   Asn Ala Ser Cys Leu Pro Arg Ser Gln Cys Pro Cys Gln Leu His Gly
                   3045                    3050                    3055
   Gln Leu Tyr Ala Ser Gly Ala Met Ala Arg Leu Asp Ser Cys Asn Asn
               3060                    3065                    3070
   Cys Thr Cys Val Ser Gly Lys Met Ala Cys Thr Ser Glu Arg Cys Pro
           3075                    3080                    3085
   Val Ala Cys Gly Trp Ser Pro Trp Thr Leu Trp Ser Leu Cys Ser Cys
       3090                    3095                    3100
   Ser Cys Asn Val Gly Ile Arg Arg Arg Phe Arg Ala Gly Thr Ala Pro
   3105                3110                    3115                    3120
   Pro Ala Ala Phe Gly Gly Ala Glu Cys Gln Gly Pro Thr Met Glu Ala
                   3125                    3130                    3135
   Glu Phe Cys Ser Leu Arg Pro Cys Pro Gly Pro Val Pro Gly Met Cys
               3140                    3145                    3150
   Pro Arg Asp Lys Gln Trp Leu Asp Cys Ala Gln Gly Pro Ala Ser Cys
           3155                    3160                    3165
   Ala Glu Leu Ser Ala Pro Arg Gly Thr Asn Gln Thr Cys His Pro Gly
       3170                    3175                    3180
   Cys His Cys Pro Ser Gly Met Leu Leu Leu Val Ser Pro Arg Gly His
   3185                3190                    3195                    3200
   Pro Gly Pro Leu Gly Ala Ser Val Gln Pro Pro Val Ala Leu Pro Gly
                   3205                    3210                    3215
   Ala Ile Gly Thr Gly Ser Val Pro Gly Ala Gly Gly Trp Gly Pro Trp
               3220                3225                3230
   Gly Pro Trp Ser His Cys Ser Arg Ser Cys Gly Gly Gly Leu Arg Ser
           3235                3240                3245
   Arg Thr Arg Ala Cys Asp Gln Pro Pro Pro Gln Gly Leu Gly Asp Tyr
       3250                3255                3260
   Cys Glu Gly Pro Arg Ala Gln Gly Glu Val Cys Gln Ala Leu Pro Cys
   3265                3270                3275                3280
   Pro Val Thr Asn Cys Thr Ala Ile Glu Gly Ala Glu Tyr Ser Pro Cys
                   3285                3290                3295
   Gly Pro Pro Cys Pro Arg Ser Cys Asp Asp Leu Val His Cys Val Trp
               3300                3305                3310
   Arg Cys Gln Pro Gly Cys Tyr Cys Pro Pro Gly Gln Val Leu Ser Ser
           3315                3320                3325
   Asn Gly Ala Ile Cys Val Gln Pro Gly His Cys Ser Cys Leu Asp Leu
       3330                3335                3340
   Leu Thr Gly Gln Arg His His Pro Gly Ala Arg Leu Ala Arg Pro Asp
   3345                3350                3355                3360
   Gly Cys Asn His Cys Thr Cys Leu Glu Gly Arg Leu Asn Cys Thr Asp
                   3365                3370                3375
   Leu Pro Cys Pro Asp Cys Gly Gly Gly Gln Ser Leu His Pro Cys Gly
               3380                3385                3390
   Gln Pro Cys Pro Arg Ser Cys Gln Asp Leu Ser Pro Gly Ser Val Cys
           3395                3400                3405
   Gln Pro Gly Ser Val Gly Cys Gln Pro Thr Cys Gly Cys Pro Leu Gly
       3410                3415                3420
   Gln Leu Ser Gln Asp Gly Leu Cys Val Pro Pro Ala His Cys Arg Cys
   3425                3430                3435                3440
   Gln Tyr Gln Pro Gly Ala Met Ala Pro Ser Phe Val Pro Ser Thr Cys
                   3445                3450                3455
   Val Ala Gly Ile Leu Gln Cys Gln Glu Val Pro Asp Cys Pro Asp Pro
               3460                3465                3470
   Gly Val Trp Ser Ser Trp Gly Pro Trp Glu Asp Cys Ser Val Ser Cys
           3475                3480                3485
   Gly Gly Gly Glu Gln Leu Arg Ser Arg Arg Cys Ala Arg Pro Pro Cys
       3490                3495                3500
   Pro Gly Pro Ala Arg Gln Ser Arg Thr Cys Ser Thr Gln Val Cys Arg
   3505                3510                3515                3520
   Glu Ala Gly Cys Pro Ala Gly Arg Leu Tyr Arg Glu Cys Gln Pro Gly
                   3525                3530                3535
   Glu Gly Cys Pro Phe Ser Cys Ala His Val Thr Gln Gln Val Gly Cys
               3540                3545                3550
   Phe Ser Glu Gly Cys Glu Glu Gly Cys His Cys Pro Glu Gly Thr Phe
           3555                3560                3565
   Gln His Arg Leu Ala Cys Val Gln Glu Cys Pro Cys Val Leu Thr Ala
       3570                3575                3580
   Trp Leu Leu Gln Glu Leu Gly Ala Thr Ile Gly Asp Pro Gly Gln Pro
   3585                3590                3595                3600
   Leu Gly Pro Gly Asp Glu Leu Asp Ser Gly Gln Thr Leu Arg Thr Ser
                   3605                3610                3615
   Cys Gly Asn Cys Ser Cys Ala His Gly Lys Leu Ser Cys Ser Leu Asp
               3620                3625                3630
   Asp Cys Phe Glu Ala Asp Gly Gly Phe Gly Pro Trp Ser Pro Trp Gly
           3635                3640                3645
   Pro Cys Ser Arg Ser Cys Gly Gly Leu Gly Thr Arg Thr Arg Ser Arg
       3650                3655                3660
   Gln Cys Val Leu Thr Met Pro Thr Leu Ser Glu Leu Pro Val Cys Pro
   3665                3670                3675                3680
   Gly Pro Gly Cys Gly Ala Gly Asn Cys Ser Trp Thr Ser Trp Ala Pro
                   3685                3690                3695
   Trp Glu Pro Cys Ser Arg Ser Cys Gly Val Gly Gln Gln Arg Arg Leu
               3700                3705                3710
   Arg Ala Tyr Arg Pro Pro Gly Pro Gly Gly His Trp Cys Pro Asn Ile
           3715                3720                3725
   Leu Thr Ala Tyr Gln Glu Arg Arg Phe Cys Asn Leu Arg Ala Cys Pro
       3730                3735                3740
   Glu Ala Gly Cys Pro Ala Gly Met Glu Val Val Thr Cys Ala Asn Arg
   3745                3750                3755                3760
   Cys Pro Arg Arg Cys Ser Asp Leu Gln Glu Gly Ile Val Cys Gln Asp
                   3765                3770                3775
   Asp Gln Val Cys Gln Lys Gly Cys Arg Cys Pro Lys Gly Ser Leu Glu
               3780                3785                3790
   Gln Asp Gly Gly Cys Val Pro Ile Gly His Cys Asp Cys Thr Asp Ala
           3795                3800                3805
   Gln Gly His Ser Trp Ala Pro Gly Ser Gln His Gln Asp Ala Cys Asn
       3810                3815                3820
   Asn Cys Ser Cys Gln Ala Gly Gln Leu Ser Cys Thr Ala Gln Pro Cys
   3825                3830                3835                3840
   Pro Pro Pro Thr His Cys Ala Trp Ser His Trp Ser Ala Trp Ser Pro
                   3845                3850                3855
   Cys Ser His Ser Cys Gly Pro Arg Gly Gln Gln Ser Arg Phe Arg Cys
               3860                3865                3870
   Gly Pro Gly Leu Ala Ser Arg Ser Gly Ser Cys Pro Cys Leu Met Ala
           3875                3880                3885
   Lys Ala Asp Pro Thr Cys Asn Ser Thr Phe Leu His Leu Asp Thr Gln
       3890                3895                3900
   Gly Cys Tyr Ser Gly Pro Cys Pro Asp Ser Cys Gln Trp Ser Leu Trp
   3905                3910                3915                3920
   Gly Pro Trp Ser Pro Cys Gln Val Pro Cys Ser Gly Gly Phe Arg Leu
                   3925                3930                3935
   Arg Trp Arg Glu Ala Glu Ala Leu Cys Gly Gly Gly Cys Arg Glu Pro
               3940                3945                3950
   Trp Ala Gln Asp Arg Lys Leu Gln Arg Arg Ala Leu Pro Ser Thr Cys
   3955 3960 3965
   Val Asn Glu Ser Leu Val Cys Pro His Gln Glu Cys Pro Val Leu Gly
   3970 3975 3980
   Pro Trp Ser Ala Trp Ser Ser Cys Ser Ala Pro Cys Gly Gly Gly Thr
   3985 3990 3995 4000
   Met Glu Arg His Arg Thr Cys Glu Gly Gly Pro Gly Val Ala Pro Cys
   4005 4010 4015
   Gln Ala Gln Asp Thr Glu Gln Arg Gln Glu Cys Asn Leu Gln Pro Cys
   4020 4025 4030
   Pro Glu Cys Pro Pro Gly Gln Val Leu Ser Ala Cys Ala Thr Ser Cys
   4035 4040 4045
   Pro Cys Leu Cys Trp His Leu Gln Pro Gly Ala Ile Cys Val Gln Glu
   4050 4055 4060
   Pro Cys Gln Pro Gly Cys Gly Cys Pro Gly Gly Gln His Ser Leu Pro
   4065 4070 4075 4080
   Trp Gly Leu Thr Leu Thr Leu Glu Glu Gln Ala Gln Glu Leu Pro Pro
   4085 4090 4095
   Gly Thr Val Leu Thr Arg Asn Cys Thr Arg Cys Val Cys His Gly Gly
   4100 4105 4110
   Ala Phe Ser Cys Ser Leu Val Asp Cys Gln Gly Glu Ile Val Pro Pro
   4115 4120 4125
   Gly Glu Thr Trp Gln Gln Val Ala Pro Gly Glu Leu Gly Leu Cys Glu
   4130 4135 4140
   Gln Thr Cys Leu Glu Met Asn Ala Thr Lys Thr Gln Ser Asn Cys Ser
   4145 4150 4155 4160
   Ser Ala Arg Ala Ser Gly Cys Val Cys Gln Pro Gly His Phe Arg Ser
   4165 4170 4175
   Gln Ala Gly Pro Cys Val Pro Glu Asp His Cys Glu Cys Trp His Leu
   4180 4185 4190
   Gly Arg Pro His Leu Val Arg His Arg Thr Pro Ser Ala Thr Thr His
   4195 4200 4205
   Pro Phe Leu Thr Pro Ser Leu Pro Ile Cys Leu
   4210 4215

<210> 3
<211> 893
<212> DNA
<213> Homo sapiens

<400> 3
gtaccttaaa gacaacaaac aagcaaacac aacttataat taaaaaacat gcaaagggct 60
caccttccac ttccttctgg tcctgctcct cttcttcctc ctctcctgcc tcctcttctc 120
cctgttccat cagaccttct ggggcccctt tcaataagca gctgctggcc ggccagccct 180
tgggggcagg gctggaaccg gggcagggga ggctgcgggg ccactcgctg gagaggcaaa 240
caggaaggac tgccccctga gcgccaggct tcgggcccgg gaatcgccgc cgccgccgcc 300
gcagagctgc agctcggggc cgagggtaag gaggcgagcc gggagcggga ggcccgggag 360
agctccgcgg gtccccgcgc ccagtcccca gccgcgcccc gaccccgccg ccccgggcct 420
aacgcggccg gcgaggccta cgcggcggcc gccgtcaccg tgctggagcc gccggcctcc 480
gaccccgagc tgcagcccgc cgagcgcccg ctgccatcgc cggggtccgg ggagggcgcc 540
ccggtcttcc tcacggggcc tcgatcccag tgggtgctgc gggggcggga ggtggtgctg 600
acgtgccggg cgggggcct ccccgagccc acactgtact gggagaagga cgggatggcc 660

ctggacgaag tgtgggacag cagccacttc gcgctccagc cgggccgcgc cgaggacggc 720
cccggcgcga gcctggcact gcgcatcctg gcggctcggc tgccggattc cggcgtctac 780
gtgtgccacg cccgcaacgc gcacggccac gcgcaggcgg gggcgctgct ccaggtgctg 840
acccccacct tcctgccgcc aagacagccc taaccaaggc ccagaaaggg tag 893

<210> 4
<211> 274
<212> PRT
<213> Homo sapiens

<400> 4
Met Gln Arg Ala His Leu Pro Leu Pro Ser Gly Pro Ala Pro Leu Leu
1               5                   10                  15

Pro Pro Leu Leu Pro Pro Leu Leu Pro Val Pro Ser Asp Leu Leu Gly
            20                  25                  30

Pro Leu Ser Ile Ser Ser Cys Trp Pro Ala Ser Pro Trp Gly Gln Gly
        35                  40                  45

Trp Asn Arg Gly Arg Gly Gly Cys Gly Ala Thr Arg Trp Arg Gly Lys
    50                  55                  60

Gln Glu Gly Leu Pro Pro Glu Arg Gln Ala Ser Gly Pro Gly Ile Ala
65                  70                  75                  80

Ala Ala Ala Ala Ala Glu Leu Gln Leu Gly Ala Glu Gly Lys Glu Ala
                85                  90                  95

Ser Arg Glu Arg Glu Ala Arg Glu Ser Ser Ala Gly Pro Arg Ala Gln
            100                 105                 110

Ser Pro Ala Ala Pro Arg Pro Arg Arg Pro Gly Pro Asn Ala Ala Gly
        115                 120                 125

Glu Ala Tyr Ala Ala Ala Ala Val Thr Val Leu Glu Pro Pro Ala Ser
    130                 135                 140

Asp Pro Glu Leu Gln Pro Ala Glu Arg Pro Leu Pro Ser Pro Gly Ser
145                 150                 155                 160

Gly Glu Gly Ala Pro Val Phe Leu Thr Gly Pro Arg Ser Gln Trp Val
                165                 170                 175

Leu Arg Gly Ala Glu Val Val Leu Thr Cys Arg Ala Gly Gly Leu Pro
            180                 185                 190

Glu Pro Thr Leu Tyr Trp Glu Lys Asp Gly Met Ala Leu Asp Glu Val
        195                 200                 205

Trp Asp Ser Ser His Phe Ala Leu Gln Pro Gly Arg Ala Glu Asp Gly
    210                 215                 220

Pro Gly Ala Ser Leu Ala Leu Arg Ile Leu Ala Ala Arg Leu Pro Asp
225                 230                 235                 240

Ser Gly Val Tyr Val Cys His Ala Arg Asn Ala His Gly His Ala Gln
                245                 250                 255

Ala Gly Ala Leu Leu Gln Val Leu Thr Pro Thr Phe Leu Pro Pro Arg
            260                 265                 270

Gln Pro

<210> 5
<211> 1703

<212> DNA
<213> Homo sapiens

<400> 5
ccaggttggg gtctcccaaa agcagcccct atgttgtggg gaagtggaga gtagtacagc 60
taagccagac cccattgtgc ccgcaggtta gagcctggca atgccgtttg ggtgtgtgac 120
tctgggcgac aagaagaact ataaccagcc atcggaggtg actgacagat atgatttggg 180
acaggtcatc aagacggagg agttttgtga aatcttccgg gccaaggaca agacgacagg 240
caagctgcac acctgcaaga agttccagaa gcgggacggc cgcaaggtgc ggaaagctgc 300
caagaacgag ataggcatcc tcaagatggt gaagcatccc aacatcctac agctggtgga 360
tgtgtttgtg acccgcaagg agtactttat cttcctggag ctggccacgg ggagggaggt 420
gtttgactgg atcctggacc agggctacta ctcggagcga gacacaagca acgtggtacg 480
gcaagtcctg gaggccgtgg cctatttgca ctcactcaag atcgtgcaca ggaatctcaa 540
gctggagaac ctggtttact acaaccggct gaagaactcg aagattgtca tcagtgactt 600
ccatctggct aagctagaaa atggcctcat caaggagccc tgtgggaccc ccgagtatct 660
ggccccagag gtggtaggcc ggcagcggta tggacgccct gtggactgct gggccattgg 720
agtcatcatg tacatcctgc tttcaggcaa cccacctttc tatgaggagg tggaagaaga 780
tgattatgag aaccatgata agaatctctt ccgcaagatc ctggctggtg actatgagtt 840
tgactctcca tattgggatg atatttcgca ggcagccaaa gacctggtca caaggctgat 900
ggaggtggag caagaccagc ggatcactgc agaagaggcc atctcccatg agtggatttc 960
tggcaatgct gcttctgata agaacatcaa ggatggtgtc tgtgcccaga ttgaaaagaa 1020
ctttgccagg gccaagtgga agaaggctgt ccgagtgacc accctcatga aacggctccg 1080
ggcaccagag cagtccagca cggctgcagc ccagtcggcc tcagccacag acactgccac 1140
ccccggggct gcaggtgggg ccacagctgc agctgcgagt ggagctacct cagcccctga 1200
gggtgatgct gctcgtgctg caaagagtga taatgtggcc cccgcagacc gtagtgccac 1260
cccagccaca gatggaagtg ccaccccagc cactgatggc agtgtcaccc cagccaccga 1320
tggaagcatc actccagcca ctgatgggag tgtcacccca gccactgaca ggagcgctac 1380
tccagccact gatgggagag ccacaccagc cacagaagag agcactgtgc ccaccaccca 1440
aagcagtgcc atgctggcca ccaaggcagc tgccacccct gagccggcta tggcccagcc 1500
ggacagcaca gccccagagg gcgccacagg ccaggctcca ccctctagta aaggggaaga 1560
ggctgctggt tatgcccagg agtctcaaag ggaggaggcc agctgagtag gcagcctggt 1620
gagggggggc aggggatggg caggagggtg ggagagtgga tgaggggctt ctcactgtac 1680
atagagtcac tggcatgatg ccc 1703

<210> 6
<211> 501
<212> PRT
<213> Homo sapiens

<400> 6
Met Pro Phe Gly Cys Val Thr Leu Gly Asp Lys Lys Asn Tyr Asn Gln
1               5                   10                  15

Pro Ser Glu Val Thr Asp Arg Tyr Asp Leu Gly Gln Val Ile Lys Thr
            20                  25                  30

Glu Glu Phe Cys Glu Ile Phe Arg Ala Lys Asp Lys Thr Thr Gly Lys
        35                  40                  45

Leu His Thr Cys Lys Lys Phe Gln Lys Arg Asp Gly Arg Lys Val Arg
    50                  55                  60

Lys Ala Ala Lys Asn Glu Ile Gly Ile Leu Lys Met Val Lys His Pro
65                  70                  75                  80

Asn Ile Leu Gln Leu Val Asp Val Phe Val Thr Arg Lys Glu Tyr Phe
                85                  90                  95

Ile Phe Leu Glu Leu Ala Thr Gly Arg Glu Val Phe Asp Trp Ile Leu
            100                 105                 110

Asp Gln Gly Tyr Tyr Ser Glu Arg Asp Thr Ser Asn Val Val Arg Gln
        115                 120                 125

Val Leu Glu Ala Val Ala Tyr Leu His Ser Leu Lys Ile Val His Arg
    130                 135                 140

Asn Leu Lys Leu Glu Asn Leu Val Tyr Tyr Asn Arg Leu Lys Asn Ser

145                 150                 155                 160

Lys Ile Val Ile Ser Asp Phe His Leu Ala Lys Leu Glu Asn Gly Leu
                165                 170                 175

Ile Lys Glu Pro Cys Gly Thr Pro Glu Tyr Leu Ala Pro Glu Val Val
            180                 185                 190

Gly Arg Gln Arg Tyr Gly Arg Pro Val Asp Cys Trp Ala Ile Gly Val
        195                 200                 205

Ile Met Tyr Ile Leu Leu Ser Gly Asn Pro Pro Phe Tyr Glu Glu Val
    210                 215                 220

Glu Glu Asp Asp Tyr Glu Asn His Asp Lys Asn Leu Phe Arg Lys Ile
225                 230                 235                 240

Leu Ala Gly Asp Tyr Glu Phe Asp Ser Pro Tyr Trp Asp Asp Ile Ser
                245                 250                 255

Gln Ala Ala Lys Asp Leu Val Thr Arg Leu Met Glu Val Glu Gln Asp
            260                 265                 270

Gln Arg Ile Thr Ala Glu Glu Ala Ile Ser His Glu Trp Ile Ser Gly
        275                 280                 285

Asn Ala Ala Ser Asp Lys Asn Ile Lys Asp Gly Val Cys Ala Gln Ile
    290                 295                 300

Glu Lys Asn Phe Ala Arg Ala Lys Trp Lys Lys Ala Val Arg Val Thr
305                 310                 315                 320

Thr Leu Met Lys Arg Leu Arg Ala Pro Glu Gln Ser Ser Thr Ala Ala
                325                 330                 335

Ala Gln Ser Ala Ser Ala Thr Asp Thr Ala Thr Pro Gly Ala Ala Gly
            340                 345                 350

Gly Ala Thr Ala Ala Ala Ala Ser Gly Ala Thr Ser Ala Pro Glu Gly
        355                 360                 365

Asp Ala Ala Arg Ala Ala Lys Ser Asp Asn Val Ala Pro Ala Asp Arg
    370                 375                 380

Ser Ala Thr Pro Ala Thr Asp Gly Ser Ala Thr Pro Ala Thr Asp Gly
385                 390                 395                 400

Ser Val Thr Pro Ala Thr Asp Gly Ser Ile Thr Pro Ala Thr Asp Gly
                405                 410                 415

Ser Val Thr Pro Ala Thr Asp Arg Ser Ala Thr Pro Ala Thr Asp Gly
            420                 425                 430

Arg Ala Thr Pro Ala Thr Glu Glu Ser Thr Val Pro Thr Thr Gln Ser
        435                 440                 445

Ser Ala Met Leu Ala Thr Lys Ala Ala Ala Thr Pro Glu Pro Ala Met
    450                 455                 460

Ala Gln Pro Asp Ser Thr Ala Pro Glu Gly Ala Thr Gly Gln Ala Pro
465                 470                 475                 480

Pro Ser Ser Lys Gly Glu Glu Ala Ala Gly Tyr Ala Gln Glu Ser Gln
                485                 490                 495

Arg Glu Glu Ala Ser
            500

<210> 7
<211> 5877
<212> DNA
<213> Homo sapiens

<400> 7
agtccccagc cccgtcgccg gcggaggcgg gcgcgggcgc gttcctgtgg ccagtcaccc 60
ggaggagttg gtcgcacaat tatgaaagac tcggcttctg ctgctagcgc cggagctgag 120
ttagtcctga gaaggtttcc ctgggcgttc cttgtccggc ctctgctgcc gcctccggag 180
acgcttcccg atagatggct acaggccgcg gaggaggagg aggtggagtt gctgcccttc 240
cggagtccgc cccgtgagga gaatgtccca gaaatcctgg atagaaagca ctttgaccaa 300
gagggaatgt gtatatatta taccaagttc caaggaccct cacaggtgcc ttccaggatg 360
tcaaatttgt cagcaactcg tcaggtgttt ttgtggtcgc ttggtcaagc aacatgcttg 420
ttttactgca agtcttgcca tgaaatactc agatgtgaaa ttgggtgacc attttaatca 480
ggcaatagaa gaatggtctg tggaaaagca tacagaacag agcccaacgg atgcttatgg 540
agtcataaat tttcaagggg gttctcattc ctacagagct aagtatgtga ggctatcata 600
tgacaccaaa cctgaagtca ttctgcaact tctgcttaaa gaatggcaaa tggagttacc 660
caaacttgtt atctctgtac atgggggcat gcagaaattt gagcttcacc cacgaatcaa 720
gcagttgctt ggaaaaggtc ttattaaagc tgcagttaca actggagcct ggattttaac 780
tggaggagta aacacaggtg tggcaaaaca tgttggagat gccctcaaag aacatgcttc 840
cagatcatct cgaaagattt gcactatcgg aatagctcca tggggagtga ttgaaaacag 900
aaatgatctt gttgggagag atgtagttgc tccttatcaa accttattga accccctgag 960
caaattgaat gttttgaata atctgcattc ccatttcata ttggtggatg atggcactgt 1020
tggaaagtat ggggcggaag tcagactgag aagagaactt gaaaaaacta ttaatcagca 1080
aagaattcat gctattggcc agggtgtccc tgtggtggca cttatatttg agggtgggcc 1140
aaatgttatc ctcacagttc ttgaatacct tcaggaaagc ccccctgttc cagtagttgt 1200
gtgtgaagga acaggcagag ctgcagatct gctagcgtat attcataaac aaacagaaga 1260
aggagggaat cttcctgatg cagcagagcc cgatattatt tccactatca aaaaaacatt 1320
taactttggc cagaatgaag cacttcattt atttcaaaca ctgatggagt gcatgaaaag 1380
aaaggagctt atcactgttt tccatattgg gtcagatgaa catcaagata tagatgtagc 1440
aatacttact gcactgctaa aaggtactaa tgcatctgca tttgaccagc ttatccttac 1500
attggcatgg gatagagttg acattgccaa aaatcatgta tttgtttatg gacagcagtg 1560
gctggtagga tccttggaac aagctatgct tgatgctctt gtaatggata gagttgcatt 1620
tgtaaaactt cttattgaaa atggagtaag catgcataaa ttccttacca ttccgagact 1680
ggaagaactt tacaacacta aacaaggtcc aactaatcca atgctgtttc atcttgttcg 1740
agacgtcaaa cagggaaatc ttcctccagg atataagatc actctgattg atataggact 1800
tgttattgaa tatctcatgg gaggaaccta cagatgcacc tatactagga aacgttttcg 1860
attaatatat aatagtcttg gtggaaataa tcggaggtct ggccgaaata cctccagcag 1920
cactcctcag ttgcgaaaga gtcatgaatc ttttggcaat agggcagata aaaaggaaaa 1980
aatgaggcat aaccatttca ttaagacagc acagccctac cgaccaaagg tagatacagt 2040
tatggaagaa ggaaagaaga aaagaaccaa agatgaaatt gtagacattg atgatccaga 2100
aaccaagcgc tttccttatc cacttaatga acttttaatt tgggcttgcc ttatgaagag 2160
gcaggtcatg gcccgttttt tatggcaaca tggtgaagaa tcaatggcta aagcattagt 2220
tgcctgtaag atctatcgtt caatggcata tgaagcaaag cagagtgacc tggtagatga 2280
tacttcagaa gaactaaaac agtattccag tgattttggt cagttggccg ttgaattatt 2340
agaacagtcc ttcagacaag atgaaaccat ggctatgaaa ttgctcactt atgaactgaa 2400
gaactggagt aattcaacct gccttaagtt agcagtttct tcaagactta gaccttttgt 2460
agctcacacc tgtacacaaa tgttgttatc tgatatgtgg atgggaaggc tgaatatgag 2520
gaaaaattcc tggtacaagg taatactaag cattttagtt ccacctgcca tattgctgtt 2580
agagtataaa actaaggctg aaatgtccca tatcccacaa tctcaagatg ctcatcagat 2640
gacaatggat gacagcgaaa acaactttca gaacataaca gaagagatcc ccatggaagt 2700
gtttaaagaa gtacggattt tggatagtaa tgaaggaaag aatgagatgg agatacaaat 2760
gaaatcaaaa aagcttccaa ttacgcgaaa gttttatgcc ttttatcatg caccaattgt 2820
aaaattctgg tttaacacgt tggcatattt aggatttctg atgctttata catttgtggt 2880
tcttgtacaa atggaacagt taccttcagt tcaagaatgg attgttattg cttatatttt 2940
tacttatgcc attgagaaag tccgtgaggt atttatgtct gaagctggga aagtaaacca 3000
gaagattaaa gtatggttta gtgattactt caacatcagt gatacaattg ccataatttc 3060
tttcttcatt ggatttggac taagatttgg agcaaaatgg aactttgcaa atgcatatga 3120
taatcatgtt tttgtggctg gaagattaat ttactgtctt aacataatat tttggtatgt 3180
gcgtttgcta gatttctag ctgtaaatca acaggcagga ccttatgtaa tgatgattgg 3240
aaaaatggtg gccaatatgt tctacattgt agtgattatg gctcttgtat tacttagttt 3300
tggtgttccc agaaaggcaa tactttatcc tcatgaagca ccatcttgga ctcttgctaa 3360
agatatagtt tttcacccat actggatgat ttttggtgaa gtttatgcat acgaaattga 3420
tgtgtgtgca aatgattctg ttatccctca aatctgtggt cctgggacgt ggttgactcc 3480
atttcttcaa gcagtctacc tctttgtaca gtatatcatt atggttaatc ttcttattgc 3540
atttttcagc aatgtgtatt tacaagtgaa ggcaatttcc aatattgtat ggaagtacca 3600
gcgttatcat tttattatgg cttatcatga gaaaccagtt ctgcctcctc cacttatcat 3660
tcttagccat atagtttctc tgttttgctg catatgtaag agaagaaaga aagataagac 3720
ttccgatgga ccagaacttt tcttaacaga agaagatcaa aagaaacttc atgattttga 3780

agagcagtgt gttgaaatgt atttcaatga aaaagatgac aaatttcatt ctgggagtga 3840
agagagaatt cgtgtcactt ttgaaagagt ggaacagatg tgcattcaga ttaaagaagt 3900
tggagatcgt gtcaactaca taaaaagatc attacaatca ttagattctc aaattggcca 3960
tttgcaagat ctttcagccc tgacggtaga tacattaaaa acactcactg cccagaaagc 4020
gtcggaagct agcaaagttc ataatgaaat cacacgagaa ctgagcattt ccaaacactt 4080
ggctcaaaac cttattgatg atggtcctgt aagaccttct gtatggaaaa agcatggtgt 4140
tgtaaataca cttagctcct ctcttcctca aggtgatctt gaaagtaata atcctttca 4200
ttgtaatatt ttaatgaaag atgacaaaga tccccagtgt aatatatttg gtcaagactt 4260
acctgcagta ccccagagaa aagaatttaa ttttccagag gctggttcct cttctggtgc 4320
cttattccca agtgctgttt cccctccaga actgcgacag agactacatg ggtagaact 4380
cttaaaaata tttaataaaa atcaaaaatt aggcagttca tctactagca taccacatct 4440
gtcatcccca ccaaccaaat tttttgttag tacaccatct cagccaagtt gcaaaagcca 4500
cttggaaact ggaaccaaag atcaagaaac tgtttgctct aaagctacag aaggagataa 4560
tacagaattt ggagcatttg taggtcacag agatagcatg gatttacaga ggtttaaaga 4620
aacatcaaac aagataaaat tgcagaataa caatacttct gaaaacactt tgaaacgagt 4680
gagttctctt gctggattta ctgactgtca cagaacttcc attcctgttc attcaaaaca 4740
agcagaaaaa atcagtagaa ggccatctac cgaagacact catgaagtag attccaaagc 4800
agctttatta ctgaaggatt ggttacaaga tagaccatca aacagagaaa tgggtctcac 4860
ttctccattt aagccagcta tggatacaaa ttactattat tcagctgtgg aaagaaataa 4920
cttgatgagg ttatcacaga gcattccatt tacacctgtg cctccaagag gggagcctgt 4980
cacagtgtat cgtttggaag agagttcacc caacatacta aataacagca tgtcttcttg 5040
gtcacaacta ggcctctgtg ccaaaataga gtttttaagc aaagaggaga tgggaggagg 5100
tttacgaaga gctgtcaaag tacagtgtac ctggtcagaa catgatatcc tcaaatcagg 5160
gcatctttat attatcaaat cttttcttcc agaggtggtt aatacatggt caagtattta 5220
caaagaagat acagttctgc atctctgtct gagagaaatt caacaacaga gagcagcaca 5280
aaagcttacg tttgccttta atcaaatgaa acccaaatcc ataccatatt ctccaaggtt 5340
ccttgaagtt ttcctgctgt attgccattc agcaggacag tggtttgctg tggaagaatg 5400
tatgactgga gaatttagaa aatacaacaa taataatgga gatgagatta ttccaactaa 5460
tactctggaa gagatcatgc tagcctttag ccactggact tacgaatata caagagggga 5520
gttactggta cttgatttgc aaggtgttgg tgaaaatttg actgacccat ctgtgataaa 5580
agcagaagaa aagagatcct gtgatatggt ttttggccca gcaaatctag gagaagatgc 5640
aattaaaaac ttcagagcaa aacatcactg taattcttgc tgtagaaagc ttaaacttcc 5700
agatctgaag aggaatgatt atacgcctga taaaattata tttcctcagg atgagccttc 5760
agatttgaat cttcagcctg gaaattccac caaagaatca gaatcaacta attctgttcg 5820
tctgatgtta taatattaat attactgaat cattggtttt gcctgcacct cacagaa 5877

<210> 8
<211> 1856
<212> PRT
<213> Homo sapiens

<400> 8
Met Ser Gln Lys Ser Trp Ile Glu Ser Thr Leu Thr Lys Arg Glu Cys
1               5                   10                  15

Val Tyr Ile Ile Pro Ser Ser Lys Asp Pro His Arg Cys Leu Pro Gly
            20                  25                  30

Cys Gln Ile Cys Gln Gln Leu Val Arg Cys Phe Cys Gly Arg Leu Val
        35                  40                  45

Lys Gln His Ala Cys Phe Thr Ala Ser Leu Ala Met Lys Tyr Ser Asp
    50                  55                  60

Val Lys Leu Gly Asp His Phe Asn Gln Ala Ile Glu Glu Trp Ser Val
65                  70                  75                  80

Glu Lys His Thr Glu Gln Ser Pro Thr Asp Ala Tyr Gly Val Ile Asn
                85                  90                  95

Phe Gln Gly Gly Ser His Ser Tyr Arg Ala Lys Tyr Val Arg Leu Ser
            100                 105                 110

Tyr Asp Thr Lys Pro Glu Val Ile Leu Gln Leu Leu Leu Lys Glu Trp
        115                 120                 125

Gln Met Glu Leu Pro Lys Leu Val Ile Ser Val His Gly Gly Met Gln
    130                 135                 140

Lys Phe Glu Leu His Pro Arg Ile Lys Gln Leu Leu Gly Lys Gly Leu
145                 150                 155                 160

Ile Lys Ala Ala Val Thr Thr Gly Ala Trp Ile Leu Thr Gly Gly Val
                165                 170                 175

Asn Thr Gly Val Ala Lys His Val Gly Asp Ala Leu Lys Glu His Ala
            180                 185                 190

Ser Arg Ser Ser Arg Lys Ile Cys Thr Ile Gly Ile Ala Pro Trp Gly
        195                 200                 205

Val Ile Glu Asn Arg Asn Asp Leu Val Gly Arg Asp Val Val Ala Pro
    210                 215                 220

Tyr Gln Thr Leu Leu Asn Pro Leu Ser Lys Leu Asn Val Leu Asn Asn
225                 230                 235                 240

Leu His Ser His Phe Ile Leu Val Asp Asp Gly Thr Val Gly Lys Tyr
                245                 250                 255

Gly Ala Glu Val Arg Leu Arg Arg Glu Leu Glu Lys Thr Ile Asn Gln
            260                 265                 270

Gln Arg Ile His Ala Ile Gly Gln Gly Val Pro Val Val Ala Leu Ile
        275                 280                 285

Phe Glu Gly Gly Pro Asn Val Ile Leu Thr Val Leu Glu Tyr Leu Gln
    290                 295                 300

Glu Ser Pro Pro Val Pro Val Val Val Cys Glu Gly Thr Gly Arg Ala
305                 310                 315                 320

Ala Asp Leu Leu Ala Tyr Ile His Lys Gln Thr Glu Glu Gly Gly Asn
                325                 330                 335

Leu Pro Asp Ala Ala Glu Pro Asp Ile Ile Ser Thr Ile Lys Lys Thr
            340                 345                 350

Phe Asn Phe Gly Gln Asn Glu Ala Leu His Leu Phe Gln Thr Leu Met
        355                 360                 365

Glu Cys Met Lys Arg Lys Glu Leu Ile Thr Val Phe His Ile Gly Ser
    370                 375                 380

Asp Glu His Gln Asp Ile Asp Val Ala Ile Leu Thr Ala Leu Leu Lys
385                 390                 395                 400

Gly Thr Asn Ala Ser Ala Phe Asp Gln Leu Ile Leu Thr Leu Ala Trp
                405                 410                 415

Asp Arg Val Asp Ile Ala Lys Asn His Val Phe Val Tyr Gly Gln Gln
            420                 425                 430

Trp Leu Val Gly Ser Leu Glu Gln Ala Met Leu Asp Ala Leu Val Met
        435                 440                 445

Asp Arg Val Ala Phe Val Lys Leu Leu Ile Glu Asn Gly Val Ser Met
    450                 455                 460

His Lys Phe Leu Thr Ile Pro Arg Leu Glu Glu Leu Tyr Asn Thr Lys
465                 470                 475                 480

Gln Gly Pro Thr Asn Pro Met Leu Phe His Leu Val Arg Asp Val Lys
                485                 490                 495

Gln Gly Asn Leu Pro Pro Gly Tyr Lys Ile Thr Leu Ile Asp Ile Gly
            500                 505                 510

Leu Val Ile Glu Tyr Leu Met Gly Gly Thr Tyr Arg Cys Thr Tyr Thr
        515                 520                 525

Arg Lys Arg Phe Arg Leu Ile Tyr Asn Ser Leu Gly Gly Asn Asn Arg
    530                 535                 540

Arg Ser Gly Arg Asn Thr Ser Ser Ser Thr Pro Gln Leu Arg Lys Ser
545                 550                 555                 560

His Glu Ser Phe Gly Asn Arg Ala Asp Lys Lys Glu Lys Met Arg His
                565                 570                 575

Asn His Phe Ile Lys Thr Ala Gln Pro Tyr Arg Pro Lys Val Asp Thr
            580                 585                 590

Val Met Glu Glu Gly Lys Lys Lys Arg Thr Lys Asp Glu Ile Val Asp
        595                 600                 605

Ile Asp Asp Pro Glu Thr Lys Arg Phe Pro Tyr Pro Leu Asn Glu Leu
    610                 615                 620

Leu Ile Trp Ala Cys Leu Met Lys Arg Gln Val Met Ala Arg Phe Leu
625                 630                 635                 640

Trp Gln His Gly Glu Glu Ser Met Ala Lys Ala Leu Val Ala Cys Lys
                645                 650                 655

Ile Tyr Arg Ser Met Ala Tyr Glu Ala Lys Gln Ser Asp Leu Val Asp
            660                 665                 670

Asp Thr Ser Glu Glu Leu Lys Gln Tyr Ser Ser Asp Phe Gly Gln Leu
        675                 680                 685

Ala Val Glu Leu Leu Glu Gln Ser Phe Arg Gln Asp Glu Thr Met Ala
    690                 695                 700

Met Lys Leu Leu Thr Tyr Glu Leu Lys Asn Trp Ser Asn Ser Thr Cys
705                 710                 715                 720

Leu Lys Leu Ala Val Ser Ser Arg Leu Arg Pro Phe Val Ala His Thr
                725                 730                 735

Cys Thr Gln Met Leu Leu Ser Asp Met Trp Met Gly Arg Leu Asn Met
            740                 745                 750

Arg Lys Asn Ser Trp Tyr Lys Val Ile Leu Ser Ile Leu Val Pro Pro
        755                 760                 765

Ala Ile Leu Leu Leu Glu Tyr Lys Thr Lys Ala Glu Met Ser His Ile
    770                 775                 780

Pro Gln Ser Gln Asp Ala His Gln Met Thr Met Asp Asp Ser Glu Asn
785                 790                 795                 800

Asn Phe Gln Asn Ile Thr Glu Glu Ile Pro Met Glu Val Phe Lys Glu
                805                 810                 815

Val Arg Ile Leu Asp Ser Asn Glu Gly Lys Asn Glu Met Glu Ile Gln
            820                 825                 830

Met Lys Ser Lys Lys Leu Pro Ile Thr Arg Lys Phe Tyr Ala Phe Tyr
        835                 840                 845

His Ala Pro Ile Val Lys Phe Trp Phe Asn Thr Leu Ala Tyr Leu Gly
    850                 855                 860

Phe Leu Met Leu Tyr Thr Phe Val Val Leu Val Gln Met Glu Gln Leu
865                 870                 875                 880

Pro Ser Val Gln Glu Trp Ile Val Ile Ala Tyr Ile Phe Thr Tyr Ala
                885                 890                 895

Ile Glu Lys Val Arg Glu Val Phe Met Ser Glu Ala Gly Lys Val Asn
            900                 905                 910

Gln Lys Ile Lys Val Trp Phe Ser Asp Tyr Phe Asn Ile Ser Asp Thr
        915                 920                 925

Ile Ala Ile Ile Ser Phe Phe Ile Gly Phe Gly Leu Arg Phe Gly Ala
    930                 935                 940

Lys Trp Asn Phe Ala Asn Ala Tyr Asp Asn His Val Phe Val Ala Gly
945                 950                 955                 960

Arg Leu Ile Tyr Cys Leu Asn Ile Ile Phe Trp Tyr Val Arg Leu Leu
                965                 970                 975

Asp Phe Leu Ala Val Asn Gln Gln Ala Gly Pro Tyr Val Met Met Ile
            980                 985                 990

Gly Lys Met Val Ala Asn Met Phe Tyr Ile Val Val Ile Met Ala Leu
        995                 1000                1005

Val Leu Leu Ser Phe Gly Val Pro Arg Lys Ala Ile Leu Tyr Pro His
    1010                1015                1020

Glu Ala Pro Ser Trp Thr Leu Ala Lys Asp Ile Val Phe His Pro Tyr
1025                1030                1035                1040

Trp Met Ile Phe Gly Glu Val Tyr Ala Tyr Glu Ile Asp Val Cys Ala
                1045                1050                1055

Asn Asp Ser Val Ile Pro Gln Ile Cys Gly Pro Gly Thr Trp Leu Thr
            1060                1065                1070

Pro Phe Leu Gln Ala Val Tyr Leu Phe Val Gln Tyr Ile Ile Met Val
        1075                1080                1085

Asn Leu Leu Ile Ala Phe Phe Ser Asn Val Tyr Leu Gln Val Lys Ala
    1090                1095                1100

Ile Ser Asn Ile Val Trp Lys Tyr Gln Arg Tyr His Phe Ile Met Ala
1105                1110                1115                1120

Tyr His Glu Lys Pro Val Leu Pro Pro Pro Leu Ile Ile Leu Ser His
                1125                1130                1135

Ile Val Ser Leu Phe Cys Cys Ile Cys Lys Arg Arg Lys Lys Asp Lys
            1140                1145                1150

Thr Ser Asp Gly Pro Glu Leu Phe Leu Thr Glu Glu Asp Gln Lys Lys
        1155                1160                1165

Leu His Asp Phe Glu Glu Gln Cys Val Glu Met Tyr Phe Asn Glu Lys
    1170                1175                1180

Asp Asp Lys Phe His Ser Gly Ser Glu Glu Arg Ile Arg Val Thr Phe
1185                1190                1195                1200

Glu Arg Val Glu Gln Met Cys Ile Gln Ile Lys Glu Val Gly Asp Arg
                1205                1210                1215

Val Asn Tyr Ile Lys Arg Ser Leu Gln Ser Leu Asp Ser Gln Ile Gly
            1220                1225                1230

His Leu Gln Asp Leu Ser Ala Leu Thr Val Asp Thr Leu Lys Thr Leu
        1235                1240                1245

Thr Ala Gln Lys Ala Ser Glu Ala Ser Lys Val His Asn Glu Ile Thr
    1250                1255                1260

Arg Glu Leu Ser Ile Ser Lys His Leu Ala Gln Asn Leu Ile Asp Asp
1265                1270                1275                1280

Gly Pro Val Arg Pro Ser Val Trp Lys Lys His Gly Val Val Asn Thr
                1285                1290                1295

Leu Ser Ser Ser Leu Pro Gln Gly Asp Leu Glu Ser Asn Asn Pro Phe
            1300                1305                1310

His Cys Asn Ile Leu Met Lys Asp Asp Lys Asp Pro Gln Cys Asn Ile
        1315                1320                1325

Phe Gly Gln Asp Leu Pro Ala Val Pro Gln Arg Lys Glu Phe Asn Phe
    1330                1335                1340

Pro Glu Ala Gly Ser Ser Ser Gly Ala Leu Phe Pro Ser Ala Val Ser
1345                1350                1355                1360

Pro Pro Glu Leu Arg Gln Arg Leu His Gly Val Glu Leu Leu Lys Ile
                1365                1370                1375

Phe Asn Lys Asn Gln Lys Leu Gly Ser Ser Ser Thr Ser Ile Pro His
            1380                1385                1390

Leu Ser Ser Pro Pro Thr Lys Phe Phe Val Ser Thr Pro Ser Gln Pro
        1395                1400                1405

Ser Cys Lys Ser His Leu Glu Thr Gly Thr Lys Asp Gln Glu Thr Val
    1410                1415                1420

Cys Ser Lys Ala Thr Glu Gly Asp Asn Thr Glu Phe Gly Ala Phe Val
1425                1430                1435                1440

Gly His Arg Asp Ser Met Asp Leu Gln Arg Phe Lys Glu Thr Ser Asn
                1445                1450                1455

Lys Ile Lys Leu Gln Asn Asn Asn Thr Ser Glu Asn Thr Leu Lys Arg
            1460                1465                1470

Val Ser Ser Leu Ala Gly Phe Thr Asp Cys His Arg Thr Ser Ile Pro
        1475                1480                1485

Val His Ser Lys Gln Ala Glu Lys Ile Ser Arg Arg Pro Ser Thr Glu
    1490                1495                1500

Asp Thr His Glu Val Asp Ser Lys Ala Ala Leu Leu Leu Lys Asp Trp
1505                1510                1515                1520

Leu Gln Asp Arg Pro Ser Asn Arg Glu Met Gly Leu Thr Ser Pro Phe
                1525                1530                1535

Lys Pro Ala Met Asp Thr Asn Tyr Tyr Tyr Ser Ala Val Glu Arg Asn
            1540                1545                1550

Asn Leu Met Arg Leu Ser Gln Ser Ile Pro Phe Thr Pro Val Pro Pro
        1555                1560                1565

Arg Gly Glu Pro Val Thr Val Tyr Arg Leu Glu Glu Ser Ser Pro Asn
    1570                1575                1580

Ile Leu Asn Asn Ser Met Ser Ser Trp Ser Gln Leu Gly Leu Cys Ala
1585                1590                1595                1600

Lys Ile Glu Phe Leu Ser Lys Glu Glu Met Gly Gly Gly Leu Arg Arg
                1605                1610                1615

Ala Val Lys Val Gln Cys Thr Trp Ser Glu His Asp Ile Leu Lys Ser
            1620                1625                1630

Gly His Leu Tyr Ile Ile Lys Ser Phe Leu Pro Glu Val Val Asn Thr
        1635                1640                1645

Trp Ser Ser Ile Tyr Lys Glu Asp Thr Val Leu His Leu Cys Leu Arg
    1650                1655                1660

Glu Ile Gln Gln Gln Arg Ala Ala Gln Lys Leu Thr Phe Ala Phe Asn
1665                1670                1675                1680

Gln Met Lys Pro Lys Ser Ile Pro Tyr Ser Pro Arg Phe Leu Glu Val
                1685                1690                1695

Phe Leu Leu Tyr Cys His Ser Ala Gly Gln Trp Phe Ala Val Glu Glu
            1700                1705                1710

Cys Met Thr Gly Glu Phe Arg Lys Tyr Asn Asn Asn Asn Gly Asp Glu
        1715                1720                1725

Ile Ile Pro Thr Asn Thr Leu Glu Glu Ile Met Leu Ala Phe Ser His
    1730                1735                1740

Trp Thr Tyr Glu Tyr Thr Arg Gly Glu Leu Leu Val Leu Asp Leu Gln
1745                1750                1755                1760

Gly Val Gly Glu Asn Leu Thr Asp Pro Ser Val Ile Lys Ala Glu Glu
                1765                1770                1775

Lys Arg Ser Cys Asp Met Val Phe Gly Pro Ala Asn Leu Gly Glu Asp
            1780                1785                1790

Ala Ile Lys Asn Phe Arg Ala Lys His His Cys Asn Ser Cys Cys Arg
        1795                1800                1805

Lys Leu Lys Leu Pro Asp Leu Lys Arg Asn Asp Tyr Thr Pro Asp Lys
    1810                1815                1820

Ile Ile Phe Pro Gln Asp Glu Pro Ser Asp Leu Asn Leu Gln Pro Gly
1825                1830                1835                1840

Asn Ser Thr Lys Glu Ser Glu Ser Thr Asn Ser Val Arg Leu Met Leu
                1845                1850                1855

<210> 9
<211> 5626
<212> DNA
<213> Homo sapiens

<400> 9
agtccccagc cccgtcgccg gcggaggcgg gcgcgggcgc gttcctgtgg ccagtcaccc 60
ggaggagttg gtcgcacaat tatgaaagac tcggcttctg ctgctagcgc cggagctgag 120
ttagtcctga gaaggtttcc ctgggcgttc cttgtccggc ctctgctgcc gcctccggag 180
acgcttcccg atagatggct acaggccgcg gaggaggagg aggtggagtt gctgcccttc 240
cggagtccgc cccgtgagga gaatgtccca gaaatcctgg atagaaagca ctttgaccaa 300
gagggaatgt gtatatatta taccaagttc caaggaccct cacaggtgcc ttccaggatg 360
tcaaatttgt cagcaactcg tcagggtttt tgtggtcgct tggtcaagca acatgcttgt 420
tttactgcaa gtcttgccat gaaatactca gatgtgaaat tgggtgacca ttttaatcag 480
gcaatagaag aatggtctgt ggaaaagcat acagaacaga gcccaacgga tgcttatgga 540
gtcataaatt tttcaagggg ttctcattcc tacagagcta agtatgtgag gctatcatat 600
gacaccaaac ctgaagtcat tctgcaactt ctgcttaaag aatggcaaat ggagttaccc 660
aaacttgtta tctctgtaca tgggggcatg cagaaatttg agcttcaccc acgaatcaag 720
cagttgcttg gaaaaggtct tattaaagct gcagttacaa ctggagcctg gattttaact 780

ggaggagtaa acacaggtac aggtgtggca aaacatgttg gagatgccct caaagaacat 840
gcttccagat catctcgaaa gatttgcact atcggaatag ctccatgggg agtgattgaa 900
aacagaaatg atcttgttgg gagagatgta agaattattt atcaaacctt attgaacccc 960
ctgagcaaat tgaatgtttt gaataatctg cattcccatt tcatattggt ggatgatggc 1020
actgttggaa agtatggggc ggaagtcaga ctgagaagag aacttgaaaa aactattaat 1080
cagcaaagaa ttcatattgg ccagggtgtc cctgtggtgg cacttatatt tgagggtggg 1140
ccaaatgtta tcctcacagt tcttgaatac cttcaggaaa gcccccctgt tccagtagtt 1200
gtgtgtgaag gaacaggcag agctgcagat ctgctagcgt atattcataa acaaacagaa 1260
gaaggagggg atgcagcaga gcccgatatt atttccacta tcaaaaaaac atttaacttt 1320
ggccagaatg aagcacttca tttatttcaa acactgatgg agtgcatgaa aagaaaggag 1380
cttgtaactg ttttccatat tgggtcagat gaacatcaag atatagatgt agcaatactt 1440
actgcactgc taaaaggtac taatgcatct gcatttgacc agcttatcct tacattggca 1500
tgggatagag ttgacattgc caaaaatcat gtatttgttt atggacagca gtggccattg 1560
cactccagcc tgggcaacag agtgagactc tctctcaaaa aaaaaaaaca aaaacaaaaa 1620
caaaaacaaa aacaaaaacc aacacctaga aattcagagt tagttggatc cttggaacaa 1680
gctatgcttg atgctcttgt aatggataga gttgcatttg taaaacttct tattgaaaat 1740
ggagtaagca tgcataaatt ccttaccatt ccgagactgg aagaacttta caacactaat 1800
cttcctccag gatataagat cactctgatt gatataggac ttgttattga atatctcatg 1860
ggaggaacct acagatgcac ctatactagg aaacgttttc gattaatata taatagtctt 1920
ggtggaaata atcggttttc cttccaggag cccaaccaca ctcgcacggt aaatattaga 1980
gacaaatctc ctcatgcttc tggcaagaag aagggaaaga agaaaagaac caaagatgaa 2040
attgtagaca ttgatgatcc agaaaccaag cgctttcctt atccacttaa tgaactttta 2100
atttgggctt gccttatgaa gaggcaggtc atggcccgtt ttttatggca acatggtgaa 2160
gaatcaatgg ctaaagcatt agttgcctgt aagatctatc gttcaatggc atatgaagca 2220
aagcagagtg acctggtaga tgatacttca gaagaactaa aacagtattc caaggatttt 2280
ggtcagttgg ccgttgaatt attagaacag tccttcagac aagatgaaac catggctatg 2340
aaattgctca cttatgaact gaagaactgg agtaattcaa cctgccttaa gttagcagtt 2400
tcttcaagac ttagaccttt tgtagctcac acctgtacac aaatgttgtt atctgatatg 2460
tggatgggaa ggctgaatat gaggaaaaat tcctggtaca aggtaatact aagcatttta 2520
gttccacctg ccatattgct gttagagtat aaaactaagg ctgaaatgtc ccatatccca 2580
caatctcaag atgctcatca gatgacaatg gatgacagcg aaaacaacag taatgaagga 2640
aagaatgaga tggagataca aatgaaatca aaaaagcttc caattacgcg aaagttttat 2700
gccttttatc atgcaccaat tgtaaaattc tggtttaaca cgttggcata tttaggattt 2760
ctgatgcttt atacatttgt ggttcttgta caaatggaac agttaccttc agttcaagaa 2820
tggattgtta ttgcttatat ttttacttat gccattgaga aagtccgtga gatctttatg 2880
tctgaagctg ggaaagtaaa ccagaagatt aaagtatggt ttagtgatta cttcaacatc 2940
agtgatacaa ttgccataat ttctttcttc attggatttg gactaagatt tggagcaaaa 3000
tggaactttg caaatgcata tgataatcat gtttttgtgg ctggaagatt aatttactgt 3060
cttaacataa tattttggta tgtgcgtttg ctagattttc tagctgttaa tcaacaggca 3120
ggaccttatg taatgatgat tggaaaaatg gtaaatatgt tctacattgt agtgattatg 3180
gctcttgtat tacttagttt tggtgttccc agaaaggcaa tactttatcc tcatgaagca 3240
ccatcttgga ctcttgctaa agatatagtt tttcacccat actggatgat ttttggtgaa 3300
gtttatgcat acgaaattga ttgtggtcct gggacgtggt tgactccatt tcttcaagca 3360
gtctacctct ttgtacagta tatcattatg gttaatcttc ttattgcatt tttcaagagc 3420
aatgtgtatt tacaagtgaa ggcaatttcc aatattgtat ggaagtacca gcgttatcat 3480
tttattatgg cttatcatga gaaaccagtt ctgcctcctc cacttatcat tcttagccat 3540
atagtttctc tgttttgctg catatgtaag agaagaaaga aagataagac ttccgatgga 3600
ccaagtaaga tagaactttt cttaacagaa gaagatcaaa agaaacttca tgattttgaa 3660
gagcagtgtg ttgaaatgta tttcaatgaa aaagatgaca aatttcattc tgggagtgaa 3720
gagagaattc gtgtcacttt tgaaagagtg gaacagaagc ccattcagat taaagaagtt 3780
ggagatcgtg tcaactacat aaaaagatca ttacaatcat tagattctca aattggccat 3840
ttgcaagatc tttcagccct gacggtagat acattaaaaa cactcactgc ccagaaagcg 3900
tcggaagcta gcaaagttca taatgaaatc acacgagaac tgagcatttc caaacacttg 3960
gctcaaaacc ttattgatga tggtcctgta agaccttctg tatggaaaaa gcatggtgtt 4020
gtaaatacac ttagctcctc tcttcctcaa ggtgatcttg aaagtaataa tccttttcat 4080
tgtaatattt taatgaaaga tgacaaagat ccccagtgta atatatttgg tcaagactta 4140
cctgcagtac cccagagaaa agaatttaat tttccagagg ctggttcctc ttctggtgcc 4200
ttattcccaa gtgctgtttc ccctccagaa ctgcgacaga gactacatgg ggtagaactc 4260
ttaaaaatat ttaataaaaa tcaaaaatta ggcagttcat ctactagcat accacatctg 4320
tcatccccac caaccaaatt ttttgttagt acaccatctc agccaagttg caaaagccac 4380
ttggaaactg gaaccaaaga tcaagaaact gtttgctcta aagctacaga aggagataat 4440
acagaatttg gagcatttgt aggtcacaga gatagcatgg atttacagag gtttaaagaa 4500
acatcaaaca agataaaatt gcagaataac aatacttctg aaaacacttt gaaacgagtg 4560
agttctcttg ctggatttac tgactgtcac agaacttcca ttcctgttca ttcaaaacaa 4620
gcagaaaaaa tcagtagaag gccatctacc gaagacactc atgaagtaga ttccaaagca 4680
gctttaatac cggattggtt acaagataga ccatcaaaca gagaaatggg tctcacttct 4740
ccatttaagc cagctatgga tacaaattac tattattcag ctgtggaaag aaataacttg 4800
atgaggttat cacagagcat tccatttaca cctgtgcctc caagagggga gcctgtcaca 4860
gtgtatcgtt tggaagagag ttcacccaac atactaaata acagcatgtc ttcttggtca 4920

caactaggcc tctgtgccaa aatagagttt ttaagcaaag aggagatggg aggaggttta 4980
cgaagagctg tcaaagtaca gtgtacctgg tcagaacatg atatcctcaa atcagggcat 5040
ctttatatta tcaaatcttt tcttccagag gtggttaata catggtcaag tatttacaaa 5100
gaagatacag ttctgcatct ctgtctgaga gaaattcaac aacagagagc agcacaaaag 5160
cttacgtttg cctttaatca aatgaaaccc aaatccatac catattctcc aggggagtta 5220
ctggtacttg atttgcaagg tgttggtgaa aatttgactg acccatctgt gataaaagca 5280
gaagaaaaga gatcctgtga tatggttttt ggcccagcaa atctaggaga agatgcaatt 5340
aaaaacttca gagcaaaaca tcactgtaat tcttgctgta gaaagcttaa acttccagat 5400
ctgaagagga atgattatac gcctgataaa attatatttc ctcaggatga gccttcagat 5460
ttgaatcttc agcctggaaa ttccaccaaa gaatcagaat caactaattc tgttcgtctg 5520
atgttataat attaatatta ctgaatcatt ggttttgcct gcacctcaca gaaatgttac 5580
tgtgtcactt ttccctcggg aggaaattgt ttggtaatat agaaag 5626

<210> 10
<211> 1815
<212> PRT
<213> Homo sapiens

<400> 10
   Met Lys Asp Ser Ala Ser Ala Ala Ser Ala Gly Ala Glu Leu Val Leu
   1 5 10 15
   Arg Arg Phe Pro Trp Ala Phe Leu Val Arg Pro Leu Leu Pro Pro Pro
   20 25 30
   Glu Thr Leu Pro Asp Arg Trp Leu Gln Ala Ala Glu Glu Glu Glu Val
   35 40 45
   Glu Leu Leu Pro Phe Arg Ser Pro Pro Arg Glu Glu Asn Val Pro Glu
   50 55 60
   Ile Leu Asp Arg Lys His Phe Asp Gln Glu Gly Met Cys Ile Tyr Tyr
   65 70 75 80
   Thr Lys Phe Gln Gly Pro Ser Gln Val Pro Ser Arg Met Ser Asn Leu
   85 90 95
   Ser Ala Thr Arg Gln Gly Phe Cys Gly Arg Leu Val Lys Gln His Ala
   100 105 110
   Cys Phe Thr Ala Ser Leu Ala Met Lys Tyr Ser Asp Val Lys Leu Gly
   115 120 125
   Asp His Phe Asn Gln Ala Ile Glu Glu Trp Ser Val Glu Lys His Thr
   130 135 140
   Glu Gln Ser Pro Thr Asp Ala Tyr Gly Val Ile Asn Phe Gln Gly Gly
   145 150 155 160
   Ser His Ser Tyr Arg Ala Lys Tyr Val Arg Leu Ser Tyr Asp Thr Lys
   165 170 175
   Pro Glu Val Ile Leu Gln Leu Leu Leu Lys Glu Trp Gln Met Glu Leu
   180 185 190
   Pro Lys Leu Val Ile Ser Val His Gly Gly Met Gln Lys Phe Glu Leu
   195 200 205
   His Pro Arg Ile Lys Gln Leu Leu Gly Lys Gly Leu Ile Lys Ala Ala
   210 215 220
   Val Thr Thr Gly Ala Trp Ile Leu Thr Gly Gly Val Asn Thr Gly Thr
   225 230 235 240
   Gly Val Ala Lys His Val Gly Asp Ala Leu Lys Glu His Ala Ser Arg
   245 250 255
   Ser Ser Arg Lys Ile Cys Thr Ile Gly Ile Ala Pro Trp Gly Val Ile
   260 265 270
   Glu Asn Arg Asn Asp Leu Val Gly Arg Asp Val Arg Ile Ile Tyr Gln
   275 280 285
   Thr Leu Leu Asn Pro Leu Ser Lys Leu Asn Val Leu Asn Asn Leu His
   290 295 300
   Ser His Phe Ile Leu Val Asp Asp Gly Thr Val Gly Lys Tyr Gly Ala
   305 310 315 320
   Glu Val Arg Leu Arg Arg Glu Leu Glu Lys Thr Ile Asn Gln Gln Arg
   325 330 335
   Ile His Ile Gly Gln Gly Val Pro Val Val Ala Leu Ile Phe Glu Gly
   340 345 350
   Gly Pro Asn Val Ile Leu Thr Val Leu Glu Tyr Leu Gln Glu Ser Pro
   355 360 365
   Pro Val Pro Val Val Val Cys Glu Gly Thr Gly Arg Ala Ala Asp Leu
   370 375 380
   Leu Ala Tyr Ile His Lys Gln Thr Glu Glu Gly Gly Asp Ala Ala Glu
   385 390 395 400
   Pro Asp Ile Ile Ser Thr Ile Lys Lys Thr Phe Asn Phe Gly Gln Asn
   405 410 415
   Glu Ala Leu His Leu Phe Gln Thr Leu Met Glu Cys Met Lys Arg Lys
   420 425 430
   Glu Leu Val Thr Val Phe His Ile Gly Ser Asp Glu His Gln Asp Ile
   435 440 445
   Asp Val Ala Ile Leu Thr Ala Leu Leu Lys Gly Thr Asn Ala Ser Ala
   450 455 460
   Phe Asp Gln Leu Ile Leu Thr Leu Ala Trp Asp Arg Val Asp Ile Ala
   465 470 475 480
   Lys Asn His Val Phe Val Tyr Gly Gln Gln Trp Pro Leu His Ser Ser
   485 490 495
   Leu Gly Asn Arg Val Arg Leu Ser Leu Lys Lys Lys Lys Gln Lys Gln
   500 505 510
   Lys Gln Lys Gln Lys Gln Lys Pro Thr Pro Arg Asn Ser Glu Leu Val
   515 520 525
   Gly Ser Leu Glu Gln Ala Met Leu Asp Ala Leu Val Met Asp Arg Val
   530 535 540
   Ala Phe Val Lys Leu Leu Ile Glu Asn Gly Val Ser Met His Lys Phe
   545 550 555 560
   Leu Thr Ile Pro Arg Leu Glu Glu Leu Tyr Asn Thr Asn Leu Pro Pro
   565 570 575
   Gly Tyr Lys Ile Thr Leu Ile Asp Ile Gly Leu Val Ile Glu Tyr Leu
   580 585 590
   Met Gly Gly Thr Tyr Arg Cys Thr Tyr Thr Arg Lys Arg Phe Arg Leu
   595 600 605
   Ile Tyr Asn Ser Leu Gly Gly Asn Asn Arg Phe Ser Phe Gln Glu Pro
   610 615 620
   Asn His Thr Arg Thr Val Asn Ile Arg Asp Lys Ser Pro His Ala Ser
   625                 630                 635                 640
   Gly Lys Lys Lys Gly Lys Lys Lys Arg Thr Lys Asp Glu Ile Val Asp
                   645                 650                 655
   Ile Asp Asp Pro Glu Thr Lys Arg Phe Pro Tyr Pro Leu Asn Glu Leu
               660                 665                 670
   Leu Ile Trp Ala Cys Leu Met Lys Arg Gln Val Met Ala Arg Phe Leu
           675                 680                 685
   Trp Gln His Gly Glu Glu Ser Met Ala Lys Ala Leu Val Ala Cys Lys
       690                 695                 700
   Ile Tyr Arg Ser Met Ala Tyr Glu Ala Lys Gln Ser Asp Leu Val Asp
   705                 710                 715                 720
   Asp Thr Ser Glu Glu Leu Lys Gln Tyr Ser Lys Asp Phe Gly Gln Leu
                   725                 730                 735
   Ala Val Glu Leu Leu Glu Gln Ser Phe Arg Gln Asp Glu Thr Met Ala
               740                 745                 750
   Met Lys Leu Leu Thr Tyr Glu Leu Lys Asn Trp Ser Asn Ser Thr Cys
           755                 760                 765
   Leu Lys Leu Ala Val Ser Ser Arg Leu Arg Pro Phe Val Ala His Thr
       770                 775                 780
   Cys Thr Gln Met Leu Leu Ser Asp Met Trp Met Gly Arg Leu Asn Met
   785                 790                 795                 800
   Arg Lys Asn Ser Trp Tyr Lys Val Ile Leu Ser Ile Leu Val Pro Pro
                   805                 810                 815
   Ala Ile Leu Leu Leu Glu Tyr Lys Thr Lys Ala Glu Met Ser His Ile
               820                 825                 830
   Pro Gln Ser Gln Asp Ala His Gln Met Thr Met Asp Asp Ser Glu Asn
           835                 840                 845
   Asn Ser Asn Glu Gly Lys Asn Glu Met Glu Ile Gln Met Lys Ser Lys
       850                 855                 860
   Lys Leu Pro Ile Thr Arg Lys Phe Tyr Ala Phe Tyr His Ala Pro Ile
   865                 870                 875                 880
   Val Lys Phe Trp Phe Asn Thr Leu Ala Tyr Leu Gly Phe Leu Met Leu
                   885                 890                 895
   Tyr Thr Phe Val Val Leu Val Gln Met Glu Gln Leu Pro Ser Val Gln
               900                 905                 910
   Glu Trp Ile Val Ile Ala Tyr Ile Phe Thr Tyr Ala Ile Glu Lys Val
           915                 920                 925
   Arg Glu Ile Phe Met Ser Glu Ala Gly Lys Val Asn Gln Lys Ile Lys
       930                 935                 940
   Val Trp Phe Ser Asp Tyr Phe Asn Ile Ser Asp Thr Ile Ala Ile Ile
   945                 950                 955                 960
   Ser Phe Phe Ile Gly Phe Gly Leu Arg Phe Gly Ala Lys Trp Asn Phe
                   965                 970                 975
   Ala Asn Ala Tyr Asp Asn His Val Phe Val Ala Gly Arg Leu Ile Tyr
               980                 985                 990
   Cys Leu Asn Ile Ile Phe Trp Tyr Val Arg Leu Leu Asp Phe Leu Ala
   995                    1000                    1005
   Val Asn Gln Gln Ala Gly Pro Tyr Val Met Met Ile Gly Lys Met Val
      1010                1015                1020
   Asn Met Phe Tyr Ile Val Val Ile Met Ala Leu Val Leu Leu Ser Phe
   1025                1030                1035                1040
   Gly Val Pro Arg Lys Ala Ile Leu Tyr Pro His Glu Ala Pro Ser Trp
                   1045                1050                1055
   Thr Leu Ala Lys Asp Ile Val Phe His Pro Tyr Trp Met Ile Phe Gly
               1060                1065                1070
   Glu Val Tyr Ala Tyr Glu Ile Asp Cys Gly Pro Gly Thr Trp Leu Thr
           1075                1080                1085
   Pro Phe Leu Gln Ala Val Tyr Leu Phe Val Gln Tyr Ile Ile Met Val
      1090                1095                1100
   Asn Leu Leu Ile Ala Phe Phe Lys Ser Asn Val Tyr Leu Gln Val Lys
   1105                1110                1115                1120
   Ala Ile Ser Asn Ile Val Trp Lys Tyr Gln Arg Tyr His Phe Ile Met
                   1125                1130                1135
   Ala Tyr His Glu Lys Pro Val Leu Pro Pro Pro Leu Ile Ile Leu Ser
               1140                1145                1150
   His Ile Val Ser Leu Phe Cys Cys Ile Cys Lys Arg Arg Lys Lys Asp
           1155                1160                1165
   Lys Thr Ser Asp Gly Pro Ser Lys Ile Glu Leu Phe Leu Thr Glu Glu
      1170                1175                1180
   Asp Gln Lys Lys Leu His Asp Phe Glu Glu Gln Cys Val Glu Met Tyr
   1185                1190                1195                1200
   Phe Asn Glu Lys Asp Asp Lys Phe His Ser Gly Ser Glu Glu Arg Ile
                   1205                1210                1215
   Arg Val Thr Phe Glu Arg Val Glu Gln Lys Pro Ile Gln Ile Lys Glu
               1220                1225                1230
   Val Gly Asp Arg Val Asn Tyr Ile Lys Arg Ser Leu Gln Ser Leu Asp
           1235                1240                1245
   Ser Gln Ile Gly His Leu Gln Asp Leu Ser Ala Leu Thr Val Asp Thr
      1250                1255                1260
   Leu Lys Thr Leu Thr Ala Gln Lys Ala Ser Glu Ala Ser Lys Val His
   1265                1270                1275                1280
   Asn Glu Ile Thr Arg Glu Leu Ser Ile Ser Lys His Leu Ala Gln Asn
                   1285                1290                1295
   Leu Ile Asp Asp Gly Pro Val Arg Pro Ser Val Trp Lys Lys His Gly
               1300                1305                1310
   Val Val Asn Thr Leu Ser Ser Ser Leu Pro Gln Gly Asp Leu Glu Ser
           1315                1320                1325
   Asn Asn Pro Phe His Cys Asn Ile Leu Met Lys Asp Asp Lys Asp Pro
      1330                1335                1340
   Gln Cys Asn Ile Phe Gly Gln Asp Leu Pro Ala Val Pro Gln Arg Lys
   1345                1350                1355                1360
   Glu Phe Asn Phe Pro Glu Ala Gly Ser Ser Ser Gly Ala Leu Phe Pro
   1365                                1370                                1375
   Ser Ala Val Ser Pro Pro Glu Leu Arg Gln Arg Leu His Gly Val Glu
               1380                                1385                                1390
   Leu Leu Lys Ile Phe Asn Lys Asn Gln Lys Leu Gly Ser Ser Ser Thr
           1395                                1400                                1405
   Ser Ile Pro His Leu Ser Ser Pro Pro Thr Lys Phe Phe Val Ser Thr
       1410                                1415                                1420
   Pro Ser Gln Pro Ser Cys Lys Ser His Leu Glu Thr Gly Thr Lys Asp
   1425                                1430                                1435                                1440
   Gln Glu Thr Val Cys Ser Lys Ala Thr Glu Gly Asp Asn Thr Glu Phe
                   1445                                1450                                1455
   Gly Ala Phe Val Gly His Arg Asp Ser Met Asp Leu Gln Arg Phe Lys
               1460                                1465                                1470
   Glu Thr Ser Asn Lys Ile Lys Leu Gln Asn Asn Asn Thr Ser Glu Asn
           1475                                1480                                1485
   Thr Leu Lys Arg Val Ser Ser Leu Ala Gly Phe Thr Asp Cys His Arg
       1490                                1495                                1500
   Thr Ser Ile Pro Val His Ser Lys Gln Ala Glu Lys Ile Ser Arg Arg
   1505                                1510                                1515                                1520
   Pro Ser Thr Glu Asp Thr His Glu Val Asp Ser Lys Ala Ala Leu Ile
                   1525                                1530                                1535
   Pro Asp Trp Leu Gln Asp Arg Pro Ser Asn Arg Glu Met Gly Leu Thr
               1540                                1545                                1550
   Ser Pro Phe Lys Pro Ala Met Asp Thr Asn Tyr Tyr Tyr Ser Ala Val
           1555                                1560                                1565
   Glu Arg Asn Asn Leu Met Arg Leu Ser Gln Ser Ile Pro Phe Thr Pro
       1570                                1575                                1580
   Val Pro Pro Arg Gly Glu Pro Val Thr Val Tyr Arg Leu Glu Glu Ser
   1585                                1590                                1595                                1600
   Ser Pro Asn Ile Leu Asn Asn Ser Met Ser Ser Trp Ser Gln Leu Gly
                   1605                                1610                                1615
   Leu Cys Ala Lys Ile Glu Phe Leu Ser Lys Glu Glu Met Gly Gly Gly
               1620                                1625                                1630
   Leu Arg Arg Ala Val Lys Val Gln Cys Thr Trp Ser Glu His Asp Ile
           1635                                1640                                1645
   Leu Lys Ser Gly His Leu Tyr Ile Ile Lys Ser Phe Leu Pro Glu Val
       1650                                1655                                1660
   Val Asn Thr Trp Ser Ser Ile Tyr Lys Glu Asp Thr Val Leu His Leu
   1665                                1670                                1675                                1680
   Cys Leu Arg Glu Ile Gln Gln Gln Arg Ala Ala Gln Lys Leu Thr Phe
                   1685                                1690                                1695
   Ala Phe Asn Gln Met Lys Pro Lys Ser Ile Pro Tyr Ser Pro Gly Glu
               1700                                1705                                1710
   Leu Leu Val Leu Asp Leu Gln Gly Val Gly Glu Asn Leu Thr Asp Pro
           1715                                1720                                1725
   Ser Val Ile Lys Ala Glu Glu Lys Arg Ser Cys Asp Met Val Phe Gly
   1730                1735                1740
   Pro Ala Asn Leu Gly Glu Asp Ala Ile Lys Asn Phe Arg Ala Lys His
   1745                1750                1755                1760
   His Cys Asn Ser Cys Cys Arg Lys Leu Lys Leu Pro Asp Leu Lys Arg
                   1765                1770                1775
   Asn Asp Tyr Thr Pro Asp Lys Ile Ile Phe Pro Gln Asp Glu Pro Ser
               1780                1785                1790
   Asp Leu Asn Leu Gln Pro Gly Asn Ser Thr Lys Glu Ser Glu Ser Thr
           1795                1800                1805
   Asn Ser Val Arg Leu Met Leu
       1810                1815

<210> 11
<211> 1869
<212> DNA
<213> Homo sapiens

<400> 11
   gcgggggcga gggccaccca cctccaagtc tccagccatg acgacctccg cactccggcg 60
   ccaggtgaag aacatcgtgc acaactactc cgaggcagaa atcaaggtgc gcgaggccac 120
   cagcaatgac ccctggggcc cccctagttc gctcatgtcc gagatcgctg acctgacctt 180
   caacacagtg gccttcaccg aagtcatggg catgctgtgg cggcggctca atgacagcgg 240
   caagaactgg cggcacgtgt acaaggctct aacattgctg gactacctgc tcaagacggg 300
   ctccgagcgg gtggcccacc agtgccgcga gaacctctac accatccaga cactcaagga 360
   cttccagtac atcgaccgcg acggcaagga ccagggcgtc aacgtgcgcg agaaggtcaa 420
   gcaggtgatg gccctgctca aggatgagga gcggctgcgg caggagcgaa cccacgccct 480
   caagaccaag gagcgcatgg cactggaggg catcggcccg ctggtgctgg gcttcagccg 540
   ccgctacggc gaggactaca gccgctcccg gggctccccg tcctcctaca actcctcctc 600
   ttcgtcaccc cgctatacct ccgacctgga gcaggcccgg cctcagacgt caggggaaga 660
   ggaactgcag ctgcagctgg ccctcgccat gagccgtgag gaggcagaga aggaggtgag 720
   gtcctggcag ggtgatggct cccccatggc caatggtgca ggggccgtgg tccaccatca 780
   gcgggacaga gagcctgaga gagaagagag aaaggaggag gagaagctaa aaaccagcca 840
   gtcctccatc ctggacttgg ctgacatctt cgtacctgcc ctggccccgc cctccacaca 900
   ctgctctgct gacccatggg acatcccagg ttttaggccg aacacagagg ccagtggatc 960
   ctcctggggg ccttctgcag acccctggtc tccgatcccc tcaggaaccg tcctgtcccg 1020
   aagccagccc tgggatctga ctcccatgct ctcctcctct gagccctggg gcaggacccc 1080
   agtgctgcct gctgggcccc ccaccacaga cccctgggcc ctgaactctc cccaccacaa 1140
   actccccagc actggggctg acccttgggg agcctccctg gagacctccg acacacctgg 1200
   tggtgcctcg acctttgacc catttgccaa acctccagaa tccacagaga ccaaggaggg 1260
   gctggagcag gccctgccct ctgggaagcc cagcagcagc ggggagctgg acctgtttgg 1320
   agaccccagc cccagttcca agcaaaatgg cacgaaggag ccagatgccc tggacctggg 1380
   catactaggg gaagcactaa cccagccaag caaagaggcc cgagcttgcc ggactcccga 1440
   gtccttcctg ggtccctcag cttcctcctt ggtcaacctt gactcgttgg tcaaggcacc 1500
   ccaggttgca aagacccgga accccttcct gacaggtggt ctcagcgctc cgtcccccac 1560
   caacccgttc ggcgcgggcg agccgggcag gccgacgcta aaccagatgc gcaccggctc 1620
   gccggcgctg ggcctggcag gcgggcctgt gggggcgccc ctgggctcca tgacctacag 1680
   cgcctctctg cccctcccgc tcagcagcgt gccagctggc ttgaccctcc ccgcctcggt 1740
   tagcgtcttc ccgcaggccg gagccttcgc accgcagccg ctgctgccca cgccgagctc 1800
   agccgggccg cggcccccgc ccccgcagac cggcaccaac cccttcctct gagccccgcc 1860
   ccgtcccat                                                         1869

<210> 12
<211> 604
<212> PRT
<213> Homo sapiens

<400> 12
Met Thr Thr Ser Ala Leu Arg Arg Gln Val Lys Asn Ile Val His Asn
1               5                   10                  15

Tyr Ser Glu Ala Glu Ile Lys Val Arg Glu Ala Thr Ser Asn Asp Pro
            20                  25                  30

Trp Gly Pro Pro Ser Ser Leu Met Ser Glu Ile Ala Asp Leu Thr Phe
        35                  40                  45
Asn Thr Val Ala Phe Thr Glu Val Met Gly Met Leu Trp Arg Arg Leu
    50                  55                  60
Asn Asp Ser Gly Lys Asn Trp Arg His Val Tyr Lys Ala Leu Thr Leu
65                  70                  75                  80
Leu Asp Tyr Leu Leu Lys Thr Gly Ser Glu Arg Val Ala His Gln Cys
                85                  90                  95
Arg Glu Asn Leu Tyr Thr Ile Gln Thr Leu Lys Asp Phe Gln Tyr Ile
            100                 105                 110
Asp Arg Asp Gly Lys Asp Gln Gly Val Asn Val Arg Glu Lys Val Lys
        115                 120                 125
Gln Val Met Ala Leu Leu Lys Asp Glu Glu Arg Leu Arg Gln Glu Arg
    130                 135                 140
Thr His Ala Leu Lys Thr Lys Glu Arg Met Ala Leu Glu Gly Ile Gly
145                 150                 155                 160
Pro Leu Val Leu Gly Phe Ser Arg Arg Tyr Gly Glu Asp Tyr Ser Arg
                165                 170                 175
Ser Arg Gly Ser Pro Ser Ser Tyr Asn Ser Ser Ser Ser Ser Pro Arg
            180                 185                 190
Tyr Thr Ser Asp Leu Glu Gln Ala Arg Pro Gln Thr Ser Gly Glu Glu
        195                 200                 205
Glu Leu Gln Leu Gln Leu Ala Leu Ala Met Ser Arg Glu Glu Ala Glu
    210                 215                 220
Lys Glu Val Arg Ser Trp Gln Gly Asp Gly Ser Pro Met Ala Asn Gly
225                 230                 235                 240
Ala Gly Ala Val Val His His Gln Arg Asp Arg Glu Pro Glu Arg Glu
                245                 250                 255
Glu Arg Lys Glu Glu Glu Lys Leu Lys Thr Ser Gln Ser Ser Ile Leu
            260                 265                 270
Asp Leu Ala Asp Ile Phe Val Pro Ala Leu Ala Pro Pro Ser Thr His
        275                 280                 285
Cys Ser Ala Asp Pro Trp Asp Ile Pro Gly Phe Arg Pro Asn Thr Glu
    290                 295                 300
Ala Ser Gly Ser Ser Trp Gly Pro Ser Ala Asp Pro Trp Ser Pro Ile
305                 310                 315                 320
Pro Ser Gly Thr Val Leu Ser Arg Ser Gln Pro Trp Asp Leu Thr Pro
                325                 330                 335
Met Leu Ser Ser Ser Glu Pro Trp Gly Arg Thr Pro Val Leu Pro Ala
            340                 345                 350
Gly Pro Pro Thr Thr Asp Pro Trp Ala Leu Asn Ser Pro His His Lys
        355                 360                 365
Leu Pro Ser Thr Gly Ala Asp Pro Trp Gly Ala Ser Leu Glu Thr Ser
    370                 375                 380
Asp Thr Pro Gly Gly Ala Ser Thr Phe Asp Pro Phe Ala Lys Pro Pro
385                 390                 395                 400

Glu Ser Thr Glu Thr Lys Glu Gly Leu Glu Gln Ala Leu Pro Ser Gly
                405                 410                 415

Lys Pro Ser Ser Ser Gly Glu Leu Asp Leu Phe Gly Asp Pro Ser Pro
            420                 425                 430

Ser Ser Lys Gln Asn Gly Thr Lys Glu Pro Asp Ala Leu Asp Leu Gly
        435                 440                 445

Ile Leu Gly Glu Ala Leu Thr Gln Pro Ser Lys Glu Ala Arg Ala Cys
    450                 455                 460

Arg Thr Pro Glu Ser Phe Leu Gly Pro Ser Ala Ser Ser Leu Val Asn
465                 470                 475                 480

Leu Asp Ser Leu Val Lys Ala Pro Gln Val Ala Lys Thr Arg Asn Pro
                485                 490                 495

Phe Leu Thr Gly Gly Leu Ser Ala Pro Ser Pro Thr Asn Pro Phe Gly
            500                 505                 510

Ala Gly Glu Pro Gly Arg Pro Thr Leu Asn Gln Met Arg Thr Gly Ser
        515                 520                 525

Pro Ala Leu Gly Leu Ala Gly Gly Pro Val Gly Ala Pro Leu Gly Ser
    530                 535                 540

Met Thr Tyr Ser Ala Ser Leu Pro Leu Pro Leu Ser Ser Val Pro Ala
545                 550                 555                 560

Gly Leu Thr Leu Pro Ala Ser Val Ser Val Phe Pro Gln Ala Gly Ala
                565                 570                 575

Phe Ala Pro Gln Pro Leu Leu Pro Thr Pro Ser Ser Ala Gly Pro Arg
            580                 585                 590

Pro Pro Pro Pro Gln Thr Gly Thr Asn Pro Phe Leu
        595                 600


<210> 13
<211> 2646
<212> DNA
<213> Homo sapiens

<400> 13
atggacaacc cacaggctct gccactcttc ctactcctgg cctccttggt agggatcctc 60
accctcagag cctcttctgg acttcagcaa accaacttct cctctgcctt ctcttcagac 120
tcaaagagct cttcccaggg gctgggtgtg gaagttccct ccatcaaacc tcccagctgg 180
aaagttccag atcagttcct ggattcaaaa gcctctgctg gaatctctga ttccagctgg 240
tttcctgagg ccctgagttc caacatgtct gggtccttct ggtcaaatgt ttctgctgag 300
ggccaagatt tgagcccggt ttcccccttc tctgaaaccc ctggttctga agtatttcct 360
gatatttcgg atcctcaagt tcctgccaaa gaccccaagc cttccttcac tgttaagacc 420
ccagcttcaa acatttctac tcaagtctcc cataccaaac tgtctgttga ggccccagat 480
tcaaaattct ccccggatga tatggatctt aaactctctg cccagagccc tgaatccaaa 540
ttttctgcag agacccactc agctgcaagc tttccccagc aggtgggggg cccactcgct 600
gtgctggtgg ggaccaccat ccggctcccc ctagtcccaa tccccaaccc tgggcccccc 660
acctctctgg tggtctggcg ccggggctca aaggtgctgg cagctggggg cctggggcca 720
ggggcacctc tgatcagcct ggaccctgct caccgagacc acctgcgatt tgaccaggcc 780
cggggggttc tggagctcgc ctctgcccag ctggacgatg caggggtcta cacggctgag 840
gtcatccggg caggggtctc ccagcagact cacgagttca cggtgggtgt gtatgagccc 900
ctaccccagc tgtcggttca gcccaaggct ccagagacag aggagggggc ggccgagctc 960
cggctgcgct gcctggggtg ggggccaggt cgcggggagc tgagctggag ccgggacgga 1020
cgcgccctgg aggcggcgga atcggaggga gccgagacgc cccggatgcg ctcagagggc 1080
gaccagctgc tcatcgtgcg ccctgtgcgc agcgaccacg cccggtacac ttgccgcgtc 1140
cgcagcccct tcggccacag ggaggctgcc gccgacgtca gcgtcttcta cggcccggac 1200
ccgccgacca tcacggtctc ctcggaccgc gacgccgcgc ctgcccgctt tgtcaccgcg 1260
ggcagtaacg tgaccttgcg ctgcgccgcc gcctcgcggc cgcccgccga catcacgtgg 1320

agcctggcgg acccggccga ggccgcggtg cccgcggggt cgcgcctcct gctgcccgcg 1380
gtcggaccgg gccacgcagg cacctacgcc tgcctggcgg cgaacccgcg taccggccgc 1440
cgccgccgct cgctgctcaa ccttacagtg gcggacctgc cccccggggc cccacagtgc 1500
tcagttgaag gggtcccgg ggaccgcagc ctccgcttcc gctgctcgtg gcccggcggg 1560
gcccctgctg cctccctgca gttccagggt ctccccgaag gcatccgcgc cgggccagtg 1620
tcctctgtgc tgctggcggc cgtccccgcc cacccccggc tcagcggcgt ccccatcacc 1680
tgccttgctc gccacctggt ggccacgcgt acctgcacag tcacgccgga ggccccccga 1740
gaggtgctgc tgcatccgct ggtggcagag acacggttgg gggaggcaga ggtggcactg 1800
gaggcctctg gttgtccccc accctcacgg gcatcctggg cccgggaagg gaggcccctg 1860
gctccaggag gcgggagtcg cctgcggctc agtcaagatg ggcggaaact ccacatcggc 1920
aacttcagcc tggattggga cctgggaaat tactccgtgc tgtgcagtgg ggcgctgggt 1980
gctggcggtg accagatcac cctcattgga ccctccatat cctcgtggag gcttcagaga 2040
gccagagatg cagccgtgct gacttgggat gtggagcgcg gggccctgat cagcagtttt 2100
gagatccagg catggccaga tgggcctgct ctgggcagga cttccaccta cagggactgg 2160
gtctccctgc tcatcctggg gcctcaggag cggtcagccg tggtgcccct tccacctcgg 2220
aacccaggga cctggacctt tcggatcctg cccatcctgg ggggccagcc agggactcca 2280
tcacaaagcc gggtctaccg ggccggcccc acgttgagcc atggggccat cgctggcatc 2340
gtcctgggct ccctgctggg cctggcgctg ctagccgtac ttctcctcct ttgcatctgc 2400
tgcctgtgcc gctttcgtgg aaagactcct gagaaaaaga agcatccttc taccttggtc 2460
cccgtggtca ccccctcaga aaagaagatg catagtgtga ccccagtgga gatttcatgg 2520
cctctggacc tcaaagtccc tctggaggac cacagctcaa ctagggccta ccaaaagaag 2580
agtcttcctg tatttgtgca agtaaggaga tgtgacttct tggctgggaa actcttgctg 2640
atctaa 2646

<210> 14
<211> 881
<212> PRT
<213> Homo sapiens

<400> 14
Met Asp Asn Pro Gln Ala Leu Pro Leu Phe Leu Leu Leu Ala Ser Leu
1               5                   10                  15

Val Gly Ile Leu Thr Leu Arg Ala Ser Ser Gly Leu Gln Gln Thr Asn
            20                  25                  30

Phe Ser Ser Ala Phe Ser Ser Asp Ser Lys Ser Ser Ser Gln Gly Leu
        35                  40                  45

Gly Val Glu Val Pro Ser Ile Lys Pro Pro Ser Trp Lys Val Pro Asp
    50                  55                  60

Gln Phe Leu Asp Ser Lys Ala Ser Ala Gly Ile Ser Asp Ser Ser Trp
65                  70                  75                  80

Phe Pro Glu Ala Leu Ser Ser Asn Met Ser Gly Ser Phe Trp Ser Asn
                85                  90                  95

Val Ser Ala Glu Gly Gln Asp Leu Ser Pro Val Ser Pro Phe Ser Glu
            100                 105                 110

Thr Pro Gly Ser Glu Val Phe Pro Asp Ile Ser Asp Pro Gln Val Pro
        115                 120                 125

Ala Lys Asp Pro Lys Pro Ser Phe Thr Val Lys Thr Pro Ala Ser Asn
    130                 135                 140

Ile Ser Thr Gln Val Ser His Thr Lys Leu Ser Val Glu Ala Pro Asp
145                 150                 155                 160

Ser Lys Phe Ser Pro Asp Asp Met Asp Leu Lys Leu Ser Ala Gln Ser
                165                 170                 175

Pro Glu Ser Lys Phe Ser Ala Glu Thr His Ser Ala Ala Ser Phe Pro
            180                 185                 190

Gln Gln Val Gly Gly Pro Leu Ala Val Leu Val Gly Thr Thr Ile Arg
        195                 200                 205

Leu Pro Leu Val Pro Ile Pro Asn Pro Gly Pro Pro Thr Ser Leu Val
    210                 215                 220

Val Trp Arg Arg Gly Ser Lys Val Leu Ala Ala Gly Gly Leu Gly Pro
225                 230                 235                 240

Gly Ala Pro Leu Ile Ser Leu Asp Pro Ala His Arg Asp His Leu Arg
                245                 250                 255

Phe Asp Gln Ala Arg Gly Val Leu Glu Leu Ala Ser Ala Gln Leu Asp
            260                 265                 270

Asp Ala Gly Val Tyr Thr Ala Glu Val Ile Arg Ala Gly Val Ser Gln
        275                 280                 285

Gln Thr His Glu Phe Thr Val Gly Val Tyr Glu Pro Leu Pro Gln Leu
    290                 295                 300

Ser Val Gln Pro Lys Ala Pro Glu Thr Glu Glu Gly Ala Ala Glu Leu
305                 310                 315                 320

Arg Leu Arg Cys Leu Gly Trp Gly Pro Gly Arg Gly Glu Leu Ser Trp
                325                 330                 335

Ser Arg Asp Gly Arg Ala Leu Glu Ala Ala Glu Ser Glu Gly Ala Glu
            340                 345                 350

Thr Pro Arg Met Arg Ser Glu Gly Asp Gln Leu Leu Ile Val Arg Pro
        355                 360                 365

Val Arg Ser Asp His Ala Arg Tyr Thr Cys Arg Val Arg Ser Pro Phe
    370                 375                 380

Gly His Arg Glu Ala Ala Ala Asp Val Ser Val Phe Tyr Gly Pro Asp
385                 390                 395                 400

Pro Pro Thr Ile Thr Val Ser Ser Asp Arg Asp Ala Ala Pro Ala Arg
                405                 410                 415

Phe Val Thr Ala Gly Ser Asn Val Thr Leu Arg Cys Ala Ala Ala Ser
            420                 425                 430

Arg Pro Pro Ala Asp Ile Thr Trp Ser Leu Ala Asp Pro Ala Glu Ala
        435                 440                 445

Ala Val Pro Ala Gly Ser Arg Leu Leu Leu Pro Ala Val Gly Pro Gly
    450                 455                 460

His Ala Gly Thr Tyr Ala Cys Leu Ala Ala Asn Pro Arg Thr Gly Arg
465                 470                 475                 480

Arg Arg Arg Ser Leu Leu Asn Leu Thr Val Ala Asp Leu Pro Pro Gly
                485                 490                 495

Ala Pro Gln Cys Ser Val Glu Gly Gly Pro Gly Asp Arg Ser Leu Arg
            500                 505                 510

Phe Arg Cys Ser Trp Pro Gly Gly Ala Pro Ala Ala Ser Leu Gln Phe
        515                 520                 525

Gln Gly Leu Pro Glu Gly Ile Arg Ala Gly Pro Val Ser Ser Val Leu
    530                 535                 540

Leu Ala Ala Val Pro Ala His Pro Arg Leu Ser Gly Val Pro Ile Thr
545                 550                 555                 560

Cys Leu Ala Arg His Leu Val Ala Thr Arg Thr Cys Thr Val Thr Pro
                565                 570                 575

Glu Ala Pro Arg Glu Val Leu Leu His Pro Leu Val Ala Glu Thr Arg
            580                 585                 590

Leu Gly Glu Ala Glu Val Ala Leu Glu Ala Ser Gly Cys Pro Pro Pro
        595                 600                 605

Ser Arg Ala Ser Trp Ala Arg Glu Gly Arg Pro Leu Ala Pro Gly Gly
    610                 615                 620

Gly Ser Arg Leu Arg Leu Ser Gln Asp Gly Arg Lys Leu His Ile Gly
625                 630                 635                 640

Asn Phe Ser Leu Asp Trp Asp Leu Gly Asn Tyr Ser Val Leu Cys Ser
                645                 650                 655

Gly Ala Leu Gly Ala Gly Gly Asp Gln Ile Thr Leu Ile Gly Pro Ser
            660                 665                 670

Ile Ser Ser Trp Arg Leu Gln Arg Ala Arg Asp Ala Ala Val Leu Thr
        675                 680                 685

Trp Asp Val Glu Arg Gly Ala Leu Ile Ser Ser Phe Glu Ile Gln Ala
    690                 695                 700

Trp Pro Asp Gly Pro Ala Leu Gly Arg Thr Ser Thr Tyr Arg Asp Trp
705                 710                 715                 720

Val Ser Leu Leu Ile Leu Gly Pro Gln Glu Arg Ser Ala Val Val Pro
                725                 730                 735

Leu Pro Pro Arg Asn Pro Gly Thr Trp Thr Phe Arg Ile Leu Pro Ile
            740                 745                 750

Leu Gly Gly Gln Pro Gly Thr Pro Ser Gln Ser Arg Val Tyr Arg Ala
        755                 760                 765

Gly Pro Thr Leu Ser His Gly Ala Ile Ala Gly Ile Val Leu Gly Ser
    770                 775                 780

Leu Leu Gly Leu Ala Leu Leu Ala Val Leu Leu Leu Leu Cys Ile Cys
785                 790                 795                 800

Cys Leu Cys Arg Phe Arg Gly Lys Thr Pro Glu Lys Lys Lys His Pro
                805                 810                 815

Ser Thr Leu Val Pro Val Val Thr Pro Ser Glu Lys Lys Met His Ser
            820                 825                 830

Val Thr Pro Val Glu Ile Ser Trp Pro Leu Asp Leu Lys Val Pro Leu
        835                 840                 845

Glu Asp His Ser Ser Thr Arg Ala Tyr Gln Lys Lys Ser Leu Pro Val
    850                 855                 860

Phe Val Gln Val Arg Arg Cys Asp Phe Leu Ala Gly Lys Leu Leu Leu
865                 870                 875                 880

Ile


<210> 15
<211> 8589
<212> DNA
<213> Homo sapiens

<400> 15
tgcaggaggc ggcgatggcc ctgggcaagg ttctggccat ggcactggtt ttggccttgg 60

ccgtgctggg gtcgctgtcc cctggggccc gggcggggga ctgcaagggg cagcggcagg 120
tgctgcggga ggcgccaggc ttcgtgacgg atggtgcggg caactacagc gtcaatggca 180
actgcgagtg gctcatcgag gccccaagcc cccagcaccg gatcctgctg gacttccttt 240
tcctggacac agagtgcacg tatgactacc tgttcgtgta tgacggtgac tccccgcgag 300
ggccgctgct tgccagtcta agtgggagca cccgacctcc gcccatcgaa gcttcctcag 360
gcaagatgct gctgcacctc ttcagtgatg ccaactacaa cctgctgggc tttaacgcct 420
cattccgctt ctccctgtgc ccgggtggct gccagagcca cgggcagtgc cagccaccgg 480
gtgtgtgtgc ctgcgagccg ggctgggggg gtcctgactg tggcctgcag gagtgctcag 540
cctactgtgg cagccacggc acctgcgcct cgcccctggg accatgccgc tgtgagcctg 600
gcttcttggg acgtgcctgt gacctgcacc tgtgggagaa ccagggggct gggtggtggc 660
acaacgtgag tgccagggac cctgccttct ctgcccgtat tggggcagct ggcgccttcc 720
tgtccccacc agggctgctg gcagttttcg gaggccagga cctcaacaat gccctgggtg 780
acctcgtcct atacaacttc tccgccaaca cctgggagtc ttgggacctg agtcctgccc 840
cggctgcccg tcactcccat gtggccgtgg cctgggccgg ctccctggta ctgatgggtg 900
gtgagctggc tgacggctcg ctcaccaacg acgtgtgggc cttcagtcca ctgggcaggg 960
gccactggga gctcctggca ccacctgcct ccagctcctc ggggccccca ggcctggcag 1020
gtcacgcggc tgccctggtg gatgatgtct ggctatatgt gtctggaggc cgcaccccgc 1080
acgacctctt ctcctctggc ctcttccgtt tccgccttga cagcaccagc gggggctatt 1140
gggagcaggt gattccggca ggcggacggc cccctgctgc cactggccac tccatggtgt 1200
tccatgcccc ctcccgtgcc ctgctggtcc atggtggaca ccggccctcc actgcccggt 1260
tctctgtgcg agtgaactcc actgagcttt tccacgtgga tcggcatgtg tggacgacgc 1320
tgaaggggcg ggatggcttt cagggcccaa gggagcgagc cttccacaca gccagtgttc 1380
tgggcaatta catggtggtc tatgggggca atgtgcacac ccattaccag gaggaaaagt 1440
gctacgaaga tggcatcttc ttctaccacc ttggctgcca tcaatgggtg tcaggagctg 1500
agcttgcccc gccaggaacc cctgagggcc gagcagcgcc tcccagtggt cggtactcac 1560
atgtagctgc ggtgcttggt ggcagcgtcc tgttggtggc tgggggggtac agcggccggc 1620
cccgtgggga cttgatggcg tacaaggtgc ccccctttgt gttccaggca cctgcccctg 1680
actaccactt ggactactgc tccatgtaca cagaccacag cgtctgctcc cgggacccgg 1740
aatgcagttg gtgccaagga gcctgccaag ctgcaccccc tcctgggacc cccttggggg 1800
cttgtccagc cgccagctgc ctgggcctgg gccgcctcct gggtgactgc caggcctgcc 1860
tggccttcag cagccccaca gcccctccac ggggacctgg caccctgggc tggtgcgtgc 1920
acaatgagag ctgcctccct aggcctgagc aggcccgctg ccgaggggag cagatctcag 1980
gcactgtggg ctggtggggg cctgcgcctg tcttcgtcac gtccctggag gcctgcgtca 2040
cccagagctt cctgcctggc ctgcacttgc tcacctttca gcagccgccc aatacctccc 2100
agcctgacaa ggtctcaatt gtccgcagca cgaccatcac cctaacaccc agcgcagaga 2160
cagatgtgtc cctggtctac cgtggcttca tctacccaat gctgcctgga gggccaggtg 2220
gaccaggggc tgaggacgtg gccgtgtgga cgcgggccca gcgcctacac gtcctggccc 2280
ggatggcccg tggccctgac acggagaaca tggaggaggt ggggcgctgg gtggctcatc 2340
aggagaagga gacgcggcgg ctgcagcgcc ctgggtctgc tcgcctcttc cctctgcctg 2400
ggcgggacca caagtatgca gtagagatcc agggccagct caatggctcg gcaggccctg 2460
ggcacagcga gctaactctg ctgtgggatc ggactggtgt gccaggaggc agcgagatct 2520
ccttcttctt cctggagccc taccgctcgt cgtcctgcac ctcctattct tcctgcctgg 2580
gctgcttggc agaccagggc tgtggctggt gcctgaccag tgccacctgc cacctgcgcc 2640
agggcggagc ccattgcggg gatgacgggg ctggtgggtc cctgctggtg ctggtgccta 2700
ccctctgccc actctgcgag gagcatcggg actgccacgc ctgcacccag gaccccttct 2760
gtgagtggca tcagagcacc agccgcaaag gggacgcggc atgcagccgg cggggccggg 2820
gtcggggtgc cctgaagagt ccagaggagt gtcccccgct ctgcagccag cgactgacct 2880
gtgaggactg cctggccaac tctagccagt gcgcctggtg ccagtccacc cacacctgct 2940
tcctgtttgc tgcctacttg gcccggtacc cacacggggg ctgtcgaggc tgggacgaca 3000
gtgtacactc ggagccacgg tgccggagct gcgatggctt cctgacctgc catgagtgtc 3060
tgcagagcca cgagtgtggc tggtgtggca atgaggacaa ccccacactg ggacggtgcc 3120
tacaggggga cttctcaggg cccctcggtg ggggtaactg ctccctgtgg gtgggggagg 3180
gcctggggct tcccgtggcc ctccctgccc gctgggcata cgcccgctgt cctgacgtgg 3240
atgagtgtcg cctgggcctg gcccggtgcc acccgcgggc gacctgcctg aacacgcccc 3300
tcagctacga gtgtcactgc cagcggggct accagggtga tggcatctca cactgcaacc 3360
gcacggatgg catctcacac tgcaaccgca cgtgcttgga ggactgtggc catggtgtgt 3420
gcagtggccc cccggacttt acctgcgtgt gtgacctagg ctggacatca gacctgcccc 3480
ctcccacacc cgccccgggt ccgccagccc cccgctgctc ccgggactgt ggctgcagct 3540
tccacagcca ctgccgcaag cggggccctg gcttctgcga cgagtgccag gactggacat 3600
gggggagca ctgcgaacga tgccggcccg gcagcttcgg caacgccaca ggctctaggg 3660
gctgccggcc ctgccagtgc aacgggcacg gggacccacg ccgtggccac tgcgacaacc 3720
tcagtgggct ctgcttctgc caggaccaca ccgagggtgc ccactgccag ctctgctccc 3780
caggctatta tggggatccc cgggccggtg gttcctgctt tcgggagtgt gggggtcgcg 3840
ccctcctcac caacgtgtcc tcagtggcac tgggctcacg ccgggtcggg gggctgctgc 3900
ctccaggtgg cggggctgca agagccgggc ctggcctgtc ctactgtgtg tgggttgtct 3960
cggccactga ggagctacag ccctgtgctc ccgggaccct ctgtccccca ctcaccctca 4020
ccttctcccc cgacagcagc acccccctgca cgctgagcta cgtcctggcg tttgatggat 4080
tcccacgctt cctggacact ggtgttgtcc agtcggaccg cagcctcata gctgccttct 4140
gcggccagcg acgggacagg cccctcactg ttcaggccct gtctgggctg ctcgtgctgc 4200

actgggaggc caatggctcc tcatcctggg gcttcaatgc ttcggtgggc tctgcccgct 4260
gtgggtcagg gggcccggg agctgtcccg tcccccagga atgcgtgccc caggacggtg 4320
ctgcaggtgc ggggctctgc cgatgtcctc agggctgggc tggcccacac tgccgcatgg 4380
ctctgtgtcc tgagaactgc aatgcccaca ctggggcagg aacttgtaac cagagcctgg 4440
gtgtgtgcat ctgtgccgag ggcttcgggg gccccgactg cgccaccaag ctggatggcg 4500
ggcagctggt ctgggagacc ctcatggaca gccgcctctc agccgacact gccagccgct 4560
tcctgcaccg cctgggccac accatggtgg atggacccga tgccaccttg tggatgtttg 4620
gggcctgggg cctgccccag gggctgctgg gaaacctgta caggtactca gtgagtgagc 4680
ggcggtggac acagatgctg gcgggagccg aggacggggg cccaggccca tcgccccgct 4740
ccttccatgc agccgcatat gtgcccgctg gccgtggtgc catgtatctg ctggggggac 4800
ttaccgctgg aggcgtcacc cgtgatttct gggtcctcaa cctcaccacc ctgcaatggc 4860
ggcaggagaa ggcccccccag accgtggagc tgccagccgt tgctggtcac acccttactg 4920
cccgccgagg cctgtctctg ctcctggtgg gcggttactc cccggaaaat ggcttcaacc 4980
agcagctgct ggagtaccag ctggcaaccg gcacctgggt gtcaggagcc cagagtggga 5040
caccccccac aggtctctat ggtcactctg ctgtctacca cgaggccacc gactccctct 5100
acgtgtttgg ggggttccga ttccatgtgg agctggcggc cccatccccc gagctctact 5160
ccctgcactg tcctgaccgc acctggagtc tgctggcccc ttctcagggg gcaaagcgag 5220
atcgtatgag gaatgtgcgt ggctcatctc ggggtctggg ccaagttcct ggggagcagc 5280
ctgggtcatg gggggttccgg gaagtcagga agaagatggc tctgtgggct gctcttgctg 5340
gtacaggagg tttcctggag gaaatctcac ctcacctgaa ggagccccgc ccccggcttt 5400
tccacgcctc agccctgtta ggggacacca tggtggttct tgggggggcgc tcggaccctg 5460
acgagttcag cagcgacgtt ctgctctacc aggtcaactg caatgcctgg cttctgcccg 5520
acctcacccg ctcggcctct gtggggcccc caatggagga gtctgtggcc catgctgtgg 5580
cagcagtcgg gagccgcctg tatatctctg ggggtttcgg gggagtggcc ctgggccgcc 5640
tgctggcact gaccctgccc cctgacccct gccgcctgct gtcctcacct gaagcttgta 5700
accagtctgg ggcctgcacc tggtgccatg ggcctgctt gtccggggat caggcccaca 5760
ggctgggctg cgggggctcc ccctgctccc caatgcctcg ctccccggag gaatgtcgac 5820
gtctccggac ctgcagtgag tgcctggccc gccatcctcg gaccctgcaa cctggagatg 5880
gagaggcgtc caccccccgc tgtaagtggt gtaccaactg ccccgaaggt gcttgcattg 5940
gacgcaatgg gtcctgcacc tctgagaatg actgtcggat caaccagcga gaggtcttct 6000
gggcagggaa ctgctccgag gctgcgtgcg gggctgctga ctgcgagcag tgcacgcggg 6060
agggcaagtg catgtggacg cggcagttca agaggacagg ggagacccgc cgcatcctct 6120
ccgtgcagcc cacctatgac tggacgtgct tcagccactc tctgctgaat gtgtccccca 6180
tgccggtgga atcatcaccc ccactgccct gccccacccc ttgtcacctc ctacccaact 6240
gtacctcctg cctggactct aagggagcag atgggggctg gcagcactgt gtttggagca 6300
gcagcctgca gcagtgtctg agcccttcct acctgcccct gcgatgtatg gccggaggct 6360
gtgggcggct gctccgggga cctgagagct gctccctggg ctgtgctcag gcaactcagt 6420
gcgccttgtg cctgcggcgc ccccattgcg gctggtgtgc ctgggggggc caggatgggg 6480
gtggccgctg catggagggt ggactcagcg gcccccgtga tgggctgaca tgtgggcgtc 6540
cgggggcctc ctgggccttc ctgtcctgcc cccctgagga cgagtgtgca aacgggcacc 6600
acgactgcaa cgagacgcag aattgccacg accagcccca cggctatgag tgcagctgca 6660
agaccggcta taccatggac aacatgacag ggctgtgccg ccctgtgtgc gcccagggct 6720
gcgtgaacgg ctcatgtgtg gagcccgacc actgccgctg ccactttggc tttgtgggcc 6780
gcaactgctc cacggaatgc cgctgcaacc gccacagtga atgcgctggt gttggggcgc 6840
gtgaccactg cttgctctgc cgcaaccaca ccaagggcag ccactgtgag cagtgcctcc 6900
cgctgtttgt gggttcagct gtcggaggcg ggacctgccg gccctgccac gccttttgtc 6960
gtggaaatag ccacatctgc atctccagga aggagttaca aatgtccaag ggagagccaa 7020
agaagtactc actggaccca gaggagattg aaaactgggt gacagagggt cctagtgaag 7080
acgaggccgt gtgcgtgaac tgccagaata acagctatgg ggagaaatgc gagagctgcc 7140
tgcagggcta cttcctcctg gacgggaagt gcaccaaatg ccagtgtaat ggccacgcgg 7200
acacatgtaa cgagcaggat gggacgggct gtccatgtca gaataacaca gagacgggca 7260
catgccaggg cagctccccc agtgaccgtc gagactgcta caagtaccag tgcgccaagt 7320
gccgggaatc atttcacggg agtccgctgg gcggccagca gtgctaccgc ctcatctcgg 7380
tggagcagga gtgctgcctg gaccccacgt cccagaccaa ctgcttccat gagcccaaac 7440
gccgggcgct aggccccggc cgcactgtcc tctttggcgt gcagcccaaa ttcaccaacg 7500
tggacatccg cctgacgctg gacgtgacct tcggggccgt ggacctctat gtctccacct 7560
cctatgacac cttcgtggtc cgtgtggccc ctgacactgg cgtccatact gtacacatcc 7620
agccaccccc agccccacca cctccaccac cccctgcaga tggtgggccc cgggggggctg 7680
ggatccagg aggagcaggg gccagcagtg ggccgggcgc cccagcagag ccacgggtac 7740
gggaggtatg gccgcggggc ctgattacct acgtgacggt gacggagccg tcggcagtgc 7800
tggtggtccg cggcgtgcgg gaccggctgg tcatcaccta cccacacgag caccatgccc 7860
tcaagtcgag ccgcttctac ctgctgctgc tgggcgtggg agacccaagt gggcccggcg 7920
ccaacggctc agccgactcg cagggcctgc tcttcttccg gcaggaccag gcccacattg 7980
acctgtttgt cttcttctcc gtcttcttct cctgcttctt cctcttcctc tcactctgtg 8040
tgctcctctg gaaggccaag caggctctgg accagcggca ggagcagcgc cggcacttgc 8100
aggagatgac caagatggcc agccgcccct tcgccaaggt caccgtctgc ttcccacctg 8160
accctactgc cccggcctcc gcctggaagc cggctgggct cccacctccc gccttccgcc 8220
gctctgagcc cttcctggca cccctgctgc tgacaggggc cggtgggccc tggggaccca 8280
tgggaggggg ctgctgccca ccagccatcc ccgccaccac tgctgggctg cgagctgggc 8340

ccatcactct cgagcccaca gaagatggca tggctggcgt ggccacactg ctgctccagc 8400
tgcctggcgg gccccatgca cccaacggcg cctgcctggg gtcagccctc gtcacactgc 8460
ggcacaggct gcacgagtac tgtgggggtg gtgggggtgc tgggggcagt gggcatggga 8520
ctggtgcggg ccggaaggga ctgttgagcc aggacaacct caccagcatg tccctctgac 8580
atgcccagg 8589

<210> 16
<211> 2854
<212> PRT
<213> Homo sapiens

<400> 16
   Met Ala Leu Gly Lys Val Leu Ala Met Ala Leu Val Leu Ala Leu Ala
   1               5                   10                  15
   Val Leu Gly Ser Leu Ser Pro Gly Ala Arg Ala Gly Asp Cys Lys Gly
               20                  25                  30
   Gln Arg Gln Val Leu Arg Glu Ala Pro Gly Phe Val Thr Asp Gly Ala
           35                  40                  45
   Gly Asn Tyr Ser Val Asn Gly Asn Cys Glu Trp Leu Ile Glu Ala Pro
       50                  55                  60
   Ser Pro Gln His Arg Ile Leu Leu Asp Phe Leu Phe Leu Asp Thr Glu
   65                  70                  75                  80
   Cys Thr Tyr Asp Tyr Leu Phe Val Tyr Asp Gly Asp Ser Pro Arg Gly
                   85                  90                  95
   Pro Leu Leu Ala Ser Leu Ser Gly Ser Thr Arg Pro Pro Pro Ile Glu
               100                 105                 110
   Ala Ser Ser Gly Lys Met Leu Leu His Leu Phe Ser Asp Ala Asn Tyr
           115                 120                 125
   Asn Leu Leu Gly Phe Asn Ala Ser Phe Arg Phe Ser Leu Cys Pro Gly
       130                 135                 140
   Gly Cys Gln Ser His Gly Gln Cys Gln Pro Pro Gly Val Cys Ala Cys
   145                 150                 155                 160
   Glu Pro Gly Trp Gly Gly Pro Asp Cys Gly Leu Gln Glu Cys Ser Ala
                   165                 170                 175
   Tyr Cys Gly Ser His Gly Thr Cys Ala Ser Pro Leu Gly Pro Cys Arg
               180                 185                 190
   Cys Glu Pro Gly Phe Leu Gly Arg Ala Cys Asp Leu His Leu Trp Glu
           195                 200                 205
   Asn Gln Gly Ala Gly Trp Trp His Asn Val Ser Ala Arg Asp Pro Ala
       210                 215                 220
   Phe Ser Ala Arg Ile Gly Ala Ala Gly Ala Phe Leu Ser Pro Pro Gly
   225                 230                 235                 240
   Leu Leu Ala Val Phe Gly Gly Gln Asp Leu Asn Asn Ala Leu Gly Asp
                   245                 250                 255
   Leu Val Leu Tyr Asn Phe Ser Ala Asn Thr Trp Glu Ser Trp Asp Leu
               260                 265                 270
   Ser Pro Ala Pro Ala Ala Arg His Ser His Val Ala Val Ala Trp Ala
           275                 280                 285
   Gly Ser Leu Val Leu Met Gly Gly Glu Leu Ala Asp Gly Ser Leu Thr
       290                 295                 300
   Asn Asp Val Trp Ala Phe Ser Pro Leu Gly Arg Gly His Trp Glu Leu
   305                 310                 315                 320
   Leu Ala Pro Pro Ala Ser Ser Ser Ser Gly Pro Pro Gly Leu Ala Gly
                   325                 330                 335
   His Ala Ala Ala Leu Val Asp Asp Val Trp Leu Tyr Val Ser Gly Gly
               340                 345                 350
   Arg Thr Pro His Asp Leu Phe Ser Ser Gly Leu Phe Arg Phe Arg Leu
           355                 360                 365
   Asp Ser Thr Ser Gly Gly Tyr Trp Glu Gln Val Ile Pro Ala Gly Gly
       370                 375                 380
   Arg Pro Pro Ala Ala Thr Gly His Ser Met Val Phe His Ala Pro Ser
   385                 390                 395                 400
   Arg Ala Leu Leu Val His Gly Gly His Arg Pro Ser Thr Ala Arg Phe
                   405                 410                 415
   Ser Val Arg Val Asn Ser Thr Glu Leu Phe His Val Asp Arg His Val
               420                 425                 430
   Trp Thr Thr Leu Lys Gly Arg Asp Gly Leu Gln Gly Pro Arg Glu Arg
           435                 440                 445
   Ala Phe His Thr Ala Ser Val Leu Gly Asn Tyr Met Val Val Tyr Gly
       450                 455                 460
   Gly Asn Val His Thr His Tyr Gln Glu Glu Lys Cys Tyr Glu Asp Gly
   465                 470                 475                 480
   Ile Phe Phe Tyr His Leu Gly Cys His Gln Trp Val Ser Gly Ala Glu
                   485                 490                 495
   Leu Ala Pro Pro Gly Thr Pro Glu Gly Arg Ala Ala Pro Pro Ser Gly
               500                 505                 510
   Arg Tyr Ser His Val Ala Ala Val Leu Gly Gly Ser Val Leu Leu Val
           515                 520                 525
   Ala Gly Gly Tyr Ser Gly Arg Pro Arg Gly Asp Leu Met Ala Tyr Lys
       530                 535                 540
   Val Pro Pro Phe Val Phe Gln Ala Pro Ala Pro Asp Tyr His Leu Asp
   545                 550                 555                 560
   Tyr Cys Ser Met Tyr Thr Asp His Ser Val Cys Ser Arg Asp Pro Glu
                   565                 570                 575
   Cys Ser Trp Cys Gln Gly Ala Cys Gln Ala Ala Pro Pro Pro Gly Thr
               580                 585                 590
   Pro Leu Gly Ala Cys Pro Ala Ala Ser Cys Leu Gly Leu Gly Arg Leu
           595                 600                 605
   Leu Gly Asp Cys Gln Ala Cys Leu Ala Phe Ser Ser Pro Thr Ala Pro
       610                 615                 620
   Pro Arg Gly Pro Gly Thr Leu Gly Trp Cys Val His Asn Glu Ser Cys
   625                 630                 635                 640
   Leu Pro Arg Pro Glu Gln Ala Arg Cys Arg Gly Glu Gln Ile Ser Gly
                   645                 650                 655
   Thr Val Gly Trp Trp Gly Pro Ala Pro Val Phe Val Thr Ser Leu Glu
               660                 665                 670
   Ala Cys Val Thr Gln Ser Phe Leu Pro Gly Leu His Leu Leu Thr Phe
           675                 680                 685
   Gln Gln Pro Pro Asn Thr Ser Gln Pro Asp Lys Val Ser Ile Val Arg
       690                 695                 700
   Ser Thr Thr Ile Thr Leu Thr Pro Ser Ala Glu Thr Asp Val Ser Leu
   705                 710                 715                 720
   Val Tyr Arg Gly Phe Ile Tyr Pro Met Leu Pro Gly Gly Pro Gly Gly
                   725                 730                 735
   Pro Gly Ala Glu Asp Val Ala Val Trp Thr Arg Ala Gln Arg Leu His
               740                 745                 750
   Val Leu Ala Arg Met Ala Arg Gly Pro Asp Thr Glu Asn Met Glu Glu
           755                 760                 765
   Val Gly Arg Trp Val Ala His Gln Glu Lys Glu Thr Arg Arg Leu Gln
       770                 775                 780
   Arg Pro Gly Ser Ala Arg Leu Phe Pro Leu Pro Gly Arg Asp His Lys
   785                 790                 795                 800
   Tyr Ala Val Glu Ile Gln Gly Gln Leu Asn Gly Ser Ala Gly Pro Gly
                   805                 810                 815
   His Ser Glu Leu Thr Leu Leu Trp Asp Arg Thr Gly Val Pro Gly Gly
               820                 825                 830
   Ser Glu Ile Ser Phe Phe Phe Leu Glu Pro Tyr Arg Ser Ser Ser Cys
           835                 840                 845
   Thr Ser Tyr Ser Ser Cys Leu Gly Cys Leu Ala Asp Gln Gly Cys Gly
       850                 855                 860
   Trp Cys Leu Thr Ser Ala Thr Cys His Leu Arg Gln Gly Gly Ala His
   865                 870                 875                 880
   Cys Gly Asp Asp Gly Ala Gly Gly Ser Leu Leu Val Leu Val Pro Thr
                   885                 890                 895
   Leu Cys Pro Leu Cys Glu Glu His Arg Asp Cys His Ala Cys Thr Gln
               900                 905                 910
   Asp Pro Phe Cys Glu Trp His Gln Ser Thr Ser Arg Lys Gly Asp Ala
           915                 920                 925
   Ala Cys Ser Arg Arg Gly Arg Gly Arg Gly Ala Leu Lys Ser Pro Glu
       930                 935                 940
   Glu Cys Pro Pro Leu Cys Ser Gln Arg Leu Thr Cys Glu Asp Cys Leu
   945                 950                 955                 960
   Ala Asn Ser Ser Gln Cys Ala Trp Cys Gln Ser Thr His Thr Cys Phe
                   965                 970                 975
   Leu Phe Ala Ala Tyr Leu Ala Arg Tyr Pro His Gly Gly Cys Arg Gly
               980                 985                 990
   Trp Asp Asp Ser Val His Ser Glu Pro Arg Cys Arg Ser Cys Asp Gly
           995                 1000                1005
   Phe Leu Thr Cys His Glu Cys Leu Gln Ser His Glu Cys Gly Trp Cys
       1010                1015                1020
   Gly Asn Glu Asp Asn Pro Thr Leu Gly Arg Cys Leu Gln Gly Asp Phe
   1025                1030                1035                1040
   Ser Gly Pro Leu Gly Gly Gly Asn Cys Ser Leu Trp Val Gly Glu Gly
                   1045                1050                1055
   Leu Gly Leu Pro Val Ala Leu Pro Ala Arg Trp Ala Tyr Ala Arg Cys
               1060                1065                1070
   Pro Asp Val Asp Glu Cys Arg Leu Gly Leu Ala Arg Cys His Pro Arg
           1075                1080                1085
   Ala Thr Cys Leu Asn Thr Pro Leu Ser Tyr Glu Cys His Cys Gln Arg
       1090                1095                1100
   Gly Tyr Gln Gly Asp Gly Ile Ser His Cys Asn Arg Thr Asp Gly Ile
   1105                1110                1115                1120
   Ser His Cys Asn Arg Thr Cys Leu Glu Asp Cys Gly His Gly Val Cys
                   1125                1130                1135
   Ser Gly Pro Pro Asp Phe Thr Cys Val Cys Asp Leu Gly Trp Thr Ser
               1140                1145                1150
   Asp Leu Pro Pro Pro Thr Pro Ala Pro Gly Pro Pro Ala Pro Arg Cys
           1155                1160                1165
   Ser Arg Asp Cys Gly Cys Ser Phe His Ser His Cys Arg Lys Arg Gly
       1170                1175                1180
   Pro Gly Phe Cys Asp Glu Cys Gln Asp Trp Thr Trp Gly Glu His Cys
   1185                1190                1195                1200
   Glu Arg Cys Arg Pro Gly Ser Phe Gly Asn Ala Thr Gly Ser Arg Gly
                   1205                1210                1215
   Cys Arg Pro Cys Gln Cys Asn Gly His Gly Asp Pro Arg Arg Gly His
               1220                1225                1230
   Cys Asp Asn Leu Ser Gly Leu Cys Phe Cys Gln Asp His Thr Glu Gly
           1235                1240                1245
   Ala His Cys Gln Leu Cys Ser Pro Gly Tyr Tyr Gly Asp Pro Arg Ala
       1250                1255                1260
   Gly Gly Ser Cys Phe Arg Glu Cys Gly Gly Arg Ala Leu Leu Thr Asn
   1265                1270                1275                1280
   Val Ser Ser Val Ala Leu Gly Ser Arg Arg Val Gly Gly Leu Leu Pro
                   1285                1290                1295
   Pro Gly Gly Gly Ala Ala Arg Ala Gly Pro Gly Leu Ser Tyr Cys Val
               1300                1305                1310
   Trp Val Val Ser Ala Thr Glu Glu Leu Gln Pro Cys Ala Pro Gly Thr
           1315                1320                1325
   Leu Cys Pro Pro Leu Thr Leu Thr Phe Ser Pro Asp Ser Ser Thr Pro
       1330                1335                1340
   Cys Thr Leu Ser Tyr Val Leu Ala Phe Asp Gly Phe Pro Arg Phe Leu
   1345                1350                1355                1360
   Asp Thr Gly Val Val Gln Ser Asp Arg Ser Leu Ile Ala Ala Phe Cys
                   1365                1370                1375
   Gly Gln Arg Arg Asp Arg Pro Leu Thr Val Gln Ala Leu Ser Gly Leu
               1380                1385                1390
   Leu Val Leu His Trp Glu Ala Asn Gly Ser Ser Ser Trp Gly Phe Asn
           1395                1400                1405
   Ala Ser Val Gly Ser Ala Arg Cys Gly Ser Gly Gly Pro Gly Ser Cys
   1410 1415 1420
   Pro Val Pro Gln Glu Cys Val Pro Gln Asp Gly Ala Ala Gly Ala Gly
   1425 1430 1435 1440
   Leu Cys Arg Cys Pro Gln Gly Trp Ala Gly Pro His Cys Arg Met Ala
   1445 1450 1455
   Leu Cys Pro Glu Asn Cys Asn Ala His Thr Gly Ala Gly Thr Cys Asn
   1460 1465 1470
   Gln Ser Leu Gly Val Cys Ile Cys Ala Glu Gly Phe Gly Gly Pro Asp
   1475 1480 1485
   Cys Ala Thr Lys Leu Asp Gly Gly Gln Leu Val Trp Glu Thr Leu Met
   1490 1495 1500
   Asp Ser Arg Leu Ser Ala Asp Thr Ala Ser Arg Phe Leu His Arg Leu
   1505 1510 1515 1520
   Gly His Thr Met Val Asp Gly Pro Asp Ala Thr Leu Trp Met Phe Gly
   1525 1530 1535
   Gly Leu Gly Leu Pro Gln Gly Leu Leu Gly Asn Leu Tyr Arg Tyr Ser
   1540 1545 1550
   Val Ser Glu Arg Arg Trp Thr Gln Met Leu Ala Gly Ala Glu Asp Gly
   1555 1560 1565
   Gly Pro Gly Pro Ser Pro Arg Ser Phe His Ala Ala Ala Tyr Val Pro
   1570 1575 1580
   Ala Gly Arg Gly Ala Met Tyr Leu Leu Gly Gly Leu Thr Ala Gly Gly
   1585 1590 1595 1600
   Val Thr Arg Asp Phe Trp Val Leu Asn Leu Thr Thr Leu Gln Trp Arg
   1605 1610 1615
   Gln Glu Lys Ala Pro Gln Thr Val Glu Leu Pro Ala Val Ala Gly His
   1620 1625 1630
   Thr Leu Thr Ala Arg Arg Gly Leu Ser Leu Leu Leu Val Gly Gly Tyr
   1635 1640 1645
   Ser Pro Glu Asn Gly Phe Asn Gln Gln Leu Leu Glu Tyr Gln Leu Ala
   1650 1655 1660
   Thr Gly Thr Trp Val Ser Gly Ala Gln Ser Gly Thr Pro Pro Thr Gly
   1665 1670 1675 1680
   Leu Tyr Gly His Ser Ala Val Tyr His Glu Ala Thr Asp Ser Leu Tyr
   1685 1690 1695
   Val Phe Gly Gly Phe Arg Phe His Val Glu Leu Ala Ala Pro Ser Pro
   1700 1705 1710
   Glu Leu Tyr Ser Leu His Cys Pro Asp Arg Thr Trp Ser Leu Leu Ala
   1715 1720 1725
   Pro Ser Gln Gly Ala Lys Arg Asp Arg Met Arg Asn Val Arg Gly Ser
   1730 1735 1740
   Ser Arg Gly Leu Gly Gln Val Pro Gly Glu Gln Pro Gly Ser Trp Gly
   1745 1750 1755 1760
   Phe Arg Glu Val Arg Lys Lys Met Ala Leu Trp Ala Ala Leu Ala Gly
   1765 1770 1775
   Thr Gly Gly Phe Leu Glu Glu Ile Ser Pro His Leu Lys Glu Pro Arg
               1780                1785                1790
   Pro Arg Leu Phe His Ala Ser Ala Leu Leu Gly Asp Thr Met Val Val
           1795                1800                1805
   Leu Gly Gly Arg Ser Asp Pro Asp Glu Phe Ser Ser Asp Val Leu Leu
       1810                1815                1820
   Tyr Gln Val Asn Cys Asn Ala Trp Leu Leu Pro Asp Leu Thr Arg Ser
   1825                1830                1835                1840
   Ala Ser Val Gly Pro Pro Met Glu Glu Ser Val Ala His Ala Val Ala
                   1845                1850                1855
   Ala Val Gly Ser Arg Leu Tyr Ile Ser Gly Gly Phe Gly Gly Val Ala
               1860                1865                1870
   Leu Gly Arg Leu Leu Ala Leu Thr Leu Pro Pro Asp Pro Cys Arg Leu
           1875                1880                1885
   Leu Ser Ser Pro Glu Ala Cys Asn Gln Ser Gly Ala Cys Thr Trp Cys
       1890                1895                1900
   His Gly Ala Cys Leu Ser Gly Asp Gln Ala His Arg Leu Gly Cys Gly
   1905                1910                1915                1920
   Gly Ser Pro Cys Ser Pro Met Pro Arg Ser Pro Glu Glu Cys Arg Arg
                   1925                1930                1935
   Leu Arg Thr Cys Ser Glu Cys Leu Ala Arg His Pro Arg Thr Leu Gln
               1940                1945                1950
   Pro Gly Asp Gly Glu Ala Ser Thr Pro Arg Cys Lys Trp Cys Thr Asn
           1955                1960                1965
   Cys Pro Glu Gly Ala Cys Ile Gly Arg Asn Gly Ser Cys Thr Ser Glu
       1970                1975                1980
   Asn Asp Cys Arg Ile Asn Gln Arg Glu Val Phe Trp Ala Gly Asn Cys
   1985                1990                1995                2000
   Ser Glu Ala Ala Cys Gly Ala Ala Asp Cys Glu Gln Cys Thr Arg Glu
                   2005                2010                2015
   Gly Lys Cys Met Trp Thr Arg Gln Phe Lys Arg Thr Gly Glu Thr Arg
               2020                2025                2030
   Arg Ile Leu Ser Val Gln Pro Thr Tyr Asp Trp Thr Cys Phe Ser His
           2035                2040                2045
   Ser Leu Leu Asn Val Ser Pro Met Pro Val Glu Ser Ser Pro Pro Leu
       2050                2055                2060
   Pro Cys Pro Thr Pro Cys His Leu Leu Pro Asn Cys Thr Ser Cys Leu
   2065                2070                2075                2080
   Asp Ser Lys Gly Ala Asp Gly Gly Trp Gln His Cys Val Trp Ser Ser
                   2085                2090                2095
   Ser Leu Gln Gln Cys Leu Ser Pro Ser Tyr Leu Pro Leu Arg Cys Met
               2100                2105                2110
   Ala Gly Gly Cys Gly Arg Leu Leu Arg Gly Pro Glu Ser Cys Ser Leu
           2115                2120                2125
   Gly Cys Ala Gln Ala Thr Gln Cys Ala Leu Cys Leu Arg Arg Pro His
       2130                2135                2140
   Cys Gly Trp Cys Ala Trp Gly Gly Gln Asp Gly Gly Gly Arg Cys Met
   2145                2150                2155                2160
   Glu Gly Gly Leu Ser Gly Pro Arg Asp Gly Leu Thr Cys Gly Arg Pro
                   2165                2170                2175
   Gly Ala Ser Trp Ala Phe Leu Ser Cys Pro Pro Glu Asp Glu Cys Ala
               2180                2185                2190
   Asn Gly His His Asp Cys Asn Glu Thr Gln Asn Cys His Asp Gln Pro
           2195                2200                2205
   His Gly Tyr Glu Cys Ser Cys Lys Thr Gly Tyr Thr Met Asp Asn Met
       2210                2215                2220
   Thr Gly Leu Cys Arg Pro Val Cys Ala Gln Gly Cys Val Asn Gly Ser
   2225                2230                2235                2240
   Cys Val Glu Pro Asp His Cys Arg Cys His Phe Gly Phe Val Gly Arg
                   2245                2250                2255
   Asn Cys Ser Thr Glu Cys Arg Cys Asn Arg His Ser Glu Cys Ala Gly
               2260                2265                2270
   Val Gly Ala Arg Asp His Cys Leu Leu Cys Arg Asn His Thr Lys Gly
           2275                2280                2285
   Ser His Cys Glu Gln Cys Leu Pro Leu Phe Val Gly Ser Ala Val Gly
       2290                2295                2300
   Gly Gly Thr Cys Arg Pro Cys His Ala Phe Cys Arg Gly Asn Ser His
   2305                2310                2315                2320
   Ile Cys Ile Ser Arg Lys Glu Leu Gln Met Ser Lys Gly Glu Pro Lys
                   2325                2330                2335
   Lys Tyr Ser Leu Asp Pro Glu Glu Ile Glu Asn Trp Val Thr Glu Gly
               2340                2345                2350
   Pro Ser Glu Asp Glu Ala Val Cys Val Asn Cys Gln Asn Asn Ser Tyr
           2355                2360                2365
   Gly Glu Lys Cys Glu Ser Cys Leu Gln Gly Tyr Phe Leu Leu Asp Gly
       2370                2375                2380
   Lys Cys Thr Lys Cys Gln Cys Asn Gly His Ala Asp Thr Cys Asn Glu
   2385                2390                2395                2400
   Gln Asp Gly Thr Gly Cys Pro Cys Gln Asn Asn Thr Glu Thr Gly Thr
                   2405                2410                2415
   Cys Gln Gly Ser Ser Pro Ser Asp Arg Arg Asp Cys Tyr Lys Tyr Gln
               2420                2425                2430
   Cys Ala Lys Cys Arg Glu Ser Phe His Gly Ser Pro Leu Gly Gly Gln
           2435                2440                2445
   Gln Cys Tyr Arg Leu Ile Ser Val Glu Gln Glu Cys Cys Leu Asp Pro
       2450                2455                2460
   Thr Ser Gln Thr Asn Cys Phe His Glu Pro Lys Arg Arg Ala Leu Gly
   2465                2470                2475                2480
   Pro Gly Arg Thr Val Leu Phe Gly Val Gln Pro Lys Phe Thr Asn Val
                   2485                2490                2495
   Asp Ile Arg Leu Thr Leu Asp Val Thr Phe Gly Ala Val Asp Leu Tyr
               2500                2505                2510
   Val Ser Thr Ser Tyr Asp Thr Phe Val Val Arg Val Ala Pro Asp Thr
           2515                2520                2525
   Gly Val His Thr Val His Ile Gln Pro Pro Pro Ala Pro Pro Pro Pro
       2530                2535                2540
   Pro Pro Pro Ala Asp Gly Gly Pro Arg Gly Ala Gly Asp Pro Gly Gly
   2545                2550                2555                2560
   Ala Gly Ala Ser Ser Gly Pro Gly Ala Pro Ala Glu Pro Arg Val Arg
                   2565                2570                2575
   Glu Val Trp Pro Arg Gly Leu Ile Thr Tyr Val Thr Val Thr Glu Pro
               2580                2585                2590
   Ser Ala Val Leu Val Val Arg Gly Val Arg Asp Arg Leu Val Ile Thr
           2595                2600                2605
   Tyr Pro His Glu His His Ala Leu Lys Ser Ser Arg Phe Tyr Leu Leu
       2610                2615                2620
   Leu Leu Gly Val Gly Asp Pro Ser Gly Pro Gly Ala Asn Gly Ser Ala
   2625                2630                2635                2640
   Asp Ser Gln Gly Leu Leu Phe Phe Arg Gln Asp Gln Ala His Ile Asp
                   2645                2650                2655
   Leu Phe Val Phe Phe Ser Val Phe Phe Ser Cys Phe Phe Leu Phe Leu
               2660                2665                2670
   Ser Leu Cys Val Leu Leu Trp Lys Ala Lys Gln Ala Leu Asp Gln Arg
           2675                2680                2685
   Gln Glu Gln Arg Arg His Leu Gln Glu Met Thr Lys Met Ala Ser Arg
       2690                2695                2700
   Pro Phe Ala Lys Val Thr Val Cys Phe Pro Pro Asp Pro Thr Ala Pro
   2705                2710                2715                2720
   Ala Ser Ala Trp Lys Pro Ala Gly Leu Pro Pro Pro Ala Phe Arg Arg
                   2725                2730                2735
   Ser Glu Pro Phe Leu Ala Pro Leu Leu Leu Thr Gly Ala Gly Gly Pro
               2740                2745                2750
   Trp Gly Pro Met Gly Gly Gly Cys Cys Pro Pro Ala Ile Pro Ala Thr
           2755                2760                2765
   Thr Ala Gly Leu Arg Ala Gly Pro Ile Thr Leu Glu Pro Thr Glu Asp
       2770                2775                2780
   Gly Met Ala Gly Val Ala Thr Leu Leu Leu Gln Leu Pro Gly Gly Pro
   2785                2790                2795                2800
   His Ala Pro Asn Gly Ala Cys Leu Gly Ser Ala Leu Val Thr Leu Arg
                   2805                2810                2815
   His Arg Leu His Glu Tyr Cys Gly Gly Gly Gly Gly Ala Gly Gly Ser
               2820                2825                2830
   Gly His Gly Thr Gly Ala Gly Arg Lys Gly Leu Leu Ser Gln Asp Asn
           2835                2840                2845
   Leu Thr Ser Met Ser Leu
       2850

<210> 17

<211> 6006
<212> DNA
<213> Homo sapiens

<400> 17
caaatgtttc tttctacctt ttcttatttc agatcagctt tgagtgaact ttgacagaag 60
atgtttcgac agttttatct ctggacatgt ttagcttcag ggatcatcct gggctctctc 120
tttgaaatct gcttgggcca gtatgatgat ggtaaggatt gcaaactagc taggggagga 180
ccaccagcta ccatagttgc tattgatgaa gaaagtcgga atggtgcagg tacaattctg 240
gtggacaaca tgctgatcaa agggactgct ggaggaccag accccaccat agaactttct 300
ttaaaggata atgtggatta ctgggtgttg atggatcctg ttaagcaaat gcttttcctg 360
aacagcaccg gaagagttct ggatagagat ccaccgatga acatacactc cattgtggtg 420
caggtccagt gcatcaacaa aaaagtgggc actattatct accatgaagt gcgaatagtg 480
gtgagagaca ggaatgacaa ctcacccact ttcaagcatg aaagctacta tgccacagtg 540
aatgagctca ctccagttgg taccacaata ttcacaggat tttcaggaga caatggagct 600
acagatatag atgatggacc aaatggacag atagagtatg ttattcagta taatccagat 660
gatccgacat ccaatgacac ctttgaaatt cccctaatgt tgactggaaa tatagtgtta 720
aggaagaggc tcaactatga agataagact cgctactttg tcataatcca agctaatgac 780
cgtgcccaaa atctgaatga gaggcgaacc accaccacca ctctcacagt ggatgttctg 840
gatggagatg acttgggtcc aatgtttctt ccttgtgtcc ttgtgccaaa cactcgtgat 900
tgccgtccac tcacttatca agctgccata cctgagttga gaactccgga agaactgaac 960
cccattattg ttacgccacc aatccaagcc attgatcagg accggaatat tcaaccgcca 1020
tcagataggc caggaatcct ctattccatc cttgttggtg ggactcctga ggattaccca 1080
cgatttttcc atatgcatcc taggacagca gaacttagtc tcctggagcc agtaaacaga 1140
gactttcacc agaaatttga tttggttatt aaggctgaac aagacaatgg tcatcctctt 1200
cctgcctttg ccggtctaca cattgaaata ctggatgaaa acaatcaaag tccatatttt 1260
acaatgccca gttatcaagg ctatatcctg gaatctgccc cagtgggagc aaccatttcg 1320
gacagtctca atttgacctc acctttaaga atagtagctc tggacaagga catagaagat 1380
acaaaagacc cagagcttca cctttttctg aatgactaca cctcagtctt caccgtcaca 1440
cagactggta ttactcgcta cctcacctta cttcaaccag tggacaggga agaacagcaa 1500
acttacacct tttcgataac agcatttgat ggtgtacaag aaagtgagcc agtcatcgtc 1560
aatattcaag tgatggatgc aaatgataac acgccaacct tccctgaaat atcctatgat 1620
gtgtatgttt atacagacat gagacctggg gacagtgtca tacagctcac tgcagtcgac 1680
gcagacgaag ggtcaaatgg ggagatcaca tatgaaatcc ttgttggggc tcagggagac 1740
ttcatcatca ataaaacaac agggcttatc accatcgctc caggggtgga aatgatagtc 1800
gggcggactt acgcactcac ggtccaagca gcggataatg ctcctcctgc agagcgaagg 1860
aactccatct gcactgtgta tattgaagtg cttccaccaa ataatcaaag ccctcctcgc 1920
ttcccacagc tgatgtatag ccttgaaatt agtgaagcca tgagggttgg tgctgtttta 1980
ttaaatctac aggcaactga tcgagaggga gactcaataa catatgccat tgagaatgga 2040
gatcctcaga gagtttttaa tctttcagaa accacgggga ttctaacctt agggaaagca 2100
ctggacaggg aaagcactga tcgctacatt ctgatcatca cagcttcaga tggcaggcca 2160
gatgggacct caactgccac agtaaacata atggtgacag atgtcaatga caatgctcca 2220
gtgtttgatc cttatctgcc aagaaattta tctgtggtgg aagaagaagc caatgccttt 2280
gtgggtcaag taaaagcaac agaccctgat gctggaataa atggtcaagt gcactacagt 2340
ttgggtaact ttaataatct tttcgtatc acatccaatg ggagcattta cacagcagtg 2400
aagcttaaca gagaagtcag ggactactat gaacttgttg ttgtggcaac agatggagca 2460
gtacaccctc gtcattcaac tctaaccttg gccatcaagg ttttggacat tgatgataac 2520
agtcctgtgt tcaccaattc aacatacact gtccttgttg aagagaattt gccagctggg 2580
actaccatcc ttcaaataga ggccaaagat gtcgaccttg gagcaaatgt gtcttaccgg 2640
ataagaagcc cagaagtgaa gcactttttt gcactacatc catttacagg agaactatcg 2700
cttttaagga gtttagatta tgaggcattt ccagaccaag aagcaagtat cacttttctg 2760
gtagaggcct ttgatattta tggaacaatg ccacctggta ttgctactgt cacagtgatt 2820
gtaaaggata tgaatgatta tcctcctgtc tttagtaaac gaatatacaa agggatggtg 2880
gctccggatg cagtcaaggg tacacctatc acaacagttt atgctgaaga tgcagaccct 2940
cctggattac ctgcaagtcg tgtgaggtat agagtagatg atgtacagtt tccttaccct 3000
gccagtattt ttgaagtgga agaagattct ggaagagtaa taacacgagt caatcttaat 3060
gaagaaccta caacaatttt taagttggtg gtggttgctt ttgatgatgg ggagcctgtg 3120
atgtccagca gtgccacagt gaagattctt gtcttacatc ctggtgagat cccacgcttc 3180
acacaggagg aatataggcc tcctccagta agtgaacttg ccaccaaagg gaccatggtt 3240
ggtgtaattt ctgctgctgc cattaatcaa agtattgtgt actccattgt ttcaggaaat 3300
gaagaagata catttggaat taataacatc acaggtgtta tctatgtgaa tggacctctg 3360
gattatgaga ccaggacaag ctatgtactt cgagtccaag ctgattccct ggaagtggtc 3420
cttgccaatc tccgagttcc ttcaaaaagt aatacagcta aagtatacat tgagattcag 3480
gatgaaaata atcatccccc agtgtttcag aaaaaattct acatcggagg tgtatctgaa 3540
gatgcaagaa tgtttacttc tgtactcaga gtgaaggcta ctgataaaga tactggcaat 3600
tatagtgtca tggcctacag actcataata ccaccaatta aagagggaaa agaaggattt 3660
gtagtggaaa catatacagg gcttatcaaa actgctatgc tcttccataa tatgaggaga 3720
tcctacttca agtttcaagt tattgcaact gacgactatg ggaagggact gagcggcaaa 3780
gccgatgtac tcgtaagtgt ctccgtggtc aatcagctgg atatgcaagt cattgtttcc 3840

aatgtgcctc ctactctagt ggaaaaaaag atagaagatc ttacagaaat cttggatcgc 3900
tatgttcagg aacaaattcc tggtgccaag gtcgtagtgg agtccattgg agctcgccgg 3960
catggagatg ccttttccct agaagattac accaaatgtg acttgactgt ctatgcaatt 4020
gacccccaaa ccaacagagc catcgataga aatgagcttt ttaagttttt ggatggcaaa 4080
ctacttgata tcaataaaga ctttcagccg tattatgggg aaggaggacg cattctggag 4140
atccggactc cagaggcagt gaccagcatt aaaaagagag gagaaagtct aggatacaca 4200
gaaggggcct tgttggctct ggccttcatc atcatcctct gctgcattcc tgccatcttg 4260
gtggttttgg tcagctacag acagtttaaa gtacgtcaag ctgagtgtac acgtcaagct 4320
gagtgtacaa agactgcacg aattcaggcc gcattacccg cggctaaacc agcagtgccg 4380
gctcctgcac cagtggcagc gcccccgccg ccgccgccgc ctccgccagg tgcgcatctc 4440
tatgaagaac ttggagacag ctcaatgtct tttctttcaa gtcttttcct tctctaccat 4500
tttcaacaaa gcaggggaaa taactcagtc tcagaagaca ggaaacatca acaagttgtg 4560
atgccctttt cttccaatac tattgaggct cacaagtcag ctcatgtaga cggatcactt 4620
aagagcaaca aactgaagtc tgcaagaaaa ttcacatttc tatctgatga ggatgactta 4680
agtgcccata atccccttta taaggaaaac ataagtcaag tatcaacaaa ttcagacatt 4740
tcacagagaa cagattttgt agacccattt tcacccaaaa tacaagccaa gagtaagtct 4800
ctgaggggcc caagagaaaa gattcagagg ctgtggagtc agtcagtcag cttacccagg 4860
aggctgatga ggaaagttcc aaatagacca gagatcatag atctgcagca gtggcaaggc 4920
accaggcaga aagctgaaaa tgaaaacact ggaatctgta caaacaaaag aggtagcagc 4980
aatccattgc ttacaactga agaggcaaat ttgacagaga aagaggaaat aaggcaaggt 5040
gaaacactga tgatagaagg aacagaacag ttgaaatctc tctcttcaga ctcttcattt 5100
tgctttccca ggcctcactt ctcattctcc actttgccaa ctgtttcaag aactgtggaa 5160
ctcaaatcag aacctaatgt catcagttct cctgctgagt gttccttgga actttctcct 5220
tcaaggcctt gtgttttaca ttcttcactc tctaggagag agacacctat ttgtatgtta 5280
cctattgaaa ccgaaagaaa tatttttgaa aattttgccc atccaccaaa catctctcct 5340
tctgcctgtc cccttccccc tcctcctcct atttctcctc cttctcctcc tcctgctcct 5400
gctcctcttg ctcctcctcc tgacatttct cctttttctc tttttgtcc tcctccctct 5460
cctccttcta tccctcttcc tcttcctcct cctacatttt ttccactttc cgtttcaacg 5520
tctggtcccc caacaccacc tcttctacct ccatttccaa ctcctcttcc tccaccacct 5580
ccttctattc cttgccctcc acctccttca gcttcatttc tgtccacaga gtgtgtctgt 5640
ataacaggtg ttaaatgcac gaccaacttg atgcctgccg agaaaattaa gtcctctatg 5700
acacagctat caacaacgac agtgtgtaaa acagaccctc agagagaacc aaaaggcatc 5760
ctcagacacg ttaaaaactt agcagaactt gaaaaatcag tagctaacat gtacagtcaa 5820
atagaaaaaa actatctacg cacaaatgtt tcagaacttc aaactatgtg cccttcagaa 5880
gtaacaaata tggaaatcac atctgaacaa aacaagggga gtttgaacaa tattgtcgag 5940
ggaactgaaa aacaatctca cagtcaatct acttcactgt aatgttgctt ttcttatttt 6000
agtcgg 6006

<210> 18
<211> 1973
<212> PRT
<213> Homo sapiens

<400> 18
Met Phe Arg Gln Phe Tyr Leu Trp Thr Cys Leu Ala Ser Gly Ile Ile
1               5                   10                  15

Leu Gly Ser Leu Phe Glu Ile Cys Leu Gly Gln Tyr Asp Asp Gly Lys
            20                  25                  30

Asp Cys Lys Leu Ala Arg Gly Gly Pro Pro Ala Thr Ile Val Ala Ile
        35                  40                  45

Asp Glu Glu Ser Arg Asn Gly Ala Gly Thr Ile Leu Val Asp Asn Met
    50                  55                  60

Leu Ile Lys Gly Thr Ala Gly Gly Pro Asp Pro Thr Ile Glu Leu Ser
65                  70                  75                  80

Leu Lys Asp Asn Val Asp Tyr Trp Val Leu Met Asp Pro Val Lys Gln
                85                  90                  95

Met Leu Phe Leu Asn Ser Thr Gly Arg Val Leu Asp Arg Asp Pro Pro
            100                 105                 110

Met Asn Ile His Ser Ile Val Val Gln Val Gln Cys Ile Asn Lys Lys
        115                 120                 125

Val Gly Thr Ile Ile Tyr His Glu Val Arg Ile Val Val Arg Asp Arg
    130                 135                 140
Asn Asp Asn Ser Pro Thr Phe Lys His Glu Ser Tyr Tyr Ala Thr Val
145                 150                 155                 160
Asn Glu Leu Thr Pro Val Gly Thr Thr Ile Phe Thr Gly Phe Ser Gly
                165                 170                 175
Asp Asn Gly Ala Thr Asp Ile Asp Asp Gly Pro Asn Gly Gln Ile Glu
            180                 185                 190
Tyr Val Ile Gln Tyr Asn Pro Asp Asp Pro Thr Ser Asn Asp Thr Phe
        195                 200                 205
Glu Ile Pro Leu Met Leu Thr Gly Asn Ile Val Leu Arg Lys Arg Leu
    210                 215                 220
Asn Tyr Glu Asp Lys Thr Arg Tyr Phe Val Ile Ile Gln Ala Asn Asp
225                 230                 235                 240
Arg Ala Gln Asn Leu Asn Glu Arg Arg Thr Thr Thr Thr Thr Leu Thr
                245                 250                 255
Val Asp Val Leu Asp Gly Asp Asp Leu Gly Pro Met Phe Leu Pro Cys
            260                 265                 270
Val Leu Val Pro Asn Thr Arg Asp Cys Arg Pro Leu Thr Tyr Gln Ala
        275                 280                 285
Ala Ile Pro Glu Leu Arg Thr Pro Glu Glu Leu Asn Pro Ile Ile Val
    290                 295                 300
Thr Pro Pro Ile Gln Ala Ile Asp Gln Asp Arg Asn Ile Gln Pro Pro
305                 310                 315                 320
Ser Asp Arg Pro Gly Ile Leu Tyr Ser Ile Leu Val Gly Gly Thr Pro
                325                 330                 335
Glu Asp Tyr Pro Arg Phe Phe His Met His Pro Arg Thr Ala Glu Leu
            340                 345                 350
Ser Leu Leu Glu Pro Val Asn Arg Asp Phe His Gln Lys Phe Asp Leu
        355                 360                 365
Val Ile Lys Ala Glu Gln Asp Asn Gly His Pro Leu Pro Ala Phe Ala
    370                 375                 380
Gly Leu His Ile Glu Ile Leu Asp Glu Asn Asn Gln Ser Pro Tyr Phe
385                 390                 395                 400
Thr Met Pro Ser Tyr Gln Gly Tyr Ile Leu Glu Ser Ala Pro Val Gly
                405                 410                 415
Ala Thr Ile Ser Asp Ser Leu Asn Leu Thr Ser Pro Leu Arg Ile Val
            420                 425                 430
Ala Leu Asp Lys Asp Ile Glu Asp Thr Lys Asp Pro Glu Leu His Leu
        435                 440                 445
Phe Leu Asn Asp Tyr Thr Ser Val Phe Thr Val Thr Gln Thr Gly Ile
    450                 455                 460
Thr Arg Tyr Leu Thr Leu Leu Gln Pro Val Asp Arg Glu Glu Gln Gln
465                 470                 475                 480
Thr Tyr Thr Phe Ser Ile Thr Ala Phe Asp Gly Val Gln Glu Ser Glu
                485                 490                 495

Pro Val Ile Val Asn Ile Gln Val Met Asp Ala Asn Asp Asn Thr Pro
            500                 505                 510

Thr Phe Pro Glu Ile Ser Tyr Asp Val Tyr Val Tyr Thr Asp Met Arg
        515                 520                 525

Pro Gly Asp Ser Val Ile Gln Leu Thr Ala Val Asp Ala Asp Glu Gly
    530                 535                 540

Ser Asn Gly Glu Ile Thr Tyr Glu Ile Leu Val Gly Ala Gln Gly Asp
545                 550                 555                 560

Phe Ile Ile Asn Lys Thr Thr Gly Leu Ile Thr Ile Ala Pro Gly Val
                565                 570                 575

Glu Met Ile Val Gly Arg Thr Tyr Ala Leu Thr Val Gln Ala Ala Asp
            580                 585                 590

Asn Ala Pro Pro Ala Glu Arg Arg Asn Ser Ile Cys Thr Val Tyr Ile
        595                 600                 605

Glu Val Leu Pro Pro Asn Asn Gln Ser Pro Pro Arg Phe Pro Gln Leu
    610                 615                 620

Met Tyr Ser Leu Glu Ile Ser Glu Ala Met Arg Val Gly Ala Val Leu
625                 630                 635                 640

Leu Asn Leu Gln Ala Thr Asp Arg Glu Gly Asp Ser Ile Thr Tyr Ala
                645                 650                 655

Ile Glu Asn Gly Asp Pro Gln Arg Val Phe Asn Leu Ser Glu Thr Thr
            660                 665                 670

Gly Ile Leu Thr Leu Gly Lys Ala Leu Asp Arg Glu Ser Thr Asp Arg
        675                 680                 685

Tyr Ile Leu Ile Ile Thr Ala Ser Asp Gly Arg Pro Asp Gly Thr Ser
    690                 695                 700

Thr Ala Thr Val Asn Ile Met Val Thr Asp Val Asn Asp Asn Ala Pro
705                 710                 715                 720

Val Phe Asp Pro Tyr Leu Pro Arg Asn Leu Ser Val Val Glu Glu Glu
                725                 730                 735

Ala Asn Ala Phe Val Gly Gln Val Lys Ala Thr Asp Pro Asp Ala Gly
            740                 745                 750

Ile Asn Gly Gln Val His Tyr Ser Leu Gly Asn Phe Asn Asn Leu Phe
        755                 760                 765

Arg Ile Thr Ser Asn Gly Ser Ile Tyr Thr Ala Val Lys Leu Asn Arg
    770                 775                 780

Glu Val Arg Asp Tyr Tyr Glu Leu Val Val Val Ala Thr Asp Gly Ala
785                 790                 795                 800

Val His Pro Arg His Ser Thr Leu Thr Leu Ala Ile Lys Val Leu Asp
                805                 810                 815

Ile Asp Asp Asn Ser Pro Val Phe Thr Asn Ser Thr Tyr Thr Val Leu
            820                 825                 830

Val Glu Glu Asn Leu Pro Ala Gly Thr Thr Ile Leu Gln Ile Glu Ala
        835                 840                 845

Lys Asp Val Asp Leu Gly Ala Asn Val Ser Tyr Arg Ile Arg Ser Pro
    850                 855                 860

Glu Val Lys His Phe Phe Ala Leu His Pro Phe Thr Gly Glu Leu Ser
865                 870                 875                 880

Leu Leu Arg Ser Leu Asp Tyr Glu Ala Phe Pro Asp Gln Glu Ala Ser
                885                 890                 895

Ile Thr Phe Leu Val Glu Ala Phe Asp Ile Tyr Gly Thr Met Pro Pro
            900                 905                 910

Gly Ile Ala Thr Val Thr Val Ile Val Lys Asp Met Asn Asp Tyr Pro
        915                 920                 925

Pro Val Phe Ser Lys Arg Ile Tyr Lys Gly Met Val Ala Pro Asp Ala
    930                 935                 940

Val Lys Gly Thr Pro Ile Thr Thr Val Tyr Ala Glu Asp Ala Asp Pro
945                 950                 955                 960

Pro Gly Leu Pro Ala Ser Arg Val Arg Tyr Arg Val Asp Asp Val Gln
                965                 970                 975

Phe Pro Tyr Pro Ala Ser Ile Phe Glu Val Glu Glu Asp Ser Gly Arg
            980                 985                 990

Val Ile Thr Arg Val Asn Leu Asn Glu Glu Pro Thr Thr Ile Phe Lys
        995                 1000                1005

Leu Val Val Val Ala Phe Asp Asp Gly Glu Pro Val Met Ser Ser Ser
    1010                1015                1020

Ala Thr Val Lys Ile Leu Val Leu His Pro Gly Glu Ile Pro Arg Phe
1025                1030                1035                1040

Thr Gln Glu Glu Tyr Arg Pro Pro Pro Val Ser Glu Leu Ala Thr Lys
                1045                1050                1055

Gly Thr Met Val Gly Val Ile Ser Ala Ala Ala Ile Asn Gln Ser Ile
            1060                1065                1070

Val Tyr Ser Ile Val Ser Gly Asn Glu Glu Asp Thr Phe Gly Ile Asn
        1075                1080                1085

Asn Ile Thr Gly Val Ile Tyr Val Asn Gly Pro Leu Asp Tyr Glu Thr
    1090                1095                1100

Arg Thr Ser Tyr Val Leu Arg Val Gln Ala Asp Ser Leu Glu Val Val
1105                1110                1115                1120

Leu Ala Asn Leu Arg Val Pro Ser Lys Ser Asn Thr Ala Lys Val Tyr
                1125                1130                1135

Ile Glu Ile Gln Asp Glu Asn Asn His Pro Pro Val Phe Gln Lys Lys
            1140                1145                1150

Phe Tyr Ile Gly Gly Val Ser Glu Asp Ala Arg Met Phe Thr Ser Val
        1155                1160                1165

Leu Arg Val Lys Ala Thr Asp Lys Asp Thr Gly Asn Tyr Ser Val Met
    1170                1175                1180

Ala Tyr Arg Leu Ile Ile Pro Pro Ile Lys Glu Gly Lys Glu Gly Phe
1185                1190                1195                1200

Val Val Glu Thr Tyr Thr Gly Leu Ile Lys Thr Ala Met Leu Phe His
                1205                1210                1215

Asn Met Arg Arg Ser Tyr Phe Lys Phe Gln Val Ile Ala Thr Asp Asp
            1220                1225                1230

Tyr Gly Lys Gly Leu Ser Gly Lys Ala Asp Val Leu Val Ser Val Ser
        1235                1240                1245

Val Val Asn Gln Leu Asp Met Gln Val Ile Val Ser Asn Val Pro Pro
    1250                1255                1260

Thr Leu Val Glu Lys Lys Ile Glu Asp Leu Thr Glu Ile Leu Asp Arg
1265                1270                1275                1280

Tyr Val Gln Glu Gln Ile Pro Gly Ala Lys Val Val Val Glu Ser Ile
                1285                1290                1295

Gly Ala Arg Arg His Gly Asp Ala Phe Ser Leu Glu Asp Tyr Thr Lys
            1300                1305                1310

Cys Asp Leu Thr Val Tyr Ala Ile Asp Pro Gln Thr Asn Arg Ala Ile
        1315                1320                1325

Asp Arg Asn Glu Leu Phe Lys Phe Leu Asp Gly Lys Leu Leu Asp Ile
    1330                1335                1340

Asn Lys Asp Phe Gln Pro Tyr Tyr Gly Glu Gly Gly Arg Ile Leu Glu
1345                1350                1355                1360

Ile Arg Thr Pro Glu Ala Val Thr Ser Ile Lys Lys Arg Gly Glu Ser
                1365                1370                1375

Leu Gly Tyr Thr Glu Gly Ala Leu Leu Ala Leu Ala Phe Ile Ile Ile
            1380                1385                1390

Leu Cys Cys Ile Pro Ala Ile Leu Val Val Leu Val Ser Tyr Arg Gln
        1395                1400                1405

Phe Lys Val Arg Gln Ala Glu Cys Thr Arg Gln Ala Glu Cys Thr Lys
    1410                1415                1420

Thr Ala Arg Ile Gln Ala Ala Leu Pro Ala Ala Lys Pro Ala Val Pro
1425                1430                1435                1440

Ala Pro Ala Pro Val Ala Ala Pro Pro Pro Pro Pro Pro Pro Pro Pro
                1445                1450                1455

Gly Ala His Leu Tyr Glu Glu Leu Gly Asp Ser Ser Met Ser Phe Leu
            1460                1465                1470

Ser Ser Leu Phe Leu Leu Tyr His Phe Gln Gln Ser Arg Gly Asn Asn
        1475                1480                1485

Ser Val Ser Glu Asp Arg Lys His Gln Gln Val Val Met Pro Phe Ser
    1490                1495                1500

Ser Asn Thr Ile Glu Ala His Lys Ser Ala His Val Asp Gly Ser Leu
1505                1510                1515                1520

Lys Ser Asn Lys Leu Lys Ser Ala Arg Lys Phe Thr Phe Leu Ser Asp
                1525                1530                1535

Glu Asp Asp Leu Ser Ala His Asn Pro Leu Tyr Lys Glu Asn Ile Ser
            1540                1545                1550

Gln Val Ser Thr Asn Ser Asp Ile Ser Gln Arg Thr Asp Phe Val Asp
        1555                1560                1565

Pro Phe Ser Pro Lys Ile Gln Ala Lys Ser Lys Ser Leu Arg Gly Pro
    1570                1575                1580

Arg Glu Lys Ile Gln Arg Leu Trp Ser Gln Ser Val Ser Leu Pro Arg
1585                1590                1595                1600

Arg Leu Met Arg Lys Val Pro Asn Arg Pro Glu Ile Ile Asp Leu Gln
                1605                1610                1615

Gln Trp Gln Gly Thr Arg Gln Lys Ala Glu Asn Glu Asn Thr Gly Ile
            1620                1625                1630

Cys Thr Asn Lys Arg Gly Ser Ser Asn Pro Leu Leu Thr Thr Glu Glu
        1635                1640                1645

Ala Asn Leu Thr Glu Lys Glu Glu Ile Arg Gln Gly Glu Thr Leu Met
    1650                1655                1660

Ile Glu Gly Thr Glu Gln Leu Lys Ser Leu Ser Ser Asp Ser Ser Phe
1665                1670                1675                1680

Cys Phe Pro Arg Pro His Phe Ser Phe Ser Thr Leu Pro Thr Val Ser
                1685                1690                1695

Arg Thr Val Glu Leu Lys Ser Glu Pro Asn Val Ile Ser Ser Pro Ala
            1700                1705                1710

Glu Cys Ser Leu Glu Leu Ser Pro Ser Arg Pro Cys Val Leu His Ser
        1715                1720                1725

Ser Leu Ser Arg Arg Glu Thr Pro Ile Cys Met Leu Pro Ile Glu Thr
    1730                1735                1740

Glu Arg Asn Ile Phe Glu Asn Phe Ala His Pro Pro Asn Ile Ser Pro
1745                1750                1755                1760

Ser Ala Cys Pro Leu Pro Pro Pro Pro Pro Ile Ser Pro Pro Ser Pro
                1765                1770                1775

Pro Pro Ala Pro Ala Pro Leu Ala Pro Pro Pro Asp Ile Ser Pro Phe
            1780                1785                1790

Ser Leu Phe Cys Pro Pro Pro Ser Pro Pro Ser Ile Pro Leu Pro Leu
        1795                1800                1805

Pro Pro Pro Thr Phe Phe Pro Leu Ser Val Ser Thr Ser Gly Pro Pro
    1810                1815                1820

Thr Pro Pro Leu Leu Pro Pro Phe Pro Thr Pro Leu Pro Pro Pro Pro
1825                1830                1835                1840

Pro Ser Ile Pro Cys Pro Pro Pro Pro Ser Ala Ser Phe Leu Ser Thr
                1845                1850                1855

Glu Cys Val Cys Ile Thr Gly Val Lys Cys Thr Thr Asn Leu Met Pro
            1860                1865                1870

Ala Glu Lys Ile Lys Ser Ser Met Thr Gln Leu Ser Thr Thr Thr Val
        1875                1880                1885

Cys Lys Thr Asp Pro Gln Arg Glu Pro Lys Gly Ile Leu Arg His Val
    1890                1895                1900

Lys Asn Leu Ala Glu Leu Glu Lys Ser Val Ala Asn Met Tyr Ser Gln
1905                1910                1915                1920

Ile Glu Lys Asn Tyr Leu Arg Thr Asn Val Ser Glu Leu Gln Thr Met
                1925                1930                1935

Cys Pro Ser Glu Val Thr Asn Met Glu Ile Thr Ser Glu Gln Asn Lys
            1940                1945                1950

Gly Ser Leu Asn Asn Ile Val Glu Gly Thr Glu Lys Gln Ser His Ser
        1955                1960                1965

Gln Ser Thr Ser Leu
    1970

<210> 19
<211> 6003
<212> DNA
<213> Homo sapiens

<400> 19
caaatgtttc tttctacctt ttcttatttc agatcagctt tgagtgaact ttgacagaag 60
atgtttcgac agttttatct ctggacatgt ttagcttcag ggatcatcct gggctctctc 120
tttgaaatct gcttgggcca gtatgatgat ggtaaggatt gcaaactagc taggggagga 180
ccaccagcta ccatagttgc tattgatgaa gaaagtcgga atggtgcagg tacaattctg 240
gtggacaaca tgctgatcaa agggactgct ggaggaccag accccaccat agaactttct 300
ttaaaggata atgtggatta ctgggtgttg atggatcctg ttaagcaaat gcttttcctg 360
aacagcaccg gaagagttct ggatagagat ccaccgatga acatacactc cattgtggtg 420
caggtccagt gcatcaacaa aaaagtgggc actattatct accatgaagt gcgaatagtg 480
gtgagagaca ggaatgacaa ctcacccact ttcaagcatg aaagctacta tgccacagtg 540
aatgagctca ctccagttgg taccacaata ttcacaggat tttcaggaga caatggagct 600
acagatatag atgatggacc aaatggacag atagagtatg ttattcagta taatccagat 660
gatccgacat ccaatgacac ctttgaaatt cccctaatgt tgactggaaa tatagtgtta 720
aggaagaggc tcaactatga agataagact cgctactttg tcataatcca agctaatgac 780
cgtgcccaaa atctgaatga gaggcgaacc accaccacca ctctcacagt ggatgttctg 840
gatggagatg acttgggtcc aatgtttctt ccttgtgtcc ttgtgccaaa cactcgtgat 900
tgccgtccac tcacttatca agctgccata cctgagttga gaactccgga agaactgaac 960
cccattattg ttacgccacc aatccaagcc attgatcagg accggaatat tcaaccgcca 1020
tcagataggc caggaatcct ctattccatc cttgttggga ctcctgagga ttacccacga 1080
tttttccata tgcatcctag gacagcagaa cttagtctcc tggagccagt aaacagagac 1140
tttcaccaga aatttgattt ggttattaag gctgaacaag acaatggtca tcctcttcct 1200
gcctttgcca gtctacacat tgaaatactg gatgaaaaca atcaaagtcc atattttaca 1260
atgcccagtt atcaaggcta tatcctggaa tctgccccag tgggagcaac catttcggac 1320
agtctcaatt tgacttcacc tttaagaata gtagctctgg acaaggacat agaagataca 1380
aaagacccag agcttcacct ttttctgaat gactacacct cagtcttcac cgtcacacag 1440
actggtatta ctcgctacct cagcttactt caaccagtgg acagggaaga acagcaaact 1500
tacacctttt cgataacagc atttgatggt gtacaagaaa gtgagccagt catcgtcaat 1560
attcaagtga tggatgcaaa tgataacacg ccaaccttcc ctgaaatatc ctatgatgtg 1620
tatgtttata cagacatgag acctggggac agtgtcatac agctcactgc agtcgacgca 1680
gacgaagggt caaatgggga gatcacatat gaaatccttg ttggggctca gggagacttc 1740
atcatcaata aaacaacagg gcttatcacc atcgctccag gggtggaaat gatagtcggg 1800
cggacttacg cactcacggt ccaagcagcg gataatgctc ctcctgcaga gcgaaggaac 1860
tccatctgca ctgtgtatat tgaagtgctt ccaccaaata atcaaagccc tcctcgcttc 1920
ccacagctga tgtatagcct tgaaattagt gaagccatga gggttggtgc tgttttatta 1980
aatctacagg caactgatcg agagggagac tcaataacat atgccattga gaatggagat 2040
cctcagagag tttttaatct ttcagaaacc acggggattc taaccttagg gaaagcactg 2100
gacagggaaa gcactgatcg ctacattctg atcatcacag cttcagatgg caggccagat 2160
gggacctcaa ctgccacagt aaacatagtg gtgacagatg tcaatgacaa tgctccagtg 2220
tttgatcctt atctgccaag aaatttatct gtggtggaag aagaagccaa tgcctttgtg 2280
ggtcaagtaa aagcaacaga ccctgatgct ggaataaatg gtcaagtgca ctacagtttg 2340
ggtaacttta ataatctttt tcgtatcaca tccaatggga gcatttacac agcagtgaag 2400
cttaacagag aagtcaggga ctactatgaa cttgttgttg tggcaacaga tggagcagta 2460
caccctcgtc attcaactct aaccttggcc atcaaggttt tggacattga tgataacagt 2520
cctgtgttca ccaattcaac atacactgtc cttgttgaag agaatttgcc agctgggact 2580
accatccttc aaatagaggc caaagatgtc gaccttggag caaatgtgtc ttaccggata 2640
agaagcccag aagtgaagca ctttttgca ctacatccat ttacaggaga actatcgctt 2700
ttaaggagtt tagattatga ggcatttcca gaccaagaag caagtatcac ttttctggta 2760
gaggcctttg atatttatgg aacaatgcca cctggtattg ctactgtcac agtgattgta 2820
aaggatatga atgattatcc tcctgtcttt agtaaacgaa tatacaaagg gatggtggct 2880
ccggatgcag tcaagggtac acctatcaca acagtttatg ctgaagatgc agaccctcct 2940
ggattacctg caagtcgtgt gaggtataga gtagatgatg tacagtttcc ttaccctgcc 3000
agtattttg aagtggaaga agattctgga agagtaataa cacgagtcaa tcttaatgaa 3060
gaacctacaa caatttttaa gttggtggtg gttgcttttg atgatgggga gcctgtgatg 3120
tccagcagtg ccacagtgaa gattcttgtc ttacatcctg gtgagatccc acgcttcaca 3180
caggaggaat ataggcctcc tccagtaagt gaacttgcca ccaaagggac catggttggt 3240
gtaatttctg ctgctgccat taatcaaagt attgtgtact ccattgtttc aggaaatgaa 3300
gaagatacat ttggaattaa taacatcaca ggtgttatct atgtgaatgg acctctggat 3360
tatgagacca ggacaagcta tgtacttcga gtccaagctg attccctgga agtggtcctt 3420
gccaatctcc gagttccttc aaaaagtaat acagctaaag tatacattga gattcaggat 3480
gaaaataatc atcccccagt gtttcagaaa aaattctaca tcggaggtgt atctgaagat 3540

gcaagaatgt ttacttctgt actcagagtg aaggctactg ataaagatac tggcaattat 3600
agtgtcatgg cctacagact cataatacca ccaattaaag agggaaaaga aggatttgta 3660
gtggaaacat atacagggct tatcaaaact gctatgctct tccataatat gaggagatcc 3720
tacttcaagt ttcaagttat tgcaactgac gactatggga agggactgag cggcaaagcc 3780
gatgtactcg taagtgtctc cgtggtcaat cagctggata tgcaagtcat tgtttccaat 3840
gtgcctccta ctctagtgga aaaaaagata gaagatctta cagaaatctt ggatcgctat 3900
gttcaggaac aaattcctgg tgccaaggtc gtagtggagt ccattggagc tcgccggcat 3960
ggagatgcct tttccctaga agattacacc aaatgtgact tgactgtcta tgcaattgac 4020
ccccaaacca acagagccat cgatagaaat gagcttttta agtttttgga tggcaaacta 4080
cttgatatca ataaagactt tcagccgtat tatggggaag gaggacgcat tctggagatc 4140
cggactccag aggcagtgac cagcattaaa aagagaggag aaagtctagg atacacagaa 4200
ggggccttgt tggctctggc cttcatcatc atcctctgct gcattcctgc catcttggtg 4260
gttttggtca gctacagaca gtttaaagta cgtcaagctg agtgtacacg tcaagctgag 4320
tgtacaaaga ctgcacgaat tcaggccgca ttacccgcgg ctaaaccagc agtgccggct 4380
cctgcaccag tggcagcgcc cccgccgccg ccgccgcctc cgccaggtgc gcatctctat 4440
gaagaacttg gagacagctc aatgtctttt ctttcaagtc ttttccttct ctaccatttt 4500
caacaaagca ggggaaataa ctcagtctca gaagacagga aacatcaaca agttgtgatg 4560
cccttttctt ccaatactat tgaggctcac aagtcagctc atgtagacgg atcacttaag 4620
agcaacaaac tgaagtctgc aagaaaattc acatttctat ctgatgagga tgacttaagt 4680
gcccataatc ccctttataa ggaaaacata agtcaagtat caacaaattc agacatttca 4740
cagagaacag attttgtaga cccattttca cccaaaatac aagccaagag taagtctctg 4800
aggggcccaa gagaaaagat tcagaggctg tggagtcagt cagtcagctt acccaggagg 4860
ctgatgagga aagttccaaa tagaccagag atcatagatc tgcagcagtg gcaaggcacc 4920
aggcagaaag ctgaaaatga aaacactgga atctgtacaa acaaaagagg tagcagcaat 4980
ccattgctta caactgaaga ggcaaatttg acagagaaag aggaaataag gcaaggtgaa 5040
acactgatga tagaaggaac agaacagttg aaatctctct cttcagactc ttcattttgc 5100
tttcccaggc ctcacttctc attctccact ttgccaactg tttcaagaac tgtggaactc 5160
aaatcagaac ctaatgtcat cagttctcct gctgagtgtt ccttggaact ttctccttca 5220
aggccttgtg ttttacattc ttcactctct aggagagaga cacctatttg tatgttacct 5280
attgaaaccg aaagaaatat ttttgaaaat tttgcccatc caccaaacat ctctccttct 5340
gcctgtcccc ttccccctcc tcctcctatt tctcctcctt ctcctcctcc tgctcctgct 5400
cctcttgctc ctcctcctga catttctcct ttttctcttt tttgtcctcc tccctctcct 5460
ccttctatcc ctcttcctct tcctcctcct acattttttc cactttccgt ttcaacgtct 5520
ggtcccccaa caccacctct tctacctcca tttccaactc ctcttcctcc accacctcct 5580
tctattcctt gccctccacc tccttcagct tcatttctgt ccacagagtg tgtctgtata 5640
acaggtgtta aatgcacgac caacttgatg cctgccgaga aaattaagtc ctctatgaca 5700
cagctatcaa caacgacagt gtgtaaaaca gaccctcaga gagaaccaaa aggcatcctc 5760
agacacgtta aaaacttagc agaacttgaa aaatcagtag ctaacatgta cagtcaaata 5820
gaaaaaaact atctacgcac aaatgtttca gaacttcaaa ctatgtgccc ttcagaagta 5880
acaaatatgg aaatcacatc tgaacaaaac aaggggagtt tgaacaatat tgtcgaggga 5940
actgaaaaac aatctcacag tcaatctact tcactgtaat gttgcttttc ttattttagt 6000
cgg 6003

<210> 20
<211> 1972
<212> PRT
<213> Homo sapiens

<400> 20
Met Phe Arg Gln Phe Tyr Leu Trp Thr Cys Leu Ala Ser Gly Ile Ile
1               5                   10                  15

Leu Gly Ser Leu Phe Glu Ile Cys Leu Gly Gln Tyr Asp Asp Gly Lys
            20                  25                  30

Asp Cys Lys Leu Ala Arg Gly Gly Pro Pro Ala Thr Ile Val Ala Ile
        35                  40                  45

Asp Glu Glu Ser Arg Asn Gly Ala Gly Thr Ile Leu Val Asp Asn Met
    50                  55                  60

Leu Ile Lys Gly Thr Ala Gly Gly Pro Asp Pro Thr Ile Glu Leu Ser
65                  70                  75                  80

Leu Lys Asp Asn Val Asp Tyr Trp Val Leu Met Asp Pro Val Lys Gln
                85                  90                  95

Met Leu Phe Leu Asn Ser Thr Gly Arg Val Leu Asp Arg Asp Pro Pro

            100                 105                 110

Met Asn Ile His Ser Ile Val Val Gln Val Gln Cys Ile Asn Lys Lys
        115                 120                 125

Val Gly Thr Ile Ile Tyr His Glu Val Arg Ile Val Val Arg Asp Arg
    130                 135                 140

Asn Asp Asn Ser Pro Thr Phe Lys His Glu Ser Tyr Tyr Ala Thr Val
145                 150                 155                 160

Asn Glu Leu Thr Pro Val Gly Thr Thr Ile Phe Thr Gly Phe Ser Gly
                165                 170                 175

Asp Asn Gly Ala Thr Asp Ile Asp Asp Gly Pro Asn Gly Gln Ile Glu
            180                 185                 190

Tyr Val Ile Gln Tyr Asn Pro Asp Asp Pro Thr Ser Asn Asp Thr Phe
        195                 200                 205

Glu Ile Pro Leu Met Leu Thr Gly Asn Ile Val Leu Arg Lys Arg Leu
    210                 215                 220

Asn Tyr Glu Asp Lys Thr Arg Tyr Phe Val Ile Ile Gln Ala Asn Asp
225                 230                 235                 240

Arg Ala Gln Asn Leu Asn Glu Arg Arg Thr Thr Thr Thr Thr Leu Thr
                245                 250                 255

Val Asp Val Leu Asp Gly Asp Asp Leu Gly Pro Met Phe Leu Pro Cys
            260                 265                 270

Val Leu Val Pro Asn Thr Arg Asp Cys Arg Pro Leu Thr Tyr Gln Ala
        275                 280                 285

Ala Ile Pro Glu Leu Arg Thr Pro Glu Glu Leu Asn Pro Ile Ile Val
    290                 295                 300

Thr Pro Pro Ile Gln Ala Ile Asp Gln Asp Arg Asn Ile Gln Pro Pro
305                 310                 315                 320

Ser Asp Arg Pro Gly Ile Leu Tyr Ser Ile Leu Val Gly Thr Pro Glu
                325                 330                 335

Asp Tyr Pro Arg Phe Phe His Met His Pro Arg Thr Ala Glu Leu Ser
            340                 345                 350

Leu Leu Glu Pro Val Asn Arg Asp Phe His Gln Lys Phe Asp Leu Val
        355                 360                 365

Ile Lys Ala Glu Gln Asp Asn Gly His Pro Leu Pro Ala Phe Ala Ser
    370                 375                 380

Leu His Ile Glu Ile Leu Asp Glu Asn Asn Gln Ser Pro Tyr Phe Thr
385                 390                 395                 400

Met Pro Ser Tyr Gln Gly Tyr Ile Leu Glu Ser Ala Pro Val Gly Ala
                405                 410                 415

Thr Ile Ser Asp Ser Leu Asn Leu Thr Ser Pro Leu Arg Ile Val Ala
            420                 425                 430

Leu Asp Lys Asp Ile Glu Asp Thr Lys Asp Pro Glu Leu His Leu Phe
        435                 440                 445

Leu Asn Asp Tyr Thr Ser Val Phe Thr Val Thr Gln Thr Gly Ile Thr
    450                 455                 460

Arg Tyr Leu Ser Leu Leu Gln Pro Val Asp Arg Glu Glu Gln Gln Thr

465                 470                 475                 480

Tyr Thr Phe Ser Ile Thr Ala Phe Asp Gly Val Gln Glu Ser Glu Pro
                485                 490                 495

Val Ile Val Asn Ile Gln Val Met Asp Ala Asn Asp Asn Thr Pro Thr
            500                 505                 510

Phe Pro Glu Ile Ser Tyr Asp Val Tyr Val Tyr Thr Asp Met Arg Pro
        515                 520                 525

Gly Asp Ser Val Ile Gln Leu Thr Ala Val Asp Ala Asp Glu Gly Ser
    530                 535                 540

Asn Gly Glu Ile Thr Tyr Glu Ile Leu Val Gly Ala Gln Gly Asp Phe
545                 550                 555                 560

Ile Ile Asn Lys Thr Thr Gly Leu Ile Thr Ile Ala Pro Gly Val Glu
                565                 570                 575

Met Ile Val Gly Arg Thr Tyr Ala Leu Thr Val Gln Ala Ala Asp Asn
            580                 585                 590

Ala Pro Pro Ala Glu Arg Arg Asn Ser Ile Cys Thr Val Tyr Ile Glu
        595                 600                 605

Val Leu Pro Pro Asn Asn Gln Ser Pro Pro Arg Phe Pro Gln Leu Met
    610                 615                 620

Tyr Ser Leu Glu Ile Ser Glu Ala Met Arg Val Gly Ala Val Leu Leu
625                 630                 635                 640

Asn Leu Gln Ala Thr Asp Arg Glu Gly Asp Ser Ile Thr Tyr Ala Ile
                645                 650                 655

Glu Asn Gly Asp Pro Gln Arg Val Phe Asn Leu Ser Glu Thr Thr Gly
            660                 665                 670

Ile Leu Thr Leu Gly Lys Ala Leu Asp Arg Glu Ser Thr Asp Arg Tyr
        675                 680                 685

Ile Leu Ile Ile Thr Ala Ser Asp Gly Arg Pro Asp Gly Thr Ser Thr
    690                 695                 700

Ala Thr Val Asn Ile Val Val Thr Asp Val Asn Asp Asn Ala Pro Val
705                 710                 715                 720

Phe Asp Pro Tyr Leu Pro Arg Asn Leu Ser Val Val Glu Glu Glu Ala
                725                 730                 735

Asn Ala Phe Val Gly Gln Val Lys Ala Thr Asp Pro Asp Ala Gly Ile
            740                 745                 750

Asn Gly Gln Val His Tyr Ser Leu Gly Asn Phe Asn Asn Leu Phe Arg
        755                 760                 765

Ile Thr Ser Asn Gly Ser Ile Tyr Thr Ala Val Lys Leu Asn Arg Glu
    770                 775                 780

Val Arg Asp Tyr Tyr Glu Leu Val Val Val Ala Thr Asp Gly Ala Val
785                 790                 795                 800

His Pro Arg His Ser Thr Leu Thr Leu Ala Ile Lys Val Leu Asp Ile
                805                 810                 815

Asp Asp Asn Ser Pro Val Phe Thr Asn Ser Thr Tyr Thr Val Leu Val
            820                 825                 830

Glu Glu Asn Leu Pro Ala Gly Thr Thr Ile Leu Gln Ile Glu Ala Lys

835                 840                 845

Asp Val Asp Leu Gly Ala Asn Val Ser Tyr Arg Ile Arg Ser Pro Glu
    850                 855                 860

Val Lys His Phe Phe Ala Leu His Pro Phe Thr Gly Glu Leu Ser Leu
865                 870                 875                 880

Leu Arg Ser Leu Asp Tyr Glu Ala Phe Pro Asp Gln Glu Ala Ser Ile
                885                 890                 895

Thr Phe Leu Val Glu Ala Phe Asp Ile Tyr Gly Thr Met Pro Pro Gly
            900                 905                 910

Ile Ala Thr Val Thr Val Ile Val Lys Asp Met Asn Asp Tyr Pro Pro
        915                 920                 925

Val Phe Ser Lys Arg Ile Tyr Lys Gly Met Val Ala Pro Asp Ala Val
    930                 935                 940

Lys Gly Thr Pro Ile Thr Thr Val Tyr Ala Glu Asp Ala Asp Pro Pro
945                 950                 955                 960

Gly Leu Pro Ala Ser Arg Val Arg Tyr Arg Val Asp Asp Val Gln Phe
                965                 970                 975

Pro Tyr Pro Ala Ser Ile Phe Glu Val Glu Glu Asp Ser Gly Arg Val
            980                 985                 990

Ile Thr Arg Val Asn Leu Asn Glu Glu Pro Thr Thr Ile Phe Lys Leu
        995                 1000                1005

Val Val Val Ala Phe Asp Asp Gly Glu Pro Val Met Ser Ser Ser Ala
    1010                1015                1020

Thr Val Lys Ile Leu Val Leu His Pro Gly Glu Ile Pro Arg Phe Thr
1025                1030                1035                1040

Gln Glu Glu Tyr Arg Pro Pro Pro Val Ser Glu Leu Ala Thr Lys Gly
                1045                1050                1055

Thr Met Val Gly Val Ile Ser Ala Ala Ala Ile Asn Gln Ser Ile Val
            1060                1065                1070

Tyr Ser Ile Val Ser Gly Asn Glu Glu Asp Thr Phe Gly Ile Asn Asn
        1075                1080                1085

Ile Thr Gly Val Ile Tyr Val Asn Gly Pro Leu Asp Tyr Glu Thr Arg
    1090                1095                1100

Thr Ser Tyr Val Leu Arg Val Gln Ala Asp Ser Leu Glu Val Val Leu
1105                1110                1115                1120

Ala Asn Leu Arg Val Pro Ser Lys Ser Asn Thr Ala Lys Val Tyr Ile
                1125                1130                1135

Glu Ile Gln Asp Glu Asn Asn His Pro Pro Val Phe Gln Lys Lys Phe
            1140                1145                1150

Tyr Ile Gly Gly Val Ser Glu Asp Ala Arg Met Phe Thr Ser Val Leu
        1155                1160                1165

Arg Val Lys Ala Thr Asp Lys Asp Thr Gly Asn Tyr Ser Val Met Ala
    1170                1175                1180

Tyr Arg Leu Ile Ile Pro Pro Ile Lys Glu Gly Lys Glu Gly Phe Val
1185                1190                1195                1200

Val Glu Thr Tyr Thr Gly Leu Ile Lys Thr Ala Met Leu Phe His Asn

1205                 1210                 1215

Met Arg Arg Ser Tyr Phe Lys Phe Gln Val Ile Ala Thr Asp Asp Tyr
        1220                 1225                 1230

Gly Lys Gly Leu Ser Gly Lys Ala Asp Val Leu Val Ser Val Ser Val
    1235                 1240                 1245

Val Asn Gln Leu Asp Met Gln Val Ile Val Ser Asn Val Pro Pro Thr
    1250                 1255                 1260

Leu Val Glu Lys Lys Ile Glu Asp Leu Thr Glu Ile Leu Asp Arg Tyr
1265                 1270                 1275                 1280

Val Gln Glu Gln Ile Pro Gly Ala Lys Val Val Val Glu Ser Ile Gly
                1285                 1290                 1295

Ala Arg Arg His Gly Asp Ala Phe Ser Leu Glu Asp Tyr Thr Lys Cys
            1300                 1305                 1310

Asp Leu Thr Val Tyr Ala Ile Asp Pro Gln Thr Asn Arg Ala Ile Asp
        1315                 1320                 1325

Arg Asn Glu Leu Phe Lys Phe Leu Asp Gly Lys Leu Leu Asp Ile Asn
    1330                 1335                 1340

Lys Asp Phe Gln Pro Tyr Tyr Gly Glu Gly Gly Arg Ile Leu Glu Ile
1345                 1350                 1355                 1360

Arg Thr Pro Glu Ala Val Thr Ser Ile Lys Lys Arg Gly Glu Ser Leu
                1365                 1370                 1375

Gly Tyr Thr Glu Gly Ala Leu Leu Ala Leu Ala Phe Ile Ile Ile Leu
            1380                 1385                 1390

Cys Cys Ile Pro Ala Ile Leu Val Val Leu Val Ser Tyr Arg Gln Phe
        1395                 1400                 1405

Lys Val Arg Gln Ala Glu Cys Thr Arg Gln Ala Glu Cys Thr Lys Thr
    1410                 1415                 1420

Ala Arg Ile Gln Ala Ala Leu Pro Ala Ala Lys Pro Ala Val Pro Ala
1425                 1430                 1435                 1440

Pro Ala Pro Val Ala Ala Pro Pro Pro Pro Pro Pro Pro Pro Pro Gly
                1445                 1450                 1455

Ala His Leu Tyr Glu Glu Leu Gly Asp Ser Ser Met Ser Phe Leu Ser
            1460                 1465                 1470

Ser Leu Phe Leu Leu Tyr His Phe Gln Gln Ser Arg Gly Asn Asn Ser
        1475                 1480                 1485

Val Ser Glu Asp Arg Lys His Gln Gln Val Val Met Pro Phe Ser Ser
    1490                 1495                 1500

Asn Thr Ile Glu Ala His Lys Ser Ala His Val Asp Gly Ser Leu Lys
1505                 1510                 1515                 1520

Ser Asn Lys Leu Lys Ser Ala Arg Lys Phe Thr Phe Leu Ser Asp Glu
                1525                 1530                 1535

Asp Asp Leu Ser Ala His Asn Pro Leu Tyr Lys Glu Asn Ile Ser Gln
            1540                 1545                 1550

Val Ser Thr Asn Ser Asp Ile Ser Gln Arg Thr Asp Phe Val Asp Pro
        1555                 1560                 1565

Phe Ser Pro Lys Ile Gln Ala Lys Ser Lys Ser Leu Arg Gly Pro Arg

1570                 1575                1580

Glu Lys Ile Gln Arg Leu Trp Ser Gln Ser Val Ser Leu Pro Arg Arg
1585                1590                1595                1600

Leu Met Arg Lys Val Pro Asn Arg Pro Glu Ile Ile Asp Leu Gln Gln
                1605                1610                1615

Trp Gln Gly Thr Arg Gln Lys Ala Glu Asn Glu Asn Thr Gly Ile Cys
            1620                1625                1630

Thr Asn Lys Arg Gly Ser Ser Asn Pro Leu Leu Thr Thr Glu Glu Ala
        1635                1640                1645

Asn Leu Thr Glu Lys Glu Glu Ile Arg Gln Gly Glu Thr Leu Met Ile
    1650                1655                1660

Glu Gly Thr Glu Gln Leu Lys Ser Leu Ser Ser Asp Ser Ser Phe Cys
1665                1670                1675                1680

Phe Pro Arg Pro His Phe Ser Phe Ser Thr Leu Pro Thr Val Ser Arg
                1685                1690                1695

Thr Val Glu Leu Lys Ser Glu Pro Asn Val Ile Ser Ser Pro Ala Glu
            1700                1705                1710

Cys Ser Leu Glu Leu Ser Pro Ser Arg Pro Cys Val Leu His Ser Ser
        1715                1720                1725

Leu Ser Arg Arg Glu Thr Pro Ile Cys Met Leu Pro Ile Glu Thr Glu
    1730                1735                1740

Arg Asn Ile Phe Glu Asn Phe Ala His Pro Pro Asn Ile Ser Pro Ser
1745                1750                1755                1760

Ala Cys Pro Leu Pro Pro Pro Pro Pro Ile Ser Pro Pro Ser Pro Pro
                1765                1770                1775

Pro Ala Pro Ala Pro Leu Ala Pro Pro Pro Asp Ile Ser Pro Phe Ser
            1780                1785                1790

Leu Phe Cys Pro Pro Pro Ser Pro Pro Ser Ile Pro Leu Pro Leu Pro
        1795                1800                1805

Pro Pro Thr Phe Phe Pro Leu Ser Val Ser Thr Ser Gly Pro Pro Thr
    1810                1815                1820

Pro Pro Leu Leu Pro Pro Phe Pro Thr Pro Leu Pro Pro Pro Pro Pro
1825                1830                1835                1840

Ser Ile Pro Cys Pro Pro Pro Pro Ser Ala Ser Phe Leu Ser Thr Glu
                1845                1850                1855

Cys Val Cys Ile Thr Gly Val Lys Cys Thr Thr Asn Leu Met Pro Ala
            1860                1865                1870

Glu Lys Ile Lys Ser Ser Met Thr Gln Leu Ser Thr Thr Thr Val Cys
        1875                1880                1885

Lys Thr Asp Pro Gln Arg Glu Pro Lys Gly Ile Leu Arg His Val Lys
    1890                1895                1900

Asn Leu Ala Glu Leu Glu Lys Ser Val Ala Asn Met Tyr Ser Gln Ile
1905                1910                1915                1920

Glu Lys Asn Tyr Leu Arg Thr Asn Val Ser Glu Leu Gln Thr Met Cys
                1925                1930                1935

Pro Ser Glu Val Thr Asn Met Glu Ile Thr Ser Glu Gln Asn Lys Gly

1940                1945                1950

Ser Leu Asn Asn Ile Val Glu Gly Thr Glu Lys Gln Ser His Ser Gln
    1955                1960                1965

Ser Thr Ser Leu
    1970


<210> 21
<211> 13700
<212> DNA
<213> Homo sapiens

<400> 21
tctctctttc ttccctccag gatggaagta tgatgtgatg gatataatta tgggacactg 60
tgtgggcaca cggcctcctg cttgttgcct catcctcctg cttttcaagc ttttggccac 120
tgtctcccag gggctgccag ggactggacc cctgggcttc cacttcacac attccattta 180
taatgctacc gtgtatgaga actcagcagc aaggacctac gtcaacagcc agagtagaat 240
gggcatcacc ttaatagatc tatcctggga tatcaaatac agaatagtgt ccggagacga 300
ggaaggcttt ttcaaagcag aggaagtcat cattgcagat ttctgttttc tcagaataag 360
aactaaaggt ggcaattctg ccatattaaa tagggaaatc caggataatt atttattgat 420
agtaaaaggt tctgtcagag gagaggattt ggaagcatgg accaaagtga atatacaggt 480
tttagatatg aatgatctga gacctttgtt ttcacccaca acatactctg ttaccatagc 540
agaaagcaca cctctaagga ctagtgttgc ccaggtgact gcaacagacg cagatattgg 600
ttccaatgga gaattctact actactttaa aaataaagtt gatctctttt cagttcaccc 660
cacgagtggt gtcatctcct taagtggtcg attaaattat gatgaaaaga ataggtatga 720
tctggaaatt ttggctgtgg accgggggat gaaactgtat gggaacaatg gagtgagcag 780
tactgcaaag ctttatgttc acattgagcg cataaatgaa catgccccaa caatccatgt 840
agtcactcat gttcctttct cgttggaaaa agagccaaca tatgcagtgg tgacagttga 900
tgacttagat gatggagcga atggagagat cgaatctgtt tccattgtgg ctggggatcc 960
tttagatcag ttcttcctgg ctaaggaagg aaagtggttg aatgagtaca agattaagga 1020
gaggaagcag attgactggg agagctttcc ctatggctac aatctcactc ttcaagcaaa 1080
agacaaggga tctcctcaaa aatgttcagc attaaaggca gtctacattg gcaaccccac 1140
aagagacact gtccccatta gatttgaaaa agaagtgtac gatgtgagca taagtgaatt 1200
ttcccctcct ggtgtcgtgg ttgctatagt aaaattaagt cctgaaccga tagatgtgga 1260
atacaaatta tctcctggtg aggatgcagt gtactttaaa attaatcctc ggtcgggtct 1320
gattgttaca gcacggccac tgaatactgt taagaaggag gtttataaac tggaggtgac 1380
aaacaaggaa ggagatttaa aagcacaggt caccatcagc atagaagatg caaatgacca 1440
caccccagaa tttcagcaac cactgtatga tgcttatgtg aatgaaagtg tcccagtggg 1500
aaccagcgtt ctaacagttt cagcttctga taaggataaa ggagaaaatg ggtacatcac 1560
ctatagtatc gctagcctga atttgttacc atttgtcatt aatcagttta caggtgttat 1620
tagcacaact gaagaactgg attttgaatc ctccccagaa atttacagat tcattgttag 1680
agcctctgac tggggttcac cataccgcca tgaaagtgag gtcaatgtga ctattcgaat 1740
aggaaatgtc aacgacaaca gccctctctt tgaaaaagtg gcttgccagg gagttatttc 1800
atatgacttt ccagttggtg gtcacatcac agcagtctca gcgatcgata tcgatgaact 1860
tgaacttgta aagtacaaaa tcatttctgg aaatgaactt ggcttctttt atttaaaccc 1920
agattctggt gttttacagc ttaaaaaatc actgacaaat tctggcatta aaaatggcaa 1980
ttttgccctc agaattacag caactgatgg agagaatctt gcagacccca tgtctattaa 2040
catttcagtc ctacatggga aagtgtcttc aaagagcttc agttgcagag aaactcgtgt 2100
ggctcaaaag ctggcagaga aactactcat taaggcaaaa gcaaatggga aactgaatct 2160
ggaagatgga tttcttgact tttattcaat taatagacag ggaccatatt ttgacaagtc 2220
ttttccttct gatgtggctg taaaggagga tctgccagtt ggtgctaaca ttctgaagat 2280
taaagcctat gatgccgact ctggcttcaa tggaaaagtg ctatttacaa tatcagatgg 2340
aaatacggat agttgcttta atattgatat ggagactggg cagcttaaag tccttatgcc 2400
catggatcga gaacacacag acctctatct ccttaatatc accatctatg acttaggtaa 2460
tccacagaaa tcgtcatgga gactgctgac catcaatgtg gaggatgcta atgacaatag 2520
cccagttttt attcaagaca gttactcagt taacattctt gaaagttcag gcattggtac 2580
tgaaatcatt caagtggaag ccagagacaa agacttaggt tctaatggtg aagtgactta 2640
ctcagtcttg acagatacac agcagtttgc catcaatagc tcaactggaa tcgtttatgt 2700
agccgaccag ttggaccggg aatccaaagc caattattct ttgaaaatag aagccaggga 2760
caaggcagag agtggtcagc agctgttttc agttgtcact cttaaagttt ttttagatga 2820
tgtcaatgac tgctccccag ctttcattcc cagtagctat agtgtgaagg ttcttgaaga 2880
tctccctgtt ggcactgtca ttgcttggct tgagacccat gatccagatc ttggactggg 2940
gggtcaagtg cgctattctt tggtcaatga ctataatggg agatttgaaa tagataaagc 3000
aagtggtgcc atccgcttga gcaaagagct tgattatgag aaacagcagt tctataacct 3060
tactgtgcgg gccaaagaca aagggcggcc tgtctctctg tcatctgttt cctttgttga 3120
ggtggaagtg gtggatgtca atgaaaacct ccacactccc tatttcccag actttgctgt 3180
tgttggatct gtaaaggaaa actcacgcat tggaacaagc gtgctgcagg tgactgctcg 3240

agatgaagac tccggaaggg atggagagat ccagtactcc atcagggatg gcagtggtct 3300
tggaaggttc agtatagacg acgagagtgg ggtcatcact gccgcagaca ttcttgatcg 3360
ggagacaatg gggtcatact ggctaacagt gtatgccaca gacaggggcg ttgttccact 3420
ctactccacc attgaggtct acattgaagt tgaagatgtg aatgacaatg ccccgctgac 3480
ctcagaaccc ctatatatta tcctggtcat ggataaacat ccgaaggacg tatctgtcat 3540
tcagatccag gctgaagatc ctgactccag ttccaatgaa aaactgacat acaggattac 3600
aagtggaaat cctcagaatt ttttgtgcat caatatcaaa acagggctga ttacaacaac 3660
ttcaaggaaa ttggatcgag aacagcaggc agaacatttt ctggaggtga ctgtgacaga 3720
tggtggtccc tctccaaaac agtcaaccat ttgggtggtg gttcaggttc tagatgaaaa 3780
tgacaacaag ccccagttcc cagagaaggt ctaccagatc aagctgccag aacgtgaccg 3840
aaagaagaga ggagaaccga tttacagggc ttttgcattt gatagagatg agggccccaa 3900
cgcagaaatc tcctacagta ttgtggatgg gaatgatgac ggaaagttct ttattgaccc 3960
taaaactggg atggtttctt ctagaaagca gtttacagca ggcagttatg acatcctaac 4020
gataaaggca gtggacaatg ggcgcccaca gaaatcctcc acggcccgcc tccacattga 4080
atggattaag aaaccacccc cttcacctat accattgacc ttcgatgagc cgttttataa 4140
cttcacagtc atggaaagtg atagagtgac tgaaattgta ggggtggtgt ctgtgcagcc 4200
agctaacacc cctctgtggt ttgacatagt tgggggggaat tttgacagcg cttttgatgc 4260
agagaagggt gttgggacaa ttgtcatcgc aaaacctttg gatgcagagc agaggtccat 4320
ctataatatg agtgtggaag tcaccgatgg gacaaatgtt gctgttactc aggtatttat 4380
caaagtgctg gataataatg ataatggccc agaattctct cagccgaatt acgatgtgac 4440
aatttccgag gatgtgcttc cagacacgga gatcctgcag attgaagcca cagatagaga 4500
tgagaagcac aagctgagct acactgttca tagcagcatc gactccatca gcatgagaaa 4560
attccggatt gaccctagca ctggcgtgct ctatactgcc gagaggctgg accatgaggc 4620
ccaggacaag cacattctca acataatggt cagagatcag gagtttcctt atcgaagaaa 4680
cttggcccga gtcattgtga atgtggagga tgctaatgat cacagtcctt attttaccaa 4740
cccactgtat gaagcgtctg tgtttgaatc tgctgctctg ggatcagctg ttctgcaagt 4800
gacggctctg gacaaagaca aaggagaaaa tgcagaactc atatatacca tagaagcagg 4860
gaacactggg aacatgttta agatcgaacc ggtcctaggc atcatcacca tttgcaaaga 4920
accagacatg acgacgatgg gtcagtttgt cctatccatc aaagtcacag atcagggatc 4980
cccgccaatg tctgctactg caattgtgcg catttccgtc accatgtctg acaattctca 5040
ccccaagttc attcacaaag actaccaagc agaagtaaat gaaaatgttg acattggaac 5100
atcagtcatt ctaatctctg ccatcagtca atctaccctc atttatgaag tcaaagatgg 5160
agacattaat gggatcttta ccataaatcc atattctgga gtcatcacca ctcagaaggc 5220
cctggattat gagcgcacat cctcttatca actcatcatt caggccacca atatggcagg 5280
aatggcttcc aatgctacag tcaatattca gattgttgat gaaaatgata atgccccagt 5340
ttttctcttt tctcaatact caggcagcct aagtgaggct gccccaatta atagcattgt 5400
caggagcttg gataacagcc cactggtgat tcgagccaca gatgctgaca gcaaccggaa 5460
tgctctgctt gtgtatcaga ttgtggagtc aacagcaaaa aagtttttca cggtggactc 5520
cagtacaggt gcaatcagaa caattgccaa cctggaccat gaaaccattg cccatttcca 5580
ttttcatgtg catgtgagag acagtggtag cccccaactg actgcagaga gtcccgttga 5640
agtcaacatt gaggtgacag atgtgaatga taacccacct gtttttactc aggctgtgtt 5700
tgagactatc ttacttctac ctacctatgt tggagtggag gttctgaaag ttagtgccac 5760
agatcctgac tctgaggtac cccctgaact gacatacagc ctaatggaag gcagtttgga 5820
tcatttttta attgactcaa acagtggagt acttaccata aaaaacaaca acctctccaa 5880
ggatcactac atgctgatag ttaaggtgtc tgatggaaag ttctacagta cctccatggt 5940
caccatcatg gttaaagaag ccatggacag cggcctccac tttacacaaa gcttctattc 6000
cacctcaatc tcagagaaca acactaacat aaccaaagtt gctattgtca atgcagttgg 6060
aaatcgcctt aatgagccct taaaatacag catcttaaac ccaggaaata agttcaagat 6120
aaaatctacc tcaggggtca ttcagacgac tggagtcccc tttgaccgtg aagaacaaga 6180
gttatatgag ctggtggtag aagccagccg tgagctggac catctgcgtg tggccagagt 6240
ggtggtcagg gttaacattg aagacataaa tgacaattct ccagtctttg tgggcctccc 6300
atactatgct gctgttcaag tggatgcgga acccggactc ctgatttatc aggtgacagc 6360
cattgacaaa gataaaggtc caaatggaga agtgacctat gtcctgcagg atgactatgg 6420
ccactttgaa attaacccta attcagggaa tgttatttta aaggaagcat tcaactctga 6480
cttgtccaac attgagtatg gagtcaccat cctagccaag gatggcggaa aaccttcttt 6540
gtctacatct gtggagcttc ccatcactat tgtcaacaaa gcaatgcctg tgtttgataa 6600
gcccttttat acagcatctg tcaatgaaga catcagaatg aacacaccca tcctaagcat 6660
caatgccacc agtccagaag gccaaggcat catatatatc attatcgatg ggaccctttt 6720
taaacagttt aacattgact ttgacactgg ggtcctgaaa gttgttagcc ctttggatta 6780
tgaagttaca tctgcttaca agctgacaat aagagccagc gacgccctta ctggtgctag 6840
ggctgaagtc actgttgact tgctagttaa tgatgtaaat gacaaccccc ctattttcga 6900
tcagcctaca tacaatacaa cactatcaga agcatctctt attgggacac ctgttttaca 6960
agttgtctct attgatgcag actcagaaaa caataaaatg gtacattatc agattgtcca 7020
ggatacctac aatagcacag attattttca catagatagc tcaagtggct taatcctgac 7080
agcacgaatg ctggaccatg agttagtaca acactgcact ttgaaagtca gatcaataga 7140
tagtggcttc ccatcactga gcagtgaggt tctcgttcat atctacatct ctgatgtaaa 7200
tgacaaccct ccagttttta atcagctcat ttatgagtca tatgtgagtg aattagcccc 7260
ccggggccat tttgtaacct gtgtacaagc ctctgatgca gacagctctg attttgaccg 7320
gttggaatat agcattttat ctgggaatga ccggacgagc tttctgatgg acagcaagag 7380

tggagttatc acattgtcca accatcggaa gcagcggatg gagcctctgt acagtctcaa 7440
tgtgtctgtc tctgatgggt tgttcaccag cactgcacag gtgcatatta gggtacttgg 7500
ggctaacttg tacagccctg ccttttcaca aagcacatac gtagctgagg tgagagagaa 7560
cgtggctgca ggaacaaagg taattcatgt tcgagccaca gatggtgatc cagggactta 7620
tgggcagatc agctatgcca tcatcaatga ctttgccaag gatcgattcc tcatagacag 7680
caatgggcag gtcatcacca cagaaaggct agaccgggaa aaccctctag aaggggatgt 7740
tagtattttt gtgagggccc ttgatggtgg agggagaaca actttctgca ctgtgagagt 7800
gattgttgtg gatgaaaatg acaatgctcc ccagttcatg acagtggaat atagagccag 7860
tgtcagggca gatgttggaa ggggccactt ggtcactcaa gttcaagcca tagatcccga 7920
tgatggagca aattcaagga ttacttattc cctctatagc gaggcctctg tttcagtggc 7980
cgacctcctg gaaatcgatc ctgacaatgg ctggatggtc acaaagggta attttaacca 8040
gctgaaaaat acagtgcttt cgttctttgt caaagcagta gatgggggca tcccagtaaa 8100
gcactccctc attcctgtct atatccacgt cttgcccccct gaaacgttct tgccatcatt 8160
cacccagtct cagtattcct ttaccattgc agaagataca gccattggga gtacagtgga 8220
caccctgagg attttgccca gtcagaatgt ctggttcagc acagttaatg gggaacggcc 8280
agaaaataac aaagggggca tattcgtcat agaacaggaa acaggcacta ttaagcttga 8340
caaacgcctt gaccgtgaaa ccagcccagc tttccacttt aaagtagcag ccactatacc 8400
cctggacaaa gtagacattg tgtttactgt ggatgtagat atcaaggtat tggatttgaa 8460
tgacaacaag ccagtctttg aaacttcaag ctatgacacc attataatgg aagggatgcc 8520
tgttggcacc aaactcacac aagtgagagc tattgatatg gactggggag ccaatggaca 8580
agtcacttac tccctccact cggattccca gcccgaaaag gtaatggaag cattcaatat 8640
tgacagcaac acgggctgga tcagtacctt gaaggaccta gatcacgaga cagaccccac 8700
attcaccttc tctgtggtgg cctctgacct tggagaggca ttctctcttt cctccacggc 8760
cttggtctct gtcagagtga cagatataaa tgacaatgca ccagtcttcg cgcaggaagt 8820
gtaccgaggg aatgtgaagg agagcgaccc accgggcgag gtggtagccg tcctcagcac 8880
ctgggacaga gacacatccg acgttaatcg ccaagtgagc taccatatta caggtggaaa 8940
ccctcgagga aggtttgctc tgggcctggt gcaaagtgag tggaaggtct atgtgaagag 9000
gcctctagac agagaagaac aggacattta ctttctcaat atcactgcca ctgatgggct 9060
ttttgtcaca caggccatgg tggaagtgag cgtcagtgat gtgaatgaca atagcccagt 9120
gtgtgatcag gttgcatata cagcattact tcctgaagac attccatcaa ataaaatcat 9180
cctgaaagtc agtgcaaagg atgctgatat tggatccaat ggatatatac gatactcact 9240
ctatggatct ggaaacagtg aatttttct agatccagaa agtggcgagt taaaaacctt 9300
ggctctgttg gaccgggaga ggatccccgt gtacagcctg atggccaagg ccactgacgg 9360
gggtggcagg ttctgccagt ccaacatcca cctaatcctg gaggatgtga atgataaccc 9420
ccctgtgttt tcttctgacc actacaacac ctgtgtctat gagaacacag ccaccaaggc 9480
tctgttgacc agagttcaag ccgtggaccc cgacattggc atcaatagga aggtcgtgta 9540
ctccctggca gactcagctg gtggggtctt ctccattgac agctcatctg gcatcatcat 9600
cctggagcag ccactggacc gtgagcagca gtcttcgtac aacatcagcg tgcgggccac 9660
tgaccagagt cctggacagt ccctgtcctc tctcactact gtcaccatca ccgttctgga 9720
cattaatgac aacccccctg tgtttgagag gagggactac ctggtgacgg tgcctgagga 9780
cacctcccct ggcacccaag tccttgctgt ttttgccacc agcaaagata ttggcacaaa 9840
tgctgagatc acttatctca tccggtctgg gaacgaacaa gggaaattta agatcaaccc 9900
caagacaggg ggtatttctg tctctgaagt cctggactat gaattatgca aaaggtttta 9960
cctggtagtg gaagccaaag atgggggcac cccagctctc agcgctgtgg ccactgtcaa 10020
catcaacctc acagatgtta atgacaaccc tcccaagttc agccaagacg tctacagtgc 10080
ggttatcagt gaagacgcct tggtgggaga ctctgtcatt ttgctaatag cagaagatgt 10140
agacagccag cccaacggac agattcattt ttccattgtg aatggagatc gggacaatga 10200
atttactgta gatcctgtct tgggacttgt gaaagttaag aagaaattgg accgggaacg 10260
ggtgtctgga tactctctgc ttgtccaggc cgtagacagt ggcattcctg caatgtcatc 10320
aactgcaact gtcaacattg atatttctga tgtgaatgac aacagcccgg tgtttacacc 10380
tgccaactat actgctgtga ttcaggaaaa taagccagtg ggcaccagca tcttgcagct 10440
ggtggtgaca gacagagact cctttcacaa tgggcctccc ttttcattct ctattttgtc 10500
gggaaatgaa gaggaggagt ttgtgttgga ccctcatggg atcttgcggt cggctgtggt 10560
cttccagcac acagagtctc tggaatacgt gttgtgtgtc caggcaaagg attcaggcaa 10620
accccagcaa gtttctcaca cttacatccg cgtgcgagtc attgaggaaa gcacccacaa 10680
gcccacagcc attcccctgg aaattttcat tgtcaccatg gaggatgact ttcctggtgg 10740
ggtcattggg aagattcatg ccacagatca agacatgtat gatgtgctca catttgccct 10800
gaaatcggag cagaaaagct tatttaaagt gaacagtcac gatgggaaaa tcatcgccct 10860
gggaggcctg gacagcggca agtatgtcct gaatgtgtct gtgagtgatg gtcgcttcca 10920
ggtacccatt gatgtggtcg tgcatgtgga gcagttggtg catgagatgc tgcagaacac 10980
tgtcaccatc cgctttgaaa atgtgtcccc tgaggacttc gtggggctgc acatgcatgg 11040
gttccggcgc accctgcgga atgcagtcct cacccagaag caggacagcc tgcgcatcat 11100
cagcatccag cccgtggcag gcaccaacca actggacatg ctgtttgcgg tggagatgca 11160
cagcagcgag ttctacaagc cagcctacct gatccagaag ctgtccaatg ctagaagaca 11220
cctggagaat atcatgcgca tctcagccat cttggagaag aactgctcag ggctggactg 11280
tcaggaacag cattgtgagc aaggcttgtc actcgattcc cacgcgctca tgacctacag 11340
cacggctcgc atcagctttg tgtgtccgcg tttctacagg aacgtgcgtt gcacctgcaa 11400
tggtggactg tgtccggggt ccaacgatcc ttgtgtggag aagccgtgtc caggggacat 11460
gcagtgtgtc agttatgaag ccagcaggag accgttcctc tgccagtgtc caccagggaa 11520

gctcggagag tgctcagggc acacttctct cagctttgct ggaaacagtt acatcaaata 11580
tcggctttct gaaaatagca aagaagagga tttcaaacta gctctgcgtc ttcgaacact 11640
gcaaagcaat gggattataa tgtacaccag agcaaatccc tgcataattc tgaagcagat 11700
tgtggatggc aagctgtggt tccagctgga ctgcggcagc ggccctggaa tcttgggcat 11760
ctcgggccgt gctgtcaacg acgggagctg gcactcggtc ttcctggagc tcaaccgcaa 11820
tttcacgagc ctgtccctgg atgacagcta cgtggagcgg cgccgggcgc ccctctactt 11880
ccagacgctg agcactgaga gtagcatcta cttcggcgcc ctggtgcaag cggataacat 11940
ccgcagcctg actgacacgc gggtcacgca ggtgctcagc ggcttccagg gctgcctgga 12000
ctcggtgata ctgaataaca atgagctgcc gctgcagaac aagcgcagca gcttcgcgga 12060
ggtggtgggc ctgacggagc tgaagctggg ctgcgtgctc tatcccgacg cctgcaagcg 12120
cagcccgtgc cagcacgggg gcagctgcac tggcctgcca tcggggggtg gctatcagtg 12180
tacctgtctc tcacagttta cggggagaaa ctgtgaatct gagattacag cctgcttccc 12240
aaacccctgc cggaatggag gatcctgcga tccaatagga aacactttca tctgcaattg 12300
taaagctggg ctcactggag tcacgtgtga ggaggacatc aatgagtgcg aacgagagga 12360
gtgtgagaac ggaggctcct gcgtgaacgt gttcggctcc ttcctctgca actgcacgcc 12420
gggctacgtg ggccagtact gcgggctgcg ccccgtggtg gtacccaata tccaggctgg 12480
ccactcctac gtggggaagg aggagctcat cggcatcgcc gtggtcctct tcgtcatctt 12540
catcctggtg gttctcttca tagtcttccg caagaaggtc ttccgcaaga actactcccg 12600
caacaacatc acgctagtgc aggacccggc caccgccgcc ctgcttaaca agagcaatgg 12660
catcccgttc cggaacctgc gcggcagtgg ggacggccgc aacgtctacc aggaggtggg 12720
gcccccgcag gtccccgtgc gccccatggc ctacacaccc tgcttccaga gtgactccag 12780
gagcaacctg gataagatcg tggacgggct gggaggcgag caccaggaaa tgaccacgtt 12840
tcaccctgag tcgccccgca tcctgacagc ccggcggggc gtggtcgtgt gcagtgtggc 12900
ccccaacctc cccgccgtgt caccctgccg ctccgactgc gactccatcc ggaagaatgg 12960
ctgggacgcg ggaactgaga gtaataaagg cagcaactct gaagttcagt ccctcagctc 13020
cttccagtca gattctggtg acgacaatgc ctatcactgg gacacctctg attggatgcc 13080
aggggcccgc ctgtcggaca tagaggaagt gcccaactat gagaaccagg atggagggtc 13140
tgcacaccag gggagcacac gggagctgga gagcgattac tacctgggtg gttatgacat 13200
tgacagtgaa tacccacccc ctcatgaaga ggagttcttg agtcaggacc agctgcctcc 13260
tcctctcccg gaggacttcc cagaccaata tgaggccctg ccaccctccc agcctgtctc 13320
cctggccagc acactgagcc cagactgcag gagaaggccc cagtttcatc ctagccagta 13380
tctccctcct cacccattcc ccaacgaaac ggatttggtg ggcccgcctg ccagctgtga 13440
atttagtact tttgctgtga gcatgaacca gggcacagag cccacaggcc cagcagacag 13500
cgtgtctctg tccttgcaca attccagagg cacctcatcc tcggatgtgt ctgccaactg 13560
cggctttgac gattccgaag tagccatgag tgactacgag agcgtgggag agctcagcct 13620
cgccagcctt cacattccct ttgtggagac tcagcatcag actcaagtgt agacatcaca 13680
tcttgggtac ttcaccctgt 13700

<210> 22
<211> 4544
<212> PRT
<213> Homo sapiens

<400> 22
Met Asp Ile Ile Met Gly His Cys Val Gly Thr Arg Pro Pro Ala Cys
1               5                   10                  15

Cys Leu Ile Leu Leu Leu Phe Lys Leu Leu Ala Thr Val Ser Gln Gly
            20                  25                  30

Leu Pro Gly Thr Gly Pro Leu Gly Phe His Phe Thr His Ser Ile Tyr
        35                  40                  45

Asn Ala Thr Val Tyr Glu Asn Ser Ala Ala Arg Thr Tyr Val Asn Ser
    50                  55                  60

Gln Ser Arg Met Gly Ile Thr Leu Ile Asp Leu Ser Trp Asp Ile Lys
65                  70                  75                  80

Tyr Arg Ile Val Ser Gly Asp Glu Glu Gly Phe Phe Lys Ala Glu Glu
                85                  90                  95

Val Ile Ile Ala Asp Phe Cys Phe Leu Arg Ile Arg Thr Lys Gly Gly
            100                 105                 110

Asn Ser Ala Ile Leu Asn Arg Glu Ile Gln Asp Asn Tyr Leu Leu Ile
        115                 120                 125

Val Lys Gly Ser Val Arg Gly Glu Asp Leu Glu Ala Trp Thr Lys Val
    130                 135                 140
Asn Ile Gln Val Leu Asp Met Asn Asp Leu Arg Pro Leu Phe Ser Pro
145                 150                 155                 160
Thr Thr Tyr Ser Val Thr Ile Ala Glu Ser Thr Pro Leu Arg Thr Ser
                165                 170                 175
Val Ala Gln Val Thr Ala Thr Asp Ala Asp Ile Gly Ser Asn Gly Glu
            180                 185                 190
Phe Tyr Tyr Tyr Phe Lys Asn Lys Val Asp Leu Phe Ser Val His Pro
        195                 200                 205
Thr Ser Gly Val Ile Ser Leu Ser Gly Arg Leu Asn Tyr Asp Glu Lys
    210                 215                 220
Asn Arg Tyr Asp Leu Glu Ile Leu Ala Val Asp Arg Gly Met Lys Leu
225                 230                 235                 240
Tyr Gly Asn Asn Gly Val Ser Ser Thr Ala Lys Leu Tyr Val His Ile
                245                 250                 255
Glu Arg Ile Asn Glu His Ala Pro Thr Ile His Val Val Thr His Val
            260                 265                 270
Pro Phe Ser Leu Glu Lys Glu Pro Thr Tyr Ala Val Val Thr Val Asp
        275                 280                 285
Asp Leu Asp Asp Gly Ala Asn Gly Glu Ile Glu Ser Val Ser Ile Val
    290                 295                 300
Ala Gly Asp Pro Leu Asp Gln Phe Phe Leu Ala Lys Glu Gly Lys Trp
305                 310                 315                 320
Leu Asn Glu Tyr Lys Ile Lys Glu Arg Lys Gln Ile Asp Trp Glu Ser
                325                 330                 335
Phe Pro Tyr Gly Tyr Asn Leu Thr Leu Gln Ala Lys Asp Lys Gly Ser
            340                 345                 350
Pro Gln Lys Cys Ser Ala Leu Lys Ala Val Tyr Ile Gly Asn Pro Thr
        355                 360                 365
Arg Asp Thr Val Pro Ile Arg Phe Glu Lys Glu Val Tyr Asp Val Ser
    370                 375                 380
Ile Ser Glu Phe Ser Pro Pro Gly Val Val Val Ala Ile Val Lys Leu
385                 390                 395                 400
Ser Pro Glu Pro Ile Asp Val Glu Tyr Lys Leu Ser Pro Gly Glu Asp
                405                 410                 415
Ala Val Tyr Phe Lys Ile Asn Pro Arg Ser Gly Leu Ile Val Thr Ala
            420                 425                 430
Arg Pro Leu Asn Thr Val Lys Lys Glu Val Tyr Lys Leu Glu Val Thr
        435                 440                 445
Asn Lys Glu Gly Asp Leu Lys Ala Gln Val Thr Ile Ser Ile Glu Asp
    450                 455                 460
Ala Asn Asp His Thr Pro Glu Phe Gln Gln Pro Leu Tyr Asp Ala Tyr
465                 470                 475                 480
Val Asn Glu Ser Val Pro Val Gly Thr Ser Val Leu Thr Val Ser Ala
                485                 490                 495

Ser Asp Lys Asp Lys Gly Glu Asn Gly Tyr Ile Thr Tyr Ser Ile Ala
            500                 505                 510

Ser Leu Asn Leu Leu Pro Phe Val Ile Asn Gln Phe Thr Gly Val Ile
        515                 520                 525

Ser Thr Thr Glu Glu Leu Asp Phe Glu Ser Ser Pro Glu Ile Tyr Arg
    530                 535                 540

Phe Ile Val Arg Ala Ser Asp Trp Gly Ser Pro Tyr Arg His Glu Ser
545                 550                 555                 560

Glu Val Asn Val Thr Ile Arg Ile Gly Asn Val Asn Asp Asn Ser Pro
                565                 570                 575

Leu Phe Glu Lys Val Ala Cys Gln Gly Val Ile Ser Tyr Asp Phe Pro
            580                 585                 590

Val Gly Gly His Ile Thr Ala Val Ser Ala Ile Asp Ile Asp Glu Leu
        595                 600                 605

Glu Leu Val Lys Tyr Lys Ile Ile Ser Gly Asn Glu Leu Gly Phe Phe
    610                 615                 620

Tyr Leu Asn Pro Asp Ser Gly Val Leu Gln Leu Lys Lys Ser Leu Thr
625                 630                 635                 640

Asn Ser Gly Ile Lys Asn Gly Asn Phe Ala Leu Arg Ile Thr Ala Thr
                645                 650                 655

Asp Gly Glu Asn Leu Ala Asp Pro Met Ser Ile Asn Ile Ser Val Leu
            660                 665                 670

His Gly Lys Val Ser Ser Lys Ser Phe Ser Cys Arg Glu Thr Arg Val
        675                 680                 685

Ala Gln Lys Leu Ala Glu Lys Leu Leu Ile Lys Ala Lys Ala Asn Gly
    690                 695                 700

Lys Leu Asn Leu Glu Asp Gly Phe Leu Asp Phe Tyr Ser Ile Asn Arg
705                 710                 715                 720

Gln Gly Pro Tyr Phe Asp Lys Ser Phe Pro Ser Asp Val Ala Val Lys
                725                 730                 735

Glu Asp Leu Pro Val Gly Ala Asn Ile Leu Lys Ile Lys Ala Tyr Asp
            740                 745                 750

Ala Asp Ser Gly Phe Asn Gly Lys Val Leu Phe Thr Ile Ser Asp Gly
        755                 760                 765

Asn Thr Asp Ser Cys Phe Asn Ile Asp Met Glu Thr Gly Gln Leu Lys
    770                 775                 780

Val Leu Met Pro Met Asp Arg Glu His Thr Asp Leu Tyr Leu Leu Asn
785                 790                 795                 800

Ile Thr Ile Tyr Asp Leu Gly Asn Pro Gln Lys Ser Ser Trp Arg Leu
                805                 810                 815

Leu Thr Ile Asn Val Glu Asp Ala Asn Asp Asn Ser Pro Val Phe Ile
            820                 825                 830

Gln Asp Ser Tyr Ser Val Asn Ile Leu Glu Ser Ser Gly Ile Gly Thr
        835                 840                 845

Glu Ile Ile Gln Val Glu Ala Arg Asp Lys Asp Leu Gly Ser Asn Gly
    850                 855                 860

Glu Val Thr Tyr Ser Val Leu Thr Asp Thr Gln Gln Phe Ala Ile Asn
865                 870                 875                 880

Ser Ser Thr Gly Ile Val Tyr Val Ala Asp Gln Leu Asp Arg Glu Ser
                885                 890                 895

Lys Ala Asn Tyr Ser Leu Lys Ile Glu Ala Arg Asp Lys Ala Glu Ser
            900                 905                 910

Gly Gln Gln Leu Phe Ser Val Val Thr Leu Lys Val Phe Leu Asp Asp
        915                 920                 925

Val Asn Asp Cys Ser Pro Ala Phe Ile Pro Ser Ser Tyr Ser Val Lys
    930                 935                 940

Val Leu Glu Asp Leu Pro Val Gly Thr Val Ile Ala Trp Leu Glu Thr
945                 950                 955                 960

His Asp Pro Asp Leu Gly Leu Gly Gly Gln Val Arg Tyr Ser Leu Val
                965                 970                 975

Asn Asp Tyr Asn Gly Arg Phe Glu Ile Asp Lys Ala Ser Gly Ala Ile
            980                 985                 990

Arg Leu Ser Lys Glu Leu Asp Tyr Glu Lys Gln Gln Phe Tyr Asn Leu
        995                 1000                1005

Thr Val Arg Ala Lys Asp Lys Gly Arg Pro Val Ser Leu Ser Ser Val
    1010                1015                1020

Ser Phe Val Glu Val Glu Val Val Asp Val Asn Glu Asn Leu His Thr
1025                1030                1035                1040

Pro Tyr Phe Pro Asp Phe Ala Val Val Gly Ser Val Lys Glu Asn Ser
                1045                1050                1055

Arg Ile Gly Thr Ser Val Leu Gln Val Thr Ala Arg Asp Glu Asp Ser
            1060                1065                1070

Gly Arg Asp Gly Glu Ile Gln Tyr Ser Ile Arg Asp Gly Ser Gly Leu
        1075                1080                1085

Gly Arg Phe Ser Ile Asp Asp Glu Ser Gly Val Ile Thr Ala Ala Asp
    1090                1095                1100

Ile Leu Asp Arg Glu Thr Met Gly Ser Tyr Trp Leu Thr Val Tyr Ala
1105                1110                1115                1120

Thr Asp Arg Gly Val Val Pro Leu Tyr Ser Thr Ile Glu Val Tyr Ile
                1125                1130                1135

Glu Val Glu Asp Val Asn Asp Asn Ala Pro Leu Thr Ser Glu Pro Leu
            1140                1145                1150

Tyr Ile Ile Leu Val Met Asp Lys His Pro Lys Asp Val Ser Val Ile
        1155                1160                1165

Gln Ile Gln Ala Glu Asp Pro Asp Ser Ser Ser Asn Glu Lys Leu Thr
    1170                1175                1180

Tyr Arg Ile Thr Ser Gly Asn Pro Gln Asn Phe Leu Cys Ile Asn Ile
1185                1190                1195                1200

Lys Thr Gly Leu Ile Thr Thr Thr Ser Arg Lys Leu Asp Arg Glu Gln
                1205                1210                1215

Gln Ala Glu His Phe Leu Glu Val Thr Val Thr Asp Gly Gly Pro Ser
            1220                1225                1230

Pro Lys Gln Ser Thr Ile Trp Val Val Val Gln Val Leu Asp Glu Asn
        1235                1240                1245

Asp Asn Lys Pro Gln Phe Pro Glu Lys Val Tyr Gln Ile Lys Leu Pro
    1250                1255                1260

Glu Arg Asp Arg Lys Lys Arg Gly Glu Pro Ile Tyr Arg Ala Phe Ala
1265                1270                1275                1280

Phe Asp Arg Asp Glu Gly Pro Asn Ala Glu Ile Ser Tyr Ser Ile Val
                1285                1290                1295

Asp Gly Asn Asp Asp Gly Lys Phe Phe Ile Asp Pro Lys Thr Gly Met
            1300                1305                1310

Val Ser Ser Arg Lys Gln Phe Thr Ala Gly Ser Tyr Asp Ile Leu Thr
        1315                1320                1325

Ile Lys Ala Val Asp Asn Gly Arg Pro Gln Lys Ser Ser Thr Ala Arg
    1330                1335                1340

Leu His Ile Glu Trp Ile Lys Lys Pro Pro Pro Ser Pro Ile Pro Leu
1345                1350                1355                1360

Thr Phe Asp Glu Pro Phe Tyr Asn Phe Thr Val Met Glu Ser Asp Arg
                1365                1370                1375

Val Thr Glu Ile Val Gly Val Val Ser Val Gln Pro Ala Asn Thr Pro
            1380                1385                1390

Leu Trp Phe Asp Ile Val Gly Gly Asn Phe Asp Ser Ala Phe Asp Ala
        1395                1400                1405

Glu Lys Gly Val Gly Thr Ile Val Ile Ala Lys Pro Leu Asp Ala Glu
    1410                1415                1420

Gln Arg Ser Ile Tyr Asn Met Ser Val Glu Val Thr Asp Gly Thr Asn
1425                1430                1435                1440

Val Ala Val Thr Gln Val Phe Ile Lys Val Leu Asp Asn Asn Asp Asn
                1445                1450                1455

Gly Pro Glu Phe Ser Gln Pro Asn Tyr Asp Val Thr Ile Ser Glu Asp
            1460                1465                1470

Val Leu Pro Asp Thr Glu Ile Leu Gln Ile Glu Ala Thr Asp Arg Asp
        1475                1480                1485

Glu Lys His Lys Leu Ser Tyr Thr Val His Ser Ser Ile Asp Ser Ile
    1490                1495                1500

Ser Met Arg Lys Phe Arg Ile Asp Pro Ser Thr Gly Val Leu Tyr Thr
1505                1510                1515                1520

Ala Glu Arg Leu Asp His Glu Ala Gln Asp Lys His Ile Leu Asn Ile
                1525                1530                1535

Met Val Arg Asp Gln Glu Phe Pro Tyr Arg Arg Asn Leu Ala Arg Val
            1540                1545                1550

Ile Val Asn Val Glu Asp Ala Asn Asp His Ser Pro Tyr Phe Thr Asn
        1555                1560                1565

Pro Leu Tyr Glu Ala Ser Val Phe Glu Ser Ala Ala Leu Gly Ser Ala
    1570                1575                1580

Val Leu Gln Val Thr Ala Leu Asp Lys Asp Lys Gly Glu Asn Ala Glu
1585                1590                1595                1600

Leu Ile Tyr Thr Ile Glu Ala Gly Asn Thr Gly Asn Met Phe Lys Ile
                1605                1610                1615

Glu Pro Val Leu Gly Ile Ile Thr Ile Cys Lys Glu Pro Asp Met Thr
            1620                1625                1630

Thr Met Gly Gln Phe Val Leu Ser Ile Lys Val Thr Asp Gln Gly Ser
        1635                1640                1645

Pro Pro Met Ser Ala Thr Ala Ile Val Arg Ile Ser Val Thr Met Ser
    1650                1655                1660

Asp Asn Ser His Pro Lys Phe Ile His Lys Asp Tyr Gln Ala Glu Val
1665                1670                1675                1680

Asn Glu Asn Val Asp Ile Gly Thr Ser Val Ile Leu Ile Ser Ala Ile
                1685                1690                1695

Ser Gln Ser Thr Leu Ile Tyr Glu Val Lys Asp Gly Asp Ile Asn Gly
            1700                1705                1710

Ile Phe Thr Ile Asn Pro Tyr Ser Gly Val Ile Thr Thr Gln Lys Ala
        1715                1720                1725

Leu Asp Tyr Glu Arg Thr Ser Ser Tyr Gln Leu Ile Ile Gln Ala Thr
    1730                1735                1740

Asn Met Ala Gly Met Ala Ser Asn Ala Thr Val Asn Ile Gln Ile Val
1745                1750                1755                1760

Asp Glu Asn Asp Asn Ala Pro Val Phe Leu Phe Ser Gln Tyr Ser Gly
                1765                1770                1775

Ser Leu Ser Glu Ala Ala Pro Ile Asn Ser Ile Val Arg Ser Leu Asp
            1780                1785                1790

Asn Ser Pro Leu Val Ile Arg Ala Thr Asp Ala Asp Ser Asn Arg Asn
        1795                1800                1805

Ala Leu Leu Val Tyr Gln Ile Val Glu Ser Thr Ala Lys Lys Phe Phe
    1810                1815                1820

Thr Val Asp Ser Ser Thr Gly Ala Ile Arg Thr Ile Ala Asn Leu Asp
1825                1830                1835                1840

His Glu Thr Ile Ala His Phe His Phe His Val His Val Arg Asp Ser
                1845                1850                1855

Gly Ser Pro Gln Leu Thr Ala Glu Ser Pro Val Glu Val Asn Ile Glu
            1860                1865                1870

Val Thr Asp Val Asn Asp Asn Pro Pro Val Phe Thr Gln Ala Val Phe
        1875                1880                1885

Glu Thr Ile Leu Leu Leu Pro Thr Tyr Val Gly Val Glu Val Leu Lys
    1890                1895                1900

Val Ser Ala Thr Asp Pro Asp Ser Glu Val Pro Pro Glu Leu Thr Tyr
1905                1910                1915                1920

Ser Leu Met Glu Gly Ser Leu Asp His Phe Leu Ile Asp Ser Asn Ser
                1925                1930                1935

Gly Val Leu Thr Ile Lys Asn Asn Asn Leu Ser Lys Asp His Tyr Met
            1940                1945                1950

Leu Ile Val Lys Val Ser Asp Gly Lys Phe Tyr Ser Thr Ser Met Val
        1955                1960                1965

Thr Ile Met Val Lys Glu Ala Met Asp Ser Gly Leu His Phe Thr Gln
    1970                1975                1980

Ser Phe Tyr Ser Thr Ser Ile Ser Glu Asn Asn Thr Asn Ile Thr Lys
1985                1990                1995                2000

Val Ala Ile Val Asn Ala Val Gly Asn Arg Leu Asn Glu Pro Leu Lys
                2005                2010                2015

Tyr Ser Ile Leu Asn Pro Gly Asn Lys Phe Lys Ile Lys Ser Thr Ser
            2020                2025                2030

Gly Val Ile Gln Thr Thr Gly Val Pro Phe Asp Arg Glu Glu Gln Glu
        2035                2040                2045

Leu Tyr Glu Leu Val Val Glu Ala Ser Arg Glu Leu Asp His Leu Arg
    2050                2055                2060

Val Ala Arg Val Val Val Arg Val Asn Ile Glu Asp Ile Asn Asp Asn
2065                2070                2075                2080

Ser Pro Val Phe Val Gly Leu Pro Tyr Tyr Ala Ala Val Gln Val Asp
                2085                2090                2095

Ala Glu Pro Gly Thr Leu Ile Tyr Gln Val Thr Ala Ile Asp Lys Asp
            2100                2105                2110

Lys Gly Pro Asn Gly Glu Val Thr Tyr Val Leu Gln Asp Asp Tyr Gly
        2115                2120                2125

His Phe Glu Ile Asn Pro Asn Ser Gly Asn Val Ile Leu Lys Glu Ala
    2130                2135                2140

Phe Asn Ser Asp Leu Ser Asn Ile Glu Tyr Gly Val Thr Ile Leu Ala
2145                2150                2155                2160

Lys Asp Gly Gly Lys Pro Ser Leu Ser Thr Ser Val Glu Leu Pro Ile
                2165                2170                2175

Thr Ile Val Asn Lys Ala Met Pro Val Phe Asp Lys Pro Phe Tyr Thr
            2180                2185                2190

Ala Ser Val Asn Glu Asp Ile Arg Met Asn Thr Pro Ile Leu Ser Ile
        2195                2200                2205

Asn Ala Thr Ser Pro Glu Gly Gln Gly Ile Ile Tyr Ile Ile Ile Asp
    2210                2215                2220

Gly Asp Pro Phe Lys Gln Phe Asn Ile Asp Phe Asp Thr Gly Val Leu
2225                2230                2235                2240

Lys Val Val Ser Pro Leu Asp Tyr Glu Val Thr Ser Ala Tyr Lys Leu
                2245                2250                2255

Thr Ile Arg Ala Ser Asp Ala Leu Thr Gly Ala Arg Ala Glu Val Thr
            2260                2265                2270

Val Asp Leu Leu Val Asn Asp Val Asn Asp Asn Pro Pro Ile Phe Asp
        2275                2280                2285

Gln Pro Thr Tyr Asn Thr Thr Leu Ser Glu Ala Ser Leu Ile Gly Thr
    2290                2295                2300

Pro Val Leu Gln Val Val Ser Ile Asp Ala Asp Ser Glu Asn Asn Lys
2305                2310                2315                2320

Met Val His Tyr Gln Ile Val Gln Asp Thr Tyr Asn Ser Thr Asp Tyr
                2325                2330                2335

Phe His Ile Asp Ser Ser Ser Gly Leu Ile Leu Thr Ala Arg Met Leu
            2340                2345                2350

Asp His Glu Leu Val Gln His Cys Thr Leu Lys Val Arg Ser Ile Asp
        2355                2360                2365

Ser Gly Phe Pro Ser Leu Ser Ser Glu Val Leu Val His Ile Tyr Ile
    2370                2375                2380

Ser Asp Val Asn Asp Asn Pro Pro Val Phe Asn Gln Leu Ile Tyr Glu
2385                2390                2395                2400

Ser Tyr Val Ser Glu Leu Ala Pro Arg Gly His Phe Val Thr Cys Val
                2405                2410                2415

Gln Ala Ser Asp Ala Asp Ser Ser Asp Phe Asp Arg Leu Glu Tyr Ser
            2420                2425                2430

Ile Leu Ser Gly Asn Asp Arg Thr Ser Phe Leu Met Asp Ser Lys Ser
        2435                2440                2445

Gly Val Ile Thr Leu Ser Asn His Arg Lys Gln Arg Met Glu Pro Leu
    2450                2455                2460

Tyr Ser Leu Asn Val Ser Val Ser Asp Gly Leu Phe Thr Ser Thr Ala
2465                2470                2475                2480

Gln Val His Ile Arg Val Leu Gly Ala Asn Leu Tyr Ser Pro Ala Phe
                2485                2490                2495

Ser Gln Ser Thr Tyr Val Ala Glu Val Arg Glu Asn Val Ala Ala Gly
            2500                2505                2510

Thr Lys Val Ile His Val Arg Ala Thr Asp Gly Asp Pro Gly Thr Tyr
        2515                2520                2525

Gly Gln Ile Ser Tyr Ala Ile Ile Asn Asp Phe Ala Lys Asp Arg Phe
    2530                2535                2540

Leu Ile Asp Ser Asn Gly Gln Val Ile Thr Thr Glu Arg Leu Asp Arg
2545                2550                2555                2560

Glu Asn Pro Leu Glu Gly Asp Val Ser Ile Phe Val Arg Ala Leu Asp
                2565                2570                2575

Gly Gly Gly Arg Thr Thr Phe Cys Thr Val Arg Val Ile Val Val Asp
            2580                2585                2590

Glu Asn Asp Asn Ala Pro Gln Phe Met Thr Val Glu Tyr Arg Ala Ser
        2595                2600                2605

Val Arg Ala Asp Val Gly Arg Gly His Leu Val Thr Gln Val Gln Ala
    2610                2615                2620

Ile Asp Pro Asp Asp Gly Ala Asn Ser Arg Ile Thr Tyr Ser Leu Tyr
2625                2630                2635                2640

Ser Glu Ala Ser Val Ser Val Ala Asp Leu Leu Glu Ile Asp Pro Asp
                2645                2650                2655

Asn Gly Trp Met Val Thr Lys Gly Asn Phe Asn Gln Leu Lys Asn Thr
            2660                2665                2670

Val Leu Ser Phe Phe Val Lys Ala Val Asp Gly Gly Ile Pro Val Lys
        2675                2680                2685

His Ser Leu Ile Pro Val Tyr Ile His Val Leu Pro Pro Glu Thr Phe
    2690                2695                2700

Leu Pro Ser Phe Thr Gln Ser Gln Tyr Ser Phe Thr Ile Ala Glu Asp
2705                2710                2715                2720

Thr Ala Ile Gly Ser Thr Val Asp Thr Leu Arg Ile Leu Pro Ser Gln
                2725                2730                2735

Asn Val Trp Phe Ser Thr Val Asn Gly Glu Arg Pro Glu Asn Asn Lys
            2740                2745                2750

Gly Gly Ile Phe Val Ile Glu Gln Glu Thr Gly Thr Ile Lys Leu Asp
        2755                2760                2765

Lys Arg Leu Asp Arg Glu Thr Ser Pro Ala Phe His Phe Lys Val Ala
    2770                2775                2780

Ala Thr Ile Pro Leu Asp Lys Val Asp Ile Val Phe Thr Val Asp Val
2785                2790                2795                2800

Asp Ile Lys Val Leu Asp Leu Asn Asp Asn Lys Pro Val Phe Glu Thr
                2805                2810                2815

Ser Ser Tyr Asp Thr Ile Ile Met Glu Gly Met Pro Val Gly Thr Lys
            2820                2825                2830

Leu Thr Gln Val Arg Ala Ile Asp Met Asp Trp Gly Ala Asn Gly Gln
        2835                2840                2845

Val Thr Tyr Ser Leu His Ser Asp Ser Gln Pro Glu Lys Val Met Glu
    2850                2855                2860

Ala Phe Asn Ile Asp Ser Asn Thr Gly Trp Ile Ser Thr Leu Lys Asp
2865                2870                2875                2880

Leu Asp His Glu Thr Asp Pro Thr Phe Thr Phe Ser Val Val Ala Ser
                2885                2890                2895

Asp Leu Gly Glu Ala Phe Ser Leu Ser Ser Thr Ala Leu Val Ser Val
            2900                2905                2910

Arg Val Thr Asp Ile Asn Asp Asn Ala Pro Val Phe Ala Gln Glu Val
        2915                2920                2925

Tyr Arg Gly Asn Val Lys Glu Ser Asp Pro Pro Gly Glu Val Val Ala
    2930                2935                2940

Val Leu Ser Thr Trp Asp Arg Asp Thr Ser Asp Val Asn Arg Gln Val
2945                2950                2955                2960

Ser Tyr His Ile Thr Gly Gly Asn Pro Arg Gly Arg Phe Ala Leu Gly
                2965                2970                2975

Leu Val Gln Ser Glu Trp Lys Val Tyr Val Lys Arg Pro Leu Asp Arg
            2980                2985                2990

Glu Glu Gln Asp Ile Tyr Phe Leu Asn Ile Thr Ala Thr Asp Gly Leu
        2995                3000                3005

Phe Val Thr Gln Ala Met Val Glu Val Ser Val Ser Asp Val Asn Asp
    3010                3015                3020

Asn Ser Pro Val Cys Asp Gln Val Ala Tyr Thr Ala Leu Leu Pro Glu
3025                3030                3035                3040

Asp Ile Pro Ser Asn Lys Ile Ile Leu Lys Val Ser Ala Lys Asp Ala
                3045                3050                3055

Asp Ile Gly Ser Asn Gly Tyr Ile Arg Tyr Ser Leu Tyr Gly Ser Gly
            3060                3065                3070

Asn Ser Glu Phe Phe Leu Asp Pro Glu Ser Gly Glu Leu Lys Thr Leu
        3075                3080                3085

Ala Leu Leu Asp Arg Glu Arg Ile Pro Val Tyr Ser Leu Met Ala Lys
    3090                3095                3100

Ala Thr Asp Gly Gly Gly Arg Phe Cys Gln Ser Asn Ile His Leu Ile
3105                3110                3115                3120

Leu Glu Asp Val Asn Asp Asn Pro Pro Val Phe Ser Ser Asp His Tyr
                3125                3130                3135

Asn Thr Cys Val Tyr Glu Asn Thr Ala Thr Lys Ala Leu Leu Thr Arg
            3140                3145                3150

Val Gln Ala Val Asp Pro Asp Ile Gly Ile Asn Arg Lys Val Val Tyr
        3155                3160                3165

Ser Leu Ala Asp Ser Ala Gly Gly Val Phe Ser Ile Asp Ser Ser Ser
    3170                3175                3180

Gly Ile Ile Ile Leu Glu Gln Pro Leu Asp Arg Glu Gln Gln Ser Ser
3185                3190                3195                3200

Tyr Asn Ile Ser Val Arg Ala Thr Asp Gln Ser Pro Gly Gln Ser Leu
                3205                3210                3215

Ser Ser Leu Thr Thr Val Thr Ile Thr Val Leu Asp Ile Asn Asp Asn
            3220                3225                3230

Pro Pro Val Phe Glu Arg Arg Asp Tyr Leu Val Thr Val Pro Glu Asp
        3235                3240                3245

Thr Ser Pro Gly Thr Gln Val Leu Ala Val Phe Ala Thr Ser Lys Asp
    3250                3255                3260

Ile Gly Thr Asn Ala Glu Ile Thr Tyr Leu Ile Arg Ser Gly Asn Glu
3265                3270                3275                3280

Gln Gly Lys Phe Lys Ile Asn Pro Lys Thr Gly Gly Ile Ser Val Ser
                3285                3290                3295

Glu Val Leu Asp Tyr Glu Leu Cys Lys Arg Phe Tyr Leu Val Val Glu
            3300                3305                3310

Ala Lys Asp Gly Gly Thr Pro Ala Leu Ser Ala Val Ala Thr Val Asn
        3315                3320                3325

Ile Asn Leu Thr Asp Val Asn Asp Asn Pro Pro Lys Phe Ser Gln Asp
    3330                3335                3340

Val Tyr Ser Ala Val Ile Ser Glu Asp Ala Leu Val Gly Asp Ser Val
3345                3350                3355                3360

Ile Leu Leu Ile Ala Glu Asp Val Asp Ser Gln Pro Asn Gly Gln Ile
                3365                3370                3375

His Phe Ser Ile Val Asn Gly Asp Arg Asp Asn Glu Phe Thr Val Asp
            3380                3385                3390

Pro Val Leu Gly Leu Val Lys Val Lys Lys Lys Leu Asp Arg Glu Arg
        3395                3400                3405

Val Ser Gly Tyr Ser Leu Leu Val Gln Ala Val Asp Ser Gly Ile Pro
    3410                3415                3420

Ala Met Ser Ser Thr Ala Thr Val Asn Ile Asp Ile Ser Asp Val Asn
3425                3430                3435                3440

Asp Asn Ser Pro Val Phe Thr Pro Ala Asn Tyr Thr Ala Val Ile Gln
                3445                3450                3455

Glu Asn Lys Pro Val Gly Thr Ser Ile Leu Gln Leu Val Val Thr Asp
            3460                3465                3470

Arg Asp Ser Phe His Asn Gly Pro Pro Phe Ser Phe Ser Ile Leu Ser
        3475                3480                3485

Gly Asn Glu Glu Glu Glu Phe Val Leu Asp Pro His Gly Ile Leu Arg
    3490                3495                3500

Ser Ala Val Val Phe Gln His Thr Glu Ser Leu Glu Tyr Val Leu Cys
3505                3510                3515                3520

Val Gln Ala Lys Asp Ser Gly Lys Pro Gln Gln Val Ser His Thr Tyr
                3525                3530                3535

Ile Arg Val Arg Val Ile Glu Glu Ser Thr His Lys Pro Thr Ala Ile
            3540                3545                3550

Pro Leu Glu Ile Phe Ile Val Thr Met Glu Asp Asp Phe Pro Gly Gly
        3555                3560                3565

Val Ile Gly Lys Ile His Ala Thr Asp Gln Asp Met Tyr Asp Val Leu
    3570                3575                3580

Thr Phe Ala Leu Lys Ser Glu Gln Lys Ser Leu Phe Lys Val Asn Ser
3585                3590                3595                3600

His Asp Gly Lys Ile Ile Ala Leu Gly Gly Leu Asp Ser Gly Lys Tyr
                3605                3610                3615

Val Leu Asn Val Ser Val Ser Asp Gly Arg Phe Gln Val Pro Ile Asp
            3620                3625                3630

Val Val Val His Val Glu Gln Leu Val His Glu Met Leu Gln Asn Thr
        3635                3640                3645

Val Thr Ile Arg Phe Glu Asn Val Ser Pro Glu Asp Phe Val Gly Leu
    3650                3655                3660

His Met His Gly Phe Arg Arg Thr Leu Arg Asn Ala Val Leu Thr Gln
3665                3670                3675                3680

Lys Gln Asp Ser Leu Arg Ile Ile Ser Ile Gln Pro Val Ala Gly Thr
                3685                3690                3695

Asn Gln Leu Asp Met Leu Phe Ala Val Glu Met His Ser Ser Glu Phe
            3700                3705                3710

Tyr Lys Pro Ala Tyr Leu Ile Gln Lys Leu Ser Asn Ala Arg Arg His
        3715                3720                3725

Leu Glu Asn Ile Met Arg Ile Ser Ala Ile Leu Glu Lys Asn Cys Ser
    3730                3735                3740

Gly Leu Asp Cys Gln Glu Gln His Cys Glu Gln Gly Leu Ser Leu Asp
3745                3750                3755                3760

Ser His Ala Leu Met Thr Tyr Ser Thr Ala Arg Ile Ser Phe Val Cys
                3765                3770                3775

Pro Arg Phe Tyr Arg Asn Val Arg Cys Thr Cys Asn Gly Gly Leu Cys
            3780                3785                3790

Pro Gly Ser Asn Asp Pro Cys Val Glu Lys Pro Cys Pro Gly Asp Met
        3795                3800                3805

Gln Cys Val Ser Tyr Glu Ala Ser Arg Arg Pro Phe Leu Cys Gln Cys
    3810                3815                3820

Pro Pro Gly Lys Leu Gly Glu Cys Ser Gly His Thr Ser Leu Ser Phe
3825                3830                3835                3840

Ala Gly Asn Ser Tyr Ile Lys Tyr Arg Leu Ser Glu Asn Ser Lys Glu
                3845                3850                3855

Glu Asp Phe Lys Leu Ala Leu Arg Leu Arg Thr Leu Gln Ser Asn Gly
            3860                3865                3870

Ile Ile Met Tyr Thr Arg Ala Asn Pro Cys Ile Ile Leu Lys Gln Ile
        3875                3880                3885

Val Asp Gly Lys Leu Trp Phe Gln Leu Asp Cys Gly Ser Gly Pro Gly
    3890                3895                3900

Ile Leu Gly Ile Ser Gly Arg Ala Val Asn Asp Gly Ser Trp His Ser
3905                3910                3915                3920

Val Phe Leu Glu Leu Asn Arg Asn Phe Thr Ser Leu Ser Leu Asp Asp
                3925                3930                3935

Ser Tyr Val Glu Arg Arg Arg Ala Pro Leu Tyr Phe Gln Thr Leu Ser
            3940                3945                3950

Thr Glu Ser Ser Ile Tyr Phe Gly Ala Leu Val Gln Ala Asp Asn Ile
        3955                3960                3965

Arg Ser Leu Thr Asp Thr Arg Val Thr Gln Val Leu Ser Gly Phe Gln
    3970                3975                3980

Gly Cys Leu Asp Ser Val Ile Leu Asn Asn Asn Glu Leu Pro Leu Gln
3985                3990                3995                4000

Asn Lys Arg Ser Ser Phe Ala Glu Val Val Gly Leu Thr Glu Leu Lys
                4005                4010                4015

Leu Gly Cys Val Leu Tyr Pro Asp Ala Cys Lys Arg Ser Pro Cys Gln
            4020                4025                4030

His Gly Gly Ser Cys Thr Gly Leu Pro Ser Gly Gly Gly Tyr Gln Cys
        4035                4040                4045

Thr Cys Leu Ser Gln Phe Thr Gly Arg Asn Cys Glu Ser Glu Ile Thr
    4050                4055                4060

Ala Cys Phe Pro Asn Pro Cys Arg Asn Gly Gly Ser Cys Asp Pro Ile
4065                4070                4075                4080

Gly Asn Thr Phe Ile Cys Asn Cys Lys Ala Gly Leu Thr Gly Val Thr
                4085                4090                4095

Cys Glu Glu Asp Ile Asn Glu Cys Glu Arg Glu Glu Cys Glu Asn Gly
            4100                4105                4110

Gly Ser Cys Val Asn Val Phe Gly Ser Phe Leu Cys Asn Cys Thr Pro
        4115                4120                4125

Gly Tyr Val Gly Gln Tyr Cys Gly Leu Arg Pro Val Val Val Pro Asn
    4130                4135                4140

Ile Gln Ala Gly His Ser Tyr Val Gly Lys Glu Glu Leu Ile Gly Ile
4145                4150                4155                4160

Ala Val Val Leu Phe Val Ile Phe Ile Leu Val Val Leu Phe Ile Val
                4165                4170                4175

Phe Arg Lys Lys Val Phe Arg Lys Asn Tyr Ser Arg Asn Asn Ile Thr
            4180                4185                4190

Leu Val Gln Asp Pro Ala Thr Ala Ala Leu Leu Asn Lys Ser Asn Gly
        4195                4200                4205

Ile Pro Phe Arg Asn Leu Arg Gly Ser Gly Asp Gly Arg Asn Val Tyr
    4210                4215                4220

Gln Glu Val Gly Pro Pro Gln Val Pro Val Arg Pro Met Ala Tyr Thr
4225                4230                4235                4240

Pro Cys Phe Gln Ser Asp Ser Arg Ser Asn Leu Asp Lys Ile Val Asp
                4245                4250                4255

Gly Leu Gly Gly Glu His Gln Glu Met Thr Thr Phe His Pro Glu Ser
            4260                4265                4270

Pro Arg Ile Leu Thr Ala Arg Arg Gly Val Val Val Cys Ser Val Ala
        4275                4280                4285

Pro Asn Leu Pro Ala Val Ser Pro Cys Arg Ser Asp Cys Asp Ser Ile
    4290                4295                4300

Arg Lys Asn Gly Trp Asp Ala Gly Thr Glu Ser Asn Lys Gly Ser Asn
4305                4310                4315                4320

Ser Glu Val Gln Ser Leu Ser Ser Phe Gln Ser Asp Ser Gly Asp Asp
                4325                4330                4335

Asn Ala Tyr His Trp Asp Thr Ser Asp Trp Met Pro Gly Ala Arg Leu
            4340                4345                4350

Ser Asp Ile Glu Glu Val Pro Asn Tyr Glu Asn Gln Asp Gly Gly Ser
        4355                4360                4365

Ala His Gln Gly Ser Thr Arg Glu Leu Glu Ser Asp Tyr Tyr Leu Gly
    4370                4375                4380

Gly Tyr Asp Ile Asp Ser Glu Tyr Pro Pro Pro His Glu Glu Glu Phe
4385                4390                4395                4400

Leu Ser Gln Asp Gln Leu Pro Pro Pro Leu Pro Glu Asp Phe Pro Asp
                4405                4410                4415

Gln Tyr Glu Ala Leu Pro Pro Ser Gln Pro Val Ser Leu Ala Ser Thr
            4420                4425                4430

Leu Ser Pro Asp Cys Arg Arg Arg Pro Gln Phe His Pro Ser Gln Tyr
        4435                4440                4445

Leu Pro Pro His Pro Phe Pro Asn Glu Thr Asp Leu Val Gly Pro Pro
    4450                4455                4460

Ala Ser Cys Glu Phe Ser Thr Phe Ala Val Ser Met Asn Gln Gly Thr
4465                4470                4475                4480

Glu Pro Thr Gly Pro Ala Asp Ser Val Ser Leu Ser Leu His Asn Ser
                4485                4490                4495

Arg Gly Thr Ser Ser Ser Asp Val Ser Ala Asn Cys Gly Phe Asp Asp
            4500                4505                4510

Ser Glu Val Ala Met Ser Asp Tyr Glu Ser Val Gly Glu Leu Ser Leu
        4515                4520                4525

Ala Ser Leu His Ile Pro Phe Val Glu Thr Gln His Gln Thr Gln Val
    4530                4535                4540

<210> 23
<211> 1071
<212> DNA
<213> Homo sapiens

<400> 23
atgcatcagt ttaccatcgt ttcattccat ccatccagga gactacacag aaacagagag 60
gactatgtgg aaagaagtgc tgagtttgca gatggtttgc tctcaaaagc tttgaaagac 120
attcagtctg gagcactgga cataaataaa gcaggcatac tttatggcat acctcaaaaa 180
actttacttc ttcacttaga agccttacca gcagggaagc ctgcatcttt taaaaacaaa 240
actcgagatt tccatgatag ttattcatat aaggacagta aagaaacttg tgcagtgctg 300
caaaaagtag ccttgtgggc aagagctcaa gcagagcgca cagaaaaaag taaactcaat 360
ctacttgaaa cctcagaaat aaaattccca acagcttcca cttacctcca tcagctaact 420
ctacagaaaa tggtcactca gtttaaagaa aaaaatgaaa gcctccaata tgaaacttca 480
aatcctactg tacagttaaa aattcctcag ctacgagtaa gttctgtctc aaaatcacaa 540
cctgatggtt ctggtctgtt ggatgttatg tatcaagttt ccaaaacctc ttcagtccta 600
gaaggatcag ctctccaaaa actgaaaaat atactcccta aacagaacaa aatagaatgt 660
tctgggcctg taactcactc aagtgttgac tcttactttc tacatgggga cctctctcct 720
ttgtgtctta attctaaaaa tggaacagtt gatggaacct ctgaaaatac tgaagatgga 780
ttagatcgaa aagacagtaa gcagcccagg aaaaaacgtg gccgctatcg gcaatatgat 840
catgaaataa tggaagaagc tattgcaatg gtaatgagcg gaaaaatgag tgtttccaaa 900
gcacaaggaa tttatggggt acctcacagc actttagaat acaaggtaaa agaaagatct 960
ggaacactga agactcctcc gaagaagaaa ctacgattac cagacactgg gttatataat 1020
atgacagatt cagggactgg cagctgcaaa aacagcagca agcctgtgta g 1071

<210> 24
<211> 356
<212> PRT
<213> Homo sapiens

<400> 24

<210> 25
<211> 6195
<212> DNA
<213> Homo sapiens

<400> 25
atgagcggcg ccctgctctg gccgttgctc ccgctcctgc tcctgctgct gtcggcgcgg 60
gacggcgtgc gcgccgcgca gcctcaggcc ccgggttact tgattgcagc tccctctgtt 120
tttcgcgcgg gcgtggagga agtcatcagc gtgaccatct ttaactctcc aagggaagtc 180
acggtccagg ctcagctggt ggcccagggt gagccggtgg tgcagagcca gggagccatc 240
ctggataaag ggacaatcaa actcaagcat acggtcctca gcacctccgg tatctccctc 300
ctgcccatcc ttgccctgct cttgggtggc cgggaccttt cctccctctt cagcctctgg 360
ccagtgttga gatatttcca gaaacagggc caggtgccca cgggcctccg gggccaagcg 420
cttctgaaag tgtggggccg cggctggcag gcggaggagg ggcccctctt tcacaaccag 480
acctcggtga ccgtggacgg ccggggcgct tctgtattca tccagacgga caagcctgtg 540
tacagacccc agcaccgagt gctcataagc atcttcaccg tctctccaaa tctgaggcct 600
gtcaacgaga agctggaagc ctacatcctg gacccccgag gctctcggat gatagagtgg 660
agacacttaa agccgttctg ctgcggcatc accaacatga gcttcccctt gtccgaccag 720
cctgtgttgg gagaatggtt catttttgtt gaaatgcaag gccacgcgta caacaagtct 780
tttgaagttc agaagtatgt gttgcccaag tttgagcttc tgattgaccc gccccggtat 840
atccaagacc tggacgcctg tgagacaggc actgtgcggg ccaggtatac ctttgggaaa 900
cctgtggctg gtgccttaat gatcaacatg actgttaatg gtgtagggta ctacagccac 960
gaggtgggac gccctgtcct cagaacaacc aagatcctcg gctcccggga cttcgacatc 1020
tgcgtgaggg acatgatccc agcggacgtc cctgagcact tccggggcag ggtcagcatc 1080
tgggccatgg tgaccagtgt ggacgggagc cagcaggtcg cgttcgatga ctccaccccc 1140
gtgcagaggc agctggtgga catccggtac tccaaggaca cgaggaagca gttcaagccg 1200
ggcctggcct acgtggggaa ggtggagcta tcctaccccg atggcagccc agctgagggg 1260
gtgacggtcc agattaaggc agagctgaca ccaaaggata acatctacac cagtgaagtt 1320
gtgtcccagc gtggactagt ggggtttgaa atcccctcca tccccacgtc agcccagcac 1380
gtgtggctgg agaccaaggt gatggcactg aacgggaagc ccgtgggggc tcagtacctg 1440
cccagctacc tctccctcgg cagctggtac tcccccagcc agtgctacct gcagctgcag 1500
ccaccctccc acccactgca ggttggggaa gaagcctatt tttctgtgaa gtccacatgt 1560

ccctgcaact ttaccctgta ctacgaggtg gctgcacggg gcaatattgt gctatcgggc 1620
cagcagcctg cccacaccac ccagcagcga agcaagcggg cggcccctgc cctggagaaa 1680
ccgattcgtt taacacacct ttctgagaca gagcccccac cagccccaga agctgaggtc 1740
gacgtgtgtg tgacctctct tcatctggcc gtgaccccca gcatggtccc ccttggtcgc 1800
ctgctggtct tctacgtcag ggagaatgga gaaggggtcg ccgacagcct tcagtttgca 1860
gtcgagacct tcttcgaaaa ccaggtttca gtgacgtatt cagcaaatga gacccaacct 1920
ggggaggttg tcgacctgcg gatcagggct gcaaggggca gctgtgtgtg cgtcgccgca 1980
gttgataaga gtgtctacct gctcaggtct gggttccggc tgactcctgc ccaggttttc 2040
caggaactgg aagattatga tgtttctgat tcctttggcg tgtccaggga ggatggtcct 2100
ttttggtggg ctgggctgac ggcacaacga cgccggcgct cctctgtctt cccgtggcct 2160
tggggcatca ccaaggactc tgggtttgcc ttcaccgaaa cgggactggt ggtgatgacc 2220
gaccgagtga gcctgaacca ccggcaggac ggtggcctct acaccgatga ggctgtcccc 2280
gctttccagc cccacacagg gagcctggtg gcagtggctc cttccaggca cccccccaga 2340
acagagaaga gaaaaaggac tttcttcccc gaaacatgga tttggcattg tctcaacatc 2400
agtgacccat ctggtgaggg gacactcagt gtgaaggtcc cggactccat caccagctgg 2460
gtgggtgagg ccgtggccct gtccacctct cagggcttag gcatcgccga gccctccctg 2520
ctgaagacct tcaagccctt cttcgtggac ttcatgctcc ccgctctcat catccgtggg 2580
gagcaggtca agatcccgct cagtgtctac aactacatgg gcacctgcgc tgaggtgtac 2640
atgaagctct cggttcccaa gggcatccag tttgttgggc atcctggcaa acgccatgtg 2700
accaagaaga tgtgtgtggc ccccggggag gctgagccca tctgggtcgt tctgtccttc 2760
agcgacctgg gactcaacaa catcacggcc aaagcccttg cttacggaga cacaaattgc 2820
tgccgggatg ggaggtccag caaacaccct gaggagaatc acgccgacag gagggtcccc 2880
atcggggtgg atcacgtcag gcgcagtgtg atggttgagg cggaaggagt cccccgggcg 2940
tacacctaca gcgcattctt ctgtcccagt gagagagtcc acatctccac ccccaacaag 3000
tatgagttcc agtatgtgca gcggccactg cgcctcaccc gctttgatgt ggctgtgcga 3060
gctcacaatg atgcccgtgt ggccttgtct tctgggcccc aggacacagc aggcatgatc 3120
gagatcgtcc tgggggggca tcagaacacc aggtcatgga tctccaccag caagatggga 3180
gagcccgtgg ccagtgcaca cacggccaag atcctctcct gggatgaatt cagaacattc 3240
tggatcagct ggcgtggtgg ccttatccag gttggccatg gtccagagcc atccaatgag 3300
tctgtcattg tggcctggac cctcccgagg ccaccagagg tccagttcat tggcttttcc 3360
accggctggg gctccatggg tgaattccga atctggagga agatggaggt ggacgagagc 3420
tacagcgagg ccttcaccct gggggtccca cacggcgcca tccctgggtc tgagcgagcc 3480
accgcctcca tcatcgggga cgtcatgggg ccaaccctga accacctcaa caacctcctg 3540
cggctgccgt ttggctgtgg agagcagaac atgatccact ttgcacccaa cgtctttgtc 3600
ttgaagtatc ttcagaaaac ccagcagctc agccctgagg tggagagaga gaccaccgac 3660
tacctagtac aaggctacca gcgccagctg acctacaagc gccaggatgg ctcctacagc 3720
gcgtttgggg agcgggacgc atcggggagc atgtggctca cagcctttgt cctgaagtcc 3780
ttcgcacagg ctcgcagctt tatcttcgtg gacccccggg agctggctgc cgccaagagc 3840
tggatcatcc agcagcagca ggccgatggc tccttcctgg ccgtgggcag ggtcctgaac 3900
aaggacatcc agggtgggat ccacggcatt gtcccgctga cagcctacgt ggtggttgct 3960
ctcctggaaa caggcacagc ctcagaggag gagagaggct ccactgacaa agcgaggcac 4020
ttcctggagt ctgctgcgcc cctggccatg gacccttata gctgtgccct gactacctac 4080
gcgctgaccc tgctccgcag cccggcagcc cctgaggcac tgcgcaagct ccgtagcctg 4140
gccatcatgc gagatggggt cacccactgg agcctgtcaa attcctggga cgtggacaag 4200
ggcacattct tgagcttcag tgacagggtc tctcagtcag tggtctcggc cgaggtggaa 4260
atgacagcct acgcccttct gacctacact ctgctgggtg acgtggctgc cgccctgcct 4320
gtggtgaagt ggctgtccca gcagcgaaat gcacttgggg gcttctcctc cactcaggac 4380
acctgcgtgg ctctgcaggc cttggctgaa tatgccatct tgtcctatgc tggaggcatc 4440
aacctcactg tctccctggc ctccaccaac ctggactacc aggaaacctt cgagctgcac 4500
aggaccaacc agaaggttct gcagacagca gcgatcccca gcctccccac ggggctgttt 4560
gtgagtgcca aggggacggg ctgctgcctg atgcagattg atgtcaccta caatgtgcct 4620
gacccggtgg ccaagccagc tttccagctg ctcgtaagcc tccaggagcc tgaggcccag 4680
ggacgcccgc cccccatgcc tgcctccgca gctgagggtt cccgaggaga ctggccccca 4740
gctgacgatg atgacccagc ggccgatcag catcaccagg aatacaaggt gatgctggag 4800
gtgtgcacca ggtggctgca tgcagggtct tccaatatgg ctgtcctgga ggtgcccctg 4860
ctgtcaggct tccgggcaga catcgagagc ctggagcagc tgctccttga caagcacatg 4920
gggatgaaga ggtatgaagt ggctggacgc cgagtgctct tctactttga tgagatcccc 4980
agccggtgcc tgacgtgcgt gcggttccgt gctctccggg agtgcgtggt gggcaggacg 5040
tcggcgctgc cagtctccgt gtacgactac tacgaacccg ccttcgaggc cactcgcttc 5100
tacaacgtca gcacgcacag cccactcgcc cgggaactgt gcgccggacc cgcgtgcaac 5160
gaagtggagc gcgcccctgc ccgggccccg ggctggttcc ccggcgagtc gggccctgcc 5220
gtggcccctg aggagggggc ggcgatcgcg cgatgcggct gcgaccacga ctgcggcgcc 5280
caggggaacc cggtgtgcgg ctccgacggg gtggtctacg ccagcgcctg ccgcctgcgg 5340
gaggccgcct gccgccaggc cgcgcccctg gagcccgcgc ctcccagctg ctgcgccctc 5400
gagcagcggc tgccggcctc gtcgtcctcc acctacgggg atgacctggc ttctgtggcc 5460
ccggggcctt tacagcagga cgtgaagctg aatggagccg gccttgaggt ggaggactca 5520
gaccctgagc ctgaagggga ggcggaggac agggtcacag ccgggcctcg gcctcctgtg 5580
agcagcggga acctggaaag cagcacccag agcgccagcc cgttccacag atggggccag 5640
actccggccc ctcagagaca tagtggccgg gtggtggggg cccacaggcc agggcttctg 5700

agccctgtct tcgtctacag cccagccttt cagagtggtg gggaggaggg tttatggatg 5760
tcaaacacct gcaccttgag ataatcctac aaccacatgc agttgtggga ccgcagtttg 5820
gtcctgggga ccattcatac ccacacaccc agcttgtgcc tgtggttaac atctcagaaa 5880
actctggtaa atgatcactc caggatattg acacgaatac acgttactga tcttactcac 5940
atgttctggg gtgcacatga actttgtgtg tgcatgtgtg tgtgtgtgca tgtgtgtgtc 6000
ccgggcacct gacaccccca gcccagggct gcccaaagtt gggctgatca gagacataga 6060
cccaatgagg agcccaacag tggccctcca accctctgcc ttgcccccat agttcatgcc 6120
ccagtggtct ttgaaactgc cctgtgccac tccctggagt gagcagcggt gtctctgtgt 6180
gtgtgtgtgt ctgtg 6195

<210> 26
<211> 1927
<212> PRT
<213> Homo sapiens

<400> 26
Met Ser Gly Ala Leu Leu Trp Pro Leu Leu Pro Leu Leu Leu Leu Leu
1               5                   10                  15

Leu Ser Ala Arg Asp Gly Val Arg Ala Ala Gln Pro Gln Ala Pro Gly
            20                  25                  30

Tyr Leu Ile Ala Ala Pro Ser Val Phe Arg Ala Gly Val Glu Glu Val
        35                  40                  45

Ile Ser Val Thr Ile Phe Asn Ser Pro Arg Glu Val Thr Val Gln Ala
    50                  55                  60

Gln Leu Val Ala Gln Gly Glu Pro Val Val Gln Ser Gln Gly Ala Ile
65                  70                  75                  80

Leu Asp Lys Gly Thr Ile Lys Leu Lys His Thr Val Leu Ser Thr Ser
                85                  90                  95

Gly Ile Ser Leu Leu Pro Ile Leu Ala Leu Leu Leu Gly Gly Arg Asp
            100                 105                 110

Leu Ser Ser Leu Phe Ser Leu Trp Pro Val Leu Arg Tyr Phe Gln Lys
        115                 120                 125

Gln Gly Gln Val Pro Thr Gly Leu Arg Gly Gln Ala Leu Leu Lys Val
    130                 135                 140

Trp Gly Arg Gly Trp Gln Ala Glu Glu Gly Pro Leu Phe His Asn Gln
145                 150                 155                 160

Thr Ser Val Thr Val Asp Gly Arg Gly Ala Ser Val Phe Ile Gln Thr
                165                 170                 175

Asp Lys Pro Val Tyr Arg Pro Gln His Arg Val Leu Ile Ser Ile Phe
            180                 185                 190

Thr Val Ser Pro Asn Leu Arg Pro Val Asn Glu Lys Leu Glu Ala Tyr
        195                 200                 205

Ile Leu Asp Pro Arg Gly Ser Arg Met Ile Glu Trp Arg His Leu Lys
    210                 215                 220

Pro Phe Cys Cys Gly Ile Thr Asn Met Ser Phe Pro Leu Ser Asp Gln
225                 230                 235                 240

Pro Val Leu Gly Glu Trp Phe Ile Phe Val Glu Met Gln Gly His Ala
                245                 250                 255

Tyr Asn Lys Ser Phe Glu Val Gln Lys Tyr Val Leu Pro Lys Phe Glu
            260                 265                 270

Leu Leu Ile Asp Pro Pro Arg Tyr Ile Gln Asp Leu Asp Ala Cys Glu

        275                 280                 285

Thr Gly Thr Val Arg Ala Arg Tyr Thr Phe Gly Lys Pro Val Ala Gly
    290                 295                 300

Ala Leu Met Ile Asn Met Thr Val Asn Gly Val Gly Tyr Tyr Ser His
305                 310                 315                 320

Glu Val Gly Arg Pro Val Leu Arg Thr Thr Lys Ile Leu Gly Ser Arg
                325                 330                 335

Asp Phe Asp Ile Cys Val Arg Asp Met Ile Pro Ala Asp Val Pro Glu
            340                 345                 350

His Phe Arg Gly Arg Val Ser Ile Trp Ala Met Val Thr Ser Val Asp
        355                 360                 365

Gly Ser Gln Gln Val Ala Phe Asp Asp Ser Thr Pro Val Gln Arg Gln
    370                 375                 380

Leu Val Asp Ile Arg Tyr Ser Lys Asp Thr Arg Lys Gln Phe Lys Pro
385                 390                 395                 400

Gly Leu Ala Tyr Val Gly Lys Val Glu Leu Ser Tyr Pro Asp Gly Ser
                405                 410                 415

Pro Ala Glu Gly Val Thr Val Gln Ile Lys Ala Glu Leu Thr Pro Lys
            420                 425                 430

Asp Asn Ile Tyr Thr Ser Glu Val Val Ser Gln Arg Gly Leu Val Gly
        435                 440                 445

Phe Glu Ile Pro Ser Ile Pro Thr Ser Ala Gln His Val Trp Leu Glu
    450                 455                 460

Thr Lys Val Met Ala Leu Asn Gly Lys Pro Val Gly Ala Gln Tyr Leu
465                 470                 475                 480

Pro Ser Tyr Leu Ser Leu Gly Ser Trp Tyr Ser Pro Ser Gln Cys Tyr
                485                 490                 495

Leu Gln Leu Gln Pro Pro Ser His Pro Leu Gln Val Gly Glu Glu Ala
            500                 505                 510

Tyr Phe Ser Val Lys Ser Thr Cys Pro Cys Asn Phe Thr Leu Tyr Tyr
        515                 520                 525

Glu Val Ala Ala Arg Gly Asn Ile Val Leu Ser Gly Gln Gln Pro Ala
    530                 535                 540

His Thr Thr Gln Gln Arg Ser Lys Arg Ala Ala Pro Ala Leu Glu Lys
545                 550                 555                 560

Pro Ile Arg Leu Thr His Leu Ser Glu Thr Glu Pro Pro Pro Ala Pro
                565                 570                 575

Glu Ala Glu Val Asp Val Cys Val Thr Ser Leu His Leu Ala Val Thr
            580                 585                 590

Pro Ser Met Val Pro Leu Gly Arg Leu Leu Val Phe Tyr Val Arg Glu
        595                 600                 605

Asn Gly Glu Gly Val Ala Asp Ser Leu Gln Phe Ala Val Glu Thr Phe
    610                 615                 620

Phe Glu Asn Gln Val Ser Val Thr Tyr Ser Ala Asn Glu Thr Gln Pro
625                 630                 635                 640

Gly Glu Val Val Asp Leu Arg Ile Arg Ala Ala Arg Gly Ser Cys Val

                645                 650                 655

Cys Val Ala Ala Val Asp Lys Ser Val Tyr Leu Leu Arg Ser Gly Phe
            660                 665                 670

Arg Leu Thr Pro Ala Gln Val Phe Gln Glu Leu Glu Asp Tyr Asp Val
        675                 680                 685

Ser Asp Ser Phe Gly Val Ser Arg Glu Asp Gly Pro Phe Trp Trp Ala
    690                 695                 700

Gly Leu Thr Ala Gln Arg Arg Arg Arg Ser Ser Val Phe Pro Trp Pro
705                 710                 715                 720

Trp Gly Ile Thr Lys Asp Ser Gly Phe Ala Phe Thr Glu Thr Gly Leu
                725                 730                 735

Val Val Met Thr Asp Arg Val Ser Leu Asn His Arg Gln Asp Gly Gly
            740                 745                 750

Leu Tyr Thr Asp Glu Ala Val Pro Ala Phe Gln Pro His Thr Gly Ser
        755                 760                 765

Leu Val Ala Val Ala Pro Ser Arg His Pro Pro Arg Thr Glu Lys Arg
    770                 775                 780

Lys Arg Thr Phe Phe Pro Glu Thr Trp Ile Trp His Cys Leu Asn Ile
785                 790                 795                 800

Ser Asp Pro Ser Gly Glu Gly Thr Leu Ser Val Lys Val Pro Asp Ser
                805                 810                 815

Ile Thr Ser Trp Val Gly Glu Ala Val Ala Leu Ser Thr Ser Gln Gly
            820                 825                 830

Leu Gly Ile Ala Glu Pro Ser Leu Leu Lys Thr Phe Lys Pro Phe Phe
        835                 840                 845

Val Asp Phe Met Leu Pro Ala Leu Ile Ile Arg Gly Glu Gln Val Lys
    850                 855                 860

Ile Pro Leu Ser Val Tyr Asn Tyr Met Gly Thr Cys Ala Glu Val Tyr
865                 870                 875                 880

Met Lys Leu Ser Val Pro Lys Gly Ile Gln Phe Val Gly His Pro Gly
                885                 890                 895

Lys Arg His Val Thr Lys Lys Met Cys Val Ala Pro Gly Glu Ala Glu
            900                 905                 910

Pro Ile Trp Val Val Leu Ser Phe Ser Asp Leu Gly Leu Asn Asn Ile
        915                 920                 925

Thr Ala Lys Ala Leu Ala Tyr Gly Asp Thr Asn Cys Cys Arg Asp Gly
    930                 935                 940

Arg Ser Ser Lys His Pro Glu Glu Asn His Ala Asp Arg Arg Val Pro
945                 950                 955                 960

Ile Gly Val Asp His Val Arg Arg Ser Val Met Val Glu Ala Glu Gly
                965                 970                 975

Val Pro Arg Ala Tyr Thr Tyr Ser Ala Phe Phe Cys Pro Ser Glu Arg
            980                 985                 990

Val His Ile Ser Thr Pro Asn Lys Tyr Glu Phe Gln Tyr Val Gln Arg
        995                 1000                1005

Pro Leu Arg Leu Thr Arg Phe Asp Val Ala Val Arg Ala His Asn Asp

1010                1015                1020

Ala Arg Val Ala Leu Ser Ser Gly Pro Gln Asp Thr Ala Gly Met Ile
1025                1030                1035                1040

Glu Ile Val Leu Gly Gly His Gln Asn Thr Arg Ser Trp Ile Ser Thr
                1045                1050                1055

Ser Lys Met Gly Glu Pro Val Ala Ser Ala His Thr Ala Lys Ile Leu
            1060                1065                1070

Ser Trp Asp Glu Phe Arg Thr Phe Trp Ile Ser Trp Arg Gly Gly Leu
        1075                1080                1085

Ile Gln Val Gly His Gly Pro Glu Pro Ser Asn Glu Ser Val Ile Val
    1090                1095                1100

Ala Trp Thr Leu Pro Arg Pro Pro Glu Val Gln Phe Ile Gly Phe Ser
1105                1110                1115                1120

Thr Gly Trp Gly Ser Met Gly Glu Phe Arg Ile Trp Arg Lys Met Glu
                1125                1130                1135

Val Asp Glu Ser Tyr Ser Glu Ala Phe Thr Leu Gly Val Pro His Gly
            1140                1145                1150

Ala Ile Pro Gly Ser Glu Arg Ala Thr Ala Ser Ile Ile Gly Asp Val
        1155                1160                1165

Met Gly Pro Thr Leu Asn His Leu Asn Asn Leu Leu Arg Leu Pro Phe
    1170                1175                1180

Gly Cys Gly Glu Gln Asn Met Ile His Phe Ala Pro Asn Val Phe Val
1185                1190                1195                1200

Leu Lys Tyr Leu Gln Lys Thr Gln Gln Leu Ser Pro Glu Val Glu Arg
                1205                1210                1215

Glu Thr Thr Asp Tyr Leu Val Gln Gly Tyr Gln Arg Gln Leu Thr Tyr
            1220                1225                1230

Lys Arg Gln Asp Gly Ser Tyr Ser Ala Phe Gly Glu Arg Asp Ala Ser
        1235                1240                1245

Gly Ser Met Trp Leu Thr Ala Phe Val Leu Lys Ser Phe Ala Gln Ala
    1250                1255                1260

Arg Ser Phe Ile Phe Val Asp Pro Arg Glu Leu Ala Ala Ala Lys Ser
1265                1270                1275                1280

Trp Ile Ile Gln Gln Gln Gln Ala Asp Gly Ser Phe Leu Ala Val Gly
                1285                1290                1295

Arg Val Leu Asn Lys Asp Ile Gln Gly Gly Ile His Gly Ile Val Pro
            1300                1305                1310

Leu Thr Ala Tyr Val Val Val Ala Leu Leu Glu Thr Gly Thr Ala Ser
        1315                1320                1325

Glu Glu Glu Arg Gly Ser Thr Asp Lys Ala Arg His Phe Leu Glu Ser
    1330                1335                1340

Ala Ala Pro Leu Ala Met Asp Pro Tyr Ser Cys Ala Leu Thr Thr Tyr
1345                1350                1355                1360

Ala Leu Thr Leu Leu Arg Ser Pro Ala Ala Pro Glu Ala Leu Arg Lys
                1365                1370                1375

Leu Arg Ser Leu Ala Ile Met Arg Asp Gly Val Thr His Trp Ser Leu

1380                 1385                 1390

Ser Asn Ser Trp Asp Val Asp Lys Gly Thr Phe Leu Ser Phe Ser Asp
        1395                 1400                 1405

Arg Val Ser Gln Ser Val Val Ser Ala Glu Val Glu Met Thr Ala Tyr
    1410                 1415                 1420

Ala Leu Leu Thr Tyr Thr Leu Leu Gly Asp Val Ala Ala Ala Leu Pro
1425                 1430                 1435                 1440

Val Val Lys Trp Leu Ser Gln Gln Arg Asn Ala Leu Gly Gly Phe Ser
                1445                 1450                 1455

Ser Thr Gln Asp Thr Cys Val Ala Leu Gln Ala Leu Ala Glu Tyr Ala
            1460                 1465                 1470

Ile Leu Ser Tyr Ala Gly Gly Ile Asn Leu Thr Val Ser Leu Ala Ser
        1475                 1480                 1485

Thr Asn Leu Asp Tyr Gln Glu Thr Phe Glu Leu His Arg Thr Asn Gln
    1490                 1495                 1500

Lys Val Leu Gln Thr Ala Ala Ile Pro Ser Leu Pro Thr Gly Leu Phe
1505                 1510                 1515                 1520

Val Ser Ala Lys Gly Asp Gly Cys Cys Leu Met Gln Ile Asp Val Thr
                1525                 1530                 1535

Tyr Asn Val Pro Asp Pro Val Ala Lys Pro Ala Phe Gln Leu Leu Val
            1540                 1545                 1550

Ser Leu Gln Glu Pro Glu Ala Gln Gly Arg Pro Pro Pro Met Pro Ala
        1555                 1560                 1565

Ser Ala Ala Glu Gly Ser Arg Gly Asp Trp Pro Pro Ala Asp Asp Asp
    1570                 1575                 1580

Asp Pro Ala Ala Asp Gln His His Gln Glu Tyr Lys Val Met Leu Glu
1585                 1590                 1595                 1600

Val Cys Thr Arg Trp Leu His Ala Gly Ser Ser Asn Met Ala Val Leu
                1605                 1610                 1615

Glu Val Pro Leu Leu Ser Gly Phe Arg Ala Asp Ile Glu Ser Leu Glu
            1620                 1625                 1630

Gln Leu Leu Leu Asp Lys His Met Gly Met Lys Arg Tyr Glu Val Ala
        1635                 1640                 1645

Gly Arg Arg Val Leu Phe Tyr Phe Asp Glu Ile Pro Ser Arg Cys Leu
    1650                 1655                 1660

Thr Cys Val Arg Phe Arg Ala Leu Arg Glu Cys Val Val Gly Arg Thr
1665                 1670                 1675                 1680

Ser Ala Leu Pro Val Ser Val Tyr Asp Tyr Tyr Glu Pro Ala Phe Glu
                1685                 1690                 1695

Ala Thr Arg Phe Tyr Asn Val Ser Thr His Ser Pro Leu Ala Arg Glu
            1700                 1705                 1710

Leu Cys Ala Gly Pro Ala Cys Asn Glu Val Glu Arg Ala Pro Ala Arg
        1715                 1720                 1725

Gly Pro Gly Trp Phe Pro Gly Glu Ser Gly Pro Ala Val Ala Pro Glu
    1730                 1735                 1740

Glu Gly Ala Ala Ile Ala Arg Cys Gly Cys Asp His Asp Cys Gly Ala

1745                1750                1755                1760

Gln Gly Asn Pro Val Cys Gly Ser Asp Gly Val Val Tyr Ala Ser Ala
                1765                1770                1775

Cys Arg Leu Arg Glu Ala Ala Cys Arg Gln Ala Ala Pro Leu Glu Pro
            1780                1785                1790

Ala Pro Pro Ser Cys Cys Ala Leu Glu Gln Arg Leu Pro Ala Ser Ser
        1795                1800                1805

Ser Ser Thr Tyr Gly Asp Asp Leu Ala Ser Val Ala Pro Gly Pro Leu
    1810                1815                1820

Gln Gln Asp Val Lys Leu Asn Gly Ala Gly Leu Glu Val Glu Asp Ser
1825                1830                1835                1840

Asp Pro Glu Pro Glu Gly Glu Ala Glu Asp Arg Val Thr Ala Gly Pro
                1845                1850                1855

Arg Pro Pro Val Ser Ser Gly Asn Leu Glu Ser Ser Thr Gln Ser Ala
            1860                1865                1870

Ser Pro Phe His Arg Trp Gly Gln Thr Pro Ala Pro Gln Arg His Ser
        1875                1880                1885

Gly Arg Val Val Gly Ala His Arg Pro Gly Leu Leu Ser Pro Val Phe
    1890                1895                1900

Val Tyr Ser Pro Ala Phe Gln Ser Gly Gly Glu Glu Gly Leu Trp Met
1905                1910                1915                1920

Ser Asn Thr Cys Thr Leu Arg
                1925


<210> 27
<211> 6069
<212> DNA
<213> Homo sapiens

<400> 27
atgagcggcg ccctgctctg gccgttgctc ccgctcctgc tcctgctgct gtcggcgcgg 60
gacggcgtgc gcgccgcgca gcctcaggcc ccgggttact tgattgcagc tccctctgtt 120
tttcgcgcgg gcgtggagga agtcatcagc gtgaccatct ttaactctcc aagggaagtc 180
acggtccagg ctcagctggt ggcccagggt gagccggtgg tgcagagcca gggagccatc 240
ctggataaag ggacaatcaa actcaaggtg cccacgggcc tccggggcca agcgcttctg 300
aaagtgtggg gccgcggctg gcaggcggag gaggggcccc tctttcacaa ccagacctcg 360
gtgaccgtgg acggccgggg cgcttctgta ttcatccaga cggacaagcc tgtgtacaga 420
ccccagcacc gagtgctcat aagcatcttc accgtctctc caaatctgag gcctgtcaac 480
gagaagctgg aagcctacat cctggacccc cgaggctctc ggatgataga gtggagacac 540
ttaaagccgt tctgctgcgg catcaccaac atgagcttcc ccttgtccga ccagcctgtg 600
ttgggagaat ggttcatttt tgttgaaatg caaggccacg cgtacaacaa gtcttttgaa 660
gttcagaagt atgtgttgcc caagtttgag cttctgattg acccgccccg gtatatccaa 720
gacctggacg cctgtgagac aggcactgtg cgggccaggt atacctttgg gaaacctgtg 780
gctggtgcct taatgatcaa catgactgtt aatggtgtag ggtactacag ccacgaggtg 840
ggacgccctg tcctcagaac aaccaagatc ctcggctccc gggacttcga catctgcgtg 900
agggacatga tcccagcgga cgtccctgag cacttccggg gcagggtcag catctgggcc 960
atggtgacca gtgtggacgg gagccagcag gtcgcgttcg atgactccac ccccgtgcag 1020
aggcagctgg tggacatccg gtactccaag gacacgagga agcagttcaa gccgggcctg 1080
gcctacgtgg ggaaggtgga gctatcctac cccgatggca gcccagctga gggggtgacg 1140
gtccagatta aggcagagct gacaccaaag gataacatct acaccagtga agttgtgtcc 1200
cagcgtggac tagtggggtt tgaaatcccc tccatcccca cgtcagccca gcacgtgtgg 1260
ctggagacca aggtgatggc actgaacggg aagcccgtgg gggctcagta cctgcccagc 1320
tacctctccc tcggcagctg gtactccccc agccagtgct acctgcagct gcagccaccc 1380
tcccacccac tgcaggttgg ggaagaagcc tatttttctg tgaagtccac atgtccctgc 1440
aactttaccc tgtactacga ggtggctgca cggggcaata ttgtgctatc gggccagcag 1500
cctgcccaca ccacccagca gcgaagcaag cgggcggccc ctgccctgga gaaaccgatt 1560
cgtttaacac acctttctga gacagagccc ccaccagccc cagaagctga ggtcgacgtg 1620

tgtgtgacct ctcttcatct ggccgtgacc cccagcatgg tccccccttgg tcgcctgctg 1680
gtcttctacg tcagggagaa tggagaaggg gtcgccgaca gccttcagtt tgcagtcgag 1740
accttcttcg aaaaccaggt ttcagtgacg tattcagcaa atgagaccca acctggggag 1800
gttgtcgacc tgcggatcag ggctgcaagg ggcagctgtg tgtgcgtcgc cgcagttgat 1860
aagagtgtct acctgctcag gtctgggttc cggctgactc ctgcccaggt tttccaggaa 1920
ctggaagatt atgatgtttc tgattccttt ggcgtgtcca gggaggatgg tcctttttgg 1980
tgggctgggc tgacggcaca acgacgccgg cgctcctctg tcttcccgtg gccttggggc 2040
atcaccaagg actctgggtt tgccttcacc gaaacgggac tggtggtgat gaccgaccga 2100
gtgagcctga accaccggca ggacggtggc ctctacaccg atgaggctgt ccccgctttc 2160
cagccccaca cagggagcct ggtggcagtg gctccttcca ggcacccccc cagaacagag 2220
aagagaaaaa ggactttctt ccccgaaaca tggatttggc attgtctcaa catcagtgac 2280
ccatctggtg aggggacact cagtgtgaag gtcccggact ccatcaccag ctgggtgggt 2340
gaggccgtgg ccctgtccac ctctcagggc ttaggcatcg ccgagccctc cctgctgaag 2400
accttcaagc ccttcttcgt ggacttcatg ctccccgctc tcatcatccg tggggagcag 2460
gtcaagatcc cgctcagtgt ctacaactac atgggcacct gcgctgaggt gtacatgaag 2520
ctctcggttc ccaagggcat ccagtttgtt gggcatcctg gcaaacgcca tgtgaccaag 2580
aagatgtgtg tggcccccgg ggaggctgag cccatctggg tcgttctgtc cttcagcgac 2640
ctgggactca acaacatcac ggccaaagcc cttgcttacg gagacacaaa ttgctgccgg 2700
gatgggaggt ccagcaaaca ccctgaggag aatcacgccg acaggagggt ccccatcggg 2760
gtggatcacg tcaggcgcag tgtgatggtt gaggcggaag gagtcccccg ggcgtacacc 2820
tacagcgcat tcttctgtcc cagtgagaga gtccacatct ccacccccaa caagtatgag 2880
ttccagtatg tgcagcggcc actgcgcctc acccgctttg atgtggctgt gcgagctcac 2940
aatgatgccc gtgtggcctt gtcttctggg ccccaggaca cagcaggcat gatcgagatc 3000
gtcctggggg ggcatcagaa caccaggtca tggatctcca ccagcaagat gggagagccc 3060
gtggccagtg cacacacggc caagatcctc tcctgggatg aattcagaac attctggatc 3120
agctggcgtg gtggccttat ccaggttggc catggtccag agccatccaa tgagtctgtc 3180
attgtggcct ggaccctccc gaggccacca gaggtccagt tcattggctt ttccaccggc 3240
tggggctcca tgggtgaatt ccgaatctgg aggaagatgg aggtggacga gagctacagc 3300
gaggccttca ccctgggggt cccacacggc gccatccctg ggtctgagcg agccaccgcc 3360
tccatcatcg gggacgtcat ggggccaacc ctgaaccacc tcaacaacct cctgcggctg 3420
ccgtttggct gtggagagca gaacatgatc cactttgcac ccaacgtctt tgtcttgaag 3480
tatcttcaga aaacccagca gctcagccct gaggtggaga gagagaccac cgactaccta 3540
gtacaaggct accagcgcca gctgacctac aagcgccagg atggctccta cagcgcgttt 3600
ggggagcggg acgcatcggg gagcatgtgg ctcacagcct ttgtcctgaa gtccttcgca 3660
caggctcgca gctttatctt cgtggacccc cgggagctgg ctgccgccaa gagctggatc 3720
atccagcagc agcaggccga tggctccttc ctggccgtgg gcagggtcct gaacaaggac 3780
atccagggtg ggatccacgg cattgtcccg ctgacagcct acgtggtggt tgctctcctg 3840
gaaacaggca cagcctcaga ggaggagaga ggctccactg acaaagcgag gcacttcctg 3900
gagtctgctg cgcccctggc catggaccct tatagctgtg ccctgactac ctacgcgctg 3960
accctgctcc gcagcccggc agcccctgag gcactgcgca agctccgtag cctggccatc 4020
atgcgagatg gggtcaccca ctggagcctg tcaaattcct gggacgtgga caagggcaca 4080
ttcttgagct tcagtgacag ggtctctcag tcagtggtct cggccgaggt ggaaatgaca 4140
gcctacgccc ttctgaccta cactctgctg ggtgacgtgg ctgccgccct gcctgtggtg 4200
aagtggctgt cccagcagcg aaatgcactt gggggcttct cctccactca ggacacctgc 4260
gtggctctgc aggccttggc tgaatatgcc atcttgtcct atgctggagg catcaacctc 4320
actgtctccc tggcctccac caacctggac taccaggaaa ccttcgagct gcacaggacc 4380
aaccagaagg ttctgcagac agcagcgatc cccagcctcc ccacggggct gtttgtgagt 4440
gccaaggggg acggctgctg cctgatgcag attgatgtca cctacaatgt gcctgacccg 4500
gtggccaagc cagctttcca gctgctcgta agcctccagg agcctgaggc ccagggacgc 4560
ccgcccccca tgcctgcctc cgcagctgag ggttcccgag gagactggcc cccagctgac 4620
gatgatgacc cagcggccga tcagcatcac caggaataca aggtgatgct ggaggtgtgc 4680
accaggtggc tgcatgcagg gtcttccaat atggctgtcc tggaggtgcc cctgctgtca 4740
ggcttccggg cagacatcga gagcctggag cagctgctcc ttgacaagca catggggatg 4800
aagaggtatg aagtggctgg acgccgagtg ctcttctact ttgatgagat ccccagccgg 4860
tgcctgacgt gcgtgcggtt ccgtgctctc cgggagtgcg tggtgggcag gacgtcggcg 4920
ctgccagtct ccgtgtacga ctactacgaa cccgccttcg aggccactcg cttctacaac 4980
gtcagcacgc acagcccact cgcccgggaa ctgtgcgccg gacccgcgtg caacgaagtg 5040
gagcgcgccc ctgcccgggg cccgggctgg ttccccggcg agtcgggccc tgccgtggcc 5100
cctgaggagg gggcggcgat cgcgcgatgc ggctgcgacc acgactgcgg cgcccagggg 5160
aacccggtgt gcggctccga cggggtggtc tacgccagcg cctgccgcct gcgggaggcc 5220
gcctgccgcc aggccgcgcc cctggagccc gcgcctccca gctgctgcgc cctcgagcag 5280
cggctgccgg cctcgtcgtc ctccacctac ggggatgacc tggcttctgt ggccccgggg 5340
cctttacagc aggacgtgaa gctgaatgga gccggccttg aggtggagga ctcagaccct 5400
gagcctgaag gggaggcgga ggacagggtc acagccgggc ctcggcctcc tgtgagcagc 5460
gggaacctgg aaagcagcac ccagagcgcc agcccgttcc acagatgggg ccagactccg 5520
gcccctcaga gacatagtgg ccgggtggtg gggcccaca ggccagggct tctgagccct 5580
gtcttcgtct acagcccagc ctttcagagt ggtggggagg agggtttatg gatgtcaaac 5640
acctgcacct tgagataatc ctacaaccac atgcagttgt gggaccgcag tttggtcctg 5700
gggaccattc atacccacac acccagcttg tgcctgtggt taacatctca gaaaactctg 5760

gtaaatgatc actccaggat attgacacga atacacgtta ctgatcttac tcacatgttc 5820
tggggtgcac atgaactttg tgtgtgcatg tgtgtgtgtg tgcatgtgtg tgtcccgggc 5880
acctgacacc cccagcccag ggctgcccaa agttgggctg atcagagaca tagacccaat 5940
gaggagccca acagtggccc tccaaccctc tgccttgccc ccatagttca tgccccagtg 6000
gtctttgaaa ctgccctgtg ccactccctg gagtgagcag cggtgtctct gtgtgtgtgt 6060
gtgtctgtg 6069

<210> 28
<211> 1885
<212> PRT
<213> Homo sapiens

<400> 28
Met Ser Gly Ala Leu Leu Trp Pro Leu Leu Pro Leu Leu Leu Leu Leu
1               5                   10                  15

Leu Ser Ala Arg Asp Gly Val Arg Ala Ala Gln Pro Gln Ala Pro Gly
            20                  25                  30

Tyr Leu Ile Ala Ala Pro Ser Val Phe Arg Ala Gly Val Glu Glu Val
        35                  40                  45

Ile Ser Val Thr Ile Phe Asn Ser Pro Arg Glu Val Thr Val Gln Ala
    50                  55                  60

Gln Leu Val Ala Gln Gly Glu Pro Val Val Gln Ser Gln Gly Ala Ile
65                  70                  75                  80

Leu Asp Lys Gly Thr Ile Lys Leu Lys Val Pro Thr Gly Leu Arg Gly
                85                  90                  95

Gln Ala Leu Leu Lys Val Trp Gly Arg Gly Trp Gln Ala Glu Glu Gly
            100                 105                 110

Pro Leu Phe His Asn Gln Thr Ser Val Thr Val Asp Gly Arg Gly Ala
        115                 120                 125

Ser Val Phe Ile Gln Thr Asp Lys Pro Val Tyr Arg Pro Gln His Arg
    130                 135                 140

Val Leu Ile Ser Ile Phe Thr Val Ser Pro Asn Leu Arg Pro Val Asn
145                 150                 155                 160

Glu Lys Leu Glu Ala Tyr Ile Leu Asp Pro Arg Gly Ser Arg Met Ile
                165                 170                 175

Glu Trp Arg His Leu Lys Pro Phe Cys Cys Gly Ile Thr Asn Met Ser
            180                 185                 190

Phe Pro Leu Ser Asp Gln Pro Val Leu Gly Glu Trp Phe Ile Phe Val
        195                 200                 205

Glu Met Gln Gly His Ala Tyr Asn Lys Ser Phe Glu Val Gln Lys Tyr
    210                 215                 220

Val Leu Pro Lys Phe Glu Leu Leu Ile Asp Pro Pro Arg Tyr Ile Gln
225                 230                 235                 240

Asp Leu Asp Ala Cys Glu Thr Gly Thr Val Arg Ala Arg Tyr Thr Phe
                245                 250                 255

Gly Lys Pro Val Ala Gly Ala Leu Met Ile Asn Met Thr Val Asn Gly
            260                 265                 270

Val Gly Tyr Tyr Ser His Glu Val Gly Arg Pro Val Leu Arg Thr Thr
        275                 280                 285

Lys Ile Leu Gly Ser Arg Asp Phe Asp Ile Cys Val Arg Asp Met Ile

290                 295                 300

Pro Ala Asp Val Pro Glu His Phe Arg Gly Arg Val Ser Ile Trp Ala
305                 310                 315                 320

Met Val Thr Ser Val Asp Gly Ser Gln Gln Val Ala Phe Asp Asp Ser
                325                 330                 335

Thr Pro Val Gln Arg Gln Leu Val Asp Ile Arg Tyr Ser Lys Asp Thr
            340                 345                 350

Arg Lys Gln Phe Lys Pro Gly Leu Ala Tyr Val Gly Lys Val Glu Leu
        355                 360                 365

Ser Tyr Pro Asp Gly Ser Pro Ala Glu Gly Val Thr Val Gln Ile Lys
    370                 375                 380

Ala Glu Leu Thr Pro Lys Asp Asn Ile Tyr Thr Ser Glu Val Val Ser
385                 390                 395                 400

Gln Arg Gly Leu Val Gly Phe Glu Ile Pro Ser Ile Pro Thr Ser Ala
                405                 410                 415

Gln His Val Trp Leu Glu Thr Lys Val Met Ala Leu Asn Gly Lys Pro
            420                 425                 430

Val Gly Ala Gln Tyr Leu Pro Ser Tyr Leu Ser Leu Gly Ser Trp Tyr
        435                 440                 445

Ser Pro Ser Gln Cys Tyr Leu Gln Leu Gln Pro Pro Ser His Pro Leu
    450                 455                 460

Gln Val Gly Glu Glu Ala Tyr Phe Ser Val Lys Ser Thr Cys Pro Cys
465                 470                 475                 480

Asn Phe Thr Leu Tyr Tyr Glu Val Ala Ala Arg Gly Asn Ile Val Leu
                485                 490                 495

Ser Gly Gln Gln Pro Ala His Thr Thr Gln Gln Arg Ser Lys Arg Ala
            500                 505                 510

Ala Pro Ala Leu Glu Lys Pro Ile Arg Leu Thr His Leu Ser Glu Thr
        515                 520                 525

Glu Pro Pro Pro Ala Pro Glu Ala Glu Val Asp Val Cys Val Thr Ser
    530                 535                 540

Leu His Leu Ala Val Thr Pro Ser Met Val Pro Leu Gly Arg Leu Leu
545                 550                 555                 560

Val Phe Tyr Val Arg Glu Asn Gly Glu Gly Val Ala Asp Ser Leu Gln
                565                 570                 575

Phe Ala Val Glu Thr Phe Phe Glu Asn Gln Val Ser Val Thr Tyr Ser
            580                 585                 590

Ala Asn Glu Thr Gln Pro Gly Glu Val Val Asp Leu Arg Ile Arg Ala
        595                 600                 605

Ala Arg Gly Ser Cys Val Cys Val Ala Ala Val Asp Lys Ser Val Tyr
    610                 615                 620

Leu Leu Arg Ser Gly Phe Arg Leu Thr Pro Ala Gln Val Phe Gln Glu
625                 630                 635                 640

Leu Glu Asp Tyr Asp Val Ser Asp Ser Phe Gly Val Ser Arg Glu Asp
                645                 650                 655

Gly Pro Phe Trp Trp Ala Gly Leu Thr Ala Gln Arg Arg Arg Ser

            660                 665                 670

Ser Val Phe Pro Trp Pro Trp Gly Ile Thr Lys Asp Ser Gly Phe Ala
            675                 680                 685

Phe Thr Glu Thr Gly Leu Val Val Met Thr Asp Arg Val Ser Leu Asn
        690                 695                 700

His Arg Gln Asp Gly Gly Leu Tyr Thr Asp Glu Ala Val Pro Ala Phe
705 710 715 720

Gln Pro His Thr Gly Ser Leu Val Ala Val Ala Pro Ser Arg His Pro
725 730 735

Pro Arg Thr Glu Lys Arg Lys Arg Thr Phe Phe Pro Glu Thr Trp Ile
                740                 745                 750

Trp His Cys Leu Asn Ile Ser Asp Pro Ser Gly Glu Gly Thr Leu Ser
            755                 760                 765

Val Lys Val Pro Asp Ser Ile Thr Ser Trp Val Gly Glu Ala Val Ala
        770                 775                 780

Leu Ser Thr Ser Gln Gly Leu Gly Ile Ala Glu Pro Ser Leu Leu Lys
785 790 795 800

Thr Phe Lys Pro Phe Phe Val Asp Phe Met Leu Pro Ala Leu Ile Ile
805 810 815

Arg Gly Glu Gln Val Lys Ile Pro Leu Ser Val Tyr Asn Tyr Met Gly
                820                 825                 830

Thr Cys Ala Glu Val Tyr Met Lys Leu Ser Val Pro Lys Gly Ile Gln
            835                 840                 845

Phe Val Gly His Pro Gly Lys Arg His Val Thr Lys Lys Met Cys Val
        850                 855                 860

Ala Pro Gly Glu Ala Glu Pro Ile Trp Val Val Leu Ser Phe Ser Asp
865 870 875 880

Leu Gly Leu Asn Asn Ile Thr Ala Lys Ala Leu Ala Tyr Gly Asp Thr
885 890 895

Asn Cys Cys Arg Asp Gly Arg Ser Ser Lys His Pro Glu Glu Asn His
                900                 905                 910

Ala Asp Arg Arg Val Pro Ile Gly Val Asp His Val Arg Arg Ser Val
            915                 920                 925

Met Val Glu Ala Glu Gly Val Pro Arg Ala Tyr Thr Tyr Ser Ala Phe
        930                 935                 940

Phe Cys Pro Ser Glu Arg Val His Ile Ser Thr Pro Asn Lys Tyr Glu
945 950 955 960

Phe Gln Tyr Val Gln Arg Pro Leu Arg Leu Thr Arg Phe Asp Val Ala
965 970 975

Val Arg Ala His Asn Asp Ala Arg Val Ala Leu Ser Ser Gly Pro Gln
                980                 985                 990

Asp Thr Ala Gly Met Ile Glu Ile Val Leu Gly Gly His Gln Asn Thr
            995                 1000                1005

Arg Ser Trp Ile Ser Thr Ser Lys Met Gly Glu Pro Val Ala Ser Ala
        1010                1015                1020

His Thr Ala Lys Ile Leu Ser Trp Asp Glu Phe Arg Thr Phe Trp Ile

1025                1030                1035                1040

Ser Trp Arg Gly Gly Leu Ile Gln Val Gly His Gly Pro Glu Pro Ser
                1045                1050                1055

Asn Glu Ser Val Ile Val Ala Trp Thr Leu Pro Arg Pro Pro Glu Val
            1060                1065                1070

Gln Phe Ile Gly Phe Ser Thr Gly Trp Gly Ser Met Gly Glu Phe Arg
        1075                1080                1085

Ile Trp Arg Lys Met Glu Val Asp Glu Ser Tyr Ser Glu Ala Phe Thr
    1090                1095                1100

Leu Gly Val Pro His Gly Ala Ile Pro Gly Ser Glu Arg Ala Thr Ala
1105                1110                1115                1120

Ser Ile Ile Gly Asp Val Met Gly Pro Thr Leu Asn His Leu Asn Asn
                1125                1130                1135

Leu Leu Arg Leu Pro Phe Gly Cys Gly Glu Gln Asn Met Ile His Phe
            1140                1145                1150

Ala Pro Asn Val Phe Val Leu Lys Tyr Leu Gln Lys Thr Gln Gln Leu
        1155                1160                1165

Ser Pro Glu Val Glu Arg Glu Thr Thr Asp Tyr Leu Val Gln Gly Tyr
    1170                1175                1180

Gln Arg Gln Leu Thr Tyr Lys Arg Gln Asp Gly Ser Tyr Ser Ala Phe
1185                1190                1195                1200

Gly Glu Arg Asp Ala Ser Gly Ser Met Trp Leu Thr Ala Phe Val Leu
                1205                1210                1215

Lys Ser Phe Ala Gln Ala Arg Ser Phe Ile Phe Val Asp Pro Arg Glu
            1220                1225                1230

Leu Ala Ala Ala Lys Ser Trp Ile Ile Gln Gln Gln Gln Ala Asp Gly
        1235                1240                1245

Ser Phe Leu Ala Val Gly Arg Val Leu Asn Lys Asp Ile Gln Gly Gly
    1250                1255                1260

Ile His Gly Ile Val Pro Leu Thr Ala Tyr Val Val Ala Leu Leu
1265                1270                1275                1280

Glu Thr Gly Thr Ala Ser Glu Glu Glu Arg Gly Ser Thr Asp Lys Ala
                1285                1290                1295

Arg His Phe Leu Glu Ser Ala Ala Pro Leu Ala Met Asp Pro Tyr Ser
            1300                1305                1310

Cys Ala Leu Thr Thr Tyr Ala Leu Thr Leu Leu Arg Ser Pro Ala Ala
        1315                1320                1325

Pro Glu Ala Leu Arg Lys Leu Arg Ser Leu Ala Ile Met Arg Asp Gly
    1330                1335                1340

Val Thr His Trp Ser Leu Ser Asn Ser Trp Asp Val Asp Lys Gly Thr
1345                1350                1355                1360

Phe Leu Ser Phe Ser Asp Arg Val Ser Gln Ser Val Val Ser Ala Glu
                1365                1370                1375

Val Glu Met Thr Ala Tyr Ala Leu Leu Thr Tyr Thr Leu Leu Gly Asp
            1380                1385                1390

Val Ala Ala Ala Leu Pro Val Val Lys Trp Leu Ser Gln Gln Arg Asn

1395                1400                1405

Ala Leu Gly Gly Phe Ser Ser Thr Gln Asp Thr Cys Val Ala Leu Gln
    1410                1415                1420

Ala Leu Ala Glu Tyr Ala Ile Leu Ser Tyr Ala Gly Gly Ile Asn Leu
1425                1430                1435                1440

Thr Val Ser Leu Ala Ser Thr Asn Leu Asp Tyr Gln Glu Thr Phe Glu
                1445                1450                1455

Leu His Arg Thr Asn Gln Lys Val Leu Gln Thr Ala Ala Ile Pro Ser
            1460                1465                1470

Leu Pro Thr Gly Leu Phe Val Ser Ala Lys Gly Asp Gly Cys Cys Leu
        1475                1480                1485

Met Gln Ile Asp Val Thr Tyr Asn Val Pro Asp Pro Val Ala Lys Pro
    1490                1495                1500

Ala Phe Gln Leu Leu Val Ser Leu Gln Glu Pro Glu Ala Gln Gly Arg
1505                1510                1515                1520

Pro Pro Pro Met Pro Ala Ser Ala Ala Glu Gly Ser Arg Gly Asp Trp
                1525                1530                1535

Pro Pro Ala Asp Asp Asp Asp Pro Ala Ala Asp Gln His His Gln Glu
            1540                1545                1550

Tyr Lys Val Met Leu Glu Val Cys Thr Arg Trp Leu His Ala Gly Ser
        1555                1560                1565

Ser Asn Met Ala Val Leu Glu Val Pro Leu Leu Ser Gly Phe Arg Ala
    1570                1575                1580

Asp Ile Glu Ser Leu Glu Gln Leu Leu Leu Asp Lys His Met Gly Met
1585                1590                1595                1600

Lys Arg Tyr Glu Val Ala Gly Arg Arg Val Leu Phe Tyr Phe Asp Glu
                1605                1610                1615

Ile Pro Ser Arg Cys Leu Thr Cys Val Arg Phe Arg Ala Leu Arg Glu
            1620                1625                1630

Cys Val Val Gly Arg Thr Ser Ala Leu Pro Val Ser Val Tyr Asp Tyr
        1635                1640                1645

Tyr Glu Pro Ala Phe Glu Ala Thr Arg Phe Tyr Asn Val Ser Thr His
    1650                1655                1660

Ser Pro Leu Ala Arg Glu Leu Cys Ala Gly Pro Ala Cys Asn Glu Val
1665                1670                1675                1680

Glu Arg Ala Pro Ala Arg Gly Pro Gly Trp Phe Pro Gly Glu Ser Gly
                1685                1690                1695

Pro Ala Val Ala Pro Glu Glu Gly Ala Ala Ile Ala Arg Cys Gly Cys
            1700                1705                1710

Asp His Asp Cys Gly Ala Gln Gly Asn Pro Val Cys Gly Ser Asp Gly
        1715                1720                1725

Val Val Tyr Ala Ser Ala Cys Arg Leu Arg Glu Ala Ala Cys Arg Gln
    1730                1735                1740

Ala Ala Pro Leu Glu Pro Ala Pro Pro Ser Cys Cys Ala Leu Glu Gln
1745                1750                1755                1760

Arg Leu Pro Ala Ser Ser Ser Ser Thr Tyr Gly Asp Asp Leu Ala Ser

1765                1770                1775

Val Ala Pro Gly Pro Leu Gln Gln Asp Val Lys Leu Asn Gly Ala Gly
        1780                1785                1790

Leu Glu Val Glu Asp Ser Asp Pro Glu Pro Glu Gly Glu Ala Glu Asp
    1795                1800                1805

Arg Val Thr Ala Gly Pro Arg Pro Pro Val Ser Ser Gly Asn Leu Glu
    1810                1815                1820

Ser Ser Thr Gln Ser Ala Ser Pro Phe His Arg Trp Gly Gln Thr Pro
1825                1830                1835                1840

Ala Pro Gln Arg His Ser Gly Arg Val Val Gly Ala His Arg Pro Gly
                1845                1850                1855

Leu Leu Ser Pro Val Phe Val Tyr Ser Pro Ala Phe Gln Ser Gly Gly
            1860                1865                1870

Glu Glu Gly Leu Trp Met Ser Asn Thr Cys Thr Leu Arg
        1875                1880                1885

<210> 29
<211> 6157
<212> DNA
<213> Homo sapiens

<400> 29
gtgagtaagt gaggggacga tccccggaag ggatcggggc gggtcggggt ccggagatgg 60
gcggagcagg cgtcccggga gggtgcgccc aggagcgggg cgagcggggc gagcggggcg 120
gtcccggaga cgaggcgggt ccggggaggg ggctggcccg gggctgcccc agcttggccg 180
ggcgcggagc ggggcgcatg gcgccgggcg cactgcgcgg gggctgcgaa caaagggccc 240
ccggcggcgg cgcgaggacg gccgcgctcg gaccctggcc ctggcccagc cctggcccgg 300
ccccctcccc aggcgcggcg ccccccagga gccgaaaatg agcggcgccc ctgctctggc 360
cgttgctccc gctcctgctc ctgctgctgt cggcgcggga cggcgtgcgc gccgcgcagc 420
ctcaggcccc gggttacttg attgcagctc cctctgtttt tcgcgcgggc gtggaggaag 480
tcatcagcgt gaccatcttt aactctccaa gggaagtcac ggtccaggct cagctggtgg 540
cccagggtga gccggtggtg cagagccagg gagccatcct ggataaaggg acaatcaaac 600
tcaaggtgcc cacgggcctc cggggccaag cgcttctgaa agtgtggggc cgcggctggc 660
aggcggagga ggggcccctc tttcacaacc agacctcggt gaccgtggac ggccggggcg 720
cttctgtatt catccagacg gacaagcctg tgtacagacc ccagcaccga gtgctcataa 780
gcatcttcac cgtctctcca aatctgaggc ctgtcaacga gaagctggaa gcctacatcc 840
tggacccccg aggctctcgg atgatagagt ggagacactt aaagccgttc tgctgcggca 900
tcaccaacat gagcttcccc ttgtccgacc agcctgtgtt gggagaatgg ttcatttttg 960
ttgaaatgca aggccacgcg tacaacaagt cttttgaagt tcagaagtat gtgttgccca 1020
agtttgagct tctgattgac ccgccccggt atatccaaga cctggacgcc tgtgagacag 1080
gcactgtgcg ggccaggtat acctttggga aacctgtggc tggtgcctta acgatcaaca 1140
tgactgttaa tggtgtaggg tactacagcc acgaggtggg acgccctgtc ctcagaacaa 1200
ccaagatcct cggctcccag gacttcgaca tctgcgtgag ggacatgatc ccagcggacg 1260
tccctgagca cttccggggc agggtcagca tctgggccat ggtgaccagt gtggacggga 1320
gccagcaggt cgcgttcgat gactccaccc ccgtgcagag gcagctggtg gacatccggt 1380
actccaagga cacgaggaag cagttcaagc cgggcctggc ctacgtgggg aaggtggagc 1440
tatcctaccc cgatggcagc ccagctgagg gggtgacggt ccagattaag gcagagctga 1500
caccaaagga taacatctac accagtgaag ttgtgtccca gcgtggacta gtggggtttg 1560
aaatcccctc catccccacg tcagcccagc acgtgtggct ggagaccaag gtgatggcac 1620
tgaacgggaa gcccgtgggg gctcagtacc tgcccagcta cctctccctc ggcagctggt 1680
actcccccag ccagtgctac ctgcagctgc agccaccctc ccacccactg caggttgggg 1740
aagaagccta tttttctgtg aagtccacat gtccctgcaa ctttaccctg tactacgagg 1800
tggctgcacg gggcaatatt gtgctatcgg gccagcagcc tgcccacacc acccagcagc 1860
gaagcaagcg ggcggcccct gccctggaga aaccgattcg tttaacacac ctttctgaga 1920
cagagccccc accagcccca gaagctgagg tcgacgtgtg tgtgacctct cttcatctgg 1980
ccgtgacccc cagcatggtc cccccttggtc gcctgctggt cttctacgtc agggagaatg 2040
gagaaggggt cgccgacagc cttcagtttg cagtcgagac cttcttcgaa aaccaggttt 2100
cagtgacgta ttcagcaaat gagacccaac ctggggaggt tgtcgacctg cggatcaggg 2160
ctgcaagggg cagctgtgtg tgcgtcgccg cagttgataa gagtgtctac ctgctcaggt 2220
ctgggttccg gctgactcct gcccaggttt tccaggaact ggaagattat gatgtttctg 2280
attcctttgg cgtgtccagg gaggatggtc ctttttggtg ggctgggctg acggcacaac 2340

gacgccggcg ctcctctgtc ttcccgtggc cttggggcat caccaaggac tctgggtttg 2400
ccttcaccga aacgggactg gtggtgatga ccgaccgagt gagcctgaac caccggcagg 2460
acggtggcct ctacaccgat gaggctgtcc ccgctttcca gccccacaca gggagcctgg 2520
tggcagtggc tccttccagg caccccccca gaacagagaa gagaaaaagg actttcttcc 2580
ccgaaacatg gatttggcat tgtctcaaca tcagtgaccc atctggtgag gggacactca 2640
gtgtgaaggt cccggactcc atcaccagct gggtgggtga ggccgtggcc ctgtccacct 2700
ctcagggctt aggcatcgcc gagccctccc tgctgaagac cttcaagccc ttcttcgtgg 2760
acttcatgct ccccgctctc atcatccgtg gggagcaggt caagatcccg ctcagtgtct 2820
acaactacat gggcacctgc gctgaggtgt acatgaagct ctcggttccc aagggcatcc 2880
agtttgttgg gcatcctggc aaacgccatg tgaccaagaa gatgtgtgtg gcccccgggg 2940
aggctgagcc catctgggtc gttctgtcct tcagcgacct gggactcaac aacatcacgg 3000
ccaaagccct tgcttacgga gacacaaatt gctgccggga tgggaggtcc agcaaacacc 3060
ctgaggagaa tcacgccgac aggagggtcc ccatcggggt ggatcacgtc aggcgcagtg 3120
tgatggttga ggcggaagga gtcccccggg cgtacaccta cagcgcattc ttctgtccca 3180
gtgagagagt ccacatctcc acccccaaca agtatgagtt ccagtatgtg cagcggccac 3240
tgcgcctcac ccgctttgat gtggctgtgc gagctcacaa tgatgcccgt gtggccttgt 3300
cttctgggcc ccaggacaca gcaggcatga tcgagatcgt cctggggggg catcagaaca 3360
ccaggtcatg gatctccacc agcaagatgg gagagcccgt ggccagtgca cacacggcca 3420
agatcctctc ctgggatgaa ttcagaacat tctggatcag ctggcgtggt ggccttatcc 3480
aggttggcca tggtccagag ccatccaatg agtctgtcat tgtggcctgg accctcccga 3540
ggccaccaga ggtccagttc attggctttt ccaccggctg ggctccatg ggtgaattcc 3600
gaatctggag gaagatggag gtggacgaga gctacagcga ggccttcacc ctgggggtcc 3660
cacacggcgc catccctggg tctgagcgag ccaccgcctc catcatcggg gacgtcatgg 3720
ggccaaccct gaaccacctc aacaacctcc tgcggctgcc gtttggctgt ggagagcaga 3780
acatgatcca ctttgcaccc aacgtctttg tcttgaagta tcttcagaaa acccagcagc 3840
tcagccctga ggtggagaga gagaccaccg actacctagt acaaggctac cagcgccagc 3900
tgacctacaa gcgccaggat ggctcctaca gcgcgtttgg ggagcgggac gcatcgggga 3960
gcatgtggct cacagccttt gtcctgaagt ccttcgcaca ggctcgcagc tttatcttcg 4020
tggacccccg ggagctggct gccgccaaga gctggatcat ccagcagcag caggccgatg 4080
gctccttcct ggccgtgggc agggtcctga acaaggacat ccagggtggg atccacggca 4140
ttgtcccgct gacagcctac gtggtggttg ctctcctgga aacaggcaca gcctcagagg 4200
aggagagagg ctccactgac aaagcgaggc acttcctgga gtctgctgcg cccctggcca 4260
tggaccctta tagctgtgcc ctgactacct acgcgctgac cctgctccgc agcccggcag 4320
cccctgaggc actgcgcaag ctccgtagcc tggccatcat gcgagatggg gtcacccact 4380
ggagcctgtc aaattcctgg gacgtggaca agggcacatt cttgagcttc agtgacaggg 4440
tctctcagtc agtggtctcg gccgaggtgg aaatgacagc ctacgccctt ctgacctaca 4500
ctctgctggg tgacgtggct gccgccctgc ctgtggtgaa gtggctgtcc cagcagcgaa 4560
atgcacttgg gggcttctcc tccactcagg acacctgcgt ggctctgcag gccttggctg 4620
aatatgccat cttgtcctat gctggaggca tcaacctcac tgtctccctg gcctccacca 4680
acctggacta ccaggaaacc ttcgagctgc acaggaccaa ccagaaggtt ctgcagacag 4740
cagcgatccc cagcctcccc acggggctgt ttgtgagtgc caagggggac ggctgctgcc 4800
tgatgcagat tgatgtcacc tacaatgtgc ctgacccggt ggccaagcca gctttccagc 4860
tgctcgtaag cctccaggag cctgaggccc agggacgccc gcccccatg cctgcctccg 4920
cagctgaggg ttcccgagga gactggcccc cagctgacga tgatgaccca gcggccgatc 4980
agcatcacca ggaatacaag gtgatgctgg aggtgtgcac caggtggctg catgcagggt 5040
cttccaatat ggctgtcctg gaggtgcccc tgctgtcagg cttccgggca gacatcgaga 5100
gcctggagca gctgctcctt gacaagcaca tggggatgaa gaggtatgaa gtggctggac 5160
gccgagtgct cttctacttt gatgagatcc ccagccggtg cctgacgtgc gtgcggttcc 5220
gtgctctccg ggagtgcgtg gtgggcagga cgtcggcgct gccagtctcc gtgtacgact 5280
actacgaacc cgccttcgag gccactcgct tctacaacgt cagcacgcac agcccactcg 5340
cccgggaact gtgcgccgga cccgcgtgca acgaagtgga gcgcgcccct gcccggggcc 5400
cgggctggtt ccccggcgag tcgggccctg ccgtggcccc tgaggagggg gcggcgatcg 5460
cgcgatgcgg ctgcgaccac gactgcggcg cccaggggaa cccggtgtgc ggctccgacg 5520
gggtggtcta cgccagcgcc tgccgcctgc gggaggccgc ctgccgccag gccgcgcccc 5580
tggagcccgc gcctcccagc tgctgcgccc tcgagcagcg gctgccggcc tcgtcgtcct 5640
ccacctacgg ggatgacctg gcttctgtgg ccccggggcc tttacagcag gacgtgaagc 5700
tgaatggagc cggccttgag gtggaggact cagaccctga gcctgaaggg gaggcggagg 5760
acagggtcac agccgggcct cggcctcctg tgagcagcgg gaacctggaa agcagcaccc 5820
agagcgccag cccgttccac agatggggcc agactccggc ccctcagaga catagtggcc 5880
gggtggtggg ggcccacagg ccagggcttc tgagccctgt cttcgtctac agcccagcct 5940
ttcagagtgg tggggaggag ggtttatgga tgtcaaacac ctgcaccttg agataatcct 6000
acaaccacat gcagttgtgg gaccgcagtt tggtcctggg gaccattcat acccacacac 6060
ccagcttgtg cctgtggtta acatctcaga aaactctggt aaatgatcac tccaggatat 6120
tgacacgaat acacgttact gatcttactc acatgtt 6157

<210> 30
<211> 1979
<212> PRT

<213> Homo sapiens

<400> 30
Met Gly Gly Ala Gly Val Pro Gly Gly Cys Ala Gln Glu Arg Gly Glu
1               5                   10                  15

Arg Gly Glu Arg Gly Gly Pro Gly Asp Glu Ala Gly Pro Gly Arg Gly
            20                  25                  30

Leu Ala Arg Gly Cys Pro Ser Leu Ala Gly Arg Gly Ala Gly Arg Met
        35                  40                  45

Ala Pro Gly Ala Leu Arg Gly Gly Cys Glu Gln Arg Ala Pro Gly Gly
    50                  55                  60

Gly Ala Arg Thr Ala Ala Leu Gly Pro Trp Pro Trp Pro Ser Pro Gly
65                  70                  75                  80

Pro Ala Pro Ser Pro Gly Ala Ala Pro Pro Arg Ser Arg Lys Met Ser
                85                  90                  95

Gly Ala Leu Leu Trp Pro Leu Leu Pro Leu Leu Leu Leu Leu Leu Ser
            100                 105                 110

Ala Arg Asp Gly Val Arg Ala Ala Gln Pro Gln Ala Pro Gly Tyr Leu
        115                 120                 125

Ile Ala Ala Pro Ser Val Phe Arg Ala Gly Val Glu Glu Val Ile Ser
    130                 135                 140

Val Thr Ile Phe Asn Ser Pro Arg Glu Val Thr Val Gln Ala Gln Leu
145                 150                 155                 160

Val Ala Gln Gly Glu Pro Val Val Gln Ser Gln Gly Ala Ile Leu Asp
                165                 170                 175

Lys Gly Thr Ile Lys Leu Lys Val Pro Thr Gly Leu Arg Gly Gln Ala
            180                 185                 190

Leu Leu Lys Val Trp Gly Arg Gly Trp Gln Ala Glu Glu Gly Pro Leu
        195                 200                 205

Phe His Asn Gln Thr Ser Val Thr Val Asp Gly Arg Gly Ala Ser Val
    210                 215                 220

Phe Ile Gln Thr Asp Lys Pro Val Tyr Arg Pro Gln His Arg Val Leu
225                 230                 235                 240

Ile Ser Ile Phe Thr Val Ser Pro Asn Leu Arg Pro Val Asn Glu Lys
                245                 250                 255

Leu Glu Ala Tyr Ile Leu Asp Pro Arg Gly Ser Arg Met Ile Glu Trp
            260                 265                 270

Arg His Leu Lys Pro Phe Cys Cys Gly Ile Thr Asn Met Ser Phe Pro
        275                 280                 285

Leu Ser Asp Gln Pro Val Leu Gly Glu Trp Phe Ile Phe Val Glu Met
    290                 295                 300

Gln Gly His Ala Tyr Asn Lys Ser Phe Glu Val Gln Lys Tyr Val Leu
305                 310                 315                 320

Pro Lys Phe Glu Leu Leu Ile Asp Pro Pro Arg Tyr Ile Gln Asp Leu
                325                 330                 335

Asp Ala Cys Glu Thr Gly Thr Val Arg Ala Arg Tyr Thr Phe Gly Lys
            340                 345                 350

Pro Val Ala Gly Ala Leu Thr Ile Asn Met Thr Val Asn Gly Val Gly
        355                 360                 365

Tyr Tyr Ser His Glu Val Gly Arg Pro Val Leu Arg Thr Thr Lys Ile
    370                 375                 380

Leu Gly Ser Gln Asp Phe Asp Ile Cys Val Arg Asp Met Ile Pro Ala
385                 390                 395                 400

Asp Val Pro Glu His Phe Arg Gly Arg Val Ser Ile Trp Ala Met Val
                405                 410                 415

Thr Ser Val Asp Gly Ser Gln Gln Val Ala Phe Asp Asp Ser Thr Pro
            420                 425                 430

Val Gln Arg Gln Leu Val Asp Ile Arg Tyr Ser Lys Asp Thr Arg Lys
        435                 440                 445

Gln Phe Lys Pro Gly Leu Ala Tyr Val Gly Lys Val Glu Leu Ser Tyr
    450                 455                 460

Pro Asp Gly Ser Pro Ala Glu Gly Val Thr Val Gln Ile Lys Ala Glu
465                 470                 475                 480

Leu Thr Pro Lys Asp Asn Ile Tyr Thr Ser Glu Val Val Ser Gln Arg
                485                 490                 495

Gly Leu Val Gly Phe Glu Ile Pro Ser Ile Pro Thr Ser Ala Gln His
            500                 505                 510

Val Trp Leu Glu Thr Lys Val Met Ala Leu Asn Gly Lys Pro Val Gly
        515                 520                 525

Ala Gln Tyr Leu Pro Ser Tyr Leu Ser Leu Gly Ser Trp Tyr Ser Pro
    530                 535                 540

Ser Gln Cys Tyr Leu Gln Leu Gln Pro Pro Ser His Pro Leu Gln Val
545                 550                 555                 560

Gly Glu Glu Ala Tyr Phe Ser Val Lys Ser Thr Cys Pro Cys Asn Phe
                565                 570                 575

Thr Leu Tyr Tyr Glu Val Ala Ala Arg Gly Asn Ile Val Leu Ser Gly
            580                 585                 590

Gln Gln Pro Ala His Thr Thr Gln Gln Arg Ser Lys Arg Ala Ala Pro
        595                 600                 605

Ala Leu Glu Lys Pro Ile Arg Leu Thr His Leu Ser Glu Thr Glu Pro
    610                 615                 620

Pro Pro Ala Pro Glu Ala Glu Val Asp Val Cys Val Thr Ser Leu His
625                 630                 635                 640

Leu Ala Val Thr Pro Ser Met Val Pro Leu Gly Arg Leu Leu Val Phe
                645                 650                 655

Tyr Val Arg Glu Asn Gly Glu Gly Val Ala Asp Ser Leu Gln Phe Ala
            660                 665                 670

Val Glu Thr Phe Phe Glu Asn Gln Val Ser Val Thr Tyr Ser Ala Asn
        675                 680                 685

Glu Thr Gln Pro Gly Glu Val Val Asp Leu Arg Ile Arg Ala Ala Arg
    690                 695                 700

Gly Ser Cys Val Cys Val Ala Ala Val Asp Lys Ser Val Tyr Leu Leu
705                 710                 715                 720

Arg Ser Gly Phe Arg Leu Thr Pro Ala Gln Val Phe Gln Glu Leu Glu
                725                 730                 735

Asp Tyr Asp Val Ser Asp Ser Phe Gly Val Ser Arg Glu Asp Gly Pro
            740                 745                 750

Phe Trp Trp Ala Gly Leu Thr Ala Gln Arg Arg Arg Arg Ser Ser Val
        755                 760                 765

Phe Pro Trp Pro Trp Gly Ile Thr Lys Asp Ser Gly Phe Ala Phe Thr
    770                 775                 780

Glu Thr Gly Leu Val Val Met Thr Asp Arg Val Ser Leu Asn His Arg
785                 790                 795                 800

Gln Asp Gly Gly Leu Tyr Thr Asp Glu Ala Val Pro Ala Phe Gln Pro
                805                 810                 815

His Thr Gly Ser Leu Val Ala Val Ala Pro Ser Arg His Pro Pro Arg
            820                 825                 830

Thr Glu Lys Arg Lys Arg Thr Phe Phe Pro Glu Thr Trp Ile Trp His
        835                 840                 845

Cys Leu Asn Ile Ser Asp Pro Ser Gly Glu Gly Thr Leu Ser Val Lys
    850                 855                 860

Val Pro Asp Ser Ile Thr Ser Trp Val Gly Glu Ala Val Ala Leu Ser
865                 870                 875                 880

Thr Ser Gln Gly Leu Gly Ile Ala Glu Pro Ser Leu Leu Lys Thr Phe
                885                 890                 895

Lys Pro Phe Phe Val Asp Phe Met Leu Pro Ala Leu Ile Ile Arg Gly
            900                 905                 910

Glu Gln Val Lys Ile Pro Leu Ser Val Tyr Asn Tyr Met Gly Thr Cys
        915                 920                 925

Ala Glu Val Tyr Met Lys Leu Ser Val Pro Lys Gly Ile Gln Phe Val
    930                 935                 940

Gly His Pro Gly Lys Arg His Val Thr Lys Lys Met Cys Val Ala Pro
945                 950                 955                 960

Gly Glu Ala Glu Pro Ile Trp Val Val Leu Ser Phe Ser Asp Leu Gly
                965                 970                 975

Leu Asn Asn Ile Thr Ala Lys Ala Leu Ala Tyr Gly Asp Thr Asn Cys
            980                 985                 990

Cys Arg Asp Gly Arg Ser Ser Lys His Pro Glu Glu Asn His Ala Asp
        995                 1000                1005

Arg Arg Val Pro Ile Gly Val Asp His Val Arg Arg Ser Val Met Val
    1010                1015                1020

Glu Ala Glu Gly Val Pro Arg Ala Tyr Thr Tyr Ser Ala Phe Phe Cys
1025                1030                1035                1040

Pro Ser Glu Arg Val His Ile Ser Thr Pro Asn Lys Tyr Glu Phe Gln
                1045                1050                1055

Tyr Val Gln Arg Pro Leu Arg Leu Thr Arg Phe Asp Val Ala Val Arg
            1060                1065                1070

Ala His Asn Asp Ala Arg Val Ala Leu Ser Ser Gly Pro Gln Asp Thr
        1075                1080                1085

Ala Gly Met Ile Glu Ile Val Leu Gly Gly His Gln Asn Thr Arg Ser
1090                1095                1100

Trp Ile Ser Thr Ser Lys Met Gly Glu Pro Val Ala Ser Ala His Thr
1105                1110                1115                1120

Ala Lys Ile Leu Ser Trp Asp Glu Phe Arg Thr Phe Trp Ile Ser Trp
                1125                1130                1135

Arg Gly Gly Leu Ile Gln Val Gly His Gly Pro Glu Pro Ser Asn Glu
            1140                1145                1150

Ser Val Ile Val Ala Trp Thr Leu Pro Arg Pro Pro Glu Val Gln Phe
        1155                1160                1165

Ile Gly Phe Ser Thr Gly Trp Gly Ser Met Gly Glu Phe Arg Ile Trp
    1170                1175                1180

Arg Lys Met Glu Val Asp Glu Ser Tyr Ser Glu Ala Phe Thr Leu Gly
1185                1190                1195                1200

Val Pro His Gly Ala Ile Pro Gly Ser Glu Arg Ala Thr Ala Ser Ile
                1205                1210                1215

Ile Gly Asp Val Met Gly Pro Thr Leu Asn His Leu Asn Asn Leu Leu
            1220                1225                1230

Arg Leu Pro Phe Gly Cys Gly Glu Gln Asn Met Ile His Phe Ala Pro
        1235                1240                1245

Asn Val Phe Val Leu Lys Tyr Leu Gln Lys Thr Gln Gln Leu Ser Pro
    1250                1255                1260

Glu Val Glu Arg Glu Thr Thr Asp Tyr Leu Val Gln Gly Tyr Gln Arg
1265                1270                1275                1280

Gln Leu Thr Tyr Lys Arg Gln Asp Gly Ser Tyr Ser Ala Phe Gly Glu
                1285                1290                1295

Arg Asp Ala Ser Gly Ser Met Trp Leu Thr Ala Phe Val Leu Lys Ser
            1300                1305                1310

Phe Ala Gln Ala Arg Ser Phe Ile Phe Val Asp Pro Arg Glu Leu Ala
        1315                1320                1325

Ala Ala Lys Ser Trp Ile Ile Gln Gln Gln Gln Ala Asp Gly Ser Phe
    1330                1335                1340

Leu Ala Val Gly Arg Val Leu Asn Lys Asp Ile Gln Gly Gly Ile His
1345                1350                1355                1360

Gly Ile Val Pro Leu Thr Ala Tyr Val Val Val Ala Leu Leu Glu Thr
                1365                1370                1375

Gly Thr Ala Ser Glu Glu Glu Arg Gly Ser Thr Asp Lys Ala Arg His
            1380                1385                1390

Phe Leu Glu Ser Ala Ala Pro Leu Ala Met Asp Pro Tyr Ser Cys Ala
        1395                1400                1405

Leu Thr Thr Tyr Ala Leu Thr Leu Leu Arg Ser Pro Ala Ala Pro Glu
    1410                1415                1420

Ala Leu Arg Lys Leu Arg Ser Leu Ala Ile Met Arg Asp Gly Val Thr
1425                1430                1435                1440

His Trp Ser Leu Ser Asn Ser Trp Asp Val Asp Lys Gly Thr Phe Leu
                1445                1450                1455

Ser Phe Ser Asp Arg Val Ser Gln Ser Val Val Ser Ala Glu Val Glu
            1460                1465                1470

Met Thr Ala Tyr Ala Leu Leu Thr Tyr Thr Leu Leu Gly Asp Val Ala
        1475                1480                1485

Ala Ala Leu Pro Val Val Lys Trp Leu Ser Gln Gln Arg Asn Ala Leu
    1490                1495                1500

Gly Gly Phe Ser Ser Thr Gln Asp Thr Cys Val Ala Leu Gln Ala Leu
1505                1510                1515                1520

Ala Glu Tyr Ala Ile Leu Ser Tyr Ala Gly Gly Ile Asn Leu Thr Val
                1525                1530                1535

Ser Leu Ala Ser Thr Asn Leu Asp Tyr Gln Glu Thr Phe Glu Leu His
            1540                1545                1550

Arg Thr Asn Gln Lys Val Leu Gln Thr Ala Ala Ile Pro Ser Leu Pro
        1555                1560                1565

Thr Gly Leu Phe Val Ser Ala Lys Gly Asp Gly Cys Cys Leu Met Gln
    1570                1575                1580

Ile Asp Val Thr Tyr Asn Val Pro Asp Pro Val Ala Lys Pro Ala Phe
1585                1590                1595                1600

Gln Leu Leu Val Ser Leu Gln Glu Pro Glu Ala Gln Gly Arg Pro Pro
                1605                1610                1615

Pro Met Pro Ala Ser Ala Ala Glu Gly Ser Arg Gly Asp Trp Pro Pro
            1620                1625                1630

Ala Asp Asp Asp Asp Pro Ala Ala Asp Gln His His Gln Glu Tyr Lys
        1635                1640                1645

Val Met Leu Glu Val Cys Thr Arg Trp Leu His Ala Gly Ser Ser Asn
    1650                1655                1660

Met Ala Val Leu Glu Val Pro Leu Leu Ser Gly Phe Arg Ala Asp Ile
1665                1670                1675                1680

Glu Ser Leu Glu Gln Leu Leu Leu Asp Lys His Met Gly Met Lys Arg
                1685                1690                1695

Tyr Glu Val Ala Gly Arg Arg Val Leu Phe Tyr Phe Asp Glu Ile Pro
            1700                1705                1710

Ser Arg Cys Leu Thr Cys Val Arg Phe Arg Ala Leu Arg Glu Cys Val
        1715                1720                1725

Val Gly Arg Thr Ser Ala Leu Pro Val Ser Val Tyr Asp Tyr Tyr Glu
    1730                1735                1740

Pro Ala Phe Glu Ala Thr Arg Phe Tyr Asn Val Ser Thr His Ser Pro
1745                1750                1755                1760

Leu Ala Arg Glu Leu Cys Ala Gly Pro Ala Cys Asn Glu Val Glu Arg
                1765                1770                1775

Ala Pro Ala Arg Gly Pro Gly Trp Phe Pro Gly Glu Ser Gly Pro Ala
            1780                1785                1790

Val Ala Pro Glu Glu Gly Ala Ala Ile Ala Arg Cys Gly Cys Asp His
        1795                1800                1805

Asp Cys Gly Ala Gln Gly Asn Pro Val Cys Gly Ser Asp Gly Val Val
    1810                1815                1820

Tyr Ala Ser Ala Cys Arg Leu Arg Glu Ala Ala Cys Arg Gln Ala Ala
1825                1830                1835                1840

Pro Leu Glu Pro Ala Pro Pro Ser Cys Cys Ala Leu Glu Gln Arg Leu
                1845                1850                1855

Pro Ala Ser Ser Ser Ser Thr Tyr Gly Asp Asp Leu Ala Ser Val Ala
            1860                1865                1870

Pro Gly Pro Leu Gln Gln Asp Val Lys Leu Asn Gly Ala Gly Leu Glu
        1875                1880                1885

Val Glu Asp Ser Asp Pro Glu Pro Glu Gly Glu Ala Glu Asp Arg Val
    1890                1895                1900

Thr Ala Gly Pro Arg Pro Pro Val Ser Ser Gly Asn Leu Glu Ser Ser
1905                1910                1915                1920

Thr Gln Ser Ala Ser Pro Phe His Arg Trp Gly Gln Thr Pro Ala Pro
                1925                1930                1935

Gln Arg His Ser Gly Arg Val Val Gly Ala His Arg Pro Gly Leu Leu
            1940                1945                1950

Ser Pro Val Phe Val Tyr Ser Pro Ala Phe Gln Ser Gly Gly Glu Glu
        1955                1960                1965

Gly Leu Trp Met Ser Asn Thr Cys Thr Leu Arg
    1970                1975


<210> 31
<211> 2119
<212> DNA
<213> Homo sapiens

<400> 31
tgtggtttcc aaacgtcggc agaggctgga gacggctctc tagtgctggg tgtggagtga 60
ggcaccaccc tcgccctgaa gcctggggca ctcagtcacc atggctcatg ccccagaacc 120
agacccggcc gccagcgacc tcggggatga gaggcccaag tgggacaaca aggcccagta 180
cctcctgagc tgcatcgggt ttgccgtggg gctggggaac atttggcggt tcccatacct 240
gtgccagacc tatggaggag gtgccttcct catcccctac gtcatcgcgc tggtcttcga 300
ggggatcccc attttccacg tcgagctcgc catcggccag cggctgcgga agggcagcgt 360
cggcgtgtgg acggccatct ccccgtacct cagtggagta ggtctgggct gtgtcacgct 420
gtccttcctg atcagcctgt actacaacac catcgtggcg tgggtgctgt ggtacctcct 480
caactccttc cagcacccgc tgccctggag ctcctgccca ccggacctca acagaacagg 540
ttttgtggag gagtgccagg gcagcagcgc cgtgagctac ttctggtacc ggcagacact 600
gaacatcaca gccgacatca atgacagtgg ctccatccag tggtggctgc tcatctgctt 660
ggcagcctcc tgggcagtcg tgtacatgtg tgtcatcagg ggcattgaga ctacagggaa 720
ggtgatttac ttcacagctt tgttccctta cctggtcctg accatctttc tcatcagagg 780
gctgaccctg ccaggggcaa caaaaggact catctacttg ttcactccca acatgcacat 840
tctccagaac ccccgggtgt ggctggacgc agccacccag atattcttct ctctgtccct 900
ggccttcgga ggacacatcg cttttgcaag ttacaactcg cccaggagga atgactgcca 960
gaaggatgcg gtggtcatcg ccctggtcaa caggatgacc tccctgtacg cgtccatcgc 1020
tgtcttctct gtcctggggt tcaaagcaac taatgaccag gagcactgcc tggacaggaa 1080
catcctcagc ctcatcaacg actttgactt cccagagcag agcatctcca gggacgacta 1140
cccagccgtc ctcatgcacc tgaacgccac ctggcccaag agggtggccc agctccccct 1200
gaaggcctgc ctcctggaag actttctgga taagagtgcc tcgggcccgg gcctggcctt 1260
cgtcgtcttc acggagaccg acctccacat gccgggggct cctgtgtggg ccatgctctt 1320
cttcgggatg ctgttcacct tggggctatc gaccatgttc gggaccgtgg aggcggtcat 1380
cacacccctg ctggacgtgg gggtcctgcc tagatgggtc cccaaggagg ccctgactgg 1440
tccagggctg gtctgcctgg tctgcttcct ctccgccacc tgcttcacgc tgcagtctgg 1500
gaactactgg ctggagattt tcgacaattt tgccgcttcc ctgaacctgc tcatgttggc 1560
ctttctcgag gttgtgggtg tcgtttatgt ttatggaatg aaacggttct gcgatgacat 1620
tgcgtggatg accgggaggc ggcccagccc ctactggcgg ctgacctgga gggtggtcag 1680
tcccctgctg ctgaccatct ttgtggctta catcatcctc ctgttctgga agccactgag 1740
atacaaggcc tggaacccca aatacgagct gttcccctcg cgtcaggaga agctctaccc 1800
gggctgggcg cgcgccgcct gtgtgctgct gtccttgctg cccgtgctgt gggtcccggt 1860
ggccgcgctt gctcagctgc tcacccggcg gaggcggacg tggagggaca gggacgcgcg 1920

cccagacacg gacatgcgcc cggacacgga cacgcgccca gacacggaca tgcgcccgga 1980
cacggacatg cgctgaagcc ggccggagcg gggcctgcat gggcgggtct gtgggggggc 2040
ttggcctgat ggtgggcggg gccccgccca cagggccgac cccaatacac cagcgactca 2100
accttaaaaa aaaaaaaaa 2119

<210> 32
<211> 631
<212> PRT
<213> Homo sapiens

<400> 32
Met Ala His Ala Pro Glu Pro Asp Pro Ala Ala Ser Asp Leu Gly Asp
1               5                   10                  15

Glu Arg Pro Lys Trp Asp Asn Lys Ala Gln Tyr Leu Leu Ser Cys Ile
            20                  25                  30

Gly Phe Ala Val Gly Leu Gly Asn Ile Trp Arg Phe Pro Tyr Leu Cys
        35                  40                  45

Gln Thr Tyr Gly Gly Gly Ala Phe Leu Ile Pro Tyr Val Ile Ala Leu
    50                  55                  60

Val Phe Glu Gly Ile Pro Ile Phe His Val Glu Leu Ala Ile Gly Gln
65                  70                  75                  80

Arg Leu Arg Lys Gly Ser Val Gly Val Trp Thr Ala Ile Ser Pro Tyr
                85                  90                  95

Leu Ser Gly Val Gly Leu Gly Cys Val Thr Leu Ser Phe Leu Ile Ser
            100                 105                 110

Leu Tyr Tyr Asn Thr Ile Val Ala Trp Val Leu Trp Tyr Leu Leu Asn
        115                 120                 125

Ser Phe Gln His Pro Leu Pro Trp Ser Ser Cys Pro Pro Asp Leu Asn
    130                 135                 140

Arg Thr Gly Phe Val Glu Glu Cys Gln Gly Ser Ser Ala Val Ser Tyr
145                 150                 155                 160

Phe Trp Tyr Arg Gln Thr Leu Asn Ile Thr Ala Asp Ile Asn Asp Ser
                165                 170                 175

Gly Ser Ile Gln Trp Trp Leu Leu Ile Cys Leu Ala Ala Ser Trp Ala
            180                 185                 190

Val Val Tyr Met Cys Val Ile Arg Gly Ile Glu Thr Thr Gly Lys Val
        195                 200                 205

Ile Tyr Phe Thr Ala Leu Phe Pro Tyr Leu Val Leu Thr Ile Phe Leu
    210                 215                 220

Ile Arg Gly Leu Thr Leu Pro Gly Ala Thr Lys Gly Leu Ile Tyr Leu
225                 230                 235                 240

Phe Thr Pro Asn Met His Ile Leu Gln Asn Pro Arg Val Trp Leu Asp
                245                 250                 255

Ala Ala Thr Gln Ile Phe Phe Ser Leu Ser Leu Ala Phe Gly Gly His
            260                 265                 270

Ile Ala Phe Ala Ser Tyr Asn Ser Pro Arg Arg Asn Asp Cys Gln Lys
        275                 280                 285

Asp Ala Val Val Ile Ala Leu Val Asn Arg Met Thr Ser Leu Tyr Ala
    290                 295                 300

Ser Ile Ala Val Phe Ser Val Leu Gly Phe Lys Ala Thr Asn Asp Gln
305                 310                 315                 320

Glu His Cys Leu Asp Arg Asn Ile Leu Ser Leu Ile Asn Asp Phe Asp
                325                 330                 335

Phe Pro Glu Gln Ser Ile Ser Arg Asp Asp Tyr Pro Ala Val Leu Met
            340                 345                 350

His Leu Asn Ala Thr Trp Pro Lys Arg Val Ala Gln Leu Pro Leu Lys
        355                 360                 365

Ala Cys Leu Leu Glu Asp Phe Leu Asp Lys Ser Ala Ser Gly Pro Gly
    370                 375                 380

Leu Ala Phe Val Val Phe Thr Glu Thr Asp Leu His Met Pro Gly Ala
385                 390                 395                 400

Pro Val Trp Ala Met Leu Phe Phe Gly Met Leu Phe Thr Leu Gly Leu
                405                 410                 415

Ser Thr Met Phe Gly Thr Val Glu Ala Val Ile Thr Pro Leu Leu Asp
            420                 425                 430

Val Gly Val Leu Pro Arg Trp Val Pro Lys Glu Ala Leu Thr Gly Pro
        435                 440                 445

Gly Leu Val Cys Leu Val Cys Phe Leu Ser Ala Thr Cys Phe Thr Leu
    450                 455                 460

Gln Ser Gly Asn Tyr Trp Leu Glu Ile Phe Asp Asn Phe Ala Ala Ser
465                 470                 475                 480

Leu Asn Leu Leu Met Leu Ala Phe Leu Glu Val Val Gly Val Val Tyr
                485                 490                 495

Val Tyr Gly Met Lys Arg Phe Cys Asp Asp Ile Ala Trp Met Thr Gly
            500                 505                 510

Arg Arg Pro Ser Pro Tyr Trp Arg Leu Thr Trp Arg Val Val Ser Pro
        515                 520                 525

Leu Leu Leu Thr Ile Phe Val Ala Tyr Ile Ile Leu Leu Phe Trp Lys
    530                 535                 540

Pro Leu Arg Tyr Lys Ala Trp Asn Pro Lys Tyr Glu Leu Phe Pro Ser
545                 550                 555                 560

Arg Gln Glu Lys Leu Tyr Pro Gly Trp Ala Arg Ala Ala Cys Val Leu
                565                 570                 575

Leu Ser Leu Leu Pro Val Leu Trp Val Pro Val Ala Ala Leu Ala Gln
            580                 585                 590

Leu Leu Thr Arg Arg Arg Arg Thr Trp Arg Asp Arg Asp Ala Arg Pro
        595                 600                 605

Asp Thr Asp Met Arg Pro Asp Thr Asp Thr Arg Pro Asp Thr Asp Met
    610                 615                 620

Arg Pro Asp Thr Asp Met Arg
625                 630

<210> 33
<211> 2039
<212> DNA
<213> Homo sapiens

<400> 33
tgtggtttcc aaacgtcggc agaggctgga gacggctctc tagtgctggg tgtggagtga 60
ggcaccaccc tcgccctgaa gcctggggca ctcagtcacc atggctcatg ccccagaacc 120
agacccggcc gccagcgacc tcggggatga gaggcccaag tgggacaaca aggcccagta 180
cctcctgagc tgcatcggct ttgccgtggg gctggggaac atttggcggt tcccatacct 240
gtgccagacc tatggaggag gtgccttcct catcccctac gtcatcgcgc tggtcttcga 300
ggggatcccc attttccacg tcgagctcgc catcggccag cggctgcgga agggcagcgt 360
cggcgtgtgg acggccatct ccccgtacct cagtggagta ggtctgggct gtgtcacgct 420
gtccttcctg atcagcctgt actacaacac catcgtggcg tgggtgctgt ggtacctcct 480
caactccttc cagcacccgc tgccctggag ctcctgccca ccggacctca acagaacagg 540
ttttgtggag gagtgccagg gcagcagcgc cgtgagctac ttctggtacc ggcagacact 600
gaacatcaca gccgacatca atgacagtgg ctccatccag tggtggctgc tcatctgctt 660
ggcagcctcc tgggcagtcg tgtacatgtg tgtcatcagg ggcattgaga ctacagggaa 720
ggtgatttac ttcacagctt tgttccctta cctggtcctg accatctttc tcatcagagg 780
gctgaccctg ccaggggcaa caaaaggact catctacttg ttcactccca acatgcacat 840
tctccagaac ccccgggtgt ggctggacgc agccacccag atattcttct ctctgtccct 900
ggccttcgga ggacacatcg cttttgcaag ttacaactcg cccaggagga atgactgcca 960
gaaggatgcg gtggtcatcg ccctggtcaa caggatgacc tccctgtacg cgtccatcgc 1020
tgtcttctct gtcctggggt tcaaagcaac taatgaccag gagcactgcc tggacaggaa 1080
catcctcagc ctcatcaacg actttgactt cccagagcag agcatctcca gggacgacta 1140
cccagccgtc ctcatgcacc tgaacgccac ctggcccaag agggtggccc agctccccct 1200
gaaggcctgc ctcctggaag actttctgga taagagtgcc tcgggcccgg gcctggcctt 1260
cgtcgtcttc acggagaccg acctccacat gccgggggct cctgtgtggg ccatgctctt 1320
cttcgggatg ctgttcacct tggggctatc gaccatgttc gggaccgtgg aggcggtcat 1380
cacacccctg ctggacgtgg gggtcctgcc tagatgggtc cccaaggagg ccctgactgg 1440
tccagggctg gtctgcctgg tctgcttcct ctccgccacc tgcttcacgc tgcagtctgg 1500
gaactactgg ctggagattt tcgacaattt tgccgcttcc ctgaacctgc tcatgttggc 1560
ctttctcgag gttgtgggtg tcgtttatgt ttatggaatg aaacggttct gcgatgacat 1620
tgcgtggatg accgggaggc ggcccagccc ctactggcgg ctgacctgga gggtggtcag 1680
tcccctgctg ctgaccatct ttgtggctta catcatcctc ctgttctgga agccactgag 1740
atacaaggcc tggaaccccc aggagctgtt cccctcgcgt caggagaagc tctacccggg 1800
ctggcgcgcc gccgcctgtg tgctgctgtc cttgctgccc gtgctgtggg tcccggtggc 1860
cgcgcttgct cagctgctca cccggcggag gcggacgtgg agacaggcgc atgctgaggc 1920
cgggctggtg ttccaggact tcgagaagca gaggcctggc gtggggatac agtacctgat 1980
tccaatgctt tgcaacttgc tccagacact cttccggtag aaaaagagcc tgttctttt 2039

<210> 34
<211> 639
<212> PRT
<213> Homo sapiens

<400> 34
Met Ala His Ala Pro Glu Pro Asp Pro Ala Ala Ser Asp Leu Gly Asp
1               5                   10                  15

Glu Arg Pro Lys Trp Asp Asn Lys Ala Gln Tyr Leu Leu Ser Cys Ile
            20                  25                  30

Gly Phe Ala Val Gly Leu Gly Asn Ile Trp Arg Phe Pro Tyr Leu Cys
        35                  40                  45

Gln Thr Tyr Gly Gly Gly Ala Phe Leu Ile Pro Tyr Val Ile Ala Leu
    50                  55                  60

Val Phe Glu Gly Ile Pro Ile Phe His Val Glu Leu Ala Ile Gly Gln
65                  70                  75                  80

Arg Leu Arg Lys Gly Ser Val Gly Val Trp Thr Ala Ile Ser Pro Tyr
                85                  90                  95

Leu Ser Gly Val Gly Leu Gly Cys Val Thr Leu Ser Phe Leu Ile Ser
            100                 105                 110

Leu Tyr Tyr Asn Thr Ile Val Ala Trp Val Leu Trp Tyr Leu Leu Asn
        115                 120                 125

Ser Phe Gln His Pro Leu Pro Trp Ser Ser Cys Pro Pro Asp Leu Asn
    130                 135                 140

Arg Thr Gly Phe Val Glu Glu Cys Gln Gly Ser Ser Ala Val Ser Tyr
145                 150                 155                 160

Phe Trp Tyr Arg Gln Thr Leu Asn Ile Thr Ala Asp Ile Asn Asp Ser
                165                 170                 175

Gly Ser Ile Gln Trp Trp Leu Leu Ile Cys Leu Ala Ala Ser Trp Ala
            180                 185                 190

Val Val Tyr Met Cys Val Ile Arg Gly Ile Glu Thr Thr Gly Lys Val
        195                 200                 205

Ile Tyr Phe Thr Ala Leu Phe Pro Tyr Leu Val Leu Thr Ile Phe Leu
    210                 215                 220

Ile Arg Gly Leu Thr Leu Pro Gly Ala Thr Lys Gly Leu Ile Tyr Leu
225                 230                 235                 240

Phe Thr Pro Asn Met His Ile Leu Gln Asn Pro Arg Val Trp Leu Asp
                245                 250                 255

Ala Ala Thr Gln Ile Phe Phe Ser Leu Ser Leu Ala Phe Gly Gly His
            260                 265                 270

Ile Ala Phe Ala Ser Tyr Asn Ser Pro Arg Arg Asn Asp Cys Gln Lys
        275                 280                 285

Asp Ala Val Val Ile Ala Leu Val Asn Arg Met Thr Ser Leu Tyr Ala
    290                 295                 300

Ser Ile Ala Val Phe Ser Val Leu Gly Phe Lys Ala Thr Asn Asp Gln
305                 310                 315                 320

Glu His Cys Leu Asp Arg Asn Ile Leu Ser Leu Ile Asn Asp Phe Asp
                325                 330                 335

Phe Pro Glu Gln Ser Ile Ser Arg Asp Asp Tyr Pro Ala Val Leu Met
            340                 345                 350

His Leu Asn Ala Thr Trp Pro Lys Arg Val Ala Gln Leu Pro Leu Lys
        355                 360                 365

Ala Cys Leu Leu Glu Asp Phe Leu Asp Lys Ser Ala Ser Gly Pro Gly
    370                 375                 380

Leu Ala Phe Val Val Phe Thr Glu Thr Asp Leu His Met Pro Gly Ala
385                 390                 395                 400

Pro Val Trp Ala Met Leu Phe Phe Gly Met Leu Phe Thr Leu Gly Leu
                405                 410                 415

Ser Thr Met Phe Gly Thr Val Glu Ala Val Ile Thr Pro Leu Leu Asp
            420                 425                 430

Val Gly Val Leu Pro Arg Trp Val Pro Lys Glu Ala Leu Thr Gly Pro
        435                 440                 445

Gly Leu Val Cys Leu Val Cys Phe Leu Ser Ala Thr Cys Phe Thr Leu
    450                 455                 460

Gln Ser Gly Asn Tyr Trp Leu Glu Ile Phe Asp Asn Phe Ala Ala Ser
465                 470                 475                 480

Leu Asn Leu Leu Met Leu Ala Phe Leu Glu Val Val Gly Val Val Tyr
                485                 490                 495

Val Tyr Gly Met Lys Arg Phe Cys Asp Asp Ile Ala Trp Met Thr Gly
            500                 505                 510

Arg Arg Pro Ser Pro Tyr Trp Arg Leu Thr Trp Arg Val Val Ser Pro
        515                 520                 525

Leu Leu Leu Thr Ile Phe Val Ala Tyr Ile Ile Leu Leu Phe Trp Lys
    530                 535                 540

Pro Leu Arg Tyr Lys Ala Trp Asn Pro Gln Glu Leu Phe Pro Ser Arg
545                 550                 555                 560

Gln Glu Lys Leu Tyr Pro Gly Trp Ala Arg Ala Ala Cys Val Leu Leu
                565                 570                 575

Ser Leu Leu Pro Val Leu Trp Val Pro Val Ala Ala Leu Ala Gln Leu
            580                 585                 590

Leu Thr Arg Arg Arg Arg Thr Trp Arg Gln Ala His Ala Glu Ala Gly
        595                 600                 605

Leu Val Phe Gln Asp Phe Glu Lys Gln Arg Pro Gly Val Gly Ile Gln
    610                 615                 620

Tyr Leu Ile Pro Met Leu Cys Asn Leu Leu Gln Thr Leu Phe Arg
625                 630                 635

<210> 35
<211> 1748
<212> DNA
<213> Homo sapiens

<400> 35
gttcacccca agactaagtt ctttcccaag ttagagaaga agagagaaag caaaaagaag 60
agaggaaagt tctcccttcc cctcctccgt gcctgtcatg tcctctaagc cagagccgaa 120
ggacgtccac caactgaacg ggactggccc ttctgcctct ccctgctctt cagatggccc 180
agggagagag cccttggctg ggacctcaga gttcctgggg cctgatgggg ctggggtaga 240
ggtggtgatt gagtctcggg ccaacgccaa gggggttcgg gaggaggacg ccctgctgga 300
gaacgggagc cagagcaacg aaagtgacga cgtcagcaca gaccgtggcc ctgcgccacc 360
ttccccgctc aaggagacct ccttttccat cgggctgcaa gtactgtttc cattcctcct 420
ggcaggcttt gggaccgtgg ctgctggcat ggtgttggac atcgtgcagc actgggaagt 480
cttccagaag gtgacagagg tcttcatcct agtgcctgcg ctgctggggc tcaaagggaa 540
cctggaaatg accctggcat caaggctttc cactgcagcg agtatcaaca ttggacacat 600
ggacacaccc aaggagctct ggcggatgat cactgggaac atggccctca tccaggtgca 660
ggccacggtg gtgggcttcc tggcgtccat cgcagccgtc gtctttggct ggatccctga 720
tggccacttc agtattccgc acgccttcct gctctgtgct agcagcgtgg ccacagcctt 780
cattgcctcc ctggtactgg gtatgatcat gattggagtc atcattggct ctcgcaagat 840
tgggatcaac ccagacaatg tggccacacc cattgctgcc agcctgggcg acctcatcac 900
cttggcgctg ctctcaggca tcagctgggg actcctgacc tctgccctct cagatcactg 960
gcgatacatc tacccactgg tgtgtgcttt ctttgtggcc ctgctgcctg tctgggtggt 1020
gctggcccga cgaagtccag ccacaaggga ggtgttgtac tcgggctggg agcctgttat 1080
cattgccatg gccatcagca gtgtgggagg cctcatcttg gacaagactg tctcagaccc 1140
caactttgct gggatggctg tcttcacgcc tgtgattaat ggtgttgggg gcaatctggt 1200
ggcagtgcag gccagccgca tctccacctt cctgcacatg aatggaatgc ccggagagaa 1260
ctctgagcaa gctcctcgcc gctgtcccag tccttgtacc accttcttca gccctggtgt 1320
gaattctcgc tcagcccggg tcctcttcct cctcgtggtc ccaggacacc tggtgttcct 1380
ctacaccatc agctgtatgc agggcgggca caccaccctc acactcatct tcatcatctt 1440
ctatatgaca gctgcactgc tccaggtgct gattctcctg tacatcgcag actggatggt 1500
gcactggatg tggggccggg gcctggaccc ggacaacttc tccatcccat acttgactgc 1560
tctgggggac ctgcttggca ctgggctcct agcactcagc ttccatgttc tctggctcat 1620
aggggaccga gacacggatg tcggggacta gcttggtcac tcaacatttt ccccatccct 1680
ctgcactttc tatttgaaat tttctttttg ttcccctgtc cctcctccac cccacactcc 1740
cacctctt 1748

<210> 36
<211> 517
<212> PRT
<213> Homo sapiens

<400> 36
Met Ser Ser Lys Pro Glu Pro Lys Asp Val His Gln Leu Asn Gly Thr
1               5                   10                  15

Gly Pro Ser Ala Ser Pro Cys Ser Ser Asp Gly Pro Gly Arg Glu Pro
            20                  25                  30

Leu Ala Gly Thr Ser Glu Phe Leu Gly Pro Asp Gly Ala Gly Val Glu
        35                  40                  45

Val Val Ile Glu Ser Arg Ala Asn Ala Lys Gly Val Arg Glu Glu Asp
    50                  55                  60

Ala Leu Leu Glu Asn Gly Ser Gln Ser Asn Glu Ser Asp Asp Val Ser
65                  70                  75                  80

Thr Asp Arg Gly Pro Ala Pro Pro Ser Pro Leu Lys Glu Thr Ser Phe
                85                  90                  95

Ser Ile Gly Leu Gln Val Leu Phe Pro Phe Leu Leu Ala Gly Phe Gly
            100                 105                 110

Thr Val Ala Ala Gly Met Val Leu Asp Ile Val Gln His Trp Glu Val
        115                 120                 125

Phe Gln Lys Val Thr Glu Val Phe Ile Leu Val Pro Ala Leu Leu Gly
    130                 135                 140

Leu Lys Gly Asn Leu Glu Met Thr Leu Ala Ser Arg Leu Ser Thr Ala
145                 150                 155                 160

Ala Ser Ile Asn Ile Gly His Met Asp Thr Pro Lys Glu Leu Trp Arg
                165                 170                 175

Met Ile Thr Gly Asn Met Ala Leu Ile Gln Val Gln Ala Thr Val Val
            180                 185                 190

Gly Phe Leu Ala Ser Ile Ala Ala Val Val Phe Gly Trp Ile Pro Asp
        195                 200                 205

Gly His Phe Ser Ile Pro His Ala Phe Leu Leu Cys Ala Ser Ser Val
    210                 215                 220

Ala Thr Ala Phe Ile Ala Ser Leu Val Leu Gly Met Ile Met Ile Gly
225                 230                 235                 240

Val Ile Ile Gly Ser Arg Lys Ile Gly Ile Asn Pro Asp Asn Val Ala
                245                 250                 255

Thr Pro Ile Ala Ala Ser Leu Gly Asp Leu Ile Thr Leu Ala Leu Leu
            260                 265                 270

Ser Gly Ile Ser Trp Gly Leu Leu Thr Ser Ala Leu Ser Asp His Trp
        275                 280                 285

Arg Tyr Ile Tyr Pro Leu Val Cys Ala Phe Phe Val Ala Leu Leu Pro
    290                 295                 300

Val Trp Val Val Leu Ala Arg Arg Ser Pro Ala Thr Arg Glu Val Leu
305                 310                 315                 320

Tyr Ser Gly Trp Glu Pro Val Ile Ile Ala Met Ala Ile Ser Ser Val
                325                 330                 335

Gly Gly Leu Ile Leu Asp Lys Thr Val Ser Asp Pro Asn Phe Ala Gly
            340                 345                 350

Met Ala Val Phe Thr Pro Val Ile Asn Gly Val Gly Gly Asn Leu Val
        355                 360                 365

Ala Val Gln Ala Ser Arg Ile Ser Thr Phe Leu His Met Asn Gly Met
    370                 375                 380

Pro Gly Glu Asn Ser Glu Gln Ala Pro Arg Arg Cys Pro Ser Pro Cys
385                 390                 395                 400

Thr Thr Phe Phe Ser Pro Gly Val Asn Ser Arg Ser Ala Arg Val Leu
                405                 410                 415

Phe Leu Leu Val Val Pro Gly His Leu Val Phe Leu Tyr Thr Ile Ser
            420                 425                 430

Cys Met Gln Gly Gly His Thr Thr Leu Thr Leu Ile Phe Ile Ile Phe
        435                 440                 445

Tyr Met Thr Ala Ala Leu Leu Gln Val Leu Ile Leu Leu Tyr Ile Ala
    450                 455                 460

Asp Trp Met Val His Trp Met Trp Gly Arg Gly Leu Asp Pro Asp Asn
465                 470                 475                 480

Phe Ser Ile Pro Tyr Leu Thr Ala Leu Gly Asp Leu Leu Gly Thr Gly
                485                 490                 495

Leu Leu Ala Leu Ser Phe His Val Leu Trp Leu Ile Gly Asp Arg Asp
            500                 505                 510

Thr Asp Val Gly Asp
        515


<210> 37
<211> 5175
<212> DNA
<213> Homo sapiens

<400> 37
aatgttcaaa ggctttctgt aactgaactt tttttttttc ttttttcccc tagctattgc 60
tctgcaatat ttactttacc ctgttaatga acaggacaaa atggttaaaa aagagataag 120
cgtgcgtcaa caaattcagg ctcttctgta caagaatttt cttaaaaaat ggagaataaa 180
aagagagctg gaggaatgga caataacatt gtttctaggg ctatatttgt gcatcttttc 240
ggaacacttc agagctaccc gttttcctga acaacctcct aaagtcctgg gaagcgtgga 300
tcagtttaat gactctggcc tggtagtggc atatacacca gtcagtaaca taacacaaag 360
gataatgaat aagatggcct tggcttcctt tatgaaaggt agaacagtca ttgggacacc 420
agatgaagag accatggata tagaacttcc aaaaaaatac catgaaatgg tgggagttat 480
atttagtgat actttctcat atcgcctgaa gtttaattgg ggatatagaa tcccagttat 540
aaaggagcac tctgaataca caggtcactg ttgggccatg catggtgaaa tttttttgtta 600
cttggcaaag tactggctaa aagggtttgt agcttttcaa gctgcaatta atgctgcaat 660
tatagaagta agtacaacaa atcattctgt aatggaggag ttgacatcag ttattggaat 720
aaatatgaag ataccacctt tcatttctaa gggagaaatt atgaatgaat ggtttcattt 780
tacttgctta gtttctttct cttcttttat atactttgca tcattaaatg ttgcaaggga 840
aagaggaaaa tttaagaaac tgatgacagt gatgggtctc cgagagtcag cattctggct 900
ctcctgggga ttgacataca tttgcttcat cttcattatg tccattttta tggctctggt 960
cataacatca atcccaattg tatttcatac tggcttcatg gtgatattca cactctatag 1020
cttatatggc ctttctttgg tgttggcttt cctcatgagt gttttaataa ggaaacctat 1080
gctcgctggt ttggctggat ttctcttcac tgtattttgg ggatgtctgg gattcactgt 1140
gttatataga caacttcctt tatctttggg atgggtatta agtcttctta gcccttttgc 1200
cttcactgct ggaatacaga ttacacacct ggataattac ttaagtggtg ttatttttcc 1260
tgatccctct ggggattcat acaaaatgat agccactttt ttcattttgg catttgatac 1320
tcttttctat ttgatattca cattatattt tgagcgagtt ttacctggta agggccatgg 1380
ggattctcca ttatttttcc ttaagtcctc attttggtcc aaacatcaaa atactcatca 1440
tgaaatcttt gagaatgaaa taaatcctga gcattcctct gatgattctt ttgaaccggt 1500
gtctccagaa ttccatggaa aagaagccat aaggatcaga aatgttataa aagaatataa 1560
tggaaagact ggaaaagtag aagcattgca aatatttttt gacatatatg aaggacagat 1620
cactgcaata cttgggcata atggagctgg taaatcaaca ctgctaaaca ttcttagtgg 1680
attgtctgtt tctacagaag gttcagccac tatttataat actcaactct ctgaaataac 1740
tgacatggaa gaaattagaa agaatattgg attttgtcca cagttcaatt ttcaatttga 1800
cttcctcact gtgagagaaa acctcagggt atttgctaaa ataaaaggga ttcagccaaa 1860

ggaagtggaa caagaggtat tgctgctaga tgaaccaact gctggattgg atccctttc 1920
aagacaccga gtgtggagcc tcctgaagga gcataaagta gaccgactta tcctcttcag 1980
tacccaattc atggatgagg ctgacatctt ggctgatagg aaagtatttc tgtctaatgg 2040
gaagttgaaa tgtgcaggat catctttgtt tctgaagcga aagtggggta ttggatatca 2100
tttaagttta cacaggaatg aaatgtgtga cacagaaaaa atcacatccc ttattaagca 2160
gcacattcct gatgccaagt taacaacaga aagtgaagaa aaacttgtat atagtttgcc 2220
tttggaaaaa acgaacaaat ttccagatct ttacagtgac cttgataagt gttctgacca 2280
gggcataagg aattatgctg tttcagtgac atctctgaat gaagtattct tgaacctaga 2340
aggaaaatca gcaattgatg aaccaggtat atttgacatt gggaaacaag agaaaataca 2400
tgtgacaaga aatactggag atgagtctga aatggaacag gttctttgtt ctcttcctga 2460
aacaagaaag gctgtcagta gtgcagctct ctggagccga caaatctatg cagtggcaac 2520
acttcgcttc ttaaagttaa ggcgtgaaag gagagctctt ttgtgtttgt tactagtact 2580
tggaattgct tttatcccca tcattctaga gaagataatg tataaagtaa ctcgtgaaac 2640
tcattgttgg gagttttcac ccagtatgta tttcctttct ctggaacaaa tcccgaagac 2700
gcctcttacc agcctgttaa tcgttaataa tacaggttca aatattgaag acctcgtgca 2760
ttcactgaag tgtcaggata tagttttgga aatagatgac tttagaaaca gaaatggctc 2820
agatgatccc tcctacaatg gagccatcat agtgtctggt gaccagaagg attacagatt 2880
ttcagttgca tgtaatacca agaaatcgaa ttgttttccg gttcttatgg gaattgttag 2940
caatgccctt attggaattt ttaacttcac agagcttatt caaatggaga gcaccttcat 3000
ttttcgtgat gacatagtgc tggatcttgg ttttatagat gggtccatat ttttgttgtt 3060
gatcacaaac tgcatttctc cttatattgg cataacagca tcagtgatta ttaaagtaag 3120
agggagagag aggtcccagt tatggatttc aggcctctgg ccttcagcat actggtgtgg 3180
acaggctctg gtggacattc cattatactt cttgattctc ttttcaatac atttaattta 3240
ctacttcata tttctgggat tccagctttc atgggaactc atgtttgttt tggtaagtga 3300
tccattattt gcaggtggta tgcataattg gttgtgcagt ttctcttata ttcctcacat 3360
atgtgctttc attcatcttt cgcaagtgga gaaaaaaaat ggcttttggt cttttggctt 3420
ttttattgta agtatatata catgcgtgca catttatatt aaattttatt tgcttgataa 3480
atcttttttg ccacttgtgt ttacttttaa cttttattgt tcttatgctt taatgcctgt 3540
ctcttgtaaa tctgtcttac tttttgcttt tttatttctc caaaaatatc tccatatagc 3600
tccaatcctt ttccctctgt ttgcttttgt tagtgttatt ttcctttttg tcataaggtg 3660
tctggaaatg aagtatggaa atgaaataat gaataaagac ccagttttca ggatctctcc 3720
acggagtaga gaaactcatc ccaatccgga agagcccgaa gaagaagatg aagatgttca 3780
agctgaaaga gtccaagcag caaatgcact cactgctcca aacttggagg aggaaccagt 3840
cataactgca agctgtttac acaaggaata ttatgagaca aagaaaagtt gcttttcaac 3900
aagaaagaag aaaatagcca tcagaaatgt ttccttttgt gttaaaaaag gtgaagtttt 3960
gggattacta ggacacaatg gagctggtaa aagtacttcc attaaaatga taactgggtg 4020
cacaaagcca actgcaggag tggtagtggt gttacaaggc agcagagcat cagtaaggca 4080
acagcatgac aacagcctca agttcttggg gtactgccct caggagaact cactgtggcc 4140
caagcttaca atgaaagagc acttggagtt gtatgcagct gtgaaaggac tgggcaaaga 4200
agatgctgct ctcagtattt caagattggt ggaagctctt aagctccagg aacaacttaa 4260
ggctcctgtg aaaactctat cagagggaat aaagagaaag ctgtgctttg tgctgagcat 4320
cctggggaac ccatcagtgg tgcttctaga tgagccgttc accgggatgg accccgaggg 4380
gcagcagcaa atgtggcttc aggctaccgt taaaaacaag gagaggggca ccctcttgac 4440
cacccattac atgtcagagg ctgaggctgt gtgtgaccgt atggccatga tggtgtcagg 4500
aacgctaagg aggtgtattg gttccattca acatctgaaa aacaagtttg gtagagatta 4560
tttactagaa ataaaaatga aagaacctac ccaggtggaa gctctccaca cagagatttt 4620
gaagcttttc ccacaggctg cttggcagga aagatattcc tctttaatgg cgtataagtt 4680
acctgtggag gatgtccacc ctctatctcg ggcctttttc aagttagagc gagtgaagca 4740
gaccttcaac ctggaggaat acagcctctc tcaggctacc ttggagcagg tgttcttaga 4800
actctgtaaa gagcaggagc tgggaaatgt tgatgataaa attgatacaa cagttgaatg 4860
gaaacttctc ccacaggaag acccttaaaa tgaagaacct cctaacattc aatttaggt 4920
cctactacat tgttagtttc cataattcta caagaatgtt tccttttact tcagttaaca 4980
aaagaaaaca tttaataaac attcaataat gattacagtt ttcattttta aaaatttagg 5040
atgaaggaaa caaggaaata tagggaaaag tagtagacaa aattaacaaa atcagacatg 5100
ttattcatcc ccaacatggg tctattttgt gcttaaaaat aatttaaaaa tcatacaata 5160
ttaggttggt tatcg 5175

<210> 38
<211> 1595
<212> PRT
<213> Homo sapiens

<400> 38
Met Val Lys Lys Glu Ile Ser Val Arg Gln Gln Ile Gln Ala Leu Leu
1               5                   10                  15

Tyr Lys Asn Phe Leu Lys Lys Trp Arg Ile Lys Arg Glu Leu Glu Glu
            20                  25                  30

Trp Thr Ile Thr Leu Phe Leu Gly Leu Tyr Leu Cys Ile Phe Ser Glu
        35                  40                  45

His Phe Arg Ala Thr Arg Phe Pro Glu Gln Pro Pro Lys Val Leu Gly
    50                  55                  60

Ser Val Asp Gln Phe Asn Asp Ser Gly Leu Val Val Ala Tyr Thr Pro
65                  70                  75                  80

Val Ser Asn Ile Thr Gln Arg Ile Met Asn Lys Met Ala Leu Ala Ser
                85                  90                  95

Phe Met Lys Gly Arg Thr Val Ile Gly Thr Pro Asp Glu Glu Thr Met
            100                 105                 110

Asp Ile Glu Leu Pro Lys Lys Tyr His Glu Met Val Gly Val Ile Phe
        115                 120                 125

Ser Asp Thr Phe Ser Tyr Arg Leu Lys Phe Asn Trp Gly Tyr Arg Ile
    130                 135                 140

Pro Val Ile Lys Glu His Ser Glu Tyr Thr Gly His Cys Trp Ala Met
145                 150                 155                 160

His Gly Glu Ile Phe Cys Tyr Leu Ala Lys Tyr Trp Leu Lys Gly Phe
                165                 170                 175

Val Ala Phe Gln Ala Ala Ile Asn Ala Ala Ile Ile Glu Val Ser Thr
            180                 185                 190

Thr Asn His Ser Val Met Glu Glu Leu Thr Ser Val Ile Gly Ile Asn
        195                 200                 205

Met Lys Ile Pro Pro Phe Ile Ser Lys Gly Glu Ile Met Asn Glu Trp
    210                 215                 220

Phe His Phe Thr Cys Leu Val Ser Phe Ser Ser Phe Ile Tyr Phe Ala
225                 230                 235                 240

Ser Leu Asn Val Ala Arg Glu Arg Gly Lys Phe Lys Lys Leu Met Thr
                245                 250                 255

Val Met Gly Leu Arg Glu Ser Ala Phe Trp Leu Ser Trp Gly Leu Thr
            260                 265                 270

Tyr Ile Cys Phe Ile Phe Ile Met Ser Ile Phe Met Ala Leu Val Ile
        275                 280                 285

Thr Ser Ile Pro Ile Val Phe His Thr Gly Phe Met Val Ile Phe Thr
    290                 295                 300

Leu Tyr Ser Leu Tyr Gly Leu Ser Leu Val Leu Ala Phe Leu Met Ser
305                 310                 315                 320

Val Leu Ile Arg Lys Pro Met Leu Ala Gly Leu Ala Gly Phe Leu Phe
                325                 330                 335

Thr Val Phe Trp Gly Cys Leu Gly Phe Thr Val Leu Tyr Arg Gln Leu
            340                 345                 350

Pro Leu Ser Leu Gly Trp Val Leu Ser Leu Leu Ser Pro Phe Ala Phe
        355                 360                 365

Thr Ala Gly Ile Gln Ile Thr His Leu Asp Asn Tyr Leu Ser Gly Val
    370                 375                 380

Ile Phe Pro Asp Pro Ser Gly Asp Ser Tyr Lys Met Ile Ala Thr Phe
385                 390                 395                 400

Phe Ile Leu Ala Phe Asp Thr Leu Phe Tyr Leu Ile Phe Thr Leu Tyr
                405                 410                 415

Phe Glu Arg Val Leu Pro Gly Lys Gly His Gly Asp Ser Pro Leu Phe
            420                 425                 430

Phe Leu Lys Ser Ser Phe Trp Ser Lys His Gln Asn Thr His His Glu
        435                 440                 445

Ile Phe Glu Asn Glu Ile Asn Pro Glu His Ser Ser Asp Asp Ser Phe
    450                 455                 460

Glu Pro Val Ser Pro Glu Phe His Gly Lys Glu Ala Ile Arg Ile Arg
465                 470                 475                 480

Asn Val Ile Lys Glu Tyr Asn Gly Lys Thr Gly Lys Val Glu Ala Leu
                485                 490                 495

Gln Ile Phe Phe Asp Ile Tyr Glu Gly Gln Ile Thr Ala Ile Leu Gly
            500                 505                 510

His Asn Gly Ala Gly Lys Ser Thr Leu Leu Asn Ile Leu Ser Gly Leu
        515                 520                 525

Ser Val Ser Thr Glu Gly Ser Ala Thr Ile Tyr Asn Thr Gln Leu Ser
    530                 535                 540

Glu Ile Thr Asp Met Glu Glu Ile Arg Lys Asn Ile Gly Phe Cys Pro
545                 550                 555                 560

Gln Phe Asn Phe Gln Phe Asp Phe Leu Thr Val Arg Glu Asn Leu Arg
                565                 570                 575

Val Phe Ala Lys Ile Lys Gly Ile Gln Pro Lys Glu Val Glu Gln Glu
            580                 585                 590

Val Leu Leu Leu Asp Glu Pro Thr Ala Gly Leu Asp Pro Phe Ser Arg
        595                 600                 605

His Arg Val Trp Ser Leu Leu Lys Glu His Lys Val Asp Arg Leu Ile
    610                 615                 620

Leu Phe Ser Thr Gln Phe Met Asp Glu Ala Asp Ile Leu Ala Asp Arg
625                 630                 635                 640

Lys Val Phe Leu Ser Asn Gly Lys Leu Lys Cys Ala Gly Ser Ser Leu
                645                 650                 655

Phe Leu Lys Arg Lys Trp Gly Ile Gly Tyr His Leu Ser Leu His Arg
            660                 665                 670

Asn Glu Met Cys Asp Thr Glu Lys Ile Thr Ser Leu Ile Lys Gln His
        675                 680                 685

Ile Pro Asp Ala Lys Leu Thr Thr Glu Ser Glu Glu Lys Leu Val Tyr
    690                 695                 700

Ser Leu Pro Leu Glu Lys Thr Asn Lys Phe Pro Asp Leu Tyr Ser Asp
705                 710                 715                 720

Leu Asp Lys Cys Ser Asp Gln Gly Ile Arg Asn Tyr Ala Val Ser Val
                725                 730                 735

Thr Ser Leu Asn Glu Val Phe Leu Asn Leu Glu Gly Lys Ser Ala Ile
            740                 745                 750

Asp Glu Pro Gly Ile Phe Asp Ile Gly Lys Gln Glu Lys Ile His Val
        755                 760                 765

Thr Arg Asn Thr Gly Asp Glu Ser Glu Met Glu Gln Val Leu Cys Ser
    770                 775                 780

Leu Pro Glu Thr Arg Lys Ala Val Ser Ser Ala Ala Leu Trp Ser Arg
785                 790                 795                 800

Gln Ile Tyr Ala Val Ala Thr Leu Arg Phe Leu Lys Leu Arg Arg Glu
                805                 810                 815

Arg Arg Ala Leu Leu Cys Leu Leu Leu Val Leu Gly Ile Ala Phe Ile
            820                 825                 830

Pro Ile Ile Leu Glu Lys Ile Met Tyr Lys Val Thr Arg Glu Thr His
        835                 840                 845

Cys Trp Glu Phe Ser Pro Ser Met Tyr Phe Leu Ser Leu Glu Gln Ile
    850                 855                 860

Pro Lys Thr Pro Leu Thr Ser Leu Leu Ile Val Asn Asn Thr Gly Ser
865                 870                 875                 880

Asn Ile Glu Asp Leu Val His Ser Leu Lys Cys Gln Asp Ile Val Leu
                885                 890                 895

Glu Ile Asp Asp Phe Arg Asn Arg Asn Gly Ser Asp Asp Pro Ser Tyr
            900                 905                 910

Asn Gly Ala Ile Ile Val Ser Gly Asp Gln Lys Asp Tyr Arg Phe Ser
        915                 920                 925

Val Ala Cys Asn Thr Lys Lys Ser Asn Cys Phe Pro Val Leu Met Gly
    930                 935                 940

Ile Val Ser Asn Ala Leu Ile Gly Ile Phe Asn Phe Thr Glu Leu Ile
945                 950                 955                 960

Gln Met Glu Ser Thr Phe Ile Phe Arg Asp Asp Ile Val Leu Asp Leu
                965                 970                 975

Gly Phe Ile Asp Gly Ser Ile Phe Leu Leu Leu Ile Thr Asn Cys Ile
            980                 985                 990

Ser Pro Tyr Ile Gly Ile Thr Ala Ser Val Ile Ile Lys Val Arg Gly
        995                 1000                1005

Arg Glu Arg Ser Gln Leu Trp Ile Ser Gly Leu Trp Pro Ser Ala Tyr
    1010                1015                1020

Trp Cys Gly Gln Ala Leu Val Asp Ile Pro Leu Tyr Phe Leu Ile Leu
1025                1030                1035                1040

Phe Ser Ile His Leu Ile Tyr Tyr Phe Ile Phe Leu Gly Phe Gln Leu
                1045                1050                1055

Ser Trp Glu Leu Met Phe Val Leu Val Ser Asp Pro Leu Phe Ala Gly
            1060                1065                1070

Gly Met His Asn Trp Leu Cys Ser Phe Ser Tyr Ile Pro His Ile Cys
        1075                1080                1085

Ala Phe Ile His Leu Ser Gln Val Glu Lys Lys Asn Gly Phe Trp Ser
    1090                1095                1100

Phe Gly Phe Phe Ile Val Ser Ile Tyr Thr Cys Val His Ile Tyr Ile
1105                1110                1115                1120

Lys Phe Tyr Leu Leu Asp Lys Ser Phe Leu Pro Leu Val Phe Thr Phe
                1125                1130                1135

Asn Phe Tyr Cys Ser Tyr Ala Leu Met Pro Val Ser Cys Lys Ser Val
            1140                1145                1150

Leu Leu Phe Ala Phe Leu Phe Leu Gln Lys Tyr Leu His Ile Ala Pro
        1155                1160                1165

Ile Leu Phe Pro Leu Phe Ala Phe Val Ser Val Ile Phe Leu Phe Val
    1170                1175                1180

Ile Arg Cys Leu Glu Met Lys Tyr Gly Asn Glu Ile Met Asn Lys Asp
1185                1190                1195                1200

Pro Val Phe Arg Ile Ser Pro Arg Ser Arg Glu Thr His Pro Asn Pro
                1205                1210                1215

Glu Glu Pro Glu Glu Glu Asp Glu Asp Val Gln Ala Glu Arg Val Gln
            1220                1225                1230

Ala Ala Asn Ala Leu Thr Ala Pro Asn Leu Glu Glu Glu Pro Val Ile
        1235                1240                1245

Thr Ala Ser Cys Leu His Lys Glu Tyr Tyr Glu Thr Lys Lys Ser Cys
    1250                1255                1260

Phe Ser Thr Arg Lys Lys Lys Ile Ala Ile Arg Asn Val Ser Phe Cys
1265                1270                1275                1280

Val Lys Lys Gly Glu Val Leu Gly Leu Leu Gly His Asn Gly Ala Gly
                1285                1290                1295

Lys Ser Thr Ser Ile Lys Met Ile Thr Gly Cys Thr Lys Pro Thr Ala
            1300                1305                1310

Gly Val Val Val Val Leu Gln Gly Ser Arg Ala Ser Val Arg Gln Gln
        1315                1320                1325

His Asp Asn Ser Leu Lys Phe Leu Gly Tyr Cys Pro Gln Glu Asn Ser
    1330                1335                1340

Leu Trp Pro Lys Leu Thr Met Lys Glu His Leu Glu Leu Tyr Ala Ala
1345                1350                1355                1360

Val Lys Gly Leu Gly Lys Glu Asp Ala Ala Leu Ser Ile Ser Arg Leu
                1365                1370                1375

Val Glu Ala Leu Lys Leu Gln Glu Gln Leu Lys Ala Pro Val Lys Thr
            1380                1385                1390

Leu Ser Glu Gly Ile Lys Arg Lys Leu Cys Phe Val Leu Ser Ile Leu
        1395                1400                1405

Gly Asn Pro Ser Val Val Leu Leu Asp Glu Pro Phe Thr Gly Met Asp
    1410                1415                1420

Pro Glu Gly Gln Gln Gln Met Trp Leu Gln Ala Thr Val Lys Asn Lys
1425                1430                1435                1440

Glu Arg Gly Thr Leu Leu Thr Thr His Tyr Met Ser Glu Ala Glu Ala
                1445                1450                1455

Val Cys Asp Arg Met Ala Met Met Val Ser Gly Thr Leu Arg Arg Cys
            1460                1465                1470

Ile Gly Ser Ile Gln His Leu Lys Asn Lys Phe Gly Arg Asp Tyr Leu
        1475                1480                1485

Leu Glu Ile Lys Met Lys Glu Pro Thr Gln Val Glu Ala Leu His Thr
    1490                1495                1500

Glu Ile Leu Lys Leu Phe Pro Gln Ala Ala Trp Gln Glu Arg Tyr Ser
1505                1510                1515                1520

Ser Leu Met Ala Tyr Lys Leu Pro Val Glu Asp Val His Pro Leu Ser
                1525                1530                1535

Arg Ala Phe Phe Lys Leu Glu Arg Val Lys Gln Thr Phe Asn Leu Glu
            1540                1545                1550

Glu Tyr Ser Leu Ser Gln Ala Thr Leu Glu Gln Val Phe Leu Glu Leu
        1555                1560                1565

Cys Lys Glu Gln Glu Leu Gly Asn Val Asp Asp Lys Ile Asp Thr Thr
    1570                1575                1580

Val Glu Trp Lys Leu Leu Pro Gln Glu Asp Pro
1585                1590                1595

<210> 39
<211> 2540
<212> DNA
<213> Homo sapiens

<400> 39
ggggcggtcg cagctgaagc aggcctaccc tctgaagagg tccaagcaac ggaagtacta 60
ctacgaagct gcctttctgg ccatccttga gaaaaataga cagatggcca aggagagggg 120
cctaataagc cccagtgatt ttgcccagct gcaaaaatac atggaatact ccaccaaaaa 180
ggtcagtgat gtcctaaagc tcttcgagga tggcgagatg gctaaatatg tccaaggaga 240
tgccattggg tacgagggat tccagcaatt cctgaaaatc tatctcgaag tggataatgt 300
tcccagacac ctaagcctgg cactgtttca atcctttgag actggtcact gcttaaatga 360
gacaaatgtg acaaaagatg tggtgtgtct caatgatgtt tcctgctact ttcccttct 420
ggagggtggt cggccagaag acaagttaga attcaccttc aagctgtacg acacggacag 480
aaatgggatc ctggacagct caatgatgcg agtggctgaa tacctggatt gggatgtgtc 540
tgagctgagg ccgattcttc aggagatgat gaaagagatt gactatgatg gcagtggctc 600
tgtctctcaa gctgagtggg tccgggctgg ggccaccacc gtgccactgc tagtgctgct 660
gggtctggag atgactctga aggacgacgg acagcacatg tggaggccca agaggttccc 720
cagaccagtc tactgcaatc tgtgcgagtc aagcattggt cttggcaaac agggactgag 780
ctgtaacctc tgtaagtaca ctgttcacga ccagtgtgcc atgaaagccc tgccttgtga 840
agtcagcacc tatgccaagt ctcggaagga cattggtgtc caatcacatg tgtgggtgcg 900
aggaggctgt gagtccgggc gctgcgaccg ctgtcagaaa aagatccgga tctaccacag 960
tctgaccggg ctgcattgtg tatggtgcca cctagagatc cacgatgact gcctgcaagc 1020
ggtgggccat gagtgtgact gtgggctgct ccgggatcac atcctgcctc catcttccat 1080
ctatcccagt gtcctggcct ctggaccgga tcgtaaaaat agcaaaacaa gccagaagac 1140
catggatgat ttaaatttga gcacctctga ggctctgcgg attgaccctg ttcctaacac 1200
ccacccactt ctcgtctttg tcaatcctaa gagtggcggg aagcaggggc agagggtgct 1260
ctggaagttc cagtatatat taaaccctcg acaggtgttc aacctcctaa aggatggtcc 1320
tgagataggg ctccgattat tcaaggatgt tcctgatagc cggattttgg tgtgtggtgg 1380
agacggcaca gtaggctgga ttctagagac cattgacaaa gctaacttgc cagttttgcc 1440
tcctgttgct gtgttgcccc tgggtactgg aaatgatctg gctcgatgcc taagatgggg 1500
aggaggttat gaaggacaga atctggcaaa gatcctcaag gatttagaga tgagtaaagt 1560
ggtacatatg gatcgatggt ctgtggaggt gatacctcaa caaactgaag aaaaaagtga 1620
cccagtcccc tttcaaatca tcaataacta cttctctatt ggcgtggatg cctctattgc 1680
tcatcgattc cacatcatgc gagagaaata tccggagaag ttcaacagca gaatgaagaa 1740
caagctatgg tacttcgaat ttgccacatc tgaatccatc ttctcaacat gcaaaaagct 1800
ggaggagtct ttgacagttg agatctgtgg gaaaccgctg gatctgagca acctgtccct 1860
agaaggcatc gcagtgctaa acatccctag catgcatggt ggctccaacc tctggggtga 1920
taccaggaga ccccatgggg atatctatgg gatcaaccag gccttaggtg ctacagctaa 1980
agtcatcacc gaccctgata tcctgaaaac ctgtgtacca gacctaagtg acaagagact 2040
ggaagtggtt gggctggagg gtgcaattga gatgggccaa atctatacca agctcaagaa 2100
tgctggacgt cggctggcca agtgctctga gatcaccttc cacaccacaa aaacccttcc 2160
catgcaaatt gacgtagaac cctggatgca gacgccctgt acaatcaaga tcacccacaa 2220
gaaccagatg cccatgctca tgggcccacc cccccgctcc accaatttct ttggcttctt 2280
gagctaaggg ggacaccctt ggcctccaag ccagccttga acccacctcc ctgtccctgg 2340
actctactcc cgaggctctg tacattgctg ccacatactc ctgccagctt gggggagtgt 2400
tccttcaccc tcacagtatt tattatcctg caccacctca ctgttcccca tgcgcacaca 2460
catacacaca ccccaaaaca catacattga aagtgcctca tctgaataaa atgacttgtg 2520
tttccctttg ggatctgctg                                              2540

<210> 40
<211> 727
<212> PRT
<213> Homo sapiens

<400> 40
Met Ala Lys Glu Arg Gly Leu Ile Ser Pro Ser Asp Phe Ala Gln Leu
1               5                   10                  15

Gln Lys Tyr Met Glu Tyr Ser Thr Lys Lys Val Ser Asp Val Leu Lys
            20                  25                  30

Leu Phe Glu Asp Gly Glu Met Ala Lys Tyr Val Gln Gly Asp Ala Ile
        35                  40                  45

Gly Tyr Glu Gly Phe Gln Gln Phe Leu Lys Ile Tyr Leu Glu Val Asp
    50                  55                  60

Asn Val Pro Arg His Leu Ser Leu Ala Leu Phe Gln Ser Phe Glu Thr
65                  70                  75                  80

Gly His Cys Leu Asn Glu Thr Asn Val Thr Lys Asp Val Val Cys Leu
                85                  90                  95

Asn Asp Val Ser Cys Tyr Phe Ser Leu Leu Glu Gly Gly Arg Pro Glu
            100                 105                 110

Asp Lys Leu Glu Phe Thr Phe Lys Leu Tyr Asp Thr Asp Arg Asn Gly
        115                 120                 125

Ile Leu Asp Ser Ser Met Met Arg Val Ala Glu Tyr Leu Asp Trp Asp
    130                 135                 140

Val Ser Glu Leu Arg Pro Ile Leu Gln Glu Met Met Lys Glu Ile Asp
145                 150                 155                 160

Tyr Asp Gly Ser Gly Ser Val Ser Gln Ala Glu Trp Val Arg Ala Gly
                165                 170                 175

Ala Thr Thr Val Pro Leu Leu Val Leu Leu Gly Leu Glu Met Thr Leu
            180                 185                 190

Lys Asp Asp Gly Gln His Met Trp Arg Pro Lys Arg Phe Pro Arg Pro
        195                 200                 205

Val Tyr Cys Asn Leu Cys Glu Ser Ser Ile Gly Leu Gly Lys Gln Gly
    210                 215                 220

Leu Ser Cys Asn Leu Cys Lys Tyr Thr Val His Asp Gln Cys Ala Met
225                 230                 235                 240

Lys Ala Leu Pro Cys Glu Val Ser Thr Tyr Ala Lys Ser Arg Lys Asp
                245                 250                 255

Ile Gly Val Gln Ser His Val Trp Val Arg Gly Gly Cys Glu Ser Gly
            260                 265                 270

Arg Cys Asp Arg Cys Gln Lys Lys Ile Arg Ile Tyr His Ser Leu Thr
        275                 280                 285

Gly Leu His Cys Val Trp Cys His Leu Glu Ile His Asp Asp Cys Leu
    290                 295                 300

Gln Ala Val Gly His Glu Cys Asp Cys Gly Leu Leu Arg Asp His Ile
305                 310                 315                 320

Leu Pro Pro Ser Ser Ile Tyr Pro Ser Val Leu Ala Ser Gly Pro Asp

                325                 330                 335

Arg Lys Asn Ser Lys Thr Ser Gln Lys Thr Met Asp Asp Leu Asn Leu
            340                 345                 350

Ser Thr Ser Glu Ala Leu Arg Ile Asp Pro Val Pro Asn Thr His Pro
        355                 360                 365

Leu Leu Val Phe Val Asn Pro Lys Ser Gly Gly Lys Gln Gly Gln Arg
    370                 375                 380

Val Leu Trp Lys Phe Gln Tyr Ile Leu Asn Pro Arg Gln Val Phe Asn
385                 390                 395                 400

Leu Leu Lys Asp Gly Pro Glu Ile Gly Leu Arg Leu Phe Lys Asp Val
                405                 410                 415

Pro Asp Ser Arg Ile Leu Val Cys Gly Gly Asp Gly Thr Val Gly Trp
            420                 425                 430

Ile Leu Glu Thr Ile Asp Lys Ala Asn Leu Pro Val Leu Pro Pro Val
        435                 440                 445

Ala Val Leu Pro Leu Gly Thr Gly Asn Asp Leu Ala Arg Cys Leu Arg
    450                 455                 460

Trp Gly Gly Gly Tyr Glu Gly Gln Asn Leu Ala Lys Ile Leu Lys Asp
465                 470                 475                 480

Leu Glu Met Ser Lys Val Val His Met Asp Arg Trp Ser Val Glu Val
                485                 490                 495

Ile Pro Gln Gln Thr Glu Glu Lys Ser Asp Pro Val Pro Phe Gln Ile
            500                 505                 510

Ile Asn Asn Tyr Phe Ser Ile Gly Val Asp Ala Ser Ile Ala His Arg
        515                 520                 525

Phe His Ile Met Arg Glu Lys Tyr Pro Glu Lys Phe Asn Ser Arg Met
    530                 535                 540

Lys Asn Lys Leu Trp Tyr Phe Glu Phe Ala Thr Ser Glu Ser Ile Phe
545                 550                 555                 560

Ser Thr Cys Lys Lys Leu Glu Glu Ser Leu Thr Val Glu Ile Cys Gly
                565                 570                 575

Lys Pro Leu Asp Leu Ser Asn Leu Ser Leu Glu Gly Ile Ala Val Leu
            580                 585                 590

Asn Ile Pro Ser Met His Gly Gly Ser Asn Leu Trp Gly Asp Thr Arg
        595                 600                 605

Arg Pro His Gly Asp Ile Tyr Gly Ile Asn Gln Ala Leu Gly Ala Thr
    610                 615                 620

Ala Lys Val Ile Thr Asp Pro Asp Ile Leu Lys Thr Cys Val Pro Asp
625                 630                 635                 640

Leu Ser Asp Lys Arg Leu Glu Val Val Gly Leu Glu Gly Ala Ile Glu
                645                 650                 655

Met Gly Gln Ile Tyr Thr Lys Leu Lys Asn Ala Gly Arg Arg Leu Ala
            660                 665                 670

Lys Cys Ser Glu Ile Thr Phe His Thr Thr Lys Thr Leu Pro Met Gln
        675                 680                 685

Ile Asp Val Glu Pro Trp Met Gln Thr Pro Cys Thr Ile Lys Ile Thr

690                 695                 700

His Lys Asn Gln Met Pro Met Leu Met Gly Pro Pro Pro Arg Ser Thr
705                 710                 715                 720

Asn Phe Phe Gly Phe Leu Ser
                725

<210> 41
<211> 3904
<212> DNA
<213> Homo sapiens

<400> 41
   ttaccggaag taaaacttcg gaagtgaggc gttcctctgc ccggaagtga gcgcggcgct 60
   aggaaagatg gcggcagcgg cggcggtggg caacgcggtg ccctgcgggg cccggccttg 120
   cggggtccgg cctgacgggc agcccaagcc cgggcgcagc cggcgcgcgc tccttgccgc 180
   cgggccggcg ctcatagcga acggtgacga gctggtggct gccgtgtggc cgtaccggcg 240
   gttggcgctg ttgcggcgcc tcacggtgct gccattcgcc gggctgcttt acccggcctg 300
   gttgggtgcc gcagccgctg gctgctgggg ctggggcagc agttgggtgc agatccccga 360
   agctgcgctg ctcgtgcttg ccaccatctg cctcgcgcac gcgctcactg tcctctcggg 420
   gcattggtct gtgcacgcgc attgcgcgct cacctgcacc ccggagtacg accccagcaa 480
   agcgaccttt gtgaaggtgg tgccaacccc caacaatggc tccacggagc tcgtggccct 540
   gcaccgcaat gagggcgaag acgggcttga ggtgctgtcc ttcgaattcc agaagatcaa 600
   gtattcctac gatgccctgg agaagaagca gtttctcccc gtggcctttc ctgtgggaaa 660
   cgccttctca tactatcaga gcaacagagg cttccaggaa gactcagaga tccgagcagc 720
   tgagaagaaa tttgggagca acaaggccga gatggtggtg cctgacttct cggagctttt 780
   caaggagaga gccacagccc ccttctttgt atttcaggtg ttctgtgtgg ggctctggtg 840
   cctggatgag tactggtact acagcgtctt tacgctatcc atgctggtgg cgttcgaggc 900
   ctcgctggtg cagcagcaga tgcggaacat gtcggagatc cggaagatgg gcaacaagcc 960
   ccacatgatc caggtctacc gaagccgcaa gtggaggccc attgccagtg atgagatcgt 1020
   accaggggac atcgtctcca tcggccgctc cccacaggag aacctggtgc catgtgacgt 1080
   gcttctgctg cgaggccgct gcatcgtaga cgaggccatg ctcacggggg agtccgtgcc 1140
   acagatgaag gagcccatcg aagacctcag cccagaccgg gtgctggacc tccaggctga 1200
   ttcccggctg cacgtcatct tcgggggcac caaggtggtg cagcacatcc ccccacagaa 1260
   agccaccacg ggcctgaagc cggttgacag cgggtgcgtg gcctacgtcc tgcggaccgg 1320
   attcaacaca tcccagggca agctgctgcg caccatcctc ttcggggtca agagggtgac 1380
   tgcgaacaac ctggagacct tcatcttcat cctcttcctc ctggtgtttg ccatcgctgc 1440
   agctgcctat gtatggattg aaggtaccaa ggaccccagc cggaaccgct acaagctgtt 1500
   tctggagtgc accctgatcc tcacctcggt cgtgcctcct gagctgccca tcgagctgtc 1560
   cctggccgtc aacacctccc tcatcgccct ggccaagctc tacatgtact gcacagagcc 1620
   cttccggatc ccctttgctg gcaaggtcga ggtgtgctgc tttgacaaga cggggacgtt 1680
   gaccagtgac agcctggtgg tgcgcggtgt ggccgggctg agagacggga aggaggtgac 1740
   cccagtgtcc agcatccctg tagaaacaca ccgggccctg gcctcgtgcc actcgctcat 1800
   gcagctggac gacggcaccc tcgtgggtga ccctctagag aaggccatgc tgacggccgt 1860
   ggactggacg ctgaccaaag atgagaaagt attcccccga agtattaaaa ctcaggggct 1920
   gaaaattcac cagcgctttc attttgccag tgccctgaag cgaatgtccg tgcttgcctc 1980
   gtatgagaag ctgggctcca ccgacctctg ctacatcgcg gccgtgaagg gggcccccga 2040
   aactctgcac tccatgttct cccagtgccc gcccgactac caccacatcc acaccgagat 2100
   ctcccgggaa ggagcccgcg tcctggcgct ggggtacaag gagctgggac acctcactca 2160
   ccagcaggcc cgggaggtca agcgggaggc cctggagtgc agcctcaagt tcgtcggctt 2220
   cattgtggtc tcctgcccgc tcaaggctga ctccaaggcc gtgatccggg agatccagaa 2280
   tgcgtcccac cgggtggtca tgatcacggg agacaacccg ctcactgcat gccacgtggc 2340
   ccaggagctg cacttcattg aaaaggccca cacgctgatc ctgcagcctc cctccgagaa 2400
   aggccggcag tgcgagtggc gctccattga cggcagcatc gtgctgcccc tggcccgggg 2460
   ctccccaaag gcactggccc tggagtacgc actgtgcctc acaggcgacg gcttggccca 2520
   cctgcaggcc accgaccccc agcagctgct ccgcctcatc ccccatgtgc aggtgttcgc 2580
   ccgtgtggct cccaagcaga aggagtttgt catcaccagc ctgaaggagc tgggctacgt 2640
   gaccctcatg tgtggggatg gcaccaacga cgtgggcgcc ctgaagcatg ctgacgtggg 2700
   tgtggcgctc ttggccaatg cccctgagcg ggttgtcgag cggcgacggc ggccccggga 2760
   cagcccaacc ctgagcaaca gtggcatcag agccacctcc aggacagcca agcagcggtc 2820
   ggggctccct ccctccgagg agcagccaac ctcccagagg gaccgcctga gccaggtgct 2880
   gcgagacctc gaggacgaga gtacgcccat tgtgaaactg ggggatgcca gcatcgcagc 2940
   acccttcacc tccaagctct catccatcca gtgcatctgc cacgtgatca agcagggccg 3000
   ctgcacgctg gtgaccacgc tacagatgtt caagatcctg gcgctcaatg ccctcatcct 3060
   ggcctacagc cagagcgtcc tctacctgga gggagtcaag ttcagtgact tccaggccac 3120
   cctacagggg ctgctgctgg ccggctgctt cctcttcatc tcccgttcca agcccctcaa 3180
   gaccctctcc cgagaacggc ccctgcccaa catcttcaac ctgtacacca tcctcaccgt 3240
   catgctccag ttctttgtgc acttcctgag ccttgtctac ctgtaccgtg aggcccaggc 3300
   ccggagcccc gagaagcagg agcagttcgt ggacttgtac aaggagtttg agccaagcct 3360
   ggtcaacagc accgtctaca tcatggccat ggccatgcag atggccacct tcgccatcaa 3420
   ttacaaaggc ccgcccttca tggagagcct gcccgagaac aagcccctgg tgtggagtct 3480
   ggcagtttca ctcctggcca tcattggcct gctcctcggc tcctcgcccg acttcaacag 3540
   ccagtttggc ctcgtggaca tccctgtgga gttcaagctg gtcattgccc aggtcctgct 3600
   cctggacttc tgcctggcgc tcctggccga ccgcgtcctg cagttcttcc tggggacccc 3660
   gaagctgaaa gtgccttcct gagatggcag tgctggtacc cactgcccac cctggctgcc 3720
   gctgggcggg aaccccaaca gggccccggg agggaaccct gcccccaacc ccccacagca 3780
   aggctgtaca gtctcgccct tggaagactg agctgggacc cccacagcca tccgctggct 3840
   tggccagcag aaccagcccc aagccagcac ctttggtaaa taaagcagca tctgagattt 3900
   taaa 3904

<210> 42
<211> 1204
<212> PRT
<213> Homo sapiens

<400> 42
   Met Ala Ala Ala Ala Ala Val Gly Asn Ala Val Pro Cys Gly Ala Arg
   1               5                   10                  15
   Pro Cys Gly Val Arg Pro Asp Gly Gln Pro Lys Pro Gly Arg Ser Arg
               20                  25                  30
   Arg Ala Leu Leu Ala Ala Gly Pro Ala Leu Ile Ala Asn Gly Asp Glu
           35                  40                  45
   Leu Val Ala Ala Val Trp Pro Tyr Arg Arg Leu Ala Leu Leu Arg Arg
       50                  55                  60
   Leu Thr Val Leu Pro Phe Ala Gly Leu Leu Tyr Pro Ala Trp Leu Gly
   65                  70                  75                  80
   Ala Ala Ala Ala Gly Cys Trp Gly Trp Gly Ser Ser Trp Val Gln Ile
                   85                  90                  95
   Pro Glu Ala Ala Leu Leu Val Leu Ala Thr Ile Cys Leu Ala His Ala
               100                 105                 110
   Leu Thr Val Leu Ser Gly His Trp Ser Val His Ala His Cys Ala Leu
           115                 120                 125
   Thr Cys Thr Pro Glu Tyr Asp Pro Ser Lys Ala Thr Phe Val Lys Val
       130                 135                 140
   Val Pro Thr Pro Asn Asn Gly Ser Thr Glu Leu Val Ala Leu His Arg
   145                 150                 155                 160
   Asn Glu Gly Glu Asp Gly Leu Glu Val Leu Ser Phe Glu Phe Gln Lys
                   165                 170                 175
   Ile Lys Tyr Ser Tyr Asp Ala Leu Glu Lys Lys Gln Phe Leu Pro Val
               180                 185                 190
   Ala Phe Pro Val Gly Asn Ala Phe Ser Tyr Tyr Gln Ser Asn Arg Gly
           195                 200                 205
   Phe Gln Glu Asp Ser Glu Ile Arg Ala Ala Glu Lys Lys Phe Gly Ser
       210                 215                 220
   Asn Lys Ala Glu Met Val Val Pro Asp Phe Ser Glu Leu Phe Lys Glu
   225                 230                 235                 240
   Arg Ala Thr Ala Pro Phe Phe Val Phe Gln Val Phe Cys Val Gly Leu
                   245                 250                 255
   Trp Cys Leu Asp Glu Tyr Trp Tyr Tyr Ser Val Phe Thr Leu Ser Met
   260 265 270
   Leu Val Ala Phe Glu Ala Ser Leu Val Gln Gln Gln Met Arg Asn Met
   275 280 285
   Ser Glu Ile Arg Lys Met Gly Asn Lys Pro His Met Ile Gln Val Tyr
   290 295 300
   Arg Ser Arg Lys Trp Arg Pro Ile Ala Ser Asp Glu Ile Val Pro Gly
   305 310 315 320
   Asp Ile Val Ser Ile Gly Arg Ser Pro Gln Glu Asn Leu Val Pro Cys
   325 330 335
   Asp Val Leu Leu Leu Arg Gly Arg Cys Ile Val Asp Glu Ala Met Leu
   340 345 350
   Thr Gly Glu Ser Val Pro Gln Met Lys Glu Pro Ile Glu Asp Leu Ser
   355 360 365
   Pro Asp Arg Val Leu Asp Leu Gln Ala Asp Ser Arg Leu His Val Ile
   370 375 380
   Phe Gly Gly Thr Lys Val Val Gln His Ile Pro Pro Gln Lys Ala Thr
   385 390 395 400
   Thr Gly Leu Lys Pro Val Asp Ser Gly Cys Val Ala Tyr Val Leu Arg
   405 410 415
   Thr Gly Phe Asn Thr Ser Gln Gly Lys Leu Leu Arg Thr Ile Leu Phe
   420 425 430
   Gly Val Lys Arg Val Thr Ala Asn Asn Leu Glu Thr Phe Ile Phe Ile
   435 440 445
   Leu Phe Leu Leu Val Phe Ala Ile Ala Ala Ala Ala Tyr Val Trp Ile
   450 455 460
   Glu Gly Thr Lys Asp Pro Ser Arg Asn Arg Tyr Lys Leu Phe Leu Glu
   465 470 475 480
   Cys Thr Leu Ile Leu Thr Ser Val Val Pro Pro Glu Leu Pro Ile Glu
   485 490 495
   Leu Ser Leu Ala Val Asn Thr Ser Leu Ile Ala Leu Ala Lys Leu Tyr
   500 505 510
   Met Tyr Cys Thr Glu Pro Phe Arg Ile Pro Phe Ala Gly Lys Val Glu
   515 520 525
   Val Cys Cys Phe Asp Lys Thr Gly Thr Leu Thr Ser Asp Ser Leu Val
   530 535 540
   Val Arg Gly Val Ala Gly Leu Arg Asp Gly Lys Glu Val Thr Pro Val
   545 550 555 560
   Ser Ser Ile Pro Val Glu Thr His Arg Ala Leu Ala Ser Cys His Ser
   565 570 575
   Leu Met Gln Leu Asp Asp Gly Thr Leu Val Gly Asp Pro Leu Glu Lys
   580 585 590
   Ala Met Leu Thr Ala Val Asp Trp Thr Leu Thr Lys Asp Glu Lys Val
   595 600 605
   Phe Pro Arg Ser Ile Lys Thr Gln Gly Leu Lys Ile His Gln Arg Phe
   610 615 620
   His Phe Ala Ser Ala Leu Lys Arg Met Ser Val Leu Ala Ser Tyr Glu
   625                 630                 635                 640
   Lys Leu Gly Ser Thr Asp Leu Cys Tyr Ile Ala Ala Val Lys Gly Ala
                   645                 650                 655
   Pro Glu Thr Leu His Ser Met Phe Ser Gln Cys Pro Pro Asp Tyr His
               660                 665                 670
   His Ile His Thr Glu Ile Ser Arg Glu Gly Ala Arg Val Leu Ala Leu
           675                 680                 685
   Gly Tyr Lys Glu Leu Gly His Leu Thr His Gln Gln Ala Arg Glu Val
       690                 695                 700
   Lys Arg Glu Ala Leu Glu Cys Ser Leu Lys Phe Val Gly Phe Ile Val
   705                 710                 715                 720
   Val Ser Cys Pro Leu Lys Ala Asp Ser Lys Ala Val Ile Arg Glu Ile
                   725                 730                 735
   Gln Asn Ala Ser His Arg Val Val Met Ile Thr Gly Asp Asn Pro Leu
               740                 745                 750
   Thr Ala Cys His Val Ala Gln Glu Leu His Phe Ile Glu Lys Ala His
           755                 760                 765
   Thr Leu Ile Leu Gln Pro Pro Ser Glu Lys Gly Arg Gln Cys Glu Trp
       770                 775                 780
   Arg Ser Ile Asp Gly Ser Ile Val Leu Pro Leu Ala Arg Gly Ser Pro
   785                 790                 795                 800
   Lys Ala Leu Ala Leu Glu Tyr Ala Leu Cys Leu Thr Gly Asp Gly Leu
                   805                 810                 815
   Ala His Leu Gln Ala Thr Asp Pro Gln Gln Leu Leu Arg Leu Ile Pro
               820                 825                 830
   His Val Gln Val Phe Ala Arg Val Ala Pro Lys Gln Lys Glu Phe Val
           835                 840                 845
   Ile Thr Ser Leu Lys Glu Leu Gly Tyr Val Thr Leu Met Cys Gly Asp
       850                 855                 860
   Gly Thr Asn Asp Val Gly Ala Leu Lys His Ala Asp Val Gly Val Ala
   865                 870                 875                 880
   Leu Leu Ala Asn Ala Pro Glu Arg Val Val Glu Arg Arg Arg Arg Pro
                   885                 890                 895
   Arg Asp Ser Pro Thr Leu Ser Asn Ser Gly Ile Arg Ala Thr Ser Arg
               900                 905                 910
   Thr Ala Lys Gln Arg Ser Gly Leu Pro Pro Ser Glu Glu Gln Pro Thr
           915                 920                 925
   Ser Gln Arg Asp Arg Leu Ser Gln Val Leu Arg Asp Leu Glu Asp Glu
       930                 935                 940
   Ser Thr Pro Ile Val Lys Leu Gly Asp Ala Ser Ile Ala Ala Pro Phe
   945                 950                 955                 960
   Thr Ser Lys Leu Ser Ser Ile Gln Cys Ile Cys His Val Ile Lys Gln
                   965                 970                 975
   Gly Arg Cys Thr Leu Val Thr Thr Leu Gln Met Phe Lys Ile Leu Ala
               980                 985                 990
   Leu Asn Ala Leu Ile Leu Ala Tyr Ser Gln Ser Val Leu Tyr Leu Glu
   995                 1000                1005
   Gly Val Lys Phe Ser Asp Phe Gln Ala Thr Leu Gln Gly Leu Leu Leu
       1010                1015                1020
   Ala Gly Cys Phe Leu Phe Ile Ser Arg Ser Lys Pro Leu Lys Thr Leu
   1025                1030                1035                1040
   Ser Arg Glu Arg Pro Leu Pro Asn Ile Phe Asn Leu Tyr Thr Ile Leu
                   1045                1050                1055
   Thr Val Met Leu Gln Phe Phe Val His Phe Leu Ser Leu Val Tyr Leu
               1060                1065                1070
   Tyr Arg Glu Ala Gln Ala Arg Ser Pro Glu Lys Gln Glu Gln Phe Val
           1075                1080                1085
   Asp Leu Tyr Lys Glu Phe Glu Pro Ser Leu Val Asn Ser Thr Val Tyr
       1090                1095                1100
   Ile Met Ala Met Ala Met Gln Met Ala Thr Phe Ala Ile Asn Tyr Lys
   1105                1110                1115                1120
   Gly Pro Pro Phe Met Glu Ser Leu Pro Glu Asn Lys Pro Leu Val Trp
                   1125                1130                1135
   Ser Leu Ala Val Ser Leu Leu Ala Ile Ile Gly Leu Leu Leu Gly Ser
               1140                1145                1150
   Ser Pro Asp Phe Asn Ser Gln Phe Gly Leu Val Asp Ile Pro Val Glu
           1155                1160                1165
   Phe Lys Leu Val Ile Ala Gln Val Leu Leu Leu Asp Phe Cys Leu Ala
       1170                1175                1180
   Leu Leu Ala Asp Arg Val Leu Gln Phe Phe Leu Gly Thr Pro Lys Leu
   1185                1190                1195                1200
   Lys Val Pro Ser



<210> 43
<211> 1167
<212> DNA
<213> Homo sapiens

<400> 43
atggggcagt tttataggac tggggcaggc agtggaaagt tacagttaaa ggtggttatc 60
tattgtcagc tgaggaggga tcacaaggtg aatggtgagg agatcataag actcattgtc 120
caaaagagga atgtcacgag gtcaatcgat cgatcagttg gggcagggca ggaacaagtc 180
ataatggaat gtcttaagcc tttttacttc aactatccat ctctagacag tgaggtcctg 240
gatgatgaca gagccataga tggaaaagac accattattc tggtctataa agaactgtct 300
agggacttgg catcctgtgt cccagccact ccagctgtgg ctgaaagggt ccaaggtaca 360
gttcaggcca tggcttcaaa gggtgcaagc cccaagtctc ggcagctttc acaaggtgtt 420
aagcctggca gtacagaagg gaaatgtggg ttggagcccc aacacagagc cccactgggg 480
acactgccta gtggagcttt gagaagaggg ccaccattct ccagacccca gaatggtaga 540
cccactgaca gcttgcacta tgcacttgga aaagacacag acactcaaca ccagcccatg 600
aaagcagcca gaagggaggc tgtaccctgc acagctacag gggcagagct gcccaagacc 660
atgggaaccc aactgttgca tcagcatgac ccagatgtga gaattggagt caaagaagat 720
cattttggag ctttaagatt tgactgtcct tctagatttt ggacttacat gaggacccca 780
gccttgctgc tctgcccatt gacctctgcc accctccata ccgggtgtga gttaccacca 840
gaagaagtct gtggcagcag cagtctttgc cttattctga cagtgccaaa gtgtgtttgg 900
ccctaccaac aactgcaagc cctttccttg ctttacttta gctttggctc cagaaggctc 960
attgctttga aatgtaacat atcctggtcc aactacatcc gattccatgg gtctgcatca 1020
ttatctccaa aaccttccag ttgcattgct gtcatgtttg tgttaaagag actgatgagt 1080
ctggactatg accggaagaa agtctccacg gccgccatct tggccaggat taaaagaact 1140
ggagatggaa gctacaagag tggctga                                     1167

<210> 44
<211> 388
<212> PRT
<213> Homo sapiens

<400> 44
Met Gly Gln Phe Tyr Arg Thr Gly Ala Gly Ser Gly Lys Leu Gln Leu
1               5                   10                  15

Lys Val Val Ile Tyr Cys Gln Leu Arg Arg Asp His Lys Val Asn Gly
            20                  25                  30

Glu Glu Ile Ile Arg Leu Ile Val Gln Lys Arg Asn Val Thr Arg Ser
        35                  40                  45

Ile Asp Arg Ser Val Gly Ala Gly Gln Glu Gln Val Ile Met Glu Cys
    50                  55                  60

Leu Lys Pro Phe Tyr Phe Asn Tyr Pro Ser Leu Asp Ser Glu Val Leu
65                  70                  75                  80

Asp Asp Asp Arg Ala Ile Asp Gly Lys Asp Thr Ile Ile Leu Val Tyr
                85                  90                  95

Lys Glu Leu Ser Arg Asp Leu Ala Ser Cys Val Pro Ala Thr Pro Ala
            100                 105                 110

Val Ala Glu Arg Val Gln Gly Thr Val Gln Ala Met Ala Ser Lys Gly
        115                 120                 125

Ala Ser Pro Lys Ser Arg Gln Leu Ser Gln Gly Val Lys Pro Gly Ser
    130                 135                 140

Thr Glu Gly Lys Cys Gly Leu Glu Pro Gln His Arg Ala Pro Leu Gly
145                 150                 155                 160

Thr Leu Pro Ser Gly Ala Leu Arg Arg Gly Pro Pro Phe Ser Arg Pro
                165                 170                 175

Gln Asn Gly Arg Pro Thr Asp Ser Leu His Tyr Ala Leu Gly Lys Asp
            180                 185                 190

Thr Asp Thr Gln His Gln Pro Met Lys Ala Ala Arg Arg Glu Ala Val
        195                 200                 205

Pro Cys Thr Ala Thr Gly Ala Glu Leu Pro Lys Thr Met Gly Thr Gln
    210                 215                 220

Leu Leu His Gln His Asp Pro Asp Val Arg Ile Gly Val Lys Glu Asp
225                 230                 235                 240

His Phe Gly Ala Leu Arg Phe Asp Cys Pro Ser Arg Phe Trp Thr Tyr
                245                 250                 255

Met Arg Thr Pro Ala Leu Leu Leu Cys Pro Leu Thr Ser Ala Thr Leu
            260                 265                 270

His Thr Gly Cys Glu Leu Pro Pro Glu Glu Val Cys Gly Ser Ser Ser
        275                 280                 285

Leu Cys Leu Ile Leu Thr Val Pro Lys Cys Val Trp Pro Tyr Gln Gln
    290                 295                 300

Leu Gln Ala Leu Ser Leu Leu Tyr Phe Ser Phe Gly Ser Arg Arg Leu
305                 310                 315                 320

Ile Ala Leu Lys Cys Asn Ile Ser Trp Ser Asn Tyr Ile Arg Phe His
                325                 330                 335

Gly Ser Ala Ser Leu Ser Pro Lys Pro Ser Ser Cys Ile Ala Val Met
            340                 345                 350

Phe Val Leu Lys Arg Leu Met Ser Leu Asp Tyr Asp Arg Lys Lys Val
        355                 360                 365

Ser Thr Ala Ala Ile Leu Ala Arg Ile Lys Arg Thr Gly Asp Gly Ser
    370                 375                 380

Tyr Lys Ser Gly
385

<210> 45
<211> 853
<212> DNA
<213> Homo sapiens

<400> 45
atggctgatc cttggcagga atgcatggat tatgcagtaa ccctagcaag acaagctgga 60
gaggtggttc atgatgctct taaaaatgaa gtgaatgtta tcctgaaagg ttctccagtt 120
gatttggtaa ctgctactga ccaaaaagtt gaaaaaatgc ttatctcttc cataaaggaa 180
aagtatccat ctcataggta tttttttatt gtgaggaatc tggcagctgg ggaaaaaggt 240
gtcttaactg acaaccctac gtggatcatt gaccctattg atggaacaac taagtttgtc 300
catagatttc cttttgtagc tgtttcgatt ggccttgttg taaataagaa ggtagaattt 360
ggagttgtgt acagttgtgt ggaagacaag aggtacactg tcaggaaagg aaaaggtgcc 420
ttttataatg gtcaaaaact acaggtttca caagaaggtg atattaccaa atcactcttg 480
gtgaccgagc tgggctattg cagaacatca gaaattgtaa gaactattct ttccaatatg 540
gaaaagcttt cttgcattcc tattcacggt atccagagtg ttggaacagc agctactaat 600
atgtgcattg cggcaagtgg aggagcagag gcattttatg aaatgggaat tcactgctgg 660
gatattgcag tagctgccat tattgttact gaagctggtg gcgtgctaat ggatgttact 720
ggtggaccat tccatttaat gtcacggaga ataattgctg caaattgtac agcattagca 780
gaaaggatag ccaaagaaat tcaggtagca ccttttcaat gagatgatga agattaatta 840
cagcagcctc ata 853

<210> 46
<211> 273
<212> PRT
<213> Homo sapiens

<400> 46
Met Ala Asp Pro Trp Gln Glu Cys Met Asp Tyr Ala Val Thr Leu Ala
1               5                   10                  15

Arg Gln Ala Gly Glu Val Val His Asp Ala Leu Lys Asn Glu Val Asn
            20                  25                  30

Val Ile Leu Lys Gly Ser Pro Val Asp Leu Val Thr Ala Thr Asp Gln
        35                  40                  45

Lys Val Glu Lys Met Leu Ile Ser Ser Ile Lys Glu Lys Tyr Pro Ser
    50                  55                  60

His Arg Tyr Phe Phe Ile Val Arg Asn Leu Ala Ala Gly Glu Lys Gly
65                  70                  75                  80

Val Leu Thr Asp Asn Pro Thr Trp Ile Ile Asp Pro Ile Asp Gly Thr
                85                  90                  95

Thr Lys Phe Val His Arg Phe Pro Phe Val Ala Val Ser Ile Gly Leu
            100                 105                 110

Val Val Asn Lys Lys Val Glu Phe Gly Val Val Tyr Ser Cys Val Glu
        115                 120                 125

Asp Lys Arg Tyr Thr Val Arg Lys Gly Lys Gly Ala Phe Tyr Asn Gly
    130                 135                 140

Gln Lys Leu Gln Val Ser Gln Glu Gly Asp Ile Thr Lys Ser Leu Leu
145                 150                 155                 160

Val Thr Glu Leu Gly Tyr Cys Arg Thr Ser Glu Ile Val Arg Thr Ile
                165                 170                 175

Leu Ser Asn Met Glu Lys Leu Ser Cys Ile Pro Ile His Gly Ile Gln
            180                 185                 190

Ser Val Gly Thr Ala Ala Thr Asn Met Cys Ile Ala Ala Ser Gly Gly
        195                 200                 205

Ala Glu Ala Phe Tyr Glu Met Gly Ile His Cys Trp Asp Ile Ala Val
    210                 215                 220

Ala Ala Ile Ile Val Thr Glu Ala Gly Gly Val Leu Met Asp Val Thr
225                 230                 235                 240

Gly Gly Pro Phe His Leu Met Ser Arg Arg Ile Ile Ala Ala Asn Cys
                245                 250                 255

Thr Ala Leu Ala Glu Arg Ile Ala Lys Glu Ile Gln Val Ala Pro Phe
            260                 265                 270

Gln

<210> 47
<211> 2071
<212> DNA
<213> Homo sapiens

<400> 47
ccccccgccg ccccgatccg ggccggcatg atgtgcctgg aatgcgcctc ggcggcggcg 60
ggcggcgcgg aggaggagga ggcggacgcg gagcggcggc gccggcgccg gggggcgcag 120
cgaggggctg gcggtagcgg ttgctgcggg gcgcggggcg cgggcggcgc tggagtctcg 180
gccgcgggcg atgaggtgca gacgctgtcg ggcagcgtaa ggcgggcccc gaccggaccc 240
cccggcaccc ccggcacccc cggctgcgca gctactgcaa agggccccgg cgctcagcag 300
cccaaaccgg ccagcttggg ccgcgggcgg ggggcagccg ccgccatcct cagcttgggc 360
aacgtgctca actacctgga caggtacacc gtggcaggcg tccttctgga catccagcag 420
cactttgggg tcaaggaccg aggcgccggc ctgctgcagt cagtgttcat ctgtagcttc 480
atggtggctg cccccatctt cggctacctg ggcgaccgct tcaacaggaa ggtgattctc 540
agctgcggca ttttcttctg gtcggccgtc accttctcca gctccttcat tccccagcag 600
tacttctggc tgctggtcct gtcccggggg ctggtgggca tcggggaggc cagctactcc 660
accatcgccc ccactatcat tggcgacctc ttcaccaaga acacgcgtac gctcatgctg 720
tccgtcttct acttcgccat cccactgggc agtggcctgg gctacattac tggctccagc 780
gtgaagcagg cagccggaga ctggcactgg gcattgcggg tgtcccctgt cctgggcatg 840
atcacaggaa cactcatcct cattctggtc ccagccacta aaaggggtca tgccgaccag 900
ctcggggacc agctcaaggc ccggacctca tggctccgag atatgaaggc cctgattcga 960
aaccgcagct acgtcttctc ctccctggcc acgtcggctg tctccttcgc cacgggggcc 1020
ctgggcatgt ggatcccgct ctacctgcac cgcgcccaag ttgtgcagaa gacagcagag 1080
acgtgcaaca gcccgccctg tggggccaag gacagcctca tctttggggc catcacctgc 1140
tttacgggat ttctgggcgt ggtcacgggg gcaggagcca cgcgctggtg ccgcctgaag 1200
acccagcggg ccgacccact ggtgtgtgcc gtgggcatgc tgggctctgc catcttcatc 1260
tgcctgatct tcgtggctgc caagagcagc atcgtaggag cctatatctg tatcttcgtc 1320
ggggagacgc tgctgttttc taactgggcc atcactgcag acatcctcat gtacgtggtc 1380
atccccacgc ggcgcgccac tgccgtggcc ttgcagagct tcacctccca cctgctgggg 1440
gacgccggga gcccctacct cattggcttt atctcagacc tgatccgcca gagcactaag 1500
gactccccgc tctgggagtt cctgagcctg ggctacgcgc tcatgctctg ccctttcgtc 1560
gtggtcctgg gcggcatgtt cttcctcgcc actgcgctct tcttcgtcag cgaccgcgcc 1620
agggctgagc agcacctggg ggagagacgg gcgggggtca gggtggtgca tcagcggggg 1680
ccgggcccgg gcactgctct ggcacatcgt gtcgtggggg ccagctgacc ggaggtgctg 1740
ggcagggacc tcgtcagccc caggggggaga tgggagagcc caggggtggg gagaggagag 1800
agagaggagt aaagaggaaa ggagaaagaa gtcagaaagt aagaggaagg ggaggggccc 1860
cagctttgaa aaccactaag tccagagaca aacccaagtc tggatccacc agacaccccc 1920
gtggcctccc acagctccag gctgaccctg gcactgggcc tcagggctgg accccagcaa 1980
ccagtgggtg cactgagtgc atgggaggtc tgtaccctcc ccgccccacc ccagggcagg 2040

gctcacggtg gctatcacgg tccctgcttc c                                    2071

<210> 48
<211> 566
<212> PRT
<213> Homo sapiens

<400> 48
Met Met Cys Leu Glu Cys Ala Ser Ala Ala Ala Gly Gly Ala Glu Glu
1               5                   10                  15

Glu Glu Ala Asp Ala Glu Arg Arg Arg Arg Arg Arg Gly Ala Gln Arg
            20                  25                  30

Gly Ala Gly Gly Ser Gly Cys Cys Gly Ala Arg Gly Ala Gly Gly Ala
        35                  40                  45

Gly Val Ser Ala Ala Gly Asp Glu Val Gln Thr Leu Ser Gly Ser Val
    50                  55                  60

Arg Arg Ala Pro Thr Gly Pro Pro Gly Thr Pro Gly Thr Pro Gly Cys
65                  70                  75                  80

Ala Ala Thr Ala Lys Gly Pro Gly Ala Gln Gln Pro Lys Pro Ala Ser
                85                  90                  95

Leu Gly Arg Gly Arg Gly Ala Ala Ala Ala Ile Leu Ser Leu Gly Asn
            100                 105                 110

Val Leu Asn Tyr Leu Asp Arg Tyr Thr Val Ala Gly Val Leu Leu Asp
        115                 120                 125

Ile Gln Gln His Phe Gly Val Lys Asp Arg Gly Ala Gly Leu Leu Gln
    130                 135                 140

Ser Val Phe Ile Cys Ser Phe Met Val Ala Ala Pro Ile Phe Gly Tyr
145                 150                 155                 160

Leu Gly Asp Arg Phe Asn Arg Lys Val Ile Leu Ser Cys Gly Ile Phe
                165                 170                 175

Phe Trp Ser Ala Val Thr Phe Ser Ser Ser Phe Ile Pro Gln Gln Tyr
            180                 185                 190

Phe Trp Leu Leu Val Leu Ser Arg Gly Leu Val Gly Ile Gly Glu Ala
        195                 200                 205

Ser Tyr Ser Thr Ile Ala Pro Thr Ile Ile Gly Asp Leu Phe Thr Lys
    210                 215                 220

Asn Thr Arg Thr Leu Met Leu Ser Val Phe Tyr Phe Ala Ile Pro Leu
225                 230                 235                 240

Gly Ser Gly Leu Gly Tyr Ile Thr Gly Ser Ser Val Lys Gln Ala Ala
                245                 250                 255

Gly Asp Trp His Trp Ala Leu Arg Val Ser Pro Val Leu Gly Met Ile
            260                 265                 270

Thr Gly Thr Leu Ile Leu Ile Leu Val Pro Ala Thr Lys Arg Gly His
        275                 280                 285

Ala Asp Gln Leu Gly Asp Gln Leu Lys Ala Arg Thr Ser Trp Leu Arg
    290                 295                 300

Asp Met Lys Ala Leu Ile Arg Asn Arg Ser Tyr Val Phe Ser Ser Leu
305                 310                 315                 320

Ala Thr Ser Ala Val Ser Phe Ala Thr Gly Ala Leu Gly Met Trp Ile
                325                 330                 335

Pro Leu Tyr Leu His Arg Ala Gln Val Val Gln Lys Thr Ala Glu Thr
            340                 345                 350

Cys Asn Ser Pro Pro Cys Gly Ala Lys Asp Ser Leu Ile Phe Gly Ala
        355                 360                 365

Ile Thr Cys Phe Thr Gly Phe Leu Gly Val Val Thr Gly Ala Gly Ala
    370                 375                 380

Thr Arg Trp Cys Arg Leu Lys Thr Gln Arg Ala Asp Pro Leu Val Cys
385                 390                 395                 400

Ala Val Gly Met Leu Gly Ser Ala Ile Phe Ile Cys Leu Ile Phe Val
                405                 410                 415

Ala Ala Lys Ser Ser Ile Val Gly Ala Tyr Ile Cys Ile Phe Val Gly
            420                 425                 430

Glu Thr Leu Leu Phe Ser Asn Trp Ala Ile Thr Ala Asp Ile Leu Met
        435                 440                 445

Tyr Val Val Ile Pro Thr Arg Arg Ala Thr Ala Val Ala Leu Gln Ser
    450                 455                 460

Phe Thr Ser His Leu Leu Gly Asp Ala Gly Ser Pro Tyr Leu Ile Gly
465                 470                 475                 480

Phe Ile Ser Asp Leu Ile Arg Gln Ser Thr Lys Asp Ser Pro Leu Trp
                485                 490                 495

Glu Phe Leu Ser Leu Gly Tyr Ala Leu Met Leu Cys Pro Phe Val Val
            500                 505                 510

Val Leu Gly Gly Met Phe Phe Leu Ala Thr Ala Leu Phe Phe Val Ser
        515                 520                 525

Asp Arg Ala Arg Ala Glu Gln His Leu Gly Glu Arg Arg Ala Gly Val
    530                 535                 540

Arg Val Val His Gln Arg Gly Pro Gly Pro Gly Thr Ala Leu Ala His
545                 550                 555                 560

Arg Val Val Gly Ala Ser
                565

<210> 49
<211> 752
<212> DNA
<213> Homo sapiens

<400> 49
caaacaacca tgaagttctt catctttacc tgccttttgg ctgttgctct ggcacatcat 60
gaacttaaac acgtttacaa gaaaaaaaca aacaacaacg tgagtgacaa atacagaaat 120
gtgaaaaacc agatttcttc tcctcaggag gacaaagtta gaggtaattt tcattcaaat 180
aaaataaaat taataccact tagtagtgtt ttatttttgt atatttgtat acaaattaat 240
tttttttctt accaggaagt taagcacact gtggatcaaa agcactacgt aaaacaactg 300
aacaaaatca acccatttta tcagaagtgg aacttcctcc catttcttca ggttccttat 360
caacatcaga tttttataaa cccaggagat cagcataaga caagtgtcta cccctttgtt 420
cccactaaat atatacagtg gccaggctca gtggctcagg ccttcttgtt ttattccttt 480
aaggaaacac caaaaaagac tgttgatatg gtaaagtatt gtttctatca gaaaactgag 540
ctgactgaag aagaaaagaa tgaccaaaaa catctgaaca aaatcaacca gtattatcag 600
ttcaccttgc cccaatatgt aaaagctgtt tatcaatatc acaaaattat gaaaccatgg 660
aaaaacatga agacaaatgc ttaccaagtt atccccactc tggtgagtgc tctcttttta 720
tttgcaactt aaaaatagtt atctgttgtg ct 752

<210> 50
<211> 240
<212> PRT
<213> Homo sapiens

<400> 50
Met Lys Phe Phe Ile Phe Thr Cys Leu Leu Ala Val Ala Leu Ala His
1               5                   10                  15

His Glu Leu Lys His Val Tyr Lys Lys Lys Thr Asn Asn Asn Val Ser
            20                  25                  30

Asp Lys Tyr Arg Asn Val Lys Asn Gln Ile Ser Ser Pro Gln Glu Asp
        35                  40                  45

Lys Val Arg Gly Asn Phe His Ser Asn Lys Ile Lys Leu Ile Pro Leu
    50                  55                  60

Ser Ser Val Leu Phe Leu Tyr Ile Cys Ile Gln Ile Asn Phe Phe Ser
65                  70                  75                  80

Tyr Gln Glu Val Lys His Thr Val Asp Gln Lys His Tyr Val Lys Gln
                85                  90                  95

Leu Asn Lys Ile Asn Pro Phe Tyr Gln Lys Trp Asn Phe Leu Pro Phe
            100                 105                 110

Leu Gln Val Pro Tyr Gln His Gln Ile Phe Ile Asn Pro Gly Asp Gln
        115                 120                 125

His Lys Thr Ser Val Tyr Pro Phe Val Pro Thr Lys Tyr Ile Gln Trp
    130                 135                 140

Pro Gly Ser Val Ala Gln Ala Phe Leu Phe Tyr Ser Phe Lys Glu Thr
145                 150                 155                 160

Pro Lys Lys Thr Val Asp Met Val Lys Tyr Cys Phe Tyr Gln Lys Thr
                165                 170                 175

Glu Leu Thr Glu Glu Glu Lys Asn Asp Gln Lys His Leu Asn Lys Ile
            180                 185                 190

Asn Gln Tyr Tyr Gln Phe Thr Leu Pro Gln Tyr Val Lys Ala Val Tyr
        195                 200                 205

Gln Tyr His Lys Ile Met Lys Pro Trp Lys Asn Met Lys Thr Asn Ala
    210                 215                 220

Tyr Gln Val Ile Pro Thr Leu Val Ser Ala Leu Phe Leu Phe Ala Thr
225                 230                 235                 240

<210> 51
<211> 1704
<212> DNA
<213> Homo sapiens

<400> 51
cctgacgctt tgatggtatc tgcaagcgtt tttgctgatc ttatctctgc cccctgaata 60
ttaattccct aatctggtag caatccatct ccccagtgaa ggacctacta gaggcaggtg 120
gggggagcca ccatcagatc atcaagcata agaataatac aaaggggagg gattcttctg 180
caaccaagag gcaagaggcg agagaaggaa aaaaaaaaaa aaagcgatga gttcaccaaa 240
tatatggagc acaggaagct cagtctactc gactcctgta ttttcacaga aaatgacggt 300
gtggattctg ctcctgctgt cgctctaccc tggcttcact agccagaaat ctgatgatga 360
ctatgaagat tatgcttcta acaaaacatg ggtcttgact ccaaaagttc ctgagggtga 420

tgtcactgtc atcttaaaca acctgctgga aggatatgac aataaacttc ggcctgatat 480
aggagtgaag ccaacgttaa ttcacacaga catgtatgtg aatagcattg gtccagtgaa 540
cgctatcaat atggaataca ctattgatat atttttgcg caaatgtggt atgacagacg 600
tttgaaattt aacagcacca ttaaagtcct ccgattgaac agcaacatgg tggggaaaat 660
ctggattcca gacactttct tcagaaattc caaaaaagct gatgcacact ggatcaccac 720
ccccaacagg atgctgagaa tttggaatga tggtcgagtg ctctactccc taaggttgac 780
aattgatgct gagtgccaat tacaattgca caatttccca atggatgaac actcctgccc 840
cttggagttc tccagttatg gctatccacg tgaagaaatt gtttatcaat ggaagcgaag 900
ttctgttgaa gtgggcgaca caagatcctg gaggctttat caattctcat ttgttggtct 960
aagaaatacc accgaagtag tgaagacaac ttccggagat tatgtggtca tgtctgtcta 1020
ctttgatctg agcagaagaa tgggatactt taccatccag acctatatcc cctgcacact 1080
cattgtcgtc ctatcctggg tgtctttctg gatcaataag gatgctgttc cagccagaac 1140
atctttaggt atcaccactg tcctgacaat gaccaccctc agcaccattg cccggaaatc 1200
gctccccaag gtctcctatg tcacagcgat ggatctcttt gtatctgttt gtttcatctt 1260
tgtcttctct gctctggtgg agtatggcac cttgcattat tttgtcagca accggaaacc 1320
aagcaaggac aaagataaaa agaagaaaaa ccctcttctt cggatgtttt ccttcaaggc 1380
ccctaccatt gatatccgcc caagatcagc aaccattcaa atgaataatg ctacacacct 1440
tcaagagaga gatgaagagt acggctatga gtgtctggac ggcaaggact gtgccagttt 1500
tttctgctgt tttgaagatt gtcgaacagg agcttggaga catggaggga tacatatccg 1560
cattgccaaa atggactcct atgctcggat cttcttcccc actgccttct gcctgtttaa 1620
tctggtctat tgggtctcct acctctacct gtgaggaggt atgggtttta ctgatatggt 1680
tcttattcac tgagtctcat ggag 1704

<210> 52
<211> 475
<212> PRT
<213> Homo sapiens

<400> 52
Met Ser Ser Pro Asn Ile Trp Ser Thr Gly Ser Ser Val Tyr Ser Thr
1               5                   10                  15

Pro Val Phe Ser Gln Lys Met Thr Val Trp Ile Leu Leu Leu Leu Ser
            20                  25                  30

Leu Tyr Pro Gly Phe Thr Ser Gln Lys Ser Asp Asp Asp Tyr Glu Asp
        35                  40                  45

Tyr Ala Ser Asn Lys Thr Trp Val Leu Thr Pro Lys Val Pro Glu Gly
    50                  55                  60

Asp Val Thr Val Ile Leu Asn Asn Leu Leu Glu Gly Tyr Asp Asn Lys
65                  70                  75                  80

Leu Arg Pro Asp Ile Gly Val Lys Pro Thr Leu Ile His Thr Asp Met
                85                  90                  95

Tyr Val Asn Ser Ile Gly Pro Val Asn Ala Ile Asn Met Glu Tyr Thr
            100                 105                 110

Ile Asp Ile Phe Phe Ala Gln Met Trp Tyr Asp Arg Arg Leu Lys Phe
        115                 120                 125

Asn Ser Thr Ile Lys Val Leu Arg Leu Asn Ser Asn Met Val Gly Lys
    130                 135                 140

Ile Trp Ile Pro Asp Thr Phe Phe Arg Asn Ser Lys Lys Ala Asp Ala
145                 150                 155                 160

His Trp Ile Thr Thr Pro Asn Arg Met Leu Arg Ile Trp Asn Asp Gly
                165                 170                 175

Arg Val Leu Tyr Ser Leu Arg Leu Thr Ile Asp Ala Glu Cys Gln Leu
            180                 185                 190

Gln Leu His Asn Phe Pro Met Asp Glu His Ser Cys Pro Leu Glu Phe
        195                 200                 205

Ser Ser Tyr Gly Tyr Pro Arg Glu Glu Ile Val Tyr Gln Trp Lys Arg
    210                 215                 220

Ser Ser Val Glu Val Gly Asp Thr Arg Ser Trp Arg Leu Tyr Gln Phe
225                 230                 235                 240

Ser Phe Val Gly Leu Arg Asn Thr Thr Glu Val Val Lys Thr Thr Ser
                245                 250                 255

Gly Asp Tyr Val Val Met Ser Val Tyr Phe Asp Leu Ser Arg Arg Met
            260                 265                 270

Gly Tyr Phe Thr Ile Gln Thr Tyr Ile Pro Cys Thr Leu Ile Val Val
        275                 280                 285

Leu Ser Trp Val Ser Phe Trp Ile Asn Lys Asp Ala Val Pro Ala Arg
    290                 295                 300

Thr Ser Leu Gly Ile Thr Thr Val Leu Thr Met Thr Thr Leu Ser Thr
305                 310                 315                 320

Ile Ala Arg Lys Ser Leu Pro Lys Val Ser Tyr Val Thr Ala Met Asp
                325                 330                 335

Leu Phe Val Ser Val Cys Phe Ile Phe Val Phe Ser Ala Leu Val Glu
            340                 345                 350

Tyr Gly Thr Leu His Tyr Phe Val Ser Asn Arg Lys Pro Ser Lys Asp
        355                 360                 365

Lys Asp Lys Lys Lys Lys Asn Pro Leu Leu Arg Met Phe Ser Phe Lys
    370                 375                 380

Ala Pro Thr Ile Asp Ile Arg Pro Arg Ser Ala Thr Ile Gln Met Asn
385                 390                 395                 400

Asn Ala Thr His Leu Gln Glu Arg Asp Glu Glu Tyr Gly Tyr Glu Cys
                405                 410                 415

Leu Asp Gly Lys Asp Cys Ala Ser Phe Phe Cys Cys Phe Glu Asp Cys
            420                 425                 430

Arg Thr Gly Ala Trp Arg His Gly Arg Ile His Ile Arg Ile Ala Lys
        435                 440                 445

Met Asp Ser Tyr Ala Arg Ile Phe Phe Pro Thr Ala Phe Cys Leu Phe
    450                 455                 460

Asn Leu Val Tyr Trp Val Ser Tyr Leu Tyr Leu
465                 470                 475


<210> 53
<211> 1602
<212> DNA
<213> Homo sapiens

<400> 53
   atgcggctgc acagacttca cgcgcggccg agcgcggtgg cctgtgggct cctgctgctt 60
   ctgatgctgt gtgggcccga agttgctcag cctgaagtag acaccaccct gggtcgtgtg 120
   cgaggccggc aggtgggcgt gaagggcaca gaccgccttg tgaatgtctt tctgggcatt 180
   ccatttgccc agccgccact gggccctgac cggttctcag ccccacaccc agcacagccc 240
   tgggagggtg tgcgggatgc cagcactgcg cccccaatgt gcctacaaga cgtgatgaac 300
   agcagcagat ttgtcctcaa cggaaaacag cagatcttct ccgtttcaga ggactgcctg 360
   gtcctcaacg tctatagccc agctgaggtc atggtatggg tccatggagg cgctctgata 420
   actggcgctg ccacctccta cgatggatca gctctggctg cctatgggga tgtggtcgtg 480
   gttacagtcc agtaccgcct tggggtcctt ggcttcttca gcactggaga tgagcatgca 540
   cctggcaacc agggcttcct agatgtggta gctgctttgc gctgggtgca agaaaacatc 600
   gcccccttcg ggggtgacct caactgtgtc actgtctttg gtggatctgc cggtgggagc 660
   atcatctctg gcctggtcct gtccccagtg gctgcagggc tgttccacag agccatcaca 720
   cagagtgggg tcatcaccac cccagggatc atcgactctc acccttggcc cctagctcag 780
   aaaatcgcaa acaccttggc ctgcagctcc agctccccgg ctgagatggt gcagtgcctt 840
   cagcagaaag aaggagaaga gctggtcctt agcaagaagc tgaaaaatac tatctatcct 900
   ctcaccgttg atggcactgt cttccccaaa agccccaagg aactcctgaa ggagaagccc 960
   ttccactctg tgcccttcct catgggtgtc aacaaccatg agttcagctg gctcatcccc 1020
   gggaccaagg tgatgcgtgt gtccaacaag atgatcatga agttcccgct aaaccggcag 1080
   gcgatgagaa aggaaaccat cactaagatg ctctggagta cccgcaccct gttggagcat 1140
   gactggaaga tgctacgaaa ccgtatgatg gacatagttc aagatgccac tttcgtgtat 1200
   gccacactgc agactgctca ctaccaccga gatgccggcc tccctgtcta cctgtatgaa 1260
   tttgagcacc acgctcgtgg aataatcgtc aaaccccgca ctgatggggc agaccatggg 1320
   gatgagatgt acttcctctt tgggggcccc ttcgccacag gcctttccat gggtaaggag 1380
   aaggcactta gcctccagat gatgaaatac tgggccaact ttgcccgcac aggaaacccc 1440
   aatgatggga atctgccctg ctggccacgc tacaacaagg atgaaaagta cctgcagctg 1500
   gatttaccca caagagtggg catgaagctc aaggagaaga agatggcttt ttggatgagt 1560
   ctgtaccagt ctcaaagacc tgagaagcag aggcaattct aa 1602

<210> 54
<211> 533
<212> PRT
<213> Homo sapiens

<400> 54
Met Arg Leu His Arg Leu His Ala Arg Pro Ser Ala Val Ala Cys Gly
1               5                   10                  15

Leu Leu Leu Leu Leu Met Leu Cys Gly Pro Glu Val Ala Gln Pro Glu
            20                  25                  30

Val Asp Thr Thr Leu Gly Arg Val Arg Gly Arg Gln Val Gly Val Lys
        35                  40                  45

Gly Thr Asp Arg Leu Val Asn Val Phe Leu Gly Ile Pro Phe Ala Gln
    50                  55                  60

Pro Pro Leu Gly Pro Asp Arg Phe Ser Ala Pro His Pro Ala Gln Pro
65                  70                  75                  80

Trp Glu Gly Val Arg Asp Ala Ser Thr Ala Pro Pro Met Cys Leu Gln
                85                  90                  95

Asp Val Met Asn Ser Ser Arg Phe Val Leu Asn Gly Lys Gln Gln Ile
            100                 105                 110

Phe Ser Val Ser Glu Asp Cys Leu Val Leu Asn Val Tyr Ser Pro Ala
        115                 120                 125

Glu Val Met Val Trp Val His Gly Gly Ala Leu Ile Thr Gly Ala Ala
    130                 135                 140

Thr Ser Tyr Asp Gly Ser Ala Leu Ala Ala Tyr Gly Asp Val Val Val
145                 150                 155                 160

Val Thr Val Gln Tyr Arg Leu Gly Val Leu Gly Phe Phe Ser Thr Gly
                165                 170                 175

Asp Glu His Ala Pro Gly Asn Gln Gly Phe Leu Asp Val Val Ala Ala
            180                 185                 190

Leu Arg Trp Val Gln Glu Asn Ile Ala Pro Phe Gly Gly Asp Leu Asn
        195                 200                 205

Cys Val Thr Val Phe Gly Gly Ser Ala Gly Gly Ser Ile Ile Ser Gly
    210                 215                 220

Leu Val Leu Ser Pro Val Ala Ala Gly Leu Phe His Arg Ala Ile Thr
225                 230                 235                 240

Gln Ser Gly Val Ile Thr Thr Pro Gly Ile Ile Asp Ser His Pro Trp
                245                 250                 255

Pro Leu Ala Gln Lys Ile Ala Asn Thr Leu Ala Cys Ser Ser Ser Ser
            260                 265                 270

Pro Ala Glu Met Val Gln Cys Leu Gln Gln Lys Glu Gly Glu Glu Leu
        275                 280                 285

Val Leu Ser Lys Lys Leu Lys Asn Thr Ile Tyr Pro Leu Thr Val Asp
    290                 295                 300

Gly Thr Val Phe Pro Lys Ser Pro Lys Glu Leu Leu Lys Glu Lys Pro
305                 310                 315                 320

Phe His Ser Val Pro Phe Leu Met Gly Val Asn Asn His Glu Phe Ser
                325                 330                 335

Trp Leu Ile Pro Gly Thr Lys Val Met Arg Val Ser Asn Lys Met Ile
            340                 345                 350

Met Lys Phe Pro Leu Asn Arg Gln Ala Met Arg Lys Glu Thr Ile Thr
        355                 360                 365

Lys Met Leu Trp Ser Thr Arg Thr Leu Leu Glu His Asp Trp Lys Met
    370                 375                 380

Leu Arg Asn Arg Met Met Asp Ile Val Gln Asp Ala Thr Phe Val Tyr
385                 390                 395                 400

Ala Thr Leu Gln Thr Ala His Tyr His Arg Asp Ala Gly Leu Pro Val
                405                 410                 415

Tyr Leu Tyr Glu Phe Glu His His Ala Arg Gly Ile Ile Val Lys Pro
            420                 425                 430

Arg Thr Asp Gly Ala Asp His Gly Asp Glu Met Tyr Phe Leu Phe Gly
        435                 440                 445

Gly Pro Phe Ala Thr Gly Leu Ser Met Gly Lys Glu Lys Ala Leu Ser
    450                 455                 460

Leu Gln Met Met Lys Tyr Trp Ala Asn Phe Ala Arg Thr Gly Asn Pro
465                 470                 475                 480

Asn Asp Gly Asn Leu Pro Cys Trp Pro Arg Tyr Asn Lys Asp Glu Lys
                485                 490                 495

Tyr Leu Gln Leu Asp Phe Thr Thr Arg Val Gly Met Lys Leu Lys Glu
            500                 505                 510

Lys Lys Met Ala Phe Trp Met Ser Leu Tyr Gln Ser Gln Arg Pro Glu
        515                 520                 525

Lys Gln Arg Gln Phe
    530


<210> 55
<211> 996
<212> DNA
<213> Homo sapiens

<400> 55
ctctcgaatt cccccaccca cctgtactct ggagagactg tgctgggaac atgtaccact 60
gagcctgaga tggggatgag ggcagagaga ggggagcccc ctcttccact cagttgttcc 120
tactcagact gttgcactct aaacctaggg aggttgaaga atgagaccct taggttttaa 180
cacgaatcct gacaccacca tctatagggt cccaacttgg ttattgtagg caaccttccc 240
tctctccttg gtgaagaaca tcccaagcca gaaagaagtt aactacagtg ttttcctttg 300

caccgatccc caccccaatt caatcccgga agggacttac ttaggaaacc cttctttact 360
agatatcctg gccccctggg cttgtgaaca cctcctagcc acatcactac agtacagtga 420
gtgaccccag cctcctgcct accccaagat gcccctcccc accctgaccg tgctaactgt 480
gtgtacatat atattctaca tatatgtata ttaaaactgc actgccatgt ctgccctttt 540
ttgtggtgtc tagcattaac ttattgtcta ggccagagcg gggtgggag gggaatgcca 600
cagtgaaggg agtggcagaa tcaaattgct acatagtcca aacaaaaaag aaggcttttt 660
caaaaaacat taaattcaca tgcagtctca gagactattt agacaaagtt caagttagga 720
gcttttagga tgtgggagta aaactttaat gggaggggag ggctggctgc tggaagaagg 780
aagaagccag actggttaga cagtactctt aactcctagc ccagcctagc gtgccctgcc 840
cctctggcca ctgctgcaga cacctgcctt aacacacaca cctctaggac tccacagttt 900
tgccttaaag gaccttccca agtctccctt tccctgtctg gcttctccct taagaagaga 960
gagatacttg tagaattggg tgggggaatt cgagag 996

<210> 56
<211> 74
<212> PRT
<213> Homo sapiens

<400> 56
Met Tyr Ile Lys Thr Ala Leu Pro Cys Leu Pro Phe Phe Val Val Ser
1               5                   10                  15

Ser Ile Asn Leu Leu Ser Arg Pro Glu Arg Gly Trp Glu Gly Asn Ala
            20                  25                  30

Thr Val Lys Gly Val Ala Glu Ser Asn Cys Tyr Ile Val Gln Thr Lys
        35                  40                  45

Lys Lys Ala Phe Ser Lys Asn Ile Lys Phe Thr Cys Ser Leu Arg Asp
    50                  55                  60

Tyr Leu Asp Lys Val Gln Val Arg Ser Phe
65                  70

<210> 57
<211> 668
<212> DNA
<213> Homo sapiens

<400> 57
ttcgccctcc cggtcatcat cttcaccacg ttctggggcc tcgtcggcat cgccgggccc 60
tggttcgtgc cgaagggacc caaccgcgga gtgatcatca ccatgctggt cgccaccgcc 120
gtctgctgtt acctcttctg gctcatcgcc atcctggcgc agctgaaccc cctgttcggg 180
ccccagctga agaatgagac catctggtac gtgcgcttcc tgtgggagtg acccgccgcc 240
cccgacccag gtgcccagct ctcggaatga ctgtggctcc actgtccctg acaacccctt 300
cgtccggacc ctcccccaca caactatgtc tggtcaccag ctccctcctg ctggcaccca 360
gagacccgga cccgcagggc ctgcctggtt cctggaagtc ttcccagtct tcccagccag 420
cccgggccct ggggagccct gggcacagca gcggccgagg ggatgtcctg ctccaatacc 480
cgcactgctc tggagtttgc cctctttccc aaggagatgc tgctggggag ctggtatggg 540
tggggtcttt ccctttacag acggggcaga tgccaggact cagcccatcc tgaggaggac 600
acgtgtcctc atggagaggg tgctccggcc caggcggggg agtcggtgcc cagtcagcag 660
gaccaggc 668

<210> 58
<211> 76
<212> PRT
<213> Homo sapiens

<400> 58
Phe Ala Leu Pro Val Ile Ile Phe Thr Thr Phe Trp Gly Leu Val Gly
1               5                   10                  15

Ile Ala Gly Pro Trp Phe Val Pro Lys Gly Pro Asn Arg Gly Val Ile
            20                  25                  30

Ile Thr Met Leu Val Ala Thr Ala Val Cys Cys Tyr Leu Phe Trp Leu

35                  40                  45

Ile Ala Ile Leu Ala Gln Leu Asn Pro Leu Phe Gly Pro Gln Leu Lys
    50                  55                  60

Asn Glu Thr Ile Trp Tyr Val Arg Phe Leu Trp Glu
65                  70                  75

<210> 59
<211> 5587
<212> DNA
<213> Homo sapiens

<400> 59
atgggcctag gagtcatact acctacctgt tcccctcttg actttcactg tgacaatggc 60
aagtgcatcc gccgctcctg ggtgtgtgac ggggacaacg actgtgagga tgactcggat 120
gagcaggact gtcccccccg ggagtgtgag gaggacgagt ttccctgcca gaatggctac 180
tgcatccgga gtctgtggca ctgcgatggt gacaatgact gtggcgacaa cagcgatgag 240
cagtgtgaca tgcgcaagtg ctccgacaag gagttccgct gtagtgacgg aagctgcatt 300
gctgagcatt ggtactgcga cggtgacacc gactgcaaag atggctccga tgaggagaac 360
tgtccctcag cagtgccagc gcccccctgc aacctggagg agttccagtg tgcctatgga 420
cgctgcatcc tcgacatcta ccactgcgat ggcgacgatg actgtggaga ctggtcagac 480
gagtctgact gctgtgagta ctctggccag ctgggagcct cccaccagcc ctgccgctct 540
ggggagttca tgtgtgacag tggcctgtgc atcaatgcag gctggcgctg cgatggtgac 600
gcggactgtg atgaccagtc tgatgagcgc aactgcacca cctccatgtg tacggcagaa 660
cagttccgct gtcactcagg ccgctgtgtc cgcctgtcct ggcgctgtga tggggaggac 720
gactgtgcag acaacagcga tgaagagaac tgtgagaata caggaagccc ccaatgtgcc 780
ttggaccagt tcctgtgttg gaatgggcgc tgcattgggc agaggaagct gtgcaacggg 840
gtcaacgact gtggtgacaa cagcgacgaa agcccacagc agaattgccg gccccggacg 900
ggtgaggaga actgcaatgt taacaacggt ggctgtgccc agaagtgcca gatggtgcgg 960
ggggcagtgc agtgtacctg ccacacaggc taccggctca cagaggatgg gcacacgtgc 1020
caagatgtga atgaatgtgc cgaggagggg tattgcagcc agggctgcac caacagcgaa 1080
ggggctttcc aatgctggtg tgaaacaggc tatgaactac ggcccgaccg gcgcagctgc 1140
aaggctctgg ggccagagcc tgtgctgctg ttcgccaatc gcatcgacat ccggcaggtg 1200
ctgccacacc gctctgagta cacactgctg cttaacaacc tggagaatgc cattgccctt 1260
gatttccacc accgccgcga gcttgtcttc tggtcagatg tcaccctgga ccggatcctc 1320
cgtgccaacc tcaacggcag caacgtggag gaggttgtgt ctactgggct ggagagccca 1380
gggggcctgg ctgtggattg ggtccatgac aaactctact ggaccgactc aggcacctcg 1440
aggattgagg tggccaatct ggacggggcc caccggaaag tgttgctgtg gcagaacctg 1500
gagaagcccc gggccattgc cttgcatccc atggagggta ccatttactg gacagactgg 1560
ggcaacaccc cccgtattga ggcctccagc atggatggct ctggacgccg catcattgcc 1620
gatacccatc tcttctggcc caatggcctc accatcgact atgccgggcg ccgtatgtac 1680
tgggtggatg ctaagcacca tgtcatcgag agggccaatc tggatgggag tcaccgtaag 1740
gctgtcatta gccagggcct cccgcatccc ttcgccatca cagtgtttga agacagcctg 1800
tactggacag actggcacac caagagcatc aatagcgcta acaaatttac ggggaagaac 1860
caggaaatca ttcgcaacaa actccacttc cctatggaca tccacacctt gcacccccag 1920
cgccaacctg cagggaaaaa ccgctgtggg gacaacaacg gaggctgcac gcacctgtgt 1980
ctgcccagtg gccagaacta cacctgtgcc tgccccactg gcttccgcaa gatcagcagc 2040
cacgcctgtg cccagagtct tgacaagttc ctgctttttg cccgaaggat ggacatccgt 2100
cgaatcagct ttgacacaga ggacctgtct gatgatgtca tcccactggc tgacgtgcgc 2160
agtgctgtgg cccttgactg ggactcccgg gatgaccacg tgtactggac agatgtcagc 2220
actgatacca tcagcagggc caagtgggat ggaacaggac aggaggtggt agtggatacc 2280
agtttggaga gcccagctgg cctggccatt gattgggtca ccaacaaact gtactggaca 2340
gatgcaggta cagaccggat tgaagtagcc aacacagatg gcagcatgag aacagtactc 2400
atctgggaga accttgatcg tcctcgggac atcgtggtgg aacccatggg cgggtacatg 2460
tattggactg actggggtgc gagccccaag attgaacgag ctggcatgga tgcctcaggc 2520
cgccaagtca ttatctcttc taatctgacc tggcctaatg ggttagctat tgattatggg 2580
tcccagcgtc tatactgggc tgacgccggc atgaagacaa ttgaatttgc tggactggat 2640
ggcagtaaga ggaaggtgct gattggaagc cagctccccc acccatttgg gctgaccctc 2700
tatggagagc gcatctattg gactgactgg cagaccaaga gcatacagag cgctgaccgg 2760
ctgacagggc tggaccggga gactctgcag gagaacctgg aaaacctaat ggacatccat 2820
gtcttccacc gccgccggcc cccagtgtct acaccatgtg ctatggagaa tggcggctgt 2880
agccacctgt gtcttaggtc cccaaatcca agcggattca gctgtacctg ccccacaggc 2940
atcaacctgc tgtctgatgg caagacctgc tcaccaggca tgaacagttt cctcatcttc 3000
gccaggagga tagacattcg catggtctcc ctggacatcc cttattttgc tgatgtggtg 3060
gtaccaatca acattaccat gaagaacacc attgccattg gagtagaccc ccaggaagga 3120
aaggtgtact ggtctgacag cacactgcac aggatcagtc gtgccaatct ggatggctca 3180
cagcatgagg acatcatcac cacagggcta cagaccacag atgggctcgc ggttgatgcc 3240

attggccgga aagtatactg gacagacacg ggaacaaacc ggattgaagt gggcaacctg 3300
gacgggtcca tgcggaaagt gttggtgtgg cagaaccttg acagtccccg ggccatcgta 3360
ctgtaccatg agatggggtt tatgtactgg acagactggg gggagaatgc caagttagag 3420
cggtccggaa tggatggctc agaccgcgcg gtgctcatca acaacaacct aggatggccc 3480
aatggactga ctgtggacaa ggccagctcc caactgctat gggccgatgc ccacaccgag 3540
cgaattgagg ctgctgacct gaatggtgcc aatcggcata cattggtgtc accggtgcag 3600
cacccatatg gcctcaccct gctcgactcc tatatctact ggactgactg gcagactcgg 3660
agcatccacc gtgctgacaa gggtactggc agcaatgtca tcctcgtgag gtccaacctg 3720
ccaggcctca tggacatgca ggctgtggac cgggcacagc cactaggttt taacaagtgc 3780
ggctcgagaa atggcggctg ctcccacctc tgcttgcctc ggccttctgg cttctcctgt 3840
gcctgcccca ctggcatcca gctgaaggga gatgggaaga cctgtgatcc ctctcctgag 3900
acctacctgc tcttctccag ccgtggctcc atccggcgta tctcactgga caccagtgac 3960
cacaccaatg tgcatgtccc tgttcctgag ctcaacaatg tcatctccct ggactatgac 4020
agcgtggatg gaaaggtcta ttacacagat gtgttcctgg atgttatcag gcgagcagac 4080
ctgaacggca gcaacatgga gacagtgatc gggcgagggc tgaagaccac tgacgggctg 4140
gcagtggact gggtggccag gaacctgtac tggacagaca caggtcgaaa taccattgag 4200
gcgtccaggc tggatggttc ctgccgcaaa gtactgatca acaatagcct ggatgagccc 4260
cgggccattg ctgtttcccc caggaagggg tacctcttct ggacagactg gggccacatt 4320
gccaagatcg aacgggcaaa cttggatggt tctgagcgga aggtcctcat caacacagac 4380
ctgggttggc ccaatggcct taccctggac tatgataccc gcaggatcta ctgggtggat 4440
gcgcatctgg accggatcga gagtgctgac ctcaatggga aactgcggca ggtcttggtc 4500
agccatgtgt cccacccctt tgccctcaca cagcaagaca ggtggatcta ctggacagac 4560
tggcagacca agtcaatcca gcgtgttgac aaatactcag gccggaacaa ggagacagtg 4620
ctggcaaatg tggaaggact catggatatc atcgtggttt cccctcagcg gcagacaggg 4680
accaatgcct gtggtgtgaa caatggtggc tgcacccacc tctgctttgc cagagcctcg 4740
gacttcgtat gtgcctgtcc tgacgaacct gatagccagc cctgctccct tgtgcctggc 4800
ctggtaccac cagctcctag ggctactggc atgagtgaaa agagcccagt gctacccaac 4860
acaccaccta ccaccttgta ttcttcaacc acccggaccc gcacgtctct ggaggaggtg 4920
gaaggaagat gctctgaaag ggatgccagg ctgggcctct gtgcacgttc caatgacgct 4980
gttcctgctg ctccagggga aggacttcat atcagctacg ccattggtgg actcctcagt 5040
attctgctga ttttggtggt gattgcagct ttgatgctgt acagacacaa aaaatccaag 5100
ttcactgatc ctggaatggg gaacctcacc tacagcaacc cctcctaccg aacatccaca 5160
caggaagtga agattgaagc aatccccaaa ccagccatgt acaaccagct gtgctataag 5220
aaagagggag ggcctgacca taactacacc aaggagaaga tcaagatcgt agagggaatc 5280
tgcctcctgt ctggggatga tgctgagtgg gatgacctca agcaactgcg aagctcacgg 5340
gggggcctcc tccgggatca tgtatgcatg aagacagaca cggtgtccat ccaggccagc 5400
tctggctccc tggatgacac agagacggag cagctgttac aggaagagca gtctgagtgt 5460
agcagcgtcc atactgcagc cactccagaa agacgaggct ctctgccaga cacgggctgg 5520
aaacatgaac gcaagctctc ctcagagagc caggtctaaa tgcccacatt ctcttccctg 5580
cctgcct 5587

<210> 60
<211> 1852
<212> PRT
<213> Homo sapiens

<400> 60
Met Gly Leu Gly Val Ile Leu Pro Thr Cys Ser Pro Leu Asp Phe His
1               5                   10                  15

Cys Asp Asn Gly Lys Cys Ile Arg Arg Ser Trp Val Cys Asp Gly Asp
            20                  25                  30

Asn Asp Cys Glu Asp Asp Ser Asp Glu Gln Asp Cys Pro Pro Arg Glu
        35                  40                  45

Cys Glu Glu Asp Glu Phe Pro Cys Gln Asn Gly Tyr Cys Ile Arg Ser
    50                  55                  60

Leu Trp His Cys Asp Gly Asp Asn Asp Cys Gly Asp Asn Ser Asp Glu
65                  70                  75                  80

Gln Cys Asp Met Arg Lys Cys Ser Asp Lys Glu Phe Arg Cys Ser Asp
                85                  90                  95

Gly Ser Cys Ile Ala Glu His Trp Tyr Cys Asp Gly Asp Thr Asp Cys
            100                 105                 110

Lys Asp Gly Ser Asp Glu Glu Asn Cys Pro Ser Ala Val Pro Ala Pro
        115                 120                 125

Pro Cys Asn Leu Glu Glu Phe Gln Cys Ala Tyr Gly Arg Cys Ile Leu
    130                 135                 140

Asp Ile Tyr His Cys Asp Gly Asp Asp Asp Cys Gly Asp Trp Ser Asp
145                 150                 155                 160

Glu Ser Asp Cys Cys Glu Tyr Ser Gly Gln Leu Gly Ala Ser His Gln
                165                 170                 175

Pro Cys Arg Ser Gly Glu Phe Met Cys Asp Ser Gly Leu Cys Ile Asn
            180                 185                 190

Ala Gly Trp Arg Cys Asp Gly Asp Ala Asp Cys Asp Asp Gln Ser Asp
        195                 200                 205

Glu Arg Asn Cys Thr Thr Ser Met Cys Thr Ala Glu Gln Phe Arg Cys
    210                 215                 220

His Ser Gly Arg Cys Val Arg Leu Ser Trp Arg Cys Asp Gly Glu Asp
225                 230                 235                 240

Asp Cys Ala Asp Asn Ser Asp Glu Glu Asn Cys Glu Asn Thr Gly Ser
                245                 250                 255

Pro Gln Cys Ala Leu Asp Gln Phe Leu Cys Trp Asn Gly Arg Cys Ile
            260                 265                 270

Gly Gln Arg Lys Leu Cys Asn Gly Val Asn Asp Cys Gly Asp Asn Ser
        275                 280                 285

Asp Glu Ser Pro Gln Gln Asn Cys Arg Pro Arg Thr Gly Glu Glu Asn
    290                 295                 300

Cys Asn Val Asn Asn Gly Gly Cys Ala Gln Lys Cys Gln Met Val Arg
305                 310                 315                 320

Gly Ala Val Gln Cys Thr Cys His Thr Gly Tyr Arg Leu Thr Glu Asp
                325                 330                 335

Gly His Thr Cys Gln Asp Val Asn Glu Cys Ala Glu Glu Gly Tyr Cys
            340                 345                 350

Ser Gln Gly Cys Thr Asn Ser Glu Gly Ala Phe Gln Cys Trp Cys Glu
        355                 360                 365

Thr Gly Tyr Glu Leu Arg Pro Asp Arg Arg Ser Cys Lys Ala Leu Gly
    370                 375                 380

Pro Glu Pro Val Leu Leu Phe Ala Asn Arg Ile Asp Ile Arg Gln Val
385                 390                 395                 400

Leu Pro His Arg Ser Glu Tyr Thr Leu Leu Leu Asn Asn Leu Glu Asn
                405                 410                 415

Ala Ile Ala Leu Asp Phe His His Arg Arg Glu Leu Val Phe Trp Ser
            420                 425                 430

Asp Val Thr Leu Asp Arg Ile Leu Arg Ala Asn Leu Asn Gly Ser Asn
        435                 440                 445

Val Glu Glu Val Val Ser Thr Gly Leu Glu Ser Pro Gly Gly Leu Ala
    450                 455                 460

Val Asp Trp Val His Asp Lys Leu Tyr Trp Thr Asp Ser Gly Thr Ser
465                 470                 475                 480

Arg Ile Glu Val Ala Asn Leu Asp Gly Ala His Arg Lys Val Leu Leu
                485                 490                 495

Trp Gln Asn Leu Glu Lys Pro Arg Ala Ile Ala Leu His Pro Met Glu
            500                 505                 510

Gly Thr Ile Tyr Trp Thr Asp Trp Gly Asn Thr Pro Arg Ile Glu Ala
        515                 520                 525

Ser Ser Met Asp Gly Ser Gly Arg Arg Ile Ile Ala Asp Thr His Leu
    530                 535                 540

Phe Trp Pro Asn Gly Leu Thr Ile Asp Tyr Ala Gly Arg Arg Met Tyr
545                 550                 555                 560

Trp Val Asp Ala Lys His His Val Ile Glu Arg Ala Asn Leu Asp Gly
                565                 570                 575

Ser His Arg Lys Ala Val Ile Ser Gln Gly Leu Pro His Pro Phe Ala
            580                 585                 590

Ile Thr Val Phe Glu Asp Ser Leu Tyr Trp Thr Asp Trp His Thr Lys
        595                 600                 605

Ser Ile Asn Ser Ala Asn Lys Phe Thr Gly Lys Asn Gln Glu Ile Ile
    610                 615                 620

Arg Asn Lys Leu His Phe Pro Met Asp Ile His Thr Leu His Pro Gln
625                 630                 635                 640

Arg Gln Pro Ala Gly Lys Asn Arg Cys Gly Asp Asn Asn Gly Gly Cys
                645                 650                 655

Thr His Leu Cys Leu Pro Ser Gly Gln Asn Tyr Thr Cys Ala Cys Pro
            660                 665                 670

Thr Gly Phe Arg Lys Ile Ser Ser His Ala Cys Ala Gln Ser Leu Asp
        675                 680                 685

Lys Phe Leu Leu Phe Ala Arg Arg Met Asp Ile Arg Arg Ile Ser Phe
    690                 695                 700

Asp Thr Glu Asp Leu Ser Asp Asp Val Ile Pro Leu Ala Asp Val Arg
705                 710                 715                 720

Ser Ala Val Ala Leu Asp Trp Asp Ser Arg Asp Asp His Val Tyr Trp
                725                 730                 735

Thr Asp Val Ser Thr Asp Thr Ile Ser Arg Ala Lys Trp Asp Gly Thr
            740                 745                 750

Gly Gln Glu Val Val Val Asp Thr Ser Leu Glu Ser Pro Ala Gly Leu
        755                 760                 765

Ala Ile Asp Trp Val Thr Asn Lys Leu Tyr Trp Thr Asp Ala Gly Thr
    770                 775                 780

Asp Arg Ile Glu Val Ala Asn Thr Asp Gly Ser Met Arg Thr Val Leu
785                 790                 795                 800

Ile Trp Glu Asn Leu Asp Arg Pro Arg Asp Ile Val Val Glu Pro Met
                805                 810                 815

Gly Gly Tyr Met Tyr Trp Thr Asp Trp Gly Ala Ser Pro Lys Ile Glu
            820                 825                 830

Arg Ala Gly Met Asp Ala Ser Gly Arg Gln Val Ile Ile Ser Ser Asn
        835                 840                 845

Leu Thr Trp Pro Asn Gly Leu Ala Ile Asp Tyr Gly Ser Gln Arg Leu
    850                 855                 860

Tyr Trp Ala Asp Ala Gly Met Lys Thr Ile Glu Phe Ala Gly Leu Asp
865                 870                 875                 880

Gly Ser Lys Arg Lys Val Leu Ile Gly Ser Gln Leu Pro His Pro Phe
                885                 890                 895

Gly Leu Thr Leu Tyr Gly Glu Arg Ile Tyr Trp Thr Asp Trp Gln Thr
            900                 905                 910

Lys Ser Ile Gln Ser Ala Asp Arg Leu Thr Gly Leu Asp Arg Glu Thr
        915                 920                 925

Leu Gln Glu Asn Leu Glu Asn Leu Met Asp Ile His Val Phe His Arg
    930                 935                 940

Arg Arg Pro Pro Val Ser Thr Pro Cys Ala Met Glu Asn Gly Gly Cys
945                 950                 955                 960

Ser His Leu Cys Leu Arg Ser Pro Asn Pro Ser Gly Phe Ser Cys Thr
                965                 970                 975

Cys Pro Thr Gly Ile Asn Leu Leu Ser Asp Gly Lys Thr Cys Ser Pro
            980                 985                 990

Gly Met Asn Ser Phe Leu Ile Phe Ala Arg Arg Ile Asp Ile Arg Met
        995                 1000                1005

Val Ser Leu Asp Ile Pro Tyr Phe Ala Asp Val Val Val Pro Ile Asn
    1010                1015                1020

Ile Thr Met Lys Asn Thr Ile Ala Ile Gly Val Asp Pro Gln Glu Gly
1025                1030                1035                1040

Lys Val Tyr Trp Ser Asp Ser Thr Leu His Arg Ile Ser Arg Ala Asn
                1045                1050                1055

Leu Asp Gly Ser Gln His Glu Asp Ile Ile Thr Thr Gly Leu Gln Thr
            1060                1065                1070

Thr Asp Gly Leu Ala Val Asp Ala Ile Gly Arg Lys Val Tyr Trp Thr
        1075                1080                1085

Asp Thr Gly Thr Asn Arg Ile Glu Val Gly Asn Leu Asp Gly Ser Met
    1090                1095                1100

Arg Lys Val Leu Val Trp Gln Asn Leu Asp Ser Pro Arg Ala Ile Val
1105                1110                1115                1120

Leu Tyr His Glu Met Gly Phe Met Tyr Trp Thr Asp Trp Gly Glu Asn
                1125                1130                1135

Ala Lys Leu Glu Arg Ser Gly Met Asp Gly Ser Asp Arg Ala Val Leu
            1140                1145                1150

Ile Asn Asn Asn Leu Gly Trp Pro Asn Gly Leu Thr Val Asp Lys Ala
        1155                1160                1165

Ser Ser Gln Leu Leu Trp Ala Asp Ala His Thr Glu Arg Ile Glu Ala
    1170                1175                1180

Ala Asp Leu Asn Gly Ala Asn Arg His Thr Leu Val Ser Pro Val Gln
1185                1190                1195                1200

His Pro Tyr Gly Leu Thr Leu Leu Asp Ser Tyr Ile Tyr Trp Thr Asp
                1205                1210                1215

Trp Gln Thr Arg Ser Ile His Arg Ala Asp Lys Gly Thr Gly Ser Asn
            1220                1225                1230

Val Ile Leu Val Arg Ser Asn Leu Pro Gly Leu Met Asp Met Gln Ala
        1235                1240                1245

Val Asp Arg Ala Gln Pro Leu Gly Phe Asn Lys Cys Gly Ser Arg Asn
    1250                1255                1260

Gly Gly Cys Ser His Leu Cys Leu Pro Arg Pro Ser Gly Phe Ser Cys
1265                1270                1275                1280

Ala Cys Pro Thr Gly Ile Gln Leu Lys Gly Asp Gly Lys Thr Cys Asp
                1285                1290                1295

Pro Ser Pro Glu Thr Tyr Leu Leu Phe Ser Ser Arg Gly Ser Ile Arg
            1300                1305                1310

Arg Ile Ser Leu Asp Thr Ser Asp His Thr Asn Val His Val Pro Val
        1315                1320                1325

Pro Glu Leu Asn Asn Val Ile Ser Leu Asp Tyr Asp Ser Val Asp Gly
    1330                1335                1340

Lys Val Tyr Tyr Thr Asp Val Phe Leu Asp Val Ile Arg Arg Ala Asp
1345                1350                1355                1360

Leu Asn Gly Ser Asn Met Glu Thr Val Ile Gly Arg Gly Leu Lys Thr
                1365                1370                1375

Thr Asp Gly Leu Ala Val Asp Trp Val Ala Arg Asn Leu Tyr Trp Thr
            1380                1385                1390

Asp Thr Gly Arg Asn Thr Ile Glu Ala Ser Arg Leu Asp Gly Ser Cys
        1395                1400                1405

Arg Lys Val Leu Ile Asn Asn Ser Leu Asp Glu Pro Arg Ala Ile Ala
    1410                1415                1420

Val Phe Pro Arg Lys Gly Tyr Leu Phe Trp Thr Asp Trp Gly His Ile
1425                1430                1435                1440

Ala Lys Ile Glu Arg Ala Asn Leu Asp Gly Ser Glu Arg Lys Val Leu
                1445                1450                1455

Ile Asn Thr Asp Leu Gly Trp Pro Asn Gly Leu Thr Leu Asp Tyr Asp
            1460                1465                1470

Thr Arg Arg Ile Tyr Trp Val Asp Ala His Leu Asp Arg Ile Glu Ser
        1475                1480                1485

Ala Asp Leu Asn Gly Lys Leu Arg Gln Val Leu Val Ser His Val Ser
    1490                1495                1500

His Pro Phe Ala Leu Thr Gln Gln Asp Arg Trp Ile Tyr Trp Thr Asp
1505                1510                1515                1520

Trp Gln Thr Lys Ser Ile Gln Arg Val Asp Lys Tyr Ser Gly Arg Asn
                1525                1530                1535

Lys Glu Thr Val Leu Ala Asn Val Glu Gly Leu Met Asp Ile Ile Val
            1540                1545                1550

Val Ser Pro Gln Arg Gln Thr Gly Thr Asn Ala Cys Gly Val Asn Asn
        1555                1560                1565

Gly Gly Cys Thr His Leu Cys Phe Ala Arg Ala Ser Asp Phe Val Cys
    1570                1575                1580

Ala Cys Pro Asp Glu Pro Asp Ser Gln Pro Cys Ser Leu Val Pro Gly
1585                1590                1595                1600

Leu Val Pro Pro Ala Pro Arg Ala Thr Gly Met Ser Glu Lys Ser Pro
                1605                1610                1615

Val Leu Pro Asn Thr Pro Pro Thr Thr Leu Tyr Ser Ser Thr Thr Arg
            1620                1625                1630

Thr Arg Thr Ser Leu Glu Glu Val Glu Gly Arg Cys Ser Glu Arg Asp
        1635                1640                1645

Ala Arg Leu Gly Leu Cys Ala Arg Ser Asn Asp Ala Val Pro Ala Ala
    1650                1655                1660

Pro Gly Glu Gly Leu His Ile Ser Tyr Ala Ile Gly Gly Leu Leu Ser
1665                1670                1675                1680

Ile Leu Leu Ile Leu Val Val Ile Ala Ala Leu Met Leu Tyr Arg His
                1685                1690                1695

Lys Lys Ser Lys Phe Thr Asp Pro Gly Met Gly Asn Leu Thr Tyr Ser
            1700                1705                1710

Asn Pro Ser Tyr Arg Thr Ser Thr Gln Glu Val Lys Ile Glu Ala Ile
        1715                1720                1725

Pro Lys Pro Ala Met Tyr Asn Gln Leu Cys Tyr Lys Lys Glu Gly Gly
    1730                1735                1740

Pro Asp His Asn Tyr Thr Lys Glu Lys Ile Lys Ile Val Glu Gly Ile
1745                1750                1755                1760

Cys Leu Leu Ser Gly Asp Asp Ala Glu Trp Asp Asp Leu Lys Gln Leu
                1765                1770                1775

Arg Ser Ser Arg Gly Gly Leu Leu Arg Asp His Val Cys Met Lys Thr
            1780                1785                1790

Asp Thr Val Ser Ile Gln Ala Ser Ser Gly Ser Leu Asp Asp Thr Glu
        1795                1800                1805

Thr Glu Gln Leu Leu Gln Glu Glu Gln Ser Glu Cys Ser Ser Val His
    1810                1815                1820

Thr Ala Ala Thr Pro Glu Arg Arg Gly Ser Leu Pro Asp Thr Gly Trp
1825                1830                1835                1840

Lys His Glu Arg Lys Leu Ser Ser Glu Ser Gln Val
                1845                1850


<210> 61
<211> 635
<212> DNA
<213> Homo sapiens

<400> 61
ccccactatg gtgacgctcg ctgagctgct gttgctccag aacactctcc tgaccatggt 60
cttgggctat ttcatcagca tccacgcaca tgctgaagaa tgcttaagtg agcatgtcac 120
ctcaggcacc aagatgggcc tcatcttcga aggtggcttc ctgggcatca acatggagat 180
tacaggacct aagaataaaa ggatttataa aggagacaaa gaatccagtg ggaaatacac 240
attttctgct cacatggatg gaacaaatac attttgtttt agtgaccgag tgtccaccat 300
gactccaaag atagtgatat tcaccattga tattggggag gctacaaaaa gagaagacat 360
ggaaacagaa gctcaccaga acaaactaga agaaatgatc agtgagctgg ctgtggccat 420
gacagctgta cagcacaaag aggaatacac gaaaatctgg gagaggatac acagagccat 480
tagtgacaac acaaacagcc cagtggtcct tcggtgcttc tttgaagctc ttgttctaat 540
tgccatgaca ttgggacaca tctactacct gaagagattt tttgaagtcc agagggttgt 600
ttcaaaagcc tcttcctgat gattccaaac tcata                            635

<210> 62
<211> 203
<212> PRT
<213> Homo sapiens

<400> 62
Met Val Thr Leu Ala Glu Leu Leu Leu Leu Gln Asn Thr Leu Leu Thr
1               5                   10                  15

Met Val Leu Gly Tyr Phe Ile Ser Ile His Ala His Ala Glu Glu Cys
            20                  25                  30

Leu Ser Glu His Val Thr Ser Gly Thr Lys Met Gly Leu Ile Phe Glu
        35                  40                  45

Gly Gly Phe Leu Gly Ile Asn Met Glu Ile Thr Gly Pro Lys Asn Lys
    50                  55                  60

Arg Ile Tyr Lys Gly Asp Lys Glu Ser Ser Gly Lys Tyr Thr Phe Ser
65                  70                  75                  80

Ala His Met Asp Gly Thr Asn Thr Phe Cys Phe Ser Asp Arg Val Ser
                85                  90                  95

Thr Met Thr Pro Lys Ile Val Ile Phe Thr Ile Asp Ile Gly Glu Ala
            100                 105                 110

Thr Lys Arg Glu Asp Met Glu Thr Glu Ala His Gln Asn Lys Leu Glu
        115                 120                 125

Glu Met Ile Ser Glu Leu Ala Val Ala Met Thr Ala Val Gln His Lys
    130                 135                 140

Glu Glu Tyr Thr Lys Ile Trp Glu Arg Ile His Arg Ala Ile Ser Asp
145                 150                 155                 160

Asn Thr Asn Ser Pro Val Val Leu Arg Cys Phe Phe Glu Ala Leu Val
                165                 170                 175

Leu Ile Ala Met Thr Leu Gly His Ile Tyr Tyr Leu Lys Arg Phe Phe
            180                 185                 190

Glu Val Gln Arg Val Val Ser Lys Ala Ser Ser
        195                 200


<210> 63
<211> 1120
<212> DNA
<213> Homo sapiens

<400> 63
   gaggccatgc ccgcttcctc tcttccagga aagctctggt tcgtcctcac gatgctgctg 60
   cggatgctgg tgattgtctt ggcggggcga cccgtctacc aggacgagca ggagaggttt 120
   gtctgcaaca cgctgcagcc gggatgcgcc aatgtttgct acgacgtctt ctcccccgtg 180
   tctcacctgc ggttctggct gatccagggc gtgtgcgtcc tcctcccctc cgccgtcttc 240
   agcgtctatg tcctgcaccg aggagccacg ctcgccgcgc tgggcccccg ccgctgcccc 300
   gacccccggg agccggcctc cgggcagaga cgctgcccgc ggccattcgg ggagcgcggc 360
   ggcctccagg tgcccgactt ttcggccggc tacatcatcc acctcctcct ccggaccctg 420
   ctggaggcag ccttcggggc cttgcactac tttctctttg gattcctggc cccgaagaag 480
   ttcccttgca cgcgccctcc gtgcacgggc gtggtggact gctacgtgtc gcggcccaca 540
   gagaagtccc tgctgatgct gttcctctgg gcggtcagcg cgctgtcttt tctgctgggc 600
   ctcgccgacc tggtctgcag cctgcggcgg cggatgcgca ggaggccggg accccccaca 660
   agcccctcca tccggaagca gagcggagcc tcaggccacg cggagggacg ccggactgac 720
   gaggagggtg ggcgggagga agagggggca ccggcgcccc cgggtgcacg cgccggaggg 780
   gagggggctg gcagccccag gcgtacatcc agggtgtcag ggcacacgaa gattccggat 840
   gaggatgaga gtgaggtgac atcctccgcc agcgaaaagc tgggcagaca gccccggggc 900
   aggccccacc gagaggccgc ccaggacccc aggggctcag gatccgagga gcagccctca 960
   gcagccccca gccgcctggc cgcgccccct tcctgcagca gcctgcagcc ccctgacccg 1020
   cctgccagct ccagtggtgc tccccacctg agagccagga agtctgagtg ggtgtgaaaa 1080
   aaacagcacc tggcggtgcc ccggggctca cgcctgtaat 1120

<210> 64
<211> 356
<212> PRT
<213> Homo sapiens

<400> 64
Met Pro Ala Ser Ser Leu Pro Gly Lys Leu Trp Phe Val Leu Thr Met
1               5                   10                  15

Leu Leu Arg Met Leu Val Ile Val Leu Ala Gly Arg Pro Val Tyr Gln
            20                  25                  30

Asp Glu Gln Glu Arg Phe Val Cys Asn Thr Leu Gln Pro Gly Cys Ala
        35                  40                  45

Asn Val Cys Tyr Asp Val Phe Ser Pro Val Ser His Leu Arg Phe Trp
    50                  55                  60

Leu Ile Gln Gly Val Cys Val Leu Leu Pro Ser Ala Val Phe Ser Val
65                  70                  75                  80

Tyr Val Leu His Arg Gly Ala Thr Leu Ala Ala Leu Gly Pro Arg Arg
                85                  90                  95

Cys Pro Asp Pro Arg Glu Pro Ala Ser Gly Gln Arg Arg Cys Pro Arg
            100                 105                 110

Pro Phe Gly Glu Arg Gly Gly Leu Gln Val Pro Asp Phe Ser Ala Gly
        115                 120                 125

Tyr Ile Ile His Leu Leu Leu Arg Thr Leu Leu Glu Ala Ala Phe Gly
    130                 135                 140

Ala Leu His Tyr Phe Leu Phe Gly Phe Leu Ala Pro Lys Lys Phe Pro
145                 150                 155                 160

Cys Thr Arg Pro Pro Cys Thr Gly Val Val Asp Cys Tyr Val Ser Arg
                165                 170                 175

Pro Thr Glu Lys Ser Leu Leu Met Leu Phe Leu Trp Ala Val Ser Ala
            180                 185                 190

Leu Ser Phe Leu Leu Gly Leu Ala Asp Leu Val Cys Ser Leu Arg Arg
        195                 200                 205

Arg Met Arg Arg Arg Pro Gly Pro Pro Thr Ser Pro Ser Ile Arg Lys
    210                 215                 220

Gln Ser Gly Ala Ser Gly His Ala Glu Gly Arg Arg Thr Asp Glu Glu
225                 230                 235                 240

Gly Gly Arg Glu Glu Glu Gly Ala Pro Ala Pro Pro Gly Ala Arg Ala
                245                 250                 255

Gly Gly Glu Gly Ala Gly Ser Pro Arg Arg Thr Ser Arg Val Ser Gly
            260                 265                 270

His Thr Lys Ile Pro Asp Glu Asp Glu Ser Glu Val Thr Ser Ser Ala
        275                 280                 285

Ser Glu Lys Leu Gly Arg Gln Pro Arg Gly Arg Pro His Arg Glu Ala
    290                 295                 300

Ala Gln Asp Pro Arg Gly Ser Gly Ser Glu Glu Gln Pro Ser Ala Ala
305                 310                 315                 320

Pro Ser Arg Leu Ala Ala Pro Pro Ser Cys Ser Ser Leu Gln Pro Pro
                325                 330                 335

Asp Pro Pro Ala Ser Ser Ser Gly Ala Pro His Leu Arg Ala Arg Lys
            340                 345                 350

Ser Glu Trp Val
        355

<210> 65
<211> 1234
<212> DNA
<213> Homo sapiens

<400> 65
taataatctt tttttaaaac tccctaacag gatgtgtggc aggttcctga ggtggtggct 60
gctggcggag gagagctggc actccacccc cgtggggcgc ctcctgtttc ccgtgctcct 120
gggattccgc cttgtgctgc tggctgccag tgggcctgga gtctatggcg atgagcagag 180
tgaattcgtg tgtcacaccc agcagccggg ctgcaaggct gcctgcttcg atgccttcca 240
cccgctctcc ccgctgcgtt tctgggtctt ccaggtcatc ttggtggctg tacctagcgt 300
cctctacatg ggtttcactc tgtatcacgt gatctggcac tgggaagaat caagaaaggg 360
gacggaggaa gaggacaccc tgatccaggg aggggagagc agcagagata ccccaggggc 420
tggaagcctc aggctgctcc gagcttatgt ggctcagctg ggagctcagc tggtcctgga 480
ggggacagcg ccggggttgc agtaccacct gtatgggttc cagatgccca gctcctttgc 540
atgtggccaa gagccttgcc cgtatagatt aacttgcacc ttttcccacc cctcggagaa 600
gatcatcttt ctaaaagcca tgtttggggt cagtgggttc cgtctcttgt tcactctttt 660
ggagattgtg cttctgggtc tgggaagact gtgtaagccc ctgcggaact tcctgggtgg 720
ggcctcttcc tccagccacg ccctggccct gagcagcaaa aggaacctcc agcagacact 780
gggagccatc catcggcctg gtcagccttg ttccatttca gagaccatgt tccccacagc 840
cccagtgact aggggtgaca tctcccgacc tcccccacct gtggatatgg ccaagtcgag 900
gtaccggtta accaaagatg ctgaaggagt gaagaaccag ccatccccta atacgcagga 960
tggttatatt gattatgtca aactgaaaac tttggagaaa ctcctctctc agaaagcgat 1020
aactgggcca gacacggtgg ctcatgcctg taatcccagc attttgggag gcctaggcag 1080
gtggatcact ggaggtcagg agttcaagac cagccaggcc aacatggtga aacccgtgtc 1140
tactaaaact acaaaaattc tgggcatggt ggtgggcgtc tgtaatccca gctacttgag 1200
aggctgaggc aggagaattg cttgaacctg ggag 1234

<210> 66
<211> 391
<212> PRT
<213> Homo sapiens

<400> 66

<210> 67
<211> 1400
<212> DNA
<213> Homo sapiens

<400> 67
attctcccca aacgccaggg atgggggtca tggctccccg aaccctcctc ctgctgctct 60
tgggggccct ggccctgacc gagacctggg ccggtgagtg cggggtcggg agggaaaggg 120
cctctgcggg gagaagcgag tggcccgccc ggcccgggga gccgcgcctc agcctctcct 180
cgcctccagg ctcccactcc ttgaggtatt tcagcaccgc agtgtcccag cccggccgcg 240
gggagccccg gttcatcgcc gtgggctacg tggacgacac agagttcgtg cggttcgaca 300
gcgactccgt gagtccgagg atggagcggc gggcgccgtg ggtggagcag gaggggctgg 360
agtattggga ccaggagaca cggaacgcca agggccacgc gcagatttac cgagtgaacc 420
tgcggaccct gctccgctat tacaaccaga gcgaggccgg tggttctcac accatccaga 480

ggaagcatga ctgcgacgtg ggcccgacag gcgggcccga caggcgcctc ctccgcaggt 540
atgaacagtt cgcctacgat ggcaaggatt acatcgccct gaacgaggac ctgccctcct 600
ggaccgccgc gaacacagcg gctcagatct cccagcacaa gtgggaagcg gacaaatact 660
cagagcaggt cagggcctac ctgagggcaa gtgcatggag tggcgagggc aagtgcatgg 720
agtggctccg cagacacctg gagaacggga aggagacgct gcagcgcgcg tcagatcccc 780
caaaggcaca tgtgacccag caccccgtct ctgaccatga ggccaccctt gaggtgctgg 840
gccctgggcc tctacccttg aggtgctggg ccctgggcct ctaccctgcg gagatcacac 900
tgacctggca gcaggatggg gaggaccaga cccaggacac ggagcttgtg gagaccaggc 960
ctgcagggga cggaaccttc cagaagtggg tggctgtagt ggtgccttcc ggagaggagc 1020
agagatacat gtgccatgtg cagcatgagg ggctgccaga gcccctcacc ctgagatggc 1080
cctcacctcc ctctcctttc ccagagccgt cttctcagcc caccatcccc atcgtgggca 1140
tcgttgctgg cctgtttctc cttggagctg tggtcactgg agctgtggtt gctgctgtga 1200
tgaagaggaa gaaaagctca ggtagggaag ggtgagaggg tgggatctgg gttttcttgt 1260
tccactgtgg gtttcaagcc acaggtagaa ttgtgacttg cttcatcact gggaagcacc 1320
gtccacacac aggccgacct agcctggggc cctgtgtgcc aacacttgct cttttgtgaa 1380
gcacatgtga aaacgaagga 1400

<210> 68
<211> 452
<212> PRT
<213> Homo sapiens

<400> 68

<210> 69
<211> 1225
<212> DNA
<213> Homo sapiens

<400> 69
acgccgagga tggggtcatg gcgtcccaaa ccctcctcct gctgctcttg ggggccctgg 60
ccctgaccga gacctgggcg ggtacccact ccataaggta tttcagcacc gccgtgtccc 120
ggccgggtcg cggggagccc cggggtaccc actccataag gtatttcagc accgccgtgt 180
cccggccggg tcgcggggag ccccggtaca tcgcagtggg ctacgtggac gacacgcagt 240
tcgtgcggtt cgacagcgac gcggcgactc cgaggatgga gccgcaggcg ccgtggttgg 300
agcaggaggg accggagtat tgggaccgga gcacaccgaa catcaggccc gcgcacagac 360
tgacaagagt gaacctgccc atgccgcgcc gctactacca ccagagcggg tctaacaccc 420
tccagataat gtatggctgc gacttggggc tggaagggcg cctcctccgc gggtatgaac 480
agcacgccaa cgatggcaaa gattacatcg ccctaaacga ggacctgagc tcttggaccg 540
cggcggccat ggcggctcag attacccagc gcaagtggga ggcggcccat gaggcggagc 600
agcagagagc ctacctggag ggcacgtgcg tggagtggct ccgcagatac ctggagaacg 660
ggaaggagac gctgcagcgc actacccccc cccccaagac acatatgatc caccattccg 720
tctctgacta taaggccacc ctgagatgct gggccctggg cttctaccct gtggagatca 780
cactgacctg gcagcaggat ggagaggacc agactcagga catggagctt gtagagacca 840
ggcctgcagg ggatggaaac ttccagaagt gggcagctgt ggtggtgcct tctggagagg 900
aacagagata catgtgccat gtgcagcatg aggggttgcc caagcccctc accctgagat 960
gggagcagtc ttctcagccc accatcccca tcgtgggtat cgttgctggc ctggttctcc 1020
ttggagctgt agtcactgga gctgtggttt ctgctgtgat gtgcaggaag aactcatttt 1080
gttctacccc aggcagcaac catgcgcagg gttctgatgt gtctctcacg gcttgtaaag 1140
gtgagacgct gggggacctg atgtgtgggg ggtgttgggg gcaatagtgg atgcagctgt 1200

gctatggggt ttctttgaat tggat                                          1225

<210> 70
<211> 389
<212> PRT
<213> Homo sapiens

<400> 70
Met Ala Ser Gln Thr Leu Leu Leu Leu Leu Leu Gly Ala Leu Ala Leu
1               5                   10                  15

Thr Glu Thr Trp Ala Gly Thr His Ser Ile Arg Tyr Phe Ser Thr Ala
            20                  25                  30

Val Ser Arg Pro Gly Arg Gly Glu Pro Arg Gly Thr His Ser Ile Arg
        35                  40                  45

Tyr Phe Ser Thr Ala Val Ser Arg Pro Gly Arg Gly Glu Pro Arg Tyr
    50                  55                  60

Ile Ala Val Gly Tyr Val Asp Asp Thr Gln Phe Val Arg Phe Asp Ser
65                  70                  75                  80

Asp Ala Ala Thr Pro Arg Met Glu Pro Gln Ala Pro Trp Leu Glu Gln
                85                  90                  95

Glu Gly Pro Glu Tyr Trp Asp Arg Ser Thr Pro Asn Ile Arg Pro Ala
            100                 105                 110

His Arg Leu Thr Arg Val Asn Leu Pro Met Pro Arg Arg Tyr Tyr His
        115                 120                 125

Gln Ser Gly Ser Asn Thr Leu Gln Ile Met Tyr Gly Cys Asp Leu Gly
    130                 135                 140

Leu Glu Gly Arg Leu Leu Arg Gly Tyr Glu Gln His Ala Asn Asp Gly
145                 150                 155                 160

Lys Asp Tyr Ile Ala Leu Asn Glu Asp Leu Ser Ser Trp Thr Ala Ala
                165                 170                 175

Ala Met Ala Ala Gln Ile Thr Gln Arg Lys Trp Glu Ala Ala His Glu
            180                 185                 190

Ala Glu Gln Gln Arg Ala Tyr Leu Glu Gly Thr Cys Val Glu Trp Leu
        195                 200                 205

Arg Arg Tyr Leu Glu Asn Gly Lys Glu Thr Leu Gln Arg Thr Thr Pro
    210                 215                 220

Pro Pro Lys Thr His Met Ile His His Ser Val Ser Asp Tyr Lys Ala
225                 230                 235                 240

Thr Leu Arg Cys Trp Ala Leu Gly Phe Tyr Pro Val Glu Ile Thr Leu
                245                 250                 255

Thr Trp Gln Gln Asp Gly Glu Asp Gln Thr Gln Asp Met Glu Leu Val
            260                 265                 270

Glu Thr Arg Pro Ala Gly Asp Gly Asn Phe Gln Lys Trp Ala Ala Val
        275                 280                 285

Val Val Pro Ser Gly Glu Glu Gln Arg Tyr Met Cys His Val Gln His
    290                 295                 300

Glu Gly Leu Pro Lys Pro Leu Thr Leu Arg Trp Glu Gln Ser Ser Gln
305                 310                 315                 320

Pro Thr Ile Pro Ile Val Gly Ile Val Ala Gly Leu Val Leu Leu Gly
                325                 330                 335

Ala Val Val Thr Gly Ala Val Val Ser Ala Val Met Cys Arg Lys Asn
            340                 345                 350

Ser Phe Cys Ser Thr Pro Gly Ser Asn His Ala Gln Gly Ser Asp Val
        355                 360                 365

Ser Leu Thr Ala Cys Lys Gly Glu Thr Leu Gly Asp Leu Met Cys Gly
    370                 375                 380

Gly Cys Trp Gly Gln
385

<210> 71
<211> 1159
<212> DNA
<213> Homo sapiens

<400> 71
tctccccaga cgccgaggat ggtgctcatg gcgccccgaa ccctcctcct gctgctctca 60
ggggccctga cccagacctg ggcgcgttcc cactccatga ggtatttcta caccaccatg 120
tcccggcccg gccgcgggga gccccgcttc atctccgtcg gctacgtgga ctatacgcag 180
ttcgtgcggt tcgacagcga cgacgcgagt ccgagagagg agccgcgggc gccgtggatg 240
gagcgggagg ggccggagta ttgggaccgg aacacacaga tctgcaaggc ccaagcacgg 300
actgaacgag agaacctgcg gatcgcgctc cgctactaca accagagcga gggcggtggt 360
tcccacacca tgcaggtgat gtatggctgc gacgtggggc ccgacgggcg cttcctccgc 420
gggtatgaac agcacgccta cgacggcaag gattacatcg ctctgaacga ggacctgcgc 480
tcctggaccg cggcggacat ggcagctcag atcaccaagc gcaagtggga ggcggcccgt 540
gtggcggagc agctgagagc ctacctggag ggcgagttcg tggagtggct ccgcagatac 600
ctggagaacg ggaaggagac gctgcagcgc gcgtcagacc cccccaagac acatatgacc 660
cactacccca tctctgacca tgaggccacc ctgaggtgct gggccctggg cttctaccct 720
gcggagatca cactgacctg gcagcgggat ggggaggacc agaccacgga gctcgtggag 780
accaggcctg caggggatgg aaccttccag aagtgggcgg ctgtggtggt gccttctgga 840
gaggagcaga gatacacctg ccatgtgcag catgagggtc tgcccgagcc cctcaccctg 900
agatggcagg gtcagggtcc ctcaccttcc cccttttccc cagagccatc ttcccagccc 960
accatcccca tcgtgggcat cattgctggc ctggttctac ttgtagctgt ggtcactgga 1020
gctgtggtca ctgctgtaat gtggaggaag aagagctcag gtaaggaagg ggatgggtat 1080
tctactccag gcggcaacag tgcccagggc tctgatgtgt ctctcacggc gtgaaaggtg 1140
agaccttggg gggcctgat                                              1159

<210> 72
<211> 371
<212> PRT
<213> Homo sapiens

<400> 72
Met Val Leu Met Ala Pro Arg Thr Leu Leu Leu Leu Leu Ser Gly Ala
  1               5                  10                  15

Leu Thr Gln Thr Trp Ala Arg Ser His Ser Met Arg Tyr Phe Tyr Thr
             20                  25                  30

Thr Met Ser Arg Pro Gly Arg Gly Glu Pro Arg Phe Ile Ser Val Gly
         35                  40                  45

Tyr Val Asp Tyr Thr Gln Phe Val Arg Phe Asp Ser Asp Asp Ala Ser
     50                  55                  60

Pro Arg Glu Glu Pro Arg Ala Pro Trp Met Glu Arg Glu Gly Pro Glu
 65                  70                  75                  80

Tyr Trp Asp Arg Asn Thr Gln Ile Cys Lys Ala Gln Ala Arg Thr Glu
                 85                  90                  95

Arg Glu Asn Leu Arg Ile Ala Leu Arg Tyr Tyr Asn Gln Ser Glu Gly

100                 105                 110

Gly Gly Ser His Thr Met Gln Val Met Tyr Gly Cys Asp Val Gly Pro
        115                 120                 125

Asp Gly Arg Phe Leu Arg Gly Tyr Glu Gln His Ala Tyr Asp Gly Lys
    130                 135                 140

Asp Tyr Ile Ala Leu Asn Glu Asp Leu Arg Ser Trp Thr Ala Ala Asp
145                 150                 155                 160

Met Ala Ala Gln Ile Thr Lys Arg Lys Trp Glu Ala Ala Arg Val Ala
                165                 170                 175

Glu Gln Leu Arg Ala Tyr Leu Glu Gly Glu Phe Val Glu Trp Leu Arg
            180                 185                 190

Arg Tyr Leu Glu Asn Gly Lys Glu Thr Leu Gln Arg Ala Ser Asp Pro
        195                 200                 205

Pro Lys Thr His Met Thr His Tyr Pro Ile Ser Asp His Glu Ala Thr
    210                 215                 220

Leu Arg Cys Trp Ala Leu Gly Phe Tyr Pro Ala Glu Ile Thr Leu Thr
225                 230                 235                 240

Trp Gln Arg Asp Gly Glu Asp Gln Thr Thr Glu Leu Val Glu Thr Arg
                245                 250                 255

Pro Ala Gly Asp Gly Thr Phe Gln Lys Trp Ala Ala Val Val Val Pro
            260                 265                 270

Ser Gly Glu Glu Gln Arg Tyr Thr Cys His Val Gln His Glu Gly Leu
        275                 280                 285

Pro Glu Pro Leu Thr Leu Arg Trp Gln Gly Gln Gly Pro Ser Pro Ser
    290                 295                 300

Pro Leu Phe Pro Glu Pro Ser Ser Gln Pro Thr Ile Pro Ile Val Gly
305                 310                 315                 320

Ile Ile Ala Gly Leu Val Leu Leu Val Ala Val Val Thr Gly Ala Val
                325                 330                 335

Val Thr Ala Val Met Trp Arg Lys Lys Ser Ser Gly Lys Glu Gly Asp
            340                 345                 350

Gly Tyr Ser Thr Pro Gly Gly Asn Ser Ala Gln Gly Ser Asp Val Ser
        355                 360                 365

Leu Thr Ala
    370

<210> 73
<211> 565
<212> DNA
<213> Homo sapiens

<400> 73
aggggaagca tgagacggct gcggatctcg ctggccccgt gggtgggcgc gggggacgcg 60
ggaggggccg agctcacggg gccagcgccg gggcctgcag gtggccctgg aggaatctgc 120
aagcacccgc ccgtgcagcg ggccttccgg gagaccagtg tggacagcgc cctggacacg 180
cccttcccag ctggaacatc tgtgaggctg gaatttaagc tccggcagac aagcggctgg 240
aggaaggcct ggaagaaacc caagtgcaaa gcccagcccg agaggaggaa acagaaatgc 300
ctgacctgcg tcaaaatgga ctgtgaggat aaggttctgg gcaggatggt tcgctgccct 360
ccagagacgc agactcggcg ggagcctgag gagcaccagg gggccgggtg cagcccggcg 420
gagcgggcgg ggaggacccc acggcggagc gggcggggag gaccccacgg ctgccgcttc 480
cctgcacggt tcgcctcctc caaggcccgg cccccagcgg agccctagcg ctgaatcgca 540

tggcgccccc tggagccctg gcggg                                          565

<210> 74
<211> 172
<212> PRT
<213> Homo sapiens

<400> 74


<210> 75
<211> 1706
<212> DNA
<213> Homo sapiens

<400> 75
ctgccaagat ggcgtcggcc tcctcccaac cgtcgttggc ggtcggtttt tcatcctttg 60
atcccggggc cccttcctgt accgcgtcct cagcatctgg aatcttgagc cccacggcat 120
ctgaggtgcc ttatgcctct ggcatgccca tcaagaaaac aggccatcga ggtgtcgatt 180
cctcaggaga gacaacatat aaaaagacaa cctcaacagc cttgaaaggt gccatccagt 240
taggcattac ttacactgtg gggagcctga gtaccaaacc agagcgtgat gtcctcatgc 300
aagatttcta cgtggtggag agtatcttct tccccagtga agggagcaac ctgacccctg 360
ctcatcacta caatgccttc cgtttcaaga cctatgcgcc ggttgccttc cgctactttc 420
gggagctatt tggtatcccg cccgatgatt acttgtgctc cctctgcagt gagccgctga 480
ttgaactctg tagctctgga gctagtggtt ccctgttcta tgtgtccagc gacgatgaac 540
tcattattaa gacactccaa cataaagagg cggagtttct gcagaagctg cttccaggat 600
actacttgaa cctcagccag aaccctcgga ctttgctgcc taaattcttt ggactgtact 660
gtgtgcagac aggtggcaag aacattcgga ttgtggtgat gaacaatctt ttaccaagat 720
ccgtcaaaat gcatatcaaa tatgacctca aaggctcaac ctacaaacgc cgggcttccc 780
agaaagagcg agagaagcct cttcccacat ttaaagatct agacttctta caagacatcc 840
ctgatggtct ttttttggat gctgacacgt acaatgctct ctgtaagacc ctgcagcgtg 900
actgtttggt gctgcagagc ttcaagataa tggactatag cctctggctg tcaatccaca 960
atatagatca tgcacaacga gagcccttaa gcagcgacac tcttcaagtg tcaatcgaca 1020
ctcaaagact ggctccccaa aaggctctgt attccacagc catggaattc atccagggag 1080
aggctcggct gggcgacacc atggaggccg atgaccatat gggtggcatc cctgctcaga 1140
atagtaaagg ggaaaggctt ctgctttata ttggcatcat tgacattcta cagtcttaca 1200

cgtttcttaa gaagttggag cactcttgga aagccgtggt acatgatggg gacgctgtct 1260
cagtgcatcg cccaggcttc tacgctgaac ggttccagca cttcatgtgc aacgcagtat 1320
ttaagaagat ccccttgaag ccttctcctt ccaaaaagtt tcggtctggc ttatctttct 1380
ctctgcatac gggctccagt ggcaactcct gcattactta ccagccattg gtctctgagg 1440
aacacaagtc acaagtgata aaggtgcaag tggagccagg tgttcacctt ggtcgttctg 1500
atgttttacc tcagacctca gaatccacct ttggaggaaa tcagggaggg ctcactatta 1560
ctgaccacag tttctcacct gtagttggaa agactttgca tatgctaact acaagtataa 1620
ccttggaaaa acttgaatgt acagagtcag agttcaccca ttaagcgcaa agcctcagaa 1680
gacctggaac aagattctgc ttctct 1706

<210> 76
<211> 551
<212> PRT
<213> Homo sapiens

<400> 76
Met Ala Ser Ala Ser Ser Gln Pro Ser Leu Ala Val Gly Phe Ser Ser
1               5                   10                  15

Phe Asp Pro Gly Ala Pro Ser Cys Thr Ala Ser Ser Ala Ser Gly Ile
            20                  25                  30

Leu Ser Pro Thr Ala Ser Glu Val Pro Tyr Ala Ser Gly Met Pro Ile
        35                  40                  45

Lys Lys Thr Gly His Arg Gly Val Asp Ser Ser Gly Glu Thr Thr Tyr
    50                  55                  60

Lys Lys Thr Thr Ser Thr Ala Leu Lys Gly Ala Ile Gln Leu Gly Ile
65                  70                  75                  80

Thr Tyr Thr Val Gly Ser Leu Ser Thr Lys Pro Glu Arg Asp Val Leu
                85                  90                  95

Met Gln Asp Phe Tyr Val Val Glu Ser Ile Phe Phe Pro Ser Glu Gly
            100                 105                 110

Ser Asn Leu Thr Pro Ala His His Tyr Asn Ala Phe Arg Phe Lys Thr
        115                 120                 125

Tyr Ala Pro Val Ala Phe Arg Tyr Phe Arg Glu Leu Phe Gly Ile Pro
    130                 135                 140

Pro Asp Asp Tyr Leu Cys Ser Leu Cys Ser Glu Pro Leu Ile Glu Leu
145                 150                 155                 160

Cys Ser Ser Gly Ala Ser Gly Ser Leu Phe Tyr Val Ser Ser Asp Asp
                165                 170                 175

Glu Leu Ile Ile Lys Thr Leu Gln His Lys Glu Ala Glu Phe Leu Gln
            180                 185                 190

Lys Leu Leu Pro Gly Tyr Tyr Leu Asn Leu Ser Gln Asn Pro Arg Thr
        195                 200                 205

Leu Leu Pro Lys Phe Phe Gly Leu Tyr Cys Val Gln Thr Gly Gly Lys
    210                 215                 220

Asn Ile Arg Ile Val Val Met Asn Asn Leu Leu Pro Arg Ser Val Lys
225                 230                 235                 240

Met His Ile Lys Tyr Asp Leu Lys Gly Ser Thr Tyr Lys Arg Arg Ala
                245                 250                 255

Ser Gln Lys Glu Arg Glu Lys Pro Leu Pro Thr Phe Lys Asp Leu Asp
            260                 265                 270

Phe Leu Gln Asp Ile Pro Asp Gly Leu Phe Leu Asp Ala Asp Thr Tyr

        275                 280                 285

Asn Ala Leu Cys Lys Thr Leu Gln Arg Asp Cys Leu Val Leu Gln Ser
    290                 295                 300

Phe Lys Ile Met Asp Tyr Ser Leu Trp Leu Ser Ile His Asn Ile Asp
305                 310                 315                 320

His Ala Gln Arg Glu Pro Leu Ser Ser Asp Thr Leu Gln Val Ser Ile
                325                 330                 335

Asp Thr Gln Arg Leu Ala Pro Gln Lys Ala Leu Tyr Ser Thr Ala Met
            340                 345                 350

Glu Phe Ile Gln Gly Glu Ala Arg Leu Gly Asp Thr Met Glu Ala Asp
        355                 360                 365

Asp His Met Gly Gly Ile Pro Ala Gln Asn Ser Lys Gly Glu Arg Leu
    370                 375                 380

Leu Leu Tyr Ile Gly Ile Ile Asp Ile Leu Gln Ser Tyr Thr Phe Leu
385                 390                 395                 400

Lys Lys Leu Glu His Ser Trp Lys Ala Val Val His Asp Gly Asp Ala
                405                 410                 415

Val Ser Val His Arg Pro Gly Phe Tyr Ala Glu Arg Phe Gln His Phe
            420                 425                 430

Met Cys Asn Ala Val Phe Lys Lys Ile Pro Leu Lys Pro Ser Pro Ser
        435                 440                 445

Lys Lys Phe Arg Ser Gly Leu Ser Phe Ser Leu His Thr Gly Ser Ser
    450                 455                 460

Gly Asn Ser Cys Ile Thr Tyr Gln Pro Leu Val Ser Glu Glu His Lys
465                 470                 475                 480

Ser Gln Val Ile Lys Val Gln Val Glu Pro Gly Val His Leu Gly Arg
                485                 490                 495

Ser Asp Val Leu Pro Gln Thr Ser Glu Ser Thr Phe Gly Gly Asn Gln
            500                 505                 510

Gly Gly Leu Thr Ile Thr Asp His Ser Phe Ser Pro Val Val Gly Lys
        515                 520                 525

Thr Leu His Met Leu Thr Thr Ser Ile Thr Leu Glu Lys Leu Glu Cys
    530                 535                 540

Thr Glu Ser Glu Phe Thr His
545                 550

<210> 77
<211> 1316
<212> DNA
<213> Homo sapiens

<400> 77
tgggtgccat ggcggttcta ctggagacta ctgtgggcaa tgtggttgtc aatttgcaca 60
ctgagcagca gccttgcaac tgtgaacttt ttgagagcag gtaccacagt ttaatggcat 120
ttaatttctt gagatattac aaaataaaat attacagtta ttgccttatt cacagtatac 180
aaaggtattt tatcatacaa actgttgatc ctacagggac tggtcatgga ggagagtcta 240
tttttggcct aggattgtat ggtgatcaag caagcttttt tgagacagaa aacgtcccaa 300
gaattaagca caagaagaag ggcacaatgt ccatggtgaa taatgacagt gatcaacatg 360
gatctcagtt tcttatcact acaggagaaa atctagatta ccttgatggt acccatacag 420
tatttggtga ggtgacagaa ggcattgaca taattaagaa aataaatgag acctttgttg 480
acaaggactt tgtaccatat caggatatca ggataaatta tatagtgatt ttagatggtc 540

catttgatga cattcctgat ttattaatcc ctgatcaatc accagaacct acaagggaac 600
aattaaagag tggtagagtt gacacaaatg aagaaattga tcatttcaaa cgaaggtcag 660
ccgaagaagt agaagaaata aaggcagaaa aagaagctaa aactcaggct ttacttttag 720
agatggtggg agacctacct gatgcagata ttaaacctcc ggaaaaatct gtgtttgtat 780
gcaaattgaa tccagtgacc acagatgagg atctggatat aatactctct agatttgggc 840
caataagaag ttgtgaagtt atctgggact ggaagacagg agaaatcctc tgttatttct 900
ttctttcttt ctatgctttt attgaatttg aaaaggaaga agattatgag aaagccttct 960
tcaaaatgga caatatactt atagatgaca gaagaaaaca tggatttgcc agtctgttac 1020
aaaggttaaa tggaaggaaa aagtgggaaa tacaccaaca gccggggcgc agcccacgcc 1080
gccgccgccg cccgcaccgt tcccgctccc ggcggcggcg acggcgggcg gggaccccgc 1140
ggcgctgcgc ctcatcctct gcgacggggg atgaggggtc tgagaggaac tggaggagga 1200
ggaggaggag gcagtggaca tggtggctct gcgggccctg gacgccgccg gccgacacgg 1260
aggcgcgggg cgggaggcgc gcgagcgtgt gggagccggc gcgatagtgc gtgcga 1316

<210> 78
<211> 432
<212> PRT
<213> Homo sapiens

<400> 78
Met Ala Val Leu Leu Glu Thr Thr Val Gly Asn Val Val Val Asn Leu
1               5                   10                  15

His Thr Glu Gln Gln Pro Cys Asn Cys Glu Leu Phe Glu Ser Arg Tyr
            20                  25                  30

His Ser Leu Met Ala Phe Asn Phe Leu Arg Tyr Tyn Lys Ile Lys Tyr
        35                  40                  45

Tyr Ser Tyr Cys Leu Ile His Ser Ile Gln Arg Tyr Phe Ile Ile Gln
    50                  55                  60

Thr Val Asp Pro Thr Gly Thr Gly His Gly Gly Glu Ser Ile Phe Gly
65                  70                  75                  80

Leu Gly Leu Tyr Gly Asp Gln Ala Ser Phe Phe Glu Thr Glu Asn Val
                85                  90                  95

Pro Arg Ile Lys His Lys Lys Lys Gly Thr Met Ser Met Val Asn Asn
            100                 105                 110

Asp Ser Asp Gln His Gly Ser Gln Phe Leu Ile Thr Thr Gly Glu Asn
        115                 120                 125

Leu Asp Tyr Leu Asp Gly Thr His Thr Val Phe Gly Glu Val Thr Glu
    130                 135                 140

Gly Ile Asp Ile Ile Lys Lys Ile Asn Glu Thr Phe Val Asp Lys Asp
145                 150                 155                 160

Phe Val Pro Tyr Gln Asp Ile Arg Ile Asn Tyr Ile Val Ile Leu Asp
                165                 170                 175

Gly Pro Phe Asp Asp Ile Pro Asp Leu Leu Ile Pro Asp Gln Ser Pro
            180                 185                 190

Glu Pro Thr Arg Glu Gln Leu Lys Ser Gly Arg Val Asp Thr Asn Glu
        195                 200                 205

Glu Ile Asp His Phe Lys Arg Arg Ser Ala Glu Glu Val Glu Glu Ile
    210                 215                 220

Lys Ala Glu Lys Glu Ala Lys Thr Gln Ala Leu Leu Leu Glu Met Val
225                 230                 235                 240

Gly Asp Leu Pro Asp Ala Asp Ile Lys Pro Pro Glu Lys Ser Val Phe
                245                 250                 255

Val Cys Lys Leu Asn Pro Val Thr Thr Asp Glu Asp Leu Asp Ile Ile
            260                 265                 270

Leu Ser Arg Phe Gly Pro Ile Arg Ser Cys Glu Val Ile Trp Asp Trp
        275                 280                 285

Lys Thr Gly Glu Ile Leu Cys Tyr Phe Phe Leu Ser Phe Tyr Ala Phe
    290                 295                 300

Ile Glu Phe Glu Lys Glu Glu Asp Tyr Glu Lys Ala Phe Phe Lys Met
305                 310                 315                 320

Asp Asn Ile Leu Ile Asp Asp Arg Arg Lys His Gly Phe Ala Ser Leu
                325                 330                 335

Leu Gln Arg Leu Asn Gly Arg Lys Lys Trp Glu Ile His Gln Gln Pro
            340                 345                 350

Gly Arg Ser Pro Arg Arg Arg Arg Arg Pro His Arg Ser Arg Ser Arg
        355                 360                 365

Arg Arg Arg Arg Arg Ala Gly Thr Pro Arg Arg Cys Ala Ser Ser Ser
    370                 375                 380

Ala Thr Gly Asp Glu Gly Ser Glu Arg Asn Trp Arg Arg Arg Arg Arg
385                 390                 395                 400

Arg Gln Trp Thr Trp Trp Leu Cys Gly Pro Trp Thr Pro Pro Ala Asp
                405                 410                 415

Thr Glu Ala Arg Gly Gly Arg Arg Ala Ser Val Trp Glu Pro Ala Arg
            420                 425                 430

<210> 79
<211> 1647
<212> DNA
<213> Homo sapiens

<400> 79
   gacctcagaa gccatgttga agccccatag tgaagccagg gctgccttca ttcagaccca 60
   gcagctgcac gcagccatgg ctgacacatt cttggagcac atgtgctgcc tggacactga 120
   ctcgccaccc atcacagcct ggagcactgg catcatctgt actatgggcc cagcttctcc 180
   attgctagag atgctgaaga aaacgattaa gtctggaatt aatgtggctc atctgaactc 240
   tcatggagcc catgagtacc atacagagac catcaagaac gtgcgcacag ccacggaaag 300
   ctttgcttct gactccatcc tctaccagcc cattgctgtg gctccagaca ctaaaggacc 360
   tgagatccca actgggcccg tcaagggcag cggcactgca gaggtggagc tgaagaaggg 420
   agccactctc aagttcacgc tggataatac ctacatggaa aagggtaaag agaacatcct 480
   gtggcgggac tacaagaaca tctgcaaggt ggtggaagtg ggctgcaaga tctacgtgga 540
   tgatgggcta atttctctcc aagtgaagca gaaggatgct cactttctgg tgacagaggt 600
   ggaaaatggt ggctccttgg gcagcaagaa gagtgtgaac cttcctgggg ctgccgtgga 660
   cctgtctgcc atgttggaga aggacatcca ggacctgaag tttgggggcg agcaagatgt 720
   cgatatgatg ttttcatcat tcatctgcaa gacatctgat gtccatgaag ttaggaaggt 780
   cttgggagag aaaggaaaga acagcaagat aaccagcaaa attgagaatc atgatggggg 840
   ttggaggttt gatgaaatcc tggaggccag cgatgggatt atggtagctc gtggtgatcc 900
   accacaagcc gtcgagatgg agattcctgc agggaaggtc tgccttgctc agaggatgat 960
   gattgggtgg tgcaaccaag ctgggaagcc tgtcatcttt gccactcaga tgctagagga 1020
   tgtgatcaag aagctccacc ccacttgggc tgagggcagt ggtgtggcca atgcagttct 1080
   ggtggaagct gactgcatca tgctgtctgg agaaacagcc aaagggaact atcctctgga 1140
   ggctgtgcac atgcagcacc tgattgcctg tgaggcagag gccaccatct accacttgca 1200
   attatttgag gagttctgcc acctggcacc cattaccagt gaccccgcag aagctactgc 1260
   catgggcact gtggaggcct ccttcaagtg ctgcagtggg gccataatcg tcctcaccaa 1320
   gtctgccagg tgtgcccacc aggtggccag atactgccca cgtgccccca tgattgttgt 1380
   gacatggcat ccccaggcag ctcgccaggc ccacctgtac cgtggtatct tccctgtgct 1440
   gtgtaaggac cccatccagg agccccaggc tgaggatgtg gacctccgag tgaacttggc 1500
   catgaatgtt ggtaaggccc gaggcttctt caagaaggat gatgtggtca ttgtgctgac 1560
   ctggggacac cctggccctg gcttctccac caccctgtgt gttattcctg tgctgtgatg 1620
   gactccagag ctcttcctcc agcccct 1647

<210> 80
<211> 534
<212> PRT
<213> Homo sapiens

<400> 80
Met Leu Lys Pro His Ser Glu Ala Arg Ala Ala Phe Ile Gln Thr Gln
1               5                   10                  15

Gln Leu His Ala Ala Met Ala Asp Thr Phe Leu Glu His Met Cys Cys
            20                  25                  30

Leu Asp Thr Asp Ser Pro Pro Ile Thr Ala Trp Ser Thr Gly Ile Ile
        35                  40                  45

Cys Thr Met Gly Pro Ala Ser Pro Leu Leu Glu Met Leu Lys Lys Thr
    50                  55                  60

Ile Lys Ser Gly Ile Asn Val Ala His Leu Asn Ser His Gly Ala His
65                  70                  75                  80

Glu Tyr His Thr Glu Thr Ile Lys Asn Val Arg Thr Ala Thr Glu Ser
                85                  90                  95

Phe Ala Ser Asp Ser Ile Leu Tyr Gln Pro Ile Ala Val Ala Pro Asp
            100                 105                 110

Thr Lys Gly Pro Glu Ile Pro Thr Gly Pro Val Lys Gly Ser Gly Thr
        115                 120                 125

Ala Glu Val Glu Leu Lys Lys Gly Ala Thr Leu Lys Phe Thr Leu Asp
    130                 135                 140

Asn Thr Tyr Met Glu Lys Gly Lys Glu Asn Ile Leu Trp Arg Asp Tyr
145                 150                 155                 160

Lys Asn Ile Cys Lys Val Val Glu Val Gly Cys Lys Ile Tyr Val Asp
                165                 170                 175

Asp Gly Leu Ile Ser Leu Gln Val Lys Gln Lys Asp Ala His Phe Leu
            180                 185                 190

Val Thr Glu Val Glu Asn Gly Gly Ser Leu Gly Ser Lys Lys Ser Val
        195                 200                 205

Asn Leu Pro Gly Ala Ala Val Asp Leu Ser Ala Met Leu Glu Lys Asp
    210                 215                 220

Ile Gln Asp Leu Lys Phe Gly Gly Glu Gln Asp Val Asp Met Met Phe
225                 230                 235                 240

Ser Ser Phe Ile Cys Lys Thr Ser Asp Val His Glu Val Arg Lys Val
                245                 250                 255

Leu Gly Glu Lys Gly Lys Asn Ser Lys Ile Thr Ser Lys Ile Glu Asn
            260                 265                 270

His Asp Gly Gly Trp Arg Phe Asp Glu Ile Leu Glu Ala Ser Asp Gly
        275                 280                 285

Ile Met Val Ala Arg Gly Asp Pro Pro Gln Ala Val Glu Met Glu Ile
    290                 295                 300

Pro Ala Gly Lys Val Cys Leu Ala Gln Arg Met Met Ile Gly Trp Cys
305                 310                 315                 320

Asn Gln Ala Gly Lys Pro Val Ile Phe Ala Thr Gln Met Leu Glu Asp
                325                 330                 335

Val Ile Lys Lys Leu His Pro Thr Trp Ala Glu Gly Ser Gly Val Ala
            340                 345                 350

Asn Ala Val Leu Val Glu Ala Asp Cys Ile Met Leu Ser Gly Glu Thr
        355                 360                 365

Ala Lys Gly Asn Tyr Pro Leu Glu Ala Val His Met Gln His Leu Ile
    370                 375                 380

Ala Cys Glu Ala Glu Ala Thr Ile Tyr His Leu Gln Leu Phe Glu Glu
385                 390                 395                 400

Phe Cys His Leu Ala Pro Ile Thr Ser Asp Pro Ala Glu Ala Thr Ala
                405                 410                 415

Met Gly Thr Val Glu Ala Ser Phe Lys Cys Cys Ser Gly Ala Ile Ile
            420                 425                 430

Val Leu Thr Lys Ser Ala Arg Cys Ala His Gln Val Ala Arg Tyr Cys
        435                 440                 445

Pro Arg Ala Pro Met Ile Val Val Thr Trp His Pro Gln Ala Ala Arg
    450                 455                 460

Gln Ala His Leu Tyr Arg Gly Ile Phe Pro Val Leu Cys Lys Asp Pro
465                 470                 475                 480

Ile Gln Glu Pro Gln Ala Glu Asp Val Asp Leu Arg Val Asn Leu Ala
                485                 490                 495

Met Asn Val Gly Lys Ala Arg Gly Phe Phe Lys Lys Asp Asp Val Val
            500                 505                 510

Ile Val Leu Thr Trp Gly His Pro Gly Pro Gly Phe Ser Thr Thr Leu
        515                 520                 525

Cys Val Ile Pro Val Leu
    530


<210> 81
<211> 600
<212> DNA
<213> Homo sapiens

<400> 81
accaggagcc ctgtactacc agccatggtc aaccccacca tgttcttcaa catcgccatc 60
aacagcgagg ccttggggca cgtctccttc gaactgtttg cagacaagtt tccaaagaca 120
gaaaactttc gtgctctgag cactggagag aaaggatttg gttataaggg ttcctgcttt 180
cacagaatta ttctagggct tttgtgtcag ggtggtgact ttacatgcca taatggcact 240
ggtggcaagt ctgtctacag ggagaaattt gatgatgaga acttcattct gaagcataca 300
ggtcctggca tcttgtccat gaagcataca ggtcctggca tcttgtccat ggcaaatgct 360
ggacccaaca caaacgattc ccagattttc atctgcactg ccaagaccga gtggttggat 420
ggcaagcatg tggtctctgg cagggtgaaa gaaggcatca agattgtgga ggccatgaag 480
cgctatgggt ccaagaatgg caagagcagg aagaagatca ccactgctga ctgtggacaa 540
ctctaataag tttgacttgt gttttatctt aaccaccaga ccattccttt tgtagctcag 600


<210> 82
<211> 173
<212> PRT
<213> Homo sapiens

<400> 82
Met Val Asn Pro Thr Met Phe Phe Asn Ile Ala Ile Asn Ser Glu Ala

1                   5                   10                  15

Leu Gly His Val Ser Phe Glu Leu Phe Ala Asp Lys Phe Pro Lys Thr
                20                  25                  30

Glu Asn Phe Arg Ala Leu Ser Thr Gly Glu Lys Gly Phe Gly Tyr Lys
            35                  40                  45

Gly Ser Cys Phe His Arg Ile Ile Leu Gly Leu Leu Cys Gln Gly Gly
    50                  55                  60

Asp Phe Thr Cys His Asn Gly Thr Gly Gly Lys Ser Val Tyr Arg Glu
65                  70                  75                  80

Lys Phe Asp Asp Glu Asn Phe Ile Leu Lys His Thr Gly Pro Gly Ile
                85                  90                  95

Leu Ser Met Lys His Thr Gly Pro Gly Ile Leu Ser Met Ala Asn Ala
            100                 105                 110

Gly Pro Asn Thr Asn Asp Ser Gln Ile Phe Ile Cys Thr Ala Lys Thr
        115                 120                 125

Glu Trp Leu Asp Gly Lys His Val Val Ser Gly Arg Val Lys Glu Gly
    130                 135                 140

Ile Lys Ile Val Glu Ala Met Lys Arg Tyr Gly Ser Lys Asn Gly Lys
145                 150                 155                 160

Ser Arg Lys Lys Ile Thr Thr Ala Asp Cys Gly Gln Leu
                165                 170

<210> 83
<211> 566
<212> DNA
<213> Homo sapiens

<400> 83
gtactaccag ccatggtcaa ccccaccatg ttcttcaaca tcgccatcaa cagcgaggcc 60
ttggggcacg tctccttcga actgtttgca gacaagtttc caaagacaga aaactttcgt 120
gctctgagca ctggagagaa aggatttggt tataagggtt cctgctttca cagaattatt 180
ctagggcttt tgtgtcaggg tggtgacttt acatgccata atggcactgg tggcaagtct 240
gtctacaggg agaaatttga tgatgagaac ttcattctga agcatacagg tcctggcatc 300
ttgtccatga agcatacagg tcctggcatc ttgtccatgg caaatgctgg acccaacaca 360
aacgattccc agattttcat ctgcactgcc aagaccgagt ggttggatgg caagcatgtg 420
gtctctggca gggtgaaaga aggcatcaag attgtggagg ccatgaagcg ctatgggtcc 480
aagaatggca agagcaggaa gaagatcacc actgctgact gtggacaact ctaataagtt 540
tgacttgtgt tttatcttaa ccacca                                      566

<210> 84
<211> 173
<212> PRT
<213> Homo sapiens

<400> 84
Met Val Asn Pro Thr Met Phe Phe Asn Ile Ala Ile Asn Ser Glu Ala
1               5                   10                  15

Leu Gly His Val Ser Phe Glu Leu Phe Ala Asp Lys Phe Pro Lys Thr
            20                  25                  30

Glu Asn Phe Arg Ala Leu Ser Thr Gly Glu Lys Gly Phe Gly Tyr Lys
        35                  40                  45

Gly Ser Cys Phe His Arg Ile Ile Leu Gly Leu Leu Cys Gln Gly Gly
    50                  55                  60

Asp Phe Thr Cys His Asn Gly Thr Gly Gly Lys Ser Val Tyr Arg Glu
65                  70                  75                  80

Lys Phe Asp Asp Glu Asn Phe Ile Leu Lys His Thr Gly Pro Gly Ile
                85                  90                  95

Leu Ser Met Lys His Thr Gly Pro Gly Ile Leu Ser Met Ala Asn Ala
            100                 105                 110

Gly Pro Asn Thr Asn Asp Ser Gln Ile Phe Ile Cys Thr Ala Lys Thr
        115                 120                 125

Glu Trp Leu Asp Gly Lys His Val Val Ser Gly Arg Val Lys Glu Gly
    130                 135                 140

Ile Lys Ile Val Glu Ala Met Lys Arg Tyr Gly Ser Lys Asn Gly Lys
145                 150                 155                 160

Ser Arg Lys Lys Ile Thr Thr Ala Asp Cys Gly Gln Leu
                165                 170

<210> 85
<211> 660
<212> DNA
<213> Homo sapiens

<400> 85
actagtcatt cttcccagta gctaatgaag ctgactttta aaaagaaggc tgtgagcttt 60
gcagatgctg ctgccgccca gggccccctg cttccagcca tggtcaaccc caccatgttt 120
ttccacattg ctgtcgatgg cgagcccttg ggctgtgtct ccttcgaggt agagctgttt 180
gcagacaagg ttccaaagac agcagaaaat ttccatgctc tgagcactgg agaaaaagga 240
tttggttata agggttcctg ctttcacaga attattccag ggtttacgtg tcagagtggt 300
gacttcacac gccatggtgg caagtccatc tgcagggaga aatttgatga caagaacttc 360
atcctgaagc atacgggtcc tggcatcttg tccatggcaa atgctggacc cagcgtgaac 420
gtttcccagt tttttatctg ccctgccaag acagagtggt tggattgcaa gcatgtggtc 480
tttggcaagg tgaaagatgg catgaatatt gtggaggtca tggagcactt ggggtccaag 540
aatggcaaga tcagcaagaa gatcaccatt gctgactgga caactgcaat aaatttgacg 600
ggtgtttctc ttaaaaaaaa aaaaaaaata ctgtgacaga ccaaggtaaa ttgtttttga 660

<210> 86
<211> 203
<212> PRT
<213> Homo sapiens

<400> 86

<210> 87
<211> 600
<212> DNA
<213> Homo sapiens

<400> 87
aatttattgg tttgtttgtt taaaatattt gttggcactt tgcagatgcc acttccactg 60
atgtcaccac tgccagtgat ggtcacctcc accttgttct ttaactttgt agtcaacggt 120
gagcacttgg gccatgtctc cttccagctg tttgcaaaga aagttccaaa gacagcagaa 180
aatgttcatt ttgtgagcac tggagagaaa ggatttggct ataagtgttc ctgttttcac 240
agaattattc cagggtttat atgccagagt ggtgacttca catgtcatga tgacactggc 300
acaaagtcca actactggga gaagtctgat gatgataact ccatcctgaa gcatacaaga 360
cctggcacct tgtccatggc aaatactgga cgctacacaa atggtttcca gtttttcatc 420
tgcactgcca aaactgtgtg gttgggtggc aagagtgcag tctttggcaa gacaaaagag 480
ggcttgaata tcttggaagc catggcgcac tttgctttct ggaatggcaa aaccagaaag 540
aagaccacga ttgacaactg tggacaactc caataaattt aacttatgtt ttgttttaac 600

<210> 88
<211> 176
<212> PRT
<213> Homo sapiens

<400> 88
Met Pro Leu Pro Leu Met Ser Pro Leu Pro Val Met Val Thr Ser Thr
1               5                   10                  15

Leu Phe Phe Asn Phe Val Val Asn Gly Glu His Leu Gly His Val Ser
            20                  25                  30

Phe Gln Leu Phe Ala Lys Lys Val Pro Lys Thr Ala Glu Asn Val His
        35                  40                  45

Phe Val Ser Thr Gly Glu Lys Gly Phe Gly Tyr Lys Cys Ser Cys Phe
    50                  55                  60

His Arg Ile Ile Pro Gly Phe Ile Cys Gln Ser Gly Asp Phe Thr Cys
65                  70                  75                  80

His Asp Asp Thr Gly Thr Lys Ser Asn Tyr Trp Glu Lys Ser Asp Asp
                85                  90                  95

Asp Asn Ser Ile Leu Lys His Thr Arg Pro Gly Thr Leu Ser Met Ala
            100                 105                 110

Asn Thr Gly Arg Tyr Thr Asn Gly Phe Gln Phe Phe Ile Cys Thr Ala
        115                 120                 125

Lys Thr Val Trp Leu Gly Gly Lys Ser Ala Val Phe Gly Lys Thr Lys
    130                 135                 140

Glu Gly Leu Asn Ile Leu Glu Ala Met Ala His Phe Ala Phe Trp Asn
145                 150                 155                 160

Gly Lys Thr Arg Lys Lys Thr Thr Ile Asp Asn Cys Gly Gln Leu Gln
                165                 170                 175

<210> 89
<211> 600
<212> DNA
<213> Homo sapiens

<400> 89
ctgaaaactt tcttgtaaga ctttgacctg cattatatga ttctccttaa tcttcacagc 60
atgatttcgt gtttttggac atttctatac tgtgatgtgt gtcccaaaac atgtaaaaat 120
tttcaggtct tgtgcacagg aaaagcaggg ttttctcaac gtggcataag actacattac 180
aaaaattcca tttttcatcg aatagtacag aatggctgga tacaaggagg ggatatagtg 240
tatggaaaag gagataatgg agagtcgatt tatggtccaa catttgaaga tgaaaacttt 300
tcagttcctc ataataaaag aggagtactt ggaatggcca acaaaggccg tcacagcaac 360
gggtcacaat tctatatcac actgcaagca actccttatc tagatagaaa atttgtggct 420
tttgggtatg tatattgtag atctatttat ataatattca cacctggtag taaaaaagcc 480
cagagaagta tgtgcaagaa actaacagta tgtggttgtg ggcgtagttt ttcaaaggaa 540
gaagtagtca aatgctgtaa caaggacaac tcatcttgaa acacttacgc agtggtgtgt 600

<210> 90
<211> 180
<212> PRT
<213> Homo sapiens

<400> 90
Met Ile Leu Leu Asn Leu His Ser Met Ile Ser Cys Phe Trp Thr Phe
1               5                   10                  15

Leu Tyr Cys Asp Val Cys Pro Lys Thr Cys Lys Asn Phe Gln Val Leu
            20                  25                  30

Cys Thr Gly Lys Ala Gly Phe Ser Gln Arg Gly Ile Arg Leu His Tyr
        35                  40                  45

Lys Asn Ser Ile Phe His Arg Ile Val Gln Asn Gly Trp Ile Gln Gly
    50                  55                  60

Gly Asp Ile Val Tyr Gly Lys Gly Asp Asn Gly Glu Ser Ile Tyr Gly
65                  70                  75                  80

Pro Thr Phe Glu Asp Glu Asn Phe Ser Val Pro His Asn Lys Arg Gly
                85                  90                  95

Val Leu Gly Met Ala Asn Lys Gly Arg His Ser Asn Gly Ser Gln Phe
            100                 105                 110

Tyr Ile Thr Leu Gln Ala Thr Pro Tyr Leu Asp Arg Lys Phe Val Ala
        115                 120                 125

Phe Gly Tyr Val Tyr Cys Arg Ser Ile Tyr Ile Ile Phe Thr Pro Gly
    130                 135                 140

Ser Lys Lys Ala Gln Arg Ser Met Cys Lys Lys Leu Thr Val Cys Gly
145                 150                 155                 160

Cys Gly Arg Ser Phe Ser Lys Glu Glu Val Val Lys Cys Cys Asn Lys
                165                 170                 175

Asp Asn Ser Ser
            180

<210> 91
<211> 572
<212> DNA
<213> Homo sapiens

<400> 91
tttgtagtca tcgctgccac ctgaagccac ctgcctctag ccatggtcaa cgccccactg 60
tgttcttttg acatcattgt tgatggtaac tcctttggcc catgcagctc cttcgagctg 120
tttgccgaca aagttccaaa aacagtggaa aactttcgtg cactgagcac tggaggaaaa 180
ggatttggtt ataagggttc ctgctttcac agaattattc cagggtttat tttatctgcc 240
agagtgctga cttcacacac cataataatg ccccagtcca tctaccagga gaaatttgat 300
gatgagaact tcatcttgaa gcacacaggt cctggcatct tgtccatggc aaatgctggc 360
ccggacacaa atggttccca gtttttcacc tgtgtggcca agactgagtg gctggatggc 420
aagcacaagg tctttggcaa agtgagaaga ggggtgaata tcatggaagc catggagtgc 480
tctgggtccg ggaatggtga gactggcaag aagatcacca ctgccaactg cggacaactc 540
taatcaatct gcttgtgttt gatcttaacc ac 572

<210> 92
<211> 166
<212> PRT
<213> Homo sapiens

<400> 92
Met Val Asn Ala Pro Leu Cys Ser Phe Asp Ile Ile Val Asp Gly Asn
1               5                   10                  15

Ser Phe Gly Pro Cys Ser Ser Phe Glu Leu Phe Ala Asp Lys Val Pro
            20                  25                  30

Lys Thr Val Glu Asn Phe Arg Ala Leu Ser Thr Gly Gly Lys Gly Phe
        35                  40                  45

Gly Tyr Lys Gly Ser Cys Phe His Arg Ile Ile Pro Gly Phe Ile Leu
    50                  55                  60

Ser Ala Arg Val Leu Thr Ser His Thr Ile Ile Met Pro Gln Ser Ile
65                  70                  75                  80

Tyr Gln Glu Lys Phe Asp Asp Glu Asn Phe Ile Leu Lys His Thr Gly
                85                  90                  95

Pro Gly Ile Leu Ser Met Ala Asn Ala Gly Pro Asp Thr Asn Gly Ser
            100                 105                 110

Gln Phe Phe Thr Cys Val Ala Lys Thr Glu Trp Leu Asp Gly Lys His
        115                 120                 125

Lys Val Phe Gly Lys Val Arg Arg Gly Val Asn Ile Met Glu Ala Met
    130                 135                 140

Glu Cys Ser Gly Ser Gly Asn Gly Glu Thr Gly Lys Lys Ile Thr Thr
145                 150                 155                 160

Ala Asn Cys Gly Gln Leu
                165

<210> 93
<211> 525
<212> DNA
<213> Homo sapiens

<400> 93
gcccagaact ccctgccacc agccatggcc aaccccactg tgttcttcaa cattgcaatt 60
gatagtgagt ccttgggctg catctccttc aagctatttg cagacaaagt tctaaagatg 120
gaagaaaatt tttgtgctct gaacactgga gagaaagtat ttggtgataa atgtccctgc 180

ttttacagaa ttattccggg ggtgtgtcag ggtggtgact tcacacacca taatggcact 240
ggtggcaagt ccctctacag caaggaattt gatgatgaga acttcatcct aaagcataca 300
gctcctggcg tcttgtccac ggcaaatgct ggacccacca caaatggttc ccagtttttc 360
ttctgtactg ccaagacaga ggatggacag catgtggtct ttggcaaggt gaaagatggc 420
atgagtattg tggaagccct ggaacgctct gggtccagga atggtaagac cagcaagaag 480
atcacagctg ctgactgtgg acaactctaa taaatttgat tgttt 525

<210> 94
<211> 161
<212> PRT
<213> Homo sapiens

<400> 94
Met Ala Asn Pro Thr Val Phe Phe Asn Ile Ala Ile Asp Ser Glu Ser
1               5                   10                  15

Leu Gly Cys Ile Ser Phe Lys Leu Phe Ala Asp Lys Val Leu Lys Met
            20                  25                  30

Glu Glu Asn Phe Cys Ala Leu Asn Thr Gly Glu Lys Val Phe Gly Asp
        35                  40                  45

Lys Cys Pro Cys Phe Tyr Arg Ile Ile Pro Gly Val Cys Gln Gly Gly
    50                  55                  60

Asp Phe Thr His His Asn Gly Thr Gly Gly Lys Ser Leu Tyr Ser Lys
65                  70                  75                  80

Glu Phe Asp Asp Glu Asn Phe Ile Leu Lys His Thr Ala Pro Gly Val
                85                  90                  95

Leu Ser Thr Ala Asn Ala Gly Pro Thr Thr Asn Gly Ser Gln Phe Phe
            100                 105                 110

Phe Cys Thr Ala Lys Thr Glu Asp Gly Gln His Val Val Phe Gly Lys
        115                 120                 125

Val Lys Asp Gly Met Ser Ile Val Glu Ala Leu Glu Arg Ser Gly Ser
    130                 135                 140

Arg Asn Gly Lys Thr Ser Lys Lys Ile Thr Ala Ala Asp Cys Gly Gln
145                 150                 155                 160

Leu

<210> 95
<211> 720
<212> DNA
<213> Homo sapiens

<400> 95
catcaggaaa atgcaaatca aaccacaacg agatatcatg tcacaccaat taggatggcc 60
actattaaaa acataaaatt aataagcatt ggcaaggatg tagaaattag aacacctgtg 120
cactgttggt gggaatataa aatgatgcag ctggctttgc agacactgct gtcccccaac 180
acccccctgtc actaggccat ggtcatcccg actgtgccct tcaacatcac catcaacagc 240
aagcccttag gacacatctc ctttcagcta tttgcagaca aatttccaaa gacaggagaa 300
aactttcaca ctctgaacaa taaagacaaa ggatttggtt cctgctttca cagaattatt 360
ccggagttta tatgccaggg tgatgacttc acaccccata atggcattgg tggcaagtcc 420
atctacgggg ataaatttga tgataagaac tttattgtga agcatacagg tcttggcatc 480
ttgtccatgg caaatgctgc acccaaaaca aatgagtccc agtttttcat ctgcactgcc 540
atggccaaat ggtgggatgg caagcatgtg atctttggca gggtgaaaga gggcatgaat 600
attgtggaag ccatggaatg ctttgggtcc aggaatggca agacaagcaa gatcgccatt 660
gccaactgca gacaactctg ataaatttga cttgtgtttt atcttaacca ccagaccttt 720

<210> 96

<211> 160
<212> PRT
<213> Homo sapiens

<400> 96

<210> 97
<211> 600
<212> DNA
<213> Homo sapiens

<400> 97
ctgtgcactg ttggtgggaa tataaaatga tgcagctggc tttgcagaca ctgctgtccc 60
ccaacacccc ctgtcactag gccatggtca tcccgactgt gcccttcaac atcaccatca 120
acagcaagcc cttaggacac atctcctttc agctatttgc agacaaattt ccaaagacag 180
gagaaaactt tcacactctg aacaataaag acaaaggatt tggttcctgc tttcacagaa 240
ttattccgga gtttatatgc cagggtgatg acttcacacc ccataatggc attggtggca 300
agtccatcta cggggataaa tttgatgata agaactttat tgtgaagcat acaggtcttg 360
gcatcttgtc catggcaaat gctgcaccca aaacaaatga gtcccagttt ttcatctgca 420
ctgccatggc caaatggtgg gatggcaagc atgtgatctt tggcagggtg aaagagggca 480
tgaatattgt ggaagccatg gaatgctttg ggtccaggaa tggcaagaca agcaagatcg 540
ccattgccaa ctgcagacaa ctctgataaa tttgacttgt gttttatctt aaccaccaga 600

<210> 98
<211> 160
<212> PRT
<213> Homo sapiens

<400> 98

<210> 99
<211> 3146
<212> DNA
<213> Homo sapiens

<400> 99
aatttgaaaa tgaaggcact tttaccattg acctttctgt tttttattag ttctccaggt 60
tgggcaatag ataggcactg ctacataggc attgaagaaa gcatttggaa ctatgctaat 120
gctgatgaaa actttctcat gattgacact tgcaggacac atatgccatt atttctacaa 180
ggaggtcaag cgaggaagag ctttgttttt aaaaaggctt tgtattttca atatactgat 240
aatacatttc aaaggatcat tgaaaaacca tcctggttgg gatttttagg tccaatgatt 300
aaagcagaga ctggagactt catttatgta catgtaaaaa ataatgcttc aagagcttat 360
agttatcatc ctcatgggct cacctactcc aaagaaaatg aaggtgctat ctatcctgat 420
aatacgacag gcctgcaaaa ggaagatgaa tatctggagc cagggaaaca atatacctac 480
aagtggtatg tagaagaaca tcagggacct ggccccaatg acagtaattg tgtgacaaga 540
atttaccatt cccatataga cactgcaaga gatgtagctt cgggacttat tggaccaata 600
ctgacttgta aaagaggtac actgaatgga gacactgaaa aagatattga caggtcttct 660
tttctgatgt tttctacaac tgatgaaagc agaagctggt atagtgatga aaatattcgt 720
gcatttactg aatctggcaa gattaatact agtgatcccc gttttgagga gagcatgagc 780
atgcaagcaa taaatggata catctatgga aatctgccca atctcaccat gtgtgctgaa 840
gatagggtcc agtggtattt tgttggcatg ggtggcgtgg ctgacataca ccccgtctac 900
ctccgcggac aaactctgat ctctcggaat cacagaaagg acaccattat gctcttcccc 960
tcctcactgg aagatgcctt catggtggcc aaggcccctg gagtgtggat gctgggatgc 1020
cagatgcagg catttttcaa agtaagtaat tgccagaaac cttcaacaga agcctttgtt 1080
actgggacac atgttataca ttactatatt gctgctaaag aaattctttg gaactatgct 1140
ccatctggta tagatttctt cactaaaaaa aatttaacag cagctggaag taaatcccag 1200
ttattttttg aacgaagtcc aaccagaatt ggaggaacta acaaaaaact gatttaccgt 1260
gaatacacag atgcttcctt ccaaacacag aaggcaagag aagaacacct tggaatccta 1320
ggccccgtta ttaaggcaga ggtgagacag accatcaaaa tcactttcta taacaatgct 1380
tccctgccac tcagcattca gcctcctgga ctgcattaca acaagagctt gtggcagagt 1440
tattacttta gttcctattc aactgtcacc caaagagaaa gatctgttcc tccaccctct 1500
tcacatgtaa gtcctggcac aacatttgtc tatacatggg aagttccaaa agatgtgggt 1560
cccacctcca cagatcccaa ctgcttgacc tggttctatt actcttcagt aaatgggaaa 1620
aaagacatca acagtggcct tctggggcct ctccttatat gtagaaatgg aagtcttgga 1680
gacgatggca aacagaaagg agtagacaaa gagttttacc tacttgccac aatatttgat 1740
gaaaatgaaa gtaatctctt ggatgaaaat atcagaacat ttatcacaga gcctgaaaac 1800
atagataaag aggatacaga ctgccaagcc tcaaataaga tgtacgccat aaatggatac 1860
atgtatggaa atctgcctgg attggacacg tgcttaggag acaacgtttt gtggcacgtt 1920
tttagtgtag gatcagtgga agatttacac gggatatatt tttcaggaaa taccttcact 1980
tctttaggag caagaaggga cacaatacct atgtttcctt atacttctca gacgcttttg 2040
atgacacctg attctatagg tacttttgat ttggtttgca tgacaataaa gcacaatcta 2100

ggaggcatga aacataaata tcacgtgagg caatgtggga agccaaaccc tgatcaaaca 2160
caataccagg aggagaaaat aattattacc attgcagccg aggaaatgga atgggattat 2220
tctcctagta gaaagtggga gaatgaactc caccacttac gaagagagag ccaaacgagc 2280
atgtatgtgg acagaagtgg aacacttctt gggtccaaat acaagaaagt cttatatcgt 2340
caatatgatg ataacacgtc acaaatcaaa caaaaaggaa tgagggtgaa aaacatctcg 2400
atactaggtc cattaatatt gctcaaccct ggtcaaataa ttcaaattat ctttaaaaat 2460
aaagccgcaa gaccgtattc tattcatgct catggagtga aaacaaataa ttccactgtt 2520
gttccaactc agccaggtga gattcaaata tatacttggc agatacctga tagaactggt 2580
cctacctcac tggactttga atgcatacct tggttttact attcaactgt atctgtggct 2640
aaggaccttc acagtggact ggtaggccct ctctctgtat gccgcaaaga catcaacccc 2700
aacatagttc accgtgttct ccacttcatg atatttgatg agaatgaatc ctggtacttc 2760
gaagacagta tcaacaccta tgcttcaaaa ccaaacaaag tggacaagga aaatgataat 2820
tttcaactca gcaaccaaat gcacgcaatt aacggaagac tgtttggaaa taaccaaggt 2880
ataacattcc atgttgggga tgtagtgaat tggtatctga ttggcatagg gaatgaagct 2940
ggagtgtatc aatctgatgt ttatgacctt cctcctgggg tctatcgaac tgtaaaaatg 3000
tatcgaagag atgttggaac ctggttattt tattgccatg tttttgagca cattggtgct 3060
ggaatggaaa gcacttacac tgtacttgaa agaaaaggta agatccattg gctaaattaa 3120
ttagaagtga tatttaaaca aatgca 3146

<210> 100
<211> 1036
<212> PRT
<213> Homo sapiens

<400> 100
Met Lys Ala Leu Leu Pro Leu Thr Phe Leu Phe Phe Ile Ser Ser Pro
1               5                   10                  15

Gly Trp Ala Ile Asp Arg His Cys Tyr Ile Gly Ile Glu Glu Ser Ile
            20                  25                  30

Trp Asn Tyr Ala Asn Ala Asp Glu Asn Phe Leu Met Ile Asp Thr Cys
        35                  40                  45

Arg Thr His Met Pro Leu Phe Leu Gln Gly Gly Gln Ala Arg Lys Ser
    50                  55                  60

Phe Val Phe Lys Lys Ala Leu Tyr Phe Gln Tyr Thr Asp Asn Thr Phe
65                  70                  75                  80

Gln Arg Ile Ile Glu Lys Pro Ser Trp Leu Gly Phe Leu Gly Pro Met
                85                  90                  95

Ile Lys Ala Glu Thr Gly Asp Phe Ile Tyr Val His Val Lys Asn Asn
            100                 105                 110

Ala Ser Arg Ala Tyr Ser Tyr His Pro His Gly Leu Thr Tyr Ser Lys
        115                 120                 125

Glu Asn Glu Gly Ala Ile Tyr Pro Asp Asn Thr Thr Gly Leu Gln Lys
    130                 135                 140

Glu Asp Glu Tyr Leu Glu Pro Gly Lys Gln Tyr Thr Tyr Lys Trp Tyr
145                 150                 155                 160

Val Glu Glu His Gln Gly Pro Gly Pro Asn Asp Ser Asn Cys Val Thr
                165                 170                 175

Arg Ile Tyr His Ser His Ile Asp Thr Ala Arg Asp Val Ala Ser Gly
            180                 185                 190

Leu Ile Gly Pro Ile Leu Thr Cys Lys Arg Gly Thr Leu Asn Gly Asp
        195                 200                 205

Thr Glu Lys Asp Ile Asp Arg Ser Ser Phe Leu Met Phe Ser Thr Thr
    210                 215                 220

Asp Glu Ser Arg Ser Trp Tyr Ser Asp Glu Asn Ile Arg Ala Phe Thr

225                 230                 235                 240

Glu Ser Gly Lys Ile Asn Thr Ser Asp Pro Arg Phe Glu Glu Ser Met
                245                 250                 255

Ser Met Gln Ala Ile Asn Gly Tyr Ile Tyr Gly Asn Leu Pro Asn Leu
            260                 265                 270

Thr Met Cys Ala Glu Asp Arg Val Gln Trp Tyr Phe Val Gly Met Gly
        275                 280                 285

Gly Val Ala Asp Ile His Pro Val Tyr Leu Arg Gly Gln Thr Leu Ile
    290                 295                 300

Ser Arg Asn His Arg Lys Asp Thr Ile Met Leu Phe Pro Ser Ser Leu
305                 310                 315                 320

Glu Asp Ala Phe Met Val Ala Lys Ala Pro Gly Val Trp Met Leu Gly
                325                 330                 335

Cys Gln Met Gln Ala Phe Phe Lys Val Ser Asn Cys Gln Lys Pro Ser
            340                 345                 350

Thr Glu Ala Phe Val Thr Gly Thr His Val Ile His Tyr Tyr Ile Ala
        355                 360                 365

Ala Lys Glu Ile Leu Trp Asn Tyr Ala Pro Ser Gly Ile Asp Phe Phe
    370                 375                 380

Thr Lys Lys Asn Leu Thr Ala Ala Gly Ser Lys Ser Gln Leu Phe Phe
385                 390                 395                 400

Glu Arg Ser Pro Thr Arg Ile Gly Gly Thr Asn Lys Lys Leu Ile Tyr
                405                 410                 415

Arg Glu Tyr Thr Asp Ala Ser Phe Gln Thr Gln Lys Ala Arg Glu Glu
            420                 425                 430

His Leu Gly Ile Leu Gly Pro Val Ile Lys Ala Glu Val Arg Gln Thr
        435                 440                 445

Ile Lys Ile Thr Phe Tyr Asn Asn Ala Ser Leu Pro Leu Ser Ile Gln
    450                 455                 460

Pro Pro Gly Leu His Tyr Asn Lys Ser Leu Trp Gln Ser Tyr Tyr Phe
465                 470                 475                 480

Ser Ser Tyr Ser Thr Val Thr Gln Arg Glu Arg Ser Val Pro Pro Pro
                485                 490                 495

Ser Ser His Val Ser Pro Gly Thr Thr Phe Val Tyr Thr Trp Glu Val
            500                 505                 510

Pro Lys Asp Val Gly Pro Thr Ser Thr Asp Pro Asn Cys Leu Thr Trp
        515                 520                 525

Phe Tyr Tyr Ser Ser Val Asn Gly Lys Lys Asp Ile Asn Ser Gly Leu
    530                 535                 540

Leu Gly Pro Leu Leu Ile Cys Arg Asn Gly Ser Leu Gly Asp Asp Gly
545                 550                 555                 560

Lys Gln Lys Gly Val Asp Lys Glu Phe Tyr Leu Leu Ala Thr Ile Phe
                565                 570                 575

Asp Glu Asn Glu Ser Asn Leu Leu Asp Glu Asn Ile Arg Thr Phe Ile
            580                 585                 590

Thr Glu Pro Glu Asn Ile Asp Lys Glu Asp Thr Asp Cys Gln Ala Ser

        595                 600                 605

Asn Lys Met Tyr Ala Ile Asn Gly Tyr Met Tyr Gly Asn Leu Pro Gly
    610                 615                 620

Leu Asp Thr Cys Leu Gly Asp Asn Val Leu Trp His Val Phe Ser Val
625                 630                 635                 640

Gly Ser Val Glu Asp Leu His Gly Ile Tyr Phe Ser Gly Asn Thr Phe
                645                 650                 655

Thr Ser Leu Gly Ala Arg Arg Asp Thr Ile Pro Met Phe Pro Tyr Thr
            660                 665                 670

Ser Gln Thr Leu Leu Met Thr Pro Asp Ser Ile Gly Thr Phe Asp Leu
        675                 680                 685

Val Cys Met Thr Ile Lys His Asn Leu Gly Gly Met Lys His Lys Tyr
    690                 695                 700

His Val Arg Gln Cys Gly Lys Pro Asn Pro Asp Gln Thr Gln Tyr Gln
705                 710                 715                 720

Glu Glu Lys Ile Ile Ile Thr Ile Ala Ala Glu Glu Met Glu Trp Asp
                725                 730                 735

Tyr Ser Pro Ser Arg Lys Trp Glu Asn Glu Leu His His Leu Arg Arg
            740                 745                 750

Glu Ser Gln Thr Ser Met Tyr Val Asp Arg Ser Gly Thr Leu Leu Gly
        755                 760                 765

Ser Lys Tyr Lys Lys Val Leu Tyr Arg Gln Tyr Asp Asp Asn Thr Ser
    770                 775                 780

Gln Ile Lys Gln Lys Gly Met Arg Val Lys Asn Ile Ser Ile Leu Gly
785                 790                 795                 800

Pro Leu Ile Leu Leu Asn Pro Gly Gln Ile Ile Gln Ile Ile Phe Lys
                805                 810                 815

Asn Lys Ala Ala Arg Pro Tyr Ser Ile His Ala His Gly Val Lys Thr
            820                 825                 830

Asn Asn Ser Thr Val Val Pro Thr Gln Pro Gly Glu Ile Gln Ile Tyr
        835                 840                 845

Thr Trp Gln Ile Pro Asp Arg Thr Gly Pro Thr Ser Leu Asp Phe Glu
    850                 855                 860

Cys Ile Pro Trp Phe Tyr Tyr Ser Thr Val Ser Val Ala Lys Asp Leu
865                 870                 875                 880

His Ser Gly Leu Val Gly Pro Leu Ser Val Cys Arg Lys Asp Ile Asn
                885                 890                 895

Pro Asn Ile Val His Arg Val Leu His Phe Met Ile Phe Asp Glu Asn
            900                 905                 910

Glu Ser Trp Tyr Phe Glu Asp Ser Ile Asn Thr Tyr Ala Ser Lys Pro
        915                 920                 925

Asn Lys Val Asp Lys Glu Asn Asp Asn Phe Gln Leu Ser Asn Gln Met
    930                 935                 940

His Ala Ile Asn Gly Arg Leu Phe Gly Asn Asn Gln Gly Ile Thr Phe
945                 950                 955                 960

His Val Gly Asp Val Val Asn Trp Tyr Leu Ile Gly Ile Gly Asn Glu

                965                 970                 975

Ala Gly Val Tyr Gln Ser Asp Val Tyr Asp Leu Pro Pro Gly Val Tyr
            980                 985                 990

Arg Thr Val Lys Met Tyr Arg Arg Asp Val Gly Thr Trp Leu Phe Tyr
        995                 1000                1005

Cys His Val Phe Glu His Ile Gly Ala Gly Met Glu Ser Thr Tyr Thr
    1010                1015                1020

Val Leu Glu Arg Lys Gly Lys Ile His Trp Leu Asn
1025                1030                1035

<210> 101
<211> 857
<212> DNA
<213> Homo sapiens

<400> 101
aacaataaac ttctaacact cccttactaa aagaacatgg ttaagggtga gaaaggcccc 60
aagggcaaga agatcaccct caaggtggcc aggaattgca tcaaaatcac ttttgatggg 120
aaaaagcgcc ttgacttgag caagatggga attaccacct tccccaagtg tattctgcgc 180
cttagtgaca tggacgagct ggaccttagc cggaatctta tcaggaagat ccctgactcc 240
atctccaagt tccagaacct ccggtggctg gacctgcaca gcaactacat agacaagctg 300
cctgagtcca ttggccagat gaccagcctg ctctacctca acgtcagcaa caaccggctg 360
accagcaacg ggctgcccgt ggagctgaag caactcaaga acatccgcgc tgtgaaccta 420
ggcttgaacc acctggacag cgtgcccacc acactggggg ccctgaagga gctccacgag 480
gtagggctcc atgacaacct actgaacaac atccccgtga gcatctccaa gctccccaag 540
ctgaaaaagc tcaacataaa gcggaacccc tttccaaagc caggtgagtc ggaaatattc 600
atagactcca tcaggaggct ggagaacttg tatgttgtgg aggagaagga tctgtgtgcg 660
gcttgcctga gaaaatgcca aaacgcccgg gacaacctga atagaatcaa gaacatggcc 720
acgacgacac cgagaaagac catctttccc aatctgatct cacccaattc catggccaag 780
gactcctggg aagactggag gtgacttgga acctgaccct gaggcagaag ggaaagagag 840
agggagggaa gagggca 857

<210> 102
<211> 255
<212> PRT
<213> Homo sapiens

<400> 102

<210> 103
<211> 847
<212> DNA
<213> Homo sapiens

<400> 103
cttctaacac tcccttacta aaagaacatg gttaagggtg agaaaggccc caagggcaag 60
aagatcaccc tcaaggtggc caggaattgc atcaaaatca cttttgatgg gaaaaagcgc 120
cttgacttga gcaagatggg aattaccacc ttccccaagt gtattctgcg ccttagtgac 180
atggacgagc tggaccttag ccggaatctt atcaggaaga tccctgactc catctccaag 240
ttccagaacc tccggtggct ggacctgcac agcaactaca tagacaagct gcctgagtcc 300
attggccaga tgaccagcct gctctacctc aacgtcagca acaaccggct gaccagcaac 360
gggctgcccg tggagctgaa gcaactcaag aacatccgcg ctgtgaacct aggcttgaac 420
cacctggaca gcgtgcccac catgctgggg gccctgaagg agctccacga ggtagggctc 480
catgacaacc tactgaacaa catccccgtg agcatctcca agctccccaa gctgaaaaag 540
ctcaacataa agcggaaccc ctttccaaag ccaggtgagt cggaaatatt catagactcc 600
atcaggaggc tggagaactt gtatgttgtg gaggagaagg atctgtgtgc ggcttgcctg 660
agaaaatgcc aaaacgcccg ggacaacctg aatagaatca agaacatggc cacgacgaca 720
ccgagaaaga ccatctttcc caatctgatc tcacccaatt ccatggccaa ggactcctgg 780
gaagactgga ggtgacttgg aacctgagcc ctgaggcaga agggaaagag agagggaggg 840
aagaggg 847

<210> 104
<211> 255
<212> PRT
<213> Homo sapiens

<400> 104

<210> 105
<211> 4321
<212> DNA
<213> Homo sapiens

<400> 105
ctggtggggg gcggggtgac cctgtgacac ggacatgggg ctgctggggc aggatctctt 60
tgtcacctcc tttctgtgtc caacctggcc gtccccatca ggcgcccacg gcctgcgaga 120
ggagcccgag tttgtgacgg caagagctgg ggagagcgtg gtcctgcgat gcgacgtgat 180
ccacccagtg acgggacagc ccccacccta tgtcgtagag tggttcaagt tcggggtccc 240
catccctatc ttcatcaagt ttggctacta cccgccgcac gtggaccctg agtatgcagg 300
taaggtcggc gcccacggcc tgcgagagga gcccgagttt gtgacggcaa gagctgggga 360
gagcgtggtc ctgcgatgcg acgtgatcca cccagtgacg ggacagcccc caccctatgt 420
cgtagagtgg ttcaagttcg gggtccccat ccctatcttc atcaagtttg gctactaccc 480
gccgcacgtg gaccctgagt atgcaggtaa ggtcagtctt catgataagg catctctgcg 540
gctggaacaa gttcgctctg aggaccaggg ctggtatgag tgcaaagtgc tcatgctgga 600
ccagcagtat gacaccttcc acaatggcag ctgggtccac ctcaccatca acgcccctcc 660
cacctttaca gaaacacccc cccagtacat cgaggccaag gagggtggta gtatcaccat 720
gacctgcaca gcttttggga accccaagcc cattgtcacc tggctcaagg aggggacgct 780
cctcggtgct agtgggaaat accaggtgag tgtggttcta ggtagcctga cagtgacatc 840
ggtcagtcgg gaggacagag gtgcctacac ctgccgagcg tacagcattc agggggaggc 900
tgtccacacg actcacctgc ttgtccaagg gcccccttc atcgtctccc ctcctgagaa 960
catcaccgtc aacatctccc aggatgctct gctcacctgc cgggcagagg cgtatccggg 1020
caacctcacc tacacctggt actggcagga cgagaacgtc tactttcaga acgacctgaa 1080
gctgagggtg cgcatcctaa tcgatgggac cctgatcatc ttccgggtga agccggagga 1140
ctcagggaag tacacctgtg tgcccagcaa cagcctgggg cgctccccct ccgcctcggc 1200
gtacctgacc gtgcagtacc cagcgcgtgt cctcaacatg cccccctgtga tttacgtgcc 1260
cgtggggatc catggctaca tccgctgccc tgtggacgca gaaccaccgg ccaccgtggt 1320
caagtggaac aaggacggcc gtcccctgca ggttgagaag aacctcggtt ggaccctgat 1380
ggaggatggc tccattcgaa ttgaggaggc cacagaggag gctcttggca cttatacctg 1440
tgtgccttac aacactctgg ggaccatggg ccagtctgcc cctgcgaggc ttgtcctgaa 1500
ggaccccccc tatttcacgg tgctaccagg ctgggagtac aggcaggagg ccggccggga 1560
gctacttatc ccctgtgctg ccgcagggga cccctttcct gtcatcactt ggagaaaggt 1620
agggaagccc agcagaagca agcacagtgc cctgcccagt gggagcctgc agttccgtgc 1680
cctgagtaag gaggaccacg ggagtgggga atgtgtcgcc accaacgtgg tcacgagcat 1740
cactgccagc acccacctca ccgtcatcgg tacgggcacc agcccccatg ccccgggcag 1800

tgtccgggtc caggtctcca tgacaactgc caacgtgtcc tgggaaccag gctatgatgg 1860
aggctacgag cagacattct cagtttggta cggacctctg atgaagcggg cacagtttgg 1920
gccccatgac tggctgtcct tgccagtgcc gccaggaccc agctggctgc tggtggacac 1980
cctggagcct gagacagcgt accagttcag cgtcctggcc cagaagctgg gaaccagcgc 2040
cttcagtgag gtggtcactg tgaacacttt agcattccct attacaactc cagaacccct 2100
ggtgctggtc accccaccga ggtgcctcat agccaatcgg actcagcagg gtgtgctcct 2160
gtcctggctt ccgcctgcca accacagctt tcccatcgac cgctacatca tggagttccg 2220
tgtcgcagag cgctgggagt tgctcgacga tggcatcccc ggcaccgaag gagagttctt 2280
tgccaaggat ctgtcacagg acacgtggta tgagttccgg gttctggccg tcatgcagga 2340
tctgatcggc gagcccagca acatcgccgg cgtctccagc acagacatct tcccgcagcc 2400
ggacctgacc gaggatgggc tggcgcggcc tgtgctggcg ggaatcgtag ctaccatctg 2460
cttcttggca gctgccatcc tgttcagcac cctggctgcc tgctttgtca acaagcagcg 2520
caagcgtaag ctcaagcgca aaaaagaccc tccactctcc atcacccact gcaggaagag 2580
cctggagtct cccttgtcct ctggcaaggt gagccccgag agcatccgca cgctccgagc 2640
gccgtcagaa tcctccgacg accagggcca gcccgcggcc aagaggatgc tgagccccac 2700
ccgtgagaag gagctgtcgc tgtacaagaa gaccaagcgg gccatcagca gcaagaagta 2760
cagcgtggcc aaggcagagg ccgaggcaga ggccaccacg cccatcgagc tcatcagcag 2820
aggccctgac ggccgcttcg tgatggaccc tgtcgagatg gagccctcgc tgaagagcag 2880
gcgcatcgag ggcttcccct tcgccgagga gacggacatg taccccgagt tccgccagtc 2940
ggacgaggag aacgaggacc cactggtgcc cacatctgtg gccgccctga agtcccagct 3000
cacccctctg tcatccagcc aggagtccta cctgccacca ccagcataca gccctcggtt 3060
ccagccccgc gggctggagg gccccggtgg cctggaaggt cggcttcagg ccacaggcca 3120
ggcccggccc cctgcccccc ggcccttcca ccatggccag tattatgggt acctcagcag 3180
cagcagccct ggggaggtgg agccgccccc gttctacgtg ccagaagtgg gcagccccct 3240
gagctccgtc atgtcgtccc cgcccctgcc caccgagggg ccctttggcc accccaccat 3300
ccccgaggag aatggagaga atgcatccaa cagcacgctg cccttgactc agacacctac 3360
aggagggcgc tcccctgagc cctggggccg gccagaattc cccttcgggg ggctggagac 3420
cccagcgatg atgttccccc accagctgcc accctgtgat gtgcccgaga gtctgcagcc 3480
caaggccggc ctcccccgag gactgccccc cacctccctg caggtgcccg cggcctaccc 3540
gggcatcctg tctctggagg caccgaaggg ttgggcaggc aagtcgcccg gcaggggccc 3600
tgtcccagcg ccccccgccg ccaagtggca ggacagacct atgcaacctc tggtaagcca 3660
agggcagctg cgacatacaa gccaaggcat gggcatacct gtgctgcctt accccgagcc 3720
ggctgagccg gggggcgcacg gcggccccag cacatttggc ctggacaccc ggtggtatga 3780
gccccagccc cggccccggc ctagccctcg gcaggccagg cgcgccgagc ccagtttaca 3840
tcaagtggtg ctacagccct cccggctctc acctctgacc caaagccccc tcagctcccg 3900
caccggctcc cctgagctcg ccgcccgtgc ccggcctcgc ccgggcctcc tgcagcaggc 3960
agagatgtca gagatcaccc tgcagccgcc ggctgcagtc agcttttctc gaaagtctac 4020
gccgtccaca ggctccccct cccagagcag ccgcagtggg agtcccagct accggcccgc 4080
catgggcttc accactctgg ccaccggcta cccttcccct ccacccggcc ccgcccctgc 4140
tgggcctggg gacagcttgg acgtgtttgg acagacgcct tcccctcgaa ggacggggga 4200
ggaattgctc cgaccggaga ccccaccacc cacgttacct acttcaggga agctgcggag 4260
agacagacca gctcccgcga ccagcccgcc tgagagagca ctctctaaac tgtagcagct 4320
g 4321

<210> 106
<211> 1426
<212> PRT
<213> Homo sapiens

<400> 106
Met Gly Leu Leu Gly Gln Asp Leu Phe Val Thr Ser Phe Leu Cys Pro
1               5                   10                  15

Thr Trp Pro Ser Pro Ser Gly Ala His Gly Leu Arg Glu Glu Pro Glu
            20                  25                  30

Phe Val Thr Ala Arg Ala Gly Glu Ser Val Val Leu Arg Cys Asp Val
        35                  40                  45

Ile His Pro Val Thr Gly Gln Pro Pro Pro Tyr Val Val Glu Trp Phe
    50                  55                  60

Lys Phe Gly Val Pro Ile Pro Ile Phe Ile Lys Phe Gly Tyr Tyr Pro
65                  70                  75                  80

Pro His Val Asp Pro Glu Tyr Ala Gly Lys Val Gly Ala His Gly Leu
                85                  90                  95

Arg Glu Glu Pro Glu Phe Val Thr Ala Arg Ala Gly Glu Ser Val Val
            100                 105                 110

Leu Arg Cys Asp Val Ile His Pro Val Thr Gly Gln Pro Pro Pro Tyr
        115                 120                 125

Val Val Glu Trp Phe Lys Phe Gly Val Pro Ile Pro Ile Phe Ile Lys
    130                 135                 140

Phe Gly Tyr Tyr Pro Pro His Val Asp Pro Glu Tyr Ala Gly Lys Val
145                 150                 155                 160

Ser Leu His Asp Lys Ala Ser Leu Arg Leu Glu Gln Val Arg Ser Glu
                165                 170                 175

Asp Gln Gly Trp Tyr Glu Cys Lys Val Leu Met Leu Asp Gln Gln Tyr
            180                 185                 190

Asp Thr Phe His Asn Gly Ser Trp Val His Leu Thr Ile Asn Ala Pro
        195                 200                 205

Pro Thr Phe Thr Glu Thr Pro Pro Gln Tyr Ile Glu Ala Lys Glu Gly
    210                 215                 220

Gly Ser Ile Thr Met Thr Cys Thr Ala Phe Gly Asn Pro Lys Pro Ile
225                 230                 235                 240

Val Thr Trp Leu Lys Glu Gly Thr Leu Leu Gly Ala Ser Gly Lys Tyr
                245                 250                 255

Gln Val Ser Val Val Leu Gly Ser Leu Thr Val Thr Ser Val Ser Arg
            260                 265                 270

Glu Asp Arg Gly Ala Tyr Thr Cys Arg Ala Tyr Ser Ile Gln Gly Glu
        275                 280                 285

Ala Val His Thr Thr His Leu Leu Val Gln Gly Pro Pro Phe Ile Val
    290                 295                 300

Ser Pro Pro Glu Asn Ile Thr Val Asn Ile Ser Gln Asp Ala Leu Leu
305                 310                 315                 320

Thr Cys Arg Ala Glu Ala Tyr Pro Gly Asn Leu Thr Tyr Thr Trp Tyr
                325                 330                 335

Trp Gln Asp Glu Asn Val Tyr Phe Gln Asn Asp Leu Lys Leu Arg Val
            340                 345                 350

Arg Ile Leu Ile Asp Gly Thr Leu Ile Ile Phe Arg Val Lys Pro Glu
        355                 360                 365

Asp Ser Gly Lys Tyr Thr Cys Val Pro Ser Asn Ser Leu Gly Arg Ser
    370                 375                 380

Pro Ser Ala Ser Ala Tyr Leu Thr Val Gln Tyr Pro Ala Arg Val Leu
385                 390                 395                 400

Asn Met Pro Pro Val Ile Tyr Val Pro Val Gly Ile His Gly Tyr Ile
                405                 410                 415

Arg Cys Pro Val Asp Ala Glu Pro Pro Ala Thr Val Val Lys Trp Asn
            420                 425                 430

Lys Asp Gly Arg Pro Leu Gln Val Glu Lys Asn Leu Gly Trp Thr Leu
        435                 440                 445

Met Glu Asp Gly Ser Ile Arg Ile Glu Glu Ala Thr Glu Glu Ala Leu
    450                 455                 460

Gly Thr Tyr Thr Cys Val Pro Tyr Asn Thr Leu Gly Thr Met Gly Gln
465                 470                 475                 480

Ser Ala Pro Ala Arg Leu Val Leu Lys Asp Pro Pro Tyr Phe Thr Val
                485                 490                 495

Leu Pro Gly Trp Glu Tyr Arg Gln Glu Ala Gly Arg Glu Leu Leu Ile
            500                 505                 510

Pro Cys Ala Ala Ala Gly Asp Pro Phe Pro Val Ile Thr Trp Arg Lys
        515                 520                 525

Val Gly Lys Pro Ser Arg Ser Lys His Ser Ala Leu Pro Ser Gly Ser
    530                 535                 540

Leu Gln Phe Arg Ala Leu Ser Lys Glu Asp His Gly Glu Trp Glu Cys
545                 550                 555                 560

Val Ala Thr Asn Val Val Thr Ser Ile Thr Ala Ser Thr His Leu Thr
                565                 570                 575

Val Ile Gly Thr Gly Thr Ser Pro His Ala Pro Gly Ser Val Arg Val
            580                 585                 590

Gln Val Ser Met Thr Thr Ala Asn Val Ser Trp Glu Pro Gly Tyr Asp
        595                 600                 605

Gly Gly Tyr Glu Gln Thr Phe Ser Val Trp Tyr Gly Pro Leu Met Lys
    610                 615                 620

Arg Ala Gln Phe Gly Pro His Asp Trp Leu Ser Leu Pro Val Pro Pro
625                 630                 635                 640

Gly Pro Ser Trp Leu Leu Val Asp Thr Leu Glu Pro Glu Thr Ala Tyr
                645                 650                 655

Gln Phe Ser Val Leu Ala Gln Lys Leu Gly Thr Ser Ala Phe Ser Glu
            660                 665                 670

Val Val Thr Val Asn Thr Leu Ala Phe Pro Ile Thr Thr Pro Glu Pro
        675                 680                 685

Leu Val Leu Val Thr Pro Pro Arg Cys Leu Ile Ala Asn Arg Thr Gln
    690                 695                 700

Gln Gly Val Leu Leu Ser Trp Leu Pro Pro Ala Asn His Ser Phe Pro
705                 710                 715                 720

Ile Asp Arg Tyr Ile Met Glu Phe Arg Val Ala Glu Arg Trp Glu Leu
                725                 730                 735

Leu Asp Asp Gly Ile Pro Gly Thr Glu Gly Glu Phe Phe Ala Lys Asp
            740                 745                 750

Leu Ser Gln Asp Thr Trp Tyr Glu Phe Arg Val Leu Ala Val Met Gln
        755                 760                 765

Asp Leu Ile Gly Glu Pro Ser Asn Ile Ala Gly Val Ser Ser Thr Asp
    770                 775                 780

Ile Phe Pro Gln Pro Asp Leu Thr Glu Asp Gly Leu Ala Arg Pro Val
785                 790                 795                 800

Leu Ala Gly Ile Val Ala Thr Ile Cys Phe Leu Ala Ala Ala Ile Leu
                805                 810                 815

Phe Ser Thr Leu Ala Ala Cys Phe Val Asn Lys Gln Arg Lys Arg Lys
            820                 825                 830

Leu Lys Arg Lys Lys Asp Pro Pro Leu Ser Ile Thr His Cys Arg Lys
        835                 840                 845

Ser Leu Glu Ser Pro Leu Ser Ser Gly Lys Val Ser Pro Glu Ser Ile
    850                 855                 860

Arg Thr Leu Arg Ala Pro Ser Glu Ser Ser Asp Asp Gln Gly Gln Pro
865                 870                 875                 880

Ala Ala Lys Arg Met Leu Ser Pro Thr Arg Glu Lys Glu Leu Ser Leu
                885                 890                 895

Tyr Lys Lys Thr Lys Arg Ala Ile Ser Ser Lys Lys Tyr Ser Val Ala
            900                 905                 910

Lys Ala Glu Ala Glu Ala Glu Ala Thr Thr Pro Ile Glu Leu Ile Ser
        915                 920                 925

Arg Gly Pro Asp Gly Arg Phe Val Met Asp Pro Val Glu Met Glu Pro
    930                 935                 940

Ser Leu Lys Ser Arg Arg Ile Glu Gly Phe Pro Phe Ala Glu Glu Thr
945                 950                 955                 960

Asp Met Tyr Pro Glu Phe Arg Gln Ser Asp Glu Glu Asn Glu Asp Pro
                965                 970                 975

Leu Val Pro Thr Ser Val Ala Ala Leu Lys Ser Gln Leu Thr Pro Leu
            980                 985                 990

Ser Ser Ser Gln Glu Ser Tyr Leu Pro Pro Pro Ala Tyr Ser Pro Arg
        995                 1000                1005

Phe Gln Pro Arg Gly Leu Glu Gly Pro Gly Gly Leu Glu Gly Arg Leu
    1010                1015                1020

Gln Ala Thr Gly Gln Ala Arg Pro Pro Ala Pro Arg Pro Phe His His
1025                1030                1035                1040

Gly Gln Tyr Tyr Gly Tyr Leu Ser Ser Ser Ser Pro Gly Glu Val Glu
                1045                1050                1055

Pro Pro Pro Phe Tyr Val Pro Glu Val Gly Ser Pro Leu Ser Ser Val
            1060                1065                1070

Met Ser Ser Pro Pro Leu Pro Thr Glu Gly Pro Phe Gly His Pro Thr
        1075                1080                1085

Ile Pro Glu Glu Asn Gly Glu Asn Ala Ser Asn Ser Thr Leu Pro Leu
    1090                1095                1100

Thr Gln Thr Pro Thr Gly Gly Arg Ser Pro Glu Pro Trp Gly Arg Pro
1105                1110                1115                1120

Glu Phe Pro Phe Gly Gly Leu Glu Thr Pro Ala Met Met Phe Pro His
                1125                1130                1135

Gln Leu Pro Pro Cys Asp Val Pro Glu Ser Leu Gln Pro Lys Ala Gly
            1140                1145                1150

Leu Pro Arg Gly Leu Pro Pro Thr Ser Leu Gln Val Pro Ala Ala Tyr
        1155                1160                1165

Pro Gly Ile Leu Ser Leu Glu Ala Pro Lys Gly Trp Ala Gly Lys Ser
    1170                1175                1180

Pro Gly Arg Gly Pro Val Pro Ala Pro Pro Ala Ala Lys Trp Gln Asp
1185                1190                1195                1200

Arg Pro Met Gln Pro Leu Val Ser Gln Gly Gln Leu Arg His Thr Ser
                1205                1210                1215

Gln Gly Met Gly Ile Pro Val Leu Pro Tyr Pro Glu Pro Ala Glu Pro
            1220                1225                1230

Gly Ala His Gly Gly Pro Ser Thr Phe Gly Leu Asp Thr Arg Trp Tyr
        1235                1240                1245

Glu Pro Gln Pro Arg Pro Arg Pro Ser Pro Arg Gln Ala Arg Arg Ala
    1250                1255                1260

Glu Pro Ser Leu His Gln Val Val Leu Gln Pro Ser Arg Leu Ser Pro
1265                1270                1275                1280

Leu Thr Gln Ser Pro Leu Ser Ser Arg Thr Gly Ser Pro Glu Leu Ala
                1285                1290                1295

Ala Arg Ala Arg Pro Arg Pro Gly Leu Leu Gln Gln Ala Glu Met Ser
            1300                1305                1310

Glu Ile Thr Leu Gln Pro Pro Ala Ala Val Ser Phe Ser Arg Lys Ser
        1315                1320                1325

Thr Pro Ser Thr Gly Ser Pro Ser Gln Ser Ser Arg Ser Gly Ser Pro
    1330                1335                1340

Ser Tyr Arg Pro Ala Met Gly Phe Thr Thr Leu Ala Thr Gly Tyr Pro
1345                1350                1355                1360

Ser Pro Pro Pro Gly Pro Ala Pro Ala Gly Pro Gly Asp Ser Leu Asp
                1365                1370                1375

Val Phe Gly Gln Thr Pro Ser Pro Arg Arg Thr Gly Glu Glu Leu Leu
            1380                1385                1390

Arg Pro Glu Thr Pro Pro Pro Thr Leu Pro Thr Ser Gly Lys Leu Arg
        1395                1400                1405

Arg Asp Arg Pro Ala Pro Ala Thr Ser Pro Pro Glu Arg Ala Leu Ser
    1410                1415                1420

Lys Leu
1425


<210> 107
<211> 7097
<212> DNA
<213> Homo sapiens

<400> 107
atggggctgc tggggcagga tctctttgtc acctcctttc tgtgtccaac ctggccgtcc 60
ccatcaggcg cccacggcct gcgagaggaa cccgagtttg tgacggcaag agctggggag 120
agcgtggtcc tgcgatgcga cgtgatccac ccagtgacgg gacagccccc accctatgtc 180
gtagagtggt tcaagttcgg ggtccccatc cctatcttca tcaagtttgg ctactacccc 240
ccacacgtgg accctgagta tgcaggccgg gccagtcttc atgataaggc atctctgcgg 300
ctggaacaag ttcgctctga ggacctgggc tggtatgagt gcaaagtgct catgctggac 360
cagcagtatg acaccttcca caatggcagc tgggtccacc tcaccatcaa cgcccctccc 420
acctttacag aaacaccccc ccggtacatc gaggccaagg agggtggtag tatcaccatg 480
acctgcacag cttttgggaa ccccaagccc attgtcacct ggctcaagga ggggacgctc 540
ctcggtgcta gtgggaaata ccaggtgagt gacggcagcc tgacagtgac atcggtcagt 600
cgggaggaca gaggtgccta cacctgccga gcgtacagca ttcaggggga ggctgtccac 660
acgactcacc tgcttgtccc agggccccct ttcatcgtct cccctcctga gaacatcacc 720
gtcaacatct cccaggatgc tctgctcacc tgccgggcag aggcgtatcc gggcaacctc 780
acctacacct ggtactggca ggacgagaac gtctactttc agaacgacct gaagctgagg 840
gtgcgcatcc taatcgatgg gaccctgatc atcttccggg tgaagccgga ggactcgggg 900
aagtacacct gtgtgcccag caacagcctg ggcgctcccc cctccgcctc ggcgtacctg 960
accgtgcagt acccagcgcg tgtcctcaac atgccccctg tgatttacgt gcccgtgggg 1020

atccatggct acatccgctg ccctgtggac gcaagaccac cggccaccgt ggtcaagtgg 1080
aacaaggacg gccgtcccct gcaggttgag aagaaccgcg gttggaccct gatggaggat 1140
ggctccattc gaattgagga ggccacagag gaggctcttg gcacttatac ctgtgtgcct 1200
tacaacactc tggggaccat gggccagtct gcccctgcga ggcttgtcct gaaggacccc 1260
ccctatttca cggtgctacc aggctgggag tacaggcagg aggccggccg ggagctactt 1320
atcccctgtg ctgccgcagg ggaccccttt cctgtcatca cttggagcaa ggtagggaag 1380
cccagcagaa gcaagcacag tgccctgccc agtgggagcc tgcagttccg tgccctgagt 1440
aaggaggacc acggggagtg ggaatgtgtc gccaccaacg tggtcacgag catcactgcc 1500
agcacccacc tcaccgtcat cggcaccagc ccccatgccc cgggcagtgt ccgggtccag 1560
gtctccatga caactgccaa cgtgtcctgg gaaccaggtg acgggctacg atggggctat 1620
gatggaggct acgagcagac attctcagtt tggatgaagc gggcacagtt tgggccccat 1680
gactggctgt ccttgccagt gccgccagga cccagctggc tgctggtgga caccctggag 1740
cctgagacag cgtaccagtt cagcgtcctg gcccagaaca agctgggaac cagcgccttc 1800
agtgaggtgg tcactgtgat cactttagca ttccctatta caactccaga acccctggtg 1860
ctggtcaccc caccgaggtg cctcatagcc aatcggactc agcagggtgt gctcctgtcc 1920
tggcttccgc ctgccaacca cagctttccc atcgaccgct acatcatgga gttccgtgtc 1980
gcagagcgct gggagttgct cgacgatggc atccccggca ccgaaggaga gttctttgcc 2040
aaggatctgt cacaggacac gtggtatgag ttccgggttc tggccgtcat gcaggatctg 2100
atcggcgagc ccagcaacat cgccggcgtc tccagcacag acatcttccc gcagccggac 2160
ctgaccgagg atgggctggc gcggcctgtg ctggcgggaa tcgtagctac catctgcttc 2220
ttggcagctg ccatcctgtt cagcaccctg gctgcctgct ttgtcaacaa gcagcgcaag 2280
cgtaagctca agcgcaaaaa agaccctcca ctctccatca cccactgcag gaagagcctg 2340
gagtctccct tgtcctctgg caaggtgagc cccgagagca tccgcacgct ccgagcgccg 2400
tcagaatcct ccgacgacca gggccagccc gcggccaaga ggatgctgag ccccacccgt 2460
gagaaggagc tgtcgctgta caagaagacc aagcgggcca tcagcagcaa gaagtacagc 2520
gtggccaagg cagaggccga ggcagaggcc accacgccca tcgagctcat cagcagaggc 2580
cctgacggcc gcttcgtgat ggaccctgtc gagatggagc cctcgctgaa gagcaggcgc 2640
atcgagggct tccccttcgc cgaggagacg gacatgtacc ccgagttccg ccagtcggac 2700
gaggagaacg aggacccact ggtgcccaca tctgtggccg ccctgaagtc ccagctcacc 2760
cctctgtcat ccagccagga gtcctacctg ccaccaccag catacagccc tcggttccag 2820
ccccgcgggc tggagggccc cggtggcctg gaaggtcggc ttcaggccac aggccaggcc 2880
cggcccccctg cccccggcc cttccaccat ggccagtatt atgggtacct cagcagcagc 2940
agccctgggg aggtggagcc gcccccgttc tacgtgccag aagtgggcag ccccctgagc 3000
tccgtcatgt cgtccccgcc cctgcccacc gagggggccct ttggccaccc caccatcccc 3060
gaggagaatg gagagaatgc atccaacagc acgctgccct tgactcagac acctacagga 3120
gggcgctccc ctgagccctg ggccggccca gaattcccct tcggggggct ggagacccca 3180
gcgatgatgt tcccccacca gctgccaccc tgtgatgtgc ccgagagtct gcagcccaag 3240
gccggcctcc cccgaggact gccccccacc tccctgcagg tgcccgcggc ctacccgggc 3300
atcctgtctc tggaggcacc gaagggttgg gcaggcaagt cgcccggcag gggccctgtc 3360
ccagcgcccc ccgccgccaa gtggcaggac agacctatgc aacctctggt aagccaaggg 3420
cagctgcgac atacaagcca aggcatgggc atacctgtgc tgccttaccc cgagccggct 3480
gagccggggg cgcacggcgg ccccagcaca tttggcctgg acacccggtg gtatgagccc 3540
cagccccggc cccggcctag ccctcggcag gccaggcgcg ccgagcccag tttacatcaa 3600
gtggtgctac agccctcccg gctctcacct ctgacccaaa gcccccctcag ctcccgcacc 3660
ggctcccctg agctcgccgc ccgtgcccgg cctcgcccgg gcctcctgca gcaggcagag 3720
atgtcagaga tcaccctgca gccgccggct gcagtcagct tttctcgaaa gtctacgccg 3780
tccacaggct ccccctccca gagcagccgc agtgggagtc ccagctaccg gcccgccatg 3840
ggcttcacca ctctggccac cggctaccct tcccctccac ccggccccgc ccctgctggg 3900
cctggggaca gcttggacgt gtttggacag acgccttccc ctcgaaggac gggggaggaa 3960
ttgctccgac cggagacccc accacccacg ttacctactt cagggaagct gcagagagac 4020
agaccagctc ccgcgaccag cccgcctgag agagcactct ctaaactgta gcagctggta 4080
ttccagctat ctgggcagtg ttgtcgagac aagcctctcc tcagctcaat aggtaaggga 4140
gctcctcggc tgggcgggcg ggggcgggca ggcggacggg gcttcggccg ggccattgct 4200
tcctggacag gggatccaaa ccatgtcccc tcaccgcccc gtggggtggc cgctgccgct 4260
cctcgatccc cacggcttct gggttcccca catcgagcca cgctcggcac cgcctagctg 4320
cagcttctgc cccccacccc ccgtccccat gtccggcccc tctagagccc cctgcggctc 4380
tcctcactcc tcgccagcct cgccagcctg ctgccagtac acccagggcc tcccacagaa 4440
gccctggggc cctgcatgca ccccgaaggg gcccagaaca cccgagatct gctttgcatc 4500
tctgcaccct tggggacctc tctggggccc ctctggtatc caggaagagc ccccccgcac 4560
cctattctcc cagcagcccc aggcataaca tcccttctcc tggggtggct ggcccctcac 4620
cagcttcttg agatcttcgt ggtattaggc tctctcgagg agtacaaggt tgggatgagc 4680
cccacttctt ccttgacagt ggggtcctct gcagggtcag gtccctgcag tcctgtgggg 4740
ctttgtagcg aaatgtcatc actcccctgt gctccttgcc ttctgcagcc tgaccttggt 4800
agtcacagga ctgaaatgtg acctagccct tgggtccaca ttgctttcaa ggtccctgtt 4860
gtagccttgt cgtcctccct gggtcatgct gtagggaaag ggtcttagga ggacctcccc 4920
gaggggaggg ggcaggcttc ccctgggcag acaggcattg agctggcagg aagtgaaacc 4980
cccagggacc agcactgtgt ccctccccct gcccccgcag tcagcctctc cttgagtgtc 5040
cttccaggtg gaagctaaag gaacttgggc attcagggag cctctgagtc cctcacagag 5100
actcagggcc acccgaagct cgctccctct cacagtagcc tgacaaggtg ctgccgctcg 5160

ccacggaccc tctgccctgt gcctgggcac acacaggcat cgggcacctg catgggagac 5220
ggcgagcctc ccgtccaggt gcttctggct ttccaggcga gaaggagaca ggtgccttcc 5280
ccccctagag atgtcaaggg aggttcactt tcctaaccag ggctataaat ctcattcatt 5340
cctaagagtg gcctccataa agaggactgc cctgacattt ctttaccatc tgtagctata 5400
aaattgtcag cagcccagag gccctggaat tgtgggtgag gctgtctggc atagggtgac 5460
tgtgagggct gtcaggcagc gtttatgagc tcacctgctc tggggccctc ctgcgcccag 5520
agacccactg gtcttccacc ttccccagct ccctccctgt cccagctgtg actcctccac 5580
agcccccggg aggtgtggga gccctagact agctctcacc ccagctcctg gaaattccta 5640
gactctgctc tcatgtgtta ttcctcaccc cttcactcaa ctccattgac cctccccttt 5700
cccagtgtcc ccactgtgcc agatccaaga gaagcccgct ctcctttcac tgctgtggca 5760
aacccagaaa ccaggggcag cgatgaggga ccatcgtttc tctcccgggc ctggccagca 5820
tccccagcct aggagaagga gaccctcccc atcctgagtc agcccctcgg tgctggcctc 5880
tcctgcctgc tgggagcctc cccggtacgg ctgctgggtc ctggggagat gcaggctctg 5940
ttcagatgct gtctccatgc tgcacctttg catgtgtgcc ctcttggtct tgcttggaga 6000
agttgtgaca gctcttgtcc acgtgcgttc tcactgtttc ttttcccttc agttcctctc 6060
ctgttccttt ggtccatatg tagttgttgc ccctgcctct ccttttctct ctcccttttt 6120
ttctcctctt cccaactccc ttctcaacat ggggagttgt attgcgtgga cctgtccttt 6180
ctgtggactg tggggaggcc gagccaattc ttagctcctg tctgagccta gtgatgccct 6240
gtcctcacct ctgcttccac tccctttccc attcaggctg actgtcaccg cagggaggca 6300
ggtgggtctg gagcatggag tggcccctgt cctgggtcag ctgtaggaag ggcccctttt 6360
gtaaacaagc ttctgccagg ttctgaggca gagcactggt ctgagtccgg ccccacagaa 6420
gggttccatc ttgtggtcag cggggtgggc tcagacctgc agactcccgc agtgggccca 6480
ccctttccct tgctatggcc cgtctatgag tggctttccc ctgcctccat cggtgtataa 6540
tcaggcaggt ggtgtttgct cttaggtgat tagaagagga ggaaaatgcc catcaagcca 6600
ctgcttcagc ctgttggcca gggaacacct gaacagggtc aaaatggtca gctcctgggc 6660
gctgagctaa ggaaatatgc aggggggtttt gactttccct gtcctgaggc ttgcctccac 6720
tttgacctgg ctgcatcttt cctcagctct tctgggtaag ctgagagcta agcaaataag 6780
acctcctggg ctacctggtc atgatttggg ttttgtatgg atttttgaa aagagagaaa 6840
aagagtgtgg gctgggtgcc gtggcccaga gggcagatct cttgagtcca ggagttcaag 6900
gccagcctgg gcaacatagt gaagccccat ctctgcaaaa aaatacaaaa aaatagctgg 6960
gcatggtggc acatgcctat agtcccagct ccttgggggg ctgaggtggg aagatcatct 7020
gagctgggga ggtcgaggca gcagtgagcc gagattgcgc cacttcactc cagcctgggt 7080
gacagagatc ctgtctc 7097

<210> 108
<211> 1356
<212> PRT
<213> Homo sapiens

<400> 108
Met Gly Leu Leu Gly Gln Asp Leu Phe Val Thr Ser Phe Leu Cys Pro
1               5                   10                  15

Thr Trp Pro Ser Pro Ser Gly Ala His Gly Leu Arg Glu Glu Pro Glu
            20                  25                  30

Phe Val Thr Ala Arg Ala Gly Glu Ser Val Val Leu Arg Cys Asp Val
        35                  40                  45

Ile His Pro Val Thr Gly Gln Pro Pro Pro Tyr Val Val Glu Trp Phe
    50                  55                  60

Lys Phe Gly Val Pro Ile Pro Ile Phe Ile Lys Phe Gly Tyr Tyr Pro
65                  70                  75                  80

Pro His Val Asp Pro Glu Tyr Ala Gly Arg Ala Ser Leu His Asp Lys
                85                  90                  95

Ala Ser Leu Arg Leu Glu Gln Val Arg Ser Glu Asp Leu Gly Trp Tyr
            100                 105                 110

Glu Cys Lys Val Leu Met Leu Asp Gln Gln Tyr Asp Thr Phe His Asn
        115                 120                 125

Gly Ser Trp Val His Leu Thr Ile Asn Ala Pro Pro Thr Phe Thr Glu
    130                 135                 140

Thr Pro Pro Arg Tyr Ile Glu Ala Lys Glu Gly Gly Ser Ile Thr Met

145                 150                 155                 160

Thr Cys Thr Ala Phe Gly Asn Pro Lys Pro Ile Val Thr Trp Leu Lys
                165                 170                 175

Glu Gly Thr Leu Leu Gly Ala Ser Gly Lys Tyr Gln Val Ser Asp Gly
            180                 185                 190

Ser Leu Thr Val Thr Ser Val Ser Arg Glu Asp Arg Gly Ala Tyr Thr
        195                 200                 205

Cys Arg Ala Tyr Ser Ile Gln Gly Glu Ala Val His Thr Thr His Leu
    210                 215                 220

Leu Val Pro Gly Pro Pro Phe Ile Val Ser Pro Pro Glu Asn Ile Thr
225                 230                 235                 240

Val Asn Ile Ser Gln Asp Ala Leu Leu Thr Cys Arg Ala Glu Ala Tyr
                245                 250                 255

Pro Gly Asn Leu Thr Tyr Thr Trp Tyr Trp Gln Asp Glu Asn Val Tyr
            260                 265                 270

Phe Gln Asn Asp Leu Lys Leu Arg Val Arg Ile Leu Ile Asp Gly Thr
        275                 280                 285

Leu Ile Ile Phe Arg Val Lys Pro Glu Asp Ser Gly Lys Tyr Thr Cys
    290                 295                 300

Val Pro Ser Asn Ser Leu Gly Arg Ser Pro Ser Ala Ser Ala Tyr Leu
305                 310                 315                 320

Thr Val Gln Tyr Pro Ala Arg Val Leu Asn Met Pro Pro Val Ile Tyr
                325                 330                 335

Val Pro Val Gly Ile His Gly Tyr Ile Arg Cys Pro Val Asp Ala Arg
            340                 345                 350

Pro Pro Ala Thr Val Val Lys Trp Asn Lys Asp Gly Arg Pro Leu Gln
        355                 360                 365

Val Glu Lys Asn Arg Gly Trp Thr Leu Met Glu Asp Gly Ser Ile Arg
    370                 375                 380

Ile Glu Glu Ala Thr Glu Glu Ala Leu Gly Thr Tyr Thr Cys Val Pro
385                 390                 395                 400

Tyr Asn Thr Leu Gly Thr Met Gly Gln Ser Ala Pro Ala Arg Leu Val
                405                 410                 415

Leu Lys Asp Pro Pro Tyr Phe Thr Val Leu Pro Gly Trp Glu Tyr Arg
            420                 425                 430

Gln Glu Ala Gly Arg Glu Leu Leu Ile Pro Cys Ala Ala Ala Gly Asp
        435                 440                 445

Pro Phe Pro Val Ile Thr Trp Ser Lys Val Gly Lys Pro Ser Arg Ser
    450                 455                 460

Lys His Ser Ala Leu Pro Ser Gly Ser Leu Gln Phe Arg Ala Leu Ser
465                 470                 475                 480

Lys Glu Asp His Gly Glu Trp Glu Cys Val Ala Thr Asn Val Val Thr
                485                 490                 495

Ser Ile Thr Ala Ser Thr His Leu Thr Val Ile Gly Thr Ser Pro His
            500                 505                 510

Ala Pro Gly Ser Val Arg Val Gln Val Ser Met Thr Thr Ala Asn Val

        515                 520                 525

Ser Trp Glu Pro Gly Asp Gly Leu Arg Trp Gly Tyr Asp Gly Gly Tyr
    530                 535                 540

Glu Gln Thr Phe Ser Val Trp Met Lys Arg Ala Gln Phe Gly Pro His
545                 550                 555                 560

Asp Trp Leu Ser Leu Pro Val Pro Pro Gly Pro Ser Trp Leu Leu Val
                565                 570                 575

Asp Thr Leu Glu Pro Glu Thr Ala Tyr Gln Phe Ser Val Leu Ala Gln
            580                 585                 590

Asn Lys Leu Gly Thr Ser Ala Phe Ser Glu Val Val Thr Val Ile Thr
        595                 600                 605

Leu Ala Phe Pro Ile Thr Thr Pro Glu Pro Leu Val Leu Val Thr Pro
    610                 615                 620

Pro Arg Cys Leu Ile Ala Asn Arg Thr Gln Gln Gly Val Leu Leu Ser
625                 630                 635                 640

Trp Leu Pro Pro Ala Asn His Ser Phe Pro Ile Asp Arg Tyr Ile Met
                645                 650                 655

Glu Phe Arg Val Ala Glu Arg Trp Glu Leu Leu Asp Asp Gly Ile Pro
            660                 665                 670

Gly Thr Glu Gly Glu Phe Phe Ala Lys Asp Leu Ser Gln Asp Thr Trp
        675                 680                 685

Tyr Glu Phe Arg Val Leu Ala Val Met Gln Asp Leu Ile Gly Glu Pro
    690                 695                 700

Ser Asn Ile Ala Gly Val Ser Ser Thr Asp Ile Phe Pro Gln Pro Asp
705                 710                 715                 720

Leu Thr Glu Asp Gly Leu Ala Arg Pro Val Leu Ala Gly Ile Val Ala
                725                 730                 735

Thr Ile Cys Phe Leu Ala Ala Ala Ile Leu Phe Ser Thr Leu Ala Ala
            740                 745                 750

Cys Phe Val Asn Lys Gln Arg Lys Arg Lys Leu Lys Arg Lys Lys Asp
        755                 760                 765

Pro Pro Leu Ser Ile Thr His Cys Arg Lys Ser Leu Glu Ser Pro Leu
    770                 775                 780

Ser Ser Gly Lys Val Ser Pro Glu Ser Ile Arg Thr Leu Arg Ala Pro
785                 790                 795                 800

Ser Glu Ser Ser Asp Asp Gln Gly Gln Pro Ala Ala Lys Arg Met Leu
                805                 810                 815

Ser Pro Thr Arg Glu Lys Glu Leu Ser Leu Tyr Lys Lys Thr Lys Arg
            820                 825                 830

Ala Ile Ser Ser Lys Lys Tyr Ser Val Ala Lys Ala Glu Ala Glu Ala
        835                 840                 845

Glu Ala Thr Thr Pro Ile Glu Leu Ile Ser Arg Gly Pro Asp Gly Arg
    850                 855                 860

Phe Val Met Asp Pro Val Glu Met Glu Pro Ser Leu Lys Ser Arg Arg
865                 870                 875                 880

Ile Glu Gly Phe Pro Phe Ala Glu Glu Thr Asp Met Tyr Pro Glu Phe

                885                 890                 895
Arg Gln Ser Asp Glu Glu Asn Glu Asp Pro Leu Val Pro Thr Ser Val
            900                 905                 910
Ala Ala Leu Lys Ser Gln Leu Thr Pro Leu Ser Ser Ser Gln Glu Ser
        915                 920                 925
Tyr Leu Pro Pro Pro Ala Tyr Ser Pro Arg Phe Gln Pro Arg Gly Leu
    930                 935                 940
Glu Gly Pro Gly Gly Leu Glu Gly Arg Leu Gln Ala Thr Gly Gln Ala
945                 950                 955                 960
Arg Pro Pro Ala Pro Arg Pro Phe His His Gly Gln Tyr Tyr Gly Tyr
                965                 970                 975
Leu Ser Ser Ser Ser Pro Gly Glu Val Glu Pro Pro Pro Phe Tyr Val
            980                 985                 990
Pro Glu Val Gly Ser Pro Leu Ser Ser Val Met Ser Ser Pro Pro Leu
        995                 1000                1005
Pro Thr Glu Gly Pro Phe Gly His Pro Thr Ile Pro Glu Glu Asn Gly
    1010                1015                1020
Glu Asn Ala Ser Asn Ser Thr Leu Pro Leu Thr Gln Thr Pro Thr Gly
1025                1030                1035                1040
Gly Arg Ser Pro Glu Pro Trp Gly Arg Pro Glu Phe Pro Phe Gly Gly
                1045                1050                1055
Leu Glu Thr Pro Ala Met Met Phe Pro His Gln Leu Pro Pro Cys Asp
            1060                1065                1070
Val Pro Glu Ser Leu Gln Pro Lys Ala Gly Leu Pro Arg Gly Leu Pro
        1075                1080                1085
Pro Thr Ser Leu Gln Val Pro Ala Ala Tyr Pro Gly Ile Leu Ser Leu
    1090                1095                1100
Glu Ala Pro Lys Gly Trp Ala Gly Lys Ser Pro Gly Arg Gly Pro Val
1105                1110                1115                1120
Pro Ala Pro Pro Ala Ala Lys Trp Gln Asp Arg Pro Met Gln Pro Leu
                1125                1130                1135
Val Ser Gln Gly Gln Leu Arg His Thr Ser Gln Gly Met Gly Ile Pro
            1140                1145                1150
Val Leu Pro Tyr Pro Glu Pro Ala Glu Pro Gly Ala His Gly Gly Pro
        1155                1160                1165
Ser Thr Phe Gly Leu Asp Thr Arg Trp Tyr Glu Pro Gln Pro Arg Pro
    1170                1175                1180
Arg Pro Ser Pro Arg Gln Ala Arg Arg Ala Glu Pro Ser Leu His Gln
1185                1190                1195                1200
Val Val Leu Gln Pro Ser Arg Leu Ser Pro Leu Thr Gln Ser Pro Leu
                1205                1210                1215
Ser Ser Arg Thr Gly Ser Pro Glu Leu Ala Ala Arg Ala Arg Pro Arg
            1220                1225                1230
Pro Gly Leu Leu Gln Gln Ala Glu Met Ser Glu Ile Thr Leu Gln Pro
        1235                1240                1245
Pro Ala Ala Val Ser Phe Ser Arg Lys Ser Thr Pro Ser Thr Gly Ser

1250                1255                1260

Pro Ser Gln Ser Ser Arg Ser Gly Ser Pro Ser Tyr Arg Pro Ala Met
1265                1270                1275                1280

Gly Phe Thr Thr Leu Ala Thr Gly Tyr Pro Ser Pro Pro Pro Gly Pro
                1285                1290                1295

Ala Pro Ala Gly Pro Gly Asp Ser Leu Asp Val Phe Gly Gln Thr Pro
            1300                1305                1310

Ser Pro Arg Arg Thr Gly Glu Glu Leu Leu Arg Pro Glu Thr Pro Pro
        1315                1320                1325

Pro Thr Leu Pro Thr Ser Gly Lys Leu Gln Arg Asp Arg Pro Ala Pro
    1330                1335                1340

Ala Thr Ser Pro Pro Glu Arg Ala Leu Ser Lys Leu
1345                1350                1355


<210> 109
<211> 1247
<212> DNA
<213> Homo sapiens

<400> 109
atccacattg catttagttg tcaggtatgc tattaggact ctccaaagag acagaaccaa 60
taggggaaat gggtatatgc agagacagag agacaggagt taattctaaa cctaagagtc 120
atgtgactat taggcattcc attttagaaa aaattggaaa tagagttaca gccagagcag 180
cacaagtagc taagaaagct cagaacacac aaagtgccag ttcaacccag ggaaacaaca 240
aatgtcaaca aacaactgaa acctactgct tctgtgaaac agtacagatg gaaatgttgg 300
ctccaaaggg tccttctccc atacctgagg atgcctccgt gaaagaagag aacatctgcc 360
gagctttttc tgatgctttg ctctacaaaa ttgaggatat tgataacaaa gattggaata 420
accctcagct ctgcagtgac tatttaagga agggtatcta tcagtacctc aggcagctgg 480
agattttgca gttcataaac ccacatgtct taggtggagg agatgtaaat ggacataagc 540
ataccatcct ggtagactgg ttggtgcaaa tccactccaa gtttaggctt cttcaggaga 600
ctctgtatgt gtgtgttgcc attatggatg gatctttact ggttcagcca gtttcccaga 660
ggaagcttca actagtttgg attactgctc tgctcttggc ttccaagtat gaggagatgt 720
tttctccaaa tactgaaggc tttgtttaca tcacagacaa tgcttatact agtttccaaa 780
tccaagaaat ggaaactcta attttgaaag aactgaaatt tgaggtgggt ggacccttgc 840
cactacactt cttaaggcaa gcatcaaaag ccgggaaggc tgatgttgaa cagcacactt 900
tagccaaata tttgatggag ctgactctca ttgactacga tatgatgcat tatcatcctt 960
ctaaggcagc aacagctgct tcctgcttgt ctcagaaggt tctgggccaa ggaaaatgga 1020
acttaaagca gcagtgttat acaggataca cacagaatga agtattggaa gtcatgcagc 1080
acgtggccaa aaatgtgctg aaagtaaatg aaaacttaac taaattcatc gccatcaaga 1140
ataagtatgc aagcaacaaa ttcctgaaga tcagcatgat ccctcagctg aactcaaaag 1200
ccatcaaaga ccttgccttc cctctgatgg gagggtccta ggctgca                1247


<210> 110
<211> 404
<212> PRT
<213> Homo sapiens

<400> 110
Met Leu Leu Gly Leu Ser Lys Glu Thr Glu Pro Ile Gly Glu Met Gly
1               5                   10                  15

Ile Cys Arg Asp Arg Glu Thr Gly Val Asn Ser Lys Pro Lys Ser His
            20                  25                  30

Val Thr Ile Arg His Ser Ile Leu Glu Lys Ile Gly Asn Arg Val Thr
        35                  40                  45

Ala Arg Ala Ala Gln Val Ala Lys Lys Ala Gln Asn Thr Gln Ser Ala
    50                  55                  60

Ser Ser Thr Gln Gly Asn Asn Lys Cys Gln Gln Thr Thr Glu Thr Tyr

65                  70                  75                  80

Cys Phe Cys Glu Thr Val Gln Met Glu Met Leu Ala Pro Lys Gly Pro
                85                  90                  95

Ser Pro Ile Pro Glu Asp Ala Ser Val Lys Glu Glu Asn Ile Cys Arg
            100                 105                 110

Ala Phe Ser Asp Ala Leu Leu Tyr Lys Ile Glu Asp Ile Asp Asn Lys
        115                 120                 125

Asp Trp Asn Asn Pro Gln Leu Cys Ser Asp Tyr Leu Arg Lys Gly Ile
    130                 135                 140

Tyr Gln Tyr Leu Arg Gln Leu Glu Ile Leu Gln Phe Ile Asn Pro His
145                 150                 155                 160

Val Leu Gly Gly Gly Asp Val Asn Gly His Lys His Thr Ile Leu Val
                165                 170                 175

Asp Trp Leu Val Gln Ile His Ser Lys Phe Arg Leu Leu Gln Glu Thr
            180                 185                 190

Leu Tyr Val Cys Val Ala Ile Met Asp Gly Ser Leu Leu Val Gln Pro
        195                 200                 205

Val Ser Gln Arg Lys Leu Gln Leu Val Trp Ile Thr Ala Leu Leu Leu
    210                 215                 220

Ala Ser Lys Tyr Glu Glu Met Phe Ser Pro Asn Thr Glu Gly Phe Val
225                 230                 235                 240

Tyr Ile Thr Asp Asn Ala Tyr Thr Ser Phe Gln Ile Gln Glu Met Glu
                245                 250                 255

Thr Leu Ile Leu Lys Glu Leu Lys Phe Glu Val Gly Gly Pro Leu Pro
            260                 265                 270

Leu His Phe Leu Arg Gln Ala Ser Lys Ala Gly Lys Ala Asp Val Glu
        275                 280                 285

Gln His Thr Leu Ala Lys Tyr Leu Met Glu Leu Thr Leu Ile Asp Tyr
    290                 295                 300

Asp Met Met His Tyr His Pro Ser Lys Ala Ala Thr Ala Ala Ser Cys
305                 310                 315                 320

Leu Ser Gln Lys Val Leu Gly Gln Gly Lys Trp Asn Leu Lys Gln Gln
                325                 330                 335

Cys Tyr Thr Gly Tyr Thr Gln Asn Glu Val Leu Glu Val Met Gln His
            340                 345                 350

Val Ala Lys Asn Val Leu Lys Val Asn Glu Asn Leu Thr Lys Phe Ile
        355                 360                 365

Ala Ile Lys Asn Lys Tyr Ala Ser Asn Lys Phe Leu Lys Ile Ser Met
    370                 375                 380

Ile Pro Gln Leu Asn Ser Lys Ala Ile Lys Asp Leu Ala Phe Pro Leu
385                 390                 395                 400

Met Gly Gly Ser

<210> 111
<211> 15645
<212> DNA

<213> Homo sapiens

<400> 111
atgtcccctc cacctgaaga gtcacccatg tctccaccac cggaggcatc tcgtctgttc 60
ccaccatttg aagagtctcc tctgtcccct ccacctgagg agtctcccct ttccccacca 120
cctgaggcat cacgcctgtc cccaccacct gaggactcgc ctatgtcccc accacctgaa 180
gaatcaccta tgtcccccc acctgaggta tcgcgcctat cccccctgcc tgtggtgtca 240
cgcctgtctc caccgcctga ggaatctccc ttgtccccac cgcctgagga gtctcccacg 300
tcccctccac ctgaggcttc acgcctctcc ccaccacctg aggactcccc cacatcccca 360
ccacctgagg actcacctgc ttccccacca ccggaggact cgctcatgtc cctgccgctg 420
gaggagtcac ccctgttgcc actacctgag gagccgcaac tctgcccccg gtccgagggg 480
ccgcacctgt caccccggcc tgaggagccg cacctgtccc cccggcctga ggagccacac 540
ctatctccgc aggctgagga gccacacctg tccccccagc ctgaggagcc atgcctatgc 600
gctgtgcctg aggagccaca cttgtccccc caggctgagg gaccacatct gtcccctcag 660
cctgaggaat tgcacctgtc cccccagact gaggagccgc acctgtctcc tgtgcctgag 720
gagccatgct tgtcccccca acctgaggaa tcacacctgt ccccccagtc tgaggagcca 780
tgcctgtccc cccggcctga ggaatcgcat ctgtcccctg agcttgagaa gccacccctg 840
tcccctcggc ctgaaaagcc ccctgaggag ccaggccaat gccctgcacc tgaggagctg 900
cccttgttcc ctcccccctgg ggaaccatcc ttatctccct tgcttggaga gccagccctg 960
tctgagcctg gggaaccacc tctgtcccct ctgcccgagg agctgccgtt gtccccatct 1020
ggggagccat ccttgtcgcc tcagctgatg ccaccagatc cccttcctcc tccactctca 1080
cccattatca cagctgcggc cccaccggcc ctgtctcctt tgggggagtt agagtacccc 1140
tttggtgcca aaggggacag tgaccctgag tcaccgttgg ctgcccccat cctggagaca 1200
cccatcagcc ctccaccaga agctaactgc actgaccctg agcctgtccc ccctatgatc 1260
cttcccccat ctccaggctc cccagtgggg ccggcttctc ccatcctgat ggagcccctt 1320
cctcctcagt gttcgccact ccttcagcat tccctggttc cccaaaactc ccctccttcc 1380
cagtgctctc ctcctgccct accactgtcc gttccctccc cgttgagtcc catagggaag 1440
gtagtggggg tctcagatga ggctgagctg cacgagatgg agactgagaa agtttcagaa 1500
cctgaatgcc cagccttgga acccagtgcc accagtcctc tcccttcccc aatgggggac 1560
ctttcctgcc ccgcccccag ccctgcccca gccctggatg acttctctgg cctaggggaa 1620
gacacagccc ctctggatgg gattgatgct ccgggttcac agccagagcc tggacagacc 1680
cctggcagtt tggctagtga acttaaaggc tcccctgtgc tcctggaccc cgaggagctg 1740
gcccctgtga cccctatgga ggtctacccc gaatgcaagc agacagcagg gcggggctca 1800
ccatgtgaag aacaggaaga gccacgtgca ccggtggccc ccacaccacc cactctcatc 1860
aaatccgaca tcgttaacga gatctctaat ctgagccagg gtgatgccag tgccagtttt 1920
cctggctcag agcccctcct gggctctcca gacccggagg ggggtggctc cctgtccatg 1980
gagttggggg tctctacgga tgttagtcca gcccgagatg agggctccct acggctctgt 2040
actgactcac tgccagagac tgatgactca ctattgtgcg atgctgggac agctatcagc 2100
ggaggcaaag ctgaggggga gaaggggcgg cggcgcagct ccccagcccg ttcccgcatc 2160
aaacagggtc gcagcagcag tttcccagga agacgccggc ctcgtggagg agcccatgga 2220
gggcgtggta gaggacgggc ccggctaaag tcaactgctt cttccattga gactctggta 2280
gttgctgaca ttgatagctc tcccagtaag gaggaggagg aagaagatga tgacaccatg 2340
cagaataccg tggttctctt ctccaacaca gacaaatttg tcctaatgca ggacatgtgt 2400
gtggtatgtg gcagctttgg ccggggggca gagggccacc tccttgcctg ttcgcagtgc 2460
tctcagtgct atcaccctta ctgtgtcaac agcaagatca ccaaggtgat gctgctcaag 2520
ggctggcgtt gtgtggagtg tattgtgtgt gaggtgtgtg gccaggcctc cgacccctca 2580
cgcctgctgc tctgtgatga ctgtgatatt agctaccaca catactgcct ggacccccca 2640
ctgctcaccg tccccaaggg cggctggaag tgcaagtggt gtgtgtcctg tatgcagtgt 2700
ggggctgctt cccctggctt ccactgtgaa tggcagaata gttacacaca ctgtgggccc 2760
tgtgccagcc tggtgacctg ccctatctgt catgctcctt acgtagaaga ggacctacta 2820
atccagtgcc gccactgtga acggtggatg catgcaggct gtgagagcct cttcacagag 2880
gacgatgtgg accacgcacc cgatgaaggc tttgactgtg tctcctgcca gccctacgtg 2940
gtaaagcctg tggcgcctgt tgcacctcca gagctggtgc ccatgaaggt gaaagagcca 3000
gagccccagt actttcgctt cgaaggcgtg tggctgacag aaactggcat ggccttgctg 3060
cgtaacctga ccatgtcacc actgcacaag cggcgccaac ggcgaggacg gcttggcctc 3120
ccaggcgagg caggattgga gggttctgag ccctcagatg cccttggccc tgatgacaag 3180
aaggatgggg acctggacac cgatgagctg ctcaagggtg aaggtggtgt ggagcacatg 3240
gagtgcgaaa ttaaactgga gggccccgtc agccctgatg tggagcctgg caaagaggag 3300
accgaggaaa gcaaaaaacg caagcgtaaa ccatatcggc ctggcattgg tggtttcatg 3360
gtgcgacagc ggaaatccca cacacgcacg aaaaaggggc ctgctgcaca ggcggaggtg 3420
ttgagtgggg atgggcagcc cgacgaggtg atacctgctg acctgcctgc agagggcgcc 3480
gtggagcaga gcttagctga aggggatgag aagaagaagc aacagcggcg agggcgcaag 3540
aggagcaaac tggagggcat gttccctgct tacttgcagg aagccttctt tgggaaggag 3600
ctgctggacc tgagccgtaa ggcccttttt gcagttgggg tgggccggcc aagctttgga 3660
ctagggaccc caaaagccaa gggagatgga ggctcagaaa ggaaggaact ccccacatcg 3720
cagaaaggag atgatggtcc agatattgca gatgaagaat cccgtggcct cgagggcaaa 3780
gccgatacac caggacctga ggatgggggc gtgaaggcat ccccagtgcc cagtgaccct 3840
gagaagccag gcaccccagg tgaagggatg cttagctctg acttagacag gatttccaca 3900
gaagaactgc ccaagatgga atccaaggac ctgcagcagc tcttcaagga tgttctgggc 3960

tctgaacgag aacagcatct gggttgtgga acccctggcc tagaaggcag ccgtacgcca 4020
ctgcagaggc cctttcttca aggtggactc cctttgggca atctgccctc cagcagccca 4080
atggactcct acccaggcct ctgccagtcc ccgttcctgg attctaggga gcgcgggggc 4140
ttctttagcc cggaacccgg tgagcccgac agcccctgga cgggctcagg tggcaccacg 4200
ccctccaccc ccacaacccc caccacggag ggtgagggcg acggactctc ctataaccag 4260
cggagtcttc agcgctggga gaaggatgag gagttgggcc agctgtccac catctcgcct 4320
gtgctctatg ccaacattaa ttttcctaat ctcaagcaag actacccaga ctggtcaagc 4380
cgttgcaaac aaatcatgaa gctctggaga aaggttccag cagctgacaa agccccctac 4440
ctgcaaaagg ccaaagataa ccgggcagct caccgcatca acaaggtgca gaagcaggct 4500
gagagccaga tcaacaagca gaccaaggtg ggcgacatag cccgtaagac tgaccgaccg 4560
gccctacatc tccgcattcc cccgcagcca ggggcactgg gcagcccgcc ccccgctgct 4620
gcccccacca ttttcattgg cagccccact acccccgccg gcttgtctac ctctgcggac 4680
gggttcctga agccgccggc gggctcggtg cctggccctg actcgcctgg tgagctcttc 4740
ctcaagctcc caccccaggt gcccgcccaa gcgccttcgc aggacccctt tggactggcc 4800
cctgcctatc ccctggagcc ccgcttcccc acggcaccgc ccacctatcc cccctatcct 4860
agtcctacgg gggcccctgc gcagcccccg atgctgggcg cctcatctcg tcctggggct 4920
ggccagccag gggaattcca cactacccca cctggcaccc ccagacacca gccctccaca 4980
cctgacccgt tcctcaaacc ccgctgcccc tcgctggata acttggctgt gcctgagagc 5040
cctggggtag gggaggcaaa agcttccgag cccctgctct cgcccccacc ttttggggag 5100
tcccggaagg ccctagaggt gaagaaggaa gagcttgggg catcctctcc tagctatggg 5160
cccccaaacc tgggctttgt tgactcaccc tcctcaggca cccacctggg tggcctggag 5220
ttaaagacac ctgatgtctt caaagccccc ctgacccctc gggcatctca ggtagagccc 5280
cagagcccgg gcttgggcct aaggccccag gagccacccc ctgcccaggc tttggcacct 5340
tctcctccaa gtcacccaga catctttcgc cctggctcct acactgaccc atatgctcag 5400
cccccattga ctcctcggcc ccaacctccg ccccctgaga gctgctgtgc tctgccccct 5460
cgctcactgc cctccgaccc tttctcccga gtgcctgtca gtcctcagtc ccagtccagc 5520
tcccagtctc cactgacacc ccggcctctg tctgctgaag ctttttgccc atcacccgtt 5580
acccctcgct tccagtcccc tgacccttat tctcgcccac cctcacgccc tcagtcccgt 5640
gacccatttg ccccattgca taagccaccc cgaccccagc cccctgaagt tgcctttaag 5700
gctgggtctc tagcccacac ttcgctgggg gctgggggt tcccagcagc cctgcccgcg 5760
gggccagcag gtgagctcca tgccaaggtc ccaagtgggc agcccccccaa ttttgtccgg 5820
tcccctggga cgggtgcatt tgtgggcacc ccctctccca tgcgtttcac tttccctcag 5880
gcagtagggg agccttccct aaagccccct gtccctcagc ctggtctccc gccaccccat 5940
gggatcaaca gccattttgg gcccggcccc accttgggca agcctcaaag cacaaactac 6000
acagtagcca cagggaactt ccacccatcg ggcagccccc tgggggccag cagcgggtcc 6060
acaggggaga gctatgggct gtccccacta cgccctccgt cggttctgcc accacctgca 6120
cccgacggat ccctccccta cctgtcccat ggagcctcac agcgatcagg catcacctct 6180
cctgtcgaaa agcgagaaga cccagggact ggaatgggta gctctttggc gacagctgaa 6240
ctcccaggta cccaggaccc aggcatgtcc ggccttagcc aaacagagct ggagaagcaa 6300
cggcagcgcc agcggctacg agagctgctg attcggcagc agatccagcg caacaccctg 6360
cggcaggaga aggaaacagc tgcagcagct gcgggagcag tggggcctcc aggcagctgg 6420
ggtgctgagc ccagcagccc tgcctttgag cagctgagtc gaggccagac cccctttgct 6480
gggacacagg acaagagcag ccttgtgggg ttgcccccaa gcaagctgag tggccccatc 6540
ctggggccag ggtccttccc tagcgatgac cgactctccc ggccacctcc accagccacg 6600
ccttcctcta tggatgtgaa cagccggcaa ctggtaggag gctcccaagc tttctatcag 6660
cgagcaccct atcctgggtc cctgccctta cagcagcaac agcaacaact gtggcagcaa 6720
caacaggcaa cagcagcaac ctccatgcga tttgccatgt cagctcgctt tccatcaact 6780
cctggacctg aacttggccg ccaagcccta ggttccccgt tggcgggaat ttccacccgt 6840
ctgccaggcc ctggtgagcc agtgcctggt ccagctggtc ctgcccagtt cattgagctg 6900
cggcacaatg tacagaaagg actgggacct gggggcactc cgtttcctgg tcagggccca 6960
cctcagagac cccgttttta ccctgtaagt gaggaccccc accgactggc tcctgaaggg 7020
cttcggggcc tggcggtatc aggtcttccc ccacagaaac cctcagcccc accggcccct 7080
gaattgaaca acagtcttca tccaacaccc cacaccaagg gtcctaccct gccaactggt 7140
ttggagctgg tcaaccggcc cccgtcgagc actgagcttg gccgccccaa tcctctggcc 7200
ctggaagctg ggaagttgcc ctgtgaggat cccgagctgg atgacgattt tgatgcccac 7260
aaggccctag aggatgatga agagcttgct cacctgggtc tgggtgtgga tgtggccaag 7320
ggtgatgatg aacttggcac cttagaaaac ctggagacca atgaccccca cttggatgac 7380
ctgctcaatg gagacgagtt tgacctgctg gcatatactg atcctgagct ggacactggg 7440
gacaagaagg atatcttcaa tgagcacctg aggctggtag aatcggctaa tgaggaggct 7500
gaacgggagg ccctgctgcg gggggtggag ccaggaccct tgggccctga ggagcgccct 7560
cccccctgctg ctgatgcctc tgaaccccgc ctggcatctg tgctccctga ggtgaagccc 7620
aaggtggagg agggtggacg ccacccttct ccttgccaat tcaccattgc taccccccaag 7680
gtagagcccg cacctgctgc caattccctt ggcctggggc taaagccagg acagagcatg 7740
atgggcagcc gggatacccg gatgggcaca gggccatttt ctagcagtgg gcacacagct 7800
gagaaggcct cctttggggc cacgggaggg ccaccagctc acctgctgac ccccagccca 7860
ctgagtggcc caggaggatc ctccctgctg gaaaagtttg agctcgagag tggggctttg 7920
accttgcctg gtggacctgc agcatctggg gatgagctag acaagatgga gagctcactg 7980
gtagccagcg agttacccct gctcattgag gacctgttgg agcatgagaa gaaggagctg 8040
cagaagaagc agcagctttc agcacagttg cagcctgccc agcagcagca gcaacagcag 8100

cagcagcatt ccctactgcc tgcaccaggc cctgcccagg ccatgtcttt gccacatgag 8160
ggctcttctc ccagtttggc tgggtcccaa cagcagcttt ccctgggtct tgcagttgcc 8220
cgacagccag gtttgcccca gccactgatg cccacccagc caccagctca tgccctccag 8280
caacgcctgg ctccatccat ggctatggtg tccaatcaag ggcatatgct aagtgggcag 8340
catggagggc aggcaggctt ggtaccccag cagagctcac agccagtgct atcacagaag 8400
cccatgggca ccatgccacc ttccatgtgc atgaagccgc agcaattggc aatgcagcag 8460
cagctggcaa acagcttctt cccagataca gacctggaca aatttgctgc agaagatatc 8520
attggtccca ttgcaaaggc caagatggtg gctttgaaag gcatcaagaa agtgatggct 8580
cagggcagca ttggggtggc acctggtatg aacagacagc aagtgtctct gctagcccag 8640
aggctctcgg ggggacctag cagtgatctg cagaaccatg tggcagctgg gagtggccag 8700
gagcggagtg ctggtgatcc ctcccagcct cgtcccaacc cgcccacttt tgctcaggga 8760
gtgatcaatg aagctgacca gcggcagtat gaggagtggc tgttccatac ccagcagctc 8820
ctacagatgc agctgaaggt gctagaggag cagattggtg tacaccgcaa gtcccggaag 8880
gctctgtgtg ccaagcagcg cactgccaaa aaagctggcc gtgagttccc agaagctgat 8940
gctgagaagc tcaagctggt tacagagcag cagagcaaga tccagaaaca actggatcag 9000
gtccggaaac agcagaagga gcacactaat ctcatggcag aatatcggaa caagcagcag 9060
caacaacagc agcagcagca gcaacaacag caacagcact cagctgtgct ggctctcagc 9120
ccttcccaga gtccccggct gctcaccaag ctccctggtc agctgctccc tggccatggg 9180
ctgcagccac cacaggggcc tccgggtggg caagccggag gtcttcgcct gacccctggg 9240
ggtatggcac tacctggaca gcctggtggc cccttcctta atacagctct ggcccaacag 9300
cagcaacagc aacattctgg tggggctgga tccctggctg gcccttcagg gggcttcttc 9360
cctggcaacc ttgctcttcg aagcctcgga cctgattcaa ggcttttaca ggaaaggcag 9420
ctgcagctgc agcagcaacg tatgcagctg gcccagaaac tgcagcagca gcagcagcag 9480
caacagcagc agcagcacct tctaggacag gtggcaatcc agcagcaaca gcagcagggt 9540
cctggagtac agacaaacca agctctgggt cccaagcccc agggccttat gcctcccagc 9600
agccaccaag gcctcctggt ccagcagctg tcccctcaac caccccaggg gccccagggc 9660
atgctgggcc ctgcccaggt ggctgtgttg cagcagcagc accctggagc tttgggcccc 9720
cagggccctc acagacaggt gcttatgacc cagtcccggg tgctcagttc cccccagctg 9780
gcacagcagg gtcagggcct tatgggacac aggctggtca cagcccagca gcagcagcag 9840
caacaacagc accaacagca agggtccatg gcagggctgt cccatcttca gcaaagtctg 9900
atgtcacaca gtgggcagcc caaactgagc gctcagccca tgggctcttt acagcagctt 9960
cagcagcagc agcagctgca acagcaacag caacttcagc agcagcagca gcagcagcta 10020
caacagcaac agcaacttca gcagcaacag cttcaacagc agcaacagca gcagcagctt 10080
caacaacagc agcagcaaca gcttcaacag cagcaacagc agctacaaca gcaacagcaa 10140
caacaacagc agcagtttca acagcagcag caacagcagc agatgggcct tttaaaccag 10200
agtcgaactt tactgtcccc tcagcaacaa cagcagcagc aagtggcact tggccctggc 10260
atgccagcaa agcctcttca acacttttct agccctggag ccctgggtcc aaccctcctc 10320
ctgacgggca aggaacaaaa caccgtagac ccagccgttt cttcagaggc cactgagggg 10380
ccctctacac atcagggagg gccgttagca ataggaacta cccctgagtc aatggccact 10440
gaaccaggag aggtaaagcc ctcactctct ggggactcac aactcctgct tgtccaaccc 10500
cagccccagc ctcagcccag ctctctgcag ctgcagccac ctctgaggct tccaggacaa 10560
cagcagcagc aagttagcct gctccacaca gcaggtggag gaagccatgg gcagctaggc 10620
agtggatcat cttctgaggc ctcatctgtg ccccacctgc tggctcagcc ctctgtttcc 10680
ttaggggatc agcctgggtc catgacccag aaccttctgg gcccccaaca gcccatgcta 10740
gagcggccca tgcaaaataa tacaggccca caacctccca aaccaggacc tgtcctccag 10800
tctgggcagg gtctgcctgg ggttggaatc atgcctacgg tgggtcagct tcgagcacag 10860
ctccaaggag tcctggccaa aaacccacag ctgcggcact taagtcctca gcagcagcag 10920
cagctacagg cactcctcat gcagcggcag ctgcagcaga gtcaggcagt acgccagacc 10980
ccaccctacc aggagcctgg gacccagacc tctcccctcc agggcctcct gggctgccaa 11040
cctcaacttg gggggcttcc tggaccacag acaggccccc tccaggagct aggggcaggg 11100
cctcgacctc agggcccacc ccggctccct gccccaccag gagccttatc tacaggacca 11160
gtccttggcc ctgtccatcc cacacctcca ccatccagcc ctcaagagcc aaagagacct 11220
tcacaattac cttcccccag ctcccagctt cccactgagg cccagctccc tcccacccat 11280
ccagggaccc ccaaacctca ggggccaacc ttggagccgc ctcctgggag ggtctcacct 11340
gctgctgccc agcttgcaga taccttgttt agcaagggtc tgggaccttg ggatccccca 11400
gacaacctag cagaaaccca gaagccagag cagagcagcc tggtacctgg gcatctggac 11460
caggtgaatg gacaggtggt gcctgaggca tcccaactca gcatcaagca ggaacctcgg 11520
gaagagccat gtgccctggg agcccagtca gtgaagaggg aggccaatgg ggagccaata 11580
ggggcaccag gaaccagcaa ccacctcctg ctggcaggcc ctcgctcaga agctgggcat 11640
ctgctcttgc agaagctact ccgggcaaag aatgtgcaac tcagcactgg gcaggggtcc 11700
gaggggctgc gagctgagat caacgggcac attgacagca agctggctgg gctggagcag 11760
aaactacagg gtacccccag caacaaggag gatgcagcag caaggaagcc tttgacaccg 11820
aagcccaagc gggtacagaa ggcaagcgac aggttggtga gctcccgaaa gaagctgcgg 11880
aaggaggacg gcgtcagggc cagcgaggcc ttgctgaaac agctgaaaca ggagctgtcc 11940
ctgctgcccc taacggagcc tgctatcacc gccaatttta gcctctttgc cccctttggc 12000
agtggctgcc cagtcaatgg gcagagccag ctgagggggg cctttggaag tggggcgctg 12060
cccactggcc ctgactacta ttcccagctg cttaccaaga ataacctgag taacccgccg 12120
acaccaccct cgtcgctgcc ccccaccccc cccccatcgg tgcagcagaa gatggtgaat 12180
ggcgtcaccc catctgaaga gctgggggag caccccaagg atgctgcctc tgcccgggat 12240

agtgaaaggg cactgaggga tacttcagag gtgaagagtc tagacctgct ggctgccttg 12300
cctacacccc ctcacaatca gactgaggat gtcaggatgg agagtgatga ggatagcgat 12360
tctcctgaca gcattgtgcc agcttcatcc cctgagagca tcttggggga ggaggcccct 12420
cgtttccctc atctgggctc aggccggtgg gagcaagagg accgggccct ctcccctgtc 12480
atccccctca ttcctcggga cagcatccca gtcttcccag ataccaaacc ttatggggcc 12540
cttggcctgg aggtccctgg aaagctgcct gtcacaactt gggaaaaggg caaaggaagt 12600
gaggtgtcag tcatgctcac agtctctgct gctgcagaca agaacctgaa tggcgtgatg 12660
gtggcagtgg cggagctgct gagcatgaag atccccaact cctatgaggt gctgttccca 12720
gagagccccg cccggggagg cactgagcca aagaaggggg aagctgaggg tcctggtggg 12780
aaggaaaagg gtctggaagg caagagccca gacactggcc ctgattggct gaagcagttt 12840
gatgcagtgt tggctggcta taccctgaag aggcaactag acatcttgag cctcctgaaa 12900
caggagagcc ccgccccaga gccacccact cagcacaggt atacctacaa tgtctccaat 12960
ctggatgtgc gacagctctc ggccccacct cctgaagaac cctccccgcc cccttccccc 13020
ttggcacctt ctcctgccag tcccccctact gagcccttgg ttgaacttcc caccgaaccc 13080
ttggctgagc cacccgtccc ctcacctctg ccactggcct catcccctga atcagcccga 13140
cccaagcccc gtgcccggcc ccctgaagaa ggtgaagata cccgtcctcc tcgcctcaag 13200
aaatggaaag gagtgcgctg gaagcggcgc ttacgaggtg ccatgttgga gctttttggt 13260
gtgaacagtc tggaagtaaa atttaggacc agaagcgaga atggcgtttt aatccatatc 13320
caagaaagca gcaattacac tactgtgaag attaagaatg gcaaagtata ttttacatcc 13380
gatgcaggaa ttgctgggaa agtggagaga aatattcctg aagtatatgt tgcagacggc 13440
cactggcaca cttttctaat tgggaaaaat ggaacagcaa cagtattgtc tgttgacaga 13500
atatataaca gagatattat ccaccctact caggacttcg gtggccttga tgtgcttact 13560
atatcacttg gaggaattcc acccaatcaa gcacatcgag atgcccaaac agcaggtttt 13620
gatggctgca ttgcttctat gtggtatggt ggagaaagtc ttcctttcag cgggaagcat 13680
agcttggcct ccatctcaaa aacagatccc tcagtgaaga ttggctgccg tggcccgaac 13740
atttgtgcca gcaacccctg ctggggtgat ttgctgtgca ttaatcagtg gtatgcctac 13800
aggtgtgtcc ctcctgggga ctgtgcctcc cacccgtgcc agaatggtgg cagctgtgag 13860
ccaggcctgc actccggctt cacctgtagc tgcccagact cgcacacggg aaggacctgt 13920
gagatggtgg tggcctgtct tggcgtcctc tgtcctcagg ggaaggtgtg caaagctgga 13980
agtcctgcgg ggcatgtctg tgttctgagt cagggccctg aagagatctc tctgcctttg 14040
tgggctgtgc ctgccatcgt gggcagctgc gcaaccgtct tggccctcct ggtccttagc 14100
ctgatcctgt gtaaccagtg caggggggaag aaggccaaaa atcccaaaga ggagaagaaa 14160
ccgaaggaga agaagaaaaa gggaagtgag aacgttgctt ttgatgaccc tgacaatatc 14220
cctccctatg gggatgacat gactgtgagg aagcagcctg aagggaaccc aaaaccagat 14280
atcattgaaa gggaaaaccc ctaccttatc tatgatgaaa ctgatattcc tcacaactca 14340
gaaaccatcc ccagcgcccc tttggcatct ccagagcagg agatagagca ctatgacatt 14400
gacaacgcca gcagcatcgc cccttcggat gcagacatca ttcaacacta caagcagttc 14460
cgcagccaca caccaaaatt ttcaatccag aggcacagtc ccctaggctt tgcaaggcaa 14520
tcccccatgc ccttaggagc aagcagtttg acttaccagc cttcatatgg tcaaggtttg 14580
agaaccagct ccctaagcca ctcagcatgc ccaactccca accctctgtc tcgacacagt 14640
ccagcccctt tctccaaatc ttctacgttc tatagaaaca gcccagcaag ggaattgcat 14700
cttcctataa gggatggtaa tactttggaa atgcatggtg acacctgcca acctggcatt 14760
ttcaactatg ccacaaggct gggaaggaga agcaagagtc ctcaggccat ggcatcacat 14820
ggttctagac cagggagtcg cctaaagcag ccgattgggc agattccact ggaatcttct 14880
cctccagtcg gactttctat tgaagaagtg gagaggctca acacacctcg ccctagaaac 14940
ccaagtatct gcagtgcaga ccatgggagg tcttcttcag aggaggactg cagaaggcca 15000
ctgtctagaa caaggaatcc agcggatggc attccagctc cagaatcctc ttctgatagt 15060
gactcccatg aatctttcac ttgctcagaa atggaatatg acagggagaa gccaatggta 15120
tatacttcca gaatgcccaa attatctcaa gtcaatgaat ctgatgcaga tgatgaagat 15180
aattatggag ccagactgaa gcctcgaagg taccacggtc gcagggccga gggaggacct 15240
gtgggcaccc aggcagcagc accaggcact gctgacaaca cactgcccat gaagctaggg 15300
cagcaagcag ggactttcaa ctgggacaac cttttgaact ggggccctgg ctttggccat 15360
tatgtagatg tttttaaaga tttggcatct cttccagaaa aagcagcagc aaatgaagaa 15420
ggcaaagctg ggacaactaa accagtcccc aaagatgggg aagcagaaca gtatgtgtga 15480
agtttatgta ctggcactat aaaatataaa aacaagaaat aatacttcaa accattgtaa 15540
agttgctgac taggttgggg tcacatttga aaaacagggc cagtatggga ctagtgggtg 15600
ggaggggaaa acttttaaaa ttattaacca cattgctggc tgaaa 15645

<210> 112
<211> 5159
<212> PRT
<213> Homo sapiens

<400> 112
   Met Ser Pro Pro Pro Glu Glu Ser Pro Met Ser Pro Pro Pro Glu Ala
   1               5                   10                  15
   Ser Arg Leu Phe Pro Pro Phe Glu Glu Ser Pro Leu Ser Pro Pro Pro
   20                  25                  30
   Glu Glu Ser Pro Leu Ser Pro Pro Pro Glu Ala Ser Arg Leu Ser Pro
           35                  40                  45
   Pro Pro Glu Asp Ser Pro Met Ser Pro Pro Pro Glu Glu Ser Pro Met
       50                  55                  60
   Ser Pro Pro Pro Glu Val Ser Arg Leu Ser Pro Leu Pro Val Val Ser
   65                  70                  75                  80
   Arg Leu Ser Pro Pro Pro Glu Glu Ser Pro Leu Ser Pro Pro Pro Glu
                   85                  90                  95
   Glu Ser Pro Thr Ser Pro Pro Pro Glu Ala Ser Arg Leu Ser Pro Pro
               100                 105                 110
   Pro Glu Asp Ser Pro Thr Ser Pro Pro Pro Glu Asp Ser Pro Ala Ser
           115                 120                 125
   Pro Pro Pro Glu Asp Ser Leu Met Ser Leu Pro Leu Glu Glu Ser Pro
       130                 135                 140
   Leu Leu Pro Leu Pro Glu Glu Pro Gln Leu Cys Pro Arg Ser Glu Gly
   145                 150                 155                 160
   Pro His Leu Ser Pro Arg Pro Glu Glu Pro His Leu Ser Pro Arg Pro
                   165                 170                 175
   Glu Glu Pro His Leu Ser Pro Gln Ala Glu Glu Pro His Leu Ser Pro
               180                 185                 190
   Gln Pro Glu Glu Pro Cys Leu Cys Ala Val Pro Glu Glu Pro His Leu
           195                 200                 205
   Ser Pro Gln Ala Glu Gly Pro His Leu Ser Pro Gln Pro Glu Glu Leu
       210                 215                 220
   His Leu Ser Pro Gln Thr Glu Glu Pro His Leu Ser Pro Val Pro Glu
   225                 230                 235                 240
   Glu Pro Cys Leu Ser Pro Gln Pro Glu Glu Ser His Leu Ser Pro Gln
                   245                 250                 255
   Ser Glu Glu Pro Cys Leu Ser Pro Arg Pro Glu Glu Ser His Leu Ser
               260                 265                 270
   Pro Glu Leu Glu Lys Pro Pro Leu Ser Pro Arg Pro Glu Lys Pro Pro
           275                 280                 285
   Glu Glu Pro Gly Gln Cys Pro Ala Pro Glu Glu Leu Pro Leu Phe Pro
       290                 295                 300
   Pro Pro Gly Glu Pro Ser Leu Ser Pro Leu Leu Gly Glu Pro Ala Leu
   305                 310                 315                 320
   Ser Glu Pro Gly Glu Pro Pro Leu Ser Pro Leu Pro Glu Glu Leu Pro
                   325                 330                 335
   Leu Ser Pro Ser Gly Glu Pro Ser Leu Ser Pro Gln Leu Met Pro Pro
               340                 345                 350
   Asp Pro Leu Pro Pro Pro Leu Ser Pro Ile Ile Thr Ala Ala Ala Pro
           355                 360                 365
   Pro Ala Leu Ser Pro Leu Gly Glu Leu Glu Tyr Pro Phe Gly Ala Lys
       370                 375                 380
   Gly Asp Ser Asp Pro Glu Ser Pro Leu Ala Ala Pro Ile Leu Glu Thr
   385                 390                 395                 400
   Pro Ile Ser Pro Pro Pro Glu Ala Asn Cys Thr Asp Pro Glu Pro Val
                   405                 410                 415
   Pro Pro Met Ile Leu Pro Pro Ser Pro Gly Ser Pro Val Gly Pro Ala
               420                 425                 430
   Ser Pro Ile Leu Met Glu Pro Leu Pro Pro Gln Cys Ser Pro Leu Leu
           435                 440                 445
   Gln His Ser Leu Val Pro Gln Asn Ser Pro Pro Ser Gln Cys Ser Pro
       450                 455                 460
   Pro Ala Leu Pro Leu Ser Val Pro Ser Pro Leu Ser Pro Ile Gly Lys
   465                 470                 475                 480
   Val Val Gly Val Ser Asp Glu Ala Glu Leu His Glu Met Glu Thr Glu
                   485                 490                 495
   Lys Val Ser Glu Pro Glu Cys Pro Ala Leu Glu Pro Ser Ala Thr Ser
               500                 505                 510
   Pro Leu Pro Ser Pro Met Gly Asp Leu Ser Cys Pro Ala Pro Ser Pro
           515                 520                 525
   Ala Pro Ala Leu Asp Asp Phe Ser Gly Leu Gly Glu Asp Thr Ala Pro
       530                 535                 540
   Leu Asp Gly Ile Asp Ala Pro Gly Ser Gln Pro Glu Pro Gly Gln Thr
   545                 550                 555                 560
   Pro Gly Ser Leu Ala Ser Glu Leu Lys Gly Ser Pro Val Leu Leu Asp
                   565                 570                 575
   Pro Glu Glu Leu Ala Pro Val Thr Pro Met Glu Val Tyr Pro Glu Cys
               580                 585                 590
   Lys Gln Thr Ala Gly Arg Gly Ser Pro Cys Glu Glu Gln Glu Glu Pro
           595                 600                 605
   Arg Ala Pro Val Ala Pro Thr Pro Pro Thr Leu Ile Lys Ser Asp Ile
       610                 615                 620
   Val Asn Glu Ile Ser Asn Leu Ser Gln Gly Asp Ala Ser Ala Ser Phe
   625                 630                 635                 640
   Pro Gly Ser Glu Pro Leu Leu Gly Ser Pro Asp Pro Glu Gly Gly Gly
                   645                 650                 655
   Ser Leu Ser Met Glu Leu Gly Val Ser Thr Asp Val Ser Pro Ala Arg
               660                 665                 670
   Asp Glu Gly Ser Leu Arg Leu Cys Thr Asp Ser Leu Pro Glu Thr Asp
           675                 680                 685
   Asp Ser Leu Leu Cys Asp Ala Gly Thr Ala Ile Ser Gly Gly Lys Ala
       690                 695                 700
   Glu Gly Glu Lys Gly Arg Arg Arg Ser Ser Pro Ala Arg Ser Arg Ile
   705                 710                 715                 720
   Lys Gln Gly Arg Ser Ser Ser Phe Pro Gly Arg Arg Arg Pro Arg Gly
                   725                 730                 735
   Gly Ala His Gly Gly Arg Gly Arg Gly Arg Ala Leu Arg Lys Ser Thr
               740                 745                 750
   Ala Ser Ser Ile Glu Thr Leu Val Val Ala Asp Ile Asp Ser Ser Pro
   755 760 765
   Ser Lys Glu Glu Glu Glu Glu Asp Asp Asp Thr Met Gln Asn Thr Val
   770 775 780
   Val Leu Phe Ser Asn Thr Asp Lys Phe Val Leu Met Gln Asp Met Cys
   785 790 795 800
   Val Val Cys Gly Ser Phe Gly Arg Gly Ala Glu Gly His Leu Leu Ala
   805 810 815
   Cys Ser Gln Cys Ser Gln Cys Tyr His Pro Tyr Cys Val Asn Ser Lys
   820 825 830
   Ile Thr Lys Val Met Leu Leu Lys Gly Trp Arg Cys Val Glu Cys Ile
   835 840 845
   Val Cys Glu Val Cys Gly Gln Ala Ser Asp Pro Ser Arg Leu Leu Leu
   850 855 860
   Cys Asp Asp Cys Asp Ile Ser Tyr His Thr Tyr Cys Leu Asp Pro Pro
   865 870 875 880
   Leu Leu Thr Val Pro Lys Gly Gly Trp Lys Cys Lys Trp Cys Val Ser
   885 890 895
   Cys Met Gln Cys Gly Ala Ala Ser Pro Gly Phe His Cys Glu Trp Gln
   900 905 910
   Asn Ser Tyr Thr His Cys Gly Pro Cys Ala Ser Leu Val Thr Cys Pro
   915 920 925
   Ile Cys His Ala Pro Tyr Val Glu Glu Asp Leu Leu Ile Gln Cys Arg
   930 935 940
   His Cys Glu Arg Trp Met His Ala Gly Cys Glu Ser Leu Phe Thr Glu
   945 950 955 960
   Asp Asp Val Asp His Ala Pro Asp Glu Gly Phe Asp Cys Val Ser Cys
   965 970 975
   Gln Pro Tyr Val Val Lys Pro Val Ala Pro Val Ala Pro Pro Glu Leu
   980 985 990
   Val Pro Met Lys Val Lys Glu Pro Glu Pro Gln Tyr Phe Arg Phe Glu
   995 1000 1005
   Gly Val Trp Leu Thr Glu Thr Gly Met Ala Leu Leu Arg Asn Leu Thr
   1010 1015 1020
   Met Ser Pro Leu His Lys Arg Arg Gln Arg Arg Gly Arg Leu Gly Leu
   1025 1030 1035 1040
   Pro Gly Glu Ala Gly Leu Glu Gly Ser Glu Pro Ser Asp Ala Leu Gly
   1045 1050 1055
   Pro Asp Asp Lys Lys Asp Gly Asp Leu Asp Thr Asp Glu Leu Leu Lys
   1060 1065 1070
   Gly Glu Gly Gly Val Glu His Met Glu Cys Glu Ile Lys Leu Glu Gly
   1075 1080 1085
   Pro Val Ser Pro Asp Val Glu Pro Gly Lys Glu Glu Thr Glu Glu Ser
   1090 1095 1100
   Lys Lys Arg Lys Arg Lys Pro Tyr Arg Pro Gly Ile Gly Gly Phe Met
   1105 1110 1115 1120
   Val Arg Gln Arg Lys Ser His Thr Arg Thr Lys Lys Gly Pro Ala Ala
   1125                    1130                    1135
   Gln Ala Glu Val Leu Ser Gly Asp Gly Gln Pro Asp Glu Val Ile Pro
               1140                    1145                    1150
   Ala Asp Leu Pro Ala Glu Gly Ala Val Glu Gln Ser Leu Ala Glu Gly
           1155                    1160                    1165
   Asp Glu Lys Lys Lys Gln Gln Arg Arg Gly Arg Lys Arg Ser Lys Leu
       1170                    1175                    1180
   Glu Gly Met Phe Pro Ala Tyr Leu Gln Glu Ala Phe Phe Gly Lys Glu
   1185                    1190                    1195                    1200
   Leu Leu Asp Leu Ser Arg Lys Ala Leu Phe Ala Val Gly Val Gly Arg
                   1205                    1210                    1215
   Pro Ser Phe Gly Leu Gly Thr Pro Lys Ala Lys Gly Asp Gly Gly Ser
               1220                    1225                    1230
   Glu Arg Lys Glu Leu Pro Thr Ser Gln Lys Gly Asp Asp Gly Pro Asp
           1235                    1240                    1245
   Ile Ala Asp Glu Glu Ser Arg Gly Leu Glu Gly Lys Ala Asp Thr Pro
       1250                    1255                    1260
   Gly Pro Glu Asp Gly Gly Val Lys Ala Ser Pro Val Pro Ser Asp Pro
   1265                    1270                    1275                    1280
   Glu Lys Pro Gly Thr Pro Gly Glu Gly Met Leu Ser Ser Asp Leu Asp
                   1285                    1290                    1295
   Arg Ile Ser Thr Glu Glu Leu Pro Lys Met Glu Ser Lys Asp Leu Gln
               1300                    1305                    1310
   Gln Leu Phe Lys Asp Val Leu Gly Ser Glu Arg Glu Gln His Leu Gly
           1315                    1320                    1325
   Cys Gly Thr Pro Gly Leu Glu Gly Ser Arg Thr Pro Leu Gln Arg Pro
       1330                    1335                    1340
   Phe Leu Gln Gly Gly Leu Pro Leu Gly Asn Leu Pro Ser Ser Ser Pro
   1345                    1350                    1355                    1360
   Met Asp Ser Tyr Pro Gly Leu Cys Gln Ser Pro Phe Leu Asp Ser Arg
                   1365                    1370                    1375
   Glu Arg Gly Gly Phe Phe Ser Pro Glu Pro Gly Glu Pro Asp Ser Pro
               1380                    1385                    1390
   Trp Thr Gly Ser Gly Gly Thr Thr Pro Ser Thr Pro Thr Thr Pro Thr
           1395                    1400                    1405
   Thr Glu Gly Glu Gly Asp Gly Leu Ser Tyr Asn Gln Arg Ser Leu Gln
       1410                    1415                    1420
   Arg Trp Glu Lys Asp Glu Glu Leu Gly Gln Leu Ser Thr Ile Ser Pro
   1425                    1430                    1435                    1440
   Val Leu Tyr Ala Asn Ile Asn Phe Pro Asn Leu Lys Gln Asp Tyr Pro
                   1445                    1450                    1455
   Asp Trp Ser Ser Arg Cys Lys Gln Ile Met Lys Leu Trp Arg Lys Val
               1460                    1465                    1470
   Pro Ala Ala Asp Lys Ala Pro Tyr Leu Gln Lys Ala Lys Asp Asn Arg
           1475                    1480                    1485
   Ala Ala His Arg Ile Asn Lys Val Gln Lys Gln Ala Glu Ser Gln Ile
   1490                    1495                    1500
   Asn Lys Gln Thr Lys Val Gly Asp Ile Ala Arg Lys Thr Asp Arg Pro
   1505                    1510                    1515                    1520
   Ala Leu His Leu Arg Ile Pro Pro Gln Pro Gly Ala Leu Gly Ser Pro
                   1525                    1530                    1535
   Pro Pro Ala Ala Ala Pro Thr Ile Phe Ile Gly Ser Pro Thr Thr Pro
               1540                    1545                    1550
   Ala Gly Leu Ser Thr Ser Ala Asp Gly Phe Leu Lys Pro Pro Ala Gly
           1555                    1560                    1565
   Ser Val Pro Gly Pro Asp Ser Pro Gly Glu Leu Phe Leu Lys Leu Pro
       1570                    1575                    1580
   Pro Gln Val Pro Ala Gln Ala Pro Ser Gln Asp Pro Phe Gly Leu Ala
   1585                    1590                    1595                    1600
   Pro Ala Tyr Pro Leu Glu Pro Arg Phe Pro Thr Ala Pro Pro Thr Tyr
                   1605                    1610                    1615
   Pro Pro Tyr Pro Ser Pro Thr Gly Ala Pro Ala Gln Pro Pro Met Leu
               1620                    1625                    1630
   Gly Ala Ser Ser Arg Pro Gly Ala Gly Gln Pro Gly Glu Phe His Thr
           1635                    1640                    1645
   Thr Pro Pro Gly Thr Pro Arg His Gln Pro Ser Thr Pro Asp Pro Phe
       1650                    1655                    1660
   Leu Lys Pro Arg Cys Pro Ser Leu Asp Asn Leu Ala Val Pro Glu Ser
   1665                    1670                    1675                    1680
   Pro Gly Val Gly Gly Gly Lys Ala Ser Glu Pro Leu Leu Ser Pro Pro
                   1685                    1690                    1695
   Pro Phe Gly Glu Ser Arg Lys Ala Leu Glu Val Lys Lys Glu Glu Leu
               1700                    1705                    1710
   Gly Ala Ser Ser Pro Ser Tyr Gly Pro Pro Asn Leu Gly Phe Val Asp
           1715                    1720                    1725
   Ser Pro Ser Ser Gly Thr His Leu Gly Gly Leu Glu Leu Lys Thr Pro
       1730                    1735                    1740
   Asp Val Phe Lys Ala Pro Leu Thr Pro Arg Ala Ser Gln Val Glu Pro
   1745                    1750                    1755                    1760
   Gln Ser Pro Gly Leu Gly Leu Arg Pro Gln Glu Pro Pro Pro Ala Gln
                   1765                    1770                    1775
   Ala Leu Ala Pro Ser Pro Pro Ser His Pro Asp Ile Phe Arg Pro Gly
               1780                    1785                    1790
   Ser Tyr Thr Asp Pro Tyr Ala Gln Pro Pro Leu Thr Pro Arg Pro Gln
           1795                    1800                    1805
   Pro Pro Pro Pro Glu Ser Cys Cys Ala Leu Pro Pro Arg Ser Leu Pro
       1810                    1815                    1820
   Ser Asp Pro Phe Ser Arg Val Pro Val Ser Pro Gln Ser Gln Ser Ser
   1825                    1830                    1835                    1840
   Ser Gln Ser Pro Leu Thr Pro Arg Pro Leu Ser Ala Glu Ala Phe Cys
                   1845                    1850                    1855
   Pro Ser Pro Val Thr Pro Arg Phe Gln Ser Pro Asp Pro Tyr Ser Arg
   1860                1865                1870
   Pro Pro Ser Arg Pro Gln Ser Arg Asp Pro Phe Ala Pro Leu His Lys
           1875                1880                1885
   Pro Pro Arg Pro Gln Pro Pro Glu Val Ala Phe Lys Ala Gly Ser Leu
       1890                1895                1900
   Ala His Thr Ser Leu Gly Ala Gly Gly Phe Pro Ala Ala Leu Pro Ala
   1905                1910                1915                1920
   Gly Pro Ala Gly Glu Leu His Ala Lys Val Pro Ser Gly Gln Pro Pro
                   1925                1930                1935
   Asn Phe Val Arg Ser Pro Gly Thr Gly Ala Phe Val Gly Thr Pro Ser
               1940                1945                1950
   Pro Met Arg Phe Thr Phe Pro Gln Ala Val Gly Glu Pro Ser Leu Lys
           1955                1960                1965
   Pro Pro Val Pro Gln Pro Gly Leu Pro Pro Pro His Gly Ile Asn Ser
       1970                1975                1980
   His Phe Gly Pro Gly Pro Thr Leu Gly Lys Pro Gln Ser Thr Asn Tyr
   1985                1990                1995                2000
   Thr Val Ala Thr Gly Asn Phe His Pro Ser Gly Ser Pro Leu Gly Pro
                   2005                2010                2015
   Ser Ser Gly Ser Thr Gly Glu Ser Tyr Gly Leu Ser Pro Leu Arg Pro
               2020                2025                2030
   Pro Ser Val Leu Pro Pro Pro Ala Pro Asp Gly Ser Leu Pro Tyr Leu
           2035                2040                2045
   Ser His Gly Ala Ser Gln Arg Ser Gly Ile Thr Ser Pro Val Glu Lys
       2050                2055                2060
   Arg Glu Asp Pro Gly Thr Gly Met Gly Ser Ser Leu Ala Thr Ala Glu
   2065                2070                2075                2080
   Leu Pro Gly Thr Gln Asp Pro Gly Met Ser Gly Leu Ser Gln Thr Glu
                   2085                2090                2095
   Leu Glu Lys Gln Arg Gln Arg Gln Arg Leu Arg Glu Leu Leu Ile Arg
               2100                2105                2110
   Gln Gln Ile Gln Arg Asn Thr Leu Arg Gln Glu Lys Glu Thr Ala Ala
           2115                2120                2125
   Ala Ala Ala Gly Ala Val Gly Pro Pro Gly Ser Trp Gly Ala Glu Pro
       2130                2135                2140
   Ser Ser Pro Ala Phe Glu Gln Leu Ser Arg Gly Gln Thr Pro Phe Ala
   2145                2150                2155                2160
   Gly Thr Gln Asp Lys Ser Ser Leu Val Gly Leu Pro Pro Ser Lys Leu
                   2165                2170                2175
   Ser Gly Pro Ile Leu Gly Pro Gly Ser Phe Pro Ser Asp Asp Arg Leu
               2180                2185                2190
   Ser Arg Pro Pro Pro Pro Ala Thr Pro Ser Ser Met Asp Val Asn Ser
           2195                2200                2205
   Arg Gln Leu Val Gly Gly Ser Gln Ala Phe Tyr Gln Arg Ala Pro Tyr
       2210                2215                2220
   Pro Gly Ser Leu Pro Leu Gln Gln Gln Gln Gln Gln Leu Trp Gln Gln
   2225                2230                2235                2240
   Gln Gln Ala Thr Ala Ala Thr Ser Met Arg Phe Ala Met Ser Ala Arg
                   2245                2250                2255
   Phe Pro Ser Thr Pro Gly Pro Glu Leu Gly Arg Gln Ala Leu Gly Ser
               2260                2265                2270
   Pro Leu Ala Gly Ile Ser Thr Arg Leu Pro Gly Pro Gly Glu Pro Val
           2275                2280                2285
   Pro Gly Pro Ala Gly Pro Ala Gln Phe Ile Glu Leu Arg His Asn Val
       2290                2295                2300
   Gln Lys Gly Leu Gly Pro Gly Gly Thr Pro Phe Pro Gly Gln Gly Pro
   2305                2310                2315                2320
   Pro Gln Arg Pro Arg Phe Tyr Pro Val Ser Glu Asp Pro His Arg Leu
                   2325                2330                2335
   Ala Pro Glu Gly Leu Arg Gly Leu Ala Val Ser Gly Leu Pro Pro Gln
               2340                2345                2350
   Lys Pro Ser Ala Pro Pro Ala Pro Glu Leu Asn Asn Ser Leu His Pro
           2355                2360                2365
   Thr Pro His Thr Lys Gly Pro Thr Leu Pro Thr Gly Leu Glu Leu Val
       2370                2375                2380
   Asn Arg Pro Pro Ser Ser Thr Glu Leu Gly Arg Pro Asn Pro Leu Ala
   2385                2390                2395                2400
   Leu Glu Ala Gly Lys Leu Pro Cys Glu Asp Pro Glu Leu Asp Asp Asp
                   2405                2410                2415
   Phe Asp Ala His Lys Ala Leu Glu Asp Asp Glu Glu Leu Ala His Leu
               2420                2425                2430
   Gly Leu Gly Val Asp Val Ala Lys Gly Asp Asp Glu Leu Gly Thr Leu
           2435                2440                2445
   Glu Asn Leu Glu Thr Asn Asp Pro His Leu Asp Asp Leu Leu Asn Gly
       2450                2455                2460
   Asp Glu Phe Asp Leu Leu Ala Tyr Thr Asp Pro Glu Leu Asp Thr Gly
   2465                2470                2475                2480
   Asp Lys Lys Asp Ile Phe Asn Glu His Leu Arg Leu Val Glu Ser Ala
                   2485                2490                2495
   Asn Glu Glu Ala Glu Arg Glu Ala Leu Leu Arg Gly Val Glu Pro Gly
               2500                2505                2510
   Pro Leu Gly Pro Glu Glu Arg Pro Pro Pro Ala Ala Asp Ala Ser Glu
           2515                2520                2525
   Pro Arg Leu Ala Ser Val Leu Pro Glu Val Lys Pro Lys Val Glu Glu
       2530                2535                2540
   Gly Gly Arg His Pro Ser Pro Cys Gln Phe Thr Ile Ala Thr Pro Lys
   2545                2550                2555                2560
   Val Glu Pro Ala Pro Ala Ala Asn Ser Leu Gly Leu Gly Leu Lys Pro
                   2565                2570                2575
   Gly Gln Ser Met Met Gly Ser Arg Asp Thr Arg Met Gly Thr Gly Pro
               2580                2585                2590
   Phe Ser Ser Ser Gly His Thr Ala Glu Lys Ala Ser Phe Gly Ala Thr
   2595                2600                2605
   Gly Gly Pro Pro Ala His Leu Leu Thr Pro Ser Pro Leu Ser Gly Pro
       2610                2615                2620
   Gly Gly Ser Ser Leu Leu Glu Lys Phe Glu Leu Glu Ser Gly Ala Leu
   2625                2630                2635                2640
   Thr Leu Pro Gly Gly Pro Ala Ala Ser Gly Asp Glu Leu Asp Lys Met
                   2645                2650                2655
   Glu Ser Ser Leu Val Ala Ser Glu Leu Pro Leu Leu Ile Glu Asp Leu
               2660                2665                2670
   Leu Glu His Glu Lys Lys Glu Leu Gln Lys Lys Gln Gln Leu Ser Ala
           2675                2680                2685
   Gln Leu Gln Pro Ala Gln Gln Gln Gln Gln Gln Gln Gln Gln His Ser
       2690                2695                2700
   Leu Leu Pro Ala Pro Gly Pro Ala Gln Ala Met Ser Leu Pro His Glu
   2705                2710                2715                2720
   Gly Ser Ser Pro Ser Leu Ala Gly Ser Gln Gln Gln Leu Ser Leu Gly
                   2725                2730                2735
   Leu Ala Val Ala Arg Gln Pro Gly Leu Pro Gln Pro Leu Met Pro Thr
               2740                2745                2750
   Gln Pro Pro Ala His Ala Leu Gln Gln Arg Leu Ala Pro Ser Met Ala
           2755                2760                2765
   Met Val Ser Asn Gln Gly His Met Leu Ser Gly Gln His Gly Gly Gln
       2770                2775                2780
   Ala Gly Leu Val Pro Gln Gln Ser Ser Gln Pro Val Leu Ser Gln Lys
   2785                2790                2795                2800
   Pro Met Gly Thr Met Pro Pro Ser Met Cys Met Lys Pro Gln Gln Leu
                   2805                2810                2815
   Ala Met Gln Gln Gln Leu Ala Asn Ser Phe Phe Pro Asp Thr Asp Leu
               2820                2825                2830
   Asp Lys Phe Ala Ala Glu Asp Ile Ile Gly Pro Ile Ala Lys Ala Lys
           2835                2840                2845
   Met Val Ala Leu Lys Gly Ile Lys Lys Val Met Ala Gln Gly Ser Ile
       2850                2855                2860
   Gly Val Ala Pro Gly Met Asn Arg Gln Gln Val Ser Leu Leu Ala Gln
   2865                2870                2875                2880
   Arg Leu Ser Gly Gly Pro Ser Ser Asp Leu Gln Asn His Val Ala Ala
                   2885                2890                2895
   Gly Ser Gly Gln Glu Arg Ser Ala Gly Asp Pro Ser Gln Pro Arg Pro
               2900                2905                2910
   Asn Pro Pro Thr Phe Ala Gln Gly Val Ile Asn Glu Ala Asp Gln Arg
           2915                2920                2925
   Gln Tyr Glu Glu Trp Leu Phe His Thr Gln Gln Leu Leu Gln Met Gln
       2930                2935                2940
   Leu Lys Val Leu Glu Glu Gln Ile Gly Val His Arg Lys Ser Arg Lys
   2945                2950                2955                2960
   Ala Leu Cys Ala Lys Gln Arg Thr Ala Lys Lys Ala Gly Arg Glu Phe
   2965 2970 2975
   Pro Glu Ala Asp Ala Glu Lys Leu Lys Leu Val Thr Glu Gln Gln Ser
   2980 2985 2990
   Lys Ile Gln Lys Gln Leu Asp Gln Val Arg Lys Gln Gln Lys Glu His
   2995 3000 3005
   Thr Asn Leu Met Ala Glu Tyr Arg Asn Lys Gln Gln Gln Gln Gln Gln
   3010 3015 3020
   Gln Gln Gln Gln Gln Gln Gln Gln His Ser Ala Val Leu Ala Leu Ser
   3025 3030 3035 3040
   Pro Ser Gln Ser Pro Arg Leu Leu Thr Lys Leu Pro Gly Gln Leu Leu
   3045 3050 3055
   Pro Gly His Gly Leu Gln Pro Pro Gln Gly Pro Pro Gly Gly Gln Ala
   3060 3065 3070
   Gly Gly Leu Arg Leu Thr Pro Gly Gly Met Ala Leu Pro Gly Gln Pro
   3075 3080 3085
   Gly Gly Pro Phe Leu Asn Thr Ala Leu Ala Gln Gln Gln Gln Gln Gln
   3090 3095 3100
   His Ser Gly Gly Ala Gly Ser Leu Ala Gly Pro Ser Gly Gly Phe Phe
   3105 3110 3115 3120
   Pro Gly Asn Leu Ala Leu Arg Ser Leu Gly Pro Asp Ser Arg Leu Leu
   3125 3130 3135
   Gln Glu Arg Gln Leu Gln Leu Gln Gln Gln Arg Met Gln Leu Ala Gln
   3140 3145 3150
   Lys Leu Gln Gln Gln Gln Gln Gln Gln Gln Gln Gln Gln His Leu Leu
   3155 3160 3165
   Gly Gln Val Ala Ile Gln Gln Gln Gln Gln Gln Gly Pro Gly Val Gln
   3170 3175 3180
   Thr Asn Gln Ala Leu Gly Pro Lys Pro Gln Gly Leu Met Pro Pro Ser
   3185 3190 3195 3200
   Ser His Gln Gly Leu Leu Val Gln Gln Leu Ser Pro Gln Pro Pro Gln
   3205 3210 3215
   Gly Pro Gln Gly Met Leu Gly Pro Ala Gln Val Ala Val Leu Gln Gln
   3220 3225 3230
   Gln His Pro Gly Ala Leu Gly Pro Gln Gly Pro His Arg Gln Val Leu
   3235 3240 3245
   Met Thr Gln Ser Arg Val Leu Ser Ser Pro Gln Leu Ala Gln Gln Gly
   3250 3255 3260
   Gln Gly Leu Met Gly His Arg Leu Val Thr Ala Gln Gln Gln Gln Gln
   3265 3270 3275 3280
   Gln Gln Gln His Gln Gln Gln Gly Ser Met Ala Gly Leu Ser His Leu
   3285 3290 3295
   Gln Gln Ser Leu Met Ser His Ser Gly Gln Pro Lys Leu Ser Ala Gln
   3300 3305 3310
   Pro Met Gly Ser Leu Gln Gln Leu Gln Gln Gln Gln Gln Leu Gln Gln
   3315 3320 3325
   Gln Gln Gln Leu Gln Gln Gln Gln Gln Gln Gln Leu Gln Gln Gln Gln
   3330                 3335                 3340
   Gln Leu Gln Gln Gln Gln Leu Gln Gln Gln Gln Gln Gln Gln Gln Leu
   3345                3350                3355                3360
   Gln Gln Gln Gln Gln Gln Gln Leu Gln Gln Gln Gln Gln Gln Leu Gln
                   3365                3370                3375
   Gln Gln Gln Gln Gln Gln Gln Gln Gln Phe Gln Gln Gln Gln Gln Gln
               3380                3385                3390
   Gln Gln Met Gly Leu Leu Asn Gln Ser Arg Thr Leu Leu Ser Pro Gln
           3395                3400                3405
   Gln Gln Gln Gln Gln Gln Val Ala Leu Gly Pro Gly Met Pro Ala Lys
       3410                3415                3420
   Pro Leu Gln His Phe Ser Ser Pro Gly Ala Leu Gly Pro Thr Leu Leu
   3425                3430                3435                3440
   Leu Thr Gly Lys Glu Gln Asn Thr Val Asp Pro Ala Val Ser Ser Glu
                   3445                3450                3455
   Ala Thr Glu Gly Pro Ser Thr His Gln Gly Gly Pro Leu Ala Ile Gly
               3460                3465                3470
   Thr Thr Pro Glu Ser Met Ala Thr Glu Pro Gly Glu Val Lys Pro Ser
           3475                3480                3485
   Leu Ser Gly Asp Ser Gln Leu Leu Leu Val Gln Pro Gln Pro Gln Pro
       3490                3495                3500
   Gln Pro Ser Ser Leu Gln Leu Gln Pro Pro Leu Arg Leu Pro Gly Gln
   3505                3510                3515                3520
   Gln Gln Gln Gln Val Ser Leu Leu His Thr Ala Gly Gly Gly Ser His
                   3525                3530                3535
   Gly Gln Leu Gly Ser Gly Ser Ser Ser Glu Ala Ser Ser Val Pro His
               3540                3545                3550
   Leu Leu Ala Gln Pro Ser Val Ser Leu Gly Asp Gln Pro Gly Ser Met
           3555                3560                3565
   Thr Gln Asn Leu Leu Gly Pro Gln Gln Pro Met Leu Glu Arg Pro Met
       3570                3575                3580
   Gln Asn Asn Thr Gly Pro Gln Pro Pro Lys Pro Gly Pro Val Leu Gln
   3585                3590                3595                3600
   Ser Gly Gln Gly Leu Pro Gly Val Gly Ile Met Pro Thr Val Gly Gln
                   3605                3610                3615
   Leu Arg Ala Gln Leu Gln Gly Val Leu Ala Lys Asn Pro Gln Leu Arg
               3620                3625                3630
   His Leu Ser Pro Gln Gln Gln Gln Gln Leu Gln Ala Leu Leu Met Gln
           3635                3640                3645
   Arg Gln Leu Gln Gln Ser Gln Ala Val Arg Gln Thr Pro Pro Tyr Gln
       3650                3655                3660
   Glu Pro Gly Thr Gln Thr Ser Pro Leu Gln Gly Leu Leu Gly Cys Gln
   3665                3670                3675                3680
   Pro Gln Leu Gly Gly Phe Pro Gly Pro Gln Thr Gly Pro Leu Gln Glu
                   3685                3690                3695
   Leu Gly Ala Gly Pro Arg Pro Gln Gly Pro Pro Arg Leu Pro Ala Pro
   3700                3705                3710
   Pro Gly Ala Leu Ser Thr Gly Pro Val Leu Gly Pro Val His Pro Thr
       3715                3720                3725
   Pro Pro Pro Ser Ser Pro Gln Glu Pro Lys Arg Pro Ser Gln Leu Pro
       3730                3735                3740
   Ser Pro Ser Ser Gln Leu Pro Thr Glu Ala Gln Leu Pro Pro Thr His
   3745                3750                3755                3760
   Pro Gly Thr Pro Lys Pro Gln Gly Pro Thr Leu Glu Pro Pro Pro Gly
                   3765                3770                3775
   Arg Val Ser Pro Ala Ala Ala Gln Leu Ala Asp Thr Leu Phe Ser Lys
               3780                3785                3790
   Gly Leu Gly Pro Trp Asp Pro Pro Asp Asn Leu Ala Glu Thr Gln Lys
           3795                3800                3805
   Pro Glu Gln Ser Ser Leu Val Pro Gly His Leu Asp Gln Val Asn Gly
       3810                3815                3820
   Gln Val Val Pro Glu Ala Ser Gln Leu Ser Ile Lys Gln Glu Pro Arg
   3825                3830                3835                3840
   Glu Glu Pro Cys Ala Leu Gly Ala Gln Ser Val Lys Arg Glu Ala Asn
                   3845                3850                3855
   Gly Glu Pro Ile Gly Ala Pro Gly Thr Ser Asn His Leu Leu Leu Ala
               3860                3865                3870
   Gly Pro Arg Ser Glu Ala Gly His Leu Leu Leu Gln Lys Leu Leu Arg
           3875                3880                3885
   Ala Lys Asn Val Gln Leu Ser Thr Gly Gln Gly Ser Glu Gly Leu Arg
       3890                3895                3900
   Ala Glu Ile Asn Gly His Ile Asp Ser Lys Leu Ala Gly Leu Glu Gln
   3905                3910                3915                3920
   Lys Leu Gln Gly Thr Pro Ser Asn Lys Glu Asp Ala Ala Ala Arg Lys
                   3925                3930                3935
   Pro Leu Thr Pro Lys Pro Lys Arg Val Gln Lys Ala Ser Asp Arg Leu
               3940                3945                3950
   Val Ser Ser Arg Lys Lys Leu Arg Lys Glu Asp Gly Val Arg Ala Ser
           3955                3960                3965
   Glu Ala Leu Leu Lys Gln Leu Lys Gln Glu Leu Ser Leu Leu Pro Leu
       3970                3975                3980
   Thr Glu Pro Ala Ile Thr Ala Asn Phe Ser Leu Phe Ala Pro Phe Gly
   3985                3990                3995                4000
   Ser Gly Cys Pro Val Asn Gly Gln Ser Gln Leu Arg Gly Ala Phe Gly
                   4005                4010                4015
   Ser Gly Ala Leu Pro Thr Gly Pro Asp Tyr Tyr Ser Gln Leu Leu Thr
               4020                4025                4030
   Lys Asn Asn Leu Ser Asn Pro Pro Thr Pro Pro Ser Ser Leu Pro Pro
           4035                4040                4045
   Thr Pro Pro Pro Ser Val Gln Gln Lys Met Val Asn Gly Val Thr Pro
       4050                4055                4060
   Ser Glu Glu Leu Gly Glu His Pro Lys Asp Ala Ala Ser Ala Arg Asp
   4065                4070                4075                4080
   Ser Glu Arg Ala Leu Arg Asp Thr Ser Glu Val Lys Ser Leu Asp Leu
                   4085                4090                4095
   Leu Ala Ala Leu Pro Thr Pro Pro His Asn Gln Thr Glu Asp Val Arg
               4100                4105                4110
   Met Glu Ser Asp Glu Asp Ser Asp Ser Pro Asp Ser Ile Val Pro Ala
           4115                4120                4125
   Ser Ser Pro Glu Ser Ile Leu Gly Glu Glu Ala Pro Arg Phe Pro His
       4130                4135                4140
   Leu Gly Ser Gly Arg Trp Glu Gln Glu Asp Arg Ala Leu Ser Pro Val
   4145                4150                4155                4160
   Ile Pro Leu Ile Pro Arg Asp Ser Ile Pro Val Phe Pro Asp Thr Lys
                   4165                4170                4175
   Pro Tyr Gly Ala Leu Gly Leu Glu Val Pro Gly Lys Leu Pro Val Thr
               4180                4185                4190
   Thr Trp Glu Lys Gly Lys Gly Ser Glu Val Ser Val Met Leu Thr Val
           4195                4200                4205
   Ser Ala Ala Ala Asp Lys Asn Leu Asn Gly Val Met Val Ala Val Ala
       4210                4215                4220
   Glu Leu Leu Ser Met Lys Ile Pro Asn Ser Tyr Glu Val Leu Phe Pro
   4225                4230                4235                4240
   Glu Ser Pro Ala Arg Gly Gly Thr Glu Pro Lys Lys Gly Glu Ala Glu
                   4245                4250                4255
   Gly Pro Gly Gly Lys Glu Lys Gly Leu Glu Gly Lys Ser Pro Asp Thr
               4260                4265                4270
   Gly Pro Asp Trp Leu Lys Gln Phe Asp Ala Val Leu Ala Gly Tyr Thr
           4275                4280                4285
   Leu Lys Arg Gln Leu Asp Ile Leu Ser Leu Leu Lys Gln Glu Ser Pro
       4290                4295                4300
   Ala Pro Glu Pro Pro Thr Gln His Arg Tyr Thr Tyr Asn Val Ser Asn
   4305                4310                4315                4320
   Leu Asp Val Arg Gln Leu Ser Ala Pro Pro Pro Glu Glu Pro Ser Pro
                   4325                4330                4335
   Pro Pro Ser Pro Leu Ala Pro Ser Pro Ala Ser Pro Pro Thr Glu Pro
               4340                4345                4350
   Leu Val Glu Leu Pro Thr Glu Pro Leu Ala Glu Pro Pro Val Pro Ser
           4355                4360                4365
   Pro Leu Pro Leu Ala Ser Ser Pro Glu Ser Ala Arg Pro Lys Pro Arg
       4370                4375                4380
   Ala Arg Pro Pro Glu Glu Gly Glu Asp Thr Arg Pro Pro Arg Leu Lys
   4385                4390                4395                4400
   Lys Trp Lys Gly Val Arg Trp Lys Arg Arg Leu Arg Gly Ala Met Leu
                   4405                4410                4415
   Glu Leu Phe Gly Val Asn Ser Leu Glu Val Lys Phe Arg Thr Arg Ser
               4420                4425                4430
   Glu Asn Gly Val Leu Ile His Ile Gln Glu Ser Ser Asn Tyr Thr Thr
   4435                4440                4445
   Val Lys Ile Lys Asn Gly Lys Val Tyr Phe Thr Ser Asp Ala Gly Ile
       4450                4455                4460
   Ala Gly Lys Val Glu Arg Asn Ile Pro Glu Val Tyr Val Ala Asp Gly
   4465                4470                4475                4480
   His Trp His Thr Phe Leu Ile Gly Lys Asn Gly Thr Ala Thr Val Leu
                   4485                4490                4495
   Ser Val Asp Arg Ile Tyr Asn Arg Asp Ile Ile His Pro Thr Gln Asp
               4500                4505                4510
   Phe Gly Gly Leu Asp Val Leu Thr Ile Ser Leu Gly Gly Ile Pro Pro
           4515                4520                4525
   Asn Gln Ala His Arg Asp Ala Gln Thr Ala Gly Phe Asp Gly Cys Ile
       4530                4535                4540
   Ala Ser Met Trp Tyr Gly Gly Glu Ser Leu Pro Phe Ser Gly Lys His
   4545                4550                4555                4560
   Ser Leu Ala Ser Ile Ser Lys Thr Asp Pro Ser Val Lys Ile Gly Cys
                   4565                4570                4575
   Arg Gly Pro Asn Ile Cys Ala Ser Asn Pro Cys Trp Gly Asp Leu Leu
               4580                4585                4590
   Cys Ile Asn Gln Trp Tyr Ala Tyr Arg Cys Val Pro Pro Gly Asp Cys
           4595                4600                4605
   Ala Ser His Pro Cys Gln Asn Gly Gly Ser Cys Glu Pro Gly Leu His
       4610                4615                4620
   Ser Gly Phe Thr Cys Ser Cys Pro Asp Ser His Thr Gly Arg Thr Cys
   4625                4630                4635                4640
   Glu Met Val Val Ala Cys Leu Gly Val Leu Cys Pro Gln Gly Lys Val
                   4645                4650                4655
   Cys Lys Ala Gly Ser Pro Ala Gly His Val Cys Val Leu Ser Gln Gly
               4660                4665                4670
   Pro Glu Glu Ile Ser Leu Pro Leu Trp Ala Val Pro Ala Ile Val Gly
           4675                4680                4685
   Ser Cys Ala Thr Val Leu Ala Leu Leu Val Leu Ser Leu Ile Leu Cys
       4690                4695                4700
   Asn Gln Cys Arg Gly Lys Lys Ala Lys Asn Pro Lys Glu Glu Lys Lys
   4705                4710                4715                4720
   Pro Lys Glu Lys Lys Lys Lys Gly Ser Glu Asn Val Ala Phe Asp Asp
                   4725                4730                4735
   Pro Asp Asn Ile Pro Pro Tyr Gly Asp Asp Met Thr Val Arg Lys Gln
               4740                4745                4750
   Pro Glu Gly Asn Pro Lys Pro Asp Ile Ile Glu Arg Glu Asn Pro Tyr
           4755                4760                4765
   Leu Ile Tyr Asp Glu Thr Asp Ile Pro His Asn Ser Glu Thr Ile Pro
       4770                4775                4780
   Ser Ala Pro Leu Ala Ser Pro Glu Gln Glu Ile Glu His Tyr Asp Ile
   4785                4790                4795                4800
   Asp Asn Ala Ser Ser Ile Ala Pro Ser Asp Ala Asp Ile Ile Gln His
   4805                    4810                    4815
   Tyr Lys Gln Phe Arg Ser His Thr Pro Lys Phe Ser Ile Gln Arg His
               4820                    4825                    4830
   Ser Pro Leu Gly Phe Ala Arg Gln Ser Pro Met Pro Leu Gly Ala Ser
           4835                    4840                    4845
   Ser Leu Thr Tyr Gln Pro Ser Tyr Gly Gln Gly Leu Arg Thr Ser Ser
       4850                    4855                    4860
   Leu Ser His Ser Ala Cys Pro Thr Pro Asn Pro Leu Ser Arg His Ser
   4865                    4870                    4875                    4880
   Pro Ala Pro Phe Ser Lys Ser Ser Thr Phe Tyr Arg Asn Ser Pro Ala
                   4885                    4890                    4895
   Arg Glu Leu His Leu Pro Ile Arg Asp Gly Asn Thr Leu Glu Met His
               4900                    4905                    4910
   Gly Asp Thr Cys Gln Pro Gly Ile Phe Asn Tyr Ala Thr Arg Leu Gly
           4915                    4920                    4925
   Arg Arg Ser Lys Ser Pro Gln Ala Met Ala Ser His Gly Ser Arg Pro
       4930                    4935                    4940
   Gly Ser Arg Leu Lys Gln Pro Ile Gly Gln Ile Pro Leu Glu Ser Ser
   4945                    4950                    4955                    4960
   Pro Pro Val Gly Leu Ser Ile Glu Glu Val Glu Arg Leu Asn Thr Pro
                   4965                    4970                    4975
   Arg Pro Arg Asn Pro Ser Ile Cys Ser Ala Asp His Gly Arg Ser Ser
               4980                    4985                    4990
   Ser Glu Glu Asp Cys Arg Arg Pro Leu Ser Arg Thr Arg Asn Pro Ala
           4995                    5000                    5005
   Asp Gly Ile Pro Ala Pro Glu Ser Ser Ser Asp Ser Asp Ser His Glu
       5010                    5015                    5020
   Ser Phe Thr Cys Ser Glu Met Glu Tyr Asp Arg Glu Lys Pro Met Val
   5025                    5030                    5035                    5040
   Tyr Thr Ser Arg Met Pro Lys Leu Ser Gln Val Asn Glu Ser Asp Ala
                   5045                    5050                    5055
   Asp Asp Glu Asp Asn Tyr Gly Ala Leu Arg Lys Pro Arg Arg Tyr His
               5060                    5065                    5070
   Gly Arg Arg Ala Glu Gly Gly Pro Val Gly Thr Gln Ala Ala Ala Pro
           5075                    5080                    5085
   Gly Thr Ala Asp Asn Thr Leu Pro Met Lys Leu Gly Gln Gln Ala Gly
       5090                    5095                    5100
   Thr Phe Asn Trp Asp Asn Leu Leu Asn Trp Gly Pro Gly Phe Gly His
   5105                    5110                    5115                    5120
   Tyr Val Asp Val Phe Lys Asp Leu Ala Ser Leu Pro Glu Lys Ala Ala
                   5125                    5130                    5135
   Ala Asn Glu Glu Gly Lys Ala Gly Thr Thr Lys Pro Val Pro Lys Asp
               5140                    5145                    5150
   Gly Glu Ala Glu Gln Tyr Val
           5155

<210> 113
<211> 1988
<212> DNA
<213> Homo sapiens

<400> 113
ggcggcgact gcggcgaccg cgggacggcg agaggcacgc ggcgggaggg gaccggaatc 60
cgcagctccg gccgcgccat ggacggcaac gacaacgtga ccctgctctt cgcccctctg 120
ctgcgggaca actacaccct ggcgcccaat gccagcagcc tgggccccgg cacggacctc 180
gccctcgccc ctgcctccag cgccggcccc ggccctgggc tcagcctcgg gccgggtccg 240
agcttcggct tcagccccgg ccccactccg accccggagc ccacgaccag cggcctcgcg 300
ggcggcgcgg cgagccacgg cccttccccg ttccctcggc cctgggcgcc ccacgcgctc 360
ccgttctggg acacgccgct gaaccacggg ctgaacgtgt tcgtgggcgc cgccctgtgc 420
atcaccatgc tgggcctggg ctgcacggtg gacgtgaacc acttcggggc gcacgtccgt 480
cggcccgtgg gcgcgctgct ggcagcgctc tgccagttcg gcctcctgcc gctgctggcc 540
ttcctgctgg ccctcgcctt caagctggac gaggtggccg ccgtggcggt gctcctgtgt 600
ggctgctgtc ccggcggcaa tctctccaat cttatgtccc tgctggttga cggcgacatg 660
aacctcagca tcatcatgac catctcctcc acgcttctgg ccctcgtctt gatgcccctg 720
tgcctgtgga tctacagctg ggcttggatc aacaccccta tcgtgcagtt actaccccta 780
gggaccgtga ccctgactct ctgcagcact ctcataccta tcgggttggg cgtcttcatt 840
cgctacaaat acagccgggt ggctgactac attgtgaagg taaggcccgt ttccctgtgg 900
tctctgctag tgactctggt ggtccttttc ataatgaccg gcactatgtt aggacctgaa 960
ctgctggcaa gtatccctgc agctgtttat gtgatagcaa tttttatgcc tttggcaggc 1020
tacgcttcag gttatggttt agctactctc ttccatcttc cacccaactg caagaggact 1080
gtatgtctgg aaacaggtag tcagaatgtg cagctctgta cagccattct aaaactggcc 1140
tttccaccgc aattcatagg aagcatgtac atgtttcctt tgctgtatgc acttttccag 1200
tctgcagaag cggggatttt tgttttaatc tataaaatgt atggaagtga aatgttgcac 1260
aagcgagatc ctctagatga agatgaagat acagatattt cttataaaaa actaaaagaa 1320
gaggaaatgg cagacacttc ctatggcaca gtgaaagcag aaaatataat aatgatggaa 1380
accgctcaga cttctctcta aatgtggaga tacacaggag cttctatctt gctgaaatat 1440
tgcttcatat ttatagcctg tggtagtgca catggttaac ataaaagata acactggttc 1500
acatcataca tgtaacaatt ctgatctttt taaggttcac tggtgtatta accaaacgtt 1560
gtcacaaatt acaaatcaat gctgtaatat aatttgcacc tggaatggct aacgtgaagc 1620
ctgaattaaa tgtggttttt agtttttacc atcaccaatt tctatgactg ttgcaaatac 1680
agaatctatt agaaaacagg gtcttggaaa tgtagaattt tggcgcacta tgaggaaaaa 1740
caagctatct ttgtaaagca taattgagtt taatgtaatt gttgtaaaaa aaaaagtgtg 1800
cttgctctac ttaaaattcc tcacaatgtt gaattttgac ctgtattcag aagaattcca 1860
aaacaggtca gttaaataag gaaatatagt atttgtcaaa ccagtatcag agaaaagtta 1920
cattaatgta tttgattact tgatctggta tctacttatt aatgaataat caacattttt 1980
ctagtgaa 1988

<210> 114
<211> 440
<212> PRT
<213> Homo sapiens

<400> 114
Met Asp Gly Asn Asp Asn Val Thr Leu Leu Phe Ala Pro Leu Leu Arg
1               5                   10                  15

Asp Asn Tyr Thr Leu Ala Pro Asn Ala Ser Ser Leu Gly Pro Gly Thr
            20                  25                  30

Asp Leu Ala Leu Ala Pro Ala Ser Ser Ala Gly Pro Gly Pro Gly Leu
        35                  40                  45

Ser Leu Gly Pro Gly Pro Ser Phe Gly Phe Ser Pro Gly Pro Thr Pro
    50                  55                  60

Thr Pro Glu Pro Thr Thr Ser Gly Leu Ala Gly Gly Ala Ala Ser His
65                  70                  75                  80

Gly Pro Ser Pro Phe Pro Arg Pro Trp Ala Pro His Ala Leu Pro Phe
                85                  90                  95

Trp Asp Thr Pro Leu Asn His Gly Leu Asn Val Phe Val Gly Ala Ala
            100                 105                 110

Leu Cys Ile Thr Met Leu Gly Leu Gly Cys Thr Val Asp Val Asn His
        115                 120                 125

Phe Gly Ala His Val Arg Arg Pro Val Gly Ala Leu Leu Ala Ala Leu
    130                 135                 140

Cys Gln Phe Gly Leu Leu Pro Leu Leu Ala Phe Leu Leu Ala Leu Ala
145                 150                 155                 160

Phe Lys Leu Asp Glu Val Ala Ala Val Ala Val Leu Leu Cys Gly Cys
                165                 170                 175

Cys Pro Gly Gly Asn Leu Ser Asn Leu Met Ser Leu Leu Val Asp Gly
            180                 185                 190

Asp Met Asn Leu Ser Ile Ile Met Thr Ile Ser Ser Thr Leu Leu Ala
        195                 200                 205

Leu Val Leu Met Pro Leu Cys Leu Trp Ile Tyr Ser Trp Ala Trp Ile
    210                 215                 220

Asn Thr Pro Ile Val Gln Leu Leu Pro Leu Gly Thr Val Thr Leu Thr
225                 230                 235                 240

Leu Cys Ser Thr Leu Ile Pro Ile Gly Leu Gly Val Phe Ile Arg Tyr
                245                 250                 255

Lys Tyr Ser Arg Val Ala Asp Tyr Ile Val Lys Val Arg Pro Val Ser
            260                 265                 270

Leu Trp Ser Leu Leu Val Thr Leu Val Val Leu Phe Ile Met Thr Gly
        275                 280                 285

Thr Met Leu Gly Pro Glu Leu Leu Ala Ser Ile Pro Ala Ala Val Tyr
    290                 295                 300

Val Ile Ala Ile Phe Met Pro Leu Ala Gly Tyr Ala Ser Gly Tyr Gly
305                 310                 315                 320

Leu Ala Thr Leu Phe His Leu Pro Pro Asn Cys Lys Arg Thr Val Cys
                325                 330                 335

Leu Glu Thr Gly Ser Gln Asn Val Gln Leu Cys Thr Ala Ile Leu Lys
            340                 345                 350

Leu Ala Phe Pro Pro Gln Phe Ile Gly Ser Met Tyr Met Phe Pro Leu
        355                 360                 365

Leu Tyr Ala Leu Phe Gln Ser Ala Glu Ala Gly Ile Phe Val Leu Ile
    370                 375                 380

Tyr Lys Met Tyr Gly Ser Glu Met Leu His Lys Arg Asp Pro Leu Asp
385                 390                 395                 400

Glu Asp Glu Asp Thr Asp Ile Ser Tyr Lys Lys Leu Lys Glu Glu Glu
                405                 410                 415

Met Ala Asp Thr Ser Tyr Gly Thr Val Lys Ala Glu Asn Ile Ile Met
            420                 425                 430

Met Glu Thr Ala Gln Thr Ser Leu
        435                 440

<210> 115
<211> 2313
<212> DNA
<213> Homo sapiens

<400> 115
ccattgactg caggaaggtt ggccagcaga gcaaatgcca tgcctgcaga ggacaacgag 60
actgaagctc agcagcatga ccgctacgtg gtagacagag ccgcatactc ccttaccctc 120
ttcgacgatg agtttgagaa gaaggaccgg acatacccag tgggagagaa acttcgcaat 180
gccttcagat gttcctcagc caagatcaaa gctgtggtgt ttgggctgct gcctgtgctc 240
tcctggctcc ccaagtacaa gattaaagac tacatcattc ctgacctgct cggtggactc 300
agcgggggat ccatccaggt cccacaaggt atggcatttg ctctgctggc caaccttcct 360
gcagtcaatg gcctctactc ctccttcttc cccctcctga cctacttctt cctggggggt 420
gttcaccaga tggtgccagg tacctttgcc gttatcagca tcctggtggg taacatctgt 480
ctgcagctgg ccccagagtc gaaattccag gtcttcaaca atgccaccaa tgagagctat 540
gtggacacag cagccatgga ggctgagagg ctgcacgtgt cagctacgct agcctgcctc 600
accgccatca tccagatggg tctgggcttc atgcagtttg gctttgtggc catctacctc 660
tccgagtcct tcatccgggg cttcatgacg gccgccggcc tgcagatcct gatttcggtg 720
ctcaagtaca tcttcggact gaccatcccc tcctacacag gcccagggtc catcgtcttt 780
gtgagtctgg ggatgtgcaa aaacctcccc cacaccaaca tcgcctcgct catcttcgct 840
ctcatcagcg gtgccttcct ggtgctggtg aaggagctca atgctcgcta catgcacaag 900
attcgcttcc ccatccctac agagatgatt gtggtaagga ccttgttcag agctgggtgt 960
aagatgccca aaaagtatca catgcagatc gtgggagaaa tccaactcgg caggttcccc 1020
accccggtgt cgcctgtggt ctcacagtgg aaggacatga taggcacagc cttctcccta 1080
gccatcgtga gctacgtcat caacctggct atgggccgga ccctggccaa caagcacggc 1140
tacgacgtgg attcgaacca ggagatgatc gctctcggct gcagcaactt ctttggctcc 1200
ttctttaaaa ttcatgtcat ttgctgtgcg ctttctgtca ctctggctgt ggatggagct 1260
ggaggaaaat cccaggtgag ccttgttcta ggggagttgt ctgagctccc cttcttactc 1320
accacggggt ttgctttaag agtactcagg tgtctctctg tgctaggagc cctgatcgct 1380
gtcaatctca agaactccct caagcaactc accgacccct actacctgtg gaggaagagc 1440
aagctggacc agtgcatctg ggtagtgagc ttcctctcct ccttcttcct cagcctgccc 1500
tatggtgtgg cagtgggtgt cgccttctcc gtcctggtcg tggtcttcca gactcagagt 1560
cgaaatggct atgcactggc ccaggtcatg gacactgaca tttatgtgaa tcccaagacc 1620
tataataggg tacaggatat ccaggggatt aaaatcatca cgtactgctc ccctctctac 1680
tttgccaact cagagatctt caggcaaaag gtcatcgcca aggtaaggct cagtccctgg 1740
cgaccagagg ctctggacag agagtggccg gaaaatggaa gcagaagggc ggtgggaccc 1800
aacaacaacc agaccccggc taacggcacc agcgtgtcct atatcacctt cagccctgac 1860
agctcctcac ctgcccagag tgagccacca gcctccgctg aggccccggg cgagcccagt 1920
gacatgctgg ccagcgtccc acccttcgtc accttccaca ccctcatcct cgacatgagt 1980
ggagtcagct tcgtggactt gatgggcatc aaggccctgg ccaagctgag ctccacctat 2040
gggaagatcg gcgtaaaggt cttcttggtg aacatccatg cccaggtgta caatgacatt 2100
agccatggag gcgtctttga ggatgggagc ctaggatgca agcacgtctt tcccagcata 2160
catgacgcag tcctctttgc ccagctgatt cagttacctg gattgaggtc actggcaatg 2220
gctgaagtgg agacgcaggt ggaactggtt caggccgggg gaatcaccca cttgagtttg 2280
tactaaaagc cccagcccag ccctgtttct ctt 2313

<210> 116
<211> 748
<212> PRT
<213> Homo sapiens

<400> 116
Met Pro Ala Glu Asp Asn Glu Thr Glu Ala Gln Gln His Asp Arg Tyr
1               5                   10                  15

Val Val Asp Arg Ala Ala Tyr Ser Leu Thr Leu Phe Asp Asp Glu Phe
            20                  25                  30

Glu Lys Lys Asp Arg Thr Tyr Pro Val Gly Glu Lys Leu Arg Asn Ala
        35                  40                  45

Phe Arg Cys Ser Ser Ala Lys Ile Lys Ala Val Val Phe Gly Leu Leu
    50                  55                  60

Pro Val Leu Ser Trp Leu Pro Lys Tyr Lys Ile Lys Asp Tyr Ile Ile
65                  70                  75                  80

Pro Asp Leu Leu Gly Gly Leu Ser Gly Gly Ser Ile Gln Val Pro Gln
                85                  90                  95

Gly Met Ala Phe Ala Leu Leu Ala Asn Leu Pro Ala Val Asn Gly Leu
            100                 105                 110

Tyr Ser Ser Phe Phe Pro Leu Leu Thr Tyr Phe Phe Leu Gly Gly Val
        115                 120                 125

His Gln Met Val Pro Gly Thr Phe Ala Val Ile Ser Ile Leu Val Gly
    130                 135                 140

Asn Ile Cys Leu Gln Leu Ala Pro Glu Ser Lys Phe Gln Val Phe Asn
145                 150                 155                 160

Asn Ala Thr Asn Glu Ser Tyr Val Asp Thr Ala Ala Met Glu Ala Glu
                165                 170                 175

Arg Leu His Val Ser Ala Thr Leu Ala Cys Leu Thr Ala Ile Ile Gln
            180                 185                 190

Met Gly Leu Gly Phe Met Gln Phe Gly Phe Val Ala Ile Tyr Leu Ser
        195                 200                 205

Glu Ser Phe Ile Arg Gly Phe Met Thr Ala Ala Gly Leu Gln Ile Leu
    210                 215                 220

Ile Ser Val Leu Lys Tyr Ile Phe Gly Leu Thr Ile Pro Ser Tyr Thr
225                 230                 235                 240

Gly Pro Gly Ser Ile Val Phe Val Ser Leu Gly Met Cys Lys Asn Leu
                245                 250                 255

Pro His Thr Asn Ile Ala Ser Leu Ile Phe Ala Leu Ile Ser Gly Ala
            260                 265                 270

Phe Leu Val Leu Val Lys Glu Leu Asn Ala Arg Tyr Met His Lys Ile
        275                 280                 285

Arg Phe Pro Ile Pro Thr Glu Met Ile Val Val Arg Thr Leu Phe Arg
    290                 295                 300

Ala Gly Cys Lys Met Pro Lys Lys Tyr His Met Gln Ile Val Gly Glu
305                 310                 315                 320

Ile Gln Leu Gly Arg Phe Pro Thr Pro Val Ser Pro Val Val Ser Gln
                325                 330                 335

Trp Lys Asp Met Ile Gly Thr Ala Phe Ser Leu Ala Ile Val Ser Tyr
            340                 345                 350

Val Ile Asn Leu Ala Met Gly Arg Thr Leu Ala Asn Lys His Gly Tyr
        355                 360                 365

Asp Val Asp Ser Asn Gln Glu Met Ile Ala Leu Gly Cys Ser Asn Phe
    370                 375                 380

Phe Gly Ser Phe Phe Lys Ile His Val Ile Cys Cys Ala Leu Ser Val
385                 390                 395                 400

Thr Leu Ala Val Asp Gly Ala Gly Gly Lys Ser Gln Val Ser Leu Val
                405                 410                 415

Leu Gly Glu Leu Ser Glu Leu Pro Phe Leu Leu Thr Thr Gly Phe Ala
            420                 425                 430

Leu Arg Val Leu Arg Cys Leu Ser Val Leu Gly Ala Leu Ile Ala Val
        435                 440                 445

Asn Leu Lys Asn Ser Leu Lys Gln Leu Thr Asp Pro Tyr Tyr Leu Trp
    450                 455                 460

Arg Lys Ser Lys Leu Asp Gln Cys Ile Trp Val Val Ser Phe Leu Ser
465                 470                 475                 480

Ser Phe Phe Leu Ser Leu Pro Tyr Gly Val Ala Val Gly Val Ala Phe
                485                 490                 495

Ser Val Leu Val Val Val Phe Gln Thr Gln Ser Arg Asn Gly Tyr Ala
            500                 505                 510

Leu Ala Gln Val Met Asp Thr Asp Ile Tyr Val Asn Pro Lys Thr Tyr
        515                 520                 525

Asn Arg Val Gln Asp Ile Gln Gly Ile Lys Ile Ile Thr Tyr Cys Ser
    530                 535                 540

Pro Leu Tyr Phe Ala Asn Ser Glu Ile Phe Arg Gln Lys Val Ile Ala
545                 550                 555                 560

Lys Val Arg Leu Ser Pro Trp Arg Pro Glu Ala Leu Asp Arg Glu Trp
                565                 570                 575

Pro Glu Asn Gly Ser Arg Arg Ala Val Gly Pro Asn Asn Asn Gln Thr
            580                 585                 590

Pro Ala Asn Gly Thr Ser Val Ser Tyr Ile Thr Phe Ser Pro Asp Ser
        595                 600                 605

Ser Ser Pro Ala Gln Ser Glu Pro Pro Ala Ser Ala Glu Ala Pro Gly
    610                 615                 620

Glu Pro Ser Asp Met Leu Ala Ser Val Pro Pro Phe Val Thr Phe His
625                 630                 635                 640

Thr Leu Ile Leu Asp Met Ser Gly Val Ser Phe Val Asp Leu Met Gly
                645                 650                 655

Ile Lys Ala Leu Ala Lys Leu Ser Ser Thr Tyr Gly Lys Ile Gly Val
            660                 665                 670

Lys Val Phe Leu Val Asn Ile His Ala Gln Val Tyr Asn Asp Ile Ser
        675                 680                 685

His Gly Gly Val Phe Glu Asp Gly Ser Leu Gly Cys Lys His Val Phe
    690                 695                 700

Pro Ser Ile His Asp Ala Val Leu Phe Ala Gln Leu Ile Gln Leu Pro
705                 710                 715                 720

Gly Leu Arg Ser Leu Ala Met Ala Glu Val Glu Thr Gln Val Glu Leu
                725                 730                 735

Val Gln Ala Gly Gly Ile Thr His Leu Ser Leu Tyr
            740                 745

<210> 117
<211> 1335
<212> DNA
<213> Homo sapiens

<400> 117
   gccttcctgt ccggctgcat ccagctggcc atgggggtcc tgcgtttggg gttcctgctg 60
   gacttcattt cctaccccgt cattaaaggc ttcacctctg ctgctgccgt caccatcggc 120
   tttggacaga tcaagaacct gctgggacta cagaacatcc ccaggccgtt cttcctgcag 180
   gtgtaccaca ccttcctcag gattgcagag accagggtag gtgacgccgt cctggggctg 240
   gtctgcatgc tgctgctgct ggtgctgaag ctgatgcggg accacgtgcc tcccgtccac 300
   cccgagatgc cccctggtgt gcggctcagc cgtgggctgg tctgggctgc cacgacagct 360
   cgcaacgccc tggtggtctc cttcgcagcc ctggttgcgt actccttcga ggtgactgga 420
   taccagcctt tcatcctaac aggggagaca gctgaggggc tccctccagt ccggatcccg 480
   cccttctcag tgaccacagc caacgggacg atctccttca ccgagatggt gcaggacatg 540
   ggagccgggc tggccgtggt gcccctgatg ggcctcctgg agagcattgc ggtggccaaa 600
   gccttcgcat ctcagaataa ttaccgcatc gatgccaacc aggagctgct ggccatcggt 660
   ctcaccaaca tgttgggctc cctcgtctcc tcctacccgg tcacaggcag ctttggacgg 720
   acagccgtga acgctcagtc gggggtgtgc accccggcgg ggggcctggt gacgggagtg 780
   ctggtgctgc tgtctctgga ctacctgacc tcactgttct actacatccc caagtctgcc 840
   ctggctgccg tcatcatcat ggccgtggcc ccgctgttcg acaccaagat cttcaggacg 900
   ctctggcgtg ttaagaggct ggacctgctg cccctgtgcg tgaccttcct gctgtgcttc 960
   tgggaggtgc agtacggcat cctggccggg gccctggtgt ctctgctcat gctcctgcac 1020
   tctgcagcca ggcctgagac caaggtgtca gaggggccgg ttctggtcct gcagccggcc 1080
   agcggcctgt ccttccctgt cctctgcccc ccactccctg ctgttcagga ccccaagacc 1140
   ctgtccccga cgctctccag tccacaagga tgcaggcatc tctgagtggg ctggaccgtc 1200
   ctctgtgggc ctcagccagt ggtgctgcag caagggtggt ggctccccac atatcactcc 1260
   ttccctgccc ctaaagtccg gttcctgttt ctgggggttt gattttaggg gagctaaggg 1320
   cctgtgagtc ctagt 1335

<210> 118
<211> 384
<212> PRT
<213> Homo sapiens

<400> 118
Met Gly Val Leu Arg Leu Gly Phe Leu Leu Asp Phe Ile Ser Tyr Pro
1               5                   10                  15

Val Ile Lys Gly Phe Thr Ser Ala Ala Ala Val Thr Ile Gly Phe Gly
            20                  25                  30

Gln Ile Lys Asn Leu Leu Gly Leu Gln Asn Ile Pro Arg Pro Phe Phe
        35                  40                  45

Leu Gln Val Tyr His Thr Phe Leu Arg Ile Ala Glu Thr Arg Val Gly
    50                  55                  60

Asp Ala Val Leu Gly Leu Val Cys Met Leu Leu Leu Leu Val Leu Lys
65                  70                  75                  80

Leu Met Arg Asp His Val Pro Pro Val His Pro Glu Met Pro Pro Gly
                85                  90                  95

Val Arg Leu Ser Arg Gly Leu Val Trp Ala Ala Thr Thr Ala Arg Asn
            100                 105                 110

Ala Leu Val Val Ser Phe Ala Ala Leu Val Ala Tyr Ser Phe Glu Val
        115                 120                 125

Thr Gly Tyr Gln Pro Phe Ile Leu Thr Gly Glu Thr Ala Glu Gly Leu
    130                 135                 140

Pro Pro Val Arg Ile Pro Pro Phe Ser Val Thr Thr Ala Asn Gly Thr
145                 150                 155                 160

Ile Ser Phe Thr Glu Met Val Gln Asp Met Gly Ala Gly Leu Ala Val
                165                 170                 175

Val Pro Leu Met Gly Leu Leu Glu Ser Ile Ala Val Ala Lys Ala Phe
            180                 185                 190

Ala Ser Gln Asn Asn Tyr Arg Ile Asp Ala Asn Gln Glu Leu Leu Ala
        195                 200                 205

Ile Gly Leu Thr Asn Met Leu Gly Ser Leu Val Ser Ser Tyr Pro Val
    210                 215                 220

Thr Gly Ser Phe Gly Arg Thr Ala Val Asn Ala Gln Ser Gly Val Cys
225                 230                 235                 240

Thr Pro Ala Gly Gly Leu Val Thr Gly Val Leu Val Leu Leu Ser Leu
                245                 250                 255

Asp Tyr Leu Thr Ser Leu Phe Tyr Tyr Ile Pro Lys Ser Ala Leu Ala

            260                 265                 270

Ala Val Ile Ile Met Ala Val Ala Pro Leu Phe Asp Thr Lys Ile Phe
        275                 280                 285

Arg Thr Leu Trp Arg Val Lys Arg Leu Asp Leu Leu Pro Leu Cys Val
    290                 295                 300

Thr Phe Leu Leu Cys Phe Trp Glu Val Gln Tyr Gly Ile Leu Ala Gly
305                 310                 315                 320

Ala Leu Val Ser Leu Leu Met Leu Leu His Ser Ala Ala Arg Pro Glu
                325                 330                 335

Thr Lys Val Ser Glu Gly Pro Val Leu Val Leu Gln Pro Ala Ser Gly
            340                 345                 350

Leu Ser Phe Pro Val Leu Cys Pro Pro Leu Pro Ala Val Gln Asp Pro
        355                 360                 365

Lys Thr Leu Ser Pro Thr Leu Ser Ser Pro Gln Gly Cys Arg His Leu
    370                 375                 380

<210> 119
<211> 2079
<212> DNA
<213> Homo sapiens

<400> 119
atggttgtgg ctgtcacaga ttttaactcc tcggtgcacc aagagtacag attccaaatt 60
gcctatatct taaaaacttg taagaaagaa gcaatggtga atgtgaatct gaacaccagg 120
gagagttcca gaaagggaat tcccatcagt tggtactacc taataatggg cgtattgggt 180
ttgggcttca ttgccactta ccttccggag tctgcaatga gtgcttacct ggctgctgtg 240
gcacttcata tcatgctgtc ccagctgact ttcatctttg ggattatgat tagtttccat 300
gccggtccca tctccttctt ctatgacata attaattact gtgtagctct cccaaaagcg 360
aattccacca gcattctagt atttctaact gttgttgttg ctctgcgaat caacaaatgt 420
atcagaattt ctttcaatca gtatcccatt gagtttccca tggaattatt tctgattatt 480
ggcttcactg tgattgcaaa caagataagc atggccacag aaaccagcca gacgcttatt 540
gacatgattc cttatagctt tctgcttcct gtaacaccag atttcagcct tcttcccaag 600
ataattttac aagccttctc cttatctttg gtgagctcct ttctgctcat atttctgggc 660
aagaagattg ccagtcttca caattacagt gtcaattcca accaggattt aatagccatc 720
ggcctttgca atgtcgtcag ttcatttttc agatcttgtg tgtttactgg tgctattgct 780
aggactatta tccaggataa atctggagga agacaacagt ttgcatctct ggtaggcgca 840
ggtgtgatgc tgctcctgat ggtgaagatg ggacactttt tctacacact gccaaatgtt 900
gatatggtaa aggtgcctct taaagaagaa gaaattttca gcttgtttaa ttcaagtgac 960
accaatctac aaggaggaaa gatttgcagg tgtttctgca actgtgatga tctggagccg 1020
ctgcccagga ttctttacac agagcgattt gaaaataaac tggatcccga agcatcctcc 1080
attaacctga ttcactgctc acattttgag agcatgaaca caagccaaac tgcatccgaa 1140
gaccaagtgc catacacagt atcgtccgtg tctcagaaaa atcaaggcca acagtatgag 1200
gaggtggagg aagtttggct tcctaataac tcatcaagaa acagctcacc aggactgcct 1260
gatgtggcgg aaagccaggg gaggagatca ctcatccctt actcagatgc gtctctactg 1320
cccagtgtcc acaccatcat cctggatttc tccatggtac actacgtgga ttcacggggg 1380
ttagtcgtat taagacagat atgcaatgcc tttcaaaacg ccaacatttt gatactcatt 1440
gcagggtgtc actcttccat agtcagggca tttgagagga atgatttctt tgacgctggc 1500
atcaccaaga cccagctgtt cctcagcgtt cacgacgccg tgctgtttgc cttgtcaagg 1560
aaggtcatag gctcctctga gttaagcatc gatgaatccg agacagtgat acgggaaacc 1620
tactcagaaa cagacaagaa tgacaattca agatataaaa tgagcagcag ttttctagga 1680
agccaaaaaa atgtaagtcc aggcttcatc aagatccaac agcctgtaga agaggagtcg 1740
gagttggatt tggagctgga atcagaacaa gaggctgggc tgggtctgga cctagacctg 1800
gatcgggagc tggagcctga aatggagccc aaggctgaga ccgagaccaa gacccagacc 1860
gagatggagc cccagcctga gactgagcct gagatggagc ccaaccccaa atctaggcca 1920
agagctcaca cttttcctca gcagcgttac tggcctatgt atcatccgtc tatggcttcc 1980
acccagtctc agactcagac tcggacatgg tcagtggaga ggagacgcca tcctatggat 2040
tcatactcac cagagggcaa cagcaatgaa gatgtctag 2079

<210> 120
<211> 692
<212> PRT
<213> Homo sapiens

<400> 120
Met Val Val Ala Val Thr Asp Phe Asn Ser Ser Val His Gln Glu Tyr
1               5                   10                  15

Arg Phe Gln Ile Ala Tyr Ile Leu Lys Thr Cys Lys Lys Glu Ala Met
            20                  25                  30

Val Asn Val Asn Leu Asn Thr Arg Glu Ser Ser Arg Lys Gly Ile Pro
        35                  40                  45

Ile Ser Trp Tyr Tyr Leu Ile Met Gly Val Leu Gly Leu Gly Phe Ile
    50                  55                  60

Ala Thr Tyr Leu Pro Glu Ser Ala Met Ser Ala Tyr Leu Ala Ala Val
65                  70                  75                  80

Ala Leu His Ile Met Leu Ser Gln Leu Thr Phe Ile Phe Gly Ile Met
                85                  90                  95

Ile Ser Phe His Ala Gly Pro Ile Ser Phe Phe Tyr Asp Ile Ile Asn
            100                 105                 110

Tyr Cys Val Ala Leu Pro Lys Ala Asn Ser Thr Ser Ile Leu Val Phe
        115                 120                 125

Leu Thr Val Val Val Ala Leu Arg Ile Asn Lys Cys Ile Arg Ile Ser
    130                 135                 140

Phe Asn Gln Tyr Pro Ile Glu Phe Pro Met Glu Leu Phe Leu Ile Ile
145                 150                 155                 160

Gly Phe Thr Val Ile Ala Asn Lys Ile Ser Met Ala Thr Glu Thr Ser
                165                 170                 175

Gln Thr Leu Ile Asp Met Ile Pro Tyr Ser Phe Leu Leu Pro Val Thr
            180                 185                 190

Pro Asp Phe Ser Leu Leu Pro Lys Ile Ile Leu Gln Ala Phe Ser Leu
        195                 200                 205

Ser Leu Val Ser Ser Phe Leu Leu Ile Phe Leu Gly Lys Lys Ile Ala
    210                 215                 220

Ser Leu His Asn Tyr Ser Val Asn Ser Asn Gln Asp Leu Ile Ala Ile
225                 230                 235                 240

Gly Leu Cys Asn Val Val Ser Ser Phe Phe Arg Ser Cys Val Phe Thr
                245                 250                 255

Gly Ala Ile Ala Arg Thr Ile Ile Gln Asp Lys Ser Gly Gly Arg Gln
            260                 265                 270

Gln Phe Ala Ser Leu Val Gly Ala Gly Val Met Leu Leu Leu Met Val
        275                 280                 285

Lys Met Gly His Phe Phe Tyr Thr Leu Pro Asn Val Asp Met Val Lys
    290                 295                 300

Val Pro Leu Lys Glu Glu Glu Ile Phe Ser Leu Phe Asn Ser Ser Asp
305                 310                 315                 320

Thr Asn Leu Gln Gly Gly Lys Ile Cys Arg Cys Phe Cys Asn Cys Asp
                325                 330                 335

Asp Leu Glu Pro Leu Pro Arg Ile Leu Tyr Thr Glu Arg Phe Glu Asn
            340                 345                 350

Lys Leu Asp Pro Glu Ala Ser Ser Ile Asn Leu Ile His Cys Ser His
        355                 360                 365

Phe Glu Ser Met Asn Thr Ser Gln Thr Ala Ser Glu Asp Gln Val Pro
    370                 375                 380

Tyr Thr Val Ser Ser Val Ser Gln Lys Asn Gln Gly Gln Gln Tyr Glu
385                 390                 395                 400

Glu Val Glu Glu Val Trp Leu Pro Asn Asn Ser Ser Arg Asn Ser Ser
                405                 410                 415

Pro Gly Leu Pro Asp Val Ala Glu Ser Gln Gly Arg Arg Ser Leu Ile
            420                 425                 430

Pro Tyr Ser Asp Ala Ser Leu Leu Pro Ser Val His Thr Ile Ile Leu
        435                 440                 445

Asp Phe Ser Met Val His Tyr Val Asp Ser Arg Gly Leu Val Val Leu
    450                 455                 460

Arg Gln Ile Cys Asn Ala Phe Gln Asn Ala Asn Ile Leu Ile Leu Ile
465                 470                 475                 480

Ala Gly Cys His Ser Ser Ile Val Arg Ala Phe Glu Arg Asn Asp Phe
                485                 490                 495

Phe Asp Ala Gly Ile Thr Lys Thr Gln Leu Phe Leu Ser Val His Asp
            500                 505                 510

Ala Val Leu Phe Ala Leu Ser Arg Lys Val Ile Gly Ser Ser Glu Leu
        515                 520                 525

Ser Ile Asp Glu Ser Glu Thr Val Ile Arg Glu Thr Tyr Ser Glu Thr
    530                 535                 540

Asp Lys Asn Asp Asn Ser Arg Tyr Lys Met Ser Ser Ser Phe Leu Gly
545                 550                 555                 560

Ser Gln Lys Asn Val Ser Pro Gly Phe Ile Lys Ile Gln Gln Pro Val
                565                 570                 575

Glu Glu Glu Ser Glu Leu Asp Leu Glu Leu Glu Ser Glu Gln Glu Ala
            580                 585                 590

Gly Leu Gly Leu Asp Leu Asp Leu Asp Arg Glu Leu Glu Pro Glu Met
        595                 600                 605

Glu Pro Lys Ala Glu Thr Glu Thr Lys Thr Gln Thr Glu Met Glu Pro
    610                 615                 620

Gln Pro Glu Thr Glu Pro Glu Met Glu Pro Asn Pro Lys Ser Arg Pro
625                 630                 635                 640

Arg Ala His Thr Phe Pro Gln Gln Arg Tyr Trp Pro Met Tyr His Pro
                645                 650                 655

Ser Met Ala Ser Thr Gln Ser Gln Thr Gln Thr Arg Thr Trp Ser Val
            660                 665                 670

Glu Arg Arg Arg His Pro Met Asp Ser Tyr Ser Pro Glu Gly Asn Ser
        675                 680                 685

Asn Glu Asp Val
    690

<210> 121
<211> 2210
<212> DNA
<213> Homo sapiens

<400> 121
tgcagttggc gggcgcatgt gggggcgcac ggcgcggcgg cgctgcccgc gggaactgcg 60
gcgcggccgg gaggcgctgt tggtgctcct ggcgctactg gcgttggccg ggctgggctc 120
ggtgctgcgg gcgcagcgtg gggccggggc cggggctgcc gagccgggac ccccgcgcac 180
cccgcgcccc gggcggcgcg agccggtcat gccgcggccg ccggtgccgg cgaacgcgct 240
gggcgcgcgg ggcgaggcgg tgcggctgca gctgcagggc gaggagctgc ggctgcagga 300
ggagagcgtg cggctgcacc agattaacat ctacctcagc gaccgcatct cactgcaccg 360
ccgcctgccc gagcgctgga acccgctgtg caaagagaag aaatatgatt atgataattt 420
gcccaggaca tctgttatca tagcatttta taatgaagcc tggtcaactc tccttcggac 480
agtttacagt gtccttgaga catccccgga tatcctgcta gaagaagtga tccttgtaga 540
tgactacagt gatagagagc acctgaagga gcgcttggcc aatgagcttt cgggactgcc 600
caaggtgcgc ctgatccgcg ccaacaagag agagggcctg gtgcgagccc ggctgctggg 660
ggcgtctgcg gcgaggggcg atgttctgac cttcctggac tgtcactgtg agtgccacga 720
agggtggctg gagccgctgc tgcagaggat ccatgaagag gagtcggcag tggtgtgccc 780
ggtgattgat gtgatcgact ggaacacctt cgaatacctg gggaactccg gggagcccca 840
gatcggcggt ttcgactgga ggctggtgtt cacgtggcac acagttcctg agagggagag 900
gatacggatg caatcccccg tcgatgtcat caggtctcca acaatggctg gtgggctgtt 960
tgctgtgagt aagaaatatt ttgaatatct ggggtcttat gatacaggaa tggaagtttg 1020
gggaggagaa aacctcgaat tttcctttag gatctggcag tgtggtgggg ttctggaaac 1080
acacccatgt tcccatgttg gccatgtttt ccccaagcaa gctccctact cccgcaacaa 1140
ggctctggcc aacagtgttc gtgcagctga agtatggatg gatgaattta aagagctcta 1200
ctaccatcgc aacccccgtg cccgcttgga accttttggg gatgtgacag agaggaagca 1260
gctccgggac aagctccagt gtaaagactt caagtggttc ttggagactg tgtatccaga 1320
actgcatgtg cctgaggaca ggcctggctt cttcgggatg ctccagaaca aaggactaac 1380
agactactgc tttgactata accctcccga tgaaaaccag attgtgggac accaggtcat 1440
tctgtacctc tgtcatggga tgggccagaa tcagtttttc gagtacacgt cccagaaaga 1500
aatacgctat aacacccacc agcctgaggg ctgcattgct gtggaagcag gaatggatac 1560
ccttatcatg catctctgcg aagaaactgc cccagagaat cagaagttca tcttgcagga 1620
ggatggatct ttatttcacg aacagtccaa gaaatgtgtc caggctgcga ggaaggagtc 1680
gagtgacagt ttcgttccac tcttacgaga ctgcaccaac tcggatcatc agaaatggtt 1740
cttcaaagag cgcatgttat gaagcctcgt gtatcaagga gccccatcga aggagactgt 1800
ggagccagga ctctgcccaa caaagactta gctaagcagt gaccagaacc acaaaaacta 1860
ggctggattg cttttgcaag aggcaatcat ttgccctttg tgaaagtgtg tggattaggt 1920
aacagtgata gctgtactat ttggcacctt ctaatgttca aatacctatt tccaggtact 1980
cagatggtac cctgttttg aattaacctt taattttctt caaacgtatt taacacgcgg 2040
cctaacttct agacaagaaa gatcttcggg ggtcacaacc cccgaagaat tcggcggacc 2100
gtccaccctg ctactagtca cccgcggagc caacaacgcc aagcgctgca tcacactcta 2160
gcacggcggc ccacacgaac acatcaagca gaggcagata ccataatagt 2210

<210> 122
<211> 581
<212> PRT
<213> Homo sapiens

<400> 122


<210> 123
<211> 2030
<212> DNA
<213> Homo sapiens

<400> 123
tttttttttt ttttgacaat cacccagtca gtatttatta ggcgcctact gcggacggtt 60
ggtcttcacg cagatcaggc gagaagggat agtgatgcgc gggccgctgc ggagacccgg 120
agcccgccgc ggatcacggg aatttcgcgc ctattttttg ttggctgggt gttctcgcca 180
gtgattgggt cccggcaagg cgtgccggtg cgctcggctg cggctgctcc gtcgaccttt 240
gcaggaccgg gcggtgcagg gctcactcgg ctggcgtccc gggggatggg ccaccaggag 300
tctccgctgg cccgggcgcc ggcgggaggt gcagcttatg taaagaggtt atgtaaaggg 360
ctcagctggc gcgaacacgt ggaaagccac gggagcctag gagcccaggc ttccccagcg 420
agcgccgcgg cagcagaagg atccgctaca cgccgggctc gggccgccac ctcccgcgct 480
gctcggtccc ggaggcagcc cgggcccgga gcggaccatc cccaggcagg ggctccaggg 540
gggaaacggg ccgcccggaa gtggaggtgc gcgggccagg tcacaatcca aggtccggct 600
cctccgcgtc ccagggccgg acggagggat gaggcagggg gggcccgggc agcgccgttg 660
ctgctccccc cgccgcccgc agccatggaa acggggaagg acggcgcccg cagaggtaca 720
caaagcccgg agcggaaaag gcgaagccca gtgccgcggg cgcccagcac gaagctgagg 780
ccggcggcgg cggcccgggc catggatccg gtggcggccg aggccccggg cgaggccttc 840
ctggcgcggc gacggcctga gggcggtggc gggtccgcgc ggccgcgtta cagcctgttg 900
gcggagatcg ggcgcggcag ctacggcgtg gtttatgagg cagtggccgg gcgcagcggg 960
gcccgggtgg cggtcaagaa gatccgctgc gacgcccccg agaacgtgga gctggcgctg 1020
gctgaattct gggccctcac cagcctcaag cggcgccacc agaacgtcgt gcagtttgag 1080
gagtgcgtcc tgcagcgcaa tgggttagcc cagcgcatga gtcacggcaa caagagctcg 1140
cagctttacc tgcgcctggt ggagacctcg ctgaaagaaa ggatcctggg ttatgctgag 1200
gagccctgct atctctggtt tgtcatggag ttctgtgaag gtggagacct gaatcagtat 1260
gtcctgtccc ggaggccaga cccagccacc aacaaaagtt tcatgctaca gctgacgagc 1320
gccattgcct tcctgcacaa aaaccatatt gtgcacaggg acctgaagcc agacaacatc 1380
ctcatcacag agcggtctgg cacccccatc ctcaaagtgg ccgactttgg actaagcaag 1440
gtctgtgctg ggctggcacc ccgaggcaaa gagggcaatc aagacaacaa aaatgtgaat 1500
gtgaataagt actggctgtc ctcagcctgc ggttcggact tctacatggc tcctgaagtc 1560
tgggagggac actacacagc caaggcggac atctttgccc tgggcattat catctgggca 1620
atgatagaaa gaatcacttt tattgactct gagaccaaga aggagctcct ggggacctac 1680
attaaacagg ggactgagat cgtccctgtt ggtgaggcgc tgctagaaaa cccaaagatg 1740
gagttgcaca tcccccaaaa acgcaggact tccatgtctg aggggatcaa gcagctcttg 1800
aaagatatgt tagctgctaa cccacaggac cggcctgatg cctttgaact tgaaaccaga 1860
atggaccagg tcacatgtgc tgcttaaaat tcagggctaa gcattttggg tgattttaaa 1920
ctaggtcgat tcctcgggac ccacagtctc accacgtctc ctccagagga cggcagaggg 1980
tacaggtggt ggcctggccg gttggcgatc tcccgacagc tggatccggc 2030

<210> 124
<211> 533
<212> PRT
<213> Homo sapiens

<400> 124
Met Gly His Gln Glu Ser Pro Leu Ala Arg Ala Pro Ala Gly Gly Ala
1               5                   10                  15

Ala Tyr Val Lys Arg Leu Cys Lys Gly Leu Ser Trp Arg Glu His Val
            20                  25                  30

Glu Ser His Gly Ser Leu Gly Ala Gln Ala Ser Pro Ala Ser Ala Ala
        35                  40                  45

Ala Ala Glu Gly Ser Ala Thr Arg Arg Ala Arg Ala Ala Thr Ser Arg
    50                  55                  60

Ala Ala Arg Ser Arg Arg Gln Pro Gly Pro Gly Ala Asp His Pro Gln
65                  70                  75                  80

Ala Gly Ala Pro Gly Gly Lys Arg Ala Ala Arg Lys Trp Arg Cys Ala
                85                  90                  95

Gly Gln Val Thr Ile Gln Gly Pro Ala Pro Pro Arg Pro Arg Ala Gly
            100                 105                 110

Arg Arg Asp Glu Ala Gly Gly Ala Arg Ala Ala Pro Leu Leu Leu Pro
        115                 120                 125

Pro Pro Pro Ala Ala Met Glu Thr Gly Lys Asp Gly Ala Arg Arg Gly
    130                 135                 140

Thr Gln Ser Pro Glu Arg Lys Arg Arg Ser Pro Val Pro Arg Ala Pro
145                 150                 155                 160

Ser Thr Lys Leu Arg Pro Ala Ala Ala Ala Arg Ala Met Asp Pro Val
                165                 170                 175

Ala Ala Glu Ala Pro Gly Glu Ala Phe Leu Ala Arg Arg Arg Pro Glu
            180                 185                 190

Gly Gly Gly Gly Ser Ala Arg Pro Arg Tyr Ser Leu Leu Ala Glu Ile
        195                 200                 205

Gly Arg Gly Ser Tyr Gly Val Val Tyr Glu Ala Val Ala Gly Arg Ser
    210                 215                 220

Gly Ala Arg Val Ala Val Lys Lys Ile Arg Cys Asp Ala Pro Glu Asn
225                 230                 235                 240

Val Glu Leu Ala Leu Ala Glu Phe Trp Ala Leu Thr Ser Leu Lys Arg
                245                 250                 255

Arg His Gln Asn Val Val Gln Phe Glu Glu Cys Val Leu Gln Arg Asn
            260                 265                 270

Gly Leu Ala Gln Arg Met Ser His Gly Asn Lys Ser Ser Gln Leu Tyr
        275                 280                 285

Leu Arg Leu Val Glu Thr Ser Leu Lys Glu Arg Ile Leu Gly Tyr Ala
    290                 295                 300

Glu Glu Pro Cys Tyr Leu Trp Phe Val Met Glu Phe Cys Glu Gly Gly
305                 310                 315                 320

Asp Leu Asn Gln Tyr Val Leu Ser Arg Arg Pro Asp Pro Ala Thr Asn
                325                 330                 335

Lys Ser Phe Met Leu Gln Leu Thr Ser Ala Ile Ala Phe Leu His Lys
            340                 345                 350

Asn His Ile Val His Arg Asp Leu Lys Pro Asp Asn Ile Leu Ile Thr
        355                 360                 365

Glu Arg Ser Gly Thr Pro Ile Leu Lys Val Ala Asp Phe Gly Leu Ser
    370                 375                 380

Lys Val Cys Ala Gly Leu Ala Pro Arg Gly Lys Glu Gly Asn Gln Asp
385                 390                 395                 400

Asn Lys Asn Val Asn Val Asn Lys Tyr Trp Leu Ser Ser Ala Cys Gly
                405                 410                 415

Ser Asp Phe Tyr Met Ala Pro Glu Val Trp Glu Gly His Tyr Thr Ala
            420                 425                 430

Lys Ala Asp Ile Phe Ala Leu Gly Ile Ile Ile Trp Ala Met Ile Glu
        435                 440                 445

Arg Ile Thr Phe Ile Asp Ser Glu Thr Lys Lys Glu Leu Leu Gly Thr
    450                 455                 460

Tyr Ile Lys Gln Gly Thr Glu Ile Val Pro Val Gly Glu Ala Leu Leu
465                 470                 475                 480

Glu Asn Pro Lys Met Glu Leu His Ile Pro Gln Lys Arg Arg Thr Ser
                485                 490                 495

Met Ser Glu Gly Ile Lys Gln Leu Leu Lys Asp Met Leu Ala Ala Asn
            500                 505                 510

Pro Gln Asp Arg Pro Asp Ala Phe Glu Leu Glu Thr Arg Met Asp Gln
        515                 520                 525

Val Thr Cys Ala Ala
    530

<210> 125
<211> 3331
<212> DNA
<213> Homo sapiens

<400> 125
atggcgctgc ggagaggcgg ctgcggagcg ctcgggctgc tgctgctgct gctgggcgcc 60
gcgtgcctga taccgcggag cgcgcaggtg aggcggctgg cgcgctgccc cgccacttgc 120
agctgtacca aggagtctat catctgcgtg ggctcttcct gggtgcccag gatcgtgccg 180
ggcgacatca gctccctgag cctggtaaat gggacgtttt cagaaatcaa ggaccgaatg 240
ttttcccatc tgccttctct gcagctgcta ttgctgaatt ctaactcatt cacgatcatc 300
cgggatgatg cttttgctgg actttttcat cttgaatacc tgttcattga agggaacaaa 360
atagaaacca tttcaagaaa tgcctttcgt ggcctccgtg acctgactca cctttctttg 420
gccaataacc acataaaagc actaccaagg gatgtcttca gtgatttaga ctctctgatt 480
gaacagattt tgaggggtaa taaatttgaa tgtgactgca aagccaagtg gctatacctg 540
tggttgaaga tgacaaattc caccgtttct gatgtgctgt gtattggtcc accagagtat 600
caggaaaaga agctaaatga cgtgaccagc tttgactatg aatgcacaac tacagatttt 660
gttgttcatc agactttacc ctaccagtcg gtttcagtgg atacgttcaa ctccaagaac 720
gatgtgtacg tggccatcgc gcagcccagc atggagaact gcatggtgct ggagtgggac 780
cacattgaaa tgaatttccg gagctatgac aacattacag gtcagtccat cgtgggctgt 840
aaggccattc tcatcgatga tcaggtcttt gtggtggtag cccagctctt cggtggctct 900
cacatttaca aatacgacga gagttggacc aaatttgtca aattccaaga catagaggtc 960
tctcgcattt ccaagcccaa tgacatcgag ctgtttcaga tcgacgacga gacgttcttt 1020
gtcatcgcag acagctcaaa ggctggtctg tccacagttt ataaatggaa cagcaaagga 1080
ttctattctt accagtcact gcacgagtgg ttcagggaca cggatgcgga gtttgttgat 1140
atcgatggaa aatcgcatct catcctgtcc agccgctccc aggtccccat catcctccag 1200
tggaataaaa gctctaagaa gtttgtcccc catggtgaca tccccaacat ggaggacgta 1260
ctggctgtga agagcttccg aatgcaaaat accctctacc tttcccttac ccgcttcatc 1320
ggggactccc gggtcatgag gtggaacagt aagcagtttg tggagatcca agctcttcca 1380

tcccgggggg ccatgaccct gcagcccttt tcttttaaag ataatcacta cctggccctg 1440
gggagtgact atacattctc tcagatatac cagtgggata aagagaagca gctattcaaa 1500
aagtttaagg agatttatgt gcaggcgcct cgttcattca cagctgtctc caccgacagg 1560
agagatttct tttttgcatc cagtttcaaa gggaaaacaa agatttttga acatataatt 1620
gttgacttaa gtttgtgaag gtgtggtggg tgaaactaag agaaatgtag cattagctct 1680
cacaaaagag gaccaagaaa aatcaacaaa caaatcaaag ccaggctcag agctctgaaa 1740
ttaaaaagca ctgaaatagt tagatgtttt caaactttta gaactcacat tttaatcagg 1800
gattgcattt attggctaac tgcatgacat gcccattcta ccattttaaa aaaaatctta 1860
aagcctgtaa tttctgagaa aagagtacag catttactct tatcatctag aaatgtaata 1920
tgcttccccc ccgctttttg atgaggaaga agacaattgg ataagatggg acagcactta 1980
taatgaaata aaaaaaaact ttgagcccct ctcattccac tttagcaatc tttttggtaa 2040
gaactcttaa agccaaaagt ctgctgaaaa gatttgctga ttattagttt aaaaatcttg 2100
taacactcag cagtgctatt ttgagtcatc ccagtttcct gaaagtaatg cccagtcttc 2160
ctgaatcctc cttaatagca gaaccttggt gattttgttg gctcatatga atgcttgtca 2220
tggatatgtt aacaatttag tgtttgacat tgcttcctct gccacaaaga caatactctg 2280
gtgacacatg tctaggccca gcacaggctg taggcccagg agtgactcaa aggagttttt 2340
ccctctttct tacggttcaa aggtgaccct ggtggtggcc agagcagtaa tgcttgtttg 2400
atgctcttca tggctcatct gcttctcaga acccacccgt tgagtttgtg ggtaaccagc 2460
aggcaggcca aagactggtg cttttcattt catcctttag agggatgaaa cagttatttc 2520
cgtctgatga gcattcggta gaatttttga agtgagattt tatgaagtca aaggggactt 2580
tacacagatc tcgacctgct ttgaaaccta gaggtggccc tttgatttgt gcgtgtcctt 2640
gccctctgga caacttaata tttcaagtaa tcgaatacca acttccctgc cagcccacct 2700
gccttccgcc ccgcttgtgt aacagtcctg ttttgttgag ttgctgctat tgcactgcca 2760
gtgcagccca caccaaatca caacccaaga tactcagata ggaagactcc ttcctctccc 2820
agtactttac caaaggaacc cccgccagga cccacatggg gccacgtgtt ggcagtggaa 2880
tcagcctgtg caggctgggg atctcaggct gatcagtagg ggccagcttt ggagccagcc 2940
aagctgaatc ccacactcca ggtctgtgct caagagacca gatggtgtat ttccaaatgg 3000
gcctctctgg tatgggcaat aggcaagctc ctggggtctg gttatgtgga agattcttag 3060
tggatgttcc gcctggttag ctggttctct tcagagaata taaagtgaat gcctttaggg 3120
gtagctctga aagagaaacc caacaacttc attcctagcc atgaaagtag cacgatcata 3180
ttgtactgta ttgttattgt aaaatgacta tttgccatgt catgagtagg tagatgtttt 3240
gccacaaata tgaatgtgtt tgttgtttcc tgactttaag caatgaagat tgagacaata 3300
aatagcactc agagaatgaa gcattgatgt t 3331

<210> 126
<211> 545
<212> PRT
<213> Homo sapiens

<400> 126
Met Ala Leu Arg Arg Gly Gly Cys Gly Ala Leu Gly Leu Leu Leu Leu
1               5                   10                  15

Leu Leu Gly Ala Ala Cys Leu Ile Pro Arg Ser Ala Gln Val Arg Arg
            20                  25                  30

Leu Ala Arg Cys Pro Ala Thr Cys Ser Cys Thr Lys Glu Ser Ile Ile
        35                  40                  45

Cys Val Gly Ser Ser Trp Val Pro Arg Ile Val Pro Gly Asp Ile Ser
    50                  55                  60

Ser Leu Ser Leu Val Asn Gly Thr Phe Ser Glu Ile Lys Asp Arg Met
65                  70                  75                  80

Phe Ser His Leu Pro Ser Leu Gln Leu Leu Leu Leu Asn Ser Asn Ser
                85                  90                  95

Phe Thr Ile Ile Arg Asp Asp Ala Phe Ala Gly Leu Phe His Leu Glu
            100                 105                 110

Tyr Leu Phe Ile Glu Gly Asn Lys Ile Glu Thr Ile Ser Arg Asn Ala
        115                 120                 125

Phe Arg Gly Leu Arg Asp Leu Thr His Leu Ser Leu Ala Asn Asn His
    130                 135                 140

Ile Lys Ala Leu Pro Arg Asp Val Phe Ser Asp Leu Asp Ser Leu Ile

145                 150                 155                 160

Glu Gln Ile Leu Arg Gly Asn Lys Phe Glu Cys Asp Cys Lys Ala Lys
                165                 170                 175

Trp Leu Tyr Leu Trp Leu Lys Met Thr Asn Ser Thr Val Ser Asp Val
            180                 185                 190

Leu Cys Ile Gly Pro Pro Glu Tyr Gln Glu Lys Lys Leu Asn Asp Val
        195                 200                 205

Thr Ser Phe Asp Tyr Glu Cys Thr Thr Thr Asp Phe Val Val His Gln
    210                 215                 220

Thr Leu Pro Tyr Gln Ser Val Ser Val Asp Thr Phe Asn Ser Lys Asn
225                 230                 235                 240

Asp Val Tyr Val Ala Ile Ala Gln Pro Ser Met Glu Asn Cys Met Val
                245                 250                 255

Leu Glu Trp Asp His Ile Glu Met Asn Phe Arg Ser Tyr Asp Asn Ile
            260                 265                 270

Thr Gly Gln Ser Ile Val Gly Cys Lys Ala Ile Leu Ile Asp Asp Gln
        275                 280                 285

Val Phe Val Val Val Ala Gln Leu Phe Gly Gly Ser His Ile Tyr Lys
    290                 295                 300

Tyr Asp Glu Ser Trp Thr Lys Phe Val Lys Phe Gln Asp Ile Glu Val
305                 310                 315                 320

Ser Arg Ile Ser Lys Pro Asn Asp Ile Glu Leu Phe Gln Ile Asp Asp
                325                 330                 335

Glu Thr Phe Phe Val Ile Ala Asp Ser Ser Lys Ala Gly Leu Ser Thr
            340                 345                 350

Val Tyr Lys Trp Asn Ser Lys Gly Phe Tyr Ser Tyr Gln Ser Leu His
        355                 360                 365

Glu Trp Phe Arg Asp Thr Asp Ala Glu Phe Val Asp Ile Asp Gly Lys
    370                 375                 380

Ser His Leu Ile Leu Ser Ser Arg Ser Gln Val Pro Ile Ile Leu Gln
385                 390                 395                 400

Trp Asn Lys Ser Ser Lys Lys Phe Val Pro His Gly Asp Ile Pro Asn
                405                 410                 415

Met Glu Asp Val Leu Ala Val Lys Ser Phe Arg Met Gln Asn Thr Leu
            420                 425                 430

Tyr Leu Ser Leu Thr Arg Phe Ile Gly Asp Ser Arg Val Met Arg Trp
        435                 440                 445

Asn Ser Lys Gln Phe Val Glu Ile Gln Ala Leu Pro Ser Arg Gly Ala
    450                 455                 460

Met Thr Leu Gln Pro Phe Ser Phe Lys Asp Asn His Tyr Leu Ala Leu
465                 470                 475                 480

Gly Ser Asp Tyr Thr Phe Ser Gln Ile Tyr Gln Trp Asp Lys Glu Lys
                485                 490                 495

Gln Leu Phe Lys Lys Phe Lys Glu Ile Tyr Val Gln Ala Pro Arg Ser
            500                 505                 510

Phe Thr Ala Val Ser Thr Asp Arg Arg Asp Phe Phe Phe Ala Ser Ser

515                 520                 525

Phe Lys Gly Lys Thr Lys Ile Phe Glu His Ile Ile Val Asp Leu Ser
    530                 535                 540

Leu
545


<210> 127
<211> 2886
<212> DNA
<213> Homo sapiens

<400> 127
attctgtgca agcctcatgg aaatgaagct gccaggccag gaagggtttg aagcctccag 60
tgctcctaga aatattcctt caggggagct ggacagcaac cctgaccctg gcaccggccc 120
cagccctgat ggcccctcag acacagagag caaggaactg ggagtaccca aagaccctct 180
gctcttcatt cagctgaatg agctgctggg ctggccccag gcgctggagt ggagagagac 240
aggccgatgg gtactgtttg aggagaagtt ggaggtggct gcaggccggt ggagtgcccc 300
ccacgtgccc accctggcac tgcccagcct ccagaagctc cgcagcctgc tggccgaggg 360
ccttgtactg ctggactgcc cagctcagag cctcctggag ctcgtggagc aggtgaccag 420
ggtggagtcg ctgagcccag agctgagagg gcagttgcag gccttgctgc tgcagagacc 480
ccagcattac aaccagacca caggcaccag gccctgctgg ggtgagagcc cctccagaaa 540
ggcttctgac aatgaggaag ccccccctgag ggaccagtgt cagaaccccc tgagacagaa 600
gctacctcca ggagctgagg cagggactgt gctggcaggg gagctgggct tcctggcaca 660
gccactggga gcctttgttc gactgcggaa ccctgtggta ctggggtccc ttactgaggt 720
gtccctccca agcaggtttt tctgccttct cctgggcccc tgtatgctgg gaaagggcta 780
ccatgagatg ggacgggcag cagctgtcct cctcagtgac ccgcattccc agcaattcca 840
gtggtcagtt cgtcgggcca gcaaccttca tgaccttctg gcagccctgg atgcattcct 900
agaggaggtg acagtgcttc ccccaggtcg gtgggaccca acagcccgga ttcccccgcc 960
caaatgtctg ccatctcagc acaaaaggac ctcggctgag gacaggcacc gccatgggcc 1020
acacgcacac agcccggagt tgcagcggac cggcaggctg tttgggggcc ttatccagga 1080
cgtgcgcagg aaggtcccgt ggtaccccag cgatttcttg gacgccctgc atctccagtg 1140
cttctcggcc gtactctaca tttacctggc cactgtcact aatgccatca cttttggggg 1200
tctgctggga gatgccactg atggtgccca gggagtgctg gaaagtttcc tgggcacagc 1260
agtggctgga gctgccttct gcctgatggc aggccagccc ctcaccattc tgagcagcac 1320
ggggccagtg ctggtctttg agcgcctgct cttctctttc agcagagatt acagcctgga 1380
ctacctgccc ttccgcctat gggtgggcat ctgggtggct accttttgcc tggtgctggt 1440
ggccacagag gccagtgtgc tggtgcgcta cttcacccgc ttcactgagg aaggtttctg 1500
tgccctcatc agcctcatct tcatctacga tgctgtgggc aaaatgctga acttgaccca 1560
tacctatcct atccagaagc ctgggtcctc tgcctacggg tgcctctgcc aatacccagg 1620
cccaggaggt aatgagtctc aatggataag gacaaggcca aaagacagag acgacattgt 1680
aagcatggac ttaggcctga tcaatgcatc cttgctgccg ccacctgagt gcacccggca 1740
gggaggccac cctcgtggcc ctggctgtca tacagtccca gacattgcct tcttctccct 1800
tctcctcttc cttacttctt tcttctttgc tatggccctc aagtgtgtaa agaccagccg 1860
cttcttcccc tctgtggtgc gcaaagggct cagcgacttc tcctcagtcc tggccatcct 1920
gctcggctgt ggccttgatg ctttcctggg cctagccaca ccaaagctca tggtacccag 1980
agagttcaag cccacactcc ctgggcgtgg ctggctggtg tcaccttttg gagccaaccc 2040
ctggtggtgg agtgtggcag ctgccctgcc tgccctgctg ctgtctatcc tcatcttcat 2100
ggaccaacag atcacagcag tcatcctcaa ccgcatggaa tacagactgc agaagggagc 2160
tggcttccac ctggacctct tctgtgtggc tgtgctgatg ctactcacat cagcgcttgg 2220
actgccttgg tatgtctcag ccactgtcat ctccctggct cacatggaca gtcttcggag 2280
agagagcaga gcctgtgccc ccggggagcg ccccaacttc ctgggtatca gggaacagag 2340
gctgacaggc ctggtggtgt tcatccttac aggagcctcc atcttcctgg cacctgtgct 2400
caagttcatt ccaatgcctg tgctctatgg catcttcctg tatatggggg tggcagcgct 2460
cagcagcatt cagttcacta atagggtgaa gctgttgttg atgccagcaa aacaccagcc 2520
agacctgcta ctcttgcggc atgtgcctct gaccagggtc cacctcttca cagccatcca 2580
gcttgcctgt ctggggctgc tttggataat caagtctacc cctgcagcca tcatcttccc 2640
cctcatgttg ctgggccttg tgggggtccg aaaggccctg gagagggtct tctcaccaca 2700
ggaactcctc tggctggatg agctgatgcc agaggaggag agaagcatcc ctgagaaggg 2760
gctggagcca gaacactcat tcagtggaag tgacagtgaa gattcagagc tgatgtatca 2820
gccaaaggct ccagaaatca acatttctgt gaattagctg gagtaggagt ctgggagtgg 2880
agaccc                                                            2886


<210> 128
<211> 946
<212> PRT

<213> Homo sapiens

<400> 128
Met Glu Met Lys Leu Pro Gly Gln Glu Gly Phe Glu Ala Ser Ser Ala
1               5                   10                  15

Pro Arg Asn Ile Pro Ser Gly Glu Leu Asp Ser Asn Pro Asp Pro Gly
            20                  25                  30

Thr Gly Pro Ser Pro Asp Gly Pro Ser Asp Thr Glu Ser Lys Glu Leu
        35                  40                  45

Gly Val Pro Lys Asp Pro Leu Leu Phe Ile Gln Leu Asn Glu Leu Leu
    50                  55                  60

Gly Trp Pro Gln Ala Leu Glu Trp Arg Glu Thr Gly Arg Trp Val Leu
65                  70                  75                  80

Phe Glu Glu Lys Leu Glu Val Ala Ala Gly Arg Trp Ser Ala Pro His
                85                  90                  95

Val Pro Thr Leu Ala Leu Pro Ser Leu Gln Lys Leu Arg Ser Leu Leu
            100                 105                 110

Ala Glu Gly Leu Val Leu Leu Asp Cys Pro Ala Gln Ser Leu Leu Glu
        115                 120                 125

Leu Val Glu Gln Val Thr Arg Val Glu Ser Leu Ser Pro Glu Leu Arg
    130                 135                 140

Gly Gln Leu Gln Ala Leu Leu Leu Gln Arg Pro Gln His Tyr Asn Gln
145                 150                 155                 160

Thr Thr Gly Thr Arg Pro Cys Trp Gly Glu Ser Pro Ser Arg Lys Ala
                165                 170                 175

Ser Asp Asn Glu Glu Ala Pro Leu Arg Asp Gln Cys Gln Asn Pro Leu
            180                 185                 190

Arg Gln Lys Leu Pro Pro Gly Ala Glu Ala Gly Thr Val Leu Ala Gly
        195                 200                 205

Glu Leu Gly Phe Leu Ala Gln Pro Leu Gly Ala Phe Val Arg Leu Arg
    210                 215                 220

Asn Pro Val Val Leu Gly Ser Leu Thr Glu Val Ser Leu Pro Ser Arg
225                 230                 235                 240

Phe Phe Cys Leu Leu Leu Gly Pro Cys Met Leu Gly Lys Gly Tyr His
                245                 250                 255

Glu Met Gly Arg Ala Ala Ala Val Leu Leu Ser Asp Pro His Ser Gln
            260                 265                 270

Gln Phe Gln Trp Ser Val Arg Arg Ala Ser Asn Leu His Asp Leu Leu
        275                 280                 285

Ala Ala Leu Asp Ala Phe Leu Glu Glu Val Thr Val Leu Pro Pro Gly
    290                 295                 300

Arg Trp Asp Pro Thr Ala Arg Ile Pro Pro Pro Lys Cys Leu Pro Ser
305                 310                 315                 320

Gln His Lys Arg Thr Ser Ala Glu Asp Arg His Arg His Gly Pro His
                325                 330                 335

Ala His Ser Pro Glu Leu Gln Arg Thr Gly Arg Leu Phe Gly Gly Leu
            340                 345                 350

Ile Gln Asp Val Arg Arg Lys Val Pro Trp Tyr Pro Ser Asp Phe Leu
        355                 360                 365

Asp Ala Leu His Leu Gln Cys Phe Ser Ala Val Leu Tyr Ile Tyr Leu
    370                 375                 380

Ala Thr Val Thr Asn Ala Ile Thr Phe Gly Gly Leu Leu Gly Asp Ala
385                 390                 395                 400

Thr Asp Gly Ala Gln Gly Val Leu Glu Ser Phe Leu Gly Thr Ala Val
                405                 410                 415

Ala Gly Ala Ala Phe Cys Leu Met Ala Gly Gln Pro Leu Thr Ile Leu
            420                 425                 430

Ser Ser Thr Gly Pro Val Leu Val Phe Glu Arg Leu Leu Phe Ser Phe
        435                 440                 445

Ser Arg Asp Tyr Ser Leu Asp Tyr Leu Pro Phe Arg Leu Trp Val Gly
    450                 455                 460

Ile Trp Val Ala Thr Phe Cys Leu Val Leu Val Ala Thr Glu Ala Ser
465                 470                 475                 480

Val Leu Val Arg Tyr Phe Thr Arg Phe Thr Glu Glu Gly Phe Cys Ala
                485                 490                 495

Leu Ile Ser Leu Ile Phe Ile Tyr Asp Ala Val Gly Lys Met Leu Asn
            500                 505                 510

Leu Thr His Thr Tyr Pro Ile Gln Lys Pro Gly Ser Ser Ala Tyr Gly
        515                 520                 525

Cys Leu Cys Gln Tyr Pro Gly Pro Gly Gly Asn Glu Ser Gln Trp Ile
    530                 535                 540

Arg Thr Arg Pro Lys Asp Arg Asp Asp Ile Val Ser Met Asp Leu Gly
545                 550                 555                 560

Leu Ile Asn Ala Ser Leu Leu Pro Pro Pro Glu Cys Thr Arg Gln Gly
                565                 570                 575

Gly His Pro Arg Gly Pro Gly Cys His Thr Val Pro Asp Ile Ala Phe
            580                 585                 590

Phe Ser Leu Leu Leu Phe Leu Thr Ser Phe Phe Phe Ala Met Ala Leu
        595                 600                 605

Lys Cys Val Lys Thr Ser Arg Phe Phe Pro Ser Val Val Arg Lys Gly
    610                 615                 620

Leu Ser Asp Phe Ser Ser Val Leu Ala Ile Leu Leu Gly Cys Gly Leu
625                 630                 635                 640

Asp Ala Phe Leu Gly Leu Ala Thr Pro Lys Leu Met Val Pro Arg Glu
                645                 650                 655

Phe Lys Pro Thr Leu Pro Gly Arg Gly Trp Leu Val Ser Pro Phe Gly
            660                 665                 670

Ala Asn Pro Trp Trp Trp Ser Val Ala Ala Ala Leu Pro Ala Leu Leu
        675                 680                 685

Leu Ser Ile Leu Ile Phe Met Asp Gln Gln Ile Thr Ala Val Ile Leu
    690                 695                 700

Asn Arg Met Glu Tyr Arg Leu Gln Lys Gly Ala Gly Phe His Leu Asp
705                 710                 715                 720

Leu Phe Cys Val Ala Val Leu Met Leu Leu Thr Ser Ala Leu Gly Leu
                725                 730                 735

Pro Trp Tyr Val Ser Ala Thr Val Ile Ser Leu Ala His Met Asp Ser
            740                 745                 750

Leu Arg Arg Glu Ser Arg Ala Cys Ala Pro Gly Glu Arg Pro Asn Phe
        755                 760                 765

Leu Gly Ile Arg Glu Gln Arg Leu Thr Gly Leu Val Val Phe Ile Leu
    770                 775                 780

Thr Gly Ala Ser Ile Phe Leu Ala Pro Val Leu Lys Phe Ile Pro Met
785                 790                 795                 800

Pro Val Leu Tyr Gly Ile Phe Leu Tyr Met Gly Val Ala Ala Leu Ser
                805                 810                 815

Ser Ile Gln Phe Thr Asn Arg Val Lys Leu Leu Leu Met Pro Ala Lys
            820                 825                 830

His Gln Pro Asp Leu Leu Leu Leu Arg His Val Pro Leu Thr Arg Val
        835                 840                 845

His Leu Phe Thr Ala Ile Gln Leu Ala Cys Leu Gly Leu Leu Trp Ile
    850                 855                 860

Ile Lys Ser Thr Pro Ala Ala Ile Ile Phe Pro Leu Met Leu Leu Gly
865                 870                 875                 880

Leu Val Gly Val Arg Lys Ala Leu Glu Arg Val Phe Ser Pro Gln Glu
                885                 890                 895

Leu Leu Trp Leu Asp Glu Leu Met Pro Glu Glu Glu Arg Ser Ile Pro
            900                 905                 910

Glu Lys Gly Leu Glu Pro Glu His Ser Phe Ser Gly Ser Asp Ser Glu
        915                 920                 925

Asp Ser Glu Leu Met Tyr Gln Pro Lys Ala Pro Glu Ile Asn Ile Ser
    930                 935                 940

Val Asn
945


<210> 129
<211> 1083
<212> DNA
<213> Homo sapiens

<400> 129
ggcggcgagg gtgcggggcg cgctgccatg ggccttggcg gcgccgccgc ggcgctggtg 60
gcggcggcag cagcagcagc agcggcagcg gcagcggtgg tggccgggcc gcggcggcgg 120
cggcgagggt gcggggcgcg ctgccatggg cctggccggg ctgcaggcaa gaagatgtcc 180
aagccccgcg cgctggaggc ggcggcggcg gcggcagcga cggccccggg cctggagatg 240
gtggagcgga ggggcccggg gaggccccgc accgatgggg agagcgtatt taccgggcag 300
tcaaagatct attcctacat gagcccgaac aaatgctctg gaatgcgttt cccccttcaa 360
gaagagaact cggttacaca tcacgaagtc aaatgccagg ggaaaccatt agccggaatc 420
tacaggaaac gagaagagaa aagaaatact gggaacgcag tacagagcgc catgaagtcc 480
aagaaacaga agatcaaaga cgccaggaga ggtcccctgc aaggaaaaac acaacagaat 540
cacaaactta cggatttcta ccctgtccga aggagatcca ggaagagcaa agccgagctg 600
cagtctgaag aaaggaaaag aatagatgaa ttgattgaaa gtgggaagga agaaggaatg 660
aagattgacc tcatcgatgg caaaggcagg ggtgtgattg ccaccaagca tttctcccgg 720
ggtgcctttg tggtggaata ccacggggac ctcatcgaga tcaccgacgc caagaaacgg 780
gaggctctgt atgcacagga cccttccacg ggctgctaca tgtactattt tcagtatctg 840
agcaaaacct actgcgtgga tgcaactaga gagacaaatc gcccaggaag accgatcaat 900
cacagcaaat gtgggaactg ccaaaccaaa ctgcacgaca tcgacggcgt acctcacctc 960
atcctcatcg cctcccaaga catcgcggct ggggaggagc tcctgtatga ctatggggac 1020

cgcagcaagg cttccattga agcccacccg tggctgaagc attaaccggt gggccccgcg 1080
ccc 1083

<210> 130
<211> 345
<212> PRT
<213> Homo sapiens

<400> 130
Met Gly Leu Gly Gly Ala Ala Ala Ala Leu Val Ala Ala Ala Ala Ala
1               5                   10                  15

Ala Ala Ala Ala Ala Ala Ala Val Val Ala Gly Pro Arg Arg Arg Arg
            20                  25                  30

Arg Gly Cys Gly Ala Arg Cys His Gly Pro Gly Arg Ala Ala Gly Lys
        35                  40                  45

Lys Met Ser Lys Pro Arg Ala Leu Glu Ala Ala Ala Ala Ala Ala Ala
    50                  55                  60

Thr Ala Pro Gly Leu Glu Met Val Glu Arg Arg Gly Pro Gly Arg Pro
65                  70                  75                  80

Arg Thr Asp Gly Glu Ser Val Phe Thr Gly Gln Ser Lys Ile Tyr Ser
                85                  90                  95

Tyr Met Ser Pro Asn Lys Cys Ser Gly Met Arg Phe Pro Leu Gln Glu
            100                 105                 110

Glu Asn Ser Val Thr His His Glu Val Lys Cys Gln Gly Lys Pro Leu
        115                 120                 125

Ala Gly Ile Tyr Arg Lys Arg Glu Glu Lys Arg Asn Thr Gly Asn Ala
    130                 135                 140

Val Gln Ser Ala Met Lys Ser Lys Lys Gln Lys Ile Lys Asp Ala Arg
145                 150                 155                 160

Arg Gly Pro Leu Gln Gly Lys Thr Gln Gln Asn His Lys Leu Thr Asp
                165                 170                 175

Phe Tyr Pro Val Arg Arg Arg Ser Arg Lys Ser Lys Ala Glu Leu Gln
            180                 185                 190

Ser Glu Glu Arg Lys Arg Ile Asp Glu Leu Ile Glu Ser Gly Lys Glu
        195                 200                 205

Glu Gly Met Lys Ile Asp Leu Ile Asp Gly Lys Gly Arg Gly Val Ile
    210                 215                 220

Ala Thr Lys His Phe Ser Arg Gly Ala Phe Val Val Glu Tyr His Gly
225                 230                 235                 240

Asp Leu Ile Glu Ile Thr Asp Ala Lys Lys Arg Glu Ala Leu Tyr Ala
                245                 250                 255

Gln Asp Pro Ser Thr Gly Cys Tyr Met Tyr Tyr Phe Gln Tyr Leu Ser
            260                 265                 270

Lys Thr Tyr Cys Val Asp Ala Thr Arg Glu Thr Asn Arg Pro Gly Arg
        275                 280                 285

Pro Ile Asn His Ser Lys Cys Gly Asn Cys Gln Thr Lys Leu His Asp
    290                 295                 300

Ile Asp Gly Val Pro His Leu Ile Leu Ile Ala Ser Gln Asp Ile Ala
305                 310                 315                 320

Ala Gly Glu Glu Leu Leu Tyr Asp Tyr Gly Asp Arg Ser Lys Ala Ser
                325                 330                 335

Ile Glu Ala His Pro Trp Leu Lys His
            340                 345


<210> 131
<211> 5896
<212> DNA
<213> Homo sapiens

<220>
<221> misc_feature
<222> (5662)
<223> Where N is G or A or T or C

<400> 131
ggcggtgctg cgggacgaag gcgaggagga ggcggaggtg gagctggcgg agagcgggag 60
gcggctgcga ctgccgcggg accagatcca gcgcatgaac ccgcgccaag ttcagcaagg 120
ccgaggacat ggccgagctg acctgcctca acgaggcctc ggtcctgcac aacctccggg 180
agcggtacta ctccggcctc atctacacgt actccggcct tttctgtgtg gtcatcaacc 240
cgtacaagca gcttcccatc tacacagaag ccattgtgga gatgtaccgg ggcaagaagc 300
gccacgaggt gccacgccac gtgtacgcag tgaccgaggg ggcctatcgg agcatgctgc 360
aggatcgtga ggaccagtcc attctctgca cgggagagtc tggagctggg aagacggaaa 420
acaccaagaa ggtcatccag tacctcgccc acgtggcgtc gtctccaaag ggcaggaagg 480
agccgggtgt ccccggtgag ctggagcggc agctgcttca ggccaacccc atcctagagg 540
cctttggcaa tgccaagaca gtgaagaatg acaactcctc ccgattcggc aaattcatcc 600
gcatcaactt tgatgttgcc gggtacatcg tgggcgccaa cattgagacc tgtctgctgg 660
agaagtcgcg ggccatccgc caggccaagg acgagtgcag cttccacatc ttctaccagc 720
tgctgggggg cgctggagag catggctgcc gagaactcct cctcgagccc tgctcccact 780
accggttcct gaccaacggg ccgtcatcct ctcccggcca ggagcgggaa ctcttccagg 840
agacgctgga gtcgctgcgg gtcctgggat tcagccacga ggaaatcatc tccatgctgc 900
ggatggtctc agcagttctc cagtttggca acattgcctt gaagagagaa cggaacaccg 960
atcaagccac catgcctgac aacacagctg cacagaagct ctgccgcctc ttgggactgg 1020
gggtgacgga tttctcccga gccttgctca cccctcgcat caaagttggc cgagactatg 1080
tgcagaaagc ccagactaag gaacaggctg acttcgcgct ggaggccctg gccaaggcca 1140
cctacgagcg cctcttccgc tggctggttc tgcgcctcaa ccgggccttg gaccgcagcc 1200
cccgccaagg cgcctccttc ctgggcatcc tggacatcgc gggctttgag atcttccagc 1260
tgaactcctt cgagcagctc tgcatcaact acaccaacga gaagctgcag cagctcttca 1320
accacaccat gttcgtgctg gagcaggagg agtaccagcg tgagggcatc ccctggacct 1380
tcctcgactt tggcctcgac ctgcagccct gcatcgacct catcgagcgg ccggccaacc 1440
cccctggact cctggccctg ctggatgagg agtgctggtt cccgaaggcc acagacaagt 1500
cgtttgtgga gaaggtagcc caggagcagg gcggccaccc caagttccag cggccgaggc 1560
acctgcggga tcaggccgac ttcagtgttc tccactacgc gggcaaggtc gactacaagg 1620
ccaacgagtg gctgatgaaa aacatggacc ctctgaatga caacgtcgca gccttgctcc 1680
accagagcac agaccggctg acggcagaga tctggaaaga cgtggagggc atcgtggggc 1740
tggaacaggt gagcagcctg ggcgacggcc caccaggtgg ccgcccccgt cggggtatgt 1800
tccggacagt gggacagctc tacaaggagt ccctgagccg cctcatggcc acactcagca 1860
acaccaaccc cagttttgtc cgctgcattg tccccaacca cgagaagagg gtcgggaagc 1920
tggagccgcg gctggtgctg gaccagcttc gctgcaacgg ggtcctggag ggcatccgca 1980
tctgtcgcca gggcttcccc aaccgcatcc tcttccagga gttccggcag cgatacgaga 2040
tcctgacacc caatgccatc cccaagggct tcatggatgg gaagcaggcc tgtgaaaaga 2100
tgatccaggc gctggaactg gaccccaacc tctaccgcgt gggacagagc aagatcttct 2160
tccgggctgg ggtcctggcc cagctggaag aggagcgaga cctgaaggtc accgacatca 2220
tcgtctcctt ccaggcagct gcccggggat acctggctcg cagggccttc cagaagcgcc 2280
agcagcagca gagcgccctg agggtgatgc agcggaactg cgcggcctac ctcaagctga 2340
gacactggca gtggtggcgg ctgtttacca aggtgaagcc actgctgcag gtgacgcggc 2400
aggatgaggt gctgcaggca cgggcccagg agctgcagaa agtgcaggag ctacagcagc 2460
agagcgcccg cgaagttggg gagctccagg gccgagtgac acagctggaa gaggagcgcg 2520
cccgcctggc agagcaattg cgagcagagg cagaactgtg tgcagaggcc gaggagacgc 2580
gggggaggct ggcagcccgc aagcaggagc tggagctggt ggtgtcagag ctggaggctc 2640
gcgtgggcga ggaggaggag tgcagccgtc aaatgcaaac cgagaagaag aggctacagc 2700
agcacataca ggagctagag gcccaccttg aggctgagga gggtgcgcgg cagaagctgc 2760
agctggagaa ggtgacgaca gaggcaaaaa tgaagaaatt tgaagaggac ctgctgctcc 2820
tggaagacca gaattccaag ctgagcaagg aactgctgga agatcgtctg gccgagttct 2880
catcccaggc agctgaggag gaggagaagg tcaagagcct caataagcta cggctcaaat 2940
atgaggccac aatcgcagac atggaggacc gcctacggaa ggaggagaag ggtcgccagg 3000

agctggagaa gctgaagcgg aggctggatg gggagagctc agagctgcag gagcagatgg 3060
tggagcagca acagcgggca gaggagctgc gggcccagct gggccggaag gaggaggagc 3120
tgcaggctgc cctggccagg gcagaagacg agggtggggc ccgggcccag ctgctgaaat 3180
ccctgcggga ggctcaagca gccctggccg aggccgaggc ccaggaggac ctggagtctg 3240
agcgtgtggc caggaccaag gcggagaagc agcgccggga cctgggcgag gagctggagg 3300
cgctgcgggg cgagctggag gacacgctgg actccaccaa cgcacagcag gagctcaggt 3360
ccaagaggga acaggaggtg acggagctga agaagactct ggaggaggag actcgcatcc 3420
acgaggcggc agtgcaggag ctgaggcagc gccacggcca ggccctgggg gagctggcgg 3480
agcagctgga gcaggcccgg aggaaaggtg catgggagaa gacccggctg gccctggagg 3540
ccgaggtgtc cgagctgcgg gcagaactga gcagcctgca gactgcacgt caggagggtg 3600
agcagcggag gcgccgcctg gagttacagc tgcaggaggt gcagggccgg gctggtgatg 3660
gggagagggc acgagcggag gctgctgaga aggtcccttc cctgcaggct gaactggaga 3720
atgtgtctgg ggcgctgaac gaggctgagt ccaaaaccat ccgtcttagc aaggagctga 3780
gcagcacaga agcccagctg cacgatgccc aggagctgct gcaggaggag accagggcga 3840
aattggcctt ggggtcccgg gtgcgagcca tggaggctga ggcagccggg ctgcgtgagc 3900
agctggagga ggaggcagct gccagggaac gggcggacca ccaaccaccc tctctctcct 3960
cccctcagct ttccgagtgg cggcggcgcc aggaggagga ggcaggggca ctggaggcag 4020
gggaggaggc acggcgccgg gcagcccggg aggccgaggc cctgacccag cgcctggcag 4080
aaaagacaga gaccgtggat cggctggagc ggggccgccg ccggctgggg caggagctgg 4140
acgacgccac catggacctg gagcagcagc ggcagcttgt gagcaccctg gagaagaagc 4200
agcgcaagtt tgaccagctt ctggcagagg agaaggcagc tgtacttcgg gcagtggagg 4260
aacgtgagcg ggccgaggca gagggccggg agcgtgaggc tcgggccctg tcactgacac 4320
gggcactgga ggaggagcag gaggcacgtg aggagctgga gcggcagaac cgggccctgc 4380
gggctgagct ggaggcactg ctgagcagca aggatgacgt cggcaagagc gtgcatgagc 4440
tggaacgagc ctgccgggta gcagaacagg cagccaatga tctgcgagca caggtgacag 4500
aactggagga tgagctgaca gcggccgagg atgccaagct gcgtctggag gtgactgtgc 4560
aggctctcaa gactcagcat gagcgtgacc tgcagggccg tgatgaggct ggtgaagaga 4620
ggcggaggca gctggccaag cagctgagag atgcagaggt ggagcgggat gaggagcgga 4680
agcagcgcac tctggccgtg gctgcccgca agaagctgga gggagagctg gaggagctga 4740
aggctcagat ggcctctgcc ggccagggca aggaggaggc ggtgaagcag cttcgcaaga 4800
tgcaggccca gatgaaggag ctatggcggg aggtggagga gacacgcacc tcccgggagg 4860
agatcttctc ccagaatcgg gaaagtgaaa agcgcctcaa gggcctggag gctgaggtgc 4920
tgcggctgca ggaggaactg gccgcctcgg accgtgctcg gcggcaggcc cagcaggacc 4980
gggatgagat ggcagatgag gtggccaatg gtaaccttag caaggcagcc attctggagg 5040
agaagcgtca gctggagggg cgcctggggc agttggagga agagctggag gaggagcaga 5100
caactcagag ctgctcaatg accgctaccg caagctgctc ctgcaggtag agtcactgac 5160
cacagagctg tcagctgagc gcagtttctc agccaaggca gagagcgggc ggcagcagct 5220
ggaacggcag atccaggagc tacggggacg cctgggtgag gaggatgctg gggcccgtgc 5280
ccgccacaag atgaccattg ctgcccttga gtctaagttg gcccaggctg aggagcagct 5340
agagcaagag accagagagc gcatcctctc tggaaagctg gtgcccaaaa gttaagaagc 5400
ggcttaaaga ggtggtgctc caggtggagg aggagcggag ggtggctgac cagctccggg 5460
accagctgga gaagggaaac cttcgagtca agcagctgaa gcggcagctg gaggaggccg 5520
aggaggaggc atcccgggct caggccggcc gccggaggct gcagcgtgag ctggaagatg 5580
tcacagagtc ggccgagtcc atgaaccgtg aagtgaccac actgaggaac cggcttcgac 5640
gcggccccct caccttcacc anccgcacgg tgcgccaggt cttccgacta gaggagggcg 5700
tggcatccga cgaggaggca gaggaagcac agcctgggtc tgggccatcc cctctcactc 5760
ctgctgctgc ccatgctctg ccctcccttc tggttgctct gagggttcgg agcttccctc 5820
tgggactaaa ggagtgtcct ttaccctccc agcctcccgg ctctggcaga aataaactcc 5880
aacccgaatg gaaaaa 5896

<210> 132
<211> 1673
<212> PRT
<213> Homo sapiens

<400> 132
Met Ala Glu Leu Thr Cys Leu Asn Glu Ala Ser Val Leu His Asn Leu
1               5                   10                  15

Arg Glu Arg Tyr Tyr Ser Gly Leu Ile Tyr Thr Tyr Ser Gly Leu Phe
            20                  25                  30

Cys Val Val Ile Asn Pro Tyr Lys Gln Leu Pro Ile Tyr Thr Glu Ala
        35                  40                  45

Ile Val Glu Met Tyr Arg Gly Lys Lys Arg His Glu Val Pro Pro His
    50                  55                  60

Val Tyr Ala Val Thr Glu Gly Ala Tyr Arg Ser Met Leu Gln Asp Arg
65                  70                  75                  80

Glu Asp Gln Ser Ile Leu Cys Thr Gly Glu Ser Gly Ala Gly Lys Thr
                85                  90                  95

Glu Asn Thr Lys Lys Val Ile Gln Tyr Leu Ala His Val Ala Ser Ser
            100                 105                 110

Pro Lys Gly Arg Lys Glu Pro Gly Val Pro Gly Glu Leu Glu Arg Gln
        115                 120                 125

Leu Leu Gln Ala Asn Pro Ile Leu Glu Ala Phe Gly Asn Ala Lys Thr
    130                 135                 140

Val Lys Asn Asp Asn Ser Ser Arg Phe Gly Lys Phe Ile Arg Ile Asn
145                 150                 155                 160

Phe Asp Val Ala Gly Tyr Ile Val Gly Ala Asn Ile Glu Thr Cys Leu
                165                 170                 175

Leu Glu Lys Ser Arg Ala Ile Arg Gln Ala Lys Asp Glu Cys Ser Phe
            180                 185                 190

His Ile Phe Tyr Gln Leu Leu Gly Gly Ala Gly Glu His Gly Cys Arg
        195                 200                 205

Glu Leu Leu Leu Glu Pro Cys Ser His Tyr Arg Phe Leu Thr Asn Gly
    210                 215                 220

Pro Ser Ser Ser Pro Gly Gln Glu Arg Glu Leu Phe Gln Glu Thr Leu
225                 230                 235                 240

Glu Ser Leu Arg Val Leu Gly Phe Ser His Glu Glu Ile Ile Ser Met
                245                 250                 255

Leu Arg Met Val Ser Ala Val Leu Gln Phe Gly Asn Ile Ala Leu Lys
            260                 265                 270

Arg Glu Arg Asn Thr Asp Gln Ala Thr Met Pro Asp Asn Thr Ala Ala
        275                 280                 285

Gln Lys Leu Cys Arg Leu Leu Gly Leu Gly Val Thr Asp Phe Ser Arg
    290                 295                 300

Ala Leu Leu Thr Pro Arg Ile Lys Val Gly Arg Asp Tyr Val Gln Lys
305                 310                 315                 320

Ala Gln Thr Lys Glu Gln Ala Asp Phe Ala Leu Glu Ala Leu Ala Lys
                325                 330                 335

Ala Thr Tyr Glu Arg Leu Phe Arg Trp Leu Val Leu Arg Leu Asn Arg
            340                 345                 350

Ala Leu Asp Arg Ser Pro Arg Gln Gly Ala Ser Phe Leu Gly Ile Leu
        355                 360                 365

Asp Ile Ala Gly Phe Glu Ile Phe Gln Leu Asn Ser Phe Glu Gln Leu
    370                 375                 380

Cys Ile Asn Tyr Thr Asn Glu Lys Leu Gln Gln Leu Phe Asn His Thr
385                 390                 395                 400

Met Phe Val Leu Glu Gln Glu Glu Tyr Gln Arg Glu Gly Ile Pro Trp
                405                 410                 415

Thr Phe Leu Asp Phe Gly Leu Asp Leu Gln Pro Cys Ile Asp Leu Ile
            420                 425                 430

Glu Arg Pro Ala Asn Pro Pro Gly Leu Leu Ala Leu Leu Asp Glu Glu
        435                 440                 445

Cys Trp Phe Pro Lys Ala Thr Asp Lys Ser Phe Val Glu Lys Val Ala
    450                 455                 460

Gln Glu Gln Gly Gly His Pro Lys Phe Gln Arg Pro Arg His Leu Arg
465                 470                 475                 480

Asp Gln Ala Asp Phe Ser Val Leu His Tyr Ala Gly Lys Val Asp Tyr
                485                 490                 495

Lys Ala Asn Glu Trp Leu Met Lys Asn Met Asp Pro Leu Asn Asp Asn
            500                 505                 510

Val Ala Ala Leu Leu His Gln Ser Thr Asp Arg Leu Thr Ala Glu Ile
        515                 520                 525

Trp Lys Asp Val Glu Gly Ile Val Gly Leu Glu Gln Val Ser Ser Leu
    530                 535                 540

Gly Asp Gly Pro Pro Gly Gly Arg Pro Arg Arg Gly Met Phe Arg Thr
545                 550                 555                 560

Val Gly Gln Leu Tyr Lys Glu Ser Leu Ser Arg Leu Met Ala Thr Leu
                565                 570                 575

Ser Asn Thr Asn Pro Ser Phe Val Arg Cys Ile Val Pro Asn His Glu
            580                 585                 590

Lys Arg Val Gly Lys Leu Glu Pro Arg Leu Val Leu Asp Gln Leu Arg
        595                 600                 605

Cys Asn Gly Val Leu Glu Gly Ile Arg Ile Cys Arg Gln Gly Phe Pro
    610                 615                 620

Asn Arg Ile Leu Phe Gln Glu Phe Arg Gln Arg Tyr Glu Ile Leu Thr
625                 630                 635                 640

Pro Asn Ala Ile Pro Lys Gly Phe Met Asp Gly Lys Gln Ala Cys Glu
                645                 650                 655

Lys Met Ile Gln Ala Leu Glu Leu Asp Pro Asn Leu Tyr Arg Val Gly
            660                 665                 670

Gln Ser Lys Ile Phe Phe Arg Ala Gly Val Leu Ala Gln Leu Glu Glu
        675                 680                 685

Glu Arg Asp Leu Lys Val Thr Asp Ile Ile Val Ser Phe Gln Ala Ala
    690                 695                 700

Ala Arg Gly Tyr Leu Ala Arg Arg Ala Phe Gln Lys Arg Gln Gln Gln
705                 710                 715                 720

Gln Ser Ala Leu Arg Val Met Gln Arg Asn Cys Ala Ala Tyr Leu Lys
                725                 730                 735

Leu Arg His Trp Gln Trp Trp Arg Leu Phe Thr Lys Val Lys Pro Leu
            740                 745                 750

Leu Gln Val Thr Arg Gln Asp Glu Val Leu Gln Ala Arg Ala Gln Glu
        755                 760                 765

Leu Gln Lys Val Gln Glu Leu Gln Gln Gln Ser Ala Arg Glu Val Gly
    770                 775                 780

Glu Leu Gln Gly Arg Val Thr Gln Leu Glu Glu Glu Arg Ala Arg Leu
785                 790                 795                 800

Ala Glu Gln Leu Arg Ala Glu Ala Glu Leu Cys Ala Glu Ala Glu Glu
                805                 810                 815

Thr Arg Gly Arg Leu Ala Ala Arg Lys Gln Glu Leu Glu Leu Val Val
            820                 825                 830

Ser Glu Leu Glu Ala Arg Val Gly Glu Glu Glu Glu Cys Ser Arg Gln
        835                 840                 845

Met Gln Thr Glu Lys Lys Arg Leu Gln Gln His Ile Gln Glu Leu Glu
    850                 855                 860

Ala His Leu Glu Ala Glu Glu Gly Ala Arg Gln Lys Leu Gln Leu Glu
865                 870                 875                 880

Lys Val Thr Thr Glu Ala Lys Met Lys Lys Phe Glu Glu Asp Leu Leu
                885                 890                 895

Leu Leu Glu Asp Gln Asn Ser Lys Leu Ser Lys Glu Leu Leu Glu Asp
            900                 905                 910

Arg Leu Ala Glu Phe Ser Ser Gln Ala Ala Glu Glu Glu Glu Lys Val
        915                 920                 925

Lys Ser Leu Asn Lys Leu Arg Leu Lys Tyr Glu Ala Thr Ile Ala Asp
    930                 935                 940

Met Glu Asp Arg Leu Arg Lys Glu Glu Lys Gly Arg Gln Glu Leu Glu
945                 950                 955                 960

Lys Leu Lys Arg Arg Leu Asp Gly Glu Ser Ser Glu Leu Gln Glu Gln
                965                 970                 975

Met Val Glu Gln Gln Gln Arg Ala Glu Glu Leu Arg Ala Gln Leu Gly
            980                 985                 990

Arg Lys Glu Glu Glu Leu Gln Ala Ala Leu Ala Arg Ala Glu Asp Glu
        995                 1000                1005

Gly Gly Ala Arg Ala Gln Leu Leu Lys Ser Leu Arg Glu Ala Gln Ala
    1010                1015                1020

Ala Leu Ala Glu Ala Glu Ala Gln Glu Asp Leu Glu Ser Glu Arg Val
1025                1030                1035                1040

Ala Arg Thr Lys Ala Glu Lys Gln Arg Arg Asp Leu Gly Glu Glu Leu
                1045                1050                1055

Glu Ala Leu Arg Gly Glu Leu Glu Asp Thr Leu Asp Ser Thr Asn Ala
            1060                1065                1070

Gln Gln Glu Leu Arg Ser Lys Arg Glu Gln Glu Val Thr Glu Leu Lys
        1075                1080                1085

Lys Thr Leu Glu Glu Glu Thr Arg Ile His Glu Ala Ala Val Gln Glu
    1090                1095                1100

Leu Arg Gln Arg His Gly Gln Ala Leu Gly Glu Leu Ala Glu Gln Leu
1105                1110                1115                1120

Glu Gln Ala Arg Arg Lys Gly Ala Trp Glu Lys Thr Arg Leu Ala Leu
                1125                1130                1135

Glu Ala Glu Val Ser Glu Leu Arg Ala Glu Leu Ser Ser Leu Gln Thr
            1140                1145                1150

Ala Arg Gln Glu Gly Glu Gln Arg Arg Arg Arg Leu Glu Leu Gln Leu
        1155                1160                1165

Gln Glu Val Gln Gly Arg Ala Gly Asp Gly Glu Arg Ala Arg Ala Glu
    1170                1175                1180

Ala Ala Glu Lys Val Pro Ser Leu Gln Ala Glu Leu Glu Asn Val Ser
1185                1190                1195                1200

Gly Ala Leu Asn Glu Ala Glu Ser Lys Thr Ile Arg Leu Ser Lys Glu
                1205                1210                1215

Leu Ser Ser Thr Glu Ala Gln Leu His Asp Ala Gln Glu Leu Leu Gln
            1220                1225                1230

Glu Glu Thr Arg Ala Lys Leu Ala Leu Gly Ser Arg Val Arg Ala Met
        1235                1240                1245

Glu Ala Glu Ala Ala Gly Leu Arg Glu Gln Leu Glu Glu Glu Ala Ala
    1250                1255                1260

Ala Arg Glu Arg Ala Asp His Gln Pro Pro Ser Leu Ser Ser Pro Gln
1265                1270                1275                1280

Leu Ser Glu Trp Arg Arg Arg Gln Glu Glu Glu Ala Gly Ala Leu Glu
                1285                1290                1295

Ala Gly Glu Glu Ala Arg Arg Arg Ala Ala Arg Glu Ala Glu Ala Leu
            1300                1305                1310

Thr Gln Arg Leu Ala Glu Lys Thr Glu Thr Val Asp Arg Leu Glu Arg
        1315                1320                1325

Gly Arg Arg Arg Leu Gly Gln Glu Leu Asp Asp Ala Thr Met Asp Leu
    1330                1335                1340

Glu Gln Gln Arg Gln Leu Val Ser Thr Leu Glu Lys Lys Gln Arg Lys
1345                1350                1355                1360

Phe Asp Gln Leu Leu Ala Glu Glu Lys Ala Ala Val Leu Arg Ala Val
                1365                1370                1375

Glu Glu Arg Glu Arg Ala Glu Ala Glu Gly Arg Glu Arg Glu Ala Arg
            1380                1385                1390

Ala Leu Ser Leu Thr Arg Ala Leu Glu Glu Glu Gln Glu Ala Arg Glu
        1395                1400                1405

Glu Leu Glu Arg Gln Asn Arg Ala Leu Arg Ala Glu Leu Glu Ala Leu
    1410                1415                1420

Leu Ser Ser Lys Asp Asp Val Gly Lys Ser Val His Glu Leu Glu Arg
1425                1430                1435                1440

Ala Cys Arg Val Ala Glu Gln Ala Ala Asn Asp Leu Arg Ala Gln Val
                1445                1450                1455

Thr Glu Leu Glu Asp Glu Leu Thr Ala Ala Glu Asp Ala Lys Leu Arg
            1460                1465                1470

Leu Glu Val Thr Val Gln Ala Leu Lys Thr Gln His Glu Arg Asp Leu
        1475                1480                1485

Gln Gly Arg Asp Glu Ala Gly Glu Glu Arg Arg Arg Gln Leu Ala Lys
    1490                1495                1500

Gln Leu Arg Asp Ala Glu Val Glu Arg Asp Glu Glu Arg Lys Gln Arg
1505                1510                1515                1520

Thr Leu Ala Val Ala Ala Arg Lys Lys Leu Glu Gly Glu Leu Glu Glu
                1525                1530                1535

Leu Lys Ala Gln Met Ala Ser Ala Gly Gln Gly Lys Glu Glu Ala Val
            1540                1545                1550

Lys Gln Leu Arg Lys Met Gln Ala Gln Met Lys Glu Leu Trp Arg Glu
        1555                1560                1565

Val Glu Glu Thr Arg Thr Ser Arg Glu Glu Ile Phe Ser Gln Asn Arg
    1570                1575                1580

Glu Ser Glu Lys Arg Leu Lys Gly Leu Glu Ala Glu Val Leu Arg Leu
1585                1590                1595                1600

Gln Glu Glu Leu Ala Ala Ser Asp Arg Ala Arg Arg Gln Ala Gln Gln
                1605                1610                1615

Asp Arg Asp Glu Met Ala Asp Glu Val Ala Asn Gly Asn Leu Ser Lys
            1620                1625                1630

Ala Ala Ile Leu Glu Glu Lys Arg Gln Leu Glu Gly Arg Leu Gly Gln
        1635                1640                1645

Leu Glu Glu Glu Leu Glu Glu Glu Gln Thr Thr Gln Ser Cys Ser Met
    1650                1655                1660

Thr Ala Thr Ala Ser Cys Ser Cys Arg
1665                1670


<210> 133
<211> 688
<212> DNA
<213> Homo sapiens

<400> 133
cccaaccacg gccaggcttg cgcgcggttc ccctcccggt gggcggattc ctgggcaaga 60
tgaagtgggt gtgggcgctc ttgctgttgg cggcgctggg cagcggccgc gcggagcgcg 120
actgccgagt gagcagcttc cgagtcaagg agaacttcga caaggctcgc ttctctggga 180
cctggtacgc catggccaag aaggaccccg agggcctctt tctgcaggac aacatcgtcg 240
cggagttctc cgtggacgag accggccaga tgagcgccac agccaagggc cgagtccgtc 300
ttttgaataa ctgggacgtg tgcgcagaca tggtgggcac cttcacagac accgaggacc 360
ctgccaagtt caagatgaag tactggggcg tagcctcctt tctccagaaa ggaaatgatg 420
accactggat cgtcgacaca gactacgaca cgtatgccgt gcagtactcc tgccgcctcc 480
tgaacctcga tggcacctgt gctgacagct actccttcgt gttttcccgg gaccccaacg 540
gcctgccccc agaagcgcag aagattgtaa ggcagcggca ggaggagctg tgcctggcca 600
ggcagtacag gctgatcgtc cacaacggtt actgcgatgg cagatcagaa agaaaccttt 660
tgtagcaagg gcgaattcca gcacactg                                    688


<210> 134
<211> 201
<212> PRT
<213> Homo sapiens

<400> 134
Met Lys Trp Val Trp Ala Leu Leu Leu Leu Ala Ala Leu Gly Ser Gly
1               5                   10                  15

Arg Ala Glu Arg Asp Cys Arg Val Ser Ser Phe Arg Val Lys Glu Asn
            20                  25                  30

Phe Asp Lys Ala Arg Phe Ser Gly Thr Trp Tyr Ala Met Ala Lys Lys
        35                  40                  45

Asp Pro Glu Gly Leu Phe Leu Gln Asp Asn Ile Val Ala Glu Phe Ser
    50                  55                  60

Val Asp Glu Thr Gly Gln Met Ser Ala Thr Ala Lys Gly Arg Val Arg
65                  70                  75                  80

Leu Leu Asn Asn Trp Asp Val Cys Ala Asp Met Val Gly Thr Phe Thr
                85                  90                  95

Asp Thr Glu Asp Pro Ala Lys Phe Lys Met Lys Tyr Trp Gly Val Ala
            100                 105                 110

Ser Phe Leu Gln Lys Gly Asn Asp Asp His Trp Ile Val Asp Thr Asp
        115                 120                 125

Tyr Asp Thr Tyr Ala Val Gln Tyr Ser Cys Arg Leu Leu Asn Leu Asp
    130                 135                 140

Gly Thr Cys Ala Asp Ser Tyr Ser Phe Val Phe Ser Arg Asp Pro Asn
145                 150                 155                 160

Gly Leu Pro Pro Glu Ala Gln Lys Ile Val Arg Gln Arg Gln Glu Glu
                165                 170                 175

Leu Cys Leu Ala Arg Gln Tyr Arg Leu Ile Val His Asn Gly Tyr Cys
            180                 185                 190

Asp Gly Arg Ser Glu Arg Asn Leu Leu
        195                 200

<210> 135
<211> 1647
<212> DNA
<213> Homo sapiens

<400> 135
atggccggcg gcgtggacgg ccccatcggg atcccgttcc ccgaccacag cagcgacatc 60
ctgagtgggc tgaacgagca gcggacgcag ggcctgctgt gcgacgtggt gatcctggtg 120
gagggccgcg agttccccac gcaccgctcg gtgctggccg cctgcagcca gtacttcaag 180
aagctgttca cgtcgggcgc cgtggtggac cagcagaacg tgtacgagat cgacttcgtc 240
agcgccgagg cgctcaccgc gctcatggac ttcgcctaca cggccacgct caccgtcagc 300
acagccaacg tgggtgacat cctcagcgcc gcccgcctgc tggagatccc cgccgtgagc 360
cacgtgtgcg ccgacctcct ggaccggcag atcctggcgg ccgacgcggg cgccgacgcc 420
gggcagctgg accttgtaga tcaaattgat cagcgcaacc tcctccgcgc caaggagtac 480
ctcgagttct tccagagcaa ccccatgaac agcctgcccc ccgcggccgc cgccgccgct 540
gccagcttcc cgtggtccgc ctttggggcg tccgatgatg acctggatgc caccaaggag 600
gccgtggccg ccgctgtggc cgccgtggcc gcgggcgact gcaacggctt agacttctat 660
gggccggggc ccccggccga gcggcccccg acgggggacg gggacgaggg cgacagcaac 720
ccgggtctgt ggccagagcg ggatgaggac gcccccaccg gggtctcttt tccgccgccg 780
gtggccccgc cggccgccac gcagaacggc cactacggcc gcggcggaga ggaggaggcc 840
gcctcgctgt cggaggcggc ccccgagccg ggcgactctc cgggcttcct gtcgggagac 900
agcgacgagg agtcgcgggc cgacgacaag ggcgtcatgg actactacct gaagtacttc 960
agcggcgccc acgacggcga cgtctacccg gcctggtcgc agaaggtgga gaagaagatc 1020
cgagccaagg ccttccagaa gtgccccatc tgcgagaagg tcatccaggg cgccggcaag 1080
ctgccgcgac acatccgcac ccacacgggc gagaagccct acgagtgcaa catctgcaag 1140
gtccgcttca ccaggcagga caagctgaag gtgcacatgc ggaagcacac gggcgagaag 1200
ccgtacctgt gccagcagtg cggcgccgcc tttgcccaca actacgacct gaagaaccac 1260
atgcgcgtgc acacgggcct gcgcccctac cagtgcgaca gctgctgcaa gaccttcgtc 1320
cgctccgacc acctgcacag acacctcaag aaagacggct gcaacggcgt cccctcgcgc 1380
cgcggccgca agccccgcgt ccggggcggg gcgcccgacc ccagcccggg ggccaccgcg 1440
acccccggcg cccccgccca gcccagctcc cccgacgccc ggcgcaacgg ccaggagaag 1500
cactttaagg acgaggacga ggacgaggac gtggccagcc ccgacggctt gggccggttg 1560
aatgtagcgg gcgccggtgg aggaggtgac agcggaggtg gccccggggc cgccaccgac 1620
ggtaacttca cagccggact cgcctaa                                    1647

<210> 136
<211> 548
<212> PRT
<213> Homo sapiens

<400> 136
Met Ala Gly Gly Val Asp Gly Pro Ile Gly Ile Pro Phe Pro Asp His
1               5                   10                  15

Ser Ser Asp Ile Leu Ser Gly Leu Asn Glu Gln Arg Thr Gln Gly Leu
            20                  25                  30

Leu Cys Asp Val Val Ile Leu Val Glu Gly Arg Glu Phe Pro Thr His
        35                  40                  45

Arg Ser Val Leu Ala Ala Cys Ser Gln Tyr Phe Lys Lys Leu Phe Thr
    50                  55                  60

Ser Gly Ala Val Val Asp Gln Gln Asn Val Tyr Glu Ile Asp Phe Val
65                  70                  75                  80

Ser Ala Glu Ala Leu Thr Ala Leu Met Asp Phe Ala Tyr Thr Ala Thr
                85                  90                  95

Leu Thr Val Ser Thr Ala Asn Val Gly Asp Ile Leu Ser Ala Ala Arg
            100                 105                 110

Leu Leu Glu Ile Pro Ala Val Ser His Val Cys Ala Asp Leu Leu Asp
        115                 120                 125

Arg Gln Ile Leu Ala Ala Asp Ala Gly Ala Asp Ala Gly Gln Leu Asp
    130                 135                 140

Leu Val Asp Gln Ile Asp Gln Arg Asn Leu Leu Arg Ala Lys Glu Tyr
145                 150                 155                 160

Leu Glu Phe Phe Gln Ser Asn Pro Met Asn Ser Leu Pro Pro Ala Ala
                165                 170                 175

Ala Ala Ala Ala Ala Ser Phe Pro Trp Ser Ala Phe Gly Ala Ser Asp
            180                 185                 190

Asp Asp Leu Asp Ala Thr Lys Glu Ala Val Ala Ala Ala Val Ala Ala
        195                 200                 205

Val Ala Ala Gly Asp Cys Asn Gly Leu Asp Phe Tyr Gly Pro Gly Pro
    210                 215                 220

Pro Ala Glu Arg Pro Pro Thr Gly Asp Gly Asp Glu Gly Asp Ser Asn
225                 230                 235                 240

Pro Gly Leu Trp Pro Glu Arg Asp Glu Asp Ala Pro Thr Gly Gly Leu
                245                 250                 255

Phe Pro Pro Pro Val Ala Pro Pro Ala Ala Thr Gln Asn Gly His Tyr
            260                 265                 270

Gly Arg Gly Gly Glu Glu Glu Ala Ala Ser Leu Ser Glu Ala Ala Pro
        275                 280                 285

Glu Pro Gly Asp Ser Pro Gly Phe Leu Ser Gly Asp Ser Asp Glu Glu
    290                 295                 300

Ser Arg Ala Asp Asp Lys Gly Val Met Asp Tyr Tyr Leu Lys Tyr Phe
305                 310                 315                 320

Ser Gly Ala His Asp Gly Asp Val Tyr Pro Ala Trp Ser Gln Lys Val
                325                 330                 335

Glu Lys Lys Ile Arg Ala Lys Ala Phe Gln Lys Cys Pro Ile Cys Glu
            340                 345                 350

Lys Val Ile Gln Gly Ala Gly Lys Leu Pro Arg His Ile Arg Thr His
        355                 360                 365

Thr Gly Glu Lys Pro Tyr Glu Cys Asn Ile Cys Lys Val Arg Phe Thr
    370                 375                 380

Arg Gln Asp Lys Leu Lys Val His Met Arg Lys His Thr Gly Glu Lys
385                 390                 395                 400

Pro Tyr Leu Cys Gln Gln Cys Gly Ala Ala Phe Ala His Asn Tyr Asp
                405                 410                 415

Leu Lys Asn His Met Arg Val His Thr Gly Leu Arg Pro Tyr Gln Cys
            420                 425                 430

Asp Ser Cys Cys Lys Thr Phe Val Arg Ser Asp His Leu His Arg His
        435                 440                 445

Leu Lys Lys Asp Gly Cys Asn Gly Val Pro Ser Arg Arg Gly Arg Lys
    450                 455                 460

Pro Arg Val Arg Gly Gly Ala Pro Asp Pro Ser Pro Gly Ala Thr Ala
465                 470                 475                 480

Thr Pro Gly Ala Pro Ala Gln Pro Ser Ser Pro Asp Ala Arg Arg Asn
                485                 490                 495

Gly Gln Glu Lys His Phe Lys Asp Glu Asp Glu Asp Glu Asp Val Ala
            500                 505                 510

Ser Pro Asp Gly Leu Gly Arg Leu Asn Val Ala Gly Ala Gly Gly Gly
        515                 520                 525

Gly Asp Ser Gly Gly Gly Pro Gly Ala Ala Thr Asp Gly Asn Phe Thr
    530                 535                 540

Ala Gly Leu Ala
545

<210> 137
<211> 1026
<212> DNA
<213> Homo sapiens

<400> 137
gatcgaggct caggccctgg aaggaccgta aacatttggc cagcttggtt tggatacctg 60
gcagagacca ggttctgaga agcaatggtg acgaaggcct ttgtcttgtt ggccatcttt 120
gcagaagcct ctgcaaaatc gtgtgctcca aataaagcag atgtcattct tgtgttttgc 180
tatcccaaaa ccatcatcac caaaatcccc gagtgtccct atggatggga agttcatcag 240
ctggccctcg gagggctgtg ttacaatggg gtccacgaag gaggttacta ccaatttgtg 300
atcccagatt tatcacctaa aaacaagtcc tattgtggaa cccagtctga gtacaagcca 360
cctatctatc acttctacag tcacatcgtt tccaatgaca ccacagtgat tgtaaaaaac 420
cagcctgtca actactcctt ctcctgcacc taccactcca cctacttggt gaaccaggct 480
gcctttgacc agagtgtcaa tttccttcca aagaatgcca agttctccat caagaaagaa 540
gctccctttg tcctggaggc atccgaaatc ggttcagatc tgtttgcagg agtggaagcc 600
aaagggttaa gcattaggtt taaagtggtc ttgaacagct gttgggccac cccctcggct 660
gacttcatgt atcccttgca gtggcagctg atcaacaagg gctgccccac ggatgaaacc 720
gtcctcgtgc atgagaatgg gagagatcac agggcaacct tccaattcaa tgctttccgg 780
ttccagaaca tccccaaact ctccaaggtg tggttacact gtgagacgtt catctgcgac 840
agtgagaaac tctcctgccc agtgacctgc gataaacgga agcgcctcct gcgagaccag 900
accgggggag tcctggtcgt ggagctctcc ctgcggaatg ttctccacca cctcatcatg 960
atgttgggga tttgtgccgt gttataggag ttagccaggc agctgccgct cctccaccca 1020
caatag 1026

<210> 138
<211> 300
<212> PRT
<213> Homo sapiens

<400> 138
Met Val Thr Lys Ala Phe Val Leu Leu Ala Ile Phe Ala Glu Ala Ser
1               5                   10                  15

Ala Lys Ser Cys Ala Pro Asn Lys Ala Asp Val Ile Leu Val Phe Cys
            20                  25                  30

Tyr Pro Lys Thr Ile Ile Thr Lys Ile Pro Glu Cys Pro Tyr Gly Trp
        35                  40                  45

Glu Val His Gln Leu Ala Leu Gly Gly Leu Cys Tyr Asn Gly Val His
    50                  55                  60

Glu Gly Gly Tyr Tyr Gln Phe Val Ile Pro Asp Leu Ser Pro Lys Asn
65                  70                  75                  80

Lys Ser Tyr Cys Gly Thr Gln Ser Glu Tyr Lys Pro Pro Ile Tyr His
                85                  90                  95

Phe Tyr Ser His Ile Val Ser Asn Asp Thr Thr Val Ile Val Lys Asn
            100                 105                 110

Gln Pro Val Asn Tyr Ser Phe Ser Cys Thr Tyr His Ser Thr Tyr Leu
        115                 120                 125

Val Asn Gln Ala Ala Phe Asp Gln Ser Val Asn Phe Leu Pro Lys Asn
    130                 135                 140

Ala Lys Phe Ser Ile Lys Lys Glu Ala Pro Phe Val Leu Glu Ala Ser
145                 150                 155                 160

Glu Ile Gly Ser Asp Leu Phe Ala Gly Val Glu Ala Lys Gly Leu Ser
                165                 170                 175

Ile Arg Phe Lys Val Val Leu Asn Ser Cys Trp Ala Thr Pro Ser Ala
            180                 185                 190

Asp Phe Met Tyr Pro Leu Gln Trp Gln Leu Ile Asn Lys Gly Cys Pro
        195                 200                 205

Thr Asp Glu Thr Val Leu Val His Glu Asn Gly Arg Asp His Arg Ala
    210                 215                 220

Thr Phe Gln Phe Asn Ala Phe Arg Phe Gln Asn Ile Pro Lys Leu Ser
225                 230                 235                 240

Lys Val Trp Leu His Cys Glu Thr Phe Ile Cys Asp Ser Glu Lys Leu
                245                 250                 255

Ser Cys Pro Val Thr Cys Asp Lys Arg Lys Arg Leu Leu Arg Asp Gln
            260                 265                 270

Thr Gly Gly Val Leu Val Val Glu Leu Ser Leu Arg Asn Val Leu His
        275                 280                 285

His Leu Ile Met Met Leu Gly Ile Cys Ala Val Leu
    290                 295                 300

<210> 139
<211> 1012
<212> DNA
<213> Homo sapiens

<400> 139
   agagaccagg ttctgagaag caatggtgac gaaggccttt gtcttgttgg ccatctttgc 60
   agaagcctct gcaaaatcgt gtgctccaaa taaagcagat gtcattcttg tgttttgcta 120
   tcccaaaacc atcatcacca aaatccccga gtgtccctat ggatgggaag ttcatcagct 180
   ggccctcgga gggctgtgtt acaatggggt ccacgaagga ggttactacc aatttgtgat 240
   cccagattta tcacctaaaa acaagtccta ttgtggaacc cagtctgagt acaagccacc 300
   tatctatcac ttctacagtc acatcgtttc caatgacgcc acagtgattg taaaaaacca 360
   gcctgtcaac tactccttct cctgcaccta ccactccacc tacttggtga accaggctgc 420
   ctttgaccag agagtggcca ctgttcacgt gaagaacggg agcatgggca catttgagag 480
   ccaactgtct ctcaacttct acactaatgc caagttctcc atcaagaaag aagctccctt 540
   tgtcctggag gcatccgaaa tcggttcaga tctgtttgca ggagtggaag ccaaagggtt 600
   aagcattagg tttaaagtgg tcttgaacag ctgttgggcc accccctcgg ctgacttcat 660
   gtatcccttg cagtggcagc tgatcaacaa gggctgcccc acggatgaaa ccgtcctcgt 720
   gcatgagaat gggagagatc acagggcaac cttccaattc aatgctttcc ggttccagaa 780
   catccccaaa ctctccaagg tgtggttaca ctgtgagacg ttcatctgcg acagtgagaa 840
   actctcctgc ccagtgacct gcgataaacg gaagcgcctc ctgcgagacc agaccggggg 900
   agtcctggtc gtggagctct ccctgcggag caggggattt tccagtctct atagcttctc 960
   agatgttctc caccacctca tcatgatgtt ggggatttgt gccgtgttat ag 1012

<210> 140
<211> 329
<212> PRT
<213> Homo sapiens

<400> 140
Met Val Thr Lys Ala Phe Val Leu Leu Ala Ile Phe Ala Glu Ala Ser
1               5                   10                  15

Ala Lys Ser Cys Ala Pro Asn Lys Ala Asp Val Ile Leu Val Phe Cys
            20                  25                  30

Tyr Pro Lys Thr Ile Ile Thr Lys Ile Pro Glu Cys Pro Tyr Gly Trp
        35                  40                  45

Glu Val His Gln Leu Ala Leu Gly Gly Leu Cys Tyr Asn Gly Val His
    50                  55                  60

Glu Gly Gly Tyr Tyr Gln Phe Val Ile Pro Asp Leu Ser Pro Lys Asn
65                  70                  75                  80

Lys Ser Tyr Cys Gly Thr Gln Ser Glu Tyr Lys Pro Pro Ile Tyr His
                85                  90                  95

Phe Tyr Ser His Ile Val Ser Asn Asp Ala Thr Val Ile Val Lys Asn
            100                 105                 110

Gln Pro Val Asn Tyr Ser Phe Ser Cys Thr Tyr His Ser Thr Tyr Leu
        115                 120                 125

Val Asn Gln Ala Ala Phe Asp Gln Arg Val Ala Thr Val His Val Lys
    130                 135                 140

Asn Gly Ser Met Gly Thr Phe Glu Ser Gln Leu Ser Leu Asn Phe Tyr
145                 150                 155                 160

Thr Asn Ala Lys Phe Ser Ile Lys Lys Glu Ala Pro Phe Val Leu Glu
                165                 170                 175

Ala Ser Glu Ile Gly Ser Asp Leu Phe Ala Gly Val Glu Ala Lys Gly
            180                 185                 190

Leu Ser Ile Arg Phe Lys Val Val Leu Asn Ser Cys Trp Ala Thr Pro
        195                 200                 205

Ser Ala Asp Phe Met Tyr Pro Leu Gln Trp Gln Leu Ile Asn Lys Gly
    210                 215                 220

Cys Pro Thr Asp Glu Thr Val Leu Val His Glu Asn Gly Arg Asp His
225                 230                 235                 240

Arg Ala Thr Phe Gln Phe Asn Ala Phe Arg Phe Gln Asn Ile Pro Lys
                245                 250                 255

Leu Ser Lys Val Trp Leu His Cys Glu Thr Phe Ile Cys Asp Ser Glu
            260                 265                 270

Lys Leu Ser Cys Pro Val Thr Cys Asp Lys Arg Lys Arg Leu Leu Arg
        275                 280                 285

Asp Gln Thr Gly Gly Val Leu Val Val Glu Leu Ser Leu Arg Ser Arg
    290                 295                 300

Gly Phe Ser Ser Leu Tyr Ser Phe Ser Asp Val Leu His His Leu Ile
305                 310                 315                 320

Met Met Leu Gly Ile Cys Ala Val Leu
                325

<210> 141
<211> 1012
<212> DNA
<213> Homo sapiens

<400> 141
agagaccagg ttctgagaag caatggtgac gaaggccttt gtcttgttgg ccatctttgc 60
agaagcctct gcaaaatcgt gtgctccaaa taaagcagat gtcattcttg tgttttgcta 120
tcccaaaacc atcatcacca aaatccccga gtgtccctat ggatgggaag ttcatcagct 180
ggccctcgga gggctgtgtt acaatggggt ccacgaagga ggttactacc aatttgtgat 240
cccagattta tcacctaaaa acaagtccta ttgtggaacc cagtctgagt acaagccacc 300
tatctatcac ttctacagtc acatcgtttc caatgacacc acagtgattg taaaaaacca 360
gcctgtcaac tactccttct cctgcaccta ccactccacc tacttggtga accaggctgc 420
ctttgaccag agagtggcca ctgttcacgt gaagaacggg agcatgggca catttgagag 480
ccaactgtct ctcaacttct acactaatgc caagttctcc atcaagaaag aagctccctt 540
tgtcctggag gcatcggaaa tcggttcaga tctgtttgca ggagtggaag ccaaagggtt 600
aagcattagg tttaaagtgg tcttgaacag ctgttgggcc acccccctcgg ctgacttcat 660
gtatcccttg cagtggcagc tgatcaacaa gggctgcccc acggatgaaa ccgtcctcgt 720
gcatgagaat gggagagatc acagggcaac cttccaattc aatgctttcc ggttccagaa 780
catccccaaa ctctccaagg tgtggttaca ctgtgagacg ttcatctgcg acagtgagaa 840
actctcctgc ccagtgacct gcgataaacg gaagcgcctc ctgcgagacc agaccggggg 900
agtcctggtc gtggagctct ccctgcggag caggggattt tccagtctct atagcttctc 960
agatgttctc caccacctca tcatgatgtt ggggatttgt gccgtgttat ag       1012

<210> 142
<211> 329
<212> PRT
<213> Homo sapiens

<400> 142
Met Val Thr Lys Ala Phe Val Leu Leu Ala Ile Phe Ala Glu Ala Ser
1               5                   10                  15

Ala Lys Ser Cys Ala Pro Asn Lys Ala Asp Val Ile Leu Val Phe Cys
            20                  25                  30

Tyr Pro Lys Thr Ile Ile Thr Lys Ile Pro Glu Cys Pro Tyr Gly Trp
        35                  40                  45

Glu Val His Gln Leu Ala Leu Gly Gly Leu Cys Tyr Asn Gly Val His
    50                  55                  60

Glu Gly Gly Tyr Tyr Gln Phe Val Ile Pro Asp Leu Ser Pro Lys Asn
65                  70                  75                  80

Lys Ser Tyr Cys Gly Thr Gln Ser Glu Tyr Lys Pro Pro Ile Tyr His
                85                  90                  95

Phe Tyr Ser His Ile Val Ser Asn Asp Thr Thr Val Ile Val Lys Asn
            100                 105                 110

Gln Pro Val Asn Tyr Ser Phe Ser Cys Thr Tyr His Ser Thr Tyr Leu
        115                 120                 125

Val Asn Gln Ala Ala Phe Asp Gln Arg Val Ala Thr Val His Val Lys

130                 135                 140

Asn Gly Ser Met Gly Thr Phe Glu Ser Gln Leu Ser Leu Asn Phe Tyr
145                 150                 155                 160

Thr Asn Ala Lys Phe Ser Ile Lys Lys Glu Ala Pro Phe Val Leu Glu
                165                 170                 175

Ala Ser Glu Ile Gly Ser Asp Leu Phe Ala Gly Val Glu Ala Lys Gly
            180                 185                 190

Leu Ser Ile Arg Phe Lys Val Val Leu Asn Ser Cys Trp Ala Thr Pro
        195                 200                 205

Ser Ala Asp Phe Met Tyr Pro Leu Gln Trp Gln Leu Ile Asn Lys Gly
    210                 215                 220

Cys Pro Thr Asp Glu Thr Val Leu Val His Glu Asn Gly Arg Asp His
225                 230                 235                 240

Arg Ala Thr Phe Gln Phe Asn Ala Phe Arg Phe Gln Asn Ile Pro Lys
                245                 250                 255

Leu Ser Lys Val Trp Leu His Cys Glu Thr Phe Ile Cys Asp Ser Glu
            260                 265                 270

Lys Leu Ser Cys Pro Val Thr Cys Asp Lys Arg Lys Arg Leu Leu Arg
        275                 280                 285

Asp Gln Thr Gly Gly Val Leu Val Val Glu Leu Ser Leu Arg Ser Arg
    290                 295                 300

Gly Phe Ser Ser Leu Tyr Ser Phe Ser Asp Val Leu His His Leu Ile
305                 310                 315                 320

Met Met Leu Gly Ile Cys Ala Val Leu
                325

<210> 143
<211> 3909
<212> DNA
<213> Homo sapiens

<400> 143
   gggcagccaa tcggggatga gcttttatta ggcggccaga tcatcagccg aagtgccaaa 60
   ccctttttct gtgagaacta ggagcctgtc ctccatgttt tataagtatt gacattacac 120
   agtgttaaca atgcatccac agagcttggc tgaagaggaa ataaaaacag aacaggaggt 180
   ggtagagggc atggatatct ctactcgctc caaagatcct ggctctgcag agagaacagc 240
   ccagaaaaga aagttcccca gccctccaca ttcttccaat ggccactcgc cgcaggacac 300
   atcaacaagc cccattaaaa agaaaaagaa acctggctta ctgaacagta acaataagga 360
   gcagtcagaa ctaagacatg gtccgtttta ctatatgaag cagccactca ccacagaccc 420
   tgttgatgtt gtaccgcagg atggacggaa tgatttctac tgctgggttt gtcaccggga 480
   aggccaagtc ctttgctgtg agctctgtcc ccgggtttat cacgctaagt gtctgagact 540
   gacatcggaa ccagaggggg actggttttg tcctgaatgt gagaaaatta cagtagcaga 600
   atgcatcgag acccagagta aagccatgac aatgctcacc attgaacagt tatcctacct 660
   gctcaagttt gccattcaga aaatgaaaca gccagggaca gatgcattcc agaagcccgt 720
   tccattggaa cagcaccctg actatgcgga atacatcttc catccaatgg acctttgtac 780
   attggaaaag aatgcgaaaa agaaaatgta tggctgcaca gaagccttcc tggctgatgc 840
   aaagtggatt ttgcacaact gcatcattta taatggggga aatcacaaat tgacgcaaat 900
   agcgaaagta gtcatcaaaa tctgtgaaca tgagatgaat gaaatcgaag tatgtccaga 960
   atgttatcta gctgcttgcc aaaaacgaga taactggttt tgtgagcctt gtagcaatcc 1020
   acatcctttg gtctggccaa aactgaaggg gtttccattc tggcctgcaa aagctctaag 1080
   ggataaagac gggcaggtcg atgcccgatt ctttggacaa catgacaggg cctgggttcc 1140
   aataaataat tgctacctca tgtctaaaga aattcctttt tctgtgaaaa agactaagag 1200
   catcttcaac agtgccatgc aagagatgga ggtttacgtg gagaacatcc gcaggaagtt 1260
   tggggttttt aattactctc catttaggac accctacaca cccaacagcc agtatcaaat 1320
   gctgctcgat cccaccaacc ccagcgccgg cactgccaag atagacaagc aggagaaggt 1380
   caagctcaac tttgacatga cggcatcccc caagatcctg atgagcaagc ctgtgctgag 1440
   tgggggcaca ggccgccgga tttccttgtc ggatatgccg cgctccccca tgagcacaaa 1500
   ctcttctgtg cacacgggct ccgacgtgga gcaggatgct gagaagaagg ccacgtcgag 1560
   ccacttcagt gcgagcgagg agtccatgga cttcctggat aagagcacag cttcaccagc 1620
   ctccaccaag acgggacaag cagggagttt atccggcagc ccaaagccct tctctcctca 1680
   actgtcagct cctatcacga cgaaaacgga caaaacctcc accaccggca gcatcctgaa 1740
   tcttaacctg gatcgaagca aagctgagat ggatttgaag gagctgagcg agtcggtcca 1800
   gcaacagtcc acccctgttc ctctcatctc tcccaagcgc cagattcgta gcaggttcca 1860
   gctgaatctt gacaagacca tagagagttg caaagcacaa ttaggcataa atgaaatctc 1920
   ggaagatgtc tatacggccg tagagcacag cgattcggag gattctgaga agtcagatag 1980
   tagcgatagt gagtatatca gtgatgatga gcagaagtct aagaacgagc cagaagacac 2040
   agaggacaaa gaaggttgtc agatggacaa agagccatct gctgttaaaa aaaagcccaa 2100
   gcctacaaac ccagtggaga ttaaagagga gctgaaaagc acgtcaccag ccagcgagaa 2160
   ggcagaccct ggagcagtca aggacaaggc cagccctgag cctgagaagg acttttccga 2220
   aaaggcaaaa ccttcacctc accccataaa ggataaactg aagggaaaag atgagacgga 2280
   ttccccaaca gtccatttgg gcctggactc tgattcagag agcgaacttg tcatagattt 2340
   aggagaagac cattctgggc gggagggtcg aaaaaataag aaggaaccca aagaaccatc 2400
   tcccaaacag gatgttgtag gtaaaactcc accatccacg acggtgggca gccattctcc 2460
   cccggaaaca ccggtgctca cccgctcttc cgcccaaact tccgcggctg gcgccacagc 2520
   caccaccagc acgtcctcca cggtcaccgt cacggccccg gcccccgccg ccacaggaag 2580
   cccagtgaaa aagcagaggc cgcttttacc gaaggagact gccccggccg tgcagcgggt 2640
   cgtgtggaac tcatcaagta agtttcaaac gtcctcccaa aagtggcaca tgcagaagat 2700
   gcagcgtcag cagcagcagc agcagcagca aaaccagcag cagcagcctc agtcttccca 2760
   ggggacgaga tatcagacca gacaggctgt gaaagctgtc cagcagaagg agatcacaca 2820
   gagcccatcc acgtccacca tcaccctggt gaccagcaca cagtcatcgc ccctggtcac 2880
   cagctcgggg tccatgagca cccttgtgtc ctcagtcaac gctgacctgc ccatcgccac 2940
   tgcctcagct gatgtcgccg ctgatattgc caagtacact agcaaaatga tggatgcaat 3000
   aaaaggaaca atgacagaaa tatacaacga tctttctaaa aacactactg gaagcacaat 3060
   agctgagatt cgcaggctga ggatcgagat agagaagctc cagtggctgc accagcaaga 3120
   gctctccgaa atgaaacaca acttagagct gaccatggcg gagatgcggc agagcctgga 3180
   gcaggagcgg gaccggctca tcgccgaggt gaagaagcag ctggagttgg agaagcagca 3240
   ggcggtggat gagaccaaga agaagcagtg gtgcgccaac tgcaagaagg aggccatctt 3300
   ttactgctgt tggaacacta gctactgtga ctacccctgc cagcaagccc actggcctga 3360
   gcacatgaag tcctgcaccc agtcagctac tgctcctcag caggaagcgg atgctgaggt 3420
   gaacacagaa acactaaata agtcctccca ggggagctcc tcgagcacac aatcagcacc 3480
   ttcagaaacg gccagcgcct ccaaagagaa ggagacgtca gctgagaaaa gcaaggagag 3540
   tggctcgacc cttgaccttt ctggctccag agagacgccc tcctccattc tcttaggctc 3600
   caaccaaggc tctgaccatt cccggagtaa taaatccagt tggagcagca gtgatgagaa 3660
   gaggggatcg acacgttccg atcacaacac cagtaccagc acgaagagcc tcctcccgaa 3720
   agagtctcgg ctggacacct tctgggacta gcagtgaatc gggacacaaa ccacccaccc 3780
   cattgggaga aaaacccaga cgccaggaaa agaagaaaca acaaaggcag gagaacagcc 3840
   actttcagac ttgaaaatga caaaaccctc agttgagcct gagcccccgg cgcgggggct 3900
   gctacacta 3909

<210> 144
<211> 1206
<212> PRT
<213> Homo sapiens

<400> 144
Met His Pro Gln Ser Leu Ala Glu Glu Glu Ile Lys Thr Glu Gln Glu
1               5                   10                  15

Val Val Glu Gly Met Asp Ile Ser Thr Arg Ser Lys Asp Pro Gly Ser
            20                  25                  30

Ala Glu Arg Thr Ala Gln Lys Arg Lys Phe Pro Ser Pro Pro His Ser
        35                  40                  45

Ser Asn Gly His Ser Pro Gln Asp Thr Ser Thr Ser Pro Ile Lys Lys
    50                  55                  60

Lys Lys Lys Pro Gly Leu Leu Asn Ser Asn Asn Lys Glu Gln Ser Glu
65                  70                  75                  80

Leu Arg His Gly Pro Phe Tyr Tyr Met Lys Gln Pro Leu Thr Thr Asp
                85                  90                  95

Pro Val Asp Val Val Pro Gln Asp Gly Arg Asn Asp Phe Tyr Cys Trp

            100                 105                 110

Val Cys His Arg Glu Gly Gln Val Leu Cys Cys Glu Leu Cys Pro Arg
        115                 120                 125

Val Tyr His Ala Lys Cys Leu Arg Leu Thr Ser Glu Pro Glu Gly Asp
    130                 135                 140

Trp Phe Cys Pro Glu Cys Glu Lys Ile Thr Val Ala Glu Cys Ile Glu
145                 150                 155                 160

Thr Gln Ser Lys Ala Met Thr Met Leu Thr Ile Glu Gln Leu Ser Tyr
                165                 170                 175

Leu Leu Lys Phe Ala Ile Gln Lys Met Lys Gln Pro Gly Thr Asp Ala
            180                 185                 190

Phe Gln Lys Pro Val Pro Leu Glu Gln His Pro Asp Tyr Ala Glu Tyr
        195                 200                 205

Ile Phe His Pro Met Asp Leu Cys Thr Leu Glu Lys Asn Ala Lys Lys
    210                 215                 220

Lys Met Tyr Gly Cys Thr Glu Ala Phe Leu Ala Asp Ala Lys Trp Ile
225                 230                 235                 240

Leu His Asn Cys Ile Ile Tyr Asn Gly Gly Asn His Lys Leu Thr Gln
                245                 250                 255

Ile Ala Lys Val Val Ile Lys Ile Cys Glu His Glu Met Asn Glu Ile
            260                 265                 270

Glu Val Cys Pro Glu Cys Tyr Leu Ala Ala Cys Gln Lys Arg Asp Asn
        275                 280                 285

Trp Phe Cys Glu Pro Cys Ser Asn Pro His Pro Leu Val Trp Ala Lys
    290                 295                 300

Leu Lys Gly Phe Pro Phe Trp Pro Ala Lys Ala Leu Arg Asp Lys Asp
305                 310                 315                 320

Gly Gln Val Asp Ala Arg Phe Phe Gly Gln His Asp Arg Ala Trp Val
                325                 330                 335

Pro Ile Asn Asn Cys Tyr Leu Met Ser Lys Glu Ile Pro Phe Ser Val
            340                 345                 350

Lys Lys Thr Lys Ser Ile Phe Asn Ser Ala Met Gln Glu Met Glu Val
        355                 360                 365

Tyr Val Glu Asn Ile Arg Arg Lys Phe Gly Val Phe Asn Tyr Ser Pro
    370                 375                 380

Phe Arg Thr Pro Tyr Thr Pro Asn Ser Gln Tyr Gln Met Leu Leu Asp
385                 390                 395                 400

Pro Thr Asn Pro Ser Ala Gly Thr Ala Lys Ile Asp Lys Gln Glu Lys
                405                 410                 415

Val Lys Leu Asn Phe Asp Met Thr Ala Ser Pro Lys Ile Leu Met Ser
            420                 425                 430

Lys Pro Val Leu Ser Gly Gly Thr Gly Arg Arg Ile Ser Leu Ser Asp
        435                 440                 445

Met Pro Arg Ser Pro Met Ser Thr Asn Ser Ser Val His Thr Gly Ser
    450                 455                 460

Asp Val Glu Gln Asp Ala Glu Lys Lys Ala Thr Ser Ser His Phe Ser

465                 470                 475                 480

Ala Ser Glu Glu Ser Met Asp Phe Leu Asp Lys Ser Thr Ala Ser Pro
                485                 490                 495

Ala Ser Thr Lys Thr Gly Gln Ala Gly Ser Leu Ser Gly Ser Pro Lys
            500                 505                 510

Pro Phe Ser Pro Gln Leu Ser Ala Pro Ile Thr Thr Lys Thr Asp Lys
        515                 520                 525

Thr Ser Thr Thr Gly Ser Ile Leu Asn Leu Asn Leu Asp Arg Ser Lys
    530                 535                 540

Ala Glu Met Asp Leu Lys Glu Leu Ser Glu Ser Val Gln Gln Gln Ser
545                 550                 555                 560

Thr Pro Val Pro Leu Ile Ser Pro Lys Arg Gln Ile Arg Ser Arg Phe
                565                 570                 575

Gln Leu Asn Leu Asp Lys Thr Ile Glu Ser Cys Lys Ala Gln Leu Gly
            580                 585                 590

Ile Asn Glu Ile Ser Glu Asp Val Tyr Thr Ala Val Glu His Ser Asp
        595                 600                 605

Ser Glu Asp Ser Glu Lys Ser Asp Ser Ser Asp Ser Glu Tyr Ile Ser
    610                 615                 620

Asp Asp Glu Gln Lys Ser Lys Asn Glu Pro Glu Asp Thr Glu Asp Lys
625                 630                 635                 640

Glu Gly Cys Gln Met Asp Lys Glu Pro Ser Ala Val Lys Lys Lys Pro
                645                 650                 655

Lys Pro Thr Asn Pro Val Glu Ile Lys Glu Glu Leu Lys Ser Thr Ser
            660                 665                 670

Pro Ala Ser Glu Lys Ala Asp Pro Gly Ala Val Lys Asp Lys Ala Ser
        675                 680                 685

Pro Glu Pro Glu Lys Asp Phe Ser Glu Lys Ala Lys Pro Ser Pro His
    690                 695                 700

Pro Ile Lys Asp Lys Leu Lys Gly Lys Asp Glu Thr Asp Ser Pro Thr
705                 710                 715                 720

Val His Leu Gly Leu Asp Ser Asp Ser Glu Ser Glu Leu Val Ile Asp
                725                 730                 735

Leu Gly Glu Asp His Ser Gly Arg Glu Gly Arg Lys Asn Lys Lys Glu
            740                 745                 750

Pro Lys Glu Pro Ser Pro Lys Gln Asp Val Val Gly Lys Thr Pro Pro
        755                 760                 765

Ser Thr Thr Val Gly Ser His Ser Pro Pro Glu Thr Pro Val Leu Thr
    770                 775                 780

Arg Ser Ser Ala Gln Thr Ser Ala Ala Gly Ala Thr Ala Thr Thr Ser
785                 790                 795                 800

Thr Ser Ser Thr Val Thr Val Thr Ala Pro Ala Pro Ala Ala Thr Gly
                805                 810                 815

Ser Pro Val Lys Lys Gln Arg Pro Leu Leu Pro Lys Glu Thr Ala Pro
            820                 825                 830

Ala Val Gln Arg Val Val Trp Asn Ser Ser Ser Lys Phe Gln Thr Ser

835                 840                 845

Ser Gln Lys Trp His Met Gln Lys Met Gln Arg Gln Gln Gln Gln Gln
    850                 855                 860

Gln Gln Gln Asn Gln Gln Gln Gln Pro Gln Ser Ser Gln Gly Thr Arg
865                 870                 875                 880

Tyr Gln Thr Arg Gln Ala Val Lys Ala Val Gln Gln Lys Glu Ile Thr
                885                 890                 895

Gln Ser Pro Ser Thr Ser Thr Ile Thr Leu Val Thr Ser Thr Gln Ser
            900                 905                 910

Ser Pro Leu Val Thr Ser Ser Gly Ser Met Ser Thr Leu Val Ser Ser
        915                 920                 925

Val Asn Ala Asp Leu Pro Ile Ala Thr Ala Ser Ala Asp Val Ala Ala
    930                 935                 940

Asp Ile Ala Lys Tyr Thr Ser Lys Met Met Asp Ala Ile Lys Gly Thr
945                 950                 955                 960

Met Thr Glu Ile Tyr Asn Asp Leu Ser Lys Asn Thr Thr Gly Ser Thr
                965                 970                 975

Ile Ala Glu Ile Arg Arg Leu Arg Ile Glu Ile Glu Lys Leu Gln Trp
            980                 985                 990

Leu His Gln Gln Glu Leu Ser Glu Met Lys His Asn Leu Glu Leu Thr
        995                 1000                1005

Met Ala Glu Met Arg Gln Ser Leu Glu Gln Glu Arg Asp Arg Leu Ile
    1010                1015                1020

Ala Glu Val Lys Lys Gln Leu Glu Leu Glu Lys Gln Gln Ala Val Asp
1025                1030                1035                1040

Glu Thr Lys Lys Lys Gln Trp Cys Ala Asn Cys Lys Lys Glu Ala Ile
                1045                1050                1055

Phe Tyr Cys Cys Trp Asn Thr Ser Tyr Cys Asp Tyr Pro Cys Gln Gln
            1060                1065                1070

Ala His Trp Pro Glu His Met Lys Ser Cys Thr Gln Ser Ala Thr Ala
        1075                1080                1085

Pro Gln Gln Glu Ala Asp Ala Glu Val Asn Thr Glu Thr Leu Asn Lys
    1090                1095                1100

Ser Ser Gln Gly Ser Ser Ser Ser Thr Gln Ser Ala Pro Ser Glu Thr
1105                1110                1115                1120

Ala Ser Ala Ser Lys Glu Lys Glu Thr Ser Ala Glu Lys Ser Lys Glu
                1125                1130                1135

Ser Gly Ser Thr Leu Asp Leu Ser Gly Ser Arg Glu Thr Pro Ser Ser
            1140                1145                1150

Ile Leu Leu Gly Ser Asn Gln Gly Ser Asp His Ser Arg Ser Asn Lys
        1155                1160                1165

Ser Ser Trp Ser Ser Ser Asp Glu Lys Arg Gly Ser Thr Arg Ser Asp
    1170                1175                1180

His Asn Thr Ser Thr Ser Thr Lys Ser Leu Leu Pro Lys Glu Ser Arg
1185                1190                1195                1200

Leu Asp Thr Phe Trp Asp

                1205

<210> 145
<211> 1629
<212> DNA
<213> Homo sapiens

<400> 145
tatagtcttg ctctttggga tgctgaaggt gctgaaatag caatgacaag agacttggct 60
cagtgttaaa taactgccgc gctggcctga cagtctctga gatgacaata gggagaatgg 120
agaacgtgga ggtcttcacc gctgagggca aaggaagggg tctgaaggcc accaaggagt 180
tctgggctgc agatatcatc tttgctgagc gggcttattc cgcagtggtt tttgacagcc 240
ttgttaattt tgtgtgccac acctgcttca agaggcagga gaagctccat cgctgtgggc 300
agtgcaagtt tgcccattac tgcgaccgca cctgccagaa ggatgcttgg ctgaaccaca 360
agaatgaatg ttcggccatc aagagatatg ggaaggtgcc caatgagaac atcaggctgg 420
cggcgcgcat catgtggcgg gtggagagag aaggcaccgg gctcacggag ggctgcctgg 480
tgtccgtgga cgacttgcag aaccacgtgg agcactttgg ggaggaggag cagaaggacc 540
tgcgggtgga cgtggacaca ttcttgcagt actggccgcc gcagagccag ccgttcagca 600
tgcagtacat ctcgcacatc ttcggagtga ttaactgcaa cggttttact ctcagtgatc 660
agagaggcct gcaggccgtg ggcgtaggca tcttccccaa cctgggcctg gtgaaccatg 720
actgttggcc caactgtact gtcatattta acaatggcaa tcatgaggca gtgaaatcca 780
tgtttcatac ccagatgaga attgaactgc gggccctagg caagatctca gaaggagagg 840
agctgactgt gtcctatatc gacttcctca acgttagtga agaacgcaag aggcagctga 900
agaagcagta ctactttgac tgcacatgtg aacactgcca gaaaaaactg aaggatgacc 960
tcttcctggg ggtgaaagac aaccccaagc cctctcagga agtggtgaag gagatgatac 1020
aattctccaa ggatacattg gaaaagatag acaaggctcg ttccgagggt ttgtatcatg 1080
aggttgtgaa attatgccgg gagtgcctgg agaagcagga gccagtgttt gctgacacca 1140
acatctacat gctgcggatg ctgagcattg tttcggaggt cctttcctac ctccaggcct 1200
ttgaggaggc ctcgttctat gccaggagga tggtggacgg ctatatgaag ctctaccacc 1260
ccaacaatgc ccaactgggc atggccgtga tgcgggcagg gctgaccaac tggcacgctg 1320
gtaacattga ggtggggcac gggatgatct gcaaagccta tgccattctc ctggtgacac 1380
acggaccctc ccaccccatc actaaggact tagaggccat gcgggtgcag acggagatgg 1440
agctacgcat gttccgccag aacgaattca tgtactacaa gatgcgcgag gctgccctga 1500
acaaccagcc catgcaggtc atggccgagc ccagcaatga gccatcccca gctctgttcc 1560
acaagaagca atgaggactg cccagtggag gaggggcgat gtggctgggg agctagggag 1620
agactctgg 1629

<210> 146
<211> 490
<212> PRT
<213> Homo sapiens

<400> 146
Met Thr Ile Gly Arg Met Glu Asn Val Glu Val Phe Thr Ala Glu Gly
1               5                   10                  15

Lys Gly Arg Gly Leu Lys Ala Thr Lys Glu Phe Trp Ala Ala Asp Ile
            20                  25                  30

Ile Phe Ala Glu Arg Ala Tyr Ser Ala Val Val Phe Asp Ser Leu Val
        35                  40                  45

Asn Phe Val Cys His Thr Cys Phe Lys Arg Gln Glu Lys Leu His Arg
    50                  55                  60

Cys Gly Gln Cys Lys Phe Ala His Tyr Cys Asp Arg Thr Cys Gln Lys
65                  70                  75                  80

Asp Ala Trp Leu Asn His Lys Asn Glu Cys Ser Ala Ile Lys Arg Tyr
                85                  90                  95

Gly Lys Val Pro Asn Glu Asn Ile Arg Leu Ala Ala Arg Ile Met Trp
            100                 105                 110

Arg Val Glu Arg Glu Gly Thr Gly Leu Thr Glu Gly Cys Leu Val Ser
        115                 120                 125

Val Asp Asp Leu Gln Asn His Val Glu His Phe Gly Glu Glu Glu Gln
    130                 135                 140
Lys Asp Leu Arg Val Asp Val Asp Thr Phe Leu Gln Tyr Trp Pro Pro
145                 150                 155                 160
Gln Ser Gln Pro Phe Ser Met Gln Tyr Ile Ser His Ile Phe Gly Val
                165                 170                 175
Ile Asn Cys Asn Gly Phe Thr Leu Ser Asp Gln Arg Gly Leu Gln Ala
            180                 185                 190
Val Gly Val Gly Ile Phe Pro Asn Leu Gly Leu Val Asn His Asp Cys
        195                 200                 205
Trp Pro Asn Cys Thr Val Ile Phe Asn Asn Gly Asn His Glu Ala Val
    210                 215                 220
Lys Ser Met Phe His Thr Gln Met Arg Ile Glu Leu Arg Ala Leu Gly
225                 230                 235                 240
Lys Ile Ser Glu Gly Glu Glu Leu Thr Val Ser Tyr Ile Asp Phe Leu
                245                 250                 255
Asn Val Ser Glu Glu Arg Lys Arg Gln Leu Lys Lys Gln Tyr Tyr Phe
            260                 265                 270
Asp Cys Thr Cys Glu His Cys Gln Lys Lys Leu Lys Asp Asp Leu Phe
        275                 280                 285
Leu Gly Val Lys Asp Asn Pro Lys Pro Ser Gln Glu Val Val Lys Glu
    290                 295                 300
Met Ile Gln Phe Ser Lys Asp Thr Leu Glu Lys Ile Asp Lys Ala Arg
305                 310                 315                 320
Ser Glu Gly Leu Tyr His Glu Val Val Lys Leu Cys Arg Glu Cys Leu
                325                 330                 335
Glu Lys Gln Glu Pro Val Phe Ala Asp Thr Asn Ile Tyr Met Leu Arg
            340                 345                 350
Met Leu Ser Ile Val Ser Glu Val Leu Ser Tyr Leu Gln Ala Phe Glu
        355                 360                 365
Glu Ala Ser Phe Tyr Ala Arg Arg Met Val Asp Gly Tyr Met Lys Leu
    370                 375                 380
Tyr His Pro Asn Asn Ala Gln Leu Gly Met Ala Val Met Arg Ala Gly
385                 390                 395                 400
Leu Thr Asn Trp His Ala Gly Asn Ile Glu Val Gly His Gly Met Ile
                405                 410                 415
Cys Lys Ala Tyr Ala Ile Leu Leu Val Thr His Gly Pro Ser His Pro
            420                 425                 430
Ile Thr Lys Asp Leu Glu Ala Met Arg Val Gln Thr Glu Met Glu Leu
        435                 440                 445
Arg Met Phe Arg Gln Asn Glu Phe Met Tyr Tyr Lys Met Arg Glu Ala
    450                 455                 460
Ala Leu Asn Asn Gln Pro Met Gln Val Met Ala Glu Pro Ser Asn Glu
465                 470                 475                 480
Pro Ser Pro Ala Leu Phe His Lys Lys Gln
                485                 490

<210> 147
<211> 1555
<212> DNA
<213> Homo sapiens

<400> 147
aaggtgctga aatagcaatg acaagagact tagctcagtg ttaaataact gccgcgctgg 60
cctgacagtt tctgagatga caatagggag aatggagaac gtggaggtct tcaccgctga 120
gggcaaagga aggggtctga aggccaccaa ggagttctgg gctgcagata tcatctttgc 180
tgatcgggct tattccgcag tggtttttga cagccttgtt aattttgtgt gccacacctg 240
cttcaagagg caggagaagc tccatcgctg tgggcagtgc aagtttgccc attactgcga 300
ccgcacctgc cagaaggatg cttggctgaa ccacaagaat gaatgttcgg ccatcaagag 360
atatgggaag gtgcccaatg agaacatcag gctggcggcg cgcatcatgt ggagggtgga 420
gagagaaggc accgggctca cggagggctg cctggtgtcc gtggacgact tgcagaacca 480
cgtggagcac tttggggagg aggagcagaa ggacctgcgg gtggacgtgg acacattctt 540
gcagtactgg ccgccgcaga gccagcagtt cagcatgcag tacatctcgc acatcttcgg 600
agtgattaac tgcaacggtt ttactctcag tgatcagaga ggcctgcagg ccgtgggcgt 660
aggcatcttc cccaacctgg gcctggtgaa ccatgactgt tggcccaact gtactgtcat 720
atttaacaat ggcaatcatg aggcagtgaa atccatgttt catacccaga tgagaattga 780
gctccgggcc ctaggcaaga tctcagaagg agaggagctg actgtgtcct atattgactt 840
cctcaacgtt agtgaagaac gcaagaggca gctgaagaag cagtactact ttgactgcac 900
atgtgaacac tgccagaaaa aactgaagga tgacctcttc ctgggggtga aagacaaccc 960
caagccctct caggaagtgg tgaaggagat gatacaattc tccaaggata cattggaaaa 1020
gatagacaag gctcgttccg agggtttgta tcatgaggtt gtgaaattat gccgggagtg 1080
cctggagaag caggagccag tgtttgctga caccaacatc tacatgctgc ggatgctgag 1140
cattgtttcg gaggtccttt cctacctcca ggcctttgag gaggcctcgt tctatgccag 1200
gaggatggtg gacggctata tgaagctcta ccaccccaac aatgcccaac tgggcatggt 1260
cgtgatgcgg gcagggctga ccaactggca tgctggtaac attgaggtgg ggcacgggat 1320
gatctgcaaa gcctatgcca ttctcctggt gacacacgga ccctcccacc ccatcactaa 1380
ggacttagag gccatgcggg tgcagacgga gatggagcta cgcatgttcc gccagaacga 1440
attcatgtac tacaagatgc gcgaggctgc cctgaacaac cagcccatgc aggtcatggc 1500
cgagcccagc aatgagccat ccccagctct gttccacaag aagcaatgag gactg 1555

<210> 148
<211> 490
<212> PRT
<213> Homo sapiens

<400> 148
Met Thr Ile Gly Arg Met Glu Asn Val Glu Val Phe Thr Ala Glu Gly
1               5                   10                  15

Lys Gly Arg Gly Leu Lys Ala Thr Lys Glu Phe Trp Ala Ala Asp Ile
            20                  25                  30

Ile Phe Ala Asp Arg Ala Tyr Ser Ala Val Val Phe Asp Ser Leu Val
        35                  40                  45

Asn Phe Val Cys His Thr Cys Phe Lys Arg Gln Glu Lys Leu His Arg
    50                  55                  60

Cys Gly Gln Cys Lys Phe Ala His Tyr Cys Asp Arg Thr Cys Gln Lys
65                  70                  75                  80

Asp Ala Trp Leu Asn His Lys Asn Glu Cys Ser Ala Ile Lys Arg Tyr
                85                  90                  95

Gly Lys Val Pro Asn Glu Asn Ile Arg Leu Ala Ala Arg Ile Met Trp
            100                 105                 110

Arg Val Glu Arg Glu Gly Thr Gly Leu Thr Glu Gly Cys Leu Val Ser
        115                 120                 125

Val Asp Asp Leu Gln Asn His Val Glu His Phe Gly Glu Glu Glu Gln
    130                 135                 140

Lys Asp Leu Arg Val Asp Val Asp Thr Phe Leu Gln Tyr Trp Pro Pro

145                 150                 155                 160

Gln Ser Gln Gln Phe Ser Met Gln Tyr Ile Ser His Ile Phe Gly Val
                165                 170                 175

Ile Asn Cys Asn Gly Phe Thr Leu Ser Asp Gln Arg Gly Leu Gln Ala
            180                 185                 190

Val Gly Val Gly Ile Phe Pro Asn Leu Gly Leu Val Asn His Asp Cys
        195                 200                 205

Trp Pro Asn Cys Thr Val Ile Phe Asn Asn Gly Asn His Glu Ala Val
    210                 215                 220

Lys Ser Met Phe His Thr Gln Met Arg Ile Glu Leu Arg Ala Leu Gly
225                 230                 235                 240

Lys Ile Ser Glu Gly Glu Glu Leu Thr Val Ser Tyr Ile Asp Phe Leu
                245                 250                 255

Asn Val Ser Glu Glu Arg Lys Arg Gln Leu Lys Lys Gln Tyr Tyr Phe
            260                 265                 270

Asp Cys Thr Cys Glu His Cys Gln Lys Lys Leu Lys Asp Asp Leu Phe
        275                 280                 285

Leu Gly Val Lys Asp Asn Pro Lys Pro Ser Gln Glu Val Val Lys Glu
    290                 295                 300

Met Ile Gln Phe Ser Lys Asp Thr Leu Glu Lys Ile Asp Lys Ala Arg
305                 310                 315                 320

Ser Glu Gly Leu Tyr His Glu Val Val Lys Leu Cys Arg Glu Cys Leu
                325                 330                 335

Glu Lys Gln Glu Pro Val Phe Ala Asp Thr Asn Ile Tyr Met Leu Arg
            340                 345                 350

Met Leu Ser Ile Val Ser Glu Val Leu Ser Tyr Leu Gln Ala Phe Glu
        355                 360                 365

Glu Ala Ser Phe Tyr Ala Arg Arg Met Val Asp Gly Tyr Met Lys Leu
    370                 375                 380

Tyr His Pro Asn Asn Ala Gln Leu Gly Met Val Val Met Arg Ala Gly
385                 390                 395                 400

Leu Thr Asn Trp His Ala Gly Asn Ile Glu Val Gly His Gly Met Ile
                405                 410                 415

Cys Lys Ala Tyr Ala Ile Leu Leu Val Thr His Gly Pro Ser His Pro
            420                 425                 430

Ile Thr Lys Asp Leu Glu Ala Met Arg Val Gln Thr Glu Met Glu Leu
        435                 440                 445

Arg Met Phe Arg Gln Asn Glu Phe Met Tyr Tyr Lys Met Arg Glu Ala
    450                 455                 460

Ala Leu Asn Asn Gln Pro Met Gln Val Met Ala Glu Pro Ser Asn Glu
465                 470                 475                 480

Pro Ser Pro Ala Leu Phe His Lys Lys Gln
                485                 490

<210> 149
<211> 3647
<212> DNA

<213> Homo sapiens

<400> 149
   gacctacaga gagacttaga ctcctacaca atagtagtgg gagactttaa caccccactg 60
   tcaacattag acagattgag acagaaaatt aacaaggata ttcagtactt gaactcagct 120
   ctggaccaag cagacctaat agacatctac agaactctcc actccaaatc aacagaatat 180
   gcatttttct cagcactaca tcacacttat tctaaaattc accacataat tggaagtaaa 240
   acactcctta gcaaatgcaa aagaatggaa atcataacaa acagtctctc agaccacagt 300
   gcaatcaaat tagaactcag gattaagaaa ctcactcaaa accacacaac tacatggaaa 360
   ctgaaaaacc tgctcctgaa taactacttg gtaaataatg aaattaaggc agaaataaat 420
   aagttctgtg aaaccaatga gaacaaagac acaacgtacc agaatttctg ggacacagct 480
   aaagcagtgg ttagagggaa atttatagca ctaaatgcgc acaggagaaa gcaagaaaga 540
   tgtaaaatca acaccctaac atcacaatta aaagaactag agaagcaaga gcaaacaaat 600
   tcaaaagcta acagaagaca agaaataact aagatcatag cagaactgaa ggagataaag 660
   acacgaaaaa cccatcaaaa aatcaatgaa tctgggagct ggtttttgaa aaagattaac 720
   aaaatagata gacaactagc cagactaata aagaagagaa gagagaagaa tcaaatagat 780
   gcaataaaaa atgataaagg ggatatcact gctgatccca cagaaataca aactaccatc 840
   agagaatact ataaacacct ctatgcaaat aaactagaaa atctagaaga aatggataaa 900
   ttcctggcca catgcaccct cccaagacta aaccaggaag agttagaatc cctgaataga 960
   caaataacaa gttctgaaat taaggcagta attaatagcc taccaaccaa acaaaagccc 1020
   ggaccagatg gattcacagc tgaattctac cagaggtaca aagaggagct ggtaccattc 1080
   cttctgaaac tattccaaac aacagaaaaa gagggactcc ttcctaactc attttatgag 1140
   gcaagcatca tgctgatgcc aaaatctggc agagacacaa caaaaaaaga aaatttcagg 1200
   cctatatccc tgatgaacat cgatgtgaaa atcctcaata aaatactggc aaaccaaatc 1260
   ttgcagcaca tcaaaaagct tatccacgat gatcaagttg gcttcatccc tgggatgcaa 1320
   ggctggttca acatatgcaa atcaatcaac ataatccatc acataaatag caccaatgac 1380
   aaaaaccaca tgattatctc aatagatgca gaaaaggcct ttggtaaaat tcaacacccc 1440
   ttcatgctaa aaactctaaa taagctaggt attgatggaa cgtatctcaa aataataaga 1500
   gctgtttatg acaaacccac agccaatatc atactgactg ggcaaaagct ggaagcattc 1560
   cctttgaaaa ccagcacaag acaagtatgc cctctctcac cactcctatt caacatggta 1620
   ttggaagttc tggctagggc aatcaggcaa gagaaagaaa taaagcatat ccaaatagga 1680
   agagaggaag tcaaattgtc cctgtttgca gatgacatga ttgtatattt agaaaacccc 1740
   atcgtctcag cccaaaatct ccttaagctg ataagaaact tcagcaaagt ctcgggatac 1800
   aaaatcaatg tgcaaaaatc acaagcattc ctatacatca ataatagaca aacagagagc 1860
   caaatcgtga gtgaactccc attcacaatt gttacaaaga gaatacaata cctaggaata 1920
   caacttacaa gggatgtgaa ggacctcttc aaggagaact acaaaccact gctcaaggaa 1980
   ataagagagg acacaaacaa atggaaaaac attctatgct catggatagg aagaatcaat 2040
   atcgtgaaaa tgaccatgct gcccaaagta atttatagat tcaacactat gcccatcaag 2100
   ctaccattga ctttcttcac ggaatcagac aaaactactt taaatttcat atggaaccaa 2160
   aaaagagcct gcacagccaa gacaatccta agcaaaaaga acaaagctgg aggcatcaca 2220
   ctacctaact tcaaactata ctacaaggct acagtgacca aaacagcatg gtactggtac 2280
   caaaacagat atacagacca atggaacaga atagaggcct cagaaataac accacacatc 2340
   tacaaccacc tgatctttga caaacctgac acaaacaagc aatggggaaa aggatctcta 2400
   tttaataaat ggtgttggga aaactggcta gccatatgca gaaaactgaa actggacccc 2460
   ttccttacac tttatacaaa aattaattca agctggatta aagacttaaa tgtaagacct 2520
   aaaacaataa aaatcctaga agaaaacctg ggcaatacca ttcaggacat aggcatgggc 2580
   aaagacttcg tgactgtaac accaaaagca atggcaacaa aagccaaaat tgacaaatgg 2640
   gatctaatta aactaaagag cttctgcaca gcaaaagaaa ctgtcatcag ggtgaacagg 2700
   caacctacag aatgggaaaa atttttgcaa atctgtccat ctgacaaagg gctaatatcc 2760
   agaatctaca aggaacttaa acaaatttac aagaaaaaaa caaacaaccc tatcaaaaag 2820
   tgggcaaagg ctatgaacag acacttctca aaagaagaca tttatgcagc caaaagacat 2880
   atgaaaaaat ggtcatcatc actggtcttc agggaaatgc aaatcaaaac cacaatgaga 2940
   taccatctca tgccagttag aatggtgatc attagaaagt caggaaacaa cacatgcatg 3000
   caaatcaaaa ccacaatgag ataccatctc atgccagtta gaatggtgat cattagaaag 3060
   tcaggaaaca acacatgcag aggatgtgga gaaataggaa tgcttttaca ctgttggtgg 3120
   gagtgtaaac tagttcaacc attgtggaag acagtgtggc gattcctcaa ggatctagaa 3180
   ccagaaatac cattagaccc agcaatccca ttactgggta tatacccaaa tgattataaa 3240
   tcatgctact ataaagacac atgcacacgt atgtttattg cggcactatt cacaatagca 3300
   aagacttgga accaacccaa atgcccatca gtgagagtca taaagaaaat gtggcacata 3360
   tacatcatgg aatactatgc agccataaaa aaggatgagt tcatgtcctt tgcagggaca 3420
   tggatgaatc tggaaaccac cattctcagc aaactaacac aggaacagaa aaccaaatac 3480
   cgcttgttct cactcgtaag ttggagttga acaatgagaa cacatggaca cagggagggg 3540
   aacaacacca gggcctgtca gggggtaggg gggatagggg agggatagca ttaagagaaa 3600
   tacctaatgt agatgacggg ttgatgggtg cagcaaacca ccaaggc 3647

<210> 150
<211> 1081
<212> PRT

<213> Homo sapiens

<400> 150
Met Glu Ile Ile Thr Asn Ser Leu Ser Asp His Ser Ala Ile Lys Leu
1               5                   10                  15

Glu Leu Arg Ile Lys Lys Leu Thr Gln Asn His Thr Thr Thr Trp Lys
            20                  25                  30

Leu Lys Asn Leu Leu Leu Asn Asn Tyr Leu Val Asn Asn Glu Ile Lys
        35                  40                  45

Ala Glu Ile Asn Lys Phe Cys Glu Thr Asn Glu Asn Lys Asp Thr Thr
    50                  55                  60

Tyr Gln Asn Phe Trp Asp Thr Ala Lys Ala Val Val Arg Gly Lys Phe
65                  70                  75                  80

Ile Ala Leu Asn Ala His Arg Arg Lys Gln Glu Arg Cys Lys Ile Asn
                85                  90                  95

Thr Leu Thr Ser Gln Leu Lys Glu Leu Glu Lys Gln Glu Gln Thr Asn
            100                 105                 110

Ser Lys Ala Asn Arg Arg Gln Glu Ile Thr Lys Ile Ile Ala Glu Leu
        115                 120                 125

Lys Glu Ile Lys Thr Arg Lys Thr His Gln Lys Ile Asn Glu Ser Gly
    130                 135                 140

Ser Trp Phe Phe Glu Lys Ile Asn Lys Ile Asp Arg Gln Leu Ala Arg
145                 150                 155                 160

Leu Ile Lys Lys Arg Arg Glu Lys Asn Gln Ile Asp Ala Ile Lys Asn
                165                 170                 175

Asp Lys Gly Asp Ile Thr Ala Asp Pro Thr Glu Ile Gln Thr Thr Ile
            180                 185                 190

Arg Glu Tyr Tyr Lys His Leu Tyr Ala Asn Lys Leu Glu Asn Leu Glu
        195                 200                 205

Glu Met Asp Lys Phe Leu Ala Thr Cys Thr Leu Pro Arg Leu Asn Gln
    210                 215                 220

Glu Glu Leu Glu Ser Leu Asn Arg Gln Ile Thr Ser Ser Glu Ile Lys
225                 230                 235                 240

Ala Val Ile Asn Ser Leu Pro Thr Lys Gln Lys Pro Gly Pro Asp Gly
                245                 250                 255

Phe Thr Ala Glu Phe Tyr Gln Arg Tyr Lys Glu Glu Leu Val Pro Phe
            260                 265                 270

Leu Leu Lys Leu Phe Gln Thr Thr Glu Lys Glu Gly Leu Leu Pro Asn
        275                 280                 285

Ser Phe Tyr Glu Ala Ser Ile Met Leu Met Pro Lys Ser Gly Arg Asp
    290                 295                 300

Thr Thr Lys Lys Glu Asn Phe Arg Pro Ile Ser Leu Met Asn Ile Asp
305                 310                 315                 320

Val Lys Ile Leu Asn Lys Ile Leu Ala Asn Gln Ile Leu Gln His Ile
                325                 330                 335

Lys Lys Leu Ile His Asp Asp Gln Val Gly Phe Ile Pro Gly Met Gln
            340                 345                 350

Gly Trp Phe Asn Ile Cys Lys Ser Ile Asn Ile Ile His His Ile Asn
        355                 360                 365

Ser Thr Asn Asp Lys Asn His Met Ile Ile Ser Ile Asp Ala Glu Lys
    370                 375                 380

Ala Phe Gly Lys Ile Gln His Pro Phe Met Leu Lys Thr Leu Asn Lys
385                 390                 395                 400

Leu Gly Ile Asp Gly Thr Tyr Leu Lys Ile Ile Arg Ala Val Tyr Asp
                405                 410                 415

Lys Pro Thr Ala Asn Ile Ile Leu Thr Gly Gln Lys Leu Glu Ala Phe
            420                 425                 430

Pro Leu Lys Thr Ser Thr Arg Gln Val Cys Pro Leu Ser Pro Leu Leu
        435                 440                 445

Phe Asn Met Val Leu Glu Val Leu Ala Arg Ala Ile Arg Gln Glu Lys
    450                 455                 460

Glu Ile Lys His Ile Gln Ile Gly Arg Glu Glu Val Lys Leu Ser Leu
465                 470                 475                 480

Phe Ala Asp Asp Met Ile Val Tyr Leu Glu Asn Pro Ile Val Ser Ala
                485                 490                 495

Gln Asn Leu Leu Lys Leu Ile Arg Asn Phe Ser Lys Val Ser Gly Tyr
            500                 505                 510

Lys Ile Asn Val Gln Lys Ser Gln Ala Phe Leu Tyr Ile Asn Asn Arg
        515                 520                 525

Gln Thr Glu Ser Gln Ile Val Ser Glu Leu Pro Phe Thr Ile Val Thr
    530                 535                 540

Lys Arg Ile Gln Tyr Leu Gly Ile Gln Leu Thr Arg Asp Val Lys Asp
545                 550                 555                 560

Leu Phe Lys Glu Asn Tyr Lys Pro Leu Leu Lys Glu Ile Arg Glu Asp
                565                 570                 575

Thr Asn Lys Trp Lys Asn Ile Leu Cys Ser Trp Ile Gly Arg Ile Asn
            580                 585                 590

Ile Val Lys Met Thr Met Leu Pro Lys Val Ile Tyr Arg Phe Asn Thr
        595                 600                 605

Met Pro Ile Lys Leu Pro Leu Thr Phe Phe Thr Glu Ser Asp Lys Thr
    610                 615                 620

Thr Leu Asn Phe Ile Trp Asn Gln Lys Arg Ala Cys Thr Ala Lys Thr
625                 630                 635                 640

Ile Leu Ser Lys Lys Asn Lys Ala Gly Gly Ile Thr Leu Pro Asn Phe
                645                 650                 655

Lys Leu Tyr Tyr Lys Ala Thr Val Thr Lys Thr Ala Trp Tyr Trp Tyr
            660                 665                 670

Gln Asn Arg Tyr Thr Asp Gln Trp Asn Arg Ile Glu Ala Ser Glu Ile
        675                 680                 685

Thr Pro His Ile Tyr Asn His Leu Ile Phe Asp Lys Pro Asp Thr Asn
    690                 695                 700

Lys Gln Trp Gly Lys Gly Ser Leu Phe Asn Lys Trp Cys Trp Glu Asn
705                 710                 715                 720

Trp Leu Ala Ile Cys Arg Lys Leu Lys Leu Asp Pro Phe Leu Thr Leu
                725                 730                 735

Tyr Thr Lys Ile Asn Ser Ser Trp Ile Lys Asp Leu Asn Val Arg Pro
            740                 745                 750

Lys Thr Ile Lys Ile Leu Glu Glu Asn Leu Gly Asn Thr Ile Gln Asp
        755                 760                 765

Ile Gly Met Gly Lys Asp Phe Val Thr Val Thr Pro Lys Ala Met Ala
    770                 775                 780

Thr Lys Ala Lys Ile Asp Lys Trp Asp Leu Ile Lys Leu Lys Ser Phe
785                 790                 795                 800

Cys Thr Ala Lys Glu Thr Val Ile Arg Val Asn Arg Gln Pro Thr Glu
                805                 810                 815

Trp Glu Lys Phe Phe Ala Ile Cys Pro Ser Asp Lys Gly Leu Ile Ser
            820                 825                 830

Arg Ile Tyr Lys Glu Leu Lys Gln Ile Tyr Lys Lys Lys Thr Asn Asn
        835                 840                 845

Pro Ile Lys Lys Trp Ala Lys Ala Met Asn Arg His Phe Ser Lys Glu
    850                 855                 860

Asp Ile Tyr Ala Ala Lys Arg His Met Lys Lys Trp Ser Ser Ser Leu
865                 870                 875                 880

Val Phe Arg Glu Met Gln Ile Lys Thr Thr Met Arg Tyr His Leu Met
                885                 890                 895

Pro Val Arg Met Val Ile Ile Arg Lys Ser Gly Asn Asn Thr Cys Met
            900                 905                 910

Gln Ile Lys Thr Thr Met Arg Tyr His Leu Met Pro Val Arg Met Val
        915                 920                 925

Ile Ile Arg Lys Ser Gly Asn Asn Thr Cys Arg Gly Cys Gly Glu Ile
    930                 935                 940

Gly Met Leu Leu His Cys Trp Trp Glu Cys Lys Leu Val Gln Pro Leu
945                 950                 955                 960

Trp Lys Thr Val Trp Arg Phe Leu Lys Asp Leu Glu Pro Glu Ile Pro
                965                 970                 975

Leu Asp Pro Ala Ile Pro Leu Leu Gly Ile Tyr Pro Asn Asp Tyr Lys
            980                 985                 990

Ser Cys Tyr Tyr Lys Asp Thr Cys Thr Arg Met Phe Ile Ala Ala Leu
        995                 1000                1005

Phe Thr Ile Ala Lys Thr Trp Asn Gln Pro Lys Cys Pro Ser Val Arg
    1010                1015                1020

Val Ile Lys Lys Met Trp His Ile Tyr Ile Met Glu Tyr Tyr Ala Ala
1025                1030                1035                1040

Ile Lys Lys Asp Glu Phe Met Ser Phe Ala Gly Thr Trp Met Asn Leu
                1045                1050                1055

Glu Thr Thr Ile Leu Ser Lys Leu Thr Gln Glu Gln Lys Thr Lys Tyr
            1060                1065                1070

Arg Leu Phe Ser Leu Val Ser Trp Ser
        1075                1080

<210> 151
<211> 3081
<212> DNA
<213> Homo sapiens

<400> 151
aaatgtaaaa gaactgtctc tcagaccatg gtgcaatcaa aactagaact cagcattaag 60
aaactcactc aaaactgccc aactacatgg aaactgaaca acctgctcct gaatgactac 120
tgggtacata acaaaatcaa ggcagaaata aagatgttct ttgaaaccaa caagaacaaa 180
gacacaacat accagaatct ctgggacaca ttcaaagcag tgtgtagagg gaaatttata 240
gcactaaata cccataagag aaagcaggaa agatctaaaa ttgacaccct aacatcacaa 300
ttaaaacaac tacagaagca agagcaaaca cattcaaaag ctagcagaag gcaagaaata 360
actaagatca gagcagaact gaaggagata gagacacaaa aaaatgcttc aaaaaaaaat 420
gaacttaaaa aagataaagg ggtttggtcc accgatccca gagaaaaaca cactaccatc 480
agagaatact ataaacacct gtatgcaaat aaactagaaa atctggaaga aatggataaa 540
tttctggaca aatacacctt cccaagacta aaccaggaag aagttgaatc cctgaataga 600
ccaataacag gctcggaaat tgaggcaata attaatagct taccaaccaa aaaaagtcca 660
gggtcagatg gattcacagc cgaattctac cagaggtaca aggaggagct ggtaccattc 720
cttctgaaac tattccaatc aatagaaaaa gagggaatcc tccctaactc atttgatgag 780
gccagcatca tcctgatacc aaagcctagc agagacacaa caaaaaaaga gaattttaga 840
ccaatatccc tgatgaacat cgatgcaaaa atcctcaata aaatactggc aaaacgaatc 900
cagcagcaca tcaaaaagtt tatccaccac gatcaagtgg gcttcatccc taggatgcaa 960
ggctggttta acatatgcaa atcaataaac gtaatccagc atataaatag aaccaaagac 1020
aaaaaccaca tgattatctc aatagatgca gaaaaggcct ttgacaaaat tcaacagccc 1080
ttcatgctaa aaactctcag taaattaggt attgatatga catatctcaa aataataaga 1140
gctatctatg acaaacccac agccaatatc atactgaatg ggcaaaaact ggaagcattc 1200
cctttgaaaa ctggcacaag acatgggtgc cctctctcac cactcctatt caacatagtg 1260
ttggaagtcc tggccagggc aatcaggcag gagaaggaaa taaagggtat tcaattagga 1320
aaagaggaag tcaaattgtc cctgtttgca gatgacatga ttttatatct agaaaacccc 1380
atcgtctcag cccaaaatct ccttaagctg ataagcaact tcagcaaagt cccaggatac 1440
aaaatcaatg tgcaaaaatc acaagcattc ttatacacca ataacagaca gacagagagc 1500
caaatcatga gtgaactccc atttacaatt gcttcaaaga gaataaaata cctaggaatc 1560
caacttacaa gggatgtgaa ggactcttca aggagaacta caaacccatg ctcaattgaa 1620
ataaaagagg atacaaacaa atggaagaac attccatgct catgggtaag aagaatcaat 1680
attgtgaaaa tggccattct gcccaaggta atttataggt tcaatgccat ccccatcaag 1740
ctaccaatgg ctttcttcac agaattggaa aaaactactt taaagttcat atggaaccaa 1800
aaaagagcct gcattgctaa gcctgcattg ctaagccaaa agaacaaagc tggaggcatc 1860
atgctacctg acttcaaact atactacaag gccacagtaa ccaaaacagc atggtactgg 1920
taccaaaaca gatatataga ccaatggaac aaagcagagc cctcagaaat aatgccacac 1980
atctataact atctgatctt tgacaaacct gacaaaaaca agaaatcggg aaaggattcc 2040
gtatttaata aacggtcctg ggaaaactgg ctagccatat gtagaaagct gaaactggac 2100
cccttcctta cacctcatac aaaaattaat tcaagatgga ttaaagactt aaatgttaga 2160
cctaaaacca taaaaaccct agaagaaaac ctaggcaata ccattcagga cataggcatg 2220
ggcaaggact tcatgtctaa aacaccaaaa gcaatggcaa caaaagacaa aattgacaaa 2280
tgggatctaa ttaaactaaa gagcttctgc acagcaatag aaactaccat cagagtgaac 2340
aggcaaccta cagaatggga gaaaattttt gcaacctact catctgacaa agggctaata 2400
tccagaatcc acaatgaact caaacaaatt tacaagaaaa aatcaaacaa ccccatcaaa 2460
aagtgggcaa aggatatgaa cagacacttc tcaaaagaag acatttatgc agccaaaaga 2520
cacatgaaaa aatgctcatc atcactggcc atcagagaaa tgcaaatgaa aaccacaatg 2580
agataccatc tcacaccagt tagaatggcg atcattaaaa agtcaggaaa caacaggtgc 2640
tggagaggat gtggagaaat aggaacactt ttacgctgtt ggtgggactg taaactagtt 2700
caaccattgt ggaagtcagt gtggcgattc ctcagggatc tagaactaga aataccattt 2760
gacccagcca tcccattact gggtatatac ccaaaggact ataaatcatg ctgctataaa 2820
gacacatgca gccgtatgtt tgttgcagca ctattcacaa cagcaaagac ttggaaccaa 2880
cccaaatgtc caacaatgat agactggatt aagaaaatgt ggcacatata caccatggaa 2940
tactatgcag ccacaaaaaa aaaggatgag ttcatgtcct ttgcagggac atggatgaag 3000
ctggaaacca tcattctcag caaactatca caaggacaga aaaccaaaca ctgcatgttc 3060
tcactcatag gtgggaatta g 3081

<210> 152
<211> 1017
<212> PRT
<213> Homo sapiens

<400> 152
Met Val Gln Ser Lys Leu Glu Leu Ser Ile Lys Lys Leu Thr Gln Asn
1               5                   10                  15

Cys Pro Thr Thr Trp Lys Leu Asn Asn Leu Leu Leu Asn Asp Tyr Trp
            20                  25                  30

Val His Asn Lys Ile Lys Ala Glu Ile Lys Met Phe Phe Glu Thr Asn
        35                  40                  45

Lys Asn Lys Asp Thr Thr Tyr Gln Asn Leu Trp Asp Thr Phe Lys Ala
    50                  55                  60

Val Cys Arg Gly Lys Phe Ile Ala Leu Asn Thr His Lys Arg Lys Gln
65                  70                  75                  80

Glu Arg Ser Lys Ile Asp Thr Leu Thr Ser Gln Leu Lys Gln Leu Gln
                85                  90                  95

Lys Gln Glu Gln Thr His Ser Lys Ala Ser Arg Arg Gln Glu Ile Thr
            100                 105                 110

Lys Ile Arg Ala Glu Leu Lys Glu Ile Glu Thr Gln Lys Asn Ala Ser
        115                 120                 125

Lys Lys Asn Glu Leu Lys Lys Asp Lys Gly Val Trp Ser Thr Asp Pro
    130                 135                 140

Arg Glu Lys His Thr Thr Ile Arg Glu Tyr Tyr Lys His Leu Tyr Ala
145                 150                 155                 160

Asn Lys Leu Glu Asn Leu Glu Glu Met Asp Lys Phe Leu Asp Lys Tyr
                165                 170                 175

Thr Phe Pro Arg Leu Asn Gln Glu Glu Val Glu Ser Leu Asn Arg Pro
            180                 185                 190

Ile Thr Gly Ser Glu Ile Glu Ala Ile Ile Asn Ser Leu Pro Thr Lys
        195                 200                 205

Lys Ser Pro Gly Ser Asp Gly Phe Thr Ala Glu Phe Tyr Gln Arg Tyr
    210                 215                 220

Lys Glu Glu Leu Val Pro Phe Leu Leu Lys Leu Phe Gln Ser Ile Glu
225                 230                 235                 240

Lys Glu Gly Ile Leu Pro Asn Ser Phe Asp Glu Ala Ser Ile Ile Leu
                245                 250                 255

Ile Pro Lys Pro Ser Arg Asp Thr Thr Lys Lys Glu Asn Phe Arg Pro
            260                 265                 270

Ile Ser Leu Met Asn Ile Asp Ala Lys Ile Leu Asn Lys Ile Leu Ala
        275                 280                 285

Lys Arg Ile Gln Gln His Ile Lys Lys Phe Ile His His Asp Gln Val
    290                 295                 300

Gly Phe Ile Pro Arg Met Gln Gly Trp Phe Asn Ile Cys Lys Ser Ile
305                 310                 315                 320

Asn Val Ile Gln His Ile Asn Arg Thr Lys Asp Lys Asn His Met Ile
                325                 330                 335

Ile Ser Ile Asp Ala Glu Lys Ala Phe Asp Lys Ile Gln Gln Pro Phe
            340                 345                 350

Met Leu Lys Thr Leu Ser Lys Leu Gly Ile Asp Met Thr Tyr Leu Lys
        355                 360                 365

Ile Ile Arg Ala Ile Tyr Asp Lys Pro Thr Ala Asn Ile Ile Leu Asn
    370                 375                 380

Gly Gln Lys Leu Glu Ala Phe Pro Leu Lys Thr Gly Thr Arg His Gly
385                 390                 395                 400

Cys Pro Leu Ser Pro Leu Leu Phe Asn Ile Val Leu Glu Val Leu Ala
                405                 410                 415

Arg Ala Ile Arg Gln Glu Lys Glu Ile Lys Gly Ile Gln Leu Gly Lys
            420                 425                 430

Glu Glu Val Lys Leu Ser Leu Phe Ala Asp Asp Met Ile Leu Tyr Leu
        435                 440                 445

Glu Asn Pro Ile Val Ser Ala Gln Asn Leu Leu Lys Leu Ile Ser Asn
    450                 455                 460

Phe Ser Lys Val Pro Gly Tyr Lys Ile Asn Val Gln Lys Ser Gln Ala
465                 470                 475                 480

Phe Leu Tyr Thr Asn Asn Arg Gln Thr Glu Ser Gln Ile Met Ser Glu
                485                 490                 495

Leu Pro Phe Thr Ile Ala Ser Lys Arg Ile Lys Tyr Leu Gly Ile Gln
            500                 505                 510

Leu Thr Arg Asp Val Lys Asp Ser Ser Arg Arg Thr Thr Asn Pro Cys
        515                 520                 525

Ser Ile Glu Ile Lys Glu Asp Thr Asn Lys Trp Lys Asn Ile Pro Cys
    530                 535                 540

Ser Trp Val Arg Arg Ile Asn Ile Val Lys Met Ala Ile Leu Pro Lys
545                 550                 555                 560

Val Ile Tyr Arg Phe Asn Ala Ile Pro Ile Lys Leu Pro Met Ala Phe
                565                 570                 575

Phe Thr Glu Leu Glu Lys Thr Thr Leu Lys Phe Ile Trp Asn Gln Lys
            580                 585                 590

Arg Ala Cys Ile Ala Lys Pro Ala Leu Leu Ser Gln Lys Asn Lys Ala
        595                 600                 605

Gly Gly Ile Met Leu Pro Asp Phe Lys Leu Tyr Tyr Lys Ala Thr Val
    610                 615                 620

Thr Lys Thr Ala Trp Tyr Trp Tyr Gln Asn Arg Tyr Ile Asp Gln Trp
625                 630                 635                 640

Asn Lys Ala Glu Pro Ser Glu Ile Met Pro His Ile Tyr Asn Tyr Leu
                645                 650                 655

Ile Phe Asp Lys Pro Asp Lys Asn Lys Lys Ser Gly Lys Asp Ser Val
            660                 665                 670

Phe Asn Lys Arg Ser Trp Glu Asn Trp Leu Ala Ile Cys Arg Lys Leu
        675                 680                 685

Lys Leu Asp Pro Phe Leu Thr Pro His Thr Lys Ile Asn Ser Arg Trp
    690                 695                 700

Ile Lys Asp Leu Asn Val Arg Pro Lys Thr Ile Lys Thr Leu Glu Glu
705                 710                 715                 720

Asn Leu Gly Asn Thr Ile Gln Asp Ile Gly Met Gly Lys Asp Phe Met
                725                 730                 735

Ser Lys Thr Pro Lys Ala Met Ala Thr Lys Asp Lys Ile Asp Lys Trp
            740                 745                 750

Asp Leu Ile Lys Leu Lys Ser Phe Cys Thr Ala Ile Glu Thr Thr Ile
        755                 760                 765

Arg Val Asn Arg Gln Pro Thr Glu Trp Glu Lys Ile Phe Ala Thr Tyr
    770                 775                 780

Ser Ser Asp Lys Gly Leu Ile Ser Arg Ile His Asn Glu Leu Lys Gln
785                 790                 795                 800

Ile Tyr Lys Lys Lys Ser Asn Asn Pro Ile Lys Lys Trp Ala Lys Asp
                805                 810                 815

Met Asn Arg His Phe Ser Lys Glu Asp Ile Tyr Ala Ala Lys Arg His
            820                 825                 830

Met Lys Lys Cys Ser Ser Ser Leu Ala Ile Arg Glu Met Gln Met Lys
        835                 840                 845

Thr Thr Met Arg Tyr His Leu Thr Pro Val Arg Met Ala Ile Ile Lys
    850                 855                 860

Lys Ser Gly Asn Asn Arg Cys Trp Arg Gly Cys Gly Glu Ile Gly Thr
865                 870                 875                 880

Leu Leu Arg Cys Trp Trp Asp Cys Lys Leu Val Gln Pro Leu Trp Lys
                885                 890                 895

Ser Val Trp Arg Phe Leu Arg Asp Leu Glu Leu Glu Ile Pro Phe Asp
            900                 905                 910

Pro Ala Ile Pro Leu Leu Gly Ile Tyr Pro Lys Asp Tyr Lys Ser Cys
        915                 920                 925

Cys Tyr Lys Asp Thr Cys Ser Arg Met Phe Val Ala Ala Leu Phe Thr
    930                 935                 940

Thr Ala Lys Thr Trp Asn Gln Pro Lys Cys Pro Thr Met Ile Asp Trp
945                 950                 955                 960

Ile Lys Lys Met Trp His Ile Tyr Thr Met Glu Tyr Tyr Ala Ala Thr
                965                 970                 975

Lys Lys Lys Asp Glu Phe Met Ser Phe Ala Gly Thr Trp Met Lys Leu
            980                 985                 990

Glu Thr Ile Ile Leu Ser Lys Leu Ser Gln Gly Gln Lys Thr Lys His
        995                 1000                1005

Cys Met Phe Ser Leu Ile Gly Gly Asn
    1010                1015


<210> 153
<211> 3021
<212> DNA
<213> Homo sapiens

<400> 153
aatgactact gagtaaataa tgaaatgaag gcagaaataa agatgttctt tgaaaccaat 60
gagaacaaag acacaatgta ccagaatctc tgggacacat ttaaagcagt gtgtagaggg 120
aaatttatag cactaaatgc ccacaagaga aagcaggaaa gatctaaaat caacatccta 180
acatcacagt taaaagaact agggaagcaa gaacaaacaa attcaaaagc tagcagaagg 240
caaaaaataa ctaagatcag agcagaactg aaggagatag agacacaaaa aacccttcaa 300
aaaatcaatg aatccaggag ctggtttttt gaaaagatca acaaaattga tagacaacta 360
gcaagaccaa taaagaagaa aagagagaag aatcaaatag atgcaacaaa aaatgataaa 420
ggggatatca ccactgatcc cacagaaata caaactacca tcagagaata ctatcaacac 480
ttctatgcaa atatactaga aaatctagaa gaaatggata aattcctgga cacatacact 540
ctcccaagac taaaccagga agaagttgaa tctctgtata gaccaataac aggttctgaa 600

attgaggcaa taattaatag gctaccaacc aaaaaaagtc caggaccaga tggattcaca 660
gctgaattct accagaggta caaagaggag ctggtaccat tccttctgaa actatttcag 720
acaacagaaa aagagggact cctccctaac tcattttatg aggccagcat catcctgaca 780
ccaaaacctg gtagagacac aacaaaaaaa gagaatttta tgccaatatc cctgatgaac 840
attgatgcga aagtcctcaa taaaatactg gcaaaagctt atccaccaca tcaaaagctt 900
atccaccacg gtcaacttgg cttcatccct gggatgcaag gctggttcaa catatgcaaa 960
tcaataaatg tagttcatca cataaacaga accaatgaca aaaaccacat gattatctca 1020
atagatgcag aaaaggcctt cgacaatatt caacaccact tcatgctaaa aactctgagt 1080
aaactaggta tcgatggaac atatctgaaa ataataagag ctatttatga caaacccaca 1140
gccaatatca tagtgaatgg gcaaaaactg gaagcattcc ctttgaaaac tggcacaaga 1200
caaggatgcc ctctctcacc actcctattc aacatagtgt tggaagttct ggctagggca 1260
atcaggcaag agaaagaaat aaacggtatt caattaggaa aagaggaagt caaattgtct 1320
ctgtgtgcag atgacatgat tgtatattta gaaaacccca tcgtctcagc ccaaaatctc 1380
cttaagctga taagcaactt cagcaaagtc tcaggataca aaatcaatgt gcaaaaatca 1440
caagcattcc tatacatcaa taatagacaa acagagagcc aaatcatgag tgaactccca 1500
ttcccaatta ccacaaagag aattaaatac ctaggaatcc aacttacaag ggatgtgaaa 1560
gacctcttca aggagaacta caaaccactg ctcgaaataa aagaggacac aaacaaatgg 1620
aaaaacattc catgctcatg gataggaaga atcaatattg tgaaaatggt catactgccc 1680
aaagtaattt atagattcaa tgctatcccc atcaagctac cactgacttt cttcacagaa 1740
ttggaaaaaa ctattttaaa gttcatatgg aaccaaaaaa gaacccagat tgccaagaca 1800
atcctaagca aaaagaacaa agctggaggc atcacactac ctgacttcaa actatactac 1860
aaggctacag taaacaaaac agcatggtac tggtaccaaa acagatatat agaccaatgg 1920
aacagaatgg aggcctcaga aataacacca cacatctaca accatctgat ctttgacaaa 1980
cctgacaaaa acaggcaatg gggaaaggat tctctattta ataaatggtg ctgggaaaac 2040
tggctagcca tatgtagaaa gctgaaactg gaccccttcc ttacacctta tacaaaaatt 2100
aacacaagat ggattaaaga cttaaacgtc agacctaata ccataaaaac cctagaagaa 2160
aacctaggca ataccattca ggacataggc atgggcaaag tcttcatgac taaaacacca 2220
aaagcaatgg caacaaaagt caaaattgac aaatgggatc taattaaact aaagagcttc 2280
tgcacagcaa aagaaactat catcagagtg aacaggcaac ctacagaatg ggagaaaatc 2340
tttgcaacct acccatctga caaagggcta atatccagaa tctacaaaga actcaaacaa 2400
atttacaaga aaaaaaaaac aaccccatca aaaagtgggc aaatacaaga aaaaaaaaac 2460
aaccccatca aaaagtgggc aaaggatatg agcagacact tctcaaaaga agacatttat 2520
gcagccaaca gaatgaaaaa gtggtcatca tcactggtct tcagggaaat gcaaatcaaa 2580
accacaatga gataccatct catgccagtt agaatggtga tcattaaaaa gtcaggaaac 2640
aacacatgcc tgagaggatg tggagaaata ggaatgcttt tacactgttg gtgggagtgt 2700
aaactagttc aaccattgtg gaagacagtg tggcgattcc tcaaggatct agaaccagaa 2760
ataccattag acccagcaat cccattactg ggtatatacc caaacaatta taaatcatgc 2820
tactataaag acacacgcac acgtatgttt attgtggcac tattcacgat agcaaagaag 2880
gatgagttca tgtcctttgc agggacatgg atgaagctgg aaaccatcat tctaagcaaa 2940
ctatcacaag gacagaaaac caaacaccac atgttctcac tcataggtgg gagttgaaca 3000
acgagaacac atggacacag g 3021

<210> 154
<211> 990
<212> PRT
<213> Homo sapiens

<400> 154
Met Lys Ala Glu Ile Lys Met Phe Phe Glu Thr Asn Glu Asn Lys Asp
1               5                   10                  15

Thr Met Tyr Gln Asn Leu Trp Asp Thr Phe Lys Ala Val Cys Arg Gly
            20                  25                  30

Lys Phe Ile Ala Leu Asn Ala His Lys Arg Lys Gln Glu Arg Ser Lys
        35                  40                  45

Ile Asn Ile Leu Thr Ser Gln Leu Lys Glu Leu Gly Lys Gln Glu Gln
    50                  55                  60

Thr Asn Ser Lys Ala Ser Arg Arg Gln Lys Ile Thr Lys Ile Arg Ala
65                  70                  75                  80

Glu Leu Lys Glu Ile Glu Thr Gln Lys Thr Leu Gln Lys Ile Asn Glu
                85                  90                  95

Ser Arg Ser Trp Phe Phe Glu Lys Ile Asn Lys Ile Asp Arg Gln Leu
            100                 105                 110

Ala Arg Pro Ile Lys Lys Lys Arg Glu Lys Asn Gln Ile Asp Ala Thr
        115                 120                 125

Lys Asn Asp Lys Gly Asp Ile Thr Thr Asp Pro Thr Glu Ile Gln Thr
    130                 135                 140

Thr Ile Arg Glu Tyr Tyr Gln His Phe Tyr Ala Asn Ile Leu Glu Asn
145                 150                 155                 160

Leu Glu Glu Met Asp Lys Phe Leu Asp Thr Tyr Thr Leu Pro Arg Leu
                165                 170                 175

Asn Gln Glu Glu Val Glu Ser Leu Tyr Arg Pro Ile Thr Gly Ser Glu
            180                 185                 190

Ile Glu Ala Ile Ile Asn Arg Leu Pro Thr Lys Lys Ser Pro Gly Pro
        195                 200                 205

Asp Gly Phe Thr Ala Glu Phe Tyr Gln Arg Tyr Lys Glu Glu Leu Val
    210                 215                 220

Pro Phe Leu Leu Lys Leu Phe Gln Thr Thr Glu Lys Glu Gly Leu Leu
225                 230                 235                 240

Pro Asn Ser Phe Tyr Glu Ala Ser Ile Ile Leu Thr Pro Lys Pro Gly
                245                 250                 255

Arg Asp Thr Thr Lys Lys Glu Asn Phe Met Pro Ile Ser Leu Met Asn
            260                 265                 270

Ile Asp Ala Lys Val Leu Asn Lys Ile Leu Ala Lys Ala Tyr Pro Pro
        275                 280                 285

His Gln Lys Leu Ile His His Gly Gln Leu Gly Phe Ile Pro Gly Met
    290                 295                 300

Gln Gly Trp Phe Asn Ile Cys Lys Ser Ile Asn Val Val His His Ile
305                 310                 315                 320

Asn Arg Thr Asn Asp Lys Asn His Met Ile Ile Ser Ile Asp Ala Glu
                325                 330                 335

Lys Ala Phe Asp Asn Ile Gln His His Phe Met Leu Lys Thr Leu Ser
            340                 345                 350

Lys Leu Gly Ile Asp Gly Thr Tyr Leu Lys Ile Ile Arg Ala Ile Tyr
        355                 360                 365

Asp Lys Pro Thr Ala Asn Ile Ile Val Asn Gly Gln Lys Leu Glu Ala
    370                 375                 380

Phe Pro Leu Lys Thr Gly Thr Arg Gln Gly Cys Pro Leu Ser Pro Leu
385                 390                 395                 400

Leu Phe Asn Ile Val Leu Glu Val Leu Ala Arg Ala Ile Arg Gln Glu
                405                 410                 415

Lys Glu Ile Asn Gly Ile Gln Leu Gly Lys Glu Glu Val Lys Leu Ser
            420                 425                 430

Leu Cys Ala Asp Asp Met Ile Val Tyr Leu Glu Asn Pro Ile Val Ser
        435                 440                 445

Ala Gln Asn Leu Leu Lys Leu Ile Ser Asn Phe Ser Lys Val Ser Gly
    450                 455                 460

Tyr Lys Ile Asn Val Gln Lys Ser Gln Ala Phe Leu Tyr Ile Asn Asn
465                 470                 475                 480

Arg Gln Thr Glu Ser Gln Ile Met Ser Glu Leu Pro Phe Pro Ile Thr
                485                 490                 495

Thr Lys Arg Ile Lys Tyr Leu Gly Ile Gln Leu Thr Arg Asp Val Lys
            500                 505                 510

Asp Leu Phe Lys Glu Asn Tyr Lys Pro Leu Leu Glu Ile Lys Glu Asp
        515                 520                 525

Thr Asn Lys Trp Lys Asn Ile Pro Cys Ser Trp Ile Gly Arg Ile Asn
    530                 535                 540

Ile Val Lys Met Val Ile Leu Pro Lys Val Ile Tyr Arg Phe Asn Ala
545                 550                 555                 560

Ile Pro Ile Lys Leu Pro Leu Thr Phe Phe Thr Glu Leu Glu Lys Thr
                565                 570                 575

Ile Leu Lys Phe Ile Trp Asn Gln Lys Arg Thr Gln Ile Ala Lys Thr
            580                 585                 590

Ile Leu Ser Lys Lys Asn Lys Ala Gly Gly Ile Thr Leu Pro Asp Phe
        595                 600                 605

Lys Leu Tyr Tyr Lys Ala Thr Val Asn Lys Thr Ala Trp Tyr Trp Tyr
    610                 615                 620

Gln Asn Arg Tyr Ile Asp Gln Trp Asn Arg Met Glu Ala Ser Glu Ile
625                 630                 635                 640

Thr Pro His Ile Tyr Asn His Leu Ile Phe Asp Lys Pro Asp Lys Asn
                645                 650                 655

Arg Gln Trp Gly Lys Asp Ser Leu Phe Asn Lys Trp Cys Trp Glu Asn
            660                 665                 670

Trp Leu Ala Ile Cys Arg Lys Leu Lys Leu Asp Pro Phe Leu Thr Pro
        675                 680                 685

Tyr Thr Lys Ile Asn Thr Arg Trp Ile Lys Asp Leu Asn Val Arg Pro
    690                 695                 700

Asn Thr Ile Lys Thr Leu Glu Glu Asn Leu Gly Asn Thr Ile Gln Asp
705                 710                 715                 720

Ile Gly Met Gly Lys Val Phe Met Thr Lys Thr Pro Lys Ala Met Ala
                725                 730                 735

Thr Lys Val Lys Ile Asp Lys Trp Asp Leu Ile Lys Leu Lys Ser Phe
            740                 745                 750

Cys Thr Ala Lys Glu Thr Ile Ile Arg Val Asn Arg Gln Pro Thr Glu
        755                 760                 765

Trp Glu Lys Ile Phe Ala Thr Tyr Pro Ser Asp Lys Gly Leu Ile Ser
    770                 775                 780

Arg Ile Tyr Lys Glu Leu Lys Gln Ile Tyr Lys Lys Lys Lys Thr Thr
785                 790                 795                 800

Pro Ser Lys Ser Gly Gln Ile Gln Glu Lys Lys Asn Asn Pro Ile Lys
                805                 810                 815

Lys Trp Ala Lys Asp Met Ser Arg His Phe Ser Lys Glu Asp Ile Tyr
            820                 825                 830

Ala Ala Asn Arg Met Lys Lys Trp Ser Ser Ser Leu Val Phe Arg Glu
        835                 840                 845

Met Gln Ile Lys Thr Thr Met Arg Tyr His Leu Met Pro Val Arg Met
    850                 855                 860

Val Ile Ile Lys Lys Ser Gly Asn Asn Thr Cys Leu Arg Gly Cys Gly
865                 870                 875                 880

Glu Ile Gly Met Leu Leu His Cys Trp Trp Glu Cys Lys Leu Val Gln
                885                 890                 895

Pro Leu Trp Lys Thr Val Trp Arg Phe Leu Lys Asp Leu Glu Pro Glu
            900                 905                 910

Ile Pro Leu Asp Pro Ala Ile Pro Leu Leu Gly Ile Tyr Pro Asn Asn
        915                 920                 925

Tyr Lys Ser Cys Tyr Tyr Lys Asp Thr Arg Thr Arg Met Phe Ile Val
    930                 935                 940

Ala Leu Phe Thr Ile Ala Lys Lys Asp Glu Phe Met Ser Phe Ala Gly
945                 950                 955                 960

Thr Trp Met Lys Leu Glu Thr Ile Ile Leu Ser Lys Leu Ser Gln Gly
                965                 970                 975

Gln Lys Thr Lys His His Met Phe Ser Leu Ile Gly Gly Ser
            980                 985                 990

<210> 155
<211> 3120
<212> DNA
<213> Homo sapiens

<400> 155
ctgaatgact actgggtaca taacaaaatg aagacagaaa taaagatgtt ctttgaaacc 60
aatgagaaca aagacacaac ataccagaat ctctgggaca cattcaaagc agtgtgtaga 120
gggaaattta cagcactaaa tgcccataag agaaagcagg aaagatccaa aattgacacc 180
ctaacatcac aattaaaaca actacagaag caagagcaaa cactttcaaa agctagcaga 240
aggcaagaaa taactaagat cagagcagaa ctgaaggaga tagagacaca aaaaaccctt 300
caaaaaatca atgaatccag gagctggttt tttgaaaaga tcaacaaaat tgatacactg 360
ctagcaagac taataaagaa gaaaagagag aagaatcaaa tagacgcaat aaaaaatgat 420
aaagcagata tcaccactga tcccacagaa atacaaacta ccatcagaga atactataaa 480
cacctctatg caaataaact agaaaatcta gaagaaatgg ataaattcct tgacacatac 540
accctcccaa gaataaacca ggaagaagtt gaatctctga atagaccaat aacaggctct 600
gaaattgagg caataattaa tagcttacca accaaaaaaa gtccaggacc agacggattc 660
acagccgaat tctaccagaa gtacaaggag gagctgatac cattccttct gaaactattc 720
caatcaatag aaaaagaggg aatcctccct aactcatttg atgaggccag catcatcctg 780
ataccaaagc ctggcagaga cacaacaaaa aaagagaatt ttagaccaat atctctgatg 840
aacattgatg caaaaatcct caataaaata ctggcaaacc gaatcaagca acacatcaaa 900
aagcttatcc accatgatca agtgggcttc atccctggga tgcaaggctg gttcaacata 960
tgcaaatcaa taaacgtaat ccagcatata aacagaacca aagacaaaaa ccacatgatt 1020
atctcaatag atgcagaaaa ggcctttgac aaaattcaac agcacttcat gctaaaaact 1080
ctcaataaat taggtattga tgggacgtat ctcaaaataa taagagctat ctgtgacaaa 1140
cccactgcca atatcatact gaatgggcaa aaactggaag cgttcccttt gaaaactggc 1200
acaagacaag ggtgccctct ctcaccactc ctattcaaca tagtgttgga agtcctggcc 1260
agggcaatca ggcaggagaa ggaaataaag ggtattcagt taggaaaaga ggaagtcaaa 1320
ttgtctctgt ttgcagatga catgattgta tatctagaaa accccatcat ctcagcccaa 1380
aatctcctta agctgataag caacttcagc aaagtctcag gatacaaaat cgatgtgcaa 1440
aaatcacaag cattcttata caccaataca gaccagacag agagccaaat catgagtgac 1500
ctcccattca caattgcttc aaagagaata aaatacctag gaatccaact tacaagggat 1560
gtgaaggacc tcttcaagga gaactacaaa ccactgctca atgaaataaa aaaggataca 1620
aacaaatgga agaacattcc aggctcatgg ataggaagaa tcaatatcgt gaaaatggcc 1680
atagagccca aggtaattta tagattcaat gccatcccca tcaagctacc aatgactttc 1740
ttcacagaac tggagaaaac tactttaaag ttcatatgga accaaaagag agcccacatt 1800
gccaagtcaa tcctaaacca aaagaacaaa gctggaggca tcacaccacc tgacttcaaa 1860
ctatactaca aggctacagt aaacaaaaca gcatggtact ggtaccaaaa cagagatata 1920
gaccagtgga acagaacaga tccctcagaa ataatgccac acatctacaa ctatctgatc 1980
tttgacaaac ctgacaaaaa gaagcaatgg ggaaaggatt ccctatttaa taaatggtgc 2040

tgggaaaact ggctagccat aggtagaaag ctgaaactgg acccttcct tacaccttat 2100
acaaaaatta attcaagatg gattaaagac ttaaatgtta gacctaaaac cataaaaacc 2160
ctagaaggaa acctaggtat taccattgag gacacaggca tgggcaagga cttcatgtct 2220
aaaacaccaa aagcaatggc aacaaaagac aaaattgaca aatgggatct aattaaacta 2280
aagagcttct gcacagcaaa agaaactacc atcagagtga acaggcaacc tacaaaatgg 2340
gagaaacttt ttgcaaccta ttcatctgac aaagggctaa tatccagaat ctacaaagaa 2400
ctcaaacaaa tttacaagaa aagaacaaac aaccccatca aaaaaaaaac taacaacccc 2460
atcaaaaagc gggcaaagga tatgaacaga cacttctcaa aagaagacat ttatgcagcc 2520
aaaagacaca tgaaaaaatg ctcatcatca ctggccatca gagaaatgca aatgaaaacc 2580
acaatgagat accatctcac accagttaga atggcgatca ttaaaaagtc aggaaacaac 2640
aggtgctgga gaggatgtgg agaaatagga acacttttac gctgttggtg ggactgtaaa 2700
ctagttcaac cattgtggaa gacagtgtgg cgattcctca gggatctaga actagaaata 2760
ccatttgacc cagccatccc attactgggt atatacccaa aggattataa atcatgctgc 2820
tataaagaca catgcagacg tatgtttatt gcggcactat tcacaatagc aaagacttgg 2880
aaccaaccca aatgtccaac aatgatagac tggattaaga aaatgtggca catatacacc 2940
atgaaatact atgcagccat aaaaaatgat gagttcatgt cctttgtagg gacatggatg 3000
aagctggaaa ccatcattct cagcaaacta tcacaaggac agaaaaccaa acaccacatg 3060
ttctcactca taggtggaaa ttgaacaatg agaatacttt gacacaggaa ggggaacatc 3120

<210> 156
<211> 1018
<212> PRT
<213> Homo sapiens

<400> 156

<210> 157
<211> 1116
<212> DNA
<213> Homo sapiens

<400> 157
ccggcaccgg cgtcaaggcc atggcgctgt gcccgggatg ggtacacacc gaattccact 60
cacgcgccaa cgtcaccggc aaccatctgc cggacttttt ctggatcgac gccgaagttc 120
tggtacgcga ggctctcaac gaccttgacc atgacaaggt agtatccatt cctaccccgc 180
tctggaagtt cttcatcgca gtggccacac ataccccacg ttccgctatg agattcctgt 240
cacgaactct gtcctcgtct cgagacaagg acgaccatcc tcgacacact ccgggaggcg 300
aggcctgaga tggccagcgt caaacccact aaggaccggg gccggtacac caatgatctg 360
tccgccgcga cgcggcaggc agcgaacatg cttctgctgc gtcctttggt gtggaaagtc 420
gtcaaagtga gcgtccacgg agccgacaac ctcgacgggc tcgacggtgc ttacgtcgcc 480
gtcgctaacc attcctccca cctcgacgcg ccgctcgttt ttggggccct tcccaagcgg 540
ctgtcaaagt acctagctac cggggccgct gctgactatt tcttcaccgc ctggtggaag 600
gccatcgctc cggtgctctt cttcaacgcg ttcccggtcg accgaggcaa aggcaaaagt 660
aagcaaggtg cccgtagtcc ccgttcccac cgcggtatgg ctgggtcact gctgacagat 720
ggcgtccccc tgctgatctt tccggagggc acccggtctc gcaccggcgc aatgggcacc 780
ttcaaacctg ggctgccgc attggctatt tcacgtgggg ttccggttat cccgattgct 840
ttagtaggag catgggcggc tatgccgtcc gagcaagcca ggttaccaaa aggacgtcca 900
ttggtccacg tggctattgg acaccctatg gaccctgttc ccggcgagat cgcccaccaa 960
ttctccgaac ggattcgtcg ccaggtcatt gagttgcacg accaaaccgc ccgcgcctac 1020
ggcatgccaa cccttgacga atacggacgc caccgcgcgc taagccaggc ctccgagagc 1080
ggcgacaccg catccaccaa ccactcgacg tgacac 1116

<210> 158
<211> 267
<212> PRT
<213> Homo sapiens

<400> 158
Met Ala Ser Val Lys Pro Thr Lys Asp Arg Gly Arg Tyr Thr Asn Asp
1               5                   10                  15

Leu Ser Ala Ala Thr Arg Gln Ala Ala Asn Met Leu Leu Leu Arg Pro
            20                  25                  30

Leu Val Trp Lys Val Val Lys Val Ser Val His Gly Ala Asp Asn Leu
        35                  40                  45

Asp Gly Leu Asp Gly Ala Tyr Val Ala Val Ala Asn His Ser Ser His
    50                  55                  60

Leu Asp Ala Pro Leu Val Phe Gly Ala Leu Pro Lys Arg Leu Ser Lys
65                  70                  75                  80

Tyr Leu Ala Thr Gly Ala Ala Ala Asp Tyr Phe Phe Thr Ala Trp Trp
                85                  90                  95

Lys Ala Ile Ala Pro Val Leu Phe Phe Asn Ala Phe Pro Val Asp Arg
            100                 105                 110

Gly Lys Gly Lys Ser Lys Gln Gly Ala Arg Ser Pro Arg Ser His Arg
        115                 120                 125

Gly Met Ala Gly Ser Leu Leu Thr Asp Gly Val Pro Leu Leu Ile Phe
    130                 135                 140

Pro Glu Gly Thr Arg Ser Arg Thr Gly Ala Met Gly Thr Phe Lys Pro
145                 150                 155                 160

Gly Ala Ala Ala Leu Ala Ile Ser Arg Gly Val Pro Val Ile Pro Ile
                165                 170                 175

Ala Leu Val Gly Ala Trp Ala Ala Met Pro Ser Glu Gln Ala Arg Leu
            180                 185                 190

Pro Lys Gly Arg Pro Leu Val His Val Ala Ile Gly His Pro Met Asp
        195                 200                 205

Pro Val Pro Gly Glu Ile Ala His Gln Phe Ser Glu Arg Ile Arg Arg
    210                 215                 220

Gln Val Ile Glu Leu His Asp Gln Thr Ala Arg Ala Tyr Gly Met Pro
225                 230                 235                 240

Thr Leu Asp Glu Tyr Gly Arg His Arg Ala Leu Ser Gln Ala Ser Glu
                245                 250                 255

Ser Gly Asp Thr Ala Ser Thr Asn His Ser Thr
            260                 265

<210> 159
<211> 906
<212> DNA
<213> Homo sapiens

<400> 159
ataccccacg ttccgctatg agattcctgt cacgaactct gtcctcgtct cgagacaagg 60
acgaccaccc tcgacacact ccgggaggcg aggcctgaga tggccagcgt caaacccact 120
aaggaccggg gccggtacac caatgatctg tccgccgcga cgcggcaggc agcgaacatg 180
cttctgctgc gtcctttggt gtggaaagtc gtcaaagtga gcgtccacgg agccgacaac 240
ctcgacgggc tcgacggtgc ctacgtcgcc gtcgctaacc attcctccca cctcgacgcg 300
ccgctcgttt ttggggccct tcccaagcgg ctgtcaaagt acctagctac cggggccgct 360
gctgactatt tcttcaccgc ctggtggaag gccatcgctc cggtgctctt cttcaacgcg 420
ttcccggtcg accgaggcaa aggcaaaagt aagcaaggtg cccgtagtcc ccgttcccac 480
cgcggtatgg ctgggtcact gctgacagat ggcgtccccc tgctgatctt tccggagggc 540
acccggtctc gcaccggtgc aatgggcacc ttcaaacctg gggctgccgc attggctatt 600
tcacgtgggg ttccggttat cccgattgct ttagtaggag catgggcggc tatgccgtcc 660
gagcaagccg ggttaccaaa aggacgccca tcggtccacg tggctattgg acaccctatg 720
gaccctgttc ccggcgagat cgcccaccaa ttctccgaac ggattcgtcg ccaggtcatt 780
gagttgcacg accaaaccgc ccgcgcctac ggcatgccaa cccttgacga atacggacgc 840
caccgcgcgc taagccaggc ctccgagagc ggcgacaccg catccaccaa ccactcgacg 900
tgacac                                                            906

<210> 160
<211> 267
<212> PRT
<213> Homo sapiens

<400> 160
Met Ala Ser Val Lys Pro Thr Lys Asp Arg Gly Arg Tyr Thr Asn Asp
1               5                   10                  15

Leu Ser Ala Ala Thr Arg Gln Ala Ala Asn Met Leu Leu Leu Arg Pro
            20                  25                  30

Leu Val Trp Lys Val Val Lys Val Ser Val His Gly Ala Asp Asn Leu
        35                  40                  45

Asp Gly Leu Asp Gly Ala Tyr Val Ala Val Ala Asn His Ser Ser His
    50                  55                  60

Leu Asp Ala Pro Leu Val Phe Gly Ala Leu Pro Lys Arg Leu Ser Lys
65                  70                  75                  80

Tyr Leu Ala Thr Gly Ala Ala Ala Asp Tyr Phe Phe Thr Ala Trp Trp
                85                  90                  95

Lys Ala Ile Ala Pro Val Leu Phe Phe Asn Ala Phe Pro Val Asp Arg
            100                 105                 110

Gly Lys Gly Lys Ser Lys Gln Gly Ala Arg Ser Pro Arg Ser His Arg
        115                 120                 125

Gly Met Ala Gly Ser Leu Leu Thr Asp Gly Val Pro Leu Leu Ile Phe
    130                 135                 140

Pro Glu Gly Thr Arg Ser Arg Thr Gly Ala Met Gly Thr Phe Lys Pro
145                 150                 155                 160

Gly Ala Ala Ala Leu Ala Ile Ser Arg Gly Val Pro Val Ile Pro Ile
                165                 170                 175

Ala Leu Val Gly Ala Trp Ala Ala Met Pro Ser Glu Gln Ala Gly Leu
            180                 185                 190

Pro Lys Gly Arg Pro Ser Val His Val Ala Ile Gly His Pro Met Asp
        195                 200                 205

Pro Val Pro Gly Glu Ile Ala His Gln Phe Ser Glu Arg Ile Arg Arg
    210                 215                 220

Gln Val Ile Glu Leu His Asp Gln Thr Ala Arg Ala Tyr Gly Met Pro
225                 230                 235                 240

Thr Leu Asp Glu Tyr Gly Arg His Arg Ala Leu Ser Gln Ala Ser Glu
                245                 250                 255

Ser Gly Asp Thr Ala Ser Thr Asn His Ser Thr
            260                 265


<210> 161
<211> 1388
<212> DNA
<213> Homo sapiens

<400> 161
aacaggtcac aggaaagtag gaacctggga ggtctcctgc ctctgctcca tgctgccctc 60
ccctgcccca agtcacctgt cccctgtatg tgggttgcag ttgcgagtga atcaggaaga 120
gctgtcggaa aactccagca gcacccccag tgaggagcag gacgaggagg ccagccagag 180
ccgccacaga cactgtgaga acaagcagca gatgcggacc aacgtcatcc gggagatcat 240
ggacaccgag cgggtgtaca tcaaacacct cagggacatc tgtgagggct atatccgaca 300
gtgccgcaag cacacaggaa tgttcaccgt tgcgcagcta gccactattt ttggaaacat 360
tgaagatatt tacaaattcc aaagaaagtt tctgaaagac cttgagaaac agtacaacaa 420
agaggaacct cacttaagtg aaataggatc ttgctttctt caaaatcaag agggctttgc 480
catctattcc gagtactgca acaaccaccc gggcgcctgc ctggagctcg ccaacctcat 540
gaagcagggc aagtacagac atttctttga agcctgccgc ctgctgcagc agatgattga 600
catcgccatc gacgggttcc tgctcacacc agtgcagaag atctgcaaat acccgctgca 660
gctggccgag ctgctcaagt ataccacaca ggaacacagt gattacagca acataaaggc 720
agcatatgag gccatgaaga atgtggcctg tctgatcaac gagcgcaagc gcaagctgga 780
gagcatcgac aagatagctc gctggcaggt gtctatcgtg ggctgggagg gactggatat 840
cttagaccga agctcagaat tgattcattc tggggagctg accaaaatca ctaagcaagg 900
caaaagccag cagcggacgt tcttcctgtt tgaccaccag ctggtgtcct gcaagaagga 960
cctgctgcgc agggacatgc tgtactacaa gggccggctg gacatggatg agatggagct 1020
tgtggacctg ggggatgggc gcgacaagga ctgcaacctc agcgtgaaaa atgccttcaa 1080
gctcgtcagt aggaccacag acgaggttta tttgttttgt gccaaaaaac aagaagacaa 1140
ggcgaggtgg ctgcaggcct gtgcagatga aaggaggcgg gtgcaagagg acaaggagat 1200
gggaatggaa atttcagaaa accagaagaa acttgccatg ttaaatgctc aaaaggcagg 1260
acatggaaag tcaaaaggta agttatggag aaggctttgt ccccttaatg cttatcagta 1320

ttctcctgaa aatgggagca taccccaagt tgtcagcctg tgaccagctt ggagcaagga 1380
gaacagta 1388

<210> 162
<211> 437
<212> PRT
<213> Homo sapiens

<400> 162
Met Leu Pro Ser Pro Ala Pro Ser His Leu Ser Pro Val Cys Gly Leu
1               5                   10                  15

Gln Leu Arg Val Asn Gln Glu Glu Leu Ser Glu Asn Ser Ser Ser Thr
            20                  25                  30

Pro Ser Glu Glu Gln Asp Glu Glu Ala Ser Gln Ser Arg His Arg His
        35                  40                  45

Cys Glu Asn Lys Gln Gln Met Arg Thr Asn Val Ile Arg Glu Ile Met
    50                  55                  60

Asp Thr Glu Arg Val Tyr Ile Lys His Leu Arg Asp Ile Cys Glu Gly
65                  70                  75                  80

Tyr Ile Arg Gln Cys Arg Lys His Thr Gly Met Phe Thr Val Ala Gln
                85                  90                  95

Leu Ala Thr Ile Phe Gly Asn Ile Glu Asp Ile Tyr Lys Phe Gln Arg
            100                 105                 110

Lys Phe Leu Lys Asp Leu Glu Lys Gln Tyr Asn Lys Glu Glu Pro His
        115                 120                 125

Leu Ser Glu Ile Gly Ser Cys Phe Leu Gln Asn Gln Glu Gly Phe Ala
    130                 135                 140

Ile Tyr Ser Glu Tyr Cys Asn Asn His Pro Gly Ala Cys Leu Glu Leu
145                 150                 155                 160

Ala Asn Leu Met Lys Gln Gly Lys Tyr Arg His Phe Phe Glu Ala Cys
                165                 170                 175

Arg Leu Leu Gln Gln Met Ile Asp Ile Ala Ile Asp Gly Phe Leu Leu
            180                 185                 190

Thr Pro Val Gln Lys Ile Cys Lys Tyr Pro Leu Gln Leu Ala Glu Leu
        195                 200                 205

Leu Lys Tyr Thr Thr Gln Glu His Ser Asp Tyr Ser Asn Ile Lys Ala
    210                 215                 220

Ala Tyr Glu Ala Met Lys Asn Val Ala Cys Leu Ile Asn Glu Arg Lys
225                 230                 235                 240

Arg Lys Leu Glu Ser Ile Asp Lys Ile Ala Arg Trp Gln Val Ser Ile
                245                 250                 255

Val Gly Trp Glu Gly Leu Asp Ile Leu Asp Arg Ser Ser Glu Leu Ile
            260                 265                 270

His Ser Gly Glu Leu Thr Lys Ile Thr Lys Gln Gly Lys Ser Gln Gln
        275                 280                 285

Arg Thr Phe Phe Leu Phe Asp His Gln Leu Val Ser Cys Lys Lys Asp
    290                 295                 300

Leu Leu Arg Arg Asp Met Leu Tyr Tyr Lys Gly Arg Leu Asp Met Asp
305                 310                 315                 320

Glu Met Glu Leu Val Asp Leu Gly Asp Gly Arg Asp Lys Asp Cys Asn
                325                 330                 335

Leu Ser Val Lys Asn Ala Phe Lys Leu Val Ser Arg Thr Thr Asp Glu
            340                 345                 350

Val Tyr Leu Phe Cys Ala Lys Lys Gln Glu Asp Lys Ala Arg Trp Leu
        355                 360                 365

Gln Ala Cys Ala Asp Glu Arg Arg Arg Val Gln Glu Asp Lys Glu Met
    370                 375                 380

Gly Met Glu Ile Ser Glu Asn Gln Lys Lys Leu Ala Met Leu Asn Ala
385                 390                 395                 400

Gln Lys Ala Gly His Gly Lys Ser Lys Gly Lys Leu Trp Arg Arg Leu
                405                 410                 415

Cys Pro Leu Asn Ala Tyr Gln Tyr Ser Pro Glu Asn Gly Ser Ile Pro
            420                 425                 430

Gln Val Val Ser Leu
        435

<210> 163
<211> 1463
<212> DNA
<213> Homo sapiens

<400> 163
ggaagacttc tgggcagaag cggaacacag gagcagagac acatagtctt ggctccagtt 60
tcgtttcagt tatgcccacc ctttcagtgt tcatggatgt gcccctcgcc cacaagctag 120
agggcagctt gttaaagacc tacaaacaag atgattaccc gaacaagata ttcttagcct 180
atagaggcac cttcccacag ccccatggag tccaggagag atttgtttgc aggctgtctg 240
cagagctcag ccctgggggc ccaaaccagg catctggagc tccctctgtg gttttcctca 300
cagtctgcat gacaaatgaa ggccatccct gggtttctct cgtggtgcag aagactcgac 360
tacagatttc acaggatccc tccctgaatt atgagtactt gcccaccatg ggcctgaaat 420
cattcatcca ggcctctcta gcactcctct ttggaaagca cagccaagcc attgtggaga 480
acagggtagg gggtgtacac actgttggtg acagtggtgc cttccagctt ggcgtccagt 540
ttctcagagc ttggcataag gatgctcgta tagtttacat catctcttct caaaaagttc 600
ccacagaact gcatggactc gtcttccagg acatgggctt tacagtttat gaatactctg 660
tctgggaccc caagaagcta tgcatggacc ccgacatact cctcaatgtg gtggagcaga 720
tcccacatgg ctgtgtcctt gtgatgggga acattatcga ctgcaagttg acaccaagtg 780
ggtgggcaaa gttgatgtcc atgataaaga gcaagcagat attcccattt tttgatattc 840
cctgtcaagg tttatacacc agtgacttgg aagaagatac tagaatctta caatactttg 900
tgtctcaagg ctttgagttc ttctgcagcc agtctctgtc caaaaatttt ggcatttatg 960
atgaaggagt ggggatgcta gtggtggtgg cagtcaacaa ccagcagctg ctgtgtgtcc 1020
tctcccagct ggaaggatta gcccaggccc tgtggctaaa cccccccaac acgggtgcac 1080
gtgtcatcac ctccatcctc tgcaaccctg ctctgctggg agaatggaag cagagtctaa 1140
aagaagttgt agagaacatc atgctaacca aggaaaaagt gaaggagaaa ctccagctcc 1200
tgggaacccc tgggtcctgg ggtcacatca ccgagcagag tgggacccac ggctatcttg 1260
gactcaactg taagcaggtg gaatacctgg tcaggaagaa gcacatctat atccccaaga 1320
acggtcagat taacttcagc tgtatcaatg ccaacaacat aaattacatc actgagggca 1380
tcaatgaggc tgtcctcctc acagagagct cagagatgtg tcttccaaag gaaaaaaaaa 1440
cactgattgg aataaaactt tag 1463

<210> 164
<211> 463
<212> PRT
<213> Homo sapiens

<400> 164
Met Pro Thr Leu Ser Val Phe Met Asp Val Pro Leu Ala His Lys Leu
1               5                   10                  15

Glu Gly Ser Leu Leu Lys Thr Tyr Lys Gln Asp Asp Tyr Pro Asn Lys

20                      25                      30

Ile Phe Leu Ala Tyr Arg Gly Thr Phe Pro Gln Pro His Gly Val Gln
        35                      40                      45

Glu Arg Phe Val Cys Arg Leu Ser Ala Glu Leu Ser Pro Gly Gly Pro
    50                      55                      60

Asn Gln Ala Ser Gly Ala Pro Ser Val Val Phe Leu Thr Val Cys Met
65                      70                      75                      80

Thr Asn Glu Gly His Pro Trp Val Ser Leu Val Val Gln Lys Thr Arg
                85                      90                      95

Leu Gln Ile Ser Gln Asp Pro Ser Leu Asn Tyr Glu Tyr Leu Pro Thr
            100                     105                     110

Met Gly Leu Lys Ser Phe Ile Gln Ala Ser Leu Ala Leu Leu Phe Gly
        115                     120                     125

Lys His Ser Gln Ala Ile Val Glu Asn Arg Val Gly Gly Val His Thr
    130                     135                     140

Val Gly Asp Ser Gly Ala Phe Gln Leu Gly Val Gln Phe Leu Arg Ala
145                     150                     155                     160

Trp His Lys Asp Ala Arg Ile Val Tyr Ile Ile Ser Ser Gln Lys Val
                165                     170                     175

Pro Thr Glu Leu His Gly Leu Val Phe Gln Asp Met Gly Phe Thr Val
            180                     185                     190

Tyr Glu Tyr Ser Val Trp Asp Pro Lys Lys Leu Cys Met Asp Pro Asp
        195                     200                     205

Ile Leu Leu Asn Val Val Glu Gln Ile Pro His Gly Cys Val Leu Val
    210                     215                     220

Met Gly Asn Ile Ile Asp Cys Lys Leu Thr Pro Ser Gly Trp Ala Lys
225                     230                     235                     240

Leu Met Ser Met Ile Lys Ser Lys Gln Ile Phe Pro Phe Phe Asp Ile
                245                     250                     255

Pro Cys Gln Gly Leu Tyr Thr Ser Asp Leu Glu Glu Asp Thr Arg Ile
            260                     265                     270

Leu Gln Tyr Phe Val Ser Gln Gly Phe Glu Phe Phe Cys Ser Gln Ser
        275                     280                     285

Leu Ser Lys Asn Phe Gly Ile Tyr Asp Glu Gly Val Gly Met Leu Val
    290                     295                     300

Val Val Ala Val Asn Asn Gln Gln Leu Leu Cys Val Leu Ser Gln Leu
305                     310                     315                     320

Glu Gly Leu Ala Gln Ala Leu Trp Leu Asn Pro Pro Asn Thr Gly Ala
                325                     330                     335

Arg Val Ile Thr Ser Ile Leu Cys Asn Pro Ala Leu Leu Gly Glu Trp
            340                     345                     350

Lys Gln Ser Leu Lys Glu Val Val Glu Asn Ile Met Leu Thr Lys Glu
        355                     360                     365

Lys Val Lys Glu Lys Leu Gln Leu Leu Gly Thr Pro Gly Ser Trp Gly
    370                     375                     380

His Ile Thr Glu Gln Ser Gly Thr His Gly Tyr Leu Gly Leu Asn Cys

385                 390                 395                 400

Lys Gln Val Glu Tyr Leu Val Arg Lys Lys His Ile Tyr Ile Pro Lys
                405                 410                 415

Asn Gly Gln Ile Asn Phe Ser Cys Ile Asn Ala Asn Asn Ile Asn Tyr
            420                 425                 430

Ile Thr Glu Gly Ile Asn Glu Ala Val Leu Leu Thr Glu Ser Ser Glu
        435                 440                 445

Met Cys Leu Pro Lys Glu Lys Lys Thr Leu Ile Gly Ile Lys Leu
    450                 455                 460


<210> 165
<211> 1280
<212> DNA
<213> Homo sapiens

<400> 165
ccaccctttc agttatgccc accctttcag tgttcatgga tgtgcccctc gcccacaagc 60
tagagggcag cttgttaaag acctacaaac aagatgatta cccgaacaag atattcttag 120
cctatagagt ctgcatgaca aatgaaggcc atccctgggt ttctctcgtg gtgcagaaga 180
ctcgactaca gatttcacag gatccctccc tgaattatga gtacttgccc accatgggcc 240
tgaaatcatt catccaggcc tctctagcac tcctctttgg aaagcacagc caagccattg 300
tggagaacag ggcagggggt gtacacactg ttggtgacag tggtgccttc cagcttggcg 360
tccagtttct cagagcttgg cataaggatg ctcgtatagt ttacatcatc tcttctcaaa 420
aagaactgca tggactcgtc ttccaggaca tgggctttac agtttatgaa tactctgtct 480
gggaccccaa gaagctatgc atggaccccg acatactcct caatgtggtg gagcagatcc 540
cacatggctg tgtccttgtg atggggaaca ttatcgactg caagttgaca ccaagtgggt 600
gggcaaagtt gatgtccatg ataaagagca agcagatatt cccatttttt gatattccct 660
gtcaaggttt atacaccagt gacttggaag aagatactag aatcttacaa tactttgtgt 720
ctcaaggctt tgagttcttc tgcagccagt ctctgtccaa gaattttggc atttatgatg 780
aaggagtggg gatgctagtg gtggtggcag tcaacaacca gcagctgctg tgtgtcctct 840
cccagctgga aggattagcc caggccctat ggctaaaccc ccccaacacg ggtgcacgtg 900
tcatcacctc catcctctgc aaccctgctc tgctgggaga atggaagcag agtctaaaag 960
aagttgtaga gaacatcatg ctaaccaagg aaaaagtgaa ggagaaactc cagctcctgg 1020
gaacccctgg gtcctggggt cacatcaccg agcagagtgg gacccacggc tatcttggac 1080
tcaactccca gcaggtggaa tacctggtca ggaagaagca catctatatc ccaaagaacg 1140
gtcagattaa cttcagctgt atcaatgcca acaacataaa ttacatcact gagggcatca 1200
atgaggctgt cctcctcaca gagagctcag agatgtgtct tccaaaggaa aaaaaaacac 1260
tgattggaat aaaactttag                                              1280


<210> 166
<211> 421
<212> PRT
<213> Homo sapiens

<400> 166
Met Pro Thr Leu Ser Val Phe Met Asp Val Pro Leu Ala His Lys Leu
1               5                   10                  15

Glu Gly Ser Leu Leu Lys Thr Tyr Lys Gln Asp Asp Tyr Pro Asn Lys
            20                  25                  30

Ile Phe Leu Ala Tyr Arg Val Cys Met Thr Asn Glu Gly His Pro Trp
        35                  40                  45

Val Ser Leu Val Val Gln Lys Thr Arg Leu Gln Ile Ser Gln Asp Pro
    50                  55                  60

Ser Leu Asn Tyr Glu Tyr Leu Pro Thr Met Gly Leu Lys Ser Phe Ile
65                  70                  75                  80

Gln Ala Ser Leu Ala Leu Leu Phe Gly Lys His Ser Gln Ala Ile Val
                85                  90                  95

Glu Asn Arg Ala Gly Gly Val His Thr Val Gly Asp Ser Gly Ala Phe
            100                 105                 110

Gln Leu Gly Val Gln Phe Leu Arg Ala Trp His Lys Asp Ala Arg Ile
        115                 120                 125

Val Tyr Ile Ile Ser Ser Gln Lys Glu Leu His Gly Leu Val Phe Gln
    130                 135                 140

Asp Met Gly Phe Thr Val Tyr Glu Tyr Ser Val Trp Asp Pro Lys Lys
145                 150                 155                 160

Leu Cys Met Asp Pro Asp Ile Leu Leu Asn Val Val Glu Gln Ile Pro
                165                 170                 175

His Gly Cys Val Leu Val Met Gly Asn Ile Ile Asp Cys Lys Leu Thr
            180                 185                 190

Pro Ser Gly Trp Ala Lys Leu Met Ser Met Ile Lys Ser Lys Gln Ile
        195                 200                 205

Phe Pro Phe Phe Asp Ile Pro Cys Gln Gly Leu Tyr Thr Ser Asp Leu
    210                 215                 220

Glu Glu Asp Thr Arg Ile Leu Gln Tyr Phe Val Ser Gln Gly Phe Glu
225                 230                 235                 240

Phe Phe Cys Ser Gln Ser Leu Ser Lys Asn Phe Gly Ile Tyr Asp Glu
                245                 250                 255

Gly Val Gly Met Leu Val Val Val Ala Val Asn Asn Gln Gln Leu Leu
            260                 265                 270

Cys Val Leu Ser Gln Leu Glu Gly Leu Ala Gln Ala Leu Trp Leu Asn
        275                 280                 285

Pro Pro Asn Thr Gly Ala Arg Val Ile Thr Ser Ile Leu Cys Asn Pro
    290                 295                 300

Ala Leu Leu Gly Glu Trp Lys Gln Ser Leu Lys Glu Val Val Glu Asn
305                 310                 315                 320

Ile Met Leu Thr Lys Glu Lys Val Lys Glu Lys Leu Gln Leu Leu Gly
                325                 330                 335

Thr Pro Gly Ser Trp Gly His Ile Thr Glu Gln Ser Gly Thr His Gly
            340                 345                 350

Tyr Leu Gly Leu Asn Ser Gln Gln Val Glu Tyr Leu Val Arg Lys Lys
        355                 360                 365

His Ile Tyr Ile Pro Lys Asn Gly Gln Ile Asn Phe Ser Cys Ile Asn
    370                 375                 380

Ala Asn Asn Ile Asn Tyr Ile Thr Glu Gly Ile Asn Glu Ala Val Leu
385                 390                 395                 400

Leu Thr Glu Ser Ser Glu Met Cys Leu Pro Lys Glu Lys Lys Thr Leu
                405                 410                 415

Ile Gly Ile Lys Leu
            420

<210> 167
<211> 4915
<212> DNA
<213> Homo sapiens

<400> 167
   aatatgcctg ccataaaggg gaaccggtgg tggcagtgga gctggtgcag gtgtggaaag 60
   tcatgagaat cctcctcctc gggttcatcc ccaggtttcc tccccttccc ctgcccgcgc 120
   ctgcttgcag gaagcgtgtt tactacaggg gcctggggat gctggcctca cacatccctg 180
   cccagccaca gggtacttcc ctgaagcctc ctgtaccttc agccccatcc tcgattctcg 240
   cctccggctc ctcctccccc cacgccctcc ggaatgagcc ccgtaccccc accctcacgc 300
   gccctcgctg tataaacgcc ctcacttgta ctgcaagtcc ctgcggtccc accttcaggc 360
   ttcagcattc gctcgacgca tcccccaggc ctgcttgcct tgtcactgta gcgccagacc 420
   cagcttcctt tgcggctccg agaagcttcc ccctgcgact tccgcgagga gacgagtctg 480
   cgcagcgtgg tggccgccgc cccccgaccc tctgcgcact ctctcccgcg ccggcggctc 540
   agcctagccc cgttcggccg gccgagacta tggacacgga ggatgacccc ttgctgcagg 600
   atgtgtggct agaggaggag caggaggagg aagaagcaac gggtgaaacc tttttagggg 660
   cccagaagcc agggccccaa cctggggcag ggggacagtg ttgctggcgg cactggcccc 720
   tggcttcccg acccccagct tcgggcttct ggagtaccct gggctgggcc ttcaccaatc 780
   cgtgctgtgc tgggctggtg ctcttcctgg gctgcagcat ccccatggcc ctgtcagcct 840
   tcatgttcct ttactaccca ccgctggaca ttgacatctc ctacaacgcc tttgagatcc 900
   gcaaccacga ggcctcacag cgtttcgacg ctctcactct ggcgcttaag tcccagtttg 960
   gatcctgggg gcggaaccgg cgcgatttgg ccgacttcac ctccgagacg cttcagcgcc 1020
   ttatctcaga gcagctgcag cagctgcatc tcggcaaccg ctcgcggcaa gcctcccgag 1080
   ccccccgcgt catccccgcg gcctcactcg gtagcccagg cccttaccgg gacacttccg 1140
   cggctcaaaa gcccacagcc aatcggagcg ggcgacttcg gcgtgagacc ccgcccctgg 1200
   aggatctggc agccaaccag agtgaagacc cgcgaaacca gcggctgagc aagaatgggc 1260
   ggtaccagcc cagcatcccg ccccacgcgg cagtcgcggc caatcagagc cgtgcccgcc 1320
   gaggcgcctc gcgctgggac tactcgcgcg cctatgtgag tgccaacact cagacgcacg 1380
   cgcactggcg catcgagctc atcttcctgg cgcgcggcga cgcggagcgc aacattttca 1440
   ccagtgagcg cctggtcacg atccatgaga tcgagcgcaa gatcatggac cacccaggct 1500
   tccgggagtt ctgctggaag ccccacgagg tgctcaagga tctgccgctg ggctcctact 1560
   cctactgctc gccccccagc tcgctcatga cctacttttt tcccaccgag aggggcggca 1620
   agatctacta tgacggcatg ggccaggacc tggcggacat ccggggctcc ctggagctgg 1680
   ccatgactca ccctgagttc tactggtatg tggatgaggg cctctctgca gacaatctga 1740
   agagctccct cctgcgcagt gagatcctgt ttggagcacc cctgcccaac tactactcag 1800
   tagatgaccg ctgggaggaa caacggctaa agtttcagag cttcgtggtc acctacgtgg 1860
   ccatgctggc caagcagtct accagcaaag tccaggttct ctatgggggg acagacctgt 1920
   ttgactatga agtgcgcagg acgttcaaca atgacatgct cctggccttc atcagcagca 1980
   gctgcattgc tgccctggtc tacatcctca cctcctgctc agtgttcctg tccttctttg 2040
   ggattgccag cattggtctc agctgcctgg tggccctctt cctgtaccac gtggtctttg 2100
   gtatccagta cttgggcatc ctgaatgggg tggccgcctt cgtgatcgtg ggcattggtg 2160
   tggacgatgt ctttgtgttc atcaacacct accgccaggc cacccacctg gaagacccac 2220
   agctgcgcat gatccacacc gtccaaactg caggcaaggc caccttcttc acctccctga 2280
   ccacagccgc cgcctacgca gctaacgtct tctcccagat cccagccgtc cacgactttg 2340
   gcctgttcat gtctctcatc gtgtcctgtt gctggctggc cgtgcttgtc accatgcctg 2400
   cagctctggg cctctggagc ctctacctgg caccactgga gagctcctgc cagaccagct 2460
   gccaccagaa ttgcagccgg aagacctccc tgcacttccc cggagacgtg tttgccactc 2520
   ccgagcaggt tggaggcagc cctgcccagg cccccatacc ctacctggat gatgacatcc 2580
   ccttgctgga ggtcgaggaa gagccagtgt cactggagct gggagacgtg tccctggtgt 2640
   ctgtgtcccc cgagggtctg cagccagcct ccaacacggg cagccgcggc catctcatcg 2700
   tgcagctgca ggagctgctg caccactggg tcctgtggtc agccgtcaag agccgctggg 2760
   tgattgtggg gctgttcgtc tccatcctca tcttgtccct ggtgttcgcc agccggctcc 2820
   gccccgccag ccgggccccg ctactcttcc ggcctgatac caacatccag gtgctgctgg 2880
   acctcaagta caacctgagc gccgagggca tctcctgcat cacctgttca ggtctgttcc 2940
   aggagaagcc ccacagcctg cagaacaaca tccggacgtc cctggagaag aagaggcgag 3000
   gctcaggggt cccctgggct agccggcctg aggccaccct gcaggatttc ccaggcaccg 3060
   tgtacatctc taaagtgaag agtcaaggcc accccgctgt ctacaggctc tccctcaatg 3120
   ccagcctgcc tgctccttgg caggctgtgt cgcctgggga tggagaggtg ccctccttcc 3180
   aggtgtatag agcgcctttt ggtaacttca ccaagaagct gaccgcttgt atgtctacag 3240
   tagggctgct ccaggcggcg agcccctccc gcaagtggat gctgacgacc ttggcctgtg 3300
   atgccaagcg gggctggaag tttgacttca gcttctacgt ggccaccaag gagcagcagc 3360
   acacccggaa gctgtacttc gcccagtccc acaagccccc cttccacggg cgcgtatgca 3420
   tggcaccccc tggctgcctg cttagctcca gccccgatgg gcctaccaaa ggcttcttct 3480
   tcgtgcctag tgagaaagtg cccaaggccc gtctctcagc caccttcggc ttcaacccct 3540
   gcgtgaacac gggctgcggg aagccggcgg tgcggccact agtggatacc ggggccatgg 3600
   tctttgtggt cttcggcatt attggcgtca accgcactcg gcaggtggac aaccacgtca 3660
   ttggagaccc gggtagtgtt gtctacgaca gcagctttga cctcttcaag gaaattggcc 3720
   acctgtgtca cctctgcaag gccatcgcag ccaactccga gctggtgaag ccgggtgggg 3780
   cccagtgcct gccttcaggc tacagcatct cctccttcct gcagatgttg caccctgagt 3840
   gcaaggagct gcccgagccc aacctgctcc cggggcagct gtcccacggg gcagtgggcg 3900
   tcagggaggg ccgcgtgcag gagatctcca tggctttcga gtcgaccacg tacaagggca 3960
   aatcctcctt ccagacctac tcggactacc tgcgctggga gagcttcctc cagcagcagc 4020
   tgcaggcctt gcccgagggc tcagtcctgc gccggggctt ccagacctgc gagcactgga 4080
   agcagatatt catggaaatc gtaggggtgc agagcgccct gtgcggcctg gtgctatccc 4140
   tgctcatctg cgtggccgcg gtggccgtgt tcaccaccca catcctgctc ctgctgcccg 4200
   tgctcctcag catcttgggc atcgtgtgcc tggtggtgac catcatgtac tggagcggct 4260
   gggagatggg ggctgtggaa gccatctccc tgtccatcct cgttggctcc tccgtggatt 4320
   actgcgtcca cctggtcgag ggctacctgc tggctggaga gaacctgccc ccccaccagg 4380
   ccgaggacgc ccgaacgcag cgccagtggc gtacgctgga ggccgtgcgg cacgtgggcg 4440
   tggccatcgt ctccagtgcc ctcaccacgg tcatcgccac agtgcccctc ttcttctgca 4500
   tcatcgcccc atttgccaag ttcggcaaga ttgtggcact caacacgggc gtgtccatcc 4560
   tctacacgct gaccgtcagc accgccctgc tgggcatcat ggcgcccagc tctttcactc 4620
   ggacccggac ttccttcctc aaggccctgg gtgccgtgct gctggcaggg gccctggggc 4680
   tgggtgcctg cctcgtgctc ctgcagagcg gctataagat tcccctgccc gcaggggcct 4740
   ccctatagcc cgggacgggc tctggacact tgcacctttg gtcccatggg tgggggacag 4800
   gagctgcttc ccagctcgac ttcagctagc tgtgtcccca ggcctgggcc cagggcgccc 4860
   tgcgggccag cgtggaggct gacacccaca cagatggtgt ggaccatgct gcctt 4915

<210> 168
<211> 1561
<212> PRT
<213> Homo sapiens

<400> 168

<210> 169
<211> 2004
<212> DNA
<213> Homo sapiens

<400> 169
cacccacttg ccacctcctc cataaaaggc ctgggcccag ctctggtggc gagggagtag 60
ggggtgtgtc tgtggcgtct catggcagga ggctcagcca cgacctgggg ttaccctgtg 120
gccctgctac tgctggtcgc caccctgggg ctgggtagct acgactgtgg gatcaaggga 180
atgcagctgc tggtgttccc caggccaggc cagactctcc ccttcaaggt ggtggatgaa 240
tttgggaacc gatttgatgt caacaactgc tccatctgct accactgggt cacctccagg 300
ccgcaggagc ctgcagtctt ctcggccgat tacagaggct gccacgtgct ggagaaggat 360
gggcgtttcc acctgagggt gttcatggag gctgtgctgc ccaatggtcg tgtggatgtg 420
gcacaagacg ctactctgat ctgtcccaaa cctgacccct cccggactct ggactcccag 480
ctggcaccac ccgccatgtt ctctgtctca accccacaaa ccctttcctt cctccccacc 540
tctggccata cctcccaagg ctctggccat gcctttccca gcccactgga cccagggcac 600
agctctgtcc acccaacccc tgctttacca tcccctggac ctggacctac cctcgccacc 660
ctggctcaac cccactgggg caccttggaa cactgggatg tgaacaaacg agattacata 720
ggtacccacc tgagccagga gcagtgccag gtggcctcag ggcacctccc ctgcatcgtg 780
agaagaactt caaaagaagc ctgtcagcag gctggctgct gctatgacaa caccagagag 840
gttccctgtt actatggcaa cacagctact gtccagtgct tcagagatgg ctacttcgtc 900
ctcgtagtgt cccaagaaat ggccttgaca cacaggatca cactggccaa catccacctg 960
gcctatgccc ccaccagctg ctccccaaca cagcacacgg aagctttcgt ggtcttctac 1020
ttccctctca cccactgtgg aaccacaatg caggtggctg gcgaccagct catctatgag 1080
aactggctgg tgtctggcat ccacatccaa aaggggccac agggttccat cacgcgggac 1140
agcaccttcc agcttcatgt gcgctgtgtc ttcaacgcca gtgacttcct gcccattcag 1200
gcatccattt tcccaccccc atcgcctgct cctatgaccc agcccggccc cctgcggctt 1260
gagctgcgga ttgccaaaga cgagacctgc agctcgtact atggggagga tgactatccc 1320

atcgtgaggc tgctccgaga accagtccat gtggaggtcc ggcttctgca gaggacagac 1380
cccaacctgg tcctgctgct gcaccagtgc tggggcgctc ccagtgccaa ccccttccag 1440
cagccccagt ggcccatcct gtcagacgga tgccccttca agggcgacag ctacagaacg 1500
caaatggtag ccttggacgg ggccacacct ttccagtcgc actaccagcg attcactgtt 1560
gctaccttcg ccctcctgga ctcaggctcc cagagagccc tcagaggact ggtttacttg 1620
ttctgcagca cctctgcctg ccacacctca gggctggaga cttgctccac tgcatgtagc 1680
actggcacta caagacagcg acgatcctca ggtcaccgta atgacactgc caggccccag 1740
gacatcgtga gctctccggg gccagtgggc tttgaggatt cttatgggca ggagcccaca 1800
cttgggccca cagactccaa tgggaactcc agcctgagac ctctcctttg ggcggtcctt 1860
ttgctgccag ctgttgccct ggtccttggg tttggtgtct ttgtgggcct gagccagacc 1920
tgggcccaga agctctggga aagcaacaga cagtgaatgg gcccaataaa caatcatttc 1980
caacctactg aaaaaaaaaa aaaa 2004

<210> 170
<211> 624
<212> PRT
<213> Homo sapiens

<400> 170
Met Ala Gly Gly Ser Ala Thr Thr Trp Gly Tyr Pro Val Ala Leu Leu
1               5                   10                  15

Leu Leu Val Ala Thr Leu Gly Leu Gly Ser Tyr Asp Cys Gly Ile Lys
            20                  25                  30

Gly Met Gln Leu Leu Val Phe Pro Arg Pro Gly Gln Thr Leu Pro Phe
        35                  40                  45

Lys Val Val Asp Glu Phe Gly Asn Arg Phe Asp Val Asn Asn Cys Ser
    50                  55                  60

Ile Cys Tyr His Trp Val Thr Ser Arg Pro Gln Glu Pro Ala Val Phe
65                  70                  75                  80

Ser Ala Asp Tyr Arg Gly Cys His Val Leu Glu Lys Asp Gly Arg Phe
                85                  90                  95

His Leu Arg Val Phe Met Glu Ala Val Leu Pro Asn Gly Arg Val Asp
            100                 105                 110

Val Ala Gln Asp Ala Thr Leu Ile Cys Pro Lys Pro Asp Pro Ser Arg
        115                 120                 125

Thr Leu Asp Ser Gln Leu Ala Pro Pro Ala Met Phe Ser Val Ser Thr
    130                 135                 140

Pro Gln Thr Leu Ser Phe Leu Pro Thr Ser Gly His Thr Ser Gln Gly
145                 150                 155                 160

Ser Gly His Ala Phe Pro Ser Pro Leu Asp Pro Gly His Ser Ser Val
                165                 170                 175

His Pro Thr Pro Ala Leu Pro Ser Pro Gly Pro Gly Pro Thr Leu Ala
            180                 185                 190

Thr Leu Ala Gln Pro His Trp Gly Thr Leu Glu His Trp Asp Val Asn
        195                 200                 205

Lys Arg Asp Tyr Ile Gly Thr His Leu Ser Gln Glu Gln Cys Gln Val
    210                 215                 220

Ala Ser Gly His Leu Pro Cys Ile Val Arg Arg Thr Ser Lys Glu Ala
225                 230                 235                 240

Cys Gln Gln Ala Gly Cys Cys Tyr Asp Asn Thr Arg Glu Val Pro Cys
                245                 250                 255

Tyr Tyr Gly Asn Thr Ala Thr Val Gln Cys Phe Arg Asp Gly Tyr Phe

            260                 265                 270

Val Leu Val Val Ser Gln Glu Met Ala Leu Thr His Arg Ile Thr Leu
        275                 280                 285

Ala Asn Ile His Leu Ala Tyr Ala Pro Thr Ser Cys Ser Pro Thr Gln
    290                 295                 300

His Thr Glu Ala Phe Val Val Phe Tyr Phe Pro Leu Thr His Cys Gly
305                 310                 315                 320

Thr Thr Met Gln Val Ala Gly Asp Gln Leu Ile Tyr Glu Asn Trp Leu
                325                 330                 335

Val Ser Gly Ile His Ile Gln Lys Gly Pro Gln Gly Ser Ile Thr Arg
            340                 345                 350

Asp Ser Thr Phe Gln Leu His Val Arg Cys Val Phe Asn Ala Ser Asp
        355                 360                 365

Phe Leu Pro Ile Gln Ala Ser Ile Phe Pro Pro Pro Ser Pro Ala Pro
    370                 375                 380

Met Thr Gln Pro Gly Pro Leu Arg Leu Glu Leu Arg Ile Ala Lys Asp
385                 390                 395                 400

Glu Thr Cys Ser Ser Tyr Tyr Gly Glu Asp Asp Tyr Pro Ile Val Arg
                405                 410                 415

Leu Leu Arg Glu Pro Val His Val Glu Val Arg Leu Leu Gln Arg Thr
            420                 425                 430

Asp Pro Asn Leu Val Leu Leu Leu His Gln Cys Trp Gly Ala Pro Ser
        435                 440                 445

Ala Asn Pro Phe Gln Gln Pro Gln Trp Pro Ile Leu Ser Asp Gly Cys
    450                 455                 460

Pro Phe Lys Gly Asp Ser Tyr Arg Thr Gln Met Val Ala Leu Asp Gly
465                 470                 475                 480

Ala Thr Pro Phe Gln Ser His Tyr Gln Arg Phe Thr Val Ala Thr Phe
                485                 490                 495

Ala Leu Leu Asp Ser Gly Ser Gln Arg Ala Leu Arg Gly Leu Val Tyr
            500                 505                 510

Leu Phe Cys Ser Thr Ser Ala Cys His Thr Ser Gly Leu Glu Thr Cys
        515                 520                 525

Ser Thr Ala Cys Ser Thr Gly Thr Thr Arg Gln Arg Arg Ser Ser Gly
    530                 535                 540

His Arg Asn Asp Thr Ala Arg Pro Gln Asp Ile Val Ser Ser Pro Gly
545                 550                 555                 560

Pro Val Gly Phe Glu Asp Ser Tyr Gly Gln Glu Pro Thr Leu Gly Pro
                565                 570                 575

Thr Asp Ser Asn Gly Asn Ser Ser Leu Arg Pro Leu Leu Trp Ala Val
            580                 585                 590

Leu Leu Leu Pro Ala Val Ala Leu Val Leu Gly Phe Gly Val Phe Val
        595                 600                 605

Gly Leu Ser Gln Thr Trp Ala Gln Lys Leu Trp Glu Ser Asn Arg Gln
    610                 615                 620

<210> 171
<211> 6108
<212> DNA
<213> Homo sapiens

<400> 171
atggccagtc acgtggacct gctgacggag ctgcagctgc tggagaaggt gcccacgctg 60
gagcggctgc gggctgccca gaagcgccgg gcccagcagc tgaagaaatg ggcacagtac 120
gagcaggact tgcagcaccg caagcgaaag catgagcgga agcgcagcac gggcggccgc 180
cgcaagaaag tgtccttcga ggccagcgtg gccctgctgg aggcctcgct gaggaacgac 240
gccgaggaag tacgctactt cctgaagaat aaggtcagcc ctgatttgtg caatgaggac 300
ggactcacag ccctacacca gtgctgcatc gacaactttg aggaaattgt gaagctgctc 360
ctctcccatg gtgccaatgt gaacgccaag gacaacgagc tgtggacacc tctccatgct 420
gcagccacct gcggccacat caacctggtg aagatcctcg ttcagtatgg ggccgacttg 480
cttgctgtca actcggatgg gaacatgcca tatgacctct gcgaggatga acccaccctg 540
gatgtcatcg agacctgcat ggcataccag ggcatcaccc aagagaaaat caacgagatg 600
cgggtggctc ctgagcagca gatgattgcg gacatccact gcatgatcgc agcgggccag 660
gacctggact ggatagatgc ccagggtgcc acactgctgc acatagctgg agccaatgga 720
tacctgcggg cagctgagct cctcctggat catggagtgc gtgtggatgt gaaggactgg 780
gatggctggg agcccctgca tgcagctgcc ttctggggac agatgcagat ggcagagcta 840
ttggtgtccc atggagctag tctcagtgca aggacatcca tggatgagat gccaatagac 900
ctgtgcgagg aggaagagtt caaggtcctg ctgctggagc taaaacacaa gcatgatgtg 960
atcatgaagt cacagctgag gcacaagtca tccttgagcc ggaggacctc cagcgcaggc 1020
agccgtggga aggtggtgcg gcgagccagc ctgtcggaca ggaccaacct gtataggaag 1080
gagtatgagg gagaggccat cctgtggcag cggagtgcag ctgaggatca gcggacctcc 1140
acctacaacg gggacatcag ggagaccagg acagaccaag agaataagga ccctaacccc 1200
aggctggaga agcccgtgct actctccgaa tttcctacca agatcccacg aggtgaactg 1260
gacatgcctg ttgagaatgg cctccgggct ccggtcagtg cctaccagta tgcgctggcc 1320
aacggggatg tctggaaggt gcatgaggtg cctgactaca gcatggccta tggcaaccct 1380
ggcgtggccg acgccacccc gccctggagc agctacaagg aacagagccc tcagacgctt 1440
ctggagctga agcggcagcg ggctgcagcc aagctgctca gccacccctt ccttagcaca 1500
cacctgggca gcagcatggc caggacgggc gagagtagca gtgaaggcaa ggcccccttg 1560
atcggaggca gaacttcacc gtacagcagc aatggacct cggtatatta cacggtcacc 1620
agcggagatc ccccactctt aaagttcaag gcccccatag aggagatgga ggagaaggtg 1680
catggctgtt gccgtatctc ctagtctccg tgtgatggag gagggagatg cctggggagg 1740
ggctcctgga atccaggcca gcccaacagc cctggctggg gaggtgtcag ggcagctggg 1800
gagaggtggg ctctgctttt cagaggaact cagaccccag ccctcagctg gctgcccata 1860
gcatcccatg tcccacgtcc cgtggttctg cttcctgctg catcgtctgc catctgacac 1920
aaggcctgtc gtggcctcct ggttcactct gctgtctgat cttgggaggg tgggcttgag 1980
atcccagctc tattcttggt ataaaggctt ctccggatca gtacatgcat gtcacattaa 2040
cacacacaca cacacacata tacacacaca cacaagctcg atcagtgtgt gtaggaatga 2100
catacctggg ctcaggggaa gcaagggggc ttagaatttg tggggtattc ccaaaaggat 2160
ggaagttaag actcagagtc tcattaccac tgccaatgtg gttttagcag gggaggggac 2220
ctgctaagct gagacccata gtcctgctca gagttatccc aaagtctgag ccaccagcca 2280
cacctgacag gggtgagaag tcctcgctgt gttcagaggg agccaggaat ctacatgggt 2340
agatgagata gacacagacc tgctccccgc agccttgttg agagccacac ttctgcccat 2400
gccaggagcc agctgtgtga ccatccaggg gtggaggggg aaaaccaggc aatttcgttc 2460
ctggaatcaa ccaaatcatg ttttcctctt ggatggaagt gtcaaaggca gaagggtgtg 2520
ggagggggac aaggtcagta tttaccaaag tgtatctgat tttaaaaatt cctttagtct 2580
gtaaaactcc tagagggagg gaggtaactg aattcacttc tttttgtgga tcgtatcaag 2640
gtcactgggt tttactggct ggtgctggga aaatgaagct aagtgaggag cttccattgg 2700
aatgcttttc cagggagaga ggccagttaa tttaaaaaaa acagtcgcta gttaacagcg 2760
acagagccca gcaccctggg gtctttgtga atatccagac tgtttcagcc cagcccatct 2820
cagccaaccc tccttagact gagctgtcag agcaagcaat taggggccag cctgcctcca 2880
cctcccaccc ccttccacct ccatcagtca tgtgtgcaga gtcagtgctc gggatcccgg 2940
gcccagcttt tgcctttttg gggatgcttg gtgagacaga tttgccagtc agccctttg 3000
agttcccgcc tcacccaggg gctcccagcc tgcacttgca ggagtggtga tgccccaagt 3060
ctgcgaatcc agggtgcacg tggtcaatat cccctcctgc attcaggaga gccatggtag 3120
ggctggagtt gggtcttgcc cagccctgca gtttcatagt cccagccttc ctggtgctgg 3180
ggagggagga ctgtgaatgg ctgttctccc ctcactgctg agtctcccag gacccccttt 3240
ggagatgccc atggcatggg cactgcccac aggctcagcc agaacctctt ggtgtacccg 3300
ataagctgca ggttatccct tgctctgtgc gccttttatt tgtccttaaa ctacctcctt 3360
agagctctga aggggtctcc tagttccaga ttttaatttg ggaacagat ctgggttctt 3420
tttaaccctc ttctttctca gtctatgaga aacttgccct gaggggcacc tgggctaggg 3480
gcttgggact ggaagaccat ccccgccttg tgccacaact ttggtcatgg gatctgctct 3540
ttgtcattct tagcccccta ctgtggcccc catagcccca taacccagag agggagctgg 3600

acttcaggga gcctgagtga tgctttccca ggagcagggc agctggctgg accagaaagt 3660
agagggccca tgggagtgac tgcacccttg gtggctgctg gaaggggaga ggttctcagc 3720
atcaggccac ctccacccca atgccaggat agatgtattc tagagtaggg gtggaggcgg 3780
cccaggaggc tgaagacagg tgcacagatg cttcccacga ccttgccatt tggggtgggc 3840
tcttcaacat ctcaggctgt ggctggaaca ggacaggatg atctaaaaca cacgtaccat 3900
tggctgtaaa acagtatgag cccagactga cgctgaaatc cctcatgagc caaccttagc 3960
tacaaggtag ggagttctga gggaagccgc gtgctcctca ggagagagct gtttaggttt 4020
tccgatcttt ttgctcaggg gccaaacact gaaggcacgt actgcccaac ccactgagcg 4080
cctgaggcca ttccctcctt ttccgcatgc ctcctgcctc ctgggctatt cctctccacc 4140
cagaaggctg ggaatcccag ctgattccct gacaggagcc gacttcacac acaggtgact 4200
ctcaggcatt ggctcatgtt ttcagccagg gataaaccat cccttcttgg ggctttaagt 4260
ccctggggag ctttccctgt aggtctcctg ggtgttgaga gacaagttgg agaccaacct 4320
ccaatgaatg agccgcggtc attcattaat tcactcacgt aatttactga gtagctgcaa 4380
catgccagcc tctacgttag gttctgcgga taaaggagga ataagacaga gtcaggagaa 4440
ctgttccttg tggtttccgt cccttgggga ccacaggcat cagcagtccc attcaagtca 4500
cctgaggcaa agtgtctgca tcttcgtcca gcgacccttt gcttttcggc tcctagaatc 4560
cttagagtct gaattccttt agctgggaac agctgtcatg gtcacccctg gataacattt 4620
gccaccaagt atagatgctg gatcttgggt tccaggcaga catcatccag gtccatctgg 4680
aactttcagt gatagctgcc ttcagccagc atctttgggg gactctataa tagcagcttg 4740
agatcagtgt ctagaagact gttctgcaat ttgctgccaa atgcatctca ggtttttaaa 4800
gtcattgttt cttgctcatg gtggctcatt tattacatag tcccctcacc ccactaatgg 4860
ataatgggag gaaaagttgc tgcttccttc agcatcaaag cctttccttg ggaatctgcc 4920
tccctccatg gcaggggtgg attcgggagc tgggagtaac caggcaaagt caaccagatg 4980
cctagctcct gctgagaccc aggtcctatg gcagctcctc attagattaa aggagaccac 5040
ttccaaagca ggtgctgcat ggctcaccat catatgcccc aaacaactga aagttggcgg 5100
ttatcaccag actgtgagtt tctggcaagt agcttgggga agctgaataa actctaggcc 5160
cagggctact aaagacttca ggatagaatt ctccatcaaa tatacagcat aagtaaaact 5220
gctctgcact gtttaatcca tttccaaggg gcttagaaaa gctaacaagg gtgtgtcccc 5280
tgtcctgccc caccggtttg ctggctttgt aataacataa gaccattgtg gttgttggtg 5340
tcagatacct tcccatcctg agctctctca cctacctgct ctctctccta gagcaggata 5400
ctggggtact tttaagaagg gtgctccttt taagatgccc agaaaagctg tatttaactc 5460
ttgctatttg taacttgggg atggtctccc ctgccccagg gcacataaga gcaaaggctc 5520
caatggtcag tggatgactc tgcaaaagtg acccccctgtg ccagaagcta tagccctctc 5580
cccaacaggt ctctcttgtt ggccagaggg cctgcttccc atgggcattg caagtgccac 5640
cgtgcggggc ctggctctgc acacccagga aaagtctgca gacccccagc cctccgcaat 5700
aattcaccag accagaagcc actggtgtac agagaacact taaaaaaatg tattttatgt 5760
gaaaaaaaat taaaactctg tatactgtat cagcagcttt gtgtaaaaat ggcaatcaag 5820
agagtctaat atatttaaaa ctttttttaaa aaaaatcttc gcagatcttt gatatcgtac 5880
tgaggtaact tccacgtagc cccttgccac gcggcaccgg tgggccttgg gtccaaaact 5940
gtggctcagc cacatcccaa agggggcaca tgtccctgga gttgcttcca gctgccaagg 6000
cctgtgacag aattcgctgt taagagtttt taattaaaat tattaaattc cttttaataa 6060
caaaaaaaaa aaaaaaaaaa aaaaaaaaaa aaaaaaaaaa aaaaaaa 6108

<210> 172
<211> 567
<212> PRT
<213> Homo sapiens

<400> 172
Met Ala Ser His Val Asp Leu Leu Thr Glu Leu Gln Leu Leu Glu Lys
1               5                   10                  15

Val Pro Thr Leu Glu Arg Leu Arg Ala Ala Gln Lys Arg Arg Ala Gln
            20                  25                  30

Gln Leu Lys Lys Trp Ala Gln Tyr Glu Gln Asp Leu Gln His Arg Lys
        35                  40                  45

Arg Lys His Glu Arg Lys Arg Ser Thr Gly Gly Arg Arg Lys Lys Val
    50                  55                  60

Ser Phe Glu Ala Ser Val Ala Leu Leu Glu Ala Ser Leu Arg Asn Asp
65                  70                  75                  80

Ala Glu Glu Val Arg Tyr Phe Leu Lys Asn Lys Val Ser Pro Asp Leu
                85                  90                  95

Cys Asn Glu Asp Gly Leu Thr Ala Leu His Gln Cys Cys Ile Asp Asn

            100                 105                 110

Phe Glu Glu Ile Val Lys Leu Leu Leu Ser His Gly Ala Asn Val Asn
        115                 120                 125

Ala Lys Asp Asn Glu Leu Trp Thr Pro Leu His Ala Ala Ala Thr Cys
    130                 135                 140

Gly His Ile Asn Leu Val Lys Ile Leu Val Gln Tyr Gly Ala Asp Leu
145                 150                 155                 160

Leu Ala Val Asn Ser Asp Gly Asn Met Pro Tyr Asp Leu Cys Glu Asp
                165                 170                 175

Glu Pro Thr Leu Asp Val Ile Glu Thr Cys Met Ala Tyr Gln Gly Ile
            180                 185                 190

Thr Gln Glu Lys Ile Asn Glu Met Arg Val Ala Pro Glu Gln Gln Met
        195                 200                 205

Ile Ala Asp Ile His Cys Met Ile Ala Ala Gly Gln Asp Leu Asp Trp
    210                 215                 220

Ile Asp Ala Gln Gly Ala Thr Leu Leu His Ile Ala Gly Ala Asn Gly
225                 230                 235                 240

Tyr Leu Arg Ala Ala Glu Leu Leu Leu Asp His Gly Val Arg Val Asp
                245                 250                 255

Val Lys Asp Trp Asp Gly Trp Glu Pro Leu His Ala Ala Ala Phe Trp
            260                 265                 270

Gly Gln Met Gln Met Ala Glu Leu Leu Val Ser His Gly Ala Ser Leu
        275                 280                 285

Ser Ala Arg Thr Ser Met Asp Glu Met Pro Ile Asp Leu Cys Glu Glu
    290                 295                 300

Glu Glu Phe Lys Val Leu Leu Leu Glu Leu Lys His Lys His Asp Val
305                 310                 315                 320

Ile Met Lys Ser Gln Leu Arg His Lys Ser Ser Leu Ser Arg Arg Thr
                325                 330                 335

Ser Ser Ala Gly Ser Arg Gly Lys Val Val Arg Arg Ala Ser Leu Ser
            340                 345                 350

Asp Arg Thr Asn Leu Tyr Arg Lys Glu Tyr Glu Gly Glu Ala Ile Leu
        355                 360                 365

Trp Gln Arg Ser Ala Ala Glu Asp Gln Arg Thr Ser Thr Tyr Asn Gly
    370                 375                 380

Asp Ile Arg Glu Thr Arg Thr Asp Gln Glu Asn Lys Asp Pro Asn Pro
385                 390                 395                 400

Arg Leu Glu Lys Pro Val Leu Leu Ser Glu Phe Pro Thr Lys Ile Pro
                405                 410                 415

Arg Gly Glu Leu Asp Met Pro Val Glu Asn Gly Leu Arg Ala Pro Val
            420                 425                 430

Ser Ala Tyr Gln Tyr Ala Leu Ala Asn Gly Asp Val Trp Lys Val His
        435                 440                 445

Glu Val Pro Asp Tyr Ser Met Ala Tyr Gly Asn Pro Gly Val Ala Asp
    450                 455                 460

Ala Thr Pro Pro Trp Ser Ser Tyr Lys Glu Gln Ser Pro Gln Thr Leu

465                 470                 475                 480

Leu Glu Leu Lys Arg Gln Arg Ala Ala Ala Lys Leu Leu Ser His Pro
                485                 490                 495

Phe Leu Ser Thr His Leu Gly Ser Ser Met Ala Arg Thr Gly Glu Ser
            500                 505                 510

Ser Ser Glu Gly Lys Ala Pro Leu Ile Gly Gly Arg Thr Ser Pro Tyr
        515                 520                 525

Ser Ser Asn Gly Thr Ser Val Tyr Tyr Thr Val Thr Ser Gly Asp Pro
    530                 535                 540

Pro Leu Leu Lys Phe Lys Ala Pro Ile Glu Glu Met Glu Glu Lys Val
545                 550                 555                 560

His Gly Cys Cys Arg Ile Ser
                565


<210> 173
<211> 1011
<212> DNA
<213> Homo sapiens

<400> 173
catggatttc ccttaagaaa aaacagaatg atcaatgata gccacttcag tggttttata 60
ctccttggat tcacagggca gcctcagctt cagatgatga tctctggggt tgtctttttc 120
ttctacacta ttgccttcat gggaaatatg gccatcatcc tattgtcttt cctagatgac 180
catctccaag tccccatgta cttcttcctt agaaatttgg ccatcttgga tctctgttat 240
accacaaata tagtcccaca aatgttggtc agtatctggg gcaaagacaa aagaattacc 300
tttggtgggt gtgcctttca acttttcatt gatgtggcac tgtactcagt tgaatgcatc 360
cttctgtcca tgatgtcata tgatcgactc aatgctatct gcaagcctct gcatcatatg 420
accataatga acctccaact ctgccagggc cttgtggtca tctcctgggt agttggtgtg 480
attaattgca tcataccttc cccttatgcc acgagtcttc ctcgatgtag gaaccaccac 540
ctagaccact tttttgtgtg tgtgaaatgt ctgcaaagat caagattcaa gattgcatgt 600
gtggacacca cagccatgga ggtaaccaca tttgccatgt gcctgattat agttcttgtt 660
cctcttcttc ttattcttgt gtcatatggt ttcattgctg tggctgtact caagatcaag 720
tctgcagcag gaagacaaaa agcatttggg acctgttcct cccatctcgt tgtggtatcc 780
atcttctgtg ggacagttac atacatgtat atacagccag gaaacagtcc aaatcagaat 840
gagggcaaac ttctcagtat attttactcc attgttactc ccagcttgaa cccattaatt 900
tatacggtaa ggaataagga gttcaagggg gccatgaaga ggctaactgg aaaagaaaaa 960
gattgcatgg aaaaaagagg acattgattc ttcctcccag caatttctaa t          1011


<210> 174
<211> 319
<212> PRT
<213> Homo sapiens

<400> 174
Met Ile Asn Asp Ser His Phe Ser Gly Phe Ile Leu Leu Gly Phe Thr
1               5                   10                  15

Gly Gln Pro Gln Leu Gln Met Met Ile Ser Gly Val Val Phe Phe Phe
            20                  25                  30

Tyr Thr Ile Ala Phe Met Gly Asn Met Ala Ile Ile Leu Leu Ser Phe
        35                  40                  45

Leu Asp Asp His Leu Gln Val Pro Met Tyr Phe Phe Leu Arg Asn Leu
    50                  55                  60

Ala Ile Leu Asp Leu Cys Tyr Thr Thr Asn Ile Val Pro Gln Met Leu
65                  70                  75                  80

Val Ser Ile Trp Gly Lys Asp Lys Arg Ile Thr Phe Gly Gly Cys Ala
                85                  90                  95

Phe Gln Leu Phe Ile Asp Val Ala Leu Tyr Ser Val Glu Cys Ile Leu
            100                 105                 110

Leu Ser Met Met Ser Tyr Asp Arg Leu Asn Ala Ile Cys Lys Pro Leu
        115                 120                 125

His His Met Thr Ile Met Asn Leu Gln Leu Cys Gln Gly Leu Val Val
    130                 135                 140

Ile Ser Trp Val Val Gly Val Ile Asn Cys Ile Ile Pro Ser Pro Tyr
145                 150                 155                 160

Ala Thr Ser Leu Pro Arg Cys Arg Asn His His Leu Asp His Phe Phe
                165                 170                 175

Val Cys Val Lys Cys Leu Gln Arg Ser Arg Phe Lys Ile Ala Cys Val
            180                 185                 190

Asp Thr Thr Ala Met Glu Val Thr Thr Phe Ala Met Cys Leu Ile Ile
        195                 200                 205

Val Leu Val Pro Leu Leu Leu Ile Leu Val Ser Tyr Gly Phe Ile Ala
    210                 215                 220

Val Ala Val Leu Lys Ile Lys Ser Ala Ala Gly Arg Gln Lys Ala Phe
225                 230                 235                 240

Gly Thr Cys Ser Ser His Leu Val Val Val Ser Ile Phe Cys Gly Thr
                245                 250                 255

Val Thr Tyr Met Tyr Ile Gln Pro Gly Asn Ser Pro Asn Gln Asn Glu
            260                 265                 270

Gly Lys Leu Leu Ser Ile Phe Tyr Ser Ile Val Thr Pro Ser Leu Asn
        275                 280                 285

Pro Leu Ile Tyr Thr Val Arg Asn Lys Glu Phe Lys Gly Ala Met Lys
    290                 295                 300

Arg Leu Thr Gly Lys Glu Lys Asp Cys Met Glu Lys Arg Gly His
305                 310                 315


<210> 175
<211> 1008
<212> DNA
<213> Homo sapiens

<400> 175
ttcacagctg atattcagaa aatagcaaaa atgatcaatg atagttactt tggttggctt 60
atgctccttg ggttccctgg gaagcctcag ctggagatga tcatctctgg ggttgtcttt 120
ttcttctatg caatttcttt gatgggaaat atggtcctta tcctgctgcc attactggat 180
aaacatctcc aaacccccat atatttcttt cttagaaatc tggctatctt ggatctttgt 240
tacaccacaa atatagtccc acagatgttg gtcaatgcct ggggtaaaga caagaaaatc 300
acttttggtg gctgtgcttt tcaacttttc actaatgtga cgctatgcac ggttgaatgt 360
atgcttctgg ctgtgatgtc atatgaccca ttcaatgctg tctgcaagcc tctggactat 420
atgaccataa tgaaccccca actctgtcaa ggcctggtgg ccatgacctg gttaattggt 480
gtcactaatt gcatgatact ttccccctgt cctgtgagtc ttcctcgatg cggagaccac 540
cacctggatc actatttttg tgaaatatct gcaatggtca aaattgcatg tggggctacc 600
acagtcatgg agttgcattg tgttgttgtt gttgttttca ttttccttgc atcacttctt 660
ctcattcttg tgtcatatgg cttcattgct gtggctgtac tcaagatcaa gtctgcagca 720
ggaagacaaa aagcatttgg gacctgtttc tcccatctca ttgtggtatc catcttctat 780
gggactgtta gatatatgta tatagagcca ggaaacagtc catctcagga tgagggcaaa 840
cttctccata tattttactc cattgttact cccaccttga acccaatccc actaaggaat 900
aaggagttca agtgggccat gaaaaggctt attggaaaag aaaaaggttc tggagacaca 960
ataggtcact aacatctttt tacaagaaat tcctggccgg gcacggtg 1008

<210> 176
<211> 313
<212> PRT
<213> Homo sapiens

<400> 176
Met Ile Asn Asp Ser Tyr Phe Gly Trp Leu Met Leu Leu Gly Phe Pro
1               5                   10                  15

Gly Lys Pro Gln Leu Glu Met Ile Ile Ser Gly Val Val Phe Phe Phe
            20                  25                  30

Tyr Ala Ile Ser Leu Met Gly Asn Met Val Leu Ile Leu Leu Pro Leu
        35                  40                  45

Leu Asp Lys His Leu Gln Thr Pro Ile Tyr Phe Phe Leu Arg Asn Leu
    50                  55                  60

Ala Ile Leu Asp Leu Cys Tyr Thr Thr Asn Ile Val Pro Gln Met Leu
65                  70                  75                  80

Val Asn Ala Trp Gly Lys Asp Lys Lys Ile Thr Phe Gly Gly Cys Ala
                85                  90                  95

Phe Gln Leu Phe Thr Asn Val Thr Leu Cys Thr Val Glu Cys Met Leu
            100                 105                 110

Leu Ala Val Met Ser Tyr Asp Pro Phe Asn Ala Val Cys Lys Pro Leu
        115                 120                 125

Asp Tyr Met Thr Ile Met Asn Pro Gln Leu Cys Gln Gly Leu Val Ala
    130                 135                 140

Met Thr Trp Leu Ile Gly Val Thr Asn Cys Met Ile Leu Ser Pro Cys
145                 150                 155                 160

Pro Val Ser Leu Pro Arg Cys Gly Asp His His Leu Asp His Tyr Phe
                165                 170                 175

Cys Glu Ile Ser Ala Met Val Lys Ile Ala Cys Gly Ala Thr Thr Val
            180                 185                 190

Met Glu Leu His Cys Val Val Val Val Val Phe Ile Phe Leu Ala Ser
        195                 200                 205

Leu Leu Leu Ile Leu Val Ser Tyr Gly Phe Ile Ala Val Ala Val Leu
    210                 215                 220

Lys Ile Lys Ser Ala Ala Gly Arg Gln Lys Ala Phe Gly Thr Cys Phe
225                 230                 235                 240

Ser His Leu Ile Val Val Ser Ile Phe Tyr Gly Thr Val Arg Tyr Met
                245                 250                 255

Tyr Ile Glu Pro Gly Asn Ser Pro Ser Gln Asp Glu Gly Lys Leu Leu
            260                 265                 270

His Ile Phe Tyr Ser Ile Val Thr Pro Thr Leu Asn Pro Ile Pro Leu
        275                 280                 285

Arg Asn Lys Glu Phe Lys Trp Ala Met Lys Arg Leu Ile Gly Lys Glu
    290                 295                 300

Lys Gly Ser Gly Asp Thr Ile Gly His
305                 310


<210> 177
<211> 1050

<212> DNA
<213> Homo sapiens

<400> 177
acttttaaa gattgatatt ttgcccaatg gccaacacat tatcctccct gaattcttgt 60
aatgtgtttc tcctagttct gaacagggtg atgggcatga ccaacagcag tgtcaaggga 120
gacttcatcc tggtgggttt ctctcatcag ccccacctgg aaaagatcct ctttgtggct 180
gttttgatat cctatctcct tacccttgtg ggaaatacag taattattct gatctgctct 240
gtagacccta aactcaagac acccatgtat tttttcttaa ctcacctctc cttagttgat 300
atctgtttta ccaccagtat tgtcccccag ctgctgtgga acctaaaagg acctgacaaa 360
acaatcacat tcctgggttg tgtcatccag ctctacatct ccctggcatt gggctccact 420
gagtgtgtcc tcctggctgt aatggctttt gatcgctatg ctgcagtttg caaacctctc 480
cactataccg ccgtaatgaa ccctcagctg tgccaggctc tggcaggggt tgcgtggctg 540
agtggagtgg gaaacactct tatccagggc actgtcaccc tctggcttcc tcgctgtgga 600
caccgattgc tccaacattt tcttcgtgag gtaccctcca tgattaagct tgcatgtgtg 660
gacatccatg ataatgaggt tcagctcttt gttgcttcac tggtcttgct cctcttgccc 720
ttagtgctaa tactgctgtc ctatggacat atagccaagg tggtcataag gatcaagtca 780
gtccaggcct ggtgcaaagg cctggggaca tgtggatccc atttgatagt agtgtccctc 840
ttctgtggga ccatcacagc tgtctacatc cagtccaaca gttcttatgc ccatgctcat 900
gggaagttca tctccctctt ctatacagtt gtgaccccga ccctcaatcc tctcatctac 960
acactgagga ataatgacgt gaaaggagca ctgcgattat ttaacagaga cttaggcaca 1020
taaaaaatga agcagagtac acagcgctca 1050

<210> 178
<211> 331
<212> PRT
<213> Homo sapiens

<400> 178
Met Ala Asn Thr Leu Ser Ser Leu Asn Ser Cys Asn Val Phe Leu Leu
1               5                   10                  15

Val Leu Asn Arg Val Met Gly Met Thr Asn Ser Ser Val Lys Gly Asp
            20                  25                  30

Phe Ile Leu Val Gly Phe Ser His Gln Pro His Leu Glu Lys Ile Leu
        35                  40                  45

Phe Val Ala Val Leu Ile Ser Tyr Leu Leu Thr Leu Val Gly Asn Thr
    50                  55                  60

Val Ile Ile Leu Ile Cys Ser Val Asp Pro Lys Leu Lys Thr Pro Met
65                  70                  75                  80

Tyr Phe Phe Leu Thr His Leu Ser Leu Val Asp Ile Cys Phe Thr Thr
                85                  90                  95

Ser Ile Val Pro Gln Leu Leu Trp Asn Leu Lys Gly Pro Asp Lys Thr
            100                 105                 110

Ile Thr Phe Leu Gly Cys Val Ile Gln Leu Tyr Ile Ser Leu Ala Leu
        115                 120                 125

Gly Ser Thr Glu Cys Val Leu Leu Ala Val Met Ala Phe Asp Arg Tyr
    130                 135                 140

Ala Ala Val Cys Lys Pro Leu His Tyr Thr Ala Val Met Asn Pro Gln
145                 150                 155                 160

Leu Cys Gln Ala Leu Ala Gly Val Ala Trp Leu Ser Gly Val Gly Asn
                165                 170                 175

Thr Leu Ile Gln Gly Thr Val Thr Leu Trp Leu Pro Arg Cys Gly His
            180                 185                 190

Arg Leu Leu Gln His Phe Leu Arg Glu Val Pro Ser Met Ile Lys Leu
        195                 200                 205

Ala Cys Val Asp Ile His Asp Asn Glu Val Gln Leu Phe Val Ala Ser
    210                 215                 220

Leu Val Leu Leu Leu Leu Pro Leu Val Leu Ile Leu Leu Ser Tyr Gly
225                 230                 235                 240

His Ile Ala Lys Val Val Ile Arg Ile Lys Ser Val Gln Ala Trp Cys
                245                 250                 255

Lys Gly Leu Gly Thr Cys Gly Ser His Leu Ile Val Val Ser Leu Phe
            260                 265                 270

Cys Gly Thr Ile Thr Ala Val Tyr Ile Gln Ser Asn Ser Ser Tyr Ala
        275                 280                 285

His Ala His Gly Lys Phe Ile Ser Leu Phe Tyr Thr Val Val Thr Pro
    290                 295                 300

Thr Leu Asn Pro Leu Ile Tyr Thr Leu Arg Asn Asn Asp Val Lys Gly
305                 310                 315                 320

Ala Leu Arg Leu Phe Asn Arg Asp Leu Gly Thr
                325                 330

<210> 179
<211> 1063
<212> DNA
<213> Homo sapiens

<400> 179
tctctaattc tcagtggctt cctcctactg ttgatgtcta tccctaactg tgggtattta 60
gaggtctcag ctggaatttc acctcccagt gctaacatgt ggatcaacaa tcaaagctcg 120
ctagatgatt ttatcctatt gggattttct gaccgtccct ggctagagac acccctctgt 180
gtaatctttc tggtggccta catcttttcc ctatttggaa atatctccat tatcctagtt 240
tcccatctgg atccccagct tgacagtccc atgtactttt ttgtctctaa tctatccttt 300
ctggacctct gctataccac cagcactgtc ccacagatgc tggtcaacct ccggggacca 360
gaaaagacca ttagctatgg gggttgtgtt gcccaactct atatattttt ggccctgggt 420
tctactgaat gcatacttct agccatcatg gcctttgacc gttacgctgc catatgcaag 480
ccccttcact acccagtcat catgaaccat agacgctgta tccacatggc tgctggcact 540
tggatcagtg gctttgctaa ctcccttgtc cagtccactc tcacagtggt ggccccaaga 600
tgtggacaga gggtgttgga ccatttcttc tgtgaagttc cagccctttt gaaactagcc 660
tgtattgata ttcgtgtgaa tgaaatggag ctcaatgtac taggcgcttt gcttctcctg 720
atgccactca ccctcatcct gggcacttat gtgttcattg ctcaggcagt aatgagaatc 780
tgctctgctg aaagtcgctg gaaggctttc aatacctgtg cctcacattt gctggtggtc 840
tccctcttct acttcacagc catcagtatg tatgtccagc ctccctctag ctattctcat 900
gaccggggga agatcatcat ggctctcttt tatggcattg tcacacccac cctcaaccca 960
ttcatctaca cattgagaaa caaggatgtg aaagctgccc tgagaaggtc actgactaaa 1020
gagttttgga ttaagacaag atgatatctg aaaagaagtc cta                   1063

<210> 180
<211> 336
<212> PRT
<213> Homo sapiens

<400> 180
Met Ser Ile Pro Asn Cys Gly Tyr Leu Glu Val Ser Ala Gly Ile Ser
1               5                   10                  15

Pro Pro Ser Ala Asn Met Trp Ile Asn Asn Gln Ser Ser Leu Asp Asp
            20                  25                  30

Phe Ile Leu Leu Gly Phe Ser Asp Arg Pro Trp Leu Glu Thr Pro Leu
        35                  40                  45

Cys Val Ile Phe Leu Val Ala Tyr Ile Phe Ser Leu Phe Gly Asn Ile
    50                  55                  60

Ser Ile Ile Leu Val Ser His Leu Asp Pro Gln Leu Asp Ser Pro Met
65                  70                  75                  80

Tyr Phe Phe Val Ser Asn Leu Ser Phe Leu Asp Leu Cys Tyr Thr Thr
                85                  90                  95

Ser Thr Val Pro Gln Met Leu Val Asn Leu Arg Gly Pro Glu Lys Thr
            100                 105                 110

Ile Ser Tyr Gly Gly Cys Val Ala Gln Leu Tyr Ile Phe Leu Ala Leu
        115                 120                 125

Gly Ser Thr Glu Cys Ile Leu Leu Ala Ile Met Ala Phe Asp Arg Tyr
    130                 135                 140

Ala Ala Ile Cys Lys Pro Leu His Tyr Pro Val Ile Met Asn His Arg
145                 150                 155                 160

Arg Cys Ile His Met Ala Ala Gly Thr Trp Ile Ser Gly Phe Ala Asn
                165                 170                 175

Ser Leu Val Gln Ser Thr Leu Thr Val Val Ala Pro Arg Cys Gly Gln
            180                 185                 190

Arg Val Leu Asp His Phe Phe Cys Glu Val Pro Ala Leu Leu Lys Leu
        195                 200                 205

Ala Cys Ile Asp Ile Arg Val Asn Glu Met Glu Leu Asn Val Leu Gly
    210                 215                 220

Ala Leu Leu Leu Leu Met Pro Leu Thr Leu Ile Leu Gly Thr Tyr Val
225                 230                 235                 240

Phe Ile Ala Gln Ala Val Met Arg Ile Cys Ser Ala Glu Ser Arg Trp
                245                 250                 255

Lys Ala Phe Asn Thr Cys Ala Ser His Leu Leu Val Val Ser Leu Phe
            260                 265                 270

Tyr Phe Thr Ala Ile Ser Met Tyr Val Gln Pro Pro Ser Ser Tyr Ser
        275                 280                 285

His Asp Arg Gly Lys Ile Ile Met Ala Leu Phe Tyr Gly Ile Val Thr
    290                 295                 300

Pro Thr Leu Asn Pro Phe Ile Tyr Thr Leu Arg Asn Lys Asp Val Lys
305                 310                 315                 320

Ala Ala Leu Arg Arg Ser Leu Thr Lys Glu Phe Trp Ile Lys Thr Arg
                325                 330                 335

<210> 181
<211> 1014
<212> DNA
<213> Homo sapiens

<400> 181
catctttagt gtggtccttg gaagctcatg ggtcaggaaa ataaaaacca gacatgggtg 60
agtgagttca ttctgctggg gatttccagt gattggggca ttcaggtatc cctcttcgcc 120
ctgatcctgg ccatgtattt ggtgactatt ttaggaaaca ccctcattct tcttctgatc 180
agactggaca acaggcttca tacccccatg tacttctccc ttagtgttct gtcatttgtg 240
gacttttgtt atacaaagag tattgtccca caaatgctgt cccacttgct ctcagcccga 300
aagtccatcc cattctacag ttgtgtgctc cagctctatg tttctctggc attgtgtggg 360
tctgagttct tcctgctggg ggccatggcc tatgaccgct acgtggccgt gtgccaccca 420
ctgcactaca cggtcatcat gcatggaggg ctgtgcctgg ggctggcggc cagccgcctg 480

gtggctggct tctcaaattc cctgatggaa acaattatca ccttccagct tttatcacct 540
tccagcttcc tgtgtcacgg tgttatcaat cactttgtct gtgagacctt agcagtgcta 600
cagctagcct gtgtggatgt ccccttcaac aaggtcatgg tggccatctc agggtttctg 660
gtgatcttgc ttccctgttc cctggttcta ttctcctatg cttgcatagt tgccaccatt 720
ttgtgcattc gttctaccca ggtacgctgc aaagcctttg ggacctgtgc ctctcacctc 780
attgtggttt gcatgtgctt tggggctacc atctgcacct acctggggcc acagttggcc 840
tcctcagcag aggaagagaa gatgattgct ctcttctatg gagtggtgtc acccatgttg 900
aaccccttga tctacagctt gaggaataag gaagttacgg ctgctgtccg gaaagtttta 960
gaaagatgca gataaagggt caagactcta agaacctctt gttatctatc atca 1014

<210> 182
<211> 315
<212> PRT
<213> Homo sapiens

<400> 182
Met Gly Gln Glu Asn Lys Asn Gln Thr Trp Val Ser Glu Phe Ile Leu
1               5                   10                  15

Leu Gly Ile Ser Ser Asp Trp Gly Ile Gln Val Ser Leu Phe Ala Leu
            20                  25                  30

Ile Leu Ala Met Tyr Leu Val Thr Ile Leu Gly Asn Thr Leu Ile Leu
        35                  40                  45

Leu Leu Ile Arg Leu Asp Asn Arg Leu His Thr Pro Met Tyr Phe Ser
    50                  55                  60

Leu Ser Val Leu Ser Phe Val Asp Phe Cys Tyr Thr Lys Ser Ile Val
65                  70                  75                  80

Pro Gln Met Leu Ser His Leu Leu Ser Ala Arg Lys Ser Ile Pro Phe
                85                  90                  95

Tyr Ser Cys Val Leu Gln Leu Tyr Val Ser Leu Ala Leu Cys Gly Ser
            100                 105                 110

Glu Phe Phe Leu Leu Gly Ala Met Ala Tyr Asp Arg Tyr Val Ala Val
        115                 120                 125

Cys His Pro Leu His Tyr Thr Val Ile Met His Gly Gly Leu Cys Leu
    130                 135                 140

Gly Leu Ala Ala Ser Arg Leu Val Ala Gly Phe Ser Asn Ser Leu Met
145                 150                 155                 160

Glu Thr Ile Ile Thr Phe Gln Leu Leu Ser Pro Ser Ser Phe Leu Cys
                165                 170                 175

His Gly Val Ile Asn His Phe Val Cys Glu Thr Leu Ala Val Leu Gln
            180                 185                 190

Leu Ala Cys Val Asp Val Pro Phe Asn Lys Val Met Val Ala Ile Ser
        195                 200                 205

Gly Phe Leu Val Ile Leu Leu Pro Cys Ser Leu Val Leu Phe Ser Tyr
    210                 215                 220

Ala Cys Ile Val Ala Thr Ile Leu Cys Ile Arg Ser Thr Gln Val Arg
225                 230                 235                 240

Cys Lys Ala Phe Gly Thr Cys Ala Ser His Leu Ile Val Val Cys Met
                245                 250                 255

Cys Phe Gly Ala Thr Ile Cys Thr Tyr Leu Gly Pro Gln Leu Ala Ser
            260                 265                 270

Ser Ala Glu Glu Glu Lys Met Ile Ala Leu Phe Tyr Gly Val Val Ser

275                    280                    285

Pro Met Leu Asn Pro Leu Ile Tyr Ser Leu Arg Asn Lys Glu Val Thr
    290                    295                    300

Ala Ala Val Arg Lys Val Leu Glu Arg Cys Arg
305                    310                    315

<210> 183
<211> 1151
<212> DNA
<213> Homo sapiens

<400> 183
ggacactggt ttgggccata tggatggtga gcaatgtatg acctgattct gttcattaag 60
ataagcttta tgtctcctac tctaagaaac tcttcaattt tcatcattct caccttttgc 120
ctttaggttt tccgaaggtc aacaatgaaa aacagaacca tgtttggtga gtttattcta 180
ctgggcctta caaatcaacc tgaactccaa gtgatgatat tcatctttct gttcctcacc 240
tacatgctaa gtgtcctagg aaatctgact attatcaccc tcaccttact agacccccac 300
ctccagaccc ccatgtattt cttcctccgg aatttctcct tcttagaaat ttccttcaca 360
tccattttta ttcccagatt tctgaccagc atgacaacag gaaataaagt tatcagcttt 420
gctggctgct tgactcagta tttttttgct atatttcttg gagctaccga gttttacctc 480
ctggcctcca tgtcttatga tcgttatgtg gccatctgca aacccttgca ttacctgact 540
attatgagca gcagagtctg catacaacta gtgttctgct cctggttggg gggattccta 600
gcaatcttac caccaatcat cctgatgacc caggtagatt tctgtgtctc caacattctg 660
aatcactatt actgtgacta tgggcctctc gtggagcttg cctgctcaga cacaagcctc 720
ttagaactga tggtcatcct cttggccgtt gtgactctca tggttactct ggtgctggtg 780
acactttctt acacatacat tatcaggact attctgagga tcccttccgc ccagcaaagg 840
acaaaggcct tttccacttg ttcctcccac atgattgtca tctccctctc ttatggcagc 900
tgcatgttta tgtacattaa tccttctgca aaagaaggag gtgctttcaa caaaggaata 960
gctgtactca ttacttcggt tactccctta ctgaatccct tcatatatac tttaagaaat 1020
cagcaagtga aacaagcttt caaggactca gtcaaaaaga ttgtgaaact ttaaaaaagg 1080
agattacact tcaaaataca ttttcactta acaaatatgc attgaatgtc tatatttcaa 1140
gtgctaaatt g                                                      1151

<210> 184
<211> 309
<212> PRT
<213> Homo sapiens

<400> 184
Met Lys Asn Arg Thr Met Phe Gly Glu Phe Ile Leu Leu Gly Leu Thr
1               5                   10                  15

Asn Gln Pro Glu Leu Gln Val Met Ile Phe Ile Phe Leu Phe Leu Thr
            20                  25                  30

Tyr Met Leu Ser Val Leu Gly Asn Leu Thr Ile Ile Thr Leu Thr Leu
        35                  40                  45

Leu Asp Pro His Leu Gln Thr Pro Met Tyr Phe Phe Leu Arg Asn Phe
    50                  55                  60

Ser Phe Leu Glu Ile Ser Phe Thr Ser Ile Phe Ile Pro Arg Phe Leu
65                  70                  75                  80

Thr Ser Met Thr Thr Gly Asn Lys Val Ile Ser Phe Ala Gly Cys Leu
                85                  90                  95

Thr Gln Tyr Phe Phe Ala Ile Phe Leu Gly Ala Thr Glu Phe Tyr Leu
            100                 105                 110

Leu Ala Ser Met Ser Tyr Asp Arg Tyr Val Ala Ile Cys Lys Pro Leu
        115                 120                 125

His Tyr Leu Thr Ile Met Ser Ser Arg Val Cys Ile Gln Leu Val Phe
    130                 135                 140

Cys Ser Trp Leu Gly Gly Phe Leu Ala Ile Leu Pro Pro Ile Ile Leu
145                 150                 155                 160

Met Thr Gln Val Asp Phe Cys Val Ser Asn Ile Leu Asn His Tyr Tyr
                165                 170                 175

Cys Asp Tyr Gly Pro Leu Val Glu Leu Ala Cys Ser Asp Thr Ser Leu
            180                 185                 190

Leu Glu Leu Met Val Ile Leu Leu Ala Val Val Thr Leu Met Val Thr
        195                 200                 205

Leu Val Leu Val Thr Leu Ser Tyr Thr Tyr Ile Ile Arg Thr Ile Leu
    210                 215                 220

Arg Ile Pro Ser Ala Gln Gln Arg Thr Lys Ala Phe Ser Thr Cys Ser
225                 230                 235                 240

Ser His Met Ile Val Ile Ser Leu Ser Tyr Gly Ser Cys Met Phe Met
                245                 250                 255

Tyr Ile Asn Pro Ser Ala Lys Glu Gly Gly Ala Phe Asn Lys Gly Ile
            260                 265                 270

Ala Val Leu Ile Thr Ser Val Thr Pro Leu Leu Asn Pro Phe Ile Tyr
        275                 280                 285

Thr Leu Arg Asn Gln Gln Val Lys Gln Ala Phe Lys Asp Ser Val Lys
    290                 295                 300

Lys Ile Val Lys Leu
305

<210> 185
<211> 1601
<212> DNA
<213> Homo sapiens

<400> 185
ctggttcttt gagtgagtta ttcctggatt ctaggagctc acagtagagt gtttcagaat 60
ggcaaatatc taaacattag ccggtaattt tatgctccgt atactgggta ctaatttaca 120
taaacatata agtaaagtct acacatatga gactgttttc ttgatagatc atggaaggaa 180
aaatccattc agggaaaaaa aagggaaata ctatataaat gtcaaaaatc cagtcttttt 240
aagagacatt ctctggaaat atctctattt tgaggtgtag tagattatct tacatatata 300
tccactcaca cataccttcc agttagaaca ctgaagcctc atcattgtaa ttaaagcaat 360
aaattttgta aaaatgaaaa ggataattgt gggaggagat tctaaacact ccttttctaa 420
tgagctgctc tgtgtcgcca ggggaaacat ggttgagtaa ggcatcacat ttttgacatg 480
gagcttctga caaataatct caaatttatc attgaccctt ttgtttacag gttctgacac 540
cttagtccaa taccttcaga agaacacatg gaaaatagga aaaattgact taattcatcc 600
tcttggggct cacacagaac cctgagggcc aaaaagtttt atttgtcaca ttcttactca 660
tctacattgt gacgataatg ggcaacctcc ttatcatggt gaccatcatg gccagccagt 720
ccctgggttc ccccatgtac tttttttctgg cttctttatc atttatacat accgtctatt 780
atactgccat tgctcccaaa atgattgttg acctgctctc tgagaaaaag accatttctt 840
ttcagggttg tatggctcaa ctttttatgg atcatttatt tgctggtgct gaggtcattc 900
ttctggtggt aatggcctat gatcaatatg tggccatctg taagcctctt cattatttga 960
tcatcatgaa tcgtcgagtc tgtgttctca tgctgttggt ggcctggatt ggaggctttc 1020
ttcactcatt ggttcaattt ctctttattt atcagctccc tttctgtgga cccaatgtca 1080
ttgacaactt cctgtgtgat ttgtatccct tattgaaact tgcttgcacc aatacctatg 1140
tcactgggct ttctatgata gctaatggtg gagcgatttg tactgtcacc ttcttccctc 1200
tcctgctttc ctatggggtc atattaccct ctcttaagac tcagagtttg gaagggaaat 1260
gcaaagcttt ctacacctgt gcatcccaca tcactgtgat cactttattc tttgtcccct 1320
gcatcttcct gtttgtaagg cccaactcca cctttcccat tgataaatcc atgactgtgg 1380
ttttaacttg tataactccc atgctgaaac cactaatcta tgccctgagg aatgcagaaa 1440
tgaaaagtgc catgaggaaa ctttggagtg aaaaagtaag cttagctgga aaagggctgt 1500
atccctcatg agaatatgac tttcattctt tcacagaagc aaggaataat ttcactatcc 1560
tatcagatta catttctgtt atcattcgcc tttagttatt t 1601

<210> 186
<211> 277
<212> PRT
<213> Homo sapiens

<400> 186
Met Gly Asn Leu Leu Ile Met Val Thr Ile Met Ala Ser Gln Ser Leu
1               5                   10                  15

Gly Ser Pro Met Tyr Phe Phe Leu Ala Ser Leu Ser Phe Ile His Thr
            20                  25                  30

Val Tyr Tyr Thr Ala Ile Ala Pro Lys Met Ile Val Asp Leu Leu Ser
        35                  40                  45

Glu Lys Lys Thr Ile Ser Phe Gln Gly Cys Met Ala Gln Leu Phe Met
    50                  55                  60

Asp His Leu Phe Ala Gly Ala Glu Val Ile Leu Leu Val Val Met Ala
65                  70                  75                  80

Tyr Asp Gln Tyr Val Ala Ile Cys Lys Pro Leu His Tyr Leu Ile Ile
                85                  90                  95

Met Asn Arg Arg Val Cys Val Leu Met Leu Leu Val Ala Trp Ile Gly
            100                 105                 110

Gly Phe Leu His Ser Leu Val Gln Phe Leu Phe Ile Tyr Gln Leu Pro
        115                 120                 125

Phe Cys Gly Pro Asn Val Ile Asp Asn Phe Leu Cys Asp Leu Tyr Pro
    130                 135                 140

Leu Leu Lys Leu Ala Cys Thr Asn Thr Tyr Val Thr Gly Leu Ser Met
145                 150                 155                 160

Ile Ala Asn Gly Gly Ala Ile Cys Thr Val Thr Phe Phe Pro Leu Leu
                165                 170                 175

Leu Ser Tyr Gly Val Ile Leu Pro Ser Leu Lys Thr Gln Ser Leu Glu
            180                 185                 190

Gly Lys Cys Lys Ala Phe Tyr Thr Cys Ala Ser His Ile Thr Val Ile
        195                 200                 205

Thr Leu Phe Phe Val Pro Cys Ile Phe Leu Phe Val Arg Pro Asn Ser
    210                 215                 220

Thr Phe Pro Ile Asp Lys Ser Met Thr Val Val Leu Thr Cys Ile Thr
225                 230                 235                 240

Pro Met Leu Lys Pro Leu Ile Tyr Ala Leu Arg Asn Ala Glu Met Lys
                245                 250                 255

Ser Ala Met Arg Lys Leu Trp Ser Glu Lys Val Ser Leu Ala Gly Lys
            260                 265                 270

Gly Leu Tyr Pro Ser
        275


<210> 187
<211> 1006
<212> DNA
<213> Homo sapiens

<400> 187
ccctttcctc ttgctctttg atgttttgta ggcctgcagc tcccaagcac agaggcatga 60

gtggggagaa tgtcaccaag gtcagcacct tcatcctggt gggcctcccc acggccccag 120
ggctgcagta cctgctcttc ctcctcttcc tgctcaccta cctctttgtc ctggtggaga 180
acctggccat catcctcatc gtctggagca gcacctccct ccacaggccc atgtactact 240
ttctgagctc catgtctttc ctggagatct ggtacgtgtc tgacatcacc cccaagatgc 300
tggagggctt cctcctccag cagaaacgca tctctttcgt cgggtgcatg acgcagctct 360
acttcttcag ctccctggtg tgcaccgagt gtgtgcttct ggcctccatg gcctacgacc 420
gctacgtggc catctgccac ccgctgcgct accacgtcct tgtgaccccg ggctgtgcc 480
tccagctggt gggcttctcc tttgtgagtg gcttcaccat ctccatgatc aaggtctgtt 540
ttatctccag cgtcacgttc tgtggctcca acgtcttgaa ccacttcttc tgtgacattt 600
cccccatcct caagctggcc tgcacggact tctccactgc agagctggtg gatttcatcc 660
tggccttcat catcctggtg tttccgctcc tggccaccat actgtcatat tggcacatca 720
ccctggctgt cctgcgcatc ccctcggcca ccggctgctg gagagccttc tctacctgcg 780
cctctcacct caccgtggtc accgtcttct atacagcctt gcttttcatg tatgtccggc 840
cccaagccat tgattcccag agctccaaca agctcatctc tgccgtgtac actgttgtca 900
cgccaataat taaccctttg atttactgcc tgaggaacaa ggaatttaag gacgccttga 960
aaaaggcctt gggcttgggt caaacttcac actaagacaa ctaaat 1006

<210> 188
<211> 324
<212> PRT
<213> Homo sapiens

<400> 188


<210> 189
<211> 1039
<212> DNA
<213> Homo sapiens

<400> 189
gaatgatgcc cttttgccac aatataatta atatttcctg tgtgaaaaac aactggtcaa 60
atgatgtccg tgcttccctg tacagtttaa tggtgctcat aattctgacc acactcgttg 120
gcaatctgat agttattgtt tctatatcac acttcaaaca acttcatacc ccaacaaatt 180
ggctcattca ttccatggcc actgtggact ttcttctggg gtgtctggtc atgccttaca 240
gtatggtgag atctgctgag cactgttggt attttggaga agtcttctgt aaaattcaca 300
caagcaccga cattatgctg agctcagcct ccattttcca tttgtctttc atctccattg 360
accgctacta tgctgtgtgt gatccactga gatataaagc caagatgaat atcttggtta 420
tttgtgtgat gatcttcatt agttggagtg tccctgctgt ttttgcattt ggaatgatct 480
ttctggagct aaacttcaaa ggcgctgaag agatatatta caaacatgtt cactgcagag 540
gaggttgctc tgtcttcttt agcaaaatat ctggggtact gacctttatg acttcttttt 600
atatacctgg atctattatg ttatgtgtct attacagaat atatcttatc gctaaagaac 660
aggcaagatt aattagtgat gccaatcaga agctccaaat tggattggaa atgaaaaatg 720
gaatttcaca aagcaaagaa aggaaagctg tgaagacatt ggggattgtg atgggagttt 780
tcctaatatg ctggtgccct ttctttatct gtacagtcat ggaccctttt cttcactaca 840
ttattccacc tactttgaat gatgtattga tttggtttgg ctacttgaac tctacattta 900
atccaatggt ttatgcattt ttctatcctt ggtttagaaa agcactgaag atgatgctgt 960
ttggtaaaat tttccaaaaa gattcatcca ggtgtaaatt atttttggaa ttgagttcat 1020
agaattatta tattttact                                               1039


<210> 190
<211> 339
<212> PRT
<213> Homo sapiens

<400> 190
Met Met Pro Phe Cys His Asn Ile Ile Asn Ile Ser Cys Val Lys Asn
1               5                   10                  15

Asn Trp Ser Asn Asp Val Arg Ala Ser Leu Tyr Ser Leu Met Val Leu
            20                  25                  30

Ile Ile Leu Thr Thr Leu Val Gly Asn Leu Ile Val Ile Val Ser Ile
        35                  40                  45

Ser His Phe Lys Gln Leu His Thr Pro Thr Asn Trp Leu Ile His Ser
    50                  55                  60

Met Ala Thr Val Asp Phe Leu Leu Gly Cys Leu Val Met Pro Tyr Ser
65                  70                  75                  80

Met Val Arg Ser Ala Glu His Cys Trp Tyr Phe Gly Glu Val Phe Cys
                85                  90                  95

Lys Ile His Thr Ser Thr Asp Ile Met Leu Ser Ser Ala Ser Ile Phe
            100                 105                 110

His Leu Ser Phe Ile Ser Ile Asp Arg Tyr Tyr Ala Val Cys Asp Pro
        115                 120                 125

Leu Arg Tyr Lys Ala Lys Met Asn Ile Leu Val Ile Cys Val Met Ile
    130                 135                 140

Phe Ile Ser Trp Ser Val Pro Ala Val Phe Ala Phe Gly Met Ile Phe
145                 150                 155                 160

Leu Glu Leu Asn Phe Lys Gly Ala Glu Glu Ile Tyr Tyr Lys His Val
                165                 170                 175

His Cys Arg Gly Gly Cys Ser Val Phe Phe Ser Lys Ile Ser Gly Val
            180                 185                 190

Leu Thr Phe Met Thr Ser Phe Tyr Ile Pro Gly Ser Ile Met Leu Cys
        195                 200                 205

Val Tyr Tyr Arg Ile Tyr Leu Ile Ala Lys Glu Gln Ala Arg Leu Ile
    210                 215                 220

Ser Asp Ala Asn Gln Lys Leu Gln Ile Gly Leu Glu Met Lys Asn Gly
225                 230                 235                 240

Ile Ser Gln Ser Lys Glu Arg Lys Ala Val Lys Thr Leu Gly Ile Val
                245                 250                 255

Met Gly Val Phe Leu Ile Cys Trp Cys Pro Phe Phe Ile Cys Thr Val
            260                 265                 270

Met Asp Pro Phe Leu His Tyr Ile Ile Pro Pro Thr Leu Asn Asp Val
        275                 280                 285

Leu Ile Trp Phe Gly Tyr Leu Asn Ser Thr Phe Asn Pro Met Val Tyr
    290                 295                 300

Ala Phe Phe Tyr Pro Trp Phe Arg Lys Ala Leu Lys Met Met Leu Phe
305                 310                 315                 320

Gly Lys Ile Phe Gln Lys Asp Ser Ser Arg Cys Lys Leu Phe Leu Glu
                325                 330                 335

Leu Ser Ser

<210> 191
<211> 1039
<212> DNA
<213> Homo sapiens

<400> 191
gaatgatgcc cttttgccac aatataatta atatttcctg tgtgaaaaac aactggtcaa 60
atgatgtccg tgcttccctg tacagtttaa tggtgctcat aattctgacc acactcgttg 120
gcaatctgat agttattgtt tctatatcac acttcaaaca acttcatacc ccaacaaatt 180
ggctcattca ttccatggcc actgtggact ttcttctggg gtgtctggtc atgccttaca 240
gtatggtgag atctgctgag cactgttggt attttggaga agtcttctgt aaaattcaca 300
caagcaccga cattatgctg agctcagcct ccattttcca tttgtctttc atctccattg 360
accgctacta tgctgtgtgt gatccactga gatataaagc caagatgaat atcttggtta 420
tttgtgtgat gatcttcatt agttggagtg tccctgctgt ttttgcattt ggaatgatct 480
ttctggagct aaacttcaaa ggcgctgaag agatatatta caaacatgtt cactgcagag 540
gaggttgctc tgtcttcttt agcaaaatat ctggggtact gacctttatg acttcttttt 600
atatacctgg atctattatg ttatgtgtct attacagaat atatcttatc gctaaagaac 660
aggcaagatt aattagtgat gccaatcaga agctccaaat tggattggaa atgaaaaatg 720
gaatttcaca aagcaaagaa aggaaagctg tgaagacatt ggggattgtg atgggagttt 780
tcctaatatg ctggtgccct ttctttatct gtacagtcat ggaccctttt cttcactaca 840

ttattccacc tactttgaat gatgtattga tttggtttgg ctacttgaac tctacattta 900
atccaatggt ttatgcattt ttctatcctt ggtttagaaa agcactgaag atgatgctgt 960
ttggtaaaat tttccaaaaa gattcatcca ggtgtaaatt atttttggaa ttgagttcat 1020
agaattatta tattttact 1039

<210> 192
<211> 339
<212> PRT
<213> Homo sapiens

<400> 192
Met Met Pro Phe Cys His Asn Ile Ile Asn Ile Ser Cys Val Lys Asn
1               5                   10                  15

Asn Trp Ser Asn Asp Val Arg Ala Ser Leu Tyr Ser Leu Met Val Leu
            20                  25                  30

Ile Ile Leu Thr Thr Leu Val Gly Asn Leu Ile Val Ile Val Ser Ile
        35                  40                  45

Ser His Phe Lys Gln Leu His Thr Pro Thr Asn Trp Leu Ile His Ser
    50                  55                  60

Met Ala Thr Val Asp Phe Leu Leu Gly Cys Leu Val Met Pro Tyr Ser
65                  70                  75                  80

Met Val Arg Ser Ala Glu His Cys Trp Tyr Phe Gly Glu Val Phe Cys
                85                  90                  95

Lys Ile His Thr Ser Thr Asp Ile Met Leu Ser Ser Ala Ser Ile Phe
            100                 105                 110

His Leu Ser Phe Ile Ser Ile Asp Arg Tyr Tyr Ala Val Cys Asp Pro
        115                 120                 125

Leu Arg Tyr Lys Ala Lys Met Asn Ile Leu Val Ile Cys Val Met Ile
    130                 135                 140

Phe Ile Ser Trp Ser Val Pro Ala Val Phe Ala Phe Gly Met Ile Phe
145                 150                 155                 160

Leu Glu Leu Asn Phe Lys Gly Ala Glu Glu Ile Tyr Tyr Lys His Val
                165                 170                 175

His Cys Arg Gly Gly Cys Ser Val Phe Phe Ser Lys Ile Ser Gly Val
            180                 185                 190

Leu Thr Phe Met Thr Ser Phe Tyr Ile Pro Gly Ser Ile Met Leu Cys
        195                 200                 205

Val Tyr Tyr Arg Ile Tyr Leu Ile Ala Lys Glu Gln Ala Arg Leu Ile
    210                 215                 220

Ser Asp Ala Asn Gln Lys Leu Gln Ile Gly Leu Glu Met Lys Asn Gly
225                 230                 235                 240

Ile Ser Gln Ser Lys Glu Arg Lys Ala Val Lys Thr Leu Gly Ile Val
                245                 250                 255

Met Gly Val Phe Leu Ile Cys Trp Cys Pro Phe Phe Ile Cys Thr Val
            260                 265                 270

Met Asp Pro Phe Leu His Tyr Ile Ile Pro Pro Thr Leu Asn Asp Val
        275                 280                 285

Leu Ile Trp Phe Gly Tyr Leu Asn Ser Thr Phe Asn Pro Met Val Tyr
    290                 295                 300

Ala Phe Phe Tyr Pro Trp Phe Arg Lys Ala Leu Lys Met Met Leu Phe
305                 310                 315                 320

Gly Lys Ile Phe Gln Lys Asp Ser Ser Arg Cys Lys Leu Phe Leu Glu
                325                 330                 335

Leu Ser Ser

<210> 193
<211> 1033
<212> DNA
<213> Homo sapiens

<400> 193
aaccatgacc agcaattttt cccaacctgt tgtgcagctt tgctatgagg atgtgaatgg 60
atcttgtatt gaaactccct attctcctgg gtcccgggta attctgtaca cggcgtttag 120
ctttgggtct ttgctggctg tatttggaaa tctcttagta atgacttctg ttcttcattt 180
taagcagctg cactctccaa ccaatttttct cattgcctct ctggcctgtg ctgacttctt 240
ggtaggtgtg actgtgatgc ttttcagcat ggtcaggacg gtggagagct gctggtattt 300
tggagccaaa ttttgtactc ttcacagttg ctgtgatgtg gcattttgtt actcttctgt 360
cctccacttg tgcttcatct gcatcgacag gtacattgtg gttactgatc ccctggtcta 420
tgctaccaag ttcaccgtgt ctgtgtcggg aatttgcatc agcgtgtcct ggattctgcc 480
tctcacgtac agcggtgctg tgttctacac aggtgtcaat gatgatgggc tggaggaatt 540
agtaagtgct ctcaactgcg taggtggctg tcaaattatt gtaagtcaag gctgggtgtt 600
gatagatttt ctgttattct tcatacctac ccttgttatg ataattcttt acagtaagat 660
tttcttata gctaaacaac aagctataaa aattgaaact actagtagca aagtagaatc 720
atcctcagag agttataaaa tcagagtggc caagagagag aggaaagcag ctaaaaccct 780
gggggtcacg gtactagcat ttgttatttc atggttaccg tatacagttg atatattaat 840
tgatgccttt atgggcttcc tgacccctgc ctatatctat gaaatttgct gttggagtgc 900
ttattataac tcagccatga atcctttgat ttatgctcta ttttatcctt ggtttaggaa 960
agccataaaa cttattttaa gtggagatgt tttaaaggct agttcatcaa ccattagttt 1020
attttagaa taa                                                     1033

<210> 194
<211> 342
<212> PRT
<213> Homo sapiens

<400> 194
Met Thr Ser Asn Phe Ser Gln Pro Val Val Gln Leu Cys Tyr Glu Asp
1               5                   10                  15

Val Asn Gly Ser Cys Ile Glu Thr Pro Tyr Ser Pro Gly Ser Arg Val
            20                  25                  30

Ile Leu Tyr Thr Ala Phe Ser Phe Gly Ser Leu Leu Ala Val Phe Gly
        35                  40                  45

Asn Leu Leu Val Met Thr Ser Val Leu His Phe Lys Gln Leu His Ser
    50                  55                  60

Pro Thr Asn Phe Leu Ile Ala Ser Leu Ala Cys Ala Asp Phe Leu Val
65                  70                  75                  80

Gly Val Thr Val Met Leu Phe Ser Met Val Arg Thr Val Glu Ser Cys
                85                  90                  95

Trp Tyr Phe Gly Ala Lys Phe Cys Thr Leu His Ser Cys Cys Asp Val
            100                 105                 110

Ala Phe Cys Tyr Ser Ser Val Leu His Leu Cys Phe Ile Cys Ile Asp
        115                 120                 125

Arg Tyr Ile Val Val Thr Asp Pro Leu Val Tyr Ala Thr Lys Phe Thr
    130                 135                 140

Val Ser Val Ser Gly Ile Cys Ile Ser Val Ser Trp Ile Leu Pro Leu
145                 150                 155                 160

Thr Tyr Ser Gly Ala Val Phe Tyr Thr Gly Val Asn Asp Asp Gly Leu
                165                 170                 175

Glu Glu Leu Val Ser Ala Leu Asn Cys Val Gly Gly Cys Gln Ile Ile
            180                 185                 190

Val Ser Gln Gly Trp Val Leu Ile Asp Phe Leu Leu Phe Phe Ile Pro
        195                 200                 205

Thr Leu Val Met Ile Ile Leu Tyr Ser Lys Ile Phe Leu Ile Ala Lys
    210                 215                 220

Gln Gln Ala Ile Lys Ile Glu Thr Thr Ser Ser Lys Val Glu Ser Ser
225                 230                 235                 240

Ser Glu Ser Tyr Lys Ile Arg Val Ala Lys Arg Glu Arg Lys Ala Ala
                245                 250                 255

Lys Thr Leu Gly Val Thr Val Leu Ala Phe Val Ile Ser Trp Leu Pro
            260                 265                 270

Tyr Thr Val Asp Ile Leu Ile Asp Ala Phe Met Gly Phe Leu Thr Pro
        275                 280                 285

Ala Tyr Ile Tyr Glu Ile Cys Cys Trp Ser Ala Tyr Tyr Asn Ser Ala
    290                 295                 300

Met Asn Pro Leu Ile Tyr Ala Leu Phe Tyr Pro Trp Phe Arg Lys Ala
305                 310                 315                 320

Ile Lys Leu Ile Leu Ser Gly Asp Val Leu Lys Ala Ser Ser Ser Thr
                325                 330                 335

Ile Ser Leu Phe Leu Glu
            340


<210> 195
<211> 1045
<212> DNA
<213> Homo sapiens

<400> 195
   cgttatgagc agcaattcat ccctgctggt ggctgtgcag ctgtgctacg cgaacgtgaa 60
   tgggtcctgt gtgaaaatcc ccttctcgcc gggatcccgg gtgattctgt acatagtgtt 120
   tggctttggg gctgtgctgg ctgtgtttgg aaacctcctg gtgatgattt caatcctcca 180
   tttcaagcag ctgcactctc cgaccaattt tctcgttgcc tctctggcct gcgctgattt 240
   cttggtgggt gtgactgtga tgcccttcag catggtcagg acggtggaga gctgctggta 300
   ttttgggagg agtttttgta ctttccacac ctgctgtgat gtggcatttt gttactcttc 360
   tctctttcac ttgtgcttca tctccatcga caggtacatt gcggttactg accccctggt 420
   ctatcctacc aagttcaccg tatctgtgtc aggaatttgc atcagcgtgt cctggatcct 480
   gcccctcatg tacagcggtg ctgtgttcta cacaggtgtc tatgacgatg ggctggagga 540
   attatctgat gccctaaact gtataggagg ttgtcagacc gttgtaaatc aaaactgggt 600
   gttgacagat tttctatcct tctttatacc tacctttatt atgataattc tgtatggtaa 660
   catatttctt gtggctagac gacaggcgaa aaagatagaa aatactggta gcaagacaga 720
   atcatcctca gagagttaca aagccagagt ggccaggaga gagagaaaag cagctaaaac 780
   cctgggggtc acagtggtag catttatgat ttcatggtta ccatatagca ttgattcatt 840
   aattgatgcc tttatgggct ttataacccc tgcctgtatt tatgagattt gctgttggtg 900
   tgcttattat aactcagcca tgaatccttt gatttatgct ttattttacc catggtttag 960
   gaaagcaata aaagttattg taactggtca ggttttaaag aacagttcag caaccatgaa 1020
   tttgttttct gaacatatat aagca                                       1045


<210> 196
<211> 345
<212> PRT

<213> Homo sapiens

<400> 196
Met Ser Ser Asn Ser Ser Leu Leu Val Ala Val Gln Leu Cys Tyr Ala
1               5                   10                  15

Asn Val Asn Gly Ser Cys Val Lys Ile Pro Phe Ser Pro Gly Ser Arg
            20                  25                  30

Val Ile Leu Tyr Ile Val Phe Gly Phe Gly Ala Val Leu Ala Val Phe
        35                  40                  45

Gly Asn Leu Leu Val Met Ile Ser Ile Leu His Phe Lys Gln Leu His
    50                  55                  60

Ser Pro Thr Asn Phe Leu Val Ala Ser Leu Ala Cys Ala Asp Phe Leu
65                  70                  75                  80

Val Gly Val Thr Val Met Pro Phe Ser Met Val Arg Thr Val Glu Ser
                85                  90                  95

Cys Trp Tyr Phe Gly Arg Ser Phe Cys Thr Phe His Thr Cys Cys Asp
            100                 105                 110

Val Ala Phe Cys Tyr Ser Ser Leu Phe His Leu Cys Phe Ile Ser Ile
        115                 120                 125

Asp Arg Tyr Ile Ala Val Thr Asp Pro Leu Val Tyr Pro Thr Lys Phe
    130                 135                 140

Thr Val Ser Val Ser Gly Ile Cys Ile Ser Val Ser Trp Ile Leu Pro
145                 150                 155                 160

Leu Met Tyr Ser Gly Ala Val Phe Tyr Thr Gly Val Tyr Asp Asp Gly
                165                 170                 175

Leu Glu Glu Leu Ser Asp Ala Leu Asn Cys Ile Gly Gly Cys Gln Thr
            180                 185                 190

Val Val Asn Gln Asn Trp Val Leu Thr Asp Phe Leu Ser Phe Phe Ile
        195                 200                 205

Pro Thr Phe Ile Met Ile Ile Leu Tyr Gly Asn Ile Phe Leu Val Ala
    210                 215                 220

Arg Arg Gln Ala Lys Lys Ile Glu Asn Thr Gly Ser Lys Thr Glu Ser
225                 230                 235                 240

Ser Ser Glu Ser Tyr Lys Ala Arg Val Ala Arg Arg Glu Arg Lys Ala
                245                 250                 255

Ala Lys Thr Leu Gly Val Thr Val Val Ala Phe Met Ile Ser Trp Leu
            260                 265                 270

Pro Tyr Ser Ile Asp Ser Leu Ile Asp Ala Phe Met Gly Phe Ile Thr
        275                 280                 285

Pro Ala Cys Ile Tyr Glu Ile Cys Cys Trp Cys Ala Tyr Tyr Asn Ser
    290                 295                 300

Ala Met Asn Pro Leu Ile Tyr Ala Leu Phe Tyr Pro Trp Phe Arg Lys
305                 310                 315                 320

Ala Ile Lys Val Ile Val Thr Gly Gln Val Leu Lys Asn Ser Ser Ala
                325                 330                 335

Thr Met Asn Leu Phe Ser Glu His Ile
            340                 345

<210> 197
<211> 948
<212> DNA
<213> Homo sapiens

<400> 197
cctttgtaaa tggccttggg gaatcacagc accatcaccg agttcctcct ccttgggctg 60
tctgccgacc ccaacatccg ggctctgctc tttgtgctgt tcctggggat ttacctcctg 120
accataatgg aaaacctgat gctgctgctc atgatcaggg ctgattcttg tctccataag 180
cccatgtatt tcttcctgag tcacctctct tttgttgatc tctgcttctc ttcagtcatt 240
gtgcccaaga tgctggagaa cctcctgtca cagaggaaaa ccatttcagt agagggctgc 300
ctggctcagg tcttctttgt gtttgtcact gcagggactg aagcctgcct tctctcaggg 360
atggcctatg accgccatgc tgccatctgc cgcccactac tttatggaca gatcatgggt 420
aaacagctgt atatgcacct tgtgtggggc tcatggggac tgggctttct ggacgcactc 480
atcaatgtcc tcctagctgt aaacatggtc ttttgtgaag ccaaaatcat tcaccactac 540
agctatgaga tgccatccct cctccctctg tcctgctctg atatctccag aagcctcatc 600
gccttgctct gctccactct cctacatggg ctgggaaact tccttttggt cttcttatcc 660
tacacccgta taatctctac catcctaagc atcagctcta cctcgggcag aagcaaggcc 720
ttctccacct gctctgccca cctcactgca gtgacacttt actatggctc aggtttgctc 780
cgccatctca tgccaaactc aggttccccc atagagttga tcttctctgt gcagtatact 840
gtagtcactc ccatgctgaa ttccctcatc tatagcctga aaaataagga agtgaaggta 900
gctctgaaaa gaactttgga aaaatatttg caatatacca gacgttga 948

<210> 198
<211> 312
<212> PRT
<213> Homo sapiens

<400> 198
Met Ala Leu Gly Asn His Ser Thr Ile Thr Glu Phe Leu Leu Leu Gly
1               5                   10                  15

Leu Ser Ala Asp Pro Asn Ile Arg Ala Leu Leu Phe Val Leu Phe Leu
            20                  25                  30

Gly Ile Tyr Leu Leu Thr Ile Met Glu Asn Leu Met Leu Leu Leu Met
        35                  40                  45

Ile Arg Ala Asp Ser Cys Leu His Lys Pro Met Tyr Phe Phe Leu Ser
    50                  55                  60

His Leu Ser Phe Val Asp Leu Cys Phe Ser Ser Val Ile Val Pro Lys
65                  70                  75                  80

Met Leu Glu Asn Leu Leu Ser Gln Arg Lys Thr Ile Ser Val Glu Gly
                85                  90                  95

Cys Leu Ala Gln Val Phe Phe Val Phe Val Thr Ala Gly Thr Glu Ala
            100                 105                 110

Cys Leu Leu Ser Gly Met Ala Tyr Asp Arg His Ala Ala Ile Cys Arg
        115                 120                 125

Pro Leu Leu Tyr Gly Gln Ile Met Gly Lys Gln Leu Tyr Met His Leu
    130                 135                 140

Val Trp Gly Ser Trp Gly Leu Gly Phe Leu Asp Ala Leu Ile Asn Val
145                 150                 155                 160

Leu Leu Ala Val Asn Met Val Phe Cys Glu Ala Lys Ile Ile His His
                165                 170                 175

Tyr Ser Tyr Glu Met Pro Ser Leu Leu Pro Leu Ser Cys Ser Asp Ile
            180                 185                 190

Ser Arg Ser Leu Ile Ala Leu Leu Cys Ser Thr Leu Leu His Gly Leu
        195                 200                 205

Gly Asn Phe Leu Leu Val Phe Leu Ser Tyr Thr Arg Ile Ile Ser Thr
    210                 215                 220

Ile Leu Ser Ile Ser Ser Thr Ser Gly Arg Ser Lys Ala Phe Ser Thr
225                 230                 235                 240

Cys Ser Ala His Leu Thr Ala Val Thr Leu Tyr Tyr Gly Ser Gly Leu
                245                 250                 255

Leu Arg His Leu Met Pro Asn Ser Gly Ser Pro Ile Glu Leu Ile Phe
            260                 265                 270

Ser Val Gln Tyr Thr Val Val Thr Pro Met Leu Asn Ser Leu Ile Tyr
        275                 280                 285

Ser Leu Lys Asn Lys Glu Val Lys Val Ala Leu Lys Arg Thr Leu Glu
    290                 295                 300

Lys Tyr Leu Gln Tyr Thr Arg Arg
305                 310

<210> 199
<211> 1039
<212> DNA
<213> Homo sapiens

<400> 199
gaatgatgcc cttttgccac aatataatta atatttcctg tgtgaaaaac aactggtcaa 60
atgatgtccg tgcttccctg tacagtttaa tggtgctcat aattctgacc acactcgttg 120
gcaatctgat agttattgtt tctatatcac acttcaaaca acttcatacc ccaacaaatt 180
ggctcattca ttccatggcc actgtggact ttcttctggg gtgtctggtc atgccttaca 240
gtatggtgag atctgctgag cactgttggt attttggaga agtcttctgt aaaattcaca 300
caagcaccga cattatgctg agctcagcct ccatttccca tttgtctttc atctccattg 360
accgctacta tgctgtgtgt gatccactga gatataaagc caagatgaat atcttggtta 420
tttgtgtgat gatcttcatt agttggagtg tccctgctgt ttttgcattt ggaatgatct 480
ttctggagct aaacttcaaa ggcgctgaag agatatatta caaacatgtt cactgcagag 540
gaggttgctc tgtcttcttt agcaaaatat ctggggtact gacctttatg acttcttttt 600
atatacctgg atctattatg ttatgtgtct attacagaat atatcttatc gctaaagaac 660
aggcaagatt aattagtgat gccaatcaga agctccaaat tggattggaa atgaaaaatg 720
gaatttcaca aagcaaagaa aggaaagctg tgaagacatt ggggattgtg atgggagttt 780
tcctaatatg ctggtgccct ttctttatct gtacagtcat ggaccctttt cttcactaca 840
ttattccacc tactttgaat gatgtattga tttggtttgg ctacttgaac tctacattta 900
atccaatggt ttatgcattt ttctatcctt ggtttagaaa agcactgaag atgatgctgt 960
ttggtaaaat tttccaaaaa gattcatcca ggtgtaaatt atttttggaa ttgagttcat 1020
agaattatta tattttact                                               1039

<210> 200
<211> 339
<212> PRT
<213> Homo sapiens

<400> 200
Met Met Pro Phe Cys His Asn Ile Ile Asn Ile Ser Cys Val Lys Asn
  1               5                  10                  15

Asn Trp Ser Asn Asp Val Arg Ala Ser Leu Tyr Ser Leu Met Val Leu
             20                  25                  30

Ile Ile Leu Thr Thr Leu Val Gly Asn Leu Ile Val Ile Val Ser Ile
         35                  40                  45

Ser His Phe Lys Gln Leu His Thr Pro Thr Asn Trp Leu Ile His Ser
     50                  55                  60

Met Ala Thr Val Asp Phe Leu Leu Gly Cys Leu Val Met Pro Tyr Ser
 65                  70                  75                  80

Met Val Arg Ser Ala Glu His Cys Trp Tyr Phe Gly Glu Val Phe Cys
                85                  90                  95

Lys Ile His Thr Ser Thr Asp Ile Met Leu Ser Ser Ala Ser Ile Phe
            100                 105                 110

His Leu Ser Phe Ile Ser Ile Asp Arg Tyr Tyr Ala Val Cys Asp Pro
        115                 120                 125

Leu Arg Tyr Lys Ala Lys Met Asn Ile Leu Val Ile Cys Val Met Ile
    130                 135                 140

Phe Ile Ser Trp Ser Val Pro Ala Val Phe Ala Phe Gly Met Ile Phe
145                 150                 155                 160

Leu Glu Leu Asn Phe Lys Gly Ala Glu Glu Ile Tyr Tyr Lys His Val
                165                 170                 175

His Cys Arg Gly Gly Cys Ser Val Phe Phe Ser Lys Ile Ser Gly Val
            180                 185                 190

Leu Thr Phe Met Thr Ser Phe Tyr Ile Pro Gly Ser Ile Met Leu Cys
        195                 200                 205

Val Tyr Tyr Arg Ile Tyr Leu Ile Ala Lys Glu Gln Ala Arg Leu Ile
    210                 215                 220

Ser Asp Ala Asn Gln Lys Leu Gln Ile Gly Leu Glu Met Lys Asn Gly
225                 230                 235                 240

Ile Ser Gln Ser Lys Glu Arg Lys Ala Val Lys Thr Leu Gly Ile Val
                245                 250                 255

Met Gly Val Phe Leu Ile Cys Trp Cys Pro Phe Phe Ile Cys Thr Val
            260                 265                 270

Met Asp Pro Phe Leu His Tyr Ile Ile Pro Pro Thr Leu Asn Asp Val
        275                 280                 285

Leu Ile Trp Phe Gly Tyr Leu Asn Ser Thr Phe Asn Pro Met Val Tyr
    290                 295                 300

Ala Phe Phe Tyr Pro Trp Phe Arg Lys Ala Leu Lys Met Met Leu Phe
305                 310                 315                 320

Gly Lys Ile Phe Gln Lys Asp Ser Ser Arg Cys Lys Leu Phe Leu Glu
                325                 330                 335

Leu Ser Ser

<210> 201
<211> 963
<212> DNA
<213> Homo sapiens

<400> 201
aacatggaaa gcaatcagac ctggatcaca gaagtcatcc tgttgggatt ccaggttgga 60
ccagctctgg agttgttcct ctttgggttt ttcttgctat tctacagctt aaccctgatg 120
ggaaatttgg actctagact gcacacaccc atgtatgtct tcctgtcaca tctggccatt 180
gtggacatgt cctatgcctc gagtactgtc cctaagatgc tagcaaatct tgtgatgcac 240
aaaaaagtca tctcctttgc tccttgcata cttcagactt ttttgtattt ggcgtttgct 300
attacagagt gtctgatttt ggtgatgatg tgctatgatc ggtatgtggc aatctgtcac 360
cccttgcaat acaccctcat tatgaactgg agagtgtgca ctgtcctggc ctcaacttgc 420
tggatattta gctttctctt ggctctggtc catattactc ttattctgag gctgcctttt 480
tgtggccaca aaagatcaac cacttttttt ttgtggccac aaaagatcaa ccactttttc 540
tgtcaaatca tgtccgtatt caaattggcc tgtgctgaca ctaggctcaa ccaggtggtc 600

ctatttgcgg gttctgcgtt catcttagtg gggccgctct gcctggtgct ggtctcctac 660
ttgcacatcc tggtggccat cttgaggatc cagtctgggg agggccgcag aaaggccttc 720
tctacctgct cctcccacct ctgcgtggtg gggcttttct ttggcagcgc cattgtcatg 780
tacatggccc ccaagtcaag ccattctcaa gaacggagga agatcctttc cctgttttac 840
agccttttca acccgatcct gaaccccctc atctacagcc ttaatgcaga ggtgaaaggg 900
gctctaaaga gagtcctttg gaaacagaga tcaattgaag aatcatttga gatttcctga 960
gaa 963

<210> 202
<211> 318
<212> PRT
<213> Homo sapiens

<400> 202
Met Glu Ser Asn Gln Thr Trp Ile Thr Glu Val Ile Leu Leu Gly Phe
1               5                   10                  15

Gln Val Gly Pro Ala Leu Glu Leu Phe Leu Phe Gly Phe Phe Leu Leu
            20                  25                  30

Phe Tyr Ser Leu Thr Leu Met Gly Asn Leu Asp Ser Arg Leu His Thr
        35                  40                  45

Pro Met Tyr Val Phe Leu Ser His Leu Ala Ile Val Asp Met Ser Tyr
    50                  55                  60

Ala Ser Ser Thr Val Pro Lys Met Leu Ala Asn Leu Val Met His Lys
65                  70                  75                  80

Lys Val Ile Ser Phe Ala Pro Cys Ile Leu Gln Thr Phe Leu Tyr Leu
                85                  90                  95

Ala Phe Ala Ile Thr Glu Cys Leu Ile Leu Val Met Met Cys Tyr Asp
            100                 105                 110

Arg Tyr Val Ala Ile Cys His Pro Leu Gln Tyr Thr Leu Ile Met Asn
        115                 120                 125

Trp Arg Val Cys Thr Val Leu Ala Ser Thr Cys Trp Ile Phe Ser Phe
    130                 135                 140

Leu Leu Ala Leu Val His Ile Thr Leu Ile Leu Arg Leu Pro Phe Cys
145                 150                 155                 160

Gly His Lys Arg Ser Thr Thr Phe Phe Leu Trp Pro Gln Lys Ile Asn
                165                 170                 175

His Phe Phe Cys Gln Ile Met Ser Val Phe Lys Leu Ala Cys Ala Asp
            180                 185                 190

Thr Arg Leu Asn Gln Val Val Leu Phe Ala Gly Ser Ala Phe Ile Leu
        195                 200                 205

Val Gly Pro Leu Cys Leu Val Leu Val Ser Tyr Leu His Ile Leu Val
    210                 215                 220

Ala Ile Leu Arg Ile Gln Ser Gly Glu Gly Arg Arg Lys Ala Phe Ser
225                 230                 235                 240

Thr Cys Ser Ser His Leu Cys Val Val Gly Leu Phe Phe Gly Ser Ala
                245                 250                 255

Ile Val Met Tyr Met Ala Pro Lys Ser Ser His Ser Gln Glu Arg Arg
            260                 265                 270

Lys Ile Leu Ser Leu Phe Tyr Ser Leu Phe Asn Pro Ile Leu Asn Pro
        275                 280                 285

Leu Ile Tyr Ser Leu Asn Ala Glu Val Lys Gly Ala Leu Lys Arg Val
    290                 295                 300

Leu Trp Lys Gln Arg Ser Ile Glu Glu Ser Phe Glu Ile Ser
305                 310                 315

<210> 203
<211> 971
<212> DNA
<213> Homo sapiens

<400> 203
tacccttgtt aatggccttt ggggaattcc ccagccccat tcccccgagt tccctcctcc 60
ctttgggctt gttctgcgga ccccccaaac aatcccgggg ctttctgccc ttctttgtgc 120
tgttcctggg gatttacctc ctgaccataa tggaaaacct gatgctgctg ctcatgatca 180
gggctgattc ttgtctccat aagcccatgt atttcttcct gagtcacctc tcttttgttg 240
atctctgctt ctcttcagtc attgtgccca agatgctgga gaacctcctg tcacagagga 300
aaaccatttc agtagagggc tgcctggctc aggtcttctt tgtgtttgtc actgcaggga 360
ctgaagcctg ccttctctca gggatggcct atgaccgcca tgctgccatc tgccgcccac 420
tactttatgg acagatcatg ggtaaacagc tgtatatgca ccttgtgtgg ggctcatggg 480
gactgggctt tctggacgca ctcatcaatg tcctcctagc tgtaaacatg gtcttttgtg 540
aagccaaaat cattcaccac tacagctatg agatgccatc cctcctccct ctgtcctgct 600
ctgatatctc cagaagcctc atcgccttgc tctgctccac tctcctacat gggctgggaa 660
acttcctttt ggtcttctta tcctacaccc gtataatctc taccatccta agcatcagct 720
ctacctcggg cagaagcaag gccttctcca cctgctctgc ccacctcact gcagtgacac 780
tttactatgg ctcaggtttg ctccgccatc tcatgccaaa ctcaggttcc cccatagagt 840
tgatcttctc tgtgcagtat actgtagtca ctcccatgct gaattccctc atctatagcc 900
tgaaaaataa ggaagtgaag gtagctctga aaagaacttt ggaaaaatat ttgcaatata 960
ccagacgttg a                                                      971

<210> 204
<211> 319
<212> PRT
<213> Homo sapiens

<400> 204
Met Ala Phe Gly Glu Phe Pro Ser Pro Ile Pro Pro Ser Ser Leu Leu
1               5                   10                  15

Pro Leu Gly Leu Phe Cys Gly Pro Pro Lys Gln Ser Arg Gly Phe Leu
            20                  25                  30

Pro Phe Phe Val Leu Phe Leu Gly Ile Tyr Leu Leu Thr Ile Met Glu
        35                  40                  45

Asn Leu Met Leu Leu Leu Met Ile Arg Ala Asp Ser Cys Leu His Lys
    50                  55                  60

Pro Met Tyr Phe Phe Leu Ser His Leu Ser Phe Val Asp Leu Cys Phe
65                  70                  75                  80

Ser Ser Val Ile Val Pro Lys Met Leu Glu Asn Leu Leu Ser Gln Arg
                85                  90                  95

Lys Thr Ile Ser Val Glu Gly Cys Leu Ala Gln Val Phe Phe Val Phe
            100                 105                 110

Val Thr Ala Gly Thr Glu Ala Cys Leu Leu Ser Gly Met Ala Tyr Asp
        115                 120                 125

Arg His Ala Ala Ile Cys Arg Pro Leu Leu Tyr Gly Gln Ile Met Gly
    130                 135                 140

Lys Gln Leu Tyr Met His Leu Val Trp Gly Ser Trp Gly Leu Gly Phe
145                 150                 155                 160

Leu Asp Ala Leu Ile Asn Val Leu Leu Ala Val Asn Met Val Phe Cys

                165                 170                 175

Glu Ala Lys Ile Ile His His Tyr Ser Tyr Glu Met Pro Ser Leu Leu
            180                 185                 190

Pro Leu Ser Cys Ser Asp Ile Ser Arg Ser Leu Ile Ala Leu Leu Cys
        195                 200                 205

Ser Thr Leu Leu His Gly Leu Gly Asn Phe Leu Leu Val Phe Leu Ser
    210                 215                 220

Tyr Thr Arg Ile Ile Ser Thr Ile Leu Ser Ile Ser Ser Thr Ser Gly
225                 230                 235                 240

Arg Ser Lys Ala Phe Ser Thr Cys Ser Ala His Leu Thr Ala Val Thr
                245                 250                 255

Leu Tyr Tyr Gly Ser Gly Leu Leu Arg His Leu Met Pro Asn Ser Gly
            260                 265                 270

Ser Pro Ile Glu Leu Ile Phe Ser Val Gln Tyr Thr Val Val Thr Pro
        275                 280                 285

Met Leu Asn Ser Leu Ile Tyr Ser Leu Lys Asn Lys Glu Val Lys Val
    290                 295                 300

Ala Leu Lys Arg Thr Leu Glu Lys Tyr Leu Gln Tyr Thr Arg Arg
305                 310                 315

<210> 205
<211> 1067
<212> DNA
<213> Homo sapiens

<400> 205
ccctttcctc ttgctctttg atgttttgta ggcctgcagc tcccaagcac agaggcatga 60
gtggggagaa tgtcaccagg gtcggcacct tcatcctggt gggcttcccc acggccccag 120
ggctgcagta cctgctcttc ctcctcttcc tgctcaccta cctctttgtc ctggtggaga 180
acctggccat catcctcacc gtctggagca gcacctccct ccacaggccc atgtactact 240
ttctgagctc catgtctttc ctagagatct ggtacgtgtc tgacatcacc cccaagatgc 300
tggagggctt cctcctccag cagaaacgca tctctttcgt cgggtgcatg acgcagctct 360
acttcttcag ctccctggtg tgcaccgagt gtgtgcttct ggcctccatg gcctacgacc 420
gctacgtggc catctgccac ccgctgcgct accacgtcct tgtgaccccg ggcctgtgcc 480
tccagctggt gggcttctcc tttgtgagtg gcttcaccat ctccatgatc aaggtctgtt 540
ttatctccag cgtcacgttc tgtggctcca acgtcttgaa ccacttcttc tgtgacattt 600
cccccatcct caagctggcc tgcacggact tctccactgc agagctggtg gatttcattc 660
tggccttcat catcctggtg tttccactcc tggccaccat gctgtcatat gcgcacatca 720
ccctggctgt cctgcgcatc ccctcggcca ccggctgctg gagagccttc ttcacctgcg 780
cctctcacct caccgtggtc accgtcttct atacagcctt gcttttcatg tatgtccggc 840
cccaggccat tgattcccgg agctccaaca agctcatctc tgttttgtac acagttatca 900
cccccatctt gaaccccttg atatactgcc tgaggaataa ggaatttaag aatgccttga 960
aaaaagcctt cggcttgacg agctgcgccg tagaggggag gctttctagt cttctggaac 1020
ttcatctcca aatacacagc cagcctctct gaggaggcca tttgact 1067

<210> 206
<211> 343
<212> PRT
<213> Homo sapiens

<400> 206
Met Phe Cys Arg Pro Ala Ala Pro Lys His Arg Gly Met Ser Gly Glu
1               5                   10                  15

Asn Val Thr Arg Val Gly Thr Phe Ile Leu Val Gly Phe Pro Thr Ala
            20                  25                  30

Pro Gly Leu Gln Tyr Leu Leu Phe Leu Leu Phe Leu Leu Thr Tyr Leu

35                  40                  45

Phe Val Leu Val Glu Asn Leu Ala Ile Ile Leu Thr Val Trp Ser Ser
    50                  55                  60

Thr Ser Leu His Arg Pro Met Tyr Tyr Phe Leu Ser Ser Met Ser Phe
65                  70                  75                  80

Leu Glu Ile Trp Tyr Val Ser Asp Ile Thr Pro Lys Met Leu Glu Gly
                85                  90                  95

Phe Leu Leu Gln Gln Lys Arg Ile Ser Phe Val Gly Cys Met Thr Gln
            100                 105                 110

Leu Tyr Phe Phe Ser Ser Leu Val Cys Thr Glu Cys Val Leu Leu Ala
        115                 120                 125

Ser Met Ala Tyr Asp Arg Tyr Val Ala Ile Cys His Pro Leu Arg Tyr
    130                 135                 140

His Val Leu Val Thr Pro Gly Leu Cys Leu Gln Leu Val Gly Phe Ser
145                 150                 155                 160

Phe Val Ser Gly Phe Thr Ile Ser Met Ile Lys Val Cys Phe Ile Ser
                165                 170                 175

Ser Val Thr Phe Cys Gly Ser Asn Val Leu Asn His Phe Phe Cys Asp
            180                 185                 190

Ile Ser Pro Ile Leu Lys Leu Ala Cys Thr Asp Phe Ser Thr Ala Glu
        195                 200                 205

Leu Val Asp Phe Ile Leu Ala Phe Ile Ile Leu Val Phe Pro Leu Leu
    210                 215                 220

Ala Thr Met Leu Ser Tyr Ala His Ile Thr Leu Ala Val Leu Arg Ile
225                 230                 235                 240

Pro Ser Ala Thr Gly Cys Trp Arg Ala Phe Phe Thr Cys Ala Ser His
                245                 250                 255

Leu Thr Val Val Thr Val Phe Tyr Thr Ala Leu Leu Phe Met Tyr Val
            260                 265                 270

Arg Pro Gln Ala Ile Asp Ser Arg Ser Ser Asn Lys Leu Ile Ser Val
        275                 280                 285

Leu Tyr Thr Val Ile Thr Pro Ile Leu Asn Pro Leu Ile Tyr Cys Leu
    290                 295                 300

Arg Asn Lys Glu Phe Lys Asn Ala Leu Lys Lys Ala Phe Gly Leu Thr
305                 310                 315                 320

Ser Cys Ala Val Glu Gly Arg Leu Ser Ser Leu Leu Glu Leu His Leu
                325                 330                 335

Gln Ile His Ser Gln Pro Leu
            340

<210> 207
<211> 939
<212> DNA
<213> Homo sapiens

<400> 207
atgtcaggag aaaataattc ctcagtgact gagttcattc tggctgggct ctcagaacag 60
ccagagctcc agctgcccct cttcctcctg ttcttaggaa tctatgtggt cacagtggtg 120
ggcaacctgg gcatgaccac actgatttgg ctcagttctc acctgcacac ccctatgtac 180

tatttcctca gcagtctgtc cttcattgac ttctgccatt ccactgtcat tacccctaag 240
atgctggtga actttgtgac agagaagaac atcatctcct accctgaatg catgactcag 300
ctctacttct tcctcgtttt tgctattgca gagtgtcaca tgttggctgc aatggcgtat 360
gaccgttaca tggccatctg tagccccttg ctgtacagtg tcatcatatc caataaggct 420
tgcttttctc tgattttagg ggtgtatata ataggcctgg tttgtgcatc agttcataca 480
ggctgtatgt ttagggttca attctgcaaa tttgatttga ttaaccatta tttctgtgat 540
cttcttcccc tcctaaagct ctcttgctct agtatctatg tcaacaaact acttattcta 600
tgtgttggtg catttaacat ccttgtcccc agcctgacca tcctttgctc ttacatcttt 660
attattgcca gcatcctcca cattcgctcc actgagggca ggtccaaagc cttcagcact 720
tgtagctccc acatgttggc ggttgtaatc ttttttggat ctgcagcatt catgtacttg 780
cagccatctt caatcagctc catggaccag gggaaagtat cctctgtgtt ttatactatt 840
attgtgccca tgttgaaccc tctgatttat agcctgagga ataaagatgt ccatgtttcc 900
ctgaagaaaa tgctacagag aagaacatta ttgtaaaca 939

<210> 208
<211> 311
<212> PRT
<213> Homo sapiens

<400> 208
Met Ser Gly Glu Asn Asn Ser Ser Val Thr Glu Phe Ile Leu Ala Gly
1               5                   10                  15

Leu Ser Glu Gln Pro Glu Leu Gln Leu Pro Leu Phe Leu Leu Phe Leu
            20                  25                  30

Gly Ile Tyr Val Val Thr Val Val Gly Asn Leu Gly Met Thr Thr Leu
        35                  40                  45

Ile Trp Leu Ser Ser His Leu His Thr Pro Met Tyr Tyr Phe Leu Ser
    50                  55                  60

Ser Leu Ser Phe Ile Asp Phe Cys His Ser Thr Val Ile Thr Pro Lys
65                  70                  75                  80

Met Leu Val Asn Phe Val Thr Glu Lys Asn Ile Ile Ser Tyr Pro Glu
                85                  90                  95

Cys Met Thr Gln Leu Tyr Phe Phe Leu Val Phe Ala Ile Ala Glu Cys
            100                 105                 110

His Met Leu Ala Ala Met Ala Tyr Asp Arg Tyr Met Ala Ile Cys Ser
        115                 120                 125

Pro Leu Leu Tyr Ser Val Ile Ile Ser Asn Lys Ala Cys Phe Ser Leu
    130                 135                 140

Ile Leu Gly Val Tyr Ile Ile Gly Leu Val Cys Ala Ser Val His Thr
145                 150                 155                 160

Gly Cys Met Phe Arg Val Gln Phe Cys Lys Phe Asp Leu Ile Asn His
                165                 170                 175

Tyr Phe Cys Asp Leu Leu Pro Leu Leu Lys Leu Ser Cys Ser Ser Ile
            180                 185                 190

Tyr Val Asn Lys Leu Leu Ile Leu Cys Val Gly Ala Phe Asn Ile Leu
        195                 200                 205

Val Pro Ser Leu Thr Ile Leu Cys Ser Tyr Ile Phe Ile Ile Ala Ser
    210                 215                 220

Ile Leu His Ile Arg Ser Thr Glu Gly Arg Ser Lys Ala Phe Ser Thr
225                 230                 235                 240

Cys Ser Ser His Met Leu Ala Val Val Ile Phe Phe Gly Ser Ala Ala
                245                 250                 255

Phe Met Tyr Leu Gln Pro Ser Ser Ile Ser Ser Met Asp Gln Gly Lys
            260                 265                 270

Val Ser Ser Val Phe Tyr Thr Ile Ile Val Pro Met Leu Asn Pro Leu
        275                 280                 285

Ile Tyr Ser Leu Arg Asn Lys Asp Val His Val Ser Leu Lys Lys Met
    290                 295                 300

Leu Gln Arg Arg Thr Leu Leu
305                 310


<210> 209
<211> 1003
<212> DNA
<213> Homo sapiens

<400> 209
tgttccatac attattttgt cttttgtctg aagcaatgct gaatacaacc tcagtcactg 60
aatttctcct tttgggagtg acagacattc aagaactgca gccttttctc tttgttgttt 120
tccttaccat ctacttcatc agtgtggctg ggaatggagc cattctgatg attgtcatct 180
ctgatcctag actccattcc cctatgtatt tcttcctggg aaacctgtcc tgcctggaca 240
tctgctactc cagcgtaaca ctgccaaaaa tgctgcagaa cttcctctct gcacacaaag 300
caatttcttt cttgggatgc ataagccaac tccatttctt ccacttcctg ggcagcacag 360
aggccatgtt gttggccgtg atggcatttg accgctttgt ggctatttgc aagccacttc 420
gctacactgt cattatgaac cctcagctct gtacccagat ggccatcaca atctggatga 480
ttggtttttt ccatgccctg ctgcactccc taatgacctc tcgcttgaac ttctgtggtt 540
ctaaccgtat ctatcacttc ttctgtgatg tgaagccatt gctaaagctg agccttaatc 600
agtggctgct cagtactgtc acagggacaa tcgccatggg ccccttcttt ctcacattac 660
tctcctattt ctacattatc acccatctct tcttcaagac tcattctttt agcatgctcc 720
gcaaagcact gtccacttgt gcctcccact tcatggtagt tattcttttg tatgcacctg 780
ttctcttcac ctatattcat catgcctcag ggacctccat ggaccaggac cggatcactg 840
ccatcatgta tactgtggtc actccagtac taaacccact gatctacact ttgaggaaca 900
aggaagtgaa aggggccttt aatagagcaa tgaaaaggtg gctttggcct aaagaaatct 960
tgaagaactc ttctgaagca taaataaaca attaaaaaga tga                   1003


<210> 210
<211> 315
<212> PRT
<213> Homo sapiens

<400> 210
Met Leu Asn Thr Thr Ser Val Thr Glu Phe Leu Leu Leu Gly Val Thr
1               5                   10                  15

Asp Ile Gln Glu Leu Gln Pro Phe Leu Phe Val Val Phe Leu Thr Ile
            20                  25                  30

Tyr Phe Ile Ser Val Ala Gly Asn Gly Ala Ile Leu Met Ile Val Ile
        35                  40                  45

Ser Asp Pro Arg Leu His Ser Pro Met Tyr Phe Phe Leu Gly Asn Leu
    50                  55                  60

Ser Cys Leu Asp Ile Cys Tyr Ser Ser Val Thr Leu Pro Lys Met Leu
65                  70                  75                  80

Gln Asn Phe Leu Ser Ala His Lys Ala Ile Ser Phe Leu Gly Cys Ile
                85                  90                  95

Ser Gln Leu His Phe Phe His Phe Leu Gly Ser Thr Glu Ala Met Leu
            100                 105                 110

Leu Ala Val Met Ala Phe Asp Arg Phe Val Ala Ile Cys Lys Pro Leu
        115                 120                 125

Arg Tyr Thr Val Ile Met Asn Pro Gln Leu Cys Thr Gln Met Ala Ile

130                 135                 140

Thr Ile Trp Met Ile Gly Phe Phe His Ala Leu Leu His Ser Leu Met
145                 150                 155                 160

Thr Ser Arg Leu Asn Phe Cys Gly Ser Asn Arg Ile Tyr His Phe Phe
                165                 170                 175

Cys Asp Val Lys Pro Leu Leu Lys Leu Ser Leu Asn Gln Trp Leu Leu
            180                 185                 190

Ser Thr Val Thr Gly Thr Ile Ala Met Gly Pro Phe Phe Leu Thr Leu
        195                 200                 205

Leu Ser Tyr Phe Tyr Ile Ile Thr His Leu Phe Phe Lys Thr His Ser
    210                 215                 220

Phe Ser Met Leu Arg Lys Ala Leu Ser Thr Cys Ala Ser His Phe Met
225                 230                 235                 240

Val Val Ile Leu Leu Tyr Ala Pro Val Leu Phe Thr Tyr Ile His His
                245                 250                 255

Ala Ser Gly Thr Ser Met Asp Gln Asp Arg Ile Thr Ala Ile Met Tyr
            260                 265                 270

Thr Val Val Thr Pro Val Leu Asn Pro Leu Ile Tyr Thr Leu Arg Asn
        275                 280                 285

Lys Glu Val Lys Gly Ala Phe Asn Arg Ala Met Lys Arg Trp Leu Trp
    290                 295                 300

Pro Lys Glu Ile Leu Lys Asn Ser Ser Glu Ala
305                 310                 315


<210> 211
<211> 950
<212> DNA
<213> Homo sapiens

<400> 211
gtaataggaa atgaatgatg atggaaaagt caatgctagc tctgaggggt actttatttt 60
agttggattt tctaattggc cttatctgga agtagttctc tttgtggtta ttttgatctt 120
ctgcttgatg acactgatag gaaacctgtt catcatcatc ctgacgtacc tggactccca 180
tctccatact cccttgtatt tcttcctttc aaatctctca tttctggatc tctgctacac 240
caccagctct atccctcagt tgctggtcag tctctggggt gtggaaaaga ccatttctta 300
tgctggttgc atggttcaac tttacttttt tctcacactg ggaaccacag agtgtgtcct 360
actggtggtg atgtcctatg accgttatgc agctgtgtgt agacctttgc attacactgt 420
cctcatgcac tctcgtttct gccacttgtt ggctgtggct tcttgggtaa gtggttttac 480
aaacccagca cttcattcct ccttcacctt ctgggtacct ctgtgtggac accgccaaat 540
agatcacttt ttctgtgaag ttccggcact tttaagatta tcatttgtca atacccgtga 600
aaataaactg accctcatga tcacaagctc catttttgtt ctgctacttc tcaccctcat 660
tttcacttcc tatggtgcta ttgcccaggc tgtactgagg atgcagtcaa ccactgggct 720
tcagaaagta tttggaacat gtggagctca tcatatggtt gtatctctct ttttcattcc 780
ggccatgtgc atgtatctcc agccaccatc agggaattct caagatcaag gcaagttcat 840
tgctctcttt tatactgttg ttacacctag tcttaaccct ctaatctaca ccctcagaaa 900
caaagatgta agaggggtag tgaagagact aagggggtgg gagtgagcct 950


<210> 212
<211> 311
<212> PRT
<213> Homo sapiens

<400> 212
Met Asn Asp Asp Gly Lys Val Asn Ala Ser Ser Glu Gly Tyr Phe Ile
1               5                   10                  15

Leu Val Gly Phe Ser Asn Trp Pro Tyr Leu Glu Val Val Leu Phe Val
            20                  25                  30

Val Ile Leu Ile Phe Cys Leu Met Thr Leu Ile Gly Asn Leu Phe Ile
        35                  40                  45

Ile Ile Leu Thr Tyr Leu Asp Ser His Leu His Thr Pro Leu Tyr Phe
    50                  55                  60

Phe Leu Ser Asn Leu Ser Phe Leu Asp Leu Cys Tyr Thr Thr Ser Ser
65                  70                  75                  80

Ile Pro Gln Leu Leu Val Ser Leu Trp Gly Val Glu Lys Thr Ile Ser
                85                  90                  95

Tyr Ala Gly Cys Met Val Gln Leu Tyr Phe Phe Leu Thr Leu Gly Thr
            100                 105                 110

Thr Glu Cys Val Leu Leu Val Val Met Ser Tyr Asp Arg Tyr Ala Ala
        115                 120                 125

Val Cys Arg Pro Leu His Tyr Thr Val Leu Met His Ser Arg Phe Cys
    130                 135                 140

His Leu Leu Ala Val Ala Ser Trp Val Ser Gly Phe Thr Asn Pro Ala
145                 150                 155                 160

Leu His Ser Ser Phe Thr Phe Trp Val Pro Leu Cys Gly His Arg Gln
                165                 170                 175

Ile Asp His Phe Phe Cys Glu Val Pro Ala Leu Leu Arg Leu Ser Phe
            180                 185                 190

Val Asn Thr Arg Glu Asn Lys Leu Thr Leu Met Ile Thr Ser Ser Ile
        195                 200                 205

Phe Val Leu Leu Leu Leu Thr Leu Ile Phe Thr Ser Tyr Gly Ala Ile
    210                 215                 220

Ala Gln Ala Val Leu Arg Met Gln Ser Thr Thr Gly Leu Gln Lys Val
225                 230                 235                 240

Phe Gly Thr Cys Gly Ala His His Met Val Val Ser Leu Phe Phe Ile
                245                 250                 255

Pro Ala Met Cys Met Tyr Leu Gln Pro Pro Ser Gly Asn Ser Gln Asp
            260                 265                 270

Gln Gly Lys Phe Ile Ala Leu Phe Tyr Thr Val Val Thr Pro Ser Leu
        275                 280                 285

Asn Pro Leu Ile Tyr Thr Leu Arg Asn Lys Asp Val Arg Gly Val Val
    290                 295                 300

Lys Arg Leu Arg Gly Trp Glu
305                 310


<210> 213
<211> 967
<212> DNA
<213> Homo sapiens

<400> 213
   gcatttgccc cagtagctat gattataatt tgcaatgaca gccacagtga tttcatcctt 60
   ctgggcttct ctaacaagcc acatttggag aagatacttt tttggatcat tttatttttt 120
   tattttttga ctcttgcagg aaatatggtc atagttcttg tgtccttgaa ggatccaaaa 180
   ctccacatcc ctatgtattt ctttctttcc aacctttcct tggtagacct ctgtttgacc 240
   agcagctgtg ttccacagat gttgattaac ttctggggcc cagaaaagac catcagctac 300
   attggctgtg ccattcaact ctatgttttt ttgtggcttg gggccacgga atatgtcctt 360
   cttgttgtca tggctgtgga ttgttatgta gcagtgtgtc atccactgca aaataccatg 420
   atcatgcacc caaaactttg tctgcagctg gctatcttgg catgggggac tggcttggcc 480
   cagtctctga tccagtcccc tgccaccctc cggttaccct tctgctccca gcggatggtg 540
   gatgatgttg tttgtgaagt cccagctctg attcagctct ccagtactga tactacctac 600
   agtgaaattc agatgtctat cgccagtgtt gtcctcctgg tgatgccctt gatcattatc 660
   ctttcctctt ctggtgctat tgctaaggct gtgctgagaa ttaagtcaac tgcaggacag 720
   aagaaagcat ttggcacctg catctctcac cttcttgtgg tttctctctt ttatggcact 780
   gtcacaggtg tctaccttca accaaaaaat cactatcctc atgaatgggg caaatttctc 840
   actcttttct acactgtagt aaccccaact cttaatcccc tcatctacac tctaaggaac 900
   aaggaggtaa agggagcact aataagattg gggaggagga cctgggattc ccagaataac 960
   taacaag 967

<210> 214
<211> 314
<212> PRT
<213> Homo sapiens

<400> 214
Met Ile Ile Ile Cys Asn Asp Ser His Ser Asp Phe Ile Leu Leu Gly
1               5                   10                  15

Phe Ser Asn Lys Pro His Leu Glu Lys Ile Leu Phe Trp Ile Ile Phe
            20                  25                  30

Ile Phe Tyr Phe Leu Thr Leu Ala Gly Asn Met Val Ile Val Leu Val
        35                  40                  45

Ser Leu Lys Asp Pro Lys Leu His Ile Pro Met Tyr Phe Phe Leu Ser
    50                  55                  60

Asn Leu Ser Leu Val Asp Leu Cys Leu Thr Ser Ser Cys Val Pro Gln
65                  70                  75                  80

Met Leu Ile Asn Phe Trp Gly Pro Glu Lys Thr Ile Ser Tyr Ile Gly
                85                  90                  95

Cys Ala Ile Gln Leu Tyr Val Phe Leu Trp Leu Gly Ala Thr Glu Tyr
            100                 105                 110

Val Leu Leu Val Val Met Ala Val Asp Cys Tyr Val Ala Val Cys His
        115                 120                 125

Pro Leu Gln Asn Thr Met Ile Met His Pro Lys Leu Cys Leu Gln Leu
    130                 135                 140

Ala Ile Leu Ala Trp Gly Thr Gly Leu Ala Gln Ser Leu Ile Gln Ser
145                 150                 155                 160

Pro Ala Thr Leu Arg Leu Pro Phe Cys Ser Gln Arg Met Val Asp Asp
                165                 170                 175

Val Val Cys Glu Val Pro Ala Leu Ile Gln Leu Ser Ser Thr Asp Thr
            180                 185                 190

Thr Tyr Ser Glu Ile Gln Met Ser Ile Ala Ser Val Val Leu Leu Val
        195                 200                 205

Met Pro Leu Ile Ile Ile Leu Ser Ser Ser Gly Ala Ile Ala Lys Ala
    210                 215                 220

Val Leu Arg Ile Lys Ser Thr Ala Gly Gln Lys Lys Ala Phe Gly Thr
225                 230                 235                 240

Cys Ile Ser His Leu Leu Val Val Ser Leu Phe Tyr Gly Thr Val Thr
                245                 250                 255

Gly Val Tyr Leu Gln Pro Lys Asn His Tyr Pro His Glu Trp Gly Lys

260                 265                 270

Phe Leu Thr Leu Phe Tyr Thr Val Val Thr Pro Thr Leu Asn Pro Leu
        275                 280                 285

Ile Tyr Thr Leu Arg Asn Lys Glu Val Lys Gly Ala Leu Ile Arg Leu
    290                 295                 300

Gly Arg Arg Thr Trp Asp Ser Gln Asn Asn
305                 310

<210> 215
<211> 23
<212> DNA
<213> Artificial Sequence

<220>
<223> Description of Artificial Sequence:
      oligonucleotide primer

<400> 215
gaaggtttcc ctgggcgttc ctt                                          23

<210> 216
<211> 26
<212> DNA
<213> Artificial Sequence

<220>
<223> Description of Artificial Sequence:
      oligonucleotide primer

<400> 216
gtgaggtgca ggcaaaacca atgatt                                       26

<210> 217
<211> 27
<212> DNA
<213> Artificial Sequence

<220>
<223> Description of Artificial Sequence:
      oligonucleotide primer

<400> 217
gaccccaaga gccttaatga ctctaga                                      27

<210> 218
<211> 24
<212> DNA
<213> Artificial Sequence

<220>
<223> Description of Artificial Sequence:
      oligonucleotide primer

<400> 218
ctgtccgtcg tccttcagag tcat                                         24

<210> 219
<211> 18
<212> DNA
<213> Artificial Sequence

<220>
<223> Description of Artificial Sequence:
      oligonucleotide primer

<400> 219
caaccaagag gcaagagg                                                    18


<210> 220
<211> 24
<212> DNA
<213> Artificial Sequence

<220>
<223> Description of Artificial Sequence:
      oligonucleotide primer

<400> 220
ctccatgaga ctcagtgaat aaga                                             24


<210> 221
<211> 20
<212> DNA
<213> Artificial Sequence

<220>
<223> Description of Artificial Sequence:
      oligonucleotide primer

<400> 221
ctgccttctg ccttatgcca                                                  20


<210> 222
<211> 27
<212> DNA
<213> Artificial Sequence

<220>
<223> Description of Artificial Sequence:
      oligonucleotide primer

<400> 222
ttaagttcta gggtacatgt gcacaac                                          27


<210> 223
<211> 18
<212> DNA
<213> Artificial Sequence

<220>
<223> Description of Artificial Sequence:
      oligonucleotide primer

<400> 223
gcctggtcct gctgactg                                                    18


<210> 224
<211> 27
<212> DNA
<213> Artificial Sequence

<220>
<223> Description of Artificial Sequence:
      oligonucleotide primer

<400> 224
ccgcatcagc ctaggggtac tagagat                                          27

<210> 225
<211> 27
<212> DNA
<213> Artificial Sequence

<220>
<223> Description of Artificial Sequence:
      oligonucleotide primer

<400> 225
ctgtgcactg ttggtgggaa tataaaa                                          27

<210> 226
<211> 28
<212> DNA
<213> Artificial Sequence

<220>
<223> Description of Artificial Sequence:
      oligonucleotide primer

<400> 226
tctggtggtt aagataaaac acaagtca                                         28

<210> 227
<211> 19
<212> DNA
<213> Artificial Sequence

<220>
<223> Description of Artificial Sequence:
      oligonucleotide primer

<400> 227
ttcggctgct gctgaccat                                                   19

<210> 228
<211> 27
<212> DNA
<213> Artificial Sequence

<220>
<223> Description of Artificial Sequence:
      oligonucleotide primer

<400> 228
cctggtagcc tcaaagcttc ttagttc                                          27

<210> 229
<211> 23
<212> DNA
<213> Artificial Sequence

<220>
<223> Description of Artificial Sequence:
      oligonucleotide primer

<400> 229
atggctgccg agaactcctc ctc                                              23

<210> 230
<211> 34
<212> DNA
<213> Artificial Sequence

<220>
<223> Description of Artificial Sequence:
      oligonucleotide primer

<400> 230
tcaagaaaag cttattctgg aaaaggttct cttc                                34


<210> 231
<211> 22
<212> DNA
<213> Artificial Sequence

<220>
<223> Description of Artificial Sequence:
      oligonucleotide primer

<400> 231
aacccctgct gtcatccttc tc                                             22


<210> 232
<211> 27
<212> DNA
<213> Artificial Sequence

<220>
<223> Description of Artificial Sequence:
      oligonucleotide primer

<400> 232
gctacaaaag gtttctttct gatctgc                                        27


<210> 233
<211> 25
<212> DNA
<213> Artificial Sequence

<220>
<223> Description of Artificial Sequence:
      oligonucleotide primer

<400> 233
gtaaacattt ggccagcttg gtttg                                          25


<210> 234
<211> 27
<212> DNA
<213> Artificial Sequence

<220>
<223> Description of Artificial Sequence:
      oligonucleotide primer

<400> 234
cagctgcctg gctaactcct ataacac                                        27


<210> 235
<211> 27
<212> DNA
<213> Artificial Sequence

<220>
<223> Description of Artificial Sequence:
      oligonucleotide primer

<400> 235
aaggtgctga aatagcaatg acaagag                                          27


<210> 236
<211> 24
<212> DNA
<213> Artificial Sequence

<220>
<223> Description of Artificial Sequence:
      oligonucleotide primer

<400> 236
cagagtctct ccctagctcc ccag                                             24


<210> 237
<211> 25
<212> DNA
<213> Artificial Sequence

<220>
<223> Description of Artificial Sequence:
      oligonucleotide primer

<400> 237
ataccccacg ttccgctatg agatt                                            25


<210> 238
<211> 22
<212> DNA
<213> Artificial Sequence

<220>
<223> Description of Artificial Sequence:
      oligonucleotide primer

<400> 238
gtgtcacgtc gagtggttgg tg                                               22


<210> 239
<211> 25
<212> DNA
<213> Artificial Sequence

<220>
<223> Description of Artificial Sequence:
      oligonucleotide primer

<400> 239
cacatagtct tggctccagt ttcgt                                            25


<210> 240
<211> 34
<212> DNA
<213> Artificial Sequence

<220>
<223> Description of Artificial Sequence:
      oligonucleotide primer

<400> 240
ctaaagtttt attccaatca gtgttttttt ttcc                                    34

<210> 241
<211> 22
<212> DNA
<213> Artificial Sequence

<220>
<223> Description of Artificial Sequence:
      oligonucleotide primer

<400> 241
gaatgatgcc cttttgccac aa                                                 22

<210> 242
<211> 34
<212> DNA
<213> Artificial Sequence

<220>
<223> Description of Artificial Sequence:
      oligonucleotide primer

<400> 242
ctatgaactc aattccaaaa ataatttaca cctg                                    34

<210> 243
<211> 21
<212> DNA
<213> Artificial Sequence

<220>
<223> Description of Artificial Sequence:
      oligonucleotide primer

<400> 243
ccttttgtgg gttccatagt c                                                  21

<210> 244
<211> 23
<212> DNA
<213> Artificial Sequence

<220>
<223> Description of Artificial Sequence:
      oligonucleotide primer

<400> 244
tttgtcccca agggccttcc agt                                                23

<210> 245
<211> 22
<212> DNA
<213> Artificial Sequence

<220>
<223> Description of Artificial Sequence:
      oligonucleotide primer

<400> 245
aaaagcaggt attcaacaag ca                                                 22

<210> 246
<211> 22
<212> DNA
<213> Artificial Sequence

<220>
<223> Description of Artificial Sequence:
      oligonucleotide primer

<400> 246
gacaacaaac aagcaaacac aa                                                22

<210> 247
<211> 26
<212> DNA
<213> Artificial Sequence

<220>
<223> Description of Artificial Sequence:
      oligonucleotide primer

<400> 247
accttccact tccttctggt cctgct                                            26

<210> 248
<211> 21
<212> DNA
<213> Artificial Sequence

<220>
<223> Description of Artificial Sequence:
      oligonucleotide primer

<400> 248
gcaggagagg aggaagaaga g                                                 21

<210> 249
<211> 22
<212> DNA
<213> Artificial Sequence

<220>
<223> Description of Artificial Sequence:
      oligonucleotide primer

<400> 249
gaggaggtgg aagaagatga tt                                                22

<210> 250
<211> 26
<212> DNA
<213> Artificial Sequence

<220>
<223> Description of Artificial Sequence:
      oligonucleotide primer

<400> 250
agaatctctt ccgcaagatc ctggct                                            26

<210> 251
<211> 22
<212> DNA

<213> Artificial Sequence

<220>
<223> Description of Artificial Sequence:
      oligonucleotide primer

<400> 251
catcccaata tggagagtca aa                                                22


<210> 252
<211> 22
<212> DNA
<213> Artificial Sequence

<220>
<223> Description of Artificial Sequence:
      oligonucleotide primer

<400> 252
acctttctat gaggaggtgg aa                                                22


<210> 253
<211> 27
<212> DNA
<213> Artificial Sequence

<220>
<223> Description of Artificial Sequence:
      oligonucleotide primer

<400> 253
tgagaaccat gataagaatc tcttccg                                           27


<210> 254
<211> 19
<212> DNA
<213> Artificial Sequence

<220>
<223> Description of Artificial Sequence:
      oligonucleotide primer

<400> 254
gtcaccagcc aggatcttg                                                    19


<210> 255
<211> 22
<212> DNA
<213> Artificial Sequence

<220>
<223> Description of Artificial Sequence:
      oligonucleotide primer

<400> 255
agacgtcaaa cagggaaatc tt                                                22


<210> 256
<211> 29
<212> DNA
<213> Artificial Sequence

<220>
<223> Description of Artificial Sequence:

oligonucleotide primer

<400> 256
cctccaggat ataagatcac tctgattga                                    29


<210> 257
<211> 21
<212> DNA
<213> Artificial Sequence

<220>
<223> Description of Artificial Sequence:
      oligonucleotide primer

<400> 257
tctgtaggtt cctcccatga g                                            21


<210> 258
<211> 29
<212> DNA
<213> Artificial Sequence

<220>
<223> Description of Artificial Sequence:
      oligonucleotide primer

<400> 258
tccaggatat aagatcactc tgattgata                                    29


<210> 259
<211> 35
<212> DNA
<213> Artificial Sequence

<220>
<223> Description of Artificial Sequence:
      oligonucleotide primer

<400> 259
aggttcctcc catgagatat tcaataacaa gtcct                             35


<210> 260
<211> 26
<212> DNA
<213> Artificial Sequence

<220>
<223> Description of Artificial Sequence:
      oligonucleotide primer

<400> 260
aacgtttcct agtataggtg catctg                                       26


<210> 261
<211> 28
<212> DNA
<213> Artificial Sequence

<220>
<223> Description of Artificial Sequence:
      oligonucleotide primer

<400> 261
tcagtgatac aattgccata atttcttt                                     28

<210> 262
<211> 32
<212> DNA
<213> Artificial Sequence

<220>
<223> Description of Artificial Sequence:
      oligonucleotide primer

<400> 262
tttgctccaa atcttagtcc aaatccaatg aa                                32


<210> 263
<211> 27
<212> DNA
<213> Artificial Sequence

<220>
<223> Description of Artificial Sequence:
      oligonucleotide primer

<400> 263
aaaaacatga ttatcatatg catttgc                                      27


<210> 264
<211> 24
<212> DNA
<213> Artificial Sequence

<220>
<223> Description of Artificial Sequence:
      oligonucleotide primer

<400> 264
tttacaagtg aaggcaattt ccaa                                         24


<210> 265
<211> 39
<212> DNA
<213> Artificial Sequence

<220>
<223> Description of Artificial Sequence:
      oligonucleotide primer

<400> 265
agccataata aaatgataac gctggtactt ccatacaat                         39


<210> 266
<211> 23
<212> DNA
<213> Artificial Sequence

<220>
<223> Description of Artificial Sequence:
      oligonucleotide primer

<400> 266
gaggcagaac tggtttctca tga                                          23


<210> 267
<211> 22

<212> DNA
<213> Artificial Sequence

<220>
<223> Description of Artificial Sequence:
      oligonucleotide primer

<400> 267
caagccttcc ttcactgtta ag                                          22


<210> 268
<211> 26
<212> DNA
<213> Artificial Sequence

<220>
<223> Description of Artificial Sequence:
      oligonucleotide primer

<400> 268
ccccagcttc aaacatttct actcaa                                      26


<210> 269
<211> 22
<212> DNA
<213> Artificial Sequence

<220>
<223> Description of Artificial Sequence:
      oligonucleotide primer

<400> 269
gcctcaacag acagtttggt at                                          22


<210> 270
<211> 21
<212> DNA
<213> Artificial Sequence

<220>
<223> Description of Artificial Sequence:
      oligonucleotide primer

<400> 270
ccatttcgga cagtctcaat t                                           21


<210> 271
<211> 30
<212> DNA
<213> Artificial Sequence

<220>
<223> Description of Artificial Sequence:
      oligonucleotide primer

<400> 271
cctcaccttt aagaatagta gctctggaca                                  30


<210> 272
<211> 21
<212> DNA
<213> Artificial Sequence

<220>

<223> Description of Artificial Sequence:
      oligonucleotide primer

<400> 272
aggtgaagct ctgggtcttt t                                                21


<210> 273
<211> 22
<212> DNA
<213> Artificial Sequence

<220>
<223> Description of Artificial Sequence:
      oligonucleotide primer

<400> 273
agccctgttg ttttattgat ga                                               22


<210> 274
<211> 26
<212> DNA
<213> Artificial Sequence

<220>
<223> Description of Artificial Sequence:
      oligonucleotide primer

<400> 274
cctgagcccc aacaaggatt tcatat                                           26


<210> 275
<211> 19
<212> DNA
<213> Artificial Sequence

<220>
<223> Description of Artificial Sequence:
      oligonucleotide primer

<400> 275
cagacgaagg gtcaaatgg                                                   19


<210> 276
<211> 22
<212> DNA
<213> Artificial Sequence

<220>
<223> Description of Artificial Sequence:
      oligonucleotide primer

<400> 276
gcctccaata tgaaacttca aa                                               22


<210> 277
<211> 28
<212> DNA
<213> Artificial Sequence

<220>
<223> Description of Artificial Sequence:
      oligonucleotide primer

<400> 277

tcctcagcta cgagtaagtt ctgtctca                                          28

<210> 278
<211> 22
<212> DNA
<213> Artificial Sequence

<220>
<223> Description of Artificial Sequence:
      oligonucleotide primer

<400> 278
cagaaccatc aggttgtgat tt                                                22

<210> 279
<211> 22
<212> DNA
<213> Artificial Sequence

<220>
<223> Description of Artificial Sequence:
      oligonucleotide primer

<400> 279
cttgtgcctg tggttaacat ct                                                22

<210> 280
<211> 26
<212> DNA
<213> Artificial Sequence

<220>
<223> Description of Artificial Sequence:
      oligonucleotide primer

<400> 280
tgatcactcc aggatattga cacgaa                                            26

<210> 281
<211> 22
<212> DNA
<213> Artificial Sequence

<220>
<223> Description of Artificial Sequence:
      oligonucleotide primer

<400> 281
accccagaac atgtgagtaa ga                                                22

<210> 282
<211> 22
<212> DNA
<213> Artificial Sequence

<220>
<223> Description of Artificial Sequence:
      oligonucleotide primer

<400> 282
ggtcttctac gtcagggaga at                                                22

<210> 283

<211> 26
<212> DNA
<213> Artificial Sequence

<220>
<223> Description of Artificial Sequence:
      oligonucleotide primer

<400> 283
cagtttgcag tcgagacctt cttcga                                          26


<210> 284
<211> 22
<212> DNA
<213> Artificial Sequence

<220>
<223> Description of Artificial Sequence:
      oligonucleotide primer

<400> 284
gctgaatacg tcactgaaac ct                                              22


<210> 285
<211> 22
<212> DNA
<213> Artificial Sequence

<220>
<223> Description of Artificial Sequence:
      oligonucleotide primer

<400> 285
ggaactactg gctggagatt tt                                              22


<210> 286
<211> 26
<212> DNA
<213> Artificial Sequence

<220>
<223> Description of Artificial Sequence:
      oligonucleotide primer

<400> 286
ctgaacctgc tcatgttggc ctttct                                          26


<210> 287
<211> 22
<212> DNA
<213> Artificial Sequence

<220>
<223> Description of Artificial Sequence:
      oligonucleotide primer

<400> 287
aaacataaac gacacccaca ac                                              22


<210> 288
<211> 22
<212> DNA
<213> Artificial Sequence

<220>
<223> Description of Artificial Sequence:
      oligonucleotide primer

<400> 288
tctacaccat cagctgtatg ca                                                22


<210> 289
<211> 26
<212> DNA
<213> Artificial Sequence

<220>
<223> Description of Artificial Sequence:
      oligonucleotide primer

<400> 289
caccaccctc acactcatct tcatca                                            26


<210> 290
<211> 22
<212> DNA
<213> Artificial Sequence

<220>
<223> Description of Artificial Sequence:
      oligonucleotide primer

<400> 290
gcagtgcagc tgtcatatag aa                                                22


<210> 291
<211> 22
<212> DNA
<213> Artificial Sequence

<220>
<223> Description of Artificial Sequence:
      oligonucleotide primer

<400> 291
tggtcataac atcaatccca at                                                22


<210> 292
<211> 26
<212> DNA
<213> Artificial Sequence

<220>
<223> Description of Artificial Sequence:
      oligonucleotide primer

<400> 292
tggcttcatg gtgatattca cactct                                            26


<210> 293
<211> 21
<212> DNA
<213> Artificial Sequence

<220>
<223> Description of Artificial Sequence:
      oligonucleotide primer

<400> 293
gaaagccaac accaaagaaa g                                                21

<210> 294
<211> 21
<212> DNA
<213> Artificial Sequence

<220>
<223> Description of Artificial Sequence:
      oligonucleotide primer

<400> 294
tgtgaatgtg gaggatgcta a                                                21

<210> 295
<211> 22
<212> DNA
<213> Artificial Sequence

<220>
<223> Description of Artificial Sequence:
      oligonucleotide primer

<400> 295
caaacaggga ctgagctgta ac                                               22

<210> 296
<211> 26
<212> DNA
<213> Artificial Sequence

<220>
<223> Description of Artificial Sequence:
      oligonucleotide primer

<400> 296
tacactgttc acgaccagtg tgccat                                           26

<210> 297
<211> 21
<212> DNA
<213> Artificial Sequence

<220>
<223> Description of Artificial Sequence:
      oligonucleotide primer

<400> 297
gcataggtgc tgacttcaca a                                                21

<210> 298
<211> 22
<212> DNA
<213> Artificial Sequence

<220>
<223> Description of Artificial Sequence:
      oligonucleotide primer

<400> 298
ggtgctctgg aagttccagt at                                               22

<210> 299
<211> 26
<212> DNA
<213> Artificial Sequence

<220>
<223> Description of Artificial Sequence:
      oligonucleotide primer

<400> 299
accctcgaca ggtgttcaac ctccta                                          26


<210> 300
<211> 22
<212> DNA
<213> Artificial Sequence

<220>
<223> Description of Artificial Sequence:
      oligonucleotide primer

<400> 300
cttgaataat cggagcccta tc                                              22


<210> 301
<211> 22
<212> DNA
<213> Artificial Sequence

<220>
<223> Description of Artificial Sequence:
      oligonucleotide primer

<400> 301
cgctacagat gttcaagatc ct                                              22


<210> 302
<211> 26
<212> DNA
<213> Artificial Sequence

<220>
<223> Description of Artificial Sequence:
      oligonucleotide primer

<400> 302
ctacagccag agcgtcctct acctgg                                          26


<210> 303
<211> 22
<212> DNA
<213> Artificial Sequence

<220>
<223> Description of Artificial Sequence:
      oligonucleotide primer

<400> 303
cctggaagtc actgaacttg ac                                              22


<210> 304
<211> 22
<212> DNA
<213> Artificial Sequence

<220>
<223> Description of Artificial Sequence:
      oligonucleotide primer

<400> 304
cgctacagat gttcaagatc ct                                              22


<210> 305
<211> 26
<212> DNA
<213> Artificial Sequence

<220>
<223> Description of Artificial Sequence:
      oligonucleotide primer

<400> 305
ctacagccag agcgtcctct acctgg                                          26


<210> 306
<211> 22
<212> DNA
<213> Artificial Sequence

<220>
<223> Description of Artificial Sequence:
      oligonucleotide primer

<400> 306
cctggaagtc actgaacttg ac                                              22


<210> 307
<211> 22
<212> DNA
<213> Artificial Sequence

<220>
<223> Description of Artificial Sequence:
      oligonucleotide primer

<400> 307
ctgaaagggt ccaaggtaca gt                                              22


<210> 308
<211> 26
<212> DNA
<213> Artificial Sequence

<220>
<223> Description of Artificial Sequence:
      oligonucleotide primer

<400> 308
cttcaaaggg tgcaagcccc aagtct                                          26


<210> 309
<211> 22
<212> DNA
<213> Artificial Sequence

<220>
<223> Description of Artificial Sequence:
      oligonucleotide primer

<400> 309
gtactgccag gcttaacacc tt                                                22

<210> 310
<211> 22
<212> DNA
<213> Artificial Sequence

<220>
<223> Description of Artificial Sequence:
      oligonucleotide primer

<400> 310
gaaagggtcc aaggtacagt tc                                                22

<210> 311
<211> 26
<212> DNA
<213> Artificial Sequence

<220>
<223> Description of Artificial Sequence:
      oligonucleotide primer

<400> 311
cttcaaaggg tgcaagcccc aagtct                                            26

<210> 312
<211> 22
<212> DNA
<213> Artificial Sequence

<220>
<223> Description of Artificial Sequence:
      oligonucleotide primer

<400> 312
caggcttaac accttgtgaa ag                                                22

<210> 313
<211> 21
<212> DNA
<213> Artificial Sequence

<220>
<223> Description of Artificial Sequence:
      oligonucleotide primer

<400> 313
acaaccctac gtggatcatt g                                                 21

<210> 314
<211> 30
<212> DNA
<213> Artificial Sequence

<220>
<223> Description of Artificial Sequence:
      oligonucleotide primer

<400> 314
ccctattgat ggaacaacta agtttgtcca                                        30

<210> 315
<211> 22
<212> DNA
<213> Artificial Sequence

<220>
<223> Description of Artificial Sequence:
      oligonucleotide primer

<400> 315
tcgaaacagc tacaaaagga aa                                                22


<210> 316
<211> 22
<212> DNA
<213> Artificial Sequence

<220>
<223> Description of Artificial Sequence:
      oligonucleotide primer

<400> 316
ttcaacagga aggtgattct ca                                                22


<210> 317
<211> 26
<212> DNA
<213> Artificial Sequence

<220>
<223> Description of Artificial Sequence:
      oligonucleotide primer

<400> 317
attttcttct ggtcggccgt cacctt                                            26


<210> 318
<211> 21
<212> DNA
<213> Artificial Sequence

<220>
<223> Description of Artificial Sequence:
      oligonucleotide primer

<400> 318
aagtactgct ggggaatgaa g                                                 21


<210> 319
<211> 22
<212> DNA
<213> Artificial Sequence

<220>
<223> Description of Artificial Sequence:
      oligonucleotide primer

<400> 319
gaacttcctc ccatttcttc ag                                                22


<210> 320
<211> 26
<212> DNA

<213> Artificial Sequence

<220>
<223> Description of Artificial Sequence:
      oligonucleotide primer

<400> 320
aaacccagga gatcagcata agacaa                                          26


<210> 321
<211> 22
<212> DNA
<213> Artificial Sequence

<220>
<223> Description of Artificial Sequence:
      oligonucleotide primer

<400> 321
tagtgggaac aaaggggtag ac                                              22


<210> 322
<211> 22
<212> DNA
<213> Artificial Sequence

<220>
<223> Description of Artificial Sequence:
      oligonucleotide primer

<400> 322
tagttcaaga tgccactttc gt                                              22


<210> 323
<211> 26
<212> DNA
<213> Artificial Sequence

<220>
<223> Description of Artificial Sequence:
      oligonucleotide primer

<400> 323
actgcagact gctcactacc accgag                                          26


<210> 324
<211> 21
<212> DNA
<213> Artificial Sequence

<220>
<223> Description of Artificial Sequence:
      oligonucleotide primer

<400> 324
cgtggtgctc aaattcatac a                                               21


<210> 325
<211> 20
<212> DNA
<213> Artificial Sequence

<220>
<223> Description of Artificial Sequence:

oligonucleotide primer

<400> 325
gagtcaccgt aaggctgtca                                                    20


<210> 326
<211> 25
<212> DNA
<213> Artificial Sequence

<220>
<223> Description of Artificial Sequence:
      oligonucleotide primer

<400> 326
catcccttcg ccatcacagt gtttg                                              25


<210> 327
<211> 22
<212> DNA
<213> Artificial Sequence

<220>
<223> Description of Artificial Sequence:
      oligonucleotide primer

<400> 327
tctgtccagt acaggctgtc tt                                                 22


<210> 328
<211> 21
<212> DNA
<213> Artificial Sequence

<220>
<223> Description of Artificial Sequence:
      oligonucleotide primer

<400> 328
cttcctctct tccaggaaag c                                                  21


<210> 329
<211> 23
<212> DNA
<213> Artificial Sequence

<220>
<223> Description of Artificial Sequence:
      oligonucleotide primer

<400> 329
tctggttcgt cctcacgatg ctg                                                23


<210> 330
<211> 28
<212> DNA
<213> Artificial Sequence

<220>
<223> Description of Artificial Sequence:
      oligonucleotide primer

<400> 330
tgatcacagt ccttatttta ccaaccca                                           28

<210> 331
<211> 21
<212> DNA
<213> Artificial Sequence

<220>
<223> Description of Artificial Sequence:
      oligonucleotide primer

<400> 331
gattcaaaca cagacgcttc a                                                21

<210> 332
<211> 22
<212> DNA
<213> Artificial Sequence

<220>
<223> Description of Artificial Sequence:
      oligonucleotide primer

<400> 332
gccaattaca attgcacaat tt                                               22

<210> 333
<211> 25
<212> DNA
<213> Artificial Sequence

<220>
<223> Description of Artificial Sequence:
      oligonucleotide primer

<400> 333
atgaacactc ctgccccttg gagtt                                            25

<210> 334
<211> 22
<212> DNA
<213> Artificial Sequence

<220>
<223> Description of Artificial Sequence:
      oligonucleotide primer

<400> 334
tcttcacgtg gatagccata ac                                               22

<210> 335
<211> 22
<212> DNA
<213> Artificial Sequence

<220>
<223> Description of Artificial Sequence:
      oligonucleotide primer

<400> 335
tgtcatacag tcccagacat tg                                               22

<210> 336
<211> 26

<212> DNA
<213> Artificial Sequence

<220>
<223> Description of Artificial Sequence:
      oligonucleotide primer

<400> 336
ccttcttctc ccttctcctc ttcctt                                          26


<210> 337
<211> 22
<212> DNA
<213> Artificial Sequence

<220>
<223> Description of Artificial Sequence:
      oligonucleotide primer

<400> 337
ggctggtctt tacacacttg ag                                              22


<210> 338
<211> 23
<212> DNA
<213> Artificial Sequence

<220>
<223> Description of Artificial Sequence:
      oligonucleotide primer

<400> 338
gtggtgtcat tggaaacgat gtg                                             23


<210> 339
<211> 24
<212> DNA
<213> Artificial Sequence

<220>
<223> Description of Artificial Sequence:
      oligonucleotide primer

<400> 339
atacatgaag tcagccgagg gggt                                            24


<210> 340
<211> 19
<212> DNA
<213> Artificial Sequence

<220>
<223> Description of Artificial Sequence:
      oligonucleotide primer

<400> 340
tcctgctcgt cctggtaga                                                  19


<210> 341
<211> 22
<212> DNA
<213> Artificial Sequence

<220>

<223> Description of Artificial Sequence:
      oligonucleotide primer

<400> 341
aaagatgctg aaggagtgaa ga                                                22


<210> 342
<211> 26
<212> DNA
<213> Artificial Sequence

<220>
<223> Description of Artificial Sequence:
      oligonucleotide primer

<400> 342
ccatccccta atacgcagga tggtta                                            26


<210> 343
<211> 22
<212> DNA
<213> Artificial Sequence

<220>
<223> Description of Artificial Sequence:
      oligonucleotide primer

<400> 343
tctgagagag gagtttctcc aa                                                22


<210> 344
<211> 22
<212> DNA
<213> Artificial Sequence

<220>
<223> Description of Artificial Sequence:
      oligonucleotide primer

<400> 344
ctacgatggc aaggattaca tc                                                22


<210> 345
<211> 23
<212> DNA
<213> Artificial Sequence

<220>
<223> Description of Artificial Sequence:
      oligonucleotide primer

<400> 345
ctgaacgagg acctgccctc ctg                                               23


<210> 346
<211> 20
<212> DNA
<213> Artificial Sequence

<220>
<223> Description of Artificial Sequence:
      oligonucleotide primer

<400> 346

cacttgtgct gggagatctg 20

<210> 347
<211> 20
<212> DNA
<213> Artificial Sequence

<220>
<223> Description of Artificial Sequence:
      oligonucleotide primer

<400> 347
cgcagattta ccgagtgaac 20

<210> 348
<211> 26
<212> DNA
<213> Artificial Sequence

<220>
<223> Description of Artificial Sequence:
      oligonucleotide primer

<400> 348
gaccctgctc cgctattaca accaga 26

<210> 349
<211> 20
<212> DNA
<213> Artificial Sequence

<220>
<223> Description of Artificial Sequence:
      oligonucleotide primer

<400> 349
ctggatggtg tgagaaccac 20

<210> 350
<211> 22
<212> DNA
<213> Artificial Sequence

<220>
<223> Description of Artificial Sequence:
      oligonucleotide primer

<400> 350
gtgatgtgca ggaagaactc at 22

<210> 351
<211> 26
<212> DNA
<213> Artificial Sequence

<220>
<223> Description of Artificial Sequence:
      oligonucleotide primer

<400> 351
tttgttctac cccaggcagc aaccat 26

<210> 352

<211> 21
<212> DNA
<213> Artificial Sequence

<220>
<223> Description of Artificial Sequence:
      oligonucleotide primer

<400> 352
ttacaagccg tgagagacac a                                                21


<210> 353
<211> 21
<212> DNA
<213> Artificial Sequence

<220>
<223> Description of Artificial Sequence:
      oligonucleotide primer

<400> 353
ctacgacggc aaggattaca t                                                21


<210> 354
<211> 24
<212> DNA
<213> Artificial Sequence

<220>
<223> Description of Artificial Sequence:
      oligonucleotide primer

<400> 354
ctctgaacga ggacctgcgc tcct                                             24


<210> 355
<211> 20
<212> DNA
<213> Artificial Sequence

<220>
<223> Description of Artificial Sequence:
      oligonucleotide primer

<400> 355
gtgatctgag ctgccatgtc                                                  20


<210> 356
<211> 22
<212> DNA
<213> Artificial Sequence

<220>
<223> Description of Artificial Sequence:
      oligonucleotide primer

<400> 356
aaaagtttcg gtctggctta tc                                               22


<210> 357
<211> 26
<212> DNA
<213> Artificial Sequence

<220>
<223> Description of Artificial Sequence:
      oligonucleotide primer

<400> 357
tttctctctg catacgggct ccagtg                                          26


<210> 358
<211> 22
<212> DNA
<213> Artificial Sequence

<220>
<223> Description of Artificial Sequence:
      oligonucleotide primer

<400> 358
gaccaatggc tggtaagtaa tg                                              22


<210> 359
<211> 22
<212> DNA
<213> Artificial Sequence

<220>
<223> Description of Artificial Sequence:
      oligonucleotide primer

<400> 359
ccagaaccta caagggaaca at                                              22


<210> 360
<211> 30
<212> DNA
<213> Artificial Sequence

<220>
<223> Description of Artificial Sequence:
      oligonucleotide primer

<400> 360
cacaaatgaa gaaattgatc atttcaaacg                                      30


<210> 361
<211> 22
<212> DNA
<213> Artificial Sequence

<220>
<223> Description of Artificial Sequence:
      oligonucleotide primer

<400> 361
ttctacttct tcggctgacc tt                                              22


<210> 362
<211> 22
<212> DNA
<213> Artificial Sequence

<220>
<223> Description of Artificial Sequence:
      oligonucleotide primer

<400> 362
aaagctttgc ttctgactcc at                                                22

<210> 363
<211> 24
<212> DNA
<213> Artificial Sequence

<220>
<223> Description of Artificial Sequence:
      oligonucleotide primer

<400> 363
ctctaccagc ccattgctgt ggct                                              24

<210> 364
<211> 22
<212> DNA
<213> Artificial Sequence

<220>
<223> Description of Artificial Sequence:
      oligonucleotide primer

<400> 364
ttgggatctc aggtccttta gt                                                22

<210> 365
<211> 22
<212> DNA
<213> Artificial Sequence

<220>
<223> Description of Artificial Sequence:
      oligonucleotide primer

<400> 365
tgtactacca gccatggtca ac                                                22

<210> 366
<211> 26
<212> DNA
<213> Artificial Sequence

<220>
<223> Description of Artificial Sequence:
      oligonucleotide primer

<400> 366
catgttcttc aacatcgcca tcaaca                                            26

<210> 367
<211> 22
<212> DNA
<213> Artificial Sequence

<220>
<223> Description of Artificial Sequence:
      oligonucleotide primer

<400> 367
acttgtctgc aaacagttcg aa                                                22

<210> 368
<211> 20
<212> DNA
<213> Artificial Sequence

<220>
<223> Description of Artificial Sequence:
      oligonucleotide primer

<400> 368
atgctctgag cactggagaa                                                    20


<210> 369
<211> 27
<212> DNA
<213> Artificial Sequence

<220>
<223> Description of Artificial Sequence:
      oligonucleotide primer

<400> 369
tcctgctttc acagaattat tccaggg                                            27


<210> 370
<211> 22
<212> DNA
<213> Artificial Sequence

<220>
<223> Description of Artificial Sequence:
      oligonucleotide primer

<400> 370
gtgtgaagtc accactctga ca                                                 22


<210> 371
<211> 22
<212> DNA
<213> Artificial Sequence

<220>
<223> Description of Artificial Sequence:
      oligonucleotide primer

<400> 371
tccagggttt atatgccaga gt                                                 22


<210> 372
<211> 26
<212> DNA
<213> Artificial Sequence

<220>
<223> Description of Artificial Sequence:
      oligonucleotide primer

<400> 372
cacatgtcat gatgacactg gcacaa                                             26


<210> 373
<211> 22
<212> DNA
<213> Artificial Sequence

<220>
<223> Description of Artificial Sequence:
      oligonucleotide primer

<400> 373
cagacttctc ccagtagttg ga                                                22


<210> 374
<211> 22
<212> DNA
<213> Artificial Sequence

<220>
<223> Description of Artificial Sequence:
      oligonucleotide primer

<400> 374
agcaacgggt cacaattcta ta                                                22


<210> 375
<211> 28
<212> DNA
<213> Artificial Sequence

<220>
<223> Description of Artificial Sequence:
      oligonucleotide primer

<400> 375
tcacactgca agcaactcct tatctaga                                          28


<210> 376
<211> 22
<212> DNA
<213> Artificial Sequence

<220>
<223> Description of Artificial Sequence:
      oligonucleotide primer

<400> 376
atacccaaaa gccacaaatt tt                                                22


<210> 377
<211> 22
<212> DNA
<213> Artificial Sequence

<220>
<223> Description of Artificial Sequence:
      oligonucleotide primer

<400> 377
gtgagactgg caagaagatc ac                                                22


<210> 378
<211> 26
<212> DNA
<213> Artificial Sequence

<220>
<223> Description of Artificial Sequence:
      oligonucleotide primer

<400> 378
ctgccaactg cggacaactc taatca                                          26

<210> 379
<211> 22
<212> DNA
<213> Artificial Sequence

<220>
<223> Description of Artificial Sequence:
      oligonucleotide primer

<400> 379
tggttaagat caaacacaag ca                                              22

<210> 380
<211> 20
<212> DNA
<213> Artificial Sequence

<220>
<223> Description of Artificial Sequence:
      oligonucleotide primer

<400> 380
ttggtgataa atgtccctgc                                                 20

<210> 381
<211> 24
<212> DNA
<213> Artificial Sequence

<220>
<223> Description of Artificial Sequence:
      oligonucleotide primer

<400> 381
ggggtgtgtc agggtggtga cttc                                            24

<210> 382
<211> 20
<212> DNA
<213> Artificial Sequence

<220>
<223> Description of Artificial Sequence:
      oligonucleotide primer

<400> 382
ccagtgccat tatggtgtgt                                                 20

<210> 383
<211> 22
<212> DNA
<213> Artificial Sequence

<220>
<223> Description of Artificial Sequence:
      oligonucleotide primer

<400> 383
caaaacaaat gagtcccagt tt                                              22

<210> 384
<211> 23
<212> DNA
<213> Artificial Sequence

<220>
<223> Description of Artificial Sequence:
      oligonucleotide primer

<400> 384
atctgcactg ccatggccaa atg                                              23


<210> 385
<211> 20
<212> DNA
<213> Artificial Sequence

<220>
<223> Description of Artificial Sequence:
      oligonucleotide primer

<400> 385
ctgccaaaga tcacatgctt                                                  20


<210> 386
<211> 22
<212> DNA
<213> Artificial Sequence

<220>
<223> Description of Artificial Sequence:
      oligonucleotide primer

<400> 386
caaccctggt caaataattc aa                                               22


<210> 387
<211> 26
<212> DNA
<213> Artificial Sequence

<220>
<223> Description of Artificial Sequence:
      oligonucleotide primer

<400> 387
aaaataaagc cgcaagaccg tattct                                           26


<210> 388
<211> 22
<212> DNA
<213> Artificial Sequence

<220>
<223> Description of Artificial Sequence:
      oligonucleotide primer

<400> 388
ttttcactcc atgagcatga at                                               22


<210> 389
<211> 22
<212> DNA

<213> Artificial Sequence

<220>
<223> Description of Artificial Sequence:
      oligonucleotide primer

<400> 389
agaccatctt tcccaatctg at                                          22


<210> 390
<211> 24
<212> DNA
<213> Artificial Sequence

<220>
<223> Description of Artificial Sequence:
      oligonucleotide primer

<400> 390
caattccatg gccaaggact cctg                                        24


<210> 391
<211> 22
<212> DNA
<213> Artificial Sequence

<220>
<223> Description of Artificial Sequence:
      oligonucleotide primer

<400> 391
gttccaagtc acctccagtc tt                                          22


<210> 392
<211> 22
<212> DNA
<213> Artificial Sequence

<220>
<223> Description of Artificial Sequence:
      oligonucleotide primer

<400> 392
cagacctatg caacctctgg ta                                          22


<210> 393
<211> 25
<212> DNA
<213> Artificial Sequence

<220>
<223> Description of Artificial Sequence:
      oligonucleotide primer

<400> 393
agctgcgaca tacaagccaa ggcat                                       25


<210> 394
<211> 20
<212> DNA
<213> Artificial Sequence

<220>
<223> Description of Artificial Sequence:

oligonucleotide primer

<400> 394
ggtaaggcag cacaggtatg                                                    20


<210> 395
<211> 22
<212> DNA
<213> Artificial Sequence

<220>
<223> Description of Artificial Sequence:
      oligonucleotide primer

<400> 395
gaggtggtca ctgtgatcac tt                                                 22


<210> 396
<211> 26
<212> DNA
<213> Artificial Sequence

<220>
<223> Description of Artificial Sequence:
      oligonucleotide primer

<400> 396
ttacaactcc agaacccctg gtgctg                                             26


<210> 397
<211> 21
<212> DNA
<213> Artificial Sequence

<220>
<223> Description of Artificial Sequence:
      oligonucleotide primer

<400> 397
ctgagtccga ttggctatga g                                                  21


<210> 398
<211> 22
<212> DNA
<213> Artificial Sequence

<220>
<223> Description of Artificial Sequence:
      oligonucleotide primer

<400> 398
gcaacaaatt cctgaagatc ag                                                 22


<210> 399
<211> 26
<212> DNA
<213> Artificial Sequence

<220>
<223> Description of Artificial Sequence:
      oligonucleotide primer

<400> 399
cctcagctga actcaaaagc catcaa                                             26

<210> 400
<211> 19
<212> DNA
<213> Artificial Sequence

<220>
<223> Description of Artificial Sequence:
      oligonucleotide primer

<400> 400
atcagaggga aggcaaggt                                                    19


<210> 401
<211> 22
<212> DNA
<213> Artificial Sequence

<220>
<223> Description of Artificial Sequence:
      oligonucleotide primer

<400> 401
agcatgtaca tgtttccttt gc                                                22


<210> 402
<211> 26
<212> DNA
<213> Artificial Sequence

<220>
<223> Description of Artificial Sequence:
      oligonucleotide primer

<400> 402
atgcactttt ccagtctgca gaagcg                                            26


<210> 403
<211> 22
<212> DNA
<213> Artificial Sequence

<220>
<223> Description of Artificial Sequence:
      oligonucleotide primer

<400> 403
tgcaacattt cacttccata ca                                                22


<210> 404
<211> 22
<212> DNA
<213> Artificial Sequence

<220>
<223> Description of Artificial Sequence:
      oligonucleotide primer

<400> 404
atctacctct ccgagtcctt ca                                                22


<210> 405
<211> 26

<212> DNA
<213> Artificial Sequence

<220>
<223> Description of Artificial Sequence:
      oligonucleotide primer

<400> 405
ctgcagatcc tgatttcggt gctcaa                                          26


<210> 406
<211> 22
<212> DNA
<213> Artificial Sequence

<220>
<223> Description of Artificial Sequence:
      oligonucleotide primer

<400> 406
gatggtcagt ccgaagatgt ac                                              22


<210> 407
<211> 22
<212> DNA
<213> Artificial Sequence

<220>
<223> Description of Artificial Sequence:
      oligonucleotide primer

<400> 407
atttcctacc ccgtcattaa ag                                              22


<210> 408
<211> 25
<212> DNA
<213> Artificial Sequence

<220>
<223> Description of Artificial Sequence:
      oligonucleotide primer

<400> 408
caccatcggc tttggacaga tcaag                                           25


<210> 409
<211> 21
<212> DNA
<213> Artificial Sequence

<220>
<223> Description of Artificial Sequence:
      oligonucleotide primer

<400> 409
gttctgtagt cccagcaggt t                                               21


<210> 410
<211> 21
<212> DNA
<213> Artificial Sequence

<220>

<223> Description of Artificial Sequence:
      oligonucleotide primer

<400> 410
gttcctgctg gacttcattt c                                                21


<210> 411
<211> 26
<212> DNA
<213> Artificial Sequence

<220>
<223> Description of Artificial Sequence:
      oligonucleotide primer

<400> 411
accccgtcat taaaggcttc acctct                                           26


<210> 412
<211> 22
<212> DNA
<213> Artificial Sequence

<220>
<223> Description of Artificial Sequence:
      oligonucleotide primer

<400> 412
caggttcttg atctgtccaa ag                                               22


<210> 413
<211> 21
<212> DNA
<213> Artificial Sequence

<220>
<223> Description of Artificial Sequence:
      oligonucleotide primer

<400> 413
gttcctgctg gacttcattt c                                                21


<210> 414
<211> 26
<212> DNA
<213> Artificial Sequence

<220>
<223> Description of Artificial Sequence:
      oligonucleotide primer

<400> 414
accccgtcat taaaggcttc acctct                                           26


<210> 415
<211> 22
<212> DNA
<213> Artificial Sequence

<220>
<223> Description of Artificial Sequence:
      oligonucleotide primer

<400> 415

caggttcttg atctgtccaa ag                                                22

<210> 416
<211> 22
<212> DNA
<213> Artificial Sequence

<220>
<223> Description of Artificial Sequence:
      oligonucleotide primer

<400> 416
ctcatatttc tgggcaagaa ga                                                22

<210> 417
<211> 26
<212> DNA
<213> Artificial Sequence

<220>
<223> Description of Artificial Sequence:
      oligonucleotide primer

<400> 417
tgccagtctt cacaattaca gtgtca                                            26

<210> 418
<211> 22
<212> DNA
<213> Artificial Sequence

<220>
<223> Description of Artificial Sequence:
      oligonucleotide primer

<400> 418
cgatggctat taaatcctgg tt                                                22

<210> 419
<211> 22
<212> DNA
<213> Artificial Sequence

<220>
<223> Description of Artificial Sequence:
      oligonucleotide primer

<400> 419
tggcaccttc taatgttcaa at                                                22

<210> 420
<211> 26
<212> DNA
<213> Artificial Sequence

<220>
<223> Description of Artificial Sequence:
      oligonucleotide primer

<400> 420
tttccaggta ctcagatggt accctg                                            26

<210> 421

<211> 22
<212> DNA
<213> Artificial Sequence

<220>
<223> Description of Artificial Sequence:
      oligonucleotide primer

<400> 421
cgcgtgttaa atacgtttga ag                                          22


<210> 422
<211> 22
<212> DNA
<213> Artificial Sequence

<220>
<223> Description of Artificial Sequence:
      oligonucleotide primer

<400> 422
ttctacatgg ctcctgaagt ct                                          22


<210> 423
<211> 26
<212> DNA
<213> Artificial Sequence

<220>
<223> Description of Artificial Sequence:
      oligonucleotide primer

<400> 423
actacacagc caaggcggac atcttt                                      26


<210> 424
<211> 22
<212> DNA
<213> Artificial Sequence

<220>
<223> Description of Artificial Sequence:
      oligonucleotide primer

<400> 424
tctttctatc attgcccaga tg                                          22


<210> 425
<211> 22
<212> DNA
<213> Artificial Sequence

<220>
<223> Description of Artificial Sequence:
      oligonucleotide primer

<400> 425
gaccagcttt gactatgaat gc                                          22


<210> 426
<211> 29
<212> DNA
<213> Artificial Sequence

<220>
<223> Description of Artificial Sequence:
      oligonucleotide primer

<400> 426
tgttcatcag actttaccct accagtcgg                                    29


<210> 427
<211> 22
<212> DNA
<213> Artificial Sequence

<220>
<223> Description of Artificial Sequence:
      oligonucleotide primer

<400> 427
ggagttgaac gtatccactg aa                                           22


<210> 428
<211> 21
<212> DNA
<213> Artificial Sequence

<220>
<223> Description of Artificial Sequence:
      oligonucleotide primer

<400> 428
gatggggaga gcgtatttac c                                            21


<210> 429
<211> 28
<212> DNA
<213> Artificial Sequence

<220>
<223> Description of Artificial Sequence:
      oligonucleotide primer

<400> 429
tcaaagatct attcctacat gagcccga                                     28


<210> 430
<211> 20
<212> DNA
<213> Artificial Sequence

<220>
<223> Description of Artificial Sequence:
      oligonucleotide primer

<400> 430
aaacgcattc cagagcattt                                              20


<210> 431
<211> 22
<212> DNA
<213> Artificial Sequence

<220>
<223> Description of Artificial Sequence:
      oligonucleotide primer

<400> 431
accagcctgt caactactcc tt                                                22

<210> 432
<211> 26
<212> DNA
<213> Artificial Sequence

<220>
<223> Description of Artificial Sequence:
      oligonucleotide primer

<400> 432
ccactccacc tacttggtga accagg                                            26

<210> 433
<211> 22
<212> DNA
<213> Artificial Sequence

<220>
<223> Description of Artificial Sequence:
      oligonucleotide primer

<400> 433
ggaaattgac actctggtca aa                                                22

<210> 434
<211> 21
<212> DNA
<213> Artificial Sequence

<220>
<223> Description of Artificial Sequence:
      oligonucleotide primer

<400> 434
catcgagacc cagagtaaag c                                                 21

<210> 435
<211> 26
<212> DNA
<213> Artificial Sequence

<220>
<223> Description of Artificial Sequence:
      oligonucleotide primer

<400> 435
catgacaatg ctcaccattg aacagt                                            26

<210> 436
<211> 22
<212> DNA
<213> Artificial Sequence

<220>
<223> Description of Artificial Sequence:
      oligonucleotide primer

<400> 436
tttcattttc tgaatggcaa ac                                                22

<210> 437
<211> 20
<212> DNA
<213> Artificial Sequence

<220>
<223> Description of Artificial Sequence:
      oligonucleotide primer

<400> 437
tctcaggaag tggtgaagga                                                    20


<210> 438
<211> 26
<212> DNA
<213> Artificial Sequence

<220>
<223> Description of Artificial Sequence:
      oligonucleotide primer

<400> 438
caattctcca aggatacatt ggaaaa                                             26


<210> 439
<211> 19
<212> DNA
<213> Artificial Sequence

<220>
<223> Description of Artificial Sequence:
      oligonucleotide primer

<400> 439
atacaaaccc tcggaacga                                                     19


<210> 440
<211> 22
<212> DNA
<213> Artificial Sequence

<220>
<223> Description of Artificial Sequence:
      oligonucleotide primer

<400> 440
ttctgtgaaa ccaatgagaa ca                                                 22


<210> 441
<211> 26
<212> DNA
<213> Artificial Sequence

<220>
<223> Description of Artificial Sequence:
      oligonucleotide primer

<400> 441
cacaacgtac cagaatttct gggaca                                             26


<210> 442
<211> 22
<212> DNA
<213> Artificial Sequence

<220>
<223> Description of Artificial Sequence:
      oligonucleotide primer

<400> 442
ccctctaacc actgctttag ct                                          22


<210> 443
<211> 21
<212> DNA
<213> Artificial Sequence

<220>
<223> Description of Artificial Sequence:
      oligonucleotide primer

<400> 443
gatgaagctg gaaaccatca t                                           21


<210> 444
<211> 26
<212> DNA
<213> Artificial Sequence

<220>
<223> Description of Artificial Sequence:
      oligonucleotide primer

<400> 444
tcacaaggac agaaaaccaa acactg                                      26


<210> 445
<211> 22
<212> DNA
<213> Artificial Sequence

<220>
<223> Description of Artificial Sequence:
      oligonucleotide primer

<400> 445
cccacctatg agtgagaaca tg                                          22


<210> 446
<211> 22
<212> DNA
<213> Artificial Sequence

<220>
<223> Description of Artificial Sequence:
      oligonucleotide primer

<400> 446
tcacaaggac agaaaaccaa ac                                          22


<210> 447
<211> 26
<212> DNA
<213> Artificial Sequence

<220>
<223> Description of Artificial Sequence:
      oligonucleotide primer

<400> 447
ccacatgttc tcactcatag gtggga                                          26

<210> 448
<211> 22
<212> DNA
<213> Artificial Sequence

<220>
<223> Description of Artificial Sequence:
      oligonucleotide primer

<400> 448
gtgtccatgt gttctcgttg tt                                              22

<210> 449
<211> 22
<212> DNA
<213> Artificial Sequence

<220>
<223> Description of Artificial Sequence:
      oligonucleotide primer

<400> 449
gccatcccat tactgggtat at                                              22

<210> 450
<211> 26
<212> DNA
<213> Artificial Sequence

<220>
<223> Description of Artificial Sequence:
      oligonucleotide primer

<400> 450
tcatgctgct ataaagacac atgcag                                          26

<210> 451
<211> 22
<212> DNA
<213> Artificial Sequence

<220>
<223> Description of Artificial Sequence:
      oligonucleotide primer

<400> 451
tagtgccgca ataaacatac gt                                              22

<210> 452
<211> 19
<212> DNA
<213> Artificial Sequence

<220>
<223> Description of Artificial Sequence:
      oligonucleotide primer

<400> 452
cctcgtctcg agacaagga                                                  19

<210> 453
<211> 24
<212> DNA
<213> Artificial Sequence

<220>
<223> Description of Artificial Sequence:
      oligonucleotide primer

<400> 453
accatcctcg acacactccg ggag                                              24


<210> 454
<211> 20
<212> DNA
<213> Artificial Sequence

<220>
<223> Description of Artificial Sequence:
      oligonucleotide primer

<400> 454
cggtccttag tgggtttgac                                                   20


<210> 455
<211> 20
<212> DNA
<213> Artificial Sequence

<220>
<223> Description of Artificial Sequence:
      oligonucleotide primer

<400> 455
ccggtacacc aatgatctgt                                                   20


<210> 456
<211> 23
<212> DNA
<213> Artificial Sequence

<220>
<223> Description of Artificial Sequence:
      oligonucleotide primer

<400> 456
cgaacatgct tctgctgcgt cct                                               23


<210> 457
<211> 19
<212> DNA
<213> Artificial Sequence

<220>
<223> Description of Artificial Sequence:
      oligonucleotide primer

<400> 457
tgacgacttt ccacaccaa                                                    19


<210> 458
<211> 22
<212> DNA

<213> Artificial Sequence

<220>
<223> Description of Artificial Sequence:
      oligonucleotide primer

<400> 458
ttgccatcta ttccgagtac tg                                                22


<210> 459
<211> 24
<212> DNA
<213> Artificial Sequence

<220>
<223> Description of Artificial Sequence:
      oligonucleotide primer

<400> 459
ccaacctcat gaagcagggc aagt                                              24


<210> 460
<211> 22
<212> DNA
<213> Artificial Sequence

<220>
<223> Description of Artificial Sequence:
      oligonucleotide primer

<400> 460
caggcttcaa agaaatgtct gt                                                22


<210> 461
<211> 21
<212> DNA
<213> Artificial Sequence

<220>
<223> Description of Artificial Sequence:
      oligonucleotide primer

<400> 461
gtgcaagagg acaaggagat g                                                 21


<210> 462
<211> 26
<212> DNA
<213> Artificial Sequence

<220>
<223> Description of Artificial Sequence:
      oligonucleotide primer

<400> 462
ttcagaaaac cagaagaaac ttgcca                                            26


<210> 463
<211> 21
<212> DNA
<213> Artificial Sequence

<220>
<223> Description of Artificial Sequence:

oligonucleotide primer

<400> 463
cctgcctttt gagcatttaa c                                                21


<210> 464
<211> 22
<212> DNA
<213> Artificial Sequence

<220>
<223> Description of Artificial Sequence:
      oligonucleotide primer

<400> 464
ggaatacctg gtcaggaaga ag                                               22


<210> 465
<211> 26
<212> DNA
<213> Artificial Sequence

<220>
<223> Description of Artificial Sequence:
      oligonucleotide primer

<400> 465
cacatctata tccccaagaa cggtca                                           26


<210> 466
<211> 22
<212> DNA
<213> Artificial Sequence

<220>
<223> Description of Artificial Sequence:
      oligonucleotide primer

<400> 466
ttggcattga tacagctgaa gt                                               22


<210> 467
<211> 22
<212> DNA
<213> Artificial Sequence

<220>
<223> Description of Artificial Sequence:
      oligonucleotide primer

<400> 467
tgtctcaagg ctttgagttc tt                                               22


<210> 468
<211> 23
<212> DNA
<213> Artificial Sequence

<220>
<223> Description of Artificial Sequence:
      oligonucleotide primer

<400> 468
ctgcagccag tctctgtcca aaa                                              23

<210> 469
<211> 22
<212> DNA
<213> Artificial Sequence

<220>
<223> Description of Artificial Sequence:
      oligonucleotide primer

<400> 469
tccccactcc ttcatcataa at                                          22


<210> 470
<211> 21
<212> DNA
<213> Artificial Sequence

<220>
<223> Description of Artificial Sequence:
      oligonucleotide primer

<400> 470
gacgttcaac aatgacatgc t                                           21


<210> 471
<211> 25
<212> DNA
<213> Artificial Sequence

<220>
<223> Description of Artificial Sequence:
      oligonucleotide primer

<400> 471
cttcatcagc agcagctgca ttgct                                       25


<210> 472
<211> 22
<212> DNA
<213> Artificial Sequence

<220>
<223> Description of Artificial Sequence:
      oligonucleotide primer

<400> 472
agcaggaggt gaggatgtag ac                                          22


<210> 473
<211> 22
<212> DNA
<213> Artificial Sequence

<220>
<223> Description of Artificial Sequence:
      oligonucleotide primer

<400> 473
ccctcagagg actggtttac tt                                          22


<210> 474
<211> 23

<212> DNA
<213> Artificial Sequence

<220>
<223> Description of Artificial Sequence:
      oligonucleotide primer

<400> 474
cacctctgcc tgccacacct cag                                              23


<210> 475
<211> 22
<212> DNA
<213> Artificial Sequence

<220>
<223> Description of Artificial Sequence:
      oligonucleotide primer

<400> 475
ctacatgcag tggagcaagt ct                                               22


<210> 476
<211> 21
<212> DNA
<213> Artificial Sequence

<220>
<223> Description of Artificial Sequence:
      oligonucleotide primer

<400> 476
gaactgttcc ttgtggtttc c                                                21


<210> 477
<211> 24
<212> DNA
<213> Artificial Sequence

<220>
<223> Description of Artificial Sequence:
      oligonucleotide primer

<400> 477
accacaggca tcagcagtcc catt                                             24


<210> 478
<211> 21
<212> DNA
<213> Artificial Sequence

<220>
<223> Description of Artificial Sequence:
      oligonucleotide primer

<400> 478
acactttgcc tcaggtgact t                                                21


<210> 479
<211> 22
<212> DNA
<213> Artificial Sequence

<220>

<223> Description of Artificial Sequence:
      oligonucleotide primer

<400> 479
gtcaggagaa ctgttccttg tg                                              22


<210> 480
<211> 23
<212> DNA
<213> Artificial Sequence

<220>
<223> Description of Artificial Sequence:
      oligonucleotide primer

<400> 480
accacaggca tcagcagtcc cat                                             23


<210> 481
<211> 20
<212> DNA
<213> Artificial Sequence

<220>
<223> Description of Artificial Sequence:
      oligonucleotide primer

<400> 481
ctttgcctca ggtgacttga                                                 20


<210> 482
<211> 21
<212> DNA
<213> Artificial Sequence

<220>
<223> Description of Artificial Sequence:
      oligonucleotide primer

<400> 482
ttggagcctt tgctcttatg t                                               21


<210> 483
<211> 26
<212> DNA
<213> Artificial Sequence

<220>
<223> Description of Artificial Sequence:
      oligonucleotide primer

<400> 483
aggggagacc atccccaagt tacaaa                                          26


<210> 484
<211> 21
<212> DNA
<213> Artificial Sequence

<220>
<223> Description of Artificial Sequence:
      oligonucleotide primer

<400> 484

aagatgccca gaaaagctgt a                                              21

<210> 485
<211> 22
<212> DNA
<213> Artificial Sequence

<220>
<223> Description of Artificial Sequence:
      oligonucleotide primer

<400> 485
ccatcttctg tgggacagtt ac                                             22

<210> 486
<211> 28
<212> DNA
<213> Artificial Sequence

<220>
<223> Description of Artificial Sequence:
      oligonucleotide primer

<400> 486
catgtatata cagccaggaa acagtcca                                       28

<210> 487
<211> 22
<212> DNA
<213> Artificial Sequence

<220>
<223> Description of Artificial Sequence:
      oligonucleotide primer

<400> 487
agaagtttgc cctcattctg at                                             22

<210> 488
<211> 22
<212> DNA
<213> Artificial Sequence

<220>
<223> Description of Artificial Sequence:
      oligonucleotide primer

<400> 488
gagttgcatt gtgttgttgt tg                                             22

<210> 489
<211> 27
<212> DNA
<213> Artificial Sequence

<220>
<223> Description of Artificial Sequence:
      oligonucleotide primer

<400> 489
tcattttcct tgcatcactt cttctca                                        27

<210> 490

<211> 22
<212> DNA
<213> Artificial Sequence

<220>
<223> Description of Artificial Sequence:
      oligonucleotide primer

<400> 490
caatgaagcc atatgacaca ag                                                22

<210> 491
<211> 22
<212> DNA
<213> Artificial Sequence

<220>
<223> Description of Artificial Sequence:
      oligonucleotide primer

<400> 491
tttgtggctg ttttgatatc ct                                                22

<210> 492
<211> 26
<212> DNA
<213> Artificial Sequence

<220>
<223> Description of Artificial Sequence:
      oligonucleotide primer

<400> 492
tctccttacc cttgtgggaa atacag                                            26

<210> 493
<211> 22
<212> DNA
<213> Artificial Sequence

<220>
<223> Description of Artificial Sequence:
      oligonucleotide primer

<400> 493
gggtctacag agcagatcag aa                                                22

<210> 494
<211> 22
<212> DNA
<213> Artificial Sequence

<220>
<223> Description of Artificial Sequence:
      oligonucleotide primer

<400> 494
tggccatgta tttggtgact at                                                22

<210> 495
<211> 26
<212> DNA
<213> Artificial Sequence

<220>
<223> Description of Artificial Sequence:
      oligonucleotide primer

<400> 495
aggaaacacc ctcattcttc ttctga                                          26


<210> 496
<211> 22
<212> DNA
<213> Artificial Sequence

<220>
<223> Description of Artificial Sequence:
      oligonucleotide primer

<400> 496
tatgaagcct gttgtccagt ct                                              22


<210> 497
<211> 22
<212> DNA
<213> Artificial Sequence

<220>
<223> Description of Artificial Sequence:
      oligonucleotide primer

<400> 497
gaggtgcttt caacaaagga at                                              22


<210> 498
<211> 28
<212> DNA
<213> Artificial Sequence

<220>
<223> Description of Artificial Sequence:
      oligonucleotide primer

<400> 498
tcggttactc ccttactgaa tcccttca                                        28


<210> 499
<211> 22
<212> DNA
<213> Artificial Sequence

<220>
<223> Description of Artificial Sequence:
      oligonucleotide primer

<400> 499
gcttgtttca cttgctgatt tc                                              22


<210> 500
<211> 22
<212> DNA
<213> Artificial Sequence

<220>
<223> Description of Artificial Sequence:
      oligonucleotide primer

<400> 500
gaggtgcttt caacaaagga at                                                22

<210> 501
<211> 28
<212> DNA
<213> Artificial Sequence

<220>
<223> Description of Artificial Sequence:
      oligonucleotide primer

<400> 501
tcggttactc ccttactgaa tcccttca                                          28

<210> 502
<211> 22
<212> DNA
<213> Artificial Sequence

<220>
<223> Description of Artificial Sequence:
      oligonucleotide primer

<400> 502
gcttgtttca cttgctgatt tc                                                22

<210> 503
<211> 22
<212> DNA
<213> Artificial Sequence

<220>
<223> Description of Artificial Sequence:
      oligonucleotide primer

<400> 503
tacactgttg tcacgccaat aa                                                22

<210> 504
<211> 26
<212> DNA
<213> Artificial Sequence

<220>
<223> Description of Artificial Sequence:
      oligonucleotide primer

<400> 504
ccctttgatt tactgcctga ggaaca                                            26

<210> 505
<211> 22
<212> DNA
<213> Artificial Sequence

<220>
<223> Description of Artificial Sequence:
      oligonucleotide primer

<400> 505
ttttcaaggc gtccttaaat tc                                                22

<210> 506
<211> 22
<212> DNA
<213> Artificial Sequence

<220>
<223> Description of Artificial Sequence:
      oligonucleotide primer

<400> 506
attgatttgg tttggctact tg                                                22


<210> 507
<211> 30
<212> DNA
<213> Artificial Sequence

<220>
<223> Description of Artificial Sequence:
      oligonucleotide primer

<400> 507
aactctacat ttaatccaat ggtttatgca                                        30


<210> 508
<211> 22
<212> DNA
<213> Artificial Sequence

<220>
<223> Description of Artificial Sequence:
      oligonucleotide primer

<400> 508
accaaacagc atcatcttca gt                                                22


<210> 509
<211> 22
<212> DNA
<213> Artificial Sequence

<220>
<223> Description of Artificial Sequence:
      oligonucleotide primer

<400> 509
actcttcaca gttgctgtga tg                                                22


<210> 510
<211> 26
<212> DNA
<213> Artificial Sequence

<220>
<223> Description of Artificial Sequence:
      oligonucleotide primer

<400> 510
cacttgtgct tcatctgcat cgacag                                            26


<210> 511
<211> 22
<212> DNA
<213> Artificial Sequence

<220>
<223> Description of Artificial Sequence:
      oligonucleotide primer

<400> 511
aggggatcag taaccacaat gt                                                22


<210> 512
<211> 22
<212> DNA
<213> Artificial Sequence

<220>
<223> Description of Artificial Sequence:
      oligonucleotide primer

<400> 512
catctccatc gacaggtaca tt                                                22


<210> 513
<211> 27
<212> DNA
<213> Artificial Sequence

<220>
<223> Description of Artificial Sequence:
      oligonucleotide primer

<400> 513
ccctggtcta tcctaccaag ttcaccg                                           27


<210> 514
<211> 22
<212> DNA
<213> Artificial Sequence

<220>
<223> Description of Artificial Sequence:
      oligonucleotide primer

<400> 514
gatgcaaatt cctgacacag at                                                22


<210> 515
<211> 22
<212> DNA
<213> Artificial Sequence

<220>
<223> Description of Artificial Sequence:
      oligonucleotide primer

<400> 515
ttacctcctg accataatgg aa                                                22


<210> 516
<211> 26
<212> DNA
<213> Artificial Sequence

<220>
<223> Description of Artificial Sequence:
      oligonucleotide primer

<400> 516
aacctgatgc tgctgctcat gatcag 26

<210> 517
<211> 22
<212> DNA
<213> Artificial Sequence

<220>
<223> Description of Artificial Sequence:
      oligonucleotide primer

<400> 517
tacatgggct tatggagaca ag 22

<210> 518
<211> 22
<212> DNA
<213> Artificial Sequence

<220>
<223> Description of Artificial Sequence:
      oligonucleotide primer

<400> 518
ttacctcctg accataatgg aa 22

<210> 519
<211> 26
<212> DNA
<213> Artificial Sequence

<220>
<223> Description of Artificial Sequence:
      oligonucleotide primer

<400> 519
aacctgatgc tgctgctcat gatcag 26

<210> 520
<211> 22
<212> DNA
<213> Artificial Sequence

<220>
<223> Description of Artificial Sequence:
      oligonucleotide primer

<400> 520
tacatgggct tatggagaca ag 22

<210> 521
<211> 22
<212> DNA
<213> Artificial Sequence

<220>
<223> Description of Artificial Sequence:
      oligonucleotide primer

<400> 521
taccgatcat agcacatcat ca 22

<210> 522
<211> 27
<212> DNA
<213> Artificial Sequence

<220>
<223> Description of Artificial Sequence:
      oligonucleotide primer

<400> 522
tcagacactc tgtaatagca aacgcca                                          27


<210> 523
<211> 22
<212> DNA
<213> Artificial Sequence

<220>
<223> Description of Artificial Sequence:
      oligonucleotide primer

<400> 523
tgctccttgc atacttcaga ct                                               22


<210> 524
<211> 22
<212> DNA
<213> Artificial Sequence

<220>
<223> Description of Artificial Sequence:
      oligonucleotide primer

<400> 524
ctttccctgt tttacagcct tt                                               22


<210> 525
<211> 26
<212> DNA
<213> Artificial Sequence

<220>
<223> Description of Artificial Sequence:
      oligonucleotide primer

<400> 525
cccctcatct acagccttaa tgcaga                                           26


<210> 526
<211> 22
<212> DNA
<213> Artificial Sequence

<220>
<223> Description of Artificial Sequence:
      oligonucleotide primer

<400> 526
   ctctctttag agcccctttc ac                                                22


<210> 527
<211> 21
<212> DNA

<213> Artificial Sequence

<220>
<223> Description of Artificial Sequence:
      oligonucleotide primer

<400> 527
ctctttgtcc tggtggagaa c                                                21


<210> 528
<211> 26
<212> DNA
<213> Artificial Sequence

<220>
<223> Description of Artificial Sequence:
      oligonucleotide primer

<400> 528
acctccctcc acaggcccat gtacta                                           26


<210> 529
<211> 22
<212> DNA
<213> Artificial Sequence

<220>
<223> Description of Artificial Sequence:
      oligonucleotide primer

<400> 529
gaaagacatg gagctcagaa ag                                               22


<210> 530
<211> 22
<212> DNA
<213> Artificial Sequence

<220>
<223> Description of Artificial Sequence:
      oligonucleotide primer

<400> 530
gaacatcatc tcctaccctg aa                                               22


<210> 531
<211> 28
<212> DNA
<213> Artificial Sequence

<220>
<223> Description of Artificial Sequence:
      oligonucleotide primer

<400> 531
tgcatgactc agctctactt cttcctcg                                         28


<210> 532
<211> 22
<212> DNA
<213> Artificial Sequence

<220>
<223> Description of Artificial Sequence:

oligonucleotide primer

<400> 532
atgtgacact ctgcaatagc aa                                                22

<210> 533
<211> 22
<212> DNA
<213> Artificial Sequence

<220>
<223> Description of Artificial Sequence:
      oligonucleotide primer

<400> 533
ggggaaagta tcctctgtgt tt                                                22

<210> 534
<211> 26
<212> DNA
<213> Artificial Sequence

<220>
<223> Description of Artificial Sequence:
      oligonucleotide primer

<400> 534
ttattgtgcc catgttgaac cctctg                                            26

<210> 535
<211> 22
<212> DNA
<213> Artificial Sequence

<220>
<223> Description of Artificial Sequence:
      oligonucleotide primer

<400> 535
cagggaaaca tggacatctt ta                                                22

<210> 536
<211> 21
<212> DNA
<213> Artificial Sequence

<220>
<223> Description of Artificial Sequence:
      oligonucleotide primer

<400> 536
tgctattgcc caggctgtac t                                                 21

<210> 537
<211> 27
<212> DNA
<213> Artificial Sequence

<220>
<223> Description of Artificial Sequence:
      oligonucleotide primer

<400> 537
aggatgcagt caaccactgg gcttcag                                           27

<210> 538
<211> 25
<212> DNA
<213> Artificial Sequence

<220>
<223> Description of Artificial Sequence:
      oligonucleotide primer

<400> 538
tgagctccac atgttccaaa tactt                                          25


<210> 539
<211> 22
<212> DNA
<213> Artificial Sequence

<220>
<223> Description of Artificial Sequence:
      oligonucleotide primer

<400> 539
gtccttgaag gatccaaaac tc                                             22


<210> 540
<211> 26
<212> DNA
<213> Artificial Sequence

<220>
<223> Description of Artificial Sequence:
      oligonucleotide primer

<400> 540
tttctttctt tccaaccttt ccttgg                                         26


<210> 541
<211> 22
<212> DNA
<213> Artificial Sequence

<220>
<223> Description of Artificial Sequence:
      oligonucleotide primer

<400> 541
ctgctggtca aacagaggtc ta                                             22


<210> 542
<211> 22
<212> DNA
<213> Artificial Sequence

<220>
<223> Description of Artificial Sequence:
      oligonucleotide primer

<400> 542
gggcaaattt ctcactcttt tc                                             22


<210> 543
<211> 27

<212> DNA
<213> Artificial Sequence

<220>
<223> Description of Artificial Sequence:
      oligonucleotide primer

<400> 543
ccccaactct taatcccctc atctaca                                          27


<210> 544
<211> 22
<212> DNA
<213> Artificial Sequence

<220>
<223> Description of Artificial Sequence:
      oligonucleotide primer

<400> 544
ccctttacct ccttgttcct ta                                               22


<210> 545
<211> 25
<212> DNA
<213> Artificial Sequence

<220>
<223> Description of Artificial Sequence:
      oligonucleotide primer

<400> 545
aattcagatg tctatcgcca gtgtt                                            25


<210> 546
<211> 29
<212> DNA
<213> Artificial Sequence

<220>
<223> Description of Artificial Sequence:
      oligonucleotide primer

<400> 546
tcctcctggt gatgcccttg atcattatc                                        29


<210> 547
<211> 25
<212> DNA
<213> Artificial Sequence

<220>
<223> Description of Artificial Sequence:
      oligonucleotide primer

<400> 547
ttagcaatag caccagaaga ggaaa                                            25


<210> 548
<211> 28
<212> DNA
<213> Artificial Sequence

<220>

<223> Description of Artificial Sequence:
      oligonucleotide primer

<400> 548
ccccatgccc cgggcagtgt ccgggtcc                                          28


<210> 549
<211> 30
<212> DNA
<213> Artificial Sequence

<220>
<223> Description of Artificial Sequence:
      oligonucleotide primer

<400> 549
gctgggctcg ccgatcagat cctgcatgac                                        30


<210> 550
<211> 19
<212> DNA
<213> Artificial Sequence

<220>
<223> Description of Artificial Sequence:
      oligonucleotide primer

<400> 550
cctggagcct gagacagcg                                                    19


<210> 551
<211> 25
<212> DNA
<213> Artificial Sequence

<220>
<223> Description of Artificial Sequence:
      oligonucleotide primer

<400> 551
agcattccct attacaactc cagaa                                             25


<210> 552
<211> 19
<212> DNA
<213> Artificial Sequence

<220>
<223> Description of Artificial Sequence:
      oligonucleotide primer

<400> 552
ggtgtccacc agcagccag                                                    19


<210> 553
<211> 23
<212> DNA
<213> Artificial Sequence

<220>
<223> Description of Artificial Sequence:
      oligonucleotide primer

<400> 553

atcacagtga ccacctcact gaa 23

<210> 554
<211> 34
<212> DNA
<213> Artificial Sequence

<220>
<223> Description of Artificial Sequence:
      oligonucleotide primer

<400> 554
gaattctgtg caaacgggca ccacgactgc aacg 34

<210> 555
<211> 36
<212> DNA
<213> Artificial Sequence

<220>
<223> Description of Artificial Sequence:
      oligonucleotide primer

<400> 555
ctcgaggcac tgctggccgc ccagcggact cccgtg 36

<210> 556
<211> 18
<212> DNA
<213> Artificial Sequence

<220>
<223> Description of Artificial Sequence:
      oligonucleotide primer

<400> 556
ttgctctgcc gcaaccac 18

<210> 557
<211> 21
<212> DNA
<213> Artificial Sequence

<220>
<223> Description of Artificial Sequence:
      oligonucleotide primer

<400> 557
agctatgggg agaaatgcga g 21

<210> 558
<211> 20
<212> DNA
<213> Artificial Sequence

<220>
<223> Description of Artificial Sequence:
      oligonucleotide primer

<400> 558
cagagcaagc agtggtcacg 20

<210> 559

<211> 21
<212> DNA
<213> Artificial Sequence

<220>
<223> Description of Artificial Sequence:
      oligonucleotide primer

<400> 559
tgttattctg gcagttcacg c                                                21


<210> 560
<211> 29
<212> DNA
<213> Artificial Sequence

<220>
<223> Description of Artificial Sequence:
      oligonucleotide primer

<400> 560
gaattcccct gccgcctgct gtcctcacc                                        29


<210> 561
<211> 36
<212> DNA
<213> Artificial Sequence

<220>
<223> Description of Artificial Sequence:
      oligonucleotide primer

<400> 561
ctcgaggcac tgctggccgc ccagcggact cccgtg                                36


<210> 562
<211> 23
<212> DNA
<213> Artificial Sequence

<220>
<223> Description of Artificial Sequence:
      oligonucleotide primer

<400> 562
ctctgagaat gactgtcgga tca                                              23


<210> 563
<211> 23
<212> DNA
<213> Artificial Sequence

<220>
<223> Description of Artificial Sequence:
      oligonucleotide primer

<400> 563
tacctcctgc ctggactcta agg                                              23


<210> 564
<211> 20
<212> DNA
<213> Artificial Sequence

<220>
<223> Description of Artificial Sequence:
      oligonucleotide primer

<400> 564
ctcctgggcc ttcctgtcct                                                   20


<210> 565
<211> 20
<212> DNA
<213> Artificial Sequence

<220>
<223> Description of Artificial Sequence:
      oligonucleotide primer

<400> 565
ctgcaaccgc cacagtgaat                                                   20


<210> 566
<211> 23
<212> DNA
<213> Artificial Sequence

<220>
<223> Description of Artificial Sequence:
      oligonucleotide primer

<400> 566
cgtgtgcgtg aactgccaga ata                                               23


<210> 567
<211> 20
<212> DNA
<213> Artificial Sequence

<220>
<223> Description of Artificial Sequence:
      oligonucleotide primer

<400> 567
agaggtgcag gacccattgc                                                   20


<210> 568
<211> 23
<212> DNA
<213> Artificial Sequence

<220>
<223> Description of Artificial Sequence:
      oligonucleotide primer

<400> 568
acagttgggt aggaggtgac aag                                               23


<210> 569
<211> 20
<212> DNA
<213> Artificial Sequence

<220>
<223> Description of Artificial Sequence:
      oligonucleotide primer

<400> 569
cccacatgtc agcccatcac                                                    20

<210> 570
<211> 20
<212> DNA
<213> Artificial Sequence

<220>
<223> Description of Artificial Sequence:
      oligonucleotide primer

<400> 570
attcactgtg gcggttgcag                                                    20

<210> 571
<211> 22
<212> DNA
<213> Artificial Sequence

<220>
<223> Description of Artificial Sequence:
      oligonucleotide primer

<400> 571
ggcctcgtct tcactaggac cc                                                 22

<210> 572
<211> 29
<212> DNA
<213> Artificial Sequence

<220>
<223> Description of Artificial Sequence:
      oligonucleotide primer

<400> 572
tactatgcca cagtgaatga gctcactcc                                          29

<210> 573
<211> 33
<212> DNA
<213> Artificial Sequence

<220>
<223> Description of Artificial Sequence:
      oligonucleotide primer

<400> 573
caaaaccttg atggccaagg ttagagttga atg                                     33

<210> 574
<211> 24
<212> DNA
<213> Artificial Sequence

<220>
<223> Description of Artificial Sequence:
      oligonucleotide primer

<400> 574
gtttcttcct tgtgtccttg tgcc                                               24

<210> 575
<211> 28
<212> DNA
<213> Artificial Sequence

<220>
<223> Description of Artificial Sequence:
      oligonucleotide primer

<400> 575
cctttgccgg tctacacatt gaaatact                                    28


<210> 576
<211> 27
<212> DNA
<213> Artificial Sequence

<220>
<223> Description of Artificial Sequence:
      oligonucleotide primer

<400> 576
ttgatggtgt acaagaaagt gagccag                                     27


<210> 577
<211> 27
<212> DNA
<213> Artificial Sequence

<220>
<223> Description of Artificial Sequence:
      oligonucleotide primer

<400> 577
cgaaggaact ccatctgcac tgtgtat                                     27


<210> 578
<211> 30
<212> DNA
<213> Artificial Sequence

<220>
<223> Description of Artificial Sequence:
      oligonucleotide primer

<400> 578
cagtaaacat aatggtgaca gatgtcaatg                                  30


<210> 579
<211> 24
<212> DNA
<213> Artificial Sequence

<220>
<223> Description of Artificial Sequence:
      oligonucleotide primer

<400> 579
aaacattgga cccaagtcat ctcc                                        24


<210> 580
<211> 25
<212> DNA
<213> Artificial Sequence

<220>
<223> Description of Artificial Sequence:
      oligonucleotide primer

<400> 580
gatgaccatt gtcttgttca gcctt                                          25


<210> 581
<211> 27
<212> DNA
<213> Artificial Sequence

<220>
<223> Description of Artificial Sequence:
      oligonucleotide primer

<400> 581
aatgctgtta tcgaaaaggt gtaagtt                                        27


<210> 582
<211> 27
<212> DNA
<213> Artificial Sequence

<220>
<223> Description of Artificial Sequence:
      oligonucleotide primer

<400> 582
aatatacaca gtgcagatgg agttcct                                        27


<210> 583
<211> 26
<212> DNA
<213> Artificial Sequence

<220>
<223> Description of Artificial Sequence:
      oligonucleotide primer

<400> 583
gtttactgtg gcagttgagg tcccat                                         26


<210> 584
<211> 35
<212> DNA
<213> Artificial Sequence

<220>
<223> Description of Artificial Sequence:
      oligonucleotide primer

<400> 584
ggatccagaa atttatctgt ggtggaagaa gaagc                               35


<210> 585
<211> 35
<212> DNA
<213> Artificial Sequence

<220>
<223> Description of Artificial Sequence:
      oligonucleotide primer

<400> 585
ctcgagccct tctgtgtatc ctagactttc tcctc                                35

<210> 586
<211> 25
<212> DNA
<213> Artificial Sequence

<220>
<223> Description of Artificial Sequence:
      oligonucleotide primer

<400> 586
actaccatcc ttcaaataga ggcca                                           25

<210> 587
<211> 28
<212> DNA
<213> Artificial Sequence

<220>
<223> Description of Artificial Sequence:
      oligonucleotide primer

<400> 587
cacaacagtt tatgctgaag atgcagac                                        28

<210> 588
<211> 25
<212> DNA
<213> Artificial Sequence

<220>
<223> Description of Artificial Sequence:
      oligonucleotide primer

<400> 588
atggttggtg taatttctgc tgctg                                           25

<210> 589
<211> 30
<212> DNA
<213> Artificial Sequence

<220>
<223> Description of Artificial Sequence:
      oligonucleotide primer

<400> 589
agagtgaagg ctactgataa agatactggc                                      30

<210> 590
<211> 25
<212> DNA
<213> Artificial Sequence

<220>
<223> Description of Artificial Sequence:
      oligonucleotide primer

<400> 590
aaatcttgga tcgctatgtt cagga                                           25

<210> 591
<211> 27
<212> DNA
<213> Artificial Sequence

<220>
<223> Description of Artificial Sequence:
      oligonucleotide primer

<400> 591
tagtcccagc tggcaaattc tcttcaa                                          27


<210> 592
<211> 25
<212> DNA
<213> Artificial Sequence

<220>
<223> Description of Artificial Sequence:
      oligonucleotide primer

<400> 592
ataggtgtac ccttgactgc atccg                                            25


<210> 593
<211> 25
<212> DNA
<213> Artificial Sequence

<220>
<223> Description of Artificial Sequence:
      oligonucleotide primer

<400> 593
cctttggtgg caagttcact tactg                                            25


<210> 594
<211> 27
<212> DNA
<213> Artificial Sequence

<220>
<223> Description of Artificial Sequence:
      oligonucleotide primer

<400> 594
cagaagtaaa cattcttgca tcttcag                                          27


<210> 595
<211> 27
<212> DNA
<213> Artificial Sequence

<220>
<223> Description of Artificial Sequence:
      oligonucleotide primer

<400> 595
catagcgatc caagatttct gtaagat                                          27


<210> 596
<211> 38
<212> DNA

<213> Artificial Sequence

<220>
<223> Description of Artificial Sequence:
      oligonucleotide primer

<400> 596
agatctcagc ctcaggcccc gggttacttg attgcagc                                38


<210> 597
<211> 34
<212> DNA
<213> Artificial Sequence

<220>
<223> Description of Artificial Sequence:
      oligonucleotide primer

<400> 597
aagcttagaa acatcataat cttccagttc ctgg                                    34


<210> 598
<211> 23
<212> DNA
<213> Artificial Sequence

<220>
<223> Description of Artificial Sequence:
      oligonucleotide primer

<400> 598
ggacaagcct gtgtacagac ccc                                                23


<210> 599
<211> 23
<212> DNA
<213> Artificial Sequence

<220>
<223> Description of Artificial Sequence:
      oligonucleotide primer

<400> 599
ggtatatcca agacctggac gcc                                                23


<210> 600
<211> 22
<212> DNA
<213> Artificial Sequence

<220>
<223> Description of Artificial Sequence:
      oligonucleotide primer

<400> 600
cagctggtgg acatccggta ct                                                 22


<210> 601
<211> 24
<212> DNA
<213> Artificial Sequence

<220>
<223> Description of Artificial Sequence:

oligonucleotide primer

<400> 601
gtactccccc agccagtgct acct                                              24


<210> 602
<211> 22
<212> DNA
<213> Artificial Sequence

<220>
<223> Description of Artificial Sequence:
      oligonucleotide primer

<400> 602
ctctcttcat ctggccgtga cc                                                22


<210> 603
<211> 23
<212> DNA
<213> Artificial Sequence

<220>
<223> Description of Artificial Sequence:
      oligonucleotide primer

<400> 603
tctggatgaa tacagaagcg ccc                                               23


<210> 604
<211> 23
<212> DNA
<213> Artificial Sequence

<220>
<223> Description of Artificial Sequence:
      oligonucleotide primer

<400> 604
gcgggtcaat cagaagctca aac                                               23


<210> 605
<211> 24
<212> DNA
<213> Artificial Sequence

<220>
<223> Description of Artificial Sequence:
      oligonucleotide primer

<400> 605
cttggagtac cggatgtcca ccag                                              24


<210> 606
<211> 27
<212> DNA
<213> Artificial Sequence

<220>
<223> Description of Artificial Sequence:
      oligonucleotide primer

<400> 606
agggagaggt agctgggcag gtactga                                           27

<210> 607
<211> 25
<212> DNA
<213> Artificial Sequence

<220>
<223> Description of Artificial Sequence:
      oligonucleotide primer

<400> 607
cagatgaaga gaggtcacac acacg                                          25


<210> 608
<211> 38
<212> DNA
<213> Artificial Sequence

<220>
<223> Description of Artificial Sequence:
      oligonucleotide primer

<400> 608
agatctcagc ctcaggcccc gggttacttg attgcagc                            38


<210> 609
<211> 34
<212> DNA
<213> Artificial Sequence

<220>
<223> Description of Artificial Sequence:
      oligonucleotide primer

<400> 609
aagcttagaa acatcataat cttccagttc ctgg                                34


<210> 610
<211> 23
<212> DNA
<213> Artificial Sequence

<220>
<223> Description of Artificial Sequence:
      oligonucleotide primer

<400> 610
ggacaagcct gtgtacagac ccc                                            23


<210> 611
<211> 23
<212> DNA
<213> Artificial Sequence

<220>
<223> Description of Artificial Sequence:
      oligonucleotide primer

<400> 611
ggtatatcca agacctggac gcc                                            23


<210> 612
<211> 22

<212> DNA
<213> Artificial Sequence

<220>
<223> Description of Artificial Sequence:
      oligonucleotide primer

<400> 612
cagctggtgg acatccggta ct                                          22


<210> 613
<211> 24
<212> DNA
<213> Artificial Sequence

<220>
<223> Description of Artificial Sequence:
      oligonucleotide primer

<400> 613
gtactccccc agccagtgct acct                                        24


<210> 614
<211> 22
<212> DNA
<213> Artificial Sequence

<220>
<223> Description of Artificial Sequence:
      oligonucleotide primer

<400> 614
ctctcttcat ctggccgtga cc                                          22


<210> 615
<211> 23
<212> DNA
<213> Artificial Sequence

<220>
<223> Description of Artificial Sequence:
      oligonucleotide primer

<400> 615
tctggatgaa tacagaagcg ccc                                         23


<210> 616
<211> 23
<212> DNA
<213> Artificial Sequence

<220>
<223> Description of Artificial Sequence:
      oligonucleotide primer

<400> 616
gcgggtcaat cagaagctca aac                                         23


<210> 617
<211> 24
<212> DNA
<213> Artificial Sequence

<220>

<223> Description of Artificial Sequence:
      oligonucleotide primer

<400> 617
cttggagtac cggatgtcca ccag                                              24


<210> 618
<211> 27
<212> DNA
<213> Artificial Sequence

<220>
<223> Description of Artificial Sequence:
      oligonucleotide primer

<400> 618
agggagaggt agctgggcag gtactga                                           27


<210> 619
<211> 25
<212> DNA
<213> Artificial Sequence

<220>
<223> Description of Artificial Sequence:
      oligonucleotide primer

<400> 619
cagatgaaga gaggtcacac acacg                                             25


<210> 620
<211> 35
<212> DNA
<213> Artificial Sequence

<220>
<223> Description of Artificial Sequence:
      oligonucleotide primer

<400> 620
aagcttatct ggcagtgtgg tggggttctg gaaac                                  35


<210> 621
<211> 39
<212> DNA
<213> Artificial Sequence

<220>
<223> Description of Artificial Sequence:
      oligonucleotide primer

<400> 621
ctcgagtaac atgcgctctt tgaagaacca tttctgatg                              39


<210> 622
<211> 25
<212> DNA
<213> Artificial Sequence

<220>
<223> Description of Artificial Sequence:
      oligonucleotide primer

<400> 622

tcttggagac tgtgtatcca gaact 25

<210> 623
<211> 27
<212> DNA
<213> Artificial Sequence

<220>
<223> Description of Artificial Sequence:
      oligonucleotide primer

<400> 623
cattgctgtc ggaagacagg aatggat 27

<210> 624
<211> 27
<212> DNA
<213> Artificial Sequence

<220>
<223> Description of Artificial Sequence:
      oligonucleotide primer

<400> 624
gaaccacttg aagtctttac actggag 27

<210> 625
<211> 27
<212> DNA
<213> Artificial Sequence

<220>
<223> Description of Artificial Sequence:
      oligonucleotide primer

<400> 625
cagccctcag gctggtgggt gttatag 27

<210> 626
<211> 37
<212> DNA
<213> Artificial Sequence

<220>
<223> Description of Artificial Sequence:
      oligonucleotide primer

<400> 626
ggatccatct cggtgtgggt cccggaaaat gcacctg 37

<210> 627
<211> 37
<212> DNA
<213> Artificial Sequence

<220>
<223> Description of Artificial Sequence:
      oligonucleotide primer

<400> 627
ctcgagatta gcaaggatgg tgagcttgtg ggactcc 37

<210> 628

<211> 21
<212> DNA
<213> Artificial Sequence

<220>
<223> Description of Artificial Sequence:
      oligonucleotide primer

<400> 628
cagggaaacc caggaccagt a                                                21

<210> 629
<211> 23
<212> DNA
<213> Artificial Sequence

<220>
<223> Description of Artificial Sequence:
      oligonucleotide primer

<400> 629
tccaggctgt ggataaagac ttg                                              23

<210> 630
<211> 22
<212> DNA
<213> Artificial Sequence

<220>
<223> Description of Artificial Sequence:
      oligonucleotide primer

<400> 630
tgtccaaagc acccatgaca at                                               22

<210> 631
<211> 20
<212> DNA
<213> Artificial Sequence

<220>
<223> Description of Artificial Sequence:
      oligonucleotide primer

<400> 631
gacagctgca attggggtgc                                                  20

<210> 632
<211> 33
<212> DNA
<213> Artificial Sequence

<220>
<223> Description of Artificial Sequence:
      oligonucleotide primer

<400> 632
ggatccctga taccgcggag cgcgcaggtg agg                                   33

<210> 633
<211> 36
<212> DNA
<213> Artificial Sequence

<220>
<223> Description of Artificial Sequence:
      oligonucleotide primer

<400> 633
ctcgagcaaa cttaagtcaa caattatatg ttcaaa                                36


<210> 634
<211> 27
<212> DNA
<213> Artificial Sequence

<220>
<223> Description of Artificial Sequence:
      oligonucleotide primer

<400> 634
tgttcattga agggaacaaa atagaaa                                          27


<210> 635
<211> 27
<212> DNA
<213> Artificial Sequence

<220>
<223> Description of Artificial Sequence:
      oligonucleotide primer

<400> 635
aggaaaagaa gctaaatgac gtgacca                                          27


<210> 636
<211> 24
<212> DNA
<213> Artificial Sequence

<220>
<223> Description of Artificial Sequence:
      oligonucleotide primer

<400> 636
atcaggtctt tgtggtggta gccc                                             24


<210> 637
<211> 27
<212> DNA
<213> Artificial Sequence

<220>
<223> Description of Artificial Sequence:
      oligonucleotide primer

<400> 637
atgcggagtt tgttgatatc gatggaa                                          27


<210> 638
<211> 25
<212> DNA
<213> Artificial Sequence

<220>
<223> Description of Artificial Sequence:
      oligonucleotide primer

<400> 638
atgaccctgc agccctttc tttta                                          25

<210> 639
<211> 27
<212> DNA
<213> Artificial Sequence

<220>
<223> Description of Artificial Sequence:
      oligonucleotide primer

<400> 639
aggtattcaa gatgaaaaag tccagca                                       27

<210> 640
<211> 27
<212> DNA
<213> Artificial Sequence

<220>
<223> Description of Artificial Sequence:
      oligonucleotide primer

<400> 640
tgatactctg gtggaccaat acacagc                                       27

<210> 641
<211> 24
<212> DNA
<213> Artificial Sequence

<220>
<223> Description of Artificial Sequence:
      oligonucleotide primer

<400> 641
catcgatgag aatggcctta cagc                                          24

<210> 642
<211> 27
<212> DNA
<213> Artificial Sequence

<220>
<223> Description of Artificial Sequence:
      oligonucleotide primer

<400> 642
tgaaccactc gtgcagtgac tggtaag                                       27

<210> 643
<211> 25
<212> DNA
<213> Artificial Sequence

<220>
<223> Description of Artificial Sequence:
      oligonucleotide primer

<400> 643
gatggaagag cttggatctc cacaa                                         25

<210> 644
<211> 36
<212> DNA
<213> Artificial Sequence

<220>
<223> Description of Artificial Sequence:
      oligonucleotide primer

<400> 644
ggatccttga ttgaaagtgg gaaggaagaa ggaatg                                36


<210> 645
<211> 39
<212> DNA
<213> Artificial Sequence

<220>
<223> Description of Artificial Sequence:
      oligonucleotide primer

<400> 645
ctcgagatgc ttcagccacg ggtgggcttc aatggaagc                             39


<210> 646
<211> 23
<212> DNA
<213> Artificial Sequence

<220>
<223> Description of Artificial Sequence:
      oligonucleotide primer

<400> 646
ctctgtatgc acaggaccct tcc                                              23


<210> 647
<211> 21
<212> DNA
<213> Artificial Sequence

<220>
<223> Description of Artificial Sequence:
      oligonucleotide primer

<400> 647
caaactgcac gacatcgacg g                                                21


<210> 648
<211> 19
<212> DNA
<213> Artificial Sequence

<220>
<223> Description of Artificial Sequence:
      oligonucleotide primer

<400> 648
ctcccgtttc ttggcgtcg                                                   19


<210> 649
<211> 20
<212> DNA
<213> Artificial Sequence

<220>
<223> Description of Artificial Sequence:
      oligonucleotide primer

<400> 649
ggtttggcag ttcccacatt                                                    20


<210> 650
<211> 40
<212> DNA
<213> Artificial Sequence

<220>
<223> Description of Artificial Sequence:
      oligonucleotide primer

<400> 650
ggatccaaat cgtgtgctcc aaataaagca gatgtcattc                              40


<210> 651
<211> 33
<212> DNA
<213> Artificial Sequence

<220>
<223> Description of Artificial Sequence:
      oligonucleotide primer

<400> 651
ctcgagtccc ccggtctggt ctcgcaggag gcg                                     33


<210> 652
<211> 29
<212> DNA
<213> Artificial Sequence

<220>
<223> Description of Artificial Sequence:
      oligonucleotide primer

<400> 652
aaaaaccagc ctgtcaacta ctccttctc                                          29


<210> 653
<211> 24
<212> DNA
<213> Artificial Sequence

<220>
<223> Description of Artificial Sequence:
      oligonucleotide primer

<400> 653
acttcatgta tcccttgcag tggc                                               24


<210> 654
<211> 23
<212> DNA
<213> Artificial Sequence

<220>
<223> Description of Artificial Sequence:
      oligonucleotide primer

<400> 654
gtggtgtcat tggaaacgat gtg                                                23

<210> 655
<211> 24
<212> DNA
<213> Artificial Sequence

<220>
<223> Description of Artificial Sequence:
      oligonucleotide primer

<400> 655
atacatgaag tcagccgagg gggt                                               24

## Description

### BACKGROUND OF THE INVENTION

The invention generally relates to nucleic acids and polypeptides encoded therefrom. More specifically, the invention relates to nucleic acids encoding cytoplasmic, nuclear, membrane bound, and secreted polypeptides, as well as vectors, host cells, antibodies, and recombinant methods for producing these nucleic acids and polypeptides.

### SUMMARY OF THE INVENTION

The invention is based in part upon the discovery of nucleic acid sequences encoding novel polypeptides. The novel nucleic acids and polypeptides are referred to herein as NOVX, or NOV1-NOV91 nucleic acids and polypeptides. These nucleic acids and polypeptides, as well as derivatives, homologs, analogs and fragments thereof, will hereinafter be collectively designated as "NOVX" nucleic acid or polypeptide sequences.

In one aspect, the invention provides an isolated NOVX nucleic acid molecule encoding a NOVX polypeptide that includes a nucleic acid sequence that has identity to the nucleic acids disclosed in SEQ ID NOS: 2*n*-1, wherein *n* is any integer between 1 and 107. In some embodiments, the NOVX nucleic acid molecule will hybridize under stringent conditions to a nucleic acid sequence complementary to a nucleic acid molecule that includes a protein-coding sequence of a NOVX nucleic acid sequence. The invention also includes an isolated nucleic acid that encodes a NOVX polypeptide; or a fragment, homolog, analog or derivative thereof. For example, the nucleic acid can encode a polypeptide at least 80% identical to a polypeptide comprising the amino acid sequences of SEQ ID NOS: 2*n*, where *n* is any integer between 1 and 107. The nucleic acid can be, for example, a genomic DNA fragment or a cDNA molecule that includes the nucleic acid sequence of any of SEQ ID NOS:2n-1.

Also included in the invention is an oligonucleotide, *e.g.,* an oligonucleotide which includes at least 6 contiguous nucleotides of a NOVX nucleic acid (*e.g.*, SEQ ID NOS:2*n*-1) or a complement of said oligonucleotide.

Also included in the invention are substantially purified NOVX polypeptides (SEQ ID NOS:2*n*). In certain embodiments, the NOVX polypeptides include an amino acid sequence that is substantially identical to the amino acid sequence of a human NOVX polypeptide. polypeptides, or fragments, homologs, analogs or derivatives thereof.

In another aspect, the invention includes pharmaceutical compositions that include therapeutically- or prophylactically-effective amounts of a therapeutic and a pharmaceutically-acceptable carrier. The therapeutic can be, *e.g.,* a NOVX nucleic acid, a NOVX polypeptide, or an antibody specific for a NOVX polypeptide. In a further aspect, the invention includes, in one or more containers, a therapeutically- or prophylactically-effective amount of this pharmaceutical composition.

In a further aspect, the invention includes a method of producing a polypeptide by culturing a cell that includes a NOVX nucleic acid, under conditions allowing for expression of the NOVX polypeptide encoded by the DNA. If desired, the NOVX polypeptide can then be recovered.

In another aspect, the invention includes a method of detecting the presence of a NOVX polypeptide in a sample. In the method, a sample is contacted with a compound that selectively binds to the polypeptide under conditions allowing for formation of a complex between the polypeptide and the compound. The complex is detected, if present, thereby identifying the NOVX polypeptide within the sample.

The invention also includes methods to identify specific cell or tissue types based on their expression of a NOVX.

Also included in the invention is a method of detecting the presence of a NOVX nucleic acid molecule in a sample by contacting the sample with a NOVX nucleic acid probe or primer, and detecting whether the nucleic acid probe or primer bound to a NOVX nucleic acid molecule in the sample.

In a further aspect, the invention provides a method for modulating the activity of a NOVX polypeptide by contacting a cell sample that includes the NOVX polypeptide with a compound that binds to the NOVX polypeptide in an amount sufficient to modulate the activity of said polypeptide. The compound can be, *e.g.*, a small molecule, such as a nucleic acid, peptide, polypeptide, peptidomimetic, carbohydrate, lipid or other organic (carbon containing) or inorganic molecule, as further described herein.

Also within the scope of the invention is the use of a therapeutic in the manufacture of a medicament for treating or preventing disorders or syndromes including, *e.g.*, cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, hypercoagulation, by heart failure and hypertension, hypotension, angina pectoris, myocardial infarction, tuberous sclerosis, scleroderma, transplantation, autoimmune disease, lupus erythematosus, viral/bacterial/parasitic infections, multiple sclerosis, autoimmume disease, allergies, immunodeficiencies, graft versus host disease, asthma, emphysema, ARDS, inflammation and modulation of the immune response, viral pathogenesis, aging-related disorders, Th1 inflammatory diseases such as rheumatoid arthritis, multiple sclerosis, inflammatory bowel diseases, AIDS, wound repair, obesity, diabetes, endocrine disorders, anorexia, bulimia, renal artery stenosis, interstitial nephritis, glomerulonephritis, polycystic kidney disease, systemic, renal tubular acidosis, IgA nephropathy, nephrological disesases, hypercalceimia, Lesch-Nyhan syndrome, Von Hippel-Lindau (VHL) syndrome, trauma, regeneration (in vitro and in vivo), Hirschsprung's disease, Crohn's Disease, appendicitis, endometriosis, laryngitis, psoriasis, actinic keratosis, acne, hair growth/loss, allopecia, pigmentation disorders, myasthenia gravis, alpha-mannosidosis, beta-mannosidosis, other storage disorders, peroxisomal disorders such as zellweger syndrome, infantile refsum disease, rhizomelic chondrodysplasia (chondrodysplasia punctata, rhizomelic), and hyperpipecolic acidemia, osteoporosis, muscle disorders, urinary retention, Albright Hereditary Ostoeodystrophy, ulcers, Alzheimer's disease, stroke, Parkinson's disease, Huntington's disease, cerebral palsy, epilepsy, Lesch-Nyhan syndrome, multiple sclerosis, ataxia-telangiectasia, behavioral disorders, addiction, anxiety, pain, neuroprotection, Stroke, Aphakia, neurodegenerative disorders, neurologic disorders, developmental defects, conditions associated with the role of GRK2 in brain and in the regulation of chemokine receptors, encephalomyelitis, anxiety, schizophrenia, manic depression, delirium, dementia, severe mental retardation and dyskinesias, Gilles de la Tourette syndrome, leukodystrophies, cancers, breast cancer, CNS cancer, colon cancer, gastric cancer, lung cancer, melanoma, ovarian cancer, pancreatic cancer, kidney cancer, colon cancer, prostate cancer, neuroblastoma, and cervical cancer, Neoplasm; adenocarcinoma, lymphoma; uterus cancer, benign prostatic hypertrophy, fertility, control of growth and development/differentiation related functions such as but not limited maturation, lactation and puberty, reproductive malfunction, and/or other pathologies and disorders of the like.

The therapeutic can be, *e.g.*, a NOVX nucleic acid, a NOVX polypeptide, or a NOVX-specific antibody, or biologically-active derivatives or fragments thereof.

For example, the compositions of the present invention will have efficacy for treatment of patients suffering from the diseases and disorders disclosed above and/or other pathologies specific for the invention, and as vaccines. They can also be used to screen for potential agonist and antagonist compounds. For example, a cDNA encoding NOVX may be useful in gene therapy, and NOVX may be useful when administered to a subject in need thereof. By way of non-limiting example, the compositions of the present invention will have efficacy for treatment of patients suffering from the diseases and disorders disclosed above and/or other pathologies and disorders of the like.

The invention further includes a method for screening for a modulator of disorders or syndromes including, *e.g.,* the diseases and disorders disclosed above and/or other pathologies and disorders of the like. The method includes contacting a test compound with a NOVX polypeptide and determining if the test compound binds to said NOVX polypeptide. Binding of the test compound to the NOVX polypeptide indicates the test compound is a modulator of activity, or of latency or predisposition to the aforementioned disorders or syndromes.

Also within the scope of the invention is a method for screening for a modulator of activity, or of latency or predisposition to disorders or syndromes including, *e.g.,* the diseases and disorders disclosed above and/or other pathologies and disorders of the like by administering a test compound to a test animal at increased risk for the aforementioned disorders or syndromes. The test animal expresses a recombinant polypeptide encoded by a NOVX nucleic acid. Expression or activity of NOVX polypeptide is then measured in the test animal, as is expression or activity of the protein in a control animal which recombinantly-expresses NOVX polypeptide and is not at increased risk for the disorder or syndrome. Next, the expression of NOVX polypeptide in both the test animal and the control animal is compared. A change in the activity of NOVX polypeptide in the test animal relative to the control animal indicates the test compound is a modulator of latency of the disorder or syndrome.

In yet another aspect, the invention includes a method for determining the presence of or predisposition to a disease associated with altered levels of a NOVX polypeptide, a NOVX nucleic acid, or both, in a subject (e.g., a human subject). The method includes measuring the amount of the NOVX polypeptide in a test sample from the subject and comparing the amount of the polypeptide in the test sample to the amount of the NOVX polypeptide present in a control sample. An alteration in the level of the NOVX polypeptide in the test sample as compared to the control sample indicates the presence of or predisposition to a disease in the subject. Preferably, the predisposition includes, *e.g.,* the diseases and disorders disclosed above and/or other pathologies and disorders of the like. Also, the expression levels of the new to determine the stage of cancers.

In a further aspect, the invention includes a method of treating or preventing a pathological condition associated with a disorder in a mammal by administering to the subj ect a NOVX polypeptide, a NOVX nucleic acid, or a NOVX-specific antibody to a subject (*e.g.*, a human subject), in an amount sufficient to alleviate or prevent the pathological condition. In preferred embodiments, the disorder, includes, *e.g.,* the diseases and disorders disclosed above and/or other pathologies and disorders of the like.

In yet another aspect, the invention can be used in a method to identity the cellular receptors and downstream effectors of the invention by any one of a number of techniques commonly employed in the art. These include but are not limited to the two-hybrid system, affinity purification, co-precipitation with antibodies or other specific-interacting molecules.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In the case of conflict, the present specification, including definitions, will control. In addition, the materials, methods, and examples are illustrative only and not intended to be limiting.

Other features and advantages of the invention will be apparent from the following detailed description and claims.

### DETAILED DESCRIPTION OF THE INVENTION

The present invention provides novel nucleotides and polypeptides encoded thereby. Included in the invention are the novel nucleic acid sequences and their encoded polypeptides. The sequences are collectively referred to herein as "NOVX nucleic acids" or "NOVX polynucleotides" and the corresponding encoded polypeptides are referred to as "NOVX polypeptides" or "NOVX proteins." Unless indicated otherwise, "NOVX" is meant to refer to any of the novel sequences disclosed herein. Table A provides a summary of the NOVX nucleic acids and their encoded polypeptides.

**TABLE A. Sequences and Corresponding SEQ ID Numbers**

| **NOVX Assignment** | **Internal Identification** | **SEQ ID NO (nucleic acid)** | **SEQ ID NO (polypeptide)** | **Homology** |
|---|---|---|---|---|
| 1 | CG57602-01 | 1 | 2 | DJ0751H13.1 Protein |
| 2 | CG57558-01 | 3 | 4 | Mac25/IGFBP7 |
| 3 | CG57560-01 | 5 | 6 | Calmodulin Binding Protein Kinase |
| 4A | CGS7S47-O1 | 7 | 8 | TRANSIENT RECEPTOR POTENTIAL-RELATED PROTEIN |
| 4B | CGS7547-02 | 9 | 10 | TRANSIENT RECEPTOR POTENTIAL-RELATED PROTEIN |
| 5 | CGS7609-O1 | 11 | 12 | Epsin-3 |
| 6 | CGS7611-O1 | 13 | 14 | CD22, a B lymphocyte-restricted adhesion molecule |
| 7 | CGS7S9S-O1 | 15 | 16 | MEGF8 |
| 8A | CG574S2-O1 | 17 | 18 | protocadherin |
| 8B | CG57452-02 | 19 | 20 | protocadherin |
| 9 | CG57625-01 | 21 | 22 | protocadherin |
| 10 | CG57553-01 | 23 | 24 | T01C1.3 |
| 11A | CG57488_01 | 25 | 26 | alpha-macroglobulin |
| 11B | CG57488_02 | 27 | 28 | alpha-macroglobulin |
| 11C | CG57488_03 | 29 | 30 | alpha-macroglobulin |
| 12A | CG57526_01 | 31 | 32 | orphan transporter |
| 12B | CG57526-02 | 33 | 34 | orphan transporter |
| 13 | CG57570-01 | 35 | 36 | cation transporter |
| 14 | CG57593-01 | 37 | 38 | ABC transporter |
| 15 | CG57652-01 | 39 | 40 | Diacylglycerol Kinase Alpha |
| 16 | CG57562-01 | 41 | 42 | Cation-transporting ATPase |
| 17 | CG55914-01 | 43 | 44 | ACYL-COA DESATURASE |
| 18 | CG57328-01 | 45 | 46 | MYO-INOSFTOL-1(OR 4)-MONOPHOSPHATASE |
| 19 | CG57358-01 | 47 | 48 | spinster |
| 20 | CG57695-01 | 49 | 50 | casein |
| 21 | CGS76S4-01 | 51 | 52 | Gamma-Aminobutyric-Acid Receptor Gamma-2 Subunit Precursor (GABA-A Receptor) |
| 22 | CGS7724-01 | 53 | 54 | Carboxylesterase |
| 23 | CG57730-01 | 55 | 56 | MAT-1 oncogene |
| 24 | CG5755-01 | 57 | 58 | VACUOLAR PROTON-ATPASE SUBUNIT H |
| 25 | CG57S03-O1 | 59 | 60 | MEGF7 |
| 26 | CG57456-01 | 61 | 62 | COP-COATED VESICLE MEMBRANE PROTEIN P24 PRECURSOR |
| 27 | CG57658-01 | 63 | 64 | Connexin |
| 28 | CG57662-01 | 65 | 66 | Connexin |
| 29 | CG57664-01 | 67 | 68 | the MHC CLASS I ANTIGEN |
| 30 | CG57666-01 | 69 | 70 | the MHC CLASS I ANTIGEN |
| 31 | CG57668-01 | 71 | 72 | the MHC CLASS I ANTIGEN |
| 32 | CG57660-01 | 73 | 74 | RETINOIC ACID RECEPTOR RESPONDER |
| 33 | CG57672-01 | 75 | 76 | PHOSPHATIDYLINOSITOL 4-PHOSPHATE 5-KINASE |
| 34 | CGS7680-01 | 77 | 78 | Cyclophilin-type peptidyl-prolyl cis-trans isomerase |
| 35 | CGS7670-01 | 79 | 80 | pyruvate kinase |
| 36A | CGS7149_01 | 81 | 82 | Cis/Trans Peptidyl Prolyl Isomerase |
| 36B | CG57149_02 | 83 | 84 | Cis/Trans Peptidyl Prolyl Isomerase |
| 37 | CG57151_01 | 85 | 86 | Cis/Trans Peptidyl Prolyl Isomerase |
| 38 | CG57153_01 | 87 | 88 | Cis/Trans Peptidyl Prolyl Isomerase |
| 39 | CG57155_01 | 89 | 90 | Cis/Trans Peptidyl Prolyl Isomerase |
| 40 | CG57157_01 | 91 | 92 | Cis/Trans Peptidyl Prolyl Isomerase |
| 41 | CG57159_01 | 93 | 94 | Cis/Trans Peptidyl Prolyl Isomerase |
| 42A | CG57226_01 | 95 | 96 | Cis/Trans Peptidyl Prolyl Isomerase |
| 42B | CG57226-02 | 97 | 98 | Cis/Trans Peptidyl Prolyl Isomerase |
| 43 | CG57538-01 | 99 | 100 | Ceruloplasmin |
| 44A | CG57623-01 | 101 | 102 | Leucine Rich Repeat |
| 44B | CG57623-02 | 103 | 104 | Leucine Rich Repeat |
| 45A | CG57656-01 | 105 | 106 | Ig/Fibronectin domain |
| 45B | CG57656-02 | 107 | 108 | Ig/Fibronectin domain |
| 46 | CG57682-01 | 109 | 110 | G2/MITOTIC-SPECIFIC CYCLIN B2 |
| 47 | CG57764-01 | 111 | 112 | ALR |
| 48 | CG57713-01 | 113 | 114 | SODIUM/BILE ACID COTRANSPORTER |
| 49 | CG57721-01 | 115 | 116 | Prestin |
| 50 | CG57787 01 | 117 | 118 | Sulfate Transporter |
| 51 | CG57785 01 | 119 | 120 | Sulfate Transporter |
| 52 | CG57748-01 | 121 | 122 | N-acetylgalactosaminyltransferase |
| 53 | CG57693-01 | 123 | 124 | Protein Kinase |
| 54 | CG57707-01 | 125 | 126 | Leucine-rich glioma-inactivated protein precursor |
| 55 | CG57306-01 | 127 | 128 | Anion exchanger |
| 56 | CG57348 01 | 129 | 130 | PR SET domain protein |
| 57 | CG57650-01 | 131 | 132 | NONMUSCLE MYOSIN HEAVY CHAIN B |
| 58 | CG57766-01 | 133 | 134 | Plasma retinol binding protein |
| 59 | CG57566-01 | 135 | 136 | HIV-1 inducer of short transcripts binding protein |
| 60A | CG57574-01 | 137 | 138 | Beta tectorin |
| 60B | CG57574-02 | 139 | 140 | Beta tectorin |
| 60C | CG57574-03 | 141 | 142 | Beta tectorin |
| 61 | CG57505-01 | 143 | 144 | KIAA1125 |
| 62A | CG57473-01 | 145 | 146 | Zinc-finger protein BOP |
| 62B | CG57473-02 | 147 | 148 | Zinc-finger protein BOP |
| 63 | CG57777_01 | 149 | 150 | Hypothetical secreted protein |
| 64 | CG57779_01 | 151 | 152 | Hypothetical secreted protein |
| 65 | CG57781_01 | 153 | 154 | Hypothetical secreted protein |
| 66 | G57783_01 | 155 | 156 | Hypothetical secreted protein |
| 67A | CG57823-01 | 157 | 158 | ACYLTRANSFERASE |
| 67B | CG57823-02 | 159 | 160 | ACYLTRANSFERASE |
| 68 | CG57801-01 | 161 | 162 | guanine nucleotide exchange factor |
| 69A | CG57719-01 | 163 | 164 | Aspartate Aminotransferase |
| 69B | CG57719-02 | 165 | 166 | Aspartate Aminotransferase |
| 70 | CG57462-01 | 167 | 168 | KIAA1337 |
| 71 | CG57584-01 | 169 | 170 | ZONA PELLUCIDA GLYCOPROTEIN PRECURSOR |
| 72 | CG56761-01 | 171 | 172 | Ankyrin repeat containing protein |
| 73 | CG57313 01 | 173 | 174 | GPCR |
| 74 | CG57315 01 | 175 | 176 | GPCR |
| 75 | CG57317 01 | 177 | 178 | GPCR |
| 76 | CG57321 01 | 179 | 180 | GPCR |
| 77 | CG57419 01 | 181 | 182 | GPCR |
| 78 | CG57425-01 | 183 | 184 | GPCR |
| 79 | CG57753-01 | 185 | 186 | GPCR |
| 80 | CG56766-01 | 187 | 188 | GPCR |
| 81A | CG57847-01 | 189 | 190 | GPCR |
| 81B | CG57847-02 | 191 | 192 | GPCR |
| 82 | CG57845-01 | 193 | 194 | GPCR |
| 83 | CG57843-01 | 195 | 196 | GPCR |
| 84A | CG57841-01 | 197 | 198 | GPCR |
| 84B | CG57841-02 | 199 | 200 | GPCR |
| 85 | CG57839-01 | 201 | 202 | GPCR |
| 86 | CG57837-01 | 203 | 204 | GPCR |
| 87 | CG56763-01 | 205 | 206 | GPCR |
| 88 | CG56753-01 | 207 | 208 | GPCR |
| 89 | CG57670-01 | 209 | 210 | GPCR |
| 90 | CG57676-01 | 211 | 212 | GPCR |
| 91 | CG57678-01 | 213 | 214 | GPCR |

NOVX nucleic acids and their encoded polypeptides are useful in a variety of applications and contexts. The various NOVX nucleic acids and polypeptides according to the invention are useful as novel members of the protein families according to the presence of domains and sequence relatedness to previously described proteins. Additionally, NOVX nucleic acids and polypeptides can also be used to identify proteins that are members of the family to which the NOVX polypeptides belong.

Table A indicates homology of NOVX nucleic acids to known protein families. Thus, the nucleic acids and polypeptides, antibodies and related compounds according to the invention corresponding to a NOVX as identified in column 1 of Table A, will be useful in therapeutic and diagnostic applications implicated in, for example, pathologies and disorders associated the the known protein families identified in column 5 of Table A.

The NOVX nucleic acids and polypeptides can also be used to screen for molecules, which inhibit or enhance NOVX activity or function. Specifically, the nucleic acids and polypeptides according to the invention may be used as targets for the identification of small molecules that modulate or inhibit, *e.g.,* neurogenesis, cell differentiation, cell proliferation, hematopoiesis, wound healing and angiogenesis.

Additional utilities for the NOVX nucleic acids and polypeptides according to the invention are disclosed herein.

### NOV1

A disclosed NOV1 nucleic acid of 12660 nucleotides (also referred to as CG57602-01) encoding a DJ0751H13.1 PROTEIN-like protein is shown in Table 1A. An open reading frame was identified beginning with an ATG initiation codon at nucleotides 1-3 and ending with a TAA codon at nucleotides 12658-12660. The start and stop codons are in bold letters.

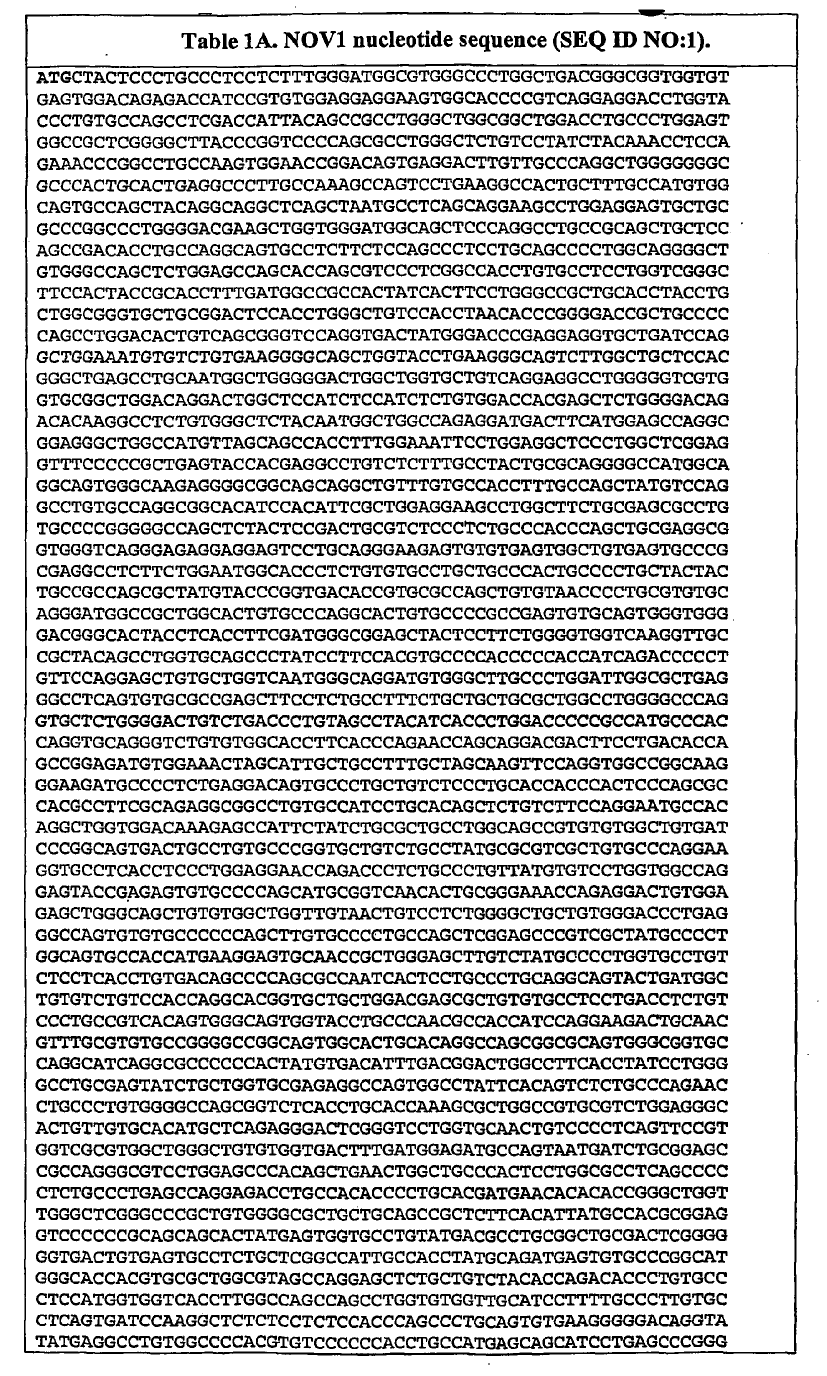

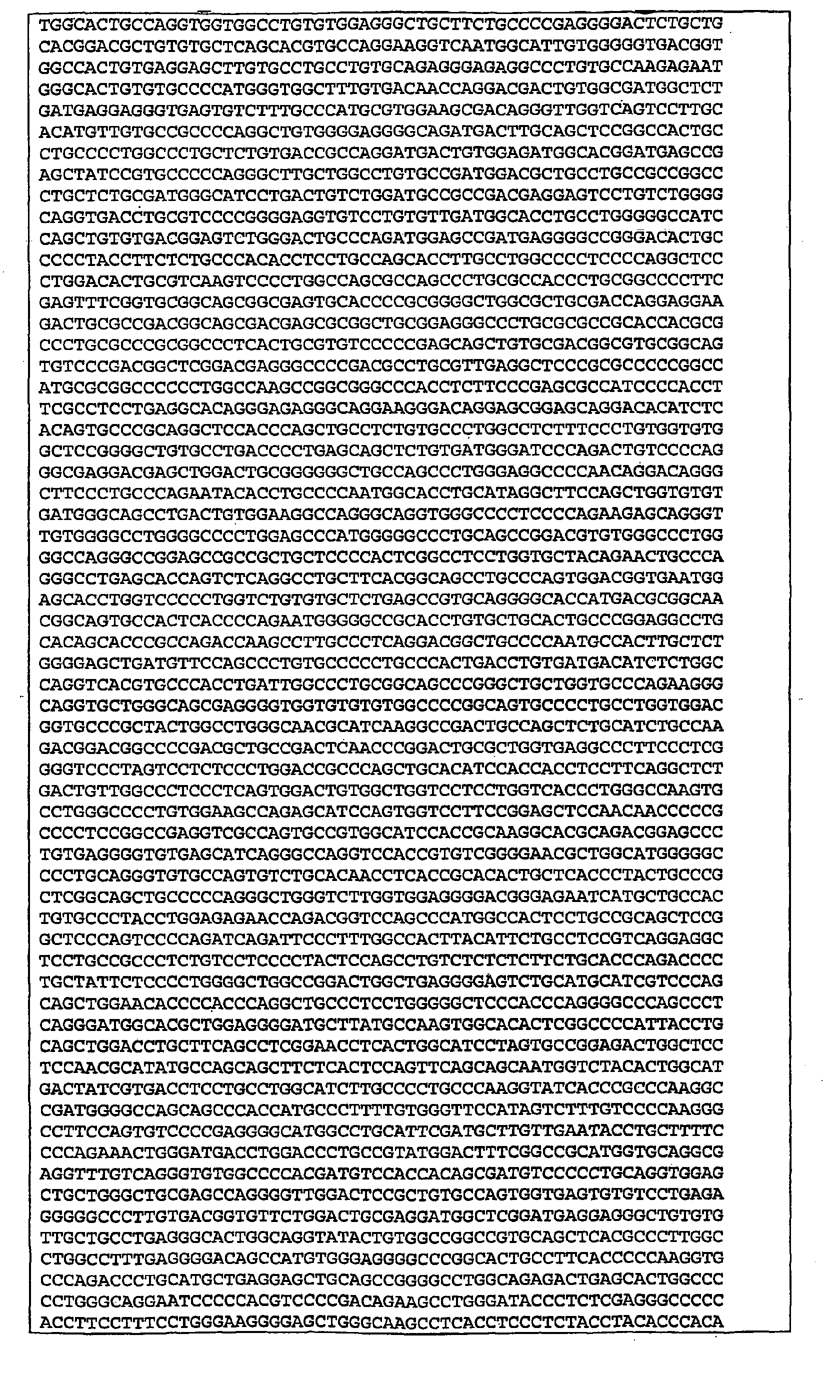

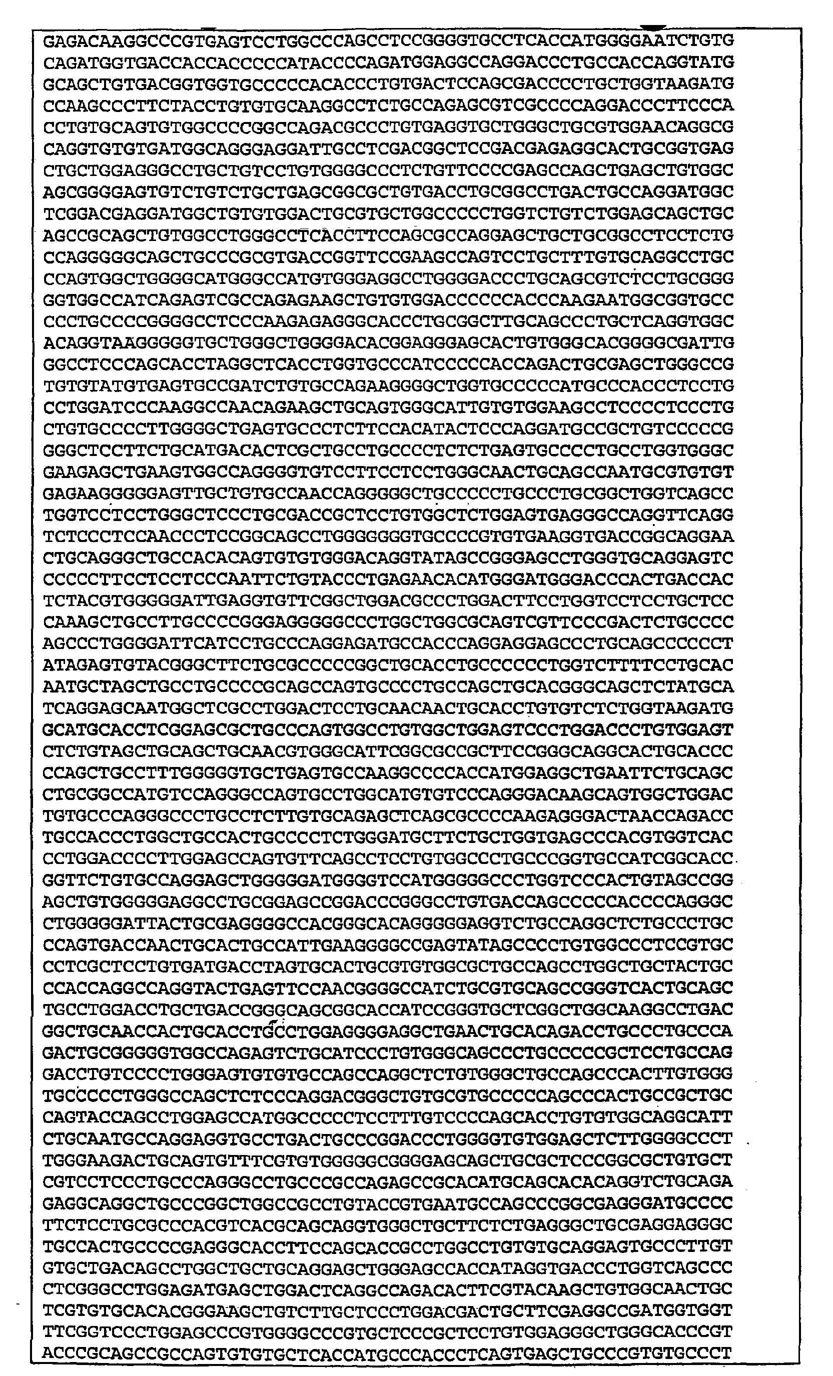

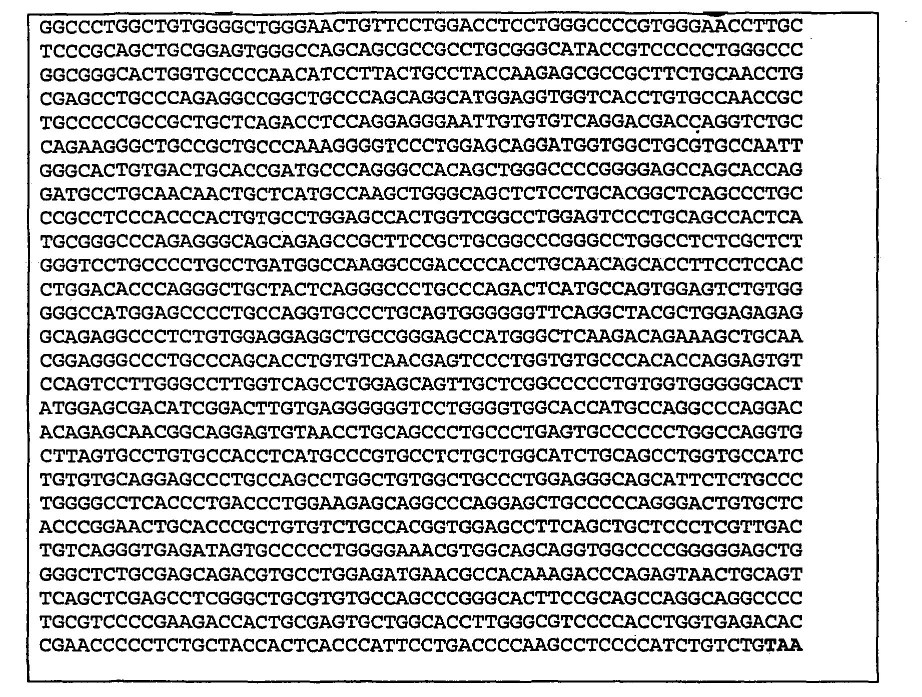

In a search of public sequence databases, the NOV1 nucleic acid sequence, located on chromsome 8 has 606 of 779 bases (77%) identical to a gb:GENBANK-ID:BTSCOSPONlacc:X93922.1 mRNA from Bos taurus (B.taurus mRNA for SCO-spondin protein). Public nucleotide databases include all GenBank databases and the GeneSeq patent database.

In all BLAST alignments herein, the "E-value" or "Expect" value is a numeric indication of the probability that the aligned sequences could have achieved their similarity to the BLAST query sequence by chance alone, within the database that was searched. For example, the probability that the subject ("Sbjct") retrieved from the NOV1 BLAST analysis, *e.g.*, B.taurus mRNA for SCO-spondin protein, matched the Query NOV1 sequence purely by chance is 2.4e-152. The Expect value (E) is a parameter that describes the number of hits one can "expect" to see just by chance when searching a database of a particular size. It decreases exponentially with the Score (S) that is assigned to a match between two sequences. Essentially, the E value describes the random background noise that exists for matches between sequences.

The Expect value is used as a convenient way to create a significance threshold for reporting results. The default value used for blasting is typically set to 0.0001. In BLAST 2.0, the Expect value is also used instead of the P value (probability) to report the significance of matches. For example, an E value of one assigned to a hit can be interpreted as meaning that in a database of the current size one might expect to see one match with a similar score simply by chance. An E value of zero means that one would not expect to see any matches with a similar score simply by chance. See, e.g., http://www.ncbi.nlm.nih.gov/Education/BLASTinfo/. Occasionally, a string of X's or N's will result from a BLAST search. This is a result of automatic filtering of the query for low-complexity sequence that is performed to prevent artifactual hits. The filter substitutes any low-complexity sequence that it finds with the letter "N" in nucleotide sequence (e.g., "NNNNNNNNNNNNN") or the letter "X" in protein sequences (e.g., "XXXXXXXXX"). Low-complexity regions can result in high scores that reflect compositional bias rather than significant position-by-position alignment. (Wootton and Federhen, *Methods Enzymol* 266: 554.571, 1996).

The disclosed NOV1 polypeptide (SEQ ID NO:2) encoded by SEQ ID NO:1 has 4219 amino acid residues and is presented in Table 1B using the one-letter amino acid code. Signal P, Psort and/or Hydropathy results predict that NOV1 has a signal peptide and is likely to be localized extracellularly with a certainty of 0.5087. The most likely cleavage site for NOV1 is between positions 17 and 18.

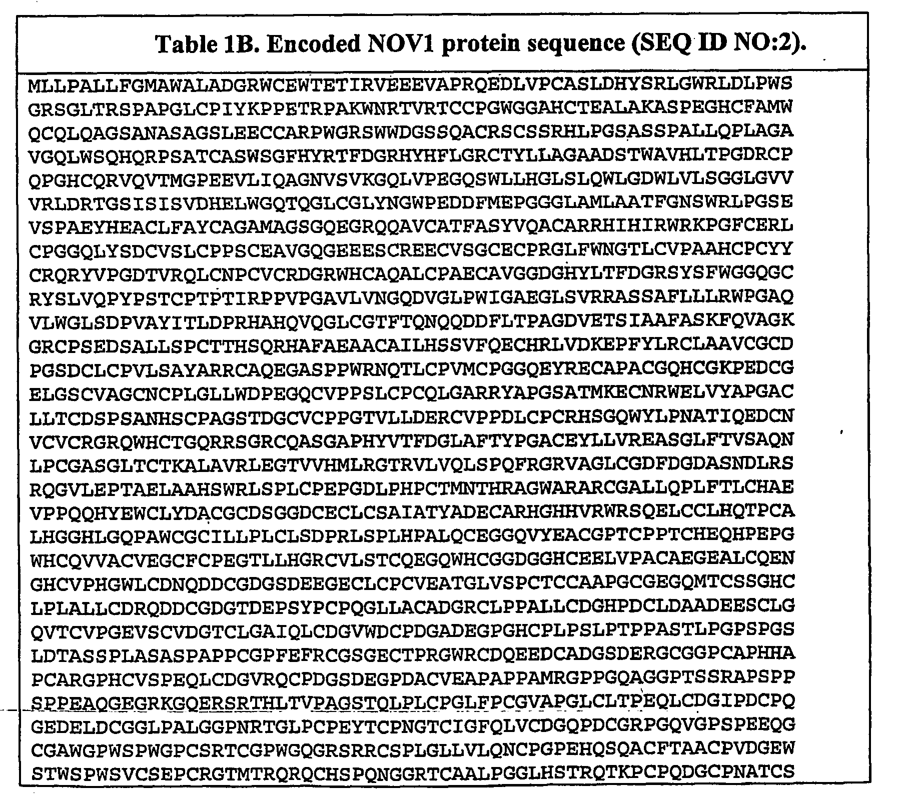

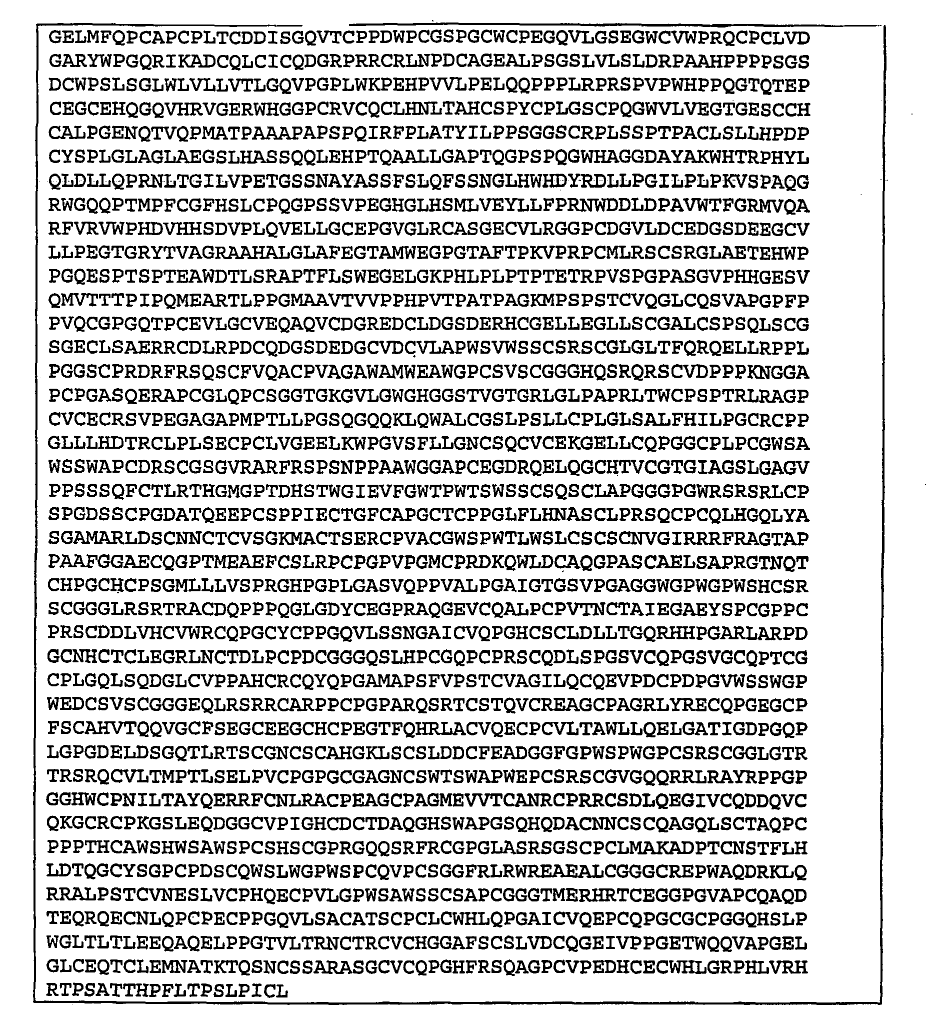

A search of sequence databases reveals that the NOV1 amino acid sequence has 4056 of 4056 amino acid residues (100%) identical to, and 4056 of 4056 amino acid residues (100%) similar to, the 4123 amino acid residue ptnr:SPTREMBL-ACC:O75851 protein from Homo sapiens (Human) (WUGSC:H_DJ0751H13.1 PROTEIN) (E = 0.0). Public amino acid databases include the GenBank databases, SwissProt, PDB and PIR.

The disclosed NOV1 polypeptide has homology to the amino acid sequences shown in the BLASTP data listed in Table 1C.

| **Table 1C. BLAST results for NOV1** | | | | | |
|---|---|---|---|---|---|
| Gene Index/ Identifier | Protein/ Organism | Length (aa) | Identity (%) | Positives (%) | Expect |
| gi\|3638957\|gb\|AAC363 01.1\| (AC004877) | sco-spondin-mucin-like; similar to P98167 (PID:g1711548); [Homo sapiens] | 4123 | 4056/405 6 (100%) | 4056/4056 (100%) | 0.0 |
| gi\|17465206\|ref\|XP 069 674.1\|(XM_069674 | similar to sco-spondin-mucin-like; similar to P98167 (PID:g1711548); (H. sapiens) | 1098 | 879/1060 (82%) | 890/1060 (83%) | 0.0 |
| gi\|3808095\|emb\|CAA69 867.1\| (Y08560) | sco-spondin [Bos taurus] | 870 | 600/990 (60%) | 633/990 (63%) | 0.0 |
| gi\|1711548\|sp\|P98167\| SSPO BOVIN | SCO-spondin, bovine | 867 | 582/1035 (56%) | 635/1035 (61%) | 0.0 |
| gi\|5823595\|emb\|CAB537 60.1\| (AJ132107) | SCO-spondin, bovine | 564 | 360/494 (72%) | 395/494 (79%) | e-173 |

The presence of identifiable domains in NOV1, as well as all other NOVX proteins, was determined by searches using software algorithms such as PROSITE, DOMAIN, Blocks, Pfam, ProDomain, and Prints, and then determining the Interpro number by crossing the domain match (or numbers) using the Interpro website (http:www.ebi.ac.uk/ interpro). DOMAIN results for NOV1 as disclosed in Tables 1D-H, were collected from the Conserved Domain Database (CDD) with Reverse Position Specific BLAST analyses. This BLAST analysis software samples domains found in the Smart and Pfam collections. For Tables 1 G-10 and all successive DOMAIN sequence alignments, fully conserved single residues are indicated by black shading or by the sign (|) and "strong" semi-conserved residues are indicated by grey shading or by the sign (+). The "strong" group of conserved amino acid residues may be any one of the following groups of amino acids: STA, NEQK, NHQK, NDEQ, QHRK, MILV, MILF, HY, FYW.

Tables 1D-H list the domain descriptions from DOMAIN analysis results for NOV1. This indicates that the NOV 1 sequence has properties similar to those of other proteins known to contain this domain.

| **Table 1D. Domain Analysis of NOV1** | | |
|---|---|---|
| gn1\|Pfam\|pfam00754, F5_F8_type_C, F5/8 type C domain. This domain is also known as the discoidin (DS) domain family. | | |
| | **CD-Length = 145 residues, 100.0% aligned** | |
| | | Score = 115 bits (288), Expect = 5e-26 |

| **Table 1E. Domain Analysis of NOV1** |
|---|
| gnl\|Pfam\|pfam00094, vwd, von Willebrand factor type D domain. |
| CD-Length = 158 residues, 99.4% aligned |
| Score = 97.1 bits (240), Expect = 2e-20 |

| **Table 1F. Domain Analysis of NOV1** |
|---|
| gnl\|Smart\|smart00231, FA58C, Coagulation factor 5/8 C-terminal domain, discoidin domain; Cell surface-attached carbohydrate-binding domain, present in eukaryotes and assumed to have horizontally transferred to eubacterial genomes. |
| CD-Length = 135 residues, 99.3% aligned |
| Score = 63.2 bits (152), Expect = 3e-10 |

| **Table 1G. Domain Analysis of NOV1** | | |
|---|---|---|
| gnl \| Smart \| smart00209, TSP1, Thrombospondin type 1 repeats; Type 1 repeats in thrombospondin-1 bind and activate TGF-beta. | | |
| | CD-Length = 51 residues, 100.0% aligned | |
| | | Score = 53.1 bits (126), Expect = 3e-07 |

| **Table 1H. Domain Analysis of NOV**1 |
|---|
| gn\|IPfam\|pfam00057, ldl_recept_a, Low-density lipoprotein receptor domain class A |
| CD-Length = 39 residues, 94.9% aligned |
| Score = 50.4 bits (119), Expect = 2e-06 |

The disclosed NOV1 protein contains a thrombospondin type I repeat domain which are found in the thrombospondin protein and is repeated 3 times. A number of proteins involved in the complement pathway (properdin, C6, C7, C8A, C8B, C9) as well as extracellular matrix protein like mindin, F-spondin, SCO-spondin and even the circumsporozoite surface protein 2 and TRAP proteins of Plasmodium contain one or more instance of this repeat. It has been involved in cell-cell interraction, inhibition of angiogenesis, apoptosis. The intron-exon organisation of the properdin gene confirms the hypothesis that the repeat might have evolved by a process involving exon shuffling. A study of properdin structure provides some information about the structure of the thrombospondin type I repeat.

The disclosed NOV1 nucleic acid of the invention encoding a DJ0751H13.1 PROTEIN -like protein includes the nucleic acid whose sequence is provided in Table 1A or a fragment thereof. The invention also includes a mutant or variant nucleic acid any of whose bases may be changed from the corresponding base shown in Table 1A while still encoding a protein that fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 27 percent of the bases may be so changed.

The disclosed NOV1 protein of the invention includes the DJ0751H13.1 PROTEIN - like protein whose sequence is provided in Table 1B. The invention also includes a mutant or variant protein any of whose residues may be changed from the corresponding residue shown in Table 1B while still encoding a protein that maintains its DJ0751H13.1 PROTEIN -like activities and physiological functions, or a functional fragment thereof. In the mutant or variant protein, up to about 0 percent of the residues may be so changed.

The invention further encompasses antibodies and antibody fragments, such as F_{ab} or (F_{ab})₂, that bind immunospecifically to any of the proteins of the invention.

The above defined information for this invention suggests that this DJ0751H13.1 PROTEIN -like protein (NOV1) may function as a member of a "DJ0751H13.1 PROTEIN family". Therefore, the NOV1 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV1 nucleic acids and proteins of the invention are useful in potential therapeutic applications implicated in cancer including but not limited to various pathologies and disorders as indicated below. For example, a cDNA encoding the DJ0751H13.1 PROTEIN -like protein (NOV1) may be useful in gene therapy, and the DJ0751H13.1 PROTEIN -like protein (NOV1) may be useful when administered to a subject in need thereof. By way of nonlimiting example, the compositions of the present invention will have hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, transplantation, diabetes, autoimmune disease, renal artery stenosis, interstitial nephritis, glomerulonephritis, polycystic kidney disease, systemic lupus erythematosus, renal tubular acidosis, IgA nephropathy, hypercalceimia, Lesch-Nyhan syndrome, Von Hippel-Lindau (VHL) syndrome, Alzheimer's disease, stroke, tuberous sclerosis, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neuroprotection, cancers, and/or other pathologies and disorders. For example, a cDNA encoding the transmembrane receptor DJ0751H13.1 PROTEIN -like protein may be useful in transmembrane receptor DJ0751H13.1 PROTEIN therapy, and the transmembrane receptor DJ0751H13.1 PROTEIN -like protein may be useful when administered to a subject in need thereof. By way of nonlimiting example, the compositions of the present invention will have efficacy for treatment of patients suffering from cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, transplantation, diabetes, autoimmune disease, renal artery stenosis, interstitial nephritis, glomerulonephritis, polycystic kidney disease, systemic lupus erythematosus, renal tubular acidosis, IgA nephropathy, hypercalceimia, Lesch-Nyhan syndrome, Von Hippel-Lindau (VHL) syndrome, Alzheimer's disease, stroke, tuberous sclerosis, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neuroprotection, cancers, and other diseases, disorders and conditions of the like. Also since this gene is expressed at a measurably higher level in several cancer cell lines (including breast cancer, CNS cancer, colon cancer, gastric cancer, lung cancer, melanoma, ovarian cancer and pancreatic cancer), it may be useful in diagnosis and treatment of these cancers. The NOV1 nucleic acid encoding the DJ0751H13.1 PROTEIN -like protein of the invention, or fragments thereof, may further be useful in diagnostic applications, wherein the presence or amount of the nucleic acid or the protein are to be assessed.

NOV1 nucleic acids and polypeptides are further useful in the generation of antibodies that bind immuno-specifically to the novel NOV1 substances for use in therapeutic or art, using prediction from hydrophobicity charts, as described in the "Anti-NOVX Antibodies" section below. The disclosed NOV proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV2

A disclosed NOV2 nucleic acid of 893 nucleotides (also referred to as CG57558-01) encoding a Mac25/IGFBP7-like protein is shown in Table 2A. Putative untranslated regions upstream and/or downstream from the coding region, if any, are underlined, and the start and stop codons are in bold letters.

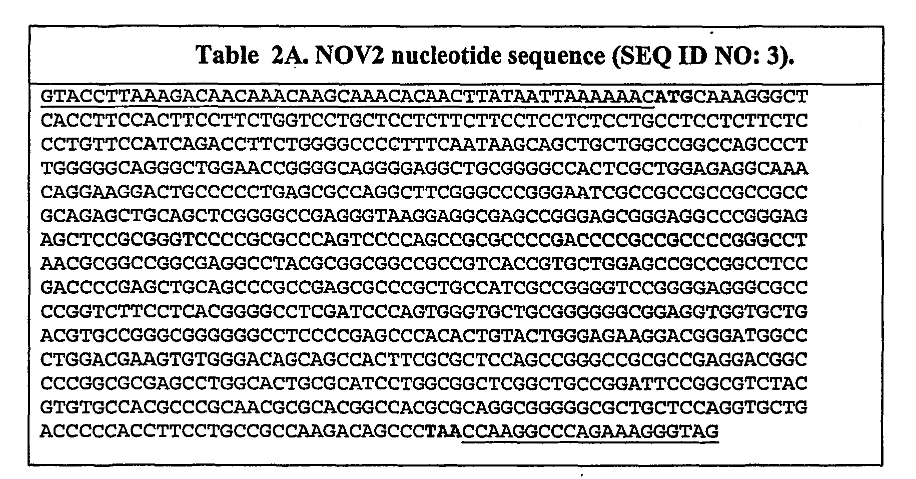

In a search of public sequence databases, the NOV2 nucleic acid sequence, located on chromsome 2 has has 564 of 779 bases (72%) identical to a gb:GENBANK-ID:S56581|acc:S56581.1 mRNA from Rattus sp. (alpha inhibin gene {5'region} [rats, Genomic, 2141 nt]). Public nucleotide databases include all GenBank databases and the GeneSeq patent database.

The disclosed NOV2 polypeptide (SEQ ID NO:4) encoded by SEQ ID NO:3 has 274 amino acid residues and is presented in Table B using the one-letter amino acid code. Signal P, Psort and/or Hydropathy results predict that NOV2 has a signal peptide and is likely to be localized extracellularly with a certainty of 0.3700. The most likely cleavage site for a NOV2 peptide is between amino acids 32 and 33.

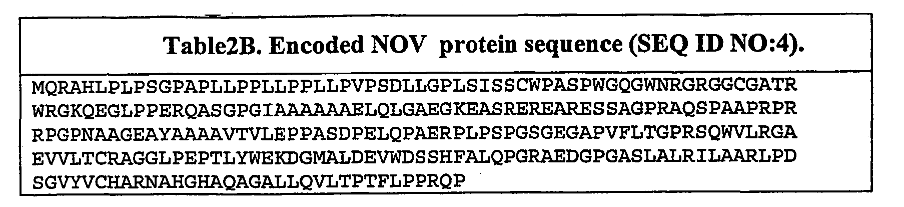

A search of sequence databases reveals that the NOV2 amino acid sequence has 80 of 266 amino acid residues (30%) identical to, and 112 of 266 amino acid residues (42%) similar to, the 277 amino acid residue ptnr:SPTREMBL-ACC:Q07822 protein from Homo sapiens (Human) (MAC25 PROTEIN). Public amino acid databases include the GenBank databases, SwissProt, PDB and PIR.

NOV2 is expressed in at least brain, ovary, breast, testis. This information was derived by determining the tissue sources of the sequences that were included in the invention including but not limited to SeqCalling sources, Public EST sources, Literature sources, and/or RACE sources.

The disclosed NOV2 polypeptide has homology to the amino acid sequences shown in the BLASTP data listed in Table 2C.

| **Table 2C. BLAST results for NOV2** | | | | | |
|---|---|---|---|---|---|
| Gene Index/ Identifier | Protein/ Organism | Length (aa) | Identity (%) | Positives (%) | Expect |
| gi\|14734071\|ref\|XP 051017.1\| (XM_051017) | KIAA0657 protein [Homo sapiens] | 1025 | 143/150 (95%) | 143/150 (95%) | 1e-66 |
| gi\|13938170\|gb\|AAH0 7201.1\|AAH07201 (BC007201) | (protein for IMAGE:2961284) [Homo sapiens] | 1044 | 143/150 (95%) | 143/150 (95%) | 4e-66 |
| gi\|18552587\|ref\|XP 087161.1\| (XM_087161) | (protein for IMAGE:2961284)[Homo sapiens] | 180 | 50/58 (86%) | 51/58 (87%) | 4e-17 |
| gi\|9623317\|gb\|AAF90 112.1\|AF254363 1 (AF254363) | stretchin-MLCK [Drosophila melanogaster] | 281 | 35/105 (33%) | 50/105 (47%) | 7e-8 |
| gi\|17559576\|ref\|NP 504582.11 (NM 072181) | titin [Caenorhabditis elegans] | 2783 | 39/120 (32%) | 57/120 (47%) | 1e-7 |

Table 2D lists the domain descriptions from DOMAIN analysis results against NOV 2. This indicates that the NOV sequence has properties similar to those of other proteins known to contain this domain.

| **Table 2D. Domain Analysis of NOV2** |
|---|
| gnl\|Smart\|smart00409, IG, Immunoglobulin |
| CD-Length = 86 residues, 100.0% aligned |
| Score = 57.4 bits (137), Expect = le-09 |

Mac25 is a follistatin (FS)-like protein thathas a growth-suppressing effect on a p53-deficient osteosarcoma cell line (Saos-2). The protein exhibits a strong homology to FS, an activin-binding protein, and part of its sequence includes the consensus sequence of the member of the Kazal serine protease inhibitor family. The mac25 protein was localized in the cytoplasm and secreted into culture medium (1). Addition of recombinant mac25 protein (10-7 M) into the culture medium induced significant suppression of the growth of human cervical carcinoma cells (HeLa) and murine embryonic carcinoma cells (P19), as well as osteosarcoma cells (Saos-2). The mac25 protein was co-immunoprecipitated with activin A, a result that suggests that mac25 may be a secreted tumor-suppressor that binds activin A. The mac25 exhibits homology to insulin-like growth factor-binding proteins (IGF-BPs) and to fibroblast growth factor receptor. The multi-functional nature of mac25 protein may be important for growth-suppression and/or cellular senescence.

The disclosed NOV2 nucleic acid of the invention encoding a Mac25/IGFBP7-like protein includes the nucleic acid whose sequence is provided in Table 2A or a fragment thereof. The invention also includes a mutant or variant nucleic acid any of whose bases may be changed from the corresponding base shown in Table 2A while still encoding a protein that maintains its Mac25/IGFBP7-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 2B percent of the bases may be so changed.

The disclosed NOV2 protein of the invention includes the Mac25/IGFBP7-like protein whose sequence is provided in Table 2B. The invention also includes a mutant or variant protein any of whose residues may be changed from the corresponding residue shown in Table physiological functions, or a functional fragment thereof. In the mutant or variant protein, up to about 70 percent of the residues may be so changed.

The invention further encompasses antibodies and antibody fragments, such as F_{ab} or (F_{ab})₂, that bind immunospecifically to any of the proteins of the invention.

The above defined information for this invention suggests that this Mac25/IGFBP7-like protein (NOV2) may function as a member of a "Mac25/IGFBP7 family". Therefore, the NOV2 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV2 nucleic acids and proteins of the invention are useful in potential therapeutic applications implicated in diseases including but not limited to various pathologies and disorders as indicated below. For example, a cDNA encoding the Mac25/IGFBP7-like protein (NOV2) may be useful in gene therapy, and the Mac25/IGFBP7-like protein (NOV2) may be useful when administered to a subject in need thereof. By way of nonlimiting example, the compositions of the present invention will have efficacy for treatment of patients suffering from Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, fertility, hypogonadism, endometriosis, hemophilia, hypercoagulation, idiopathic thrombocytopenic purpura, immunodeficiencies, graft versus host disease, or other pathologies or conditions. The NOV2 nucleic acid encoding the Mac25/IGFBP7-like protein of the invention, or fragments thereof, may further be useful in diagnostic applications, wherein the presence or amount of the nucleic acid or the protein are to be assessed.

NOV2 nucleic acids and polypeptides are further useful in the generation of antibodies that bind immuno-specifically to the novel NOV2 substances for use in therapeutic or diagnostic methods. These antibodies may be generated according to methods known in the art, using prediction from hydrophobicity charts, as described in the "Anti-NOVX Antibodies" section below. The disclosed NOV2 proteins have multiple hydrophilic regions, each of functional analysis of various human disorders, which will help in understanding of pathology of the disease and development of new drug targets for various disorders.

### NOV3

A disclosed NOV3 nucleic acid of 1703 nucleotides (also referred to as CG57560-01) encoding a Calmodulin Binding Protein Kinase-like protein is shown in Table 3A. Putative untranslated regions upstream and/or downstream from the coding region, if any, are underlined, and the start and stop codons are in bold letters.

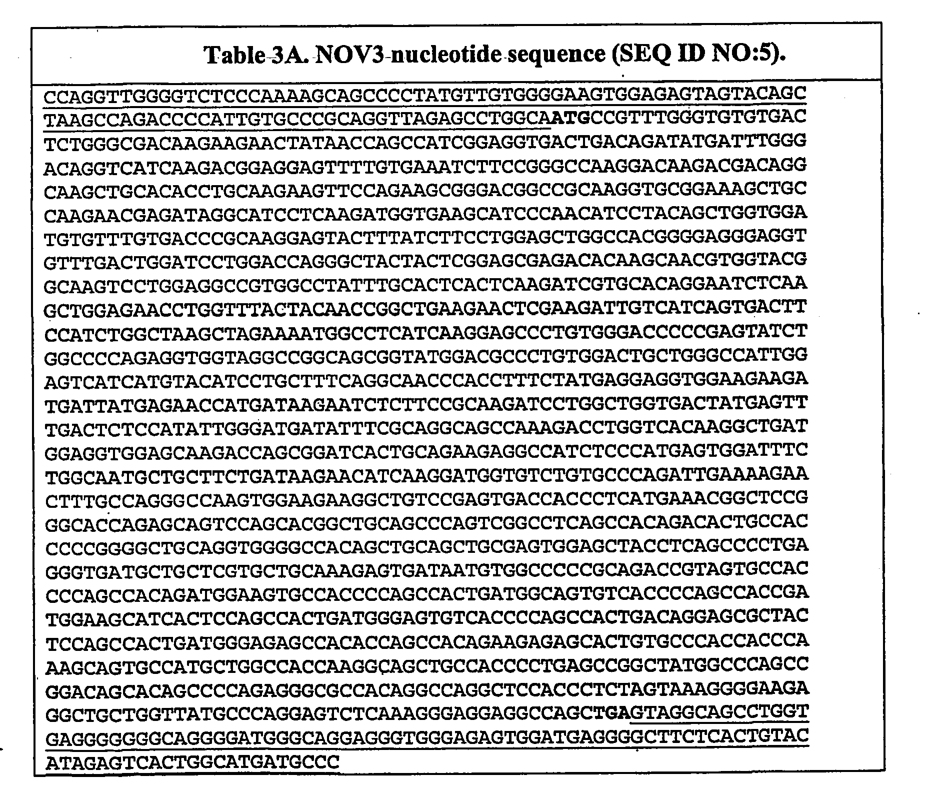

In a search of public sequence databases, the NOV3 nucleic acid sequence, located on chromsome 3 has 1015 of 1158 bases (87%) identical to a gb:GENBANK-ID:RATCBVA|acc:L22557.1 mRNA from Rattus norvegicus (Rattus norvegicus vesicla-associate calmodulin-binding protein mRNA, complete cds). Public nucleotide databases include all GenBank databases and the GeneSed patent database. amino acid residues and is presented in Table 3B using the one-letter amino acid code. Signal P, Psort and/or Hydropathy results predict that NOV3 has no signal peptide and is likely to be localized in the in the cytoplasm with a certainty of 0.4500.

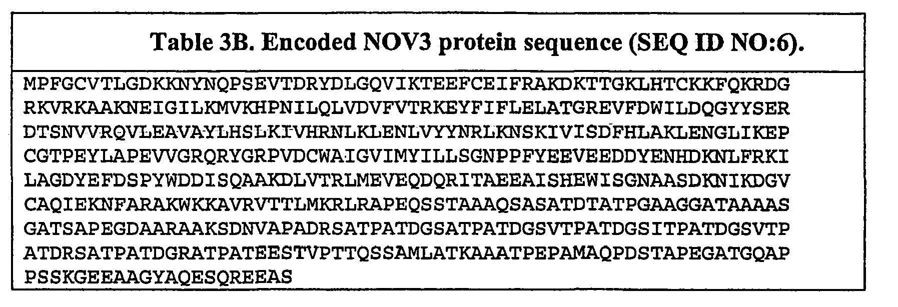

A search of sequence databases reveals that the NOV3 amino acid sequence has 1015 of 1158 amino acid residues (87%) identical to, and 1015 of 1158 amino acid residues (87%) similar to, the 3655 amino acid residue gb:GENBANK-ID:RATCBVA|acc:L22557.1 protein from Rattus norvegicus (Rattus norvegicus vesicla-associate calmodulin-binding protein mRNA, complete cds). Public amino acid databases include the GenBank databases, SwissProt, PDB and PIR.

NOV3 is expressed in at least Bone Marrow, Brain, Hypothalamus, Thalamus. This information was derived by determining the tissue sources of the sequences that were included in the invention including but not limited to SeqCalling sources, Public EST sources, Literature sources, and/or RACE sources.

The disclosed NOV3 polypeptide has homology to the amino acid sequences shown in the BLASTP data listed in Table 3C.

| **Table 3C. BLAST results for NOV3** | | | | | |
|---|---|---|---|---|---|
| Gene Index/ Identifier | Protein/ Organism | Length (aa) | Identity (%) | Positives (%) | Expect |
| gi\|16924331\|gb\|AAH17363.1\|AAH17363 (BC017363) | protein MGC8407 [Homo sapiens] | 501 | 501/501 (100%) | 501/501 (100%) | 0.0 |
| gi\|13129008\|ref\|NP 076951.1\| (NM_024046) | protein MGC8407 [Homo sapiens] | 501 | 500/501 (99%) | 500/501 (99%) | 0.0 |
| gi\|17160946\|gb AAH1 7634.1\|AAH17634 (BC017634 | Similar to vesicle-associated calmodulin-binding protein [Mus musculus] | 512 | 468/512 (91%) | 476/512 (92%) | 0.0 |
| gi\|13027458\|ref\|NP 076490.1\| (NM_024000) | vesicle-associated calmodulin-binding protein [Rattus norvegicus] | 504 | 459/504 (91%) | 465/504 (92%) | 0.0 |
| gi\|13649042\|ref\|XP 017796.1\| (XM 017796) | protein MGC8407 [Homo sapiens] | 478 | 469/471 (99%) | 470/471 (99%) | 0.0 |

Table 3D lists the domain descriptions from DOMAIN analysis results against NOV3. This indicates that the NOV3 sequence has properties similar to those of other proteins known to contain this domain.

| **Table 3E. Domain Analysis of NOV** |
|---|
| gnl\|Smart\|smart00220, S_TKc, Serine/Threonine protein kinases, catalytic domain; Phosphotransferases. Serine or threonine-specific kinase subfamily. |
| CD-Length = 256 residues, 100.0% aligned |
| Score = 271 bits (693), Expect = 7e-74 |

Protein phosphorylation is a fundamental process for the regulation of cellular functions. The coordinated action of both protein kinases and phosphatases controls the levels of phosphorylation and, hence, the activity of specific target proteins. One of the predominant roles of protein phosphorylation is in signal transduction, where extracellular signals are amplified and propagated by a cascade of protein phosphorylation and dephosphorylation events. Eukaryotic protein kinases are enzymes that belong to a very extensive family of proteins which share a conserved catalytic core common with both serine/threonine and tyrosine protein kinases. There are a number of conserved regions in the catalytic domain of protein kinases. In the N-terminal extremity of the catalytic domain there is a glycine-rich stretch of residues in the vicinity of a lysine residue, which has been shown to be involved in ATP binding. In the central part of the catalytic domain there is a conserved aspartic acid residue which is important for the catalytic activity of the enzyme. Protein kinases are excellent small molecule drug targets for therapeutic intervention.

The disclosed NOV3 nucleic acid of the invention encoding a Calmodulin Binding Protein Kinase-like protein includes the nucleic acid whose sequence is provided in Table 3A or a fragment thereof. The invention also includes a mutant or variant nucleic acid any of whose bases may be changed from the corresponding base shown in Table 3A while still encoding a protein that maintains its Calmodulin Binding Protein Kinase-like activities and physiological functions, or a fragment of such a nucleic acid. The invention further includes acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 13 percent of the bases may be so changed.

The disclosed NOV3 protein of the invention includes the Calmodulin Binding Protein Kinase-like protein whose sequence is provided in Table 3B. The invention also includes a mutant or variant protein any of whose residues may be changed from the corresponding residue shown in Table B while still encoding a protein that maintains its Calmodulin Binding Protein Kinase-like activities and physiological functions, or a functional fragment thereof. In the mutant or variant protein, up to about 13 percent of the residues may be so changed.

The invention further encompasses antibodies and antibody fragments, such as F_{ab} or (F_{ab})₂, that bind immunospecifically to any of the proteins of the invention.

The above defined information for this invention suggests that this Calmodulin Binding Protein Kinase-like protein (NOV3) may function as a member of a "Calmodulin Binding Protein Kinase family". Therefore, the NOV3 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV3 nucleic acids and proteins of the invention are useful in potential therapeutic applications implicated in diseases including but not limited to various pathologies and disorders as indicated below. For example, a cDNA encoding the Calmodulin Binding Protein Kinase-like protein (NOV3) may be useful in gene therapy, and the Calmodulin Binding Protein Kinase-like protein (NOV3) may be useful when administered to a subject in need thereof. By way of nonlimiting example, the compositions of the present invention will thrombocytopenic purpura, autoimmune disease, allergies, immunodeficiencies, transplantation, graft versus host disease, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, or other pathologies or conditions. The NOV3 nucleic acid encoding the Calmodulin Binding Protein Kinase-like protein of the invention, or fragments thereof, may further be useful in diagnostic applications, wherein the presence or amount of the nucleic acid or the protein are to be assessed.

NOV3 nucleic acids and polypeptides are further useful in the generation of antibodies that bind immuno-specifically to the novel NOV3 substances for use in therapeutic or diagnostic methods. These antibodies may be generated according to methods known in the art, using prediction from hydrophobicity charts, as described in the "Anti-NOVX Antibodies" section below. The disclosed NOV3 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV4

NOV4 includes two TRANSIENT RECEPTOR POTENTIAL-RELATED PROTEIN-like proteins disclosed below. The disclosed sequences have been named NOV4a and NOV4b.

### NOV4a

A disclosed NOV4a nucleic acid of 4877 nucleotides (also referred to as CG57547-01) encoding a TRANSIENT RECEPTOR POTENTIAL-RELATED PROTEIN-like protein is shown in Table 4A. Putative untranslated regions upstream and/or downstream from the coding region, if any, are underlined, and the start and stop codons are in bold letters.

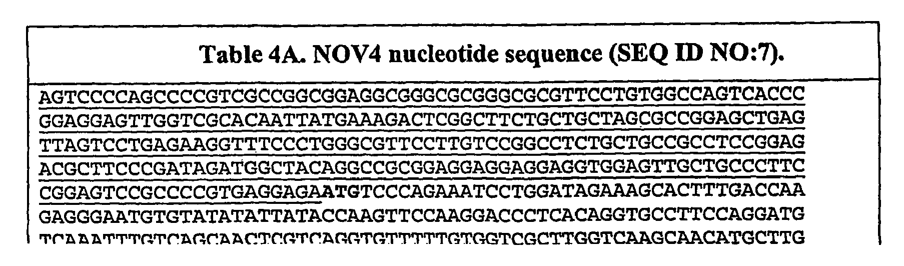

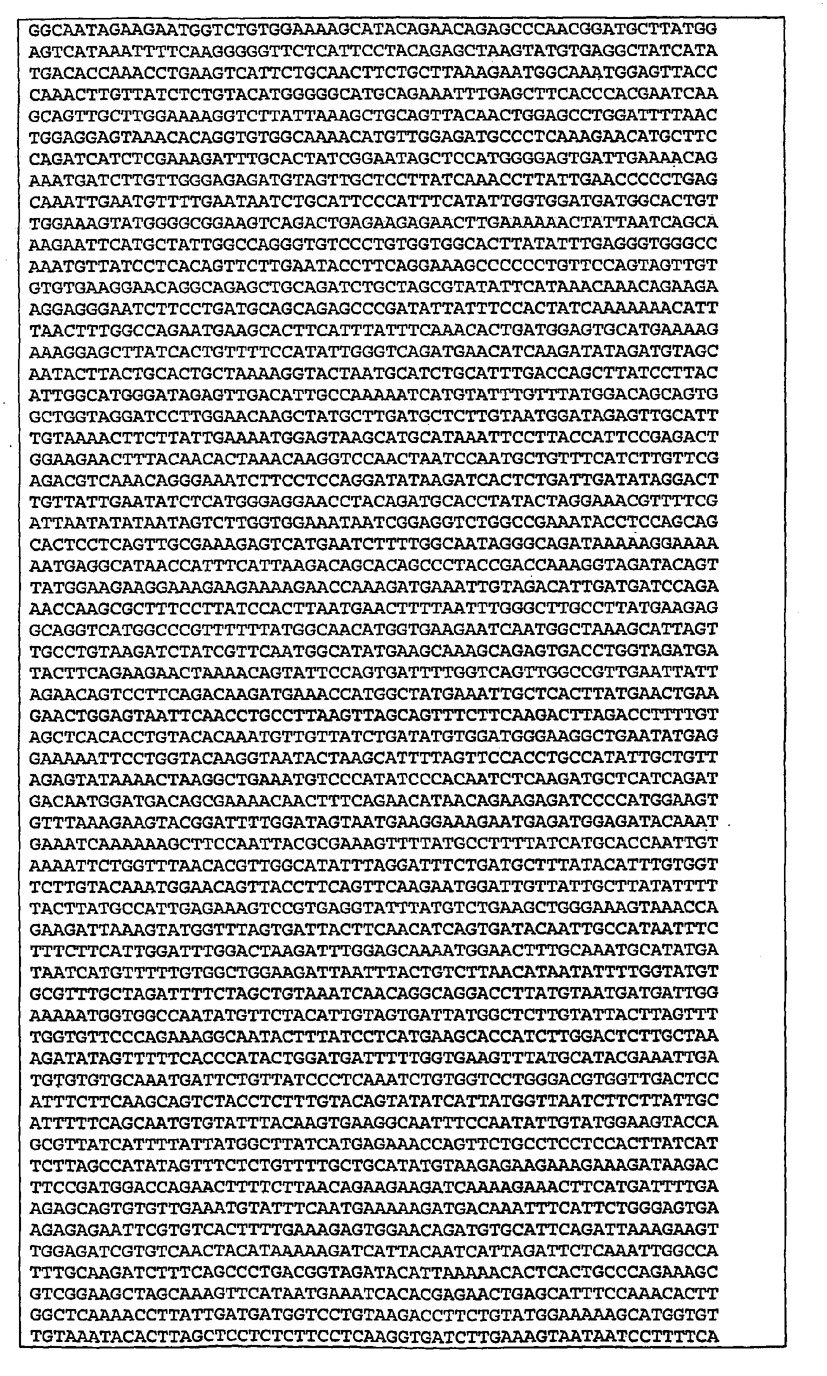

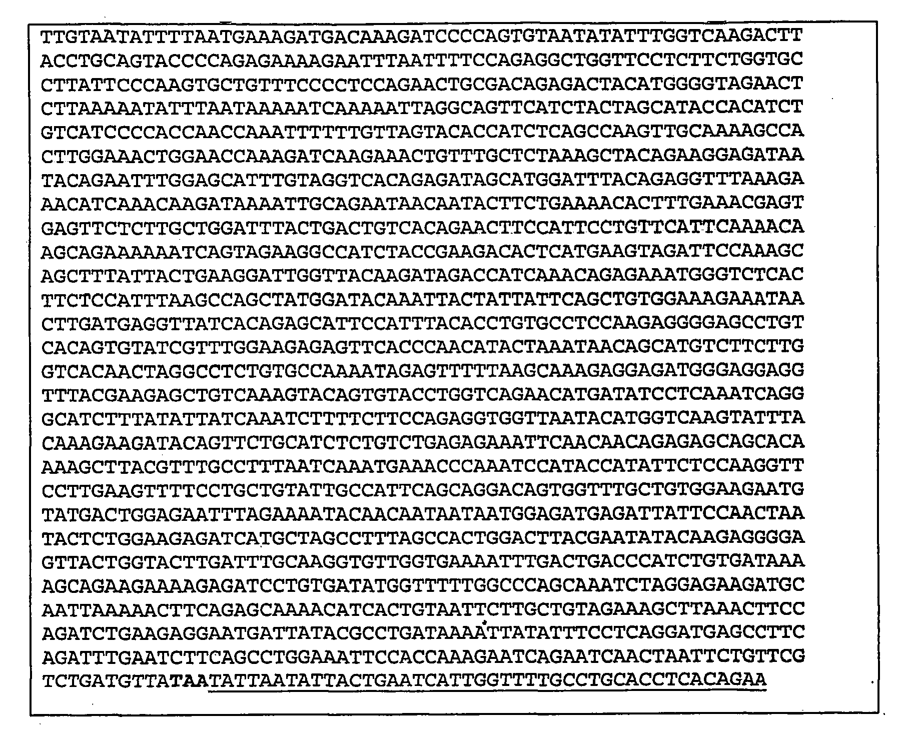

In a search of public sequence databases, the NOV4a nucleic acid sequence, located on chromsome 15 has 4374 of 4825 bases (90%) identical to a gb:GENBANK-ID:AF149013|acc:AF149013.1 mRNA from Mus musculus (Mus musculus transient receptor potential-related protein (ChaK) mRNA, complete cds). Public nucleotide databases include all GenBank databases and the GeneSeq patent database.

The disclosed NOV4a polypeptide (SEQ ID NO:8) encoded by SEQ ID NO:7 has 1856 amino acid residues and is presented in Table 4B using the one-letter amino acid code. Signal P, Psort and/or Hydropathy results predict that NOV4a has no signal peptide and is likely to be localized at the plasma membrane with a certainty of 0.6000.

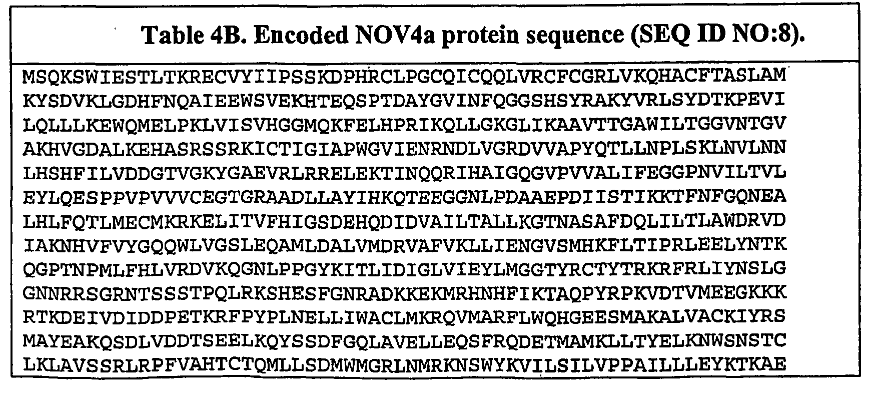

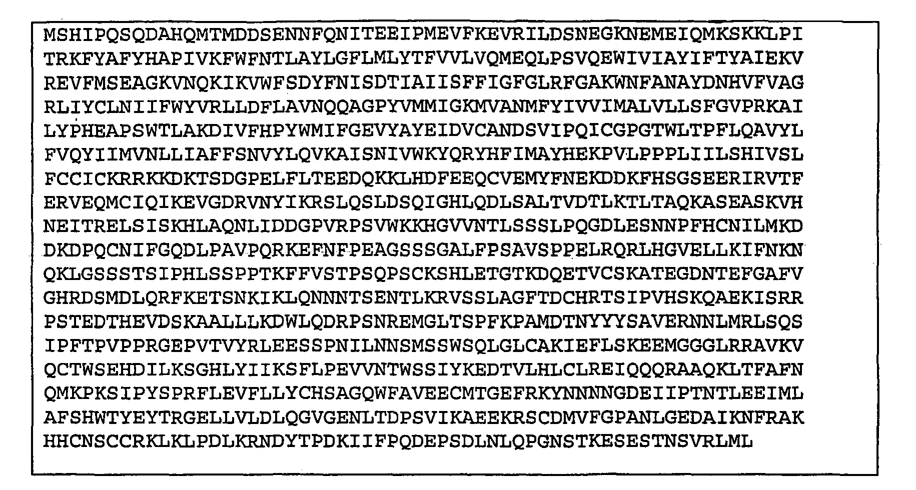

A search of sequence databases reveals that the NOV4a amino acid sequence has 174 of 1863 amino acid residues (93%) identical to, and 1803 of 1863 amino acid residues (96%) similar to the 1863 amino acid residue ptnr:SPTREMBL-ACC:Q9JLQ1 protein from Mus musculus (Mouse) (TRANSIENT RECEPTOR POTENTIAL-RELATED PROTEIN). Publi. amino acid databases include the GenBank databases, SwissProt, PDB and PIR.

NOV4a is expressed in at least Adrenal Gland/Suprarenal gland, Bone Marrow, Brail Bronchus, Cartilage, Colon, Hippocampus, Kidney, Liver, Lymph node, Skeletal Muscle, Stomach, Substantia Nigra, Tonsils and Whole Organism. This information was derived by determining the tissue sources of the sequences that were included in the invention including but not limited to SeqCalling sources, Public EST sources, Literature sources, and/or RACE sources.

### NOV4b

A disclosed NOV4b nucleic acid of 5626 nucleotides (also referred to as CG57547-02) encoding a TRANSIENT RECEPTOR POTENTIAL-RELATED PROTEIN-like protein is shown i Table 4C. Putative untranslated regions upstream and/or downstream from the coding region, if any, are underlined, and the start and stop codons are in bold letters.

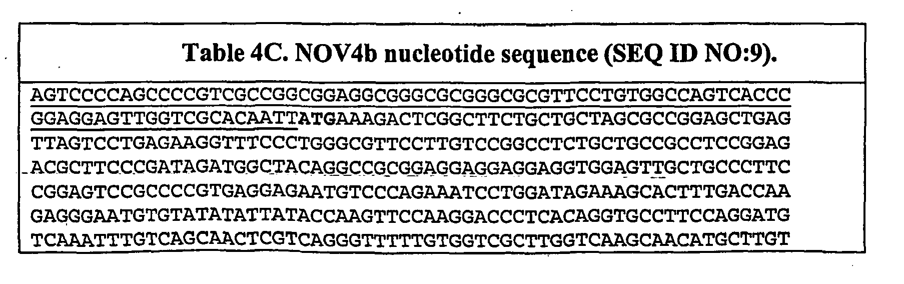

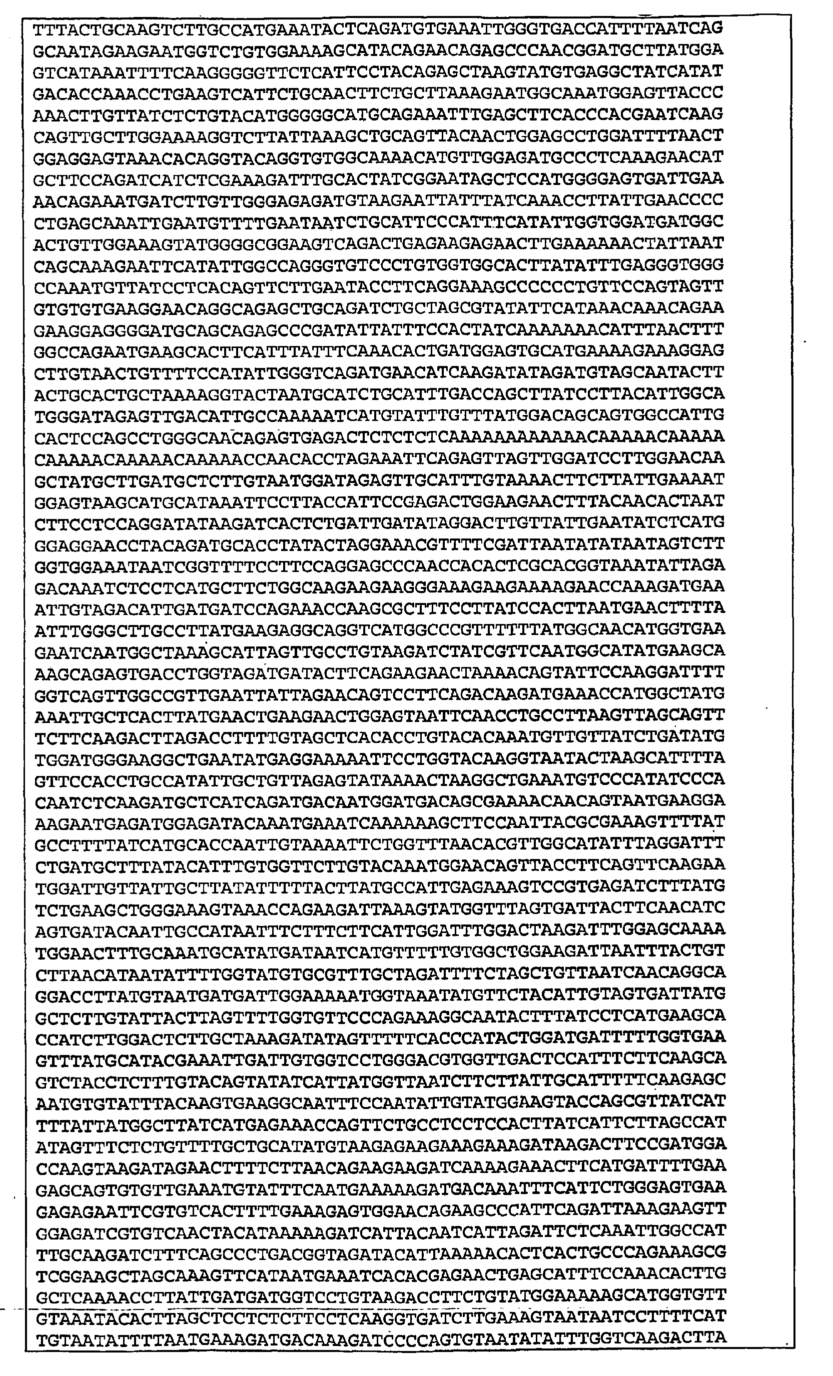

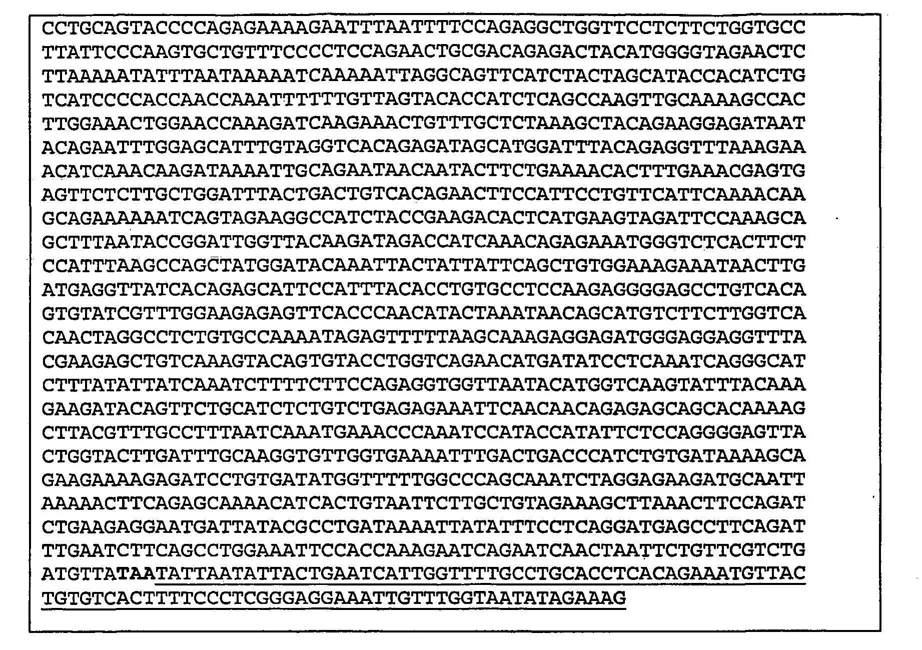

In a search of public sequence databases, the NOV4b nucleic acid sequence, located o chromsome 15 has 1134 of 1246 bases (91%) identical to a gb:GENBANK-ID:AF149013|acc:AF149013.1 mRNA from Mus musculus (Mus musculus transient receptor potential-related protein (ChaK) mRNA, complete cds). Public nucleotide databases include all GenBank databases and the GeneSeq patent database.

The disclosed NOV4b polypeptide (SEQ ID NO:10) encoded by SEQ ID NO:9 has 1815 amino acid residues and is presented in Table 4D using the one-letter amino acid code. Signal P, Psort and/or Hydropathy results predict that NOV4b has no signal peptide and is likely to be localized at the plasma membrane with a certainty of 0.6000.

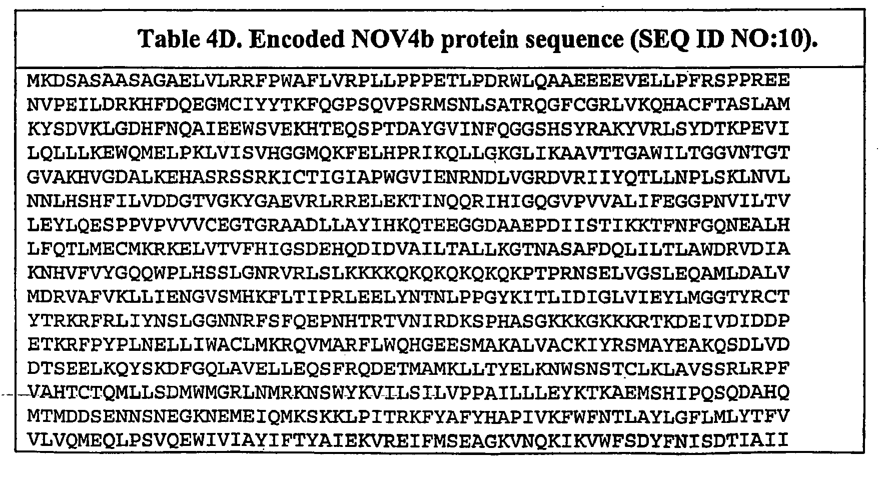

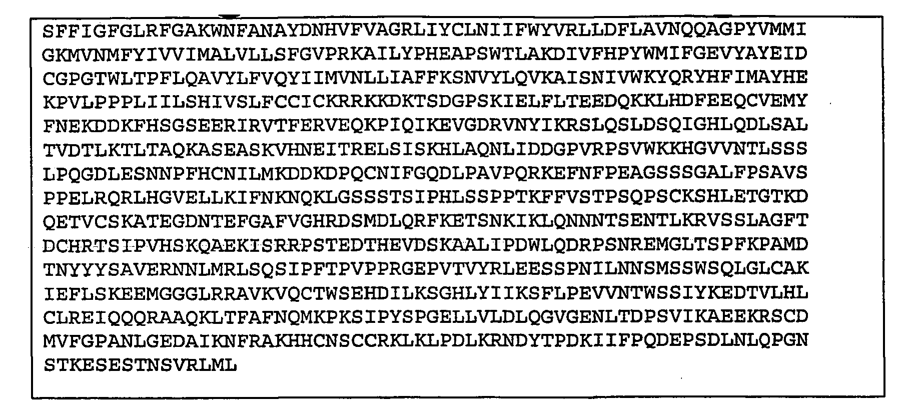

A search of sequence databases reveals that the NOV4b amino acid sequence has 776 of 892 amino acid residues (86%) identical to, and 819 of 892 amino acid residues (91%) similar to, the 1863 amino acid residue ptnr:SPTREMBL-ACC:Q9JLQ1 protein from Mus musculus (Mouse) (TRANSIENT RECEPTOR POTENTIAL-RELATED PROTEIN). Public amino acid databases include the GenBank databases, SwissProt, PDB and PIR.

NOV4b is expressed in at least adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea and uterus. This information was derived by determining the tissue sources of the sequences that were included in the invention including but not limited to SeqCalling sources, Public EST sources, Literature sources, and/or RACE sources.

The disclosed NOV4a polypeptide has homology to the amino acid sequences shown in the BLASTP data listed in Table 4E.

| **Table 4E.BLAST results for NOV4** | | | | | |
|---|---|---|---|---|---|
| Gene Index/ Identifier | Protein/ Organism | Length (aa) | Identity (%) | Positives (%) | Expect |
| gi\|13959785\|gb\|AAK4 4211.1\| (AY032950) | LTRPC7 [Homo sapiens] | 1865 | 1847/1866 (98%) | 1853/1866 (98%) | 0.0 |
| gi\|13562153\|gb\|AAK1 9738.2\|AF346629 1 (AF346629) | channel-kinase 1 [Homo sapiens] | 1864 | 1845/1866 (98%) | 1851/1866 (98%) | 0.0 |
| gi\|14009344\|gb\|AAK5 0377.1\| (AY032951) | LTRPC7 [Mus musculus] | 1863 | 1748/1866 (93%) | 1803/1866 (95%) | 0.0 |
| gi\|109468301ref\|NP 067425.1\| (NM_021450) | transient receptor potential M7; transient receptor potential-related protein, ChaK [Mus musculus] | 1863 | 1747/1866 (93%) | 1803/1866 (96%) | 0.0 |
| gi\|14211383\|gb\|AAK5 7433.1\|AF376052 1 (AF376052) | transient receptor potential phospholipase C interacting kinase [Mus musculus] | 1862 | 1747/1866 (93%) | 1802/1866 (95%) | 0.0 |

Table 4F-G lists the domain descriptions from DOMAIN analysis results against NOV4. This indicates that the NOV4 sequence has properties similar to those of other proteins known to contain this domain.

| **Table 4F. Domain Analysis of NOV4** |
|---|
| gnl\|Pfam\|pfam02816, MHCK_EF2_kinase, MHCK/EF2 kinase domain family. This family is a novel family of eukaryotic protein kinase catalytic domains, which have no detectable similarity to conventional kinases. The family contains myosin heavy chain kinases and Elongation Factor-2 kinase and a bifunctional ion channel. |
| CD-Length = 206 residues, 94.7% aligned |
| Score = 79.7 bits (195), Expect = le-15 |

| **Table 4G. Domain Analysis of NOV4** |
|---|
| gnl\|Pfam\|pfam00520, ion_trans, Ion transport protein. This family contains Sodium, Potassium, Calcium ion channels. This family is 6 transmembrane helices in which the last two helices flank a loop which determines ion selectivity. In some sub-families (e.g. Na channels) the domain is repeated four times, whereas in others (e.g. K channels) the protein forms as a tetramer in the membrane. |
| CD-Length = 191 residues, 99.0% aligned |
| Score = 62.8 bits (151), Expect = 2e-10 |

Capacitative calcium entry (CCE) describes CA2+ influx into cells that replenishes CA2+ stores emptied through the action of IP3 and other agents. It is an essential component of cellular responses to many hormones and growth factors. The molecular basis of this form of Ca2+ entry is complex and may involve more than one type of channel. Studies on visual signal transduction in Drosophila led to the hypothesis that a protein encoded in transient receptor potential (Trp) and related proteins may be a component of CCE channels. Zhu et al.) small portions of these genes in antisense orientation suppressed CCE. Expression in COS cells of two full-length cDNAs encoding human trp homologs, Htrp1 and Htrp3, increased CCE. This identifies mammalian gene products that participate in CCE.

Human TRPC genes encode proteins with sequence similarity to the Drosophila 'transient receptor potential' (trp) gene product. TRPC proteins are thought to be subunits of capacitative calcium entry (CCE) channels, which mediate calcium influx into cells to replenish internal stores of calcium. Using exon trapping on a contig from 21q22.3, Kudoh et al. (1997) isolated an exon whose deduced amino acid sequence shows similarity to the sequences of human TRPC and Drosophila trp proteins. Nagamine et al. (1998) isolated human fetal brain and caudate nucleus cDNAs corresponding to the exon and its parent gene. The deduced 1,503-amino acid protein, which is named TRPC7, is 22.9% identical to human TRPC1 (602343), 21.2% identical to human TRPC3 (602345), and 22.6% identical to Drosophila trp. TRPC7 contains 7 predicted membrane-spanning domains. The TRPC7 gene has 32 exons spanning approximately 90 kb. Northern blot analysis of human tissues detected a 6.5-kb TRPC7 transcript predominantly in fetal and adult brains, where it was expressed in several regions. In caudate nucleus and putamen, a putative 5.5-kb alternatively spliced TRPC7 product also was detected.

The disclosed NOV4 nucleic acid of the invention encoding a TRANSIENT RECEPTOR POTENTIAL-RELATED PROTEIN-like protein includes the nucleic acid whose sequence is provided in Table 4A or 4C or a fragment thereof. The invention also includes a mutant or variant nucleic acid any of whose bases may be changed from the corresponding base shown in Table 4A or 4C while still encoding a protein that maintains its TRANSIENT RECEPTOR POTENTIAL-RELATED PROTEIN-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or changed.

The disclosed NOV4 protein of the invention includes the TRANSIENT RECEPTOR POTENTIAL-RELATED PROTEIN-like protein whose sequence is provided in Table 4B or 4D. The invention also includes a mutant or variant protein any of whose residues may be changed from the corresponding residue shown in Table 4B or 4D while still encoding a protein that maintains its TRANSIENT RECEPTOR POTENTIAL-RELATED PROTEIN-like activities and physiological functions, or a functional fragment thereof. In the mutant or variant protein, up to about 7 percent of the residues may be so changed.

The invention further encompasses antibodies and antibody fragments, such as F_{ab} or (F_{ab})₂, that bind immunospecifically to any of the proteins of the invention.

The above defined information for this invention suggests that this TRANSIENT RECEPTOR POTENTIAL-RELATED PROTEIN-like protein (NOV4) may function as a member of a "TRANSIENT RECEPTOR POTENTIAL-RELATED PROTEIN family". Therefore, the NOV4 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV4 nucleic acids and proteins of the invention are useful in potential therapeutic applications implicated in diseases including but not limited to various pathologies and disorders as indicated below. For example, a cDNA encoding the TRANSIENT RECEPTOR POTENTIAL-RELATED PROTEIN-like protein (NOV4) may be useful in gene therapy, and the TRANSIENT RECEPTOR POTENTIAL-RELATED PROTEIN-like protein (NOV4) may be useful when administered to a subject in need thereof. By way of nonlimiting example, the compositions of the present invention will have efficacy for treatment of patients suffering from adrenoleukodystrophy, congenital adrenal hyperplasia, hemophilia, hypercoagulation, idiopathic thrombocytopenic purpura, autoimmune disease, allergies, immunodeficiencies, transplantation, graft versus host disease, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, glomerulonephritis, polycystic kidney disease, systemic lupus erythematosus, renal tubular acidosis, IgA nephropathy, cirrhosis, lymphedema, ulcers, tonsillitis, or other pathologies or conditions. The NOV4 nucleic acid encoding the TRANSIENT RECEPTOR POTENTIAL-RELATED PROTEIN-like protein of the invention, or fragments thereof, may further be useful in diagnostic applications, wherein the presence or amount of the nucleic acid or the protein are to be assessed.

NOV4 nucleic acids and polypeptides are further useful in the generation of antibodies that bind immuno-specifically to the novel NOV4 substances for use in therapeutic or diagnostic methods. These antibodies may be generated according to methods known in the art, using prediction from hydrophobicity charts, as described in the "Anti-NOVX Antibodies" section below. The disclosed NOV4 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV5

A disclosed NOV5 nucleic acid of 1869 nucleotides (also referred to as CG57609-01) encoding a Epsin-3-like protein is shown in Table 5A. Putative untranslated regions upstream and/or downstream from the coding region, if any, are underlined, and the start and stop codons are in bold letters.

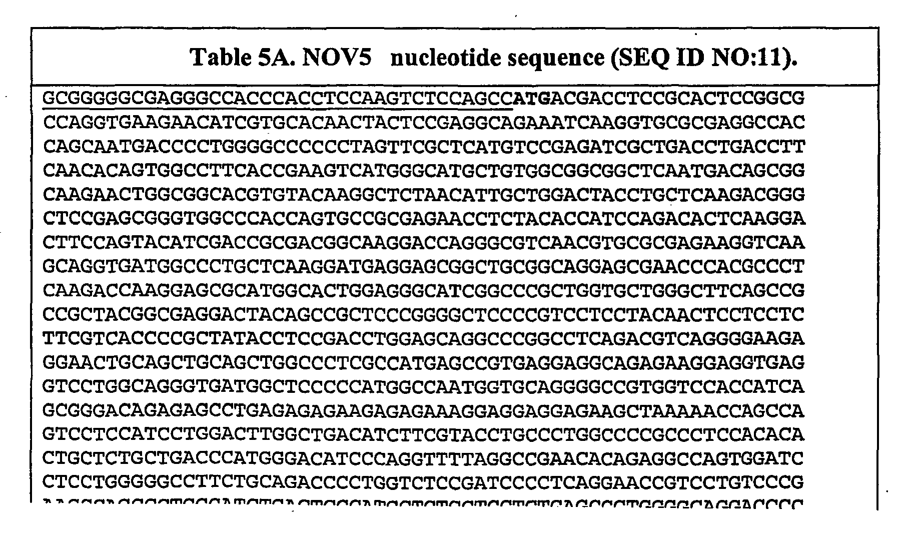

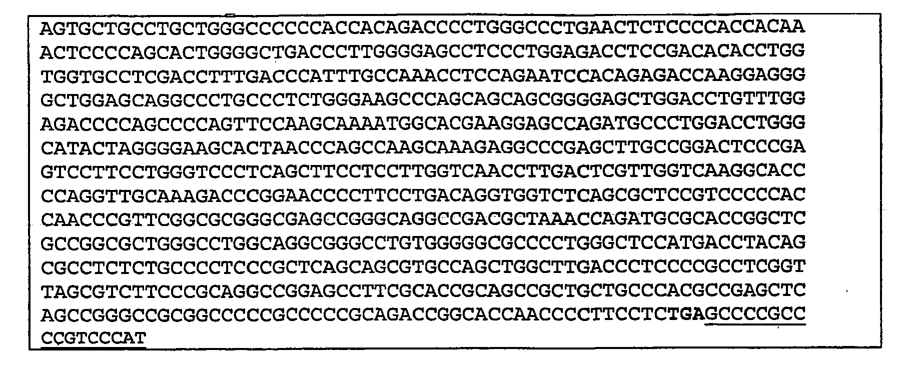

In a search of public sequence databases, the NOV5 nucleic acid sequence, located on chromsome 17 has 1210 of 1234 bases (98%) identical to a gb:GENBANK-ID:AK000785|acc:AK000785.1 mRNA from Homo sapiens (Homo sapiens cDNA FLJ20778 fis, clone COL05704). Public nucleotide databases include all GenBank databases and the GeneSeq patent database.

The disclosed NOV5 polypeptide (SEQ ID N0:12) encoded by SEQ ID NO:11 has 604 amino acid residues and is presented in Table 5B using the one-letter amino acid code. Signal P, Psort and/or Hydropathy results predict that NOV5 has no signal peptide and is likely to be localized in the cytoplasm with a certainty of 0.4500.

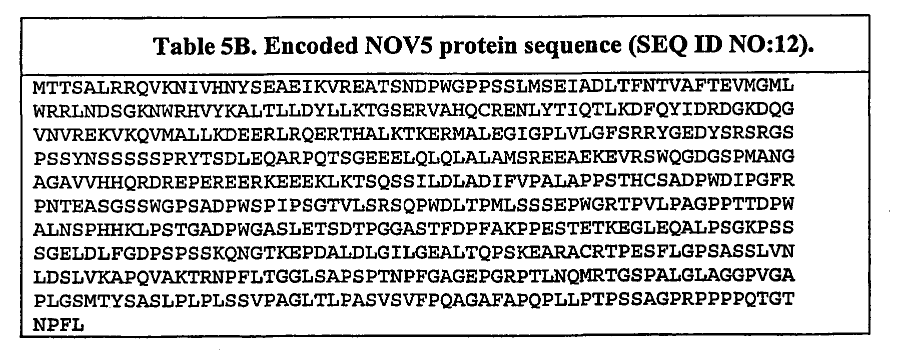

A search of sequence databases reveals that the NOV5 amino acid sequence has 398 of 441 amino acid residues (90%) identical to, and 406 of 441 amino acid residues (92%) similar to, the 632 amino acid residue ptnr:TREMBLNEW-ACC:AAG45223 protein from Homo sapiens (Human) (EPSIN 3). Public amino acid databases include the GenBank databases, SwissProt, PDB and PIR.
tissue sources of the sequences that were included in the invention including but not limited to SeqCalling sources, Public EST sources, Literature sources, and/or RACE sources.

The disclosed NOV5 polypeptide has homology to the amino acid sequences shown in the BLASTP data listed in Table 5C.

| **Table 5C. BLAST results for NOV5** | | | | | |
|---|---|---|---|---|---|
| Gene Index/ Identifier | Protein/ Organism | Length (aa) | Identity (%) | Positives (%) | Expect |
| ref\|XP 037971.1\| (XM_037971) | epsin 3 [Homo sapiens] | 632 | 598/633 (94%) | 598/633 (94%) | 0.0 |
| ref\|NP 060427.1\| (NM_017957 | epsin 3 [Homo sapiens] | 632 | 596/633 (94%) | 597/633 (94%) | 0.0 |
| gb\|AAH16454.1\|AAH16 454 (BC016454) | (protein for MGC:25634) [Mus musculus] | 636 | 518/646 (80%) | 535/646 (82%) | 0.0 |
| dbj\|BAB26309.1\| (AK009469) | homolog to EPSIN 3-putative [Mus musculus] | 569 | 452/579 (78%) | 468/579 (80%) | 0.0 |
| gb\|AAH01038.1\|AAH01 038 (BC001038) | Similar to epsin 3 [Homo sapiens] | 208 | 186/205 (90%) | 188/205 (90%) | e-101 |

Tables 5D-E list the domain descriptions from DOMAIN analysis results against NOV5. This indicates that the NOV sequence has properties similar to those of other proteins known to contain this domain.

| **Table 5E. Domain Analysis of NOV5** |
|---|
| gnl\|Pfam\|pfam01417, ENTH, ENTH domain. The ENTH (Epsin N-terminal homology) domain is found in proteins involved in endocytosis and cytoskeletal machinery. |
| CD-Length = 123 residues, 100.0% aligned |
| Score = 174 bits (442), Expect = 1e-44 |

| **Table 5F. Domain Analysis of NOV5** | | |
|---|---|---|
| gnl\|smart\|smart00288, VHS, Domain present in VPS-27, Hrs and STAM; Unpublished observations | | |
| | CD-Length = 133 residues, 88.7% aligned | |
| | | Score = 38.5 bits (88), Expect = 0.001 |

The mammalian protein epsin is required for endocytosis. There are two homologous for the highly conserved N-terminal epsin N-terminal homology (ENTH) domain was revealed using deletions and randomly generated temperature-sensitive ent1 alleles. Changes in conserved ENTH domain residues in entl(ts) cells revealed defects in endocytosis and actin cytoskeleton structure. The Ent1 protein was localized to peripheral and internal punctate structures, and biochemical fractionation studies found the protein associated with a large, Triton X-100-insoluble pellet. Finally, an Entlp clathrin-binding domain was mapped to the final eight amino acids (RGYTLIDL*) in the Ent1 protein sequence. Based on these and other data, yeast epsin-like proteins are essential components of an endocytic complex that may act at multiple stages in the endocytic pathway.

An approximately 140 amino acid domain is shared by a variety of proteins in budding and fission yeast, nematode, rat, mouse, frog, oat, and man. Typically, this domain is located within 20 residues of the N-terminus of the various proteins. The percent identity among the domains in the 12 proteins ranges from 42 to 93%, with 16 absolutely conserved residues. Even though these proteins share little beyond their segment of homology, data are emerging that several of the proteins are involved in endocytosis and or regulation of cytoskeletal organization. This protein segment is the ENTH domain, for Epsin N-terminal Homology domain.

The disclosed NOV5 nucleic acid of the invention encoding a Epsin-3-like protein includes the nucleic acid whose sequence is provided in Table 5A or a fragment thereof. The invention also includes a mutant or variant nucleic acid any of whose bases may be changed from the corresponding base shown in Table 5A while still encoding a protein that maintains its Epsin-3-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 2 percent of the bases may be so changed.

The disclosed NOV5 protein of the invention includes the Epsin-3-like protein whose sequence is provided in Table 5B. The invention also includes a mutant or variant protein any still encoding a protein that maintains its Epsin-3-like activities and physiological functions, or a functional fragment thereof. In the mutant or variant protein, up to about 10 percent of the residues may be so changed.

The invention further encompasses antibodies and antibody fragments, such as F_{ab} or (F_{ab})₂, that bind immunospecifically to any of the proteins of the invention.

The above defined information for this invention suggests that this Epsin-3-like protein (NOV5) may function as a member of a "Epsin-3 family". Therefore, the NOV5 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV5 nucleic acids and proteins of the invention are useful in potential therapeutic applications implicated in diseases including but not limited to various pathologies and disorders as indicated below. For example, a cDNA encoding the Epsin-3-like protein (NOV5) may be useful in gene therapy, and the Epsin-3-like protein (NOV5) may be useful when administered to a subject in need thereof. By way of nonlimiting example, the compositions of the present invention will have efficacy for treatment of patients suffering from psoriasis, actinic keratosis, tuberous sclerosis, acne, hair growth/loss, allopecia, pigmentation disorders, endocrine disorders, or other pathologies or conditions. The NOV5 nucleic acid encoding the Epsin-3-like protein of the invention, or fragments thereof, may further be useful in diagnostic applications, wherein the presence or amount of the nucleic acid or the protein are to be assessed.

NOV5 nucleic acids and polypeptides are further useful in the generation of antibodies that bind immuno-specifically to the novel NOV5 substances for use in therapeutic or diagnostic methods. These antibodies may be generated according to methods known in the art, using prediction from hydrophobicity charts, as described in the "Anti-NOVX Antibodies" section below. The disclosed NOV5 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV6

A disclosed NOV6 nucleic acid of 2646 nucleotides (also referred to as CG57611-01) encoding a CD22-like protein is shown in Table 6A. Putative untranslated regions upstream and/or downstream from the coding region, if any, are underlined, and the start and stop codons are in bold letters.

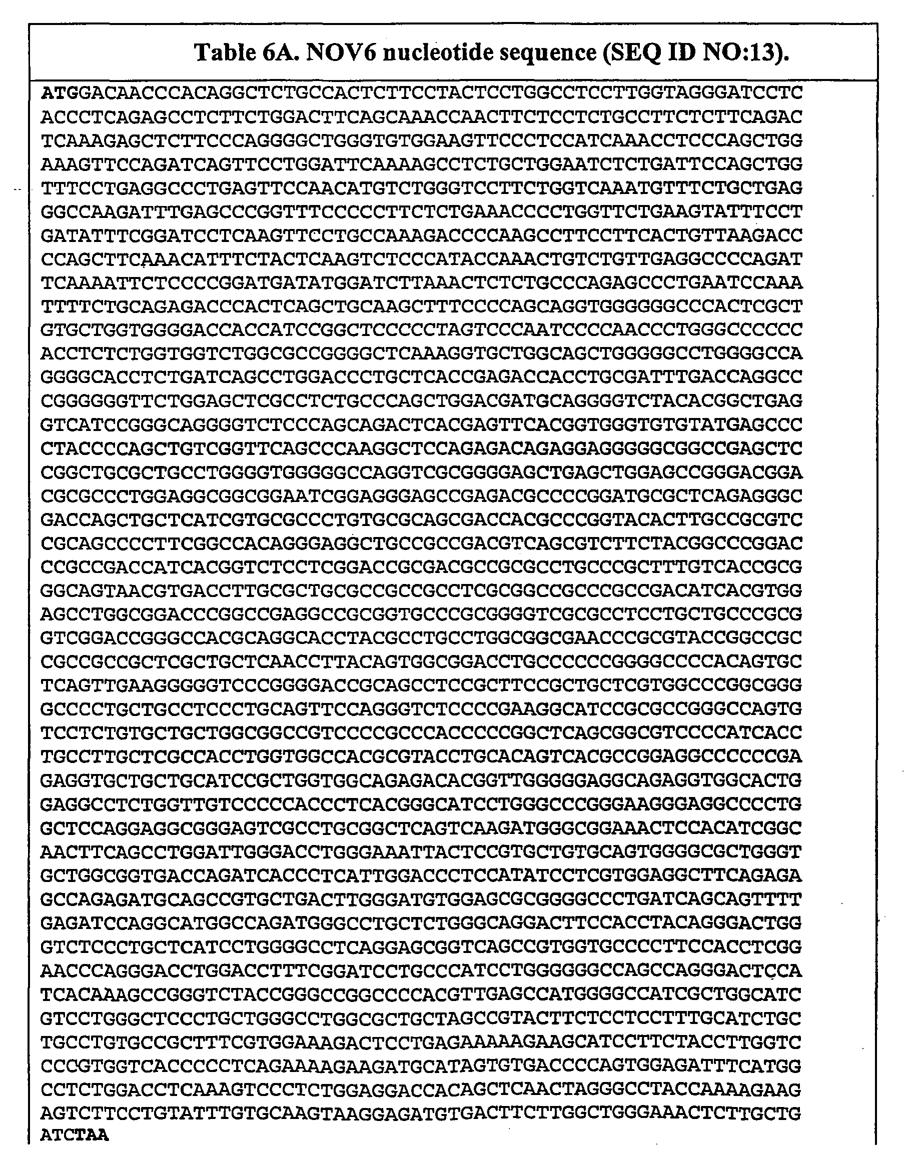

chromsome 19 has 941 of 1572 bases (59%) identical to a gb:GENBANK-ID:AF246990|acc:AF246990.1 mRNA from Chlamydomonas reinhardtii (Chlamydomonas reinhardtii flagellar autotomy protein Fa1p (FA1) mRNA, complete cds). Public nucleotide databases include all GenBank databases and the GeneSeq patent database.

The disclosed NOV6 polypeptide (SEQ ID N0:14) encoded by SEQ ID NO: 13 has 881 amino acid residues and is presented in Table 6B using the one-letter amino acid code. Signal P, Psort and/or Hydropathy results predict that NOV6 has a signal peptide and is likely to be localized at the plasma membrane with a certainty of 0.4600. The signal peptide is predicted by SignalP to be cleaved at amino acid 27-28.

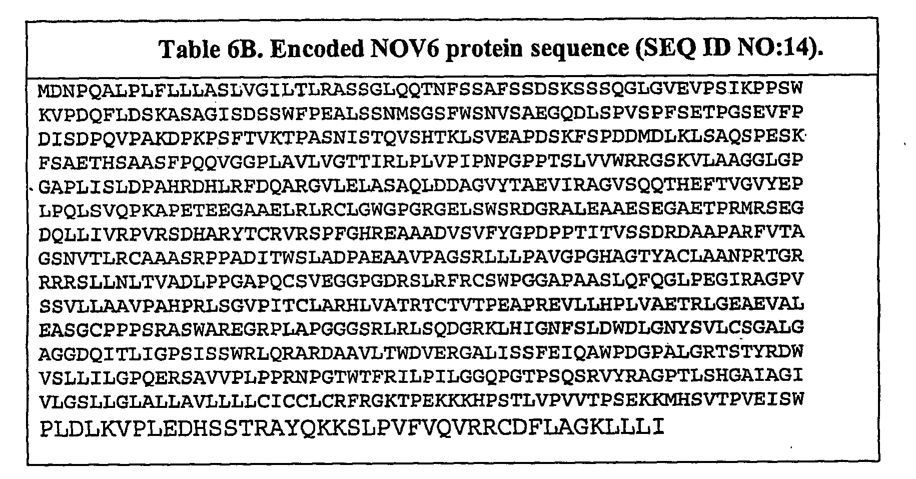

A search of sequence databases reveals that the NOV6 amino acid sequence has 76 of 254 amino acid residues (29%) identical to, and 117 of 254 amino acid residues (46%) similar to, the 521 amino acid residue ptnr:SPTREMBL-ACC:Q61352 protein from Mus musculus (Mouse) (BILIARY GLYCOPROTEIN 1 PRECURSOR). Public amino acid databases include the GenBank databases, SwissProt, PDB and PIR.

NOV6 is expressed in at least lung, ovary, squamous cell carcinoma, and fibrotheoma. This information was derived by determining the tissue sources of the sequences that were included in the invention including but not limited to SeqCalling sources, Public EST sources, Literature sources, and/or RACE sources.

The disclosed NOV6 polypeptide has homology to the amino acid sequences shown in the BLASTP data listed in Table 6C.

| **Table 6C. BLAST results for NOV6** | | | | | |
|---|---|---|---|---|---|
| Gene Index/ Identifier | Protein/ Organism | Length (aa) | Identity (%) | Positives (%) | Expect |
| gi\|312584\|emb\|CAA47 697.1\| (X67280) | biliary glycoprotein [Mus musculus] | 458 | 68/237 (28%) | 109/237 (45%) | 1e-16 |
| gi\|14029256\|gb\|AAK5 2602.1\| (AF287912) | CEA-related cell adhesion molecule 2 [Mus musculus] | 520 | 68/237 (28%) | 109/237 (45%) | 3e-16 |
| gi\|423398\|pir\|\|S343 38 | biliary glycoprotein F - mouse | 521 | 68/237 (28%) | 109/237 (45%) | 4e-16 |
| gi\|483309\|pir\|\|JC15 09 | biliary glycoprotein E - mouse | 458 | 68/237 (28%) | 108/237 (44%) | 5e-16 |
| gi\|l09630\|pir\|\|S116 26 | carcinoembryonic antigen - mouse (fragment) | 379 | 66/237 (27%) | 106/237 (43%) | 1e-15 |

Table 6D lists the domain descriptions from DOMAIN analysis results against NOV6. This indicates that the NOV6 sequence has properties similar to those of other proteins known to contain this domain.

| **Table 6D. Domain Analysis of NOV6** | | |
|---|---|---|
| gnl\|Smart\|smart00408, IGc2, Immunoglobulin C-2 Type | | |
| | CD-Length = 63 residues, 93.7% aligned | |
| | | Score = 55.5 bits (132), Expect = 1e-08 |

The disclosed NOV6 novel gene described here contains three immunoglobulin domains and has homology to mouse CD22, a B lymphocyte-restricted adhesion molecule, mouse colon biliary glycoprotein, and carcinoembryonic antigen. The immunoglobulin domain is found as a tandem repeat in Streptococcal cell surface proteins, such as the IgG binding proteins G and MIG. These proteins are type I membrane proteins that bind to the constant Fc region of IgG with high affinity. The N-terminus of MIG mediates binding to plasma proteinase inhibitor alpha 2-macroglobulin after complex formation with proteases.

The human B lymphocyte-specific Ag, CD22, is a cell adhesion moleculé expressed on the surface during a narrow window of B cell development, coincident with surface IgD. A ligand for CD22 has recently been identified on human T cells as the low molecular mass isoform of the leukocyte common Ag, CD45RO. CD22 has been reported to function in the regulation of both T and B cell activation in vitro.

Carcinoembryonic antigen (CEA) is a widely used tumor marker, especially in the surveillance of colonic cancer patients. Although CEA is also present in some normal tissues, tissues. Carcinoembryonic antigen (CEA) expression is perhaps the most prevalent of phenotypic changes observed in human cancer cells. Twenty-seven CEA cDNA clones were isolated from a human colon adenocarcinoma cell line. Most of these clones are full length and consist of a number (usually three) of surprisingly similar long (534 base pairs) repeats between a 5' end of 520 base pairs and a 3' end with three different termination points. The predicted translation product of these clones consists of a processed signal sequence of 34 amino acids, an amino-terminal sequence of 107 amino acids, which includes the known terminal amino acid sequence of CEA, three repeated domains of 178 amino acids each, and a membrane-anchoring domain of 27 amino acids, giving a total of 702 amino acids and a molecular weight of 72,813 for the mature protein. The repeated domains have conserved features, including the first 67 amino acids at their N termini and the presence of four cysteine residues. Comparisons with the amino acid sequences of other proteins reveals homology of the repeats with various members of the immunoglobulin supergene family, particularly the human T-cell receptor gamma chain. CEA cDNA clones in the SP-65 vector were shown to produce transcripts in vitro which could be translated in vitro to yield a protein of molecular weight 73,000 which in turn could be precipitated with CEA-specific antibodies (See Schrewe H et al., Mol Cell Biol 1990 Jun;10(6):2738-48.).

The biliary glycoprotein (BGP)-encoding gene is a member of the human carcinoembryonic antigen (CEA) gene family. McCuaig et al. cloned several mouse Bgp cDNAs from an outbred CDR-1 mouse colon cDNA library, as well as by reverse transcription-PCR amplification of colon RNA. The distinguishing features of the deduced Bgp protein isoforms are found in the two divergent N-terminal domains, the highly conserved internal C2-set immunoglobulin domains, and an intracytoplasmic domain of either 10 or 73 amino acids (aa). The cDNA structures suggest that these mRNAs are produced through alternative splicing of a Bgp gene and the usage of multiple transcriptional terminators. The Bgp deduced aa sequences are highly homologous to several well characterized rat hepatocyte proteins such as the cell CAM105/ecto-ATPase/pp120/FiA4 proteins. Oligodeoxyribonucleotide probes representing the various cDNA isoform domains revealed predominant transcripts of 1.8, 3.1 and 4.0 kb on Northern analyses of mouse colon RNA; some of these bands are actually composed of several co-migrating transcripts. The transcripts encoding the long intracytoplasmic-tailed Bgp proteins are expressed at one-tenth the relative abundance of the shorter-tailed species. The expression of the many Bgp isoforms at the surface of epithelial cells, such as colon, suggests that these proteins play a determinant role, McCuaig et al., Gene 1993 May 30;127(2):173-83).

The disclosed NOV6 nucleic acid of the invention encoding a CD22-like protein includes the nucleic acid whose sequence is provided in Table 6A or a fragment thereof. The invention also includes a mutant or variant nucleic acid any of whose bases may be changed from the corresponding base shown in Table 6A while still encoding a protein that maintains its CD22-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 41 percent of the bases may be so changed.

The disclosed NOV6 protein of the invention includes the CD22-like protein whose sequence is provided in Table 6B. The invention also includes a mutant or variant protein any of whose residues may be changed from the corresponding residue shown in Table 6B while still encoding a protein that maintains its CD22-like activities and physiological functions, or a functional fragment thereof. In the mutant or variant protein, up to about 71 percent of the residues may be so changed.

The invention further encompasses antibodies and antibody fragments, such as F_{ab} or (F_{ab})₂, that bind immunospecifically to any of the proteins of the invention.

The above defined information for this invention suggests that this CD22-like protein (NOV6) may function as a member of a "CD22 family". Therefore, the NOV6 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here. therapeutic applications implicated in diseases including but not limited to various pathologies and disorders as indicated below. For example, a cDNA encoding the CD22-like protein (NOV6) may be useful in gene therapy, and the CD22-like protein (NOV6) may be useful when administered to a subject in need thereof. By way of nonlimiting example, the compositions of the present invention will have efficacy for treatment of patients suffering from endometriosis, fertility, systemic lupus erythematosus, autoimmune disease, asthma, emphysema, scleroderma, allergy, ARDS, or other pathologies or conditions. The NOV6 nucleic acid encoding the CD22-like protein of the invention, or fragments thereof, may further be useful in diagnostic applications, wherein the presence or amount of the nucleic acid or the protein are to be assessed.

NOV6 nucleic acids and polypeptides are further useful in the generation of antibodies that bind immuno-specifically to the novel NOV6 substances for use in therapeutic or diagnostic methods. These antibodies may be generated according to methods known in the art, using prediction from hydrophobicity charts, as described in the "Anti-NOVX Antibodies" section below. The disclosed NOV6 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV7

A disclosed NOV7 nucleic acid of 8589 nucleotides (also referred to as CG57595-01) encoding a MEGF8-like protein is shown in Table 7A. Putative untranslated regions upstream and/or downstream from the coding region, if any, are underlined, and the start and stop codons are in bold letters.

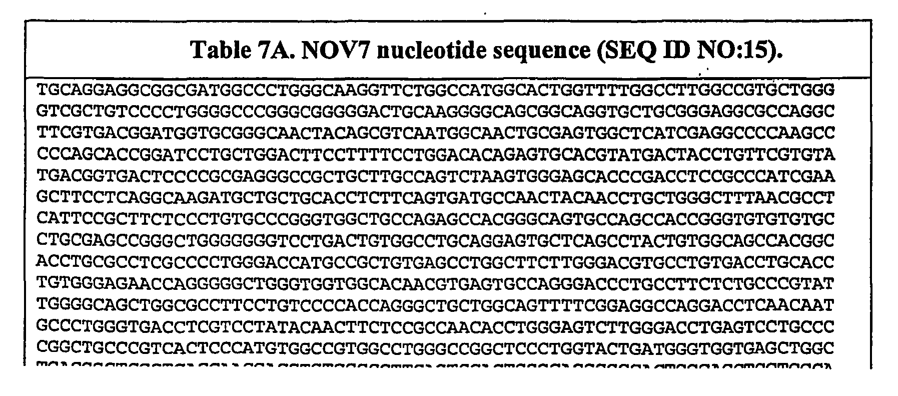

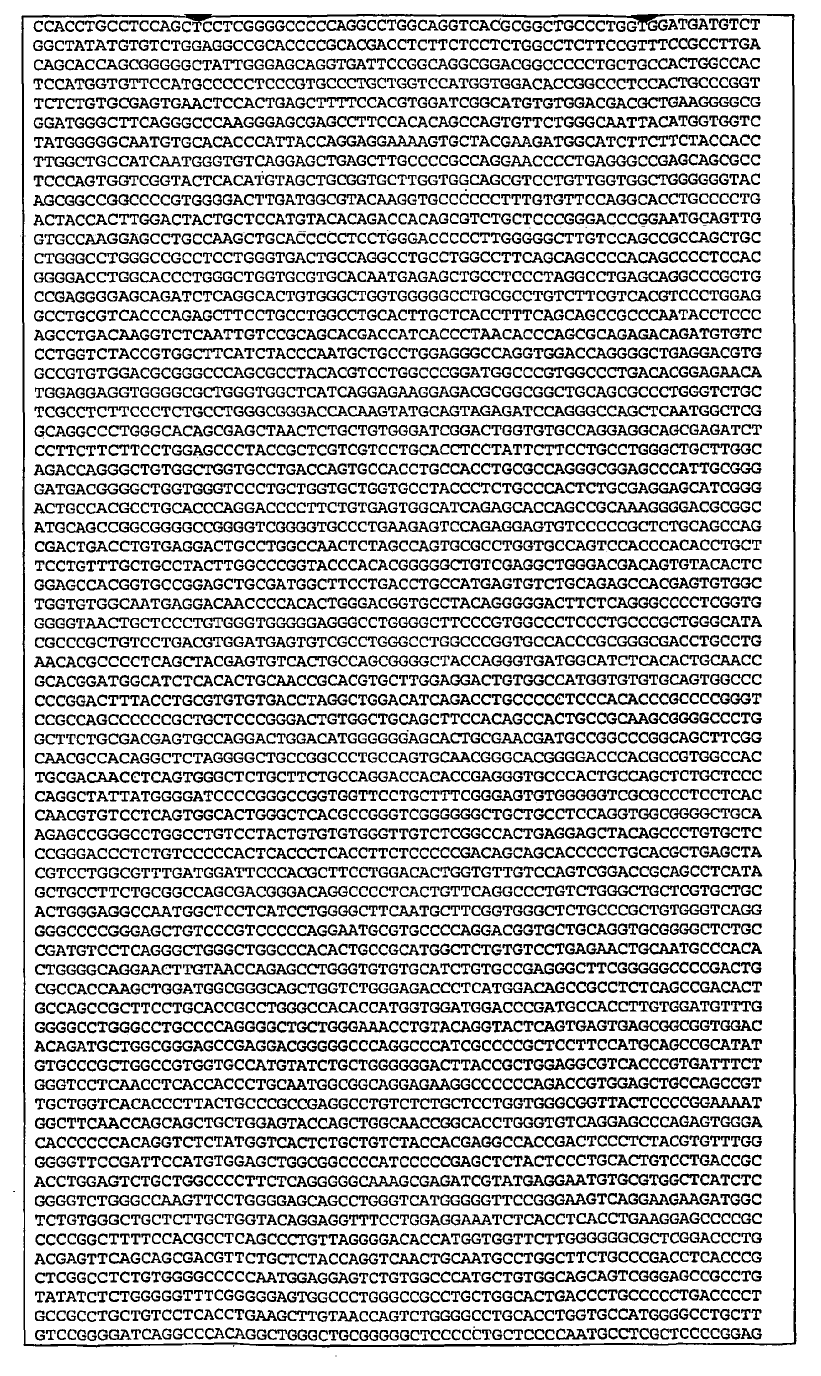

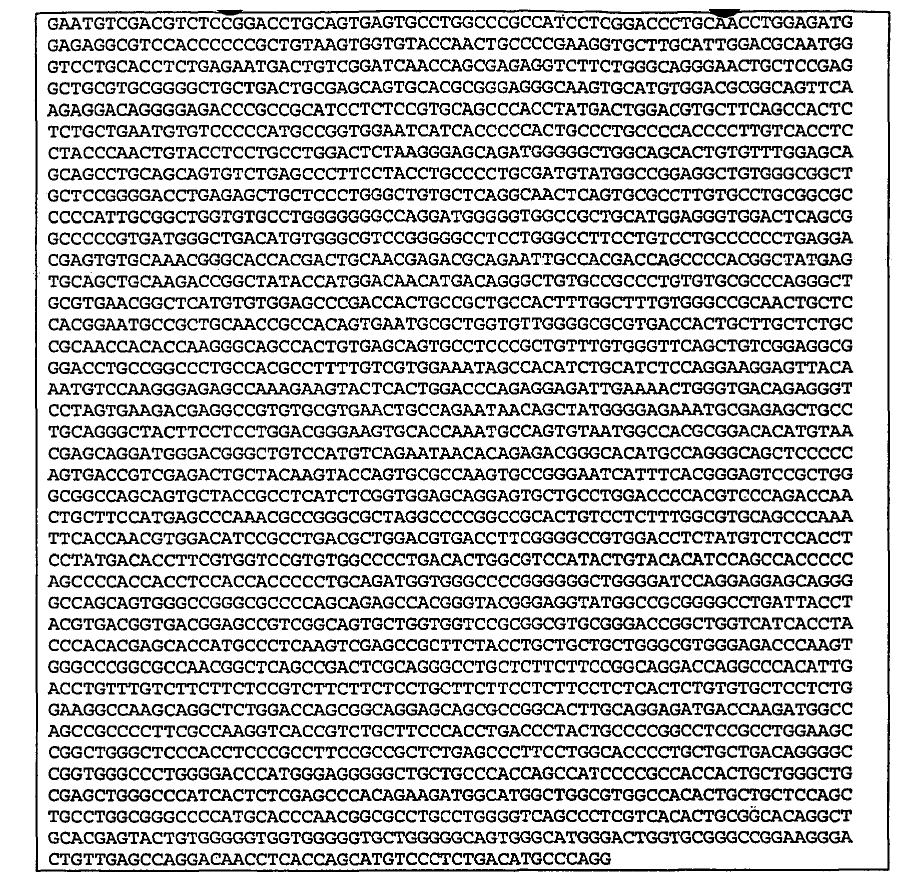

In a search of public sequence databases, the NOV7 nucleic acid sequence, located on chromsome 19 has 5224 of 5224 bases (100%) identical to a gb:GENBANK-ID:AB011541|acc:AB011541.1 mRNA from Homo sapiens (Homo sapiens mRNA for MEGF8, partial cds). Public nucleotide databases include all GenBank databases and the GeneSeq patent database.

The disclosed NOV7 polypeptide (SEQ ID NO: 16) encoded by SEQ ID NO:15 has 2854 amino acid residues and is presented in Table 7B using the one-letter amino acid code. Signal P, Psort and/or Hydropathy results predict that NOV7 has a signal peptide and is likely to be localized at the plasma membrane with a certainty of 0.4600. The most likely cleavage site for a NOV7 peptide is between amino acids 27 and 28.

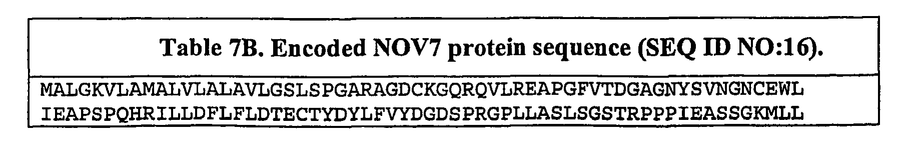

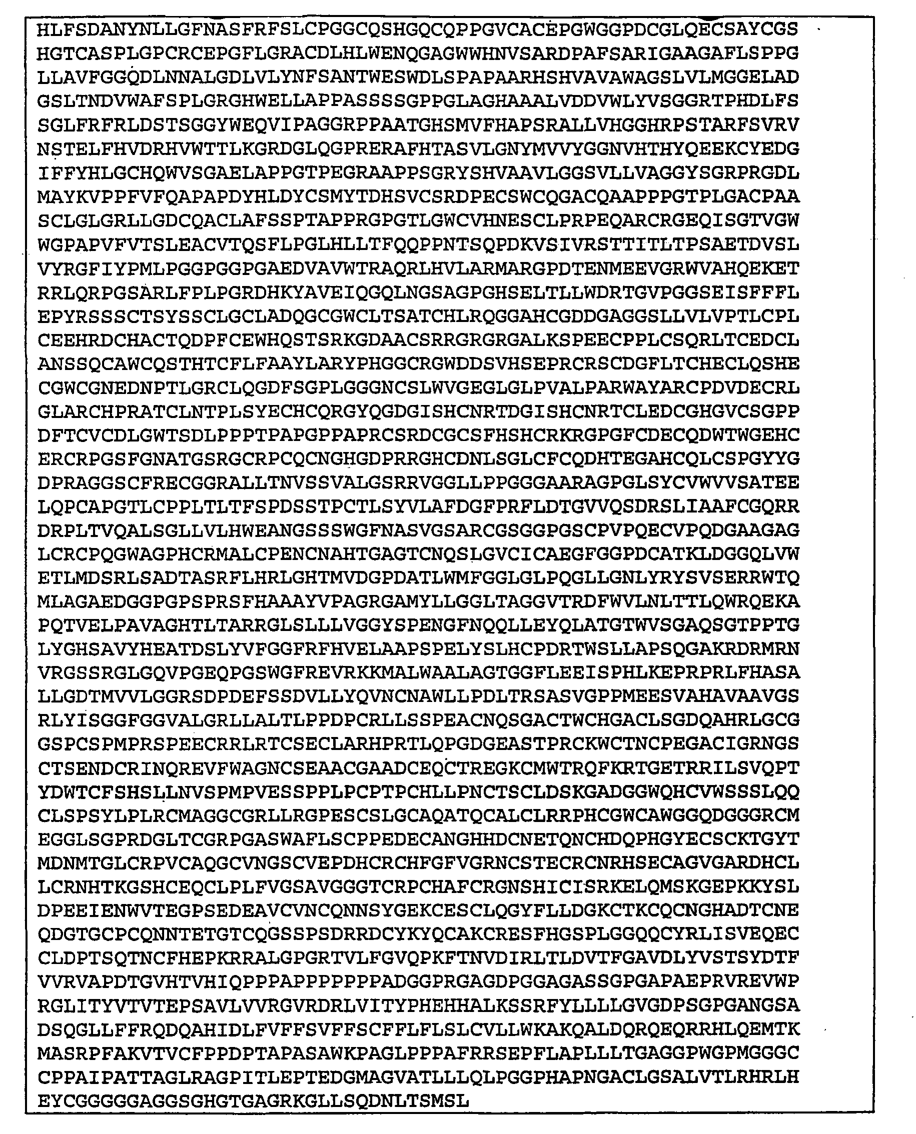

A search of sequence databases reveals that the NOV7 amino acid sequence has 1737 of 1737 amino acid residues (100%) identical to, and 1737 of 1737 amino acid residues (100%) similar to, the 1737 amino acid residue ptnr:SPTREMBL-ACC:O75097 protein from Homo sapiens (Human) (MEGF8). Public amino acid databases include the GenBank databases, SwissProt, PDB and PIR.

NOV7 is expressed in at least kidney, nervous system, brain, lung. This information was derived by determining the tissue sources of the sequences that were included in the sources, and/or RACE sources.

The disclosed NOV7 polypeptide has homology to the amino acid sequences shown in the BLASTP data listed in Table 7C.

| **Table 7C. BLAST results for NOV7** | | | | | |
|---|---|---|---|---|---|
| Gene Index/ Identifier | Protein/ Organism | Length (aa) | Identity (%) | Positives (%) | Expect |
| gi\|7513144\|pir\|\|T00 209 | MEGF8 protein - human (fragment) | 1737 | 1737/1737 (100%) | 1737/1737 (100%) | 0.0 |
| gi\|14756108\|ref\|XP 029883.1\| (XM_029883) | EGF-like-domain, multiple 4 [Homo sapiens] | 1214 | 1171/1173 (99%) | 1171/1173 (99%) | 0.0 |
| gi\|6681364\|dbj\|BAA8 8689.1\| (AB011534 | MEGF8 [Rattus norvegicus] | 874 | 836/874 (95%) | 850/874 (96%) | 0.0 |
| gi\|10728654\|gb\|AAF5 2597.2\| (AE003619) | CG7466 gene product [Drosophila melanogaster] | 2820 | 786/2379 (33%) | 1107/2379 (46%) | 0.0 |
| gi\|17862106\|gb\|AAL3 9530.1\| (AY069385) | LD09511p [Drosophila melanogaster] | 779 | 192/466 (41%) | 263/466 (56%) | 3e-94 |

Tables 7D-F list the domain descriptions from DOMAIN analysis results against NOV7. This indicates that the NOV7 sequence has properties similar to those of other proteins known to contain this domain.

| **Table 7D. Domain Analysis of NOV7** | | |
|---|---|---|
| gnl\|Smart\|smart00042, CUB, Domain first found in C1r, C1s, uEGF, and bone morphogenetic protein.; This domain is found mostly among developmentally-regulated proteins. Spermadhesins contain only this domain. | | |
| | CD-Length = 114 residues, 82.5% aligned | |
| | | Score = 84.0 bits (206), Expect = 1e-16 |

| **Table 7E. Domain Analysis of NOV7** | | |
|---|---|---|
| gnl\|Pfam\|pfam00053, laminin_EGF, Laminin EGF-like (Domains III and V). This family is like pfam00008 but has 8 conserved cysteines instead of 6. | | |
| | CD-Length = 49 residues, 87.8% aligned | |
| | | Score = 47.4 bits (111), Expect = 1e-05 |

| **Table 7F. Domain Analysis of NOV7** |
|---|
| gnl\|Smart\|smart00179, EGF_CA, Calcium-binding EGF-like domain |
| CD-Length = 41 residues, 90.2% aligned |
| Score = 38.5 bits (88), Expect = 0.005 |

The domain that characterizes epidermal growth factor (EGF) consists of approximately 50 amino acids, and has been shown to be present in a more or less conserved form in a large number of other, mostly animal proteins. EGF-like domains are believed to play a critical role in a number of extracellular events, including cell adhesion and receptor-ligand interactions. Proteins with EGF-like domains often consist of more than 1,000 amino acids, have multiple copies of the EGF-like domain, and contain additional domains known to be involved in specific protein-protein interactions. The list of proteins currently known to contain one or more copies of an EGF-like pattern is large and varied. The functional significance of EGF domains in what appear to be unrelated proteins is not yet clear. However, a common feature is that these repeats are found in the extracellular domain of membrane-bound proteins or in proteins known to be secreted (exception: prostaglandin G/H synthase). The EGF domain includes six cysteine residues which have been shown (in EGF) to be involved in 3 disulfide bonds. The main structure is a two-stranded beta-sheet followed by a loop to a C-terminal short two-stranded sheet. Subdomains between the conserved cysteines vary in length.

To identify proteins containing EGF-like domains, Nakayama et al. (1998) searched a database of long cDNA sequences randomly selected from a human brain cDNA library for those that encode an EGF-like motif. They identified several partial cDNAs encoding novel proteins with multiple EGF-like domains, such as EGFL4, which they named MEGF8. The predicted partial EGFL4 protein has a laminin-type EGF-like domain, 5 EGF-like domains, and a transmembrane domain. Using a radiation hybrid mapping panel, Nakayama et al. (1998) mapped the EGFL4 gene to 19q12.
includes the nucleic acid whose sequence is provided in Table 7A or a fragment thereof. The invention also includes a mutant or variant nucleic acid any of whose bases may be changed from the corresponding base shown in Table 7A while still encoding a protein that maintains its MEGF8-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 0 percent of the bases may be so changed.

The disclosed NOV7 protein of the invention includes the MEGF8-like protein whose sequence is provided in Table 7B. The invention also includes a mutant or variant protein any of whose residues may be changed from the corresponding residue shown in Table 7B while still encoding a protein that maintains its MEGF8-like activities and physiological functions, or a functional fragment thereof. In the mutant or variant protein, up to about 0 percent of the residues may be so changed.

The invention further encompasses antibodies and antibody fragments, such as F_{ab} or (F_{ab})₂, that bind immunospecifically to any of the proteins of the invention.

The above defined information for this invention suggests that this MEGF8-like protein (NOV7) may function as a member of a "MEGF8 family". Therefore, the NOV7 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV7 nucleic acids and proteins of the invention are useful in potential therapeutic applications implicated in diseases including but not limited to various pathologies (NOV7) may be useful in gene therapy, and the MEGF8-like protein (NOV7) may be useful when administered to a subject in need thereof. By way of nonlimiting example, the compositions of the present invention will have efficacy for treatment of patients suffering from Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia telangiectasia, leukodystrophies, behavioral disorders, addiction, anxiety, pain, neuroprotection, multiple sclerosis, myasthenia gravis, systemic lupus erythematosus, autoimmune disease, asthma, emphysema, scleroderma, allergy, ARDS, diabetes, renal artery stenosis, interstitial nephritis, glomerulonephritis, polycystic kidney disease, renal tubular acidosis, IgA nephropathy, or other pathologies or conditions. The NOV7 nucleic acid encoding the MEGF8-like protein of the invention, or fragments thereof, may further be useful in diagnostic applications, wherein the presence or amount of the nucleic acid or the protein are to be assessed.

NOV7 nucleic acids and polypeptides are further useful in the generation of antibodies that bind immuno-specifically to the novel NOV7 substances for use in therapeutic or diagnostic methods. These antibodies may be generated according to methods known in the art, using prediction from hydrophobicity charts, as described in the "Anti-NOVX Antibodies" section below. The disclosed NOV7 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV8

NOV8 includes two protocadherin-like proteins disclosed below. The disclosed sequences have been named NOV8a and NOV8b.

### NOV8a

A disclosed NOV8a nucleic acid of 6006 nucleotides (also referred to as CG57542-0 1) encoding a protocadherin-like protein is shown in Table 8A. Putative untranslated regions upstream and/or downstream from the coding region, if any, are underlined, and the start and stop codons are in bold letters.

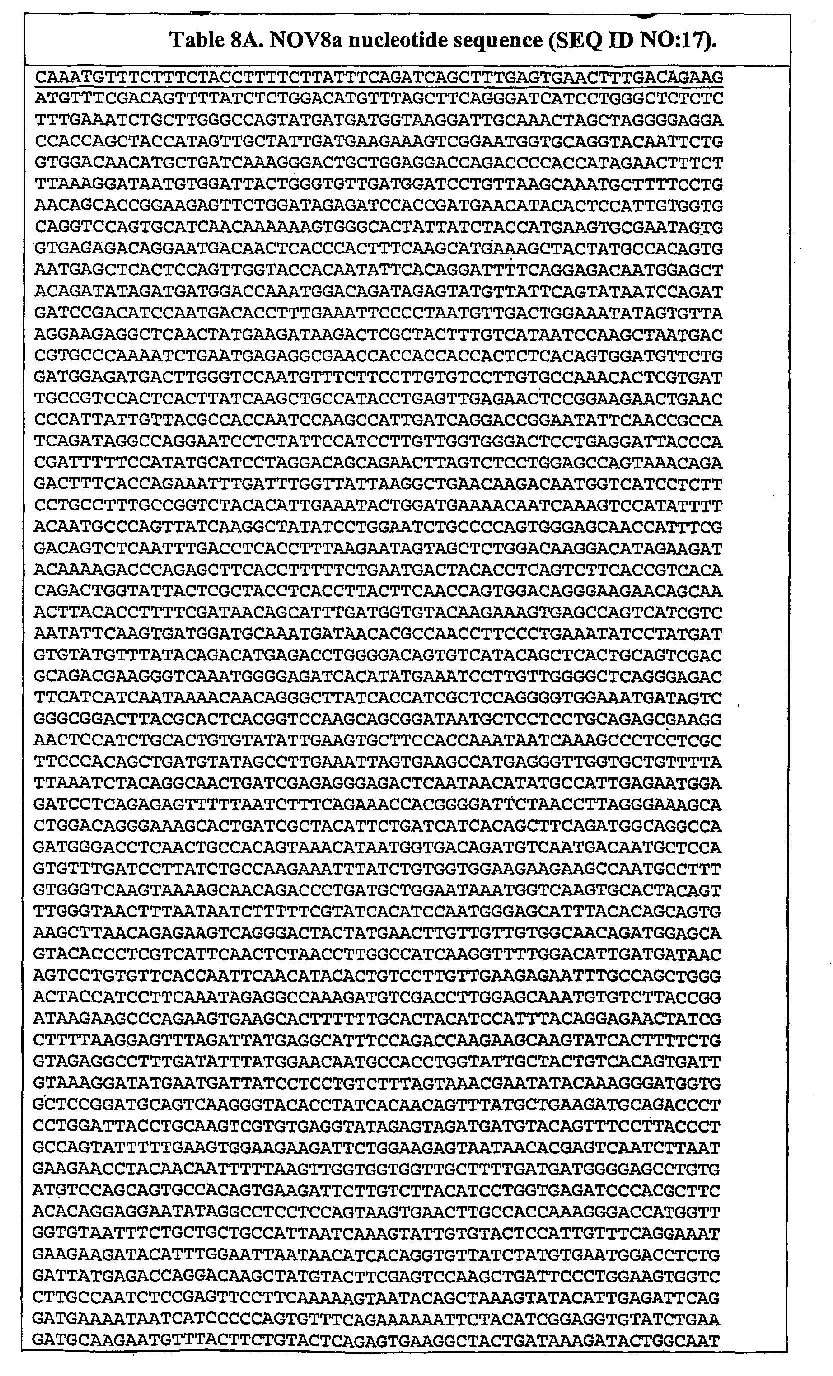

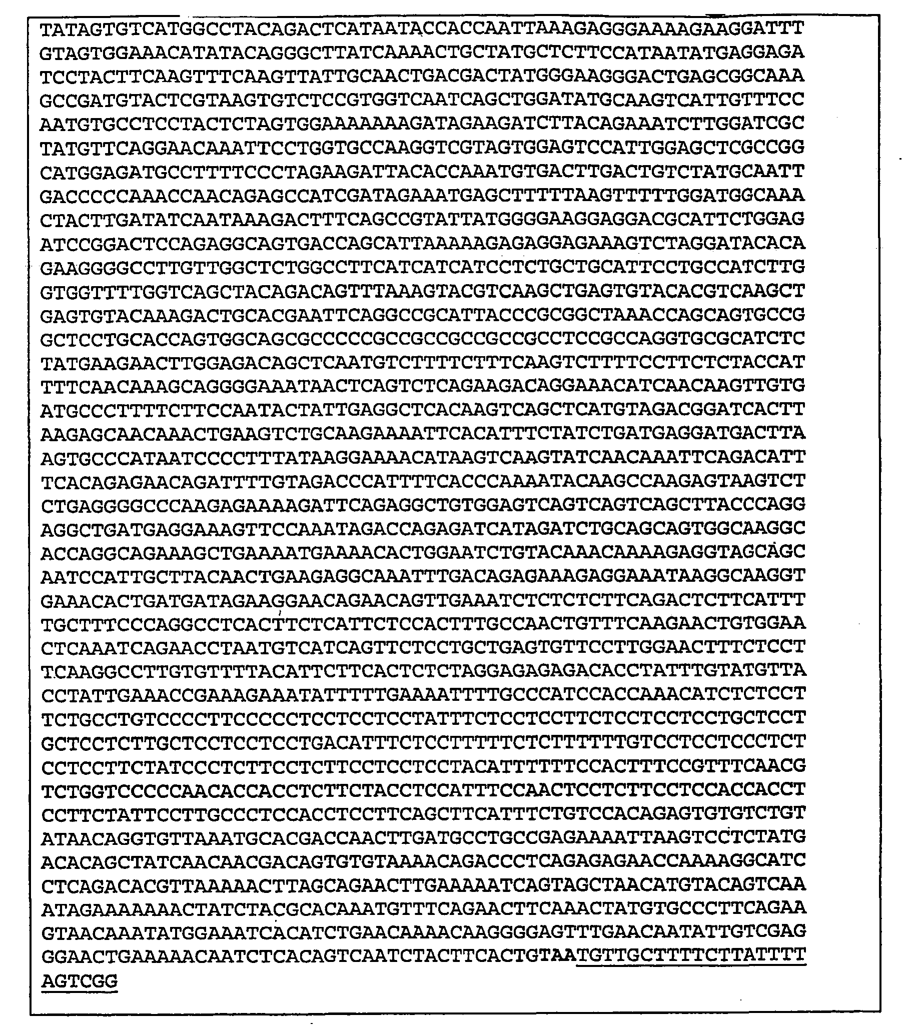

In a search of public sequence databases, the NOV8a nucleic acid sequence, located on chromsome 10 has 557 of 955 bases (58%) identical to a GENBANK-ID:AF169693|acc:AF169693.1 mRNA from Homo sapiens (Homo sapiens protocadherin 13 (PCDH13) mRNA, partial cds). Public nucleotide databases include all GenBank databases and the GeneSeq patent database.

The disclosed NOV8a polypeptide (SEQ ID N0:18) encoded by SEQ ID N0:17 has 1973 amino acid residues and is presented in Table 8B using the one-letter amino acid code. Signal P, Psort and/or Hydropathy results predict that NOV8 has a signal peptide and is likely to be localized extracellularly with a certainty of 0.6760. The most likely cleavage point is between residues 26 and 27.

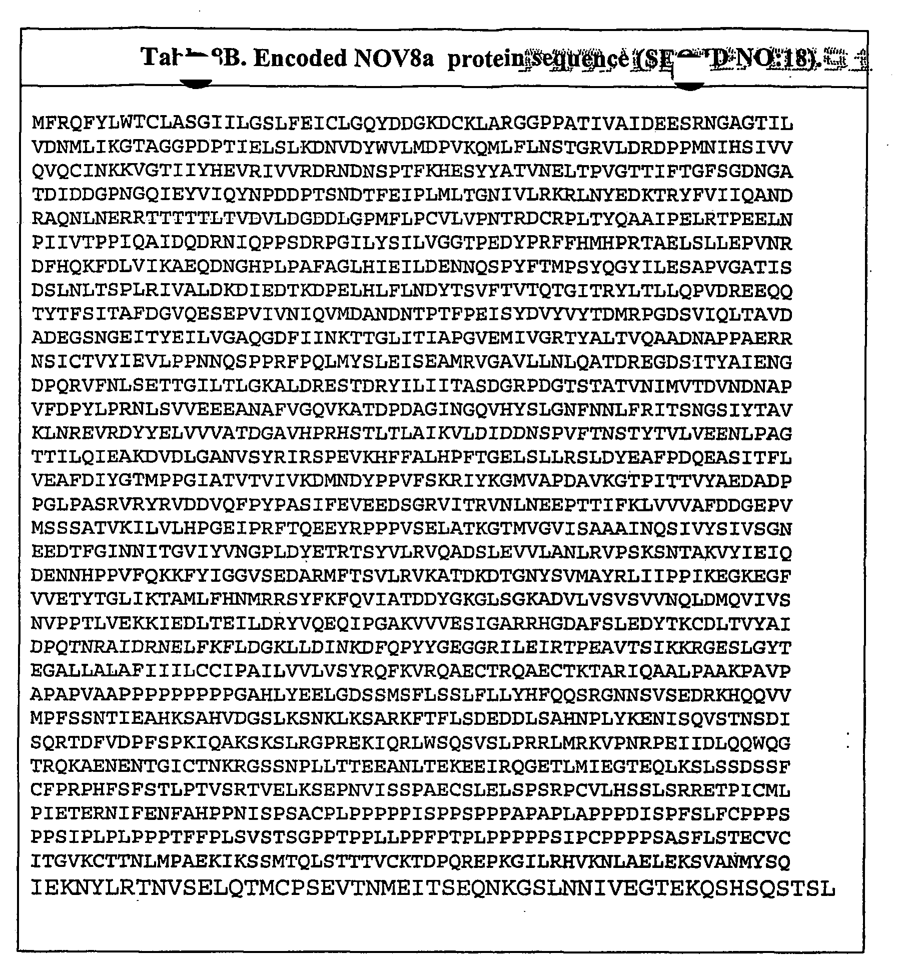

A search of sequence databases reveals that the NOV8a amino acid sequence has 1580 of 1846 amino acid residues (85%) identical to, and 1682 of 1846 amino acid residues (91%) similar to, the 1943 amino acid residue ptnr:TREMBLNEW-ACC:AAG53891 protein from Mus musculus (Mouse) (PROTOCADHERIN). Public amino acid databases include the GenBank databases, SwissProt, PDB and PIR.

NOV8a is expressed in at least brain, lymphoid tissue, placenta. This information was derived by determining the tissue sources of the sequences that were included in the invention including but not limited to SeqCalling sources, Public EST sources, Literature sources, and/or RACE sources.

A disclosed NOV8b nucleic acid of 6003 nucleotides (also referred to as CG57452-02) encoding a protocadherin-like protein is shown in Table 8C. Putative untranslated regions upstream and/or downstream from the coding region, if any, are underlined, and the start and stop codons are in bold letters.

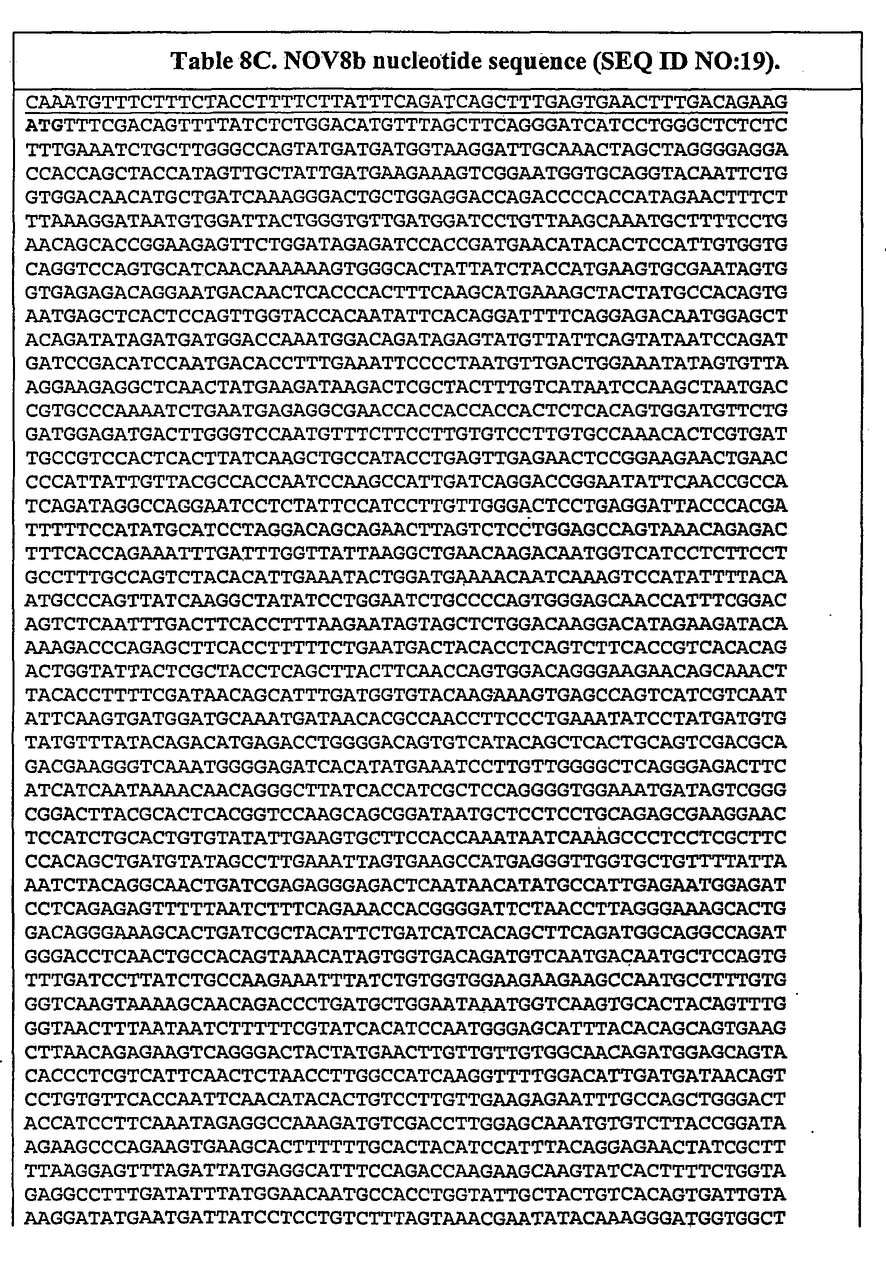

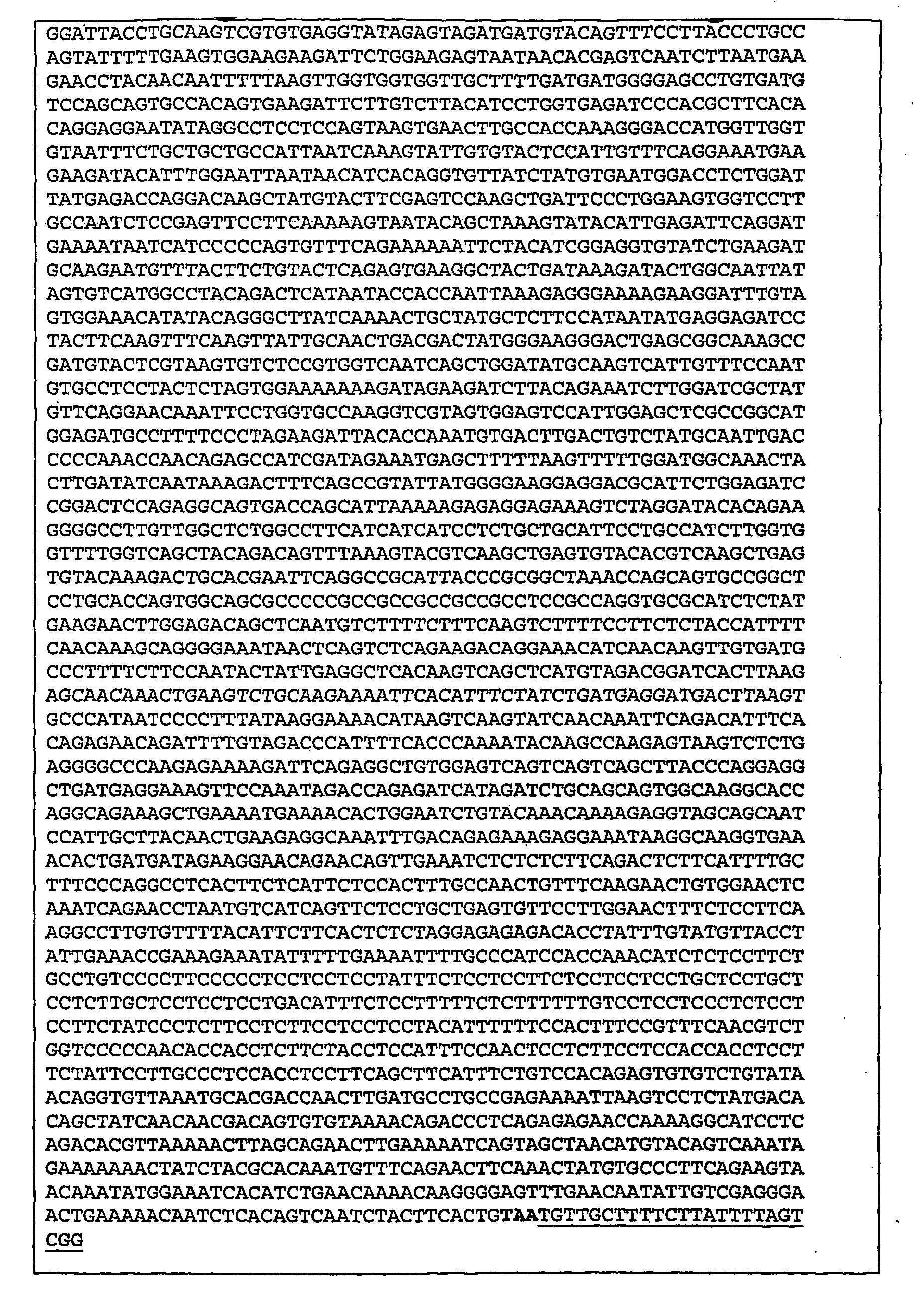

In a search of public sequence databases, the NOV8b nucleic acid sequence has 3708 of 4369 bases (84%) identical to a gb:GENBANK-ID:AF281899|acc:AF281899.1 mRNA from Mus musculus (Mus musculus protocadherin (av) mRNA, complete cds). Public nucleotide databases include all GenBank databases and the GeneSeq patent database. 1972 amino acid residues and is presented in Table 8D using the one-letter amino acid code. Signal P, Psort and/or Hydropathy results predict that NOV8b has a signal peptide and is likely to be localized at the plasma membrane with a certainty of 0.6760. The most likely cleavage site is between amino acids 26 and 27.

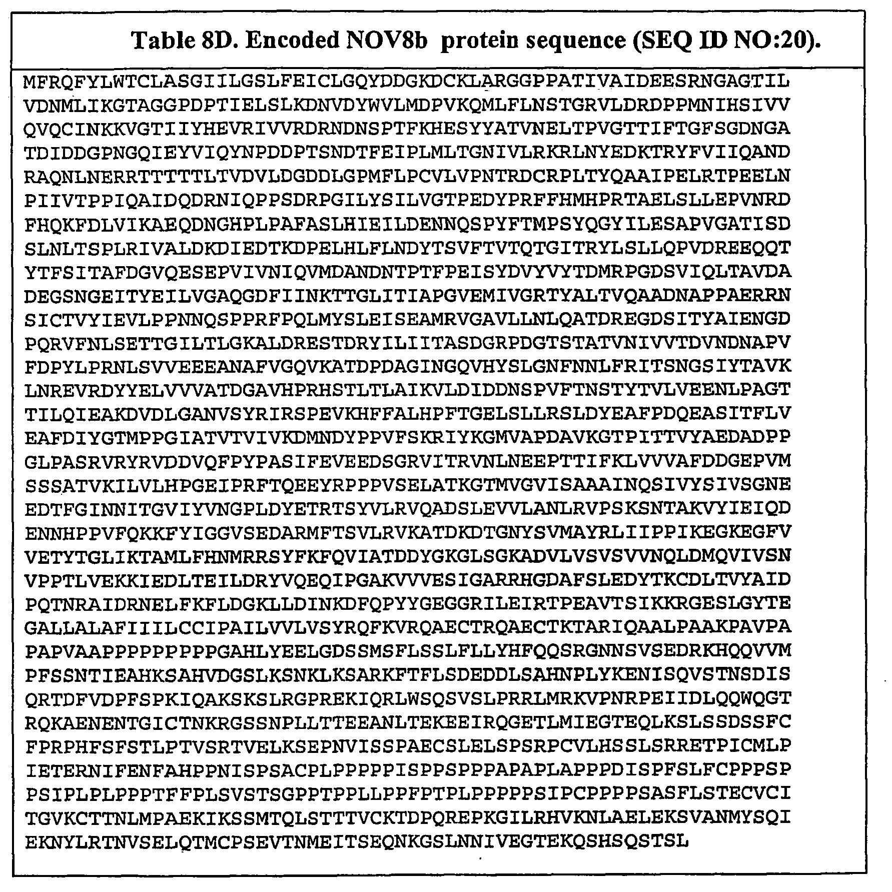

A search of sequence databases reveals that the NOV8b amino acid sequence has 3708 of 4369 bases (84%) identical to a gb:GENBANK-ID:AF281899|acc:AF281899.1 mRNA from Mus musculus (Mus musculus protocadherin (av) mRNA, complete cds). Public amino acid databases include the GenBank databases, SwissProt, PDB and PIR.

NOV8b is expressed in at least adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, by determining the tissue sources of the sequences that were included in the invention including but not limited to SeqCalling sources, Public EST sources, Literature sources, and/or RACE sources.

The disclosed NOV8a polypeptide has homology to the amino acid sequences shown in the BLASTP data listed in Table 8E.

| **Table 8E. BLAST results for NOV8a** | | | | | |
|---|---|---|---|---|---|
| Gene Index/Identifier | Protein/ Organism | Length (aa) | Identity (%) | Positives (%) | Expect |
| gi\|16933555\|ref\|NP 149045.2\| (NM_033056) | protocadherin 15 precursor; Usher syndrome 1F (autosomal recessive, severe) [Homo sapiens] | 1955 | 1954/1973 (99%) | 1955/1973 (99%) | 0.0 |
| gi\|14581464\|gb\|AAK3 1804.1\| (AY029237) | protocadherin 15 [Homo sapiens] | 1955 | 1955/1973 (98%) | 1955/1973 (98%) | 0.0 |
| gi\|15072441\|gb\|AAK3 1581.1 (AY029205) | protocadherin 15 [Homo sapiens] | 1955 | 1949/1973 (98%) | 1955/1973 (98%) | 0.0 |
| gi\|12963485\|ref\|NP 075604.1 (NM_023115) | protocadherin 15; Ames waltzer [Mus musculus] | 1943 | 1626/1977 (82%), Positives 1745/1977 (88%) | 1626/1977 (82%), Positives 1745/1977 (88%) | 0.0 |
| gi\|18574084\|ref\|XP 053625.2\| (XM_053625 | protocadherin 15 precursor [Homo sapiens] | 936 | 924/928 (99%) | 925/928 (99%) | 0.0 |

Table 8F lists the domain descriptions from DOMAIN analysis results against NOV8. This indicates that the NOV8 sequence has properties similar to those of other proteins known to contain this domain.

| **Table 8F. Domain Analysis of NOV8** |
|---|
| gnl Smart smart00112 , CA, Cadherin repeats.; Cadherins are glycoproteins involved in Ca2+-mediated cell-cell adhesion. Cadherin domains occur as repeats in the extracellular regions which are thought to mediate cell-cell contact when bound to calcium. |
| CD-Length = 82 residues, 100.0% aligned |
| Score = 83.2 bits (204), Expect = le-16 |

The disclosed NOV8 polypeptides are members of the protocadherin family, which in turn is one of the six subfamilies of the cadherin superfamily. Cadherins are membrane-associated glycoproteins that mediate cell-cell interactions in a calcium-dependent fashion. Protocadherins may act as cell-cell recognition molecules and may be involved in signal transduction cascades.

The disclosed NOV8 polypeptides have homology to the mouse protocadherin whose mutant version causes the Ames waltzer mouse phenotype, which includes deafness and a balance disorder due to degeneration of the neuroepithelium of the inner ear. Mutant mice show abnormal stereocilia in the inner ear at a very early age. The gene of invention may therefore have a role in developmental processes, cellular communication and disease processes such as cancer.

The disclosed NOV8 nucleic acids of the invention encode a protocadherin-like protein includes the nucleic acid whose sequence is provided in Table 8A or 8C or a fragment thereof. The invention also includes a mutant or variant nucleic acid any of whose bases may be changed from the corresponding base shown in Table 8A or 8C while still encoding a protein that maintains its protocadherin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 42 percent of the bases may be so changed. whose sequence is provided in Table 8B or 8D. The invention also includes a mutant or variant protein any of whose residues may be changed from the corresponding residue shown in Table 8B or 8D while still encoding a protein that maintains its protocadherin-like activities and physiological functions, or a functional fragment thereof. In the mutant or variant protein, up to about 15 percent of the residues may be so changed.

The invention further encompasses antibodies and antibody fragments, such as F_{ab} or (F_{ab})₂, that bind immunospecifically to any of the proteins of the invention.

The above defined information for this invention suggests that this protocadherin-like protein (NOV8) may function as a member of a "protocadherin family". Therefore, the NOV8 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV8 nucleic acids and proteins of the invention are useful in potential therapeutic applications implicated in diseases including but not limited to various pathologies and disorders as indicated below. For example, a cDNA encoding the protocadherin-like protein (NOV8) may be useful in gene therapy, and the protocadherin-like protein (NOV8) may be useful when administered to a subject in need thereof. By way of nonlimiting example, the compositions of the present invention will have efficacy for treatment of patients suffering from Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegenerationhemophilia, hypercoagulation, idiopathic thrombocytopenic purpura, autoimmune disease, allergies, immunodeficiencies, transplantation, graft versus host disease (GVHD), lymphaedema, hearing loss, tinnitus, balance disorders, cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, transplantation, cancer, tissue degeneration, bacterial/viral/parasitic infection, or other pathologies or conditions. The NOV8 nucleic acid useful in diagnostic applications, wherein the presence or amount of the nucleic acid or the protein are to be assessed.

NOV8 nucleic acids and polypeptides are further useful in the generation of antibodies that bind immuno-specifically to the novel NOV8 substances for use in therapeutic or diagnostic methods. These antibodies may be generated according to methods known in the art, using prediction from hydrophobicity charts, as described in the "Anti-NOVX Antibodies" section below. The disclosed NOV8 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV9

A disclosed NOV9 nucleic acid of 13700 nucleotides (also referred to as CG57625-01) encoding a protocadherin-like protein is shown in Table 9A. Putative untranslated regions upstream and/or downstream from the coding region, if any, are underlined, and the start and stop codons are in bold letters.

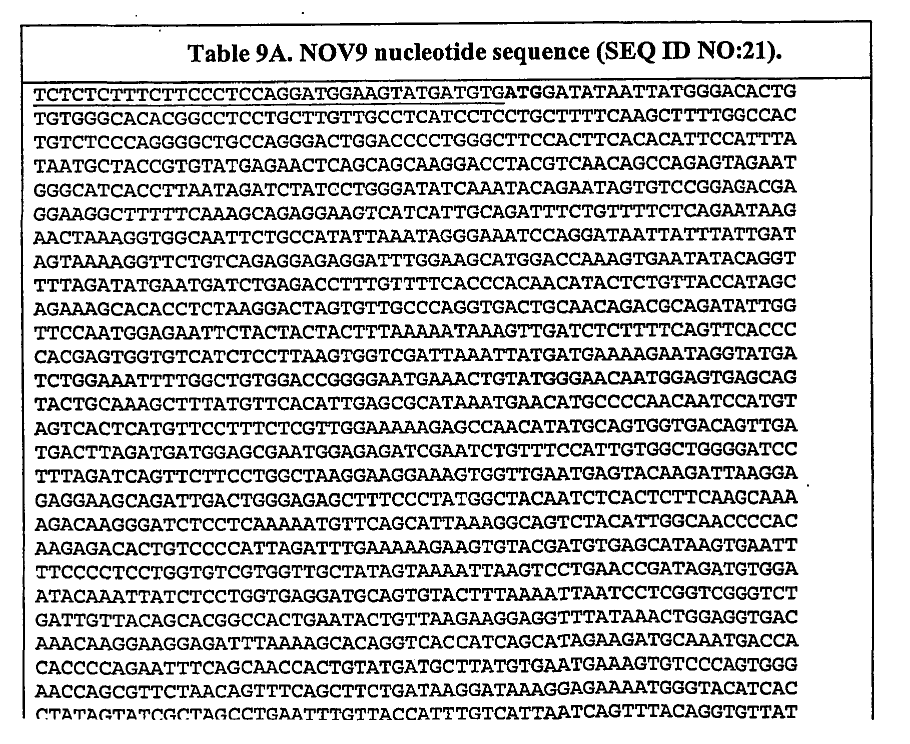

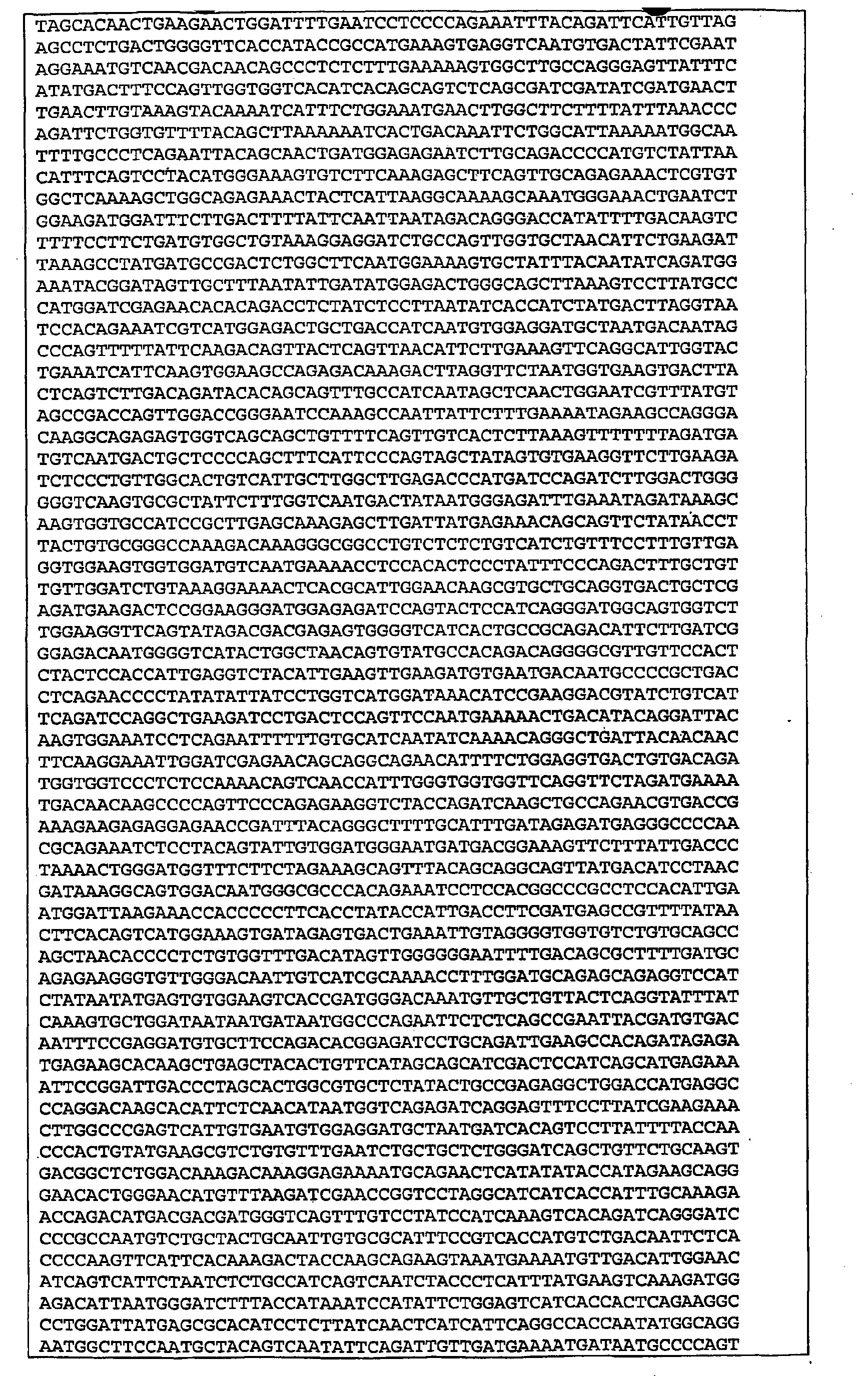

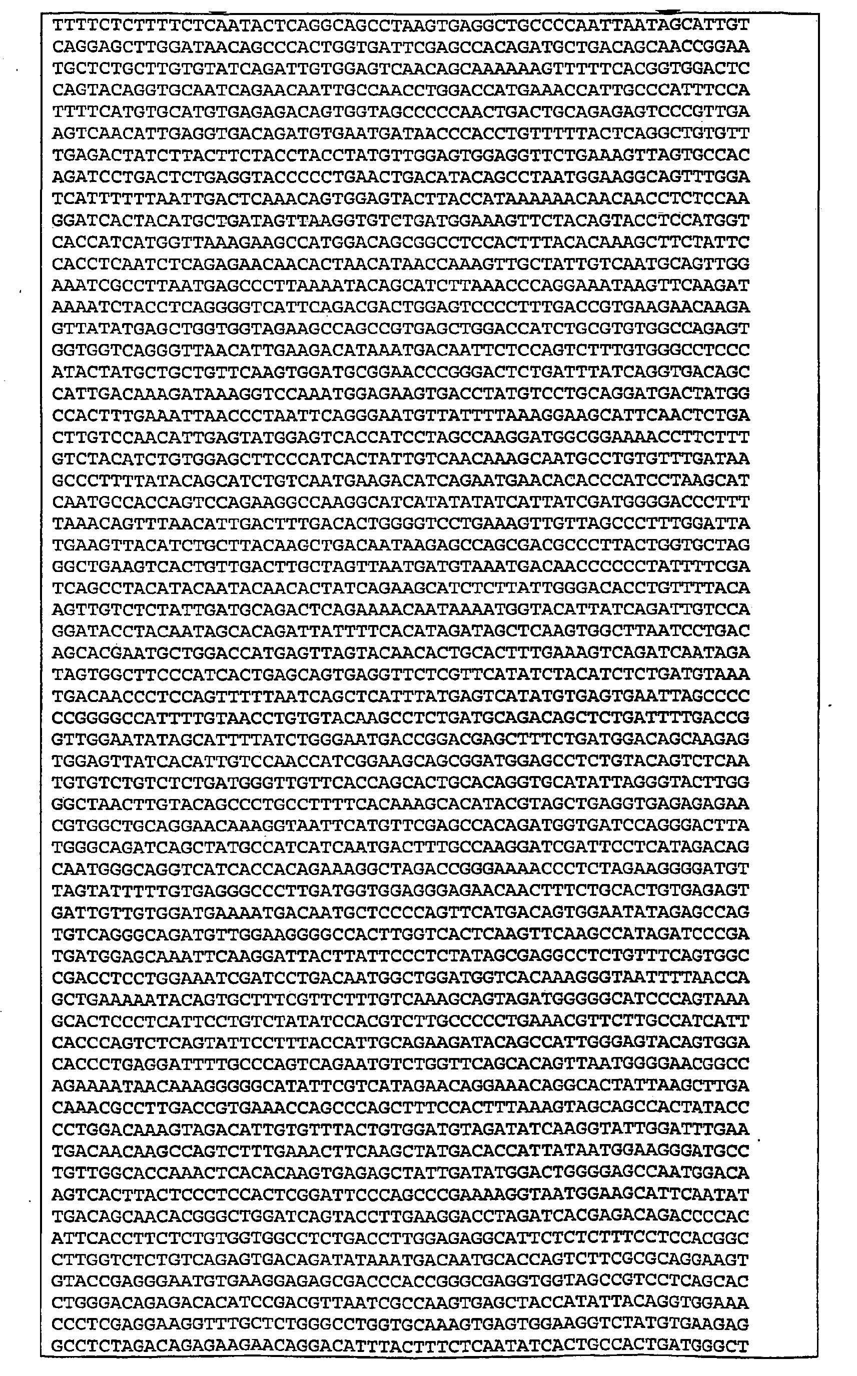

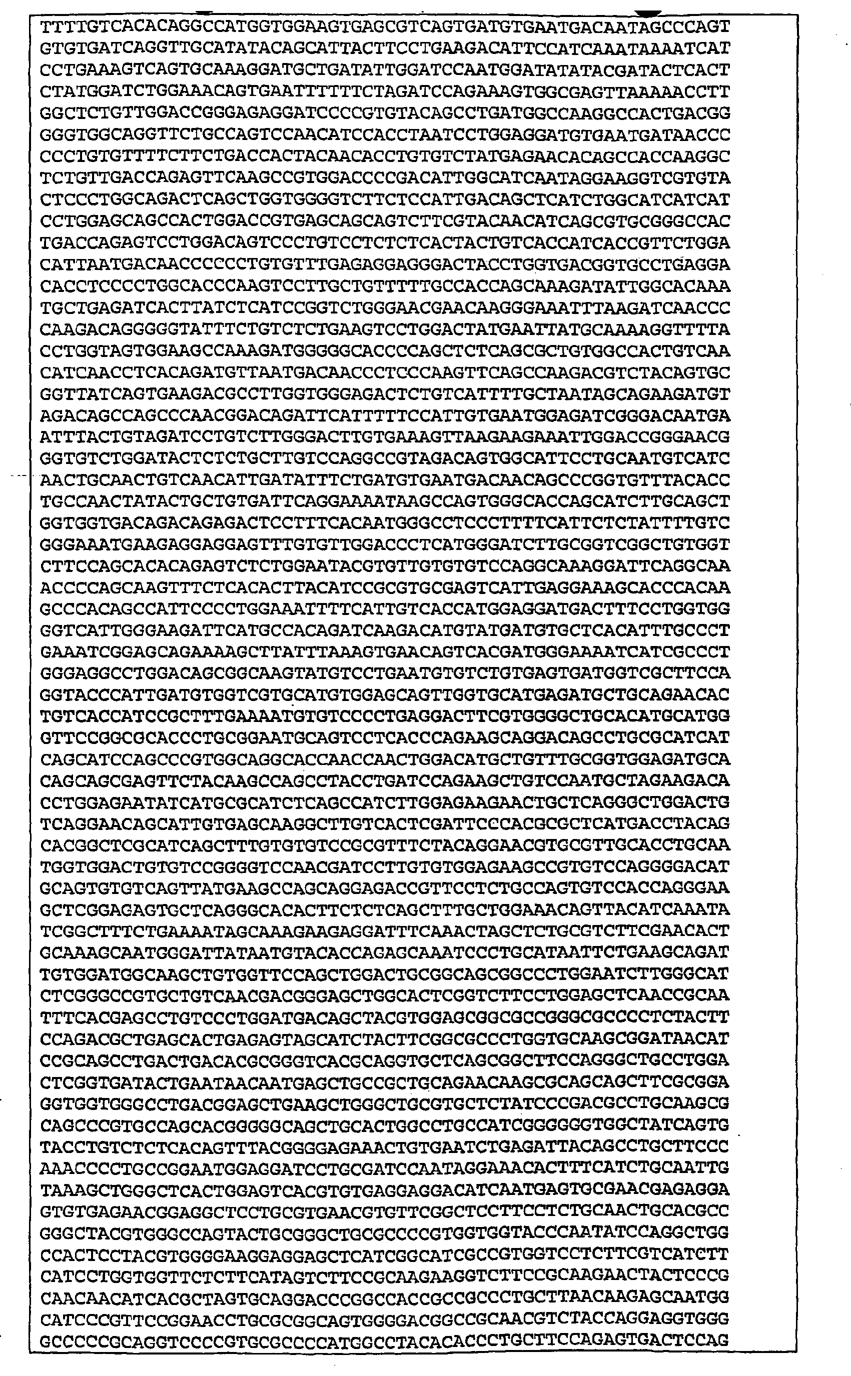

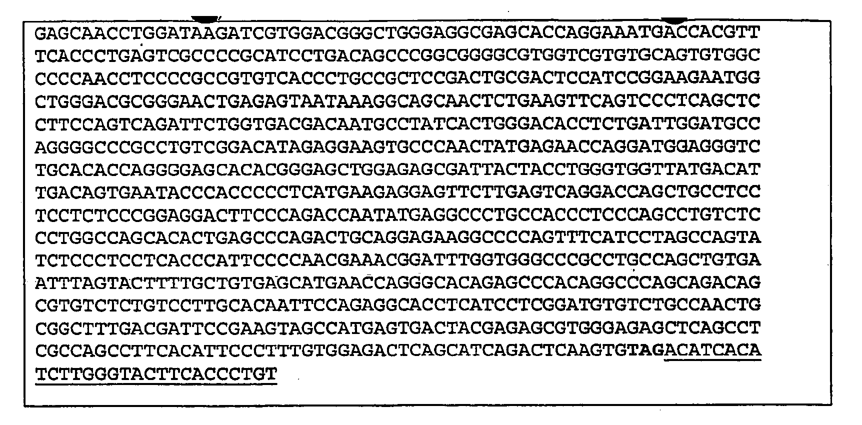

In a search of public sequence databases, the NOV9 nucleic acid sequence, located on chromsome 11 has 4976 of 7882 bases (63%) identical to a gb:GENBANK-ID:AF100960|acc:AF100960.1 mRNA from Rattus norvegicus (Rattus norvegicus protocadherin (Fat) mRNA, complete cds). Public nucleotide databases include all GenBank databases and the GeneSeq patent database.

The disclosed NOV9 polypeptide (SEQ ID NO:22) encoded by SEQ ID NO:21 has 4544 amino acid residues and is presented in Table 9B using the one-letter amino acid code. Signal P, Psort and/or Hydropathy results predict that NOV9 has 2 signal peptide and is likely to be localized extracellularly with a certainty of 0.4600. The most likely cleavage site is between residues 32 and 33.

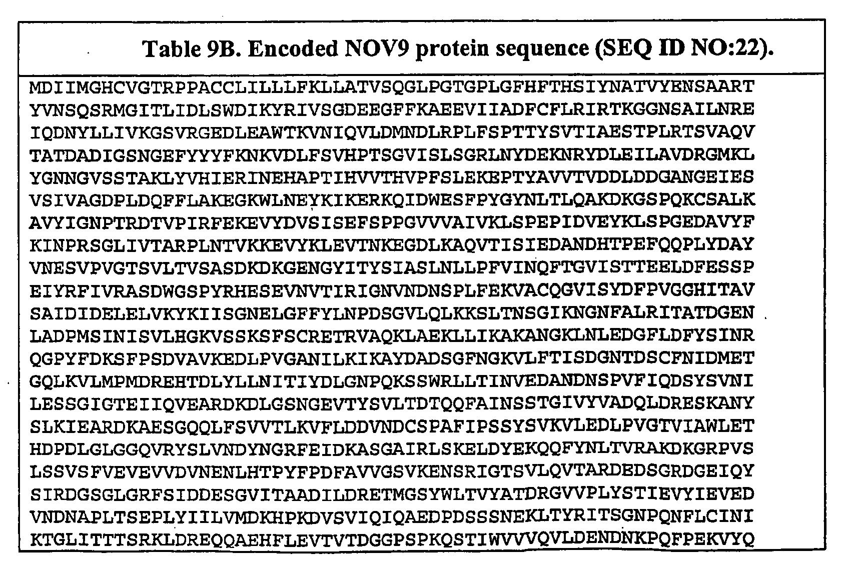

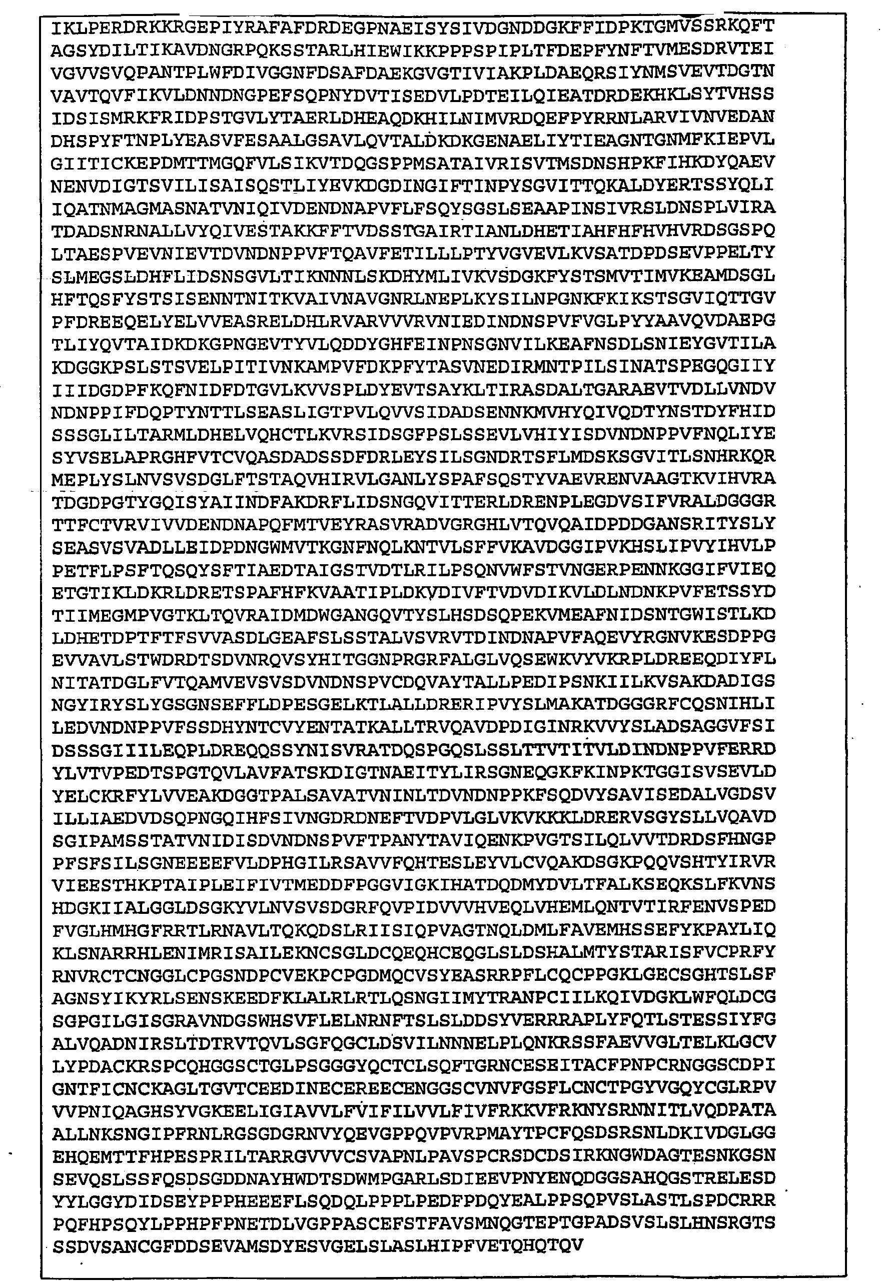

A search of sequence databases reveals that the NOV9 amino acid sequence has 2201 of 4118 amino acid residues (53%) identical to, and 2931 of 4118 amino acid residues (71 %) similar to, the 4589 amino acid residue ptnr: SPTREMBL-ACC:Q9WU10 protein from Rattus norvegicus (Rat) (PROTOCADHERIN). Public amino acid databases include the GenBank databases, SwissProt, PDB and PIR.

NOV9 is expressed in at least breast, prostate, bone marrow, brain, liver, stomach, pituitary, cartilage. This information was derived by determining the tissue sources of the sequences that were included in the invention including but not limited to SeqCalling sources, Public EST sources, Literature sources, and/or RACE sources.

The disclosed NOV9 polypeptide has homology to the amino acid sequences shown in the BLASTP data listed in Table 9C.

| **Table 9C. BLAST results for NOV9** | | | | | |
|---|---|---|---|---|---|
| Gene Index/ Identifier | Protein/ Organism | Length (aa) | Identity (%) | Positives (%) | Expect |
| gi\|18578022\|ref\|XP 061871.2\| (XM_061871) | similar to FAT tumor suppressor (Drosophila) | 3446 | 3319/3386 (98%) | 3320/3386 (98%) | 0.0 |
| gi\|4885229\|ref\|NP 0 05236.1\| (NM_005245) | FAT tumor suppressor precursor | 4620 | 2381/4620 (51%) | 3178/4620 (68%) | 0.0 |
| gi\|13929168\|ref\|NP 114007.1\| (NM_031819) | FATtumorsuppressor (Drosophila) homolog [Rattus norvegicus] | 4589 | 2368/4619 (51%) | 3170/4619 (68%) | 0.0 |
| gi\|6688786\|emb\|CAB6 5271.1\| (AJ250768 | mouse fat 1 cadherin [Mus musculus] | 4587 | 2367/4633 (51%) | 3167/4633 (68%) | 0.0 |
| gi\|13787217\|ref\|NP 001438.1\| (NM_001447) | FAT tumor suppressor 2 precursor [Homo sapiens] | 4349 | 1854/4434 (41%) | 2635/4434 (58%) | 0.0 |

Table 9D lists the domain descriptions from DOMAIN analysis results against NOV9. This indicates that the NOV9 sequence has properties similar to those of other proteins known to contain this domain.

| **Table 9D. Domain Analysis of NOV9** | | |
|---|---|---|
| gnl\|smart\|smart00112, CA, Cadherin repeats.; Cadherins are glycoproteins involved in Ca2+-mediated cell-cell adhesion. Cadherin domains occur as repeats in the extracellular regions which are thought to mediate cell-cell contact when bound to calcium. | | |
| | CD-Length = 82 residues, 100.0% aligned | |
| | | Score = 97.4 bits (241), Expect = 2e-20 |

| **Table 9E. Domain Analysis of NOV9** | |
|---|---|
| gnl \|Pfam \|pfam00028, cadherin, Cadherin domain | |
| | CD-Length = 92 residues, 100.0% aligned |
| | Score = 94.4 bits (233), Expect = le-19 |

NOV9 is a member of the protocadherin family, which in turn is one of the six subfamilies of the cadherin superfamily. Cadherins are membrane-associated glycoproteins that mediate cell-cell interactions in a calcium-dependent fashion. Protocadherins may act as cell-cell recognition molecules and may be involved in signal transduction cascades.

NOV9 has homology to the rat protocadherin that is most related to the Drosophila FAT gene. The Drosophila FAT gene shows the presence of multiple characteristic cadherin domains and is likely involved in cell guidance, cell repulsion and/or cell adhesion. Recessive lethal mutations in the fat locus of Drosophila cause hyperplastic, tumor-like overgrowth of larval imaginal discs, defects in differentiation and morphogenesis, and death during the pupal stage. This indicates that the fat gene has a tumor suppressor function (See Mahoney et al., Cell 1991 Nov 29;67(5):853-68).

The disclosed NOV nucleic acid of the invention encoding a protocadherin-like protein includes the nucleic acid whose sequence is provided in Table 9A or a fragment thereof. The invention also includes a mutant or variant nucleic acid any of whose bases may be changed from the corresponding base shown in Table 9A while still encoding a protein that maintains its protocadherin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 37 percent of the bases may be so changed.

The disclosed NOV9 protein of the invention includes the protocadherin-like protein whose sequence is provided in Table 9B. The invention also includes a mutant or variant protein anv of whose residues mav be changed from the corresponding residue shown in Table 9B while still encoding a protein that maintains its protocadherin-like activities and physiological functions, or a functional fragment thereof. In the mutant or variant protein, up to about 47 percent of the residues may be so changed.

The invention further encompasses antibodies and antibody fragments, such as F_{ab} or (F_{ab})₂, that bind immunospecifically to any of the proteins of the invention.

The above defined information for this invention suggests that this protocadherin-like protein (NOV9) may function as a member of a "protocadherin family". Therefore, the NOV9 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV9 nucleic acids and proteins of the invention are useful in potential therapeutic applications implicated in diseases including but not limited to various pathologies and disorders as indicated below. For example, a cDNA encoding the protocadherin-like protein (NOV9) may be useful in gene therapy, and the protocadherin-like protein (NOV9) may be useful when administered to a subject in need thereof. By way of nonlimiting example, the compositions of the present invention will have efficacy for treatment of patients suffering from hemophilia, hypercoagulation, idiopathic thrombocytopenic purpura, autoimmune disease, allergies, immunodeficiencies, transplantation, graft versus host disease, endocrine dysfunctions, diabetes, obesity, growth and reproductive disorders, Von Hippel-Lindau (VHL) syndrome, cirrhosis, transplantation, hypercalceimia, ulcers, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration,cancer, tissue degeneration, bacterial/viral/parasitic infections, or other pathologies or conditions. The NOV9 nucleic acid encoding the protocadherin-like protein of the invention, or fragments thereof, may further be useful in diagnostic applications, wherein the presence or amount of the nucleic acid or the protein are to be assessed.

NOV9 nucleic acids and polypeptides are further useful in the generation of antibodies that bind immuno-specifically to the novel NOV9 substances for use in therapeutic or diagnostic methods. These antibodies may be generated according to methods known in the art, using prediction from hydrophobicity charts, as described in the "Anti-NOVX Antibodies" section below. The disclosed NOV9 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV10

A disclosed NOV10 nucleic acid of 1071 nucleotides (also referred to as CG57553-01) encoding a T01C1.3-like protein is shown in Table 10A. Putative untranslated regions upstream and/or downstream from the coding region, if any, are underlined, and the start and stop codons are in bold letters.

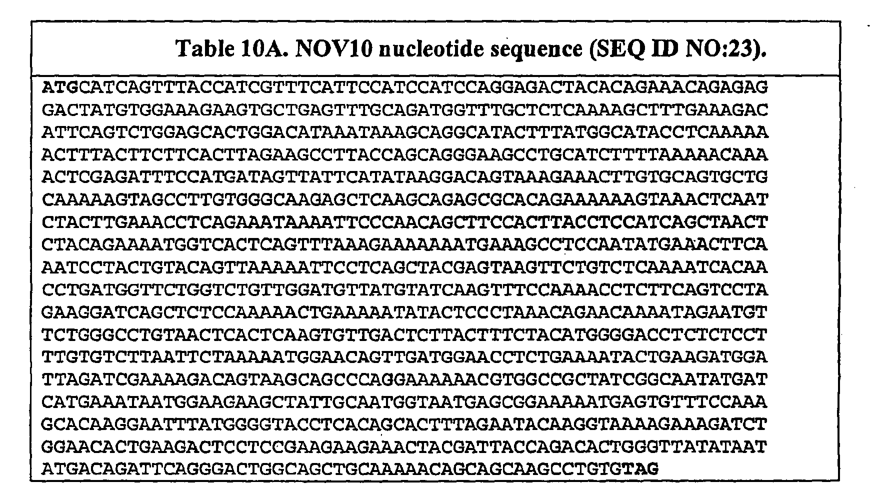

In a search of public sequence databases, the NOV10 nucleic acid sequence, located on chromsome 4 has 165 of 267 bases (61 %) identical to a gb:GENBANK-ID:BGDNA66KD|acc:X87727.1 mRNA from Borrelia garinii (B.garinii p66 gene for 66kDa protein). Public nucleotide databases include all GenBank databases and the GeneSeq pate database.

The disclosed NOV10 polypeptide (SEQ ID NO:24) encoded by SEQ ID NO:23 has 356 amino acid residues and is presented in Table 10B using the one-letter amino acid code. Signal P, Psort and/or Hydropathy results predict that NOV10 has no signal peptide and is likely to be localized in the nucleus with a certainty of 0.7000.

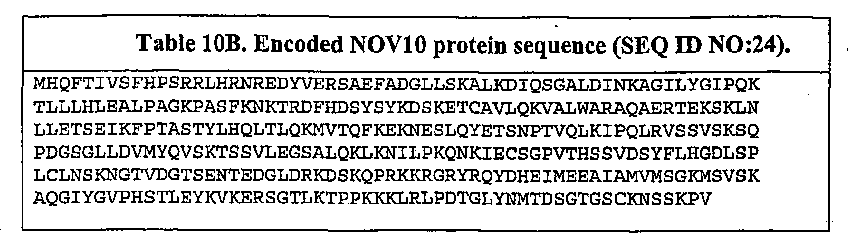

A search of sequence databases reveals that the NOV10 amino acid sequence has 47 of 144 amino acid residues (32%) identical to, and 77 of 144 amino acid residues (53%) similar to, the 185 amino acid residue ptnr:SPTREMBL-ACC:Q22051 protein from Caenorhabditis elegans (T01C1.3 PROTEIN). Public amino acid databases include the GenBank databases, SwissProt, PDB and PIR.

NOV10 is expressed in at least brain and kidney. This information was derived by determining the tissue sources of the sequences that were included in the invention including but not limited to SeqCalling sources, Public EST sources, Literature sources, and/or RACE sources.

The disclosed NOV10 polypeptide has homology to the amino acid sequences shown in the BLASTP data listed in Table 10C.

| **Table 10C. BLAST results for NOV10** | | | | | |
|---|---|---|---|---|---|
| Gene Index/ Identifier | Protein/ Organism | Length (aa) | Identity (%) | Positives (%) | Expect |
| gi\|18559097\|ref\|XP 087581.1\| (XM_087581) | protein XP_087581 [Homo sapiens] | 213 | 213/213 (100%) | 213/213 (100%) | e-110 |
| gi\|16549953\|dbj\|BAB 70892.1\| (AK055258) | unnamed protein product [Homo sapiens] | 213 | 212/213 (99%) | 213/213 (100%) | e-109 |
| gi\|14017807\|dbj\|BAB 47424.1\| (AB058698) | KIAA1795 protein [Homo sapiens] | 572 | 94/242 (38%) | 125/242 (50%) | 7e-28 |
| gi\|14744761\|ref\|XP 050988.1\| (XM_050988) | KIAA1795 protein [Homo sapiens] | 433 | 94/242 (38%) | 125/242 (50%) | 4e-26 |
| gi\|18487927\|ref\|XP 081696.1\| (XM_081696) | Eip93F [Drosophila melanogaster] | 1165 | 37/76 (48%) | 46/76 (59%) | 6e-10 |

The T01C1.3-like protein described in this invention is similar to the *C. elegans* protein T01C1.3, a novel protein. The T01C1.3-like protein appears to be expressed in kidney and brain and may therefore play a role in the development of cancer, neurological diseases, or metabolic disorders. The T01C1.3-like gene maps to human chromosome 4 and has no identifiable domains.

The disclosed NOV10 nucleic acid of the invention encoding a T01C1.3-like protein includes the nucleic acid whose sequence is provided in Table 10A or a fragment thereof. The invention also includes a mutant or variant nucleic acid any of whose bases may be changed from the corresponding base shown in Table 10A while still encoding a protein that maintains its T01C1.3-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 39 percent of the bases may be so changed.

The disclosed NOV10 protein of the invention includes the T01C1.3-like protein whose sequence is provided in Table 10B. The invention also includes a mutant or variant protein any of whose residues may be changed from the corresponding residue shown in Table 10B while still encoding a protein that maintains its T01C1.3-like activities and physiological functions, or a functional fragment thereof. In the mutant or variant protein, up to about 68 percent of the residues may be so changed.

The invention further encompasses antibodies and antibody fragments, such as F_{ab} or (F_{ab})₂, that bind immunospecifically to any of the proteins of the invention.

The above defined information for this invention suggests that this T01C1.3-like protein (NOV10) may function as a member of a "T01C1.3 family". Therefore, the NOV10 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV10 nucleic acids and proteins of the invention are useful in potential therapeutic applications implicated in diseases including but not limited to various pathologies and disorders as indicated below. For example, a cDNA encoding the T01C1.3-like protein (NOV10) may be useful in gene therapy, and the TO1C1.3-like protein (NOV10) may be useful when administered to a subject in need thereof. By way of nonlimiting example, the compositions of the present invention will have efficacy for treatment of patients suffering from cancer, trauma, bacterial and viral infections, in vitro and in vivo regeneration, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, diabetes, autoimmune disease, renal artery-stenosis, interstitial nephritis, glomerulonephritis, polycystic kidney disease, systemic lupus erythematosus, renal tubular acidosis, IgA nephropathy, and hypercalceimia, or other pathologies or conditions. The NOV10 nucleic acid encoding the T01C1.3-like protein of the invention, or fragments thereof, may further be useful in diagnostic applications, wherein the presence or amount of the nucleic acid or the protein are to be assessed.

NOV10 nucleic acids and polypeptides are further useful in the generation of antibodies that bind immuno-specifically to the novel NOV10 substances for use in therapeutic or diagnostic methods. These antibodies may be generated according to methods known in the art, using prediction from hydrophobicity charts, as described in the "Anti-NOVX Antibodies" section below. The disclosed NOV 10 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV 11

NOV11 includes three alpha-macroglobulin-like proteins disclosed below. The disclosed sequences have been named NOV11a, NOV11b and NOV11c.

### NOV 11a

A disclosed NOV11a nucleic acid of 6195 nucleotides (also referred to as CG57488_01) encoding a alpha-macroglobulin-like protein is shown in Table 11A. Putative untranslated regions upstream and/or downstream from the coding region, if any, are underlined, and the start and stop codons are in bold letters.

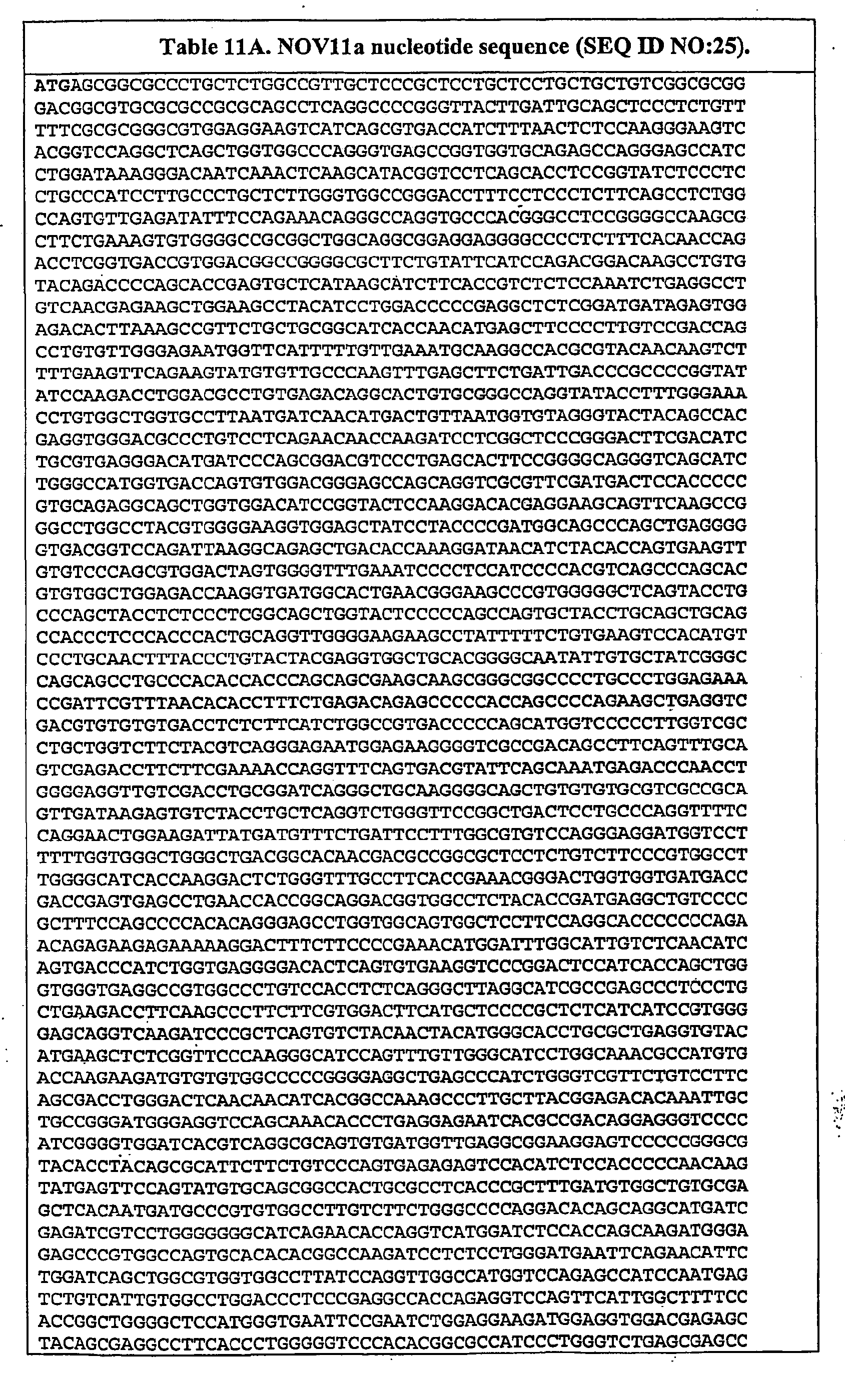

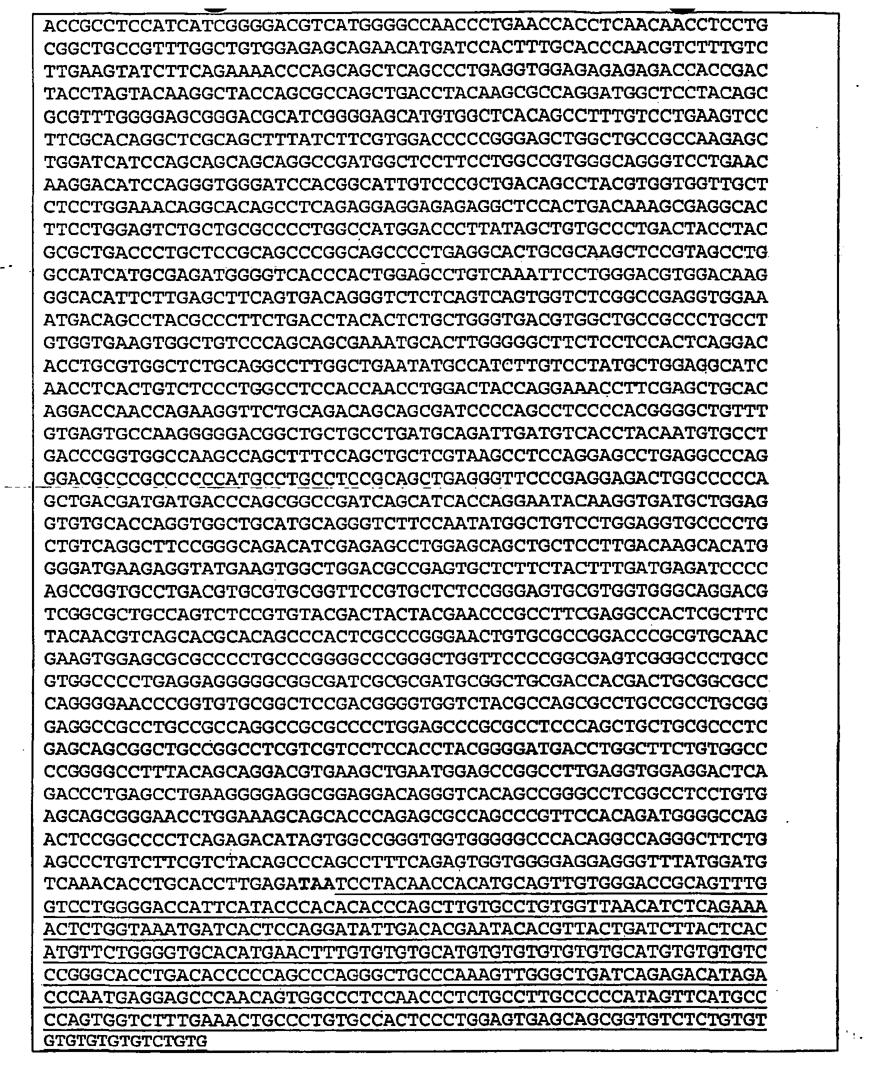

In a search of public sequence databases, the NOV11a nucleic acid sequence, located on chromsome 19 has 5574 of 5594 bases (99%) identical to a gb:GENBANK-ID:AB033109|acc:AB033109.1 mRNA from Homo sapiens (Homo sapiens mRNA for KIAA1283 protein, partial cds). Public nucleotide databases include all GenBank databases and the GeneSeq patent database.

The disclosed NOV11a polypeptide (SEQ ID NO:26) encoded by SEQ ID NO:25 has 1927 amino acid residues and is presented in Table 11B using the one-letter amino acid code. Signal P, Psort and/or Hydropathy results predict that NOV11a has a signal peptide and is likely to be localized at the plasma membrane with a certainty of 0.6400. The most likely cleavage site for a NOV11a peptide is between amino acids 25 and 26.

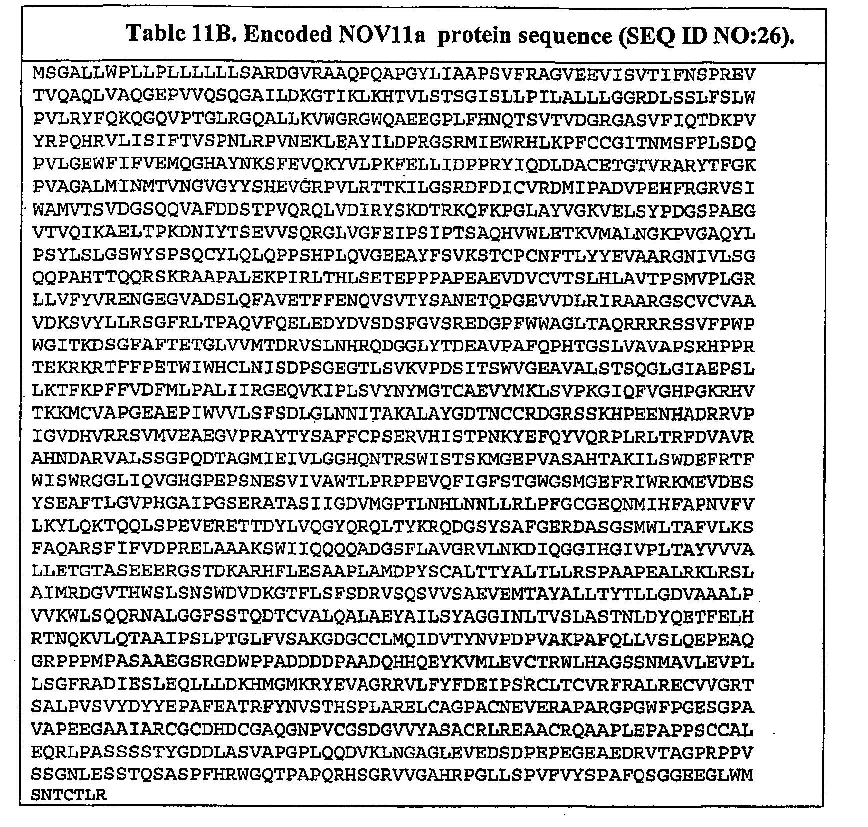

A search of sequence databases reveals that the NOV11a amino acid sequence has 1794 of 1797 amino acid residues (99%) identical to, and 1796 of 1797 amino acid residues (99%) similar to, the 1884 amino acid residue ptnr:SPTREMBL-ACC:Q9ULD7 protein from Homo sapiens (Human) (KIAA1283 PROTEIN). Public amino acid databases include the GenBank databases, SwissProt, PDB and PIR.

NOV11a is expressed in at least Adrenal Gland/Suprarenal gland, Bone Marrow, Brain, Heart, Kidney, Lung, Lymphoid tissue, Mammary gland/Breast, Pituitary Gland, Placenta, Prostate, Retina, Salivary Glands, Spleen, Thalamus, Thyroid. This information was derived by determining the tissue sources of the sequences that were included in the invention including but not limited to SeqCalling sources, Public EST sources, Literature sources, and/or RACE sources.

### NOV 11b

A disclosed NOV11b nucleic acid of 6069 nucleotides (also referred to as CG57488_02) encoding a alpha-macroglobulin-like protein is shown in Table 11C. Putative untranslated regions upstream and/or downstream from the coding region, if any, are underlined, and the start and stop codons are in bold letters.

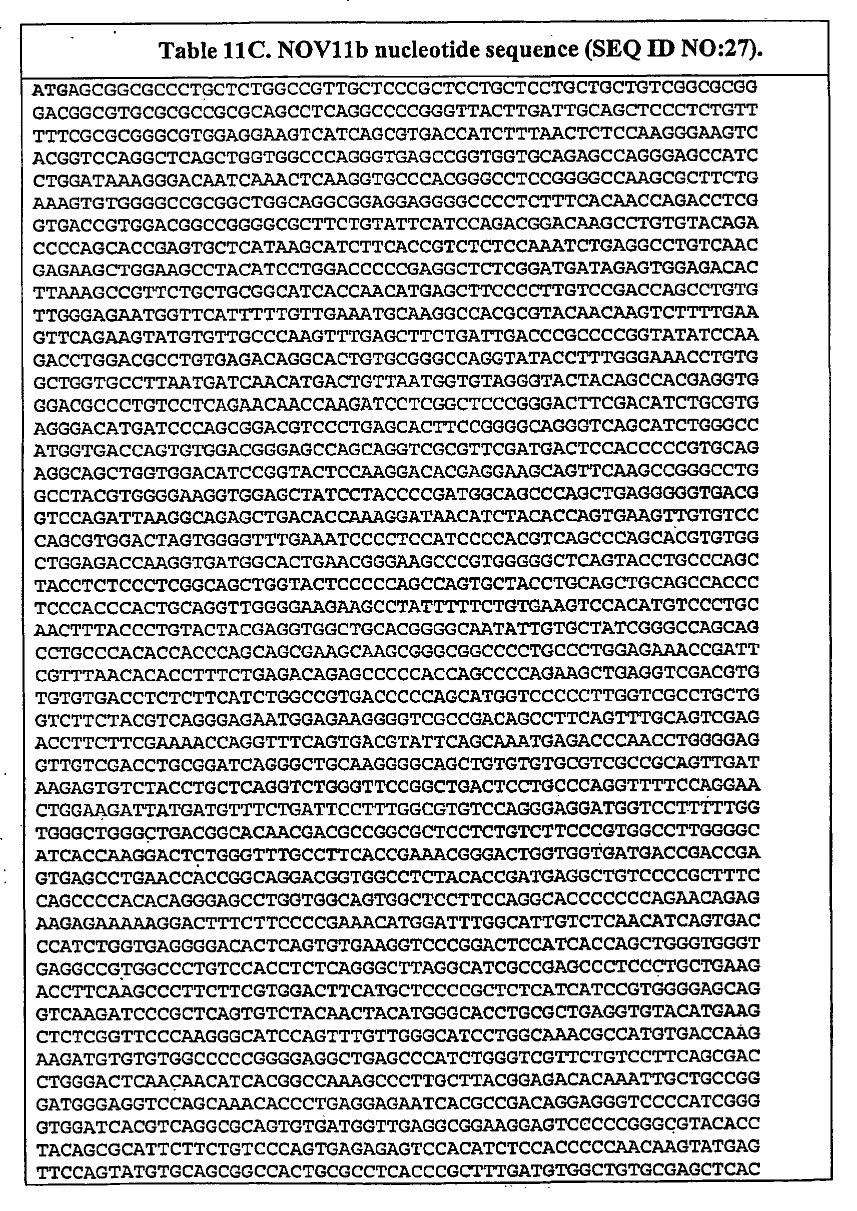

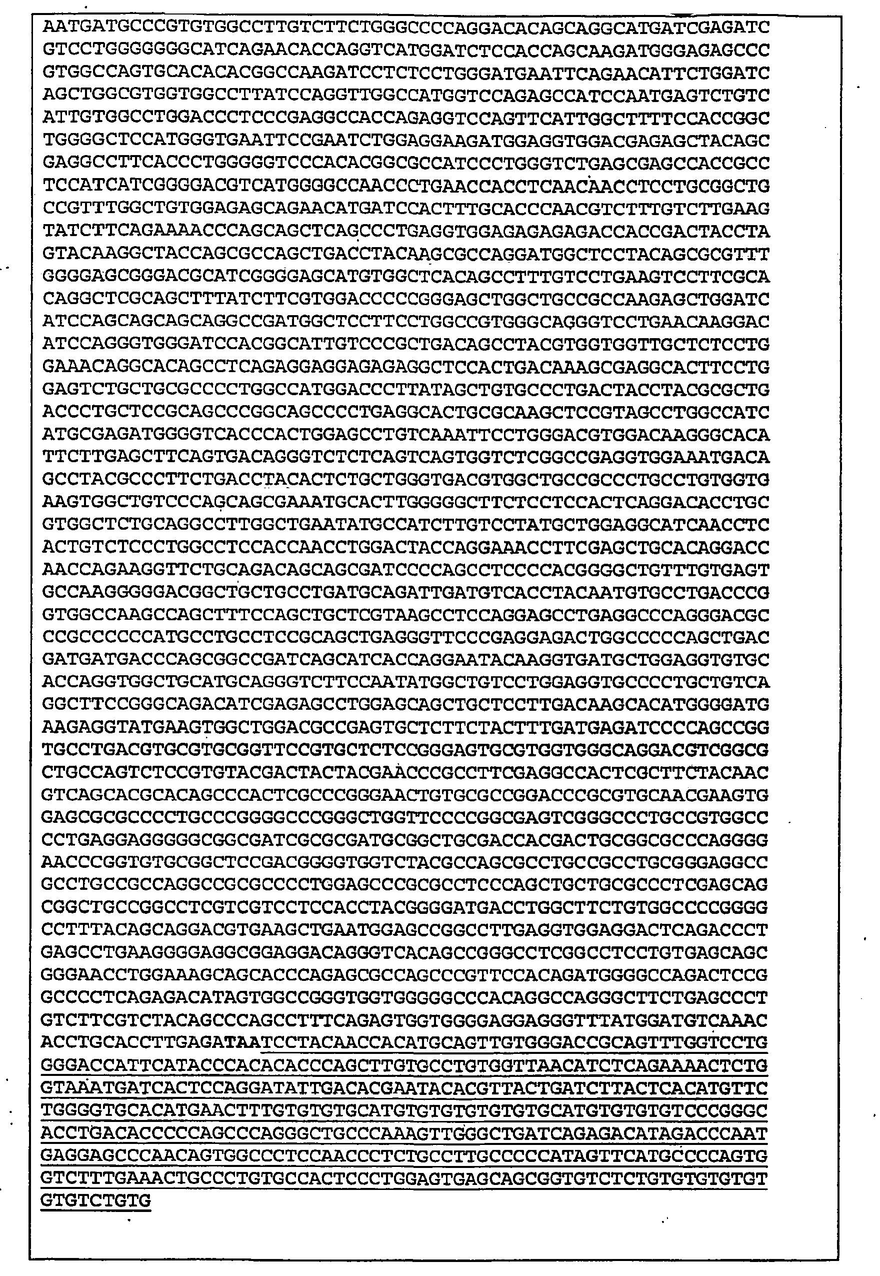

In a search of public sequence databases, the NOV1 1b nucleic acid sequence, located on chromsome 19 has 5815 of 5817 bases (99%) identical to a gb:GENBANK-ID:AB033109|acc:AB033109.1 mRNA from Homo sapiens (Homo sapiens mRNA for KIAA1283 protein, partial cds). Public nucleotide databases include all GenBank databases and the GeneSeq patent database.

The disclosed NOV11b polypeptide (SEQ ID NO:28) encoded by SEQ ID NO:27 has 1885 amino acid residues and is presented in Table D using the one-letter amino acid code. Signal P, Psort and/or Hydropathy results predict that NOV1 1b has a signal peptide and is likely to be localized at the plasma membrane with a certainty of 0.4600. The most likely cleavage site for a NOV11b peptide is between amino acids 25 and 26.

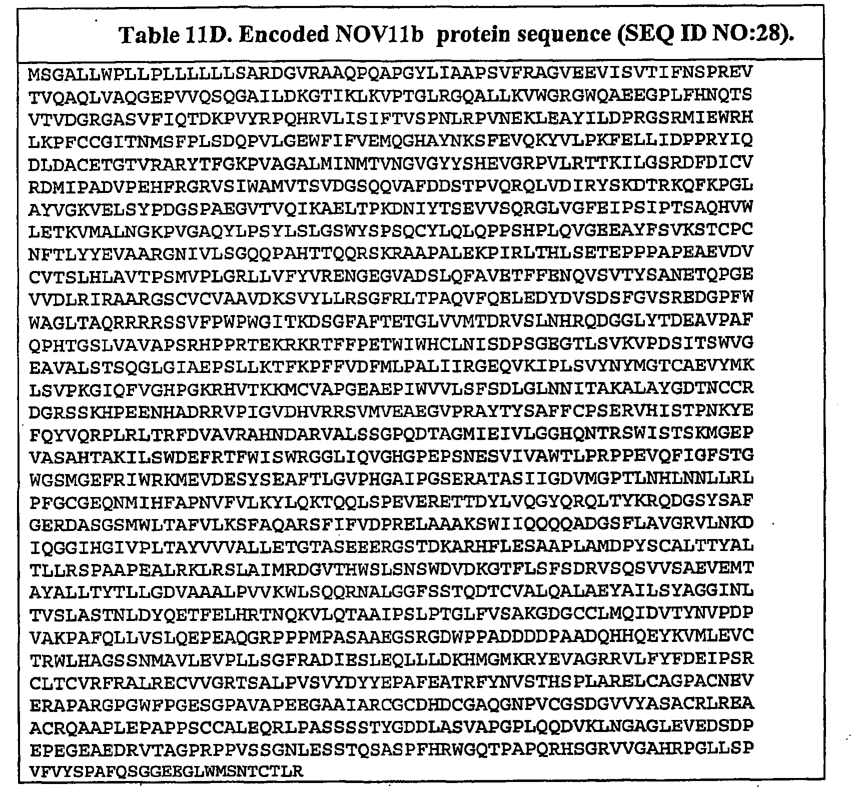

A search of sequence databases reveals that the NOV11b amino acid sequence has 1882 of 1884 amino acid residues (99%) identical to, and 1883 of 1884 amino acid residue (99%) similar to, the 1884 amino acid residue ptnr:SPTREMBL-ACC:Q9ULD7 protein from Homo sapiens (Human) (KIAA1283 PROTEIN). Public amino acid databases include the GenBank databases, SwissProt, PDB and PIR.

NOV11b is expressed in at least Adrenal Gland/Suprarenal gland, Bone Marrow, Brain, Heart, Kidney, Lung, Lymphoid tissue, Mammary gland/Breast, Pituitary Gland, Placenta Prostate Retina Salivary Glands Spleen Thalamus Thyroid. This information was derived by determining the tissue sources of the sequences that were included in the invention including but not limited to SeqCalling sources, Public EST sources, Literature sources, and/or RACE sources.

### NOV 11c

A disclosed NOV11c nucleic acid of 6157 nucleotides (also referred to as CG57488_03) encoding a alpha-macroglobulin-like protein is shown in Table 11E. Putative untranslated regions upstream and/or downstream from the coding region, if any, are underlined, and the start and stop codons are in bold letters.

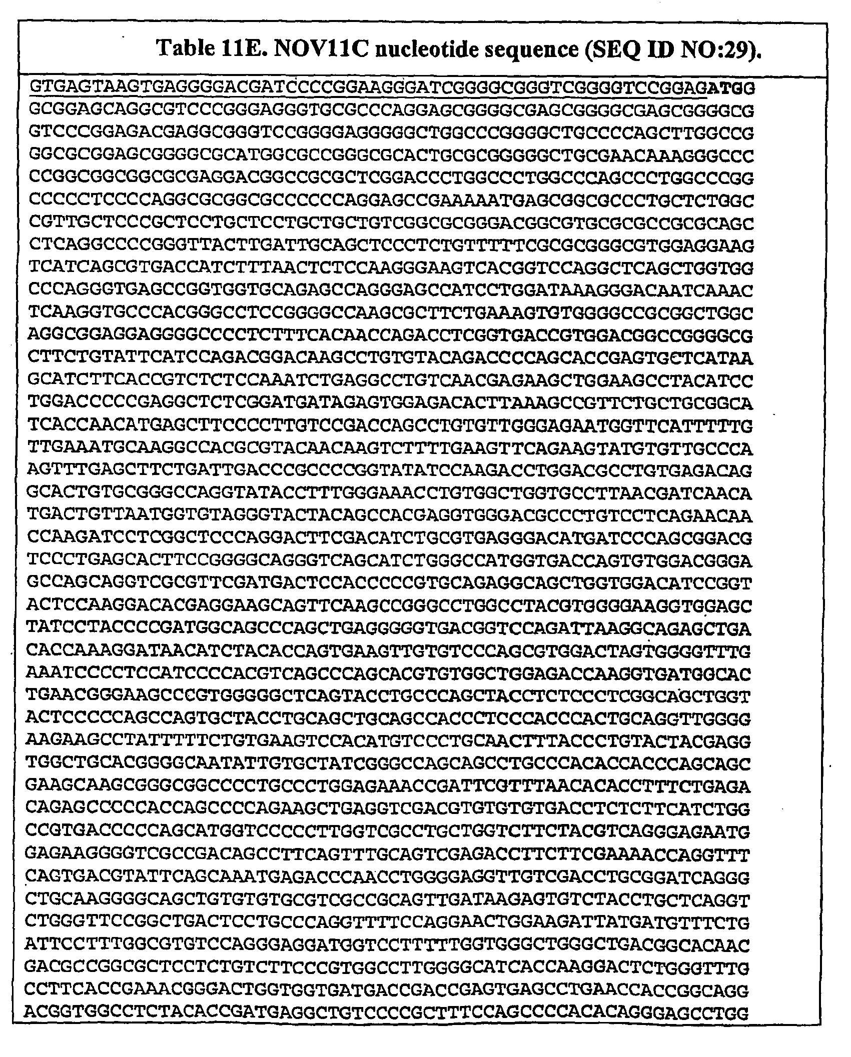

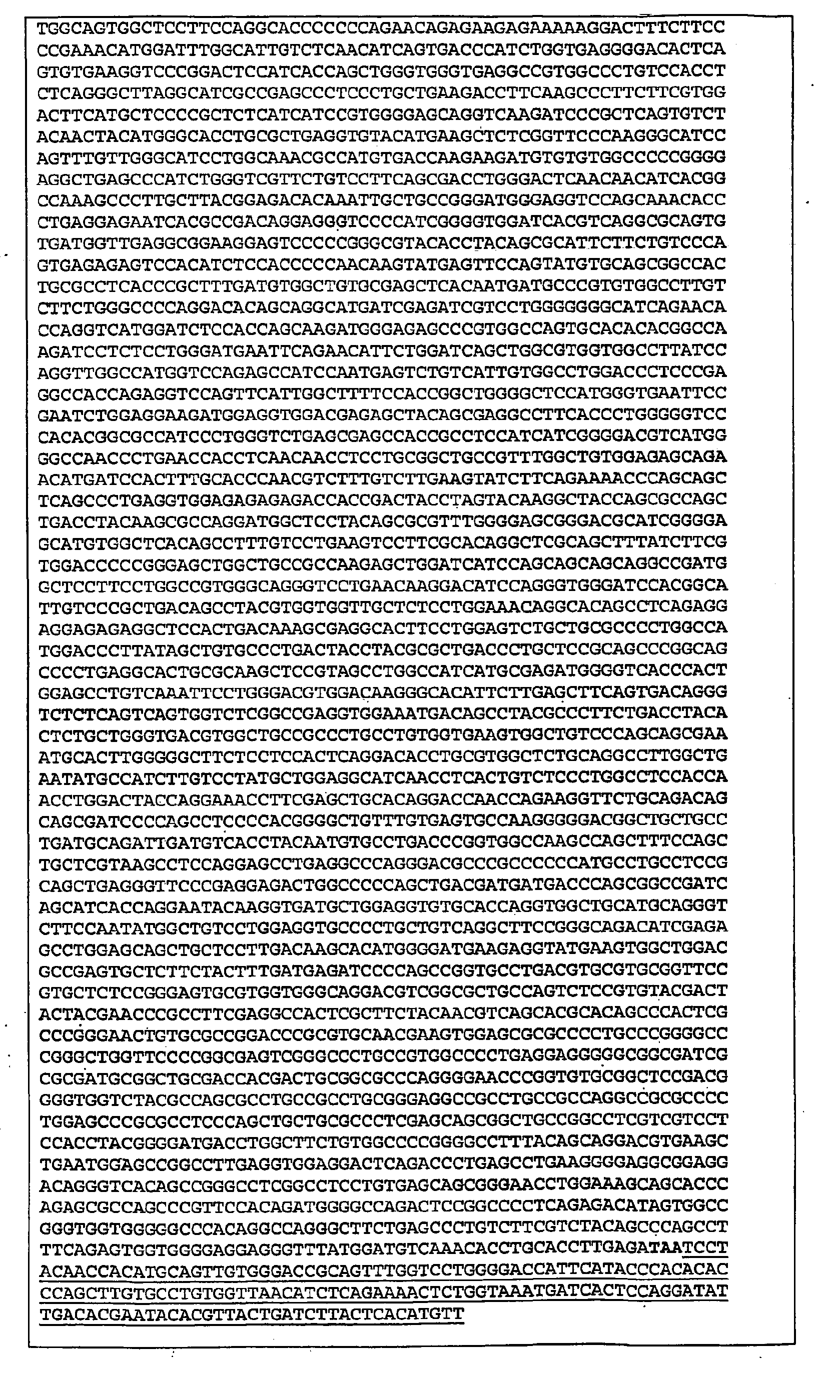

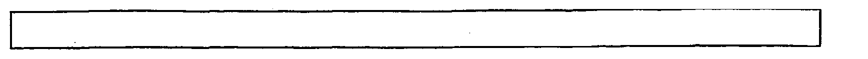

In a search of public sequence databases, the NOV11C nucleic acid sequence, located on chromsome 19 has 332 of 513 (64%) identical to a GENBANK-ID:GPIMSPB|acc:D84339.1 Cavia porcellus mRNA for murinoglobulin. Public nucleotide databases include all GenBank databases and the GeneSeq patent database.

The disclosed NOV11C polypeptide (SEQ ID NO:30) encoded by SEQ ID NO:29 has 1979 amino acid residues and is presented in Table 11F using the one-letter amino acid code. Signal P, Psort and/or Hydropathy results predict that NOV11C has no signal peptide and is likely to be localized at the plasma membrane with a certainty of 0.6000.

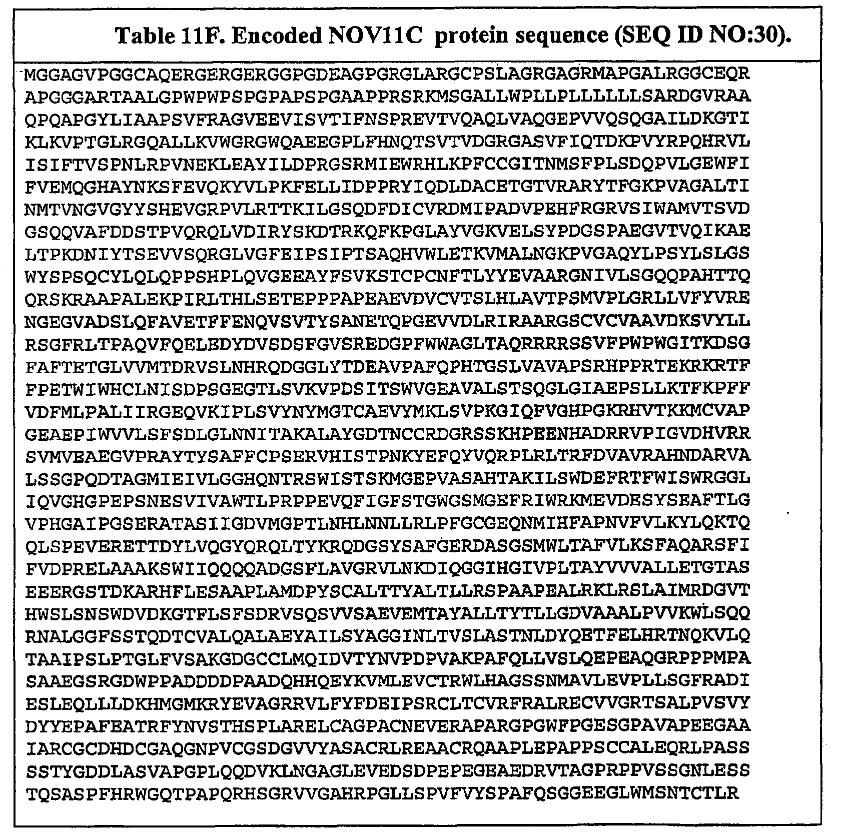

A search of sequence databases reveals that the NOV11C amino acid sequence has 171 of 432 amino acid residues (39%) identical to, and 258 of 432 amino acid residues (58%) similar to, the guinea pig protein ptnr:SPTREMBL-ACC:Q60486 ALPHA-MACROGLOBULIN PRECURSOR - Cavia porcellus. Public amino acid databases include the GenBank databases, SwissProt, PDB and PIR.

The disclosed NOV11a polypeptide has homology to the amino acid sequences shown in the BLASTP data listed in Table 11G.

| **Table 11G. BLAST results for NOV11** | | | | | |
|---|---|---|---|---|---|
| Gene Index/ Identifier | Protein/ Organism | Length (aa) | Identity (%) | Positives (%) | Expect |
| gi\|6331358\|dbj\|BAA8 6597.1\| (AB033109 | KIAA1283 protein [Homo sapiens] | 1884 | 1882/1926 (97%) | 1883/1926 (97%) | 0.0 |
| gi\|15302736\|ref\|XP 050563.2\| (XM_050563 | KIAA1283 protein [Homo sapiens] | 1711 | 1710/1711 (99%) | 1710/1711 (99%) | 0.0 |
| gi\|18567969\|ref\|XP 095282.1 (XM 095282 | Similar to KIAA1283 protein [Homo sapiens] | 837 | 665/732 (90%) | 667/732 (90%) | 0.0 |
| gi\|13928544\|dbj\|BAB 47146.1\| (AB050668 | complement component C3 [Branchiostoma belcheri] | 1732 | 220/611 (36%) | 313/611 (51%), | 4e-92 |
| gi\|17975514\|ref\|NP 523506.1\| (NM_078782) | Thiolester containing protein II [Drosophila melanogaster] | 1420 | 195/583 (33%) | 312/583 (53%) | 4e-85 |

Tables 11H-I lists the domain descriptions from DOMAIN analysis results against NOV11. This indicates that the NOV11 sequence has properties similar to those of other proteins known to contain this domain.

| **Table 11H. Domain Analysis of NOV11** |
|---|
| gnl\|Pfam\|pfam00207, A2M, Alpha-2-macroglobulin family. This family includes the C-terminal region of the alpha-2-macroglobulin family. |
| CD-Length = 751 residues, Score = 330 bits (847), Expect = 3e-91 |

| **Table 11I. Domain Analysis of NOV11** | |
|---|---|
| gnl\|Smart\|smart00280, KAZAL, Kazal type serine protease inhibitors; Kazal type serine protease inhibitors and follistatin-like domains. | |
| CD-Length = 46 residues, | Score = 52.0 bits (123), Expect = 3e-07 |

NOV11 is a member of the alpha-macroglobulin family. Alpha-macroglobulin proteins are large extracellular glycoproteins that can bind to and often act as reservoirs of growth factors and extracellular enzymes (See Gonias et al., J Biol Chem 2000 Feb 25;275(8):5826-31). Decreased level of these proteins in serum is often a sign of tissue damage (See Ruaux et al., Res Vet Sci 1999 Aug;67(1):83-7; Levine et al., J Pediatr Gastroenterol Nutr 1989 Nov;9(4):517-20; Wiedermann et al., Neoplasma 1978;25(2):189-96). These proteins may also help defend the body against bacterial or parasitic infection (See Araujo-Jorge et al., Parasitol Res 1992;78(3):215-21).

The disclosed NOV11 nucleic acid of the invention encoding an alpha-macroglobulin-like protein includes the nucleic acid whose sequence is provided in Table 11A, 11C or 11E or a fragment thereof. The invention also includes a mutant or variant nucleic acid any of whose bases may be changed from the corresponding base shown in Table 11A, 11C or 11E while still encoding a protein that maintains its alpha-macroglobulin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1 percent of the bases may be so changed.

The disclosed NOV11 protein of the invention includes the alpha-macroglobulin-like protein whose sequence is provided in Table 11B, 11D or 11F. The invention also includes a mutant or variant protein any of whose residues may be changed from the corresponding residue shown in Table 11B, 11D or 11F while still encoding a protein that maintains its alpha-macroglobulin-like activities and physiological functions, or a functional fragment thereof. In the mutant or variant protein, up to about 1 percent of the residues may be so changed.

The invention further encompasses antibodies and antibody fragments, such as F_{ab} or (F_{ab})_{2,} that bind immunospecifically to any of the proteins of the invention.

The above defined information for this invention suggests that this alpha-macroglobulin-like protein (NOV11) may function as a member of a "alpha-macroglobulin family". Therefore, the NOV11 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV11 nucleic acids and proteins of the invention are useful in potential therapeutic applications implicated in diseases including but not limited to various pathologies and disorders as indicated below. For example, a cDNA encoding the alpha-macroglobulin-like protein (NOV11) may be useful in gene therapy, and the alpha-macroglobulin-like protein (NOV11) may be useful when administered to a subject in need thereof. By way of nonlimiting example, the compositions of the present invention will have efficacy for treatment of patients suffering from cancer,trauma, regeneration (in vitro and in vivo), viral/bacterial/parasitic infections, adrenoleukodystrophy , congenital adrenal hyperplasia, hemophilia, hypercoagulation, idiopathic thrombocytopenic purpura, autoimmune disease, allergies, immunodeficiencies, transplantation, graft versus host disease, cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, systemic lupus erythematosus, autoimmune disease, asthma, emphysema, scleroderma, allergy, ARDS, renal artery stenosis, interstitial nephritis, glomerulonephritis, polycystic kidney disease, systemic lupus erythematosus, renal tubular acidosis, IgA nephropathy, hypercalceimia, Lesch-Nyhan syndrome, Von Hippel-Lindau (VHL) syndrome, diabetes, tuberous sclerosis, xerostomia, fertility, endocrine dysfunctions, growth and reproductive disorders, or other pathologies or conditions. The NOV1 nucleic acid encoding the alpha-macroglobulin-like protein of the invention, or fragments thereof, may further be useful in diagnostic applications, wherein the presence or amount of the nucleic acid or the protein are to be assessed.

NOV11 nucleic acids and polypeptides are further useful in the generation of antibodies that bind immuno-specifically to the novel NOV11 substances for use in therapeutic or diagnostic methods. These antibodies may be generated according to methods known in the art, using prediction from hydrophobicity charts, as described in the "Anti-NOVX Antibodies" section below. The disclosed NOV11 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV12

NOV12 includes two orphan transporter-like proteins disclosed below. The disclosed sequences have been named NOV12a and NOV12b.

### NOV12a

A disclosed NOV 12a nucleic acid of 2119 nucleotides (also referred to as CG57526-01) encoding an orphan receptor-like protein is shown in Table 12A. Putative untranslated regions upstream and/or downstream from the coding region, if any, are underlined, and the start and stop codons are in bold letters.

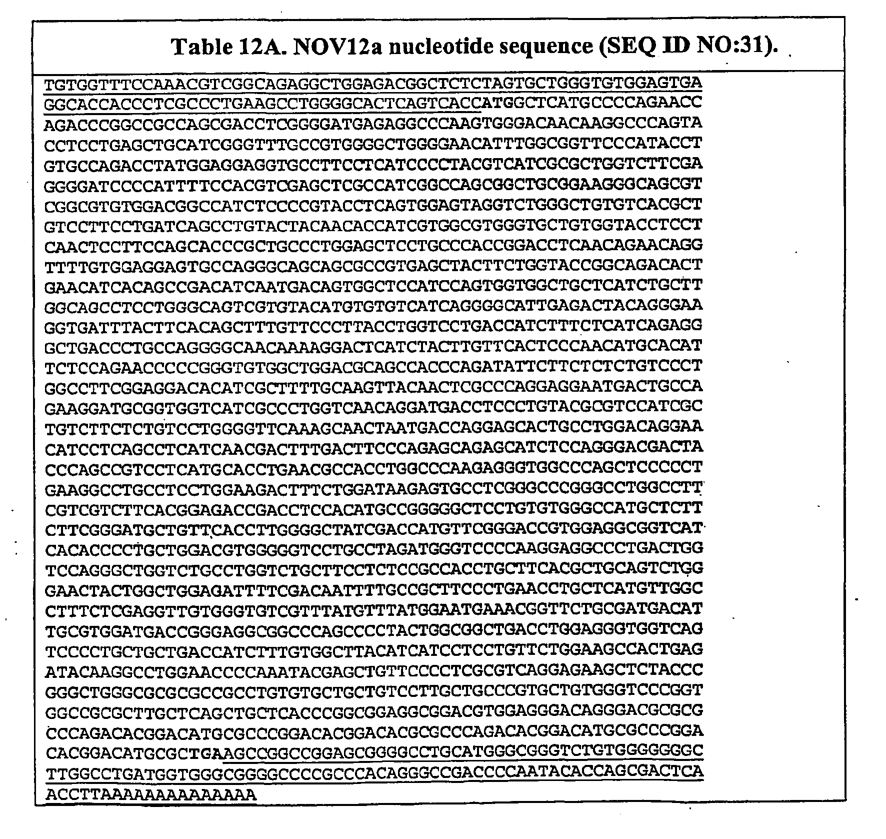

In a search of public sequence databases, the NOV 12a nucleic acid sequence, located on chromsome 5 has 1122 of 1396 bases (80%) identical to a gb:GENBANK-ID:AF075263)acc:AF075263.1 mRNA from Mus musculus (Mus musculus orphan transporter isoform A11 (Xtrp2) mRNA, alternatively spliced, complete cds. Public nucleotide databases include all GenBank databases and the GeneSeq patent database.

The disclosed NOV 12a polypeptide (SEQ ID N0:32) encoded by SEQ ID NO:31 has 631 amino acid residues and is presented in Table 12B using the one-letter amino acid code. Signal P, Psort and/or Hydropathy results predict that NOV12a has a signal peptide and is likely to be localized to the plasma membrane with a certainty of 0.8000.

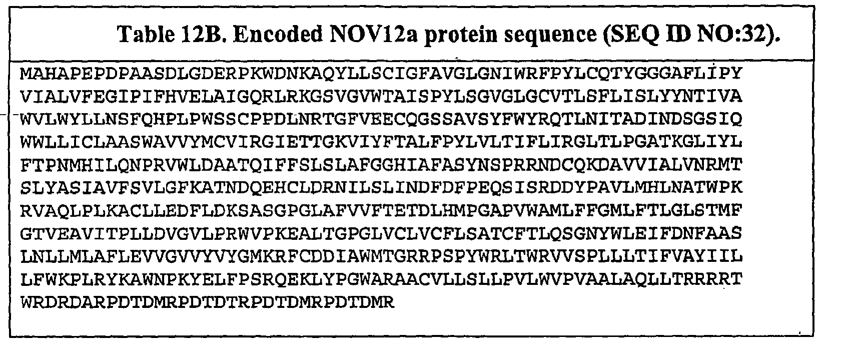

A search of sequence databases reveals that the NOV12a amino acid sequence has 460 of 602 amino acid residues (76%) identical to, and 528 of 602 amino acid residues (87%) similar to, the 615 amino acid residue ptnr:SPTREMBL-ACC:088576 protein from Mus musculus (Mouse) (ORPHAN TRANSPORTER ISOFORM A12). Public amino acid databases include the GenBank databases, SwissProt, PDB and PIR.

NOV12a is expressed in at least Colon and Kidney. This information was derived by determining the tissue sources of the sequences that were included in the invention including but not limited to SeqCalling sources, Public EST sources, Literature sources, and/or RACE sources.

### NOV12b

A disclosed NOV12b nucleic acid of 2039 nucleotides (also referred to as CG57526-02) encoding an orphan receptor-like protein is shown in Table 12C. Putative untranslated regions upstream and/or downstream from the coding region, if any, are underlined, and the start and stop codons are in bold letters.

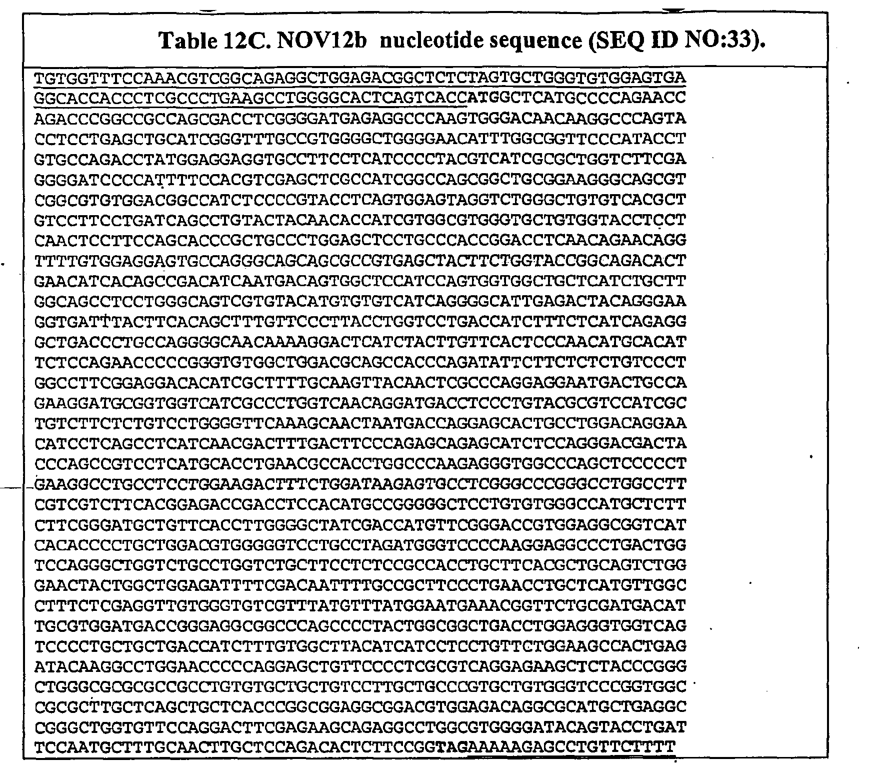

In a search of public sequence databases, the NOV12b nucleic acid sequence, located on chromsome 5 has 1122 of 1396 bases (80%) identical to a gb:GENBANK-ID:AF075263|acc:AF075263.1 mRNA from Mus musculus (Mus musculus orphan transporter isoform A11 (Xtrp2) mRNA, alternatively spliced, complete cds). Public nucleotide databases include all GenBank databases and the GeneSeq patent database.

The disclosed NOV12b polypeptide (SEQ ID NO:34) encoded by SEQ ID NO:33 has 639 amino acid residues and is presented in Table 12D using the one-letter amino acid code. Signal P, Psort and/or Hydropathy results predict that NOV12b has a signal peptide and is likely to be localized to the plasma membrane with a certainty of 0.8000.

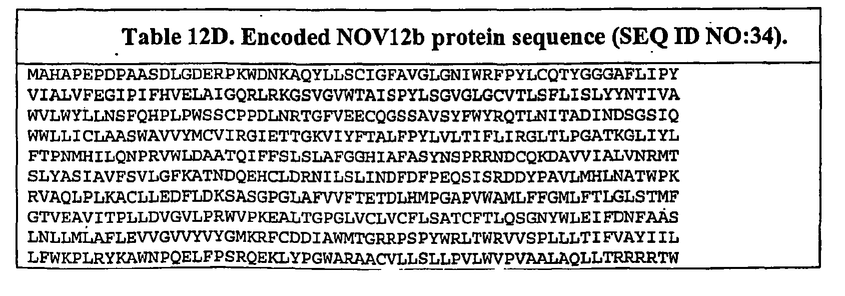

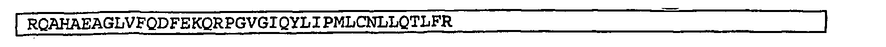

A search of sequence databases reveals that the NOV12b amino acid sequence has 465 of 613 amino acid residues (75%) identical to, and 534 of 613 amino acid residues (87%) similar to, the 615 amino acid residue ptnr:SPTREMBL-ACC:O88576 protein from Mus musculus (Mouse) (ORPHAN TRANSPORTER ISOFORM A12)(. Public amino acid databases include the GenBank databases, SwissProt, PDB and PIR.

NOV12b is expressed in at least Colon and Kidney. This information was derived by determining the tissue sources of the sequences that were included in the invention including but not limited to SeqCalling sources, Public EST sources, Literature sources, and/or RACE sources.

The disclosed NOV12 polypeptide has homology to the amino acid sequences shown in the BLASTP data listed in Table 12E.

| **Table 12E. BLAST results for NOV12** | | | | | |
|---|---|---|---|---|---|
| Gene Index/ Identifier | Protein/ Organism | Length (aa) | Identity (%) | Positives (%) | Expect |
| gi\|16550619\|dbj\|BAB 71018.1\| (AK055798 | unnamed protein product [Homo sapiens] | 628 | 626/631 (99%) | 626/631 (99%) | 0.0 |
| gi\|13347922\|gb\|AAC27 757.1\| (AF075262) | orphan transporter isoform A12 [Mus musculus] | 615 | 460/603 (76%) | 528/603 (87%) | 0.0 |
| gi\|8394204\|ref\|NP 0 58859.1\| (NM 017163) | X transporter protein 2 [Rattus norvegicus] | 615 | 461/604 (76%) | 528/604 (87%) | 0.0 |
| gi\|3347924\|gb\|AAC27 758.1\| (AF075263) | orphan transporter isoform A11 [Mus musculus] | 605 | 454/603 (75%) | 520/603 (85%) | 0.0 |
| gi\|3347926\|gb\|AAC27 759.1\| (AF075264) orphan | orphan transporter isoform B11 [Mus musculus] | 577 | 416/603 (68%) | 478/603 (78%) | 0.0 |

Table 12G lists the domain descriptions from DOMAIN analysis results against NOV12. This indicates that the NOV12 sequence has properties similar to those of other proteins known to contain this domain.

| **Table 12G. Domain Analysis of NOV12** | | |
|---|---|---|
| gnl\|Pfam\|pfam00209, SNF, Sodium:neurotransmitter symporter family. | | |
| | CD-Length = 534 residues, 92.5% aligned | |
| | | Score = 469 bits (1207), Expect = 2e-133 |

A gene family encoding many Na(+)- and Cl(-)-dependent organic solute cotransporters has recently been recognized. Among the cotransporters that have been characterized are those for neurotransmitters, amino acids, and organic osmolytes. The cDNA is 2,354 bp long with an open reading frame of 1,845 bp. The 615 deduced amino sequence shows ROSIT to be most clearly related to two orphan cDNAs of this family isolated from brain. Northern analysis showed the mRNA is normally expressed in renal cortex but not in brain, heart, colon, liver, stomach, or skeletal muscle. Moreover, hypematremic rats displayed a marked increase in mRNA levels in renal cortex, renal outer medulla, and perhaps intestine. Heterologous expression of the cRNA in Xenopus laevis oocytes failed to reveal the function of this gene product when analyzed with isotope fluxes or electrophysiological measurements using a wide variety of organic solutes. ROSIT is likely to be involved in kidney reclamation of an organic osmolyte or osmolyte precursor required for adaptation to hypertonic stress. (See Wasserman et al., Am J Physiol 1994 Oct;267(4 Pt 2):F688-94).

The disclosed NOV12 nucleic acid of the invention encoding a orphan receptor-like protein includes the nucleic acid whose sequence is provided in Table 12A or 12C or a fragment thereof. The invention also includes a mutant or variant nucleic acid any of whose bases may be changed from the corresponding base shown in Table 12A or 12C while still encoding a protein that maintains its orphan receptor-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 20 percent of the bases may be so changed.

The disclosed NOV12 protein of the invention includes the orphan receptor-like protein whose sequence is provided in Table 12B or 12D. The invention also includes a mutant or variant protein any of whose residues may be changed from the corresponding residue shown in Table 12B or 12D while still encoding a protein that maintains its orphan receptor-like activities and physiological functions, or a functional fragment thereof. In the mutant or variant protein, up to about 24 percent of the residues may be so changed.

The invention further encompasses antibodies and antibody fragments, such as F_{ab} or (F_{ab})₂, that bind immunospecifically to any of the proteins of the invention.

The above defined information for this invention suggests that this orphan receptor-like protein (NOV12) may function as a member of a "orphan receptor family". Therefore, the NOV12 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV12 nucleic acids and proteins of the invention are useful in potential therapeutic applications implicated in diseases including but not limited to various pathologies and disorders as indicated below. For example, a cDNA encoding the orphan receptor-like protein (NOV12) may be useful in gene therapy, and the orphan receptor-like protein (NOV12) may be useful when administered to a subject in need thereof. By way of nonlimiting example, the compositions of the present invention will have efficacy for treatment of patients suffering from cancer, trauma, regeneration (in vitro and in vivo), viral/bacterial/parasitic infections, Hirschsprung's disease , Crohn's Disease, appendicitis, diabetes, autoimmune disease, renal artery stenosis, interstitial nephritis, glomerulonephritis, polycystic kidney disease, systemic lupus erythematosus, renal tubular acidosis, IgA nephropathy, hypercalceimia, Lesch-Nyhan syndrome, or other pathologies or conditions. The NOV12 nucleic acid encoding the orphan receptor-like protein of the invention, or fragments thereof, may further be useful in diagnostic applications, wherein the presence or amount of the nucleic acid or the protein are to be assessed.

NOV12 nucleic acids and polypeptides are further useful in the generation of antibodies that bind immuno-specifically to the novel NOV12 substances for use in therapeutic or diagnostic methods. These antibodies may be generated according to methods known in the art, using prediction from hydrophobicity charts, as described in the "Anti-NOVX Antibodies" section below. The disclosed NOV12 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV13

A disclosed NOV13 nucleic acid of 1748 nucleotides (also referred to as CG-57570-01) encoding a cation transporter-like protein is shown in Table 13A. Putative untranslated regions upstream and/or downstream from the coding region, if any, are underlined, and the start and stop codons are in bold letters.

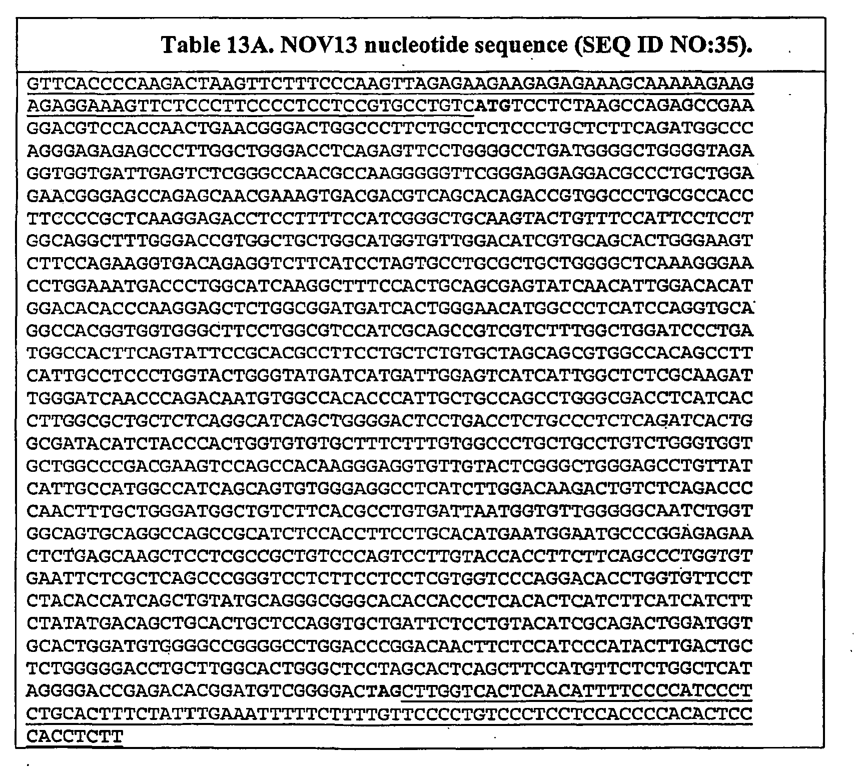

In a search of public sequence databases, the NOV13 nucleic acid sequence, located on chromsome 1 has 440 of 674 bases (65%) identical to a gb:GENBANK-ID:AK021925|acc:AK021925.1 mRNA from Homo sapiens (Homo sapiens cDNA FLJ11863 fis, clone HEMBA1006926). Public nucleotide databases include all GenBank databases and the GeneSeq patent database.

The disclosed NOV13 polypeptide (SEQ ID NO:36) encoded by SEQ ID NO:35 has 517 amino acid residues and is presented in Table 13B using the one-letter amino acid code. Signal P, Psort and/or Hydropathy results predict that NOV13 has a signal peptide and is likely to be localized at the plasma membrane with a certainty of 0.6000:

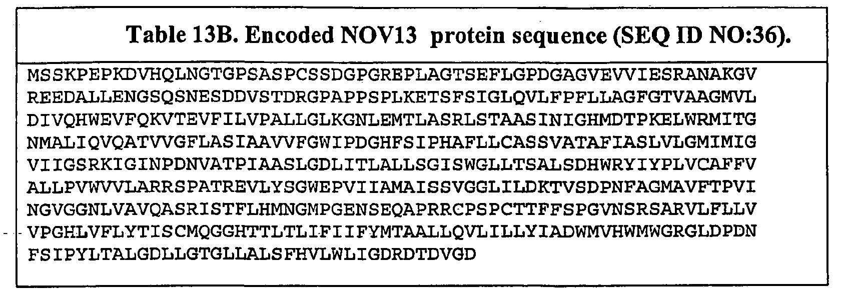

A search of sequence databases reveals that the NOV13 amino acid sequence has 307 of 456 amino acid residues (67%) identical to, and 373 of 456 amino acid residues (81 %) similar to, the 490 amino acid residue ptnr:TREMBLNEW-ACC:CAB66762 protein from Homo sapiens (Human) (HYPOTHETICAL 53.3 KDA PROTEIN). Public amino acid databases include the GenBank databases, SwissProt, PDB and PIR.

NOV13 is expressed in at least Liver, Pituitary Gland, Heart, Uterus, and B-cells. This information was derived by determining the tissue sources of the sequences that were included in the invention including but not limited to SeqCalling sources, Public EST sources, Literature sources, and/or RACE sources.

The disclosed NOV13 polypeptide has homology to the amino acid sequences shown in the BLASTP data listed in Table 13C.

| **Table 13C. BLAST results for NOV13** | | | | | |
|---|---|---|---|---|---|
| Gene Index/ Identifier | Protein/ Organism | Length (aa) | Identity (%) | Positives (%) | Expect |
| gi\|14149819\|ref\|NP 115524.1 (NM_032148) | hypothetical protein DKFZp434K0427 [Homo sapiens] | 490 | 305/457 (66%) | 372/457 (80%) | e-166 |
| gi\|12053165\|emb\|CAB 66762.11 (AL136828) | hypothetical protein [Homo sapiens] | 490 | 305/457 (66%) | 372/457 (80%), | e-166 |
| gi\|5079232\|gb\|AAH1 1108.1\|AAH11108 (BC011108) | protein for MGC:18986) [Mus musculus] | 488 | 235/438 (53%) | 306/438 (69%), | e-112 |
| gi\|14290540\|gb\|AAH0 9039.1\|AAH09039 (BC009039 | Similar to hypothetical protein FLJ20473 [Homo sapiens] | 507 | 237/438 (54%) | 306/438 (69%), | e-111 |
| gi\|12833620\|dbj\|BAB 22598.1\| (AK003140) | homolog to CDNA FLJ12718 FIS, CLONE NT2RP1001286-puta tive [Mus musculus] | 462 | 230/445 (51%) | 304/445 (67%), | e-109 |

Table 13D lists the domain descriptions from DOMAIN analysis results against NOV13. This indicates that the NOV13 sequence has properties similar to those of other proteins known to contain this domain.

| **Table 13D. Domain Analysis of NOV13** |
|---|
| gnl\|Pfam\|pfam01769, MgtE, Divalent cation transporter. This region is the integral membrane part of the eubacterial MgtE family of magnesium transporters. Related regions are found also in archaebacterial and eukaryotic proteins. All the archaebacterial and eukaryotic examples have two copies of the region. This suggests that the eubacterial examples may act as dimers. Members of this family probably transport Mg2+ or other divalent cations into the cell. The alignment contains two highly conserved Ds that may be involved in cation binding (Bateman A unpubl.) |
| CD-Length = 131. residues, 99.2% aligned |
| Score = 66.6 bits (161), Expect = 3e-12 |

A gene family encoding many Na(+)- and Cl(-)-dependent organic solute cotransporters has recently been recognized. Among the cotransporters that have been characterized are those for neurotransmitters, amino acids, and organic osmolytes. The cDNA is 2,354 bp long with an open reading frame of 1,845 bp. The 615 deduced amino sequence shows ROSIT to be most clearly related to two orphan cDNAs of this family isolated from brain. Northern analysis showed the mRNA is normally expressed in renal cortex but not in brain, heart, colon, liver, stomach, or skeletal muscle. Moreover, hypernatremic rats displayed a marked increase in mRNA levels in renal cortex, renal outer medulla, and perhaps intestine. Heterologous expression of the cRNA in Xenopus laevis oocytes failed to reveal the function of this gene product when analyzed with isotope fluxes or electrophysiological measurements using a wide variety of organic solutes. ROSIT is likely to be involved in kidney reclamation of an organic osmolyte or osmolyte precursor required for adaptation to hypertonic stress. (See Wasserman et al., Am J Physiol 1994 Oct;267(4 Pt 2):F688-94).

Nramp1 regulates macrophage activation in infectious and autoimmune diseases. Nramp2 controls anaemia. Both are divalent cation (Fe(2+), Zn(2+), and Mn(2+)) transporters; Nramp2 a symporter of H(+) and metal ions, Nramp1 a H(+)/divalent cation antiporter. This provides a model for metal ion homeostasis in macrophages. Nramp2, localised to early endosomes, delivers extracellularly acquired divalent cations into the cytosol. Nrampl, localised to late endosomes/lysosomes, delivers divalent cations from the cytosol to phagolysosomes. Here, Fe(2+) generates antimicrobial hydroxyl radicals via the Fenton reaction. Zn(2+) and Mn(2+) may also influence endosomal metalloprotease activity and phagolysosome fusion. The many cellular functions dependent on metal ions as cofactors may explain the multiple pleiotropic effects of Nrampl, and its complex roles in infectious and autoimmune disease. (See Blackwell et al., Microbes Infect 2000 Mar;2(3):317-21).

Mutations in the gene encoding the renal epithelial K(+) channel ROMK1 (Kir 1.1)is one of the causes for Bartter's syndrome, an autosomal recessive disease. It results in defective renal tubular transport in the thick ascending limb of the loop of Henle that leads to hypokalemic metabolic alkalosis and loss of salt. Two novel ROMK1 mutations, L220F/A156V, have been described recently in a compound heterozygote patient demonstrating typical manifestations of Bartter's syndrome. Functional properties of these ROMK1 mutants were studied by coexpressing in Xenopus oocytes and by means of double electrode voltage clamp experiments. When both ROMK1 mutants were coexpressed no K(+) conductance could be detected. The same was found in oocytes expressing A156V-ROMKl only or coexpressing wild type (wt) ROMK1 together with A156V-ROMK1. In contrast, K(+) conductances were indistinguishable from that of wt-ROMK1 when L220F-ROMK1 was expressed alone. Activation of protein kinase C signaling inhibited the conductance in both L220F-ROMK1 and wt-ROMK1 expressing oocytes. These effects were not seen in A156V-ROMKl expressing oocytes.

The disclosed NOV13 nucleic acid of the invention encoding a cation transporter-like protein includes the nucleic acid whose sequence is provided in Table 13A or a fragment thereof. The invention also includes a mutant or variant nucleic acid any of whose bases may be changed from the corresponding base shown in Table 13A while still encoding a protein that maintains its cation transporter-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 35 percent of the bases may be so changed.

The disclosed NOV13 protein of the invention includes the cation transporter-like protein whose sequence is provided in Table 13B. The invention also includes a mutant or variant protein any of whose residues may be changed from the corresponding residue shown in Table B while still encoding a protein that maintains its cation transporter-like activities and physiological functions, or a functional fragment thereof In the mutant or variant protein, up to about 33 percent of the residues may be so changed.

The invention further encompasses antibodies and antibody fragments, such as F_{ab} or (F_{ab})₂, that bind immunospecifically to any of the proteins of the invention.

The above defined information for this invention suggests that this cation transporter-like protein (NOV13) may function as a member of a "cation transporter family". Therefore, the NOV13 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV13 nucleic acids and proteins of the invention are useful in potential therapeutic applications implicated in diseases including but not limited to various pathologies and disorders as indicated below. For example, a cDNA encoding the cation transporter-like protein (NOV13) may be useful in gene therapy, and the cation transporter-like protein (NOV13) may be useful when administered to a subject in need thereof. By way of nonlimiting example, the compositions of the present invention will have efficacy for treatment of patients suffering from cancer,trauma, regeneration (in vitro and in vivo), viral/bacterial/parasitic infections, cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, transplantation, endometriosis, fertility, Von Hippel-Lindau (VHL) syndrome, cirrhosis, endocrine dysfunctions, diabetes, obesity, growth and reproductive disorders, or other pathologies or conditions. The NOV13 nucleic acid encoding the cation transporter-like protein of the invention, or fragments thereof, may further be useful in diagnostic applications, wherein the presence or amount of the nucleic acid or the protein are to be assessed.

NOV13 nucleic acids and polypeptides are further useful in the generation of antibodies that bind immuno-specifically to the novel NOV13 substances for use in therapeutic or diagnostic methods. These antibodies may be generated according to methods known in the art, using prediction from hydrophobicity charts, as described in the "Anti-NOVX Antibodies" section below. The disclosed NOV13 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV14

A disclosed NOV14 nucleic acid of 5175 nucleotides (also referred to as CG57593-01) encoding a ABC transporter-like protein is shown in Table 14A. Putative untranslated regions upstream and/or downstream from the coding region, if any, are underlined, and the start and stop codons are in bold letters.

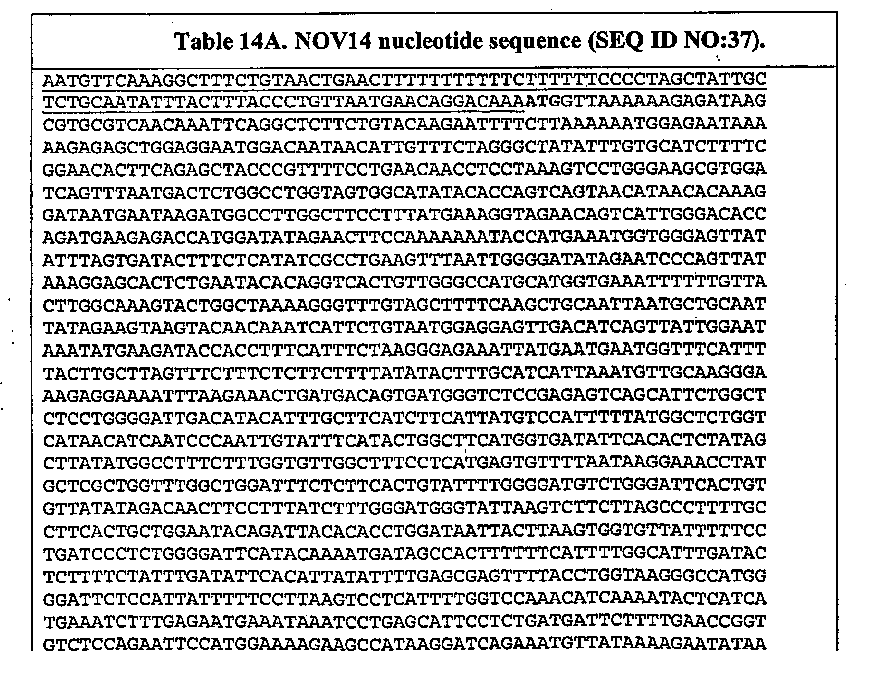

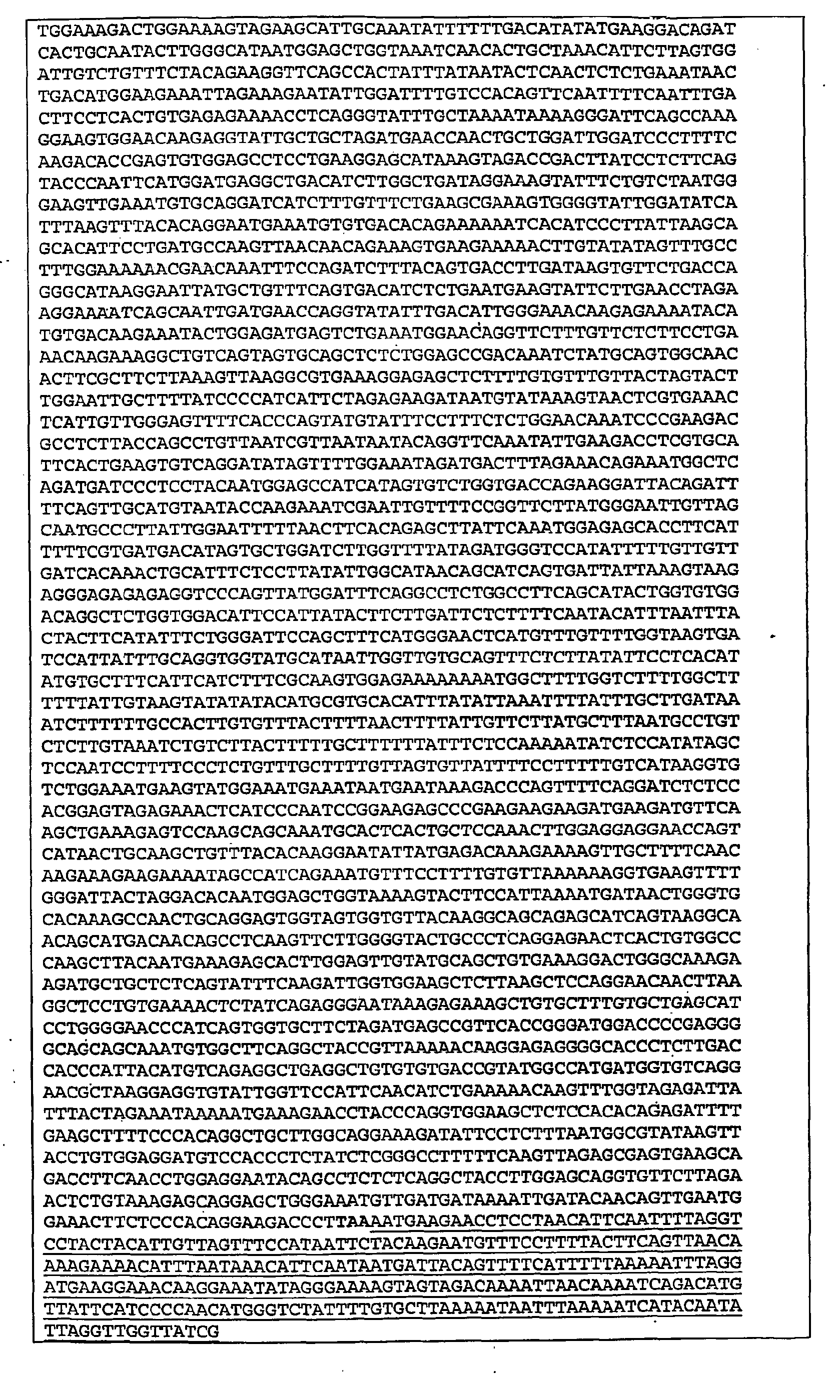

In a search of public sequence databases, the NOV14 nucleic acid sequence, located on chromsome 17 has 1737 of 2520 bases (68%) identical to a gb:GENBANK-ID:AB020629/acc:AB020629.1 mRNA from Homo sapiens (Homo sapiens mRNA for KIAA0822 protein, complete cds). Public nucleotide databases include all GenBank databases and the GeneSeq patent database.

The disclosed NOV14 polypeptide (SEQ ID NO:38) encoded by SEQ ID NO:37 has 1595 amino acid residues and is presented in Table 14 using the one-letter amino acid code. Signal P, Psort and/or Hydropathy results predict that NOV14 has a signal peptide and is likely to be localized at the plasma membrane with a certainty of 0.8000. The most likely cleavage site for a NOV14 peptide is between amino acids 52 and 53.

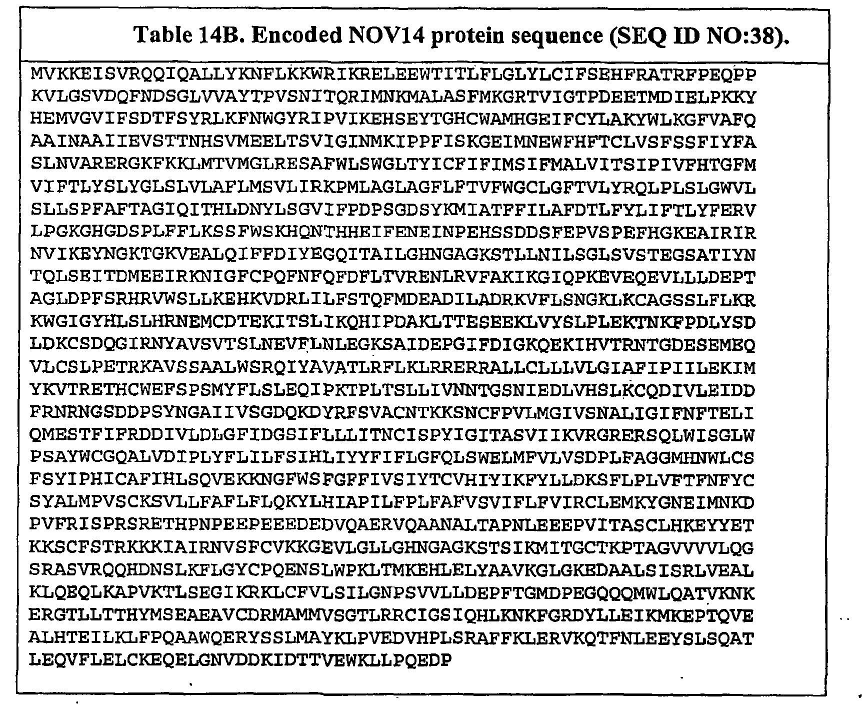

A search of sequence databases reveals that the NOV14 amino acid sequence has 747 of 1321 amino acid residues (56%) identical to, and 951 of 1321 amino acid residues (71%) similar to, the 1581 amino acid residue ptnr:SPTREMBL-ACC:094911 protein from Homo sapiens (Human) (KIAA0822 PROTEIN). Public amino acid databases include the GenBank databases, SwissProt, PDB and PIR.

NOV14 is expressed in at least epidermis. This information was derived by determining the tissue sources of the sequences that were included in the invention including but not limited to SeqCalling sources, Public EST sources, Literature sources, and/or RACE sources.

The disclosed NOV14 polypeptide has homology to the amino acid sequences shown in the BLASTP data listed in Table 14C.

| **Table 14C. BLAST results for NOV14** | | | | | |
|---|---|---|---|---|---|
| Gene Index/ Identifier | Protein/ Organism | Length (aa) | Identity (%) | Positives (%) | Expect |
| gi\|18587267\|ref\|XP 085647.1\| (XM_085647 | ATP-binding cassette, sub- family A (ABC1), member 10 [Homo sapiens] | 1543 | 1391/1563 (88%) | 1423/1563 (90%) | 0.0 |
| gi\|17933760\|ref\|NP 525021.1\| (NM_080282) | ATP-binding cassette, sub- family A (ABC1), member 10 [Homo sapiens] | 1543 | 1387/1563 (88%) | 1420/1563 (90%), | 0.0 |
| gi\|6005701\|ref\|NP 0 09099.1\| (NM_007168) | ATP-binding cassette, sub- family A (ABC1), member 8 [Homo sapiens] | 1581 | 943/1604 (58%) | 1183/1604 (72%) | 0.0 |
| gi\|17933764\|ref\|NP 525023.1\| (NM_080284) | ATP-binding cassette, sub- family A (ABC1), member 6 [Homo sapiens] | 1617 | 951/1659 (57%) | 1173/1659 (70%), | 0.0 |
| gi\|18587273\|ref\|XP 085646.1\| (XM_085646) | ATP-binding cassette, sub- family A (ABC1), member 9 [Homo sapiens] | 1624 | 940/1653 (56%) | 1189/1653 (71%), | 0.0 |

Table 14D lists the domain descriptions from DOMAIN analysis results against NOV 14. This indicates that the NOV14 sequence has properties similar to those of other proteins known to contain this domain.

| **Table 14D. Domain Analysis of NOV14** |
|---|
| gnl Pfam pfam00005, ABC_tran, ABC transporter. ABC transporters for a large family of proteins responsible for translocation of a variety of compounds across biological membranes. ABC transporters are the largest family of proteins in many completely sequenced bacteria. ABC transporters are composed of two copies of this domain and two copies of a transmembrane domain pfam00664. These four domains may belong to a single polypeptide, or belong in different polypeptide chains. |
| CD-Length = 183 residues, 98.4% aligned |
| Score = 132 bits (331), Expect = 2e-31 |

| **Table 14E. Domain Analysis of NOV14** | | |
|---|---|---|
| gnl\|Smart\|smart00382, AAA, ATPases associated with a variety of cellular activities; AAA - ATPases associated with a variety of cellular activities. This profile/alignment only detects a fraction of this vast family. The poorly conserved N-terminal helix is missing from the alignment. | | |
| | CD-Length = 151 residues, 98.7% aligned | |
| | | Score = 49.3 bits (116), Expect = 2e-06 |

ABCAI, a member of the ATP binding cassette family, mediates the efflux of excess cellular lipid to HDL and is defective in Tangier disease. The apolipoprotein acceptor specificity for lipid efflux by ABCAI was examined in stably transfected Hela cells, expressing a human ABCAI-GFP fusion protein. ApoA-I and all of the other exchangeable apolipoproteins tested (apoA-II, apoA-IV, apoC-I, apoC-II, apoC-III, apoE) showed greater than a threefold increase in cholesterol and phospholipid efflux from ABCAI-GFP transfected cells compared to control cells. Expression of ABCAI in Hela cells also resulted in a marked increase in specific binding of both apoA-I (Kd = 0.60 ?g/mL) and apoA-II (Kd = 0.58 ?g/mL) to a common binding site. In summary, ABCAI-mediated cellular binding of apolipoproteins and lipid efflux is not specific for only apoA-I but can also occur with other apolipoproteins that contain multiple amphipathic helical domains. (See Remaley et al., Biochem Biophys Res Commun 2001 Jan 26;280(3):818-823).

The molecular mechanisms regulating the amount of dietary cholesterol retained in the body, as well as the body's ability to exclude selectively other dietary sterols, are poorly understood. An average western diet will contain about 250-500 mg of dietary cholesterol and about 200-400 mg of non-cholesterol sterols. About 50-60% of the dietary cholesterol is absorbed and retained by the normal human body, but less than 1% of the non-cholesterol sterols are retained. Thus, there exists a subtle mechanism that allows the body to distinguish between cholesterol and non-cholesterol sterols. In sitosterolemia, a rare autosomal recessive disorder, affected individuals hyperabsorb not only cholesterol but also all other sterols, including plant and shellfish sterols from the intestine. The major plant sterol species is sitosterol; hence the name of the disorder. Consequently, patients with this disease have very high levels of plant sterols in the plasma and develop tendon and tuberous xanthomas, accelerated atherosclerosis, and premature coronary artery disease. The STSL locus was mapped to human chromosome 2p21 (ref. 4) and was localized it to a region of less than 2 cM bounded by markers D2S2294 and D2S2291. A new member of the ABC transporter family, ABCG5, is mutant in nine unrelated sitosterolemia patients. (See Lee et al., Nat Genet 2001 Jan;27(1):79-83).

Pseudoxanthoma elasticum (PXE) is an inherited systemic disorder of connective tissue, characterized by progressive calcification of the elastic fibers in the eye, the skin, and the cardiovascular system. The PXE locus has been mapped to chromosome 16p13.1, and was recently further refined to a 500 kb-region, containing four candidate genes : MRP1 (ABCC1), MRP6 (ABCC6), pM5, and two copies of an unknown gene, the later subsequently found to be identical to the gene encoding the Nuclear Pore Interacting Protein (NPIP). In a comprehensive mutational screening, the entire coding region of the pM5, MRP1, and NPIP genes were analyzed in 7 patients affected with pseudoxanthoma elasticum, but failed to find evidence of disease-causing defects in any of these three genes. Five synonymous (G232G, P395P, A862A, G912G, D1106D), and five non synonymous (V404I, N458K, D490N, F1141I, G1195R) polymorphisms were found in the pM5 gene,

Mutations in the gene encoding ABCR (ABCA4), a photoreceptor-specific ATP-binding cassette (ABC) transporter, are responsible for autosomal recessive Stargardt disease (STGD), an early onset macular degeneration, and some forms of autosomal recessive cone-rod dystrophy and autosomal recessive retinitis pigmentosa. Heterozygosity for ABCA4 mutations may also represent a risk factor for age-related macular degeneration (AMD). An ongoing challenge in the analysis of ABCA4-based retinopathies arises from the observation that most of the ABCA4 sequence variants identified so far are missense mutations that are rare in both patient and control populations. With the current sample size of most sequence variants, one cannot determine statistically whether a particular sequence variant is pathogenic or neutral. A related challenge is to determine the degree to which each pathogenic variant impairs ABCR function, as genotype-phenotype analyses indicate that age of onset and disease severity correlate with different ABCA4 alleles. To address these questions, a functional analysis of human ABCR and its variants was performed. These experiments reveal a wide spectrum of biochemical defects in these variants and provide insight into the transport mechanism of ABCR. (See Sun et al., Nat Genet 2000 Oct;26(2):242-6).

The disclosed NOV14 nucleic acid of the invention encoding a ABC transporter-like protein includes the nucleic acid whose sequence is provided in Table 14A or a fragment thereof. The invention also includes a mutant or variant nucleic acid any of whose bases may be changed from the corresponding base shown in Table 14A while still encoding a protein that maintains its ABC transporter-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 32 percent of the bases may be so changed.

The disclosed NOV14 protein of the invention includes the ABC transporter-like protein whose sequence is provided in Table 14B. The invention also includes a mutant or variant protein any of whose residues maybe changed from the corresponding residue shown in Table 14B while still encoding a protein that maintains its ABC transporter-like activities and physiological functions, or a functional fragment thereof. In the mutant or variant protein, up to about 44 percent of the residues may be so changed.

The invention further encompasses antibodies and antibody fragments, such as F_{ab} or (T_{ab})₂, that bind immunospecifically to any of the proteins of the invention.

The above defined information for this invention suggests that this ABC transporter-like protein (NOV14) may function as a member of a "ABC transporter family". Therefore, the NOV14 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV14 nucleic acids and proteins of the invention are useful in potential therapeutic applications implicated in diseases including but not limited to various pathologies and disorders as indicated below. For example, a cDNA encoding the ABC transporter-like protein (NOV14) may be useful in gene therapy, and the ABC transporter-like protein (NOV14) may be useful when administered to a subject in need thereof. By way of nonlimiting example, the compositions of the present invention will have efficacy for treatment of patients suffering from cancer, trauma, regeneration (in vitro and in vivo), viral/bacterial/parasitic infections, psoriasis, actinic keratosis, tuberous sclerosis, acne, hair growth/loss, allopecia, pigmentation disorders, endocrine disorders, or other pathologies or conditions. The NOV14 nucleic acid encoding the ABC transporter-like protein of the invention, or fragments thereof, may further be useful in diagnostic applications, wherein the presence or amount of the nucleic acid or the protein are to be assessed.

NOV14 nucleic acids and polypeptides are further useful in the generation of antibodies that bind immuno-specifically to the novel NOV14 substances for use in therapeutic or diagnostic methods. These antibodies may be generated according to methods known in the art, using prediction from hydrophobicity charts, as described in the "Anti-NOVX Antibodies" section below. The disclosed NOV14 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV15

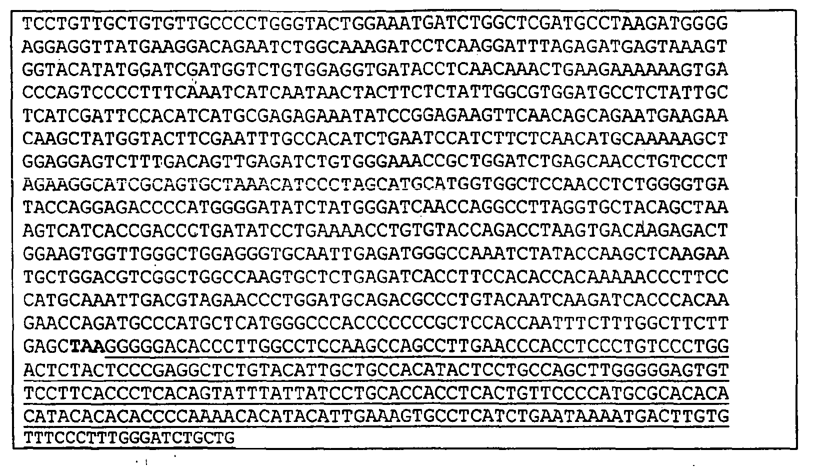

In a search of public sequence databases, the NOV15 nucleic acid sequence, located on chromsome 12 has 2038 of 2038 bases (100%) identical to a gb:GENBANK-ID:HSDKRNA|acc:X62535.1 mRNA from Homo sapiens (H.sapiens mRNA for diacylglycerol kinase). Public nucleotide databases include all GenBank databases and the GeneSeq patent database.

The disclosed NOV15 polypeptide (SEQ ID NO:40) encoded by SEQ ID NO:39 has 727 amino acid residues and is presented in Table 15B using the one-letter amino acid code. Signal P, Psort and/or Hydropathy results predict that NOV15 has no signal peptide and is likely to be localized in the nucleus with a certainty of 0.3000.

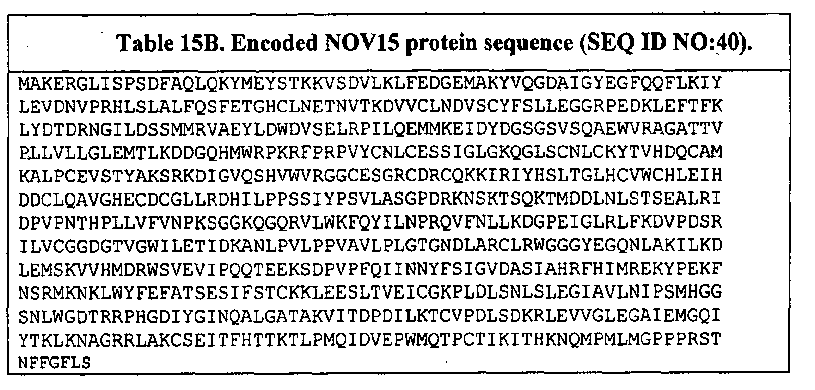

A search of sequence databases reveals that the NOV15 amino acid sequence has 727 of 735 amino acid residues (98%) identical to, and 727 of 735 amino acid residues (98%) similar to, the 735 amino acid residue ptnr:SWISSNEW-ACC:P23743 protein from Homo sapiens (Human) (DIACYLGLYCEROL KINASE, ALPHA (EC 2.7.1.107) (DIGLYCERIDE KINASE) (DGK- ALPHA) (DAG KINASE ALPHA) (80 KDA DIACYLGLYCEROL KINASE)). Public amino acid databases include the GenBank databases, SwissProt, PDB and PIR.

NOV15 is expressed in at least Aorta, Appendix, Ascending Colon, Bone, Bone Marrow, Brain, Bronchus, Cartilage, Cervix, Colon, Coronary Artery, Dermis, Heart, Hippocampus, Kidney, Left cerebellum, Liver, Lung, Lymph node, Lymphoid tissue, Mammary gland/Breast, Ovary, Pancreas, Parotid Salivary glands, Peripheral Blood, Pituitary Gland, Placenta, Prostate, Respiratory Bronchiole, Small Intestine, Spleen, Stomach, Substantia Nigra, Synovium/Synovial membrane, Temporal Lobe, Testis, Thymus, Tonsils, Trachea, Umbilical Vein, Uterus, Vein, Whole Organism. Expression information was derived from the tissue sources of the sequences that were included in the derivation of the sequence of CG57652-01. The sequence is predicted to be expressed in the following tissues because of the expression pattern of (GENBANK-ID: gb:GENBANK-ID:HSDKRNA|acc:X62535.1) a closely related H.sapiens mRNA for diacylglycerol kinase homolog in species Homo sapiens: lymphocytes, oligodendroglial cells, and neutrophils.. This information was derived by determining the tissue sources of the sequences that were included in the invention including but not limited to SeqCalling sources, Public EST sources, Literature sources, and/or RACE sources.

The disclosed NOV15 polypeptide has homology to the amino acid sequences shown in the BLASTP data listed in Table 15C.

| **Table 15C. BLAST results for NOV15** | | | | | |
|---|---|---|---|---|---|
| Gene Index/ Identifier | Protein/ Organism | Length (aa) | Identity (%) | Positives (%) | Expect |
| gi\|11415024\|ref\|NP 001336.1 (NM_001345) | diacylglycerol kinase, alpha (80kD) [Homo sapiens] | 735 | 727/735 (98%) | 727/735 (98%) | 0.0 |
| gi\|125323\|sp\|P20192 \|KDGA PIG | Diacylglycerol kinase, alpha (Diglyceride kinase) (DGK-alpha) (DAG kinase alpha) (80 kDa diacylglycerol kinase) | 734 | 679/734 (92%) | 703/734 (95%), | 0.0 |
| gi\|13879470\|gb\|AAH0\| 6713.1\|AAH06713 (BC006713) | diacylglycerol kinase, alpha (80 kDa) [Mus musculus] | 730 | 597/736 (81%) | 647/736 (87%), | 0.0 |
| gi\|18158459\|ref\|NP 542965.1 (NM_080787) | diacylglycerol kinase, alpha (80kD) [Rattus norvegicus] | 727 | 600/736 (81%) | 646/736 (87%), | 0.0 |
| gi\|7949033\|ref\|NP 0 58091.1\| (NM_016811) | diacylglycerol kinase, alpha (80 kDa) [Mus musculus] | 730 | 594/736 (80%) | 645/736 (86%) | 0.0 |

Tables 15D-E list the domain descriptions from DOMAIN analysis results against NOV15. This indicates that the NOV15 sequence has properties similar to those of other proteins known to contain this domain.

| **Table 15D. Domain Analysis of NOV15** |
|---|
| gnl\|Pfam\|pfam00609, DAGKa, Diacylglycerol kinase accessory domain (presumed). Diacylglycerol (DAG) is a second messenger that acts as a protein kinase C activator |
| CD-Length = 170 residues, 100.0% aligned |
| Score = 275 bits (702), Expect = 9e-75 |

| **Table 15E. Domain Analysis of NOV15** |
|---|
| gn1\|Smart\|smart00109, C1, Protein kinase C conserved region 1 (C1) domains (Cysteine-rich domains); Some bind phorbol esters and diacylglycerol. Some bind RasGTP. Zinc-binding domains. |
| CD-Length = 50 residues, 96.0% aligned |
| Score = 53.5 bits (127), Expect = 4e-08 |

Diacylglycerol (DAG) functions in intracellular signaling pathways as an allosteric activator of protein kinase C (PKC; see 600448). In addition, DAG appears to play a role in regulating RAS (see 190020) and RHO (see 165370) family proteins by activating the guanine nucleotide exchange factors VAV (164875) and RASGRP1 (603962). DAG also occupies a central position in the synthesis of major phospholipids and triacylglycerols. Thus, to maintain cellular homeostasis, intracellular DAG levels must be tightly regulated (Topham and Prescott, 1999). DAG kinases (DGKs or DAGKs; EC 2.7.1.107) phosphorylate DAG to phosphatidic acid, thus removing DAG. In intracellular signaling pathways, DAGK can be viewed as a modulator that competes with PKC for the second messenger DAG. Schaap et al. (1990) purified and characterized an 86-kD DAGK from normal human white blood cells. Based on partial amino acid sequences of the purified enzyme, primers were designed that permitted cloning of the human DAGK cDNA by use of PCR. The sequence demonstrated that it is the human homolog of the porcine gene. The human DAGK cDNA, transfected into COS-7 cells, resulted in a 6- to 7-fold increase in enzyme activity.

Several mammalian isozymes of DAGK have been identified. The isoform described by Schaap et al. (1990) has been designated DGK-alpha or DAGK1. Topham and Prescott (1999) stated that all DGKs have a conserved catalytic domain and at least 2 cysteine-rich regions homologous to the C1A and C1B motifs ofPKCs. Most DGKs have structural motifs that are likely to play regulatory roles, and these motifs form the basis for dividing the DGKs into 5 subtypes. Type I DGKs, such as DGK-alpha, -beta (604070), and -gamma (601854), have calcium-binding EF-hand motifs at their N termini. DGK-delta (601826) and DKG-eta (604071) contain N-terminal pleckstrin homology (PH) domains and are defined as type II. DGK-epsilon (601440) contains no identifiable regulatory domains and is a type III DGK. The defining characteristic of type IV isozymes, such as DGK-zeta (601441) and -iota (604072), is that they have C-terminal ankyrin repeats. Group V is exemplified by DGK-theta (601207), which contains 3 cysteine-rich domains and a PH domain.

Pilz et al. (1995) pointed to the growing evidence to support some form of light-activated phosphoinositide signal transduction pathway in the mammalian retina. Although this pathway had no obvious role in mammalian phototransduction, mutations in this pathway were known to cause retinal degeneration in Drosophila. For example, the 'retinal degeneration A'mutant in Drosophila is caused by an alteration in the eye-specific DAGK gene. In an effort to consider genes mutated in Drosophila as candidates for mammalian eye disease, Pilz et al. (1995) determined the map position of 3 DAGK genes in the mouse. They localized the mouse homolog of DAGK1 to chromosome 10 by linkage analysis. By Southern blot analysis of human-hamster somatic cell hybrid DNA, Hart et al. (1994) assigned the DAGK gene to chromosome 12. Hart et al. (1994) further localized the gene to 12q13.3 by fluorescence in situ hybridization.

The disclosed NOV15 nucleic acid of the invention encoding a diacylglycerol kinase alpha-like protein includes the nucleic acid whose sequence is provided in Table 15A or a fragment thereof. The invention also includes a mutant or variant nucleic acid any of whose, bases may be changed from the corresponding base shown in Table 15A while still encoding a protein that maintains its diacylglycerol kinase alpha-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 0 percent of the bases may be so changed.

The disclosed NOV15 protein of the invention includes the diacylglycerol kinase alpha-like protein whose sequence is provided in Table 15B. The invention also includes a mutant or variant protein any of whose residues may be changed from the corresponding residue shown in Table 15B while still encoding a protein that maintains its diacylglycerol kinase alpha-like activities and physiological functions, or a functional fragment thereof. In the mutant or variant protein, up to about 2 percent of the residues may be so changed.

The invention further encompasses antibodies and antibody fragments, such as F_{ab} or (F_{ab})₂, that bind immunospecifically to any of the proteins of the invention.

The above defined information for this invention suggests that this diacylglycerol kinase alpha-like protein (NOV15) may function as a member of a "diacylglycerol kinase alpha family". Therefore, the NOV15 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV15 nucleic acids and proteins of the invention are useful in potential therapeutic applications implicated in diseases including but not limited to various pathologies and disorders as indicated below. For example, a cDNA encoding the diacylglycerol kinase alpha-like protein (NOV15) may be useful in gene therapy, and the diacylglycerol kinase alpha-like protein (NOV15) may be useful when administered to a subject in need thereof. By way of nonlimiting example, the compositions of the present invention will have efficacy for treatment of patients suffering from osteoporosis, hypercalceimia, arthritis, ankylosing spondylitis, scoliosis, hemophilia, hypercoagulation, idiopathic thrombocytopenic purpura, autoimmune disease, allergies, asthma, immunodeficiencies, transplantation, graft versus host disease, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, tendonitis, fertility, atherosclerosis,
aneurysm, hypertension, fibromuscular dysplasia, scleroderma, myocardial infarction, embolism, cardiovascular disorders, bypass surgery, cardiomyopathy, atherosclerosis, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, renal artery stenosis, interstitial nephritis, glomerulonephritis, polycystic kidney disease; renal tubular acidosis, IgA nephropathy, cirrhosis, systemic lupus erythematosus, emphysema, ARDS, lymphedema , endometriosis, diabetes, pancreatitis, obesity, anemia, ataxia-telangiectasia, endocrine dysfunctions, growth and reproductive disorders, inflammatory bowel disease, diverticular disease, ulcers, tonsillitis, ARDS, anemia , bleeding disorders, or other pathologies or conditions. The NOV15 nucleic acid encoding the diacylglycerol kinase alpha-like protein of the invention, or fragments thereof, may further be useful in diagnostic applications, wherein the presence or amount of the nucleic acid or the protein are to be assessed.

NOV15 nucleic acids and polypeptides are further useful in the generation of antibodies that bind immuno-specifically to the novel NOV15 substances for use in therapeutic or diagnostic methods. These antibodies may be generated according to methods known in the art, using prediction from hydrophobicity charts, as described in the "Anti-NOVX Antibodies" section below. The disclosed NOV15 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV16

A disclosed NOV16 nucleic acid of 3904 nucleotides (also referred to as CG57562-01) encoding a cation-transporting ATPase-like protein is shown in Table 16A. Putative untranslated regions upstream and/or downstream from the coding region, if any, are underlined, and the start and stop codons are in bold letters.

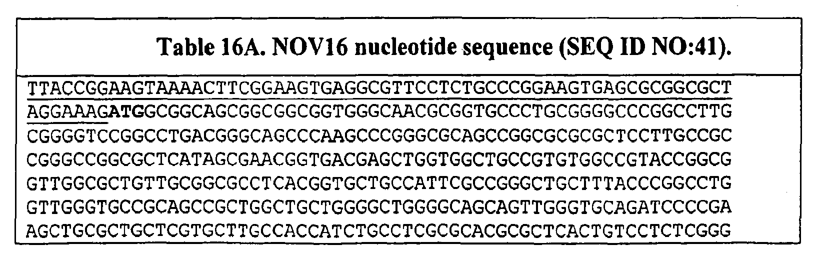

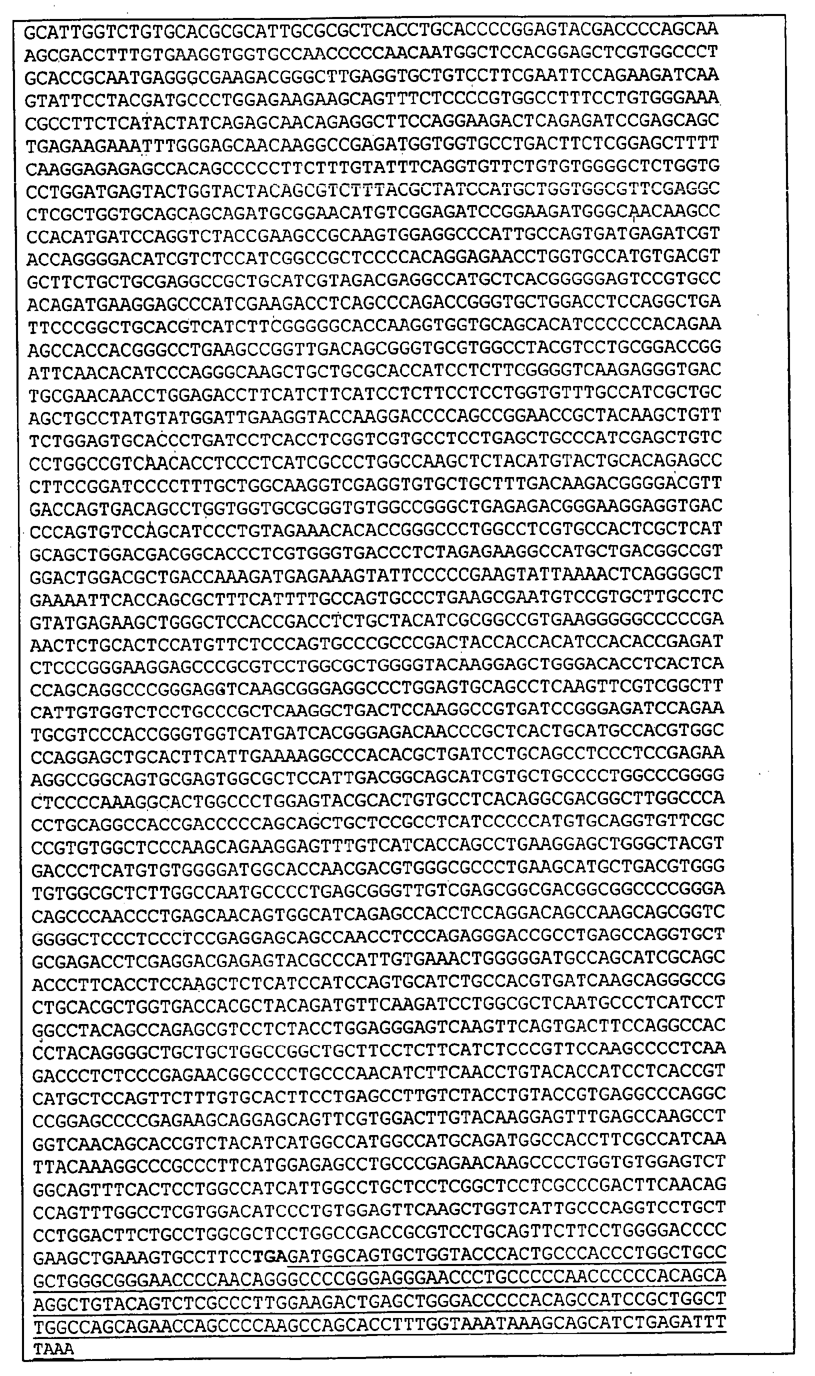

In a search of public sequence databases, the NOV16 nucleic acid sequence, located on chromsome 19 has 3442 of 3442 bases (100%) identical to a gb:GENBANK-ID:AF288687|acc:AF288687.1 mRNA from Homo sapiens (Homo sapiens CGI-152 protein mRNA, complete cds). Public nucleotide databases include all GenBank databases and the GeneSeq patent database.

The disclosed NOV16 polypeptide (SEQ ID NO:42) encoded by SEQ ID NO:41 has 1204 amino acid residues and is presented in Table 16B using the one-letter amino acid code. Signal P, Psort and/or Hydropathy results predict that NOV16 has a signal peptide and is likely to be localized at the plasma membrane with a certainty of 0.8000.

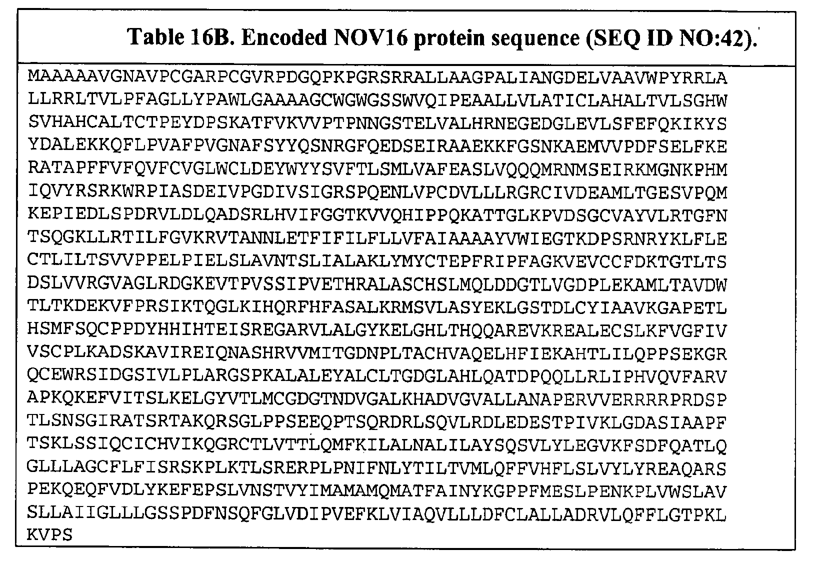

A search of sequence databases reveals that the NOV16 amino acid sequence has 1141 of 1200 amino acid residues (95%) identical to, and 1164 of 1200 amino acid residues (97%) similar to, the 1200 amino acid residue ptnr:TREMBLNEW-ACC:BAB20095 protein from Mus musculus (Mouse) (CATION-TRANSPORTING ATPASE). Public amino acid databases include the GenBank databases, SwissProt, PDB and PIR.

NOV16 is expressed in at least Adrenal Gland/Suprarenal gland, Amygdala, Aorta, Appendix, Artery, Bone, Bone Marrow, Brain, Bronchus, Brown adipose, Cartilage, Cerebral Medulla/Cerebral white matter, Cervix, Colon, Coronary Artery, Epidermis, Hair Follicles, Heart, Hippocampus, Kidney, Left cerebellum, Liver, Lung, Lymph node, Lymphoid tissue, Mammary gland/Breast, Ovary, Oviduct/Uterine Tube/Fallopian tube, Pancreas, Parietal Lobe, Peripheral Blood, Pituitary Gland, Placenta, Prostate, Respiratory Bronchiole, Right Cerebellum, Skeletal Muscle, Skin, Spinal Cord, Spleen, Stomach, Substantia Nigra, Synovium/Synovial membrane, Temporal Lobe, Testis, Thymus, Urinary Bladder, Uterus, Vein, and Vulva. This information was derived by determining the tissue sources of the sequences that were included in the invention including but not limited to SeqCalling sources, Public EST sources, Literature sources, and/or RACE sources.

The disclosed NOV16 polypeptide has homology to the amino acid sequences shown in the BLASTP data listed in Table 16C.

| **Table 16C. BLAST results for NOV16** | | | | | |
|---|---|---|---|---|---|
| Gene Index/ Identifier | Protein/ Organism | Length (aa) | Identity (%) | Positives (%) | Expect |
| gi\|14017867\|dbj\|BAB 47454.1\| (AB058728) | KIAA1825 protein [Homo sapiens] | 1203 | 1200/1203 (99%) | 1200/1203 (99%) | 0.0 |
| gi\|18202961\|sp\|Q9HD 20\|ATY2 HUMAN | Probable cation-transporting ATPase 2 (CGI-152) | 1204 | 1201/1204 (99%) | 1201/1204 (99%) | 0.0 |
| gi\|18202867\|sp\|Q9EP E9\|ATY2 MOUSE | Probable cation-transporting ATPase 2 (CATP) | 1200 | 1141/1200 (95%) | 1164/1200 (96%), | 0.0 |
| gi\|9966897\|ref\|NP 0 65143.1\| (NM_020410 | CGI-152 protein [Homo sapiens] | 1086 | 1072/1072 (100%) | 1072/1072. (100%) | 0.0 |
| gi\|18467838\|ref\|XP 079402.1\| (XM 079402) | BcDNA:GH06032 [Drosophila melanogaster] | 1225 | 611/1125 (54%) | 803/1125 (71%), | 0.0 |

Tables 16D-E list the domain descriptions from DOMAIN analysis results against NOV16. This indicates that the NOV16 sequence has properties similar to those of other proteins known to contain this domain.

| **Table 16D. Domain Analysis of NOV16** |
|---|
| gnl \|Pfam\|pfam00122, E1-E2_ATPase, E1-E2 ATPase |
| CD-Length = 223 residues, 57.4% aligned |
| Score = 64.3 bits (155), Expect = 4e-11 |

| **Table 16E. Domain Analysis of NOV16** |
|---|
| gnl\|Pfam\|pfam00702, Hydrolase, haloacid dehalogenase-like hydrolase. This family are structurally different from the alpha/ beta hydrolase family (pfam00561). This family includes L-2-haloacid dehalogenase, epoxide hydrolases and phosphatases. The structure of the family consists of two domains. One is an inserted four helix bundle, which is the least well conserved region of the alignment, between residues 16 and 96. The rest of the fold is composed of the core alpha/beta domain. |
| CD-Length = 197 residues, 78.2% aligned |
| Score = 39.7 bits (91), Expect = 0.001 |

Regulation of cation homeostasis is of critical importance to the cell and defects in proteins that regulate this process have been shown to cause a number of human diseases, including Darier-White disease, Menkes disease and Wilson disease. The human plasma membrane Ca(2+)-ATPase (PMCA) isoforms are members of the P class of ion-motive ATPases. PMCA removes bivalent calcium ions from eukaryotic cells and plays a critical role in intracellular calcium homeostasis by its capacity for removing calcium ions from cells against very large concentration gradients. Together with the highly related SERCA1 and SERCA3 isoforms encoded by ATP2A1 and ATP2A3, respectively, SERCA2 belongs to the large family of P-type cation pumps that couple ATP hydrolysis with cation transport across membranes. SERCA pumps specifically maintain low cytosolic Ca(2+) concentrations by actively transporting Ca(2+) from the cytosol into the sarco/endoplasmic reticulum lumen. The ATP2A2 gene has been shown to be the site of mutations in Darier-White disease, an autosomal dominant skin disorder characterized by warty papules and plaques in seborrheic areas (central trunk, flexures, scalp, and forehead), palmoplantar pits, and distinctive nail abnormalities (See Sakuntabhai et al., (1999) Mutations in ATP2A2, encoding a Ca(2+) pump, cause Darier disease. Nature Genet. 21: 271-277). Patients with Menkes disease have mutations in the gene encoding Cu(2+)-transporting ATPase alpha polypeptide and display early retardation in growth, peculiar hair, and focal cerebral and cerebellar degeneration ((See Chelly et al., (1993) Isolation of a candidate gene for Menkes disease that encodes a potential heavy metal binding protein. Nature Genet. 3: 14-19). Wilson disease is an autosomal recessive disorder caused by mutations in the ATP7B gene, which encodes a copper-transporting ATPase, and is characterized by dramatic build-up of intracellular hepatic copper with subsequent hepatic and neurologic abnormalities (See Bull et al.. (1993) The Wilson disease gene is a putative copper transporting P-type ATPase similar to the Menkes gene. Nature Genet. 5: 327-337).

The human cation transporting ATPase-like protein described in this invention is predicted to share the attributes of the other cation transporting ATPase family members and is thus implicated in the regulation of cation homeostasis. Given that a large number of cation transporting ATPases have been demonstrated to have a causative role in a variety of human diseases, the cation transporting ATPase-like protein is an attractive target for drug intervention in the treatment of human metabolic diseases, central nervous system disorders, immunological diseases and cancer, among others. The cation transporting ATPase-like gene described in this patent maps to human chromosome 19.

The disclosed NOV16 nucleic acid of the invention encoding a cation-transporting ATPase-like protein includes the nucleic acid whose sequence is provided in Table 16A or a fragment thereof. The invention also includes a mutant or variant nucleic acid any of whose bases may be changed from the corresponding base shown in Table 16A while still encoding a protein that maintains its cation-transporting ATPase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 0 percent of the bases may be so changed.

The disclosed NOV16 protein of the invention includes the cation-transporting ATPase-like protein whose sequence is provided in Table 16B. The invention also includes a mutant or variant protein any of whose residues may be changed from the corresponding residue shown in Table 16B while still encoding a protein that maintains its cation-transporting ATPase-like activities and physiological functions, or a functional fragment thereof. In the mutant or variant protein, up to about 5 percent of the residues may be so changed.

The invention further encompasses antibodies and antibody fragments, such as F_{ab} or (F_{ab})₂, that bind immunospecifically to any of the proteins of the invention.

The above defined information for this invention suggests that this cation-transporting ATPase-like protein (NOV 16) may function as a member of a "cation-transporting ATPase family". Therefore, the NOV16 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue *regeneration in vivo* and *in vitro* of all tissues and cell types composing (but not limited to) those defined here.

The NOV16 nucleic acids and proteins of the invention are useful in potential therapeutic applications implicated in diseases including but not limited to various pathologies and disorders as indicated below. For example, a cDNA encoding the cation-transporting ATPase-like protein (NOV16) may be useful in gene therapy, and the cation-transporting ATPase-like protein (NOV16) may be useful when administered to a subject in need thereof. By way of nonlimiting example, the compositions of the present invention will have efficacy for treatment of patients suffering from cancer, trauma, bacterial and viral infections, in vitro and in vivo regeneration, cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, aneurysm, hypertension, fibromuscular dysplasia, stroke, obesity, transplantation, myocardial infarction, embolism, cardiovascular disorders, bypass surgery, anemia, bleeding disorders, adrenoleukodystrophy , congenital adrenal hyperplasia, diabetes, Von Hippel-Lindau (VHL) syndrome, pancreatitis, fertility, endometriosis, hypogonadism, hypercalceimia, ulcers, cirrhosis, Hirschsprung's disease, Crohn's Disease, appendicitis, hemophilia, hypercoagulation, idiopathic thrombocytopenic purpura, ataxia-telangiectasia, lymphedema, allergies, hemophilia, autoimmune disease, allergies, immunodeficiencies, transplantation, graft versus host disease (GVHD), osteoporosis, arthritis, ankylosing spondylitis, scoliosis, arthritis, tendonitis, muscular dystrophy, Lesch-Nyhan syndrome, myasthenia gravis, Alzheimer's disease, Parkinson's disease, Huntington's disease, cerebral palsy, epilepsy, multiple sclerosis, leukodystrophies, behavioral disorders, addiction, anxiety, pain, neurodegeneration, endocrine dysfunctions, growth and reproductive disorders, systemic lupus erythematosus, asthma, emphysema, ARDS, psoriasis, actinic keratosis, acne, hair growth/loss, allopecia, pigmentation disorders,, cystitis, incontinence, renal artery stenosis, interstitial nephritis, glomerulonephritis, polycystic kidney disease, renal tubular acidosis, and IgA nephropathy, or other pathologies or conditions. The NOV16 nucleic acid encoding the cation-transporting ATPase-like protein of the invention, or fragments thereof, may further be useful in diagnostic applications, wherein the presence or amount of the nucleic acid or the protein are to be assessed.

NOV16 nucleic acids and polypeptides are further useful in the generation of antibodies that bind immuno-specifically to the novel NOV16 substances for use in therapeutic or diagnostic methods. These antibodies may be generated according to methods known in the art, using prediction from hydrophobicity charts, as described in the "Anti-NOVX Antibodies" section below. The disclosed NOV16 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV17

A disclosed NOV17 nucleic acid of 1167 nucleotides (also referred to as CG55914-O1) encoding an acyl CoA desaturase-like protein is shown in Table 17A. Putative untranslated regions upstream and/or downstream from the coding region, if any, are underlined, and the start and stop codons are in bold letters.

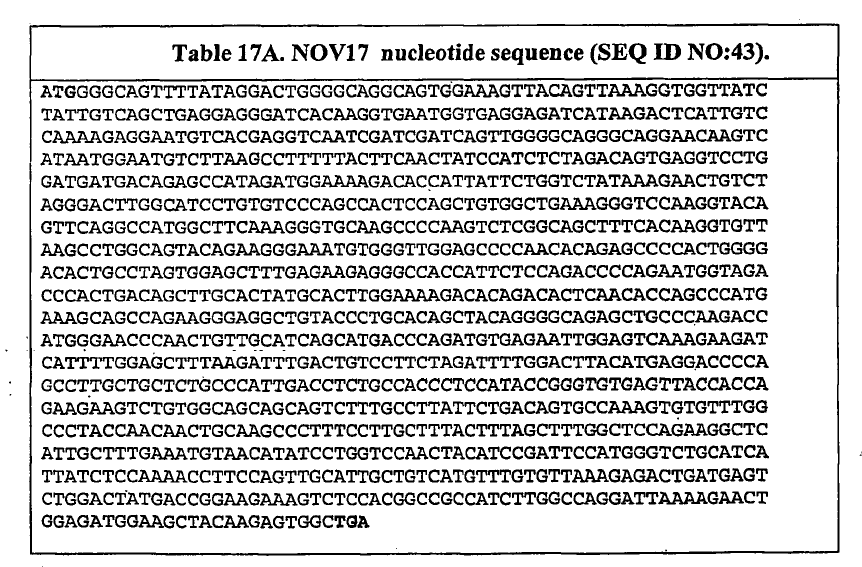

In a search of public sequence databases, the NOV17 nucleic acid sequence, located on chromsome 5 has 316 of 381 bases (82%) identical to a gb:GENBANK-ID:AK000899|acc:AK000899.1 mRNA from Homo sapiens (Homo sapiens cDNA FLJ10037 fis, clone HEMBA1000968). Public nucleotide databases include all GenBank databases and the GeneSeq patent database.

The disclosed NOV17 polypeptide (SEQ ID NO:44) encoded by SEQ ID NO:43 has 388 amino acid residues and is presented in Table B using the one-letter amino acid code. Signal P, Psort and/or Hydropathy results predict that NOV17 has no signal peptide and is likely to be localized in the cytoplasm with a certainty of 0.6500.

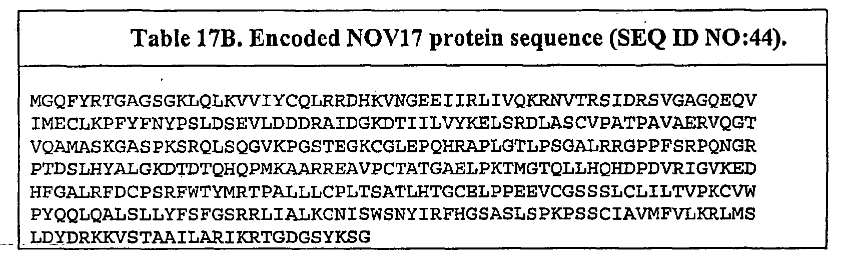

A search of sequence databases reveals that the NOV17 amino acid sequence has 25 of 30 amino acid residues (83%) identical to, and 27 of 30 amino acid residues (90%) similar to, the 359 amino acid residue ptnr:SWISSNEW-ACC:O00767 protein from Homo sapiens (Human) (ACYL-COA DESATURASE (EC 1.14.99.5) (STEAROYL-COA DESATURASE) (FATTY ACID DESATURASE) (DELTA(9)-DESATURASE)). Public amino acid databases include the GenBank databases, SwissProt, PDB and PIR.

NOV17 is expressed in at least Adipose, Adrenal Gland/Suprarenal gland, Aorta, Appendix, Artery, Bone, Bone Marrow, Brain, Bronchus, Buccal mucosa, Cartilage, Cerebral Medulla/Cerebral white matter, Cervix, Cochlea, Colon, Cornea, Coronary Artery, Dermis, Epidermis, Foreskin, Frontal Lobe, Gall Bladder, Hair Follicles, Heart, Hippocampus, Hypothalamus, Kidney, Larynx, Left cerebellum, Liver, Lung, Lung Pleura, Lymph node, Lymphoid tissue, Mammary gland/Breast, Myometrium, Ovary, Pancreas, Parathyroid Gland, Parietal Lobe, Parotid Salivary glands, Peripheral Blood, Pharynx, Pituitary Gland, Placenta, Prostate, Respiratory Bronchiole, Retina, Right Cerebellum, Salivary Glands, Skeletal Muscle, Skin, Small Intestine, Spinal Chord, Spleen, Stomach, Substantia Nigra, Synovium/Synovial membrane, Temporal Lobe, Testis, Thalamus, Thymus, Thyroid, Trachea, Umbilical Vein, Urinary Bladder, Uterus, Vein, Vulva. This information was derived by determining the tissue sources of the sequences that were included in the invention including but not limited, to SeqCalling sources, Public EST sources, Literature sources, and/or RACE sources.

The disclosed NOV17 polypeptide has homology to the amino acid sequences shown in the BLASTP data listed in Table 17C.

| **Table 17C. BLAST results for NOV17** | | | | | |
|---|---|---|---|---|---|
| Gene Index/ Identifier | Protein/ Organism | Length (aa) | Identity (%) | Positives (%) | Expect |
| gi\|14756057\|ref\|XP 052325.11 (XM_052325) | hypothetical protein FLJ14058 [Homo sapiens] | 222 | 47/74 (63%) | 53/74 (71%) | 2e-18 |
| gi \|185725321 \|ref \|XP 088501.11 (XM_088501) | KIAA1161 protein [Homo sapiens] | 188 | 59/145 (40%) | 72/145 (48%), | 7e-17 |
| gi\|14722528\|ref\|XP 033352.1\| (XM_033352 | similar to hypothetical protein FLJ14058 [Homo sapiens] | 129 | 45/94(47% ) | 56/94 (58%) | 2e-15 |
| gi 16588389 gb AAL2 6787.1 AF304442 1 (AF304442) | B lymphocyte activation-related protein BC-1514 [Homo sapiens] | 130 | 48/126 (38%) | 56/126 (44%), | 3e-10 |
| gi\|18562020\|ref\|XP 087778.1 \| (XM 087778) | hypothetical protein XP_087778 [Homo sapiens] | 216 | 36/68 (52%) | 39/68 (56%), | 2e-8 |

Fatty acid desaturases (ec 1.14.99.-) are enzymes that catalyze the insertion of a double bond at the delta position of fatty acids. There are two distinct families of fatty acid desaturases which do not seem to be evolutionary related.

### Family 1 is composed of:

- Stearoyl-coa desaturase (scd) (ec 1.14.99.5).scd is a key regulatory enzyme of unsaturated fatty acid biosynthesis. scd introduces a cis double bond at the delta(9) position of fatty acyl-coa's such as palmitoleoyl- and oleoyl-coa. scd is a membrane-bound enzyme that is thought to function as a part of a multienzyme complex in the endoplasmic reticulum of vertebrates and fungi.

### Family 2 is composed of:

- Plants stearoyl-acyl-carrier-protein desaturase (ec 1.14.99.6), these enzymes catalyze the introduction of a double bond at the delta(9) position of steraoyl-acp to produce oleoylacp. this enzyme is responsible for the conversion of saturated fatty acids to unsaturated fatty acids in the synthesis of vegetable oils. - cyanobacteria desa an enzyme that can introduce a second cis double bond at the delta(12) position of fatty acid bound to membranes glycerolipids. desa is involved in chilling tolerance; the phase transition temperature of lipids of cellular membranes being dependent on the degree ofunsaturation of fatty acids of the membrane lipids.

The disclosed NOV17 nucleic acid of the invention encoding an acyl CoA desaturase-like protein includes the nucleic acid whose sequence is provided in Table 17A or a fragment thereof. The invention also includes a mutant or variant nucleic acid any of whose bases may be changed from the corresponding base shown in Table 17A while still encoding a protein that maintains its acyl CoA desaturase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 18 percent of the bases may be so changed.

The disclosed NOV17 protein of the invention includes the acyl CoA desaturase-like protein whose sequence is provided in Table 17B. The invention also includes a mutant or variant protein any of whose residues may be changed from the corresponding residue shown, in Table 17B while still encoding a protein that maintains its acyl CoA desaturase-like activities and physiological functions, or a functional fragment thereof. In the mutant or variant protein, up to about 17 percent of the residues may be so changed.

The invention further encompasses antibodies and antibody fragments, such as F_{ab} or (F_{ab})₂, that bind immunospecifically to any of the proteins of the invention.

The above defined information for this invention suggests that this acyl CoA desaturase-like protein (NOV17) may function as a member of a "acyl CoA desaturase family". Therefore, the NOV17 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV17 nucleic acids and proteins of the invention are useful in potential therapeutic applications implicated in diseases including but not limited to various pathologies and disorders as indicated below. For example, a cDNA encoding the acyl CoA desaturase-like protein (NOV17) may be useful in gene therapy, and the acyl CoA desaturase-like protein (NOV17) may be useful when administered to a subject in need thereof. By way of nonlimiting example, the compositions of the present invention will have efficacy for treatment of patients suffering from Cardiomyopathy, Atherosclerosis, Hypertension, Congenital heart defects, Aortic stenosis, Atrial septal defect (ASD), Atrioventricular (A-V) canal defect, Ductus arteriosus , Pulmonary stenosis, Subaortic stenosis, Ventricular septal defect (VSD), valve diseases, Tuberous sclerosis, Scleroderma, Obesity, Transplantation, Von Hippel-Lindau (VHL) syndrome, Cirrhosis, Transplantation, Von Hippel-Lindau (VHL) syndrome, Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection, or other pathologies or conditions. The NOV17 nucleic acid encoding the acyl CoA desaturase-like protein of the invention, or fragments thereof, may further be useful in diagnostic applications, wherein the presence or amount of the nucleic acid or the protein are to be assessed.

NOV17 nucleic acids and polypeptides are further useful in the generation of antibodies that bind immuno-specifically to the novel NOV17 substances for use in therapeutic or diagnostic methods. These antibodies may be generated according to methods known in the art, using prediction from hydrophobicity charts, as described in the "Anti-NOVX Antibodies" section below. The disclosed NOV17 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV18

A disclosed NOV18 nucleic acid of 853 nucleotides (also referred to as CG57328-01) encoding a myo-inositol-1 (or 4) monophosphatase-like protein is shown in Table 18A. The start and stop codons are in bold letters.

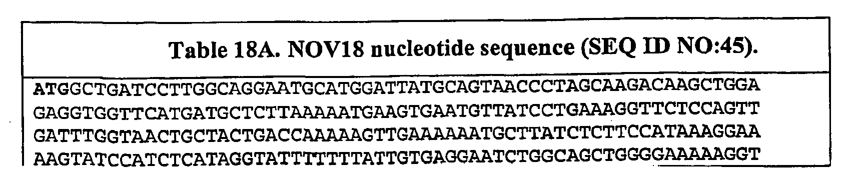

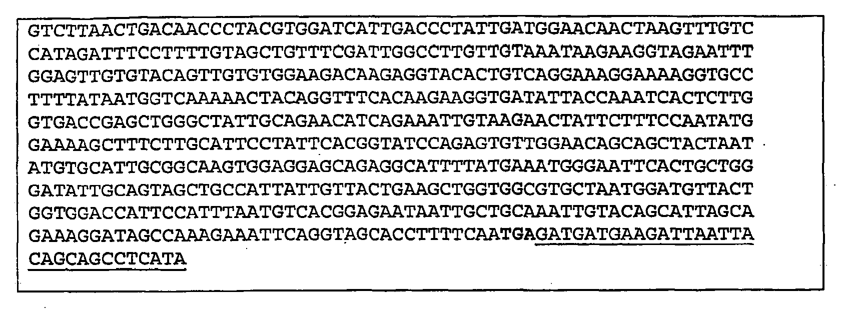

In a search of public sequence databases, the NOV18 nucleic acid sequence, located on chromsome 8 has 761 of 853 bases (89%) identical to a gb:GENBANK-ID:AF042729|acc:AF042729.2 mRNA from Homo sapiens (Homo sapiens lithium-sensitive myo-inositol monophosphatase A1 (IMPA1) mRNA, complete cds). Public nucleotide databases include all GenBank databases and the GeneSeq patent database.

The disclosed NOV18 polypeptide (SEQ ID NO:46) encoded by SEQ ID NO:45 has 273 amino acid residues and is presented in Table 18B using the one-letter amino acid code. Signal P, Psort and/or Hydropathy results predict that NOV18 has no signal peptide and is likely to be localized in the cytoplasm with a certainty of 0.6500.

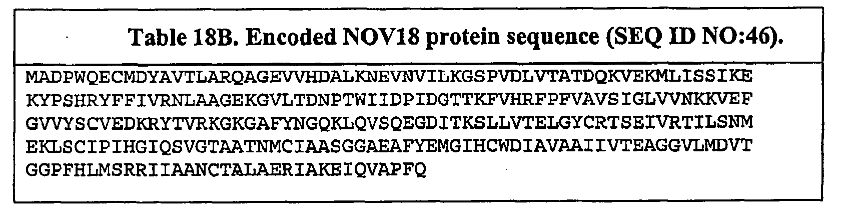

A search of sequence databases reveals that the NOV18 amino acid sequence has 222 of 273 amino acid residues (81 %) identical to, and 240 of 273 amino acid residues (87%) similar to, the 277 amino acid residue ptnr:SWISSNEW-ACC:P29218 protein from Homo sapiens (Human) (MYO-INOSITOL-1(OR 4)-MONOPHOSPHATASE (EC 3.1.3.25) (IMPASE) (IMP) (INOSITOL MONOPHOSPHATASE) (LITHIUM-SENSITIVE MYO-INOSITOL MONOPHOSPHATASE Al)). Public amino acid databases include the GenBank databases, SwissProt, PDB and PIR.

NOV18 is expressed in at least Brain, Lung. This information was derived by determining the tissue sources of the sequences that were included in the invention including but not limited to SeqCalling sources, Public EST sources, Literature sources, and/or RACE sources.

The disclosed NOV18 polypeptide has homology to the amino acid sequences shown in the BLASTP data listed in Table 18C.

| **Table 18C. BLAST results for NOV18** | | | | | |
|---|---|---|---|---|---|
| Gene Index/ Identifier | Protein/ Organism | Length (aa) | Identity (%) | Positives (%) | Expect |
| gi\|18570157\|ref\|XP 095533.1 (XM_095533) | hypothetical protein XP_095533 [Homo sapiens] | 512 | | | |
| gi\|5031789\|ref\|NP 0 05527.11 (NM_005536) | inositol(myo)-1(or 4)-monophosphatase 1 [Homo sapiens] | 277 | 222/273 (81%) | 240/273 (87%), | e-124 |
| gi\|3914092\|sp\|P9769 7\|MYOP RAT | Myo-inositol-1(or 4)-monophosphatase (IMPase) (IMP) (Inositol monophosphatase) (Lithium-sensitive myoinositol monophosphatase A1) | 277 | 222/273 (81%) | 240/273 (87%), | e-124 |
| gi\|443382\|pdb\|2HHM\| A | Chain A, Human Inositol Monophosphatase (E.C.3.1.3.25) Dimer Complex with Gadolinium And Sulfate | 276 | | | |
| gi\|127716\|sp\|P20456 MYOP BOVIN | Myo-inositol-1(or 4)-monophosphatase (IMPase) (IMP) (Inositol monophosphatase) (Lithium-sensitive myoinositol monophosphatase A1) | 277 | 209/273 (76%) | 236/273 (85%), | e-119 |

Table 18Dlists the domain descriptions from DOMAIN analysis results against NOV18. This indicates that the NOV18 sequence has properties similar to those of other proteins known to contain this domain.

| **Table 18D. Domain Analysis of NOV18** | | |
|---|---|---|
| gnl\|Pfam\|pfam00459, inositol_P, Inositol monophosphatase family | | |
| | CD-Length = 270 residues, 97.0% aligned | |
| | | Score = 238 bits (607), Expect = 3e-64 |

Myo-inositol-1(or 4)-monophosphatase enzyme catalyzes the reaction:
myo-inositol 1-monophosphate + H(2)O <=> myo-inositol + phosphate Acts on both enantiomers of myo-inositol-1-phosphate and myo- inositol 4-phosphate. It does not act on inositol bisphosphates, trisphosphates or tetrakisphosphates. It has been shown that several proteins share two sequence motifs. Two of these proteins are enzymes of the inositol phosphate second messenger signalling pathway:
   - Vertebrate and plants inositol monophosphatase (BC 3.1.3.25). Vertebrate inositol polyphosphate 1-phosphatase (EC 3.1.3.57).

### Other proteins are:

- Bacterial protein cysQ. CysQ could help to control the pool of PAPS (3'-phosphoadenoside 5'-phosphosulfate), or be useful in sulfite synthesis. - Escherichia coli protein suhB. Mutations in suhB results in the enhanced synthesis of heat shock sigma factor (htpR). - Neurospora crassa protein Qa-X. Probably involved in quinate metabolism.-Emericella nidulans protein qutG. Probably involved in quinate metabolism. - Yeast protein HAL2/MET22 involved in salt tolerance as well as methionine biosynthesis. - Yeast hypothetical hypothetical protein YHR046c. - Caenorhabditis elegans hypothetical protein F13G3.5. - A Rhizobium leguminosarum hypothetical protein encoded upstream of the pss gene for exopolysaccharide synthesis. - Methanococcus jannaschii hypothetical protein MJ0109.

It is suggested that these proteins may act by enhancing the synthesis or degradation of phosphorylated messenger molecules. From the X-ray structure of human inositol monophosphatase, it seems that some of the conserved residues are involved in binding a metal ion and/or the phosphate group of the substrate.

The disclosed NOV18 nucleic acid of the invention encoding a myo-inositol-1 (or 4) monophosphatase-like protein includes the nucleic acid whose sequence is provided in Table . 18A or a fragment thereof. The invention also includes a mutant or variant nucleic acid any of whose bases may be changed from the corresponding base shown in Table 18A while still encoding a protein that maintains its myo-inositol-1 (or 4) monophosphatase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 11 percent of the bases may be so changed.

The disclosed NOV18 protein of the invention includes the myo-inositol-1 (or 4) monophosphatase-like protein whose sequence is provided in Table 18B. The invention also includes a mutant or variant protein any of whose residues may be changed from the corresponding residue shown in Table 18B while still encoding a protein that maintains its myo-inositol-1 (or 4) monophosphatase-like activities and physiological functions, or a functional fragment thereof. In the mutant or variant protein, up to about 19 percent of the residues may be so changed.

The invention further encompasses antibodies and antibody fragments, such as F_{ab} or (F_{ab})₂, that bind immunospecifically to any of the proteins of the invention.

The above defined information for this invention suggests that this myo-inositol-1 (or 4) monophosphatase-like protein (NOV18) may function as a member of a "myo-inositol-1 (or 4) monophosphatase family". Therefore, the NOV18 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV18 nucleic acids and proteins of the invention are useful in potential therapeutic applications .implicated in diseases including but not limited to various pathologies and disorders as indicated below. For example, a cDNA encoding the myo-inositol-1 (or 4) morlophosphatase-like protein (NOV18) may be useful in gene therapy, and the myo-inositol-1 (or 4) monophosphatase-like protein (NOV18) maybe useful when administered to a subject in need thereof. By way of nonlimiting example, the compositions of the present invention will have efficacy for treatment of patients suffering from Systemic lupus erythematosus, Autoimmune disease, Asthma, Emphysema, Scleroderma, allergy, Von Hippel-Lindau (VHL) syndrome, Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection, or other pathologies or conditions. The NOV18 nucleic acid encoding the myo-inositol-1 (or 4) monophosphatase-like protein of the invention, or fragments thereof, may further be useful in diagnostic applications, wherein the presence or amount of the nucleic acid or the protein are to be assessed.

NOV18 nucleic acids and polypeptides are further useful in the generation of antibodies that bind immuno-specifically to the novel NOV18 substances for use in therapeutic or diagnostic methods. These antibodies may be generated according to methods known in the art, using prediction from hydrophobicity charts, as described in the "Anti-NOVX Antibodies" section below. The disclosed NOV18 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV19

A disclosed NOV19 nucleic acid of 2071 nucleotides (also referred to as CG57358-01) encoding a spinster-like protein is shown in Table 19A. The start and stop codons are in bold letters.

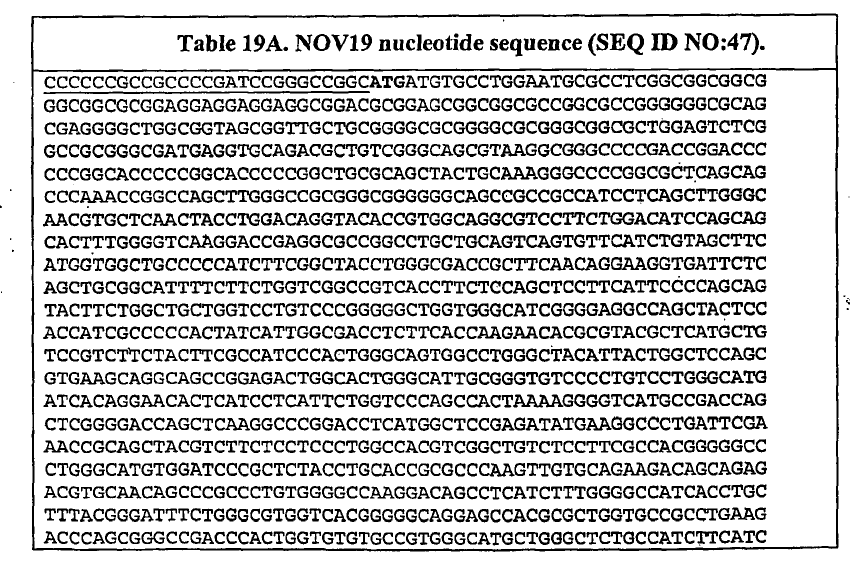

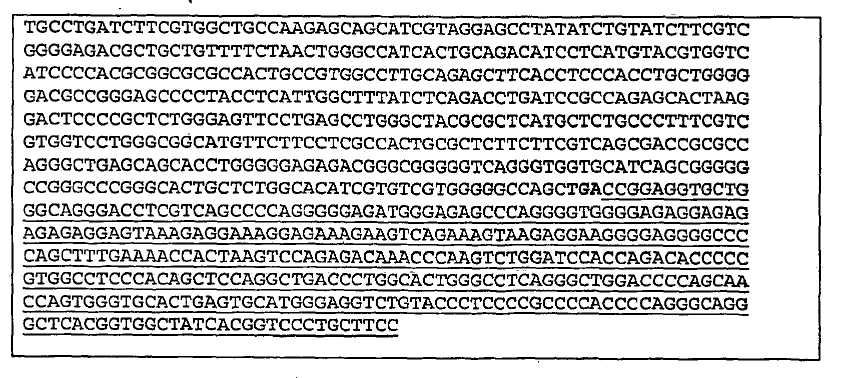

In a search of public sequence databases, the NOV19 nucleic acid sequence, located on chromsome 17 has 290 of 431 bases (67%) identical to a gb:GENBANK-ID:E12646|acc:E12646:1 mRNA from Homo sapiens (cDNA encoding cell growth inhibiting factor). Public nucleotide databases include all GenBank databases and the GeneSeq patent database.

The disclosed NOV19 polypeptide (SEQ ID NO:48) encoded by SEQ ID NO:47 has 566 amino acid residues and is presented in Table 19B using the one-letter amino acid code. Signal P, Psort and/or Hydropathy results predict that NOV19 has no signal peptide and is likely to be localized at the plasma membrane with a certainty of 0.6000.

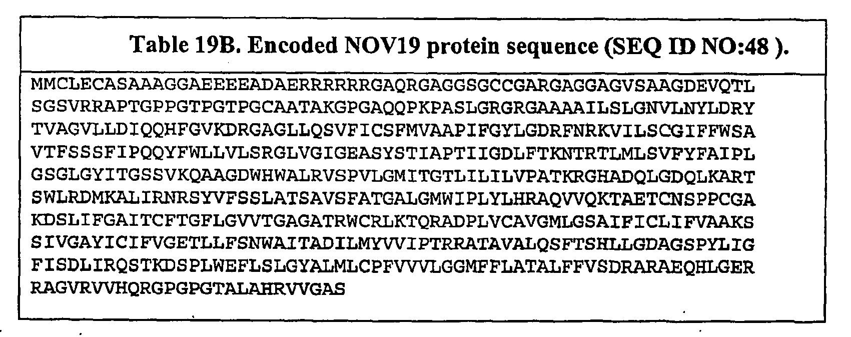

A search of sequence databases reveals that the NOV19 amino acid sequence has 268 of 495 amino acid residues (54%) identical to, and 330 of 495 amino acid residues (66%) similar to, the 528 amino acid residue ptnr:TREMBLNEW-ACC:AAG43830 protein from Homo sapiens (Human) (SPINSTER-LIKE PROTEIN). Public amino acid databases include the GenBank databases, SwissProt, PDB and PIR.

NOV is expressed in at least brain and heart. This information was derived by determining the tissue sources of the sequences that were included in the invention including but not limited to SeqCalling sources, Public EST sources, Literature sources, and/or RACE sources.

The disclosed NOV19 polypeptide has homology to the amino acid sequences shown in the BLASTP data listed in Table 19C.

| **Table 19C. BLAST results for NOV19** | | | | | |
|---|---|---|---|---|---|
| Gene Index/ identifier | Protein/ organism | Length (aa) | Identity (%) | Positives (%) | Expect |
| gi\|15079262\|gb\|AAH1 1467.1\|AAH11467 (BC011467) | protein for IMAGE:3154539) [Mus musculus] | 590 | 514/539 (95%) | 523/539 (96%) | 0.0 |
| gi\|18448989\|gb\|AAL6 9987.1\|AF465772 1 (AF465772) | not really started [Danio rerio] | 506 | 248/431 (57%) | 312/431 (71%), | e-133 |
| gi\|13544043\|gb\|AAH0 6156.1\|AAH06156 (BC006156) | (protein for IMAGE:3627317) [Homo sapiens] | 524 | 266/478 (55%) | 327/478 (67%), | e-129 |
| gi\|14042968\|ref\|NP 114427.1 (NM 032038) | spinster-like protein [Homo sapiens] | 528 | 268/495 (54%) | 330/495 (66%), | e-129 |
| gi 12963795 ref NP 076201.1 (NM 023712) | spinster-like protein [Mus musculus] | 528 | 266/510 (52%) | 330/510 (64%), | e-126 |

Table 19D-E lists the domain descriptions from DOMAIN analysis results against NOV19. This indicates that the NOV sequence has properties similar to those of other proteins known to contain this domain.

| **Table 19D. Domain Analysis of NOV19** |
|---|
| gnl Pfam pfam00083, sugar_tr, Sugar (and other) transporter. |
| CD-Length = 447 residues, 28.2% aligned |
| Score = 55.1 bits (131), Expect = 1e-08 |

| **Table 19E. Domain Analysis of NOV19** | |
|---|---|
| gnl\|Pfam\|pfam03137, OATP_C, Organic Anion Transporter Polypeptide (OATP) family, C-terminus. This family consists of several eukaryotic Organic-Anion-Transporting Polypeptides (OATPs). Several have been identified mostly in human and rat. Different OATPs vary in tissue distribution and substrate specificity. Since the numbering of different OATPs in particular species was based originally on the order of discovery, similarly numbered OATPs in humans and rats did not necessarily correspond in function, tissue distribution and substrate specificity (in spite of the name, some OATPs also transport organic cations and neutral molecules). Thus, Tamai et al. initiated the current scheme of using digits for rat OATPs and letters for human ones. Prostaglandin transporter (PGT) proteins (e.g. Pfam:Q92959) are also considered to be OATP family members. In addition, the methotrexate transporter OATK (Pfam:P70502) is closely related to OATPs. This family aligns residues towards the C-terminus. The family OATP_N aligns residues from similar proteins towards the N-terminus. This family also includes several predicted proteins from Caenorhabditis elegans and Drosophila melanogaster. This similarity was not previously noted. Note: Members of this family are described (in the Swiss-Prot database) as belonging to the SLC21 family of transporters. | |
| | CD-Length = 375 residues, 18.9% aligned |
| | Score = 37.7 bits (86), Expect = 0.002 |

NOV19 is a homolog of the spinster-like proteins in human and mouse. Spinster is a novel membrane protein in Drosophila, mutants of which exhibit accumulation of ceroid lipofuscin and neural degeneration (See Nakano et al., Genbank entry for AAG43830.1). Accumulation of ceroid lipofuscin occurs in several hereditary disorders that are probably related to lysosomal storage defects. The pigment makes fibroblasts in vitro more susceptible to oxidative stress, leading to apoptosis (See Terman et al., Exp Gerontol 1999 Sep;34(6):755-70). It is also a component of atherogenic lesions in arteries (See Hoffe and Hoppe, Curr Opin Lipidol 1995 Oct;6(5):317-25). In neurons, accumulation of the pigment leads to neurodegeneration, even leading to death in some cases (See Dyken and Wisniewski, Am J Med Genet 1995 Jun 5;57(2):150-4). This is seen both in inherited forms of human ceroid lipofuscinoses (See Jagadha et al., Acta Neuropathol (Ber1) 1988;75(3):233-40) and in a cathepsin-D-deficient mouse model (See Koike et al., J Neurosci 2000 Sep 15;20(18):6898-906). In at least one case, neuronal deposition of ceroid lipofuscin was also correlated with accumulation in the myocardium (See Jay and Haslam, J Inherit Metab Dis 1995;18(3):359-60).

The disclosed NOV19 nucleic acid of the invention encoding a spinster-like protein includes the nucleic acid whose sequence is provided in Table 19A or a fragment thereof. The invention also includes a mutant or variant nucleic acid any of whose bases may be changed from the corresponding base shown in Table 19A while still encoding a protein that maintains its spinster-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 33 percent of the bases may be so changed.

The disclosed NOV19 protein of the invention includes the spinster-like protein whose sequence is provided in Table 19B. The invention also includes a mutant or variant protein any of whose residues may be changed from the corresponding residue shown in Table 19B while still encoding a protein that maintains its spinster-like activities and physiological functions, or a functional fragment thereof In the mutant or variant protein, up to about 46 percent of the residues may be so changed.

The invention further encompasses antibodies and antibody fragments, such as F_{ab} or (F_{ab})₂, that bind immunospecifically to any of the proteins of the invention.

The above defined information for this invention suggests that this spinster-like protein (NOV19) may function as a member of a "spinster family". Therefore, the NOV19 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV19 nucleic acids and proteins of the invention are useful in potential therapeutic applications implicated in diseases including but not limited to various pathologies and disorders as indicated below. For example, a cDNA encoding the spinster-like protein (NOV19) may be useful in gene therapy, and the spinster-like protein (NOV19) may be useful when administered to a subject in need thereof. By way of nonlimiting example, the compositions of the present invention will have efficacy for treatment of patients suffering from cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, transplantation, Von Hippel-Lindau (VHL) syndrome, Alzheimer's disease, stroke, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders; addiction, anxiety, pain, neurodegeneration, cancer, tissue degeneration, bacterial/viral/parasitic infection, or other pathologies or conditions. The NOV19 nucleic acid encoding the spinster-like protein of the invention, or fragments thereof, may further be useful in diagnostic applications, wherein the presence or amount of the nucleic acid or the protein are to be assessed.

NOV19 nucleic acids and polypeptides are further useful in the generation of antibodies that bind immuno-specifically to the novel NOV19 substances for use in therapeutic or diagnostic methods. These antibodies may be generated according to methods known in the art, using prediction from hydrophobicity charts, as described in the "Anti-NOVX Antibodies" section below. The disclosed NOV19 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV20

A disclosed NOV20 nucleic acid of 752 nucleotides (also referred to as CG57695-01) encoding a casein-like protein is shown in Table 20A. The start and stop codons are in bold letters.

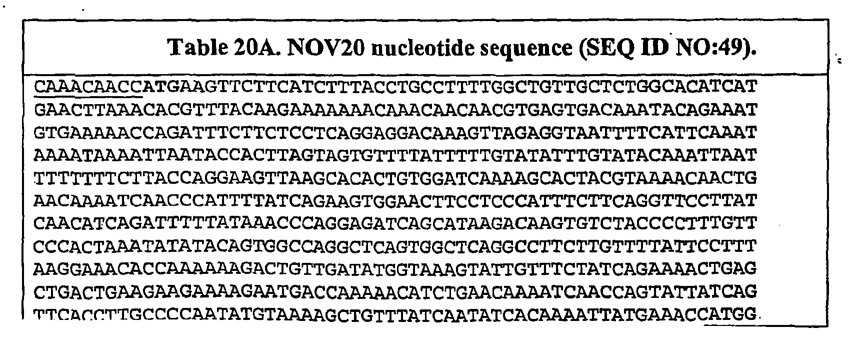

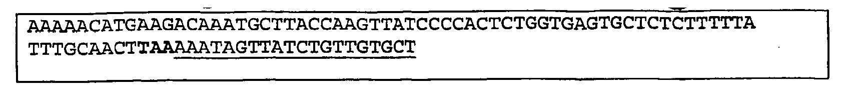

In a search of public sequence databases, the NOV20 nucleic acid sequence, located on chromsome 4 has 291 of 445 bases (65%) identical to a gb:GENBANK-ID:CHI249995|acc:AJ249995.1 mRNA from Capra hircus (Capra hircus mRNA for alpha s2-casein (cns 1s2 gene)). Public nucleotide databases include all GenBank databases and the GeneSeq patent database.

The disclosed NOV20 polypeptide (SEQ ID NO:50) encoded by SEQ ID NO:49 has 240 amino acid residues and is presented in Table 20B using the one-letter amino acid code. Signal P, Psort and/or Hydropathy results predict that NOV20 has a signal peptide and is likely to be localized extracellularly with a certainty of 0.5140.

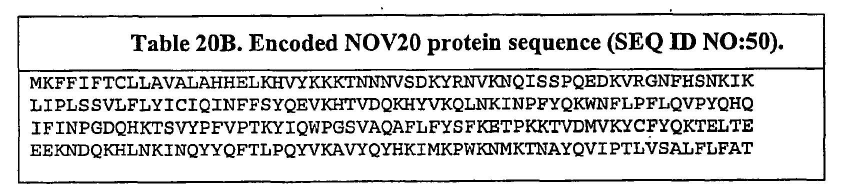

A search of sequence databases reveals that the NOV20 amino acid sequence has 112 of 232 amino acid residues (48%) identical to, and 142 of 232 amino acid residues (61 %) similar to, the 235 amino acid residue ptnr:pir-id:A48383 protein from pig (alpha s2-casein). Public amino acid databases include the GenBank databases, SwissProt, PDB and PIR.

NOV20 is expressed in at least lung, testis, and b-cell. This information was derived by determining the tissue sources of the sequences that were included in the invention including but not limited to SeqCalling sources, Public EST sources, Literature sources, and/or RACE sources.

The disclosed NOV20 polypeptide has homology to the amino acid sequences shown in the BLASTP data listed in Table 20C.

| **Table 20C. BLAST results for NOV20** | | | | | |
|---|---|---|---|---|---|
| Gene Index/ . Identifier | Protein/ organism | Length (aa) | Identity (%) | Positives (%) | Expect |
| gi\|477186\|pir\|A483 83 | alpha s2-casein-pig | 235 | 111/234 (47%) | 141/234 (59%) | 2e-37 |
| gi\|729044\|sp\|P39036 \|CAS2 PIG | Alpha-S2 casein precursor | 235 | 109/234 (46%) | 138/234 (58%) | 2e-35 |
| gi\|115658\|sp\|P04654 \|CAS2 SHEEP | Alpha-S2 casein precursor | 223 | 100/227 (44%) | 128/227 (56%) | 3e-28 |
| gi\|416751\|sp\|P33049 \|CAS2 CAPHI | Alpha-S2 casein precursor (Alpha-S2-CN) | 223 | 96/227 (42%) | 125/227 (54%) | 4e-28 |
| gi\|423305\|pir\|\|S338 81 | alphas2-casein B - goat | 223 | 96/227 (42%) | 125/227 (54%), | 4e-28 |

Table 20D lists the domain descriptions from DOMAIN analysis results against NOV20. This indicates that the NOV20 sequence has properties similar to those of other proteins known to contain this domain.

| **Table 20D. Domain Analysis of NOV20** |
|---|
| gnl\|Pfam \|pfam00363, caseins, Casein. |
| CD-Length = 84 residues, 71.4% aligned |
| Score = 44.3 bits (103), Expect = 8e-06 |

NOV20 has homology to pig alpha S casein. Caseins are the major protein constituent of milk. Caseins can be classified into two families; the first consists of the kappa-caseins, and the second groups the alpha-s1, alpha-s2, and beta-caseins. The alpha/beta caseins are a rapidly diverging family of proteins. However two regions are conserved: a cluster of phosphorylated serine residues and the signal sequence. Alpha-s2 casein is known as epsilon-casein in mouse, gamma-casein in rat and casein-A in guinea pig. Alpha-s1 casein is known as alpha-casein in rat and rabbit and as casein-B in guinea-pig.

Milk casein can be separated by urea starch electrophoresis into 3 regions, alpha, beta (115460), and kappa (601695) casein. Alpha and beta variants are present in the human population. Voglino and Ponzone (See Voglino, G. F.; Ponzone, A.: Nature N.B. 238: 149, 1972) postulated 2 biallelic systems. In Italy the frequency of the 2 alpha alleles was 0.908 and 0.092; 2 beta alleles had a frequency of 0.678 and 0.322.

Fujiwara et al. (See Fujiwara, Y.et al., Hum. Genet. 99: 368-373, 1997) found that the human alpha-S1, beta-, and kappa-casein genes are closely linked and arranged in that order. By fluorescence in situ hybridization, they demonstrated that the casein gene family is localized to 4q21.1. Rijnkels et al. (See Rijnkels, M., et al., Mammalian Genome 8: 285-286, 1997) concluded that the human 'locus' comprises at least 4 casein genes: 3 genes encoding calcium-sensitive, casein-like genes, and 1 kappa-casein gene, in the order alpha-s1-beta-alpha-s2--kappa. The approximate size of the human casein gene locus is 350 kb. Chen et al. (See Chen, C.-S. et al., Cytogenet. Cell Genet. 69: 260-265, 1995.) suggested that the casein cluster is located within 700 kb of the albumin (103600) gene cluster, which is located on 4q13.

The disclosed NOV20 nucleic acid of the invention encoding a casein-like protein includes the nucleic acid whose sequence is provided in Table 20A or a fragment thereof. The invention also includes a mutant or variant nucleic acid any of whose bases may be changed from the corresponding base shown in Table 20A while still encoding a protein that maintains its casein-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 35 percent of the bases may be so changed.

The disclosed NOV20 protein of the invention includes the casein-like protein whose sequence is provided in Table 20B. The invention also includes a mutant or variant protein any of whose residues may be changed from the corresponding residue shown in Table 20B while still encoding a protein that maintains its casein-like activities and physiological functions, or a functional fragment thereof. In the mutant or variant protein, up to about 52 percent of the residues may be so changed.

The invention further encompasses antibodies and antibody fragments, such as F_{ab} or (F_{ab})₂, that bind immunospecifically to any of the proteins of the invention.

The above defined information for this invention suggests that this casein-like protein (NOV20) may function as a member of a "casein family". Therefore, the NOV20 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo and in vitro* of all tissues and cell types composing (but not limited to) those defined here.

The NOV20 nucleic acids and proteins of the invention are useful in potential therapeutic applications implicated in diseases including but not limited to various pathologies and disorders as indicated below. For example, a cDNA encoding the casein-like protein (NOV20) may be useful in gene therapy, and the casein-like protein (NOV20) may be useful when administered to a subject in need thereof. By way of nonlimiting example, the compositions of the present invention will have efficacy for treatment of patients suffering from fertility, hypogonadism, systemic lupus erythematosus, autoimmune disease, asthma, emphysema, scleroderma, allergy, ARDS, hemophilia, hypercoagulation, idiopathic thrombocytopenic purpura, autoimmune disease, allergies, immunodeficiencies, transplantation, graft versus host disease (GVHD), lymphaedema, or other pathologies or conditions. The NOV20 nucleic acid encoding the casein-like protein of the invention, or fragments thereof, may further be useful in diagnostic applications, wherein the presence or amount of the nucleic acid or the protein are to be assessed.

NOV20 nucleic acids and polypeptides are further useful in the generation of antibodies that bind immuno-specifically to the novel NOV20 substances for use in therapeutic or diagnostic methods. These antibodies may be generated according to methods known in the art, using prediction from hydrophobicity charts, as described in the "Anti-NOVX Antibodies" section below. The disclosed NOV20 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV21

A disclosed NOV21 nucleic acid of 1704 nucleotides (also referred to as CG57654-01) encoding a gamma-aminobutyric acid receptor-like protein is shown in Table 21A. The start and stop codons are in bold letters.

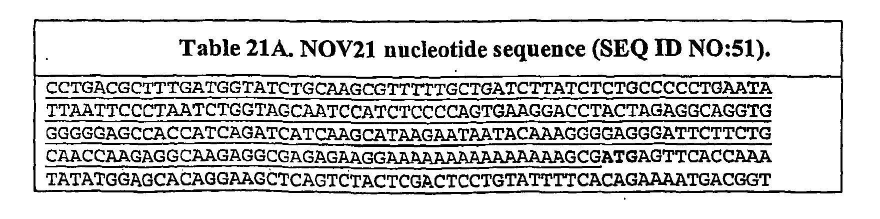

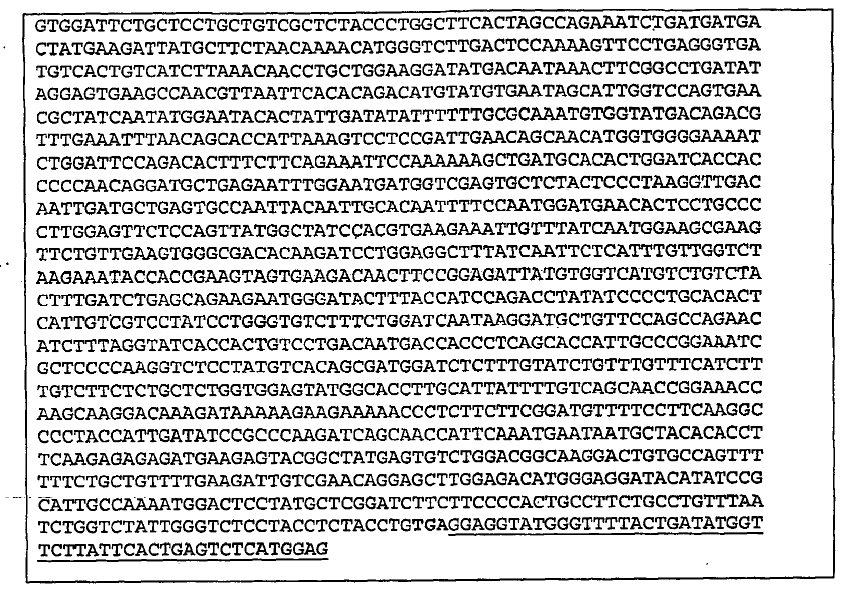

In a search of public sequence databases, the NOV21 nucleic acid sequence, located on chromsome 5 has 1379 of 1398 bases (98%) identical to a gb:GENBANK-ID:HSGABAAS|acc:X15376.1 mRNA from Homo sapiens (Human mRNA for GABA-A receptor, gamma 2 subunit). Public nucleotide databases include all GenBank databases and the GeneSeq patent database.

The disclosed NOV21 polypeptide (SEQ ID NO:52) encoded by SEQ ID NO:51 has 475 amino acid residues and is presented in Table 21B using the one-letter amino acid code. Signal P, Psort and/or Hydropathy results predict that NOV21 has a signal peptide and is likely to be localized at the plasma membrane with a certainty of 0.6000. The most likely cleavage site for a NOV21 peptide is between amino acids 39 and 40.

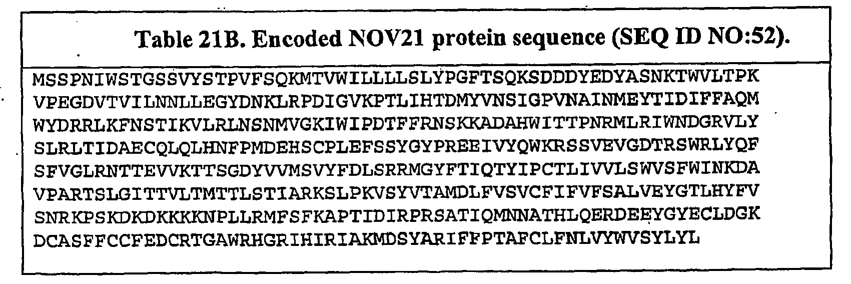

A search of sequence databases reveals that the NOV21 amino acid sequence has 467 of 475 amino acid residues (98%) identical to, and 467 of 475 amino acid residues (98%) similar to, the 467 amino acid residue ptnr:SWISSNEW-ACC:P18507 protein from Homo sapiens (Human) (GAMMA-AMINOBUTYRIC-ACID RECEPTOR GAMMA-2 SUBUNIT PRECURSOR (GABA(A) RECEPTOR)). Public amino acid databases include the GenBank databases, SwissProt, PDB and PIR.

NOV21 is expressed in at least Adrenal Gland/Suprarenal gland, Brain, Hippocampus, Pituitary Gland, and Right Cerebellum. Expression information was derived from the tissue sources of the sequences that were included in the derivation of the sequence of CG57654-01. The sequence is predicted to be expressed in the following tissues because of the expression pattern of (GENBANK-ID: gb:GENBANK-ID:HSGABAAS|acc:X15376.1) a closely related Human mRNA for GABA-A receptor, gamma 2 subunit homolog in species Homo sapiens: fetal brain. This information was derived by determining the tissue sources of the sequences that were included in the invention including but not limited to SeqCalling sources, Public EST sources, Literature sources, and/or RACE sources.

The disclosed NOV21 polypeptide has homology to the amino acid sequences shown in the BLASTP data listed in Table 21C.

| **Table 21C. BLAST results for NOV21** | | | | | |
|---|---|---|---|---|---|
| Gene Index/ Identifier | Protein/ Organism | Length (aa) | Identity (%) | Positives (%) | Expect |
| gi\|4557611\|ref\|NP 0 00807.1\| (NM_000816) | gamma-aminobutyric acid A receptor, gamma 2 precursor [Homo sapiens] | 467 | 467/475 (98%) | 467/475 (98%) | 0.0 |
| gi\|573B138\|gb\|AAD50 273.1 (AF165124) | gamma-aminobutyric acid A receptor gamma 2 [Homo sapiens] | 467 | 465/475 (97%) | 466/475 (97%) | 0.0 |
| gi\|120784\|sp\|P22300 GAC2 BOVIN | GAMMA-AMINOBUTYRIC-ACID RECEPTOR GAMMA-2 SUBUNIT PRECURSOR (GABA(A) RECEPTOR) | 475 | 467/475 (98%) | 470/475 (98%) | 0.0 |
| gi\|108682\|pir\|\|B392 72 | gamma-aminobutyric acid receptor A gamma-2L chain - bovine | 475 | 467/475 (98%) | 470/475 (98%) | 0.0 |
| gi \| 6679915 \| ref \| NP 0 32099.1 (NM_008073) | gamma-aminobutyric acid (GABA-A) receptor, subunit gamma 2 [Mus musculus] | 474 | 469/475 (98%) | 471/475 (98%) | 0.0 |

Table 21D lists the domain descriptions from DOMAIN analysis results against NOV21. This indicates that the NOV21 sequence has properties similar to those of other proteins known to contain this domain.

| **Table 21D. Domain Analysis ofNOV21** | | |
|---|---|---|
| gn \|Pfam\|pfam02931, Neur_chan_LBD, Neurotransmitter-gated ion-channel ligand binding domain. This family is the extracellular ligand binding domain of these ion channels. This domain forms a pentameric arrangement in the known structure. | | |
| | CD-Length = 200 residues, 91.5% aligned | |
| | | Score = 170 bits (431), Expect = 1e-43 |

Neurotransmission effected by GABA (gamma-aminobutyric acid) is predominantly mediated by a gated chloride channel intrinsic to the GABAA receptor. This heterooligomeric receptor exists in most inhibitory synapses in the vertebrate central nervous system (CNS) and can be regulated by clinically important compounds such as benzodiazepines and barbiturates. The primary structures of GABAA receptor alpha- and beta-subunits have been deduced from cloned complementary DNAs. Co-expression of these subunits in heterologous systems generates receptors which display much of the pharmacology of their neural counterparts, including potentiation by barbiturates. Conspicuously, however, they lack binding sites for, and consistent electrophysiological responses to, benzodiazepines. (See Pritchett et al. *Nature* 1989;338:582-5) reported the isolation of a cloned cDNA encoding a new GABAA receptor subunit, termed gamma 2, which shares approximately 40% sequence identity with alpha- and beta-subunits and whose messenger RNA is prominently localized in neuronal subpopulations throughout the CNS. Importantly, coexpression of the gamma 2 subunit with alpha 1 and beta 1 subunits produces GABAA receptors displaying high-affinity binding for central benzodiazepine receptor ligands.

The disclosed NOV21 nucleic acid of the invention encoding a gamma-aminobutyric acid receptor-like protein includes the nucleic acid whose sequence is provided in Table 21A or a fragment thereof. The invention also includes a mutant or variant nucleic acid any of whose bases may be changed from the corresponding base shown in Table 21A while still encoding a protein that maintains its gamma-aminobutyric acid receptor-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 2 percent of the bases may be so changed.

The disclosed NOV21 protein of the invention includes the gamma-aminobutyric acid receptor-like protein whose sequence is provided in Table 21B. The invention also includes a mutant or variant protein any of whose residues may be changed from the corresponding residue shown in Table 21B while still encoding a protein that maintains its gamma-aminobutyric acid receptor-like activities and physiological functions, or a functional fragment thereof. In the mutant or variant protein, up to about 2 percent of the residues may be so changed.

The invention further encompasses antibodies and antibody fragments, such as F_{ab} or (F_{ab})₂, that bind immunospecifically to any of the proteins of the invention.

The above defined information for this invention suggests that this gamma-aminobutyric acid receptor-like protein (NOV21) may function as a member of a "gamma-aminobutyric acid receptor family". Therefore, the NOV21 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV21 nucleic acids and proteins of the invention are useful in potential therapeutic applications implicated in diseases including but not limited to various pathologies and disorders as indicated below. For example, a cDNA encoding the gamma-aminobutyric acid receptor-like protein (NOV21) may be useful in gene therapy, and the gamma-aminobutyric acid receptor-like protein (NOV21) maybe useful when administered to a subject in need thereof. By way of nonlimiting example, the compositions of the present invention will have efficacy for treatment of patients suffering from adrenoleukodystrophy, congenital adrenal hyperplasia, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, endocrine dysfunctions, diabetes, obesity, growth and reproductive disorders, or other pathologies or conditions. The NOV21 nucleic acid encoding the gamma-aminobutyric acid receptor-like protein of the invention, or fragments thereof, may further be useful in diagnostic applications, wherein the presence or amount of the nucleic acid or the protein are to be assessed.

NOV21 nucleic acids and polypeptides are further useful in the generation of antibodies that bind immuno-specifically to the novel NOV21 substances for use in therapeutic or diagnostic methods. These antibodies may be generated according to methods known in the art, using prediction from hydrophobicity charts, as described in the "Anti-NOVX Antibodies" section below. The disclosed NOV21 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV22

A disclosed NOV22 nucleic acid of 1602 nucleotides (also referred to as 57724-01) encoding a carboxylesterase-like protein is shown in Table 22A. The start and stop codons are in bold letters.

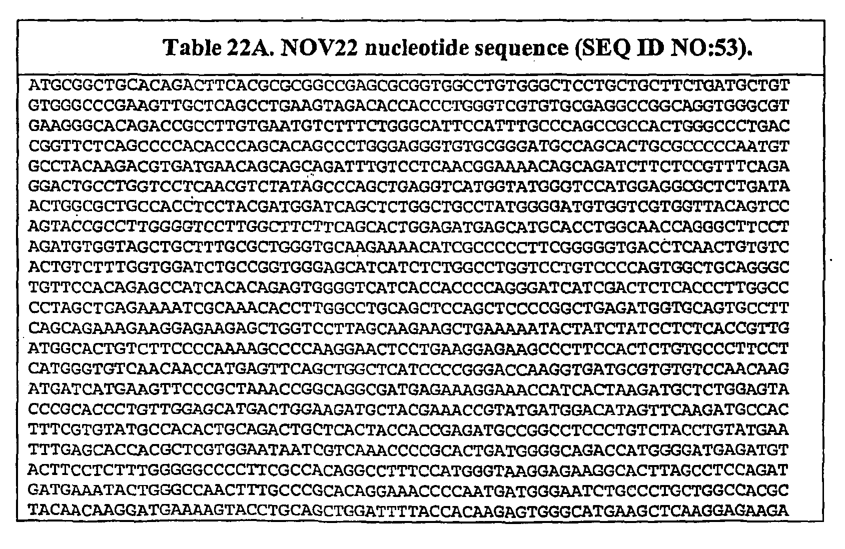

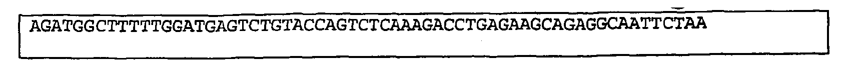

In a search of public sequence databases, the NOV22 nucleic acid sequence, located on chromsome 16 has 695 of 735 bases (94%) identical to a gb:GENBANK-ID:AK000105|acc:AK000105.1 mRNA from Homo sapiens (Homo sapiens cDNA FLJ20098 fis, clone COL04537, highly similar to ESTM_MOUSE LIVER CARBOXYLESTERASE PRECURSOR). Public nucleotide databases include all GenBank databases and the GeneSeq patent database.

The disclosed NOV22 polypeptide (SEQ ID NO:54) encoded by SEQ ID NO:53 has 533 amino acid residues and is presented in Table 22B using the one-letter amino acid code. Signal P, Psort and/or Hydropathy results predict that NOV22 has a signal peptide and is likely to be localized extracellularly with a certainty of 0.7953. The most likely cleavage site for a NOV22 peptide is at amino acid position 29.

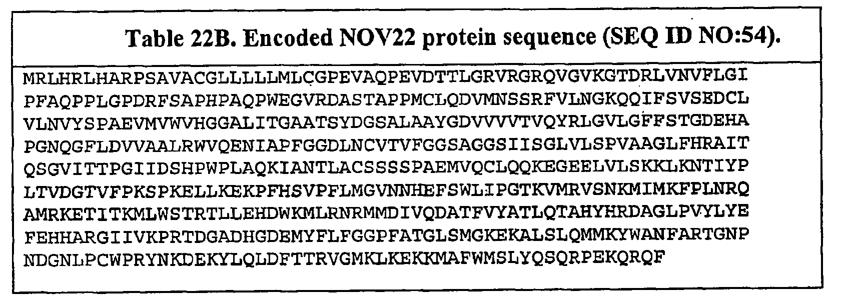

A search of sequence databases reveals that the NOV22 amino acid sequence has 296 of 544 amino acid residues (54%) identical to, and 373 of 544 amino acid residues (68%) similar to, the 554 amino acid residue ptnr:SWISSPROT-ACC:Q63880 protein from Mus musculus (Mouse) (LIVER CARBOXYLESTERASE PRECURSOR (EC 3.1.1.1) (ES-MALE) (ESTERASE-31))(.Public amino acid databases include the GenBank databases, SwissProt, PDB and PIR.

NOV22 is expressed in at least liver, colon, small intestine, kidney, pancrease, brain, and plasma. Expression information was derived from the tissue sources of the sequences that were included in the derivation of the sequence of CG57724-01. The sequence is predicted to be expressed in the following tissues because of the expression pattern of (GENBANK-ID: gb:GENBANK-ID:AK000105|acc:AK000105.1) a closely related Homo sapiens cDNA FLJ20098 fis, clone COL04537, highly similar to ESTM_MOUSE LIVER CARBOXYLESTERASE PRECURSOR homolog in species Homo sapiens : liver, colon, small intestine, kidney, pancrease, brain, and plasma. This information was derived by determining the tissue sources of the sequences that were included in the invention including but not limited to SeqCalling sources, Public EST sources, Literature sources, and/or RACE sources.

The disclosed NOV22 polypeptide has homology to the amino acid sequences shown in the BLASTP data listed in Table 22C.

| **Table 22C. BLAST results for NOV22** | | | | | |
|---|---|---|---|---|---|
| Gene Index/ Identifier | Protein/ Organism | Length (aa) | Identity (%) | Positives (%) | Expect |
| gi\|17512361\|gb\|AAH1 9147.1\|AAH19147 (BC019147) | protein for MGC:29382) [Mus musculus] | 568 | 293/547 (53%) | 370/547 (67%) | e-156 |
| "gi 2494382 sp Q6388 LIVER O\|ESTM MOUSE | LIVER CARBOXYLESTERASE PRECURSOR (ES-MALE) (ESTERASE-31) | 554 | 297/560 (53%) | 369/560 (65%) | e-155 |
| gi\|14789873\|gb\|AAH1 0812.1\|AAH10812 (BC010812) | (protein for IMAGE:4211034) [Mus musculus] | 524 | 272/538 (50%) | 346/538 (63%) | e-140 |
| gi\|730714\|sp\|Q04791 \|SASB ANAPL | FATTY ACYL-COA HYDROLASE PRECURSOR, MEDIUM CHAIN (THIOESTERASE B) | 557 | = 252/547 (46%) | 346/547 (63%) | e-136 |
| gi\|57554\|emb\|CAA463 91.1\| (X65296 | carboxylesterase [Rattus rattus] | 565 | (42%) | 336/559 (59%) | e-124 |

Table 22D lists the domain descriptions from DOMAIN analysis results against NOV22. This indicates that the NOV22 sequence has properties similar to those of other proteins known to contain this domain.

| **Table 22D. Domain Analysis of NOV22** |
|---|
| gnl\|Pfam\|pfam00135, COesterase, carboxylesterase |
| CD-Length = 532 residues, 93.0% aligned |
| Score = 374 bits (960), Expect = 8e-105 |

The mammalian carboxylesterases (EC 3.1.1.1) comprise a multigene family, the gene products of which are localized in the endoplasmic reticulum (ER) and cytosol of many tissues. These enzymes efficiently catalyze the hydrolysis of a variety of ester- and amide-containing chemicals, as well as drugs (including prodrugs) to the respective free acids. They are involved in detoxification or metabolic activation of various drugs, environmental toxicants, and carcinogens. Carboxylesterases also catalyze the hydrolysis of endogenous compounds such as short- and long-chain acylglycerols, long-chain acylcarnitine, and long-chain acyl-CoA esters. Multiple isozymes of hepatic microsomal carboxylesterases exist in various animal species, and some of these isozymes are involved in the metabolic activation of certain carcinogens and are associated with hepatocarcinogenesis.

Several studies have shown that various carboxylesterases are present in a wide variety of organs and tissues of many mammalian species; the highest hydrolase activity occurs in the liver. Humans express carboxylesterase in the liver, small intestine, brain, stomach, colon, pancreas, kidney, macrophages, monocytes, and plasma. Carboxylesterases, in addition to the metabolism of exogenous compounds, have been shown to hydrolyze endogenous fatty acid esters of steroids in both rat pancreas and kidney. The nonspecific esterases found in brain appear to be present only in the central nervous system, and four unique carboxylesterases have been isolated from human brain extract. Carboxylesterase activity of is found predominantly in the microsomal fraction, although significant carboxylesterase activity is present in the lysosomal fraction, and the lysosomes contribute substantially to the general esterolytic capacity of liver. The microsomal and lysosomal enzymes can be differentiated on the basis of both substrate specificity and structure and are considered to belong to separate classes. Carboxylesterase activity is also found in the cytosolic fraction of brain and in the plasma. Carboxylesterase is present in the plasma, but it is most likely syn-thesized in liver and then secreted into the circulation via the Golgi apparatus.

Human liver and plasma carboxylesterase activates lovastatin, and there are a significant number of additional drugs and endogenous compounds that are substrates of carboxylesterases, e.g. dipivefrin hydrochloride, carbonates, cocaine, salicylates, capsaicin, , palmitoyl-coenzyme A, haloperidol, imidapril, pyrrolizidine alkaloids, and steroids.

The disclosed NOV22 nucleic acid of the invention encoding a carboxylesterase-like protein includes the nucleic acid whose sequence is provided in Table 22A or a fragment thereof. The invention also includes a mutant or variant nucleic acid any of whose bases may be changed from the corresponding base shown in Table 22A while still encoding a protein that maintains its carboxylesterase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 6 percent of the bases may be so changed.

The disclosed NOV22 protein of the invention includes the carboxylesterase-like protein whose sequence is provided in Table 22B. The invention also includes a mutant or variant protein any of whose residues may be changed from the corresponding residue shown in Table 22B while still encoding a protein that maintains its carboxylesterase-like activities and physiological functions, or a functional fragment thereof. In the mutant or variant protein, up to about 46 percent of the residues may be so changed.

The invention further encompasses antibodies and antibody fragments, such as F_{ab} or (F_{ab})₂, that bind immunospecifically to any of the proteins of the invention.

The above defined information for this invention suggests that this carboxylesterase-like protein (NOV22) may function as a member of a "carboxylesterase family". Therefore, the NOV22 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV22 nucleic acids and proteins of the invention are useful in potential therapeutic applications implicated in diseases including but not limited to various pathologies and disorders as indicated below. For example, a cDNA encoding the carboxylesterase-like protein (NOV22) may be useful in gene therapy, and the carboxylesterase-like protein (NOV22) may be useful when administered to a subject in need thereof. By way of nonlimiting example, the compositions of the present invention will have efficacy for treatment of patients suffering from hepatocarcinoma, as well as other diseases, disorders and conditions, along with patients receiving pharmacotherapy with drug classes known to be metabolized by carboxylesterases, such as salicylates, carbonates, pyrrolizidine alkaloids, and steroids, or other pathologies or conditions. The NOV22 nucleic acid encoding the carboxylesterase-like protein of the invention, or fragments thereof, may further be useful in diagnostic applications, wherein the presence or amount of the nucleic acid or the protein are to be assessed.

NOV22 nucleic acids and polypeptides are further useful in the generation of antibodies that bind immuno-specifically to the novel NOV22 substances for use in therapeutic or diagnostic methods. These antibodies may be generated according to methods known in the art, using prediction from hydrophobicity charts, as described in the "Anti-NOVX Antibodies" section below. The disclosed NOV22 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV23

A disclosed NOV23 nucleic acid of 996 nucleotides (also referred to as CG57730-01) encoding a MAT-1-like protein is shown in Table 23A. The start and stop codons are in bold letters.

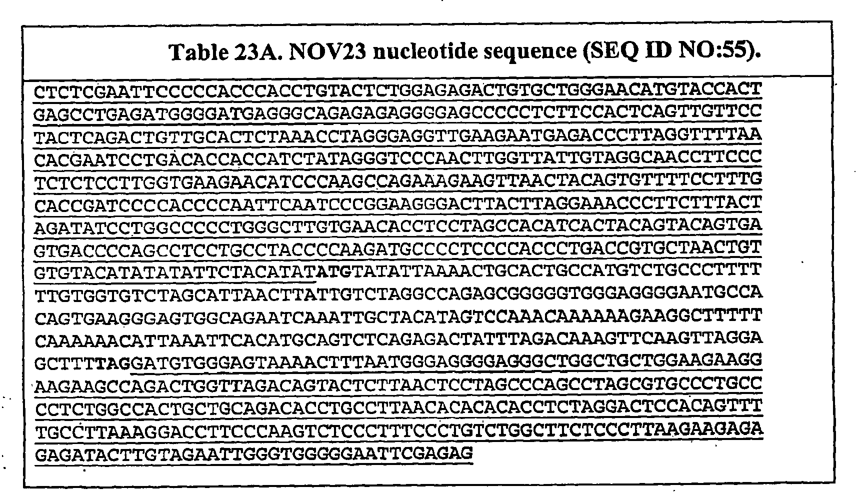

In a search of public sequence databases, the NOV23 nucleic acid sequence, located on chromsome 1q21.1 has 983 of 987 bases (99%) identical to a gb:GENBANK-ID:PEAGENE3|acc:AF153274.1 mRNA from Homo sapiens (Homo sapiens PEA15 protein (PEA15) gene, exons 3 and 4 and complete cds). Public nucleotide databases include all GenBank databases and the GeneSeq patent database.

The disclosed NOV23 polypeptide (SEQ ID NO:56) encoded by SEQ ID NO:55 has 74 amino acid residues and is presented in Table B using the one-letter amino acid code. Signal P, Psort and/or Hydropathy results predict that NOV23 has a signal peptide and is likely to be localized in the endoplasmic reticulum with a certainty of 0.5500. The most likely cleavage site for a NOV23 peptide is between amino acids 18 and 19.

| **Table 23B. Encoded NOV23 protein sequence (SEQ ID NO:56).** |
|---|
| MYIKTALPCILPFFWSSINLLSRPERGWEGNATVKGVAESNCYIVQTKKKAFSKNIKFTC SLRDYLDKVQVRSF |

A search of sequence databases reveals that the NOV23 amino acid sequence has 62 of 75 amino acid residues (82%) identical to, and 66 of 75 amino acid residues (88%) similar to, the 75 amino acid residue ptnr:SPTREMBL-ACC:Q14801 protein from Homo sapiens (Human) (HYPOTHETICAL 8.6 KDA PROTEIN). Public amino acid databases include the GenBank databases, SwissProt, PDB and PIR.

NOV23 is expressed in at least ovary, testis, brain, amygdala, pancreas, colon, and stomach.. This information was derived by determining the tissue sources of the sequences that were included in the invention including but not limited to SeqCalling sources, Public EST sources, Literature sources, and/or RACE sources.

The disclosed NOV23 polypeptide has homology to the amino acid sequences shown in the BLASTP data listed in Table 23C.

| **Table 23C. BLAST results for NOV23** | | | | | |
|---|---|---|---|---|---|
| Gene Index/ Identifier | Protein/ Organism | Length (aa) | Identity (%) | Positives (%) | Expect |
| gi\|14714659\|gb\|AAH1 0469.1\|AAH10469 (BC010469) | Similar to homolog of mouse MAT-1 oncogene [Homo sapiens] | 74 | 74/74 (100%) | 74/74 (100%) | 1e-34 |
| gi\|7019425\|ref\|NP 0 37419.1\| (NM_013287) | homolog of mouse MAT-1 oncogene; phosphoprotein enriched in astrocytes, 15kD [Homo sapiens] | 75 | 62/75 (82%) | 66/75 (87%), | 5e-27 |
| gi\|6678812\|ref\|NP 0 32582.1\| (NM_008556) | phosphoprotein enriched in astrocytes 15; mammary transforming gene 1 [Mus musculus] | 61 | 27/30 (90%) | 27/30 (90%) | 8e-9 |

An efficient in vitro transformation system has been developed using N-methyl-N-nitrosourea that allows the role of hormones and growth factors in mouse mammary tumorigenesis to be studied. Utilizing this system, it was reported that mammary tumors induced in vitro with N-methyl-N-nitrosourea in the presence of mammogenic hormones (progesterone and prolactin) contain predominately an activated c-Ki-ras protooncogene with a G35 --> A35 transitional mutation in the 12th codon. Mammary tumors induced in the presence of another mitogen, lithium (Li), do not have a mutation in the c-Ki-ras protooncogene: By using an expression cloning system, a plasmid clone containing a 1.75-kb cDNA insert has been isolated from this group of tumors. Nucleic acid sequence analysis of the insert reveals that it has a short open reading frame of 61 amino acids and that it does not have sequence homology with any known gene. The gene, designated MAT1, can neoplastically transform NIH 3T3 cells and also the mammary epithelial cell line TM3. Expression of this gene occurs in normal mouse tissues including mammary gland and is overexpressed in the original mammary tumors as indicated by Northern blot analysis. In vitro transcription and translation of the clone shows a protein product of 6000 Da, which agrees with the predicted open reading frame.

The disclosed NOV23 nucleic acid of the invention encoding a MAT-1-like protein includes the nucleic acid whose sequence is provided in Table 23A or a fragment thereof. The invention also includes a mutant or variant nucleic acid any of whose bases may be changed from the corresponding base shown in Table 23A while still encoding a protein that maintains its MAT-1-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as aritisense binding nucleic acids in therapeutic applications in a subject. In the mutant or'variant nucleic acids, and their complements, up to about 1 percent of the bases may be so changed.

The disclosed NOV23 protein of the invention includes the MAT-1-like protein whose sequence is provided in Table 23B. The invention also includes a mutant or variant protein any of whose residues may be changed from the corresponding residue shown in Table 23B while still encoding a protein that maintains its MAT-1-like activities and physiological functions, or a functional fragment thereof. In the mutant or variant protein, up to about 12 percent of the residues may be so changed.

The invention further encompasses antibodies and antibody fragments, such as F_{ab} or (F_{ab})_{2,} that bind immunospecifically to any of the proteins of the invention.

The above defined information for this invention suggests that this MAT-1-like protein (NOV23) may function as a member of a "MAT-1 family". Therefore, the NOV23 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue *regeneration in vivo* and in *vitro* of all tissues and cell types composing (but not limited to) those defined here.

The NOV23 nucleic acids and proteins of the invention are useful in potential therapeutic applications implicated in diseases including but not limited to various pathologies and disorders as indicated below. For example, a cDNA encoding the MAT-1-like protein (NOV23) may be useful in gene therapy, and the MAT-1-like protein (NOV23) may be useful when administered to a subject in need thereof. By way of nonlimiting example, the compositions of the present invention will have efficacy for treatment of patients suffering from Cataract, zonular pulverulent-1; MHC class II deficiency, complementation group C; cancer, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration; diabetes, pancreatitis, obesity; fertility, or other pathologies or conditions. The NOV23 nucleic acid encoding the MAT-1-like protein of the invention, or fragments thereof, may further be useful in diagnostic applications, wherein the presence or amount of the nucleic acid or the protein are to be assessed.

NOV23 nucleic acids and polypeptides are further useful in the generation of antibodies that bind immuno-specifically to the novel NOV23 substances for use in therapeutic or diagnostic methods. These antibodies may be generated according to methods known in the art, using prediction from hydrophobicity charts, as described in the "Anti-NOVX Antibodies" section below. The disclosed NOV23 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV24

A disclosed NOV24 nucleic acid of 668 nucleotides (also referred to as CG57755-01) encoding a vacuolar proton-ATPase subunit H-like protein is shown in Table 24A. The start and stop codons are in bold letters.

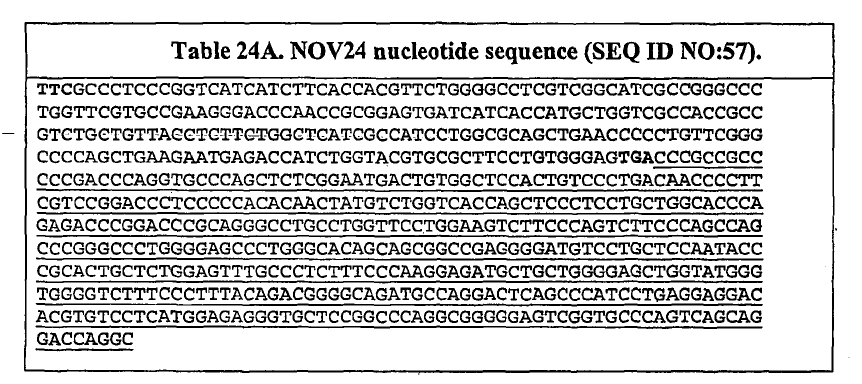

In a search of public sequence databases, the NOV24 nucleic acid sequence has 169 of 230 bases (73%) identical to a gb:GENBANK-ID:AF258614|acc:AF258614.1 mRNA from Canis familiaris (Canis familiaris vacuolar proton-ATPase subunit ATP6H (ATP6H) mRNA, complete cds). Public nucleotide databases include all GenBank databases and the GeneSeq patent database.

The disclosed NOV24 polypeptide (SEQ ID NO:58) encoded by SEQ ID NO:57 has 76 amino acid residues and is presented in Table 24B using the one-letter amino acid code. Signal P, Psort and/or Hydropathy results predict that NOV24 has a signal peptide and is likely to be localized in the plasma membrane with a certainty of 06400. The most likely cleavage site for a NOV24 peptide is between amino acids 53 and 54.

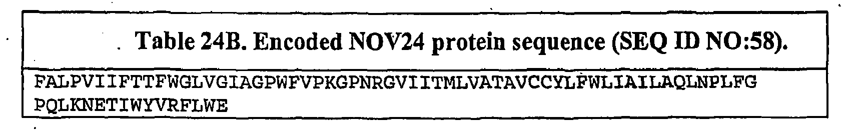

A search of sequence databases reveals that the NOV24 amino acid sequence has 56 of 73 amino acid residues (76%) identical to, and 64 of 73 amino acid residues (87%) similar to, the 81 amino acid residue ptnr:SPTREMBL-ACC:Q9N0Q1 protein from Canis familiaris (Dog) (VACUOLAR PROTON-ATPASE SUBUNIT ATP6H). Public amino acid databases include the GenBank databases, SwissProt, PDB and PIR.

The disclosed NOV24 polypeptide has homology to the amino acid sequences shown in the BLASTP data listed in Table 24C.

| **Table 24C. BLAST results for NOV24** | | | | | |
|---|---|---|---|---|---|
| Gene Index/ Identifier | Protein/ Organism | Length (aa) | Identity (%) | Positives (%) | Expect |
| gi\|14789787\|gb\|AAH1 0790.1\|AAH10790 (BC010790) | protein for MGC:18845) [Mus musculus] | 81 | 75/76 (98%) | 76/76 (99%) | 4e-28 |
| gi\|18600961\|ref\|XP 095170.1 (XM_095170) | protein XP_095170 [Homo sapiens] | 109 | 65/86 (75%) | 67/86 (77%) | 9e-22 |
| gi\|8050819\|gb\|AAF71 753.1\|AF258614 1 (AF258614) | vacuolar proton-ATPase subunit ATP6H [Canis familiaris] | 81 | 56/73 (76%) | 64/73 (86%) | 3e-21 , |
| gi\|13507387\|gb\|AAK2 8556.1\|AF343440 1 (AF343440) | lysosomal H+ transporting-ATPase subunit M9.2 [Canis familiaris] | 81 | 55/73 (75%) | 64/73 (87%) | 6e-21 |
| gi\|13384606\|ref\|NP 079548.11 (NM_D25272) | ATPase, H+ transporting lysosomal (vacuolar proton pump), 9.2 kDa [Mus musculus] | 81 | 55/73 (75%) | 65/73 (88%) | 8e-21 |

Vacuolar-type H(+)-ATPase (V-ATPase) is a multisubunit enzyme responsible for acidification of eukaryotic intracellular organelles. V-ATPase-dependent organelle acidification is essential for intracellular processes such as protein sorting, zymogen activation, and receptor-mediated endocytosis. The V-ATPase is composed of peripheral (V1) and integral (V0) membrane sectors. Proteolipids are major components of the V0 sector. In C. elegans, Oka et al. (See Oka, T. et al., J. Biol. Chem. 272: 24387-24392, 1997) identified the VHA1 and VHA2 genes, which encode 16-kD proteolipids, and the VHA4 gene, which encodes a 23-kD proteolipid product. Nishigori et al. (1998) isolated human cDNAs encoding a proteolipid which they designated 'ATP6F.' Sequence analysis revealed that the predicted 205-amino acid protein shares 61% identity with the S. cerevisiae proteolipid VMA16 and 67% identity with C. elegans VHA4. ATP6F contains 5 transmembrane segments and a conserved glutamic acid residue that is essential for proton transport activity in VMA16. As with ATP6C (108745), a 16-kD V-ATPase proteolipid, the N- and C-terminal halves of ATP6F share homology and may have resulted from a gene duplication event. The duplicated segments of ATP6F and ATP6C are 75% similar on the amino acid level. Northern blot analysis indicated that the 1.1-kb ATP6F mRNA was expressed in all tissues tested. The ATP6F gene contains 8 exons and spans approximately 4 kb. By FISH and radiation hybrid analysis, Nishigori et al. (1998) mapped the ATP6F gene to 1p32.3. (See Nishigori et al., Genomics 50: 222-228, 1998).

The vacuolar proton-ATPase (V-ATPase) is composed of an extramembrane catalytic sector and a transmembrane proton-conducting sector. See 603717. Ludwig et al. (1998) identified 2 novel proteins, 8-9 and 9.2 kD in size, in the membrane sector of bovine chromaffin granule V-ATPase. They designated the larger protein M9.2. By searching an EST database with the N-terminal sequence of bovine M9.2, Ludwig et al. (See Ludwig, et al., J. Biol. Chem. 273: 10939-10947, 1998) identified homologous cDNAs from human and mouse. The deduced 80-amino acid human M9.2 protein is extremely hydrophobic with 2 predicted membrane-spanning helices. Human and mouse M9.2 differed at only 1 amino acid position. Northern blot analysis revealed that M9.2 was present in all bovine tissues tested.

The disclosed NOV24 nucleic acid of the invention encoding a vacuolar proton-ATPase subunit H-like protein includes the nucleic acid whose sequence is provided in Table 24A or a fragment thereof. The invention also includes a mutant or variant nucleic acid any of whose bases may be changed from the corresponding base shown in Table 24A while still encoding a protein that maintains its vacuolar proton-ATPase subunit H-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 27 percent of the bases maybe so changed.

The disclosed NOV24 protein of the invention includes the vacuolar proton-ATPase subunit H-like protein whose sequence is provided in Table 24B. The invention also includes a mutant or variant protein any of whose residues may be changed from the corresponding residue shown in Table 24B while still encoding a protein that maintains its vacuolar proton-ATPase subunit H-like activities and physiological functions, or a functional fragment thereof. In the mutant or variant protein, up to about 24 percent of the residues may be so changed.

The invention further encompasses antibodies and antibody fragments, such as F_{ab} or (F_{ab})₂, that bind immunospecifically to any of the proteins of the invention.

The above defined information for this invention suggests that this vacuolar proton-ATPase subunit H-like protein (NOV24) may function as a member of a "vacuolar proton-ATPase subunit H family". Therefore, the NOV24 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV24 nucleic acids and proteins of the invention are useful in potential therapeutic applications implicated in diseases including but not limited to various pathologies and disorders as indicated below. For example, a cDNA encoding the vacuolar proton-ATPase subunit H-like protein (NOV24) may be useful in gene therapy, and the vacuolar proton-ATPase subunit H-like protein (NOV24) may be useful when administered to a subject in need thereof. By way of nonlimiting example, the compositions of the present invention will have efficacy for treatment of patients suffering from polycystic kidney disease I; osteopetrosis; mucolipidosis IV, or other pathologies or conditions. The NOV24 nucleic acid encoding the vacuolar proton-ATPase subunit H-like protein of the invention, or fragments thereof, may further be useful in diagnostic applications, wherein the presence or amount of the nucleic acid or the protein are to be assessed.

NOV24 nucleic acids and polypeptides are further useful in the generation of antibodies that bind immuno-specifically to the novel NOV24 substances for use in therapeutic or diagnostic methods. These antibodies may be generated according to methods known in the art, using prediction from hydrophobicity charts, as described in the "Anti-NOVX Antibodies" section below. The disclosed NOV24 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV25

A disclosed NOV25 nucleic acid of 5587 nucleotides (also referred to as CG57503-01) encoding a MEGF7-like protein is shown in Table 25A. The start and stop codons are in bold letters.

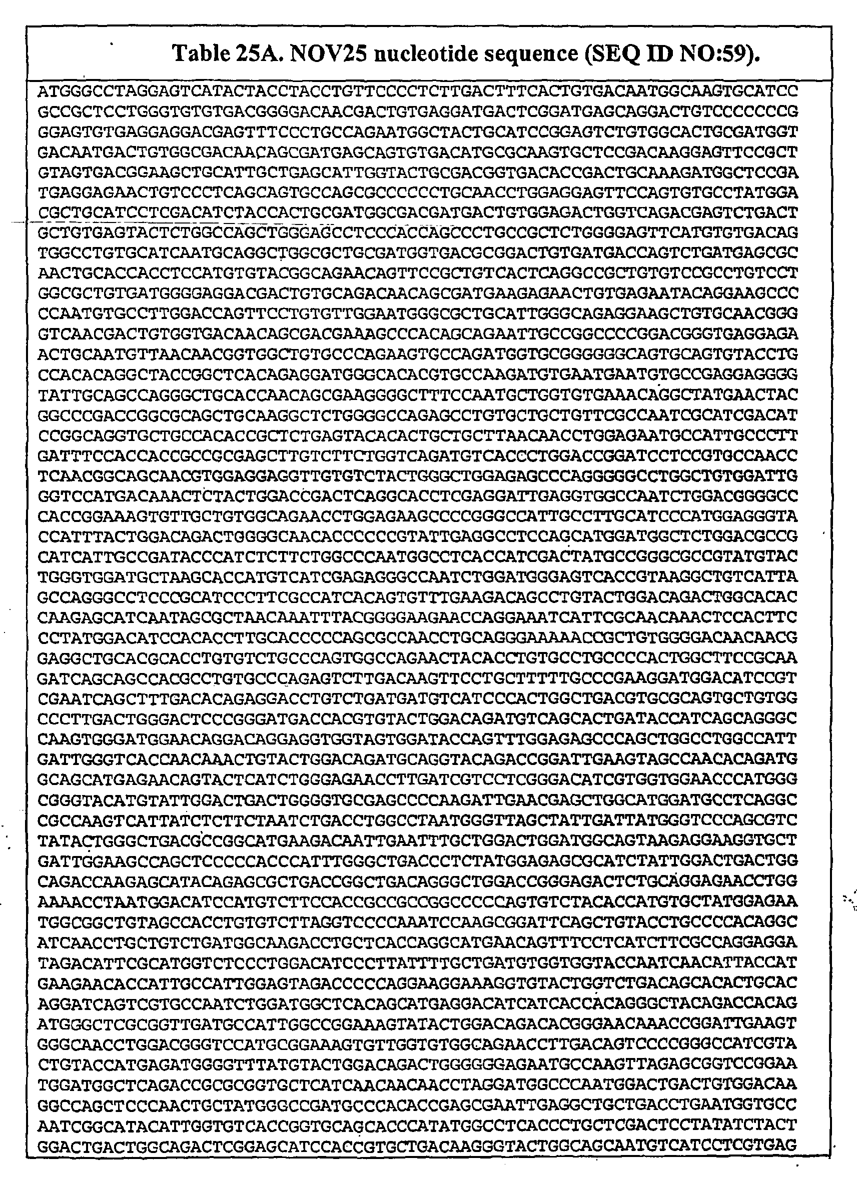

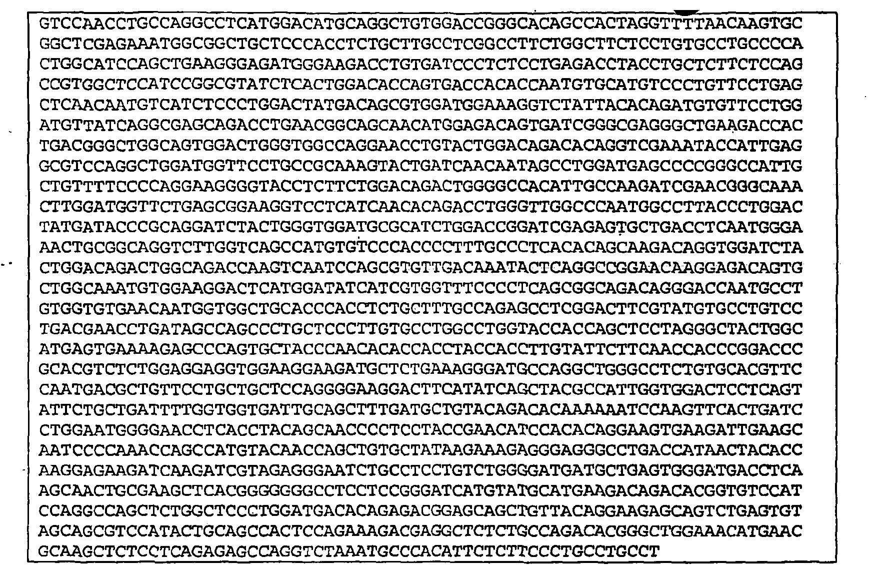

In a search of public sequence databases, the NOV25 nucleic acid sequence, located on chromsome 11 has 4754 of 4759 bases (99%) identical to a gb:GENBANK-ID:AB011540|acc:AB011540.1 mRNA from Homo sapiens (Homo sapiens mRNA for MEGF7, partial cds). Public nucleotide databases include all GenBank databases and the GeneSeq patent database.

The disclosed NOV25 polypeptide (SEQ ID NO:60) encoded by SEQ ID NO:59 has 1852 amino acid residues and is presented in Table 25B using the one-letter amino acid code. Signal P, Psort and/or Hydropathy results predict that NOV25 has no signal peptide and is likely to be localized at the plasma membrane with a certainty of 0.8200.

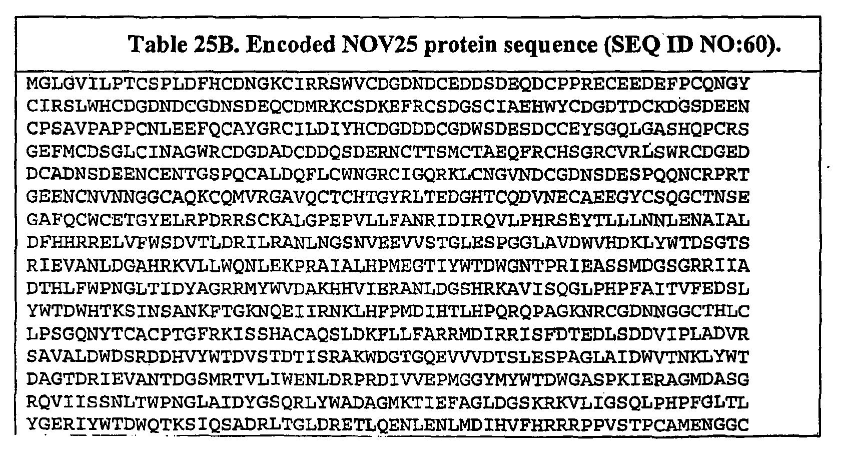

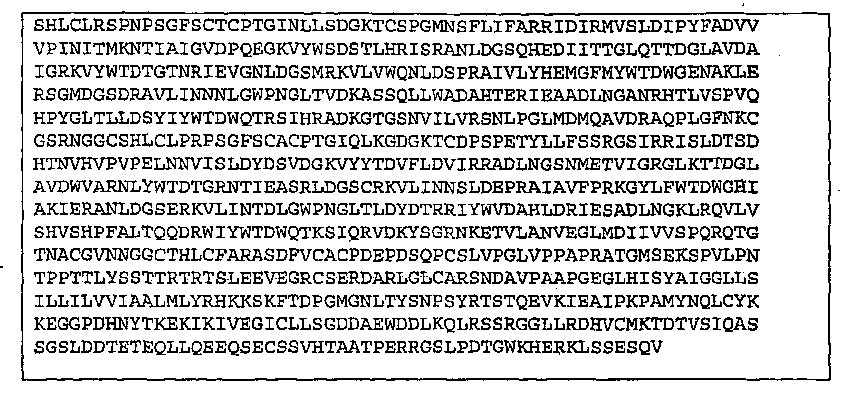

A search of sequence databases reveals that the NOV25 amino acid sequence has 1572 of 1576 amino acid residues (99%) identical to, and 1574 of 1576 amino acid residues (99%) similar to, the 1576 amino acid residue ptnr:SPTREMBL-ACC:O75096 protein from Homo sapiens (Human) (MEGF7). Public amino acid databases include the GenBank databases, SwissProt, PDB and PIR

NOV25 is expressed in at least adrenal gland/suprarenal gland, bone marrow, brain, bronchus, brown adipose, cartilage, cervix, colon, heart, hypothalamus, lung, peripheral blood, pituitary gland, spinal chord, stomach, testis, thalamus, uterus. This information was derived by determining the tissue sources of the sequences that were included in the invention including but not limited to SeqCalling sources, Public EST sources, Literature sources, and/or RACE sources.

The disclosed NOV25 polypeptide has homology to the amino acid sequences shown in the BLASTP data listed in Table 25C.

| **Table 25C. BLAST results for NOV25** | | | | | |
|---|---|---|---|---|---|
| Gene Index/ Identifier | Protein/ Organism | Length (aa) | Identity (%) | Positives (%) | Expect |
| gi\|17224416\|gb\|AAL 36970.1\| (AF247637) | LDLR dan [Mus musculus] | 1905 | 1779/1849 (96%) | 1809/1849 (97%), | 0.0 |
| gi\|3449306\|dbj\|BAA 32468.1\| (AB011540 | MEGF7 [Homo sapiens] | 1576 | 1572/1576 (99%) | 1574/1576 (99%) | 0.0 |
| gi\|6681362\|dbj\|BAA 88688.1\| (AB011533) | MEGF7 [Rattus norvegicus] | 1298 | 1248/1298 (96%) | 1274/1298 (98%) | 0.0 |
| gi \|17472590 \|ref \|XP 061753.1 (XM 061753) | similar to MEGF7 (H. sapiens) | 1007 | 992/992 (100%) | 992/992 (100%) | 0.0 |
| gi\|4763921\|ref\|XP 035037.1\| (XM_035037) | low density lipoprotein receptor-related protein 4 [Homo sapiens] | 859 | 857/859 (99%) | 859/859 (99%) | 0.0 |

Tables 25D-E list the domain descriptions from DOMAIN analysis results against NOV25. This indicates that the NOV25 sequence has properties similar to those of other proteins known to contain this domain.

| **Table 25D. Domain Analysis of NOV25** | |
|---|---|
| gnl \| Smart \| smart00192, LDLa, Low-density lipoprotein receptor domain class A; Cysteine-rich repeat in the low-density lipoprotein (LDL) receptor that plays a central role in mammalian cholesterol metabolism. The N-terminal type A repeats in LDL receptor bind the lipoproteins. Other-homologous domains occur in related receptors, including the very low-density lipoprotein receptor and the LDL receptor-related protein/alpha 2-macroglobulin receptor, and in proteins which are functionally unrelated, such as the C9 component of complement. Mutations in the LDL receptor gene cause familial hypercholesterolemia. | |
| | CD-Length = 38 residues, 97.4% aligned |
| | Score = 64.3 bits (155), Expect = 6e-11 |

| **Table 25E. Domain Analysis of NOV25** | | |
|---|---|---|
| gnl \| Smart \| smart00135, LY, Low-density lipoprotein-receptor YWTD domain; Type "B" repeats in low-density lipoprotein (LDL) receptor that plays a central role in mammalian cholesterol metabolism. Also present in a variety of molecules similar to gp300/megalin. | | |
| | CD-Length = 43 residues, 95.3% aligned | |
| | | Score = 62.0 bits (149), Expect = 3e-10 |

The domain that characterizes epidermal growth factor (EGF) consists of approximately 50 amino acids, and has been shown to be present, in a more or less conserved form, in a large number of other, mostly animal proteins. EGF-like domains are believed to play a critical role in a number of extracellular events, including cell adhesion and receptor-ligand interactions. Proteins with EGF-like domains often consist of more than 1,000 amino acids, have multiple copies of the EGF-like domain, and contain additional domains known to be involved in specific protein-protein interactions. The list of proteins currently known to contain one or more copies of an EGF-like pattern is large and varied. The functional significance of EGF domains in what appear to be unrelated proteins is not yet clear. However, a common feature is that these repeats are found in the extracellular domain of membrane-bound proteins or in proteins known to be secreted (exception: prostaglandin G/H synthase). The EGF domain includes six cysteine residues which have been shown (in EGF) to be involved in 3 disulfide bonds. The main structure is a two-stranded beta-sheet followed by a loop to a C-terminal short two-stranded sheet. Subdomains between the conserved cysteines vary in length.

To identify proteins containing EGF-like domains, Nakayama et al. (1998) searched a database of long cDNA sequences randomly selected from a human brain cDNA library for those that encode an EGF-like motif. They identified several partial cDNAs encoding novel proteins with EGF-like domains, such as LRP4, which they named MEGF7. The predicted partial LRP4 protein contains 2 EGF-like domains, a calcium binding-type EGF-like domain, 3 LDL receptor-type EGF-like domains, 4 YWTD spacer regions, a transmembrane domain, a cytoplasmic NPXY motif, which is required for clustering and internalization of LDL receptors, and a cytoplasmic tSXV motif, which anchors proteins with a PDZ domain. The sequence and domain organization of LRP4 shows significant similarities to those of members of the LDL receptor family. Northern blot analysis detected rat Megf7 expression in several regions of the brain. Using a radiation hybrid mapping panel, Nakayama et al. (1998) mapped the LRP4 gene to 11p12-p11.2.

The disclosed NOV25 nucleic acid of the invention encoding a MEGF7-like protein includes the nucleic acid whose sequence is provided in Table 25A or a fragment thereof. The invention also includes a mutant or variant nucleic acid any of whose bases may be changed from the corresponding base shown in Table 25A while still encoding a protein that maintains its MEGF7-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1 percent of the bases may be so changed.

The disclosed NOV25 protein of the invention includes the MEGF7-like protein whose sequence is provided in Table 25B. The invention also includes a mutant or variant protein any of whose residues may be changed from the corresponding residue shown in Table 25B while still encoding a protein that maintains its MEGF7-like activities and physiological functions, or a functional fragment thereof. In the mutant or variant protein, up to about 1 percent of the residues may be so changed.

The invention further encompasses antibodies and antibody fragments, such as F_{ab} or (F_{ab})₂. that bind immunospecifically to any of the proteins of the invention.

The above defined information for this invention suggests that this MEGF7-like protein (NOV25) may function as a member of a "MEGF7 family". Therefore, the NOV25 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo and in vitro* of all tissues and cell types composing (but not limited to) those defined here.

The NOV25 nucleic acids and proteins of the invention are useful in potential therapeutic applications implicated in diseases including but not limited to various pathologies and disorders as indicated below. For example, a cDNA encoding the MEGF7-like protein (NOV25) may be useful in gene therapy, and the MEGF7-like protein (NOV25) may be useful when administered to a subject in need thereof. By way of nonlimiting example, the compositions of the present invention will have efficacy for treatment of patients suffering from adrenoleukodystrophy, congenital adrenal hyperplasia, hemophilia, hypercoagulation, idiopathic thrombocytopenic purpura, autoimmume disease, allergies, immunodeficiencies, transplantation, graft vesus host; diseases of the brain and nervous system, including Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neuroprotection; diseases of the respiratory system, including systemic lupus erythematosus , autoimmune disease, asthma, emphysema, scleroderma, allergy, ARDS; diseases and disorders of adipose tissue, reproductive system, colon, circulatory system, spinal chord, digestive system, and endocrine system, or other pathologies or conditions. The NOV25 nucleic acid encoding the MEGF7-like protein of the invention, or fragments thereof, may further be useful in diagnostic applications, wherein the presence or amount of the nucleic acid or the protein are to be assessed.

NOV25 nucleic acids and polypeptides are further useful in the generation of antibodies that bind immuno-specifically to the novel NOV25 substances for use in therapeutic or diagnostic methods. These antibodies may be generated according to methods known in the art, using prediction from hydrophobicity charts, as described in the "Anti-NOVX Antibodies" section below. The disclosed NOV25 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV26

A disclosed NOV26 nucleic acid of 635 nucleotides (also referred to as CG57456-01) encoding a COP-Coated Vesicle Membrane Protein P24 Precursor-like protein is shown in Table 26A. The start and stop codons are in bold letters.

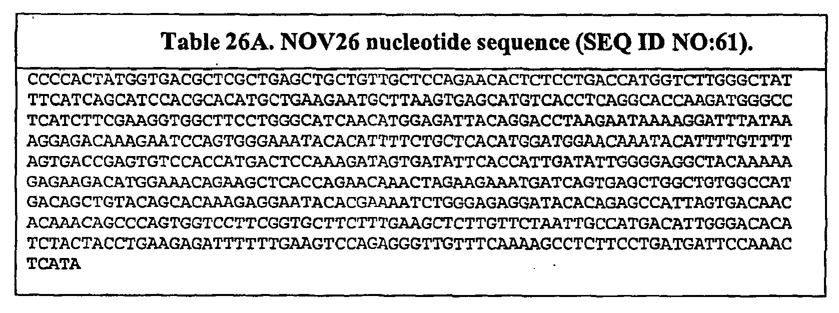

In a search of public sequence databases, the NOV26 nucleic acid sequence has 630 of 635 bases (99%) identical to a gb:GENBANK-ID:AF152363|acc:AF152363.1 mRNA from Homo sapiens (Homo sapiens constitutive fragile region FRA3B sequence). Public nucleotide databases include all GenBank databases and the GeneSeq patent database.

The disclosed NOV26 polypeptide (SEQ ID NO:62) encoded by SEQ ID NO:61 has 203 amino acid residues and is presented in Table 26B using the one-letter amino acid code. Signal P, Psort and/or Hydropathy results predict that NOV26 has a signal peptide and is likely to be localized at the plasma membrane with a certainty of 0.4600. The most likely cleavage site for a NOV26 peptide is between amino acids 29 and 30.

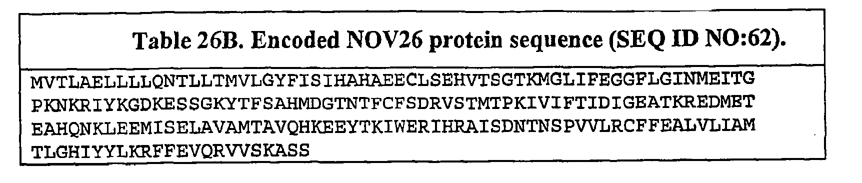

A search of sequence databases reveals that the NOV26 amino acid sequence has 156 of 201 amino acid residues (77%) identical to, and 175 of 201 amino acid residues (87%) similar to, the 201 amino acid residue ptnr:SWISSNEW-ACC:Q15363 protein from Homo sapiens (Human) (COP-COATED VESICLE MEMBRANE PROTEIN P24 PRECURSOR (P24A) (RNP24)). Public amino acid databases include the GenBank databases, SwissProt, PDB and PIR.

NOV26 is expressed in at least Eye, placenta, colon, and ovary. This information was derived by determining the tissue sources of the sequences that were included in the invention including but not limited to SeqCalling sources, Public EST sources, Literature sources, and/or RACE sources.

The disclosed NOV26 polypeptide has homology to the amino acid sequences shown in the BLASTP data listed in Table 26C.

| **Table 26C. BLAST results for NOV26** | | | | | |
|---|---|---|---|---|---|
| Gene Index/ Identifier | Protein/ Organism | Length (aa) | Identity (%) | Positives (%) | Expect |
| gi\|17440558\|ref\|XP 067504. 1\|(XM_067504) | similar to coated vesicle membrane protein [Homo sapiens] | 271 | 186/192 (96%) | 186/192 (96%) | e-100 |
| gi 9790015 ref NP 062744.1 (NM_019770) | coated vesicle membrane protein; Sid394p [Mus musculus] | 201 | 155/201 (77%) | 173/201 (85%), | 4-74 |
| gi\|1352660\|sp\|P49020\|P24 CRIGR | Cop-coated vesicle membrane protein p24 precursor | 196 | 148/196 (75%) | 169/196 (85%), | 6-74 |
| gi\|5803149\|ref\|NP 006806.1 (NM_006815) | coated vesicle membrane protein [Homo sapiens] | 201 | 156/201 (77%) | 175/201 (86%), | e-73 |
| gi\|13929014\|ref\|NP 113910.1\| (NM_031722 | coated vesicle membrane protein [Rattus norvegicusl] | 201 | 155/201 (77%) | 174/201 (86%), | e-73 |

Table 26D lists the domain descriptions from DOMAIN analysis results against NOV26. This indicates that the NOV26 sequence has properties similar to those of other proteins known to contain this domain.

| **Table 26D. Domain Analysis of NOV26** |
|---|
| gnl Pfam pfam01105, EMP24_GP25L, emp24/gp25L/p24 family. Members of this family are implicated in bringing cargo forward from the ER and binding to coat proteins by their cytoplasmic domains. |
| CD-Length = 202 residues, 92.6% aligned |
| Score = 135 bits (341), Expect = 2e-33 |

Members of the p24 family of putative cargo receptors are proposed to contain retrograde and anterograde trafficking signals in their cytoplasmic domain to facilitate coat protein binding and cycling in the secretory pathway. The localization and transit of the wild-type chimera from the endoplasmic reticulum (ER) through the Golgi complex involved a glutamic acid residue and a conserved glutamine in the TMD. The TMD glutamic acid mediated the localization of the chimeras to the ER in the absence of the conserved glutamine. Efficient ER exit required the TMD glutamine and was further facilitated by a pair of phenylalanine residues in the cytoplasmic tail. TMD residues of p24 proteins may mediate the interaction with integral membrane proteins of the vesicle budding machinery to ensure p24 packaging into transport vesicles.

Blum et al. (1996) identified a 21-kD rat pancreatic microsomal membrane protein that they designated Tmp21. By probing a human brain cDNA library with a fragment of the rat sequence, they isolated a cDNA encoding human TMP21. The deduced 219-amino acid type I intracellular transmembrane protein contains a signal sequence and is predicted to be located in the lumen of the endoplasmic reticulum. Northern blot analysis detected a 1.4-kb TMP21 transcript. Immunoblot analysis showed that the rat Tmp21 protein is expressed predominantly in the microsomal fraction of pancreatic acinar cells. Horer et al. (1999) determined that a putative TMP21 isoform, TMP21-II, is a neutral pseudogene.

The disclosed NOV26 nucleic acid of the invention encoding a COP-Coated Vesicle Membrane Protein P24 Precursor-like protein includes the nucleic acid whose sequence is provided in Table 26A or a fragment thereof. The invention also includes a mutant or variant nucleic acid any of whose bases may be changed from the corresponding base shown in Table 26A while still encoding a protein that maintains its COP-Coated Vesicle Membrane Protein P24 Precursor-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or sclerosis, or other pathologies or conditions. The NOV26 nucleic acid encoding the COP-Coated Vesicle Membrane Protein P24 Precursor-like protein of the invention, or fragments thereof, may further be useful in diagnostic applications, wherein the presence or amount of the nucleic acid or the protein are to be assessed.

NOV26 nucleic acids and polypeptides are further useful in the generation of antibodies that bind immuno-specifically to the novel NOV26 substances for use in therapeutic or diagnostic methods. These antibodies may be generated according to methods known in the art, using prediction from hydrophobicity charts, as described in the "Anti-NOVX Antibodies" section below. The disclosed NOV16 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV27

A disclosed NOV27 nucleic acid of 1120 nucleotides (also referred to as CG57658-01) encoding a connexin-like protein is shown in Table 27A. The start and stop codons are in bold letters.

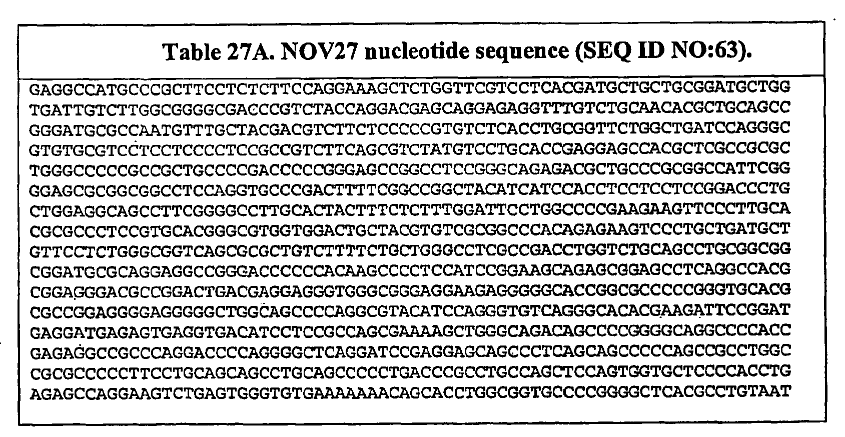

In a search of public sequence databases, the NOV27 nucleic acid sequence, located on chromsome 10 has 1037 of 1097 bases (94%) identical to a gb:GENBANK-ID:AB046017|acc:AB046017.1 mRNA from Macaca fascicularis (Macaca fascicularis brain cDNA, clone:QccE-15512). Public nucleotide databases include all GenBank databases and the GeneSeq patent database.

The disclosed NOV27 polypeptide (SEQ ID NO:64) encoded by SEQ ID NO:63 has 356 amino acid residues and is presented in Table 27B using the one-letter amino acid code. Signal P, Psort and/or Hydropathy results predict that NOV27 has a signal peptide and is likely to be localized in the plasma membranewith a certainty of 0.6400. The most likely cleavage site for a NOV27 peptide is between amino acids 26 and 27.

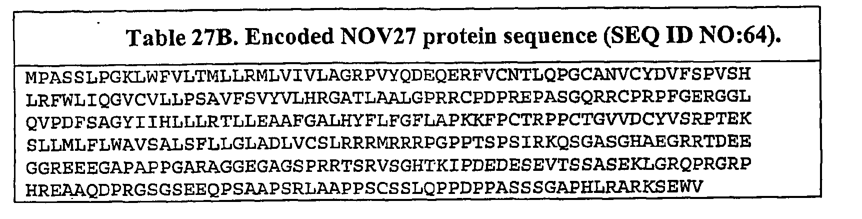

A search of sequence databases reveals that the NOV27 amino acid sequence has 348/348 (100%) identical to TREMBLNEW-ACC:CAC10186 BA425A6.2 (SIMILAR TO CONNEXIN) - Homo sapiens. Public amino acid databases include the GenBank databases, SwissProt, PDB and PIR.

NOV27 is expressed in at least Brain, Lung, Ovary, colon. This information was derived by determining the tissue sources of the sequences that were included in the invention including but not limited to SeqCalling sources, Public EST sources, Literature sources, and/or RACE sources.

The disclosed NOV27 polypeptide has homology to the amino acid sequences shown in the BLASTP data listed in Table 27C.

| **Table 27C. BLAST results for NOV27** | | | | | |
|---|---|---|---|---|---|
| Gene Index/ Identifier | Protein/ Organism | Length (aa) | Identity (%) | Positives (%) | Expect |
| gi\|17489790\|ref\|XP 05B368.1 (XM_058368) | similar to bA425A62 (similar to connexin) [Homo sapiens] | 370 | 349/352 (99%) | 351/352 (99%) | e-167 |
| gi\|10334641\|emb\|CAC 10186.11 (AL121749) | bA425A6.2 (similar to connexin) [Homo sapiens] | 348 | 348/348 (100%) | 348/348 (100%) | e-163 |
| gi\|9280090\|dbj\|BABO 1599.1\| (AB046017) | unnamed protein product [Macaca fascicularis] | 341 | 316/341 (92%) | 323/341 (94%) | e-159 |
| gi\|17489782\|ref\|XP 061277.1\| (XM 061277) | similar to connexin) [Homo sapiens] | 341 | 341/341 (100%) | 341/341 (100%) | e-158 |
| gi\|15990849\|emb\|CAC 93844.1\| (AJ414562 | connexin39 [Mus musculus] | 364 | 175/372 (47%) | 215/372 (57%), | 6e-67 |

Tables 27D-E list the domain descriptions from DOMAIN analysis results against NOV27. This indicates that the NOV27 sequence has properties similar to those of other proteins known to contain this domain.

| **Table 27D. Domain Analysis of NOV27** | | |
|---|---|---|
| gnl\|Pfam\|pfam00029, connexin, Connexin. | | |
| | CD-Length = 218 residues, 91.7% aligned | |
| | | Score = 172 bits (436), Expect = 3e-44 |

| **Table 27E. Domain Analysis of NOV27** | | |
|---|---|---|
| gnl\|Smart\|smart00037, CNX, Connexin homologues; Connexin channels participate in the regulation of signaling between developing and differentiated cell types. | | |
| | CD-Length = 34 residues, 97.1% aligned | |
| | | Score = 64.7 bits (156), Expect = 9e-12 |

Gap junctions were first characterized by electron microscopy as regionally specialized structures on plasma membranes of contacting adherent cells. These structures were shown to consist of cell-to-cell channels. Proteins, called connexins, purified from fractions of enriched gap junctions from different tissues differ. The connexins are designated by their molecular mass. Another system of nomenclature divides gap junction proteins into 2 categories, alpha and beta, according to sequence similarities at the nucleotide and amino acid levels. For example, CX43 is designated alpha-1 gap junction protein, whereas CX32 and CX26 are called beta-1 and beta-2 gap junction proteins, respectively. This nomenclature emphasizes that CX32 and CX26 are more homologous to each other than either of them is to CX43. The connexins are a family of integral membrane proteins that oligomerise to form intercellular channels that are clustered at gap junctions. These channels are specialised sites of cell-cell contact that allow the passage of ions, intracellular metabolites and messenger molecules (with molecular weight <1-2 kD) from the cytoplasm of one cell to its apposing neighbours. They are found in almost all vertebrate cell types, and somewhat similar proteins have been cloned from plant species. Invertebrates utilise a different family of molecules, innexins, that share a similar predicted secondary structure to the vertebrate connexins, but have no sequence identity to them. Vertebrate gap junction channels are thought to participate in diverse biological functions. For instance, in the heart they permit the rapid cell-cell transfer of action potentials, ensuring coordinated contraction of the cardiomyocytes. They are also responsible for neurotransmission at specialised 'electrical' synapses. In non-excitable tissues, such as the liver, they may allow metabolic cooperation between cells. In the brain, glial cells are extensively-coupled by gap junctions; this allows waves of intracellular Ca2+ to propagate through nervous tissue, and may contribute to their ability to spatially-buffer local changes in extracellular K+ concentration. The connexin protein family is encoded by at least 13 genes in rodents, with many homologues cloned from other species. They show overlapping tissue expression patterns, most tissues expressing more than one connexin type. Their conductances, permeability to different molecules, phosphorylation and voltage-dependence of their gating, have been found to vary. Possible communication diversity is increased further by the fact that gap junctions may be formed by the association of different connexin isoforms from apposing cells. However, in vitro studies have shown that not all possible combinations of connexins produce active channels. Hydropathy analysis predicts that all cloned connexins share a common transmembrane (TM) topology. Each connexin is thought to contain 4 TM domains, with two extracellular and three cytoplasmic regions. This model has been validated for several of the family members by in vitro biochemical analysis. Both N- and C-termini are thought to face the cytoplasm, and the third TM domain has an amphipathic character, suggesting that it contributes to the lining of the formed-channel. Amino acid sequence identity between the isoforms is ~50-80%, with the TM domains being well conserved. Both extracellular loops contain characteristically conserved cysteine residues, which likely form intramolecular disulphide bonds. By contrast, the single putative intracellular loop (between TM domains 2 and 3) and the cytoplasmic C-terminus are highly variable among the family members. Six connexins are thought to associate to form a hemi-channel, or connexon. Two connexons then interact (likely via the extracellular loops of their connexins) to form the complete gap junction channel.

The disclosed NOV27 nucleic acid of the invention encoding a connexin-like protein includes the nucleic acid whose sequence is provided in Table 27A or a fragment thereof. The invention also includes a mutant or variant nucleic acid any of whose bases may be changed from the corresponding base shown in Table 27A while still encoding a protein that maintains its connexin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 6 percent of the bases may be so changed.

The disclosed NOV27 protein of the invention includes the connexin-like protein whose sequence is provided in Table 27B. The invention also includes a mutant or variant protein any of whose residues may be changed from the corresponding residue shown in Table B while still encoding a protein that maintains its connexin-like activities and physiological functions, or a functional fragment thereof. In the mutant or variant protein, up to about 0 percent of the residues may be so changed.

The invention further encompasses antibodies and antibody fragments, such as F_{ab} or (F_{ab})₂, that bind immunospecifically to any of the proteins of the invention.

The above defined information for this invention suggests that this connexin-like protein (NOV27) may function as a member of a "connexin family". Therefore, the NOV27 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV27 nucleic acids and proteins of the invention are useful in potential therapeutic applications implicated in diseases including but not limited to various pathologies and disorders as indicated below. For example, a cDNA encoding the connexin-like protein (NOV27) may be useful in gene therapy, and the connexin-like protein (NOV27) may be useful when administered to a subject in need thereof. By way of nonlimiting example, the compositions of the present invention will have efficacy for treatment of patients suffering from Cardiomyopathy, Atherosclerosis,Hypertension, Congenital heart defects, Aortic stenosis ,Atrial septal defect (ASD),Atrioventricular (A-V) canal defect, Ductus arteriosus , Pulmonary stenosis , Subaortic stenosis, Ventricular septal defect (VSD), valve diseases,Tuberous sclerosis, Scleroderma, Obesity,Transplantation, Diabetes,Von Hippel-Lindau (VHL) syndrome, Pancreatitis, Obesity, Endometriosis,Fertility, Hemophilia, Hypercoagulation,Idiopathic thrombocytopenic purpura, Immunodeficiencies,Graft vesus host, Autoimmune disease, Renal artery stenosis, Interstitial nephritis, Glomerulonephritis, Polycystic kidney disease, Systemic lupus erythematosus, Renal tubular acidosis, IgA nephropathy, Hypercalceimia, Lesch-Nyhan syndrome, Von Hippel-Lindau (VHL) syndrome, Alzheimer's disease, Stroke, Tuberous sclerosis, hypercalceimia, Parkinson's disease, Huntington's disease, Cerebral palsy, Epilepsy,Lesch-Nyhan syndrome, Multiple sclerosis,Ataxia-telangiectasia,Leukodystrophies,Behavioral disorders, Addiction, Anxiety, Pain, Neuroprotection, or other pathologies or conditions. The NOV27 nucleic acid encoding the connexin-like protein of the invention, or fragments thereof, may further be useful in diagnostic applications, wherein the presence or amount of the nucleic acid or the protein are to be assessed.

NOV27 nucleic acids and polypeptides are further useful in the generation of antibodies that bind immuno-specifically to the novel NOV27 substances for use in therapeutic or diagnostic methods. These antibodies may be generated according to methods known in the art, using prediction from hydrophobicity charts, as described in the "Anti-NOVX Antibodies" section below. The disclosed NOV27 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV28

A disclosed NOV28 nucleic acid of 1234 nucleotides (also referred to as CG57662-01) encoding a -like protein is shown in Table 28A. The start and stop codons are in bold letters.

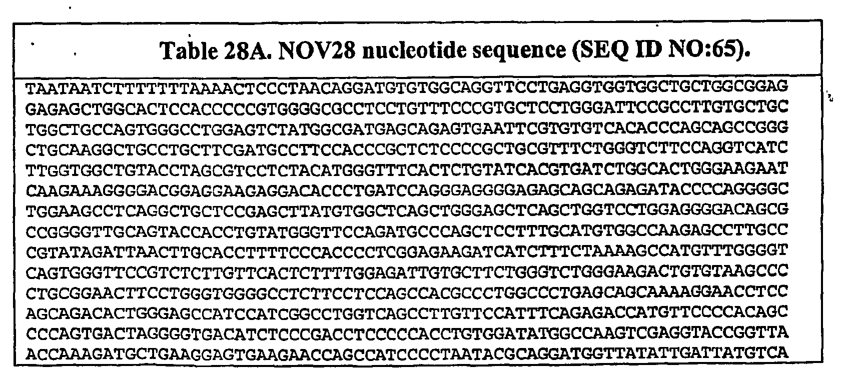

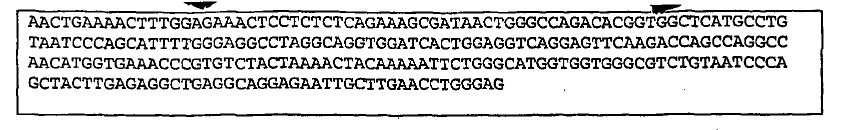

In a search of public sequence databases, the NOV28 nucleic acid sequence, located on chromsome 7 has 206 of 244 bases (84%) identical to a gb:GENBANK-ID:AP000692|acc:AP000692.1 mRNA from Homo sapiens (Homo sapiens genomic DNA, chromosome 21q22.2, PAC clone:24J14, CBR1-HLCS region). Public nucleotide databases include all GenBank databases and the GeneSeq patent database.

The disclosed NOV28 polypeptide (SEQ ID NO:66) encoded by SEQ ID NO:65 has 391 amino acid residues and is presented in Table 28B using the one-letter amino acid code. Signal P, Psort and/or Hydropathy results predict that NOV28 has a signal peptide and is likely to be localized in the plasma membrane with a certainty of 0.6000. The most likely cleavage site for a NOV28 peptide is between amino acids 46 and 47.

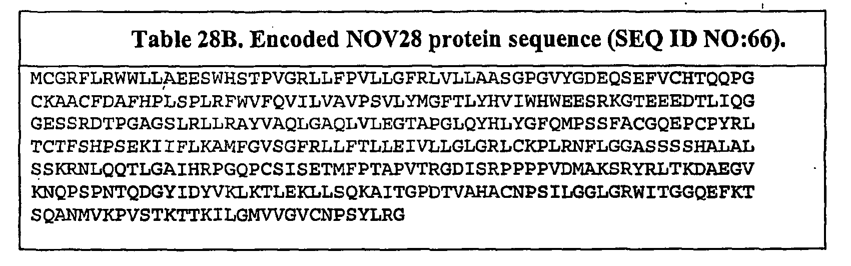

A search of sequence databases reveals that the NOV28 amino acid sequence has 108/108 (100%) amino acids identical with ptnr:SPTREMBL-ACC:060387 WUGSC:H_DJ0604G05.3 PROTEIN - Homo sapiens (Human). Public amino acid databases include the GenBank databases, SwissProt, PDB and PIR.

NOV28 is expressed in at least Brain, Breast, Colon, Gall bladder, Germ Cell, Heart, Kidney, Liver, Ovary, Pancreas, Prostate, Stomach, Testis, Whole embryo, brain, breast, breast-normal, colon, colon_ins, head_neck, lung, nervous_tumor, prostate, prostate_normal, prostate_tumor, stomach. This information was derived by determining the tissue sources of the sequences that were included in the invention including but not limited to SeqCalling sources, Public EST sources, Literature sources, and/or RACE sources.

The disclosed NOV28 polypeptide has homology to the amino acid sequences shown in the BLASTP data listed in Table 28C .

| **Table 28C. BLAST results for NOV28** | | | | | |
|---|---|---|---|---|---|
| Gene Index/ Identifier | Protein/ Organism | Length (aa) | Identity (%) | Positives (%) | Expect |
| gi\|3006230\|gb\|AAC09 485.1\| (AC004522 | gap junction protein; similar to P36383 (PID:g544117) [Homo sapiens] | 207 | 207/250 (82%) | 207/250 (82%) | e-103 |
| gi\|17978264\|ref\|NP 536698.1\| (NM_080450) | gap junction membrane channel protein epsilon 1; connexin 29 [Mus musculus] | 258 | 135/219 (61%) | 167/219 (75%) | 2e-75 |
| gi\|18566654\|ref\|XP 095131.1\| (XM 095131) | hypothetical protein XP_095131 [Homo sapiens] | 222 | 100/115 (86%) | 103/115 (88%) | 3e-51 |
| gi\|6680007\|ref\|NP 0 32148.1\| (NM_008122) | gap junction membrane channel protein alpha 7; connexin 45 [Mus musculus] | 396 | 96/250 (38%) | 136/250 (54%) | 5e-41 |
| gi\|4885169\|ref\|NP 0 05488.1 (NM_005497) | gap junction protein, alpha 7, 45kD (connexin 45) [Homo sapiens] | 396 | 96/250 (38%) | 136/250 (54%) | 8e-41 |

Table 28D lists the domain descriptions from DOMAIN analysis results against NOV28. This indicates that the NOV28 sequence has properties similar to those of other proteins known to contain this domain.

| **Table 28D. Domain Analysis of NOV28** | | |
|---|---|---|
| gnl\|Pfam\|pfam00029, connexin, Connexin. | | |
| | CD-Length = 218 residues, 90.4% aligned | |
| | | Score = 173 bits (438), Expect = 2e-44 |

The disclosed NOV28 nucleic acid of the invention encoding a connexin-like protein includes the nucleic acid whose sequence is provided in Table 28A or a fragment thereof. The invention also includes a mutant or variant nucleic acid any of whose bases may be changed from the corresponding base shown in Table 28A while still encoding a protein that maintains its connexin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way ofnonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 16 percent of the bases may be so changed.

The disclosed NOV28 protein of the invention includes the connexin-like protein whose sequence is provided in Table 28B. The invention also includes a mutant or variant protein any of whose residues may be changed from the corresponding residue shown in Table 28B while still encoding a protein that maintains its connexin-like activities and physiological functions, or a functional fragment thereof. In the mutant or variant protein, up to about 0 percent of the residues may be so changed.

The invention further encompasses antibodies and antibody fragments, such as F_{ab} or (F_{ab})₂, that bind immunospecifically to any of the proteins of the invention.

The above defined information for this invention suggests that this connexin-like protein (NOV28) may function as a member of a "connexin family". Therefore, the NOV28 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV28 nucleic acids and proteins of the invention are useful in potential therapeutic applications implicated in diseases including but not limited to various pathologies and disorders as indicated below. For example, a cDNA encoding the connexin-like protein (NOV28) may be useful in gene therapy, and the connexin-like protein (NOV28) may be useful when administered to a subject in need thereof. By way of nonlimiting example, the compositions of the present invention will have efficacy for treatment of patients suffering from Cardiomyopathy, Atherosclerosis, Hypertension, Congenital heart defects, Aortic stenosis ,Atrial septal defect (ASD),Atrioventricular (A-V) canal defect, Ductus arteriosus, Pulmonary stenosis , Subaortic stenosis, Ventricular septal defect (VSD), valve diseases,Tuberous sclerosis, Scleroderma, Obesity,Transplantation, Diabetes,Von Hippel-Lindau (VHL) syndrome, Pancreatitis,Obesity, Endometriosis,Fertility, Hemophilia, Hypercoagulation,Idiopathic thrombocytopenic purpura, Immunodeficiencies, Graft vesus host, Autoimmune disease, Renal artery stenosis, Interstitial nephritis, Glomerulonephritis, Polycystic kidney disease, Systemic lupus erythematosus, Renal tubular acidosis, IgA nephropathy, Hypercalceimia, Lesch-Nyhan syndrome, or other pathologies or conditions. The NOV28 nucleic acid encoding the connexin-like protein of the invention, or fragments thereof, may further be useful in diagnostic applications, wherein the presence or amount of the nucleic acid or the protein are to be assessed.

NOV28 nucleic acids and polypeptides are further useful in the generation of antibodies that bind immuno-specifically to the novel NOV28 substances for use in therapeutic or diagnostic methods. These antibodies may be generated according to methods known in the art, using prediction from hydrophobicity charts, as described in the "Anti-NOVX Antibodies" section below. The disclosed NOV28 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV29

A disclosed NOV29 nucleic acid of 1400 nucleotides (also referred to as CG57664-01) encoding a MHC Class I antigen-like protein is shown in Table 29A.

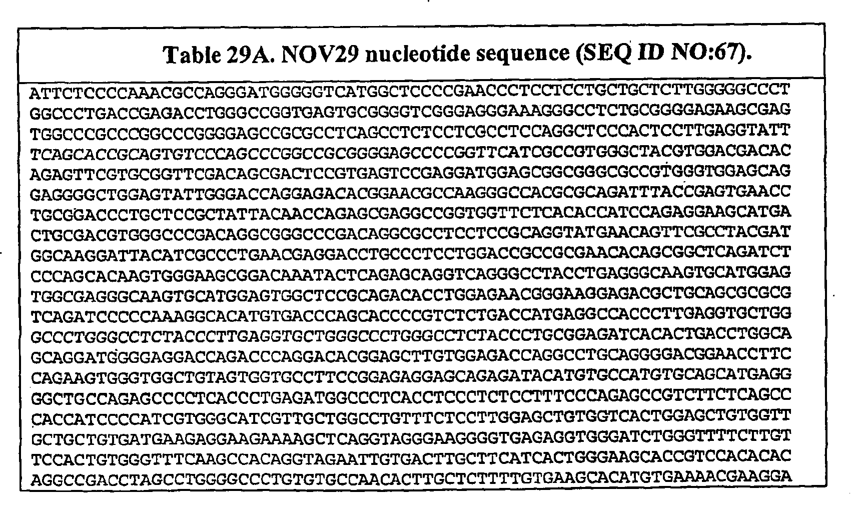

In a search of public sequence databases, the NOV29 nucleic acid sequence, located on chromsome 6 has 332 of 353 bases (94%) identical to a gb:GENBANK-ID:HUMHLA92|acc:M96338.1 mRNA from Homo sapiens (Homo sapiens HLA-92 gene sequence). Public nucleotide databases include all GenBank databases and the GeneSeq patent database.

The disclosed NOV29 polypeptide (SEQ ID NO:68) encoded by SEQ ID NO:67 has 452 amino acid residues and is presented in Table 29B using the one-letter amino acid code. Signal P, Psort and/or Hydropathy results predict that NOV29 has a signal peptide and is likely to be localized at the plasma membrane with a certainty of 0.4600. The most likely cleavage site for a NOV29 peptide is between amino acids 24 and 25.

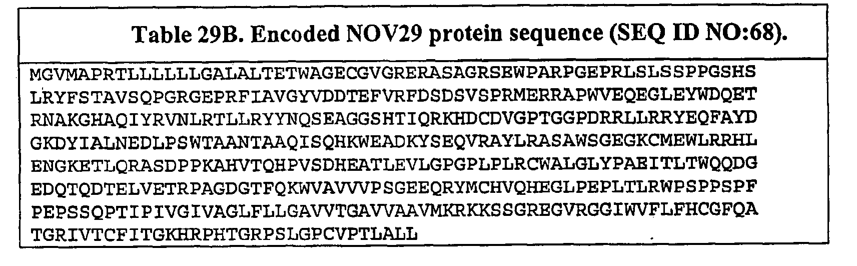

A search of sequence databases reveals that the NOV29 amino acid sequence has 158/223 (70%) identity and 177/223 (79%) similarity with SPTREMBL-ACC:Q9TPL2 MHC CLASS I ANTIGEN - Pan troglodytes (Chimpanzee). Public amino acid databases include the GenBank databases, SwissProt, PDB and PIR.

NOV29 is expressed in at least Bone Marrow, Dermis, Hippocampus, Placenta, and Tonsils. Expression information was derived from the tissue sources of the sequences that were included in the derivation of the sequence of CG57664-01. The sequence is predicted to be expressed in the following tissues because of the expression pattern of (GENBANK-ID: gb:GENBANK-ID:HUMHLA92|acc:M96338.1) a closely related Homo sapiens HLA-92 gene sequence homolog in species Homo sapiens :Lymphoblastoid cell line. This information was derived by determining the tissue sources of the sequences that were included in the invention including but not limited to SeqCalling sources, Public EST sources, Literature sources, and/or RACE sources.

The disclosed NOV29 polypeptide has homology to the amino acid sequences shown in the BLASTP data listed in Table 29C.

| **Table 29C. BLAST results for NOV29** | | | | | |
|---|---|---|---|---|---|
| Gene Index/ Identifier | Protein/ Organism | Length (aa) | Identity (%) | Positives (%) | Expect |
| gi\|8117819\|gb\|AAF72 785.1\|AF168404 1 (AF168404) | MHC class I antigen [Pan troglodytes] | 365 | 275/404 (68%) | 300/404 (74%), | e-141 |
| gi\|8117799\|gb\|AAF72 776.1\|AF168395 1 (AF168395) | MHC class I antigen [Pan troglodytes] | 365 | 276/404 (68%) | 301/404 (74%) | e-141 |
| gi\|2118771\|pir\|I54 493 | MHC class I histocompatibilit y antigen HLA-A alpha chain precursor human | 365 | 271/404 (67%) | 299/404 (73%), | e-141 |
| gi\|8117808\|gb\|AAF72 780.1\|AF168399 1 (AF168399) | MHC class I antigen [Pan troglodytes] | 365 | 275/404 (68%) | 300/404 (74%), | e-140 |
| gi\|2251146\|emb\|CAA6 5501.1 (X96724) | human leukocyte antigen [Homo sapiens] | 365 | 270/404 (66%) | 300/404 (73%), | e-140 |

Tables 29D-E list the domain descriptions from DOMAIN analysis results against NOV29. This indicates that the NOV29 sequence has properties similar to those of other proteins known to contain this domain.

| **Table 29D. Domain Analysis of NOV29** | | |
|---|---|---|
| gnl\|Pfam\|pfam00129, MHC_I, Class I Histocompatibility antigen, domains alpha 1 and 2 | | |
| | CD-Length = 179 residues, 96.6% aligned | |
| | | Score = 248 bits (634), Expect = 4e-67 |

| **Table 29E. Domain Analysis of NOV29** | | |
|---|---|---|
| gnl\|Smart\|smart00407, IGc1, Immunoglobulin C-Type | | |
| | CD-Length = 75 residues, 96.0% aligned | |
| | | Score = 59.7 bits (143), Expect = 4e-10 |

The major histocompatibility complex (MHC) encodes the class I and class II families of glycoproteins, that present peptides for immunorecognition by cytotoxic and helper T lymphocytes, respectively. Class I molecules bind peptides generated by degradation of proteins intracellularly, whereas class II molecules associate mainly with peptides derived from endocytosed extracellular proteins. Two genes encode components of the proteasome complex, which degrades cytosolic proteins and may generate antigenic peptides. Two closely linked genes, PSF1 and PSF2, encode subunits of a transporter, which presumably translocates peptides into an exocytic compartment where they associate with class I molecules. The location of these genes in the MHC in close linkage to the class I and class II gene families suggests that they coevolved to optimize functional interactions.

The disclosed NOV29 nucleic acid of the invention encoding a MHC Class I antigen-like protein includes the nucleic acid whose sequence is provided in Table 29A or a fragment thereof. The invention also includes a mutant or variant nucleic acid any of whose bases may be changed from the corresponding base shown in Table 29A while still encoding a protein that maintains its MHC Class I antigen-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 6 percent of the bases may be so changed.

The disclosed NOV29 protein of the invention includes the MHC Class I antigen-like protein whose sequence is provided in Table 29B. The invention also includes a mutant or variant protein any of whose residues may be changed from the corresponding residue shown in Table 29B while still encoding a protein that maintains its MHC Class I antigen-like activities and physiological functions, or a functional fragment thereof. In the mutant or variant protein, up to about 30 percent of the residues may be so changed.

The invention further encompasses antibodies and antibody fragments, such as F_{ab} or (F_{ab})₂, that bind immunospecifically to any of the proteins of the invention.

The above defined information for this invention suggests that this MHC Class I antigen-like protein (NOV29) may function as a member of a "MHC Class I antigen family". Therefore, the NOV29 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration in *vivo* and in *vitro* of all tissues and cell types composing (but not limited to) those defined here.

The NOV29 nucleic acids and proteins of the invention are useful in potential therapeutic applications implicated in diseases including but not limited to various pathologies and disorders as indicated below. For example, a cDNA encoding the MHC Class I antigen-like protein (NOV29) may be useful in gene therapy, and the MHC Class I antigen-like protein (NOV29) maybe useful when administered to a subject in need thereof By way of nonlimiting example, the compositions of the present invention will have efficacy for treatment of patients suffering from Von Hippel-Lindau (VHL) syndrome, Alzheimer's disease, Stroke, Tuberous sclerosis, hypercalceimia, Parkinson's disease, Huntington's disease, Cerebral palsy, Epilepsy,Lesch-Nyhan syndrome, Multiple sclerosis,Ataxia-telangiectasia,Leukodystrophies,Behavioral disorders, Addiction, Anxiety, Pain, Neuroprotection, Tonsilitis, Hemophilia, hypercoagulation,Idiopathic thrombocytopenic purpura, autoimmume disease,allergies, immunodeficiencies,transplantation, Graft vesus host, or other pathologies or conditions. The NOV29 nucleic acid encoding the MHC Class I antigen-like protein of the invention, or fragments thereof, may further be useful in diagnostic applications, wherein the presence or amount of the nucleic acid or the protein are to be assessed.

NOV29 nucleic acids and polypeptides are further useful in the generation of antibodies that bind immuno-specifically to the novel NOV29 substances for use in therapeutic or diagnostic methods. These antibodies may be generated according to methods known in the art, using prediction from hydrophobicity charts, as described in the "Anti-NOVX Antibodies" section below. The disclosed NOV29 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV30

A disclosed NOV30 nucleic acid of 1225 nucleotides (also referred to as CG57666-01) encoding a MHC Class I antigen-like protein is shown in Table 30A. The start and stop codons are in bold letters.

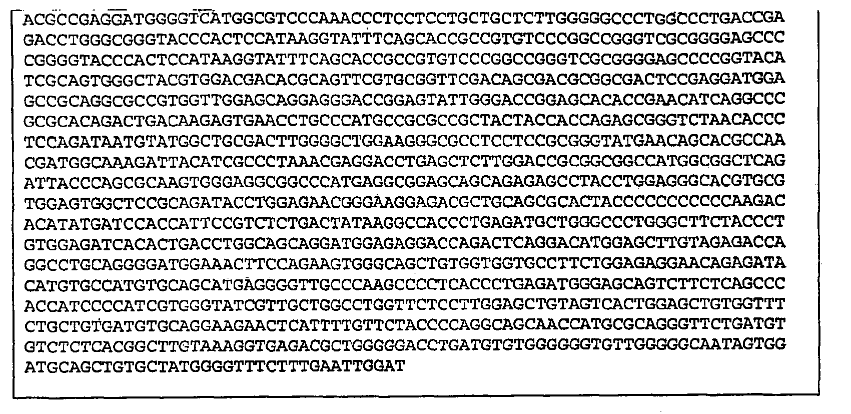

In a search of public sequence databases, the NOV30 nucleic acid sequence, located on chromsome 6 has 265 of 271 bases (97%) identical to a gb:GENBANK-ID:AF055066|acc:AF055066.1 mRNA from Homo sapiens (Homo sapiens MHC class 1 region). Public nucleotide databases include all GenBank databases and the GeneSeq patent database.

The disclosed NOV30 polypeptide (SEQ ID NO:70) encoded by SEQ ID NO:69 has 389 amino acid residues and is presented in Table 30B using the one-letter amino acid code. Signal P, Psort and/or Hydropathy results predict that NOV30 has a signal peptide and is likely to be localized at the plasma membrane with a certainty of 0.4600. The most likely cleavage site for a NOV30 peptide is between amino acids 21 and 22.

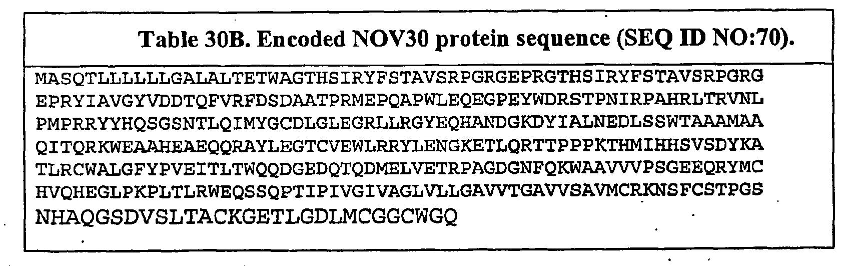

A search of sequence databases reveals that the NOV30 amino acid sequence has 258/338 (76%) identity and 284/338 (84%) similarity with SPTREMBL-ACC:Q31602 MHC CLASS I ANTIGEN - Homo sapiens. Public amino acid databases include the GenBank databases, SwissProt, PDB and PIR.

NOV30 is expressed in at least Bone Marrow, Dermis, Hippocampus, Placenta, Tonsils. This information was derived by determining the tissue sources of the sequences that were included in the invention including but not limited to SeqCalling sources, Public EST sources, Literature sources, and/or RACE sources.

The disclosed NOV30 polypeptide has homology to the amino acid sequences shown in the BLASTP data listed in Table 30C.

| **Table 30C. BLAST results for NOV30** | | | | | |
|---|---|---|---|---|---|
| Gene Index/ Identifier | Protein/ Organism | Length (aa) | Identity (%) | Positives (%) | Expect |
| gi \|8571968 \|gb \|AAF76 942.1 \|AF179640 1 (AF179640) | MHC class I antigen [Pan troglodytes] | 365 | 283/383 (73%) | 313/383 (80%) | e-155 |
| gi \|18571970 \|gb \|AAF76 943.1 \|AP179641 1 (AF179641) | MHC class I antigen [Pan troglodytes] | 363 | 282/383 (73%) | 312/383 (80%) | e-155 |
| gi \|8117808 \|gb \|AAF72 780.1 \|AF168399 1 (AF16B399) | MHC class I antigen [Pan troglodytes] | 365 | 282/383 (73%) | 312/383 (80%) | e-155 |
| gi \|2118771 \|pir\| \|I54 493 | MHC class I histocompatibilit y antigen HLA-A alpha chain precursor- human | 365 | 280/383 (73%) | 312/383 (80%) | e-154 |
| gi\|6049049\|gb\|AAF02 442.1\| (AF115463) | MHC class I antigen [Pan troglodytes] | 365 | 281/383 (73%) ' | 311/383 (80%) | e-153 |

Tables 30D-E list the domain descriptions from DOMAIN analysis results against NOV30. This indicates that the NOV30 sequence has properties similar to those of other proteins known to contain this domain.

| **Table 30D. Domain Analysis ofNOV30** | |
|---|---|
| gnl\|Pfam\|pfam00129, MHC_I, Class I Histocompatibility antigen, domains alpha 1 and 2. | |
| | CD-Length = 179 residues, 100.0% aligned |
| | Score = 245 bits (626), Expect = 3e-66 |

| **Table 30E. Domain Analysis of NOV30** | |
|---|---|
| gnl\|Smart\|smart00407, IGc1, Immunoglobulin C-Type | |
| | CD-Length = 75 residues, 100.0% aligned |
| | Score = 75.5 bits (184), Expect = 5e-15 |

The disclosed NOV30 nucleic acid of the invention encoding a MHC Class I antigen-like protein includes the nucleic acid whose sequence is provided in Table 30A or a fragment thereof. The invention also includes a mutant or variant nucleic acid any of whose bases may be changed from the corresponding base shown in Table 30A while still encoding a protein that maintains its MHC Class I antigen-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 3 percent of the bases may be so changed.

The disclosed NOV30 protein of the invention includes the MHC Class I antigen-like protein whose sequence is provided in Table 30B. The invention also includes a mutant or variant protein any of whose residues may be changed from the corresponding residue shown in Table 30B while still encoding a protein that maintains its MHC Class I antigen-like activities and physiological functions, or a functional fragment thereof. In the mutant or variant protein, up to about 24 percent of the residues may be so changed.

The invention further encompasses antibodies and antibody fragments, such as F_{ab} or (F_{ab})₂, that bind immunospecifically to any of the proteins of the invention.

The above defmed information for this invention suggests that this MHC Class I antigen-like protein (NOV30) may function as a member of a "MHC Class I antigen family". Therefore, the NOV30 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration in vivo and *in vitro* of all tissues and cell types composing (but not limited to) those defined here.

The NOV30 nucleic acids and proteins of the invention are useful in potential therapeutic applications implicated in diseases including but not limited to various pathologies and disorders as indicated below. For example, a cDNA encoding the MHC Class I antigen-like protein (NOV30) may be useful in gene therapy, and the MHC Class I antigen-like protein (NOV30) may be useful when administered to a subject in need thereof. By way of nonlimiting example, the compositions of the present invention will have efficacy for treatment of patients suffering from Von Hippel-Lindau (VHL) syndrome , Alzheimer's disease, Stroke, Tuberous sclerosis, hypercalceimia, Parkinson's disease, Huntington's disease, Cerebral palsy, Epilepsy,Lesch-Nyhan syndrome, Multiple sclerosis,Ataxia-telangiectasia,Leukodystrophies,Behavioral disorders, Addiction, Anxiety, Pain, Neuroprotection, Tonsilitis, Hemophilia, hypercoagulation,Idiopathic thrombocytopenic purpura; autoimmume disease, allergies, immunodeficiencies,transplantation, Graft vesus host, or other pathologies or conditions. The NOV30 nucleic acid encoding the MHC Class I antigen-like protein of the invention, or fragments thereof, may further be useful in diagnostic applications, wherein the presence or amount of the nucleic acid or the protein are to be assessed.

NOV30 nucleic acids and polypeptides are further useful in the generation of antibodies that bind immuno-specifically to the novel NOV30 substances for use in therapeutic or diagnostic methods. These antibodies may be generated according to methods known in the art, using prediction from hydrophobicity charts, as described in the "Anti-NOVX Antibodies" section below. The disclosed NOV30 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV31

A disclosed NOV31 nucleic acid of 1159 nucleotides (also referred to as CG57668-01) encoding a MHC Class I antigen-like protein is shown in Table 31A. The start and stop codons are in bold letters.

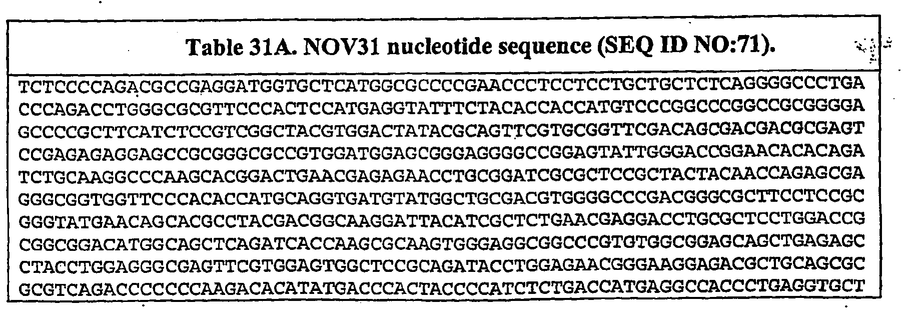

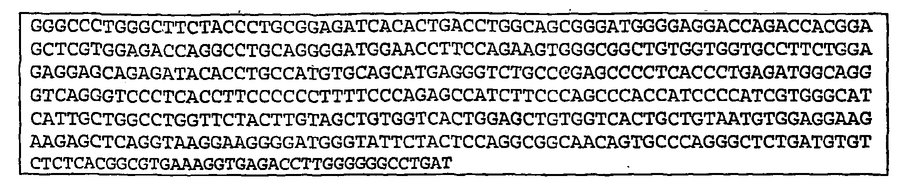

In a search of public sequence databases, the NOV31 nucleic acid sequence, located on chromsome 6 has 404 of 512 bases (78%) identical to a gb:GENBANK-ID:AF055066|acc:AF055066.1 mRNA from Homo sapiens (Homo sapiens MHC class 1 region). Public nucleotide databases include all GenBank databases and the GeneSeq patent database.

The disclosed NOV31 polypeptide (SEQ ID NO:72) encoded by SEQ ID NO:71 has 371 amino acid residues and is presented in Table 31B using the one-letter amino acid code. Signal P, Psort and/or Hydropathy results predict that NOV31 has a signal peptide and is likely to be localized at the plasma membrane with a certainty of 0.7300. The most likely cleavage site for a NOV31 peptide is between amino acids 18 and 19.

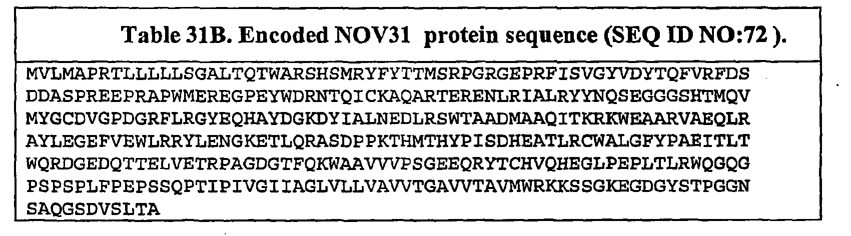

A search of sequence databases reveals that the NOV31 amino acid sequence has 335/368 (91 %) identity and 342/368 (92%) similarity with REMTREMBL-ACC:CAB66931 Gogo-H protein - Gorilla gorilla (gorilla). Public amino acid databases include the GenBank databases, SwissProt, PDB and PIR.

NOV31 is expressed in at least Bone Marrow, Dermis, Hippocampus, Placenta, Tonsils. This information was derived by determining the tissue sources of the sequences that were included in the invention including but not limited to SeqCalling sources, Public EST sources, Literature sources, and/or RACE sources.

The disclosed NOV31 polypeptide has homology to the amino acid sequences shown in the BLASTP data listed in Table 31C.

| **Table 31C. BLAST results for NOV31** | | | | | |
|---|---|---|---|---|---|
| Gene Index/ Identifier | Protein/ Organism | Length (aa) | Identity (%) | Positives (%) | Expect |
| gi\|232260\|sp\|P01893 \|HLAH HUMAN | HLA class I histocompatibilit y antigen, alpha chain H precursor (HLA-AR) (HLA-12.4) | 362 | 330/376 (87%) | 339/376 (89%) | e-167 |
| gi\|70075\|pir\|\|HLHU1 2 | MHC class I histocompatibilit y antigen HLA alpha chain precursor (clone pHLA 12.4)-human | 359 | 329/373 (88%) . | 337/373 (90%), | e-166 |
| gi\|2118771\|pir\|\|I54 493 | MHC class I histocompatibilit y antigen HLA-A alpha chain precursor - human | 365 | 303/376 (80%) | 326/376 (86%), | e-164 |
| gi 4164602 gb AAD05 568.1 (AF116214) | MHC class I antigen heavy chain [Homo sapiens] | 365 | 302/376 (80%) | 325/376 (86%), | e-163 |
| gi\|915219\|gb\|AAA735 18.1\| (U25971) | MHC class I antigen HLA-A2407 [Homo sapiens] | 365 | 302/376 (80%) | 326/376 (86%), | e-163 |

Tables 31D-E list the domain descriptions from DOMAIN analysis results against NOV31. This indicates that the NOV31 sequence has properties similar to those of other proteins known to contain this domain.

| **Table 31D. Domain Analysis of NOV31** | | |
|---|---|---|
| gnl \|Pfam \|pfam00129, MHC_I, Class I Histocompatibility antigen, domains alpha 1 and 2 | | |
| | CD-Length = 179 residues, 99.4% aligned | |
| | | Score = 284 bits (727), Expect = 5e-78 |

| **Table 31E. Domain Analysis of NOV31** |
|---|
| gnl\|smart\|smart00407, IGc1, Immunoglobulin C-Type |
| CD-Length = 75 residues, 98.7% aligned |
| Score = 77.8 bits (190), Expect = 1e-15 |

The disclosed NOV31 nucleic acid of the invention encoding a MHC Class I antigen-like protein includes the nucleic acid whose sequence is provided in Table 31A or a fragment thereof. The invention also includes a mutant or variant nucleic acid any of whose bases may be changed from the corresponding base shown in Table 31A while still encoding a protein that maintains its MHC Class I antigen-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases; and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 22 percent of the bases may be so changed.

The disclosed NOV31 protein of the invention includes the MHC Class I antigen-like protein whose sequence is provided in Table 31B. The invention also includes a mutant or variant protein any of whose residues may be changed from the corresponding residue shown in Table 31B while still encoding a protein that maintains its MHC Class I antigen-like activities and physiological functions, or a functional fragment thereof. In the mutant or variant protein, up to about 9 percent of the residues may be so changed.

The invention further encompasses antibodies and antibody fragments, such as F_{ab} or (F_{ab})₂, that bind immunospecifically to any of the proteins of the invention.

The above defined information for this invention suggests that this MHC Class I antigen-like protein (NOV31) may function as a member of a "MHC Class I antigen family". Therefore, the NOV31 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types Composing (but not limited to) those defined here.

The NOV31 nucleic acids and proteins of the invention are useful in potential therapeutic applications implicated in diseases including but not limited to various pathologies and disorders as indicated below. For example, a cDNA encoding the MHC Class I antigen-like protein (NOV31) may be useful in gene therapy, and the MHC Class I antigen-like protein (NOV31) may be useful when administered to a subject in need thereof. By way of nonlimiting example, the compositions of the present invention will have efficacy for treatment of patients suffering from Von Hippel-Lindau (VHL) syndrome, Alzheimer's disease, Stroke, Tuberous sclerosis, hypercalceimia, Parkinson's disease, Huntington's disease, Cerebral palsy, Epilepsy,Lesch-Nyhan syndrome, Multiple sclerosis,Ataxia-telangiectasia,Leukodystrophies,Behavioral disorders, Addiction, Anxiety, Pain, Neuroprotection, Tonsilitis, Hemophilia, hypercoagulation,Idiopathic thrombocytopenic purpura, autoimmume disease,allergies, immunodeficiencies, transplantation, Graft vesus host as well, or other pathologies or conditions. The NOV nucleic acid encoding the MHC Class I antigen-like protein of the invention, or fragments thereof, may further be useful in diagnostic applications, wherein the presence or amount of the nucleic acid or the protein are to be assessed.

NOV31 nucleic acids and polypeptides are further useful in the generation of antibodies that bind immuno-specifically to the novel NOV31 substances for use in therapeutic or diagnostic methods. These antibodies may be generated according to methods known in the art, using prediction from hydrophobicity charts, as described in the "Anti-NOVX Antibodies" section below. The disclosed NOV31 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV32

A disclosed NOV32 nucleic acid of nucleotides (also referred to as CG57660-01) encoding a retinoic acid receptor responder-like protein is shown in Table 32A. The start and stop codons are in bold letters.

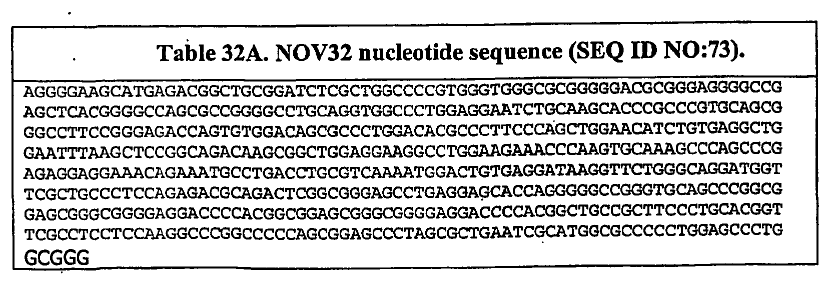

In a search of public sequence databases, the NOV32 nucleic acid sequence, located on chromsome 4 has 443 of 451 bases (98%) identical to a gb:GENBANK-ID:AF146191|acc:AF146191.1 mRNA from Homo sapiens (Homo sapiens FRG1 (FRG1) gene, complete cds; 5S ribosomal RNA gene, complete sequence; TUB4q and TIG2 pseudogenes, complete sequence). Public nucleotide databases include all GenBank databases and the GeneSeq patent database.

The disclosed NOV32 polypeptide (SEQ ID NO:74) encoded by SEQ ID NO:73 has amino acid residues and is presented in Table 32B using the one-letter amino acid code. Signal P, Psort and/or Hydropathy results predict that NOV32 has a signal peptide and is likely to be localized at the plasma membrane with a certainty of 0.8800.

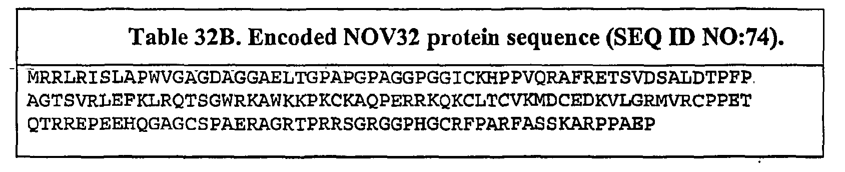

A search of sequence databases reveals that the NOV32 amino acid sequence has 94/168 (55%) identity and 109/168 (64%) similarity with ptnr:SWISSPROT-ACC:Q99969 RETINOIC ACID RECEPTOR RESPONDER PROTEIN 2 PRECURSOR (TAZAROTENE-INDUCED GENE 2 PROTEIN) (RAR-RESPONSIVE PROTEIN TIG2)- Homo sapiens. Public amino acid databases include the GenBank databases, SwissProt, PDB and PIR.

NOV32 is expressed in at least Adipose, Adrenal gland, Breast, Colon, Esophagus, Eye, Heart, Kidney, Liver, Lung, Ovary, Parathyroid, Placenta, Prostate, Stomach, Testis, Uterus, Whole embryo, bladder tumor, brain, cervix, colon, head and neck, kidney, lung, muscle and ovary. This information was derived by determining the tissue sources of the sequences that were included in the invention including but not limited to SeqCalling sources, Public EST sources, Literature sources, and/or RACE sources.

The disclosed NOV32 polypeptide has homology to the amino acid sequences shown in the BLASTP data listed in Table 32C.

| **Table 32C. BLAST results for NOV32** | | | | | |
|---|---|---|---|---|---|
| Gene Index/ Identifier | Protein/ Organism | Length (aa) | Identity (%) | Positives (%) | Expect |
| gi\|145O6427\|ref\|NP 0 02880.1 (NM_002889) | retinoic acid receptor responder (tazarotene induced) 2 [Homo sapiens] | 163 | 94/169 (55%) | 109/169 (63% | 8e-37 |
| gi\|12832179\|dbj\|BAB 21997.1\| (AK002298) | homolog to RETINOIC ACID RECEPTOR RESPONDER PROTEIN 2 PRECURSOR (TAZAROTENE-INDUCED GENE 2 PROTEIN) (RAR-RESPONSIVE PROTEIN TIG2)-putative [Mus musculus] | 162 | 72/169 (42%) | 94/169 (55%), | 3e-22 |
| gi\|17436162\|ref\|XP 067978.1 (XM_067976) | similar to retinoic acid receptor responder (tazarotene induced) 2 [Homo sapiens] | 206 | 73/74 . (98%) | 74/74 (99%) | 1e-20 |
| gil\|185954\|ref\|XP 091375.1\| (XM 091375) | hypothetical protein XP_091375 [Homo sapiens] | 322 | 52/79 (65%) | 59/79 (73%) | 2e-18 |
| gi\|17488051\|ref\|XP 064055.1\| (XM_064055) | similar to Fibroblast growth factor receptor 3 precursor (FGFR-3) (Heparin-binding growth factor receptor) [Homo sapiens] | 296 | 28/54 (51%) | 32/54 (58%), | 2e-5 |

Retinoids exert their biologic effects through two families of nuclear receptors, retinoic acid receptors (RARs) and retinoid X receptors (RXRs), which belong to the superfamily of steroid/thyroid hormone nuclear receptors. The retinoid-mediated up-regulation in the expression of TIG2 was confirmed by Northern blot analysis. Upon sequencing, TIG2 was found to be a cDNA whose complete sequence was not in the GenBank and EMBL data bases. The TIG2 cDNA is 830 bp long and encodes a putative protein product of 164 amino acids. TIG2 is neither expressed nor induced by tazarotene in primary keratinocyte and fibroblast cultures. Thus, TIG2 is expressed and induced by tazarotene only when keratinocytes and fibroblasts form a tissue-like 3-dimensional structure. RAR-specific retinoids increase TIG2 mRNA levels. In contrast, neither RXR-specific retinoids nor 1,25-dihydroxyvitamin D3 increased TIG2 levels. TIG2 is expressed at high levels in nonlesional psoriatic skin but at lower levels in the psoriatic lesion and that its expression is up-regulated in psoriatic lesions after topical application of tazarotene.

The disclosed NOV32 nucleic acid of the invention encoding a retinoic acid receptor responder-like protein includes the nucleic acid whose sequence is provided in Table 32A or a fragment thereof. The invention also includes a mutant or variant nucleic acid any of whose bases may be changed from the corresponding base shown in Table 32A while still encoding a protein that maintains its retinoic acid receptor responder-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 2 percent of the bases may be so changed.

The disclosed NOV32 protein of the invention includes the retinoic acid receptor responder-like protein whose sequence is provided in Table 32B. The invention also includes a mutant or variant protein any of whose residues may be changed from the corresponding residue shown in Table 32B while still encoding a protein that maintains its retinoic acid receptor responder-like activities and physiological functions, or a functional fragment thereof. In the mutant or variant protein, up to about 45 percent of the residues may be so changed.

The invention further encompasses antibodies and antibody fragments, such as F_{ab} or (F_{ab})₂, that bind immunospecifically to any of the proteins of the invention.

The above defined information for this invention suggests that this retinoic acid receptor responder-like protein (NOV32) may function as a member of a "retinoic acid receptor responder family". Therefore, the NOV32 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV32 nucleic acids and proteins of the invention are useful in potential therapeutic applications implicated in diseases including but not limited to various pathologies and disorders as indicated below. For example, a cDNA encoding the retinoic acid receptor responder-like protein (NOV32) may be useful in gene therapy, and the retinoic acid receptor responder-like protein (NOV32) may be useful when administered to a subject in need thereof. By way of nonlimiting example, the compositions of the present invention will have efficacy for treatment of patients suffering from Cardiomyopathy, Atherosclerosis,Hypertension, Congenital heart defects, Aortic stenosis ,Atrial septal defect (ASD),Atrioventricular (A-V) canal defect, Ductus arteriosus , Pulmonary stenosis , Subaortic stenosis, Ventricular septal defect (VSD), valve diseases,Tuberous sclerosis, Scleroderma, Obesity,Transplantation, Diabetes,Von Hippel-Lindau (VHL) syndrome, Pancreatitis,Obesity, Endometriosis,Fertility, Hemophilia, Hypercoagulation,Idiopathic thrombocytopenic purpura, Immunodeficiencies,Graft vesus host, Autoimmune disease, Renal artery stenosis, Interstitial nephritis, Glomerulonephritis, Polycystic kidney disease, Systemic lupus erythematosus, Renal tubular acidosis, IgA nephropathy, Hypercalceimia, Lesch-Nyhan syndrome, or other pathologies or conditions. The NOV32 nucleic acid encoding the retinoic acid receptor responder-like protein of the invention, or fragments thereof, may further be useful in diagnostic applications, wherein the presence or amount of the nucleic acid or the protein are to be assessed.

NOV32 nucleic acids and polypeptides are further useful in the generation of antibodies that bind immuno-specifically to the novel NOV32 substances for use in therapeutic or diagnostic methods. These antibodies may be generated according to methods known in the art, using prediction from hydrophobicity charts, as described in the "Anti-NOVX Antibodies" section below. The disclosed NOV32 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV33

A disclosed NOV33 nucleic acid of 1706 nucleotides (also referred to as) encoding a PHOSPHATIDYLINOSITOL 4-PHOSPHATE 5-KINASE -like protein is shown in Table 33A. The start and stop codons are in bold letters.

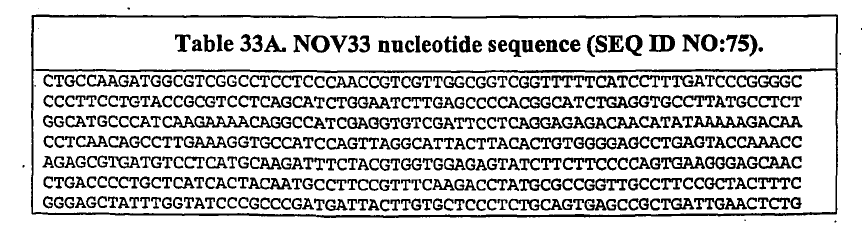

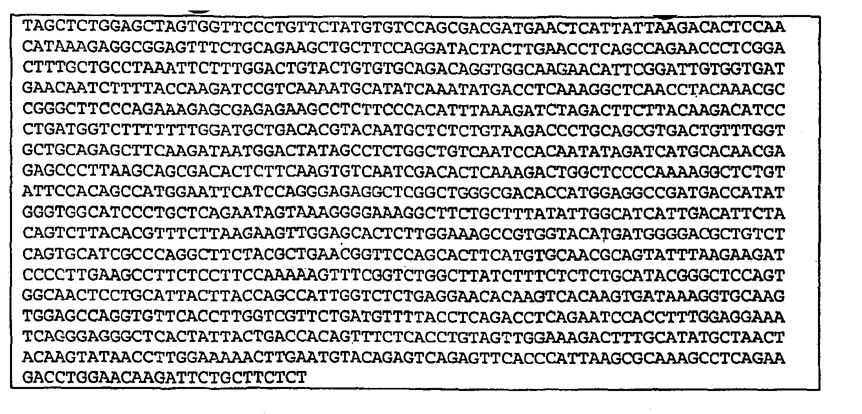

In a search of public sequence databases, the NOV33 nucleic acid sequence, located on chromsome 6 has 1586 of 1706 bases (92%) identical to a gb:GENBANK-ID:HSU78575|acc:U78575.1 mRNA from Homo sapiens (Human 68 kDa type I phosphatidylinositol-4-phosphate 5-kinase alpha mRNA, clone PIP5KIal, complete cds). Public nucleotide databases include all GenBank databases and the GeneSeq patent database.

The disclosed NOV33 polypeptide (SEQ ID NO:76) encoded by SEQ ID NO:75 has 551 amino acid residues and is presented in Table 33B using the one-letter amino acid code.

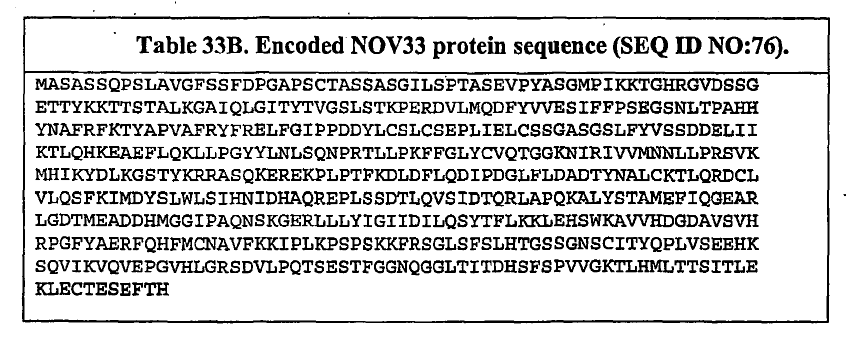

A search of sequence databases reveals that the NOV33 amino acid sequence has 478 of 551 amino acid residues (86%) identical to, and 496 of 551 amino acid residues (90%) similar to, the 549 amino acid residue ptnr:SPTREMBL-ACC:Q99754 protein from Homo sapiens (Human) (68 KDA TYPE I PHOSPHATIDYLINOSITOL-4-PHOSPHATE 5-KINASE ALPHA (EC 2.7.1.68) (1-PHOSPHATIDYLINOSITOL-4-PHOSPHATE KINASE) (DIPHOSPHOINOSITIDE KINASE) (PTDINS(4)P-5-KINASE)). Public amino acid databases include the GenBank databases, SwissProt, PDB and PIR.

NOV33 is expressed in at least Adrenal gland, Aorta, B-cells, Blood, Bone, Brain, Breast, CNS, Colon, Ear, Esophagus, Eye, Gall bladder, Germ Cell, Head and neck, Heart, Kidney, Larynx, Liver, Lung, Lymph, Marrow, Muscle, Neural, Omentum, Ovary, Pancreas, Parathyroid, Peripheral nervous system, Placenta, Pooled, Prostate, Skin, Small intestine, Spleen, Stomach, Synovial membrane, Testis, Tissue culture, Tonsil, Uterus, Whole embryo, and adrenal gland. Expression information was derived from the tissue sources of the sequences that were included in the derivation of the sequence of CG57672-01.The sequence is predicted to be expressed in the following tissues because of the expression pattern of (GENBANK-lD: gb:GENBANK-ID:HSU78575Iacc:U78575.1) a closely related Human 68 kDa type I phosphatidylinositol-4-phosphate 5-kinase alpha mRNA, clone PIPSKIal, complete cds homolog in species Homo sapiens :fetal brain. This information was derived by determining-the tissue sources of the sequences that were included in the invention including but not limited to SeqCalling sources, Public EST sources, Literature sources, and/or RACE sources.

The disclosed NOV33 polypeptide has homology to the amino acid sequences shown in the BLASTP data listed in Table 33C.

| **Table 33C. BLAST results for NOV33** | | | | | |
|---|---|---|---|---|---|
| Gene Index/ Identifier | Protein/ Organism | Length (aa) | Identity (%) | Positives (%) | Expect |
| gi\|4505815\|ref\|NP 0 03548.1\| (NM_003557) | phosphatidylinosi tol-4-phosphate 5-kinase, type I, alpha [Homo sapiens] | 549 | 478/552 (86%) | 496/552 (89%), | 0.0 |
| gi\|1743873\|gb\|AAC50 911.1\| (U78576) | 68 kDa type I phosphatidylinosi tol-4-phosphate 5-kinase alpha [Homo sapiens] | 562 | 478/565 (84%) | 496/565 (87%), | 0.0 |
| gil\|743875\|gb\|AAC50 912.1\| (U78577) | 68 kDa type I phosphatidylinosi tol-4-phosphate 5-kinase alpha [Homo sapiens] | 500 | 439/551 (79%) | 455/551 (81%), | 0.0 |
| gi\|6679331\|ref\|NP 0 32873.1\| (NM_008847) | phosphatidylinosi tol-4-phosphate 5-kinase, type 1 beta; PI4P5K-I[b] [Mus musculus] | 546 | 434/552 (78%) | 470/552 (84%), | 0.0 |
| gi\|14745097\|ref\|XP 018166.2 (XM_018166) | similar to phosphatidylinosi tol-4-phosphate 5-kinase, type I, alpha (H. sapiens) [Homo sapiens] | 435 | 361/408 (88%) | 381/408 (92%), | 0.0 |

Tables 33D-E list the domain descriptions from DOMAIN analysis results against NOV33. This indicates that the NOV33 sequence has properties similar to those of other proteins known to contain this domain.

| **Table 33D. Domain Analysis of NOV33** | | |
|---|---|---|
| gnl\|smart\|smart0330, PIPKc, Phosphatidylinositol phosphate kinases | | |
| | CD-Length = 339 residues, 100.0% aligned | |
| | | Score = 410 bits (1054), Expect = le-115 |

| **Table 33E. Domain Analysis of NOV33** | | |
|---|---|---|
| gnl\|Pfam\|pfam01504, PIP5K, Phosphatidylinositol-4-phosphate 5-Kinase. This family contains a region from the common kinase core found in the type I phosphatidylinositol-4-phosphate 5-kinase (PIP5K) family as described in. The family consists of various type I, II and III PIP5K enzymes. PIP5K catalyses the formation of phosphoinositol-4,5-bisphosphate via the phosphorylation of phosphatidylinositol-4-phosphate a precursor in the phosphinositide signaling pathway | | |
| | CD-Length = 293 residues, 99.7% aligned | |
| | | Score = 390 bits (1002), Expect = 1e-109 |

Phosphatidylinositol-4-phosphate 5-kinases (PIP5K) synthesize phosphatidylinositol-4,5-bisphosphate, a key precursor in phosphoinositide signaling that also regulates some proteins and cellular processes directly. Two distinct PIP5Ks have been characterized in erythrocytes, the 68-kDa type I (PEP5KI) and 53-kDa type II (PIP5KII) isoforms. Using peptide sequences from the erythroid 68-kDa PIP5KI, cDNAs encoding PIP5KI from human brain were isolated. Partial cDNAs obtained for a second isoform, PIP5KI , established that the human STM7 gene encoded a previously unrecognized PIP5KI. However, the peptide sequences demonstrated that erythroid PIP5KI corresponded to PIP5KI . Recombinant, bacterially expressed PIP5KI possessed PIP5K activity and was immunoreactive with erythroid PIP5KI antibodies. By Northern analysis, PIP5KI and PIP5KI had wide tissue distributions; but their expression levels differed greatly. PIP5KIs had homology to the kinase domains of PIP5KII, yeast Mss4p and Fablp, and a new Caenorhabditis elegans Fab1-like, protein identified in the data base. These new isoforms have refined the sequence requirements for PIP5K activity and, potentially, regulation of these enzymes. Furthermore, the limited homology between PIP5KIs and PIPSKII , which was almost exclusively within the kinase domain core, provided a molecular basis for distinction between type I and II PIP5Ks. Phosphatidylinositol-4-phosphate 5-kinases (PIP5Ks) synthesize phosphatidylinositol 4,5-bisphosphate by phosphorylating phosphatidylinositol 4-phosphate. By searching sequence databases with peptide sequences obtained from the 68-kD type I PIP5K purified from bovine erythrocytes, Loijens and Anderson (1996) identified a human EST encoding PIP5K1A, which they called PIPSKI-alpha. They screened a human fetal brain cDNA library and isolated full-length PIPSK1A cDNAs. The deduced 549-amino acid protein has the conserved kinase homology domain of PIP5K family members. Within this domain, PIP5K1A shows 83% and 35% amino acid identity with PIP5K1B and PIP5K2A, respectively: Overall, the PIP5K1A and PIP5K1B proteins are 64% identical. Recombinant PIP5K1A expressed in bacteria had a molecular mass of approximately 66.3 kD by Western blot analysis. The authors isolated additional PIPSK1A cDNAs which they suggested represent splicing isoforms. Northern blot analysis detected a major 4.2-kb PIP5K1A transcript which had a wide tissue distribution.

The disclosed NOV33 nucleic acid of the invention encoding a PHOSPHATIDYLINOSITOL 4-PHOSPHATE 5-KINASE-like protein includes the nucleic acid whose sequence is provided in Table 33A or a fragment thereof. The invention also includes a mutant or variant nucleic acid any of whose bases may be changed from the corresponding base shown in Table 33A while still encoding a protein that maintains its PHOSPHATIDYLINOSITOL 4-PHOSPHATE 5-KINASE-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 8 percent of the bases may be so changed.

The disclosed NOV33 protein of the invention includes the PHOSPHATIDYLINOSITOL 4-PHOSPHATE 5-KINASE-like protein whose sequence is provided in Table 33B. The invention also includes a mutant or variant protein any of whose residues may be changed from the corresponding residue shown in Table 33B while still encoding a protein that maintains its PHOSPHATIDYLINOSITOL 4-PHOSPHATE 5-KINASE-like activities and physiological functions, or a functional fragment thereof. In the mutant or variant protein, up to about 14 percent of the residues may be so changed.

The invention further encompasses antibodies and antibody fragments, such as F_{ab} or (F_{ab})₂, that bind immunospecifically to any of the proteins of the invention.

The above defined information for this invention suggests that this PHOSPHATIDYLINOSITOL 4-PHOSPHATE 5-KINASE-like protein (NOV33) may function as a member of a "PHOSPHATIDYLINOSITOL 4-PHOSPHATE 5-KINASE family". Therefore, the NOV33 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV33 nucleic acids and proteins of the invention are useful in potential therapeutic applications implicated in diseases including but not limited to various pathologies and disorders as indicated below. For example, a cDNA encoding the PHOSPHATIDYLINOSITOL 4-PHOSPHATE 5-KINASE-like protein (NOV33) may be useful in gene therapy, and the PHOSPHATIDYLINOSITOL 4-PHOSPHATE 5-KINASE-like protein (NOV33) may be useful when administered to a subject in need thereof. By way of nonlimiting example, the compositions of the present invention will have efficacy for treatment of patients suffering from Cardiomyopathy, Atherosclerosis,Hypertension, Congenital heart defects, Aortic stenosis ,Atrial septal defect (ASD),Atrioventricular (A-V) canal defect, Ductus arteriosus , Pulmonary stenosis , Subaortic stenosis, Ventricular septal defect (VSD), valve diseases,Tuberous sclerosis, Scleroderma, Obesity,Transplantation, Adrenoleukodystrophy , Congenital Adrenal Hyperplasia, Hemophilia, Hypercoagulation,Idiopathic thrombocytopenic purpura , Immunodeficiencies,Graft vesus host, Von Hippel-Lindau (VHL) syndrome , Alzheimer's disease, Stroke, Tuberous sclerosis, hypercalceimia, Parkinson's disease, Huntington's disease, Cerebral palsy, Epilepsy,Lesch-Nyhan syndrome, Multiple sclerosis,Ataxia-telangiectasia,Leukodystrophies,Behavioral disorders, Addiction, Anxiety, Pain, Neuroprotection, or other pathologies or conditions. The NOV33 nucleic acid encoding the PHOSPHATIDYLINOSITOL 4-PHOSPHATE 5-KINASE-like protein of the invention, or fragments thereof, may further be useful in diagnostic applications, wherein the presence or amount of the nucleic acid or the protein are to be assessed.

NOV33 nucleic acids and polypeptides are further useful in the generation of antibodies that bind immuno-specifically to the novel NOV33 substances for use in therapeutic or diagnostic methods. These antibodies may be generated according to methods known in the art, using prediction from hydrophobicity charts, as described in the "Anti-NOVX Antibodies" section below. The disclosed NOV33 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV34

A disclosed NOV34 nucleic acid of 1316 nucleotides (also referred to as CG57680-01) encoding a Cyclophilin-type peptidyl-prolyl cis-trans isomerase-like protein is shown in Table 34A. The start and stop codons are in bold letters.

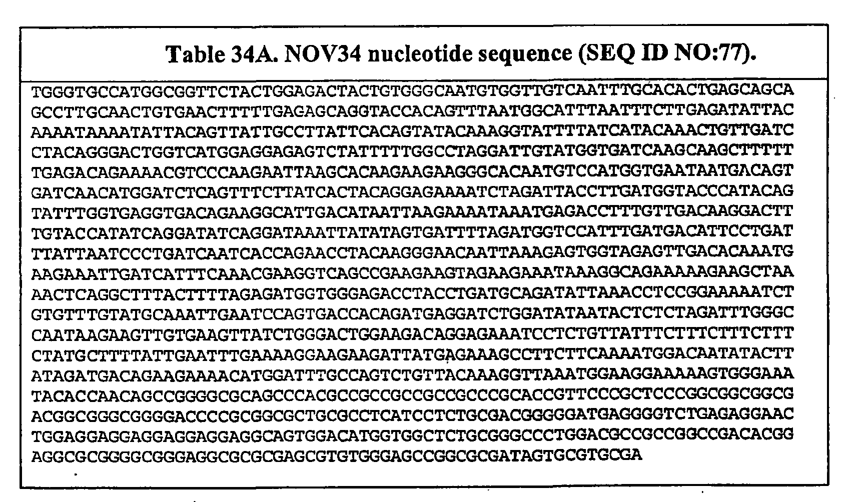

In a search of public sequence databases, the NOV34 nucleic acid sequence, located on chromsome 7 has 149 of 235 bases (63%) identical to a gb:GENBANK-ID:A45258|acc:A45258.1 mRNA from human herpesvirus 2 (Sequence 2 from Patent WO9516779). Public nucleotide databases include all GenBank databases and the GeneSeq patent database.

The disclosed NOV34 polypeptide (SEQ ID NO:78) encoded by SEQ ID NO:77 has 432 amino acid residues and is presented in Table 34B using the one-letter amino acid code. Signal P, Psort and/or Hydropathy results predict that NOV34 has a signal peptide and is likely to be localized at the plasma membrane with a certainty of 0.9820.

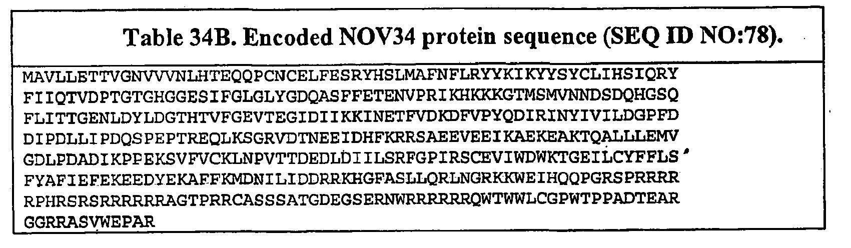

A search of sequence databases reveals that the NOV34 amino acid sequence has 263/345 (76%) identity and 291/345 (84%) similarity with TREMBLNEW-ACC:BAB30711 6 DAYS NEONATE HEAD CDNA, RIKEN FULL-LENGTH ENRICHED LIBRARY, CLONE:5430431E21, FULL INSERT SEQUENCE - Mus musculus. Public amino acid databases include the GenBank databases, SwissProt, PDB and PIR.

NOV34 is expressed in at least Adrenal Gland/Suprarenal gland, Bone, Bone Marrow, Brain, Colon, Hair Follicles, Heart, Hippocampus, Kidney, Liver, Lung, Lymphoid tissue, Pancreas, Peripheral Blood, Prostate, Salivary Glands, Small Intestine, Testis, Tonsils, Uterus. This information was derived by determining the tissue sources of the sequences that were included in the invention including but not limited to SeqCalling sources, Public EST sources, Literature sources, and/or RACE sources.

The disclosed NOV34 polypeptide has homology to the amino acid sequences shown in the BLASTP data listed in Table 34C.

| **Table 34C. BLAST results for NOV34** | | | | | |
|---|---|---|---|---|---|
| Gene Index/ Identifier | Protein/ Organism | Length (aa) | Identity (%) | Positives (%) | Expect |
| gi 12849069 dbj BAB 28194.1 (AK012371) | homolog to DJ12G14.1 (NOVEL CYCLOPHILIN TYPE PEPTIDYL-PROLYL CIS-TRANS ISOMERASE) (FRAGMENT)-putati ve [Mus musculus] | 382 | 261/331 (78%) | 284/331 (84%) | e-138 |
| gi\|12847571\|dbj\|BAB 27623.1\| (AK011443) | homolog to DJ12G14.1 (NOVEL CYCLOPHILIN TYPE PEPTIDYL-PROLYL CIS-TRANS ISOMERASE) (FRAGMENT)-putati ve [Mus musculus] | 418 | 261/331 (78%) | 284/331 (84%) | e-137 |
| gi 12856573 dbj BAB 30711.1 (AK017370) | homolog to DJ12G14.1 *(NOVEL CYCLOPHILIN TYPE PEPTIDYL-PROLYL CIS-TRANS ISOMERASE) (FRAGMENT)-putati ve [Mus musculus] | 460 | 261/331 (78%) | 284/331 (84% | e-136 |
| gi\|12852237\|dbj\|BAB 29330.1 (AK014406 | homolog to DJ12G14.1 (NOVEL CYCLOPHILIN TYPE PEPTIDYL-PROLYL CIS-TRANS ISOMERASE) (FRAGMENT)-putati ve [Mus musculus] | 492 | 259/331 (78%) | 284/331 (85%) | e-135 |
| gi\|18088111\|gb\|AAH2 0986.1\|AAH20986 (BC020986) | protein for MGC:9727) [Homo sapiens] | 492 | 263/331 (79%) | 284/331 (85%) | e-135 |

Tables 34D-E list the domain descriptions from DOMAIN analysis results against NOV34. This indicates that the NOV34 sequence has properties similar to those of other proteins known to contain this domain.

| **Table 34D. Domain Analysis of NOV34** | | |
|---|---|---|
| gnl \|Pfam \|pfam00160, pro_isomerase, Cyclophilin type peptidyl-prolyl cis-trans isomerase | | |
| | CD-Length = 162 residues, 88.9% aligned | |
| | | Score = 91.7 bits (226), Expect = 8e-20 |

| **Table 34E. Domain Analysis of NOV34** | |
|---|---|
| gnl\|Pfam\|pfam00076, rrm, RNA recognition motif. (a.k.a. RRM, RBD, or RNP domain). The RRM motif is probably diagnostic of an RNA binding protein. RRMs are found in a variety of RNA binding proteins, including various hnRNP proteins, proteins implicated in regulation of alternative splicing, and protein components of snRNPs. The motif also appears in a few single stranded DNA binding proteins. The RRM structure consists of four strands and two helices arranged in an alpha/beta sandwich, with a third helix present during RNA binding in some cases The C-terminal beta strand (4th strand) and final helix are hard to align and have been omitted in the SEED alignment The LA proteins have a N terminus rrm which is included in the seed. There is a second region towards the C terminus that has some features of a rrm but does not appear to have the important structural core of a rrm. The LA proteins are one of the main autoantigens in Systemic lupus erythematosus (SLE), an autoimmune disease. | |
| | CD-Length = 71 residues, 95.8% aligned |
| | Score = 47.8 bits (112), Expect = 1e-06 |

The cyclophilins are a conserved class of proteins that bind the immunosuppressive drug cyclosporin A (CsA) with high affinity. CsA blocks helper T-cell activation at a step between T-cell receptor stimulation and the transcriptional activation of cytokine genes. Cyclophilins from many species possess peptidyl-prolyl cis-trans isomerase (PPIase) activity that is blocked by CsA and therefore may be relevant in CsA-mediated immunosuppression. Probing with the previously known cyclophilin cDNA under reduced stringencies, Price et al. (1991) identified a second cyclophilin gene, which encoded cyclophilin B (CYPB). The deduced protein was 64% identical to CYPA and was distinguished from it by a signal sequence that probably directs it to the endoplasmic reticulum (ER): CYPB showed even stronger similarity to yeast CYPB, which also has an ER-directed signal sequence. The signal sequence is removed from the protein upon expression in E. coli, and the processed protein possesses PPIase activity that is inhibited by CsA. Peddada et al. (1992) used the PCR technique to generate a unique probe complementary to the hydrophobic 5-prime end of the human cyclophilin B gene. Using this probe in an analysis of human/hamster hybrid somatic cell lines, they assigned the gene to chromosome 15. The human PIN1 gene encodes an essential nuclear peptidyl-prolyl cis/trans isomerase involved in the regulation of mitosis. PIN1 is a member of a new class of peptidyl-prolyl cis/trans isomerases that includes the Escherichia coli parvulin, yeast ESS1, and Drosophila melanogaster dodo gene products. Lu et al. (1996) described human PIN1 and showed that deletion of PIN1 from HeLa cells induces mitotic arrest, while HeLa cells overexpressing PIN1 arrest in the G2 phase. Campbell et al. (1997) identified a gene closely related to the gene encoding the essential nuclear peptidylprolyl cis/trans isomerase (PIN1) involved in the regulation of mitosis. The novel gene, called PIN1L by them, is 89% identical at the nucleotide level to the PIN1 transcript, but contains a shift in the reading frame. It encodes a 100-amino acid variant protein consisting of 63 amino acids homologous (90% identical) to PIN1 and contains the entire WW domain, fused to a 37-amino acid tail. By fluorescence in situ hybridization and somatic cell hybrid analysis, Campbell et al. (1997) mapped PIN1L to 1q31. They commented that the protein encoded by PIN1L may have some functional role or, alternatively, PIN1L may be a transcribed pseudogene. Campbell et al. (1997) found by analysis of human expressed sequence tags (ESTs) 2 different but closely related human transcripts, 1 of which corresponds to PIN1. Gene localization, using both fluorescence in situ hybridization and tritium-labeled probes, showed that each of the human transcripts hybridized to 1p31 and 19p13. Primers were designed to discriminate between the 2 transcripts, and PCR on DNA from hamster/human somatic cell hybrids retaining chromosomes 1 or 19 was used to map the human PIN1 gene to chromosome 19, and PIN1L, the closely related gene, to chromosome 1. Their results established that PIN1 is at 19p13 and PIN1L at 1p31. PCR was used to clone the coding region for PIN1L. The PIN1L cDNA is 89% identical at the nucleotide level to the PIN1 transcript, but contained a shift in the reading frame. Campbell et al. (1997) commented that the protein encoded by PIN1L may have some functional role or, alternatively, PIN1L may be a transcribed pseudogene. With the knowledge that PIN1 specifically isomerizes phosphorylation of a serine or threonine that precedes proline and regulates the function of mitotic phosphoproteins, and hypothesizing that restoring the function of phosphorylated tau might prevent or reverse paired helical filament (PHF) formation in Alzheimer disease, Lu et al. (1999) demonstrated that the WW domain of PIN1 binds to phosphorylated tau at threonine-231 (T231). The T231 residue is hyperphosphorylated in Alzheimer disease and is phosphorylated to a certain extent in the normal brain. Using a pulldown assay, Lu et al. (1999) demonstrated that PIN1 binds to hyperphosphorylated tau from the brains of people with Alzheimer disease but not to tau from age-matched healthy brains. By immunoblotting, Lu et al. (1999) detected endogenous PIN1 in the PHFs of diseased brains, and using immunohistochemistry, they found that recombinant PIN1 binds to pathologic tau. Using immunohistochemistry, Lu et al. (1999) localized PIN1 to the nucleus in healthy brains. In the brains of people with Alzheimer disease, PIN1 staining was associated with pathologic tau in neuronal cells. Lu et al. (1999) also demonstrated that phosphorylated tau could neither bind microtubules nor promote microtubule assembly. However, PIN1 was able to restore the ability of phosphorylated tau to bind microtubules and promoted microtubule assembly in vitro. The level of soluble PIN1 in the brains of Alzheimer patients was greatly reduced compared to that in age-matched control brains. The authors concluded with the hypothesis that since depletion of PIN1 induces mitotic arrest and apoptotic cell death, sequestration of PIN1 into PHFs may contribute to neuronal death. In the frog, Pin1 is implicated in the regulation of cell cycle progression and required for the DNA replication checkpoint. By fluorescence microscopy, Winkler et al. (2000) observed that nuclear extracts from Xenopus eggs depleted of Pin1 inappropriately transited from the G2 to the M phase of the cell cycle in the presence of a DNA replication inhibitor. Immunoblot analysis revealed that inappropriate transition was accompanied by hyperphosphorylation of CDC25 (see CDC25A), activation of CDC2 cyclin B and mitotic phosphoproteins. Addition of recombinant wildtype, but not mutant, Pin1 reversed the defect in replication checkpoint function.

The disclosed NOV34 nucleic acid of the invention encoding a Cyclophilin-type peptidyl-prolyl cis-trans isomerase-like protein includes the nucleic acid whose sequence is provided in Table 34A or a fragment thereof. The invention also includes a mutant or variant nucleic acid any of whose bases may be changed from the corresponding base shown in Table 34A while still encoding a protein that maintains its Cyclophilin-type peptidyl-prolyl cis-trans isomerase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 37 percent of the bases maybe so changed.

The disclosed NOV34 protein of the invention includes the Cyclophilin-type peptidyl-prolyl cis-trans isomerase-like protein whose sequence is provided in Table 34B. The invention also includes a mutant or variant protein any of whose residues may be changed from the corresponding residue shown in Table 34B while still encoding a protein that maintains its Cyclophilin-type peptidyl-prolyl cis-trans isomerase-like activities and physiological functions, or a functional fragment thereof. In the mutant or variant protein, up to about 24 percent of the residues may be so changed.

The invention further encompasses antibodies and antibody fragments, such as F_{ab} or (F_{ab})₂, that bind immunospecifically to any of the proteins of the invention.

The above defined information for this invention suggests that this Cyclophilin-type peptidyl-prolyl cis-trans isomerase-like protein (NOV34) may function as a member of a "Cyclophilin-type peptidyl-prolyl cis-trans isomerase family". Therefore, the NOV34 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration in *vivo* and *in vitro* of all tissues and cell types composing (but not limited to) those defined here.

The NOV34 nucleic acids and proteins of the invention are useful in potential therapeutic applications implicated in diseases including but not limited to various pathologies and disorders as indicated below. For example, a cDNA encoding the Cyclophilin-type peptidyl-prolyl cis-trans isomerase-like protein (NOV34) may be useful in gene therapy, and the Cyclophilin-type peptidyl-prolyl cis-trans isomerase-like protein (NOV34) may be useful when administered to a subject in need thereof. By way of nonlimiting example, the compositions of the present invention will have efficacy for treatment of patients suffering from Cardiomyopathy, Atherosclerosis, Hypertension, Congenital heart defects, Aortic stenosis ,Atrial septal defect (ASD),Atrioventricular (A-V) canal defect, Ductus arteriosus , Pulmonary stenosis , Subaortic stenosis, Ventricular septal defect (VSD), valve diseases,Tuberous sclerosis, Scleroderma, Obesity, Transplantation, Adrenoleukodystrophy , Congenital Adrenal Hyperplasia, Hemophilia, Hypercoagulation,Idiopathic thrombocytopenic purpura, Immunodeficiencies, Graft vesus host, Von Hippel-Lindau (VHL) syndrome, Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia,Leukodystrophies,Behavioral disorders, Addiction, Anxiety, Pain, Neuroprotection, or other pathologies or conditions. The NOV34 nucleic acid encoding the Cyclophilin-type peptidyl-prolyl cis-trans isomerase-like protein of the invention, or fragments thereof, may further be useful in diagnostic applications, wherein the presence or amount of the nucleic acid or the protein are to be assessed.

NOV34 nucleic acids and polypeptides are further useful in the generation of antibodies that bind immuno-specifically to the novel NOV34 substances for use in therapeutic or diagnostic methods. These antibodies may be generated according to methods known in the art, using prediction from hydrophobicity charts, as described in the "Anti-NOVX Antibodies" section below. The disclosed NOV34 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV35

A disclosed NOV35 nucleic acid of 1647 nucleotides (also referred to as CG57670-01) encoding a pyruvate kinase-like protein is shown in Table 35A. The start and stop codons are in bold letters.

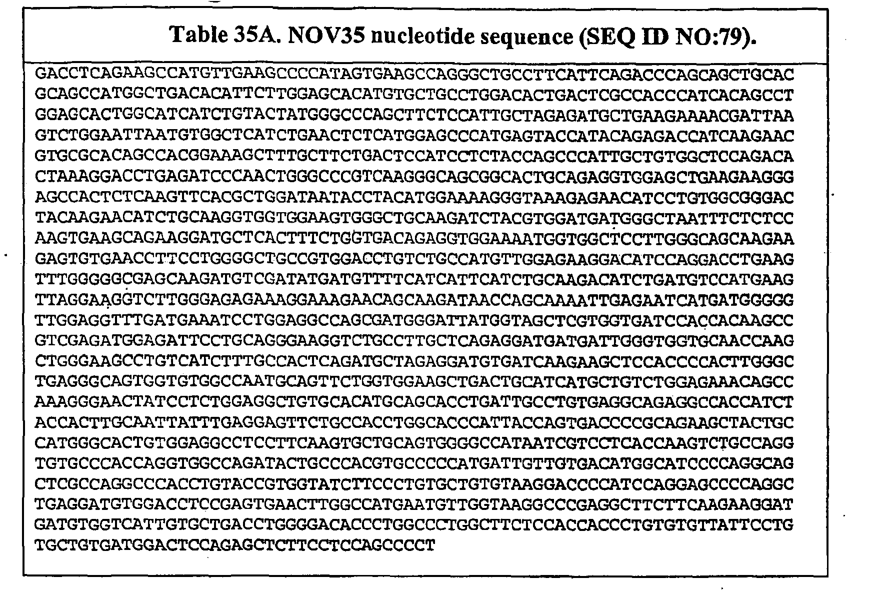

In a search of public sequence databases, the NOV35 nucleic acid sequence, located on chromsome 6 has 751 of 894 bases (84%) identical to a gb:GENBANK-ID:MMM2PK|acc:X97047.1 mRNA from Mus musculus (M.musculus mRNA for M2-type pyruvate kinase). Public nucleotide databases include all GenBank databases and the GeneSeq patent database.

The disclosed NOV35 polypeptide (SEQ ID NO:80) encoded by SEQ ID NO:79 has 534 amino acid residues and is presented in Table 35B using the one-letter amino acid code. Signal P, Psort and/or Hydropathy results predict that NOV35 has a signal peptide and is likely to be localized extracellularly with a certainty of 0.4500.

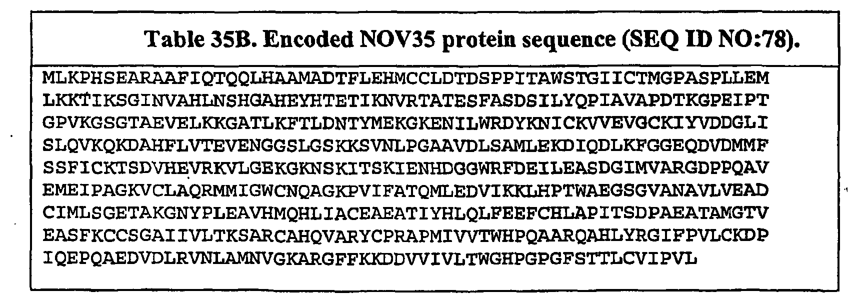

A search of sequence databases reveals that the NOV35 amino acid sequence has 433/533 (81%) identity and 458/533 (85%) similarity with pir-id:S30038 pyruvate kinase (EC 2.7.1.40), muscle splice form M2 - human. Public amino acid databases include the GenBank databases, SwissProt, PDB and PIR.

NOV35 is expressed in at least Adrenal gland, Aorta, B-cells, Blood, Bone, Brain, Breast, CNS, Colon, Ear, Esophagus, Eye, Gall bladder, Germ Cell, Head and neck, Heart, Kidney, Larynx, Liver, Lung, Lymph, Marrow, Muscle, Neural, Omentum, Ovary, Pancreas, Parathyroid, Peripheral nervous system, Placenta, Pooled, Prostate, Skin, Small intestine, Spleen, Stomach, Synovial membrane, Testis, Tissue culture, Tonsil, Uterus, Whole embryo, and adrenal gland. This information was derived by determining the tissue sources of the sequences that were included in the invention including but not limited to SeqCalling sources, Public EST sources, Literature sources, and/or RACE sources.

The disclosed NOV35 polypeptide has homology to the amino acid sequences shown in the BLASTP data listed in Table 35C.

| **Table 35C. BLAST results for NOV35** | | | | | |
|---|---|---|---|---|---|
| Gene Index/ Identifier | Protein/ Organism | Length (aa) | Identity (%) | Positives (%) | Expect |
| gi\|14750405\|ref\|XP 037768.1\| (XM_037768) | similar to pyruvate kinase, muscle (H. sapiens) | 531 | 433/S34 (81%) | 458/534 (85%) | 0.0 |
| gi\|4505839\|ref\|NP 0 02645.11 (NM_002654 | pyruvate kinase, muscle; Pyruvate kinase-3; Thyroid hormone-binding protein, cytosolic (p58) [Homo sapiens] | 531 | 432/534 (80%) | 458/534 (84%) | 0.0 |
| gi \| 107S54 \| pir \| \|A339 83 | pyruvate kinase (EC 2.7.1.40) isozyme M2 -human | 531 | 431/534 (80%) | 457/534 (84%) | 0.0 |
| gi \| 266427\| sp \| P14618 KPY1 HUMAN | Pyruvate kinase, M1 isozyme (Pyruvate kinase muscle isozyme) (Cytosolic thyroid hormone-binding protein) (CTHBP) (THBP1) | 531 | 433/534 (81%) | 458/534 (85%) | 0.0 |
| gi\|15215430\|gb\|AAH1 2811.1\|AAH12811 (BC012811 | (protein for MGC:3932) [Homo sapiens] | 531 | 431/534 (80%) | 457/534 (84%) | 0.0 |

Tables 35D-E list the domain descriptions from DOMAIN analysis results against NOV35. This indicates that the NOV35 sequence has properties similar to those of other proteins known to contain this domain.

| **Table 35D. Domain Analysis of NOV35** | | |
|---|---|---|
| gnl\|Pfam\|pfam02887, PK_C, Pyruvate kinase, alpha/beta domain | | |
| | CD-Length = 116 residues, 99.1% aligned | |
| | | Score = 129 bits (324), Expect = 4e-31 |

| **Table 35E. Domain Analysis of NOV35** | | |
|---|---|---|
| gnl\|Pfam\|pfam00224, PK, Pyruvate kinase, barrel domain. This domain of the is actually a small beta-barrel domain nested within a larger TIM barrel. The active site is found in a cleft between the two domains. | | |
| | CD-Length = 349 residues, 100.0% aligned | |
| | | Score = 422 bits (1084), Expect = 3e-119 |

Pyruvate kinase is also known as ATP:pyruvate phosphotransferase (EC 2.7.1.40). At least 3 molecular forms with pyruvate kinase activity are known (Bigley et al., 1968). The form that is deficient in a type of hemolytic anemia is the red cell variety, PK1. PK2 is found in kidney. PK3 is found in leukocytes, muscle, platelets, and brain but not in red cells or kidney. PK1 is found also in liver. A patient with red cell PK deficiency has been found to have abnormal liver enzyme also (Bunn, 1981); see Nakashima et al. (1977). During fetal development, PK3 changes to PK1 in the liver. PK1 is a tetramer composed of two dissimilar polypeptides of somewhat different molecular weight. It is an allosteric enzyme exhibiting cooperative binding for phosphoenolpyruvate and sensitivity to fructose-1,6-diphosphate. PK3 also is a tetrameric protein but, unlike PK1, all subunits are alike and, not unexpectedly, there is no cooperative behavior. The enzyme is insensitive to fructose-1,6-diphosphate. Patients with deficiency of red cell PK have normal PK2 and PK3. Tsutsumi et al. (1988) showed that pyruvate kinase occurs in 4 isozymic forms (L, R, M1, M2) and that these are encoded by 2 different genes, PKL and PKM. The L and R isozymes are generated from the PKL gene by differential splicing of RNA; the M1 and M2 forms are produced from the PKM gene by differential splicing. Studies of somatic cell hybrids showed that the PK3 and MPI loci are syntenic (Shows, 1972). By cell hybridization studies, Van Heyningen et al. (1975) found that the MPI and PK3 loci are on chromosome 15. Chem et al. (1977) narrowed the assignment to 15q22-qter. Tani et al. (1988) isolated and sequenced 2 overlapping clones covering the entire coding sequence of PKM2. By in situ hybridization they demonstrated that the gene is located at band 15q22. Northern blot analysis with RNA from a human hepatoma demonstrated that the M2-type PK was predominantly expressed in hepatoma cells, whereas L-type PK was preferentially expressed in the nontumor portion of the liver. Takenaka et al. (1991) reported that the gene that encodes both the M1 and the M2 isozymes is approximately 32 kb long and comprises 12 exons and 11 introns. Exons 9 and 10 contain sequences specific for the M1 and M2 types, respectively, indicating that the human fetal and adult isozymes are produced from the same gene by alternative splicing.

The disclosed NOV35 nucleic acid of the invention encoding a pyruvate kinase-like protein includes the nucleic acid whose sequence is provided in Table 35A or a fragment thereof. The invention also includes a mutant or variant nucleic acid any of whose bases may be changed from the corresponding base shown in Table 35A while still encoding a protein that maintains its pyruvate kinase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 16 percent of the bases may be so changed.

The disclosed NOV35 protein of the invention includes the pyruvate kinase-like protein whose sequence is provided in Table 35B. The invention also includes a mutant or variant protein any of whose residues may be changed from the corresponding residue shown in Table 35B while still encoding a protein that maintains its pyruvate kinase-like activities and physiological functions, or a functional fragment thereof. In the mutant or variant protein, up to about 19 percent of the residues may be so changed

The invention further encompasses antibodies and antibody fragments, such as F_{ab} or (F_{ab})₂, that bind immunospecifically to any of the proteins of the invention.

The above defined information for this invention suggests that this pyruvate kinase-like protein (NOV35) may function as a member of a "pyruvate kinase family". Therefore, the NOV35 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV35 nucleic acids and proteins of the invention are useful in potential therapeutic applications implicated in diseases including but not limited to various pathologies and disorders as indicated below. For example, a cDNA encoding the pyruvate kinase-like protein (NOV35) may be useful in gene therapy, and the pyruvate kinase-like protein (NOV35) may be useful when administered to a subject in need thereof. By way of nonlimiting example, the compositions of the present invention will have efficacy for treatment of patients suffering from Cardiomyopathy, Atherosclerosis,Hypertension, Congenital heart defects, Aortic stenosis Atrial septal defect (ASD),Atrioventricular (A-V) canal defect, Ductus arteriosus , Pulmonary stenosis , Subaortic stenosis, Ventricular septal defect (VSD), valve diseases,Tuberous sclerosis, Scleroderma, Obesity,Transplantation, Adrenoleukodystrophy , Congenital Adrenal Hyperplasia, Hemophilia, Hypercoagulation,Idiopathic thrombocytopenic purpura, Immunodeficiencies,Graft vesus host, Von Hippel-Lindau (VHL) syndrome, Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis,Ataxia-telangiectasia,Leukodystrophies,Behavioral disorders, Addiction, Anxiety, Pain, Neuroprotection, or other pathologies or conditions. The NOV nucleic acid encoding the pyruvate kinase-like protein of the invention, or fragments thereof, may further be useful in diagnostic applications, wherein the presence or amount of the nucleic acid or the protein are to be assessed.

NOV35 nucleic acids and polypeptides are further useful in the generation of antibodies that bind immuno-specifically to the novel NOV35 substances for use in therapeutic or diagnostic methods. These antibodies may be generated according to methods known in the art, using prediction from hydrophobicity charts, as described in the "Anti-NOVX Antibodies" section below. The disclosed NOV35 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV36

NOV36 includes two Cis/Trans Peptidyl Prolyl Isomerase-like proteins disclosed below. The disclosed sequences have been named NOV36a and NOV36b.

### NOV36a

A disclosed NOV36a nucleic acid of 600 nucleotides (also referred to as CG57149-01) encoding a Cis/Trans Peptidyl Prolyl Isomerase-like protein is shown in Table 36A. The start and stop codons are in bold letters.

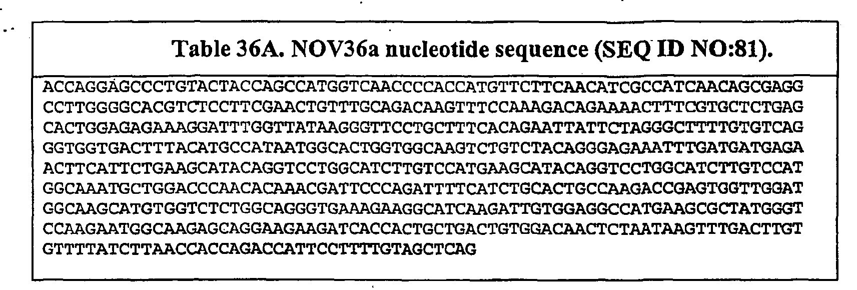

In a search of public sequence databases, the NOV36a nucleic acid sequence, located on chromsome 10 has 288 of 327 bases (88%) identical to a gb:GENBANK-ID:HSCYCR|acc:Y00052.1 mRNA from Homo sapiens (Human mRNA for T-cell cyclophilin). Public nucleotide databases include all GenBank databases and the GeneSeq patent database.

The disclosed NOV36a polypeptide (SEQ ID NO: 82) encoded by SEQ ID NO: 81 has 173 amino acid residues and is presented in Table 36B using the one-letter amino acid code. Signal P, Psort and/or Hydropathy results predict that NOV36a has no signal peptide and is likely to be localized at the plasma membrane with a certainty of 0.6000.

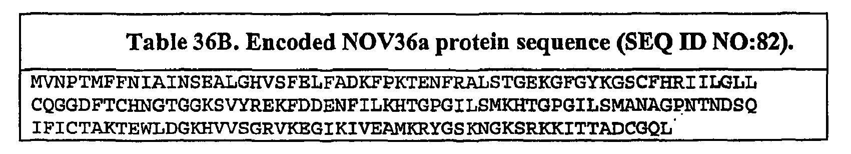

A search of sequence databases reveals that the NOV36a amino acid sequence has 136 of 173 amino acid residues (78%) identical to, and 149 of 173 amino acid residues (86%) similar to, the 165 amino acid residue ptnr:pir-id:CSHUA protein from human (peptidylprolyl isomerase (EC 5.2.1.8) A). Public amino acid databases include the GenBank databases, SwissProt, PDB and PIR.

### NOV36b

A disclosed NOV36b nucleic acid of 566 nucleotides (also referred to as CG57149-02) encoding a Cis/Trans Peptidyl Prolyl Isomerase-like protein is shown in Table 36A. The start and stop codons are in bold letters.

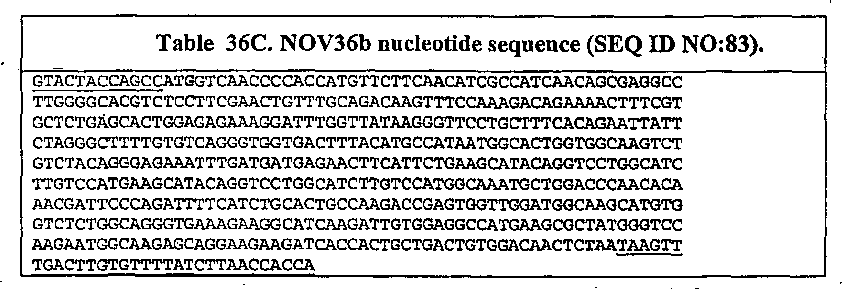

In a search of public sequence databases, the NOV36b nucleic acid sequence has 269 of 311 bases (86%) identical to a gb:GENBANK-ID:AF139893|acc:AF139893.1 mRNA from Oryctolagus cuniculus (Oryctolagus cuniculus cyclophilin 18 mRNA, complete cds). Public nucleotide databases include all GenBank databases and the GeneSeq patent database.

The disclosed NOV36b polypeptide (SEQ ID NO:84) encoded by SEQ ID NO:83 has 173 amino acid residues and is presented in Table 36B using the one-letter amino acid code. Signal P, Psort and/or Hydropathy results predict that NOV36b has no signal peptide and is likely to be localized in the cytoplasm with a certainty of 0.4500.

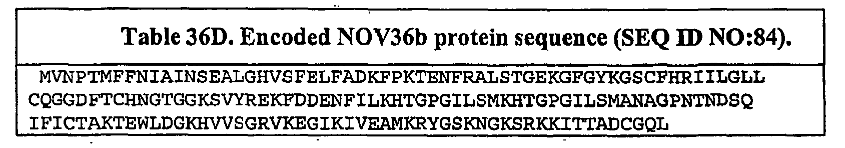

A search of sequence databases reveals that the NOV36b amino acid sequence has 136 of 173 amino acid residues (78%) identical to, and 149 of 173 amino acid residues (86%) similar to, the 165 amino acid residue ptnr:TREMBLNEW-ACC:AAH00689 protein from Homo sapiens (Human) (PEPTIDYLPROLYL ISOMERASE A (CYCLOPHILIN A)). Public amino acid databases include the GenBank databases, SwissProt, PDB and PIR.

NOV36b is expressed in at least Epidermis, Lymphoid tissue. This information was derived by determining the tissue sources of the sequences that were included in the invention including but not limited to SeqCalling sources, Public EST sources, Literature sources, and/or RACE sources.

The disclosed NOV36a polypeptide has homology to the amino acid sequences shown in the BLASTP data listed in Table 36E.

| **Table 36E. BLAST results for NOV36a** | | | | | |
|---|---|---|---|---|---|
| Gene Index/ Identifier | Protein/ Organism | Length (aa) | Identity (%) | Positives (%) | Expect |
| gi\|12804335\|gb\|AAH0 3026.1\|AAH03026 (BC003026) | (protein.for IMAGE:2823490) [Homo sapiens] | 174 | 136/174 (78%) | 149/174 (85%) | 2e-70 |
| gil\|4033689\|sp\|P0437 4\|CYPH BOVIN | PEPTIDYL-PROLYL CIS-TRANS ISOMERASE A (PPIASE) (ROTAMASE) (CYCLOPHILIN A) (CYCLOSPORIN A-BINDING PROTEIN) | 164 | 136/174 (78%) | 149/174 (85%) | 8e-70 |
| gi\|10863927\|ref\|NP 066953.1\| (NM_021130 | peptidylprolyl isomerase A (cyclophilin A) [Homo sapiens] | 165 | 136/174 (78%) | 149/174 (85%) | le-69 |
| gi\|68401\|pir\| \|CSBOA B | peptidylprolyl isomerase (EC 5.2.1.8) A-bovine | 163 | 135/173 (78%) | 148/173 (85%) | 4e-69 |
| gi\|13543666\|gb\|AAHO 5982.1\|AAH05982 (BC005982) | peptidylprolyl isomerase A (cyclophilin A) [Homo sapiens] | 165 | 136/174 (78%) | 149/174 (85%) | 6e-69 |

Table 36F lists the domain descriptions from DOMAIN analysis results against NOV36a. This indicates that the NOV 36a sequence has properties similar to those of other proteins known to contain this domain.

| **Table 36F. Domain Analysis ofNOV36** |
|---|
| gnl\|pfam\|pfam00160, pro_isomerase, Cyclophilin type peptidyl-prolyl cis-trans isomerase |
| CD-Length = 162 residues, 100.0% aligned |
| Score = 196 bits (497), Expect = le-51 |

The human parvulin Pin1 is a member of the peptidyl-prolyl cis-trans isomerase groups of proteins, which modulate the assembly, folding, activity, and transport of essential cellular proteins. Pin1 is a mitotic regulator interacting with a range of proteins that are phosphorylated before cell division. In addition, an involvement of Pin1 in the tau-related neurodegenerative brain disorders has recently been shown. In this context, Pin1 becomes depleted from the nucleus in Alzheimer's disease (AD) neurons when it is redirected to the large amounts of hyperphosphorylated tau associated with the neurofibrillary tangles. This depletion from the nucleus may ultimately contribute to neuron cell death. The 131-amino acid residue parvulin-like human peptidyl-prolyl cis/trans isomerase (PPIase) hPar14 was shown to exhibit sequence similarity to the regulator enzyme for cell cycle transitions human hPin1, but specificity for catalyzing pSer(Thr)-Pro cis/trans isomerizations was lacking. That FK and CsA completely inhibit immune function without completely inhibiting CN suggests that the inhibition of immune function is not mediated by general CN inhibition but by inhibition of a subset of CN which is critical for lymphocyte activation.

The disclosed NOV36b nucleic acid of the invention encoding a Cis/Trans Peptidyl Prolyl Isomerase-like protein includes the nucleic acid whose sequence is provided in Table 36A or a fragment thereof. The invention also includes a mutant or variant nucleic acid any of whose bases may be changed from the corresponding base shown in Table 36A while still encoding a protein that maintains its Cis/Trans Peptidyl Prolyl Isomerase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 14 percent of the bases may be so changed.

The disclosed NOV36b protein of the invention includes the Cis/Trans Peptidyl Prolyl Isomerase-like protein whose sequence is provided in Table 36B. The invention also includes a mutant or variant protein any of whose residues may be changed from the corresponding residue shown in Table 36B while still encoding a protein that maintains its Cis/Trans Peptidyl Prolyl Isomerase-like activities and physiological functions, or a functional fragment thereof. In the mutant or variant protein, up to about 22 percent of the residues may be so changed.

The invention further encompasses antibodies and antibody fragments, such as F_{ab} or (F_{ab})₂, that bind immunospecifically to any of the proteins of the invention.

The above defined information for this invention suggests that this Cis/Trans Peptidyl Prolyl Isomerase-like protein (NOV36) may function as a member of a "Cis/Trans Peptidyl Prolyl Isomerase family". Therefore, the NOV36 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV36 nucleic acids and proteins of the invention are useful in potential therapeutic applications implicated in diseases including but not limited to various pathologies and disorders as indicated below. For example, a cDNA encoding the Cis/Trans Peptidyl Prolyl Isomerase-like protein (NOV36) maybe useful in gene therapy, and the Cis/Trans Peptidyl Prolyl Isomerase-like protein (NOV36) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV36 nucleic acid encoding the Cis/Trans Peptidyl Prolyl Isomerase-like protein of the invention, or fragments thereof, may further be useful in diagnostic applications, wherein the presence or amount of the nucleic acid or the protein are to be assessed.

NOV36 nucleic acids and polypeptides are further useful in the generation of antibodies that bind immuno-specifically to the novel NOV substances for use in therapeutic or diagnostic methods. These antibodies may be generated according to methods known in the art, using prediction from hydrophobicity charts, as described in the "Anti-NOVX Antibodies" section below. The disclosed NOV36 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV37

A disclosed NOV37 nucleic acid of 660 nucleotides (also referred to as CG57151-01) encoding a Cis/Trans Peptidyl Prolyl Isomerase-like protein is shown in Table 37A. The start and stop codons are in bold letters.

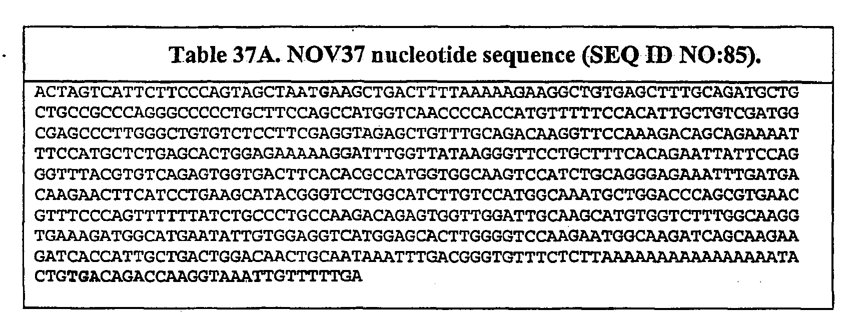

In a search of public sequence databases, the NOV37 nucleic acid sequence, located on chromsome 10 has 492 of 581 bases.(85%) identical to a (HSCPH192|acc: X52857.1) cyclophilin-related processed pseudogene mRNA from human. Public nucleotide databases include all GenBank databases and the GeneSeq patent database.

The disclosed NOV37 polypeptide (SEQ ID NO:86) encoded by SEQ ID NO:85 has 203 amino acid residues and is presented in Table 37B using the one-letter amino acid code. Signal P, Psort and/or Hydropathy results predict that NOV37 has no signal peptide and is likely to be localized in the cytoplasm with a certainty of 0.6400.

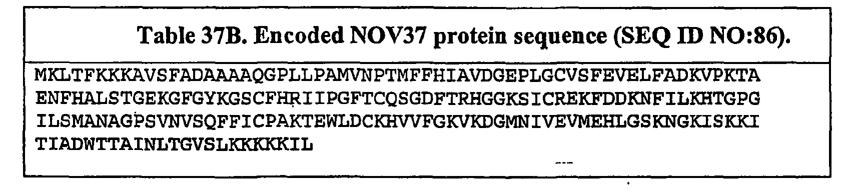

A search of sequence databases reveals that the NOV37 amino acid sequence has 136 of 160 amino acid residues (85%) identical to, and 142 of 160 amino acid residues (88%) similar to, the 165 amino acid residue ptnr:pir-id:CSHUA protein from human (peptidylprolyl isomerase (EC 5.2.1.8) A). Public amino acid databases include the GenBank databases, SwissProt, PDB and PIR.

NOV37 is expressed in at least Bone Marrow, Brain, Cartilage, Cochlea, Colon, Epidermis, Kidney, Lung, Mammary gland/Breast, Ovary, Pancreas, Prostate, Stomach, Testis, Thymus, Umbilical Vein, Uterus, Vulva, Whole Organism. Expression information was derived from the tissue sources of the sequences that were included in the derivation of the sequence of CG57151_01. The sequence is predicted to be expressed in the following tissues because of the expression pattern of (GENBANK-ID: pir-id:CSHUA peptidylprolyl isomerase) a closely related peptidylprolyl isomerase homolog in species human: Kidney, Lung. This information was derived by determining the tissue sources of the sequences that were included in the invention including but not limited to SeqCalling sources, Public EST sources, Literature sources, and/or RACE sources.

The disclosed NOV37 polypeptide has homology to the amino acid sequences shown in the BLASTP data listed in Table 37C.

| Table 37C. BLAST results for NOV37 | | | | | |
|---|---|---|---|---|---|
| Gene Index/ Identifier | Protein/ Organism | Length (aa) | Identity (%) | Positives (%) | Expect |
| gi\|12804335\|gb\|AAHO 3026.1 AAH03026 (BC003026) | protein for IMAGE:2823490)[Homo sapiens] | 174 | 139/171 (81%) | 147/171 (85%) | E3-71 |
| gi 4033689 sp P0437 4 CYPH BOVIN | PEPTIDYL-PROLYL CIS-TRANS ISOMERASE A (PPIASE) (ROTAMASE) (CYCLOPHILIN A) (CYCLOSPORIN A-BINDING PROTEIN) | 164 | 137/162 (84%) | 142/162 (87%) | 4e-70 |
| gi\|10863927\|ref\|NP 066953.11 (NM_021130) | peptidylprolyl isomerase A (cyclophilin A) [Homo sapiens] | 165 | 136/162 (83%) | 142/162 (86%) | 1e-69 |
| gi\|68401\|pir\|\|CSBOA B | peptidylprolyl isomerase (EC 5.2.1.8) A -bovine | 163 | 136/161 (84%) | 141/161 (87%) | 2e-69 |
| gi\|13937981\|gb\|AAH0 7104.1\|AAH07104 (BC007104 | peptidylprolyl isomerase A (cyclophilin A) [Homo sapiens] | 165 | 136/162 (83%) | 141/162 (86%) | 2e-69 |

Table 37D lists the domain descriptions from DOMAIN analysis results against NOV37. This indicates that the NOV37 sequence has properties similar to those of other proteins known to contain this domain.

| Table 37D. Domain Analysis of NOV37 | | |
|---|---|---|
| gnl\|Pfam\|pfam00160, pro_isomerase, Cyclophilin type peptidyl-prolyl cis-trans isomerase | | |
| | CD-Length = 162 residues, 97.5% aligned | |
| | | Score = 191 bits (484), Expect = 5e-50 |

The disclosed NOV37 nucleic acid of the invention encoding a Cis/Trans Peptidyl Prolyl Isomerase-like protein includes the nucleic acid whose sequence is provided in Table 37A or a fragment thereof. The invention also includes a mutant or variant nucleic acid any of whose bases may be changed from the corresponding base shown in Table 37A while still encoding a protein that maintains its Cis/Trans Peptidyl Prolyl Isomerase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 15 percent of the bases may be so changed.

The disclosed NOV37 protein of the invention includes the Cis/Trans Peptidyl Prolyl Isomerase-like protein whose sequence is provided in Table 37B. The invention also includes a mutant or variant protein any of whose residues may be changed from the corresponding residue shown in Table 37B while still encoding a protein that maintains its Cis/Trans Peptidyl Prolyl Isomerase-like activities and physiological functions, or a functional fragment thereof. In the mutant or variant protein, up to about 15 percent of the residues may be so changed.

The invention further encompasses antibodies and antibody fragments, such as F_{ab} or (F_{ab})₂, that bind imununospecifically to any of the proteins of the invention.

The above defined information for this invention suggests that this Cis/Trans Peptidyl Prolyl Isomerase-like protein (NOV37) may function as a member of a "Cis/Trans Peptidyl Prolyl Isomerase family". Therefore, the NOV37 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV37 nucleic acids and proteins of the invention are useful in potential therapeutic applications implicated in diseases including but not limited to various pathologies and disorders as indicated below. For example, a cDNA encoding the Cis/Trans Peptidyl Prolyl Isomerase-like protein (NOV37) may be useful in gene therapy, and the Cis/Trans Peptidyl Prolyl Isomerase-like protein (NOV37) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV37 nucleic acid encoding the Cis/Trans Peptidyl Prolyl Isomerase-like protein of the invention, or fragments thereof, may further be useful in diagnostic applications, wherein the presence or amount of the nucleic acid or the protein are to be assessed.

NOV37 nucleic acids and polypeptides are further useful in the generation of antibodies that bind immuno-specifically to the novel NOV37 substances for use in therapeutic or diagnostic methods. These antibodies may be generated according to methods known in the art, using prediction from hydrophobicity charts, as described in the "Anti-NOVX Antibodies" section below. The disclosed NOV37 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV38

A disclosed NOV38 nucleic acid of 600 nucleotides (also referred to as CG57153-01) encoding a Cis/Trans Peptidyl Prolyl Isomerase-like protein is shown in Table 38A. The start and stop codons are in bold letters.

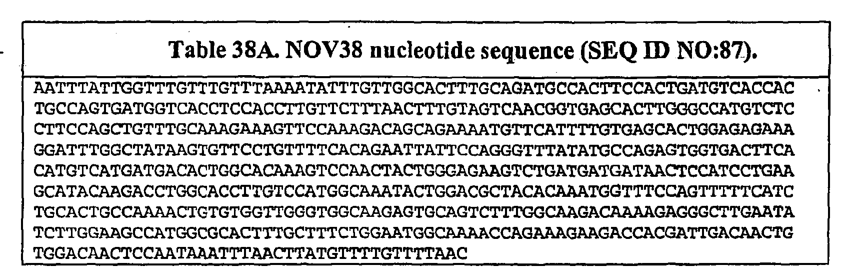

In a search of public sequence databases, the NOV38 nucleic acid sequence, located on chromsome 16 has 456 of 564 bases (80%) identical to a gb:GENBANK-ID:AK026569|acc:AK026569.1 mRNA from Homo sapiens (Homo sapiens cDNA: FLJ22916 fis, clone KAT06406, highly similar to HSCYCR Human mRNA for T-cell cyclophilin. Public nucleotide databases include all GenBank databases and the GeneSeq patent database.

The disclosed NOV38 polypeptide (SEQ ID NO:88) encoded by SEQ ID NO:87 has 176 amino acid residues and is presented in Table 38B using the one-letter amino acid code. Signal P, Psort and/or Hydropathy results predict that NOV38 has no signal peptide and is likely to be localized in the cytoplasm with a certainty of 0.5500.

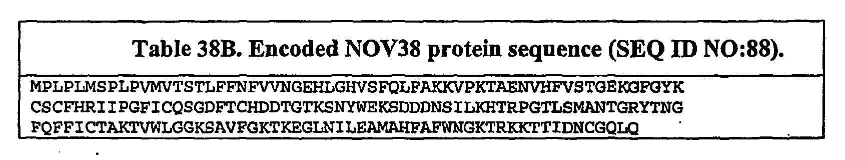

A search of sequence databases reveals that the NOV38 amino acid sequence has 113 of 164 amino acid residues (68%) identical to, and 127 of 164 amino acid residues (77%) similar to, the 164 amino acid residue ptnr:SPTREMBL-ACC:Q9TTC6 protein from Oryctolagus cuniculus (Rabbit) (CYCLOPHILIN 18). Public amino acid databases include the GenBank databases, SwissProt, PDB and PIR.

NOV38 is expressed in at least Bone Marrow, Brain, Cartilage, Cochlea, Colon, Epidermis, Kidney, Lung, Mammary gland/Breast, Ovary, Pancreas, Prostate, Stomach, Testis, Thymus, Umbilical Vein, Uterus, Vulva, and Whole Organism. Expression information was derived from the tissue sources of the sequences that were included in the derivation of the sequence of CG57153_01.The sequence is predicted to be expressed in the following tissues because of the expression pattern of (GENBANK-ID: gb:GENBANK-ID:AK026569|acc:AK026569.1) a closely related Homo sapiens cDNA: FLJ22916 fis, clone KAT06406, highly similar to HSCYCR Human mRNA for T-cell cyclophilin homolog in species Homo sapiens. This information was derived by determining the tissue sources of the sequences that were included in the invention including but not limited to SeqCalling sources, Public EST sources, Literature sources, and/or RACE sources.

The disclosed NOV38 polypeptide has homology to the amino acid sequences shown in the BLASTP data listed in Table 38C.

| **Table 38C. BLAST results for NOV38** | | | | | |
|---|---|---|---|---|---|
| Gene Index/ Identifier | Protein/ Organism | Length (aa) | Identity (%) | Positives (%) | Expect |
| gi\|12804335\|gb\|AAH0 3026.1\|AAR03026 (BC003026) | (protein for IMAGE:2823490) [Homo sapiens] | 174 | 115/168 (68%) | 128/168 (75%) | 6e-58 |
| gi\|13937981\|qb\|AAH0 7104.1\|AAH07104 (BC007104) | peptidylprolyl isomerase A (cyclophilin A) [Homo sapiens] | 165 | 114/165 (69%) | 127/165 (76%) | 2e-57 |
| gi\|10863927\|ref\|NP066953.1 (NM_021130) | peptidylprolyl isomerase A (cyclophilin A) [Homo sapiens] | 165 | 114/165 (69%) | 127/165 (76%) | 4e-57 |
| gi\|4033689\|sp\|P04374\|CYPH BOVIN | PEPTIDYL-PROLYL CIS-TRANS ISOMERASE A (PPIASE) (ROTAMASE) (CYCLOPHILIN A) (CYCLOSPORIN A-BINDING PROTEIN) | 154 | 114/164 (69%) | 126/164 (76%) | 4e-57 |
| gi\|6651171\|gb\|AAF22 215.1\|AF139893 1 (AF139893) | cyclophilin 18 [Oryctolagus cuniculus] | 154 | 113/164 (68%) | 127/164 (76%) | 7e-57 |

Table 38D lists the domain descriptions from DOMAIN analysis results against NOV38. This indicates that the NOV38 sequence has properties similar to those of other proteins known to contain this domain.

| **Table 38D. Domain Analysis of NOV 38** | | |
|---|---|---|
| gnl\|Pfam\|pfam00160, pro_isomerase, Cyclophilin type peptidyl-prolyl cis-trans isomerase. | | |
| | CD-Length = 162 residues, 100.0% aligned | |
| | | Score = 171 bits (433), Expect = 3e-44 |

The disclosed NOV38 nucleic acid of the invention encoding a Cis/Trans Peptidyl Prolyl Isomerase-like protein includes the nucleic acid whose sequence is provided in Table 38A or a fragment thereof. The invention also includes a mutant or variant nucleic acid any of whose bases may be changed from the corresponding base shown in Table 38A while still encoding a protein that maintains its Cis/Trans Peptidyl Prolyl Isomerase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 20 percent of the bases may be so changed.

The disclosed NOV38 protein of the invention includes the Cis/Trans Peptidyl Prolyl Isomerase-like protein whose sequence is provided in Table 38B. The invention also includes a mutant or variant protein any of whose residues may be changed from the corresponding residue shown in Table 38B while still encoding a protein that maintains its Cis/Trans Peptidyl Prolyl Isomerase-like activities and physiological functions, or a functional fragment thereof. In the mutant or variant protein, up to about 32 percent of the residues may be so changed.

The invention further encompasses antibodies and antibody fragments, such as F_{ab} or (F_{ab})₂, that bind immunospecifically to any of the proteins of the invention.

The above defined information for this invention suggests that this Cis/Trans Peptidyl Prolyl Isomerase-like protein (NOV38) may function as a member of a "Cis/Trans Peptidyl Prolyl Isomerase family". Therefore, the NOV38 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV38 nucleic acids and proteins of the invention are useful in potential therapeutic applications implicated in diseases including but not limited to various pathologies and disorders as indicated below. For example, a cDNA encoding the Cis/Trans Peptidyl Prolyl Isomerase-like protein (NOV38) may be useful in gene therapy, and the Cis/Trans Peptidyl Prolyl Isomerase-like protein (NOV38) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV38 nucleic acid encoding the Cis/Trans Peptidyl Prolyl Isomerase-like protein of the invention, or fragments thereof, may further be useful in diagnostic applications, wherein the presence or amount of the nucleic acid or the protein are to be assessed.

NOV38 nucleic acids and polypeptides are further useful in the generation of antibodies that bind immuno-specifically to the novel NOV38 substances for use in therapeutic or diagnostic methods. These antibodies may be generated according to methods known in the art, using prediction from hydrophobicity charts, as described in the "Anti-NOVX Antibodies" section below. The disclosed NOV38 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV39

A disclosed NOV39 nucleic acid of 600 nucleotides (also referred to as CG57155-01) encoding a Cis/Trans Peptidyl Prolyl Isomerase-like protein is shown in Table 39A.

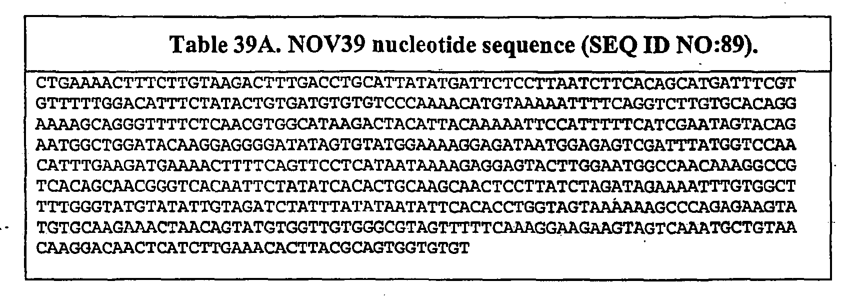

In a search of public sequence databases, the NOV39 nucleic acid sequence, located on chromsome 6 has 257 of 397 bases (64%) identical to a gb:GENBANK-ID:AF043642iacc:AF043642.1 mRNA from Rattus norvegicus (Rattus norvegicus matrin cyclophilin (matrin-cyp) mRNA, complete cds). Public nucleotide databases include all GenBank databases and the GeneSeq patent database.

The disclosed NOV39 polypeptide (SEQ ID NO:90) encoded by SEQ ID NO:89 has 180 amino acid residues and is presented in Table 39B using the one-letter amino acid code. Signal P, Psort and/or Hydropathy results predict that NOV39 has a signal peptide and is likely to be localized extracellularly with a certainty of 0.5128. The most likely cleavage site for a NOV39 peptide is between amino acids 19 and 20.

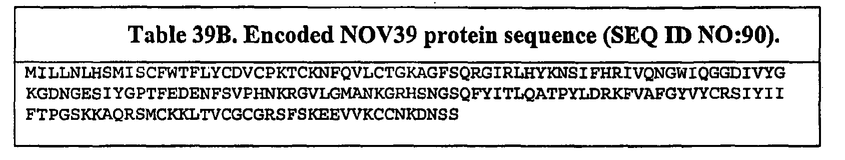

A search of sequence databases reveals that the NOV39 amino acid sequence has 70 of 87 amino acid residues (80%) identical to, and 80 of 87 amino acid residues (91%) similar to, the 278 amino acid residue ptnr:TREMBLNEW-ACC:BAB29003 protein from Mus musculus (Mouse) (ADULT MALE HIPPOCAMPUS CDNA, RIKEN FULL-LENGTH ENRICHED LIBRARY, CLONE:2900084F20, FULL INSERT SEQUENCE). Public amino acid databases include the GenBank databases, SwissProt, PDB and PIR.

NOV39 is expressed in at least: Kidney, Lung, Lymphoid tissue, Mammary gland/Breast, Oviduct/Uterine Tube/Fallopian tube, Testis, Whole Organism. Expression information was derived from the tissue sources of the sequences that were included in the derivation of the sequence of CG57155_01. The sequence is predicted to be expressed in the following tissues because of the expression pattern of (GENBANK-ID: gb:GENBANK-ID:AF043642|acc:AF043642.1) a closely related Rattus norvegicus matrin cyclophilin (matrin-cyp) mRNA, complete cds homolog in species Rattus norvegicus: Kidney, Lung, Lymphoid tissue. This information was derived by determining the tissue sources of the sequences that were included in the invention including but not limited to SeqCalling sources, Public EST sources, Literature sources, and/or RACE sources.

The disclosed NOV39 polypeptide has homology to the amino acid sequences shown in the BLASTP data listed in Table39C.

| **Table 39C. BLAST results for NOV39** | | | | | |
|---|---|---|---|---|---|
| Gene Index/ Identifier | Protein/ Organism | Length (aa) | Identity (%) | Positives (%) | Expect |
| gi\|12851324\|db\|BAB 29003.1 \| (AK013818) | similar to NK-TUMOR RECOGNITION PROTEIN (NATURAL-KILLER CELLS CYCLOPHILIN-RELATED PROTEIN) (NK-TR PROTEIN) [Mus musculue] | 278 | 70/87 (80%) | 80/87 (91%) | 2e-41 |
| gi\|6841028\|gb\|AAF28 867.1\| (AF121134) | cyclophilin [Schistosoma mansoni] | 181 | 65/120 (54%) | 89/120 (74%) | 3e-35 |
| gi\|13929124\|ref\|NP 113981.11 (NM_031793) | matrin cyclophilin (matrin-cyp) [Rattus norvegicus] | 752 | 64/117 (54%) | 85/117 (71%) | 1e-31 |
| gi\|6754858\|ref\|NP 0 35048.1 (NM 010918) | natural killer tumor recognition [Mus musculus] | 1482 | 63/117 (53%) | 82/117 (69%) | 2e-31 |
| gi\|8039799\|sp\|P3041 5\|NKCR MOUSE | NK-tumor recognition protein (Natural-killer cells; cyclophilin-related protein) (NK-TR protein) | 1453 | 63/117 (53%) | 82/117 (69%) | 2e-31 |

Table 39D lists the domain descriptions from DOMAIN analysis results against NOV39. This indicates that the NOV39 sequence has properties similar to those of other proteins known to contain this domain.

| **Table 39E. Domain Analysis of NOV39** |
|---|
| gnllPfamlpfam00160, pro_isomerase, Cyclophilin type peptidyl-prolyl cis-trans isomerase |
| CD-Length = 162 residues, 67.9% aligned |
| Score = 141 bits (356), Expect = 3e-35 |

The disclosed NOV39 nucleic acid of the invention encoding a Cis/Trans Peptidyl Prolyl Isomerase-like protein includes the nucleic acid whose sequence is provided in Table 39A or a fragment thereof. The invention also includes a mutant or variant nucleic acid any of whose bases may be changed from the corresponding base shown in Table 39A while still encoding a protein that maintains its Cis/Trans Peptidyl Prolyl Isomerase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments; or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 36 percent of the bases may be so changed.

The disclosed NOV39 protein of the invention includes the Cis/Trans Peptidyl Prolyl Isomerase-like protein whose sequence is provided in Table 39B. The invention also includes a mutant or variant protein any of whose residues may be changed from the corresponding residue shown in Table 39B while still encoding a protein that maintains its Cis/Trans Peptidyl Prolyl Isomerase-like activities and physiological functions, or a functional fragment thereof. In the mutant or variant protein, up to about 20 percent of the residues may be so changed.

The invention further encompasses antibodies and antibody fragments, such as F_{ab} or (F_{ab})_{2,} that bind immunospecifically to any of the proteins of the invention.

The above defined information for this invention suggests that this Cis/Trans Peptidyl Prolyl Isomerase-like protein (NOV39) may function as a member of a "Cis/Trans Peptidyl Prolyl Isomerase family". Therefore, the NOV39 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV39 nucleic acids and proteins of the invention are useful in potential therapeutic applications implicated in diseases including but not limited to various pathologies and disorders as indicated below. For example, a cDNA encoding the Cis/Trans Peptidyl Prolyl Isomerase-like protein (NOV39) may be useful in gene therapy, and the Cis/Trans Peptidyl Prolyl Isomerase-like protein (NOV39) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV39 nucleic acid encoding the Cis/Trans Peptidyl Prolyl Isomerase-like protein of the invention, or fragments thereof, may further be useful in diagnostic applications, wherein the presence or amount of the nucleic acid or the protein are to be assessed.

NOV39 nucleic acids and polypeptides are further useful in the generation of antibodies that bind immuno-specifically to the novel NOV39 substances for use in therapeutic or diagnostic methods. These antibodies may be generated according to methods known in the art, using prediction from hydrophobicity charts, as described in the "Anti-NOVX Antibodies" section below. The disclosed NOV39 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV40

A disclosed NOV40 nucleic acid of 572 nucleotides (also referred to as CG57157-01) encoding a Cis/Trans Peptidyl Prolyl Isomerase-like protein is shown in Table 40A. The start and stop codons are in bold letters.

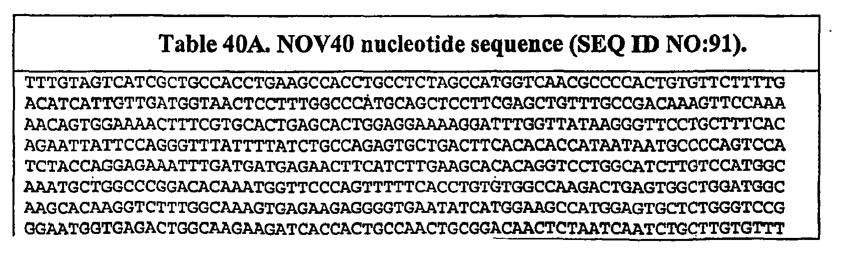

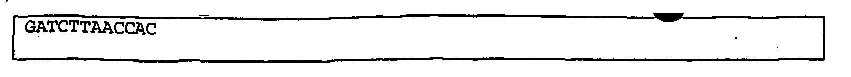

In a search of public sequence databases, the NOV40 nucleic acid sequence, located on chromsome 17 has has 446 of 535 bases (83%) identical to a gb:GENBANK-ID:HSCYCR|acc:Y00052.1 mRNA from Homo sapiens (Human mRNA for T-cell cyclophilin). Public nucleotide databases include all GenBank databases and the GeneSeq patent database.

The disclosed NOV40 polypeptide (SEQ ID NO:92) encoded by SEQ ID NO:91 has 166 amino acid residues and is presented in Table 40B using the one-letter amino acid code. Signal P, Psort and/or Hydropathy results predict that NOV40 has no signal peptide and is likely to be localized at the plasma membrane with a certainty of 0.6000.

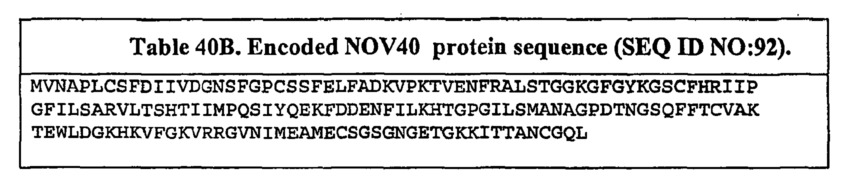

A search of sequence databases reveals that the NOV40 amino acid sequence has 122 of 166 amino acid residues (73%) identical to, and 131 of 166 amino acid residues (78%) similar to, the 165 amino acid residue ptnr:pir-id:CSHUA protein from human (peptidylprolyl isomerase (EC 5.2.1.8) A). Public amino acid databases include the GenBank databases, SwissProt, PDB and PIR.

NOV40 is expressed in at least Brain. Expression information was derived from the tissue sources of the sequences that were included in the derivation of the sequence of CG57157_01.The sequence is predicted to be expressed in the following tissues because of the expression pattern of (GENBANK-ID: gb:GENBANK-ID:HSCYCR|acc:Y00052.1) a closely related Human mRNA for T-cell cyclophilin homolog in species Homo sapiens: Brain. This information was derived by determining the tissue sources of the sequences that were included in the invention including but not limited to SeqCalling sources, Public EST sources, Literature sources, and/or RACE sources.

The disclosed NOV40 polypeptide has homology to the amino acid sequences shown in the BLASTP data listed in Table 40C.

| **Table 40C. BLAST results for NOV40** | | | | | |
|---|---|---|---|---|---|
| Gene Index/ Identifier | Protein/ Organism | Length (aa) | Identity (%) | Positives (%) | Expect |
| gi\|14743515\|ref\|XP 017224.2\| (XM_017224) | hypothetical protein XP_017224 [Homo sapiens] | 165 | 121/166 (72%) | 130/166 (77%) | e-59 |
| gi\|12804335\|gb\|AAH0 3026.1\|AAH03026 (BC003026) | (protein for IMAGE:2823490) [Homo sapiens] | 174 | 122/166 (73%) | 131/166 (78%) | e-59 |
| gi\|4033689\|sp\|P0437 4\|CYPH BOVIN | PEPTIDYL-PROLYL CIS-TRANS ISOMERASE A (PPIASE) (ROTAMASE) (CYCLOPHILIN A) (CYCLOSPORIN A-BINDING PROTEIN) | 164 | 122/166 (73%) | 131/166 (78%) | 4e-59 |
| gi\|10863927\|ref\|NP 066953.1\| (NM_021130) | peptidylprolyl isomerase A (cyclophilin A) [Homo sapiens] | 165 | 122/166 (73%) | 131/166 (78%) | 5e-59 |
| gi\|168401\|pir\|\|CSBOA B | peptidylprolyl isomerase (EC 5.2.1.8) A - bovine | 163 | 119/162 (73%) | 128/162 (78%) | 9e-59 |

Table 40D lists the domain descriptions from DOMAIN analysis results against NOV40. This indicates that the NOV40 sequence has properties similar to those of other proteins known to contain this domain.

| **Table 40D. Domain Analysis ofNOV40** | | |
|---|---|---|
| gnl\|Pfam\|pfam00160, pro_isomerase, Cyclophilin type peptidyl-prolyl cis-trans isomerase | | |
| | CD-Length = 162 residues, 99.4% aligned | |
| | | Score = 185 bits (469), Expect = 2e-48 |

The disclosed NOV40 nucleic acid of the invention encoding a Cis/Trans Peptidyl Prolyl Isomerase-like protein includes the nucleic acid whose sequence is provided in Table 40A or a fragment thereof. The invention also includes a mutant or variant nucleic acid any of whose bases may be changed from the corresponding base shown in Table 40A while still encoding a protein that maintains its Cis/Trans Peptidyl Prolyl Isomerase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 17 percent of the bases may be so changed.

The disclosed NOV40 protein of the invention includes the Cis/Trans Peptidyl Prolyl Isomerase-like protein whose sequence is provided in Table 40B. The invention also includes a mutant or variant protein any of whose residues may be changed from the corresponding residue shown in Table 40B while still encoding a protein that maintains its Cis/Trans Peptidyl Prolyl Isomerase-like activities and physiological functions, or a functional fragment thereof. In the mutant or variant protein, up to about 27 percent of the residues may be so changed.

The invention further encompasses antibodies and antibody fragments, such as F_{ab} or (F_{ab})₂, that bind immunospecifically to any of the proteins of the invention.

The above defined information for this invention suggests that this Cis/Trans Peptidyl Prolyl Isomerase-like protein (NOV40) may function as a member of a "Cis/Trans Peptidyl Prolyl Isomerase family". Therefore, the NOV40 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV40 nucleic acids and proteins of the invention are useful in potential therapeutic applications implicated in diseases including but not limited to various pathologies and disorders as indicated below. For example, a cDNA encoding the Cis/Trans Peptidyl Prolyl Isomerase-like protein (NOV40) may be useful in gene therapy, and the Cis/Trans Peptidyl Prolyl Isomerase-like protein (NOV40) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV40 nucleic acid encoding the Cis/Trans Peptidyl Prolyl Isomerase-like protein of the invention, or fragments thereof, may further be useful in diagnostic applications, wherein the presence or amount of the nucleic acid or the protein are to be assessed.

NOV40 nucleic acids and polypeptides are further useful in the generation of antibodies that bind immuno-specifically to the novel NOV40 substances for use in therapeutic or diagnostic methods. These antibodies may be generated according to methods known in the art, using prediction from hydrophobicity charts, as described in the "Anti-NOVX Antibodies" section below. The disclosed NOV40 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV41

A disclosed NOV41 nucleic acid of 525 nucleotides (also referred to as CG57159-01) encoding a Cis/Trans Peptidyl Prolyl Isomerase-like protein is shown in Table 41 A. The start and stop codons are in bold letters.

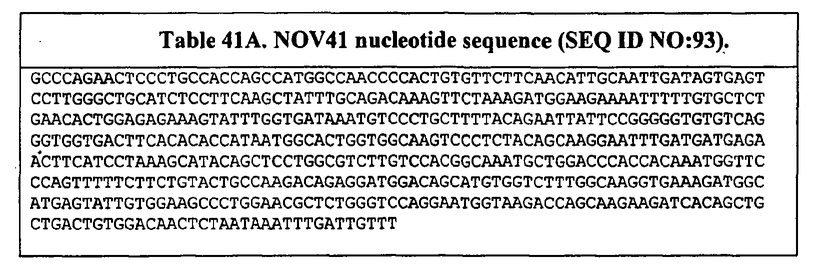

In a search of public sequence databases, the NOV41 nucleic acid sequence, located on chromsome 11 has 442 of 515 bases (85%) identical to a gb:GENBANK-ID:HSCYCR|acc:Y00052.1 mRNA from Homo sapiens (Human mRNA for T-cell cyclophilin). Public nucleotide databases include all GenBank databases and the GeneSeq patent database.

The disclosed NOV41 polypeptide (SEQ ID NO:94) encoded by SEQ ID NO :93 has 161 amino acid residues and is presented in Table 41B using the one-letter amino acid code. Signal P, Psort and/or Hydropathy results predict that NOV41 has no signal peptide and is likely to be localized in the plasma membrane with a certainty of 0.600.

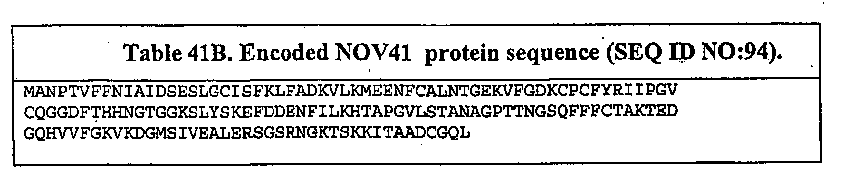

A search of sequence databases reveals that the NOV41 amino acid sequence has 125 of 164 amino acid residues (76%) identical to, and 141 of 164 amino acid residues (85%) similar to, the 165 amino acid residue ptnr:pir-id:CSHUA protein from human (peptidylprolyl isomerase (EC 5.2.1.8) A). Public amino acid databases include the GenBank databases, SwissProt, PDB and PIR.

NOV41 is expressed in at least Heart, Placenta, Stomach, Whole Organism. Expression information was derived from the tissue sources of the sequences that were included in the derivation of the sequence of CG57159_01.The sequence is predicted to be expressed in the following tissues because of the expression pattern of (GENBANK-ID: gb:GENBANK-ID:HSCYCR|acc:Y00052.1) a closely related Human mRNA for T-cell cyclophilin homolog in species Homo sapiens : signet-ring cell carcinoma cell_line. This information was derived by determining the tissue sources of the sequences that were included in the invention including but not limited to SeqCalling sources, Public EST sources, Literature sources, and/or RACE sources.

The disclosed NOV41 polypeptide has homology to the amino acid sequences shown in the BLASTP data listed in Table 41C.

| **Table 41C. BLAST results for NOV41** | | | | | |
|---|---|---|---|---|---|
| Gene Index/ Identifier | Protein/ organism | Length (aa) | Identity (%) | Positives (%) | Expect |
| gi\|12804335\|gb\|AAH0 3026.1\|AAH03026 (BC003026) | (protein for IMAGE:2823490) [Homo sapiens] | 174 | 125/164 (76%) | 141/164 (85%) | 3e-53 |
| gi\|4033689\|sp\|P0437 4\|CYPH BOVIN | PEPTIDYL-PROLYL CIS-TRANS ISOMERASE A (PPIASE) (ROTAMASE) (CYCLOPHILIN A) (CYCLOSPORIN A-BINDING PROTEIN) | 164 | 125/164 (76%) | 141/164 (85%) | 9e-53 |
| gi\|13937981\|gb\|AAH0 7104.1\|AAH07104 (BC007104) | peptidylprolyl isomerase A (cyclophilin A) [Homo sapiens] | 165 | 125/164 (76%) | 141/164 (85%) | 9e-53 |
| gi\|10863927\|ref\|NP 066953.1\| (NM_021130) | peptidylprolyl isomerase A (cyclophilin A) [Homo sapiens] | 165 | 125/164 (76%) | 141/164 (85%) | le-52 |
| gi\|68401\|pir\|\|CSBOA B | peptidylprolyl isomerase (EC 5.2.1.8) A - bovine | 163 | 124/162 (76%) | 140/162 (85%) | 4e-52 |

Table 41D lists the domain descriptions from DOMAIN analysis results against NOV41. This indicates that the NOV41 sequence has properties similar to those of other proteins known to contain this domain.

| **Table 41D. Domain Analysis of NOV41** |
|---|
| gnl\|Pfam\|pfam00160, pro_isomerase, Cyclophilin type peptidyl-prolyl cis-trans isomerase |
| CD-Length = 162 residues, 100.0% aligned |
| Score = 177 bits (448), Expect = 5e-46 |

The disclosed NOV41 nucleic acid of the invention encoding a Cis/Trans Peptidyl Prolyl Isomerase-like protein includes the nucleic acid whose sequence is provided in Table 41 A or a fragment thereof. The invention also includes a mutant or variant nucleic acid any of whose bases may be changed from the corresponding base shown in Table 41A while still encoding a protein that maintains its Cis/Trans Peptidyl Prolyl Isomerase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 15 percent of the bases may be so changed.

The disclosed NOV41 protein of the invention includes the Cis/Trans Peptidyl Prolyl Isomerase-like protein whose sequence is provided in Table 41B. The invention also includes a mutant or variant protein any of whose residues may be changed from the corresponding residue shown in Table 41B while still encoding a protein that maintains its Cis/Trans Peptidyl Prolyl Isomerase-like activities and physiological functions, or a functional fragment thereof. In the mutant or variant protein, up to about 24 percent of the residues may be so changed.

The invention further encompasses antibodies and antibody fragments, such as F_{ab} or (F_{ab})₂, that bind immunospecifically to any of the proteins of the invention.

The above defined information for this invention suggests that this Cis/Trans Peptidyl Prolyl Isomerase-like protein (NOV41) may function as a member of a "Cis/Trans Peptidyl Prolyl Isomerase family". Therefore, the NOV41 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV41 nucleic acids and proteins of the invention are useful in potential therapeutic applications implicated in diseases including but not limited to various pathologies and disorders as indicated below. For example, a cDNA encoding the Cis/Trans Peptidyl Prolyl Isomerase-like protein (NOV41) may be useful in gene therapy, and the Cis/Trans Peptidyl Prolyl Isomerase-like protein (NOV41) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV41 nucleic acid encoding the Cis/Trans Peptidyl Prolyl Isomerase-like protein of the invention, or fragments thereof, may further be useful in diagnostic applications, wherein the presence or amount of the nucleic acid or the protein are to be assessed.

NOV41 nucleic acids and polypeptides are further useful in the generation of antibodies that bind immuno-specifically to the novel NOV41 substances for use in therapeutic or diagnostic methods. These antibodies may be generated according to methods known in the art, using prediction from hydrophobicity charts, as described in the "Anti-NOVX Antibodies" section below. The disclosed NOV41 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV42

NOV42 includes two Cis/Trans Peptidyl Prolyl Isomerase-like proteins disclosed below. The disclosed sequences have been named NOV42a and NOV42b.

### NOV42a

A disclosed NOV42a nucleic acid of 720 nucleotides (also referred to as CG57226-01) encoding a Cis/Trans Peptidyl Prolyl Isomerase -like protein is shown in Table 42A. The start and stop codons are in bold letters.

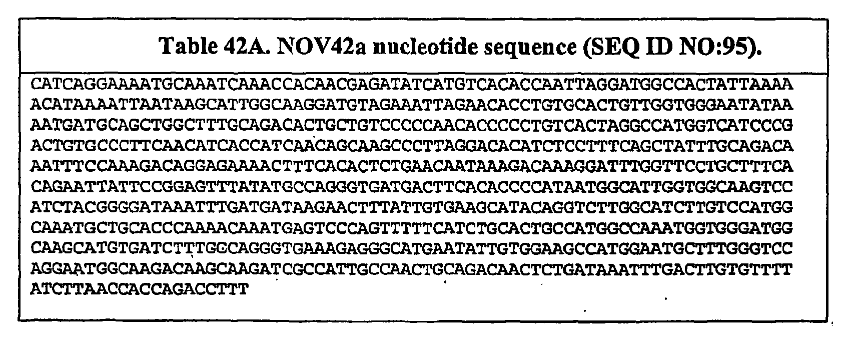

In a search of public sequence databases, the NOV42a nucleic acid sequence, located on chromsome 11 has 338 of 387 bases (87%) identical to a gb:GENBANK-ID:AK026569|acc:AK026569.1 mRNA from Homo sapiens (Homo sapiens cDNA: FLJ22916 fis, clone KAT06406, highly similar to HSCYCR Human mRNA for T-cell cyclophilin). Public nucleotide databases include all GenBank databases and the GeneSeq patent database.

The disclosed NOV42a polypeptide (SEQ ID NO:96) encoded by SEQ ID NO:95 has 160 amino acid residues and is presented in Table 42B using the one-letter amino acid code. Signal P, Psort and/or Hydropathy results predict that NOV42a has no signal peptide and is likely to be localized in the microbody with a certainty of 0.6400.

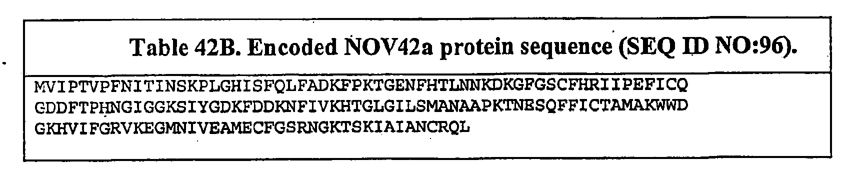

A search of sequence databases reveals that the NOV42a amino acid sequence has 118 of 164 amino acid residues (71%) identical to, and 135 of 164 amino acid residues (82%) similar to, the 165 amino acid residue ptnr:pir-id:CSHUA protein from human (peptidylprolyl isomerase (EC 5.2.1.8) A). Public amino acid databases include the GenBank databases, SwissProt, PDB and PIR.

NOV42a is expressed in at least Brain and Peripheral Blood. Expression information was derived from the tissue sources of the sequences that were included in the derivation of the sequence of CG57226_01. The sequence is predicted to be expressed in the following tissues because of the expression pattern of (GENBANK-ID: gb:GENBANK-ID:AK026569|acc:AK026569.1) a closely related Homo sapiens cDNA: FLJ22916 fis, clone KAT06406, highly similar to HSCYCR Human mRNA for T-cell cyclophilin homolog in species Homo sapiens : signet-ring cell carcinoma cell_line. This information was derived by determining the tissue sources of the sequences that were included in the invention including but not limited to SeqCalling sources, Public EST sources, Literature sources, and/or RACE sources.

### NOV42b

A disclosed NOV42b nucleic acid of 600 nucleotides (also referred to as CG57226-02) encoding a Cis/Trans Peptidyl Prolyl Isomerase -like protein is shown in Table 42C. The start and stop codons are in bold letters.

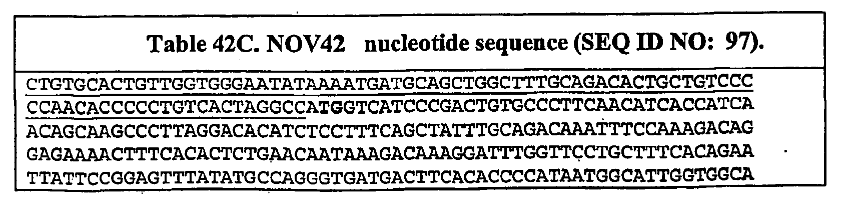

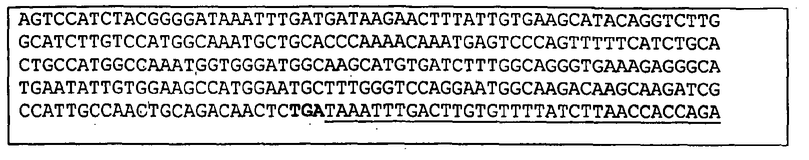

In a search of public sequence databases, the NOV42b nucleic acid sequence, located on chromsome 11 has 335 of 382 bases (87%) identical to a gb:GENBANK-ID:AK026569|acc:AK026569.1 mRNA from Homo sapiens (Homo sapiens cDNA: FLJ22916 fis, clone KAT06406, highly similar to HSCYCR Human mRNA for T-cell cyclophilin). Public nucleotide databases include all GenBank databases and the GeneSeq patent database.

The disclosed NOV42b polypeptide (SEQ ID NO:98) encoded by SEQ ID NO:97 has 160 amino acid residues and is presented in Table 42D using the one-letter amino acid code. Signal P, Psort and/or Hydropathy results predict that NOV42b has no signal peptide and is likely to be localized in the microbody with a certainty of 0.6400.

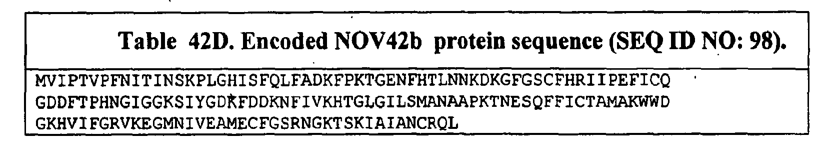

A search of sequence databases reveals that the NOV42b amino acid sequence has 118 of 164 amino acid residues (71%) identical to, and 135 of 164 amino acid residues (82%) similar to, the 165 amino acid residue ptnr:pir-id:CSHUA protein from human (peptidylprolyl isomerase (EC 5.2.1.8) A). Public amino acid databases include the GenBank databases, SwissProt, PDB and PIR.

NOV42b is expressed in at least Brain, and Peripheral Blood. This information was derived by determining the tissue sources of the sequences that were included in the invention including but not limited to SeqCalling sources, Public EST sources, Literature sources, and/or RACE sources.

The disclosed NOV42a polypeptide has homology to the amino acid sequences shown in the BLASTP data listed in Table 42E.

| Table 42E. BLAST results for NOV42a | | | | | |
|---|---|---|---|---|---|
| Gene Index/ Identifier | Protein/ Organism | Length (aa) | Identity (%) | Positives (%) | Expect |
| gi\|12804335\|gb\|AAH0 3026.1\|AAH03026 (BC003026 | protein for IMAGE:2823490) [Homo sapiens] | 174 | 118/164 (71%) | 135/164 (81%) | le-60 |
| gi\|4033689\|sp\|P0437 4\|CYPH BOVIN | PEPTIDYL-PROLYL CIS-TRANS ISOMERASE A (PPIASE) (ROTAMASE) (CYCLOPHILIN A) (CYCLOSPORIN A-BINDING PROTEIN) | 164 | 118/164 (71%) | 135/164 (81%) | 8e-60 |
| gi\|13937981\|gb\|AAH0 7104.1 \| AAH0 7104 (BC007104) | peptidylprolyl isomerase A (cyclophilin A) [Homo sapiens] | 165 | 118/164 (71%) | 135/164 (81%) | 9e-60 |
| gi\|14743515\|ref\|XP 017224.2\| (XM_017224) | hypothetical protein XP_017224 [Homo sapiens] | 165 | 118/164 (71%) | 135/164 (81%) | 3e-59 |

Table 42F lists the domain descriptions from DOMAIN analysis results against NOV 42. This indicates that the NOV42 sequence has properties similar to those of other proteins known to contain this domain.

| **Table 42F Domain Analysis of NOV42** | |
|---|---|
| gnl\|pfam\|pfam00160, pro_isomerase, Cyclophilin type peptidyl-prolyl cis-trans isomerase. | |
| | CD-Length = 162 residues, 100.0% aligned |
| | Score = 185 bits (469), Expect = 2e-48 |

The disclosed NOV42 nucleic acid of the invention encoding a Cis/Trans Peptidyl Prolyl Isomerase-like protein includes the nucleic acid whose sequence is provided in Table 42A or 42C or a fragment thereof. The invention also includes a mutant or variant nucleic acid any of whose bases may be changed from the corresponding base shown in Table 42A or 42C while still encoding a protein that maintains its Cis/Trans Peptidyl Prolyl Isomerase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. bases may be so change

The disclosed NOV42 protein of the invention includes the Cis/Trans Peptidyl Prolyl Isomerase-like protein whose sequence is provided in.Table 42B or 42D. The invention also includes a mutant or variant protein any of whose residues may be changed from the corresponding residue shown in Table 42B or 42D while still encoding a protein that maintains its Cis/Trans Peptidyl Prolyl Isomerase-like activities and physiological functions, or a functional fragment thereof. In the mutant or variant protein, up to about 29 percent of the residues may be so changed.

The invention further encompasses antibodies and antibody fragments, such as F_{ab} or (F_{ab})₂, that bind immunospecifically to any of the proteins of the invention.

The above defined information for this invention suggests that this Cis/Trans Peptidyl Prolyl Isomerase-like protein (NOV42) may function as a member of a "Cis/Trans Peptidyl Prolyl Isomerase family". Therefore, the NOV42 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV42 nucleic acids and proteins of the invention are useful in potential therapeutic applications implicated in diseases including but not limited to various pathologies and disorders as indicated below. For example, a cDNA encoding the Cis/Trans Peptidyl Prolyl Isomerase-like protein (NOV42) may be useful in gene therapy, and the Cis/Trans Peptidyl Prolyl Isomerase-like protein (NOV42) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV42 nucleic acid encoding the Cis/Trans Peptidyl Prolyl Isomerase-like protein of the invention, or fragments thereof, may further be useful in diagnostic applications, wherein the presence or amount of the nucleic acid or the protein are to be assessed.

NOV42 nucleic acids and polypeptides are further useful in the generation of antibodies that bind immuno-specifically to the novel NOV42 substances for use in therapeutic or diagnostic methods. These antibodies may be generated according to methods known in the art, using prediction from hydrophobicity charts, as described in the "Anti-NOVX Antibodies" section below. The disclosed NOV42 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV43

A disclosed NOV43 nucleic acid of 3146 nucleotides (also referred to as CG57538-01) encoding a ceruloplasmin-like protein is shown in Table 43A. The start and stop codons are in bold letters.

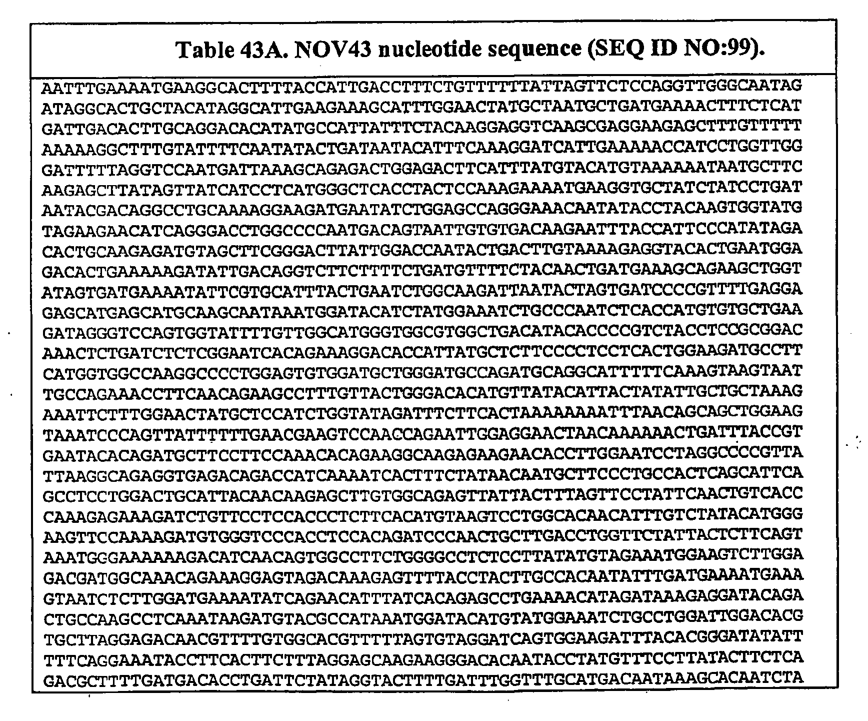

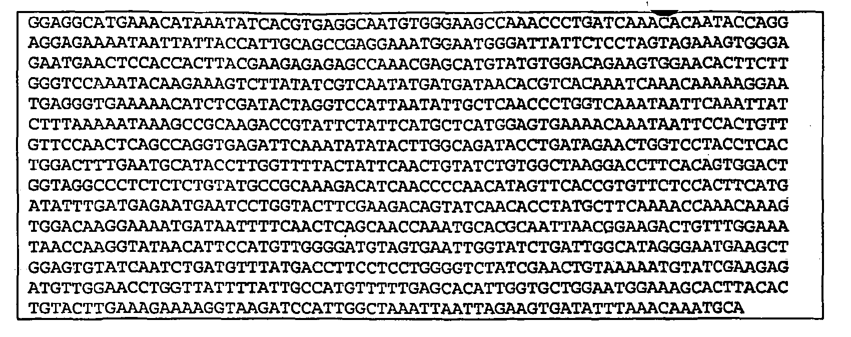

In a search of public sequence databases, the NOV43 nucleic acid sequence, located on chromsome 3 has 1113 of 1697 bases (65%) identical to a gb:GENBANK-ID:HUMCERP|acc:M13699.1 mRNA from Homo sapiens (Human ceruloplasmin (ceruloplasmin) mRNA, complete cds). Public nucleotide databases include all GenBank databases and the GeneSeq patent database.

The disclosed NOV43 polypeptide (SEQ ID NO:100) encoded by SEQ ID NO:99 has 1036 amino acid residues and is presented in Table 43B using the one-letter amino acid code. Signal P, Psort and/or Hydropathy results predict that NOV43 has a signal peptide and is likely to be localized extracellularly with a certainty of 0.4085. The most likely cleavage site for a NOV43 peptide is between amino acids 19 and 20.

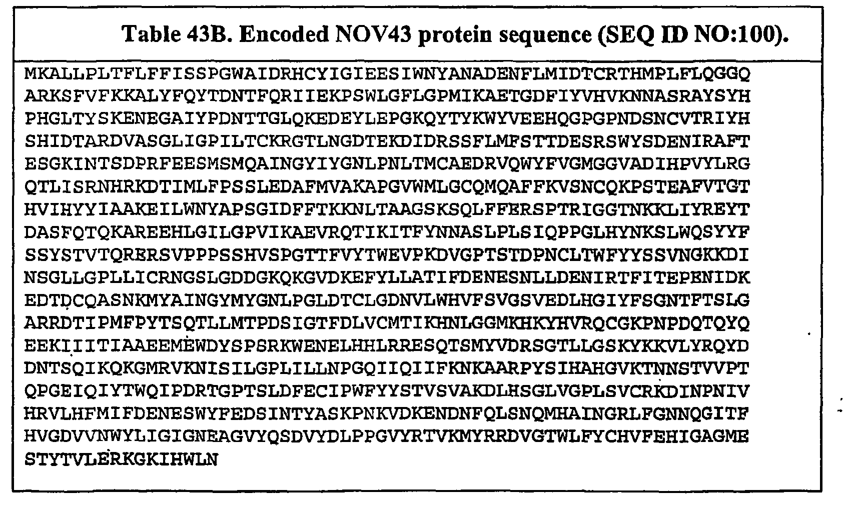

A search of sequence databases reveals that the NOV43 amino acid sequence has 548 of 994 amino acid residues (55%) identical to, and 719 of 994 amino acid residues (72%) similar to, the 1069 amino acid residue ptnr:pir-id:KUHU protein from human (ceruloplasmin (EC 1.16.3.1) precursor [validated]). Public amino acid databases include the GenBank databases, SwissProt, PDB and PIR.

NOV43 is expressed in at least salivary glands. Expression information was derived from the tissue sources of the sequences that were included in the derivation of the sequence of CG57538-01.The sequence is predicted to be expressed in the following tissues because of the expression pattern of (GENBANK-ID: gb:GENBANK-ID:HUMCERP|acc:M13699.1) a closely related Human ceruloplasmin (ceruloplasmin) mRNA, complete cds homolog in species Homo sapiens :liver, secreted into plasma.. This information was derived by determining the tissue sources of the sequences that were included in the invention including but not limited to SeqCalling sources, Public EST sources, Literature sources, and/or RACE sources.

The disclosed NOV43 polypeptide has homology to the amino acid sequences shown in the BLASTP data listed in Table 43C.

| **Table 43C. BLAST results for NOV43** | | | | | |
|---|---|---|---|---|---|
| Gene Index/ Identifier | Protein/ Organism | Length (aa) | Identity (%) | Positives (%) | Expect |
| gi\|1070458 \|pir \| \|KUH U | ferroxidase (EC 1.16.3.1) precursor - human | 1069 | 579/1068 (54%) | 760/1068 (70%) | 0.0 |
| gi \|14557485 \|ref \|NP 0 00087.1\| (NM_000096) | ceruloplasmin (ferroxidase); Ceruloplasmin [Homo sapiens] | 1065 | 578/1066, (54%) | 758/1066 (70%) | 0.0 |
| gi \|1942284 \|pd \|1KCW ┴ | X-Ray Crystal Structure of Human Ceruloplasmin At 3.0 Angstroms | 1048 | 568/1046 (54%) | 746/1046 (71%) | 0.0 |
| gi\|5281319\|gb\|AAD41 477.1\|AF134814 1 (AF134814) | (AF134814) ceruloplasmin [Ovis aries | 1048 | 577/1054 (54%) | 742/1054 (69%) | 0.0 |
| gi\|6680997\|ref\|NP 0 31778.1\| (NM 007752) | gi\|6680997\|ref\|NP 031778.1\| (NM_007752 | 1062 | 560/1062 (52%) | 737/1062 (68%) | 0.0 |

Table 43D lists the domain descriptions from DOMAIN analysis results against NOV43. This indicates that the NOV43 sequence has properties similar to those of other proteins known to contain this domain.

| **Table 43D. Domain Analysis of NOV43** | | |
|---|---|---|
| gnl\|Pfam\|pfam00394, Cu-oxidase, Multicopper oxidase. Many of the proteins in this family contain multiple similar copies of this plastocyanin-like domain. | | |
| | CD-Length = 135 residues, 90.4% aligned | |
| | | Score = 37.7 bits (86), Expect = 0.003 |

In Wilson disease, the basal ganglia and liver undergo changes that express themselves in neurologic manifestations and signs of cirrhosis, respectively. A disturbance in copper metabolism is somehow involved in the mechanism. Low ceruloplasmin is found in the serum. Shokeir and Shreffler (1969) advanced the hypothesis that ceruloplasmin functions in enzymatic transfer of copper to copper-containing enzymes such as cytochrome oxidase. Supporting the hypothesis was the finding of markedly reduced levels of activity of cytochrome oxidase in Wilson disease and moderate reductions in heterozygotes. An abnormality of ceruloplasmin seems to be involved in Wilson disease. The fact that individuals with hereditary ceruloplasmin deficiency have profound iron accumulation in most tissues suggests that ceruloplasmin is important for normal release of cellular iron (Mukhopadhyay et al., 1998). At least 3 variants determined by codominant alleles have been identified by starch gel electrophoresis (Shreffler et al., 1967). Human ceruloplasmin is composed of a single polypeptide chain (Takahashi et al., 1984).

The disclosed NOV43 nucleic acid of the invention encoding a ceruloplasmin-like protein includes the nucleic acid whose sequence is provided in Table 43A or a fragment thereof. The invention also includes a mutant or variant nucleic acid any of whose bases may be changed from the corresponding base shown in Table 43A while still encoding a protein that maintains its ceruloplasmin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to, enhance the chemical stability of the modified nucleic acid, such that they may be used, for example, as antisense binding nucleic acids in therapeutic applications in a subject. In the mutant or variant nucleic acids, and their complements, up to about 35 percent of the bases may be so changed.

The disclosed NOV43 protein of the invention includes the ceruloplasmin-like protein whose sequence is provided in Table 43B. The invention also includes a mutant or variant protein any of whose residues may be changed from the corresponding residue shown in Table 43B while still encoding a protein that maintains its ceruloplasmin-like activities and physiological functions, or a functional fragment thereof. In the mutant or variant protein, up to about 45 percent of the residues may be so changed.

The invention further encompasses antibodies and antibody fragments, such as F_{ab} or (F_{ab})₂. that bind immunospecifically to any of the proteins of the invention.

The above defined information for this invention suggests that this ceruloplasmin-like protein (NOV43) may function as a member of a "ceruloplasmin family". Therefore, the NOV43 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV43 nucleic acids and proteins of the invention are useful in potential therapeutic applications implicated in diseases including but not limited to various pathologies and disorders as indicated below. For example, a cDNA encoding the ceruloplasmin-like protein (NOV43) may be useful in gene therapy, and the ceruloplasmin-like protein (NOV43) may be useful when administered to a subject in need thereof. By way of nonlimiting example, the compositions of the present invention will have efficacy for treatment of patients suffering from Wilson disease, dementia, diabetes, retinal degeneration, neurologic degeneration, xerostomia, or other pathologies or conditions. The NOV43 nucleic acid encoding the ceruloplasmin-like protein of the invention, or fragments thereof, may further be useful in diagnostic applications, wherein the presence or amount of the nucleic acid or the protein are to be assessed.

NOV43 nucleic acids and polypeptides are further useful in the generation of antibodies that bind immuno-specifically to the novel NOV43 substances for use in therapeutic or diagnostic methods. These antibodies may be generated according to methods known in the art, using prediction from hydrophobicity charts, as described in the "Anti-NOVX Antibodies" section below. The disclosed NOV43 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV44

NOV44 includes two Leucine rich repeat-like proteins disclosed below. The disclosed sequences have been named NOV44a and NOV44b..

### NOV44a

A disclosed NOV44a nucleic acid of 857 nucleotides (also referred to as CG57623-01) encoding a Leucine rich repeat -like protein is shown in Table 44A. The start and stop codons are in bold letters.

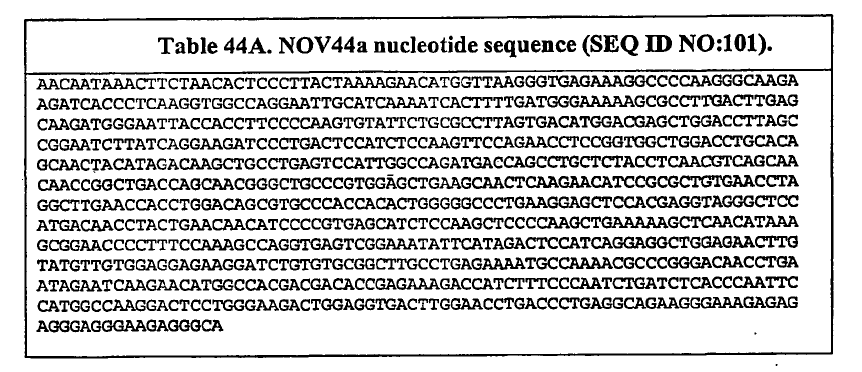

In a search of public sequence databases, the NOV44a nucleic acid sequence, located on chromsome 10 has 188 of 313 bases (60%) identical to a gb:GENBANK ID:AB016816|acc:AB016816.1 mRNA from Homo sapiens (Homo sapiens MASL1 mRNA, complete cds). Public nucleotide databases include all GenBank databases and the GeneSeq patent database.

The disclosed NOV44a polypeptide (SEQ ID NO:102) encoded by SEQ ID NO:101 has 255 amino acid residues and is presented in Table 44B using the one-letter amino acid code. Signal P, Psort and/or Hydropathy results predict that NOV44a has no signal peptide and is likely to be localized cytoplasmically with a certainty of 0.4500.

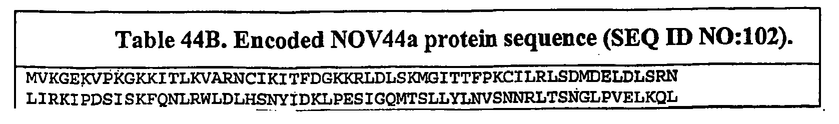

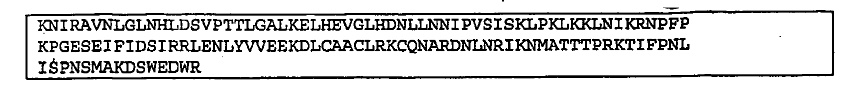

A search of sequence databases reveals that the NOV44a amino acid sequence has 211 of 255 amino acid residues (82%) identical to, and 235 of 255 amino acid residues (92%) similar to, the 262 amino acid residue ptnr:TREMBLNEW-ACC:BAB29635 protein from Mus musculus (Mouse) (ADULT MALE TESTIS CDNA, RIKEN FULL-LENGTH ENRICHED LIBRARY, CLONE:4921523N16, FULL INSERT SEQUENCE). Public amino acid databases include the GenBank databases, SwissProt, PDB and PIR.

NOV44a is expressed in at least cervix, brain, and testis. This information was derived by determining the tissue sources of the sequences that were included in the invention including but not limited to SeqCalling sources, Public EST sources, Literature sources, and/or RACE sources.

### NOV44b

A disclosed NOV44b nucleic acid of 847 nucleotides (also referred to as CG57623-01) encoding a Leucine rich repeat -like protein is shown in Table 44C. The start and stop codons are in bold letters.

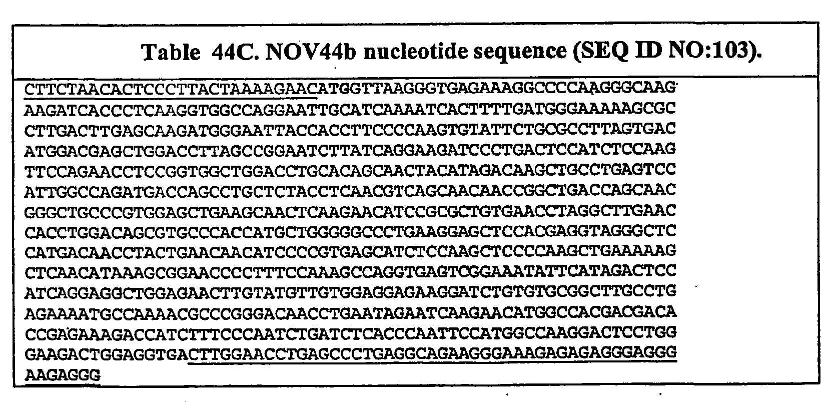

In a search of public sequence databases, the NOV nucleic acid sequence, located on chromsome 10 has 187 of 313 bases (59%) identical to a gb:GENBANK-ID:AB016816|acc:AB016816.1 mRNA from Homo sapiens (Homo sapiens MASL1 mRNA, complete cds). Public nucleotide databases include all GenBank databases and the GeneSeq patent database.

The disclosed NOV44b polypeptide (SEQ ID NO: 104) encoded by SEQ ID NO: 103 has 255 amino acid residues and is presented in Table B using the one-letter amino acid code. Signal P, Psort and/or Hydropathy results predict that NOV44b as no signal peptide and is likely to be localized cytoplasmically with a certainty of 0.4500.

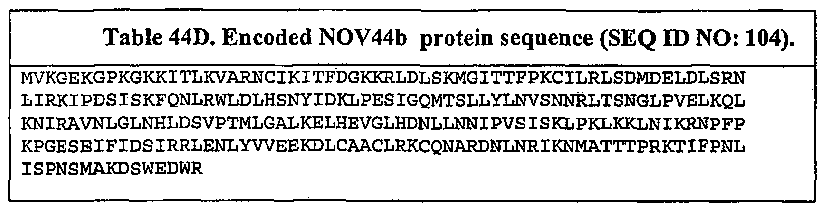

A search of sequence databases reveals that the NOV44b amino acid sequence has 211 of 255 amino acid residues (82%) identical to, and 235 of 255 amino acid residues (92%) similar to, the 262 amino acid residue ptnr:SPTREMBL-ACC:Q9CQ07 protein from Mus musculus (Mouse) (4930442L21RIK PROTEIN). Public amino acid databases include the GenBank databases, SwissProt, PDB and PIR.

NOV44b is expressed in at least cervix, brain and testis. This information was derived by determining the tissue sources of the sequences that were included in the invention including but not limited to SeqCalling sources, Public EST sources, Literature sources, and/or RACE sources.

The disclosed NOV44 polypeptide has homology to the amino acid sequences shown in the BLASTP data listed in Table 44E.

| **Table 44E. BLAST results for NOV44** | | | | | |
|---|---|---|---|---|---|
| Gene Index/ Identifier | Protein/ Organism | Length (aa) | Identity (%) | Positives (%) | Expect |
| gi\|3385762\|ref\|NP 080529.1\| (NM_026253) | RIKEN cDNA 4930442L21 [Mus musculus] | 262 | 211/255 (82%) | 235/255 (91%) | e-108 |
| gi\|12a3e968\|dbj\|BAB24391.1 \| (AK006063) | Leucine Rich Repeat containing protein-data source:Pfam, source key:PF00560, evidence:ISS-puta tive [Mus musculus] | 255 | 210/255 (82%) | 234/255 (91%) | e-108 |
| gi\|12838360\|dbj\|BAB 24176.1\| (AK005666) | Leucine Rich Repeat containing protein-data source:Pfam, source key:PF00560, evidence:ISS-puta tive [Mus musculus] | 230 | 195/228 (85%) | 214/228 (93%) | e-97 |
| gi\|10130019\|gb\|AAG1 3461.1\|AF274972 1 (AF274972) | PIDD [Homo sapiens] | 910 | 48/146 (32%) | 87/146 (58%), | 2e-18 |
| gi\|12083587\|ref\|NP 073145.1\| (NM_022654) | p53 protein induced, with death domain [Mus musculus] | 915 | 47/146 (32%) | 87/146 (59%) | 2e-18 |

Leucine-rich repeats (LRRs) are relatively short motifs (22-28 residues in length) found in a variety of cytoplasmic, membrane and extracellular proteins. Although these proteins are associated with widely different functions, a common property involves protein-protein interaction. Little is known about the 3D structure of LRRs, although it is believed that they can. form amphipathic structures with hydrophobic surfaces capable of interacting with membranes. In vitro studies of a synthetic LRR from Drosophila Toll protein have indicated that the peptides form gels by adopting beta-sheet structures that form extended filaments. These results are consistent with the idea that LRRs mediate protein-protein interactions and cellular adhesion. Other functions of LRR-containing proteins include, for example, binding to enzymes and vascular repair. The 3-D structure of ribonuclease inhibitor, a protein containing 15 LRRs, has been determined, revealing LRRs to be a new class of alpha/beta fold. LRRs form elongated non-globular structures and are often flanked by cysteine rich domains.

The disclosed NOV44 nucleic acid of the invention encoding a leucine-rich repeat-like protein includes the nucleic acid whose sequence is provided in Table 44A or a fragment thereof. The invention also includes a mutant or variant nucleic acid any of whose bases may be changed from the corresponding base shown in Table 44A while still encoding a protein that maintains its leucine-rich repeat-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 41 percent of the bases may be so changed.

The disclosed NOV44 protein of the invention includes the leucine-rich repeat-like protein whose sequence is provided in Table 44B. The invention also includes a mutant or variant protein any of whose residues may be changed from the corresponding residue shown in Table B while still encoding a protein that maintains its leucine-rich repeat-like activities and physiological functions, or a functional fragment thereof. In the mutant or variant protein, up to about 18 percent of the residues may be so changed.

The invention further encompasses antibodies and antibody fragments, such as F_{ab} or (F_{ab})₂, that bind immunospecifically to any of the proteins of the invention.

The above defined information for this invention suggests that this leucine-rich repeat-like protein (NOV44) may function as a member of a "leucine-rich repeat family". Therefore, the NOV44 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV44 nucleic acids and proteins of the invention are useful in potential therapeutic applications implicated in diseases including but not limited to various pathologies and disorders as indicated below. For example, a cDNA encoding the leucine-rich repeat-like protein (NOV44) may be useful in gene therapy, and the leucine-rich repeat-like protein (NOV44) may be useful when administered to a subject in need thereof. By way of nonlimiting example, the compositions of the present invention will have efficacy for treatment of patients suffering from fertility, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neuroprotection; or other pathologies or conditions. The NOV44 nucleic acid encoding the leucine-rich repeat-like protein of the invention, or fragments thereof, may further be useful in diagnostic applications, wherein the presence or amount of the nucleic acid or the protein are to be assessed.

NOV44 nucleic acids and polypeptides are further useful in the generation of antibodies that bind immuno-specifically to the novel NOV44 substances for use in therapeutic or diagnostic methods. These antibodies may be generated according to methods known in the art, using prediction from hydrophobicity charts, as described in the "Anti-NOVX Antibodies" section below. The disclosed NOV44 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV45

NOV45 includes two Ig/fibronectin-like proteins disclosed below. The disclosed sequences have been named NOV45a and NOV45b.

### NOV45a

A disclosed NOV45a nucleic acid of 4321 nucleotides (also referred to as CG57656-01) encoding a Ig/fibronectin -like protein is shown in Table 45A. The start and stop codons are in bold letters.

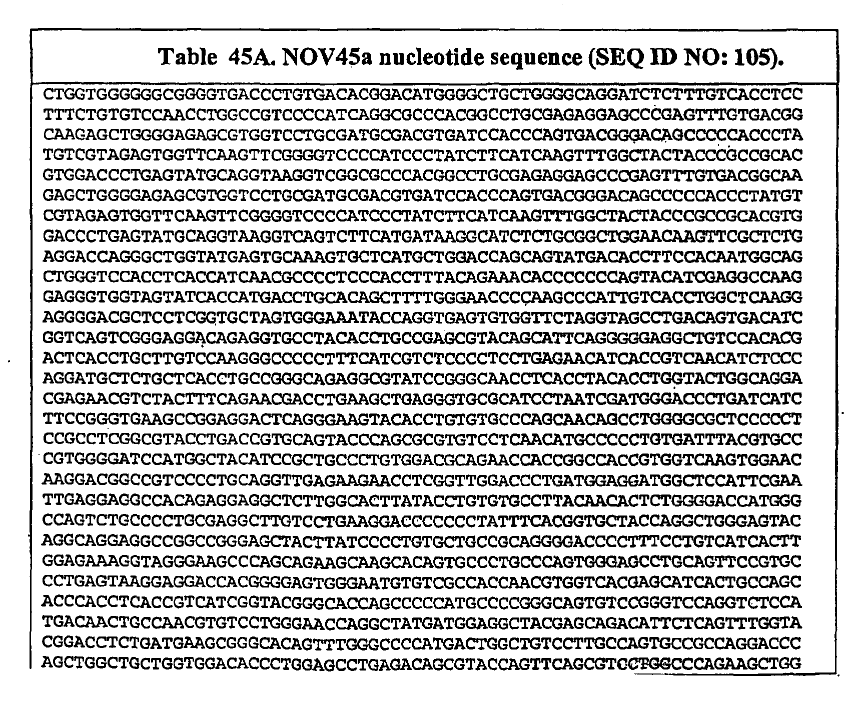

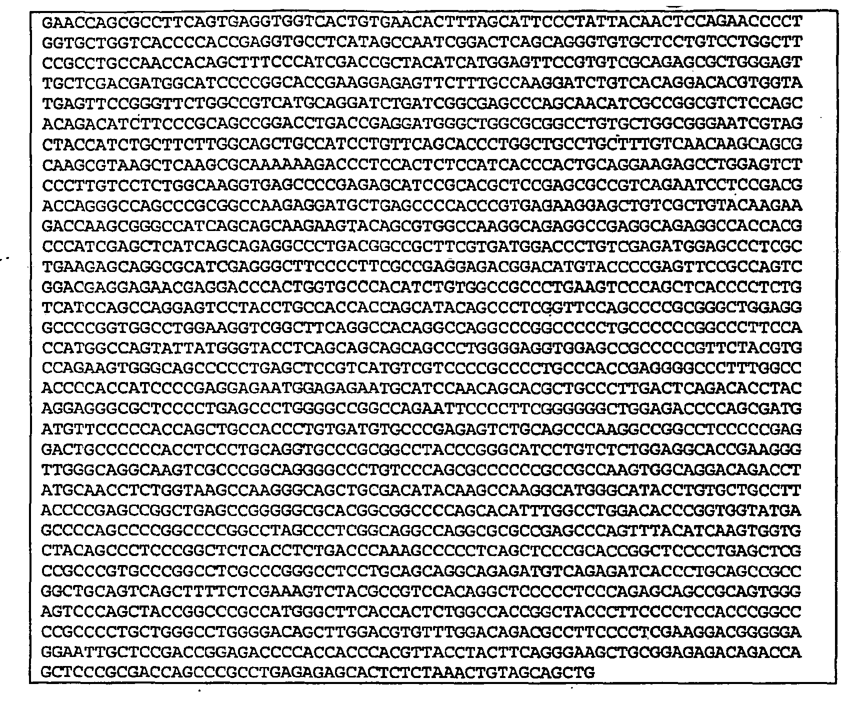

In a search of public sequence databases, the NOV45a nucleic acid sequence, located on chromsome 11 has 2296 of 2298 bases (99%) identical to a gb:GENBANK-ID:AB028953|acc:AB028953.1 mRNA from Homo sapiens (Homo sapiens mRNA for KIAA1030 protein, partial cds). Public nucleotide databases include all GenBank databases and the GeneSeq patent database.

The disclosed NOV45a polypeptide (SEQ ID NO: 106) encoded by SEQ ID NO:105 has 1328 amino acid residues and is presented in Table 45B using the one-letter amino acid code. Signal P, Psort and/or Hydropathy results predict that NOV45a has a signal peptide and is likely to be localized at the plasma membrane with a certainty of 0.4600. The most likely cleavage site for a NOV45a peptide is between amino acids 19 and 20.

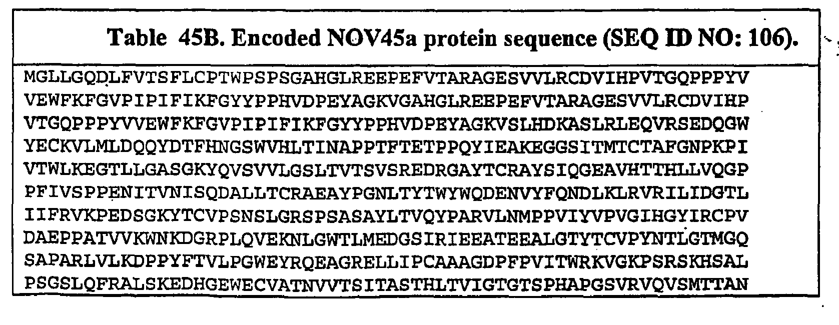

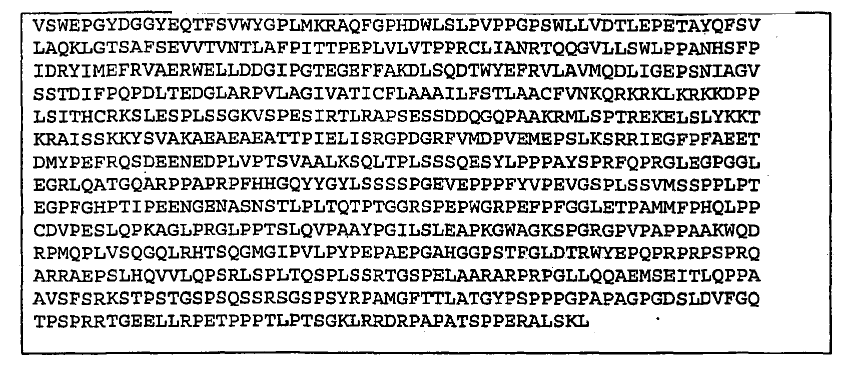

A search of sequence databases reveals that the NOV45a amino acid sequence has 761 of 763 amino acid residues (99%) identical to, and 761 of 763 amino acid residues (99%) similar to, the 763 amino acid residue ptnr:SPTREMBL-ACC:Q9UPX0 protein from Homo sapiens (Human) (KIAA1030 PROTEIN). Public amino acid databases include the GenBank databases, SwissProt, PDB and PIR.

NOV45a is expressed in at least brain, cerebral medulla/cerebral white matter, prostate, thalamus, placenta. Expression information was derived from the tissue sources of the sequences that were included in the derivation of the sequence of CG57656-01.The sequence is predicted to be expressed in the following tissues because of the expression pattern of (GENBANK-ID: gb:GENBANK-ID:AB028953|acc:AB028953.1) a closely related Homo sapiens mRNA for KIAA1030 protein, partial cds homolog in species Homo sapiens :liver. This information was derived by determining the tissue sources of the sequences that were included in the invention including but not limited to SeqCalling sources, Public EST sources, Literature sources, and/or RACE sources.

### NOV45b

A disclosed NOV45b nucleic acid of 7097 nucleotides (also referred to as CG57656-02) encoding a Ig/fibronectin -like protein is shown in Table 45C. The start and stop codons are in bold letters.

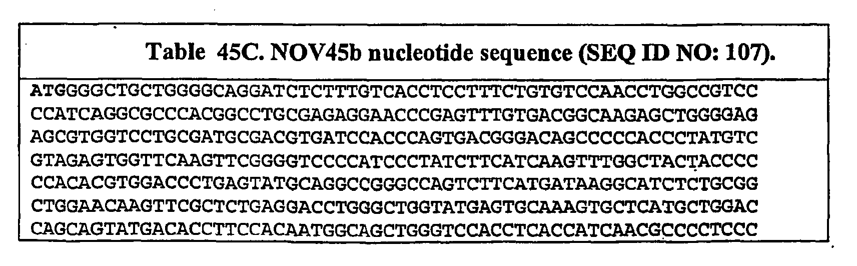

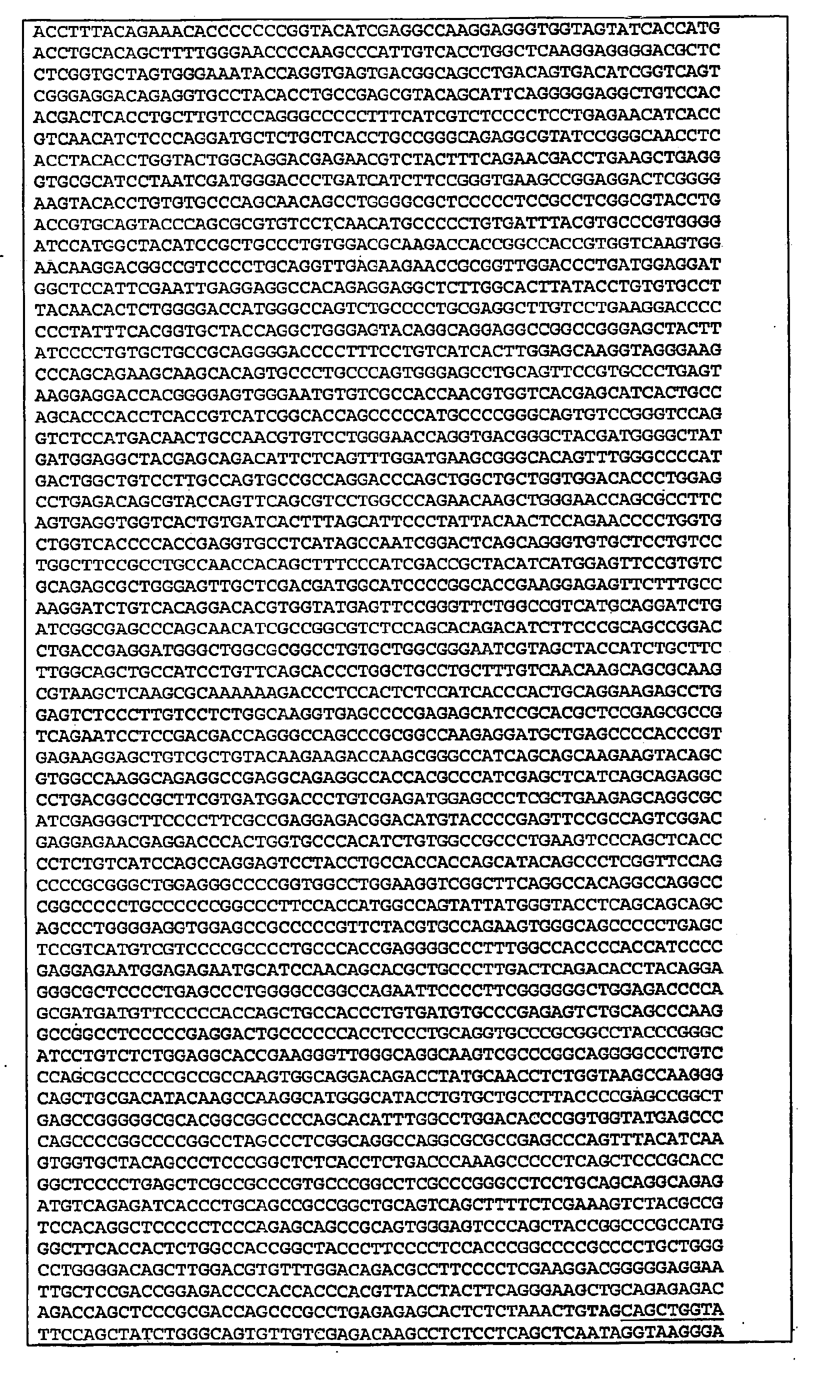

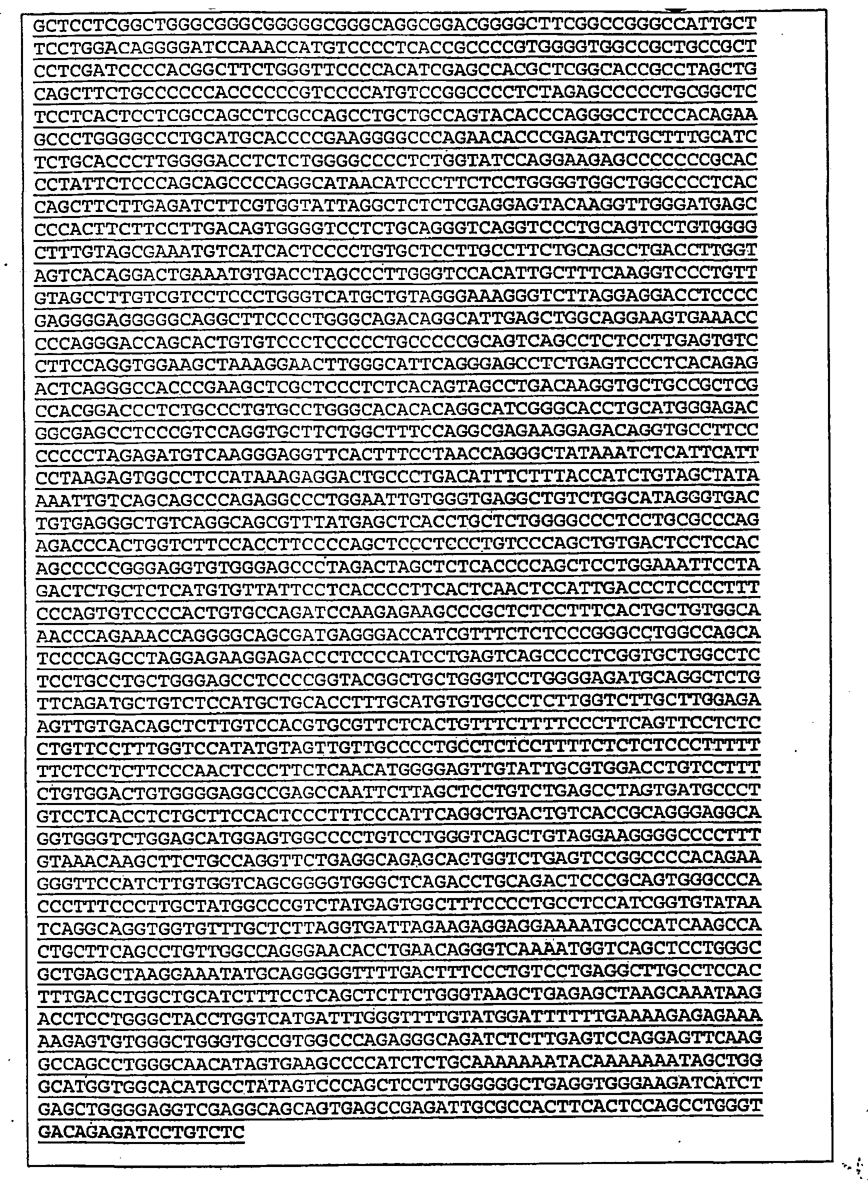

In a search of public sequence databases, the NOV45b nucleic acid sequence, located on chromsome 11 has 5312 of 5319 bases (99%) identical to a gb:GENBANK-ID:AB028953|acc:AB028953.1 mRNA from Homo sapiens (Homo sapiens mRNA for KIAA1030 protein, partial cds). Public nucleotide databases include all GenBank databases and the GeneSeq patent database.

The disclosed NOV45b polypeptide (SEQ ID NO: 108) encoded by SEQ ID NO: 107 has 1356 amino acid residues and is presented in Table 45D using the one-letter amino acid code. Signal P, Psort and/or Hydropathy results predict that NOV 45b has a signal peptide and is likely to be localized at the plasma membrane with a certainty of 0.4600. The most likely cleavage site for a NOV45b peptide is between amino acids 19 and 20.

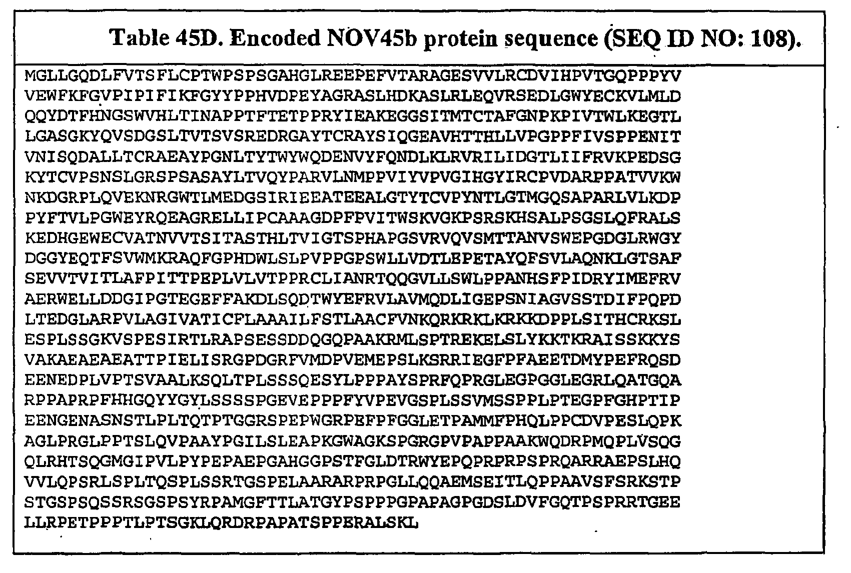

A search of sequence databases reveals that the NOV45b amino acid sequence has 425 of 911 amino acid residues (46%) identical to, and 553 of 911 amino acid residues (60%) similar to, the 1189 amino acid residue ptnr:SPTREMBL-ACC:Q9P2J2 protein from Homo sapiens (Human) (KIAA1355 PROTEIN). Public amino acid databases include the GenBank databases, SwissProt, PDB and PIR.

NOV45b is expressed in at least prostate, brain (cerebellum). This information was derived by determining the tissue sources of the sequences that were included in the invention including but not limited to SeqCalling sources, Public EST sources, Literature sources, and/or. RACE sources.

The disclosed NOV45a polypeptide has homology to the amino acid sequences shown-in the BLASTP data listed in Table 45E.

| **Table 45E. BLAST results for NOV45** | | | | | |
|---|---|---|---|---|---|
| Gene Index/ Identifier | protein/ Organism | Length (aa) | Identity (%) | Positives (%) | Expect |
| gi\|5689397\|dbj\|BAA8 2982.11 (AB028953) | KIAA1030 protein [Homo sapiens] | 763 | 761/763 (99%) | 761/763 (99%) | 0.0 |
| gi\|15311046\|ref\|XP 027486.2\| (XM_027486) | KIAA1030 protein [Homo sapiens] | 747 | 583/627 (92%) | 583/627 (92%), | 0.0 |
| gi\|18578690\|ref\|XP 062186.2 (XM_062186) | similar to KIAA1355 protein [Homo sapiens] | 904 | 413/472 (87%) | 417/472 (87%) | 0.0 |
| gi\|7243091\|dbj\|BAA9 2593.1\| (AB037776) | KIAA1355 protein [Homo sapiens] | 1189 | 410/882 (46%) | 527/882 (59%) | e-179 |
| gi\|18426807\|ref\|NP 291086.1\| (NM_033608) | neural cell adhesion molecule (Ncam)-like; KIAA1355 hypothetical protein (human); NCAM-like protein NRT1 [Mus musculus] | 1179 | 409/866 (47%) | 525/866 (60%) | e-177 |

Tables 45F-H list the domain descriptions from DOMAIN analysis results against NOV . This indicates that the NOV sequence has properties similar to those of other proteins known to contain this domain.

| **Table 45F. Domain Analysis of NOV45** |
|---|
| gnl \| Smart \| smart00409, IG, Immunoglobulin |
| CD-Length = 86 residues, 100.0% aligned |
| Score = 62.4 bits (150), Expect = 2e-10 |

| **Table 45G. Domain Analysis of NOV45** | | |
|---|---|---|
| qnl\|Smart\|smart00408, IGc2, Immunoglobulin C-2 Type | | |
| | CD-Length = 63 residues, 92.1% aligned | |
| | | Score = 54.7 bits (130), Expect = 4e-08 |

| **Table 45H. Domain Analysis of NOV45** |
|---|
| gnl\|pfam\|pfam00041, fn3, Fibronectin type III domain. |
| CD-Length = 86 residues, 90.7% aligned |
| Score = 43.5 bits (101), Expect = 8e-05 |

The basic structure of immunoglobulin (Ig) molecules is a tetramer of two light chains and two heavy chains linked by disulfide bonds. There are two types of light chains: kappa and lambda, each composed of a constant domain (CL) and a variable domain (VL). There are five types of heavy chains: alpha, delta, epsilon, gamma and mu, all consisting of a variable domain (VH) and three (in alpha, delta and gamma) or four (in epsilon and mu) constant domains (CH1 to CH4). The major histocompatibility complex (MHC) molecules are made of two chains. In class I the alpha chain is composed of three extracellular domains, a transmembrane region and a cytoplasmic tail. The beta chain (beta-2- microglobulin) is composed of a single extracellular domain. In class II, both the alpha and the beta chains are composed of two extracellular domains, a transmembrane region and a cytoplasmic tail. It is known that the Ig constant chain domains and a single extracellular domain in each type of MHC chains are related. These homologous domains are approximately one hundred amino acids long and include a conserved intradomain disulfide bond. Members of the immunoglobulin superfamily are found in hundreds of proteins of different functions. Examples include antibodies, the giant muscle kinase titin and receptor tyrosine kinases. Immunoglobulin-like domains may be involved in protein-protein and protein-ligand interactions.

Fibronectins are multi-domain glycoproteins found in a soluble form in plasma, and in an insoluble form in loose connective tissue and basement membranes. They contain multiple copies of 3 repeat regions (types I, II and III), which bind to a variety of substances including heparin, collagen, DNA, actin, fibrin and fibronectin receptors on cell surfaces. The wide variety of these substances means that fibronectins are involved in a number of important functions: e.g., wound healing; cell adhesion; blood coagulation; cell differentiation and migration; maintenance of the cellular cytoskeleton; and tumour metastasis. The role of fibronectin in-cell differentiation is demonstrated by the marked reduction in the expression of its gene when neoplastic transformation occurs. Cell attachment has been found to be mediated by the binding of the tetrapeptide RGDS to integrins on the cell surface, although related sequences can also display cell adhesion activity. Plasma fibronectin occurs as a dimer of 2 different subunits, linked together by 2 disulphide bonds near the C-terminus. The difference in the 2 chains occurs in the type III repeat region and is caused by alternative splicing of the mRNA from one gene. The observation that, in a given protein, an individual repeat of one of the 3 types (e.g., the first FnIII repeat) shows much less similarity to its subsequent tandem repeats within that protein than to its equivalent repeat between fibronectins from other species, has suggested that the repeating structure of fibronectin arose at an early stage of evolution. It also seems to suggest that the structure is subject to high selective pressure. The fibronectin type III repeat region is an approximately 100 amino acid domain, different tandem repeats of which contain binding sites for DNA, heparin and the cell surface. The superfamily of sequences believed to contain FnIII repeats represents 45 different families, the majority of which are involved in cell surface binding in some manner, or are receptor protein tyrosine kinases, or cytokine receptors.

The disclosed NOV45 nucleic acid of the invention encoding a Ig/fibronectin domain-like protein includes the nucleic acid whose sequence is provided in Table 45A or 45C or a fragment thereof. The invention also includes a mutant or variant nucleic acid any of whose bases may be changed from the corresponding base shown in Table 45A or 45C while still encoding a protein that maintains its Ig/fibronectin domain-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1 percent of the bases may be so changed.

The disclosed NOV45 protein of the invention includes the Ig/fibronectin domain-like protein whose sequence is provided in Table 45B or 45D. The invention also includes a mutant or variant protein any of whose residues may be changed from the corresponding residue shown in Table 45B or 45D while still encoding a protein that maintains its Ig/fibronectin domain-like activities and physiological functions, or a functional fragment thereof. In the mutant or variant protein, up to about 54 percent of the residues may be so changed.

The invention further encompasses antibodies and antibody fragments, such as F_{ab} or (F_{ab})_{2,} that bind immunospecifically to any of the proteins of the invention.

The above defined information for this invention suggests that this Ig/fibronectin domain-like protein (NOV45) may function as a member of a "Ig/fibronectin domain family". Therefore, the NOV45 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV45 nucleic acids and proteins of the invention are useful in potential therapeutic applications implicated in diseases including but not limited to various pathologies and disorders as indicated below. For example, a cDNA encoding the Ig/fibronectin domain-like protein (NOV45) may be useful in gene therapy, and the Ig/fibronectin domain-like protein (NOV45) may be useful when administered to a subject in need thereof. By way of nonlimiting example, the compositions of the present invention will have efficacy for treatment of patients suffering from Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neuroprotection, fertility, or other pathologies or conditions. The NOV45 nucleic acid encoding the Ig/fibronectin domain-like protein of the invention, or fragments thereof, may further be useful in diagnostic applications, wherein the presence or amount of the nucleic acid or the protein are to be assessed.

NOV45 nucleic acids and polypeptides are further useful in the generation of antibodies that bind immuno-specifically to the novel NOV45 substances for use in therapeutic or diagnostic methods. These antibodies may be generated according to methods known in the art, using prediction from hydrophobicity charts, as described in the "Anti-NOVX Antibodies" section below. The disclosed NOV45 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV46

A disclosed NOV46 nucleic acid of 1247 nucleotides (also referred to as CG57682-01) encoding a G2/mitotic-specific cyclin B2-like protein is shown in Table 46A. The start and stop codons are in bold letters.

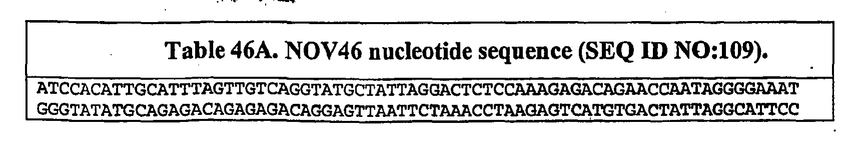

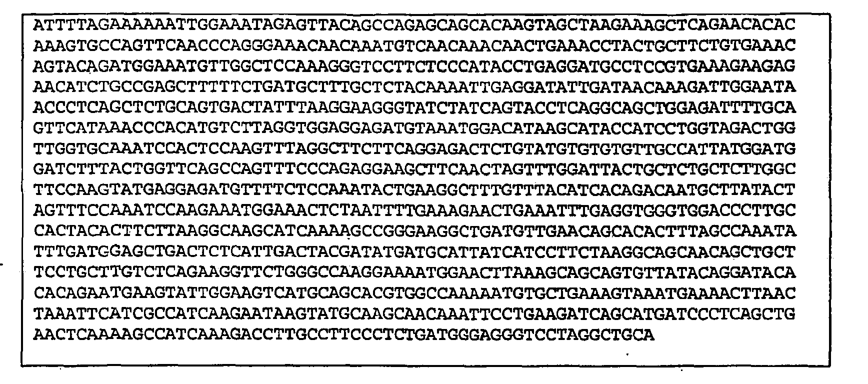

In a search of public sequence databases, the NOV46 nucleic acid sequence, located on chromsome 7 has 1068 of 1172 bases (91 %) identical to a gb:GENBANK-ID:HSM800659|acc:AL080146.1 mRNA from Homo sapiens (Homo sapiens mRNA; cDNA DKFZp434B174 (from clone DKFZp434B174); complete cds). Public nucleotide databases include all GenBank databases and the GeneSeq patent database.

The disclosed NOV46 polypeptide (SEQ ID NO:110) encoded by SEQ ID NO: 109 has 404 amino acid residues and is presented in Table 46B using the one-letter amino acid code. Signal P, Psort and/or Hydropathy results predict that NOV46 has a signal peptide and is likely to be localized in the cytoplasm with a certainty of 0.6500.

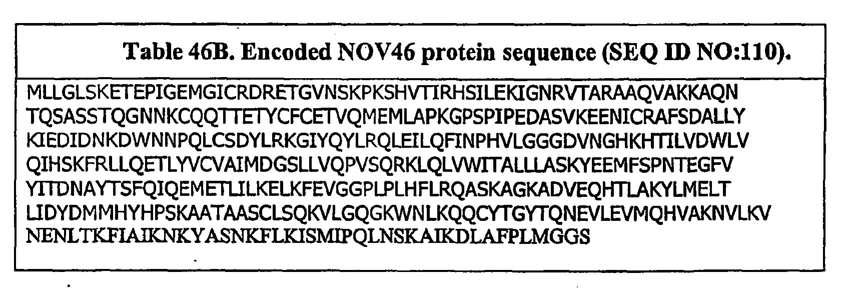

A search of sequence databases reveals that the NOV46 amino acid sequence has 303 of 383 amino acid residues (79%) identical to, and 333 of 383 amino acid residues (86%) similar to, the 398 amino acid residue ptnr:SWISSNEW-ACC:O95067 protein from Homo sapiens (Human) (G2/MITOTIC-SPECIFIC CYCLIN B2). Public amino acid databases include the GenBank databases, SwissProt, PDB and PIR.

NOV46 is expressed in at least Adrenal gland, Aorta, B-cells, Blood, Bone, Brain, Breast, CNS, Colon, Ear, Esophagus, Eye, Gall bladder, Germ Cell, Head and neck, Heart, Kidney, Larynx, Liver, Lung, Lymph, Marrow, Muscle, Neural, Omentum, Ovary, Pancreas, Parathyroid, Peripheral nervous system, Placenta, Pooled, Prostate, Skin, Small intestine, Spleen, Stomach, Synovial membrane, Testis, Tissue culture, Tonsil, Uterus, Whole embryo, and adrenal gland. This information was derived by determining the tissue sources of the sequences that were included in the invention including but not limited to SeqCalling sources, Public EST sources, Literature sources, and/or RACE sources.

The disclosed NOV46 polypeptide has homology to the amino acid sequences shown in the BLASTP data listed in Table 46C.

| **Table 46C. BLAST results for NOV46** | | | | | |
|---|---|---|---|---|---|
| Gene Index/ Identifier | Protein/ Organism | Length (aa) | Identity (%) | Positives (%) | Expect |
| gi\|4757930\|ref\|NP 0 04692.1 (NM_004701) | cyclin B2 [Homo sapiens] | 398 | 303/387 (78%) | 333/387 (85%) | e-67 |
| gi\|5921730\| sp\|07768 9\|CGB2 BOVIN | G2/mitotic-specific cyclin B2 | 398 | 285/385 (74%) | 328/385 (85%) | e-62 |
| gi\|584914\|sp\| P37883 CGB2 MESAU | G2/mitotic-specific cyclin B2 | 397 | 288/385 (74%) | 321/385 (82%) | e-58 |
| gi\|14198371\|gb\|AAH0 8247.1\|AAH08247 (BC008247) | Similar to cyclin B2 [Mus musculus] | 398 | 278/382 (72%) | 316/382 (81%) | e-51 |
| gi\|6680866\|ref\|NP 0 31656.1\| (NM 007630) | cyclin B2 [Mus musculus] | 398 | 274/382 (71%) | 313/382 (81%) | e-49 |

Tablez 46D-E list the domain descriptions from DOMAIN analysis results against NOV46. This indicates that the NOV46 sequence has properties similar to those of other proteins known to contain this domain.

| **Table 46D. Domain Analysis of NOV46** | | |
|---|---|---|
| gn1\|Pfam\|pfam00134, cyclin, Cyclin, N-terminal domain. Cyclins regulate cyclin dependent kinases (CDKs). Cyclins contain two domains of similar all-alpha fold, of which this family corresponds with the N-terminal domain. | | |
| | CD-Length = 128 residues, 99.2% aligned | |
| | | Score = 119 bits (298), Expect = 3e-28 |

| **Table 46E. Domain Analysis of NOV46** | | |
|---|---|---|
| gnl\|Smart\|smart00385, CYCLIN, domain present in cyclins, TFIIB and Retinoblastoma; A helical domain present in cyclins and TFIIB (twice) and Retinoblastoma (once). A protein recognition domain functioning in cell-cycle and transcription control. | | |
| | CD-Length = 83 residues, 100.0% aligned | |
| | | Score = 62.8 bits (151), Expect = 4e-11 |

Two B-type cyclins, B1 and B2, have been identified in mammals. Proliferating cells express both cyclins, which bind to and activate p34(cdc2). To test whether the two B-type cyclins have distinct roles, lines of transgenic mice were generated, one lacking cyclin B1 and the other lacking cyclin B2. Cyclin B1 proved to be an essential gene; no homozygous B1-null pups were born. In contrast, nullizygous B2 mice developed normally and did not display any obvious abnormalities. Both male and female cyclin B2-null mice were fertile, which was unexpected in view of the high levels and distinct patterns of expression of cyclin B2 during spermatogenesis. The expression of cyclin B1 overlaps the expression of cyclin B2 in the mature testis, but not vice versa. Cyclin B1 can be found both on intracellular membranes and free in the cytoplasm, in contrast to cyclin B2, which is membrane-associated. These observations suggest that cyclin B1 may compensate for the loss of cyclin B2 in the mutant mice, and implies that cyclin B1 is capable of targeting the p34(cdc2) kinase to the essential substrates of cyclin B2.

The disclosed NOV46 nucleic acid of the invention encoding a G2/mitotic-specific cyclin B2-like protein includes the nucleic acid whose sequence is provided in Table 46A or a fragment thereof. The invention also includes a mutant or variant nucleic acid any of whose bases may be changed from the corresponding base shown in Table 46A while still encoding a protein that maintains its G2/mitotic-specific cyclin B2-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 9 percent of the bases may be so changed.

The disclosed NOV46 protein of the invention includes the G2/mitotic-specific cyclin B2-like protein whose sequence is provided in Table 46B. The invention also includes a mutant or variant protein any of whose residues may be changed from the corresponding residue shown in Table 46B while still encoding a protein that maintains its G2/mitotic-specific cyclin B2-like activities and physiological functions, or a functional fragment thereof. In the mutant or variant protein, up to about 21 percent of the residues may be so changed.

The invention further encompasses antibodies and antibody fragments, such as F_{ab} or (F_{ab})₂, that bind immunospecifically to any of the proteins of the invention.

The above defined information for this invention suggests that this G2/mitotic-specific cyclin B2-like protein (NOV46) may function as a member of a "G2/mitotic-specific cyclin B2 family". Therefore, the NOV46 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV46 nucleic acids and proteins of the invention are useful in potential therapeutic applications implicated in diseases including but not limited to various pathologies and disorders as indicated below. For example, a cDNA encoding the G2/mitotic-specific cyclin B2-like protein (NOV46) may be useful in gene therapy, and the G2/mitotic-specific cyclin B2-like protein (NOV46) may be useful when administered to a subject in need thereof. By way of nonlimiting example, the compositions of the present invention will have efficacy for treatment of patients suffering from Cardiomyopathy, Atherosclerosis,Hypertension, Congenital heart defects, Aortic stenosis ,Atrial septal defect (ASD),Atrioventricular (A-V) canal defect, Ductus arteriosus , Pulmonary stenosis , Subaortic stenosis, Ventricular septal defect (VSD), valve diseases,Tuberous sclerosis, Scleroderma, Obesity,Transplantation, Adrenoleukodystrophy, Congenital Adrenal Hyperplasia, Hemophilia, Hypercoagulation,Idiopathic thrombocytopenic purpura , Immunodeficiencies,Graft vesus host, Von Hippel-Lindau (VHL) syndrome, Alzheimer's disease, Stroke, Tuberous sclerosis, hypercalceimia, Parkinson's disease, Huntington's disease, Cerebral palsy, Epilepsy,Lesch-Nyhan syndrome, Multiple sclerosis,Ataxia-telangiectasia,Leukodystrophies,Behavioral disorders, Addiction, Anxiety, Pain, Neuroprotection, or other pathologies or conditions. The NOV46 nucleic acid encoding the G2/mitotic-specific cyclin B2-like protein of the invention, or fragments thereof, may further be useful in diagnostic applications, wherein the presence or amount of the nucleic acid or the protein are to be assessed.

NOV46 nucleic acids and polypeptides are further useful in the generation of antibodies that bind immuno-specifically to the novel NOV46 substances for use in therapeutic or diagnostic methods. These antibodies may be generated according to methods known in the art, using prediction from hydrophobicity charts, as described in the "Anti-NOVX Antibodies" section below. The disclosed NOV46 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV47

A disclosed NOV47 nucleic acid of 15645 nucleotides (also referred to as CG57764-01) encoding a ALR-like protein is shown in Table 47A. The start and stop codons are in bold letters.

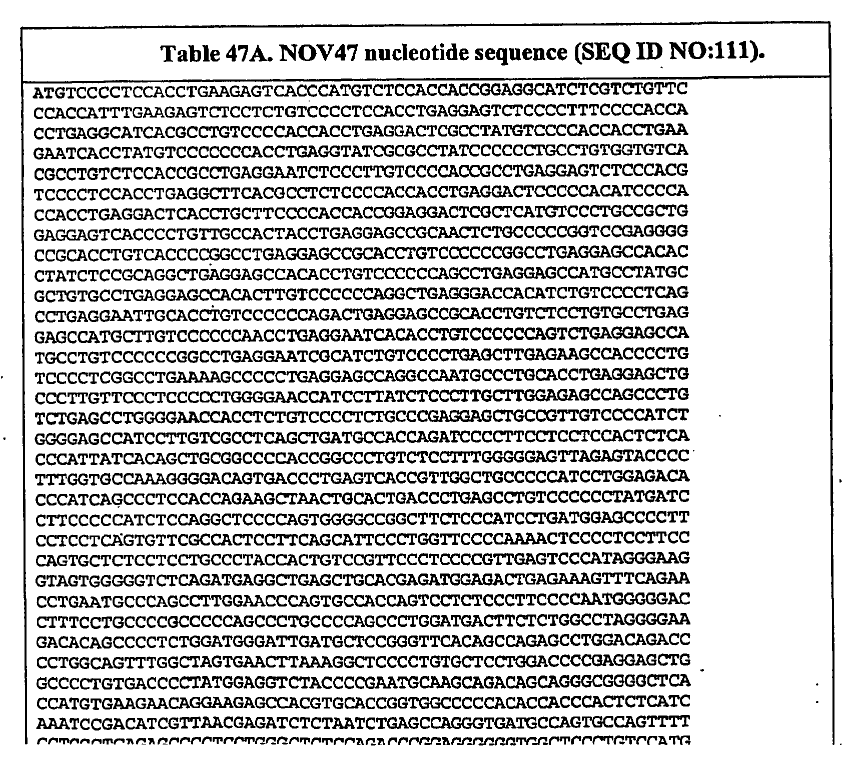

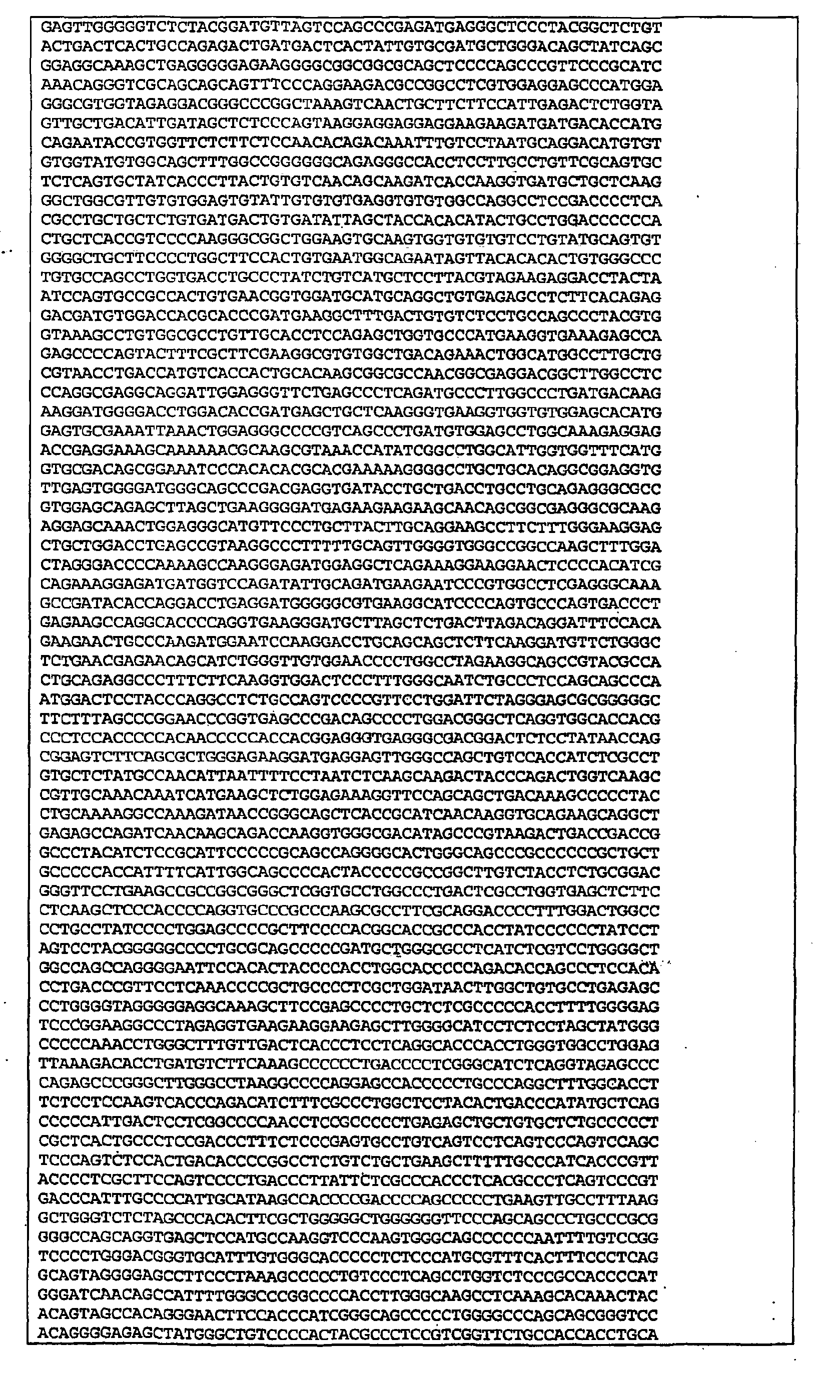

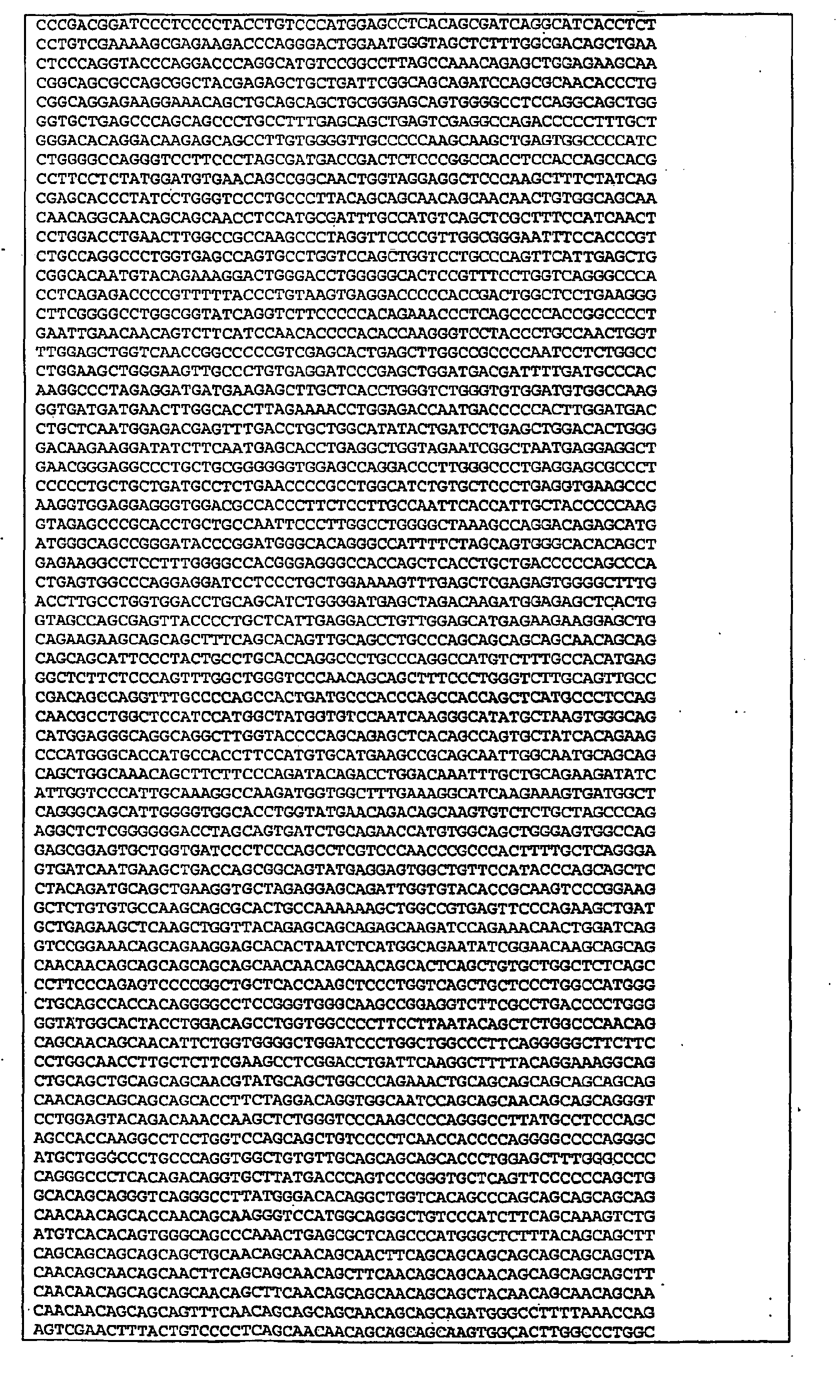

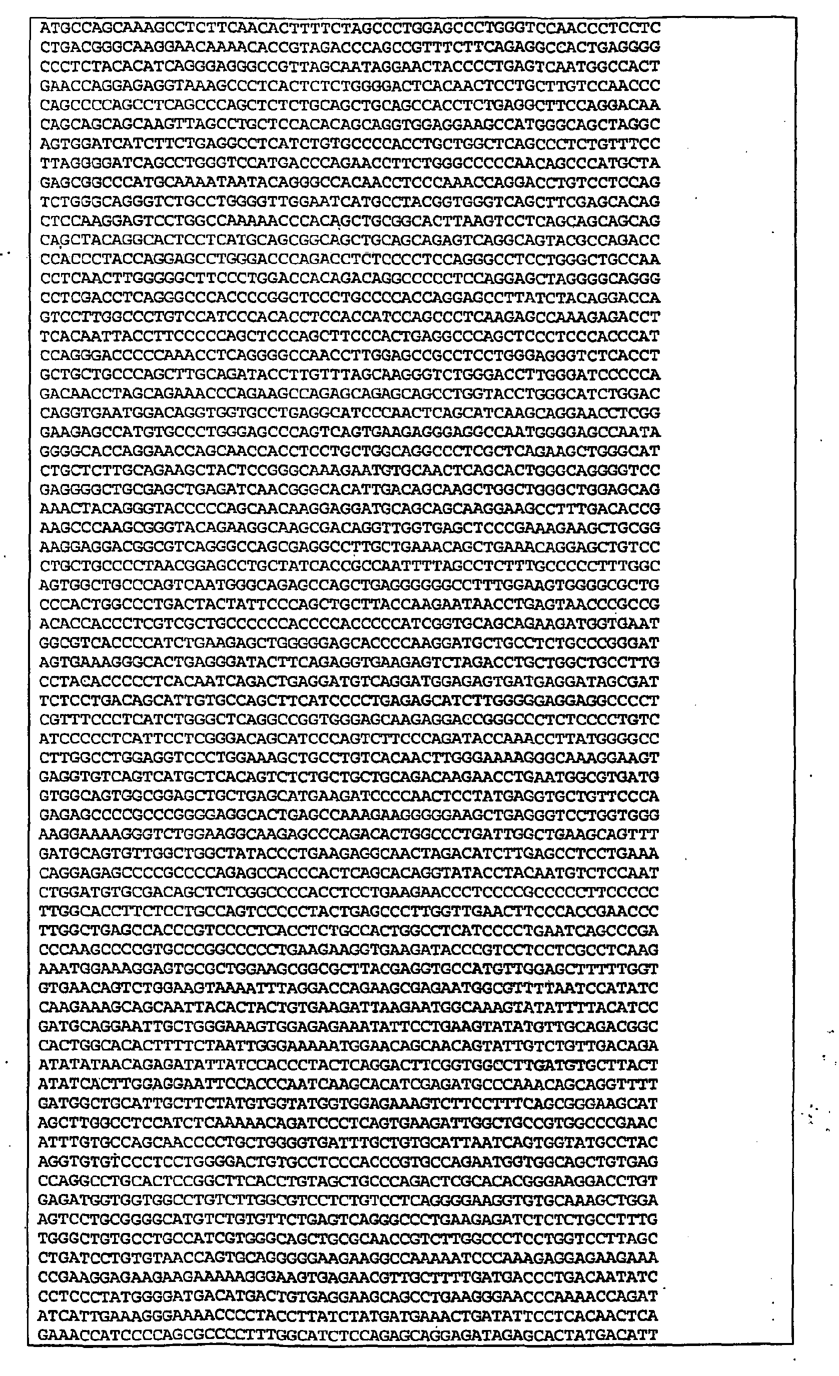

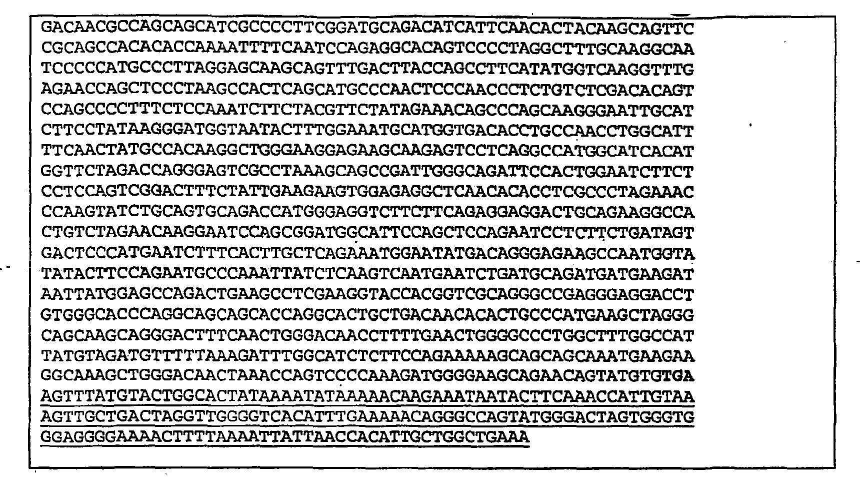

In a search of public sequence databases, the NOV47 nucleic acid sequence, located on chromsome 12q12.14 has 14307 of 14312 bases (99%) identical to a gb:GENBANK-ID:AF010404|cc:AF010404.1 mRNA from Homo sapiens (Homo sapiens ALR mRNA, complete cds). Public nucleotide databases include all GenBank databases and the GeneSeq patent database.

The disclosed NOV47 polypeptide (SEQ ID NO:112) encoded by SEQ ID NO:111 has 5159 amino acid residues and is presented in Table 47B using the one-letter amino acid code. Signal P, Psort and/or Hydropathy results predict that NOV47 has no signal peptide and is likely to be localized in the cytoplasm with a certainty of 0.9800.

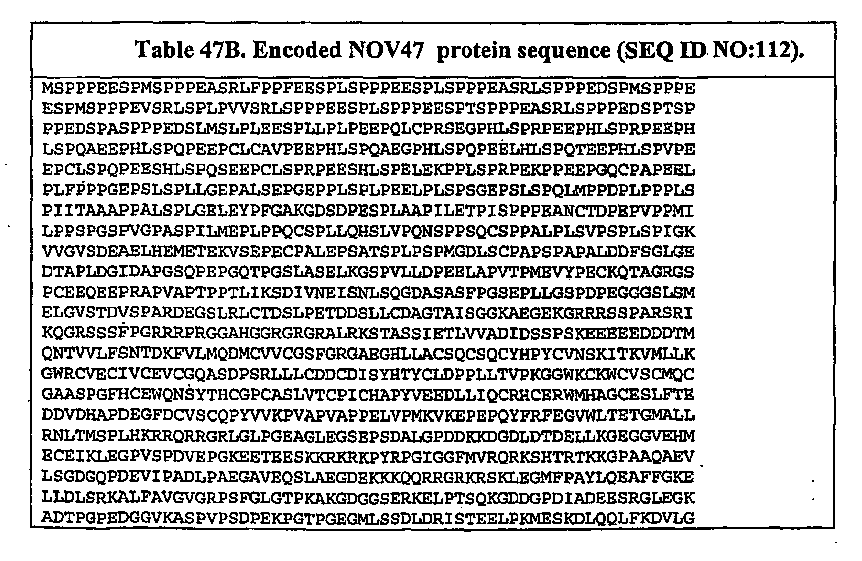

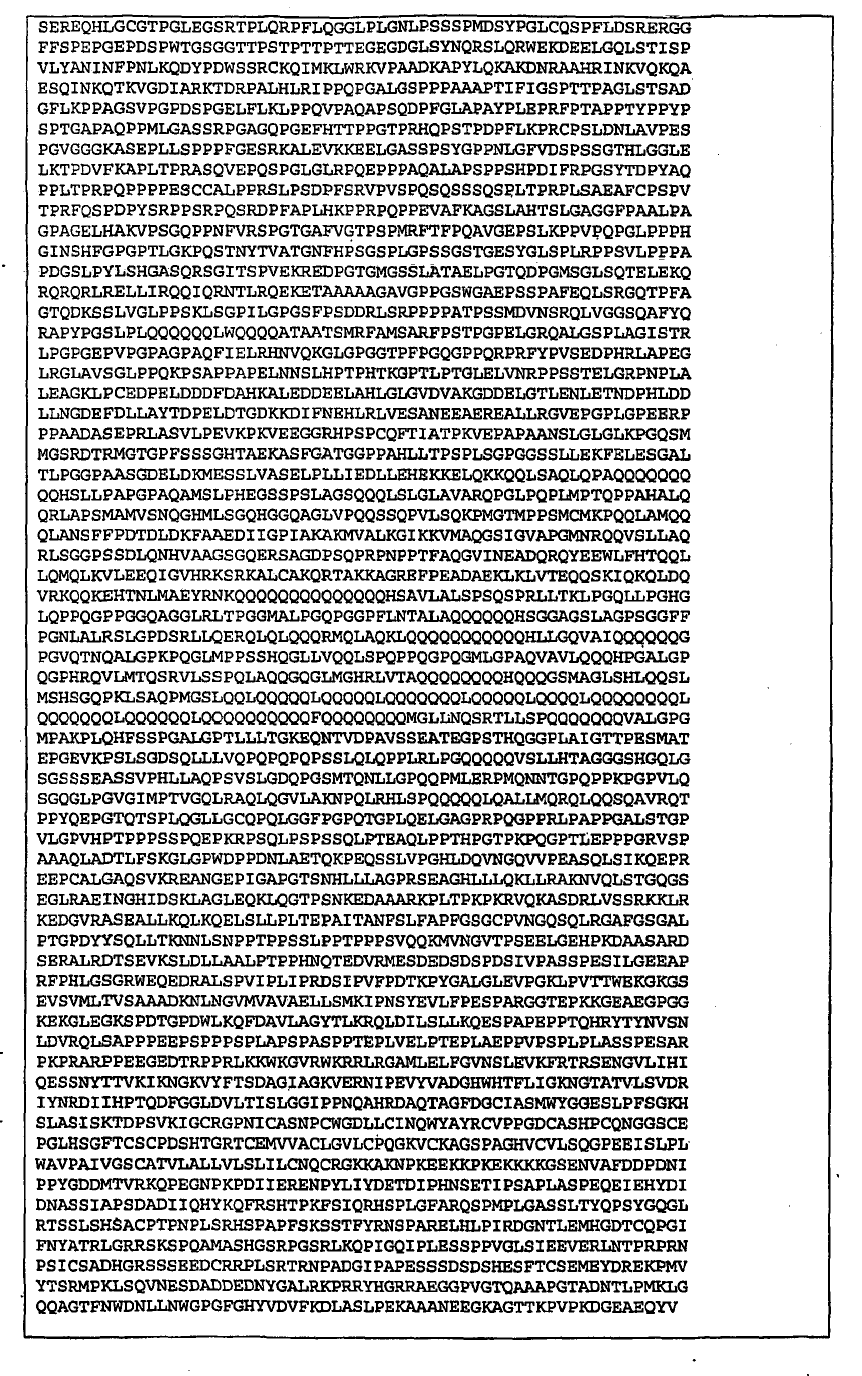

A search of sequence databases reveals that the NOV47 amino acid sequence has 4776 of 4796 amino acid residues (99%) identical to, and 4779 of 4796 amino acid residues (99%) similar to, the 4957 amino acid residue ptnr:SPTREMBL-ACC:014687 protein from Homo sapiens (Human) (ALR). Public amino acid databases include the GenBank databases, SwissProt, PDB and PIR.

NOV47 is expressed in at least brain. This information was derived by determining the tissue sources of the sequences that were included in the invention including but not limited to SeqCalling sources, Public EST sources, Literature sources, and/or RACE sources.

The disclosed NOV47 polypeptide has homology to the amino acid sequences shown in the BLASTP data listed in Table 47C.

| **Table 47C. BLAST results for NOV47** | | | | | |
|---|---|---|---|---|---|
| Gene Index/ Identifier | Protein/ Organism | Length (aa) | Identity (%) | Positives (%) | Expect |
| gi\|7512280\|pir\|T03 455 | ALR protein - human | 4957 | 4384/4427 (99%) | 4385/4427 (99%) | 0.0 |
| gi\|18601907\|ref\|XP 028760.2\| (XM_028760) | myeloid/lymphoid or mixed-lineage leukemia 2 [Homo sapiens] | 3492 | 2930/2944 (99%) | 2933/2944 (99%) | 0.0 |
| gi\|3540281\|gb\|AAC34 383.1\| (AF056116) | All-1 related protein [Takifugu rubripes] | 4823 | 648/1458 (44%) | 822/1458 (55%) | 0.0 |
| gi\|13640139\|ref\|XP 017017.1\| (XM_017017) | protein FLJ23056 [Homo sapiens] | 317 | 315/317 (99%) | 315/317 (99%) | e-149 |
| gi\|10864041\|ref\|NP 067053.11 (NM_021230) | myeloid/lymphoid or mixed-lineage leukemia 3; ALR-like protein [Homo sapiens] | 4025 | 340/802 (42%) | 467/802 (57%) | e-135 |

Tables 47D-H list the domain descriptions from DOMAIN analysis results against NOV47. This indicates that the NOV47 sequence has properties similar to those of other proteins known to contain this domain.

| **Table 47D. Domain Analysis of NOV47** | | |
|---|---|---|
| gnl \|Smart \|smart00282, LamG, Laminin G domain | | |
| | CD-Length = 135 residues, 98.5% aligned | |
| | | Score 85.1 bits (209), Expect = 9e-17 |

| **Table 47E. Domain Analysis of NOV47** | | |
|---|---|---|
| gnl\|Pfam\|pfam00628, PHD, PHD-finger. PHD folds into an interleaved type of Zn-finger chelating 2 Zn ions in a similar manner to that of the RING and FYVE domains. | | |
| | CD-Length = 49 residues, 98.0% aligned | |
| | | Score = 71.2 bits (173), Expect = 1e-12 |

| **Table 47F. Domain Analysis of NOV47** |
|---|
| gnl\|pfam\|pfam00769, ERM, Ezrin/radixin/moesin family. This family of proteins contain a band 4.1 domain (pfam00373), at their amino terminus. This family represents the rest of these proteins. |
| CD-Length = 365 residues, 23.0% aligned |
| * Score = 52.8 bits (125), Expect = 5e-07 |

| **Table 47G. Domain Analysis of NOV47** |
|---|
| gnl\|Pfam\|pfam00529, HlyD, HlyD family secretion protein. |
| CD-Length = 310 residues, 53.9% aligned |
| Score = 53.9 bits (128), Expect = 2e-07 |

| **Table 47H. Domain Analysis of NOV47** |
|---|
| gnl\|Pfam\|pfam02166, Androgen_recep, Androgen receptor. |
| CD-Length = 456 residues, 18.6% aligned |
| Score = 52.8 bits (125), Expect = 5e-07 |

The ALL-1 gene is involved in human acute leukemia through chromosome translocations or internal rearrangements. ALL-1 is the human homologue of Drosophila trithorax. The latter is a member of the trithorax group (trx-G) genes which together with the Polycomb group (Pc-G) genes act as positive and negative regulators, respectively, to determine the body structure of Drosophila. ALR encodes a gigantic 5262 amino acid long protein containing a SET domain, five PHD fingers, potential zinc fingers, and a very long run of glutamines interrupted by hydrophobic residues, mostly leucine. The SET motif, PDH fingers, zinc fingers and two other regions are most similar to domains of ALL-1 and TRX. The first two motifs are also found in other trx-G and Pc-G proteins. The ALR gene was mapped to chromosome band 12q12-13, adjacent to the VDR gene. This region is involved in duplications and translocations associated with cancer.

The human ALL-1/MLL/HRX gene on chromosome 11q23 is the site of many locally clustered chromosomal alterations associated with several types of acute leukemias, including deletions partial duplications and reciprocal translocations. Structurally variant proteins derived from an altered ALL-1 gene presumably make essential contributions to the malignant transformation of hematopoietic progenitor cells.

Many haematologic malignancies carry characteristic chromosomal translocations, which are thought to play an important role in the pathogenesis of these tumours. The t(8; 14) trarislocation in Burkitt's lymphoma was one of the first characterized at the molecular level. In this translocation the c-myc oncogene at chromosome 8q24 becomes deregulated by enhancer elements of the immunoglobulin heavy chain locus at chromosome 14q32 leading to a very aggressive B cell malignancy. Chromosomal translocations involving the MLL gene occur in about 80% of infant leukemias.

The disclosed NOV47 nucleic acid of the invention encoding a ALR-like protein includes the nucleic acid whose sequence is provided in Table 47A or a fragment thereof. The invention also includes a mutant or variant nucleic acid any of whose bases may be changed from the corresponding base shown in Table 47A while still encoding a protein that maintains its ALR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1 percent of the bases may be so changed.

The disclosed NOV47 protein of the invention includes the ALR-like protein whose sequence is provided in Table 47B. The invention also includes a mutant or variant protein any of whose residues may be changed from the corresponding residue shown in Table 47B while still encoding a protein that maintains its ALR-like activities and physiological functions, or a functional fragment thereof. In the mutant or variant protein, up to about 1 percent of the residues may be so changed.

The invention further encompasses antibodies and antibody fragments, such as F_{ab} or (F_{ab})₂, that bind immunospecifically to any of the proteins of the invention.

The above defined information for this invention suggests that this ALR-like protein (NOV47) may function as a member of a "ALR family". Therefore, the NOV47 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and in vitro of all tissues and cell types composing (but not limited to) those defined here.

The NOV47 nucleic acids and proteins of the invention are useful in potential therapeutic applications implicated in diseases including but not limited to various pathologies and disorders as indicated below. For example, a cDNA encoding the ALR-like protein (NOV47) may be useful in gene therapy, and the ALR-like protein (NOV47) may be useful when administered to a subject in need thereof. By way of nonlimiting example, the compositions of the present invention will have efficacy for treatment of patients suffering from Osteoporosis, involutional; Rickets, vitamin D-resistant; Fibrosis of extraocular muscles, congenital, 1; Achalasia-addisonianism-alacrimia syndrome; Cataract, polymorphic and lamellar; acute leukemias, cancers, or other pathologies or conditions. The NOV47 nucleic acid encoding the ALR-like protein of the invention, or fragments thereof, may further be useful in diagnostic applications, wherein the presence or amount of the nucleic acid or the protein are to be assessed.

NOV47 nucleic acids and polypeptides are further useful in the generation of antibodies that bind immuno-specifically to the novel NOV47 substances for use in therapeutic or diagnostic methods. These antibodies may be generated according to methods known in the art, using prediction from hydrophobicity charts, as described in the "Anti-NOVX Antibodies" section below. The disclosed NOV47 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV48

A disclosed NOV48 nucleic acid of 1988 nucleotides (also referred to as CG57713-01) encoding a sodium/bile acid transporter-like protein is shown in Table 48A. The start and stop codons are in bold letters.

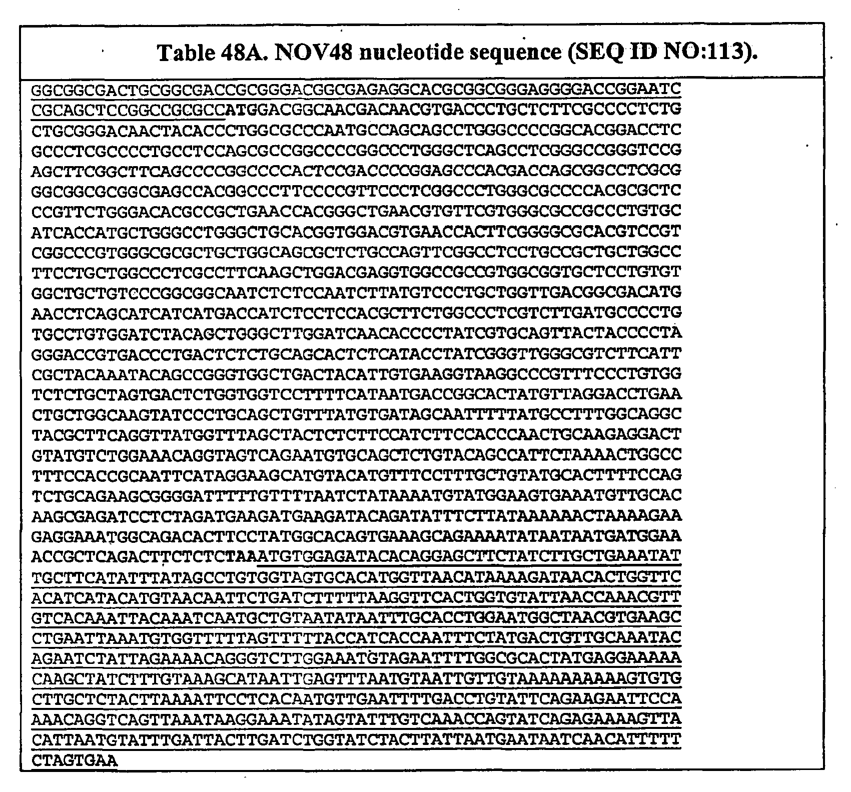

In a search of public sequence databases, the NOV48 nucleic acid sequence, located on chromsome 4 has 250 of 395 bases (63%) identical to a gb:GENBANK ID:HUMNTCP|acc:L21893.1 mRNA from Homo sapiens (Human Na/taurocholate cotransporting polypeptide mRNA, complete cds). Public nucleotide databases include all GenBank databases and the GeneSeq patent database.

The disclosed NOV48 polypeptide (SEQ ID NO:114) encoded by SEQ ID NO: 113 has 440 amino acid residues and is presented in Table 48B using the one-letter amino acid code. Signal P, Psort and/or Hydropathy results predict that NOV48 has no signal peptide and is likely to be localized in the plasma membrane with a certainty of 0.6000.

| | | | | | |
|---|---|---|---|---|---|
| gi\|2811069\|sp\|00870 5\|NTCP MOUSE | SODIUM/BILE ACID COTRANSPORTER (NA(+)/BILE ACID COTRANSPORTER) (NA(+)/TAUROCHOLA TE TRANSPORT PROTEIN) (SODIUM/TAUROCHOL ATE COTRANSPORTING POLYPEPTIDE) | 362 | 123/312 (39%) | 189/312 (60%) | e-52 |

Table 48D lists the domain descriptions from DOMAIN analysis results against NOV48. This indicates that the NOV48 sequence has properties similar to those of other proteins known to contain this domain.

| **Table 48D. Domain Analysis of NOV48** | | |
|---|---|---|
| gnl\|Pfam\|pfam01758, SBF, Sodium Bile acid symporter family. This family consists of Na+/bile acid co-transporters. These transmembrane proteins function in the liver in the uptake of bile acids from portal blood plasma a process mediated by the co-transport of Na+. Also in the family is ARC3 from S. cerevisiae this is a putative transmembrane protein involved in resistance to arsenic compounds. | | |
| | CD-Length = 186 residues, 95.7% aligned | |
| | | Score = 104 bits (259), Expect = 1e-23 |

AcDNA probe from a cloned rat liver Na+/taurocholate cotransporting polypeptide (Ntcp) was used to screen a human liver cDNA library. A 1,599-bp cDNA clone that encodes a human Na+/taurocholate cotransporting polypeptide (NTCP) was isolated. The human NTCP consists of 349 amino acids (calculated molecular mass of 38 kD) and exhibits 77% amino acid homology with the rat Ntcp. In vitro translation experiments indicate that the protein is glycosylated and has a molecular weight similar to the rat Ntcp. Injection of in vitro transcribed cRNA into Xenopus laevis oocytes resulted in the expression of Na(+)-dependent taurocholate uptake. Saturation kinetics indicated that the human NTCP has a higher affinity for taurocholate (apparent Km = 6 microM) than the previously cloned rat protein (apparent Km = 25 microM). NTCP-mediated taurocholate uptake into oocytes was inhibited by all major bile acid derivatives (100 microM), bumetanide (500 microM), and bromosulphophthalein (100 microM). Southern blot analysis of genomic DNA from a panel of human/hamster somatic cell hybrids mapped the human NTCP gene to chromosome 14. PMID: 8132774

Liver parenchymal cells continuously extract high amounts of bile acids from portal blood plasma. This uptake process is mediated by a Na+/bile acid cotransport system. A cDNA encoding the rat liver bile acid uptake system has been isolated by expression cloning in Xenopus laevis oocytes. The cloned transporter is strictly sodium-dependent and can be inhibited by various non-bile-acid organic compounds. Sequence analysis of the cDNA revealed an open reading frame of 1086 nucleotides coding for a protein of 362 amino acids (calculated molecular mass 39 kDa) with five possible N-linked glycosylation sites and seven putative transmembrane domains. Translation experiments in vitro and in oocytes indicate that the transporter is indeed glycosylated and that its polypeptide backbone has an apparent molecular mass of 33-35 kDa. Northern blot analysis with the cloned probe revealed crossreactivity with mRNA species from rat kidney and intestine as well as from liver tissues of mouse, guinea pig, rabbit, and man. PMID: 1961729

Using expression cloning in Xenopus laevis oocytes, a cDNA encoding a rat liver organic anion-transporting polypeptide (oatp) was isolated. The cloned oatp mediated Na(+)-independent uptake of sulfobromophthalein (BSP) which was Cl(-)-dependent in the presence of bovine serum albumin (BSA) at low BSP concentrations (e.g., 2 microM). Addition of increasing amounts of BSA had no effects on the maximal velocity of initial BSP uptake, but it increased the Km value from 1.5 microM (no BSA) to 24 microM (BSA/BSP molar ratio, 3.7) and 35 microM (BSA/BSP ratio, 18.4). In addition to BSP, the cloned oatp also mediated Na(+)-independent uptake of conjugated (taumcholate) and unconjugated (cholate) bile acids. Sequence analysis of the cDNA revealed an open reading frame of 2010 nucleotides coding for a protein of 670 amino acids (calculated molecular mass, 74 kDa) with four possible N-linked glycosylation sites and 10 putative transmembrane domains. Translation experiments in vitro indicated that the transporter was indeed glycosylated and that its polypeptide backbone had an apparent molecular mass of 59 kDa. Northern blot analysis with the cloned probe revealed crossreactivity with several mRNA species from rat liver, kidney, brain, lung, skeletal muscle, and proximal colon as well as from liver tissues of mouse and rabbit, but not of skate (Raja erinacea) and human. PMm: 8278353

Active uptake of bile acids from the lumen of the small intestine is mediated by an ileal Na(+)-dependent bile acid transport system. To identify components of this transport system, an expression cloning strategy was employed to isolate a hamster ileal cDNA that exhibits bile acid transport activity. By Northern blot analysis, mRNA for the cloned transporter was readily detected in ileum and kidney but was absent from liver and proximal small intestine. The transporter cDNA encoded a 348-amino acid protein with seven potential transmembrane domains and three possible N-linked glycosylation sites. The amino acid sequence was 35% identical and 63% similar to the rat liver Na+/bile acid cotransporter. After transfection into COS cells, the hamster cDNA transported taurocholate in a strict Na(+)-dependent fashion with an apparent Km of 33 microM. This taurocholate transport was inhibited by various bile acids but not by taurine or other organic anions. The Na+ dependence, saturability, and bile acid specificity of transport as well as the tissue specificity of mRNA expression strongly argue that the transporter cDNA characterized in this study is the Na+/bile acid cotransporter described previously in ileum. PMID: 8288599

Uptake of long-chain and aromatic neutral amino acids into cells is known to be catalyzed by the Na(+)-independent system L transporter, which is ubiquitous in animal cells and tissues.. The 2.3-kilobase cDNA codes for a protein of 683 amino acids. The transporter has four putative membrane-spanning domains and bears no sequence or structural homology to any known animal or bacterial transporter. When transcribed and expressed in Xenopus oocytes, the transporter exhibits many, but not all, of the characteristics of L-system transporters, suggesting that this represents one of several related L-system transporters. PMID: 1729674

The phylogenic and ontogenic expression of mRNA for the Na+/bile acid cotransporter was determined by Northern analysis utilizing a full-length cDNA probe recently cloned from rat liver. mRNA was detected in several mammalian species, including rat, mouse, and man, but could not be found in livers from nonmammalian species, including chicken, turtle, frog, and small skate. When expression of the bile acid transporter in developing rat liver was studied, mRNA was detected between 18 and 21 days of gestation, at the time when Na(+)-dependent bile acid transport is first detected. Two hepatoma cell lines (HTC and HepG2), the latter of which is known to have lost the Na+/bile acid cotransport system, also did not express mRNA for this transporter. Finally, when mRNA from the lower vertebrate (the small skate) was injected into Xenopus oocytes, only a sodium-independent, chloride-dependent transport system for bile acids was expressed, confirming the integrity of the mRNA and consistent with prior functional studies of bile acid transport in this species. These findings establish that the Na+/bile acid cotransport mRNA is first transcribed in mammalian species, a process that is recapitulated late during mammalian fetal development in rat liver, and that this mRNA is lost in dedifferentiated hepatocytes. In contrast, the mRNA for a multispecific Na+/independent organic anion transport system is transcribed earlier in vertebrate evolution. PMID: 8421672

The disclosed NOV48 nucleic acid of the invention encoding a sodium/bile acid transporter-like protein includes the nucleic acid whose sequence is provided in Table 48A or a fragment thereof. The invention also includes a mutant or variant nucleic acid any of whose bases mav be changed from the corresponding base shown in Table 48A while still encoding a protein that maintains its sodium/bile acid transporter-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 37 percent of the bases may be so changed.

The disclosed NOV48 protein of the invention includes the sodium/bile acid transporter-like protein whose sequence is provided in Table 48B. The invention also includes a mutant or variant protein any of whose residues may be changed from the corresponding residue shown in Table 48B while still encoding a protein that maintains its sodium/bile acid transporter-like activities and physiological functions, or a functional fragment thereof. In the mutant or variant protein, up to about 62 percent of the residues may be so changed.

The invention further encompasses antibodies and antibody fragments, such as F_{ab} or (F_{ab})₂, that bind immunospecifically to any of the proteins of the invention.

The above defined information for this invention suggests that this sodium/bile acid transporter-like protein (NOV48) may function as a member of a "sodium/bile acid transporter family". Therefore, the NOV48 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV48 nucleic acids and proteins of the invention are useful in potential therapeutic applications implicated in diseases including but not limited to various pathologies and disorders as indicated below. For example, a cDNA encoding the sodium/bile acid transporter-like protein (NOV48) may be useful in gene therapy, and the sodium/bile acid transporter-like protein (NOV48) may be useful when administered to a subject in need thereof. By way of nonlimiting example, the compositions of the present invention will have efficacy for treatment of patients suffering from cancer,trauma, regeneration (in vitro and in vivo), viral/bacterial/parasitic infections, Von Hippel-Lindau (VHL) syndrome, Cirrhosis,Transplantation, or other pathologies or conditions. The NOV48 nucleic acid encoding the sodium/bile acid transporter-like protein of the invention, or fragments thereof, may further be useful in diagnostic applications, wherein the presence or amount of the nucleic acid or the protein are to be assessed.

NOV48 nucleic acids and polypeptides are further useful in the generation of antibodies that bind immuno-specifically to the novel NOV48 substances for use in therapeutic or diagnostic methods. These antibodies may be generated according to methods known in the art, using prediction from hydrophobicity charts, as described in the "Anti-NOVX Antibodies" section below. The disclosed NOV48 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV49

A disclosed NOV49 nucleic acid of 2313 nucleotides (also referred to as CG57721-01) encoding a prestin-like protein is shown in Table 49A. The start and stop codons are in bold letters.

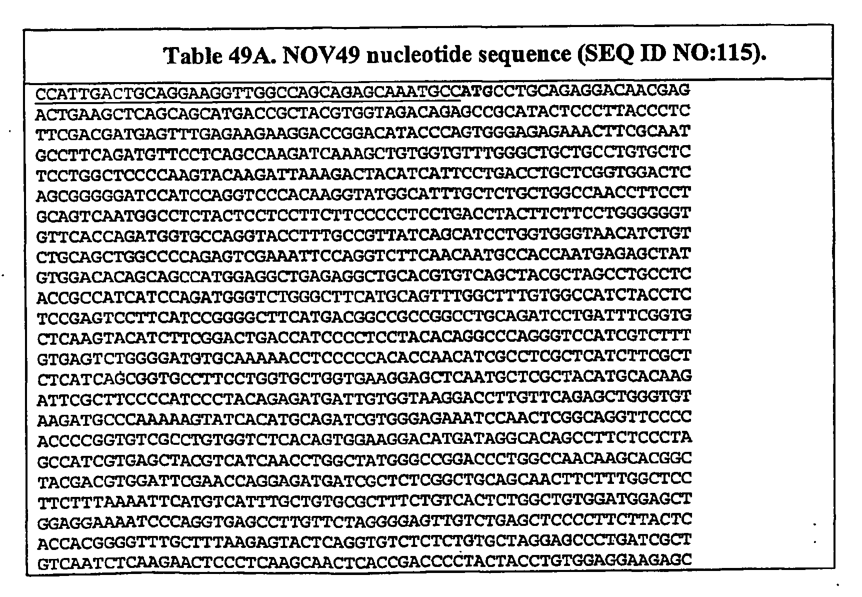

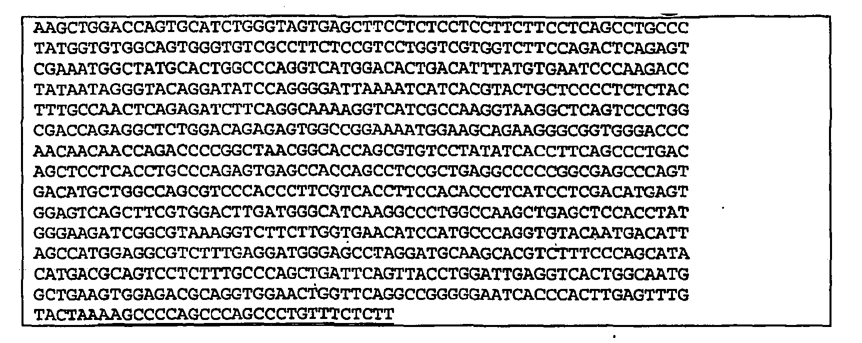

In a search of public sequence databases, the NOV49 nucleic acid sequence, located on chromsome 3 has 966 of 1618 bases (59%) identical to a gb:GBNBANK-ID:AF279265|acc:AF279265.1 mRNA from Homo sapiens (Homo sapiens putative anion transporter 1 mRNA, complete cds). Public nucleotide databases include all GenBank databases and the GeneSeq patent database.

The disclosed NOV49 polypeptide (SEQ ID NO:116) encoded by SEQ ID NO:115 has 748 amino acid residues and is presented in Table 49B using the one-letter amino acid code. Signal P, Psort and/or Hydropathy results predict that NOV49 has a signal peptide and is likely to be localized at the plasma membrane with a certainty of 0.6000. The most likely cleavage site for a NOV49 peptide is between amino acids 8 and 9.

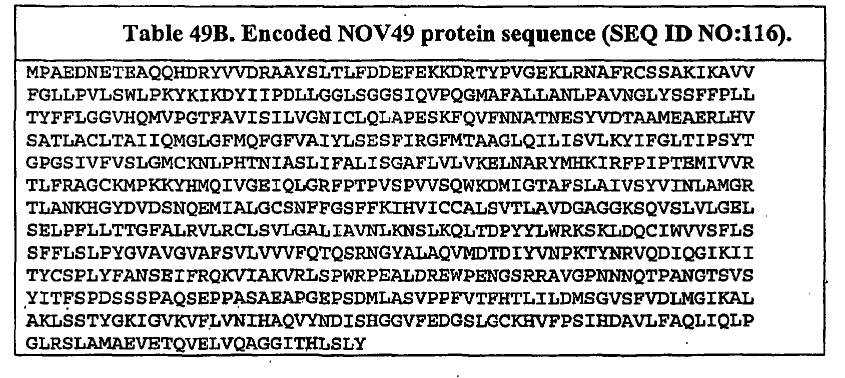

A search of sequence databases reveals that the NOV49 amino acid sequence has 277 of 732 amino acid residues (37%) identical to, and 434 of 732 amino acid residues (59%) similar to, the 744 amino acid residue ptnr:TREMBLNEW-ACC:CAC21555 protein from Rattus norvegicus (Rat) (PRESTIN). Public amino acid databases include the GenBank databases, SwissProt, PDB and PIR.

NOV49 is expressed in at least Parotid Salivary glands. Expression information was derived from the tissue sources of the sequences that were included in the derivation of the sequence of CG57721-O1.The sequence is predicted to be expressed in the following tissues because of the expression pattern of (GENBANK-ID: gb:GENBANK-ID:AF279265|acc:AF279265.1) a closely related Homo sapiens putative anion transporter 1 mRNA, complete cds homolog in species Homo sapiens :kidney and pancreas. This information was derived by determining the tissue sources of the sequences that were included in the invention including but not limited to SeqCalling sources, Public EST sources, Literature sources, and/or RACE sources.

The disclosed NOV49 polypeptide has homology to the amino acid sequences shown in the BLASTP data listed in Table 49C.

| **Table 49C. BLAST results for NOV49** | | | | | |
|---|---|---|---|---|---|
| Gene Index/ Identifier | Protein/ Organism | Length (aa) | Identity (%) | Positives (%) | Expect |
| gi\|16588681\|gb\|AAL2 6867.1\|AF314958 1(AF314958) | anion transporter/excha nger-9 [Homo sapiens] | 887 | 642/739 (86%) | 657/739 (88%) | 0.0 |
| gi\|16418413\|ref\|NP 443166.1 (NM_052934), | solute carrier family 26, member 9 [Homo sapiens] | 791 | 642/739 (86%) | 657/739 (88%) | 0.0 |
| gi\|15011891\|ref\|NP 109652.2\| (NM 030727) | prestin (motor protein) [Mus musculus] | 744 | 281/748 (37%) | 430/748 (56%) | e-129 |
| gi\|13540646\|ref\|NP 110467.1 (NM 030840) | prestin [Rattus norvegicus] | 744 | 278/746 (37%) | 435/746 (58%) | e-128 |
| gi\|8050590\|gb\|AAF71 715.1\|AF230376 1 (AF230376) | prestin [Meriones unguiculatus] | 744 | 271/738 (36%) | 424/738 (56%) | e-125 |

Tables 49D-E list the domain descriptions from DOMAIN analysis results against NOV49. This indicates that the NOV49 sequence has properties similar to those of other proteins known to contain this domain.

| **Table 49D. Domain Analysis of NOV49** |
|---|
| gnl\|Pfam\|pfam00916, Sulfate_transp, Sulfate transporter family. . Mutations in human Diastrophic Dysplasia Protein lead to several diseases. |
| CD-Length = 312 residues, 100.0% aligned |
| Score = 167 bits (423), Expect = 2e-42 |

| **Table 49E. Domain Analysis of NOV49** | | |
|---|---|---|
| gnl\|Pfam pfam01740, STAS, STAS domain. The STAS (after Sulphate Transporter and AntiSigma factor antagonist) domain is found in the C terminal region of Sulphate transporters and bacterial antisigma factor antagonists. It has been suggested that this domain may have a general NTP binding function. | | |
| | CD-Length = 106 residues, 62.3% aligned | |
| | | Score = 57.0 bits (136), Expect = 4e-09 |

A second distinct family of anion transporters, in addition to the classical SLC4 (or AE) family, has recently been delineated. Members of the SLC26 family are structurally well conserved and can mediate the electroneutral exchange of Cl(-) for HCO(-)(3) across the plasma membrane of mammalian cells like members of the SLC4 family. Three human transporter proteins have been functionally characterized: SLC26A2 (DTDST), SLC26A3 (CLD or DRA), and SLC26A4 (PDS) can transport with different specificities the chloride, iodine, bicarbonate, oxalate, and hydroxyl anions, whereas SLC26A5 (prestin) was suggested to act as the motor protein of the cochlear outer hair cell.

Electromotility, i.e., the ability of cochlear outer hair cells (OHCs) to contract and elongate at acoustic frequencies, is presumed to depend on the voltage-driven conformational changes of "motor" proteins present in the OHC lateral plasma membrane. Recently, two membrane proteins have been proposed as candidates for the OHC motor. A sugar transporter, GLUT-5, was proposed based on its localization in the OHCs and on the observation that sugar transport alters the voltage sensitivity of the OHC motor mechanism. Another candidate, "prestin," was identified from a subtracted OHC cDNA library and shown to impart voltage-driven shape changes to transfected cultured cells.

The disclosed NOV49 nucleic acid of the invention encoding a prestin-like protein includes the nucleic acid whose sequence is provided in Table 49A or a fragment thereof. The invention also includes a mutant or variant nucleic acid any of whose bases may be changed from the corresponding base shown in Table 49A while still encoding a protein that maintains its prestin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 41 percent of the bases may be so changed.

The disclosed NOV49 protein of the invention includes the prestin-like protein whose sequence is provided in Table 49B. The invention also includes a mutant or variant protein any of whose residues may be changed from the corresponding residue shown in Table 49B while still encoding a protein that maintains its prestin-like activities and physiological functions, or a functional fragment thereof. In the mutant or variant protein, up to about 63 percent of the residues may be so changed.

The invention further encompasses antibodies and antibody fragments, such as F_{ab} or (F_{ab})_{2,} that bind immunospecifically to any of the proteins of the invention.

The above defined information for this invention suggests that this prestin-like protein (NOV49) may function as a member of a "prestin family". Therefore, the NOV49 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV49 nucleic acids and proteins of the invention are useful in potential therapeutic applications implicated in diseases including but not limited to various pathologies and disorders as indicated below. For example, a cDNA encoding the prestin-like protein (NOV49) may be useful in gene therapy, and the prestin-like protein (NOV49) may be useful when administered to a subject in need thereof. By way of nonlimiting example, the compositions of the present invention will have efficacy for treatment of patients suffering from Autoimmune disease, Renal artery stenosis, Interstitial nephritis, Glomerulonephritis, Polycystic kidney disease, Systemic lupus erythematosus, Renal tubular acidosis, IgA nephropathy, Hypercalceimia, Lesch-Nyhan syndrome,Diabetes,Von Hippel-Lindau (VHL) syndrome, Pancreatitis, Obesity, Xerostomia, cancer,trauma, regeneration (in vitro and in vivo), viral/bacterial/parasitic infections, or other pathologies or conditions. The NOV49 nucleic acid encoding the prestin-like protein of the invention, or fragments thereof, may further be useful in diagnostic applications, wherein the presence or amount of the nucleic acid or the protein are to be assessed.

NOV49 nucleic acids and polypeptides are further useful in the generation of antibodies that bind immuno-specifically to the novel NOV49 substances for use in therapeutic or diagnostic methods. These antibodies may be generated according to methods known in the art, using prediction from hydrophobicity charts, as described in the "Anti-NOVX Antibodies" section below. The disclosed NOV49 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV50

A disclosed NOV50 nucleic acid of 1335 nucleotides (also referred to as CG57787-01) encoding a sulfate transporter-like protein is shown in Table 50A. The start and stop codons are in bold letters.

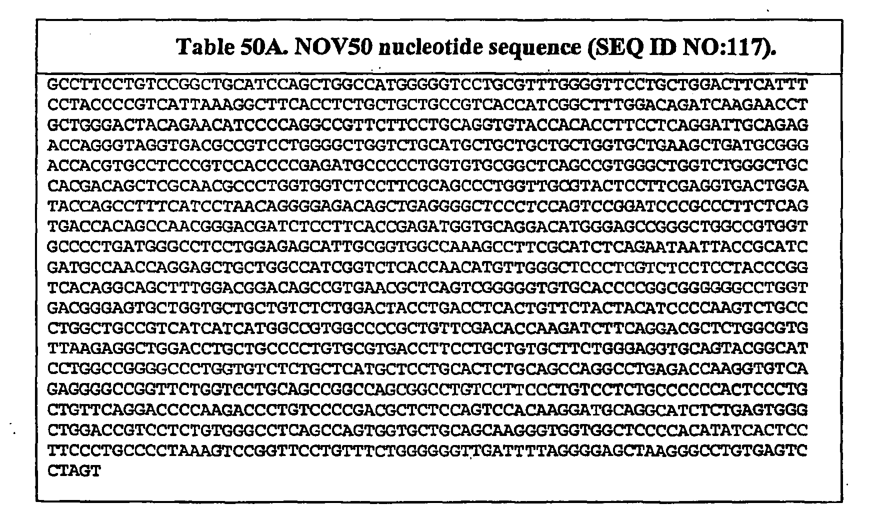

In a search of public sequence databases, the NOV50 nucleic acid sequence has 585 of 993 bases (58%) identical to a gb:GENBANK-ID:AF297659|acc:AF297659.2 mRNA from Homo sapiens (Homo sapiens sulfate/anion transporter SAT-1 protein (SLC26A1) mRNA, complete cds). Public nucleotide databases include all GenBank databases and the GeneSeq patent database.

The disclosed NOV50 polypeptide (SEQ ID NO:118) encoded by SEQ ID NO:117 has 384 amino acid residues and is presented in Table 50B using the one-letter amino acid code. Signal P, Psort and/or Hydropathy results predict that NOV50 has a signal peptide and is likely to be localized extracellularly with a certainty of 0.6000. The most likely cleavage site for a NOV50 peptide is between amino acids 20 and 21.

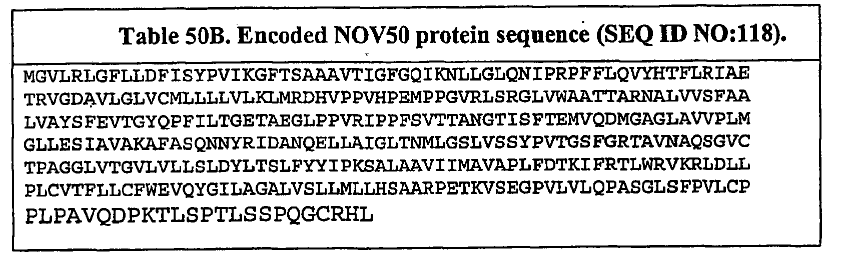

A search of sequence databases reveals that the NOV50 amino acid sequence has 146 of 339 amino acid residues (43%) identical to, and 210 of 339 amino acid residues (61%) similar to, the 595 amino acid residue ptnr:SPTREMBL-ACC:Q9V879 protein from Drosophila melanogaster (Fruit fly) (CG5002 PROTEIN). Public amino acid databases include the GenBank databases, SwissProt, PDB and PIR.

NOV50 is expressed in at least Brain, Cerebral Medulla/Cerebral white matter, Coronary Artery, Epidermis, Frontal Lobe, Hippocampus, Hypothalamus, Kidney, Liver, Lung, Mammary gland/Breast, Oviduct/Uterine Tube/Fallopian tube, Pituitary Gland, Retina, Spinal Chord, Spleen, Substantia Nigra, Temporal Lobe, Testis, Umbilical Vein, Whole Organism. Expression information was derived from the tissue sources of the sequences that were included in the derivation of the sequence of CG57787_01.The sequence is predicted to be expressed in the following tissues because of the expression pattern of (GENBANK-ID: gb:GENBANK-ID:AF297659|acc:AF297659.2) a closely related Homo sapiens sulfate/anion transporter SAT-1 protein (SLC26A1) mRNA, complete cds homolog in species Homo sapiens :liver. This information was derived by determining the tissue sources of the sequences that were included in the invention including but not limited to SeqCalling sources, Public EST sources, Literature sources, and/or RACE sources.

The disclosed NOV50 polypeptide has homology to the amino acid sequences shown in the BLASTP data listed in Table 50C.

| **Table 50C. BLAST results for NOV50** | | | | | |
|---|---|---|---|---|---|
| Gene Index/ Identifier | Protein/ Organism | Length (aa) | Identity (%) | Positives (%) | Expect |
| gi\|18485058\|ref\|XP 080006.1\| (XM_080006) | CG5002 [Drosophila melanogaster] | 595 | 121/341 (35%) | 169/341 (49%) | le-46 |
| gi\|7301216 gb AAF56 347.1\| (AE003749) | Esp gene product [Drosophila melanogaster] | 654 | 124/368 (33%) | 180/368 (48%) | 7e-44 |
| gi\|17738183\|ref\|NP 524490.1524490.1\| (NM_079766 | Epidermal stripes and patches [Drosophila melanogaster] | 623 | 127/370 (34%) | 179/370 (48%) | 3e-43 |
| gi\|7301881\|gb\|AAF56 989.1\| (AE003772 | CG7912 gene product [Drosophila melanogaster | 573 | 120/323 (37%) | 170/323 (52%) | 4e-42 |
| gi\|7300023\|gb\|AAF55 195.1\| (AE003708) | CG6125 gene product [Drosophila melanogaster] | 640 | 100/315 (31%) | 162/315 (50%) | 3e-38 |

Table 50D lists the domain descriptions from DOMAIN analysis results against NOV50. This indicates that the NOV50 sequence has properties similar to those of other proteins known to contain this domain.

| **Table 50D. Domain Analysis of NOV50** |
|---|
| gnl\|Pfam\|pfam00916, Sulfate_transp, Sulfate transporter family. Mutations in human Diastrophic Dysplasia Protein lead to several diseases. |
| CD-Length = 312 residues, 95.2% aligned |
| Score = 163 bits (413), Expect =1e-41 |

Lymphocytes continuously recirculate from blood, through lymphoid and other tissues, and back through the efferent lymphatics to the blood. The first critical step in lymphocyte migration from circulation into lymphoid tissues is the adhesion of lymphocytes to specialized postcapillary vascular sites called high endothelial venules (HEV).

Two vertebrate sulfate transporters that play a role in sulfate incorporation in other tissues are members of the superfamily of anion exchangers: the diastrophic dysplasia sulfate transporter (DTDST; 222600), which is mutant in diastrophic dysplasia and certain other skeletal dysplasias, and downregulated in adenoma (DRA; 126650), which is mutant in familial chloride diarrhea (214700). These 2 sulfate transporters contain 12 membrane-spanning domains and are sensitive to the anion-exchanger inhibitor DIDS. Girard et al. (1999) showed that HEVECs express 2 functional classes of sulfate transporters defined by their differential sensitivity to the DIDS anion-exchanger inhibitor. They reported the molecular characterization of a DIDS-resistant sulfate transporter from human HEVECs, designated SUT1. SUT1 belongs to the family of sodium-coupled anion transporters and exhibits 40 to 50% amino acid identity with the rat renal sodium/sulfate cotransporter NaSil, as well as with the human and rat sodium/dicarboxylate cotransporters NADC1/SDCT1 (604148) and NADC3/SDCT2.

The disclosed NOV50 nucleic acid of the invention encoding a sulfate transporter-like protein includes the nucleic acid whose sequence is provided in Table 50A or a fragment thereof. The invention also includes a mutant or variant nucleic acid any of whose bases may be changed from the corresponding base shown in Table 50A while still encoding a protein that maintains its sulfate transporter-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 42 percent of the bases may be so changed.

The disclosed NOV50 protein of the invention includes the sulfate transporter-like protein whose sequence is provided in Table 50B. The invention also includes a mutant or variant protein any of whose residues may be changed from the corresponding residue shown in Table 50B. while still encoding a protein that maintains its sulfate transporter-like activities and physiological functions, or a functional fragment thereof. In the mutant or variant protein, up to about 57 percent of the residues may be so changed.

The invention further encompasses antibodies and antibody fragments, such as F_{ab} or (F_{ab})₂, that bind immunospecifically to any of the proteins of the invention.

The above defined information for this invention suggests that this sulfate transporter-like protein (NOV50) may function as a member of a "sulfate transporter family". Therefore, the NOV50 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV50 nucleic acids and proteins of the invention are useful in potential therapeutic applications implicated in diseases including but not limited to various pathologies and disorders as indicated below. For example, a cDNA encoding the sulfate transporter-like protein (NOV50) may be useful in gene therapy, and the sulfate transporter-like protein (NOV50) may be useful when administered to a subject in need thereof. By way of nonlimiting example, the compositions of the present invention will have efficacy for treatment of patients suffering from diastrophic dysplasia and certain other skeletal dysplasias, and adenoma, familial chloride diarrhea, CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV50 nucleic acid encoding the sulfate transporter -like protein of the invention, or fragments thereof, may further be useful in diagnostic applications, wherein the presence or amount of the nucleic acid or the protein are to be assessed.

NOV50 nucleic acids and polypeptides are further useful in the generation of antibodies that bind immuno-specifically to the novel NOV50 substances for use in therapeutic or diagnostic methods. These antibodies may be generated according to methods known in the art, using prediction from hydrophobicity charts, as described in the "Anti-NOVX Antibodies" section below. The disclosed NOV50 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV51

A disclosed NOV51 nucleic acid of 2079 nucleotides (also referred to as CG57785-01) encoding a sulfate transporter-like protein is shown in Table 51A. The start and stop codons are in bold letters.

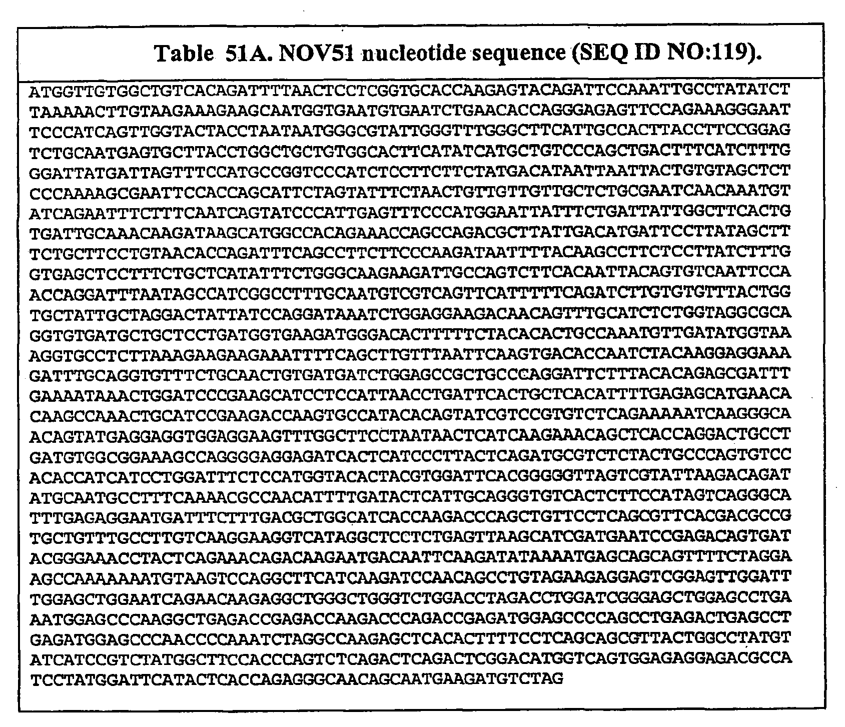

In a search of public sequence databases, the NOV51 nucleic acid sequence, located on chromsome 6 has 128 of 198 bases (64%) identical to a gb:GENBANK-ID:AF189262|acc:AF189262.1 mRNA from Rattus norvegicus (Rattus norvegicus GABA-A receptor epsilon-like subunit (Epsilon) mRNA, complete cds). Public nucleotide databases include all GenBank databases and the GeneSeq patent database.

The disclosed NOV51 polypeptide (SEQ ID NO:120) encoded by SEQ ID NO:119 has 692 amino acid residues and is presented in Table 51B using the one-letter amino acid code. Signal P, Psort and/or Hydropathy results predict that NOV51 has a signal peptide and is likely to be localized in the plasma membrane with a certainty of 0.6000. The most likely cleavage site for a NOV51 peptide is between amino acids 69 and 70.

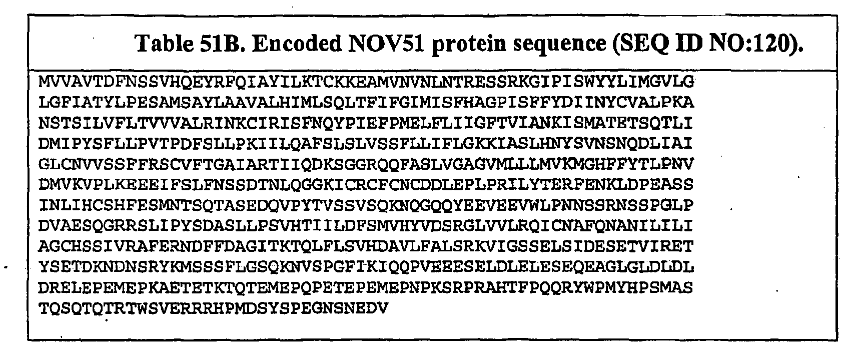

A search of sequence databases reveals that the NOV51 amino acid sequence has 123 of 123 amino acid residues (100%) identical to, and 123 of 123 amino acid residues (100%) similar to, the 123 amino acid residue ptnr:SPTREMBL-ACC:Q9NQP0 protein from Homo sapiens (Human) (BA48209.2 (NOVEL SULPHATE TRANSPORTER FAMILY MEMBER)). Public amino acid databases include the GenBank databases, SwissProt, PDB and PIR.

NOV51 is expressed in at least Adipose, Peripheral Blood, Spinal Chord, Testis, and Colon. Expression information was derived from the tissue sources of the sequences that were included in the derivation of the sequence of CGS7785_01. The sequence is predicted to be expressed in the following tissues because of the expression pattern of (GENBANK-ID: gb:GENBANK-ID:AF189262|acc:AF189262.1) a closely related Rattus norvegicus GABA-A receptor epsilon-like subunit (Epsilon) mRNA, complete cds homolog in species Rattus norvegicus :Adipose, Peripheral Blood, Spinal Chord, Testis,Colon. This information was derived by determining the tissue sources of the sequences that were included in the invention including but not limited to SeqCalling sources, Public EST sources, Literature sources, and/or RACE sources.

The disclosed NOV51 polypeptide has homology to the amino acid sequences shown in the BLASTP data listed in Table 51C.

| **Table 51C. BLAST results for NOV51** | | | | | |
|---|---|---|---|---|---|
| Gene Index/ Identifier | Protein/ organism | Length (aa) | Identity (%) | Positives (%) | Expect |
| gi\|16418457\|ref\|NP 443193.11\| (NM_052961 | solute carrier family 26, member 8 [Homo sapiens] | 970 | 394/396 (99%) | 394/396 (99%) | 0.0 |
| gi\|1658B684\|qb\|AAL2 6868-1\|AF314959 1 (AF314959) | anion transporter/excha nger-8 [Homo sapiens] | 970 | 405/464 (87%) | 418/464 (89%) | 0.0 |
| gi\|16552904\|dbj\|BAB 71408.1\| (AK057276) | unnamed protein product [Homo sapiens] | 278 | 171/182 (93%) | 172/182 (93%) | e-92 |
| gi\|9453726\|emb\|CAB9 9352.1\| (AL133507) | bA48209.2 (Novel sulphate transporter family member) [Homo sapiens] | 123 | 123/123 (100%) | 123/123 (100%) | 3e-62 |
| gi\|15080864\|gb\|AAK5 1131.1\|\| (AY032863 | chloride-formate exchanger [Mus musculus] | 734 | 78/251 (31%) | 142/251 (56%) | e-35 |

Tables 51D-E list the domain descriptions from DOMAIN analysis results against NOV51. This indicates that the NOVS1 sequence has properties similar to those of other proteins known to contain this domain.

| **Table 51D. Domain Analysis of NOV51** |
|---|
| gnlPfam pfam00916, Sulfate_transp, Sulfate transporter family. Mutations in human Diastrophic Dysplasia Protein lead to several diseases. |
| CD-Length = 312 residues, 79.5% aligned |
| Score = 145 bits (366), Expect = 7e-36 |

| **Table 51E. Domain Analysis of NOV51** | | |
|---|---|---|
| gnl\|Pfam\|pfam01740, STAS, STAS domain. The STAS (after Sulphate Transporter and AntiSigma factor antagonist) domain is found in the C terminal region of Sulphate transporters and bacterial antisigma factor antagonists. It has been suggested that this domain may have a general NTP binding function. | | |
| | CD-Length = 106 residues, 63.2% aligned | |
| | | Score = 53.1 bits (126), Expect = 5e-08 |

The disclosed NOV51 nucleic acid of the invention encoding a sulfate transporter-like protein includes the nucleic acid whose sequence is provided in Table 51A or a fragment thereof. The invention also includes a mutant or variant nucleic acid any of whose bases may be changed from the corresponding base shown in Table 51A while still encoding a protein that maintains its sulfate transporter-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 36 percent of the bases may be so changed.

The disclosed NOV51 protein of the invention includes the sulfate transporter-like protein whose sequence is provided in Table 51B. The invention also includes a mutant or variant protein any of whose residues may be changed from the corresponding residue shown in Table 51B while still encoding a protein that maintains its sulfate transporter-like activities and physiological functions, or a functional fragment thereof. In the mutant or variant protein, up to about 0 percent of the residues may be so changed.

The invention further encompasses antibodies and antibody fragments, such as F_{ab} or (F_{ab})₂, that bind immunospecifically to any of the proteins of the invention.

The above defined information for this invention suggests that this sulfate transporter-like protein (NOV51) may function as a member of a "sulfate transporter family". Therefore, the NOV51 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV51 nucleic acids and proteins of the invention are useful in potential therapeutic applications implicated in diseases including but not limited to various pathologies and disorders as indicated below. For example, a cDNA encoding the sulfate transporter-like protein (NOV51) may be useful in gene therapy, and the sulfate transporter-like protein (NOV51) may be useful when administered to a subject in need thereof. By way of nonlimiting example, the compositions of the present invention will have efficacy for treatment of patients suffering from diastrophic dysplasia and certain other skeletal dysplasias, and adenoma, familial chloride diarrhea, CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV51 nucleic acid encoding the sulfate transporter-like protein of the invention, or fragments thereof, may further be useful in diagnostic applications, wherein the presence or amount of the nucleic acid or the protein are to be assessed.

NOV51 nucleic acids and polypeptides are further useful in the generation of antibodies that bind immuno-specifically to the novel NOV51 substances for use in therapeutic or diagnostic methods. These antibodies may be generated according to methods known in the art, using prediction from hydrophobicity charts, as described in the "Anti-NOVX Antibodies" section below. The disclosed NOV51 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV52

A disclosed NOV52 nucleic acid of 2210 nucleotides (also referred to as CG57748-01) encoding a N-acetylgalactosaminyltransferase-like protein is shown in Table 52A. The start and stop codons are in bold letters.

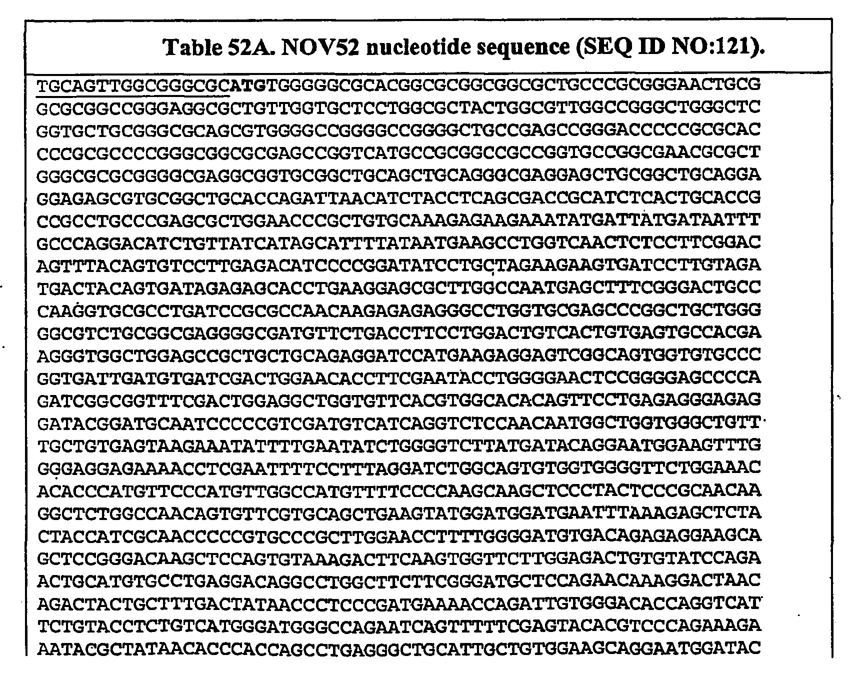

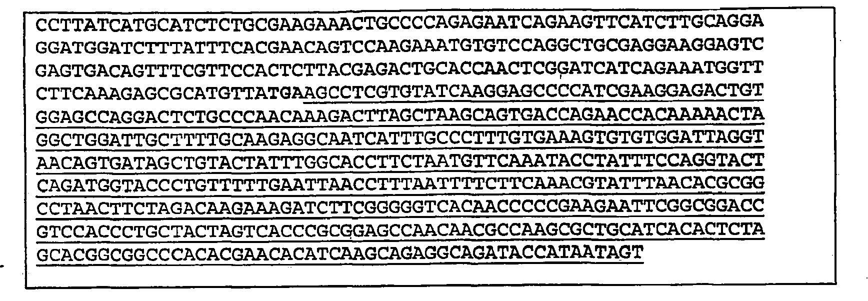

In a search of public sequence databases, the NOV52 nucleic acid sequence, located on chromsome has 951 of 1468 bases (64%) identical to a gb:GENBANK-ID:MMU73819|acc:U73819.1 mRNA from Mus musculus (Mus musculus polypeptide GalNAc transferase-T4 (ppGaNTase-T4) mRNA, complete cds). Public nucleotide databases include all GenBank databases and the GeneSeq patent database.

The disclosed NOV52 polypeptide (SEQ ID NO:122) encoded by SEQ ID NO:121 has 581 amino acid residues and is presented in Table 52B using the one-letter amino acid code. Signal P, Psort and/or Hydropathy results predict that NOV52 has a signal peptide and is likely to be localized extracellularly with a certainty of 0.8200. The most likely cleavage site for a NOV52 peptide is between amino acids 39 and 40.

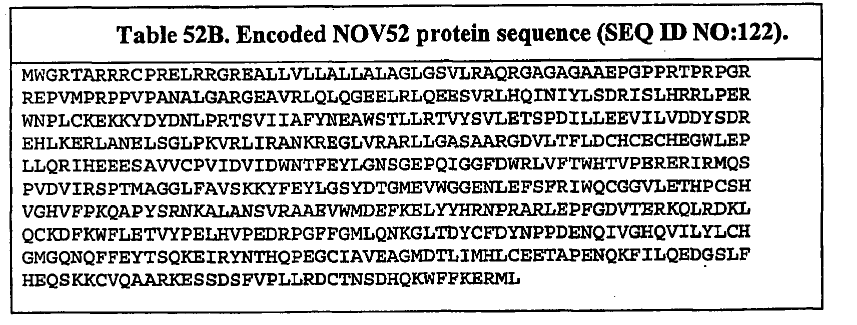

A search of sequence databases reveals that the NOV52 amino acid sequence has 330 of 566 amino acid residues (58%) identical to, and 408 of 566 amino acid residues (72%) similar to, the 578 amino acid residue ptnr:SPTREMBL-ACC:008832 protein from Mus musculus (Mouse) (POLYPEPTIDE GALNAC TRANSFERASE-T4). Public amino acid databases include the GenBank databases, SwissProt, PDB and PIR.

NOV52 is expressed in at least bone marrow, colon, lung, ovary, kidney, respiratory bronchiole, stomach, testis, tonsils, and germ cells. Expression information was derived from the tissue sources of the sequences that were included in the derivation of the sequence of CG57748-01.The sequence is predicted to be expressed in the following tissues because of the expression pattern of (GENBANK-ID: gb:GENBANK-ID:MMU73819|acc:U73819.1) a closely related Mus musculus polypeptide GaINAc transferase-T4 (ppGaNTase-T4) mRNA, complete cds homolog in species Mus musculus :spleen. This information was derived by determining the tissue sources of the sequences that were included in the invention including but not limited to SeqCalling sources, Public EST sources, Literature sources, and/or RACE sources.

The disclosed NOV52 polypeptide has homology to the amino acid sequences shown in the BLASTP data listed in Table52C.

| **Table 52C. BLAST results for NOV52** | | | | | |
|---|---|---|---|---|---|
| Gene Index/ Identifier | Protein/ Organism | Length (aa) | Identity (%) | Positives (%) | Expect |
| qi\|7657112\|ref\|NP 056552.1\| (NM_015737) | UDP-N-acetyl-alpha-D-galactosamine:poly peptide N-acetylgalactosamin yltransferase 4; ppGaNTase-T4 [Mus musculus] | 578 | 330/570 (57%) | 408/570 (70%) | e-180 |
| gi\|4503901\|ref\|NP 003765.1\| (NM_003774 | polypeptide N-acetylgalactosamin yltransferase 4 UDP-N-acetyl-alpha-D-galactosamine:poly peptide N-acetylgalactosamin yltransferase 4; GalNAc transferase 4; UDP-GalNAc: polypeptide N-acetylgalactosamin yltransferase 4 | 578 | 324/570 (56%) | 402/570 (69%) | e-179 |
| gi\|13375881\|ref\|NP 078918.1\| (NM 024642) | protein FLJ21212 [Homo sapiens] | 284 | 284/284 (100%) | 284/284 (100%) | e-174 |
| gi\|15530299\|gb\|AAH 13945.1\|AAH13945 (BC013945) | Similar to hypothetical protein FLJ21212 [Homo sapiens] | 272 | 272/272 (100%) | 272/272 (100%) | e-166 |
| gi\|14530626 \|emb\|CA C42368.1\|(AL110487) | cDNA EST EMBL:AF031835 comes from this gene [Caenomabditis elegans] | 623 | 248/529 (46%) | 322/529 (59%) | e-128 |

Tables 52D-E list the domain descriptions from DOMAIN analysis results of NOV52. This indicates that the NOV52 sequence has properties similar to those of other proteins known to contain this domain.

| **Table 52D. Domain Analysis of NOV52** |
|---|
| gnl\|Pfam\|pfam00535, Glycos_transf_2, Glycosyl transferase. Diverse family, transferring sugar from UDP-glucose, UDP-N-acetyl-galactosamine, GDP-mannose or CDP-abequose, to a range of substrates including cellulose, dolichol phosphate and teichoic acids. |
| CD-Length = 168 residues, 100.0% aligned |
| Score = 109 bits (273), Expect = 4e-25 |

| **Table 52E. Domain Analysis of NOV52** | |
|---|---|
| gnl\|Smart\|smart 00458, RICIN, Ricin-type beta-trefoil; Carbohydrate-binding domain formed from presumed gene triplication | |
| | CD-Length = 125 residues, 97.6% aligned |
| | Score = 78.2 bits (191), Expect = 1e-15 |

NOV52 has homology with uridine diphosphate (UDP)-GalNAc: polypeptide N-acetylgalactosaminyltransferase (GalNAc transferase), a member of the glycosyl transferase family. This enzyme catalyzes the initial step in mucin-type O-glycosylation of specific proteins. Glycosylation of cell surface proteins is critical to normal development, immune response and tissue functions, as evidenced by the phenotypes of a number of mouse knockout models (See Muramatsu; J Biochem (Tokyo) 2000 Feb;127(2):171-6). Glycosylation patterns are known to change during the process of carcinogenesis (Kohsaki et al., J Gastroenterol 2000;35(11):840-8). Alterations of these patterns by introducing a transgene coding for a GalNAc transferase (See Tsurifune et al., Int J Onco1 2000 Jul;17(1):159-65)or by means of antisense oligonucleotides (See Zeng et al., Proc Natl Acad Sci U S A 1995 Sep 12;92(19):8670-4) alter cell morphology, growth and adhesion patterns. Therefore these proteins are important markers and therapeutic targets for oncology applications. In addition, a member of this family has been implicated in autosomal dominant hypophosphatemic rickets (See White et al., Gene 2000 Apr 4;246(1-2):347-56).

Glycosyl transferases comprise a fairly diverse group of proteins that catalyze the addition of sugar from UDP-glucose, UDP-N-acetyl-galactosamine, GDP-mannose or CDP-abequose, to a range of substrates including cellulose, dolichol phosphate and teichoic acids.

The disclosed NOV52 nucleic acid of the invention encoding a N-acetylgalactosaminyltransferase-like protein includes the nucleic acid whose sequence is provided in Table 52A or a fragment thereof. The invention also includes a mutant or variant nucleic acid any of whose bases may be changed from the corresponding base shown in Table 52A while still encoding a protein that maintains its N-acetylgalactosaminyltransferase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 36 percent of the bases may be so changed.

The disclosed NOV52 protein of the invention includes the N-acetylgalactosaminyltransferase-like protein whose sequence is provided in Table 52B. The invention also includes a mutant or variant protein any of whose residues may be changed from the corresponding residue shown in Table 52B while still encoding a protein that maintains its N-acetylgalactosaminyltransferase-like activities and physiological functions, or a functional fragment thereof. In the mutant or variant protein, up to about 42 percent of the residues may be so changed.

The invention further encompasses antibodies and antibody fragments, such as F_{ab} or (F_{ab})₂, that bind immunospecifically to any of the proteins of the invention.

The above defined information for this invention suggests that this N-acetylgalactosaminyltransferase-like protein (NOV52) may function as a member of a "N-acetylgalactosaminyltransferase family". Therefore, the NOV52 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV52 nucleic acids and proteins of the invention are useful in potential therapeutic applications implicated in diseases including but not limited to various pathologies and disorders as indicated below. For example, a cDNA encoding the N-acetylgalactosaminyltransferase-like protein (NOV52) may be useful in gene therapy, and the N-acetylgalactosaminyltransferase-like protein (NOV52) may be useful when administered to a subject in need thereof. By way of nonlimiting example, the compositions of the present invention will have efficacy for treatment of patients suffering from hemophilia, hypercoagulation, idiopathic thrombocytopenic purpura, immunodeficiencies, graft versus host disease, hypercoagulation, autoimmune disease, allergies,transplantation, Hirschsprung's disease, Crohn's Disease, appendicitis, systemic lupus erythematosus, autoimmune disease, asthma, emphysema, scleroderma, allergy, ARDS, endometriosis, fertility, diabetes, renal artery stenosis; interstitial nephritis, glomerulonephritis, polycystic kidney disease, systemic lupus erythematosus, renal tubular acidosis, IgA nephropathy, hypercalceimia, Lesch-Nyhan syndrome, hypercalceimia, ulcers, fertility, hypogonadism, polycystic ovarian syndrome, cancer, tissue degeneration, bacterial/viral/parasitic infection, or other pathologies or conditions. The NOV52 nucleic acid encoding the N-acetylgalactosaminyltransferase-like protein of the invention, or fragments thereof, may further be useful in diagnostic applications, wherein the presence or amount of the nucleic acid or the protein are to be assessed.

NOV52 nucleic acids and polypeptides are further useful in the generation of antibodies that bind immuno-specifically to the novel NOV52 substances for use in therapeutic or diagnostic methods. These antibodies may be generated according to methods known in the art, using prediction from hydrophobicity charts, as described in the "Anti-NOVX Antibodies" section below. The disclosed NOV52 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV53

A disclosed NOV53 nucleic acid of 2030 nucleotides (also referred to as CG57693-01) encoding a protein kinase-like protein is shown in Table 53A. The start and stop codons are in bold letters.

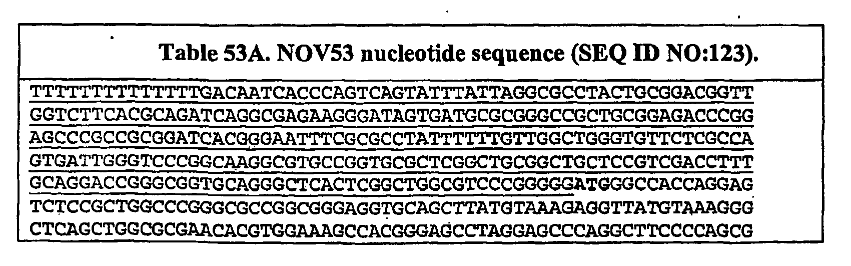

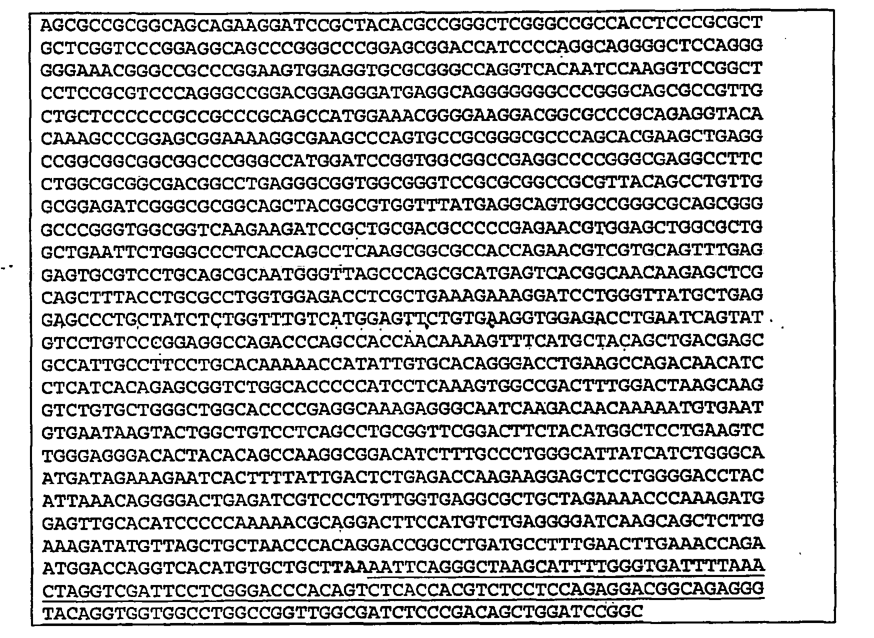

In a search of public sequence databases, the NOV53 nucleic acid sequence, located on chromsome 20 has 262 of 361 bases (72%) identical to a gb:GENBANK-ID:AB041802|acc:AB041802.1 mRNA from Mus musculus (Mus musculus brain cDNA, clone MNCb-1723). Public nucleotide databases include all GenBank databases and the GeneSeq patent database.

The disclosed NOV53 polypeptide (SEQ ID NO:124) encoded by SEQ ID NO:123 has 533 amino acid residues and is presented in Table 53B using the one-letter amino acid code. Signal P, Psort and/or Hydropathy results predict that NOV53 has no signal peptide and is likely to be localized to the cytoplasm with a certainty of 0.8500.

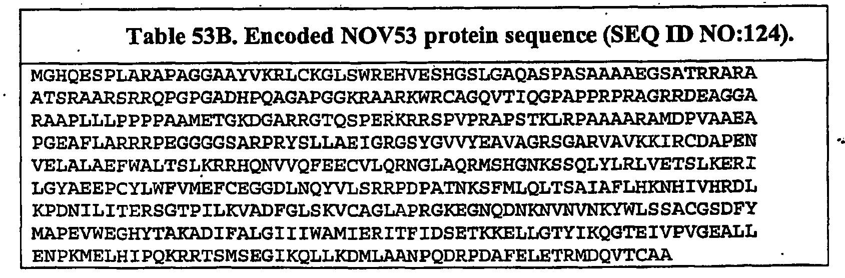

A search of sequence databases reveals that the NOV53 amino acid sequence has 517 of 517 amino acid residues (100%) identical to, and 517 of 517 amino acid residues (100%) similar to, the 517 amino acid residue ptnr:TREMBLNEW-ACC:CAC10518 protein from Homo sapiens (Human) (BA550O8.2 (NOVEL PROTEIN KINASE)). Public amino acid databases include the GenBank databases, SwissProt, PDB and PIR.

NOV53 is expressed in at least Adrenal Gland/Suprarenal gland, Lymphoid tissue, Oviduct/Uterine Tube/Fallopian tube, Peripheral Blood, Placenta, Retina, Thymus. This information was derived by determining the tissue sources of the sequences that were included in the invention including but not limited to SeqCalling sources, Public EST sources, Literature sources, and/or RACE sources.

The disclosed NOV53 polypeptide has homology to the amino acid sequences shown in the BLASTP data listed in Table 53C.

| **Table 53C. BLAST results for NOV53** | | | | | |
|---|---|---|---|---|---|
| Gene Index/ Identifier | Protein/ Organism | Length (aa) | Identity (%) | Positives (%) | Expect |
| gi\|12830335\|emb\|CAC 10518.2 (AL359916) | bA55008.2 (novel protein kinase) [Homo sapiens] | 517 | 517/517 (100%) | 517/517 (100%) | 0.0 |
| gi\|18592261\|ref\|XP 086681.1 (XM_086681) | serine/threonine kinase 35 [Homo sapiens] | 401 | 400/401 (99%) | 400/401 (99%) | 0.0 |
| gi\|16878290\|gb\|AAH1 7340.1 AAH17340 (BC017340) | (protein for IMAGE:4869353) [Homo sapiens] | 398 | 395/398 (99%) | 395/398 (99%) | 0.0 |
| gi\|8549074\|ref\|XP 086530.1 (XM_086530) | similar to Cell division control protein 2 homolog (P34 protein kinase) [Homo sapiens] | 161 | 101/156 (64%) | 129/156 (81%) | e-56 |
| gi\|15224378\|ref\|NP 181320.11 (NM_129340) | putative protein kinase [Arabidopsis thalianal | 257 | 92/314 (29%) | 143/314 (45%) | e-23 |

Tables 53D-F list the domain descriptions from DOMAIN analysis results against NOV53. This indicates that the NOV53 sequence has properties similar to those of other proteins known to contain this domain.

| **Table 53D. Domain Analysis of NOV53** |
|---|
| gnl\|Smart\|smart00220, S_TKc, Serine/Threonine protein kinases, catalytic domain; Phosphotransferases. Serine or threonine-specific kinase subfamily. |
| CD-Length = 256 residues, 97.7% aligned |
| Score = 193 bits (491), Expect = 2e-50 |

| **Table 53E. Domain Analysis of NOV53** | | |
|---|---|---|
| gnl\|Pfam\|pfam00069, pkinase,, Protein kinase domain | | |
| | CD-Length = 256 residues, 97.7% aligned | |
| | | Score = 189 bits (481), Expect = 3e-49 |

| **Table 53F. Domain Analysis of NOV53** | | |
|---|---|---|
| gnl\|Smart \|smart00219, TyrKc, Tyrosine kinase, catalytic domain; Phosphotransferases. Tyrosine-specific kinase subfamily | | |
| | CD-Length = 258 residues, 99.6% aligned | |
| | | Score = 121 bits (303), Expect = le-28 |

Protein phosphorylation is a fundamental process for the regulation of cellular functions. The coordinated action of both protein kinases and phosphatases controls the levels of phosphorylation and, hence, the activity of specific target proteins. One of the predominant roles of protein phosphorylation is in signal transduction, where extracellular signals are amplified and propagated by a cascade of protein phosphorylation and dephosphorylation events. Eukaryotic protein kinases are enzymes that belong to a very extensive family of proteins which share a conserved catalytic core common with both serine/threonine and tyrosine protein kinases. There are a number of conserved regions in the catalytic domain of protein kinases. In the N-terminal extremity of the catalytic domain there is a glycine-rich stretch of residues in the vicinity of a lysine residue, which has been shown to be involved in ATP binding. In the central part of the catalytic domain there is a conserved aspartic acid residue which is important for the catalytic activity of the enzyme. Protein kinases are excellent small molecule drug targets for therapeutic intervention.

The disclosed NOV53 nucleic acid of the invention encoding a protein kinase-like protein includes the nucleic acid whose sequence is provided in Table 53A or a fragment thereof. The invention also includes a mutant or variant nucleic acid any of whose bases may be changed from the corresponding base shown in Table 53A while still encoding a protein that maintains its protein kinase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 28 percent of the bases may be so changed.

The disclosed NOV53 protein of the invention includes the protein kinase-like protein whose sequence is provided in Table 53B. The invention also includes a mutant or variant protein any of whose residues may be changed from the corresponding residue shown in Table 53B while still encoding a protein that maintains its protein kinase-like activities and physiological functions, or a functional fragment thereof. In the mutant or variant protein, up to about 0 percent of the residues may be so changed.

The invention further encompasses antibodies and antibody fragments, such as F_{ab} or (F_{ab})₂, that bind immunospecifically to any of the proteins of the invention.

The above defined information for this invention suggests that this protein kinase-like protein (NOV53) may function as a member of a "protein kinase family". Therefore, the NOV53 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV53 nucleic acids and proteins of the invention are useful in potential therapeutic applications implicated in diseases including but not limited to various pathologies and disorders as indicated below. For example, a cDNA encoding the protein kinase-like protein (NOV53) may be useful in gene therapy, and the protein kinase-like protein (NOV53) may be useful when administered to a subject in need thereof. By way of nonlimiting example, the compositions of the present invention will have efficacy for treatment of patients suffering from adrenoleukodystrophy , congenital adrenal hyperplasia, anemia, ataxia-telangiectasia, autoimmune disease, fertility, hemophilia, hypercoagulation, idiopathic thrombocytopenic purpura, graft versus host disease, allergies, immunodeficiencies, transplantation, graft versus host disease (GVHD), lymphaedema, Von Hippel-Lindau (VHL) syndrome, diabetes, tuberous sclerosis, or other pathologies or conditions. The NOV53 nucleic acid encoding the protein kinase-like protein of the invention, or fragments thereof, may further be useful in diagnostic applications, wherein the presence or amount of the nucleic acid or the protein are to be assessed.

NOV53 nucleic acids and polypeptides are further useful in the generation of antibodies that bind immuno-specifically to the novel NOV53 substances for use in therapeutic or diagnostic methods. These antibodies may be generated according to methods known in the art, using prediction from hydrophobicity charts, as described in the "Anti-NOVX Antibodies" section below. The disclosed NOV53 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV54

A disclosed NOV54 nucleic acid of 3331 nucleotides (also referred to as CG57707-01) encoding a Leucine-rich glioma-inactivated protein precursor -like protein is shown in Table 54A. The start and stop codons are in bold letters.

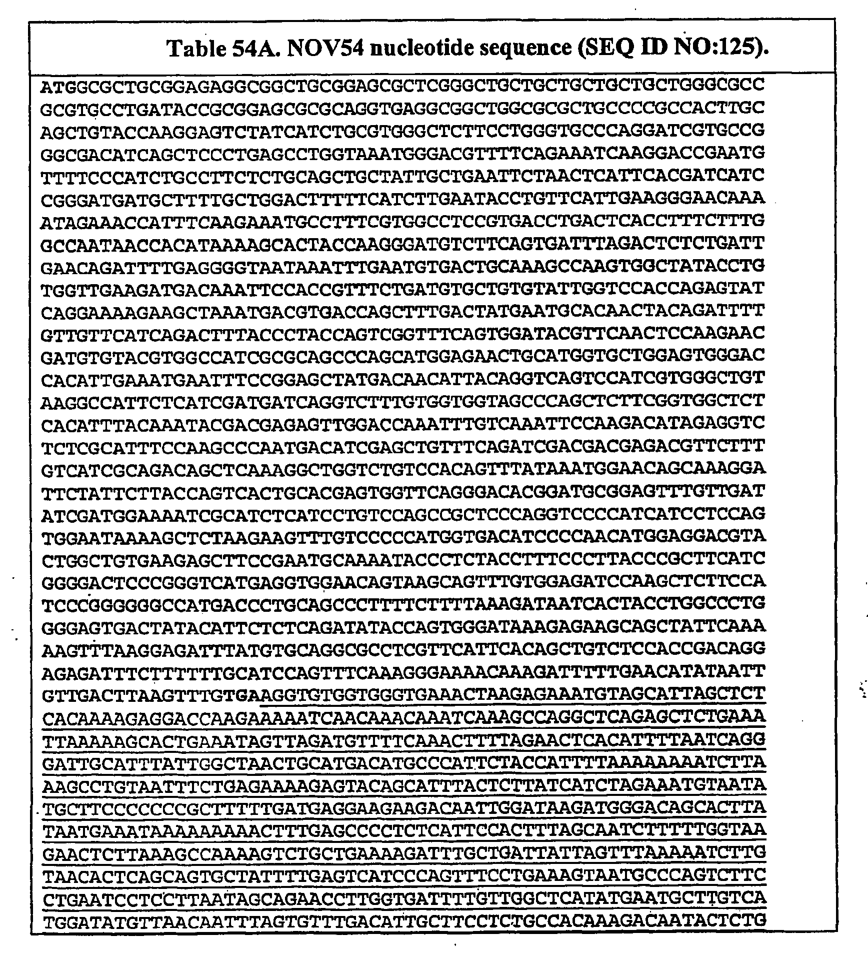

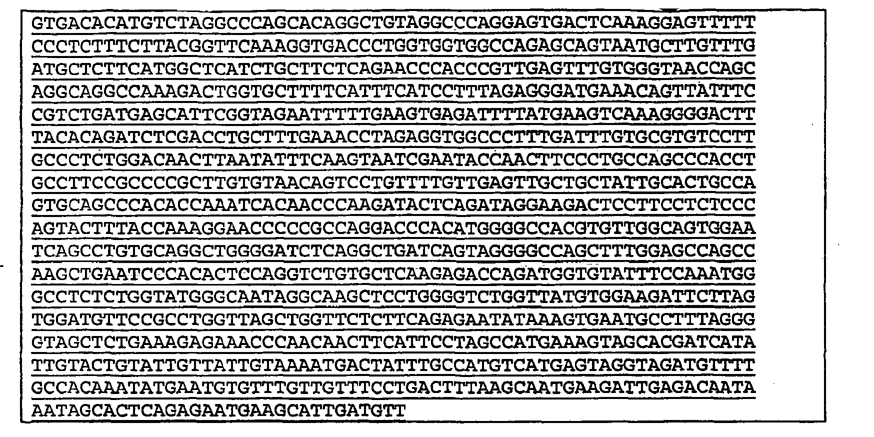

In a search of public sequence databases, the NOV54 nucleic acid sequence, located on chromsome 4p16 has 682 of 1052 bases (64%) identical to a gb:GENBANK-ID:AF055636|acc:AF055636.1 mRNA from Homo sapiens (Homo sapiens leucine-rich glioma-inactivated protein precursor (LGI1) mRNA, complete cds). Public nucleotide databases include all GenBank databases and the GeneSeq patent database.

The disclosed NOV54 polypeptide (SEQ ID NO:126) encoded by SEQ ID NO:125 has 545 amino acid residues and is presented in Table 54B using the one-letter amino acid code. Signal P, Psort and/or Hydropathy results predict that NOV54 has a signal peptide and is likely to be localized localized extracellularly with a certainty of 0.8200. The most likely cleavage site for a NOV54 peptide is between amino acids 22 and 23.

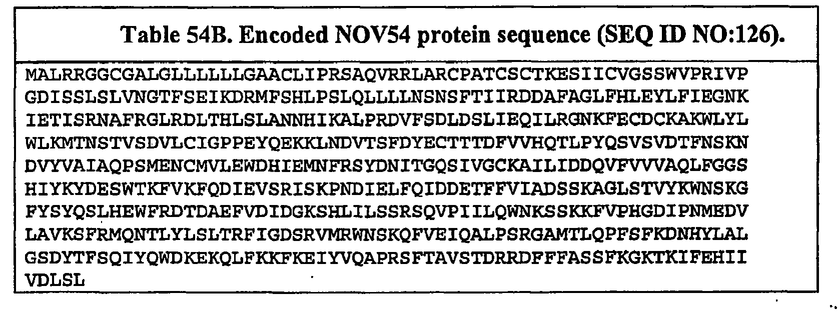

A search of sequence databases reveals that the NOV54 amino acid sequence has 301 of 538 amino acid residues (55%) identical to, and 386 of 538 amino acid residues (71%) similar to, the 557 amino acid residue ptnr:SPTREMBL-ACC:O95970 protein from Homo sapiens (Human) (LEUCINE-RICH GLIOMA-INACTIVATED PROTEIN PRECURSOR). Public amino acid databases include the GenBank databases, SwissProt, PDB and PIR.

NOV54 is expressed in at least Appendix, brain, colon, heart, kidney, ovary, pancreas, parathyroid gland, uterus, and vein. This information was derived by determining the tissue sources of the sequences that were included in the invention including but not limited to SeqCalling sources, Public EST sources, Literature sources, and/or RACE sources.

The disclosed NOV54 polypeptide has homology to the amino acid sequences shown in the BLASTP data listed in Table 54C.

| **Table 54C. BLAST results for NOV54** | | | | | |
|---|---|---|---|---|---|
| Gene Index/ Identifier | Protein/ Organism | Length (aa) | Identity (%) | Positives (%) | Expect |
| gi\|15620891\|dbj\|BAB 67809.1\| (AB067503) | KIAA1916 protein [Homo sapiens] | 542 | 539/542 (99%) | 539/542 (99%) | 0.0 |
| gi\|9938002\|ref\|NP 064674.1\| (NM_020278) | leucine-rich, glioma inactivated 1 [Mus musculus] | 557 | 296/516 (57%) | 378/516 (72%) | e-178 |
| gi 4826816 ref NP 0 05088.1 (NM_005097) | leucine-rich, glioma inactivated 1 precursor [Homo sapiens] | 557 | 296/516 (57%) | 379/516 (73%) | e-178 |
| gi\|15722102\|emb\|CAC 78757.1\| (AL358154 | bA512J3.1 (leucine-rich, glioma inactivated 1) [Homo sapiens] | 461 | 269/460 (58%) | 342/460 (73%) | e-160 |
| gi\|118591028\|ref\|XP 092048.1\| (XM 092048) | protein XP_092048 [Homo sapiens] | 466 | 179/437 (40%) | 267/437 (60%) | 3e-93 |

Table 54D lists the domain descriptions from DOMAIN analysis results against NOV54. This indicates that the NOV54 sequence has properties similar to those of other proteins known to contain this domain.

| **Table 54D. Domain Analysis of NOV54** |
|---|
| gnl\|Pfam\|pfam01463, LRRCT, Leucine rich repeat C-terminal domain. Leucine Rich Repeats pfam00560 are short sequence motifs present in a number of proteins with diverse functions and cellular locations. Leucine Rich Repeats are often flanked by cysteine rich domains. This domain is often found at the C-terminus of tandem leucine rich repeats. |
| CD-Length = 51 residues, 98.0% aligned |
| Score = 43.1 bits (100), Expect = 4e-05 |

Loss of heterozygosity for 10q23-26 is seen in over 80% of glioblastoma multiforme tumors. Positional cloning was used to isolate the LGI1 (Leucine-rich gene-Glioma Inactivated) gene, which is rearranged as a result of the t(10;19)(q24;q13) balanced translocation in the T98G glioblastoma cell line lacking any normal chromosome 10 (See Chemova et al., Oncogene 17: 2873-2881). Rearrangement of the LGI1 gene was also detected in the A172 glioblastoma cell line and several glioblastoma tumors. These rearrangements lead to a complete absence of LGI1 expression in glioblastoma cells. The LGI1 gene encodes a protein with a calculated molecular mass of 60 kD and contains 3.5 leucine-rich repeats (LRR) with conserved flanking sequences. In the LRR domain, LGI1 has the highest homology with a number of transmembrane and extracellular proteins which function as receptors and adhesion proteins. LGI1 is predominantly expressed in neural tissues, especially in brain; its expression is reduced in low grade brain tumors and it is significantly reduced or absent in malignant gliomas. Its localization to the 10q24 region, and rearrangements or inactivation in malignant brain tumors, suggest that LGI1 is a candidate tumor suppressor gene involved in progression of glial tumors.

The human leucine-rich glioma-inactivated protein precursor-like protein described in this invention is predicted to share the attributes of other family members and is thus implicated in regulation of cell growth and survival as well as cellular metabolism. Like the LGI1 gene, the leucine-rich glioma-inactivated protein precursor-like gene described in this patent is expressed in neural tissues; however, it also appears to be frequently expressed in parathyroid tumors. Therefore, this protein is an attractive target for drug intervention in the treatment of cancer, central nervous system disorders, and metabolic diseases, among others. The leucine-rich glioma-inactivated protein precursor-like gene maps to human chromosome 4p16 and is predicted to encode a secreted protein.

The disclosed NOV54 nucleic acid of the invention encoding a Leucine-rich glioma-inactivated protein precursor-like protein includes the nucleic acid whose sequence is provided in Table 54A or a fragment thereof. The invention also includes a mutant or variant nucleic acid any of whose bases may be changed from the corresponding base shown in Table 54A while still encoding a protein that maintains its Leucine-rich glioma-inactivated protein precursor-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 36 percent of the bases may be so changed.

The disclosed NOV54 protein of the invention includes the Leucine-rich glioma-inactivated protein precursor-like protein whose sequence is provided in Table 54B. The invention also includes a mutant or variant protein any of whose residues may be changed from the corresponding residue shown in Table 54B while still encoding a protein that maintains its Leucine-rich glioma-inactivated protein precursor-like activities and physiological functions, or a functional fragment thereof. In the mutant or variant protein, up to about 45 percent of the residues may be so changed.

The invention further encompasses antibodies and antibody fragments, such as F_{ab} or (F_{ab})₂, that bind immunospecifically to any of the proteins of the invention.

The above defined information for this invention suggests that this Leucine-rich glioma-inactivated protein precursor-like protein (NOV54) may function as a member of a "Leucine-rich glioma-inactivated protein precursor family". Therefore, the NOV54 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration in vivo and *in vitro* of all tissues and cell types composing (but not limited to) those defined here.

The NOV54 nucleic acids and proteins of the invention are useful in potential therapeutic applications implicated in diseases including but not limited to various pathologies and disorders as indicated below. For example, a cDNA encoding the Leucine-rich glioma-inactivated protein precursor-like protein (NOV54) may be useful in gene therapy, and the Leucine-rich glioma-inactivated protein precursor-like protein (NOV54) may be useful when administered to a subject in need thereof. By way of nonlimiting example, the compositions of the present invention will have efficacy for treatment of patients suffering from cancer, trauma, bacterial and viral infections, in vitro and in vivo regeneration, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, anemia, bleeding disorders, scleroderma, transplantation, hyperparathyroidism, hypoparathyroidism, diabetes, autoimmune disease, renal artery stenosis, interstitial nephritis, glomerulonephritis, polycystic kidney disease, systemic lupus erythematosus, renal tubular acidosis, IgA nephropathy, Lesch-Nyhan syndrome, Hirschsprung's disease, Crohn's Disease, appendicitis, endometriosis, fertility, cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, obesity, transplantation, and pancreatitis, or other pathologies or conditions. The NOV54 nucleic acid encoding the Leucine-rich glioma-inactivated protein precursor-like protein of the invention, or fragments thereof, may further be useful in diagnostic applications, wherein the presence or amount of the nucleic acid or the protein are to be assessed.

NOV54 nucleic acids and polypeptides are further useful in the generation of antibodies that bind immuno-specifically to the novel NOV54 substances for use in therapeutic or diagnostic methods. These antibodies may be generated according to methods known in the art, using prediction from hydrophobicity charts, as described in the "Anti-NOVX Antibodies" section below. The disclosed NOV54 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV55

A disclosed NOV55 nucleic acid of 2886 nucleotides (also referred to as CG57306-01) encoding an anion exchanger-like protein is shown in Table 55A. The start and stop codons are in bold letters.

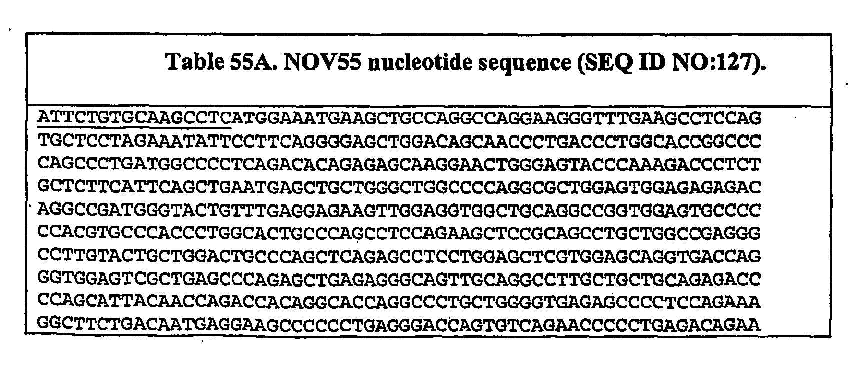

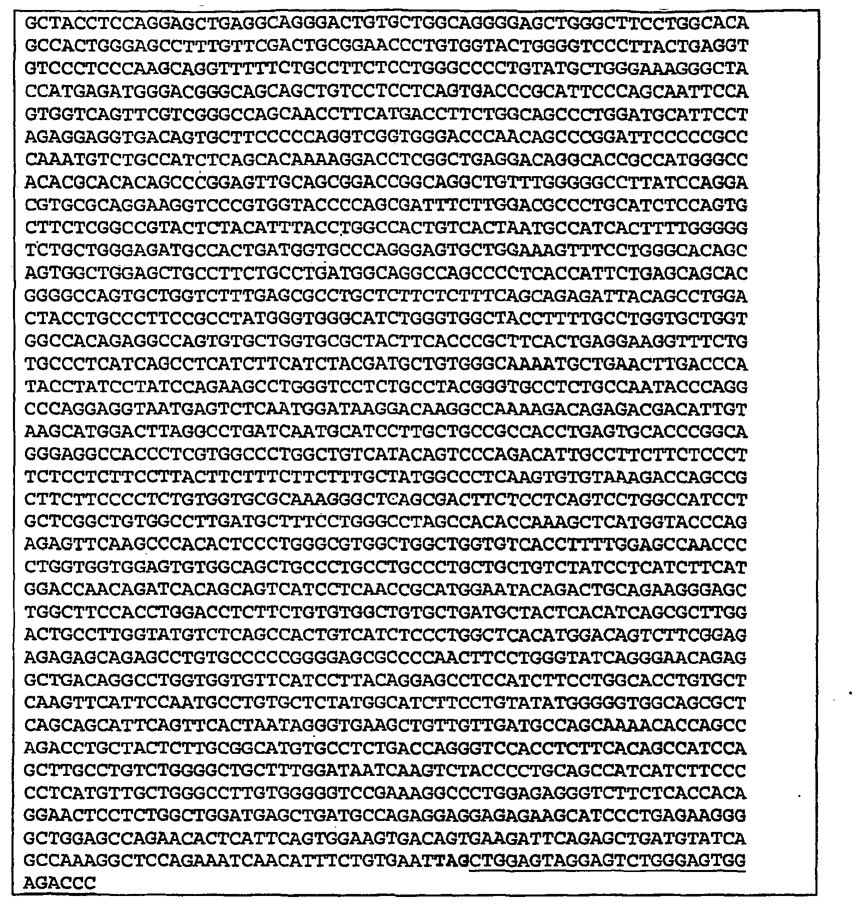

In a search of public sequence databases, the NOV nucleic acid sequence, located on chromsome 17 has 2250 of 2788 bases (80%) identical to a gb:GENBANK-ID:AB038264|acc:AB038264.1 mRNA from Oryctolagus cuniculus (Oryctolagus cuniculus AE4b mRNA for anion exchanger 4b, complete cds). Public nucleotide databases include all GenBank databases and the GeneSeq patent database.

The disclosed NOV55 polypeptide (SEQ ID NO:128) encoded by SEQ ID NO:127 has 946 amino acid residues and is presented in Table 55B using the one-letter amino acid code. Signal P, Psort and/or Hydropathy results predict that NOV55 has no signal peptide and is likely to be localized extracellularly with a certainty of 0.8000.

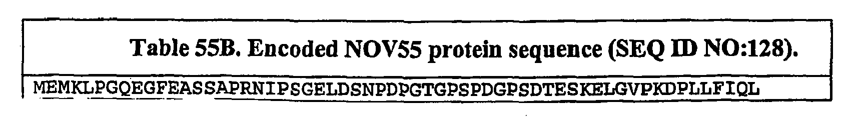

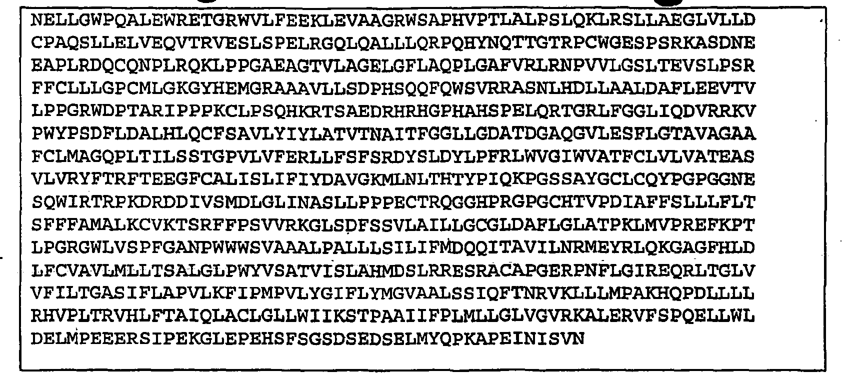

A search of sequence databases reveals that the NOV55 amino acid sequence has 827 of 944 amino acid residues (87%) identical to, and 873 of 944 amino acid residues (92%) similar to, the 939 amino acid residue ptnr:TREMELNEW-ACC:BAB18936 protein from Oryctolagus cuniculus (Rabbit) (ANION EXCHANGER 4B). Public amino acid databases include the GenBank databases, SwissProt, PDB and PIR.

NOV55 is expressed in at least kidney, testis. This information was derived by determining the tissue sources of the sequences that were included in the invention including but not limited to SeqCalling sources, Public EST sources, Literature sources, and/or RACE sources.

The disclosed NOV55 polypeptide has homology to the amino acid sequences shown in the BLASTP data listed in Table 55C.

| **Table 55C. BLAST results for NOV55** | | | | | |
|---|---|---|---|---|---|
| Gene Index/ Identifier | Protein/ Organism | Length (aa) | Identity (%) | Positives (%) | Expect |
| gi \| 18560973 \| ref \| Xp 038736.2 \| (XM_038736) | solute carrier family 4, sodium bicarbonate cotransporter, member 9 [Homo sapiens] | 959 | 938/962 (97%) | 940/962 (97%) | 0.0 |
| gi \| 14582760 \| gb \| AAK6 9625.1 \| AF332961 1 (AF332961) | anion exchanger AE4 [Homo sapiens | 959 | 937/962 (97%) | 939/962 (97%) | 0.0 |
| gi\|7363254\|dbj\|BAA9 3010.1 (AB032762) | sodium bicarbonate cotransporter 5 [Homo sapiens | 957 | 936/960 (97%) | 938/960 (97%) | 0.0 |
| gi\|1351750B\|gb\|AAK2 8832.1\|AF313465 1 (AF313465) | sodium bicarbonate cotransporter [Homo sapiens] | 990 | 938/986 (95%) | 940/986 (95%) | 0.0 |
| gi\|13249295\|qb\|AAK1 6733.1\|AF336237 1 (AF336237) | anion exchanger AE4 [Homo sapiens] | 945 | 922/962 (95%) | 926/962 (95%) | 0.0 |

Table 55D lists the domain descriptions from DOMAIN analysis results against NOV55. This indicates that the NOV55 sequence has properties similar to those of other proteins known to contain this domain.

| **Table 55D. Domain Analysis of NOV55** |
|---|
| gnl\|Pfam\|pfam00955, HCO3_cotransp,HCO3- transporter family. This family contains Band 3 anion exchange proteins that exchange CL-/HCO3-. This family also includes cotransporters of Na+/HCO3-. |
| CD-Length = 781 residues, 100.0% aligned |
| Score = 731 bits (1887), Expect = 0.0 |

The rabbit anion exchanger 4B protein is a member of the bicarbonate ion transporter superfamily and is present on the apical membrance of beta-intercalated cells in the collecting ducts of the rabbit kidney (See Tsuganezawa et al., J Biol Chem 2000 Dec 1). The rabbit protein has sodium-independent anion exchanger activity when expressed in cultured COS-7 cells and Xenopus oocytes.

The acid-secreting alpha intercalated cells and bicarbonate-secreting beta intercalated cells are sites for modulation of urinary acid secretion, which in turn governs acid-base homeostasis. Mutations in the red cell anion exchanger gene, for instance, are correlated with familial distal renal tubular acidosis (See Bruce et al., Biochem Cell Biol 1998; 76(5): 723-728).

The disclosed NOV55 nucleic acid of the invention encoding a anion exchanger-like protein includes the nucleic acid whose sequence is provided in Table 55A or a fragment thereof. The invention also includes a mutant or variant nucleic acid any of whose bases may be changed from the corresponding base shown in Table 55A while still encoding a protein that maintains its anion exchanger-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 20 percent of the bases may be so changed.

The disclosed NOV55 protein of the invention includes the anion exchanger-like protein whose sequence is provided in Table 55B. The invention also includes a mutant or variant protein any of whose residues may be changed from the corresponding residue shown in Table 55B while still encoding a protein that maintains its anion exchanger-like activities and physiological functions, or a functional fragment thereof. In the mutant or variant protein, up to about 13 percent of the residues may be so changed.

The invention further encompasses antibodies and antibody fragments, such as F_{ab} or (F_{ab})₂, that bind immunospecifically to any of the proteins of the invention.

The above defined information for this invention suggests that this anion exchanger-like protein (NOV55) may function as a member of a "anion exchanger family". Therefore, the NOV55 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV55 nucleic acids and proteins of the invention are useful in potential therapeutic applications implicated in diseases including but not limited to various pathologies and disorders as indicated below. For example, a cDNA encoding the anion exchanger-like protein (NOV55) may be useful in gene therapy, and the anion exchanger-like protein (NOV55) may be useful when administered to a subject in need thereof. By way of nonlimiting example, the compositions of the present invention will have efficacy for treatment of patients suffering from acidosis, alkalosis, diabetes, autoimmune disease, renal artery stenosis, interstitial nephritis, glomerulonephritis, polycystic kidney disease, systemic lupus erythematosus, renal tubular acidosis, IgA nephropathy, hypercalceimia, Lesch-Nyhan syndrome, cancer, tissue degeneration, bacterial/viral/parasitic infection, or other pathologies or conditions. The NOV55 nucleic acid encoding the anion exchanger-like protein of the invention, or fragments thereof, may further be useful in diagnostic applications, wherein the presence or amount of the nucleic acid or the protein are to be assessed.

NOV55 nucleic acids and polypeptides are further useful in the generation of antibodies that bind immuno-specifically to the novel NOV55 substances for use in therapeutic or diagnostic methods. These antibodies may be generated according to methods known in the art, using prediction from hydrophobicity charts, as described in the "Anti-NOVX Antibodies" section below. The disclosed NOV55 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV56

A disclosed NOV56 nucleic acid of 1083 nucleotides (also referred to as CG57348-01) encoding a PR_SET_domain protein-like protein is shown in Table 56A. The start and stop codons are in bold letters.

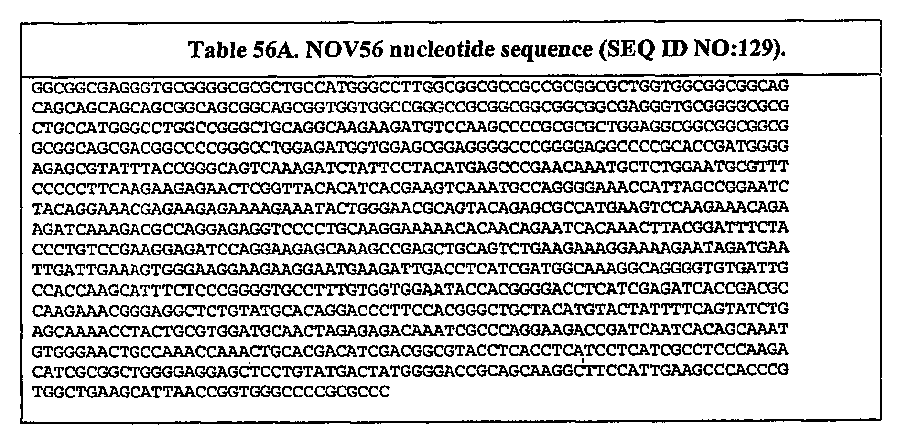

In a search of public sequence databases, the NOV56 nucleic acid sequence, located on chromsome 12 has 1036 of 1086 bases (95%) identical to a gb:GENBANK-ID:AF287261 |acc:AF287261.1 mRNA from Homo sapiens (Homo sapiens PR/SET domain containing protein 07 (SET07) mRNA, complete cds). Public nucleotide databases include all GenBank databases and the GeneSeq patent database.

The disclosed NOV56 polypeptide (SEQ ID NO:130) encoded by SEQ ID NO:129 has 345 amino acid residues and is presented in Table 56B using the one-letter amino acid code. Signal P, Psort and/or Hydropathy results predict that NOV56 has a signal peptide and is likely to be localized in the endoplasmic reticulum with a certainty of 0.600. The most likely cleavage site for a NOV56 peptide is between amino acids 22 and 23.

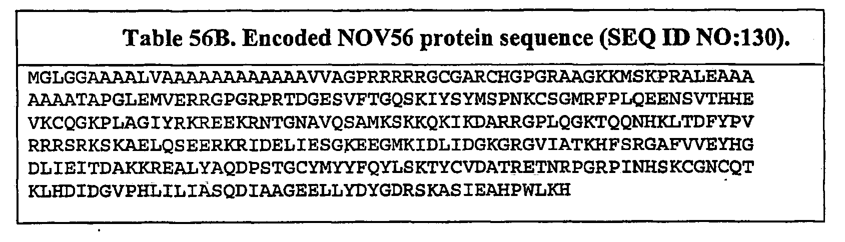

A search of sequence databases reveals that the NOV56 amino acid sequence has 314 of 345 amino acid residues (91%) identical to, and 322 of 345 amino acid residues (93%) similar to, the 345 amino acid residue ptnr:SPTREMBL-ACC:Q9NQR1 protein from Homo sapiens (Human) (PR/SET DOMAIN CONTAINING PROTEIN 07). Public amino acid databases include the GenBank databases, SwissProt, PDB and PIR.

NOV56 is expressed in at least Bone Marrow, Brain, Cervix, Dermis, Heart, Kidney, Liver, Lung, Lymph node, Pancreas, Parietal Lobe, Pituitary Gland, Placenta, Skin, Spinal Chord, Spleen, Thymus, Thyroid, Umbilical Vein,Adrenal Gland/Suprarenal gland, Aorta, Ascending Colon, Brain, Buccal mucosa, Cartilage, Cervix, Chorionic Villus, Colon, Coronary Artery, Duodenum, Heart, Kidney, Liver, Lung, Ovary, Parietal Lobe, Parotid Salivary glands, Peripheral Blood, Prostate, Retina, Salivary Glands, Small Intestine, Synovium/Synovial membrane, Testis, Tonsils, Umbilical Vein, and Urinary Bladder. Expression information was derived from the tissue sources of the sequences that were included in the derivation of the sequence of CG57348_01. The sequence is predicted to be expressed in the following tissues because of the expression pattern of (GENBANK-ID: gb:GENBANK-ID:AF287261|acc:AF287261.1) a closely related Homo sapiens PR/SET domain containing protein 07 (SET07) mRNA, complete cds homolog in species Homo sapiens :Bone Marrow, Brain, Cervix, Dermis, Heart, Kidney, Liver, Lung, Lymph node, Pancreas, Parietal Lobe, Pituitary Gland, Skin, and Colon. This information was derived by determining the tissue sources of the sequences that were included in the invention including but not limited to SeqCalling sources, Public EST sources, Literature sources, and/or RACE sources.

The disclosed NOV56 polypeptide has homology to the amino acid sequences shown in the BLASTP data listed in Table 56C.

| **Table 56C. BLAST results for NOV56** | | | | | |
|---|---|---|---|---|---|
| Gene Index/ Identifier | Protein/ Organism | Length (aa) | Identity (%) | Positives (%) | Expect |
| gi\|15295688\|ref\|XP 004668.4\| (XM_004668 | similar to PR/SET domain containing protein 07 (H. sapiens) [Homo sapiens] | 403 | 306/359 (85%) | 306/359 (85%) | e-149 |
| gi\|15042945 \|ref \|NP 065115.2\| (NM_020382) | PR/SET domain containing protein 07 [Homo sapiens] | 393 | 319/393 (81%) | 324/393 (82%) | e-139 |
| gi\|15303323\|ref\|XP 017300.2\| (XM_017300) | PR/SET domain containing protein 07 [Homo sapiens] | 240 | 145/165 (87%) | 150/165 (90%) | 3e-46 |
| gi\|7299871\|gb\|AAF55 047.1\| (AE003704) | CG3307 gene product [Drosophila melanogaster] | 689 | 100/230 (43%) | 143/230 (61%) | 2e-45 |
| gi\|17554790\|ref\|NP 498417.1\| (NM 066016) | T26A5.7.p [Caenorhabditis elegans] | 242 | 83/203 (40%) | 125/203 (60%) | 2e-33 |

Table 56D lists the domain descriptions from DOMAIN analysis results against NOV56. This indicates that the NOV56 sequence has properties similar to those of other proteins known to contain this domain.

| **Table 56D. Domain Analysis of NOV56** |
|---|
| gnl\|Smart\|smart00317, SET, SET (Su(var)3-9, Enhancer-of-zeste, Trithorax) domain; Putative methyl transferase, based on outlier plant homologues |
| CD-Length = 125 residues, 96.8% aligned |
| Score = 107 bits (266), Expect = 1e-24 |

Association of SET domain and myotubularin-related proteins modulates growth control. The PR domain of the Rb-binding zinc finger protein RIZ1 is a protein binding interface and is related to the SET domain functioning in chromatin-mediated gene expression.

SET domains appear to be protein-protein interaction domains. It has been demonstrated that SET domains mediate interactions with a family of proteins that display similarity with dual-specificity phosphatases (dsPTPases) [2]. A subset of SET domains have beencalled PR domains. These domains are divergent in sequence from other SET domains, but also appear to mediate protein-protein interaction [3].

The SET domain is a highly conserved, approximately 150-amino acid motif implicated in the modulation of chromatin structure. It was originally identified as part of a larger conserved region present in the Drosophila Trithorax protein and was subsequently identified in the Drosophila Su(var)3-9 and 'Enhancer of zeste' proteins, from which the acronym SET is derived. Studies have suggested that the SET domain may be a signature of proteins that modulate transcriptionally active or repressed chromatin states through chromatin remodeling activities.

By sequencing cDNAs randomly selected from a cDNA library derived from a human immature myeloid cell line, Nomura et al. (1994) isolated a cDNA encoding SETDB1, which they called KIAA0067. The deduced SETDB1 protein has 1,291 amino acids. Northern blot analysis detected SETDB 1 expression in all 16 human tissues examined.

In the course of searching sequence databases for proteins containing SET domains, Harte et al. (1999) identified the SETDB 1 sequence. They determined that SETDB 1 has a C-terminal SET domain that is well-conserved except that it contains a 347-amino acid insertion between its most highly conserved regions. The authors found that the C. elegans YNCA gene product is highly similar to SETDB 1 and also contains a bifurcated SET domain.

Nomura et al. (1994) mapped the SETDB1 gene to chromosome 1 using a somatic cell hybrid mapping panel. By FISH and radiation hybrid mapping, Harte et al. (1999) mapped the SETDB 1 gene to 1q21.

The disclosed NOV56 nucleic acid of the invention encoding a PR_SET_domain protein-like protein includes the nucleic acid whose sequence is provided in Table 56A or a fragment thereof. The invention also includes a mutant or variant nucleic acid any of whose bases may be changed from the corresponding base shown in Table 56A while still encoding a protein that maintains its PR_SET_domain protein-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV56 protein of the invention includes the PR_SET_domain protein-like protein whose sequence is provided in Table 56B. The invention also includes a mutant or variant protein any of whose residues may be changed from the corresponding residue shown in Table 56B while still encoding a protein that maintains its PR_SET_domain protein-like activities and physiological functions, or a functional fragment thereof. In the mutant or variant protein, up to about 9 percent of the residues may be so changed.

The invention further encompasses antibodies and antibody fragments, such as F_{ab} or (F_{ab})₂, that bind immunospecifically to any of the proteins of the invention.

The above defined information for this invention suggests that this PR_SET_domain protein-like protein (NOV56) may function as a member of a "PR_SET_domain protein family". Therefore, the NOV56 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV56 nucleic acids and proteins of the invention are useful in potential therapeutic applications implicated in diseases including but not limited to various pathologies and disorders as indicated below. For example, a cDNA encoding the PR_SET_domain protein-like protein (NOV56) may be useful in gene therapy, and the PR_SET_domain protein-like protein (NOV56) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV56 nucleic acid encoding the PR_SET_domain protein-like protein of the invention, or fragments thereof, may further be useful in diagnostic applications, wherein the presence or amount of the nucleic acid or the protein are to be assessed.

NOV56 nucleic acids and polypeptides are further useful in the generation of antibodies that bind immuno-specifically to the novel NOV56 substances for use in therapeutic or diagnostic methods. These antibodies may be generated according to methods known in the art, using prediction from hydrophobicity charts, as described in the "Anti-NOVX Antibodies" section below. The disclosed NOV56 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV57

A disclosed NOV57 nucleic acid of 5896 nucleotides (also referred to as CG57650-01) encoding a non-muscle myosin heavy chain B-like protein is shown in Table 57A. The start and stop codons are in bold letters.

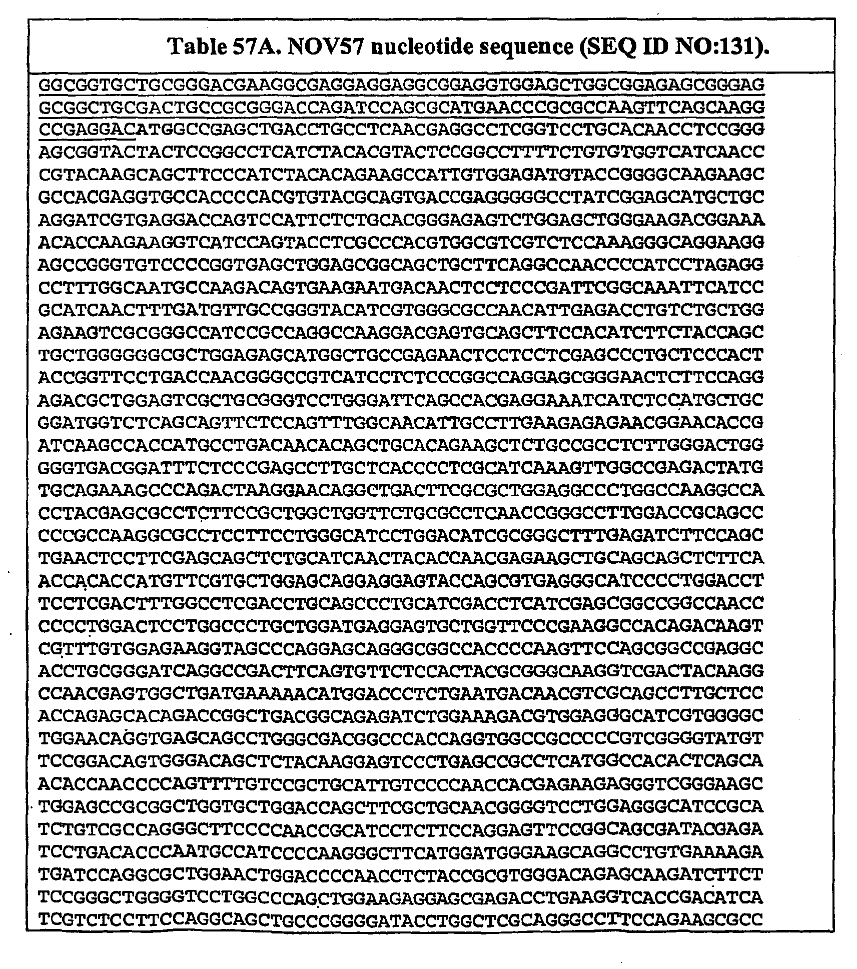

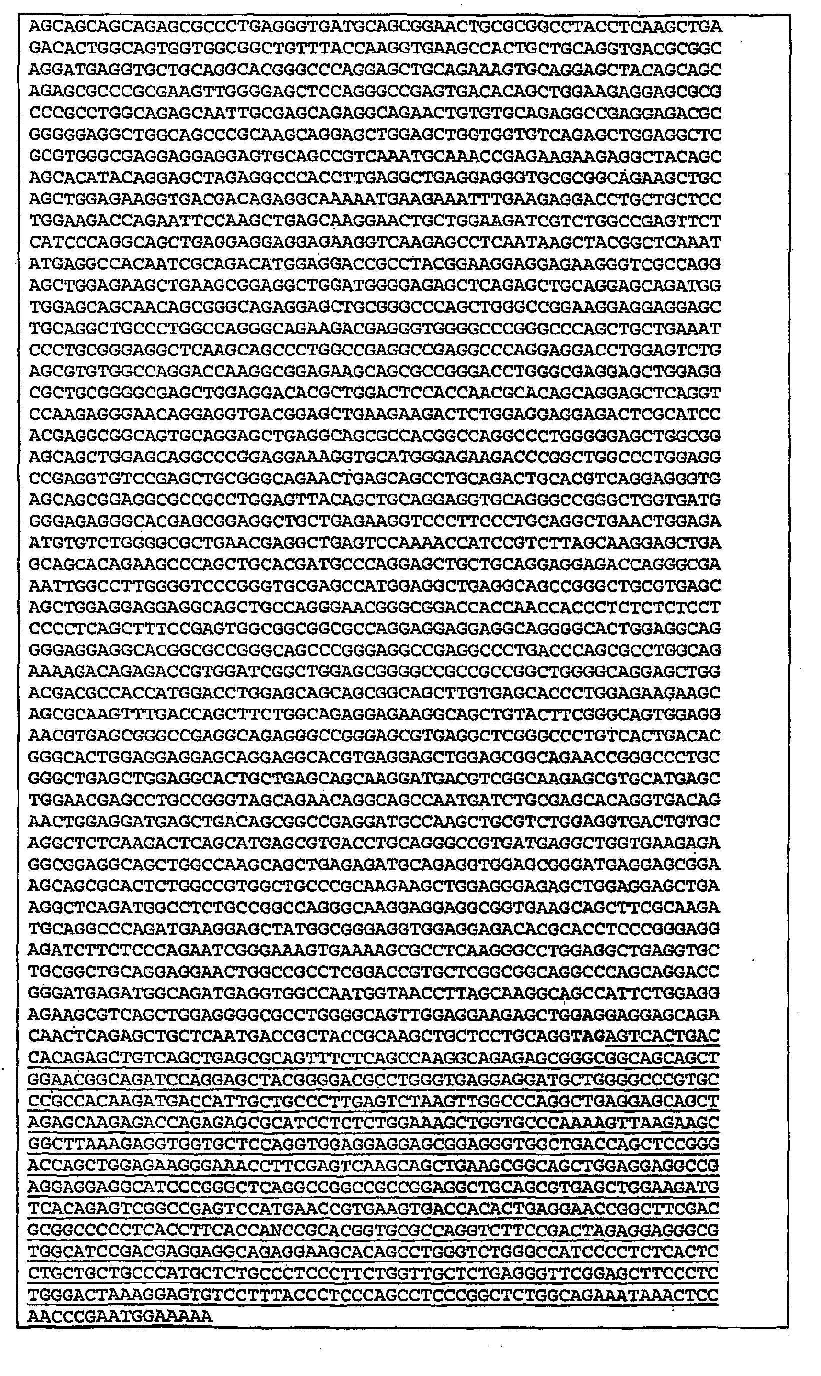

In a search of public sequence databases, the NOV57 nucleic acid sequence, located on chromsome 10 has 3509 of 4934 bases (71%) identical to a gb:GENBANK-ID:RABMHCP|acc:M77812.1 mRNA from Oryctolagus cuniculus (Rabbit myosin heavy chain mRNA, complete cds). Public nucleotide databases include all GenBank databases and the GeneSeq patent database.

The disclosed NOV57 polypeptide (SEQ ID NO: 132) encoded by SEQ ID NO: 131 has 1673 amino acid residues and is presented in Table 57B using the one-letter amino acid code. Signal P; Psort and/or Hydropathy results predict that NOV57 has no signal peptide and is likely to be localized in the nucleus with a certainty of 0.9600.

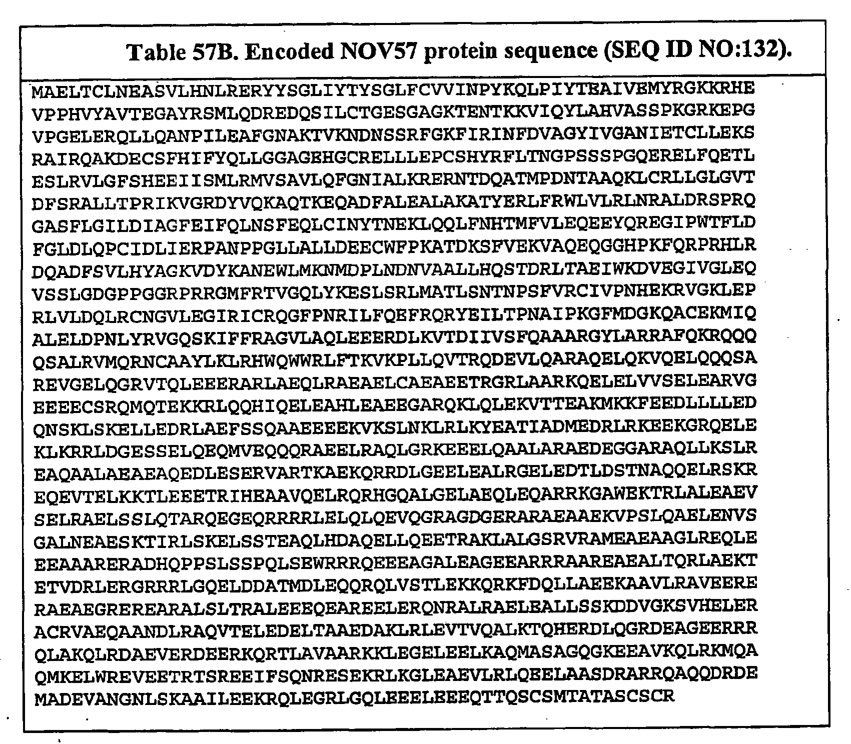

A search of sequence databases reveals that the NOV57 amino acid sequence has 1149 of 1661 amino acid residues (69%) identical to, and 1395 of 1661 amino acid residues (83%) similar to, the 1976 amino acid residue ptnr:SWISSNEW-ACC:P35580 protein from Homo sapiens (Human) (MYOSIN HEAVY CHAIN, NONMUSCLE TYPE B (CELLULAR MYOSIN HEAVY CHAIN, TYPE B) (NMMHC-B))(.Public amino acid databases include the GenBank databases, SwissProt, PDB and PIR.

NOV57 is expressed in at least adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea and uterus. This information was derived by determining the tissue sources of the sequences that were included in the invention including but not limited to SeqCalling sources, Public EST sources, Literature sources, and/or RACE sources:

The disclosed NOV57 polypeptide has homology to the amino acid sequences shown in the BLASTP data listed in Table 57C .

| **Table 57C. BLAST results for NOV57** | | | | | |
|---|---|---|---|---|---|
| Gene Index/ Identifier | Protein/ Organism | Length (aa) | Identity (%) | Positives (%) | Expect |
| gi\|13928704\|ref\|NP 113708.11\| (NM_031520) | myosin heavy chain 11 [Rattus norvegicus] | 1976 | 1144/1664 (68%) | 1389/1664 (82%) | 0.0 |
| gi\|212449\|gb\|AAA489 85.1\| (M93676) | nonmuscle myosin heavy chain [Gallus gallus] | 1976 | 1148/1664 (68%) | 1395/1664 (82%) | 0.0 |
| gi\|1346640\|sp\|P3558 O\|MYHA HUMAN | Myosin heavy chain, nonmuscle type B (Cellular myosin heavy chain, type B) (Nonmuscle myosin heavy chain-B) (NMMHC-B) | 1976 | 1149/1664 (69%) | 1395/1664 (83%) | 0.0 |
| gi\|212451\|gb\|AAA489 87.1\| (M93676) | nonmuscle myosin heavy chain [Gallus gallus | 1997 | 1148/1685 (68%) | 1395/1685 (82%) | 0.0 |
| gi\|212450\|gb\|AAA489 86.1\| (M93676) | nonmuscle myosin heavy chain [Gallus gallus] | 1986 | 1148/1674 (68%) | 1395/1674 (82%) | 0.0 |

Tables 57D-H list the domain descriptions from DOMAIN analysis results against NOV57. This indicates that the NOV57 sequence has properties similar to those of other proteins known to contain this domain.

| **Table 57D. Domain Analysis of NOV57** |
|---|
| gn1\|Pfam\|pfam00063, myosin_head, Myosin head (motor domain). |
| CD-Length = 670 residues, 99.6% aligned |
| Score = 922 bits (2384), Expect = 0.0 |

| **Table 57E. Domain Analysis of NOV57** |
|---|
| gnl\|Smart\|smart00242, MYSc, Myosin. Large ATPases.; ATPase; molecular motor. Muscle contraction consists of a cyclical interaction between myosin and actin. The core of the myosin structure is similar in fold to that of kinesin. |
| CD-Length = 688 residues, 98.4% aligned |
| Score = 866 bits (2238), Expect = 0.0 |

| **Table 57F. Domain Analysis of NOV57** | | |
|---|---|---|
| gnl\|Pfam\|pfam01576, Myosin_tail, Myosin tail. The myosin molecule is a multi-subunit complex made up of two heavy chains and four light chains it is a fundamental contractile protein found in all eukaryote cell types. This family consists of the coiled-coil myosin heavy chain tail region. The coiled-coil is composed of the tail from two molecules of myosin. These can then assemble into the macromolecular thick filament. The coiled-coil region provides the structural backbone the thick filament. | | |
| | CD-Length = 860 residues, 78.4% aligned | |
| | | Score = 344 bits (882), Expect = 3e-95 |

| **Table 57G. Domain Analysis of NOV57** |
|---|
| gnl\|Pfam\|pfam01496, V_ATPase_sub_a, V-type ATPase 116kDa subunit family. This family consists of the 116kDa V-type ATPase (vacuolar (H+)-ATPases) subunits, as well as V-type ATP synthase subunit i. The V-type ATPases family are proton pumps that acidify intracellular compartments in eukaryotic cells for example yeast central vacuoles, clathrin-coated and synaptic vesicles. They have important roles in membrane trafficking processes. The 116kDa subunit (subunit a) in the V-type ATPase is part of the V0 functional domain responsible for proton transport. The a subunit is a transmembrane glycoprotein with multiple putative transmembrane helices it has a hydrophilic amino terminal and a hydrophobic carboxy terminal. It has roles in proton transport and assembly of the V-type ATPase complex. This subunit is encoded by two homologous gene in yeast VPH1 and STV1. |
| CD-Length = 703 residues, 34.3% aligned |
| Score = 57.0 bits (136), Expect = 8e-09 |

| **Table 57H. Domain Analysis of NOV57** |
|---|
| gnl\|Pfam\|pfam00769, ERM, Ezrin/radixin/moesin family. This family of proteins contain a band 4.1 domain (pfam00373), at their amino terminus. This family represents the rest of these proteins. |
| CD-Length = 365 residues, 43.6% aligned |
| Score = 52.0 bits (123), Expect = 3e-07 |

Myosins constitute a large superfamily of actin-dependent molecular motors. Phylogenetic analysis currently places myosins into 15 classes. The conventional myosins which form filaments in muscle and non-muscle cells form class II. There has been extensive characterization of these myosins and much is known about their function. With the exception of class I and class V myosins, little is known about the structure, enzymatic properties, intracellular localization and physiology of most unconventional myosin classes. (See Sellers JR, 2000, Biochim Biophys Acta 1496:3-22). The discovery in of a huge diversity within the myosin superfamily has been coupled with an understanding of the role of these motor proteins in various cellular functions. Extensive studies have revealed that myosin isoforms are not only involved in muscle contraction but also in crucial functions of many specialized mammalian cells such as melanocytes, kidney and intestinal brush border microvilli, nerve growth cones or inner ear hair cells. A search for genes involved in the pathology of human genetic deafness resulted in identification of three novel myosins: myosin VI, myosin VIIIA and, very recently, myosin XV. Recently, mutations have been detected within these genes that have been found to affect the hearing process (See Redowicz MJ, 1999, J Muscle Res Cell Motil 20:241-8). Class II non-muscle myosins are implicated in diverse biological processes such as cytokinesis, cellularization, cell shape changes and gastrulation. Two distinct non-muscle myosin heavy chain genes have been reported in all vertebrates: non-muscle myosin heavy chain-A (NMHC-A) and -B (NMHC-B). Whole mount in situ hybridization with tailbud stage embryos of Xenopus showed that NMHC-A mRNA is predominantly expressed in the epidermis, whereas NMHC-B mRNA is expressed in the somites, brain, eyes and branchial arches. Interestingly, the expression of NMHC-B in developing somites was gradually restricted to the center of each somite as differentiation proceeds. DAPI nuclear staining demonstrated that NMHC-B mRNA is colocalized with the nuclei or perinuclear area. In animal cap experiments, treatment with activin A or ectopic expression of Xbra and an activated form of Xlim1 markedly up-regulates NMHC-B as well as muscle actin mRNAs and slightly down-regulates NMHC-A mRNA, consistent with NMHC-B expression in the somitic muscle and NMHC-A expression in the epidermis. (See Bhatia et al., 1998, Mech Dev 78:33-6).

The disclosed NOV57 nucleic acid of the invention encoding a non-muscle myosin heavy chain B-like protein includes the nucleic acid whose sequence is provided in Table 57A or a fragment thereof. The invention also includes a mutant or variant nucleic acid any of whose bases may be changed from the corresponding base shown in Table 57A while still encoding a protein that maintains its non-muscle myosin heavy chain B-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 29 percent of the bases may be so changed.

The disclosed NOV57 protein of the invention includes the non-muscle myosin heavy chain B-like protein whose sequence is provided in Table 57B. The invention also includes a mutant or variant protein any of whose residues may be changed from the corresponding residue shown in Table 57B while still encoding a protein that maintains its non-muscle myosin heavy chain B-like activities and physiological functions, or a functional fragment thereof. In the mutant or variant protein, up to about 31 percent of the residues may be so changed.

The invention further encompasses antibodies and antibody fragments, such as F_{ab} or (F_{ab})₂, that bind immunospecifically to any of the proteins of the invention.

The above defined information for this invention suggests that this non-muscle myosin heavy chain B-like protein (NOV57) may function as a member of a "non-muscle myosin heavy chain B family". Therefore, the NOV57 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV57 nucleic acids and proteins of the invention are useful in potential therapeutic applications implicated in diseases including but not limited to various pathologies and disorders as indicated below. For example, a cDNA encoding the non-muscle myosin heavy chain B-like protein (NOV57) may be useful in gene therapy, and the non-muscle myosin heavy chain B-like protein (NOV57) may be useful when administered to a subject in need thereof. By way of nonlimiting example, the compositions of the present invention will have efficacy for treatment of patients suffering from hypertension and vasospasm of the coronary and cerebral arteries, coronary artery spasm, artherosclerosis, hypertrophic cardiomyopathy, inflammatory diseases such as asthma, cancer, or other pathologies or conditions. The NOV57 nucleic acid encoding the non-muscle myosin heavy chain B-like protein of the invention, or fragments thereof, may further be useful in diagnostic applications, wherein the presence or amount of the nucleic acid or the protein are to be assessed.

NOV57 nucleic acids and polypeptides are further useful in the generation of antibodies that bind immuno-specifically to the novel NOV57 substances for use in therapeutic or diagnostic methods. These antibodies may be generated according to methods known in the art, using prediction from hydrophobicity charts, as described in the "Anti-NOVX Antibodies" section below. The disclosed NOV57 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV58

A disclosed NOV58 nucleic acid of 688 nucleotides (also referred to as CG57766-01) encoding a plasma retinol binding protein-like protein is shown in Table 58A. The start and stop codons are in bold letters.

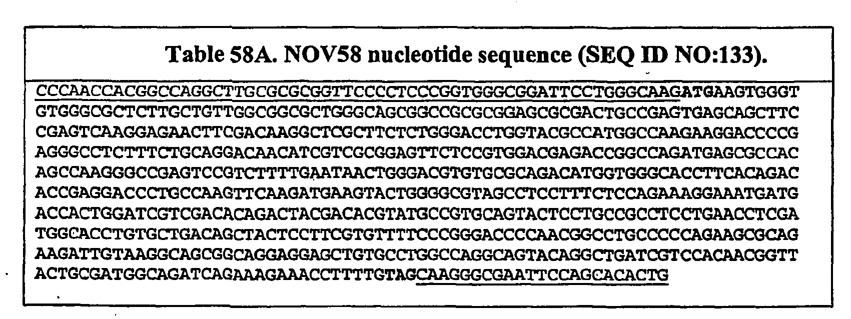

In a search of public sequence databases, the NOV58 nucleic acid sequence, located on chromsome 1Oq23-24 has 657 of 673 bases (97%) identical to a gb:GENBANK-ID:HSRBP1|acc:X00129.1 mRNA from Homo sapiens (Human mRNA for retinol binding protein (RBP)). Public nucleotide databases include all GenBank databases and the GeneSeq patent database.

The disclosed NOV58 polypeptide (SEQ ID N0:134) encoded by SEQ ID N0:133 has 201 amino acid residues and is presented in Table 58B using the one-letter amino acid code. Signal P, Psort and/or Hydropathy results predict that NOV58 has a signal peptide and is likely to be localized extracellularly with a certainty of 0.3700. The most likely cleavage site for a NOV58 peptide is between amino acids 18 and 19.

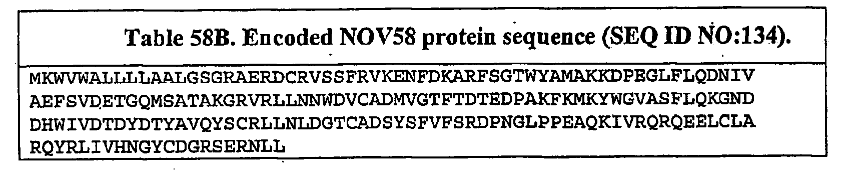

A search of sequence databases reveals that the NOV58 amino acid sequence has 196 of 201 amino acid residues (97%) identical to, and 197 of 201 amino acid residues (98%) similar to, the 199 amino acid residue ptnr:SWISSNEW-ACC:P02753 protein from Homo sapiens (Human) (PLASMA RETINOL-BINDING PROTEIN PRECURSOR (PRBP) (RBP)). Public amino acid databases include the GenBank databases, SwissProt, PDB and PIR.

NOV58 is expressed in at least adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea and uterus. This information was derived by determining the tissue sources of the sequences that were included in the invention including but not limited to SeqCalling sources, Public EST sources, Literature sources, and/or RACE sources.

The disclosed NOV58 polypeptide has homology to the amino acid sequences shown in the BLASTP data listed in Table 58C.

| **Table 58C. BLAST results for NOV58** | | | | | |
|---|---|---|---|---|---|
| Gene Index/ Identifier | Protein/ Organism | Length (aa) | Identity (%) | Positives (%) | Expect |
| gi\|18088326\|gb\|AAH2 0633.1\|AAH20633 (BC020633 | Similar to retinol binding protein 4, plasma [Homo sapiens] | 201 | 200/201 (99%) | 200/201 (99%) | e-113 |
| gi\|2136468\|pir\|\|146 257 | retinol binding protein precursor - horse | 201 | 186/201 (92%) | 195/201 (96%) | e-107 |
| gi\|3041715\|sp\|P2748 5\|RETB PIG | PLASMA RETINOL-BINDING PROTEIN PRECURSOR (PRBP) (RBP) | 201 | 184/201 (91%) | 193/201 (95%) | e-106 |
| gi\|1710096\|sp\|P0691 2\|RETB RAHIT | PLASMA RETINOL-BINDING PROTEIN PRECURSOR (PRBP) (RBP) | 201 | 183/201 (91%) | 194/201 (96%) | e-106 |
| gi\|89271\|pir\|\|A3948 6 | plasma retinol-binding protein precursor - pig | 201 | 184/201 (91%) | 192/201 (94%) | e-106 |

Table 58D lists the domain descriptions from DOMAIN analysis results against NOV58. This indicates that the NOV58 sequence has properties similar to those of other proteins known to contain this domain.

| **Table 58D. Domain Analysis of NOV58** |
|---|
| gnl\|Pfam\|pfam00061, lipocalin, Lipocalin / cytosolic fatty-acid binding protein family. Lipocalins are transporters for small hydrophobic molecules, such as lipids, steroid hormones, bilins, and retinoids. Alignment subsumes both the lipocalin and fatty acid binding protein signatures from PROSITE. This is supported on structural and functional grounds. Structure is an eight-stranded beta barrel. |
| CD-Length = 145 residues, 100.0% aligned |
| Score = 102 bits (255), Expect = 2e-23 |

Vitamin A is mobilized from liver stores and transported in plasma in the form of the lipid alcohol retinol, bound to a specific transport protein, retinol-binding protein (RBP). A great deal is known about the chemical structure, metabolism, and biological roles of RBP. RBP is a single polypeptide chain with molecular weight close to 20,000. RBP interacts strongly with plasma prealbumin, and normally circulates in plasma as a 1:1 molar RBP-prealbumin complex. Both the primary and the tertiary structure of prealbumin are known, and the primary structure of RBP has recently been reported. Much information is available about the protein-protein and protein-ligand interactions that are involved in this transport system. Many clinical studies have examined the effects of a variety of diseases on the plasma levels of RBP and prealbumin in humans. Plasma RBP levels are low in patients with liver disease and are high in patients with chronic renal disease. These findings reflect the facts that RBP is produced in the liver and mainly catabolized in the kidneys. Delivery of retinol to extra-hepatic tissues appears to involve specific cell surface receptors for RBP. Vitamin A mobilization from the liver, and delivery to peripheral tissues, is highly regulated by factors that control the rates of RBP production and secretion. Retinol deficiency specifically blocks the secretion of RBP, so that plasma RBP levels fall and liver RBP levels rise. Injection of retinol into vitamin A-deficient rats stimulates the rapid secretion of RBP from the liver into the plasma (See Goodman D.S. 1980. Ann N Y Acad Sci 348:378-90).

The disclosed NOV58 nucleic acid of the invention encoding a plasma retinol binding protein-like protein includes the nucleic acid whose sequence is provided in Table 58A or a fragment thereof. The invention also includes a mutant or variant nucleic acid any of whose bases may be changed from the corresponding base shown in Table 58A while still encoding a protein that maintains its plasma retinol binding protein-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up t_{Q} about 3 percent of the bases may be so changed.

The disclosed NOV58 protein of the invention includes the plasma retinol binding protein-like protein whose sequence is provided in Table 58B. The invention also includes a mutant or variant protein any of whose residues may be changed from the corresponding residue shown in Table 58B while still encoding a protein that.maintains its plasma retinol binding protein-like activities and physiological functions, or a functional fragment thereof. In the mutant or variant protein, up to about 3 percent of the residues may be so changed.

The invention further encompasses antibodies and antibody fragments, such as F_{ab} or (F_{ab})₂, that bind immunospecifically to any of the proteins of the invention.

The above defined information for this invention suggests that this plasma retinol binding protein-like protein (NOV58) may function as a member of a "plasma retinol binding protein family". Therefore, the NOV58 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV58 nucleic acids and proteins of the invention are useful in potential therapeutic applications implicated in diseases including but not limited to various pathologies and disorders as indicated below. For example, a cDNA encoding the plasma retinol binding protein-like protein (NOV58) may be useful in gene therapy, and the plasma retinol binding protein-like protein (NOV58) may be useful when administered to a subject in need thereof. By way of nonlimiting example, the compositions of the present invention will have efficacy for treatment of patients suffering from cervical dysplasias and cancer, breast cancer, phenylketonuria, liver diseases, kidney diseases, alzheimers, infection and inflammations, or other pathologies or conditions. The NOV58 nucleic acid encoding the plasma retinol binding protein-like protein of the invention, or fragments thereof, may further be useful in diagnostic applications, wherein the presence or amount of the nucleic acid or the protein are to be assessed.

NOV58 nucleic acids and polypeptides are further useful in the generation of antibodies that bind immuno-specifically to the novel NOV58 substances for use in therapeutic or diagnostic methods. These antibodies may be generated according to methods known in the art, using prediction from hydrophobicity charts, as described in the "Anti-NOVX Antibodies" section below. The disclosed NOV58 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV59

A disclosed NOV59 nucleic acid of 1647 nucleotides (also referred to as CG57566-01) encoding a HIV-1 inducer of short transcripts binding protein like protein-like protein is shown in Table 59A. The start and stop codons are in bold letters.

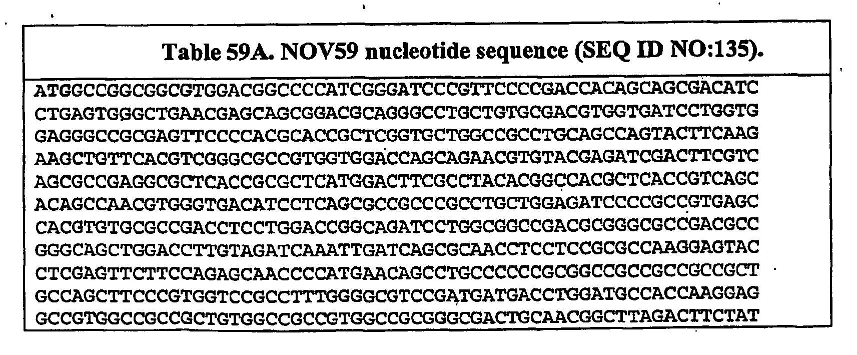

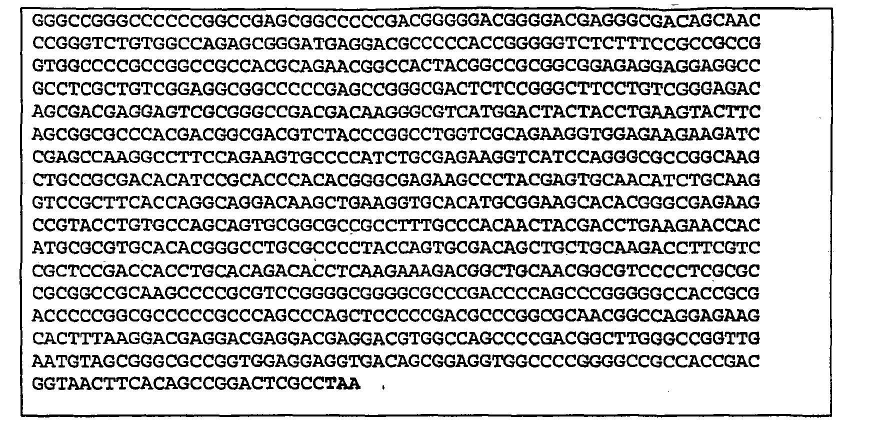

In a search of public sequence databases, the NOV59 nucleic acid sequence has 1271 of 1560 bases (81%) identical to a gb:GENBANK-ID:AF097916|acc:AF097916.1 mRNA from Homo sapiens (Homo sapiens HIV-1 inducer of short transcripts binding protein (FBI1) mRNA, complete cds). Public nucleotide databases include all GenBank databases and the GeneSeq patent database.

The disclosed NOV59 polypeptide (SEQ ID NO:136) encoded by SEQ ID NO:135 has 548 amino acid residues and is presented in Table 59B using the one-letter amino acid code. Signal P, Psort and/or Hydropathy results predict that NOV59 has no signal peptide and is likely to be localized in the nucleus with a certainty of 0.8800.

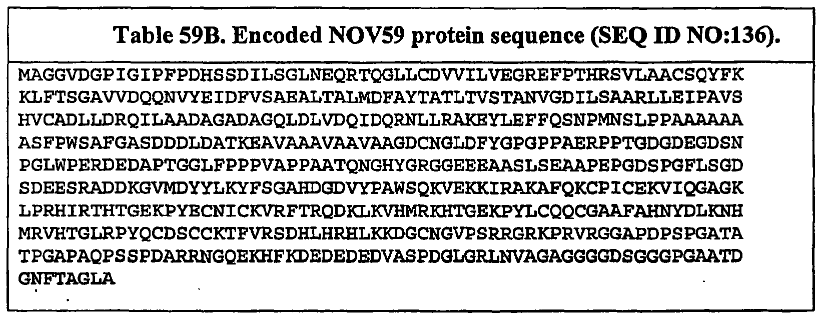

A search of sequence databases reveals that the NOV59 amino acid sequence has 344 of 458 amino acid residues (75%) identical to, and 362 of 458 amino acid residues (79%) similar to, the 584 amino acid residue ptnr:SPTREMBL-ACC:O95365 protein from Homo sapiens (Human) (HIV-1 INDUCER OF SHORT TRANSCRIPTS BINDING PROTEIN). Public amino acid databases include the GenBank databases, SwissProt, PDB and PIR.

NOV59 is expressed in at least tumor, inflammed, and brain. This information was derived by determining the tissue sources of the sequences that were included in the invention including but not limited to SeqCalling sources, Public EST sources, Literature sources, and/or RACE sources.

The disclosed NOV59 polypeptide has homology to the amino acid sequences shown in the BLASTP data listed in Table 59C.

| **Table 59C. BLAST results for NOV59** | | | | | |
|---|---|---|---|---|---|
| Gene Index/ Identifier | Protein/ Organism | Length (aa) | Identity (%) | Positives (%) | Expect |
| gi\|7705375\|ref \|NP 0 56982.1 (NM_015898) | HIV-1 inducer of short transcripts binding protein [Homo sapiens] | 584 | 548/584 (93%) | 548/584 (93%) | e-167 |
| gi\|16758916\|ref\|NP 446454.1 (NM_054002) | leukemia/lymphoma related factor [Rattus norvegicus] | 569 | 477/579 (82%) | 489/579 (84%) | e-159 |
| gi\|6754572\|ref\|NP_0 34861.1\| (NM 010731) | leukemia/lymphoma related factor [Mus musculus] | 565 | 479/579 (82%) | 488/579 (83%) | e-125 |
| gi\|3599513\|gb\|AAC35 368.1\| (AF086831) | leukemia/lymohoma related factor cLRF [Gallus gallus] | 546 | 359/578 (62%) | 400/578 (69%) | 4e-13 |
| gi\|2145062\|gb\|AAB58 414.1 (AF000561) | TTF-I interacting peptide 21; TIP21; Transcription Termination Factor I Interacting Peptide 21 [Homo sapiens] | 590 | 293/297 (98%) | 295/297 (98%) | 4e-12 |

Tables 59D-E list the domain descriptions from DOMAIN analysis results against NOV59. This indicates that the NOV59 sequence has properties similar to those of other proteins known to contain this domain.

| **Table 59D. Domain Analysis of NOV59** | |
|---|---|
| gnl\|Pfam\|pfam00651, BTB, BTB/POZ domain. The BTB (for BR-C, ttk and bab) or POZ (for Pox virus and Zinc finger) domain is present near the N-terminus of a fraction of zinc finger (pfam00096) proteins and in proteins that contain the pfam01344 motif such as Kelch and a family of pox virus proteins. The BTB/POZ domain mediates homomeric dimerisation and in some instances heteromeric dimerisation. The structure of the dimerised PLZF BTB/POZ domain has been solved and consists of a tightly intertwined homodimer. The central scaffolding of the protein is made up of a cluster of alpha-helices flanked by short beta-sheets at both the top and bottom of the molecule. POZ domains from several zinc finger proteins have been shown to mediate transcriptional repression and to interact with components of histone deacetylase co-repressor complexes including N-CoR and SMRT. The POZ or BTB domain is also known as BR-C/Ttk or ZiN | |
| | CD-Length = 114 residues, 100.0% aligned |
| | Score = 122 bits (305), Expect = 7e-29 |

| **Table 59E. Domain Analysis of NOV59** | | |
|---|---|---|
| gnl\|Pfam\|pfam00096, zf-C2H2, Zinc finger, C2H2 type. The C2H2 zinc finger is the classical zinc finger domain. The two conserved cysteines and histidines co-ordinate a zinc ion. The following pattern describes the zinc finger. #-X-C-X(1-5)-C-X3-#-X5-#-X2-H-X(3-6)-[H/C] Where X can be any amino acid, and numbers in brackets indicate the number of residues. The positions marked # are those that are important for the stable fold of the zinc finger. The final position can be either his or cys. The C2H2 zinc finger is composed of two short beta strands followed by an alpha helix. The amino terminal part of the helix binds the major groove in DNA binding zinc fingers. | | |
| | CD-Length = 23 residues, 100.0% aligned | |
| | | Score = 38.5 bits (88), Expect = 0.001 |

The HIV-1 promoter directs the synthesis of two classes of transcripts, short, non-polyadenylated transcripts and full-length, polyadenylated transcripts. The synthesis of these transcripts is activated by a bipartite DNA element, the inducer of short transcripts or IST, located downstream of the HIV-1 transcriptional start site, while the synthesis of full-length transcripts is activated by the viral activator Tat. Tat binds to the RNA element TAR, which is encoded largely between the two IST half-elements. Upon activation by Tat, the synthesis of short RNAs is repressed. A factor called FBI-1 (for factor that binds to IST) whose binding to wild-type and mutated ISTs correlated well with the abilities of these ISTs to direct the synthesis of short transcripts was identified by Morrison, et al (See Morrison et al., Nucleic Acids Res 1999 Mar 1: 1251-62). FBI-1 contains a POZ domain at its N-terminus and four Kruppel-type zinc fingers at its C-terminus. The C-terminus is sufficient for specific binding, and FBI-1 can form homomers through its POZ domain and, in vivo, through its zinc finger domain as well. In addition, FBI-1 associates with Tat, suggesting that repression of the short transcripts by Tat may be mediated through interactions between the two factors.

The disclosed NOV59 nucleic acid of the invention encoding a HIV-1 inducer of short transcripts binding protein like protein-like protein includes the nucleic acid whose sequence is provided in Table 59A or a fragment thereof. The invention also includes a mutant or variant nucleic acid any of whose bases may be changed from the corresponding base shown in Table 59A while still encoding a protein that maintains its HIV-1 inducer of short transcripts binding protein like protein-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 19 percent of the bases may be so changed.

The disclosed NOV59 protein of the invention includes the HIV-1 inducer of short transcripts binding protein like protein-like protein whose sequence is provided in Table 59B. The invention also includes a mutant or variant protein any of whose residues may be changed from the corresponding residue shown in Table 59B while still encoding a protein that maintains its HIV-1 inducer of short transcripts binding protein like protein-like activities and physiological functions, or a functional fragment thereof. In the mutant or variant protein, up to about 25 percent of the residues may be so changed.

The invention further encompasses antibodies and antibody fragments, such as F_{ab} or (F_{ab})₂, that bind immunospecifically to any of the proteins of the invention.

The above defined information for this invention suggests that this HIV-1 inducer of short transcripts binding protein like protein-like protein (NOV59) may function as a member of a "HIV-1 inducer of short transcripts binding protein like protein family". Therefore, the NOV59 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and in *vitro* of all tissues and cell types composing (but not limited to) those defined here.

The NOV59 nucleic acids and proteins of the invention are useful in potential therapeutic applications implicated in diseases including but not limited to various pathologies and disorders as indicated below. For example, a cDNA encoding the HIV-1 inducer of short transcripts binding protein like protein-like protein (NOV59) may be useful in gene therapy, and the HIV-1 inducer of short transcripts binding protein like protein-like protein (NOV59) may be useful when administered to a subject in need thereof. By way of nonlimiting example, the compositions of the present invention will have efficacy for treatment of patients suffering from Human Immunodeficiency Virus/ Acquired immune deficiency syndrome, cancer, and inflammatory diseases, or other pathologies or conditions. The NOV59 nucleic acid encoding the HIV-1 inducer of short transcripts binding protein like protein-like protein of the invention, or fragments thereof, may further be useful in diagnostic applications, wherein the presence or amount of the nucleic acid or the protein are to be assessed.

NOV59 nucleic acids and polypeptides are further useful in the generation of antibodies that bind immuno-specifically to the novel NOV59 substances for use in therapeutic or diagnostic methods. These antibodies may be generated according to methods known in the art, using prediction from hydrophobicity charts, as described in the "Anti-NOVX Antibodies" section below. The disclosed NOV59 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV60

NOV60 includes three beta tectorin-like proteins disclosed below. The disclosed sequences have been named NOV60a, NOV60b and NOV60c.

### NOV60a

A disclosed NOV60a nucleic acid of 1011 nucleotides (also referred to as CG57574-01) encoding a beta tectorin-like protein is shown in Table 60A. The start and stop codons are in bold letters.

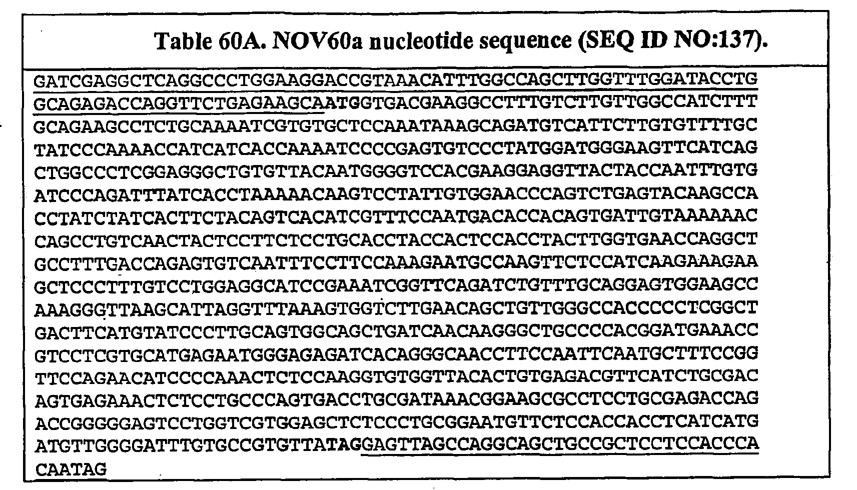

In a search of public sequence databases, the NOV60a nucleic acid sequence, located on chromsome 10 has 428 of 496 bases (86%) identical to a gb:GENBANK-ID:MMBETATEC(acc:X99806.2 mRNA from Mus musculus (Mus musculus mRNA for beta tectorin). Public nucleotide databases include all GenBank databases and the GeneSeq patent database.

The disclosed NOV60a polypeptide (SEQ ID N0:138) encoded by SEQ ID N0:137 has 300 amino acid residues and is presented in Table 60B using the one-letter amino acid code. Signal P, Psort and/or Hydropathy results predict that NOV60a has a signal peptide and is likely to be localized plasma membrane with a certainty of 0.6850. The most likely cleavage site for a NOV60a peptide is between amino acids 17 and 18.

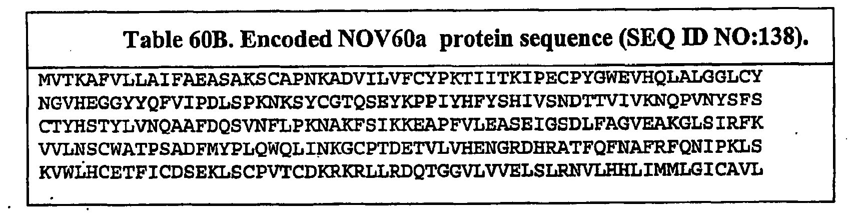

A search of sequence databases reveals that the NOV60a amino acid sequence has 149 of 174 amino acid residues (85%) identical to, and 155 of 174 amino acid residues (89%) similar to, the 329 amino acid residue ptnr:SPTREMBL-ACC:008524 protein from Mus musculus (Mouse) (BETA TECTORIN). Public amino acid databases include the GenBank databases, SwissProt, PDB and PIR.

NOV60a is predicted to be expressed in the following tissues because of the expression pattern of (GENRANK-ID: gb:GENBANK-ID:MMBETATEClacc:X99806.2) a closely related Mus musculus mRNA for beta tectorin homolog in species Mus musculus :cochleae. This information was derived by determining the tissue sources of the sequences that were included in the invention including but not limited to SeqCalling sources, Public EST sources, Literature sources, and/or RACE sources.

### NOV60b

A disclosed NOV60b nucleic acid of 1012 nucleotides (also referred to as CG57574-02) encoding a beta tectorin-like protein is shown in Table 60C. The start and stop codons are in bold letters.

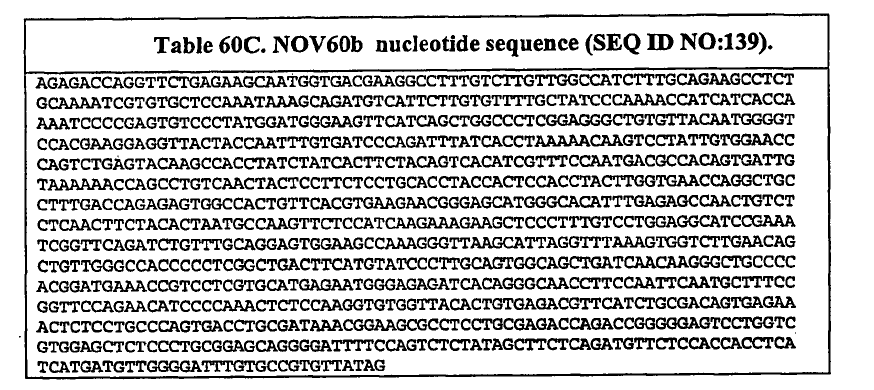

In a search of public sequence databases, the NOV60b nucleic acid sequence, located on chromsome 10 has 887 of 1012 bases (87%) identical to a gb:GENBANK-ID:MMBETATEC|acc:X99806.2 mRNA from Mus musculus (Mus musculus mRNA for beta tectorin). Public nucleotide databases include all GenBank databases and the GeneSeq patent database.

The disclosed NOV60b polypeptide (SEQ ID NO: 140) encoded by SEQ ID NO:139 has 329 amino acid residues and is presented in Table 60D using the one-letter amino acid code. Signal P, Psort and/or Hydropathy results predict that NOV60b has a signal peptide and is likely to be localized extracellularly with a certainty of 0.6850. The most likely cleavage site for a NOV60b peptide is between amino acids 17 and 18.

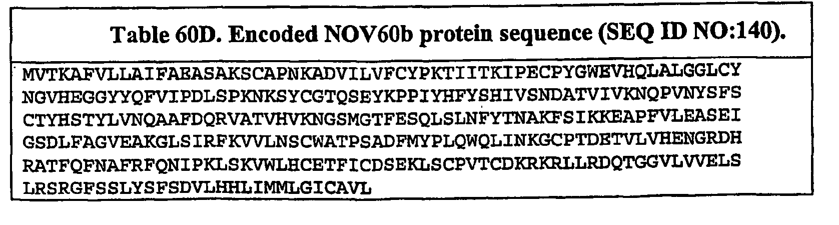

A search of sequence databases reveals that the NOV60b amino acid sequence has have 310 of 329 amino acid residues (94%) identical to, and 317 of 329 amino acid residues (96%) similar to, the 329 amino acid residue ptnr:SPTREMBL-ACC:O08524 protein from Mus musculus (Mouse) (BETA TECTORIN). Public amino acid databases include the GenBank databases, SwissProt, PDB and PIR.

NOV60b is predicted to be expressed in at least the ear. This information was derived by determining the tissue sources of the sequences that were included in the invention including but not limited to SeqCalling sources, Public EST sources, Literature sources, and/or RACE sources.

### NOV60c

A disclosed NOV60c nucleic acid of 1012 nucleotides (also referred to as CG57574-02) encoding a beta tectorin-like protein is shown in Table 60E. The start and stop codons are in bold letters.

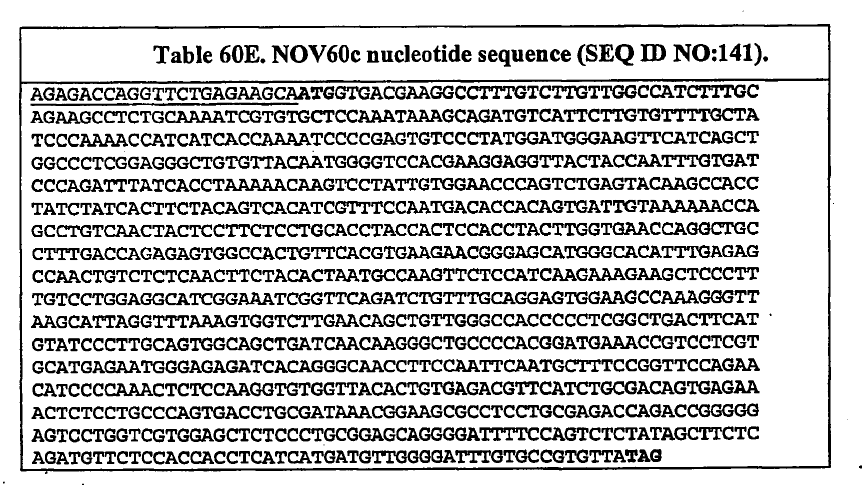

In a search of public sequence databases, the NOV60c nucleic acid sequence, located on chromsome 10 has 887 of 1012 bases (87%) identical to a gb:GENBANK-ID:MMBETATEC|acc:X99806.2 mRNA from Mus musculus (Mus musculus mRNA for beta tectorin). Public nucleotide databases include all GenBank databases and the GeneSeq patent database.

The disclosed NOV60c polypeptide (SEQ ID NO:142) encoded by SEQ ID NO:141 has 329 amino acid residues and is presented in Table 60F using the one-letter amino acid code. Signal P, Psort and/or Hydropathy results predict that NOV60c has a signal peptide and is likely to be localized plasma membrane with a certainty of 0.6850. The most likely cleavage site for a NOV60c peptide is between amino acids 17 and 18.

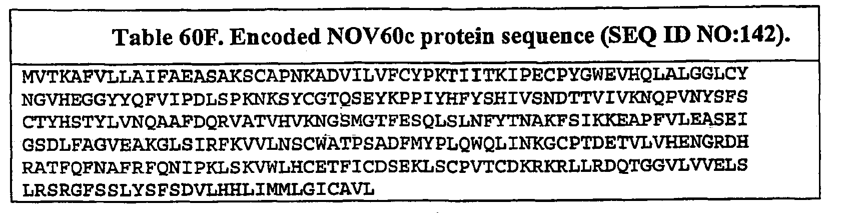

A search of sequence databases reveals that the NOV60c amino acid sequence has 310 of 329 amino acid residues (94%) identical to, and 317 of 329 amino acid residues (96%) similar to, the 329 amino acid residue ptnr:SPTREMBL-ACC:008524 protein from Mus musculus (Mouse) (BETA TECTORIN)Public amino acid databases include the GenBank databases, SwissProt, PDB and PIR.

NOV60c is predicted to be expressed in the following tissues because of the expression pattern of (GENBANK-ID: gb:GENBANK-ID:MMBETATEC|acc:X99806.2) a closely related Mus musculus mRNA for beta tectorin homolog in species Mus musculus :cochleae. This information was derived by determining the tissue sources of the sequences that were included in the invention including but not limited to SeqCalling sources, Public EST sources, Literature sources, and/or RACE sources.

The disclosed NOV60c polypeptide has homology to the amino acid sequences shown in the BLASTP data listed in Table 60G.

| **Table 60G. BLAST results for NOV60c** | | | | | |
|---|---|---|---|---|---|
| Gene Index/ Identifier | Protein/ Organism | Length (aa) | Identity (%) | Positives (%) | Expect |
| gi\|17158035\|ref\|NP 478129.1\| (NM 058222 | tectorin beta [Homo sapiens] | 329 | 295/330 (89%) | 297/330 (89%) | e-167 |
| gi\|7363457\|ref\|NP 0 33374.2 \| (NM_009348) | tectorin beta; [b]-tectorin [Mus musculus] | 329 | 280/330 (84%) | 289/330 (86%) | e-159 |
| gi\|1729889\|sp\|P5409 7\|TECB CHICK | BETA-TECTORIN PRECURSOR | 329 | 220/330 (66%) | 260/330 (78%) | e-125 |
| gi\|13385494\|ref\|NP 080265.1 (NM 025989) | RIKEN cDNA 2310037I18 [Mus musculus] | 531 | 63/208 (30%) | 99/208 (47%) | 4e-13 |
| gi\|12844889\|dbj\|BAB 26538.1\| (AK009843) | homolog to PANCREATIC SECRETORY GRANULE MEMBRANE MAJOR GLYCOPROTEIN GP2 PRECURSOR (PANCREATIC ZYMOGEN GRANULE MEMBRANE PROTEIN GP-2) (ZAP75)-putative [Mus musculus] | 573 | 62/200 (31%) | 96/200 (48%) | 4e-12 |

Table 60H lists the domain descriptions from DOMAIN analysis results against NOV60c. This indicates that the NOV60c sequence has properties similar to those of other proteins known to contain this domain.

| **Table 60H. Domain Analysis of NOV60c** |
|---|
| gnl\|Smart\|smart00241, ZP, Zona pellucida (ZP) domain; ZP proteins are responsible for sperm-adhesion fo the zona pellucida. ZP domains are also present in multidomain transmembrane proteins such as glycoprotein GP2, uromodulin and TGF-beta receptor type III (betaglycan). |
| CD-Length = 253 residues, 98.0% aligned |
| Score = 119 bits (297), Expect = 3e-28 |

Legan et al. (1997) cloned mouse alpha- and beta-tectorins. The mouse beta-tectorin gene encodes a 320-amino acid protein containing a hydrophobic secretory signal sequence and 4 potential N-glycosylation sites. Both alpha- and beta-tectorin contain a zona pellucida domain, but otherwise are not homologous.

To identify genes expressed in the vertebrate inner ear, Heller et al. (1998) established an assay that allowed rapid analysis of the differential expression pattern of mRNAs derived from an auditory epithelium-specific cDNA library. They performed subtractive hybridization to create an enriched probe, which was then used to screen the cDNA library. After digoxigenin-labeled antisense cRNAs had been transcribed from hybridization-positive clones, they conducted in situ hybridization on slides bearing cryosections of late embryonic chicken heads, bodies, and cochleas. They found 12 proteins whose mRNAs were specifically or highly expressed in the chicken's inner ear; the remainder encoded proteins that occur more widely. They identified proteins that had previously been described as expressed in the inner ear, such as beta-tectorin, calbindin (CALB1; 114050), and type II collagen (COL2A1; 120140). A second group of proteins abundant in the inner ear included 5 additional types of collagen. A third group, including COCH5B2 (COCH; 603196) and ear-specific connexin, comprised the proteins whose human equivalents are candidates to account for hearing disorders. This last group also included proteins expressed in 2 cells types unique to the inner ear, homogene cells and cells of the tegmentum vasculosum.

The disclosed NOV60c nucleic acid of the invention encoding a beta tectorin-like protein includes the nucleic acid whose sequence is provided in Table 60A or a fragment thereof. The invention also includes a mutant or variant nucleic acid any of whose bases may be changed from the corresponding base shown in Table 60A while still encoding a protein that maintains its beta tectorin-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 13 percent of the bases may be so changed.

The disclosed NOV60 protein of the invention includes the beta tectorin-like protein whose sequence is provided in Table 60B. The invention also includes a mutant or variant protein any of whose residues may be changed from the corresponding residue shown in Table 60B while still encoding a protein that maintains its beta tectorin-like activities and physiological functions, or a functional fragment thereof. In the mutant or variant protein, up to about 6 percent of the residues may be so changed.

The invention further encompasses antibodies and antibody fragments, such as F_{ab} or (F_{ab})₂, that bind immunospecifically to any of the proteins of the invention.

The above defined information for this invention suggests that this beta tectorin-like protein (NOV60) may function as a member of a "beta tectorin family". Therefore, the NOV60 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV60 nucleic acids and proteins of the invention are useful in potential therapeutic applications implicated in diseases including but not limited to various pathologies and disorders as indicated below. For example, a cDNA encoding the beta tectorin-like protein (NOV60) may be useful in gene therapy, and the beta tectorin-like protein (NOV60) may be useful when administered to a subject in need thereof. By way of nonlimiting example, the compositions of the present invention will have efficacy for treatment of patients suffering from hearing loss, or other pathologies or conditions. The NOV60 nucleic acid encoding the replacement-like protein of the invention, or fragments thereof, may further be useful in diagnostic applications, wherein the presence or amount of the nucleic acid or the protein are to be assessed.

NOV60 nucleic acids and polypeptides are further useful in the generation of antibodies that bind immuno-specifically to the novel NOV60 substances for use in therapeutic or diagnostic methods. These antibodies may be generated according to methods known in the art, using prediction from hydrophobicity charts, as described in the "Anti-NOVX Antibodies" section below. The disclosed NOV60 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV61

A disclosed NOV61 nucleic acid of 3802 nucleotides (also referred to as CG57505-01) encoding a KIAA1125-like protein is shown in Table 61A. The start and stop codons are in bold letters.

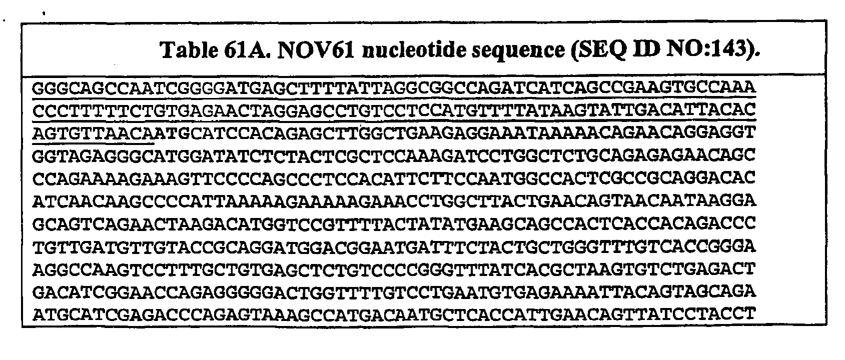

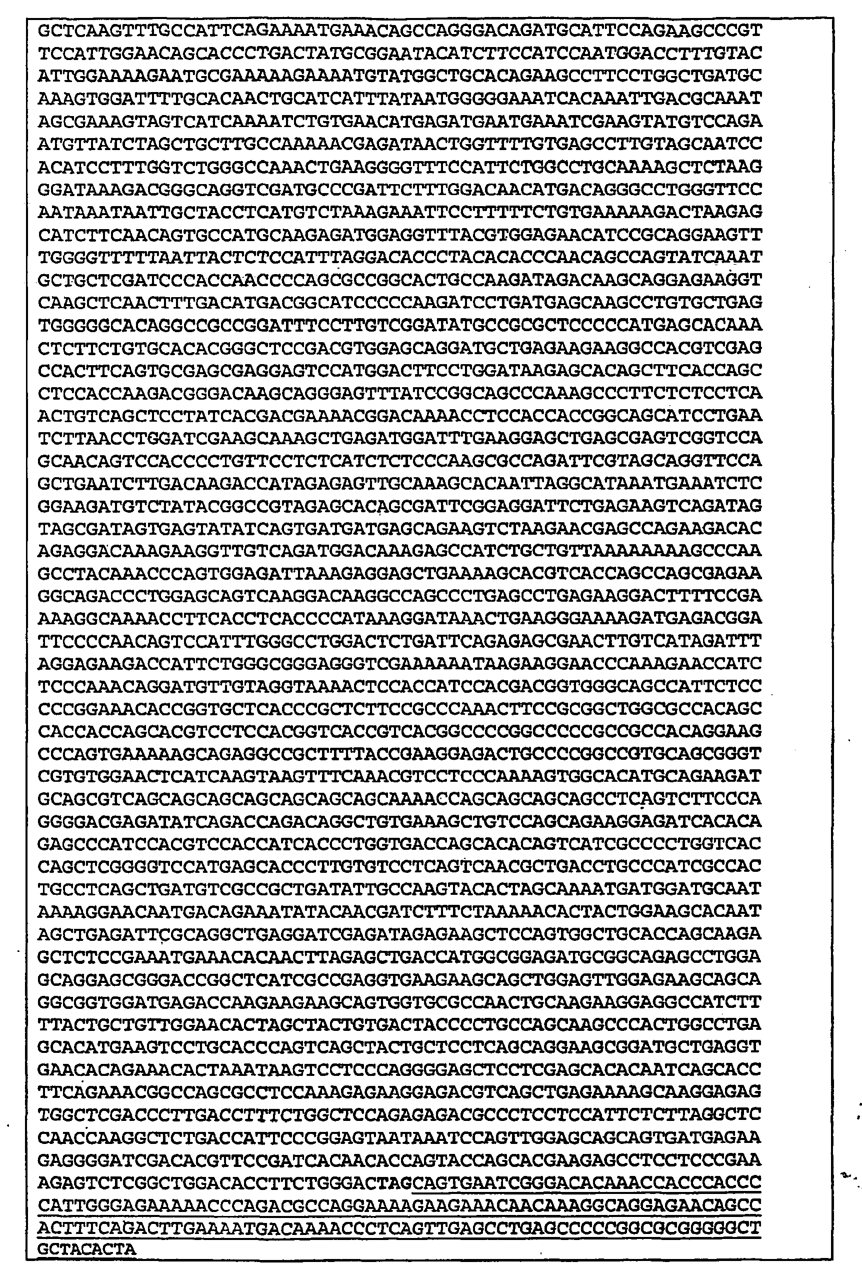

In a search of public sequence databases, the NOV61 nucleic acid sequence, located on chromsome 20 has 3797 of 3827 bases (99%) identical to a gb:GENBANK-ID:AB032951 1|acc:AB032951.1 mRNA from Homo sapiens (Homo sapiens mRNA for KIAA1125 protein, partial cds). Public nucleotide databases include all GenBank databases and the GeneSeq patent database.

The disclosed NOV61 polypeptide (SEQ ID NO: 144) encoded by SEQ ID NO: 143 has 1206 amino acid residues and is presented in Table 61B using the one-letter amino acid code. Signal P, Psort and/or Hydropathy results predict that NOV61 has no signal peptide and is likely to be localized in the nucleus with a certainty of 0.7000.

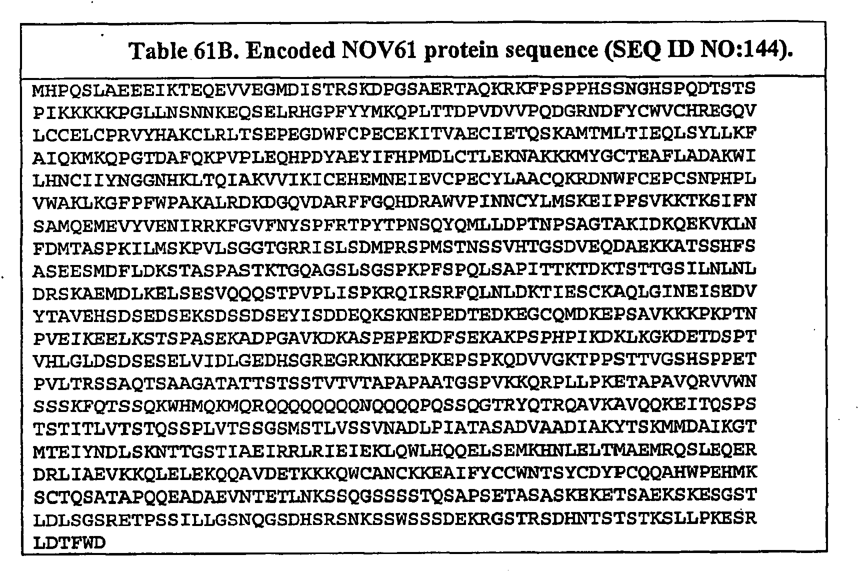

A search of sequence databases reveals that the NOV61 amino acid sequence has 1201 of 1201 amino acid residues (100%) identical to, and 1201 of 1201 amino acid residues (100%) similar to, the 1205 amino acid residue ptnr:SPTREMBL-ACC:Q9ULU4 protein from Homo sapiens (Human) (KIAA1125 PROTEIN). Public amino acid databases include the GenBank databases, SwissProt, PDB and PIR.

NOV61 is expressed in at least Brain, Lymphoid tissue, Kidney, Whole Organism, Bone Marrow, Prostate, Lung, Lung Pleura, Retina. This information was derived by determining the tissue sources of the sequences that were included in the invention including but not limited to SeqCalling sources, Public EST sources, Literature sources, and/or RACE sources.

The disclosed NOV61 polypeptide has homology to the amino acid sequences shown in the BLASTP data listed in Table 61 C.

| **Table 61C. BLAST results for NOV61** | | | | | |
|---|---|---|---|---|---|
| Gene Index/ Identifier | Protein/ Organism | Length (aa) | Identity (%) | Positives (%) | Expect |
| gi\|6329749\|dbj\|BAA8 6439.1\| (AB032951) | KIAA1125 protein [Homo sapiens] | 1205 | 1201/1201 (100%) | 1201/1201 (100%) | 0.0 |
| gi\|17980969\|gb\|AAL5 0790.1\|AF454056 1 (AF454056) | se14-3r protein [Homo sapiens] | 995 | 992/1041 (95%) | 994/1041 (95%) | 0.0 |
| gi\|14786224\|ref\|XP 012932.3\| (XM_012932) | similar to protein kinase C binding protein 1 (H. sapiens) [Homo sapiens] | 949 | 932/933 (99%) | 933/933 (99%) | 0.0 |
| gi\|11385648\|gb\|AAG3 4905.1\|AF273045 1 (AF273045) | CTCL tumor antigen sel4-3 [Homo sapiens] | 764 | 762/810 (94%) | 763/810 (94%) | 0.0 |
| gi\|13677199\|emb\|CAC 19781.1\|(AL031666 | dJ569M23.1. - 1 (protein kinase C binding protein 1, isoform 1 (KIAA1125)) [Homo sapiens] | 521 | 520/520 (100%) | 520/520 (100%) | 0.0 |

Table 61D-F lists the domain descriptions from DOMAIN analysis results against NOV61. This indicates that the NOV61 sequence has properties similar to those of other proteins known to contain this domain.

| **Table 61D. Domain Analysis of NOV61** |
|---|
| gnl\|Smart \|smart00297, BROMO, bromo domain |
| CD-Length = 109 residues, 94.5% aligned |
| Score = 81.3 bits (199), Expect = 3e-16 |

| **Table 61E. Domain Analysis of NOV61** |
|---|
| gnlPfam\|pfam00628, PHD, PHD-finger. PHD folds into an interleaved type of Zn-finger chelating 2 Zn ions in a similar manner to that of the RING and FYVE domains. |
| CD-Length = 49 residues, 93.9% aligned |
| Score = 56.6 bits (135), Expect = 8e-09 |

| **Table 61F. Domain Analysis of NOV61** |
|---|
| gnl\|Pfam\|pfam01753, zf-MYND, MYND finger. |
| CD-Length = 38 residues, 100.0% aligned |
| Score = 45.1 bits (105), Expect = 2e-05 |

Mukai and Ono (1994) isolated a cDNA for a protein kinase, designated PKN by them, from a human hippocampus cDNA library. The putative 942-amino acid protein has leucine zipper-like sequences at its amino terminus and contains a domain with strong similarity to that of the protein kinase C family. Ubiquitous expression in human tissues was shown. Antisera detected a 120-kD recombinantly expressed protein on Western blots. The protein showed intrinsic protein kinase activity that was abolished by a mutation in the predicted ATP binding site.

Palmer et al. (1994) used degenerate PCR to isolate 3 novel members of the closely related protein kinase C (PKC) family, termed PRK1, PRK2 (602549), and PRK3. Palmer et al. (1995) cloned a full-length cDNA of PRK1 from a human fetal brain library. Using Northern blot and RT-PCR analyses Palmer et al. (1995) detected expression of PRK1 in all tissues and cell lines tested.

In a study of proteins that bind to the rho GTPase (see Ridley and Hall, 1992), Amano et al. (1996) discovered 1 protein that had partial amino acid sequences identical to PKN. They found that rho binds directly to a polybasic region of the N-terminal regulatory domain that precedes the leucine zipper-like motif. The authors speculated that through this activity, PKN may mediate the rho-dependent signaling pathway.

Bartsch et al. (1998) used fluorescence in situ hybridization to map the PRKCL1 gene to 19p13.1-p12 and radiation hybrid mapping to localize the gene in subband 19p12. By segregation analysis, they mapped the corresponding mouse gene (Prkcll) to chromosome 8.

The disclosed NOV nucleic acid of the invention encoding a KIAA1125-like protein includes the nucleic acid whose sequence is provided in Table 61A or a fragment thereof. The invention also includes a mutant or variant nucleic acid any of whose bases may be changed from the corresponding base shown in Table 61A while still encoding a protein that maintains its KIAA1125-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1 percent of the bases may be so changed.

The disclosed NOV61 protein of the invention includes the KIAA1125-like protein whose sequence is provided in Table 61B. The invention also includes a mutant or variant protein any of whose residues may be changed from the corresponding residue shown in Table 61B while still encoding a protein that maintains its KIAA1125-like activities and physiological functions, or a functional fragment thereof. In the mutant or variant protein, up to about 0 percent of the residues may be so changed.

The invention further encompasses antibodies and antibody fragments, such as F_{ab} or (F_{ab})₂, that bind immunospecifically to any of the proteins of the invention.

The above defined information for this invention suggests that this KIAA1125-like protein (NOV61) may function as a member of a "KIAA1125 family". Therefore, the NOV61 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV61 nucleic acids and proteins of the invention are useful in potential therapeutic applications implicated in diseases including but not limited to various pathologies and disorders as indicated below. For example, a cDNA encoding the KIAA1125-like protein (NOV61) may be useful in gene therapy, and the KIAA1125-like protein (NOV61) may be useful when administered to a subject in need thereof. By way of nonlimiting example, the compositions of the present invention will have efficacy for treatment of patients suffering from Cardiomyopathy, Atherosclerosis, Hypertension, Congenital heart defects, Aortic stenosis , Atrial septal defect (ASD), Atrioventricular (A-V) canal defect, Ductus arteriosus , Pulmonary stenosis , Subaortic stenosis, Ventricular septal defect (VSD), valve diseases, Tuberous sclerosis, Scleroderma, Obesity, Transplantation, Aneurysm, Fibromuscular dysplasia, Stroke, Anemia, Bleeding disorders, Adrenoleukodystrophy , Congenital Adrenal Hyperplasia, Diabetes, Von Hippel-Lindau (VHL) syndrome, Pancreatitis, Hyperparathyroidism, Hypoparathyroidism, SIDS, Endometriosis, Fertility, Xerostomia, Hypercalceimia, Ulcers, Cirrhosis, Inflammatory bowel disease, Diverticular disease, Hirschsprung's disease, Crohn's Disease, Appendicitis, Hemophilia, hypercoagulation, Idiopathic thrombocytopenic purpura, autoimmume disease, allergies, immunodeficiencies, Graft vesus host, Ataxia-telangiectasia, Hemophilia, Lymphedema, Tonsilitis, Osteoporosis, Arthritis; Ankylosing spondylitis, Scoliosis, Tendinitis, Muscular dystrophy, Lesch-Nyhan syndrome, Myasthenia gravis, Dental disease and infection, Alzheimer's disease, Tuberous sclerosis, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection, Growth and reproductive disorders, Endocrine dysfunctions, Systemic lupus erythematosus, Asthma, Emphysema, ARDS, Pharyngitis, Laryngitis, Hearing loss, Tinnitus, Psoriasis, Actinic keratosis ,Tuberous sclerosis, Acne, Hair growth, allopecia, pigmentation disorders, cystitis, incontinence, Renal artery stenosis, Interstitial nephritis, Glomerulonephritis, Polycystic kidney disease, Systemic lupus erythematosus, Renal tubular acidosis, IgA nephropathy, Vesicoureteral reflux, glaucoma, blindness, and Hypothyroidism, or other pathologies or conditions. The NOV61 nucleic acid encoding the KIAA1125-like protein of the invention, or fragments thereof, may further be useful in diagnostic applications, wherein the presence or amount of the nucleic acid or the protein are to be assessed.

NOV61 nucleic acids and polypeptides are further useful in the generation of antibodies that bind immuno-specifically to the novel NOV61 substances for use in therapeutic or diagnostic methods. These antibodies may be generated according to methods known in the art, using prediction from hydrophobicity charts, as described in the "Anti-NOVX Antibodies" section below. The disclosed NOV61 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV62

NOV62 includes two zinc finger BOP-like proteins disclosed below. The disclosed sequences have been named NOV62a and NOV62b.

### NOV62a

A disclosed NOV62a nucleic acid of 1629 nucleotides (also referred to as CG57473-01) encoding a zinc finger BOP-like protein is shown in Table 62A. The start and stop codons are in bold letters.

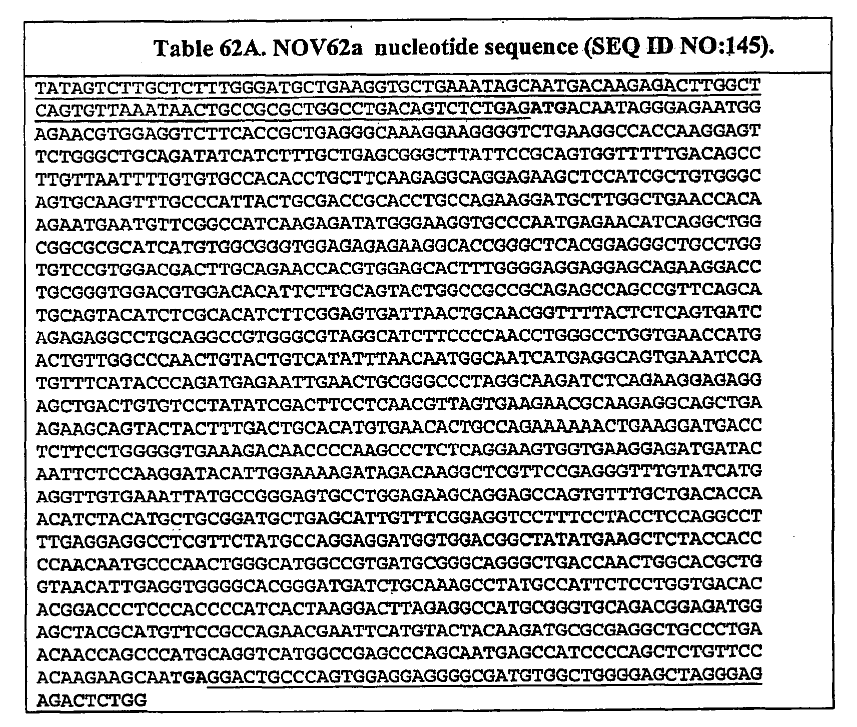

In a search of public sequence databases, the NOV62a nucleic acid sequence, located on chromsome 2 has 1392 of 1573 bases (88%) identical to a gb:GENBANK-ID:MMU76373|acc:U76373.2 mRNA from Mus musculus (Mus musculus skm-BOP1 (Bop) mRNA, complete cds). Public nucleotide databases include all GenBank databases and the GeneSeq patent database.

The disclosed NOV62a polypeptide (SEQ ID NO:146) encoded by SEQ ID NO:145 has 490 amino acid residues and is presented in Table 62B using the one-letter amino acid code. Signal P, Psort and/or Hydropathy results predict that NOV62a has no signal peptide and is likely to be localized in the cytoplasm with a certainty of 0.6500.

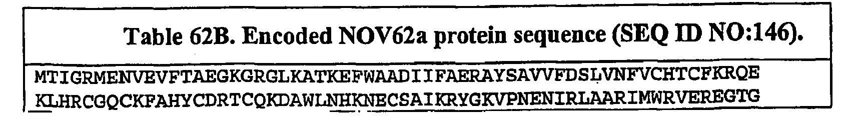

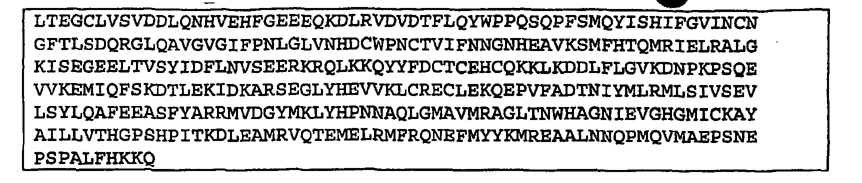

A search of sequence databases reveals that the NOV62a amino acid sequence has 458 of 485 amino acid residues (94%) identical to, and 478 of 485 amino acid residues (98%) similar to, the 485 amino acid residue ptnr:SPTREMBL-ACC:P97443 protein from Mus musculus (Mouse) (ZINC-FINGER PROTEIN BOP). Public amino acid databases include the GenBank databases, SwissProt, PDB and PIR.

NOV62a is expressed in at least Whole Organism, Heart, Lung, Prostate, Skeletal Muscle. This information was derived by determining the tissue sources of the sequences that were included in the invention including but not limited to SeqCalling sources, Public EST sources, Literature sources, and/or RACE sources.

### NOV62b

A disclosed NOV62b nucleic acid of 1555 nucleotides (also referred to as CG57473-01) encoding a zinc finger BOP-like protein is shown in Table 62C. The start and stop codons are in bold letters.

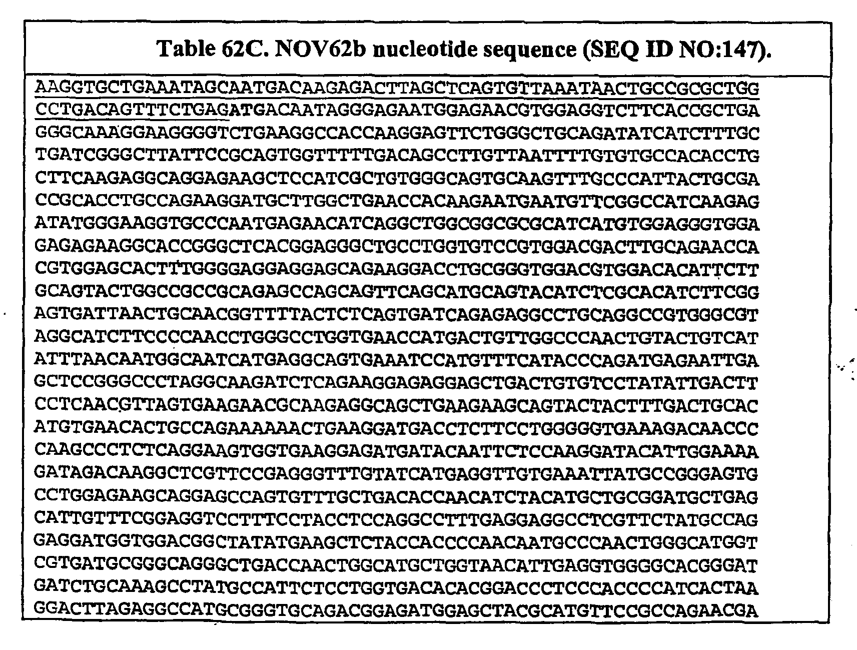

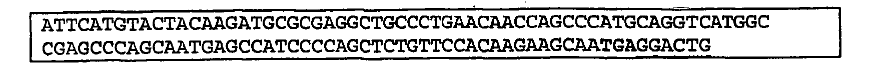

In a search of public sequence databases, the NOV62b nucleic acid sequence, located on chromsome 2 has has 1356 of 1525 bases (88%) identical to a gb:GENBANK-ID:MMU76373|acc:U76373.2 mRNA from Mus musculus (Mus musculus skm-BOP1 (Bop) mRNA, complete cds). Public nucleotide databases include all GenBank databases and the GeneSeq patent database.

The disclosed NOV62b polypeptide (SEQ ID NO:148) encoded by SEQ ID NO:147 has 490 amino acid residues and is presented in Table 62D using the one-letter amino acid code. Signal P, Psort and/or Hydropathy results predict that NOV62b has no signal peptide and is likely to be localized in the cytoplasm with a certainty of 0.6500.

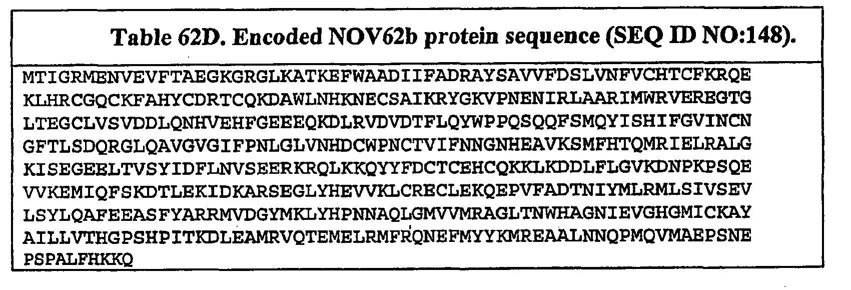

A search of sequence databases reveals that the NOV62b amino acid sequence has 457 of 485 amino acid residues (94%) identical to, and 478 of 485 amino acid residues (98%) similar to, the 485 amino acid residue ptnr:SPTREMBL-ACC:P97443 protein from Mus musculus (Mouse) (ZINC-FINGER PROTEIN BOP). Public amino acid databases include the GenBank databases, SwissProt, PDB and PIR.

NOV62b is expressed in at least Whole Organism, Heart, Lung, Prostate, Skeletal and Muscle. This information was derived by determining the tissue sources of the sequences that were included in the invention including but not limited to SeqCalling sources, Public EST sources, Literature sources, and/or RACE sources.

The disclosed NOV62b polypeptide has homology to the amino acid sequences shown in the BLASTP data listed in Table 62E .

| **Table 62E. BLAST results for NOV62b** | | | | | |
|---|---|---|---|---|---|
| Gene Index/ Identifier | Protein/ Organism | Length (aa) | Identity (%) | Positives (%) | Expect |
| gi\|5870832\|gb\|AAC53 021.2\| (U76373) | skm-BOP1 [Mus musculus] | 485 | 458/485 (94%) | 478/485 (98%) | 0.0 |
| gi\|10257425\|ref\|NP 033892.1\| (NM_009762) | CD8beta opposite strand [Mus musculus] | 472 | 444/485 (91%) | 465/485 (95%) | 0.0 |
| gi\|1809322\|gb\|AAC53 020.1\| (U76371) | t-BOP [Mus musculus] | 456 | 419/447 (93%) | 437/447 (97%) | 0.0 |
| gi\|16930387\|gb\|AAL3 1880.1\|AF410781 1 (AF410781) | cardiac and skeletal muscle-specific BOP1 [Gallus gallus] | 486 | 397/486 (81%) | 441/486 (90%) | 0.0 |
| gi\|16930389\|gb\|AAL31881.1\|AF410782 1 (AF410782) | cardiac and skeletal muscle-specific BOP2 [Gallus gallus] | 473 | 384/486 (79%) | 428/486 (88%) | 0.0 |

Table 62F-G lists the domain descriptions from DOMAIN analysis results against NOV62b. This indicates that the NOV62b sequence has properties similar to those of other proteins known to contain this domain.

| **Table 62F. Domain Analysis of NOV62b** |
|---|
| gnlPfamlpfam01753, zf-MYND, MYND finger. |
| CD-Length = 38 residues, 100.0% aligned |
| Score = 57.8 bits (138), Expect = 1e-09 |

| **Table 62G. Domain Analysis of NOV62b** |
|---|
| gnl\|Smart\|smart00317, SET, SET (Su(var)3-9, Enhancer-of zeste, Trithorax) domain; Putative methyl transferase, based on outlier plant homologues |
| CD-Length = 125 residues, 44.0% aligned |
| Score = 53.9 bits (128), Expect = 2e-08 |

Transcriptional regulatory proteins containing tandemly repeated zinc finger domains are thought to be involved in both normal and abnormal cellular proliferation and differentiation. One abundant class of such transcriptional regulators resembles the Drosophila Kruppel segmentation gene product due to the presence of repeated Cys2-His2 (C2H2) zinc finger domains that are connected by conserved sequences, called H/C links. See ZNF91 (603971) for general information on zinc finger proteins.

By screening a human insulinoma cDNA library with a degenerate oligonucleotide corresponding to the H/C linker sequence, Tommerup et al. (1993) isolated cDNAs potentially encoding zinc finger proteins. Tommerup and Vissing (1995) performed sequence analysis on a number of these cDNAs and identified several novel zinc finger protein genes, including ZNF36; which they called ZNF139. The ZNF139 cDNA predicts a protein belonging to the Kruppel family of zinc finger proteins.

By isotopic in situ hybridization, Rousseau-Merck et al. (1995) mapped the ZNF36 gene, which they called KOX18, to 7q21-q22. From pulsed field gel electrophoresis studies, they showed that KOX18 is within less than 250 kb of KOX25 (ZNF38; 601261). Rousseau-Merck et al. (1995) tabulated 18 different KOX genes that had been located in pairs within 9 DNA fragments of 200 to 580 kb on 7 different chromosomes. By FISH, Tommerup and Vissing (1995) mapped the ZNF36 gene to 7q21.3-q22.1.

The disclosed NOV62 nucleic acid of the invention encoding a zinc finger BOP-like protein includes the nucleic acid whose sequence is provided in Table 62A or 62C or a fragment thereof. The invention also includes a mutant or variant nucleic acid any of whose bases may be changed from the corresponding base shown in Table 62A or 62C while still encoding a protein that maintains its zinc finger BOP-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 12 percent of the bases may be so changed.

The disclosed NOV62 protein of the invention includes the zinc finger BOP-like protein whose sequence is provided in Table 62B or 62D. The invention also includes a mutant or variant protein any of whose residues may be changed from the corresponding residue shown in Table 62B or 62D while still encoding a protein that maintains its zinc finger BOP-like activities and physiological functions, or a functional fragment thereof. In the mutant or variant protein, up to about 6 percent of the residues may be so changed.

The invention further encompasses antibodies and antibody fragments, such as F_{ab} or (F_{ab})₂, that bind immunospecifically to any of the proteins of the invention.

The above defined information for this invention suggests that this zinc finger BOP-like protein (NOV62) may function as a member of a "zinc finger BOP family". Therefore, the NOV62 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV62 nucleic acids and proteins of the invention are useful in potential therapeutic applications implicated in diseases including but not limited to various pathologies and disorders as indicated below. For example, a cDNA encoding the zinc finger BOP-like protein (NOV62) may be useful in gene therapy, and the zinc finger BOP-like protein (NOV62) may be useful when administered to a subject in need thereof. By way of nonlimiting example, the compositions of the present invention will have efficacy for treatment of patients suffering from Cardiomyopathy, Atherosclerosis, Hypertension, Congenital heart defects, Aortic stenosis , Atrial septal defect (ASD), Atrioventricular (A-V) canal defect, Ductus arteriosus , Pulmonary stenosis , Subaortic stenosis, Ventricular septal defect (VSD), valve diseases, Tuberous sclerosis, Scleroderma, Obesity, Transplantation, Aneurysm, Fibromuscular dysplasia, Stroke, Anemia, Bleeding disorders, Adrenoleukodystrophy, Congenital Adrenal Hyperplasia, Diabetes, Von Hippel-Lindau (VHL) syndrome, Pancreatitis, Hyperparathyroidism, Hypoparathyroidism, SIDS, Endometriosis, Fertility, Xerostomia, Hypercalceimia, Ulcers, Cirrhosis, Inflammatory bowel disease, Diverticular disease, Hirschsprung's disease, Crohn's Disease, Appendicitis, Hemophilia, hypercoagulation, Idiopathic thrombocytopenic purpura, autoimmume disease, allergies, immunodeficiencies, Graft vesus host, Ataxia-telangiectasia, Hemophilia, Lymphedema, Tonsilitis, Osteoporosis, Arthritis, Ankylosing spondylitis, Scoliosis, Tendinitis, Muscular dystrophy, Lesch-Nyhan syndrome, Myasthenia gravis, Dental disease and infection, Alzheimer's disease, Tuberous sclerosis, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection, Growth and reproductive disorders, Endocrine dysfunctions, Systemic lupus erythematosus , Asthma, Emphysema, ARDS, Pharyngitis, Laryngitis, Hearing loss, Tinnitus, Psoriasis, Actinic keratosis ,Tuberous sclerosis, Acne, Hair growth, allopecia, pigmentation disorders, cystitis, incontinence, Renal artery stenosis, Interstitial nephritis, Glomerulonephritis, Polycystic kidney disease, Systemic lupus erythematosus, Renal tubular acidosis, IgA nephropathy, Vesicoureteral reflux, glaucoma, blindness, and Hypothyroidism, or other pathologies or conditions. The NOV62 nucleic acid encoding the zinc finger BOP-like protein of the invention, or fragments thereof, may further be useful in diagnostic applications, wherein the presence or amount of the nucleic acid or the protein are to be assessed.

NOV62 nucleic acids and polypeptides are further useful in the generation of antibodies that bind immuno-specifically to the novel NOV62 substances for use in therapeutic or diagnostic methods. These antibodies may be generated according to methods known in the art, using prediction from hydrophobicity charts, as described in the "Anti-NOVX Antibodies" section below. The disclosed NOV62 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV63

A disclosed NOV63 nucleic acid of 3647 nucleotides (also referred to as CG57777-01) encoding a secreted protein-like protein is shown in Table 63A. The start and stop codons are in bold letters.

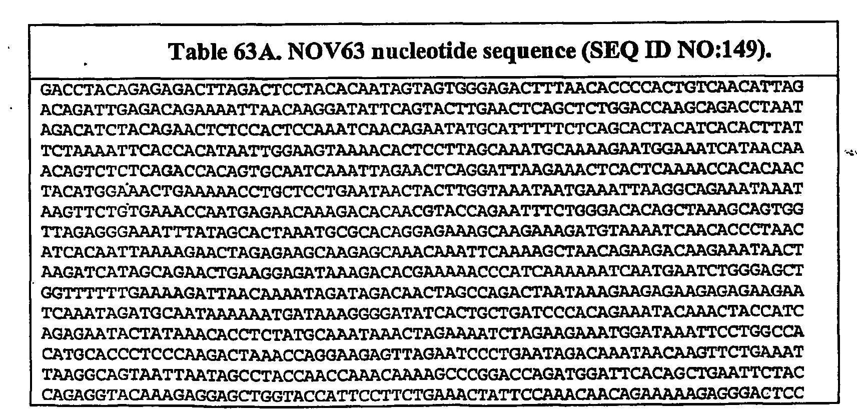

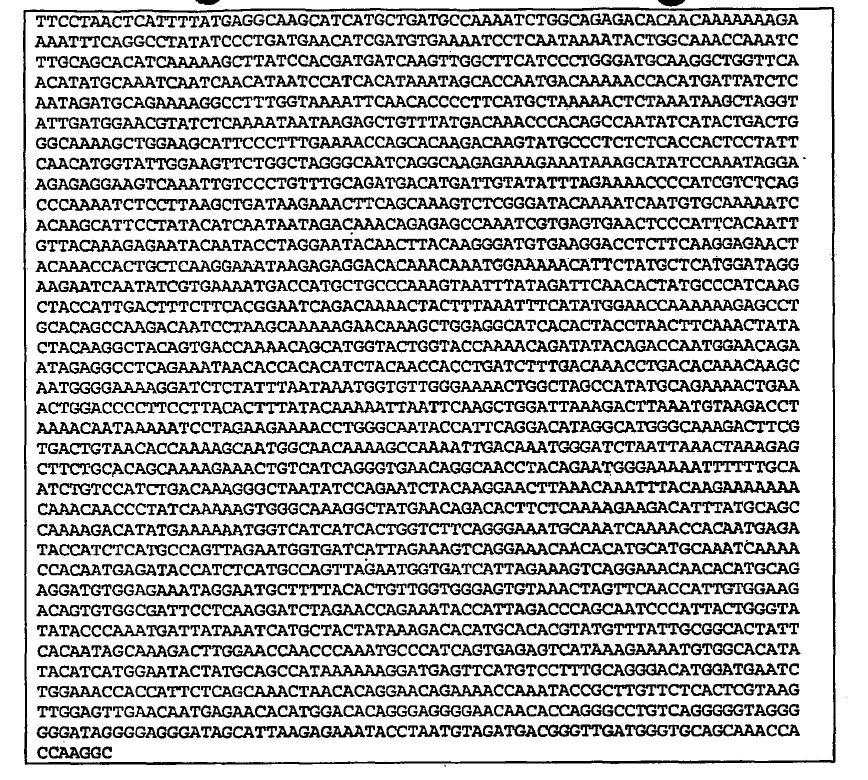

In a search of public sequence databases, the NOV63 nucleic acid sequence, located on chromsome 13 has 3146 of 3647 bases (86%) identical to a gb:GENBANK-ID:HSIL25FLlacc:X67285.1 mRNA from Homo sapiens (H.sapiens gene for interleukin-2 (5' flanking region)). Public nucleotide databases include all GenBank databases and the GeneSeq patent database.

The disclosed NOV63 polypeptide (SEQ ID NO: 150) encoded by SEQ ID NO: 149 has 1081 amino.acid residues and is presented in Table 63B using the one-letter amino acid code. Signal P, Psort and/or Hydropathy results predict that NOV63 has no signal peptide and is likely to be localized in the cytoplasm with a certainty of 0.6000.

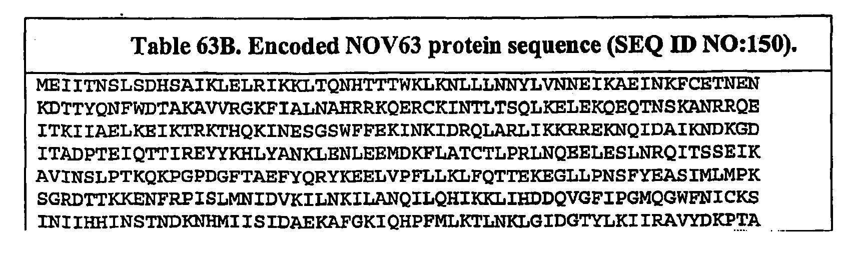

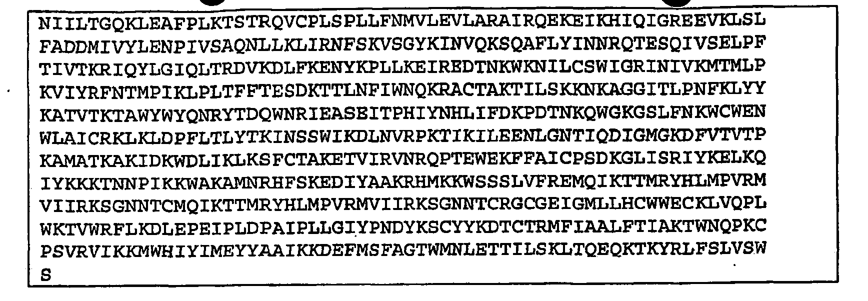

A search of sequence databases reveals that the NOV63 amino acid sequence has 752 of 865 amino acid residues (86%) identical to, and 787 of 865 amino acid residues (90%) similar to, the 1010 amino acid residue patp:B38012 Human secreted protein encoded by gene 3 clone HNHCT15. Public amino acid databases include the GenBank databases, SwissProt, PDB and PIR.

The disclosed NOV63 polypeptide has homology to the amino acid sequences shown in the BLASTP data listed in Table 63C.

| **Table 63C. BLAST results for NOV63** | | | | | |
|---|---|---|---|---|---|
| Gene Index/ Identifier | Protein/ Organism | Length (aa) | Identity (%) | Positives (%) | Expect |
| gi\|1106322\|pir\|\|B340 87 | protein (L1H 3' region) - human | 1280 | 919/1078 (85%) | 966/1078 (89%) | 0.0 |
| gi\|2072948\|gb\|AAC51 261.1 (U93563) | putative p150 [Homo sapiens] | 1275 | 904/1078 (83%) | 959/1078 (88%) | 0.0 |
| gi\|339777\|gb \|AABS93 68.1 (M80344) | ORF2 contains a reverse transcriptase domain. [Homo sapiens] | 1275 | 898/1078 (83%) | 957/1078 (88%) | 0.0 |
| gi\|5052951\|9b\|AAD38 785.1\|AF149422 2 (AF149422) | unknown [Homo sapiens] | 1275 | 900/1078 (83%) | 957/1078 (88%) | 0.0 |
| gi\|12136-112\|pir\| \|S65 824 | reverse transcriptase homolog - human transposon L1.1 | 1275 | 898/1078 (83%) | 957/1078 (88%) | 0.0 |

Table 63D lists the domain descriptions from DOMAIN analysis results against NOV63. This indicates that the NOV63 sequence has properties similar to those of other proteins known to contain this domain.

| **Table 63D. Domain Analysis of NOV63** | | |
|---|---|---|
| gnl\|Pfam\|pfam00078, rvt, Reverse transcriptase (RNA-dependent DNA polymerase). A reverse transcriptase gene is usually indicative of a mobile element such as a retrotransposon or retrovirus. Reverse transcriptases occur in a variety of mobile elements, including retrotranspobons, retroviruses, group II introns, bacterial msDNAs, hepadnaviruses, and caulimoviruses. | | |
| | CD-Length = 208 residues, 97.6% aligned. | |
| | | Score = 99.0 bits (245), Expect = 1e-21 |

Secreted proteins can act as cytokines, growth factors, chemotactic factors, and ligands for cell surface receptors. Secreted functions play vital roles in the regulation of cell motility, proliferation, differentiation and apoptosis.

The disclosed NOV63 nucleic acid of the invention encoding a secreted protein-like protein includes the nucleic acid whose sequence is provided in Table 63A or a fragment thereof. The invention also includes a mutant or variant nucleic acid any of whose bases may be changed from the corresponding base shown in Table 63A while still encoding a protein that maintains its secreted protein-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 14 percent of the bases may be so changed.

The disclosed NOV63 protein of the invention includes the secreted protein-like protein whose sequence is provided in Table 63B. The invention also includes a mutant or variant protein any of whose residues may be changed from the corresponding residue shown in Table 63B while still encoding a protein that maintains its secreted protein-like activities and physiological functions, or a functional fragment thereof. In the mutant or variant protein, up to about 14 percent of the residues may be so changed.

The invention further encompasses antibodies and antibody fragments, such as F_{ab} or (F_{ab})₂, that bind immunospecifically to any of the proteins of the invention.

The above defined information for this invention suggests that this secreted protein-like protein (NOV63) may function as a member of a "secreted protein family". Therefore, the NOV63 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and in *vitro* of all tissues and cell types composing (but not limited to) those defined here.

The NOV63 nucleic acids and proteins of the invention are useful in potential therapeutic applications implicated in diseases including but not limited to various pathologies and disorders as indicated below. For example, a cDNA encoding the secreted protein-like protein (NOV63) may be useful in gene therapy, and the secreted protein-like protein (NOV63) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV63 nucleic acid encoding the secreted protein-like protein of the invention, or fragments thereof, may further be useful in diagnostic applications, wherein the presence or amount of the nucleic acid or the protein are to be assessed.

NOV63 nucleic acids and polypeptides are further useful in the generation of antibodies that bind immuno-specifically to the novel NOV63 substances for use in therapeutic or diagnostic methods. These antibodies may be generated according to methods known in the art, using prediction from hydrophobicity charts, as described in the "Anti-NOVX Antibodies" section below. The disclosed NOV63 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV64

A disclosed NOV64 nucleic acid of 3081 nucleotides (also referred to as CGS7779-01) encoding a secreted protein-like protein is shown in Table 64A. The start and stop codons are in bold letters.

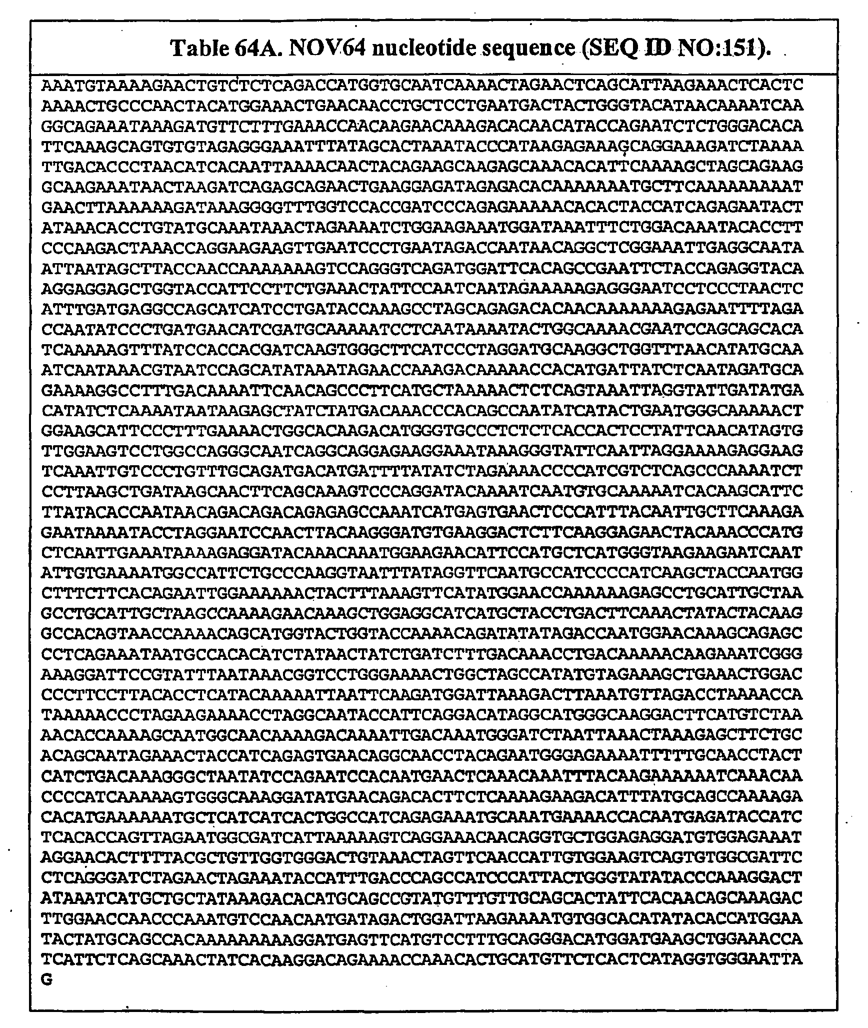

In a search of public sequence databases, the NOV64 nucleic acid sequence, located on chromsome 13 has 2741 of 3050 bases (89%) identical to a gb:GENBANK-ID:HSU157D4|acc:Z68871.1 mRNA from Homo sapiens (Human DNA sequence from cosmid U157D4, between markers DXS366 and DXS87 on chromosome X). Public nucleotide databases include all GenBank databases and the GeneSeq patent database.

The disclosed NOV64 polypeptide (SEQ ID NO:152) encoded by SEQ ID NO:151 has 1017 amino acid residues and is presented in Table 64B using the one-letter amino acid code. Signal P, Psort and/or Hydropathy results predict that NOV64 has no signal peptide and is likely to be localized in the cytoplasm with a certainty of 0.3906.

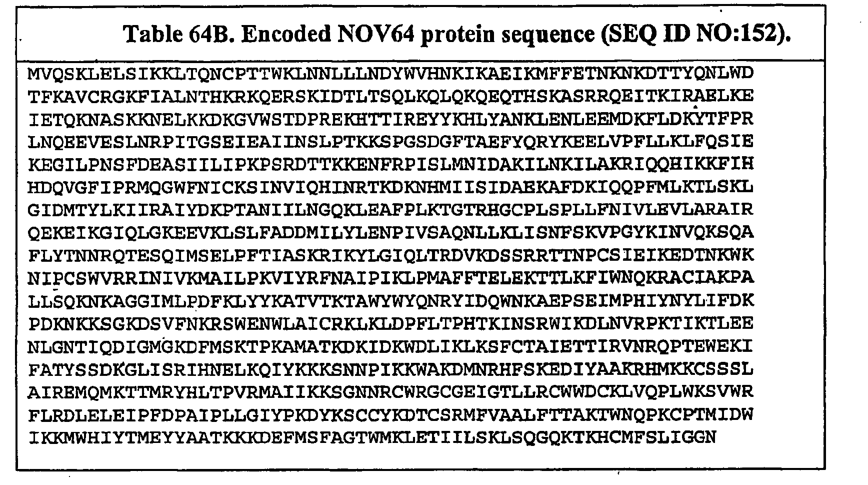

A search of sequence databases reveals that the NOV64 amino acid sequence has 829 of 977 amino acid residues (84%) identical to, and 864 of 977 amino acid residues (88%) similar to, the 1010 amino acid residue patp:B38012 Human secreted protein encoded by gene 3 clone HNHCT15. Public amino acid databases include the GenBank databases, SwissProt, PDB and PIR.

The disclosed NOV64 polypeptide has homology to the amino acid sequences shown in the BLASTP data listed in Table 64C.

| **Table 64C. BLAST results for NOV64** | | | | | |
|---|---|---|---|---|---|
| Gene Index/ Identifier | Protein/ Organism | Length (aa) | Identity (%) | Positives (%) | Expect |
| gi\|106322\|pir\|\|B340 87 | protein (L1H 3' region) - human | 1280 | 929/1044 (88%) | 956/1044 (90%) | 0.0 |
| gi\|5052951\|gb\|AAD38 785.1\|AF149422 2 (AF149422) | unknown [Homo sapiens] | 1275 | 920/1044 (88%) | 949/1044 (90%) | 0.0 |
| gi\|2072948\|gb\|AAC51 261.1\| (U93563) | putative p150 [Homo sapiens] | 1275 | 919/1044 (88%) | 946/1044 (90%) | 0.0 |
| gi\|2072958\|gb\|AAC51 267.1\| (U93567 | p150 [Homo sapiens] | 1275 | 918/1044 (87%) | 947/1044 (89%), | 0.0 |
| gi\|5070622\|gb\|AAD39 215.1\|AF148856 2 (AF148856) | unknown [Homo sapiens] | 1275 | 918/1044 (87%) | 949/1044 (89%), | 0.0 |

Table 64D lists the domain descriptions from DOMAIN analysis results against NOV64. This indicates that the NOV64 sequence has properties similar to those of other proteins known to contain this domain.

| **Table 64D. Domain Analysis of NOV64** |
|---|
| gnl\|Pfam\|pfam00078, rvt, Reverse transcriptase (RNA-dependent DNA polymerase). A reverse transcriptase gene is usually indicative of a mobile element such as a retrotransposon or retrovirus. Reverse transcriptases occur in a variety of mobile elements, including retrotransposons, retroviruses, group II introns, bacterial msDNAs, hepadnaviruses, and caulimoviruses. |
| CD-Length = 208 residues, 97.6% aligned |
| Score = 109 bits (272), Expect = 9e-25 |

The disclosed NOV64 nucleic acid of the invention encoding a secreted protein-like protein includes the nucleic acid whose sequence is provided in Table 64A or a fragment thereof. The invention also includes a mutant or variant nucleic acid any of whose bases may be changed from the corresponding base shown in Table 64A while still encoding a protein that maintains its secreted protein-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 11 percent of the bases may be so changed.

The disclosed NOV64 protein of the invention includes the secreted protein-like protein whose sequence is provided in Table 64B. The invention also includes a mutant or variant protein any of whose residues may be changed from the corresponding residue shown in Table 64B while still encoding a protein that maintains its secreted protein-like activities and physiological functions, or a functional fragment thereof. In the mutant or variant protein, up to about 16 percent of the residues may be so changed.

The invention further encompasses antibodies and antibody fragments, such as F_{ab} or (F_{ab})₂, that bind immunospecifically to any of the proteins of the invention.

The above defined information for this invention suggests that this secreted protein-like protein (NOV64) may function as a member of a "secreted protein family". Therefore, the NOV64 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and in *vitro* of all tissues and cell types composing (but not limited to) those defined here.

The NOV64 nucleic acids and proteins of the invention are useful in potential therapeutic applications implicated in diseases including but not limited to various pathologies and disorders as indicated below. For example, a cDNA encoding the secreted protein-like protein (NOV64) may be useful in gene therapy, and the secreted protein-like protein (NOV64) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV64 nucleic acid encoding the secreted protein-like protein of the invention, or fragments thereof, may further be useful in diagnostic applications, wherein the presence or amount of the nucleic acid or the protein are to be assessed.

NOV64 nucleic acids and polypeptides are further useful in the generation of antibodies that bind immuno-specifically to the novel NOV64 substances for use in therapeutic or diagnostic methods. These antibodies may be generated according to methods known in the art, using prediction from hydrophobicity charts, as described in the "Anti-NOVX Antibodies" section below. The disclosed NOV64 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV65

A disclosed NOV65 nucleic acid of 3021 nucleotides (also referred to as CG57781-01) encoding a secreted protein-like protein.is shown in Table 65A. The start and stop codons are in bold letters.

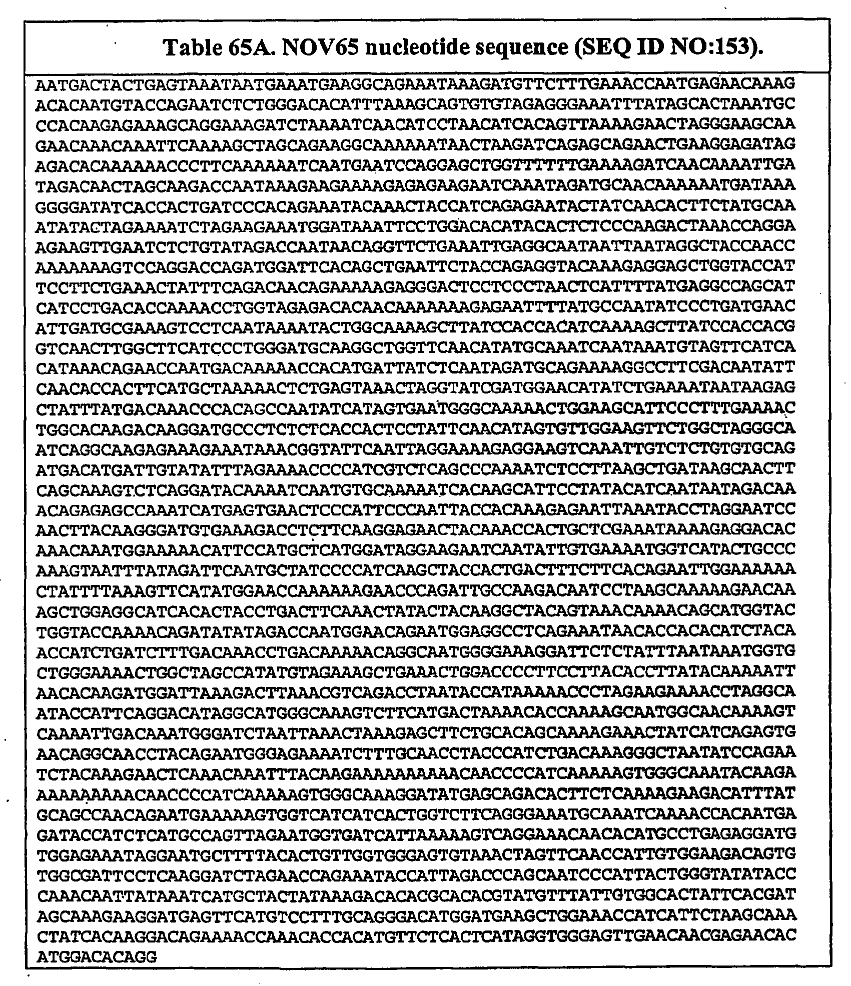

In a search of public sequence databases, the NOV65 nucleic acid sequence, located on chromsome 13 has 2524 of 2878 bases (87%) identical to a gb:GENBANK-ID:F229117S02|acc:AF229118.1 mRNA from Homo sapiens (Homo sapiens acetylcholinesterase collagen-like tail subunit (COLQ) gene, exons 1A, 2, 3, 4, and 5). Public nucleotide databases include all GenBank databases and the GeneSeq patent database.

The disclosed NOV65 polypeptide (SEQ ID NO:154) encoded by SEQ ID NO:153 has 990 amino acid residues and is presented in Table 65B using the one-letter amino acid code. Signal P, Psort and/or Hydropathy results predict that NOV65 has no signal peptide and is likely to be localized in the cytoplasm with a certainty of 0.7000.

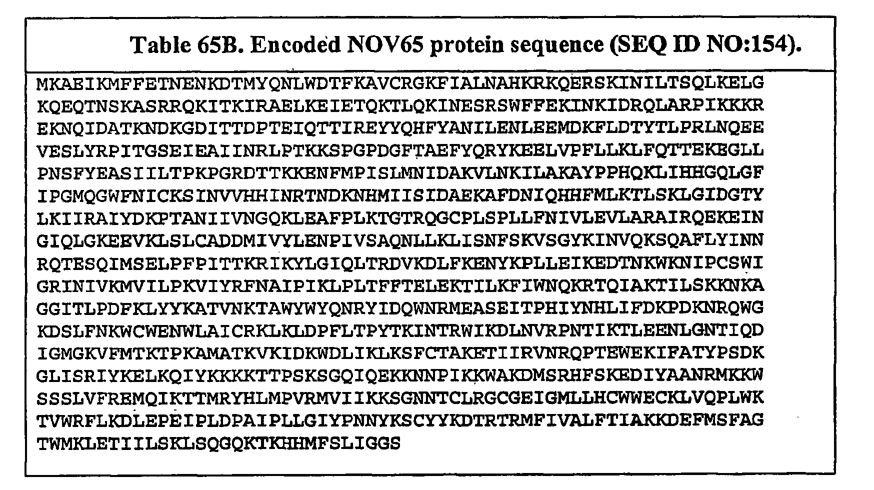

A search of sequence databases reveals that the NOV65 amino acid sequence has have 842 of 951 amino acid residues (88%) identical to, and 867 of 951 amino acid residues (91%) similar to, the 1010 amino acid residue patp:B38012 Human secreted protein encoded by gene 3 clone HNHCT15. Public amino acid databases include the GenBank databases, SwissProt, PDB and PIR.

The disclosed NOV65 polypeptide has homology to the amino acid sequences shown in the BLASTP data listed in Table 65C.

| **Table 65C. BLAST results for NOV65** | | | | | |
|---|---|---|---|---|---|
| Gene Index/ Identifier | Protein/ Organism | Length (aa) | Identity (%) | Positives (%) | Expect |
| gi\|106322\|pir\|\|B340 87 | protein (L1H 3' region) - human | 1280 | 885/1023 (86%) | 911/1023 (88%) | 0.0 |
| gi\|179B0447\|gb \|AAL5 0637.1 (AF421375) | unknown [Homo sapiens] | 1275 | 870/1023 (85%) | 904/1023 (88%) | 0.0 |
| gi\|2072948\|gb \|AAC51 261.1 (U93563) | p150 [Homo sapiens] | 1275 | 870/1023 (85%) | 903/1023 (88%) | 0.0 |
| gi\|2136112 \|pir\| \|S65 824 | reverse transcriptase homolog - human transposon L1.1 | 1275 | 868/1023 (84%) | 904/1023 (87%) | 0.0 |
| gi\|5052951\|gb\|AAD38 785.1\|AF149422 2 (AF149422) | unknown [Homo sapiens] | 1275 | 868/1023 (84%) | 904/1023 (87%) | 0.0 |

Table 65D lists the domain descriptions from DOMAIN analysis results against NOV65. This indicates that the NOV65 sequence has properties similar to those of other proteins known to contain this domain.

| **Table 65D. Domain Analysis of NOV65** | | |
|---|---|---|
| gnl\|Pfam\|pfam00078, rvt, Reverse transcriptase (RNA-dependent DNA polymerase). A reverse transcriptase gene is usually indicative of a mobile element such as a retrotransposon or retrovirus. Reverse transcriptases occur in a variety of mobile elements, including retrotransposons, retroviruses, group II introns, bacterial msDNAs, hepadnaviruses, and caulimoviruses. | | |
| | CD-Length = 208 residues, 97.6% aligned | |
| | | Score = 108 bits (269), Expect = 2e-24 |

The disclosed NOV65 nucleic acid of the invention encoding a secreted protein-like protein includes the nucleic acid whose sequence is provided in Table 65A or a fragment thereof. The invention also includes a mutant or variant nucleic acid any of whose bases may be changed from the corresponding base shown in Table 65A while still encoding a protein that maintains its secreted protein-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 13 percent of the bases may be so changed.

The disclosed NOV65 protein of the invention includes the secreted protein-like protein whose sequence is provided in Table 65B. The invention also includes a mutant or variant protein any of whose residues may be changed from the corresponding residue shown in Table 65B while still encoding a protein that maintains its secreted protein-like activities and physiological functions, or a functional fragment thereof. In the mutant or variant protein, up to about 12 percent of the residues may be so changed.

The invention further encompasses antibodies and antibody fragments, such as F_{ab} or (F_{ab})₂, that bind immunospecifically to any of the proteins of the invention.

The above defined information for this invention suggests that this secreted protein-like protein (NOV65) may function as a member of a "secreted protein family". Therefore, the NOV65 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV65 nucleic acids and proteins of the invention are useful in potential therapeutic applications implicated in diseases including but not limited to various pathologies and disorders as indicated below. For example, a cDNA encoding the secreted protein-like protein (NOV65) may be useful in gene therapy, and the secreted protein-like protein (NOV65) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV65 nucleic acid encoding the secreted protein-like protein of the invention, or fragments thereof, may further be useful in diagnostic applications, wherein the presence or amount of the nucleic acid or the protein are to be assessed.

NOV65 nucleic acids and polypeptides are further useful in the generation of antibodies that bind immuno-specifically to the novel NOV65 substances for use in therapeutic or diagnostic methods. These antibodies may be generated according to methods known in the art, using prediction from hydrophobicity charts, as described in the "Anti-NOVX Antibodies" section below. The disclosed NOV65 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV66

A disclosed NOV66 nucleic acid of 3120 nucleotides (also referred to as CG57783-01) encoding a secreted protein-like protein is shown in Table 66A. The start and stop codons are in bold letters.

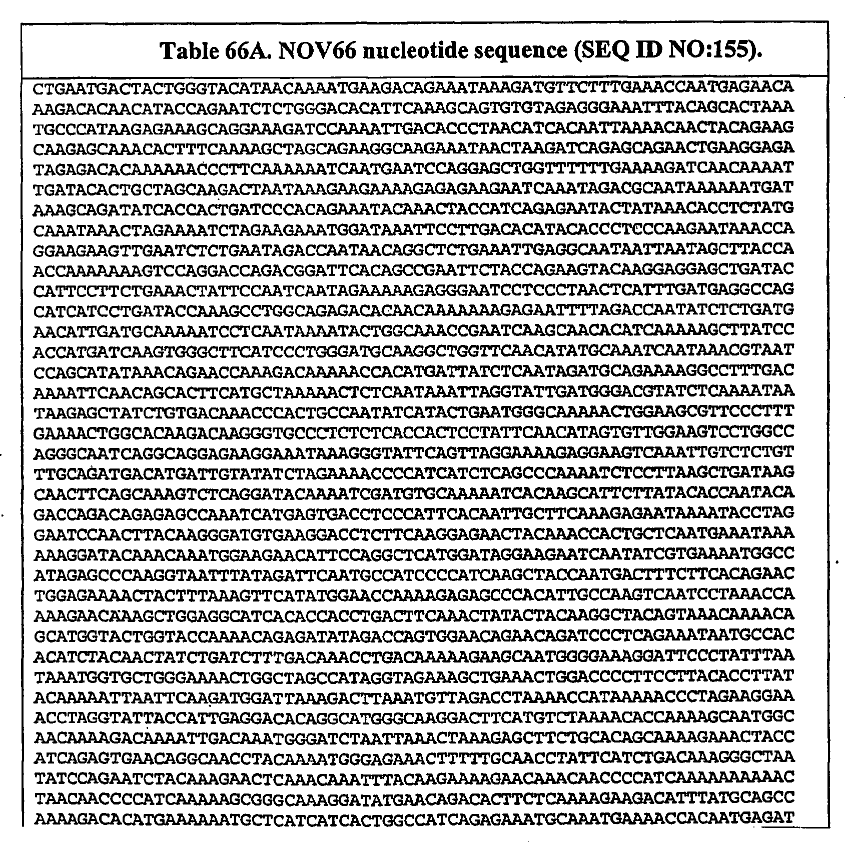

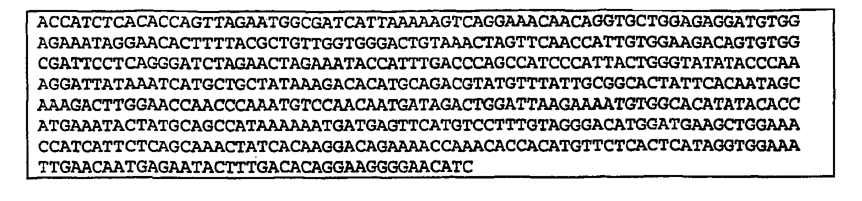

In a search of public sequence databases, the NOV66 nucleic acid sequence, located on chromsome 13 has 2399 of 2567 bases (93%) identical to a gb:GENBANK-ID:HSNODIG2|acc:AF149774.1 mRNA from Homo sapiens (Homo sapiens NOD1 protein (NOD1) gene, exons 4 through 14 and complete cds). Public nucleotide databases include all GenBank databases and the GeneSeq patent database.

The disclosed NOV66 polypeptide (SEQ ID NO:156) encoded by SEQ ID NO:155 has 1018 amino acid residues and is presented in Table 66B using the one-letter amino acid code. Signal P, Psort and/or Hydropathy results predict that NOV66 has no signal peptide and is likely to be localized in the cytoplasm with a certainty of 0.6000

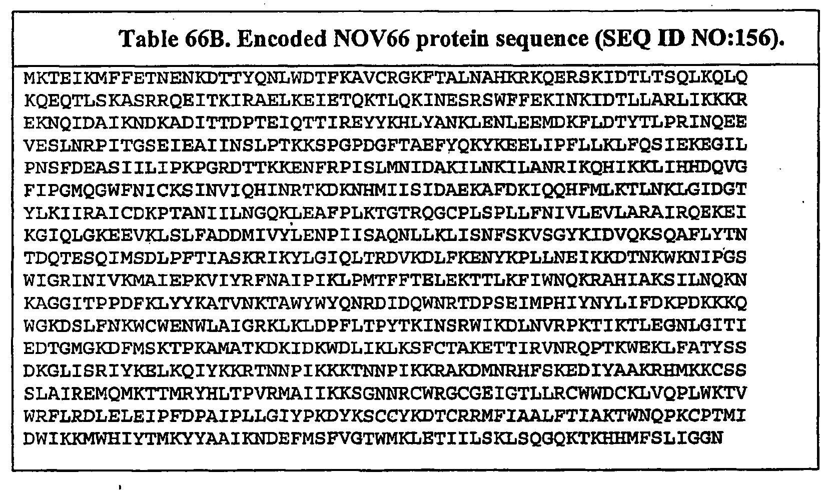

A search of sequence databases reveals that the NOV66 amino acid sequence has have 947 of 1018 amino acid residues (93%) identical to, and 965 of 1018 amino acid residues (94%) similar to, the 1010 amino acid residue patp:B38012 protein from human (Human secreted protein (L1H 3' region)). Public amino acid databases include the GenBank databases, SwissProt, PDB and PIR.

The disclosed NOV66 polypeptide has homology to the amino acid sequences shown in the BLASTP data listed in Table 66C.

| **Table 66C. BLAST results for NOV66** | | | | | |
|---|---|---|---|---|---|
| Gene Index/ Identifier | Protein/ Organism | Length (aa) | Identity (%) | Positives (%) | Expect |
| gil1063221pirlIB340 87 | protein (L1H 3' region) - human | 1280 | 955/1018 (93%) | 979/1018 (95%) | 0.0 |
| gi\|2072958\|gb\|AAC51 267.1\|(U93567) | putative p150 [Homo sapiens] | 1275 | 945/1018 (92%) | 971/1018 (94%) | 0.0 |
| gi\|5052951\|gb\|AAD38 785.1\|AF1.49422 2 (AF149422) | unknown [Homo sapiens] | 1275 | 948/1018 (93%) | 972/1018 (95%) | 0.0 |
| gi\|S070622\|gb\|AAD39 215.1\|AF148856 2 (AF148856) | unknown [Homo sapiens] | 1275 | 945/1018 (92%) | 973/1018 (94%) | 0.0 |
| gi\|2072953\|gb\|AAC51 264.1 (U93565) | p150 [Homo sapiens] | 1275 | 943/1018 (92%) | 971/1018 (94%) | 0.0 |

Table 66D lists the domain descriptions from DOMAIN analysis results against NOV66. This indicates that the NOV66 sequence has properties similar to those of other proteins known to contain this domain.

| **Table 66D. Domain Analysis of NOV66** |
|---|
| gn1\|Pfam\|pfam00078, rvt, Reverse transcriptase (RNA-dependent DNA polymerase). A reverse transcriptase gene is usually indicative of a mobile element such as a retrotransposon or retrovirus. Reverse transcriptases occur in a variety of mobile elements, including retrotransposons, retroviruses, group II introns, bacterial msDNAs, hepadnaviruses, and caulimoviruses. |
| CD-Length = 208 residues, 93.8% aligned |
| Score = 114 bits (285), Expect = 3e-26 |

The disclosed NOV66 nucleic acid of the invention encoding a secreted protein-like protein includes the nucleic acid whose sequence is provided in Table 66A or a fragment thereof. The invention also includes a mutant or variant nucleic acid any of whose bases may be changed from the corresponding base shown in Table 66A while still encoding a protein that maintains its secreted protein-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 7 percent of the bases may be so changed.

The disclosed NOV66 protein of the invention includes the secreted protein-like protein whose sequence is provided in Table 66B. The invention also includes a mutant or variant protein any of whose residues may be changed from the corresponding residue shown in Table 66B while still encoding a protein that maintains its secreted protein-like activities and physiological functions, or a functional fragment thereof. In the mutant or variant protein, up to about 7 percent of the residues may be so changed.

The invention further encompasses antibodies and antibody fragments, such as F_{ab} or (F_{ab})₂, that bind immunospecifically to any of the proteins of the invention.

The above defined information for this invention suggests that this secreted protein-like protein (NOV66) may function as a member of a "secreted protein family". Therefore, the NOV66 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV66 nucleic acids and proteins of the invention are useful in potential therapeutic applications implicated in diseases including but not limited to various pathologies and disorders as indicated below. For example, a cDNA encoding the secreted protein-like protein (NOV66) may be useful in gene therapy, and the secreted protein-like protein (NOV66) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV66 nucleic acid encoding the secreted protein-like protein of the invention, or fragments thereof, may further be useful in diagnostic applications, wherein the presence or amount of the nucleic acid or the protein are to be assessed.

NOV66 nucleic acids and polypeptides are further useful in the generation of antibodies that bind immuno-specifically to the novel NOV66 substances for use in therapeutic or diagnostic methods. These antibodies may be generated according to methods known in the art, using prediction from hydrophobicity charts, as described in the "Anti-NOVX Antibodies" section below. The disclosed NOV66 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV67

NOV67 includes two acyltransferase-like proteins disclosed below. The disclosed sequences have been named NOV67a and NOV67b.

### NOV67a

A disclosed NOV67a nucleic acid of 1116 nucleotides (also referred to as CG57823-01) encoding a acyltransferase-like protein is shown in Table 67A. The start and stop codons are in bold letters.

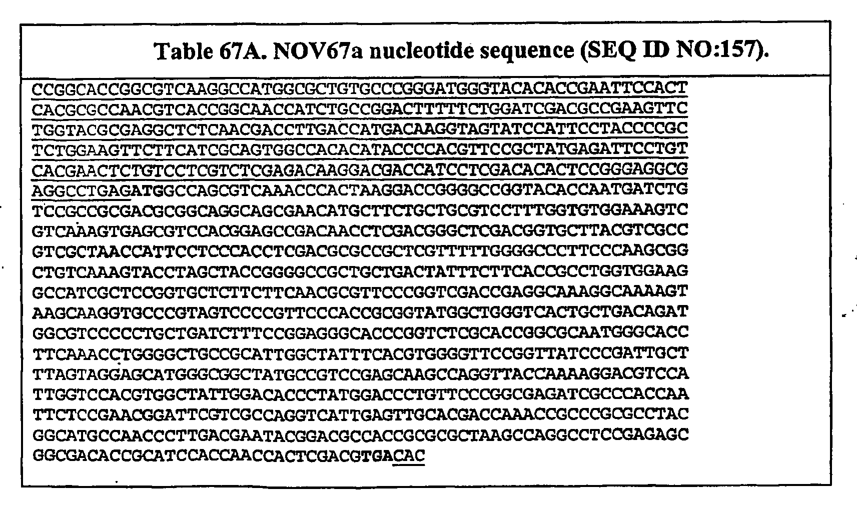

In a search of public sequence databases, the NOV67a nucleic acid sequence has 323 of 558 bases (57%) identical to a gb:GENBANK-ID:AF263912|acc:AF263912.1 mRNA from Streptomyces noursei (Streptomyces noursei ATCC 11455 nystatin biosynthetic gene cluster, complete sequence). Public nucleotide databases include all GenBank databases and the GeneSeq patent database.

The disclosed NOV67a polypeptide (SEQ ID NO:158) encoded by SEQ ID NO:157 has 267 amino acid residues and is presented in Table 67B using the one-letter amino acid code. Signal P, Psort and/or Hydropathy results predict that NOV67a has a signal peptide and is likely to be localized at the ER plasma membrane with a certainty of 0.85000. The most likely cleavage site for a NOV67a peptide is between amino acids 44 and 45.

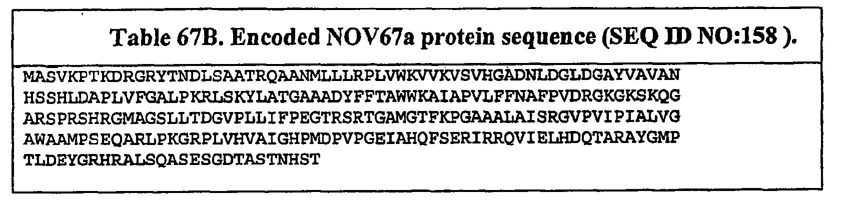

A search of sequence databases reveals that the NOV67a amino acid sequence has 65 of 181 amino acid residues (35%) identical to, and 96 of 181 amino acid residues (53%) similar to, the 240 amino acid residue ptnr:TREMBLNEW-ACC:CAC01452 protein from Streptomyces coelicolor (PUTATIVE ACYLTRANSFERASE). Public amino acid databases include the GenBank databases, SwissProt, PDB and PIR.

NOV67a is expressed in at least Bone, Bone Marrow, Brain, Liver, Lung, Lymph node, Placenta, Prostate, Thalamus, Thyroid and Uterus. This information was derived by determining the tissue sources of the sequences that were included in the invention including but not limited to SeqCalling sources, Public EST sources, Literature sources, and/or RACE sources.

### NOV67b

A disclosed NOV67b nucleic acid of 906 nucleotides (also referred to as CG57823-02) encoding a acyltransferase-like protein is shown in Table 67C. The start and stop codons are in bold letters.

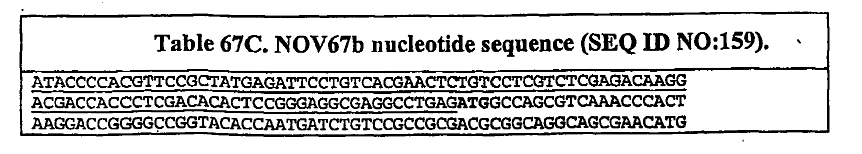

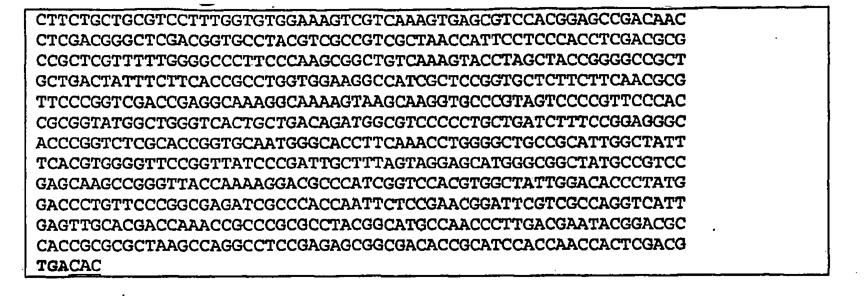

In a search of public sequence databases, the NOV nucleic acid sequence has 323 of 558 bases (57%) identical to a gb:GENBANK-ID:AF263912|acc:AF263912.1 mRNA from Streptomyces noursei (Streptomyces noursei ATCC 11455 nystatin biosynthetic gene cluster, complete sequence). Public nucleotide databases include all GenBank databases and the GeneSeq patent database.

The disclosed NOV67b polypeptide (SEQ ID NO:160) encoded by SEQ ID NO:159 has 267 amino acid residues and is presented in Table 67D using the one-letter amino acid code. Signal P, Psort and/or Hydropathy results predict that NOV67b has a signal peptide and is likely to be localized at the ER plasma membrane with a certainty of 0.85000. The most likely cleavage site for a NOV67b peptide is between amino acids 44 and 45.

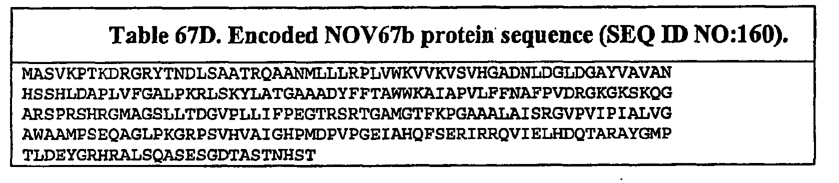

A search of sequence databases reveals that the NOV67b amino acid sequence has 65 of 181 amino acid residues (35%) identical to, and 96 of 181 amino acid residues (53%) similar to, the 240 amino acid residue ptnr:TREMBLNEW-ACC:CAC01452 protein from Streptomyces coelicolor (PUTATIVE ACYLTRANSFERASE). Public amino acid databases include the GenBank databases, SwissProt, PDB and PIR.

NOV67b is expressed in at least Bone, Bone Marrow, Brain, Liver, Lung, Lymph node, Placenta, Prostate, Thalamus, Thyroid, Uterus. This information was derived by determining the tissue sources of the sequences that were included in the invention including but not limited to SeqCalling sources, Public EST sources, Literature sources, and/or RACE sources.

The disclosed NOV67 polypeptide has homology to the amino acid sequences shown in the BLASTP data listed in Table 67E.

| **Table 67E. BLAST results for NOV67** | | | | | |
|---|---|---|---|---|---|
| Gene Index/ Identifier | Protein/ Organism | Length (aa) | Identity (%) | Positives (%) | Expect |
| gi\|15644441\|ref\|NP 229493.1\| (NC_000853) | 1-acyl-sn-glycerol-3-phosphate acetyltransferase putative [Thermotoga maritima] | 247 | 64/214 (29%) | 100/214 (45%) | 3e-14 |
| gi\|197161141\|emb \|CACO 1452.1 (AL391014) | putative acyltransferase [Streptomyces coelicolor A3(2)] | 240 | 63/184 (34%) | 92/184 (49%) | e-12 |
| gi\|11693120\|gb\|AAG3 8841.1\| (AY010120) | putative acetyl transferase [Xanthomonas oryzae pv. oryzae] | 249 | 68/203 (33%) | 94/203 (45%) | 4e-12 |
| gi\|15607028\|ref\|NP 214410.1 (NC_000918) | 2-acylglycerophosph oethanolamine acyltransferase [Aquifex aeolicus] | 211 | 59/199 (29%) | 92/199 (45%) | e-11 |
| gi\|15606303\|ref\|NP 213682.1 (NC_000918) | long-chain-fatty-acid CoA ligase [Aquifex aeolicus] | 823 | 47/149 (31%) | 72/149 (47%) | 3e-10 |

Tables 67F-G list the domain descriptions from DOMAIN analysis results against NOV67. This indicates that the NOV67 sequence has properties similar to those of other proteins known to contain this domain.

| **Table 67F. Domain Analysis of NOV67** |
|---|
| gn1\|Pfam\|pfam01553, Acyltransferase, Acyltransferase. This family contains acyltransferases involved in phospholipid biosynthesis and other proteins. This family also includes tafazzin, the Barth syndrome gene. |
| CD-Length = 185 residues, 95.7% aligned |
| Score = 92.4 bits (228), Expect = 3e-20 |

| **Table 67G. Domain Analysis of NOV67** |
|---|
| gn1\|Smart\|smart00563, PlsC, Phosphate acyltransferases; Function in phospholipid biosynthesis and have either glycerolphosphate, 1-acylglycerolphosphate, or 2-acylglycerolphosphoethanolamine acyltransferase activities. Tafazzin, the product of the gene mutated in patients with Barth syndrome, is a member of this family. |
| CD-Length = 118 residues, 99.2% aligned |
| Score = 81.6 bits (200), Expect = 5e-17 |

The polyene macrolide antibiotic nystatin produced by Streptomyces noursei ATCC 11455 is an important antifungal agent. The nystatin molecule contains a polyketide moiety represented by a 38-membered macrolactone ring to which the deoxysugar mycosamine is attached. Molecular cloning and characterization of the genes governing the nystatin biosynthesis is of considerable interest because this information can be used for the generation of new antifungal antibiotics.

Glycerol-3-phosphate 1-acyltransferase (E.C. 2.3.1.15; G3PAT) catalyses the incorporation of an acyl group from either acyl-acyl carrier proteins (acylACPs) or acylCoAs into the sn-1 position of glycerol 3-phosphate to yield 1-acylglycerol 3-phosphate. Crystals of squash G3PAT have been obtained by the hanging-drop method of vapour diffusion using PEG 4000 as the precipitant. These crystals are most likely to belong to space group P2(1)2(1)2(1), with approximate unit-cell parameters a = 61.1, b = 65.1, c =103.3 A, alpha = beta = gamma = 90 degrees and a monomer in the asymmetric unit X-ray diffraction data to 1.9 A resolution have been collected in-house using a MAR 345 imaging-plate system. PMID: 11223529.

Exposure of heparinized human plasma to gas-phase cigarette smoke produced a dose dependent reduction in the activity of platelet-activating factor acetylhydrolase (PAF-AH). Reductions of nearly 50% in PAF-AH activity were observed following exposure to gas-phase smoke from four cigarettes over an 8-h period. During this time of exposure, lecithin:cholesterol acyltransferase (LCAT) was rendered almost completely inactive (>80%). In contrast, paraoxonase was totally unaffected by cigarette smoke. Supplementation of plasma with 1 mM reduced glutathione was found to protect both PAF-AH and LCAT from cigarette smoke, suggesting that cysteine modifications may have contributed to the inhibition of these two enzymes.

Although the atheroprotective role of high-density lipoprotein (HDL) has been well documented in epidemiological and animal studies, highly effective therapeutic approaches for the selective increase of plasma HDL levels or function are not yet available. Several mechanisms by which HDL exerts an atheroprotective effect have been proposed on the basis of experiments in vitro and in vivo. These mechanisms include directing excess cellular cholesterol from the peripheral tissues to the liver in 'reverse cholesterol transport', inhibiting oxidative modification or aggregation of LDL, and modulating inflammatory responses to favour vasoprotection. High density lipoproteins (HDL) mediate reverse cholesterol transport as well as the clearance of oxidation

The disclosed NOV67 nucleic acid of the invention encoding a acyltransferase-like protein includes the nucleic acid whose sequence is provided in Table 67A or a fragment thereof. The invention also includes a mutant or variant nucleic acid any of whose bases may be changed from the corresponding base shown in Table 67A while still encoding a protein that maintains its acyltransferase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 43 percent of the bases may be so changed.

The disclosed NOV67 protein of the invention includes the acyltransferase-like protein whose sequence is provided in Table 67B. The invention also includes a mutant or variant protein any of whose residues may be changed from the corresponding residue shown in Table B while still encoding a protein that maintains its acyltransferase-like activities and physiological functions, or a functional fragment thereof. In the mutant or variant protein, up to about 65 percent of the residues may be so changed.

The invention further encompasses antibodies and antibody fragments, such as F_{ab} or (F_{ab})₂, that bind immunospecifically to any of the proteins of the invention.

The above defined information for this invention suggests that this acyltransferase-like protein (NOV67) may function as a member of a "acyltransferase family". Therefore, the NOV67 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV67 nucleic acids and proteins of the invention are useful in potential therapeutic applications implicated in diseases including but not limited to various pathologies and disorders as indicated below. For example, a cDNA encoding the acyltransferase-like protein (NOV67) may be useful in gene therapy, and the acyltransferase-like protein (NOV67) may be useful when administered to a subject in need thereof. By way of nonlimiting example, the compositions of the present invention will have efficacy for treatment of patients suffering from Osteoporosis,Hypercalceimia, Arthritis, Ankylosing spondylitis, Scoliosis , Hemophilia, hypercoagulation,Idiopathic thrombocytopenic purpura, autoimmume disease,allergies, inununodeficiencies,transplantation, Graft vesus host, Von Hippel-Lindau (VHL) syndrome, Cirrhosis,Transplantation , Lymphedema , Allergies, Hemophilia, hypercoagulation,Idiopathic thrombocytopenic purpura, immunodeficiencies, Fertility , Osteoporosis,Hypercalceimia, Arthritis, Ankylosing spondylitis, Scoliosis , Von Hippel-Lindau (VHL) syndrome, Alzheimer's disease, Stroke, Tuberous sclerosis, hypercalceimia, Parkinson's disease, Huntington's disease, Cerebral palsy, Epilepsy,Lesch-Nyhan syndrome, Multiple sclerosis,Ataxia-telangiectasia,Leukodystrophies,Behavioral disorders, Addiction, Anxiety, Pain, Neuroprotection, or other pathologies or conditions. The NOV67 nucleic acid encoding the acyltransferase-like protein of the invention, or fragments thereof, may further be useful in diagnostic applications, wherein the presence or amount of the nucleic acid or the protein are to be assessed.

NOV67 nucleic acids and polypeptides are further useful in the generation of antibodies that bind immuno-specifically to the novel NOV67 substances for use in therapeutic or diagnostic methods. These antibodies may be generated according to methods known in the art, using prediction from hydrophobicity charts; as described in the "Anti-NOVX Antibodies" section below. The disclosed NOV67 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV68

A disclosed NOV68 nucleic acid of 1388 nucleotides (also referred to as CG57801-01) encoding a guanine nucleotide exchange factor-like protein is shown in Table 68A. The start and stop codons are in bold letters.

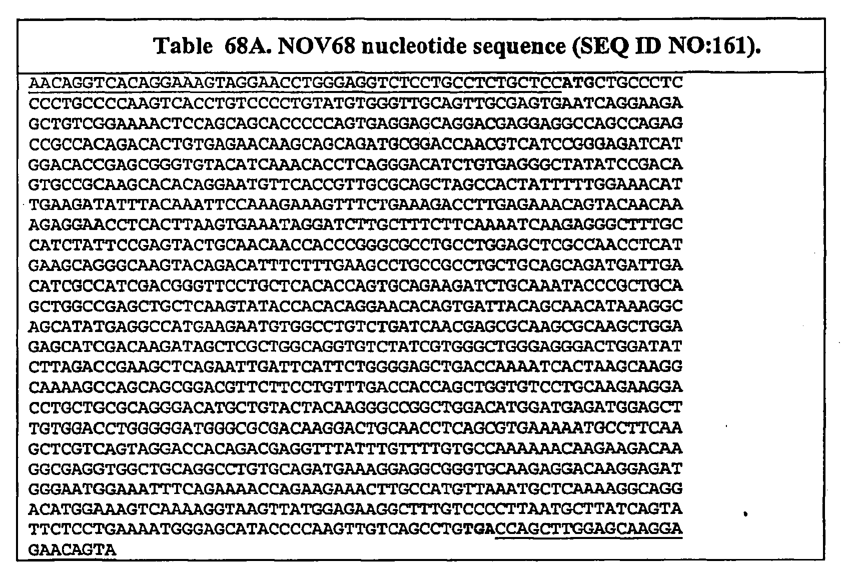

In a search of public sequence databases, the NOV68 nucleic acid sequence, located on chromsome 13 has has 822 of 1143 bases (71%) identical to a gb:GENBANK-ID:AB029035|acc:AB029035.1 mRNA from Homo sapiens (Homo sapiens mRNA for KIAA1112 protein, partial cds). Public nucleotide databases include all GenBank databases and the GeneSeq patent database.

The disclosed NOV68 polypeptide (SEQ ID NO:162) encoded by SEQ ID NO:161 has 437 amino acid residues and is presented in Table 68B using the one-letter amino acid code. Signal P, Psort and/or Hydropathy results predict that NOV68 has localized in the cytoplasm with a certainty of 0.3000.

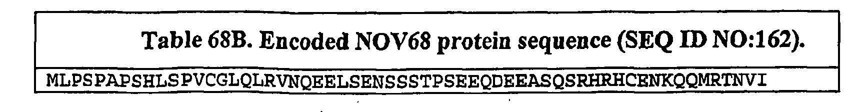

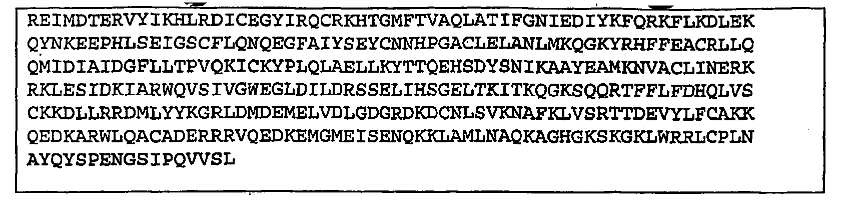

A search of sequence databases reveals that the NOV68 amino acid sequence has 253 of 402 amino acid residues (62%) identical to, and 317 of 402 amino acid residues (78%) similar to, the 493 amino acid residue ptnr:SPTREMBL-ACC:Q9QX73 protein from Rattus norvegicus (Rat) (COLLYBISTIN I). Public amino acid databases include the GenBank databases, SwissProt, PDB and PIR.

NOV68 is expressed in at least Kidney, Pituitary Gland, Placenta, Uterus, Aorta, Hypothalamus, Pancreas, Spleen, Epidermis, Muscle, Spinal Cord. This information was derived by determining the tissue sources of the sequences that were included in the invention including but not limited to SeqCalling sources, Public EST sources, Literature sources, and/or RACE sources.

The disclosed NOV68 polypeptide has homology to the amino acid sequences shown in the BLASTP data listed in Table 68C.

| **Table 68C. BLAST results for NOV68** | | | | | |
|---|---|---|---|---|---|
| Gene Index/ Identifier | Protein/ organism | Length (aa) | Identity (%) | Positives (%) | Expect |
| gi\|18581232\|ref\|XP 062774.2\| (XM_062774) | similar to Rho guanine nucleotide exchange factor 4, isoform a; APC-stimulated guanine nucleotide exchange factor [Homo sapiens] | 652 | 393/399 (98%) | 395/399 (98%) | 0.0 |
| gi\|8809845\|gb\|AAF79 955.1\| AF249745 1 (AF249745) | RhoGEF [Homo sapiens] | 720 | 256/429 (59%) | 319/429 (73%) | e-141 |
| gi\|5689561\|dbj\|BAA8 3064.1(AB029035) | KIAA1112 protein [Homo sapiens] | 694 | 250/402 (62%) | 311/402 (77%) | e-140 |
| gi \|13027402 \|ref \|NP 076447.1 (NM 023957) | collybistin I [Rattus norvegicus] | 493 | 254/407 (62%) | 318/407 (77%) | e-140 |
| gi\|7662108\|ref\|NP 0 56000.1\| (NM_015185) | Cdc42 guanine exchange factor 9; Cdc42 guanine exchange factor (GEF) 9 [Homo sapiens] | 516 | 250/397 (62%) | 312/397 (77%) | e-140 |

Table 68D-E lists the domain descriptions from DOMAIN analysis results against NOV68. This indicates that the NOV68 sequence has properties similar to those of other proteins known to contain this domain.

| **Table 68D. Domain Analysis of NOV68** |
|---|
| gn1\|Smart\|smart00325, RhoGEF, Guanine nucleotide exchange factor for Rho/Rac/Cdc42-like GTPases; Guanine nucleotide exchange factor for Rho/Rac/Cdc42-like GTPases Also called Dbl-homologous (DH) domain. It appears that PH domains invariably occur C-terminal to RhoGEF/DH domains. Improved coverage. |
| CD-Length = 181 residues, 100.0% aligned |
| Score = 156 bits (394), Expect = 3e-39 |

| **Table 68E. Domain Analysis of NOV68** | |
|---|---|
| gn1lSmartlSmartl00233, PH, Pleckstrin homology domain.; Domain commonly found in eukaryotic signalling proteins. The domain family possesses multiple functions including the abilities to bind inositol phosphates, and various proteins. PH domains have been found to possess inserted domains (such as in PLC gamma, syntrophins) and to be inserted within other domains. Mutations in Brutons tyrosine kinase (Btk) within its PH domain cause X-linked agammaglobulinaemia (XLA) in patients. Point mutations cluster into the positively charged end of the molecule around the predicted binding site for phosphatidylinositol lipids. | |
| | CD-Length = 104 residues, 92.3% aligned |
| | Score = 56.2 bits (134), Expect = 4e-09 |

The novel protein described in this application belongs to the guanine nucleotide exchange factor family of proteins which play a significant role in signal transduction. The guanine nucleotide exchange factor (GEF) domain that regulates GTP binding protein signaling. The GEF domain regulates positively the signaling cascades that utilize GTP-binding proteins (such as those of the ras superfamily) that function as molecular switches in fundamental events such as signal transduction, cytoskeleton dynamics and intracellular trafficking. Experiments have shown that the GEF and (PH) domains of FGD1 (faciogenital dyplasia protein (FGD1)) can bind specifically to the Rho family GTPase Cdc42Hs and stimulates the GDP-GTP exchange of the isoprenylated form of Cdc42Hs. The GEF domain of FGD1 has also been shown to activate 2 kinases involved in cell proliferation; the Jun NH2-terminal kinase and the p70 S6 kinase (See Zheng et. al.; J. Biol. Chem 1996 Dec 27;271(52):33169-72). Thus this novel protein may play an important role in normal development as well as disease. This class of molecules (GEFs) is also being considered as a good drug target as the guanine nucleotide exchange factor RasGRP is a high -affinity target for diacylglycerol and phorbol esters and is bound by bryostatin 1, a compound currently in clinical trials (See Lorenzo et. al.; Mol. Pharmacol 2000 May;57(5):840-6). Collybistin I and II, which belong to the family of dbl-like GDP/GTP exchange factors (GEFs) are most homologous to the protein described in this application. Collybistin II regulates the membrane deposition of gephyrin (an integral membrane protein) by activating a GTPase of the Rho/Rac family and may be an important determinant of inhibitory postsynaptic membrane formation and plasticity (See Kins et. al. Nat. Neurosci 2000 Jan;3(1):22-9).

The disclosed NOV68 nucleic acid of the invention encoding a guanine nucleotide exchange factor-like protein includes the nucleic acid whose sequence is provided in Table 68A or a fragment thereof. The invention also includes a mutant or variant nucleic acid any of whose bases may be changed from the corresponding base shown in Table 68A while still encoding a protein that maintains its guanine nucleotide exchange factor-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 29 percent of the bases may be so changed.

The disclosed NOV68 protein of the invention includes the guanine nucleotide exchange factor-like protein whose sequence is provided in Table 68B. The invention also includes a mutant or variant protein any of whose residues may be changed from the corresponding residue shown in Table 68B while still encoding a protein that maintains its guanine nucleotide exchange factor-like activities and physiological functions, or a functional fragment thereof. In the mutant or variant protein, up to about 38 percent of the residues may be so changed.

The invention further encompasses antibodies and antibody fragments, such as F_{ab} or (F_{ab})_{2,} that bind immunospecifically to any of the proteins of the invention.

The above defined information for this invention suggests that this guanine nucleotide exchange factor-like protein (NOV68) may function as a member of a "guanine nucleotide exchange factor family". Therefore, the NOV68 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV68 nucleic acids and proteins of the invention are useful in potential therapeutic applications implicated in diseases including but not limited to various pathologies and disorders as indicated below. For example, a cDNA encoding the guanine nucleotide exchange factor-like protein (NOV68) may be useful in gene therapy, and the guanine nucleotide exchange factor-like protein (NOV68) may be useful when administered to a subject in need thereof. By way of nonlinuting example, the compositions of the present invention will have efficacy for treatment of patients suffering from cancer,trauma, regeneration (in vitro and in vivo), viral/bacterial/parasitic infections, diabetes, autoimmune disease, renal artery stenosis, interstitial nephritis, glomerulonephritis, polycystic kidney disease, systemic lupus erythematosus, renal tubular acidosis, IgA nephropathy, hypercalceimia, Lesch-Nyhan syndrome, Von Hippel-Lindau (VHL) syndrome, Alzheimer's disease, stroke, tuberous sclerosis, hypercalceimia, Parkinson's disease, Huntington's disease, cerebral palsy, epilepsy, Lesch-Nyhan syndrome, multiple sclerosis, ataxia-telangiectasia, leukodystrophies, behavioral disorders, addiction, anxiety, pain, neurodegeneration, muscular dystrophy, myasthenia gravis, atherosclerosis, aneurysm, hypertension, fibromuscular dysplasia, stroke, scleroderma, obesity, transplantation, or other pathologies or conditions. The NOV68 nucleic acid encoding the guanine nucleotide exchange factor-like protein of the invention, or fragments thereof, may further be useful in diagnostic applications, wherein the presence or amount of the nucleic acid or the protein are to be assessed.

NOV68 nucleic acids and polypeptides are further useful in the generation of antibodies that bind immuno-specifically to the novel NOV68 substances for use in therapeutic or diagnostic methods. These antibodies may be generated according to methods known in the art, using prediction from hydrophobicity charts, as described in the "Anti-NOVX Antibodies" section below. The disclosed NOV68 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV69

NOV69 includes two aspartate aminotransferase-like proteins disclosed below. The disclosed sequences have been named NOV69a and NOV69b.

### NOV69a

A disclosed NOV60a nucleic acid of 1463 nucleotides (also referred to as CG57719-01) encoding a aspartate aminotransferase-like protein is shown in Table 69A. The start and stop codons are in bold letters.

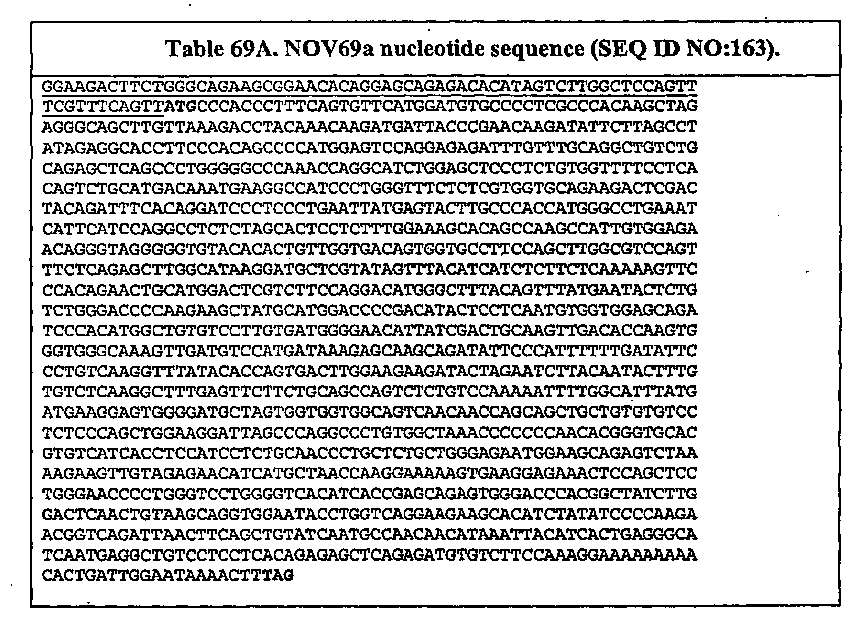

In a search of public sequence databases, the NOV69a nucleic acid sequence, located on chromsome 8 has 316 of 327 bases (96%) identical to a gb:GENBANK-ID:AP000501| acc:AP000501.1 mRNA from Homo sapiens (Homo sapiens genomic DNA, chromosome 8p11.2, clone:91h23 to 9-41). Public nucleotide databases include all GenBank databases and the GeneSeq patent database.

The disclosed NOV69a polypeptide (SEQ ID NO: 164) encoded by SEQ ID NO:163 has 463 amino acid residues and is presented in Table 69B using the one-letter amino acid code. Signal P, Psort and/or Hydropathy results predict that NOV69a has no signal peptide and is likely to be localized in the cytoplasm with a certainty of 0.3696.

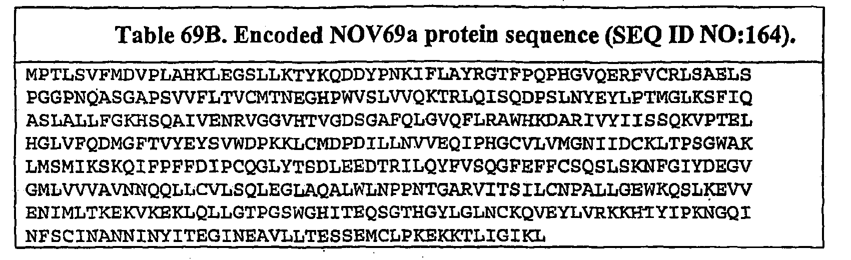

A search of sequence databases reveals that the NOV69a amino acid sequence has 163 of 228 amino acid residues (71%) identical to, and 187 of 228 amino acid residues (82%) similar to, the 264 amino acid residue ptnr:TREMBLNEW-ACC:BAB24820 protein from Mus musculus (Mouse) (ADULT MALE TESTIS CDNA, RIKEN FULL-LENGTH ENRICHED LIBRARY, CLONE: 1700083M11, FULL INSERT SEQUENCE). Public amino acid databases include the GenBank databases, SwissProt, PDB and PIR.

NOV69a is expressed in at least testis. This information was derived by determining the tissue sources of the sequences that were included in the invention including but not limited to SeqCalling sources, Public EST sources, Literature sources, and/or RACE sources.

### NOV69b

A disclosed NOV69b nucleic acid of 1280 nucleotides (also referred to as CG57719-02) encoding a aspartate aminotransferase-like protein is shown in Table 69C. The start and stop codons are in bold letters.

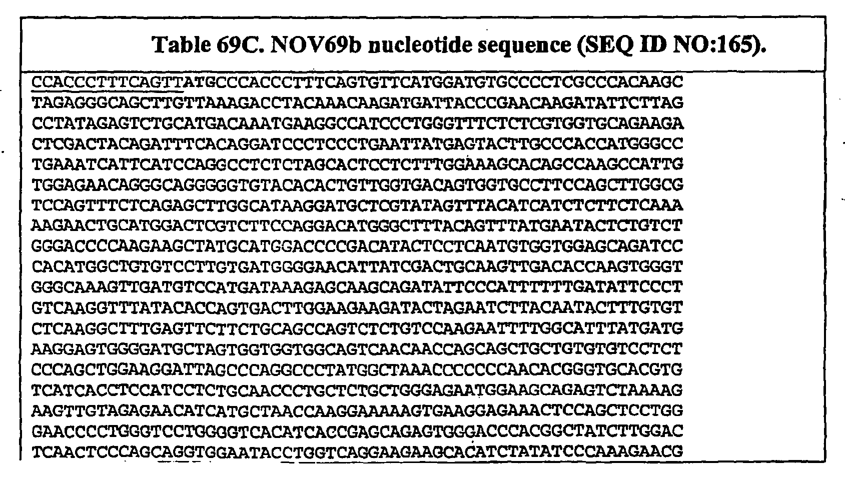

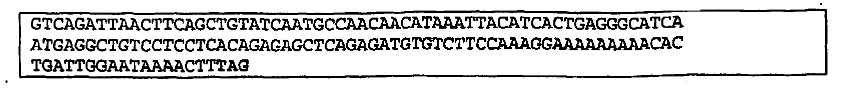

In a search of public sequence databases, the NOV69b nucleic acid sequence, located on chromsome 8 has has 401 of 620 bases (64%) identical to a gb:GENBANK-ID:RATCASP AT|acc:D00252.1 mRNA from Rattus norvegicus (Rattus norvegicus mRNA for cytosolic aspartate aminotransferase, complete cds). Public nucleotide databases include all GenBank databases and the GeneSeq patent database.

The disclosed NOV69b polypeptide (SEQ ID NO:166) encoded by SEQ ID NO:165 has 421 amino acid residues and is presented in Table 69D using the one-letter amino acid code. Signal P, Psort and/or Hydropathy results predict that NOV69b has no signal peptide and is likely to be localized in the cytoplasm with a certainty of 0.3645.

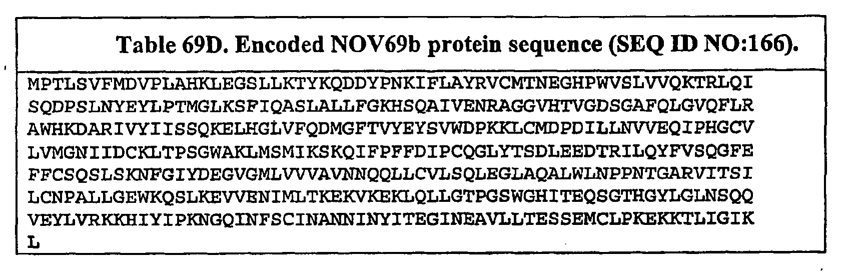

A search of sequence databases reveals that the NOV69b amino acid sequence has have 163 of 405 amino acid residues (40%) identical to, and 236 of 405 amino acid residues (58%) similar to, the 412 amino acid residue ptnr:SWISSNEW-ACC:P17174 protein from Homo sapiens (Human) (ASPARTATE AMINOTRANSFERASE, CYTOPLASMIC (EC 2.6.1.1) (TRANSAMINASE A) (GLUTAMATE OXALOACETATE TRANSAMINASE-1)). Public amino acid databases include the GenBank databases, SwissProt, PDB and PIR.

NOV69b is expressed in at least testis. This information was derived by determining the tissue sources of the sequences that were included in the invention including but not limited to SeqCalling sources, Public EST sources, Literature sources, and/or RACE sources.

The disclosed NOV69b polypeptide has homology to the amino acid sequences shown in the BLASTP data listed in Table 69E.

| **Table 69E. BLAST results for NOV69b** | | | | | |
|---|---|---|---|---|---|
| Gene Index/ Identifier | Protein/ Organism | Length (aa) | Identity (%) | Positives (%) | Expect |
| gi\|12840318\|dbj\|BAB 24820.1\| (AK006984) | homolog to ASPARTATE AMINOTRANSFERASE, CYTOPLASMIC (EC 2.6.1.1) (TRANSAMINASE A) (GLUTAMATE OXALOACETATE TRANSAMINASE-1)-putative [Mus musculus] | 264 | 185/296 (62%) | 185/296 (62%) | e-102 |
| gi\|345752\|pir\|\|S290 28 | aspartate transaminase (EC 2.6.1.1) (clone 8C7) - human | 413 | 224/374 (59%) | 224/374 (59%) | 2e-80 |
| gi\|91997\|pir\|JT043 9 | aspartate transaminase (EC 2.6.1.1), cytosolic - rat | 413 | 155/374 (41%) | 224/374 (59%) | 2e-80 |
| >gi\|105387\|pir\|\|S13 035 | aspartate transaminase (EC 2.6.1.1) - human | 412 | 155/371 (41%) | 222/371 (59%) | 3e-80 |
| gi\|6754034\|ref\|NP 0 34454.1\| (NM_010324) | glutamate oxaloacetate transaminase 1, soluble; cytosolic aspartate aminotransferase [Mus musculus] | 412 | 154/369 (41%) | 223/369 (59%) | 4e-80 |

Table 69F-G lists the domain descriptions from DOMAIN analysis results against NOV69b. This indicates that the NOV69b sequence has properties similar to those of other proteins known to contain this domain.

| **Table 69F. Domain Analysis of NOV69b** |
|---|
| gn1\|Pfam\|pfam00873, ACR_tran, AcrB/AcrD/AcrF family. Members of this family are integral membrane proteins. Some are involved in drug resistance. |
| CD-Length = 1020 residues, 15.0% aligned |
| Score = 51.2 bits (121), Expect = 4e-07 |

| **Table 69G. Domain Analysis of NOV69b** |
|---|
| gnl\|Pfam\|pfam02460, Patched, Patched family. The transmembrane protein Patched is a receptor for the morphogene Sonic Hedgehog. This protein associates with the smoothened protein to transduce hedgehog signals. |
| CD-Length = 821 residues, 31.4% aligned |
| Score = 46.6 bits (109), Expect = 1e-05 |

Concentrations of glutamate, aspartate and glycine are significantly increased in epileptogenic cerebral cortex. The activities of the enzymes, glutamate dehydrogenase and aspartate aminotransferase, involved in glutamate and aspartate metabolism are also increased.

Polyamine synthesis is enhanced in epileptogenic cortex and may contribute to the activation of N-methyl-D-aspartate (NMDA) Receptors (See Sherwin AL (1999). Neurochem Res 24(11):1387-95). Nuclear magnetic resonance spectroscopy (NMRS) reveals that patients with poorly controlled complex partial seizures have a significant diminution in occipital lobe gamma aminobutyric acid (GABA) concentration. The activity of the enzyme GABA-aminotransaminase (GABA-T) which catalyzes GABA degradation is not altered in epileptogenic cortex. NMRS studies show that vigabatrin, a GABA-T inhibitor and effective antiepileptic, significantly increases brain ABA. Glutamate decarboxylase (GAD), responsible for GABA synthesis, is diminished in intemeurons in discrete regions of epileptogenic cortex and hippocampus. In vivo microdialysis performed in epilepsy surgery patients provides measurements of extracellular amino acid levels during spontaneous seizures. Glutamate concentrations are higher in epileptic hippocampi and increase berore seizure onset reaching potentially excitotoxic levels. Frontal or temporal cortical epileptogenic foci also release aspartate, glutamate and serine particularly during intense seizures or status epilepticus. GABA in contrast, exhibits a delayed and feeble rise in the epileptic hippocampus possibly due to a reduction in the number and/or efficiency of GABA transporters. In additon, aspartate aminotransferase activity is an important index for liver function. Abnormal level and activity of aspartate aminotransferase correlates diseased liver conditions, e.g., hepatitis (See Gopal et al., (2000). Postgrad Med 107(2):100-2,105-9, 113-4; Vesely et al., (1999). Am J Med Sci 317(6):419-24; Johnston DE (1999). Am Fam Phys 59(8):2223-30; Johnston SC, Pelletier LL (1997). Medicine (Baltimore) 76(3):185-91). Finally, aspartate aminotransferase activity is also a marker for diagnosis of cardiovascular diseases (See Wu AH (1999). Ann Clin Lab Sci 29(1):18-23) and periodontal disease (See Eley BM, Cox SW (1998). Br Dent J 184(9):427-30). Therefore, aspartate aminotransferase is an excellent small molecule target and diagnostic marker for epilepsis, liver diseases, cardiovascular and periodontal diseases.

The disclosed NOV69 nucleic acid of the invention encoding a aspartate aminotransferase-like protein includes the nucleic acid whose sequence is provided in Table 69A or 69C or a fragment thereof. The invention also includes a mutant or variant nucleic acid any of whose bases may be changed from the corresponding base shown in Table 69A or 69C while still encoding a protein that maintains its aspartate aminotransferase-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 36 percent of the bases may be so changed.

The disclosed NOV69 protein of the invention includes the aspartate aminotransferase-like protein whose sequence is provided in Table 69B. The invention also includes a mutant or variant protein any of whose residues may be changed from the corresponding residue shown in Table 69B while still encoding a protein that maintains its aspartate aminotransferase-like activities and physiological functions, or a functional fragment thereof. In the mutant or variant protein, up to about 60 percent of the residues may be so changed.

The invention further encompasses antibodies and antibody fragments, such as F_{ab} or (F_{ab})₂, that bind immunospecifically to any of the proteins of the invention.

The above defined information for this invention suggests that this aspartate aminotransferase-like protein (NOV69) may function as a member of a "aspartate aminotransferase family". Therefore, the NOV69 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV69 nucleic acids and proteins of the invention are useful in potential therapeutic applications implicated in diseases including but not limited to various pathologies and disorders as indicated below. For example, a cDNA encoding the aspartate aminotransferase-like protein (NOV69) may be useful in gene therapy, and the aspartate aminotransferase-like protein (NOV69) may be useful when administered to a subject in need thereof. By way of nonlimiting example, the compositions of the present invention will have efficacy for treatment of patients suffering from fertility, hypogonadism" or other pathologies or conditions. The NOV69 nucleic acid encoding the aspartate aminotransferase-like protein of the invention, or fragments thereof, may further be useful in diagnostic applications, wherein the presence or amount of the nucleic acid or the protein are to be assessed.

NOV69 nucleic acids and polypeptides are further useful in the generation of antibodies that bind immuno-specifically to the novel NOV69 substances for use in therapeutic or diagnostic methods. These antibodies may be generated according to methods known in the art, using prediction from hydrophobicity charts, as described in the "Anti-NOVX Antibodies" section below. The disclosed NOV69 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV70

A disclosed NOV70 nucleic acid of 4915 nucleotides (also referred to as CG-57462-01) encoding a KIAA1337-like protein is shown in Table 70A. The start and stop codons are in bold letters.

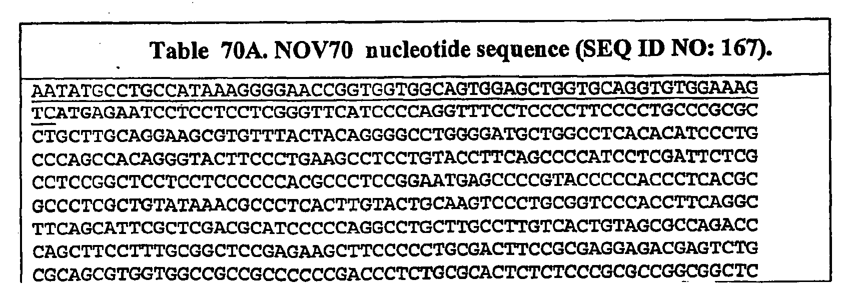

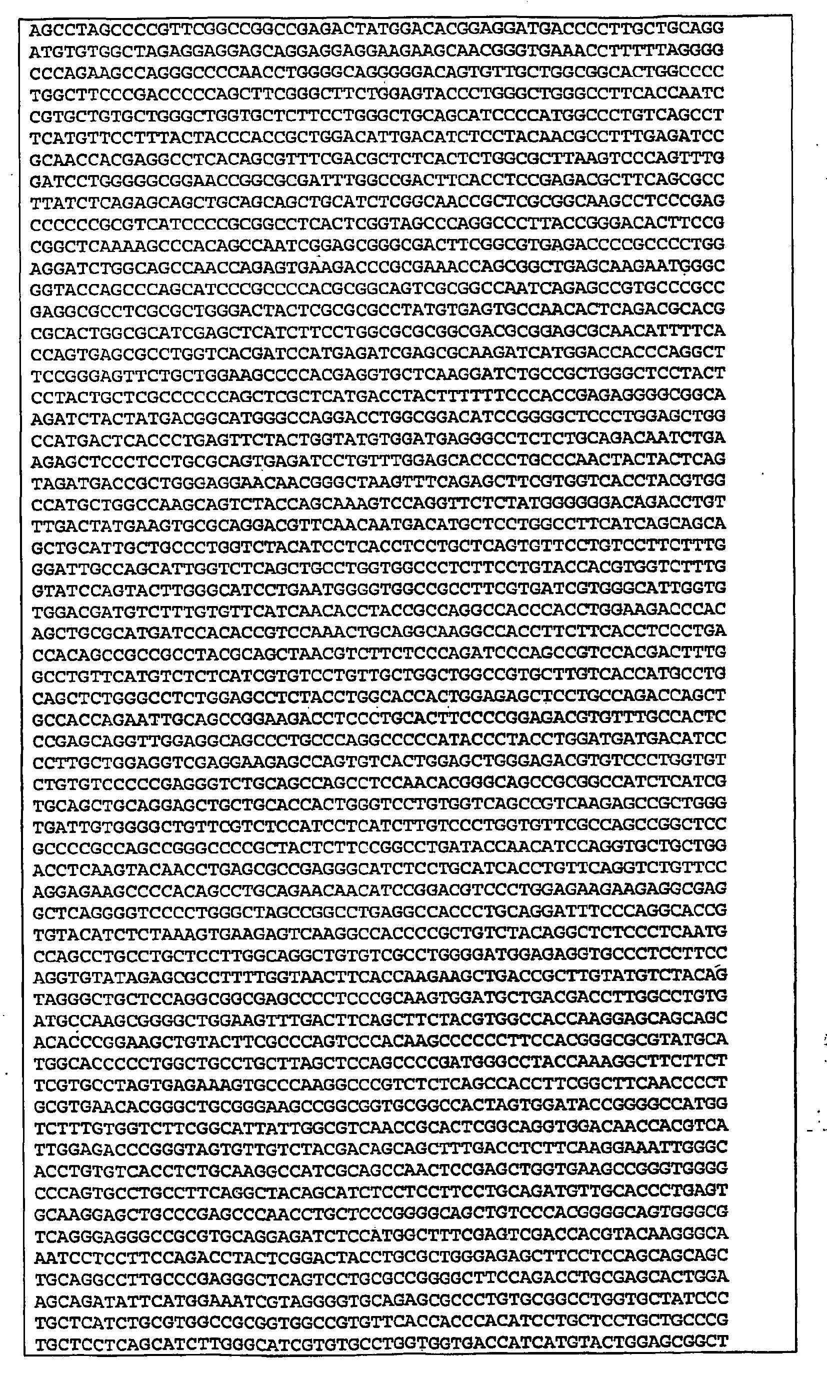

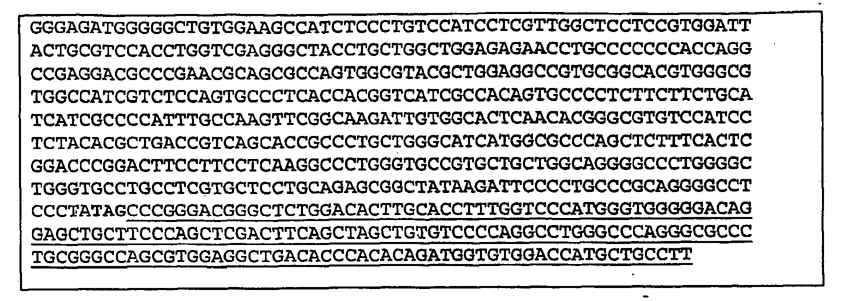

In a search of public sequence databases, the NOV70 nucleic acid sequence, located on chromsome 1 has 4481 of 4484 bases (99%) identical to a gb:GENBANK-ID:AB037758|acc:AB037758.1 mRNA from Homo sapiens (Homo sapiens mRNA for KIAA1337 protein, partial cds). Public nucleotide databases include all GenBank databases and the GeneSeq patent database.

The disclosed NOV70 polypeptide (SEQ ID NO: 168) encoded by SEQ ID NO:167 has 1561 amino acid residues and is presented in Table 70B using the one-letter amino acid code. Signal P, Psort and/or Hydropathy results predict that NOV70 has a signal peptide and is likely to be localized plasma membrane with a certainty of 0.8000. The most likely cleavage site for a NOV70 peptide is between amino acids 18 and 19.

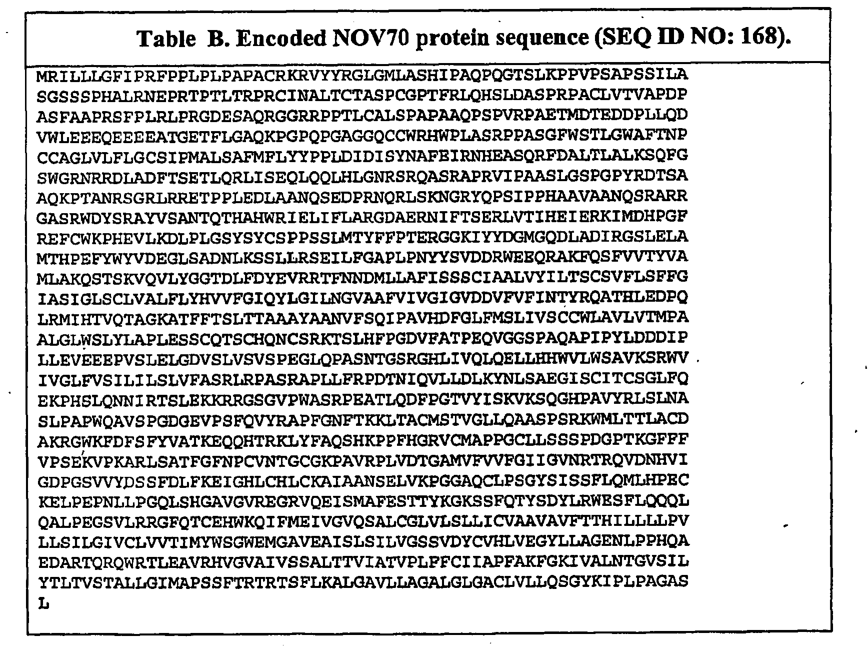

A search of sequence databases reveals that the NOV70 amino acid sequence has 1436 of 1438 amino acid residues (99%) identical to, and 1436 of 1438 amino acid residues (99%) similar to, the 1438 amino acid residue ptnr:SPTREMBL-ACC:Q9P2K9 protein from Homo sapiens (Human) (KIAA1337 PROTEIN). Public amino acid databases include the GenBank databases, SwissProt, PDB and PIR.

NOV70 is expressed in at least Brain, Cerebral Medulla/Cerebral white matter, hippocampus, Hypothalamus, Left cerebellum, Lung, Parietal Lobe, Testis, and Right Cerebellum. This information was derived by determining the tissue sources of the sequences that were included in the invention including but not limited to SeqCalling sources, Public EST sources, Literature sources, and/or RACE sources.

The disclosed NOV70 polypeptide has homology to the amino acid sequences shown in the BLASTP data listed in Table 70C.

| **Table 70C. BLAST results for NOV70** | | | | | |
|---|---|---|---|---|---|
| Gene Index/ Identifier | Protein/ Organism | Length (aa) | Identity (%) | Positives (%) | Expect |
| gi\|7243055\|dbj\|BAA9 2575.1\| (AB037758) | KIAA1337 protein [Homo sapiens] | 1438 | 1436/1438 (99%) | 1436/1438 (99%) | 0.0 |
| gi\|17448847\|ref\|XP 052561.4 (XM_052561) | KIAA1337 protein [Homo sapiens] | 1392 | 1388/1392 (99%) | 1388/1392 (99%) | 0.0 |
| gi\|5B34578\|emb\|CAB5 5303.1 (AL117236) | hypothetical protein [Homo sapiens] | 594 | 592/594 (99%) | 592/594 (99%) | 0.0 |
| gi\|17448809\|ref\|XP 052559.31 (XM 052559) | Similar to KIAA1337 protein [Homo sapiens] | 383 | 361/363 (99%) | 361/363 (99%) | 0.0 |
| gi\|18545186\|ref\|XP 046122.2\| (XM-046122) | protein MGC13130 [Homo sapiens] | 1524 | 54/177 (30%) | 89/177 (49%) | 5e-14 |

Tables 70D-E list the domain descriptions from DOMAIN analysis results against NOV70 . This indicates that the NOV70 sequence has properties similar to those of other proteins known to contain this domain.

| **Table 70D. Domain Analysis of NOV70** | |
|---|---|
| gnl\|Pfam\|pfam00873, ACR_tran, AcrB/AcrD/AcrF family. Members of this family are integral membrane proteins. Some are involved in drug resistance. | |
| | CD-Length = 1020 residues, 15.0% aligned |
| | Score = 51.2 bits (121), Expect = 4e-07 |

| **Table 70E. Domain Analysis of NOV70** |
|---|
| gnl\|Pfam\|pfam02460, Patched, Patched family. The transmembrane protein Patched is a receptor for the morphogene Sonic Hedgehog. This protein associates with the smoothened protein to transduce hedgehog signals. |
| CD-Length = 821 residues, 31.4% aligned |
| Score = 46.6 bits (109), Expect = le-05 |

The Drosophila 'patched' (ptc) gene encodes a transmembrane protein that represses transcription in specific cells of genes encoding members of the TGF beta (see 190180) and Wnt (164820) families of signaling proteins. Vertebrate homologs of ptc have been identified in mice, chickens, and zebra fish. The human PTC protein is predicted to contain 12 hydrophobic membrane-spanning domains and 2 large hydrophilic extracellular loops. Johnson et al. (1996) mapped the human PTC gene to chromosome 9q22.3 by radiation hybrid analysis.

The murine PTCH homolog, Ptc, maps to mouse chromosome 13. Pte maps close to the murine Face locus (0 recombinants in 188 meioses). They noted that mouse mutations such as flexed tail (f), purkinje cell degeneration (pcd), and mesenchymal dysplasia (mes) which involve abnormal development of skeletal and neural tissues are also located in this region of chroinosome 13 and may be allelic to Ptc. The 2 extracellular loops of the Ptc protein are necessary for binding and that binding also requires that the Ptc protein be glycosylated. Marigo et al. (1996) proposed that Ptc does not carry out signaling to the cell directly but that an additional molecule is involved, namely the 7-transmembrane protein 'Smoothened' (SMO; 601500). The Ptc gene encodes a candidate receptor for Shh by showing that epitope-tagged N-Shh binds specifically to human embryonic kidney 293 cells expressing mouse Ptc.

Basal cell carcinoma, medulloblastoma, rhabdomyosarcoma, and other human tumors are associated with mutations that activate the protooncogene 'Smoothened' or that inactivate the tumor suppressor 'Patched.' Smoothened and Patched mediate the cellular response to the Hedgehog secreted protein signal, and oncogenic mutations affecting these proteins cause excess activity of the Hedgehog response pathway.

Approximately 5% of patients with Gorlin syndrome develop medulloblastoma in the first few years of life, and 10% of patients with medulloblastoma diagnosed at age 2 years or under have Gorlin syndrome. Cowan et al. (1997) found that 1 out of 3 unrelated patients with medulloblastoma complicated by Gorlin syndrome had lost the wildtype allele on 9q, indicating that the Gorlin locus probably acts a tumor suppressor in the development of this tumor. They also confirmed this role in a basal cell carcinoma from the same individual. Studying patients who presented with multiple odontogenic keratocysts, Lench et al. (1997) identified 5 novel germline mutations in PTCH. Four mutations caused premature stop codons and 1 resulted in an amino acid substitution toward the C terminus of the predicted protein.

The disclosed NOV70 nucleic acid of the invention encoding a KIAA1337-like protein includes the nucleic acid whose sequence is provided in Table 70A or a fragment thereof. The invention also includes a mutant or variant nucleic acid any of whose bases may be changed from the corresponding base shown in Table 70A while still encoding a protein that maintains its KIAA1337-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1 percent of the bases may be so changed.

The disclosed NOV70 protein of the invention includes the KIAA1337-like protein whose sequence is provided in Table 70B. The invention also includes a mutant or variant protein any of whose residues may be changed from the corresponding residue shown in Table 70B while still encoding a protein that maintains its KIAA1337-like activities and physiological functions, or a functional fragment thereof. In the mutant or variant protein, up to about 1 percent of the residues may be so changed.

The invention further encompasses antibodies and antibody fragments, such as F_{ab} or (F_{ab})₂, that bind immunospecifically to any of the proteins of the invention.

The above defined information for this invention suggests that this KIAA1337-like protein (NOV70) may function as a member of a "KIAA1337 family". Therefore, the NOV70 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV70 nucleic acids and proteins of the invention are useful in potential therapeutic applications implicated in diseases including but not limited to various pathologies and disorders as indicated below. For example, a cDNA encoding the KIAA1337-like protein (NOV70) may be useful in gene therapy, and the KIAA1337-like protein (NOV70) may be useful when administered to a subject in need thereof. By way of nonlimiting example, the compositions of the present invention will have efficacy for treatment of patients suffering from Von Hippel-Lindau (VHL) syndrome, Alzheimer's disease, Stroke, Tuberous sclerosis, hypercalceimia, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection, Endocrine dysfunctions, Diabetes, obesity, Growth, Systemic lupus erythematosus, Autoimmune disease, Asthma, Emphysema, Scleroderma, allergy, Fertility, ARDS, Pharyngitis, Laryngitis, Myasthenia gravis,, or other pathologies or conditions. The NOV70 nucleic acid encoding the KIAA1337-like protein of the invention, or fragments thereof, may further be useful in diagnostic applications, wherein the presence or amount of the nucleic acid or the protein are to be assessed.

NOV70 nucleic acids and polypeptides are further useful in the generation of antibodies that bind immuno-specifically to the novel NOV70 substances for use in therapeutic or diagnostic methods. These antibodies may be generated according to methods known in the art, using prediction from hydrophobicity charts, as described in the "Anti-NOVX Antibodies" section below. The disclosed NOV70 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV71

A disclosed NOV71 nucleic acid of 2004 nucleotides (also referred to as CG57584-01) encoding a zona pellucida glycoprotein 1 precursor-like protein is shown in Table 71A. The start and stop codons are in bold letters.

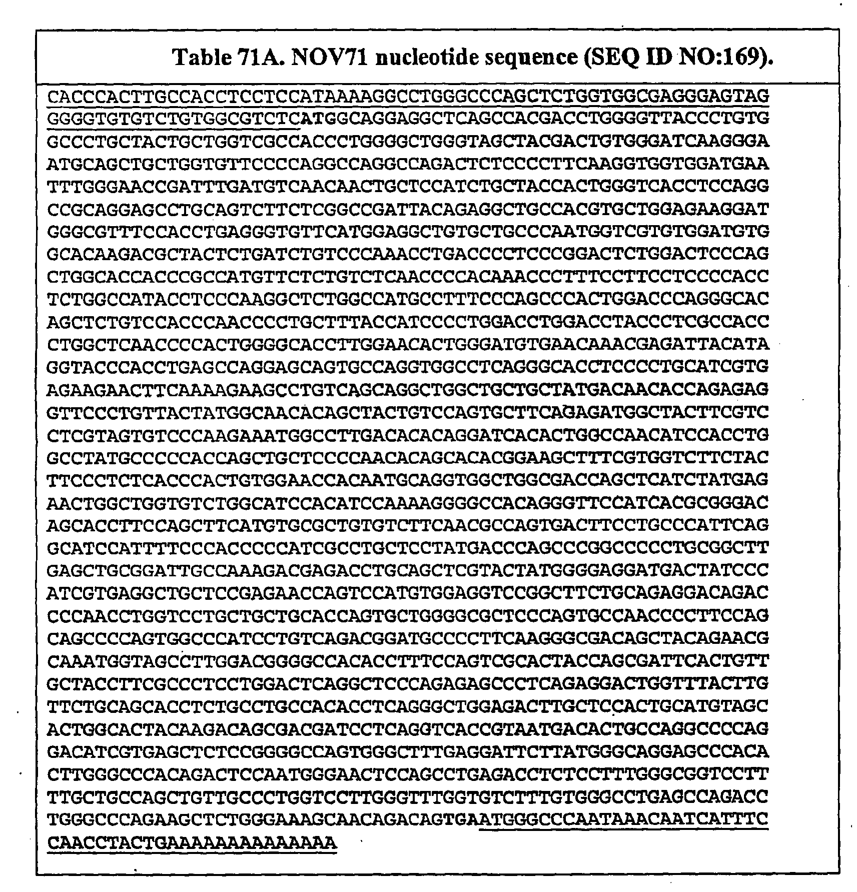

In a search of public sequence databases, the NOV71 nucleic acid sequence, located on chromsome 11 has 1305 of 1704 bases (76%) identical to a gb:GENBANK ID:MOZP1|acc:U20448.1 mRNA from Mus musculus (Mus musculus ZP1 precursor (Zp-1) mRNA, complete cds). Public nucleotide databases include all GenBank databases and the GeneSeq patent database.

The disclosed NOV71 polypeptide (SEQ ID NO:170) encoded by SEQ ID NO:169 has 624 amino acid residues and is presented in Table 71B using the one-letter amino acid code. Signal P, Psort and/or Hydropathy results predict that NOV71 has a signal peptide and is likely to be localized localized at the plasma membrane with a certainty of 0.4600. The most likely cleavage site for a NOV71 peptide is between amino acids 25 and 26.

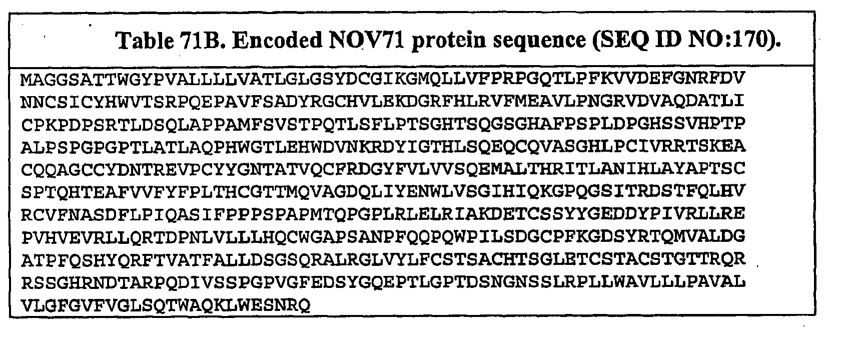

A search of sequence databases reveals that the NOV71 amino acid sequence has 422 of 620 amino acid residues (68%) identical to, and 477 of 620 amino acid residues (76%) similar to, the 623 amino acid residue ptnr:SPTREMBL-ACC:Q62016 protein from Mus musculus (Mouse) (ZONA PELLUCIDA GLYCOPROTEIN 1 PRECURSOR (ZP1)). Public amino acid databases include the GenBank databases, SwissProt, PDB and PIR.

NOV71 is expressed in at least Kidney, Pituitary Gland, Testis, Whole Organism. Expression information was derived from the tissue sources of the sequences that were included in the derivation of the sequence of CG57584-01. The sequence is predicted to be expressed in the following tissues because of the expression pattern of (GENBANK-ID: gb:GENBANK-ID:MOZP1|acc:U20448.1) a closely related Mus musculus ZP1 precursor (Zp-1) mRNA, complete cds homolog in species Mus musculus :ovary. This information was derived by determining the tissue sources of the sequences that were included in the invention including but not limited to SeqCalling sources, Public EST sources, Literature sources, and/or RACE sources.

The disclosed NOV71 polypeptide has homology to the amino acid sequences shown in the BLASTP data listed in Table 71C.

| **Table 71C. BLAST results for NOV71** | | | | | |
|---|---|---|---|---|---|
| Gene Index/ Identifier | Protein/ Organism | Length (aa) | Identity (%) | Positives (%) | Expect |
| gi\|17460872\|ref\|XP 062198.1\| (XM_062198) | similar to zona pellucida glycoprotein 1 (H. sapiens) | 496 | 458/458 (100%) | 458/458 (100%) | 0.0 |
| gi\|6677653\|ref\|NP 0 33606.1\| (NM_009580) | zona pellucida glycoprotein 1 [Mus musculus] | 623 | 416/628 (66%) | 471/628 (74%) | 0.0 |
| gi\|1113794\|gb\|AAB60 507.1 (U24230) | zona pellucida [Mus musculus] | 623 | 414/628 (65%) | 470/628 (73%) | 0.0 |
| gi\|16758240\|ref\|NP 445961.1 (NM_053509 | zona pellucida glycoprotein 1 [Rattus norvegicus] | 617 | 416/621 (66%) | 469/621 (74%) | 0.0 |
| gi\|11140012\|emb\|CAC 16087.1 (AJ289697) | zona pellucida protein 1 [Gallus gallus] | 934 | 197/369 (53%) | 254/369 (68%) | 2E-97 |

Tables 71D-E list the domain descriptions from DOMAIN analysis results against NOV71. This indicates that the NOV71 sequence has properties similar to those of other proteins known to contain this domain.

| **Table 71D. Domain Analysis of NOV71** |
|---|
| gn1\|Pfam\|pfam00100, zona_pellucida, Zona pellucida-like domain. |
| CD-Length = 266 residues, 99.6% aligned |
| Score = 209 bits (533), Expect = 3e-55 |

| **Table 71E. Domain Analysis of NOV71** | | |
|---|---|---|
| gnl\|Smart\|smart 00018, P, P or trefoil or TFF domain; Proposed role in renewal and pathology of mucous epithelia | | |
| | CD-Length = 47 residues, 100.0% aligned | |
| | | Score = 39.7 bits (91), Expect = 5e-04 |

The mammalian zona pellucida is composed of 3 major glycoproteins, ZP1, ZP2 (182888), and ZP3 (182889). ZP3, the molecule responsible for the major sperm-receptor activity of the zona, plays a significant role in fertilization. ZP2 is implicated as a secondary sperm receptor that binds sperm only after the induction of the sperm acrosome reaction. See review by Dean (1992). The mature ZP 1, ZP2, and ZP3 proteins have molecular weights of 90-110 kD, 64-76 kD, and 57-73 kD, respectively.

The disclosed NOV71 nucleic acid of the invention encoding a zona pellucida glycoprotein 1 precursor-like protein includes the nucleic acid whose sequence is provided in Table 71A or a fragment thereof. The invention also includes a mutant or variant nucleic acid any of whose bases may be changed from the corresponding base shown in Table 71A while still encoding a protein that maintains its zona pellucida glycoprotein 1 precursor-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 24 percent of the bases may be so changed.

The disclosed NOV71 protein of the invention includes the zona pellucida glycoprotein 1 precursor-like protein whose sequence is provided in Table 71B. The invention also includes a mutant or variant protein any of whose residues may be changed from the corresponding residue shown in Table 71B while still encoding a protein that maintains its zona pellucida glycoprotein 1 precursor-like activities and physiological functions, or a functional fragment thereof. In the mutant or variant protein, up to about 32 percent of the residues may be so changed.

The invention further encompasses antibodies and antibody fragments, such as F_{ab} or (F_{ab})₂, that bind immunospecifically to any of the proteins of the invention.

The above defined information for this invention suggests that this zona pellucida glycoprotein 1 precursor-like protein (NOV71) may function as a member of a "zona pellucida glycoprotein 1 precursor family". Therefore, the NOV7I nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV71 nucleic acids and proteins of the invention are useful in potential therapeutic applications implicated in diseases including but not limited to various pathologies and disorders as indicated below. For example, a cDNA encoding the zona pellucida glycoprotein 1 precursor-like protein (NOV71) may be useful in gene therapy, and the zona pellucida glycoprotein 1 precursor-like protein (NOV71) may be useful when administered to a subject in need thereof. By way of nonlimiting example, the compositions of the present invention will have efficacy for treatment of patients suffering from Cardiomyopathy, Atherosclerosis, Hypertension, Congenital heart defects, Aortic stenosis , Atrial septal defect (ASD), Atrioventricular (A-V) canal defect, Ductus arteriosus , Pulmonary stenosis , Subaortic stenosis, Ventricular septal defect (VSD), valve diseases, Tuberous sclerosis, Scleroderma, Obesity, Transplantation, Aneurysm, Fibromuscular dysplasia, Stroke, Anemia, Bleeding disorders, Adrenoleukodystrophy , Congenital Adrenal Hyperplasia, Diabetes, Von Hippel-Lindau (VHL) syndrome, Pancreatitis, Hyperparathyroidism, Hypoparathyroidism, SIDS, Endometriosis, Fertility, Xerostomia, Hypercalceimia, Ulcers, Cirrhosis, Inflammatory bowel disease, Diverticular disease, Hirschsprung's disease, Crohn's Disease, Appendicitis, Hemophilia, hypercoagulation, Idiopathic thrombocytopenic purpura, autoimmume disease, allergies, immunodeficiencies, Graft vesus host, Ataxia-telangiectasia, Hemophilia, Lymphedema, Tonsilitis, Osteoporosis, Arthritis, Ankylosing spondylitis, Scoliosis, Tendinitis, Muscular dystrophy, Lesch-Nyhan syndrome, Myasthenia gravis, Dental disease and infection, Alzheimer's disease, Tuberous sclerosis, Parkinson's disease, Huntington's disease, Cerebral palsy, Epilepsy, Lesch-Nyhan syndrome, Multiple sclerosis, Ataxia-telangiectasia, Leukodystrophies, Behavioral disorders, Addiction, Anxiety, Pain, Neuroprotection, Growth and reproductive disorders, Endocrine dysfunctions, Systemic lupus erythematosus , Asthma, Emphysema, ARDS, Pharyngitis, Laryngitis, Hearing loss, Tinnitus, Psoriasis, Actinic keratosis ,Tuberous sclerosis, Acne, Hair growth, allopecia, pigmentation disorders, cystitis, incontinence, Renal artery stenosis, Interstitial nephritis, Glomerulonephritis, Polycystic kidney disease, Systemic lupus erythematosus, Renal tubular acidosis, IgA nephropathy, Vesicoureteral reflux, glaucoma, blindness, and Hypothyroidism, or other pathologies or conditions. The NOV71 nucleic acid encoding the zona pellucida glycoprotein 1 precursor-like protein of the invention, or fragments thereof, may further be useful in diagnostic applications, wherein the presence or amount of the nucleic acid or the protein are to be assessed.

NOV71 nucleic acids and polypeptides are further useful in the generation of antibodies that bind immuno-specifically to the novel NOV71 substances for use in therapeutic or diagnostic methods. These antibodies may be generated according to methods known in the art, using prediction from hydrophobicity charts, as described in the "Anti-NOVX Antibodies" section below. The disclosed NOV71 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV72

A disclosed NOV72 nucleic acid of 6108 nucleotides (also referred to as CG56761-01) encoding an ankyrin repeat containing protein-like protein is shown in Table 72A. The start and stop codons are in bold letters.

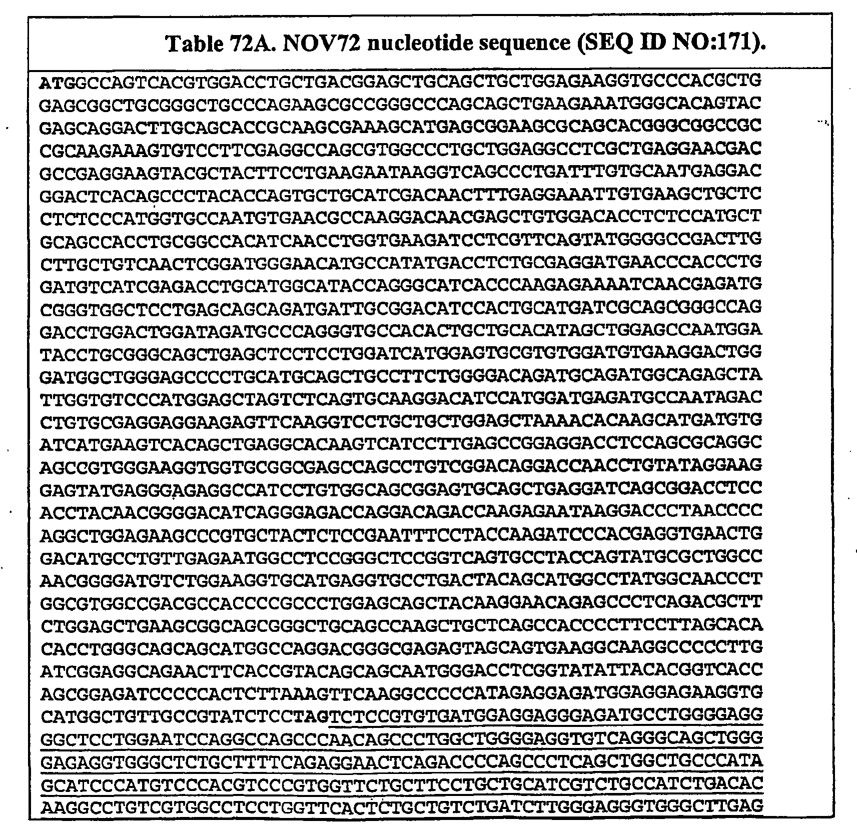

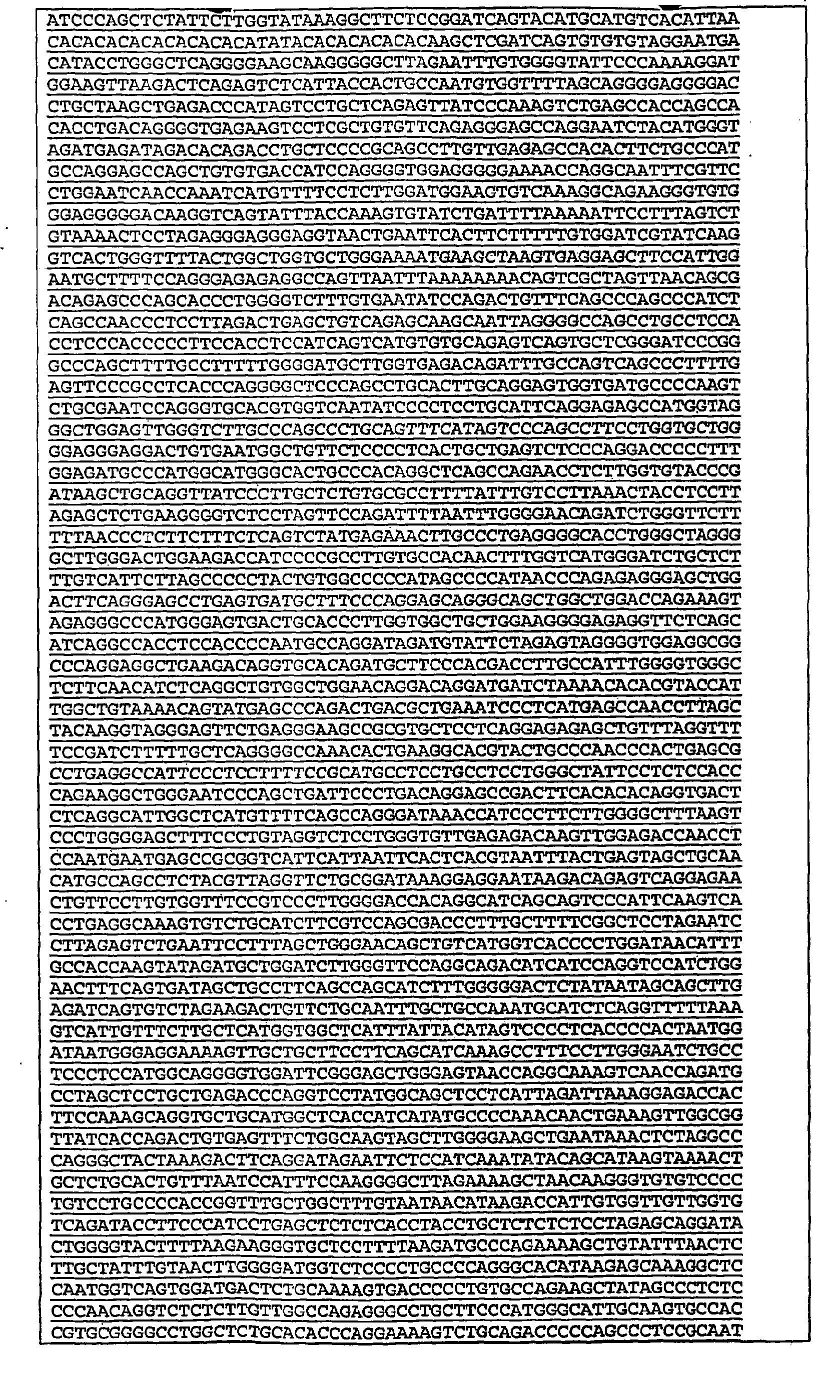

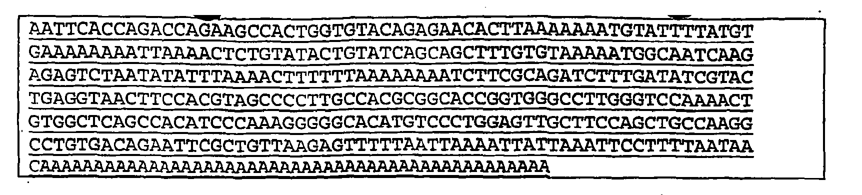

In a search of public sequence databases, the NOV72 nucleic acid sequence, located on chromsome 20 has 5593 of 5597 bases (99%) identical to a gb:GENBANK-ID:AB020630|acc:AB020630.1 mRNA from Homo sapiens (Homo sapiens mRNA for KIAA0823 protein, partial cds). Public nucleotide databases include all GenBank databases and the GeneSeq patent database.

The disclosed NOV72 polypeptide (SEQ ID N0:172) encoded by SEQ ID N0:171 has 567 amino acid residues and is presented in Table 72B using the one-letter amino acid code. Signal P, Psort and/or Hydropathy results predict that NOV72 has no signal peptide and is likely to be localized in the nucleus with a certainty of 0.9800.

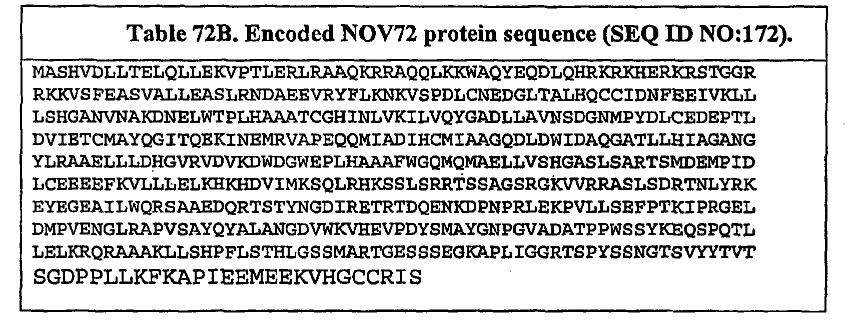

A search of sequence databases reveals that the NOV72 amino acid sequence has have 412 of 412 amino acid residues (100%) identical to, and 412 of 412 amino acid residues (100%) similar to, the 412 amino acid residue ptnr:SPTREMBL-ACC:094912 protein from Homo sapiens (Human) (KIAA0823 PROTEIN). Public amino acid databases include the GenBank databases, SwissProt, PDB and PIR.

NOV72 is expressed in at least Adrenal Gland/Suprarenal gland, Amygdala, Artery, Bone, Brain, Dermis, Heart, Hippocampus, Kidney, Lung, Lymph node, Lymphoid tissue, Mammary gland/Breast, Pancreas, Peripheral Blood, Small Intestine, Spleen, Stomach, Substantia Nigra, Synovium/Synovial membrane, Thalamus, Tonsils, Umbilical Vein, Urinary Bladder; and Uterus. This information was derived by determining the tissue sources of the sequences that were included in the invention including but not limited to SeqCalling sources, Public EST sources, Literature sources, and/or RACE sources.

The disclosed NOV72 polypeptide has homology to the amino acid sequences shown in the BLASTP data listed in Table 72C.

| **Table 72C. BLAST results for NOV72** | | | | | |
|---|---|---|---|---|---|
| Gene Index/ Identifier | Protein/ Organism | Length (aa) | Identity (%) | Positives (%) | Expect |
| gi\|14770818\|ref\|XP 028840.1\| (XM_028840) | KIAA0823 protein [Homo sapiens] | 567 | 567/567 (100%) | 567/567 (100%) | 0.0 |
| gi\|14029700\|gb\|AAK5 2795.1\|AF362909 1 (AF362909 | CAAX box protein TIMAP [Bos taurus] | 568 | 550/568 (96%) | 557/568 (97%) | 0.0 |
| gi \|18157719\|gb\|AAL6 2093.1\|AP423761 1 (AF423761) | protein phosphatase 1 regulatory subunit 16B [Mus musculus] | 568 | 547/568 (96%) | 556/568 (97%), | 0.0 |
| gi\|4240132\|dbj\|BAA7 4846.1\| (AB020630 | KIAA0823 protein [Homo sapiens] | 412 | 412/412 (100%) | 412/412 (100%) | 0.0 |
| gi\|9368796\|emb\|CAB9 8284.1\| (AL121889) | dJ1076E17.1 (KIAA0823 protein (continues in AL023803)) [Homo sapiens] | 411 | 411/411 (100%) | 411/411 (100%) | 0.0 |

Table 72D lists the domain descriptions from DOMAIN analysis results against NOV72 . This indicates that the NOV72 sequence has properties similar to those of other proteins known to contain this domain.

| **Table 72D. Domain Analysis of NOV72** | | |
|---|---|---|
| gnl\|pfam\|pfam00023, ank, Ank repeat. Ankyrin repeats generally consist of a beta, alpha, alpha, beta order of secondary structures. The repeats associate to form a higher order structure. | | |
| | CD-Length = 33 residues, 100.0% aligned | |
| | | Score = 47.4 bits (111), Expect = 2e-06 |

Ankyrin repeats are tandemly repeated modules of about 33 amino acids. They occur in a large number of functionally diverse proteins mainly from eukaryotes. The few known examples from prokaryotes and viruses may be the result of horizontal gene transfers. The conserved fold of the ankyrin repeat unit is known from several crystal and solution structures, e.g. from: p53-binding protein 53BP2, cyclin-dependent kinase inhibitor p19Ink4d, transcriptional regulator GABP-beta, and NF-kappaB inhibitory protein IkB-alpha. It has has been described as an L-shaped structure consisting of a beta-hairpin and two alpha-helices. Many ankyrin repeat regions are known to function as protein-protein interaction domains.

The disclosed NOV72 nucleic acid of the invention encoding a ankyrin repeat containing protein-like protein includes the nucleic acid whose sequence is provided in Table 72A or a fragment thereof. The invention also includes a mutant or variant nucleic acid any of whose bases may be changed from the corresponding base shown in Table 72A while still encoding a protein that maintains its ankyrin repeat containing protein-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 1 percent of the bases may be so changed.

The disclosed NOV72 protein of the invention includes the ankyrin repeat containing protein-like protein whose sequence is provided in Table 72B. The invention also includes a mutant or variant protein any of whose residues may be changed from the corresponding residue shown in Table 72B while still encoding a protein that maintains its ankyrin repeat containing protein-like activities and physiological functions, or a functional fragment thereof. In the mutant or variant protein, up to about 0 percent of the residues may be so changed.

The invention further encompasses antibodies and antibody fragments, such as F_{ab} or (F_{ab})₂, that bind immunospecifically to any of the proteins of the invention.

The above defined information for this invention suggests that this ankyrin repeat containing protein-like protein (NOV72) may function as a member of a "ankyrin repeat containing protein family". Therefore, the NOV72 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV72 nucleic acids and proteins of the invention are useful in potential therapeutic applications implicated in diseases including but not limited to various pathologies and disorders as indicated below. For example, a cDNA encoding the ankyrin repeat containing protein-like protein (NOV72) may be useful in gene therapy, and the ankyrin repeat containing protein-like protein (NOV72) may be useful when administered to a subject in need thereof. By way of nonlimiting example, the compositions of the present invention will have efficacy for treatment of patients suffering from Cardiomyopathy, Atherosclerosis, Hypertension, Congenital heart defects, Aortic stenosis, Atrial septal defect (ASD), Atrioventricular (A-V) canal defect, Ductus arteriosus, Pulmonary stenosis, Subaortic stenosis, Ventricular septal defect (VSD), valve diseases, Tuberous sclerosis, Scleroderma, Obesity, Transplantation, Systemic lupus erythematosus , Autoimmune disease, Asthma, Emphysema, Scleroderma, allergy, Diabetes, Autoimmune disease, Renal artery stenosis, Interstitial nephritis, Glomerulonephritis, Polycystic kidney disease, Systemic lupus erythematosus, Renal tubular acidosis, IgA nephropathy, Hypercalceimia, Lesch-Nyhan syndrome, Von Hippel-Lindau (VHL) syndrome, Alzheimer's disease, Stroke, Tuberous sclerosis, hypercalceimia, Parkinson's disease, Huntington's disease, Cerebral palsy, Epilepsy, Multiple sclerosis, Ataxia-telangiectasia, Leukodystrophies, Behavioral disorders, Addiction, Anxiety, Pain, Neuroprotection, or other pathologies or conditions. The NOV72 nucleic acid encoding the ankyrin repeat containing protein-like protein of the invention, or fragments thereof, may further be useful in diagnostic applications, wherein the presence or amount of the nucleic acid or the protein are to be assessed.

NOV72 nucleic acids and polypeptides are further useful in the generation of antibodies that bind immuno-specifically to the novel NOV72 substances for use in therapeutic or diagnostic methods. These antibodies may be generated according to methods known in the art, using prediction from hydrophobicity charts, as described in the "Anti-NOVX Antibodies" section below. The disclosed NOV72 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV73

A disclosed NOV73 nucleic acid of 1011 nucleotides (also referred to as CG57313-01) encoding a GPCR-like protein is shown in Table 73A. The start and stop codons are in bold letters.

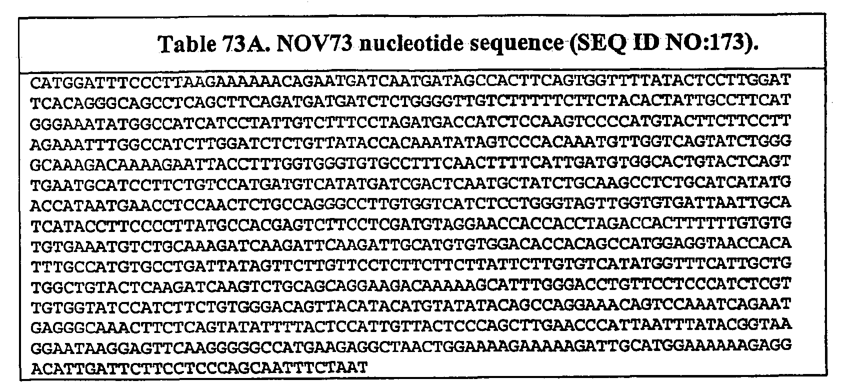

In a search of public sequence databases, the NOV73 nucleic acid sequence, located on chromsome 6 has has 223 of 363 bases (61%) identical to a gb:GENBANK-ID:U86270|acc:U86270.1 mRNA from Homo sapiens (Homo sapiens olfactory receptor (OR5-40) gene, partial cds). Public nucleotide databases include all GenBank databases and the GeneSeq patent database.

The disclosed NOV73 polypeptide (SEQ ID N0:174) encoded by SEQ ID N0:173 has 319 amino acid residues and is presented in Table 73B using the one-letter amino acid code. Signal P, Psort and/or Hydropathy results predict that NOV73 has a signal peptide and is likely to be localized extracellularly with a certainty of 0.6400. The most likely cleavage site for a NOV73 peptide is between amino acids 42 and 43.

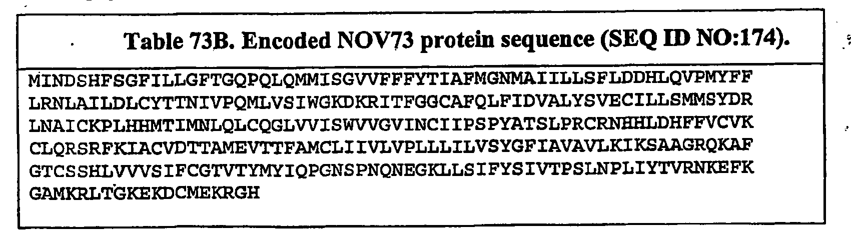

A search of sequence databases reveals that the NOV73 amino acid sequence has have 165 of 304 amino acid residues (54%) identical to, and 226 of 304 amino acid residues (74%) similar to, the 320 amino acid residue ptnr:SPTREMBL-ACC:Q9Y3N9 protein from Homo sapiens (Human) (DJ88J8.1 (NOVEL 7 TRANSMEMBRANE RECEPTOR (RHODOPSIN FAMILY) (OLFACTORY RECEPTOR LIKE) PROTEIN) (HS6M1-15))). Public amino acid databases include the GenBank databases, SwissProt, PDB and PIR.

The disclosed NOV73 polypeptide has homology to the amino acid sequences shown in the BLASTP data listed in Table 73C.

| Table 73C. BLAST results for NOV73 | | | | | |
|---|---|---|---|---|---|
| Gene Index/ Identifier | Protein/ Organism | Length (aa) | Identity (%) | Positives (%) | Expect |
| gi\|18480000\|gb\|AAL6 1014.1) (AY073351) | olfactory receptor MOR256-8 [Mus musculus] | 308 | 169/307 (55%) | 232/307 (75%) | 8e-88 |
| gi\|13624329\|ref\|NP 112165.1 (NM_030903) | olfactory receptor, family 2, subfamily W, member 1 [Homo sapiens] | 320 | 165/304 (54%) | 226/304 (74%) | 5e-86 |
| gi\|18480974\|gb \|AAL6 1501.1 (AY073838 | olfactory receptor MOR256-31 [Mus musculus] | 312 | 168/304 (55%) | 227/304 (74%) | 5e-86 |
| gi\|12054429\|emb \|CAC 20522.1 (AJ302602) | olfactory receptor [Mus musculus] | 320 | 165/304 (54%) | 225/304 (73%) | e-85 |
| gi\|12054431\|emb\|CAC 20523.1\|(AJ302603) | olfactory receptor [Homo sapiens] | 320 | 164/304 (53%) | 226/304 (73%) | e-85 |

Table 73D lists the domain descriptions from DOMAIN analysis results against NOV73. This indicates that the NOV73 sequence has properties similar to those of other proteins known to contain this domain.

| **Table 73D. Domain Analysis of NOV73** |
|---|
| gnl\|Pfam\|pfam00001, 7tm_1, 7 transmembrane receptor (rhodopsin family). |
| CD-Length = 254 residues, 100.0% aligned |
| Score = 92.0 bits (227), Expect = 4e-20 |

The protein sequence fingerprint is potently diagnostic of all sequences of this type in the database in which it was derived (the OWL composite sequence database, version 8.1), and has continued to perform well on subsequent database updates, identifying 240 receptors in OWL17.0. Results are compared with a commonly used pattern template for this class of receptors. The investigation suggests that discriminating power is improved in the fingerprint approach because the recognition of individual features is made mutually conditional. Furthermore, by avoiding the definition of predetermined feature separations, members of protein families possessing all or only part of the fingerprint may be identified. PMID: 8386361

The fingerprint encodes the seven putative membrane-spanning motifs and was potently diagnostic of all GPCRs (52 in all) in version 8.1 of the OWL composite sequence database, readily distinguishing them from all other integral membrane proteins. With a 3-fold increase in the size of OWL, the fingerprint has been updated and now finds 332 receptors that match all the motifs.

The glucagon receptor is a member of a distinct class of G protein-coupled receptors (GPCRs) sharing little amino acid sequence homology with the larger rhodopsin-like GPCR family. To identify the components of the glucagon receptor necessary for G-protein coupling, sequentially all or part of each intracellular loop (i1, i2, and i3) and the C-terminal tail of the glucagon receptor were replaced with the 11 amino acids comprising the first intracellular loop of the D4 dopamine receptor.

Whereas numerous mutations of the human lutropin receptor (hLHR) and human TSH receptor (hTSHR) have been shown to cause constitutive activation of these receptors, it has been suggested that either the hFSHR as a whole, or the i3/TM VI region of the hFSHR, is less susceptible to mutation-induced constitutive activation.

The disclosed NOV73 nucleic acid of the invention encoding a GPCR-like protein includes the nucleic acid whose sequence is provided in Table 73A or a fragment thereof. The invention also includes a mutant or variant nucleic acid any of whose bases may be changed from the corresponding base shown in Table 73A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 39 percent of the bases may be so changed.

The disclosed NOV73 protein of the invention includes the GPCR-like protein whose sequence is provided in Table 73B. The invention also includes a mutant or variant protein any of whose residues may be changed from the corresponding residue shown in Table 73B while still encoding a protein that maintains its GPCR-like activities and physiological functions, or a functional fragment thereof. In the mutant or variant protein, up to about 46 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV73) may function as a member of a "GPCR family". Therefore, the NOV73 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV73 nucleic acids and proteins of the invention are useful in potential therapeutic applications implicated in diseases including but not limited to various pathologies and disorders as indicated below. For example, a cDNA encoding the GPCR-like protein (NOV73) may be useful in gene therapy, and the GPCR-like protein (NOV73) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV73 nucleic acid encoding the GPCR-like protein of the invention, or fragments thereof, may further be useful in diagnostic applications, wherein the presence or amount of the nucleic acid or the protein are to be assessed.

NOV73 nucleic acids and polypeptides are further useful in the generation of antibodies that bind immuno-specifically to the novel NOV73 substances for use in therapeutic or diagnostic methods. These antibodies may be generated according to methods known in the art, using prediction from hydrophobicity charts, as described in the "Anti-NOVX Antibodies" section below. The disclosed NOV73 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV74

A disclosed NOV74 nucleic acid of 1008 nucleotides (also referred to as CG57315-01) encoding a GPCR-like protein is shown in Table 74A. The start and stop codons are in bold letters.

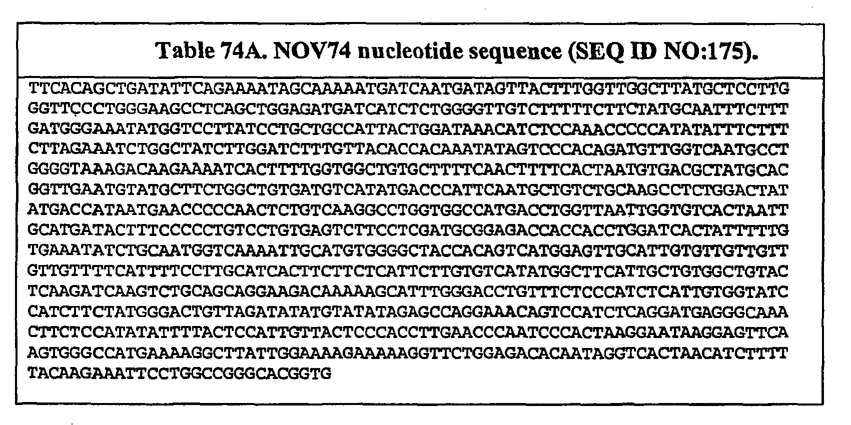

In a search of public sequence databases, the NOV74 nucleic acid sequence, located on chromsome 6 has has 583 of 946 bases (61 %) identical to a gb:GENBANK-ID:RATOL1RECE|acc:L34074.1 mRNA from Rattus norvegicus (Rat OL1 receptor gene, complete cds). Public nucleotide databases include all GenBank databases and the GeneSeq patent database.

The disclosed NOV74 polypeptide (SEQ ID NO:176) encoded by SEQ ID NO:175 has 313 amino acid residues and is presented in Table 74B using the one-letter amino acid code. Signal P, Psort and/or Hydropathy results predict that NOV74 has a signal peptide and is likely to be localized extracellularly with a certainty of 0.6000. The most likely cleavage site for a NOV74 peptide is between amino acids 63 and 64.

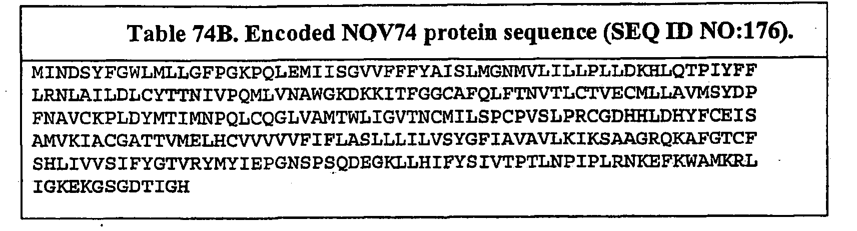

A search of sequence databases reveals that the NOV74 amino acid sequence has 161 of 298 amino acid residues (54%) identical to, and 220 of 298 amino acid residues (73%) similar to, the 320 amino acid residue ptnr:SPTREMBL-ACC:Q9Y3N9 protein from Homo sapiens (Human) (DJ88J8.1 (NOVEL 7 TRANSMEMBRANE RECEPTOR (RHODOPSIN FAMILY) (OLFACTORY RECEPTOR LIKE) PROTEIN) (HS6M1-15))). Public amino acid databases include the GenBank databases, SwissProt, PDB and PIR.

NOV74 is expressed in at least Whole Organism. Expression information was derived from the tissue sources of the sequences that were included in the derivation of the sequence ofCG57315_01.The sequence is predicted to be expressed in the following tissues because of the expression pattern of (GENBANK-ID: gb:GENBANK-ID:RATOL1RECE|acc: L34074.1) a closely related Rat OL1 receptor gene, complete cds homolog in species Rattus norvegicus: heart. This information was derived by determining the tissue sources of the sequences that were included in the invention including but not limited to SeqCalling sources, Public EST sources, Literature sources, and/or RACE sources.

The disclosed NOV74 polypeptide has homology to the amino acid sequences shown in the BLASTP data listed in Table 74C.

| **Table 74C. BLAST results for NOV74** | | | | | |
|---|---|---|---|---|---|
| Gene Index/ Identifier | Protein/ Organism | Length (aa) | Identity (%) | Positives (%) | Expects |
| gi\|17464347\|ref\|XP 069460.1 (XM_069460) | similar to dM53SM10.7 (novel 7 transmembrane receptor (rhodopsin family) (olfactory receptor like) protein) [Homo sapiens] | 590 | 186/186 (100%) | 186/186 (100%) | e-100 |
| gi\|18480974\|gb\|AAL6 1501.1 (AY073838) | olfactory receptor MOR256-31 [Mus musculus] | 312 | 167/293 (56%) | 218/293 (73%) | e-84 |
| gi\|18480000\|gb\|AAL6 1014.1\| (AY073351 | olfactory receptor MOR256-8 [Mus musculus] | 308 | 159/303 (52%) | 225/303 (73%) | 2e-83 |
| gi\|13624329\|ref\|NP 112165.1\| (NM_030903) | olfactory receptor, family 2, subfamily W, member 1 [Homo sapiens] | 320 | 161/301 (53%) | 219/301 (72%) | 3e-83 |
| - gi\|12054429\|emb\|CAC 20522.1\| (AJ302602) | olfactory receptor [Homo sapiens] | 320 | 161/301 (53%) | 218/301 (71%) | 6e-83 |

Table 74D lists the domain descriptions from DOMAIN analysis results against NOV74. This indicates that the NOV74 sequence has properties similar to those of other proteins known to contain this domain.

| **Table 74D. Domain Analysis of NOV74** |
|---|
| gnl\|Pfam\|pfam00001, 7tm_1, 7 transmembrane receptor (rhodopsin family). |
| CD-Length = 254 residues, 99.2% aligned |
| Score = 96.3 bits (238), Expect = 2e-21 |

The disclosed NOV74 nucleic acid of the invention encoding a GPCR-like protein includes the nucleic acid whose sequence is provided in Table 74A or a fragment thereof. The invention also includes a mutant or variant nucleic acid any of whose bases may be changed from the corresponding base shown in Table 74A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 39 percent of the bases may be so changed.

The disclosed NOV74 protein of the invention includes the GPCR-like protein whose. sequence is provided in Table 74B. The invention also includes a mutant or variant protein any of whose residues may be changed from the corresponding residue shown in Table 74B while still encoding a protein that maintains its GPCR-like activities and physiological functions, or a functional fragment thereof. In the mutant or variant protein, up to about 46 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV74) may function as a member of a "GPCR family". Therefore, the NOV74 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV74 nucleic acids and proteins of the invention are useful in potential therapeutic applications implicated in diseases including but not limited to various pathologies and disorders as indicated below. For example, a cDNA encoding the GPCR-like protein (NOV74) may be useful in gene therapy, and the GPCR-like protein (NOV74) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV74 nucleic acid encoding the GPCR-like protein of the invention, or fragments thereof, may further be useful in diagnostic applications, wherein the presence or amount of the nucleic acid or the protein are to be assessed.

NOV74 nucleic acids and polypeptides are further useful in the generation of antibodies that bind immuno-specifically to the novel NOV74 substances for use in therapeutic or diagnostic methods. These antibodies may be generated according to methods known in the art, using prediction from hydrophobicity charts, as described in the "Anti-NOVX Antibodies" section below. The disclosed NOV74 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV75

A disclosed NOV75 nucleic acid of 1050 nucleotides (also referred to as CG57317-01) encoding a GPCR-like protein is shown in Table 75A. The start and stop codons are in bold letters.

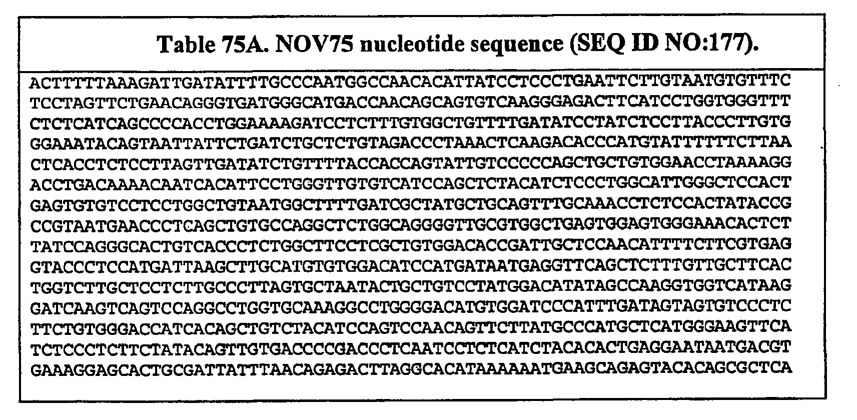

In a search of public sequence databases, the NOV75 nucleic acid sequence, located on chromsome 6 has 611 of 912 bases (66%) identical to a gb:GENBANK-ID:HUMORLMHC|acc:L35475.1 mRNA from Homo sapiens (Human olfactory receptor-like gene, complete cds). Public nucleotide databases include all GenBank databases and the GeneSeq patent database.

The disclosed NOV75 polypeptide (SEQ ID NO:178) encoded by SEQ ID NO:177 has 331 amino acid residues and is presented in Table 75B using the one-letter amino acid code. Signal P, Psort and/or Hydropathy results predict that NOV75 has a signal peptide and is likely to be localized extracellularly with a certainty of 0.6400. The most likely cleavage site for a NOV75 peptide is between amino acids 23 and 24.

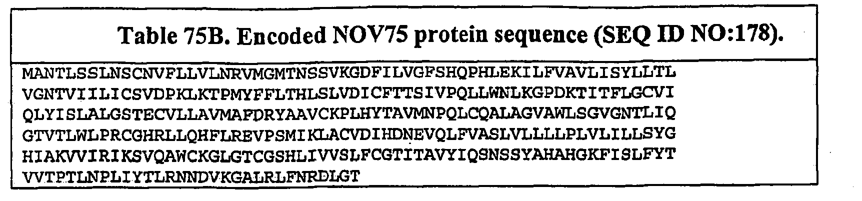

A search of sequence databases reveals that the NOV75 amino acid sequence has 178 of 306 amino acid residues (58%) identical to, and 234 of 306 amino acid residues (76%) similar to, the 312 amino acid residue ptnr:SPTREMBL-ACC:Q9R0Z2 protein from Mus musculus (Mouse) (573K1.3 (MM17M1-4 (NOVEL 7 TRANSMEMBRANE RECEPTOR (RHODOPSIN FAMILY) (OLFACTORY RECEPTOR LIKE) PROTEIN))). Public amino acid databases include the GenBank databases, SwissProt, PDB and PIR.

The disclosed NOV75 polypeptide has homology to the amino acid sequences shown in the BLASTP data listed in Table 75C.

| **Table 75C. BLAST results for NOV75** | | | | | |
|---|---|---|---|---|---|
| Gene Index/ Identifier | Protein/ Organism | Length (aa) | Identity (%) | Positives (%) | Expect |
| gi\|17464347\|ref\|Xp 069460.1 (XM_069460) | similar to dM538M10.7 (novel 7 transmembrane receptor (rhodopsin family) (olfactory receptor like) protein) [Homo sapiens] | 590 | 270/322 (83%) | 271/322 (83%) | e-121 |
| gi\|18480232\|gb\|AAL6 1130.1\|(AY073467 | olfactory receptor MOR256-9 [Mus musculus] | 309 | 254/308 (82%) | 273/308 (88%) | e-120 |
| gi\|18480518\|gb\|AAL6 1273.1\|(AY073610) | olfactory receptor MOR256-19 [Mus musculus] | 317 | 229/298 (76%) | 259/298 (86%) | e-112 |
| gi\|18565094\|ref\|XP 094939.1\| (XM 094939) | hypothetical protein XP_094939 [Homo sapiens] | 310 | 225/235 (95%) | 226/235 (95%) | e-112 |
| gi\|114596252\|emb \|CAC 43450.11 (AL136158) | dM538M10.7 (novel 7 transmembrane receptor (rhodopsin family) (olfactory receptor like) protein) [Mus musculus] | 317 | 233/307 (75%) | 260/307 (83%) | e-111 |

Table 75D lists the domain descriptions from DOMAIN analysis results against NOV75. This indicates that the NOV75 sequence has properties similar to those of other proteins known to contain this domain.

| **Table 75D. Domain Analysis of NOV75** |
|---|
| gnl\|Pfam\|pfam00001, 7tm_1, 7 transmembrane receptor (rhodopsin family). |
| CD-Length = 254 residues, 46.9% aligned |
| Score = 78.6 bits (192), Expect = 5e-16 |

The disclosed NOV75 nucleic acid of the invention encoding a GPCR-like protein includes the nucleic acid whose sequence is provided in Table 75A or a fragment thereof. The invention also includes a mutant or variant nucleic acid any of whose bases may be changed from the corresponding base shown in Table 75A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 34 percent of the bases may be so changed.

The disclosed NOV75 protein of the invention includes the GPCR-like protein whose sequence is provided in Table 75B. The invention also includes a mutant or variant protein any of whose residues may be changed from the corresponding residue shown in Table 75B while still encoding a protein that maintains its GPCR-like activities and physiological functions, or a functional fragment thereof. In the mutant or variant protein, up to about 42 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV75) may function as a member of a "GPCR family". Therefore, the NOV75 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV75 nucleic acids and proteins of the invention are useful in potential therapeutic applications implicated in diseases including but not limited to various pathologies and disorders as indicated below. For example, a cDNA encoding the GPCR-like protein (NOV75) may be useful in gene therapy, and the GPCR-like protein (NOV75) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV75 nucleic acid encoding the GPCR-like protein of the invention, or fragments thereof, may further be useful in diagnostic applications, wherein the presence or amount of the nucleic acid or the protein are to be assessed.

NOV75 nucleic acids and polypeptides are further useful in the generation of antibodies that bind immuno-specifically to the novel NOV75 substances for use in therapeutic or diagnostic methods. These antibodies may be generated according to methods known in the art, using prediction from hydrophobicity charts, as described in the "Anti-NOVX Antibodies" section below. The disclosed NOV75 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV76

A disclosed NOV76 nucleic acid of 1063 nucleotides (also referred to as CG57321-01) encoding a GPCR-like protein is shown in Table 76A. The start and stop codons are in bold letters.

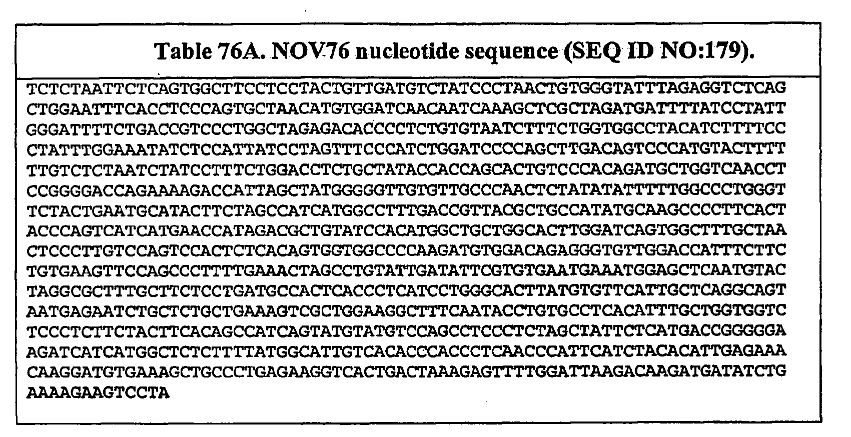

In a search of public sequence databases, the NOV76 nucleic acid sequence, located on chromsome 6 has 657 of 971 bases (67%) identical to a gb:GENBANK-ED:RATOL1RECE|acc:L34074.1 mRNA from Rattus norvegicus (Rat OL1 receptor gene, complete cds). Public nucleotide databases include all GenBank databases and the GeneSeq patent database.

The disclosed NOV76 polypeptide (SEQ ID NO:180) encoded by SEQ ID NO:179 has 336 amino acid residues and is presented in Table 76B using the one-letter amino acid code. Signal P, Psort and/or Hydropathy results predict that NOV76 has a signal peptide and is likely to be localized localized extracellularly with a certainty of 0.6000. The most likely cleavage site for a NOV76 peptide is between amino acids 14 and 15.

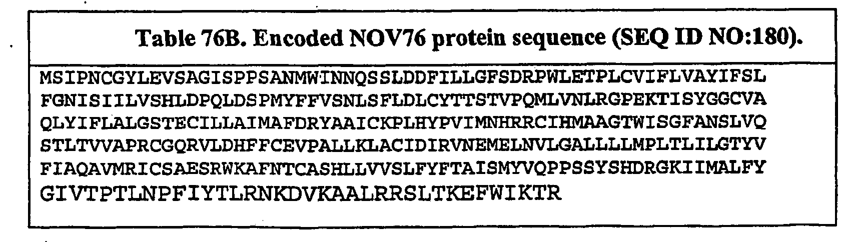

A search of sequence databases reveals that the NOV76 amino acid sequence has 197 of 308 amino acid residues (63%) identical to, and 245 of 308 amino acid residues (79%) similar to, the 313 ammo acid residue ptnr:SPTREMBL-ACC:Q63394 protein from Rattus norvegicus (Rat) (OL1 RECEPTOR). Public amino acid databases include the GenBank databases, SwissProt, PDB and PIR.

The disclosed NOV76 polypeptide has homology to the amino acid sequences shown in the BLASTP data listed in Table 76C.

| **Table 76C. BLAST results for NOV76** | | | | | |
|---|---|---|---|---|---|
| Gene Index/ Identifier | Protein/ Organism | Length (aa) | Identity (%) | Positives (%) | Expect |
| gi\|17464349\|ref\|XP 069461.1 (XM_069461) | similar to olfactory receptor, family 2, subfamily B, member 2 [Homo sapiens] | 298 | 297/315 (94%) | 298/315 (94%) | e-147 |
| gi\|18479350\|gb\|AAL6 0689.1) (AY073026) | olfactory receptor MOR256-3 [Mus musculus] | 315 | 287/315 (91%) | 302/315 (95%) | e-147 |
| gi\|111177906\|ref\|NP 068632.1 (NM 021860) | olfactory receptor [Mus musculus] | 313 | 197/308 (63%) | 245/308 (78%) | e-104 |
| gi\|14780900\|ref\|NP 149046.1 (NM_033057) | olfactory receptor, family 2, subfamily B, member 2 [Homo sapiens] | 357 | 199/307 (64%) | 245/307 (78%) | e-103 |
| gi\|18480406\|gb\|AAL6 1217.1 (AY073554) | olfactory receptor MOR256-10 [Mus musculus] | 313 | 196/308 (63%) | 242/308 (77%) | e-103 |

Table 76D lists the domain descriptions from DOMAIN analysis results against NOV76. This indicates that the NOV76 sequence has properties similar to those of other proteins known to contain this domain.

| **Table 76D. Domain Analysis of NOV 76** |
|---|
| gn\|Pfam\|pfam00001, 7tm_1 , 7 transmembrane receptor (rhodopsin family). |
| CD-Length = 254 residues, 100.0% aligned |
| Score = 95.9 bits (237), Expect = 3e-21 |

The disclosed NOV76 nucleic acid of the invention encoding a GPCR-like protein includes the nucleic acid whose sequence is provided in Table 76A or a fragment thereof. The invention also includes a mutant or variant nucleic acid any of whose bases may be changed from the corresponding base shown in Table 76A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 33 percent of the bases may be so changed.

The disclosed NOV76 protein of the invention includes the GPCR-like protein whose sequence is provided in Table 76B. The invention also includes a mutant or variant protein any of whose residues may be changed from the corresponding residue shown in Table 76B while still encoding a protein that maintains its GPCR-like activities and physiological functions, or a functional fragment thereof. In the mutant or variant protein, up to about 37 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV76) may function as a member of a "GPCR family". Therefore, the NOV76 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV76 nucleic acids and proteins of the invention are useful in potential therapeutic applications implicated in diseases including but not limited to various pathologies and disorders as indicated below. For example, a cDNA encoding the GPCR-like protein (NOV76) may be useful in gene therapy, and the GPCR-like protein (NOV76) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV76 nucleic acid encoding the GPCR-like protein of the invention, or fragments thereof, may further be useful in diagnostic applications, wherein the presence or amount of the nucleic acid or the protein are to be assessed.

NOV76 nucleic acids and polypeptides are further useful in the generation of antibodies that bind immuno-specifically to the novel NOV76 substances for use in therapeutic or diagnostic methods. These antibodies may be generated according to methods known in the art, using prediction from hydrophobicity charts, as described in the "Anti-NOVX Antibodies" section below. The disclosed NOV76 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV77

A disclosed NOV77 nucleic acid of 1014 nucleotides (also referred to as CG57419-01) encoding a GPCR-like protein is shown in Table 77A. The start and stop codons are in bold letters.

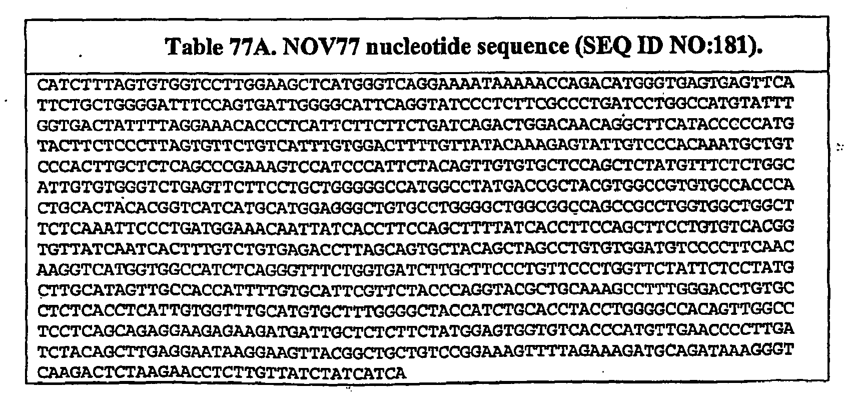

In a search of public sequence databases, the NOV77 nucleic acid sequence, located on chromsome 7 has 358 of 495 bases (72%) identical to a gb:GENBANK-ID:HSU56421 |acc:U56421.1 mRNA from Homo sapiens (Human olfactory receptor (OLF3) gene, complete cds). Public nucleotide databases include all GenBank databases and the GeneSeq patent database.

The disclosed NOV77 polypeptide (SEQ ID NO:182) encoded by SEQ ID NO:181 has 315 amino acid residues and is presented in Table 77B using the one-letter amino acid code. Signal P, Psort and/or Hydropathy results predict that NOV77 has a signal peptide and is likely to be localized extracellularly with a certainty of 0.6000. The most likely cleavage site for a NOV77 peptide is between amino acids 43 and 44.

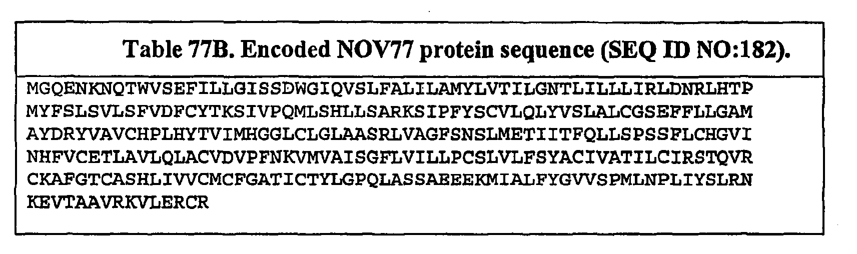

A search of sequence databases reveals that the NOV77 amino acid sequence has have 180 of 305 amino acid residues (59%) identical to, and 229 of 305 amino acid residues (75%) similar to, the 317 amino acid residue ptnr:SWISSPROT-ACC:Q95156 protein from Canis familiaris (Dog) (OLFACTORY RECEPTOR-LIKE PROTEIN OLF3). Public amino acid databases include the GenBank databases, SwissProt, PDB and PIR.

The disclosed NOV77 polypeptide has homology to the amino acid sequences shown in the BLASTP data listed in Table 77C .

| **Table 77C. BLAST results for NOV77** | | | | | |
|---|---|---|---|---|---|
| Gene Index/ Identifier | Protein/ organism | Length (aa) | Identity (%) | Positives (%) | Expect |
| gi\|18480604\|gb\|AAL6 1316.1 (AY073653 | olfactory receptor MOR257-3 [Mus musculus] | 310 | 239/315 (75%) | 268/315 (84%) | e-112 |
| gi\|2495055\|sp\|Q9515 6\|OLF3 CANFA | OLFACTORY RECEPTOR-LIKE PROTEIN OLF3 | 317 | 178/305 (58%) | 226/305 (73%) | E-82 |
| gi\|6912558\|ref\|NP 0 36501.1\|\| (NM_012369) | olfactory receptor, family 2, subfamily F, member 1; olfactory receptor, family 2, subfamily F, member 5; olfactory receptor, family 2, subfamily F, member 4 [Homo sapiens] | 317 | 173/305 (56%) | 221/305 (71%) | e-79 |
| gi\|9297120\|sp \|Q1360 7\|O2F1 HUMAN | OLFACTORY RECEPTOR 2F1 (OLFACTORY RECEPTOR-LIKE PROTEIN OLF3) | 317 | 173/305 (56%) | 221/305 (71%) | e-79 |
| gi\|18479500\|gb\|AAL6 0764.1\|(AY073101) | olfactory receptor MOR257-1 [Mus musculus] | 313 | 176/305 (57%) | 219/305 (71%) | 3e-79 |

Table 77D lists the domain descriptions from DOMAIN analysis results against NOV77. This indicates that the NOV77 sequence has properties similar to those of other proteins known to contain this domain.

| **Table 77D. Domain Analysis of NOV77** | |
|---|---|
| gal\|Pfam\|pfam00001, 7tm_1,7 transmembrane receptor (rhodopsin family). | |
| | CD-Length = 254 residues, 94.1% aligned |
| | Score = 75.5 bits (184), Expect = 4e-15 |

The disclosed NOV77 nucleic acid of the invention encoding a GPCR-like protein includes the nucleic acid whose sequence is provided in Table 77A or a fragment thereof. The invention also includes a mutant or variant nucleic acid any of whose bases may be changed from the corresponding base shown in Table 77A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 28 percent of the bases may be so changed.

The disclosed NOV77 protein of the invention includes the GPCR-like protein whose sequence is provided in Table 77B. The invention also includes a mutant or variant protein any of whose residues may be changed from the corresponding residue shown in Table 77B while still encoding a protein that maintains its GPCR-like activities and physiological functions, or a functional fragment thereof. In the mutant or variant protein, up to about 41 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV77) may function as a member of a "GPCR family". Therefore, the NOV77 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV77 nucleic acids and proteins of the invention are useful in potential therapeutic applications implicated in diseases including but not limited to various pathologies and disorders as indicated below. For example, a cDNA encoding the GPCR-like protein (NOV77) may be useful in gene therapy, and the GPCR-like protein (NOV77) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV77 nucleic acid encoding the GPCR-like protein of the invention, or fragments thereof, may further be useful in diagnostic applications, wherein the presence or amount of the nucleic acid or the protein are to be assessed.

NOV77 nucleic acids and polypeptides are further useful in the generation of antibodies that bind immuno-specifically to the novel NOV77 substances for use in therapeutic or diagnostic methods. These antibodies may be generated according to methods known in the art, using prediction from hydrophobicity charts, as described in the "Anti-NOVX Antibodies" section below. The disclosed NOV77 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV78

A disclosed NOV78 nucleic acid of 1151 nucleotides (also referred to as CG57425-01) encoding a GPCR-like protein is shown in Table 78A. The start and stop codons are in bold letters.

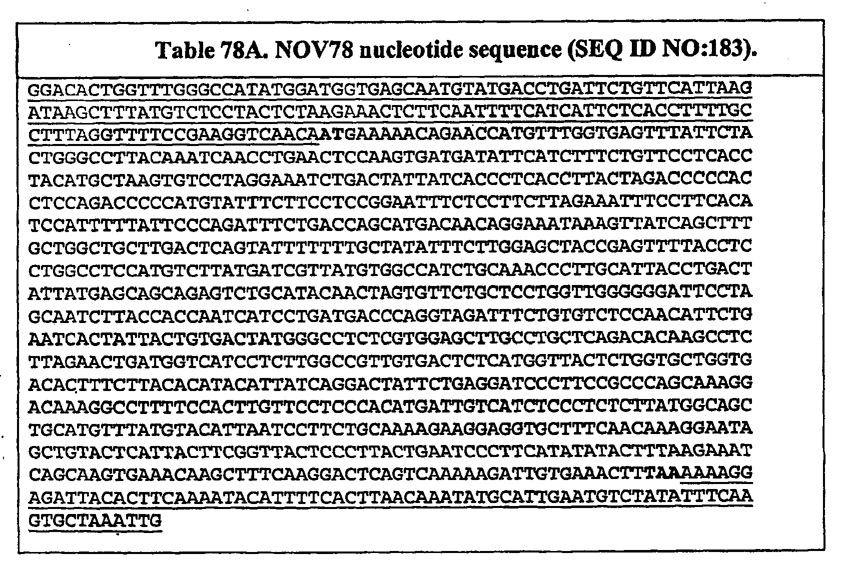

In a search of public sequence databases, the NOV78 nuceic acid sequence, located on chromsome 12 has 605 of 931 bases (64%) identical to a gb:GENBANK-ID:AF102523|acc:AF102523.1 mRNA from Mus musculus (Mus musculus olfactory receptor C6 gene, complete cds). Public nucleotide databases include all GenBank databases and the GeneSeq patent database.

The disclosed NOV78 polypeptide (SEQ ID NO:184) encoded by SEQ ID NO:183 has 309 amino acid residues and is presented in Table 78B using the one-letter amino acid code. Signal P, Psort and/or Hydropathy results predict that NOV78 has a signal peptide and is likely to be localized at the plasma membrane with a certainty of 0.6000. The most likely cleavage site for a NOV78 peptide is at amino acid 39.

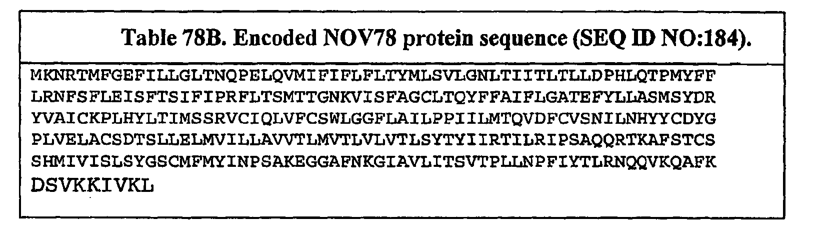

A search of sequence databases reveals that the NOV78 amino acid sequence has 175 of 309 amino acid residues (56%) identical to, and 222 of 309 amino acid residues (71%) similar to, the 313 amino acid residue ptnr:SPTREMBL-ACC:Q9Z1V0 protein from Mus musculus (Mouse) (OLFACTORY RECEPTOR C6)(. Public amino acid databases include the GenBank databases, SwissProt, PDB and PIR.

NOV78 is expressed in at least Apical microvilli of the retinal pigment epithelium, arterial (aortic), basal forebrain, brain, Burkitt lymphoma cell lines, corpus callosum, cardiac (atria and ventricle), caudate nucleus, CNS and peripheral tissue, cerebellum, cerebral cortex, colon, cortical neurogenic cells, endothelial (coronary artery and umbilical vein) cells, palate epithelia, eye, neonatal eye, frontal cortex, fetal hematopoietic cells, heart, hippocampus, hypothalamus, leukocytes, liver, fetal liver, lung, lung lymphoma cell lines, fetal lymphoid tissue, adult lymphoid tissue, Those that express MHC II and III nervous, medulla, subthalamic nucleus, ovary, pancreas, pituitary, placenta, pons, prostate, putamen, serum, skeletal muscle, small intestine, smooth muscle (coronary artery in aortic) spinal cord, spleen, stomach, taste receptor cells of the tongue, testis, thalamus, and thymus tissue. This information was derived by determining the tissue sources of the sequences that were included in the invention including but not limited to SeqCalling sources, Public EST sources, Literature sources, and/or RACE sources.

The disclosed NOV78 polypeptide has homology to the amino acid sequences shown in the BLASTP data listed in Table 78C.

| **Table 78C. BLAST results for NOV78** | | | | | |
|---|---|---|---|---|---|
| Gene Index/ Identifier | Protein/ Organism | Length (aa) | Identity (%) | Positives (%) | Expect |
| gi\|17474307\|ref\|XP 062466.1\| (XM_062466) | similar to. olfactory receptor 49 [Homo sapiens] | 309 | 308/309 (99%) | 309/309 (99%) | e-141 |
| gi\|18480848\|gb\|AAL6 1438.1\| (AY073775) | olfactory receptor MOR115-4 [Mus musculus] | 309 | 238/309 (77%), Positives = 273/309 (88%) | 238/309 (77%), positives = 273/309 (88%) | e-114 |
| gi\|18479958\|gb\|AAL6 0993.1 (AY073330) | olfactory receptor MOR115-1 [Mus musculus] | 309 | 239/309 (77%), Positives = 274/309 (88%) | 239/309 (77%), Positives = 274/309 (88%) | e-112 |
| gi\|17474309\|ref\|XP 062467.1\| (XM_062467) | similar to olfactory receptor 49 [Homo sapiens] | 309 | 247/309 (79%), Positives = 278/309 (89%) | 247/309 (79%), Positives = 278/309 (89%) | e-112 |
| qi \|18479614 \|gb \|AAL6 0821.1 \| (AY073158) | olfactory receptor MOR114-1 [Mus musculus] | 312 | 207/306 (67%), Positives = 256/306 (83%) | 207/306 (67%), Positives = 256/306 (83%) | e-110 |

Table 78D lists the domain descriptions from DOMAIN analysis results against NOV78. This indicates that the NOV78 sequence has properties similar to those of other proteins known to contain this domain.

| **Table 78D. Domain Analysis of NOV78** |
|---|
| gnl\|Pfam\|pfam00001, 7tm_1, 7 transmembrane receptor (rhodopsin family). |
| CD-Length = 254 residues, 100.0% aligned |
| Score = 90.1 bits (222), Expect = 2e-19 |

G-Protein Coupled Receptor (GPCRs) have been identified as an extremely large family of protein receptors in a number of species. At the phylogenetic level they can be classified into four major subfamilies. These receptors share a seven transmembrane domain structure with many neurotransmitter and hormone receptors. They are likely to be involved in the recognition and transduction of various signals mediated by G-Proteins, hence their name G-Protein Coupled Receptors. The human GPCR genes are generally intron-less and belong to four gene subfamilies, displaying great sequence variability. These genes are dominantly expressed in olfactory epithelium.

Olfactory receptors (ORs) have been identified as extremely large family of GPCRs in a number of species. As members of the GPCR family, these receptors share a seven transmembrane domain structure with many neurotransmitter and hormone receptors, and are likely to underlie the recognition and G-protein-mediated transduction of odorant signals. Like GPCRs, the ORs they can be expressed in a variety of tissues where they are thought to be involved in recognition and transmission of a variety of signals. The human OR genes are typically intron-less and belong to four different gene subfamilies, displaying great sequence variability. These genes are dominantly expressed in olfactory epithelium.

The disclosed NOV78 nucleic acid of the invention encoding a GPCR-like protein includes the nucleic acid whose sequence is provided in Table 78A or a fragment thereof. The invention also includes a mutant or variant nucleic acid any of whose bases may be changed from the corresponding base shown in Table 78A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 36 percent of the bases may be so changed.

The disclosed NOV78 protein of the invention includes the GPCR-like protein whose sequence is provided in Table 78B. The invention also includes a mutant or variant protein any of whose residues may be changed from the corresponding residue shown in Table 78B while still encoding a protein that maintains its GPCR-like activities and physiological functions, or a functional fragment thereof. In the mutant or variant protein, up to about 44 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV78) may function as a member of a "GPCR family". Therefore, the NOV78 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV78 nucleic acids and proteins of the invention are useful in potential therapeutic applications implicated in diseases including but not limited to various pathologies and disorders as indicated below. For example, a cDNA encoding the GPCR-like protein (NOV78) may be useful in gene therapy, and the GPCR-like protein (NOV78) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV78 nucleic acid encoding the GPCR-like protein of the invention, or fragments thereof, may further be useful in diagnostic applications, wherein the presence or amount of the nucleic acid or the protein are to be assessed.

NOV78 nucleic acids and polypeptides are further useful in the generation of antibodies that bind immuno-specifically to the novel NOV78 substances for use in therapeutic or diagnostic methods. These antibodies may be generated according to methods known in the art, using prediction from hydrophobicity charts, as described in the "Anti-NOVX Antibodies" section below. The disclosed NOV78 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV79

A disclosed NOV79 nucleic acid of 1601 nucleotides (also referred to as CG57753-01) encoding a GPCR-like protein is shown in Table 79A. The start and stop codons are in bold letters.

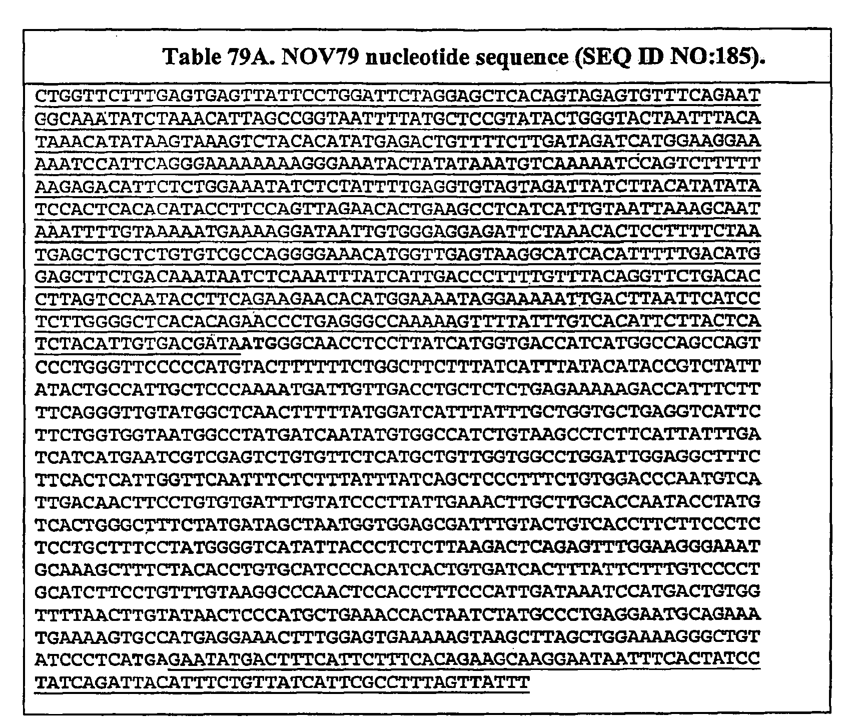

In a search of public sequence databases, the NOV79 nucleic acid sequence, located on chromsome 12 has 605 of 931 bases (64%) identical to a gb:GENBANK-ID:AF102523|acc:AF102523.1 mRNA from Mus musculus (Mus musculus olfactory receptor C6 gene, complete cds). Public nucleotide databases include all GenBank databases and the GeneSeq patent database.

The disclosed NOV79 polypeptide (SEQ ID NO:186) encoded by SEQ ID NO:185 has 277 amino acid residues and is presented in Table 79B using the one-letter amino acid code. Signal P, Psort and/or Hydropathy results predict that NOV79 has a signal peptide and is likely to be localized at the plasma membrane with a certainty of 0.6000. The most likely cleavage site for a NOV79 peptide is at amino acid position 39.

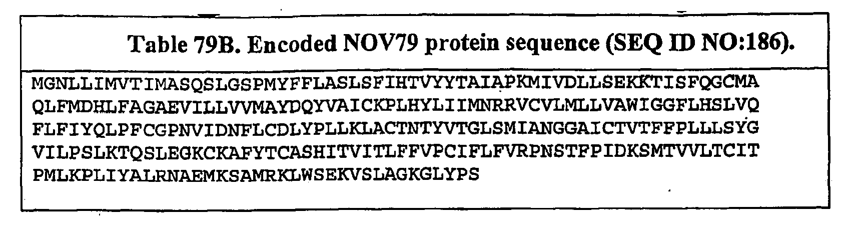

A search of sequence databases reveals that the NOV79 amino acid sequence has 175 of 309 amino acid residues (56%) identical to, and 222 of 309 amino acid residues (71%) similar to, the 313 amino acid residue ptnr:SPTREMBL-ACC:Q9ZIVO protein from Mus musculus (Mouse) (OLFACTORY RECEPTOR C6)(. Public amino acid databases include the GenBank databases, SwissProt, PDB and PIR.

NOV79 is expressed in at least Apical microvilli of the retinal pigment epithelium, arterial (aortic), basal forebrain, brain, Burkitt lymphoma cell lines, corpus callosum, cardiac (atria and ventricle), caudate nucleus, CNS and peripheral tissue, cerebellum, cerebral cortex, colon, cortical neurogenic cells, endothelial (coronary artery and umbilical vein) cells, palate epithelia, eye, neonatal eye, frontal cortex, fetal hematopoietic cells, heart, hippocampus, hypothalamus, leukocytes, liver, fetal liver, lung, lung lymphoma cell lines, fetal lymphoid tissue, adult lymphoid tissue, Those that express MHC II and III nervous, medulla, subthalamic nucleus, ovary, pancreas, pituitary, placenta, pons, prostate, putamen, serum, skeletal muscle, small intestine, smooth muscle (coronary artery in aortic) spinal cord, spleen, stomach, taste receptor cells of the tongue, testis, thalamus, and thymus tissue. This information was derived by determining the tissue sources of the sequences that were included in the invention including but not limited to SeqCalling sources, Public EST sources, Literature sources, and/or RACE sources.

The disclosed NOV79 polypeptide has homology to the amino acid sequences shown in the BLASTP data listed in Table 79C.

| **Table 79C. BLAST results for NOV79** | | | | | |
|---|---|---|---|---|---|
| Gene Index/ Identifier | Protein/ Organism | Length (aa) | Identity (%) | Positives (%) | Expect |
| gi\|17459952\|ref\|XP 062090.1\| (XM_062090) | similar to odorant receptor 16 [Homo sapiens] | 277 | 275/277 (99%) | 276/277 (99%) | e-133 |
| gi\|17460091\|ref\|XP 062159.1\| (XM_062159) | similar to odorant receptor 16 [Homo sapiens] | 277 | 275/277 (99%) | 275/277 (99%) | e-132 |
| gi\|17460099\|ref\|XP 062161.1\| (XM 062161 | similar to odorant receptor 16 [Homo sapiens] | 722 | 242/266 (90%) | 251/266 (93%) | e-119 |
| gi\|18479528\|gb\|AAL6 0778.1\| (AY073115) | olfactory receptor MOR231-2 [Mus musculus] | 314 | 226/277 (81%) | 247/277 (88%). | e-109 |
| gi\|18479534\|gb\|AAL6 0781.1\| (AY073118) | olfactory receptor MOR231-3 [Mus musculus] | 305 | 186/265 (70%) | 223/265 (83%) | 7e-94 |

Table 79D lists the domain descriptions from DOMAIN analysis results against NOV79. This indicates that the NOV79 sequence has properties similar to those of other proteins known to contain this domain.

| **Table 79D. Domain Analysis of NOV79** | |
|---|---|
| gnl\|Pfam\|pfam00001, 7tm_1, 7 transmembrane receptor (rhodopsin family). | |
| | CD-Length = 254 residues, 100.0% aligned |
| | Score = 91.3 bits (225), Expect = 7e-20 |

G-Protein Coupled Receptor (GPCRs) have been identified as an extremely large family of protein receptors in a number of species. At the phylogenetic level they can be classified into four major subfamilies. These receptors share a seven transmembrane domain structure with many neurotransmitter and hormone receptors. They are likely to be involved in the recognition and transduction of various signals mediated by G-Proteins, hence their name G-Protein Coupled Receptors. The human GPCR genes are generally intron-less and belong to four gene subfamilies, displaying great sequence variability. These genes are dominantly expressed in olfactory epithelium.

Olfactory receptors (ORs) have been identified as extremely large family of GPCRs in a number of species. As members of the GPCR family, these receptors share a seven transmembrane domain structure with many neurotransmitter and hormone receptors, and are likely to underlie the recognition and G-protein-mediated transduction of odorant signals. Like GPCRs, the ORs they can be expressed in a variety of tissues where they are thought to be involved in recognition and transmission of a variety of signals. The human OR genes are typically intron-less and belong to four different gene subfamilies, displaying great sequence variability. These genes are dominantly expressed in olfactory epithelium. The disclosed NOV79 nucleic acid of the invention encoding a GPCR-like protein includes the nucleic acid whose sequence is provided in Table 79A or a fragment thereof. The invention also includes a mutant or variant nucleic acid any of whose bases may be changed from the corresponding base shown in Table 79A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 36 percent of the bases maybe so changed.

The disclosed NOV79 protein of the invention includes the GPCR-like protein whose sequence is provided in Table 79B. The invention also includes a mutant or variant protein any of whose residues may be changed from the corresponding residue shown in Table 79B while still encoding a protein that maintains its GPCR-like activities and physiological functions, or a functional fragment thereof. In the mutant or variant protein, up to about 44 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV79) may function as a member of a "GPCR family". Therefore, the NOV79 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV79 nucleic acids and proteins of the invention are useful in potential therapeutic applications implicated in diseases including but not limited to various pathologies and disorders as indicated below. For example, a cDNA encoding the GPCR-like protein (NOV79) may be useful in gene therapy, and the GPCR-like protein (NOV79) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV79 nucleic acid encoding the GPCR-like protein of the invention, or fragments thereof, may further be useful in diagnostic applications, wherein the presence or amount of the nucleic acid or the protein are to be assessed.

NOV79 nucleic acids and polypeptides are further useful in the generation of antibodies that bind immuno-specifically to the novel NOV79 substances for use in therapeutic or diagnostic methods. These antibodies may be generated according to methods known in the art, using prediction from hydrophobicity charts, as described in the "Anti-NOVX Antibodies" section below. The disclosed NOV79 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV80

A disclosed NOV80 nucleic acid of 1006 nucleotides (also referred to as CG56766-01) encoding a GPCR-like protein is shown in Table 80A. The start and stop codons are in bold letters.

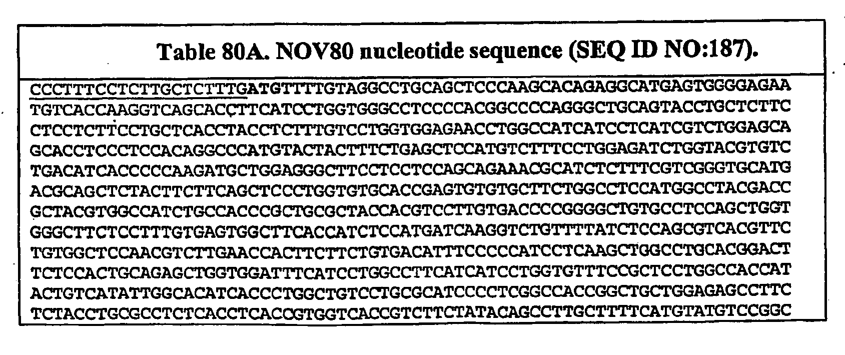

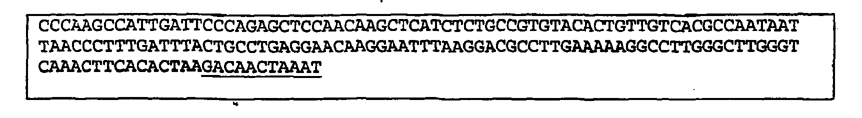

The disclosed NOV80 polypeptide (SEQ ID NO:188) encoded by SEQ ID NO:187 has 324 amino acid residues and is presented in Table 80B using the one-letter amino acid code.

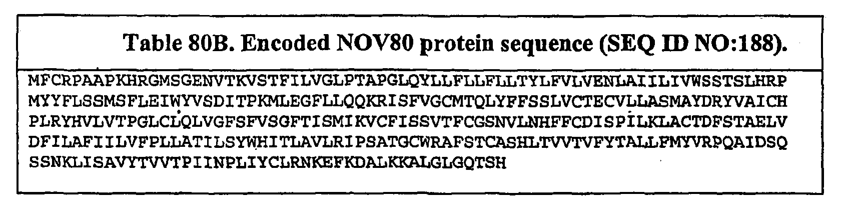

A search of sequence databases reveals that the NOV80 amino acid sequence has 215/305 (70%) identity and 253/305 (82%) similarity with TREMBLNEW-ACC:AAG45189 M51 OLFACTORY RECEPTOR - Mus musculus. Public amino acid databases include the GenBank databases, SwissProt, PDB and PIR.

The disclosed NOV80 polypeptide has homology to the amino acid sequences shown in the BLASTP data listed in Table 80C.

| **Table 80C. BLAST results for NOV80** | | | | | |
|---|---|---|---|---|---|
| Gene Index/ Identifier | Protein/ Organism | Length (aa) | Identity (%) | Positives (%) | Expect |
| gi\|17435888\|ref\|XP 065377.1 (XM_065377 | similar to olfactory receptor 41 [Homo sapiens] | 312 | 312/312 (100%) | 312/312 (100%) | e-142 |
| gi\|17435880\|ref\|XP 065375.1 (XM_065375) | similar to olfactory receptor 41 [Homo sapiens] | 331 | 293/307 (95%) | 300/307 (97%) | e-132 |
| gi\|18479396\|gb\|AAL6 0712.1\| (AY073049) | olfactory receptor MOR103-2 [Mus musculus] | 312 | 271/309 (8.7%) | 289/309 (92%) | e-123 |
| gi\|18479398\|gb\|AAL6 0713.1\| (AY073050) | olfactory receptor MOR103-3 [Mus musculus] | 312 | 268/310 (86%) | 288/310 (92%) | e-123 |
| gi 12007416 gb AAG4 5189.1 (AF321234) | m51 olfactory receptor [Mus musculus | 314 | 215/305 (70%) | 253/305 (82%) | e-102 |

Table 80D lists the domain descriptions from DOMAIN analysis results against NOV80. This indicates that the NOV80 sequence has properties similar to those of other proteins known to contain this domain.

| **Table 80D. Domain Analysis of NOV80** | |
|---|---|
| gnl\|Pfam\|pfam00001, 7tm_1, 7 transmembrane receptor (rhodopsin family). | |
| | CD-Length = 254 residues, 100.0% aligned |
| | Score = 98.6 bits (244), Expect = 5e-22 |

The disclosed NOV80 nucleic acid of the invention encoding a GPCR-like protein includes the nucleic acid whose sequence is provided in Table 80A or a fragment thereof The invention also includes a mutant or variant nucleic acid any of whose bases may be changed from the corresponding base shown in Table 80A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV80 protein of the invention includes the GPCR-like protein whose sequence is provided in Table 80B. The invention also includes a mutant or variant protein any of whose residues may be changed from the corresponding residue shown in Table 80B while still encoding a protein that maintains its GPCR-like activities and physiological functions, or a functional fragment thereof. In the mutant or variant protein, up to about 30 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV80) may function as a member of a "GPCR family". Therefore, the NOV80 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV80 nucleic acids and proteins of the invention are useful in potential therapeutic applications implicated in diseases including but not limited to various pathologies and disorders as indicated below. For example, a cDNA encoding the GPCR-like protein (NOV80) may be useful in gene therapy, and the GPCR-like protein (NOV80) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV80 nucleic acid encoding the GPCR-like protein of the invention, or fragments thereof, may further be useful in diagnostic applications, wherein the presence or amount of the nucleic acid or the protein are to be assessed.

NOV80 nucleic acids and polypeptides are further useful in the generation of antibodies that bind immuno-specifically to the novel NOV80 substances for use in therapeutic or diagnostic methods. These antibodies may be generated according to methods known in the art, using prediction from hydrophobicity charts, as described in the "Anti-NOVX Antibodies" section below. The disclosed NOV80 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV81

NOV81 includes two GPCR-like proteins disclosed below. The disclosed sequences have been named NOV81a and NOV81b.

### NOV81a

A disclosed NOV81a nucleic acid of 1039 nucleotides (also referred to as CG57847-01) encoding a GPCR-like protein is shown in Table 81A. The start and stop codons are in bold letters.

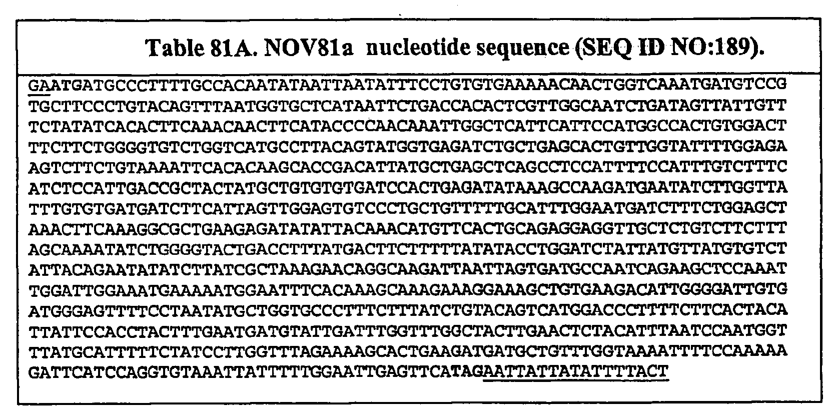

The disclosed NOV81a polypeptide (SEQ ID NO:190) encoded by SEQ ID NO:189 has 339 amino acid residues and is presented in Table 81B using the one-letter amino acid code.

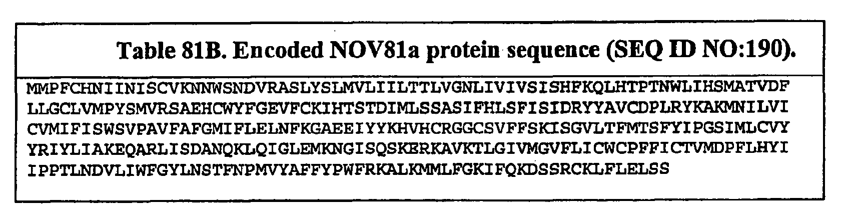

A search of sequence databases reveals that the NOV81a amino acid sequence has 152/299 (50%) identity and 206/299 (68%) similarity to SPTREMBL-ACC:Q9P1P4 G PROTEIN-COUPLED RECEPTOR 57 - Homo sapiens. Public amino acid databases include the GenBank databases, SwissProt, PDB and PIR.

### NOV81b

A disclosed NOV81b nucleic acid of 1039 nucleotides (also referred to as CG57847-02) encoding a GPCR-like protein is shown in Table 81C. The start and stop codons are in bold letters.

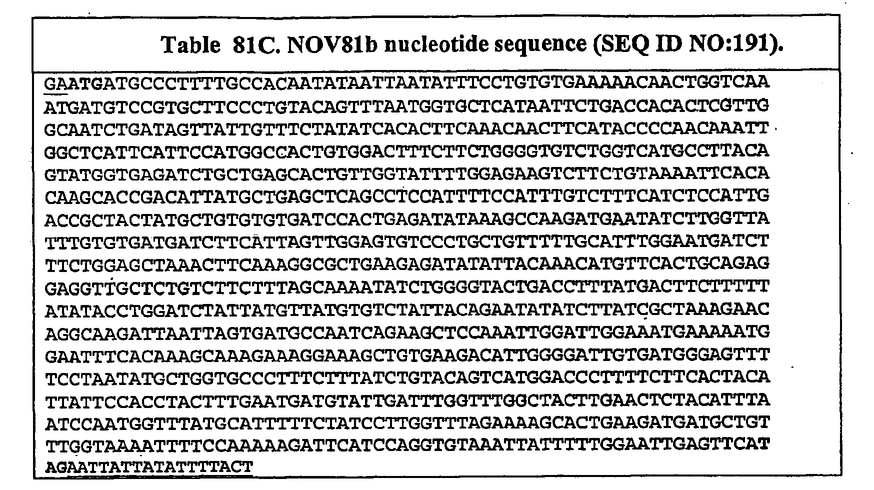

In a search of public sequence databases, the NOV81b nucleic acid sequence, located on chromsome 6 has 616 of 979 bases (62%) identical to a gb:GENBANK-ID:HSU88828|acc:U88828.1 mRNA from Homo sapiens (Homo sapiens serotonin-4-receptor-like pseudogene). Public nucleotide databases include all GenBank databases and the GeneSeq patent database.

The disclosed NOV81b polypeptide (SEQ ID NO:192) encoded by SEQ ID NO:191 has 339 amino acid residues and is presented in Table B using the one-letter amino acid code. Signal P, Psort and/or Hydropathy results predict that NOV81b has a signal peptide and is likely to be localized in the plasma membrane with a certainty of 0.6000. The most likely cleavage site for a NOV81b peptide is between amino acids 47 and 48.

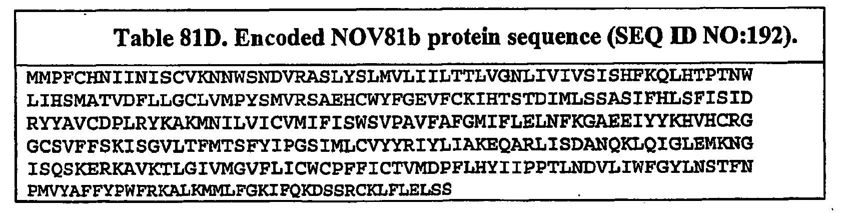

A search of sequence databases reveals that the NOV81b amino acid sequence has 152 of 299 amino acid residues (50%) identical to, and 206 of 299 amino acid residues (68%) similar to, the 343 amino acid residue ptnr:SPTREMBL-ACC:Q9P1P4 protein from Homo sapiens (Human) (G PROTEIN-COUPLED RECEPTOR 57). Public amino acid databases include the GenBank databases, SwissProt, PDB and PIR.

NOV81b is expressed in at least Apical microvilli of the retinal pigment epithelium, arterial (aortic), basal forebrain, brain, Burkitt lymphoma cell lines, corpus callosum, cardiac (atria and ventricle), caudate nucleus, CNS and peripheral tissue, cerebellum, cerebral cortex, colon, cortical neurogenic cells, endothelial (coronary artery and umbilical vein) cells, palate epithelia, eye, neonatal eye, frontal cortex, fetal hematopoietic cells, heart, hippocampus, hypothalamus, leukocytes, liver, fetal liver, lung, lung lymphoma cell lines, fetal lymphoid tissue, adult lymphoid tissue, Those that express MHC II and III nervous, medulla, subthalamic nucleus, ovary, pancreas, pituitary, placenta, pons, prostate, putamen, serum, skeletal muscle, small intestine, smooth muscle (coronary artery in aortic) spinal cord, spleen, stomach, taste receptor cells of the tongue, testis, thalamus, and thymus tissue. This information was derived by determining the tissue sources of the sequences that were included in the invention including but not limited to SeqCalling sources, Public EST sources, Literature sources, and/or RACE sources.

The disclosed NOV81b polypeptide has homology to the amino acid sequences shown in the BLASTP data listed in Table 81E.

| **Table 81E. BLAST results for NOV81b** | | | | | |
|---|---|---|---|---|---|
| Gene Index/ Identifier | Protein/ Organism | Length (aa) | Identity (%) | Positives (%) | Expect |
| gi\|10441577\|gb \|AAG1 7112.1 \|AF200627 1 (AF200627) | putative catecholamine receptor [Homo sapiens] | 339 | 339/339 (100%) | 339/339 (100%) | e-177 |
| gi\|17453976\|ref\|XP 069048.1\| (XM_069048) | similar to trace amine receptor 1 [Homo sapiens] | 338 | 338/338 (100%) | 338/338 (100%) | e-176 |
| gi\|14600076\|gb\|AAK7 1237.1\|AF380186 1 (AF380186) | trace amine receptor 1 [Rattus norvegicus] | 332 | 261/334 (78%), Positives = 288/334 (86%), | 261/334 (78%), Positives = 288/334 (86%), | e-136 |
| gi\|18182341\|gb\|AAL6 5137.1\|AF421352 1 (AF421352) | trace amine receptor 1 [Rattus norvegicus] | 332 | 261/334 (78%), Positives = 287/334 (85%), | 261/334 (78%), Positives 287/334 (85%), | e-136 |
| gi\|167165131\|ref \| NP 444435.1\| (NM_053205) | trace amine receptor 1 [Mus musculus] | 332 | 252/334 (75%), Positives = 283/334 (84%), | 252/334 (75%), Positives = 283/334 (84%), | e-133 |

Table 81F lists the domain descriptions from DOMAIN analysis results against NOV81b. This indicates that the NOV81b sequence has properties similar to those of other proteins known to contain this domain.

| **Table 81F. Domain Analysis of NOV 81b** | |
|---|---|
| gnl\|Pfam\|pfam00001, 7tm_1, 7 transmembrane receptor (rhodopsin family). | |
| | CD-Length = 254 residues, 100.0% aligned |
| | Score = 142 bits (358), Expect = 3e-35 |

The disclosed NOV81b nucleic acid of the invention encoding a GPCR-like protein includes the nucleic acid whose sequence is provided in Table 81A or a fragment thereof. The invention also includes a mutant or variant nucleic acid any of whose bases may be changed from the corresponding base shown in Table 81A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV81b protein of the invention includes the GPCR-like protein whose sequence is provided in Table 81B. The invention also includes a mutant or variant protein any of whose residues may be changed from the corresponding residue shown in Table 81B while still encoding a protein that maintains its GPCR-like activities and physiological functions, or a functional fragment thereof. In the mutant or variant protein, up to about 50 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV81b) may function as a member of a "GPCR family". Therefore, the NOV81b nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV81b nucleic acids and proteins of the invention are useful in potential therapeutic applications implicated in diseases including but not limited to various pathologies and disorders as indicated below. For example, a cDNA encoding the GPCR-like protein (NOV81b) may be useful in gene therapy, and the GPCR-like protein (NOV81b) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV81b nucleic acid encoding the GPCR-like protein of the invention, or fragments thereof, may further be useful in diagnostic applications, wherein the presence or amount of the nucleic acid or the protein are to be assessed.

NOV81b nucleic acids and polypeptides are further useful in the generation of antibodies that bind immuno-specifically to the novel NOV81b substances for use in therapeutic or diagnostic methods. These antibodies may be generated according to methods known in the art, using prediction from hydrophobicity charts, as described in the "Anti-NOVX Antibodies" section below. The disclosed NOV81b proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV82

A disclosed NOV82 nucleic acid of 1033 nucleotides (also referred to as CG57845-01) encoding a GPCR-like protein is shown in Table 82A. The start and stop codons are in bold letters.

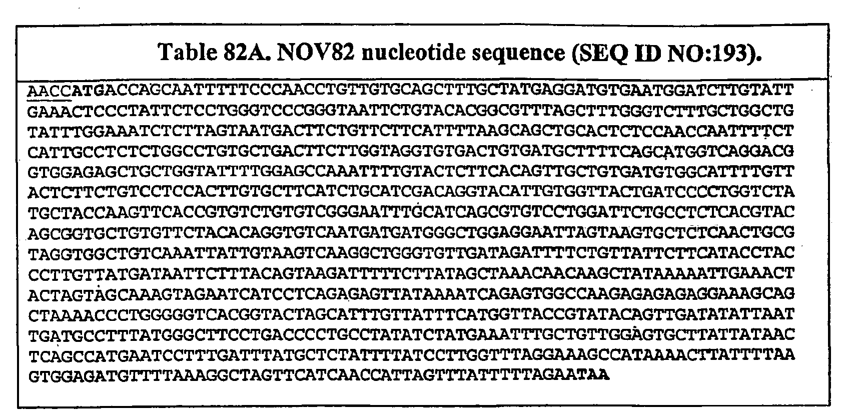

The disclosed NOV82 polypeptide (SEQ ID NO:194) encoded by SEQ ID NO:193 has 342 amino acid residues and is presented in Table 82B using the one-letter amino acid code.

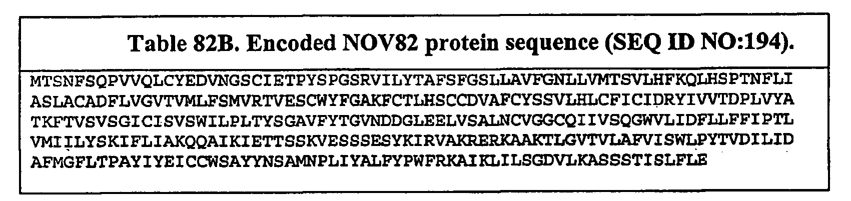

A search of sequence databases reveals that the NOV82 amino acid sequence has 145/330 (43%) identity and 214/330 (64%) similarity with SPTREMBL-ACC:O14804 PUTATIVE NEUROTRANSMITTER RECEPTOR - Homo sapiens. Public amino acid databases include the GenBank databases, SwissProt, PDB and PIR.

The disclosed NOV82 polypeptide has homology to the amino acid sequences shown in the BLASTP data listed in Table 82C.

| **Table 82C. BLAST results for NOV82** | | | | | |
|---|---|---|---|---|---|
| Gene Index/ Identifier | Protein/ Organism | Length (aa) | Identity (%) | Positives (%) | Expect |
| gi\|16751917\|ref\|NP 444508.1 (NM_053278) | G protein-coupled receptor 102; trace amine receptor 5 [Homo sapiens] | 342 | 342/342 (100%) | 342/342 (100%) | e-166 |
| gi\|14600102\|gb AAK7 1250.1\| AF380199 1 (AF380199 | similar to trace amine receptor 4 (H. sapiens) [Homo sapiens] | 374. | 274/344 (79%) | 308/344 (88%) | e-143 |
| gi\|17453968\|ref\|XP 069046.1\| (XM_069046 | Similar to trace amine receptor 4 [Rattus norvegicus] | 345 | 273/343 (79%) | 303/343 (87%) | e-142 |
| gi\|14600086\|gb\|AAK7 1242.1\|AF380191 1 (AF380191) | trace amine receptor 4 [Rattus norvegicus] | 345 | 266/343 (77%) | 302/343 (87%) | e-142 |
| gi\|14600100\|gb\|AAK7 1249.1\|AF380198 1 (AF380198 | trace amine receptor 10 [Rattus norvegicus] | 344 | 274/344 (79%) | 305/344 (88%) | e-141 |

Table 82D lists the domain descriptions from DOMAIN analysis results against NOV82. This indicates that the NOV82 sequence has properties similar to those of other proteins known to contain this domain.

| **Table 82D. Domain Analysis of NOV82** | |
|---|---|
| gn1\|Pfam\|pfam00001, 7tm_1, 7 transmembrane receptor (rhodopsin family). | |
| | CD-Length = 254 residues, 100.0% aligned |
| | Score = 132 bits (331), Expect = 4e-32 |

The disclosed NOV82 nucleic acid of the invention encoding a GPCR-like protein includes the nucleic acid whose sequence is provided in Table 82A or a fragment thereof. The invention also includes a mutant or variant nucleic acid any of whose bases may be changed from the corresponding base shown in Table 82A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV82 protein of the invention includes the GPCR-like protein whose sequence is provided in Table 82B. The invention also includes a mutant or variant protein any of whose residues may be changed from the corresponding residue shown in Table 82B while still encoding a protein that maintains its GPCR-like activities and physiological functions, or a functional fragment thereof. In the mutant or variant protein, up to about 57 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV82) may function as a member of a "GPCR family". Therefore, the NOV82 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV82 nucleic acids and proteins of the invention are useful in potential therapeutic applications implicated in diseases including but not limited to various pathologies and disorders as indicated below. For example, a cDNA encoding the GPCR-like protein (NOV82) may be useful in gene therapy, and the GPCR-like protein (NOV82) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV82 nucleic acid encoding the GPCR-like protein of the invention, or fragments thereof, may further be useful in diagnostic applications, wherein the presence or amount of the nucleic acid or the protein are to be assessed.

NOV82 nucleic acids and polypeptides are further useful in the generation of antibodies that bind immuno-specifically to the novel NOV82 substances for use in therapeutic or diagnostic methods. These antibodies may be generated according to methods known in the art, using prediction from hydrophobicity charts, as described in the "Anti-NOVX Antibodies" section below. The disclosed NOV82 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV83

A disclosed NOV83 nucleic acid of 1045 nucleotides (also referred to as CG57843-01) encoding a GPCR-like protein is shown in Table 83A. The start and stop codons are in bold letters.

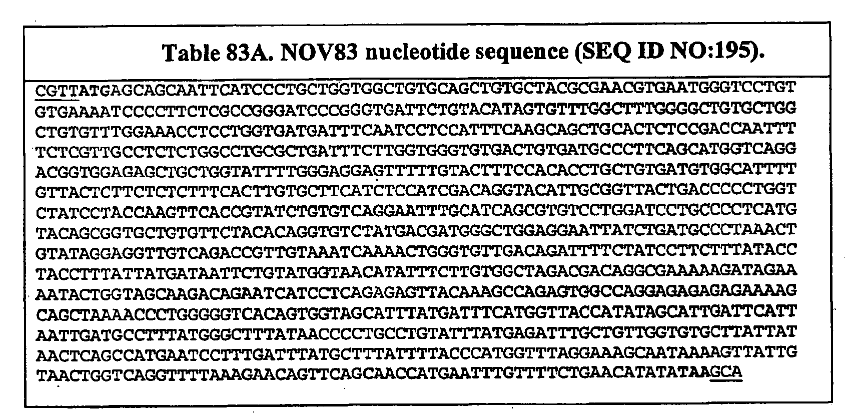

The disclosed NOV83 polypeptide (SEQ ID NO:196) encoded by SEQ ID NO:195 has 345 amino acid residues and is presented in Table 83B using the one-letter amino acid code.

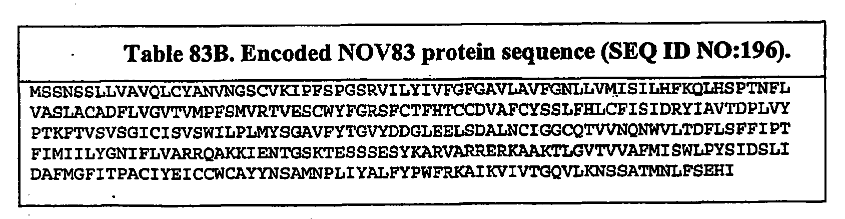

A search of sequence databases reveals that the NOV83 amino acid sequence has 146/330 (44%) identity and 216/330 (65%) similarity with SPTREMBL-ACC:O14804 PUTATIVE NEUROTRANSMITTER RECEPTOR - Homo sapiens. Public amino acid databases include the GenBank databases, SwissProt, PDB and PIR.

The disclosed NOV83 polypeptide has homology to the amino acid sequences shown in the BLASTP data listed in Table 83C.

| **Table 83C. BLAST results for NOV83** | | | | | |
|---|---|---|---|---|---|
| Gene Index/ Identifier | Protein/ Organism | Length (aa) | Identity (%) | Positives (%) | Expect |
| gi\|17453968\|ref\|XP 069046.1\| (XM_069046) | similar to trace amine receptor 4 (H. sapiens) | 345 | 345/345 (100%) | 345/345 (100%) | e-180 |
| gi\|14600086\|gb\|AAK7 1242.11\|AF380191 1 (AF380191) | trace amine receptor 4 [Rattus norvegicus] | 345 | 302/345 (87%) ' | 321/345 (92%) | e-162 |
| gi\|14600102\|gb\|AAK7 1250.1 \|AF380199 1 (AF380199) | trace amine receptor 11 [Rattus norvegicus] | 373 | 265/345 (76%) | 306/345 (87%) | e-141 |
| gi\|16751917\|ref\|NP 444508.1\| (NM_053278) | G protein-coupled receptor 102; trace amine receptor 5 [Homo sapiens] | 342 | 273/343 (79%) | 303/343 (87%) | e-137 |
| gi\|14600094\|gb\|AAK7 1246.1\|AF380195 1 (AF380195) | trace amine receptor 7 [Rattus norvegicus] | 344 | 258/345 (74%) | 302/345 (86%) | e-137 |

Table 83D lists the domain descriptions from DOMAIN analysis results against NOV83. This indicates that the NOV83 sequence has properties similar to those of other proteins known to contain this domain.

| **Table 83D. Domain Analysis of NOV83** | |
|---|---|
| gnl\|Pfam\|pfam00001, 7tm-1, 7 transmembrane receptor (rhodopsin family). | |
| | CD-Length = 254 residues,100.0% aligned |
| | Score = 130 bits (326), Expect = 2e-31 |

The disclosed NOV83 nucleic acid of the invention encoding a GPCR-like protein includes the nucleic acid whose sequence is provided in Table 83A or a fragment thereof. The invention also includes a mutant or variant nucleic acid any of whose bases may be changed from the corresponding base shown in Table 83A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV83 protein of the invention includes the GPCR like protein whose sequence is provided in Table 83B. The invention also includes a mutant or variant protein any of whose residues maybe changed from the corresponding residue shown in Table 83B while still encoding a protein that maintains its GPCR-like activities and physiological functions, or a functional fragment thereof. In the mutant or variant protein, up to about 56 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV83) may function as a member of a "GPCR family". Therefore, the NOV83 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV83 nucleic acids and proteins of the invention are useful in potential therapeutic applications implicated in diseases including but not limited to various pathologies and disorders as indicated below. For example, a cDNA encoding the GPCR-like protein (NOV83) may be useful in gene therapy, and the GPCR-like protein (NOV83) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV83 nucleic acid encoding the GPCR-like protein of the invention, or fragments thereof, may further be useful in diagnostic applications, wherein the presence or amount of the nucleic acid or the protein are to be assessed.

NOV83 nucleic acids and polypeptides are further useful in the generation of antibodies that bind immuno-specifically to the novel NOV83 substances for use in therapeutic or diagnostic methods. These antibodies may be generated according to methods known in the art, using prediction from hydrophobicity charts, as described in the "Anti-NOVX Antibodies" section below. The disclosed NOV83 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV84

NOV84 includes two GPCR-like proteins disclosed below. The disclosed sequences have been named NOV84a and NOV84b.

### NOV84a

A disclosed NOV84a nucleic acid of 948 nucleotides (also referred to as CG57841-01) encoding a GPCR-like protein is shown in Table 84A. The start and stop codons are in bold letters.

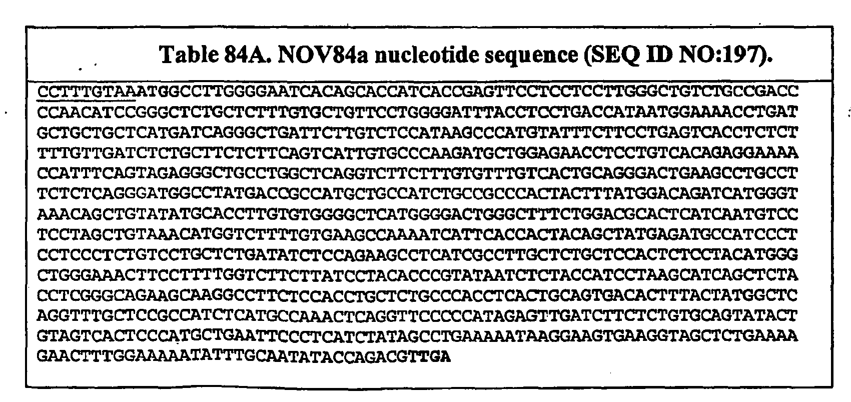

The disclosed NOV84a polypeptide (SEQ ID NO:198) encoded by SEQ ID NO:197 has 312 amino acid residues and is presented in Table 84B using the one-letter amino acid code.

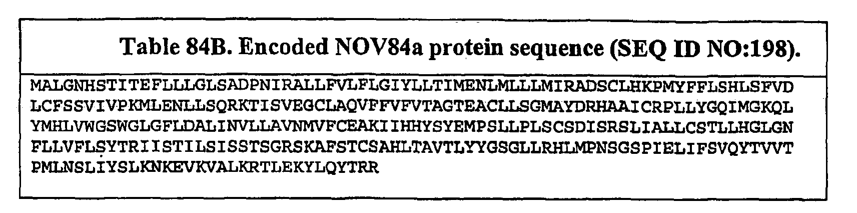

A search of sequence databases reveals that the NOV84a amino acid sequence has 138/309 (44%) identity and 203/309 (65%) similarity with TREMBLNEW-ACC:AAG39860 ODORANT RECEPTOR K15 - Mus musculus. Public amino acid databases include the GenBank databases, SwissProt, PDB and PIR.

### NOV84b

A disclosed NOV84b nucleic acid of 1039 nucleotides (also referred to as CG57841-02) encoding a GPCR-like protein is shown in Table 84C. The start and stop codons are in bold letters.

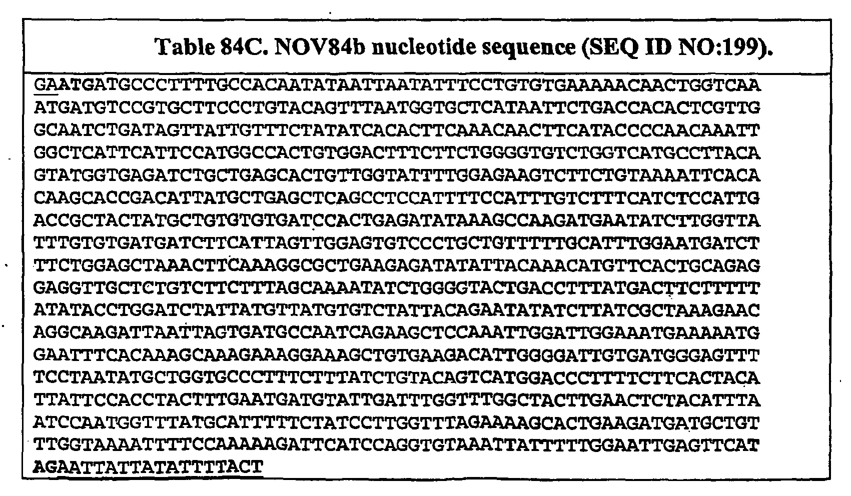

In a search of public sequence databases, the NOV84b nucleic acid sequence, located on chromsome 6 has 616 of 979 bases (62%) identical to a gb:GENBANK-ID:HSU88828|acc:U88828.1 mRNA from Homo sapiens (Homo sapiens serotonin-4-receptor-like pseudogene). Public nucleotide databases include all GenBank databases and the GeneSeq patent database.

The disclosed NOV84b polypeptide (SEQ ID NO:200) encoded by SEQ ID NO:199 has 339 amino acid residues and is presented in Table 84D using the one-letter amino acid code. Signal P, Psort and/or Hydropathy results predict that NOV84b has a signal peptide and is likely to be localized in the plasma membrane with a certainty of 0.6000. The most likely cleavage site for a NOV84b peptide is between amino acids 47 and 48.

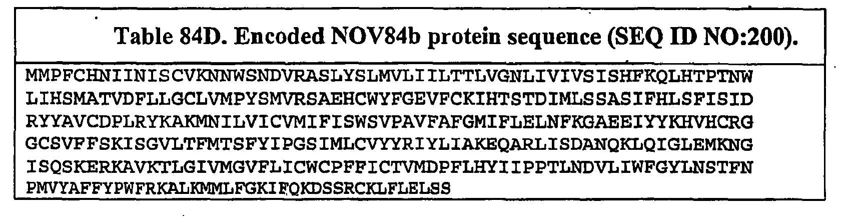

A search of sequence databases reveals that the NOV84b amino acid sequence has 152 of 299 amino acid residues (50%) identical to, and 206 of 299 amino acid residues (68%) similar to, the 343 amino acid residue ptnr:SPTREMBL-ACC:Q9P1P4 protein from Homo sapiens (Human) (G PROTEIN-COUPLED RECEPTOR 57). Public amino acid databases include the GenBank databases, SwissProt, PDB and PIR.

NOV84b is expressed in at least Apical microvilli of the retinal pigment epithelium, arterial (aortic), basal forebrain, brain, Burkitt lymphoma cell lines, corpus callosum, cardiac (atria and ventricle), caudate nucleus, CNS and peripheral tissue, cerebellum, cerebral cortex, colon, cortical neurogenic cells, endothelial (coronary artery and umbilical vein) cells, palate epithelia, eye, neonatal eye, frontal cortex, fetal hematopoietic cells, heart, hippocampus, hypothalamus, leukocytes, liver, fetal liver, lung, lung lymphoma cell lines, fetal lymphoid tissue, adult lymphoid tissue, Those that express MHC II and III nervous, medulla, subthalamic nucleus, ovary, pancreas, pituitary, placenta, pons, prostate, putamen, serum, skeletal muscle, small intestine, smooth muscle (coronary artery in aortic) spinal cord, spleen, stomach, taste receptor cells of the tongue, testis, thalamus, and thymus tissue. This information was derived by determining the tissue sources of the sequences that were included in the invention including but not limited to SeqCalling sources, Public EST sources, Literature sources, and/or RACE sources.

The disclosed NOV84b polypeptide has homology to the amino acid sequences shown in the BLASTP data listed in Table 84E.

| **Table 84E. BLAST results for NOV84b** | | | | | |
|---|---|---|---|---|---|
| Gene Index/ Identifier | Protein/ Organism | Length (aa) | Identity (%) | Positives (%) | Expect |
| gi\|174746031\|ref\|Xp 062552.1\| (XM_062552) | similar to olfactory receptor [Homo sapiens] | 312 | 312/312 (100%) | 312/312 (100%) | e-145 |
| gi\|18479402\|gb\|AAL6 0715.1\| (AY073052) | olfactory receptor MOR160-1. [Mus musculus] | 309 | 216/304 (71%) | 254/304 (83%) | e-105 |
| gi\|18480580\|gb\|AAL6 1304.1\| (AY073641) | olfactory receptor MOR160-2 [Mus musculus] | 308 | 212/307 (69%) | 256/307 (83%) | e-95 |
| gi\|18480924\|gb\|AAL6 1476.1\|(AY073813) | olfactory receptor MOR160-5 [Mus musculus] | 311 | 211/309 (68%) | 258/309 (83%) | e-94 |
| gi\|18480922\|gb \|AAL6 1475.1\|(AY073812) | olfactory receptor MOR160-4 [Mus musculus] | 305 | 184/302 (60%) | 233/302 (76%) | 6e-84 |

Table 84F lists the domain descriptions from DOMAIN analysis results against NOV84b. This indicates that the NOV84b sequence has properties similar to those of other proteins known to contain this domain.

| **Table 84F. Domain Analysis of NOV84b** | |
|---|---|
| gn1\|Pfam\|pfam00001, 7tm_1,7 transmembrane receptor (rhodopsin family). | |
| | CD-Length = 254 residues, 44.9% aligned |
| | Score = 68.6 bits (166), Expect = 5e-13 |

The disclosed NOV84b nucleic acid of the invention encoding a GPCR-like protein includes the nucleic acid whose sequence is provided in Table 84A or a fragment thereof. The invention also includes a mutant or variant nucleic acid any of whose bases may be changed from the corresponding base shown in Table 84A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 38 percent of the bases may be so changed.

The disclosed NOV84b protein of the invention includes the GPCR-like protein whose sequence is provided in Table B. The invention also includes a mutant or variant protein any of whose residues may be changed from the corresponding residue shown in Table 84B while still encoding a protein that maintains its GPCR-like activities and physiological functions, or a functional fragment thereof. In the mutant or variant protein, up to about 50 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV84b) may function as a member of a "GPCR family". Therefore, the NOV84b nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV84b nucleic acids and proteins of the invention are useful in potential therapeutic applications implicated in diseases including but not limited to various pathologies and disorders as indicated below. For example, a cDNA encoding the GPCR-like protein (NOV84b) may be useful in gene therapy, and the GPCR-like protein (NOV84b) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon Cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV84b nucleic acid encoding the GPCR-like protein of the invention, or fragments thereof, may further be useful in diagnostic applications, wherein the presence or amount of the nucleic acid or the protein are to be assessed.

NOV84b nucleic acids and polypeptides are further useful in the generation of antibodies that bind immuno-specifically to the novel NOV84b substances for use in therapeutic or diagnostic methods. These antibodies may be generated according to methods known in the art, using prediction from hydrophobicity charts, as described in the "Anti-NOVX Antibodies" section below. The disclosed NOV84b proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV85

A disclosed NOV85 nucleic acid of 963 nucleotides (also referred to as CG57839-01) encoding a GPCR-like protein is shown in Table 85A. The start and stop codons are in bold letters.

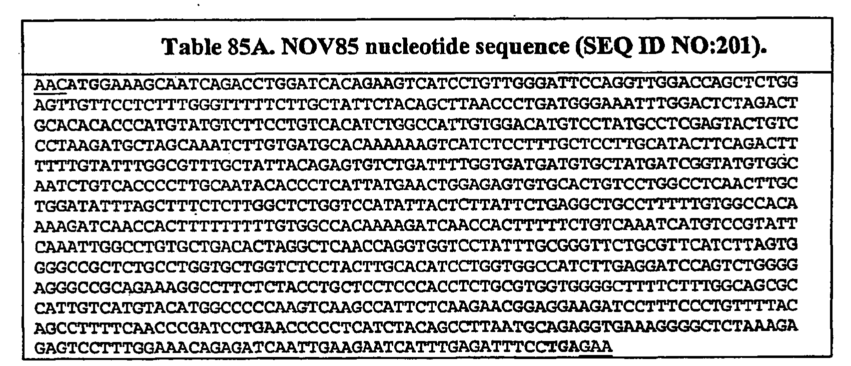

The disclosed NOV85 polypeptide (SEQ ID N0:202) encoded by SEQ ID NO:201 has 318 amino acid residues and is presented in Table 85B using the one-letter amino acid code

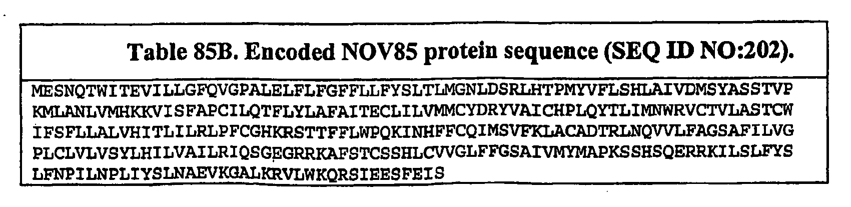

A search of sequence databases reveals that the NOV85 amino acid sequence has 181/311 (58%) identity and 232/311 (74%) similarity with SPTREMBL-ACC:O95047 WUGSC:H_DJ0988G15.2 PROTEIN - Homo sapiens. Public amino acid databases include the GenBank databases, SwissProt, PDB and PIR.

The disclosed NOV85 polypeptide has homology to the amino acid sequences shown in the BLASTP data listed in Table 85C.

| **Table 85C. BLAST results for NOV85** | | | | | |
|---|---|---|---|---|---|
| Gene Index/ Identifier | Protein/ Organism | Length (aa) | Identity (%) | Positives (%) | Expect |
| gi\|18565914\|ref\|XP 095005.1\| (XM_095005) | protein XP_095005 [Homo sapiens] | 310 | 299/320 (93%) | 300/320 (93%) | e-134 |
| gi\|18480894\|gb\|AAL6 1461.1 (AY073798) | olfactory receptor MOR261-12 [Mus musculus] | 308 | 254/317 (80%) | 277/317 (87%) | e-115 |
| gi\|18480182\|gb\|AAL6 1105.1 (AY073442 | olfactory receptor MOR261-4 [Mus musculus] | 310 | 238/320 (74%) | 266/320 (82%) | e-111 |
| gi\|18480180\|gb\|AAL6 1104.1\| (AY073441) | olfactory receptor MOR261-3 [Mus musculus] | 310 | 235/320 (73%) | 263/320 (81%) | e-110 |
| gi\|18565912\|ref\|XP 069619.2 (XM_069619 | similar to olfactory receptor [Homo sapiens]' | 311 | 231/320 (72%) | 258/320 (80%) | e-108 |

Table 85D lists the domain descriptions from DOMAIN analysis results against NOV85. This indicates that the NOV85 sequence has properties similar to those of other proteins known to contain this domain. -

| **Table 85D. Domain Analysis of NOV85** | |
|---|---|
| gnl\|Pfam\|pfam00001, 7tm_1, 7 transmembrane receptor (rhodopsin family). | |
| | CD-Length = 254 residues, 94.9% aligned |
| | Score = 111 bits (278), Expect = 5e-26 |

The disclosed NOV85 nucleic acid of the invention encoding a GPCR-like protein includes the nucleic acid whose sequence is provided in Table 85A or a fragment thereof. The invention also includes a mutant or variant nucleic acid any of whose bases may be changed from the corresponding base shown in Table 85A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV85 protein of the invention includes the GPCR-like protein whose sequence is provided in Table 85B. The invention also includes a mutant or variant protein any of whose residues may be changed from the corresponding residue shown in Table 85B while still encoding a protein that maintains its GPCR-like activities and physiological functions, or a functional fragment thereof. In the mutant or variant protein, up to about 42 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV85) may function as a member of a "GPCR family". Therefore, the NOV85 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV85 nucleic acids and proteins of the invention are useful in potential therapeutic applications implicated in diseases including but not limited to various pathologies and disorders as indicated below. For example, a cDNA encoding the GPCR-like protein (NOV85) may be useful in gene therapy, and the GPCR-like protein (NOV85) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV85 nucleic acid encoding the GPCR-like protein of the invention, or fragments thereof, may further be useful in diagnostic applications, wherein the presence or amount of the nucleic acid or the protein are to be assessed.

NOV85 nucleic acids and polypeptides are further useful in the generation of antibodies that bind immuno-specifically to the novel NOV85 substances for use in therapeutic or diagnostic methods. -,These antibodies may be generated according to methods known in the art, using prediction from hydrophobicity charts, as described in the "Anti-NOVX Antibodies" section below. The disclosed NOV85 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV86

A disclosed NOV86 nucleic acid of 971 nucleotides (also referred to as CG-01) encoding a -like protein is shown in Table 86A. The start and stop codons are in bold letters.

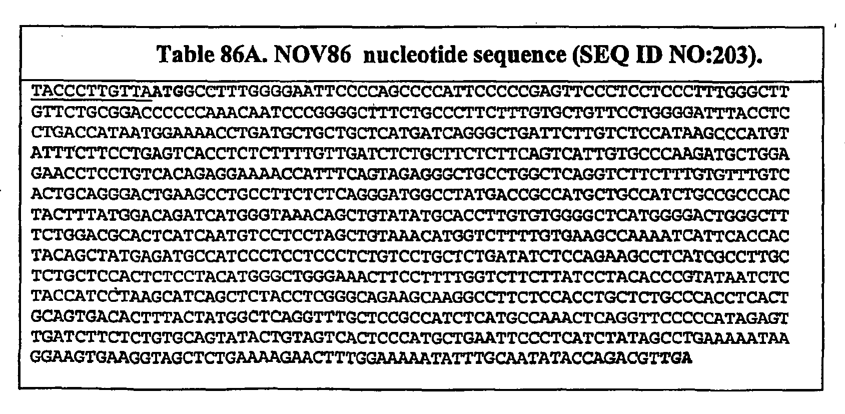

The disclosed NOV86 polypeptide (SEQ ID NO:204) encoded by SEQ ID NO:203 has 319 amino acid residues and is presented in Table 86B using the one-letter amino acid code.

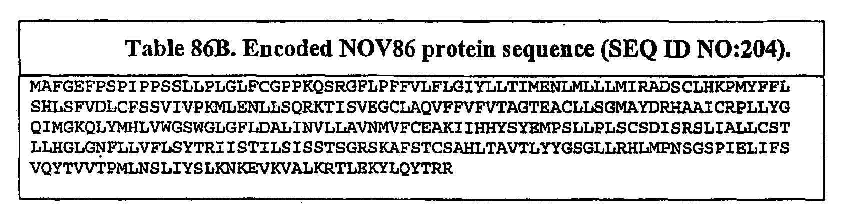

A search of sequence databases reveals that the NOV86 amino acid sequence has 134/300 (44%) identity and 192/300 (64%) similarity with SPTREMBL-ACC:035184 OLFACTORY RECEPTOR - Rattus norvegicus. Public amino acid databases include the GenBank databases, SwissProt, PDB and PIR.

The disclosed NOV86 polypeptide has homology to the amino acid sequences shown in the BLASTP data listed in Table 86C.

| **Table 86C. BLAST results for NOV86** | | | | | |
|---|---|---|---|---|---|
| Gene Index/ Identifier | Protein/ Organism | Length (aa) | Identity (%) | Positives (%) | Expect |
| gi\|17474603\|ref\|XP 062552.1 (XM_062552) | similar to olfactory receptor [Homo sapiens] | 312 | 290/307 (94%) | 293/307 (94%), | e-134 |
| gi\|18479402\|gb\|AAL6 0715.1 (AY073052) | olfactory receptor MOR160-1 [Mus musculus] | 309 | 200/281 (71%) | 234/281 (83%) | 3e-97 |
| gi\|18480580\|gb\|AAL6 1304.1\| (AY073641) | olfactory receptor MOR160-2 [Mus musculus] | 308 | 193/280 (68%) | 236/280 (83%) | 8e-88 |
| gi\|18480924\|gb\|AAL6 1476.1\| (AY073813) | olfactory receptor MOR160-5 [Mus musculus] | 311 | 192/282 (68%) | 237/282 (83%) | 5e-87 |
| gi\|18480922\|gb\|AAL6 1475.1\| \|(AY073812) | olfactory receptor MOR160-4 [Mus musculus] | 305 | 170/279 (60%) | 215/279 (76%) | e-78 |

Table 86D lists the domain descriptions from DOMAIN analysis results against NOV86. This indicates that the NOV86 sequence has properties similar to those of other proteins known to contain this domain.

| **Table 86D. Domain Analysis of NOV86** | |
|---|---|
| gn\|Pfam\|pfam00001, 7tm_1, 7 transmembrane receptor (rhodopsin family). | |
| | CD-Length = 254 residues, 44.9% aligned |
| | Score = 68.2 bits (165), Expect = 7e-13 |

The disclosed NOV86 nucleic acid of the invention encoding a GPCR-like protein includes the nucleic acid whose sequence is provided in Table 86A or a fragment thereof. The invention also includes a mutant or variant nucleic acid any of whose-bases may be changed from the corresponding base shown in Table 86A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV86 protein of the invention includes the GPCR-like protein whose sequence is provided in Table 86B. The invention also includes a mutant or variant protein any of whose residues may be changed from the corresponding residue shown in Table 86B while still encoding a protein that maintains its GPCR-like activities and physiological functions, or a functional fragment thereof. In the mutant or variant protein, up to about 56 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV86) may function as a member of a "GPCR family". Therefore, the NOV86 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV86 nucleic acids and proteins of the invention are useful in potential therapeutic applications implicated in diseases including but not limited to various pathologies and disorders as indicated below. For example, a cDNA encoding the GPCR-like protein (NOV86) may be useful in gene therapy, and the GPCR-like protein (NOV86) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV86 nucleic acid encoding the GPCR-like protein of the invention, or fragments thereof, may further be useful in diagnostic applications, wherein the presence or amount of the nucleic acid or the protein are to be assessed.

NOV86 nucleic acids and polypeptides are further useful in the generation of antibodies that bind immuno-specifically to the novel NOV86 substances for use in therapeutic or diagnostic methods. These antibodies may be generated according to methods known in the art, using prediction from hydrophobicity charts, as described in the "Anti-NOVX Antibodies" section below. The disclosed NOV86 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV87

A disclosed NOV87 nucleic acid of 1067 nucleotides (also referred to as CG56763-01) encoding a GPCR-like protein is shown in Table 87A. The start and stop codons are in bold letters.

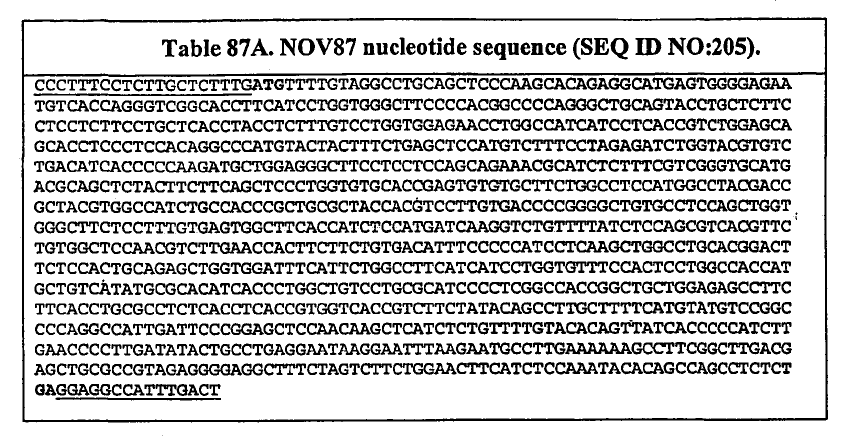

The disclosed NOV87 polypeptide (SEQ ID NO:206) encoded by SEQ ID NO:205 has 343 amino acid residues and is presented in Table 87B using the one-letter amino acid code.

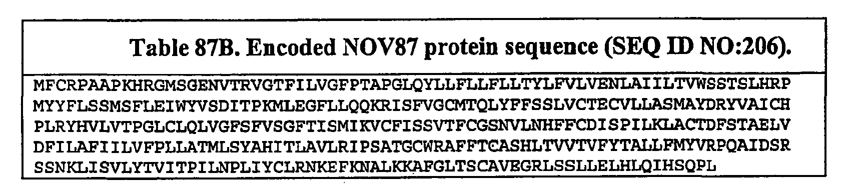

A search of sequence databases reveals that the NOV87 amino acid sequence has 211/306 (68%) identity and 250/306 (81 %) similarity with TREMBLNEW-ACC:AAG45189 M51 OLFACTORY RECEPTOR - Mus musculus. Public amino acid databases include the GenBank databases, SwissProt, PDB and PIR.

The disclosed NOV87 polypeptide has homology to the amino acid sequences shown in the BLASTP data listed in Table 87C.

| **Table 87C. BLAST results for NOV87** | | | | | |
|---|---|---|---|---|---|
| Gene Index/ Identifier | Protein/ Organism | Length (aa) | Identity (%) | Positives (%) | Expect |
| gi\|117435880\|ref\|XP 065375.1 (XM_065375) | similar to olfactory receptor 41 [Homo sapiens] | 331 | 331/331 (100%) | 331/331 (100%) | e-150 |
| gi\|17435888\|ref\|XF 065377.1\| (XM_065377) | similar to olfactory receptor 41 [Homo sapiens] | 312 | 293/307 (95%) | 300/307 (97%) | e-133 |
| gi\|18479396\|gb\|AAL6 0712.1\| (AY073049 | olfactory receptor MOR103-2 [Mus musculus] | 312 | 270/307 (87%) | 287/307 (92%) | e-122 |
| gi\|18479398\|gb\|AAL6 0713.1 (AY073050) | olfactory receptor MOR103-3 [Mus musculus] | 312 | 261/307 (85%) | 284/307 (92%) | e-120 |
| gi\|12007416\|gb\|AAG45189.1 (AF321234) | m51 olfactory receptor [Mus musculus] | 314 | 211/306 (68%) | 250/306 (80%) | e-101 |

Table 87D lists the domain descriptions from DOMAIN analysis results against NOV87. This indicates that the NOV87 sequence has properties similar to those of other proteins known to contain this domain.

| **Table 87D. Domain Analysis of NOV87** | |
|---|---|
| gn1\|Pfam\|pfam00001, 7tm_1, 7 transmembrane receptor (rhodopsin family). | |
| | CD-Length = 254 residues, 100.0% aligned |
| | Score = 98.2 bits (243), Expect = 7e-22 |

The disclosed NOV87 nucleic acid of the invention encoding a GPCR-like protein includes the nucleic acid whose sequence is provided in Table 87A or a fragment thereof. The invention also includes a mutant or variant nucleic acid any of whose bases may be changed from the corresponding base shown in Table 87A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV87 protein of the invention includes the GPCR-like protein whose sequence is provided in Table 87B. The invention also includes a mutant or variant protein any of whose residues may be changed from the corresponding residue shown in Table 87B while still encoding a protein that maintains its GPCR-like activities and physiological functions, or a functional fragment thereof. In the mutant or variant protein, up to about 32 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV87) may function as a member of a "GPCR family". Therefore, the NOV87 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV87 nucleic acids and proteins of the invention are useful in potential therapeutic applications implicated in diseases including but not limited to various pathologies and disorders as indicated below. For example, a cDNA encoding the GPCR-like protein (NOV87) may be useful in gene therapy, and the GPCR-like protein (NOV87) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV87 nucleic acid encoding the GPCR-like protein of the invention, or fragments thereof, may further be useful in diagnostic applications, wherein the presence or amount of the nucleic acid or the protein are to be assessed.

NOV87 nucleic acids and polypeptides are further useful in the generation of antibodies that bind immuno-specifically to the novel NOV87 substances for use in therapeutic or diagnostic methods. These antibodies may be generated according to methods known in the art, using prediction from hydrophobicity charts, as described in the "Anti-NOVX Antibodies" section below. The disclosed NOV87 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV88

A disclosed NOV88 nucleic acid of 939 nucleotides (also referred to as CG56753-01) encoding a GPCR-like protein is shown in Table 88A. The start and stop codons are in bold letters.

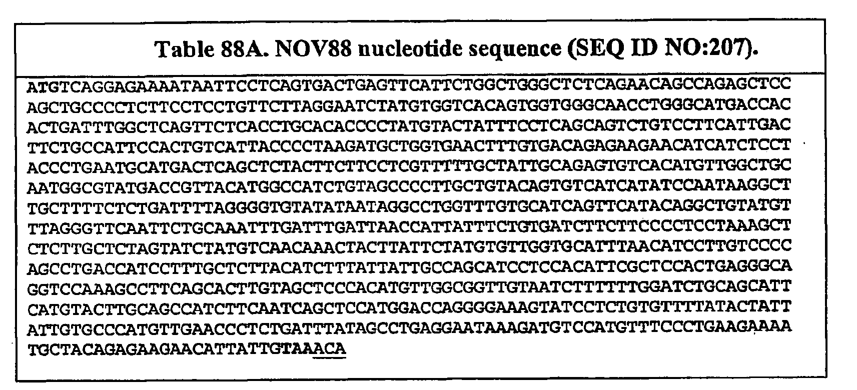

The disclosed NOV88 polypeptide (SEQ ID NO:208) encoded by SEQ ID NO:207 has 311 amino acid residues and is presented in Table 88B using the one-letter amino acid code.

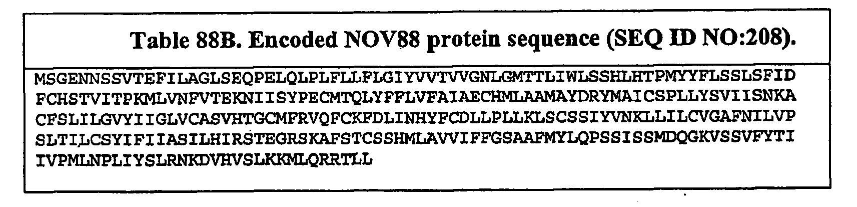

A search of sequence databases reveals that the NOV88 amino acid sequence has 239/311 (76%) identity and 275/311 (88%) similarity with TREMBLNEW-ACC:AAG39856 ODORANT RECEPTOR K11 - Mus musculus. Public amino acid databases include the GenBank databases, SwissProt, PDB and PIR.

The disclosed NOV88 polypeptide has homology to the amino acid sequences shown in the BLASTP data listed in Table 88C.

| **Table, 88C. BLAST results for NOV88** | | | | | |
|---|---|---|---|---|---|
| Gene Index/ Identifier | Protein/ Organism | Length (aa) | Identity (%) | Positives (%) | Expect |
| gi\|18578547\|ref\|XP 090109.1 (XM_090109) | olfactory receptor, family 8, subfamily G, member 1 [Homo sapiens] | 311 | 311/311 (100%) | 311/311 (100%) | e-141 |
| gi\|17472672\|ref\|XP 061794.1 <XM_061794) | similar to odorant receptor K11 [Homo sapiens] | 311 | 263/311 (84%) | 285/311 (91%) | e-122 |
| gi\|18479824\|gb\|AAL6 0926.1\| (AY073263) | olfactory receptor MOR171-5 [Mus musculus] | 314 | 240/311 (77%) | 276/311 (88%) | e-114 |
| gi\|11692519\|gb\|AAG3 9856.1\|AP282271 1 (AF282271) | odorant receptor K11 [Mus musculus] | 314 | 239/311 (76%) | 275/311 (87%) | e-113 |
| gi 17472670 ref XP 061793.1 (XM_061793) | similar to odorant receptor K15 [Homo sapiens] | 258 | 258/258 (100%) | 258/258 (100%) | e-113 |

Table 88D lists the domain descriptions from DOMAIN analysis results against NOV88. This indicates that the NOV88 sequence has properties similar to those of other proteins known to contain this domain.

| **Table 88D. Domain Analysis of NOV88** | |
|---|---|
| gnl \|Pfam \|pfam00001, 7tm_1, 7 transmembrane receptor (rhodopsin family). | |
| | CD-Length = 254 residues, 100.0% aligned |
| | Score = 88.6 bits (218), Expect = 5e-19 |

The disclosed NOV88 nucleic acid of the invention encoding a GPCR-like protein includes the nucleic acid whose sequence is provided in Table 88A or a fragment thereof. The invention also includes a mutant or variant nucleic acid any of whose bases may be changed from the corresponding base shown in Table 88A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way ofnonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV88 protein of the invention includes the GPCR-like protein whose sequence is provided in Table 88B. The invention also includes a mutant or variant protein any of whose residues may be changed from the corresponding residue shown in Table 88B while still encoding a protein that maintains its GPCR-like activities and physiological functions, or a functional fragment thereof. In the mutant or variant protein, up to about 24 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV88) may function as a member of a "GPCR family". Therefore, the NOV88 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV88 nucleic acids and proteins of the invention are useful in potential therapeutic applications implicated in diseases including but not limited to various pathologies and disorders as indicated below. For example, a cDNA encoding the GPCR-like protein (NOV88) may be useful in gene therapy, and the GPCR-like protein (NOV88) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV88 nucleic acid encoding the GPCR-like protein of the invention, or fragments thereof, may further be useful in diagnostic applications, wherein the presence or amount of the nucleic acid or the protein are to be assessed.

NOV88 nucleic acids and polypeptides are further useful in the generation of antibodies that bind immuno-specifically to the novel NOV88 substances for use in therapeutic or diagnostic methods. These antibodies may be generated according to methods known in the art, using prediction from hydrophobicity charts, as described in the "Anti-NOVX Antibodies" section below. The disclosed NOV88 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV89

A disclosed NOV89 nucleic acid of 1003 nucleotides (also referred to as CG57670-01) encoding a GPCR-like protein is shown in Table 89A. The start and stop codons are in bold letters.

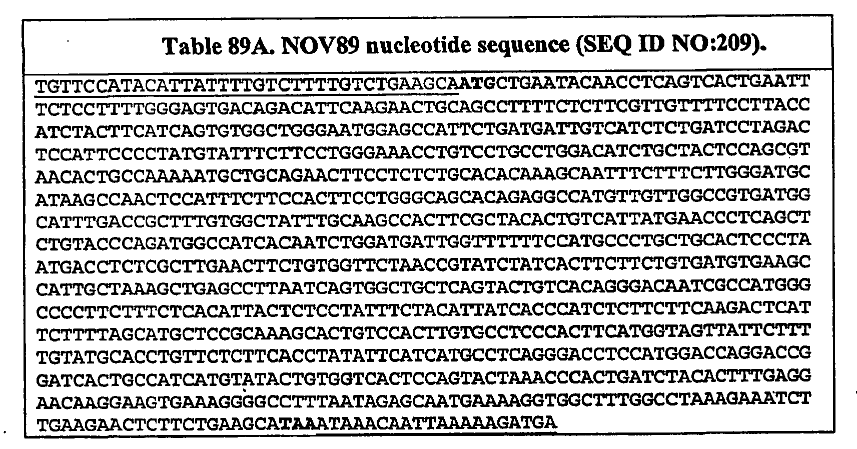

The disclosed NOV89 polypeptide (SEQ ID NO:210) encoded by SEQ ID NO:209 has 315 amino acid residues and is presented in Table 89B using the one-letter amino acid code

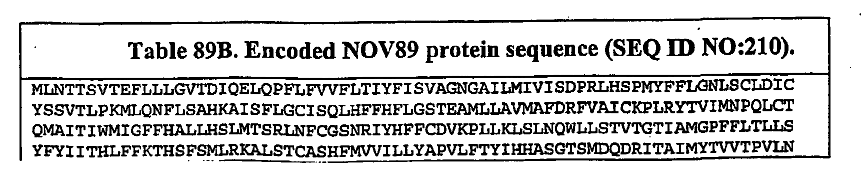

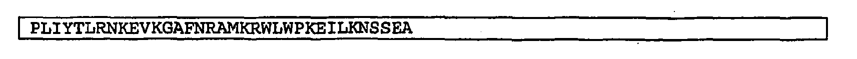

A search of sequence databases reveals that the NOV89 amino acid sequence has similarity with SPTREMBL-ACC:Q9UGF7 BA150A6.1 (NOVEL 7 TRANSMEMBRANE RECEPTOR (RHODOPSIN FAMILY)(OLFACTORY RECEPTOR LIKE) PROTEIN (HS6M1-27)) - Homo sapiens. Public amino acid databases include the GenBank databases, SwissProt, PDB and PIR.

The disclosed NOV89 polypeptide has homology to the amino acid sequences shown in the BLASTP data listed in Table 89C.

| **Table 89C. BLAST results for NOV89** | | | | | |
|---|---|---|---|---|---|
| Gene Index/ Identifier | Protein/ Organism | Length (aa) | Identity (%) | Positives (%) | Expect |
| gi \|17464355 \|ref \|XP 069464.1 \| (XM_069464) | similar to olfactory receptor, family 12, subfamily D, member 2 [Homo sapiens] | 284 | 279/315 (88%) | 280/315 (88%) | e-130 |
| gi\|18563691\|ref\|XP 094753.1\| (XM_094753) | hypothetical protein XP_094753 [Homo sapiens] | 284 | 278/315 (88%) | 279/315 (88%) | e-130 |
| gi\|7363443\|ref\|NP0 39224.1\|(NM_013936 | olfactory receptor, family 12, subfamily D, member 2 [Homo sapiens] | 307 | 269/306 (87%) | 281/306 (90%) | e-126 |
| gi\|18563689\|ref\|XP 084201.11 (XM_084201) | similar to olfactory receptor, family 12, subfamily D, member 2 (H. sapiens) [Homo sapiens] | 307 | 268/306 (87%) | 280/306 (90%) | e-125 |
| gi\|15020328\|emb\|CAC 44545.1\|(AL133159) | bM332P19.2 (novel 7 transmembrane receptor (rhodopsin family) (olfactory receptor like) protein (mm17M1-13); ortholog of human DJ994E9.8 (HS6M1-20)) [Mus musculus] | 308 | 241/308 (78%) | 269/308 (87%) | e-117 |

Table 89D lists the domain descriptions from DOMAIN analysis results against NOV89. This indicates that the NOV89 sequence has properties similar to those of other proteins known to contain this domain.

| **Table 89D. Domain Analysis of NOV89** | |
|---|---|
| gnl\|Pfam\|pfam00001, 7tm_1, 7 transmembrane receptor (rhodopsin family). | |
| | CD-Length = 254 residues, 100.0% aligned |
| | Score = 101 bits (251), Expect = 7e-23 |

The disclosed NOV89 nucleic acid of the invention encoding a GPCR-like protein includes the nucleic acid whose sequence is provided in Table 89A or a fragment thereof. The invention also includes a mutant or variant nucleic acid any of whose bases may be changed from the corresponding base shown in Table 89A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids wh ose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV89 protein of the invention includes the GPCR-like protein whose sequence is provided in Table 89B. The invention also includes a mutant or variant protein any of whose residues may be changed from the corresponding residue shown in Table 89B while still encoding a protein that maintains its GPCR-like activities and physiological functions, or a functional fragment thereof. In the mutant or variant protein, up to about 5 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV89) may function as a member of a "GPCR family". Therefore, the NOV89 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV89 nucleic acids and proteins of the invention are useful in potential therapeutic applications implicated in diseases including but not limited to various pathologies and disorders as indicated below. For example, a cDNA encoding the GPCR-like protein (NOV89) may be useful in gene therapy, and the GPCR-like protein (NOV89) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV89 nucleic acid encoding the GPCR-like protein of the invention, or fragments thereof, may further be useful in diagnostic applications, wherein the presence or amount of the nucleic acid or the protein are to be assessed.

NOV89 nucleic acids and polypeptides are further useful in the generation of antibodies that bind immuno-specifically to the novel NOV89 substances for use in therapeutic or diagnostic methods. These antibodies may be generated according to methods known in the art, using prediction from hydrophobicity charts, as described in the "Anti-NOVX Antibodies" section below. The disclosed NOV89 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV90

A disclosed NOV90 nucleic acid of 950 nucleotides (also referred to as CG57676-01) encoding a GPCR-like protein is shown in Table 90A. The start and stop codons are in bold letters.

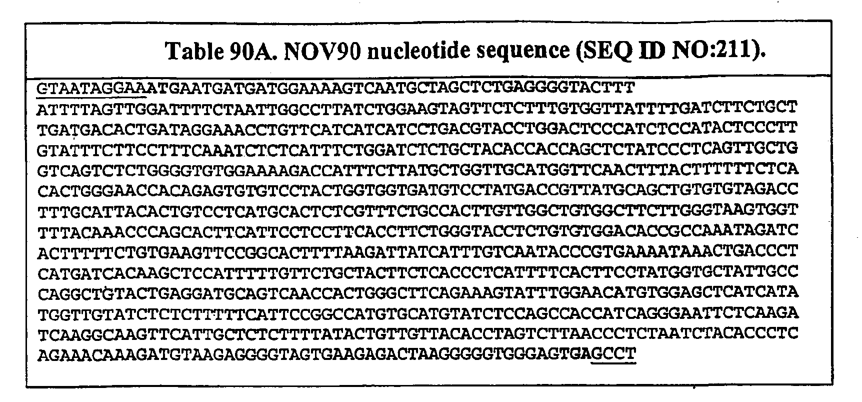

The disclosed NOV90 polypeptide (SEQ ID NO:212) encoded by SEQ ID NO:211 has 311 amino acid residues and is presented in Table 90B using the one-letter amino acid code.

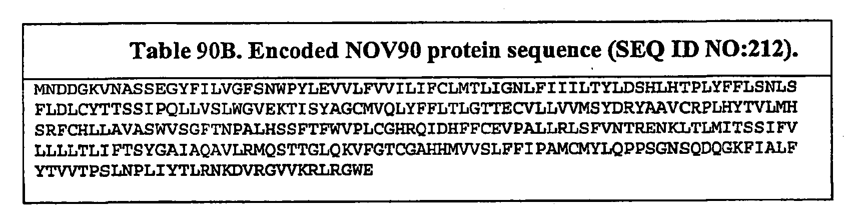

A search of sequence databases reveals that the NOV90 amino acid sequence has similarity with TREMBLNEW-ACC:CAC20478 OLFACTORY RECEPTOR - Homo sapiens. Public amino acid databases include the GenBank databases, SwissProt, PDB and PIR.

The disclosed NOV90 polypeptide has homology to the amino acid sequences shown in the BLASTP data listed in Table 90C.

| **Table 90C. BLAST results for NOV90** | | | | | |
|---|---|---|---|---|---|
| Gene Index/ Identifier | Protein/ Organism | Length (aa) | Identity (%) | Positives (%) | Expect |
| gi\|12054347\|emb\|CAC 20478.1\| (AJ302558) | olfactory receptor [Homo sapiens] | 311 | 281/311 (90%) | 293/311 (93%) | e-139 |
| gi\|112054345\|emb\|CAC 20477.1\| (AJ302557) | olfactory receptor [Homo sapiens] | 311 | 280/311 (90%) | 293/311 (94%) | e-139 |
| gi\|12140469\|emb\|CAC 21440.1\|(AJ302552) | olfactory receptor [Homo sapiens] | 311 | 280/311 (90%) | 292/311 (93%) | e-139 |
| gi\|14423775\|sp\|0760 01\|O2J3 HUMAN | OLFACTORY RECEPTOR 2J3 (OLFACTORY RECEPTOR 6-6) (OR6-6) | 311 | 280/311 (90%) | 293/311 (94%) | e-139 |
| gi\|18564769\|ref\|xp 069457.2 (XM_069457 | similar to OLFACTORY RECEPTOR 2J3 (OLFACTORY RECEPTOR 6-6) (OR6-6) (HS6M1-3) [Homo sapiens] | 391 | 270/298 (90%) | 283/298 (94%) | e-136 |

Table 90D lists the domain descriptions from DOMAIN analysis results against NOV90. This indicates that the NOV90 sequence has properties similar to those of other proteins known to contain this domain.

| **Table 90D. Domain Analysis of NOV90** | |
|---|---|
| gnl\|Pfam\|pfam00001, 7tm_1, 7 transmembrane receptor (rhodopsin family). | |
| | CD-Length = 254 residues, 100.0% aligned |
| | Score = 89.7 bits (221), Expect = 2e-19 |

The disclosed NOV90 nucleic acid of the invention encoding a GPCR-like protein includes the nucleic acid whose sequence is provided in Table 90A or a fragment thereof. The invention also includes a mutant or variant nucleic acid any of whose bases may be changed from the corresponding base shown in Table 90A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV90 protein of the invention includes the GPCR-like protein whose sequence is provided in Table 90B. The invention also includes a mutant or variant protein any of whose residues may be changed from the corresponding residue shown in Table 90B while still encoding a protein that maintains its GPCR-like activities and physiological functions, or a functional fragment thereof. In the mutant or variant protein, up to about 5 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV90) may function as a member of a "GPCR family". Therefore, the NOV90 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV90 nucleic acids and proteins of the invention are useful in potential therapeutic applications implicated in diseases including but not limited to various pathologies and disorders as indicated below. For example, a cDNA encoding the GPCR-like protein (NOV90) may be useful in gene therapy, and the GPCR-like protein (NOV90) may be useful when administered to a subject in need thereof. By way of nonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV nucleic acid encoding the GPCR-like protein of the invention, or fragments thereof, may further be useful in diagnostic applications, wherein the presence or amount of the nucleic acid or the protein are to be assessed.

NOV90 nucleic acids and polypeptides are further useful in the generation of antibodies that bind immuno-specifically to the novel NOV90 substances for use in therapeutic or diagnostic methods. These antibodies may be generated according to methods known in the art, using prediction from hydrophobicity charts, as described in the "Anti-NOVX Antibodies" section below. The disclosed NOV90 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOV91

A disclosed NOV91 nucleic acid of 967 nucleotides (also referred to as CG57668-01) encoding a GPCR-like protein is shown in Table 91A. The start and stop codons are in bold letters.

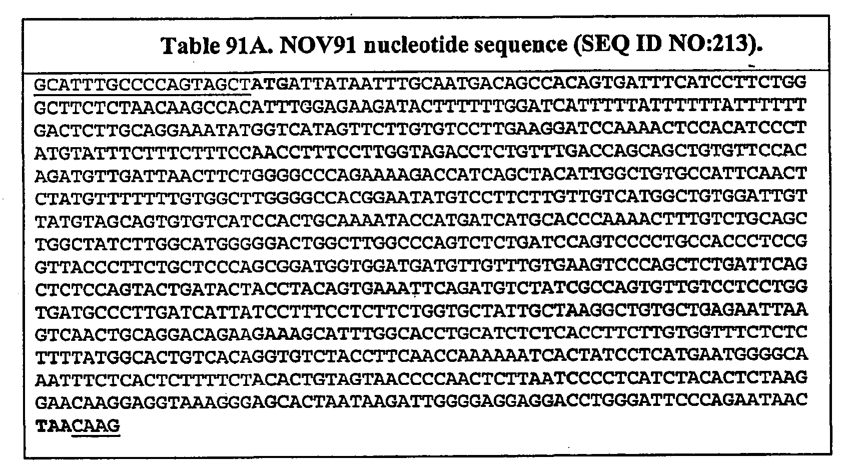

The disclosed NOV91 polypeptide (SEQ ID NO:214) encoded by SEQ ID NO:213 has 314 amino acid residues and is presented in Table 91B using the one-letter amino acid code.

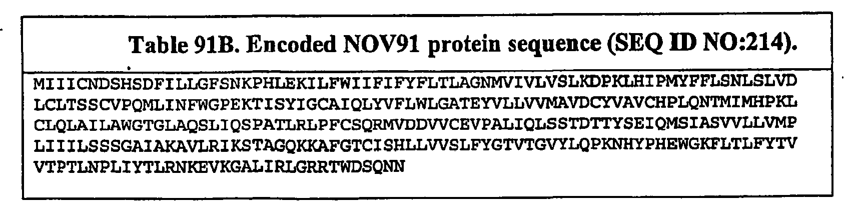

A search of sequence databases reveals that the NOV91 amino acid sequence has 188/303 (62%) identity and 232/303 (76%) similarity with SWISSNEW-ACC:Q15062 OLFACTORY RECEPTOR 2H3 (OLFACTORY RECEPTOR-LIKE FAT11)- Homo sapiens. Public amino acid databases include the GenBank databases, SwissProt, PDB and PIR.

The disclosed NOV91 polypeptide has homology to the amino acid sequences shown in the BLASTP data listed in Table 91C.

| **Table 91C. BLAST results for NOV91** | | | | | |
|---|---|---|---|---|---|
| Gene Index/ Identifier | Protein/ Organism | Length (aa) | Identity (%)' | Positives (%) | Expect |
| gi\|17464345\|ref\|XP069459.1\| (XM_069459) | similar to olfactory receptor [Homo sapiens] | 470 | 217/221 (98%) | 219/221 (98%) | e-111 |
| gi\|18565396\|ref\|XP 094938.1\| (XM_094938 | 216/221 (97%), Positives = 218/221 (97%) | 289 | 216/221 (97%) | 218/221 (97%) | e-111 |
| gi\|112231029\|sp\|Q150 62\|02H3 HUMAN | Olfactory receptor 2H3 (Olfactory receptor-like protein FAT11) | 316 | 188/303 (62%) | 232/303 (76%) | 6e-99 |
| gi\|9798922\|gb\|AAF98 753.1 AF211941 1 (AF211941) | olfactory receptor [Homo sapiens] | 303 | 187/293 (63%) | 228/293 (76%) | 2e-97 |
| gi\|4423783\|sp\|0959 18\|O2H2 HUMAN | Olfactory receptor 2H2 (Hs6M1-12) | 312 | 187/302 (61%) | 230/302 (75%) | 3e-97 |

Table 91D lists the domain descriptions from DOMAIN analysis results against NOV91. This indicates that the NOV91 sequence has properties similar to those of other proteins known to contain this domain.

| **Table 91D. Domain Analysis of NOV91** | |
|---|---|
| gn1\|Pfam\|pfam00001, 7tm_1,7 transmembrane receptor (rhodopsin family). | |
| | CD-Length = 254 residues, 100.0% aligned |
| | Score = 98.6 bits (244), Expect = 5e-22 |

The disclosed NOV91 nucleic acid of the invention encoding a GPCR-like protein includes the nucleic acid whose sequence is provided in Table 91A or a fragment thereof. The invention also includes a mutant or variant nucleic acid any of whose bases may be changed from the corresponding base shown in Table 91A while still encoding a protein that maintains its GPCR-like activities and physiological functions, or a fragment of such a nucleic acid. The invention further includes nucleic acids whose sequences are complementary to those just described, including nucleic acid fragments that are complementary to any of the nucleic acids just described. The invention additionally includes nucleic acids or nucleic acid fragments, or complements thereto, whose structures include chemical modifications. Such modifications include, by way of nonlimiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject. In the mutant or variant nucleic acids, and their complements, up to about 5 percent of the bases may be so changed.

The disclosed NOV91 protein of the invention includes the GPCR-like protein whose sequence is provided in Table 91B. The invention also includes a mutant or variant protein any of whose residues may be changed from the corresponding residue shown in Table 91B while still encoding a protein that maintains its GPCR-like activities and physiological functions, or a functional fragment thereof. In the mutant or variant protein, up to about 38 percent of the residues may be so changed.

The invention further encompasses antibodies and antibody fragments, such as F_{ab} or (F_{ab})₂, that bind immunospecifically to any of the proteins of the invention.

The above defined information for this invention suggests that this GPCR-like protein (NOV91) may function as a member of a "GPCR family". Therefore, the NOV91 nucleic acids and proteins identified here may be useful in potential therapeutic applications implicated in (but not limited to) various pathologies and disorders as indicated below. The potential therapeutic applications for this invention include, but are not limited to: protein therapeutic, small molecule drug target, antibody target (therapeutic, diagnostic, drug targeting/cytotoxic antibody), diagnostic and/or prognostic marker, gene therapy (gene delivery/gene ablation), research tools, tissue regeneration *in vivo* and *in vitro* of all tissues and cell types composing (but not limited to) those defined here.

The NOV91 nucleic acids and proteins of the invention are useful in potential therapeutic applications implicated in diseases including but not limited to various pathologies and disorders as indicated below. For example, a cDNA encoding the GPCR-like protein (NOV91) may be useful in gene therapy, and the GPCR-like protein (NOV91) may be useful when administered to a subject in need thereof. By way ofnonlimiting example, the compositions of the present invention will have efficacy for treatment of patients suffering from CNS disorders, brain disorders including epilepsy, eating disorders, schizophrenia, ADD; cancer; heart disease; inflammation and autoimmune disorders including Crohn's disease, IBD, allergies, rheumatoid and osteoarthritis, inflammatory skin disorders, blood disorders; psoriasis colon cancer, leukemia AIDS; thalamus disorders; metabolic disorders including diabetes and obesity; lung diseases such as asthma, emphysema, cystic fibrosis, pancreatic disorders including pancreatic insufficiency and cancer; and prostate disorders including prostate cancer, or other pathologies or conditions. The NOV nucleic acid encoding the GPCR-like protein of the invention, or fragments thereof, may further be useful in diagnostic applications, wherein the presence or amount of the nucleic acid or the protein are to be assessed.

NOV91 nucleic acids and polypeptides are further useful in the generation of antibodies that bind immuno-specifically to the novel NOV91 substances for use in therapeutic or diagnostic methods. These antibodies may be generated according to methods known in the art, using prediction from hydrophobicity charts, as described in the "Anti-NOVX Antibodies" section below. The disclosed NOV91 proteins have multiple hydrophilic regions, each of which can be used as an immunogen. These novel proteins can be used in assay systems for functional analysis of various human disorders, which will help in understanding of pathology of the disease and development of new drug targets for various disorders.

### NOVX Nucleic Acids and Polypeptides

One aspect of the invention pertains to isolated nucleic acid molecules that encode NOVX polypeptides or biologically active portions thereof. Also included in the invention are nucleic acid fragments sufficient for use as hybridization probes to identify NOVX-encoding nucleic acids (*e.g.,* NOVX mRNAs) and fragments for use as PCR primers for the amplification and/or mutation of NOVX nucleic acid molecules. As used herein, the term "nucleic acid molecule" is intended to include DNA molecules (*e.g.,* cDNA or genomic DNA), RNA molecules (*e.g.,* mRNA), analogs of the DNA or RNA generated using nucleotide analogs, and derivatives, fragments and homologs thereof. The nucleic acid molecule may be single-stranded or double-stranded, but preferably is comprised double-stranded DNA.

An NOVX nucleic acid can encode a mature NOVX polypeptide. As used herein, a "mature" form of a polypeptide or protein disclosed in the present invention is the product of a naturally occurring polypeptide or precursor form or proprotein. The naturally occurring polypeptide, precursor or proprotein includes, by way of nonlimiting example, the full-length gene product, encoded by the corresponding gene. Alternatively, it may be defined as the polypeptide, precursor or proprotein encoded by an ORF described herein. The product "mature" form arises, again by way of nonlimiting example, as a result of one or more naturally occurring processing steps as they may take place within the cell, or host cell, in which the gene product arises. Examples of such processing steps leading to a "mature" form of a polypeptide or protein include the cleavage of the N-terminal methionine residue encoded by the initiation codon of an ORF, or the proteolytic cleavage of a signal peptide or leader sequence. Thus a mature form arising from a precursor polypeptide or protein that has residues 1 to N, where residue 1 is the N-terminal methionine, would have residues 2 through N remaining after removal of the N-terminal methionine. Alternatively, a mature form arising from a precursor polypeptide or protein having residues 1 to N, in which an N-terminal signal sequence from residue 1 to residue M is cleaved, would have the residues from residue M+1 to residue N remaining. Further as used herein, a "mature" form of a polypeptide or-protein may arise from a step of post-translational modification other than a proteolytic cleavage event. Such additional processes include, by way of non-limiting example, glycosylation, myristoylation or phosphorylation. In general, a mature polypeptide or protein may result from the operation of only one of these processes, or a combination of any of them.

The term "probes", as utilized herein, refers to nucleic acid sequences of variable length, preferably between at least about 10 nucleotides (nt), 100 nt, or as many as approximately, e.g., 6,000 nt, depending upon the specific use. Probes are used in the detection of identical, similar, or complementary nucleic acid sequences. Longer length probes are generally obtained from a natural or recombinant source, are highly specific, and much slower to hybridize than shorter-length oligomer probes. Probes may be single- or double-stranded and designed to have specificity in PCR, membrane-based hybridization technologies, or ELISA-like technologies.

The term "isolated" nucleic acid molecule, as utilized herein, is one, which is separated from other nucleic acid molecules which are present in the natural source of the nucleic acid. Preferably, an "isolated" nucleic acid is free of sequences which naturally flank the nucleic acid (*i. e.,* sequences located at the 5'- and 3'-termini of the nucleic acid) in the genomic DNA of the organism from which the nucleic acid is derived. For example, in various embodiments, the isolated NOVX nucleic acid molecules can contain less than about 5 kb, 4 kb, 3 kb, 2 kb, 1 kb, 0.5 kb or 0.1 kb of nucleotide sequences which naturally flank the nucleic acid molecule in genomic DNA of the cell/tissue from which the nucleic acid is derived (*e.g.*, brain, heart, liver, spleen, etc.). Moreover, an "isolated" nucleic acid molecule, such as a cDNA molecule, can be substantially free of other cellular material or culture medium when produced by recombinant techniques, or of chemical precursors or other chemicals when chemically synthesized.

A nucleic acid molecule of the invention, *e.g.,* a nucleic acid molecule having the nucleotide sequence SEQ ID NOS:1, 3, 5, 7, 9, 11, 13, 15, 17, 19, 21, 23, 25, 27, 29, and 31, or a complement of this aforementioned nucleotide sequence, can be isolated using standard molecular biology techniques and the sequence information provided herein. Using all or a portion of the nucleic acid sequence of SEQ ID NOS:1,3,5, 7, 9, 11, 13, 15, 17, 19, 21, 23, 25, 27, 29, and 31 as a hybridization probe, NOVX molecules can be isolated using standard hybridization and cloning techniques (e.g., as described in Sambrook, *et al.,* (eds.), MOLECULAR CLONING: A LABORATORY MANUAL 2^{nd} Ed., Cold Spring Harbor Laboratory Press, Cold Spring Harbor, NY, 1989; and Ausubel, *et al.,* (eds.), CURRENT PROTOCOLS IN MOLECULAR BIOLOGY, John Wiley & Sons, New York, NY, 1993.)

A nucleic acid of the invention can be amplified using cDNA, mRNA or alternatively, genomic DNA, as a template and appropriate oligonucleotide primers according to standard PCR amplification techniques. The nucleic acid so amplified can be cloned into an appropriate vector and characterized by DNA sequence analysis. Furthermore, oligonucleotides corresponding to NOVX nucleotide sequences can be prepared by standard synthetic techniques, *e.g.,* using an automated DNA synthesizer.

As used herein, the term "oligonucleotide" refers to a series of linked nucleotide residues, which oligonucleotide has a sufficient number of nucleotide bases to be used in a PCR reaction. A short oligonucleotide sequence may be based on, or designed from, a genomic or cDNA sequence and is used to amplify, confirm, or reveal the presence of an identical, similar or complementary DNA or RNA in a particular cell or tissue. Oligonucleotides comprise portions of a nucleic acid sequence having about 10 nt, 50 nt, or 100 nt in length, preferably about 15 nt to 30 nt in length. In one embodiment of the invention, an oligonucleotide comprising a nucleic acid molecule less than 100 nt in length would further comprise at least 6 contiguous nucleotides SEQ ID NOS:1, 3, 5, 7, 9, 11,13, 15, 17, 19, 21, 23, 25, 27, 29, and 31, or a complement thereof. Oligonucleotides may be chemically synthesized and may also be used as probes.

In another embodiment, an isolated nucleic acid molecule of the invention comprises a nucleic acid molecule that is a complement of the nucleotide sequence shown in SEQ ID NOS:1, 3, 5, 7, 9,11, 13,15,17, 19, 21, 23, 25, 27, 29, and 31, or a portion of this nucleotide sequence (e.g., a fragment that can be used as a probe or primer or a fragment encoding a biologically-active portion of an NOVX polypeptide). A nucleic acid molecule that is complementary to the nucleotide sequence shown SEQ ID NOS:1, 3, 5, 7, 9, 11, 13, 15, 17, 19, 21, 23, 25, 27, 29, or 31 is one that is sufficiently complementary to the nucleotide sequence shown SEQ ID NOS:1, 3, 5, 7, 9, 11, 13, 15, 17, 19, 21, 23, 25, 27, 29, or 31 that it can hydrogen bond with little or no mismatches to the nucleotide sequence shown SEQ ID NOS:1, 3, 5, 7, 9, 11, 13, 15, 17, 19, 21, 23, 25, 27, 29, and 31, thereby forming a stable duplex.

As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof. Binding includes ionic, non-ionic, van der Waals, hydrophobic interactions, and the like. A physical interaction can be either direct or indirect. Indirect interactions may be through or due to the effects of another polypeptide or compound. Direct binding refers to interactions that do not take place through, or due to, the effect of another polypeptide or compound, but instead are without other substantial chemical intermediates.

Fragments provided herein are defined as sequences of at least 6 (contiguous) nucleic acids or at least 4 (contiguous) amino acids, a length sufficient to allow for specific hybridization in the case of nucleic acids or for specific recognition of an epitope in the case of amino acids, respectively, and are at most some portion less than a full length sequence. Fragments may be derived from any contiguous portion of a nucleic acid or amino acid sequence of choice. Derivatives are nucleic acid sequences or amino acid sequences formed from the native compounds either directly or by modification or partial substitution. Analogs are nucleic acid sequences or amino acid sequences that have a structure similar to, but not identical to, the native compound but differs from it in respect to certain components or side chains. Analogs may be synthetic or from a different evolutionary origin and may have a similar or opposite metabolic activity compared to wild type. Homologs are nucleic acid sequences or amino acid sequences of a particular gene that are'derived from different species.

Derivatives and analogs may be full length or other than full length, if the derivative or analog contains a modified nucleic acid or amino acid, as described below. Derivatives or analogs of the nucleic acids or proteins of the invention include, but are not limited to, molecules comprising regions that are substantially homologous to the nucleic acids or proteins of the invention, in various embodiments, by at least about 70%, 80%, or 95% identity (with a preferred identity of 80-95%) over a nucleic acid or amino acid sequence of identical size or when compared to an aligned sequence in which the alignment is done by a computer homology program known in the art, or whose encoding nucleic acid is capable of hybridizing to the complement of a sequence encoding the aforementioned proteins under stringent, moderately stringent, or low stringent conditions. *See e.g.* Ausubel, *et al.,* CURRENT PROTOCOLS IN MOLECULAR BIOLOGY, John Wiley & Sons, New York, NY, 1993, and below.

A "homologous nucleic acid sequence" or "homologous amino acid sequence," or variations thereof, refer to sequences characterized by a homology at the nucleotide level or amino acid level as discussed above. Homologous nucleotide sequences encode those sequences coding for isoforms of NOVX polypeptides. Isoforms can be expressed in different tissues of the same organism as a result of, for example, alternative splicing ofRNA. Alternatively, isoforms can be encoded by different genes. In the invention, homologous nucleotide sequences include nucleotide sequences encoding for an NOVX polypeptide of species other than humans, including, but not limited to: vertebrates, and thus can include, *e.g*., frog, mouse, rat, rabbit, dog, cat cow, horse, and other organisms. Homologous nucleotide sequences also include, but are not limited to, naturally occurring allelic variations and mutations of the nucleotide sequences set forth herein. A homologous nucleotide sequence does not, however, include the exact nucleotide sequence encoding human NOVX protein. Homologous nucleic acid sequences include those nucleic acid sequences that encode conservative amino acid substitutions (see below) in SEQ ID NOS:1,3, 5,7,9,11,13,15,17, 19, 21, 23, 25, 27, 29, and 31, as well as a polypeptide possessing NOVX biological activity. Various biological activities of the NOVX proteins are described below.

An NOVX polypeptide is encoded by the open reading frame ("ORF') of an NOVX nucleic acid. An ORF corresponds to a nucleotide sequence that could potentially be translated into a polypeptide. A stretch of nucleic acids comprising an ORF is uninterrupted by a stop codon. An ORF that represents the coding sequence for a full protein begins with an ATG "start" codon and terminates with one of the three "stop" codons, namely, TAA, TAG, or TGA. For the purposes of this invention, an ORF may be any part of a coding sequence, with or without a start codon, a stop codon, or both. For an ORF to be considered as a good candidate for coding for a *bona fide* cellular protein, a minimum size requirement is often set, e.g., a stretch ofDNA that would encode a protein of 50 amino acids or more.

The nucleotide sequences determined from the cloning of the human NOVX genes allows for the generation of probes and primers designed for use in identifying and/or cloning NOVX homologues in other cell types, *e.g.* from other tissues, as well as NOVX homologues from other vertebrates. The probe/primer typically comprises substantially purified oligonucleotide. The oligonucleotide typically comprises a region of nucleotide sequence that hybridizes under stringent conditions to at least about 12, 25, 50,100, 150, 200, 250, 300, 350 or 400 consecutive sense strand nucleotide sequence SEQ ID NOS:1, 3, 5, 7, 9, 11, 13, 15, 17, 19, 21, 23, 25, 27, 29, or 31; or an anti-sense strand nucleotide sequence of SEQ ID NOS:1, 3, 5,7, 9,11,13,15,17,19,21,23,25,27,29, or 31; or of a naturally occurring mutant of SEQ ED NOS:1,3, 5,7,9,11,13,15,17,19,21,23,25,27,29, and 31.

Probes based on the human NOVX nucleotide sequences can be used to detect transcripts or genomic sequences encoding the same or homologous proteins. In various embodiments, the probe further comprises a label group attached thereto, *e.g.* the label group can be a radioisotope, a fluorescent compound, an enzyme, or an enzyme co-factor. Such probes can be used as a part of a diagnostic test kit for identifying cells or tissues which mis-express an NOVX protein, such as by measuring a level of an NOVX-encoding nucleic acid in a sample of cells from a subject e.g., detecting NOVX mRNA levels or determining whether a genomic NOVX gene has been mutated or deleted.

"A polypeptide having a biologically-active portion of an NOVX polypeptide" refers to polypeptides exhibiting activity similar, but not necessarily identical to, an activity of a polypeptide of the invention, including mature forms, as measured in a particular biological assay, with or without dose dependency. A nucleic acid fragment encoding a "biologically-active portion of NOVX" can be prepared by isolating a portion SEQ ID NOS:1, 3, 5, 7, 9, 11, 13, 15, 17, 19, 21, 23, 25, 27, 29, or 31, that encodes a polypeptide having an NOVX biological activity (the biological activities of the NOVX proteins are described below), expressing the encoded portion of NOVX protein (e.g., by recombinant expression *in vitro*) and assessing the activity of the encoded portion of NOVX.

### NOVX Nucleic Acid and Polypeptide Variants

The invention further encompasses nucleic acid molecules that differ from the nucleotide sequences shown in SEQ ID NOS:1, 3, 5, 7, 9, 11, 13, 15, 17, 19, 21, 23, 25, 27, 29, and 31 due to degeneracy of the genetic code and thus encode the same NOVX proteins as that encoded by the nucleotide sequences shown in SEQ ID NOS:1, 3, 5, 7, 9,11,13, 15,17, 19, 21, 23, 25, 27, 29, and 31. In another embodiment, an isolated nucleic acid molecule of the invention has a nucleotide sequence encoding a protein having an amino acid sequence shown in SEQ ID NOS:2, 4, 6, 8, 10, 12, 14, 16, 18, 20, 22, 24, 26, 28, 30, or 32.

In addition to the human NOVX nucleotide sequences shown in SEQ ID NOS:1, 3, 5, 7, 9, 11, 13, 15, 17, 19, 21, 23, 25, 27, 29, and 31, it will be appreciated by those skilled in the art that DNA sequence polymorphisms that lead to changes in the amino acid sequences of the NOVX polypeptides may exist within a population (e.g., the human population). Such genetic polymorphism in the NOVX genes may exist among individuals within a population due to natural allelic variation. As used herein, the terms "gene" and "recombinant gene" refer to nucleic acid molecules comprising an open reading frame (ORF) encoding an NOVX protein, preferably a vertebrate NOVX protein. Such natural allelic variations can typically result in 1-5% variance in the nucleotide sequence of the NOVX genes. Any and all such nucleotide variations and resulting amino acid polymorphisms in the NOVX polypeptides, which are the result of natural allelic variation and that do not alter the functional activity of the NOVX polypeptides, are intended to be within the scope of the invention.

Moreover, nucleic acid molecules encoding NOVX proteins from other species, and thus that have a nucleotide sequence that differs from the human SEQ ID NOS:1, 3, 5, 7, 9, 11, 13, 15, 17, 19, 21, 23, 25, 27, 29, and 31 are intended to be within the scope of the invention. Nucleic acid molecules corresponding to natural allelic variants and homologues of the NOVX cDNAs of the invention can be isolated based on their homology to the human NOVX nucleic acids disclosed herein using the human cDNAs, or a portion thereof, as a hybridization probe according to standard hybridization techniques under stringent hybridization conditions.

Accordingly, in another embodiment, an isolated nucleic acid molecule of the invention is at least 6 nucleotides in length and hybridizes under stringent conditions to the nucleic acid molecule comprising the nucleotide sequence of SEQ ID NOS:1, 3, 5, 7, 9, 11, 13, 15, 17, 19, 21, 23, 25, 27, 29, and 31. In another embodiment, the nucleic acid is at least 10, 25, 50,100, 250, 500, 750, 1000, 1500, or 2000 or more nucleotides in length. In yet another embodiment, an isolated nucleic acid molecule of the invention hybridizes to the coding region. As used herein, the term "hybridizes under stringent conditions" is intended to describe conditions for hybridization and washing under which nucleotide sequences at least 60% homologous to each other typically remain hybridized to each other.

Homologs (*i.e.,* nucleic acids encoding NOVX proteins derived from species other than human) or other related sequences (*e.g.,* paralogs) can be obtained by low, moderate or high stringency hybridization with all or a portion of the particular human sequence as a probe using methods well known in the art for nucleic acid hybridization and cloning.

As used herein, the phrase "stringent hybridization conditions" refers to conditions under which a probe, primer or oligonucleotide will hybridize to its target sequence, but to no other sequences. Stringent conditions are sequence-dependent and will be different in different circumstances. Longer sequences hybridize specifically at higher temperatures than shorter sequences. Generally, stringent conditions are selected to be about 5 °C lower than the thermal melting point (Tm) for the specific sequence at a defined ionic strength and pH. The Tm is the temperature (under defined ionic strength, pH and nucleic acid concentration) at which 50% of the probes complementary to the target sequence hybridize to the target sequence at equilibrium. Since the target sequences are generally present at excess, at Tm, 50% of the probes are occupied at equilibrium. Typically, stringent conditions will be those in which the salt concentration is less than about 1.0 M sodium ion, typically about 0.01 to 1.0 M sodium ion (or other salts) at pH 7.0 to 8.3 and the temperature is at least about 30°C for short probes, primers or oligonucleotides (e.g., 10 nt to 50 nt) and at least about 60°C for longer probes, primers and oligonucleotides. Stringent-conditions may also be achieved with the addition of destabilizing agents, such as formamide.

Stringent conditions are known to those skilled in the art and can be found in Ausubel, *et al.*, (eds.), CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6X SSC, 50 mM Tris-HCl (pH 7.5), 1 mM EDTA, 0.02% PVP, 0.02% Ficoll, 0.02% BSA, and 500 mg/ml denatured salmon sperm DNA at 65°C, followed by one or more washes in 0.2X SSC, 0.01% BSA at 50°C. An isolated nucleic acid molecule of the invention that hybridizes under stringent conditions to the sequences SEQ ID NOS:1, 3, 5, 7, 9, 11, 13, 15, 17, 19, 21, 23, 25, 27, 29, and 31, corresponds to a naturally-occurring nucleic acid molecule. As used herein, a "naturally-occurring" nucleic acid molecule refers to an RNA or DNA molecule having a nucleotide sequence that occurs in nature (*e.g.,* encodes a natural protein).

In a second embodiment, a nucleic acid sequence that is hybridizable to the nucleic acid molecule comprising the nucleotide sequence of SEQ ID NOS:1, 3, 5, 7, 9, 11, 13, 15, 17, 19, 21, 23, 25, 27, 29, and 31, or fragments, analogs or derivatives thereof, under conditions of moderate stringency is provided. A non-limiting example of moderate stringency hybridization conditions are hybridization in 6X SSC, 5X Denhardt's solution, 0.5% SDS and 100 mg/ml denatured salmon sperm DNA at 55°C, followed by one or more washes in 1X SSC, 0.1% SDS at 37°C. Other conditions of moderate stringency that may be used are well-known within the art. *See, e.g.,* Ausubel, et al. (eds.), 1993, CURRENT PROTOCOLS IN MOLECULAR BIOLOGY, John Wiley & Sons, NY, and Kriegler, 1990; GENE TRANSFER AND EXPRESSION, A LABORATORY MANUAL, Stockton Press, NY.

In a third embodiment, a nucleic acid that is hybridizable to the nucleic acid molecule comprising the nucleotide sequences SEQ ID NOS:1, 3, 5, 7, 9, 11, 13, 15,17,19, 21, 23, 25, 27, 29, and 31, or fragments, analogs or derivatives thereof, under conditions of low stringency, is provided. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7.4), 5 mM EDTA, and 0.1% SDS at 50°C. Other conditions of low stringency that may be used are well known in the art (*e.g.,* as employed for cross-species hybridizations). *See, e.g.,* Ausubel, *et al.* (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981. *Proc Natl Acad Sci USA **78:*** 6789-6792.

### Conservative Mutations

In addition to naturally-occurring allelic variants of NOVX sequences that may exist in the population, the skilled artisan will further appreciate that changes can be introduced by mutation into the nucleotide sequences SEQ ID NOS:1, 3, 5, 7, 9, 11, 13, 15,17, 19, 21, 23, 25, 27, 29, and 31, thereby leading to changes in the amino acid sequences of the encoded NOVX proteins, without altering the functional ability of said NOVX proteins. For example, nucleotide substitutions leading to amino acid substitutions at "non-essential" amino acid residues can be made in the sequence SEQ ID NOS:2, 4, 6, 8, 10, 12, 14, 16, 18, 20, 22, 24, 26, 28, 30, or 32. A "non-essential" amino acid residue is a residue that can be altered from the wild-type sequences of the NOVX proteins without altering their biological activity, whereas an "essential" amino acid residue is required for such biological activity. For example, amino acid residues that are conserved among the NOVX proteins of the invention are predicted to be particularly non-amenable to alteration. Amino acids for which conservative substitutions can be made are well-known within the art.

Another aspect of the invention pertains to nucleic acid molecules encoding NOVX proteins that contain changes in amino acid residues that are not essential for activity. Such NOVX proteins differ in amino acid sequence from SEQ ID NOS:1, 3, 5, 7, 9, 11, 13, 15, 17, 19, 21, 23, 25, 27, 29, and 31 yet retain biological activity. In one embodiment, the isolated nucleic acid molecule comprises a nucleotide sequence encoding a protein, wherein the protein comprises an amino acid sequence at least about 45% homologous to the amino acid sequences SEQ ID NOS:2, 4, 6, 8, 10, 12, 14, 16, 18, 20, 22, 24, 26, 28, 30, and 32. Preferably, the protein encoded by the nucleic acid molecule is at least about 60% homologous to SEQ ID NOS:2, 4, 6, 8, 10, 12, 14, 16, 18, 20, 22, 24, 26, 28, 30, and 32; more preferably at least about 70% homologous SEQ ID NOS:2, 4, 6, 8, 10, 12, 14, 16, 18, 20, 22, 24, 26, 28, 30, or 32; still more preferably at least about 80% homologous to SEQ ID NOS:2, 4, 6, 8, 10, 12, 14, 16, 18, 20, 22, 24, 26, 28, 30, or 32; even more preferably at least about 90% homologous to SEQ ID NOS:2, 4, 6, 8, 10, 12, 14, 16, 18, 20, 22, 24, 26, 28, 30, or 32; and most preferably at least about 95% homologous to SEQ ID NOS:2, 4, 6, 8, 10, 12, 14, 16, 18, 20, 22, 24, 26, 28, 30, or 32.

An isolated nucleic acid molecule encoding an NOVX protein homologous to the protein of SEQ ID NOS:2, 4, 6, 8, 10, 12, 14, 16, 18, 20, 22, 24, 26, 28, 30, or 32 can be created by introducing one or more nucleotide substitutions, additions or deletions into the nucleotide sequence of SEQ ID NOS:1, 3, 5, 7, 9, 11, 13, 15, 17, 19, 21, 23, 25, 27, 29, and 31, such that one or more amino acid substitutions, additions or deletions are introduced into the encoded protein.

Mutations can be introduced into SEQ ID NOS:1, 3, 5, 7, 9, 11, 13, 15, 17, 19, 21, 23, 25, 27, 29, and 31 by standard techniques, such as site-directed mutagenesis and PCR-mediated mutagenesis. Preferably, conservative amino acid substitutions are made at one or more predicted, non-essential amino acid residues. A "conservative amino acid substitution" is one in which the amino acid residue is replaced with an amino acid residue having a similar side chain. Families of amino acid residues having similar side chains have been defined within the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Thus, a predicted non-essential amino acid residue in the NOVX protein is replaced with another amino acid residue from the same side chain family. Alternatively, in another embodiment, mutations can be introduced randomly along all or part of an NOVX coding sequence, such as by saturation mutagenesis, and the resultant mutants can be screened for NOVX biological activity to identify mutants that retain activity. Following mutagenesis SEQ ID NOS:1, 3, 5, 7, 9, 11, 13, 15, 17, 19, 21, 23, 25, 27, 29, and 31, the encoded protein can be expressed by any recombinant technology known in the art and the activity of the protein can be determined.

The relatedness of amino acid families may also be determined based on side chain interactions. Substituted amino acids may be fully conserved "strong" residues or fully conserved "weak" residues. The "strong" group of conserved amino acid residues may be any one of the following groups: STA, NEQK, NHQK, NDEQ, QHRK, MILV, MILF, HY, FYW, wherein the single letter amino acid codes are grouped by those amino acids that may be substituted for each other. Likewise, the "weak" group of conserved residues may be any one of the following: CSA, ATV, SAG, STNK, STPA, SGND, SNDEQK, NDEQHK, NEQHRK, VLIM, HFY, wherein the letters within each group represent the single letter amino acid code.

In one embodiment, a mutant NOVX protein can be assayed for *(i)* the ability to form protein:protein interactions with other NOVX proteins, other cell-surface proteins, or biologically-active portions thereof, *(ii)* complex formation between a mutant NOVX protein and an NOVX ligand; or *(iii)* the ability of a mutant NOVX protein to bind to an intracellular target protein or biologically-active portion thereof; *(e.g.* avidin proteins).

In yet another embodiment, a mutant NOVX protein can be assayed for the ability to regulate a specific biological function *(e.g.,* regulation of insulin release).

### Antisense Nucleic Acids

Another aspect of the invention pertains to isolated antisense nucleic acid molecules that are hybridizable to or complementary to the nucleic acid molecule comprising the nucleotide sequence of SEQ ID NOS:1, 3, 5, 7, 9, 11, 13, 15, 17, 19, 21, 23, 25, 27, 29, and 31, or fragments, analogs or derivatives thereof. An "antisense" nucleic acid comprises a nucleotide sequence that is complementary to a "sense" nucleic acid encoding a protein (*e.g*., complementary to the coding strand of a double-stranded cDNA molecule or complementary to an mRNA sequence). In specific aspects, antisense nucleic acid molecules are provided that comprise a sequence complementary to at least about 10, 25, 50, 100, 250 or 500 nucleotides or an entire NOVX coding strand, or to only a portion thereof. Nucleic acid molecules encoding fragments, homologs, derivatives and analogs of an NOVX protein of SEQ ID NOS:2, 4, 6, 8, 10, 12, 14, 16, 18, 20, 22, 24, 26, 28, 30, or 32, or antisense nucleic acids complementary to an NOVX nucleic acid sequence of SEQ ID NOS:1, 3, 5, 7, 9, 11,13,15, 17, 19, 21, 23, 25, 27, 29, and 31, are additionally provided.

In one embodiment, an antisense nucleic acid molecule is antisense to a "coding region" of the coding strand of a nucleotide sequence encoding an NOVX protein. The term "coding region" refers to the region of the nucleotide sequence comprising codons which are translated into amino acid residues. In another embodiment, the antisense nucleic acid molecule is antisense to a "noncoding region" of the coding strand of a nucleotide sequence encoding the NOVX protein. The term "noncoding region" refers to 5' and 3' sequences which flank the coding region that are not translated into amino acids (*i.e.,* also referred to as 5' and 3'untranslated regions).

Given the coding strand sequences encoding the NOVX protein disclosed herein, antisense nucleic acids of the invention can be designed according to the rules of Watson and Crick or Hoogsteen base pairing. The antisense nucleic acid molecule can be complementary to the entire coding region of NOVX mRNA, but more preferably is an oligonucleotide that is antisense to only a portion of the coding or noncoding region of NOVX mRNA. For example, the antisense oligonucleotide can be complementary to the region surrounding the translation start site of NOVX mRNA. An antisense oligonucleotide can be, for example, about 5, 10, 15, 20, 25, 30, 35, 40, 45 or 50 nucleotides in length. An antisense nucleic acid of the invention can be constructed using chemical synthesis or enzymatic ligation reactions using procedures known in the art. For example, an antisense nucleic acid (*e.g.*, an antisense oligonucleotide) can be chemically synthesized using naturally-occurring nucleotides or variously modified nucleotides designed to increase the biological stability of the molecules or to increase the physical stability of the duplex formed between the antisense and sense nucleic acids (*e.g.*, phosphorothioate derivatives and acridine substituted nucleotides can be used).

Examples of modified nucleotides that can be used to generate the antisense nucleic acid include: 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject or generated *in situ* such that they hybridize with or bind to cellular mRNA and/or genomic DNA encoding an NOVX protein to thereby inhibit expression of the protein (*e.g.*, by inhibiting transcription and/or translation). The hybridization can be by conventional nucleotide complementarity to form a stable duplex, or, for example, in the case of an antisense nucleic acid molecule that binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface (*e.g.*, by linking the antisense nucleic acid molecules to peptides or antibodies that bind to cell surface receptors or antigens). The antisense nucleic acid molecules can also be delivered to cells using the vectors described herein. To achieve sufficient nucleic acid molecules, vector constructs in which the antisense nucleic acid molecule is placed under the control of a strong pol II or pol III promoter are preferred.

In yet another embodiment, the antisense nucleic acid molecule of the invention is an α-anomeric nucleic acid molecule. An α-anomeric nucleic acid molecule forms specific double-stranded hybrids with complementary RNA in which, contrary to the usual β-units, the strands run parallel to each other. *See, e.g.,* Gaultier, *et al.,* 1987. *Nucl. Acids Res. 15:* 6625-6641. The antisense nucleic acid molecule can also comprise a 2'-o-methylribonucleotide (*See, e.g.*, Inoue, *et al.* 1987. *Nucl. Acids Res.* **15**: 6131-6148) or a chimeric RNA-DNA analogue (*See, e.g.*, Inoue, *et al.*, 1987*. FEBS Lett.* **215**: 327-330.

### Ribozymes and PNA Moieties

Nucleic acid modifications include, by way of non-limiting example, modified bases, and nucleic acids whose sugar phosphate backbones are modified or derivatized. These modifications are carried out at least in part to enhance the chemical stability of the modified nucleic acid, such that they may be used, for example, as antisense binding nucleic acids in therapeutic applications in a subject.

In one embodiment, an antisense nucleic acid of the invention is a ribozyme. Ribozymes are catalytic RNA molecules with ribonuclease activity that are capable of cleaving a single-stranded nucleic acid, such as an mRNA, to which they have a complementary region. Thus, ribozymes (*e.g.*, hammerhead ribozymes as described in Haselhoff and Gerlach 1988. *Nature* 334: 585-591) can be used to catalytically cleave NOVX mRNA transcripts to thereby inhibit translation of NOVX mRNA. A ribozyme having specificity for an NOVX-encoding nucleic acid can be designed based upon the nucleotide sequence of an NOVX cDNA disclosed herein (*i.e.*, SEQ ID NOS:1, 3, 5, 7, 9,11, 13, 15, 17, 19, 21, 23, 25, 27, 29, and 31). For example, a derivative of a *Tetrahymena* L-19 IVS RNA can be constructed in which the nucleotide sequence of the active site is complementary to the nucleotide sequence to be cleaved in an NOVX-encoding mRNA. *See, e.g.,* U.S. Patent 4,987,071 to Cech, *et al.* and U.S. Patent 5,116,742 to Cech, *et al.* NOVX mRNA can also be used to select a catalytic RNA having a specific ribonuclease activity from a pool of RNA molecules. *See, e.g., Bartel et al.,* (1993) *Science* 261:1411-1418.

Alternatively, NOVX gene expression can be inhibited by targeting nucleotide sequences complementary to the regulatory region of the NOVX nucleic acid (e.g., the NOVX promoter and/or enhancers) to form triple helical structures that prevent transcription of the NOVX gene in target cells. *See, e.g.,* Helene, 1991. *Anticancer Drug Des.* 6: 569-84; Helene, *et al.* 1992. *Ann. N. Y. Acad Sci.* 660: 27-36; Maher, 1992. *Bioassays* 14: 807-15.

In various embodiments, the NOVX nucleic acids can be modified at the base moiety, sugar moiety or phosphate backbone to improve, e.g., the stability, hybridization, or solubility of the molecule. For example, the deoxyribose phosphate backbone of the nucleic acids can be modified to generate peptide nucleic acids. *See, e.g.,* Hyrup, *et al.,* 1996. *Bioorg Med Chem* 4: 5-23. As used herein, the terms "peptide nucleic acids" or "PNAs" refer to nucleic acid mimics *(e.g.,* DNA mimics) in which the deoxyribose phosphate backbone is replaced by a pseudopeptide backbone and only the four natural nucleobases are retained. The neutral backbone of PNAs has been shown.to allow for specific hybridization to DNA and RNA under conditions of low ionic strength. The synthesis of PNA oligomers can be performed using standard solid phase peptide synthesis protocols as described in Hyrup, *et al.,* 1996*. supra;* Perry-O'Keefe, *et al.,* 1996. *Proc. Natl. Acad. Sci. USA* 93: 14670-14675.

PNAs of NOVX can be used in therapeutic and diagnostic applications. For example, PNAs can be used as antisense or antigene agents for sequence-specific modulation of gene expression by, *e.g.,* inducing transcription or translation arrest or inhibiting replication. PNAs of NOVX can also be used, for example, in the analysis of single base pair mutations in a gene *(e.g.,* PNA directed PCR clamping; as artificial restriction enzymes when used in combination with other enzymes, *e.g.,* S₁ nucleases *(See, Hyrup, et al., 1996.supra);* or as probes or primers for DNA sequence and hybridization *(See,* Hyrup, *et al.,* 1996, *supra;* Perry-O'Keefe, *et al.,* 1996. *supra).*

In another embodiment, PNAs of NOVX can be modified, e.g., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras of NOVX can be generated that may combine the advantageous properties of PNA and DNA. Such chimeras allow DNA recognition enzymes (e.g., RNase H and DNA polymerases) to interact with the DNA portion while the PNA portion would provide high binding affinity and specificity. PNA-DNA chimeras can be linked using linkers of appropriate lengths selected in terms of base stacking, number of bonds between the nucleobases, and orientation (see, Hyrup, et al., 1996. *supra*)*.* The synthesis of PNA-DNA chimeras can be performed as described in Hyrup, *et al., 1996. supra* and Finn, *et al.,* 1996. *Nucl Acids Res* 24: 3357-3363. For example, a DNA chain can be synthesized on a solid support using standard phosphoramidite coupling chemistry, and modified nucleoside analogs, e.g., 5'-(4-methoxytrityl)amino-5'-deoxy-thymidine phosphoramidite, can be used between the PNA and the 5' end of DNA. *See, e.g.,* Mag, *et al.,* 1989. *Nucl Acid Res* 17: 5973-5988. PNA monomers are then coupled in a stepwise manner to produce a chimeric molecule with a 5' PNA segment and a 3' DNA segment. *See, e.g.,* Finn, *et al.,* 1996. *supra.* Alternatively, chimeric molecules can be synthesized with a 5' DNA segment and a 3' PNA segment. *See, e.g.,* Petersen, *et al.,* 1975. *Bioorg. Med. Chem. Lett. 5:* 1119-11124.

In other embodiments, the oligonucleotide may include other appended groups such as peptides *(e.g.,* for targeting host cell receptors *in vivo),* or agents facilitating transport across the cell membrane *(see, e.g.,* Letsinger, *et al.,* 1989. *Proc. Natl. Acad. Sci. U.S.A.* 86: 6553-6556; Lemaitre, *et al.,* 1987. *Proc. Natl. Acad Sci.* 84: 648-652; PCT Publication No. WO88/09810) or the blood-brain barrier (*see, e.g.,* PCT Publication No. WO 89/10134). In addition, oligonucleotides can be modified with hybridization triggered cleavage agents *(see, e.g.,* Krol, *et al.,* 1988. *BioTechniques* 6:958-976) or intercalating agents (*see, e.g.,* Zon, 1988. *Pharm. Res.* 5: 539-549). To this end, the oligonucleotide may be conjugated to another molecule, *e.g.,* a peptide, a hybridization triggered cross-linking agent, a transport agent, a hybridization-triggered cleavage agent, and the like.

### NOVX Polypeptides

A polypeptide according to the invention includes a polypeptide including the amino acid sequence of NOVX polypeptides whose sequences are provided in SEQ ID NOS:2, 4, 6, 8, 10, 12, 14, 16, 18, 20, 22, 24, 26, 28, 30, or 32. The invention also includes a mutant or variant protein any of whose residues may be changed from the corresponding residues shown in SEQ ID NOS:2, 4, 6, 8,10,12,14,16,18,20,22,24,26,28,30, or 32 while still encoding a protein that maintains its NOVX activities and physiological functions, or a functional fragment thereof.

In general, an NOVX variant that preserves NOVX-like function includes any variant in which residues at a particular position in the sequence have been substituted by other amino acids, and further include the possibility of inserting an additional residue or residues between two residues of the parent protein as well as the possibility of deleting one or more residues from the parent sequence. Any amino acid substitution, insertion, or deletion is encompassed by the invention. In favorable circumstances, the substitution is a conservative substitution as defined above.

One aspect of the invention pertains to isolated NOVX proteins, and biologically-active portions thereof, or derivatives, fragments, analogs or homologs thereof. Also provided are polypeptide fragments suitable for use as immunogens to raise anti-NOVX antibodies. In one embodiment, native NOVX proteins can be isolated from cells or tissue sources by an appropriate purification scheme using standard protein purification techniques. In another embodiment, NOVX proteins are produced by recombinant DNA techniques. Alternative to recombinant expression, an NOVX protein or polypeptide can be synthesized chemically using standard peptide synthesis techniques.

An "isolated" or "purified" polypeptide or protein or biologically-active portion thereof is substantially free of cellular material or other contaminating proteins from the cell or tissue source from which the NOVX protein is derived, or substantially free from chemical precursors or other chemicals when chemically synthesized. The language "substantially free of cellular material" includes preparations of NOVX proteins in which the protein is separated from cellular components of the cells from which it is isolated or recombinantly-produced. In one embodiment, the language "substantially free of cellular material" includes preparations of NOVX proteins having less than about 30% (by dry weight) ofnon-NOVX proteins (also referred to herein as a "contaminating protein"), more preferably less than about 20% of non-NOVX proteins, still more preferably less than about 10% of non-NOVX proteins, and most preferably less than about 5% of non-NOVX proteins. When the NOVX protein or biologically-active portion thereof is recombinantly-produced, it is also preferably substantially free of culture medium, *i.e.,* culture medium represents less than about 20%, more preferably less than about 10%, and most preferably less than about 5% of the volume of the NOVX protein preparation.

The language "substantially free of chemical precursors or other chemicals" includes preparations of NOVX proteins in which the protein is separated from chemical precursors or other chemicals that are involved in the synthesis of the protein. In one embodiment, the language "substantially free of chemical precursors or other chemicals" includes preparations of NOVX proteins having less than about 30% (by dry weight) of chemical precursors or non-NOVX chemicals, more preferably less than about 20% chemical precursors or non-NOVX chemicals, still more preferably less than about 10% chemical precursors or non-NOVX chemicals, and most preferably less than about 5% chemical precursors or non-NOVX chemicals.

Biologically-active portions of NOVX proteins include peptides comprising amino acid sequences sufficiently homologous to or derived from the amino acid sequences of the NOVX proteins (*e*.*g*., the amino acid sequence shown in SEQ ID NOS:2, 4, 6, 8, 10, 12, 14, 16, 18, 20, 22, 24, 26, 28, 30, or 32) that include fewer amino acids than the full-length NOVX proteins, and exhibit at least one activity of an NOVX protein. Typically, biologically-active portions comprise a domain or motif with at least one activity of the NOVX protein. A biologically-active portion of an NOVX protein can be a polypeptide which is, for example, 10, 25, 50, 100 or more amino acid residues in length.

Moreover, other biologically-active portions, in which other regions of the protein are deleted, can be prepared by recombinant techniques and evaluated for one or more of the functional activities of a native NOVX protein.

In an embodiment, the NOVX protein has an amino acid sequence shown SEQ ID NOS:2, 4, 6, 8,10, 12, 14,16, 18, 20, 22, 24, 26, 28, 30, or 32. In other embodiments, the NOVX protein is substantially homologous to SEQ ID NOS:2, 4, 6, 8,10, 12,14,16, 18, 20, 22, 24, 26, 28, 30, or 32, and retains the functional activity of the protein of SEQ ID NOS:2, 4, 6, 8, 10, 12, 14, 16, 18, 20, 22, 24, 26, 28, 30, or 32, yet differs in amino acid sequence due to natural allelic variation or mutagenesis, as described in detail, below. Accordingly, in another embodiment, the NOVX protein is a protein that comprises an amino acid sequence at least about 45% homologous to the amino acid sequence SEQ ID NOS:2, 4, 6, 8, 10, 12, 14, 16, 18, 20, 22, 24, 26, 28, 30, or 32, and retains the functional activity of the NOVX proteins of SEQ ID NOS:2, 4, 6, 8, 10, 12, 14, 16, 18, 20, 22, 24, 26, 28, 30, or 32.

### Determining Homology Between Two or More Sequences

To determine the percent homology of two amino acid sequences or of two nucleic acids, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are homologous at that position (*i.e.,* as used herein amino acid or nucleic acid "homology" is equivalent to amino acid or nucleic acid "identity").

The nucleic acid sequence homology may be determined as the degree of identity between two sequences. The homology may be determined using computer programs known in the art, such as GAP software provided in the GCG program package. *See,* Needleman and Wunsch, 1970. *J Mol Biol* 48: 443-453. Using GCG GAP software with the following settings for nucleic acid sequence comparison: GAP creation penalty of 5.0 and GAP extension penalty of 0.3, the coding region of the analogous nucleic acid sequences referred to above exhibits a degree of identity preferably of at least 70%, 75%, 80%, 85%, 90%, 95%, 98%, or 99%, with the CDS (encoding) part of the DNA sequence shown in SEQ ID NOS:1, 3, 5, 7, 9, 11, 13, 15,17, 19, 21, 23, 25, 27, 29, and 31.

The term "sequence identity" refers to the degree to which two polynucleotide or polypeptide sequences are identical on a residue-by-residue basis over a particular region of comparison. The term "percentage of sequence identity" is calculated by comparing two optimally aligned sequences over that region of comparison, determining the number of positions at which the identical nucleic acid base *(e.g.,* A, T, C, G, U, or I, in the case of nucleic acids) occurs in both sequences to yield the number of matched positions, dividing the number of matched positions by the total number of positions in the region of comparison *(i.e.,* the window size), and multiplying the result by 100 to yield the percentage of sequence identity. The term "substantial identity" as used herein denotes a characteristic of a polynucleotide sequence, wherein the polynucleotide comprises a sequence that has at least 80 percent sequence identity, preferably at least 85 percent identity and often 90 to 95 percent sequence identity, more usually at least 99 percent sequence identity as compared to a reference sequence over a comparison region.

### Chimeric and Fusion Proteins

The invention also provides NOVX chimeric or fusion proteins. As used herein, an NOVX "chimeric protein" or "fusion protein" comprises an NOVX polypeptide operatively-linked to a non-NOVX polypeptide. An "NOVX polypeptide" refers to a polypeptide having an amino acid sequence corresponding to an NOVX protein SEQ ID NOS:2, 4, 6, 8, 10, 12, 14, 16, 18, 20, 22, 24, 26, 28, 30, or 32, whereas a "non-NOVX polypeptide" refers to a polypeptide having an amino acid sequence corresponding to a protein that is not substantially homologous to the NOVX protein, e.g., a protein that is different from the NOVX protein and that is derived from the same or a different organism. Within an NOVX fusion protein the NOVX polypeptide can correspond to all or a portion of an NOVX protein. In one embodiment, an NOVX fusion protein comprises at least one biologically-active portion of an NOVX protein. In another embodiment, an NOVX fusion protein comprises at least two biologically-active portions of an NOVX protein. In yet another embodiment, an NOVX fusion protein comprises at least three biologically-active portions of an NOVX protein. Within the fusion protein, the term "operatively-linked" is intended to indicate that the NOVX polypeptide and the non-NOVX polypeptide are fused in-frame with one another. The non-NOVX polypeptide can be fused to the N-terminus or C-terminus of the NOVX polypeptide.

In one embodiment, the fusion protein is a GST-NOVX fusion protein in which the NOVX sequences are fused to the C-terminus of the GST (glutathione S-transferase) sequences. Such fusion proteins can facilitate the purification of recombinant NOVX polypeptides.

In another embodiment, the fusion protein is an NOVX protein containing a heterologous signal sequence at its N-terminus. In certain host cells (e.g., mammalian host cells), expression and/or secretion of NOVX can be increased through use of a heterologous signal sequence.

In yet another embodiment, the fusion protein is an NOVX-immunoglobulin fusion protein in which the NOVX sequences are fused to sequences derived from a member of the immunoglobulin protein family. The NOVX-immunoglobulin fusion proteins of the invention can be incorporated into pharmaceutical compositions and administered to a subject to inhibit an interaction between an NOVX ligand and an NOVX protein on the surface of a cell, to thereby suppress NOVX-mediated signal transduction *in vivo.* The NOVX-immunoglobulin fusion proteins can be used to affect the bioavailability of an NOVX cognate ligand. Inhibition of the NOVX ligand/NOVX interaction may be useful therapeutically for both the treatment of proliferative and differentiative disorders, as well as modulating (*e.g.* promoting or inhibiting) cell survival. Moreover, the NOVX-immunoglobulin fusion proteins of the invention can be used as immunogens to produce anti-NOVX antibodies in a subject, to purify NOVX ligands, and in screening assays to identify molecules that inhibit the interaction of NOVX with an NOVX ligand.

An NOVX chimeric or fusion protein of the invention can be produced by standard recombinant DNA techniques. For example, DNA fragments coding for the different polypeptide sequences are ligated together in-frame in accordance with conventional techniques, e.g., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of gene fragments can be carried out using anchor primers that give rise to complementary overhangs between two consecutive gene fragments that can subsequently be annealed and reamplified to generate a chimeric gene sequence *(see, e.g.,* Ausubel, *et al.* (eds.) CURRENT PROTOCOLS IN MOLECULAR BIOLOGY, John Wiley & Sons, 1992). Moreover, many expression vectors are commercially available that already encode a fusion moiety (e.g.; a GST polypeptide). An NOVX-encoding nucleic acid can be cloned into such an expression vector such that the fusion moiety is linked in-frame to the NOVX protein.

### NOVX Agonists and Antagonists

The invention also pertains to variants of the NOVX proteins that function as either NOVX agonists *(i.e.,* mimetics) or as NOVX antagonists. Variants of the NOVX protein can be generated by mutagenesis (e.g., discrete point mutation or truncation of the NOVX protein). An agonist of the NOVX protein can retain substantially the same, or a subset of, the biological activities of the naturally occurring form of the NOVX protein. An antagonist of the NOVX protein can inhibit one or more of the activities of the naturally occurring form of the NOVX protein by, for example, competitively binding to a downstream or upstream member of a cellular signaling cascade which includes the NOVX protein. Thus, specific biological effects can be elicited by treatment with a variant of limited function. In one embodiment, treatment of a subject with a variant having a subset of the biological activities of the naturally occurring form of the protein has fewer side effects in a subject relative to treatment with the naturally occurring form of the NOVX proteins.

Variants of the NOVX proteins that function as either NOVX agonists (*i*.*e*., mimetics) or as NOVX antagonists can be identified by screening combinatorial libraries of mutants (e.g., truncation mutants) of the NOVX proteins for NOVX protein agonist or antagonist activity. In one embodiment, a variegated library ofNOVX variants is generated by combinatorial mutagenesis at the nucleic acid level and is encoded by a variegated gene library. A variegated library of NOVX variants can be produced by, for example, enzymatically ligating a mixture of synthetic oligonucleotides into gene sequences such that a degenerate set of potential NOVX sequences is expressible as individual polypeptides, or alternatively, as a set of larger fusion proteins (*e.g*., for phage display) containing the set of NOVX sequences therein. There are a variety of methods which can be used to produce libraries of potential NOVX variants from a degenerate oligonucleotide sequence. Chemical synthesis of a degenerate gene sequence can be performed in an automatic DNA synthesizer, and the synthetic gene then ligated into an appropriate expression vector. Use of a degenerate set of genes allows for the provision, in one mixture, of all of the sequences encoding the desired set of potential NOVX sequences. Methods for synthesizing degenerate oligonucleotides are well-known within the art. *See, e.g.,* Narang, 1983. *Tetrahedron* 39: 3; Itakura, *et al.,* 1984. *Annu. Rev. Biochem.* 53: 323; Itakura, *et al.,* 1984. *Science* 198: 1056; Ike, *et al.*,1983. *Nucl. Acids Res*. 11: 477.

### Polypeptide Libraries

In addition, libraries of fragments of the NOVX protein coding sequences can be used to generate a variegated population of NOVX fragments for screening and subsequent selection of variants of an NOVX protein. In one embodiment, a library of coding sequence fragments can be generated by treating a double stranded PCR fragment of an NOVX coding sequence with a nuclease under conditions wherein nicking occurs only about once per molecule, denaturing the double stranded DNA, renaturing the DNA to form double-stranded DNA that can include sense/antisense pairs from different nicked products, removing single stranded portions from reformed duplexes by treatment with S₁ nuclease, and ligating the resulting fragment library into an expression vector. By this method, expression libraries can be derived which encodes N-terminal and internal fragments of various sizes of the NOVX proteins.

Various techniques are known in the art for screening gene products of combinatorial libraries made by point mutations or truncation, and for screening cDNA libraries for gene products having a selected property. Such techniques are adaptable for rapid screening of the gene libraries generated by the combinatorial mutagenesis of NOVX proteins. The most widely used techniques, which are amenable to high throughput analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected. Recursive ensemble mutagenesis (REM), a new technique that enhances the frequency of functional mutants in the libraries, can be used in combination with the screening assays to identify NOVX variants. *See, e.g.,* Arkin and Yourvan, 1992. *Proc. Natl. Acad. Sci. USA* 89: 7811-7815; Delgrave, *et al.,* 1993. *Protein Engineering* 6:327-331.

### Anti-NOVX Antibodies

Also included in the invention are antibodies to NOVX proteins, or fragments of NOVX proteins. The term "antibody" as used herein refers to immunoglobulin molecules and immunologically active portions of immunoglobulin (Ig) molecules, i.e., molecules that contain an antigen binding site that specifically binds (immunoreacts with) an antigen. Such antibodies include, but are not limited to, polyclonal, monoclonal, chimeric, single chain, F_{ab}, F_{ab}, and F_{(ab')2} fragments, and an F_{ab} expression library. In general, an antibody molecule obtained from humans relates to any of the classes IgG, IgM, IgA, IgE and IgD, which differ from one another by the nature of the heavy chain present in the molecule. Certain classes have subclasses as well, such as IgG₁, IgG₂, and others. Furthermore, in humans, the light chain may be a kappa chain or a lambda chain. Reference herein to antibodies includes a reference to all such classes, subclasses and types of human antibody species.

An isolated NOVX-related protein of the invention may be intended to serve as an antigen, or a portion or fragment thereof, and additionally can be used as an immunogen to generate antibodies that immunospecifically bind the antigen, using standard techniques for polyclonal and monoclonal antibody preparation. The full-length protein can be used or, alternatively, the invention provides antigenic peptide fragments of the antigen for use as immunogens. An antigenic peptide fragment comprises at least 6 amino acid residues of the amino acid sequence of the full length protein and encompasses an epitope thereof such that an antibody raised against the peptide forms a specific immune complex with the full length protein or with any fragment that contains the epitope. Preferably, the antigenic peptide comprises at least 10 amino acid residues, or at least 15 amino acid residues, or at least 20 amino acid residues, or at least 30 amino acid residues. Preferred epitopes encompassed by the antigenic peptide are regions of the protein that are located on its surface; commonly these are hydrophilic regions.

In certain embodiments of the invention, at least one epitope encompassed by the antigenic peptide is a region of NOVX-related protein that is located on the surface of the protein, *e.g.,* a hydrophilic region. A hydrophobicity analysis of the human NOVX-related protein sequence will indicate which regions of a NOVX-related protein are particularly hydrophilic and, therefore, are likely to encode surface residues useful for targeting antibody production. As a means for targeting antibody production, hydropathy plots showing regions of hydrophilicity and hydrophobicity may be generated by any method well known in the art, including, for example, the Kyte Doolittle or the Hopp Woods methods, either with or without Fourier transformation. See, *e.g*., Hopp and Woods, 1981, *Proc. Nat. Acad. Sci. USA 78:* 3824-3828; Kyte and Doolittle 1982, *J. Mol. Biol.* 157: 105-142, each of which is incorporated herein by reference in its entirety. Antibodies that are specific for one or more domains within an antigenic protein, or derivatives, fragments, analogs or homologs thereof, are also provided herein.

A protein of the invention, or a derivative, fragment, analog, homolog or ortholog thereof, may be utilized as an immunogen in the generation of antibodies that immunospecifically bind these protein components.

Various procedures known within the art may be used for the production of polyclonal or monoclonal antibodies directed against a protein of the invention, or against derivatives, fragments, analogs homologs or orthologs thereof (see, for example, Antibodies: A Laboratory Manual, Harlow and Lane, 1988, Cold Spring Harbor Laboratory Press, Cold Spring Harbor, NY, incorporated herein by reference). Some of these antibodies are discussed below.

### Polyclonal Antibodies

For the production of polyclonal antibodies, various suitable host animals (e.g., rabbit, goat, mouse or other mammal) may be immunized by one or more injections with the native protein, a synthetic variant thereof, or a derivative of the foregoing. An appropriate immunogenic preparation can contain, for example, the naturally occurring immunogenic protein, a chemically synthesized polypeptide representing the immunogenic protein, or a recombinantly expressed immunogenic protein. Furthermore, the protein may be conjugated to a second protein known to be immunogenic in the mammal being immunized. Examples of such immunogenic proteins include but are not limited to keyhole limpet hemocyanin, serum albumin, bovine thyroglobulin, and soybean trypsin inhibitor. The preparation can further ' include an adjuvant. Various adjuvants used to increase the immunological response include, but are not limited to, Freund's (complete and incomplete), mineral gels (e.g., aluminum hydroxide), surface active substances (e.g., lysolecithin, pluronic polyols, polyanions, peptides, oil emulsions, dinitrophenol, etc.), adjuvants usable in humans such as Bacille Calmette-Guerin and Corynebacterium parvum, or similar immunostimulatory agents. Additional examples of adjuvants which can be employed include MPL-TDM adjuvant (monophosphoryl Lipid A, synthetic trehalose dicorynomycolate).

The polyclonal antibody molecules directed against the immunogenic protein can be isolated from the mammal (e.g., from the blood) and further purified by well known techniques, such as affinity chromatography using protein A or protein G, which provide primarily the IgG fraction of immune serum. Subsequently, or alternatively, the specific antigen which is the target of the immunoglobulin sought, or an epitope thereof, may be immobilized on a column to purify the immune specific antibody by immunoaffinity chromatography. Purification of immunoglobulins is discussed, for example, by D. Wilkinson (The Scientist, published by The Scientist, Inc., Philadelphia PA, Vol. 14, No. 8 (April 17, 2000), pp. 25-28).

### Monoclonal Antibodies

The term "monoclonal antibody" (MAb) or "monoclonal antibody composition", as used herein, refers to a population of antibody molecules that contain only one molecular species of antibody molecule consisting of a unique light chain gene product and a unique heavy chain gene product. In particular, the complementarity determining regions (CDRs) of the monoclonal antibody are identical in all the molecules of the population. MAbs thus contain an antigen binding site capable of immunoreacting with a particular epitope of the antigen characterized by a unique binding affinity for it.

Monoclonal antibodies can be prepared using hybridoma methods, such as those described by Kohler and Milstein, *Nature,* 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes can be immunized in vitro.

The immunizing agent will typically include the protein antigen, a fragment thereof or a fusion protein thereof. Generally, either peripheral blood lymphocytes are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, MONOCLONAL ANTIBODIES: PRINCIPLES AND PRACTICE, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells can be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies (Kozbor, *J. Immunol.,* 133:3001 (1984); Brodeur et al., MONOCLONAL ANTIBODY PRODUCTION TECHNIQUES AND APPLICATIONS, Marcel Dekker, Inc., New York, (1987) pp. 51-63).

The culture medium in which the hybridoma cells are cultured can then be assayed for the presence of monoclonal antibodies directed against the antigen. Preferably, the binding specificity of monoclonal antibodies produced by the hybridoma cells is determined by immunoprecipitation or by an in vitro binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, *Anal. Biochem.,* 107:220 (1980). Preferably, antibodies having a high degree of specificity and a high binding affinity for the target antigen are isolated.

After the desired hybridoma cells are identified, the clones can be subcloned by limiting dilution procedures and grown by standard methods. Suitable culture media for this purpose include, for example, Dulbecco's Modified Eagle's Medium and RPMI-1640 medium. Alternatively, the hybridoma cells can be grown in vivo as ascites in a mammal.

The monoclonal antibodies secreted by the subclones can be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies can also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA can be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also can be modified, for example, by substituting the coding sequence for human heavy and light chain constant domains in place of the homologous murine sequences (U.S. Patent No. 4,816,567; Morrison, *Nature* 368, 812-13 (1994)) or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

### Humanized Antibodies

The antibodies directed against the protein antigens of the invention can further comprise humanized antibodies or human antibodies. These antibodies are suitable for administration to humans without engendering an immune response by the human against the administered immunoglobulin. Humanized forms of antibodies are chimeric immunoglobulins, immunoglobulin chains or fragments thereof (such as Fv, Fab, Fab', F(ab')₂ or other antigen-binding subsequences of antibodies) that are principally comprised of the sequence of a human immunoglobulin, and contain minimal sequence derived from a non-human immunoglobulin. Humanization can be performed following the method of Winter and co-workers (Jones et al., *Nature,* 321:522-525 (1986); Riechmann et al., *Nature,* 332:323-327 (1988); Verhoeyen et al., *Science,* 239:1534-1536 (1988)), by substituting rodent CDRs or CDR sequences for the corresponding sequences of a human antibody. (See also U.S. Patent No. 5,225,539.) In some instances, Fv framework residues of the human immunoglobulin are replaced by corresponding non-human residues. Humanized antibodies can also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework regions are those of a human immunoglobulin consensus sequence. The humanized antibody optimally also will comprise at least a portion of an immunoglobulin constant region (Fc), typically that of a human immunoglobulin (Jones et al., 1986; Riechmann et al., 1988; and Presta, *Curr. Op. Struct. Biol.,* 2:593-596 (1992)).

### Human Antibodies

Fully human antibodies relate to antibody molecules in which essentially the entire sequences of both the light chain and the heavy chain, including the CDRs, arise from human genes. Such antibodies are termed "human antibodies", or "fully human antibodies" herein. Human monoclonal antibodies can be prepared by the trioma technique; the human B-cell hybridoma technique (see Kozbor, et al., 1983 Immunol Today 4: 72) and the EBV hybridoma technique to produce human monoclonal antibodies (see Cole, et al., 1985 In: MONOCLONAL ANTIBODIES AND CANCER THERAPY, Alan R. Liss, Inc., pp. 77-96). Human monoclonal antibodies may be utilized in the practice of the present invention and may be produced by using human hybridomas (see Cote, et al., 1983. Proc Natl Acad Sci USA 80: 2026-2030) or by transforming human B-cells with Epstein Barr Virus in vitro (see Cole, et al., 1985 In: MONOCLONAL ANTIBODIES AND CANCER THERAPY, Alan R. Liss, Inc., pp. 77-96).

In addition, human antibodies can also be produced using additional techniques, including phage display libraries (Hoogenboom and Winter, *J. Mol. Biol.,* 227:381 (1991); Marks et al., *J. Mol. Biol.,* 222:581 (1991)). Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in Marks et al. (*Bio*/*Technology* 10, 779-783 (1992)); Lonberg et al. (*Nature* 368 856-859 (1994)); Morrison (*Nature* 368, 812-13 (1994)); Fishwild et al,(*Nature Biotechnology* 14, 845-51 (1996)); Neuberger (*Nature Biotechnology* 14, 826 (1996)); and Lonberg and Huszar (*Intent Rev. Immunol.* 13 65-93 (1995)).

Human antibodies may additionally be produced using transgenic nonhuman animals which are modified so as to produce fully human antibodies rather than the animal's endogenous antibodies in response to challenge by an antigen. (See PCT publication WO94/02602). The endogenous genes encoding the heavy and light immunoglobulin chains in the nonhuman host have been incapacitated, and active loci encoding human heavy and light chain immunoglobulins are inserted into the host's genome. The human genes are incorporated, for example, using yeast artificial chromosomes containing the requisite human DNA segments. An animal which provides all the desired modifications is then obtained as progeny by crossbreeding intermediate transgenic animals containing fewer than the full complement of the modifications. The preferred embodiment of such a nonhuman animal is a mouse, and is termed the Xenomouse™ as disclosed in PCT publications WO 96/33735 and WO 96/34096. This animal produces B cells which secrete fully human immunoglobulins. The antibodies can be obtained directly from the animal after immunization with an immunogen of interest, as, for example, a preparation of a polyclonal antibody, or alternatively from immortalized B cells derived from the animal, such as hybridomas producing monoclonal antibodies. Additionally, the genes encoding the immunoglobulins with human variable regions can be recovered and expressed to obtain the antibodies directly, or can be further modified to obtain analogs of antibodies such as, for example, single chain Fv molecules.

An example of a method of producing a nonhuman host, exemplified as a mouse, lacking expression of an endogenous immunoglobulin heavy chain is disclosed in U.S. Patent No. 5,939,598. It can be obtained by a method including deleting the J segment genes from at least one endogenous heavy chain locus in an embryonic stem cell to prevent rearrangement of the locus and to prevent formation of a transcript of a rearranged immunoglobulin heavy chain locus, the deletion being effected by a targeting vector containing a gene encoding a selectable, marker; and producing from the embryonic stem cell a transgenic mouse whose somatic and germ cells contain the gene encoding the selectable marker.

A method for producing an antibody of interest, such as a human antibody, is disclosed in U.S. Patent No. 5,916,771. It includes introducing an expression vector that contains a nucleotide sequence encoding a heavy chain into one mammalian host cell in culture, introducing an expression vector containing a nucleotide sequence encoding a light chain into another mammalian host cell, and fusing the two cells to form a hybrid cell. The hybrid cell expresses an antibody containing the heavy chain and the light chain.

In a further improvement on this procedure, a method for identifying a clinically relevant epitope on an immunogen, and a correlative method for selecting an antibody that binds immunospecifically to the relevant epitope with high affinity, are disclosed in PCT publication WO 99/53049.

### F_{ab} Fragments and Single Chain Antibodies

According to the invention, techniques can be adapted for the production of single-chain antibodies specific to an antigenic protein of the invention (see e.g., U.S. Patent No. 4,946,778). In addition, methods can be adapted for the construction of F_{ab} expression libraries (see e.g., Huse, et al., 1989 Science 246: 1275-1281) to allow rapid and effective identification of monoclonal F_{ab} fragments with the desired specificity for a protein or derivatives, fragments, analogs or homologs thereof. Antibody fragments that contain the idiotypes to a protein antigen may be produced by techniques known in the art including, but not limited to: (i) an F_{(ab')2} fragment produced by pepsin digestion of an antibody molecule; (ii) an F_{ab} fragment generated by reducing the disulfide bridges of an F_{(ab')2} fragment; (iii) an F_{ab} fragment generated by the treatment of the antibody molecule with papain and a reducing agent and (iv) Fᵥ fragments.

### Bispecific Antibodies

Bispecific antibodies are monoclonal, preferably human or humanized, antibodies that have binding specificities for at least two different antigens. In the present case, one of the binding specificities is for an antigenic protein of the invention. The second binding target is any other antigen, and advantageously is a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, *Nature,* 305:537-539 (1983)). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker *et al.,* 1991 *EMBO J.,* 10:3655-3659.

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., *Methods in Enzymology,* 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared using chemical linkage. Brennan et al., *Science* 229:81 (1985) describe a procedure wherein intact antibodies are proteolytically cleaved to generate F(ab')₂ fragments. These fragments are reduced in the presence of the dithiol com26S protease regulatory subunit 4g agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Additionally, Fab' fragments can be directly recovered from E. coli and chemically coupled to form bispecific antibodies. Shalaby et al., *J. Exp. Med.* 175:217-225 (1992) describe the production of a fully humanized bispecific antibody F(ab')₂ molecule. Each Fab' fragment was separately secreted from E. coli and subjected to directed chemical coupling in vitro to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., *J. Immunol.* 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., *Proc. Natl. Acad. Sci. USA* 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., *J. Immunol.* 152:5368 (1994).

Antibodies with more than two valencies are contemplated. For example, trispecific antibodies can be prepared. Tutt et al., *J*. *Immunol.* 147:60 (1991).

Exemplary bispecific antibodies can bind to two different epitopes, at least one of which originates in the protein antigen of the invention. Alternatively, an anti-antigenic arm of an immunoglobulin molecule can be combined with an arm which binds to a triggering molecule on a leukocyte such as a T-cell receptor molecule (e.g. CD2, CD3, CD28, or B7), or Fc receptors for IgG (FcγR), such as FcγRI (CD64), FcγRII (CD32) and FcγRIII (CD16) so as to focus cellular defense mechanisms to the cell expressing the particular antigen. Bispecific antibodies can also be used to direct cytotoxic agents to cells which express a particular antigen. These antibodies possess an antigen-binding arm and an arm which binds a cytotoxic agent or a radionuclide chelator, such as EOTUBE, DPTA, DOTA, or TETA. Another bispecific antibody of interest binds the protein antigen described herein and further binds tissue factor (TF).

### Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells (U.S. Patent No. 4,676,980), and for treatment of HIV infection (WO 91/00360; WO 92/200373; EP 03089). It is contemplated that the antibodies can be prepared in vitro using known methods in synthetic protein chemistry, including those involving crosslinking agents. For example, immunotoxins can be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### Effector Function Engineering

It can be desirable to modify the antibody of the invention with respect to effector function, so as to enhance, e.g., the effectiveness of the antibody in treating cancer. For example, cysteine residue(s) can be introduced into the Fc region, thereby allowing interchain disulfide bond formation in this region. The homodimeric antibody thus generated can have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity can also be prepared using heterobifunctional cross-linkers as described in Wolff et al. Cancer Research, 53: 2560-2565 (1993). Alternatively, an antibody can be engineered that has dual Fc regions and can thereby have enhanced complement lysis and ADCC capabilities. See Stevenson et al., Anti-Cancer Drug Design, 3: 219-230 (1989).

### Immunoconjugates

The invention also pertains to immunoconjugates comprising an antibody conjugated to a cytotoxic agent such as a chemotherapeutic agent, toxin (e.g., an enzymatically active toxin of bacterial, fungal, plant, or animal origin, or fragments thereof), or a radioactive isotope (i.e., a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi, ¹³¹I, ^{13t}In, ⁹⁰Y, and ¹⁸⁶Re.

Conjugates of the antibody and cytotoxic agent are made using a variety of bifunctional protein-coupling agents such as N-succinimidyl-3-(2-pyridyldithiol), propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody can be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (e.g., avidin) that is in turn conjugated to a cytotoxic agent.

In one embodiment, methods for the screening of antibodies that possess the desired specificity include, but are not limited to, enzyme-linked immunosorbent assay (ELISA) and other immunologically-mediated techniques known within the art. In a specific embodiment, selection of antibodies that are specific to a particular domain of an NOVX protein is facilitated by generation of hybridomas that bind to the fragment of an NOVX protein possessing such a domain. Thus, antibodies that are specific for a desired domain within an NOVX protein, or derivatives, fragments, analogs or homologs thereof, are also provided herein.

Anti-NOVX antibodies may be used in methods known within the art relating to the localization and/or quantitation of an NOVX protein (*e.g.,* for use in measuring levels of the NOVX protein within appropriate physiological samples, for use in diagnostic methods, for use in imaging the protein, and the like). In a given embodiment, antibodies for NOVX proteins, or derivatives, fragments, analogs or homologs thereof, that contain the antibody derived binding domain, are utilized as pharmacologically-active compounds (hereinafter "Therapeutics").

An anti-NOVX antibody (e.g., monoclonal antibody) can be used to isolate an NOVX polypeptide by standard techniques, such as affinity chromatography or immunoprecipitation. An anti-NOVX antibody can facilitate the purification of natural NOVX polypeptide from cells and of recombinantly-produced NOVX polypeptide expressed in host cells. Moreover, an anti-NOVX antibody can be used to detect NOVX protein (e.g., in a cellular lysate or cell supernatant) in order to evaluate the abundance and pattern of expression of the NOVX protein. Anti-NOVX antibodies can be used diagnostically to monitor protein levels in tissue as part of a clinical testing procedure, *e.g.,* to, for example, determine the efficacy of a given *treatment* regimen. Detection can be facilitated by coupling *(i.e.,* physically linking) the antibody to a detectable substance. Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin, and examples of suitable radioactive material include ¹²⁵I, ¹³¹I, ³⁵S or ³H.

### NOVX Recombinant Expression Vectors and Host Cells

Another aspect of the invention pertains to vectors, preferably expression vectors, containing a nucleic acid encoding an NOVX protein, or derivatives, fragments, analogs or homologs thereof. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g.*, bacterial vectors having a bacterial origin of replication and episomal mammalian vectors). Other vectors (*e.g*., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid'' and "vector" can be used interchangeably as the plasmid is the most commonly used form of vector. However, the invention is intended to include such other forms of expression vectors, such as viral vectors *(e.g.,* replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence *(e.g.,* in *an in vitro* transcription/translation system or in a host cell when the vector is introduced into the host cell).

The term "regulatory sequence" is intended to includes promoters, enhancers and other expression control elements (e.g., polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein *(e.g.,* NOVX proteins, mutant forms of NOVX proteins, fusion proteins, etc.).

The recombinant expression vectors of the invention can be designed for expression of NOVX proteins in prokaryotic or eukaryotic cells. For example, NOVX proteins can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro,* for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. *Gene* 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann *et al.,* (1988) *Gene* 69:301-315) and pET 11d (Studier *et al.,* GENE EXPRESSION TECHNOLOGY: METHODS IN ENZYMOLOGY 185, Academic Press, San Diego, Calif. (1990) 60-89).

One strategy to maximize recombinant protein expression in *E. coli* is to express the protein in a host bacteria with an impaired capacity to proteolytically cleave the recombinant protein. *See, e.g.,* Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E. coli (see, e.g.,* Wada, *et al.,* 1992. *Nucl. Acids Res.* 20: 2111-2118). Such alteration of nucleic acid sequences of the invention can be carried out by standard DNA synthesis techniques.

In another embodiment, the NOVX expression vector is a yeast expression vector. Examples of vectors for expression in yeast *Saccharomyces cerivisae* include pYepSec1 (Baldari, *et al.,* 1987. *EMBO J.* 6: 229-234), pMFa (Kurjan and Herskowitz, 1982. *Cell* 30: 933-943), pJRY88 (Schultz *et al.,* 1987. *Gene* 54: 113-123), pYES2 (Invitrogen Corporation, San Diego, Calif.), and picZ (InVitrogen Corp, San Diego, Calif.).

Alternatively, NOVX can be expressed in insect cells using baculovirus expression vectors. Baculovirus vectors available for expression of proteins in cultured insect cells *(e.g.,* SF9 cells) include the pAc series (Smith, *et al*., 1983. *Mol. Cell. Biol.* 3: 2156-2165) and the pVL series (Lucklow and Summers, 1989. *Virology* 170: 31-39).

In yet another embodiment, a nucleic acid of the invention is expressed in mammalian cells using a mammalian expression vector. Examples of mammalian expression vectors include pCDM8 (Seed, 1987. *Nature* 329: 840) and pMT2PC (Kaufman, *et al.,* 1987. *EMBO* J. 6: 187-195): When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells see, *e.g.,* Chapters 16 and 17 of Sambrook, *et al.,* MOLECULAR CLONING: A LABORATORY MANUAL. 2nd ed., Cold Spring Harbor Laboratory, Cold Spring Harbor Laboratory Press, Cold Spring Harbor, N.Y., 1989.

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert, *et al.,* 1987. *Genes Dev. 1:* 268-277), lymphoid-specific promoters (Calame and Eaton, 1988. *Adv. Immunol.* 43: 235-275), in particular promoters of T cell receptors (Winoto and Baltimore, 1989. *EMBO J.* 8: 729-733) and immunoglobulins (Banerji, *et al.,* 1983. *Cell* 33: 729-740; Queen and Baltimore, 1983. *Cell* 33: 741-748), neuron-specific promoters (e.g., the neurofilament promoter; Byrne and Ruddle, 1989. *Proc. Natl. Acad. Sci. USA* 86: 5473-5477), pancreas-specific promoters (Edlund, *et al.,* 1985. *Science* 230: 912-916), and mammary gland-specific promoters (*e.g*., milk whey promoter, U.S. Pat. No. 4,873,316 and European Application Publication No. 264,166). Developmentally-regulated promoters are also encompassed, e.g., the murine hox promoters (Kessel and Gruss, 1990. *Science* 249: 374-379) and the α-fetoprotein promoter (Campes and Tilghman, 1989. *Genes Dev.* 3: 537-546).

The invention further provides a recombinant expression vector comprising a DNA molecule of the invention cloned into the expression vector in an antisense orientation. That is, the DNA molecule is operatively-linked to a regulatory sequence in a manner that allows for expression (by transcription of the DNA molecule) of an RNA molecule that is antisense to NOVX mRNA. Regulatory sequences operatively linked to a nucleic acid cloned in the antisense orientation can be chosen that direct the continuous expression of the antisense RNA molecule in a variety of cell types, for instance viral promoters and/or enhancers, or regulatory sequences can be chosen that direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activity of which can be determined by the cell type into which the vector is introduced. For a discussion of the regulation of gene expression using antisense genes *see, e.g.,* Weintraub, *et al.,* "Antisense RNA as a molecular tool for genetic analysis," *Reviews-Trends in Genetics,* Vol. 1(1) 1986.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, NOVX protein can be expressed in bacterial cells such as *E. coli,* insect cells, yeast or mammalian cells (such as Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid *(e.g.,* DNA) into a host cell, including calcium phosphate or calcium chloride co-precipitation, DEAE-dextran-mediated transfection, lipofection, or electroporation. Suitable methods for transforming or transfecting host cells can be found in Sambrook, *et al.* (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker *(e.g.,* resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding NOVX or can be introduced on a separate vector. Cells stably transfected with the introduced nucleic acid can be identified by drug selection (e.g., cells that have incorporated the selectable marker gene will survive, while the other cells die).

A host cell of the invention, such as a prokaryotic or eukaryotic host cell in culture, can be used to produce (*i.e.,* express) NOVX protein. Accordingly, the invention further provides methods for producing NOVX protein using the host cells of the invention. In one embodiment, the method comprises culturing the host cell of invention (into which a recombinant expression vector encoding NOVX protein has been introduced) in a suitable medium such that NOVX protein is produced. In another embodiment, the method further comprises isolating NOVX protein from the medium or the host cell.

### Transgenic NOVX Animals

The host cells of the invention can also be used to produce non-human transgenic animals. For example, in one embodiment, a host cell of the invention is a fertilized oocyte or an embryonic stem cell into which NOVX protein-coding sequences have been introduced. Such host cells can then be used to create non-human transgenic animals in which exogenous NOVX sequences have been introduced into their genome or homologous recombinant - animals in which endogenous NOVX sequences have been altered. Such animals are useful for studying the function and/or activity of NOVX protein and for identifying and/or evaluating modulators of NOVX protein activity. As used herein, a "transgenic animal" is a non-human animal, preferably a mammal, more preferably a rodent such as a rat or mouse, in which one or more of the cells of the animal includes a transgene. Other examples of transgenic animals include non-human primates, sheep, dogs, cows, goats, chickens, amphibians, etc. A transgene is exogenous DNA that is integrated into the genome of a cell from which a transgenic animal develops and that remains in the genome of the mature animal, thereby directing the expression of an encoded gene product in one or more cell types or tissues of the transgenic animal. As used herein, a "homologous recombinant animal" is a non-human animal, preferably a mammal, more preferably a mouse, in which an endogenous NOVX gene has been altered by homologous recombination between the endogenous gene and an exogenous DNA molecule introduced into a cell of the animal, *e.g.*, an embryonic cell of the animal, prior to development of the animal.

A transgenic animal of the invention can be created by introducing NOVX-encoding nucleic acid into the male pronuclei of a fertilized oocyte (e.g., by microinjection, retroviral infection) and allowing the oocyte to develop in a pseudopregnant female foster animal. The human NOVX cDNA sequences SEQ ID NOS:1, 3, 5, 7, 9, 11, 13, 15, 17, 19, 21, 23, 25, 27, 29, and 31 can be introduced as a transgene into the genome of a non-human animal. Alternatively, a non-human homologue of the human NOVX gene, such as a mouse NOVX gene, can be isolated based on hybridization to the human NOVX cDNA (described further *supra*) and used as a transgene. Intronic sequences and polyadenylation signals can also be included in the transgene to increase the efficiency of expression of the transgene. A tissue-specific regulatory sequence(s) can be operably-linked to the NOVX transgene to direct expression of NOVX protein to particular cells. Methods for generating transgenic animals via embryo manipulation and microinjection, particularly animals such as mice, have become conventional in the art and are described, for example, in U.S. Patent Nos. 4,736,866; 4,870,009; and 4,873,191; and Hogan,1986. In: MANIPULATING THE MOUSE EMBRYO, Cold Spring Harbor Laboratory Press, Cold Spring Harbor, N.Y. Similar methods are used for production of other transgenic animals. A transgenic founder animal can be identified based upon the presence of the NOVX trans gene in its genome and/or expression of NOVX mRNA in tissues or cells of the animals. A transgenic founder animal can then be used to breed additional animals carrying the transgene. Moreover, transgenic animals carrying a transgene-encoding NOVX protein can further be bred to other transgenic animals carrying other transgenes.

To create a homologous recombinant animal, a vector is prepared which contains at least a portion of an NOVX gene into which a deletion, addition or substitution has been introduced to thereby alter, e.g., functionally disrupt, the NOVX gene. The NOVX gene can be a human gene (*e.g.,* the cDNA of SBQ ID NOS:1,3,5,7,9,11,13,15,17,19,21,23,25, 27, 29, and 31), but more preferably, is a non-human homologue of a human NOVX gene. For example, a mouse homologue of human NOVX gene of SEQ ID NOS:1, 3, 5, 7, 9,11,13, 15, 17, 19, 21, 23, 25, 27, 29, and 31 can be used to construct a homologous recombination vector suitable for altering an endogenous NOVX gene in the mouse genome. In one embodiment, the vector is designed such that, upon homologous recombination, the endogenous NOVX gene is functionally disrupted (*i.e*., no longer encodes a functional protein; also referred to as a "knock out" vector).

Alternatively, the vector can be designed such that, upon homologous recombination, the endogenous NOVX gene is mutated or otherwise altered but still encodes functional protein (e.g., the upstream regulatory region can be altered to thereby alter the expression of the endogenous NOVX protein). In the homologous recombination vector, the altered portion of the NOVX gene is flanked at its 5'- and 3'-termini by additional nucleic acid of the NOVX gene to allow for homologous recombination to occur between the exogenous NOVX gene carried by the vector and an endogenous NOVX gene in an embryonic stem cell. The additional flanking NOVX nucleic acid is of sufficient length for successful homologous recombination with the endogenous gene. Typically, several kilobases of flanking DNA (both at the 5'- and 3'-termini) are included in the vector. *See, e.g., Thomas, et al.,* 1987. *Cell* 51: 503 for a description of homologous recombination vectors. The vector is ten introduced into an embryonic stem cell line (e.g., by electroporation) and cells in which the introduced NOVX gene has homologously-recombined with the endogenous NOVX gene are selected. *See, e.g.,* Li, *et al.,* 1992. *Cell* 69: 915.

The selected cells are then injected into a blastocyst of an animal (e.g., a mouse) to form aggregation chimeras. *See, e.g.,* Bradley, 1987. In: TERATOCARCINOMAS AND EMBRYONIC STEM CELLS: A PRACTICAL APPROACH, Robertson, ed. IRL, Oxford, pp. 113-152. A chimeric embryo can then be implanted into a suitable pseudopregnant female foster animal and the embryo brought to term. Progeny harboring the homologously-recombined DNA in their germ cells can be used to breed animals in which all cells of the animal contain the homologously-recombined DNA by germline transmission of the transgene. Methods for constructing homologous recombination vectors and homologous recombinant animals are described further in Bradley, 1991. *Curr. Opin. Biotechnol.* 2: 823-829; PCT International Publication Nos.: WO 90/11354; WO 91/01140; WO 92/0968; and WO 93/04169.

In another embodiment, transgenic non-humans animals can be produced that contain selected systems that allow for regulated expression of the transgene. One example of such a system is the cre/loxP recombinase system of bacteriophage P1. For a description of the cre/loxP recombinase system, *See, e.g., Lakso, et al.,* 1992. *Proc. Natl. Acad. Sci. USA* 89: 6232-6236. Another example of a recombinase system is the FLP recombinase system of *Saccharomyces cerevisiae. See,* O'Gorman, *et al.,* 1991. *Science* 251:1351-1355. If a cre/loxP recombinase system is used to regulate expression of the transgene, animals containing transgenes encoding both the Cre recombinase and a selected protein are required. Such animals can be provided through the construction of "double" transgenic animals, *e.g.,* by mating two transgenic animals, one containing a transgene encoding a selected protein and the other containing a transgene encoding a recombinase.

Clones of the non-human transgenic animals described herein can also be produced according to the methods described in Wilmut, *et al.,1997. Nature* 385: 810-813. In brief, a cell *(e.g.,* a somatic cell) from the transgenic animal can be isolated and induced to exit the growth cycle and enter G₀ phase. The quiescent cell can then be fused, e.g., through the use of electrical pulses, to an enucleated oocyte from an animal of the same species from which the quiescent cell is isolated. The reconstructed oocyte is then cultured such that it develops to morula or blastocyte and then transferred to pseudopregnant female foster animal. The offspring borne of this female foster animal will be a clone of the animal from which the cell (e.g., the somatic cell) is isolated.

### Pharmaceutical Compositions

The NOVX nucleic acid molecules, NOVX proteins, and anti-NOVX antibodies (also referred to herein as "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

A pharmaceutical composition of the invention is formulated to be compatible with its intended route of administration. Examples of routes of administration include parenteral, *e.g*., intravenous, intradermal, subcutaneous, oral (*e.g.,* inhalation), transdermal (*i.e*., topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, N:J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e.g.,* an NOVX protein or anti-NOVX antibody) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g.,* a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories *(e.g.*, with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration *(see, e.g.,* U.S. Patent No. 5,328,470) or by stereotactic injection *(see, e.g., Chen, et al.,* 1994. *Proc. Natl. Acad. Sci. USA* 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.,* retroviral vectors, the pharmaceutical preparation can include one or more cells that produce the gene delivery system.

The pharmaceutical compositions can be included in a container, pack, or dispenser together with instructions for administration.

### Screening and Detection Methods

The isolated nucleic acid molecules of the invention can be used to express NOVX protein *(e.g.,* via a recombinant expression vector in a host cell in gene therapy applications), to detect NOVX mRNA (*e.g.*, in a biological sample) or a genetic lesion in an NOVX gene, and to modulate NOVX activity, as described further, below. In addition, the NOVX proteins can be used to screen drugs or compounds that modulate the NOVX protein activity or expression as well as to treat disorders characterized by insufficient or excessive production of NOVX protein or production of NOVX protein forms that have decreased or aberrant activity compared to NOVX wild-type protein (e.g.; diabetes (regulates insulin release); obesity (binds and transport lipids); metabolic disturbances associated with obesity, the metabolic syndrome X as well as anorexia and wasting disorders associated with chronic diseases and various cancers, and infectious disease(possesses anti-microbial activity) and the various dyslipidemias. In addition, the anti-NOVX antibodies of the invention can be used to detect and isolate NOVX proteins and modulate NOVX activity. In yet a further aspect, the invention can be used in methods to influence appetite, absorption of nutrients and the disposition of metabolic substrates in both a positive and negative fashion.

The invention further pertains to novel agents identified by the screening assays described herein and uses thereof for treatments as described, *supra.*

### Screening Assays -

The invention provides a method (also referred to herein as a "screening assay") for identifying modulators, i.e., candidate or test compounds or agents (e.g., peptides, peptidomimetics, small molecules or other drugs) that bind to NOVX proteins or have a stimulatory or inhibitory effect on, *e.g.,* NOVX protein expression or NOVX protein activity. The invention also includes compounds identified in the screening assays described herein.

In one embodiment, the invention provides assays for screening candidate or test compounds which bind to or modulate the activity of the membrane-bound form of an NOVX protein or polypeptide or biologically-active portion thereof. The test compounds of the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds. *See, e.g.,* Lam, 1997. *Anticancer Drug Design* 12: 145.

A "small molecule" as used herein, is meant to refer to a composition that has a molecular weight of less than about 5 kD and most preferably less than about 4 kD. Small molecules can be, e.g., nucleic acids, peptides, polypeptides, peptidomimetics, carbohydrates, lipids or other organic or inorganic molecules. Libraries of chemical and/or biological mixtures, such as fungal, bacterial, or algal extracts, are known in the art and can be screened with any of the assays of the invention.

Examples of methods for the synthesis of molecular libraries can be found in the art, for example in: DeWitt, et *al.,1993.* Proc. Natl. *Acad* Sci. *U.S.A.* 90: 6909; Erb, et *al.,* 1994. Proc. *Natl. Acad. Sci. U.S.A.* 91: 11422; Zuckermann, et *al.,* 1994. *J. Med. Chem.* 37: 2678; Cho, et al.,1993. Science 261: 1303; Carrell, et al., 1994. Angew. Chem. Int. Ed. Engl. 33: 2059; Carell, et al., 1994. Angew. Chem. Int. Ed. Engl. 33: 2061; and Gallop, et al.,1994. J. Med. *Chem.* 37: 1233.

Libraries of compounds may be presented in solution (e.g., Houghten, 1992. *Biotechniques* 13: 412-421), or on beads (Lam, 1991. *Nature* 354: 82-84), on chips (Fodor, 1993. *Nature* 364: 555-556), bacteria (Ladner, U.S. Patent No. 5,223,409), spores (Ladner, U.S. Patent 5,233,409), plasmids *(Cull, et al.,* 1992. *Proc. Natl. Acad. Sci. USA* 89: 1865-1869) or on phage (Scott and Smith, 1990. *Science* 249: 386-390; Devlin, 1990. *Science* 249: 404-406; *Cwirla, et al.,* 1990. *Proc. Natl. Acad. Sci. U.S.A.* 87: 6378-6382; Felici, 1991. *J. Mol. Biol.* 222: 301-310; Ladner, U.S. Patent No. 5,233,409.).

In one embodiment, an assay is a cell-based assay in which a cell which expresses a membrane-bound form of NOVX protein, or a biologically-active portion thereof, on the cell surface is contacted with a test compound and the ability of the test compound to bind to an NOVX protein determined. The cell, for example, can of mammalian origin or a yeast cell. Determining the ability of the test compound to bind to the NOVX protein can be accomplished, for example, by coupling the test compound with a radioisotope or enzymatic label such that binding of the test compound to the NOVX protein or biologically-active portion thereof can be determined by detecting the labeled compound in a complex. For example, test compounds can be labeled with ¹²⁵I, ³⁵S, ¹⁴C, or ³H, either directly or indirectly, and the radioisotope detected by direct counting of radioemission or by scintillation counting. Alternatively, test compounds can be enzymatically-labeled with, for example, horseradish peroxidase, alkaline phosphatase, or luciferase, and the enzymatic label detected by determination of conversion of an appropriate substrate to product. In one embodiment, the assay comprises contacting a cell which expresses a membrane-bound form of NOVX protein, or a biologically-active portion thereof, on the cell surface with a known compound which binds NOVX to form an assay mixture, contacting the assay mixture with a test compound, and determining the ability of the test compound to interact with an NOVX protein, wherein determining the ability of the test compound to interact with an NOVX protein comprises determining the ability of the test compound to preferentially bind to NOVX protein or a biologically-active portion thereof as compared to the known compound.

In another embodiment, an assay is a cell-based assay comprising contacting a cell expressing a membrane-bound form of NOVX protein, or a biologically-active portion thereof, on the cell surface with a test compound and determining the ability of the test compound to modulate (e.g., stimulate or inhibit) the activity of the NOVX protein or biologically-active portion thereof. Determining the ability of the test compound to modulate the activity of NOVX or a biologically-active portion thereof can be accomplished, for example, by determining the ability of the NOVX protein to bind to or interact with an NOVX target molecule. As used herein, a "target molecule" is a molecule with which an NOVX protein binds or interacts in nature, for example, a molecule on the surface of a cell which expresses an NOVX interacting protein, a molecule on the surface of a second cell, a molecule in the extracellular milieu, a molecule associated with the internal surface of a cell membrane or a cytoplasmic molecule. An NOVX target molecule can be a non-NOVX molecule or an NOVX protein or polypeptide of the invention. In one embodiment, an NOVX target molecule is a component of a signal transduction pathway that facilitates transduction of an extracellular signal (*e.g.* a signal generated by binding of a compound to a membrane-bound NOVX molecule) through the cell membrane and into the cell. The target, for example, can be a second intercellular protein that has catalytic activity or a protein that facilitates the association of downstream signaling molecules with NOVX.

Determining the ability of the NOVX protein to bind to or interact with an NOVX target molecule can be accomplished by one of the methods described above for determining direct binding. In one embodiment, determining the ability of the NOVX protein to bind to or interact with an NOVX target molecule can be accomplished by determining the activity of the target molecule. For example, the activity of the target molecule can be determined by detecting induction of a cellular second messenger of the target (*i.e.* intracellular Ca²⁺, diacylglycerol, IP₃, etc.), detecting catalytic/enzymatic activity of the target an appropriate substrate, detecting the induction of a reporter gene (comprising an NOVX-responsive regulatory element operatively linked to a nucleic acid encoding a detectable marker, e.g., luciferase), or detecting a cellular response, for example, cell survival, cellular differentiation, or cell proliferation.

In yet another embodiment, an assay of the invention is a cell-free assay comprising contacting an NOVX protein or biologically-active portion thereof with a test compound and determining the ability of the test compound to bind to the NOVX protein or biologically active portion thereof. Binding of the test compound to the NOVX protein can be determined either directly or indirectly as described above. In one such embodiment, the assay comprises contacting the NOVX protein or biologically-active portion thereof with a known compound which binds NOVX to form an assay mixture, contacting the assay mixture with a test compound, and determining the ability of the test compound to interact with an NOVX protein, wherein determining the ability of the test compound to interact with an NOVX protein comprises determining the ability of the test compound to preferentially bind to NOVX or biologically-active portion thereof as compared to the known compound.

In still another embodiment, an assay is a cell-free assay comprising contacting NOVX protein or biologically-active portion thereof with a test compound and determining the ability of the test compound to modulate (e.g. stimulate or inhibit) the activity of the NOVX protein or biologically-active portion thereof. Determining the ability of the test compound to modulate the activity of NOVX can be accomplished, for example, by determining the ability of the NOVX protein to bind to an NOVX target molecule by one of the methods described above for determining direct binding. In an alternative embodiment, determining the ability of the test compound to modulate the activity of NOVX protein can be accomplished by determining the ability of the NOVX protein further modulate an NOVX target molecule. For example, the catalytic/enzymatic activity of the target molecule on an appropriate substrate can be determined as described, *supra.*

In yet another embodiment, the cell-free assay comprises contacting the NOVX protein or biologically-active portion thereof with a known compound which binds NOVX protein to form an assay mixture, contacting the assay mixture with a test compound, and determining the ability of the test compound to interact with an NOVX protein, wherein determining the ability of the test compound to interact with an NOVX protein comprises determining the ability of the NOVX protein to preferentially bind to or modulate the activity of an NOVX target molecule.

The cell-free assays of the invention are amenable to use of both the soluble form or the membrane-bound form of NOVX protein. In the case of cell-free assays comprising the membrane-bound form of NOVX protein, it may be desirable to utilize a solubilizing agent such that the membrane-bound form of NOVX protein is maintained in solution. Examples of such solubilizing agents include non-ionic detergents such as n-octylglucoside, n-dodecylglucoside, n-dodecylmaltoside, octanoyl-N-methylglucamide, decanoyl-N-methylglucamide, Triton® X-100, Triton® X-114, Thesit® , Isotridecypoly(ethylene glycol ether)ₙ, N-dodecyl-N,N-dimethyl-3-ammonio-1-propane sulfonate, 3-(3-cholamidopropyl) dimethylamminiol-1-propane sulfonate (CHAPS), or 3-(3-cholamidopropyl)dimethylamminiol-2-hydroxy-1-propane sulfonate (CHAPSO).

In more than one embodiment of the above assay methods of the invention, it may be desirable to immobilize either NOVX protein or its target molecule to facilitate separation of complexed from uncomplexed forms of one or both of the proteins, as well as to accommodate automation of the assay. Binding of a test compound to NOVX protein, or interaction of NOVX protein with a target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided that adds a domain that allows one or both of the proteins to be bound to a matrix. For example, GST-NOVX fusion proteins or GST-target fusion proteins can be adsorbed onto glutathione sepharose beads (Sigma Chemical, St. Louis, MO) or glutathione derivatized microtiter plates, that are then combined with the test compound or the test compound and either the non-adsorbed target protein or NOVX protein, and the mixture is incubated under conditions conducive to complex formation (e.g., at physiological conditions for salt and pH). Following incubation, the beads or microtiter plate wells are washed to remove any unbound components, the matrix immobilized in the case of beads, complex determined either directly or indirectly, for example, as described, *supra.* Alternatively, the complexes can be dissociated from the matrix, and the level of NOVX protein binding or activity determined using standard techniques.

Other techniques for immobilizing proteins on matrices can also be used in the screening assays of the invention. For example, either the NOVX protein or its target molecule can be immobilized utilizing conjugation of biotin and streptavidin. Biotinylated NOVX protein or target molecules can be prepared from biotin-NHS (N-hydroxy-succinimide) using techniques well-known within the art (e.g., biotinylation kit, Pierce Chemicals, Rockford, Ill.), and immobilized in the wells of streptavidin-coated 96 well plates (Pierce Chemical). Alternatively, antibodies reactive with NOVX protein or target molecules, but which do not interfere with binding of the NOVX protein to its target molecule, can be derivatized to the wells of the plate, and unbound target or NOVX protein trapped in the wells by antibody conjugation. Methods for detecting such complexes, in addition to those described above for the GST-immobilized complexes, include immunodetection of complexes using antibodies reactive with the NOVX protein or target molecule, as well as enzyme-linked assays that rely on detecting an enzymatic activity associated with the NOVX protein or target molecule.

In another embodiment, modulators of NOVX protein expression are identified in a method wherein a cell is contacted with a candidate compound and the expression of NOVX mRNA or protein in the cell is determined. The level of expression of NOVX mRNA or protein in the presence of the candidate compound is compared to the level of expression of NOVX mRNA or protein in the absence of the candidate compound. The candidate compound can then be identified as a modulator of NOVX mRNA or protein expression based upon this comparison. For example, when expression of NOVX mRNA or protein is greater (i.e., statistically significantly greater) in the presence of the candidate compound than in its absence, the candidate compound is identified as a stimulator of NOVX mRNA or protein expression. Alternatively, when expression of NOVX mRNA or protein is less (statistically significantly less) in the presence of the candidate compound than in its absence, the candidate compound is identified as an inhibitor of NOVX mRNA or protein expression. The level of NOVX mRNA or protein expression in the cells can be determined by methods described herein for detecting NOVX mRNA or protein.

In yet another aspect of the invention, the NOVX proteins can be used as "bait proteins" in a two-hybrid assay or three hybrid assay *(see, e.g.,* U.S. Patent No. 5,283,317; Zervos, *et al.,* 1993. Cell 72: 223-232; Madura, *et al.,* 1993. *J. BioL Chem.* 268: 12046-12054; Bartel, *et al.,* 1993. *Biotechniques* 14: 920-924; Iwabuchi, *et al.,* 1993. *Oncogene* 8: 1693-1696; and Brent WO 94/10300), to identify other proteins that bind to or interact with NOVX ("NOVX-binding proteins" or "NOVX-bp") and modulate NOVX activity. Such NOVX-binding proteins are also likely to be involved in the propagation of signals by the NOVX proteins as, for example, upstream or downstream elements of the NOVX pathway.

The two-hybrid system is based on the modular nature of most transcription factors, which consist of separable DNA-binding and activation domains. Briefly, the assay utilizes two different DNA constructs. In one construct, the gene that codes for NOVX is fused to a gene encoding the DNA binding domain of a known transcription factor (*e.g*., GAL-4). In the other construct, a DNA sequence, from a library of DNA sequences, that encodes an unidentified protein ("prey" or "sample") is fused to a gene that codes for the activation domain of the known transcription factor. If the "bait" and the "prey" proteins are able to interact, *in vivo,* forming an NOVX-dependent complex, the DNA-binding and activation domains of the transcription factor are brought into close proximity. This proximity allows transcription of a reporter gene (*e.g*., LacZ) that is operably linked to a transcriptional regulatory site responsive to the transcription factor. Expression of the reporter gene can be . detected and cell colonies containing the functional transcription factor can be isolated and used to obtain the cloned gene that encodes the protein which interacts with NOVX.

The invention further pertains to novel agents identified by the aforementioned screening assays and uses thereof for treatments as described herein.

### Detection Assays

Portions or fragments of the cDNA sequences identified herein (and the corresponding complete gene sequences) can be used in numerous ways as polynucleotide reagents. By way of example, and not of limitation, these sequences can be used to: (*i*) map their respective genes on a chromosome; and, thus, locate gene regions associated with genetic disease; (ii) identify an individual from a minute biological sample (tissue typing); and (*iii*) aid in forensic identification of a biological sample. Some of these applications are described in the subsections, below.

### Chromosome Mapping

Once the sequence (or a portion of the sequence) of a gene has been isolated, this sequence can be used to map the location of the gene on a chromosome. This process is called chromosome mapping. Accordingly, portions or fragments of the NOVX sequences, SEQ ID NOS: 1, 3, 5, 7, 9, 11, 13, 15, 17, 19, 21, 23, 25, 27, 29, and 31, or fragments or derivatives thereof, can be used to map the location of the NOVX genes, respectively, on a chromosome. The mapping of the NOVX sequences to chromosomes is an important first step in correlating these sequences with genes associated with disease.

Briefly, NOVX genes can be mapped to chromosomes by preparing PCR primers (preferably 15-25 bp in length) from the NOVX sequences. Computer analysis of the NOVX, sequences can be used to rapidly select primers that do not span more than one exon in the genomic DNA, thus complicating the amplification process. These primers can then be used for PCR screening of somatic cell hybrids containing individual human chromosomes. Only those hybrids containing the human gene corresponding to the NOVX sequences will yield an amplified fragment.

Somatic cell hybrids are prepared by fusing somatic cells from different mammals (e.g., human and mouse cells). As hybrids of human and mouse cells grow and divide, they gradually lose human chromosomes in random order, but retain the mouse chromosomes. By using media in which mouse cells cannot grow, because they lack a particular enzyme, but in which human cells can, the one human chromosome that contains the gene encoding the needed enzyme will be retained. By using various media, panels of hybrid cell lines can be established. Each cell line in a panel contains either a single human chromosome or a small number of human chromosomes, and a full set of mouse chromosomes, allowing easy mapping of individual genes to specific human chromosomes. *See, e.g.*, D'Eustachio, *et al.,* 1983. *Science* 220: 919-924. Somatic cell hybrids containing only fragments of human chromosomes can also be produced by using human chromosomes with translocations and deletions.

PCR mapping of somatic cell hybrids is a rapid procedure for assigning a particular sequence to a particular chromosome. Three or more sequences can be assigned per day using a single thermal cycler. Using the NOVX sequences to design oligonucleotide primers, sub-localization can be achieved with panels of fragments from specific chromosomes.

Fluorescence *in situ* hybridization (FISH) of a DNA sequence to a metaphase chromosomal spread can further be used to provide a precise chromosomal location in one step. Chromosome spreads can be made using cells whose division has been blocked in metaphase by a chemical like colcemid that disrupts the mitotic spindle. The chromosomes can be treated briefly with trypsin, and then stained with Giemsa. A pattern of light and dark bands develops on each chromosome, so that the chromosomes can be identified individually. The FISH technique can be used with a DNA sequence as short as 500 or 600 bases. However, clones larger than 1,000 bases have a higher likelihood of binding to a unique chromosomal location with sufficient signal intensity for simple detection. Preferably 1,000 bases, and more preferably 2,000 bases, will suffice to get good results at a reasonable amount of time. For a review of this technique, *see,* Verma, *et al.,* HUMAN CHROMOSOMES: A MANUAL OF BASIC TECHNIQUES (Pergamon Press, New York 1988).

Reagents for chromosome mapping can be used individually to mark a single chromosome or a single site on that chromosome, or panels of reagents can be used for marking multiple sites and/or multiple chromosomes. Reagents corresponding to noncoding regions of the genes actually are preferred for mapping purposes. Coding sequences are more likely to be conserved within gene families, thus increasing the chance of cross hybridizations during chromosomal mapping.

Once a sequence has been mapped to a precise chromosomal location, the physical position of the sequence on the chromosome can be correlated with genetic map data. Such data are found, e.g., in McKusick, MENDELIAN INHERITANCE IN MAN, available on-line through Johns Hopkins University Welch Medical Library). The relationship between genes and disease, mapped to the same chromosomal region, can then be identified through linkage analysis (co-inheritance of physically adjacent genes), described in, *e.g.,* Egeland, *et al.,* 1987. *Nature,* 325: 783-787.

Moreover, differences in the DNA sequences between individuals affected and unaffected with a disease associated with the NOVX gene, can be determined. If a mutation is observed in some or all of the affected individuals but not in any unaffected individuals, then the mutation is likely to be the causative agent of the particular disease. Comparison of affected and unaffected individuals generally involves first looking for structural alterations in the chromosomes, such as deletions or translocations that are visible from chromosome spreads or detectable using PCR based on that DNA sequence. Ultimately, complete sequencing of genes from several individuals can be performed to confirm the presence of a mutation and to distinguish mutations from polymorphisms.

### Tissue Typing

The NOVX sequences of the invention can also be used to identify individuals from minute biological samples. In this technique, an individual's genomic DNA is digested with one or more restriction enzymes, and probed on a Southern blot to yield unique bands for identification. The sequences of the invention are useful as additional DNA markers for RFLP ("restriction fragment length polymorphisms," described in U.S. Patent No. 5,272,057).

Furthermore, the sequences of the invention can be used to provide an alternative technique that determines the actual base-by-base DNA sequence of selected portions of an individual's genome. Thus, the NOVX sequences described herein can be used to prepare two PCR primers from the 5'- and 3'-termini of the sequences. These primers can then be used to amplify an individual's DNA and subsequently sequence it.

Panels of corresponding DNA sequences from individuals, prepared in this manner, can provide unique individual identifications, as each individual will have a unique set of such DNA sequences due to allelic differences. The sequences of the invention can be used to obtain such identification sequences from individuals and from tissue. The NOVX sequences of the invention uniquely represent portions of the human genome. Allelic variation occurs to some degree in the coding regions of these sequences, and to a greater degree in the noncoding regions. It is estimated that allelic variation between individual humans occurs with a frequency of about once per each 500 bases. Much of the allelic variation is due to single nucleotide polymorphisms (SNPs), which include restriction fragment length polymorphisms (RFLPs).

Each of the sequences described herein can, to some degree, be used as a standard against which DNA from an individual can be compared for identification purposes. Because greater numbers of polymorphisms occur in the noncoding regions, fewer sequences are necessary to differentiate individuals. The noncoding sequences can comfortably provide positive individual identification with a panel of perhaps 10 to 1,000 primers that each yield a noncoding amplified sequence of 100 bases. If predicted coding sequences, such as those in SEQ ID NOS: 1, 3, 5, 7, 9, 11, 13, 15, 17, 19, 21, 23, 25, 27, 29, and 31 are used, a more appropriate number of primers for positive individual identification would be 500-2,000.

### Predictive Medicine

The invention also pertains to the field of predictive medicine in which diagnostic assays, prognostic assays, pharmacogenomics, and monitoring clinical trials are used for prognostic (predictive) purposes to thereby treat an individual prophylactically. Accordingly, one aspect of the invention relates to diagnostic assays for determining NOVX protein and/or nucleic acid expression as well as NOVX activity, in the context of a biological sample (e.g., blood, serum, cells, tissue) to thereby determine whether an individual is afflicted with a disease or disorder, or is at risk of developing a disorder, associated with aberrant NOVX expression or activity. The disorders include metabolic disorders, diabetes, obesity, infectious disease, anorexia, cancer-associated cachexia, cancer, neurodegenerative disorders, Alzheimer's Disease, Parkinson's Disorder, immune disorders, and hematopoietic disorders, and the various dyslipidemias, metabolic disturbances associated with obesity, the metabolic syndrome X and'wasting disorders associated with chronic diseases and various cancers. The invention also provides for prognostic (or predictive) assays for determining whether an individual is at risk of developing a disorder associated with NOVX protein, nucleic acid expression or activity. For example, mutations in an NOVX gene can be assayed in a biological sample. Such assays can be used for prognostic or predictive purpose to thereby prophylactically treat an individual prior to the onset of a disorder characterized by or associated with NOVX protein, nucleic acid expression, or biological activity.

Another aspect of the invention provides methods for determining NOVX protein, nucleic acid expression or activity in an individual to thereby select appropriate therapeutic or prophylactic agents for that individual (referred to herein as "pharmacogenomics"). Pharmacogenomics allows for the selection of agents *(e.g.,* drugs) for therapeutic or prophylactic treatment of an individual based on the genotype of the individual *(e.g.,* the genotype of the individual examined to determine the ability of the individual to respond to a particular agent.)

Yet another aspect of the invention pertains to monitoring the influence of agents (e.g., drugs, compounds) on the expression or activity ofNOVX in clinical trials.

These and other agents are described in further detail in the following sections.

### Diagnostic Assays

An exemplary method for detecting the presence or absence of NOVX in a biological sample involves obtaining a biological sample from a test subject and contacting the biological sample with a compound or an agent capable of detecting NOVX protein or nucleic acid *(e.g.,* mRNA, genomic DNA) that encodes NOVX protein such that the presence of NOVX is detected in the biological sample. An agent for detecting NOVX mRNA or genomic DNA is a labeled nucleic acid probe capable of hybridizing to NOVX mRNA or genomic DNA. The nucleic acid probe can be, for example, a full-length NOVX nucleic acid, such as the nucleic acid of SEQ ID NOS: 1, 3, 5, 7, 9, 11, 13, 15, 17, 19, 21, 23, 25, 27, 29, and 31, or a portion thereof, such as an oligonucleotide of at least 15, 30, 50, 100, 250 or 500 nucleotides in length and sufficient to specifically hybridize under stringent conditions to NOVX mRNA or genomic DNA. Other suitable probes for use in the diagnostic assays of the invention are described herein.

An agent for detecting NOVX protein is an antibody capable of binding to NOVX protein, preferably an antibody with a detectable label. Antibodies can be polyclonal, or more preferably, monoclonal. An intact antibody, or a fragment thereof (e.g., Fab or F(ab')₂) can be used. The term "labeled", with regard to the probe or antibody, is intended to encompass direct labeling of the probe or antibody by coupling *(i.e.,* physically linking) a detectable substance to the probe or antibody, as well as indirect labeling of the probe or antibody by reactivity with another reagent that is directly labeled. Examples of indirect labeling include detection of a primary antibody using a fluorescently-labeled secondary antibody and end-labeling of a DNA probe with biotin such that it can be detected with fluorescently-labeled streptavidin. The term "biological sample" is intended to include tissues, cells and biological fluids isolated from a subject, as well as tissues, cells and fluids present within a subject. That is, the detection method of the invention can be used to detect NOVX mRNA, protein, or genomic DNA in a biological sample *in vitro* as well as *in vivo.* For example, *in vitro* techniques for detection of NOVX mRNA include Northern hybridizations and *in situ* hybridizations. *In vitro* techniques for detection of NOVX protein include enzyme linked immunosorbent assays (ELISAs), Western blots, immunoprecipitations, and immunofluorescence. *In vitro* techniques for detection of NOVX genomic DNA include Southern hybridizations. Furthermore, *in vivo* techniques for detection ofNOVX protein include introducing into a subject a labeled anti-NOVX antibody. For example, the antibody can be labeled With a radioactive marker whose presence and location in a subject can be detected by standard imaging techniques.

In one embodiment, the biological sample contains protein molecules from the test subject. Alternatively, the biological sample can contain mRNA molecules from the test subject or genomic DNA molecules from the test subject. A preferred biological sample is a peripheral blood leukocyte sample isolated by conventional means from a subject.

In another embodiment, the methods further involve obtaining a control biological sample from a control subject, contacting the control sample with a compound or agent capable of detecting NOVX protein, mRNA, or genomic DNA, such that the presence of NOVX protein, mRNA or genomic DNA is detected in the biological sample, and comparing the presence of NOVX protein, mRNA or genomic DNA in the control sample with the presence of NOVX protein, mRNA or genomic DNA in the test sample.

The invention also encompasses kits for detecting the presence of NOVX in a biological sample. For example, the kit can comprise: a labeled compound or agent capable of detecting NOVX protein or mRNA in a biological sample; means for determining the amount of NOVX in the sample; and means for comparing the amount of NOVX in the sample with a standard. The compound or agent can be packaged in a suitable container. The kit can further comprise instructions for using the kit to detect NOVX protein or nucleic acid.

### Prognostic Assays

The diagnostic methods described herein can furthermore be utilized to identify subjects having or at risk of developing a disease or disorder associated with aberrant NOVX expression or activity. For example, the assays described herein, such as the preceding diagnostic assays or the following assays, can be utilized to identify a subject having or at risk of developing a disorder associated with NOVX protein, nucleic acid expression or activity. Alternatively, the prognostic assays can be utilized to identify a subject having or at risk for developing a disease or disorder. Thus, the invention provides a method for identifying a disease or disorder associated with aberrant NOVX expression or activity in which a test sample is obtained from a subject and NOVX protein or nucleic acid *(e.g.,* mRNA, genomic DNA) is detected, wherein the presence of NOVX protein or nucleic acid is diagnostic for a subject having or at risk of developing a disease or disorder associated with aberrant NOVX expression or activity. As used herein, a "test sample" refers to a biological sample obtained from a subject of interest. For example, a test sample can be a biological fluid (*e.g.,* serum), cell sample, or tissue.

Furthermore, the prognostic assays described herein can be used to determine whether a subject can be administered an agent (*e*.*g*., an agonist, antagonist, peptidomimetic, protein, peptide, nucleic acid, small molecule, or other drug candidate) to treat a disease or disorder associated with aberrant NOVX expression or activity. For example, such methods can be used to determine whether a subject can be effectively treated with an agent for a disorder. Thus, the invention provides methods for determining whether a subject can be effectively treated with an agent for a disorder associated with aberrant NOVX expression or activity in which a test sample is obtained and NOVX protein or nucleic acid is detected (*e.g.,* wherein the presence of NOVX protein or nucleic acid is diagnostic for a subject that can be administered the agent to treat a disorder associated with aberrant NOVX expression or activity).

The methods of the invention can also be used to detect genetic lesions in an NOVX gene, thereby determining if a subject with the lesioned gene is at risk for a disorder characterized by aberrant cell proliferation and/or differentiation. In various embodiments, the methods include detecting, in a sample of cells from the subject, the presence or absence of a genetic lesion characterized by at least one of an alteration affecting the integrity of a gene encoding an NOVX-protein, or the misexpression of the NOVX gene. For example, such genetic lesions can be detected by ascertaining the existence of at least one of: (*i*) a deletion of one or more nucleotides from an NOVX gene; (*ii*) an addition of one or more nucleotides to an NOVX gene; *(iii)* a substitution of one or more nucleotides of an NOVX gene, (iv) a chromosomal rearrangement of an NOVX gene; (v) an alteration in the level of a messenger RNA transcript of an NOVX gene, (*vi*) aberrant modification of an NOVX gene, such as of the methylation pattern of the genomic DNA, (vii) the presence of a non-wild-type splicing pattern of a messenger RNA transcript of an NOVX gene, (*viii*) a non-wild-type level of an NOVX protein, (ix) allelic loss of an NOVX gene, and (x) inappropriate post-translational modification of an NOVX protein. As described herein, there are a large number of assay techniques known in the art which can be used for detecting lesions in an NOVX gene. A preferred biological sample is a peripheral blood leukocyte sample isolated by conventional means from a subject. However, any biological sample containing nucleated cells may be used, including, for example, buccal mucosal cells.

In certain embodiments, detection of the lesion involves the use of a probe/primer in a polymerase chain reaction (PCR) *(see, e.g.,* U.S. Patent Nos. 4,683,195 and 4,683,202), such as anchor PCR or RACE PCR, or, alternatively, in a ligation chain reaction (LCR) *(see, eg.,* Landegran, *et al.,* 1988. *Science* 241: 1077-1080; and Nakazawa, *et al.,* 1994. *Proc. Natl. Acad. Sci. USA* 91: 360-364), the latter of which can be particularly useful for detecting point mutations in the NOVX-gene *(see,* Abravaya, *et al.,* 1995. *Nucl. Acids Res.* 23: 675-682). This method can include the steps of collecting a sample of cells from a patient, isolating nucleic acid *(e.g.,* genomic, mRNA or both) from the cells of the sample, contacting the nucleic acid sample with one or more primers that specifically hybridize to an NOVX gene under conditions such that hybridization and amplification of the NOVX gene (if present) occurs, and detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication *(see,* Guatelli, *et al.,* 1990. *Proc. Natl. Acad. Sci. USA* 87: 1874-1878), transcriptional amplification system *(see,* Kwoh, *et al.,* 1989. *Proc. Natl. Acad. Sci. USA* 86: 1173-1177); Qβ Replicase *(see, Lizardi, et al,* 1988. *BioTechnology* 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In an alternative embodiment, mutations in an NOVX gene from a sample cell can be identified by alterations in restriction enzyme cleavage patterns. For example, sample and control DNA is isolated, amplified (optionally), digested with one or more restriction endonucleases, and fragment length sizes are determined by gel electrophoresis and compared. Differences in fragment length sizes between sample and control DNA indicates mutations in the sample DNA. Moreover, the use of sequence specific ribozymes *(see, e.g.,* U.S. Patent No. 5,493,531) can be used to score for the presence of specific mutations by development or loss of a ribozyme cleavage site.

In other embodiments, genetic mutations in NOVX can be identified by hybridizing a sample and control nucleic acids, e.g., DNA or RNA, to high-density arrays containing hundreds or thousands of oligonucleotides probes. *See, e.g.,* Cronin, *et al., 1996. Human Mutation* 7: 244-255; *Kozal, et al.,* 1996. *Nat. Med.* 2: 753-759. For example, genetic mutations in NOVX can be identified in two dimensional arrays containing light-generated DNA probes as described in Cronin, *et al., supra.* Briefly, a first hybridization array of probes can be used to scan through long stretches of DNA in a sample and control to identify base changes between the sequences by making linear arrays of sequential overlapping probes. This step allows'the identification of point mutations. This is followed by a second hybridization array that allows the characterization of specific mutations by using smaller, specialized probe arrays complementary to all variants or mutations detected. Each mutation array is composed of parallel probe sets, one complementary to the wild-type gene and the other complementary to the mutant gene.

In yet another embodiment, any of a variety of sequencing reactions known in the art can be used to directly sequence the NOVX gene and detect mutations by comparing the sequence of the sample NOVX with the corresponding wild-type (control) sequence. Examples of sequencing reactions include those based on techniques developed by Maxim and Gilbert, 1977. *Proc. Natl. Acad Sci. USA* 74: 560 or Sanger, 1977. *Proc. Natl. Acad. Sci. USA* 74: 5463. It is also contemplated that any of a variety of automated sequencing procedures can be utilized when performing the diagnostic assays *(see, e.g.,* Naeve, *et al.,* 1995. *Biotechniques* 19: 448), including sequencing by mass spectrometry (see, eg., PCT International Publication No. WO 94/16101; Cohen, *et al.,* 1996. *Adv. Chromatography* 36: 127-162; and Griffin, *et al.,* 1993. *Appl. Biochem. Biotechnol.* 38: 147-159).

Other methods for detecting mutations in the NOVX gene include methods in which protection from cleavage agents is used to detect mismatched bases in RNA/RNA or RNA/DNA heteroduplexes. *See, e.g.,* Myers, *et al.,* 1985. *Science* 230: 1242. In general, the art technique of "mismatch cleavage" starts by providing heteroduplexes of formed by hybridizing (labeled) RNA or DNA containing the wild-type NOVX sequence with potentially mutant RNA or DNA obtained from a tissue sample. The double-stranded duplexes are treated with an agent that cleaves single-stranded regions of the duplex such as which will exist due to basepair mismatches between the control and sample strands. For instance, RNA/DNA duplexes can be treated with RNase and DNA/DNA hybrids treated with S₁ nuclease to enzymatically digesting the mismatched regions. In other embodiments, either DNA/DNA or RNA/DNA duplexes can be treated with hydroxylamine or osmium tetroxide and with piperidine in order to digest mismatched regions. After digestion of the mismatched regions, the resulting material is then separated by size on denaturing polyacrylamide gels to determine the site of mutation. *See, e.g., Cotton, et al.,* 1988. *Proc. Natl. Acad. Sci. USA* 85: 4397; Saleeba, *et al.,* 1992. *Methods Enzymol.* 217: 286-295. In an embodiment, the control DNA or RNA can be labeled for detection.

In still another embodiment, the mismatch cleavage reaction employs one or more proteins that recognize mismatched base pairs in double-stranded DNA (so called "DNA mismatch repair" enzymes) in defined systems for detecting and mapping point mutations in NOVX cDNAs obtained from samples of cells. For example, the mutY enzyme of *E. coli* cleaves A at G/A mismatches and the thymidine DNA glycosylase from HeLa cells cleaves T at G/T mismatches. *See, e.g., Hsu, et al.,* 1994. *Carcinogenesis* 15: 1657-1662. According to an exemplary embodiment, a probe based on an NOVX sequence, e.g., a wild-type NOVX sequence, is hybridized to a cDNA or other DNA product from a test cell(s). The duplex is treated with a DNA mismatch repair enzyme, and the cleavage products, if any, can be detected from electrophoresis protocols or the like. *See, e.g.,* U.S. Patent No. 5,459,039.

In other embodiments, alterations in electrophoretic mobility will be used to identify mutations in NOVX genes. For example, single strand conformation polymorphism (SSCP) may be used to detect differences in electrophoretic mobility between mutant and wild type nucleic acids. *See, e.g.,* Orita, *et al.,* 1989. *Proc. Natl. Acad. Sci. USA:* 86: 2766; Cotton, 1993. *Mutat. Res.* 285: 125-144; Hayashi, 1992. *Genet. Anal. Tech. Appl.* 9: 73-79. Single-stranded DNA fragments of sample and control NOVX nucleic acids will be denatured and allowed to renature. The secondary structure of single-stranded nucleic acids varies according to sequence, the resulting alteration in electrophoretic mobility enables the detection of even a single base change. The DNA fragments may be labeled or detected with labeled probes. The sensitivity of the assay may be enhanced by using RNA (rather than DNA), in which the secondary structure is more sensitive to a change in sequence. In one embodiment, the subject method utilizes heteroduplex analysis to separate double stranded heteroduplex molecules on the basis of changes in electrophoretic mobility. *See, e.g.,* Keen, *et al.,* 1991. *Trends Genet.* 7: 5.

In yet another embodiment, the movement of mutant or wild-type fragments'in polyacrylamide gels containing a gradient of denaturant is assayed using denaturing gradient gel electrophoresis (DGGE). *See, e.g.,* Myers, *et al.,* 1985. *Nature* 313: 495. When DGGE is used as the method of analysis, DNA will be modified to insure that it does not completely denature, for example by adding a GC clamp of approximately 40 bp of high-melting GC-rich DNA by PCR. In a further embodiment, a temperature gradient is used in place of a denaturing gradient to identify differences in the mobility of control and sample DNA. *See, e.g.,* Rosenbaum and Reissner, 1987. *Biophys. Chem.* 265: 12753.

Examples of other techniques for detecting point mutations include, but are not limited to, selective oligonucleotide hybridization, selective amplification, or selective primer extension. For example, oligonucleotide primers may be prepared in which the known mutation is placed centrally and then hybridized to target DNA under conditions that permit hybridization only if a perfect match is found. *See, e.g.,* Saiki, *et al.,* 1986. *Nature* 324: 163; Saiki, *et al.,* 1989. *Proc. Natl. Acad. Sci. USA* 86: 6230. Such allele specific oligonucleotides are hybridized to PCR amplified target DNA or a number of different mutations when the oligonucleotides are attached to the hybridizing membrane and hybridized with labeled target DNA.

Alternatively, allele specific amplification technology that depends on selective PCR amplification may be used in conjunction with the instant invention. Oligonucleotides used as primers for specific amplification may carry the mutation of interest in the center of the molecule (so that amplification depends on differential hybridization; *see, e.g.,* Gibbs, *et al.,* 1989. *Nucl. Acids Res.* 17: 2437-2448) or at the extreme 3'-terminus of one primer where, under appropriate conditions, mismatch can prevent, or reduce polymerase extension *(see, e.g.,* Prossner, 1993. *Tibtech.* 11: 238). In addition it may be desirable to introduce a novel restriction site in the region of the mutation to create cleavage-based detection. *See, e.g.,* Gasparini, *et al.,* 1992. *Mol. Cell Probes* 6: 1. It is anticipated that in certain embodiments amplification may also be performed using *Taq* ligase for amplification. *See, e.g.,* Barany, *1991. Proc. Natl. Acad. Sci. USA* 88: 189. In such cases, ligation will occur only if there is a perfect match at the 3'-terminus of the 5' sequence, making it possible to detect the presence of a known mutation at a specific site by looking for the presence or absence of amplification.

The methods described herein may be performed, for example, by utilizing pre-packaged diagnostic kits comprising at least one probe nucleic acid or antibody reagent described herein, which may be conveniently used, e.g., in clinical settings to diagnose patients exhibiting symptoms or family history of a disease or illness involving an NOVX gene.

Furthermore, any cell type or tissue, preferably peripheral blood leukocytes, in which NOVX is expressed may be utilized in the prognostic assays described herein. However, any biological sample containing nucleated cells may be used, including, for example, buccal mucosal cells.

### Pharmacogenomics

Agents, or modulators that have a stimulatory or inhibitory effect on NOVX activity *(e.g.,* NOVX gene expression), as identified by a screening assay described herein can be administered to individuals to treat (prophylactically or therapeutically) disorders (The disorders include metabolic disorders, diabetes, obesity, infectious disease, anorexia, cancer-associated cachexia, cancer, neurodegenerative disorders, Alzheimer's Disease, Parkinson's Disorder, immune disorders, and hematopoietic disorders, and the various dyslipidemias, metabolic disturbances associated with obesity, the metabolic syndrome X and wasting disorders associated with chronic diseases and various cancers.) In conjunction with such treatment, the pharmacogenomics (*i.e.,* the study of the relationship between an individual's genotype and that individual's response to a foreign compound or drug) of the individual may be considered. Differences in metabolism of therapeutics can lead to severe toxicity or therapeutic failure by altering the relation between dose and blood concentration of the pharmacologically active drug. Thus, the pharmacogenomics of the individual permits the selection of effective agents (e.g., drugs) for prophylactic or therapeutic treatments based on a consideration of the individual's genotype. Such pharmacogenomics can further be used to determine appropriate dosages and therapeutic regimens. Accordingly, the activity of NOVX protein, expression of NOVX nucleic acid, or mutation content of NOVX genes in an individual can be determined to thereby select appropriate agent(s) for therapeutic or prophylactic treatment of the individual.

Pharmacogenomics deals with clinically significant hereditary variations in the response to drugs due to altered drug disposition and abnormal action in affected persons. See e.g.,-Eichelbaum, *1996. Clin. Exp. Pharmacol. Physiol.,* 23: 983-985; Linder, 1997. *Clin. Chem.,* 43: 254-266. In general, two types of pharmacogenetic conditions can be differentiated. Genetic conditions transmitted as a single factor altering the way drugs act on the body (altered drug action) or genetic conditions transmitted as single factors altering the way the body acts on drugs (altered drug metabolism). These pharmacogenetic conditions can occur either as rare defects or as polymorphisms. For example, glucose-6-phosphate dehydrogenase (G6PD) deficiency is a common inherited enzymopathy in which the main clinical complication is hemolysis after ingestion of oxidant drugs (anti-malarials, sulfonamides, analgesics, nitrofurans) and consumption of fava beans.

As an illustrative embodiment, the activity of drug metabolizing enzymes is a major determinant of both the intensity and duration of drug action. The discovery of genetic polymorphisms of drug metabolizing enzymes (*e*.*g*., N-acetyltransferase 2 (NAT 2) and cytochrome P450 enzymes CYP2D6 and CYP2C19) has provided an explanation as to why some patients do not obtain the expected drug effects or show exaggerated drug response and serious toxicity after taking the standard and safe dose of a drug. These polymorphisms are expressed in two phenotypes in the population, the extensive metabolizer (EM) and poor metabolizer (PM). The prevalence of PM is different among different populations. For example, the gene coding for CYP2D6 is highly polymorphic and several mutations have been identified in PM, which all lead to the absence of functional CYP2D6. Poor metabolizers of CYP2D6 and CYP2C19 quite frequently experience exaggerated drug response and side effects when they receive standard doses. If a metabolite is the active therapeutic moiety, PM show no therapeutic response, as demonstrated for the analgesic effect of codeine mediated by its CYP2D6-formed metabolite morphine. At the other extreme are the so called ultra-rapid metabolizers who do not respond to standard doses. Recently, the molecular basis of ultra-rapid metabolism has been identified to be due to CYP2D6 gene amplification.

Thus, the activity of NOVX protein, expression of NOVX nucleic acid, or mutation content of NOVX genes in an individual can be determined to thereby select appropriate agent(s) for therapeutic or prophylactic treatment of the individual. In addition, pharmacogenetic studies can be used to apply genotyping of polymorphic alleles encoding drug-metabolizing enzymes to the identification of an individual's drug responsiveness phenotype. This knowledge, when applied to dosing or drug selection, can avoid adverse reactions or therapeutic failure and thus enhance therapeutic or prophylactic efficiency when treating a subject with an NOVX modulator, such as a modulator identified by one of the exemplary screening assays described herein.

### Monitoring of Effects During Clinical Trials

Monitoring the influence of agents (e.g., drugs, compounds) on the expression or activity of NOVX (*e.g.,* the ability to modulate aberrant cell proliferation and/or differentiation) can be applied not only in basic drug screening, but also in clinical trials. For example, the effectiveness of an agent determined by a screening assay as described herein to increase NOVX gene expression, protein levels, or upregulate NOVX activity, can be monitored in clinical trails of subjects exhibiting decreased NOVX gene expression, protein levels, or downregulated NOVX activity. Alternatively, the effectiveness of an agent determined by a screening assay to decrease NOVX gene expression, protein levels, or downregulate NOVX activity, can be monitored in clinical trails of subjects exhibiting increased NOVX gene expression, protein levels, or upregulated NOVX activity. In such clinical trials, the expression or activity of NOVX and, preferably, other genes that have been implicated in, for example, a cellular proliferation or immune disorder can be used as a "read out" or markers of the immune responsiveness of a particular cell.

By way of example, and not of limitation, genes, including NOVX, that are modulated in cells by treatment with an agent (e.g., compound, drug or small molecule) that modulates NOVX activity (e.g., identified in a screening assay as described herein) can be identified. Thus, to study the effect of agents on cellular proliferation disorders, for example, in a clinical trial, cells can be isolated and RNA prepared and analyzed for the levels of expression of NOVX and other genes implicated in the disorder. The levels of gene expression *(i.e.,* a gene expression pattern) can be quantified by Northern blot analysis or RT-PCR, as described herein, or alternatively by measuring the amount of protein produced, by one of the methods as described herein, or by measuring the levels of activity of NOVX or other genes. In this manner, the gene expression pattern can serve as a marker, indicative of the physiological response of the cells to the agent. Accordingly, this response state may be determined before, and at various points during, treatment of the individual with the agent.

In one embodiment, the invention provides a method for monitoring the effectiveness of treatment of a subject with an agent (e.g., an agonist, antagonist, protein, peptide, peptidomimetic, nucleic acid, small molecule, or other drug candidate identified by the screening assays described herein) comprising the steps of *(i)* obtaining a pre-administration sample from a subject prior to administration of the agent; (ii) detecting the level of expression of an NOVX protein, mRNA, or genomic DNA in the preadministration sample; (iii) obtaining one or more post-administration samples from the subject; (iv) detecting the level of expression or activity of the-NOVX protein, mRNA, or genomic DNA in the post-administration samples; (v) comparing the level of expression or activity of the NOVX protein, mRNA, or genomic DNA in the pre-administration sample with the NOVX protein, mRNA, or genomic DNA in the post administration sample or samples; and (*vi*) altering the administration of the agent to the subject accordingly. For example, increased administration of the agent may be desirable to increase the expression or activity of NOVX to higher levels than detected, i.e., to increase the effectiveness of the agent. Alternatively, decreased administration of the agent may'be desirable to decrease expression or activity of NOVX to lower levels than detected, *i.e.*, to decrease the effectiveness of the agent.

### Methods of Treatment

The invention provides for both prophylactic and therapeutic methods of treating a subject at risk of (or susceptible to) a disorder or having a disorder associated with aberrant NOVX expression or activity. The disorders include cardiomyopathy, atherosclerosis, hypertension, congenital heart defects, aortic stenosis, atrial septal defect (ASD), atrioventricular (A-V) canal defect, ductus arteriosus, pulmonary stenosis, subaortic stenosis, ventricular septal defect (VSD), valve diseases, tuberous sclerosis, scleroderma, obesity, transplantation, adrenoleukodystrophy, congenital adrenal hyperplasia, prostate cancer, neoplasm; adenocarcinoma, lymphoma, uterus cancer, fertility, hemophilia, hypercoagulation, idiopathic thrombocytopenic purpura, immunodeficiencies, graft versus host disease, AIDS, bronchial asthma, Crohn's disease; multiple sclerosis, treatment of Albright Hereditary Ostoeodystrophy, and other diseases, disorders and conditions of the like.

These methods of treatment will be discussed more fully, below.

### Disease and Disorders

Diseases and disorders that are characterized by increased (relative to a subject not suffering from the disease or disorder) levels or biological activity may be treated with Therapeutics that antagonize (*i.e.*, reduce or inhibit) activity. Therapeutics that antagonize activity may be administered in a therapeutic or prophylactic manner. Therapeutics that may be utilized include, but are not limited to: *(i)* an aforementioned peptide, or analogs, derivatives, fragments or homologs thereof; *(ii)* antibodies to an aforementioned peptide; *(iii)* nucleic acids encoding an aforementioned peptide; *(iv)* administration of antisense nucleic acid and nucleic acids that are "dysfunctional" *(i.e.,* due to a heterologous insertion within the coding sequences of coding sequences to an aforementioned peptide) that are utilized to "knockout" endogenous function of an aforementioned peptide by homologous recombination *(see, e.g.,* Capecchi, 1989. *Science* 244: 1288-1292); or (v) modulators *(i.e.,* inhibitors, agonists and antagonists, including additional peptide mimetic of the invention or antibodies specific to a peptide of the invention) that alter the interaction between an aforementioned peptide and its binding partner.

Diseases and disorders that are characterized by decreased (relative to a subject not suffering from the disease or disorder) levels or biological activity may be treated with Therapeutics that increase *(i.e.,* are agonists to) activity. Therapeutics that upregulate activity may be administered in a therapeutic or prophylactic manner. Therapeutics that may be utilized include, but are not limited to, an aforementioned peptide, or analogs, derivatives, fragments or homologs thereof; or an agonist that increases bioavailability.

Increased or decreased levels can be readily detected by quantifying peptide and/or RNA, by obtaining a patient tissue sample (e.g., from biopsy tissue) and assaying it *in vitro* for RNA or peptide levels, structure and/or activity of the expressed peptides (or mRNAs of an aforementioned peptide). Methods that are well-known within the art include, but are not limited to, immunoassays (*e.g*., by Western blot analysis, immunoprecipitation followed by sodium dodecyl sulfate (SDS) polyacrylamide gel electrophoresis, immunocytochemistry, etc.) and/or hybridization assays to detect expression of mRNAs *(e.g.,* Northern assays, dot blots, *in situ* hybridization, and the like).

### Prophylactic Methods

In one aspect, the invention provides a method for preventing, in a subject, a disease or condition associated with an aberrant NOVX expression or activity, by administering to the subject an agent that modulates NOVX expression or at least one NOVX activity. Subjects at risk for a disease that is caused or contributed to by aberrant NOVX expression or activity can be identified by, for example, any or a combination of diagnostic or prognostic assays as described herein. Administration of a prophylactic agent can occur prior to the manifestation of symptoms characteristic of the NOVX aberrancy, such that a disease or disorder is prevented or, alternatively, delayed in its progression. Depending upon the type of NOVX aberrancy, for example, an NOVX agonist or NOVX antagonist agent can be used for treating the subject. The appropriate agent can be determined based on screening assays described herein. The prophylactic methods of the invention are further discussed in the following subsections.

### Therapeutic Methods

Another aspect of the invention pertains to methods of modulating NOVX expression or activity for therapeutic purposes. The modulatory method of the invention involves contacting a cell with an agent that modulates one or more of the activities of NOVX protein activity associated with the cell. An agent that modulates NOVX protein activity can be an agent as described herein, such as a nucleic acid or a protein, a naturally-occurring cognate ligand of an NOVX protein, a peptide, an NOVX peptidomimetic, or other small molecule. In one embodiment, the agent stimulates one or more NOVX protein activity. Examples of such stimulatory agents include active NOVX protein and a nucleic acid molecule encoding NOVX that has been introduced into the cell. In another embodiment, the agent inhibits one or more NOVX protein activity. Examples of such inhibitory agents include antisense NOVX nucleic acid molecules and anti-NOVX antibodies. These modulatory methods can be performed in *vitro (e.g.,* by culturing the cell with the agent) or, alternatively, *in vivo (e.g.,* by administering the agent to a subject). As such, the invention provides methods of treating an individual afflicted with a disease or disorder characterized by aberrant expression or activity of an NOVX protein or nucleic acid molecule. In one embodiment, the method involves administering an agent (*e.g.*, an agent identified by a screening assay described herein), or combination of agents that modulates (*e.g.,* up-regulates or down-regulates) NOVX expression or activity. In another embodiment, the method involves administering an NOVX protein or nucleic acid molecule as therapy to compensate for reduced or aberrant NOVX expression or activity.

Stimulation of NOVX activity is desirable in situations in which NOVX is abnormally downregulated and/or in which increased NOVX activity is likely to have a beneficial effect. One example of such a situation is where a subject has a disorder characterized by aberrant cell proliferation and/or differentiation *(e.g.,* cancer or immune associated disorders). Another example of such a situation is where the subject has a gestational disease *(e.g.,* preclampsia).

### Determination of the Biological Effect of the Therapeutic

In various embodiments of the invention, suitable *in vitro* or *in vivo* assays are performed to determine the effect of a specific Therapeutic and whether its administration is indicated for treatment of the affected tissue.

In various specific embodiments, *in vitro* assays may be performed with representative cells of the type(s) involved in the patient's disorder, to determine if a given Therapeutic exerts the desired effect upon the cell type(s). Compounds for use in therapy may be tested in suitable animal model systems including, but not limited to rats, mice, chicken, cows, monkeys, rabbits, and the like, prior to testing in human subjects. Similarly, for *in vivo* testing, any of the animal model system known in the art may be used prior to administration to human subjects.

### Prophylactic and Therapeutic Uses of the Compositions of the Invention

The NOVX nucleic acids and proteins of the invention are useful in potential prophylactic and therapeutic applications implicated in a variety of disorders including, but not limited to: metabolic disorders, diabetes, obesity, infectious disease, anorexia, cancer-associated cancer, neurodegenerative disorders, Alzheimer's Disease, Parkinson's Disorder, immune disorders, hematopoietic disorders, and the various dyslipideniias, metabolic disturbances associated with obesity, the metabolic syndrome X and wasting disorders associated with chronic diseases and various cancers.

As an example, a cDNA encoding the NOVX protein of the invention may be useful in gene therapy, and the protein may be useful when administered to a subject in need thereof. By way of non-limiting example, the compositions of the invention will have efficacy for treatment of patients suffering from: metabolic disorders, diabetes, obesity, infectious disease, anorexia, cancer-associated cachexia, cancer, neurodegenerative disorders, Alzheimer's Disease, Parkinson's Disorder, immune disorders, hematopoietic disorders, and the various dyslipidemias.

Both the novel nucleic acid encoding the NOVX protein, and the NOVX protein of the invention, or fragments thereof, may also be useful in diagnostic applications, wherein the presence or amount of the nucleic acid or the protein are to be assessed. A further use could be as an anti-bacterial molecule *(i.e.,* some peptides have been found to possess anti-bacterial properties). These materials are further useful in the generation of antibodies, which immunospecifically-bind to the novel substances of the invention for use in therapeutic or diagnostic methods.

The invention will be further described in the following examples, which do not limit the scope of the invention described in the claims.

### Examples

### Example 1. Identification of NOVX clones

The novel NOVX target sequences identified in the present invention were subjected to the exon linking process to confirm the sequence. PCR primers were designed by starting at the most upstream sequence available, for the forward primer, and at the most downstream sequence available for the reverse primer. Table 13A shows the sequences of the PCR primers used for obtaining different clones. In each case, the sequence was examined, walking inward from the respective termini toward the coding sequence, until a suitable sequence that is either unique or highly selective was encountered, or, in the case of the reverse primer, until the stop codon was reached. Such primers were designed based on in silico predictions for the full length cDNA, part (one or more exons) of the DNA or protein sequence of the target sequence, or by translated homology of the predicted exons to closely related human sequences from other species. These primers were then employed in PCR amplification based on the following pool of human cDNAs: adrenal gland, bone marrow, brain - amygdala, brain - cerebellum, brain - hippocampus, brain - substantia nigra, brain - thalamus, brain -whole, fetal brain, fetal kidney, fetal liver, fetal lung, heart, kidney, lymphoma - Raji, mammary gland, pancreas, pituitary gland, placenta, prostate, salivary gland, skeletal muscle, small intestine, spinal cord, spleen, stomach, testis, thyroid, trachea, uterus. Usually the resulting amplicons were gel purified, cloned and sequenced to high redundancy. The PCR product derived from exon linking was cloned into the pCR2.1 vector from Invitrogen. The resulting bacterial clone has an insert covering the entire open reading frame cloned into the pCR2.1 vector. Table 13B shows a list of these bacterial clones. The resulting sequences from all clones were assembled with themselves, with other fragments in CuraGen Corporation's database and with public ESTs. Fragments and ESTs were included as components for an assembly when the extent of their identity with another component of the assembly was at least 95% over 50 bp. In addition, sequence traces were evaluated manually and edited for corrections if appropriate. These procedures provide the sequence reported herein.

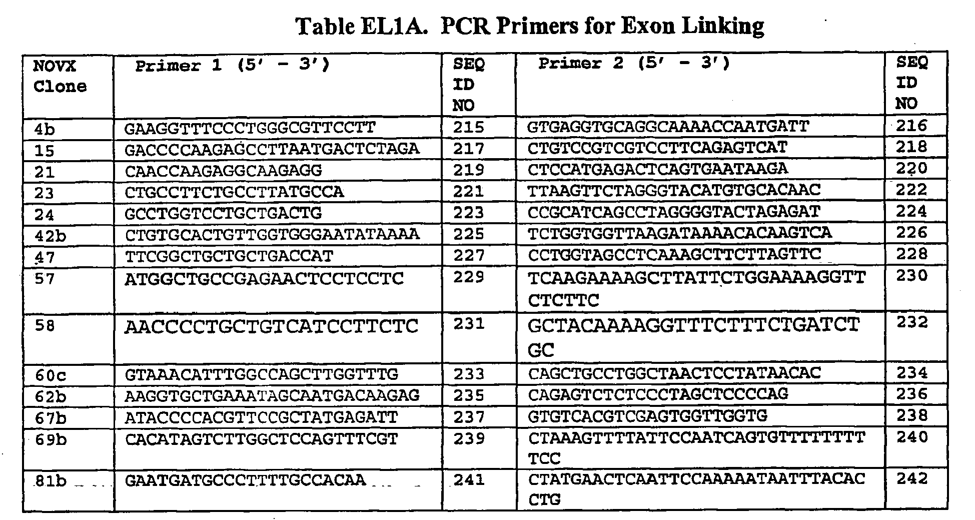

Physical clone: Exons were predicted by homology and the intron/exon boundaries were determined using standard genetic rules. Exons were further selected and refined by means of similarity determination using multiple BLAST (for example, tBlastN, BlastX, and BlastN) searches, and, in some instances, GeneScan and Grail. Expressed sequences from both public and proprietary databases were also added when available to further define and complete the gene sequence. The DNA sequence was then manually corrected for apparent inconsistencies thereby obtaining the sequences encoding the full-length protein.

### Example 2. Quantitative expression analysis of clones in various tissues and cells

The quantitative expression of various clones was assessed using microtiter plates containing RNA samples from a variety of normal and pathology-derived cells, cell lines and tissues using real time quantitative PCR (RTQ PCR). RTQ PCR was performed on an Applied Biosystems ABI PRISM® 7700 or an ABI PRISM® 7900 HT Sequence Detection System. Various collections of samples are assembled on the plates, and referred to as Panel 1 (containing normal tissues and cancer cell lines), Panel 2 (containing samples derived from tissues from normal and cancer sources), Panel 3 (containing cancer cell lines), Panel 4 (containing cells and cell lines from normal tissues and cells related to inflammatory conditions), Panel 5D/5I (containing human tissues and cell lines with an emphasis on metabolic diseases), AI_comprehensive_panel (containing normal tissue and samples from autoimmune diseases), Panel CNSD.01 (containing central nervous system samples from normal and diseased brains) and CNS_neurodegeneration_panel (containing samples from normal and Alzheimer's diseased brains).

RNA integrity from all samples is controlled for quality by visual assessment of agarose gel electropherograms using 28S and 18S ribosomal RNA staining intensity ratio as a guide (2:1 to 2.5:1 28s:18s) and the absence of low molecular weight RNAs that would be indicative of degradation products. Samples are controlled against genomic DNA contamination by RTQ PCR reactions run in the absence of reverse transcriptase using probe and primer sets designed to amplify across the span of a single exon.

First, the RNA samples were normalized to reference nucleic acids such as constitutively expressed genes (for example, β-actin and GAPDH). Normalized RNA (5 ul) was converted to cDNA and analyzed by RTQ-PCR using One Step RT-PCR Master Mix Reagents (Applied Biosystems; Catalog No. 4309169) and gene-specific primers according to the manufacturer's instructions.

In other cases, non-normalized RNA samples were converted to single strand cDNA (sscDNA) using Superscript II (Invitrogen Corporation; Catalog No. 18064-147) and random hexamers according to the manufacturer's instructions. Reactions containing up to 10 µg of total RNA were performed in a volume of 20 µl and incubated for 60 minutes at 42°C. This reaction can be scaled up to 50 µg of total RNA in a fmal volume of 100 µl. sscDNA samples are then normalized to reference nucleic acids as described previously, using 1X TaqMan® Universal Master mix (Applied Biosystems; catalog No. 4324020), following the manufacturer's instructions.

Probes and primers were designed for each assay according to Applied Biosystems Primer Express Software package (version I for Apple Computer's Macintosh Power PC) or a similar algorithm using the target sequence as input. Default settings were used for reaction conditions and the following parameters were set before selecting primers: primer concentration = 250 nM, primer melting temperature (Tm) range = 58°-60°C, primer optimal Tm = 59°C, maximum primer difference = 2°C, probe does not have 5'G, probe Tm must be 10°C greater than primer Tm, amplicon size 75bp to 100bp. The probes and primers selected' (see below) were synthesized by Synthegen (Houston, TX, USA). Probes were double purified by HPLC to remove uncoupled dye and evaluated by mass spectroscopy to verify coupling of reporter and quencher dyes to the 5' and 3' ends of the probe, respectively. Their final concentrations were: forward and reverse primers, 900nM each, and probe, 200nM.

PCR conditions: When working with RNA samples, normalized RNA from each tissue and each cell line was spotted in each well of either a 96 well or a 384-well PCR plate (Applied Biosystems). PCR cocktails included either a single gene specific probe and primers set, or two multiplexed probe and primers sets (a set specific for the target clone and another gene-specific set multiplexed with the target probe). PCR reactions were set up using TaqMan® One-Step RT-PCR Master Mix (Applied Biosystems, Catalog No. 4313803) following manufacturer's instructions. Reverse transcription was performed at 48°C for 30 minutes followed by amplification/PCR cycles as follows: 95°C 10 min, then 40 cycles of 95°C for 15 seconds, 60°C for 1 minute. Results were recorded as CT values (cycle at which a given sample crosses a threshold level of fluorescence) using a log scale, with the difference in RNA concentration between a given sample and the sample with the lowest CT value being represented as 2 to the power of delta CT. The percent relative expression is then obtained by taking the reciprocal of this RNA difference and multiplying by 100.

When working with sscDNA samples, normalized sscDNA was used as described previously for RNA samples. PCR reactions containing one or two sets of probe and primers were set up as described previously, using 1X TaqMan® Universal Master mix (Applied Biosystems; catalog No. 4324020), following the manufacturer's instructions. PCR amplification was performed as follows: 95°C 10 min, then 40 cycles of 95°C for 15 seconds, 60°C for 1 minute. Results were analyzed and processed as described previously.

### Panels 1, 1.1, 1.2, and 1.3D

The plates for Panels 1, 1.1, 1.2 and 1.3D include 2 control wells (genomic DNA control and chemistry control) and 94 wells containing cDNA from various samples. The samples in these panels are broken into 2 classes: samples derived from cultured cell lines and samples derived from primary normal tissues. The cell lines are derived from cancers of the following types: lung cancer, breast cancer, melanoma, colon cancer, prostate cancer, CNS cancer, squamous cell carcinoma, ovarian cancer, liver cancer, renal cancer, gastric cancer and pancreatic cancer. Cell lines used in these panels are widely available through the American Type Culture Collection (ATCC), a repository for cultured cell lines, and were cultured using the conditions recommended by the ATCC. The normal tissues found on these panels are comprised of samples derived from all major organ systems from single adult individuals or fetuses. These samples are derived from the following organs: adult skeletal muscle, fetal skeletal muscle, adult heart, fetal heart, adult kidney, fetal kidney, adult liver, fetal liver, adult lung, fetal lung, various regions of the brain, the spleen, bone marrow, lymph node, pancreas, salivary gland, pituitary gland, adrenal gland, spinal cord, thymus, stomach, small intestine, colon, bladder, trachea, breast, ovary, uterus, placenta, prostate, testis and adipose.

In the results for Panels 1, 1.1,1.2 and 1.3D, the following abbreviations are used:
ca. = carcinoma,
* = established from metastasis,
met = metastasis,
s cell var = small cell variant,
non-s = non-sm = non-small,
squam = squamous,
pl. eff= pl effusion = pleural effusion,
glio = glioma,
astro = astrocytoma, and
neuro = neuroblastoma.
**General_screening_panel_v1.4**

The plates for Panel 1.4 include 2 control wells (genomic DNA control and chemistry control) and 94 wells containing cDNA from various samples. The samples in Panel 1.4 are broken into 2 classes: samples derived from cultured cell lines and samples derived from primary normal tissues. The cell lines are derived from cancers of the following types: lung cancer, breast cancer, melanoma, colon cancer, prostate cancer, CNS cancer, squamous cell carcinoma, ovarian cancer, liver cancer, renal cancer, gastric cancer and pancreatic cancer. Cell lines used in Panel 1.4 are widely available through the American Type Culture Collection (ATCC), a repository for cultured cell lines, and were cultured using the conditions recommended by the ATCC. The normal tissues found on Panel 1.4 are comprised of pools of samples derived from all major organ systems from 2 to 5 different adult individuals or fetuses. These samples are derived from the following organs: adult skeletal muscle, fetal skeletal muscle, adult heart, fetal heart, adult kidney, fetal kidney, adult liver, fetal liver, adult lung, fetal lung, various regions of the brain, the spleen, bone marrow, lymph node, pancreas, salivary gland, pituitary gland, adrenal gland, spinal cord, thymus, stomach, small intestine, colon, bladder, trachea, breast, ovary, uterus, placenta, prostate, testis and adipose. Abbreviations are as described for Panels 1, 1.1, 1.2, and 1.3D.

### Panels 2D and 2.2

The plates for Panels 2D and 2.2 generally include 2 control wells and 94 test samples composed of RNA or cDNA isolated from human tissue procured by surgeons working in close cooperation with the National Cancer Institute's Cooperative Human Tissue Network (CHTN) or the National Disease Research Initiative (NDRI). The tissues are derived from human malignancies and in cases where indicated many malignant tissues have "matched margins" obtained from noncancerous tissue just adjacent to the tumor. These are termed normal adjacent tissues and are denoted "NAT" in the results below. The tumor tissue and the "matched margins" are evaluated by two independent pathologists (the surgical pathologists and again by a pathologist at NDRI or CHTN). This analysis provides a gross histopathological assessment of tumor differentiation grade. Moreover, most samples include the original surgical pathology report that provides information regarding the clinical stage of the patient. These matched margins are taken from the tissue surrounding (i.e. immediately proximal) to the zone of surgery (designated "NAT", for normal adjacent tissue, in Table RR). In addition, RNA and cDNA samples were obtained from various human tissues derived from autopsies performed on elderly people or sudden death victims (accidents, etc.). These tissues were ascertained to be free of disease and were purchased from various commercial sources such as Clontech (Palo Alto, CA), Research Genetics, and Invitrogen.

### Panel 3D

The plates of Panel 3D are comprised of 94 cDNA samples and two control samples. Specifically, 92 of these samples are derived from cultured human cancer cell lines, 2 samples of human primary cerebellar tissue and 2 controls. The human cell lines are generally obtained from ATCC (American Type Culture Collection), NCI or the German tumor cell bank and fall into the following tissue groups: Squamous cell carcinoma of the tongue, breast cancer, prostate cancer, melanoma, epidermoid carcinoma, sarcomas, bladder carcinomas, pancreatic cancers, kidney cancers, leukemias/lymphomas, ovarian/uterine/cervical, gastric, colon, lung and CNS cancer cell lines. In addition, there are two independent samples of cerebellum. These cells are all cultured under standard recommended conditions and RNA extracted using the standard procedures. The cell lines in panel 3D and 1.3D are of the most common cell lines used in the scientific literature.

### Panels 4D, 4R, and 4.1D

Panel 4 includes samples on a 96 well plate (2 control wells, 94 test samples) composed of RNA (Pane14R) or cDNA (Panels 4D/4.1D) isolated from various human cell lines or tissues related to inflammatory conditions. Total RNA from control normal tissues such as colon and lung (Stratagene, La Jolla, CA) and thymus and kidney (Clontech) was employed. Total RNA from liver tissue from cirrhosis patients and kidney from lupus patients was obtained from BioChain (Biochain Institute, Inc., Hayward, CA). Intestinal tissue for RNA preparation from patients diagnosed as having Crohn's disease and ulcerative colitis was obtained from the National Disease Research Interchange (NDRI) (Philadelphia, PA).

Astrocytes, lung fibroblasts, dermal fibroblasts, coronary artery smooth muscle cells, small airway epithelium, bronchial epithelium, microvascular dermal endothelial cells, microvascular lung endothelial cells, human pulmonary aortic endothelial cells, human umbilical vein endothelial cells were all purchased from Clonetics (Wallcersville, MD) and grown in the media supplied for these cell types by Clonetics. These primary cell types were activated with various cytokines or combinations ofcytokines for 6 and/or 12-14 hours, as indicated. The following cytokines were used; IL-1 beta at approximately 1-5ng/ml, TNF alpha at approximately 5-10ng/ml, IFN gamma at approximately 20-50ng/ml, IL-4 at approximately 5-10ng/ml, IL-9 at approximately 5-10ng/ml, IL-13 at approximately 5-10ng/ml. Endothelial cells were sometimes starved for various times by culture in the basal media from Clonetics with 0.1% serum.

Mononuclear cells were prepared from blood of employees at CuraGen Corporation, using Ficoll. LAK cells were prepared from these cells by culture in DMEM 5% FCS (Hyclone), 100µM non essential amino acids (Gibco/Life Technologies, Rockville, MD), 1mM sodium pyruvate (Gibco), mercaptoethanol 5.5x10⁻⁵M (Gibco), and 10mM Hepes (Gibco) and Interleukin 2 for 4-6 days. Cells were then either activated with 10-20ng/ml PMA and 1-2µg/ml ionomycin, IL-12 at 5-10ng/ml, IFN gamma at 20-50ng/ml and IL-18 at 5-10ng/ml for 6 hours. In some cases, mononuclear cells were cultured for 4-5 days in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) with PHA (phytohemagglutinin) or PWM (pokeweed mitogen) at approximately 5µg/ml. Samples were taken at 24, 48 and 72 hours for RNA preparation. MLR (mixed lymphocyte reaction) samples were obtained by taking blood from two donors, isolating the mononuclear cells using Ficoll and mixing the isolated mononuclear cells 1:1 at a final concentration of approximately 2x10⁶cells/ml in DMEM 5% FCS (Hyclone), 100µM non essential amino acids (Gibco), 1mM sodium pyruvate (Gibco), mercaptoethanol (5.5x10⁻⁵M) (Gibco), and 10mM Hepes (Gibco). The MLR was cultured and samples taken at various time points ranging from 1-7 days for RNA preparation.

Monocytes were isolated from mononuclear cells using CD14 Miltenyi Beads, +ve VS selection columns and a Vario Magnet according to the manufacturer's instructions. Monocytes were differentiated into dendritic cells by culture in DMEM 5% fetal calf serum (FCS) (Hyclone, Logan, UT), 100µM non essential amino acids (Gibco), 1mM sodium pyruvate (Gibco), mercaptoethanol 5.5x10⁻⁵M (Gibco), and 10mM Hepes (Gibco), 50ng/ml GMCSF and 5ng/ml IL-4 for 5-7 days. Macrophages were prepared by culture of monocytes for 5-7 days in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and 10% AB Human Serum or MCSF at approximately 50ng/ml. Monocytes, macrophages and dendritic cells were stimulated for 6 and 12-14 hours with lipopolysaccharide (LPS) at 100ng/ml. Dendritic cells were also stimulated with anti-CD40 monoclonal antibody (Pharmingen) at 10µg/ml for 6 and 12-14 hours.

CD4 lymphocytes, CD8 lymphocytes and NK cells were also isolated from mononuclear cells using CD4, CD8 and CD56 Miltenyi beads, positive VS selection columns and a Vario Magnet according to the manufacturer's instructions. CD45RA and CD45RO CD4 lymphocytes were isolated by depleting mononuclear cells of CD8, CD56, CD14 and CD19 cells using CD8, CD56, CD14 and CD19 Miltenyi beads and positive selection. CD45RO beads were then used to isolate the CD45RO CD4 lymphocytes with the remaining cells being CD45RA CD4 lymphocytes. CD45RA CD4, CD45RO CD4 and CD8 lymphocytes were placed in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) and plated at 10⁶cells/ml onto Falcon 6 well tissue culture plates that had been coated overnight with 0.5µg/ml anti-CD28 (Pharmingen) and 3ug/ml anti-CD3 (OKT3, ATCC) in PBS. After 6 and 24 hours, the cells were harvested for RNA preparation. To prepare chronically activated CD8 lymphocytes, the isolated CD8 lymphocytes were activated for 4 days on anti-CD28 and anti-CD3 coated plates and then harvested the cells and expanded them in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) and IL-2. The expanded CD8 cells were then activated again with plate bound anti-CD3 and anti-CD28 for 4 days and expanded as before. RNA was isolated 6 and 24 hours after the second activation and after 4 days of the second expansion culture. The isolated NK cells were cultured in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco) and IL-2 for 4-6 days before RNA was prepared.

To obtain B cells, tonsils were procured from NDRI. The tonsil was cut up with sterile dissecting scissors and then passed through a sieve. Tonsil cells were then spun down and resupended at 10⁶cells/ml in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco). To activate the cells, PWM was used at 5µg/ml or anti-CD40 (Pharmingen) at approximately 10µg/ml and IL-4 at 5-10ng/ml. Cells were harvested for RNA preparation at 24,48 and 72 hours.

To prepare the primary and secondary Thl/Th2 and Tr1 cells, six-well Falcon plates were coated overnight with 10µg/ml anti-CD28 (Pharmingen) and 2µg/ml OKT3 (ATCC), and then washed twice with PBS. Umbilical cord blood CD4 lymphocytes (Poietic Systems, German Town, MD) were cultured at 10⁵-10⁶cells/ml in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and IL-2 (4ng/ml). IL-12 (5ng/ml) and anti-IL4 (1µg/ml) were used to direct to Th1, while IL-4 (5ng/ml) and anti-IFN gamma (1µg/ml) were used to direct to Th2 and IL-10 at 5ng/ml was used to direct to Tr1. After 4-5 days, the activated Th1, Th2 and Tr1 lymphocytes were washed once in DMEM and expanded for 4-7 days in DMEM 5% FCS (Hyclone), 100µM non essential amino acids (Gibco), 1mM sodium pyruvate (Gibco), mercaptoethanol 5.5x10⁻⁵M (Gibco), 10mM Hepes (Gibco) and IL-2 (1ng/ml). Following this, the activated Th1, Th2 and Tr1 lymphocytes were re-stimulated for 5 days with anti-CD28/OKT3 and cytokines as described above, but with the addition of anti-CD95L (1µg/ml) to prevent apoptosis. After 4-5 days, the Th1, Th2 and Tr1 lymphocytes were washed and then expanded again with IL-2 for 4-7 days. Activated Th1 and Th2 lymphocytes were maintained in this way for a maximum of three cycles. RNA was prepared from primary and secondary Th1, Th2 and Trl after 6 and 24 hours following the second and third activations with plate bound anti-CD3 and anti-CD28 mAbs and 4 days into the second and third expansion cultures in Interleukin 2.

The following leukocyte cells lines were obtained from the ATCC: Ramos, EOL-1, KU-812. EOL cells were further differentiated by culture in 0.1mM dbcAMP at 5x10⁵cells/ml for 8 days, changing the media every 3 days and adjusting the cell concentration to 5x10⁵cells/ml. For the culture of these cells, DMEM or RPMI (as recommended by the ATCC) was used, with the addition of 5% FCS (Hyclone), 100µM non essential amino acids (Gibco), 1mM sodium pyruvate (Gibco), mercaptoethanol 5.5x10⁻⁵M (Gibco), 10mM Hepes (Gibco). RNA was either prepared from resting cells or cells activated with PMA at 10ng/ml and ionomycin at 1µg/ml for 6 and 14 hours. Keratinocyte line CCD106 and an airway epithelial tumor line NCI-H292 were also obtained from the ATCC. Both were cultured in DMEM 5% FCS (Hyclone), 100µM non essential amino acids (Gibco), 1mM sodium pyruvate (Gibco), mercaptoethanol 5.5x10⁻⁵M (Gibco), and 10mM Hepes (Gibco). CCD1106 cells were activated for 6 and 14 hours with approximately 5 ng/ml TNF alpha and 1ng/ml IL-1 beta, while NCI-H292 cells were activated for 6 and 14 hours with the following cytokines: 5ng/ml IL-4, 5ng/ml IL-9, 5ng/ml IL-13 and 25ng/ml IFN gamma.

For these cell lines and blood cells, RNA was prepared by lysing approximately 10⁷cells/ml using Trizol (Gibco BRL). Briefly, 1/10 volume ofbromochloropropane (Molecular Research Corporation) was added to the RNA sample, vortexed and after 10 minutes at room temperature, the tubes were spun at 14,000 rpm in a Sorvall SS34 rotor. The aqueous phase was removed and placed in a 15ml Falcon Tube. An equal volume of isopropanol was added and left at -20°C overnight. The precipitated RNA was spun down at 9,000 rpm for 15 min in a Sorvall SS34 rotor and washed in 70% ethanol. The pellet was redissolved in 300µl of RNAse-free water and 35µl buffer (Promega) 5µl DTT, 7µl RNAsin and 8µl DNAse were added. The tube was incubated at 37°C for 30 minutes to remove contaminating genomic DNA, extracted once with phenol chloroform and re-precipitated with 1/10 volume of 3M sodium acetate and 2 volumes of 100% ethanol. The RNA was spun down and placed in RNAse free water. RNA was stored at -80°C.

### AI_comprehensive panel_v1.0

The plates for AI_comprehensive panel_v1.0 include two control wells and 89 test samples comprised of cDNA isolated from surgical and postmortem human tissues obtained from the Backus Hospital and Clinomics (Frederick, MD). Total RNA was extracted from tissue samples from the Backus Hospital in the Facility at CuraGen. Total RNA from other tissues was obtained from Clinomics.

Joint tissues including synovial fluid, synovium, bone and cartilage were obtained from patients undergoing total knee or hip replacement surgery at the Backus Hospital. Tissue samples were immediately snap frozen in liquid nitrogen to ensure that isolated RNA was of optimal quality and not degraded. Additional samples of osteoarthritis and rheumatoid arthritis joint tissues were obtained from Clinomics. Normal control tissues were supplied by Clinomics and were obtained during autopsy of trauma victims.

Surgical specimens of psoriatic tissues and adjacent matched tissues were provided as total RNA by Clinomics. Two male and two female patients were selected between the ages of 25 and 47. None of the patients were taking prescription drugs at the time samples were isolated.

Surgical specimens of diseased colon from patients with ulcerative colitis and Crohns disease and adjacent matched tissues were obtained from Clinomics. Bowel tissue from three female and three male Crohn's patients between the ages of 41-69 were used. Two patients were not on prescription medication while the others were taking dexamethasone, phenobarbital, or tylenol. Ulcerative colitis tissue was from three male and four female patients. Four of the patients were taking lebvid and two were on phenobarbital.

Total RNA from post mortem lung tissue from trauma victims with no disease or with emphysema, asthma or COPD was purchased from Clinomics. Emphysema patients ranged in age from 40-70 and all were smokers, this age range was chosen to focus on patients with cigarette-linked emphysema and to avoid those patients with alpha-lanti-trypsin deficiencies. Asthma patients ranged in age from 36-75, and excluded smokers to prevent those patients that could also have COPD. COPD patients ranged in age from 35-80 and included both smokers and non-smokers. Most patients were taking corticosteroids, and bronchodilators.

In the labels employed to identify tissues in the AI_comprehensive panel_v1.0 panel, the following abbreviations are used:
AI = Autoimmunity
Syn = Synovial
Normal = No apparent disease
Rep22 /Rep20 = individual patients
RA = Rheumatoid arthritis
Backus = From Backus Hospital
OA = Osteoarthritis
(SS) (BA) (MF) = Individual patients
Adj = Adjacent tissue
Match control = adjacent tissues
-M = Male
-F = Female
COPD = Chronic obstructive pulmonary disease

### Panels 5D and 51

The plates for Panel 5D and 5I include two control wells and a variety of cDNAs isolated from human tissues and cell lines with an emphasis on metabolic diseases. Metabolic tissues were obtained from patients enrolled in the Gestational Diabetes study. Cells were obtained during different stages in the differentiation of adipocytes from human mesenchymal stem cells. Human pancreatic islets were also obtained.

In the Gestational Diabetes study subjects are young (18 - 40 years), otherwise healthy women with and without gestational diabetes undergoing routine (elective) Caesarean section. After delivery of the infant, when the surgical incisions were being repaired/closed, the obstetrician removed a small sample (<1 cc) of the exposed metabolic tissues during the closure of each surgical level. The biopsy material was rinsed in sterile saline, blotted and fast frozen within 5 minutes from the time of removal. The tissue was then flash frozen in liquid nitrogen and stored, individually, in sterile screw-top tubes and kept on dry ice for shipment to or to be picked up by CuraGen. The metabolic tissues of interest include uterine wall (smooth muscle), visceral adipose, skeletal muscle (rectus) and subcutaneous adipose. Patient descriptions are as follows:
Patient 2: Diabetic Hispanic, overweight, not on insulin
Patient 7-9: Nondiabetic Caucasian and obese (BMI>30)
Patient 10: Diabetic Hispanic, overweight, on insulin
Patient 11: Nondiabetic African American and overweight
Patient 12: Diabetic Hispanic on insulin

Adipocyte differentiation was induced in donor progenitor cells obtained from Osirus (a division of Clonetics/BioWhittaker) in triplicate, except for Donor 3U which had only two replicates. Scientists at Clonetics isolated, grew and differentiated human mesenchymal stem cells (HuMSCs) for CuraGen based on the published protocol found in Mark F. Pittenger, et al., Multilineage Potential of Adult Human Mesenchymal Stem Cells Science Apr 2 1999: 143-147. Clonetics provided Trizol lysates or frozen pellets suitable for mRNA isolation and ds cDNA production. A general description of each donor is as follows:
Donor 2 and 3 U: Mesenchymal Stem cells, Undifferentiated Adipose
Donor 2 and 3 AM: Adipose, AdiposeMidway Differentiated
Donor 2 and 3 AD: Adipose, Adipose Differentiated

Human cell lines were generally obtained from ATCC (American Type Culture Collection), NCI or the German tumor cell bank and fall into the following tissue groups: kidney proximal convoluted tubule, uterine smooth muscle cells, small intestine, liver HepG2 cancer cells, heart primary stromal cells, and adrenal cortical adenoma cells. These cells are all cultured under standard recommended conditions and RNA extracted using the standard procedures. All samples were processed at CuraGen to produce single stranded cDNA.

Panel 5I contains all samples previously described with the addition of pancreatic islets from a 58 year old female patient obtained from the Diabetes Research Institute at the University of Miami School of Medicine. Islet tissue was processed to total RNA at an outside source and delivered to CuraGen for addition to panel 5I.

In the labels employed to identify tissues in the 5D and 5I panels, the following abbreviations are used:
GO Adipose = Greater Omentum Adipose
SK = Skeletal Muscle
UT = Uterus
PL = Placenta
AD = Adipose Differentiated
AM = Adipose Midway Differentiated
U = Undifferentiated Stem Cells

### Panel CNSD.01

The plates for Panel CNSD.01 include two control wells and 94 test samples comprised of cDNA isolated from postmortem human brain tissue obtained from the Harvard Brain Tissue Resource Center. Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains two brains from each of the following diagnoses: Alzheimer's disease, Parkinson's disease, Huntington's disease, Progressive Supernuclear Palsy, Depression, and "Normal controls". Within each of these brains, the following regions are represented: cingulate gyrus, temporal pole, globus palladus, substantia nigra, Brodman Area 4 (primary motor strip), Brodman Area 7 (parietal cortex), Brodman Area 9 (prefrontal cortex), and Brodman area 17 (occipital cortex). Not all brain regions are represented in all cases; e.g., Huntington's disease is characterized in part by neurodegeneration in the globus palladus, thus this region is impossible to obtain from confirmed Huntington's cases. Likewise Parkinson's disease is characterized by degeneration of the substantia nigra making this region more difficult to obtain. Normal control brains were examined for neuropathology and found to be free of any pathology consistent with neurodegeneration.

In the labels employed to identify tissues in the CNS panel, the following abbreviations are used:
PSP = Progressive supranuclear palsy
Sub Nigra = Substantia nigra
Glob Palladus= Globus palladus
Temp Pole = Temporal pole
Cing Gyr = Cingulate gyrus
BA 4 = Brodman Area 4

### Panel CNS_Neurodegeneration_V1.0

The plates for Panel CNS_Neurodegeneration_V1.0 include two control wells and 47 test samples comprised of cDNA isolated from postmortem human brain tissue obtained from the Harvard Brain Tissue Resource Center (McLean Hospital) and the Human Brain and Spinal Fluid Resource Center (VA Greater Los Angeles Healthcare System). Brains are removed from calvaria of donors between 4 and 24 hours after death, sectioned by neuroanatomists, and frozen at -80°C in liquid nitrogen vapor. All brains are sectioned and examined by neuropathologists to confirm diagnoses with clear associated neuropathology.

Disease diagnoses are taken from patient records. The panel contains six brains from Alzheimer's disease (AD) patients, and eight brains from "Normal controls" who showed no evidence of dementia prior to death. The eight normal control brains are divided into two categories: Controls with no dementia and no Alzheimer's like pathology (Controls) and controls with no dementia but evidence of severe Alzheimer's like pathology, (specifically senile plaque load rated as level 3 on a scale of 0-3; 0 = no evidence of plaques, 3 = severe AD senile plaque load). Within each of these brains, the following regions are represented: hippocampus, temporal cortex (Brodman Area 21), parietal cortex (Brodman area 7), and occipital cortex (Brodman area 17). These regions were chosen to encompass all levels of neurodegeneration in AD. The hippocampus is a region of early and severe neuronal loss in AD; the temporal cortex is known to show neurodegeneration in AD after the hippocampus; the parietal cortex shows moderate neuronal death in the late stages of the disease; the occipital cortex is spared in AD and therefore acts as a "control" region within AD patients. Not all brain regions are represented in all cases.

In the labels employed to identify tissues in the CNS_Neurodegeneration_V1.0 panel, the following abbreviations are used:
AD = Alzheimer's disease brain; patient was demented and showed AD-like pathology upon autopsy
Control = Control brains; patient not demented, showing no neuropathology
Control (Path) = Control brains; pateint not demented but showing sever AD-like pathology
SupTemporal Ctx =Superior Temporal Cortex
Inf Temporal Ctx =Inferior Temporal Cortex

### NOV1

Expression of NOV1 (CG57602-01) was assessed using the primer-probe set Ag3294, described in Table AA. Results of the RTQ-PCR runs are shown in Tables AB, AC and AD.

**Table AA. Probe Name Ag3294**

| **Primers** | **Sequences** | **length** | **Start Position** | **SEQ ID NO** |
|---|---|---|---|---|
| Forward | 5'-ccttttgtgggttccatagtc-3' | 21 | 6686 | 243 |
| Probe | TET-5'-tttgtccccaagggccttccagt-3'-TAMRA | 23 | 6707 | 244 |
| Reverse | 5'-aaaagcaggtattcaacaagca-3' | 22 | 6758 | 245 |

**Table AB. CNS_neurodegeneration_v1.0**

| **Tissue Name** | **Rel. Exp.(%) Ag3294, Run 210062302** | **Tissue Name** | **Rel. Exp.(%) Ag3294, Run 210062302** |
|---|---|---|---|
| AD 1 Hippo | 0.0 | Control (Path) 3 Temporal Ctx | 0.0 |
| AD 2 Hippo | 0.0 | Control (Path) 4 Temporal Ctx | 0.0 |
| AD 3 Hippo | 0.0 | AD 1 Occipital Ctx | 0.0 |
| AD 4 Hippo | 0.0 | AD 2 Occipital Ctx (Missing) | 0.0 |
| AD 5 Hippo | 0.0 | AD 3 Occipital Ctx | 0.0 |
| AD 6 Hippo | 0.1 | AD 4 Occipital Ctx | 100.0 |
| Control 2 Hippo | 0.0 | AD 5 Occipital Ctx | 0.0 |
| Control 4 Hippo | 0.0 | AD 6 Occipital Ctx | 0.0 |
| Control (Path) 3 Hippo | 0.0 | Control 1 Occipital Ctx | 0.0 |
| AD 1Temporal Ctx | 0.0 | Control 2 Occipital Ctx | 0.0 |
| AD 2 Temporal Ctx | 0.0 | Control 3 Occipital Ctx | 0.0 |
| AD 3 Temporal Ctx | 0.0 | Control 4 Occipital Ctx | 0.0 |
| AD 4 Temporal Ctx | 0.0 | Control (Path) 1 Occipital Ctx | 0.1 |
| AD 5 Inf Temporal Ctx | 0.1 | Control (Path) 2 Occipital Ctx | 0.0 |
| AD 5 Sup Temporal Ctx | 0.1 | Control (Path) 3 Occipital Ctx | 0.0 |
| AD 6 Inf Temporal Ctx | 0.1 | Control (path) 4 Occipital Ctx | 0.0 |
| AD 6 Sup Temporal Ctx | 0.1 | Control 1 Parietal Ctx | |
| Control 1 Temporal Ctx | 0.0 | Control 2 Parietal Ctx | 0.1 |
| Control 2 Temporal Ctx | 0.0 | Control 3 Parietal Ctx | 0.0 |
| Control 3 Temporal Ctx | 0.0 | Control (Path) 1 Parietal Ctx | 0.0 |
| Control 3 Temporal Ctx | 0.0 | Control (Path)2 Parietal Ctx | 0.0 |
| Control (Path) 1 Temporal | 0.1 | Control (Path) 3 Parietal | 0.0 |
| Ctx | | Ctx | |
| Control (Path) 2 Temporal Ctx | 0.0 | Control (Path) 4 Parietal Ctx | 0.0 |

**Table AC. General_screening_panel_v1.4**

| **Tissue Name** | **Rel.Exp.(%)Ag3294,Run 215669619** | **Tissue Name** | **Rel.Exp.(%)Ag3294,Run 215669619** |
|---|---|---|---|
| Adipose | 0.0 | Renal ca. TK-10 | 0.0 |
| Melanoma* Hs688(A).T | 0.0 | Bladder | 4.9 |
| Melanoma* Hs688(B).T | 0.9 | Gastric ca. (liver met.) NCI-N87 | 11.1 |
| Melanoma* M14 | 0.0 | Gastric ca. KATO III | 0.0 |
| Melanoma* LOXIMVI | 0.0 | Colon ca. SW-948 | 0.0 |
| Melanoma* SK-MEL-5 | 1.4 | Colon ca. SW480 | 7.1 |
| Squamous cell carcinoma SCC-4 | 0.0 | Colon ca.* (SW480 met) SW620 | 1.2 |
| Testis Pool | 20.0 | Colon ca. HT29 | 3.2 |
| Prostate ca.* (bone met) PC-3 | 5.1 | Colon ca.HCT-116 | 2.3 |
| Prostate Pool | 3.6 | Colon.ca CaCo-2 | 0.7 |
| Placenta | 2.6 | Colon cancer tissue | 0.0 |
| Uterus Pool | 0.0 | Colon ca. SW1116 | 0.5 |
| Ovarian ca. OVCAR-3 | 17.9 | Colon ca. Colo-205 | 0.0 |
| Ovarian ca. SK-OV-3 | 1.4 | Colon ca. SW-48 | 0.0 |
| Ovarian ca. OVCAR-4 | 0.0 | Colon Pool | 6.0 |
| Ovarian ca. OVCAR-5 | 23.7 | Small Intestine Pool | 2.5 |
| Ovarian ca. IGROV-1 | 6.2 | Stomach Pool | 2.6 |
| Ovarian ca. OVCAR-8 | 1.4 | Bone Marrow Pool | 0.0 |
| Ovary | 1.3 | Fetal Heart | 0.0 |
| Breast ca. MCF-7 | 8.0 | Heart Pool | 2.6 |
| Breast ca. MDA-MB-231 | 4.1 | Lymph Node Pool | 3.6 |
| Breast ca. BT 549 | 4.0 | Fetal Skeletal Muscle | 1.1 |
| Breast ca.T47D | 38.2 | Skeletal Muscle Pool | 2.0 |
| Breast ca. MDA-N | 1.3 | Spleen Pool | 0.9 |
| Breast Pool | 10.5 | Thymus Pool | 10.2 |
| Trachea | 7.2 | CNS cancer (gHo/astro) U87-MG | 0.0 |
| Lung | 0.0 | CNS cancer (glio/astro) U-118-MG | 1.1 |
| Fetal Lung | 54.3 | CNS cancer (neuro;met) SK-N-AS | 100.0 |
| Lung ca.NCI.N417 | 0.0 | CNS cancer (astro) SF-539 | 0.0 |
| Lung ca. LX-1 | 2.6 | CNS cancer (astro) SNB-75 | 0.0 |
| Lungea.NCI-H146 | 0.7 | CNS cancer (glio) SNB-19 | 2.4 |
| Lung ca. SHP-77 | 0.0 | CNS cancer (glio) SF-295 | 33.9 |
| Lung ca. A549 | 13.3 | Brain (Amygdala) Pool | 8.4 |
| Lungea.NCI-H526 | 2.4 | Brain (cerebellum) | 23.8 |
| Lung ca. NCI-H23 | 71.7 | Brain (fetal) | 5.1 |
| Lung ca. NCI-H460 | 10.0 | Brain (Hippocampus) Pool | 26.2 |
| Lung ca. HOP-62 | 1.4 | Cerebral Cortex Pool | 13.7 |
| Lung ca. NCI-H522 | 34.6 | Brain (Substantia nigra) Pool | 20.7 |
| Liver | 3.1 | Brain (Thalamus) Pool | 26.1 |
| Fetal Liver | 2.5 | Brain (whole) | 15.8 |
| Liver ca. HepG2 | 0.0 | Spinal Cord Pool | 15.4 |
| Kidney Pool | 6.1 | Adrenal Gland | 5.6 |
| Fetal Kidney | 18.7 | Pituitary gland Pool | 2.4 |
| Renal ca. 786-0 | 1.3 | Salivary Gland | 0.0 |
| Renal ca. A498 | 7.3 | Thyroid (female) | 0.0 |
| Renal ca. ACHN | 2.9 | Pancreatic ca. CAPAN2 | 5.5 |
| Renal ca. UO-31 | 0.0 | Pancreas Pool | 5.3 |

**Table AD. Panel 4D**

| **Tissue Name** | **Rel. Exp.(%) Ag3294, Run 165014460** | **Tissue Name** | **Rel. Exp.(%) Ag3294, Run 165014460** |
|---|---|---|---|
| Secondary Th1 act | 4.5 | HUVEC IL-1beta | 0.0 |
| Secondary Th2 act | 0.0 | HUVEC IFN gamma | 3.1 |
| Secondary Tr1 act | 3.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 4.3 | Lung Microvascular EC none | 0.0 |
| Primary Th1 act | 0.0 | Lung Microvascular EC TNFalpha +IL-1beta +IL-1beta | 0.0 |
| Primary Th2 act | 0.0 | Microvascular Dermal EC none | 3.6 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 0.0 |
| Primary Th2 rest | 4.6 | Small airway epithelium none | 0.0 |
| Primary Tr1 rest | 0.0 | Small airway epithelium TNFalpha +IL-1beta | 0.0 |
| CD45RA CD4 lymphocyte act | 0.0 | Coronery artery SMC rest | 0.0 |
| CD45RO CD4 lymphocyte act | 0.0 | Coronery artery SMC TNFalpha+ IL-1beta | 3.5 |
| CD8 lymphocyte act | 0.0 | Astrocytes rest | 4.5 |
| Secondary CD8 lymphocyte rest | 0.0 | Astrocytes TNFalpha + IL-1beta | 0.0 |
| Secondary CD8 lymphocyte act act | 0.0 | KU-812 (Basophil) rest | 10.2 |
| CD4 lymphocyte none | 3.7 | KU-812 (Basophil) PMA/ionomycin | 0.0 |
| 2ry Th1/Th2/Tr1_anti-CD95 CH11 | 0.0 | CCD1106 (Keratinocytes) none | 0.0 |
| LAK cells rest | 0.0 | CCD1106 (Keratinocytes) TNFalpha + IL-1beta | 0.0 |
| LAK cells IL-2 | 0.0 | Liver cirrhosis | 26.2 |
| LAK cells IL-2+IL-12 | 0.0 | Lupus kidney | 0.0 |
| LAK cells IL-2+IFN gamma | 0.0 | NCI-H292 none | 6.5 |
| LAK cells IL-2+ IL-18 | 0.0 | NCI-H292 IL-4 | 12.6 |
| LAK cells PMA/ionomycin | 0.0 | NCI-H292 IL-9 | 3.8 |
| NK Cells IL-2 rest | 0.0 | NCI-H292 IL-13 | 12.2 |
| Two Way MLR 3 day | 12.9 | NCI-H292 IFN gamma | 3.8 |
| Two Way MLR 5 day | 0.0 | HPAEC none | 0.0 |
| Two Way MLR 7 day | 6.7 | HPAEC TNF alpha + IL-1 beta | 0.0 |
| PBMC rest | 0.0 | Lung fibroblast none | 2.4 |
| PBMC PWM | 0.0 | Lung fibroblast TNF alpha+ IL-1 beta | 7.2 |
| PBMC PHA-L | 0.0 | Lung fibroblast IL-4 | 8.9 |
| Ramos (B cell) none | 0.0 | Lung fibroblast.IL-9 | 0.0 |
| Ramos(B cell)ionomycin | 7.6 | Lung fibroblast IL-13 | 0.0 |
| B lymphocytes PWM | 0.0 | Lung fibroblast IFN gamma | 0.0 |
| B lymphocytes CD40L and IL-4 | 7.3 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 6.3 |
| Monocytes rest | 4.2 | IBD Crohn's | 0.0 |
| Monocytes LPS | 0.0 | Colon | 100.0 |
| Macrophages rest | 2.1 | Lung | 43.2 |
| Macrophages LPS | 0.0 | Thymus | 7.6 |
| HUVEC none | 2.3 | Kidney | 3.4 |
| HUVEC starved | 3.3 | | |

**CNS_neurodegeneration_v1.0 Summary:** Ag3294 Results from one experiment with the CG57602-01 gene are not included. The amp plot indicates that there were experimental difficulties with this run.

**General_screening_panel_vl.4 Summary:** Ag3294 Expression of the CG57602-01 gene is highest in a CNS cancer cell line (CT = 31.8). Therefore, expression of this gene may be used to distinguish this sample from the other samples on this panel. Interestingly, expression of this gene is higher in fetal lung (CT = 32.7) than in adult lung (CT = 40), suggesting that expression of this gene may be used to distinguish adult and fetal lung. Additionally, this gene is expressed at a moderate level in two lung cancer cell lines suggesting a possible role in lung cancer.

In addition, this gene is expressed at low levels in several regions of the central nervous system, including cerebellum, hippocampus, cerebral cortex, substantia nigra, thalamus and spinal cord (CTs = 33.7-35). Therefore, therapeutic modulation of the activity of this gene may be of benefit in the treatment of CNS disorders such as Alzheimer's disease, Parkinson's disease, epilepsy, multiple sclerosis, schizophrenia and depression.

**Panel 4D Summary:** Ag3294 Expression of the CG57602-01 gene is highest in colon (CT = 33.3), with low but significant expression also detected in lung (CT = 34.5). Therefore, expression of this gene may be used to distinguish colon and lung from the other tissues on this panel. Furthermore, expression of this gene is decreased in colon samples from patients with IBD colitis and Crohn's disease relative to normal colon. Therefore, therapeutic modulation of the activity of the protein encoded by this gene, using small molecule drugs, antibodies, or protein therapeutics, may be useful in the treatment of inflammatory bowel disease.

### NOV2

Expression of NOV2 (CG57558-01) was assessed using the primer-probe set Ag3285, described in Table BA.

**Table CA. Probe Name Ag3286**

| **Primers** | **Sequences** | **Length** | **Start Position** | **SEQ ID NO** |
|---|---|---|---|---|
| Forward | 5'-gacaacaaacaagcaaacacaa-3' | 22 | 11 | 246 |
| Probe | TET-5'-accttccacttccttctggtcctgct-3'-TAMRA | 26 | 62 | 257 |
| Reverse | 5'-gcaggagaggaggaagaagag-3' | 221 | 89 | 258 |

**CNS_neurodegeneration_v1.0 Summary:** Ag3285 Expression of the CG57558-01 gene is low/undetectable (CTs > 35) across all of the samples on this panel (data not shown).

**Panel 1.3D Summary:** Ag3285 Expression of the CG57558-01 gene is low/undetectable (CTs > 35) across all of the samples on this panel (data not shown).

**Panel 4D Summary:** Ag3285 Expression of the CG57558-01 gene is low/undetectable (CTs > 35) across all of the samples on this panel (data not shown).

### NOV3

Expression of NOV3 (CG57560-01) was assessed using the primer-probe sets Ag3286 and Ag663, described in Tables CA and CB. Results of the RTQ-PCR runs are shown in Tables CC, CD, CE and CF.

**Table CA. Probe Name Ag3286**

| **Primers** | **Sequences** | **Length** | **Start Position** | **SEQ ID NO** |
|---|---|---|---|---|
| Forward | 5'-gaggaggtggaagaagatgatt-3' | 22 | 764 | 249 |
| Probe | TET-5'-agaatctcttccgcaagatcctggct-3'-TAMRA | 26 | 801 | 250 |
| Reverse | 5'-cateccaatatggagagtcaaa-3' | 22 | 839 | 251 |

**Table CB. Probe Name Ag663**

| **Primers** | **Sequences** | **Length** | **Start Position** | **SEQ ID NO** |
|---|---|---|---|---|
| Forward | 5'-acctttctatgaggaggtggaa-3' | 22 | 754 | 252 |
| Probe | TET-5'-tgagaaccatgataagaatctcttccg-3'-TAMRA | 27 | 787 | 253 |
| Reverse | 5'-gtcaccagccaggatcttg-3' | 19 | 814 | 254 |

**Table CC. CNS_neurodegeneration_v1.0**

| **Tissue Name** | **Rel. Exp.(%) Ag3286, Run 210060841** | **Tissue Name** | **Rel. Exp.(%) Ag3286, Run 210060841** |
|---|---|---|---|
| AD 1 Hippo | 12.2 | Control (Path) Temporal Ctx | 1.3 |
| AD 2 Hippo | 41.8 | Control (Path) 4 Temporal Ctx | 27.7 |
| AD 3 Hippo | 6.3 | AD 1 Occipital Ctx | 9.0 |
| AD 4 Hippo | 5.7 | AD 2 Occipital Ctx (Missing) | 0.0 |
| AD 5 hippo | 61.1 | AD 3 Occipital Ctx | 0.8 |
| AD 6 Hippo | 58.6 | AD 4 Occipital Ctx | 14.9 |
| Control 2 Hippo | 68.3 | AD 5 Occipital Ctx | 11.6 |
| Control 4 Hippo | 2.6 | AD 6 Occipital Ctx | 60.7 |
| Control (Path) 3 Hippo | 1.2 | Control 1 Occipital Ctx | 0.2 |
| AD 1 Temporal Ctx | 7.5 | Control 2 Occipital Ctx | 88.3 |
| AD 2 Temporal Ctx | 39.0 | Control 3 Occipital Ctx | 8.2 |
| AD 3 Temporal Ctx | 1.9 | Control 4 Occipital Ctx | 0.8 |
| AD 4 Temporal Ctx | 17.6 | Control (Path) 1 Occipital Ctx | 75.3 |
| AD 5 Inf Temporal Ctx | 88.9 | Control (Path) 2 Occipital Ctx | 4.4 |
| AD 5 SupTemporal Ctx | 34.9 | Control (Path) 3 Occipital Ctx | 0.2 |
| AD 6 Inf Temporal Ctx | 37.6 | Control (Path) 4 Occipital Ctx | 6.7 |
| AD 6 Sup Temporal Ctx | 47.3 | Control 1 Parietal CtX | 1.4 |
| Control Temporal Ctx | 1.2 | Control 2 Parietal Ctx | 15.2 |
| Control 2 Temporal Ctx | 70.7 | Control 3 Parietal Ctx | 11.3 |
| Control 3 Temporal Ctx | 14.3 | Control (Path) Parietal Ctx | 1 100.0 |
| Control 4 Temporal Ctx | 3.5 | Control (Path) 2 Parietal Ctx | 15.4 |
| Control (path) 1 Temporal Ctx | 87.7 | Control (Path) 3 Parietal Ctx | 0.7 |
| Control (Path) 2 Temporal Ctx | 39.2 | Control (Path) 4 Parietal Ctx | 33.9 |

**Table CD. General_screening_panel_v1.4**

| **Tissue Name** | **Rel. Exp.(%) Ag3286, Run 216512997** | **Tissue Name** | **Rel. Exp.(%) Ag3286, Run 216512997** |
|---|---|---|---|
| Adipose | 0.0 | Renal ca. TK-10 | 0.1 |
| Melanoma* Hs688(A).T | 0.0 | Bladder | 0.0 |
| Melanoma* Hs688(B).T | 0.0 | Gastric ca. (liver met.) NCI-N87 | 0.1 |
| Melanoma^{*} M14 | 0.0 | Gastric ca. KATO III | 0.0 |
| Melanoma* LOXIMVI | 0.0 | Colon ca. SW-948 | 0.0 |
| Melanoma* SK-MEL-5 | 0.3 | Colon ca. SW480 | 0.6 |
| Squamous cell carcinoma SCC-4 | 0.0 | Colon ca.* (SW480 met) SW620 | 0.9 |
| Testis Pool | 0.2 | Colon ca.HT29 | 0.0 |
| Prostate ca.* (bone met) PC-3 | 0.0 | Colon ca. HCT-116 | 0.0 |
| Prostate Pool | 0.0 | Colon ca. CaCo-2 | 3.1 |
| Placenta | 0.0 | Colon cancer tissue | 0.0 |
| Uterus Pool | 0.0 | Colon ca. SW1116 | 0.0 |
| Ovarian ca. OVCAR-3 | 0.1 | Colon ca. Colo-205 | 0.1 |
| Ovarian ca. SK-OV-3 | 0.0 | Colon ca. SW-48 | 0.0 |
| Ovarian ca. OVCAR-4 | 0.0 | Colon Pool | 0.1 |
| Ovarian ca. OVCAR-5 | 0.1 | Small Intestine Pool | 0.0 |
| Ovarian ca. IGROV-1 | 0.1 | Stomach Pool | 0.0 |
| Ovarian ca. OVCAR-8 | 0.0 | Bone Marrow Pool | 0.0 |
| Ovary | 0.0 | Fetal Heart | 0.0 |
| Breast ca. MCF-7 | 0.0 | Heart Pool | 0.0 |
| Breast ca. MDA-MB-231 | 0.0 | Lymph Node Pool | 0.0 |
| Breast ca. BT 549 | 0.1 | Fetal Skeletal Muscle | 0.0 |
| Breast ca. T47D | 0.1 | Skeletal Muscle Pool | 0.0 |
| Breast ca. MDA-N | 0.0 | Spleen Pool | 0.0 |
| Breast Pool | 0.0 | Thymus Pool | 0.0 |
| Trachea | 0.1 | CNS cancer (glio/astro) U87-MG | 0.0 |
| Lung | 0.0 | CNS cancer (glio/astro) U-118-MG | 0.1 |
| Fetal Lung | 0.0 | CNS cancer (neuro;met) SK-N-AS | 2.3 |
| Lung ca. NCI-N417 | 7.2 | CNS cancer (astro) SF-539 | 100.0 |
| Lung ca. LX-1 | 0.1 | CNS cancer (astro) SNB-75 cancer | 0.2 |
| Lungca.NCI-H146 | 1.9 | CNS cancer (glio) SNB-19 | 0.0 |
| Lung ca. SHP-77 | 0.5 | CNS cancer (glio) SF-295 | 0.0 |
| Lung ca. A549 | 0.1 | Brain (Amygdala) Pool | 10.4 |
| Lung ca.NCI-H526 | 0.6 | Brain (cerebellum) | 1.2 |
| Lung ca. NCI-H23 | 0.3 | Brain (fetal) | 35.6 |
| Lung ca. NCI-H460 | 0.3 | Brain (Hippocampus) Pool | 20.7 |
| Lung ca. HOP-62 | 0.0 | Cerebral Cortex Pool | 20.4 |
| Lung ca.NCI-H522 | 0.1 | Brain (Substantia nigra) Pool | 13.4 |
| Liver | 0.0 | Brain (Thalamus) Pool | 22.2 |
| Fetal Liver | 0.0 | Brain (whole) | 19.1 |
| Liver ca. HepG2 | 0.0 | Spinal Cord Pool | 1.5 |
| Kidney Pool | 0.0 | Adrenal Gland | 0.0 |
| Fetal Kidney | 0.0 | Pituitary gland Pool | 1.0 |
| Renal ca. 786-0 | 0.0 | Salivary Gland | 0.0 |
| Renal ca. A498 | 0.0 | Thyroid (female) | 0.0 |
| Renal ca. ACHN | 0.0 | Pancreatic ca. CAPAN2 | 0.0 |
| Renal ca. UO-31 | 0.1 | Pancreas Pool | 0.0 |

**Table CE. Panel 1.1**

| **Tissue Name** | **Rel. Exp.(%) Ag663, Run 109559269** | **Tissue Name** | **Rel. Exp.(%) Ag663, Run 109559269** |
|---|---|---|---|
| Adrenal gland | 0.0 | Renal ca. UO-31 | 0.0 |
| Bladder | 0.0 | Renal ca. RXF 393 | 0.0 |
| Brain (amygdala) | 23.2 | Liver | 0.0 |
| Brain (cerebellum) | 1.8 | Liver (fetal) | 0.0 |
| Brain (hippocampus) | 82.9 | Liver ca. (hepatoblast) HepG2 | 0.0 |
| Brain (substantia nigra) | 15.6 | Lung | 0.0 |
| Brain (thalamus) | 32.8 | Lung (fetal) | 0.0 |
| Cerebral Cortex | 90.8 | Lung ca. (non-s.cell) HOP-62 | 0.0 |
| Brain (fetal) Brain (fetal) | 100.0 | Lung ca. (large cell)NCI-H460 | 0.0 |
| Brain (whole) | 41.8 | Lung ca. (non-s.cell) NCI-H23 | 0.0 |
| glio/astro U-118-MG | 0.0 | Lung ca. (non-s.cl) NCI-H522 | 0.0 |
| astrocytomaSF-539 | 0.0 | Lung ca. (non-sm. cell) A549 | 0.0 |
| astrocytoma SNB-75 | 0.0 | Lung ca. (s.cell var.) SHP-77 | 0.0 |
| astrocytoma SW1783 | 0.0 | Lung ca.(small,cell) LX-1 | 0.0 |
| glioma U251 | 0.0 | Lung ca. (small cell) NCI-H69 | 4.3 |
| gliomaSF-295 | 0.0 | Lung ca. (squam.) SW 900 | 0.0 |
| glioma SNB-19 | 0.0 | Lung ca. (squam.) NCI-H596 | 16.6 |
| glio/astro U87-MG | 0.0 | Lymph node | 0.0 |
| neuro*; met SK-N-AS | 3.8 | Spleen | 0.0 |
| Mammary gland | 0.0 | Thymus | 0.0 |
| Breast ca. BT-549 | 0.0 | Ovary | 0.0 |
| Breast ca. MDA-N | 0.0 | Ovarian ca. IGROV-1 | 0.0 |
| Breast ca.* (pl.ef) T47D | 0.0 | Ovarian ca. OVCAR-3 | 0.0 |
| Breast ca.* (pl.ef) MCF-7 | 0.0 | Ovarian ca. OVCAR-4 | 0.0 |
| Breast ca.* . (pl.ef) MDA-MB-231 | 0.0 | Ovarian ca. OVCAR-5 | 0.0 |
| Small intestine | 0.0 | Ovarian ca. OVCAR-8 | 0.0 |
| Colorectal | 0.0 | Ovarian ca.* (ascites) SK-OV-3 | 0.0 |
| Colon ca. HT29 | 0.0 | Pancreas | 0.0 |
| Colon ca. CaCo-2 | 2.9 | Pancreatic ca. CAP AN 2 CAPAN 2 | 0.0 |
| Colon ca. HCT-15 | 0.0 | Pituitary gland | 5.7 |
| Colon ca.HCT-116 | 0.0 | Placenta | 0.0 |
| Colon ca. HCC-2998 | 0.0 | Prostate | 0.0 |
| Colon ca. SW480 | 0.0 | Prostate ca.* (bone 0.0 met) PC-3 | |
| Colon ca.* SW620 (SW480 met) | 0.0 | Salivary gland | 0.0 |
| Stomach | 0.0 | Trachea | 0.0 |
| Gastric ca. (liver met) NCI-N87 | 0.0 | Spinal cord | 0.3 |
| Heart | 0.0 | Testis | 0.0 |
| Skeletal muscle (Fetal) | 0.0 | Thyroid | 0.0 |
| Skeletal muscle | 0.0 | Uterus | 0.0 |
| Endothelial cells | 0.0 | Melanoma M14 | 0.0 |
| Heart (Fetal) | 0.0 | Melanoma LOX IMVI | 0.0 |
| Kidney | 0.0 | Melanoma UACC-62 | 0.0 |
| Kidney (fetal) | 0.0 | Melanoma SK-MEL-28 | 0.0 |
| Renal ca. 786-0 | 0.0 | Melanoma* (met) SK-MEL-5 | 0.0 |
| Renal ca. A498 | 0.0 | Melanoma Hs688 (A).T | 0.0 |
| Renal ca. ACHN | 0.0 | Melanoma* (met) Hs688(B).T | 0.0 |
| Renal ca. TK-10 | 0.0 | | |

**Table CF. Panel 4D**

| **Tissue Name** | **Rel. Exp.(%) Ag3286, Run 164633940** | **Tissue Name** | **Rel.Exp.(%) Ag3286,Run 164633940** |
|---|---|---|---|
| Secondary Th1 act | 5.5 | HUVEC IL-1beta | 0.0 |
| Secondary Th2 act | 4.2 | HUVEC IFN gamma | 0.0 |
| Secondary Tr1 act | 15.3 | HUVEC TNF alpha+IFN gamma | 0.0 |
| Secondary Th1 rest | 0.0 | HUVEC TNF alpha + ILA | 0.0 |
| Secondary Th2 rest | 0.0 | HUVECIL-11 | 0.0 |
| Secondary Tr1 rest | 0.0 | Lung Microvascular EC none | 0.0 |
| Primary Th1 act | 33.2 | Lung Microvascular EC TNFalpha + IL-1beta | 0.0 |
| Primary Th2 act | 24.7 | Microvascular Dermal EC none | 0.0 |
| Primary Tr1 act | 16.2 | Microsvasular Dermal EC TNFalpha + IL-1beta | 0.0 |
| Primary Th1 rest | 0.0 | Bronchial epithelium TNFalpha + ILlbeta | 5.1 |
| Primary Th2 rest | 0.0 | Small airway epithelium none | 0.0 |
| Primary Tr1 rest | 4.8 | Small airway epithelium TNFalpha + IL-1beta | 0.0 |
| CD45RA CD4 lymphocyte act | 0.0 | Coronery artery SMC rest | 0.0 |
| CD45RO CD4 lymphocyte act | 27.0 | Coronery artery SMCI TNFalpha+IL-1beta | 0.0 |
| CD8 lymphocyte act | 10.4 | Astrocytes rest | 3.5 |
| Secondary CD8 lymphocyte rest | 18.6 | Astrocytes TNFalpha + IL-1beta | 0.0 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1_anti-CD95CH11 | 0.0 | CCD1106 (Keratinocytes) none | 0.0 |
| LAK cells rest | 7.9 | CCD1106 (Keratinocytes) TNFalpha+ IL-lbeta | 0.0 |
| LAK cells IL-2 | 0.0 | Liver cirrhosis | 17.6 |
| LAK cells IL-2+IL-12 | 0.0 | Lupus kidney | 0.0 |
| LAK cells IL-2+IFN gamma | 7.6 | NCI-H292 none | 0.0 |
| LAK cells IL-2 + IL-18 | 26.8 | NCI-H292 IL-4 | 0.0 |
| LAK cells PMA/ionomycin | 0.0 | NCI-H292 IL-9 | 0.0 |
| NK Cells IL-2 rest | 0.0 | NCI-H292 IL-13 | 0.0 |
| Two Way MLR 3 day | 0.0 | NCI-H292 IFN gamma | 0.0 |
| Two Way MLR 5 day | 4.4 | HPAEC none | 0.0 |
| Two Way MLR 7 day | 0.0 | HPAEC TNF alpha + IL-1 beta | 0.0 |
| PBMC rest | 0.0 | Lung fibroblast none | 0.0 |
| PBMC PWM | 11.3 | Lung fibroblast TNF alpha + IL-1 beta | 0.0 |
| PBMC PHA-L | 14.3 | Lung fibroblast IL-4 | 0.0 |
| Ramos (B cell) none | 0.0 | Lung fibroblast IL-9 | 2.8 |
| Ramos (B cell) ionomycin | 0.0 | Lung fibroblast IL-13 | 0.0 |
| B lymphocytes PWM | 100.0 | Lung fibroblast IFN gamma | 0.0 |
| B lymphocytes CD40L and 1L-4 | 8.7 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 0.0 |
| Monocytesrest | 0.0 | IBD Crohn's | 6.7 |
| Monocytes LPS | 0.0 | Colon | 49.3 |
| Macrophages rest | 1.0 | Lung | 17.9 |
| Macrophages LPS | 6.2 | Thymus | 0.0 |
| HUVEC none | 0.0 | Kidney | 1.5 |
| HUVEC starved | 0.0 | | |

**CNS_neurodegeneration_v1.0 Summary:** Ag3286 This panel confirms the expression of this gene at low levels in the brains of an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders. **General_screening_panel_v1.4 Summary:** Ag3286 The CG57560-01 gene encodes a protein with homology to calmodulin-binding protein kinases. Expression of this gene is highest in the CNS cancer cell line SF-539 (CT = 23.9). Strikingly, this gene is also expressed at moderate to high levels almost exclusively in the CNS; expression is detected amygdala, cerebellum, thalamus, hippocampus, cerebral cortex, substantia nigra and spinal cord. Thus, expression of this gene may be used to distinguish brain from the other samples pn this panel. Furthermore, this brain-specific expression pattern suggests that this gene may play a role in central nervous system disorders such as Alzheimer's disease, Parkinson's disease, epilepsy, multiple sclerosis, schizophrenia and depression.
In addition, moderate expression of this gene is seen in the pituitary gland (CT = 30.6). Therefore, this gene may play a role in endocrine disorders, diabetes, obesity, and growth disorders.
Furthermore, this gene is expressed in most of the lung cancer cell lines on this panel but at undetectable levels in the normal adult lung or fetal tissues. Hence, expression of the CG57560-01 gene might be used as a diagnostic marker for lung cancer. In addition, therapeutic modulation of the activity of this gene, using small molecule drugs, antibodies or protein therapeutics, may be of benefit in the treatment of lung cancer.
**Panel 1.1 Summary:** Ag663 The CG57560-01 gene is expressed at high levels in all of the CNS samples examined including amygdala, cerebellum, thalamus, hippocampus, cerebral cortex, spinal cord, and the substantia nigra. Highest expression of this gene is seen in the fetal brain sample (CT = 22.1). These results are consistent with what is observed in General_screening_panel_v1.4; please see this panel for a discussion of the relevance of this gene in the central nervous system.
**Panel 4D Summary:** Ag3286 Expression of the CG57560-01 gene is highest in pokeweed mitogen stimulated peripheral blood mononuclear cells (CT = 32.7) Expression of this transcript in B cells suggests that this gene may be involved in rheumatic disease including rheumatoid arthritis, lupus, osteoarthritis, and hyperproliferative B cell disorders.
In addition, low but significant expression of this gene is seen in activated T cells and memory T cells, but not resting T cells, suggesting that CG57560-01 gene expression may play a role in T cell mediated diseases such as autoimmunity or delayed type hypersensitivity reactions.

### NOV4

Expression of NOV4a (CG57547-01) and NOV4b CG57547-02 was assessed using the primer-probe sets Ag3690, Ag531, Ag534 and Ag535, described in Tables DA, DB, DC and DD. Results of the RTQ-PCR runs are shown in Tables DE, DF, DG, DH, DI, DJ and DK. Please note that the CG57547-02 variant is not recognized by primer-probe set Ag3690.

**Table DA. Probe Name Ag3690**

| **Primers** | **Sequences** | **Length** | **Start Position** | **SEQ ID NO** |
|---|---|---|---|---|
| Forward | 5'-agacgtcaaacagggaaatctt-3' | 22 | 1744 | 255 |
| Probe | TET-5'-cctccaggatataagatcactctgattga-3'-TAMRA | 29 | 1766 | 256 |
| Reverse | 5'-tctgtaggttcctcccatgag-3' | 21 | 1817 | 257 |

**Table DB. Probe Name Ag531**

| **Primers** | **Sequences** | **Length** | **Start Position** | **SEQ ID NO** |
|---|---|---|---|---|
| Forwaid | 5'-tccaggatataagatcactctgattgata-3' | 29 | 1768 | 258 |
| Probe | TET-5'-aggttcctcccatgagatattcaataacaagtcct-3'-TAMRA | 35 | 1798 | 259 |
| Reverse | 5'-aacgtttcctagtataggtgcatctg-3' | 26 | 1834 | 260 |

**Table DC. Probe Name Ag534**

| **Primers** | **Sequences** | **Length** | **Start Position** | **SEQ ID NO** |
|---|---|---|---|---|
| Forward | 5'-tcagtgatacaattgccataatttcttt-3' | 28 | 3039 | 261 |
| Probe | TET-5'-tttgctccaaatcttagtccaaatccaatgaa-3'-TAMRA | 32 | 3068 | 262 |
| Reverse | 5'-aaaaacatgattatcatatgcatttgc-3' | 27 | 3110 | 263 |

**Table DD. Probe Name Ag535**

| Primers | Sequences | Length | Start Position | SEQ ID NO |
|---|---|---|---|---|
| Forward | 5'-tttacaagtgaaggcaatttceaa-3' | 24 | 3562 | 264 |
| Probe | TET-5'-agccataataaaatgataacgctggtacttrlratucaat-3'-TAMRA, | 39 | 3587 | 265 |
| Reverse | 5'-gaggcagaactggtttctcatga-3' | 23 | 3628 | 266 |

**Table DE. CNS_neurodegeneration_v1.0**

| **Tissue Name** | **Rel. Exp.(%) Ag3690, Run 211144703** | **Tissue Name** | **Rel. Exp.(%).Ag3690, Run 211144703** |
|---|---|---|---|
| AD 1 Hippo | 17.9 | Control (Path) 3 Temporal Ctx | 4.3 |
| AD 2 Hippo | 20.0 | Control (Path) 4 Temporal Ctx | 39.5 |
| AD 3 Hippo | 17.2 | AD 1 Occipital Ctx | 38.2 |
| AD 4 Hippo | 6.1 | AD 2 Occipital Ctx (Missing) | 0.0 |
| AD 5 hippo | 97.3 | AD 3 Occipital Ctx | 11.8 |
| AD 6 Hippo | 62.4 | AD 4 Occipital Ctx | 28.5 |
| Control 2 Hippo | 23.2 | AD 5 Occipital Ctx | 25.0 |
| Control 4 Hippo | 12.4 | AD 6 Occipital Ctx | 45.4 |
| Control (Path) 3 Hippo | 22.4 | Control 1 Occipital Ctx . | 5.1 |
| AD 1 Temporal Ctx | 34.2 | Control 2 Occipital Ctx | 51.1 |
| AD 2 Temporal Ctx | 37.1 | Control 3 Occipital Ctx | 24.8 |
| AD 3 Temporal Ctx | 12.2 | Control 4 Occipital Ctx | 11.7 |
| AD 4 Temporal Ctx | 27.0 | Control (path) 1 Occipital Ctx | 100.0 |
| AD 5 InfT ral Ctx | 85.3 | Control (path) 2 Occipital Ctx | 22.5 |
| AD 5 SupTemporal Ctx | 87.7 | Control (Path) 3 Occipital | 2.8 |
| AD 6 Inf Temporal Ctx | 80.7 | Control (Path) 4 Occipital Ctx | 29.7 |
| AD 6 SupTemporal Ctx | 82.9 | Control I Parietal Ctx | 14.0 |
| Control 1 Temporal Ctx | 7.9 | Controt2PanetatObe / | 62.4 |
| Control 2 Temporal Ctx | 23.7 | Control 3 Parietal Ctx | 18.3 |
| Control 3 Temporal Ctx | 19.3 | Control (Path) 1 Parietal Ctx | 74.7 |
| Control 4 Temporal Ctx | 9.1 | Control (Path) 2 Parietal Ctx | 33.4 |
| Control (Path) 1 Temporal Ctx | 56.6 | Control (Path) 3 Parietal Ctx | 5.1 |
| Control (Path) 2 Temporal Ctx | 43.2 | Control (Path) 4 Parietal Ctx | 48.6 |

**Table DF. General_screening_panel_v1.4**

| **Tissue Name** | **Rel. Exp.(%) Ag3690, Run 217130999** | **Tissue Name** | **Rel. Exp.(%) Ag3690, Run 217130999** |
|---|---|---|---|
| Adipose | 12.6 | Renal ca.TK-10 | 26.8 |
| Melanoma* Hs688(A).T | 32.5 | Bladder | 24.1 |
| Melanoma* Hs688(B).T | 31.0 | Gastric ca. (liver met.) NCI-N87 | 61.1 |
| Melanoma* M14 | 53.6 | Gastric ca. KATO III | 38.2 |
| Melanoma* LOXIMVI | 20.0 | Colon ca SW-948 | 6.1 |
| Melanoma* SK-MEL-5 | 57.0 | Colon ca. SW480 | 22.5 |
| Squamous cell carcinoma SCC-4 | 11.6 | Colon ca.* (SW480 met) SW620 | 35.4 |
| Testis Pool | 10.1 | Colon ca. HT29 | 20.7 |
| Prostate ca.* (bone met) PC-3 | 39.2 | Colon ca.HCT-116 | 37.1 |
| Prostate Pool | 11.3 | Colon ca. CaCo-2 | 66.0 |
| Placenta | 3.8 | Colon cancer tissue | 16.7 |
| Uterus Pool | 8.1 | Colon ca. SW1116 | 5.1 |
| Ovarian ca. OVCAR-3 | 26.1 | Colon ca. Colo-205 | 8.0 |
| Ovarian ca. SK-OV-3 | 34.9 | Colon ca. SW-48 | 3.7 |
| Ovarian ca. OVCAR-4 | 3.7 | Colon Pool | 22.4 |
| Ovarian ca. OVCAR-5 | 40.1 | Small Intestine Pool | 20.4 |
| Ovarian ca. IGROV-1 | 11.7 | Stomach Pool | 11.0 |
| Ovarian ca. OVCAR-8 | 15.1 | Bone Marrow Pool | 9.2 |
| Ovary | 15.6 | Fetal Heart | 27.0 |
| Breast ca. MCF-7 | 100.0 | Heart Pool | 13.3 |
| Breast ca.MDA-MB-231 | 20.9 | Lymph Node Pool | 27.5 |
| Breast ca. BT 549 | 52.1 | Fetal Skeletal Muscle | 5.6 |
| Breast ca. T47D | 54.0 | Skeletal Muscle Pool | 15.8 |
| Breast ca. MDA-N | 16.7 | spleen Pool | 15.9 |
| Breast Pool | 24.3 | Thymus Pool | 24.5 |
| Trachea | 10.6 | CNS cancer (glio/astro) U87-MG | 63.3 |
| Lung | 6.7 | CNS cancer (glio/astro) U-118-MG | 54.7 |
| Fetal Lung | 32.3 | CNS cancer (neuro;met) SK-N-AS | 24.1 |
| Lung ca. NCI-N417 | 5.6 | CNS cancer (astro) SF-539 | 27.0 |
| Lung ca. LX-1 | 34.6 | CNS cancer (astro) SNB-75 | 55.1 |
| Lung ca. NCI-H146 | 2.9 | CNS cancer (glio) SNB-19 | 14.9 |
| Lung ca. SHP-77 | 19.8 | CNS cancer (glio) SF-295 | 66.4 |
| Lung ca. A549 | 35.8 | Brain (Amygdala) Pool | 4.6 |
| Lung ca. NCI-H526 | 4.7 | Brain (cerebellum) | 7.9 |
| Lung ca. NCI-H23 | 47.3 | Brain (fetal) | 14.0 |
| Lung ca. NCI-H460 | 27.9 | Brain (Hippocampus) Pool | 6.4 |
| Lung ca. HOP-62 | 10.1 | Cerebral Cortex Pool | 7.4 |
| Lung ca. NCI-H522 | 24.0 | Brain (Substantia nigra) Pool | 5.0 |
| Liver | 1.4 | Brain (Thalamus) Pool | 10.7 |
| Fetal Liver | 25.2 | Brain (whole) | 6.5 |
| Liver ca. HepG2 | 12.1 | Spinal Cord Pool | 8.0 |
| Kidney Pool | 29.9 | Adrenal Gland | 7.2 |
| Fetal Kidney | 31.2 | Pituitary gland Pool | 4.5 |
| Renal ca. 786-0 | 26.1 | Salivary Gland | 2.3 |
| Renal ca. A498 | 15.4 | Thyroid (female) | 5.2 |
| Renal ca. ACHN | 14.3 | Pancreatic ca. CAPAN2 | 15.3 |
| Renal ca. UO-31 | 12.2 | Pancreas Pool | 27.4 |

**Table DG.Panel 1.1**

| **Tissue Name** | **Rel. Exp.(%) Ag534, Run 111162676** | **Tissue Name** | **Rel. Exp.(%) Ag534, Run 111162676** |
|---|---|---|---|
| Adrenal gland | 21.2 | Renal ca. UO-31 | 6.3 |
| Bladder | 57.8 | Renal ca. RXF 393 | 4.4 |
| Brain (amygdala) | 3.7 | Liver | 59.5 |
| Brain (cerebellum) | 59.9 | Liver (fetal) | 13.8 |
| Brain (hippocampus) | 15.0 | Liver ca. (hepatoblast) HepG2 | 13.7 |
| Brain (substantia nigra) | 21.5 | Lung | 5.6 |
| Brain (thalamus) | 7.6 | Lung (fetal) | 6.6 |
| Cerebral Cortex | 12.9 | Lung ca. (non-s.cell) HOP-62 | 50.3 |
| Brain (fetal) | 17.9 | Lung ca. (large cell) NCI- H640 | 47.6 |
| Brain (whole) | 9.9 | Lung ca. (non-s.cell) NCI-H23 | 27.7 |
| glio/astro U-118-MG | 12.3 | Lung ca. (non-s.cl) NCI-H522 | 74.2 |
| astrocytoma SF-539 | 27.0 | Lung ca. (non-sm.cell) A549 | 32.1 |
| astrocytoma SNB-75 | 7.6 | Lung ca. (s.cell var.) SHP-77 | 7.6 |
| astrocytoma SW1783 | 4.9 | Lung ca. (small cell) LX-1 | 52.5 |
| glioma U251 | 18.0 | Lung ca. (small cell) NCI-H69 | 39.2 |
| glioma SF-295 | 31.2 | Lung ca. (squam.) SW 900 | 12.6 |
| glioma SNB-19 | 35.6 | Lung ca. (squam.) NCI-H596 | 34.6 |
| glio/astro U87-MG | 35.4 | Lymph node | 10.4 |
| neuro*; met SK-N-AS | 32.5 | Spleen | 6.4 |
| Mammary gland | 10.7 | Thymus | 13.1 |
| Breast ca. BT 549 | 8.0 | Ovary | 2.9 |
| Breast ca. MDA-N | 32.1 | Ovarian ca. IGROV-1 | 21.9 |
| Breast ca.* (pl.ef) T47D | 62.9 | Ovarian ca. OVCAR-3 | 11.7 |
| Breast ca.*(pl.ef) MCF-7 | 98.6 | Ovarian ca. OVCAR-4 | 4.5 |
| Breast ca.* (pl.ef) MDA-MB-231 | 7.4 | Ovarian ca. OVCAR-5 | 42.3 |
| Small intestine | 24.8 | Ovarian ca. OVCAR-8 | 51.4 |
| Colorectal | 3.1 | Ovarian ca.* (ascites) SK- OV-3 | 28.1 |
| Colon ca. HT29 | 24.7 | Pancreas | 39.5 |
| Colon ca. CaCo-2 | 48.0 | Pancreatic ca. CAPAN 2 | 4.3 |
| Colon ca. HCT-15 | 15.3 | Pituitary gland | 28.7 |
| Coton ca. HCT-116 | 21.3 | Placenta | 27.5 |
| Colon ca.HCC-2998 | 532 | Prostate | 23.0 |
| Colon ca.SW480 | 5.2 | Prostate ca.* (bone met) PC-3 | 32.1 |
| Colon ca.* SW620 (SW480 met) | 44.4 | Salivary gland | 41.2 |
| Stomach | 13.0 | Trachea | 15.3 |
| Gastric ca. (liver met) NCI-N87 | 67.4 | Spinal cord | 14.1 |
| Heart | 44.1 | Testis | 9.8 |
| Skeletal muscle (Fetal) | 6.1 | Thyroid | 19.2 |
| Skeletal muscle | 54.0 | Uterus | 17.1 |
| Endothelial cells | 17.2 | Melanoma M14 | 26.6 |
| Heart (Fetal) | 6.5 | Melanoma LOX IMVI | 5.3 |
| Kidney | 100.0 | Melanoma UACC-62 | 15.5 |
| Kidney(fetal) | 30.4 | Melanoma SK-MEL-28 | 80.7 |
| Renal ca. 786-0 | 15.9 | Melanoma* (met) SK-MEL-5 | 39.5 |
| Renal ca. A498 | 16.4 | Melanoma Hs688(A).T | 14.9 |
| Renal ca. ACHN Renal ca. ACHN | 11.3 | Melanoma* (met) Hs688(B).T | 16.3 |
| Renal ca. TK-10 | 25.9 | | |

**Table DH. Panel 1.2**

| **Tissue Name** | **Rel. Exp.(%) Ag535, Run 112162329** | **Tissue Name** | **Rel. Exp.(%) Ag535, Run 112162329** |
|---|---|---|---|
| Endothelial cells | 10.2 | Renal ca. 786-0 | 14.6 |
| Heart (Fetal) | 1.5 | Renal ca. A498 | 21.8 |
| Pancreas | 94.0 | Renal ca. RXF 393 | 5.5 |
| Pancreatic ca. CAPAN 2 | 8.4 | Renal ca. ACHN | 13.4 |
| Adrenal Gland | 41.5 | Renal ca. UO-31 | 5.8 |
| Thyroid | 29.1 | Renal ca.TK-10 | 22.2 |
| Salivary gland | 47.0 | Liver | 56.3 |
| Pituitary gland | 48.3 | Liver (fetal) | 20.6 |
| Brain (fetal) | 17.1 | Liver ca. (hepatoblast) | 11.3 |
| Brain (whole) | 41.8 | Lung | 14.0 |
| Brain(amygdala) | 9.3 | Lung (fetal) | 20.7 |
| Brain (cerebellum) | 20.0 | Lung ca. (small cell) LX-1 | 48.3 |
| Brain(hippocampus) | 20.7 | Lung ca. (small cell) NCI- H69 | 27.4 |
| Brain (thalamus) | 17.6 | Lung ca. (S.cell var.) SBP-77 | 5.6 |
| Cerebral Cortex | 11.9 | Lung ca.(large cell)NCl-H460 | 61.1 |
| Spinal cord | 12.0 | Lung ca. (non-sm. cell) A549 | 25.9 |
| glio/astro U87-MG | 45.1 | Lung ca.(non-s.cell) NCI-H23 | 22.4 |
| glio/astro U-118-MG | 20.0 | Lung ca. (non-s.cell) HOP-62 | 34.9 |
| astrocytomaSW1783 | 6.3 | Lung ca. (non-s.cl) NCI- H522 | 100.0 |
| neuro*; met SK-N-AS | 34.4 | Lung ca. (squam.) SW 900 | 15.8 |
| astrocytoma SF-539 | 22.2 | Lung ca. (squam) NCI-H596 | 40.3 |
| astrocytoma SNB-75 | 9.6 | Mammary gland | 30.4 |
| glioma SNB-19 | 31.0 | Breast ca* (pl.ef) MCF-7 | 70.7 |
| glioma U251 | 24.7 | Breast ca.* (pl.ef) MDA-MB-231 | 6.6 |
| glioma SF-295 | 27.7 | Breast ca.* (pl. ef) T47D | 37.9 |
| Heart | 49.3 | Breast ca. BT-549 | 16.6 |
| Skeletal Muscle | 48.6 | Breast ca.MDA-N | 31.4 |
| Bone marrow | 23.2 | Ovary | 2.5 |
| Thymus | 17.7 | Ovarian ca: OVCAR-3 | 25.5 |
| Spleen | 22.5 | Ovarian ca. OVCAR-4 | 5.6 |
| Lymph node | 33.0 | Ovarian ca. OVCAR-5 | 45.1 |
| Colorectal Tissue | 1.5 | Ovarian ca. OVCAR-8 | 23.3 |
| Stomach | 24.7 | Ovarian ca. IGROV-1 | 27.2 |
| Small intestine | 34.4 | Ovarian ca. (ascites) SK-OV-3 | 33.9 |
| Colon ca. SW480 | 5.0 | Uterus | 21.2 |
| Colon ca.* SW620 (SW480 met) | 25.3 | Placenta | 60.7 |
| Colon ca. HT29 | 20.3 | Prostate | 25.3 |
| Colon ca. HCT-116 | 20.4 | Prostate ca.* (bone met) PC-3 | 59.9 |
| Colon ca. CaCo-2 | 39.8 | Testis | 37.1 |
| Colon ca. Tissue (ODO3866) | 2.5 | Melanoma Hs688(A).T | 14.5 |
| Colon ca. HCC-2998 | 54.7 | Melanoma* (met) Hs688(B).T | 18.7 |
| Gastric ca.* (liver met) NCI-N87 | 69.3 | Melanoma UACC-62 | 19.1 |
| Bladder | 43.8 | Melanoma M14 | 25.2 |
| Trachea | 13.2 | Melanoma LOX IMVI | 4.2 |
| Kidney | 48.3 | Melanoma* (met) SK-MEL-5 | 50.0 |
| Kidney (fetal) | 51.8 | | |

**Table DI. Panel 1.3D**

| **Tissue Name** | **Rel· Exp.(%) Ag531, Run 165974812** | **Tissue Name** | **Rel. Exp.(%) Ag531, Run 165974812** |
|---|---|---|---|
| Liver adenocarcinoma | 22.1 | Kidney (fetal) | 17.0 |
| Pancreas | 6.8 | Renal ca. 786-0 | 15.7 |
| Pancreatic ca. CAPAN 2 | 19.3 | Renal ca. A498 | 22.4 |
| Adrenal gland | 3.8 | Renal ca. RXF 393 | 30.8 |
| Thyroid | 3.8 | Renal ca. ACHN | 10.7 |
| Salivary gland | 10.4 | Renal ca. UO-31 | 10.7 |
| Pituitary, gland | 15.8, | Renal ca. TK-10 | 12.3 |
| Brain (fetal) | 11.8 | Liver | 10.9 |
| Brain (whole) | 30.6 | Liver (fetal) | 10.0 |
| Brain (amygdala) | 16.8 | Liver ca. (hepatoblast) Hep G2 | 20.2 |
| Brain(cerebellum) | 45.4 | Lung | 8.5 |
| Brain (hippocampus) | 20.3 | Lung (fetal) | 18.7 |
| Brain (substantia nigra) | 7.6 | Lung ca. (small cell) LX-1 | 49.0 |
| Brain (thalamus) | 21.9 | Lung ca. (small cell) NCI-H69 | 50.3 |
| Cerebral Cortex | 9.0 | Lung ca.(s.cell var.)SHP-77 | 23.5 |
| Spinal cord | 27.5 | Lung ca. (large cell)NCI-H460 | 11.7 |
| glio/astro U87-MG | 40.1 | Lung ca. (non-sm.cell) A549 | 10.2 |
| glio/astro U-118-MG | 26.4 | Lung ca. (non-s.cell) NCI-H23 | 30.4 |
| astrocytoma SW1783 | 21.3 | Lung ca. (non-scell) HOP- | 13.5 |
| neuro*; met SK-N-AS | 13.0 | Lung ca. (non-scl) NCI-H522 | 12.9 |
| astrocytoma SF-539 | 68.3 | Lung ca. (squam.) SW 900 | 22.5 |
| astrocytoma SNB-75 | 12.3 | Lung ca. (squam) NCI-H596 | 49.0 |
| glioma SNB-19 | 65.1 | Mammary gland | 5.9 |
| glioma U251 | 29.9 | Breast ca.*(pl.ef)MCF-7 | 100.0 |
| glioma SF-295 | 11.8 | Breast ca.*(pl.ef)MDA- MB-231 | 7.7 |
| Heart (fetal) | 3.0 | Breast ca.*(pl.ef)T47D | 18.9 |
| Heart | 9.7 | Breast ca.BT-549 | 14.2 |
| Skeletal muscle (fetal) | 1.2 | Breast ca.MDA-N | 8.6 |
| Skeletal muscle | 11.0 | Ovary | 1.5 |
| Bone marrow | 19.5 | Ovarian ca. OVCAR-3 | 11.3 |
| Thymus | 16.2 | Ovarian ca. OVCAR-4 | 7.5 |
| Spleen | 11.3 | Ovarian ca. OVCAR-5 | 34.6 |
| Lymph node | 25.0 | Ovarian ca.OVCAR-8 | 20.9 |
| Colorectal | 10.5 | Ovarian ca. IGROV-1 | 11.0 |
| Stomach | 11.1 | Ovarian ca.*(aseites)SK-OV-3 | 41.5 |
| Small intestine | 17.1 | Uterus | 9.9 |
| Colon ca. SW480 | 8.7 | Placenta | 26.4 |
| Colon ca.* SW620(SW480 met) | 24.8 | Prostate | 5.7 |
| Colon ca. HT29 | 14.3 | Prostate ca.* (bone met)PC-3 | 31.0 |
| Colon ca. HCT-116 | 13.0 | Testis | 9.0 |
| Colon ca. CaCo-2 | 50.7 | Melanoma Hs688(A).T | 11.7 |
| Colan ca. tissue(OD03866) Coton ca. tissue(OD03866) | 18.7 | Melanoma* (met) Hs688(B).T | 21.9 |
| Colon ca. HCC-2998 | 36.1 | Melanoma UACC-62 | 20.4 |
| Gastric ca.*(liver met) NCI-N87 | 48.6 | Metanoma M14 | 31.4 |
| Bladder | 22.4 | Melanoma LOX IMVI | 3.7 |
| Trachea | 3.1 | Melanoma* (met) SK-MEL-5 | 19.5 |
| Kidney | 13.0 | Adipose | 11.0 |

**Table DJ. Panel 4.1D**

| **Tissue Name** | **Rel.Exp.(%)Ag3690,Run 169988048** | **Tissue Name** | **Rel.Exp.(%)Ag3690,Run 169988048** |
|---|---|---|---|
| Second Th1 act | 69.7 | HUVEC IL-1beta | 22.7 |
| Secondary Th2 act | 77.4 | HUVEC IFN gamma | 33.9 |
| Secondary Tr1 act | 79.6 | HUVEC TNF alpha + IFN gamma | 25.5 |
| Secondary Th1 rest | 28.7 | HUVEC TNF alpha + ILA | 29.3 |
| Secondary 7h2 rest | 39.2 | HUVBCIL-11 | 14.8 |
| Secondary Tr1 rest | 38.7 | Lung Microvascutar EC none | 45.4 |
| Primary Th1 act | 64.2 | Lung Microvascular EC TNF alpha +IL-beta | 43.8 |
| Primary Th2 act | 79.6 | Microvascular Dermal EC none | 35.4 |
| Primary Tr1 act | 76.3 | Microsvasular Dermal EC TNFalpha+1beta | 34.6 |
| Primary Th1 rest | 45.1 | Bronchial epithelium TNFalpha + IL1beta | 57.4 |
| Primary Th2 rest | 38.2 | Small airway epithelium none | 12.4 |
| Primary Tr1 rest | 64.6 | Small airway epithelium TNFalpha +IL-1beta | 23.2 |
| CD45RA CD4 lymphocyte act | 52.9 | Coronery artery SMC rest | 26.8 |
| CD45RO CD4 lymphocyte act | 84.1 | Coronery artery SMC TNFalpha+IL-lbeta | 20.3 |
| CD8 lymphocyte act | 75.8 | Astrocytes rest | 20.4 |
| Secondary CD8 lymphocyte rest | 74.2 | Astrocytes TNFalpha + IL- 1beta | 22.1 |
| Secondary CD8 lymphocyte act | 33.0 | KU-812 (Basophil) rest | 45.1 |
| CD4 lymphocyte none | 26.6 | KU-812 (Basophil) | 85.9 |
| 2ry Th1/th2/Tr1 anti-CD95 CH11 | 49.7 | CCD1106 (Keratmocytes) none | 39.8 |
| LAK cells rest | 57.8 | CCD1106 (Keratinocytes) TNFalpha + IL-1beta | 33.0 |
| LAK cells IL-2 | 66.0 | Liver cirrhosis | 18.2 |
| LAK cells IL-2+IL-12 | 51.8 | NCI-H292 none | 31.9 |
| LAK cells IL-2+IFN gamma | 68.3 | NCl-H292 IL·4 | 38.2 |
| LAK cell IL-2+IL-18 | 68.3 | NCI-H292 IL-9 | 64.6 |
| LAK cells PMA/ionomycin | 56.6 | NCI-H292 IL-13 | 37.9 |
| NK Cells IL-2 rest | 66.4 | NCI-H292 IFN gamma | 50.0 |
| Two Way MLR 3 day | 80.1 | HPAEC none | 27.7 |
| Two Way MLR 5 day | 59.5 | HPAEC TNF alpha + IL-1 beta | 49.0 |
| Two Way MLR 7 day | 44.1 | Lung fibroblast none | 50.7 |
| PBMC rest | 22.5 | Lung fibroblast TNF alpha+IL-1 beta | 18.3 |
| PBMCPWM | 74.7 | Lung fibroblast IL-4 | 38.7 |
| PBMC PHA-L | 59.9 | Lung fibroblast IL-9 | 52.5 |
| Ramos (B cell) none | 100.0 | Lung fibroblast IL-13 | 37.9 |
| Ramos (B cell) ionomycin | 88.9 | Lung fibroblast IFN gamma | 41.8 |
| B lymphocytes PWM | 50.0 | Dermal fibroblast CCD1070 rest | 52.9 |
| B lymphocytes CD40L and IL-4 CD40L and | 88.9 | Dermal fibroblast CCD1070 TNF alpha fibroblast CCD1070 TNF | 84.7 |
| EOL-1 dbcAMP | 54.3 | Dermal fibroblast CCD1070 IL-1 beta | 27.7 |
| EOL-1 dbcAMP PMA/ionomycin | 73.2 | Dermal fibroblast IFN gamma | 30.1 |
| Dendritic cells none | 59.9 | Dermal fibroblast IL-4 | 80.1 |
| Dendritic cells LPS | 53.2 | Dermal Fibroblasts rest | 37.6 |
| Dendritic cells anti-CD40 | 61.1 | Neutrophils TNFa+LPS | 28.9 |
| Manocytes rest | 59.9 | Neutrophils rest | 13.9 |
| Monocytes LPS | 55.5 | Colon | 21.6 |
| Macrophages rest | 49.0 | Lung | 30.1 |
| Macrophages LPS | 28.9 | Thymus | 92.0 |
| HUVEC none | 16.2 | Kidney | 74.7 |
| HUVEC starved | 27.9 | | |

**Table DK. Panel 4D**

| **Tissue Name** | **Rel.Exp.(%) Ag531, Run 165919167** | **Tissue Name** | **Rel.Exp.(%)Ag531,Run 165919167** |
|---|---|---|---|
| Secondary Th1 act | 18.4 | HUVEC IL-1beta | 2.1 |
| Secondary Th2 act | 0.0 | HUVEC IFN gamma | 5.4 |
| Secondary Tr1 act | 16.8 | HUVEC TNF alpha +IFN gamma | 6.1 |
| Secondary Th1 rest | 4.4 | HUVEC TNF alph+IL4 | 6.6 |
| Secondary Th2 rest | 7.8 | HUVEC IL-11 | 3.4 |
| Secondary Tr1 rest | 7.3 | Lung Microvascular EC none | 5.8 |
| Primary Th1 act | 5.1 | Lung Microvascular EC TNFalpha + IL-1beta | 7.0 |
| Primary Th2 act | 16.6 | Microvascular Dermal EC none | 7.2 |
| Primary Tr1 act | 18.3 | Microsvasular Dermal EC TNFalpha+IL-1beta | 5.8 |
| Primary Th1 rest | 31.9 | Bronchial epithelium TNFalpha + IL1beta | 8.4 |
| Primary Th2 rest | 16.2 | Small airway epithelium none | 2.2 |
| Primary Tr1 rest | 8.4 | Small airway epithelium TNFalpha +IL-1beta | 14.4 |
| CD45RA CD4 lymphocyte | 10.2 | Coronery artery SMC rest | 3.9 |
| CD45RO CD4 lymphocyte act | 17.4 | Coronery artery SMC TNFalpha+ IL- 1beta | 3.9 |
| CD8 lymphocyte act | 13.7 | Astrocytes rest | 3.7 |
| Secondary CD8 lymphocyte rest | 15.6 | Astrocytes TNFalpha + IL-1 beta | 3.3 |
| Secondary CD8 lymphocyte act | 10.5 | KU-812 (Basophil) rest | 10.3 |
| CD4 lymphocyte none | 4.2 | KU-812 (Basophil) PMA/ionomycin | 31.0 |
| 2ry Th1/Th2/Tr1_anti-CD95 CH11 | 10.3 | CCD1106 (Keratinocytes) none | 7.3 |
| LAK cells rest | 15.0 | CCD1106 (Keratmocytes) TNFalpha+IL-1beta | 1.8 |
| LAK cells IL-2 | 14.9 | Liver cirrhosis | 2.2 |
| LAK cells IL-2+IL-12 | 10.1 | Lupus kidney | 1.8 |
| LAK cells IL-2+IFN gamma | 15.7, | NCI-H292 none | 12.5 |
| LAK cells IL-2+IL-18 | 14.8 | NCI-H292 IL-4 | 17.1 |
| LAK cells PMA/ionomycin | 11.7 | NCI-H292 IL-9 | 8.5 |
| NK Cells IL-2 test | 13.2 | NCI-H292IL-13 | 15.5 |
| Two Way MLR 3 day | 18.3 | NCI-H292 IFN gamma | 12.9 |
| Two Way MLR 5 day | 8.0 | HPABC none | 5.7 |
| Two Way MER 7 day | 7.1 | HPAEC TNF alpha + IL-1 beta | 7.3 |
| PBMC rest | 4.9 | Lung fibroblast none | 8.4 |
| PBMC PWM | 61.6 | Lung fibroblast TNF alpha+ IL-1 beta | 4.0 |
| PBMCPHA-L | 19.8 | Lung fibmblast IL-4 | 13.6 |
| Ramos (B cell) none | 19.1 | Lung fibroblast IL-9 | 9.7 |
| Ramos (B cell) ionomycin | 100.0 | Lung fibroblast IL-13 | 7.9 |
| B lymphocytes PWM | 36.1 | Lung fibroblast IFN gamma | 11.7 |
| B lymphocytes CD40L and IL-4 | 25.0 | Dermal fibroblast CCD1070 rest | 14.4 |
| EOL-1 dbcAMP | 13.0 | Dermal fibroblast CCD 1070 TNF alpha | 50.0 |
| EOL-1 dbcAMP PMA/ionomycin | 13.4 | Dermal fibroblast CCD1070 IL-1 beta | 10.8 |
| Dendritic cells none | 7.0 | Dermal fibroblast IFN gamma | 10.1 |
| Dendritic cells LPS | 8.3 | Dermal fibroblast IL-4 | 22.1 |
| Dendritic cells anti-CD40 | 8.7 | IBD Colitis 2 | 1.4 |
| Monocytes rest | 7.3 | IBD Crohn's | 1.5 |
| Monocytes LPS | 6.7 | Colon | 11.8 |
| Macrophages rest | 9.6 | Lung | 6.8 |
| Macrophages LPS | 4.2 | Thymus | 33.0 |
| HUVEC none | 3.9 | Kidney | 22.7 |
| HUVEC starved | 8.0 | | |

**CNS_neurodegeneration_v1.0 Summary:** Ag3690 This panel confirms the expression of the CG57547-01 gene at low levels in the brain in an independent group of individuals. Interestingly, this gene appears to be upregulated in the temporal cortex of Alzheimer's disease patients. Therefore, blockade of the transient receptor potential-related protein encoded for by this gene may be of use in the treatment of this disease and decrease neuronal death. Ag531 Expression of this gene is low/undetectable (CTs > 35) across all of the samples on this panel (data not shown). It is likely that there were experimental difficulties with this run.

**General_screening_panel_v1.4** Summary: Ag3690 The CG57547-01 gene is expressed at moderate levels across all of the samples on this panel with highest expression detected in breast cancer cell line MCF-7 (CT = 27.5). In general, the pattern of gene expression is consistent with that observed in Panels 1.1 and 1.2. Please see Panel 1.1 for a discussion of the potential relevance of this expression pattern in CNS and metabolically relevant tissues.

In addition, consistent with what is seen in Panel 1.2, this gene is expressed at higher levels in fetal liver (CT = 29.5) than in adult liver (CT = 33.6). Therefore, expression of this gene may be used to distinguish fetal liver from adult liver.

**Panel 1.1** Summary: Ag534 The CG57547-01 gene encodes a protein with homology to melastatin, a member of the transient receptor potential (Trp) family of calcium ion channels. The CG57547-01 gene is expressed at moderate to high levels across all the samples on this panel with the highest expression detected in kidney (CT = 25). Interestingly, defects in ion channels are associated with kidney disorders, such as Bartter's syndrome, policystic kidney disease and Dent's disease (ref. 1), suggesting that the CG57547-01 gene may also play a role in kidney homeostasis.

Furthermore, this gene is expressed in a variety of metabolically relevant tissues, including adrenal gland, heart, skeletal muscle, liver, pancreas, pituitary gland, and thyroid. Therefore, as a classical drug target, the CG57547-01 protein may be useful for the treatment of disease in any or all of these tissues, including diabetes and obesity. In support of this hypothesis, mutations in ion channels have previously been associated with hyperinsulinemic hypoglycemia of infancy (ref. 1).

Among CNS samples, the CG57547-01 gene is expressed in hippocampus, substantia nigra, thalamus, and cerebral cortex with highest expression detected in the cerebellum (CT values < 30). The protein encoded by the CG57547-01 gene shows considerable homology to known ion channels, which are the primary targets of all known antiepileptics. Furthermore, all gene mutations known to cause epilepsy or seizure disorders are found in ion channels (ref. 1-2). Two established antiepileptics (valproate and carbamazepine) also have efficacy in the treatment of bipolar disorder. Therefore, therapeutic modulation of this gene or its protein product may be beneficial in the treatment of these disorders as may agonism/antagonism of the ion channel.

Interestingly, it also appears that there is a difference in CG57547-01 gene expression between several adult tissues and their fetal counterparts. Specifically, expression of this gene is significantly higher in adult kidney, liver, skeletal muscle and heart when compared to the corresponding fetal tissues. Thus, the expression of the CG57547-01 gene could be used as a marker of adult tissues, or alternatively its relative absence could be used as a marker of fetal tissues. Since fetal tissues show potential use for organ regeneration, the expression of this gene may be inhibitory to organogenesis. Thus, the therapeutic modulation of the activity of the CG57547-01 gene product, through the use of small molecule drugs or antibodies, might be of use for the treatment of diseases whose pathology is characterized by organ degeneration.

### References:

### 1. Dworakowska B., Dolowy K. (2000) Ion channels-related diseases. Acta Biochim. Pol. 47: 685-703.

There are many diseases related to ion channels. Mutations in muscle voltage-gated sodium, potassium, calcium and chloride channels, and acetylcholine-gated channel may lead to such physiological disorders as hyper- and hypokalemic periodic paralysis, myotonias, long QT syndrome, Brugada syndrome, malignant hyperthermia and myasthenia. Neuronal disorders, e.g., epilepsy, episodic ataxia, familial hemiplegic migraine, Lambert-Eaton myasthenic syndrome, Alzheimer's disease, Parkinson's disease, schizophrenia, hyperekplexia may result from dysfunction of voltage-gated sodium, potassium and calcium channels, or acetylcholine- and glycine-gated channels. Some kidney disorders, e.g., Bartter's syndrome, policystic kidney disease and Dent's disease, secretion disorders, e.g., hyperinsulinemic hypoglycemia of infancy and cystic fibrosis, vision disorders, e.g., congenital stationary night blindness and total colour-blindness may also be linked to mutations in ion channels.

### PMID: 11310970

### 2. Li M., Lester H.A. (2001) Ion channel diseases of the central nervous system. CNS Drug Rev. 7:214-240.

In the last decade, advances in molecular genetics and cellular electrophysiology have increased our understanding of ion channel function. A number of diseases termed "channelopathies" have been discovered that are caused by ion channel dysfunction. Channelopathies can be caused by autoimmune, iatrogenic, toxic or genetic mechanisms. Mutations in genes encoding ion channel proteins that disrupt channel function are now the most commonly identified cause of channelopathies, perhaps because gene disruption is readily detected by the methods of molecular genetics. Ion channels are abundant in the central nervous system (CNS), but CNS channelopathies are rare; however, they overlap with some important neurological disorders, such as epilepsy, ataxia, migraine, schizophrenia, Alzheimer's disease and other neurodegenerative diseases. It is possible that more CNS channelopathies will be discovered when additional ion channels are characterized and the complex mechanisms of brain function are better understood. At present, increased knowledge of the identity, structure and function of ion channels is facilitating diagnosis and treatment of many channelopathies.

### PMID: 11474425

**Panel 1.2 Summary:** Ag535 The CG57547-01 gene is expressed at moderate to high levels across all the samples on this panel with the highest expression detected in lung cancer cell line NCI-H522 (CT = 25). In general, the pattern of CG57547-01 gene expression is consistent with that observed in Panel 1.1; see Panel 1.1 for discussion of expression pattern in CNS and metabolically relevant tissues.

Interestingly, there appears to be a difference in CG57547-01 gene expression between fetal and adult liver. In addition, this gene is also highly expressed in pancreas (CT = 25). Thus, the relative expression of the CG57547-01 gene might be useful as a marker of pancreas tissue. Furthermore, since this gene appears to be differentially expressed in adult and fetal liver, and that fetal liver represents a state of organogenesis, the therapeutic down-modulation of this gene product, through the use of small molecule drugs or antibodies might be of use in the treatment of diseases involving liver degeneration.

**Panel 1.3D** Summary: Ag531 This probe/primer set produced a similar pattern of expression as is seen in Panels 1.1,1.2, and 1.4 using different probe/primer sets, although the expression level is lower in this experiment. This gene is expressed at highest levels in the breast cancer cell line MCF-7 (CT = 33) and is also expressed at low levels in a number of other samples on this panel. Please see Panel 1.1 for discussion of expression pattern in CNS and metabolically relevant tissues.

**Panel 4.1D Summary:** Ag3690 The CG57547-01 gene is expressed at low to moderate levels in each of the cells and tissues examined on this panel. This observation suggests that this gene plays an important role in a variety of immunologically relevant cell types. Interestingly, calcium release activated calcium channels have been shown to be required for T cell activation, cytokine synthesis, and proliferation (ref. 1).

### References:

1. Lepple-Wienhues A., Belka C., Laun T., Jekle A., Walter B., Wieland U., Welz M., Heil L., Kun J., Busch G., Weller M., Bamberg M., Gulbins E., Lang F. (1999) Stimulation of CD95 (Fas) blocks T lymphocyte calcium channels through sphingomyelinase and sphingolipids. Proc. Natl. Acad. Sci. U S A 96: 13795-13800.

Calcium influx through store-operated calcium release-activated calcium channels (CRAC) is required for T cell activation, cytokine synthesis, and proliferation. The CD95 (Apo-1/Fas) receptor plays a role in self-tolerance and tumor immune escape, and it mediates apoptosis in activated T cells. CD95-stimulation blocks CRAC and Ca(2+) influx in lymphocytes through the activation of acidic sphingomyelinase (ASM) and ceramide release. The block of Ca(2+) entry is lacking in CD95-defective lpr lymphocytes as well as in ASM-defective cells and can be restored by retransfection of ASM. C2 ceramide, C6 ceramide, and sphingosine block CRAC reversibly, whereas the inactive dihydroceramide has no effect. CD95-stimulation or the addition of ceramide prevents store-operated Ca(2+) influx, activation of the transcriptional regulator NFAT, and IL-2 synthesis. The block of CRAC by sphingomyelinase metabolites adds a function to the repertoire of the CD95 receptor inhibiting T cell activation signals.

### PMID: 10570152

**Panel 4D Summary**: Ag531 This probe/primer set produced a similar pattern of expression as was seen in Panel 4.1D using probe/primer set Ag3690, although the levels of expression are lower on Panel 4D. Expression of the CG57547-01 gene is highest in Ramos B cells treated with ionomycin (CT = 30.4). Moderate expression of this gene is also seen in other B cell samples including peripheral blood mononuclear cells treated with pokeweed mitogen and B lymphocytes. Expression of this transcript in B cells suggests that this gene may be involved in rheumatic disease including rheumatoid arthritis, lupus, osteoarthritis, and hyperproliferative B cell disorders. Low but significant expression of this gene is seen in the other samples on this panel.

### NOV6

Expression of gene NOV6/CG57611-01 was assessed using the primer-probe set Ag3295, described in Table EA. Results of the RTQ-PCR runs are shown in Tables EB, EC and ED.

**Table EA. Probe Name Ag3295**

| **Primers** | **Sequences** | **Length** | **Start Position** | **SEQ ID NO** |
|---|---|---|---|---|
| Forward | 5'-caagccttccttcactgttaag-3' | 22 | 396 | 267 |
| Probe | TET-5'-ccccagcttcaaacatttctactcaa-3'-TAMRA | 26 | 419 | 268 |
| Reverse | 5'-gcctcaacagacagtttggtat-3' | 22 | 452 | 269 |

**Table EB. CNS_neurodegeneration_v1.0**

| **Tissue Name** | **Rel. Exp.(%) Ag3295, Run 210062303** | **Tissue Name** | **Rel. Exp.(%) Ag3295, Run 210062303** |
|---|---|---|---|
| AD 1 Hippo | 9.7 | Control (Path) 3 Temporal Ctx | 17.0 |
| AD 2 Hippo | 29.1 | Control (Path) 4 Temporal Ctx | 40.6 |
| AD 3 Hippo | 28.3 | AD 1 Occipital Ctx | 32.8 |
| AD 4 Hippo | 8.1 | AD 2 Occipital Ctx (Missing) | 0.0 |
| AD 5 hippo | 17.4 | AD 3 Occipital Ctx | 29.3 |
| AD 6 Hippo | 64.6 | AD 4 Occipital Ctx | 15.0 |
| Control 2 Hippo | 63.7 | AD 5 Occipital Ctx | 66.0 |
| Control 4 Hippo | 16.6 | AD 6 Occipital Ctx | 73.2 |
| Control (Path) 3 Hippo | 0.0 | Control 1 Occipital Ctx | 8.5 |
| AD 1 Temporal Ctx | 41.2 | Control 2 Occipital Ctx | 24.7 |
| AD 2 Temporal Ctx | 35.4 | Control 3 Occipital Ctx | 43.2 |
| AD 3 Temporal Ctx | 48.3 | Control 4 Occipital Ctx | 10.4 |
| AD 4 Temporal Ctx | 36.9 | Control (Path) 1 Occipital Ctx | 95.3 |
| AD 5 Inf Temporal Ctx | 74.7 | control (Path) 2 Occipital Ctx | 7.2 |
| AD 5 SupTemporal Ctx | 65.5 | Control (Path) 3 Occipital Ctx | 1.4 |
| AD 6 Inf Temporal Ctx | 85.9 | Control (Path) 4 Occipital,Ctx | 25.7 |
| AD 6 Sup Temporal Ctx | 49.0 | Control 1 Parietal Ctx | 10.6 |
| Control 1 Temporal Ctx | 8.4 | Control 2 Parietal Ctx | 100.0 |
| Control 2 Temporal Ctx | 42.9 | Control 3 Parietal Ctx | 14.1 |
| Control 3 Temporal Ctx | 23.0 | Control (Path) 1 Parietal Ctx | 47.0 |
| Control 4 Temporal Ctx | 31.0 | Control (Path) 2 Parietal Ctx | 12.4 |
| Control (Path) 1 Temporal Ctx | 62.4 | Control (Path) 3 Parietal Ctx | 4.8 |
| Control (Path) 2 Temperal Ctx | 42.6 | Control (Path) 4 Parietal Ctx | 45.7 |

**Table EC. General_screening_panel_v1.4**

| **Tissue Name** | **Rel. Exp.(%) Ag3295, Run 215669651** | **Tissue Name** | **Rel. Exp.(%) Ag3295, Run 215669651** |
|---|---|---|---|
| Adipose | 0.2 | Renal ca. TK-10 | 18.2 |
| Metmoma* Hs688(A).T | 0.1 | Bladder | 0.7 |
| Melanoma* Hs688 (B).T | 0.4 | Gastric ca. (liver met.) NCI-N87 | 4.3 |
| Melanoma^{*} M14 | 2.6 | Gastric ca. KATO III | 2.3 |
| Melanoma^{*} LOXIMVI | 0.1 | Colon ca. SW-948 | 0.2 |
| Melanoma* SK-MEL-5 | 1.3 | Colon ca. SW480 | 3.2 |
| Squamous cell carcinoma SCC-4 | 0.5 | Colon ca.*(SW480 met) SW620 | 0.8 |
| Testis Pool | 0.5 | Colon ca. HT29 | 29.1 |
| Prostate ca.* (bone met) PC-3 | 0.9 | Colon ca. HCT-1 16 | 1.3 |
| Prostate Pool | 3.6 | Colon ca. CaCo-2 | 4.9 |
| Placenta | 1.0 | Colon cancer tissue | 1.3 |
| Uterus Pool | 1.4 | Colon ca. SW1116 | 0.7 |
| Ovarian ca. OVCAR-3 | 0.2 | Colon ca. Colo-205 | 0.6 |
| Ovarian ca. SK-OV-3 | 0.9 | Colon ca. SW-48 | 0.4 |
| Ovarian ca. OVCAR-4 | 0.3 | Colon Pool | 0.9 |
| Ovarian ca. OVCAR-5 | 4.2 | Small Intestine Pool | 1.2 |
| Ovarian ca. IGROV-1 | 0.4 | Stomach Pool | 0.1 |
| Ovarian ca. OVCAR-8 | 0.9 | Bone Marrow Pool | 2.9 |
| Ovary | 0.2 | Fetal Heart | 0.1 |
| Breast ca. MCF-7 | 0.4 | Heart Pool | 0.0 |
| Breast ca. MDA-MB-231 | 0.6 | Lymph Node Pool | 0.1 |
| Breast ca. BT 549 | 2.9 | Fetal Skeletal Muscle | 0.1 |
| Breast ca. T47D | 20.4 | Skeletal Muscle Pool | 0.5 |
| Breast ca. MDA-N | 2.0 | Spleen Pool | 0.5 |
| Breast Pool | 0.4 | Thymus Pool | 0.5 |
| Trachea | 12.5 | CNS cancer (glio/astro) U87-MG | 3.4 |
| Lung | 0.3 | CNS cancer (glio/astro) U-118-MG | 0.4 |
| Fetal Lung | 0.5 | CNS cancer (neuro;met) SK- | 0.6 |
| Lung ca. NCI-N417 | 0.0 | CNS cancer (astro) SF-539 | 0.1 |
| Lung,ca. LX-1, | 2.0 | CNS cancer (astro) SNB-75 | 2.2 |
| Lung ca. NCI-H146 | 0.6 | CNS cancer (glio) SNB-19 | 1.3 |
| Lung ca. SHP-77 | 1.4 | CNS cancer (glio) SF-295 | 3.7 |
| Lung ca. A549 | 27.4 | Brain (Amygdala) Pool | 0.8 |
| Lung ca NCI-H526 | 0.2 | Brain (cerebellum) | 3.3 |
| Lung ca. NCI-H23 | 3.1 | Brain (fetal) | 1.5 |
| Lung ca. NCI-H460 | 2.7 | Brain (Hippocampus) Pool | 0.5 |
| Lung ca. HOP-62 | 0.2 | Cerebral Cortex Pool | 0.6 |
| Lung ca. NCI-H522 | 4.3 | Brain (Substantia nigra) Pool | 0.4 |
| Liver | 0.4 | Brain (Thalamus) Pool | 0.8 |
| Fetal Liver | 1.1 | Brain (whole) | 0.5 |
| Liver ca. HepG2 | 42.9 | Spinal Cord Pool | 1.1 |
| Kidney Pool | 1.0 | Adrenal Gland | 0.2 |
| Fetal Kidney | 0.5 | Pituitary gland Pool | 0.1 |
| Renal ca. 786-0 | 0.3 | Salivary Gland | 100.0 |
| Renal ca. A498 | 5.1 | Thyroid (female) | 0.1 |
| Renal ca. ACHN | 0.4 | Panc_reatic ca. CAPAN2 | 2.8 |
| Renal ca. UO-31 | 0.3 | Pancreas Pool | 0.7 |

**Table ED.Panel 4D**

| **Tissue Name** | **Rel. Exp.(%) Ag3295, Run 164331710** | **Tissue Name** | **Rel. Exp.(%) Ag3295, Run 164331710** |
|---|---|---|---|
| Secondary Th1 act | 0.8 | HUVEC IL-1beta | 0.0 |
| Secondary Th2 act | 23.8 | HUVEC IFN gamma | 3.2 |
| Secondary Tr1 act | 11.9 | HUVEC TNF alpha + IFN gamum | 3.0 |
| Secondary Th1 rest | 0.0 | HUVEC TNF alpha+IL4 | 0.0 |
| Secondary Th2 rest | 2.4 | HUVEC IL-11 | 0.0 |
| Secondary Tr1 rest | 3.0 | Lung Microvascular EC none | 1.8 |
| Primary Th1 act | 12.5 | Lung Microvascular EC TNFalpha IL-1beta | 3.7 |
| Primary Th2 act | 47.0 | Microvascular Dermal EC none | 4.0 |
| Primary Tr1 act | 30.1 | Microsvasular Dermal EC TNFalpha+IL-1beta | 0.0 |
| Primary Th1 rest | 0.0 | Bronchial epithelium TNFalpha + IL1beta | 9.5 |
| Primary Th2 rest | 1.4 | Small airway epithelium none | 100.0 |
| Primaryl Tr1 rest | 11.5 | Small airway epithelium TNFalpha + IL-1beta | 55.5 |
| CD45RA CD4 lymphocyte act | 0.7 | Coronery artery SMC rest | 0.0 |
| CD45RO CD4 lymphocyte act | 0.0 | Coronery artery SMC TNFalpha + IL-1beta | 0.0 |
| CD8 lymphocyte act | 5.0 | Astrocytes rest | 1.3 |
| Secondary CD8 lymphocyte rest | 5.9 | AstrocytesTNFalpha+IL-1beta | 0.0 |
| Secondary CD8 lymphocyte act | 8.8 | KU-812 (Basophil) rest | 2.5 |
| CD4 lymphocyte none | 0.0 | KU-812 (Basophil) PMA/ionomycin | 3.9 |
| 2ryTh1/Th2/Tr1_anti-CD95: CH11 | 3.6 | CCD1106 (Keratinocytes) none | 2.7 |
| LAK cells rest LAK cells rest | 17.7 | CCD 1106 (Keratinocytes) TNFalpha +IL-1beta | 14.9 |
| LAK cells IL-2 | 6.2 | liver cirrhosis | 11.1 |
| LAK cells IL-2+IL-12 | 1.6 | Lupus kidney | 0.0 |
| LAK cells IL-2+IFN gamma | 5.7 | NCI-H292 none | 1.4 |
| LAK cells IL-2+ IL-18 | 9.9 | NCI-H292 IL-4 | 3.1 |
| LAK cells PMA/ionomycin | 8.0 | NCl-H292 IL-9 | 7.1 |
| NK Cells IL-2 rest | 3.3 | NCI-H292 IL-13 | 1.0 |
| Two Way MLR 3 day | 9.9 | NCI-H292 IFN gamma | 1.6 |
| Two Way MLR 5 day | 8.7 | HPAEC none | 1.2 |
| Two Way MLR 7 day | 3.2 | HPAEC TNF alpha+lL-1 beta | 0.0 |
| PBMC rest | 0.0 | Lung fibroblast none | 3.4 |
| PBMC PWM | 21.0 | Lung fibroblast TNF alpha + IL-1 beta | 0.0 |
| PBMCPHA-L | 14.9 | Lung fibroblast IL-4 | 0.5 |
| Ramos (B cell) none | 2.2 | Lung fibroblast IL-9 | 3.7 |
| Ramos (B cell) ionomycin | 7.0 | Lung fibroblast IL-13 | 1.5 |
| B lymphocytes PWM | 18.6 | Lung fibroblast IFN gamma | 1.6 |
| B lymphocytes CD40L and IL-4 | 7.5 | Dermal fibroblast CCD1070 rest | 14.0 |
| EOL-1 dbcAMP EOL-1 dbcAMP | 0.0 | Dermal fibroblast CCD1070 TNF alpha | 14.7 |
| EOL-1 dbcAMP PMA/ionomycin | 1.5 | Dermal fibroblast CCD1070 IL-1 beta | 2.7 |
| Dendritic cells none | 6.4 | Dermal fibroblast IFN gamma | 3.5 |
| Dendritic cells LPS | 11.0 | Dermal fibroblast IL-4 | 6.6 |
| Dendritic cells anti-CD40 | 8.4 | IBD Colitis 2 | 0.0 |
| Monocytes rest | 0.0 | IBD Crohn's | 0.0 |
| Monacytes LPS | 6.7 | Colon | 41.5 |
| Macrophages rest | 4.9 | Lung | 16.7 |
| Macrophages LPS | 8.1 | Thymus | 0.0 |
| HWEC none | 0.0 | Kidney | 13.9 |
| HUVEC starved | 1.8 | | |

**CNS_neurodegeneration_v1. 0 Summary:** Ag3295 This panel confirms the expression of this gene at low levels in the brain in an independent group of individuals (CTs = 33-35). However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screenmg_panel_v1.4** Summary: Ag3295 Expression of the CG57611-01 gene is highest in the salivary gland (CT = 28). Therefore, expression of this gene may be used to distinguish salivary gland from the other samples on this panel. In general, expression of this gene appears to be higher in several cancer cell lines, including lung, breast, colon and liver cancer, when compared to normal tissues. Thus, therapeutic modulation of the activity of this gene or its protein product, using small molecule drugs, antibodies or protein therapeutics, may be of benefit in the treatment of lung, breast, colon and liver cancer. This gene encodes a protein with homology to CD22, a B lymphocyte-restricted adhesion molecule. Antibodies to the CD22 protein have been used as a therapeutic treatment for lymphomas (ref. 1), suggesting that the CG57611-01 protein may also be an attractive target for the treatment of leukemias and lymphomas.

This gene is also expressed at low but significant levels in the spinal cord, cerebellum, and amygdala. Therefore, this gene may play a role in central nervous system disorders such as Alzheimer's disease, Parkinson's disease, epilepsy, multiple sclerosis, schizophrenia and depression.

### References:

### 1. Rybak SM, Newton DL. Antibody targeted therapeutics for lymphoma: new focus on the CD22 antigen and RNA. Expert Opin Biol Ther 2001 Nov;1(6):995-1003

The approval of antibodies for cancer treatment has provoked increased interest in the development of new and improved antibody-mediated therapies. This emerging approach centres on targeting CD22 on human B-cells with a monoclonal antibody (mAb). Anti-CD22 antibodies conjugated to a cytotoxic RNAse elicits potent and specific killing of the lymphoma cells in vitro and in human lymphoma models in severe combined immune deficiency (SCID) mice. RNA damage caused by RNAses could be an important alternative to standard DNA damaging chemotherapeutics. Moreover, targeted RNAses may overcome problems of toxicity and immunogenicity associated with plant- or bacterial toxin-containing immunotoxins.

**Panel 4D Summary:** Ag3295 Expression of this gene is highest in small airway epithelium, irrespective of treatment (CTs = 31-32). Therefore, modulation of the expression or activity of the protein encoded by this transcript through the application of small molecule therapeutics may be useful in the treatment of asthma, COPD, and emphysema. Low but significant expression of this gene is seen in a number of samples on this panel. Expression of this gene appears to be higher in activated T cells than in resting T cells, suggesting that expression of this gene could be used to distinguish these two types of T cells.

### NOV8

Expression of gene NOV8a/CG57452-01 and NOV8b/CG57452-02 was assessed using the primer-probe sets Ag3243 and Ag878, described in Tables FA and FB. Results of the RTQ-PCR runs are shown in Tables FC, FD, FE, FF, FG and FH. Please note that the CG57452-02 is only recognized by the Ag878 primer-probe set.

**Table FA. Probe Name Ag3243**

| **Primers** | **Sequences** | **Length** | **Start Position** | **SEQ ID NO** |
|---|---|---|---|---|
| Forward | 5'-ccatttcggacagtctcaatt-3' | 21 | 1313 | 270 |
| Probe | TET-5'-cctcacctttaagaatagtagctctggaca-3'-TAMRA | 30 | 1337 | 271 |
| Reverse | 5'-aggtgaagctctgggtctttt-3' | 21 | 1383 | 272 |

**Table FB. Probe Name Ag878**

| **Primers** | **Sequences** | **Length** | **Start Position** | **SEQ ID NO** |
|---|---|---|---|---|
| Forward | 5'-agccctgttgttttattgatga-3' | 22 | 1745 | 273 |
| Probe | TET-5'-cctgagccccaacaaggatttcatat-3'-TAMRA | 26 | 1710 | 274 |
| Reverse | 5'-cagacgaagggteaaatgg-3' | 19 | 1682 | 275 |

**Table FC. CNS_neurodegeneration_v1.0**

| **Tissue Name** | **Rel.Exp.(%)Ag3243,Run 210037850** | **Tissue Name** | **Rel. Exp.(%) Ag3243, Run 210037850** |
|---|---|---|---|
| AD 1 Hippo | 33.2 | Control (Path) 3 Temporal | 18.7 |
| AD 2 Hippo | 49.7 | Control (Path) 4 Temporal Ctx | 74.7 |
| AD 3 Hippo | 30.8 | AD 1 Occipital Ctx | 33.9 |
| AD 4 Hippo | 25.3 | AD 2 Occipital Ctx (Missing) | 0.0 |
| AD 5 hippo | 52.1 | AD 3 Occipital Ctx | 8.2 |
| AD 6 Hippo | 49.7 | AD 4 Occipital Ctx | 35.6 |
| Control 2 Hippo | 97.3 | AD 5 Occipital Ctx | 14.0 |
| Control 4 Hippo | 31.9 | AD 6 Occipital Ctx | 26.4 |
| Control (Path) 3 Hippo | 30.4 | Control 1 Occipital Ctx | 4.9 |
| AD 1 Temporal Ctx | 48.0 | Control 2 Occipital Ctx | 35.6 |
| AD 2 Temporal Ctx | 38.4 | Control 3 Occipital Ctx | 24.8 |
| AD 3 Temporal Ctx | 16.6 | Control 4 Occipital Ctx | 17.0 |
| AD 4 Temporal Ctx | 51.4 | Control (Path) 1 Occipital Ctx | **100.0** |
| AD 5 Inf Temporal Ctx | 51.4 | Control (Path) 2 Occipital Ctx | 35.6 |
| AD 5 SupTemporal Ctx | 80.1 | Control (Path) 3 Occipital Ctx | 6.1 |
| AD 6 Inf Temporal Ctx | 32.8 | Control (Path) 4 Occipital Ctx | 43.8 |
| AD 6 Sup Temporal Ctx | 32.5 | Control 1 Parietal Ctx | 19.8 |
| Control 1 Temporal Ctx | 43.8 | Control 2 Parietal Ctx | 52.1 |
| Control 2 Temporal Ctx | 26.1 | Control 3 Parietal Ctx | 9.7 |
| Control 3 Temporal Ctx | 42.3 | Control (Path) 1 Parietal Ctx | 62.4 |
| Control 4 Temporal Ctx | 26.1 | Control (Path) 2 Parietal Ctx | 50.3 |
| Control (Path) 1 Temporal Ctx | 93.3 | Control (Path) 3 Parietal | 10.9 |
| Control (Path) 2 Temporal Ctx | 77.9 | Control (Path) 4 Parietal Ctx | 46.7 |

**Table FD. General_screening_panel-v1.4**

| **Tissue Name** | **Rel. Exp.(%) Ag3243, Run 214693632** | **Tissue Name** | **Rel. Exp.(%) Ag3243, Run 214693632** |
|---|---|---|---|
| Adipose | 0.0 | Renal ca. TK-10 | 0.0 |
| Melanoma* Hs688(A).T | 0.0 | Bladder | 0.9 |
| Melanoma* Hs688(B).T | 0.0 | Gastric ca. (liver met.) NCI-N87 | 0.0 |
| Melanoma* M14 | 0.0 | Gastric ca. KATO III | 0.0 |
| Melanoma* LOXIMVI | 1.7 | Colon ca. SW-948 | 0.0 |
| Melanoma* SK-MEL-5 | 0.5 | Colon ca. SW480 | 0.7 |
| Squamous cell carcinoma SCC-4 | 0.0 | Colon ca.* (SW480 met) SW620 | 0.0 |
| Testis Pool | 3.4 | Colon ca. HT79 | 0.0 |
| Prostate ca.* (bone met) PC-3 | 0.7 | Colon ca. HCT-116 | 0.0 |
| Prostate Pool | 0.0 | Colon ca. CaCo-2 | 0.0 |
| Placenta | 0.0 | Colon cancer tissue | 0.2 |
| Uterus Pool | 0.0 | Colon ca. SW1116 | 0.0 |
| Ovarian ca. OVCAR-3 | 0.8 | Colon ca. Colo-205 | 0.0 |
| Ovarian ca. SK-OV-3 | 8.1 | Colon ca. SW-48 | 0.0 |
| Ovarian ca. OVCAR-4 | 0.0 | Colon Pool | 0.2 |
| Ovarian ca.OVCAR-5 | 0.0 | Small Intestine Pool | 5.3 |
| Ovarian ca. IGROV-1 | 0.0 | Stomach Pool | 0.3 |
| Ovarian ca. OVCAR-8 | 2.8 | Bone Marrow Pool | 0.0 |
| Ovary | 0.5 | Fetal Heart. | 1.5 |
| Breast ca. MCF-7 | 0.0 | Heart Pool | 0.3 |
| Breast ca. MDA-MB-231 | 0.0 | Lymph Node Pool | 0.0 |
| Breast caw BT 549 | 0.2 | Fetal Skeletal Muscle | 1.4 |
| Breast ca. T47D | 0.4 | Skeletal Muscle Pool | 0.0 |
| Breast ca.MDA-N | 0.0 | Spleen Pool | 9.4 |
| Breast Pool | 0.8 | Thymus Pool | 2.9 |
| Trachea | 0.3 | CNS cancer (glio/astro) U87-MG | 3.1 |
| Lung | 0.0 | CNS cancer (glio/astro) U-118-MG | 20.9 |
| Fetal Lung | 100.0 | CNS cancer (neuro;met) SK-N-AS | 0.7 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 2.5 | CNS cancer (glio) SNB-19 | 0.0 |
| Lung ca. SHP-77 | 0.2 | CNS cancer (glio) SF-295 | 0.0 |
| Lung ca. A549 | 0.3 | Brain (Amygdala) Pool | 38.4 |
| Lung ca. NCI-H526 | 0.0 | Brain (cerebellum) | 5.1 |
| Lung ca. NCI-H23 | 1.4 | Brain (fetal) | 11.9 |
| Lung ca. NCI-H460 | 16.2 | Brain (Hippocampus) Pool | 42.9 |
| Lung ca. HOP-62 | 0.4 | Cerebral Cortex Pool | 44.8 |
| Lung ca. NCI-H522 | 1.1 | Brain (Substantia nigra) Pool | 28.5 |
| Liver | 0.0 | Brain (Thalamus) Pool | 54.3 |
| Fetal Liver | 0.0 | Brain(whole) | 32.8 |
| Liver ca.HepG2 | 0.0 | Spinal Cord Pool | 32.1 |
| kidney Pool | 0.0 | Adrenal Gland | 26.2 |
| Fetal Kidney | 73.2 | Pituitary gland Pool | 17.9 |
| Renalca. 786-0 | 0.0 | Salivary Gland | 0.0 |
| Renal ca. A498 | 0.0 | Thyroid (female) | 0.0 |
| Renal ca. ACHN | 0.0 | Pancreatic ca. CAPAN2 | 0.0 |
| Renal ca. UO-31 | 0.5 | Pancreas Pool | 0.2 |

**Table FE. Panel 1.2**

| **Tissue Name** | **Rel. Exp.(%) Ag878, Run 118826261** | **Tissue Name** | **Rel. Exp.(%) Ag878, Run 118826261** |
|---|---|---|---|
| Endothelial cells | 4.0 | Renal ca. 786-0 | 0.0 |
| Heart (Fetal) | 2.8 | Renal ca. A498 | 0.3 |
| Pancreas | 6.5 | Renal ca. RXF 393 | 0.0 |
| Pancreatic ca. CAPAN 2 | 0.0 | Renal ca. XCHN | 0.1 |
| Adrenal Gland | 68.8 | Renal ca. UO-31 | 0.2 |
| Thyroid | 1.1 | Renal ca. TK-10 | 0.0 |
| Salivary gland | 5.7 | Liver | 4.6 |
| Pituitary gland | 100.0 | Liver (fetal) | 0.6 |
| Brain (fetal) | 73.7 | Liver ca. (hepatoblast) HepG2 | 0.0 |
| Brain (whole) | 53.2 | Lung | 27.2 |
| Brain (amygdala) | 46.7 | Lung(fetal) | 20.7 |
| Brain (cerebellum) | 20.2 | Lung ca. (small cel) LX-1 | 0.1 |
| Brain (hippocampus) | 46.3 | Lung ca. (small cell) NCI-H69 | 8.3 |
| Brain (thalamus) | 28.9 | Lung ca. (s.cell var.) SHP- | 0.0 |
| Cerebral Cortex | 45.7 | Lung ca. (large cell)NCI-H460 | 28.7 |
| Spinal cord | 27.4 | Lung ca. (non-sm cell) | 1.0 |
| glio/astro U87-MG | 4.3 | Lung ca. (non-s.cell) NCI- | 0.8 |
| glio/astro U-1 18-MG | 8.3 | Lung ca. (non-s.cell) HOP-62 | 1.9 |
| astrocytoma SW1783 | 0.5 | Lung ca. (non-s.cl) NCI-H522 | 5.7 |
| neuro*; met SK-N-AS | 9.2 | Lung ca. (squam.) SW 900 | 0.1 |
| astrocytoma SF-539 | 0.7 | Lung ca. (squam) NCI-H596 | 32.3 |
| astrocytoma SNB-75 | 0.0 | Mammary gland | 6.4 |
| glioma SNB-19 | 3.9 | Breast ca.*(pl. ef) MCF-7 | 0.0 |
| glioma U251 | 10.4 | Breast ca.* (pl.ef) MDA-MB-231 | 0.0 |
| glioma SF-295 | 2.4 | Breast ca.* (pl. ef) T47D | 5.3 |
| Heart | 16.2 | Breast ca. BT-549 | 1.2 |
| Skeletal Muscle | 3.9 | Breast ca. MDA-N | 0.4 |
| Bone marrow | 0.0 | Ovary | 7.7 |
| Thymus | 0.8 | Ovarian ca. OVCAR-3 | 12.2 |
| Spleen | 3.6 | Ovarian ca. OVCAR-4 | 0.0 |
| Lymph node | 24.0 | Ovarian ca. OVCAR-5 | 5.1 |
| Colorectal Tissue | 0.3 | Ovarian ca. OVCAR-8 | 1.9 |
| Stomach | 5.7 | Ovarian ca. IGROV-1 | 0.8 |
| Small intestine | 6.2 | Ovarian ca. (ascites) SK-OV-3 | 12.5 |
| Colon ca. SW480 | 0.3 | Uterus | 2.2 |
| Colon ca.*SW620 (SW480 met) | 0.0 | Placenta | 3.3 |
| Colon ca. HT29 | 0.3 | Prostate | 0.5 |
| Colon ca. HCT-116 | 0.0 | Prostate ca.* (bone met) PC-3 | 4.1 |
| Colon ca. CaCo-2 | 0.4 | Testis | 14.7 |
| Colon ca. Tissue (ODO,3866) | 2.6 | Melanoma Hs688(A).T | 0.1 |
| Colon ca. HCC-2998 | 0.0 | Melanoma* (met) Hs688(B).T | 0.9 |
| Gastric ca.* (liver met) NCI-N87 | 0.0 | Melanoma UACC-62 | 20.3 |
| Bladder | 4.9 | Melanoma M14 | 1.4 |
| Trachea | 0.7 | Melanoma LOX IMVI | 5.6 |
| Kidney | 28.3 | Melanoma* (met) SK-MEL-5 | 0.2 |
| Kidney (fetal) | 33.7 | | |

**Table FF. Panel 2.2**

| **Tissue Name** | **Rel.Exp.(%) Ag3243,Run 174443356** | **Tissue Name** | **Rel. Exp. (%)Ag3243, Run 174443356** |
|---|---|---|---|
| Normal Colon | 0.0 | Kidney Margin (OD04348) | 20.2 |
| Colon cancer (OD06064) | 0.0 | Kidney malignant cancer (OD04450-01) | 0.0 |
| (OD06204B) | 0.0 | Kidney normal adjacent tissue (OD06204B) | 5.2 |
| Colon cancer(OD06159) | 0.0 | Kidney Cancer (OD04450-01) | 0.0 |
| Colon Margin (OD06159) | 0.0 | Kidney Margin (OD0450-03) | 34.2 |
| Colon cancer (OD06297-04) | 0.0 | Kidney Cancer 8120613 | 0.0 |
| Colon Margin (OD06297-05) | 0.0 | Kidney Margin 8120614 | 1.7 |
| CC Gr.2 ascend colon (OD03921) | 0.0 | Kidney Cancer 9010320 | 0.0 |
| CC Margin (OD03921) | 0.0 | Kidney Margin 9010321 | 0.0 |
| Colon cancer metastasis (OD06104) | 0.0 | Kidney Cancer 8120607 | 0.0 |
| Lung Margin (OD06104) | 0.0 | Kidney Margin 8120608 | 3.4 |
| Colon mets to lung (OD04451-01) | 8.3 | Normal Uterus | 0.0 |
| Lung Margin (OD04451-02) | 100.0 | Uterine Cancer 064011 | 0.0 |
| 'Normal Prostate | 0.0 | Normal Thyroid | 0.0 |
| Prostate Cancer (OD04410) | 0.0 | Thyroid Cancer 064010 | 0.0 |
| Prostate Margin (OD04410) | 0.0 | Thyroid Cancer A302152 | 3.4 |
| Normal Ovary | 0.0 | Thyroid Margin A302153 | 0.0 |
| Ovarian cancer (OD06283-03) | 0.0 | Normal Breast | 3.3 |
| Ovanan Margin(OD06283-07) | 0.0 | Breast Cancer (OD04566) | 0.0 |
| Ovarian Cancer 064008 | 0.0 | Breast Cancer 1024 | 0.0 |
| Ovarian cancer (OD06145) | 0.0 | Breast Cancer (OD04590-01) | 0.0 |
| Ovarian Margin (OD06145) | 0.0 | Breast Cancer Mets (OD04590-03) | 0.0 |
| Ovarian cancer (OD06455-03) | 0.0 | Breast Cancer Metastasis (OD04655-05) | 15.1 |
| Ovarian Margin (OD06455-07) | 0.0 | Breast Cancer 064006 | 0.0 |
| Normal Lung | 30.4 | Breast Cancer 9100266 | 0.0 |
| Invasive poor diff. lung adeno (OP04945-01 | 0.0 | Breast Margin 9100265 | 0.0 |
| Lung Margin (OD04945-03) | 51.4 | Breast Cancer A209073 | 0.0 |
| Lung Malignant Cancer (OD03126) | 0.0 | Breast Margin A2090734 | 0.0 |
| Lung Margin (OD03126) | 9.9 | Breast cancer (OD06083) | 0.0 |
| Lung Cancer (OD05014A) | 6.9 | Breast cancer node metastasis (OD06083) | 2.9 |
| Lung Margin (OD05014B) | 21.2 | Normal Liver | 0.0 |
| Lung cancer (OD06081) | 0.0 | Liver Cancer 1026 | 0.0 |
| Lung Margin (OD06081) | 23.0 | Liver Cancer 1025 | 0.0 |
| Lung Cancer (OD04237-01) | 0.0 | Liver Cancer 6004-T | 0.0 |
| Lung Margin (OD04237-02) | 40.3 | Liver Tissue 6004-N | 0.0 |
| Ocular Melanoma Metastasis | 0.0 | Liver Cancer 6005-T | 0.0 |
| Ocular Melanoma Margin (Liver) | 0.0 | Liver Tissue 6005-N | 0.0 |
| Melanoma Metastasis | 0.0 | Liver Cancer 064003 | 3.0 |
| Melanoma Margin (Lung) | 52.5 | Normal Bladder | 0.0 |
| Normal Kidney | 16.3 | Bladder Cancer 1023 | 0.0 |
| Kidney Ca, Nuclear grade 2 (OD04338) | 28.7 | Bladder Cancer A302173 | 0.0 |
| Kidney Margin (OD04338) | 0.0 | Normal Stomach | 3.9 |
| Kidney Ca Nuclear grade 1/2 | 0.0 | Gastric Cancer 9060397 | 0.0 |
| Kidney Margin (OD04339) | 41.2 | Stomach Margin 9060396 | 0.0 |
| Kidney Ca, Clear cell type (OD04340) | 0.0 | Gastric Cancer 9060395 | 0.0 |
| Kidney Margin (OD04340) | 39.5 | Stomach Margin 9060394 | 0.0 |
| Kidney Ca, Nuclear grade 3 (OD04348) | 0.0 | Gastric Cancer 064005 | 0.0 |

**Table FG. Panel 4.1D**

| **Tissue Name** | **Rel. Exp.(%) Ag3243, Run 202013056** | **Tissue Name** | **Rel. Exp.(%) Ag3243, Run 202013056** |
|---|---|---|---|
| Secondary Th1 act | 0.0 | HUVEC IL-1beta | 0.0 |
| Secondary Th2 act | 0.0 | HUVEC IFN gamma | 1.6 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEG IL -11 | 0.0 |
| Secondary Tr1 rest | 0.0 | Lung Microvascular EC none | 0.0 |
| Primary Th1 act | 0.0 | Lung Microvascular EC TNFalpha +IL-1beta | 0.0 |
| Primary Th2 act | 0.0 | Microvascular Dermal EC none | 0.0 |
| Primary Tr1 act | 0.0 | Microsvasular Dermal EC TNF alpha+IL-1beta | 0.0 |
| Primary Th1 rest | 0.0 | Bronchial epithelium TNFalpha+ IL1beta | 0.0 |
| Primary Th2 rest | 0.0 | Small airway epithelium none | 0.0 |
| Primary Tr1 rest | 0.0 | Small airway epithelium TNFalpha +IL-1beta | 0.0 |
| CD45RA CD4 lymphocyte | 0.0 | Coronery artery SMC rest | 0.0 |
| CD45RO CD4 lymphocyte act | 0.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest | 0.0 |
| CD4-lymphocyte none | 0.0 | KU-812(Basophil) PMA/ionomycin | 0.0 |
| 2ry Th1/Th2/Tr1_anti-CD95 CH11 | 0.0 | CCD1106 (Keratinocytes) none | 0.0 |
| LAK cells rest ' | 0.0 | CCD1106(Keratinocytes) TNFfalpha + IL-1beta | 0.0 |
| LAK cells IL-2 | 0.0 | Liver cirrhosis | 0.0 |
| LAK cells IL-2+IL-12 | 0.0 | NCI-H292 none | 0.0 |
| LAK cells IL-2+IFN gamma | 0.0 | NCI-H292 IL-4 | 0.0 |
| LAK cells IL-2+ IL-18 | 0.0 | NCI-H292 IL-9 | 0.0 |
| LAK cells PMA/ionomycin | 0.0 | NCl-H292 IL-13 | 0.0 |
| NK Cells IL-2 rest | 0.0 | NCI-H292 IFN gamma | 0.0 |
| Two Way MLR 3 day | 0.0 | HPAEC none | 0.0 |
| Two Way MLR 5 day | 0.0 | HP ABC TNF alpha + IL-1 beta | 0.0 |
| Two Way MLR 7 day | 0.0 | Lung fibroblast none | 0.0 |
| PBMC rest | 0.0 | Lung fibroblast TNF alpha + IL-1 | 0.0 |
| PBMC PWM | 0.0 | Lung fibroblast IL-4 | 0.0 |
| PBMC PHA-L | 0.0 | Lung fibroblast IL-9 | 0.0 |
| Ramos (B cell) none | 0.0 | Lung fibroblast IL-13 | 0.0 |
| Ramos (B cell) ionomycin | 0.0 | Lung fibroblast IFN gamma | 0.0 |
| B lymphocytes PWM | 0.0 | Dermal fibroblast CCD1070 rest | 0.0 |
| B lymphocytes CD40L and IL-4 | 0.0 | Dermal fibroblast CCD1070 TNF alpha | 0.0 |
| EOL-1 dbcAMP | 0.0 | Dermal fibroblast CCD1070IL-1 beta | 0.0 |
| EOL-1 dbcAMP PMA/ionomycin | 0.0 | Dermal fibroblast IFN gamma | 0.0 |
| Dendritic cells none | 0.0 | Dermal fibroblast IL-4 | 0.0 |
| Dendritic cells LPS | 0.0 | Dermal Fibroblasts rest | 0.0 |
| Dendritic cells anti-CD40 | 0.0 | Neutrophils TNFa+LPS | 0.0 |
| Monocytes rest | 0.0 | Neutrophils rest | 0.0 |
| Monocytes LPS | 0.0 | Colon | 0.0 |
| Macrophages rest | 0.0 | Lung | 7.2 |
| Macrophages LPS | 0.0 | Thymus | 0.0 |
| HUVEC none | 0.0 | Kidney | 100.0 |
| HUVEC starved | 0.0 | | |

**Table FH. Panel 4D**

| **Tissue Name** | **Rel. Exp.(%) Ag3243, Run 164390552** | **Tissue Name** | **Rel.Exp.(%)Ag3243,Run 164390552** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha+IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha + IL-1 beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest + IL-1beta | 0.0 | Small airway epithelium TNFalpha | 0.0 |
| CD45RA CD4 lymphocyte act | 0.0 | Coronery artery SMC rest | 0.0 |
| CD45R0 CD4 lymphocyte act | 0.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFatpha + IL-1beta | 0.0 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1_anti-CD95 , CH11 | 0.0 | CCD1106(Keratinocytes)none | 0.0 |
| LAK cells rest | 0.0 | CCD1106 (Keratinocytes) TNFalpha + IL-1beta | 0.0 |
| LAK cells IL-2 | 0.0 | Liver cirrhosis | 0.0 |
| LAK cells IL-2+IL-12 | 0.0 | Lupus kidney | 0.0 |
| LAK cells IL-2+IFN gamma | 0.0 | NCI-H292 none | 0.0 |
| LAK cells IL-2+ IL-18 | 0.0 | NCI-H292 IL-4 | 0.0 |
| LAK cells PMA/ionomycin | 0.0 | NCI-H292 IL-9 | 0.0 |
| NK Cells IL-2 rest | 0.0 | NCI-H292 IL-13 NCI-H292 IL-13 | 0.0 |
| Two Way MLR 3 day | 0.0 | NCI-H292 IFN gamma | 0.0 |
| Two Way MLR 5 day | 0.0 | HPAEC none | 0.0 |
| Two Way MLR 7 day | 0.0 | HPAEC TNF alpha+IL-1 beta | 0.0 |
| PBMC rest | 0.0 | Lung fibroblast none | 0.0 |
| PBMC PWM | 0.0 | Lung fibroblast TNF alpha + IL-1 | 0.0 |
| PBMC PHA-L | 0.0 | Lung fibroblast IL-4 | 0.0 |
| Ramos (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 0.0 | Lung fibroblast IFN gamma | 0.0 |
| B lymphocytes CD40L and IL-4 | 0.0 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 0.0 | Dermal Bbroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 0.0 |
| Monocytes rest | 0.0 | IBD Crohn's | 0.0 |
| Monocytes LPS | 0.0 | Colon | 3.2 |
| Macrophages rest | 0.0 | Lung | 18.2 |
| Macrophages LPS | 0.0 | Thymus | 100.0 |
| HUVEC none | 0.0 | Kidney | 3.8 |
| HUVEC starved | 0.0 | | |

**CNS_neurodegeneration_v1.0 Summary:** Ag3243 This panel confirms the expression of the CG57452-01 gene at low levels in the brain in an independent group of individuals. Interestingly, expression of this gene appears to be slightly down-regulated in the temporal cortex of Alzheimer's disease patients. Therefore, up-regulation of this gene or its protein product may be of use in reversing the dementia/memory loss and neuronal death associated with Alzheimer's disease.

**General_screening_panel_v1.4 Summary:** Ag3243 Expression of the CG57452-01 gene is highest in fetal lung (CT = 30) and fetal kidney (CT= 30.4). Therefore, expression of this gene may be used to distinguish these samples from the other samples on this panel.

This gene is also expressed at moderate levels throughout the central nervous system (including in the amygdala, hippocampus, substantia nigra, thalamus, cerebellum, cerebral cortex, and spinal cord) and in one brain cancer line. This gene encodes a protein that is almost identical to protocadherin 15, a cell-adhesion molecule and member of the cadherin family of proteins. Cadherins can act as axon guidance and cell adhesion proteins, specifically during development and in the response to injury (ref. 1-2). Therefore, manipulation of levels of this protein may be of use in inducing a compensatory synaptogenic response to neuronal death in Alzheimer's disease, Parkinson's disease, Huntington's disease, spinocerebellar ataxia, progressive supranuclear palsy, ALS, head trauma, stroke, or any other disease/condition associated with neuronal loss.

Among tissues with metabolic or endocrine function, this gene is moderately expressed in pituitary gland and adrenal gland. This expression suggests that this gene product may play a role in normal metabolic and neuroendocrine function and that dysregulated expression of this gene may contribute to metabolic diseases (such as obesity and diabetes) or neuroendocrine disorders.

### References:

### 1. Ranscht B. (2000) Cadherins: molecular codes for axon guidance and synapse formation. Int. J. Dev. Neurosci. 18: 643-651.

The formation of the myriad of neuronal connections within the vertebrate nervous system relies on expression of molecular tags that match extending axon populations with synaptic target sites. Recent work suggests that cadherins, a group of calcium-dependent cell adhesion molecules, are candidates to serve such a role. The diversity of the cadherin family in the nervous system allows for a multitude of interactions to specify neuronal connections. Specific cadherin types demarcate subpopulations of developing axons that interconnect within neuronal circuits. Expression of different cadherin species at select synapse populations raises exciting prospects for this molecule class in controlling adhesive interactions during synaptogenesis and plasticity. Regulation of cadherin-mediated adhesive strength is an attractive mechanism to explain the different cadherin functions in axon growth and at synapses.

### PMID: 10978842

### 2. Hilschmann N, Bamikol HU, Barnikol-Watanabe S, Gotz H, Kratzin H, Thinnes FP. The immunoglobulin-like genetic predetermination of the brain: the protocadherins, blueprint of the neuronal network. Naturwissenschaften 2001 Jan;88(1):2-12

The morphogenesis of the brain is governed by synaptogenesis. Synaptogenesis in turn is determined by cell adhesion molecules, which bridge the synaptic cleft and, by homophilic contact, decide which neurons are connected and which are not. Because of their enormous diversification in specificities, protocadherins (pcdh alpha, pcdh beta, pcdh gamma), a new class of cadherins, play a decisive role. Surprisingly, the genetic control of the protocadherins is very similar to that of the immunoglobulins. There are three sets of variable (V) genes followed by a corresponding constant (C) gene. Applying the rules of the immunoglobulin genes to the protocadherin genes leads, despite of this similarity, to quite different results in the central nervous system. The lymphocyte expresses one single receptor molecule specifically directed against an outside stimulus. In contrast, there are three specific recognition sites in each neuron, each expressing a different protocadherin. In this way, 4,950 different neurons arising from one stem cell form a neuronal network, in which homophilic contacts can be formed in 52 layers, permitting an enormous number of different connections and restraints between neurons. This network is one module of the central computer of the brain. Since the V-genes are generated during evolution and V-gene translocation during embryogenesis, outside stimuli have no influence on this network. The network is an inborn property of the protocadherin genes. Every circuit produced, as well as learning and memory, has to be based on this genetically predetermined network. This network is so universal that it can cope with everything, even the unexpected. In this respect the neuronal network resembles the recognition sites of the immunoglobulins.

### PMID: 11261353

**Panel 1.2 Summary:** Ag878 The CG57452-01 gene encodes a protein that is almost identical to protocadherin 15, a cell-adhesion molecule and member of the cadherin family of proteins. Expression of this gene is highest in pituitary gland (CT = 28.0) and adrenal gland (CT = 28.6). Moderate expression of this gene is also seen in kidney, brain, and lung, consistent with what has been recently reported for the protocadherin 15 gene (ref 1). Expression of this gene is seen throughout the central nervous system, including in amygdala, hippocampus, substantia nigra, thalamus, cerebellum, cerebral cortex, and spinal cord. Mutations of the protocadherin gene PCDH15 have recently been shown to cause Usher syndrome type 1F (ref 2). Therefore, therapeutic modulation of the CG57452-01 protocadherin 15 protein may be of benefit in the treatment of Usher syndrome type 1F, deafness and retinitis pigmentosa. These results are consistent with what is observed in General_screening_panel_v1.4; please see this panel for additional information regarding the potential role of the protocadherin 15 gene in the CNS and metabolic/endocrine function.

### References:

### 1. Murcia CL, Woychik RP. Expression ofPcdh15 in the inner ear, nervous system and various epithelia of the developing embryo. Mech Dev 2001 Jul; 105(1-2):163-6.

Protocadherin 15 (Pcdh15) is associated with the Ames waltzer mutation in the mouse. Here we describe where the Pcdh15 gene is expressed at specific times during mouse development using RNA in situ hybridization. The expression ofPcdh15 is found in the sensory epithelium in the developing inner ear, in Rathke's pouch, and broadly throughout the brain with the highest level of expression being detected at embryonic day 16 (E16). Pcdh15 transcripts are also found in the developing eye, dorsal root ganglion, and the dorsal aspect of the neural tube, floor plate and ependymal cells adjacent to the neural canal. Additionally, expression is also detected in the developing glomeruli of the kidney, surface of the tongue, vibrissae, bronchi of the lung, and in the epithelium of the olfactory apparatus, gut and lung.

### 2. Ahmed ZM, Riazuddin S, Bernstein SL, Ahmed Z, Khan S, Griffith AJ, Morell RJ, Friedman TB, Riazuddin S, Wilcox ER. Mutations of the protocadherin gene PCDH15 cause Usher syndrome type 1F. Am J Hum Genet 2001 Jul;69(1):25-34

Human chromosome 10q21-22 harbors USH1F in a region of conserved synteny to mouse chromosome 10. This region of mouse chromosome 10 contains Pcdh15, encoding a protocadherin gene that is mutated in ames waltzer and causes deafness and vestibular dysfunction. Here we report two mutations of protocadherin 15 (PCDH15) found in two families segregating Usher syndrome type 1F. A Northern blot probed with the PCDH15 cytoplasmic domain showed expression in the retina, consistent with its pathogenetic role in the retinitis pigmentosa associated with USH1F.

### PMID: 11398101

**Panel 2.2 Summary:** Ag3243 Expression of this gene is seen at low levels in normal lung and kidney. Interestingly, expression is much lower in lung and kidney tumor samples than in the matched adjacent normal tissue. Therefore, expression of this gene could be used to distinguish normal lung and kidney from lung and kidney tumors. Furthermore, therapeutic modulation of the activity or amount of this gene product using protein therapeutics may be of benefit in the treatment of lung and kidney cancer.

**Panel 4.1D Summary:** Ag3243 The CG57452-01 gene is only expressed at detectable levels in the kidney (CT = 32.4). Therefore, antibody or small molecule therapies designed with the protein encoded for by this gene could modulate kidney function and be important in the treatment of inflammatory or autoimmune diseases that affect the kidney, including lupus and glomerulonephritis.

**Panel 4D Summary**: Ag3243 The CG57452-01 transcript is expressed at significant levels only in the thymus (CT = 33.1) in both runs. The putative protocadherin encoded for by the CG57452-01 gene could therefore play an important role in T cell development. Small molecule therapeutics, or antibody therapeutics designed against the protocadherin encoded for by this gene could be utilized to modulate immune function (T cell development) and be important for organ transplant, AIDS treatment or post chemotherapy immune reconstitiution.

### NOV9

Expression of NOV9/CG57625-01 was assessed using the primer-probe set Ag3297, described in Table GA. Results of the RTQ-PCR runs are shown in Tables GB, GC, GD and GE.

**Table GA. Probe Name Ag3297**

| **Primers** | **Sequences** | **Length** | **Start Position** |
|---|---|---|---|
| Forward | 5'-tgtgaatgtggaggatgctaa-3' | 21 | 4696 |
| Probe | TET-5'-tgatcacagtccttattttaccaaca-3'-TAMRA | 28 | 4717 |
| Reverse | 5'-gattcaaacacagacgcttca-3' | 21 | 4750 |

**Table GB. CNS_neurodegeneration_v1.0**

| **Tissue Name** | **Rel. Exp.(%) Ag3297, Run 210063001** | **Tissue Name** | **Rel.Exp.(%) Ag3297, Run 210063001** |
|---|---|---|---|
| AD 1 Hippo | 4.5 | Control (Path) 3 Temporal Ctx | 1.7 |
| AD 2 Hippo | 10.2 | Control (Path) 4 Temporal | 18.4 |
| AD 3 Hippo | 2.3 | AD 1 Occipital Ctx | 9.3 |
| AD 4 Hippo | 2.1 | AD 2 Occipital Ctx (Missing) | 0.0 |
| AD 5 hippo | 100.0 | AD 3 Occipital Ctx | 2.5 |
| AD 6 Hippo | 22.1 | AD 4 Occipital Ctx | 12.1 |
| control 2 Hippo | 19.1 | AD 5 Occipital Ctx | 32.1 |
| Control 4 Hippo | 3.2 | AD 6 Occipital Ctx | 44.8 |
| Control (Path) 3 Hippo | 2.6 | Control 1 Occipital Ctx | 0.5 |
| AD 1 Temporal Ctx | 4.5 | Control 2 Occipital Ctx | 43.5 |
| AD 2 Temporal Ctx | 16.7 | Control 3 Occipital Ctx | 11.5 |
| AD 3 Temporal Ctx | 1.5 | Control 4 Occipital Ctx | 2.4 |
| AD 4 Temporal Ctx | 12.6 | Control (Path) 1 Occipital Ctx | 49.7 |
| AD 5 Inf Temporal Ctx | 72.7 | Control (Path) 2 Occipital Ctx | 10.0 |
| AD 5 SupTemporal Ctx | 35.8 | Control (Path) 3 Occipital Ctx | 0.6 |
| AD 6 Inf Temporal Ctx | 21.3 | Control (Path) 4 Occipital Ctx | 11.3 |
| AD 6 Sup Temporal Ctx | 27.7 | Control 1 Parietal Ctx | 4.3 |
| Control 1 Temporal Ctx | 3.1 | Control 2 Parietal Ctx | 25.0 |
| Control 2 Temporal Ctx | 22.1 | Control 3 Parietal Ctx | 11.7 |
| Control 3 Temporal Ctx | 9.9 | Control (Path) 1 Parietal Ctx | 51.1 |
| Control 4 Temporal Ctx | 5.9 | Control (Path) 2 Parietal Ctx | 22.4 |
| Control (path) 1 Temporal Ctx | 46.3 | Control (Path) 3 Parietal Ctx | 1.8 |
| Control (Path) 2 Temporal Ctx | 27.7 | Control (Path) 4 Parietal Ctx | 24.7 |

**Table GC. General_sereening_panel_vl.4**

| **Tissue Name** | **Rel. Exp.(%) Ag3297, Run 215669728** | **Tissue Name** | **Rel. Exp.(%) Ag3297, Run 215669728** |
|---|---|---|---|
| Adtpose | 0.9 | Renal ca. TK-10 | 0.0 |
| Melanoma* Hs688(A).T | 1.5 | Bladder | 1.1 |
| Melanoma* Hs688(B).T | 3.8 | Gastric ca. (liver met) NCI-N87 | 6.9 |
| Melanoma* M14 | 0.0 | Gastric ca, KATO III | 0.0 |
| Metanoma'LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.*(SW480 met) SW620 | 0.0 |
| Testis Pool | 2.9 | Colon ca. HT29 | 0.0 |
| Prostate ca.*(bone met) PC-3 | 0.0 | Colon ca. HCT-116 | 0.0 |
| Prostate Pool | 6.8 | Colon ca. CaCo-2 | 0.0 |
| Placenta | 0.1 | Colon cancer tissue | 0.7 |
| Uterus Pool | 0.9 | Colon ca. SW1116 | 0.0 |
| Ovarian ca. OVCAR-3 | 4.8 | Colon ca. Colo-205 | 0.0 |
| Ovarian ca. SK-OV-3 | 1.0 | Colon ca.SW-48 | 0.0 |
| Ovarian ca. OVCAR-4 | 8.7 | Colon Pool | 1.7 |
| Ovarian ca. OVCAR-5 | 0.2 | Small Intestine Pool | 1.8 |
| Ovarian ca. IGROV-1 | 1.8 | Stomach Pool | 0.9 |
| Ovarian ca. OVCAR-8 | 0.9 | Bone Marrow Pool | 0.5 |
| Ovary | 0.6 | Fetal Heart | 1.6 |
| Breast ca. MCF-7 | 0.0 | Heart Pool | 0.7 |
| Breast ca. MDA-MB-231 | 0.0 | Lymph Node Pool | 5.2 |
| Breast ca. BT 549 | 0.0 | Fetal Skeletal Muscle | 0.5 |
| Breast ca. T47D | 0.2 | Skeletal Muscle Pool | 0.2 |
| Breast ca. MDA-N | 1.2 | Spleen Pool | 0.2 |
| Breast Pool | 1.2 | Thymus Pool | 1.7 |
| Trachea | 0.6 | CNS cancer (gHo/astro)U87- | 0.0 |
| Lung | 1.4 | CNS cancer (glio/astro) 118-MG | 0.2 |
| Fetal Lung | 99.3 | CNS cancer (neuro;met) SK-N-AS | 0.0 |
| Lung ca. NCI-N417 | 0.0 | CNS cancer (astro) SF-539 | 2.0 |
| Lung ca.LX-1 | 0.0 | CNS cancer (astro) SNB-75 | 6.9 |
| Lung ca. NCI-H146 | 0.1 | CNS cancer (glio) SNB-19 | 1.9 |
| Lung ca. SHP-77 | 7.0 | CNS cancer (glio) SF-295 | 0.0 |
| Lung ca. A549 | 2.0 | Brain (Amygdala) Pool | 14.3 |
| Lung ca.NCI-H526 | 40.6 | Brain (cerebellum) | 3.4 |
| Lung ca. NCI-H23 | 0.6 | Brain(fetal) | 100.0 |
| Lung ca. NCI-H460 | 0.0 | Brain (Hippocampus) Pool | 14.7 |
| Lung ca. HOP-62 | 0.0 | Cerebral Cortex Pool | 23.8 |
| Lung ca. NCI-H522 | 0.0 | ' Brain (Substantia nigra) Pool | 15.2 |
| Liver | 0.1 | Brain (Thalamus) Pool | 21.3 |
| Fetal Liver | 1.4 | Brain (whole) | 23.3 |
| Liver ca. HepG2 | 0.0 | Spinal Cord Pool | 7.5 |
| Kidney Pool | 3.4 | Adrenal Gland | 0.2 |
| Fetal Kidney | 12.3 | Pituitary gland Pool | 1.7 |
| Renal ca. 786-0 | 0.1 | Salivary Gland | 0.0 |
| Renal ca. A498 | 0.9 | Thyroid (female) | 0.1 |
| Renal ca. ACHN | 0.0 | Pancreatic ca. CAPAN2 | 0.0 |
| Renal ca. UO-31 | 0.5 | Pancreas Pool | 0.5 |

**Table GD. Panel 4D**

| **Tissue Name** | **Rel. Exp.(%) Ag3297, Run 164633943** | **Tissue Name** | **Rel. Exp.(%) Ag3297, Run 164633943** |
|---|---|---|---|
| Secondary Th1 act | 0.1 | HUVEC IL-1beta | 0.1 |
| Secondary Th2 act | 0.2 | HUVEC IFN gamma | 1.3 |
| Secondary Tr1 act | 0.4 | HUVEC TNF alpha + IFN gamma | 0.5 |
| Secondary Th1 rest | 0.4 | HUVEC TNF alpha + IL4 | 0.4 |
| Secondary Th2 rest | 0.0 | HUVEC IL-11 | 0.2 |
| Secondary Tri rest | 0.1 | Lung Microvascular EC none | 0.6 |
| Primary Th 1 act | 0.1 | Lung Microvascular EC TNFalpha + IL-1beta | 0.1 |
| PrimaryTh2 act | 0.5 | Mierovascular DermalEC none | 1.2 |
| Primary Tr1 act | 0.4 | Microsvasular Dermal EC TNFalpha+IL-1beta | 0.2 |
| Primary Th1 rest | 0.3 | Bronchial epithelium TNFalpha + IL-1beta | 0.8 |
| Primary Th2 rest | 0.1 | Small airway epithelium none | 0.7 |
| Primary Tr1 rest | 0.0 | Small airway epithelium TNFalpha + IL-1beta | 1.9 |
| CD45RA CD4 lymphocyte act | 0.2 | Coronery artery SMC rest | 2.2 |
| CD45RO CD4 lymphocyte act | 0.1 | Coronery artery SMC TNFalpha + IL-1beta | 2.3 |
| CD8 lymphocyte act | 0.1 | Astrocytes rest | 100.0 |
| Secondary CD8 lymphocyte rest | 1.1 | Astrocytes TNFalpha+IL-1beta | 28.9 |
| Secondary CD8 lymphocyte act | 1.6 | KU-812 (Basophil) rest | 2.9 |
| CD4 lymphocyte none | 0.4 | KU-812(Basophil) PMA/ionomycin | 15.2 |
| 2ry Th1/Th2/Tr1_anti-CD95 CH11 | 1.5 | CCD1106 (Keratinocytes) none | 0.1 |
| LAK cells rest | 0.4 | CCD1106 (Keratinocytes) TNFalpha + IL-1beta | 0.5 |
| LAK cells IL-2 | 1.7 | Liver cirrhosis | 2.6 |
| LAK cells IL-2+IL-12 | 1.4 | Lupus kidney | 2.0 |
| LAK cells IL-2+IFN gamma | 3.5 | NCI-H292 none | 1.0 |
| LAK cells IL-2+ IL-18 | 1.1 | NCI-H292 IL-4 | 4.7 |
| LAK cells PMA/ionomycin | 0.1 | NCI-H292 IL-9 | 1.1 |
| NK Cells IL-2 rest | 0.8 | NCI-H292 IL-13 | 1.7 |
| Two Way MLR 3 day | 0.3 | NCI-H292 IFN gamma | 0.6 |
| Two Way MLR 5 day | 1.0 | HPAEC none | 0.6 |
| Two Way MLR 7 day | 0.4 | HPAEC TNF alpha + IL-1 beta | 0.2 |
| PBMC rest | 0.3 | Lung fibroblast none | 8.7 |
| PBMCPWM | 1.0 | Lung fibroblast TNF alpha +IL-1 beta | 2.3 |
| PBMCPHA-L | 1.0 | Lung fibroblast IL-4 | 18.7 |
| Ramos (B cell) none | 0.7 | Lung fibroblast IL-9 | 18.8 |
| Ramos (B cell) ionomycin | 2.2 | Lung fibroblast IL-13 | 14.7 |
| B lymphocytes PWM | 1.2 | Lung fibroblast IFN gamma | 12.2 |
| B lymphocytes CD40L and IL-4 | 2.1 | Dermal fibroblast CCD 1070 rest | 18.4 |
| EOL-1 dbcAMP | 0.5 | Dermal fibroblast CCD1070 TNF alpha | 16.2 |
| EOL-1 dbcAMP PMA/ionomycin | 0.4 | Dermal fibroblast CCD1070 IL-1 beta | 3.1 |
| Dendritic cells none | 0.4 | Dermal fibroblast IFN gamma | 0.3 |
| Dendritic cells LPS | 0.3 | Dermal fibroblast IL-4 | 0.0 |
| Dendritic cells anti-CD40 | 1.3 | IBD Colitis 2 | 1.2 |
| Monocytes rest | 0.4 | IBD Crohn's | 0.5 |
| Monocytes LPS | 0.3 | Colon | 0.3 |
| Macrophages rest | 0.4 | Lung | 5.5 |
| Macrophages LPS | 0.7 | Thymus | 2.2 |
| HUVEC none | 0.8 | Kidney | 4.4 |
| HUVEC starved | 6.9 | | |

**Table GE. Panel CNS_1.1**

| **Tissue Name** | **Rel. Exp.(%) Ag3297, Run 204173323** | **Tissue Name** | **Rel. Exp.(%) Ag3297, Run 204173323** |
|---|---|---|---|
| Cing Gyr Depression2 | 5.1 | BA17PSP2 | 14.9 |
| Cing Gyr Depression | 10.1 | BA17PSP | 46.3 |
| Cing Gyr PSP2 | 1.5 | BA17 Huntington's2 | 10.1 |
| Ping Gyr PSP | 11.8 | BA17 Huntington's | 32.3 |
| Cing Gyr Huntington's2 | 13.4 | BA17 Parkinson's2 | 54.7 |
| Cing Gyr Huntington's | 48.3 | BA17 Parkinson's | 37.6 |
| Cing Gyr Parkinson's2 | 37.4 | BA17 Alzheimer's2 | 7.0 |
| Cing Gyr Parkinson's | 47.3 | BA17 Control2 | 48.3 |
| Cing Gyr Alzheimer's2 | 4.5 | BA17 Control | 69.3 |
| Cing Gyr Alzheimer's | 16.3 | BA9 Depression2 | 6.6 |
| Cing Gyr Control2 | 30.8 | BA9 Depression | 9.2 |
| Cing Gyr Control | 66.0 | BA9 PSP2 | 6.5 |
| Temp Pole Depression2 | 5.6 | BA9 PSP | 18.4 |
| Temp Pole PSP2 | 3.0 | BA9 Huntington's2 | 8.7 |
| Temp Pole PSP | 4.4 | BA9 Huntington's | 61.6 |
| Temp Pole Huntington's | 22.1 | BA9 Parkinson's2 | 64.6 |
| Temp Pole ParMnson's2 , | 24.8 | BA9 Parkinson's | 46.7 |
| Temp Pole Parkinson's | 32.8 | BA9 Alzheimetr's2 | 11.3 |
| Temp Pole Alzheimer's2 | 3.3 | BA9 Alzheimer's | 7.4 |
| Temp Pole Alzheimer's | 5.1 | BA9.Control2 | 69.3 |
| Temp Pole Conytrol2 | 51.8 | BA9 Control | 27.4 |
| Temp Pole Control | 27.5 | BA7 Depression | 10.9 |
| Glob Palladus Depression | 3.6 | BA7 PSP2 | 27.9 |
| Glob Palladus PSP2 | 5.5 | BA7 PSP | 56.3 |
| Glob Palladus PSP | 6.7 | BA7 Huntington's2 | 21.3 |
| Glob Palladus Parkinon's2 | 11.7 | BA7 Huntington's | 49.7 |
| Glob Palladus Parkinson's | 97.9 | BA7 Parkinson's2 | 50.7 |
| Glob Palladus Alzheimer's2 | 10.9 | BA7 Parkinson's | 20.3 |
| Glob Palladus Alzheimer's | 9.8 | BA7 Alzheimer's2 | 4.3 |
| Glob Palladus Control2 | 24.5 | BA7 Control2 | 25.3 |
| Glob Palladus Control | 23.2 | BA7 Control | 45.4 |
| Sub Nigra Depression2 | 5.9 | BA4 Depression2 | 6.4 |
| Sub Nigra Depression | 3.3 | BA4 Depression | 14.5 |
| Sub Nigra PSP2 | 5.1 | BA4 PSP2 | 36.3 |
| Sub Nigra Huntington's2 | 5.8 | BA4 PSP | 12.0 |
| Sub Nigra Huntington's | 74.2 | BA4 Huntington's2 | 5.4 |
| Sub Nigra Parkinson's2 | 39.5 | BA4 Huntington's | 42.0 |
| Sub Nigra Alzheimer's2 | 3.8 | BA4 Parkinson's2 | 100.0 |
| Sub Nigra Control2 | 12.7 | BA4 Parkinson's | 52.9 |
| Sub Nigra Control | 45.1 | BA4 Alzheimer's2 | 3.0 |
| BA17 Depression2 | 11.0 | BA4 Control2 | 53.2 |
| BA17 Depression | 7.1 | BA4 Control | 36.3 |

**CNS_neurodegeneration_v1.0 Summary:** Ag3297 This panel confirms the expression of this gene at low levels in the brain in an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel_v1.4 Summary:** Ag3297 The CG57625-01 gene is expressed at low to moderate levels in many of the tissues on this panel, with the highest levels of expression in fetal brain and fetal lung (CT = 27.4). Interestingly, the levels of expression are significantly lower in the sample from adult lung (CT = 33.5) than in fetal lung. Therefore, expression of this gene can be used to distinguish fetal lung from adult lung.

This gene is expressed at low levels throughout the CNS, including in amygdala, substantia nigra, thalamus, cerebellum, cerebral cortex, and spinal cord. The CG57625-01 gene encodes a protein with homology to protocadherin, a cell-adhesion molecule and member of the cadherin family of proteins. Cadherins can act as axon guidance and cell adhesion proteins, specifically during development and in the response to injury (ref. 1-2). Therefore, manipulation of levels of this protein may be of use in inducing a compensatory synaptogenic response to neuronal death in Alzheimer's disease, Parkinson's disease, Huntington's disease, spinocerebellar ataxia, progressive supranuclear palsy, ALS, head trauma, stroke, or any other disease/condition associated with neuronal loss.

This gene is also expressed at low to moderate levels in a number of tissues with metabolic or endocrine function, including heart, gastrointestinal tract, fetal liver, pituitary gland, and adipose. Therefore, therapeutic modulation of the activity of this gene may prove useful in the treatment of endocrine/metabolically related diseases, such as obesity and diabetes.

Additionally, this gene is also expressed at significant levels in lung cancer, ovarian cancer, gastric cancer and melanoma cell lines. Hence expression of this gene could be used as a diagnostic marker for these types of cancers. Furthermore, therapeutic modulation of the activity of this gene or its protein product, using small molecule drugs, antibodies, or protein therapeutics, may be of use in the treatment of lung cancer, ovarian cancer, gastric cancer and melanoma.

### References:

### 1. Ranscht B. (2000) Cadherins: molecular codes for axon guidance and synapse formation. Int. J. Dev. Neurosci. 18: 643-651.

The formation of the myriad of neuronal connections within the vertebrate nervous system relies on expression of molecular tags that match extending axon populations with synaptic target sites. Recent work suggests that cadherins, a group of calcium-dependent cell adhesion molecules, are candidates to serve such a role. The diversity of the cadherin family in the nervous system allows for a multitude of interactions to specify neuronal connections. Specific cadherin types demarcate subpopulations of developing axons that interconnect within neuronal circuits. Expression of different cadherin species at select synapse populations raises exciting prospects for this molecule class in controlling adhesive interactions during synaptogenesis and plasticity. Regulation of cadherin-mediated adhesive strength is an attractive mechanism to explain the different cadherin functions in axon growth and at synapses.

### PMID: 10978842

### 2. Hilschmann N, Bamikol HU, Barnikol-Watanabe S, Gotz H, Kratzin H, Thinnes FP. The immunoglobulin-like genetic predetermination of the brain: the protocadherins, blueprint of the neuronal network. Naturwissenschaften 2001 Jan;88(1):2-12

The morphogenesis of the brain is governed by synaptogenesis. Synaptogenesis in turn is determined by cell adhesion molecules, which bridge the synaptic cleft and, by homophilic contact, decide which neurons are connected and which are not. Because of their enormous diversification in specificities, protocadherins (pcdh alpha, pcdh beta, pcdh gamma), a new class of cadherins, play a decisive role. Surprisingly, the genetic control of the protocadherins is very similar to that of the immunoglobulins. There are three sets of variable (V) genes followed by a corresponding constant (C) gene. Applying the rules of the immunoglobulin genes to the protocadherin genes leads, despite of this similarity, to quite different results in the central nervous system. The lymphocyte expresses one single receptor molecule specifically directed against an outside stimulus. In contrast, there are three specific recognition sites in each neuron, each expressing a different protocadherin. In this way, 4,950 different neurons arising from one stem cell form a neuronal network, in which homophilic contacts can be formed in 52 layers, permitting an enormous number of different connections and restraints between neurons. This network is one module of the central computer of the brain. Since the V-genes are generated during evolution and V-gene translocation during embryogenesis, outside stimuli have no influence on this network. The network is an inborn property of the protocadherin genes. Every circuit produced, as well as learning and memory, has to be based on this genetically predetermined network. This network is so universal that it can cope with everything, even the unexpected. In this respect the neuronal network resembles the recognition sites of the immunoglobulins.

### PMID: 11261353

**Panel 4D Summary:** A-g3297 Expression of the CG57625-01 gene is highest in resting astrocytes (CT = 28), consistent with the expression of this gene in the brain in General_screening_panel_v1.4. This gene is also moderately expressed in lung and dermal fibroblasts, irrespective of treatment. Therefore, therapeutic modulation of the activity of this gene or its protein product may be of benefit in the treatment of asthma, emphysema, and psoriasis. Low levels of expression of this gene are detected in a number of other samples on this panel.

**Panel CNS_1.1 Summary:** Ag3297 This panel confirms the expression of this gene at low to moderate levels in the brain in an independent group of individuals. Please see Panel 1.4 for a discussion of the potential utility of this gene in treatment of central nervous system disorders.

### NOV10

Expression ofNOV10/CGS7553-01 was assessed using the primer-probe set Ag3283, described in Table HA. Results of the RTQ-PCR runs are shown in Tables HB, HC and HD.

**Table HA. Probe Name Ag3283**

| **Primers:** | **Sequences** | **Length** | **Start Position** | **SEQ ID NO** |
|---|---|---|---|---|
| Forward | 5'-gcctccaatatgaaacttcaaa-3' | 22 | 461 | 276 |
| Probe | TET-5'-tcctcagctacgagtaagttctgtctca-3'-TAMRA | 28 | 504 | 277 |
| Reverse | 5'-cagaaccatcaggttgtgattt-3' | 22 | 532 | 278 |

**Table HB. CNS_neurodegeneration_vl.0**

| **Tissue Name** | **Rel. Exp.(%) Ag3283, Run 210060840** | **Tissue Name** | **Rel. Exp.(%) Ag3283, Run 210060840** |
|---|---|---|---|
| AD 1 Hippo | 13.3 | Control (Path) 3 Temporal Ctx | 4.5 |
| AD 2 Hippo | 14.1 | Control (Path) 4 Temporal Ctx | 44.1 |
| AD 3 Hippo | 15.4 | AD 1 Occipital Ctx | 19.1 |
| AD 4 Hippo | 7.6 | AD 2 Occipital Ctx (Missing) | 0.0 |
| AD 5 Hippo | 76.8 | AD 3 Occipital Ctx | 15.1 |
| AD 6 Hippo | 65.1 | AD 4 Occipital Ctx | 22.7 |
| Control 2 Hippo | 25.2 | AD 5 Occipital Ctx | 41.8 |
| Control 4 Hippo | 10.7 | AD 6 Occipital Ctx | 26.4 |
| Control (Path) 3 Hippo | 13.8 | Control 1 Occipital Ctx | 6.2 |
| AD 1 Temporal Ctx | 34.9 | Control 2 Occipital Ctx | 35.8 |
| AD 2 Temporal Ctx | 40.3 | Control 3 Occiptial Ctx | 23.7 |
| AD 3 Temporal Ctx | 9.2 | Control 4 Occipital Ctx | 11.1 |
| AD 4 Temporal Ctx | 18.4 | Control (Path) 1 Occipital Ctx | 90.1 |
| AD 5 Inf Temporal Ctx | 100.0 | Control (Path) 2 Occipital Ctx | 12.3 |
| AD 5 Sup Temporal Ctx | 51.1 | Control (Path) 3 Occipital Ctx | 5.3 |
| AD 6 Inf Temporal Ctx | 76.8 | Control (Path) 4 Occipital Ctx | 23.0 |
| AD 6 Sup Temporal Ctx | 93.3 | Control 1 Parietal Ctx | 2.9 |
| Control 1 Temporal Ctx | 6.7 | Control 2 Parietal Ctx | 65.1 |
| Control 2 Temporal Ctx | 36.3 | Control 3 Parietal Ctx | 18.3 |
| Control 3 Temporal Ctx | 13.1 | Control(Path)1 Parietal Ctx | 52.1 |
| Control 3 Temporal Ctx | 8.7 | Control (Path) 2 Parietal Ctx | 28.7 |
| Control (Path) 1 Temporal | 45.1 | Control (Path) 3 Parietal Ctx | 5.4 |
| Control (Path) 2 Temporal Ctx | 27.7 | Control (Path) 4 Parietal Ctx | 52.1 |

**Table HC. General_screening_panel-v1.4**

| **Tissue Name** | **Rel. Exp.(%) Ag3283, Run 216512996** | **Tissue Name** | **Rel. Exp.(%) Ag3283, Run 216512996** |
|---|---|---|---|
| Adipose | 8.3 | Renal ca. TK-10 | 29.3 |
| Melanoma* Hs688(A).T | 5.0 | Bladder | 25.2 |
| Melanoma* Hs688(B).T | 9.7 | Gastric ca. (liver met.) NCI-N87 | 40.9 |
| Melanoma* M14 | 21.6 | Gastric ca. KATO III | 48.3 |
| Melanoma* LOXIMVI | 12.8 | Colon ca. SW-948 | 9.1 |
| Melanoma* SK-MEL-5 | | Colon ca. SW480 | 28.3 |
| Squamous cell carcinoma SCC-4 | 8.4 | Colon ca.* (SW480 met) SW620 | 21.2 |
| Testis Pool | 20.3 | Colon ca.HT29 | 13.4 |
| Prostate ca.*(bone met) PC-3 | 16.4 | Colon ca. HCT-116 | 37.6 |
| Prostate Pool | 6.2 | Colon ca. CaCo-2 | 14.2 |
| Placenta | 1.0 | Colon cancer tissue | 13.9 |
| Uterus Pool | 4.1 | Colon ca. SW1116 | 2.6 |
| Ovarian ca. OVCAR-3 | 30.6 | Colon ca. Colo-205 | 10.8 |
| Ovarian ca. SK-OV-3 | 61.6 | Colon ca. SW-48 | 6.5 |
| Ovarian ca. OVCAR-4 | 4.9 | Colon Pool | 15.5 |
| Ovarian ca. OVCAR-5 | 28.5 | Small Intestine Pool | 21.5 |
| Ovarian ca. IGROV-1 | 16.0 | Stomach Pool | 13.3 |
| Ovarian ca. OVCAR-8 | 8.8 | Bone Marrow Pool | 5.4 |
| Ovary | 14.8 | Fetal Heart | 10.2 |
| Breast ca. MCF-7 | 24.5 | Heart Pool | 7.9 |
| Breast ca. MDA-MB-231 | 50.3 | Lymph Node Pool | 17.0 |
| Breast ca. BT 549 | 100.0 | Fetal Skeletal Muscle | 6.2 |
| Breast ca. T47D | 47.3 | Skeletal Muscle Pool | 17.7 |
| Breast ca.MDA-N | 14.5 | Spleen Pool | 17.2 |
| Breast Pool | 19.8 | Thymus Pool | 20.6 |
| Trachea | 7.3 | CNS cancer (glio/astro) U87-MG | 37.4 |
| Lung | 7.5 | CNS cancer (glio/astro) U-118-MG | 49.7 |
| Fetal Lung | 37.6 | CNS cancer (neuro; met) SK-N-AS | 80.7 |
| Lung ca.NCI-N417 | 4.6 | CNS cancer (astro) SF-S39 | 11.5 |
| Lung ca. LX-1 | 41.8 | CNS cancer (astro) SNB-75 | 36.3 |
| Lung ca. NCI-H146 | 11.3 | CNS cancer (glio) SNB-19 | 12.4 |
| Lung ca. SHP-77 | 26.6 | CNS cancer (glio) SF-295 | 29.5 |
| Lung ca. A549 | 18.8 | Brain (Amygdala) Pool | 6.3 |
| Lung ca. NCI-H526 | 13.9 | Brain (cerebellum) | 4.4 |
| Lung ca. NCI-H23 | 31.4 | Brain (fetal) | 43.8 |
| Lung ca. NCI-H460 | 17.6 | Brain (Hippocampus) Pool | 14.5 |
| Lung ca. HOP-62 | 16.2 | Cerebral Cortex Pool | 11.4 |
| Lung ca. NCI-H522 | 23.3 | Brain (Substantia nigra) Pool | 7.9 |
| Liver | 0.1 | Brain (Thalamus) Pool | 17.0 |
| Fetal Liver | 22.8 | Brain (whole) | 3.2 |
| Liver ca. HepG2 | 21.5 | Spinal Cord Pool | 11.5 |
| Kidney Pool | 23.3 | Adrenal Gland | 4.0 |
| Fetal Kidney | 40.9 | Pituitary gland Pool | 3.8 |
| Renal ca. 786-0 | 57.0 | Salivary Gland | 0.4 |
| Renal ca. A498 | 2.3 | Thyroid (female) | 5.1 |
| Renal ca. ACHN | 11.3 | Pancreatic ca. CAPAN2 | 51.1 |
| Renal ca. UO-31 | 15.0 | Pancreas Pool | 22.2 |

**Table HD. Panel 4D**

| **Tissue Name** | **Rel. Exp.(%) Ag3283, Run : 164634322** | **Tissue Name** | **Rel. Exp.(%) Ag3283, Run-164634322** |
|---|---|---|---|
| Secondary Th1 act | 6.9 | HUVEC IL-1beta | 7.2 |
| Secondary Th2 act | 7.3 | HUVEC IFN gamma | 10.7 |
| Secondary Tr1 act | 6.3 | HUVEC TNF alpha + IFN gamma | 2.9 |
| Secondary Th1 rest | 1.8 | HUVEC TNF alpha + IL4 | 5.8 |
| Secondary Th2 rest | | 3.4 HUVEC IL-11 | 3.5 |
| Secondary Tr1 rest | 4.7 | Lung Microvascular EC none | 8.8 |
| Primary Th1 act | 7.6 | Lung Microvascular EC TNFalpha + IL-1beta | 10.7 |
| Primary Th2 act | 5.5 | Microvascular Dermal EC none | 11.6 |
| Primary Tr1 act | 7.4 | Microsvasular Dermal EC TNFalpha + IL-1beta | 6.6 |
| Primary Th1 rest | 23.0 | Bronchial epithelium TNFalpha + IL1beta | 7.8 |
| Primary Th2 rest | 17.9 | Small airway epithelium none | 1.0 |
| Primary Trl rest | 8.2 | Small airway epithelium TNFalpha + IL-1beta | 18.4 |
| CD45RA CD4 lymphocyte act | 4.9 | Coronery artery SMC rest | 2.5 |
| CD45RO CD4 lymphocyte act | 8.2 | Coronery artery SMC TNFalpha + IL-1beta | 2.8 |
| CD8 lymphocyte act | 5.8 | Astrocytes rest | 5.9 |
| Secondary CD8 lymphocyte rest | 3.2 | Astrocytes TNFalpha+IL-1beta | 2.4 |
| Secondary CD8 lymphocyte act | 6.8 | KU-812 (Basophil) rest | 4.3 |
| CD4 lymphocyte none | 2.5 | KU-812 (Basophil) PMA/ionomycin | 21.8 |
| 2ry Th1/Th2/Tr1_anti-CD95 CH11 | 6.5 | CCD1106 (Keratinocytes) none | 6.6 |
| LAK cells rest | 14.2 | CCD1106 (Keratinocytes) TNFalpha + IL-1beta | 2.0 |
| LAK cells IL-2 | 14.2 | Liver cirrhosis | 1.5 |
| LAK cells IL-2+IL-12 | 7.4 | Lupus kidney | 1.5 |
| LAK cells IL-2+IFN gamma | 13.8 | NCI-H292 none | 17.4 |
| LAK cells IL-2 + IL-18 | 9.7 | NCI-H292 IL-4 | 18.9 |
| LAK cells PMA/ionomycin | 5.0 | NCI-H292 IL-9 | 23.5 |
| NK Cells IL-2 rest | 14.4 | NCI-H292 IL-13 | 5.3 |
| Two Way MLR 3 day | 19.2 | NCI-H292 IFN gamma | 8.5 |
| Two Way MLR 5 day | 5.6 | HPAEC none | 6.3 |
| Two Way MLR 7 day | 3.1 | HPAEC TNF alpha + IL-1 beta | 8.0 |
| PBMC rest | 7.4 | Lung fibroblast none | 4.7 |
| PBMC PWM | 24.8 | Lung fibroblast TNF alpha + IL-1 beta | 1.8 |
| PBMC PHA-L | 8.8 | Lung fibroblast IL-4 | 10.4 |
| Ramos (B cell) none | 26.4 | Lung fibroblast IL-9 | 6.7 |
| Ramos (B cell) ionomycin | 100.0 | Lung fibroblast IL-13 | 6.5 |
| B lymphocytes PWM | 22.7 | Lung fibroblast IFN gamma | 7.2 |
| B lymphocytes CD40L and IL-4 | 21.5 | Dermal fibroblast CCD1070 rest | 25.0 |
| EOL-1 dbcAMP | 15.3 | Dermal fibroblast CD1070 TNF alpha | 56.3 |
| EOL-1 dbcAMP PMA/ionomycin | 27.7 | Dermal fibroblast CCD1070 IL-1 beta | 7.5 |
| Dendritic cells none | 13.5 | Dermal fibrolast IFN gamma | 2.8 |
| Dendritic cells LPS | 6.8 | Dermal fibroblast IL-4 | 8.6 |
| Dendritic cells anti-CD40 | 12.3 | IBD Colitis 2 | 0.4 |
| Monocytes rest | 13.8 | IBD Crohn's | 1.8 |
| Monocytes LPS | 13.4 | Colon | 28.7 |
| Macrophages rest | 12.4 | Lung | 4.4 |
| Macrophages LPS | 10.3 | Thymus | 16.3 |
| HUVEC none | 7.3 | Kidney | 32.5 |
| HUVEC starved | 22.1 | | |

**CNS_neurodegeneration_v1.0 Summary:** Ag3283 This panel confirms the expression of the CG57553-01 gene at low to moderate levels in the brain in an independent group of individuals. Interestingly, this gene appears to be slightly upregulated in the temporal cortex of Alzheimer's disease patients. Therefore, therapeutic modulation of the activity of this gene or its protein product may be of use to decrease neuronal death in the treatment of Alzheimer's disease or other neurological disorders.

**General_screening_panel_v1.4 Summary:** Ag3283 Expression of the CG57553-01 gene is highest in a breast cancer cell line (CT= 27.2). This gene is expressed at low to moderate levels in a number of tissues with metabolic or endocrine function, including adipose, adrenal gland, gastrointestinal tract, pancreas, skeletal muscle and thyroid. Therefore, therapeutic modulation of the activity of this gene may prove useful in the treatment of endocrine/metabolically related diseases, such as obesity and diabetes. Interestingly, this gene is differentially expressed in adult (CT = 36) vs fetal liver (CT = 29) and may be useful for distinguishing adult and fetal liver.

This gene is also expressed at moderate levels throughout the CNS, including in amygdala, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

There are also significant levels of expression in clusters of cell lines derived from melanoma, prostate, ovarian, brain, breast, pancreatic, gastric, and liver cancers. This observation suggests that expression of this gene may be associated with these cancers. Therefore, therapeutic modulation of the activity of this gene or its protein product might be of use in the treatment of these cancers.

**Panel 4D Summary:** Ag3283 The CG57553-01 gene is expressed at low to moderate levels in a number cell types within this panel, with highest expression in activated B cells (CT = 27.2). Therefore, therapeutic modulation of the activity of this gene may prove useful in the treatment of rheumatic disease including rheumatoid arthritis, lupus, osteoarthritis, and hyperproliferative B cell disorders.

### NOV11

Expression of gene NOV11a/CG57488-01 and variants NOV11B/CG57488-02 and NOV11C/CG57488-03 was assessed using the primer-probe sets Ag3254 and Ag3339, described in Tables IA and IB. Results of the RTQ-PCR runs are shown in Tables IC, ID and IE. Please note that variant CG57488-03 is only recognized by primer-probe set Ag3339.

**Table IA. Probe Name Ag3254**

| **Primers** | **Sequences** | **Length** | **Start Position** | **SEQ ID NO** |
|---|---|---|---|---|
| Forward | 5'-cttgtgcctgtggttaacatct-3' | 22 | 5853 | 279 |
| Probe | TET-5'-tgatcactccaggatattgacacgaa-3'-TAMRA | 26 | 5892 | 280 |
| Reverse | 5'-accccagaacatgtgagtaaga-3' | 22 | 5931 | 281 |

**Table IB. Probe Name Ag3339**

| **Primers** | **Sequences** | **Length** | **Start Position** | **SEQ ID NO** |
|---|---|---|---|---|
| Forward | 5'-ggtcttctacgtcagggagaat-3' | 22 | 1806 | 282 |
| Probe | TET-5'-cagtttgcagtcgagaccttcttcga-3'-TAMRA | 26 | 1852 | 283 |
| Reverse | 5'-gctgaatacgtcactgaaacct-3' | 22 | 883 | 284 |

**Table IC. CNS_neurodegeneration_v1.0**

| **Tissue Name** | **Rel. Exp.(%) Ag3254, Run 209990706** | **Rel. Exp.(%) Ag3339, Run 210139045** | **Tissue Name** | **Rel. Exp.(%) Ag3254, Run 209990706** | **Rel. Exp.(%) Ag3339, Run 210139045** |
|---|---|---|---|---|---|
| AD 1 Hippo | 0.0 | 19.1 | Control (path) 3 Temporal Ctx | 28.9 | 6.2 |
| AD 2 Hippo | 38.2 | 70.7 | Control (Path) 4 Temporal Ctx | 0.0 | 3.7 |
| AD3 Hippo | 7.3 | 9.1 | AD 1 Occipital Ctx | 0.0 | 17.7 |
| AD 4 Hippo | 12.1 | 5.4 | AD 2 Occipital Ctx (Missing) | 0.0 | 0.0 |
| AD 5 Hippo | 53.2 | 253 | AD 3 Occipital Ctx | 18.2 | 19.2 |
| AD 6 Hippo | 5.8 | 100.0 | AD 4 Occipital Ctx | 59.9 | 11.7 |
| Control 2 Hippo | 39.2 | 17.7 | AD 5 Occipital Ctx | 38.2 | 12.3 |
| Control 4 Hippo | 58.6 | 6.9 | AD 6 Occipital Ctx | 0.0 | 22.5 |
| Control (Path) 3 Hippo | 16.2 | 10.6 | Control 1 Occipital Ctx | 0.0 | 7.2 |
| AD 1 Temporal Ctx | 0.0 | 6.1 | Control 2 Occipital Ctx | 68.8 | 31.2 |
| AD 2 Temporal Ctx | 49.0 | 14.7 | Control 3 Occipital Ctx | 23.3 | 18.0 |
| AD3 Temporal Ctx | 8.5 | 3.1 | Control 4 Occipital Ctx | 27.2 | 31.4 |
| AD4 Temporal Ctx | 57.0 | 4.5 | Control (Path) 1 Occipital Ctx | 33.4 | 34.2 |
| AD 5 Inf Temporal Ctx | 89.5 | 53.6 | Control (Path) 2 Occipital Ctx | 11.7 | 4.6 |
| AD 5 Sup Temporal Ctx | 60.7 | 62.4 | Control (Path) 3 Occipital Ctx | 100.0 | 4.3 |
| AD 6 Inf Ctx Temporal Ctx | 0.0 | 31.6 | Control (Path) 4 Occipital Ctx | 0.0 | 10.4 |
| AD 6 Sup Temporal Ctx | 0.0 | 22.4 | Control 1 Parietal Ctx | 13.0 | 4.7 |
| Control 1 Temporal Ctx | 0.0 | 2.6 | Control 2 Parietal Ctx | 61.6 | 20.6 |
| Control 2 Temporal Ctx | 56.3 | 3.8 | Control 3 Parietal Ctx | 0.0 | 12.3 |
| Control 3 Temporal Ctx | 27.2 | 3.3 | Control (Path) 1 Parietal Ctx | 9.1 | 11.3 |
| Control 3 Temporal Ctx | 0.0 | 3.9 | Control (Path) 2 Parietal Ctx | 0.0 | 15.9 |
| Control (Path) 1 Temporal Ctx | 63.7 | 3.2 | Control (path) 3 Parietal Ctx | 68.3 | 7.0 |
| Control (Path) 2 Temporal Ctx | 16.0 | 1.5 | Control (Path) 4 Parietal Ctx | 5.8 | 31.6 |

**Table ID. General_screening_panel_v1.4**

| **Tissue Name** | **Rel. Exp.(%) Ag3254, Run 214694852** | **Rel. Exp.(%) Ag3339, Run 215773747** | **Tissue Name** | **Rel.Exp.(%) Ag3254, Run 214694852** | **Rel.Exp.(%) Ag3339, Run 215773747** |
|---|---|---|---|---|---|
| Adipose | 0.1 | 0.1 | Renal ca. TK-10 | 0.7 | 0.0 |
| Melanoma* Hs688(A).T | 0.2 | 0.0 | Bladder | 0.3 | 0.1 |
| Melanoma* Hs688(B).T | 0.3 | 0.0 | Gastric ca. (liver met.) NCI-N87 | 4.9 | 0.0 |
| Melanoma* M14 | 0.0 | 0.0 | Gastric ca. KATO III | 0.0 | 0.0 |
| Melanoma* LOXIMVI | 0.2 | 0.0 | Colon ca. SW-948 | 0.0 | 0.0 |
| Melanoma* SK-MEL-5 | 0.9 | 0.0 | Colon ca. SW480 | 1.6 | 0.0 |
| Squamous cell carcinoma SCC-4 | 0.4 | 0.0 | Colon ca.* (SW48.0 met) SW620 | 5.3 | 0.1 |
| Testis Pool | 2.5 | 2.0 | Colon ca. HT29 | 0.0 | 0.0 |
| Prostate ca.* (bone met) PC-3 | 3.6 | 0.0 | Colon ca. HCT- 116 | 0.0 | 0.0 |
| Prostate Pool | 0.3 | 1.9 | Colon ca. CaCo-2 | 0.0 | 0.2 |
| Placenta | 3.0 | 1.3 | Colon cancer tissue | 1.8 | 0.1 |
| Uterus Pool | 1.3 | 0.1 | Colon ca. SW1116 | 0.0 | 0.0 |
| Ovarian ca. OVCAR-3 | 1.8 | 0.2 | Colon ca. Colo-205 | 0.0 | 0.0 |
| Ovarian ca. SK-OV-3 | 2.4 | 0.7 | Colon ca. SW-48 | 0.2 | 0.0 |
| Ovarian ca. OVCAR-4 | 0.8 | 0.9 | Colon Pool | 5.7 | 0.7 |
| Ovarian ca. OVCAR-5 | 1.5 | 1.8 | Small Intestine Pool | 22.4 | 0.8 |
| Ovarian ca. IGROV-1 | 2.0 | 0.1 | Stomach Pool | 3.1 | 0.8 |
| Ovarian ca. OVCAR-8 | 0.4 | 0.0 | Bone Marrow Pool | 3.8 | 0.3 |
| Ovary | 2.5 | 0.2 | Fetal Heart | 0.1 | 0.4 |
| Breast ca. MCF-7 | 0.5 | 0.1 | Heart Pool | 1.9 | 0.4 |
| Breast ca. MDA-MB-231 | 1.0 | 0.0 | Lymph Node Pool | 14.6 | 0.8 |
| Breast ca. BT 549 | 0.0 | 0.1 | Fetal Skeletal Muscle: | 0.2 | 0.2 |
| Breast ca. T47D | 6.4 | 7.6 | Skeletal Muscle Pool | 0.4 | 0.2 |
| Breast ca. MDA-N | 0.0 | 0.0 | Spleen Pool | 6.2 | 0.2 |
| Breast Pool | 7.0 | 1.1 | Thymus Pool | 4.1 | 1.0 |
| Trachea | 2.8 | 2.0 | CNS cancer (glio/astro) U87-MG | 0.0 | 0.0 |
| Lung | 0.3 | 0.0 | CNS cancer (glio/astro) U-118-MG | 0.0 | 0.0 |
| Fetal Lung | 7.5 | 17.0 | CNS cancer (neuro;met) SK-N-AS | 1.2 | 0.0 |
| Lung ca. NCI-N417 | 0.0 | 0.0 | CNS cancer (astro) SF-539 | 0.0 | 0.0 |
| Lung ca. LX-1 | 26.4 | 0.1 | CNS cancer (astro) SNB-75 | 0.0 | 0.0 |
| Lung ca. NCI-H146 | 0.1 | 0.0 | CNS cancer (glio) SNB-19 | 0.9 | 0.0 |
| Lung ca. SHP-77 | 100.0 | 0.1 | CNS cancer (glio) SF-295 | 0.0 | 0.0 |
| Lung ca. A549 | 0.0 | 0.0 | Brain (Amygdala) Pool | 0.2 | 0.7 |
| Lung ca. NCI-H526 | 0.0 | 0.0 | Brain (cerebellum) | 0.6 | 0.9 |
| Lung ca. NCI-H23 | 2.1 | 100.0 | Brain (fetal) | 5.9 | 0.9 |
| Lungca.NCI-H460 | 0.0 | 0.1 | Brain (Hippocampus) Pool | 0.8 | 0.9 |
| Lung ca. HOP-62 | 0.0 | 0.0 | Cerebral Cortex Pool | 0.1 | 0.5 |
| Lung ca. NCI-H522 | 3.3 | 0.6 | Brain (Substantia nigra) Pool | 1.8 | 1.0 |
| Liver | 0.0 | 0.1 | Brain (Thalamus) Pool | 1.5 | 0.9 |
| Fetal Liver | 1.8 | 0.1 | Brain (whole) | 1.4 | 0.3 |
| Liver ca. HepG2 | 0.6 | 0.0 | Spinal Cord Pool | 3.2 | 5.9 |
| Kidney Pool | 25.7 | 0.5 | Adrenal Gland | 4.2 | 0.6 |
| Fetal Kidney | 5.4 | 2.8 | Pituitary gland Pool | 0.5 | 0.1 |
| Renal ca. 786-0 | 0.5 | 0.0 | Salivary Gland | 0.9 | 0.1 |
| Renal ca. A498 | 0.3 | 0.0 | Thyroid (female) | 0.4 | 0.2 |
| Renal ca. ACHN | 0.1 | 0.0 | Pancreatic ca. CAPAN2 | 0.0 | 0.1 |
| Renal ca. UO-31 | 1.3 | 0.0 | Pancreas Pool | 6.4 | 1.1 |

**Table IE. Panel 4D**

| **Tissue Name** | **Rel. Exp.(%) Ag3254, Run 164391364** | **Rel. Exp.(%) Ag3339, Run 165221778** | **Tissue Name** | **Rel. Exp.(%) Ag3254, Run 164391364** | **Rel. Exp.(%) Ag3339, Run 165221778** |
|---|---|---|---|---|---|
| Secondary Th1 act | 0.0 | 0.0 | HUVEC IL-1beta | 0.0 | 1.4 |
| Secondary Th2 act | 0.0 | 0.0 | HUVEC IFN gamma | 0.0 | 0.0 |
| Secondary Tr1 act | 0.0 | 0.0 | HUVEC TNF alpha + IFN gamma | 0.0 | 0.0 |
| Secondary Th1 rest | 0.0 | 0.0 | HUVEC THF alpha + IL4 | 0.0 | 1.0 |
| Secondary Th2 rest | 0.0 | 0.0 | HUVEC IL-11 | 0.0 | 1.4 |
| Secondary Tr1 rest | 0.0 | 0.0 | Lung Microvascular EC none | 0.0 | 0.0 |
| Primary Th1 act | 0.0 | 0.0 | Lung Microvascular EC TNFalpha + IL-1beta | 0.0 | 0.0 |
| Primary Th2 act | 0.0 | 0.0 | Microvascular Dermal EC none | 0.0 | 0.0 |
| Primary Tr1 act | 0.0 | 0.0 | Microsvasular Dermal EC TNFalpha + IL-1 beta | 0.0 | 7.5 |
| Primary Th1 rest | 0.0 | 0.0 | Bronchial epithelium TNFalpha + IL 1beta | 0.0 | -6.7 |
| Primary Th2 rest | 0.0 | 1.2 | Small airway epithelium; none | 0.0 | 0.0 |
| Primaary Th1 rest | 0.0 | 0.9 | Small airway epithelium TNFalpha + IL-1beta | 0.0 | 36.6 |
| CD45RA CD4 lymphocyte act | 0.0 | 0.0 | Coronery artery SMC rest | 0.0 | 0.0 |
| CD45RO CD4 lymphocyte act | 0.0 | 0.0 | Coronery artery SMC TNFalpha + IL-1 beta | 0.0 | 0.0 |
| CD8 lymphocyte act | 0.0 | 0.0 | Astrocytes rest | 0.0 | 0.0 |
| Secondary CD8 lymphocyte rest | 0.0 | 0.0 | Astrocytes TNFalpha+ IL-1beta | 0.0 | 0.0 |
| Secondary CD8 lymphocyte act | 0.0 | 0.0 | KU-812 (Basophil) rest | 0.0 | 1.9 |
| CD4 lymphocyte none | 0.0 | 5.7 | KU-812 (Basophil) PMA/ionomycin | 0.0 | 2,8 |
| 2ry Th1/Th2/Tr1_anti-CD95 CH11 | 0.0 | 0.0 | CCD1106 (Keratinocytes) none | 0.0 | 0.0 |
| LAK cells rest | 0.0 | 1.1 | CCD1106 (Keratinocytes) TNFalpha + IL-1beta | 0.0 | 1.4 |
| LAK cells IL-2 | 0.0 | 0.0 | Liver cirrhosis | 0.0 | 4.6 |
| LAK cells IL-2+IL-12 | 0.0 | 0.0 | Lupus kidney | 0.0 | 32.1 |
| LAK cells IL-2+IFN gamma | 0.0 | 0.0 | NCl-H292 none | 0.0 | 0.0 |
| LAK cells IL-2+ IL-18 | 0.0 | 0.7 | NCI-H292 IL-4 | 0.0 | 1.3 |
| LAK cells PMA/ionomycin | 0.0 | 1.2 | NCI-H292 IL-9 | 0.0 | 2.5 |
| NK Cells IL-2 rest | 0.0 | 3.0 | NCI-H292 IL-13 | 0.0 | 0.0 |
| Two Way MLR 3 day | 0.0 | 2.7 | NCI-H292 IFN gamma | 0.0 | 0.0 |
| Two Way MLR 5 day | 0.0 | 0.0 | HPAEC none | 0.0 | 0.0 |
| Two Way MLR 7 day | 100.0 | 0.0 | HPAEC TNF alpha + IL-1 beta | 0.0 | 44.8 |
| PBMC rest | 0.0 | 16.8 | Lung fibroblast none | 0.0 | 0.0 |
| PBMC PWM | 0.0 | 2.4 | Lung fibroblast TNF alpha + IL-1 beta | 0.0 | 0.0 |
| PBMCPHA-L | 0.0 | 0.0 | Lung fibroblast IL-4 | 0.0 | 0.0 |
| Ramos (B cell) none | 0.0 | 0.0 | Lung fibroblast IL-9 | 0.0 | 0.0 |
| Ramos (B cell) ionomycin | 0.0 | 0.0 | Lung fibroblast IL-13 | 0.0 | 0.0 |
| B lymphocytes PWM | 0.0 | 0.0 | Lung fibroblast IFN gamma | 0.0 | 0.0 |
| B lymphocytes CD40L and IL-4 | 0.0 | 0.0 | Dermal fibroblast CCD1070 rest | 0.0 | 0.0 |
| EOL-1 dbcAMP | 0.0 | 0.0 | Dermal fibroblast CCD1070 TNF alpha | 0.0 | 1.5 |
| EOL-1 dbcAMP PMA/ionomycin | 0.0 | 1.4 | Dermal fibroblast CCD1070 IL-1 beta | 0.0 | 0.0 |
| Dendritic cells none | 0.0 | 1.3 | Dermal fibroblast IFN gamma | 0.0 | 0.0 |
| Dendritic cells LPS | 0.0 | 0.0 | Dermal fibroblast IL-4 | 0.0 | 0.0 |
| Dendritic cells anti-CD40 | 0.0 | 0.0 | IBD Colitis 2 | 0.0 | 0.5 |
| Monocytes rest | 0.0 | 49.3 | IBD Crohn's | 0.0 | 0.0 |
| Monocytes LPS | 0.0 | 79.0 | colon | 0.0 | 15.1 |
| Macrophages rest | 0.0 | 9.0 | Lung | 0.0 | 100.0 |
| Macrophages LPS | 0.0 | 3.1 | Thymus | 0.0 | 95.3 |
| HUVEC none | 0.0 | 0.0 | Kidney | 0.0 | 32.3 |
| HUVEC starved | 0.0 | 0.0 | | | |

**CNS_neurodegeneration_v1.0 Summary:** Ag3339 This panel confirms the expression of the CG57488-01 gene at low levels in the brain in an independent group of individuals. Interestingly, this gene appears to be slightly upregulated in the temporal cortex of Alzheimer's disease patients. Therefore, therapeutic modulation of the activity of this gene or its protein product may be of use to decrease neuronal death in the treatment of Alzheimer's disease. Ag3254 Expression of this gene is low/undetectable (CTs > 35) across all of the samples on this panel (data not shown).

**General_screeninuanel_v1.4 Summary:** Ag3254 Expression of the CG57488-01 gene is highest in lung cancer cell line SHP-77 (CT = 29.1). Expression of this gene is also higher in fetal lung (CT = 32.9) than in adult lung (CT = 37.7), suggesting that expression of this gene can be used to distinguish fetal lung from adult lung. In addition, significant expression of this gene is seen in adult and fetal kidney. This gene resembles members of the alpha-2-macroglobulin family. It has been shown that patients with chronic renal failure treated with haemodialysis have significantly lower blood serum levels of alpha-2-macroglobulin than healthy patients (ref. 1). Therefore, therapeutic modulation of the activity of this gene or its protein product, using small molecule drugs, antibodies, or protein therapeutics, may be used as a treatment for patients with renal failure.

Among tissues with metabolic or endocrine function, this gene is expressed at low levels in adrenal gland, heart, and pancreas. Therefore, therapeutic modulation of the activity of this gene or its protein product may prove useful in the treatment of endocrine/metabolically related diseases, such as obesity and diabetes.

The CG57488-01 gene is expressed at low levels in some regions of the CNS, including fetal brain, substantia nigra and spinal cord. Alpha-2-macroglobulin, a serum pan-protease inhibitor, has been implicated in Alzheimer's disease based on its ability to mediate the clearance and degradation of A-beta, the major component of amyloid beta deposits (ref. 2). Therefore, the CG57488-01 gene may also play a role in the development of Alzheimer's disease.

### References:

### 1. Bartelik S, Starzyk J, Krajewska R. Concentration of prealbumin, ceruloplasmin, alpha-macroglobulin and haptoglobin in blood serum of patients with chronic non-A, non-B hepatitis treated with hemodialysis for chronic renal failure. Wiad Lek 1992 Oct;45(19-20):733-6

In 25 patients with chronic renal failure, treated with haemodialysis (13 patients with chronic non-A, non-B hepatitis, and 12 cases without evidence of hepatocellular damage), and in 20 healthy persons, blood serum concentrations were determined of prealbumin, ceruloplasmin, alpha 2-macroglobulin, and haptoglobin. It was found that the concentrations of these proteins in both subgroups of patients were not significantly different. The concentration of prealbumin was higher, and that of alpha 2-macroglobulin and haptoglobin was significantly lower in comparison with healthy subjects.

### PMID: 1284260

### 2. Blacker D, Wilcox MA, Laird NM, Rodes L, Horvath SM, Go RC, Perry R, Watson B Jr, Bassett SS, McInnis MG, Albert MS, Hyman BT, Tanzi RE. Alpha-2 macroglobulin is genetically associated with Alzheimer disease. Nat Genet 1998 Aug;19(4):357-60

Alpha-2-macroglobulin (alpha-2M; encoded by the gene A2M) is a serum pan-protease inhibitor that has been implicated in Alzheimer disease (AD) based on its ability to mediate the clearance and degradation of A beta, the major component of beta-amyloid deposits. Analysis of a deletion in the A2M gene at the 5' splice site of'exon IT of the bait region (exon 18) revealed that inheritance of the deletion (A2M-2) confers increased risk for AD (Mantel Haenzel odds ratio=3.56, P=0.001). The sibship disequilibrium test (SDT) also revealed a significant association between A2M and AD (P=0.00009). These values were comparable to those obtained for the APOE-epsilon4 allele in the same sample, but in contrast to APOE epsilon4, A2M-2 did not affect age of onset. The observed association of A2M with AD did not appear to account for the previously published linkage of AD to chromosome 12, which we were unable to confirm in this sample. A2M, LR(encoding the alpha-2M receptor) and the genes for two other LRP ligands, APOE and APP (encoding the amyloid beta protein precursor), have now all been genetically linked to AD, suggesting that these proteins may participate in a common neuropathogenic pathway leading to AD.

### PMID: 9697696

**Panel 4D Summary:** Ag3339 Expression of the CG57488-01 gene is highest in normal lung and thymus (CTs = 31). In addition, expression of this gene is upregulated in small airway epithelium treated with TNFalpha and IL-1beta. Therefore, therapeutic modulation of the activity of this gene or its protein product, using small molecule drugs, antibodies or protein therapeutics, may be of benefit in the treatment of asthma and emphysema. Furthermore, this gene is moderately expressed in monocytes, suggesting that this gene may also play a role in rheumatoid arthritis or other autoimmune diseases. Ag3254 Results from one experiment with the CG57488-01 gene are not included. The amp plot indicates that there were experimental difficulties with this run.

### NOV12

Expression of NOV12A/CG57526-01 and NOV12B/CG57526-02 was assessed using the primer-probe set Ag3275, described in Table JA. Results of the RTQ-PCR runs are shown in Tables JB, JC and JD.

**Table JA. Probe Name Ag3275**

| **Primers** | **Sequences** | **Length** | **Start Position** | **SEQ ID NO** |
|---|---|---|---|---|
| Forward | 5'-ggaactactggctggagatttt-3' | 22 | 1500 | 285 |
| Probe | TET-5'-ctgaacctgcteatgttggcctttct-3'-TAMRA | 26 | 1541 | 286 |
| Reverse | 5'-aaacataaacgacacecacaac-3' | 22 | 1571 | 287 |

**Table JB. CNS_neurodegeneration_v1.0**

| **Tissue Name** | **Rel. Exp.(%) Ag3275, Run 210059235** | **Tissue Name** | **Rel. Exp.(%) Ag3275, Run 210059235** |
|---|---|---|---|
| AD 1 Hippo | 0.0 | Control (Path) 3 Temporal Ctx | 0.0 |
| AD 2 Hippo | 0.0 | Control (Path) 4 Temporal Ctx | 0.0 |
| AD 3 Hippo | 0.0 | AD1 Occipital Ctx | 0.0 |
| AD 4 Hippo | 0.0 | AD 2 Occipital Ctx (Missing) | 0.0 |
| AD 5 Hippo | 0.0 | AD 3 Occipital Ctx | 0.0 |
| AD 6 Hippo | 22.5 | AD 4 Occipital Ctx | 0.0 |
| Control 2 Hippo | 59.0 | AD 5 Occipital Ctx | 0.0 |
| Control 4 Hippo | 0.0 | AD 6 Occipital Ctx | 0.0 |
| Control (Path) 3 Hippo | 0.0 | Control 1 Occipital Ctx | 0.0 |
| AD 1 Temporal Ctx | 0.0 | Control 2 Occipital Ctx | 0.0 |
| AD 2 Temporal Ctx | 0.0 | Control 3 Occipital Ctx | 0.0 |
| AD 3 Temporal Ctx | 26.8 | Control 4 Occipital Ctx | 51.8 |
| AD 4 Temporal Ctx | 0.0 | Control (Path) 1 Occipital Ctx | 100.0 |
| AD 5 Inf Temporal Ctx | 0.0 | Control (Path) 2 Occipital Ctx | 27.7 |
| AD 5 Sup Temporal Ctx | 0.0 | Control (Path) 3 Occipital Ctx | 0.0 |
| AD 6 Inf Temporal Ctx | 0.0 | Control (Path) 4 Occipital Ctx | 0.0 |
| AD 6 Sup Temporal Ctx | 0.0 | Control 1 Parietal Ctx | 0.0 |
| Control Temporal Ctx | 96.6 | Control 2 Parietal Ctx | 0.0 |
| Control 2 Temporal Ctx | 62.9 | Control 3 Parietal Ctx | 48.0 |
| Control 3 Temporal Ctx | 0.0 | Control (Path) 1 Parietal Ctx | 0.0 |
| Control 3 Temporal Ctx | 0.0 | Control (Path) 2 Parietal Ctx | 19.5 |
| Control (Path) 1 Temporal Ctx | 73.2 | Control (Path) 3 Parietal Ctx | 0.0 |
| Control (Path) 2 Temporal Ctx | 0.0 | Control (Path) 4 Parietal Ctx | 0.0 |

**Table JC. Genend_screening_panel_v1.4**

| **Tissue Name** | **Rel. Exp.(%) Ag3275, Run 215775538** | **Tissue Name** | **Rel. Exp.(%) Ag3275, Run 215775538** |
|---|---|---|---|
| Adipose | 0.0 | Renal ca. TK-10 | 5.1 |
| Melanoma* Hs688(A).T | 0.0 | Bladder | 0.0 |
| Melanoma* Hs688(B).T | 5.5 | Gastric ca. (liver met) NCI-N87 | 9.4 |
| Melanoma* M14 | 0.0 | Gastric ca. KATO III | 5.1 |
| Melanoma* LOXIMVI | 0.0 | Colon ca. SW-948 | 3.7 |
| Melanoma* SK-MEL-5 | 0.0 | Colon ca. SW480 | 0.0 |
| Squamous cell carcinoma SCC4 | 4.4 | Colon ca.* (SW480 met) SW620 | 4.0 |
| Testis Pool | 0.0 | Colon ca. HT29 | 4.2 |
| Prostate ca.* (bone met) PC-3 | 0.0 | Colon ca. HCT-116 | 0.0 |
| Prostate Pool | 0.0 | Colon ca. CaCo-2 | 0.0 |
| Placenta | 0.0 | Colon cancer tissue | 0.0 |
| Uterus Pool | 0.0 | Colon ca. SW1116 | 0.0 |
| Ovarian ca. OVCAR-3 | 0.0 | Colon ca. Colo-205 | 0.0 |
| Ovarian ca. SK-OV-3 | 0.0 | Colon ca. SW.48 | 17.2 |
| Ovarian ca. OVCAR-4 | 0.0 | Colon Pool | 3.2 |
| Ovarian ca. OVCAR-5 | 0.0 | Small Intestine Pool | 0.0 |
| Ovarian ca.IGROV-1 | 0.0 | Stomach Pool | 0.0 |
| Ovarian ca. OVCAR-8 | 0.0 | Bone Marrow Pool | 0.0 |
| Ovary | 0.0 | Fetal Heart | 0.0 |
| Breast ca. MCF-7 | 0.0 | Heart Pool | 10.4 |
| Breast ca MDA-MB-231 | 10.4 | Lymph Node Pool | 4.8 |
| Breast ca. BT 549 | 0.0 | Fetal Skeletal Muscle | 0.0 |
| Breast ca. T47D | 0.0 | Skeletal Muscle Pool | 10.5 |
| Breast ca. MDA-N | 0.0 | Spleen Pool | 4.6 |
| Breast Pool | 0.0 | Thymus Pool | 5.0 |
| Trachea | 0.0 | CNS cancer (glio/astro) U87-MG | 5.0 |
| Lung | 0.0 | CNS cancer (glio/astro) U-118-MG | 0.0 |
| Fetal Lung | 0.0 | CNS cancer (neuro;met) SK-N-AS | 61.6 |
| Lung ca. NCI-N417 | 0.0 | CNS cancer (astro) SF-539 | 0.0 |
| Lung ca. LX-1 | 6.7 | CNS cancer (astro) SNB-75 | 0.0 |
| Lung ca. NCI-H146 | 7.5 | CNS cancer (glio) SNB-19 | 0.0 |
| Lung ca. SHP-77 | 59.0 | CNS cancer (glio) SF-295 | 0.0 |
| Lung ca. A549 | 4.4 | Brain (Amygdala) Pool | 0.0 |
| Lung ca. NCI-H526 | 10.2 | Brain (cerebellum) | 0.0 |
| Lung ca. NCI-H23 | 100.0 | Brain (fetal) | 5.3 |
| Lung ca. NCI-H460 | 7.6 | Brain (Hippocampus) Pool | 0.0 |
| Lung ca. HOP-62 | 0.0 | Cerebral Cortex Pool | 0.0 |
| Lung ca. NCI-H522 | 5.6 | Brain (Substantia nigra) Pool | 1.3 |
| Liver | 0.0 | Brain (Thalamus) Pool | 0.0 |
| Fetal Liver | 0.0 | Brain (whole) | 0.0 |
| Liver ca. HepG2 | 0.0 | Spinal Cord Pool | 0.0 |
| Kidney Pool | 0.0 | Adrenal Gland | 2.4 |
| Fetal Kidney | 55.5 | Pituitary gland Pool | 0.0 |
| Renal ca. 786-0 | 0.0 | Salivary Gland | 0.0 |
| Renal ca. A498 | 0.0 | Thyroid (female) | 0.0 |
| Renal ca. ACHN | 9.4 | Pancreane ca.CAPAN2 | 0.0 |
| Renal ca. UO-31 | 0.0 | Pancreas Pool | 0.0 |

**Table JD. Panel 4D**

| **Tissue Name** | **Rel. Exp.(%) Ag3275, Run 164634885** | **Tissue Name** | **Rel. Exp.(%) Ag3275, Run 164634885** |
|---|---|---|---|
| Secondary Th1 act | 0.7 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVECIL-11 | 0.0 |
| Secondary Tr1 rest | 0.0 | Lung Microvascular EC none | 0.0 |
| Primary Th1 act | 0.8 | Lung Microvascular EC TNFalpha +IL-1 beta | 0.0 |
| Primary Th2 act | 0.5 | Microvascular Dermal EC none | 0.0 |
| Primary Tr1 act | 0.6 | Microsvasular Dermal EC TNFalpha +IL-1beta | 0.0 |
| Primary Th1 rest | 0.0 | Bronchial epithelium TNFalpha + IL1beta | 0.0 |
| Primary Th2 rest | 0.7 | Small airway epithelium none | 0.0 |
| Primary Tr1 rest | 0.0 | Small airway epithelium TNFalpha +IL-1 beta | 0.0 |
| CD45RA CD4 lymphocyte act | 0.0 | Coronery artery SMC rest | 0.0 |
| CD45RO CD4 lymphocyte act | 1.3 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1 beta | 0.0 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1 anti-CD95 CH11 | 0.0 | CCD1106 (Keratinocytes) none | 0.0 |
| LAK cells rest | 0.0 | CCD 1106 (Keratinocytes) TNFalpha + IL-1beta | 0.0 |
| LAK cells IL-2 | 0.0 | Liver cirrhosis | 4.5 |
| LAK cells IL-2+IL-12 | 0.0 | Lupus kidney | 3.6 |
| LAK cells IL-2+IFN ganima | 0.0 | NCI-H292 none | 3.6 |
| LAK cells IL-2+ IL-18 | 0.0 | NCI-H292 IL-4 | 9.5 |
| LAK cells PMA/ionomycin | 0.0 | NCI-H292 IL-9 | 1.7 |
| NK Cells IL-2 rest | 0.0 | NCI-H292 IL-13 | 3.6 |
| Two Way MLR 3 day | 0.0 | NCI-H292 IFN gamma | 0.0 |
| Two Way MLR 5 day | 0.3 | HPAEC none | 1.4 |
| Two Way MLR 7 day | 0.9 | HPAEC TNF alpha + IL-1 beta | 0.7 |
| PBMC rest | 0.0 | Lung fibroblast none | 0.0 |
| PBMC PWM | 0.0 | Lung fibroblast TNF alpha + IL-1 beta | 0.0 |
| PBMC PHA-L | 0.0 | Lung fibroblast IL-4 | 0.0 |
| Ramos (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 0.0 | Lung fibroblast IFN gamma | 0.0 |
| B lymphocytes. CD40L IL-4 | 0.0 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 0.0 |
| Monocytes rest | 0.0 | IBD Crolm's | 0.0 |
| Monocytes LPS | 0.0 | Colon | 10.9 |
| Macrophages rest | 0.0 | Lung | 2.5 |
| Macrophages LPS | 0.0 | Thymus | 100.0 |
| HUVEC none | 0.0 | Kidney | 1.4 |
| HUVEC starved | 0.0 | | |

**CNS_neurodegeneration_v1.0 Summary:** Ag3275 Expression of this gene is low/undetectable (CTs > 35) across all of the samples on this panel (data not shown).

**General_screening_panel_v1.4 Summary**: Ag3275 Expression of the CG57526-01 gene is highest in lung cancer cell line NCI-H23 (CT=32.8). Expression of this gene is higher in several additional lung cancer cell lines when compared to normal lung. Thus, expression of this gene may be used as a marker for lung cancer. Furthermore, therapeutic modulation of the activity of this gene or its protein product, using small molecule drugs, antibodies or protein therapeutics, may be of use in the treatment of lung cancer. The CG57526-01 gene encodes a protein with homology to sodium- and chloride-dependent transporter XT3, a protein that mediates transit of structurally related small hydrophilic substances across plasma membranes (ref. 1).

Interestingly, while there is low expression of this gene in fetal kidney (CT=33.6), this gene does not seem to be expressed at detectable levels in adult kidney. This observation suggests that expression of this gene can be used to distinguish fetal from adult kidney. Expression of this gene is consistent with what is known about the XT3 transporter (ref. 1).

### References:

### 1. Nash SR, Giros B, Kingsmore SF, Kim KM, el-Mestikawy S, Dong Q, Fumagalli F, Seldin MF, Caron MG. Cloning, gene structure and genomic localization of an orphan transporter from mouse kidney with six alternatively-spliced isoforms. Receptors Channels 1998;6(2):113-28

Two genes were identified and characterized that express cDNAs related to previously identified neurotransmitter and/or osmolyte transporters, but which are expressed specifically in the kidney. RNA transcribed from one of these two genes (XT2) was found to undergo an extensive degree of alternative splicing to generate six distinct isoforms. The intron-exon structure of the XT2 gene and the sites of alternative splicing were identified. Expression of the second gene (XT3) was found to be conserved in human kidney, and partial sequence was obtained from a human cDNA library. The expressions of both XT2 and XT3 RNAs were determined in mouse and human tissues, respectively, and the locations of the two genes within the mouse genome were identified. Screening experiments to identify the substrate(s) of these proteins failed to identify specific uptake with any of the tested compounds; however, immunofluorescent microscopy demonstrated that epitope-tagged variants of the protein products of the XT2 and XT3 cDNAs were present on the plasma membrane of transfected cells.

### PMID: 9932288

Panel 4D Summary: Ag3275 Expression of the CG57526-01 gene is highest in thymus (CT = 29). Therefore, expression of this gene may be used to distinguish thymus from the other samples on this panel. In addition, the putative ion transporter encoded for by the CG57526-01 gene could play an important role in T cell development. Small molecule therapeutics or antibody therapeutics designed against the protein encoded for by this gene could be utilized to modulate immune function (T cell development) and be important for organ transplant; AIDS treatment or post chemotherapy immune reconstitiution. In addition, this gene is expressed at low levels in the NCI-H292 mucoepidermoid cell line, consistent with its expression in lung cancer cell lines on General_screening_panel_v1.4.

### NOV13

Expression of NOV13/CG57570-01 was assessed using the primer-probe set Ag3288, described in Table KA. Results of the RTQ-PCR runs are shown in Tables KB, KC and KD.

**Table KA. Probe Name Ag3288**

| **Primers** | **Sequences** | **Length** | **Start Position** | **SEQ ID NO** |
|---|---|---|---|---|
| Forward | 5'-tctacaccatcagctgtatgca-3' | 22 | 1380 | 288 |
| Probe | TET-5'-caccaccctcacactcatcttcatca-3'-TAMRA | 26 | 1411 | 289 |
| Reverse | 5'-gcagtgcagctgtcatatagaa-3' | 22 | 1439 | 290 |

**Table KB. CNS_neurodegeneration_v1.0**

| **Tissue Name** | **Rel. Exp.(%) Ag3288, Run 210058660** | **Rel. Exp.(%) Ag3288, Run 229929907** | **Tissue Name** | **Rel.Exp.(%) Ag3288, Run 210058660** | **Rel.Exp.(%) Ag3288, Run 229929907** |
|---|---|---|---|---|---|
| AD 1 Hippo | 26.6 | 31.0 | Control) (Path) 3 Temporal Ctx | 13.3 | 15.1 |
| AD 2 Hippo | 43.5 | 48.0 | Control (Path) 4 Temporal Ctx | 33.7 | 36.3 |
| AD 3 Hippo | 15.0 | 14.8 | AD 1 Occipital | 25.7 | 15.5 |
| AD 4 Hippo | 13.0 | 11.5 | AD 2 Occipital Ctx (Missing) | 0.0 | 0.0 |
| AD 5 hippo | 97.3 | 100.0 | AD 3 Occipital | 16.5 | 15.8 |
| AD 6 Hippo | 100.0 | 90.1 | AD 4 Occipital Ctx | 26.1 | 35.1 |
| Control 2 Hippo | 29.3 | 28.9 | AD 5 Occipital Ctx | 26.4 | 28.5 |
| Control 4 Hippo | 33.7 | 41.2 | AD 6 Occipital | 33.2 | 37.6 |
| Control (Path) 3 Hippo | 22.8 | 27.9 | Control Ctx Occipital Ctx | 8.8 | 10.4 |
| AD 1 Temporal Ctx | 34.2 | 35.8 | Control 2 Occipital Ctx | 51.8 | 59.9 |
| AD 2 Temporal Ctx | 47.3 | 52.1 | Control 3 Occipital Ctx | 28.5 | 12.8 |
| AD 3 Temporal Ctx | 14.0 | 18.6 | Control 4 Occipital Ctx | 16.5 | 17.8 |
| AD 4 Temporal Ctx | 34.6 | 46.7 | Control (Path) Occipital Ctx | 66.0 | 82.4 |
| AD 5 Inf Temporal Ctx | 80.1 | 100.0 | Control (Path) 2 Occipital Ctx | 11.3 | 14.4 |
| AD 5 SupTemporal Ctx | 64.2 | 85.9 | Control (Path) 3 Occipital Ctx | 9.5 | 8.5 |
| AD 6 Inf Temporal Ctx | 68.8 | 87.1 | Control (Path) 4 Occipital Ctx | 13.9 | 17.7 |
| AD 6 Sup Temporal Ctx | 66.4 | 87.1 | Control 1 Parietal Ctx | 11.1 | 15.8 |
| Control 1 Temporal Ctx | 11.3 | 16.2 | Control2 Parietal Ctx | 48.0 | 66.0 |
| Control 2 Temporal Ctx | 33.0 | 36.3 | Control 3 Parietal Ctx | 17.0 | 22.2 |
| Control 3 Temporal Ctx | 19.2 | 18.9 | Control (Path) 1 Parietal Ctx | 55.5 | 66.4 |
| Control 4 Temporal Ctx | 14.7 | 17.8 | Control (Path) 2 Parietal Ctx | 30.6 | 34.2 |
| Control (Path) 1 Temporal Ctx | 32.3 | 50.7 | Control (Path) 3 Parietal Ctx | 14.3 | 11.3 |
| Control (Path) 2 Temporal Ctx | 36.9 | 13.2 | Control (Path)4 Parietal Ctx | 37.1 | 43.2 |

**Table KC. General_screening_panel_v1.4**

| **Tissue Name** | **Rel. Exp.(%) Ag3288, Run 216516909** | **Tissue Name** | **Rel. Exp.(%) Ag3288, Run 216516909** |
|---|---|---|---|
| Adipose | 15.8 | Renal ca. TK-10 | 24.0 |
| Melanoma* Hs688(A).T | 38.4 | Bladder | 26.8 |
| Melanoma* Hs688(B).T | 51.4 | Gastric ca. (liver met) NCI-N87 | 55.1 |
| Melanoma* M14 | 43.5 | Gastric ca. KATO III | 53.6 |
| Melanoma*LOXIMVI | 25.0 | Colon ca. SW-948 | 19.1 |
| Melanoma* SK-MEL-5 | 52.9 | Colon ca. SW480 | 41.2 |
| Squamous cell carcinoma SCC-4 | 16.5 | Colon ca.* (SW480 met) SW620 | 14.4 |
| Testis Pool | 44.4 | Colon ca. HT29 | 9.5 |
| Prostate ca.* (bone met) PC-3 | 57.4 | Colon ca. HCT-116 | 50.7 |
| Prostate Pool | 16.2 | Colon ca. CaCo-2 | 9.5 |
| Placenta | 4.3 | Colon cancer tissue | 7.3 |
| Uterus Pool | 4.9 | Colon ca. SW1116 | 6.7 |
| Ovarian ca. OVCAR-3 | 37.9 | Colon ca. Colo-205 | 10.5 |
| Ovarian ca. SK-OV-3 | 39.0 | Colon ca. SW-48 | 7.3 |
| Ovarian ca. OVCAR-4 | 18.0 | Colon Pool | 15.9 |
| Ovarian ca. OVCAR-5 | 63.3 | Small Intestine Pool | 15.0 |
| Ovarian ca. IGROV-1 | 14.5 | Stomach Pool | 9.6 |
| Ovarian ca. OVCAR-8 | 12.1 | Bone Marrow Pool | 9.4 |
| Ovary | 17.2 | Fetal Heart | 72.7 |
| Breast ca. MGF-7 | 32.5 | Heart Pool | 66.9 |
| Breast ca. MDA-MB-231 | 47.3 | Lymph Node Pool | 19.3 |
| Breast ca. BT 549 | 58.2 | Fetal Skeletal Muscle | 17.6 |
| Breastca.T47D | 73.7 | Skeletal Muscle Pool | 63.7 |
| Breast ca. MDA-N | 12.6 | Spleen Pool | 11.0 |
| Breast Pool | 14.6 | Thymus Pool | 7.5 |
| Trachea | 14.6 | CNS cancer (glio/astro) U87-MG | 30.8 |
| Lung | 7.5 | CNS cancer (glio/astro) U-118-MG | 44.1 |
| Fetal Lung | 63.7 | CNS cancer(neuro ; met)SK-N-AS | 37.9 |
| Lung ca.NCI-N417 | 2.7 | CNS cancer (astro) SF-539 | 33.2 |
| Lung ca. LX-1 | 19.5 | CNS cancer (astro) SNB-75 | 34.6 |
| Lung ca. NCI-H146 | 4.9 | CNS cancer (glio)SNB-19 | 18.9 |
| Lung ca.SHP.77 | 12.0 | CNS cancer (glio) SF-295 | 64.2 |
| Lung ca. A549 | 24.8 | Brain (Amygdala) Pool | 13.4 |
| Lung ca. NCI-H526 | 8.8 | Brain (cerebeHum) | 100.0 |
| Lung ca. NCI-H23 | 23.8 | Brain (fetal) | 28.5 |
| Lung ca. NCI-H460 | 29.5 | Brain (Hippocampus) Pool | 21.5 |
| Lung ca. HOP-62 | 23.0 | Cerebral Cortex Pool | 22.8 |
| Lung ca. NCI-H522 | 36.1 | Brain (Substantia nigra) Pool | 19.2 |
| Liver | 0.5 | Brain (Thalamus) Pool | 26.8 |
| Fetal Liver | 27.2 | Brain (whole) | 40.9 |
| Liver ca. HepG2 | 0.2 | Spinal Cord Pool | 19.8 |
| Kidney Pool | 23.0 | Adrenal Gland | 40.3 |
| Fetal Kidney | 21.2 | Pituitary gland Pool | 5.8 |
| Renal ca. 786-0 | 32.1 | Salivary Gland | 6.4 |
| Renal ca.A498 | 17.6 | Thyroid (female) | 17.2 |
| Renal ca.ACHN | 36.1 | Pancreatic ca. CAPAN2 | 14.8 |
| Renal ca. UO-31 | 21.0 | Pancreas Pool | 22.8 |

**Table KD. Panel 4D**

| **Tissue Name** | **Rel. Exp.(%) Ag3288, Run 165007638** | **Tissue Name** | **Rel. Exp.(%) Ag3288, Run 165007638** |
|---|---|---|---|
| Secondary Th1 act | 15.0 | HUVEC IL-1beta | 10.7 |
| Secondary Th2 act | 15.8 | HUVEC IFN gamma | 9.3 |
| Secondary Tr1 act | 15.0 | HUVEC TNF alpha+IFN gamma | 20.9 |
| Secondary Th1 rest | 3.0 | HUVEC TNF alpha+IL4 | 14.9 |
| Secondary Th2 rest | 8.5 | HUVEC IL-11 | 6.6 |
| Secondary Tr1 rest | 4.4 | Lung Microvascular EC none | 9.3 |
| Primary Th1 act | 28.5 | Lung Microvascular EC TNFalpha + IL-1 beta | 58.2 |
| Primary Th2 act | 19.5 | Microvascular Dermal EC none | 22.4 |
| Primary Tr1 act | 24.0 | Microsvasular Dermal EC TNFalpha+IL-1beta | 63.3 |
| Primary Th1 rest | 11.5 | Bronchial epithelium TNFalpha + IL1beta | 20.6 |
| Primary Th2 rest | 8.5 | Small airway epithelium none | 3.8 |
| Primary Tr1 rest | 4.6 | Small airway epithelium TNFalpha +IL-1beta | 48.6 |
| CD45RA CD4 lymphocyte act | 12.2 | Coronery artery SMC rest | 11.7 |
| CD45RO CD4 lymphocyte act | 21.2 | Coronery artery SMC TNF alpha+ IL-1 beta | 9.1 |
| CD8 lymphocyte act | 14.2 | Astrocytes rest | 16.4 |
| Secondary CD8 lymphocyte rest | 22.1 | Astrocytes TNFalpha + IL-1beta | 12.9 |
| Secondary CD8 lymphocyte act | 10.8 | KU-812 (Basophil) rest | 16.8 |
| CD4 lymphocyte none | 5.1 | KU-812 (Basophil) PMA/ionomycin | 50.3 |
| 2ry Th1/Th2/Tr1_anti-CD95 CH11 | 4.0 | CCD1106(Keratinocytes)none | 14.0 |
| LAK cells rest | 4.8 | CCD1106 (Keratinocytes) TNFalpha + IL-1beta | 10.8 |
| LAK cells IL-2 | 10.6 | Liver cirrhosis | 3.3 |
| LAK cells IL-2+IL-12 | 15.7 | Lupus kidney | 1.6 |
| LAK cells IL-2+IFN gamma | 19.3 | NCI-H292 none | 14.6 |
| LAK cells IL-2+IL-18 | 14.2 | NCI-H292 IL-4 | 18.9 |
| LAK cells PMA/ionomycin | 5.7 | NCI-H292 IL-9 | 16.3 |
| NK Cells IL-2 rest | 7.9 | NCI-H292 IL-13 | 13.1 |
| Two Way MLR 3 day | 11.5 | NCI-H292 IFN gamma | 11.2 |
| Two Way MLR 5 day | 9.7 | HPAEC none | 11.5 |
| Two Way MLR 7 day | 6.7 | HPAEC TNF alpha + IL-1beta | 31.0 |
| PBMC rest | 3.6 | Lung fibroblast none | 13.9 |
| PBMCPWM | 61.6 | Lung fibroblast 1NF alpha + IL-1 beta | 13.6 |
| PBMCPHA-L | 31.6 | Lung fibroblast IL-4 | 28.1 |
| Ramos (B cell) none | 13.7 | Lung fibroblast IL-9 | 22.2 |
| Ramos (B cell) ionomycin | 94.6 | Lung fibroblast IL-13 | 13.5 |
| B lymphocytes PWM | 63.7 | Lung fibroblast IFN gamma | 23.7 |
| B lymphocytes CD40L and IL-4 | 100.0 | Dermal fibroblast CCD1070 rest | 30.4 |
| EOL-1 dbcAMP | 3.9 | Dermal fibroblast CCD1070 TNF alpha | 49.0 |
| EOL-1 dbcAMP PMA/ionomycin | 6.7 | Dermal fibroblast CCD1070 IL-1 beta | 15.6 |
| Dendritic cells none | 2.2 | Dermal fibroblast IFN gamma | 6.4 |
| Dendritic cells LPS | 1.9 | Dermal fibroblast IL-4 | 14.2 |
| Dendritic cells anti-CD40 | 0.8 | IBD Colitis 2 | 1.6 |
| Monocytes rest | 0.5 | IBD Crohn's | 0.9 |
| Monocytes LPS | 5.8 | Colon | 5.9 |
| Macrophages rest | 1.0 | Lung | 13.8 |
| Macrophages LPS | 7.2 | Thymus | 48.0 |
| HUVEC none | 10.6 | Kidney | 20.4 |
| HUVEC starved | 17.3 | | |

**CNS_neurodegeneration_v1.0** Summary: Ag3288 Results from two experiments using the same probe/primer set are in excellent agreement. The CG57570-01 gene was found to upregulated in the temporal cortex of Alzheimer's disease (AD) patients by two separate TaqMan runs (p = 0.0007 when analyzed by ANCOVA); the temporal cortex shows marked neuronal loss in the early to middle stages of AD. Upregulation of CG57570-01 gene expression, however, was not apparent in the occipital cortex, where neuronal degeneration does not occur in AD. Taken together, these data suggest that the protein in question is involved in the pathologic process of Alzheimer's disease, making this an excellent small molecule drug target.

The CG57570-01 gene encodes a protein with homology to cation transporters. For example, iron transporters in the brain have been shown to play an important role in age-related neurodegenerative diseases, including Parkinson's disease, Alzheimer's disease, Huntington's disease and amyotrophic lateral sclerosis (ref. 1).

### References:

### 1. Qian ZM, Wang Q. Expression of iron transport proteins and excessive iron accumulation in the brain in neurodegenerative disorders. Brain Res Brain Res Rev 1998 Aug;27(3):257-67

New findings on the role of LfR (lactotransferrin receptor), MTf (melanotransferrin), CP (ceruloplasmin) and DCT1 (Divalent Cation Transporter) in brain iron transport, obtained during the past 3 years, are important advances in the fields of physiology and pathophysiology of brain iron metabolism. According to these findings, disruption in the expression of these proteins in the brain is probably one of the important causes of the altered brain iron metabolism in age-related neurodegenerative diseases, including Parkinson's Disease, Alzheimer's disease, Huntington's disease and amyotrophic lateral sclerosis. Further studies on the involvement of LfR, MTf and DCT1 in iron uptake by and CP in iron egress from different types of brain cells as well as control mechanisms of expression of these proteins in the brain are critical for elucidating the causes of excessive accumulation of iron in the brain and neuronal death in neurodegenerative diseases.

### PMID: 9729418

**General_screrning_panel_v1.4 Summary:** Ag3288 The CG57570-01 gene is expressed at high to moderate levels across almost all samples in this panel, with highest expression in the cerebellum (CT=26.7). This gene is also moderately expressed in all other regions of the CNS examined, including in amygdala, substantia nigra, thalamus, cerebral cortex, and spinal cord, suggesting that this gene may play a role in central nervous system disorders such as Alzheimer's disease, Parkinson's disease, epilepsy, multiple sclerosis, schizophrenia and depression.

This gene is also expressed in a number of tissues with metabolic or endocrine function, including adipose, adrenal gland, gastrointestinal tract, pancreas, skeletal muscle and thyroid. Therefore, therapeutic modulation of the activity of this gene may prove useful in the treatment of endocrine/metabolically related diseases, such as obesity and diabetes. Interestingly, this gene is differentially expressed in adult liver (CT = 34) vs fetal liver (CT = 29), suggesting that expression of this gene may be used to distinguish adult and fetal liver.

In addition, there is substantial expression of this gene associated with cancer cell lines. Therefore, therapeutic modulation of the activity of this gene or its protein product, through the use of small molecule drugs, protein therapeutics or antibodies, might be beneficial in the treatment of cancer.

**Panel 4D Summary**: Ag3288 The CG57570-01 gene is expressed at high to moderate levels in a wide range of cell types of significance in the immune response in health and disease, with the highest expression being detected in activated B cells (CT = 25). Therefore, targeting the CG57570-01 gene product with a small molecule drug or antibody therapeutic may modulate the functions of cells of the immune system, and particularly B cells, and lead to improvement of the symptoms of patients suffering from rheumatoid diseases, B hyperglobulinemia and autoimmune disorders.

### NOV14

Expression of gene CG57593-01 was assessed using the primer-probe set Ag3292, described in Table LA.

**Table LA. Probe Name Ag3292**

| **Primers** | **Sequences** | **Length** | **Start Position** | **SEQ ID NO** |
|---|---|---|---|---|
| Forward | 5'-tggtcataacatcaatcccaat-3' | 22 | 957 | 291 |
| Probe | TET-5'-tggcttcatggtgatattcacactct-3'-TAMRA | 26 | 991 | 292 |
| Reverse | 5'-gaaagccaacaccaaagaaag-3' | 21 | 1031 | 293 |

**CNS_neurodegeneration_v1.0 Summary**: Ag3292 Expression of the CG57593-01 gene is low/undetectable (CTs > 35) across all of the samples on this panel (data not shown).

**General_screening_panel_v1.4 Summary**: Ag3292 Expression of the CG57593-01 gene is low/undetectable (CTs > 35) across all of the samples on this panel (data not shown).

**Panel 4D Summary:** Ag3292 Expression of the CG57593-01 gene is low/undetectable (CTs > 35) across all of the samples on this panel (data not shown).

### NOV15

Expression ofNOV15/CG57652-01 was assessed using the primer-probe sets Ag1762 and Ag1763, described in Tables MA and MB. Results of the RTQ-PCR runs are shown in Tables MC and MD.

**Table MA. Probe Name Ag1762**

| **Primers** | **Sequences** | **Length** | **Start Position** | **SEQ ID NO** |
|---|---|---|---|---|
| Forward | 5'-caaacagggactgagctgtaac-3' | 22 | 766 | 295 |
| Probe | TET-5'-tacactgttcacgaccagtgtgccat-3'-TAMRA | 26 | 797 | 296 |
| Reverse | 5'-gcataggtgctgacttcacaa-3' | 21 | 835 | 297 |

**Table MB. Probe Name Ag1763**

| **Primers** | **Sequences** | **Length** | **Start Position** | **SEQ ID NO** |
|---|---|---|---|---|
| Forward | 5'-ggtgctctggaagttccagtat-3' | 22 | 1255 | 298 |
| Probe | TET-5'-accctcgacaggtgttcaacctccta-3'-TAMRA, | 26 | 1284 | 299 |
| Reverse | 5'-cttgaataatcggagccctatc-3' | 22 | 1324 | 300 |

**Table MC. Panel 1.3D**

| **Tissue Name** | **Rel. Exp.(%) Ag1762,Run 157720756** | **Rel. Exp.(%) Ag1763, Run 157717718** | **Tissue Name** | **Rel. Exp.(%) Ag1762,Run 157720756** | **Rel. Exp.(%) Ag1763, Run 157717718** |
|---|---|---|---|---|---|
| Liver adenocarcinoma | 26.6 | 25.7 | Kidney (fetal) | 2.5 | 3.7 |
| Pancreas | 1.6 | 2.8 | Renal ca. 786-0 | 0.2 | 0.1 |
| Pancreatic ca. CAPAN 2 | 9.2 | 8.1 | Renal ca. A498 | 20.3 | 18.7 |
| Adrenal gland | 6.7 | 6.2 | Renal ca. RXF 393 | 0.7 | 0.6 |
| Thyroid | 5.0 | 3.4 | Renal ca. ACHN | 4.7 | 4.4 |
| Salivary gland | 13.6 | 23.7 | Renal ca. UO-31 | 21.9 | 15.8 |
| Pituitary gland | 3.9 | 3.9 | Renal ca. TK-10 | 1.7 | 2.2 |
| Brain (fetal) | 1.7 | 3.6 | Liver | 1.1 | 1.0 |
| Brain (whole) | 6.6 | 8.2 | Liver (fetal) | 3.7 | 3.5 |
| Brain (amygdala) | 6.5 | 8.2 | Liver ca. (hepatoblast) HepG2 | 2.4 | 1.3 |
| Brain (cerebellum) | 2.1 | 3.4 | Lung | 15.9 | 16.7 |
| Brain (hippocampus) | 28.9 | 35.6 | Lung (fetal) | 7.7 | 12.6 |
| Brain (substantia nigra) | 0.8 | 1.3 | Lung ca.(small cell) LX-1 | 3.5 | 1.6 |
| Brain (thalamus) | 1.9 | 2.4 | Lung ca. (small cell) NCI-H69 | 2.4 | 2.3 |
| Cerebral Cortex | 25.5 | 18.3 | Lung ca. (s.cell var.) SHP-77 | 3.9 | 4.1 |
| Spinal cord | 2.2 | 3.3 | Lung ca. (large cell)NCI-H460 | 0.9 | 1.1 |
| glio/astro U87-MG | 4.9 | 5.6 | Lung ca. (non-sm. cell) A549 | 5.0 | 3.5 |
| glio/astro U-118-MG | 46.7 | 38.2 | Lung ca. (non-s.cell) NCI-H23 | 1.3 | 2.3 |
| astrocytoma SW1783 | 7.3 | 3.8 | Lung ca. (non-s.cell)HOP-62 | 1.5 | 2.4 |
| neuro*; met SK-N-AS | 24.1 | 25.7 | Lung ca. (non-s.cl) NCI-H522 | 3.0 | 2.9 |
| astrocytoma SF-539 | 4.8 | 5.8 | Lung ca. (squam.) SW 900 | 7.4 | 7.9 |
| astrocytoma SNB-75 | 15.1 | 10.0 | Lung ca. (squam.) NCI-H596 | 0.2 | 0.3 |
| glioma SNB-19 | 1.5 | 2.1 | Mammary gland | 7.9 | 9.2 |
| glioma U251 | 3.0 | 1.6 | Breast ca.* (pl.ef) MCF-7 | 16.5 | 1.2 |
| glioma SF-295 | 5.1 | 5.8 | Breast ca.* (pl.ef) MDA-MB-231 | 8.6 | 8.1 |
| Heart (fetal) | 3.2 | 1.4 | Breast ca.* (pl.ef) T T47D | 5.4. | 5.6 |
| Heart | 1.3 | 0.9 | Breast ca. BT-549 | 6.5 | 5.9 |
| Skeletal muscle (fetal) | 6.7 | 5.8 | Breast ca.MDA-N | 2.4 | 1.2 |
| Skeletal muscle | 0.8 | 0.3 | Ovary | 13.2 | 10.8 |
| Bone marrow | 15.0 | 14.7 | Ovarian ca. OVCAR-3 | 1.6 | 1.3 |
| Thymus | 100.0 | 100.0 | Ovarian ca. OVCAR-4 | 2.0 | 0.9 |
| Spleen | 24.5 | 39.0 | Ovarian ca. OVCAR-5 | 3.9 | 3.9 |
| Lymph node | 26.6 | 40.1 | Ovarian ca. OVCAR-8 | 11.8 | 9.2 |
| Colorectal | 7.5 | 8.7 | Ovarian ca. IGROV-1 | 1.5 | 2.7 |
| Stomach | 7.5 | 13.8 | Ovarian ca.* (ascites) SK-OV-3 | 8.9 | 6.0 |
| Small intestine | 19.8 | 19.5 | Uterus | 5.5 | 8.2 |
| Colon ca. SW480 | 5.1 | 6.1 | Placenta | 14.2 | 12.9 |
| Colon ca.* SW620(SW480 met) | 0.9 | 1.2 | Prostate | 7.6 | 10.7 |
| Colon ca. HT29 | 1.3 | 0.7 | Prostate ca.* (bone met)PC-3 | 10.7 | 11.1 |
| Colon ca. HCT-116 | 5.4 | 3.2 | Testis | 9.0 | 10.7 |
| Colon ca. CaCo-2 | 3.8 | 3.2 | Melanoma Hs688(A).T | 5.9 | 4.4 |
| Colon ca. tissue(OD03866) | 26.1 | 19.5 | Melanoma* (met) Hs688(B).T | 4.7 | 2.6 |
| Colon ca.HCC-2998 | 8.1 | 4.3 | Melanoma UACC-62 | 0.3 | 0.4 |
| Gastric ca.* (liver met) NCI-N87 | 21.6 | 21.0 | Melanoma M14 | 0.1 | 0.0 |
| Bladder | 3.7 | 2.0 | Melanoma LOX IMVI | 2.2 | 0.7 |
| Trachea | 46.7 | 50.3 | Melanoma* (met) SK-MEL-5 | 0.4 | 0.3 |
| Kidney | 0.2 | 0.7 | Adipose | 2.6 | 2.5 |

**Table MD. Panel 5 Islet**

| **Tissue Name** | **Rel. Exp.(%) Ag1763, Run 242285275** | **Tissue Name** | **Rel.Exp.(%)Ag1763, Run 242285275** |
|---|---|---|---|
| 97457_Patient-02go_adipose | 22.2 | 94709_Donor 2 AM - A_adipose | 55.9 |
| 97476_Patient-07sk_skeletal muscle | 7.5 | 94710_Donor 2 AM-B_adipose | 11.0 |
| 97477_Patient-07ut_uterus | 13.8 | 94711_Donor 2 AM-C_adipose | 16.0 |
| 97478_Patient-07pl_placenta | 34.2 | 94712_Donor 2 AD - A_adipose | 68.8 |
| 99167_Bayer Patient 1 | 47.3 | 94713_Donor 2 AD-B_adipose | 56.6 |
| 97482_Patient-08ut_uterus | 12.0 | 94714_Donor 2 AD-C_adipose | 59.5 |
| 97483_Patient-08pl_placenta | 29.5 | 94742_Donor 3 U-A_Mesenchymal Stem Cells | 33.2 |
| 97486_Patient-09sk_skeletal muscle | 1.9 | 94743_Donor 3 U-B_Mesenchymal Stem Cells | 26.4 |
| 97487_Patient-09ut_uterus | 21.5 | 94730_Donor 3 AM-A_adipose | 73.7 |
| 97488_Patient-09pl_placenta | 23.8 | 94731_Donor 3 AM-B_adipose | 26.1 |
| 97492_Patient-10ut_uterus | 23.7 | 94732_Donor 3 AM-C_adipose | 26.4 |
| 97493_Patient-10pl_placenta | 65.1 | 94733_Donor 3 AD-A_adipose | 55.5 |
| 97495_Patient-11go_adipose | 23.3 | 94734_Donor 3 AD-B_adipose | 31.0 |
| 97496_Patient-11sk_skeletat muscle | 4.4 | 94735 Donor 3 AD - C_adipose | 41.5 |
| 97497_Pattent-11ut_uterus | 12.9 | 77138_Liver_HepG2untreated | 35.6 |
| 97498_Patient- 11pl_placenta | 15.1 | 73556_Heart_Cardiac stromal cells (primary) | 44.8 |
| 97500_Patient-12go_adipose | 18.2 | 81735_Small Intestine | 74.7 |
| 97501_Patient-12sk_sketetal muscle | 9.2 | 72409_Kidney_Proximal Convoluted Tubule | 6.4 |
| 97502_Patient-12ut_uterus | 18.9 | 82685- Small intestine-Duodenum | 22.7 |
| 97503_Patient-12pl_placenta | 18.7 | 90650_Adrenal_Adrenocortical adenoma | 11.3 |
| 94721_Donor2U-A_Mesenchymal Stem Cells | 28.3 | 72410_Kidney_HRCE | 100.0 |
| 94722_Donor 2U - B_Mesenchymal Stem Cells | 22.8 | 72411_Kidney_HRE | 62.0 |
| 94723_Donor 2U - C_Mesenchymal Stem Cells | 37.1 | 73139_Uterus_Uterine smooth muscle cells | 16.4 |

**Panel 1.3D** Summary: Ag1762/Ag1763 Results from experiments using two different probe/primer sets are in excellent agreement. In both experiments, the CG57652-01 gene is expressed at highest levels in thymus tissue (CT = 27.3/25.8).

In addition, this gene is expressed at moderate levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

This gene is expressed in a number of tissues with metabolic or endocrine function, including adipose, adrenal gland, gastrointestinal tract, pancreas, skeletal muscle and thyroid. Therefore, therapeutic modulation of the activity of this gene may prove useful in the treatment of endocrine/metabolically related diseases, such as obesity and diabetes. The CG57652-01 gene encodes a variant of the diacylglycerol kinase-alpha gene (ref. 1). It has been shown that unsaturated fatty acids can lead to increased protein kinase C (PKC) activation by a deactivation of diacylglycerol kinase-alpha (ref. 2-3). Therefore, therapeutic modulation of the activity of the CG57652-01 gene or its protein product using small molecule drugs may be used to combat the negative effects of increased fatty acids in patients with diabetes.

### References:

### 1. Schaap D, de Widt J, van der Wal J, Vandekerckhove J, van Damme J, Gussow D, Ploegh HL, van Blitterswijk WJ, van der Bend RL. Purification, cDNA-cloning and expression of human diacylglycerol kinase. FEBS Lett 1990 Nov 26;275(1-2):151-8

Diacylglycerol (DG) kinase attenuates the level of the second messenger DG in signal transduction, and therefore possibly modulates protein kinase C (PKC). DG kinase was purified to homogeneity from human white blood cells, showing an Mr of 86 kDa as determined by SDS-PAGE and gel filtration. Two amino acid sequences of tryptic peptides from DG kinase were determined and degenerate oligonucleotides were prepared and used in the polymerase chain reaction. An amplified DNA fragment was subsequently used to clone the full-length human DG kinase cDNA. This sequence is the human homolog of a porcine DG kinase cDNA sequence reported recently. The sequence contains a double EF-hand structure typical for Ca2+ binding proteins. DG kinase further contains a double cysteine repeat that is present in all PKC isoforms, where it constitutes the phorbol ester (and most likely diacylglycerol) binding site. Therefore we speculate that the double cysteine repeat in DG kinase is involved in DG binding. DG kinase is transcribed as a single mRNA of 3.2 kb, that is highly expressed in T-lymphocytes. The human DG kinase cDNA when transfected in mammalian cells (COS-7) results in a 6-7-fold increase of DG kinase activity.

### PMID: 2175712

2. Du X, Jiang Y, Qian W, Lu X, Walsh JP. Fatty acids inhibit growth-factor-induced diacylglycerol kinase alpha activation in vascular smooth-muscle cells. Biochem J 2001 Jul 1;357(Pt 1):275-82.

We have previously shown that unsaturated fatty acids amplify platelet-derived-growth-factor (PDGF)-induced protein kinase C (PKC) activation in vascular smooth-muscle cells (VSMCs). Diacylglycerol-induced PKC activation is normally terminated by diacylglycerol kinases (DGKs). We thus hypothesized that fatty acids act by inhibiting a DGK. Fractionation of VSMC extracts demonstrated that the DGK alpha isoform was the major DGK activity present. PDGF markedly increased the DGK activity of cultured cells. An inhibitor selective for the DGK alpha isoform, R59949 [3-[2-[4-(bis-(4-fluorophenyl)methylene]piperidin-1-yl)ethyl]-2,3-dihydro-2-thioxo-4(1H)-quinazolinone], abolished the growth-factor-induced increase in DGK activity, but had little effect on basal activity. PDGF thus selectively activates DGKalpha. Epidermal growth factor and alpha-thrombin stimulated total DGK activity similarly to PDGF. Activation by epidermal growth factor was sensitive to R59949, again suggesting involvement of DGKalpha. However, the alpha-thrombin-induced activity was unaffected by this agent. Unsaturated fatty acids inhibited growth-factor-induced DGKalpha activation, but had no effect on basal activity. Fatty acids also amplified the PDGF-induced increase in cell diacylglycerol content. These results indicate that inhibition of DGKalpha contributes to fatty-acid-induced amplification of PKC activation. Increased levels of fatty acids in diabetes may thus contribute to chronic PKC activation associated with this disorder.

### PMID: 11415460

### 3. Koya D, Lee IK, Ishii H, Kanoh H, King GL. Prevention of glomerular dysfunction in diabetic rats by treatment with d-alpha-tocopherol. J Am Soc Nephrol 1997 Mar;8(3):426-35.

Because d-alpha-tocopherol (vitamin E) has been shown to decrease diacylglycerol (DAG) levels and prevent the activation of protein kinase C (PKC), which is associated with retinal and renal dysfunctions in diabetes, the study presented here characterized the effect of d-alpha-tocopherol treatment to prevent glomerular hyperfiltration and increased albuminuria as well as PKC activities in streptozotocin (STZ)-induced diabetic rats. Two weeks after the induction of diabetes, total DAG content and PKC activity in glomeruli were significantly increased in diabetic rats by 106.4 +/- 16.8% and 66.4 +/- 8.4%, respectively, compared with control rats. Intraperitoneal injection of d-alpha-tocopherol (40 mg/kg of body weight) every other day prevented the increases in total DAG content and PKC activity in glomeruli of diabetic rats. Glomerular filtration rate (GFR) and filtration fraction (FF) were significantly elevated to 4.98 +/- 0.34 mL/min and 0.36 +/- 0.05, respectively, in diabetic rats, compared with 2.90 +/- 0.14 mL/min and 0.25 +/-0.02. respectively, in control rats. These hemodynamic abnormalities in diabetic rats were normalized to 2.98 +/- 0.09 mL/min and 0.24 +/- 0.01, respectively, by d-alpha-tocopherol. Albuminuriain 10-wk diabetic rats was significantly increased to 9.1 +/- 2.2 mg/day compared with 1.2 +/- 0.3 mg/day in control rats, whereas d-alpha-tocopherol treatment improved albumin excretion rate to 2.4 +/- 0.6 mg/day in diabetic rats. To clarify the mechanism of d-alpha-tocopherol's effect on DAG-PKC pathway, the activity and protein levels of DAG kinase alpha and gamma, which metabolize DAG to phosphatidic acid, were examined. Treatment with d-alpha-tocopherol increased DAG kinase activity in the glomeruli of both control and diabetic rats, by 22.6 +/- 3.6% and 28.5 +/- 2.3% respectively, although no differences were observed in the basal DAG kinase activity between control and diabetic rats. Because immunoblotting studies did not exhibit any difference in the protein levels of DAG kinase alpha and gamma, the effect of d-alpha-tocopherol is probably modulating the enzyme kinetics of DAG kinase. These findings suggest that the increases in DAG-PKC pathway play an important role for the development of glomerular hyperfiltration and increased albuminuria in diabetes and that d-alpha-tocopherol treatment could be preventing early changes of diabetic renal dysfunctions by normalizing the increases in DAG and PKC levels in glomerular cells.

### PMID: 9071711

**Panel 5 Islet Summary:** Ag1763 The CG57652-01 gene is expressed at low levels in the majority of samples on this panel. This gene has a low level of expression in the islets of Langerhans (patient 1). Diacylglycerol kinase attenuates protein kinase C activation, which may in turn, reduce insulin secretion (ref. 1). Since the secretion of insulin is enhanced by activation of protein kinase C, an inhibitor of diacylglycerol kinase encoded by the CG57652-01 gene may be a treatment for the insulin secretory defect in Type 2 diabetes.

### References:

### 1. Formisano P, Beguinot F. The role of protein kinase C isoforms in insulin action. J Endocrinol Invest. 2001 Jun;24(6):460-7.

Insulin action on target tissues is mediated by specific tyrosine kinase receptors. Upon ligand binding insulin receptors autophosphorylate and phosphorylate intracellular substrates on tyrosine residues. These early events of insulin action are followed by the activation of a number of enzymes, including protein kinase C (PKC). At least 14 PKC isoforms have been identified and cloned to date. PKCs appear to play dual roles in insulin signaling. For instance, they are involved in transduction of specific insulin signals but also contribute to the generation of insulin resistance. In this article, we will analyze the experimental evidence addressing the mechanism by which insulin might activate individual PKC isoforms as well as the role of single PKCs in insulin-induced bioeffects.

### PMID: 11434672

### NOV16

Expression of NOV16/CG57562-01 was assessed using the primer-probe sets Ag3287 and Ag1179, described in Tables NA and NB. Results of the RTQ-PCR runs are shown in Tables NC, ND, NE and NF.

**Table NA. Probe Name Ag3287**

| **Primers** | **Sequences** | **Length** | **Start Position** | **SEQ ID NO** |
|---|---|---|---|---|
| Forward | 5'cgctacagatgttcaagatcct-3' | 22 | 3018 | 301 |
| Probe | TET-5'-ctacagccagagcgtcetctacctgg-3'-TAMRA | 26 | 3064 | 302 |
| Reverse | 5'-cctggaagtcactgaacttgac-3' | 22 | 3095 | 303 |

**Table NB. Probe Name Ag1179**

| **Primers** | **Sequences** | **Length** | **Start Position** | **SEQ ID NO** |
|---|---|---|---|---|
| Forward | 5'-cgctacagatgttcaagatcct-3' | 22 | 3018 | 304 |
| Probe | TET-5'-ctacagccagagcgtcctctacctgg-3'-TAMRA | 26 | 3064 | 305 |
| Reverse | 5'-cctggaagtcactgaacttgac-3' | 22 | 3095 | 306 |

**Table NC. CNS_neurodegeneration_v1.0**

| **Tissue Name** | **Rel.Exp.(%) Ag3287, Run 210058659** | **Tissue Name** | **Rel. Exp.(%) Ag3287, Run 210058659** |
|---|---|---|---|
| AD 1 Hippo | 23.2 | Control (Path) 3 Temporal Ctx | 6.8 |
| AD 2 Hippo | 35.6 | Control (Path) 4 Temporal | 42.0 |
| AD 3 Hippo | 11.8 | AD 1 Occipital Ctx | 19.9 |
| AD 4 Hippo | 11.9 | AD 2 Occipital Ctx (Missing) | 1.6 |
| AD 5 Hippo | 100.0 | AD 3 Occipital Ctx | 10.2 |
| AD 6 Hippo | 72.2 | AD 4 Occipital Ctx | 20.4 |
| Control 2 Hippo | 33.7 | AD 5 Occipital Ctx | 42.6 |
| Control 4 Hippo | 15.9 | AD 6 Occipital Ctx | 14.9 |
| Control (Path) Hippo | 9.1 | Control 1 Occipital Ctx | 5.0 |
| AD 1 Temporal Ctx | 34.2 | Control 2 Occipital Ctx | 60.3 |
| AD 2 Temporal Ctx | 37.9 | Control 3 Occipital Ctx | 22.4 |
| AD 3 Temporal Ctx | 10.2 | Control 4 Occipital Ctx | 6.7 |
| AD 4 Temporal Ctx | 26.4 | Control (Path) 1 Occipital Ctx | 86.5 |
| AD 5 Inf Temporal Ctx | 88.9 | Control (Path) 2 Occipital Ctx | 12.5 |
| AD 5 Sup Temporal Ctx | 64.6 | Control (Path) 3 Occipital Ctx | 3.5 |
| AD 6 Inf Temporal Ctx | 53.2 | Control (Path) 4 Occipital Ctx | 26.2 |
| AD 6 Sup Temporal Ctx | 62.0 | Control 1 Parietal Ctx | 10.4 |
| Control 1 Temporal Ctx | 8.2 | Control 2 Parietal Ctx | 52.5 |
| Control 2 Temporal Ctx | 39.8 | Control 3 Parietal Ctx | 21.9 |
| Control 3 Temporal Ctx | 17.6 | Control (Path) 1 Parietal Ctx | 62.4 |
| Control 3 Temporal Ctx | 6.3 | Control (Path) 2 Parietal Ctx | 24.1 |
| Control (Path) 1 Temporal Ctx | 54.0 | Control (Path) 3 Parietal Ctx | 6.5 |
| Control (Path) 2 Temporal Ctx | 41.2 | Control (Path) 4 Parietal Ctx | 32.5 |

**Table ND. General_screening_panel_v1.4**

| **Tissue Name** | **Rel. Exp.(%) Ag3287, Run 216516908** | **Tissue Name** | **Rel. Exp.(%) Ag3287, Run 216516908** |
|---|---|---|---|
| Adipose | 4.8 | Renal ca.TK-10 | 26.4 |
| Melanoma* Hs688(A).T | 23.0 | Bladder | 19.9 |
| Melanoma* Hs688(B).T | 27.9 | Gastric ca. (liver met.) NCI- | 44.4 |
| Melanoma* M14 | 31.6 | Gastric ca. KATO III | 39.8 |
| Melanoma* LOXIMVI | 33.7 | Colon ca. SW-948 | 24.3 |
| Melanoma* SK-MEL-5 | 31.0 | Colon ca. SW480 | 39.8 |
| Squamous cell carcinoma SCC-4 | 18.4 | Colon ca.* (SW480 met) SW620 | 33.0 |
| Testis Pool | 12.2 | Colon ca. HT29 | 10.0 |
| Prostate ca.*(bone met) PC-3 | 49.7 | Colon ca.HCT-116 | 48.3 |
| Prostate Pool | 6.8 | Colon ca. CaCo-2 | 46.7 |
| Placenta | 23.0 | Colon cancer tissue | 19.1 |
| Uterus Pool | 4.5 | Colon ca. SW1116 | 11.7 |
| Ovarian ca. OVCAR-3 | 22.7 | Colon ca. Colo-205 | 15.9 |
| Ovarian ca. SK-OV-3 | 48.0 | Colon ca. SW-48 | 10.9 |
| Ovarian ca. OVCAR-4 | 20.6 | Colon Pool | 16.5 |
| Ovarian ca. OVCAR-5 | 38.4 | Small Intestine Pool | 14.5 |
| Ovarian ca. IGROV-1 | 34.2 | Stomach Pool | 9.2 |
| Ovarian ca. OVCAR-8 | 13.5 | Bone Marrow Pool | 6.8 |
| Ovary | 11.7 | Fetal Heart | 18.0 |
| Breast ca. MCF-7 | 33.0 | Heart Pool | 5.2 |
| Breast ca. MDA-MB-231 | 49.3 | Lymph Node Pool | 19.8 |
| Breast ca. BT 549 | 100.0 | Fetal Skeletal Muscle | 10.5 |
| Breast ca. T47D | 76.8 | Skeletal Muscle Pool | 5.8 |
| Breast ca.MDA-N | 24.0 | Spleen Pool | 12.0 |
| Breast Pool | 18.4 | Thymus Pool | 19.1 |
| Trachea | 16.6 | CNS cancer (glio/astro) U87- | 36.1 |
| Lung | 5.5 | CNS cancer (glio/astro) U-118-MG | 65.5 |
| Fetal Lung | 42.6 | CNS cancer (neuro;met) SK-N-AS | 69.3 |
| Lung ca. NCI-N417 | 18.3 | CNS cancer (astro) SF-539 | 22.4 |
| Lung ca. LX-1 | 26.1 | CNS cancer (astro) SNB-75 | 49.0 |
| Lung ca. NCI.H146 | 17.8 | CNS cancer (glio) SNB-19 | 31.9 |
| Lung ca. SHP-77 | 37.9 | CNS cancer (glio) SF-295 | 84.7 |
| Lung ca. A549 | 24.1 | Brain (Amygdala) Pool | 7.5 |
| Lung ca. NCI-H526 | 7.3 | Brain (cerebellum) | 42.3 |
| Lung ca.NCI.H23 | 41.8 | Brain (fetal) | 26.8 |
| Lung ca. NCI.H460 | 34.6 | Brain (Hippocampus) Pool | 8.2 |
| Lung ca.HOP-62 | 13.4 | Cerebral Cortex Pool | 9.1 |
| Lung ca. NCI-H522 | 17.6 | Brain (Substantia nigra) Pool | 9.1 |
| Liver | 5.3 | Brain (Thalamus) Pool | 13.1 |
| Fetal Liver | 21.6 | Brain (whole) | 18.4 |
| Liver ca. HepG2 | 23.0 | Spinal Cord Pool | 6.6 |
| Kidney Pool | 22.1 | Adrenal Gland | 24.0 |
| Fetal Kidney | 24.1 | Pituitary gland Pool | 6.0 |
| Renal ca. 786-0 | 20.9 | Salivary Gland | 13.3 |
| Renal ca. A498 | 23.2 | Thyroid (female) | 9.5 |
| Renal ca. ACHN | 12.4 | Pancreatic ca. CAPAN2 | 44.8 |
| Renal ca. UO-31 | 27.7 | Pancreas Pool | 21.6 |

**Table NE. Panel 1.2**

| **Tissue Name** | **Rel. Exp.(%) Ag1179, Run 129140472** | **Tissue Name** | **Rel. Exp.(%) Ag1179, Run 129140472** |
|---|---|---|---|
| Endothelial cells | 6.0 | Renal ca. 786-0 | 1.8 |
| Heart (Fetal) | 25.2 | Renal ca.A498 | 16.6 |
| Pancreas | 3.5 | Renal ca. RXF 393 | 0.6 |
| Pancreatic ca. CAPAN 2 | 23.2 | Renal ca. ACHN | 8.0 |
| Adrenal Gland | 53.2 | Renal ca. UO-31 | 5.0 |
| Thyroid | 6.2 | Renal ca. TK-10 | 7.5 |
| Salivary gland | 26.4 | Liver | 15.6 |
| Pituitary gland | 14.4 | Liver(fetal) | 13.6 |
| Brain (fetal) | 17.9 | Liver ca. (hepatoblast) | 9.9 |
| Brain (whole) | 18.7 | Lung | 10.0 |
| Brain (amygdala) | 17.7 | Lung(fetal) | 13.6 |
| Brain (cerebellum), | 15.4 | Lung ca. (small cell) LX-1 | 3.0 |
| Brain (hippocampus) | 31.0 | Lung ca. (small cell) NCI-H69 | 3.5 |
| Brain(thalamus) | 9.3 | Lung ca. (s.cell var.) SHP-77 | 4.6 |
| Cerebral Cortex | 47.0 | Lung ca. (large cell)NCI-H460 | 100.0 |
| Spinal cord | 14.7 | Lung ca. (non-sm. cell) A549 | 5.0 |
| glio/astro U87-MG | 8.7 | Lung ca.(non-s.cell)NCI-H23 | 8.1 |
| glio/astro U-118-MG g | 13.3 | Lung ca. (non-s.cell) HOP-62 | 7.5 |
| \|astrocytoma SW1783 | 4.7 | Lung ca. (non-s.cl) NCI-H522 | 19.2 |
| neuro*;met SK-N-AS | 17.9 | Lung ca. (scquam.) SW 900 | 3.7 |
| astrocytoma SF-539 | 1.7 | Lung ca.(squam)NCI- NCI-H596 | 8.0 |
| astrocytoma SNB-75 | 2.2 | Mammary gland | 28.9 |
| glioma SNB-19 | 9.0 | Breast ca.*(pl.ef) MCF-7 | 8.2 |
| glioma U251 MB-231 | 3.1 | Breast ca.* (pl.ef) MDA- | 3.5 |
| glioma SF-295 | 49.7 | Breastca.*(pl.ef) T47D | 4.9 |
| Heart | 20.6 | Breast ca. BT-549 | 11.7 |
| Skeletal Muscle | 6.2 | Breast ca. MDA-N | 4.8 |
| Bone marrow | 7.5 | Ovary | 34.9 |
| Thymus | 12.8 | Ovarian ca. OVCAR-3 | 8.4 |
| Spleen | 9.3 | Ovarian ca. OVCAR-4 | 12.0 |
| Lymph node | 42.0 | Ovarian ca. OVCAR-5 | 9.7 |
| Colorectal Tissue | 10.8 | Ovarian ca. OVCAR-8 | 1.6 |
| Stomach | 67.8 | Ovarian ca. IGROV-1 | 8.6 |
| Small intestine | 20.3 | Ovarian ca. (ascites) SK- OC-3 | 16.7 |
| Colon ca. SW480 | 1.0 | Uterus | 9.2 |
| Colon ca.* SW620 (SW480 met) | 3.6 | Placenta | 31.2 |
| Colon ca. HT29 | 1.3 | Prostate | 29.7 |
| Colon ca. HCT-116 | 3.4 | Prostate ca.* (bone met) PC-3 | 72.7 |
| Colon ca. CaCo-2 | 7.2 | Testis | 45.4 |
| Colon ca. Tissue (OD03866) | 6.6 | Melanoma Hs688(A).T | 4.9 |
| Colon ca. HCC-2998 | 3.6 | Melanoma* (met) Hs688(B).T | 5.1 |
| Gastric ca.* (liver met) NCI-N87 | 9.6 | Melanoma UACC-62 | 34.9 |
| Bladder | 28.3 | MelanomaM14 | 21.9 |
| Trachea | 16.8 | Melanoma LOX 1MVI | 19.3 |
| Kidney | 4.6 | Melanoma* (met) SK-MEL-5 | 25.5 |
| Kidney (fetal) | 25.3 | | |

**Table NF. Panel 4D**

| **Tissue Name** | **Rel. Exp.(%) Ag1179, Run 139820117** | **Rel. Exp.(%) Ag3287, Run 164633941** | **Tissue Name** | **Rel.Exp.(%) Ag1179, Run 139820117** | **Rel.Exp.(%) Ag3287, Run 164633941** |
|---|---|---|---|---|---|
| Secondary Th1 act | 46.0 | 55.5 | HUVEC IL-1beta | 5.9 | 9.9 |
| Secondary Th2 act | 76.3 | 71.7 | HUVEC IFN gamma | 21.3 | 65.1 |
| SecondaryTr1 act | 38.4 | 49.7 | HUVEC TNF alpha + IFN ganum | 10.7 | 25.9 |
| Secondary Th1 rest | 15.0 | 18.2 | HUVEC TNF alpha+ IL4 | 17.1 | 23.5 |
| Secondery Th2 rest | 18.8 | 18.8 | HUVEC IL-11 | 9.3 | 14.4 |
| Secondary Tr1 rest | 10.7 | 23.5 | Lung Microvascular EC none | 17.6 | 26.8 |
| Primary Th1 act | 59.5 | 52.1 | Lung Microvascular EC TNFalpha+IL-1beta | 10.9 | 21.0 |
| Primary Th2 act | 47.0 | 40.1 | Microvascular Dermal EC none EC none | 37.9 | 26.1 |
| Primary Tr1 act | 75.3 | 68.8 | Microsvasular Dermal EC TNFalpha + IL-1beta | 21.3 | 17.7 |
| Primary Th1 rest | 52.1 | 60.3 | Bronchial epithelium TNFalpha+ IL1beta | 32.5 | 33.4 |
| Primary 1112 rest | 30.1 | 33.9 | Small airway epithelium none | 11.9 | 19.8 |
| Primary Tr1 rest | 25.3 | 25.7 | Small airway epithelium TNFalpha+IL-1beta | 58.6 | 73.2 |
| CD45RA CD4 lymphocyte act | 25.0 | 40.1 | Coronery artery SMC rest | 15.6 | 22.5 |
| CD45RO CD4 lymphocyte act | 56.3 | 63.7 | Coronery artery SMC TNFalpha+IL-1beta | 14.9 | 13.6 |
| CD8 lymphocyte act | 35.6 | 53.6 | Astrocytes rest | 15.2 | 21.8 |
| Secondary CD8 lymphocyte rest | 44.4 | 48.3 | Astrocytes TNFalpha + IL-1beta | 17.3 | 19.6 |
| Secondary CD8 lymphocyte act | 29.1 | 21.3 | KU-812 (Basophil) rest | 39.0 | 59.9 |
| CD4 lymphocyte none | 16.5 | 22.5 | KU-812 (Basophil) pMA/ionomycin | 83.5 | 83.5 |
| 2ry Th1/Th2/Tr1 anti-CD95 CH11 | 31.4 | 25.2 | CCD1106 (Keratinocytes) none | 16.4 | 24.1 |
| LAK cells rest | 40.6 | 43.8 | CCD1106 (Keratinocytes) TNFalpha+IL-1beta | 54.7 | 25.0 |
| LAKceDsIL-2 | 37.1 | 42.6 | Liver cirrhosis | 6.0 | 6.8 |
| LAK cells IL-2+IL-12 | 35.8 | 34.2 | Lupus kidney | 17.1 | 10.4 |
| LAK cells IL-2+IFN gamma | 39.8 | 42.0 | NCI-H292 none | 29.5 | 35.4 |
| LAK cells IL-2+IL-18 | 28.3 | 42.0 | NCI-H292 IL-4 | 41.8 | 73.2 |
| LAK cells PMA/ionomycin | 18.4 | 19.8 | NCI-H292 IL-9 | 35.4 | 48.6 |
| NK Cells IL-2 rest | 18.3 | 26.2 | NCI-H292 IL-13 | 39.8 | 40.9 |
| Two Way MLR 3 day | 27.9 | 50.0 | NCI-H292 IFN gamma | 24.5 | 48.3 |
| Two Way MLR 5 day | 21.9 | 29.5 | HPAEC none | 20.0 | 29.5 |
| Two Way MLR 7 day | 19.3 | 19.6 | HPAEC TNF alpha + IL-1 beta | 18.9 | 23.7 |
| PBMC rest | 10.8 | 11.5 | Lung fibroblast none | 20.0 | 22.8 |
| PBMCPWM | 100.0 | 94.6 | Lung fibroblast TNF alpha+IL-1 beta | 9.9 | 15.5 |
| PBMC PHA-L | 62.0 | 49.0 | Lung fibroblast IL-4 | 26.2 | 42.0 |
| Ramos (B cell) none | 69.7 | 39.8 | Lung fibroblast IL-9 | 19.3 | 28.5 |
| Ramos (B cell) ionomycin | 93.3 | 100.0 | Lung fibroblast IL-13 | 41.5 | 27.9 |
| B lymphocytes PWM | 79.6 | 91.4 | Lung fibroblast IFN gamma | 34.6 | 43.5 |
| B lymphocytes CD40L and IL-4 | 40.6 | 59.0 | Dermal fibroblast CCD1070 rest | 47.3 | 48.3 |
| EOL-1 dbcAMP | 43.2 | 76.3 | TNF alpha | 47.6 | 67.4 |
| EOL-1 dbcAMP PMA/ionomycin | 28.9 | 23.8 | Dermal fibroblast 23.8 CCD1070 IL-1 beta | 31.4 | 34.6 |
| Dendritic cells none | 30.8 | 36.3 | Dermal fibroblast IFN gamma | 10.2 | 18.6 |
| Dendritic cells LPS | 32.8 | 44.4 | Dermal fibroblast IL-4 | 23.7 | 24.8 |
| Dendritic cells anti-CD40 | 35.6 | 38.4 | IBD Colitis 2 | 2.6 | 2.8 |
| Monocytes rest | 28.1 | 37.9 | IBD Crohn's | 1.1 | 1.4 |
| Monocytes LPS | 37.9 | 24.3 | Colon | 17.0 | 23.7 |
| Macrophages rest | 58.2 | 67.4 | Lung | 13.9 | 21.6 |
| Macrophages LPS | 69.3 | 40.9 | Thymus | 39.0 | 31.6 |
| HUVEC none | 18.7 | 23.5 | Kidney | 40.6 | 63.3 |
| HUVEC starved | 24.8 | 34.9 | | | |

**CNS_neurodegeneration_v1.0 Summary:** Ag3287 This panel confirms the expression of this gene at low levels in the brain in an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel_v1.4 Summary:** Ag3287 The CG57562-01 gene shows moderate to high expression in all samples on this panel, with the highest expression in breast cancer cell line BT 549 (CT=25.0). The widespread expression of this gene suggests that the gene product may be involved in cell differentiation and growth.

This gene is also widely expressed among tissues with metabolic and endocrine function, including adipose, skeletal muscle, heart, pancreas, liver, adrenal gland, thyroid, and pituitary gland. This expression profile suggests that this gene product may also be involved in metabolic function and that therapeutic modulation of the expression or function of this gene may be effective in the treatment of metabolic disorders, such as obesity and diabetes.

The expression profile of this gene also shows widespread expression of this gene in the brain. This suggests that the protein encoded by this gene may be important for normal neurological function. Therefore, modulation of the function or expression of this gene may be effective in the treatment of neurodegenerative disorders, such as Alzheimer's disease and Parkinson's disease.

**Panel 1.2 Summary:** Ag1179 Results using the Ag1179 primer pair in this panel are in good agreement with the results using Ag3287 on panel 1.4. There is moderate to high expression in all samples on this panel, with the highest level of expression in lung cancer cell line NCI-H460 (CT=23.6). Please see General_screening_panel_v1.4 summary.

**Panel 4D Summary:** Ag1179/Ag3287 Results from two experiments using identical probe/primer sets are in excellent agreement. This gene is expressed at high to moderate levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues. This pattern is in agreement with the expression profile in General_screening_panel_v1.4 and also suggests a role for the gene product in cell survival and proliferation.

Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### NOV17

Expression of NOV17/CG55914-01 was assessed using the primer-probe sets Ag4424 and Ag2842, described in Tables OA and OB. Results of the RTQ-PCR runs are shown in Tables OC, OD, OE, OF, OG and OH.

**Table OA. Probe Name Ag4424**

| **Primers** | **Sequences** | **Length** | **Start Position** | **SEQ ID NO** |
|---|---|---|---|---|
| Forward | 5'-ctgaaagggtceaaggtacagt-3' | 22 | 341 | 307 |
| Probe | TET-5'-cttcaaagggtgeaagccccaagtct-3'-TAMRA | 26 | 374 | 308 |
| Reverse | 5'-gtactgccaggcttaacacctt-3' | 22 | 413 | 308 |

**Table OB. Probe Name Ag2842**

| **Primers** | **Sequences** | **Length** | **Start Position** | **SEQ ID NO** |
|---|---|---|---|---|
| Forward | 5'-gaaagggtccaaggtacagttc-3' | 22 | 343 | 310 |
| Probe | TET-5'-cttcaaagggtgcaagccccaagtct-3'-TAMRA | 26 | 374 | 311 |
| Reverse | 5'-caggcttaacaccttgtgaaag-3' | 22 | 406 | 312 |

**Table OC. General_screening_panel_v1.4**

| **Tissue Name** | **Rel. Exp.(%) Ag4424, Run 219941948** | **Tissue Name** | **Rel. Exp.(%) Ag4424, Run 219941948** |
|---|---|---|---|
| Adipose | 0.0 | Renal ca. TK-10 | 6.4 |
| Melanoma* Hs688(A).T | 0.0 | Bladder | 0.3 |
| Melanoma* Hs688(B).T | 0.0 | Gastric ca. (liver met.) NCI-N87 | 0.0 |
| Melanoma* M14 | 0.0 | Gastric ca. KATO III | 0.2 |
| Melanoma* LOXJMVI | 0.0 | Colon ca. SW-948 | 0.0 |
| Melanoma* SK-MEL-5 | 1.1 | Colon ca. SW480 | 0.0 |
| Squamous cell carcinoma SCC-4 | 0.7 | Colon ca.* (SW480met) SW620 | 0.0 |
| Testis Pool | 4.2 | Colon ca. HT29 | 0.0 |
| Prostate ca.* (bone met) PC-3 | 5.5 | Colon ca. HCT-116 | 0.0 |
| Prostate Pool | 1.1 | Colon ca. CaCo-2 | 2.7 |
| Placenta | 0.0 | Colon cancer tissue | 0.0 |
| Uterus Pool | 0.0 | Colon ca. SW1116 | 0.0 |
| Ovarian ca. OVCAR-3 | 100.0 | Colon ca. Colo-205 | 0.0 |
| Ovarian ca. SK-OV-3 | 7.4 | Colon ca. SW-48 | 0.0 |
| Ovarian ca. OVCAR-4 | 0.0 | Colon Pool | 0.0 |
| Ovarian ca. OVCAR-5 | 1.9 | Small Intestine Pool | 0.0 |
| Ovarian ca. IGROV-1 | 0.0 | Stomach Pool | 0.4 |
| Ovarian ca. OVCAR-8 | 3.7 | Bone Marrow Pool | 0.0 |
| Ovary | 0.9 | Fetal Heart | 0.0 |
| Breast ca. MCF-7 | 1.7 | Heart Pool | 0.0 |
| Breast ca. MDA-MB-231 | 0.0 | Lymph Node Pool | 0.6 |
| Breast ca.BT 549 | 0.4 | Fetal Skeletal Muscle | 0.0 |
| Breast ca. T47D | 3.6 | Skeletal Muscle Pool | 2.6 |
| Breast ca. MDA-N | 0.0 | Spleen Pool | 0.0 |
| Breast Pool | 0.8 | Thymus Pool | 0.0 |
| Trachea | 1.7 | CNS cancer (glio/astro) U87-MG | 0.0 |
| Lung | 2.0 | CNS cancer (glio/astro) U-118-MG | 0.0 |
| Fetal Lung | 0.3 | CNS cancer (neuro;met) SK-N-AS | 0.2 |
| Lung ca.NCI-N417 | 0.0 | CNS cancer (astro) SF-539 | 0.0 |
| Lung ca. LX-1 | 0.6 | CNS, cancer (astro) SNB-75 | 0.0 |
| Lung ca. NCI-H146 | 5.5 | CNS cancer (glio) SNB-19 | 0.0 |
| Lung ca.SHP-77 | 4.3 | CNS cancer (glio) SF-295 | 14.3 |
| Lung ca. A549 | 1.8 | Brain (Amygdala) Pool | 1.0 |
| Lung ca. NCI-H526 | 0.0 | Brain (cerebellum) | 0.0 |
| Lung ca. NCI-H23 | 1.6 | Brain (fetal) | 0.8 |
| Lung ca. NCI-H460 | 0.3 | Brain (Hippocampus) Pool | 0.0 |
| Lung ca. HOP-62 | 0.0 | Cerebral Cortex Pool | 3.0 |
| Lung ca. NCI-H522 | 1.1 | Brain (Substantia nigra) Pool | 1.4 |
| Liver | 0.0 | Brain (Thalamus) Pool | 0.3 |
| Fetal Liver | 2.0 | Brain (whole) | 0.1 |
| Liver ca. HepG2 | 0.0 | Spinal Cord Pool | 1.8 |
| Kidney Pool | 1.2 | Adrenal Gland | 0.0 |
| Fetal Kidney | 0.2 | Pituitary gland Pool | 0.4 |
| Renal ca. 786-0 | 0.0 | Salivary Gland | 0.0 |
| Renal ca. A498 | 0.0 | Thyroid (female) | 0.0 |
| Renal ca. ACHN | 0.0 | Pancreatic ca. CAPAN2 | 0.0 |
| Renal ca. UO-31 | 0.2 | Pancreas Pool | 1.0 |

**Table OD. Panel 1.3D**

| **Tissue Name** | **Rel. Exp.(%) Ag2842, Run 161559587** | **Rel. Exp.(%) Ag2842, Run 165701935** | **Tissue Name** | **Rel. Exp.(%) Ag2842, Run 161559587** | **Rel. Exp.(%) Ag2842, Run 165701935** |
|---|---|---|---|---|---|
| Liver adenocarcinoma | 0.0 | 0.0 | Kidney (fetal) | 0.0 | 0.0 |
| Pancreas | 0.0 | 0.0 | Renal ca. 786-0 | 0.0 | 0.0 |
| Pancreatic ca. CAPAN 2 | 0.0 | 0.0 | Renal ca. A498 | 0.0 | 0.0 |
| Adrenal gland | 0.0 | 0.0 | Renal ca. RXF 393 | 0.0 | 0.0 |
| Thyroid | 0.0 | 0.0 | Renal ca. ACHN | 0.0 | 0.0 |
| Salivary gland | 0.0 | 0.0 | Renal ca. UO-31 | 0.0 | 0.0 |
| Pituitary gland | 0.0 | 0.0 | Renal ca. TK-10 | 0.0 | 24.0 |
| Brain (fetal) | 0.0 | 0.0 | Liver | 0.0 | 10.4 |
| Brain (whole) | 0.0 | 0.0 | Liver (fetal) | 0.0 | 0.0 |
| Brain (amygdala) | 7.8 | 0.0 | Liver ca. (hepatoblast) HepG2 | 0.0 | 0.0 |
| Brain (cerebellum) | 0.0 | 0.0 | Lung | 0.0 | 0.0 |
| Brain (hippocampus) | 0.0 | 0.0 | Lung (fetal) | 0.0 | 0.0 |
| Brain (substantia nigra) | 0.0 | 0.0 | Lung ca. (small cell) LX-1 | 0.0 | 0.0 |
| Brain (thalamus) | 0.0 | 9.3 | Lung ca. (small cell) NCI-H69 | 30.8 | 0.0 |
| Cerebral Cortex | 6.3 | 7.4 | Lung ca (s.cell var.)SHP-77 | 19.1 | 9.6 |
| Spinal cord | 0.0 | 0.0 | Lung ca. (large cell)NCI-H460 | 0.0 | 9.6 |
| glio/astro U87-MG | 0.0 | 0.0 | Lung ca. (non-sm. cell) A549 | 0.0 | 6.9 |
| glio/astro U-118-MG | 0.0 | 0.0 | Lung ca. (non-s.cell)NCI-H23 | 0.0 | 18.0 |
| astrocytoma SW1783 | 0.0 | 0.0 | Lung ca. (non-s.cell)HOP-62 | 0.0 | 0.0 |
| Lung ca- (non-s.cl) neuro*; met SK-N-AS | 0.0 | 6.0 | NCl-H522 | 0.0 | 4.8 |
| astrocytoma SF-539 | 0.0 | 0.0 | Lung ca. (squam.) SW900 | 5.4 | 17.6 |
| astrocytoma SNB-75 | 5.4 | 9.4 | Lung ca. (squam.) NCI-H596 | 44.8 | 100.0 |
| glioma SNB-19 | 0.0 | 0.0 | Mammary gland | 5.8 | 0.0 |
| glioma U251 | 5.3 | 0.0 | Breast ca.* (pl.ef) MCF-7 | 0.0 | 0.0 |
| glioma SF-295 | 0.0 | 30.1 | Breast ca.* (pl.ef) MDA-MB-231 | 0.0 | 0.0 |
| Heart (fetal) | 0.0 | 0.0 | Breast ca* (Pl.ef) T47D | 0.0 | 0.0 |
| Heart | 0.0 | 0.0 | Breast ca. BT-549 | 0.0 | 0.0 |
| Skeletal muscle (fetal) | 8.8 | 0.0 | Breast ca.MDA-N | 0.0 | 0.0 |
| Skeletal muscle | 6.0 | 14.7 | Ovary | 0.0 | 0.0 |
| Bone marrow | 0.0 | 0.0 | Ovarian ca. OVCAR-3 | 100.0 | 64.6 |
| Thymus | 0.0 | 0.0 | Ovarian ca. OVCAR-4. | 0.0 | 7.7 |
| Spleen | 0.0 | 0.0 | Ovarian ca. OVCAR-5 | 0.0 | 0.0 |
| Lymph node | 0.0 | 0.0 | Ovarian ca. OVCAR-8 | 42.3 | 33.9 |
| Colorectal | 7.8 | 8.5 | Ovarian ca. IGROV-1 | 0.0 | 0.0 |
| Stomach | 0.0 | 0.0 | Ovarian ca.* (ascites) SK-OV-3 | 49.3 | 9.3 |
| Small intestine | 0.0 | 0.0 | Uterus | 0.0 | 0.0 |
| Colon ca. SW480 | 0.0 | 0.0 | Placenta | 0.0 | 0.0 |
| Colon ca.* SW620(SW480 met) | 0.0 | 0.0 | Prostate | 4.7 | 0.0 |
| Colon ca. HT29 | 0.0 | 0.0 | Prostate ca.* (bone met)PC-3 | 3.9 | 6.2 |
| Colon ca. HCT-116 | 0.0 | 0.0 | Testis | 39.8 | 7.5 |
| Colon ca. CaCo-2 | 5.7 | 0.0 | Melanoma Hs688(A).T | 0.0 | 0.0 |
| Colon ca. tissue(ODo3866) | 0.0 | 0.0 | Melanoma* (met) Hs688(B).T | 0.0 | 0.0 |
| Colon ca. HCC-2998 | 0.0 | 0.0 | Melanoma UACC-62 | 0.0 | 0.0 |
| Gastric ca.* (liver met) NCI-N87 | 0.0 | 0.0 | Melanoma M14 | 0.0 | 0.0 |
| Bladder | 2.4 | 0.0 | Melanoma LOX IMVI | 0.0 | 0.0 |
| Trachea | 0.0 | 0.0 | Melanoma* (met) SK-MeL-5 | 2.7 | 0.0 |
| Kidney | 6.0 | 0.0 | Adipose | 0.0 | 0.0 |

**Table OE Panel 2D**

| **Tissue Name** | **Rel. Exp.(%) Ag2842,Run 161559907** | **Tissue Name** | **Rel. Exp.(%) Ag2842, Run 161559907** |
|---|---|---|---|
| Normal Colon | 1.6 | Kidney Margin 8120608 | 0.8 |
| CC Well to Mod Diff (ODO3866) | 0.9 | Kidney Cancer 8120613 | 7.8 |
| CC Margin (ODO3866) | 0.0 | Kidney Margin 8120614 | 3.3 |
| CC Gr.2 rectosigmoid (ODO3868) | 1.1 | Kidney Cancer 9010320 | 4.1 |
| CC Margin (ODO3868) | 0.0 | Kidney Margin 9010321 | 2.4 |
| CC Mod Diff (ODO3920) | 0.2 | Normal Uterus | 0.0 |
| CC Margin (ODO3920) | 0.5 | Uterus Cancer 064011 | 1.6 |
| CC Gr.2 ascend colon (ODO3921) | 0.0 | Normal Thyroid | 1.5 |
| CC Margin (ODO3921) | 0.0 | Thyroid Cancer 064010 | 0.9 |
| CC from Partial Hepatectomy (OD04309) Mets | 2.8 | Thyroid Cancer A302152 | 0.0 |
| Liver Margin (OD04309) | 9.9 | Thyroid Margin A302153 | 0.0 |
| Colon mets to lung (OD04451-01) | 0.0 | Normal Breast | 2.1 |
| Lung Margin (OD04451-02) | 0.0 | Breast Cancer (OD04566) | 0.0 |
| Normal Prostate 6546-1 | 0.8 | Breast Cancer (OD04590-01) | 24.0 |
| Prostate Cancer (OD04410) | 9.0 | Breast Cancer Mets (OD04590-03) | 16.5 |
| Prostate Margin (OD04410) | 0.0 | Breast Cancer Metastasis (OD04655-O5) | 8.5 |
| Prostate Cancer (OD04720-01) | 3.8 | Breast Cancer 064006 | 2.1 |
| Prostate Margin (OD04720-02) | 3.6 | Breast Cancer 1024 | 2.2 |
| Normal Lung 061010 | 0.9 | Breast Cancer 9100266 | 1.8 |
| Lung Met to Muscle (OD04286) | 2.4 | Breast Margin 9100265 | 0.0 |
| MuscleMargin(OD64286) | 0.9 | Breast Cancer A209073 | 3.5 |
| Lung Malignant Cancer (OD03126) | 2.9 | Breast Margin A209073 | 7.1 |
| Lung Margin (OD03126) | 0.7 | Normal Liver | 4.0 |
| Lung Cancer (OD04404) | 0.0 | Liver Cancer 064003 | 2.6 |
| Lung Margin (OD04404) | 0.0 | Liver Cancer 1025 | 1.8 |
| Lung Cancer (OD04565) | 2.5 | Liver Cancer 1026 | 0.0 |
| Lung Margin (OD04565) | 0.0 | Liver Cancer 6004-T | 0.0 |
| Lung Cancer (OD04237-01) | 4.4 | Liver Tissue 6004-N | 3.0 |
| Lung Margin (OD04237-02) | 0.0 | Liver Cancer 6005-T | 0.0 |
| Ocular Mel Met to Liver (OD04310) | 1.2 | Liver Tissue 6005-N | 0.0 |
| Liver Margm(OD04310) | 6.8 | Normal Bladder | 1.9 |
| Melanoma Mets to Lung (OD04321) | 0.0 | Bladder Cancer 1023 | 0.7 |
| Lung Margin (OD04321) | 0.0 | Bladder Cancer A302173 | 6.0 |
| Normal Kidney | 14.6 | Bladder Cancer (OD04718-01) | 0.0 |
| Kidney Ca, Nuclear grade 2 (OD04338) | 0.0 | Bladder Normal Adjacent (OD04718-03) | 0.0 |
| KidneyMargin (OD04338) | 4.7 | Normal Ovary | 0.0 |
| Kidney Ca Nuclear grade 1/2 (OD04339) | 0.0 | Ovarian Cancer 064008 | 12.6 |
| KidneyMargin(OD04339) | 4.0 | Ovarian Cancer (OD04768-07) | 100.0 |
| Kidney Ca, Clear cell type (OD04340) , | 0.0 | Ovary Margin (OD04768-08) | 0.0 |
| Kidney Margin (OD04340) | 7.3 | Normal Stomach | 0.0 |
| Kidney Ca, Nuclear grade 3 (OD04348) | 0.0 | Gastric Cancer 9060358 | 0.0 |
| Kidney Margin (OD04348) | 1.0 | Stomach Margm 9060359 | 0.0 |
| Kidney Cancer (OD04622-01) | 0.0 | Gastric Cancer 9060395 | 0.0 |
| Kidney Margin (OD04622-03) | 0.8 | Stomach Margin 9060394 | 0.0 |
| Kidney Cancer (OD04450-01) | 0.7 | Gastric Cancer 9060397 | 0.0 |
| Kidney Margin (OD04450-03) | 3.9 | Stomach Margin 9060396 | 0.0 |
| Kidney Cancer 8120607 | 0.0 | Gastric Cancer 064005 | 0.0 |

**Table OF. Panel 3D**

| **Tissue Name** | **Rel. Exp.(%) Ag2842, Run 164843469** | **Tissue Name** | **Rel.Exp.(%) Ag2842, Run 164843469** |
|---|---|---|---|
| Daoy-Medulloblastoma | 0.0 | Ca Ski-Cervical epidermoid carcinoma (metastasis) | 0.0 |
| TE671- Medulloblastoma | 0.0 | ES-2- Ovarian clear cell carcinoma | 0.0 |
| D283 Med-Medutloblastoma | 0.0 | Ramos- Stimulated with PMA/ionomycin 6h | 0.0 |
| PFSK-1- Primitive Neuroectodermal | 0.0 | Ramos-Stimulated with PMA/ionomycin 14h | 0.0 |
| XF-498- CNS | 0.0 | MEG-01 - Chronic myelogenous leukemia (megokaryoblast) | 0.0 |
| SNB-78- Glioma | 0.0 | Raji-Burkitt's lymphoma | 0.0 |
| SF 268- Glioblastoma | 0.0 | Daudi-Burkitt's lymphoma | 0.0 |
| T98G- Glioblastoma | 0.0 | U266- B-cell plasmacytoma | 33.4 |
| SK-N-SH- Neuroblastoma (metastasis) ; | 0.0 | CA46- Burkitt's lymphoma | 0.0 |
| SF-295- Glioblastoma | 0.0 | RL-non-Hodgkin's B-cell lymphoma | 0.0 |
| Cerebellum | 80.7 | JM1-pre-B-cell lymphoma | 0.0 |
| Cerebellum | 0.0 | Jurkat- T cell leukemia | 0.0 |
| NCI-H292-Mucoepidermoid lung carcinoma | 15.4 | TF-1-Erythroleukemia | 0.0 |
| DMS-1 14- Small cell lung cancer | 0.0 | HUT 78-T-cell lymphoma | 0.0 |
| DMS-79-Small cell lung cancer | 0.0 | U937-Histiocytic lymphoma | 0.0 |
| NCI-H146-Small cell lung cancer | 89.5 | KU-812-Myelogenous leukemia | 0.0 |
| NCI-H526- Small cell lung cancer | 0.0 | 769-P-Clear cell renal carcinoma | 0.0 |
| NCI-N417- Small cell lung cancer | 0.0 | Caki-2-Clear cell renal carcinoma | 0.0 |
| NCI-H82-Small cell lung cancer | 0.0 | SW 839-Clear cell renal carcinoma | 1.8 |
| NCI-H157- Squamous cell lung cancer (metastasis) | 0.0 | G401- Wilms'tumor | 0.0 |
| NCI-H1155- Large cell lung cancer | 13.2 | Hs766T- Pancreatic carcinoma (LN metastasis) | 0.0 |
| NCI-H1299- Large cell lung cancer | 0.0 | CAPAN-1-Pancreatic adenocarcinoma (liver metastasis) | 0.0 |
| NCI-H727-Lung carcinoid | 0.0 | SU86.86- Pancreatic carcinoma (liver metastasis) | 0.0 |
| NCI-UMC-11-Lung carcinoid | 54.0 | BxPC-3-Pancreatic adenocarcinoma | 0.0 |
| LX-1- Small cell lung cancer | 0.0 | HPAC-Pancreatic adenocarcinoma | 0.0 |
| Colo-205- Colon cancer | 0.0 | MIA PaCa-2- Pancreatic carcinoma | 0.0 |
| KM12- Colon cancer | 0.0 | CFPAC-1-Pancreatic ductal adenocarcinoma | 59.9 |
| KM20L2- Colon cancer | 0.0 | PANC-1- Pancreatic epithelioid ductal carcinoma | 0.0 |
| NCI-H716- Colon cancer | 0.0 | T24-Bladder carcinma (transitional cell) | 0.0 |
| SW-48- Colon adenocarcinoma | 0.0 | 5637- Bladder carcinoma | 7.2 |
| SW1116-Colon adenocarcinoma | 0.0 | HT-1197- Bladder carcinoma | 36.6 |
| LS 174T-Colon adenocarcinoma | 0.0 | UM-UC-3-Bladder carcinma (transitional cell) | 0.0 |
| SW-948-Colon adenocarcinoma | 0.0 | A204-Rhabdomyosarcoma | 0.0 |
| SW-480- Colon adenocarcinoma | 0.0 | HT-1080-Fibrosarcoma | 100.0 |
| NCI-SNU-5- Gastric carcinoma | 0.0 | MG-63- Osteosarcoma | 0.0 |
| KATO III-Gastric carcinoma | 0.0 | SK-LMS- 1- Leiomyosarcoma (vulva) | 0.0 |
| NCI-SNU-16- Gastric carcinoma | 0.0 | SJRH30- Rhabdomyosarcoma (met to bone marrow) | 0.0 |
| NCI-SNU-1- Gastric carcinoma | 0.0 | A431-E pidermoid carcinoma | 0.0 |
| RF-1- Gastric adenocarcinoma | 0.0 | WM266-4- Melanoma | 0.0 |
| RF-48-Gastric adenocarcinoma | 0.0 | DU 145-Prostate carcinoma (brain metastasis) | 0.0 |
| MKN-45- Gastric carcinoma | 0.0 | MDA-MB-468- Breast adenocarcinoma | 44.8 |
| NCI-N87- Gastric carcinoma | 0.0 | SCC-4- Squamous cell carcinoma of tongue | 0.0 |
| OVCAR-5-Ovarian carcinoma | 0.0 | SCC-9- Squamous cell carcinoma of tongue | 0.0 |
| RL95-2- Uterine carcinoma | 0.0 | SCC-15- Squamous cell carcinoma of tongue | 0.0 |
| HelaS3- Cervical adenocarcinoma | 0.0 | CAL 27- Squamous cell carcinoma of tongue | 36.3 |

**Table OG. Panel 4D**

| **Tissue Name** | **Rel. Exp.(%) Ag2842, Run 159841917** | **Tissue Name** | **Rel. Exp.(%) Ag2842, Run 159841917** |
|---|---|---|---|
| SecondaryTh1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha+ IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha +IL-1beta | 0.0 |
| Primary Th2 act | 0.0 | Microvascular Dermal EC none | 0.0 |
| Primary Tr1 act | 0.0 | Microsvascular Dermal EC TNFalpha + IL-1beta | 0.0 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha +IL-Ibeta | 0.0 |
| CD45RA CD4 lymphocyte act | 0.0 | Coronery artery SMC rest | 0.0 |
| CD45RO CD4 lymphocyte act | 0.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha+IL-1beta | 0.0 |
| Secondary CD8 lymphocyte act | 0.0 | KU-812 (Basophil) rest | 0.0 |
| CD4 lymlphocyte none | 0.0 | KU-812 (Basophil) PMA/ionomycin | 0.0 |
| 2ry Th1/Th2/Tr1_anti-CD95 CH11 | 0.0 | CCD1106 (Keratinocytes) none | 0.0 |
| LAK cells Test | 0.0 | CCD1106 (Keratinocytes) TNFalpha + IL-1beta | 0.0 |
| LAK cells IL-2 | 0.0 | Liver cirrhosis | 0.0 |
| LAK cells IL-2+IL-12 | 0.0 | Lupus kidney | 0.0 |
| LAK cells IL-2+IFN gamma | 0.0 | NCI-H292 none | 0.0 |
| LAK cells IL-2 + IL-18 | 100.0 | NCI-H292 IL-4 | 0.0 |
| LAK cells PMA/ionomycin | 0.0 | NCI-H292 IL-9 | 0.1 |
| NK Cells IL-2 rest | 0.0 | NCI-H292 IL-13 | 0.0 |
| Two Way MLR 3 day | 0.0 | NCI-H292 IFN gamma | 0.0 |
| Two Way MLR 5 day | 0.0 | HPAEC none | 0.0 |
| Two Way MLR 7 day | 0.0 | HPAEC TNF alpha + IL-1 beta | 0.0 |
| PBMC rest | 0.0 | Lung fibroblast none | 0.0 |
| PBMC PWM | 0.0 | Lung fibroblast TNF alpha + IL-1 beta | 0.0 |
| PBMC PHA-L | 0.0 | Lung fibroblast IL-4 | 0.0 |
| Ramos (B cell) none | 0.0 | Lung fibroblast IL-9 | 0.0 |
| Ramos (B cell) ionomycin | 1.1 | Lung fibroblast IL-13 | 0.0 |
| B lymphocytes PWM | 0.0 | Lung fibroblast IFN gamma | 0.0 |
| B lymphocytes CD40L and IL-4 | 0.0 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 0.0 |
| Monocytes rest | 0.0 | IBD Crohn's | 0.0 |
| Monocytes LPS | 0.0 | Colon | 0.0 |
| Mactophages rest | 0.0 | Lung | 0.0 |
| Macrophages LPS | 0.0 | Thymus | 0.1 |
| HUVEC none | 0.0 | Kidney | 0.0 |
| HUVEC starved | 0.0 | | |

**Table OH. Panel 5D**

| **Tissue Name** | **Rel. Exp.(%) Ag2842, Run 223784832** | **Tissue Name** | **Rel. Exp.(%) Ag2842, Run 223784832** |
|---|---|---|---|
| 97457_Patient-02go_adipose | 0.0 | 94709_Donor 2 AM-A_adipose | 0.0 |
| 97476_Patient-07sk_skeletal muscle | 0.0 | 94710_Donor 2 AM-B_adipose | 0.0 |
| 97477_Patient-07ut_uterus | 0.0 | 94711_Donor 2 AM - C_adipose | 0.0 |
| 97478_Patient-07pl_placenta | 0.0 | 94712_Donor 2 AD - A_adipose | 0.0 |
| 97481_Patient-08sk_skeletal muscle | 0.0 | 94713_Donor 2 AD - B_adipose | 0.0 |
| 97482_Patient-08pl_uterus | 0.0 | 94714_Donor 2 AD - C_adipose | 29.9 |
| 97483_Patient-08pl_placenta | 0.0 | 94742_Donor 3 U - A_Mesenchymal Stein Cells | 0.0 |
| 97486_Patient-09sk_sketetal muscle | 0.0 | 94743_Donor3 U-B_Mesenchymal Stem Cells | 0.0 |
| 97487_Patient-09ut_uterus | 0.0 | 94730_Donor 3 AM - A_adipose | 0.0 |
| 97488_Patient-09pl_placenta | 0.0 | 94731_Donor 3 AM-B_adipose | 0.0 |
| 97492_Patient-10ut_uterus | 25.9 | 94732_Donor 3AM-C_adipose | 0.0 |
| 97493_Patient-10pl_placenta | 0.0 | 94733_Donor 3AD-A_adipose | 0.0 |
| 97495_Patient-11go_adipose | 0.0 | 94734_Donor3 AD-B_adipose | 0.0 |
| 97496_Pofient-11sk_skeletal muscle | 0.0 | 94735 Donor 3 AD-C adipose | 0.0 |
| 97497_Patient-11ut_uterus | 0.0 | 77138_Liver_HepG2untreated | 0.0 |
| 97498_Patient-11pt_placenta | 0.0 | 73556_Heart_Cardiac stromal cells | 0.0 |
| 97500_Patient-12go_adipose | 0.0 | 81735_Small Intestine | 0.0 |
| 97501_Patient-12sk_skeletal muscle | 100.0 | 72409_Kidney_Proximal Convoluted Tubule | 0.0 |
| 97502_Patient-12ut_uterus | 0.0 | 82685_Small intestine_Duodenum | 33.4 |
| 97503_Patient-12pl_placenta | 0.0 | 90650_Adrenal_Adrenocortical adenoma | 0.0 |
| 94721_Donor 2 U - A Mesenchymal Stem Cells | 0.0 | 72410_Kidney_HRCE | 0.0 |
| 94722_Donor 2 U - B_Mesenchymal Stem Cells | 0.0 | 72411_Kidney_HRE | 0.0 |
| 94723_Donor 2 U - C_Mesenchymal Stem Cells | 00 | 73139_Uterus_Uterine smooth muscle cells | 0.0 |

**General_screening_panel_v1.4 Summary:** Ag4424 Expression of the CG55914-01 gene is highest in ovarian cancer cell line OVCAR-3 (CT=29.5). Therefore, expression of this gene can be used to distinguish this cell line from the other samples on this panel. In addition, there is low but significant expression of this gene associated with an additional ovarian cancer and two lung cancer cell lines. Therefore, therapeutic modulation of the activity of this gene or its protein product, through the use of small molecule drugs, protein therapeutics or antibodies, might be beneficial in the treatment of lung cancer or ovarian cancer.

**Panel 1.3D Summary:** Ag2842 Results from two experiments using the same probe/primer pair gave similar results. Expression of the CG55914-01 gene is highest in ovarian and lung cancer cell lines, consistent with what is observed in General_screening_panel_v1.4.

**Panel 2D Summary:** Ag2842 Expression of this gene is highest in an ovarian cancer sample (CT = 30.5) and is significantly higher than in the matched normal ovarian tissue. In addition, this gene is expressed at significant levels in another ovarian tumor. Therefore, expression of this gene may be used to distinguish ovarian cancers from the other samples on this panel. Furthermore, therapeutic modulation of the activity of the protein encoded by this gene may be beneficial in the treatment of ovarian cancer.

**Panel 3D Summary:** Ag2842 This gene is expressed at low levels in a number of cancer cell lines including fibrosarcoma, lung cancer and pancreatic ductal adenocarcinoma. Therefore, expression of this gene may play a role in these types of cancers.

**Panel 4D Summary:** Ag2842 Expression of the CG55914-01 gene is highest in stimulated lymphokine-activated killer (LAK) cells (CT = 24.3). Therefore, expression of this gene can be used to distinguish this sample from the other samples on this panel. Since LAK cells are involved in tumor immunology and tumor cell clearance, as well as virally and bacterial infected cells, therapeutic modulation of this gene product may alter the functions of these cells and lead to improvement in cancer cell killing as well as host immunity to microbial and viral infections.

**Panel 5D Summary:** Ag2842 Results from one experiment with the CG55914-01 gene are not included. The amp plot indicates that there were experimental difficulties with this run.

### NOV18

Expression ofNOV18/CG57328-01 was assessed using the primer-probe set Ag3200, described in Table PA. Results of the RTQ-PCR runs are shown in Table PB.

**Table PA. Probe Name Ag3200**

| **Primers** | **Sequences** | **Length** | **Start Position** | **SEQ ID NO** |
|---|---|---|---|---|
| Forward | 5'-acaaccctacgtggatcattg-3' | 21 | 251 | 313 |
| Probe | TET-5'-ccctattgatggaacaactaagtttgtcca-3'-TAMRA | 30 | 273 | 314 |
| Reverse | 5'-tcgaaacagctacaaaaggaaa-3' | 22 | 307 | 315 |

**Table PB. Panel 1.3D**

| **Tissue Name** | **Rel. Exp.(%) Ag3200, Run 167994644** | **Tissue Name** | **Rel. Exp.(%) Ag3200, Run 167994644** |
|---|---|---|---|
| Liver adenocarcinoma, | 0.0 | Kidney (fetal) | 0.6 |
| Pancreas | 0.0 | Renal ca. 786-0 | 1.4 |
| Pancreatic ca. CAPAN 2 | 0.0 | Renal ca.A498 | 4.0 |
| Adrenal gland | 0.0 | Renal ca. RXF 393 | 0.5 |
| Thyroid | 0.8 | Renal ca.ACHN | 0.0 |
| Salivary gland | 0.0 | Renal ca.UO-31 | 0.0 |
| Pituitary gland | 12.0 | Renal ca. TK-10 | 7.9 |
| Brain (fetal) | 6.6 | Liver | 0.9 |
| Brain (whole) | 0.0 | Liver (fetal) | 0.0 |
| Brain (amygdala) | 0.0 | Liver ca. (hepatoblast) HepG2 | 0.0 |
| Brain (cerebellum) | 2.7 | Lung | 4.9 |
| Brain (hippocampus) | 0.0 | | 2.0 |
| Brain (substantia nigra) | 2.1 | Lung ca. (small cell) LX-1 | 0.0 |
| Brain (thalamus) | 0.0 | Lung ca. (small cell) NCI-H69 | 10.2 |
| Cerebral Cortex | 0.0 | Lung ca. (s.cell var.) SHP-77 | 54.3 |
| Spinal cord | 0.0 | Lung ca. (large cell)NCI-H460 | 0.0 |
| glio/astro U87-MG | 0.0 | Lung ca. (non-sm. cell) A549 | 0.4 |
| glio/astro U-118-MG | 0.0 | Lung ca. (non-s.cell) NCI-H23 | 0.4 |
| astrocytoma SW1783 | 0.0 | Lung ca. (non-s.cell) HOP-62 | 0.0 |
| neuro*; met SK-N-AS | 1.1 | Lung ca. (non-s.cl) NCI-H522 | 0.0 |
| astrocytoma SF-539 | 0.0 | Lung ca. (squam.) SW 900 | 0.0 |
| astrocytoma SNB-75 | 0.0 | Lung ca.(squam.)NCI-H596 | 100.0 |
| glioma SNB-19 | 0.0 | Mammary gland | 0.0 |
| glioma U251, | 2.1 | Breast ca. *(pl.ef)MCF-7 | 0.7 |
| glioma SF-295 | 0.0 | Breast ca.*(pl.ef) MDA-MB-231 | 0.0 |
| Heart (fetal) | 0.0 | Breastea.*(pl.ef) T47D | 2.0 |
| Heart | 6.4 | Breast ca. BT-549 | 0.0 |
| Skeletal muscle (fetal) | 0.0 | Breast ca. MDA-N | 0.9 |
| Skeletal muscle | 0.0 | Ovary | 0.0 |
| Bone marrow | 1.2 | Ovarian ca. OVCAR-3 | 1.1 |
| Thymus | 1.5 | Ovarian ca. OVCAR-4 | 0.0 |
| Spleen | 0.0 | Ovarian ca.OVCAR-5 | 1.6 |
| Lymph node | 1.8 | Ovarian ca. OVCAR·8 | 0.0 |
| Colorectal | 2.5 | Ovarian ca. IGROV-1 | 8.0 |
| Stomach | 0.0 | Ovarian ca.* (ascites) SK-OV-3 | 16.0 |
| Small intestine | 2.0 | Uterus | 0.0 |
| Colon ca. SW480 | 0.0 | Placenta | 0.0 |
| Colon ca.* SW620(SW480 met) | 3.1 | Prostate | 1.9 |
| Colon ca. HT29 | 1.6 | Prostate ca.* (bone met)PC-3 | 2.3 |
| Colon ca. HCT-116 | 2.0 | Testis | 1.9 |
| Colon ca. CaCo-2 | 5.0 | Melanoma Hs688(A).T | 0.0 |
| Colon ca. tissue(OD03866) | 0.7 | Melanoma* (met) Hs688(B).T | 0.0 |
| Colon ca HCC-2998 | 7.8 | Melanoma UACC-62 | 0.0 |
| Gastric ca.* (liver met) NCI-N87 | 8.9 | Melanoma M14 | 0.0 |
| Bladder | 3.1 | Melanoma LOX IMVI | 0.0 |
| Trachea | 2.2 | Melanoma* (met) SK-MEL-5 | 0.4 |
| Kidney | 0.0 | Adipose | 2.3 |

Panel 1.3D Summary: Ag3200 Significant expression of the CG57328-01 gene is restricted to two lung cancer cell lines (CT = 32-33). Thus, expression of this gene could be used to distinguish these samples from the other samples in the panel. In addition, therapeutic modulation of the activity of this gene or its protein product, through the use of small molecule drugs, protein therapeutics or antibodies, might be beneficial in the treatment of lung cancer.

### NOV19

Expression of NOV19 CG57358-01 was assessed using the primer-probe set Ag3214, described in Table QA. Results of the RTQ-PCR runs are shown in Tables QB, QC, QD and QE.

**Table QA. Probe Name Ag3214**

| **Primers** | **Sequences** | **Length** | **Start Position** | **SEQ ID NO** |
|---|---|---|---|---|
| Forward | 5'-ttcaacaggaaggtgattctca-3' | 22 | 520 | 316 |
| Probe | TET-5'-attttcttctggtcggccgtcaccft-3'-TAMRA | 26 | 550 | 317 |
| Reverse | 5'-aagtactgctggggaatgaag-3' | 21 | 585 | 318 |

**Table QB. CNS_neurodegeneration_v1.0**

| **Tissue Name** | **Rel. Exp.(%) Ag3214, Run 209861781** | **Tissue Name** | **Rel.Exp.(%) Ag3214,Run 209961781** |
|---|---|---|---|
| AD 1 Hippo | 19.5 | Control (Path) 3 Temporal Ctx | 5.4 |
| AD 2 Hippo | 23.5 | Control (Path) 4 Temporal Ctx | 25.9 |
| AD 3 Hippo | 14.8 | AD 1 Occipital Ctx | 19.3 |
| AD 4 Hippo | 8.5 | AD 2 Occipital Ctx (Missing) | 0.0 |
| AD 5 Hippo | 100.0 | AD 3 Occipital Ctx | 9.3 |
| AD 6 Hippo | 48.0 | AD 4 Occipital Ctx | 20.2 |
| Control 2 Hippo | 36.6 | AD 5 Occipital Ctx | 50.0 |
| Control 4 Hippo | 10.3 | AD 6 Occipital Ctx | 30.6 |
| Control (Path) 3 Hippo | 10.3 | Control 1 Occipital Ctx | 8.6 |
| AD 1 Temporal Ctx | 24.7 | Control 2 Occipital Ctx | 68.8 |
| AD 2 Temporal Ctx | 28.7 | Control 3 Occipital Ctx | 27.0 |
| AD 3 Temporal Ctx | 8.7 | Control 4 Occipital Ctx | 11.6 |
| AD 4 Temporal Ctx | 23.7 | Control (Path) 1 Occipital Ctx | 81.8 |
| AD 5 Inf Temporal Ctx | 95.3 | Control (Path) 2 Occipital Ctx | 14.8 |
| AD 5 Sup Temporal Ctx | 55.9 | Control (Path) 3 Occipital | 7.0 |
| AD 6 Inf Temporal Ctx | 52.9 | Control (Path) 4 Occipital Ctx | 17.0 |
| AD 6 Sup Temporal Ctx | 46.3 | Control 1 Parietal Ctx | 13.2 |
| Control 1 Temporal Ctx | 7.9 | Control 2 Parietal Ctx | 33.7 |
| Control 2 Temporal Ctx | 52.5 | Control 3 Parietal Ctx | 18.8 |
| Control 3 Temporal Ctx | 18.8 | Control (Path) 1 Parietal Ctx | 68.3 |
| Control 3 Temporal Ctx | 9.0 | Control (Path) 2 Parietal Ctx | 21.0 |
| Control (Path) Temporal Ctx | 52.1 | Control (Path) 3 Parietal Ctx | 5.6 |
| Control (Path) 2 Temporal Ctx | 26.6 | Control (Path) 4 Parietal Ctx | 38.2 |

**Table QC. Panel 1.3D**

| **Tissue Name** | **Rel. Exp.(%) Ag3214, Run 168012858** | **Tissue Name** | **Rel. Exp.(%) Ag3214, Run 168012858** |
|---|---|---|---|
| Liver adenocarcinoma | 0.4 | Kidney (fetal) | 18.8 |
| Pancreas | 3.1 | Renal ca. 786-0 | 4.3 |
| Pancreatic ca. CAPAN 2 | 5.8 | Renal ca. A498 | 0.6 |
| Adrenal gland | 0.8 | Renal ca. RXF 393 | 24.1 |
| Thyroid | 0.7 | Renal ca. ACHN | 75.3 |
| Salivary gland | 3.5 | Renal ca. UO-31 | 3.0 |
| Pituitary gland | 0.7 | Renal ca. TK-10 | 3.2 |
| Brain (fetal) | 7.3 | Liver | 1.5 |
| Brain (whole) | 21.9 | Liver (fetal) | 1.1 |
| Brain (amygdala) | 14.8 | Liver ca. (hepatoblast) HepG2 | 0.0 |
| Brain (cerebellum) | 9.0 | Lung | 16.6 |
| Brain (hippocampus) | 10.8 | Lung (fetal) | 14.3 |
| Brain (substantia nigra) | 12.9 | Lung ca. (small cell) LX-1 | 78.5 |
| Brain (thalamus) | 14.4 | Lung ca.(small cell)NCI H69 | 1.5 |
| Cerebral Cortex | 24.5 | Lung ca.(s.cell var.)SHP-77 | 10.8 |
| Spinal cord | 6.7 | Lung ca.(large cell) NCI-H460 | 0.2 |
| glio/astro U87-MG | 0.0 | Lung ca. (non-sm. cell) A549 | 0.4 |
| glio/astro U-118-MG | 0.9 | Lung ca. (non-s.cell) NCl-H23 | 0.1 |
| astrocytoma SW1783 | 0.1 | Lung ca. (non-s.cell)HOP-62 | 0.1 |
| neuro*; met SK-N-AS | 0.1 | Lung ca.(non-s.cl)NCI-H522 | 0.2 |
| astrocytoma SF-539 | 0.9 | Lung ca.(squam.)SW 900 | 8.4 |
| astrocytoma SNB-75 | 4.7 | Lung ca. (squam.) NCI-H596 | 0.9 |
| glioma SNB-19 | 0.1 | Mammary gland | 4.7 |
| glioma U251 | 0.1 | Breast ca.* (pl.ef)MCF-7 | 1.3 |
| glioma SF-295 | 0.1 | Breast ca.* (pl.ef) MDA-MB-231 | 4.5 |
| Heart (fetal) | 13.3 | Breast ca.* (pl.ef) T47D | 6.6 |
| Heart | 3.6 | Breast ca. BT-549 | 0.1 |
| Skeletal muscle (fetal) | 6.2 | Breast ca. MDA-N | 0.2 |
| Skeletal muscle | 1.5 | Ovary | 1.3 |
| Bone marrow | 1.7 | Ovarian ca. OVCAR-3 | 0.0 |
| Thymus | 1.3 | Ovarian ca. OVCAR-4 | 13.3 |
| Spleen | 3.0 | Ovarian ca. OVCAR-5 | 98.6 |
| Lymph node | 5.0 | Ovarian ca. OVCAR-8 | 0.2 |
| Colorectal | 1.0 | Ovarian ca. IGROV-1 | 8.4 |
| Stomach | 3.3 | Ovarian ca. * (ascites)SK-OV-3 | 0.1 |
| Small intestine | 3.8 | Uterus | 3.1 |
| Colon ca. SW480 | 3.6 | Placenta | 1.0 |
| Colon ca.* SW620 (SW480 met) | 100.0 | Prostate | 0.6 |
| Colon ca. HT29 | 13.0 | Prostate ca.* (bone met)PC- | 0.0 |
| Colon ca. HCT-116 | 0.2 | Testis | 0.6 |
| Colon ca. CaCo-2 | 0.1 | Melanoma Hs688 (A).T | 0.0 |
| Colon ca. tissue(OD03866) | 16.2 | Melanoma* (met) Hs688(B).T | 0.0 |
| Colon ca. HCC-2998 | 28.9 | Melanoma UACC-62 | 0.1 |
| Gastric ca. * (liver met) NCI-N87 | 6.2 | Melanoma M14 | 0.0 |
| Bladder | 11.0 | Melanoma LOX IMVI | 0.0 |
| Trachea | 2.1 | Melanoma* (met) SK-MEL-5 | 0.2 |
| Kidney | 32.5 | Adipose | 3.9 |

**Table QD. Panel 2.2**

| **Tissue Name** | **Rel.Exp.(%) Ag3214, Run 174416265** | **Tissue Name** | **Rel. Exp.(%) Ag3214, Run 174416265** |
|---|---|---|---|
| Normal Colon | 1.8 | Kidney Margin (OD04348) | 62.9 |
| Colon cancer (OD06064) | 37.6 | Kidney malignant cancer (OD06204B) | 0.8 |
| Colon Margin (OD06064) | 2.8 | Kidney normal adjacent tissue (OD06204E) | 26.4 |
| Colon cancer (OD06159) | 1.1 | Kidney Cancer (OD04450-01) | 33.9 |
| Colon Margin (OD06159) | 0.8 | Kidney Margin (OD04450-03) | 15.7 |
| Colon cancer (OD06297-04) | 1.9 | Kidney Cancer 8120613 | 0.4 |
| Coton Margin (OD06297-05) | 2.1 | Kidney Margin 8120614 | 58.6 |
| CC Gr.2 ascend colon | 5.3 | Kidney Cancer 9010320 | 3.5 |
| CC Margin (OD03921) | 0.6 | Kidney Margin 9010321 | 20.2 |
| Colon cancer metastasis (OD06104) | 3.4 | Kidney Cancer 8120607 | 100.0 |
| Lung Margin (OD06104) | 3.2 | Kidney Margin 8120608 | 47.6 |
| Colon mets to lung (OD04451-01) | 6.8 | Normal Uterus | 2.8 |
| Lung Margin(OD04451-02) | 5.0 | Uterine Cancer 064011 | 1.1 |
| Normal Prostate | 0.4 | Normal Thyroid | 0.5 |
| Prostate Cancer (OD04410) | 1.1 | Thyroid Cancer 064010 | 0.1 |
| Prostate Margin (OD04410) | 1.2 | Thyroid Cancer A302152 | 2.0 |
| Normal Ovary | 1.4 | Thyroid Margin A302153 | 0.7 |
| Ovarian cancer (OD06283-03) | 0.3 | Normal Breast | 1.7 |
| Ovarian Margin (OD06283-07) | 8.0 | Breast Cancer (OD04566) | 0.8 |
| Ovarian Cancer 064008 | 1.7 | Breast Cancer 1024 | 6.9 |
| Ovarian cancer (OD06145) | 1.1 | Breast Cancer (OD04590-01) | 3.0 |
| Ovarian Margin (OD06145) | 2.2 | Breast Cancer Mets (OD04590-03) | 1.6 |
| Ovarian cancer (OD06455-03) | 6.5 | Breast Cancer Metastasis (OD04655-03) | 2.9 |
| Ovarian Margin (OD064S5-07) | 1.3 | Breast Cancer 064006 | 2.3 |
| Normal Lung | 5.7 | Breast Cancer 9100266 | 1.4 |
| Invasive poor diff. lung adeno (OD04945-01 | 0.2 | Breast Margin 9100265 | 1.1 |
| Lung Margin (OD04945-03) | 6.2 | Breast Cancer A209073 | 1.9 |
| Lung Malignant Cancer (OD03126) | 15.6 | Breast Margin A2090734 | 2.1 |
| Lung Margin (OD03126) | 3.2 | Breast cancer (OD06083) | 11.9 |
| Lung Cancer (OD05014A) | 1.3 | Breast cancer node metastasis (OD06083) | 11.2 |
| Lung Margin (OD05014B) | 3.5 | Normal Liver | 1.9 |
| Lung cancer (OD06081) | 1.4 | Liver Cancer 1026 | 2.5 |
| Lung Margin (OD06081) | 4.9 | Liver Cancer 1025 | 3.1 |
| Lung Cancer (OD04237-01) | 1.0 | Liver Cancer 6004-T | 1.8 |
| Lung Margin (OD04237-02) | 12.3 | Liver Tissue 6004-N | 0.0 |
| Ocular Melanoma Metastasis | 0.0 | Liver Cancer 6005-T | 2.2 |
| Ocular Melanoma Margin (Liver) | 1.0 | Liver Tissue 6005-N | 11.2 |
| Melanoma Metastasis | 0.2 | Liver Cancer 064003 | 1.0 |
| Melanoma Margin (Lung) | 4.2 | Normal Bladder | 8.1 |
| Normal Kidney | 9.1 | Bladder Cancer 1023 | 4.1 |
| Kidney Ca, Nuclear grade 2 (OD04a338) | 24.8 | Bladder Cancer A302173 | 0.6 |
| Kidney Margin (OD04338) | 11.7 | Normal Stomach | 3.8 |
| Kidney Ca Nuclear grade 1/2 (OD04339) | 5.1 | Gastric Cancer 9060397 | 15.2 |
| Kidney Margin (OD04339) | 23.0 | Stomach Margin 9060396 | 11.7 |
| Kidney Ca, Clear cell type (OD04340) | 14.1 | Gastric Cancer 9060395 | 7.1 |
| Kidney Margin (OD04340) | 16.6 | Stomach Margin 9060394 | 17.2 |
| Kidney Ca, Nuclear grade 3 (OD04348) | 0.7 | Gastric Cancer 064005 | 3.7 |

**Table QE. Panel 4D**

| **Tissue Name** | **Rel. Exp.(%) Ag3214, Run 164682510** | **Tissue Name** | **Rel. Exp.(%) Ag3214, Run 164682510** |
|---|---|---|---|
| Secondary Th1 act | 0.3 | HUVEC IL-1beta | 3.5 |
| Secondary Th2 act | 0.2 | HUVEC IFN gamma | 14.4 |
| Secondary Tr1 act | 0.6 | HUVEC TNF alpha + IFN gamma | 6.0 |
| Secondary Th1 rest | 0.5 | HUVEC TNF alpha + IL4 | 5.8 |
| Secondary Th2 rest | 1.4 | HUVEC IL-11 | 24.8 |
| Secondary Tr1 rest | 1.1 | Lung Microvascular EC none | 50.0 |
| Primary Th1 act | 0.2 | Lung Microvascular EC TNFalpha + IL-1 beta | 40.6 |
| Primary Th2 act | 0.1 | Microvascular Dermal EC none | 32.1 |
| Primary Tr1 act | 0.0 | Microsvasular Dermal EC TNFalpha + IL-1beta | 24.8 |
| Primary Th1 rest | 0.8 | Bronchial epithelium TNFalpha+ IL1beta | 3.0 |
| Primary Th2 rest | 0.7 | Small airway epithelium none | 9.0 |
| Primary Tr1 rest | 1.6 | Small airway epithelium TNFalpha +IL-1beta | 19.6 |
| CD45RA CD41ymphocyte act | 0.1 | Coronery artery SMC rest | 0.2 |
| CD45RO CD4 lymphocyte act | 0.3 | Coronery artery SMC TNFalpha + IL-1beta | 0.0 |
| CD8 lymphocyte act | 0.1 | Astrocytes rest | 3.5 |
| Secondary CD8 lymphocyte rest | 0.0 | Astrocytes TNFalpha+IL-1beta | 5.0 |
| Secondary CD8 lymphocyte act | 0.0 | KU-812 (Basophil) rest | 0.6 |
| CD4 lymphocyte none | 0.4 | KU-812 (Basophil) PMA/ionomycin | 0.4 |
| 2ry Th1/Th2/Tr1 anti-CD95 CH11 | 0.9 | CCD1106 (Keratinocytes) none | 0.8 |
| LAK cells rest | 0.1 | CCD1106 (Keratinocytes) TNFalpha + IL- 1beta | 0.1 |
| LAK cells IL-2 | 0.4 | Liver cirrhosis | 6.3 |
| LAK cells-2+IL-12 | 0.2 | Lupus kidney | 6.1 |
| LAK cells IL-2+IFN gamma | 0.2 | NCI-H292 none | 1.3 |
| LAK cells IL-2+IL-18 | 0.0 | NCI-H292 IL-4 | 1.0 |
| LAK cells PMA/ionomycin | 0.0 | NCI-H292IL-9 | 2.2 |
| NK Cells IL-2 rest | 0.2 | NCI-H292 IL-13 | 0.7 |
| Two Way MLR 3 day | 0.0 | NCI-H292 IFN gamma | 2.3 |
| Two Way MLR 5 day | 0.3 | HPAEC none | 15.4 |
| Two Way MLR 7 day | 0.0 | HPAEC TNF alpha + IL-1 beta | 2.1 |
| PBMC rest | 0.1 | Lung fibroblast none | 0.0 |
| PBMC PWM | 0.3 | Lung fibroblast TNF alpha + IL-1 beta | 0.1 |
| PBMC PHA-L | 0.6 | Lung fibroblast IL-4 | 0.3 |
| Ramos (B cell) none | 0.0 | Lung fibroblast IL-9 | 0.0 |
| Ramos (B cell) ionomycin | 0.2 | Lung fibroblast IL-13 | 0.0 |
| B lymphocytes PWM | 0.1 | Lung fibroblast IFN gamma | 0.1 |
| B lymphocytes CD40L and IL-4 | 0.2 | Dermal fibroblast CCD1070 rest | 0.0 |
| EOL-1 dbcAMP | 3.5 | Dermal fibroblast CCD1070 TNF alpha | 2.9 |
| EOL-1 dbcAMP PMA/ionomycin | 6.6 | Dermal fibroblast CCD1070 IL-1 beta | 0.0 |
| Dendritic cells none | 0.1 | Dermal fibroblast IFN gamma | 0.0 |
| Dendritic cells LPS | 0.2 | Dermal fibroblast IL-4 | 0.0 |
| Dendritic cells anti-CD40 | 0.2 | IBD Colitis 2 | 0.3 |
| Monocytes rest | 0.5 | IBD Crohn's | 0.9 |
| Monocytes LPS | 0.1 | Colon | 9.1 |
| Macrophages rest | 0.1 | Lung | 20.0 |
| Macrophages LPS | 0.0 | Thymus | 100.0 |
| HUVEC none | 12.1 | Kidney | 5.8 |
| HUVEC starved | 23.5 | | |

**CNS_neurodegeneration_v1.0 Summary:** Ag3214 This panel confirms the expression of this gene at low levels in the brain in an independent group of individuals. specificity. GABAA receptors are of great clinical significance in several disorders, including epilepsy, anxiety and alcoholism. In addition to treating epilepsy with drugs that target GABAA and BZ binding sites, epileptic lesions can be localised presurgically using radiolabelled BZ ligands. BZs are used commonly to treat anxiety, and studies suggest that BZ antagonists and inverse agonists (which induce the opposite effect to agonists at receptors) may be useful in alcohol rehabilitation.

**Panel 2D Summary:** Ag1680 Expression of the CG57654-01 gene is highest in a sample of normal colon tissues (CT = 33.6). There appears to be a general pattern of overexpression of this gene in the normal matched colon samples when compared to the adjacent colon tumors. Therefore, therapeutic modulation of the activity of this gene or its protein product, using small molecule drugs, antibodies or protein therapeutics, may be of use in the treatment of colon cancer.

**Panel 4D Summary:** Ag1680 Expression of the CG57654-01 gene is highest in the lung-derived mucoepidermoid cell line (CT = 34.7). Therefore, therapeutic modulation of the activity of this gene, using small molecule drugs or antibodies, may be of use in the treatment of asthma and emphysema.

### NOV22

Expression of gene NOV22/CG57724-01 was assessed using the primer-probe set Ag3316, described in Table TA. Results of the RTQ-PCR runs are shown in Tables TB, TC and TD.

**Table TA. Probe Name Ag3316**

| **Primers** | **Sequences** | **Length** | **Start Position** | **SEQ ID NO** |
|---|---|---|---|---|
| Forward | 5'-tagttcaagatgccactttcgt-3' | 22 | 1175 | 322 |
| Probe | TET-5'-actgcagactgctcaetaceaccgag-3'-TAMRA | 26 | 1206 | 323 |
| Reverse | 5'-cgtggtgctcaaattcataca-3' | 21 | 1253 | 324 |

**Table TB. CNS_neurodegeneration_v1.0**

| **Tissue Name** | **Rel. Exp.(%) Ag3316, Run 210144080** | **Tissue Name** | **ReL Exp.(%) Ag3316, Rum 210144080** |
|---|---|---|---|
| AD 1 Hippo | 2.1 | Control (Path) 3 Temporal Ctx | 4.6 |
| AD 2 Hippo | 35.8 | Control (Path) 4 Temporal | 71.7 |
| AD 3 Hippo | 5.1 | AD 1 Occipital Ctx | 13.9 |
| AD 4 Hippo | 19.5 | AD 2 Occipital Ctx (Missing) | 0.0 |
| AD 5 Hippo | 26.1 | AD 3 Occipital Ctx | 1.4 |
| AD 6 Hippo | 81.8 | AD 4 Occipital Ctx | 37.9 |
| Control 2 Hippo | 52.9 | AD 5 Occipital Ctx | 55.5 |
| Control 4 Hippo | 5.2 | AD 6 Occiptal Ctx | 35.1 |
| Control (Path) 3 Hippo | 4.2 | Control 1 Occipital Ctx | 2.8 |
| AD 1 Temporal Ctx | 18.4 | Control 2 Occipital Ctx | 40.9 |
| AD 2 Temporal Ctx | 47.6 | Control 3 Occipital Ctx | 31.9 |
| AD 3 Temporal Ctx | 2.5 | Control 4 Occipital Ctx | 2.1 |
| AD 4 Temporal Ctx | 40.9 | Control (Path) 1 Occipital Ctx | 87.1 |
| AD 5 Inf Temporal Ctx | 74.2 | Control (Path) 2 Occipital Ctx | 17.2 |
| AD 5 Sup Temporal Ctx | 25.9 | Control (Path) 3 Occipital Ctx | 0.0 |
| AD 6 Inf Temporal Ctx | 100.0 | Control (Path) 4 Occipital Ctx | 29.7 |
| AD 6 Sup Temporal Ctx | 88.3 | Control 1 Parietal Ctx | 6.2 |
| Control 1 Temporal Ctx | 5.8 | Control 2 Parietal Ctx | 25.9 |
| Control 2 Temporal Ctx | 46.7 | Control 3 Parietal Ctx | 23.0 |
| Control 3 Temporal Ctx | 54.3 | Control (Path) 1 Parietal | 57.0 |
| Control 3 Temporal Ctx | 5.7 | Control (Path) 2 Parietal Ctx | 34.4 |
| Control (Path) 1 Temporal Ctx | 85.3 | Control (Path) 3 Parietal Ctx | 2.8 |
| Control (Path) 2 Temporal Ctx | 59.9 | Control (Path) 4 Parietal Ctx | 53.2 |

**Table TC. General_screening_panel_v1.4**

| **Tissue Name** | **Rel. Exp.(%) Ag3316, Run 215678598** | **Tissue Name** | **Rel. Exp.(%) Ag3316, Run 215678598** |
|---|---|---|---|
| Adipose | 12.4 | Renal ca.TK-10 | 0.4 |
| Melanoma* Hs688(A).T | 2.5 | Bladder | 8.1 |
| Melanoma* Hs688(B).T | 4.0 | Gastric ca. (liver met.) NCI-N87 | 0.0 |
| Melanoma* M14 | 0.0 | Gastric ca. KATO III | 0.6 |
| Melanoma* LOXIMVI | 0.0 | Colon ca. SW-948 | 2.7 |
| Melanoma* SK-MEL-5 | 0.0 | Colon ca. SW480 | 0.0 |
| Squamous cell carcinoma SCC-4 | 0.6 | Colon ca.* (SW480 met) SW620 | 0.0 |
| Testis Pool | 28.5 | Colon ca. HT29 | 3.0 |
| Prostate ca.* (bone met) PC-3 | 8.8 | Colon ca. HCT-116 | 0.0 |
| Prostate Pool | 11.3 | Colon ca. CaCo-2 | 0.0 |
| Placenta | 7.2 | Colon cancer tissue | 4.3 |
| Uterus Pool | 1.0 | Colon ca. SW1116 | 0.0 |
| Ovarian ca. OVCAR-3 | 8.5 | Colon ca. Colo-205 | 0.6 |
| Ovarian ca. SK-OV-3 | 4.6 | Colon ca. SW-48 | 3.5 |
| Ovarian ca. OVCAR-4 | 0.0 | Colon Pool | 3.7 |
| Ovarian ca. OVCAR-5 | 2.2 | Small Intestine Pool | 10.2 |
| Ovarian ca. IGROV-1 | 0.0 | Stomach Pool | 2.7 |
| Ovarian ca. OVCAR-8 | 1.0 | Bone Marrow Pool | 3.6 |
| Ovary | 2.9 | Fetal Heart | 1.5 |
| Breast ca. MCF-7 | 10.2 | Heart Pool | 2.6 |
| Breast ca. MDA-MB-231 | 6.5 | Lymph Node Pool | 3.8 |
| Breast ca. BT 549 | 12.1 | Fetal Skeletal Muscle | 1.7 |
| Breast ca. T47D | 2.7 | Skeletal Muscle Pool | 2.5 |
| Breast ca. MDA-N | 0.0 | Spleen Pool | 3.7 |
| Breast Pool | 4.5 | Thymus Pool | 8.4 |
| Trachea | 80.7 | CNS cancer (glio/astro) U87-MG | 0.2 |
| Lung | 2.1 | CNS cancer (glio/astro) U-118-MG | 0.4 |
| Fetal Lung | 29.3 | cancer (neuro;met) SK-N-AS | 1.1 |
| Lung ca.NCI-N417 | 0.0 | CNS cancer (astro) SF-539 | 0.0 |
| Lung ca. LX-1 | 2.0 | CNS cancer (astro) SNB-75 | 0.0 |
| Lung ca. NCI-H146 | 2.6 | CNS cancer (glio) SNB-19 | 0.4 |
| Lung ca.SHP-77 | 5.8 | CNS cancer (glio) SF-295 | 0.2 |
| Lung ca. A549 | 3.4 | Brain (Amygdala) Pool | 14.4 |
| Lung ca. NCI-H526 | 2.7 | Brain (cerebellum) | 35.6 |
| Lung ca. NCI-H23 | 100.0 | Brain (fetal) | 9.4 |
| Lung ca. NCI-H460 | 0.0 | Brain (Hippocampus) Pool | 11.8 |
| Lung ca. HOP-62 | 3.5 | Cerebral Cortex Pool | 18.0 |
| Lung ca. NCI-H522 | 14.2 | Brain (Substantia nigra) Pool | 12.0 |
| Liver | 13.4 | Brain (Thalamus) Pool | 17.4 |
| Fetal Liver | 12.3 | Brain (whole) | 17.6 |
| Liver ca. HepG2 | 0.0 | Spinal Cord Pool | 5.9 |
| Kidney Pool | 6.0 | Adrenal Gland | 22.5 |
| Fetal Kidney | 16.2 | Pituitary gland Pool | 31.0 |
| Renal ca. 786-0 | 0.8 | Salivary Gland | 32.3 |
| Renal ca. A498 | 4.9 | Thyroid (female) | 7.3 |
| Renal ca. ACHN | 4.2 | Pancreatic ca. CAPAN2 | 10.2 |
| Renal ca. UO-31 | 2.3 | Pancreas Pool | 8.0 |

**Table TD. Panel 4D**

| **Tissue Name** | **Rel. Exp.(%) Ag3316, Run 164683048** | **Tissue Name** | **Rel. Exp.(%) Ag3316, Run 164683048** |
|---|---|---|---|
| Secondary Th1 act | 46.0 | HUVEC IL-1beta | 7.8 |
| Secondary Th2 act | 60.7 | HUVEC IFN gamma | 5.0 |
| Secondary Tr1 act | 88.3 | HUVEC TNF alpha + IFN gamma | 2.2 |
| Secondary Th1 rest | 31.2 | HUVEC TNF alpha + IL4 | 2.5 |
| Secondary Th2 rest | 57.4 | HUVEC IL-11 | 2.4 |
| Secondary Tr1 rest | 72.7 | Lung Microvascular EC none | 7.5 |
| Primary Th1 act | 23.3 | Lung Microvascular EC TNFalpha + IL-1beta | 3.8 |
| Primary Th2 act | 59.5 | Microvascular Dermal EC none | 7.8 |
| Primary Tr1 act | 76.8 | Microsvasular Dermal EC TNFalpha + IL-1beta | 2.7 |
| Primary Th1 rest | 90.1 | Bronchial epithelium TNFalpha+ IL1 beta | 8.8 |
| Primary Th2 rest | 87.7 | Small airway epithelium none | 2.7 |
| Primary Tr1 rest | 99.3 | Small airway epithelium TNFalpha + IL-1beta | 37.9 |
| CD45RA CD4 lymphocyte act | 5.3 | Coronery artery SMC rest | 2.2 |
| CD45RO CD4 lymphocyte act | 35.8 | Coronery artery SMC TNFalpha + IL-1 beta | 4.5 |
| CD8 lymphocyte act | 6.5 | Astrocytes rest | 9.8 |
| Secondary CD8 lymphocyte rest | 38.2 | Astrocytes TNFalpha + IL-1 beta | 2.2 |
| Secondary CD8 lymphocyte act | 14.7 | KU-812 (Basophil) rest | 0.0 |
| CD4 lymphocyte none | 18.9 | KU-8 12 (Basophil) PMA/ionomycin | 1.7 |
| 2ry Th1/Th2/Tr1_anti-CD95 CH11 | 100.0 | CCD1106 (Keratinocytes) none | 34.6 |
| LAK cells rest | 64.6 | CCD1106 (Keratinocytes) TNFalpha + IL-1 beta | 32.3 |
| LAK cells IL-2 | 2.3 | Liver cirrhosis | 11.4 |
| LAK cells IL-2+IL-12 | 19.3 | Lupus kidney | 10.7 |
| LAK cells IL-2+IFN gamma | 60.7 | NCl-H292 none | 25.0 |
| LAK cells IL-2+IL-18 | 34.4 | NCl-H292 IL-4 | 20.7 |
| LAK cells PMA/ionomycin | 37.6 | NCI-H292 IL-9 | 25.5 |
| NK Cells IL-2 rest | 6.1 | NCI-H292 IL-13 | 7.6 |
| Two Way MLR 3 day | 8.9 | NCI-H292 IFN gamma | 7.0 |
| Two Way MLR 5 day | 2.0 | HPAEC none | 0.0 |
| Two Way NER 7 day | 1.4 | HPAEC TNF alpha+IL-1beta | 0.0 |
| PBMC rest | 4.7 | Lung fibroblast none | 3.4 |
| PBMCPWM | 48.6 | Lung fibroblast TNF alpha + IL-1 | 5.1 |
| PBMC PHA-L | 33.4 | Lung fibroblast IL-4 | 12.6 |
| Ramos (B cell) none | 0.0 | Lung fibroblast IL-9 | 5.8 |
| Ramos (B cell) ionomycin Ramos (B cell) ionomycin | 0.0 | Lung fibroblast IL-13 Lung fibroblast IL-13 | 4.8 |
| B lymphocytes PWM | 68.8 | Lung fibroblast IFN gamma | 2.3 |
| B lymphocytes CD40L and IL-4 | 33.2 | Dermal fibroblast CCD1070 rest | 20.7 |
| EOL-1 dbcAMP | 0.0 | Dermal fibroblast CCD1070 TNF alpha | 64.6 |
| EOL-1 dbcAMP PMA/ionomycin | 0.0 | Dermal fibroblast CCD1070 IL-1 beta | 2.2 |
| Dendritic cells none | 9.8 | Dermal fibroblast IFN gamma | 6.1 |
| Dendritic cells LPS | 6.3 | Dermal fibroblast IL-4 | 8.2 |
| Dendritic cells anti-CD40 | 14.5 | IBD Colitis 2 | 0.0 |
| Monocytes rest | 41.2 | IBD Crohn's | 4.5 |
| Monocytes LPS | 0.0 | Colon | 16.7 |
| Macrophages rest | 4.6 | Lung | 8.5 |
| Macrophages LPS | 4.8 | Thymus | 93.3 |
| HUVEC none | 0.0 | Kidney | 17.1 |
| HWEC starved | 12.4 | | |

**CNS_neurodegeneration_v1.0 Summary:** Ag3316 This panel confirms the expression of this gene at low levels in the brain in an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel_v1.4 Summary:** Ag3316 Expression of the CG57724-01 gene is highest in lung cancer cell line NCI-H23 (CT = 29.4). This gene is also expressed at moderate levels in all regions of the central nervous system examined, including in amygdala, cerebellum, hippocampus, cerebral cortex, substantia nigra, thalamus, and spinal cord. Therefore, this gene may play a role in central nervous system disorders such as Alzheimer's disease, Parkinson's disease, epilepsy, multiple sclerosis, schizophrenia and depression.

Among tissues with metabolic or endocrine function, this gene is expressed at moderate levels in adrenal gland and pituitary gland and at low levels in pancreas, thyroid, liver, and adipose. Therefore, therapeutic modulation of the activity of this gene may prove useful in the treatment of endocrine/metabolically related diseases, such as obesity and diabetes.

The CG57724-01 gene encodes a protein with homology to carboxylesterases. Carboxylesterases (CESs) catalyze the hydrolysis of a variety of compounds containing ester and amide bonds. They may also play an important role in lipid and drug metabolism by hydrolyzing endogenous long-chain fatty acid esters.

**Panel 4D Summary:** Ag3316 Moderate to low expression of this gene is found in a wide range of cells included in this panel, with a highest expression in activated T cells and in particular T regulatory cells (Tr1). This gene encodes for a carboxylesterase like protein, a protein that plays an important role in the metabolism of endogenous lipids. Therefore regulation of carboxylesterase gene expression may have physiological significance essential in T cell mediated diseases such as autoimmune diseases and allergies as well as in B cell disorders.

### NOV25

Expression of gene NOV25/CG57503-01 was assessed using the primer-probe set Ag3258, described in Table UA. Results of the RTQ-PCR runs are shown in Tables UB, UC and UD.

**Table UA. Probe Name Ag3258**

| **Primers** | **Sequences** | **Length** | **Start Position** | **SEQ ID NO** |
|---|---|---|---|---|
| Forward | 5'-gagtcaccgtaaggctgtea-3' | 20 | 1728 | 325 |
| Probe | TET-5'-catcccttcgccatcacagtgtttg-3'-TAMRA | 25 | 1765 | 326 |
| Reverse | 5'-tctgtccagtacaggctgtctt-3' | 22 | 1790 | 327 |

**Table UB. CNS_neurodegeneration_v1.0**

| **Tissue Name** | **Rel. Exp.(%) Ag3258, Run 209990877** | **Tissue Name** | **Rel. Exp.(%) Ag3258, Run 209990877** |
|---|---|---|---|
| AD 1 Hippo | 56.6 | Control (Path) 3 Temporal | 42.3 |
| AD 2 Hippo | 44.8 | Control (Path) 4 Temporal Ctx | 49.3 |
| AD 3 Hippo | 42.6 | AD 1 Occipital Ctx | 56.6 |
| AD 4 Hippo | 23.3 | AD 2 Occipital Ctx | 0.0 |
| AD 5 Hippo | 96.6 | AD 3 Occipital Ctx | 68.8 |
| AD 6 Hippo | 77.4 | AD 4 Occipital Ctx | 50.3 |
| Control 2 Hippo | 39.0 | AD 5 Occipital Ctx | 43.8 |
| Control 4 Hippo | 39.5 | AD 6 Occipital Ctx | 38.4 |
| Control (Path) 3 Hippo | 30.1 | Control 1 Occipital Ctx | 27.4 |
| AD 1 Temporal Ctx | 90.8 | Control 2 Occipital Ctx | 34.2 |
| AD 2 Temporal Ctx | 55.5 | Control 3 Occipital Ctx | 39.0 |
| AD 3 Temporal Ctx | 46.0 | Control 4 Occipital Ctx | 27.7 |
| AD 4 Temporal Ctx | 47.3 | Control (Path) 1 Occipital Ctx | 53.6 |
| AD 5 Inf Temporal Ctx | 100.0 | Control (Path) 2 Occipital Ctx | 23.0 |
| AD 5 Sup Temporal Ctx | 88.9 | Control (Path) 3 Occipital Ctx | 24.3 |
| AD 6 Inf Temporal Ctx | 79.6 | Control (Path) 4 Occipital Ctx | 19.1 |
| AD.6 Sup Temporal Ctx | 57.0 | Control 1 Parietal Ctx | 33.21 |
| Control 1 Temporal Ctx | 36.1 | Control 2 Parietal Ctx | 85.9 |
| Control 2 Temporal Ctx | 35.8 | Control 3 Parietal Ctx | 25.9 |
| Control 3 Temporal Ctx | 42.6 | Control (Path) 1 Parietal Ctx | 68.8 |
| Control 3 Temporal Ctx | 26.8 | Control (Path) 2 Parietal Ctx | 47.6 |
| Control (Path) 1 Temporal Ctx | 53.2 | Control (Path) 3 Parietal Ctx | 26.2 |
| Control (Path) 2 Temporal Ctx | 39.8 | Control (Path) 4 Parietal Ctx | 39.8 |

**Table UC. General_screening_panel-v1.4**

| **Tissue Name** | **Rel. Exp.(%) Ag3258, Run 214694854** | **Tissue Name** | **Rel. Exp.(%) Ag3258, Run 214694854** |
|---|---|---|---|
| Adipose | 4.6 | Renal ca. TK-10 | 13.1 |
| Melanoma* Hs688(A).T | 13.3 | Bladder | 3.1 |
| Melanoma* Hs688(B).T | 7.3 | Gastric ca. (liver met.) NCI-N87 | 1.5 |
| Melanoma* M14 | 22.4 | Gastric ca. KATO III | 0.0 |
| Melanoma* LOXIMVI | 26.1 | Colon ca. SW-948 | 6.7 |
| Melanoma* SK-MEL-5 | 50.7 | Colon ca. SW480 | 7.7 |
| Squamous cell carcinoma SCC-4 | 11.8 | Colon ca.* (SW480 met) SW620 | 17.8 |
| Testis Pool | 3.8 | Colon ca. HT29 | 0.0 |
| Prostate ca.* (bone met) PC-3 | 3.6 | Colon ca. HCT-116 | 12.9 |
| Prostate Pool | 7.3 | Colon ca. CaCo-2 | 19.8 |
| Placenta | 7.7 | Colon cancer tissue | 4.4 |
| Uterus Pool | 3.1 | Colon ca. SW1116 | 0.9 |
| Ovarian ca. OVCAR-3 | 4.5 | Colon ca. Colo-205 | 56.3 |
| Ovarian ca. SK-OV-3 | 10.7 | Colon ca. SW-48, | 100.0 |
| Ovarian ca. OVCAR-4 | 0.5 | Colon Pool | 5.4 |
| Ovarian ca. OVCAR-5 | 7.2 | Small Intestine Pool | 14.4 |
| Ovarian ca. IGROV-1 | 12.5 | Stomach Pool | 6.0 |
| Ovarian ca. OVCAR-8 | 6.5 | Bone Marrow Pool | 4.1 |
| Ovary | 18.8 | Fetal Heart | 12.7 |
| Breast ca. MCF-7 | 0.2 | Heart Pool | 14.3 |
| Breast ca.MDA-MB-231 | 5.6 | Lymph Node Pool | 7.4 |
| Breast ca. BT 549 | 1.0 | Fetal Skeletal Muscle | 14.8 |
| Breastea.T47D | 9.0 | Skeletal Muscle Pool | 43.5 |
| Breast ca. MDA-N | 0.6 | Spleen Pool | 16.7 |
| Breast Pool | 5.6 | Thymus Pool | 8.5 |
| Trachea | 12.4 | CNS cancer (glio/astro) U87-MG | 7.5 |
| Lung | 2.1 | CNS cancer (glio/astro) U-118-MG | 58.6 |
| Fetal Lung | 83.5 | CNS cancer (neuro;met) SK-N-AS | 23.7 |
| Lung ca. NCI-N417 | 3.4 | CNS cancer (astro) SF-539 | 1.9 |
| Lung ca. LX-1 | 2.6 | CNS cancer (astro) SNB-75 | 1.5 |
| Lung ca. NCI-H146 | 20.9 | CNS cancer (glio) SNB-19 | 12.2 |
| Lucig ca. SHP-77 | 20.9 | CNS cancer (glio) SF-295 | 4.8 |
| Lung ca. A549 | 37.9 | Brain (Amygdala) Pool | 28.9 |
| Lung ca. NCI-H526 | 10.2 | Brain (cerebellum) | 59.9 |
| Lung ca. NCI-H23 | 16.2 | Brain (fetal) | 34.2 |
| Lung ca. NCI-H460 | 5.2 | Brain (Hippocampus) Pool | 40.6 |
| Lung ca. HOP-62 | 3.1 | Cerebral Cortex Pool | 62.9 |
| Lung ca. NCI-H522 | 38.2 | Brain (Substantia nigra)Pool | 39.0 |
| Liver | 0.9 | Brain (Thalamus) Pool | 56.6 |
| Fetal Liver | 2.2 | Brain (whole) | 58.2 |
| Liver ca. HepG2 | 14.0 | Spinal Cord Pool | 58.2 |
| Kidney Pool | 15.9 | Adrenal Gland | 12.1 |
| Fetal Kidney | 21.6 | Pituitary gland Pool | 6.7 |
| Renal ca. 786-0 | 6.6 | Salivary Gland | 4.8 |
| Renal ca. A498 | 3.7 | Thyroid (female) | 13.1 |
| Renal ca. ACHN | 19.2 | Pancreatic ca. CAPAN2 | 0.1 |
| Renal ca. UO-31 | 8.2 | Pancreas Pool | 10.5 |

**Table UD. Panel 4D**

| **Tissue Name** | **Rel. Exp.(%) Ag3258, Run 164537289** | **Tissue Name** | **Rel. Exp.(%) Ag3258, Run 164537289** |
|---|---|---|---|
| Secondary Th1 act | 0.3 | HUVEC IL-1beta | 3.2 |
| Secondary Th2 act | 3.8 | HUVEC IFN gamma | 6.5 |
| Secondary Tr1 act | 0.3 | HUVEC TNF alpha + IFN gamma | 4.2 |
| Secondary Th1 rest | 0.2 | HUVEC TNF alpha + IL4 | 5.2 |
| Secondary Th2 rest | 0.0 | HUVEC IL-11 | 3.4 |
| Secondary Tr1 rest | 0.0 | Lung Microvascular EC none | 4.4 |
| Primary Th1 act | 2.4 | Lung Microvascular EC TNFalpha + IL-1beta | 1.8 |
| Primary Th2 act | 0.0 | Microvascular Dermal EC none | 8.5 |
| Primary Tr1 act | 0.0 | Microsvasular Dermal EC TNFalpha+IL-1beta | 4.9 |
| Primary Th1 rest | 0.0 | Bronchial epithelium TNFalpha + | 14.9 |
| IL1beta Primary Th2 rest | 0.2 | Small airway epithelium none | 2.1 |
| Primary Tr1 rest | 0.0 | Small airway epithelium TNFalpha + IL-1beta | 19.3 |
| CD45RA CD4 lymphocyte act | 3.0 | Coronery artery SMC rest | 133 |
| CD45RO CD4 lymphocyte act | 0.0 | Coronery artery SMC TNFalpha + IL-1beta | 9.4 |
| CD8 lymphocyte act | 0.0 | Astrocytes rest | 21.9 |
| Secondary CD8 lymphocyte rest | 0.0 | Astrocytes TNFalpha+ IL-1beta | 3.5 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.3 |

However, no differential expression of this gene was detected between Alzheimer's diseased postmortem brains and those of non-demented controls in this experiment. Please see Panel 1.3D for a discussion of the potential utility of this gene in treatment of central nervous system disorders.

Panel 1.3D Summary: Ag3214 Expression of the CG57358-01 gene is highest in colon cancer cell line SW620 and an ovarian cancer cell line (CTs = 26.1). It is expressed in a number of cancer cell lines including ovarian, breast, lung, renal, liver adenocarcinoma, and astrocytoma. Therefore, this gene may play a role in these types of cancer.

This gene is expressed at moderate levels throughout the CNS, including in amygdala, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression. The CG57358-01 gene encodes a protein with similarity to the Drosophila spinster gene. Expression of the CG57358-01 gene in brain is consistent with observations made on the Drosophila spinster gene. Mutations of the spinster gene in Drosophila cause degeneration of adults neurons and reductions in programmed neuronal cell death (ref. 1). Thus, modulation of expression of the CG57358-01 gene may be important in the treatment of neurodegenerative diseases and aging.

Among tissues with metabolic or endocrine function, this gene is expressed at moderate levels in adipose, pancreas, liver, skeletal muscle, and heart and at low levels in thyroid, adrenal gland and pituitary gland. Therefore, therapeutic modulation of the activity of this gene may prove useful in the treatment of endocrine/metabolically related diseases, such as obesity and diabetes.

### References:

### 1. Nakano Y, Fujitani K, Kurihara J, Ragan J, Usui-Aoki K, Shimoda L, Lukacsovich T, Suzuki K, Sezaki M, Sano Y, Ueda R, Awano W, Kaneda M, Umeda M, Yamamoto D. Mutations in the novel membrane protein spinster interfere with programmed cell death and cause neural degeneration in Drosophila melanogaster. Mol Cell Biol 2001 Jun;21 (11):3775-88.

Mutations in the spin gene are characterized by an extraordinarily strong rejection behavior of female flies in response to male courtship. They are also accompanied by decreases in the viability, adult life span, and oviposition rate of the flies. In spin mutants, some oocytes and adult neural cells undergo degeneration, which is preceded by reductions in programmed cell death of nurse cells in ovaries and of neurons in the pupal nervous system, respectively. The central nervous system (CNS) of spin mutant flies accumulates autofluorescent lipopigments with characteristics similar to those of lipofuscin. The spin locus generates at least five different transcripts, with only two of these being able to rescue the spin behavioral phenotype; each encodes a protein with multiple membrane-spanning domains that are expressed in both the surface glial cells in the CNS and the follicle cells in the ovaries. Orthologs of the spin gene have also been identified in a number of species from nematodes to humans. Analysis of the spin mutant will give us new insights into neurodegenerative diseases and aging.

### PMID: 11340170

Panel 2.2 Summary: Ag3214 Expression of the CG57358-01 gene is highest in a kidney cancer sample (CT = 28.4). However, in general expression of this gene appears to be higher in matched normal kidney and lung tissues when compared to the adjacent tumors. Therefore, therapeutic modulation of the activity of this gene or its protein product, using small molecule drugs, antibodies or protein therapeutics, may be of use in the treatment of kidney and lung cancer.

Panel 4D Summary: Ag3214 Expression of the CG57358-01gene is highest in thymus (CT = 27). In addition, low levels of expression of this gene are detected in resting T cells. Therefore, small molecule therapeutics or antibody therapeutics designed against the protein encoded for by this gene could be utilized to modulate immune function (T cell development) and be important for organ transplant, AIDS treatment or post chemotherapy immune reconstitiution.

Expression of this gene is also detected at moderate levels in a number of endothelial cell samples on this panel including lung microvascular endothelial cells, microvascular dermal endothelial cells, HPAEC and HUVECs, suggesting a role for this gene in endothelium integrity or homeostasis. Thus, this gene may play a role in inflammation. Therefore, therapeutic modulation of the activity of this gene, using small molecule drugs, antibodies or protein therapeutics, may be of use in the treatment or prevention of inflammatory reactions.

### NOV20

Expression of NOV20/CG57695-01 was assessed using the primer-probe set Ag3310, described in Table RA. Results of the RTQ-PCR run are shown in Tables RB.

**Table RA. Probe Name Ag3310**

| **Primers** | **Sequences** | **Length** | **Start Position** | **SEQ ID NO** |
|---|---|---|---|---|
| Forward | 5'-gaacttcctcccatttcttcag-3' | 22 | 330 | 319 |
| Probe | TET-5'-aaacccaggagatcagcataagacaa-3'-TAMRA | 26 | 378 | 320 |
| Reverse | 5'-tagtgggaacaaaggggtagac-3' | 22 | 406 | 321 |

**Table RB. General_screening_panel_v1.4**

| **Tissue Name** | **Rel. Exp.(%) Ag3310, Run 215642635** | **Tissue Name** | **Rel. Exp.(%) Ag3310, Run 215642635** |
|---|---|---|---|
| Adipose | 0.0 | Renal ca. TK-10 | 0.0 |
| Melanoma* Hs688(A).T | 0.0 | Bladder | 0.0 |
| Melanoma* Hs688(B).T | 0.0 | Gastric ca. (liver met.) NCI-N87 | 0.0 |
| Melanoma* M14 | 0.0 | Gastric ca. KATO III | 7.5 |
| Melanoma* LOXIMVI | 0.0 | Colon ca. SW-948 | 0.0 |
| Melanoma*SK-MBL-5 | 0.0 | Colon ca. SW480 | 0.0 |
| Squamous cell carcinoma SCC-4 | 0.0 | Colon ca.* (SW480 met) SW620 | 0.0 |
| Testis Pool | 100.0 | Colon ca. HT29 | 0.0 |
| Prostate ca.* (bone met) PC-3 | 0.0 | Colon ca. HCT-116 | 0.0 |
| Prostate Pool | 0.0 | Colon ca. CaCo-2 | 0.0 |
| Placenta | 0.0 | Colon cancer tissue | 0.0 |
| Uterus Pool | 0.0 | Colon ca.SW1116 | 5.4 |
| Ovarian ca.OVCAR-3 | 0.0 | Colon ca. Colo-205 | 0.0 |
| Ovarian ca. SK-OV-3 | 4.5 | Colon ca. SW-48 | 0.0 |
| Ovarian ca. OVCAR-4 | 0.0 | Colon Pool | 11.9 |
| Ovarian ca. OVCAR-5 | 0.0 | Small Intestine Pool | 0.0 |
| Ovarian ca. IGROV-1 | 0.0 | Stomach Pool | 0.0 |
| Ovarian ca. OVCAR-8 | 0.0 | Bone Marrow Pool | 0.0 |
| Ovary | 0.0 | Fetal Heart | 0.0 |
| Breast ca. MCF-7 | 0.0 | Heart Pool | 0.0 |
| Breast ca.MDA-MB-231 | 0.0 | Lymph Node Pool | 0.0 |
| Breast ca. BT 549 | 10.5 | Fetal Skeletal Muscle | 0.0 |
| Breast ca. T47D | 0.0 | Skeletal Muscle Pool | 0.0 |
| Breast ca. MDA-N | 0.0 | Spleen Pool | 1.6 |
| Breast Pool | 0.0 | Thymus Pool | 0.0 |
| Trachea | 0.0 | CNS cancer (glio/astro) U87- | 0.0 |
| Lung | 17.7 | CNS cancer (glio/astro) U-118-MG | 0.0 |
| Fetal Lung | 25.2 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 0.0 | CNS cancer (glio) SNB-19 | 0.0 |
| Lung ca. SHP-77 | 0.0 | CNS cancer (glio) SF-295 | 0.0 |
| Lung ca. A549 | 0.0 | Brain (Amygdala) Pool | 0.0 |
| Lung ca. NCI-H526 | 0.0 | Brain (cerebellum) | 0.0 |
| Lung ca. NCI-H23 | 1.6 | Brain (fetal) | 0.0 |
| Lung ca. NCI-H460 | 0.0 | Brain (Hippocampus) Pool | 0.0 |
| Lung ca. HOP-62 | 0.0 | Cerebral Cortex Pool | 0.0 |
| Lung ca. NCI-H522 | 0.0 | Brain (Substantia nigra) Pool | 0.0 |
| Liver | 0.0 | Brain (Thalamus) Pool | 0.0 |
| Fetal Liver | 0.0 | Brain (whole) | 0.0 |
| Liver ca. HepG2 | 0.0 | Spinal Cord Pool | 0.0 |
| Kidney Pool | 0.0 | Adrenal Gland | 0.0 |
| Fetal Kidney | 7.5 | Pituitary gland Pool | 0.0 |
| Renal ca 786-0 | 0.0 | Salivery Gland | 0.0 |
| Renal ca A498 | 0.0 | Thyroid (female) | 0.0 |
| Renal ca. ACHN | 0.0 | Pancreatic ca. CAPAN2 | 0.0 |
| Renal ca. UO-31 | 0.0 | Pancreas Pool | 0.0 |

**CNS_neurodegeneration_v1.0 Summary:** Ag3310 Expression of the CG57695-01 gene is low/undetectable (CTs > 35) across all of the samples on this panel (data not shown).

**General_screening_panel_v1.4 Summary:** Ag3310 Expression of the CG57695-01. gene is seen only in the testis (CT = 33.8) Therefore, expression of this gene can be used to distinguish testis from the other samples on this panel. In addition, therapeutic modulation of the activity of this gene may be of use in the treatment of reproductive disorders such as infertility.

**Panel 4D Summary:** Ag3310 Expression of the CG57695-01 gene is low/undetectable (CTs > 35) across all of the samples on this panel (data not shown).

### NOV21

Expression of NOV21/CG576S4-O1 was assessed using the primer-probe set Agl680, described in Table SA. Results of the RTQ-PCR runs are shown in Tables SB, SC, SD and SE.

**Table SA. Probe Name Ag1680**

| **Primers** | **Sequences** | **Length** | **Start Position** |
|---|---|---|---|
| Forward | 5'-gccaattacaattgcacaattt-3' | 22 | 795 |
| Probe | TET-5'-atgaacactcctgceccttggagtt-3'-TAMRA | 25 | 825 |
| Reverse | 5'-tcttcacgtggatagccataac-3' | 22 | 855 |

**Table SB. CNS_neurodegeneration_v1.0**

| **Tissue Name** | R**el. Exp.(%) Ag1680, Run 207624859** | **Tissue Name** | **Rel. Exp.(%) Ag1680, Run 207624859** |
|---|---|---|---|
| AD 1 Hippo | 5.0 | Control (Path) 3 Temporal Ctx | 0.8 |
| AD 2 Hippo | 9.0 | Control (Path) 4 Temporal | 20.4 |
| AD 3 Hippo | 2.9 | AD Occipital Ctx | 12.9 |
| AD 4 Hippo | 3.7 | AD 2 Occipital Ctx (Missing) | 0.0 |
| AD 5 Hippo | 85.3 | AD 3 Occipital Ctx | 1.3 |
| AD 6 Hippo | 38.2 | AD 4 Occipital Ctx | 19.3 |
| Control 2 Hippo | 27.0 | AD 5 Occipital Ctx | 65.5 |
| Control 4 Hippo | 2.4 | AD 6 Occipital Ctx | 43.2 |
| Control (Path) 3 Hippo | 0.6 | Control 1 Occipital Ctx | 0.3 |
| AD 1 Temporal Ctx | 4.8 | Control 2 Occipital Ctx | 100.0 |
| AD 2 Temporal Ctx | 12.6 | Control 3 Occipital Ctx | 16.6 |
| AD 3 Temporal Ctx | 2.5 | Control 4 Occipital Ctx | 2.1 |
| AD 4 Temporal Ctx | 14.0 | Control (Path) 1 Occipital | 69.7 |
| AD 5 Inf Temporal Ctx | 68.3 | Control (Path) 2 Occipital Ctx | 12.8 |
| AD 5 Sup Temporal Ctx | 17.3 | Control (Path) 3 Occipital Ctx | 0.2 |
| AD 6 Inf Temporal Ctx | 47.6 | Control (Path) 4 Occipital Ctx | 10.2 |
| AD 6 Sup Temporal Ctx | 61.1 | Control 1 Parietal Ctx, | 1.3 |
| Control 1 Temporal Ctx | 0.9 | Control 2 Parietal Ctx | 18.2 |
| Control 2 Temporal Ctx | 52.5 | Control 3 Parietal Ctx | 21.0 |
| Control 3 Temporal Ctx | 15.9 | Control (Path) 1 Parietal Ctx | 59.5 |
| Control 3 Temporal Ctx | 4.8 | Control (Path) 2 Parietal Ctx | 23.2 |
| Control (Path) 1 Temporal Ctx | 42.0 | Control (Path) 3 Parietal Ctx | 0.6 |
| Control (Path) 2 Temporal Ctx | 40.6 | Control (Path) 4 Parietal Ctx | 30.6 |

**Table SC. Panel 1.3D**

| **Tissue Name** | **Rel. Exp.(%) Ag1680, Run 158317296** | **Tissue Name** | **Rel. Exp.(%) Ag1680, Run 158317296** |
|---|---|---|---|
| Liver adenocarcinoma | 0.3 | Kidney (fetal) | 0.0 |
| Pancreas | 0.0 | Renal ca. 786-0 | 0.0 |
| Pancreatic ca. CAPAN 2 | 0.0 | Renal ca. A498 | 0.0 |
| Adrenal gland | 0.5 | Renal ca. RXF 393 | 0.0 |
| Thyroid | 0.0 | Renal ca. ACHN | 0.0 |
| Salivary gland | 0.0 | Renal ca. UO-31 | 0.0 |
| Pituitary gland | 8.1 | Renal ca. TK-10 | 0.0 |
| Brain (fetal) | 3.7 | Liver | 0.0 |
| Brain (whole) | 27.0 | Liver (fetal) | 0.0 |
| Brain (amygdala) | 25.0 | Liver ca. (hepatoblast) HePG2 | 0.0 |
| Brain (cerebellum) | 17.4 | Lung | 0.0 |
| Brain (hippocampus) | 100.0 | Lung (fetal) | 0.0 |
| Brain (substantia nigra) | 2.4 | Lung ca. (small cell) LX-1 | 0.6 |
| Brain (thalamus) | 14.6 | Lung ca. (small cell) NCI-H69 | 0.0 |
| Cerebral Cortex | 83.5 | Lung ca.(s.cell var.)SHP- | 4.2 |
| Spinal cord | 0.5 | Lung ca. (large cell)NCI-H460 | 0.0 |
| glio/astro U87-MG | 0.0 | Lung ca. (non-sm. cell) A549 | 0.0 |
| glio/astro U-118-MG | 0.0 | Lung ca. (non-s.cell) NCI-H23 | 0.0 |
| astrocytoma SW1783 | 0.0 | Lung ca. (non-s.cell) HOP-62 | 0.0 |
| neuro*; met SK-N-AS | 4.0 | Lung ca. (non-s.cl) NCI-H522 | 0.0 |
| astrocytomaSF-539 | 0.0 | Lung ca.(squam.) SW 900 | 0.0 |
| astrocytoma SNB-75 | 0.0 | Lung ca. (squam.) NCI-H596 | 0.0 |
| glioma SNB-19 | 0.0 | Mammary gland | 0.0 |
| glioma U251 | 0.0 | Breast ca.*(pl.ef)MCP-7 | 0.0 |
| glioma SF-295 | 0.0 | Breast ca.* (pl. ef) MDA-MB-231 | 0.0 |
| Heart (fetal) | 0.0 | Breast ca.* (pl.cf) T47D | 0.5 |
| Heart | 0.0 | Breast ca. BT-549 | 0.0 |
| Skeletal muscle (fetal) | 0.0 | Breast ca. MDA-N | 1.3 |
| Skeletal muscle | 0.0 | Ovary | 0.0 |
| Bone marrow | 0.0 | Ovarian ca. OVCAR-3 | 0.3 |
| Thymus | 0.0 | Ovarian ca. OVCAR-4 | 0.0 |
| Spleen | 0.0 | Ovarian ca. OVCAR-5 | 0.0 |
| Lymph node | 0.0 | Ovarian ca. OVCAR-8 | 0.0 |
| Colorectal | 0.0 | Ovarian ca. IGROV-1 | 0.0 |
| Stomach | 0.0 | Ovarian ca.* (ascites) SK-OV-3 | 0.0 |
| Small intestine | 0.2 | Uterus | 0.0 |
| Colon ca. SW480 | 0.0 | Placenta | 0.1 |
| Colon ca.* SW620(SW480 met) | 0.9 | Prostate | 0.0 |
| Colon ca. HT29 | 0.0 | Prostate ca.* (bone met)PC-3 | 0.0 |
| Colon ca. HCT-116 | 0.7 | Testis | 0.1 |
| Colon ca. CaCo-2 | 0.0 | Melanoma Hs688(A).T | 0.0 |
| Colon ca.tissue(OD03866) | 0.0 | Melanoma* (met) Hs688(B).T | 0.0 |
| Colon ca. HCC-2998 | 0.0 | Melanoma UACC-62 | 0.2 |
| Gastric ca.* (liver met) NCl-N87 | 0.0 | Melanoma M14 | 0.0 |
| Bladder | 0.0 | Melanoma LOX IMVI | 0.0 |
| Trachea | 0.0 | Melanoma* (met) SK-MEL-5 | 0.0 |
| Kidney | 0.0 | Adipose | 0.0 |

**Table SD. Panel 2D**

| **Tissue Name** | **Rel. Exp.(%) Ag1680, Run 158318977** | **Tissue Name** | **Rel. Exp.(%) Ag1680, Run 158318977** |
|---|---|---|---|
| Normal Colon | 100.0 | Kidney Margin 8120608 | 0.0 |
| CC Well to Mod Diff (ODO3866) | 0.0 | Kidney Cancer 8120613 | 0.0 |
| CC Margin (ODO3866) | 8.7 | Kidney Margin 8120614 | 0.0 |
| CC Gr.2 rectosigmoid (ODO3868) | 0.0 | Kidney Cancer 9010320 | 85.3 |
| CC Margin (ODO3868) | 12.3 | Kidney Margin 9010321 | 0.0 |
| CC Mod Diff (ODO3920) | 7.5 | Normal Uterus | 0.0 |
| CC Margin (ODO3920) | 35.4 | Uterus Cancer 064011 | 0.0 |
| CC Gr.2 ascend colon (ODO3921) | 7.6 | Normal Thyroid | 0.0 |
| CC Margin (OD03921) | 9.5 | Thyroid Cancer 064010 | 0.0 |
| CC from Partial Hepatectomy (OD04309) Mets | 0.0 | Thyroid Cancer A302152 | 0.0 |
| Liver Margin (ODO4309) | 0.0 | Thyroid Margin A302153 | 0.0 |
| Colon mets to lung (OD04451-01) | 0.0 | Normal Breast | 0.0 |
| Lung Margin (OP04451-02) | 0.0 | Breast Cancer (OD04566) | 0.0 |
| Normal Prostate 6S46-1 | 0.0 | Breast Cancer (OD04590-01) | 0.0 |
| Prostate Cancer (OD04410) | 0.0 | Breast Cancer Mets (OD04590-03) | 0.0 |
| Prostate Margin (OD04410) (OD04655-05) | 0.0 | Breast Cancer Metastasis | 7.7 |
| Prostate Cancer (OD04720-01) | 7.4 | Breast Cancer 064006 | 0.0 |
| Prostate Margin (OD04720-02) | 12.6 | Breast Cancer 1024 | 0.0 |
| Normal Lung 061010 | 7.9 | Breast Cancer 9100266 | 0.0 |
| Lung Met to Muscle (ODO4286) | 0.0 | Breast Margin 9100265 | 0.0 |
| Muscle Margin (ODO4286) | 0.0 | Breast Cancer A209073 | 0.0 |
| Lung Malignant Cancer (OD03126) | 0.0 | Breast Margin A209073 | 0.0 |
| Lung Margin (OD03126) | 0.0 | Normal Liver | 0.0 |
| Lung Cancer (0D04404) | 0.0 | Liver Cancer 064003 | 0.0 |
| Lung Margin (OD04404) | 0.0 | Liver Cancer 1025 | 0.0 |
| Lung Cancer (OD04565) | 26.8 | Liver Cancer 1026 | 0.0 |
| Lung Margin (OD04S6S) | 0.0 | Liver Cancer 6004-T | 0.0 |
| Lung Cancer (OD04237-01) | 0.0 | Liver Tissue 6004-N | 0.0 |
| Lung Margin (OD04237-02) | 0.0 | Liver Cancer 6005-T | 0.0 |
| Ocular Mel Met to Liver (OD04310) | 0.0 | Liver Tissue 6005-N | 0.0 |
| Liver Margin (ODO4310) | 0.0 | Normal Bladder | 16.0 |
| Melanoma Mets to Lung (OD04321) | 0.0 | Bladder Cancer 1023 | 0.0 |
| Lung Margin (OD04321) | 0.0 | Bladder Cancer A302173 | 57.4 |
| Normal Kidney | 0.0 | Bladder Cancer (OD04718-01) | 0.0 |
| Kidney Ca, Nuclear grade 2 (OD04338) | 0.0 | Bladder Normal Adjacent (OD04718-3) | 0.0 |
| Kidney Margin (OD04338) | 0.0 | Normal Ovary | 0.0 |
| Kidney Ca Nuclear grade 1/2 (OD04339) | 0.0 | Ovarian Cancer 064008 | 43.2 |
| Kidney Margin (OD04339) | 0.0 | Ovarian Cancer (OD04768-07) | 0.0 |
| Kidney Ca, Clear cell type (OD04340) | 0.0 | Ovary Margin (OD04768-08) | 0.0 |
| Kidney Margin(OB04340) | 0.0 | Normal Stomach | 7.4 |
| Kidney Ca, Nuclear grade 3 (OD04348) | 0.0 | Gastric Cancer 9060358 | 0.0 |
| Kidney Margin (OD04348) | 0.0 | Stomach Margin 9060359 | 3.3 |
| Kidney Cancer (OD04622-01) | 0.0 | Gastric Cancer 9060395 | 8.1 |
| Kidney Margin (OD04622-03) | 0.0 | Stomach Margin 9060394 | 7.4 |
| Kidney Cancer (OD04450-01) | 0.0 | Gastric Cancer 9060397 | 1.3 |
| Kidney Margin (OD04450-03) | 0.0 | Stomach Margin 9060396 | 6.5 |
| Kidney Cancer 8120607 | 0.0 | Gastric Cancer 064005 | 0.0 |

**Table SE. Panel 4D**

| **Tissue Name** | **Rel. Exp.(%) Ag1680, Run 158320708** | **Tissue Name** | **Rel. Exp.(%) Ag1680, Run 158320708** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha+IFN gamma | 0.0 |
| Secondary Th1 rest | 0.0 | HWEC TNF alpha+IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha + IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1 beta | 0.0 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha +IL-1beta | 0.0 |
| CD45RA CD4 lymphocyte act | 0.0 | Coronery artery SMC rest | 0.0 |
| CD45RO CD4 lymphocyte act | 0.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha+IL-1beta | 0.0 |
| Secondary CD8 lymphocyte act | 0.0 | KU-812 (Basophil) rest | 19.9 |
| CD4 lymphocyte none | 0.0 | KU-812 (Basophil) PMA/ionomycin | 48.3 |
| 2ry Th1/Th2/Tr1_anti-CD95 CH11 | 0.0 | CCD1106 (Keratinocytes) none | 0.0 |
| LAK cells rest LAK cells rest | 0.0 | CCD1106 (Keratinocytes) TNFalpha+ IL-1beta | 0.0 |
| LAK cells IL-2 | 0.0 | Liver cirrhosis | 0.0 |
| LAK cells IL-2+IL-12 | 0.0 | Lupus kidney | 0.0 |
| LAK cells, IL-2+IFN gamma | 0.0 | NCI-H292 none | 94.0 |
| LAK cells IL-2+ IL-18 | 0.0 | NCI-H292 IL-4 | 31.2 |
| LAK cells PMA/ionomycin | 0.0 | NCI-H292 IL-9 | 100.0 |
| NK Cells IL-2 rest | 0.0 | NCI-H292 IL-13 | 16.4 |
| Two Way MLR 3 day | 0.0 | NCI-H292 IFN gamma | 20.2 |
| Two Way MLR 5 day | 0.0 | HPAEC none | 0.0 |
| Two Way MLR 7 day | 0.0 | HPAEC TNF alpha+IL-1 beta | 0.0 |
| PBMC rest | 0.0 | Lung fibroblast none | 0.0 |
| PHMC PWM | 0.0 | Lung fibroblast TNF alpha+ IL-1 beta | 0.0 |
| PBMCPHA-L | 0.0 | Lung fibroblast IL-4 | 0.0 |
| Ramos (B cell) none | 0.0 | Lung fibroblast IL-9 | 0.0 |
| Ramos (B, cell) ionomycin | 0.0 | Lung fibroblast IL-13 | 0.0 |
| B lymphocytes PWM | 0.0 | Lung fibroblast IFN gamma | 0.0 |
| B lymphocytes CD40L and IL-4 | 0.0 | Dermal fibroblast CCD1070 rest | 0.0 |
| EOL-1 dbcAhO EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic celts none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 7.5 |
| Monocytes rest | 0.0 | IBD Crohn's | 36.1 |
| Monocytes LPS | 0.0 | Colon | 49.3 |
| Macrophages rest | 0.0 | Lung | 0.0 |
| Macrophages LPS | 0.0 | Thymus | 0.0 |
| HUVEC none | 0.0 | Kidney | 0.0 |
| HUVEC starved | 0.0 | | |

**CNS_nenrodegeneration_v1.0 Summary:** Ag1680 This panel confirms the expression of this gene at moderate to high levels in the brain in an independent group of individuals. However, no differential expression of this gene was detected between Alzheimer's diseased postmortem brains and those of non-demented controls in this experiment. Please see Panel 1.3D for a discussion of the potential utility of this gene in treatment of central nervous system disorders.

**Panel 1.3D Summary:** Ag1680 The CG57654-01 gene is expressed at high to moderate levels in all regions of the CNS examined, including in amygdala, substantia nigra, thalamus, cerebellum, cerebral cortex, and spinal cord with the highest levels in the hippocampus (CT = 26.3). Therefore, expression of this gene may be used to distinguish brain samples from the other samples on this panel. This gene encodes a novel splice variant of the gamma-aminobutyric-acid receptor gamma-2 subunit precursor (GABA-A Receptor) gene. Several drugs, including benzodiazepines, anticonvulsants, anaesthetics and neurosteroids interact with binding sites on GABA-A receptors (ref 1). Therefore, modulation of expression of this gene may be used for treatment of central nervous system disorders such as epilepsy, anxiety and alcoholism.

Among tissues with metabolic or endocrine function, this gene is expressed at low levels in adrenal gland and pituitary gland. Therefore; therapeutic modulation of the activity of this gene may prove useful in the treatment of endocrine/metabolically related diseases, such as obesity and diabetes.

### References:

### 1. Smith TA. Type A gamma-aminobutyric acid (GABAA) receptor subunits and benzodiazepine binding: significance to clinical syndromes and their treatment. Br J Biomed Sci 2001;58(2):111-21.

Gamma (gamma)-aminobutyric acid (GABA) acting via GABAA receptors is the brain's major inhibitory neurotransmitter system and exerts a crucial role in regulating brain excitability. A number of drugs interact with binding sites on GABAA receptors, and these include benzodiazepines, anticonvulsants, anaesthetics and neurosteroids (e.g. the progesterone metabolite pregnalone). GABAA receptors comprise five subunits (19 are known currently), and are classified into three major groups (alpha, beta and gamma) and several minor ones. The subunit make-up of a receptor, particularly its alpha-subunit content, determines its pharmacological characteristics. Thus, receptors that include an alphal subunit have a benzodiazepine (BZ) type I (BZ[I]) pharmacology and bind zolpidem and CL218,872 with high affinity, whilst receptors with alpha2, alpha3 or alpha5 subunits have a BZ type II (BZ[II]) pharmacology and bind these drugs with low affinity. In contrast to receptors that contain alpha4 and alpha6 subunits, which are diazepam-insensitive, both BZ(I) and -(II) bind diazepam and other benzodiazepines. The ligand selectivity of receptor subunits assists in their characterisation. Using immunochemical and ligand-binding techniques, the subunit composition of GABAA receptors has been shown to exhibit a degree of brain regional

| | | | |
|---|---|---|---|
| 2ry Th1/Th2/Tr1_anti-CD95 CH11 | 0.0 | CCD1106(Keratinocytes)none | 24.5 |
| LAK cells rest | 1.0 | CCD1106 (Keratinocytes) TNFalpha+IL-1 beta | 6.2 |
| LAK cells IL-2 | 0.4 | Liver cirrhosis | 1.0 |
| LAK cells IL-2+IL-12 | 0.0 | Lupus kidney | 3.9 |
| LAK cells IL-2+IFN gamma | 3.1 | NCI-H292 none | 78.5 |
| LAK cells IL-2+ IL-18 | 0.7 | NCI-H292 IL-4 | 100.0 |
| LAK cells PMA/ionomycin | 0.0 | NCI-H292 IL-9 | 87.1 |
| NK Cells IL-2 rest | 0.0 | NCI-H292 IL-13 | 53.2 |
| Two Way MLR 3 day | 0.0 | NCI-H292 IFN gamma | 37.6 |
| Two WayMLR 5 day | 0.0 | HPAEC none | 1.3 |
| Two Way MLR 7 day | 0.0 | HPAEC TNF alpha + IL-1 beta | 2.9 |
| PBMC rest | 0.0 | Lung fibroblast none | 16.5 |
| PBMC PWM | 2.1 | Lung fibroblast TNF alpha + IL-1 beta | 14.0 |
| PBMC PHA-L | 0.3 | Lung fibroblast IL-4, | 21.8 |
| Ramos (B cell) none | 13.1 | Lung fibroblast IL-9 | 17.4 |
| Ramos (B cell) ionomycin | 42.3 | Lung fibroblast IL-13 | 13.6 |
| B lymphocytes PWM | 4.2 | Lung fibroblast IFN gamma | 20.6 |
| B lymphocytes CD40L and IL-4 | 0.4 | Dermal fibroblast CCD 1070 rest | 20.6 |
| EOL-1 dbcAMP | 0.0 | Dermal fibroblast CCD1070 TNF alpha | 12.9 |
| EOL-1 dbcAMP PMA/ionomycin | 0.0 | Dermal fibroblast CCD1070 IL-1 beta | 20.3 |
| Dendritic cells none | 0.0 | Dermal fibroblast IFN gamma | 13.3 |
| Dendritic cells LPS | 0.0 | Dermal fibroblast IL-4 | 33.7 |
| Dendritic cells anti-CD40 | 0.0 | IBD Colitis 2 | 1.6 |
| Monocytes rest | 0.0 | IBD Crohn's | 9.8 |
| Monocytes LPS | 0.0 | Colon | 52.9 |
| Macrophages rest | 0.0 | Lung | 17.3 |
| Macrophages LPS | 0.3 | Thymus | 13.4 |
| HUVEC none | 5.9 | Kidney | 29.3 |
| HUVEC starved | 10.3 | | |

**CNS_neurodegeneration_v1.0 Summary:** Ag3258 This panel confirms the expression of this gene at low levels in the brain in an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel-v1.4 Summary:** Ag3258 This gene is expressed at high to moderate levels in the majority of samples on this panel. In particular, high levels of CG57503-01 gene expression are seen in all regions of the central nervous system examined, including in amygdala, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

The expression of this gene in metabolic tissues such as pancreas, thyroid, pituitary gland, adrenal gland, skeletal muscle, heart, adipose, and liver suggests that modulation of the activity of this gene may prove useful in the treatment of endocrine/metabolically related diseases, such as obesity and diabetes.

In addition, there is substantial expression of this gene associated with several cancer cell lines, particularly melanoma and lung cancer cell lines. Therefore, therapeutic modulation of the activity of this gene or its protein product, through the use of small molecule drugs, protein therapeutics or antibodies, might be beneficial in the treatment of melanoma and lung cancer.

Interestingly, this gene is expressed at much higher levels in fetal lung (CT=25.6) than in adult lung (CT=31.0). This observation suggests that expression of this gene can be used to distinguish fetal from adult lung tissue.

**Panel 4D Summary:** Ag3258 Expression of the CG57503-01 gene is highest in the lung-derived mucoepidermoid cell line NCI-H292 (CT = 28.1). Thus, therapeutic modulation of the activity of this gene, using small molecule drugs, antibodies or protein therapeutics, may be of use in the treatment of asthma and emphysema. Low to moderate expression of this gene is also seen in a number of fibroblast and epithelial cell lines.

The CG57503-01 gene encodes a protein with homology to MEGF7 and is a Type I membrane protein with 7 LDL-R type A domains, 19 LDL-R type B domains, 11 EGF domains, and a cytoplasmic tail of >100 residues. This protein may act as a cell surface receptor for an uncharacterized ligand and play a role in Ca++-dependent functions, as is the case for other proteins with multiple EGF domains. Expression of the CG57503-01 gene at moderate levels in the brain, in lung and skin fibroblasts, and selectively in the pulmonary epidermoid carcinoma cell line NCI-H292, suggests that therapeutic antibodies and small molecule antagonists that block its function may be useful in reduction or elimination of the symptoms of asthma, emphysema, or psoriasis.

### NOV27

Expression of gene NOV27/CG57658-01 was assessed using the primer-probe set Ag3299, described in Table VA. Results of the RTQ-PCR runs are shown in Tables VB, VC and VD.

**Table VA. Probe Name Ag3299**

| **Primers** | **Sequences** | **Length** | **Start Position** | **SEQ ID NO** |
|---|---|---|---|---|
| Forward | 5'-cttcctctcttccaggaaagc-3' | 21 | 14 | 328 |
| Probe | TET-5'-tctggttcgtcctcacgatgctg-3'-TAMRA | 23 | 35 | 329 |
| Reverse | 5'-tcctgctcgtcctggtaga-3' | 19 | 95 | 340 |

**Table VB. CNS_neurodegeneration_v1.0**

| **Tissue Name** | **Rel. Exp.(%) Ag3299, Run 210063511** | **Tissue Name** | **Rel. Exp.(%) Ag3299, Run 210063511** |
|---|---|---|---|
| AD 1 Hippo | 0.0 | Control (Path) 3 Temporal Ctx | 6.3 |
| AD 2 Hippo | 12.0 | Control (Path) 4 Temporal Ctx | 79.6 |
| AD 3 Hippo | 0.0 | AD 1 Occipital Ctx | 21.5 |
| AD 4 Hippo | 3.3 | AD 2 Occipital Ctx (Missing) | 0.0 |
| AD 5 hippo | 78.5 | AD 3 Occipital Ctx | 4.1 |
| AD 6 Hippo | 13.9 | AD 4 Occipital Ctx | 23.3 |
| Control 2 Hippo | 22.8 | AD 5 Occipital Ctx | 8.0 |
| Control 4 Hippo | 0.0 | AD 6 Occipital Ctx | 44.4 |
| Control (Path) 3 Hippo | 5.4 | Control 1 Occipital Ctx | 0.0 |
| AD 1 Temporal Ctx | 10.2 | Control 2 Occipital Ctx | 89.5 |
| AD 2 Temporal Ctx | 51.8 | Control 3 Occipital Ctx | 16.8 |
| AD 3 Temporal Ctx | 12.9 | Control 4 Occipital Ctx | 0.0 |
| AD 4 Temporal Ctx | 22.1 | Control (Path) 1 Occipital Ctx | 66.4 |
| AD 5 Inf Temporal Ctx | 72.7 | Control (Path) 2 Occipital Ctx | 20.4 |
| AD 5 SupTemporal Ctx | 6.0 | Control (Path) 3 Occipital Ctx | 0.0 |
| AD 6 Inf Temporal Ctx | 32.3 | Control (Path) 4 Occipital Ctx | 21.5 |
| AD 6 Sup Temporal Ctx | 32.8 | Control 1 Parietal Ctx | 10.2 |
| Control 1 Temporal Ctx | 0.0 | Control 2 Parietal Ctx | 35.1 |
| Control 2 Temporal Ctx | 37.4 | Control 3 Parietal Ctx | 21.5 |
| Control 3 Temporal Ctx | 20.4 | Control (Path) 1 Parietal Ctx | 100.0 |
| Control 4 Temporal Ctx | 3.2 | Control (Path) 2 Parietal Ctx | 22.2 |
| Control (Path) 1 Temporal Ctx | 94.0 | Control (Path) 3 Parietal Ctx | 0.0 |
| Control (Path) 2 Temporal Ctx | 45.7 | Control (Path) 4 Parietal Ctx | 66.9 |

**Table VC. General_screening_panel_v1.4**

| **Tissue Name** | **Rel. Exp.(%) Ag3299, Run 215620706** | **Tissue Name** | **Rel. Exp. (%) Ag3299, Run 215620706** |
|---|---|---|---|
| Adipose | 2.1 | Renal ca. TK-10 | 2.8 |
| Melanoma* Hs688(A).T | 4.4 | Bladder | 0.0 |
| Melanoma* Hs688(B).T | 12.1 | Gastric ca. (liver met.) NCI-N87 | 7.3 |
| Melanoma* M14 | 0.0 | Gastric ca. KATO III | 1.1 |
| Melanoma* LOXIMVI | 4.1 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.*(SW480 met) SW620 | 0.0 |
| Testis Pool | 8.5 | Colon ca. HT29 | 0.0 |
| Prostate ca.* (bone met) PC-3 | 100.0 | Colon ca. HCT-116 | 4.4 |
| Prostate Pool | 6.7 | Colon ca. CaCo-2 | 0.0 |
| Placenta | 0.0 | Colon cancer tissue | 4.0 |
| Uterus Pool | 0.0 | Colon ca.SW1116 | 0.0 |
| Ovarian ca. OVCAR-3 | 6.7 | Colon ca. Colo-205 | 0.0 |
| Ovarian ca. SK-OV-3 | 6.0 | Colon ca. SW-48 | 0.0 |
| Ovarian ca. OVCAR-4 | 0.0 | Colon Pool | 7.6 |
| Ovarian ca. OVCAR-5 | 2.0 | Small Intestine Pool | 9.7 |
| Ovarian ca. IGROV-1 | 0.0 | Stomach Pool | 17.0 |
| Ovarian ca.OVCAR-8 | 0.0 | Bone Marrow Pool | 2.8 |
| Ovary | 0.0 | Fetal Heart | 2.1 |
| Breast ca. MCF-7 | 0.0 | Heart Pool | 0.0 |
| Breast ca.MDA-MB-231 | 8.4 | Lymph Node Pool | 7.0 |
| Breast ca. BT 549 | 6.8 | Fetal Skeletal Muscle | 2.2 |
| Breast ca. T47D | 2.5 | Skeletal Muscle Pool | 0.0 |
| Breast ca. MDA-N | 0.0 | Spleen Pool | 4.1 |
| Breast Pool | 6.1 | Thymus Pool | 12.9 |
| Trachea | 0.0 | CNS cancer (glio/astro) U87-MG | 2.1 |
| Lung | 1.8 | CNS cancer (glio/astro) U-118-MG | 3.6 |
| Fetal Lung | 6.8 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.0 | CNS cancer (astro) SNB-75 | 2.0 |
| Lung ca. NCI-H146 | 13.8 | CNS cancer (glio) SNB-19 | 1.3 |
| Lung ca. SHP-77 | 3.9 | CNS cancer (glio) SF-295 | 0.0 |
| Lung ca. A549 | 0.0 | Brain (Amygdala) Pool | 21.2 |
| Lung ca. NCI-H526 | 0.0 | Brain (cerebellum) | 5.5 |
| Lung ca. NCI-H23 | 47.0 | Brain (fetal) | 14.3 |
| Lung ca. NCI-H460 | 10.2 | Brain (Hippocampus) Pool | 13.9 |
| Lung ca. HOP-62 | 2.2 | Cerebral Cortex Pool | 38.7 |
| Lung ca. NCI-H522 | 0.0 | Brain (Substantia nigra) Pool | 20.9 |
| Liver | 0.0 | Brain (Thalamus) Pool | 33.4 |
| Fetal Liver | 0.0 | Brain (whole) | 36.1 |
| Liver ca. HepG2 | 0.0 | Spinal Cord Pool | 4.2 |
| Kidney Pool | 9.8 | Adrenal Gland | 3.8 |
| Fetal Kidney | 17.6 | Pituitary gland Pool | 4.1 |
| Renal ca. 786-0 | 2.6 | Salivary Gland | 0.0 |
| Renal ca. A498 | 0.0 | Thyroid (female) | 0.0 |
| Renal ca. ACHN | 0.0 | Pancreatic ca. CAPAN2 | 3.0 |
| Renal ca. U0-31 | 0.0 | Pancreas Pool | 2.1 |

**Table VD. Panel 4D**

| **Tissue Name** | **Rel. Exp.(%) Ag3299, Run 164682522** | **Tissue Name** | **Rel. Exp.(%) Ag3299, Run 164682522** |
|---|---|---|---|
| Secondary Th1 act | 2.4 | HUVEC IL-1beta | 0.0 |
| Secondary Th2 act | 0.0 | HUVEC IFN gamma | 0.0 |
| Secondary Tr1 act | 7.2 | HUVEC TNF alpha+IFN gamma | 0.0 |
| Secondary Th1 rest | 0.0 | HUVEC TNF alpha+IL4 | 0.0 |
| Secondary Th2 rest | 0.0 | HUVEC IL-11 | 11.2 |
| Secondary Tr1 rest | 0.0 | Lung Microvascular EC none | 25.5 |
| Primary Th1 act | 0.0 | Lung Microvascular EC TNFalpha + IL-1beta | 18.9 |
| Primary Th2 act | 0.0 | Microvascular Dermal EC none | 9.9 |
| Primary Tr1 act | 8.5 | Microsvasular Dermal EC TNFalpha+ IL-1beta | 0.0 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha + IL-1beta | 12.5 |
| CD45RA CD4 lymphocyte act | 0.0 | Coronery artery SMC rest | 19.8 |
| CD45RO CD4 lymphocyte act | 0.0 | Coronery artery SMC TNFalpha+ IL-1beta | 9.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte , act | 0.0 | KU-812 (Basophil) rest | 0.0 |
| CD4 lymphocyte none | 0.0 | KU-812 (Basophil) PMA/lonomycin | 0.0 |
| 2ry Th1/Th2/Tr1_anti-CD95 CH 11 | 0.0 | CCD1106(Keratinocytes)none | 0.0 |
| LAK cells rest | 0.0 | CCD1106 (Keratinocytes) TNFalpha+IL-1beta | 0.0 |
| LAK cells IL-2 | 0.0 | Liver cirrhosis | 66.4 |
| LAK cells IL-2+IL-12 | 0.0 | Lupus kidney | 0.0 |
| LAK cells IL-2+IFN gamma | 0.0 | NCI-H292 none | 10.2 |
| LAK cells IL-2+IL-18 | 13.3 | NCI-H292 IL-4 | 0.0 |
| LAK cells PMA/ionomycin | 0.0 | NCI-H292 IL-9 | 9.7 |
| NK Cells IL-2 rest | 12.5 | NCI-H292 IL-13 | 19.6 |
| Two Way MLR 3 day | 0.0 | NCI-H292 IFN gamma | 0.0 |
| Two Way MLR 5 day | 8.6 | HPAECnone | 9.2 |
| Two Way MLR 7 day | 0.0 | HPAEC TNF alpha+IL-1 beta | 0.0 |
| PBMC rest | 0.0 | Lung fibroblast none | 0.0 |
| PBMC PWM | 11.3 | Lung fibroblast TNF alpha + IL-1 beta | 0.0 |
| PBMC PHA-L | 0.0 | Lung fibroblast IL-4 | 0.0 |
| Ramos (B cell) none | 0.0 | Lung fibroblast IL-9 | 0.0 |
| Ramos (B cell) ionomycin | 0.0 | Lung fibroblast IL-13 | 9.7 |
| B lymphocytes PWM | 23.5 | Lung fibroblast IFN gamma | 0.0 |
| B lymphocytes CD40L and IL-4 | 0.0 | Dermal fibroblast CCD1070 rest | 19.5 |
| EOL-1 dbcAMIP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 7.7 |
| Dendritic cells none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IDD Colitis 2 | 0.0 |
| Monocytes rest | 11.2 | IBD Crohn's | 0.0 |
| Monocytes LPS | 0.0 | Colon | 100.0 |
| Macrophages rest | 0.0 | Lung | 41.2 |
| Macrophages LPS | 0.0 | Thymus | 0.0 |
| HUVEC none | 5.9 | Kidney | 9.2 |
| HUVEC starved | 0.0 | | |

**CNS_oeurodegeneration_v1.0 Summary:** Ag3299 This panel confirms the expression of this gene at low levels in the brain in an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders. **General_screening_panel_v1.4 Summary:** Ag3299 The CG57658-01 gene is expressed at highest levels in prostate cancer cell line PC-3 (CT=30.5). Interestingly, this gene is expressed at much lower levels in the normal prostate. Therefore, therapeutic modulation of the activity of this gene, using small molecule drugs, antibodies or protein therapeutics, may be of benefit in the treatment of prostate cancer.

This gene is also expressed at low levels in all regions of the brain examined, including amygdala, cerebral cortex, cerebellum, substantia nigra, thalamus and hippocampus. The CG57658-01 gene encodes a protein that is a variant of the human CONNEXIN40.1 gene. Cell-specific expression of connexins in the central nervous system has been shown to regulate gap junctional coupling between glial cells and neurons (ref. 1). Therefore, modulation of the activity of this gene or its protein product may be useful in the treatment of inherited demyelinating neuropathies or other neurological diseases.

### References:

### 1. Rash JE, Yasumura T, Dudek FE, Nagy JI. Cell-specific expression of connexins and evidence of restricted gap junctional coupling between glial cells and between neurons. J Neurosci 2001 Mar 15;21(6):1983-2000

The transmembrane connexin proteins of gap junctions link extracellularly to form channels for cell-to-cell exchange of ions and small molecules. Two primary hypotheses of gap junction coupling in the CNS are the following: (1) generalized coupling occurs between neurons and glia, with some connexins expressed in both neurons and glia, and (2) intercellular junctional coupling is restricted to specific coupling partners, with different connexins expressed in each cell type. There is consensus that gap junctions link neurons to neurons and astrocytes to oligodendrocytes, ependymocytes, and other astrocytes. However, unresolved are the existence and degree to which gap junctions occur between oligodendrocytes, between oligodendrocytes and neurons, and between astrocytes and neurons. Using light microscopic immunocytochemistry and freeze-fracture replica immunogold labeling of adult rat CNS, we investigated whether four of the best-characterized CNS connexins are each present in one or more cell types, whether oligodendrocytes also share gap junctions with other oligodendrocytes or with neurons, and whether astrocytes share gap junctions with neurons. Connexin32 (Cx32) was found only in gap junctions of oligodendrocyte plasma membranes, Cx30 and Cx43 were found only in astrocyte membranes, and Cx36 was only in neurons. Oligodendrocytes shared intercellular gap junctions only with astrocytes, with each oligodendrocyte isolated from other oligodendrocytes except via astrocyte intermediaries. Finally, neurons shared gap junctions only with other neurons and not with glial cells. Thus, the different cell ypes of the CNS express different connexins, which define separate pathways for neuronal versus glial gap junctional communication.

### PMID: 11245683

**Panel 4D** Summary: Ag3299 The CG57658-01 gene, which encodes a variant of the human CONNEXIN40.1 gene, is expressed at highest levels in the colon (CT = 30) and appears to be down-regulated in colon tissue isolated from Crohn's and colitis patients (CT = 40). Connexin is expressed in GAP junctions in gastrointestinal muscle (ref. 1). Thus, the expression of the transcript or the protein it encodes could be used to detect normal colon tissue. Furthermore, therapeutic modulation of the activity of this gene or its protein product could be useful in the treatment of inflammatory bowel disease.

### References:

### 1. Wang YF, Daniel EE. Gap junctions in gastrointestinal muscle contain multiple connexins. Am J Physiol Gastrointest Liver Physiol 2001 Aug;281(2):G533-43.

In the canine gastrointestinal tract, the roles that gap junctions play in pacemaking and neurotransmission are unclear. Using antibodies to connexin (Cx)43, Cx45, and Cx40, we determined the distribution of these connexins. Cx43 was present in all locations where structural gap junctions occur. Cx40 was also widely distributed in the circular muscle of the lower esophageal sphincter (LES), stomach, and ileum. Cx45 was sparsely distributed in circular muscle of the LES. In the interstitial cells of Cajal (ICC) networks of myenteric plexus, in the deep muscular and submuscular plexuses, sparse Cx45 and Cx40 immunoreactivity was present. In colon, immunoreactivity was found only in the myenteric and submuscular plexus and nearby circular muscle cells. No immunoreactivity was found in sites lacking structural gap junctions (longitudinal muscle, inner circular muscle of the intestine, and most circular muscle of the colon). Studies of colocalization of connexins suggested that in the ICC networks, some colocalization of Cx43 with Cx40 and/or Cx45 occurred. Thus gap junctions in canine intestine may be heterotypic or heteromeric and have different conductance properties in different regions based on different connexin compositions.

### PMID: 11447034

### NOV28

Expression of gene NOV28/CG57662-01 was assessed using the primer-probe set Ag3300, described in Table WA. Results of the RTQ-PCR runs are shown in Tables WB, WC and WD.

**Table WA. Probe Name Ag3300**

| **Primers** | **Sequences** | **Length** | **Start Position** | **SEQ ID NO** |
|---|---|---|---|---|
| Forward | 5'-aaagatgctgaaggagtgaaga-3' | 22 | 914 | 341 |
| Probe | TET-5'-ccatcccctaatacgcaggatggtta-3'-TAMRA | 26 | 941 | 342 |
| Reverse | 5'-tctgagagaggagmctccaa-3' | 22 | 992 | 343 |

**Table WB. CNS_neurodegeneration_v1.0**

| **Tissue Name** | **Rel. Exp.(%) Ag3300, Run 210063573** | **Tissue Name** | **Rel. Exp.(%) Ag3300, Run 210063573** |
|---|---|---|---|
| AD 1 Hippo | 0.0 | Control (Path) 3 Temporal Ctx | 0.0 |
| AD 2 Hippo | 0.0 | Control (Path) 4 Temporal Ctx | 0.0 |
| AD 3 Hippo | 0.0 | AD 1 Occipital Ctx | 0.0 |
| AD 4 Hippo | 0.0 | AD 2 Occipital Ctx (Missing) | 0.0 |
| AD 5 Hippo | 0.0 | AD 3 Occipital Ctx | 0.0 |
| AD 6 Hippo | 16.6 | AD 4 Occipital Ctx | 0.0 |
| Control 2 Hippo | 7.1 | AD 5 Occipital Ctx | 0.0 |
| Control 4 Hippo | 0.0 | AD 6 Occipital Ctx | 0.0 |
| Control (Path) 3 Hippo | 0.0 | Control Occipital Ctx | 0.0 |
| AD 1, Temporal Ctx | 0.0 | Control 2 Occipital Ctx | 0.0 |
| AD 2 Temporal Ctx | 0.0 | Control 3 Occipital Ctx | 38.4 |
| AD 3 Temporal Ctx | 0.0 | Control 4 Occipital Ctx | 0.0 |
| AD 4 Temporal Ctx | 0.0 | Control (Path) 1 Occipital Ctx | 100.0 |
| AD 5 Inf Temporal Ctx | 42.6 | Control (Path) 2 Occipital Ctx | 0.0 |
| AD 5 Sup Temporal Ctx | 0.0 | Control (Path) 3 Occipital Ctx | 0.0 |
| AD 6 Inf Temporal Ctx | 0.0 | Control (Path) 4 Occipital Ctx | 0.0 |
| AD 6 Sup Temporal Ctx | 0.0 | Control 1 Parietal Ctx | 39.8 |
| Control 1 Temporal Ctx | 0.0 | Control 2 Parietal Ctx | 0.0 |
| Control 2 Temporal Ctx | 0.0 | Control 3 Parietal Ctx | 0.0 |
| Control 3 Temporal Ctx | 0.0 | Control (Path) 1 Parietal Ctx | 0.0 |
| Control 3 Temporal Ctx | 0.0 | Control (Path) 2 Parietal Ctx | 0.0 |
| Control (Path) 1 Temporal Ctx | 57.0 | Control (Path) 3 Parietal Ctx | 0.0 |
| Control (Path) 2 Temporal Ctx | 41.8 | Control (Path) 4 Parietal Ctx | 0.0 |

**Table WC. General_screening_panel_v1.4**

| **Tissue Name** | **Rel. Exp.(%) Ag3300, Run 215669620** | **Rel. Exp.(%) Ag3300, Run 217235346** | **Tissue Name** | **Rel. Exp.(%) Ag3300, Run 215669620** | **Rel. Exp.(%) Ag3300, Run 217235346** |
|---|---|---|---|---|---|
| Adipose | 0.0 | 0.0 | Renal ca. TK-10 | 5.5 | 10.4 |
| Melanoma* Hs688(A).T | 0.0 | 0.0 | Bladder | 0.0 | 0.0 |
| Melanoma* Hs68S(B).T | 0.0 | 0.0 | Gastric ca. (liver met.) NCI-NS7 | 0.0 | 0.0 |
| Melanoma* M14 | 0.0 | 0.0 | Gastric ca. KATO III | 0.0 | 0.0 |
| Melanoma* LOXIMVI | 0.0 | 0.0 | Colon ca. SW-948 | 0.0 | 10.2 |
| Melanoma* SK-MEL-5 | 0.0 | 0.0 | Colon ca. SW480 | 0.0 | 0.0 |
| Squamous cell carcinoma SCC-4 | 0.0 | 0.0 | Colon ca.*(SW480 met) SW620 | 0.0 | 0.0 |
| Testis Pool | 4.1 | 5.6 | Colon ca. HT29 | 0.0 | 0.0 |
| Prostate ca.* (bone met)PC-3 | 0.0 | 7.1 | Colon ca. HCT-116 | 0.0 | 0.0 |
| Prostate Pool | 13.1 | 29.7 | Colon ca. CaCo-2 | 0.0 | 17.2 |
| Placenta | 0.0 | 0.0 | Colon cancer tissue | 8.7 | 18.4 |
| Uterus Pool | 0.0 | 0.0 | Colon ca. SW1116 | 0.0 | 0.0 |
| Ovarian ca. OVCAR-3 | 0.0 | 0.0 | Colon ca. Colo-205 | 0.0 | 0.0 |
| Ovarian ca. SK-OV-3 | 0.0 | 0.0 | Colon ca. SW-48 | 0.0 | 0.0 |
| Ovarian ca. OVCAR-4 | 0.0 | 0.0 | Colon Pool | 0.0 | 0.0 |
| Ovarian ca. OVCAR-5 | 19.1 | 6.7 | Small Intestine Pool | 0.0 | 5.9 |
| Ovarian ca. IGROV-1 | 0.0 | 0.0 | Stomach Pool | 0.0 | 0.0 |
| Ovarian ca. OVCAR-8 | 0.0 | 0.0 | Bone Marrow Pool | 0.0 | 0.0 |
| Ovary | 0.0 | 0.0 | Fetal Heart | 0.0 | 0.0 |
| Breast ca. MCF-7 | 7.4 | 0.0 | Heart Pool | 0.0 | 0.0 |
| Breast ca. MDA-MB-231 | 0.0 | 0.0 | Lymph Node Pool | 0.0 | 0.0 |
| Breast ca. BT 549 | 0.0 | 0.0 | Fetal Skeletal Muscle | 0.0 | 0.0 |
| Breast ca. T47D | 43.5 | 63.7 | Skeletal Muscle Pool | 0.0 | 0.0 |
| Breast ca.MDA-N | 0.0 | 0.0 | Spleen Pool | 0.0 | 0.0 |
| Breast Pool | 0.0 | 0.0 | Thymus Pool | 0.0 | 5.3 |
| Trachea | 4.4 | 0.0 | CNS cancer (glio/astro) U87-MG | 0.0 | 0.0 |
| Lung | 0.0 | 0.0 | CNS cancer (glio/astro) U-118-MG | 5.4 | 5.4 |
| Fetal Lung | 3.5 | 0.0 | CNS cancer (neuro;met) SK-N-AS | 0.0 | 0.0 |
| Lung ca. NCI-N417 | 0.0 | 0.0 | CNS Cancer (astro) SF-539 | 0.0 | 0.0 |
| Lung ca. LX-1 | 0.0 | 0.0 | CNS cancer (astro) SNB-75 | 0.0 | 0.0 |
| Lung ca. NCI-H146 | 16.0 | 0.0 | CNS cancer (glio) SNB-19 | 0.0 | 0.0 |
| Lung ca. SHP-77 | 0.0 | 0.0 | CNS cancer (glio) SF-295 | 0.0 | 0.0 |
| Lung ca. A549 | 0.0 | 0.0 | Brain (Amygdala) Pool | 6.3 | 0.0 |
| Lung ca. NCI-H526 | 100.0 | 100.0 | Brain (cerebellum) | 0.0 | 6.8 |
| Lung ca. NCI-H23 | 88.3 | 0.0 | Brain (fetal) | 7.9 | 13.9 |
| Lung ca. NCI-H460 | 34.4 | 0.0 | Brain (Hippocampus) Pool | 0.0 | 0.0 |
| Lung ca. HOP-62 | 0.0 | 0.0 | Cerebral Cortex Pool | 0.0 | 0.0 |
| Lung ca. NCI-H522 | 0.0 | 0.0 | Brain (Substantia nigra) Pool | 0.0 | 0.0 |
| Liver | 0.0 | 8.2 | Brain (Thalamus) Pool | 4.9 | 0.0 |
| Fetal Liver | 15.5 | 16.5 | Brain (whole) | 10.2 | 0.0 |
| Liver ca. HepG2 | 8.0 | 7.1 | Spinal Cord Pool | 0.0 | 0.0 |
| Kidney Pool | 0.0 | 12.7 | Adrenal Gland | 0.0 | 0.0 |
| Fetal Kidney | 0.0 | 7.6 | Pituitary gland Pool | 0.0 | 0.0 |
| Renal ca. 786-0 | 0.0 | 0.0 | 0.0 Salivary Gland | 19.6 | 14.3 |
| Renal ca. A498 | 39.8 | 0.0 | Thyroid (female) | 0.0 | 0.0 |
| Renal ca. ACHN Renal ca. ACHN | 0.0 | 0.0 | Pancreatic ca. CAPAN2 | 0.0 | 0.0 |
| Renal ca. UO-31 | 0.0 | 0.0 | Pancreas Pool | 0.0 | 0.0 |

**Table WD. Panel 4D**

| **Tissue Name** | **Rel. Exp.(%) Ag3300, Run 164682539** | **Tissue Name** | **Rel. Exp.(%) Ag3300, Run 164682539** |
|---|---|---|---|
| Secondary Th1 act | 0.0 | HUVEC IL-1beta | 0.0 |
| Secondary Th2 act | 0.0 | HWEC IFN gamma | 0.0 |
| Secondary Tr1 act | 0.0 | HUVEC TNF alpha + IFN gamma | 0.0 |
| Secondary Th1 rest | 0.0 | HUVEC TNF alpha+IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha +IL-1beta | 0.0 |
| Primary Th2 act | 0.0 | Microvascular Dermal EC none | 0.0 |
| Primary Tr1 act | 0.0 | Microsvasular Dermal EC TNFalpha+IL-1beta | 0.0 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha +IL-1beta | 0.0 |
| CD45RA CD4 lymphocyte act | 0.0 | Coronery artery SMC rest | 0.0 |
| CD45RO CD4 lymphocyte act | 0.0 | Coronery artery SMC TNFalpha + IL-1 beta | 0.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812 (Basophil) rest | 0.0 |
| CD4 lymphocyte none | 12.7 | KU-812 (Basophil) PMA/ionomycin | 0.0 |
| 2ry Th1/Th2/Tr1_anti-CD95 CH11 | 0.0 | CCD1106 (Keratinocytes) none | 0.0 |
| LAK cells rest | 0.0 | CCD1106 (Keratinocytes) TNFalpha + IL-1 beta | 0.0 |
| LAK cells IL-2 | 0.0 | Liver cirrhosis | 74.2 |
| LAK cells IL-2+IL-12 | 0.0 | Lupus kidney | 0.0 |
| LAK cells IL-2+IFN gamma | 0.0 | NCI-H292 none | 0.0 |
| LAK cells IL-2+ IL-18 | 0.0 | NCI-H292 IL-4 | 0.0 |
| LAK cells PMA/ionomycin | 31.4 | NCI-H292 IL-9 | 0.0 |
| NK Cells IL-2 rest | 0.0 | NCI-H292 IL-13 | 0.0 |
| Two Way MLR 3 day | 0.0 | NCI H292 IFN gamma , | 0.0 |
| Two Way MLR 5 day | 0.0 | HPAEC none | 0.0 |
| Two Way MLR 7 day | 0.0 | HPAEC TNF alpha + IL-1 beta | 0.0 |
| PBMC rest | 0.0 | Lung fibroblast none | 0.0 |
| PBMC PWM | 0.0 | Lung fibroblast TNF alpha + IL-1 beta | 0.0 |
| PBMC PHA-L | 0.0 | Lung fibroblast IL-4 | 0.0 |
| Ramos (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 0.0 | Lung fibroblast IFN gamma | 0.0 |
| B lymphocytes CD40L and IL-4 | 16.5 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 0.0 |
| Monocytes rest | 0.0 | IBD Crohn's | 0.0 |
| Monocytes LPS | 0.0 | Colon | 24.8 |
| Macrophages rest | 0.0 | Lung | 37.1 |
| Macrophages LPS | 0.0 | Thymus | 100.0 |
| HWEC none | 0.0 | Kidney | 0.0 |
| HUVEC starved | 0.0 | | |

**CNS_neurodegeneration_v1.0 Summary:** Ag3300 Expression of this gene is low/undetectable (CTs > 35) across all of the samples on this panel (data not shown).

**General_screening_panel-v1.4 Summary:** Ag3300 Two experiments using the same probe/primer set on this panel yielded similar results. Significant expression of the CG57662-01 gene is seen only in breast, renal, and lung cancer cell lines. Therefore, expression of this gene can be used to differentiate these lines from from the other samples on this panel. Furthermore, therapeutic modulation of the activity of this gene or its protein product, using small molecule drugs, antibodies or protein therapeutics, may be of use in the treatment of breast, renal, and lung cancer.

**Panel 4D Summary:** Ag3300 The CG57662-01 gene is expressed at significant levels only in the thymus (CT = 34.6). The putative connexin encoded for by this gene could therefore play an important role in T cell development. Small molecule therapeutics, or antibody therapeutics designed against the GPCR encoded for by this gene could be utilized to modulate immune function (T cell development) and be important for organ transplant, AIDS treatment or post chemotherapy immune reconstitiution.

### References:

### 1. Evans WH, Boitano S. Connexin mimetic peptides: specific inhibitors of gap-junctional intercellular communication. Biochem Soc Trans. 2001 Aug;29(Pt 4):606-12.

Intercellular co-operation is a fundamental and widespread feature in tissues and organs. An important mechanism ensuring multicellular homoeostasis involves signalling between cells via gap junctions that directly connect the cytosolic contents of adjacent cells. Cell proliferation and intercellular communication across gap junctions are closely linked, and a number of pathologies in which communication is disrupted are known where connexins, the gap-junctional proteins, are modified. The proteins of gap junctions thus emerge as therapeutic targets inviting the development and exploitation of chemical tools and drugs that specifically influence intercellular communication. Connexin mimetic peptides that correspond to short specific sequences in the two extracellular loops of connexins are a class of benign, specific and reversible inhibitors of gap-junctional communication that have been studied recently in a broad range of cells, tissues and organs. This review summarizes the properties and uses of these short synthetic peptides, and compares their probable mechanism of action with those of a wide range of other less specific traditional gap-junction inhibitors.

### NOV29

Expression of NOV29/CG57664-01 was assessed using the primer-probe sets Ag3301 and Ag3417, described in Tables XA and XB. Results of the RTQ-PCR runs are shown in Tables XC, XD and XE.

**Table XA. Probe Name Ag3301**

| **Primers** | **Sequences** | **Length** | **Start Position** | **SEQ ID NO** |
|---|---|---|---|---|
| Forward | 5'-ctacgatggcaaggattacatc-3' | 22 | 554 | 344 |
| Probe | TET-5'-ctgaacgaggacctgccctcctg-3'-TAMRA | 23 | 579 | 345 |
| Reverse | 5'-cacttgtgctgggagatctg-3' | 20 | 624 | 346 |

**Table XB. Probe Name Ag3417**

| **Primers** | **Sequences** | **Length** | **Start Position** | **SEQ ID NO** |
|---|---|---|---|---|
| Forward | 5'-cgcagatttaccgagtgaac-3' | 20 | 400 | 347 |
| Probe | TET-5'-gaccctgctccgctattacaaccaga-3'-TAMRA | 26 | 425 | 348 |
| Reverse | 5'-ctggatggtgtgagaaccac-3' | 20 | 460 | 349 |

**Table XC. CNS_neurodegeneration_v1.0**

| **Tissue Name** | **Rel. Exp.(%) Ag3301, Run 210063574** | **Tissue Name** | **Rel. Exp.(%) Ag3301, Run 210063574** |
|---|---|---|---|
| AD 1 Hippo | 34.4 | Control (Path) 3 Temporal Ctx | 8.8 |
| AD 2 Hippo | 48.0 | Control (Path) 4 Temporal Ctx | 71.2 |
| AD 3 Hippo | 3.8 | AD 1 Occipital Ctx | 46.0 |
| AD 4 Hippo | 15.6 | AD 2 Occipital Ctx (Missing) | 0.0 |
| AD 5 hippo | 77.4 | AD 3 Occipital Ctx | 8.3 |
| AD 6 Hippo | 100.0 | AD 4 Occipital Ctx | 36.1 |
| Control 2 Hippo | 29.1 | AD 5 Occipital Ctx | 33.4 |
| Control 4 Hippo | 33.0 | AD 6 Occipital Ctx | 25.3 |
| Control (Path) 3 Hippo | 4.6 | Control 1 Occipital Ctx | 10.6 |
| AD 1 Temporal Ctx | 33.4 | Control 2 Occipital Ctx | 41.5 |
| AD 2 Temporal Ctx | 36.6 | Control 3 Occipital Ctx | 27.2 |
| AD 3 Temporal Ctx | 7.9 | Control 4 Occipital Ctx | 10.7 |
| AD 4 Temporal Ctx | 69.3 | Control (Path) 1 Occipital Ctx | 94.6 |
| AD 5 Inf Temporal Ctx | 39.5 | Control (Path) 2 Occipital | 26.8 |
| AD 5 SupTemporal Ctx | 36.9 | Control (Path) 3 Occipital Ctx | 4.1 |
| AD 6 Inf Temporal Ctx | 94.0 | Control (Path) 4 Occipital Ctx | 45.4 |
| AD 6 Sup Temporal Ctx | 96.6 | Control 1 Parietal Ctx | 21.0 |
| Cpntro) 1 Temporal Ctx | 15.7 | Control 2 Parietal Ctx | 36.1 |
| Controt 2 Temporal Ctx | 37.6 | Control 3 Parietal Ctx | 20.6 |
| Control 3 Temporal Ctx | 14.7 | Control(Path) 1 Parietal | 71.7 |
| Control 4 Temporal Ctx | 20.0 | Control (Path) 2 Parietal Ctx | 43.2 |
| Control (Path) 1 Temporal Ctx | 85.9 | Control (Path) 3 Parietal Ctx | 5.4 |
| Control (Path) 2 Temporal Ctx | 52.9 | Control (Path) 4 Parietal Ctx | 88.9 |

**Table XD. General_screening_panel_v1.4**

| **Tissue Name** | **Rel. Exp.(%) Ag3301, Run 215670031** | **Rel. Exp.(%) Ag3301, Run 217235352** | **Tissue Name** | **Rel. Exp.(%) Ag3301, Run 215670031** | **Rel. Exp.(%) Ag3301, Run 217235352** |
|---|---|---|---|---|---|
| Adipose | 7.4 | 7.9 | Renal ca. TK-10 | 1.6 | 0.9 |
| Melanoma* Hs688(A).T | 2.0 | 1.8 | Bladder | 18.3 | 20.7 |
| Melanoma* Hs688(B).T | 1.3 | 1.8 | Gastric ca. (liver met.) NCI-N87 | 57.0 | 52.5 |
| Melanom* M14 | 0.2 | 0.4 | Gastrte ca. KATO III | 25.5 | 27.7 |
| Melanoma* LOXIMVI | 5.9 | 4.0 | Colon ca. SW-948 | 4.6 | 4.8 |
| Melanoma* SK-MEL-5 | 3.3 | 4.0 | Colon ca. SW480 | 7.9 | 6.9 |
| Squamous cell carcinoma SCC-4 | 1.4 | 1.4 | Colon ca.* (SW480 met) SW620 | 3.6 | 3.1 |
| Testis Pool | 2.3 | 1.5 | Colone ca HT29 | 0.0 | 0.0 |
| Prostate ca.* (bonne met) PC-3 | 5.0 | 4.2 | Colon ca. HCT-116 | 3.2 | 3.4 |
| Prostate Pool | 4.5 | 4.6 | Colon ca. CaCo-2 | 0.4 | 0.5 |
| Placenta | 4.4 | 4.4 | Colon cancer tissue | 87.7 | 100.0 |
| Uterus Pool | 1.8 | 1.7 | Colon ca.SW1116 | 2.3 | 3.5 |
| Ovarian ca. OVCAR-3 | 8.6 | 8.4 | Colon ca. Colo-205 | 8.8 | 6.7 |
| Ovarian ca. SK-OV-3 | 2.5 | 4.3 | Colon ca. SW-48 | 25.0 | 16.8 |
| Ovarian ca. OVCAR-4 | 8.8 | 9.1 | Colon Pool | 6.8 | 7.5 |
| Ovarian ca. OVCAR-5 | 32.5 | 20.6 | Small Intestine Pool | 6.8 | 6.7 |
| Ovarian ca. IGROV-1 | 6.2 | 4.2 | Stomach Pool | 5.3 | 7.6 |
| Ovarian ca. OVCAR-8 | 24.8 | 30.8 | Bone Marrow Pool | 1.9 | 1.7 |
| Ovary | 9.1 | 7.7 | Fetal Heart | 1.4 | 0.9 |
| Breast ca. MCF-7 | 0.4 | 0.3 | Heart Pool | 2.9 | 4.4 |
| Breast ca. MDA-MB-231 | 100.0 | 73.2 | Lymph Node Pool | 8.6 | 9.8 |
| Breast ca. BT 549 | 8.5 | 9.3 | Fetal Skeletal Muscle | 0.6 | 0.4 |
| Breast ca. T47D | 86.5 | 93.3 | Skeletal Muscle Pool | 3.5 | 4.1 |
| Breast ca. MDA-N | 0.3 | 0.3 | Spleen Pool | 24.5 | 31.9 |
| Breast Pool | 7.5 | 6.2 | Thymus Pool | 13.2 | 11.7 |
| Trachea | 15.5 | 13.6 | CNS cancer (glio/astro) U87-MG | 0.1 | 0.2 |
| Lung | 0.9 | 1.1 | CNS cancer (glio/astro)U-118-MG | 8.9 | 10.7 |
| Fetal Lung | 8.1 | 9.2 | CNS cancer (neuro;met) SK-N-AS | 5.8 | 4.9 |
| Lung ca. NCI-N417 | 0.1 | 0.2 | CNS cancer (astro) SF-539 | 14.4 | 18.3 |
| Lung ca. LX-1 | 6.0 | 6.8 | CNS cancer (astro) | 21.5 | 34.4 |
| Lung ca. NCI-H146 | 0.8 | 0.4 | CNS cancer (glio) SNB-19 | 6.3 | 5.0 |
| Lung ca. SHP-77 | 0.2 | 0.3 | CNS cancer (glio) SF-295 | 22.4 | 17.9 |
| Lung ca. A549 | 4.4 | 3.8 | Brain (Amygdala) Pool | 4.5 | 3.5 |
| Lung ca. NCI-H526 | 15.3 | 12.8 | Brain (cerebellum) | 7.3 | 5.4 |
| Lung ca. NCI-H23 | 15.1 | 9.4 | Brain (fetal) | 4.9 | 5.6 |
| Lung ca. NCI. H460 | 10.7 | 9.8 | Brain (Hippocampus) Pool | 4.1 | 3.6 |
| Lung ca. HOP-62 | 20.6 | 18.3 | Cerebral Cortex Pool | 4.2 | 3.8 |
| Lung ca.NCI-H522 | 2.4 | 1.3 | Brain (Substantia nigra) Pool | 6.6 | 6.3 |
| Liver | 1.9 | 1.5 | Brain (Thalamus) Pool | 4.9 | 6.0 |
| Fetal Liver | 2.0 | 1.8 | Brain (whole) | 3.8 | 4.7 |
| Liver ca. HepG2 | 1.2 | 1.2 | Spinal Cord Pool | 3.0 | 3.7 |
| Kidney Pool | 15.0 | 16.7 | Adrenal Gland | 7.3 | 7.9 |
| Fetal Kidney | 2.6 | 2.6 | Pituitary gland Pool | 2.7 | 2.3 |
| Renal ca. 786-0 | 6.4 | 5.6 | Salivary Gland | 4.0 | 3.6 |
| Renal ca. A498 | 11.7 | 10.6 | Thyroid (female) | 4.2 | 4.0 |
| Renal ca. ACHN | 4.2 | 2.6 | Pancreatic ca. CAPAN2 | 13.6 | 9.6 |
| Renal ca. UO-31 | 7.9 | 4.7 | Pancreas Pool | 11.9 | 13.6 |

**Table XE. Panel 4D**

| **Tissue Name** | **Rel. Exp.(%) Ag3301, Run 164682541** | **Rel. Exp.(%) Ag3417, Run 166453854** | **Tissue Name** | **Rel. Exp·(%) Ag3301, Run 164682541** | **Rel. Exp.(%) Ag3417, Run 166453854** |
|---|---|---|---|---|---|
| Secondary Th1 act | 13.6 | 0.0 | HUVEC IL-1beta | 0.2 | 0.0 |
| Secondary Th2 act | 44.4 | 0.0 | HUVEC IFN gamma | 5.9 | 100.0 |
| Secondary Tr1 act | 33.9 | 0.0 | HUVEC IFN TNF alpha + IFN gamma | 0.4 | 0.0 |
| Secondary Th1 rest | 31.0 | 0.0 | HUVEC TNF alpha + IL4 | 0.2 | 0.0 |
| Secondary Th2 rest | 22.4 | 0.0 | HUVEC IL-11 | 1.9 | 0.0 |
| Secondary Tr1 rest | 34.9 | 0.0 | Lung Microvascular EC none | 8.5 | 0.0 |
| Primary Th1 act | 21.5 | 0.0 | Lung Microvascular EC TNFalpha+IL-1beta | 13.4 | 0.0 |
| Primary Th2 act | 22.5 | 0.0 | Microvascular Dermal EC none | 6.0 | 0.0 |
| Primary Tr1 act | 33.9 | 0.0 | Microsvasular Dermal BC TNFalpha + IL-1beta | 22.5 | 0.0 |
| Primary Th1 rest | 37.4 | 0.0 | Bronchial epithelium TNFalpha+IL1beta | 16.8 | 0.0 |
| Primary Th2 rest | 0.0 | 0.0 | Small airway epithelium none | 0.0 | 0.0 |
| Primary Tr1 rest | 17.2 | 0.0 | Small airway epithelium TNFalpha + IL-1beta | 0.7 | 0.0 |
| CD45RA CD4 lymphocyte act | 10.6 | 0.0 | Coronery artery SMC rest | 1.1 | 0.0 |
| CD45R0 CD4 lymphocyte act | 29.3 | 0.0 | Coronery artery SMC TNFalpha + IL-1beta | 0.3 | 0.0 |
| CD8 lymphocyte act | 29.3 | 0.0 | Astrocytes rest | 8.1 | 0.0 |
| Secondary CD8 lymphocyte rest | 48.3 | 0.0 | Astrocytes TNFalpha + IL-1beta | 7.0 | 0.0 |
| Secondary CD8 lymphocyte act | 2.4 | 0.0 | KU-812 (Basophil) rest | 0.8 | 0.0 |
| CD4 lymphocyte none PMA/ionomycin | 13.6 | 0.0 | KU-812 (Basophil) | 2.4 | 0.0 |
| 2ry Th1/Th2/Tr1_anti-CD95 CH11 | 173 | 0.0 | CCD1106 (Keratinocytes) none | 4.1 | 0.0 |
| LAK cells rest | 100.0 | 0.0 | CCD1106 (Keratinocytes) TNFalpha+IL-1beta | 12.0 | 0.0 |
| LAK cells IL-2 | 54.7 | 0.0 | Liver cirrhosis | 8.4 | 0.0 |
| LAK cells IL-2+IL,12 | 49.7 | 0.0 | Lupus kidney | 0.4 | 0.0 |
| LAK cells IL-2+IFN gamma | 44.8 | 0.0 | NCI-H292 none | 2.3 | 0.0 |
| LAK cells IL-2+IL- 18 | 34.2 | 0.0 | NCI-H292 IL-4 | 3.6 | 0.0 |
| LAK cells PMA/ionomycin | 80.1 | 0.0 | NCI-H292 IL-9 | 2.6 | 0.0 |
| NK Cells IL-2 rest | 24.1 | 0.0 | NCI-H292 IL-13 | 1.4 | 0.0 |
| Two Way MLR3 day | 58.6 | 0.0 | NCI-H292 IFN gamma | 1.4 | 0.0 |
| Two Way MLR 5 day | 25.9 | 0.0 | HPAECnone | 6.0 | 0.0 |
| Two Way MLR 7 day | 6.6 | 0.0 | HPAEC TNF alpha + IL-1 beta | 23.7 | 0.0 |
| PBMC rest | 14.5 | 0.0 | Lung fibroblast none | 0.7 | 0.0 |
| PBMC PWM | 35.4 | 0.0 | Lung fibroblast TNF alpha+ IL-1 beta | 0.1 | 0.0 |
| PBMC PHA-L | 24.8 | 0.0 | Lung fibroblast IL-4 | 0.5 | 0.0 |
| Ramos (B cell) none | 8.4 | 0.0 | Lung fibroblast IL-9 | 0.4 | 0.0 |
| Ramos (B cell) ionomycin | 14.7 | 0.0 | Lung fibroblast IL-13 Lung fibroblast IL-13 | 0.5 | 0.0 |
| B lymphocytes PWM | 12.9 | 0.0 | Lung fibroblast IFN gamma | 1.5 | 0.0 |
| B lymphocytes CD40L and IL-4 | 18.4 | 0.0 | Dermal fibroblast CCD1070 rest | 12.5 | 0.0 |
| EOL-1 dbcAMP | 0.8 | 0.0 | Dermal fibroblast CCD1070 TNF alpha | 8.2 | 0.0 |
| EOL-1 dbcAMP PMA/ionomycin | 0.3 | 0.0 | Dermal fibroblast CCD1070 IL- I beta | 6.5 | 0.0 |
| Dendritic cells none | 59.0 | 0.0 | Dermal fibroblast IFN gamma | 34.6 | 0.0 |
| Dendritic cells LPS | 10.8 | 0.0 | Dermal fibroblast IL-4 | 9.7 | 0.0 |
| Dendritic cells anti-CD40 | 16.8 | 0.0 | IBD Colitis 2 | 1.2 | 0.0 |
| Monocytes rest | 36.1 | 0.0 | IBD Crohn's | 0.2 | 0.0 |
| Monocytes LPS | 30.1 | 0.0 | Colon | 27.2 | 0.0 |
| Macrophages rest | 17.3 | 0.0 | Lung | 6.1 | 0.0 |
| Macrophages LPS' | 57.4 | 0.0 | Thymus | 8.4 | 0.0 |
| HUVEC none | 0.2 | 0.0 | Kidney | 33.7 | 0.0 |
| HUVEC starved | 0.4 | 0.0 | | | |

**CNS_neurodegeneration_v1.0 Summary:** Ag3301 This panel confirms the expression of this gene at low to moderate levels in the brain in an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders. Ag3417 Expression of this gene is low/undetectable (CTs > 35) across all of the samples on this panel (data not shown).

**General_screening_panel_v1.4 Summary:** Ag3301 Results from two experiments with using the same probe/primer set yielded results that are in excellent agreement, Expression of the CG57664-01 gene is moderate to high across the majority of samples on this panel, with highest expression in a colon cancer tissue (CT = 26.8). This gene appears to be more highly expressed in CNS, colon, gastric, renal, lung, ovarian, breast, and melanoma cancer cell lines when compared to normal tissues. The CG57664-01 gene encodes a protein with homology to MHC class 1 antigen. The major histocompatibility complex (MHC) class I family of glycoproteins presents peptides for immunorecognition by cytotoxic T lymphocytes. Increased expression of this gene in cancer cell lines agrees with the results of other studies that found elavated levels of HLA class I antigen in tumors (ref. 1). Therefore, reduction in the expression or activity of this novel HLA class I antigen may make cancer cells sensitive to lysis by natural killer cells.

In addition, this gene is expressed at moderate levels in all regions of the central nervous system examined including amygdala, hippocampus, substantia nigra, cerebellum, cerebral cortex, thalamus and spinal cord. Class I MHC molecules, known to be important for immune responses to antigen, are expressed also by neurons that undergo activity-dependent, long-term structural and synaptic modifications (ref. 2). Specific class I MHC mRNAs are expressed by distinct mosaics of neurons, reflecting a potential for diverse neuronal functions and suggesting an important role for these molecules in the activity-dependent remodeling and plasticity of connections in the'developing and mature mammalian central nervous system.

Among tissues with metabolic or endocrine function, this gene is expressed at moderate levels in adrenal gland, thyroid, pituitary gland, pancreas, adipose, skeletal muscle, heart and liver. Therefore, therapeutic modulation of the activity of this gene may prove useful in the treatment of endocrine/metabolically related diseases, such as obesity and diabetes.

### References:

### 1. Sette A, Chesnut R, Fikes J. HLA expression in cancer: implications for T cell-based immunotherapy. Immunogenetics 2001 May-Jun;53(4):255-63.

HLA class I expression is altered in a significant fraction of the tumor types reviewed here, reflecting either immune pressure or, simply, the accumulation of pathological changes and alterations. However, in all tumor types analyzed, a majority of the tumors express HLA class I. with a general tendency for the more severe alterations to be found in later-stage and less differentiated tumors. These results are encouraging for the development of specific immunotherapies, especially considering that (1) the relatively low sensitivity of immunohistochemical techniques might underestimate HLA expression in tumors, (2) class I expression can be induced in tumor cells as a result of local inflammation and lymphokine release, and (3) class I-negative cells would be predicted to be sensitive to lysis by natural killer cells.

### PMID: 11491528

### 2. Huh GS, Boulanger LM, Du H, Riquelme PA, Brotz TM, Shatz CJ. Functional requirement for class I MHC in CNS development and plasticity. Science 2000 Dec 15;290(5499):2155-9

Class I major histocompatibility complex (class I MHC) molecules, known to be important for immune responses to antigen, are expressed also by neurons that undergo activity-dependent, long-term structural and synaptic modifications. Here, we show that in mice genetically deficient for cell surface class I MHC or for a class I MHC receptor component, CD3zeta, refinement of connections between retina and central targets during development is incomplete. In the hippocampus of adult mutants, N-methyl-D aspartate receptor-dependent long-term potentiation (LTP) is enhanced, and long-term depression (LTD) is absent. Specific class I MHC messenger RNAs are expressed by distinct mosaics of neurons, reflecting a potential for diverse neuronal functions. These results demonstrate an important role for these molecules in the activity dependent remodeling and plasticity of connections in the developing and mature mammalian central nervous system (CNS).

### PMID: 11118151

**Panel 4D Summary:** Ag3301 The CG57664-01 gene is moderately expressed in a number of samples on this panel, with highest expression in lymphokine-activated killer (LAK) cells (CT = 26-28), independent of stimulation. This observation suggests that modulation of the expression or activity of this gene may be useful in the treatment of intracellular bacterial or viral infections. This gene is also expressed at significant levels in T cells, B cells, dendritic cells, monocytes and macrophages.

The CG57664-01 gene encodes a protein with homology to MHC class I antigen. Major histocompatibility complex (MHC) class I molecules present antigenic peptides to CD8-positive T cells. The HLA class I gene family in humans consists of 6 members: polymorphic HI,A-A, HLA-B, and HLA-C, and oligomorphic HLA-E, HLA-F, and HLA-G. BLA-A/B-alleles such as the CG57664-01 gene may have utility in genotyping criminals as well as in paternity disputes. These antigens are ubiquitously expressed on all nucleated human cells except neurons and trophoblasts, and participate in antigen presentation of viral antigens in the adaptive immune response.

Ag3417 Results from one experiment with the CG57664-01 gene are not included. The amp plot indicates that there were experimental difficulties with this run.

### NOV30

Expression of NOV30/CG57666-01 was assessed using the primer-probe set Ag3302, described in Table YA.

**Table YA. Probe Name Ag3302**

| **Primers** | **Sequences** | **Length** | **start Position** | **SEQ ID NO** |
|---|---|---|---|---|
| Forward | 5'-gtgatgtgcaggaagaactcat-3' | 22 | 1056 | 350 |
| Probe | TET-5'-tttgttctaccccaggcagcaaccat-3'-TAMRA | 26 | 1078 | 351 |
| Reverse | 5'-ttacaagccgtgagagacaca-3' | 21 | 1118 | 352 |

**CNS_neurodegeneration_v1.0 Summary:** Ag3302 Expression of the CG57666-01 gene is low/undetectable (CTs > 35) across all of the samples on this panel (data not shown).

**General_screening_panel_v1.4 Summary:** Ag3302 Expression of the CG57666-01 gene is low/undetectable (CTs > 35) across all of the samples on this panel (data not shown).

**Panel 4D Summary:** Ag3302 Expression of the CG57666-01 gene is low/undetectable (CTs > 35) across all of the samples on this panel (data not shown).

### NOV31

Expression ofNOV31/CG57668-01 was assessed using the primer-probe set Ag3303, described in Table ZA. Results of the RTQ-PCR runs are shown in Tables ZB, ZC and ZD.

**Table ZA. Probe Name Ag3303**

| **Primers** | **Sequences** | **Length** | **Start Position** | **SEQ ID NO** |
|---|---|---|---|---|
| Forward | 5'-ctacgacggcaaggattacat-3' | 21 | 438 | 353 |
| Probe | TET-5'-ctctgaacgaggacctgcgctcct-3'-TAMRA | 24 | 461 | 354 |
| Reverse | 5'-gtgatctgagctgccatgtc-3' | 20 | 496 | 355 |

**Table ZB. CNS_neurodegenetation_v1.0**

| **Tissue Name** | **Rel. Exp.(%) Ag3303, Run 210063655** | **Tissue Name** | **Rel. Exp.(%) Ag3303, Run 210063655** |
|---|---|---|---|
| AD 1 Hippo | 27.4 | Control (Path) 3 Temporal Ctx | 16.6 |
| AD 2 Hippo | 55.9 | Control (Path) 4 Temporal Ctx | 14.5 |
| AD 3 Hippo | 14.3 | AD 1 Occipital Ctx | 16.3 |
| AD 4 Hippo | 35.6 | AD 2 Occipital Ctx (Missing) | 0.0 |
| AD 5 hippo | 96.6 | AD 3 Occipital Ctx | 21.8 |
| AD 6 Hippo | 12.7 | AD 4 Occipital Ctx | 29.3 |
| Control 2 Hippo | 30.4 | AD 5 Occipital Ctx | 3.0 |
| Control 4 Hippo | 94.6 | AD 6 Occipital Ctx | 37.4 |
| Control (Path) 3 Hippo | 15.3 | Control 1 Occipital Ctx | 16.5 |
| AD 1 Temporal Ctx | 18.2 | Control 2 Occipital Ctx | 47.0 |
| AD 2 Temporal Ctx | 34.4 | Control 3 Occipital Ctx | 18.0 |
| AD 3 Temporal Ctx | 17.9 | Control 4 Occipital Ctx | 37.6 |
| AD 4 Temporal Ctx | 31.9 | Control (Path) 1. Occipital Ctx | 18.4 |
| AD 5 Inf Temporal Ctx | 100.0 | Control (Path) 2 Occipital ctx | 13.9 |
| AD 5 SupTemporal Ctx | 100.0 | Control (Path) 3 Occipital ctx | 12.3 |
| AD 6 Inf Temporal Ctx | 6.5 | Control (Path) 4 Occipital | 13.4 |
| AD 6 Sup Temporal Ctx | 11.0 | Control 1 Parietal Ctx | 18.2 |
| Control 1 Temporal Ctx | 19.6 | Control 2 Parietal Ctx | 72.7 |
| Control 2 Temporal Ctx | 40.6 | Control 3 Parietal Ctx | 21.3 |
| Control 3 Temporal Ctx | 20.6 | Control (path) 1 Parietal | 15.2 |
| Control 4 Temporal Ctx | 61.1 | Control (Path) 2 Parietal Ctx | 22.2 |
| Control (Path) 1 Temporal Ctx | 14.0 | Control (Path) 3 Parietal Ctx | 11.6 |
| Control (Path) 2 Temporal Ctx | 31.6 | Control (Path) 4 Parietal Ctx | 18.2 |

**Table ZC. General_screening_panel_vl.4**

| **Tissue Name** | **Rel. Exp.(%) Ag3303, Run 215648467** | **Tissue Name** | **Rel. Exp.(%) Ag3303, Run 215648467** |
|---|---|---|---|
| Adipose | 0.1 | Renal ca. TK-10 | 2.1 |
| Melanoma* Hs688(A).T | 20.7 | Bladder | 0.4 |
| Melanoma* Hs688(B).T | 15.5 | Gastric ca. (liver met.) NCI-N87 | 100.0 |
| Melanoma* M14 | 4.2 | Gastric ca. KATO III | 0.2 |
| Melanoma* LOXIMVI | 4.2 | Colon ca. SW-948 | 6.2 |
| Melanoma* SK-MEL-5 | 8.0 | Colon ca. SW480 | 0.3 |
| Squamous cell carcinoma SCC-4 | 0.2 | Colon ca.*(SW480 met) SW620 | 0.1 |
| Testis Pool | 2.3 | Colon ca. HT29 | 2.8 |
| Prostate ca.* (bone met) PC-3 | 5.5 | Colon ca. HCT-116 | 9.8 |
| Prostate Pool | 1.5 | Colon ca. CaCo-2 | 0.4 |
| Placenta | 2.9 | Colon cancer tissue | 18.0 |
| Uterus Pool | 1.9 | Colon ca.SW1116 | 0.1 |
| Ovarian ca. OVCAR-3 | 0.2 | Colon ca. Colo-205 | 5.5 |
| Ovarian ca. SK-OV-3 | 23.5 | Colon ca. SW-48 | 19.9 |
| Ovarian ca. OVCAR-4 | 4.2 | Colon Pool | 2.0 |
| Ovarian ca. OVCAR-5 | 12.1 | Small Intestine Pool | 3.3 |
| Ovarian ca. IGROV-1 | 0.2 | Stomach Pool | 4.6 |
| Ovarian ca. OVCAR-8 | 28.5 | Bone Marrow Pool | 2.8 |
| Ovary | 4.6 | Fetal Heart | 4.4 |
| Breast ca. MCF-7 | 0.0 | Heart Pool | 3.5 |
| Breast ca. MDA-MB-231 | 1.0 | Lymph Node Pool | 9.4 |
| Breast ca.BT 549 | 4.1 | Fetal Skeletal Muscle | 0.3 |
| Breast ca. T47D | 63.7 | Skeletal Muscle Pool | 3.5 |
| Breast ca. MDA-N | 3.2 | Spleen Pool | 21.3 |
| Breast Pool | 1.9 | Thymus Pool | 10.9 |
| Trachea | 14.8 | CNS cancer (glio/astro) U87-MG | 0.1 |
| Lung | 1.5 | CNS cancer (glio/astro) U-118-MG | 1.2 |
| Fetal Lung | 14.0 | CNS cancer (neuro;met) SK-N-AS | 0.6 |
| Lung ca. NCI-N417 | 0.5 | CNS cancer (astro) SF-539 | 0.3 |
| Lung ca. LX-1 | 0.0 | CNS cancer (astro) SNB-75 | 65.5. |
| Lung ca. NCI-H146 | 5.3 | CNS cancer (glio) SNB-19 | 0.3 |
| Lung ca. SHP-77 | 0.0 | CNS cancer (glio) SF-295 | 71.2 |
| Lung ca. A549 | 2.5 | Brain (Amygdala) Pool | 1.7 |
| Lung ca. NCI-H526 | 5.8 | Brain (cerebellum) | 2.9 |
| Lung ca. NCI-H23 | 11.7 | Brain (fetal) | 1.6 |
| Lung ca. NCI-H460 | 0.0 | Brain (Hippocampus)Pool | 0.4 |
| Lung ca. HOP-62 | 21.2 | Cerebral Cortex Pool | 0.4 |
| Lung ca. NCI-H522 | 0.0 | Brain (Substantia nigra) Pool | 0.8 |
| Liver | 4.0 | Brain (Thalamus) Pool | 1.0 |
| Fetal Liver | 1.7 | Brain (whole) | 1.1 |
| Liver ca. HepG2 | 0.0 | Spinal Cord Pool | 1.4 |
| Kidney Pool | 9.6 | Adrenal Gland | 14.0 |
| Fetal Kidney | 2.2 | Pituitary gland Pool | 4.1 |
| Renal ca. 786-0 | 17.8 | Salivary Gland | 2.8 |
| Renal ca. A498 | 0.0 | Thyroid (female) | 3.1 |
| Renal ca. ACHN | 2.8 | Pancreatic ca. CAPAN2 | 4.0 |
| Renal ca. UO-31 | 28.7 | Pancreas Pool | 8.2 |

**Table ZD. Panel 4D**

| **Tissue Name** | **Rel. Exp.(%) Ag3303, Run 164682641** | **Tissue Name** | **Rel. Exp.(%) Ag3303, Run 164682641** |
|---|---|---|---|
| Secondary Th1 act | 38.4 | HUVEC IL-1beta | 1.7 |
| Secondary Th2 act | 63.7 | HUVEC IFN gamma | 6.2 |
| Secondary Tr1 act | 52.1 | HUVEC TNF alpha+ IFN gamma | 7.6 |
| Secondary Th1 rest | 1.5 | HUVEC TNF alpha + IL4 | 3.2 |
| Secondary Th2 rest | 2.0 | HUVEC IL-11 | 3.0 |
| Secondary Tr1 rest | 3.0 | Lung Microvascular EC none | 26.6 |
| Primary Th1 act | 2.7 | Lung Microvascular EC TNFalpha +IL-1beta | 41.5 |
| Primary Th2 act | 4.7 | Microvascular Dermal EC none | 18.9 |
| Primary Tr1 act | 6.0 | Microsvasular Dermal EC TNFalpha + IL-1beta | 38.2 |
| Primary Th1 rest | 81.2 | Bronchial epithelium TNFalpha + IL1beta | 27.7 |
| Primary Th2 rest | 50.7 | Small airway epithelium none | 10.9 |
| Primary Tr1 rest | 33.0 | Small airway epithelium TNFalpha +IL-1 beta | 24.3 |
| CD45RA CD4 lymphocyte act | 28.7 | Coronery artery SMC rest | 2.9 |
| CD45RO CD4 lymphocyte act | 44.4 | Coronery artery SMC TNFalpha+ IL-1 beta | 2.8 |
| CD8 lymphocyte act | 26.8 | Astrocytes rest | 0.9 |
| Secondary CD8 lymphocyte rest | 44.4 | Astrocytes TNFalpha+IL-1beta | 1.2 |
| Secondary CD8 lymphocyte act | 9.6 | KU-812(Basophil)rest | 0.6 |
| CD4 lymphocyte none | 17.0 | KU-812 (Basophil) PMA/ionomycin | 1.7 |
| 2ry Th1/Th2/Tr1_anti-CD95 CH11 | 33.0 | CCD1106 (Keratinocytes) none | 11.7 |
| LAK cells rest LAK cells rest | 87.7 | CCD1106 (Keratinocytes) TNFalpha+IL-1 beta | 28.9 |
| LAK cells IL-2 | 60.3 | Liver cirrhosis | 3.4 |
| LAK cells IL-2+IL-12 | 60.3 | Lupus kidney | 1.0 |
| LAK cells IL-2+IFN gamma | 77.9 | NCI-H292 none | 2.0 |
| LAK cells IL-2+IL-18 | 66.0 | NCI-H292 IL-4 | 3.3 |
| LAK cells PMA/ionomycin | 100.0 | NCI-H292 IL-9 | 3.0 |
| NK Cells IL-2 rest | 45.1 | NCI-H292 IL-13 | 3.0 |
| Two Way MLR 3 day | 98.6 | NCI-H292 IFN gamma | 6.0 |
| Two Way MILR 5 day | 67.4 | HPAEC none | 1.8 |
| Two Way MLR 7 day | 20.0 | HPAEC TNFalpha+IL-1beta | 9.5 |
| PBMC rest | 30.1 | Lung fibroblast none | 21.3 |
| PBMCPWM | 82.9 | Lung fibroplast TNF alpha+IL-1 beta | 59.0 |
| PBMC PHA-L | 52.5 | Lung fibroblast IL-4 | 28.1 |
| Ramos (B cell) none | 29.1 | Lung fibroblast IL-9 | 22.4 |
| Ramos (B cell) ionomycin | 45.4 | Lung fibroblast IL-13 | 20.7 |
| B lymphocytes PWM | 24.1 | Lung fibroblast IFN gamma | 40.1 |
| B lymphocytes CD40L and IL-4 | 12.4 | Dermal fibroblast CCD1070 rest | 17.8 |
| EOL-1dbcAMP | 1.5 | Dermal fibroblast CCD1070TNF alpha | 24.1 |
| EOL-1 dbcAMP | 1.1 | Dermal fibroblast CCD1070 IL-1 | 14.5 |
| PMA/ionomycin | | beta | |
| Dendritic cells none | 56.6 | Dermal fibroblast IFN gamma | 2.7 |
| Dendritic cells LPS | 29.9 | Dermal fibroblast IL-4 | 1.4 |
| Dendritic cells anti-CD40 | 41.8 | IBD Colitis 2 | 2.7 |
| Monocytes rest | 53.6 | IBD Crohn's | 8.2 |
| Monocytes LPS | 66.9 | Colon | 5.6 |
| Macrophages rest | 88.9 | Lung | 46.0 |
| MacrophagesLPS | 82.4 | Thymus | 10.7 |
| HUVEC none | 1.0 | Kidney | 34.9 |
| HUVEC starved | 2.5 | | |

**CNS_neurodegeneration_v1.0 Summary:** Ag3303 This panel confirms the expression of this gene at moderate to high levels in the brain in an independent group of individuals. However, no differential expression of this gene was detected between Alzheimer's diseased postmortem brains and those of non-demented controls in this experiment Please see Panel 1.4 for a discussion of the potential utility of this gene in treatment of central nervous system disorders.

**General_screening_panel_v1.4 Summary:** Ag3303 Expression of the CG57668-01 gene is moderate to high across the majority of samples on this panel, with highest expression in a gastric cancer cell line (CT = 24.8). This gene appears to be more highly expressed in CNS, colon, gastric, renal, lung, ovarian, breast, and melanoma cancer cell lines when compared to normal tissues. The CG57668-01 gene encodes a protein that is highly homologous to HLA class I histocompatibility antigen, alpha chain H precursor (HLA-AR). The major histocompatibility complex (MHC) class I family of glycoproteins presents peptides for immunorecognition by cytotoxic T lymphocytes. Increased expression of this gene in cancer cell lines agrees with the results of other studies that found elavated levels of HLA class I antigen in tumors (ref. 1). Therefore, reduction in the expression or activity of this novel HLA class I antigen may make cancer cells sensitive to lysis by natural killer cells.

In addition, this gene is expressed at moderate levels in all regions of the central nervous system examined including amygdala, hippocampus, substantia nigra, cerebellum, cerebral cortex, thalamus and spinal cord. Class I MHC molecules, known to be important for immune responses to antigen, are expressed also by neurons that undergo activity-dependent, long-term structural and synaptic modifications (ref. 2). Specific class I MHC mRNAs are expressed by distinct mosaics of neurons, reflecting a potential for diverse neuronal functions and suggesting an important role for these molecules in the activity-dependent remodeling and plasticity of connections in the developing and mature mammalian central nervous system.

Among tissues with metabolic or endocrine function, this gene is expressed at moderate levels in adrenal gland, thyroid, pituitary gland, pancreas, skeletal muscle, heart and liver. Therefore, therapeutic modulation of the activity of this gene may prove useful in the treatment of endocrine/metabolically related diseases, such as obesity and diabetes. In addition, there are significant differences of expression in fetal lung(CT = 27.8) and adult lung (CT = 30.8) as well as fetal skeletal muscle (CT = 33.2) and adult skeletal muscle (CT = 29.6). Therefore expression of this gene can be used to differentiate between fetal and adult lung as well as fetal and adult skeletal muscle.

### References:

### 1. Sette A, Chesnut R, Fikes J. HLA expression in cancer: implications for T cell-based immunotherapy. Immunogenetics 2001 May-Jun;53(4):255-63.

HLA class I expression is altered in a significant fraction of the tumor types reviewed here, reflecting either immune pressure or, simply, the accumulation of pathological changes and alterations. However, in all tumor types analyzed, a majority of the tumors express HLA class I. with a general tendency for the more severe alterations to be found in later-stage and less differentiated tumors. These results are encouraging for the development of specific immunotherapies, especially considering that (1) the relatively low sensitivity of immunohistochemical techniques might underestimate HLA expression in tumors, (2) class I expression can be induced in tumor cells as a result of local inflammation and lymphokine release, and (3) class I-negative cells would be predicted to be sensitive to lysis by natural killer cells.

### PMID: 11491528

### 2. Huh GS, Boulanger LM, Du H, Riquehne PA, Brotz TM, Shatz CJ. Functional requirement for class I MHC in CNS development and plasticity. Science 2000 Dec 15;290(5499):2155-9

Class I major histocompatibility complex (class I MHC) molecules, known to be important for immune responses to antigen, are expressed also by neurons that undergo activity-dependent, long-term structural and synaptic modifications. Here, we show that in mice genetically deficient for cell surface class I MHC or for a class I MHC receptor component, CD3zeta, refinement of connections between retina and central targets during development is incomplete. In the hippocampus of adult mutants, N-methyl-D aspartate receptor-dependent long-term potentiation (LTP) is enhanced, and long-term depression (LTD) is absent. Specific class I MHC messenger RNAs are expressed by distinct mosaics of neurons, reflecting a potential for diverse neuronal functions. These results demonstrate an important role for these molecules in the activity dependent remodeling and plasticity of connections in the developing and mature mammalian central nervous system (CNS)..

### PMID: 11118151

**Panel 4D Summary:** Ag3303 The CG57668-01 gene is moderately expressed in a number of samples on this panel, with highest expression in lymphokine-activated killer (LAK) cells (CT = 26-28), independent of stimulation. This observation suggests that modulation of the expression or activity of this gene may be useful in the treatment of intracellular bacterial or viral infections. This gene is also expressed at significant levels in T cells, B cells, dendritic cells, monocytes and macrophages.

The CG57668-01 gene encodes a protein with homology to MHC class I antigen. Major histocompatibility complex (MHC) class I molecules present antigenic peptides to CD8-positive T cells. The HLA class I gene family in humans consists of 6 members: polymorphic HLA-A, HLA-B, and HLA-C, and oligomorphic HLA-E, HLA-F, and HLA-G. HLA-A/B-alleles such as the CG57664-01 gene may have utility in genotyping criminals as well as in paternity disputes. These antigens are ubiquitously expressed on all nucleated human cells except neurons and trophoblasts, and participate in antigen presentation of viral antigens in the adaptive immune response.

### NOV33

Expression of NOV33/CG57672-01 was assessed using the primer-probe set Ag3305, described in Table AAA. Results of the RTQ-PCR runs are shown in Tables AAB, AAC, and AAD.

**Table AAA. Probe Name Ag3305**

| **Primers** | **Sequences** | **Length** | **Start Position** | **SEQ ID NO** |
|---|---|---|---|---|
| Forward | 5'-aaaagtttcggtctggcttatc-3' | 22 | 1354 | 356 |
| Probe | TET-5'-tttctctctgcatacgggctccagtg-3'-TAMRA | 26 | 1376 | 357 |
| Reverse | 5'-gaccaatggctggtaagtaatg-3' | 22 | 1412 | 358 |

**Table AAB. Generat_sereening_panet_v1.4**

| **Tissue Name** | **Rel. Exp.(%) Ag3305, Run 215648474** | **Tissue Name** | **Rel. Exp.(%) Ag3305, Run 215648474** |
|---|---|---|---|
| Adipose | 0.0 | Renal ca. TK-10 | 1.3 |
| Melanoma* Hs688(A).T | 0.0 | Bladder | 0.0 |
| Melanoma* Hs688(B).T | 0.0 | Gastric ca. (liver met.) NCI-N87 | 0.0 |
| Melanoma* M14 | 0.0 | Gastric ca. KATO III | 2.4 |
| Melanoma* LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca* (SW480 met) SW620 | 0.5 |
| Testis Pool | 100.0 | Colon ca. HT29 | 1.6 |
| PC-3 Prostate ca.* (bone met) PC-3 | 0.0 | Colon ca. HCT-116 | 4.8 |
| Prostate Pool | 0.0 | Colon ca. CaCo-2 | 1.8 |
| Placenta | 0.0 | Colon cancer tissue | 2.8 |
| Uterus Pool | 0.0 | Colon ca.SW1116 | 0.0 |
| Ovarian ca. OVCAR-3 | 0.0 | Colon ca. Colo-205 | 0.0 |
| Ovarian ca. SK-OV-3 | 4.4 | Colon ca. SW-48 | 0.0 |
| Ovarian ca. OVCAR-4 | 0.0 | Colon Pool | 1.4 |
| Ovarian ca. OVCAR-5 | 0.0 | Small Intestine Pool | 0.8 |
| Ovarian ca. IGROV-1 | 9.2 | Stomach Pool | 1.3 |
| Ovarian ca. OVCAR-8 | 0.0 | Bone Marrow Pool | 2.6 |
| Ovary | 0.5 | Fetal Heart | 0.0 |
| Breast ca. MCF-7 | 0.6 | Heart Pool | 2.8 |
| Breast ca. MDA-MB-231 | 2.3 | Lymph Node Pool | 1.8 |
| Breast ca. BT 549 | 0.9 | Fetal Skeletal Muscle | 0.0 |
| Breast ca. T47D | 1.2 | Skeletal Muscle Pool | 0.0 |
| Breast ca. MDA-N | 1.6 | Spleen Pool | 0.5 |
| Breast Pool | 2.8 | Thymus Pool | 1.8 |
| Trachea | 0.0 | CNS cancer (glio/astro) U87-MG | 0.0 |
| Lung | 0.0 | CNS cancer (glio/astro) U-118-MG | 1.1 |
| Fetal Lung | 0.6 | CNS cancer (neuro;met) SK-N-AS | 0.0 |
| Lung ca. NCI-N417 | 0.5 | CNS cancer (astro) SF-539 | 0.0 |
| Lung ca. LX-1 | 1.8 | CNS cancer (astro) SNB-75 | 2.1 |
| Lung ca. NCl-H146 | 0.0 | CNS cancer (glio) SNB-19 | 2.5 |
| Lung ca. SHP-77 | 0.0 | CNS cancer (glio) SF-295 | 2.1 |
| Lung ca. A549 | 0.0 | Brain (Amygdala) Pool | 0.0 |
| Lung ca. NCI-H526 | 0.0 | Brain (cerebellum) | 0.0 |
| Lung ca. NCI-H23 | 3.8 | Brain (fetal) | 0.0 |
| Lungca.NCI-H460 | 0.0 | Brain (Hippocampus) Pool | 1.4 |
| Lung ca. HOP-62 | 0.0 | Cerebral Cortex Pool | 1.0 |
| Lung ca. NCI-H522 | 0.0 | Brain (Substantia nigra)Pool | 1.4 |
| Liver | 0.0 | Brain (Thalamus) Pool | 1.1 |
| Fetal Liver | 0.0 | Brain(whole) | 0.0 |
| Liver ca. HepG2 | 4.6 | Spinal Cord Pool | 0.0 |
| Kidney Pool | 13.3 | Adrenal Gland | 0.0 |
| Fetal Kidney | 0.6 | Pituitary gland Pool | 0.0 |
| Renal ca. 786-0 | 1.1 | Salivary Gland | 0.0 |
| Renal ca. A498 | 0.0 | Thyroid (female) | 0.0 |
| Renal ca. ACHN | 1.0 | Pancreatic ca. CAPAN2 | 0.0 |
| Renal ca. UO-31 | 0.0 | Pancreas Pool | 9.9 |

**Table AAC. Panel 4D**

| **Tissue Name** | **Rel. Exp.(%) A.g3305, Run 164682643** | **Tissue Name** | **Rel.Exp.(%) Ag3305, Run 164682643** |
|---|---|---|---|
| SecondaryTh1 act | 11.7 | HUVEC IL-1beta | 4.2 |
| Secondary Th2 act | 12.2 | HUVEC IFN gamma | 29.9 |
| Secondary Trt1 act | 9.2 | HUVEC TNF alpha + IFN gamma | 6.4 |
| Secondary Th1 rest | 10.0 | HUVEC TNF alpha + IL4 | 7.3 |
| Secondary Th2 rest | 3.3 | HUVEC IL-1 | 7.9 |
| Secondary Tr1 rest | 27.5 | Lung Microvascular EC none | 19.6 |
| Primary Th1 act | 10.9 | Lung Microvascular EC TNF alpha + IL-1beta | 32.8 |
| Primary Th2 act | 14.6 | Microvascular Dermal EC none | 23.2 |
| Primary Tr1 act | 8.7 | Microsvasular Dermal EC TNF alpha+IL-1beta | 13.3 |
| PrimaryTh1 rest | 20.2 | Bronchial epithelium TNFalpha + IL1beta | 12.0 |
| Primary Th2 rest | 11.3 | Small airway epithelium none | 0.0 |
| Primary Trl rest | 17.6 | Small airway epithelium TNFalpha + IL-lbeta | 2.4 |
| CD45RA CD4 lymphocyte act | 5.1 | Coronery artery SMC rest | 0.0 |
| CD45RO CD4 lymphocyte act | 20.0 | Coronery artery SMC TNFalpha + IL-1beta | 0.0 |
| CD8 lymphocyte act | 9.5 | Astrocytes rest | 1.7 |
| Secondary CD8 lymphocyte rest | 19.2 | Astrocytes TNFalpha+ IL-1beta | 0.0 |
| Secondary CD8 Lymphocyte act | 2.4 | KU-812 (Basophil) rest | 24.7 |
| CD4 lymphocyte none | 5.1 | KU-812 (Basophil) PMA/ionomycin | 29.1 |
| 2ry Th1/Th2/Tr1_anti-CD95 CH11 | 18.4 | CCD1106 (Keratinocytes) none | 18.9 |
| LAK cells rest | 24.7 | CCD1106 (Keratinocytes) TNFalpha+IL-1beta | 3.4 |
| LAK cells IL-2 | 24.3 | Liver cirrhosis | 3.0 |
| LAK cells IL-2+IL-12 | 11.2 | Lupus kidney | 1.8 |
| LAK cells IL-2+IFN gamma | 45.7 | NCI-H292 none | 18.6 |
| LAK cells IL-2+IL-18 | 13.3 | NCI-H292 IL-4 | 34.4 |
| LAK cells PMA/ionomycin | 1.3 | NCI-H292 IL-9 | 2.5 |
| NK Cells IL-2 rest | 3.0 | NCI-H292 IL-13 | 1.3 |
| Two Way MLR 3 day | 27.9 | NCI-H292 IFN gamma | 0.8 |
| Two Way MLR 5 day | 8.0 | HPAEC none | 12.5 |
| Two Way MLR 7 day | 0.0 | HPAEC TNF alpha + IL-1 beta | 10.0 |
| PBMC rest | 11.7 | Lung fibroblast none | 5.4 |
| PBMC PWM | 19.1 | Lung fibroblast TNF alpha + IL-1 beta | 2.6 |
| PBMC PHA-L | 2.8 | Lung fibroblast IL-4 | 16.2 |
| Ramos (B cell) none | 73.2 | Lung fibroblast IL-9 | 4.1 |
| Ramos (B cell) ionomycin | 100.0 | Lung fibroblast IL-13 | 3.5 |
| B lymphocytes PWM | 6.6 | Lung fibroblast IFN gamma | 15.5 |
| B lymphocytes CD40L and IL-4 | 44.8 | Dermal fibroblast CCD1070 rest | 35.1 |
| EOL-1 dbcAMP | 4.2 | Dermal fibroblast CCD1070 TNF alpha | 27.4 |
| EOL-1 dbcAMP PMA/ionomycin | 0.0 | Dermal fibroblast CCD1070 IL-1 beta | 1.7 |
| Dendritic cells none | 3.7 | Dermal fibroblast IFN gamma | 3.0 |
| Dendritic cells LPS | 14.2 | Dermal fibrobtast IL-4 | 3.3 |
| Dendritic cells anti-CD40 | 11.8 | IBD Colitis 2 | 0.5 |
| Monocytes rest | 0.0 | IBD Crohn's | 0.0 |
| Monocytes LPS | 8.1 | Colon | 1.2 |
| Macrophages rest | 21.3 | Lung | 0.0 |
| Macrophages LPS | 23.7 | Thymus | 5.9 |
| HUVEC none | 27.9 | Kidney | 18.6 |
| HUVEC starved | 39.2 | | |

**Table AAD. Panel 5 Islet**

| **Tissue Name** | **Rel. Exp.(%) Ag3305, Run 242322479** | **Tissue Name** | **Rel. Exp.(%) Ag3305, Run 242322479** |
|---|---|---|---|
| 97457_Patient-02go_adipose | 0.0 | 94709_Donor 2 AM-A_adipose | 13.0 |
| 97476_Patient-07sk_skeletal muscle | 0.0 | 94710_Donor 2 AM-B adipose | 0.0 |
| 97477_Patient-07ut_uterus | 21.6 | 94711_Donor 2 AM-C_adipose | 100.0 |
| 97478_Patient-07pt_placenta | 0.0 | 94712_Donor2AD-A_adipose | 26.8 |
| 99167_Bayer Patient 1 | 0.0 | 94713_Donor 2 AD-B_adipose | 0.0 |
| 97482_Patient-08ut_uterus | 0.0 | 94714_Donor 2 AD-C_adipose | 0.0 |
| 97483_Patient-08pl_placenta | 23.7 | 94742_Donor 3U - A_Mesenchymal Stem Cells | 21.3 |
| 97486_Patient-09sk_skeletal muscle | 0.0 | 94743_Donor 3 U - B_Mesenchymal Stem Cells | 0.0 |
| 97487_Patient-09ut_uterus | 3.4 | 94730_Donor 3 AM - A_adipose | 42.0 |
| 97488_Patient-09pl_placenta | 7.9 | 94731_Donor 3 AM-B_adipose | 0.0 |
| 97492_Patient-10ut_uterus | 0.0 | 94732_Donor 3 AM-C_adipose | 17.4 |
| 97493_Patient-10pl_placenta | 0.0 | 94733_Donor 3 AD-A_adipose | 0.0 |
| 97495_Patient-11go_adipose | 11.7 | 94734_Donor 3 AD-B_adipose | 0.0 |
| 97496_Patient-11sk_skeletal muscle | 8.5 | 94735_Donor 3 AD - C_adipose | 0.0 |
| 97497_Patient-11ut_uterus | 5.2 | 77138_Liver_HepG2untreated | 84.1 |
| 97498_Patient-11pl_placenta | 0.0 | 73556_Heart_Cardiac stromal cells (primary) | 0.0 |
| 97500_Patient-12go_adipose | 17.9 | 81735_Small Intestine | 12.7 |
| 97501_Patient-12sk_skeletal muscle | 15.1 | 72409_Kidney_Proximal Convoluted Tubule | 0.0 |
| 97502_Patient-12ut_uterus | 8.0 | 82685_Small intestine_Duodenum | 0.0 |
| 97503 Patient-12pl_placenta | 0.0 | 90650 Adrenal Adrenocortical adenoma | 0.0 |
| 94721 Donor 2 U - A_Mesenchymal Stem Cells | 0.0 | 72410_Kidney_HRCE | 12.9 |
| 94722_Donor 2 U - B_Mesenchymal Stem Cells | 8.7 | 72411_Kidney_HRE | 59.5 |
| 94723_Donor 2 U - C_Mesenchymal Stem Cells | 13.0 | 73139_Uterus_Uterine smooth muscle cells | 0.0 |

**CNS_neurodegeneration_v1.0 Summary:** Ag3305 Results from one experiment with the CG57672-01 gene are not included because signal was high in the water-only control well (data not shown).

**General_screening_panel_v1.4 Summary:** Ag3305 Significant expression of the CG57672-01 gene is limited to testis (CT = 32.2). Thus, expression of this gene could be used to distinguish testis from the other samples on this panel. Furthermore, therapeutic modulation of the activity of this gene or its protein product, using small molecule drugs, antibodies or protein therapeutics, may be of use in the treatment of reproductive disorders such as infertility.

**Panel 4D Summary:** Ag3305 The CG57672-01 gene is expressed at low levels in many of the samples on this panel, with highest expression in ionomycin-treated Ramos B cells (CT = 31). This gene is expressed at low levels in members of the T-cell, B-cell, basophil, macrophage/dendritic cell, and peripheral blood mononuclear cell family, as well as in lung and dermal fibroblasts and microvascular endothelial cells. This ubiquitous pattern of expression suggests that this gene product may be involved in homeostatic processes for these and other cell types and tissues.

Therefore, therapeutic modulation of the activity of this gene or its protein product may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

**Panel 5 Islet Summary:** Ag3305 The CG57672-01 gene is expressed at low levels in several samples of adipocytes cultured in vitro from mesenchymal stem cells. Therefore, this gene product may be involved in adipocyte differentiation and its therapeutic modulation may be a treatment for obesity and obesity-related Type 2 diabetes.

### NOV34

Expression of NOV34/CG57680-O1 was assessed using the primer-probe set Ag3307, described in Table ABA. Results of the RTQ-PCR runs are shown in Tables ABB, ABC and ABD.

**Table ABA. Probe Name Ag3307**

| **Primers** | **Sequences** | **Length** | **Start Position** | **SEQ ID NO** |
|---|---|---|---|---|
| Forward | 5'-ccagaacctacaagggaacaat-3' | 22 | 582 | 359 |
| Probe | TET-5'-cacaaatgaagaaattgatcatttcaaacg-3'-TAMRA | 30 | 623 | 360 |
| Reverse | 5'-ttctacttcttcggctgaactt-3' | 22 | 653 | 361 |

**Table ABB. CNS_neurodegeneration_v1.0**

| **Tissue Name** | **Rel. Exp.(%) Ag3307, Run 210138283** | **Tissue Name** | **Rel. Exp.(%) Ag3307, Run 210138283** |
|---|---|---|---|
| AD 1 Hippo | 11.6 | Control (Path) 3 Temporal Ctx | 23.2 |
| AD 2 Hippo | 10.4 | Control (Path) 4 Temporal Ctx | 36.6 |
| AD 3 Hippo | 13.8 | AD 1 Occipital Ctx | 44.4 |
| AD 4 Hippo | 7.9 | AD 2 Occipital Ctx (Missing) | 0.0 |
| AD 5 hippo | 100.0 | AD 3 Occipital Ctx | 30.8 |
| AD 6 Hippo | 18.7 | AD 4 Occipital Ctx | 12.5 |
| Control 2 Hippo | 9.6 | AD 5 Occipital Ctx | 34.6 |
| Control 4 Hippo | 11.6 | AD 6 Occipital Ctx | 13.7 |
| Control (Path) 3 Hippo | 15.7 | Control 1 Occipital Ctx | 10.0 |
| AD 1 Temporal Ctx | 22.4 | Control 2 Occipital Ctx | 14.0 |
| AD 2 Temporal Ctx | 23.8 | Control 3 Occipital Ctx | 40.6 |
| AD 3 Temporal Ctx | 19.5 | Control 4 Occipital Ctx | 13.0 |
| AD 4 Temporal Ctx | 19.5 | Control (Path) 1 Occipital | 36.1 |
| AD 5 Inf Temporal Ctx | 85.3 | Control (Path) 2 Occipital Ctx | 36.3 |
| AD 5 SupTemporal Ctx | 34.6 | Control (Path) 3 Occipital Ctx | 11.7 |
| AD 6 Inf Temporal Ctx | 36.1 | Control (Path) 4 Occipital Ctx | 52.9 |
| AD 6 Sup Temporal Ctx | 44.1 | Control 1 Parietal Ctx | 17.2 |
| Control 1 Temporal Ctx | 16.5 | Control 2 Parietal Ctx | 63.7 |
| Control 2 Temporal Ctx | 13.1 | Control 3 Parietal Ctx | 22.4 |
| Control3 Temporal Ctx | 18.3 | Control (Path) 1 Parietal , Ctx | 32.5 |
| Control 4 Temporal Ctx | 22.1 | Control (Path) 2 Parietal Ctx | 28.5 |
| Control (Path) 1 Temporal | 29.7 | Control (Path) 3 Parietal Ctx | Ctx |
| Control (Path) 2 Temporal Ctx | 25.9 | Control (Path) 4 Parietal Ctx | 49.7 |

**Table ABC.General_screening_panel_v1.4**

| **Tissue Name** | **Rel. Exp.(%) Ag3307, Run 215648237** | **Tissue Name** | **Rel. Exp.(%) Ag3307, Run 215648237** |
|---|---|---|---|
| Adipose | 1.1 | Renal ca.TK-10 | 1.8 |
| Melanoma*Hs688(A).T | 0.2 | Bladder | 4.0 |
| Melanoma* Hs688(B).T | 0.5 | Gastric ca. (liver met.) NCI-N87 | 1.4 |
| Melanoma* M14 | 0.6 | Gastric ca. KATO III | 1.6 |
| Melanoma* LOXIMVI | 0.6 | Colon ca. SW-948 | 0.5 |
| Melanoma* SK-MEL-5 | 1.4 | Colon ca. SW480 | 1.9 |
| Squamous cell carcinoma SCC-4 | 0.9 | Colon ca.* (SW480 met) SW620 | 1.3 |
| Testis Pool | 1.8 | Colon ca. HT29 | 1.3 |
| Prostate ca.* (bone met) PC-3 | 1.2 | Colon ca. HCT-116 | 1.3 |
| Prostate Pool | 2.2 | Colon ca. CaCo-2 | 2.7 |
| Placenta | 3.3 | Colon cancer tissue | 1.8 |
| Uterus Pool | 0.4 | Colon ca.SW1116 | 1.8 |
| Ovarian ca. OVCAR-3 | 1.0 | Colon ca. Colo-205 | 0.0 |
| Ovarian ca. SK-OV-3 | 0.9 | Colon ca. SW-48 | 0.4 |
| Ovarian ca.OVCAR-4 | 0.1 | Colon Pool | 100.0 |
| Ovarian ca. OVCAR-5 | 4.2 | Small Intestine Pool | 3.0 |
| Ovarian ca. IGROV-1 | 5.2 | Stomach Pool | 4.3 |
| Ovarian ca. OVCAR-8 | 1.1 | Bone Marrow Pool | 1.8 |
| Ovary | 1.2 | Fetal Heart | 3.5 |
| Breast ca. MCF-7 | 3.4 | Heart Pool | 1.1 |
| Breast ca. MDA-MB-231 | 3.2 | Lymph Node Pool | 2.9 |
| Breast ca. BT 549 | 3.6 | Fetal Skeletal Muscle | 2.6 |
| Breast ca. T47D | 7.8 | Skeletal Muscle Pool | 1.3 |
| Breast ca.MDA-N | 0.9 | Spleen Pool | 13.8 |
| Breast Pool | 4.2 | Thymus Pool | 16.7 |
| Trachea | 3.9 | CNS cancer (glio/astro) U87-MG | 2.0 |
| Lung | 3.5 | CNS cancer (glio/astro) U-118-MG | 6.4 |
| Fetal Lung | 40.9 | CNS cancer (neuro;met) SK-N-AS | 3.7 |
| Lung ca. NCI-N417 | 3.2 | CNS cancer (astro) SF-539 | 0.3 |
| Lung ca. LX-1 | 1.1 | CNS cancer (astro) SNB-75 | 2.5 |
| Lung ca.NCI-H146 | 1.5 | CNS cancer (glio) SNB-19 | 4.0 |
| Lung ca. SHP-77 | 4.8 | CNS cancer (glio) SF-295 | 3.2 |
| Lung ca. A549 | 0.0 | Brain (Amygdala) Pool | 1.8 |
| Lung ca., NCI-H526 | 0.6 | Brain (cerebellum) | 2.8 |
| Lung ca. NCI-H23 | 1.1 | Brain (fetal) | 5.2 |
| Lung ca. NCI-H460 | 5.3 | Brain (Hippocampus) Pool | 2.1 |
| Lung ca. HOP-62 | 1.6 | Cerebral Cortex Pool | 2.7 |
| Lungca.NCI-H522 | 1.2 | Brain(Substantia nigra) Pool | 2.3 |
| Liver | 0.0 | Brain (Thalamus) Pool | 4.2 |
| Fetal Liver | 1.1 | Brain (whole) | 1.1 |
| Liver ca. HepG2 | 0.1 | Spinal Cord Pool | 1.7 |
| KidneyPool | 4.4 | Adrenal Gland | 0.7 |
| Fetal Kidney | 9.8 | Pituitary gland Pool | 0.8 |
| Renal ca. 786-0 | 3.0 | Salivary Gland | 0.5 |
| Renal ca. A498 | 1.2 | (female) | 1.8 |
| Renal ca. ACHN | 0.8 | Pancreatic ca. CAPAN2 | 2.6 |
| Renal ca. UO-31 | 0.3 | Pancreas Pool | 3.4 |

**Table ABD. Panel 4D**

| **Tissue Name** | **Rel. Exp.(%) Ag3307, Run 164335286** | **Tissue Name** | **Rel. Exp.(%) Ag3307, Run 164335286** |
|---|---|---|---|
| SecondaryTh1 act | 3.1 | HUVEC IL-1beta | 0.8 |
| Secondary Th2 act | 4.7 | HUVEC IFN gamma | 3.5 |
| Secondary Tr1 act | 6.1 | HUYEC TNF alpha+IFNgamma | 2.2 |
| SecondaryTh1 rest | 6.7 | HUVEC TNF alpha+IL4 | 0.6 |
| Secondaly Th2 rest | 5.0 | HUVEC IL-11 | 0.8 |
| Secondary Tr1 rest | 7.4 | Lung Microvascular EC none | 0.4 |
| Primary Th1 act | 4.6 | Lung Microvascular EC TNFalpha IL-1beta | 0.2 |
| Primary Th2 act | 6.0 | Microvascular Dermal EC none | 0.7 |
| Primary Tr1 act | 9.9 | Microsvasular Dermal EC TNFalpha + IL-1beta | 0.0 |
| Primary Th1 rest | 37.6 | Bronchial epithelium TNFalpha + IL1beta | 1.3 |
| Primary Th2 rest | 24.5 | Small airway epithelium none | 0.7 |
| Primary Tr1 rest | 13.5 | Small airway epithelium TNFalpha + IL-1beta | 3.3 |
| CD45RA CD4 lymphocyte act | 2.5 | Coronery artery SMC rest | 0.3 |
| CD45RO CD4 lymphocyte act | 8.6 | Coronery artery SMC TNFalpha + IL-1beta | 0.4 |
| CD8 lymphocyte act | 4.6 | Astcocytesr est | 1.1 |
| Secondary CD8 lymphocyte rest | 3.3 | Astrocytes TNFalpha + IL-1beta | 1.8 |
| Secondary CD8 lymphocyte act | 8.0 | KU-812 (Basophil) rest | 3.8 |
| CD4 lymphocyte none | 6.3 | KU-812 (Basophil) PMA/ionomycin | 8.7 |
| 2ry Th1/Th2/Tr1_anti-CD95 CH11 | 14.9 | CCD1106 (Keratinocytes) none | 1.2 |
| LAK cells rest | 5.6 | CCD1106(Keratinocytes) TNFalpha +IL-1 beta | 0.0 |
| LAK cells IL-2 | 20.4 | Liver cirrhosis | 1.8 |
| LAK cells IL-2+IL-12 | 6.0 | Lupus kidney | 1.5 |
| LAK cells IL-2+IFN gamma | 9.8 | NCI-H292 none | 15.2 |
| LAK cells IL-2+IL-18 | 10.3 | NCI-H292 IL-4 | 7.8 |
| LAK cells PMA/ionomycin | 1.7 | NCI-H292 IL-9 | 18.2 |
| NK Cells IL-2 rest | 14.2 | NCI-H292 IL-13 | 7.0 |
| Two Way MLR 3 day | 14.9 | NCI-H292 IFN gamma | 8.5 |
| Two Way MLR 5 day | 3.0 | HPAEC none | 1.7 |
| Two Way MLR 7 day | 8.1 | HPAEC TNF alpha + IL-1 beta | 0.3 |
| PBMC rest | 5.1 | Lung fibroblast none | 2.6 |
| PBMC PWM | 13.8 | Lung fibroblast TFN alpha + IL-1 beta | 2.2 |
| PBMC PHA-L | 6.7 | Lung fibroblast IL-4 | 4.0 |
| Ramos (B cell) none | 10.7 | Lung fibroblast IL-9 | 2.8 |
| Ramos (B cell) ionomycin | 24.0 | Lung fibroblast IL-13 | 2.5 |
| B lymphocytes PWM | 24.1 | Lung fibroblast IFN gamma | 3.7 |
| B lymphocytes CD40L and IL-4 | 54.3 | Dermal fibroblast CCD1070 rest | 3.5 |
| EOL-1 dbcAMP | 1.7 | Dermal fibroblast CCD1070 TNF alpha | 18.8 |
| EOL-1 dbcAMP PMA/ionomycin | 1.8 | Dermal fibroblast CCD1070 IL-1 beta | 0.8 |
| Dendritic cells none | 1.4 | Dermal fibroblast IFN gamma | 1.4 |
| Dendritic cells LPS | 0.4 | Dermal fibroblast IL-4 | 1.0 |
| Dendritic cells anti-CD40 | 0.5 | IBD Colitis 2 | 3.3 |
| Monocytesrest | 1.1 | IBD Crohn's | 2.2 |
| Monocytes LPS | 0.7 | Colon | 7.2 |
| Macrophages rest | 2.8 | Lung | 1.6 |
| Macrophages LPS | 0.3 | Thymus | 8.8 |
| HUVEC none | 1.7 | Kidney | 100.0 |
| HUVEC starved | 4.5 | | |

CNS_neurodegeneration_v1.0 Summary: AP-3307 This panel confirms the expression of this gene at low to moderate levels in the brain in an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel-v1.4 Summary:** Ag3307 The CG57680-01 gene is expressed at low levels in many of the tissues on this panel, with the highest expression in a normal colon sample (CT = 28.8). Significant expression of this gene is also detected in fetal lung (CT = 30.1). Interestingly, this gene is expressed at much lower levels in adult lung (CT = 33.6), suggesting that expression of this gene can be used to distinguish fetal lung from adult lung.

This gene is also expressed at low levels in many regions of the central nervous system, including amygdala, substantia nigra, thalamus, hippocampus, cerebellum, cerebral cortex, and spinal cord. The CG57680-01 gene encodes a protein with homology to cyclophilin-type peptidyl-prolyl cis-trans isomerase. Cyclophilin is a specific high-affinity binding protein for the immunosuppressant agent cyclosporin A. Neuroimmunophilin ligands, such as cyclosporin A, are a class of compounds that hold great promise for the treatment of nerve injuries and neurological disease. Therefore, modulation of the activity of the CG57680-01 gene or its protein product may be of use in the treatment of neurodegenerative disorders such as Alzheimer's disease and Parkinson's disease.

Among tissues with metabolic or endocrine function, this gene is expressed at low levels in thyroid, pancreas, fetal heart, and fetal skeletal muscle. Therefore, therapeutic modulation of the activity of this gene may prove useful in the treatment of endocrine/metabolically related diseases, such as obesity and diabetes.

### References:

### 1. Gold BG. Neuroimmunophilin ligands: evaluation of their therapeutic potential for the treatment of neurological disorders. Expert Opin Investig Drugs 2000 Oct;9(10):2331-42

Neuroimmunophilin ligands are a class of compounds that hold great promise for the treatment of nerve injuries and neurological disease. In contrast to neurotrophins (e.g., nerve growth factor), these compounds readily cross the blood-brain barrier, being orally effective in a variety of animal models of ischaemia, traumatic nerve injury and human neurodegenerative disorders. A further distinction is that neuroimmunophilin ligands act via unique receptors that are unrelated to the classical neurotrophic receptors e.g., trk), making it unlikely that clinical trials will encounter the same difficulties found with the neurotrophins. Another advantage is that two neuroimmunophilin ligands (cyclosporin A and FK-506) have already been used in humans (as immunosuppressant drugs). Whereas both cyclosporin A and FK-506 demonstrate neuroprotective actions, only FK-506 and its derivatives have been clearly shown to exhibit significant neuroregenerative activity. Accordingly, the neuroprotective and neuroregenerative properties seem to arise via different mechanisms. Furthermore, the neuroregenerative property does not involve calcineurin inhibition (essential for immunosuppression). This is important since most of the limiting side effects produced by these drugs arise via calcineurin inhibition. A major breakthrough for the development of this class of compounds for the treatment of human neurological disorders was the ability to separate the neuroregenerative property of FK-506 from its immunosuppressant action via the development of non-immunosuppressant (non-calcineurin inhibiting) derivatives. Further studies revealed that different receptor subtypes, or FK-506-binding proteins (FKBPs), mediate immunosuppression and nerve regeneration (FKBP-12 and FKBP-52, respectively, the latter being a component of steroid receptor complexes). Thus, steroid receptor chaperone proteins represent novel targets for future drug development of novel classes of compounds for the treatment of a variety of human neurological disorders, including traumatic injury (e.g., peripheral nerve and spinal cord), chemical exposure (e.g., vinca alkaloids, Taxol) and neurodegenerative disease (e.g., diabetic neuropathy and Parkinson's disease).

### PMID: 11060810

**Panel 4D Summary:** Ag3307 Expression of the CG57680-01 gene is highest in kidney (CT = 29.2). This gene is also expressed at moderate to low levels in numerous cell types, but is expressed at higher levels (CTs 20-32) in T cells and B cells. Therefore, small molecule antagonists that block the function of the CG57680-01 protein may be useful to reduce or eliminate the symptoms of Crohn's disease, ulcerative colitis, multiple sclerosis, chronic obstructive pulmonary disease, asthma, emphysema, rheumatoid arthritis, lupus erythematosus, or psoriasis.

### NOV35

Expression of NOV35/CG57670-01 was assessed using the primer-probe set Ag3304, described in Table ACA. Results of the RTQ-PCR runs are shown in Tables ACB, ACC, ACD, ACE and ACF.

**Table ACA. Probe Name Ag3304**

| **Primers** | **Sequence** | **Length** | **Start Position** | **SEQ ID NO** |
|---|---|---|---|---|
| Forward | 5'-aaagctttgcttctgactccat-3' | 22 | 29.7 | 362 |
| Probe | TET-5'-ctctaccagcccattgctgtggct-3'-TAMPA | 24 | 320 | 363 |
| Reverse | 5'-ttgggatctcaggtcctttagt-3' | 22 | 350 | 364 |

**Table ACB. CNS_neurodegeneration_v1.0**

| **Tissue Name** | **Rel. Exp.(%) Ag3304, Run 210063833** | **Tissue Name** | **Rel. Exp.(%) Ag3304, Run 210063833** |
|---|---|---|---|
| AD 1 Hippo | 2.0 | Control (Path) 3 Temporal | 1.2 |
| AD 2 Hippo | 22.2 | Control (Path) 4 Temporal Ctx | 14.9 |
| AD 3 Hippo | 3.2 | AD 1 Occipital Ctx | 17.3 |
| AD 4 Hippo | 24.1 | AD 2 Occipital Ctx (Missing) | 0.0 |
| AD 5 Hippo | 45.4 | AD 3 Occipital Ctx | 7.5 |
| AD 6 Hippo | 70.2 | AD 4 Occipital Ctx | 48.3 |
| Control 2 Hippo | 51.8 | AD 5 Occipital Ctx | 9.0 |
| Control 4 Hippo | 18.2 | AD 6 Occipital Ctx | 15.7 |
| Control (Path) 3 Hippo | 14.8 | Control 1 Occipital Ctx | 3.1 |
| AD 1 Temporal Ctx | 8.0 | Control 2 Occipital Ctx | 50.0 |
| AD 2 Temporal Ctx | 85.3 | Control 3 Occipital Cbc | 24.0 |
| AD 3 Temporal Ctx | 4.1 | Control 4 Occipital Ctx | 11.7 |
| AD 4 Temporal Ctx | 23.3 | Control (Path) 1 Occipital Ctx | 57.0 |
| AD 5 Inf Temporal Ctx | 11.3 | Control (Path) 2 Occipital Ctx | 21.5 |
| AD 5 Sup Temporal Ctx | 49.7 | Control (Path) 3 Occipital | 5.5 |
| AD 6 Inf Temporal Ctx | 31.6 | Control (Path) 4 Occipital Ctx | 12.2 |
| AD 6 Sup Temporal Ctx | 29.3 | Control 1 Parietal Ctx | 17.2 |
| Control 1 Temporal Ctx | 16.6 | Control 2 Parietal Ctx | 13.8 |
| Control 2 Temporal Ctx | 46.0 | Control 3 Parietal Ctx | 16.3 |
| Control 3 Temporal Ctx | 41.8 | Control (Path) 1 Parietal | 70.2 |
| Control 3 Temporal Ctx | 3.6 | Control (Path) 2 Parietal Ctx | 21.3 |
| Control (Path) 1 Temporal Ctx | 100.0 | Control (Path) 3 Parietal Ctx | 5.4 |
| Control (Path) 2 Temporal Ctx | 89.5 | Control (Path) 4 Parietal Ctx | 15.0 |

**Table ACC. General_screening_panel v1.4**

| **Tissue Name** | **Rel. Exp.(%) Ag3304, Run 215648468** | **Tissue Name** | **Rel. Exp.(%) Ag3304, Run 215648468** |
|---|---|---|---|
| Adipose | 0.0 | Renal ca. TK-10 | 0.0 |
| Melanoma* Hs688(A).T | 0.0 | Bladder | 0.0 |
| Melanoma* Hs688(B).T | 0.0 | Gastric ca. (liver met.) NCI- N87 | 0.0 |
| Melanoma* M14 | 0.0 | Gastric ca. KATO III | 0.0 |
| Melanoma* LOXIMVI | 0.0 | Colon ca. SW-948 | 100.0 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| Testis Pool | 0.0 | Colon ca. HT29 | 0.0 |
| Prostate ca.* (bone met) PC-3 | 0.0 | Colon ca. HCT-116 Colon HCT-116 | 0.0 |
| Prostate Pool | 0.0 | Colon ca. CaCo-2 | 0.0 |
| Placenta | 0.0 | Colon cancer tissue | 0.0 |
| Uterus Pool | 0.0 | Colon ca. SW1116 | 0.0 |
| Ovarian ca. OVCAR-3 | 0.0 | Colon ca.Colo-205 | 0.0 |
| Ovarian ca. SK-OV-3 | 0.0 | Colon ca. SW-48 | 0.0 |
| Ovarian ca. OVCAR-4 | 0.0 | Colon Pool | 0.0 |
| Ovarian ca. OVCAR-5 | 0.0 | Small Intestine Pool | 0.0 |
| Ovarian ca. IGROV-1 | 0.0 | Stomach Pool | 0.0 |
| Ovarian ca. OVCAR-8 | 0.0 | Bone Marrow Pool | 0.0 |
| Ovary | 0.0 | Fetal Heart | 0.0 |
| Breast ca.MCF-7 | 0.0 | Heart Pool | 0.0 |
| Breast ca. MDA-MB-231 | 0.0 | Lymph Node Pool | 0.0 |
| Breast ca. BT 549 | 0.0 | Fetal Skeletal Muscle | 0.0 |
| Breast ca. T47D | 0.0 | Skeletal Muscle Pool | 0.0 |
| Breast ca. MDA-N | 0.0 | Spleen Pool | 0.0 |
| Breast Pool | 0.0 | Thymus Pool | 0.0 |
| Trachea | 0.0 | CNS cancer (glio/astro) U87-MG | 0.0 |
| Lung | 0.0 | CNS cancer (glio/astro) U-118-MG | 0.0 |
| Fetal Lung | 0.0 | CNS cancer (neuro;met) SK-N-AS | 0.0 |
| Lungca.NCL.N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 0.0 | CNS cancer (glio) SNB-19 | 0.0 |
| Lung ca. SHP-77 | 0.0 | CNS cancer (glio)SF-295 | 0.0 |
| Lung ca. A549 | 0.0 | Brain (Amygdala) Pool | 0.0 |
| Lung ca. NCI-H526 | 0.0 | Brain (cerebellum) | 0.0 |
| Lung ca. NCI-H23 | 0.0 | Brain (fetal) | 0.0 |
| Lung ca. NCI-H460 | 0.0 | Brain (Hippocampus) Pool | 0.0 |
| Lung ca. HOP-62 | 0.0 | Cerebral Cortex Pool | 0.0 |
| Lung ca. NCI-H522 | 0.0 | Brain (Substantia nigra) Pool | 0.0 |
| Liver | 0.0 | Brain (Thalamus) Pool | 0.0 |
| Fetal Liver | 0.0 | Brain (whole) | 0.0 |
| Liver ca. HepG2 | 0.0 | Spinal Cord Pool | 0.0 |
| Kidney Pool | 0.0 | Adrenal Gland | 0.0 |
| Fetal Kidney | 0.0 | Pituitary gland Pool | 0.0 |
| Renal ca. 786-0 | 0.0 | Salivary Gland | 0.0 |
| Renal ca. A498 | 0.0 | Thyroid (female) | 0.0 |
| Renal ca. ACHN | 0.0 | Pancreatic ca. CAPAN2 | 0.0 |
| Renal ca.UO-31 | 0.0 | Pancreas Pool | 0.0 |

**Table ACD. General_screening_panel_v1.5**

| **Tissue Name** | **Rel. Exp.(%) Ag3304, Run 244373099** | **Rel. Exp.(%) Ag3304, Run 248445829** | **Tissue Name** | **Rel. Exp.(%) Ag3304, Run 244373099** | **Rel. Exp.(%) Ag3304, Run 248445829** |
|---|---|---|---|---|---|
| Adipose | 0.5 | 5.6 | Renal ca. TK-10 | 8.1 | 6.2 |
| Melanoma* Hs688(A).T | 15.0 | 0.0 | Bladder | 4.6 | 35.6 |
| Melanoma* Hs688(B).T | 19.9 | 1.0 | Gastric ca. (liver met.) NCI-N87 | 3.2 | 11.4 |
| Melanoma* M14 | 17.3 | 0.5 | Gastric ca. KATO III | 3.4 | 18.4 |
| Melanoma* LOXIMVI | 13.7 | 0.7 | Colon ca. SW-948 | 0.6 | 39.8 |
| Melanoma* SK-MEL-5 | 11.0 | 0.0 | Colon ca. SW480 | 7.6 | 6.2 |
| Squamous cell carcinoma SCC-4 | 15.6 | 47.3 | Colon ca.* (SW480 met) SW620 | 4.1 | 5.3 |
| Testis Pool | 1.1 | 4.9 | Colon ca. HT29 | 6.3 | 3.8 |
| Prostate ca.* (bone met) PC-3 | 11.6 | 19.3 | Colon ca. HCT-116 | 2.6 | 5.8 |
| Prostate Pool | 3.8 | 1.7 | Colon ca. CaCo-2 | 0.7 | 4.7 |
| Placenta | 0.0 | 5.1 | Colon cancer tissue | 0.0 | 11.2 |
| Uterus Pool | 0.3 | 1.9 | Colon ca. SW1116 | 7.8 | 2.1 |
| Ovarian ca. OVCAR-3 | 0.0 | 2.1 | Colon ca. Colo-205 | 4.0 | 39.8 |
| Ovarian ca. SK-OV-3 | 1.1 | 13.7 | Colon ca. SW-48 | 4.4 | 4.3 |
| Ovarian ca. OVCAR-4 | 5.0 | 100.0 | Colon Pool | 0.0 | 1.3 |
| Ovarian ca. OVCAR-5 | 6.4 | 43.2 | Small Intestine Poll | 0.7 | 4.4 |
| Ovarian ca. IGROV-1 | 1.1 | 26.6 | Stomach Pool | 1.0 | 22.4 |
| Ovarian ca. OVCAR-8 | 11.3 | 5.8 | Bone Marrow Pool | 3.7 | 5.4 |
| Ovary | 0.3 | 13.7 | Fetal Heart | 0.0 | 12.0 |
| Breast ca.MCF-7 | 3.8 | 3.6 | Heart Pool | 0.3 | 12.4 |
| Breast ca. MDA-MB-231 | 1.3 | 3.3 | Lymph Node Pool | 0.2 | 5.6 |
| Breast ca. BT 549 | 3.6 | 0.9 | Fetal Skeletal Muscle | 0.8 | 1.4 |
| Breast ca. T47D | 0.3 | 0.9 | Skeletal Muscle Pool | 0.9 | 3.1 |
| Breast ca. MDA-. N | 0.2 | 3.0 | Spleen Pool | 2.2 | 12.2 |
| Breast Pool | 0.0 | 6.6 | Thymus Pool | 2.1 | 7.3 |
| Trachea | 0.0 | 5.4 | CNS cancer cancer (glio/astro) U87-MG | 0.8 | 1.3 |
| Lung | 0.0 | 7.9 | CNS cancer (glio/astro) U-118-MG | 2.7 | 22.7 |
| Fetal Lung | 0.4 | 13.9 | CNS cancer (neuro;met) SK-N-AS | 3.4 | 1.2 |
| Lung ca. NCI-N417 | 2.1 | 0.9 | CNS cancer (astro) SF-539 | 2.8 | 4.3 |
| Lung ca. LX-1 | 24.3 | 9.7 | CNS cancer (astro) SNB-75 | 5.0 | 15.4 |
| Lung ca. NCI-H146 | 0.7 | 0.0 | CNS cancer (glio) SNB- 19 | 1.4 | 28.5 |
| Lung ca. SHP-77 | 5.1 | 12.1 | CNS cancer (glio) SF-295 | 16.2 | 45.1 |
| Lung ca. A549 | 19.9 | 3.9 | Brain (Amygdala) Pool | 4.3 | 8.4 |
| Lung ca. NCI-H526 | 2.1 | 0.7 | Brain (cerebellum) | 3.2 | 3.1 |
| Lung ca. NCI-H23 | 29.5 | 63.7 | Brain (fetal) | 1.3 | 5.6 |
| Lung ca. NCI-H460 | 100.0 | 18.4 | Brain (Hippocampus) Pool | 0.5 | 2.8 |
| Lung ca. HOP-62 | 16.7 | 4.1 | Cerebral Cortex Pool | 0.9 | 3.1 |
| Lung ca. NCI-H522 | 3.0 | 1.6 | Brain (Substantia nigra) Pool | 4.1 | 31.0 |
| Liver | 0.4 | 2.3 | Brain (Thalamus) Pool | 3.3 | 5.4 |
| Fetal Liver | 0.0 | 5.0 | Brain (whole) | 0.0 | 17.0 |
| Liver ca. HepG2 | 3.3 | 4.8 | Spinal Cord Pool | 0.3 | 2.7 |
| Kidney Pool | 1.4 | 4.0 | Adrenal Gland | 15.7 | 4.6 |
| Fetal Kidney | 1.2 | 4.2 | Pituitary gland Pool | 0.0 | 36.9 |
| Renal ca. 786-0 | 20.0 | 9.2 | Salivary Gland | 0.0 | 0.0 |
| Renal ca A498 | 3.4 | 16.5 | Thyroid (female) | 0.3 | 8.2 |
| Renal ca. ACHN | 6.3 | 8.7 | Pancreatic ca. CAPAN2 | 11.1 | 26.2 |
| Renal ca. U0-31 | 7.6 | 6.3 | Pancreas Pool | 1.9 | 19.9 |

**Table ACE. Panel 4D**

| **Tissue Name** | **Rel. Exp.(%) Ag3304, Run 164682642** | **Tissue Name** | **Rel. Exp.(%) Ag3304, Run 164682642** |
|---|---|---|---|
| Secondary Th1 act | 9.4 | HUVEC IL-lbeta | 8.6 |
| Secondary Th2 act | 9.3 | HUVEC IFN gamma | 11.7 |
| Secondary Tr1 act | 20.9 | HUVEC TNF alpha+IFN gamma | 27.0 |
| Secondary Th1 rest | 3.5 | HUVEC TNF alpha + ILA | 14.4 |
| Secondary Th2 rest | 2.1 | HUVEC IL-11 | 15.0 |
| Secondary Tr1 rest | 1.5 | Lung Microvascular EC none | 8.0 |
| Primary Th1 act | 10.1 | Lung Microvascular EC TNFalpha +IL-1beta | 13.3 |
| Primary Th2 act | 8.7 | Microvascular Dermal EC none | 6.1 |
| Primary Tr1 act | 25.0 | Microsvasular Dermal EC TNFalpha+IL-1beta | 15.2 |
| Primary Th1 rest | 25.9 | Bronchial epithelium TNFalpha + IL1beta | 54.3 |
| Primary Th2 rest | 8.9 | Small airway epithetium none | 20.4 |
| Primary Tr1 rest | 5.4 | Small airway epithelium TNFalpha +IL-1beta | 51.4 |
| CD45RA CD4 lymphocyte act | 5.4 | Coronery artery SMC rest | 30.1 |
| CD45RO CD4 lymphocyte act | 25.2 | Coronery artery SMC TNFalpha + IL-1beta | 10.2 |
| CD8 lymphocyte act | 43.2 | Astrocytes rest | 17.7 |
| Secondary CD8 lymphocyte rest | 31.0 | Astrocytes TNFa1pha + IL-1 beta | 25.7 |
| Secondary CD8 lymphocyte act | 13.6 | KU-812 (Basophil) rest | 4.0 |
| CD4 lymphocyte none | 1.6 | KU-812 (Basophil) PMA/ionomycin | 20.3 |
| 2ry Th1/Th2/Tr1_anti-CD95 CH11 | 6.2 | CCD1106 (Keratinocytes) none | 21.0 |
| LAK cells rest | 5.3 | CCD1106 (Keratinocytes) TNFalpha + IL-1beta | 58.6 |
| LAK cells IL-2 | 11.9 | Liver cirrhosis | 2.7 |
| LAK cells IL-2+IL-12 | 11.5 | Lupus kidney | 3.7 |
| LAK cells IL-2+IFN gamma | 11.2 | NCI-H292 none | 31.2 |
| LAK cells IL-2+ IL-18 | 17.9 | NCI-H292 IL-4 | 58.2 |
| LAK cells PMA/ionomycin | 18.3 | NCI-H292 IL-9 | 100.0 |
| NK Cells IL-2 rest | 17.1 | NCI-H292 IL-13 | 1.4 |
| Two Way MLR 3 day | 12.1 | NCI H292 IFN gamma | 35.6 |
| Two Way MLR 5 day | 6.4 | HPAEC none | 12.5 |
| Two Way MLR 7 day | 10.7 | BPAEC TNF alpha+ IL-1 beta | 21.0 |
| PBMC rest | 3.3 | Lung fibroblast none | 21.0 |
| PBMC PWM | 33.9 | Lung fibroblast TNF alpha + IL-1 | 11.6 |
| FBMC PHA-L | 33.9 | Lung fibroblast IL-4 | 18.8 |
| Ramos (B cell) none | 8.0 | Lung fibroblast IL-9 | 26.4 |
| Ramos (B cell) ionomycin | 32.5 | Lung fibroblast IL-13 | 20.9 |
| B lymphocytes PWM , | 36.9 | Lung fibroblast IFN gamma | 67.4 |
| B lymphocytes CD40L and IL-4 | 11.3 | Demral fibroblast CCD1070 rest | 62.0 |
| EOL-1 dbcAMP | 1.9 | Dermal fibroblast CCD1070 TNF alpha | 94.6 |
| EOL-1 dbcAMP PMA/ionomycin | 23.0 | Dermal fibroblast CCD1070 IL-1 beta | 16.7 |
| Dendritic cells none | 15.4 | Dermal fibroblast IFN gamma | 16.4 |
| Dendritic cells LPS | 34.9 | Dermal fibroblast IL-4 | 43.5 |
| Dendritic cells anti-CD40 | 9.5 | IBD Colitis 2 | 0.3 |
| Monocytes rest | 16.4 | IBD Crohn's | 0.8 |
| Monocytes LPS | 12.9 | Colon | 12.1 |
| Macrophages rest | 30.8 | Lung | 9.0 |
| Macrophages LPS | 29.5 | Thymus | 0.7 |
| HUVEC none | 13.5 | Kidney | 10.2 |
| HUVEC starved | 38.4 | | |

**Table ACF. Panel 5 Islet**

| **Tissue Name** | **Rel. Exp.(%) Ag3304, Run 242322478** | **Tissue Name** | **Rel. Exp.(%) Ag3304, Run 242322478** |
|---|---|---|---|
| 97457_Patient-02go_adipose | 9.0 | 94709_Donor 2 AM - A adipose | 12.1 |
| 97476 Patient-07sk skeletal muscle | 11.2 | 94710_Donor 2 AM-B adipose | 6.1 |
| 97477_Patient-07ut_uterus | 20.0 | 94711_Donor 2 AM - C_adipose | 2.4 |
| 97478_Patient-07pl_placenta | 8.1 | 94712_Donor2AD-A_adipose | 19.6 |
| 99167_Bayer Patient 1 | 42.3 | 94713_Donor 2 AD-B_adipose | 16.8 |
| 97482_Patient-08ut_uterus | 8.8 | 94714_Donor2AD-C_adipose | 15.8 |
| 97483_Patient-08pl_placenta | 4.6 | 94742_Donor 3U-A_Mesenchymal Stem Cells | 4.9 |
| 97486_Patient-09sk_skeletal muscle | 6.2 | 94743_Donor 3 U-B_Mesenchymal Stem Cells | 6.4 |
| 97487_Pattent-09ut_uterus | 12.4 | 94730_Donor 3 AM - A_adipose | 10.8 |
| 97488_Patient-09pl_placenta | 3.9 | 94731_Donor 3 AM - B_adipose | 8.0 |
| 97492_Patient-10ut_uterus | 8.0 | 94732_Donor 3 AM - C_adipose | 6.2 |
| 97493_Patient-10pl_placenta | 3.4 | 94733_Donor 3 AD - A_adipose | 32.8 |
| 97495_Patient-11go_adipose | 2.0 | 94734_Donor 3 AD - B_adipose | 11.7 |
| 97496_Patient-11sk_skeletal muscle | 4.7 | 94735_Donor 3 AD - C_adipose | 23.5 |
| 97497_Patient-11ut_uterus | 2.9 | 77138_Liver_HepG2untreated | 9.2 |
| 97498_Patient-11pl_placenta | 1.8 | 73556_Heart_Cardiac stromal cells (primary) | 2.4 |
| 97500_Patient-12go_adipose | 4.2 | 81735_Small Intestine | 3.8 |
| 97501_Patient-12sk_skeletal muscle | 14.1 | 72409_Kidney_Proximal Convoluted Tubule | 16.8 |
| 97502 Patient-12ut uterus | 3.5 | 82685_Small intestine_Duodenum | 4.1 |
| 97503 Patient-12pl_placenta | 0.9 | 90650_Adrenal_Adrenocortical adenoma | 2.4 |
| 94721_Donor 2 U - A_Mesenchymal Stem Cells | 8.9 | 72410_Kidney_HRCE | 100.0 |
| 94722 Donor 2 U - B_Mesenchymal Stem Cells | 4.3 | 72411_Kidney_HRE | 36.1 |
| 94723 -Donor 2 U - C_Mesenchymal Stem Cells | 9.1 | 73139_Uterus_Uterine smooth muscle cells | 20.6 |

**CNS_neurodegeneration_v1.0 Summary:** A-g3304 This panel demonstrates the expression of this gene at low levels in the brain in several different individuals. However, no differential expression of this gene was detected between Alzheimer's diseased postmortem brains and those of non-demented controls in this experiment.

**General_screening_panel_v1.4 Summary:** Ag3304 Expression of this gene is low/undetectable (CTs > 35) across all of the samples on this panel (data not shown).

**General_screening_panel_v1.5 Summary:** Ag3304 Results from two experiments using the same probe/primer set gave results that are only in moderate agreement. Only those results that are in agreement will be discussed here. Expression of the CG57670-01 gene is detected at low levels in pancreatic cancer cell line CAPAN2, CNS cancer cell line SF-295, lung cancer cell lines NCI-H460 and NCI-H23, and squamous cell carcinoma cell line SCC-4. Thus, expression of this gene can be used to distinguish these cell lines from the other samples on this panel. In addition, therapeutic modulation of the activity of this gene or its protein product, through the use of small molecule drugs, protein therapeutics or antibodies, might be beneficial in the treatment of lung cancer, pancreatic cancer, CNS cancer and squamous cell carcinoma.

In one experiment, this gene is expressed at low levels in the adrenal gland. As a glycolytic enzyme, activation of the CG57670-01 gene may increase oxidative metabolism in this tissue and be a treatment for Addison's disease and other adrenalopathies.

**Panel 4D Summary:** Ag3304 The CG57670-01 gene is expressed at low to moderate levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, and kidney. This ubiquitous pattern of expression suggests that this gene product may be involved in homeostatic processes for these and other cell types and tissues.

Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

**Panel 5 Islet Summary:** Ag3304 The CG57670-01 gene is expressed at moderate levels in islets of Langerhans (patient 1). Insulin secretion is dependent on glycolysis, and activation of this glycolytic enzyme may increase the glycolytic rate and insulin secretion in Type 2 diabetes. This gene is also expressed at low levels in adipose, cultured adipocytes, skeletal muscle, uterus and placenta. Therefore, activation of this enzyme may also be a treatment for obesity by increasing the glycolytic rate and energy expenditure in adipose tissue.

### NOV36

Expression of NOV36A/CG57149-01 and NOV36B/CGS7149-02 was assessed using the primer-probe set Ag3119, described in Table DA. Results of the RTQ-PCR runs are shown in Tables DB, DC, DD and DE. Please note that CG57149-02 represents a full-length physical clone of the CG57149-01 gene, validating the prediction of the gene sequence.

**Table DA. Probe Name Ag3119**

| **Primers** | **Sequences** | **Length** | **Start Position** | **SEQ ID NO** |
|---|---|---|---|---|
| Foreward | 5'-tgtactaccagccatggtcaac-3' | 22 | 12 | 365 |
| Probe | TET-5'-catgttcttcaacatcgccatcaaca-3'-TAMRA | 26 | 39 | 366 |
| Reverse | 5'-acttgtctgcaacagttcgaa-3' | 22 | 88 | 367 |

**Table DB. CNS_neurodegeneration_v1.0**

| **Tissue Name** | **Rel. Exp.(%) Ag3119, Run 208976882** | **Tissue Name** | **Rel. Exp.(%) Ag3119, Run 208976882** |
|---|---|---|---|
| AD 1 Hippo | 3.8 | Control (Path) 3 Temporal | 8.4 |
| AD 2 Hippo | 7.0 | Control (Path) 4 Temporal | 35.8 |
| AD 3 Hippo | 14.5 | AD 1 Occipital Ctx | 36.3 |
| AD 4 Hippo | 1.5 | AD 2 Occipital Ctx (Missing) | 0.0 |
| AD 5 Hippo | 100.0 | AD 3 Occipital Ctx | 18.4 |
| AD 6 Hippo | 4.8 | AD 4 Occipital Ctx | 7.1 |
| Control 2 Hippo | 1.7 | AD 5 Occipital Ctx | 0.7 |
| Control 4 Hippo | 1.2 | AD 6 Occipital Ctx | 36.3 |
| Control (Path) 3 Hippo | 2.2 | Control 1 Occipital Ctx | 0.6 |
| AD 1 Temporal Ctx | 16.4 | Control Occipital Ctx | 3.7 |
| AD 2 Temporal Ctx | 12.2 | Control 3 Occipital Ctx | 49.0 |
| AD3 Temporal Ctx | 16.6 | Control 4 Occipital Ctx ; | 3.0 |
| AD4 Temporal Ctx | 19.8 | Control (Path) 1 Occipital Ctx | 29.9 |
| AD 5 Inf Temporal Ctx | 35.8 | Control (Path) 2 Occipital Ctx | 15.8 |
| AD 5 Sup Temporal Ctx | 41.2 | Control (Path) 3 Occipital Ctx | 7.4 |
| AD 6 Inf Temporal Ctx | 16.4 | Control (Path) 4 Occipital | 42.9 |
| AD 6 Sup Temporal Ctx | 35.6 | Control 1 Parietal Ctx | 10.4 |
| Control 1 Temporal Ctx | 7.9 | Control 2 Parietal Ctx | 40.9 |
| Control 2 Temporal Ctx | 0.0 | Control 3 Parietal Ctx | 5.2 |
| Control 3 Temporal Ctx | 4.7 | Control (Path) 1 Parietal Ctx | 5.2 |
| Control 3 Temporal Ctx | 4.0 | Control (Path) 2 Parietal Ctx | 8.9 |
| Control (Path) 1 Temporal Ctx | 14.9 | Control (Path) 3 Parietal Ctx | 5.7 |
| Control (Path) 2 Temporal Ctx | 20.0 | Control (Path) 4 Parietal Ctx | 44.1 |

**Table DC Panel 1.3D**

| **Tissue Name** | **Rel. Exp.(%) Ag3119, Run 167985261** | **Tissue Name** | **Rel. Exp.(%) Ag3119, Run 167985261** |
|---|---|---|---|
| Liver adenocarcinoma | 1.2 | Kidney (fetal) | 16.3 |
| Pancreas | 0.6 | Renal ca. 786-0 | 7.1 |
| Pancreatic ca. CAPAN 2 | 27.9 | Renal ca. A498 | 0.0 |
| Adrenal gland | 0.9 | Renal ca. RXF 393 | 7.9 |
| Thyroid | 0.0 | Renal ca. ACHN | 26.6 |
| Salivary gland | 3.3 | Renal ca. UO-31 | 0.0 |
| Pituitary gland | 1.0 | Renal ca. TK-10 | 34.9 |
| Brain (fetal) | 100.0 | Liver | 5.5 |
| Brain (whole) | 3.2 | Liver (fetal) | 0.5 |
| Brain (amygdala) | 12.7 | Liver ca. (hepatoblast) HepG2 | 44.4 |
| Brain (cerebellum) | 68.3 | Lung | 6.7 |
| Brain (hippocampus) | 1.0 | Lung (fetal) | 0.0 |
| Brain (substantia nigra) | 5.1 | Lung ca. (small cell) LX-1 | 1.0 |
| Brain (thalamus) | 9.9 | Lung ca. (small cell) NCI-H69 | 0.0 |
| Cerebral Cortex | 6.7 | Lung ca. (s.cell var.) SHP-77 | 0.0 |
| Spinal cord | 18.2 | Lung ca. (large cell)NCI-H460 | 0.0 |
| glio/astro U87-MG | 1.1 | Lung ca. (non-sm. cell) A549 | 7.1 |
| glio/astro U-118-MG | 1.5 | Lung ca.(non-s.cell) NCI-H23 | 0.5 |
| astrocytoma SW1783 | 2.1 | Lung ca.(non-s.cell) HOP-62 | 0.0 |
| neuro*; met SK-N-AS | 1.4 | Lung ca.(non-s.cl) NCI-H522 | 2.0 |
| astrocytoma SF-539 | 0.0 | Lung ca. (squam) SW 900 | 0.0 |
| astrocytoma SNB-75 | 1.5 | Lung ca. (squam.) NCI-H596 | 0.0 |
| glioma SNB-19 | 0.0 | Mammary gland | 1.0 |
| glioma U251 | 0.0 | Breast ca.*(pl.ef)MCF-7 | 0.0 |
| glioma SF-295 | 6.5 | Breast ca.*(pl.ef)MDA-MB-231 | 0.0 |
| Heart (fetal) | 0.0 | Breast ca.* (pl.ef) T47D | 0.0 |
| Heart | 8.1 | Breast ca. BT-549 | 5.6 |
| Skeletal muscle (fetal) | 9.9 | Breast ca. MDA-N | 0.0 |
| Skeletal muscle | 4.1 | Ovary | 0.0 |
| Bone marrow | 3.1 | Ovarian ca. OVCAR-3 | 0.0 |
| Thymus | 97.3 | Ovarian ca. OVCAR-4 | 0.0 |
| Spleen | 9.5 | Ovarian ca. OVCAR-5 | 0.0 |
| Lymph node | 19.8 | Ovarian ca. OVCAR-8 | 0.7 |
| Colorectal | 24.5 | Ovarian ca. IGROV-1 | 0.0 |
| Stomach | 18.8 | Ovarian ca.* (ascites) SK-OV-3 | 11.5 |
| Small intestine | 22.7 | Uterus | 10.8 |
| Colon ca. SW480 | 0.0 | Placenta | 0.0 |
| Colon ca.* SW620(SW480 met) | 4.3 | Prostate | 5.6 |
| Colon ca. HT29 | 0.0 | Prostate ca.* (bone met) PC- 3 | 0.0 |
| Colon ca. HCT-116 | 2.1 | Testis | 4.3 |
| Colon ca. CaCo-2 | 16.4 | Melanoma Hs688(A).T | 0.0 |
| Colon ca. tissue(ODO3866) | 0.0 | Melanoma* (met) Hs688(B).T | 0.5 |
| Colon ca.HCC-2998 | 0.0 | Melanoma UACC-62 | 0.0 |
| Gastric ca.* (liver met) NCI-N87 | 0.0 | Melanoma M14 | 0.0 |
| Bladder | 1.7 | Melanoma LOX IMVI | 0.0 |
| Trachea | 1.6 | Melanoma* (met) SK-MEL-5 | 0.0 |
| Kidney | 0.0 | Adipose | 0.0 |

**Table DD. Panel 4D**

| **Tissue Name** | **Rel. Exp.(%) Ag3119, Run 164526109** | **Tissue Name** | **Rel. Exp.(%) Ag3119, Run 164526109** |
|---|---|---|---|
| Secondary Tb1 act | 5.7 | HUVEC IL-1beta | 3.3 |
| Secondary Th2 act | 8.8 | HUVEC IFN gamma | 19.5 |
| Secondary Tr1 act | 11.3 | HUVEC TNF alpha+ IFN gamma | 34.6 |
| Secondary Th1 rest | 9.2 | HUVEC TNF alpha+ILA | 10.8 |
| Secondary Th2 rest | 12.9 | HUVEC IL-11 | 14.2 |
| Secondary Tr1 rest | 18.9 | Lung Microvascular EC none | 36.3 |
| Primary Th1 act | 1.5 | Lung Microvascular EC TNFalpha + IL-1beta | 31.9 |
| Primary Th2 act | 7.2 | Microvascular Dermal EC none | 38.7 |
| Primary Tr1 act | 5.9 | Microsvasular Dermal EC TNF alpha + IL-1beta | 16.4 |
| Primary Th1 rest | 56.6 | Bronchial epithelium TNFalpha + IL1beta | 0.0 |
| Primary Th2 rest | 41.8 | Small airway epithelium none | 2.3 |
| Primary Tr1 rest | 31.2 | Small airway epithelium TNFalpha +IL-1beta | 1.6 |
| CD45RA CD4 lymphocyte act | 4.4 | Coronery artery SMC rest | 5.4 |
| CD45RO CD4 lymphocyte act | 8.5 | Coronery artery SMC TNFalpha + IL-1beta | 0.9 |
| CD8 lymphocyte act | 3.6 | Astrocytes rest | 14.8 |
| Secondary CD8 lymphocyte rest | 5.3 | Astrocytes TNFalpha + IL-1beta | 3.4 |
| Secondary CDS lymphocyte act | 6.9 | KU-812 (Basophil) rest | 0.3 |
| CD4 lymphocyte none | 8.1 | KU-812 (Basophil) PMA/ionomycin | 0.7 |
| 2ry Th1/Th2/Tr1_anti-CD95 CH11 | 20.0 | CCD1106 (Keratinocytes) none | 2.5 |
| LAK cells rest TNF alpha + IL-1beta | 4.7 | CCD1106 (Keratinocytes) | 1.9 |
| LAK cells IL-2 | 6.0 | Liver cirrhosis | 5.3 |
| LAK cells IL-2+EL-12 | 11.8 | Lupus kidney | 1.6 |
| LAK cells IL-2+IFN gamma, | 17.6 | NCI-H292 none | 1.8 |
| LAK cells IL-2+IL-18 | 19.3 | NCI-H292 IL-4 | 0.2 |
| LAK cells PMA/ionomycin | 2.8 | NCI-H292 IL-9 | 3.0 |
| NK Cells IL-2rest | 22.8 | NCI-H292 IL-13 | 0.4 |
| Two Way MLR 3 day | 21.2 | NCI-H292 IFN gamma | 2.5 |
| Two Way MLR 5 day | 7.7 | HPAEC none | 16.7 |
| Two Way MLR 7 day | 3.4 | HPABCTNFalpha+IL-1beta | 11.0 |
| PBMC rest | 1.1 | Lung fibroblast none | 9.9 |
| PBMC PWM | 15.7 | Lung fibroblast TNF alpha +IL-1 beta | 0.6 |
| PBMC PHA-L | 4.9 | Lung fibroblast IL-4 | 5.6 |
| Ramos (B cell) none | 0.0 | Lung fibroblast IL-9 | 9.0 |
| Ramos (B cell) ionomycin | 0.0 | Lung fibroblast IL-13 | 8.1 |
| B lymphocytes PWM | 0.7 | Lung fibroblast IFN gamma | 10.4 |
| B lymphocytes CD40L and IL-4 | 1.2 | Dermal fibroblast CCD1070 rest | 2.2 |
| EOL-1 dbcAMP | 0.0 | Dermal fibroblast CCD1070 TNF alpha | 35.1 |
| EOL-1 dbcAMP PMA/ionomycin | 0.0 | Dermal fibroblast CCD1070 IL-1 beta | 3.0 |
| Dendritic cells none | 0.2 | Dermal fibroblast IFN gamma | 3.9 |
| Dendritic cells LPS | 0.0 | Dermal fibroblast IL-4 | 10.7 |
| Dendritic cells anti-CD40 | 0.0 | IBD Colitis 2 | 0.9 |
| Monocytes rest | 0.0 | IBD Crohn's | 1.9 |
| Monocytes LPS | 11.5 | Colon | 7.2 |
| Macrophages rest | 8.0 | Lung | 0.0 |
| Macrophages LPS | 0.0 | Thymus | 12.4 |
| HUVEC none | 20.3 | Kidney | 100.0 |
| HUVEC starved | 82.4 | | |

**Table DE. Panel CNS_1**

| **Tissue Name** | **Rel. Exp.(%) Ag3119, Run 182257490** | **Tissue Name** | **Rel. Exp.(%) Ag3119, Run 182257490** |
|---|---|---|---|
| BA4 Control | 2.8 | BA17PSP | 2.6 |
| BA4 Control2 | 0.0 | BA17 PSP2 | 0.0 |
| BA4 Alzheimer's2 | 2.2 | Sub Nigra Control | 1.3 |
| BA4 Parkinson's | 31.0 | Sub Nigra Control2 | 11.3 |
| BA4 Parkinson's2 | 7.2 | Sub Nigra Alzheimer's2 | 5.7 |
| BA4 Huntington's | 26.8 | Sub Nigira Parkinson's2 | 4.7 |
| BA4 Huntington's2 | 5.9 | Sub Nigra Huntington's | 28.7 |
| BA4 PSP | 2.0 | Sub Nigra Huntington's2 | 1.1 |
| BA4 PSP2 | 4.6 | Sub Nigra PSP2 | 0.0 |
| BA4 Depression | 14.5 | Sub Nigra Depression | 4.6 |
| BA4 Depression2 | 6.8 | Sub Nigra Depression2 | 0.0 |
| BA7 Control | 48.0 | Glob Palladus Control | 8.1 |
| BA7 Control2 | 0.0 | Glob Palladus Control2 | 8.2 |
| BA7 Alzheimer's2 | 16.3 | Glob Palladus Alzheimer's | .1 |
| BA7 Parkinson's | 6.7 | Glob Palladus Alzheimer's2 | 6.9 |
| BA7 Parldnson's2 | 30.1 | Glob Palladus Parkinson's | 44.1 |
| BA7 Huntington's | 2.8 | Glob Palladus Parkinson's2 | 19.6 |
| BA7 Huntington's2 | 75.8 | Glob Palladus PSP | 0.0 |
| BA7 PSP | 2.4 | Glob Palladus PSP2 | 0.0 |
| BA7 PSP2 | 3.6 | Glob Palladus Depression | 0.0 |
| BA7 Depression | 36.3 | Temp Pole Control | 7.1 |
| BA9 Control | 3.3 | Temp Pole Control2 | 2.5 |
| BA9 Control2 | 23.7 | Temp Pole Alzheimer's | 6.4 |
| BA9 Alzheimer's | 8.4 | Temp Pole Alzheimer's2 | 9.1 |
| BA9 Alzheimer's2 | 9.2 | Temp Pole Parkinson's | 29.1 |
| BA9 Parkinson's | 22.5 | Temp Pole Parkinson's2 | 2.8 |
| BA9 Parkinson's2 | 10.4 | Temp Pole Huntington's | 28.5 |
| BA9 Huntington's | 20.3 | Temp Pole PSP | 8.2 |
| BA9 Huntington's2 | 35.8 | TEMP pole PSP2 | 0.0 |
| BA9 PSP | 5.9 | Temp Pole Depression2 | 0.0 |
| BA9 PSP2 | 0.0 | Cing Gyr Control | 18.4 |
| BA9 Depression | 0.0 | Cing Gyr Control2 | 4.5 |
| BA9 Depression2 | 0.0 | Cing Gyr Alzheimer's | 5.3 |
| BA17 Control | 60.7 | Cing Gyr Alzheimer's2 | 15.5 |
| BA17 Control2 | 31.0 | Cing Gyr Pankinson's | 3.6 |
| BA17 Alzheimer's2 | 36.9 | Cing Gyr Parkinson's2 | 30.6 |
| BA17 Parkinson's | 40.3 | Cing Gyr Huntington's | 1.6 |
| BA17Parktnson's2 | 100.0 | Cing Gyr Huntington's2 | 1.6 |
| BA17 Hundngton's | 1.8 | Cing Gyr PSP | 0.0 |
| BA17 Huntington's2 | 37.1 | Cing Gyr PSP2 | 14.5 |
| BA17 Depression | 7.2 | Cing Gyr Depression | 0.0 |
| BA17 Depression2 | 4.3 | Cing Gyr Depression2 | 5.8 |

**CNS neurodegeneration_v1.0 Summary:** Ag3119 This panel confirms the expression of this gene at low levels in the brain in an independent group of individuals. However, no differential expression of this gene was detected between Alzheimer's diseased postmortem brains and those of non-demented controls in this experiment. Please see Panel 1.3D for a discussion of the potential utility of this gene in treatment of central nervous system disorders.

**Panel 1.3D Summary:** Ag3119 The CG57149-01 gene is expressed at highest levels in fetal brain (CT = 32.2). Interestingly, this gene is expressed at much lower levels in adult brain (CT = 37.2). Therefore, expression of this gene can be used to distinguish fetal brain from adult brain. In addition, the relative overexpression of this gene in fetal brain suggests that this protein product may enhance brain growth or development in the fetus and thus may also act in a regenerative capacity in the adult.

This gene is also expressed at relatively high levels in thymus. The CG57149-01 gene encodes a protein with homology to cis/trans peptidyl prolyl isomerase. peptidyl-prolyl cis/trans isomerase A (also known as PPIA or cyclophilin A) is a member of the immunophilin class of proteins. These cytosolic enzymes have peptidyl-prolyl cis/trans isomerase activity and are thought to play a role in protein folding and/or intracellular protein transport (ref. 1). The immunosuppressive action of the immunomodulatory agent cyclosporin is linked to its binding to cyclophilin, which inhibits the enzymatic acticity of cyclophilin (ref. 2). Cyclosporin has major effects on T cells. Modulation of this novel cyclophilin may therefore be important in the treatment of in regulating T cell function and treating T cell mediated diseases such as asthma, arthritis, psoriasis, IBD, and systemic lupus erythematosus.

### References:

### 1. Ivery MT. Immunophilins: switched on protein binding domains? Med Res Rev 2000 Nov;20(6):452-84..

Peptidylprolyl isomerases (PPIases) are a group of cytosolic enzymes first characterized by their ability to catalyze the cis-trans isomerization of cis-peptidylprolyl bonds. Subsequently, some PPIases were also identified as the initial targets of the immunosuppressant drugs-cyclosporin A (CsA), FK506, and rapamycin-have been called immunophilins. Immunophilins have been found to be both widely distributed and abundantly expressed leading to suggestions that they may play a general role in cellular biochemistry. However, the nature of this role has been difficult to elucidate and is still controversial in vivo. A number of roles for these enzymes have been identified in vitro including the ability to catalyze the refolding of partly denatured proteins and stabilize multiprotein complexes such as Ca(2+) channels, inactive steroid receptor complexes, and receptor protein tyrosine kinases. Generally, these effects appear to depend on the ability of immunophilins to selectively bind to other proteins. This review will examine in detail experimental and structural investigations of the mechanism of PPIase activity for both FKBPs and cyclophilins and suggest a mechanism for these enzymes, which depends on their ability to recognize a specific peptide conformation rather than sequence. Examination of structures of immunophilin-protein complexes will then be used to further suggest that the ability of these enzymes to recognize specific peptide conformations is central to the formation of these complexes and may constitute a general function of immunophilin enzymes. The binding of ligand to immunophilins will also be shown to stabilize specific conformations in surface loops of these proteins that are observed to play a critical role in a number of immunophilin-protein complexes suggesting that the immunophilins may constitute a class of ligand-triggered selective protein binders.

### PMID: 11058892

### 2. Russell G, Graveley R, Seid J, al-Humidan AK, Skjodt H. Mechanisms of action of cyclosporine and effects on connective tissues. Semin Arthritis Rheum 1992 Jun;21(6 Suppl 3):16-22.

Cyclosporine is a potent immunomodulatory agent with an increasing number of clinical applications. Its major mode of action is inhibition of the production of cytokines involved in the regulation of T-cell activation. In particular, cyclosporine inhibits the transcription of interleukin 2. Although cyclosporine's major actions are on T cells, there is some evidence that it produces direct effects on other cell types. Its immunosuppressive action is closely linked to its binding of cyclophilin, a member of a family of high-affinity cyclosporine-binding proteins widely distributed in different cell types and in different species. The cyclophilins have been shown to have peptidyl-prolyl cis-trans isomerase enzyme activity that is blocked by cyclosporine. Although this may be a factor in cyclosporine's selective inhibition of cytokine gene transcription, it is still unclear whether inhibition of this activity is the mechanism through which cyclosporine exerts its effects on target cells. The ubiquitous presence of cyclophilins raises the question of why cyclosporine has major effects on T cells. Perhaps the critical proteins affected are transcriptional regulators restricted in their tissue distribution. The effects of cyclosporine on T cells and, directly or indirectly, on connective tissue cells, all of which can produce a range of cytokines, are of interest in relation to the tissue changes that occur in such inflammatory conditions as rheumatoid arthritis.

### PMID: 1502562

**Panel 4D Summary:** Ag3119 The CG57149-01 gene is expressed at moderate levels in T cells and is also expressed at significant levels in endothelium, epithelium and fibroblasts as well as in normal thymus and kidney. Expression of this gene in thymus is consistent with what is observed in Panel 1.3D. Furthermore, treatment of proinflammatory mediators reduces the level of this transcript in HUVEC cells as compared to in starved cells. The protein encoded for by this transcript may be important in the normal function of the cell types that express it. Regulation of the protein encoded for by this transcript could be important for maintaining or resuming normal homeostasis.

**Panel CNS_1 Summary:** Ag3119 This panel confirms the expression of this gene at low levels in the brain in an independent group of individuals. Please see Panel 1.3D for a discussion of the potential utility of this gene in treatment of central nervous system disorders.

### NOV37

Expression of NOV37/CG57151-01 was assessed using the primer-probe set Ag3120, described in Table AEA. Results of the RTQ-PCR runs are shown in Tables AEB, AEC, AED and AEE.

**Table AEA. Probe Name Ag3120**

| **Primers** | **Sequences** | **Length** | **Start Position** | **SEQ ID NO** |
|---|---|---|---|---|
| Forward | 5'-atgctctgagcactggagaa-3' | 20 | 215 | 368 |
| Probe | TET-5'-tcctgctttcacagaattattccaggg-3'-TAMRA | 27 | 256 | 369 |
| Reverse | 5'-gtgtgaagtcaccactctgaca-3' | 22 | 289 | 370 |

**Table AEB. CNS_neurodegeneration_v1.0**

| **Tissue Name** | **Rel. Exp.(%) Ag3120, Run 208976883** | **Tissue Name** | **Rel. Exp.(%) Ag3120, Run 208976883** |
|---|---|---|---|
| AD 1 Hippo | 18.6 | Control (Path) 3 Temporal Ctx | 6.2 |
| AD 2 Hippo | 26.1 | Control (Path) 4 Temporal Ctx | 46.0 |
| AD 3 Hippo | 10.1 | AD 1 Occipital Ctx | 26.4 |
| AD 4 Hippo | 10.2 | AD 2 Occipital Ctx (Missing) | 0.0 |
| AD 5 hippo | 90.1 | AD 3 Occipital Ctx | 8.9 |
| AD 6 Hippo | 67.8 | AD 4 Occipital Ctx | 24.5 |
| Control 2 Hippo | 35.1 | AD 5 Occipital Ctx | 47.3 |
| Control 4 Hippo | 16.8 | AD 6 Occipital Ctx | 36.6 |
| Control (Path) 3 Hippo | 12.0 | Control 1 Occipital Ctx | 6.5 |
| AD 1 Temporal Ctx | 35.8 | Control 2 Occipital Ctx | 74.7 |
| AD 2 Temporal Ctx | 41.5 | Control 3 Occipital Ctx | 36.6 |
| AD 3 Temporal Ctx | 10.3 | Control 4 Occipital Ctx | 8.7 |
| AD 4 Temporal Ctx | 25.0 | Control (Path) 1 Occipital Ctx | 100.0 |
| AD 5 Inf Temporal Ctx | 87.1 | Control (Path) 2 Occipital Ctx | 17.1 |
| AD 5 Sup Temporal Ctx | 57.8 | Control (Path) 3 Occipital Ctx | 5.1 |
| AD 6 Inf Temporal Ctx | 79.6 | Control (Path) 4 Occipital Ctx | 37.9 |
| AD 6 Sup Temporal Ctx | 97.9 | Control 1 Parietal Ctx | 13.1 |
| Control 1 Temporal Ctx | 12.2 | Control 2 Parietal Ctx | 44.8 |
| Control 2 Temporal Ctx | 43.2 | Control 3 Parietal Ctx | 19.5 |
| Control 3 Temporal Ctx | 26.6 | Control (Path) 1 Parietal Ctx | 65.5 |
| Control 4 Temporal Ctx | 13.5 | Control (Path) 2 Parietal Ctx | 40.6 |
| Control (Path) 1 Temporal Ctx | 59.5 | Control (Path) 3 Parietal Ctx | 3.9 |
| Control (Path) 2 Temporal Ctx | 47.3 | Control (Path) 4 Parietal Ctx | 56.3 |

**Table AEC. Panel 1.3D**

| **Tissue Name** | **Rel. Exp.(%) Ag3120, Run 167985266** | **Tissue Name** | **Rel. Exp.(%) Ag3120, Run 167985266** |
|---|---|---|---|
| Liver adenocarcinoma | 46.0 | Kidney (fetal) | 97.3 |
| Pancreas | 14.3 | Renal ca. 786-0 | 25.7 |
| Pancreatic ca. CAPAN 2 | 8.2 | Renal ca. A498 | 26.2 |
| Adrenal gland | 12.8 | Renal ca. RXF 393 | 43.5 |
| Thyroid | 8.4 | Renal ca. ACHN | 12.0 |
| Salivary gland | 3.2 | Renal ca.UO-31 | 29.5 |
| Pituitary gland | 16.8 | Renal ca.TK-10 | 36.9 |
| Brain (fetal) | 30.1 | Liver | 8.7 |
| Brain (whole) | 12.4 | Liver (fetal) | 18.7 |
| Brain (amygdala) | 20.0 | Liver ca. (hepatoblast) HepG2 | 42.0 |
| Brain (cerebellum) | 30.8 | Lung | 12.2 |
| Brain (hippocampus) | 16.4 | Lung (fetal) | 45.7 |
| Brain (substantia nigra) | 42.3 | Lung ca. (small cell) LX-1 | 26.2 |
| Brain (thalamus) | 3.0 | Lung ca. (small cell) NCI-H69 | 10.1 |
| Cerebral Cortex | 12.4 | Lung ca. (s.cell var.) SHP- 77 | 85.3 |
| Spinal cord | 24.5 | Lung ca. (large cell)NCI-H460 | 4.2 |
| glio/astro U87-MG | 20.4 | Lung ca. (non-sm cell) A549 | 32.5 |
| glio/astro U-118-MG | 24.7 | Lung ca. (non-s.cell) NCI-H23 | 26.4 |
| astrocytoma SW1783 | 16.5 | Lung ca.(non-s.cell) HOP-62 | 15.0 |
| neuro*; met SK-N-AS | 21.9 | Lung ca. (non-s.cl)NCI-H522 | 15.6 |
| astrocytoma SF-539 | 21.6 | Lung ca. (squam.) SW 900 | 26.8 |
| astrocytoma SNB-75 | 28.3 | Lung ca. (squam.) NCI-H596 | 33.7 |
| glioma SNB-19 | 12.8 | Mammary gland | 6.7 |
| glioma U251 | 47.0 | Breast ca.* (pl.ef)MCF-7 | 26.1 |
| glioma SF-295 | 13.3 | Breast ca.* (pl.ef) MDA-MB-231 | 22.7 |
| Heart (fetal) | 8.7 | Breast ca.* (pl.ef) T47D | 43.5 |
| Heart | 11.6 | Breast ca. BT-549 | 16.4 |
| Skeletal muscle (fetal) | 5.4 | Breast ca. MDA-N | 25.0 |
| Skeletal muscle | 10.8 | Ovary | 5.2 |
| Bone marrow | 26.4 | Ovarian ca. OVCAR-3 | 13.5 |
| Thymus | 49.7 | Ovarian ca. OVCAR-4 | 21.3 |
| Spleen | 49.7 | Ovarian ca. OVCAR-5 | 100.0 |
| Lymph node | 37.4 | Ovarian ca. OVCAR-8 | 6.8 |
| Colorectal | 7.3 | Ovarian ca. IGROV-1 | 8.0 |
| Stomach | 12.7 | Ovarian ca.* (ascites) SK-OV-3 | 56.3 |
| Small intestine | 14.6 | Uterus | 16.7 |
| Colon ca. SW480 | 29.5 | Placenta | 5.1 |
| Colon ca.* SW620(SW480 met) | 52.1 | Prostate | 11.1 |
| Colon ca. HT29 | 23.0 | Prostate ca.* (bone met)PC-3 | 22.2 |
| Colon ca. HCT-116 | 23.8 | Testis | 8.4 |
| Colon ca. CaCo-2 | 48.0 | Melanoma Hs688(A).T | 4.5 |
| Colon ca. tissue (ODO3866) | 32.5 | Melanoma* (met) Hs688(B).T | 4.1 |
| Colon ca. HCC-2998 | 32.5 | Melanoma UACC-62 | 13.0 |
| Gastric ca.*(liver met) NCI- | 27.4 | Melanoma M14 | 11.0 |
| Bladder | 34.2 | Melanoma LOX IMVI | 15.4 |
| Trachea | 12.3 | Melanoma* (met) SK-MEL-5 | 12.6 |
| Kidney | 22.7 | Adipose | 44.4 |

**Table AED. Panel 4D**

| **Tissue Name** | **Rel.Exp.(%) Ag3120,Run 164526129** | **Tissue Name** | **Rel. Exp.(%) Ag3120, Run 164526129** |
|---|---|---|---|
| Secondary Th1 act | 15.5 | HUVEC IL-1beta | 7.1 |
| Secondary Th2 act | 17.6 | HUVEC IFN gamma | 19.3 |
| Secondary Tr1 act | 21.0 | HUVEC TNF alpha+IFN gamma | 12.4 |
| Secondary Th1 rest | 9.0 | HUVEC TNF alpha+IL4 | 16.0 |
| Secondary Th2 rest | 10.7 | HUVEC IL-11 | 20.7 |
| Secondary Tr1 rest | 16.8 | Lung Microvascular EC none | 12.1 |
| Primary Th1 act | 17.3 | Lung Microvascular EC TNFalpha + IL-1beta | 11.9 |
| Primary Th2 act | 15.7 | Microvascular Dermal EC none | 14.0 |
| Primary Tr1 act | 23.8 | Microsvasular Dermal EC TNFalpha + IL-1beta | 7.6 |
| Primary Th1 rest | 50.0 | Bronchial epithelium TNFalpha + IL1beta | 18.0 |
| PrimaryTh2 rest | 33.2 | Small airway epithelium none | 7.0 |
| Primary Tr1 rest | 26.8 | Small airway epithelium TNFalpha +IL-1beta | 34.4 |
| CD45RA CD4 lymphocyte act | 9.8 | Coronery artery SMC rest | 8.1 |
| CD45RO CD4 lymphocyte | 21.3 | Coronery artery SMC TNFalpha + act IL-1beta | 5.0 |
| CD8 lymphocyte act | 23.7 | Astrocytes rest | 4.9 |
| Secondary CD8 lymphocyte rest | 23.2 | Astrocytes TNFalpha + IL-1 beta | 7.0 |
| Secondary CD8 lymphocyte act | 10.2 | KU-812 (Basophil) rest | 8.3 |
| CD4 lymphocyte none | 12.9 | KU-812 (Basophil) PMA/ionomycin | 19.2 |
| 2ry Th1/Th2/Tr1_anti-CD95 CH11 | 25.3 | CCD1106 (Keratinocytes) none | 10.3 |
| LAK cells rest | 18.8 | CCD1106 (Keratinocytes) TNFalpha+IL-1beta | 5.4 |
| LAK cells IL-2 | 19.9 | Liver cirrhosis | 5.4 |
| LAK cells IL-2+IL-12 | 20.0 | Lupus kidney | 3.2 |
| LAK cells IL-2+1FN gamma | 26.1 | NCI-H292 none | 40.6 |
| LAK cells IL-2+ IL-18 | 23.3 | NCI-H292 IL-4 | 33.7 |
| LAK cells PMA/ionomycin | 29.3 | NCI-H292 IL-9 | 33.2 |
| NK Cells IL-2 rest | 20.4 | NCI-H292 IL-13 | 23.0 |
| Two Way MLR 3 day | 27.9 | NCI-H292 IFN gamma | 21.0 |
| Two Way MLR 5 day | 12.9 | HPAEC none | 10.7 |
| Two Way MLR 7 day | 11.3 | HPAEC TNF alpha + IL- 1 beta | 12.6 |
| PBMC rest | 35.4 | Lung fibroblast none | 4.7 |
| PBMCPWM | 50.0 | Lung fibroblast TNF alpha + IL-1 beta | 6.1 |
| PBMC PHA-L | 19.3 | Lung fibroblast IL-4 | 10.2 |
| Ramos (B cell) none | 26.6 | Lung fibroblast IL-9 | 11.1 |
| Ramos (B cell) ionomycin | 49.0 | Lung fibroblast IL-13 | 8.8 |
| B lymphocytes PWM | 43.5 | Lung fibroblast IFN gamma | 11.0 |
| B lymphocytes CD40L and IL-4 | 14.6 | Dermal fibroblast CCD1070 rest | 17.8 |
| EOL-1 dbcAMP | 19.9 | Dermal fibroblast CCD1070 TNF alpha | 40.9 |
| EOL-1 dbcAMP PMA/ionomycin | 26.2 | Dermal fibroblast CCD1070 IL-1 beta | 8.6 |
| Dendritic cells none | 14.1 | Dermal fibroblast IFN gamma | 12.0 |
| Dendritic cells LPS | 113 | Dermal fibroblast IL-4 | 8.2 |
| Dendritic cells anti-CD40 | 11.2 | IBD Colitis 2 | 2.5 |
| Monocytes rest | 100.0 | IBD Crohn's | 3.1 |
| Monocytes LPS | 21.8 | Colon | 10.2 |
| Macrophages rest | 14.6 | Lung | 8.7 |
| Macrophages LPS | 13.0 | Thymus | 22.7 |
| HUVEC none | 17.7 | Kidney | 44.8 |
| HUVEC starved | 22.2 | | |

**Table AEE. Panel CNS_1**

| **Tissue Name** | **Rel. Exp.(%) Ag3120, Run 182257530** | **Tissue Name** | **Rel. Exp.(%) Ag3120, Run 182257530** |
|---|---|---|---|
| BA4 Control | 23.7 | BA17 PSP | 26.8 |
| BA4 Control2 | 40.9 | BA17 PSP2 | 12.2 |
| BA4 Alzheimer's2 | 3.1 | Sub Nigra Control | 50.3 |
| BA4 Parkinson's | 90.1 | Sub Nigra Control2 | 30.6 |
| BA4 Parkinson's2 | 73.2 | Sub Nigra Alzheimer's2 | 8.1 |
| BA4 Huntington's | 46.0 | Sub Nigra Parkinson's2 | 86.5 |
| BA4 Huntington's2 | 9.0 | Sub Nigra Huntington's | 76.8 |
| BA4 PSP | 8.7 | Sub Nigra Huntington's2 | 87.7 |
| BA4 PSP2 | 25.9 | Sub Nigra PSP2 | 15.1 |
| BA4 Depression | 31.4 | Sub Nigra Depression | 18.8 |
| BA4 Depression2 | 28.3 | Sub Nigra Depression2 | 16.2 |
| BA7 Control | 66.4 | Glob Palladus Control | 34.2 |
| BA7 Control2 | 64.6 | Glob Palladus Control2 | 12.5 |
| BA7 Alzheimer's2 | 11.5 | Glob Palladus Alzheimer's | 9.9 |
| BA7 Parkinson's | 26.8 | Glob Palladus Alzheimer's2 | 12.8 |
| BA7 Parkinson's2 | 49.3 | Glob Palladus Parkinson's | 95.9 |
| BA7 Huntington's | 50.7 | Glob Palladus Parkinson's2 | 15.4 |
| BA7 Huntington's2 | 66.4 | Glob Palladus PSP | 12.9 |
| BA7 PSP | 70.7 | Glob Palladus PSP2 | 10.1 |
| BA7 PSP2 | 23.7 | Glob Palladus Depression | 7.3 |
| BA7 Depression | 24.1 | Temp Pole Control | 14.9 |
| BA9 Control | 16.7 | Temp Pole Control2 | 39.2 |
| BA9 Control2 | 78.5 | Temp Pole Alzheimer's | 10.5 |
| BA9 Alzheimer's | 11.2 | Temp Pole Alzheimer's2 | 11.4 |
| BA9 Alzheimer's2 | 38.7 | Temp Pole Parkinson's | 30.8 |
| BA9 Parkinson's | 33.2 | Temp Pole Parkinson's2 | 40.3 |
| BA9 Parkinson's2 | 88.3 | Temp Pole Huntington's | 65.5 |
| BA9 Huntington's | 37.6 | Temp Pole PSP | 3.3 |
| BA9 Huntington's2 | 20.4 | Temp Pole PSP2 | 7.6 |
| BA9PSP | 23.3 | Temp Pole Depression 2 | 18.8 |
| BA9 PSP2 | 2.7 | Cing Gyr Control | 47.6 |
| BA9 Depression | 13.0 | Cing Gyr Control2 | 26.1 |
| BA9 Depression2 | 12.1 | Cing Gyr Alzheimer's | 22.2 |
| BA17 Control | 88.3 | Cing Gyr Alzheimer's 2 | 23.0 |
| BA17 Control2 | 82.9 | Cing Gyr Parkinson's | 43.2 |
| BA17 Alzheimer's2, | 35.1 | Cing Gyr Parkinson's2 | 37.4 |
| BA17 Parkinson's | 99.3 | Cing Gyr Huntingtpn's | 60.3 |
| BA17 Parkinson's2 | 100.0 | Cing Gyr Huntington's2 | 39.8 |
| BA17 Huntington's | 45.7 | Cing Gyr PSP | 38.2 |
| BA17 Huntington's2 | 40.3 | Cing Gyr PSP2 | 10.2 |
| BA17 Depression | 38.7 | CingGyr Depression | 21.5 |
| BA17 Depression2 | 48.3 | Cing Gyr Depression2 | 16.7 |

**CNS_neurodegeneration_v1.0 Summary:** Ag3120 This panel confirms the expression of this gene at moderate levels in the brain in an independent group of individuals. However, no differential expression of this gene was detected between Alzheimer's diseased postmortem brains and those of non-demented controls in this experiment. Please see Panel 1.3D for a discussion of the potential utility of this gene in treatment of central nervous system disorders.

**Panel 1.3D Summary:** Ag3120 The CG57151-O1 gene is expressed at moderate to low levels in all tissues on this panel, with the highest expression seen in ovarian cancer cell line OVCAR-5 (CT = 29.1). This gene is expressed at moderate levels throughout the central nervous system, including in amygdala, hippocampus, cerebral cortex, cerebellum, substantia nigra, and spinal cord, with lower expression detected in thalamus. Therefore, this gene may play a role in central nervous system disorders such as Alzheimer's disease, Parkinson's disease, epilepsy, multiple sclerosis, schizophrenia and depression.

Among tissues with metabolic or endocrine function, this gene is-pressed at low to moderate levels in adrenal gland, thyroid, pituitary gland, pancreas, adipose, skeletal muscle, heart and liver. Therefore, therapeutic modulation of the activity of this gene may prove useful in the treatment of endocrine and metabolically related diseases, such as obesity and diabetes.

This gene encodes a protein with homology to peptidyl-prolyl cis/trans isomerase A (PPIA or cyclophilin A), a cytosolic enzyme involved in protein folding and/or intracellular protein transport. Cyclophilin genes are expressed in numerous tissues. The interaction of cyclophilin and cyclosporine has an effect on cytokine production in T-cells. Therefore, modulation of this gene may be used in the treatment of inflamatory diseases such as rheumatoid arthritis.

### References:

### 1. Russell G, Graveley R, Seid J, al-Humidan AK, Skjodt H. Mechanisms of action of cyclosporine and effects on connective tissues. Semin Arthritis Rheum 1992 Jun;21(6 Suppl 3):16-22.

Cyclosporine is a potent immunomodulatory agent with an increasing number of clinical applications. Its major mode of action is inhibition of the production of cytokines involved in the regulation of T-cell activation. In particular, cyclosporine inhibits the transcription of interleukin 2. Although cyclosporine's major actions are on T cells, there is some evidence that it produces direct effects on other cell types. Its immunosuppressive action is closely linked to its binding of cyclophilin, a member of a family of high-affinity cyclosporine-binding proteins widely distributed in different cell types and in different species. The cyclophilins have been shown to have peptidyl-prolyl cis-trans isomerase enzyme activity that is blocked by cyclosporine. Although this may be a factor in cyclosporine's selective inhibition of cytokine gene transcription, it is still unclear whether inhibition of this activity is the mechanism through which cyclosporine exerts its effects on target cells. The ubiquitous presence of cyclophilins raises the question of why cyclosporine has major effects on T cells. Perhaps the critical proteins affected are transcriptional regulators restricted in their tissue distribution. The effects of cyclosporine on T cells and, directly or indirectly, on connective tissue cells, all of which can produce a range of cytokines, are of interest in relation to the tissue changes that occur in such inflammatory conditions as rheumatoid arthritis.

### PMID: 1502562

**Panel 4D Summary:** Ag3120 The CG57151-01 gene is expressed at high to moderate levels in a wide range of cell types of significance in the immune response in health and disease. These cells include members of the T-cell, B-cell, endothelial cen, macrophage/monocyte/dendritic cell, basophil, eosinophil, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues.

This pattern is in agreement with the expression profile in Panel 1.3D and also suggests a role for the gene product in cell survival and proliferation.

Therefore, therapeutic modulation of the activity of this gene or its protein product may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

**Panel CNS_1 Summary:** Ag3120 This panel confirms the expression of this gene at low to moderate levels in the brain in an independent group of individuals. Please see Panel 1.3D for a discussion of the potential utility of this gene in treatment of central nervous system disorders.

### NOV38

Expression of NOV38/CG57153-01 was assessed using the primer-probe set Ag3121, described in Table AFA. Results of the RTQ-PCR runs are shown in Tables AFB, AFC, AFD and AFE.

**Table AFA. Probe Name Ag3121**

| **Primers** | **Sequences** | **Length** | **Start Position** | **SEQ ID NO** |
|---|---|---|---|---|
| Forward | 5'-tccagggtttatatgccagagt-3' | 22 | 249 | 371 |
| Probe | TET-5'-cacatgtcatgatgacactggcacaa-3'-TAMRA | 26 | 279 | 372 |
| Reverse | 5'-cagacttctcccagtagttgga-3' | 22 | 307 | 373 |

**Table AFB. CNS_neurodegeneration_v1.0**

| **Tissue Name** | **Rel. Exp.(%) Ag3121, Run 208976899** | **Tissue Name** | **Rel. Exp.(%) Ag3121, Run 208976899** |
|---|---|---|---|
| AD 1 Hippo | 0.0 | Control (Path) 3 Temporal Ctx | 3.5 |
| AD 2 Hippo | 100.0 | Control (Path) 4 Temporal Ctx | 33.2 |
| AD 3 Hippo | 0.0 | AD 1 Occipital Ctx | 13.4 |
| AD 4 Hippo | 15.4 | AD 2 Occipital Ctx (Missing) | 0.0 |
| AD 5 Hippo | 5.0 | AD 3 Occipital Ctx | 0.0 |
| AD 6 Hippo | 66.4 | AD 4 Occipital Ctx | 7.7 |
| Control 2 Hippo | 32.5 | AD 5 Occipital Ctx | 3.8 |
| Control 4 Hippo | 17.8 | AD 6 Occipital Ctx | 3.1 |
| Control (Path) 3 Hippo | 0.0 | Control 1 Occipital Ctx | 0.0 |
| AD 1 Temporal Ctx | 0.0 | Control 2 Occipital Ctx | 0.0 |
| AD 2 Temporal Ctx | 18.0 | Control 3 Occipital Ctx | 0.0 |
| AD 3 Temporal Ctx | 0.0 | Control 4 Occipital Ctx | 0.0 |
| AD 4 Temporal Ctx | 14.0 | Control (Path) 1 Occipital | 6.5 |
| AD5 Inf Temporal Ctx | 28.3 | Control (Path) 2 Occipital Ctx | 12.7 |
| AD 5 Sup Temporal Ctx | 37.4 | Control (Path) 3 Occipital Ctx | 0.0 |
| AD 6 Inf Temporal Ctx | 10.8 | Control (Path) 4 Occipital Ctx | 0.0 |
| AD 6 Sup Temporal Ctx | 35.1 | Control 1 Parietal Ctx | 0.0 |
| Control 1 Temporal Ctx | 0.0 | Control 2 Parietal Ctx | 35.4 |
| Control 2 Temporal Ctx | 5.2 | ontrol 3 Parietal Ctx | 6.4 |
| Control 3 Temporal Ctx | 13.2 | Control (Path) 1 Parietal Ctx | 14.5 |
| Control 3 Temporal Ctx | 0.0 | Control (Path) 2 Parietal Ctx | 6.4 |
| Control (Path) Temporal Ctx | 19.8 | Control (Path) 3 Parietal Ctx | 0.0 |
| Control (Path) 2 Temporal Ctx | 28.9 | Control (Path) 4 Parietal Ctx | 6.5 |

**Table AFC. Panel 1.3D**

| **Tissue Name** | **Rel. Exp.(%) Ag3121, Run 167985269** | **Tissue Name** | **Rel. Exp.(%) Ag3121,Run 167985269** |
|---|---|---|---|
| Liver adenocarcinoma | 0.0 | Kidney (fetal) | 9.3 |
| Pancreas | 0.0 | Renal ca. 786-0 | 0.0 |
| Pancreatic ca. CAPAN 2 | 0.0 | Renal ca. A498 | 0.0 |
| Adrenal gland | 3.3 | Renal ca.RXF 393 | 0.0 |
| Thyroid | 0.0 | Renal ca. ACHN | 0.0 |
| Salivary gland | 0.0 | Renal ca. UO-31 | 0.0 |
| Pituitary gland | 0.0 | Renal ca. TK-10 | 0.0 |
| Brain (fetal) | 100.0 | Liver | 0.0 |
| -Brain (whole) | 0.0 | Liver (fetal) | 0.0 |
| Brain (amygdala) | 6.3 | Liver ca. (hepatoblast) HcpG2 | 0.0 |
| Brain(cerebellum) | 0.0 | Lung | 0.0 |
| Brain (hippocampus) | 11.3 | Lung (fetal) | 0.0 |
| Brain (substantia nigra) | 3.1 | Lung ca. (small cell) LX-1 | 0.0 |
| Brain (thalamus) | 0.0 | Lung ca. (small cell) NCI-H69 | 0.0 |
| Cerebral Cortex | 0.0 | Lung ca. (s.cell var.) SHP-77 | 0.0 |
| Spinal cord | 0.0 | Lung ca. (large cell)NCI-H460 | 0.0 |
| glio/astro U87-MG | 0.0 | Lung ca.(non-sm.cell) A549 | 0.0 |
| glio/astro U-118-MG | 0.0 | Lung ca. (non-s.cell) NCI-H23 | 0.0 |
| astrocytoma SW1783 | 0.0 | Lung ca.(non-s.cell) HOP-62 | 0.0 |
| neuro*;met SK-N-AS | 6.3 | Lung ca. (non-s.cl) NCI-H522 | 0.0 |
| astrocytoma SF-539 | 0.0 | Lung ca. (squam.) SW 900 | 0.0 |
| astrocytoma SNB-75 | 0.0 | Lung ca. (squam) NCI-H596 | 0.0 |
| glioma SNB-19 | 3.1 | Mammary gland | 0.0 |
| glioma U251 | 4.8 | Breast ca.* (pl.ef) MCF-7 | 0.0 |
| glioma SF-295 | 0.0 | Breast ca.*(pl.ef) MDA- | 0.0 |
| Heart (fetal) | 0.0 | Breast ca.*(pl.ef) T47D | 0.0 |
| Heart | 0.0 | Breast ca. BT-549 | 0.0 |
| Skeletal muscle (fetal) | 0.0 | Breast ca. MDA-N | 0.0 |
| Skeletal muscle | 0.0 | Ovary | 0.0 |
| Bone marrow | 0.0 | Ovarian ca. OVCAR-3 | 0.0 |
| Thymus | 0.0 | Ovarian ca. OVCAR-4 | 0.0 |
| Spleen | 0.0 | Ovarian ca. OVCAR-5 | 0.0 |
| Lymph node | 0.0 | Ovarian ca. OVCAR-8 | 2.5 |
| Colorectal | 0.0 | Ovarian ca. IGROV-1 | 0.0 |
| Stomach | 0.0 | Ovarian ca.* (ascites) SK-OV-3 | 0.0 |
| Small intestine | 0.0 | Uterus | 0.0 |
| Colon ca. SW480 | 0.0 | Plancenta | 0.0 |
| Colon ca.* SW620(SW480 met) | 0.0 | Prostate | 0.0 |
| Colon ca. HT29 | 0.0 | Prostate ca.* (bone met)PC-3 | 0.0 |
| Colon ca. HCT-116 | 0.0 | Testis | 0.0 |
| Colon ca. CaCo-2 | 0.0 | Melanoma Hs688(A).T | 0.0 |
| Colon ca. tissue(ODO3866) | 4.7 | Melanoma* (met) Hs688(B).T | 0.0 |
| Colon ca. HCC-2998 | 0.0 | Melanoma UACC-62 | 0.0 |
| Gastric ca.* (liver met) NCI-N87 | 0.0 | Melanoma M14 | 0.0 |
| Bladder | 0.0 | Melanoma LOX IMVI | 0.0 |
| Trachea | 0.0 | Melanoma* (met) SK-MEL-5 | 0.0 |
| Kidney | 0.0 | Adipose | 0.0 |

**Table AFD. Panel 4D**

| **Tissue Name** | **Rel. Exp.(%) Ag3121, Run 164526517** | **Tissue Name** | **Rel. Exp.(%) Ag3121, Run 164526517** |
|---|---|---|---|
| Secondary Th1 act | 0.0 | HUVEC IL-1 beta | 0.0 |
| Secondary Th2 act | 0.0 | HUVEC IFN gamma | 0.0 |
| Secondary Tr1 act | 0.0 | HUVEC TNF alpha + IFN gamma | 9.7 |
| Secondary Th1 rest | 0.0 | HUVEC TNF alpha+ IL4 | 0.0 |
| Secondary Th2 rest | 0.0 | HUVEC IL-1 | 0.0 |
| Secondary Tr1 rest | 0.0 | Lung Microvascular EC none | 0.0 |
| Primary Th1 act | 0.0 | Lung Microvascular BCTN Falpha +IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 33.0 |
| Primary Th2 rest | 0.0 | Small airway epithelium none | 0.0 |
| Primary Tr1 rest | 0.0 | Small airway epithelium TNFalpha +IL-1beta | 0.0 |
| CD45RA CD4 lymphocyte act | 0.0 | Coronery artery SMC rest | 0.0 |
| CD45RO CD4 lymphocyte act | 0.0 | Coronery artery SMC TNFalpha+ IL-lbeta | 0.0 |
| CD8 lymphocyte act | 0.0 | Astrocytes rest | 0.0 |
| Secondary CD8 lymphocyte rest | 0.0 | Astrocytes TNFalpha+IL-1beta | 0.0 |
| Secondary CD8 lymphocyte act | 0.0 | KU-812 (Basophil) rest | 0.0 |
| CD4 lymphocyte none | 0.0 | KU-812 (Basophil) PMA/ionomycin | 0.0 |
| 2ry Th1/Th2/Tr1_anti-CD95 CH11 | 0.0 | CCD1106 (Keratinocytes) none | 0.0 |
| LAK cells rest | 0.0 | CCD1106(Keratinocytes) TNFalpha+IL-1beta | 0.0 |
| LAK cells IL-2 | 0.0 | Liver cirrhosis | 100.0 |
| LAK cells IL-2+IL-12 | 0.0 | Lupus kidney | 0.0 |
| LAK cells IL-2+IFN gamma | 0.0 | NCI-H292 none | 0.0 |
| LAK cells IL-2+IL-18 | 0.0 | NCI-H292 IL-4 | 0.0 |
| LAK cells PMA/ionomycin | 0.0 | NCI-H292 IL-9 | 0.0 |
| NK Cells IL-2 rest | 0.0 | NCI-H292 IL-13 | 0.0 |
| Two Way MLR 3 day | 0.0 | NCI-H292 IFN gamma | 0.0 |
| Two Way MLR 5 day | 0.0 | HPAEC none | 0.0 |
| Two Way MLR 7 day | 0.0 | HPAEC TNF alpha+IL-1 beta | 0.0 |
| PBMC rest | 0.0 | Lung fibroblast none | 0.0 |
| PBMC PWM | 0.0 | Lung fibroblast TNF alpha + IL-1 beta | 10.9 |
| PBMC PHA-L | 0.0 | Lung fibroblast IL-4 | 22.2 |
| Ramos (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 0.0 | Lung fibroblast IFN gamma | 0.0 |
| B lymphocytes CD40L and IL-4 | 0.0 | Dermal fibroblast CCD1070 rest | 0.0 |
| EOL-1 dbcANP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 0.0 | Dermal fibroblast IL-4 | 37.6 |
| Dendritic cells anti-CD40 | 0.0 | IBD Colitis2 | 0.0 |
| Monocytes rest | 0.0 | IBD Crohn's | 0.0 |
| Monocytes LPS | 0.0 | Colon | 28.9 |
| Macrophages rest | 0.0 | Lung | 0.0 |
| Macrophages LPS | 0.0 | Thymus | 0.0 |
| HUVEC none | 0.0 | Kidney | 0.0 |
| HUVEC starved | 0.0 | | |

**Table AFE. Panel CNS_1**

| **Tissue Name** | **Rel. Exp.(%) Ag3121, Run 171694170** | **Rel. Exp.(%) Ag3121, Run 182257570** | **Tissue Name** | **Rel. Exp.(%) Ag3121, Run 171694170** | **Rel. Exp.(%) Ag3121, Run 182257570** |
|---|---|---|---|---|---|
| BA4 Control | 24.0 | 57.0 | BA17 PSP | 0.0 | 0.0 |
| BA4 Control2 | 49.0 | 0.0 | BA17PSP2 | 0.0 | 0.0 |
| BA4 Alzheimrr's2 | 0.0 | 33.2 | Sub Nigra Control | 0.0 | 25.0 |
| BA4 Parkinson's | 33.0 | 72.7 | Sub Nigra Control2 | 0.0 | 0.0 |
| BA4 Parkinson's2 | 0.0 | 27.9 | Sub Nigra Alzbeimer's2 | 27.9 | 0.0 |
| BA4 Hunrington's | 11.4 | 0.0 | Sub Nigra Parkinson's2 | 0.0 | 0.0 |
| BA4 Huntington's2 | 0.0 | 0.0 | Sub Nigra Huntington's | 0.0 | 82.9 |
| BA4 PSP | 0.0 | 21.8 | Sub Nigra Huntington's2 | 0.0 | 0.0 |
| BA4 PSP2 | 26.8 | 0.0 | Sub Nigra PSP2 | 0.0 | 0.0 |
| BA4 Depression | 8.1 | 30.1 | Sub Nigra Depression | 0.0 | 0.0 |
| BA4 Depression2 | 0.0 | 0.0 | Sub Nigra Depression2 | 0.0 | 0.0 |
| BA7 Control | 0.0 | 0.0 | Glob Palladus Control | 0.0 | 0.0 |
| BA7 Control2 | 29.3 | 0.0 | Glob Palladus Control2 | 0.0 | 0.0 |
| BA7 Alzheimer's2 | 0.0 | 0.0 | Glob Palladus Alzheimer's | 24.8 | 0.0 |
| BA7 Parkinson's | 0.0 | 0.0 | Glob Palladus Alzheimer's2 | 0.0 | 0.0 |
| BA7 Parkinson's2 | 0.0 | 0.0 | Glob Palladus Parkinson's | 82.4 | 58.2 |
| BA7 Huntington's | 29.7 | 19.9 | Glob Palladus Parkinson's2 | 27.4 | 0.0 |
| BA7 Huntington's2 | 28.5 | 0.0 | Glob Palladus PSP | 0.0 | 24.1 |
| BA7 PSP | 0.0 | 0.0 | Glob Palladus PSP2 | 0.0 | 0.0 |
| BA7 PSP2 | 0.0 | 0.0 | Glob Palladus Depression | 0.0 | 26.8 |
| BA7 Depression | 0.0 | 0.0 | Temp Pole Control | 21.3 | 33.0 |
| BA9 Control | 0.0 | 0.0 | Temp Pole Control2 | 27.7 | 0.0 |
| BA9 Control2 | 100.0 | 36.1 | Temp Pole Alzheimer's | 0.0 | 0.0 |
| BA9 Alzheimer's | 0.0 | 0.0 | Temp Pole Alzheimer's2 | 0.0 | 0.0 |
| BA9 Alzheimer's2 | 24.7 | 0.0 | Temp Pole Parkinson's | 0.0 | 31.6 |
| BA9 Parkinson's | 0.0 | 26.2 | Temp Pole Parkinson's2 | 50.3 | 0.0 |
| BA9 Parkinson's2 | 0.0 | 30.6 | Temp Pole Huntington's | 0.0 | 0.0 |
| BA9 Huntington's | 0.0 | 0.0 | Temp Pole PSP | 0.0 | 0.0 |
| BA9 Huntington's2 | 0.0 | 29.1 | Temp Pole PSP2 | 0.0 | 0.0 |
| BA9 PSP | 0.0 | 100.0 | Temp Pole Depression2 | 0.0 | 0.0 |
| BA9 PSP2 | 0.0 | 0.0 | Cing Gyr Control | 0.0 | 94.6 |
| BA9 Depression | 0.0 | 0.0 | Cing Gyr Control2 | 0.0 | 30.8 |
| BA9 Depression2 | 0.0 | 0.0 | Cing Gyr Alzheimer's | 0.0 | 0.0 |
| BA17 Control | 0.0 | 37.9 | Cing Gyr Alzheimer's2 | 0.0 | 0.0 |
| BA17 Control2 | 45.1 | 0.0 | Cing Gyr Parkinson's | 28.3 | 29.3 |
| BA17 Atzheimer's2 | 0.0 | 0.0 | Cing Gyr Parkinson's2 | 0.0 | 91.4 |
| BA17 Parkinson's | 0.0 | 27.7 | Cing Gyr Huntington's | 14.2 | 28.7 |
| BA17 Parkinson's2 | 0.0 | 43.8 | Cing Gyr Huntington's2 | 90.1 | 41.8 |
| BA17 Huntingtan's | 24.8 | 0.0 | Cing Gyr PSP | 28.1 | 29.9 |
| BA17 Huntingt n's2 | 0.0 | 0.0 | Cing Gyr PSP2 | 0.0 | 0.0 |
| BA17 Depression | 0.0 | 0.0 | Cing Gyr Depression | 0.0 | 31.2 |
| BA17 Depression2 | 0.0 | 29.1 | Cing Gyr Depression2 | 0.0 | 0.0 |

**CNS_neurodegeneration_v1.0 Summary:** Ag3121 This panel confirms the expression of this gene at low levels in the brain in an independent group of individuals. However, no differential expression of this gene was detected between Alzheimer's diseased postmortem brains and those of non-demented controls in this experiment. Please see Panel 1.3D for a discussion of the potential utility of this gene in treatment of central nervous system disorders.

**Panel 1.3D Summary:** Ag3121 Significant expression of the CG57153-01 gene is restricted to a sample derived from fetal brain tissue (CT = 33.1). Interestingly, expression of this gene is much lower in adult brain (CT = 40), suggesting that expression of this gene can be used to distinguish fetal brain from adult brain as well as the other samples on this panel. In addition, the relative overexpression of this gene in fetal brain suggests that the protein product may enhance brain development in the fetus and thus may also act in a regenerative capacity in the adult. Therefore, therapeutic modulation of the cyclophilin encoded by this gene could be useful in treatment of neurodegenerative diseases, such as Alzheimer's disease and Parkinson's disease.

**Panel 4D Summary:** Ag3121 Significant expression of this gene is detected in a liver cirrhosis sample (CT = 33.64). Furthermore, expression of this gene is not detected in normal liver in Panel 1.3D, suggesting that its expression is unique to liver cirrhosis. This gene encodes a putative cyclophilin; therefore, small molecule therapeutics designed against this protein could reduce or inhibit fibrosis that occurs in liver cirrhosis. In addition, antibodies to this protein could also be used for the diagnosis of liver cirrhosis.

**Panel CNS_1 Summary:** Ag3121 Expression of this gene is low/undetectable (CTs > 35) across all of the samples on this panel (data not shown).

### NOV39

Expression of NOV39./G57155-01 was assessed using the primer-probe set Ag3122, described in Table AGA. Results of the RTQ-PCR runs are shown in Tables AGB, AGC, AGD and AGE.

**Table AGA. Probe Name Ag3122**

| **Primers** | **Sequences** | **Length** | **Start Position** | **SEQ ID NO** |
|---|---|---|---|---|
| Forward | 5'-agcaacgggtcacaattctata-3' | 22 | 355 | 374 |
| Probe | TET-5'-tcacactgcaagcaactccttatctaga-3'-TAMRA | 28 | 377 | 375 |
| Reverse | 5'-atacccaaaagccacaaatttt-3' | 22 | 408 | 376 |

**Table AGB. GNS_neurodegeneration_v1.0**

| **Tissue Name** | **Rel. Exp.(%) Ag3122, Run 208976900** | **Tissue Name** | **Rel. Exp.(%) Ag3122, Run 208976900** |
|---|---|---|---|
| AD 1 Hippo | 30.4 | Control (Path) 3 Temporal Ctx | 9.7 |
| AD 2 Hippo | 33.4 | Control (Path) 4 Temporal Ctx | 28.5 |
| AD 3 Hippo | 22.1 | AD 1 Occipital Ctx | 12.2 |
| AD 4 Hippo | 10.2 | AD 2 Occipital Ctx (Missing) | 0.0 |
| AD 5 hippo | 100.0 | AD 3 Occipital Ctx | 11.7 |
| AD 6 Hippo | 59.5 | AD 4 Occipital Ctx | 16.2 |
| Control 2 Hippo | 33.4 | AD 5 Occipital Ctx | 22.5 |
| Control 4 Hippo | 27.9 | AD 6 Occipital Ctx | 48.6 |
| Control (Path) 3 Hippo | 14.9 | Control 1 Occipital Ctx | 3.1 |
| AD 1 Temporal Ctx | 23.0 | Control 2 Occipital Ctx | 42.6 |
| AD 2 Temporal Ctx | 0.0 | Control 3 Occipital Ctx | 17.6 |
| AD 3 Temporal Ctx | 10.4 | Control 4 Occipital Ctx | 12.6 |
| AD 4 Temporal Ctx | 22.5 | Control (Path) 1 Occipital Ctx | 83.5 |
| AD 5 Inf Temporal Ctx | 86.5 | Control (path) 2 Occipital Ctx | 13.4 |
| AD 5 SupTemporal Ctx | 57.8 | Control (Path) 3 Occipital Ctx | 4.9 |
| AD 6 Inf Temporal Ctx | 55.1 | Control (Path) 4 Occipital Ctx | 12.2 |
| AD 6 Sup Temporal Ctx | 80.7 | Control 1 Parietal Ctx | 6.5 |
| Control Temporal Ctx | 5.9 | Control 2 Parietal Ctx | 45.7 |
| Control 2 Temporal Ctx | 43.8 | Control 3 Parietal Ctx | 22.5 |
| Control 3 Temporal Ctx | 14.0 | Control (Path) 1 Parietal Ctx | 64.6 |
| Control 4 Temporal Ctx | 10.1 | Control (Path) 2 Parietal Ctx | 31.9 |
| Control (Path) 1 Temporal Ctx | 73.2 | Control (Path) 3 Parietal Ctx | 11.0 |
| Control (Path) 2 Temporal Ctx | 33.7 | Control (Path) 4 Parietal Ctx | 48.3 |

**Table AGC. Panel 1.3D**

| **Tissue Name** | **Rel. Exp.(%) Ag3122, Run 167985270** | **Tissue Name** | **Rel. Exp.(%) Ag3122, Run 167985270** |
|---|---|---|---|
| Liver adenocarcinoma | 5.7 | Kidney (fetal) | 100.0 |
| Pancreas | 13.2 | Renal ca. 786-0 | 20.6 |
| Pancreatic ca. CAPAN 2 | 0.0 | Renal ca. A498 | 22.5 |
| Adrenal gland | 0.0 | Renal ca. RXF 393 | 22.1 |
| Thyroid | 40.1 | Renal ca. ACHN | 28.3 |
| Salivary gland, | 0.0 | Renal ca. UO-31 | 5.0 |
| Pituita land | 19.1 | Renal ca. TK-10 | 9.5 |
| Brain (fetal) | 88.9 | Liver | 0.0 |
| Brain (whole) | 11.0 | Liver (fetal) | 0.0 |
| Brain (amygdala) | 73.7 | Liver ca. (hepatoblast) HepG2 | 7.2 |
| Brain (cerebellum) | 45.7 | Lung | 8.5 |
| Brain (hippocampus) | 33.0 | Lung (fetal) | 77.4 |
| Brain (substantia nigra) | 27.7 | Lung ca. (small cell) LX-1 | 23.5 |
| Brain (thalamus) | 11.5 | Lung ca. (small cell) NCI-H69 | 20.6 |
| Cerebral Cortex | 12.2 | Lung ca. (s.cell var.) SHP-77 | 56.6 |
| Spinal cord | 55.9 | Lung ca. (large cell)NCI-H460 | 0.0 |
| glio/astro U87-MG | 9.7 | Lung ca. (non-sm. cell) A549 | 34.9 |
| glio/astro U-118-MG | 2.5 | Lung ca. (non-s.cell) NCI- H23 | 40.3 |
| astrocytoma SW1783 | 3.5 | Lung ca. (non-s-cell) HOP-62 | 8.4 |
| neuro*; met SK-N-AS | 6.1 | Lung ca. (non-s.el) NCI-H522 | 33.2 |
| astrocytoma SF-539 | 5.3 | Lung ca. (squam.) SW 900 | 14.9 |
| astrocytoma SNB-75 | 12.2 | Lung ca. (squam.) NCI-H596 | 39.8 |
| glioma SNB-19 1 | 11.3 | Mammary gland | 0.0 |
| glioma U251 | 13.1 | Breast ca.* (pl.ef) MCF-7 | 0.0 |
| glioma SF-295 | 36.1 | Breast ca.* (pl.et) MDA-MB-231 | 6.9 |
| Heart (fetal) | 20.6 | Breast ca.* (pl.ef) T47D | 77.9 |
| Heart | 0.0 | Breast ca. BT-549 | 3.7 |
| Skeletal muscle (fetal) | 14.5 | Breast ca. MDA-N | 2.8 |
| Skeletal muscle | 22.2 | Ovary | 13.0 |
| Bone marrow | 0.0 | Ovarian ca. OVCAR-3 | 12.0 |
| Thymus | 12.5 | Ovarian ca. OVCAR-4 | 6.9 |
| Spleen | 3.6 | Ovarian ca. OVCAR-5 | 29.3 |
| Lymph node | 17.8 | Ovarian ca. OVCAR-8 | 2.7 |
| Colorectal | 4.9 | Ovarian ca. IGROV-1 | 0.0 |
| Stomach | 0.0 | Ovarian ca.* (ascites) SK-OV-3 | 39.8 |
| Small intestine | 6.2 | Uterus | 18.4 |
| Colon ca. SW480 | 0.0 | Placenta | 0.0 |
| Colon ca.* SW620(SW480 met) | 13.0 | Prostate | 4.1 |
| Colon ca. HT29 | 5.1 | Prostate ca.* (bone met)PC-3 | 8.4 |
| Colon ca. HCT-116 | 0.0 | Testis | 73.7 |
| Coton ca.CaCo-2 | 0.0 | Melanoma Hs688(A).T | 0.0 |
| Colon ca. tissue(OD03866) | 17.3 | Melanoma* Hs688(B).T (met) | 0.0 |
| Colon ca.HCC-2998 | 25.7 | Melanoma UACC-62 | 2.1 |
| Gastric.ca.* (liver met) NCI-N87 | 35.4 | Melanoma M14 | 5.6 |
| Bladder | 14.2 | Melanoma LOX IMVI | 0.0 |
| Trachea | 57.4 | Melanoma* (met) SK MBL-5 | 0.0 |
| Kidney | 59.0 | Adipose | 3.6 |

**Table AGD. Panel 4D**

| **Tissue Name** | **Rel. Exp.(%) Ag3122, Run 164527262** | **Tissue Name** | **Rel. Exp.(%) Ag3122, Run 164527262** |
|---|---|---|---|
| Secondary Th1 act | 0.0 | UVEC IL-1beta | 0.0 |
| Secondary Th2 act | 1.4 | HUVEC IFN gamma | 3.1 |
| Secondary Tr1 act | 0.0 | HUVEC TNF alpha+IFN gamma | 3.8 |
| Secondary Th1 rest | 0.0 | HUVEC TNF alpha+IL4 | 1.1 |
| Secondary Th2 rest | 0.0 | HUVEC IL-11 | 0.0 |
| Secondary Tr1 rest | 0.0 | Lung Microvascular EC none | 3.2 |
| Primary Th1act | 1.6 | Lung Microvascular EC TNFalpha +IL-1beta | 2.2 |
| Primary Th2 act | 3.1 | Microvascular Dermal EC none | 0.4 |
| Primary Tr1 act | 0.0 | Microsvasular Dermal EC TNFalpha + IL-1beta | 1.6 |
| Primary Th1 rest | 1.4 | Bronchial epithelium TNFalpha + IL1beta | 10.4 |
| Primary Th2 rest | 0.0 | Small airway epithelium none | 2.1 |
| Primary Tr1 rest | 0.0 | Small airway epithelium TNFalpha +IL-1beta | 7.4 |
| CD45RA CD4 lymphocyte act | 0.5 | Coronery artery SMC rest | 1.5 |
| CD4SRO CD4 lymphocyte act | 0.0 | Coronery artery SMC TNFalpha + IL-1beta | 0.8. |
| CD8 lymphocyte act | 1.9 | Astrocytes rest | 6.3 |
| Secondary CD8 lymphocyte rest | 3.3 | Astrocytes TNFalpha + IL-1beta | 3.3 |
| Secondary CD8 lymphocyte act | 1.8 | KU-812 (Basophil) rest | 7.9 |
| CD4 lymphocyte none | 0.0 | KU-812 PMA/ionomycin (Basophil) | 15.5 |
| 2ry Th1/Th2/Tr1_anti-CD95 CH11 | 1.5 | CCD1106 (Keratinocytes) none | 1.4 |
| LAK cells rest | 0.8 | CCD1106 (Keratinocytes) TNFalpha+IL-1beta | 0.0 |
| LAK cells IL-2 | 0.6 | Liver cirrhosis | 2.9 |
| LAK cells IL-2+IL-12 | 4.5 | Lupus Kidney | 2.2 |
| LAK cells IL-2+IFN gamma | 1.9 | NCI-H292 none | 4.1 |
| LAK cells IL-2+IL-18 | 2.0 | NCI-H292 IL-4 | 4.8 |
| LAK cells PMA/ionomycin | 0.0 | NCI-H292 IL-9 | 9.9 |
| NK Cells IL-2 rest | 100.0 | NCI-H292 IL-13 | 4.5 |
| Two Way MLR 3 day | 7.7 | NCI-H292 IFN gamma | 1.6 |
| Two Way MLR 5 day | 0.0 | HPAEC none | 0.6 |
| Two Way MLR 7 day | 0.0 | HPABC TNF alpha + IL-1 beta | 1.0 |
| PBMC rest | 2.8 | Lung fibroblast none | 3.2 |
| PBMCPWM | 4.5 | Lung fibroblast TNF alpha + IL-1 beta. | 1.4 |
| PBMCPHA-L | 0.9 | Lung fibroblast IL-4 | 5.8 |
| Ramos (B cell) none | 0.0 | Lung fibroblast IL-9 | 1.1 |
| Ramos (B cell) ionomycin | 8.0 | Lung fibroblast IL-13 | 3.8 |
| B lymphocytes PWM | 9.2 | Lung fibroblast IFN gamma | 13.8 |
| B lymphocytes CD40L and IL-4 | 2.5 | Dermal fibroblast CCD1070 rest | 1.2 |
| EOL-1 dbcAMP | 0.0 | Dermal fibroblast CCD1070 TNF | 3.2 |
| EOL-1 dbcAMP PMA/ionomycin | 3.0 | Dermal fibroblast CCD1070 IL-1 beta | 2.5 |
| Dendritic cells none | 1.0 | Dermal fibroblast IFN gamma | 1.2 |
| Dendritic cells LPS | 2.5 | Dermal fibroblast IL-4 | 5.8 |
| Dendritic cells anti-M40 | 0.7 | IBD Colitis 2 | 1.4 |
| Monocytes rest | 1.6 | IBD Crohn's | 3.2 |
| Monocytes LPS | 1.4 | Colon | 4.8 |
| Macrophages rest | 2.9 | Lung | 11.9 |
| Macrophages LPS | 0.0 | Thymus | 59.9 |
| HUVEC none | 0.0 | Kidney | 6.1 |
| HUVEC starved | 1.3 | | |

**Table AGE. Panel CNS_1**

| **Tissue Name** | **Rel. Exp. (%) Ag3122, Run 182257573** | **Tissue Name** | **Rel. Exp.(%) Ag3122, Run 182257573** |
|---|---|---|---|
| BA4 Control | 47.0 | BA17 PSP | 19.1 |
| BA4 Control2 | 81.2 | BA17 PSP2 | 9.2 |
| BA4 Alzheimer's2 | 0.0 | Sub Nigra Control | 27.7 |
| BA4 Parkinson's | 53.6 | Sub Nigra Control2 | 14.2 |
| BA4 Parkinson's2 | 72.7 | Sub Nigra Alzheimer's2 | 16.4 |
| BA4 Huntington's | 47.0 | Sub Nigra Parkinson's2 | 24.0 |
| BA4 Huntington's2 | 8.2 | Sub Nigra Huntington's | 100.0 |
| BA4PSP | 7.7 | Sub Nigra Huntington's2 | 36.1 |
| BA4 PSP2 | 7.5 | Sub Nigra PSP2 | 15.9 |
| BA4 Depression | 24.3 | Sub Nigra Depression | 16.3 |
| BA4 Depression2 | 15.3 | Sub Nigra Depression2 | 0.0 |
| BA7 Control | 29.5 | Glob Palladus Control | 18.2 |
| BA7 Control2 | 24.5 | Glob Palladus Control2 | 99.3 |
| BA7 Alzheimer's2 | 24.0 | Glob Palladus Alzheimer's2 | 0.0 |
| BA7 Parkinson's | 20.9 | Glob Palladus Alzheimer's2 | 8.7 |
| BA7 Parkinson's2 | 29.3 | Glob Palladus Parldnson's | 100.0 |
| BA7 Huntington's | 31.9 | Glob Palladus Parkinson's2 | 15.8 |
| BA7 Huntington's2 | 68.8 | Glob Palladus PSP | 7.2 |
| BA7 PSP | 11.0 | Glob Palladus PSP2 | 0.0 |
| BA7 PSP2 | 7.5 | Glob Palladus Depression | 9.7 |
| BA7 Depression | 11.3 | Temp Pole Control | 12.3 |
| BA9 Control | 24.3 | Temp Pole Control2 | 32.5 |
| BA9 Control2 | 44.4 | Temp Pole Alzheimer's | 11.9 |
| BA9 Alzheimer's | 8.8 | Temp Pole Alzheimer's2 | 0.0 |
| BA9 Alzheimer's2 | 19.3 | Temp Pole Parkinson's | 37.1 |
| BA9 Parkinson's | 28.5 | Temp Pole Parkinson's2 | 30.1 |
| BA9 Parkinson's2 | 30.4 | Temp Pole Huntington's | 28.1 |
| BA9 Huntington's | 54.7 | Temp Polc PSP | 14.7 |
| BA9 Huntington's2 | 17.4 | Temp Pole PSP2 | 0.0 |
| BA9 PSP | 0.0 | Temp Pole Depression2 | 19.8 |
| BA9 PSP2 | 7.4 | Cing Gyr Control | 49.0 |
| BA9 Depression | 12.6 | Cing Gyr Control2 | 19.3 |
| BA9 Depression2 | 0.0 | Cing Gyr Alzheimer's | 11.0 |
| BA17 Control | 50.7 | Cing Gyr Alzheimer's2 | 6.3 |
| BA17 Control2 | 35.8 | Cing Gyr Parkinso's | 39.8 |
| BA17 Alzheimer's2 | 9.0 | CingGyrParkinson's2 | 31.6 |
| BA17 Parkinson's | 38.4 | Cing Gyr Huntington's | 59.5 |
| BA17Partdnson's2 | 60.7 | Cing Gyr Huntington's2 | 13.5 |
| BA17 Huntington's | 19.6 | CingGyrPSP | 26.4 |
| BA17 Huntington's2 | 34.4 | Cing Gyr PSP2 | 0.0 |
| BA17 Depression | 14.4 | Cing Gyr Depression | 0.0 |
| BA17 Depression2 | 24.0 | Cing Gyr Depression2 | 12.5 |

**CNS_neurodegeneration_v1.0 Summary:** Ag3122 This panel confirms the expression of this gene at low levels in the brain in an independent group of individuals. However, no differential expression of this gene was detected between Alzheimer's diseased postmortem brains and those of non-demented controls in this experiment. Please see Panel 1.3D for a discussion of the potential utility of this gene in treatment of central nervous system disorders.

**Panel 1.3D Summary:** Ag3122 Expression of the CG57155-01 gene is highest in fetal kidney (CT = 34.3). In addition, low but significant expression of this gene is detected in fetal brain (CT = 34.5) and fetal lung (CT = 34.6). Interestingly, expression of the CG57155-01 gene is much lower in adult brain (CT =37.5) and adult lung (CT = 37.8). This observation suggests that expression of this gene can be used to distinguish fetal brain from adult brain as well as fetal lung from adult lung. In addition, the relative overexpression of this gene in fetal brain suggests that the protein product may enhance brain development in the fetus and thus may also act in a regenerative capacity in the adult. Therefore, therapeutic modulation of the cyclophilin encoded by this gene could be useful in treatment of neurodegenerative diseases.

**Panel 4D Summary:** Ag3122 Expression of the CG57155-01 gene is highest in natural killer cells (CT = 31). This observation suggests that therapeutic modulation of this gene using small molecule drugs could be of use in the treatment of viral or bacterial intracellular infections.

In addition, this gene is expressed at significant levels in thymus (CT = 31.8). The putative cyclophilin encoded for by the CG57155-01 gene could therefore play an important role in T cell development. Small molecule therapeutics, or antibody therapeutics designed against the protein encoded for by this gene could be utilized to modulate immune function (T cell development) and be important for organ transplant, AIDS treatment or post chemotherapy immune reconstitiution.

This gene encodes a protein with homology to peptidyl-prolyl cis/trans isomerase A (PPIA or cyclophilin A), a cytosolic enzyme involved in protein folding and/or intracellular protein transport. Cyclophilin genes have been shown to be expressed in numerous tissues. The interaction of cyclophilin and cyclosporine has an effect on cytokine production in T-cells. Therefore, modulation of this gene may be used in the treatment of inflamatory diseases such as rheumatoid arthritis.

### References:

### 1. Russell G, Graveley R, Seid J, al-Humidan AK, Skjodt H. Mechanisms of action of cyclosporine and effects on connective tissues. Semin Arthritis Rheum 1992 Jun;21(6 Suppl 3):16-22.

Cyclosporine is a potent immunomodulatory agent with an increasing number of clinical applications. Its major mode of action is inhibition of the production of cytokines involved in the regulation ofT-cell activation. In particular, cyclosporine inhibits the transcription of interleukin 2. Although cyclosporine's major actions are on T cells, there is some evidence that it produces direct effects on other cell types. Its immunosuppressive action is closely linked to its binding of cyclophilin, a member of a family of high-affinity cyclosporine-binding proteins widely distributed in different cell types and in different species. The cyclophilins have been shown to have peptidyl-prolyl cis-trans isomerase enzyme activity that is blocked by cyclosporine. Although this may be a factor in cyclosporine's selective inhibition of cytokine gene transcription, it is still unclear whether inhibition of this activity is the mechanism through which cyclosporine exerts its effects on target cells. The ubiquitous presence of cyclophilins raises the question of why cyclosporine has major effects on T cells. Perhaps the critical proteins affected are transcriptional regulators restricted in their tissue distribution. The effects of cyclosporine on T cells and, directly or indirectly, on connective tissue cells, all of which can produce a range of cytokines, are of interest in relation to the tissue changes that occur in such inflammatory conditions as rheumatoid arthritis.

### PMID: 1502562

**Panel CNS_1 Summary:** Ag3122 This panel confirms the expression of this gene at low levels in the brain in an independent group of individuals. Please see Panel 1.3D for a discussion of the potential utility of this gene in treatment of central nervous system disorders.

### NOV40

Expression of NOV40/CG57157-01 was assessed using the primer-probe set Ag3123, described in Table AHA. Results of the RTQ-PCR runs are shown in TablesAHB.

**Table AHA. Probe Name Ag3123**

| **Primers** | **Sequences** | **Length** | **Start Position** | **SEQ ID NO** |
|---|---|---|---|---|
| Forward | 5'-gtgagactggcaagaagatcac-3' | 22 | 497 | 377 |
| Probe | 'TET-5'-ctgecaactgeggacaactctaatea-3'-TAMRA | 26 | 521 | 378 |
| Reverse | 5'-tggtaatcaaacaraagca-3' | 22 | 550 | 379 |

**Table AHB. Panel 4D**

| **Tissue Name** | **Rel. Exp.(%) Ag3123, Run 164527712** | **Tissue Name** | **Rel. Exp.(%) Ag3123, Run 164527712** |
|---|---|---|---|
| Secondary Th1 act | 9.3 | HUVEC IL-1beta | 9.4 |
| Secondary Th2 act | 1.5 | HUVEC IFN gamma | 25.7 |
| Secondary Tr1 act | 10.2 | HUVEC TNF alpha + IFN gamma | 0.9 |
| Secondary Th1 test | 1.1 | HUVEC TNF alpha + IL4 | 18.0 |
| Secondary Th2 rest | 3.5 | HUVEC IL-11 | 52.1 |
| Secondary Tr1 rest | 1.4 | Lung Microvascular EC none | 28.9 |
| Primary Th1 act | 15.3 | Lung Microvascular EC TNFalpha +IL-1beta | 18.9 |
| Primary Th2 act | 6.5 | Microvascular Dermal EC none | 50.3 |
| Primary Tr1 act | 30.8 | Microsvasular Dermal EC TNFalpha+IL-1beta | 18.8 |
| Primary Th1 rest | 23.0 | Bronchial epithelium TNFalpha + IL1beta | 11.7 |
| Primary Th2 rest | 9.7 | Small airway epithelium none | 4.7 |
| Primary Tr1 rest | 5.4 | Small airway epithelium TNFalpha +IL-1beta | 27.7 |
| CD45RA CD4 lymphocyte act | 2.4 | Coronery artery SMC rest | 15.9 |
| CD4SRO CD4 lymphocyte act | 5.9 | Coronery artery SMC TNFalpha + IL-1beta | 8.2 |
| CD8 lymphocyte act | 1.1 | Astrocytes rest | 38.2 |
| Secondary CD8 lymphocyte rest | 4.9 | Astrocytes TNFalpha + IL1beta | 8.0 |
| Secondary CD8 lymphocyte act | 3.5 | KU-812 (Basophil) rest | 57.0 |
| CD4 lymphocyte none | 0.0 | KU-812 (Basophil) PMA/ionomycin | 89.5 |
| 2ry Th1/Th2/Tr1_anti-CD95 CH1 | 4.8 | CCD1106 (Keratinocytes) none | 24.5 |
| LAK cells rest | 1.2 | CCD1106 (Keratinocytes) TNFalpha + IL-1beta | 14.4 |
| LAK cells IL-2 | 1.4 | Liver cirrhosis | 19.1 |
| LAK cells IL-2+IL-12 | 0.9 | Lupus kidney | 10.0 |
| LAK cells IL-2+IFN gamma | 1.7 | NCI-H292 none | 43.5 |
| LAK cells IL-2+ IL-18 | 1.1 | NCI-H292 IL-4 | 52.9 |
| LAK cells PMA/ionomycin | 0.0 | NCI-H292 IL-9 | 51.8 |
| NK Cells IL-2 rest | 3.6 | NCI-H292 IL-13 | 29.1 |
| Two Way MLR 3 day | 0.0 | NCI-H292 IFN gamma | 19.2 |
| Two Way MLR 5 day | 0.0 | HPAEC none | 24.5 |
| Two Way MLR 7 day | 4.8 | HPAEC TNF alpha + IL-1beta | 3.5 |
| PBMC rest | 0.0 | Lung fibroblast none | 4.6 |
| PBMC PWM | 10.4 | Lung fibroblast TNF alpha + IL-1 beta | 1.2 |
| PBMC PHA-L | 26.2 | Lung fibroblast IL-4 | 7.0 |
| Ramos (B cell) none | 14.0 | Lung fibroblast IL-9 | 21.6 |
| Ramos (B cell) ionomycin | 34.9 | Lung fibroblast IL-13 | 5.9 |
| B lymphocytes PWM | 30.1 | Lung fibroblast IFN gamma | 3.3 |
| B lymphocytes CD40L and IL-4 | 12.8 | Dermal fibroblast CCD1070 rest | 38.4 |
| EOL-1 dbcAMP | 3.5 | Dermal fibroblast CCD1070 TNF alpha | 63.3 |
| EOL-1 dbcAMP PMA/ionomycin | 0.9 | Dermal fibroplast CCD 1070 IL-1 beta | 15.9 |
| Dendritic cells none | 2.1 | Dermal fibroblast IFN gamma | 10.4 |
| Dendritic cells LPS | 1.0 | Derma fibroplast IL-4 | 8.4 |
| Dendritic cells anti-CD40 | 0.0 | IBD Colitis 2 | 1.7 |
| Monocytes rest | 0.0 | IBD Crohn's | 1.9 |
| Monocytes LPS | 1.3 | Colon | 7.1 |
| Macrophagesrest | 4.6 | Lung | 17.6 |
| Macrophages LPS | 0.0 | Thymus | 61.6 |
| HWEC none | 17.8 | Kidney | 30.4 |
| HUVEC starved | 100.0 | | |

**Panel 1.3D** Summary: Ag3123 Results from one experiment with the CG57157-01 gene are not included. The amp plot indicates that there were experimental difficulties with this run (data not shown).

**Panel 4D Summary:** Ag3123 The CG57157-01 gene is expressed at low levels in T cells and is also expressed at significant levels in endothelium, epithelium and fibroblasts as well as in normal thymus and kidney. Furthermore, treatment of proinflammatory mediators reduces the level of this transcript in HUVEC cells as compared to in starved cells. The protein encoded for by this transcript may be important in the normal function of the cell types that express it. Regulation of the protein encoded for by this transcript could be important for maintaining or resuming normal homeostasis.

### NOV41

Expression ofNOV41/CG57159-01 was assessed using the primer-probe set Ag3320, described in Table AIA. Results of the RTQ-PCR runs are shown in Tables AIB and AIC.

**Table AIA. Probe Name Ag3320**

| Primers | Sequences | Length | Start Position | SEQ ID NO |
|---|---|---|---|---|
| Forward | 5'-ttggtgataaatgtccctgc-3' | 20 | 161 | 380 |
| Probe | TET-5'-ggggtgtgtcagggtggtgacttc-3'-TAMRA | 24 | 199 | 381 |
| Reverse | 5'-ccagtgccattatggtgtgt-3' | 20 | 223 | 382 |

**Table AIB. General_screening_panel_v1.4**

| **Tissue Name** | **Rel. Exp.(%) Ag3320, Run 215775833** | **Tissue Name** | **Rel. Exp.(%) Ag3320, Run 215775833** |
|---|---|---|---|
| Adipose | 0.0 | Renal ca. TK-10 | 0.0 |
| Melanoma* Hs688(A).T | 0.0 | Bladder | 0.0 |
| Melanoma* Hs688(B).T | 0.0 | Gastric ca. (liver met.) NCI-N87 | 0.0 |
| Melanoma* M14 | 0.0 | Gastric ca. KATO III | 0.0 |
| Melanoma* LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| Testis Pool | 0.0 | Colon ca. HT29 | 0.0 |
| Prostate ca.* (bone met) PC-3 | 0.0 | Colon ca. HCT-116 | 0.0 |
| Prostate Pool | 0.0 | Colon ca. CaCo-2 | 0.0 |
| Placenta | 0.0 | Colon cancer tissue | 0.0 |
| Uterus Pool | 0.0 | Colon ca. SW1116 | 0.0 |
| Ovarian ca. OVCAR-3 | 0.0 | Colon ca. Colo-205 | 0.0 |
| Ovarian ca. SK-OV-3 | 0.0 | Colon ca. SW-48 | 0.0 |
| Ovarian ca. OVCAR-4 | 0.0 | Colon Pool | 0.0 |
| Ovarian ca. OVCAR-5 | 0.0 | Small Intestine Pool | 0.0 |
| Ovarian ca. IGROV-1 | 0.0 | Stomach Pool | 0.1 |
| Ovarian ca. OVCAR-8 | 0.0 | Bone Marrow Pool | 0.0 |
| Ovary | 0.0 | Fetal Heart | 0.0 |
| Breast ca. MCF-7 | 0.0 | Heart Pool | 0.3 |
| Breast ca. MDA-MB-231 | 0.0 | Lymph Node Pool | 0.2 |
| Breast ca. BT 549 | 0.0 | Fetal Skeletal Muscle | 0.0 |
| Breast ca. T47D | 0.0 | Skeletal Muscle Pool | 0.0 |
| Breast ca. MDA-N | 0.0 | Spleen Pool | 0.0 |
| Breast Pool | 0.0 | Thymus Pool | 0.0 |
| Trachea | 0.0 | CNS cancer (glio/astro) U87- MG | 0.0 |
| Lung | 0.6 | CNS cancer (glio/astro) U-118-MG | 0.0 |
| Fetal Lung | 0.0 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 0.1 | CNS cancer (glio) SNB-19 | 0.0 |
| Lungca.SHP-77 | 0.0 | CNS cancer (glio) SF-295 | 0.0 |
| Lung ca. A549 | 0.0 | Brain (Amygdala) Pool | 0.0 |
| Lung ca. NCI-H526 | 0.0 | Brain (cerebellum) | 0.0 |
| Lung ca. NCI-H23 | 0.0 | Brain (fetal) | 0.0 |
| Lung ca. NCI-H460 | 0.0 | Brain (Hippocampus) Pool | 0.0 |
| Lung ca. HOP-62 | 0.0 | Cerebral Cortex Pool | 0.0 |
| Lun ca. NCI H522 | 0.0 | Brain (Substantia nigra) Pool | 0.0 |
| Liver | 0.0 | Brain (Thalamus) Pool | 0.0 |
| Fetal Liver | 0.0 | Brain (whole) | 0.0 |
| Liver ca. HepG2 | 0.0 | Spinal Cord Pool | 0.0 |
| Kidney Pool | 1.6 | Adrenal Gland | 0.0 |
| Fetal Kidney | 0.0 | Pituitary gland Pool | 0.0 |
| Renal ca. 786-0 | 0.0 | Salivary Gland | 0.0 |
| Renal ca. A498 | 0.0 | Thyroid (female) | 0.1 |
| Renal ca. ACHN | 0.0 | Pancreatic ca. CAPAN2 | 0.0 |
| Renal ca. UO-31 | 0.0 | Pancreas Pool | 100.0 |

**CNS_neurodegeneration_v1.0 Summary:** Ag3320 Expression of this gene is low/undetectable (CTs > 35) across all of the samples on this panel (data not shown).

**General_screening_panel_v1.4 Summary:** Ag3320 Expression of the CG57159-01 gene is restricted to a sample derived from pancreatic tissue (CT = 27.6). Thus, expression of this gene could be used to distinguish pancreas from the other samples in the panel. In addition, therapeutic modulation of the activity of this gene or its protein product may prove useful in the treatment of metabolically related diseases, such as obesity and diabetes.

**Panel 4D Summary:** Ag3320 Expression of this gene is low/undetectable (CTs > 35) across all of the samples on this panel (data not shown).

### NOV42

Expression of gene NOV42A/CG57226-01 and NOV42B/CG57226-02 was assessed using the primer-probe set Ag3142, described in Table AJA. Results of the RTQ-PCR runs are shown in Tables AJB, AJC and AJD. Please note that CG57226-02 represents a full-length physical clone of the CG57226-01 gene, validating the prediction of the gene sequence.

**Table AJA. Probe Name Ag3142**

| **Primers** | **Sequences** | **Length** | **Start Position** | **SEQ ID NO** |
|---|---|---|---|---|
| Forward | 5'-caaaacaaatgagtcccagttt-3' | 22 | 504 | 383 |
| Probe | TET-5'-atctgcactgccatggccaaatg-3'-TAMRA | 23 | 529 | 384 |
| Reverse | 5'-ctgccaaagateacatgett-3' | 20 | 562 | 385 |

**Table AJB. CNS_neurodegeneration_v1.0**

| **Tissue Name** | **Rel. Exp.(%) Ag3142, Run 209055796** | **Tissue Name** | **Rel. Exp.(%) Ag3142, Run 209055796** |
|---|---|---|---|
| AD 1 Hippo | 14.4 | Control (Path) 3 Temporal Ctx | 3.4 |
| AD 2 Hippo | 25.0 | Control (Path) 4 Temporal Ctx | 71.7 |
| AD 3 Hippo | 10.8 | AD 1 Occipital Ctx | 27.9 |
| AD 4 Hippo | 19.8 | AD 2 Occipital Ctx (Missing) | 0.0 |
| AD 5 hippo | 82.9 | AD 3 Occipital Ctx | 13.1 |
| AD 6 Hippo | 58.2 | AD 4 Occipital Ctx | 29.5 |
| Control 2 Hippo | 30.1 | AD 5 Occipital Ctx | 15.0 |
| Control 4 Hippo | 12.2 | AD 6 Occipital Ctx | 31.0 |
| Hippo Control (Path) 3 | 9.0 | Control 1 Occipital Ctx | 7.1 |
| AD 1 Temporal Ctx | 49.0 | Control 2 Occipital Ctx | 32.5 |
| AD 2 Temporal Ctx | 28.5 | Control 3 Occipital Ctx | 0.0 |
| AD 3 Temporal Ctx | 11.3 | Control 4 Occipital Ctx | 11.4 |
| Ctx AD 4 Temporal | 47.0 | Control (Path)1 Ctx Occipital Ctx | 100.0 |
| AD 5 Inf Temporal Ctx | 89.5 | Control (path) 2 Occipital Ctx | 26.1 |
| AD 5 Sup Temporal Ctx | 49.7 | Control (Path) 3 Occipital Ctx | 1.8 |
| AD6 Inf Temporal Ctx | 54.3 | Control (Path) 4 Occipital Ctx | 36.3 |
| AD 6 Sup Temporal Ctx | 69.3 | Control 1 Parietal Ctx | 9.0 |
| Control 1 | 11.5 | Ctx Control 2 Parietal | 57.0 |
| Control 2 Temporal Ctx | 20.3 | Control 3 Parietal | 13.6 |
| Control 3 Temporal Ctx | 21.3 | Control (Path) 1 Parietal Ctx | 54.7 |
| Control 4 Temporal Ctx | 11.0 | Parietal Ctx Control (Path) 2 | 34.4 |
| Control (Path) 1 Temporal Ctx | 66.0 | Control (Path) 3 Parietal Ctx | 9.0 |
| Control (Path) 2 Temporal Ctx | 55.1 | Control (Path) 4 Parietal Ctx | 65.5 |

**Table AJC. Panel 1.3D**

| **Tissue Name** | **Rel. Exp.(%) Ag3142, Run 167994809** | **Tissue Name** | **Rel. Exp.(%) Ag3142, Run 167994809** |
|---|---|---|---|
| Liver adenocarcinoma | 16.6 | Kidney (fetal) | 20.2 |
| Pancreas | 6.8 | Renal ca. 786-0 | 6.6 |
| Pancreatic ca. CAPAN 2 | 5.7 | Renal ca. A498 | 8.7 |
| Adrenal gland | 3.6 | Renal ca. RXF 393 | 5.7 |
| Thyroid | 2.7 | Renal ca. ACHN | 2.4 |
| Salivary gland | 9.0 | Renal ca. UO-31 | 1.1 |
| Pituitary gland | 9.7 | Renal ca. TK-10 | 18.3 |
| Brain (fetal) | 16.7 | Liver | 11.3 |
| Brain (whole) | 12.8 | Liver (fetal) | 8.3 |
| Brain (amygdala) | 12.2 | Liver ca. (hepatoblast) HepG2 | 11.0 |
| Brain (cerebellum) | 27.0 | Lung | 2.3 |
| Brain (hippocampus) | 13.9 | Lung (fetal) | 10.4 |
| Brain (substantia nigra) | 16.5 | LX-1 Lung ca. (small cell) | 16.6 |
| Brain (thalamus) | 4.7 | NCI-H69 Lung ca. (small cell) | 14.3 |
| Cerebral Cortex | 5.1 | SHP-77 Lung ca. (s.cell var.) | 78.5 |
| Spinal cord | 3.3 | Lung ca. (large cell)NCI-H460 | 2.5 |
| glio/astro U87-MG | 13.5 | cell)A549 Lung ca. (non-sm. | 25.0 |
| glio/astro U-118-MG | 11.1 | NCI-H23 Lung ca.(non-s.cell) | 12.4 |
| SW1783 astmcytoma | 8.8 | HOP-62 Lung ca. (non-s.cell) | 16.0 |
| AS euro*; met SK-N- | 28.7 | Lung ca.(non-s.cl) NCI-HS22 | 12.6 |
| astmcytoma SF-539 | 10.1 | SW 900 Lung ca. (squam.) | 16.5 |
| astrocytoma SNB-75 | 13.2 | NCI-HS9 Lung ca.(squam.) | 48.6 |
| glioma SNB-19 | 30.4 | Mammary gland | 2.1 |
| glioma U251 | 36.6 | Breast ca.* (pl.ef) MCF-7 | 14.7 |
| glioma SF-295 | 13.6 | Breast ca.*(pl.ef) MDA-MB-231 | 14.1 |
| Heart (fetal) | 0.6 | T47D Breast ca.* (pl.ef) | 57.8 |
| Heart | 8.7 | Breast ca. BT-549 | 4.9 |
| Skeletal muscle (fetal) | 2.5 | Breast ca. MDA-N | 7.5 |
| Skeletal muscle | 2.9 | Ovary | 1.3 |
| Bone marrow | 8.0 | Ovarian ca. OVCAR-3 | 23.0 |
| Thymus | 20.6 | Ovarian ca. OVCAR-4 | 4.2 |
| Spleen | 7.6 | Ovarian ca. OVCAR-5 | 100.0 |
| Lymph node | 26.6 | Ovarian ca. OVCAR-8 | 4.9 |
| Colorectal | 20.3 | Ovarian ca. IGROV-1 | 3.0 |
| Stomach | 9.5 | Ovarian ca.* (ascites) SK-OV-3 | 66.4 |
| Small intestine | 15.6 | Uterus | 7.3 |
| Colon ca. SW480 | 11.4 | Placenta | 0.0 |
| Colon ca.* SW620(SW480 met) | 59.5 | Prostate | 4.0 |
| Colon ca. HT29 | 18.3 | Prostate ca.* (bone met)PG3 | 12.2 |
| Colon ca. HCT-116 | 14.8 | Testis | 3.1 |
| Colon ca. CaCo-2 | 26.8 | Melanoma Hs688(A).T | 3.9 |
| Colon ca. tissue(ODO3866) | 6.9 | Melanoma* (met) Hs688(B).T | 9.4 |
| Colon ca. HCC-2998 | 28.7 | 62 Melanoma UACC- | 3.2 |
| Gastric ca.* (liver met) NCI-N87 | 25.2 | Melanoma M14 | 2.9 |
| Bladder | 51.4 | Melanoma LOX IMVI | 0.0 |
| Trachea | 2.4 | SK-MEL-5 Melanoma* (met) | 6.6 |
| Kidney | 11.4 | Adipose | 21.8 |

**Table AJD. Panel 4D**

| **Tissue Name** | **Rel.Exp.(%) Ag3142, Run 164527962** | **Tissue Name** | **Rel. Exp.(%) Ag3142, Run 164527962** |
|---|---|---|---|
| Secondary Th1 act | 30.8 | HUVEC IL-1beta | 11.5 |
| Secondary Th2 act | 30.1 | HUVEC IFN gamma | 12.8 |
| Secondary Tr1 act | 34.6 | HUVEC TNF alpha + IFN | 14.1 |
| Secondary Th1 rest | 7.1 | HUVEC TNF alpha+IL4 | 7.6 |
| Secondary Th2 rest | 20.4 | HUVEC IL- 11 | 7.7 |
| Secondary Tr1 rest | 21.0 | Lung Microvascular EC none none | 14.9 |
| Primary Th1 act | 32.1 | TNFalpha + IL-1beta | |
| Primary Th2 act | 46.3 | Microvascular Dermal EC none | 17.9 |
| Primary Tr1 act | 47.3 | Microsvasular Dermal EC TNFalpha + IL-1beta | 11.3 |
| Primary Th1 rest | 76.8 | Bronchial epithelium TNFalpha + IL1beta | 10.4 |
| Primary Th2 rest | 42.9 | none Small airway epithelium | 1.3 |
| Primary Tr1 rest | 42.3 | Small airway epithelium TNFalpha + IL-1beta | 21.3 |
| CD45RA CD4 lymphocyte act | 15.4 | Coronery artery SMC rest | 6.7 |
| CD45RO CD4 lymphocyteact | 31.2 | Coronery artery SMC TNFalpha + IL-1beta | 4.5 |
| CD8 lymphocyte act | 25.5 | Astrocytes rest | 11.0 |
| Secondary CD8 lymphocyte rest | 27.4 | Astrocytes TNFalpha+ IL-1beta | 5.0 |
| Secondary CD8 lymphocyte act | 17.8 | KU-812 (Basophil) rest | 25.5 |
| CD4 lymphocyte none | 28.9 | KU-812 (Basophil) PMA/ionomycin | 61.1 |
| 2ry Th1/Th2/Tr1_anti-CD95 CH11 | 32.5 | none CCD1106 (Keratinocytes) none | 14.4 |
| LAK cells rest | 29.1 | CCD1106 (Keratinocytes) TNFalpha + IL-1beta | 0.5 |
| LAK cells IL-2 | 42.3 | Liver cirrhosis | 4.5 |
| LAK cells IL-2+IL- 12 | 31.4 | Lupus kidney | 6.7 |
| LAK cells IL-2+IFN gamma | 64.6 | NCI-H292 none | 46.7 |
| LAK cells IL-2+ IL- 18 | 81.8 | NCI-H292 IL-4 | 68.3 |
| LAK cells PMA/ionomycin | 20.3 | NCI.H292 IL-9 | 46.0 |
| NK Cells IL-2 rest | 28.9 | NCI-H292 IL-13 | 24.5 |
| Two Way MLR 3 day | 46.7 | NCI-H292 IFN gamma | 35.8 |
| Two Way MLR 5 day | 12.0 | HPAEC none | 10.2 |
| Two Way MLR 7 day | 7.9 | beta HPAEC TNF alpha+ IL-1: beta | 9.4 |
| PBMC rest | 13.5 | Lung fibroblast none | 8.2 |
| PBMC PWM | 81.8 | Lung fibroblast TNF alpha + IL-1 beta | 10.4 |
| PBMCPHA-L | 29.7 | Lung fibroblast IL-4 | 17.6 |
| Ramos (B cell) none | 37.4 | Lung fibroblast IL-9 | 17.8 |
| Ramos (B cell) ionomycin | 100.0 | Lung fibroblast IL-13 | 11.8 |
| B lymphocytes PWM | 66.4 | gamma Lung fibroblast IFN | 14.3 |
| B lymphocytes CD40L and IL-4 | 67.4 | Dermal fibroblast CCD1070 rest | 20.9 |
| EOL-1 dbcAMP | 20.9 | Dermal fibroblast CCD1070 TNF alpha | 73.7 |
| EOL-1 dbcAMP PMA/ionomycin | 22.4 | Dermal fibroblast CCD1070 IL-1 beta | 14.0 |
| Dentritic cells none | 11.6 | Dermal fibroblast IFN gamma | 9.0 |
| Dendritic cells LPS | 11.3 | Dermal fibroblast IL4 | 17.0 |
| Dendritic cells anti-CD40 | 19.8 | EBD Colitis 2 | 2.8 |
| Monocytes rest | 19.1 | IBD Crohn's | 5.2 |
| Monocytes LPS | 18.9 | Colon | 20.9 |
| Macrophages rest | 11.1 | Lung | 8.3 |
| Macrophages LPS | 11.7 | Thymus | 29.3 |
| HUVEC none | 10.0 | Kidney | 51.1 |
| HWEC starved | 23.3 | | |

**CNS_neurodegeneration_v1.0 Summary:** Ag3142 This panel confirms the expression of this gene at moderate to low levels in the brain in an independent group of individuals. However, no differential expression of this gene was detected between Alzheimer's diseased postmortem brains and those of non-demented controls in this experiment. Please see Panel 1.3D for a discussion of the potential utility of this gene in treatment of central nervous system disorders.

**Panel 1.3D Summary:** Ag3142 The CG57226-01 gene is expressed at low to moderate levels in the majority of samples on this panel, with highest expression in ascites derived ovarian cancer cell line OVCAR-5 (CT=30.4). It is also expressed in melanoma, prostate, breast, lung, renal, gastric, colon, neuroblastoma, pancreatic and liver adenocarcinoma cell lines. Hence, this gene is likely to play a role in cell survival and proliferation.

Among tissues with metabolic or endocrine function, this gene is expressed in pituitary gland, liver, adipose, and pancreas. Therefore, therapeutic modulation of the activity of this gene may prove useful in the treatment of endocrine/metabolically related diseases, such as obesity and diabetes.

This gene is expressed at low levels throughout the CNS, including in amygdala, hippocampus, substantia nigra, thalamus, cerebellum, and cerebral cortex. Therefore, this gene may play a role in central nervous system disorders such as Alzheimer's disease, Parkinson's disease, epilepsy, multiple sclerosis, schizophrenia and depression.

**Panel 4D Summary:** Ag3142 The CG57226-01 gene is expressed at moderate levels in a wide range of cell types of significance in the immune response in health and disease. These cells include members of the T-cell, B-cell, endothelial cell, macrophage/monocyte/dendritic cell, basophil, eosinophil and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues.

This pattern is in agreement with the expression profile in Panel 1.3D and also suggests a role for the gene product in cell survival and proliferation.

Therefore, therapeutic modulation of the activity of this gene or its protein product may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### NOV43

Expression of NOV43/CG57538-01 was assessed using the primer-probe set Ag3277, described in Table AKA. Results of the RTQ-PCR runs are shown in Table AKB.

**Table AKA. Probe Name Ag3277**

| **Primers** | **Sequences** | **Length** | **Start** Position | **SEQ ID NO** |
|---|---|---|---|---|
| Forward | 5'-caaccctggtcaaataattcaa-3' | 22 | 2424 | 386 |
| Probe | TBT-5'-aaaataaagccgcaagaecgtattct-3'-TAMRA | 26 | 2456 | 387 |
| Reverse | 5'-ttttcactccatgagcatgaat-3' | 22 | 2482 | 388 |

**Table AKB. Panel 4D**

| **Tissue Name** | **Rel. Exp.(%) Ag3277, Run 164635110** | **Tissue Name** | **Rel.Exp.(%) Ag3277, Run 164635110** |
|---|---|---|---|
| Secondary Th1 act | 0.0 | HUVEC IL-1 beta | 0.0 |
| Secondary Th2 act | 0.0 | HUVEC IFN gamma | 2.6 |
| Secondary Tr1 act | 0.0 | HUVEC TNF alpha + IFN gamma | 8.7 |
| Secondary Th1 rest | 2.8 | HUVEC TNF alpha + ILA | 2.3 |
| SecondaryTh2 rest | 0.0 | HUVEC IL-11 | 0.0 |
| Secondary Tr1 rest | 0.0 | Lung Microvascutar EC none | 4.0 |
| Primary Th1 act | 0.0 | Lung Microvascular EC TNFalpha + IL-1 beta | 4.5 |
| Primary Th2 act | 0.0 | Microvascular Dermal EC none | 6.4 |
| Primary Tr1 act | 0.0 | Microsvasular Dermal EC TNFalpha + IL-1beta | 0.0 |
| Primary Th1 rest | 0.0 | Bronchial epithelium TNFalpha + IL 1beta | 0.0 |
| Primary Th2 rest | 6.7 | Small airway epithelium none | 0.0 |
| Primary Tr1 rest | 0.0 | Small airway epithelium TNFalpha + IL-1 beta | 7.6 |
| CD45RA CD4 lymphocyte act | 0.0 | Coronery artery SMC rest | 0.0 |
| CD45RO CD4 lymphocyte act | 5.1 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CDB lymphocyte act | 0.0 | KU-812 (Basophil) rest | 0.0 |
| CD4 lymphocyte none | 0.0 | PMA/ionomycin | 44.8 |
| 2ry Th1/Th2/Tr1_anti-CD95 CH11 | 0.0 | CCD1106 (Keratinocytes) none | 0.0 |
| LAK cells rest | 0.0 | CCD1106 (Keratinocytes) TNFalpha + IL-1beta | 0.0 |
| LAK cells IL-2 | 0.0 | Liver cirrhosis | 6.1 |
| LAK cell IL-2+12 | 0.0 | Lupus kidney | 4.1 |
| LAK cells IL-2+IFN gamma | 0.0 | NCI-H292 none | 14.5 |
| LAK cells IL-2+ IL-18 | 0.0 | NCI-H292 IL-4 | 100.0 |
| LAK cells PMA/ionomycin | 0.0 | NCI-H292IL-9 | 27.4 |
| NK Cells IL-2 rest | 17.4 | NCI-H292IL-13 | 37.4 |
| Two Wa MLR 3 day | 0.0 | NCI-H292 IFN gamma | 8.2 |
| Two Way MLR 5 day | 4.7 | HPAEC none | 0.0 |
| Two Way MLR 7 day | 0.0 | HPAEC TNF alpha + IL-1 beta | 0.0 |
| PBMC rest | 0.0 | Lung fibroblast none | 0.0 |
| PBMC PWM | 0.0 | Lung fibroblast 1NF alpha + IL-1 beta | 0.0 |
| PBMC PHA-L | 3.9 | Lung fibroblast IL-4 | 0.0 |
| RaTms (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 0.0 | Lung fibrobast IFN gamma | 0.0 |
| B lymphocytes CD40L and IL-4 | 0.0 | Dermal fibroblast CCD1070 rest | 0.0 |
| EOL-1 dbcAMP | 0.0 | Dermal fibroblast CCD1070 TNF alpha | 9.5 |
| EOL-1 dbcAMP PMA/ionomycin | 12.2 | Dermal fibroblast CCD1070 IL-1 beta | 0.0 |
| Dendritic cells none | 0.0 | Dermal fibroblast IFN gamma | 0.0 |
| Dendritic cells LPS | 2.3 | Dermal fibroblast IL-4 | 0.0 |
| Dendritic cells anti-CD40 | 0.0 | IBD Colitis 2 | 0.0 |
| Monocytes rest | 0.0 | IBD Crohn's | 0.0 |
| Monocytes LPS | 5.2 | Colon | 0.0 |
| Macrophages rest | 0.0 | Lung | 3.2 |
| Macrophages LPS | 0.0 | Thymus | 0.0 |
| HUVEC none | 4.2 | Kidney | 15.1 |
| HUVEC starved | 0.0 | | |

**CNS_neurodegeneration- v1.0 Summary:** Ag3277 Expression of this gene is low/undetectable (CTs > 35) across all of the samples on this panel (data not shown).

**General_screening_panel-v1.4 Summary:** Ag3277 Results from one experiment with the CG57538-01 gene are not included. The amp plot indicates that there were experimental difficulties with this run (data not shown).

**Panel 4D Summary:** Ag3277 Highest expression of the CG57538-01 gene, a Ceruloplasmin homolog, is in samples derived from the pulmonary mucoepidermoid cell line NCI-H292 stimulated with IL-4 (CTs=34.4). Thus, this expression profile indicates that this gene product may play a key role as a mediator of inflammation, especially in late-phase allergic reactions, and as a mediator of local cellular movement or trafficking into the inflamed area by cytokines and chemokines. Airway epithelial cells are believed to be the major source of Ceruloplasmin in the lung fluid and may play critical role in host defense against oxidative damage and infection in the lung (ref. 1).

### References:

### 1. Yang F, Friedrichs WE, deGraffenried L, Herbert DC, Weaker FJ, Bowman BH, Coalson JJ. Cellular expression of ceruloplasmin in baboon and mouse lung during development and inflammation. Am J Respir Cell Mol Biol 1996 Feb;14(2):161-9

Abstract - Ceruloplasmin (CP) is an important extracellular antioxidant and free radical scavenger. Although CP is expressed mainly in the liver, recent studies have identified the lung as another major site of CP synthesis. The sites and cell types that are responsible for CP expression in baboon and mouse lung are described. CP mRNA is detected in primordial bronchial epithelium in baboon fetuses by 60 days of gestation. At 140 days of gestation and thereafter, CP mRNA is found in airway epithelium and in the ductal cells of the submucosal glands. In developing and mature mice, CP mRNA is present in epithelial cells throughout the airway. In endotoxin-treated mice, the amount of CP mRNA increases several-fold in large airways but increases only moderately in small airways. This suggests that the high concentration of CP in the mucus lining of the upper airway, which serves to filter harmful substances, is particularly important during stressful conditions. Endotoxin treatment in mice also results in the induction of high levels of CP mRNA in a subset of alveolar wall cells. The data suggest that the airway epithelial cells are the major source of CP in the lung fluid and support ceruloplasmin's critical role in host defense against oxidative damage and infection in the lung.

### NOV44

Expression of NOV44A/CG57623-01 and NOV44B/CG57623-02 was assessed using the primer-probe set Ag3296, described in Table AKA. Results of the RTQ-PCR runs are shown in Tables AKB, AKC and AKD. Please note that CG57623-02 represents a full-length physical clone of the CG57623-01 gene, validating the prediction of the gene sequence.

**Table ALA. Probe Name Ag3296**

| **Primers** | **Sequences** | **Length** | **Start Position** | **SEQ ID NO** |
|---|---|---|---|---|
| Forward | 5'-agaccatctttcccaatctgat-3' | 22 | 737 | 389 |
| Probe | TET-5'-caattccatggccaaggactcctg-3'-TAMRA | 24 | 765 | 390 |
| Reverse | 5'-gttccaagtcacctccagtctt-3' | 22 | 791 | 391 |

**Table ALB. CNS_neurodegeneratio_v1.0**

| **Tissue Name** | **Rel. Exp.(%) Ag3296, Run 210063000** | **Tissue Name** | **Rel. Exp(%) Ag3296, Run 210063000** |
|---|---|---|---|
| AD 1 Hippo | 19.5 | Control (Path) 3 Temporal Ctx | 9.8 |
| AD 2 Hippo | 3.0 | Control (Path) 4 Temporal Ctx | 4.3 |
| AD 3 Hippo | 6.5 | AD 1 Occipital Ctx | 10.9 |
| AD 4 Hippo | 39.5 | AD 2 Occipital Ctx (Missing) | 0.0 |
| AD 5 Hippo | 26.6 | AD 3 Occipital Ctx | 10.4 |
| AD 6 Hippo | 84.7 | AD 4 Occipital Ctx | 6.5 |
| Control 2 Hippo | 10.8 | AD 5 Occipital Ctx | 5.1 |
| Control 4 Hippo | 33.7 | AD 6 Occipital Ctx | 0.0 |
| Control (Path) 3 Hippo | 16.4 | Control 1 Occipital Ctx | 12.6 |
| AD 1 Temporal Ctx | 18.3 | Control 2 Occipital Ctx | 7.4 |
| AD 2 Temporal Ctx | 4.7 | Control 3 Occipital Ctx | 0.0 |
| AD 3 Temporal Ctx | 4.3 | Control 4 Occipital Ctx | 4.2 |
| AD 4 Temporal Ctx | 15.8 | Control (Path) 1 Occipital Ctx | 12.5 |
| AD 5 Inf Tempora Ctx | 100.0 | Control (Path) 2 Occipital Ctx | 0.0 |
| AD 5 Sup Temporal Ctx | 83.5 | Control (Path)3 Occipital Ctx | 2.3 |
| AD 6 Inf Temporal Ctx | 39.0 | Control (Path) 4 Occipital Ctx | 0.0 |
| AD 6 Sup Temporal Ctx | 18.0 | Control 1 Parietal Ctx | 12.6 |
| Control 1 Temporal Ctx | 0.0 | Control 2 Parietal Ctx | 13.2 |
| Control 2 Temporal Ctx | 13.5 | Control 3 Parietal Ctx | 9.7 |
| Control 3 Temporal Ctx | 4.7 | Control (Path) 1 Parietal Ctx | 24.5 |
| Control 3 Temporal Ctx | 15.8 | Control (Path) 2 Parietal Ctx | 0.0 |
| Control (Path) 1 Temporal Ctx | 16.3 | Control (Path) 3 Parietal Ctx | 0.0 |
| Control (Path) 2 Temporal Ctx | 10.3 | Control (Path) 4 Parietal Ctx | 3.3 |

**Table ALC. Generat_screening_panel_v1.4**

| **Tissue Name** | **Rel. Exp.(%) Ag3296, Run 215669667** | **Tissue Name** | **Rel. Exp.(%) Ag3296, Run 215669667** |
|---|---|---|---|
| Adipose | 6.3 | Renal ca. TK-10 | 0.0 |
| Melanoma* Hs688(A).T | 0.0 | Bladder | 25.7 |
| Melanoma* Hs688(B).T | 0.0 | Gastric ca.(liver met.) NCI-N87 | 0.0 |
| Melanoma* M14 | 0.0 | Gastric ca. KATO III | 0.0 |
| Melanoma* LOXIMVI | 0.0 | Colon ca. SW-948 | 0.0 |
| Melanoma* SK-MEL-5 | 1.2 | Colon ca. SW480 | 0.0 |
| Squamous cell carcinoma SCC-4 | 0.0 | Colon ca.* (SW480 met) SW620 | 0.0 |
| Testis Pool | 47.6 | Colon ca.HT29 | 0.0 |
| Prostate ca.* (bone met) PC-3 | 0.0 | Colon ca.HCT-116 | 0.0 |
| Prostate Pool | 4.1 | Colon ca. CaCo-2 | 0.0 |
| Placenta | 1.1 | Colon cancer tissue | 12.9 |
| Uterus Pool | 3.0 | Colon ca. SW1116 | 0.0 |
| Ovarian ca. OVCAR-3 | 0.0 | Colon ca. Colo-205 | 0.0 |
| Ovarian ca. SK-OV.3 | 1.8 | Colon ca. SW-48 | 0.0 |
| Ovarian ca. OVCAR-4 | 1.0 | Colon Pool | 19.5 |
| Ovarian ca. OVCAR-5 | 6.8 | Small Intestine Pool | 10.4 |
| Ovarian ca. IGROV-1 | 0.0 | Stomach Pool | 6.3 |
| Ovarian ca. OVCAR-8 | 0.0 | Bone Marrow Pool | 4.3 |
| Ovary | 18.9 | Fetal Heart | 5.7 |
| Breast ca. MCF-7 | 0.0 | Heart Pool | 5.3 |
| Breast ca. MDA-MB-231 | 0.0 | Lymph Node Pool | 14.6 |
| Breast ca. BT 549 | 0.0 | Fetal Skeletal Muscle | 12.2 |
| Breast ca. T47D | 5.6 | Skeletal Muscle Pool | 3.3 |
| Breast ca. MDA-N | 0.0 | Spleen Pool | 87.1 |
| Breast Pool | 25.7 | Thymus Pool | 25.3 |
| Trachea | 26.2 | CNS cancer (glio/astro) U87-MG | 0.9 |
| Lung | 2.2 | CNS cancer (glio/astro) U-118-MG | 7.5 |
| Fetal Lung | 100.0 | CNS cancer (neuro;met) SK- N-AS | 0.0 |
| Lung.ca. NCI-N417 | 0.0 | CNS cancer (astro) SF-539 | 0.0 |
| Lung ca. LX-1 | 0.0 | GNS cancer (astro) SNB-75 | 0.0 |
| Lung ca. NCI-H146 | 0.0 | CNS cancer (glio) SNB-19 | 0.0 |
| Lung ca. SHP-77 | 0.0 | CNS cancer (glio) SF-295 | 0.0 |
| Lung ca. A549 | 0.0 | Brain (Amygdala) Pool | 11.3 |
| Lungca. NCI-H526 | 0.0 | Brain (cerebellum) | 1.3 |
| Lung ca. NCI-H23 | 22.4 | Brain (fetal) | 1.2 |
| Lung ca. NCI-H460 | 5.7 | Brain (Hippocampus) Pool | 10.7 |
| Lung ca. HOP-62 | 0.0 | Cerebral Cortex Pool | 2.4 |
| Lung ca. NCI-H522 | 0.0 | Brain (Substantia nigra) Pool | 4.4 |
| Liver | 2.4 | Brain (Thalamus) Pool | 7.1 |
| Fetal Liver | 8.4 | Brain (whole) | 3.4 |
| Liver ca HepG2 | 0.0 | Spinal Cord Pool | 11.0 |
| Kidney Pool | 13.8 | Adrenal Gland | 8.4 |
| Fetal Kidney | 17.8 | Pituitary gland Pool | 3.4 |
| Renal ca. 786-0 | 0.0 | Salivary Gland | 3.3 |
| Renal ca. A498 | 0.0 | Thyroid (female) | 1.5 |
| Renal ca. ACHN | 0.0 | Pancreatic ca. CAPAN2 | 0.0 |
| Renal ca. UO-31 | 0.0 | Pancreas Pool | 16.8 |

**Table ALD. Panel 4D**

| **Tissue Name** | **Rel. Exp.(%) Ag3296, Run 164633942** | **Tissue Name** | **Rel. Exp.(%) Ag3296, Run 164633942** |
|---|---|---|---|
| Secondary Th1 act | 0.2 | HUVEC IL-lbeta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.2 | Lung Microvascular EC TNFalpha +IL-1beta | 0.0 |
| Primary Th2 act | 0.6 | Microvascular Dermal EC none | 0.0 |
| Primary Tr1 act | 0.0 | Microsvasular Dermal EC TNFalph+ IL-1beta | 0.0 |
| Primary Th1 rest | 0.6 | Bronchial epithelium TNFalpha + IL1beta | 0.0 |
| Primary Th2 rest | 0.5 | Small airway epithelium none | 0.0 |
| Primary Tr1 rest | 0.5 | Small airway epithelium TNFalpha + IL-1beta | 0.3 |
| CD4SRA CD4 lymphocyte act | 3.1 | Coronery artery SMC rest | 0.0 |
| CD45RO CD4 lymphocyte act | 2.0 | Coronery artery SMC TNF alpha+ IL-1beta | 0.0 |
| CD8 lymphocyte act | 0.6 | Astrocytes rest | 0.0 |
| Secondary CD8 lymphocyte rest | 0.0 | Astrocytes TNFalpha+IL-1beta | 0.0 |
| Secondary CD8 lymphocyte act | 0.0 | KU-812 (Basophil) rest | 0.0 |
| CD4 lymphocyte none | 1.9 | KU-812 (Basophil) PMA/ionomycin | 0.0 |
| 2ry Th1/Th2/Tr1_anti-CD95 CH11 | 0.0 | CCD1106 (Keratinocytes) none CCD1106 (Keratinocytes) none | 0.0 |
| LAK cells rest | 18.7 | CCD1106 (Keratinocytes) TNF alpha+IL-1beta | 0.0 |
| LAK cells IL-2 | 0.9 | Liver cirrhosis | 1.4 |
| LAK cells IL-2+IL-12 | 5.5 | Lupus kidney | 0.0 |
| LAK cells IL-2+IFN gamma, | 4.6 | NCI-H292 none | 0.0 |
| LAK cells IL-2+IL-18 | 4.8 | NCI-H292 IL-4 | 0.0 |
| LAK cells PMA/ionomycin | 21.2 | NCI H292 IL-9 | 0.0 |
| NK Cells IL-2 rest | 2.7 | NCI-H292 IL-13 | 0.0 |
| Two Way MLR 3 day | 12.0 | NCI-H292 IFN gamma | 0.0 |
| Two Way MLR 5 day | 3.7 | HPAEC none | 0.0 |
| Two Way MLR 7 day | 0.8 | HPAEC TNF alpha + IL-1 beta | 0.0 |
| PBMC rest | 8.1 | Lung fibroblast none | 0.0 |
| PBMCPWM | 12.6 | Lung fibroblast TNF alpha + IL-1 beta | 0.0 |
| PBMC PHA-L | 14.9 | "Lung fibroblast IL-4 | 0.3 |
| Ramos (B cell) none | 21.2 | Lung fibroblast IL-9 | 0.2 |
| Ramos (B cell) ionomycin | 83.5 | Lung fibroblast IL-13 | 0.3 |
| B lymphocytes PWM | 39.5 | Lung fibroblast IFN gamma | 0.3 |
| B lymphocytes CD40L and IL-4 | 100.0 | Dermal fibroblast CCD1070 rest | 0.0 |
| EOL-1 dbcAMP | 18.4 | Dermal fibroblag CCD1070 TNF alpha | 0.4 |
| EOL-1 dbcAMP PMA/ionomycin | 55.5 | Dermal fibroblast CCD1070 IL-1 beta | 0.0 |
| Dendritic cells none | 21.0 | Dermal fibroblast IFN gamma | 0.0 |
| Dendritic cells LPS | 10.3 | Dermal fibroblast IL-4 | 0.0 |
| Dendritic cells anti-CD40 | 35.8 | IBD Colitis 2 | 1.6 |
| Monocytes rest | 25.9 | IBD Crohn's | 0.0 |
| Monocytes LPS | 11.3 | colon | 4.6 |
| Macrophages rest | 27.5 | Lung | 4.5 |
| Macrophages LPS | 13.7 | Thymus | 2.2 |
| HUVEC none | 0.0 | Kidney | 15.3 |
| HUVEC starved. | 0.0 | | |

**CNS_neurodegeneration_v1.0 Summary:** Ag3296 This panel confirms the expression of this gene at low levels in the brains of an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel_v1.4 Summary:** Ag3296 Expression of this gene is primarily associated with normal tissues rather than cancer cell lines. Expression of the CG57623-01 gene is highest in the fetal lung (CT = 31.1). Interestingly, this gene is expressed at higher levels in fetal lung (CT = 31.1) when compared to adult lung (CT = 36.6), suggesting that expression of this gene can be used to distinguish fetal from adult lung.

This gene is also expressed at low levels in several regions of the brain, including amygdala, hippocampus, thalamus and spinal cord. Therefore, this gene may play a role in central nervous system disorders such as Alzheimer's disease, Parkinson's disease, epilepsy, multiple sclerosis, schizophrenia and depression.

Among tissues with metabolic or endocrine function, this gene is expressed at low levels in adrenal gland, pancreas, fetal skeletal muscle, and fetal liver. Therefore, therapeutic modulation of the activity of this gene may prove useful in the treatment of endocrine/metabolically related diseases, such as obesity and diabetes.

**Panel 4D Summary:** Ag3296 The CG57623-01 gene is expressed at highest levels in activated B lymphocytes, represented by ionomycin-activated Ramos and CD40L and IL-4 or pokeweed mitogen-activated B lymphocytes. Therefore, small molecule drugs or antibodies that antagonize the function of this gene product may be useful as therapeutic drugs to reduce or eliminate the symptoms in patients with autoimmune and inflammatory diseases in which B cells play a part in the initiation or progression of the disease process, such as lupus erythematosus, Crohn's disease, ulcerative colitis, multiple sclerosis, chronic obstructive pulmonary disease, asthma, emphysema, rheumatoid arthritis, or psoriasis.

### NOV45

Expression ofNOV45A/CG57656-01 and NOV45B/CG57656-02 was assessed using the primer-probe sets Ag3298 and Ag4482, described in Tables AMA and AMB. Results of the RTQ-PCR runs are shown in Tables AMC, AMD, AME and AMF. Please note that primer-probe set Ag4482 is specific for CG57656-02.

**Table AMA. Probe Name Ag3298**

| **Primers** | **Sequences** | **Length** | **Start Position** | **SEQ ID NO** |
|---|---|---|---|---|
| Forward | 5'-cagacctatgcaacctctggta-3' | 22 | 3390 | 392 |
| Probe | TET-5'-agctgcgacatacaagccaaggcat-3'-TAMRA | 25 | 3422 | 393 |
| Reverse | 5'-ggtaaggcagcacaggtatg-3' | 20 | 3450 | 394 |

**Table AMB. Probe Name Ag4482**

| **Primers** | **Sequences** | **Length** | **Start Position** | **SEQ ID NO** |
|---|---|---|---|---|
| Forward | 5'-gaggtggtcactgtgatcactt-3' | 22 | 1804 | 395 |
| Probe | TET-5'-ttacaactccagaacccctggtgctg-3'-TAMRA | 26 | 1838 | 396 |
| Reverse | 5'-ctgagtccgattggctatgag-3' | 21 | 1882 | 397 |

**Table AMC. CNS_neurodegeneration_v1.0**

| **Tissue Name** | **Rel.Exp.(%) Ag3298, Run 210063510** | **Rel. Exp.(%) Ag4482, Run 224535697** | **Tissue Name** | **Rel.Exp.(%) Ag3298, Run 210063510** | **Rel. Exp.(%) Ag4482, Run 224535697** |
|---|---|---|---|---|---|
| AD 1 Hippo | 6.0 | 6.9 | Control (Path) 3 Temporal Ctx | 20.3 | 3.9 |
| AD 2 Hippo | 25.5 | 24.7 | Control (path) 4 Temporal Ctx | 54.3 | 43.2 |
| AD 3 Hippo | 9.5 | 6.2 | AD 1 Occipital Ctx | 48.0 | 12.9 |
| AD 4 Hippo | 16.0 | 8.4 | AD 2 Occipital Ctx (Missing) | 0.0 | 0.0 |
| AD 5 Hippo | 100.0 | 100.0 | AD 3 Occipital | 17.6 | 6.6 |
| AD 6 Hippo | 34.6 | 27.7 | AD 4 Occipital Ctx | 38.7 | 20.2 |
| Control 2 Hippo | 19.9 | 12.3 | AD 5 Occipital Ctx | 30.1 | 42.6 |
| Control 4 Hippo | 23.2 | 10.0 | AD 6 Occipital Ctx | 47:6 | 23.7 |
| Control (Path) 3 Hippo | 12.9 | 5.5 | Control 1 Occipital Ctx | 10.0 | 3.1 |
| AD 1 Temporal ctx | 42.3 | 12.3 | Control 2 Occipital Ctx | 43.2 | 38.7 |
| AD 2 Temporal Ctx | 35.4 | 34.6 | Control 3 Occipital Ctx | 66.4 | 22.2 |
| AD 3 Temporal Ctx | 24.3 | 9.1 | Control 4 Occipital Ctx | 20.7 | 4.8 |
| AD 4 Temporal Ctx | 61.6 | 24.3 | Control (Path) 1 Occipital Ctx | 76.3 | 96.6 |
| AD 5 Inf Temporal Ctx | 79.0 | 59.9 | Control (Path) 2 Occipital Ctx | 38.7 | 17.1 |
| AD 5 Sup Temporal Ctx | 45.1 | 39.0 | Control (Path) 3 Occipital Ctx | 4.9 | 1.4 |
| AD 6 Inf Temporal Ctx | 65.1 | 42.6 | Control (path) 4 Occipital Ctx | 67.8 | 32.1 |
| AD 6 Sup Temporal Ctx | 64.6 | 57.4 | Control 1 Parietal Ctx | 27.9 | 7.8 |
| Control 1 Temporal Ctx | 25.9 | 6.7 | Control 2 Parietal Ctx | 70.2 | 49.3 |
| Control 2 Temporal Ctx | 30.1 | 22.1 | Control 3 Parietal Ctx | 34.6 | 18.4 |
| Control 3 Tempora Ctx | 56.6 | 19.5 | Control (Path) 1 Parietal Ctx 1 | 61.1 | 68.8 |
| Control 3 Temporal Ctx | 31.6 | 13.6 | Control (Path) 2 Parietal Ctx | 46.7 | 33.0 |
| Control (Path) 1 Temporal Ctx | 71.2 | 76.8 | Control (Path) 3 Parietal Ctx | 7.6 | 3.4 |
| Control (Path) 2 Temporal Ctx | 76.3 | 50.0 | Control (Path) 4 Parietal Ctx | 78.5 | 54.7 |

**Table AMD. General_screening_panel_v1.4**

| **Tissue Name** | **Rel. Exp.(%) Ag3298, Run 215601953** | **Rel.Exp.(%) Ag3298, Run 216607677** | **Re. Exp.(%) Ag3298, Run 222691295** | **Rel. Exp.(%) Ag4482, Run 222666310** |
|---|---|---|---|---|
| Adipose | 0.2 | 0.9 | 1.9 | 0.7 |
| Melanoma* Hs688(A).T | 0.0 | 0.0 | 0.0 | 0.0 |
| Melanoma* Hs688(B).T | 0.1 | 0.2 | 0.0 | 0.1 |
| Melanoma* M14 | 0.0 | 0.0 | 0.0 | 0.0 |
| Melanoma* LOXIMVI | 2.1 | 2.5 | 3.3 | 1.7 |
| Melanoma* SK-MEL-5 | 2.4 | 2.2 | 1.5 | 3.7 |
| Squamous cell carcinoma SCC-4 | 0.1 | 0.2 | 0.1 | 0.1 |
| Testis Pool | 5.5 | 7.3 | 7.9 | 5.6 |
| Prostate ca.* (bone met) PC-3 | 0.9 | 1.4 | 0.5 | 1.1 |
| Prostate Pool | 5.5 | 4.5 | 4.5 | 2.4 |
| Placenta | 0.5 | 0.7 | 0.7 | 0.6 |
| Uterus Pool | 2.5 | 1.5 | 1.6 | 2.7 |
| Ovarian ca. OVCAR-3 | 0.0 | 0.1 | 0.0 | 0.0 |
| Ovarian ca. SK OV-3 | 0.0 | 0.0 | 0.0 | 0.0 |
| Ovarian ca. OVCAR-4 | 0.1 | 0.0 | 0.0 | 0.1 |
| Ovarian ca. OVCAR-5 | 20.2 | 19.1 | 21.6 | 13.7 |
| Ovarian ca. IGROV-1 | 0.0 | 0.4 | 0.4 | 0.1 |
| Ovarian ca.OVCAR-8 | 0.0 | 0.0 | 0.0 | 0.0 |
| Ovary | 0.5 | 0.7 | 0.3 | 0.6 |
| Breast ca.MCF-7 | 3.0 | 2.1 | 2.1 | 1.6 |
| Breast ca MDA-MB-231 | 0.0 | 0.0 | 0.0 | 0.0 |
| Breast ca. BT 549 | 0.6 | 0.2 | 0.5 | 0.2 |
| Breastca.T47D | 45.4 | 52.5 | 43.5 | 17.0 |
| Breast ca. MDA-N | 0.0 | 0.0 | 0.0 | 0.0 |
| Breast Pool | 10.4 | 10.8 | 12.5 | 9.0 |
| Trachea | 1.4 | 4.9 | 4.6 | 2.5 |
| Lung | 0.8 | 1.0 | 0.7 | 0.5 |
| Fetal Lung | 6.5 | 7.0 | 5.7 | 5.2 |
| Lung ca. NCI-N417 | 16.3 | 14.8 | 13.6 | 4.0 |
| Lung ca. LX-I | 12.9 | 15.7 | 11.4 | 13.1 |
| Lung ca. NCI-H146 | 4.3 | 5.6 | 5.2 | 2.2 |
| Lungca. SHP-77 | 7.4 | 5.2 | 5.0 | 7.9 |
| Lung ca. A549 | 2.2 | 1.1 | 1.1 | 1.1 |
| Lung ca. NCI-H526 | 25.5 | 21.6 | 18.7 | 6.8 |
| Lung ca. NCI-H23 | 9.2 | 4.9 | 4.1 | 3.8 |
| Lung ca. NCI-H460 | 1.4 | 0.4 | 1.0 | 0.5 |
| Lung ca. HOP-62 | 0.2 | 0.0 | 0.2 | 0.1 |
| Lung ca. NCI-H522 | 3.5 | 2.2 | 4.2 | 3.2 |
| Liver | 0.0 | 0.0 | 0.0 | 0.0 |
| Fetal Liver | 0.1 | 0.4 | 0.1 | 0.1 |
| Liver ca. HepG2 | 0.0 | 0.0 | 0.0 | 0.0 |
| Kidney Pool | 51.8 | 62.0 | 59.0 | 27.0 |
| Fetal Kidney | 3.1 | 3.9 | 2.6 | 1.4 |
| Renal ca. 786-0 | 0.0 | 0.0 | 0.0 | 0.0 |
| Renal ca. A498 | 0.1 | 0.1 | 0.2 | 0.0 |
| Renal ca. ACHN | 0.0 | 0.1 | 0.2 | 0.2 |
| Renal ca. UO-31 | 2.6 | 1.2 | 2.0 | 0.9 |
| Renal ca. TK-10 | 1.5 | 1.9 | 1.7 | 2.3 |
| Bladder | 2.2 | 1.5 | 1.7 | 1.2 |
| Gastric ca. (liver met.) NCI-N87 | 100.0 | 93.3 | 94.0 | 100.0 |
| Gastric ca. KATO III | 97.3 | 85.3 | 100.0 | 62.4 |
| Colon ca.SW-948 | 8.1 | 6.9 | 7.7 | 2.0 |
| Colon ca.SW480 | 32.5 | 37.6 | 16.4 | 23.7 |
| Colon ca.*(SW480 met) SW620 | 4.3 | 6.6 | 3.9 | 4.6 |
| Colon ca. HT29 | 2.5 | 3.3 | 3.8 | 2.8 |
| Colon ca. HCT-116 | 9.3 | 11.7 | 12.7 | 8.7 |
| Colon ca. CaCo-2 | 2.2 | 1.9 | 2.9 | 2.1 |
| Colon cancer tissue | 0.4 | 0.9 | 0.8 | 0.3 |
| Colon ca. SW1116 | 13.7 | 15.2 | 11.9 | 4.2 |
| Colon ca. Colo-205 | 15.4 | 8.9 | 12.4 | 2.8 |
| Colon ca. SW-48 | 0.0 | 0.0 | 0.0 | 0.1 |
| Colon Pool | 13.4 | 17.9 | 16.8 | 11.0 |
| Small Intestine Pool | 19.3 | 19.2 | 19.8 | 12.0 |
| Stomach Pool | 6.6 | 7.2 | 7.9 | 4.5 |
| Bone Marrow Pool | 2.4 | 4.3 | 6.6 | 5.5 |
| Fetal Heart | 9.0 | 12.3 | 16.5 | 9.4 |
| Heart Pool | 6.2 | 8.6 | 8.0 | 5.3 |
| Lymph Node Pool | 22.1 | .18.2 | 25.5 | 15.8 |
| Fetal Skeletal Muscle | 2.4 | 2.6 | 1.9 | 1.2 |
| Skeletal Muscle Pool | 1.6 | 2.4 | 1.1 | 1.2 |
| Spleen Pool | 1.7 | 3.0 | 2.4 | 2.0 |
| Thymus Pool | 4.1 | 5.0 | 8.0 | 2.4 |
| CNS cancer (glio/astro) U87-MG | 5.1 | 4.7 | 4.6 | 2.8 |
| CNS cancer (glio/astro) U-118-MG | 78.5 | 100.0 | 80.7 | 69.7 |
| CNS cancer (neuro;met) SK-N-AS | 5.1 | 5.3 | 6.2 | 4.9 |
| CNS cancer (astro) SF-539 | 0.0 | 0.0 | 0.0 | 0.0 |
| CNS cancer (astro) SNB-75 | 7.1 | 9.5 | 10.4 | 5.6 |
| CNS cancer (glio) SNB-19 | 0.2 | 0.0 | 0.2 | 0.1 |
| CNS cancer (glio) SF-295 | 5.6 | 4.7 | 4.2 | 4.1 |
| Brain (Amygdala) Pool | 5.9 | 7.3 | 5.8 | 2.4 |
| Brain (cerebellum) | 59.5 | 56.3 | 55.5 | 37.9 |
| Brain (fetal) | 10.7 | 12.0 | 15.1 | 8.7 |
| Brain (Hippocampus) Pool | 5.6 | 5.1 | 5.8 | 3.4 |
| Cerebral Cortex Pool | 7.5 | 7.9 | 7.3 | 4.4 |
| Brain (Substantia nigra) Pool | 9.9 | 8.8 | 9.5 | 3.8 |
| Brain (Thalamus) Pool | 11.3 | 8.8 | 9.6 | 5.8 |
| Brain (whole) | 11.6 | 12.8 | 17.1 | 8.5 |
| Spinal Cord Pool | 2.0 | 2.6 | 5.3 | 2.0 |
| Adrenal Gland | 1.6 | 2.7 | 2.0 | 1.4 |
| Pituitarygland Pool | 2.5 | 2.8 | 2.4 | 1.4 |
| Salivary Gland | 0.2 | 0.3 | 0.5 | 0.3 |
| Thyroid (female) | 3.5 | 5.0 | 4.8 | 2.3 |
| Pancreatic ca. CAPAN2 | 8.2 | 4.9 | 6.4 | 6.8 |
| Pancreas Pool | 12.5 | 12.8 | 9.1 | 7.9 |

**Table AME. Panel 4.1D**

| **Tissue Name** | **Rel. Exp.(%) Ag4482, Run 195476203** | **Tissue Name** | **Rel. Exp.(%) Ag4482, Run 195476203** |
|---|---|---|---|
| Secondary Th1 act | 18.7 | HUVEC IL-1beta | 2.1 |
| Secondary Th2 act | 24.0 | HUVEC IFN gamma | 2.0 |
| Secondary Tr1 act | 15.1 | HUVEC TKF alpha+ IFN gamma | 1.5 |
| Secondary Th1 rest | 40.6 | HUVEC TNF alpha+IL4 | 0.0 |
| Secondary Th2 rest | 43.2 | HUVEC IL-11 | 0.0 |
| Secondary Tr1 rest | 90.8 | Lung Microvascular EC none | 0.0 |
| Primary Th1 act | 54.7 | Lung Microvascular EC TNFalpha + IL-1beta | 0.5 |
| Primary Th2 act | 92.0 | Microvascular Dermial EC none EC none | 0.0 |
| Primary Tr1 act | 75.3 | Microsvasular Dermal EC TNFalpha + IL-1 beta | 0.0 |
| Primary Th1 rest | 60.3 | Bronchial epithelium TNFalpha + IL1beta | 0.0 |
| Primary Th2 rest | 49.0 | Small airway epithelium none | 0.0 |
| Primary Tr1 rest | 84.7 | Small airway epithelium TNFalpha + IL-1beta | 0.0 |
| CD45RA CD4 lymphocyte act | 26.2 | Coronery artery SMC rest | 0.3 |
| CD45RO CD4 lymphocyte act | 42.0 | Coronery artery SMC TNFalpha + IL-1beta | 0.5 |
| CD8 lymphocyte act | 33.9 | Astrocytes test | 16.4 |
| Secondary CD8 lymphocyte rest | 60.7 | Astrocytes TNFalpha + IL-1beta | 15.3 |
| Secondary CD8 lymphocyte act | 13.9 | KU-812 (Basophit) rest | 52.9 |
| CD4 lymphocyte none | 41.8 | KU-812 (Basophil) PMA/ionomycin | 92.7 |
| 2ry Th1/Th2/Tr1_anti-CD95 CH11 | 68.8 | CCD1106 (Keratinocytes) none | 0.0 |
| LAK cells rest | 40.3 | CCD1106 (Kemtinocytes) TNFalpha + IL-1beta | 0.0 |
| LAK cells IL-2 | 26.1 | Liver cirrhosis | 1.7 |
| LAK cells IL-2+IL-12 | 32.8 | NCI-H292 none | 0.0 |
| LAK cells IL-2+IFN gamma | 34.4 | NCI-H292 IL-4 | 0.0 |
| LAK cells IL-2+ IL-18 | 50.3 | NCI-H292 IL-9 | 1.3 |
| LAK cells PMA/ionomycin | 100.0 | NCI-H292 IL-13 | 0.7 |
| NK Cells IL-2 rest | 29.7 | NCI-H292 IFN gamma | 0.0 |
| Two Way MLR 3 day | 28.7 | HPAEC none | 0.6 |
| Two Way MLR 5 day | 21.8 | HPAEC TNF alpha+IL-1beta | 0.7 |
| Two way MLR 7 day | 20.7 | Lung fibroblast none | 0.0 |
| PBMC rest | 14.1 | Lung fibroblast TNF alpha + IL- 1 beta | 0.6 |
| PBMCPWM | 32.8 | Lung fibrobast IL-4 | 1.3 |
| PBMC PHA-L | 88.3 | Lung fibroblast IL-9 | 1.0 |
| Ramos(B cell)none | 0.0 | Lung fibroblast IL-13 | 0.6 |
| Ramos (B cell) iononpymn | 0.0 | Lung fibroblast IFN gamma | 1.6 |
| B lymphocytes PWM | 23.8 | Dermal fibroblast CCD1070 rest | 2.7 |
| B lymphocytes CD40L and IL-4 | 9.7 | Dermal fibroblast CCD1070 TNF alpha | 32.3 |
| EOL-1 dboAMP | 0.6 | Dermal fibroblast CCD1070 IL-1 beta | 1.4 |
| EOL-1 dbcAMP PMA/ionomycin | 0.0 | Dermal fibroblast IFN gamma | 1.4 |
| Dendritic cells none | 5.0 | Dermal fibroblast IL-4 | 1.2 |
| Dendritic cells LPS | 0.8 | Dermal Fibroblasts rest | 2.2 |
| Dendritic cells anti-CD40 | 0.0 | Neutrophils TNFa+LPS | 0.8 |
| Monocytes rest | 0.6 | Neutrophils rest | 0.6 |
| Monocytes LPS | 0.0 | Colon | 19.3 |
| Macrophages rest | 0.8 | Lung | 3.1 |
| Macrophages LPS | 0.0 | Thymus | 18.9 |
| HUVEC none | 0.0 | Kidney | 7.5 |
| HUVEC starved | 0.0 | | |

**Table AMF. Panel 4D**

| **Tissue Name** | **Rel. Exp.(%) Ag3298, Run 164682520** | **Tissue Name** | **Rel. Exp.(%) Ag3298, Run 164682520** |
|---|---|---|---|
| Secondary Th1 act | 18.9 | HUVEC IL-1beta | 0.0 |
| Secondary Th2 act | 20.4 | HUVEC IFN gamma | 0.9 |
| Secondary Tr1 act | 19.6 | HUVEC TNF alpha+IFN gamma | 2.5 |
| Secondary Th1 rest | 40.3 | HUVEC TNF alpha + IL4 | 1.1 |
| Secondary Th2 rest | 34.4 | HUVEC IL-11 | 0.0 |
| Secondary Tr1 rest | 100.0 | Lung Microvascular EC none | 0.0 |
| Primary Th1 act | 38.2 | Lung Microvascular EC TNFalpha +IL-1beta | 0.0 |
| Primary Th2 act | 56.3 | Microvascular Dermal EC none | 0.0 |
| Primary Tr1 act TNFalpha+IL-1beta | 49.0 | Microsvasular Dermal EC | 0.0 |
| Primary Th1 rest | 87.1 | Bronchial epithelium TNFalpha+ IL1beta | 0.0 |
| Primary Th2 rest | 63.7 | Small airway epithelium none | 0.0 |
| Primary Tr1 rest | 58.6 | Small airway epithelium TNFalpha + IL-1beta | 0.1 |
| CD45RA CD4 lymphocyte act | 27.2 | Coronery artery SMC rest | 0.0 |
| CD45RO CD4 lymphocyte act | 31.9 | Coronery artery SMC TNFalpha+ IL-1beta | 0.0 |
| CD8 lymphocyte act | 46.0 | Astrocytes rest | 58.2 |
| Secondary CD8 lymphocyte rest | 41.8 | Astrocytes TNFalpha+IL-1beta | 13.2 |
| Secondary CD8 lymphocyte act | 10.4 | KU-812 (Basophil) rest | 24.8 |
| CD4 lymphocyte none | 26.1 | KU-812 (Basophil) PMA/ionomycin | 52.9 |
| 2ry Th1/Th2/Tr1_anti-CD95 CH11 | 31.9 | CCD1106 (Keratinocytes) none | 0.0 |
| LAK cells rest | 24.3 | CCD1106 (Keratinocytes) TNFalpha + IL-1beta | 0.0 |
| LAK cells IL-2 | 11.7 | Liver cirrhosis | 10.6 |
| LAK cells IL-2+IL-12 | 29.3 | Lupus Kidney | 0.0 |
| LAK cells IL2+IFN gamma | 35.8 | NCI-H292 none | 0.0 |
| LAK cells IL-2+ IL-18 | 32.3 | NCI-H292 IL-4 | 0.0 |
| LAK cells PMA/ionomycin | 59.5 | NCI-H292 IL-9 | 0.0 |
| NK Cells IL-2 rest | 21.8 | NCI-H292 IL-13 | 0.0 |
| Two Way MLR 3 day | 22.4 | NCI-H292 IFN gamma | 0.0 |
| Two Way MLR 5 day | 17.2 | HPAEC none | 0.0 |
| Two Way MLR 7 day | 17.0 | HPAECTNF alpha+ IL-1 beta | 0.0 |
| PBMC rest | 15.1 | Lung fibroblast none | 0.0 |
| PBMC PWM | 37.9 | Lung fibroblast TNF alpha +IL-1 beta | 0.0 |
| PBMCPHA-L | 51.4 | Lung fibroblast IL-4 | 0.0 |
| Ramos (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 12.4 | Lung fibroblast IFN gamma | 2.4 |
| B lyphocytes CD40L and IL-4 | 10.2 | Dermal fibroblast CCD1070 rest | 0.5 |
| EOL-1 dbcAMP | 0.0 | Dermal fibroblast CCD1070 TNF alpha | 12.8 |
| EOL-1 dbcAMP PMA/ionomycin | 0.0 | Dermal fibroblast CCD1070 IL-1 beta | 0.0 |
| Dendritic cells none | 1.9 | Dermal fibroblast IFN gamma | 0.0 |
| Dendritic cells LPS | 0.0 | Dermal fibroblast IL-4 | 1.0 |
| Dendritic cells anti-CD40 | 0.0 | IBD Colitis 2 | 3.0 |
| Monocytes rest | 0.7 | IBD Crohn's | 0.0 |
| Monocytes LPS | 0.4 | Colon | 54.7 |
| Macrophages rest | 0.9 | Lung | 13.8 |
| Macrophages LPS | 1.4 | Thymus | 7.8 |
| HUVEC none | 1.0 | Kidney | 14.8 |
| HUVEC starved | 0.0 | | |

**CNS_neurodegeneration_v1.0** Summary: Ag3298/Ag4482 Results from two experiments using different probe/primer sets are in excellent agreement. This panel confirms the expression of this gene at moderate levels in the brain in an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel-v1.4** Summary: Ag3298/Ag4482 Results from four experiments using two different probe/primer sets are in excellent agreement, with highest expression of the CG57656-02 gene in two samples derived from gastric cancer cell lines (CTs=27.5). Interestingly, expression of this gene is much higher in the gastric cancer cell lines than in normal stomach. Thus, expression of this gene could be used to differentiate between these samples and other samples on this panel and as a marker for gastric cancer. Furthermore, therapeutic modulation of the activity of this gene or its protein product, using small molecule drugs, antibodies or protein therapeutics, could be of benefit in the treatment of gastric cancer.

This gene is also expressed at moderate levels throughout the CNS, including in amygdala, hippocampus, substantia nigra, thalamus, cerebral cortex, and spinal cord, and at high levels in the cerebellum. Therefore, this gene may play a role in central nervous system disorders such as Alzheimer's disease, Parkinson's disease, epilepsy, multiple sclerosis, schizophrenia and depression.

The CG57656-02 gene encodes a protein with similarity to the Drosophila turtle protein, a member of the Ig superfamily (IgSF). In Drosophila, the turtle (tutl) protein is expressed in the CNS and in a small, defined subset of the peripheral nervous system (PNS). Alternative splicing of the primary transcript results in membrane-bound and secreted Tut1 isoforms that are essential for development and adult viability. In addition, tutl function is required for establishing a nervous system capable of executing complex forms of coordinated movement such as tactile escape response, coordinated righting behavior in larval and adult stages, and flight in adulthood (ref. 1).

Among tissues with metabolic or endocrine function, this gene is expressed at moderate levels in pancreas and heart and at low levels in thyroid, pituitary gland, adrenal gland, and skeletal muscle. Therefore, therapeutic modulation of the activity of this gene may prove useful in the treatment of endocrine/metabolically related diseases, such as obesity and diabetes.

### References:

### 1. Bodily KD, Morrison CM, Renden RB, Broadie K. A novel member of the Ig superfamily, turtle, is a CNS specific protein required for coordinated motor control. J Neurosci 2001 May 1;21(9):3113-25

We describe here the cloning and functional characterization of a neural-specific novel member of the Ig superfamily, turtle (tutl), with a structure of five Ig C2-type domains, two fibronectin type III domains, and one transmembrane region. Alternative splicing of the tutl gene produces at least four Tutl isoforms, including two transmembrane proteins and two secreted proteins, with primary structures closely related to a human brain protein (KIAA1355), the Deleted in Colorectal Cancer/Neogenin/Frazzled receptor family, and the Roundabout/Duttl receptor family. An allelic series of tutl gene mutations resulted in recessive lethality to semilethality, indicating that the gene is essential. In contrast to other family members, tut1 does not play a detectable role in axon pathfinding or nervous system morphogenesis. Likewise, basal synaptic transmission and locomotory movement are unaffected. However, tutl mutations cause striking movement defects exhibited in specific types of highly coordinated behavior. Specifically, tutl mutants display an abnormal response to tactile stimulation, the inability to regain an upright position from an inverted position (hence, "turtle"), and the inability to fly in adulthood. These phenotypes demonstrate that tutl plays an essential role in establishing a nervous system capable of executing coordinated motor output in complex behaviors.

### PMID: 11312296

**Panel 4.1D Summary:** Ag4482 The CG57656-02 gene is expressed at highest levels in stimulated lymphokine-activated killer cells (LAK). LAK cells are involved in tumor immunology and cell clearance of virally and bacterial infected cells as well as tumors.

In addition, expression of this gene is upregulated in resting dermal fibroblast CCD1070 (CT = 35.7) by treatment with TNF alpha (CT = 32.1). Expression of this gene in stimulated dermal fibroblasts suggests that this gene may be important in the treatment of psoriasis.

The CG57656-02 gene encodes a protein with similarity to the Drosophila turtle protein, a member of the Ig superfamily (IgSF). The 5 Ig domains and 2 fibronectin domains in this membrane protein are characteristic of several types of cell surface receptors and cell adhesion molecules.

In addition to the utility in treatment of psoriasis, the expression of this gene in basophils and several types of T lymphocyte preparations suggests that antibodies or small molecules that antagonize the function of the CG57656-02 protein may be useful in reduction or elimination of the symptoms of various T cell-dependent or basophil-dependent medical conditions, such as, but not limited to, Crohn's disease, ulcerative colitis, multiple sclerosis, chronic obstructive pulmonary disease, asthma, emphysema, rheumatoid arthritis, and lupus erythematosus.

Furthermore, the extracellular domain of this protein may function to block the binding of the cognate ligand of the CG57656-02 protein on neighboring cells. This may modulate the function of T lymphocytes and basophils, and be useful as a protein therapeutic to reduce or eliminate the symptoms in the symptoms of various T cell-dependent or basophil-dependent medical conditions, such as, but not limited to, Crohn's disease, ulcerative colitis, multiple sclerosis, chronic obstructive pulmonary disease, asthma, emphysema, rheumatoid arthritis, and lupus erythematosus, or psoriasis.

**Panel 4D Summary:** Ag3298 Results using this probe/primer set are in excellent agreement with those obtained using Ag4482 on Panel 4.1D. Please see Panel 4.1D for a description of the potential utility of this gene in immune function.

### NOV46

Expression of NOV46/CG57682-01 was assessed using the primer-probe set Ag3308, described in Table ANA. Results of the RTQ-PCR runs are shown in Tables ANB, ANC and AND.

**Table ANA. Probe Name Ag3308**

| **Primers** | **Sequences** | **Length** | **Start Position** | **SEQ ID NO** |
|---|---|---|---|---|
| Forward | 5'-gcaacaaattcctgaagatcag-3' | 22 | 1153 | 398 |
| Probe | TET-5'-cctcagctgaactcaaaagccatcaa-3'-TAMRA | 26 | 1182 | 399 |
| Reverse | 5'-atcagagggaaggcaaggt-3' | 19 | 1210 | 400 |

**Table ANB. CNS_neurodegeneration_v1.0**

| **Tissue Name** | **Rel. Exp.(%) Ag3308, Run 210143040** | **Tissue Name** | **Rel. Exp.(%) Ag3308, Run 210143040** |
|---|---|---|---|
| AD 1 Hippo | 5.0 | Control (Path) 3 Temporal Ctx | 9.9 |
| AD 2 Hippo | 25.2 | Control (Path) 4 Temporal Ctx | 36.6 |
| AD 3 Hippo | 14.6 | AD 1 Occipital Ctx | 46.3 |
| AD 4 Hippo | 4.4 | AD 2 Occipital Ctx (Missing) | 0.0 |
| AD 5 Hippo | 100.0 | AD 3 Occipital Ctx | 17.2 |
| AD 6 Hippo | 37.1 | AD 4 Occipital Ctx | 33.9 |
| Control 2 Hippo | 8.2 | AD 5 Occipital Ctx | 8.4 |
| Control 4 Hippo | 25.2 | AD 6 Occipital Ctx | 18.6 |
| Control (Path) 3 Hippo | 4.4 | Control 1 Occipital Ctx | 2.4 |
| AD 1 Temporal Ctx | 21.9 | Control 2 Occipital Ctx | 18.2 |
| AD 2 Temporal Ctx | 21.8 | Control 3 Occipital Ctx | 32.1 |
| AD 3 Temporal Ctx | 15.1 | Control 4 Occipital Ctx | 2.2 |
| AD 4 Temporal Ctx | 29.7 | Control (Path) 1 Occipital Ctx | 64.2 |
| AD 5 Inf Temporal Ctx | 70.7 | Control (Path) 2 Occipital Ctx | 22.1 |
| AD 5 Sup Temporal Ctx | 41.5 | Control (Path) 3 Occipital Ctx | 8.4 |
| AD 6 Inf Temporal Ctx | 59.5 | Control (Path) 4 Occipital Ctx | 38.2 |
| AD 6 Sup Temporal Ctx | 53.2 | Control 1 Parietal Ctx | 10.5 |
| Control 1 Temporal Ctx | 17.8 | Control 2 Parietal ax | 42.3 |
| Control 2 Temporal Ctx | 17.0 | Control 3 Parietal Ctx | 16.3 |
| Control 3 Temporal Ctx | 15.3 | Control (Path) 1 Parietal Ctx | 56.3 |
| Control 3 Temporal Ctx | 16.6 | Control (Path) 2 Parietal Ctx | 17.9 |
| Control (Path) 1 Temporal Ctx | 47.0 | Control (Path) 3 Parietal Ctx | 7.3 |
| Control (Path) 2 Temporal Ctx | 44.1 | Control (Path) 4 Parietal Ctx | 69.3 |

**Table ANC. General_screening_panel_v1.4**

| **Tissue Name** | **Rel. Exp.(%) Ag3308, Run 215648361** | **Tissue Name** | **Rel. Exp.(%) Ag3308, Run 215648361** |
|---|---|---|---|
| Adipose | 13.4 | Renal ca. TK-10 | 31.0 |
| Melanoma* Hs688(A).T | 4.6 | Bladder | 33.0 |
| Melanoma* Hs688(B).T | 4.0 | Gastric ca. (liver met.) NCI-N87 | 57.0 |
| Melanoma* M14 | 21.8 | Gastric ca. KATO III | 59.9 |
| Melanoma* LOXIMVI | 10.4 | Colon ca. SW-948 | 6.8 |
| Melanoma* SK-MEL-5 | 24.3 | Colon ca. SW480 | 82.9 |
| Squamous cell carcinoma SCC-4 | 14.5 | Colon ca.* (SW480 met) SW620 | 47.6 |
| Testis Pool | 12.6 | Colon ca. HT29 | 30.6 |
| Prostate ca.* (bone met) PC-3 | 24.7 | Colon ca. HCT-116 | 42.3 |
| Prostate Pool | 30.8 | Colon ca. CaCo-2 | 42.0 |
| Placenta | 6.9 | Colon cancer tissue | 8.5 |
| Uterus Pool | 9.5 | Colon ca. SW1116 | 8.7 |
| Ovarian ca. OVCAR-3 | 29.3 | Colon ca. Colo-205 | 13.2 |
| Ovarian ca. SK-OV-3 | 37.4 | Colon ca. SW-48 | 4.0 |
| Ovarian ca.OVCAR-4 | 4.8 | Colon Pool | 31.9 |
| Ovarian ca. OVCAR-5 | 59.9 | Small Intestine Pool | 60.3 |
| Ovarian ca. IGROV-1 | 7.5 | Stomach Pool | 34.2 |
| Ovarian ca. OVCAR-8 | 19.6 | Bone Marrow Pool | 39.0 |
| Ovary | 25.7 | Fetal Heart | 17.2 |
| Breast ca. MCF-7 | 43.2 | Heart Pool | 9.5 |
| Breast ca. MDA-MB-231 | 47.3 | Lymph Node Pool | 34.4 |
| Breast ca. BT 549 | 32.1 | Fetal Skeletal Muscle | 13.2 |
| Breast ca. T47D | 59.5 | Skeletal Muscle Pool | 23.0 |
| Breast ca. MDA-N | 27.5 | Spleen Pool | 22.4 |
| Breast Pool | 52.9 | Thymus Pool | 18.3 |
| Trachea | 32.3 | CNS cancer (glio/astro) U87-MG | 40.3 |
| Lung | 16.5 | CNS cancer (glio/astro) U-118-MG | 61.1 |
| Fetal Lung | 52.5 | CNS cancer (neuro;met) SK-N-AS | 27.5 |
| Lung ca. NCI-N417 | 11.4 | CNS cancer (astro) SF-539 | 19.5 |
| Lung ca. LX-1 | 47.3 | CNS cancer (astro) SNB-75 | 65.1 |
| Lung ca. NCI-H146 | 7.6 | CNS cancer (glio) SNB-19 | 15.0 |
| Lungca.SHP-77 | 69.3 | CNS cancer (glio) SF-295 | 92.7 |
| Lung ca. A549 | 19.2 | Brain (Amygdala) Pool | 9.8 |
| Lung ca. NCI-H526 | 11.7 | Brain (cerebellum) | 6.2 |
| Lung ca. NCI-H23 | 81.8 | Brain (fetal) | 38.4 |
| Lung ca.NCI-H460 | 25.7 | Brain (Hippocampus) Pool | 12.0 |
| Lung ca. HOP-62 | 26.2 | Cerebral Cortex Pool | 13.0 |
| Lung ca. NCI-H522 | 29.7 | Brain (Substantia nigra) Pool | 4.6 |
| Liver | 0.0 | Brain (Thalamus) Pool | 19.9 |
| Fetal Liver | 14.8 | Brain (whole) | 8.9 |
| Liver ca. HepG2 | 12.6 | Spinal Cord Pool | 9.8 |
| Kidney Pool | 100.0 | Adrenal Gland | 20.2 |
| Fetal Kidney | 47.0 | Pituitary gland Pool | 7.3 |
| Renal ca. 786-0 | 24.1 | Salivary Gland | 12.0 |
| Renal ca. A498 | 9.3 | Thyroid (female) | 4.3 |
| Renal ca. ACHN | 7.3 | Pancreatic ca. CAPAN2 | 42.6 |
| Renal ca. UO-31 | 13.9 | Pancreas Pool | 62.0 |

**Table AND. Panel 4D**

| **Tissue Name** | **Rel. Exp.(%) Ag3308, Run 164335486** | **Tissue Name** | **Rel. Exp.(%) Ag3308, Run 164335486** |
|---|---|---|---|
| Secondary Th1 act | 17.7 | HUVEC IL-1 beta | 6.0 |
| Secondary Th2 act | 29.3 | HUVEC IFN gamma | 16.5 |
| Secondary Tr1 act | 22.5 | HUVEC TNF alpha + IFN | 9.2 |
| Secondary Th1 rest | 10.3 | HUVEC TNF alpha + ILA | 15.9 |
| Secondary Th2 rest | 7.4 | HUVEC IL-11 | 9.9 |
| Secondary Tr1 rest | 20.3 | Lung Microvascular EC none | 9.2 |
| Primary Th1 act | 9.2 | Lung Microvascular EC TNFalpha + IL-1 beta | 9.9 |
| Primary Th2 act | 16.6 | Microvascular Dermal EC none | 5.9 |
| Primary Tr1 act | 23.7 | Microsvasular Dermal EC TNFalpha + IL-1 beta | 11.7 |
| Primary Th1 rest | 82.4 | Bronchial epithelium TNFalpha + IL1beta | 19.1 |
| Primary Th2 rest | 50.7 | Small airway epithelium none | 7.9 |
| Primary Tr1 rest | 45.1 | Small airway epithelium TNFalpha + IL-1beta | 40.9 |
| CD45RA CD4 lymphocyte act | 19.9 | Coronery artery SMC rest | 7.5 |
| CD45RO CD4 lymphocyte act | 20.0 | Coronery artery SMC TNFalpha + IL-1beta | 0.7 |
| CD8 lymphocyte act | 8.2 | Astrocytes rest | 7.6 |
| Secondary CD8 lymphocyte rest | 24.3 | Astrocytes TNFalpha + IL-1beta | 0.8 |
| Secondary CD8 lymphocyte act | 18.3 | KU-812 (Basophil) rest | 37.6 |
| CD4 lymphocyte none | 9.8 | KU-812 (Basophil) PMA/ionomycin | 75.8 |
| 2ry Th1/Th2/Tr1_anti-CD95 CH11 | 28.3 | CCD1106 (Keratinocytes) none | 13.1 |
| LAK cells rest LAK cells rest | 36.1 | CCD1106 (Keratinocytes) TNFalpha + IL-1beta | 4.3 |
| LAK cells IL-2 | 38.7 | Liver cirrhosis | 9.0 |
| LAK cells IL-2+IL-12 | 36.3 | Lupus kidney | 10.9 |
| LAK cells IL-2+IFN gamma | 65.5 | NCI-H292 none | 36.1 |
| LAK cells IL-2+IL-18 | 30.1 | NCI-H292 IL-4 | 56.3 |
| LAK cells PMA/ionomycin | 11.0 | NCI-H292 IL-9 | 73.2 |
| NK Cells IL-2 rest | 25.0 | NCI-H292 IL-13 | 28.9 |
| Two Way MLR 3 day | 31.9 | NCI-H292 IFN gamma | 31.2 |
| Two Way MLR 5 day | 19.9 | HPAEC none | 15.7 |
| Two Way MLR 7 day | 17.9 | HPAEC TNF alpha + IL-1 beta | 10.1 |
| PBMC rest | 28.9 | Lung fibroblast none | 5.2 |
| PBMC PWM | 58.2 | Lung fibroblast TNF alpha+IL-1 beta | 6.5 |
| PBMC PHA-L | 30.4 | Lung fibroblast IL-4 | 3.7 |
| Ramos (B cell) none | 53.2 | Lung fibroblast IL-9 | 11.8 |
| Ramos (B cell) ionomycin | 71.2 | Lung fibroblast IL-13 | 4.0 |
| B lymphocytes PWM | 100.0 | Lung fibroblast IFN gamma | 17.3 |
| B lymphocytes CD40L and IL-4 | 52.5 | Dermal fibroblast CCD1070 rest | 52.1 |
| EOL-1 dbcAMP | 17.8 | Dermal fibroblast CCD1070 TNF alpha | 78.5 |
| EOL-tdbcAMP PMA/ionomycin | 19.6 | Dermal fibroblast CCD1070 IL-1 beta | 11.9 |
| Dendritic cells none | 10.2 | Dermal fibroblast IFN gam_ m_ a | 4.3 |
| Dendritic tells LPS | 11.0 | Dermal fibroblast IL-4 | 12.0 |
| Dendritic cells anti-CD40 | 15.1 | IBD Colitis 2 | 2.0 |
| Monocytes rest | 20.9 | IBD Crohn's | 2.4 |
| Monocytes LPS | 7.2 | Colon | 55.9 |
| Macrophages rest | 4.7 | Lung | 3.7 |
| Macrophages LPS | 0.0 | Thymus | 60.3 |
| HUVEC none HUVEC none | 16.4 | Kidney | 45.4 |
| HUVEC starved | 37.6 | | |

**CNS_neurodegeneration_v1.0 Summary:** Ag3308 This panel confirms the expression of CG57678-01 gene at low levels in the brain in an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel-v1.4 Summary:** Ag3308 Expression of CG57682-01 gene is highest in kidney (CT = 31.5). However, this gene is expressed at low to moderate levels across the majority of samples on this panel, suggesting that it may play a general role in cellular function and proliferation in a variety of cell types. The CG57682-01 gene encodes a protein with homology to G2/mitotic-specific cyclin B2.

This gene is expressed at low levels in several regions of the brain, including hippocampus, amygdala, cerebral cortex, thalamus, and spinal cord. Therefore, this gene may play a role in central nervous system disorders such as Alzheimer's disease, Parkinson's disease, epilepsy, multiple sclerosis, schizophrenia and depression.

Among tissues with metabolic or endocrine function, this gene is expressed at low levels in adrenal gland, pancreas, adipose, heart and skeletal muscle. Therefore, therapeutic modulation of the activity of this gene or its protein product may prove useful in the treatment of endocrine/metabolically related diseases, such as obesity and diabetes.

In addition, this gene is differentially expressed in fetal (CT = 34) as compared to adult liver (CT = 40) and may be useful for the identification of the fetal phenotype in this tissue.

**Panel 4D Summary:** Ag3308 CG57682-01 gene is expressed at highest levels in stimulated B lymphocytes (CT = 31.7). This gene is expressed at low levels in a wide range of cell types of significance in the immune response in health and disease. These cells include members of the T-cell, B-cell, endothelial cell, basophil, eosinophil, and peripheral blood mononuclear cell family, as well as epithelial and fibroblast cell types from lung and skin, and normal tissues represented by colon, thymus and kidney. This ubiquitous pattern of expression suggests that this gene product may be involved in homeostatic processes for these and other cell types and tissues.

This pattern is in agreement with the expression profile in General_screeninuanel_v1.4 and also suggests a role for the gene product in cell survival and proliferation.

Therefore, therapeutic modulation of the activity of this gene or its protein product may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### NOV48

Expression ofNOV48/CG57713-01 was assessed using the primer-probe set Ag3313, described in Table AOA. Results of the RTQ-PCR runs are shown in Tables AOB, AOC, AOD and AOE.

**Table AOA. Probe Name Ag3313**

| **Primers** | **Sequences** | **Length** | **Start Position** | **SEQ ID NO** |
|---|---|---|---|---|
| Forward | 5'-agcatgtacatgftttcctttgc-3' | 22 | 1162 | 401 |
| Probe | TET-5'-atgcacttttecagtctgcagaageg-3'-TAMRA | 26 | 1187 | 402 |
| Reverse | 5'-tgcaacatttcacttccataca-3' | 22 | 1238 | 403 |

**Table AOB. CNS_ncurodcgcneration_v1.0**

| **Tissue Name** | **Rel. Exp.(%) Ag3313, Run 210143317** | **Tissue Name** | **Rel. Exp.(%) Ag3313, Run 210143317** |
|---|---|---|---|
| AD 1 Hippo | 5.7 | Control (Path) 3 Temporal Ctx | 1.4 |
| AD 2 Hippo | 13.7 | Control (Path) 4 Temporal Ctx | 32.8 |
| AD 3 Hippo | 1.0 | AD 1 Occipital Ctx | 16.2 |
| AD 4 Hippo | 4.0 | AD 2 Occipital Ctx (Missing) | 0.0 |
| AD 5 hippo | 51.1 | AD 3 Occipital Ctx | 3.1 |
| AD 6 Hippo | 0.0 | AD 4 Occipital Ctx | 39.5 |
| Control 2 Hippo | 16.5 | AD 5 Occipital Ctx | 10.9 |
| Control 4 Hippo | 3.7 | AD 6 Occipital Ctx | 63.3 |
| Control (Path) 3 Hippo | 0.7 | Control 1 Occipital Ctx | 1.4 |
| AD 1 Temporal Ctx | 6.8 | Control 2 Occipital Ctx | 33.4 |
| AD 2 Temporal Ctx | 36.6 | Control 3 Occipital Ctx | 19.5 |
| AD 3 Temporal Ctx | 1.2 | Control 4 Occipital Ctx | 2.5 |
| AD 4 Temporal Ctx | 25.3 | Control (Path) 1 Occipital | 80.7 |
| AD 5 Inf Temporal Ctx | 100.0 | Control (Path) 2 Occipital Ctx | 14.5 |
| AD 5 SupTernporal Ctx | 26.6 | Control (Path) 3 Occipital | 0.0 |
| AD 6 Inf Temporal Ctx | 15.4 | Control (Path) 4 Occipital Ctx | 12.6 |
| AD 6 Sup Temporal Ctx | 30.4 | Control 1 Parietal Ctx | 10.1 |
| Control 1 Temporal Ctx | 2.2 | Control 2 Parietal Ctx | 37.1 |
| Control 2 Temporal Ctx | 34.4 | Control 3 Parietal Ctx | 14.7 |
| Control 3 Temporal Ctx | 18.2 | Control (Path) 1 Parietal Ctx | 63.7 |
| Control 4 Temporal Ctx | 11.1 | Control (Path) 2 Parietal Ctx | 32.3 |
| Control (Path) 1 Temporal Ctx | 58.2 | Control (Path) 3 Parietal Ctx | 1.9 |
| Control (Path) 2 Temporal Ctx | 49.7 | Control (Path) 4 Parietal Ctx | 52.1 |

**Table AOC. General_screening_panel_v1.4**

| **Tissue Name** | **Rel. Exp.(%) Ag3313, Run 215648086** | **Tissue Name** | **Rel. Exp.(%) Ag3313, Run 215648086** |
|---|---|---|---|
| Adipose | 0.1 | Renal ca. TK-10 | 0.2 |
| Melanoma* Hs688(A).T | 0.1 | Bladder | 0.3 |
| Melanoma*Hs688(B).T | 0.1 | Gastric ca. (liver met.) NCI-N87 | 0.4 |
| Melanoma*M14 | 1.5 | Gastric ca. KATO III | 0.1 |
| Melanoma* LOXIMVI | 0.1 | Colon ca. SW-948 | 0.0 |
| Melanoma* SK-MEL-5 | 0.1 | Colon ca. SW480 | 0.2 |
| Squamous cell carcinoma SCC-4 | 0.3 | Colon ca.* (SW480 met) SW620 | 0.1 |
| Testis Pool | 0.6 | Colon ca. HT29 | 0.1 |
| Prostate ca.* (bone met) PC-3 | 0.2 | Colon ca.HCT-116 | 30.1 |
| Prostate Pool | 0.2 | Colon ca. CaCo-2 | 7.0 |
| Placenta | 0.3 | Colon cancer tissue | 0.3 |
| Uterus Pool | 0.0 | Colon ca. SW1116 | 0.1 |
| Ovarian ca.OVCAR-3 | 1.6 | Colon ca. Colo-205 | 0.1 |
| Ovarian ca. SK-OV-3 | 6.4 | Colon ca.SW-48 | 0.0 |
| Ovarian ca. OVCAR-4 | 0.4 | Colon Pool | 0.3 |
| Ovarian ca. OVCAR-5 | 0.4 | Small Intestine Pool | 0.5 |
| Ovarian ca. IGROV-1 | 1.7 | Stomach Pool | 0.5 |
| Ovarian ca. OVCAR-8 | 0.0 | Bone Marrow Pool | 0.6 |
| Ovary | 0.1 | Fetal Heart | 0.3 |
| Breast ca. MCF-7 | 0.4 | Heart Pool | 0.1 |
| Breast ca. MDA-MB-231 | 0.7 | Node Pool | 0.3 |
| Breast ca. BT 549 | 3.1 | Fetal Skeletal Muscle | 0.4 |
| Breast ca. T47D | 0.4 | Skeletal Muscle Pool | 0.1 |
| Breast ca. MDA-N | 0.0 | Spleen Pool | 0.4 |
| Breast Pool | 0.2 | Thymus Pool | 0.9 |
| Trachea | 0.5 | CNS cancer (glio/astro) U87- MG | 0.3 |
| Lung | 0.1 | CNS cancer (glio/astro) U-118-MG | 0.3 |
| Fetal Lung | 0.5 | CNS cancer (neuro;met) SK-N-AS | 100.0 |
| Lung ca. NCI-N417 | 1.9 | CNS cancer (astro) SF-539 | 0.7 |
| Lung ca. LX-1 | 0.4 | CNS cancer (astro) SNB-75 | 1.0 |
| Lung ca. NCI-H146 | 3.3 | CNS caneer (glio) SNB-19 | 0.6 |
| Lung ca. SHP-77 | 1.2 | CNS cancer (glio) SF-295 | 0.5 |
| Lung ca. A549 | 13.0 | Brain (Amygdala) Pool | 4.5 |
| Lung ca. NCI-H526 | 0.4 | Brain (cerebellum) | 0.1 |
| Lung ca. NCI-H23 | 5.6 | Brain (fetal) | 7.4 |
| Lung ca. NCI-H460 | 2.9 | Brain (Hippocampus) Pool | 3.3 |
| Lung ca. HOP-62 | 1.1 | Cerebral Cortex Pool | 2.9 |
| Lung ca. NCI-H522 | 3.8 | Brain (Substantia nigra) Pool | 4.5 |
| Liver | 0.0 | Brain (Thalamus) Pool | 4.5 |
| Fetal Liver | 2.4 | Brain (whole) | 2.6 |
| Liver ca. HepG2 | 0.0 | Spinal Cord Pool | 4.3 |
| Kidney Pool | 0.1 | Adrenal Gland | 2.0 |
| Fetal Kidney | 0.7 | Pituitary gland Pool | 5.9 |
| Renal ca. 786-0 | 0.0 | Salivary Gland | 3.2 |
| Renal ca. A498 | 0.0 | Thyroid (female) | 0.3 |
| Renal ca. ACHN | 0.3 | Pancreatic ca. CAPAN2 | 0.8 |
| Renal ca. UO-31 | 0.0 | Pancreas Pool | 0.2 |

**Table AOD. Panel 4D**

| **Tissue Name** | **Rel. Exp.(%) Ag3313, Run 164682847** | **Tissue Name** | **Rel. Exp.(%) Ag3313, Run 164682847** |
|---|---|---|---|
| Secondary Th1 act | 0.2 | HUVEC IL-1beta | 0.1 |
| Secondary Th2 act | 0.2 | HUVECIFN gamma | 0.1 |
| Secondary Tr1 act | 0.0 | HUVEC TNF alpha + IFN gamma | 0.1 |
| Secondary Th1 rest | 0.0 | HUVEC TNF alpha + IL4 | 0.0 |
| Secondary Th2 rest | 0.0 | HUYEC IL-11 | 0.0 |
| Secondary Tr1 rest | 0.2 | Lung Microvascular EC none | 0.1 |
| Primary Th1 act | 0.0 | Lung Microvascular EC TNFalpha + IL-1beta | 0.1 |
| Primary lb2 act | 0.1 | Microvascular Dermal EC none | 0.1 |
| Primary Tr1 act | 0.0 | Microsvasular Dermal EC TNFalpha + IL-1beta | 0.1 |
| Primary Th1 rest | 0.0 | Bronchial epithelium TNFalpha + IL1beta | 0.0 |
| Primary Th2 rest | 0.4 | Small airway epithelium none | 0.1 |
| Primary Tr1 rest | 0.1 | Small airway epithelium TNFalpha + IL-lbeta | 0.4 |
| CD45RA CD4 lymphocyte act | 0.3 | Coronery artery SMC rest | 0.2 |
| CD45RO CD4 lymphocyte act | 0.1 | Coronery artery SMC TNFalpha + IL-1beta | 0.0 |
| CD8 lymphocyte act | 0.1 | Astrocytes rest | 4.9 |
| Secondary CD8 lymphocyte rest | 0.1 | Astrocytes TNFalpha + IL-1beta | 0.9 |
| Secondary CD8 lymphocyte act | 0.3 | KU-812 (Basophil) rest | 81.2 |
| CD4 lymphocyte none | 0.1 | KU-812 (Basophil) PMA/ionomycin | 100.0 |
| 2ry Th1/Th2/Tr1_anti-CD95 CH11 | 0.1 | CCD1106 (Keratinocytes) none | 0.3 |
| LAK cells rest | 0.0 | CCD1106 (Keratinocytes) TNFalpha+IL-1beta | 0.1 |
| LAK cells IL-2 | 0.0 | Liver cirrhosis | 0.2 |
| LAK cells IL-2+IL-12 | 0.2 | Lupus kidney | 0.0 |
| LAK cells IL-2+IFN gamma | 0.4 | NCI-H292 none | 0.2 |
| LAK cells IL-2+IL-18 | 0.3 | NCI-H292 IL-4 | 0.6 |
| LAK cells PMA/ionomycin | 0.3 | NCI-H292IL-9 | 0.1 |
| NK Cells IL-2 rest | 0.0 | NCI-H292 IL-13 | 0.0 |
| Two Way MLR 3 day | 0.2 | NCI-H292 IFN gamma | 0.2 |
| Two Way MLR 5 day | 0.0 | HPAEC none | 0.1 |
| Two Way MLR 7 day | 0.0 | HPAEC TNF alpha + IL-1 beta | 0.1 |
| PBMC rest | 0.0 | Lung fibroblast none | 0.3 |
| PBMC PWM | 0.2 | Lung fibroblast TNF alpha + IL-1 beta | 0.1 |
| PBMC PHA-L | 0.0 | Lung fibroblast IL-4 | 0.3 |
| Ramos (B cell) none | 0.0 | Lung fibroblast IL-9 | 0.4 |
| Ramos (B cell) ionomycin | 0.0 | Lung fibroblast 1L-13 | 1.1 |
| B lymphocytes PWM | 1.3 | Lung fibroblast IFN gamma | 0.3 |
| B lymphocytes CD40L and IL-4 | 0.7 | Dermal fibroblast CCD1070 rest | 0.4 |
| EOL-1dbcAMP | 0.2 | Dermal fibroblast CCD1070 TNF alpha | 0.8 |
| EOL-1dbcAMP PMA/ionomycin | 0.6 | Dermal fibroblast CCD1070 IL-1 beta | 0.1 |
| Dendritic cells none | 0.1 | Dermal fibroblast IFN gamma | 0.1 |
| Dendritic cells LPS | 0.1 | Dermal fibroblast IL-4 | 0.1 |
| Dendritic cells anti-CD40 | 0.1 | IBD Colitis 2 | 0.1 |
| Monocytes rest | 0.0 | IBD Crohn's | 0.3 |
| Monocytes LPS | 0.3 | Colon | 2.7 |
| Macrophages rest | 0.0 | Lung | 1.9 |
| Macrophages LPS | 0.1 | Thymus | 0.5 |
| HUVEC none | 0.0 | Kidney | 1.2 |
| HUVEC starved | 0.1 | | |

**Table AOE Panel 5 Islet**

| **Tissue Name** | **Rel. Exp.(%) Ag3313, Run 242385176** | **Tissue Name** | **Rel. Exp.(%) Ag3313, Run 242385176** |
|---|---|---|---|
| 97457_Patient-02go_adipose | 0.0 | 94709_Donor 2 AM - A_adipose | 0.0 |
| 97476_Patient-07sk_skeletal muscle | 0.0 | 94710_Donor 2 AM - B_adipose | 0.0 |
| 97477_Patient-07ut_uterus | 0.0 | 94711_Donor 2 AM - C_adipose | 0.0 |
| 97478_Patient-07pl_placenta | 9.2 | 94712_Donor 2 AD - A_adipose | 22.2 |
| 99167_Bayer Patient 1 | 0.0 | 94713_Donor 2 AD - B_adipose | 0.0 |
| 97482_Patient-08ut_uterus | 0.0 | 94714_Donor 2 AD-C_adipose | 0.0 |
| 97483 Patient-08pl_placenta | 7.8 | 94742_Donor 3 U - A_Mesenchymal Stern Cells | 0.0 |
| 97486_Patient-09sk_skeletat muscle | 0.0 | 94743_Donor 3 U - B_Mesenchymal Stem Cells | 0.0 |
| 97487_Patient-09ut_uterus | 0.0 | 94730_Donor 3 AM - A_adipose | 0.0 |
| 97488_Patient-09pl_placenta | 0.0 | 94731_Donor 3 AM-B_adipose | 0.0 |
| 97492_Patient-10ut_uterus | 0.0 | 94732_Donor 3 AM - C_adipose | 0.0 |
| 97493_Patient-10pl_placenta | 7.3 | 94733_Donor 3 AD - A_adipose | 13.5 |
| 97495_Patient-11go_adipose | 0.0 | 94734_Donor 3 AD-B_adipose | 0.0 |
| 97496_Patient-11sk_skeletal muscle | 0.0 | 94735_Donor 3 AD-C_adipose | 0.0 |
| 97497_Patient-11ut_uterus | 0.0 | 77138_Liver_HepG2untreated | 0.0 |
| 97498_Patient-11pl_placenta | 0.0 | 73556_Heart_Cardiac stromal cells primary) | 0.0 |
| 97500_Patient-12go_adipose | 6.4 | 81735_Small Intestine | 100.0 |
| 97501_Patient-12sk_skeletal muscle | 7.9 | 72409_Kidney_Proximal Convoluted Tubule | 0.0 |
| 97502_Patient-12ut_uterus | 0.0 | 82685_Small intestine_Duodenum | 0.0 |
| 97503_Patient-12pl_placenta | 0.0 | 90650 Adrenal_Adrenocortical adenoma | 0.0 |
| 94721_Donor 2U - A_Mesenchymal Stern Cells | 0.0 | 72410 Kidney_HRCE | 9.5 |
| 94722_Donor 2 U - B_Mesenehymal Stem Cells | 7.2 | 72411_Kidney_HRE | 0.0 |
| 94723_Donor 2 U - C_Mesenchymal Stem Cells | 0.0 | 73139_Uterus_Uterine smooth muscle cells | 0.0 |

CNS_neurodegeneration_v1.0 Summary: Ag3313 This panel confirms the expression of CG5 7713-01 gene at low to moderate levels in the brains of an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

General_screening_panel_v1.4 Summary: Ag3313 Expression of the CG57713-01 gene is highest in CNS cancer cell line SK-N-AS (CT = 26.1). Expression of this gene also appears to be upregulated in a number of lung cancer cell lines and ovarian cancer cell lines when compared to the normal tissues. Therefore, therapeutic modulation of the activity of this gene or its protein product may be of use in the treatment of lung or ovarian cancer.

In addition, this gene is expressed at moderate levels in most regions of the central nervous system examined, including amygdala, hippocampus, cerebral cortex, substantia nigra, thalamus and spinal cord. The CGS7713-01 gene encodes a protein with homology to sodium/bile acid cotransporters. Changes in expression of sodium/bile acid cotransporters have been found to be associated with methamphetamine (METH)-induced dopamine (DA). neurotoxicity (ref. 1). In addition, based on the expression of the CG57713-01 gene in the brain, this gene may play a role in central nervous system disorders such as Alzheimer's disease, Parkinson's disease, epilepsy, multiple sclerosis, schizophrenia and depression.

Among tissues with metabolic or endocrine function, this gene is expressed at moderate levels in adrenal gland and pituitary gland and at low levels in pancreas, thyroid, fetal heart, fetal skeletal muscle, and fetal liver. Therefore, therapeutic modulation of the activity of this gene may prove useful in the treatment of endocrine/metabolically related diseases, such as obesity and diabetes. Interestingly, expression of this gene is much higher in fetal liver (CT = 31.5) than in adult liver (CT = 40), suggesting that expression of this gene can be used to distinguish these two tissues.

### References:

### 1. Xie T, Tong L, Barrett T, Yuan J, Hatzidimitriou G, McCann UD, Becker KG, Donovan DM, Ricaurte GA. Changes in gene expression linked to methamphetamine induced dopaminergic neurotoxicity. J Neurosci 2002 Jan 1;22(1):274-83

The purpose of these studies was to examine the role of gene expression in methamphetamine (METIT)-induced dopamine (DA) neurotoxicity. First, the effects of the mRNA synthesis inhibitor, actinomycin-D, and the protein synthesis inhibitor, cycloheximide, were examined. Both agents afforded complete protection against METH-induced DA neurotoxicity and did so independently of effects on core temperature, DA transporter function, or METH brain levels, suggesting that gene transcription and mRNA translation play a role in METH neurotoxicity. Next, microarray technology, in combination with an experimental approach designed to facilitate recognition of relevant gene expression patterns, was used to identify gene products linked to METH-induced DA neurotoxicity. This led to the identification of several genes in the ventral midbrain associated with the neurotoxic process, including genes for energy metabolism [cytochrome c oxidase subunit 1 (COX1), reduced nicotinamide adenine dinucleotide ubiquinone oxidoreductase chain 2, and phosphoglycerate mutase B], ion regulation (members of sodium/hydrogen exchanger and sodium/bile acid cotransporter family), signal transduction(adenylyl cyclase III), and cell differentiation and degeneration (N-myc downstream-regulated gene 3 and tau protein). Of these differentially expressed genes, we elected to further examine the increase in COX1 expression, because of data implicating energy utilization in METH neurotoxicity and the known role of COX1 in energy metabolism. On the basis of time course studies, Northern blot analyses, in situ hybridization results, and temperature studies, we now report that increased COX1 expression in the ventral midbrain is linked to METH induced DA neuronal injury. The precise role of COX1 and other genes in METH neurotoxicity remains to be elucidated.

### PMID: 11756511

**Panel 4D Summary:** Ag3313 The CG57713-01 transcript is expressed at highest levels in both PMA/ionomycin-treated KU-812 basophil cell line and in untreated KU-812 cells (CTs = 28). Therefore, expression of this gene can be used to distinguish basophils from the other samples on this panel. In addition, small molecule therapies designed with the protein encoded for by this gene could block or inhibit inflammation or tissue damage due to basophil activation in response to asthma, allergies, hypersensitivity reactions, psoriasis, and viral infections.

In addition, expression of this gene is higher in normal colon (CT = 32.9) than in colon samples from patients with IBD Crohn's (CT = 36) or IBD colitis (CT = 37). The CG57713-01 gene encodes a protein with homology to sodium/bile acid cotransporters. Defects in the sodium/bile acid cotransporter are one of the causative agents for Crohn's disease (Ref 1). Thus, therapeutic modulation of this novel cotransporter may be of use in the treatment of Crohn's disease.

### References.

### 1. Wong MH, Oelkers P, Dawson PA. (1995) Identification of a mutation in the ileal sodium-dependent bile acid transporter gene that abolishes transport activity. J Biol Chem. 270(45):27228-34.

The ileal Na+/bile acid cotransporter plays a critical role in the reabsorption of bile acids from the small intestine. In the course of cloning and characterizing the human ileal Na+/bile acid cotransporter cDNA, a dysfunctional isoform was identified in a patient diagnosed with Crohn's disease. Expression studies using hamster-human ileal Na+/bile acid cotransporter cDNA chimeras narrowed the location of the defect to the carboxyl-terminal 94 amino acids. Comparison of the sequence of the dysfunctional isoform to that of a wild-type human ileal Na+/bile acid cotransporter genomic clone revealed a single C to T transition resulting in a proline to serine substitution at amino acid position 290. The inheritance of this mutation in the proband's family was confirmed by single-stranded conformation polymorphism analysis and DNA sequencing. In transfected COS-1 cells, the single amino acid change abolished taurocholate transport activity but did not alter the transporter's synthesis or subcellular distribution. This dysfunctional mutation represents the first known molecular defect in a human sodium-dependent bile acid transporter.

### PMID: 7592981

Panel 5 Islet Summary: Ag3313 A low level of expression of the CG57713-01 gene is seen in a sample derived from small intestine (CT=34), which is confirmed by the expression observed in panel 1.4. Defects in the sodium/bile acid cotransporter are one of the causative agent for Crohn's disease (Ref 1). Thus, therapeutic modulation of this novel cotransporter may be of use in the treatment of Crohn's disease.

### References.

### 1. Wong MH, Oelkers P, Dawson PA. (1995) Identification of a mutation in the ileal sodium-dependent bile acid transporter gene that abolishes transport activity. J Biol Chem. 270(45):27228-34.

The ileal Na+/bile acid cotransporter plays a critical role in the reabsorption of bile acids from the small intestine. In the course of cloning and characterizing the human ileal Na+/bile acid cotransporter cDNA, a dysfunctional isoform was identified in a patient diagnosed with Crohn's disease. Expression studies using hamster-human ieal Na+/bile acid cotransporter cDNA chimeras narrowed the location of the defect to the carboxyl-terminal 94 amino acids. Comparison of the sequence of the dysfunctional isoform to that of a wild-type human ileal Na+/bile acid cotransporter genomic clone revealed a single C to T transition resulting in,a proline to serine substitution at amino acid position 290. The inheritance of this mutation in the proband's family was confirmed by single-stranded conformation polymorphism analysis and DNA sequencing. In transfected COS-1 cells, the single amino acid change abolished taurocholate transport activity but did not alter the transporter's synthesis or subcellular distribution. This dysfunctional mutation represents the first known molecular defect in a human sodium-dependent bile acid transporter.

### PMID: 7592981

### NOV49

Expression of NOV49/CG57721-01 was assessed using the primer-probe set Ag3315, described in Table APA. Results of the RTQ-PCR runs are shown in Tables APB, APC and APD.

**Table APA. Probe Name Ag3315**

| **Primers** | **Sequences** | **Length** | **Start Position** | **SEQ ID NO** |
|---|---|---|---|---|
| Forward | 5'-atctacctctccgagtccttca-3' | 22 | 652 | 404 |
| Probe | TET-5'-ctgcagatcctgatttcggtgctcaa-3'-TAMRA | 26 | 700 | 405 |
| Reverse | 5'-gatggtcagtccgaagatgtac-3' | 22 | 726 | 406 |

**Table APB. CNS_neurodegeneration_v1.0**

| **Tissue Name** | **Rel. Exp.(%) Ag3315, Run 210138297** | **Tissue Name** | **Rel. Exp.(%) Ag3315, Run 210138297** |
|---|---|---|---|
| AD 1 Hippo | 17.1 | Control (Path) 3 Temporal Ctx | 4.9 |
| AD 2 Hippo | 0.0 | Control (Path) 4 Temporal Ctx | 13.5 |
| AD 3 Hippo | 6.4 | AD 1 Occipital Ctx | 7.0 |
| AD 4 Hippo | 37.6 | AD 2 Occipital Ctx (Missing) | 0.0 |
| AD 5 hippo | 12.7 | AD 3 Occipital Ctx | 0.0 |
| AD 6 Hippo | 92.7 | AD 4 Occipital Ctx | 17.1 |
| Control 2 Hippo | 0.0 | AD5 Occipital Ctx | 31.6 |
| Control 4 Hippo | 18.3 | AD 6 Occipital Ctx | 11.6 |
| Control (Path) 3 Hippo | 0.0 | Control 1 Occipital Ctx | 0.0 |
| AD 1 Temporal Ctx | 0.0 | Control 2 Occipital Ctx | 18.9 |
| AD 2 Tamporal Ctx | 0.0 | Control 3 Occipital Ctx | 0.0 |
| AD 3 Temporal Ctx | 0.0 | Control 4 Occipital Ctx | 12.2 |
| AD 4 Temporal Ctx | 29.5 | Control (Path) 1 Occipital | 40.6 |
| AD 5 Inf Temporal Ctx | 11.7 | Control (Path) 2 Occipital | 9.3 |
| AD 5 SupTemporal Ctx | 30.6 | Control (Path) 3 Occipital Ctx | 5.2 |
| AD 6 Inf Temporal Ctx | 80.7 | Control (Path) 4 Occipital | 19.2 |
| AD 6 Sup Temporal Ctx | 100.0 | Control 1 Parietal Ctx | 0.0 |
| Control 1 Temporal Ctx | 0.0 | Control 2 Parietal Ctx | 29.7 |
| Control 2 Temporal Ctx | 5.5 | Control 3 Parietal Ctx | 14.4 |
| Control 3 Temporal Ctx | 5.0 | Control (Path) 1 Parietal Ctx | 11.5 |
| Control 4 Temporal Ctx | 13.8 | Control (Path) 2 Parietal Ctx | 5.5 |
| Control (Path) Temporal Ctx | 31.4 | Control (Path) 3 Parietal Ctx | 0.0 |
| Control (Path) 2 Temporal Ctx | 24.1 | Control (Path) 4 Parietal Ctx | 11.3 |

**Table APC. General_screening_panel_v1.4**

| **Tissue Name** | **Rel. Exp.(%) Ag3315, Run 215678587** | **Tissue Name** | **Rel. Exp.(%) Ag3315, Run 215678587** |
|---|---|---|---|
| Adipose | 0.2 | Renal ca. TK 10 | 0.3 |
| Melanoma* Hs688(A).T | 0.0 | Bladder | 2.2 |
| Melanoma* Hs688(B).T | 0.0 | Gastc ca. (liver met.) NCI-N87 | 12.7 |
| Melanoma* M14 | 0.0 | Gastric ca. KATO III | 100.0 |
| Melanoma* LOXIMVI | 0.1 | Colon ca. SW-948 | 0.0 |
| Melanoma* SK-MEL-5 | 0.0 | Colon ca. SW480 | 7.1 |
| Squamous cell carcinoma SCC-4 | 0.1 | Colon ca.* (SW480 met) 0.1 SW620 | 0.0 |
| Testis Pool | 0.1 | Colon ca.HT29 | 0.9 |
| Proteta ca.*(bone met) PC-3 | 0.0 | Colon ca. HCT-116 | 0.0 |
| Prostate Pool | 0.0 | Colon ca. CaCo-2 | 0.1 |
| Placenta | 0.0 | Colon cancer tissue | 0.4 |
| UterusPool | 0.1 | Colon ca. SW1116 | 0.0 |
| Ovarian ca. OVCAR-3 | 0.4 | Colon ca. Colo-205 | 13.3 |
| Ovarian ca. SK-OV-3 | 0.2 | Colon ca. SW-48 | 0.0 |
| Ovarianca.OVCAR-4 | 0.2 | Colon Pool | 0.1 |
| Ovarian ca. OVCAR-5 | 6.6 | Small Intestine Pool | 0.2 |
| Ovarian ca. IGROV-1 | 3.2 | Stomach Pool | 6.7 |
| Ovarian ca.OVCAR-8 | 0.1 | Bone Marrow Pool | 0.0 |
| Ovary | 0.9 | Fetal Heart | 0.1 |
| Breast ca. MCF-7 | 0.2 | Heart Pool | 0.1 |
| Breast ca. MDA-MB-231 | 0.2 | Lymph Node Pool | 0.2 |
| Breast ca. BT 549 | 0.2 | Fetal Skeletal Muscle | 0.0 |
| Breast ca. T47D | 7.3 | Skeletal Muscle Pool | 0.4 |
| Breast ca. MDA.N | 0.0 | Spleen Pool | 0.0 |
| Breast Pool | 0.0 | Thymus Pool | 0.1 |
| Trachea | 3.3 | CNS cancer (glio/astro) U87- MG | 0.2 |
| Lung | 0.1 | CNS cancer (glio/astro) U-118-MG | 0.0 |
| Fetal Lung | 6.0 | CNS cancer (neuro;met) SK-N-AS | 0.0 |
| Lung ca. NCI-N417 | 0.0 | CNS cancer (astro) SF-539 | 0.1 |
| Lung ca. LX-1 | 0.6 | CNS cancer (astro) SNB-75 | 0.2 |
| Lung ca. NCI-H146 | 0.0 | CNS cancer (glio) SNB-19 | 2,6 |
| Lung ca. SHP-77 | 0.0 | CNS cancer (glio) SF-295 | 0.2 |
| Lung ca. A549 | 1.4 | Brain (Amygdala) Pool | 0.0 |
| Lung ca. NCI-H526 | 0.1 | Brain (cerebellum) | 0.0 |
| Lung ca.NCI-H23 | 0.0 | Brain (fetal) | 0.2 |
| Lung ca. NCI-H460 | 0.1 | Brain (Hippocampus) Pool | 0.0 |
| Lung ca. HOP-62 | 0.3 | Cerebral Cortex Pool | 0.1 |
| Lung ca. NCI-H522 | 0.0 | Brain (Substantia nigra) Pool | 0.2 |
| Liver | 0.0 | Brain(Thalamus) Pool | 0.1 |
| Fetal Liver , | 0.1 | Brain (whole) | 0.1 |
| Liver ca. HepG2 | 0.5 | Spinal Cord Pool | 0.3 |
| Kidney Foot | 0.2 | Adrenal Gland | 0.0 |
| Fetat Kidney | 0.1 | Pituitary gland Pool | 0.1 |
| Renal ca.786-0 | 0.1 | Salivary Gland | 79.6 |
| Renal ca. A498 | 0.1 | Thyroid (female) | 1.5 |
| Renatea-ACHN | 0.1 | Pancreatic ca. CAPAN2 | 7.2 |
| Renal ca. UO-31 | 0.1 | Pancreas Pool | 0.7 |

**Table APD. Panel 4D**

| **Tissue Name** | **Rel. Exp.(%) Ag3315, Run 164683047** | **Tissue Name** | **Rel. Exp.(%) Ag3315, Run 164683047** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha+IFN gamma | 0.0 |
| Secondary Th1 rest | 0.0 | HUVEC TNF alpha + IL4 | 1.3 |
| Secondary Th2 rest | 1.4 | HUVEC IL-11 | 3.8 |
| Secondary Tr1 rest | 1.5 | Lung Merovascular EC none | 0.0 |
| Primary Th1 act | 0.0 | Lung Microvascutar EC TNFalpha +IL-1beta | 0.0 |
| Primary Th2 act | 0.0 | Microvascular Dermal EC none | 2.7 |
| Primary Tr1 act | 0.0 | Microsvasular Dermal EC TNFalpha + IL-1beta | 2.9 |
| Primary Th1 rest | 0.0 | Bronchial epithelium TNFalpha+ IL1beta | 2.8 |
| Primary Th2 rest | 0.0 | Small airway epithelium none | 4.1 |
| Primary Tr1 rest | 0.0 | Small airway epithelium TNFalpha +iL-1beta | 100.0 |
| CD45RA CD4 lymphocyte act | 0.0 | Caronery artery SMC rest | 1.4 |
| CD45RO CD4 lymphocyte act | 0.0 | Coronery artery SMC TNFalpha+ IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 1.6 | KU-812 (Basophil) rest | 2.8 |
| CD4 lymphocyte none | 1.7 | KU-812 (Basophil) PMA/ionomycin | 0.0 |
| 2ry Th1/Th2/Tr1_anti-CD95 CH11 | 0.0 | CCD1106 (Keratinocytes)none | 0.0 |
| LAK cells rest | 0.0 | CCD1106 (Keratinocytes) TNFalpha+IL-1beta | 0.0 |
| LAK cells IL-2 | 0.0 | Liver cirrhosis | 3.1 |
| LAK cells IL-2+IL-12 | 0.0 | Lupus kidney | 0.0 |
| LAK cells IL-2+IFN gamma | 0.0 | NCI-H292 none | 0.0 |
| LAK cells IL-2+IL-18 | 0.0 | NCI-H292 IL-4 | 1.5 |
| LAK cells PMA/ionomycin | 1.4 | NCI-H292 IL-9 | 2.1 |
| NK Cells IL-2 rest | 0.0 | NCI-H292 IL-13 | 0.0 |
| Two Way MLR 3 day | 0.0 | NCI-H292 IFN gamma | 1.5 |
| Two Way MLR5 day | 0.0 | HPAEC none | 2.6 |
| Two Way MLR 7 day | 0.0 | HPAEC TNF alpha + IL-1 beta | 1.6 |
| PBMC rest | 0.0 | Lung fibroblast none | 0.0 |
| PBMC PWM | 1.5 | Lung fibroblast TNF alpha + IL-1 | 0.0 |
| PBMC PHA-L | 0.0 | Lung fibroblast IL-4 | 0.0 |
| Ramos (B cell) none | 0.0 | Lung fibroblast IL-9 | 0.0 |
| Ramos (B cell) ionomycin | 3.3 | Lung fibroblast IL-13 | 0.0 |
| B lymphocytes PWM | 1.5 | Lung fibroblast IFN gamma | 0.0 |
| B lymphocytes CD40L and IL-4 | 0.0 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 1.4 | Dermal fibroblast CCD1070 IL-1 beta | 0.0 |
| Dendritic cells none | 0.0 | Dermal fibroblast IFN gamma | 0.0 |
| Dendritic cells LPS | 3.1 | Dermal fibroblast IL-4 | 0.9 |
| Dendritic cells anti-CD40 | 0.0 | IBD Colitis 2 | 0.9 |
| Monocytes rest | 0.0 | IBD Crohn's | 0.0 |
| Monocytes LPS | 3.2 | Colon | 1.4 |
| Macrophages rest | 0.0 | Lung | 39.0 |
| Macrophages LPS | 1.9 | Thymus | 5.7 |
| HUVEC none | 0.0 | Kidney | 6.9 |
| HUVEC starved | 6.7 | | |

**CNS_neurodegeneration_v1.0 Summary:** Ag3315 This panel confirms the expression of the CG57721-01 gene at low levels in the brains of an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel_v1.4** Summary: Ag3315 Expression of the CG57721-01 gene is highest in gastric cancer cell line KATO III and in salivary gland (CTs = 25). Therefore, expression of this gene can be used to distinguish these samples from the other samples on this panel. Interestingly, this gene is expressed at much higher levels in fetal lung (CT = 29.3) than in adult lung (CT = 35.1), suggesting that expression of this gene may be used to distinguish fetal from adult lung.

In addition, this gene is expressed at low levels in some regions of the central nervous system, including cerebral cortex, substantia nigra, and spinal cord. Therefore, this gene may play a role in central nervous system disorders such as Alzheimer's disease, Parkinson's disease, epilepsy, multiple sclerosis, schizophrenia and depression. The CG57721-01 gene encodes a protein with similarity to the mouse prestin gene, a putative anion transporter protein. Prestin has been demonstrated to act as a motor protein of cochlear outer hair cells and presumably plays an important role in hearing (Ref. 1,2). Based on the similarity of the CG57721-01 gene to prestin, therapeutic modulation of the activity of this gene or its protein product may be of benefit in the treatment of deafness.

Finally, among tissues with metabolic or endocrine function, this gene is expressed at low levels in pancreas, thyroid, skeletal muscle, and adipose. Therefore, therapeutic modulation of the activity of this gene may prove useful in the treatment of endocrine/metabolically related diseases, such as obesity and diabetes. This gene is expressed in the thyroid and is also similar to the pendrin gene. Mutatations in the pendrin gene are known to cause the human disease Pendred syndrome (Ref. 3). Therefore, therapeutic modulatin of this gene may be useful in treatment of Pendred syndrome and related disorders.

### References:

### 1. Zheng J, Shen W, He DZ, Long KB, Madison LD, Dallos P. (2000) Prestin is the motor protein of cochlear outer hair cells. Nature 405(6783):149-55

The outer and inner hair cells of the mammalian cochlea perform different functions. In response to changes in membrane potential, the cylindrical outer hair cell rapidly alters its length and stiffness. These mechanical changes, driven by putative molecular motors, are assumed to produce amplification of vibrations in the cochlea that are transduced by inner hair cells. Here we have identified an abundant complementary DNA from a gene, designated Prestin, which is specifically expressed in outer hair cells. Regions of the encoded protein show moderate sequence similarity to pendrin and related sulphate/anion transport proteins. Voltage-induced shape changes can be elicited in cultured human kidney cells that express prestin. The mechanical response of outer hair cells to voltage change is accompanied by a 'gating current', which is manifested as nonlinear capacitance. We also demonstrate this nonlinear capacitance in transfected kidney cells. We conclude that prestin is the motor protein of the cochlear outer hair cell.

### PMID: 10821263

### 2. Peter Dallos & Bernd Fakler (2002) Prestin, a new type of motor protein. Nature Reviews Molecular Cell Biology 3,104 -111.

Prestin, a transmembrane protein found in the outer hair cells of the cochlea, represents a new type of molecular motor, which is likely to be of great interest to molecular cell biologists. In contrast to enzymatic-activity-based motors, prestin is a direct voltage-to-force converter, which uses cytoplasmic anions as extrinsic voltage sensors and can operate at microsecond rates. As prestin mediates changes in outer hair cell length in response to membrane potential variations, it might be responsible for sound amplification in the mammalian hearing organ.

### 3. Waldegger S, Moschen I, Ramirez A, Smith RJ, Ayadi H, Lang F, Kubisch C. (2001) Cloning and characterization of SLC26A6, a novel member of the solute carrier 26 gene family. Genomics. 72(1):43-50.

The SLC26 gene family (solute carrier family 26) comprises five mammalian genes that encode anion transporter-related proteins. In addition to sat-1 and prestin, which were cloned from rat and gerbil, respectively, three human members have been identified and associated with specific genetic diseases (DTD, diastrophic dysplasia; CLD, congenital chloride diarrhea; PDS, Pendred syndrome). In this study we used a homology approach combined with RACE PCR to identify human SLC26A6, the sixth member of this gene family. Northern blot analysis showed the highest SLC26A6 transcript levels in kidney and pancreas. Expression in MDCK cells and in Xenopus oocytes demonstrated trafficking of the SLC26A6 protein to the cell membrane but did not reveal anion transport activity with tracer uptake or intracellular pH measurements. We determined the genomic structure of the SLC26A6 gene and excluded mutations in the 21 coding exons as the cause of DFNB6 and USH2B, which closely map to the SLC26A6 chromosomal locus (3p21).

### PMID: 11247665

**Panel 4D Summary:** Ag3315 Expression of the CG57721-01 gene is limited to small airway epithelium (CT = 35) and normal lung (CT = 31.7) on this panel. Interestingly, expression of this gene in small airway epithelium is strongly upregulated by treatment with TNF-alpha and IL-1 beta (CT = 30.4). This observation suggests that expression of this gene could be used as a marker for activated epithelium. Furthermore, therapeutic modulation of the activity of this gene or its protein product using small molecule drugs could be of use in the treatment of asthma and emphysema.

### NOV50

Expression of NOV50/CG57787-01 was assessed using the primer-probe sets Ag3332, Ag2005 and Ag2259, described in Tables AQA, AQB and AQC. Results of the RTQ-PCR runs are shown in Tables AQD, AQE, AQF and AQG.

**Table AOA. Probe Name Ag3332**

| **Primers** | **Sequences** | **Length** | **Start Position** | **SEQ ID NO** |
|---|---|---|---|---|
| Forward | 5'-atttcctaccccgtcattaaag-3' | 22 | 67 | 407 |
| Probe | TET-5'-caccatcggctttggacagatcaag-3'-TAMRA | 25 | 111 | 408 |
| Reverse | 5'-gttctgtagtcccagcaggtt-3' | 21 | 136 | 409 |

**Table AOB. Probe Name Ag2005**

| **Primers** | **Sequences** | **Length** | **Start Position** | **SEQ ID NO** |
|---|---|---|---|---|
| Forward | 5'-gttcctgctggacttcatttc-3' | 21 | 51 | 410 |
| Probe | TET-5'-accccgtcattaaaggcttcacctct-3'-TAMRA | 26 | 74 | 411 |
| Reverse | 5'-caggttcttgatctgtccaaag-3' | 22 | 120 | 412 |

**Table AOC. Probe Name Ag2259**

| **Primers** | **Sequences** | **Length** | **Start Position** | **SEQ ID NO** |
|---|---|---|---|---|
| Forward | 5'-gttcctgctggacttcatttc-3' | 21 | 51 | 413 |
| Probe | TET-5'-accccgtcattaaaggcttcacctct-3'-TAMRA | 26 | 74 | 414 |
| Reverse | 5'-caggttcttgatctgtccaaag-3' | 22 | 120 | 415 |

**Table AOD. CNS_neurodegeneration_v1.0**

| **Tissue Name** | **Rel. Exp.(%) Ag3332, Run 210146364** | **Tissue Name** | **Rel. Exp.(%) Ag3332, Run 210146364** |
|---|---|---|---|
| AD 1 Hippo | 16.4 | Control (Path) 3 Temporal Ctx | 3.9 |
| AD 2 Hippo | 56.6 | Control (Path) 4 Temporal Ctx | 44.4 |
| AD 3 Hippo | 7.1 | AD 1 Occipital Ctx | 15.6 |
| AD 4 Hippo | 13.2 | AD 2 Occipital Ctx (Missing) | 0.0 |
| AD 5 hippo | 97.3 | AD 3 Occipital Ctx | 5.7 |
| AD 6 Hippo | 59.0 | AD 4 Occipital Ctx | 28.9 |
| Control 2 Hippo | 40.9 | AD 5 Occipital Ctx | 27.5 |
| Control 4 Hippo | 23.7 | AD 6 Occipital Ctx | 88.3 |
| Control (Path) 3 Hippo | 8.7 | Control 1 Occipital Ctx | 2.6 |
| AD 1 Tempotal Ctx | 18.7 | Control 2 Occipital Ctx | 53.6 |
| AD 2 Temporal Ctx | 45.1 | Control 3, Occipital Ctx | 19.1 |
| AD 3 Temporal Ctx | 9.9 | Control 4 Occipital Ctx | 9.9 |
| AD 4 Temporal Ctx | 40.9 | Control (Path) Occipital Ctx | 81.8 |
| AD 5 Inf Temporal Ctx | 100.0 | Controt(Path)2 Occipital Ctx | 9.5 |
| AD 5 Sup Temporal Cbx | 87.1 | Control (Path) 3 Occipital Ctx | 3.4 |
| AD 6 Inf Temporal Ctx | 62.4 | Control (Path) 4 Occipital Ctx | 16.5 |
| AD 6 Sup Temporal Ctx | 62.0 | Control 1 Parietal Ctx | 6.6 |
| Control 1 Temporal Ctx | 5.3 | Control 2 Parietal Ctx | 83.5 |
| Control 2 Temporal Ctx | 46.7 | Control 3 Parietal Ctx | 17.8 |
| Control 3 Temporal Ctx | 31.6 | Control (Path) 1 Parietal Ctx | 99.3 |
| Control 4 Temporal Ctx | 14.7 | Control (Path) 2 Parietal Ctx | 19.9 |
| Control (Path) 1 Temporal Ctx | 84.1 | Control (Path) 3 Parietal Ctx | 3.7 |
| Control (Path) 2 Temporal Ctx | 41.8 | Control (Path) 4 Parietal Ctx | 47.3 |

**Table AOE. General_screening_panel_v1.4**

| **Tissue Name** | | **Tissue Name** | |
|---|---|---|---|
| | 215678734 | | 215678734 |
| Adipose | 6.6 | Renal ca. TK-10 | 100.0 |
| Melanoma* Hs688(A).T | 28.5 | Bladder | 18.0 |
| Melanoma* Hs688(B).T | 31.0 | Gastric ca. (liver met.) NCI-N87 | 17.1 |
| Melanoma* M14 | 30.4 | Gastric ca. KATO III | 19.3 |
| Melanoma* LOXIMVI | 22.5 | Colon ca. SW-948 | 9.0 |
| Melanoma* SK-MEL-5 | 30.1 | Colon ca. SW480 | 0.7 |
| Squamous cell carcinoma SCC-4 | 0.2 | Colon ca.* (SW480 met) SW620 | 1.9 |
| Testis Pool | 5.3 | Colon ca. HT29 | 4.9 |
| Prostate ca.* (bone met) PC-3 | 53.6 | Colon ca. HCT-116 | 28.9 |
| Prostate Pool | 12.7 | Colon ca. CaCo-2 | 49.0 |
| Placenta | 55.9 | Colon cancer tissue | 12.9 |
| Uterus Pool | 3.7 | Colon ca. SW1116 | 6.8 |
| Ovarian ca. OVCAR-3 | 42.0 | Colon ca. Colo-205 | 0.0 |
| Ovarian ca. SK-OV-3 | 40.6 | Colon ca. SW-48 | 16.7 |
| Ovarian ca. OVCAR-4 | 19.9 | Colon Pool | 26.6 |
| Ovarian ca. OVCAR-5 | 57.0 | Small Intestine Pool | 46.0 |
| Ovarian ca. IGROV-1 | 30.4 | Stomach Pool | 13.7 |
| Ovarian ca. OVCAR-8 | 9.7 | Bone Marrow Pool | 5.9 |
| Ovary | 18.3 | Fetal Heart | 14.0 |
| Breast ca.MCF-7 | 35.1 | Heart Pool | 10.0 |
| Breast ca. MDA-MB-231, | 19.5 | Lymph Node Pool | 16.8 |
| Breast ca. BT 549 | 37.4 | Fetal Skeletal Muscle | 4.2 |
| Breast ca. T47D | 97.3 | Skeletal Muscle Pool | 6.9 |
| Breast ca. MDA-N | 13.6 | Spleen Pool | 17.7 |
| Breast Pool | 22.7 | Thymus Pool | 14.8 |
| Trachea | 21.0 | CNS cancer (glio/astro) U87-MG | 9.0 |
| Lung | 4.0 | CNS cancer (glio/astro) U-118-MG | 24.7 |
| Fetal Lung | 53.2 | CNS cancer (neuro;met) SK-N-AS | 79.6 |
| Lung ca. NCI-N417 | 0.9 | CNS cancer (astro) SF-539 | 3.7 |
| Lung ca. LX-1 | 0.8 | CNS cancer (astro) SNB-75 | 54.7 |
| Lung ca. NCI-H146 | 12.4 | CNS cancer (glio) SNB-19 | 24.0 |
| Lung ca. SHP-77 | 24.3 | CNS cancer (glio) SF-295 | 58.6 |
| Lung ca. A549 | 33.9 | Brain (Amygdala) Pool | 13.9 |
| Lung ca. NCI-H526 | 20.0 | Brain (cerebellum) | 14.9 |
| Lung ca. NCI-H23 | 30.4 | Brain (fetal) | 40.3 |
| Lung ca. NCI-H460 | 34.9 | Brain (Hippocampus) Pool | 25.5 |
| Lung ca. HOP-62 | 4.0 | Cerebral Cortex Pool | 23.8 |
| Lung ca. NCI-H522 | 72.2 | Brain (Substantia nigra) Pool | 35.4 |
| Liver | 5.0 | Brain (Thalamus) Pool | 29.9 |
| Fetal Liver | 17.4 | Brain (whole) | 10.6 |
| Liver ca.HepG2 | 26.4 | Spinal Cord Pool | 24.8 |
| Kidney Pool | 35.6 | Adrenal Gland | 65.1 |
| Fetal Kidney | 14.6 | Pituitary gland Pool | 17.0 |
| Renal ca. 786-0 | 0.9 | Salivary Gland | 11.6 |
| Renal ca. A498 | 16.4 | Thyroid (female) | 17.9 |
| Renal ca. ACHN | 11.4 | Pancreatic ca. CAPAN2 | 2.4 |
| Renal ca. UO-31 | 4.6 | Pancreas Pool | 21.0 |

**Table AOF. Panel 1.3D**

| **Tissue Name** | R**el.Exp.(%) Ag2005, Run 165981814** | **Tissue Name** | **Rel. Exp.(%) Ag2005, Run 165981814** |
|---|---|---|---|
| Liver adenocarcinoma | 36.6 | Kidney (fetal) | 33.2 |
| Pancreas | 14.4 | Renal ca. 786-0 | 0.0 |
| Pancreatic ca. CAPAN 2 | 0.9 | Renal ca. A498 | 12.8 |
| Adrenal gland | 46.3 | Renal ca. RXF 393 | 20.9 |
| Thyroid | 9.6 | Renal ca. ACHN | 3.3 |
| Salivary gland | 20.7 | Renal ca. UO-31 | 5.4 |
| Pituitary gland | 58.2 | Renal ca. TK-10 | 40.1 |
| Brain (fetal) | 28.7 | Liver | 3.1 |
| Brain (whole) | 92.7 | Liver (fetal) | 10.7 |
| Brain (amygdala) | 78.5 | Liver ca. (hepatoblast) HepG2 | 15.6 |
| Brain (cerebellum) | 12.7 | Lung | 27.9 |
| Brain (hippocampus) | 53.2 | Lung (fetal) | 53.2 |
| Brain (substantia nigra) | 52.1 | Lung ca. (small cell) LX-1 | 0.0 |
| Brain (thalamus) | 90.8 | Lung ca. (small cell) NCI-H69 | 6.6 |
| Cerebral Cortex | 100.0 | Lung ca. (s.cell var.) SHP-77 | 22.4 |
| Spinal cord | 56.6 | Lung ca. (large cell)NCI-H460 | 15.9 |
| glio/astro U87-MG | 6.0 | Lung ca. (non-sm. cell) A549 | 9.8 |
| glio/astro U-118-MG | 7.6 | Lung ca. (non-s.cell) NCI-H23 | 13.7 |
| astrocytoma SW1783 | 6.4 | Lung ca. (non-s.cell) HOP- | 8.4 |
| neuro*; met SK-N-AS | 26.8 | Lung ca. (non-s.cl) NCI-H522 | 15.8 |
| astrocytomaSF-539 | 3.1 | Lung ca. (squam.) SW 90 | 28.1 |
| astrocytonia SNB-75 | 12.9 | Lung ca.(squam.) NCI-H596 | 2.6 |
| glioma SNB-19 | 13.1 | Mammary gland | 10.5 |
| glioma U251 | 8.2 | Breast ca.*(pl.ef) MCF-7 | 14.8 |
| glioma SF-295 | 19.3 | Breast ca.*(pl.ef)MDA-MB-231 | 10.4 |
| Heart (fetal) | 28.5 | Breast ca.*(pl.ef) T47D | 28.7 |
| Heart | 4.8 | Breast ca. BT-549 | 5.3 |
| Skeletal muscle (fetal) | 16.5 | Breast ca. MDA-N | 3.6 |
| Skeletal muscle | 13.9 | Ovary | 39.2 |
| Bone marrow | 2.6 | Ovarian ca. OVCAR-3 | 11.4 |
| Thymus | 16.4 | Ovarian ca. OVCAR-4 | 51.4 |
| Spleen | 23.5 | Ovarian ca. OVCAR-5 | 20.4 |
| Lymph node | 37.4 | Ovarian ca. OVCAR-8 | 12.2 |
| Colorectal | 12.4 | Ovarian ca. IGROV-1 | 12.2 |
| Stomach | 14.6 | Ovarian ca.* (ascites) SK-OV-3 | 15.5 |
| Small intestine | 18.6 | Uterus | 20.3 |
| Colon ca. SW480 | 0.0 | Placenta | 71.2 |
| Colon ca.* SW620(SW480 met) | 1.5 | Prostate | 14.1 |
| Colon ca. HT29 | 0.7 | Prostate ca.* (bone met)PC- | 22.7 |
| Colon ca. HCT-116 | 6.8 | Testis | 8.6 |
| Colon ca. CaCo-2 | 13.7 | Melanoma Hs688(A).T | 11.7 |
| Colon ca. tissue(ODO3866) | 13.5 | Melanoma* (met) Hs688(B).T | 8.8 |
| Colon ca. HCC-2998 | 18.2 | Melanoma UACC-62 | 28.7 |
| Gastric ca.* (liver met) NCI-N87 | 9.7 | Melanoma M14 | 17.4 |
| Bladder | 6.8 | Melanoma LOX IMVI | 2.6 |
| Trachea | 6.3 | Melanoma* (met) SK-MEL-5 | 16.6 |
| Kidney | 28.7 | Adipose | 5.6 |

**Table AOG. Panel 4D**

| **Tissue Name** | **Rel. Exp.(%) Ag2259, Run 150910665** | **Rel. Exp.(%) Ag3332, Run 165725931** | **Tissue Name** | **Rel. Exp.(%) Ag2259, Run 150910665** | **Rel. Exp.(%) Ag3332, Run 165725931** |
|---|---|---|---|---|---|
| Secondary Th1 act | 3.1 | 2.7 | HUVEC IL-1beta | 1.9 | 1.2 |
| Secondary Th2 act | 2.4 | 1.2 | HUVEC IFN gamma | 8.5 | 3.5 |
| Secondary Tr1 act | 6.6 | 6.7 | HUVEC TNF alpha + IFN gamma | 3.5 | 1.5 |
| Secondary Th1 rest | 1.8 | 2.8 | HUVEC TNF alpha+ IL4 | 3.8 | 1.4 |
| Secondary Th2 rest | 4.5 | 4.1 | HUVEC IL-11 | 2.2 | 1.7 |
| Secondary Tr1 rest | 2.5 | 2.9 | Lung Microvascular EC none | 6.8 | 2.5 |
| Primary Th1 act | 1.0 | 0.4 | Lung Microvascular EC TNFalpha + IL-1beta | 4.7 | 2.1 |
| Primary Th2 act | 3.2 | 2.7 | Microvascular Dermal EC none | 3.5 | 2.5 |
| Primary Tri act | 1.2 | 0.9 | Microsvasular Dermal EC TNFalpha + IL. 1beta | 3.0 | 2.1 |
| Primary Th1 rest | 2.0 | 2.0 | Bronchial epithelium TNFalpha+IL1beta | 1.7 | 1.1 |
| Primary Th2 rest | 3.3 | 2.4 | Small airway epithelium none | 1.4 | 0.3 |
| Primary Tr1 rest | 2.0 | 1.3 | Small airway epithelium TNFalpha+IL-1beta | 1.6 | 0.8 |
| CD45RA CD4 lymphocyte act | 2.0 | 1.3 | Coronery artery SMC rest | 2.3 | 1.4 |
| CD45RO CD4 lymphocyte act | 3.3 | 2.4 | Coronery artery SMC TNFalpha+IL-1beta | 2.3 | 1.1 |
| CD8 lymphocyte act | 2.6 | 1.5 | Astrocytes rest | 0.4 | 0.8 |
| Secondary CD8 lymphocyte rest | 6.1 | 6.1 | Astrocytes TNFalpha + IL-1beta | 1.6 | 2.6 |
| Secondary CD8 lymphocyte act | 0.3 | 0.6 | KU-812 (Basophil) rest | 0.0 | 0.0 |
| CD4 lymphocyte none | 5.6 | 5.3 | KU-812 (Basophil) PMA/ionomycin | 0.2 | 0.0 |
| 2ry Th1/Th2/Tr1_anti-CD95 CH11 | 1.9 | 2.5 | CCD1106 (Keratmocytes) none | 0.7 | 0.4 |
| LAK cells rest | 25.5 | 17.2 | CCD1106 (Keratinocytes) TNFalpha+IL-1beta | 1.1 | 3.4 |
| LAK cells IL-2 | 3.8 | 3.9 | Liver cirrhosis | 1.2 | 1.9 |
| LAK cells IL-2+IL-12 | 15.0 | 8.8 | Lupus kidney | 1.1 | 3.3 |
| LAK cells IL-2+IFN gamma | 16.2 | 13.8 | NCI-H292 none | 4.5 | 2.2 |
| LAK cells IL-2+IL-, 18 | 12.6 | 7.9 | NCI-H292 IL-4 | 8.0 | 3.7 |
| LAK cells PMA/ionomycin | 15.7 | 12.7 | NCI-H292 IL-9 | 6.4 | 4.5 |
| NK Cells IL-2 rest | 5.4 | 2.9 | NCI-H292 IL-13 | 4.5 | 2.6 |
| Two Way MLR 3 day | 14.7 | 9.9 | NCI-H292 IFN gamma | 3.3 | 1.4 |
| Two Way MLR 5 day | 9.6 | 7.3 | HPAEC none | 6.4 | 3.7 |
| Two Way MLR 7 day | 2.5 | 1.5 | HPAEC TNF alpha + IL-1 beta | 8.8 | 5.3 |
| PBMC rest | 3.5 | 4.3 | Lung fibroblast none | 6.7 | 10.1 |
| PBMC PWM | 14.3 | 4.1 | Lung fibroblast TNF alpha+IL-1 beta | 3.7 | 9.4 |
| PBMC PHA-L | 2.4 | 0.7 | Lung fibroblast IL-4 | 13.3 | 5.6 |
| Ramos (B cell) none | 0.0 | 0.1 | Lung fibroblast IL-9 | 8.5 | 4.7 |
| Ramos (B cell) ionomycin | 0.2 | 0.0 | Lung fibroblast IL-13 | 8.7 | 4.2 |
| B lymphocytes PWM gamma | 3.7 | 0.7 | Lung fibroblast IFN | 9.0 | 5.1 |
| B lymphocytes CD40L and IL-4 | 10.2 | 3.0 | Dermal fibroblast CCD1070 rest | 4.6 | 2.3 |
| EOL-1 dbcAMP | 4.2 | 2.9 | Dermal fibroblast CCD1070 TNF alpha | 4.2 | 3.1 |
| EOL-1 dbcAMP PMA/ionomycin | 4.3 | 4.5 | Dermal fibroblast CCD1070 IL-1 beta | 3.7 | 1.3 |
| Dendritic cells none | 77.4 | 74.7 | Dermal fibroblast IFN gamma | 3.5 | 1.8 |
| Dendritic cells LPS | 30.4 | 19.3 | Dermal fibroblast IL-4 | 6.7 | 4.1 |
| Dendritic cells anti-CD40 | 95.3 | 100.0 | IBD Colitis 2 | 0.6 | 0.5 |
| Monocytes rest | 7.0 | 3.3 | IBD Crohn's | 0.5 | 0.6 |
| Monocytes LPS | 18.8 | 18.4 | Colon | 4.2 | 11.0 |
| Macrophages rest | 100.0 | 89.5 | Lung | 4.5 | 3.1 |
| Macrophages LPS | 15.2 | 10.2 | Thymus | 13.8 | 6.1 |
| HUVEC none | 3.5 | 2.4 | Kidney | 3.9 | 1.9 |
| HUVEC starved | 7.7 | 6.8 | | | |

**CNS_neurodegeneration_v1.0 Summary:** Ag3332 This panel confirms the expression of the CG57787-01 gene at moderate levels in the brains of an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General screening_panel v1.4 Summary:** Ag3332 Expression of the CG57787-01 gene is highest in renal cell carcinoma cell line TK-10 (CT = 28.3). In addition, levels of expression of this gene are higher in fetal lung (CT=29) and 7 lung cancer cell lines than in the adult lung (CT=33). Thus, expression of this gene could be used to differentiate adult and fetal lung and also as marker to detect the presence of lung cancer. Furthermore, therapeutic modulation of the expression or function of this gene may be effective in the treatment of lung cancer.

In addition, this gene is expressed at moderate levels throughout the central nervous system, including in amygdala, cerebellum, hippocampus, cerebral cortex, substantia nigra, thalamus and spinal cord. Therefore, this gene may play a role in central nervous system disorders such as Alzheimer's disease, Parkinson's disease, epilepsy, multiple sclerosis, schizophrenia and depression.

Among tissues with metabolic or endocrine function, this gene is expressed at moderate levels in pancreas, adrenal gland, pituitary gland, adipose, thyroid, skeletal muscle, heart, and liver. Therefore, therapeutic modulation of the activity of this gene may prove useful in the treatment of endocrine/metabolically related diseases, such as obesity and diabetes.

**Panel 1.3D Summary:** Ag3332 Expression of the CG57787-01 gene is highest in cerebral cortex (CT = 31.2). In addition, this gene is expressed at moderate levels throughout the central nervous system, including in amygdala, cerebellum, hippocampus, cerebral cortex, substantia nigra, thalamus and spinal cord. Therefore, this gene may play a role in central nervous system disorders such as Alzheimer's disease, Parkinson's disease, epilepsy, multiple sclerosis, schizophrenia and depression.

Among tissues with metabolic or endocrine function, this gene is expressed at moderate levels in pancreas, adrenal gland, pituitary gland, adipose, thyroid, skeletal muscle, heart, and liver. Therefore, therapeutic modulation of the activity of this gene may prove useful in the treatment of endocrine/metabolically related diseases, such as obesity and diabetes.

**Panel 4D Summary:** Ag2259/Ag3332 Results from two experiments using different probe/primer sets are in excellent agreement. Expression of the CG57787-01 gene is highest in treated and untreated dendritic cells and macrophages. Dendritic cells and macrophages are powerful antigen-presenting cells (APC) whose function is pivotal in the initiation and maintenance of normal immune responses. Autoimmunity and inflammation may also be reduced by suppresssion of this function. Therefore, therapeutic utilization of the protein encoded by this transcript may be important in the treatment of diseases where antigen presentation, a function of mature dendritic cells, plays an important role, such as asthma, rheumatoid arthrtis, IBD, and psoriasis.

### AR. CG57785-01: Sulfate transporter

Expression of gene CG57785-01 was assessed using the primer-probe set Ag3331, described in Table ARA. Results of the RTQ-PCR runs are shown in Tables ARB, ARC and ARD.

**Table ARA. Probe Name Ag3331**

| **Primers** | **Sequences** | **Length** | **Start Position** | **SEQ ED NO** |
|---|---|---|---|---|
| Forward | 5'-ctcatatttctgggcaagaaga-3' | 22 | 646 | 416 |
| Probe | TET-5'-tgccagtcttcacaattacagtgtca-3'-TAMRA | 26 | 669 | 417 |
| Reverse | 5'-cgatggctattaaatcctggtt-3' | 22 | 700 | 418 |

**Table ARB. CNS_neurodegeneration_v1.0**

| **Tissue Name** | **Rel. Exp.(%) Ag3331, Run 210146363** | **Tissue Name** | **Rel. Exp.(%) Ag3331, Run 210146363** |
|---|---|---|---|
| AD 1 Hippo | 15.5 | Control (Path) 3 Temporal Ctx | 4.5 |
| AD 2 Hippo | 39.2 | Control (Path) 4 Temporal Ctx | 57.0 |
| AD 3 Hippo | 3.3 | AD 1 Occipital Ctx | 24.7 |
| AD 4 Hippo | 6.3 | AD 2 Occipital Ctx (Missing) | 0.0 |
| AD 5 Hippo | 84.7 | AD 3 Occipital Ctx | 0.0 |
| AD 6 Hippo | 40.9 | AD 4 Occipital Ctx | 18.4 |
| Control 2 Hippo | 35.6 | AD 5 Occipital Ctx | 82.4 |
| Control 4 Hippo | 7.3 | AD 6 Occipital Ctx | 30.8 |
| Control (Path) 3 Hippo | 2.0 | Control 1 Occipital Ctx | 3.1 |
| AD 1 Temporal Ctx | 36.9 | Control 2 Occipital Ctx | 38.2 |
| AD 2 Temporal Ctx | 62.4 | Control 3 Occipital Ctx | 33.4 |
| AD 3 Temperal Ctx | 16.0 | Control 4 Occipital Ctx | 2.8 |
| AD 4 Temporal Ctx | 26.4 | Control (Path) 1 Occipital | 93.3 |
| AD 5 Inf Temporal Ctx | 68.3 | Controt(Path)2 Occipital Ctx | 4.4 |
| AD 5 Sup Temporal Ctx | 44.1 | Control (Path) 3 Occipital Ctx | 5.1 |
| AD 6 Inf Temporal Ctx | 68.3 | Control (Path) 4 Occipital Ctx | 22.4 |
| AD 6 Sup Temporal Ctx | 64.6 | Control 1 Parietal Ctx | 7.6 |
| Control 1 Temporal Ctx | 6.2 | Control 2 Parietal Ctx | 48.3 |
| Control 2 Temporal Ctx | 62.9 | Control 3 Parietal Ctx | 41.5 |
| Control 3 Temporal Ctx | 45.4 | Control (Path) 1 Parietal | 84.7 |
| Control 3 Temporal Ctx | 7.1 | Control (Path) 2 Parietal Ctx | 31.2 |
| Control (Path) 1 Temporal Ctx | 100.0 | Control (Path) 3 Parietal Ctx | 1.0 |
| Control (Path) 2 Temporal Ctx | 57.8 | Control (Path) 4 Parietal Ctx | 67.8 |

**Table ARC. General_screening_panel_v1.4**

| **Tissue Name** | R**el. Exp.(%) Ag3331, Run 215678710** | **Tissue Name** | **Rel. Exp.(%) Ag3331, Run 215678710** |
|---|---|---|---|
| Adipose | 0.4 | Renal ca. TK-10 | 0.0 |
| Melanoma* Hs688(A).T | 0.0 | Bladder | 0.3 |
| Melanoma* Hs688(B).T | 0.0 | Gastric ca. (liver met.) NCI-N87 | 0.2 |
| Melanoma* M14 | 0.0 | Gastric ca. KATO III | 0.0 |
| Melanoma*LOXIMVI | 0.0 | Colon ca. SW-948 | 0.0 |
| Melanoma* SK-MEL-5 | 0.0 | Colon ca. SW480 | 0.2 |
| Squamous cell carcinoma SCC-4 | 0.0 | Colon ca.* (SW480 met) SW620 | 0.0 |
| Testis Pool | 100.0 | Colon ca. HT29 | 0.0 |
| Prostate ca.* (bone met) PC-3 | 0.0 | Colon ca. HCT-1l6 | 0.0 |
| Prostate Pool | 0.1 | Colon ca. CaCo-2 | 0.0 |
| Placenta | 0.2 | Colon cancer tissue | 0.2 |
| Uterus Pool | 0.1 | Colon ca. SW1116 | 0.0 |
| Ovarian ca. OVCAR-3 | 0.0 | Colon ca. Colo-205 | 0.0 |
| Ovarian ca. SK-OV-3 | 0.0 | Colon ca. SW-48 | 0.0 |
| Ovarian ca. OVCAR-4 | 0.0 | Colon Pool | 0.0 |
| Ovarian ca. OVCAR-5 | 0.5 | Small Intestine Pool | 0.0 |
| Ovarian ca. IGROV-1 | 0.0 | Stomach Pool | 0.0 |
| Ovarian ca. OVCAR-8 | 0.0 | Bone Marrow Pool | 0.1 |
| Ovary | 0.0 | Fetal Heart | 0.0 |
| Breast ca. MCF-7 | 0.0 | Heart Pool | 0.1 |
| Breast ca. MDA-MB-231 | 0.0 | Lymph Node Poot | 0.3 |
| Breast ca. BT 549 | 0.0 | Fetal Skeletal Muscle | 0.2 |
| Breast ca. T47D | 0.2 | Skeletal Muscle Pool | 0.0 |
| Breast ca. MDA-N | 0.0 | Spleen Pool | 1.3 |
| Breast Pool | 0.0 | Thymus Pool | 0.6 |
| Trachea | 0.6 | CNS cancer (glio/astro) U87-MG | 0.0 |
| Lung | 0.0 | CNS cancer (glio/astro) U-118-MG | 0.0 |
| Fetal Lung | 0.5 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 0.6 | CNS cancer (glio) SNB-19 | 0.0 |
| Lung ca. SHP-77 | 0.0 | CNS cancer (glio) SF-295 | 0.0 |
| Lung ca. A549 | 0.0 | Brain (Amygdala) Pool | 4.0 |
| Lungca_NCI-H526 | 1.2 | Brain (cerebellum) | 12.8 |
| Lung ca. NCI-H23 | 0.0 | Brain (fetal) | 12.4 |
| Lung ca. NCI-H460 | 0.0 | Brain (Hippocampus) Pool | 3.9 |
| Lung ca. HOP-62 | 0.0 | Cerebral Cortex Pool | 5.0 |
| Lung ca. NCI-H522 | 0.1 | Brain (Substantia nigra) Pool | 4.2 |
| Liver | 0.0 | Brain (Thalamus) Pool | 6.9 |
| Fetal Liver | 0.1 | Brain (whole) | 6.7 |
| Liver ca. HepG2 | 0.0 | Spinal Cord Pool | 0.2 |
| Kidney Pool | 0.1 | Adrenal Gland | 0.1 |
| Fetal Kidney | 0.3 | Pituitary gland Pool | 0.0 |
| Renal ca. 786-0 | 0.0 | Salivary Gland | 0.0 |
| Renal ca. A498 | 0.0 | Thyroid (female) | 0.5 |
| Renal ca. ACHN | 0.0 | Pancreatic ca. CAPAN2 | 0.0 |
| Renal ca. UO-31 | 0.0 | Pancreas Pool | 0.1 |

**Table ARD. Panel 4D**

| **Tissue Name** | **Rel. Exp.(%) Ag3331, Run 165084166** | **Tissue Name** | **Rel. Exp.(%) Ag3331, Run 165084166** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha+ IFN gamma | 0.0 |
| Secondary Th1 rest | 0.0 | HUVEC TNF alpha +IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha +IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL1beta | 0.0 |
| Primary Th2 rest | 0.0 | Small airway epithelium none | 0.0 |
| Primary Tr1 rest | 0.0 | Small airway epithelium TNFalpha IL-1beta | 9.3 |
| CD45RA CD4 lymphocyte act | 0.0 | Coronery artery SMC rest | 0.0 |
| CD45RO CD4 lymphocyte act | 0.0 | Coronery artery SMC TNFalpha+ IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1 beta | 0.0 |
| Secondary CD8 lymphocyte act | 0.0 | KU-812 (Basophil) rest | 0.0 |
| CD4 lymphocyte none | 0.0 | KU-812(Basophil) PMA/ionomycin | 9.0 |
| 2ry Th1/Th2/Tr1_anti-CD95 CH11 | 0.0 | CCD1106 (Keratinocytes) none | 0.0 |
| LAK cells rest | 0.0 | CCD1106 (Keratinocytes) TNFalpha+ IL-1beta | 0.0 |
| LAK cells IL-2 | 0.0 | Liver cirrhosis | 0.0 |
| LAK cells IL-2+IL-12 | 0.0 | Lupus kidney | 0.0 |
| LAK cells IL-2+IFN gamma | 0.0 | NCI-H292 none | 0.0 |
| LAK cells IL-2+IL-18 | 0.0 | NCI-H292 IL-4 | 0.0 |
| LAK cells PMA/ionomycin | 0.0 | NCI-H292 IL-9 | 0.0 |
| NK Cells IL-2 rest | 0.0 | NCI-H292 IL-13 | 0.0 |
| Two Way MLR 3 day | 0.0 | iNCI-H292 IFN gamma | 0.0 |
| Two Way MLR 5 day | 0.0 | HPAEC none | 0.0 |
| Two Way MLR 7 day | 0.0 | HPAEC TNF alpha+IL-1 beta | 0.0 |
| PBMC rest | 0.0 | Lung fibroblast none | 0.0 |
| PBMC PWM | 6.7 | Lung fibroblast TNF alpha + IL-1 beta | 10.5 |
| PBMC PHA-L | 9.3 | Lung fibroblast IL-4 | 0.0 |
| Ramos (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 0.0 | Lung fibroblast IFN gamma | 0.0 |
| B lymphocytes CD40L and IL-4 | 0.0 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 0.0 |
| Monocytes rest | 100.0 | IBD Crohn's | 10.4 |
| Monocytes LPS | 9.2 | Colon | 7.6 |
| Macrophages rest | 6.7 | Lung | 0.0 |
| Macrophages LPS | 0.0 | Thymus | 0.0 |
| HUVEC none | 0.0 | Kidney | 0.0 |
| HUVEC starved | 0.0 | | |

**CNS_neurodegeneration_v1.0 Summary:** Ag3331 This panel confirms the expression of this gene at low levels in the brains of an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel_v1.4 Summary:** Ag3331 Expression of the CG57785-01 gene is highest in a sample derived from testis (CT = 28.6). Thus, the expression of this gene could be used to distinguish testis from the other samples in the panel. In addition, therapeutic modulation of the activity of this gene or its protein product, through the use of small molecule drugs, protein therapeutics or antibodies, might be beneficial in the treatment of fertility and hypogonadism.

This gene is also expressed at low to moderate levels throughout the CNS, including in amygdala, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

**Panel 4D Summary:** The CG57785-01 transcript is expressed at detectable levels only in resting monocytes (CT = 34.3). Thus, the expression of this gene could be used to distinguish resting monocytes from the other samples in the panel. In addition, the protein encoded by this transcript may be important in monocytic differentiation and activation. Therefore, regulating the expression of this transcript or the function of the protein it encodes may alter the types and levels of monocytic cells regulated by cytokine and chemolcine production and T cell activation. Therapeutics designed with the protein encoded by this transcript could therefore be important for the treatment of asthma, emphysema, inflammatory bowel disease, arthritis and psoriasis.

### AS. CG57748-01: N-acetylgalactosaminyltransferase

Expression of gene CG57748-01 was assessed using the primer-probe set Ag3325, described in Table ASA.

**Table ASA. Probe Name Ag3325**

| **Primers** | **Sequences** | **Length** | **Start Position** | **SEQ ID NO** |
|---|---|---|---|---|
| Forward | 5'-tggcaccttctaatgttcaaat-3' | 22 | 1942 | 419 |
| Probe | TET-5'-tttccaggtactcagatggtaccctg-3'-TAMRA | 26 | 1969 | 420 |
| Reverse | 5'-cgcgtgttaaatacgtttgaag-3' | 22 | 2018 | 421 |

**CNS_neurodegeneration_v1.0** Summary: Ag3325 Expression of this gene is low/undetectable (CTs > 35) across all of the samples on this panel (data not shown).

**General_screening_panel_v1.4 Summary**: Ag3325 Expression of this gene is low/undetectable (CTs > 35) across all of the samples on this panel (data not shown).

**Panel 4D Summary:** Ag3325 Expression of this gene is low/undetectable (CTs > 35) across all of the samples on this panel due to a probable experimental failure (data not shown).

### AT. CG57693-01: Novel Protein Kinase

Expression of gene CG57693-01 was assessed using the primer-probe set Ag3309, described in Table ATA. Results of the RTQ-PCR runs are shown in Tables ATB, ATC and ATD.

**Table ATA. Probe Name Ag3309**

| **Primers** | **Sequences** | **Length** | **Start Position** | **SEQ ID NO** |
|---|---|---|---|---|
| Forward | 5'-ttctacatggctcctgaagtct-3' | 22 | 1540 | 422 |
| Probe | TET-5'-actacacagccaaggcggacatcttt-3'-TAMRA | 26 | 1571 | 423 |
| Reverse | 5'-tctttctatcattgcccagatg-3' | 22 | 1611 | 424 |

**Table ATB. CNS_neurodegeneration_vl.0**

| **Tissue Name** | **Rel. Exp.(%) Ag3309, Run 210143041** | **Tissue Name** | **Rel. Exp.(%) Ag3309, Run 210143041** |
|---|---|---|---|
| AD 1 Hippo | 9.5 | Control (Path) 3 Temporal Ctx | 3.7 |
| AD 2 Hippo | 21.2 | Control (Path) 4 Temporal Ctx | 26.2 |
| AD 3 Hippo | 4.6 | AD 1 Occipital Ctx | 19.5 |
| AD 4 Hippo | 3.0 | AD 2 Occipital Ctx (Missing) | 0.0 |
| AD 5 hippo | 100.0 | AD 3 Occipital Ctx | 5.4 |
| AD 6 Hippo | 44.4 | AD 4 Occipital Ctx | 12.2 |
| Control 2 Hippo | 18.9 | AD 5 Occipital Ctx | 16.4 |
| Control 4 Hippo | 7.7 | AD 6 Occipital Ctx | 40.6 |
| Control (Path) 3 Hippo | 3.9 | Control 1 Occipital Ctx | 3.5 |
| AD 1 Temporal Ctx | 13.1 | Control 2 Occipital Ctx | 63.3 |
| AD 2 Temporal Ctx | 21.9 | Control 3 Occipital Ctx | 17.2 |
| AD 3 Temporal Ctx | 6.4 | Control 4 Occipital Ctx | 4.2 |
| AD 4 Temporal Ctx | 17.6 | Control (Path) 1 Occipital Ctx | 72.2 |
| AD 5 Inf Temporal Ctx | 83.5 | (Path) 2 Occipital Control (Path) 2 Occipital Ctx | 8.1 |
| AD 5 SupTemporal Ctx | 31.0 | Control (Path) 3 Occipital Ctx | 2.7 |
| AD 6 Inf Temporal Ctx | 46.7 | Control (Path) 4 Occipital | 12.9 |
| AD 6 Sup Temporal Ctx | 43.8 | Control 1 Parietal Ctx | 5.4 |
| Control I Temporal Ctx | 6.3 | Control 2 Parietal Ctx | 39.2 |
| Control 2 Temporal Ctx | 32.5 | Control 3 Parietal Ctx | 11.0 |
| Control 3 Temporal Ctx | 13.3 | Control (Path) 1 Parietal Ctx | 54.7 |
| Control 4 Temporal Ctx | 5.0 | Control (path) 2 Parietal Ctx | 15.5 |
| Control (Path) 1 Temporal Ctx | 46.0 | Control (Path) 3 Parietal Ctx | 2.6 |
| Control (Path) 2 Temporal Ctx | 28.5 | Control (Path) 4 Parietal Ctx | 37.1 |

**Table ATC. General_screening_panel_v1.4**

| **Tissue Name** | **Rel. Exp.(%) Ag3309, Run 215648362** | **Tissue Name** | **Rel. Exp.(%) Ag3309, Run 215648362** |
|---|---|---|---|
| Adipose | 8.1 | Renal ca. TK-10 | 45.4 |
| Melanoma* Hs688(A).T | 19.6 | Bladder | 13.9 |
| Melanoma* Hs688(B).T | 20.9 | Gastric ca. (liver met) NCI-N87 | 73.2 |
| Melanoma*M14 | 35.6 | Gastric ca. KATO in | 100.0 |
| Melanoma* LOXIMVI | 28.9 | Colon ca. SW-948 | 8.4 |
| Melanoma* SK-MEL-5 | 31.4 | Colon ca. SW480 | 63.7 |
| Squamous cell carcinoma SCC-4 | 28.9 | Colon ca.* (SW480 met) SW620 | 52.9 |
| Testis Pool | 19.8 | Colon ca. HT29 | 26.4 |
| Prostate ca.* (bone met) PC-3 | 72.7 | Colon ca.HCT-116 | 61.6 |
| Prostate Pool | 4.9 | Colon ca. CaCo-2 | 47.3 |
| Placenta | 18.0 | Colon cancer tissue | 23.5 |
| Uterus Pool | 2.1 | Colon ca. SW1116 | 8.8 |
| Ovarian ca. OVCAR-3 | 24.1 | Colon ca. Colo-205 | 18.6 |
| Ovarian ca. SK-OV-3 | 40.6 | Colon ca. SW-48 | 19.6 |
| Ovarian ca. OVCAR-4 | 17.7 | Colon Pool | 10.2 |
| Ovarian ca. OVCAR-5 | 43.5 | Small Intestine Pool | 11.8 |
| Ovarian ca. IGROV-1 | 18.3 | Stomach Pool | 9.0 |
| Ovarian ca. OVCAR-8 | 13.7 | Bone Marrow Pool | 5.9 |
| Ovary | 6.5 | Fetal Heart | 7.1 |
| Breast ca. MCF-7 | 31.2 | Heart Pool | 4.7 |
| Breast ca. MDA-MB-231 | 37.6 | Lymph Node Pool | 12.2 |
| Breast ca. BT 549 | 36.6 | Fetal Skeletal Muscle | 3.8 |
| Breast ca. T47D | 68.3 | Skeletal Muscle Pool | 6.6 |
| Breast ca. MDA-N | 14.5 | Spleen Pool | 9.2 |
| Breast Pool | 9.6 | Thymus Pool | 13.6 |
| Trachea | 14.0 | CNS cancer (glio/astro) U87-MG | 16.7 |
| Lung | 2.3 | CNS cancer (glio/astro) U-118-MG | 62.9 |
| Fetal Lung | 21.8 | CNS cancer (neuro;met) SK-N-AS | 30.1 |
| Lung ca. NCI-N417 | 4.9 | CNS cancer (astro) SF-539 | 12.9 |
| Lung ca. LX-1 | 52.5 | CNS cancer (astro) SNB-75 | 50.3 |
| Lung ca. NCI-H146 | 22.5 | CNS cancer (glio) SNB-19 | 16.4 |
| Lung ca. SHP-77 | 39.2 | CNS cancer (glio) SF-295 | 23.2 |
| Lung ca. A549 | 38.7 | Brain (Amygdala) Pool | 9.1 |
| Lung ca. NCI-H526 | 17.1 | Brain (cerebellum) | 23.8 |
| Lung ca. NCI-H23 | 39.2 | Brain (fetal) | 18.7 |
| Lung ca. NCI-H460 | 24.5 | Brain (Hippocampus) Pool | 9.2 |
| Lung ca. HOP-62 | 27.4 | Cerebral Cortex Pool | 11.7 |
| Lung ca. NCI-H522 | 37.9 | Brain (Substantia nigra) Pool | 7.9 |
| Liver | 2.4 | Brain (Thalamus) Pool | 14.0 |
| Fetal liver | 25.3 | Brain (whole) | 16.8 |
| Liver ca. HepG2 | 49.7 | Spinal Cord Pool | 4.9 |
| Kidney Pool | 21.0 | Adrenal Gland | 24.1 |
| Fetal Kidney | 9.5 | Pituitary gland Pool | 3.7 |
| Renal ca. 786-0 | 16.4 | Salivary Gland | 8.8 |
| Renal ca. A498 | 6.0 | Thyroid (female) | 5.7 |
| Renal ca.ACHN | 16.7 | Pancreatic ca. CAPAN2 | 37.4 |
| Renal ca. UO-31 | 25.2 | Pancreas Pool | 14.8 |

**Table ATD Panel 4D**

| **Tissue Name** | **Rel. Exp.(%) Ag3309, Run 164335487** | **Tissue Name** | **Rel. Exp.(%) Ag3309, Run 164335487** |
|---|---|---|---|
| Secondary Th1 act | 24.7 | HUVEC IL-1 beta | 10.7 |
| Secondary Th2 act | 33.7 | HUVEC IFN gamma | 18.7 |
| Secondary Tr1 act | 31.9 | HUVEC TNF alpha + IFN gamma | 13.4 |
| Secondary Th1 rest | 5.8 | HUVEC TNF alpha + IL4 | 16.3 |
| Secondary Th2 rest | 7.9 | HUVEC IL-11 | 11.4 |
| Secondary Tr1 rest | 9.4 | Lung Microvascular EC none | 13.9 |
| Primary Th1 act | 28.3 | Lung Microvascular EC TNFalpha +IL-1beta | 15.7 |
| Primary Th2 act | 28.1 | Microvascular Dermal EC none | 19.3 |
| Primary Tr1 act | 37.9 | Microsvasular Dermal EC TNFalpha+IL-1beta | 16.3 |
| Bronchial epithelium TNFalpha+ Primary Th1 rest | 49.0 | IL1beta | 36.3 |
| Primary Th2 rest | 26.8 | Small airway epithelium none | 9.5 |
| Primary Trl rest | 22.8 | Small airway epithelium TNFalpha +IL-1beta | 64.2 |
| CD45RA CD4 lymphocyte act | 14.9 | Coronery artery SMC rest | 14.9 |
| CD45RO CD4 lymphocyte act | 23.7 | Coronery artery SMC TNFalpha+ IL-1 beta | 8.1 |
| CD8 lymphocyte act | 17.3 | Astrocytes rest | 14.6 |
| Secondary CD8 lymphocyte rest | 21.8 | Astrocytes TNFalpha+IL-1beta | 9.0 |
| Secondary CD8 lymphocyte act | 15.7 | KU-812 (Basophil) rest | 17.8 |
| CD4 lymphocyte none | 5.5 | KU-812 (Basophil) PMA/ionomycin | 53.6 |
| 2ry Th1/Th2/Tr1_anti-CD95 CH11 | 12.2 | CCD1106 (Keratinocytes) none | 48.6 |
| LAK cells rest | 15.3 | CCD1106 (Keratinocytes) TNFalpha + IL-1beta | 25.5 |
| LAK cells IL-2 | 24.7 | Liver cirrhosis | 3.4 |
| LAK cells IL-2+IL-12 | 28.5 | Lupus kidney | 2.3 |
| LAK cells IL-2+IFN gamma | 36.1 | NCI-H292 none | 21.6 |
| LAK cells IL-2+IL-18 | 23.0 | NCI-H292 IL-4 | 31.4 |
| LAK cells PMA/ionomycin | 8.2 | NCI-H292 IL-9 | 24.7 |
| NK Cells IL-2 rest | 20.3 | NCI-H292 IL-13 | 15.2 |
| Two Way MLR 3 day | 18.6 | NCI-H292 IFN gamma | 18.2 |
| Two Way MLR 5 day | 17.9 | HPABC none | 12.3 |
| Two Way MLR 7 day | 11.1 | HP ABC TNF alpha + IL-1 beta | 15.7 |
| PBMC rest | 10.0 | Lung fibroblast none | 10.1 |
| PBMC PWM | 77.4 | Lung fibrolast TFN alpha + IL-1 beta | 11.1 |
| PBMC PHA-L | 32.3 | Lung fibroblast IL-4 | 24.5 |
| Ramos (B cell) none | 26.4 | Lung fibroblast IL-9 | 18.7 |
| Ramos (B cell) ionomycin | 100.0 | Lung fibroblast IL-13 | 17.6 |
| B lymphocytes PWM | 97.3 | Lung fibroblast IFN gamma | 26.4 |
| B lymphocytes CD40L and IL-4 | 53.2 | Dermal fibroblast CCD1070 rest | 40.1 |
| EOL-1 dbcAMP | 15.1 | Dermal fibroblast CCD 1070 TNF alpha | 59.9 |
| EOL-1 dbcAMP PMA/ionomycin | 27.5 | Dermal fibroblast CCD1070 IL-1 beta | 17.1 |
| Dendritic cells none | 9.3 | Dermal fibroblast IFN gamma | 9.5 |
| Dendritic cells LPS | 7.2 | Dermal fibroblast IL-4 fibroblast IL-4 | 18.6 |
| Dendritic cells anti-CD40 | 7.3 | IBD Colitis 2 | 2.0 |
| Monocytes rest | 13.1 | IBD Crohn's | 2.0 |
| Monocytes LPS | 15.7 | Colon | 25.0 |
| Macrophages rest | 12.1 | Lung | 19.9 |
| Macrophages LPS | 9.9 | Thymus | 27.2 |
| HUVEC none | 20.4 | Kidney | 33.2 |
| HUVEC starved | 43.5 | | |

**CNS_neurodegeneration_v1.0 Summary:** Ag3309 This panel confirms the expression of the CG57693-01 gene at moderate levels in the brains of an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**Generat_screening_panel_v1.4** Summary: Ag3309 The CG57693-01 gene is expressed at high to moderate levels in the majority of samples on this panel, with highest expression detected in a gastric cancer cell line (CT = 26.7). This gene encodes a protein with homology to Ser/Thr protein kinases. In general, expression of this gene appears to be higher in cancer cell lines than in the corresponding normal tissues. This overexpression is particularly evident in lung, colon, prostate and gastric cancer cell lines. Therefore, therapeutic modulation of the activity of this gene and it protein product may be of benefit in the treatment of lung, colon, prostate and gastric cancer. Furthermore, expression of this gene is significantly higher in fetal lung than in adult lung, suggesting that its expression may be used to distinguish fetal (CT = 28.9) from adult lung (CT = 32.1).

In addition, this gene is expressed at moderate levels throughout the CNS, including in amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

Among tissues with metabolic or endocrine function, this gene is expressed at moderate levels in pancreas, adipose, adrenal gland, thyroid, pituitary gland, heart, skeletal muscle and liver. Therefore, therapeutic modulation of the activity of this gene may prove useful in the treatment of endocrine/metabolically related diseases, such as obesity and diabetes. Interestingly, expression of this gene is higher in fetal liver (CT = 28.7) than in adult liver (CT = 32.1), suggesting that expression of this gene can be used to distinguish adult from fetal liver.

**Panel 4D Summary:** Ag3309 This transcript is highly expressed in the PMA and ionomycin treated basophil cell line KU-812 (CT=28.2) and to a lesser extent in untreated KU-812 cells (CT=29.8). Therefore, antibody or small molecule therapies designed with the protein encoded for by this gene could block or inhibit inflammation or tissue damage due to basophil activation in response to asthma, allergies, hypersensitivity reactions, psoriasis, and viral infections.

This gene is also moderately expressed in Ramos B cells (CT = 29.3) and the expression is highly stimulated by treatment with ionomycin and PWM (CTs=27). Expression of this transcript in B cells suggests that this gene may be involved in rheumatic disease including rheumatoid arthritis, lupus, osteoarthritis, and hyperproliferative B cell disorders.

In addition, treatment of small airway epithelium with TNFalpha + IL-1beta stimulated the expression of this transcript. Therefore, modulation of the expression or activity of the protein encoded by this transcript through the application of small molecule therapeutics may be useful in the treatment of asthma, COPD, and emphysema.

### NOV54

Expression of NOV54/CG57707-01 was assessed using the primer-probe set Ag3312, described in Table AUA. Results of the RTQ-PCR runs are shown in Tables AUB, AUC and ADD.

**Table AUA. Probe Name Ag3312**

| **Primers** | **Sequences** | **Length** | **Start Position** | **SEQ ID NO** |
|---|---|---|---|---|
| Forward | 5'-gaccagctttgactatgaatgc-3' | 22 | 624 | 425 |
| Probe | TET-5'-tgttcatcagactttaccctaccagtcgg-3'-TAMRA | 29 | 663 | 426 |
| Reverse. | 5'-ggagttgaacgtatccactgaa-3' | 22 | 693 | 427 |

**Table AUB. CNS neurodegenerarion v1.0**

| **Tissue Name** | **Rel. Exp.(%) Ag3312, Run 210143316** | **Tissue Name** | **Rel. Exp.(%) Ag3312, Run 210143316** |
|---|---|---|---|
| AD 1 Hippo | 2.1 | Control (Path) 3 Temporal Ctx | 1.2 |
| AD 2 Hippo | 7.7 | Control (Path) 4 Temporal Ctx | 12.5 |
| AD 3 Hippo | 0.9 | AD 1 Occipital Ctx | 10.9 |
| AD 4 Hippo | 2.8 | AD 2 Occipital Ctx (Missing) | 0.0 |
| AD 5 Hippo | 100.0 | AD 3 Occipital Ctx | 2.2 |
| AD 6 Hippo | 10.3 | AD 4 Occipital Ctx | 6.6 |
| Control 2 Hipp | 17.1 | AD 5 Occipital Ctx | 54.7 |
| Control 4 Hippo | 3.1 | AD 6 Occipital Ctx | 12.9 |
| Control (Path) 3 Hippo | 2.5 | Control 1 Occipital Ctx | 5.2 |
| AD 1 Temporal Ctx | 5.1 | Control 2 Occiptal Ctx | 84.7 |
| AD 2 Temporal Ctx | 6.7 | Control 3 Occipital Ctx | 6.9 |
| AD 3 Temporal Ctx | 1.2 | Control 4 Occipital Ctx | 0.9 |
| AD 4 Teinporal Ctx | 5.3 | Control (Path) 1 Occipital Ctx | 66.0 |
| AD 5 Inf Temporal Ctx | 52.9 | Control (Path)2 Occipital | 4.1 |
| AD 5 Sup Temporal Ctx | 14.0 | Control (Path) 3 Occipital | 3.7 |
| AD 6 Inf Temporal Ctx | 7.4 | Control (Path) 4 Occipital Ctx | 13.6 |
| AD 6 Sup Temporal Ctx | 8.1 | Control 1 Parietal Ctx | 3.0 |
| Control 1 Temporal Ctx | 5.6 | Control 2 Parietal Ctx | 7.9 |
| Control 2 Temporal Ctx | 27.0 | Control 3 Parietal Ctx | 16.8 |
| Control 3 Temporal Ctx | 8.6 | Control (Path) 1 Parietal Ctx | 79.0 |
| Control 3 Temporal Ctx | 1.4 | Control (Path) 2 Parietal Ctx | 11.8 |
| Control (Path) 1 Temporal Ctx | 25.7 | Control (Path) 3 Parietal Ctx | 4.6 |
| Control (Path) 2 Temporal Ctx | 21.8 | Control (Path) 4 Parietal Ctx | 34.9 |

**Table AUC. General_screening_panel_v1.4**

| **Tissue Name** | **Rel. Exp.(%) Ag3312, Run 215648085** | **Tissue Name** | **Rel. Exp.(%) Ag3312, Run 215648085** |
|---|---|---|---|
| Adipose | 11.8 | Renal ca. TK-10 | 38.2 |
| Melanoma* Hs688(A).T | 0.0 | Bladder | 8.5 |
| Melanoma* Hs688(B).T | 0.1 | Gastric ca.(liver met.)NCI-N87 | 2.8 |
| Melanoma* M14 | 0.0 | Gastric ca. KATO III | 0.0 |
| Melanoma*LOXIMVI | 0.3 | Colon ca. SW-948 | 0.0 |
| Melanoma* SK-MEL-5 | 3.6 | Colon ca. SW480 | 0.2 |
| Squamous cell carcinoma SCC-4 | 0.5 | Colon ca.* (SW480 met) SW620 | 0.4 |
| Testis Pool | 9.0 | Colon ca. HT29 | 0.0 |
| Prostate ca.* (bone met) PC-3 | 0.0 | Colon ca. HCT-116 | 0.0 |
| Prostate Pool | 2.0 | Colon ca. CaCo-2 | 11.6 |
| Placenta | 9.5 | Colon cancer tissue | 21.9 |
| Uterus Pool | 19.2 | Colon ca.SW1116 | 0.0 |
| Ovarian ca.OVCAR-3 | 29.3 | Colon ca. Colo-205 | 0.0 |
| Ovarian ca. SK-OV-3 | 8.4 | Colon ca. SW-48 | 0.0 |
| Ovarian ca. OVCAR-4 | 4.3. | Colon Pool | 59.9 |
| Ovarian ca. OVCAR-5 | 5.0 | Small Intestine Pool | 26.6 |
| Ovarian ca. IGROV-1 | 34.6 | Stomach Pool | 35.6 |
| Ovarian ca. OVCAR-8 | 2.2 | Bone Marrow Pool | 20.7 |
| Ovary | 8.9 | Fetal Heart | 18.6 |
| Breast ca. MCF-7 | 0.5 | Heart Pool | 20.7 |
| Breast ca. MDA-MB-231 | 0.0 | Lymph Node Pool | 55.1 |
| Breast ca. BT 549 | 18.4 | Fetal Skeletal Muscle | 8.2 |
| Breast ca. T47D | 4.3 | Skeletal Muscle Pool | 4.6 |
| Breast ca. MDA-N | 0.0 | Spleen Pool | 2.6 |
| Breast Pool | 36.9 | Thymus Pool | 16.6 |
| Trachea | 7.2 | CNS cancer (glio/astro) U87-MG | -2.4 |
| Lung | 10.5 | CNS cancer (glio/astro) U-118-MG | 3.6 |
| Fetal Lung | 36.6 | CNS cancer (neuro;met) SK-N-AS | 6.9 |
| Lung ca. NCI-N417 | 11.0 | CNS cancer (astro) SF-539 | 0.9 |
| Lung ca.LX-1 | 0.0 | CNS cancer (astro) SNB-75 | 6.0 |
| Lung ca. NCI-H146 | 48.6 | CNS cancer (glio) SNB-19 | 41.5 |
| Lung ca. SHP-77 | 2.1 | CNS cancer (glio) SF-295 | 1.0 |
| Lung ca. A549 | 0.0 | .Brain (Amygdala) Pool | 30.1 |
| Lung ca. NCI-H526 | 3.2 | Brain (cerebellum) | 100.0 |
| Lung ca. NCI-H23 | 10.4 | Brain (fetal) | 33.2 |
| Lung ca. NCI-H460 | 2.0 | Brain (Hippocampus) Pool | 31.9 |
| Lung ca. HOP-62 | 0.0 | Cerebral Cortex Pool | 51.1 |
| Lung ca. NCI-H522 | 3.1 | Brain (Substantia nigra) Pool | 50.7 |
| Liver | 0.7 | Brain (Thalamus) Pool | 43.2 |
| Fetal Liver | 3.0 | Brain (whole) | 55.1 |
| Liver ca. HepG2 | 0.0 | Spinal Cord Pool | 23.3 |
| Kidney Pool | 67.4 | Adrenal Gland | 8.8 |
| Fetal Kidney | 83.5 | Pituitary gland Pool | 0.0 |
| Renal ca. 786-0 | 0.0 | Salivary Gland | 1.8 |
| Renal ca. A498 | 2.0 | Thyroid (female) | 5.6 |
| Renal ca. ACHN | 0.2 | Pancreatic ca. CAPAN2 | 0.0 |
| Renal ca. UO-31 | 0.0 | Pancreas Pool | 35.6 |

**Table AUD. Panel 4D**

| **Tissue Name** | **Rel. Exp.(%) Ag3312, Run 164682846** | **Tissue Name** | **Rel. Exp.(%) Ag3312, Run 164682846** |
|---|---|---|---|
| Secondary Th1 act | 0.0 | HUVEC IL-1beta | 0.0 |
| Secondary Th2 act | 0.0 | HUVEC IFN gamma | 0.9 |
| SecondaryTr1 act | 0.0 | HUVEC TNF alpha+ IFN gamma | 0.0 |
| Secondary Th1 rest | 0.0 | HUVEC TNF alpha+IL4 | 0.0 |
| Secondary Th2 rest | 0.0 | HLTVEC IL-11 | 0.0 |
| Secondary Tr1 rest | 0.0 | Lung Microvascular EC none | 0.0 |
| PrimaryTh1 act Primary Th1 act | 0.0 | Lung Microvascular EC TNFalpha + IL-1beta | 0.4 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha+ IL1beta | 0.0 |
| Primary Th2 rest | 0.0 | Small airway epithelium none | 0.0 |
| Primary Tr1 rest | 0.0 | Small airway epithelium TNFalpha + IL-1beta | 0.0 |
| CD45RA CD4 lymphocyte | 0.0 | Coronery artery SMC rest | 0.0 |
| CD45RO CD4 lymphocyte act | 0.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1beta | 0.0 |
| Secondary CD8 lymphocyte act | 0.0 | KU-812(Basophil)rest | 0.0 |
| CD4 lymphocyte none | 0.0 | KU-812(Basophil) PMA/ionomycin | 1.7 |
| 2ry Th1/Th2/Tr1_anti-CD95 CH11 | 0.0 | CCD1106 (Keratinocytes) none | 0.0 |
| LAK cells rest LAK cells rest | 0.0 | CCD1106 (Keratinocytes) TNF alpha + IL-1beta | 0.6 |
| LAK cells IL-2 | 0.0 | Liver cirrhosis | 2.2 |
| LAK cells IL-2+IL-12 | 2.2 | Lupus kidney | 3.0 |
| LAK cells IL-2+IFN gamma | 0.0 | NCI-H292 none | 13.0 |
| LAK cells IL-2+ IL-18 | 0.0 | NCI-H292 IL-4 | 12.2 |
| LAK cells PMA/ionomycin | 0.0 | NCI-H292 IL-9 | 24.7 |
| NK Cells IL-2 rest | 0.0 | NCI-H292 IL-13 | 8.9 |
| Two Way MLR 3 day | 0.0 | NCI-H292 IFN gamma | 5.4 |
| Two Way MLR 5 day | 0.0 | HPAEC none | 0.0 |
| Two Way MLR 7 day | 0.0 | HPAEC TNF alpha + IL-1 beta | 0.8 |
| PBMCrest | 0.0 | Lung fibroblast none Lung fibrobtast none | 0.0 |
| PBMC PWM | 0.0 | Lung fibroblast TNF alpha+IL-1 beta | 0.0 |
| PBMC PHA-L | 0.0 | Lung fibroblast IL-4 | 0.8 |
| Ramos (B cell) none | 0.0 | Lung fibroblast IL-9 | 0.8 |
| Ramos (B cell) ionomycin | 0.0 | Lung fibroblast IL-13 | 0.7 |
| B lymphocytes PWM | 1.3 | Lung fibroblast IFN gamma | 3.0 |
| B lymphocytes CD40L and IL-4 | 0.9 | Dermal fibroblast CCD1070 rest | 2.2 |
| EOL-1 dbcAMP | 0.0 | Dermal fibroblast CCD1070 TNF alpha | 2.8 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 8.1 |
| Dendritic cells LPS | 0.0 | Dermal fibroblast IL-4 | 0.0 |
| Dendritic cells anti-CD40 | 0.0 | IBD Colitis2 | 11.0 |
| Monocytes rest | 0.0 | IBD Crohn's | 16.4 |
| Monocytes LPS | 0.0 | Colon | 100.0 |
| Macrophages rest | 8.2 | Lung | 69.3 |
| Macrophages LPS | 1.0 | Thymus | 47.3 |
| HUVEC none | 0.0 | Kidney | 23.7 |
| HUVEC starved | 0.0 | | |

**CNS_neurodegeneration_v1.0 Summary:** Ag3312 This panel confirms the expression of CG57707-01 gene at low to moderate levels in the brains of an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel_v1.4 Summary:** Ag3312 Expression of the CG57707-01 gene is highest in the cerebellum (CT = 27.6). This gene is also expressed at moderate levels in all other regions of the central nervous system examined, including amygdala, hippocampus, substantia nigra, thalamus, cerebral cortex and spinal cord. The CG57707-01 gene encodes a protein with homology to the leucine-rich glioma-activated gene (LGI1). Recently, mutations in the LGI1 gene have been shown to cause autosomal-dominant partial epilepsy with auditory features (ref. 1). Based upon its homology to the LGI1 gene and significant exprssion in the brain, the CG57707-01 gene may also play a role in epilepsy or other central nervous system disorders such as Alzheimer's disease, Parkinson's disease, multiple sclerosis, schizophrenia and depression.

Strikingly, expression of this gene is primarily associated with the normal tissues on this panel. CG57707-01 gene expression appears to be down-regulated in pancreatic, colon, gastric, renal, lung, breast and prostate cancer cell lines as well as in most astrocytoma and glioma cell lines when compared to their respective normal tissues. This observation is consistent with what is known about the LGI1 gene, which was originally identified on the basis of its downregulation in malignant brain tumors (Rcf.1,2). Therefore, therapeutic modulation of the activity of the CG57707-01 gene or its protein product, using small molecule drugs, antibodies or protein therapeutics, may be of benefit in the treatment of pancreatic, colon, gastric, renal, lung, breast, prostate, and CNS cancers.

Among tissues with metabolic or endocrine function, this gene is expressed at moderate levels in pancreas, adipose, adrenal gland, thyroid, skeletal muscle and heart. Therefore, therapeutic modulation of the activity of this gene may prove useful in the treatment of endocrine/metabolically related diseases, such as obesity and diabetes.

### References:

### 1. Kalachikov S, Evgrafov O, Ross B, Winawer M, Barker Cummings C, Boneschi FM, Choi C, Morozov P, Das K, Teplitskaya E, Yu A, Cayanis E, Penchaszadeh G, Kottmann AH, Pedley TA, Hauser WA, Ottman R, Gilliam TC. Mutations in LGI1 cause autosomal-dominant partial epilepsy with auditory features. Nat Genet 2002 Jan 28; [epub ahead of print]

The epilepsies are a common, clinically heterogeneous group of disorders defined by recurrent unprovoked seizures. Here we describe identification of the causative gene in autosomal-dominant partial epilepsy with auditory features (ADPEAF, MIM 600512), a rare form of idiopathic lateral temporal lobe epilepsy characterized by partial seizures with auditory disturbances. We constructed a complete, 4.2-Mb physical map across the genetically implicated disease gene region, identified 28 putative genes (Fig. 1) and resequenced all or part of 21 genes before identifying presumptive mutations in one copy of the leucine-rich, glioma-inactivated 1 gene (LGI1) in each of five families with ADPEAF. Previous studies have indicated that loss of both copies of LGI1 promotes glial tumor progression. We show that the expression pattern of mouse Lgil is predominantly neuronal and is consistent with the anatomic regions involved in temporal lobe epilepsy. Discovery of LGI1 as a cause of ADPEAF suggests new avenues for research on pathogenic mechanisms of idiopathic epilepsies.

### PMID: 11810107

### 2. Chernova OB, Somerville RP, Cowell JK. A novel gene, LGI1, from 10q24 is rearranged and downregulated in malignant brain tumors. Oncogene 1998 Dec 3;17(22):2873-81

Loss of heterozygosity for 10q23-26 is seen in over 80% of glioblastoma multiforme tumors. We have used a positional cloning strategy to isolate a novel gene, LGI1 (Leucine rich gene-Glioma Inactivated), which is rearranged as a result of the t(10;19)(q24;q13) balanced translocation in the T98G glioblastoma cell line lacking any normal chromosome 10. Rearrangement of the LGI1 gene was also detected in the A172 glioblastoma cell line and several glioblastoma tumors. These rearrangements lead to a complete absence of LGIl expression in glioblastoma cells. The LGII gene encodes a protein with a calculated molecular mass of 60 kD and contains 3.5 leucine-rich repeats (LRR) with conserved flanking sequences. In the LRR domain, LGI1 has the highest homology with a number of transmembrane and extracellular proteins which function as receptors and adhesion proteins. LGI1 is predominantly expressed in neural tissues, especially in brain; its expression is reduced in low grade brain tumors and it is significantly reduced or absent in malignant gliomas. Its localization to the 10q24 region, and rearrangements or inactivation in malignant brain tumors, suggest that LGI1 is a candidate tumor suppressor gene involved in progression of glial tumors.

### PMID: 9879993

**Panel 4D Summary:** Ag3312 The CG57707-01 gene is moderately expressed in samples derived from normal colon (CT=29.9), lung (CT=30.4), thymus (CT=30.9) and kidney (CT=31.9). Thus, the expression of this gene could be used to distinguish these tissues from the other samples in this panel. Expression of this gene in normal tissues is consistent with what is observed in General_screening_{_}panel_v1.4.

Furthermore, expression of this gene is decreased in colon samples from patients with IBD colitis (CT=33) and Crohn's disease (CT=32.5) relative to normal colon. Therefore, therapeutic modulation of the activity of the protein encoded by this gene may be useful in the treatment of inflammatory bowel disease.

### NOV55

Expression of NOV55/CG57306-01 was assessed using the primer-probe set Ag3157, described in Table AVA. Results of the RTQ-PCR runs are shown in Tables AVB, and AVC.

**Table AVA. Probe Name Ag3157**

| **Primers** | **Sequences** | **Length** | **Start Position** |
|---|---|---|---|
| Forward | 5'-tgtcatacagtcccagacattg-3' | 22 | 1766 |
| Probe | TET-5'-ccttcttctcccttctcctctcttcctt-3'-TAMRA | 26 | 1788 |
| Reverse | 5'-ggctggtctttacacacttgag-3' | 22 | 1838 |

**Table AVB. Panel 1.3D**

| **Tissue Name** | **Rel. Exp.(%) Ag3157, Run 167994581** | **Tissue Name** | **Rel. Exp.(%) Ag3157, Run 167994581** |
|---|---|---|---|
| Liver adenocarcinoma | 0.0 | Kidney (fetal) | 48.6 |
| Pancreas | 0.2 | Renal ca. 786-0 | 0.0 |
| Pancreatic ca CAPAN 2 | 1.1 | Renal ca. A498 | 0.2 |
| Adrenal gland | 0.0 | Renal ca.RXF 393 | 0.5 |
| Thyroid | 0.6 | Renal ca. ACHN | 0.0 |
| Salivary gland | 1.2 | Renal ca. UO-31 | 0.2 |
| Pituitary gland | 0.2 | Renal ca.TK-10 | 0.0 |
| Brain (fetal) | 2.9 | Liver | 0.2 |
| Brain (whole) | 5.5 | Liver (fetal) | 0.2 |
| Brain (amygdala) | 2.0 | Liver ca. (hepatoblast) HepG2 ca. | 0.0 |
| Brain (cerebellum) | 2.0 | Lung | 0.8 |
| Brain (hippocampus) | 1.5 | Lung (fetal) | 0.0 |
| Brain (substantia nigra) | 3.5 | Lung ca. (small cell) LX-1 | 0.8 |
| Brain (thalamus) Brain (thalamus) | 1.6 | Lung ca. (small cell) NCI-H69 | 0.0 |
| Cerebral Cortex | 1.6 | Lung ca. (s.cell var.) SHP-77 | 0.9 |
| Spinal cord | 1.7 | Lung ca. (large cell)NCI-H460 | 0.0 |
| glio/astro U87-MG | 0.5 | Lung ca. (non-sm cell) A549 | 1.0 |
| glio/astro U-118-MG | 0.0 | Lung ca. (non-s.cell) NCI-H23 | 1.4 |
| astrocytoma SW1783 | 0.2 | Lung ca. (non-s.cell) HOP-62 | 0.2 |
| neuro*; met SK-N-AS | 0.5 | Lung ca. (non-s.cl) NCI-H522 | 1.2 |
| astrocytoma SF-539 | 0.5 | Lung ca.(squam.)SW 900 | 0.1 |
| astrocytoma SNB-75 | 0.4 | Lung ca. (squam.) NCI-H596 | 0.0 |
| glioma SNB-19 | 0.0 | Mammary gland | 0.0 |
| glioma U251 | 0.3 | Breast ca.*(pl.ef) MCF-7 | 1.0 |
| glioma SF-295 | 0.2 | Breast ca.*(pl.ef) MDA-MB-231 | 0.2 |
| Heart (fetal) | 0.4 | Breast ca.*(pl.ef) T47D | 1.8 |
| Heart | 0.2 | Breast ca. BT-549 | 0.8 |
| Skeletal muscle (fetal) | 0.6 | Breast ca. MDA-N | 0.4 |
| Skeletal muscle | 0.0 | Ovary | 0.0 |
| Bone marrow | 0.2 | Ovarian ca. OVCAR-3 | 0.4 |
| Thymus | 0.2 | Ovarian ca. OVCAR-4 | 0.0 |
| Spleen | 0.0 | Ovarian ca. OVCAR-5 | 2.7 |
| Lymph node | 0.0 | Ovarian ca. OVCAR-8 | 0.2 |
| Colorectal | 0.5 | Ovarian ca. IGROV-1 | 0.0 |
| Stomach | 0.0 | Ovarian ca.* (ascites) SK-OV-3 | 0.9 |
| Small intestine | 0.0 | Uterus | 0.0 |
| Colon ca. SW480 | 0.2 | Placenta | 0.0 |
| Colon ca. * SW620(SW480 met) | 1.6 | Prostate | 0.5 |
| Colon ca. HT29 | 0.2 | Prostate ca.* (bone met)PC-3 | 0.9 |
| Colonca.HCT-116 | 1.3 | Testis | 1.3 |
| Colon ca. CaCo-2 | 0.5 | Melanoma Hs688(A).T | 0.0 |
| Colon ca. tissue(ODO3866) | 0.0 | Melanoma*(met) Hs688(B).T | 0.2 |
| Colon ca.HCC-2998 | 2.6 | Melanoma LTACC-62 | 0.0 |
| Gastric ca.* (liver met) NCI-N87 | 0.3 | Melanoma M14 | 0.3 |
| Bladder | 0.5 | Melanoma LOX IMVI | 0.0 |
| Trachea | 0.0 | Melanoma* (met) SK-MEL-5 | 1.5 |
| Kidney | 100.0 | Adipose | 0.4 |

**Table AVC. Panel 4D**

| **Tissue Name** | **Rel. Exp.(%) Ag3157, Run 164529941** | **Tissue Name** | **Rel. Exp.(%) Ag3157, Run 164529941** |
|---|---|---|---|
| Secondary Th1 act | 0.2 | HUVEC IL-1beta | 0.0 |
| Secondary Th2 act | 0.3 | HUVEC IFN gamma | 0.1 |
| Secondary Tr1 act | 1.1 | HUVEC TNF alpha + IFN gamma | 0.4 |
| Secondary Th1 rest | 0.7 | HUVEC TNF alpha + IL4 | 0.1 |
| Secondary Th2 rest | 0.4 | HUVEC IL-11 | 0.4 |
| Secondary Tr1 rest | 0.6 | Lung Microvascular EC none | 0.2 |
| Primary Th1 act | 0.4 | Lung Microvascular EC TNFalpha + IL-1beta | 0.3 |
| Primary Th2 act | 0.2 | Microvascular Dermal EC none | 0.1 |
| Primary Tr1 act | 0.8 | Microsvasular Dermal EC TNFalpha + IL-1beta | 0.0 |
| Primary Th11 rest | 1.1 | Bronchial epithelium TNFalpha + IL1beta | 0.3 |
| Primary Th2 rest | 1.1 | Small airway epithelium none | 0.4 |
| Primary Tr1 rest | 0.2 | Small airway epithelium TNFalpha + IL-1beta | 0.2 |
| CD45RA CD4 lymphocyte act | 0.3 | Coronery artery SMC rest | 0.0 |
| CD45RO CD4 lymphocyte act | 0.4 | Coronery artery SMC TNFalpha + IL-1beta | 0.1 |
| CD8 lymphocyte act | 0.0 | Astrocytes rest | 0.1 |
| Secondary CD8 lymphocyte rest | 0.2 | Astrocytes TNFalpha+IL-1beta | 0.2 |
| Secondary CD8 lymphocyte act | 0.3 | KU-812(Basophil)rest | 0.0 |
| CD4 lymphocyte none | 0.2 | KU-812 (Basophil) PMA/ionomycin | 0.5 |
| 2ry Th1/Th2/Tr1_anti-CD95 CH11 | 1.4 | CCD1106 (Keratinocytes) none | 0.1 |
| LAK cells rest | 0.1 | CCD1106 (Keratinosytes) TNFalpha + IL-1beta | 0.2 |
| LAK cells IL-2 | 0.9 | Liver cirrhosis | 1.0 |
| LAK cells IL-2+IL-12 | 0.7 | Lupus kidney | 4.4 |
| LAK cells IL-2+IFN gamma | 0.4 | NCI-H292 none | 0.7 |
| LAK cells IL-2+ IL-18 | 1.3 | NCI-H292 IL-4 | 0.5 |
| LAK cells PMA/ionomycin | 0.0 | NCI-H292 IL-9 | 1.2 |
| NK Cells IL-2 rest | 0.3 | NCI-H292 IL-13 | 0.1 |
| Two Way MLR 3 day | 0.8 | NCI-H292 IFN gamma | 0.2 |
| Two Way MLR 5 day | 0.1 | HPAEC none | 0.2 |
| Two Way MLR 7 day | 0.3 | HPAEC TNF alpha + IL-1 beta | 0.0 |
| PBMC rest | 0.0 | Lung fibroblast none | 0.0 |
| PBMC PWM | 1.7 | Lung fibroblast TNF alpha + IL-1 beta | 0.0 |
| PBMC PHA-L | 0.3 | Lung fibroblast IL-4 | 0.3 |
| Ramos (B cell) none | 0.9 | Lung fibroblast IL-9 | 0.1 |
| Ramos (B cell) ionomycin | 5.4 | Lung fibroblast IL-13 | 0.2 |
| B lymphocytes PWM | 1.1 | Lung fibroblast IFN gamma | 0.1 |
| B lymphocytes CD40L and IL-4 | 2.2 | Dermal fibroblast CCD1070 rest | 0.0 |
| EOL-1 dbcAMP | 0.0 | Dermal fibroblast CCD 1070 TNF alpha alpha | 1.3 |
| EOL-1 dbcAMP PMA/ionomycin | 0.5 | Dermal fibroblast CCD1070 IL-1 beta | 0.0 |
| Dendritic cells none | 0.0 | Dermal fibroblast IFN gamma | 0.1 |
| Dendritic cells LPS | 0.2 | Dermal fibroblast IL-4 | 0.2 |
| Dendritic cells anti-CD40 | 0.1 | IBD Colitis 2 | 0.0 |
| Monocytes rest | 0.0 | IBD Crohn's | 0.0 |
| Monocytes LPS | 0.7 | Colon | 0.6 |
| Macrophages rest | 0.1 | Lung | 1.1 |
| Macrophages LPS | 0.0 | Thymus | 100.0 |
| HUVEC none | 0.1 | Kidney | 0.3 |
| HUVEC starved | 1.0 | | |

**Panel 1.3D Summary:** Ag3157 Expression of the CG57306-01 gene is highest in samples derived from normal kidney (CT = 29.7) and fetal kidney (CT=30.8). Thus, the expression of this gene could be used to distinguish kidney from the other samples in the panel.

The CG57306-01 gene encodes a variant of anion exchanger AE4, which is expressed primarily in the kidney and is predicted to play a role in sodium bicarbonate exchange (ref. 1). Mutations in sodium bicarbonate transporters have been shown to be associated with renal tubular acidosis (RTA) (ref. 2-3). Thus, therapies designed with the protein encoded for by this gene may potentially play a role in the identification and treatment of RTA or other kidney related diseases.

This transcript is also expressed at low levels in samples derived from brain, including whole adult brain (CT=33.9) and substantia nigra (CT=34.5). Therefore, this gene may play a role in central nervous system disorders such as Alzheimer's disease, Parkinson's disease, epilepsy, multiple sclerosis, schizophrenia and depression.

### References:

### 1. Parker MD, Ourmozdi EP, Tanner MJ. Human BTR1, a new bicarbonate transporter superfamily member and human AE4 from kidney. Biochem Biophys Res Commun 2001 Apr 20;282(5):1103-9

We report the cloning, characterization, and chromosomal localization of two novel human members of the bicarbonate transporter superfamily, BTR1 (Bicarbonate Transporter Related protein-1) and AE4 (Anion Exchange protein 4). BTR1 is a novel mammalian protein. The BTR1 gene maps to chromosome 20p12 and encodes a 100 kDa protein predominantly expressed in the kidney, salivary glands, testis, thyroid glands, and trachea. The AE4 gene maps to chromosome 5q23-31 and encodes a 104 kDa protein expressed mainly in the kidney. Human AE4 shares 84% identity with the recently reported rabbit AE4, a sodium independent, C1(-)/HCO(-)(3) exchanger located on the apical membrane of beta-intercalated kidney cells. Copyright 2001 Academic Press.

### PMID: 11302728

### 2. Soleimani M, Burnham CE. Physiologic and molecular aspects of the Na+:HCO3-cotransporter in health and disease processes. Kidney Int 2000 Feb;57(2):371 -84

Approximately 80% of the filtered load of HC03- is reabsorbed in the proximal tubule via a process of active acid secretion by the luminal membrane. The major mechanism for the transport of HC03- across the basolateral membrane is via the electrogenic Na+:3HCO3-cotransporter (NBC). Recent molecular cloning experiments have identified the existence of three NBC isoforms (NBC-1, NBC-2, and NBC-3).1 Functional and molecular studies indicate the presence of all three NBC isoforms in the kidney. All are presumed to mediate the cotransport of Na+ and HC03- under normal conditions and may be functionally altered in certain pathophysiologic states. Specifically, NBC-1 may be up regulated in metabolic acidosis and potassium depletion and in response to glucocorticoid excess and may be down regulated in response to HCO3- loading or alkalosis. Recent studies provide molecular evidence indicating the expression of NBC-1 in pancreatic duct cells. NBC is activated by cystic fibrosis transmembrane conductance regulator (CFTR) and plays an important role in HCO3- secretion in the agonist-stimulated state in pancreatic duct cells. The purpose of this review is to summarize recent functional and molecular studies on the regulation of NBCs in physiologic and pathophysiologic states. Possible signals responsible for the regulation of NBCs in these conditions are examined. Furthermore, the possible role of this transporter in acid-base disorders (such as proximal renal tubular acidosis) is discussed.

### PMID: 10652014

### 3. Rodriguez-Soriano J., 2000, New insights into the pathogenesis of renal tubular acidosis-from functional to molecular studies. Pediatr Nephrol Oct;14(12): 1121-36.

The diagnosis and classification of renal tubular acidosis (RTA) have traditionally been made on the basis of functional studies. On these grounds, RTA has been separated into three main categories: (1) proximal RTA, or type 2; (2) distal RTA, or type 1; and (3) hyperkalemic RTA, or type 4. In recent years significant advances have been made in our understanding of the subcellular mechanisms involved in renal bicarbonate (HCO3-) and H+ transport. Application of molecular biology techniques has also opened a completely new perspective to the understanding of the pathophysiology of inherited cases of RTA. Mutations in the gene SLC4A4, encoding Na+-HCO3- cotransporter (NBC-1), have been found in proximal RTA with ocular abnormalities; in the gene SLC4A1, encoding Cl(-)-HC03- exchanger (AE1), in autosomal dominant distal RTA; in the gene ATP6B1, encoding B1 subunit of H+-ATPase, in autosomal recessive distal RTA with sensorineural deafness; and in the gene CA2, encoding carbonic anhydrase II, in autosomal recessive osteopetrosis. Syndromes of aldosterone resistance have been also characterized molecularly and mutations in the gene MLR, encoding mineralocorticoid receptor, and in the genes SNCC1A, SNCC1B, and SCNN1G, encoding subunits of the epithelial Na+ channel, have been found in dominant and recessive forms of pseudohypoaldosteronism type 1, respectively. It can be concluded that, although functional studies are still necessary, a new molecular era in the understanding of disorders of renal acidification has arrived.

### PMID: 11045400

**Panel 4D Summary:** Ag3157 Expression of the CG57306-01 gene is highest in thymus (CT = 27.9). Therefore, expression of this gene can be used to distinguish thymus from the other samples on this panel. The putative anion exchanger encoded for by this gene could therefore play an important role in T cell development. Furthermore, small molecule drugs or antibody therapeutics designed against the protein encoded for by this gene could be utilized to modulate immune function (T cell development) and be important for organ transplant, AIDS treatment or post chemotherapy immune reconstitiution.

**Panel 5 Islet Summary:** Ag3157 Expression of this gene is low/undetectable (CTs > 35) across all of the samples on this panel (data not shown).

### NOV56

Expression of NOV56/CG57348-01 was assessed using the primer-probe set Ag3763, described in Table AWA. Results of the RTQ-PCR runs are shown in Tables AWB, AWC and AWD.

**Table AWA. Probe Name Ag3763**

| **Primers** | **Sequences** | **Length** | **Start Position** | **SEQ ID NO** |
|---|---|---|---|---|
| Forward | 5'-gatggggagagcgtatttacc-3' | 21 | 274 | 428 |
| Probe | TET-5'-tcaaagatctattcctacatgagcccga-3'-TAMRA | 28 | 301 | 429 |
| Reverse | 5'-aaacgcattccagagcattt-3' | 20 | 331 | 430 |

**Table AWB. CNS_neurodegeneration v1.0**

| **Tissue Name** | **Rel. Exp.(%) Ag3763, Run 211175130** | **Tissue Name** | **Rel. Exp.(%) Ag3763, Run 211175130** |
|---|---|---|---|
| AD 1 Hippo | 19.8 | Control (Path) 3 Temporal Ctx | 17.7 |
| AD 2 Hippo | 46.7 | control (path) 4 Temporal Ctx | 59.9 |
| AD 3 Hippo | 19.6 | AD 1 Occipital Ctx | 27.9 |
| AD 4 Hippo | 13.2 | AD 2 Occipital Ctx (Missing) | 0.0 |
| AD 5 hippo | 86.5 | AD 3 Occipital Ctx | 20.7 |
| AD 6 Hippo | 92.0 | AD 4 Occipital Ctx | 20.9 |
| Control 2 Hippo | 46.7 | AD 5 Occipital Ctx | 39.8 |
| Control 4 Hippo | 25.2 | AD 6 Occipital Ctx | 44.8 |
| Control (Path) 3 Hippo | 12.0 | Control 1 Occipital Ctx | 15.4 |
| AD 1 Temporal Ctx | 45.7 | Control 2 Occipital Ctx | 49.0 |
| AD 2 Temporal Ctx | 47.3 | Control 3 Occipital Ctx | 27.7 |
| AD 3 Temporal Ctx | 12.2 | Control 4 Occipital Ctx | 14.5 |
| AD 4 Temporal Ctx | 24.3 | Control (Path)1 Occipital Ctx | 96.6 |
| AD 5 Inf Temporal Ctx | 100.0 | Control (Path)2 Occipital Ctx | 28.9 |
| AD 5 SupTemporal Ctx | 87.1 | Control (Path) 3 Occipital Ctx | 5.9 |
| AD 6 Inf Temporal Ctx | 61.1 | Control (Path) 4 Occipital Ctx | 35.1 |
| AD 6 Sup Temporal Ctx | 88.9 | Control 1 Parietal Ctx | 18.0 |
| Control 1 Temporal Ctx | 12.5 | Control 2 Parietal Ctx | 66.0 |
| Control 2 Temporal Ctx | 48.6 | Control 3 Parietal Ctx | 34.9 |
| Control 3 Temporal Ctx | 30.6 | Control (Path) 1 Parietal | 77.9 |
| Control 4 Temporal Ctx | 13.2 | Control (Path) 2 Parietal Ctx | 45.1 |
| Control (Path) 1 Temporal Ctx | 85.3 | Control (Path) 3 Parietal Ctx | 6.6 |
| Control (Path) 2 Temporal Ctx | 65.1 | Control (Path) 4 Parietal Ctx | 52.5 |

**Table AWC. General_screening_panel_vl.4**

| **Tissue Name** | **Rel. Exp.(%) Ag3763, Run 216557719** | **Tissue Name** | **Rel. Exp.(%) Ag3763, Run 216557719** |
|---|---|---|---|
| Adipose | 7.7 | Renal ca. TK-10 | 24.8 |
| Melanoma* Hs688(A).T | 31.4 | Bladder | 17.6 |
| Melanoma* Hs688(B).T | 27.7 | Gastric ca. (liver met.) NCI-N87 | 30.4 |
| Melanoma* M14 | 30.8 | Gastric ca. KATO III | 42.0 |
| Melanoma* LOXIMVI | 29.1 | Colon ca. SW-948 | 7.7 |
| Melanoma* SK-MEL-5 | 19.2 | Colon ca. SW480 | 40.6 |
| Squamous cell carcinoma SCC-4 | 16.4 | Colon ca.* (SW480 met) SW620 | 54.3 |
| Testis Pool | 6.4 | Colon ca. HT29 | 18.9 |
| Prostate ca.* (bone met) PC-3 | 52.9 | Colon ca. HCT-116 | 58.2 |
| Prostate Pool | 23.8 | Colon ca. CaCo-2 Colon ca. CaCo-2 | 21.6 |
| Placenta | 9.3 | Colon cancer tissue | 21.8 |
| Uterus Pool | 7.9 | Colon ca. SW1116 | 11.2 |
| Ovarian ca. OVCAR-3 | 19.3 | Colon ca. Colo-205 | 10.8 |
| Ovarian ca. SK-OV-3 | 35.4 | Colon ca. SW-48 | 11.2 |
| Ovarian ca. OVCAR-4 | 13.7 | Colon Pool | 8.8 |
| Ovarian ca. OVCAR-5 | 35.8 | Small Intestine Pool | 16.0 |
| Ovarian ca. IGROV-1 | 31.9 | Stomach Pool | 11.0 |
| Ovarian ca. OVCAR-8 | 52.1 | Bone Marrow Pool | 6.2 |
| Ovary | 4.5 | Fetal Heart | 6.0 |
| Breast ca. MCF-7 | 37.9 | Heart Pool | 4.6 |
| Breast ca. MDA-MB-231 | 36.9 | Lymph Node Pool | 14.1 |
| Breast ca. BT 549 | 44.1 | Fetal Skeletal Muscle | 5.5 |
| Breast ca. T47D | 100.0 | Skeletal Muscle Pool | 12.2 |
| Breast ca. MDA-N | 12.2 | Spleen Pool | 12.3 |
| Breast Pool | 8.7 | Thymus Pool | 14.4 |
| Trachea | 16.3 | CNS cancer (glio/astro) U87-MG | 25.0 |
| Lung | 7.6 | CNS cancer (glio/astro) U-118-MG | 52.9 |
| Fetal Lung | 15.0 | CNS cancer (neuro;met) SK- N-AS | 42.9 |
| Lung ca. NCI-N417 | 14.8 | CNS cancer (astro) SF-539 | 20.9 |
| Lung ca. LX-1 | 47.3 | CNS cancer (astro)SNB-75 | 18.9 |
| Lung ca. NCI-H146 | 12.0 | CNS cancer (glio)SNB-19 | 32.1 |
| Lung ca. SHP-77 | 47.0 | CNS cancer (glio) SF-295 | 63.3 |
| Lung ca. A549 | 23.8 | Brain (Amygdala) Pool | 7.5 |
| Lung ca. NCI-H526 | 17.1 | Brain (cerebellum) | 10.1 |
| Lung ca. NCI-H23 | 64.6 | Brain (fetal) | 12.6 |
| Lung ca. NCI-H460 | 26.2 | Brain (Hippocampus) Pool | 8.7 |
| Lung ca. HOP-62 | 8.8 | Cerebral Cortex Pool | 11.5 |
| Lung ca. NCI-H522 | 42.9 | Brain (Substantia nigra) Pool | 10.8 |
| Liver | 0.6 | Brain (Thalamus) Pool | 14.5 |
| Fetal Liver | 18.6 | Brain (whole) | 13.3 |
| Liver ca. HepG2 | 10.1 | Spinal Cord Pool | 13.3 |
| Kidney Pool | 12.2 | Adrenal Gland | 9.9 |
| Fetal Kidney | 4.8 | Pituitary gland Pool | 3.5 |
| Renal ca. 786-0 | 18.2 | Salivary Gland | 14.4 |
| Renal ca. A498 | 10.4 | Thyroid(female) | 11.3 |
| Renal ca. ACHN | 17.1 | Pancreatic ca. CAPAN2 | 17.7 |
| Renal ca. UO-31 | 20.3 | Pancreas Pool | 21.5 |

**Table AWD. Panel 4.1D**

| **Tissue Name** | **Rel. Exp.(%) Ag3763, Run 170069604** | **Tissue Name** | **Rel. Exp.(%) Ag3763, Run 170069604** |
|---|---|---|---|
| Secondary Th1 act | 72.7 | HUVECIL-1beta | 56.3 |
| Secondary Th2 act | 73.2 | HUVEC IFN gamma | 45.1 |
| Secondary Trl act | 58.2 | HUVECTNF alpha + IFN gamma | 25.3 |
| Secondary Thl rest | 8.7 | HUVEC TNF alpha+IL4 | 37.4 |
| Secondary Th2 rest | 28.1 | HUVEC IL-11 | 26.4 |
| Secondary Tr1 rest | 14.7 | Lung Microvascular EC none | 48.0 |
| Primary Th1 act | 52.1 | Lung Microvascular EC TNFalpha IL-lbeta | 27.2 |
| Primary Th2 act | 52.1 | Microvascular Dermal EC none | 48.3 |
| Primary Tr1 act | 52.5 | Microsvasular Dermal EC TNF alpha+IL-1beta | 26.8 |
| Primary Th1 rest | 28.1 | Bronchial epithelium TNFalpha + IL1beta | 24.5 |
| Primary Th2 rest | 17.9 | Small airway epithelium none | 17.3 |
| Primary Tr1 rest | 36.9 | Small airway epithelium TNFalpha + IL-1beta | 27.9 |
| CD45RA CD4 lymphocyte | 43.5 | Coronery artery SMC rest | 26.8 |
| CD45RO CD4 lymphocyte act | 43.2 | Coronery artery SMC TNFalpha + IL-lbeta | 15.9 |
| CD8 lymphocyte act | 48.0 | Astrocytes rest | 29.9 |
| Secondary CD8 lymphocyte rest | 37.9 | Astrocytes TNFalpha + IL-1 beta | 23.5 |
| Secondary CD8 lymphocyte act | 32.5 | KU-812 (Basophil) rest | 46.7 |
| CD4 lymphocyte none | 11.5 | KU-812 (Basophil) PMA/ionomycin | 60.3 |
| 2ryTh1/Th2/Tr1 anti-CD95 CH11 | 23.8 | CCD1106 (Keratinocytes) none | 34.9 |
| LAK cells rest | 29.7 | CCD1106 (Keratinocytes) TNFalpha + IL-1beta | 29.5 |
| LAK cells IL-2 | 38.7 | Liver cirrhosis | 23.7 |
| LAK cells IL-2+IL-12 | 55.9 | NCI-H292 none | 20.7 |
| LAK cells IL-2+IFN gamma | 46.3 | NCI-H292 IL-4 | 28.7 |
| LAK cells IL-2+ IL-18 | 43.8 | NCI-H292 IL-9 | 43.8 |
| LAK cells PMA/ionomycin | 30.1 | NCI-H292 IL-13 | 27.7 |
| NK Cells IL-2 rest | 57.0 | NCI-H292 IFN gamma | 48.3 |
| Two Way MIR 3 day | 26.1 | HPABC none | 25.3 |
| Two Way MLR 5 day | 37.6 | HPAEC TNF alpha + IL-1 beta | 26.4 |
| Two Way MLR 7 day | 39.8 | Lung fibroblast none | 41.8 |
| PBMC rest PBMC rest | 19.2 | Lung fibroblast TNF alpha + IL-1 beta | 15.1 |
| PBMC PWM | 38.4 | Lung fibroblast IL-4 | 39.0 |
| PBMCPHA-L | 39.5 | Lung fibroblast IL-9 | 43.8 |
| Ramos (B cell) none | 80.7 | Lung fibroblast IL-13 | 44.1 |
| Ramos (B cell) ionomycin | 76.8 | Lung fibroblast IFN gamma | 35.8 |
| B lymphocytes PWM | 43.2 | Dermal fibroblast CCD1070 rest | 95.9 |
| B lymphocytes CD40L and IL-4 | 32.5 | Dermal fibroblast CCD1070 TNF alpha | 90.8 |
| EOL-1 dbcAMP | 29.3 | Dermal fibroblast CCD1070 IL-1 beta | 45.7 |
| EOL-1 dbcAMP PMA/ionomycin | 48.0 | Dermal fibroblast IFN gamma | 19.6 |
| Dendritic cells none | 44.1 | Dermal fibroblast IL-4 | 66.0 |
| Dendritic cells LPS | 8.9 | Dermal Fibroblasts rest | 35.8 |
| Dendritic cells anti-CD40 | 29.7 | Neutrophils TNFa+LPS | 9.8 |
| Monocytes rest | 25.3 | Neutrophils rest | 17.6 |
| Monocytes LPS | 18.0 | Colon | 13.2 |
| Macrophages rest | 27.9 | Lung | 20.6 |
| Macrophages LPS | 9.3 | Thymus | 100.0 |
| HUVEC none | 48.3 | Kidney | 19.9 |
| HUVEC starved | 31.4 | | |

**CNS_neurodegeneration_v1.0 Summary:** Ag3763 This panel confirms the expression of the CG57348-01 gene at low to moderate levels in the brains of an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

**General_screening_panel_v1.4 Summary:** Ag3763 Expression of the CG57348-01 gene is highest in a breast cancer cell line (CT = 29.8). Interestingly, this gene appears to be more highly expressed in a number of cancer cell lines than in normal tissues. Expression of this gene appears to be upregulated in CNS, colon, gastric, lung, breast and ovarian cancer cell lines. Therefore, therapeutic modulation of the activity of this gene or its protein product, using small molecule drugs, antibodies or protein therapeutics, may be of benefit in the treatment of CNS, colon, gastric, lung, breast, and ovarian cancer.

In addition, this gene is expressed at low levels in all regions of the central nervous system examined, including in amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

This gene is also expressed at low levels in a number of tissues with metabolic or endocrine function, including adipose, pancreas, adrenal gland, thyroid, gastrointestinal tract, skeletal muscle, heart and liver. Therefore, therapeutic modulation of the activity of this gene may prove useful in the treatment of endocrine/metabolically related diseases, such as obesity and diabetes. Interestingly, this gene is expressed at higher levels in fetal liver (CT = 32.2) when compared to adult liver (CT = 37.2), suggesting that expression of this gene can be used to distinguish fetal from adult liver and may be of benefit in the treatment of liver degeneration.

**Panel 4.1D Summary:** Ag3763 This gene is expressed at low to moderate levels in a wide range of cell types of significance to the immune response in health and disease. These cells include T-cells, B-cells, endothelial cells, macrophages, monocytes, dendritic cells, basophils, eosinophils and peripheral blood mononuclear cells, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues.

This pattern is in agreement with the expression profile in General_screening_panel_v1.4 and also suggests a role for the gene product in cell survival and proliferation.

Therefore, therapeutic modulation of the activity of this gene or its protein product may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### NOV60

Expression of gene NOV60/CG57574-01 was assessed using the primer-probe set Ag3289, described in Table AXA.

**Table AXA. Probe Name Ag3289**

| **Primers** | **Sequences** | **Length** | **Start Position** | **SEQ ID NO** |
|---|---|---|---|---|
| Forward | 5'-accagsstgtcaactactcctt-3' | 22 | 419 | 431 |
| Probe | TET-5'-ccactccaectacttggtgaaccagg-3'-TAMRA | 26 | 453 | 432 |
| Reverse | 5'-ggaaattgacactctggtcaaa-3' | 22 | 484 | 433 |

**CNS_neurodegeneration_v1.0 Summary:** Ag3289 Expression of this gene is low/undetectable (CTs > 35) across all of the samples on this panel (data not shown).

**General_screening_panel v1.4 Summary:** Ag3289 Expression of this gene is low/undetectable (CTs > 35) across all of the samples on this panel (data not shown). The amp plot indicates that there were experimental difficulties with this run.

**Panel 4D Summary:** Ag3289 Expression of this gene is low/undetectable (CTs> 35) across all of the samples on this panel (data not shown).

### NOV61

Expression of NOV61/CG57505-01 was assessed using the primer-probe set Ag3259, described in Table AYA. Results of the RTQ-PCR runs are shown in Tables AYB, AYC and AYD.

**Table AYA. Probe Name Ag3259**

| **Primers** | **Sequences** | **Length** | **Start Position** | **SEQ ID NO:** |
|---|---|---|---|---|
| Forward | 5'-catcgagacccagagtaaagc-3' | 21 | 604 | 434 |
| Probe | TET-5'-catgacaatgctcaccattgaacagt-3'-TAMRA | 26 | 625 | 435 |
| Reverse | 5'-tttcattttctgaatggcaaac-3' | 22 | 667 | 436 |

**Table AYB. CNS_neurodegeneration_v1.0**

| **Tissue Name** | **Rel. Exp.(%) Ag3259, Run 209990878** | **Tissue Name** | **Rel. Exp.(%) Ag3259, Run 209990878** |
|---|---|---|---|
| AD 1 Hippo | 7.1 | Control (Path) 3 Temporal Ctx | 3.5 |
| AD 2 Hippo | 14.9 | Control (Path) 4 Temporal Ctx. | 16.8 |
| AD 3 Hippo | 5.8 | AD1 Occipital Ctx | 8.3 |
| AD 4 Hippo | 4.3 | AD 2 Occipital Ctx (Missing) | 0.0 |
| AD 5 hippo | 100.0 | AD 3 Occipital Ctx | 6.6 |
| AD 6 Hippo | 6.1 | AD 4 Occipital Ctx | 9.7 |
| Control 2 Hippo | 11.9 | AD 5 Occipital Ctx | 6.8 |
| Control 4 Hippo | 7.5 | AD 6 Occipital Ctx | 27.2 |
| Control (Path) 3 Hippo | 4.3 | Control Occipital Ctx | 2.4 |
| AD 1 Temporal Ctx | 7.7 | Control 2 Occipital Ctx | 30.1 |
| AD 2 Temporal Ctx | 17.4 | Control 3 Occipital Ctx | 8.0 |
| AD 3 Temporal Ctx | 5.7 | Control 4 Occipital Ctx | 4.8 |
| AD 4 Temporal Ctx | 10.6 | Control (Path) 1 Occipital Ctx | 51.1 |
| AD5 Inf Temporal Ctx | 67.8 | Control (Path) 2 Occipital Ctx | 4.8 |
| AD 5 SupTemporal Ctx | 33.9 | Control (Path) 3 Occipital Ctx | 0.6 |
| AD 6 Inf Temporal Ctx | 23.7 | Control (Path) 4 Occipital Ctx | 10.7 |
| AD 6 Sup Temporal Ctx | 17.2 | Control 1 Parietal Ctx | 3.2 |
| Control 1 Temporal Ctx | 3.9 | Control 2 Parietal Ctx | 9.7 |
| Control 2 Temporal Ctx | 23.8 | Control 3 Parietal Ctx | 6.9 |
| Control 3 Temporal Ctx | 4.2 | Control (Path) 1 Parietal Ctx | 38.4 |
| Control 4 Temporal Ctx | 4.4 | Control (Path) 2 Parietal Ctx | 11.0 |
| Control (Path) 1 Temporal Ctx | 37.4 | Control (Path) 3 Parietal Ctx | 2.6 |
| Control (Path) 2 Temporal Ctx | 9.3 | Control (Path) 4 Parietal Ctx | 30.1 |

**Table AYC. General_screening_panel_v1.4**

| **Tissue Name** | **Rel. Exp.(%) Ag3259, Run 214694855** | **Tissue Name** | **Rel. Exp.(%) Ag3259, Run 214694855** |
|---|---|---|---|
| Adipose | 4.2 | Renal ca. TK-10 | 30.1 |
| Melanoma* Hs688(A).T | 6.8 | Bladder | 10.8 |
| Melanoma* Hs688(B).T | 5.1 | Gastric ca. (liver met.) NCI- N87 | 100.0 |
| Melanoma* M 14 | 31.9 | Gastric ca. KATO III | 25.5 |
| Melanoma* LOXIMVI | 21.6 | Colon ca. SW-948 | 2.2 |
| Melanoma* SK-MEL-5 | 17.3 | Colon ca. SW480 | 52.5 |
| Squamous cell carcinoma SCC-4 | 14.9 | Colon ca.* (SW480 met) SW620 | 42.9 |
| Testis Pool | 8.0 | Colon ca. HT29 | 24.0 |
| Prostate ca.* (bone met) PC-3 | 14.3 | Colon ca. HCT-116 | 15.4 |
| Prostate Pool | 4.2 | Colon ca. CaCo-2 | 88.9 |
| Placenta | 6.9 | Colon cancer tissue | 23.3 |
| Uterus Pool | 1.2 | Colon ca. SW1116 | 3.0 |
| Ovarian ca. OVCAR-3 | 9.7 | Colon ca. Colo-205 | 8.0 |
| Ovarian ca. SK-OV-3 | 6.1 | Colon ca. SW-48 | 3.7 |
| Ovarian ca. OVCAR-4 | 6.4 | Colon Pool | 6.4 |
| Ovarian ca. OVCAR-5 | 62.4 | Small Intestine Pool | 4.5 |
| Ovarian ca. IGROV-1 | 15.8 | Stomach Pool | 3.7 |
| Ovarian ca. OVCAR-8 | 4.4 | Bone Marrow Pool | 2.6 |
| Ovary | 6.2 | Fetal Heart | 12.2 |
| Breast ca. MCF-7 | 94.6 | Heart Pool | 1.0 |
| Breast ca. MDA-MB-231 | 32.5 | Lymph Node Pool | 5.8 |
| Breast ca. BT 549 | 63.7 | Fetal Skeletal Muscle | 4.2 |
| Breast ca. T47D | 34.4 | Skeletal Muscle Pool | 5.1 |
| Breast ca. MDA.N | 17.4 | Spleen Pool | 9.7 |
| Breast Pool | 8.5 | Thymus Pool | 14.4 |
| Trachea | 10.3 | CNS cancer (glio/astro) U87-MG | 42.9 |
| Lung | 2.0 | CNS cancer (glio/astro) U-118-MG | 17.3 |
| Fetal Lung | 21.2 | CNS cancer (neuro;met) SK-N-AS | 26.8 |
| Lung ca. NCI.N417 | 5.4 | CNS cancer (astro) SF-539 | 14.9 |
| Lung ca. LX-1 | 48.3 | CNS cancer (astro) SNB-75 | 22.1 |
| Lung ca.NCI-H146 | 9.1 | CNS cancer (glio) SNB-19 | 3.7 |
| Lung ca. SHP-77 | 17.3 | CNS cancer (glio) SF-295 | 24.5 |
| Lung ca. A549 | 13.3 | Brain (Amygdala) Pool | 6.6 |
| Lung ca. NCI-H526 | 4.4 | Brain(cerebellum) | 14.0 |
| Lung ca. NCI-H23 | 39.0 | Brain (fetal) | 24.5 |
| Lung ca. NCI-H460 | 7.6 | Brain (Hippocampus) Pool | 8.1 |
| Lung ca. HOP-62 | 9.9 | Cerebral Cortex Pool | 11.9 |
| Lung ca. NCI-H522 | 16.2 | Brain (Substantia nigra) Pool | 7.4 |
| Liver | 0.9 | Brain (Thalamus) Pool | 6.3 |
| Fetal Liver | 5.0 | Brain (whole) | 8.2 |
| Liver ca. HepG2 | 33.0 | Spinal Cord Pool | 5.8 |
| Kidney Pool | 3.5 | Adrenal Gland | 24.8 |
| Fetal Kidney | 15.2 | Pituitary gland Pool | 5.3 |
| Renal ca. 786-0 | 20.2 | Salivary Gland | 4.4 |
| Renal ca. A498 | 17.2 | Thyroid (female) | 2.1 |
| Renal ca. ACHN | 17.3 | Pancreatic ca. CAPAN2 | 18.6 |
| Renal ca. UO-31 | 46.3 | Pancreas Pool | 10.4 |

**Table AYD. Panel 4D**

| **Tissue Name** | **Rel. Exp.(%) Ag3259, Run 164537290** | **Tissue Name** | **Rel. Exp.(%)Ag3259, Run 164537290** |
|---|---|---|---|
| Secondary Th1 act | 22.5 | HUVEC IL-1beta | 13.5 |
| Secondary Th2 act | 20.6 | HUVEC IFN gamma | 50.0 |
| Secondary Tr1 act | 23.3 | HUVEC TNF alpha + IFN gamma | 21.6 |
| Secondary Th1 rest | 7.7 | HUVEC TNF alpha + IL4 | 15.4 |
| Secondary Th2 rest | 9.9 | HUVEC IL-11 | 27.0 |
| Secondary Tr1 rest | 12.8 | Lung Microvascular EC none | 24.5 |
| Primary Th1 act | 9.4 | Lung Microvascular EC TNFalpha +IL-1 beta | 24.3 |
| Primary Th2 act | 9.5 | Microvascular Dermal EC none | 46.3 |
| Primary Tr1 act | 12.5 | Microsvasular Dermal EC TNFalpha+IL-1beta | 22.2 |
| Primary Th1 rest | 56.3 | Bronchial epithelium TNFalpha+ IL1beta | 27.5 |
| Primary Th2 rest | 26.1 | Small airway epithelium none | 9.6 |
| Primary Tr1 rest | 19.2 | Small airway epithelium TNFalpha IL-I beta | 50.0 |
| CD45RA CD4 lymphocyte act | 9.9 | Coronery artery SMC rest | 13.0 |
| CD45RO CD4 lymphocyte act | 24.0 | Coronery artery SMC TNFalpha + IL-1beta | 6.6 |
| CD8 lymphocyte act | 11.9 | Astrocytes rest | 6.2 |
| Secondary CD8 lymphocyte rest | 13.8 | Astrocytes TNFalpha + IL-1beta | 5.0 |
| Secondary CD8 lymphocyte act | 10.5 | KU-812 (Basophil) rest | 28.7 |
| CD4 lymphocyte none | 9.1 | KU-812 (Basophil) PMA/ionomycin | **100.0** |
| 2ry Th1/Th2/Tr1_anti-CD95 CH11 | 22.8 | CCD1106 (Keratinocytes)none | 24.5 |
| LAK cells rest | 1.6 | CCD1106 (Keratinocytes) TNFalpha+IL-1beta | 12.9 |
| LAK cell IL-2 | 21.0 | Liver cirrhosis | 3.4 |
| LAK cells IL-2+IL-12 | 17.3 | Lupus kidney | 4.1 |
| LAK cells IL-2+IFN gamma | 28.5 | NCI-H292 none | 31.4 |
| LAK cells IL-2+ IL-18 | 27.9 | NCI-H292 IL-4 | 22.7 |
| LAK cells PMA/ionomycin | 7.1 | NCI-H292 IL-9 | 28.5 |
| NK Cells IL-2 rest | 16.5 | NCI-H292 IL-13 | 11.2 |
| Two Way MLR 3 day | 18.8 | NCI-H292 IFN gamma | 15.1 |
| Two Way MLR 5 day | 9.7 | HPAEC none | 24.1 |
| Two Way MLR 7 day | 9.3 | HPAEC TNF alpha+IL-1beta | 17.8 |
| PBMC rest | 8.2 | Lung fibroblast none | 7.1 |
| PBMC PWM | 52.9 | Lung fibroblast TNF alpha + IL-1beta | 10.3 |
| PBMC PHA-L | 24.8 | Lung fibroblast IL-4 | 10.3 |
| Ramos (B cell) none | 24.1 | Lung fibroblast IL-9 | 12.5 |
| Ramos (B cell) ionomycin | 81.8 | Lung fibroblast IL-13 | 8.3 |
| B lymphocytes PWM | 49.0 | Lung fibroblast IFN gamma | 16.8 |
| B lymphocytes CD40L and IL-4 | 27.0 | Dermal fibroblast CCD1070 rest | 22.8 |
| EOL-1 dbcAMP | 15.4 | Dermal alpha fibroblast CCD1070 TNF alpha | 45.4 |
| EOL-1 dbcAMP PMA/ionomycin | 24.5 | Dermal fibroblast CCD1070 IL-1 beta | 12.6 |
| Dendritic cells none | 18.6 | Dermal fibroblast IFN gamma | 9.3 |
| Dendritic cells LPS | 10.4 | Dermal fibroblast IL-4 | 16.6 |
| Dendritic cells anti-CD40 | 14.1 | IBD Colitis 2 | 2.6 |
| Monocytes rest | 11.0 | IBD Crohn's | 3.5 |
| Monocytes LPS | 7.3 | Colon | 13.9 |
| Macrophages rest | 19.3 | Lung | 11.8 |
| Macrophages LPS | 11.7 | Thymus | 29.1 |
| HUVEC none | 30.6 | Kidney | 41.8 |
| HUVEC starved | 63.7 | | |

CNS_neurodegeneration_v1.0 Summary: Ag3259 This panel confirms the expression of the CG57505-01 gene at moderate levels in the brains of an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

General_screening_panel_v1.4 Summary: Ag3259 Expression of the CG57505-01 gene is highest in a gastric cancer cell line (CT = 25.6). Interestingly, expression of this gene appears to be higher in cancer cell lines than in normal tissues. Specifically, CG57505-01 gene expression appears to be upregulated in colon, gastric, renal, lung and breast cancer cell lines. Therefore, therapeutic modulation of the activity of this gene or its protein product, through the use of small molecule drugs, protein therapeutics or antibodies, might be beneficial in the treatment of these cancers.

In addition, this gene is expressed at moderate levels in all regions of the central nervous system examined, including in amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression.

Among tissues with metabolic or endocrine function, this gene is expressed at moderate levels in adipose, adrenal gland, thyroid, pituitary gland, gastrointestinal tract, pancreas, skeletal muscle, heart and liver. Therefore, therapeutic modulation of the activity of this gene may prove useful in the treatment of endocrine/metabolically related diseases, such as obesity and diabetes. This gene is moderately expression in pancreas, which includes the insulin-secreting islets of Langerhans. Insulin secretion is augmented by protein kinase C activation. The CG57505-01 gene encodes a protein with homology to a protein kinase C-binding protein, and as such, may be a therapeutic modulator of insulin secretion in Type 2 diabetes.

Panel 4D Summary: Ag3259 The CG57505-01 transcript is expressed in the PMA and ionomycin treated basophil cell line KU-812 (CT=27) and to a lesser extent in untreated KU-812 cells (CT=29). Therefore, antibody or small molecule therapies designed with the protein encoded for by this gene could block or inhibit inflammation or tissue damage due to basophil activation in response to asthma, allergies, hypersensitivity reactions, psoriasis, and viral infections.

This gene is also expressed at low levels in Ramos (B cells) (CT = 29) and expression of this gene is stimulated in these cells by ionomycin (CT=27). Expression of this gene in B cells suggests that this gene may be involved in rheumatic disease including rheumatoid arthritis, lupus, osteoarthritis, and hyperproliferative B cell disorders.

Furthermore, treatment of small airway epithelium with TNFalpha + IL-1beta stimulated the expression of the CG57721-01 gene. Therefore, modulation of the expression or activity of the protein encoded by this transcript through the application of small molecule therapeutics may be useful in the treatment of asthma, COPD, and emphysema.

### NOV62

Expression of NOV62/CG57473-01 was assessed using the primer-probe set Ag3251, described in Table AZA. Please note that CG57473-02 represents a full-length physical clone of the CG57473-01 gene, validating the prediction of the gene sequence.

**Table AZA. Probe Name Ag3251**

| **Primers** | **Sequences** | **Length** | **Start Position** | **SEQ ID NO** |
|---|---|---|---|---|
| Forward | 5'-tctcaggaagtggtgaagga-3' | 20 | 993 | 437 |
| Probe | TET-5'-caattctccaaggatacattggaaaa-3'-TAMRA | 26 | 1020 | 438 |
| Reverse | 5'-atacaaeccctcggaacga-3' | 19 | 1058 | 439 |

**CNS_neurodegeneration_v1.0** Summary: Ag3251 Expression of this gene is low/undetectable (CTs > 35) across all of the samples on this panel (data not shown).

**General_screening_panel_v1.4** Summary: Ag3251 Results from one experiment with the CG57473-01 gene are not included. The amp plot indicates that there were experimental difficulties with this run.

**Panel 4D Summary:** Ag3251 Expression of this gene is low/undetectable (CTs > 35) across all of the samples on this panel (data not shown).

### NOV63

Expression of NOV63/CG57777-01 was assessed using the primer-probe set Ag3327, described in Table BFA. Results of the RTQ-PCR runs are shown in Tables BFB, BFC and BFD.

**Table BFA. Probe Name Ag3327**

| **Primers** | **Sequences** | **Length** | **Start Position** | **SEQ ID NO** |
|---|---|---|---|---|
| Forwards | 5'-ttctgtgaaaccaatgagaaca-3' | 22 | 424 | 440 |
| Probe | TET-5'-cacaacgtaccagaatttctgggaca-3'-TAMRA | 26 | 450 | 441 |
| Reverse | 5'-ccctctaaccactgctttagct-3' | 22 | 477 | 442 |

**Table BFB. CNS_neurodegeneration_v1.0**

| **Tissue Name** | **Rel. Exp.(%) Ag3327, Run 210138310** | **Rel. Exp.(%) Ag3327, Run 224063127** | **Tissue Name** | **Rel.Exp.(%) Ag3327, Run 210138310** | **Rel. Exp.(%) Ag3327, Run 224063127** |
|---|---|---|---|---|---|
| AD 1 Hippo | 6.1 | 6.0 | Control (Path) 3 Temporal Ctx | 14.0 | 12.3 |
| AD 2 Hippo | 28.1 | 29.3 | Control (Path) 4 Temporal Ctx | 69.7 | 63.7 |
| AD 3 Hippo | 14.9 | 13.5 | AD 1 Occipital Ctx | 18.8 | 31.4 |
| AD 4 Hippo | 24.7 | 15.9 | AD 2 Occipital Ctx (Missing) | 0.0 | 0.0 |
| AD 5 Hippo | 59.0 | 65.1 | AD 3 Occipital Ctx | 17.1 | 9.1 |
| AD 6 Hippo | 45.7 | 52.5 | AD 4 Occipital Ctx | 48.0 | 35.8 |
| Control 2 Hippo | 38.2 | 28.7 | AD 5 Occipital Ctx | 52.9 | 0.0 |
| Control 4 Hippo | 15.5 | 16.4 | AD 6 Occipital Ctx | 23.0 | 40.1 |
| Control (Path) 3 Hippo | 11.7 | 2.5 | control 1 Occipital Ctx | 1.2 | 2.1 |
| AD 1 Temporal Ctx | 16.3 | 14.2 | Control 2 Occipital Ctx | 31.6 | 59.0 |
| AD 2 Temporal Ctx | 21.9 | 29.5 | Control 3 Occipital Ctx | 62.9 | 60.3 |
| AD 3 Temporal Ctx | 15.1 | 12.2 | Control 4 Occipital Ctx | 10.2 | 6.9 |
| AD 4 Temporal Ctx | 24.7 | 16.0 | Control (Path) 1 Occipital Ctx | 100.0 | 96.6 |
| AD 5 Inf Ctx Temporal Ctx | 85.9 | 41.8 | Control (Path) 2 Occipital Ctx | 44.1 | 10.0 |
| AD 5 Sup Temporal Ctx | 47.3 | 59.9 | Control (Path) 3 Occipital Ctx | 1.7 | 2.0 |
| AD 6 Inf Temporal Ctx | 57.4 | 61.6 | Control (Path) 4 Occipital Ctx | 55.5 | 41.2 |
| AD 6 Sup Temporal Ctx | 77.9 | 70.7 | Control 1 Parietal Ctx | 9.0 | 7.1 |
| Control 1 Temporal Ctx | 14.3 | 13.6 | Control 2 Parietal Ctx | 52.9 | 67.8 |
| Control 2 Temporal Ctx | 34.9 | 18.8 | Control 3 Parietal Ctx | 15.4 | 10.9 |
| Control 3 Temporal Ctx | 42.6 | 28.1 | Control (Path) 1 Parietal Ctx | 84.1 | 77.4 |
| Control 3 Temporal Ctx | 19.6 | 13.1 | Control (Path) 2 Parietal Ctx | 43.8 | 58.6 |
| Control (Path) 1 Temporal Ctx | 75.8 | 100.0 | Control (Path) 3 Parietal Ctx | 9.2 | 9.0 |
| Control (Path) 2 Temporal Ctx | 98.6 | 72.7 | Control (Path) 4 Parietal Ctx | 89.5 | 75.8 |

**Table BFC. General_screening_panel_v1.4**

| **Tissue Name** | **Rel. Exp.(%) Ag3327, Run 215678614** | **Tissue Name** | **Ret. Exp.(%) Ag3327, Run 215678614** |
|---|---|---|---|
| Adipose | 2.3 | Renal ca. TK-10 | 13.3 |
| Metanoma*Hs688(A).T | 2.8 | Bladder | 17.1 |
| Melanoma* Hs688(B).T | 1.1 | Gastric ca.(liver met.) NCI-N87 | 0.0 |
| Melanoma* M14 | 0.0 | Gastric ca. KATO III | 1.2 |
| Melanoma* LOXIWVI | 0.7 | Colon ca. SW-948 | 0.0 |
| Melanoma" SK-MEL-5 | 0.6 | Colon ca. SW480 | 8.1 |
| Squamous cell carcinoma SCC-4 | 2.0 | Colon ca.* (SW480 met) SW620 | 0.0 |
| Testis Pool | 15.1 | Colon ca. HT29 | 1.5 |
| Prostate ca.*(bone met) PC-3 | 0.6 | Colon ca. HCT-116 | 8.2 |
| ProstatePool | 1.6 | Colon ca.CaCo-2 | 6.1 |
| Placenta | 0.0 | Colon cancer tissue | 0.0 |
| Uterus Pool | 0.0 | Colon ca. SW1116 | 0.0 |
| Ovarian ca. OVCAR-3 | 1.6 | Colon ca. Colo-205 | 0.0 |
| Ovarian ca. SK-OV-3 | 1.3 | Colon ca. SW-48 | 0.0 |
| Ovarian ca. OVCAR-4 | 1.5 | Colon Pool | 0.0 |
| Ovarian ca. OVCAR-5 | 7.8 | Small Intestine Pool | 0.7 |
| Ovarian ca. IGROV-1 | 0.0 | Stomach Pool | 4.1 |
| Ovarian ca. OVCAR-8 | 1.1 | Bone Marrow Pool | 1.0 |
| Ovary | 4.6 | Fetal Heart | 0.0 |
| Breast ca. MCF-7 | 0.0 | Heart Pool | 0.0 |
| Breast ca. MDA-MB-231 | 0.0 | Lymph Node Pool | 0.6 |
| Breast ca. BT 549 | 9.2 | Fetal Skeletal Muscle | 0.0 |
| Breast ca. T47D | 1.5 | Skeletal Muscle Pool | 3.5 |
| Breast ca. MDA-N | 0.0 | Spleen Pool | 1.4 |
| Breast Pool | 14.4 | Thymus Pool | 2.2 |
| Trachea | 0.0 | CNS cancer (glio/astro) U87-MG | 13.2 |
| Lung | 16.7 | CNS cancer (glio/astro) U-118-MG | 0.0 |
| Fetal Lung | 15.6 | CNS cancer (neuro;met) SK-N-AS | 1.3 |
| Lung ca. NCI-N417 | 1.8 | CNS cancer (astro) SF-539 | 1.3 |
| Lung ca LX-1 | 2.0 | CNS cancer (astro)SNB-75 | 100.0 |
| Lung ca. NCI-HI46 | 1.6 | CNS cancer (glio) SNB-19 | 2.7 |
| Lung ca. SHP-77 | 0.0 | CNS cancer (glio) SF-295 | 39.8 |
| Lung ca. A549 | 12.7 | Brain (Amygdala) Pool | 4.3 |
| Lung ca. NCI-H526 | 1.4 | Brain (cerebellum) | 8.7 |
| Lung ca. NCI-H23 | 3.1 | Brain (fetal) | 0.6 |
| Lung ca.NCI-H460 | 8.4 | Brain(Hippocampus)Pool | 3.0 |
| Lung ca. HOP-62 | 0.9 | Cerebral Cortex Pool | 12.0 |
| Lung ca. NCI-H522 | 1.2 | Brain (Substantia nigra) Pool | 3.9 |
| Liver | 0.0 | Brain (Thalamus) Pool | 25.9 |
| Fetal Liver | 0.0 | Brain (whole) | 8.8 |
| Liver ca. HepG2 | 0.8 | Spinal Cord Pool | 0.8 |
| Kidney Pool | 0.0 | Adrenal Gland | 0.0 |
| Fetal Kidney | 0.7 | Pituitary gland Pool | 1.3 |
| Renal ca. 786-0 | 21.5 | Salivary Gland | 0.0 |
| Renal ca. A498 | 11.8 | Thyroid (female) | 3.1 |
| Renal ca. ACHN | 1.1 | Pancreatic ca. CAPAN2 | 1.2 |
| Renal ca. UO-31 | 2.7 | Pancreas Pool | 6.3 |

**Table BFD. Panel 4D**

| **Tissue Name** | **Rel. Exp.(%) Ag3327, Run 165021004** | **Tissue Name** | **Rel. Exp.(%) Ag3327, Run 165021004** |
|---|---|---|---|
| Secondary Th1 act | 11.3 | HUVEC IL-1beta | 4.0 |
| Secondary Th2 act | 12.5 | HUVEC IFN gamma | 12.3 |
| Secondary Tr1 act | 17.9 | HUVEC TNF alpha + IFN gamma | 2.3 |
| Secondary Th1 rest | 4.4 | HUVEC TNF alpha + IL4 | 1.8 |
| Secondary Th2 rest | 6.0 | HUVEC IL-11 | 6.5 |
| Secondary Tr1 rest | 6.4 | Lung Microvascular EC none | 8.5 |
| Primary Th1 act | 20.6 | Lung Microvascular EC TNFalpha + IL-1beta | 31.6 |
| Primary Th2 act | 13.8 | Microvascular Dermal EC none | 17.9 |
| Primary Tr1 act | 17.1 | Microsvasular Dermal EC TNFalpha + IL-1beta | 29.7 |
| Primary th1 rest | 25.5 | Bronchial epithelium TNFalpha+ IL1beta | 4.2 |
| Primary Th2 rest | 11.7 | Small airway epithelium none | 2.0 |
| Primary Tr1 rest | 4.6 | Small airway epithelium TNFalpha + IL-1beta | 13.2 |
| CD45RA CD4 lymphocyte act | 9.3 | Coronery artery SMC rest | 9.2 |
| CD45RO CD4 lymphocyte act | 12.9 | Coronery artery SMC TNFalpha + IL-1beta | 2.4 |
| CD8 lymphocyte act | 14.9 | Astrocytes rest | 12.5 |
| Secondary CD8 lymphocyte rest | 11.1 | Astrocytes TNFalpha + IL-1beta | 9.9 |
| Secondary CD8 lymphocyte act | 13.2 | KU-812 (Basophil) rest | 7.1 |
| CD4 lymphocyte none | 5.8 | KU-812 (Basophil) PMA/ionomycin | 21.5 |
| 2ry Th 1lTh2/Tr1 anti-CD95 CH11 | 6.3 | CCD1106(Keratmocytes)none | 7.1 |
| LAK cells rest | 12.8 | CCD1106 (Keratinocytes) TNFalpha+IL-1beta | 2.2 |
| LAK cells IL-2 | 0.0 | Liver cirrhosis | 17.7 |
| LAK cells IL-2+IL-12 | 11.1 | Lupus kidney | 19.3 |
| LAK cells IL-2+IFN gamma | 12.2 | NCI-H292 none | 27.7 |
| LAK cells IL-2+ IL-18 | 6.3 | NCI-H292 IL-4 | 14.9 |
| LAK cells PMA/ionomycin | 8.2 | NCI-H292IL-9 | 14.8 |
| NK Cells IL-2 rest | 9.4 | NCI-H292 IL-13 | 2.7 |
| Two Way MLR 3 day | 8.3 | NCI-H292 IFN gamma | 3.0 |
| Two Way MLR 5 day | 4.0 | HPAEC none | 1.4 |
| Two Way MLR 7 day | 11.6 | HPAEC TNF alpha + IL-1 beta | 1.2 |
| PBMC rest | 5.0 | Lung fibroblast none | 7.6 |
| PBMC PWM | 73.2 | Lung fibroblast TNF alpha + IL-1 beta | 9.0 |
| PBMC PHA-L | 16.2 | Lung fibroblast IL-4 | 9.5 |
| Ramos (B cell) none | 22.5 | Lung fibroblast IL-9 | 10.7 |
| Ramos (B cell) ionomycin | 100.0 | Lung fibroblast IL-13 | 8.4 |
| B lymphocytes PWM | 23.8 | Lung fibroblast IFN gamma | 9.5 |
| B lymphocytes CD40L and IL-4 | 11.0 | Dermal fibroblast CCD1070 rest | 17.3 |
| EOL-1 dbcAMP | 2.1 | Dermal fibroblast CCD1070 TNF alpha | 35.8 |
| EOL-1 dbcAW PMA/ionomycin | 2.1 | Dermal fibroblast CCD1070 IL-1 beta | 14.0 |
| Dendritic cells none | 2.9 | Dermal fibroblast IFN gamma | 5.1 |
| Dendritic cells LPS | 6.3 | Dermal fibroblast IL-4 | 8.1 |
| Dendritic cells anti-CD40 | 3.7 | IBD Colitis 2 | 5.3 |
| Monocytesrest | 12.6 | IBD Crohn's | 4.5 |
| Monocytes LPS | 13.5 | Colon | 9.3 |
| Macrophages rest | 5.1 | Lung | 7.7 |
| Macrophages LPS | 9.2 | Thymus | 62.4 |
| HUVEC none | 3.8 | Kidney | 7.4 |
| HUVEC starved | 4.9 | | |

**CNS_neurodegeneration_v1.0 Summary:** Ag3327 This panel confirms the expression of the CG57777-01 gene at low levels in the brain in an independent group of individuals. This gene is found to be down-regulated in the temporal cortex of Alzheimer's disease patients. Therefore, up-regulation of this gene or its protein product, or treatment with specific agonists for this receptor may be of use in reversing the dementia/memory loss associated with this disease and neuronal death.

**General_screening_panel_v1.4 Summary:** Ag3327 The CG57777-01 codes for a homologue of LINE-1 reverse transcriptase. This gene is moderately expressed in samples derived from two of the CNS cancer cell line. Thus, the expression of this gene could be used to distinguish these samples from the other samples in the panel.

**Panel 4D Summary:** Ag3327 The CG57777-01 gene is expressed at low to moderate levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues.

This pattern is in agreement with the expression profile in General_screening_panel_v1.5 and also suggests a role for the gene product in cell survival and proliferation.

Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### NOV64

Expression of NOV64/CG57779-01 was assessed using the primer-probe set Ag3328, described in Table BGA. Results of the RTQ-PCR runs are shown in Tables BGB, BGC and BGD.

**Table BGA. Probe Name Ag3328**

| **Primers** | **Sequences** | **Length** | **Start Postthm** | **SEQ ID NO** |
|---|---|---|---|---|
| Forward | 5'-gatgaagctggaaaccatcat-3' | 21 | 2994 | 443 |
| Probe | TET-5'-tcacaaggacagaaaaccaaacactg-3'-TAMRA | 26 | 3028 | 444 |
| Reverse | 5'-ccacctatgagtgagaacatg-3' | 22 | 3054 | 445 |

**Table BGB. CNS_neurodegeneration_v1.0**

| **Tissue Name** | **ReL Exp.(%) Ag3328, Run 210138311** | **Tissue Name** | **Rel. Exp.(%) Ag3328, Run 210138311** |
|---|---|---|---|
| AD 1 Hippo | 9.4 | Control (Path) 3 Temporal Ctx | 5.8 |
| AD 2 Hippo | 22.7 | Control (Path) 4 Temporal | 30.8 |
| AD 3 Hippo | 6.6 | AD 1 Occipital Ctx | 19.1 |
| AD 4 Hippo | 7.8 | AD 2 Occipital Ctx (Missing) | 0.5 |
| AD 5 hippo | 91.4 | AD 3 Occipital Ctx | 6.1 |
| AD 6 Hippo | 50.3 | AD 4 Occipital Ctx | 14.4 |
| Control 2 Hippo | 17.7 | AD 5 Occipital Ctx | 3.6 |
| Control 4 Hippo | 9.7 | AD 6 Occipital Ctx | 22.5 |
| Control (Path) 3 Hippo | 5.5 | Control 1 Occipital Ctx | 3.6 |
| AD 1 Temporal Ctx | 15.4 | Control 2 Occipital Ctx | 27.2 |
| AD 2 Temporal Ctx | 22.8 | Control 3 Occipital Ctx | 21.2 |
| AD 3 Temporal Ctx | 5.6 | Control 4 Occipital Ctx | 6.9 |
| AD 4 Temporal Ctx | 14.7 | Control (Path) 1 Occipital Ctx | 76.8 |
| AD 5 Inf Temporal Ctx | 100.0 | Control (Path) 2 Occipital Ctx | 9.5 |
| AD 5 SupTemporal Ctx | 68.8 | Control (Path) 3 Occiptal | 26 |
| AD 6 Inf Temporal Ctx | 47.6 | Control (Path) 4 Occipital | 22.2 |
| AD 6 Sup Temporal Ctx | 55.9 | Control Parietal Ctx | 5.0 |
| Control 1 Temporal Ctx | 5.4 | Control 2 Parietal Ctx | 53.2 |
| Control 2 Temporal Ctx | 11.7 | Control 3 Parietal Ctx | 12.9 |
| Control 3 Temporal ax | 10.1 | Control (Path) 1 Parietal Ctx | 49.7 |
| Control 4 Temporal Ctx | 7.3 | Control (Path) 2 Parietal Ctx | 22.5 |
| Control (Path) 1 Temporal Ctx | 42.9 | Control (Path) 3 Parietal Ctx | 7.4 |
| Control (Path) 2 Temporal Ctx | 34.6 | Control (Path) 4 Parietal Ctx | 46.3 |

**Table BGC. General_screening_panel_v1.4**

| **Tissue Name** | **Rel. Exp.(%) Ag3328, Run 215678615** | **Tissue Name** | **Rel.Exp.(%) Ag 3328, Run 215678615** |
|---|---|---|---|
| Adipose | 20.0 | Renal ca. TK-10 | 27.2 |
| Melanoma* Hs688(A).T | 18.9 | Bladder | 39.8 |
| Melanoma* Hs688(B).T | 14.0 | Gastric ca. (liver met) NCI-N87 | 48.0 |
| Melanoma* M14 | 11.6 | Gastric ca.KATO III | 25.5 |
| Melanoma* LOXIMVI | 13.3 | Colon ca. SW-948 | 3.6 |
| Melanoma* SK-MEL-5 | 28.1 | Colon ca. SW480 | 33.9 |
| Squamous cell carcinoma SCC-4 | 11.0 | Colon ca.* (SW480 met) SW620 | 24.1 |
| Testis Pool | 15.7 | Colon ca. HT29 | 12.7 |
| Prostate ca.*(bone met) PC-3 | 24.1 | Colon ca. HCT-116 | 24.5 |
| Prostate Pool | 12.3 | Colon ca. CaCo-2 | 28.7 |
| Placenta | 6.1 | Colon cancer tissue | 20.7 |
| Uterus Pool | 7.3 | Colon ca. SW1116 | 6.7 |
| Ovarian ca. OVCAR-3 | 33.7 | Colon ca. Colo-205 | 3.7 |
| Ovarian ca. SK-OV-3 | 69.3 | Colon ca. SW-48 | 3.5 |
| Ovarian ca. OVCAR-4 | 10.4 | Colon Pool | 41.2 |
| Ovarian ca. OVCAR-5 | 54.0 | Small Intestine Pool | 51.4 |
| Ovarian ca. IGROV-1 | 16.4 | Stomach Pool | 5.2 |
| Ovarian ca. OVCAR-8 | 11.3 | Bone Marrow Pool | 22.7 |
| Ovary | 15.1 | Fetal Heart | 34.2 |
| Breast ca. MCF-7 | 25.3 | Heart Pool | 16.2 |
| Breastca. MDA-MB-231 | 29.1 | Lymph Node Pool | 44.1 |
| Breast ca. BT 549 | 33.0 | Fetal Skeletal Muscle | 26.1 |
| Breast ca. T47D | 44.4 | Skeletal Muscle Pool | 7.7 |
| Breast ca. MDA-N | 17.7 | Spleen Pool | 21.5 |
| Breast Pool | 2.4 | Thymus Pool | 33.2 |
| Trachea | 26.1 | CNS cancer (glio/astro) U87-MG | 23.5 |
| Lung | 27.0 | CNS cancer (glio/astro) U-118-MG | 38.4 |
| Fetal Lung | 100.0 | CNS cancer (neuro;met) SK-N-AS | 27.7 |
| Lung ca. NCI-N417 | 1.4 | CNS cancer (astro) SF-539 | 12.5 |
| Lung ca. LX-1 | 35.4 | CNS cancer (astro) SNB-75 | 59.9 |
| Lung ca. NCI-H146 | 8.8 | CNS cancer (glio) SNB-19 | 14.7 |
| Lung ca. SHP-77 | 27.4 | CNS cancer (glio) SF-295 | 64.2 |
| Lung ca. A549 | 21.8 | Brain (Amygdala) Pool | 13.4 |
| Lungca.NCI-H526 | 5.6 | Brain (cerebellum) | 14.3 |
| Lung ca. NCI-H23 | 39.5 | Brain (fetal) | 28.9 |
| Lung ca. NCI-H460 | 31.6 | Brain (Hippocampus) Pool | 14.6 |
| Lung ca. HOP-62 | 23.2 | Cerebral Cortex Pool | 21.3 |
| Lung ca. NCI-H522 | 42.3 | Brain (substantia nigra) Pool | 14.2 |
| Liver | 0.5 | Brain (Thalamus) Pool | 32.8 |
| Fetal Liver | 13.6 | Brain (whole) | 12.8 |
| Liver ca. HepG2 | 11.2 | Spinal Cord Pool | 17.4 |
| Kidney Pool | 49.3 | Adrenal Gland | 24.1 |
| Fetal Kidney | 54.3 | Pituitary gland Pool | 7.9 |
| Renal ca. 786-0 | 31.9 | Salivary Gland | 5.5 |
| Renal ca. A498 | 5.4 | Thyroid (female) | 3.7 |
| Renal ca. ACHN | 15.1 | Pancreatic ca. CAPAN2 | 33.0 |
| Renal ca. UO-31 | 21.8 | Pancreas Pool | 58.2 |

**Table BGD. Panel 4D**

| **Tissue Name** | **Rel. Exp.(%) Ag3329, Run 165021014** | **Tissue Name** | **Rel. Exp.(%) Ag3328, Run 165021014** |
|---|---|---|---|
| Secondary Th act | 21.0 | HUVEC IL-1beta | 4.7 |
| Secondary Th2 act | 26.4 | HUVEC IFN gamma | 15.0 |
| Secondary Tr1 act | 39.8 | HUVEC TNF alpha + IFN gamma | 13.4 |
| Secondary Th1 rest | 2.8 | HUVEC TNF alpha + IL4 | 0.0 |
| Secondary Th2 rest | 12.6 | HUVEC IL-11 | 12.2 |
| Secondary Tr1 rest | 15.5 | Lung Microvascular EC none | 16.5 |
| Primary Th1 act | 29.1 | Lung Microvascular EC TNFalpha +IL-1beta | 18.6 |
| Primary Th2 act | 25.9 | Microvascular Dermal EC none | 16.2 |
| Primary Tr1 act | 22.8 | Microsvasular Dermal EC TNFalpha + IL-1beta | 14.4 |
| Primary Th1 rest | 36.9 | Bronchial epithelium TNFalpha + IL 1beta | 12.6 |
| Primary Th2 rest | 18.8 | Small airway epithelium none | 4.2 |
| Primary Tr1 rest | 15.3 | Small airway epithelium 7NFalpha+ +IL-1beta | 30.1 |
| CD45RA CD4 lymphocyte act | 11.8 | Coronery artery SMC rest | 6.5 |
| CD45RO CD4 lymphocyte act | 25.0 | Coronery artery SMC TNFalpha + IL-1beta | 3.0 |
| CD8 lymphocyte act | 17.0 | Astrocytes rest | 10.1 |
| Secondary CD8 lymphocyte rest | 19.5 | Astrocytes TNFalpha+IL-1beta | 10.0 |
| Secondary CD8 lymphocyte act | 15.9 | KU-812 (Basophil) rest | 11.0 |
| CD4 lymphocyte none | 11.9 | KU-812 (Basophil) PMA/ionomycin | 31.6 |
| 2ry Th1/Th2/Tr1_anti-CD95 CH11 | 24.0 | CCD1106 (Keratinocytes) none CCD1106 (Kerahnocytes) none | 9.8 |
| LAK cells rest | 21.9 | CCD1106 (Keratinocytes) TNFalpha + IL-1beta | 6.7 |
| LAK cells IL-2 | 21.3 | Liver cirrhosis | 26.1 |
| LAK cells IL-2+IL-12 | 13.5 | Lupus kidney | 7.8 |
| LAK cells IL-2+IFN gamma | 26.8 | NCI-H292 none | 29.9 |
| LAK cells IL-2+IL-18 | 18.9 | NCI-H292 IL-4 | 24.5 |
| LAK cells PMA/ionomycin | 11.0 | NCI-H292 IL-9 | 22.1 |
| NK Cells IL-2 rest | 18.4 | NCI-H292 IL-13 | 13.1 |
| Two Way MLR 3 day | 23.2 | NCI-H292 IFN gamma | 18.7 |
| Two Way MLR 5 day | 8.8 | HPAEC none | 8.7 |
| Two Way MLR 7 day | 10.8 | HPAEC TNFalpha +IL-1beta | 18.7 |
| PBMC rest | 9.9 | Lung fibroblast none | 9.6 |
| PBMC PWM | 48.3 | Lung fibroblast TNF alpha+IL-1 beta beta | 6.3 |
| PBMC PHA-L | 16.7 | Lung fibroblast IL-4 | 20.3 |
| Ramos (B cell) none | 19.3 | Lung fibroblast IL-9 | 13.8 |
| Ramos (B cell) ionomycin | 43.8 | Lung fibroblast IL-13 | 14.9 |
| B lymphocytes PWM | 19.3 | Lung fibroblast IFN gamma | 15.7 |
| B lymphocytes CD40L and IL-4 | 22.7 | Dermal fibroblast CCD1070 rest | 24.0 |
| EOL-1 dbcAMP | 12.2 | Dermal fibroblast CCD1070 TNF alpha | 50.7 |
| EOL-1 dbcAMP PMA/ionomycin | 15.2 | Dermal fibroblast CCD 1070 IL-1 beta | 11.4 |
| Dendritic cells none | 10.5 | Dermal fibroblast IFN gamma | 12.5 |
| Dendritic cells LPS | 12.5 | Dermal fibroblast IL-4 | 21.8 |
| Dendritic cells anti-CD40 | 10.8 | IBD Colitis 2 | 8.3 |
| Monocytes rest | 19.9 | IBD Crohn's | 7.2 |
| Monocytes LPS | 13.9 | Colon | 30.4 |
| Macrophages rest | 20.4 | Lung | 17.0 |
| Macrophages LPS | 7.9 | Thymus | 84.7 |
| HUVEC none | 8.2 | Kidney | 100.0 |
| HUVEC starved | 10.0 | | |

**CNS_neurodegeneration_v1.0 Summary:** Ag3328 This panel confirms the expression of CG57779-01 gene at low levels in the brains of an independent group of individuals. However, no differential expression of this gene was detected between Alzheimer's diseased postmortem brains and those of non-demented controls in this experiment.

**General_screening_panel_v1.4 Summary:** Ag3328 The CG57779-01 gene encodes a homolog of LINE-1 reverse transcriptase. Its expression is moderate to high across all of the samples on this panel. Therefore, this gene may be playing an important role in cellular function

**Panel 4D Summary:** Ag3328 The CG57779-01 gene is expressed at high to moderate levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues.

This pattern is in agreement with the expression profile in General_screening_panel_v1.4 and also suggests a role for the gene product in cell survival and proliferation.

Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### NOV65

Expression of NOV65/CG57781-01 was assessed using the primer-probe set Ag3329, described in Table BHA. Results of the RTQ-PCR runs are shown in Tables BHB, BHC and BHD.

**Table BHA. Probe Name Ag3329**

| **Primers** | **Sequences** | **Length** | **Start Position** | **SEQ ID NO** |
|---|---|---|---|---|
| Forward | 5'-tcacaaggacagaaaaccaaac-3' | 22 | 2944 | 446 |
| Probe | TET-5'-ccacatgttctcactcataggtggga-3-TAMRA | 26 | 2967 | 447 |
| Reverse | 5'-gtgtccatgtgttctcgttgtt-3' | 22 | 2997 | 448 |

**Table BHB. CNS_neurodegeneration_v1.0**

| **Tissue Name** | **Rel. Exp.(%) Ag3329, Run 210146230** | **Tissue Name** | **Rel. Exp.(%) Ag3319, Run 210146230** |
|---|---|---|---|
| AD 1 Hippo | 9.2 | Control (Path) 3 Temporal Ctx | 6.4 |
| AD 2 Hippo | 19.9 | Control (Path) 4 Temporal Ctx | 35.4 |
| AD 3 Hippo | 10.0 | AD 1 Occipital Ctx | 20.3 |
| AD 4 Hippo | 10.0 | AD 2 Occipital Ctx (Missing) | 0.1 |
| AD 5 Hippo | 64.6 | AD 3 Occipital Ctx | 6.4 |
| AD 6 Hippo | 42.9 | AD 4 Occipital Ctx | 18.6 |
| Control 2 Hippo | 20.4 | AD 5 Occipital Ctx | 16.0 |
| Control 4 Hippo | 10.7 | AD6 Occipital Ctx | 18.0 |
| Control (Path) 3 Hippo | 5.5 | Control Occipital Ctx | 3.1 |
| AD 1 Temporal Ctx | 20.4 | Control 2 Occipital Ctx | 28.7 |
| AD 2 Temporal Ctx | 23.7 | Control 3 Occipital Ctx | 24.1 |
| AD 3 Temporal Ctx | 8.7 | Control 4 Occipital Ctx | 6.0 |
| AD 4 Temporal Ctx | 19.9 | Control (Path) Occipital Ctx | 75.3 |
| AD 5 Inf Temporal Ctx | 100.0 | Control (Path) 2 Occipital Ctx | 13.2 |
| AD 5 Sup Temporal Ctx | 65.5 | Control (Path) 3 Occipital Ctx | 3.0 |
| AD 6 Inf Temporal Ctx | 46.3 | Control (Path) 4 Occipital | 0.0 |
| AD 6 Sup Temporal Ctx | 62.0 | Control 1 Parietal Ctx | 5.4 |
| Control 1 Temporal Ctx | 5.7 | Control 2 Parietal Ctx | 43.8 |
| Control 2 Temporal Ctx | 15.7 | Control 3 Parietal Ctx | 11.8 |
| Control 3 Temporal Ctx | 16.6 | Control (Path) 1 Parietal Ctx | 47.0 |
| Control 3 Temporal Ctx | 10.3 | Control (Path) 2 Parietal Ctx | 23.0 |
| Control (Path) 1 Temporal Ctx | 50.0 | Control (Path) 3 Parietal Ctx | 5.9 |
| Control (Path) 2 Temporal Ctx | 44.1 | Control (Path) 4 Parietal Ctx | 39.5 |

**Table BHC. General_screening_panel_v1.4**

| **Tissue Name** | **Rel. Exp.(%) Ag3329, Run 215678554** | **Tissue Name** | **Rel. Exp.(%) Ag3329, Run 215678554** |
|---|---|---|---|
| Adipose | 18.4 | Renal ca. TK-10 | 31.0 |
| Melanoma* Hs688(A)T | 15.3 | Bladder | 48.0 |
| Melanoma* Hs688(B).T | 13.7 | Gastric ca. (liver met.) NCI-N87 | 38.4 |
| Melanoma* M14 | 21.3 | Gastric ca. KATO III | 22.2 |
| Melanoma* LOXIMVI | 15.8 | Colon ca. SW-948 | 4.3 |
| Melanoma* SK-MEL-5 | 39.0 | Colon ca. SW480 | 38.4 |
| Squamous cell carcinoma SCC-4 | 17.9 | Colon ca.* (SW480 met) SW620 | 21.9 |
| Testis Pool | 10.4 | Coton ca. HT29 | 15.7 |
| Prostate ca.* (bone met) PC-3 | 31.0 | Colon ca. HCT-116 | 21.3 |
| Prostate Pool | 18.8 | Colon ca. CaCo-2 | 27.5 |
| Placenta | 4.4 | Colon cancer tissue | 17.1 |
| Uterus Pool | 12.6 | Colon ca. SW1116 | 5.2 |
| Ovarian ca.OVCAR-3 | 22.7 | Colon ca. Colo-205 | 3.8 |
| Ovarian ca. SK-OV-3 | 34.9 | Colon ca. SW-48 | 3.3 |
| Ovarian ca. OVCAR-4 | 5.6 | Colon Pool | 79.0 |
| Ovarian ca. OVCAR-5 | 44.8 | Small Intestine Pool | 59.9 |
| Ovarian ca. IGROV-1 | 15.8 | Stomach Pool | 48.3 |
| Ovarian ca. OVCAR-8 | 11.7 | Bone Marrow Pool | 39.8 |
| Ovary | 19.2 | Fetal Heart | 67.8 |
| Breast ca. MCF-7 | 26.4 | Heart Pool | 22.8 |
| Breast ca. MDA-MB-231 | 25.0 | Lymph Node Pool | 91.4 |
| Breast ca. BT 549 | 48.3 | Fetal Skeletal Muscle | 30.1 |
| Breast ca. T47D | 49.3 | Skeletal Muscle Pool | 29.7 |
| Breast ca. MDA-N | 16.3 | Spleen Pool | 28.7 |
| Breast Pool | 76.3 | Thymus Pool | 59.9 |
| Trachea | 32.5 | CNS cancer (glio/astro) U87-MG | 32.5 |
| Lung | 26.2 | CNS cancer (glio/astro) U-118-MG | 40.3 |
| Fetal Lung | 89.5 | CNS cancer (neuro;met) SK- N-AS | 35.4 |
| Lung ca. NCI-N417 | 22.7 | CNS cancer (astro) SF-539 | 11.0 |
| Lung ca. LX-1 | 28.3 | CNS cancer (astro) SNB-75 | 54.3 |
| Lung ca. NCI-H146 | 10.6 | CNS cancer (glio) SNB-19 | 9.9 |
| Lung ca. SHP-77 | 26.8 | CNS cancer (glio) SP-295 | 81.2 |
| Lung ca. A549 | 23.2 | Brain (Amygdala) Pool | 19.3 |
| Lung ca. NCI-H526 | 4.7 | Brain (cerebellum) | 21.0 |
| Lung ca. NCI-H23 | 54.3 | Brain (fetal) | 47.0 |
| Lung ca. NCI-H460 | 46.0 | Brain (Hippocampus) Pool | 20.0 |
| Lung ca. HOP-62 | 18.2 | Cerebral Cortex Pool | 21.6 |
| Lung ca NCI-H522 | 35.4 | Brain (Substantia nigra) Pool | 14.5 |
| Liver | 0.5 | Brain (Thalamus) Pool | 35.6 |
| Fetal Liver | 17.1 | Brain (whole) | 18.4 |
| Liver ca. HepG2 | 19.6 | Spinal Cord Pool | 20.6 |
| Kidney Pool | 100.0 | Adrenal Gland | 210 |
| Fetal Kidney. | 98.6 | Pituitary gland Pool | 11.1 |
| Renal Ca: 786-0 | 27.5 | Salivary Gland | 5.4 |
| Renal ca A498 | 9.9 | Thyroid (female) | 4.4 |
| Renal ca. ACHN | 23.3 | Pancreatic ca. CAPAN2 | 39.5 |
| Renal ca. UO-31 | 31.9 | Pancreas Pool | 56.6 |

**Table BHD. Panel 4D**

| **Tissue Name** | **Rel. Exp.(%) Ag3329, Run 165021015** | **Tissue Name** | **Rel. Exp.(%) Ag3329, Run 165021015** |
|---|---|---|---|
| Secondary Th1 act | 27.4 | HUVEC IL-1beta | 10.4 |
| Secondary Th2 act | 0.0 | HUVEC IFN gamma | 0.0 |
| Secondary Tr1 act | 25.0 | HUVEC TNF alpha + IFN gamma | 6.6 |
| Secondary Th1 rest | 7.5 | HUVEC TNF alpha + IL4 | 5.8 |
| Secondary Th2 rest | 10.2 | HUVEC IL-11 | 5.4 |
| Secondary Tr1 rest | 12.7 | Lung Microvascular EC none | 7.9 |
| Primary Th1 act | 24.0 | Lung Microvascular EC TNFalpha + IL-1beta | 9.9 |
| Primary Th2 act | 21.6 | Microvascular Dermal EC none | 9.5 |
| Primary Tr1 act | 27.9 | Microsvasular Dermal EC TNFalpha + IL-1beta | 8.5 |
| Primary Th1 rest | 39.0 | Bronchial epithelium TNFalpha+ IL1beta | 8.1 |
| Primary Th2 rest | 17.8 | Small airway epithelium none | 2.1 |
| Primary Tr1 rest | 20.7 | Small airway epithelium TNFalpha +IL-1beta | 16.5 |
| CD45RA CD4 lymphocyte act | 10.7 | Coronery artery SMC rest | 5.4 |
| CD45RO CD4 lymphocyte act | 21.0 | Coronery artery SMC TNFalpha + IL-1beta | 2.7 |
| CD8 lymphocyte act | 11.3 | Astrocytes rest | 7.4 |
| Secondary CD8 lymphocyte rest | 11.8 | Astrocytes TNFalpha + IL-1beta | 6.7 |
| Secondary CD8 lymphocyte act | 9.9 | KU-812 (Basophil) rest | 9.4 |
| CD4 lymphocyte none | 11.5 | KU-812 (Basophil) PMA/ionomycin | 49.7 |
| 2ry Th1/Th2/Tr1_anti-CD95 CH11 | 26.4 | CCD1106 (Keratinocytes) none | 6.7 |
| LAK cells rest | 24.5 | CCD1106 (Keratinocytes) TNFalpha + IL-1beta | 4.0 |
| LAK cells IL-2 | 22.4 | Liver cirrhosis | 36.6 |
| LAK cells IL-2+IL-12 | 10.5 | Lupus kidney | 11.3 |
| LAK cells IL-2+IFNgamma | 14.1 | NCI-H292 none | 27.9 |
| LAK cells IL-2+ IL-18 | 15.3 | NCI-H292 IL-4 | 20.2 |
| LAK cells PMA/ionomycin | 15.4 | NCI-H292 IL-9 | 26.2 |
| NK Cells IL-2 rest | 17.7 | NCI-H292 IL-13 | 17.8 |
| Two Way MLR 3 day | 15.6 | NCI-H292 IFN gamma | 13.3 |
| Two Way MLR 5 day | 7.8 | HPAEC none | 6.5 |
| Two Way MLR 7 day | 7.5 | HPAEC TNF alpha+IL-1 beta | 12.6 |
| PBMC rest | 3.9 | Lung fibroblast none | 6.3 |
| PBMC PWM | 57.8 | Lung fibroblast TNF alpha+ IL-1 beta | 3.8 |
| PBMC PHA-L | 19.3 | Lung fibroblast IL-4 | 11.2 |
| Ramos (B cell) none | 20.3 | Lung fibroblast IL-9 | 9.4 |
| Ramos (B cell) ionomycin | 42.9 | Lung fibroblast IL-13 | 15.9 |
| B lymphocytes PWM | 19.6 | Lung fibroblast IFN gamma | 12.9 |
| B lymphocytes CD40L and IL-4 | 27.5 | Dermal fibroblast CCD1070 rest | 20.3 |
| EOL-1 dbcAMP | 3.7 | Dermal fibroblast CCD1070 TNF alpha | 73.7 |
| EOL-1 dbcAMP PMA/ionomycin | 15.0 | Dermal fibroblast CCD1070 IL-1 beta | 11.6 |
| Dendritic cells none | 10.4 | Dermal fibroblast IFN gamma | 11.2 |
| Dendritic cells LPS | 10.1 | Dermal fibroblast IL-4 | 20.3 |
| Dendritic cells anti-CD40 | 10.4 | IBD Colitis 2 | 8.6 |
| Monocytes rest | 15.2 | IBD Crohn's | 5.5 |
| Monocytes LPS | 20.9 | Colon | 33.4 |
| Macrophages rest | 25.2 | Lung | 18.3 |
| Macrophages LPS | 7.1 | Thymus | 87.7 |
| HUVEC none | 5.4 | Kidney | 100.0 |
| HUVEC starved | 11.7 | | |

**CNS_neurodegeneration_v1.0 Summary:** Ag3329 The CG57781-01 encodes a gene that is homologous to LINE-1 reverse transcriptase. This panel confirms the expression of this gene at low levels in the brains of an independent group of individuals. However, no differential expression of this gene was detected between Alzheimer's diseased postmortem brains and those of non-demented controls in this experiment.

**General_sereening_panel_v1.4 Summary:** Ag3329 The CG57781-01 encodes a gene that is homologous to LINE-1 reverse transcriptase. Its expression is moderate to high across all of the samples on this panel. Interestingly, this gene is expressed at much higher levels in fetal (CT = 25.44) when compared to adult liver(CT = 30.53). This observation suggests that expression of this gene can be used to distinguish fetal from adult liver.

**Panel 4D Summary:** Ag3329 This gene is expressed at high to moderate levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues.

This pattern is in agreement with the expression profile in General_screening_panel_v1.4 and also suggests a role for the gene product in cell survival and proliferation.

Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### NOV66

Expression of NOV66/CG57783-Ol was assessed using the primer-probe set Ag3330, described in Table BIA. Results of the RTQ-PCR runs are shown in Tables BIB, BIC, and BID.

**Table BIA. Probe Name Ag3330**

| **Primers** | **Sequences** | **Length** | **Start Position** | **Seq id no:** |
|---|---|---|---|---|
| Forward | 5'-gccatcccattactgggtatat-3' | 22 | 2773 | 449 |
| Probe | TET-5'-tcatgctgctataaagacacatgcag-3'-TAMRA | 26 | 2812 | 450 |
| Reverse | 5'-tagtgccgcaataaacatacgt-3' | 22 | 2838 | 451 |

**Table BIB. CNS_neurodegeneration_v1.0**

| **Tissue Name** | **Rel. Exp.(%) Ag3330, Run 210146231** | **Tissue Name** | **Rel. Exp.(%) Ag3330, Run 210146231** |
|---|---|---|---|
| AD 1 Hippo | 6.8 | Control (Path) 3 Temporal Ctx | 4.5 |
| AD 2 Hippo | 18.3 | Control (Path) 4 Temporal Ctx | 26.1 |
| AD 3 Hippo | 5.6 | AD 1 Occipital Ctx | 13.2 |
| AD 4 Hippo | 5.2 | AD 2 Occipital Ctx (Missing) | 0.2 |
| AD 5 hippo | 66.4 | AD 3 Occipital Ctx | 4.2 |
| AD 6 Hippo | 42.0 | AD 4 Occipital Ctx | 12.7 |
| Control 2 Hippo | 19.2 | AD 5 Occipital Ctx | 17.1 |
| Control 4 Hippo | 7.2 | AD 6 Occipital Ctx | 20.9 |
| Control (Path) 3 Hippo | 4.0 | Control 1 Occipital Ctx | 1.8 |
| AD 1 Temporal Ctx | 14.2 | Control 2 Occipital Ctx | 29.5 |
| AD 2 Temporal Ctx | 23.0 | control 3 Occipital Ctx | 12.7 |
| AD 3 Temporal Ctx | 5.1 | Control 4 Occipital Ctx | 4.6 |
| AD 4 Temporal Ctx | 17.2 | Control (Path) 1 Occipital | 70.7 |
| AD 5 Inf Temporal Ctx | 100.0 | Control (Path) 2 Occipital | 11.7 |
| AD 5 SupTemporal Ctx | 50.7 | Control (Path) 3 Occipital Ctx | 2.0 |
| AD 6 Inf Temporal Ctx | 51.1 | Control (Path) 4 Occipital Ctx | 13.1 |
| AD 6 Sup Temporal Ctx | 52.9 | Control 1 Parietal Ctx | 5.8 |
| Control 1 Temporal Ctx | 4.2 | Control 2 Parietal Ctx | 49.3 |
| Control 2 Temporal Ctx | 13.4 | Control 3 Parietal Ctx | 11.8 |
| Control 3 Temporal Ctx | 10.2 | Control (Path) 1 Parietal Ctx | 56.6 |
| Control 4 Temporal Ctx | 5.7 | Control (path) 2 Parietal Ctx | 18.9 |
| Control (Path) 1 Temporal Ctx | 44.1 | Control (Path) 3 Parietal Ctx | 3.1 |
| Control (Path) 2 Temporal Ctx | 41.2 | Control (Path) 4 Parietal Ctx | 34.9 |

**Table BIC. General_scmening_panel_v1.4**

| **Tissue Name** | **Rel. Exp.(%) Ag3330, Run 215678685** | **Tissue Name** | **Rel. Exp.(%) Ag3330, Run 215678685** |
|---|---|---|---|
| Adipose | 27.4 | Renal ca. TK-10 | 32.8 |
| Melanoma* Hs688(A).T | 29.1 | Bladder | 54.3 |
| Melanoma* Hs688(B).T | 23.7 | Gastric ca. (liver met.) NCI-N87 | 51.1 |
| Melanoma* M14 | 20.7 | Gastric ca. KATO III | 31.0 |
| Melanoma* LOXIMVI | 18.2 | Colon ca. SW-948 | 5.5 |
| Melanoma* SK-MEL-5 | 44.1 | Colon ca. SW480 | 39.5 |
| Squamous cell carcinoma SCC-4 | 17.2 | Colon ca.* (SW480 met) SW620 | 29.7 |
| Testis Pool | 24.0 | Colon ca. H129 | 15.3 |
| Prostate ca.* (bone met) PC-3 | 41.8 | Colon ca.HCT-116 | 41.8 |
| Prostate Pool | 23.0 | Colon ca.CaCo-2 | 44.1 |
| Placenta | 6.7 | Colon cancer tissue | 27.4 |
| Uterus Pool | 13.7 | Colon ca. SW1116 | 7.6 |
| Ovarian ca. OVCAR-3 | 39.0 | Colon ca. Colo-205 | 5.3 |
| Ovarian ca. SK-OV-3 | 46.3 | Colon ca. SW-48 | 3.9 |
| Ovarian ca. OVCAR-4 | 9.2 | Colon Pool | 68.3 |
| Ovarian ca. OVCAR-5 | 63.7 | Small Intestine Pool, | 73.7 |
| Ovarian ca. IGROV-1 | 26.4 | Stomach Pool | 42.0 |
| Ovarian ca. OVCAR-8 | 12.4 | Bone Marrow Pool | 34.6 |
| Ovary | 24.0 | Fetal Head | 70.2 |
| Breast ca. MCF-7 | 39.5 | Heart Pool | 33.9 |
| Breast ca. MDA-MB-231 | 33.0 | Lymph Node Pool | 64.6 |
| Breast ca. BT 549 | 49.7 | Fetal Skeletal Muscle | 31.6 |
| Breast ca. T47D | 58.2 | Skeletal Muscle Pool | 30.8 |
| Breast ca. MDA-N | 24.5 | Spleen Pool | 26.4 |
| Breast Pool | 56.3 | Thymus Pool | 44.8 |
| Trachea | 32.5 | CNS cancer (glio/astro) U87-MG | 44.4 |
| Lung | 43.5 | CNS cancer (glio/astro) U-118-MG | 58.2 |
| Fetal Lung | 100.0 | CNS cancer (neuro;met) SK-N-AS | 45.1 |
| Lung ca.NCI-N417 | 11.8 | CNS cancer (astro) SF-539 | 16.7 |
| Lung ca. LX-1 | 42.6 | CNS caneer (astro) SNB-75 | 59.9 |
| Lung ca. NCI-H146 | 0.0 | CNS cancer (glio) SNB-19 | 21.0 |
| Lung ca. SHP-77 | 44.8 | CNS cancer (glio) SF-295 | 71.2 |
| Lung ca. A549 | 27.5 | Brain (Amygdala) Pool | 19.5 |
| Lung ca. NCI-H526 | 7.2 | Brain (cerebellum) | 17.2 |
| Lung ca. NCI-H23 | 57.4 | Brain (fetal) | 52.1 |
| Lung ca. NCI-H460 | 50.3 | Brain (Hippocampus) Pool | 28.3 |
| Lung ca. HOP-62 | 27.7 | Cerebral Cortex Pool | 29.5 |
| Lung ca. NCI-H522 | 51.8 | Brain (Substantia nigra) Pool | 18.3 |
| Liver | 0.9 | Brain (Thalamus) Pool | 43.5 |
| Fetal Liver | 24.8 | Brain (whole) | 23.8 |
| Liver ca. HepG2 | 17.7 | Spinal Cord Pool | 22.1 |
| Kidney Pool | 95.9 | Adrenal Gland | 23.8 |
| Fetal Kidney | 94.0 | Pituitary gland Pool | 11.6 |
| Renal ca. 786-0 | 31.6 | Salivary Gland | 7.9 |
| Renal ca. A498 | 15.5 | Thyroid (female) | 6.2 |
| Renal ca. ACHN | 28.5 | Pancreatic ca. CAPAN2 | 52.9 |
| Renal ca. UO-31 | 29.3 | Pancreas Pool | 65.1 |

**Table BID. Panel 4D**

| **Tissue Name** | **Rel. Exp.(%) Ag3330,Run 165725930** | **Tissue Name** | **Rel. Exp.(%) Ag3330, Run 165725930** |
|---|---|---|---|
| Secondary Th1 act | 20.2 | HUVEC IL-1beta | 20.4 |
| Secondary Th2 act | 27.0 | HUVEC IFN gamma | 23.0 |
| Secondary Tr1 act | 41.8 | HUVEC TNF alpha + IFN gamma | 14.7 |
| Secondary Th1 rest | 33.4 | HUVEC TNF alpha + IL4 | 13.7 |
| Secondary Th2 rest | 15.8 | HUVEC IL-11 | 13.0 |
| Secondary Tr1 rest | 17.0 | Lung Mierovascular EC none | 18.3 |
| Primary Th1 act | 14.1 | Lung Microvascular EC TNFalpha +IL-1beta | 13.8 |
| Primary Th2 act | 33.2 | Microvascular Dermal EC none | 15.7 |
| Primary Tr1 act | 37.6 | Microsvasular Dermal EC TNFalpha+IL-1beta | 37.9 |
| Primary Th1 rest | 100.0 | Bronchial epithelium TNFalpha + IL1beta | 8.5 |
| Primary Th2 rest | 37.4 | Small airway epithelium none | 4.9 |
| Primary Tr1 rest | 34.9 | Small airway epithelium TNFalpha + IL-1 beta | 41.5 |
| CD45RA CD4 lymphocyte act | 16.0 | Coronery artery SMC rest | 11.7 |
| CD45RO CD4 lymphocyte act | 28.9 | Coronery artery SMC TNFalpha + IL-1beta | 4.5 |
| CD8 lymphocyte act | 25.5 | Astrocytes rest | 33.9 |
| Secondary CD8 lymphocyte rest | 29.3 | Astrocytes TNFalpha + IL-1beta | 32.1 |
| Secondary CD8 lymphocyte act | 22.7 | KU-812 (Basophil) rest | 17.1 |
| CD4 lymphocyte none | 21.2 | KU-812 (Basophil) PMA/ionomycin | 44.8 |
| 2ryTh1/Th2/Tr1_anti-CD95 CH1 | 33.2 | CCD1106(Keratinocytes)none | 13.8 |
| LAK cells rest | 16.5 | CCD1106 (Keratinocytes) TNFalpha + IL-1 beta | 55.9 |
| LAK cells IL-2 | 48.6 | Liver cirrhosis | 76.8 |
| LAK cells IL.2+IL-12 | 33.2 | Lupus kidney | 67.8 |
| LAK cells IL-2+IFN gamma | 42.6 | NCI-H292 none | 39.2 |
| LAK cells IL-2+ IL-18 | 30.8 | NCI-H292 IL-4 | 57.4 |
| LAK cells PMA/ionomycin | 23.2 | NCI-H292 IL-9 | 53.2 |
| NK Cells IL-2 rest | 20.7 | NCI-H292 IL-13 | 23.2 |
| Two Way MLR 3 day | 41.8. | NCI-H292 IFN gamma | 22.8 |
| Two Way MLR 5 day | 16.0 | HPAEC none | 11.9 |
| Two Way MLR 7 day | 26.6 | HPAEC TNF alpha+IL-1 beta | 26.1 |
| PBMC rest | 16.2 | Lung fibroblast none | 19.6 |
| PBMC PWM | 33.0 | Lung fibroblast TNF alpha + IL-1 beta | 19.8 |
| PBMC PHA-L | 38.2 | Lung fibroblast IL-4 | 13.6 |
| Ramos (B cell) none | 42.6 | Lung fibroblast IL-9 | 15.5 |
| Ramos (B cell) ionomycin | 25.5 | Lung fibroblast IL-13 | 14.5 |
| B lymphocytes PWM | 32.1 | Lung fibroblast IFN gamma | 25.5 |
| B lymphocytes CD40L and IL-4 | 36.9 | Dermal fibroblast CCD1070 rest | 32.1 |
| EOL-1 dbeAW | 20.2 | Dermal fibroblast CCD1070 TNF alpha | 62.4 |
| EOL-1 dbcAMP PMA/ionomycin | 65.1 | Dermal fibroblast CCD1070 IL-1 beta | 12.0 |
| Dendritic cells none | 17.4 | Dermal fibroblast IFN gamma | 7.2 |
| Dendritic cells LPS | 20.9 | Dermal fibroblast IL-4 | 22.7 |
| Dendritic cells anti-CD40 | 22.2 | IBD Colitis 2 | 12.0 |
| Monocytes rest | 33.0 | IBD Crohn's | 11.4 |
| Monocytes LPS | 30.1 | Colon | 95.9 |
| Macrophages rest | 19.3 | Lung | 18.3 |
| Macrophages LPS | 14.8 | Thymus | 73.2 |
| HUVEC none | 19.5 | Kidney | 67.4 |
| HUVEC starved | 35.4 | | |

**AI_comprehensive panel_v1.0 Summary:** Ag3330 Expression of the CG57783-01 gene is low/undetectable (CTs > 35) across all of the samples on this panel (data not shown).

CNS_neurodegeneration_v1.0 Summary: Ag3330 This panel confirms the expression of the CG57783-01 gene at low levels in the brains of an independent group of individuals. However, no differential expression of this gene was detected between Alzheimer's diseased postmortem brains and those of non-demented controls in this experiment.

**Generat_screening_panel_v1.4 Summary:** Ag3330 The CG57783-01 gene encodes a protein with homology to LINE-1 reverse transcriptase. Its expression is moderate to high across all of the samples on this panel. Therefore, this gene may be playing an important role in cellular function.

**Panel 4D Summary:** Ag3330 The CG57783-01 gene is expressed at high to moderate levels in a wide range of cell types of significance in the immune response in health and disease. These cells include members of the T-cell, B-cell, endothelial cell, macrophage/monocyte, and peripheral blood mononuclear cell family, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues.

This pattern is in agreement with the expression profile in General_screening_panel_v1.4 and also suggests a role for the gene product in cell survival and proliferation.

Therefore, modulation of the gene product with a functional therapeutic may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

### NOV67

Expression of NOV67A/CG57823-01 and NOV67B/CG57823-02 was assessed using the primer-probe sets Ag3514 and Ag4117, described in Tables BJA and BJB. Results of the RTQ-PCR runs are shown in Tables BJC, BJD, and BJE. Please note that Ag3514 only recognizes the CG57823-01 variant. In addition, CG57823-02 represents a full-length physical clone of the CG57823-01 gene, validating the prediction of the gene sequence.

**Table BJA. Probe Name Ag3514**

| **Primers** | **Sequences** | **Length** | **Start Position** | **SEQ ID NO** |
|---|---|---|---|---|
| Forward | 5'-cctcgtctcgagacaagga-3' | 19 | 253 | 452 |
| Probe | TET-5'-accatcctcgacacactccgggag-3'-TAMRA | 24 | 274 | 453 |
| Reverse | 5'-cggtccttagtgggtttgac-3' | 20 | 319 | 454 |

**Table BJB. Probe Name Ag4117**

| **Primers** | **Sequences** | **Length** | **Start Position** | **SEQ ED NO** |
|---|---|---|---|---|
| Forward | 5'-ccggtatacaccaatgatctgt-3' | 20 | 342 | 455 |
| Probe | TET-5'-cgaacatgcttgctgt-3'-TAMRA | 23 | 383 | 456 |
| Reverse | 5'-tgacgactttccacaccaa-3' | 19 | 406 | 457 |

**Table BJC. CNS_neurodegeneration_v1.0**

| **Tissue Name** | **Rel. Exp.(%) Ag3514, Run 210499746** | **Rel. Exp.(%) Ag4117, Run 206943850** | **Tissue Name** | **Rel. Exp.(%) Ag3514, Run 210499746** | **Rel. Exp.(%) Ag4117, Run 206943850** |
|---|---|---|---|---|---|
| AD 1 Hippo | 35.1 | 65.5 | Control (Path) 3 Temporal Ctx | 0.0 | 42.6 |
| AD 2 Hippo | 13.7 | 70.2 | Control (path) 4 Temporal Ctx | 28.3 | 37.1 |
| AD 3 Hippo | 0.0 | 27.9 | AD 1 Occipital Ctx | 0.0 | 81.2 |
| AD 4 Hippo | 0.0 | 75.3 | AD 2 Occipital Ctx (Missing) | 0.0 | 73.2 |
| AD 5 Hippo | 80.1 | 65.1 | AD 3 Occipital Ctx | 0.0 | 71.2 |
| AD 6 Hippo | 0.0 | 67.4 | AD 4 Occipital Ctx | 75.3 | 50.0 |
| Control 2 Hippo | 0.0 | 76.8 | AD 5 Occipital Ctx | 12.9 | 66.4 |
| Control 4 Hippo | 0.0 | 65.5 | AD 6 Occipital Ctx | 0.0 | 45.4 |
| Control (Path) ³ Hippo | 0.0 | 48.3 | Control 1 Occipital Ctx | 15.1 | 74.2 |
| AD 1 Temporal Ctx | 72.2 | 49.3 | Control 2 Occipital Ctx | 0.0 | 69.3 |
| AD 2 Temporal Ctx | 0.0 | 83.5 | Control 3 Occipital Ctx | 0.0 | 38.7 |
| AD 3 Temporal Ctx | 0.0 | 46.7 | Control 4 Occipital Ctx | 41.8 | 83.5 |
| AD 4 Temporal Ctx | 0.0 | 31.6 | Control (Path) 1 Occipital Ctx | 14.0 | 72.7 |
| AD 5 Inf Temporal Ctx | 40.9 | 64.6 | Control (Path) 2 Occipital Ctx | 23.8 | 79.0 |
| AD 5 Sup Temporal Ctx | 0.0 | 23.5 | Control (Path) 3 Occipital Ctx | 0.0 | 73.2 |
| AD 6 Inf Temporal Ctx | 0.0 | 90.1 | Control Path) 4 Occipital Ctx | 33.2 | 13.8 |
| AD 6 Sup Temporal Ctx | 0.0 | 51.4 | Control 1 Parietal Ctx | 0.0 | 39.5 |
| Control 1 Temporal Ctx | 13.8 | 63.7 | Control 2 Parietal Ctx | 0.0 | 64.2 |
| Control 2 Temporal Ctx Temporal Ctx | 0.0 | 73.7 | Control 3 Parietal Ctx | 0.0 | 51.1 |
| Control 3 Temporal Ctx | 100.0 | 29.7 | Control (Path) 1 Parietal Ctx | 50.3 | 16.8 |
| Control 3 Temporal Ctx | 0.0 | 84.7 | Control (Path) 2 Parietal Ctx | 0.0 | 25.7 |
| Control (Path) Temporal Ctx | 1 0.0 | 51.1 | Control (Path) 3 Parietal Ctx | 0.0 | 100.0 |
| Control (Path) 2 Temporal Ctx | 26.2 | 41.2 | Control (Path) 4 Parietal Ctx | 0.0 | 37.1 |

**Table BJD.General_screeming_panel_v1.4**

| **Tissue Name** | **Rel. Exp.(%) Ag3514, Run 216607683** | **Rel. Exp.(%) Ag3S14, Run 222691301** | **Tissue Name** | **Rel. Exp.(%) Ag3514, Run 216607683** | **Rel. Exp.(%) Ag3514, Run 222691301** |
|---|---|---|---|---|---|
| Adipose | 0.0 | 0.0 | Renal ca. TK-10 | 0.0 | 0.0 |
| Melanoma* Hs688(A).T | 0.0 | 0.0 | Bladder | 35.6 | 0.0 |
| Melanoma* Hs688(B).T | 0.0 | 0.0 | Gastric ca. (livermet.) NCI-N87 | 0.0 | 0.0 |
| Melanoma* M14 | 0.0 | 0.0 | Gastric ca. KATO III | 0.0 | 0.0 |
| Melanoma* LOXIMVI | 0.0 | 0.0 | Colon ca. SW-948 | 2.9 | 0.0 |
| Melanoma* SK-MEL-5 MBL-5 | 0.0 | 0.0 | Colon ca. SW480 | 0.0 | 0.0 |
| Squamous cell carcinoma SCC-4 | 0.0 | 0.0 | Colon ca.* (SW480 met) SW620 | 3.6 | 0.0 |
| Testis-Pool | 0.0 | 0.0 | Colon ca. HT29 | 0.0 | 0.0 |
| Prostate ca* (bone met) PC-3 | 0.0 | 0.0 | Colon ca HCT-116 | 0.0 | 0.0 |
| Prostate Pool | 0.0 | 0.0 | Colon ca. CaCo-2 | 2.8 | 0.0 |
| Placenta | 2.3 | 0.0 | Colon cancer tissue | 0.0 | 0.0 |
| Uterus Pool. | 0.0 | 0.0 | Colon ca. SW1116 | 0.0 | 0.0 |
| Ovarian ca. OVCAR-3 | 0.0 | 0.0 | Colon ca. Colo-205 | 1.8 | 0.0 |
| Ovarian ca. SK-OV-3 | 0.0 | 0.0 | Colon ca. SW-48 | 0.0 | 0.0 |
| Ovarian ca. OVCAR-4 | 0.0 | 0.0 | Colon Pool | 0.0 | 0.0 |
| Ovarian ca. OVCAR-5 | 0.0 | 0.0 | Small Intestine Pool | 3.6 | 0.0 |
| Ovarian ca. IGROV-1 | 0.0 | 0.0 | Stomach Pool | 7.2 | 0.0 |
| Ovarian ca. OVCAR-8 | 0.0 | 0.0 | Bone Marrow Pool | 100.0 | 0.0 |
| Ovary | 0.0 | 0.0 | Fetal Heart | 5.7 | 0.0 |
| Breast ca. MCF-7 | 0.0 | 0.0 | Heart Pool | 0.0 | 0.0 |
| Breast ca. MDA-MB-231 | 2.5 | 0.0 | Lymph Node Pool | 0.0 | 0.0 |
| Breast ca. BT 549 | 0.0 | 0.0 | Fetal Skeletal Muscle | 21.8 | 0.0 |
| Breast ca. T47D | 0.0 | 0.0 | Skeletal Muscle Pool | 15.1 | 0.0 |
| Breast ca. MDA-N | 0.0 | 0.0 | Spleen Pool | 2.2 | 0.0 |
| Breast Pool | 0.0 | 0.0 | Thymus Pool | 0.0 | 0.0 |
| Trachea | 0.0 | 0.0 | CNS cancer (glio/astro) U87-MG | 0.0 | 0.0 |
| Lung | 0.0 | 0.0 | CNS cancer (glio/astro) U-118-MG | 3.0 | 0.0 |
| Fetal Lung | 0.0 | 0.0 | CNS cancer (neuro;met) SK-N-AS | 0.0 | 0.0 |
| Lung ca. NCI-N417 | 0:0 | 0.0 | CNS cancer (astro) SF-539 | 0.0 | 0.0 |
| Lung ca. LX-1 | 3.6 | 0.0 | CNS cancer (astro) SNB-75 cancer (astro) SNB-75 | 20.3 | 0.0 |
| Lung ca. NCI-H146 | 7.4 | 0.0 | CNS cancer (glio) SNB-19 | 39.5 | 0.0 |
| Lung ca. SHP-77 | 2.4 | 0.0 | CNS cancer (glio) SF-295 | 0.0 | 0.0 |
| Lung ca. A549 | 1.8 | 0.0 | Brain (Amygdala) Pool | 0.0 | 0.0 |
| Lung ca. NCI-H526 | 0.0 | 0.0 | Brain (cerebellum) | 0.0 | 0.0 |
| Lung ca. NCI-H23 | 0.0 | 0.0 | Brain (fetal) | 0.0 | 0.0 |
| Lung ca. NCI-H460 | 0.0 | 0.0 | Brain (Hippocampus) Pool | 3.2 | 0.0 |
| Lung ca. HOP-62 | 0.0 | 100.0 | Cerebral Cortex Pool | 0.0 | 0.0 |
| Lung ca. NCI-H522 | 0.0 | 0.0 | Brain (Substantia nigra) Pool | 0.0 | 0.0 |
| Liver | 0.0 | 0.0 | Brain (Thalamus) Pool | 2.3 | 0.0 |
| Fetal Liver | 2.2 | 0.0 | Brain (whole) | 12.9 | 0.0 |
| Liver ca. HepG2 | 0.0 | 0.0 | Spinal Cord Pool | 0.0 | 0.0 |
| Kidney Pool | 0.0 | 0.0 | Adrenal Gland | 0.0 | 0.0 |
| Fetal Kidney | 10.4 | 0.0 | Pituitary gland Pool | 0.0 | 0.0 |
| Renal ca. 786-0 | 0.0 | 0.0 | Salivary Gland | 7.2 | 0.0 |
| Renal ca. A498 | 0.0 | 0.0 | Thyroid (female) | 3.5 | 0.0 |
| Renal ca. ACHN | 0.0 | 0.0 | Pancreatic ca. CAPAN2 | 0.0 | 0.0 |
| Renal ca. UO-31 | 0.0 | 0.0 | Pancreas Pool | 0.0 | 0.0 |

**Table BJE. Panel 4.1D**

| **Tissue Name** | **Rel. Exp.(%) Ag4117, Run 172774997** | **Tissue Name** | **Rel. Exp.(%) Ag4117, Run 172774997** |
|---|---|---|---|
| Secondary Th1 act | 19.9 | HUVEC IL-1beta | 10.6 |
| Secondary Th2 act | 32.5 | HUVEC IFN gamma | 100.0 |
| Secondary Tr1 act | 31.0 | HUVEC TNF alpha+IFN gamma | 13.1 |
| Secondary Th1 rest | 17.7 | HUVEC TNF alpha+IL4 | 21.3 |
| Secondary Th2 rest | 22.4 | HUYEC IL-11 | 11.7 |
| Secondary Tr1 rest | 19.6 | Lung Microvascular EC none | 19.6 |
| Primary Th1 act | 26.6 | Lung Microvascular EC TNFalpha +IL-1beta | 35.6 |
| Primary Th2 act | 33.2 | Microvascular Dermal EC none | 19.6 |
| Primary Tr1 act | 27.4 | Microsvasular Dermal EC TNFalpha + IL-1beta | 32.8 |
| Primary Th1 rest | 23.5 | Bronchial epithelium TNFalpha + IL1beta | 22.5 |
| Primary Th2 rest | 22.5 | Small airway epitheliumnone | 23.0 |
| Primary Tr1 rest | 25.0 | Small airway epithelium TMFalpha + IL-1beta | 14.0 |
| CD45RA CD4 lymphocyte act | 34.2 | Coronery artery SMC rest | 21.6 |
| CD45RO CD4 lymphocyte act | 39.2 | Coronery artery SMC TNFalpha + IL-1beta | 18.6 |
| CD8 lymphocyte act | 43.2 | Astrocytes rest | 31.9 |
| Secondary CD8 lymphocyte rest | 27.0 | Astrocytes TNFalpha + IL-1beta | 27.4 |
| Secondary CD8 lymphocyte act | 27.5 | KU-812 (Basophil) rest | 30.6 |
| CD4 lymphocyte none | 22.1 | KU-812 (Basophil) PMA/ionomycin | 33.2 |
| 2ry Th1/Th2/Tr1_anti-CD95 CH 11 | 30.8 | CCD1106 (Keratinocytes) none | 13.8 |
| LAK cells rest | 38.7 | CCD1106 (Keratinocytes) TNFalpha + IL-1beta | 16.8 |
| LAK cells IL-2 | 33.4 | Liver cirrhosis | 22.4 |
| LAK cells IL-2+IL-12 | 25.7 | NCI-H292 none | 23.2 |
| LAK cells IL-2+IFN gamma | 20.4 | NCI-H292 IL-4 | 29.9 |
| LAK cells IL-2+IL-18 | 27.2 | NCI-H292 IL-9 | 14.9 |
| LAK cells PMA/ionomycin | 19.8 | NCI-H292 IL-13 | 33.0 |
| NK Cells IL-2 rest | 36.3 | NCI-H292 IFN gamma | 14.7 |
| Two Way MLR 3 day | 28.7 | HPAEC none | 20.6 |
| Two Way MLR 5 day | 16.8 | HPAEC TNF alpha + IL-1 beta | 24.0 |
| Two Way MLR 7 day | 9.1 | Lung fibroblast none | 24.0 |
| PBMC rest | 28.5 | Lung fibroblast TNF alpha + IL-1 beta | 26.8 |
| PBMC PWM | 22.4 | Lung fibroblast IL-4 | 20.0 |
| PBMC PHA-L | 17.2 | Lung fibroblast IL-9 | 22.4 |
| Ramos (B cell) none | 32.3 | Lung fibroblast IL-13 | 23.5 |
| Ramos (B cell) ionomycin | 22.2 | Lung fibroblast IFN gamma | 23.7 |
| B lymphocytes PWM | 25.2 | Dermal fibroblast CCD1070 rest | 26.8 |
| B lymphocytes CD40L and IL-4 | 23.5 | Dermal fibroblast CCD1070 TNF alpha | 21.9 |
| EOL-1 dbcAMP | 22.1 | Dermal fibroblast CCD1070 IL-1 beta | 36.1 |
| EOL-1 dbcAMP PMA/ionomycin | 27.7 | Dermal fibroblast IFN gamma | 39.5 |
| Dendritic cells none | 26.1 | Dermal fibroblast IL-4 | 23.2 |
| Dendritic cells LPS | 26.8 | Dermal Fibroblasts rest | 11.3 |
| Dendritic cells anti-CD40 | 30.8 | Neutrophils TNFa+LPS | 21.5 |
| Monocytesrest | 31.2 | Neutrophils rest | 39.0 |
| Monocytes LPS | 27.0 | Colon | 45.4 |
| Macrophages rest | 27.0 | Lung | 34.9 |
| Macrophages LPS | 21.5 | Thymus | 8.0 |
| HUVEC none | 25.2 | Kidney | 24.7 |
| HUVEC starved | 14.2 | | |

**CNS_neurodegeneration_v1.0 Summary:** Ag4117 This panel demonstrates the expression of the CG57823-01 gene at low levels in the brains of several individuals. However, no differential expression of this gene was detected between Alzheimer's diseased postmortem brains and those of non-demented controls in this experiment. The CG57823-01 gene encodes a protein with homology to acyltransferase. Ag3514 Expression of this gene is low/undetectable (CTs > 35) across all of the samples on this panel (data not shown).

**General_screening_panel_v1.4** Summary: Ag3514 In one experiment with this probe/primer set, expression of the CG57823-01 gene is limited to a bone marrow sample (CT = 33.9). Thus, expression of this gene may be used to distinguish bone marrow from the other samples on this panel. In the other experiment using this probe/primer set, expression of this gene is low/undetectable (CTs > 35) across all of the samples on this panel (data not shown).

**Panel 4.iD Summary:** Ag4117 Expression of the CG57823-01 gene is highest in IFN gamma treated human umbilical vein endothelial cells (CT = 33). This gene is expressed at low levels in a wide range of cell types of significance in the immune response in health and disease, including T-cells, B-cells, endothelial cells, macrophages, monocytes, dendritic cells, basophils, eosinophils and peripheral blood mononuclear cells, as well as epithelial and fibroblast cell types from lung and skin, and normal tissues represented by colon, lung, thymus and kidney. This ubiquitous pattern of expression suggests that this gene product may be involved in homeostatic processes for these and other cell types and tissues. Therefore, therapeutic modulation of the activity of this gene or its protein product may lead to the alteration of functions associated with these cell types and lead to improvement of the symptoms of patients suffering from autoimmune and inflammatory diseases such as asthma, allergies, inflammatory bowel disease, lupus erythematosus, psoriasis, rheumatoid arthritis, and osteoarthritis.

**Panel 4D Summary:** Ag3514 Expression of this gene is low/undetectable (CTs > 35) across all of the samples on this panel (data not shown).

### NOV68

Expression of NOV68/CG57801-01 was assessed using the primer-probe sets Ag3335 and Ag3336, described in Tables BKA and BKB. Results of the RTQ-PCR runs are shown in Tables BKC, BKD, BKE and BKF.

**Table BKA. Probe Name Ag3335**

| **Primers** | **Sequences** | **Length** | **Start Position** | **Seq id not** |
|---|---|---|---|---|
| Forward | 5'-ttgccatctattccgagtactg-3' | 22 | 477 | 458 |
| Probe | TET-5'-ccaacctcatgaagcagggcaagt-3'-TAMRA | 24 | 531 | 459 |
| Reverse | 5'-caggcttcaaagaaatgtctgt-3' | 22 | 555 | 460 |

**Table BKB. Probe Name Ag3336**

| **Primers** | **Sequences** | **Length** | **Start Position** | **Seq id not** |
|---|---|---|---|---|
| Forward | 5'-gtgcaagaggacaaggagatg-3' | 21 | 1181 | 461 |
| Probe | TET-5'-ttcagaaaaccagaagaaacttgcca-3'-TAMRA | 26 | 1213 | 462 |
| Reverse | 5'-cctgccttttgagcatttaac-3' | 21 | 1240 | 463 |

**Table BKC. CNS_neurodegeneration_v1.0**

| **Tissue Name** | **Rel.Exp.(%) Ag3335, Run 210138107** | **Rel. Exp.(%) Ag3335, Run 230512529** | **Rel. Exp.(%) Ag3336, Run 210138110** | **Tissue Name** | **Rel. Exp.(%) Ag3335, Run 210138107** | **Rel. Exp.(%) Ag3335, Run 230512529** | **Rel. Exp.(%) Ag3336, Run 210138110** |
|---|---|---|---|---|---|---|---|
| AD 1 Hippo | 26.1 | 22.8 | 28.1 | Control (Path) 3 Temporal Ctx | 5.3 | 4.3 | 6.1 |
| AD 2 Hippo | 29.9 | 31.9 | 29.5 | Control (Path) 4 Temporal Ctx | 18.3 | 15.8 | 17.3 |
| AD 3 Hippo | 13.7 | 11.8 | 11.7 | AD1 Occipital Ctx | 11.7 | 12.1 | 15.6 |
| AD 4 Hippo | 13.4 | 8.3 | 10.4 | AD 2 Occipital (Missing) | 0.0 | 0.0 | 0.0 |
| AD 5 Hippo | 39.8 | 39.0 | 30.8 | AD 3 Occipital Ctx | 11.6 | 8.5 | 7.9 |
| AD 6 Hippo | 100.0 | 99.3 | 100.0 | AD 4 Occipital Ctx | 25.5 | 15.1 | 12.1 |
| Control 2 Hippo | 40.3 | 27.4 | 44.8 | AD 5 Occipital Ctx | 25.5 | 26.8 | 27.4 |
| Control 4 Hippo | 26.1 | 25.0 | 27.7 | AD6 Occipital Ctx | 27.4 | 22.2 | 27.9 |
| Control (Path) 3 Hippo | 15.9 | 7.3 | 12.8 | Control 1 Occipital Ctx | 4.9 | 4.6 | 5.8 |
| AD 1 Temporal Ctx | 28.1 | 24.7 | 33.2 | Control 2 Occipital Ctx | 31.6 | 26.8 | 43.2 |
| AD 2 Temporal Ctx | 26.8 | 21.9 | 23.5 | Control 3 Occipital Ctx | 13.7 | 13.8 | 6.0 |
| AD3 Temporal Ctx | 6.2 | 8.3 | 6.7 | Control 4 Occipital Ctx | 11.7 | 11.7 | 11.6 |
| AD 4 Temporal Ctx | 27.5 | 22.4 | 21.9 | Control (Path) 1 Occipital Ctx | 50.7 | 48.0 | 48.0 |
| AD 5 Inf Temporal Ctx | 84.1 | 80.7 | 63.7 | Control (Path) 2 Occipital Ctx | 13.2 | 7.9 | 7.8 |
| AD 5 Sup Temporal Ctx | 52.9 | 58.6 | 52.9 | Control (Path) 3 Occipital Ctx | 4.0 | 4.2 | 3.9 |
| AD 6 Inf Temporal Ctx | 95.9 | 100.0 | 97.3 | Control (Path) 4 Occipital Ctx | 11.0 | 11.3 | 10.8 |
| AD 6 Sup Temporal Ctx | 67.4 | 67.4 | 54.3 | Control 1 Parietal Ctx | 11.8 | 7.3 | 9.9 |
| Control 1 Temporal Ctx | 8.4 | 6.8 | 8.0 | Control 2 Parietal Ctx | 48.6 | 37.6 | 49.7 |
| Control 2 Temporal Ctx | 25.2 | 31.0 | 25.0 | Control 3 Parietal Ctx | 22.7 | 12.8 | 13.6 |
| Control 3 Temporal Ctx | 15.9 | 16.3 | 13.5 | Control (Path) 1 Parietal Ctx | 27.0 | 24.7 | 28.1 |
| Control 3 Temporal Ctx | 12.1 | 8.1 | 9.4 | Control (Path) 2 Parietal Ctx | 19.8 | 16.7 | 16.3 |
| Control (Path) 1 Temporal Ctx | 26.2 | 27.5 | 30.6 | Control (Path) 3 Parietal Ctx | 4.0 | 3.8 | 0.4 |
| Control (path) 2 Temporal Temporal. Ctx | 19.5 | 20.6 | 13.3 | Control Control (Path) 4 Parietal Ctx | 27.4 | 23.7 | 25.2 |

**Table BKD. General_screening_panel_v1.4**

| **Tissue Name** | **Rel. Exp.(%) Ag3335, Run 213333209** | **Rel. Exp.(%) Ag3336, Run 215773745** | **Tissue Name** | **Rel. Exp.(%) Ag3335, Run 213333209** | **Rel. Exp.(%) Ag3336, Run 215773745** |
|---|---|---|---|---|---|
| Adipose | 4.0 | 4.6 | Renal ca. TK-10 | 12.2 | 12.2 |
| Melanoma* Hs688(A).T | 2.5 | 2.0 | Bladder | 24.0 | 19.8 |
| Melanoma* Hs688(B).T | 0.8 | 0.7 | Gastric ca. (liver met)NCI-N87 | 29.5 | 21.6 |
| Melanoma* M14 | 30.1 | 28.9 | Gastric ca KATO III | 52.5 | 41.2 |
| LOXIMVI Melanoma* | 2.4 | 2.4 | Colon ca. SW-948 | 4.3 | 6.2 |
| Melanoma. SK-MEL-5 | 7.5 | 12.0 | Colon ca. SW480 | 100.0 | 100.0 |
| Squamous cell carcinoma SCC-4 | 1.9 | 1.8 | Colon ca.* (SW480, met)SW620 | 49.3 | 57.4 |
| Testis Pool | 5.1 | 4.1 | Colon ca HT29 | 12.7 | 13.4 |
| Prostate ca.* (bone met) PC-3 | 9.6 | 7.3 | Colon ca. HCT- 116 | 14.2 | 16.7 |
| Prostate Pool | 11.4 | 9.3 | Colon ca. CaCo-2 | 13.2 | 9.9 |
| Placenta | 13.9 | 9.5 | Colon cancer tissue | 20.3 | 23.5 |
| Uterus Pool | 2.9 | 2.1 | Colon ca. SW1116 | 5.6 | 7.5 |
| Ovarian ca. OVCAR-3 | 6.4 | 4.9 | Colon ca. Colo-205 | 32.1 | 39.2 |
| Ovarian ca. SK-OV-3 | 12.5 | 12.5 | Colon ca. SW-48 | 10.9 | 10.6 |
| Ovarian ca. OVCAR-4 | 2.6 | 2.0 | Colon Pool | 6.9 | 5.2 |
| Ovarian ca. OVCAR-5 | 15.1 | 15.3 | Small Intestine Pool | 6.1 | 6.5 |
| Ovarian ca. IGROV-1 | 7.5 | 6.6 | Stomach Pool | 7.6 | 4.1 |
| Ovarian ca. OVCAR-8 | 2.5 | 2.6 | Bone Marrow Pool | 2.0 | 2.5 |
| Ovary | 9.5 | 8.4 | Fetal Heart | 3.3 | 1.7 |
| Breast ca. MCF-7 | 27.9 | 20.6 | Heart Pool | 3.0 | 4.8 |
| Breast ca MDA-MB-231 | 5.7 | 6.2 | Lymph Node Pool | 4.9 | 5.7 |
| Breast ca BT 549 | 3.8 | 3.5 | Fetal Skeletal Muscle | 0.8 | 0.7 |
| Breast ca.T47D | 23.2 | 32.1 | Skeletal Muscle Pool | 1.9 | 1.4 |
| Breast ca. MDA-N | 9.6 | 9.8. | Spleen Pool | 49.0 | 39.0 |
| Breast Pool | 6.9 | 5.6 | Thymus Pool | 9.6 | 0.0 |
| Trachea | 10.6 | 6.5 | CNS cancer (glio/astro) U87-MG | 2.1 : | 3.2 |
| Lung | 2.8 | 2.9 | CNS cancer (glio/astro) U-118-MG | 6.7 | 4.4 |
| Fetal Lung | 11.3 | 10.5 | CNS cancer (neuro;met) SK-N-AS | 0.6 | 0.2 |
| Lung ca. NCl-N417 | 2.6 | 2.9 | CNS cancer (astro) SF-539 | 1.4 | 1.5 |
| Lung ca. LX-1 | 79.0 | 92.0 | CNS cancer (astro) CNS cancer (astro) SNB-75 | 6.6 | 11.3 |
| Lung ca. NCI-H146 | 2.0 | 2.7 | CNS cancer (glio) SNB-19 | 5.8 | 7.3 |
| Lung ca. SHP-77 | 0.0 | 0.0 | CNS cancer (glio) SF-295 | 29.7 | 18.0 |
| Lung ca. A549 | 5.0 | 5.4 | Brain (Amygdala) Pool | 14.9 | 9.0 |
| Lung ca. NCI-H526 | 2.4 | 3.2 | Brain (cerebellum) | 7.7 | 6.9 |
| Lung ca. NCl-H23 | 2.3 | 2.4 | Brain (fetal) | 10.2 | 7.6 |
| Lung ca. NCI-H460 | 1.8 | 2.4 | Brain (Hippocampus) Pool | 13.6 | 9.5 |
| Lung ca. HOP-62 | 1.7 | 1.2 | Cerebral Cortex Pool | 12.6 | 7.6 |
| Lung ca. NCI-H522 | 1.1 | 0.8 | Brain (Substantia nigra) Pool | 14.9 | 9.5 |
| Liver | 1.6 | 1.0 | Brain (Thalamus) | 19.5 | 12.3 |
| Fetal Liver | 6.3 | 15.2 | Brain (whole) | 9.0 | 7.2 |
| Liver ca.HepG2 | 7.6 | 7.7 | Spinal Cord Pool | 21.0 | 16.2 |
| Kidney Pool | 10.6 | 8.5 | Adrenal Gland | 12.3 | 7.2 |
| Fetal Kidney | 11.0 | 10.6 | Pituitary gland Pool | 2.9 | 1.8 |
| Renal ca. 786-0 | 7.1 | 7.7 | Salivary Gland | 3.1 | 3.0 |
| Renal ca. A498 | 1.8 | 2.9 | Thyroid (female) | 5.0 | 4.9 |
| Renal ca. ACHN | 4.0 | 3.4 | Pancreatic ca. CAPAN2 | 14.0 | 12.6 |
| Renal ca. UO-31 | 1.9 | 2.0 | Pancreas Pool | 13.4 | 11.9 |

**Table BKE Panel 2.2**

| **Tissue Name** | **Rel. Exp.(%) Ag3335, Run 173762744** | **Tissue Name** | **Rel. Exp.(%) Ag3335, Run 173762744** |
|---|---|---|---|
| Normal Coton | 12.8 | KidneyMargin(OD04348) | 100.0 |
| Colon cancer (OD06064) | 12.2 | Kidney malignant cancer (OD06204B) | 5.7 |
| Colon Margin (OD06064) | 8.1 | Kidney normal adjacent tissue (OD06204E) | 9.7 |
| Colon cancer (OD06159) | 1.6 | Kidney Cancer (OD04450-O1) | 35.4 |
| Colon Margin (OD06159) | 10.6 | Kidney Margin (OD04450-03) | 25.5 |
| Colon cancer (OD06297-04) | 3.8 | Kidney Cancer 8120613 | 1.8 |
| Colon Margin (OD06297-05) | 11.4 | Kidney Margin 8120614 | 10.7 |
| CC Gr.2 ascend colon (ODO3921) | 8.0 | Kidney Cancer 9010320 | 1.8 |
| CC Margin (OD03921) | 3.1 | Kidney Margin 9010321 | 6.7 |
| Colan cancer metastasis (OD06104) | 6.6 | Kidney Cancer 8120607 | 6.1 |
| Lung Margin (OD06104) | 4.5 | Kidney Margin 8120608 | 3.5 |
| Colon mets to lung (OD04451-01) | 4.8 | Normal Uterus | 4.3 |
| Lung Margin (OD04451-02) | 21.3 | Uterine Cancer 064011 | 6.6 |
| Normal Prostate | 5.8 | Normal Thyroid | 1.7 |
| Prostate Cancer (OD04410) | 11.5 | Thyroid Cancer 064010 | 3.4 |
| Prostate Margin (OD04410) | 9.5 | Thyroid Cancer A302152 | 9.7 |
| Normal Ovary | 11.0 | Thyroid Margin A302153 | 6.6 |
| Ovarian cancer (OD06283-03) | 73 | Normal Breast | 15.2 |
| Ovarian Margin (OD06283-07) | 6.5 | Breast Cancer (OD04566) | 3.2 |
| Ovarian Cancer 064008 | 6.6 | Breast Cancer 1024 | 12.1 |
| Ovarian cancer (OD06145) | 10.3 | Breast Cancer (OD04590-01) | 5.8 |
| Ovarian Margin (OD06145) | 12.4 | Breast Cancer Mets (OD04590-03) | 9.6 |
| Ovarian cancer (OD06455-03) | 5.8 | Breast Cancer Metastasis (OD04655-05) | 22.8 |
| Ovarian Margin (OD06455-07) | 1.7 | Breast Cancer 064006 | 14.1 |
| Normal Lung | 13.1 | Breast Cancer 9100266 | 16.7 |
| Invasive poor diff. lung adeno (OD04945-01 | 6.7 | Breast Margin 9100265 | 7.6 |
| Lung Margin (OD04945-03) | 24.1 | Breast Cancer A209073 | 4.6 |
| Lung Malignant Cancer (OD03I26) (OD03126) | 12.8 | Breast Margin A2090734 | 15.8 |
| LungMargin (OD03126) | 5.4 | Breast cancer (OD06083) | 12.4 |
| Lung Cancer (OD05014A) | 11.6 | Breast cancer node metastasis (OD0683) | 12.8 |
| Lung Margin (OD05014B) | 16.4 | Normal Liver | 16.7 |
| Lung cancer (OD06081) | 2.0 | Liver Cancer 1026 | 2.0 |
| Lung Margin (OD06081) | 10.2 | Liver Cancer 1025 | 13.6 |
| Lung Cancer (OD04237-01) | 5.9 | Liver Cancer 6004-T | 11.8 |
| Lung Margin (OD04237-02) | 21.8 | Liver Tissue 6004-N | 6.7 |
| Ocular Melanoma Metastasis | 1.2 | Liver Cancer 6005-T | 9.7 |
| Ocular Melanoma Margin (Liver) | 7.4 | Liver Tissue 6005-N | 13.5 |
| Melanoma Metastasis | 16.8 | Liver Cancer 064003 | 16.6 |
| Melanoma Margin (Lung) | 24.7 | Normal Bladder 14.1 | |
| Normal Kidney | 9.3 | Bladder Cancer 1023 | 9.1 |
| Kidney Ca, Nuclear grade 2 (OD04338) | 45.1 | Bladder Cancer A302173 | 4.5 |
| Kidney Margin (OD04338) | 9.5 | Normal Stomach | 33.0 |
| Kidney Ca Nuclear grade 1/2 (OD04339) | 72.2 | Gastric Cancer 9060397 | 2.6 |
| Kidney Margin (OD04339) | 18.7 | Stomach Margin 9060396 | 11.3 |
| Kidney Ca, Clear cell type (OD04340) | 7.2 | Gastric Cancer 9060395 | 8.7 |
| Kidney Margin (OD04340) | 18.3 | Stomach Margin 9060394 | 11.4 |
| Kidney Ca, Nuclear grade 3 (OD04348) | 2.8 | Gastric Cancer 064005 | 18.3 |

**Table BKF. Panel 4D**

| **Tissue Name** | **Rel. Exp.(%) Ag3335, Run 165128064** | **Rel. Exp.(%) Ag3336, Run 165128065** | **Tissue Name** | **Rel. Exp.(%) Ag3335, Run 165128064** | **Rel.Exp.(%) Ag3336, Run 165128065** |
|---|---|---|---|---|---|
| Secondary Th1 act | 32.1 | 14.0 | HUVEC IL-1beta | 2.7 | 0.4 |
| Secondary Th2 act | 42.6 | 14.6 | HUVEC IFN gamma | 3.3 | 0.8 |
| Secondary Tr1 act | 43.5 | 11.6 | HUVEC TNF alpha + IFN gamma | 5.8 | 0.8 |
| Secondary Th1 rest | 9.0 | 3.1 | HUVEC TNF alpha + IL4 | 7.5 | 1.7 |
| Secondary Th2 rest | 15.5 | 4.0 | HUVEC IL-1 | 1.6 | 0.5 |
| Secondary Tr1 rest | 15.4 | 3.3 | Lung Microvascular EC none | 2.7 | 0.4 |
| Primary Th1 act | 38.2 | 9.3 | Lung Microvascular EC | 1.7 | 0.4 |
| Primary Th2 act | 34.4 | 8.1 | Microvascular Dermal EC none | 2.8 | 0.7 |
| Primary Tr1 act | 40.6 | 0.2 | Microsvasular Dermal EC TNFalpba + IL- 1beta | 2.5 | 0.6 |
| Primary Th1 rest | 62.4 | 15.5 | Bronchial epithelium TNFalpa + IL1beta | 0.5 | 0.2 |
| Primary Th2 rest | 41.8 | 100.0 | Small airway epithelium none | 0.8 | 0.2 |
| Primary Tr1 rest | 29.3 | 6.3 | Small airway epithelium TNFalpha + IL-1beta | 2.6 | 0.3 |
| CD45RA CD4 lymphocyte act | 20.4 | 5.3 | Coronery artery SMC rest | 5.4 | 1.2 |
| CD45RO CD4 lymphocyte act | 45.7 | 11.0 | Coronery artery SMC TNFaIpha+IL-lbeta | 3.4 | 0.8 |
| CD8 lymphocyte act | 41.8 | 7.7 | Astrocytes rest | 3.5 | 0.8 |
| Secondary CD8 lymphocyte rest | 42.3 | 6.4 | Astrocytes TNFalpha + IL-lbeta | 6.4 | 1.5 |
| Secondary CD8 lymphocyte act | 28.5 | 5.0 | KU-812 (Basophil) rest | 6.3 | 1.0 |
| CD4 lymphocyte none | 23.5 | 3.4 | KU-812 (Basophil) PMA/ionomycin | 19.9 | 4.5 |
| 2ry Th1/Th2/Tr1_anti-CD95 CH11 | 29.7 | 6.2 | CCD1106 (Keratinocytes) none | 1.2 | 0.2 |
| LAK cells rest | 39.0 | 6.9 | CCD1106 (Keratinocytes) TNFalpha+IL-1beta | 0.1 | 0.1 |
| LAK cells IL-2 | 58.2 | 12.8 | Liver cirrhosis | 5.7 | 1.2 |
| LAK cells IL-2+IL-12 | 47.0 | 7.2 | Lupus kidney | 3.0 | 0.9 |
| LAK cells IL-2+IFN gamma | 66.4 | 10.5 | NCI-H292 none | 3.8 | 0.8 |
| LAK cells IL-2+IL- | 49.7 | 7.8 | NCI-H292 IL-4 | 5.0 | 0.6 |
| PMA/ionomycin | 20.3 | 4.5 | NCI-H292IL-9 | 5.1 | 2.6 |
| NK Cells IL-2 rest | 47.3 | 9.7 | NCI-H292 IL-13 | 3.2 | 0.4 |
| Two Way MLR 3 day | 49.3 | 9.7 | NCI-H292 IFN gamma | 1.6 | 0.5 |
| Two Way MLR 5 day | 28.1 | 6.7 | HPAEC none | 1.7 | 0.2 |
| Two Way MLR 7 day | 26.6 | 5.8 | HPAEC TNF alpha+ IL-1 beta | 3.4 | 0.8 |
| PBMC rest | 22.1 | 3.3 | Lung fibroblast none | 6.7 | 2.1 |
| PBMC PWM | 100.0 | 25.0 | Lung fibroblast TNF alpha + IL-1 beta | 6.4 | 2.0 |
| PBMC PHA-L | 41.8 | 8.0 | Lung fibroblast IL-4 | 45.7 | 13.4 |
| Ramos (B cell) none | 4.3 | 0.7 | Lung fibroblast IL-9 | 17.2 | 2.1 |
| Ramos (B cell) ionomycin | 17.9 | 2.4 | Lung fibroblast IL-13 | 32.8 | 8.8 |
| B lymphocytes PWM | 79.6 | 11.0 | Lung fibroblast IFN gamma | 51.8 | 9.9 |
| B lymphocytes CD40L and IL-4 | 22.2 | 3.9 | Dermal fibroblast CCD1070 rest | 7.3 | 1.8 |
| EOL-1 dbcAMP | 1.8 | 0.4 | Dermal fibroblast CCD1070 TNF alpha | 42.9 | 13.4 |
| EOL-1 dbcAMP PMA/ionomycin | 13.0 | 2.4 | Dermal fibroblast CCD1070 IL-1 beta | 8.4 | 2.3 |
| Dendritic cells none | 13.2 | 1.8 | Dermal fibroblast IFN gamma | 9.0 | 2.8 |
| Dendritic cells LPS | 21.0 | 4.0 | Dermal fibroblast IL-4 | 12.4 | 3.5 |
| Dendritic cells anti-CD40 | 8.7 | 1.9 | IBD Colitis 2 | 1.7 7 | 0.4 |
| Monocytes rest | 16.2 | 2.4 | IBD Crohn's | 3.2 | 0.8 |
| Monocytes LPS | 12.8 | 3.8 | Colon | 17.7 | 4.5 |
| Macrophages rest | 37.6 | 7.2 | Lung | 10.6 | 2.8 |
| Macrophages LPS | 21.5 | 3.0 | Thymus | 35.1 | 13.7 |
| HUVEC none | 4.5 | 0.9 | Kidney | 27.2 | 8.6 |
| HUVEC starved | 12.0 | 1.6 | | | |

**CNS_neurodegeneration_v1.0 Summary:** Ag3335/Ag3336 Results from three experiments using two different probe/primer sets are in excellent agreement and when analyzed by ANCOVA p = 0.01 and p = 0.01. This panel confirms the expression of the CG57801-01 gene at moderate levels in the brains of an independent group of individuals. This gene is found to be upregulated in the temporal cortex of Alzheimer's disease patients. The CG57801-01 gene encodes a protein with homology to guanine nucleotide exchange factor. Inhibition of the CG57801-01 gene or its protein product may decrease neuronal death and be of use in the treatment of Alzheimer's disease.

**General_screening_panel_v1.4 Summary:** Ag3335/Ag3336 Results from two experiments using two different probe/primer sets are in excellent agreement. Expression of the CG57801-01 gene appears to be upregulated in a number of colon cancer cell lines, when compared to normal colon. Therefore, expression of this gene may be used to distinguish colon cancer cell lines from the other samples on this panel. Furthermore, therapeutic modulation of the activity of this gene or its protein product, using small molecule drugs, antibodies, and protein therapeutics, may be of use in the treatment of colon cancer. These results are consistent with what is observed in Panel 2.2. The CG57801-01 gene encodes a putative guanine nucleotide exchange factor (GEF) that is homologous to the ASEF protein. ASEF is known to stimulate cell flattening, membrane ruffling, and lamellipodia formation in the presence of APC (adenomatous polyposis coli protein), suggesting that the APC-Asef complex may regulate the actin cytoskeletal network, cell morphology and migration, and neuronal function (ref. 1). The adenomatous polyposis coli gene (APC) is mutated in familial adenomatous polyposis and in sporadic colorectal tumors.

In addition, this gene is expressed at high levels in all regions of the central nervous system examined, including amygdala, hippocampus, substantia nigra, thalamus, cerebellum, cerebral cortex, and spinal cord. Therefore, this gene may play a role in central nervous system disorders such as Alzheimer's disease, Parkinson's disease, epilepsy, multiple sclerosis, schizophrenia and depression. The CG57801-01 gene also displays significant homology to the collybistin gene. Collybistin is a newly identified brain-specific GEF that induces submembrane clustering of gephyrin and may be an important determinant of inhibitory postsynaptic membrane formation and plasticity (ref. 2).

Among tissues with metabolic or endocrine function, this gene is expressed at high to moderate levels in pancreas, adipose, adrenal gland, thyroid, pituitary gland, skeletal muscle, heart, liver and the gastrointestinal tract. Therefore, therapeutic modulation of the activity of this gene may prove useful in the treatment of endocrine/metabolically related diseases, such as obesity and diabetes. This gene is well-expressed in pancreas, which includes the insulin-secreting beta cells in the islets of Langerhans. Guanine nucleotide exchange factors are present in beta cells and are activated by cAMP. A rise in cAMP levels precedes glucose-induced insulin secretion (Ref. 3). Thus, the CG57801-01 gene maybe a target for therapeutic modulation of the beta cell secretory defect in Type 2 diabetes.

### References:

### 1. Kawasaki Y, Senda T, Ishidate T, Koyama R, Morishita T, Iwayama Y, Higuchi O, Akiyama T. (2000) Asef, a link between the tumor suppressor APC and G-protein signaling. Science 289(5482):1194-7

The adenomatous polyposis coli gene (APC) is mutated in familial adenomatous polyposis and in sporadic colorectal tumors. Here the APC gene product is shown to bind through its armadillo repeat domain to a Rac-specific guanine nucleotide exchange factor (GEF), termed Asef. Endogenous APC colocalized with Asef in mouse colon epithelial cells and neuronal cells. Furthermore, APC enhanced the GEF activity of Asef and stimulated Asef-mediated cell flattening, membrane ruffling, and lamellipodia formation in MDCK cells. These results suggest that the APC-Asef complex may regulate the actin cytoskeletal network, cell morphology and migration, and neuronal function.

### PMID: 10947987

### 2. Kins S, Betz H, Kirsch J (2000) Collybistin, a newly identified brain-specific GEF, induces submembrane clustering of gephyrin. Nat Neurosci 3(1):22-9

The formation of postsynaptic GABAA and glycine receptor clusters requires the receptor-associated peripheral membrane protein gephyrin. Here we describe two splice variants of a novel gephyrin-binding protein, termed collybistin I and II, which belong to the family of db1-like GDP/GTP exchange factors (GEFs). Co-expression of collybistin II with gephyrin induced the formation of submembrane gephyrin aggregates that accumulate heterooligomeric glycine receptors. Our data suggest that collybistin II regulates the membrane deposition of gephyrin by activating a GTPase of the Rho/Rac family. Therefore, this protein may be an important determinant of inhibitory postsynaptic membrane formation and plasticity.

### PMID: 10607391

### 3. Leech CA, Holz GG, Chepumy O, Habener JF. (2000) Expression of cAMP-regulated guanine nucleotide exchange factors in pancreatic beta-cells. Biochem Biophys Res Commun. 278(1):44-7.

The insulinotropic hormone glucagon-like peptide-1 (GLP-1) binds to a Gs-coupled receptor on pancreatic beta-cells and potentiates glucose-induced insulin secretion, insulin gene transcription, and beta-cell growth. These stimulatory effects have been attributed to the elevation of intracellular cAMP levels, though it is now apparent that some stimulatory effects of GLP-1 occur independently of the cAMP-mediated activation of protein kinase A (PKA). The nature of this alternative, PKA-independent signaling pathway remains unknown. Here we present evidence for the expression of type 1 and type 2 cAMP-regulated guanine nucleotide exchange factors (cAMP-GEFs) in beta-cells. GEFs are activated by their binding of cAMP. Because cAMP-GEFs activate Ras/MAPK proliferation signaling pathways, they may play an important role in PKA-independent, GLP-1-modiated, signaling pathways in the regulation of beta-cell growth and differentiation.

### PMID: 11071853

Panel 2.2 Summary: Ag3335 The CG57801-01 gene encodes a protein with homology to APC-stimulated guanine nucleotide exchange factor (ASEF). Its expression is low to moderate in all of the samples on this panel. Interestingly, expression of this gene is lower in some colon, lung and kidney cancer tissues compared to matched normal tissues. This observation suggests that expression of this gene can be used to distinguish these cancers from normal tissues. Also, therapeutic modulation of the activity of the protein encoded by this gene may be beneficial in the treatment of colon, kidney and lung cancer.

ASEF is known to mediate cell flattening, membrane ruffling, and lamellipodia formation which is stimulated by presence of APC (adenomatous polyposis coli gene) (Ref 1). The adenomatous polyposis coli gene (APC) is mutated in familial adenomatous polyposis and in sporadic colorectal tumors.

### References:

### 1. Kawasaki Y, Senda T, Ishidate T, Koyama R, Morishita T, Iwayama Y, Higuchi O, Akiyama T. (2000) Asef, a link between the tumor suppressor APC and G-protein signaling. Science 289(5482):1194-7

The adenomatous polyposis coli gene (APC) is mutated in familial adenomatous polyposis and in sporadic colorectal tumors. Here the APC gene product is shown to bind through its armadillo repeat domain to a Rac-specific guanine nucleotide exchange factor (GEF), termed Asef. Endogenous APC colocalized with Asef in mouse colon epithelial cells and neuronal cells. Furthermore, APC enhanced the GEF activity of Asef and stimulated Asef-mediated cell flattening, membrane ruffling, and lamellipodia formation in MDCK cells. These results suggest that the APC-Asef complex may regulate the actin cytoskeletal network, cell morphology and migration, and neuronal function.

### 10947987

**Panel 4D Summary:** Ag3336/Ag3335 The CG57801-01 transcript is expressed in several tissues. It is expressed in most lymphocytes, in both unstimulated and stimulated T cells. The expression of this gene is upregulate in B cells and PBMC treated with pokeweed mitogen. Myeloid cells also express the transcript. Most non-hematopoietic cell types do not consistently express this transcript with the exception of fibroblasts which upregulate the transcript in response to IL-4, IL-13, gamma interferon and tnfalpha. The expression profile of this gene suggests that it may be important in the normal function or activation of leukocytes and fibroblasts. Therefore, modulation of the gene product with a functional therapeutic may be useful for immunomodulation, or to treat diseases such as asthma, emphysema, psoriasis, and arthritis.

### NOV69

Expression of NOV69/CG57719-01 was assessed using the primer-probe sets Ag3314 and Ag4358, described in Tables BLA and BLB. Results of the RTQ-PCR runs are shown in Tables BLC, BLD and BLE.

**Table BLA. Probe Name Ag3314**

| **Primers** | **Sequences** | **Length** | **Start Position** | **SEQ ID NO** |
|---|---|---|---|---|
| Forward | 5'-ggaatacctggtcaggaagaag-3' | 22 | 1280 | 464 |
| Probe | TET-5'-cacatctatatccccaagaacggtca-3'-TAMRA | 26 | 1302 | 465 |
| Reverse | 5'-ttggcattgatacagctgaagt-3' | 22 | 1333 | 466 |

**Table BLB. Probe Name Ag4358**

| **Primers** | **Sequences** | **Length** | **Start Position** | **SEQ ID NO** |
|---|---|---|---|---|
| Forward | 5'-tgtctcaaggctttgagttctt-3' | 22 | 901 | 467 |
| Probe | TET-5'-ctgcagccagtctctgtccaaaa-3'-TAMRA | 23 | 923 | 468 |
| Reverse | 5'-tcccactccttcatcataaat-3' | 22 | 954 | 469 |

**Table BLC. CNS_neurodegeneration_v1.0**

| **Tissue Name** | **Rei. Exp.(%) Ag3314, Run 210138296** | **Rel. Exp.(%) Ag4358, Run 224372540** | **Tissue Name** | **Rel. Exp.(%) Ag3314, Run 210138296** | **Rel. Exp.(%) Ag4358,Run 224372540** |
|---|---|---|---|---|---|
| AD 1 Hippo | 4.6 | 25.2 | Control (Path) 3 Temporal Ctx | 0.0 | 9.7 |
| AD 2 Hippo | 18.6 | 52.5 | Control (Path) 4 Temporal Ctx | 74.2 | 39.0 |
| AD 3 Hippo | 4.1 | 8.5 | AD 1 Occipital Ctx | 16.4 | 14.6 |
| AD 4 Hippo | 6.4 | 11.8 | AD 2 Occipital Ctx (Missing) | 0.0 | 0.0 |
| AD 5 Hippo | 35.6 | 51.4 | AD3 Occipital | 7.4 | 8.2 |
| AD 6 Hippo | 61.6 | 95.9 | AD 4 Occipital | 11.7 | 29.7 |
| Control 2 Hippo | 5.8 | 29.3 | AD 5 Occipital Ctx | 10.7 | 46.3 |
| Control 4 Hippo | 5.7 | 36.1 | AD 6 Occipital Ctx | 5.4 | 37.4 |
| Control (path) 3 Hippo | 0.0 | 9.6 | Control 1 Occipital Ctx | 0.0 | 8.4 |
| AD 1 Temporal Ctx | 4.2 | 25.2 | Control 2 Occipital Ctx | 4.3 | 77.9 |
| AD 2 Temporal Ctx | 14.9 | 43.2 | Control 3 Occipital Ctx | 36.6 | 15.3 |
| AD 3 Temporal Ctx | 0.0 | 5.4 | Control 4 | 0.0 | 14.8 |
| AD 4 Temporal Ctx | 11.5 | 25.2 | Control (Path) 1 Occipital Ctx | 12.4 | 66.4 |
| AD 5 Inf Temporal Ctx | 13.9 | 87.7 | Control (Path) 2 Occipital Ctx | 19.3 | 12.8 |
| AD 5 Sup Temporal Ctx | 16.6 | 100.0 | Control (Path)3 Occipital Ctx | 0.0 | 9.5 |
| AD 6 Inf Temporal Ctx | 55.1 | 78.5 | Control (Path) 4 Occipital Ctx | 27.5 | 12.4 |
| AD 6 Sup Temporal Ctx | 100.0 | 80.1 | Control 1 Parietal Ctx | 21.5 | 18.9 |
| Control 1 Temporal Ctx | 15.6 | 13.1 | Control 2 Parietal Ctx | 20.0 | 53.6 |
| Control 2 Temporal Ctx | 24.3 | 42.0 | Control 3 Parietal Ctx | 0.0 | 17.0 |
| Control 3 Temporal Ctx | 19.1 | 12.7 | Control (Path) 1 Parietal Ctx | 20.6 | 49.0 |
| Control 3 Temporal Ctx | 0.0 | 14.7 | Control (path) 2 Parietal Ctx | 5.8 | 30.6 |
| Control (Path) 1 Temporat Ctx | 15.8 | 58.2 | Control (Path) 3 Parietal Ctx | 5.8 | 17.0 |
| Control (Path) 2 Temporal Ctx | 8.3 | 33.0 | Control (path) 4 Parietal Ctx | 37.6 | 22.4 |

**Table BLD. General_screening_panel_v1.4**

| **Tissue Name** | **Rel. Exp.(%) Ag3314, Run 215678552** | **Tissue Name** | **Rel. Exp.(%) Ag3314, Run 215678552** |
|---|---|---|---|
| Adipose | 1.0 | Renal ca. TK-10 | 0.0 |
| Melanoma* Hs688(A).T | 0.0 | Bladder | 0.0 |
| Melanoma* Hs688(B).T | 0.0 | Castric ca. (liver met.) NCI-87 | 0.0 |
| Melanoma* M14 | 0.0 | Gastric ca. KATO III | 0.0 |
| Melanoma* LOXIMVI | 0.0 | Colon ca. SW-948 | 0.8 |
| Melanoma* SK-MBL-5 | 0.0 | Colon ca. SW480 | 0.0 |
| Squamous cell carcinoma SCC-4 | 0.0 | Colon ca.*(SW480 met) SW620 | 0.8 |
| Testis Pool | **100.0** | Colon ca. HT29 | 0.0 |
| Prostate ca.*(bone met) PC-3 | 0.0 | Colon ca. HCT-116 | 0.9 |
| Prostate Pool | 1.0 | Colon ca. CaCo-2 | 0.0 |
| Placenta | 3.5 | Colon cancer tissue | 0.0 |
| Uterus Pool | 0.7 | Colon ca. SW1116 | 0.0 |
| Ovarian ca. OVCAR-3 | 0.0 | Colon ca. Colo-205 | 0.0 |
| Ovarian ca. SK-OV-3 | 0.0 | Colon ca. SW-48 | 0.0 |
| Ovarian ca. OVCAR-4 | 0.0 | Colon Pool | 0.0 |
| Ovarian ca. OVCAR-5 | 38.2 | Small Intestine Pool | 9.9 |
| Ovarian ca. IGROV-1 | 0.0 | Stomach Pool | 2.3 |
| Ovarian ca. OVCAR-8 | 0.0 | Bone Marrow Pool | 0.8 |
| Ovary | 5.7 | Fetal Heart | 0.9 |
| Breast ca. MCF-7 | 0.0 | Heart Pool | 1.7 |
| Breast ca. MDA-MB-231 | 0.0 | Lymph Node Pool | 0.9 |
| Breast ca. BT 549 | 0.0 | Fetal Skeletal Muscle | 0.0 |
| Breast ca. T47D | 0.0 | Skeletal Muscle Pool | 0.0 |
| Breast ca. MDA-N | 0.0 | Spleen Pool | 0.0 |
| Breast Pool | 1.2 | Thymus Pool | 2.5 |
| Trachea | 1.2 | CNS cancer (glio/astro) U87-MG | 0.0 |
| Lung ung | 73.7 | CNS cancer (glio/astro) U- 118 MG 118-MG | 1.4 |
| Fetal Lung | 0.0 | CNS cancer (neuro;met) SK-N-AS | 0.0 |
| Lungca.NCI-N4t7 | 0.0 | CNS cancer (astro) SF-539 | 0.0 |
| Lung ca.LX-1 | 1.2 | CNS cancer (astro) SNB-75 | 0.0 |
| Lung ca. NCI-H 146 | 0.0 | CNS cancer (glio) SNB-19 | 1.7 |
| Lung ca. SHP-77 | 0.0 | CNS cancer (glio) SF-295 | 0.0 |
| Lung ca. A549 | 0.7 | Bram (Amygdala) Pool | 1.7 |
| Lung ca. NCI-H526 | 0.0 | Brain (cerebellum) | 0.0 |
| Lung ca. NCI-H23 | 0.0 | Brain (fetal) | 0.0 |
| Lung ca. NCI-H460 | 0.0 | Brain (Hippocampus) Pool | 5.2 |
| Lung ca. HOP-62 | 0.0 | Cerebral Cortex Pool | 2.1 |
| Lung ca. NCI-H522 | 2.0 | Brain (Substantia nigra) Pool | 0.0 |
| Liver | 0.0 | Brain (Thalamus) Pool | 0.0 |
| Fetal Liver | 0.0 | Brain (whole) | 4.5 |
| Liver ca. HepG2 | 0.0 | Spinal Cord Pool | 0.0 |
| Kidney Pool | 25.0 | Adrenal Gland | 0.0 |
| Fetal Kidney | 9.6 | Pituitary gland Pool | 0.0 |
| Renal ca. 786-0 | 0.0 | Salivary Gland | 0.0 |
| Renal ca. A498 | 0.0 | Thyroid (female) | 0.0 |
| Renal ca. ACHN | 0.0 | Pancreatic ca. CAPAN2 | 0.0 |
| Renal ca. UO-31 | 0.0 | Pancreas Pool | 0.0 |

**Table BLE. General_screening_panel_v1.5**

| **Tissue Name** | **Rel.Exp.(%) Ag4358, Run 244373100** | **Tissue Name** | **Rel.Exp.(%) Ag4358, Run 244373100** |
|---|---|---|---|
| adipose | 0.0 | Renal ca. TK-10 | 0.0 |
| Melanoma* Hs688(A).T | 0.0 | Bladder | 0.0 |
| Melanoma* Hs688(B).T | 0.0 | Gastric ca. (liver met.) NCI- | 0.0 |
| Melanoma* M14 | 0.0 | Gastric ca. KATO III | 0.0 |
| Melanoma* LOXIMVI | 0.0 | Colon ca. SW-948 | 2.9 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| Testis Pool | 100.0 | Colon ca. HT29 | 0.0 |
| Prostate ca.* (bone met) PC-3 | 0.0 | Colon ca.HCT-116 | 0.0 |
| Prostate Pool | 0.0 | Colon ca. CaCo-2 | 0.0 |
| Placenta | 0.0 | Colon cancer tissue | 0.0 |
| Uterus Pool | 0.0 | Colon ca. SW1116 | 0.0 |
| Ovarian ca. OVCAR-3 | 0.0 | Colon ca. Colo-205 | 0.0 |
| Ovarian ca. SK-OV-3 | 0.0 | Colon ca. SW-48 | 0.0 |
| Ovarian ca. OVCAR-4 | 2.1 | Colon Pool | 0.0 |
| Ovarian ca. OVCAR-5 | 0.0 | Small Intestine Pool | 0.0 |
| Ovarian ca. IGROV-1 | 0.0 | Stomach Pool | 0.0 |
| Ovarian ca. OVCAR-8 | 0.0 | Bone Marrow Pool | 0.0 |
| Ovary | 1.2 | Fetal Heart | 0.0 |
| Breast ca. MCF-7 | 0.0 | Heart Pool | 0.0 |
| Breast ca. MDA MB 231 | 0.0 | Lymph Node Pool | 0.0 |
| Breast ca. BT 549 | 0.0 | Fetal Skeletal Muscle | 0.0 |
| Breast ca. T47D | 0.0 | Skeletal Muscle Pool | 0.0 |
| Breast ca. MDA-N | 0.0 | Spleen Pool | 0.0 |
| Breast Pool | 0.0 | Thymus Pool | 0.0 |
| Trachea | 0.0 | CNS cancer (glio/astro) U87-MG | 0.0 |
| Lung | 0.0 | CNS cancer (glio/astro) U-118-MG | 0.0 |
| Fetal Lung | 0.0 | CNS cancer (neuro;met) SK-N-AS | 0.0 |
| Lung. ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 0.0 | CNS cancer (glio) SNB-19 | 0.0 |
| Lung ca. SHP-77 | 0.0 | CNS cancer (glio) SF-295 | 0.0 |
| Lung ca. A549 | 0.0 | Brain (Amygdala) Pool | 0.0 |
| Lung ca. NCI-H526 | 0.0 | Brain (cerebellum) | 0.0 |
| Lung ca. NCI-H23 | 0.0 | Brain (fetal) | 0.0 |
| Lung ca. NCI-H460 | 0.0 | Brain (Hippocampus) Pool | 0.0 |
| Lung ca. HOP-62 | 0.0 | Cerebral Cortex Pool | 0.0 |
| Lung ca. NCI-H522 | 0.0 | Brain (Substantia nigra) Pool | 0.0 |
| Liver | 0.0 | Brain (Thalamus) Pool | 0.0 |
| Fetal Liver | 0.0 | Brain (whole) | 0.0 |
| Liver ca. HepG2 | 0.0 | Spinal Cord Pool | 0.0 |
| Kidney Pool | 0.0 | Adrenal Gland | 0.0 |
| Fetal Kidney | 0.0 | Pituitary gland Pool | 0.0 |
| Renal ca. 786-0 | 0.0 | Salivary Gland | 0.0 |
| Renal ca. A498 | 0.0 | Thyroid (female) | 0.0 |
| Renal ca. ACHN | 0.0 | Pancreatic ca. CAPAN2 | 0.0 |
| Renal ca. UO-31 | 0.0 | Pancreas Pool | 1.5 |

**CNS_neurodegeneration_v1.0 Summary:** Ag3314/Ag4358 Results from two experiments using different probe/primer sets show moderate agreement. This panel demonstrates the expression of the CG57719-01 gene at low levels in the brains of an independent group of individuals. However, no differential expression of this gene was detected between Alzheimer's diseased postmortem brains and those of non-demented controls in this experiment. The CG57719-01 gene encodes a protein with homology to aspartate aminotransferase.

**General_screening_panel_v1.4 Summary:** Ag3314 Expression of the CG57719-01 gene is highest in a sample derived from testis (CT = 32.2) with low but significant expression also detected in lung and kidney. Thus, the expression of this gene could be used to distinguish these samples from the other samples in the panel. Furthermore, therapeutic modulation of this gene product may be useful for the diagnosis and/or treatment of fertility and hypogonadism.

**General_screening_panel_v1.5 Summary:** Ag4358 Expression of the CG57719-01 gene is restricted to a sample derived from testis (CT = 32.67). This observation is consistent with the results obtained using Ag3314 on Panel 1.4. Thus, the expression of this gene could be used to distinguish testis from the other samples in the panel. Furthermore, therapeutic modulation of this gene product may be useful for the diagnosis and/or treatment of fertility and hypogonadism.

**Panel 4.1D Summary:** Ag4358 Expression of this gene is low/undetectable (CTs > 35) across all of the samples on this panel (data not shown).

**Panel 4D** Summary: Ag3314 Expression of this gene is low/undetectable (CTs > 35) across all of the samples on this panel (data not shown).

### NOV70

Expression of NOV70/CG57462-01 was assessed using the primer-probe set Ag3247, described in Table BMA. Results of the RTQ-PCR runs are shown in Tables BMB, BMC, BMD and BME.

**Table BMA. Probe Name Ag3247**

| **Primers** | **Sequences** | **Length** | **Start Position** | **SEQ ID NO** |
|---|---|---|---|---|
| Forward | 5'-gacgttcaacaatgacatgct-3' | 21 | 1940 | 470 |
| Probe | TET-5'-cttcatcagcagcagctgcattgct-3'-TAMRA | 25 | 1967 | 471 |
| Reverse | 5'-agcaggaggtgaggatgtagec-3' | 22 | 1998 | 472 |

**Table BMB. CNS_neurodegeneration_v1.0**

| **Tissue Name** | **Rel. Exp.(%) Ag3247, Run 210037961** | **Tissue Name** | **Rel. Exp.(%) Ag3247, Run 210037961** |
|---|---|---|---|
| AD 1 Hippo | 18.8 | Control (Path) 3 Temporal Ctx | 3.7 |
| AD 2 Hippo | 28.1 | Control (Path) 4 Temporal Ctx | 32.3 |
| AD 3 Hippo | 3.2 | AD 1 Occipital Ctx | 14.7 |
| AD 4 Hippo | 7.9 | AD 2 Occipital Ctx (Missing) | 0.0 |
| AD 5 Hippo | 97.9 | AD 3 Occipital Ctx | 3.2 |
| AD 6 Hippo | 37.6 | AD 4 Occipital Ctx | 11.5 |
| Control 2 Hippo | 30.1 | AD 5 Occipital Ctx | 15.2 |
| Control 4 Hippo | 8.7 | AD 6 Occipital Ctx | 25.0 |
| Control (Path) 3 Hippo | 3.4 | Control 1 Occipital Ctx | 1.8 |
| AD 1 Temporal Ctx | 17.7 | Control 2 Occipital Ctx | 59.9 |
| AD 2 Temporal Ctx | 41.8 | Control 3 Occipital Ctx | 17.3 |
| AD 3 Temporal Ctx | 6.7 | Control 4 Occipital Ctx | 6.7 |
| AD 4 Temporal Ctx | 27.2 | Control(Path) 1 Occipital Ctx | 81.2 |
| AD 5 Inf Temporal Ctx | 100.0 | Control (Path) 2 Occipital Ctx | 10.8 |
| AD Sup Temporal Ctx | 57.4 | Control (Path) 3 Occipital Ctx | 0.6 |
| AD 6 Inf Temporal Ctx | 23.2 | Control (Path) 4 Occipital Ctx | 18.7 |
| AD 6 SupTemporal Ctx | 43.5 | Control 1 Parietal Ctx | 7.5 |
| Control 1 Temporal Ctx | 6.0 | Control 2 Parietal Ctx | 57.8 |
| Control 2 Temporal Ctx | 16.3 | Control 3 Parietal Ctx | 14.4 |
| Control 3 Temporal Ctx | 11.7 | Control (Path) 1 Parietal Ctx | 37.9 |
| Control 3 Temporal Ctx | 12.4 | Control (Path) 2 Parietal | 26.1 |
| Control (Path) 1 Temporal Ctx | 61.6 | Control (Path) 3 Parietal Ctx | 2.2 |
| Control (Path) 2 Temporal Ctx | 32.1 | Control (Path) 4 Parietal Ctx | 29.7 |

**Table BMC. Generat_screening_panel_v1.4**

| **Tissue Name** | **Rel.Exp.(%) Ag3247, Run 214693633** | **Tissue Name** | **Rel. Exp(%) Ag3247, Run 214693633** |
|---|---|---|---|
| Adipose | 0.1 | Renal ca. TK-10 | 1.9 |
| Melanoma* Hs688(A).T | 0.1 | Bladder | 0.5 |
| Melanoma* Hs688(B).T | 0.0 | Gastric ca. (liver met.) NCI-N87 | 0.0 |
| Melanoma* M14 | 0.1 | Gastric ca. KATO III | 0.0 |
| Melanoma* LOXIMVI | 0.1 | Colon ca. SW-948 | 0.0 |
| Melanoma* SK-MEL-5 | 0.0 | Colon ca. SW480 | 1.2 |
| Squamous cell carcinoma SCC-4 | 0.5 | Colon ca.* (SW480 met) SW620 | 1.1 |
| Testis Pool | 2.8 | Colon ca. HT29 | 0.3 |
| Prostate ca* (bone met) PC-3 | 0.7 | Colon ca.HCT.116 | 1.6 |
| Prostate Pool | 0.3 | Colon ca. CaCo-2 | 0.9 |
| Placenta | 0.0 | Colon cancer tissue | 0.0 |
| Uterus Pool | 0.0 | Colon ca. SW1116 | 1.2 |
| Ovarian ca. OVCAR-3 | 0.3 | Colon ca. Colo-205 | 0.0 |
| Ovarian ca. SK-OV-3 | 0.1 | Colon ca. SW-48 | 0.1 |
| Ovarian ca.OVCAR-4 | 0.0 | Colon Pool | 0.2 |
| Ovarian ca. OVCAR-5 | 0.4 | Small Intestine Pool | 0.1 |
| Ovarian ca. IGROV-1 | 0.1 | Stomach Pool | 0.4 |
| Ovarian ca. OVCAR-8 | 0.1 | Bone Marrow Pool | 0.0 |
| Ovary | 0.1 | Fetal Heart | 0.0 |
| Breast ca. MCF-7 | 4.2 | Heart Pool | 0.0 |
| Breast ca. MDA-MB-231 | 0.2 | Lymph Node Pool | 0.1 |
| Breast ca. BT 549 | 0.4 | Fetal Skeletal Muscle | 0.1 |
| Breast ca. T47D | 0.9 | Skeletal Muscle Pool | 0.0 |
| Breast ca. MDA-N | 0.4 | Spleen Pool | 0.1 |
| Breast Pool | 0.4 | Thymus Pool | 0.6 |
| Trachea | 0.0 | CNS cancer (glio/astro) U87- , MG | 0.0 |
| Lung | 0.1 | CNS cancer (glio/astro) U-118-MG | 0.0 |
| Fetal Lung | 0.5 | CNS cancer (neuro;met) SK-N-AS | 0.2 |
| Lung ca. NCI-N417 | 8.7 | CNS cancer (astro) SF-539 | 0.1 |
| Lung ca. LX-1 | 0.2 | CNS cancer (astro) SNB-75 | 0.2 |
| Lung ca.NCI.H146 | 1.6 | CNS cancer(glio)SNB-19 | 0.0 |
| Lung ca. SHP-77 | 6.3 | CNS cancer (glio)SF-295 | 0.4 |
| Lung ca. A549 | 0.0 | Brain (Amygdala) Poll | 3.6 |
| Lung ca. NCI-H526 | 1.2 | Brain (cerebellum) | 16.7 |
| Lungca.NCI.H23 | 4.5 | Brain (fetal) | 100.0 |
| Lung ca.NCI-H460 | 0.4 | Bram(Hippocampus)Pool | 6.0 |
| Lung ca. HOP-62 | 0.0 | Cerebral Cortex Pool | 6.0 |
| Lung ca. NCI-H522 | 2.2 | Brain (Substantia nigra) Pool | 5.6 |
| Liver | 0.0 | Brain(Thalamus) Pool | 6.8 |
| Fetal Liver | 0.1 | Brain (whole) | 12.8 |
| Liver ca. HepG2, | 0.4 | Spinal Cord Pool | 3.8 |
| Kidney Pool | 0.0 | Adrenal Gland | 0.4 |
| Fetal Kidney | 1.6 | Pituitary gland Pool | 0.7 |
| Renal ca. 786-0 | 0.7 | Salivary Gland | 0.0 |
| Renal ca. A498 | 0.2 | Thyroid (female) | 0.0 |
| Renal ca. ACHN | 0.1 | Pancreatic ca. CAPAN2 | 1.2 |
| Renal ca. UO-31 | 0.5 | Pancreas Pool | 0.5 |

**Table BMD. Panel 2.2**

| **Tissue Name** | **Rel.Exp.(%) Ag3247, Run 174441297** | **Tissue Name** | **Rel.Exp.(%) Ag3247, Run 174441297** |
|---|---|---|---|
| Normal Colon | 0.7 | Kidney Margin (OD04348) | 2.4 |
| Colon cancer (OD06064) | 0.0 | Kidney malignant cancer (OD06204B) | 0.0 |
| Colon Margin (OD06064) | 0.0 | Kidney normal adjacent tissue (OD06204B) | 0.0 |
| Colon cancer (OD06159) | 0.0 | Kidney Cancer (OD04450-01) | 3.4 |
| Colon Margin (OD061S9) | 0.0 | Kidney Margin (OD04450-03) | 2.2 |
| Colon cancer (OD06297-04) | 0.0 | Kidney Cancer 8120613 | 0.0 |
| Colon Margin (OD06297-05) | 0.0 | Kidney Margin 8120614 | 0.0 |
| CC Gr.2 ascend colon (ODO3921) | 1.4 | Kidney Cancer 9010320 | 0.0 |
| CC Margin (ODO3921) | 0.0 | Kidney Margin 9010321 | 0.0 |
| Colon cancer metastasis (OD06104) | 0.0 | Kidney Cancer 8120607 | 2.5 |
| Lung Margin (OD06104) | 0.0 | Kidney Margin 8120608 | 1.2 |
| Colon mets to lung (OD04451- | 4.1 | Normal Uterus | 1.2 |
| Lung Margin (OD04451-02) | 0.0 | Uterine Cancer 064011 | 2.7 |
| Normal Prostate | 3.3 | Normal Thyroid | 0.0 |
| Prostate Cancer (OD04410) | 0.0 | Thyroid Cancer 064010 | 0.0 |
| Prostate Margin (OD04410) | 0.0 | Thyroid Cancer A302152 | 0.0 |
| Normal Ovary | 8.0 | Thyroid Margin A302153 | 0.0 |
| Ovarian cancer (OD06283-03) | 0.0 | Normal Breast | 2.4 |
| Ovarian Margin (OD06283-07) | 0.0 | Breast Cancer (OD04566) | 0.0 |
| Ovarian Cancer 064008 | 4.2 | Breast Cancer 1024 | 1.3 |
| Ovarian cancer (OD06145) | 0.0 | Breast Cancer (OD04590-01) | 7.1 |
| Ovarian Margin (OD06145) | 0.0 | Breast Cancer Mets (OD04590-03) | 4.4 |
| Ovarian cancer (OD06455-03) | 0.0 | Breast Cancer Metastasis (OD04655-05) | 100.0 |
| Ovarian Margin (OD06455-07) | 0.0 | Breast Cancer 064006 | 1.2 |
| Normal Lung | 1.6 | Breast Cancer 9100266 | 2.5 |
| Invasive poor diff. lung adeno (ODO4945-01 | 0.0 | Breast Margin 9100265 | 0.0 |
| Lung Margin (ODO4945-03) | 0.0 | Breast Cancer A209073 | 3.4 |
| Lung Malignant Cancer (OD03126) | 0.0 | Breast Margin A2090734 | 0.0 |
| Lung Margin (OD03126) | 0.0 | Breast cancer (OD06083) | 3.2 |
| Lung Cancer (OD05014A) | 0.0 | Breast cancer node metastasis (OD06083) | 2.5 |
| Lung Margin (OD05014B) | 0.6 | Normal Liver | 0.0 |
| Lung cancer (OD06081) | 0.0 | Liver Cancer 1026 | 0.0 |
| Lung Margin (OD06081) | 1.4 | Liver Cancer 1025 | 2.1 |
| Lung Cancer (OD04237-01) | 0.0 | Liver Cancer 6004-T | 0.0 |
| Lung Margin (OD04237-02) | 0.4 | Liver Tissue 6004-N | 0.0 |
| Ocular Melanoma Metastasis | 0.0 | Liver Cancer 6005-T | 2.4 |
| Ocular Melanoma Margin (Liver) | 0.0 | Liver Tissue 6005-N | 0.0 |
| Melanoma Metastasis | 0.0 | Liver Cancer 064003 | 0.0 |
| Melanoma Margin (Lung) | 0.0 | Normal Bladder | 0.0 |
| Normal Kidney | 0.0 | Bladder Cancer 1023 | 0.0 |
| Kidney Ca, Nuclear grade 2 (OD04338) | 0.0 | Bladder Cancer A302173 | 0.0 |
| Kidney Margin (OD04338) | 0.0 | Normal Stomach | 0.0 |
| Kidney Ca Nuclear grade 1/2 (OD04339) | 1.2 | Gastric Cancer 9060397 | 0.8 |
| Kidney Margin (OD04339) | 0.0 | Stomach Margin 9060396 | 0.9 |
| Kidney Ca, Clear cell type (OD04340) | 0.0 | Gastric Cancer 9060395 | 0.0 |
| Kidney Margin (OD04340) | 0.0 | Stomach Margin 9060394 | 1.4 |
| Kidney Ca, Nuclear grade 3 (OD04348) | 0.0 | Gastric Cancer 064005 | 0.0 |

**Table BME. Panel 4D**

| **Tissue Name** | **Rel. Exp.(%)A g3247, Run 164390951** | **Tissue Name** | **Rel. Exp.(%) Ag3247, Run 164390951** |
|---|---|---|---|
| Secondary Th1 act | 1.6 | HUVEC IL-1beta | 0.6 |
| Secondary 7h2 act | 0.6 | HUVEC IFN gamma | 4.1 |
| Secondary Tr1 act | 0.8 | HUVEC TNF alpha + IFN gamma | 0.0 |
| Secondary Th 1 rest | 0.0 | HUVEC TNF alpha + IL4 | 1.2 |
| Secondary Th2 rest | 0.8 | HUVEC IL-11 | 3.1 |
| Secondary Tr1 rest | 0.0 | Lung Microvascular EC none | 17.1 |
| Primary Th1 act | 7.0 | Lung Microvascular EC TNFalpha +IL-1beta | 11.0 |
| Primary Th2 act | 3.5 | Microvascular Dermal EC none | 0.6 |
| Primary Tr1 act | 4.9 | Microsvasular Dermal EC TNFalpha+IL-1beta | 0.7 |
| PrimaryTh1 rest | 0.6 | Bronchial epithelium TNFalpha + EL1beta | 100.0 |
| Primary Th2 rest | 1.2 | Small airway epithelium none | 1.8 |
| Primary Tr1 rest | 4.7 | Small airway epithelium TNFalpha +IL-1beta | 28.3 |
| CD45RA CD4 lymphocyte act | 0.7 | Coronery artery SMC rest | 0.0 |
| CD45RO CD4 lymphocyte act | 2.7 | Coronery artery SMC TNFalpha + IL-1beta | 0.0 |
| CD8 lymphocyte act | 3.0 | Astrocytes rest | 1.2 |
| Secondary CD8 lymphocyte rest | 4.2 | Astrocytes TNFalpha + IL-1beta | 0.6 |
| Secondary CD8 lymphocyte act | 0.7 | KU-812 (Basophil) rest | 0.0 |
| CD4 lymphocyte none | 1.3 | KU-812 (Basophil) PMA/ionomycin | 0.7 |
| 2ry Th1/Th2/Tr1_anti-CD95 CH11 | 0.7 | CCD1106(Keratinocytes)none | 37.1 |
| LAK cells rest | 0.0 | CCD1106 (Keratinocytes) TNF atpha+IL-1beta | 28.9 |
| LAK cells IL-2 | 4.0 | Liver cirrhosis | 6.2 |
| LAK cells IL-2+IL-12 | 2.1 | Lupus kidney | 1.1 |
| LAK cells IL-2+IFN gamma | 6.5 | NCI-H292 none | 2.5 |
| LAK cells IL-2+ IL-18 | 4.7 | NCI-H292 IL-4 | 1.3 |
| LAK cells PMA/ionomycin | 0.5 | NCI-H292 IL-9 | 4.6 |
| NK Cells IL-2 rest | 1.3 | NCI-H292 IL-13 | 1.1 |
| Two Way MLR 3 day | 1.8 | NCI-H292 IFN gamma | 0.0 |
| Two Way MLR 5 day | 0.5 | HPAEC none | 0.0 |
| Two Way MLR 7 day | 1.7 | HPAEC TNF alpha+IL-1 beta | 0.0 |
| PBMC rest | 0.0 | Lung fibroblast none | 0.0 |
| PBMCPWM | 6.0 | Lung fibroblast TNF alpha+IL-1 beta | 0.0 |
| PBMC PHA-L | 0.6 | Lung fibroblast IL-4 | 0.0 |
| Ramos (B cell) none | 1.9 | Lung fibroblast IL-9 | 0.0 |
| Ramos (B cell) ionomycin | 1.9 | Lung fibroblast IL-13 | 0.0 |
| B lymphocytes PWM | 1.3 | Lung fibroblast IFN gamma | 0.0 |
| B lymphocytes CD40L and IL-4 | 5.0 | Dermal fibroblast CCD1070 rest | 0.7 |
| EOL-1 dbcAMP | 3.0 | Dermal fibroblast CCD1070 TNF alpha | 0.7 |
| EOL-1 dbcAMP PMA/ionomycin | 0.6 | Dermal fibroblast CCD1070 IL-beta | 0.0 |
| Dendritic cells none | 0.0 | Dermal fibroblast IFN gamma | 0.0 |
| Dendritic cells LPS | 0.0 | Dermal fibroblast IL-4 | 0.7 |
| Dendritic cells anti-CD40 | 0.0 | IBD Colitis 2 | 0.0 |
| Monocytes rest | 0.6 | IBD Crohn's | 0.0 |
| Monocytes LPS | 0.0 | Colon | 2.7 |
| Macrophages rest | 0.0 | Lung | 0.6 |
| Macrophages LPS | 0.0 | Thymus | 1.0 |
| HUVEC none | 0.0 | Kidney | 7.0 |
| HUVEC starved | 5.0 | | |

**CNS_neurodegeneration_v1.0 Summary:** Ag3247 This panel confirms the expression of this gene at low levels in the brains of an independent group of individuals. However, no differential expression of this gene was detected between Alzheimer's diseased postmortem brains and those of non-demented controls in this experiment. Please see Panel 1.4 for a discussion of the potential utility of this gene in treatment of central nervous system disorders.

General_screening_panel_v1.4 Summary: Ag3247 Highest expression of CG57462-01 is seen in samples derived from fetal brain (CT=26.5). In addition moderate expression of this gene is also observed in samples derived from whole brain, thalamus, Substantia nigra, cerebral cortex and hippocampus. Thus, expression of this gene could be used to differentiate between these samples and other samples on this panel. Also, this gene may play a role in central nervous system disorders such as Alzheimer's disease, Parkinson's disease, epilepsy, multiple sclerosis, schizophrenia and depression.

The CG57462-01 gene encodes for a protein containing a patched domain (IPR003392). Patched, is a receptor for the morphogene sonic hedgehog. In Drosophila melanogaster, this protein associates with the smoothened protein to transduce hedgehog signals, leading to the activation of wingless, decapentaplegic and patched itself. It participates in cell interactions that establish pattern within the segment and imaginal disks during development. In mammals, the Hedgehog (HH) signaling pathway is involved in patterning and development of a variety of organ systems, including the nervous system, the skeletal system, the craniofacial structures, and the gastrointestinal tract. Abnormalities in this signaling cascade have been found in various developmental pathologies and neoplasms such as basal cell carcinoma, Niemann-Pick type II disease, holoprosencephaly. The abnormalities are associated with congenital or sporadic genetic alteration affecting function of different components of the HH signaling pathway, including SHH, PTCH, SMOH and GLI proteins (Ref. 2, 3). Thus, therapeutic modulation of the activity of the protein encoded by the CG57463-01 gene may be useful in the treatment of basal cell carcinoma, Niemann-Pick type II disease, and holoprosencephaly

### References:

### 1. IPR003392: Patched family

The transmembrane protein, patched, is a receptor for the morphogene Sonic Hedgehog. In Drosophila melanogaster, this protein associates with the smoothened protein to transduce hedgehog signals, leading to the activation of wingless, decapentaplegic and patched itself. It participates in cell interactions that establish pattern within the segment and imaginal disks during development. The mouse homolog may play a role in epidermal development. The human Niemann-Pick C1 protein, defects in which cause Niemann-Pick type II disease, is also a member of this family. This protein is involved in the intracellular trafficking of cholesterol, and may play a role in vesicular trafficking in glia, a process that may be crucial for maintaining the structural functional integrity of nerve terminals.

### 2. Incardona JP, Roelink H. (2000) The role of cholesterol in Shh signaling and teratogen-induced holoprosencephaly. Cell Mol Life Sci 57(12):1709-19.

Holoprosencephaly, or an undivided forebrain, is a complex brain malformation associated with Sonic hedgehog (Shh) mutations. Other causes of holoprosencephaly have converged upon the Shh signaling pathway: genetic and pharmacologic impairment of cholesterol synthesis, and the action of the steroidal alkaloid cyclopamine. This review focuses on recent studies aimed at determining how Shh signaling is affected by these causes of holoprosencephaly, whether they involve a common mechanism and the role played by cholesterol. Cholesterol is potentially important for both biogenesis of Shh and in signal transduction in Shh-responsive cells. Teratogens that induce holoprosencephaly appear to affect Shh signal transduction rather than Shh biogenesis. Analysis of these agents and other compounds that affect various aspects of cellular cholesterol distribution indicates that the role of cholesterol in Shh signal transduction is novel and complicated. The similarity of the Shh receptor, Patched (Ptc), to the Niemann-Pick C1 protein, which is involved in the vesicular trafficking of cholesterol, provides insight into the role of cholesterol and the action of compounds like cyclopamine.

### PMID: 11130177

### 3. Oldak M, Grzela T, Lazarczyk M, Malejczyk J, Skopinski P. (2001) Clinical aspects of disrupted Hedgehog signaling (Review). Int J Mol Med 8(4):445-52.

The Hedgehog (HH) signaling pathway is involved in patterning and development of a variety of organ systems, including the nervous system, the skeletal system, the craniofacial structures, and the gastrointestinal tract. Recent evidence also implicates this signaling pathway in the postembryonic regulation of stem-cell number in epithelia and blood. The family of HH proteins consists of at least three different members, i.e., sonic HH (SHH), Indian HH (IHH), and desert HH (DHH). SHH is the most broadly expressed member of this family and is probably responsible for the major effects of this signaling pathway. The HH signal is received and transduced via a specific receptor complex composed of patched (PTCH) and smoothened (SMOH) transmembrane proteins. Abnormalities in this signaling cascade have been found in various developmental pathologies and neoplasms such as basal cell carcinoma. The abnormalities are associated with congenital or sporadic genetic alteration affecting function of different components of the HH signaling pathway, including SHH, PTCH, SMOH and GLI proteins.

### PMID: 11562786

**Panel 2.2 Summary:** Ag3247 Expression of the CG57462-01 gene is highest in a metastatic breast cancer sample (CT = 29.8). Strikingly, this gene is expressed at higher levels in breast tumors when compared to their matched normal breast controls. Thus, expression of this gene may be used for the diagnosis of breast cancer. Furthermore, therapeutic modulation of the activity of this gene or its protein product, using small molecule drugs, antibodies or protein therapeutics, may be of benefit in the treatment of breast cancer.

**Panel 4D Summary:** Ag3247 Expression of this gene is highest in bronchial epithelium treated with TNF alpha and IL-1 beta (CT = 30). In addition, expression of this gene is upregulated in activated small airway epithelium. Thus, therapeutic modulation of the activity of this gene may be of benefit in the treatment of asthma and emphysema.

### NOV71

Expression of NOV71/CG57584-01 was assessed using the primer-probe set Ag3290, described in Table BNA. Results of the RTQ-PCR runs are shown in Tables BNB, and BNC.

**Table BNA. Probe Name Ag3290**

| **Primers** | **Sequences** | **Length** | **Start Position** | **SEQ ID NO** |
|---|---|---|---|---|
| Forward | 5'-ccctcagaggactggtttactt-3' | 22 | 1598 | 473 |
| Probe | TET-5'-cacctctgcctgccacacctcag-3'-TAMRA | 23 | 1629 | 474 |
| Reverse | 5'-ctacatgcagtggagcaagtct-3' | 22 | 1658 | 475 |

**Table BNB. CNS_neurodegeneration_v1.0**

| Tissue Name | Rel. Exp.(%) Ag3290, Run 210062016 | Tissue Name | Rel. Exp.(%) Ag3290, Run 210062016 |
|---|---|---|---|
| AD 1 Hippo | 24.8 | Control (Path) 3 Temporal Ctx | 11.5 |
| AD 2 Hippo | 15.1 | Control (Path) 4 Temporal Ctx | 94.0 |
| AD 3 Hippo | 0.0 | AD 1 Occipital Ctx | 12.6 |
| AD 4 Hippo | 19.1 | AD 2 Occipital Ctx (Missing) | 0.0 |
| AD 5 hippo | 51.8 | AD 3 Occipital Ctx | 33.9 |
| AD 6 Hippo | 28.7 | AD 4 Occipital Ctx | 0.0 |
| Control 2 Hippo | 26.8 | AD 5 Occipital Ctx | 0.0 |
| Control 4 Hippo | 23.8 | AD 6 Occipital Ctx | 55.1 |
| Control (Path) 3 Hippo | 6.3 | Control 1 Occipital Ctx | 17.4 |
| AD1 Temporat Ctx | 12.2 | Control 2 Occipital Ctx | 100.0 |
| AD 2 Temporal Ctx | 42.0 | Control 3 Occipital Ctx | 29.3 |
| AD 3 Temporal Ctx | 9.8 | Control 4 Occipital Ctx | 21.0 |
| AD 4 Temporal Ctx | 62.0 | Control (Path) 1 Occipital Ctx | 52.9 |
| AD 5 Inf Temporal Ctx | 14.0 | Control (Path) 2 Occipital Ctx | 15.4 |
| AD 5 Sup Temporal Ctx | 26.6 | Control (Path) 3 Occipital Ctx | 9.8 |
| AD 6 Inf Temporal Ctx | 12.5 | Control (Path) 4 Occipital Ctx | 13.0 |
| AD 6 Sup Temporal Ctx | 2.3 | Control 1 Parietal Ctx | 13.8 |
| Control 1 Temporal Ctx | 39.0 | Control 2 Parietal Ctx | 76.3 |
| Control 2 Temporal Ctx | 39.8 | Control 3 Parietal Ctx | 21.3 |
| Control 3 Temporal Ctx | 0.0 | Control (Path) 1 Parietal | 50.3 |
| Control 4 Temporal Ctx | 12.4 | Control (Path) 2 Parietal Ctx | 16.0 |
| Control (Path)1 Temporal Ctx | 48.3 | Control (Path) 3 Parietal Ctx | 19.8 |
| Control (Path) 2 Temporal Ctx | 51.1 | Control (Path) 4 Parietal Ctx | 84.7 |

**Table BNC. Panel 4D**

| **Tissue Name** | **Rel. Exp.(%) Ag3290, Run 164331693** | **Tissue Name** | **Rel. Exp.(%) Ag3290, Run 164331693** |
|---|---|---|---|
| Secondary Th1 act | 0.0 | HUVEC IL-1beta | 0.0 |
| Secondary Th2 act | 0.0 | HUVEC IFN gamma | 0.0 |
| SecondaryTr1 act | 0.0 | HUVEC TNFalpha+IFN gamma | |
| Secondary Th1 rest | 0.0 | HUVEC TNF alpha +IL4 | 0.0 |
| Secondary Th2 rest | 14.9 | HUVEC IL-11, | 0.0 |
| Secondary Trl rest | 3.6 | Lung Microvascular EC none | 0.0 |
| Primary Th1 act Primary Th1 act | 0.0 | Lung Microvascular EC TNFalpha +IL-1beta | 0.0 |
| Primary Th2 act | 0.0 | Microvascular Dermal EC none | 0.0 |
| Primary Tr1 act | 4.0 | Microsvasular Dermal EC TNFalpha+ IL-1beta | 0.0 |
| Primary Th1 rest | 9.4 | Bronchial epithelium TNFalpha + IL1beta | 0.0 |
| Primary Th2 rest | 36.6 | Small airway epithelium none | 9.1 |
| Primary Tr1 rest | 16.2 | Small airway epithelium TNFalpha + IL-1beta | 0.0 |
| CD45RA CD4 lymphocyte act | 0.0 | Coronery artery SMC rest | 100.0 |
| CD45RO CD4 lymphocyte act | 0.0 | Coronery artery SMC TNFalpha + IL-1beta | 38.2 |
| CD8 lymphocyte act | 0.0 | Astrocytes rest | 4.0 |
| Secondary CDS lymphocyte rest | 0.0 | Astrocytes TNFalpha+ IL-1beta | 3.7 |
| Secondary CD8 lymphocyte act | 0.0 | KU-812 (Basophil) rest | 0.0 |
| CD4 lymphocyte none | 0.0 | KU-812 (Basophil) pMA/ionomycin | 0.5 |
| 2ry Th1/Th2/Tr1_anti-CD95 CH11 | 6.8 | CCD1106 (Keratinocytes) none | 0.0 |
| LAK cells rest | 0.0 | CCD1106 (Keratinocytes) TNFalpha+IL-1 beta | 0.0 |
| LAK cells IL-2 | 2.9 | Liver cirrhosis | 0.0 |
| LAK cells IL-2+IL-12 | 0.0 | Lupus kidney | 0.0 |
| LAK cells IL-2+IFN gamma | 4.6 | NCI-H292 none | 0.0 |
| LAK cells IL-2+IL-18 | 0.0 | NCI-H292 IL-4 | 0.0 |
| LAK cells PMA/ionomycin | 0.0 | NCI-H292 IL-9 | 0.5 |
| NK Cells IL-2 rest | 0.0 | NCI-H292 IL-13, | 4.0 |
| Two Way MLR 3 day | 0.0 | NCI-H292 IFN gamma | 0.0 |
| Two Way MLR 5 day | 0.0 | HPAEC none | 0.0 |
| Two Way MLR 7 day | 0.0 | HPAEC TNFalpha+IL-1 beta | 0.0 |
| PBMC rest | 12.8 | Lung fibroblast none | 0.0 |
| PBMC PWM | 0.0 | Lung fibroblast TNF alpha + IL-1 beta | 0.0 |
| PBMCPHA-L | 4.4 | Lung fibroblast IL-4 | 0.0 |
| Ramos (B cell) none | 0.0 | Lung fibroblast IL-9 | 0.0 |
| Ramoi (B cell) ionomycin | 0.0 | Lung fibroblast IL-13 | 0.0 |
| B lymphocytes PWM | 0.0 | Lung fibroblast IFN gamma | 0.0 |
| B lymphocytes CD40L and IL-4 | 0.0 | Dermal fibroblast CCD1070 rest | 17.6 |
| EOL-1 dbcAMP alpha | 0.0 | Dermal fibroblast CCD1070 TNF | 13.9 |
| EOL-1 dbcAMP PMA/ionomycin | 0.0 | Dermal fibroblast CCD1070 IL-1 beta | 8.9 |
| Dendritic cells none | 0.0 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 0.0 |
| Monocytes rest | 0.0 | IBD Crohn's | 0.0 |
| Monocytes LPS | 4.2 | Colon | 3.6 |
| Macrophagesrest | 3.7 | Lung | 16.2 |
| Macrophages LPS | 0.0 | Thymus | 0.0 |
| HUVEC none | 0.0 | Kidney | 26.1 |
| HUVEC starved | 0.0 | | |

**CNS_neurodegeneration_v1.0 Summary:** Ag3290 This panel demonstrates the expression of this gene at low levels in the brains of several individuals. However, no differential expression of this gene was detected between Alzheimer's diseased postmortem brains and those of non-demented controls in this experiment. The CG57584-01 gene encodes a protein with homology to zona pellucida sperm-binding protein 1 precursor.

**General_screeninuanel- vl.4 Summary:** Ag3290 Results from one experiment with the CG57584-01 gene are not included. The amp plot indicates that there were experimental difficulties with this run.

**Panel 4D Summary:** Ag3290 Expression of the CG57584-01 gene is highest in resting coronary artery smooth muscle cells (CT = 31.6). In addition, low but significant expression of this gene is seen in normal lung and kidney samples as well as in dermal fibroblasts. Thus, expression of this gene may be used to distinguish these samples from the other samples on this panel. Furthermore, therapeutic modulation of the activity of this gene may be useful for the treatment of asthma and restenosis.

### NOV72

Expression of NOV72/CG56761-01 was assessed using the primer-probe sets Ag3011, Ag1810 and Ag1811, described in Tables BOA, BOB and BOC. Results of the RTQ-PCR runs are shown in Tables BOD, BOE, BOF, BOG, BOH and BOI.

**Table BOA. Probe Name Ag3011**

| **Primers** | **Sequences** | **Length** | **Start Position** | **Seq id no:** |
|---|---|---|---|---|
| Forward | 5'-gaactgttccttgtggtttcc-3' | 21 | 4438 | 476 |
| Probe | TET-5'-accacaggcatcagcagtcccatt-3'-TAMRA. | 24 | 4470 | 477 |
| Reverse | 5'-acactttgcctcaggtgactt-3' | 21 | 4495 | 478 |

**Table BOB. Probe Name Ag1810**

| **Primers** | **Sequences** | **Length** | **Start Position** | **Seq id no:** |
|---|---|---|---|---|
| Forward | 5'-gtcaggagaactgttccttgtg-3 | 22 | 4431 | 479 |
| Probe | TET-5'-accacaggcatcagcagtcccat-3'-TAMRA | 23 | 4470 | 480 |
| Reverse | 5'-ctttgcctcaggtgacttga-3' | 20 | 4493 | 481 |

**Table BOC. Probe Name Ag1811**

| **Primers** | **Sequences** | **Length** | **Start Position** | **Seq id no:** |
|---|---|---|---|---|
| Forward | 5'-ttggagcctttgctcttatgt-3' | 21 | 5503 | 482 |
| Probe | TET-5'-aggggagaccatccccaagttacaaa-3'-TAMRA | 26 | 5467 | 483 |
| Reverse | 5'-aagatgcccagaaaagctgta-3' | 21 | 5432 | 484 |

**Table BOD. AI_comprehensive panel_v1.0**

| **Tissue Name** | **Rel. Exp.(%) Ag3011, Run 225567597** | **Tissue Name** | **Rel. Exp.(%) Ag3011, Run 225567597** |
|---|---|---|---|
| 110967 COPD-F | 6.1 | 112427 Match Control Psoriasis-F | 35.6 |
| 110980 COPD-F | 11.8 | 112418 Psoriasis-M | 10.7 |
| 110968 COPD-M | 7.4 | 112723 Match Control Psoriasis-M | 0.1 |
| 110977 COPD-M | 0.0 | 112419Psoriasis-M | 11.4 |
| 110989 Emphysema-F | 8.0 | 112424 Match Control Psonasis-M | 3.6 |
| 110992 Emphysema-F | 3.8 | 112420 Psoriasis-M | 12.2 |
| 110993 Emphysema-F | 6.3 | 112425 Match Control Psoriasis-M | 25.5 |
| 110994 Emphysema-F | 2.4 | 104689 (MF) OA Bone-Backus | 24.5 |
| 110995 Emphysema-F | 8.5 | 104690 (MF) Adj "Normal" Bone-Backus | 24.0 |
| 110996 Emphysema-F | 1.0 | 104691 (MF) OA Synovium-Backus | 10.6 |
| 110997 Asthma-M | 2.3 | 104692 (BA) OA Cartilage-Backus | 0.0 |
| 111001 Asthma-F | 6.7 | 104694 (BA)OA Bone-Backus | 8.5 |
| 111002 Asthma-F | 5.5 | 104695 (BA) Adj "Normal" Bone-Backus | 27.2 |
| 111003 Atopic Asduna-F | 5.0 | 104696 (BA) OA Synovium-Backus (BA) | 6.4 |
| 111004 Atopic Asthma-F | 2.5 | 104700 (SS) OA Bone-Backus | 14.6 |
| 111005 Atopic Asthma-F | 1.8 | 104701 (SS) Adj "Normal" Bone-Backus | 22.8 |
| 111006 Atopic Asthma-F | 0.4 | 104702 (SS) OA Synovium-Backus | 23.8 |
| 111417 Allergy-M | 2.4 | 117093 OA Cartilage Rep7 | 5.8 |
| 112347 Allergy-M | 0.2 | 112672 OA Bone5 | 10.9 |
| 112349 Normal Lung-F | 0.7 | 112673 OA Synovium5 | 4.6 |
| 112357 Normal Lung-F | 4.6 | 112674 OA Synovial Fluid cells5 | 5.6 |
| 112354 Normal Lung-M | 5.8 | 117100 OA Cartilage Rep14 | 5.3 |
| 112374 Crohns-F | 1.3 | 112756 OA Bone9 | 20.3 |
| 112389 Match Control Crohns-F | 4.2 | 112757 OA Synovium9 | 2.8 |
| 112375 Crohns-F | 1.0 | 112758 OA Synovial Fluid Cells9 | 4.1 |
| 112732 Match Control Crohns-F | 56.6 | 117125 RA Cartilage Rep2 | 12.4 |
| 112725 Crohns-M | 0.5 | 113492 Bone2 RA | 21.2 |
| 112387 Match Control | 4.9 | 113493 Synovium2 RA | 9.4 |
| 112378 Crohns-M | 1.0 | 113494 Syn Fluid Cells RA | 12.8 |
| 112390 Match Control Crohns-M | 7.9 | 113499 Cartilage4 RA | 12.8 |
| 112726 Crohns-M | 5.0 | 113500 Bone4 RA | 19.5 |
| 112731 Match Control Crohns-M | 5.9 | 113501 Synovium4 RA | 14.0 |
| 112380 Ulcer Col-F | 5.2 | 113502 Syn Fluid Cells4 RA | 6.4 |
| 112734 Match Control Ulcer Col-F | 100.0 | 113495 Cartilage3 RA | 11.5 |
| 112384 Ulcer Col-F | 11.0 | 113496 Bone3 RA | 13.8 |
| 112737 Match Control Ulcer | 1.7 | 113497 Synovium3 RA | 5.6 |
| 112386 Ulcer Col-F | 4.5 | 113498 Syn Fluid Cells3 RA | 15.3 |
| 112738 Match Control Ulcer Col-F | 5.3 | 117106 Normal Cartilage Rep20 | 5.9 |
| 112381 Ulcer Col-M | 3.1 | 113663 Bone3 Normal | 0.4 |
| 112735 Match Control Ulcer Col-M | 2.3 | 113664Synovium3 Normal | 0.0 |
| 112382 Ulcer Col-M | 6.7 | 113665 Syn Fluid Cells3 Normal | 0.0 |
| 112394 Match Control Ulcer Col-M | 2.4 | 117107 Normal Cartilage Rep22 | 3.0 |
| 112383 Ulcer Col-M | 6.6 | 113667 Bone4 Normal | 2.8 |
| 112736 Match Control Ulcer Col-M | 3.0 | 113668 Synovium4 Normal | 3.4 |
| 112423 Psoriasis-F | 4.2 | 113669 Syn Fluid Cells4 Normal | 4.3 |

**Table BOE. CNS_neurodegeneration_v1.0**

| **Tissue Name** | **Rel. Exp.(%) Ag1810, Run 207742479** | **Rel. Exp.(%) Ag1811, Run 207776005** | **Rel. Exp.(%) Ag3011, Run 211010257** | **Tissue Name** | **Rel. Exp.(%) Ag1810, Run 207742479** | **Rel. Exp.(%) Agl8ll,Run 207776005** | **Rel. Exp.(%) Ag3011, Run 211010257** |
|---|---|---|---|---|---|---|---|
| AD 1 Hippo | 7.8 | 11.3 | 9.1 | Control (Path) 3 Temporal Ctx | 4.3 | 6.9 | 4.0 |
| AD 2 Hippo | 13.1 | 21.9 | 15.2 | Control (Path) 4 Temporal Ctx | 20.7 | 26.6 | 23.5 |
| AD 3 Hippo | 4.6 | 6.7 | 5.3 | AD 1 Occipital Ctx | 12.7 | 21.3 | 13.3 |
| AD 4 Hippo | 4.6 | 6.2 | 3.7 | AD 2 Occipital Ctx (Missing) | 0.2 | 0.0 | 0.0 |
| AD 5 Hippo | 100.0 | 100.0 | 100.0 | AD 3 Occipital Ctx | 6.1 | 10.9 | 6.9 |
| AD 6 Hippo | 19.9 | 19.9 | 18.8 | AD 4 Occipital Ctx | 12.1 | 16.7 | 13.4 |
| Control 2 Hippo | 11.9 | 19.1 | 12.3 | AD 5 Occipital Ctx | 29.1 | 41.2 | 21.5 |
| Control 4 Hippo | 3.9 | 7.0 | 3.4 | AD6 Occipital Ctx | 17.3 | 17.4 | 31.6 |
| Control (Path) 3 Hippo | 3.7 | 5.0 | 3.6 | Control 1 Occipital Ctx | 3.7 | 5.7 | 3.4 |
| AD 1 Temporal Ctx | 11.7 | 15.0 | 9.5 | Control 2 Occipital Ctx | 59.9 | 81.2 | 62.0 |
| AD 2 Temporal Ctx | 20.9 | 22.7 | 17.7 | Control 3 Occipital Ctx | 13.9 | 21.0 | 13.5 |
| AD3 Temporal Ctx | 5.8 | 9.0 | 5.4 | Control 4 Occipital Ctx | 3.7 | 8.8 | 3.8 |
| AD 4 Temporal Ctx | 15.2 | 17.8 | 14.1 | Control (Path) 1 Occipital Ctx | 51.4 | 58.6 | 54.7 |
| AD 5 Inf Temporal Ctx | 91.4 | 95.3 | 82.4 | Control (Path) 2 Occipital Ctx | 8.9 | 13.9 | 11.3 |
| AD 5 Sup Temporal Ctx Ctx | 25.3 | 35.4 | 33.0 | Control (path) 3 Occipital Ctx | 2.8 | 4.5 | 3.3 |
| AD 6 Inf Temporal Ctx | 27.4 | 27.7 | 30.4 | Control (Path) 4 Occipital ctx | 17.2 | 15.9 | 13.9 |
| AD 6 Sup Temporal Ctx | 28.7 | 26.8 | 32.1 | Control 1 Parietal Ctx | 5.5 | 7.4 | 4.9 |
| Control 1 Temporal Ctx | 3.4 | 5.9 | 2.8 | Control 2 Parietal Ctx | 31.9 | 38.7 | 35.6 |
| Control 2 Temporal Ctx | 34.4 | 42.9 | 28.9 | Control 3 Parietal Ctx | 14.9 | 20.9 | 15.1 |
| Control 3 Temporal Ctx | 6.4 | 12.8 | 7.7 | Control (Path) 1 Parietal Ctx | 47.3 | 53.2 | 51.8 |
| Control 3 Temporal Ox | 6.0 | 11.3 | 8.2 | Control (Path) 2 Parietal Ctx | 14.5 | 15.9 | 16.2 |
| Control (Path) 1 Temporal Ctx | 37.6 | 37.9 | 39.8 | Control (Path) 3 Parietal Ctx | 3.6 | 5.6 | 4.3 |
| Control (path) 2 Temporal Ctx | 24.1 | 30.1 | 26.8 | Control (Path) 4 Parietal Ctx | 25.2 | 35.4 | 31.6 |

**Table BOF. Panel 1.3D**

| **Tissue Name** | **Rel. Exp.(%) Ag1810, Run 146338117** | **Rel. Exp.(%) Ag1811, Run 147104851** | **Rel. Exp.(%) Ag1811, Run 165544641** | **Rel. Exp.(%) Ag3011, Run 167927169** |
|---|---|---|---|---|
| Liver adenocarcinoma | 3.8 | 3.3 | 2.3 | 7.0 |
| Pancreas | 0.3 | 0.0 | 0.6 | 0.4 |
| Pancreatic ca. CAPAN 2 | 0.0 | 0.0 | 0.2 | 0.1 |
| Adrenal gland | 1.1 | 2.2 | 1.7 | 1.2 |
| Thyroid | 0.6 | 1.8 | 1.1 | 0.4 |
| Salivary gland | 0.9 | 1.7 | 0.8 | 1.4 |
| Pituitary gland | 1.2 | 1.5 | 0.6 | 0.7 |
| Brain (fetal) | 2.8 | 4.1 | 8.8 | 15.1 |
| Brain (whole) | 48.0 | 49.0 | 100.0 | 93.3 |
| Brain (amygdala) | 32.1 | 47.6 | 84.1 | 48.0 |
| Brain (cerebellum) | 9.9 | 13.9 | 44.4 | 60.7 |
| Brain (hippocampus) | 75.8 | 86.5 | 88.3 | 31.6 |
| Brain (substantia nigra) | 10.8 | 23.0 | 49.7 | 39.8 |
| Brain (thalamus) | 18.0 | 29.9 | 71.7 | 28.5 |
| Cerebral Cortex | 100.0 | 100.0 | 77.4 | 100.0 |
| Spinal cord | 11.3 | 17.8 | 28.5 | 17.8 |
| glio/astro U87-MG | 0.0 | 0.0 | 0.0 | 0.0 |
| glio/astro U-118-MG | 0.0 | 0.0 | 0.0 | 0.0 |
| astrocytoma SW1783 | 0.0 | 0.0 | 0.0 | 0.1 |
| neuro*; met SK-N-AS | 23.7 | 30.4 | 15.4 | 13.3 |
| astrocytoma SF-539 | 0.0 | 0.0 | 0.2 | 0.0 |
| astrocytoma SNB-75 | 0.0 | 0.1 | 0.1 | 0.2 |
| glioma SNB-19 | 0.0 | 0.0 | 0.0 | 0.0 |
| glioma U251 | 0.0 | 0.0 | 0.1 | 0.0 |
| glioma SF-295 | 0.1 | 0.0 | 0.0 | 0.0 |
| Heart (fetal) | 3.9 | 2.5 | 0.9 | 3.0 |
| Heart | 0.6 | 0.6 | 1.5 | 1.4 |
| Skeletal muscle (fetal) | 11.5 | 18.3 | 2.4 | 6.5 |
| Skeletal muscle | 0.2 | 0.1 | 1.0 | 0.4 |
| Bone marrow | 0.9 | 1.8 | 3.0 | 0.5 |
| Thymus | 5.4 | 7.2 | 4.7 | 9.5 |
| Spleen | 14.3 | 25.9 | 17.4 | 11.9 |
| Lymph node | 7.9 | 15.9 | 29.7 | 11.5 |
| Colorectal | 1.5 | 3.0 | 1.1 | 1.6 |
| Stomach | 3.8 | 4.9 | 2.8 | 1.9 |
| Small intestine | 2.6 | 4.6 | 5.3 | 1.6 |
| Colon ca. SW480 | 0.0 | 0.0 | 0.0 | 0.0 |
| Colon ca.* SW620(SW480 met) | 0.0 | 0.0 | 0.0 | 0.0 |
| Colon ca. HT29 | 0.0 | 0.0 | 0.0 | 0.0 |
| Colon ca. HCT-116 | 0.0 | 0.0 | 0.0 | 0.0 |
| Colon ca. CaCo-2 | 0.0 | 0.1 | 0.0 | 0.0 |
| Colon ca. tissue (OD03866) | 0.7 | 2.0 | 1.1 | 0.7 |
| Colon ca. HCC-2998 | 0.0 | 0.0 | 0.2 | 0.0 |
| Gastric ca.* (liver met) NCI-N87 | 0.2 | 0.1 | 0.0 | 0.3 |
| Bladder | 0.1 | 0.1 | 0.1 | 0.4 |
| Trachea | 1.8 | 3.8 | 3.8 | 0.8 |
| Kidney | 6.3 | 11.8 | 22.2 | 38.2 |
| Kidney (fetal) | 2.0 | 4.5 | 2.8 | 13.5 |
| Renal ca. 786-0 | 0.0 | 0.0 | 0.0 | 0.0 |
| Renal ca. A498 | 0.0 | 0.0 | 0.0 | 0.1 |
| Renal ca. RXF 393 | 0.0 | 0.0 | 0.0 | 0.0 |
| Renal ca. ACHN | 0.0 | 0.0 | 0.0 | 0.0 |
| Renal ca. UO-31 | 0.0 | 0.0 | 0.0 | 0.0 |
| Renal ca. TK-10 | 0.0 | 0.0 | 0.0 | 0.0 |
| Liver | 0.4 | 1.4 | 1.2 | 2.0 |
| Liver (fetal) | 0.4 | 1.3 | 1.0 | 0.6 |
| Liver ca. (hepatoblast) HepG2 | 0.0 | 0.0 | 0.0 | 0.0 |
| Lung | 6.7 | 11.9 | 9.3 | 8.0 |
| Lung (fetal) | 5.1 | 7.1 | 4.7 | 3.3 |
| Lung ca. (small cell) LX-1 | 0.0 | 0.0 | 0.0 | 0.0 |
| Lung ca. (small cell) NCI-H69 | 4.2 | 3.2 | 1.6 | 3.5 |
| Lung ca. (s.cell var.) SHP-77 | 0.0 | 0.1 | 0.0 | 0.0 |
| Lung ca. (large cell)NCI-H460 | 0.0 | 0.0 | 0.0 | 00 |
| Lung ca. (non-sm cell) A549 | 0.0 | 0.1 | 0.0 | 0.4 |
| Lung ca. (non-s.cell) NCI-H23 | 0.5 | 0.3 | 0.4 | 0.7 |
| Lung ca. (non-s.cell) HOP-62 | 0.0 | 0.0 | 0.0 | 0.1 |
| Lung ca.(non-s.cl) NCI-H522 | 6.9 | 5.6 | 1.7 | 11.8 |
| Lung ca. (squani.) SW 900 | 0.0 | 0.0 | 0.0 | 0.0 |
| Lung ca. (squam.) NCI-H596 | 0.6 | 1.6 | 0.7 | 2.2 |
| Mammary gland | 3.0 | 4.0 | 1.8 | 2.3 |
| Breast ca.* (pl.ef) MCF-7 | 0.0 | 0.0 | 0.0 | 0.0 |
| Breast ca.* (pl.ef) MDA-MB-231 | 0.0 | 0.0 | 0.0 | 0.0 |
| Breast ca.*(pl.ef) T47D | 0.0 | 0.0 | 0.0 | 0.0 |
| Breast ca. BT-549 | 0.0 | 0.0 | 0.0 | 0.0 |
| Breast ca. MDA-N | 0.0 | 0.0 | 0.0 | 0.0 |
| Ovary | 2.2 | 1.0 | 0.4 | 0.4 |
| Ovarian ca. OVCAR-3 | 1.2 | 1.1 | 0.8 | 1.5 |
| Ovarian ca. OVCAR-4 | 0.1 | 0.1 | 0.0 | 0.2 |
| Ovarianca.OVCAR-5 | 0.0 | 0.1 | 0.0 | 0.0 |
| Ovarian ca. OVCAR-8 | 1.4 | 2.3 | 0.9 | 1.1 |
| Ovarian ca. IGROV-1 | 0.0 | 0.0 | 0.1 | 0.1 |
| Ovarian ca.* (ascites) SK-OV-3 | 0.0 | 0.0 | 0.0 | 0.1 |
| Uterus | 1.8 | 2.7 | 4.0 | 1.4 |
| Placenta | 3.2 | 4.5 | 2.8 | 0.6 |
| Prostate | 0.3 | 0.6 | 0.8 | 0.4 |
| Prostate ca.* (bone met)PC-3 | 0.0 | 0.0 | 0.0 | 0.0 |
| Testis | 0.4 | 0.3 | 0.4 | 0.1 |
| Melanoma Hs688(A). T | 0.0 | 0.0 | 0.0 | 0.0 |
| Melanoma* (met) Hs688(B).T | 0.0 | 0.0 | 0.0 | 0.0 |
| Melanoma UACC-62 | 0.0 | 0.0 | 0.0 | 0.0 |
| Melanoma M14 | 0.0 | 0.0 | 0.0 | 0.0 |
| Melanoma LOX IMVI | 0.0 | 0.0 | 0.0 | 0.0 |
| Melanoma* (met) SK-MEL-5 | 0.4 | 0.1 | 0.1 | 0.9 |
| Adipose | 2.4 | 4.0 | 3.0 | 8.0 |

**Table BOG. Panel 2D**

| **Tissue Name** | **Rel. Exp.(%) 164024410** | **Rel.Exp.(%) 147164308** | **Tissue Name** | **Rel.Exp.(%) 164024410** | **Rel. Exp.(%) 147164308** |
|---|---|---|---|---|---|
| Normal Colon | 6.8 | 6.3 | Kidney Margin 8120608 | 36.3 | 37.6 |
| CC Well to Mod Diff (ODO3866) | 1.5 | 1.7 | Kidney Cancer 8120613 | 3.0 | 2.3 |
| CC Margin (OD03866) | 3.0 | 2.9 | Kidney Margin 8120614 | 100.0 | 100.0 |
| CC Gr.2 rectosigmoid (OD03868) | 1.3 | 1.2 | Kidney Cancer 9010320 | 11.0 | 12.8 |
| CC Margin (OD03868) | 0.9 | 1.0 | Kidney Margin 9010321 | 24.5 | 22.5 |
| CC Mod Diff (OD03920) | 3.5 | 2.0 | Normal Uterus | 3.4 | 2.6 |
| CC Margin (OD03920) | 3.9 | 2.1 | Uterus Cancer 064011 | 4.8 | 3.7 |
| CC Gr.2 ascend colon (OD03921) | 8.9 | 6.9 | Normal Thyroid | 3.0 | 1.3 |
| CC Margin (ODO3921) | 3.7 | 2.3 | Thyroid Cancer 064010 | 2.4 | 2.0 |
| CC from Partial Hepatectomy (ODO4309) Mets | 5.3 | 3.7 | Thyroid Cancer A302152 | 4.5 | 1.8 |
| Liver Margin (ODO4309) | 3.0 | 3.0 | Thyroid Margin A302153 | 9.5 | 8.0 |
| Colon mets to lung (OD04451-01) | 9.0 | 3.2 | Normal Breast | 14.1 | 12.1 |
| Lung Margin (OD04451-02) | 6.2 | 4.5 | Breast Cancer (OD04566) | 0.7 | 0.5 |
| Normal Prostate 6546-1 | 5.6 | 1.1 | Breast Cancer (OD04590-01) | 2.5 | 3.0 |
| Prostate Cancer (OD04410) | 3.0 | 3.2 | Breast Cancer Meta (OD04590-03) | 16.6 | 19.6 |
| Prostate Margin (OD04410) (OD04410) | 4.8 | 2.9 | Breast Cancer Metastasis (OD04655-05) | 9.4 | 7.3 |
| Prostate Cancer (OD04720-01) | 4.4 | 2.5 | Breast Cancer 064006 | 9.5 | 7.1 |
| Prostate Margin (OD04720-02) | 11.4 | 7.5 | Breast Cancer 1024 | 6.9 | 5.6 |
| Normal Lung 061010 | 19.3 | 17.8 | Breast Cancer 9100266 | 3.4 | 1.7 |
| Lung Met to Muscle (OD04286) | 3.3 | 2.7 | Breast Margin 9100265 | 2.8 | 2.5 |
| Muscle Margin (OD04286) | 2.1 | 0.7 | Breast Cancer A209073 | 8.3 | 5.9 |
| Lung Malignant Cancer (OD03126) | 4.7 | 5.6 | Breast Margin A209073 | 5.6 | 3.7 |
| Lung Margin (OD03126) | 17.9 | 16.4 | Normal Liver | 1.5 | 0.7 |
| Lung Cancer (OD04404) | 8.2 | 8.1 | Liver Cancer 064003 | 1.8 | 2.2 |
| Lung Margin (OD04404) | 13.8 | 9.8 | Liver Cancer 1025 | 2.2 | 1.5 |
| Lung Cancer (OD04565) | 1.9 | 1.4 | Liver Cancer 1026 | 2.0 | 2.1 |
| Lung Margin (OD04565) | 8.0 | 4.2 | Liver Cancer 6004-T | 3.2 | 2.0 |
| Lung Cancer (OD04237-01) | 8.1 | 7.5 | Liver Tissue 6004-N | 1.4 | 1.8 |
| Lung Margin (OD04237-02) | 12.7 | 11.1 | Liver Cancer 6005-T | 3.6 | 2.3 |
| Ocular Mel Met to Liver (OD04310) | 0.6 | 0.9 | Liver Tissue 6005- | 2.9 | 1.5 |
| Liver Margin (OR94310) | 2.4 | 2.1 | Normal Bladder | 3.1 | 2.6 |
| Melanoma Mets to Lung (OD04321) | 4.5 | 3.8 | Bladder Cancer 1023 | 3.3 | 1.4 |
| Lung Margin (OD04321) | 15.2 | 18.7 | Bladder Cancer A302173 | 2.7 | 2.6 |
| Normal Kidney | 54.0 | 45.1 | Bladder Cancer (OD04718-01) | 4.8 | 3.1 |
| Kidney Ca, Nuclear grad 2 (0004338) | 2.2 | 1.6 | Bladder Normal Adjacent (OD04718-03) | 5.7 | 3.8 |
| Kidney Margin (OD04338) | 23.3 | 14.4 | Normal Ovary | 1.2 | 1.7 |
| Kidney Ca Nuclear grade 1/2 (OD04339) | 2.7 | 1.0 | Ovarian Cancer 064008 | 7.2 | 5.0 |
| Kidney Margin (OD04339) | 94.0 | 84.1 | Ovarian Cancer (ODM768-07) | 1.5 | 0.7 |
| Kidney Ca, Clear cell type (OD04340) | 27.0 | 22.8 | Ovary Margin (OD04768-08), | 3.4 | 3.8 |
| Kidney Margin (OD04340) | 54.7 | 52.5 | Normal Stomach | 8.0 | 7.1 |
| Kidney Ca, Nuclear grade 3 (OD04348) | 2.7 | 1.7 | Gastric Cancer 9060358 | 5.1 | 3.6 |
| Kidney Margin (OD04348) | 15.0 | 9.2 | Stomach Margin 9060359 | 8.7 | 7.9 |
| Kidney Cancer (OD04622-01) | 11.5 | 5.6 | Gastric Cancer 9060395 | 7.1 | 6.6 |
| Kidney Margin (OD04622-03) | 17.6 | 9.0 | Stomach Margin 9060394 | 14.5 | 13.3 |
| Kidney Cancer (ODD4450-01) | 0.4 | 0.5 | Gastric Cancer 9060397 | 3.3 | 2.9 |
| Kidney Margin (OD04450-03) | 18.7 | 12.1 | Stomach Margin 9060396 | 7.2 | 5.0 |
| Kidney Cancer 8120607 | 1.7 | 1.0 | Gastric Cancer 064005 | 21.8 | 10.4 |

**Table BOH. Panel 4D**

| **Tissue Name** | **Rel. Exp.(%) Ag1810, Run 162733751** | **Rel. Exp.(%) Ag1811, Run 147164258** | **Rel. Exp.(%) Ag3011, Run 164043491** | **Tissue Name** | **ReL Exp.(%) Ag1810, Run 162733751** | **Rel. Exp.(%) Ag1811, Run 147164258** | **Rel. Exp.(%) Ag3011, Run 164043491** |
|---|---|---|---|---|---|---|---|
| Secondary Th1 act | 42.3 | 40.9 | 30.1 | HUVEC IL-1beta | 3.8 | 4.5 | 4.5 |
| Secondary Th2 act | 37.1 | 43.8 | 32.5 | HUVEC IFN gamma | 18.8 | 15.7 | 13.6 |
| Secondary Tr1 act | 47.6 | 54.0 | 35.8 | HUVEC TNF alpha + IFN gamma | 1.4 | 1.1 | 0.6 |
| Secondary Th1 rest | 8.0 | 11.5 | 7.2 | HUVEC TNF alpha + IL4 | 2.5 | 1.8 | 1.2 |
| Secondary Th2 rest | 13.4 | 11.6 | 11.9 | HUVEC IL-11 | 12.2 | 12.0 | 7.5 |
| Secondary Tr1 rest | 12.8 | 12.8 | 9.3 | Lung Microvascular EC none | 13.1 | 11.3 | 10.2 |
| Primary Th1 act | 21.3 | 18.9 | 14.9 | Lung Microvascular EC TNFalpha+IL-1 beta | 3.8 | 4.4 | 2.5 |
| Primary Th2 act | 29.5 | 39.8 | 26.1 | Microvascular Dermal EC none | 37.1 | 27.4 | 29.3 |
| Primary Tr1 act | 33.9 | 42.3 | 27.7 | Microsvasular Dermal EC TNFalpha + IL-1 beta | 10.7 | 9.5 | 7.4 |
| Primary Th1 rest | 44.8 | 36.1 | 34.6 | Bronchial epithelium TNFalpha + IL1beta | 0.0 | 0.0 | 0.0 |
| Primary Th2 rest | 25.9 | 20.6 | 28.9 | Small airway epithelium none | 0.0 | 0.0 | 0.0 |
| Primary Tr1 rest | 22.2 | 11.0 | 15.1 | Small airway epithelium TNFalpha + IL-1beta | 0.0 | 0.0 | 0.0 |
| CD45RA CD4 lymphocyte act | 11.6 | 11.3 | 8.7 | Coronery artery SMC rest | 0.3 | 0.3 | 0.3 |
| CD45RO CD4 lymphocyte act | 41.8 | 35.1 | 29.3 | Coronery artery SMC TNFalpha + IL-1beta | 0.2 | 0.1 | 0.1 |
| CD8 lymphocyte act | 31.4 | 24.1 | 23.3 | Astrocytes rest | 0.0 | 0.0 | 0.0 |
| Secondary CD8 lymphocyte rest | 42.6 | 25.3 | 31.6 | Astrocytes TNFalpha+IL-1 beta | 0.0 | 0.0 | 0.0 |
| Secondary CD8 lymhocyte act | 28.7 | 17.1 | 20.4 | KU-812 (Basophil) rest | 0.1 | 0.3 | 0.2 |
| CD4 lymphocyte none | 6.2 | 3.6 | 4.6 | KU-812 (Basophil) PMA/ionomycin | 5.6 | 2.9 | 2.9 |
| 2ry Th1/Th2/Tr1_anti-CD95 CH11 | 20.4 | 16.0 | 18.9 | CCD1106 (Keratinocytes) none | 0.0 | 0.1 | 0.0 |
| LAK cells rest | 12.9 | 9.6 | 7.9 | CCD1106 (Keratinocytes) TNFalpha+ IL-1beta | 0.0 | 0.0 | 0.0 |
| LAK cells IL-2 | 38.7 | 28.3 | 30.1 | Liver cirrhosis | 0.6 | 0.5 | 0.8 |
| LAK cells IL-2+IL- | 27.4 | 18.4 | 20.2 | Lupus kidney | 0.3 | 0.5 | 0.4 |
| LAK cells IL- 2+IFN gamma | 42.9 | 29.3 | 26.6 | NCI-H292 none | 0.0 | 0.0 | 0.0 |
| LAK cells IL-2+ IL-18 | 45.7 | 22.7 | 20.3 | NCI-H292 IL-4 | 0.0 | 0.0 | 0.0 |
| LAK cells PMA/ionomycin | 31.6 | 26.6 | 20.6 | NCI-H292 IL-9 | 0.0 | 0.0 | 0.0 |
| NK Cells IL-2 rest | 21.9 | 17.9 | 15.5 | NCI-H292 IL-13 | 0.1 | 0.0 | 0.0 |
| Two Way MLR3 day | 20.3 | 19.9 | 14.4 | NCI-H292 IFN gamma | 0.0 | 0.0 | 0.0 |
| Two Way MLR 5 day | 23.2 | 16.2 | 15.1 | HPAEC none | 13.0 | 14.5 | 9.4 |
| Two Way MLR 7 day | 16.7 | 16.3 | 17.9 | HPAEC TNF alpha+ IL-1beta | 4.6 | 5.6 | 4.4 |
| PBMC rest | 5.9 | 4.1 | 4.5 | Lung fibroblast none | 0.0 | 0.0 | 0.0 |
| PBMCPWM | 79.6 | 54.7 | 84.1 | Lung fibroblast TNF alpha + IL-1 beta | 0.0 | 0.0 | 0.0 |
| PBMC PHA-L | 39.8 | 31.6 | 39.0 | Lung fibroblast IL-4 | 0.0 | 0.0 | 0.0 |
| Ramos (B cell) none | 10.2 | 7.5 | 8.7 | Lung fibroblast IL-9 | 0.0 | 0.0 | 0.0 |
| Ramos (B cell) ionomycin | 20.9 | 12.2 | 13.6 | Lung fibroblast IL-13 | 0.0 | 0.0 | 0.0 |
| B lymphocytes PWM | 96.6 | 100.0 | 100.0 | Lung fibroblast IFN gamma | 0.1 | 0.0 | 0.0 |
| B lymphocytes CD40L and IL-4 | 100.0 | 59.9 | 53.2 | Dermal fibroblast CCD1070 rest | 0.0 | 0.0 | 0.0 |
| EOL-1 dbcAMP | 6.2 | 5.4 | 3.9 | Dermal fibroblast CCD1070 TNF alpha | 27.0 | 22.1 | 22.5 |
| EOL-1 dbcAMP PMA/ionomycin | 61.1 | 463 | 38.4 | Dermal fibroblast CCD1070 IL-I beta | 0.0 | 0.0 | 0.0 |
| Dendritic cells none | 3.1 | 2.2 | 1.7 | Dermal fibroblast IFN gamma | 0.0 | 0.0 | 0.0 |
| Dendritic cells LPS | 5.3 | 3.8 | 4.0 | Dermal fibroblast IL-4 fibroblast | 0.0 | 0.0 | 0.0 |
| Dendritic cells anti- | 1.1 | 0.9 | 1.3 | IBD Colitis 2 | 0.7 | 0.9 | 0.6 |
| Monocytes rest | 1.3 | 0.7 | 0.9 | IBD Crohn's | 0.4 | 0.2 | 0.2 |
| Monocytes LPS | 1.4 | 1.0 | 1.1 | Colon | 8.3 | 6.8 | 5.7 |
| Macrophages rest | 1.8 | 1.4 | 1.7 | Lung | 8.5 | 7.5 | 5.8 |
| Macrophages LPS | 0.7 | 0.7 | 0.4 | Thymus | 21.2 | 27.2 | 21.3 |
| HUVEC none | 11.4 | 7.2 | 7.5 | Kidney | 9.3 | 8.3 | 7.6 |
| HUVEC starved | 17.7 | 13.7 | 10.0 | | | | |

**Table BOI. Panel CNS_1**

| **Tissue Name** | **Rel. Exp.(%) Ag1811, Run 171628390** | **Tissue Name** | **Rel. Exp.(%) Ag1811, Run 171628390** |
|---|---|---|---|
| BA4 Control | 27.7 | BA17PSP | 31.9 |
| BA4 Control2 | 52.1 | BA17PSP2 | 10.1 |
| BA4 Alzheimer's2 | 6.0 | Sub Nigra Control | 36.1 |
| BA4 Parkinson's | 51.4 | Sub Nigra Control2 | 20.3 |
| BA4 Parkinson's2 | 93.3 | Sub Nigra Alzheimer's2 | 5.9 |
| BA4 Hunttngton's | 13.2 | Sub Nigra Parkinson's2 | 32.5 |
| BA4 Huntinton's2 | 7.7 | Sub Nigra Huntington's | 35.1 |
| BA4 PSP | 7.7 | Sub Nigra Huntington's2 | 18.6 |
| BA4 PSP2 | 17.3 | Sub Nigra PSP2 | 5.5 |
| BA4 Depression | 19.2 | Sub Nigra Depression | 8.8 |
| BA4 Depression2 | 11.1 | Sub Nigra Depression2 | 3.0 |
| BA7 Control | 42.6 | Glob Palladus Control | 17.4 |
| BA7 Control2 | 35.6 | Glob Palladus Control2 | 19.3 |
| BA7 Alzheimer's2 | 5.9 | Glob Palladus Alzheimer's | 4.0 |
| BA7 Parkinson's | 21.2 | Glob Palladus Alzheimer's2 | 8.5 |
| BA7 Paricinson's2 | 61.6 | Glob Palladus Parkinson's | 100.0 |
| BA7 Huntington's | 61.1 | Glob Palladus Parkinson's2 | 15.3 |
| BA7 Huntington's2 | 75.3 | Glob Palladus PSP | 3.9 |
| BA7 PSP | 29.3 | Glob Palladus PSP2 | 8.4 |
| BA7 PSP2 | 17.6 | Glob Palladus Depression | 4.1 |
| BA7 Depression | 13.6 | Temp Pole Control | 14.0 |
| BA9 Control | 21.2 | Temp Pole Control2 | 70.7 |
| BA9 Control2 | 90.1 | Temp Pole Alzheimer's | 3.8 |
| BA9 Alzheimer's | 4.0 | Temp Pole Alzheimer's2 | 5.4 |
| BA9 Alzheimer's2 | 12.6 | Temp Pole Parkinson's | 32.8 |
| BA9 Parkinson's | 40.9 | Temp Pole Parkinson's2 | 28.1 |
| BA9 Parkinson's2 | 61.1 | Temp Pole Huntington's | 38.4 |
| BA9 Huntington's | 67.4 | Temp Pole PSP | 3.8 |
| BA9 Huntington's2 | 17.8 | Temp Pole PSP2 | 2.7 |
| BA9 PSP | 13.8 | Temp Pole Depression2 | 8.9 |
| BA9 PSP2 | 5.1 | Cing Gyr Control | 60.7 |
| BA9 Depression | 14.0 | Cing Gyr Control2 | 29.7 |
| BA9 Depression2 | 14.5 | Cing Gyr Alzheimer's | 16.7 |
| BA17 Control | 61.6 | Cing Gyr Alzheimer's2 | 5.9 |
| BA17 Control2 | 56.3 | Cing Gyr Parkinsor's | 43.5 |
| BA17 Alzheimer's2 | 9.0 | Cing Gyr Parkinson's2 | 45.7 |
| BA17 Parkinson's | 49.3 | Cing Gyr Huntington's | 78.5 |
| BA17 Parkinson's2 | 74.7 | Cing Gyr Huntington's2 | 28.5 |
| BA17 Huntington's | 44.4 | Cing Gyr PSP | 13.1 |
| BA17 Huntington's2 | 42.9 | Cing Gyr PSP2 | 6.1 |
| BA17 Depression | 14.5 | Cing Gyr. Depression | 7.7 |
| BA17 Depression2 | 37.9 | Cing Gyr Depression2 | 14.1 |

**AI_comprehensive panel_v1.0 Summary:** Ag3011 CG56761-01 gene expression is upregulated in arthritis tissue as compared to normal joint tissue. It is also expressed in matched control gut tissue and at low levels in pulmonary tissue. The consistent induction of this transcript in arthritic tissue suggests that the protein encoded for by this transcript may be involved in the pathological processes associated with arthritis and may serve as a relevant target for therapeutic intervention.

**CNS_neurodegeneration_v1.0 Summary:** Ag1810/Ag1811/Ag3011 Results from three experiments using different probe/primer sets are in excellent agreement. This panel confirms the expression of the CG56761-01 gene at low levels in the brains of an independent group of individuals. However, no differential expression of this gene was detected between Alzheimer's diseased postmortem brains and those of non-demented controls in this experiment. Please see Panel 1.3D for a discussion of the potential utility of this gene in treatment of central nervous system disorders.

**Panel 1.3D Summary:** Ag1810/Ag1811/Ag3011 Three experiments with three different probe and primer sets produce results that are in excellent agreement. The CG56761-01 gene is expressed at moderate to high levels in all regions of the central nervous system examined, including amygdala, hippocampus, substantia nigra, thalamus, cerebellum, cerebral cortex, and spinal cord. Thus, expression of this gene may be used to distinguish brain from the other samples on this panel. In addition, this gene may play a role in central nervous system disorders such as Alzheimer's disease, Parkinson's disease, epilepsy, multiple sclerosis, schizophrenia and depression. The CG56761-01 gene encodes a protein that contains ankyrin repeats.

Interestingly, expression of this gene appears to be highly down-regulated in CNS cancer cell lines. Thus, modulation of the activity of this gene product may be of benefit in the treatment of cancers of the central nervous system.

**Panel 2D Summary:** Ag1810/Ag1811 Results from two experiments using different probe/primer sets are in excellent agreement. Expression of the CG56761-01 gene is highest in a sample of normal kidney tissue (CT = 27). Expression of this gene is upregulated in matched normal kidney, lung, thyroid, ovary and stomach tissues when compared to the adjacent tumor samples. Thus, expression of this gene may be used to distinguish normal kidney, lung, thyroid, ovary and stomach from cancers of these tissues. In addition, therapeutic modulation of the activity of this gene or its protein product, using small molecule drugs, antibodies or protein therapeutics, may be of use in the treatment of these cancers.

**Panel 4D Summary:** Ag1810/Ag1811/Ag3011 Three experiments with two different probe and primer sets produce results that are in excellent agreement. High expression of CG56761-01 is detected in samples derived from PWM and CD40L/IL4 treated B lymphocytes (CTs=26), PWM (CT=26.4) and PHLA (CT=27.4) treated PMBC. Thus, expression of this gene could be used to differentiate between these samples and other samples on this panel. Expression of this transcript in B cells suggests that this gene may be involved in rheumatic disease including rheumatoid arthritis, lupus, osteoarthritis, and hyperproliferative B cell disorders.

**Panel CNS_1 Summary:** Ae1811 This panel confirms the expression of CG56761-01 gene at low levels in the brains of an independent group of individuals. Please see Panel 1.3D for a discussion of the potential utility of this gene in treatment of central nervous system disorders.

### NOV73

Expression of NOV73/CG57313-01 was assessed using the primer-probe set Ag3185, described in Table BAA.

**Table BAA. Probe Name Ag3185**

| **Primers** | **Sequences** | **Length** | **Start Position** | **SEQ ID NO** |
|---|---|---|---|---|
| Forward | 5'-ccatcttctgtgggacagttac-3' | 22 | 779 | 485 |
| Probe | TET-5'-catgtatatacagccaggaaacagtcca-3'-TAMRA | 28 | 804 | 486 |
| Reverse | 5'-agaagtttgccctcattctgat-3' | 22 | 833 | 487 |

**CNS_neurodegeneration_v1.0 Summary:** Ag3185 Expression of this gene is low/undetectable (CTs > 35) across all of the samples on this panel (data not shown).

**Panel 1.3D Summary:** Ag3185 Expression of this gene is low/undetectable (CTs > 35) across all of the samples on this panel (data not shown).

**Panel 4D Summary:** Ag3185 Expression of this gene is low/undetectable (CTs > 35) across all of the samples on this panel (data not shown).

### NOV74

Expression of NOV74/CG57315-01 was assessed using the primer-probe set Ag3186, described in Table BBA. Results of the RTQ-PCR runs are shown in Tables BBB, and BBC.

**Table BBA. Probe Name Ag3186**

| **Primers** | **Sequences** | **Length** | **Start Position** | **SEQ ID NO** |
|---|---|---|---|---|
| Forward | 5'-gagttgcattgtgttgttgttg-3' | 22 | 610 | 488 |
| Probe | TET-5'-tcattttccttgcatcacttcttctca-3'-TAMRA | 27 | 638 | 489 |
| Reverese | 5'-caatgaagccatatgacacaag-3' | 22 | 667 | 490 |

**Table BBB. CNS_neurodegeneration_v1.0**

| **Tissue Name** | **Rel. Exp.(%) Ag3186, Run 209859516** | **Tissue Name** | **Rel. Exp. (%) Ag3186, Run 209859516** |
|---|---|---|---|
| AD 1 Hippo | 32.5 | Control (Path) 3 Temporal Ctx | 0.0 |
| AD 2 Hippo | 100.0 | Control (Path) 4 Temporal | 0.0 |
| AD 3 Hippo | 29.9 | AD 1 Occipital Ctx | 0.0 |
| AD 4 Hippo | 38.7 | AD 2 Occipital Ctx (Missing) | 0.0 |
| AD 5 hippo | 0.0 | AD3 0ccipital Ctx | 0.0 |
| AD 6 Hippo | 76.8 | AD 4 Occipital Ctx | 5.4 |
| Control 2 Hippo | 55.5 | AD 5 Oceipital Ctx | 0.0 |
| Control 4 Hippo | 92.7 | AD 6 Occipital Ctx | 0.0 |
| Control (Path) 3 Hippo | 32.8 | Control 1 Occipital Ctx | 0.0 |
| AD 1 Temporal Ctx | 0.0 | Control 2 Occipital Ctx | 0.0 |
| AD 2 Temporal Ctx | 13.2 | Control 3 Occipital Ctx | 7.6 |
| AD 3 Temporal Ctx | 0.0 | Control 4 Occipital Ctx | 0.0 |
| AD 4 Temporal Ctx | 0.0 | Control (Path) 1 Occipital Ctx | 42.0 |
| AD 5 Inf Temporal Ctx | 0.0 | Control (Path) 2 Occipital Ctx | 0.0 |
| AD 5 Sup Temporal Ctx | 87.1 | Control (Path) 3 Occipital Ctx | 0.0 |
| AD 6 Inf Temporal Ctx | 0.0 | Control (Path) 4 Occipital | 0.0 |
| AD 6 Sup Temporal Ctx | 0.0 | Control 1 Parietal Ctx | 0.0 |
| Control 1 Temporal Ctx | 0.0 | Control Parietal Ctx | 0.0 |
| Control 2 Temporal Ctx | 0.0 | Control 3 Parietal Ctx | 0.0 |
| Control 3 Temporal Ctx | 11.2 | Control (Path) 1 Parietal Ctx | 7.0 |
| Control 4 Temporal Ctx | 0.0 | Control (Path) 2 Parietal Ctx | 0.0 |
| Control (Path) 1 Temporal Ctx | 16.2 | Control (Path) 3 Parietal Ctx | 0.0 |
| Control (Path) 2 Temporal Ctx | 0.0 | Control (Path) 4 Parietal Ctx | 0.0 |

**Table BBC. Panel 1.3D**

| **Tissue Name** | **Rel. Exp.(%) Ag3186, Run 167994623** | **Tissue Name** | **Rel. Exp.(%) Ag3186, Run 167994623** |
|---|---|---|---|
| Liver adenocarcinoma | 20.4 | Kidney (fetal) | 0.0 |
| Pancreas | 0.0 | Renal ca. 786-0 | 0.0 |
| Pancreatic ca. CAPAN 2 | 0.0 | Renal ca. A498 | 0.0 |
| Adrenal gland | 0.0 | Renal ca. RXF 393 | 0.0 |
| Thyroid | 0.0 | Renal ca. ACHN | 0.0 |
| Salivary gland | 0.0 | Renal ca. UO-31 | 0.0 |
| Pituitary gland | 0.0 | Renal ca. TK-10 | 0.0 |
| Brain (fetal) | 11.0 | Liver | 0.0 |
| Brain (whole) | 0.0 | Liver (fetal) | 0.0 |
| Brain (amygdala) | 3.6 | Liver ca. (hepatoblast) HepG2 | 0.0 |
| Brain (cerebellum) | 0.0 | Lung | 0.0 |
| Brain (hippocampus) | 15.4 | Lung (fetal) | 0.0 |
| Brain (substantia nigra) | 0.0 | Lung ca. (small cell) LX-1 | 0.0 |
| Brain (thalamus) | 14.7 | Lung ca. (small cell) NCI- H69 | 0.0 |
| Cerebral Cortex | 0.0 | Lung ca. (s.cell var.) SHP- 77 | 0.0 |
| Spinal cord | 38.2 | Lung ca. (large cell)NCI-H460 | 0.0 |
| glio/astro U87-MG | 0.0 | Lung ca. (non-sm. cell) A549 | 9.7 |
| glio/astro-U-118-MG | 0.0 | Lung ca.(non-s.cell) NCI-H23 | 36.3 |
| astrocytoma SW1783 | 0.0 | Lung ca.(non-s.cell) HOP-62 | 9.7 |
| neuro*; met SK-N-AS | 0.0 | Lung ca. (non-s.cl) NCI-H522 | 0.0 |
| astrocytoma SF-539 | 15.6 | Lung ca. (squam.) SW 900 | 0.0 |
| astrocytoma SNB-75 | 0.0 | Lung ca. (rquam.) NCI-H596 | 0.0 |
| glioma SNB-19 | 0.0 | Mammary gland | 0.0 |
| glioma U251 | 0.0 | Breast ca.* (pl.ef) MCF-7 | 21.6 |
| glioma SF-295 | 0.0 | Breast ca.* (pl.ef) MDA-MB-231 | 0.0 |
| Heart (fetal) | 0.0 | Breast ca.* (pl.ef) T47D | 0.0 |
| Heart | 0.0 | Breast ca. BT-549 | 9.0 |
| Skeletal muscle (fetal) | 0.0 | Breast ca. MDA-N | 0.0 |
| Skeletal muscle | 0.0 | Ovary | 0.0 |
| Bone marrow | 0.0 | Ovarian ca. OVCAR-3 | 0.0 |
| Thymus | 0.0 | Ovarian ca.OVCAR-4 | 0.0 |
| Spleen | 0.0 | Ovarian ca.OVCAR-5 | 8.0 |
| Lymph node | 0.0 | Ovarian ca. OVCAR-8 | 0.0 |
| Colorectal | 24.7 | Ovarian ca. IGROV-1 | 0.0 |
| Stomach | 0.0 | ovarian OV-3 ca.* (ascites) SK- | 100.0 |
| Small intestine | 0.0 | Uterus | 0.0 |
| Colon ca. SW480 | 16.2 | Placenta | 0.0 |
| Colon ca.* SW620(SW480 met) | 0.0 | Prostate | 0.0 |
| Colon ca. HT29 | 0.0 | Prostate ca*(bone met)PC- | 0.0 |
| Colon ca. HCT-116 | 0.0 | Testis | 32.1 |
| Colon ca. CaCo-2 | 0.0 | Melanoma Hs688(A).T | 0.0 |
| Colon ca. tissue(OD03866) | 0.0 | Melanoma* (met) Hs688(B).T | 0.0 |
| Colon ca. HCC-2998 | 0.0 | Melanoma UACC-62 | 0.0 |
| Gastric ca.* (liver met) NCI-N87 | 0.0 | Melanoma M14 | 0.0 |
| Bladder | 21.6 | Melanoma LOX IMVI | 0.0 |
| Trachea | 0.0 | Melanoma* (met) SK-MEL-5 | 0.0 |
| Kidney | 0.0 | Adipose | 0.0 |

**CNS_neurodegeneration_v1.0 Summary**: Ag3186 This panel demonstrates the expression of the CG57315-01 gene at low levels in the brains of a group of several individuals. However, no differential expression of this gene was detected between Alzheimer's diseased postmortem brains and those of non-demented controls in this experiment. The CG57315-01 gene encodes a putative GPCR. Several neurotransmitter receptors are GPCRs, including the dopamine receptor family, the serotonin receptor family, the GABAB receptor, muscarinic acetylcholine receptors, and others; thus this GPCR may represent,a novel neurotransmitter receptor. Targeting various neurotransmitter receptors (dopamine, serotonin) has proven to be an effective therapy in psychiatric illnesses such as schizophrenia, bipolar disorder, and depression. Furthermore, the cerebral cortex and hippocampus are regions of the brain that are known to be involved in Alzheimer's disease, seizure disorders, and in the normal process of memory formation. Therefore, therapeutic modulation of this gene or its protein product may be beneficial in the treatment of one or more of these diseases, as may stimulation and/or blockade of the receptor coded for by the gene.

**Panel 1.3D Summary:** Ag3186 Low levels of expression of the CG57315-01 gene are seen exclusively in an ovarian cancer sample (CT = 34.7). Therefore, expression of this gene may be used to distinguish ovarian cancer cell lines from the other samples on this panel. Furthermore, therapeutic modulation of the activity of the GPCR encoded by this gene may be beneficial in the treatment of ovarian cancer.

**Panel 4D Summary:** Ag3186 Expression of CG57315-01 is low/undetectable (CTs > 35) across all of the samples on this panel (data not shown).

### NOV75

Expression of NOV75/CG57317-01 was assessed using the primer-probe set Ag3187, described in Table BCA. Results of the RTQ-PCR runs are shown in Tables BCB.

**Table BCA. Probe Name Ag3187**

| **Primers** | **Sequences** | **Length** | **Start Position** | **SEQ ID NO** |
|---|---|---|---|---|
| Forward | 5'-tttgtggctgttttgatatcct-3' | 22 | 172 | 491 |
| Probe | TET-5'-tctccttacccttgtgggaaatacag-3'-TAMRA | 26 | 195 | 492 |
| Reverse | 5'-gggtctacagagcagatcagaa-3' | 22 | 227 | 493 |

**Table BCB. Panel 1.3D**

| **Tissue Name** | **Rel. Exp.(%) Ag3187, Run 167994630** | **Tissue Name** | **Rel. Exp.(%) Ag3187, Run 167994630** |
|---|---|---|---|
| Liver adenocarcinoma | 0.0 | Kidney (fetal) | 0.0 |
| Pancreas | 0.0 | Renal ca. 786-0 | 0.0 |
| Pancreatic ca. CAPAN 2 | 0.0 | Renal ca. A498 | 0.0 |
| Adrenal gland | 0.0 | Renal ca. RXF 393 | 0.0 |
| Thyroid | 0.0 | Renal ca. ACHN | 0.0 |
| Salivary gland | 0.0 | Renal ca. UO-31 | 0.0 |
| Pituitary gland | 0.0 | Renal ca.TK-10 | 0.0 |
| Brain (fetal) | 0.0 | Liver | 0.0 |
| Brain (whole) | 0.0 | Liver (fetal) | 0.0 |
| Brain (amygdala) | 0.0 | Liver ca. (hepatoblast) HepG2 | 0.0 |
| Brain (cerebellum) | 0.0 | Lung | 0.0 |
| Brain (hippocampus) | 8.1 | Lung (fetal) | 0.0 |
| Brain (substantia nigra) | 0.0 | Lung ca. (small cell) LX-1 | 0.0 |
| Brain (thalamus) | 0.0 | Lung ca. (small cell) NCI-H69 | 0.0 |
| Cerebral Cortex | 0.0 | Lung ca. (s.cell var.) SHP-77 | 0.0 |
| Spinal cord | 0.0 | Lung ca. (large cell)NCI-H460 | 0.0 |
| glio/astro U87-MG | 0.0 | Lung ca.(non-sm. cell) A549 | 0.0 |
| glio/astro U-118-MG | 0.0 | Lung ca. (non-s.cell) NCI-H23 | 27.5 |
| astrocytoma SW1783 | 5.9 | Lung ca. (non-s.cell) HOP- | 0.0 |
| neuro*;met SK-N-AS | 0.0 | Lung ca. (non-s.cl) NCI-H522 | 5.9 |
| astrocytoma SF-539 | 0.0 | Lung ca. (squam.) SW 900 | 0.0 |
| astrocytoma SNB-75 | 0.0 | Lung ca. (squaim) NCI-H596 | 0.0 |
| glioma SNB-19 | 0.0 | Mammary gland | 0.0 |
| glioma U251 | 0.0 | Breast ca.* (pl.ef) MCF-7 | 0.0 |
| glioma SF-295 | 0.0 | Breast ca.* (pi-ef) MDA-MB-231 | 0.0 |
| Heart (fetal) | 0.0 | Breast ca.* (pi.ef) T47D | 0.0 |
| Heart | 0.0 | Breast ca. BT-549 | 0.0 |
| Skeletal muscle (fetal) | 0.0 | Breast ca. MDA-N | 0.0 |
| Skeletal muscle | 0.0 | Ovary | 0.0 |
| Bone marrow | 0.0 | Ovarian ca. OVCAR-3 | 0.0 |
| Thymus | 0.0 | Ovarian ca. OVCAR-4 | 0.0 |
| Spleen | 9.3 | Ovarian ca. OVCAR-5 | 0.0 |
| Lymph node | 0.0 | Ovarian ca. OVCAR-8 | 0.0 |
| Colorectal | 16.0 | Ovarian ca. IGROV-1 | 0.0 |
| Stomach | 0.0 | Ovarian ca.* (ascites) SK-OV-3 | 100.0 |
| Small intestine | 0.0 | Uterus | 0.0 |
| Colon ca. SW480 | 0.0 | Placenta | 0.0 |
| Colon ca.* SW620(SW480 met) | 0.0 | Prostate | 0.0 |
| Colon ca. HT29 | 0.0 | Prostate ca.* (bone met)PC-3 | 0.0 |
| Colon ca. HCT-116 | 0.0 | Testis | 0.0 |
| Colon ca. CaCo-2 | 0.0 | Melanoma Hs688(A).T | 0.0 |
| Colon ca. tissue(OD03866) | 0.0 | Melanoma* (met) Hs688(B).T | 0.0 |
| Colon ca HCC-2998 | 0.0 | Melanoma UACC-62 | 0.0 |
| Gastric ca.* (liver met) NCI-N87 | 0.0 | Melanoma M14 | 0.0 |
| Bladder | 0.0 | Melanoma LOX IMVI | 0.0 |
| Trachea | 0.0 | Melanoma* (met) SK-MEL-5 | 0.0 |
| Kidney | 0.0 | Adipose | 0.0 |

**Panel 1.3D Summary:** Ag3187 Significant expression of CG57317-01 gene is seen exclusively in an ovarian cancer sample (CT = 34.12). Therefore, expression of this gene may be used to distinguish ovarian cancers from the other samples on this panel. Furthermore, therapeutic modulation of the activity of the GPCR encoded by this gene may be beneficial in the treatment of ovarian cancer.

**Panel 4D Summary:** Ag3187 Expression of CG57317-01 gene is low/undetectable (CTs > 35) across all of the samples on this panel (data not shown).

### NOV77

Expression of NOV77/CG57419-01 was assessed using the primer-probe set Ag3233, described in Table BDA.

**Table BDA. Probe Name Ag3233**

| **Primers** | **Sequences** | **Length** | **Start Position** | **SEQ ID NO** |
|---|---|---|---|---|
| Forward | 5'-tggccatgtatttggtgactat-3' | 22 | 128 | 494 |
| Probe | TET-5'-aggaaacaccctcattcttcttctga-3'-TAMRA | 26 | 153 | 495 |
| Reverse | 5'-tatgaagcctgttgtccagtct-3' | 22 | 181 | 496 |

**CNS_neurodegeneration_v1.0Summary:** Ag3233 Expression of this gene is low/undetectable (CTs > 35) across all of the samples on this panel (data not shown).

**General_screening_panel-v1.4 Summary:** Ag3233 Expression of this gene is low/undetectable (CTs > 35) across all of the samples on this panel (data not shown).

**Panel 2.2 Summary:** Ag3233 Expression of this gene is low/undetectable (CTs > 35) across all of the samples on this panel (data not shown).

### NOV78

Expression of NOV78/CG57425-01 was assessed using the primer-probe sets Ag1703 and Ag1747, described in Tables BEA and BEB. Results of the RTQ-PCR runs are shown in Table BEC.

**Table BEA. Probe Name Ag1703**

| **Primers** | **Sequences** | **Length** | **Start Position** | **SEQ ID NO** |
|---|---|---|---|---|
| Forward | 5'-gaggtgctttcaacaaaggaat-3' | 22 | 108 | 497 |
| Probe | TET-5'-tcggttactcccttactgaatcccttca-3'-TAMRA | 28 | 64 | 498 |
| Reverse | 5'-gcttgtttcacttgctgatttc-3' | 22 | 30 | 499 |

**Table BEB. Probe Name Ag1747**

| **Primers** | **Sequences** | **Length** | **Start Position** | **SEQ ID NO** |
|---|---|---|---|---|
| Forward | 5'-gaggtgctttcaacaaaggaat-3' | 22 | 108 | 500 |
| Probe | TET-5'-tcggttactcccttactgaatcccttca-3'-TAMRA | 28 | 64 | 501 |
| Reverse | 5'-gcttgtttcacttgctgatttc-3' | 22 | 30 | 502 |

**Table BEC. Panel 4D**

| **Tissue Name** | **Rel. Exp.(%) Ag1747, Run 165808155** | **Tissue Name** | **Rel. Exp.(%) Ag1747, Run 165808155** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 5.0 | HUVEC TNF alpha + IFN gamma | 0.0 |
| Secondary Th1 rest | 0.0 | HUVEC TNF alpha + IL4 | 0.0 |
| Secondary Th2 rest | 0.0 | HUVEC IL-11 | 0.0 |
| Secondary Tr1 rest | 2.6 | Lung Microvascular EC none | 0.0 |
| Primary Th1 act | 0.0 | Lung Microvascular EC TNFalpha + IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1 beta | 0.0 |
| Primary Th1 rest | 10.3 | Bronchial epithelium TNFalpha + IL1 beta | 0.0 |
| Primary Th2 rest | 0.0 | Small airway epithelium none | 0.0 |
| Primary Tr1 rest | 0.0 | Small airway epithelium TNFalpha + IL-1 beta | 0.0 |
| CD45RA CD4 lymphocyte act | 0.0 | Coronery artery SMC rest | 0.0 |
| CD45RO CD4 lymphocyte act | 3.0 | Coronery artery SMC TNFalpha+ IL-1beta | 0.0 |
| CD8 lymphocyte act | 10.2 | Astrocytes rest | 0.0 |
| Secondary CD8 lymphocyte rest | 7.2 | Astrocytes TNFalpha+IL-1beta | 0.0 |
| Secondary CD8 lymphocyte act | 0.0 | KU-812 (Basophil) rest | 0.0 |
| CD4 act lymphocyte none | 0.0 | KU-812 (Basophil) PMA/ionomycin | 0.0 |
| 2ry Th1/Th2/Tr1_anti-CD95 CH11 | 2.9 | CCD1106(Keratinocytes)none | 0.0 |
| LAK cells rest | 4.1 | CCD1106 (Keratinocytes) TNFalpha + IL-1 beta | 0.0 |
| LAK cells IL-2 | 0.0 | Liver cirrhosis | 100.0 |
| LAK cells IL-2+IL-12 | 7.5 | Lupus kidney | 0.0 |
| LAK cells IL-2+IFN gamma | 13.1 | NCl-H292 none | 0.0 |
| LAK cells IL-2 + IL-18 | 0.0 | NCI-H292 IL-4 | 0.0 |
| LAK cells PMA/ionomycin | 15.3 | NCI-H292 IL-9 | 0.0 |
| NK Cells IL-2 rest | 0.0 | NCI-H292 IL-13 | 0.0 |
| Two Way MLR 3 day | 0.0 | NCI-H292 IFN gamma | 0.0 |
| Two Way MLR 5 day | 5.9 | HPAEC none | 0.0 |
| Two Way MLR 7 day | 0.0 | HPAEC TNF alpha.+ IL-1 beta | 0.0 |
| PBMC rest | 0.0 | Lung fibroblast none | 0.0 |
| PBMC PWM | 10.0 | Lung fibroblast TNF alpha + IL-1 beta | 0.0 |
| PBMC PHA-L | 6.0 | Lung fibroblast IL-4 | 0.0 |
| Ramos (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 0.0 | Lung fibroblast IFN gamma | 0.0 |
| B lymphocytes CD40L and LL-4 | 8.2 | Dermal fibroblast CCD1070 rest | 0.0 |
| EOL-1 dbcAMP | 0.0 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendritic cells none | 4.2 | Dermal fibroblast IFN gamma | 0.0 |
| Dendritic cells LPS | 0.0 | Dermal fibroblast IL-4 | 0.0 |
| Dendritic cells anti-CD40 | 0.0 | IBD Colitis 2 | 12.4 |
| Monocytes rest | 0.0 | IBD Crohn's | 7.9 |
| Monocytes LPS | 0.0 | Colon | 0.0 |
| Macrophages rest | 0.0 | Lung | 0.0 |
| Macrophages LPS | 0.0 | Thymus | 0.0 |
| HUVEC none | 0.0 | Kidney | 0.0 |
| HUVEC starved | 0.0 | | |

**Panel 1.3D** Summary: Ag1703 Expression of the CG57425-01 gene is low/undetectable (CTs > 35) across all of the samples on this panel due to a probable probe or chemistry failure (data not shown). Ag1747 Expression of this gene is low/undetectable (CTs > 35) across all of the samples on this panel (data not shown).

**Panel 2.2 Summary:** Ag1703 Expression of the CG57425-01 gene is low/undetectable (CTs > 35) across all of the samples on this panel due to a probable probe or chemistry failure (data not shown). Ag1747 Expression of this gene is low/undetectable (CTs > 35) across all of the samples on this panel (data not shown).

**Panel 4D Summary:** Ag1703 Expression of CG57425-O1 gene is low/undetectable (CTs > 35) across all of the samples on this panel due to a probable probe or chemistry failure (data not shown). Ag1747 Low levels of expression of this gene are detected in a liver cirrhosis sample (CT = 33.1). Furthermore, expression of this gene is not detected in normal liver in Panel 1.3D, suggesting that its expression is unique to liver cirrhosis. This gene encodes a putative GPCR; therefore, antibodies or small molecule therapeutics could reduce or inhibit fibrosis that occurs in liver cirrhosis. In addition, antibodies to this putative GPCR could also be used for the diagnosis of liver cirrhosis.

### NOV80

Expression of gene CG56766-01 was assessed using the primer-probe set Ag3013, described in Table BPA.

**Table BPA. Probe Name Ag3013**

| **Primers** | **Sequences** | **Length** | **Start Position** | **SEQ ID NO** |
|---|---|---|---|---|
| Forward | 5'-tacactgttgtcacgccaataa-3' | 22 | 888 | 503 |
| Probe | TET-5'-ccctttgatttactgcctgaggaaca-3'-TAMRA | 26 | 914 | 504 |
| Reverse | 5'-ttttcaaggcgtccttaaattc-3' | 22 | 942 | 505 |

**CNS_neurodegeneration_v1.0 Summary:** Ag3013 Expression of the CG56766-01 gene is low/undetectable (CTs > 35) across all of the samples on this panel (data not shown).

**Panel 1.3D Summary:** Ag3013 Expression of this gene is low/undetectable (CTs > 35) across all of the samples on this panel (data not shown). The CG56766-01 gene encodes a G protein-coupled receptor (GPCR), a type of cell surface receptor involved in signal transduction. This gene product is most similar to members of the odorant receptor subfamily of GPCRs. Based on analogy to other odorant receptor genes, we predict that expression of this gene may be highest in nasal epithelium, a sample not represented on this panel.

**Panel 4D** Summary: Ag3013 Expression of the CG56766-01 gene is low/undetectable (CTs > 35) across all of the samples on this panel (data not shown).

### NOV81

Expression of NOV81A/CG57847-01 and NOV81B/CG57847-02 was assessed using the primer-probe set Ag3347, described in Table BQA. Results of the RTQ-PCRruns are shown in Table BQB. Please note that CG57847-02 represents a full-length physical clone of the CG57847-01 gene, validating the prediction of the gene sequence.

**Table BQA. Probe Name Ag3347**

| **Primers** | **Sequences** | **Length** | **Start Position** | **SEQ ID NO** |
|---|---|---|---|---|
| Forward | 5'-attgatttggtttggctacttg-3' | 22 | 866 | 506 |
| Probe | TET-5'-aactctacatttaatccaatggtttatgca-3'-TAMRA | 30 | 888 | 507 |
| Reverse | 5'-accaaacagcatcatcttcagt-3' | 22 | 944 | 508 |

**Table BOB. General_screening_panel_v1.4**

| **Tissue Name** | **Rel. Exp.(%) Ag3347, Run 215620630** | **Tissue Name** | **Rel. Exp.(%) Ag3347, Run 215620630** |
|---|---|---|---|
| Adipose | 0.0 | Renal ca. TK-10 | 0.0 |
| Melanoma* Hs688(A).T | 0.0 | 'Bladder | 0.0 |
| Melanoma* Hs688(B).T | 0.0 | Gastric ca. (liver met) NCI-NB7 | 0.0 |
| Melanoma* M14 | 0.0 | Gastric ea.KATOm | 0.0 |
| Melanoma* LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| Testis Pool | 0.0 | Colon ca. HT29 | 0.0 |
| Prostate ca.*(bone met) PC-3 | 2.3 | Colon ca. HCT-116 | 1.5 |
| Prostate Pool | 0.0 | Colon ca. CaCo-2 | 1.8 |
| Placenta | 0.0 | Colon cancer tissue | 0.0 |
| Uterus Pool | 0.0 | Colon ca. SW1116 | 0.0 |
| Ovarian ca. OVCAR-3 | 0.0 | Colon ca. Colo-205 | 0.0 |
| Ovarian ca. SK-OV-3 | 7.4 | Colon ca. SW-48 | 0.0 |
| Ovarian ca. OVCAR-4 | 0.0 | Colon Pool | 4.1 |
| Ovarian ca. OVCAR-5 | 0.0 | Small Intestine Pool | 0.0 |
| Ovarian ca. IGROV-1 | 0.0 | Stomach Pool | 3.7 |
| Ovarian ca. OVCAR-8 | 0.0 | Bone Marrow Pool | 0.0 |
| Ovary | 0.0 | Fetal Heart | 0.0 |
| Breast ca. MCF-7 | 0.0 | Heart Pool | 2.7 |
| Breast ca. MDA-MB-231 | 0.0 | Lymph Node Pool | 3.3 |
| Breast ca. BT 549 | 0.0 | Fetal Skeletal Muscle | 0.0 |
| Breast ca. T47D | 0.0 | Skeletal Muscle Pool | 0.0 |
| Breast ca. MDA-N | 0.0 | Spleen Pool | 4.5 |
| Breast Pool | 0.0 | Thymus Pool | 0.0 |
| Trachea | 11.0 | CNS cancer(glio/astro)U87-MG | 0.0 |
| Lung | 1.7 | CNS cancer (glio/astro)U-118-MG | 4.0 |
| Fetal Lung | 5.8 | CNS cancer (neuro;met) SK-N-AS | 0.0 |
| Lung ca. NCI-N417 | 0.0 | CNS cancer (astro) SF-539 | 0.0 |
| Lung ca.LX-1 | 0.0 | CNS cancer (astro) SNB-75 | 0.0 |
| Lung ca. NCI-H146 | 75.3 | CNS cancer (glio) SNB-19 | 0.0 |
| Lung ca. SHP-77 | 100.0 | CNS cancer (glio) SF-295 | 0.0 |
| Lung ca. A549 | 0.0 | Brain (Amygdala) Pool | 1.9 |
| Lung ca. NCI-H526 | 0.0 | Brain (cerebellum) | 0.0 |
| Lung ca. NCI-H23 | 21.9 | Brain (fetal) | 0.0 |
| Lung ca. NCI-H460 | 8.2 | Brain (Hippocampus) Pool | 0.0 |
| Lung ca. HOP-62 | 0.0 | Cerebral Cortex Pool | 0.0 |
| Lung ca. NCI-H522 | 0.0 | Brain (Substantia nigra) Pool | 0.0 |
| Liver | 0.0 | Brain (Thalamus) Pool | 0.0 |
| Fetal Liver | 1.5 | Brain (whole) | 0.0 |
| Liver ca. HepG2 | 0.0 | Spinal Cord Pool | 0.0 |
| Kidney Pool | 0.0 | Adrenal Gland | 0.0 |
| Fetal Kidney | 6.1 | Pituitary gland Pool | 0.0 |
| Renal ca. 786-0 | 0.0 | Salivary Gland | 4.7 |
| Renal ca. A498 | 0.0 | Thyroid (female) | 0.0 |
| Renal.ca. ACHN | 0.0 | Pancreatic ca.CAPAN2 | 0.0 |
| Renal ca. U0-31 | 0.0 | Pancreas Pool | 5.3 |

**CNS_neurodegeneration_v1.0 Summary:** Ag3347 Results from one experiment with the CG57847-01 gene are not included. The amp plot indicates that there were experimental difficulties with this run.

**General_screening_panel_v1.4 Summary:** Ag3347 Expression of the CG57847-01 gene is restricted to two lung cancer cell lines. Therefore, expression of this gene may be used to distinguish lung cancer cell lines from the other samples on this panel. In addition, therapeutic modulation of the activity of this gene or its protein product, through the use of small molecule drugs, protein therapeutics or antibodies, might be beneficial in the treatment of lung cancer.

**Panel 4D Summary:** Ag3347 Expression of the CG57847-01 gene is low/undetectable (CTs > 35) across all of the samples on this panel (data not shown).

### NOV82

Expression of NOV82/CG57845-01 was assessed using the primer-probe set Ag3346, described in Table BRA. Results of the RTQ-PCR runs are shown in Table BRB.

**Table BRA. Probe Name Ag3346**

| **Primers** | **Sequences** | **Length** | **Start Position** | **SEQ ID NO** |
|---|---|---|---|---|
| Forward | 5'-actcttcacagttgctgtgatg-3' | 22 | 317 | 509 |
| Probe | TET-5'-cacttgtgcttcatctgcatcgacag-3'-TAMRA | 26 | 365 | 510 |
| Reverse | 5'-aggggatcagtaaccacaatgt-3' | 22 | 393 | 511 |

**Table BRB. General_screening_panel_v1.4**

| **Tissue Name** | **Rel. Exp.(%) Ag3346, Run 215601952** | **Tissue Name** | **Rel. Exp.(%) Ag3346, Run 215601952** |
|---|---|---|---|
| Adipose | 0.0 | Renal ca. TK-10 | 0.0 |
| Melanoma* Hs688(A).T | 0.0 | Bladder | 4.7 |
| Melanoma* Hs688(B).T | 0.0 | Gastric ca. (liver met.) NCI-N87 | 0.0 |
| Melanoma* M14 | 0.0 | Gastric ca. KATO III | 0.0 |
| Melanoma*LOXIMVI | 4.5 | Colon ca. SW-948 | 0.0 |
| Melanoma*SK-MEL-3 | 0.0 | Colon ca. SW480 | 0.0 |
| Squamous cell carcinoma SCC-4 | 0.0 | Cotonca.*(SW480met) SW620 | 0.0 |
| Testis Pool | 6.1 | Colon ca. HT29 | 0.0 |
| Prostate ca.* (bone met) PC-3 | 0.0 | Colon ca. HCT-116 | 0.0 |
| Prostate Pool | 0.0 | Colon ca. CaCo-2 | 0.0 |
| Placenta | 0.0 | Colon cancer tissue | 0.0 |
| Uterus Pool | 0.0 | Coton ca. SW1116 | 0.0 |
| Ovarian ca. OVCAR-3 | 0.0 | Colon ca.Colo-205 | 0.0 |
| Ovarian ca. SK-OV-3 | 0.0 | Colon ca. SW-48 | 0.0 |
| Ovarian ca. OVCAR-4 | 0.0 | Colon Pool | 8.4 |
| Ovarian ca. OVCAR-5 | 7.9 | Small Intestine Pool | 0.0 |
| Ovarian caJGROV-1 | 0.0 | Stomach Pool | 0.0 |
| Ovarian ca. OVCAR-8 | 0.0 | Bone Marrow Pool | 2.4 |
| Ovary | 0.0 | Fetal Heart | 0.0 |
| Breast ca. MCF-7 | 0.0 | Heart Pool | 0.0 |
| Breast ca.MDA-MB-231 | 0.0 | Lymph Node Pool | 0.0 |
| Breast ca. BT 549 | 0.0 | Fetal Skeletal Muscle | 0.0 |
| Breast ca. T47D | 0.0 | Skeletal Muscle Pool | 0.0 |
| Breast ca. MDA-N | 0.0 | Spleen Pool | 0.0 |
| Breast Pool | 0.0 | Thymus Pool | 0.0 |
| Trachea | 0.0 | CNS cancer (glio/astro) U87-MG | 0.0 |
| Lung | 0.0 | CNS cancer (glio/astro) U-118-MG | 6.0 |
| Fetal Lung | 0.0 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 23.0 | CNS cancer (glio)SNB-19 | 0.0 |
| Lung ca. SHP-77 | 0.0 | CNS cancer (glio) SF-295 | 0.0 |
| Lung ca. A549 | 0.0 | Brain (Amygdala) Pool | 0.0 |
| Lung ca. NCI-H526 | 0.0 | Brain (cerebellum) | 0.0 |
| Lung ca. NCI-H23 | 100.0 | Brain (fetal) | 0.0 |
| Lung ca. NCI-H460 | 19.8 | Brain (Hippocampus) Pool | 8.7 |
| Lung ca. HOP-62 | 0.0 | Cerebral Cortex Pool | 11.5 |
| Lung ca. NCI-H522 | 0.0 | Brain (Substantia nigra) Pool | 0.0 |
| Liver | 0.0 | Brain (Thalamus) Pool | 0.0 |
| Fetal Liver | 0.0 | Brain (whole) | 0.0 |
| Liver ca. HepG2 | 0.0 | Spinal Cord Pool | 0.0 |
| Kidney Pool | 6.3 | Adrenal Gland | 0.0 |
| Fetal Kidney | 7.6 | Pituitary gland Pool | 0.0 |
| Renal ca. 786-0 | 0.0 | Salivary Gland | 0.0 |
| Renal ca. A498 | 0.0 | Thyroid (female) | 0.0 |
| Renal ca. ACHN | 4.2 | Pancreatic ca. CAPAN2 | 0.0 |
| Renal ca. UO-31 | 0.0 | Pancreas Pool | 0.0 |

**CNS_neurodegeneration_v1.0** Summary: Ag3346 Results from one experiment with the CG57845-01 gene are not included. The amp plot indicates that there were experimental difficulties with this run.

**General_screening_panel_v1.4** Summary: Ag3346 Significant expression of the CG57845-01 gene is detected exclusively in a lung cancer cell line NCI-H23 sample (CT=33.7). Therefore, expression of this gene may be used to distinguish lung cancers from the other samples on this panel. Furthermore, therapeutic modulation of the activity of the GPCR encoded by this gene may be beneficial in the treatment of lung cancer.

**Panel 4D Summary:** Ag3346 Expression of the CG57845-01 gene is low/undetectable (CTs > 35) across all of the samples on this panel (data not shown).

### NOV83

Expression of NOV83 CG57843-01 was assessed using the primer-probe set Ag3345, described in Table BSA. Results of the RTQ-PCR runs are shown in Table BSB.

**Table BSA. Probe Name Ag3345**

| **Primers** | **Sequences** | **Length** | **Start Position** | **SEQ ID NO** |
|---|---|---|---|---|
| Forward | 5'-catctccatcgacaggtacatt-3' | 22 | 379 | 512 |
| Probe | TET-5'ccctggtctatcctaccaagttcaccg-3'-TAMRA | 27 | 414 | 513 |
| Reverse | 5'-gatgcaaattcctgacacagat-3' | 22 | 442 | 514 |

**Table BSB. General_screening_panel_v1.4**

| **Tissue Name** | **Rel. Exp.(%) Ag3345, Run 215773854** | **Tissue Name** | **Rel. Exp.(%) Ag3345, Run 215773854** |
|---|---|---|---|
| Adipose | 0.0 | Renal ca. TK-10 | 0.0 |
| Melanoma* Hs688(A).T | 0.0 | Bladder | 0.0 |
| Melanoma* Hs688(B).T | 0.0 | Gastric ca. (liver met.) Na-N87 | 0.0 |
| Melanoma* M14 | 0.0 | Gastric ca. KATO III | 0.0 |
| Melanoma* LOXIMVI | 0.0 | Colon ca. SW-948 | 0.0 |
| Melanoma* SK-MEL-5 | 0.0 | Colon ca. SW480 | 0.0 |
| Squamous cell carcinoma SCC-4 | 0.0 | Colon ca.* (SW480 met) SW620 | 0.0 |
| TesttsPool | 0.0 | Colon ca. HT29 | 0.0 |
| Prostate ca.* (bone met) PC-3 | 0.0 | Colon ca. HCT-116 | 0.0 |
| Prostate Pool | 0.0 | Colon ca. CaCo-2 | 0.0 |
| Placenta | 0.0 | Colon cancer tissue | 0.0 |
| Uterus Pool | 100.0 | Colon ca. SW1116 | 0.0 |
| Ovarian ca.OVCAR-3 | 0.0 | Colon ca. Colo-205 | 0.0 |
| Ovarian ca. SK-OV-3 | 0.0 | Colon ca. SW-48 | 0.0 |
| Ovarian ca. OVCAR-4 | 0.0 | Colon Pool | 0.1 |
| Ovarian ca. OVCAR-5 | 0.0 | Small Intestine Pool | 0.0 |
| Ovarian ca. IGROV-1 | 0.0 | Stomach Pool | 0.0 |
| Ovarian ca. OVCAR-8 | 0.0 | Bone Marrow Pool | 0.0 |
| Ovary | 0.0 | Fetal Heart | 0.0 |
| Breast ca. MCF-7 | 0.0 | Heart Pool | 0.0 |
| Breast ca. MDA-MB-231 | 0.0 | Lymph Node Pool | 0.0 |
| Breast ca. BT 549 | 0.0 | Fetal Skeletal Muscle | 0.0 |
| Breast ca. T47D | 0.0 | Skeletal Muscle Pool | 0.0 |
| Breast ca. MDA-N | 0.0 | Spleen Pool | 0.0 |
| Breast Pool | 0.0 | Thymus Pool | 0.1 |
| Trachea | 0.0 | CNS MG cancer (glio/astro) U87- | 0.0 |
| Lung | 0.0 | CNS cancer (glio/astro) U-118-MG | 0.1 |
| Fetal Lung | 0.0 | CNS cancer (neuro;met) SK-N-AS | 0.0 |
| Lung ca. NCI-N417 | 0.0 | CNS cancer (astro) SF-539 | 0.0 |
| Lung ca. LX-1 | 0.0 | CNS cancer (astro) SNB-75 | 0.0 |
| Lung ca. NCI-H146 | 0.2 | CNS cancer (glio) SNB-19 | 0.0 |
| Lung ca. SHP-77 | 0.0 | CNS cancer (glio) SF-295 | 0.0 |
| Lung ca. A549 | 0.0 | Brain (Amygdala) Pool | 0.1 |
| Lung ca. NCI-H526 | 0.0 | Brain (cerebellum) | 0.0 |
| Lung ca. NCI-H23 | 0.8 | Brain (fetal) | 0.0 |
| Lung ca. NCI-H460 | 0.3 | Brain (Hippocampus) Pool | 0.0 |
| Lung ca. HOP-62 | 0.0 | Cerebral Cortex Pool | 0.1 |
| Lung ca. NCI-H522 | 0.0 | Brain (Substantia nigra) Foot | 0.1 |
| Liver | 0.0 | Brain (Thalamus) Pool | 0.1 |
| Fetal Liver | 3.1 | Brain (whole) | 0.1 |
| Liver ca. HepG2 | 0.0 | Spinal Cord Pool | 0.0 |
| Kidney Pool | 0.1 | Adrenal Gland | 0.0 |
| Fetal Kidney | 0.2 | Pituitary gland Pool | 0.0 |
| Renal ca. 786-0 | 0.0 | Salivary Gland | 0.0 |
| Renal ca. A498 | 0.0 | Thyroid (female) | 0.0 |
| Renal ca. ACHN | 0.1 | Pancreatic ca. CAPAN2 | 0.0 |
| Renal ca. UO-31 | 0.0 | Pancreas Pool | 0.0 |

**CNS_neurodegencration_v1.0 Summary:** Ag3345 Expression of the CG57843-01 gene is low/undetectable (CTs > 35) across all of the samples on this panel (data not shown).

**General_screening_panel_v1.4 Summary:** Ag3345 Expression of the CG57843-01 gene is highest in a sample derived from normal uterus (CT = 26.6). Thus, expression of this gene can be used to distinguish uterus from the other samples on this panel. Interestingly, expression of this gene is also detected in fetal liver (CT = 31) at much higher levels than in adult liver (CT = 40), suggesting that expression of this gene can be used to distinguish fetal from adult liver.

Panel 4D Summary: Ag3345 Expression of the CG57843-01 gene is low/undetectable (CTs > 34.5) across all of the samples on this panel (data not shown).

### NOV84

Expression of NOV84a, NOV84b, and NOV84c (CG57841-01, CG57841-02, and CG57837-01) was assessed using the primer-probe sets Ag3342 and Ag3344, described in Tables BTA and BTB. Results of the RTQ-PCR runs are shown in Table BTC. Please note that CG57841-02 represents a full-length physical clone of the CG57841-01 gene, validating the prediction of the gene sequence.

**Table BTA. Probe Name Ag3342**

| **Primers** | **Sequences** | **Length** | **Start Position** | **SEQ ID NO** |
|---|---|---|---|---|
| Forward | 5'-ttacctcctgaccataatggaa-3' | 22 | 111 | 515 |
| Probe | TET-5'-aacctgatgctgctgctcatgatcag-3'-TAMRA | 26 | 133 | 516 |
| Reverse | 5'-tacatgggcttatggagacaag-3' | 22 | 167 | 517 |

**Table BTB. Probe Name Ag3344**

| **Primers.** | **Sequences** | **Length** | **Start Position** | **SEQ ID NO** |
|---|---|---|---|---|
| Forward | 5'-ttacctcctgaccatgaa-3' | 22 | 111 | 518 |
| Probe | TET-5'-aacctgatgctgctgctcatgatcag-3'-TAMRA | 26 | 133 | 519 |
| Reverse | 5'-tacatgggettatggagacaag-3' | 22 | 167 | 520 |

**Table BTC. Genenal_screening_panel_v1.4**

| **Tissue Name** | **Rel. Exp.(%) Ag3342, Run 215773812** | **Tissue Name** | **Rel. Exp.(%) Ag3342, Run 215773812** |
|---|---|---|---|
| Adipose | 2.4 | Renal ca. TK-10 | 5.3 |
| Melanoma* Hs688(A).T | 0.0 | Bladder | 8.3 |
| Melanoma* Hs688(B).T | 0.0 | Gastric ca.(liver met.) NCl- | 40.9 |
| Melanoma* M14 | 0.0 | Gastric ca. KATO III | 0.0 |
| Melanoma* LOXIMVI | 4.6 | Colon ca. SW-948 | 0.0 |
| Melanoma* SK-MEL-5 | 0.0 | Colon ca. SW480 | 7.8 |
| Squamous cell carcinoma SCC-4 | 0.0 | Colon ca.* (SW480 met) SW620 | 5.1 |
| Testis Pool | 27.4 | Colon ca. HT29 | 0.0 |
| Prostate ca.* (bone met) PC-3 | 0.0 | Colon ca. HGT-116 | 2.3 |
| Prostate Pool | 3.4 | Colon ca CaCo-2 | 16.4 |
| Placenta | 0.0 | Colon cancer tissue | 0.0 |
| Uterus Pool | 0.0 | Colon ca. SW1116 | 0.0 |
| Ovarian ca. OVCAR-3 | 0.0 | Colon ca. Colo-205 | 0.0 |
| Ovarian ca. SK.OV-3 | 0.0 | Colon ca. SW-48 | 0.0 |
| Ovarian ca. OVCAR-4 | 4.8 | Colon Pool | 18.6 |
| Ovarian ca. OVCAR-5 | 0.0 | Small Intestine Pool | 15.4 |
| Ovarian ca. IGROV-1 | 4.3 | Stomach Pool | 0.0 |
| Ovarian ca. OVCAR-8 | 0.0 | Bone Marrow Pool | 9.2 |
| Ovary | 21.0 | Fetal Heart | 0.0 |
| Breast ca. MCF-7 | 7.5 | Heart Pool | 4.4 |
| Breast ca. MDA-MB-231 | 0.0 | Lymph Node Pool | 21.5 |
| Breast ca. BT 549 | 11.0 | Fetal Skeletal Muscle | 0.0 |
| Breast ca. T47D | 0.0 | Skeletal Muscle Pool | 3.0 |
| Breast ca. MDA-N | 0.0 | Spleen Pool | 5.6 |
| Breast Pool | 11.4 | Thymus Pool | 0.0 |
| Trachea | 0.0 | CNS cancer (glio/astro) U87-MG | 4.2 |
| Lung | 16.8 | CNS cancer (glio/astro) U-118-MG | 0.0 |
| Fetal Lung | 0.0 | CNS cancer (neuro;met) SK-N-AS | 0.0 |
| Lung ca. NCI-N417 | 0.0 | CNS cancer(astro)SF-539 | 0.0 |
| Lung ca.LX-1 | 3.1 | CNS cancer (astro) SNB-75 | 0.0 |
| Lung ca._NCI H146 | 12.5 | CNS cancer (glio) SNB-19 | 0.0 |
| Lung ca. SHP-77 | 0.0 | CNS cancer (glio) SF-295 | 5.4 |
| Lung ca. A549 | 0.0 | Brain (Amygdala) Pool | 0.0 |
| Lung ca. NCI-H526 | 0.0 | Brain (cerebellum) | 5.9 |
| Lung ca. NCI-H23 | 100.0 | Brain (fetal) | 0.0 |
| Lung ca. NCI-H460 | 8.3 | Brain (Hippocampus) Pool | 15.3 |
| Lung ca. HOP-62 | 0.0 | Cerebral Cortex Pool | 8.1 |
| Lung ca. NCI-H522 | 0.0 | Brain (Substantia nigra) Pool | 7.7 |
| Liver | 0.0 | Brain (Thalamus) Pool | 11.0 |
| Fetal Liver | 0.0 | Brain(whole) | 0.0 |
| Liver ca. HepG2 | 0.0 | Spinal Cord Pool | 16.4 |
| Kidney Pool | 23.8 | Adrenal Gland | 4.6 |
| Fetal Kidney | 15.7 | Pituitary gland Pool | 0.0 |
| Renal ca. 786-0 | 4.7 | Salivary Gland | 4.8 |
| Renal ca. A498 | 0.0 | Thyroid (female) | 0.0 |
| Renal ca. ACHN | 0.0 | Pancreatic ca. CAPAN2 | 9.8 |
| Renal ca. UO-31 | 0.0 | Pancreas Pool | 13.7 |

**CNS_neurodegeneration_v1.0 Summary:** Ag3342/Ag3344 Expression of the CG57841-01 gene is low/undetectable (CTs > 34.5) across all of the samples on this panel (data not shown).

**General_screening_panel_v1.4 Summary:** Ag3342 Significant expression of the CG57841-01 gene is seen exclusively in a lung cancer NCI-H23 sample (CT = 34.1). Therefore, expression of this gene may be used to distinguish this sample from the other samples on this panel. Furthermore, therapeutic modulation of the activity of the GPCR encoded by this gene may be beneficial in the treatment of lung cancer. Ag3344 Results from one experiment with the CG57841-01 gene are not included. The amp plot indicates that there were experimental difficulties with this run.

**Panel 4D Summary:** Ag3342/Ag3344 Expression of this gene is low/undetectable (CTs > 35) across all of the samples on this panel (data not shown).

### NOV85

Expression of NOV85 CG57839-01 was assessed using the primer-probe sets Ag2308 and Ag3343, described in Tables BUA and BUB. Results of the RTQ-PCR runs are shown in Tables BUC, and BUD.

**Table BUA. Probe Name Ag2308**

| **Primers** | **Sequences** | **Length** | **Start Position** | **SEQ ID NO** |
|---|---|---|---|---|
| Forward | 5'-taccgatcatagcacatcatca-3' | 22 | 323 | 521 |
| Probe | TET-5'-tcagacactctgtaatagcaaacgcca-3'-TAMRA | 27 | 290 | 522 |
| Reverse | 5'-tgctccttgcatacttcagact-3' | 22 | 258 | 523 |

**Table BUB. Probe Name Ag3343**

| **Primers** | **Sequences** | **Length** | **Start Position** | **SEQ ID NO** |
|---|---|---|---|---|
| Forward | 5'-ctttccctgttttacagccttt-3' | 22 | 826 | 524 |
| Probe | TET-5'-cccctcatctacagccttaatgcaga-3'-TAMRA | 26 | 865 | 525 |
| Reverse | 5'-ctctctttagagcccctttcac-3' | 22 | 892 | 526 |

**Table BUC. CNS_neurodegeneration_v1.0**

| **Tissue Name** | **Rel. Exp.(%) Ag2308, Run 207970871** | **Tissue Name** | **ReL Exp.(%) Ag2308, Run 207970871** |
|---|---|---|---|
| AD 1 Hippo | 0.0 | Control (Path) 3 Temporal Ctx | 0.0 |
| AD 2 Hippo | 9.8 | Control (Path) 4 Temporal Ctx | 10.7 |
| AD 3 Hippo | 0.0 | AD 1 Occipital Ctx | 24.0 |
| AD 4 Hippo | 18.2 | AD 2 Occipital Ctx (Missing) | 0.0 |
| AD 5 Hippo | 16.6 | AD 3 Occipital Ctx | 0.0 |
| AD 6 Hippo | 81.8 | AD 4 Occipital Ctx | 1.2 |
| Control 2 Hippo | 0.0 | AD 5 Occipital Ctx | 1.8 |
| Control 4 Hippo | 38.7 | AD 6 Occipital Ctx | 1.9 |
| Control (Path) 3 Hippo | 1.2 | Control 1 Occipital Ctx | 17.6 |
| AD 1 Temporal Ctx | 16.4 | Control 2 Occipital Ctx | 0.0 |
| AD 2 Temporal Ctx | 14.6 | Control 3 Occipital Ctx | 17.2 |
| AD 3 Temporal Ctx | 0.0 | Control 4 Occipital Ctx | 30.8 |
| AD 4 Temporal Ctx Ctx | 20.4 | Control (Path) 1 Occipital | 0.0 |
| AD 5 Inf Temporal Ctx | 15.1 | Control (path) 2 Occipital Ctx | 11.7 |
| AD 5 Sup Temporal Ctx | 27.9 | Control (Path) 3 Occipital Ctx | 0.0 |
| AD 6 Inf Temporal Ctx | 13.0 | Control (Path) 4 Occipital Ctx | 16.7 |
| AD 6 Sup Temporal Ctx | 1.6 | Control 1 Parietal Ctx | 0.0 |
| Control 1 Temporal Ctx | 0.0 | Control Parietal Ctx | 0.0 |
| Control 2 Temporal Ctx | 1.2 | Control 3 Parietak Ctx | 0.0 |
| Control 3 Temporal Ctx | 100.0 | Control (Path) 1 Parietal | 4.0 |
| Control 3 Temporal Ctx | 38.7 | Control (Path) 2 Parietal | 0.0 |
| Control (Path) 1 Temporal Ctx | 16.2 | Control (Path) 3 Parietal Ctx | 10.3 |
| Control (Path) 2 Temporal Ctx | 0.0 | Control (Path) 4 Parietal Ctx | 31.0 |

**Table BUD. Panel 4D**

| **Tissue Name** | **Rel. Exp.(%) Ag2308, Run 158927487** | **Tissue Name** | **Rel. Exp.(%) Ag2308, Run 158927487** |
|---|---|---|---|
| Secondary Th1 act | 0.0 | HUVEC IL-1beta | 0.0 |
| Secondary Th2 act | 0.0 | HUVEC IFN gamma | 17.4 |
| Secondary Tr1 act | 0.0 | HUVEC TNF alpha + IFN gamma | 0.0 |
| Secondary Th1 rest | 17.2 | HUVEC TNF alpha + IL4 | 0.0 |
| Secondary Th2 rest | 0.0 | HUVEC IL-11 | 4.4 |
| Secondary Tr1 rest | 0.0 | Lung Microvascular EC none | 0.0 |
| Primary Th1 act | 11.3 | Lung Microvascular EC TNFalpha +IL-1beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 24.0 | Bronchial epithelium TNFalpha + IL1beta | 0.0 |
| Primary Th2 rest | 14.5 | Small airway epithelium none | 0.0 |
| PrimaryTr1 rest | 0.0 | Small airway epithelium TNFalpha +IL+beta | 15.1 |
| CD45RA CD4 lymphocyte act | 0.0 | Coronery artery SMC rest | 0.0 |
| CD45R0 CD4 lymphocyte act | 5.0 | Coronery artery SMC TNFalpha + IL-1beta | 0.0 |
| CD8 lymphocyte act | 0.0 | Astrocytes rest | 0.0 |
| Secondary CD8 lymphocyte rest | 0.0 | Astrocytes TNFalpha + IL-1 beta | 0.0 |
| Secondary CDB lymphocyte act | 0.0 | KU-812(Basophil)rest | 0.0 |
| CD4lymphocyte none | 0.0 | KU-812 (Basophil) PMA/ionomycin | 0.0 |
| 2ry Th1/Th2/Tr1 anti-CD95 CH11 | 0.0 | CCDI 106 (Keratinocytes) none | 0.0 |
| LAK cells rest | 0.0 | CCD1106 (Keratinocytes) TNFalpha+IL-1beta | 0.0 |
| LAK cells IL-2 | 0.0 | Liver cirrhosis | 29.1 |
| LAK cells IL-2+IL-12 | 0.0 | Lupus kidney | 9.9 |
| LAK cells IL-2+IFN gamma | 21.8 | NCI-H292 none | 26.6 |
| LAK cells IL-2+IL-18 | 0.0 | NCI-H292 IL-4 | 28.7 |
| LAK cells PMA/ionomycin | 0.0 | NCI-H292 IL-9 | 37.9 |
| NK Cells IL-2 rest | 0.0 | NCI-H292 IL-13 | 0.0 |
| Two Way MLR 3 day | 10.6 | NCI-H292 IFN gamma | 13.3 |
| Two Way MLR 5 day | 21.5 | HPAEC none | 0.0 |
| Two Way MLR 7 day | 0.0 | HPAEC TNF alpha + IL-1 beta | 0.0 |
| PBMC rest | 0.0 | Lung fibroblast none | 11.3 |
| PBMC PWM | 0.0 | Lung fibroblast TNF alpha + IL-1 | 0.0 |
| PBMC PHA-L | 14.6 | Lung fibroblast IL-4 | 18.6 |
| Ramos(B cell) none | 0.0 | Lung fibroblast IL-9 | 0.0 |
| Ramos (B cell) ionomycin | 0.0 | Lung fibroblast IL-13 | 15.9 |
| B lymphocytes PWM | 0.0 | Lung fibroblast IFN gamma | 22.2 |
| B lymphocytes CD40L and IL-4 | 0.0 | Dermal fibroblast CCD1070 rest | 0.0 |
| EOL-1 dbcAMP | 0.0 | Dermal fibroblast CCD1070 TNF alpha | 6.7 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 0.0 | Dermal fibroblast IL-4 | 27.5 |
| Dendritic cells anti-CD40 | 0.0 | IBD Colitis 2 | 0.0 |
| Monocytes rest | 0.0 | IBD Crohn's | 0.0 |
| Monocytes LPS | 0.0 | Colon | 8.7 |
| Macrophages rest | 7.5 | Lung | 47.0 |
| Macrophages LPS | 0.0 | Thymus | 100.0 |
| HUVEC none | 0.0 | Kidney | 5.8 |
| HUVEC starved | 0.0 | | |

**CNS_neurodegeneration_v1.0 Summary:** Ag2308 The CG57839-01 gene is expressed at low levels in the brains of both normal and Alzheimer's disease patients and encodes a putative GPCR (CTs=33-35). Several neurotransmitter receptors are GPCRs, including the dopamine receptor family, the serotonin receptor family, the GABAB receptor, muscarinic acetylcholine receptors, and others; thus this GPCR may represent a novel neurotransmitter receptor. Targeting various neurotransmitter receptors (dopamine, serotonin) has proven to be an effective therapy in psychiatric illnesses such as schizophrenia, bipolar disorder, and depression. Furthermore, the cerebral cortex and hippocampus are regions of the brain that are known to be involved in Alzheimer's disease, seizure disorders, and in the normal process of memory formation. Therefore, therapeutic modulation of this gene or its protein product may be beneficial in the treatment of one or more of these diseases, as may stimulation and/or blockade of the receptor coded for by the gene. Ag3343 Expression of this gene is low/undetectable (CTs > 35) across all of the samples on this panel (data not shown).

### References:

### 1. E1 Yacoubi M, Ledent C, Parmentier M, Bertorelli R, Ongini E, Costentin J, Vaugeois JM. Adenosine A2A receptor antagonists are potential antidepressants: evidence based on pharmacology and A2A receptor knockout mice. Br J Pharmacol 2001 Sep;134(1):68-77

1. Adenosine, an ubiquitous neuromodulator, and its analogues have been shown to produce 'depressant' effects in animal models believed to be relevant to depressive disorders, while adenosine receptor antagonists have been found to reverse adenosine-mediated 'depressant' effect. 2. We have designed studies to assess whether adenosine A2A receptor antagonists, or genetic inactivation of the receptor would be effective in established screening procedures, such as tail suspension and forced swim tests, which are predictive of clinical antidepressant activity. 3. Adenosine A2A receptor knockout mice were found to be less sensitive to 'depressant' challenges than their wildtype littermates. Consistently, the adenosine A2A receptor blockers SCH 58261 (1-10 mg kg(-1), i.p.) and KW 6002 (0.1-10 mg kg(-1), p.o.) reduced the total immobility time in the tail suspension test. 4. The efficacy of adenosine A2A receptor antagonists in reducing immobility time in the tail suspension test was confirmed and extended in two groups of mice. Specifically, SCH 58261 (1 - 10 mg kg(-1)) and ZM 241385 (15-60 mg kg(-1)) were effective in mice previously screened for having high immobility time, while SCH 58261 at 10 mg kg(-1) reduced immobility of mice that were selectively bred for their spontaneous 'helplessness' in this assay. 5. Additional experiments were carried out using the forced swim test. SCH 58261 at 10 mg kg(-1) reduced the immobility time by 61 %, while KW 6002 decreased the total immobility time at the doses of 1 and 10 mg kg(-1) by 75 and 79%, respectively. 6. Administration of the dopamine D2 receptor antagonist haloperidol (50 - 200 microg kg(-1) i.p.) prevented the antidepressant-like effects elicited by SCH 58261 (10mg kg(-1) i.p.) in forced swim test whereas it left unaltered its stimulant motor effects. 7. In conclusion, these data support the hypothesis that A2A receptor antagonists prolong escape-directed behaviour in two screening tests for antidepressants. Altogether the results support the hypothesis that blockade of the adenosine A2A receptor might be an interesting target for the development of effective antidepressant agents.
2. Blier P. Pharmacology of rapid-onset antidepressant treatment strategies. Clin Psychiatry 2001;62 Suppl 15:12-7

Although selective serotonin reuptake inhibitors (SSRIs) block serotonin (5-HT) reuptake rapidly, their therapeutic action is delayed. The increase in synaptic 5-HT activates feedback mechanisms mediated by 5-HT1A (cell body) and 5-HT1B (terminal) autoreceptors, which, respectively, reduce the firing in 5-HT neurons and decrease the amount of 5-HT released per action potential resulting in attenuated 5-HT neurotransmission. Long-term treatment desensitizes the inhibitory 5-HT1 autoreceptors, and 5-HT neurotransmission is enhanced. The time course of these events is similar to the delay of clinical action. The addition of pindolol, which blocks 5-HT1A receptors, to SSRI treatment decouples the feedback inhibition of 5-HT neuron firing and accelerates and enhances the antidepressant response. The neuronal circuitry of the 5-HT and norepinephrine (NE) systems and their connections to forebrain areas believed to be involved in depression has been dissected. The firing of 5-HT neurons in the raphe nuclei is driven, at least partly, by alphal-adrenoceptor-mediated excitatory inputs from NE neurons. Inhibitory alpha2-adrenoceptors on the NE neuroterminals form part of a feedback control mechanism. Mirtazapine, an antagonist at alpha2-adrenoceptors, does not enhance 5-HT neurotransmission directly but disinhibits the NE activation of 5-HT neurons and thereby increases 5-HT neurotransmission by a mechanism that does not require a time-dependent desensitization of receptors. These neurobiological phenomena may underlie the apparently faster onset of action of mirtazapine compared with the SSRIs.

### 3. Tranquillini ME, Reggiani A. Glycine-site antagonists and stroke. Expert Opin Investig Drugs 1999 Nov;8(11):1837-1848

The excitatory amino acid, (S)-glutamic acid, plays an important role in controlling many neuronal processes. Its action is mediated by two main groups of receptors: the ionotropic receptors (which include NMDA, AMPA and kainic acid subtypes) and the metabotropic receptors (mGluR(1-8)) mediating G-protein coupled responses. This review focuses on the strychnine insensitive glycine binding site located on the NMDA receptor channel, and on the possible use of selective antagonists for the treatment of stroke. Stroke is a devastating disease caused by a sudden vascular accident. Neurochemically, a massive release of glutamate occurs in neuronal tissue; this overactivates the NMDA receptor, leading to increased intracellular calcium influx, which causes neuronal cell death through necrosis. NMDA receptor activation strongly depends upon the presence of glycine as a co-agonist. Therefore, the administration of a glycine antagonist can block overactivation of NMDA receptors, thus preserving neurones from damage. The glycine antagonists currently identified can be divided into five main categories depending on their chemical structure: indoles, tetrahydroquinolines, benzoazepines, quinoxalinediones and pyrida-zinoquinolines.

### 4. Monopoli A, Lozza G, Forlani A, Mattavelli A, Ongini E. Blockade of adenosine A2A receptors by SCH 58261 results in neuroprotective effects in cerebral ischaemia in rats. Neuroreport 1998 Dec 1;9(17):3955-9

Blockade of adenosine receptors can reduce cerebral infarct size in the model of global ischaemia. Using the potent and selective A2A adenosine receptor antagonist, SCH 58261, we assessed whether A2A receptors are involved in the neuronal damage following focal cerebral ischaemia as induced by occluding the left middle cerebral artery. SCH 58261 (0.01 mg/kg either i.p. or i.v.) administered to normotensive rats 10 min after ischaemia markedly reduced cortical infarct volume as measured 24 h later (30% vs controls, p < 0.05). Similar effects were observed when SCH 58261 (0.01 mg/kg, i.p.) was administered to hypertensive rats (28% infarct volume reduction vs controls, p < 0.05). Neuroprotective properties of SCH 58261 administered after ischaemia indicate that blockade of A2A adenosine receptors is a potentially useful biological target for the reduction of brain injury.

**Panel 1.3D Summary:** Ag2308 Expression of the CG57839-01 gene is low/undetectable (CT>35) in all samples shown in this Panel (data not shown).

**Panel 4D Summary:** Ag2308 Expression of the CG57839-01 gene is detected in the thymus (CT = 33.3) and lung (CT = 34.4). Thus, expression of this gene could be used as a marker to detect the presence of thymus or lung tissue. The putative GPCR encoded for by this gene may also play an important role in the normal homeostasis of these tissues. Therefore, therapeutics designed with this gene product could be important for maintaining or restoring normal function to these organs during inflammation. Ag3343 Expression of this gene is low/undetectable (CTs > 35) across all of the samples on this panel (data not shown).

### NOV87

Expression of NOV87 CG56763-01 was assessed using the primer-probe set Ag3012, described in Table BVA. Results of the RTQ-PCR runs are shown in Tables BVB, and BVC.

**Table BVA. Probe Name Ag3012**

| **Primers** | **Sequences** | **Length** | **Start Position** | **SEQ ID NO** |
|---|---|---|---|---|
| Forward | 5'-ctctttgtcctggtggagaac-3' | 21 | 162 | 527 |
| Probe | TET-5'-acctccctecacaggcccatgtacta-3'-TAMRA | 26 | 213 | 528 |
| Reverse | 5'-gaaagacatggagctcagaaag-3' | 22 | 239 | 529 |

**Table BVB. Panel 1.3D**

| **Tissue Name** | **Rel. Exp.(%) Ag3012, Run 167810404** | **Tissue Name** | **Rel.Exp.(%) Ag3012, Run 167810404** |
|---|---|---|---|
| Liver adenocarcinoma | 0.0 | Kidney (fetal) | 0.0 |
| Pancreas | 0.0 | Renal ca. 786-0 | 0.0 |
| Pancreatic ca. CAPAN 2 | 1.6 | Renal ca. A498 | 0.0 |
| Adrenal gland | 0.0 | Renal ca. RXF 393 | 0.0 |
| Thyroid | 0.0 | Renal ca.ACHN | 0.0 |
| Salivary gland | 2.8 | Renal ca. UO-31 | 0.0 |
| Pituitary gland | 0.0 | Renal ca.TK-10 | 0.0 |
| Brain (fetal) | 0.0 | Liver | 0.0 |
| Brain (whole) | 0.0 | Liver (fetal) | 0.0 |
| Brain (amygdala) | 12.6 | Liver ca. (hepatoblast) HepG2 | 0.0 |
| Brain (cerebellum) | 0.0 | Lung | 0.0 |
| Brain (hippocampus) | 5.5 | Lung (fetal) | 0.0 |
| Brain (substantia nigra) | 3.0 | Lung ca. (small cell) LX-1 | 1.4 |
| Brain (thalamus) | 3.1 | Lung ca. (small cell) NCI-H69 | 0.0 |
| Cerebral Cortex | 0.0 | Lung ca. (s.cell var.) SHP-77 | 2.4 |
| Spinal cord | 100.0 | Lung ca. (large cell)NCI- H460 | 0.0 |
| glio/astro U87-MG | 0.0 | Lung ca. (non-sm. cell) A549 | 0.0 |
| glio/astro U-118-MG | 0.0 | Lung ca. (non-s.cell) NCI-H23 | 0.0 |
| astrocytoma SW1783 | 0.0 | Lung ca. (non-s.cell) HOP-62 | 0.0 |
| neuro*; met SK-N-AS | 0.0 | Lung ca. (non-s.cl) NCI- H522 | 0.0 |
| astrocytoma SF-539 | 0.0 | Lung ca.(squam.) SW 900 | 0.0 |
| astrocytoma SNB-75 | 0.0 | H596 Lung ca. (squam.) NCI-H596 | 0.0 |
| glioma SNB-19 | 3.2 | Mammary gland | 2.9 |
| glioma U251 | 0.0 | Breast ca.* (pl.ef) MCF-7 | 3.5 |
| glioma SF-295 | 0.0 | Breast ca.* (pl.ef) MDA-MB-231 | 0.0 |
| Heart (fetal) | 0.0 | Breast ca.* (pl.ef) T47D | 0.0 |
| Heart | 2.6 | Breast ca. BT-549 | 0.0 |
| Skeletal muscle (fetal) | 12.5 | Breast ca. MDA-N | 0.0 |
| Skeletal muscle | 0.0 | Ovary | 0.0 |
| Bone marrow | 0.0 | Ovarian ca. OVCAR-3 | 3.0 |
| Thymus | 9.5 | Ovarian ca. OVCAR-4 | 0.0 |
| Spleen | 0.0 | Ovarian ca. OVCAR-5 | 3.5 |
| Lymph node | 0.0 | Ovarian ca. OVCAR-8 | 0.0 |
| Colorectal | 4.3 | Ovarian ca. IGROV-1 | 0.0 |
| Stomach | 0.0 | Ovarian ca.* (ascites) SK-OV-3 | 0.0 |
| Small intestine | 0.0 | Uterus | 0.0 |
| Colon ca. SW480 | 0.0 | Placenta | 0.0 |
| Colonca.*SW620(SW480 met) | 0.0 | Prostate | 0.0 |
| Colon ca. HT29 | 0.0 | prostate ca.* (bone met)PC- 3 | |
| Colon ca.HCT.116 | 0.0 | Testis | 35.4 |
| Colon ca. CaCo-2 | 0.0 | Melanoma Hs688(A).T | 0.0 |
| Colon ca. tissue(OD03866) | 0.0 | Melanoma* (met) Hs688(B).T | 0.0 |
| Colon ca. HCG2998 | 6.0 | Melanoma UACC-62 | 0.0 |
| Gastric ca.* (liver met) NCI-N87 | 0.0 | Melanoma M14 | 0.0 |
| Bladder | 0.0 | Melanoma LOX IMVI | 0.0 |
| Trachea | 0.0 | Melanoma* (met) SK-MEL-5 | 0.0 |
| Kidney | 0.0 | Adipose | 0.0 |

**Table BVC. Panel 4D**

| **Tissue Name** | **Rel. Exp.(%) Ag3012, Run 164404080** | **Tissue Name** | **Rel. Exp.(%) Ag3012, Run 164404080** |
|---|---|---|---|
| Secondary Th1 act | 0.0 | HUVEC IL-1beta | 0.0 |
| Secondary Th2 act | 0.0 | HUVEC IFN gamma | 0.0 |
| Secondary Tr1 act | 0.0 | HUVEC TNF alpha + IFN gamma | 0.0 |
| Secondary Th1 rest | 0.0 | HUVEC TNF alpha+IL4 | 0.0 |
| Secondary Th2 rest | 0.0 | HUVEC IL-11 | 0.0 |
| Secondary Tr1 rest | 0.0 | Lung Microvascular EC none | 0.0 |
| Primary Th1 act | 0.0 | Lung Microvascular EC TNFalpha +UL-1beta | 0.0 |
| Primary Th2 act | 0.0 | Microvascular Dermal BC none | 0.0 |
| Primary Tr1 act | 0.0 | Microsvasular Dermal EC TNFalpha + IL-lbeta | 0.0 |
| Primary Th1 rest | 0.0 | Bronchial epithelium TNFalpha + IL 1beta IL 1beta | 0.0 |
| Pnmary Th2 rest | 8.0 | Small airway epithelium none | 0.0 |
| Primary Tr1 rest | 0.0 | Small airway epithelium TNF alpha IL-1beta | 0.0 |
| CD45RA CD4 lymphocyte act | 0.0 | Coronery artery SMC rest | 0.0 |
| CD45RO CD4 lymphocyte act | 0.0 | Coronery artery SMC TNFalpha + IL-1beta | 0.0 |
| CD8 lymphocyte act | 0.0 | Astrocytes rest | 7.2 |
| Secondary CD8 lymphocyte rest | 7.9 | Astrocytes TNFalpha + IL-1 beta | 0.0 |
| Secondary CD8 lymphocyte act | 0.0 | KU-812 (Basophil) rest | 0.0 |
| CD4 lymphocyte none | 10.2 | KU-812 (Basophil) PMA/ionomycin | 7.7 |
| 2ry Th1/Th2/Tr1_anti-CD95 CH11 | 0.0 | CCD1106 (Keratinocytes) none | 0.0 |
| LAK cells rest | 10.7 | CCD1106 (Keratinocytes) TNFalpha + IL-1beta | 0.0 |
| LAK cells IL-2 | 0.0 | Liver cirrhosis | 100.0 |
| LAK cells IL-2+IL-12 | 0.0 | Lupus kidney | 0.0 |
| LAK cells IL-2+IFN gamma | 9.4 | NCI-H292 none | 0.0 |
| LAK cells IL-2+IL-18 | 14.1 | NCI-H292 IL-4 | 0.0 |
| LAK cells PMA/ionomycin | 0.0 | NCI-H292IL-9 | 18.9 |
| NK Cells IL-2 rest | 0.0 | NCl-H292 IL-13 | 0.0 |
| Two Way MLR 3 day | 4.0 | NCI-H292 IFN gamma | 0.0 |
| Two Way MLR 5 day | 0.0 | HPAEC none | 0.0 |
| Two Way MLR 7 day | 0.0 | HPAEC TNF alpha+ IL-1-beta | 0.0 |
| PBMC rest | 0.0 | Lung fibroblast none | 0.0 |
| PBMC PWM | 8.1 | Lung fibroblast TNF alpha +IL-1 beta | 0.0 |
| PBMC PHA-L | 0.0 | Lung fibroblast IL-4 | 0.0 |
| Ramos (B cell) none | 0.0 | Lung fibroblast IL-9 | 0.0 |
| Ramos (B cell) ionomycin | 0.0 | Lung fibroblast IL-13 | 0.0 |
| B lymphocytes PWM | 0.0 | Lung fibroblast IFN gamma | 0.0 |
| B lymphocytes CD40L and IL-4 | 19.6 | Dermal fibroblast CCD 1070 rest | 0.0 |
| EOL-1 dbcAMP | 16.7 | Dermal fibroblast CCD1070TNF | 0.0 |
| EOL-1 dbcAMP PMA/ionomycin | 0.0 | Dermal fibroblast CCD1070 IL-1 beta | 0.0 |
| Dendritic cells none | 0.0 | Dermal fibroblast IFN gamma | 0.0 |
| Dendritic cells LPS | 4.5 | Dermal fibroblast IL-4 | 0.0 |
| Dendritic cells anti-CD40 | 0.0 | IBD Colitis 2 | 0.0 |
| Monocytes rest | 0.0 | IBD Crohn's | 57.0 |
| Monocytes LPS | 0.0 | Colon | 56.6 |
| Macrophages rest | 0.0 | Lung | 65.5 |
| Macrophages LPS | 0.0 | Thymus | 14.9 |
| HUVEC none | 0.0 | Kidney | 0.0 |
| HUVEC starved | 0.0 | | |

**CNS_neurodegeneration_v1.0 Summary:** Ag3012 Expression of the CG56763-01 gene is low/undetectable (CTs > 34.5) across all of the samples on this panel (data not shown).

Panel 1.3D Summary: Ag3012 The CG56763-01 gene is expressed at low levels in the samples derived from spinal cord (CT = 32.1) and testis (CT=33.6). Thus, the expression of this gene could be used to distinguish these samples from the other samples in the panel. Furthermore, therapeutic modulation of the expression or function of this gene may be effective in the treatment of CNS disorders, fertility and hypogonadism.

**Panel 4D Summary:** Ag3012 The CG56763-01 gene is expressed at low levels in a sample derived from liver cirrhosis (CT = 33.4). In addition, expression of this gene is not detected in normal liver in Panel 1.3D, suggesting that its expression is unique to liver cirrhosis. This gene encodes a putative GPCR; therefore, antibodies or small molecule therapeutics could reduce or inhibit fibrosis that occurs in liver cirrhosis. This gene is also expressed at low levels in the lung and colon.

### NOV88

Expression of NOV88/CG56753-01 was assessed using the primer-probe sets Ag2875 and Ag3010, described in Tables BWA and BWB. Results of the RTQ-PCR runs are shown in Table BWC.

**Table BWA. Probe Name Ag2875**

| **Primers** | **Sequence** | **Length** | **Start Position** | **SEQ ID NO** |
|---|---|---|---|---|
| Forward | 5'-gaacatcatctcctaccctgaa-3' | 22 | 267 | 530 |
| Probe | TET-5'-tgcatgactctagctctacttcttcctcg-3'-TAMRA | 28 | 289 | 531 |
| Reverse | 5'-atgtgacactctgcaatagcaa-3' | 22 | 320 | 532 |

**Table BWB. Probe Name Ag3010**

| **Primers** | **Sequences** | **Length** | **Start Position** | **SEQ ID NO** |
|---|---|---|---|---|
| Forward | 5'-ggggaaagtatcctctgtgttt-3' | 22 | 810 | 533 |
| Probe | TET-5'-ttattgtgcccatgttgaaccctctg-3'-TAMRA | 26 | 839 | 534 |
| Reverse | 5'-cagggaaacatggacatcttta-3' | 22 | 882 | 535 |

**Table BWC. Panel 4D**

| **Tissue Name** | **Rel. Exp.(%) Ag2875, Run 164311029** | **Tissue Name** | **Rel. Exp.(%) Ag2875, Run 164311029** |
|---|---|---|---|
| Secondary Th1 act | 0.0 | HUVEC IL-1beta | 0.0 |
| Secondary Th2 act | 0.0 | HUVEC IFN gamma | 3.0 |
| Secondary Tr1 act | 0.0 | HUVEC TNF alpha + IFN gamma | 0.0 |
| Secondary Th1 rest | 0.0 | HUVEC INF alpha + IL4 | 0.0 |
| Secondary Th2 rest | 0.0 | HUVEC IL-11 | 0.0 |
| SecondaryTr1 rest | 0.0 | Lung Microvascular EC none | 0.0 |
| Primary Th1 act | 0.0 | Lung Microvascular EC TNFalpha + IL, I beta | 0.0 |
| Primary Th2 act | 0.0 | Microvascular Dermal EC none | 0.0 |
| Primary Tr1 act | 0.0 | Microsvasular Dermal EC TNFalpha + IL-1beta | 0.0 |
| Primary Th1 rest | 0.0 | Bronchial epithelium TNFalpha + IL1 beta | 0.0 |
| Primary Th2 rest | 0.0 | Small airway epithelium none | 0.0 |
| Primary Tr1 rest | 0.0 | Small airway epithelium TNFalpha + IL-1beta | 0.0 |
| CD45RA CD4 lymphocyte act | 0.0 | Coronery artery SMC rest | 0.0 |
| CD45RO CD4 lymphocyte act | 0.0 | Coronery artery SMC TNFalpha+ IL-1beta | 0.0 |
| CD8 lymphocyte act | 0.0 | Astrocytes rest | 0.0 |
| Secondary CD8 lymphocyte rest | 0.0 | Astrocytes TNF alpha+IL-1beta | 0.0 |
| Secondary CD8 lymphocyte | 0.0 | KU-812 (Basophil) rest | 0.0 |
| CD4 lymphocyte none | 0.0 | KU-812 (Basophil) PMA/ionomycin | 24.0 |
| 2ry Th1/Th2/Tr1_anti-CD95 CH11 | 5.1 | CCD1106 (Keratinocytes) none | 0.0 |
| LAK cells rest | 0.0 | CCD1106 (Keratinocytes) TNFalpha + IL-1beta | 0.0 |
| LAK cells IL-2 | 0.0 | Liver cirrhosis | 72.2 |
| LAK cells IL-2+IL-12 | 0.0 | Lupus kidney | 0.0 |
| LAK cells IL-2+IFN gamma, | 0.0 | NCI-H292 none | 20.7 |
| LAK cells IL-2+IL-18 | 0.0 | NCI-H292 IL-4 | 0.0 |
| LAK cells PMA/ionomycin | 0.0 | NCI-H292 IL-9 | 0.0 |
| NK Cells IL-2 rest | 0.0 | NCI-H292 IL-13 | 0.0 |
| Two Way MLR 3 day | 0.0 | NCI-H292 IFN gamma | 0.0 |
| Two Way MLR 5 day | 0.0 | HPAEC none | 0.0 |
| Two Way MLR 7 day | 0.0 | HPAEC TNF alpha + IL-1 beta | 1.4 |
| PBMC rest | 0.0 | Lung fibroMast none | 0.0 |
| PBMC PWM | 0.0 | Lung fibroblast TNF alpha +IL-1 beta | 0.0 |
| PBMC PHA-L | 0.0 | Lung fibroblast IL-4 | 0.0 |
| Ramos (B cell) none | 0.0 | Lung fibroblast IL,9 | 0.0 |
| Ramos (B cell) ionomycin | 0.0 | Lung fibroblast IL-13 | 0.0 |
| B lymphocytes PWM | 0.0 | Lung fibroblast IFN gamma | 3.2 |
| B lymphocytes CD40L and IL-4 | 0.0 | Dermal fibroblast CCD 1070 rest | 0.0 |
| EOL-1 dbcAMP | 3.1 | Dermal fibroblast CCD1070 TNF alpha | 0.0 |
| EOL-1 dbcAMP PMA/ionomycin | 0.0 | Dermal fibroblast CCD1070 IL-1 beta | 0.0 |
| Dendridc cells none | 33.2 | Dermal fibroblast IFN gamma | 0.0 |
| Dendritic cells LPS | 2.4 | Dermal fibroblast IL-4 | 1.9 |
| Dendritic cells anti-CD40 | 100.0 | IBD Colitis 2 | 16.3 |
| Monocytes rest | 0.0 | IBD Crohn's | 6.7 |
| Monocytes LPS | 0.0 | Colon | 0.0 |
| Macrophages rest | 44.1 | Lung | 21.8 |
| Macrophages LPS | 0.0 | Thymus | 0.0 |
| HUVECnone | 3.1 | Kidney | 0.0 |
| HUVEC starved | 0.0 | | |

**Panel 1.3D Summary:** Ag2875/Ag3010 Results from two experiments with the CG56753-01 gene are not included. The amp plots indicate that there were experimental difficulties with these runs.

**Panel 2.2 Summary:** Ag2875 Expression of this gene is low/undetectable (CTs > 35) across all of the samples on this panel (data not shown).

Panel 4D Summary: Ag2875 Highest expression of the CG56753-01 gene is in anti-CD40 treated dendritic cells (CT=33.2), with much lower expression in untreated dendritic cells. Thus, this gene product may be important in dendritic cell activation.

Significant expression of this gene is also seen in liver cirrhosis. This gene encodes a putative GPCR; therefore, antibodies or small molecule therapeutics could reduce or inhibit fibrosis that occurs in liver cirrhosis. In addition, antibodies to this putative GPCR could also be used for the diagnosis of liver cirrhosis. In addition, significant expression of this gene is seen in resting macrophages. The putative GPCR encoded for by this transcript may therefore be important in macrophage detection of chemokine gradients and trafficking into specific sites within a tissue and in activation. Antibody or protein therapeutics designed against the protein encoded for by this transcript could reduce or inhibit inflammation in asthma, emphysema, allergy, psoriasis, arthritis, or any other condition in which macrophage localization/activation is important.

Ag3010 Expression of this gene is low/undetectable (CTs > 35) across all of the samples on this panel (data not shown).

### NOV90

Expression of NOV90 CG57676-01 was assessed using the primer-probe set Gpcr45, described in Table BXA.

**Table BXA. Probe Name Gpcr45**

| **Primers** | **Sequences** | **Length** | **Start Position** | **SEQ ID NO** |
|---|---|---|---|---|
| Forward | 5'-tgctattgcccaggctgtact-3' | 21 | 676 | 536 |
| Probe | TET-5'-aggatgcagtcaaccactgggettcag-3'-TAMRA | 27 | 698 | 537 |
| Reverse | 5'-tgagctccacgttccaaatactt-3' | 25 | 726 | 538 |

**Panel 1.1 Summary:** G-por45 The results from two experiments with the same probe and primer set do not correlate and no conclusions can be drawn from this data (data not shown).

### NOV91

Expression of NOV91/CG57678-01 was assessed using the primer-probe sets Ag2497, Ag3306 and Gpcr22, described in Tables BYA, BYB and BYC. Results of the RTQ-PCR runs are shown in Tables BYD, and BYE

**Table BYA. Probe Name Ag2497**

| **Primers** | **Sequences** | **Length** | **Start Position** | **SEQ ID NO** |
|---|---|---|---|---|
| Forward | 5'-gtccttgaaggatccaaaactc-3' | 22 | 162 | 539 |
| Probe | TET-5'-tttctttctttccaacctttccttgg-3'-TAMRA | 26 | 198 | 540 |
| Reverse | 5'-ctgctggtcaaacagaggtcta-3' | 22 | 224 | 541 |

**Table BYB. Probe Name Ag3306**

| **Primers** | **Sequences** | **Length** | **Start Position** | **SEQ ID NO** |
|---|---|---|---|---|
| Forward | 5'-gggcaaatttctcactcttttc-3' | 22 | 828 | 542 |
| Probe | TET-5'-ccccaactcttaatcccctcatctMa-3'-TAMRA | 27 | 863 | 543 |
| Reverse | 5-ccctttacctccttgttcctta-3' | 22 | 893 | 544 |

**Table BYC. Probe Name Gpcr22**

| **Primers** | **Sequences** | **Length** | **Start Position** | **SEQ ID NO** |
|---|---|---|---|---|
| Forward | 5'-aattcagatgtctatcgccagtgtt-3' | 25 | 606 | 545 |
| Probe | TET-5'-tcctcctggtgatgcccttgatcattatc-3'-TAMRA | 29 | 632 | 546 |
| Reverse | 5'-ttagcaatagcaccagaagaggaaa-3' | 25 | 662 | 547 |

**Table BYD. CNS neurodegeneration v1.0**

| **Tissue Name** | **Rel. Exp.(%) Ag2497, Run 208122050** | **Tissue Name** | **Rel. Exp.(%) Ag2497, Run 208122050** |
|---|---|---|---|
| AD 1 Hippo | 16.4 | Control (Path) 3 Temporal Ctx | 0.0 |
| AD 2 Hippo | 20.6 | Control (Path) 4 Temporal Ctx | 0.0 |
| AD 3 Hippo | 22.8 | AD 1 Occipital Ctx | 0.0 |
| AD 4 Hippo | 0.0 | AD 2 Occipital Ctx (Missing) | 0.0 |
| AD 5 Hippo | 0.0 | AD 3 Occipital Ctx | 0.0 |
| AD 6 Hippo | 87.1 | AD 4 Occipital Ctx | 0.0 |
| Control 2 Hippo | 6.2 | AD 5 Occipital Ctx | 0.0 |
| Control 4 Hippo | 30.6 | AD 6 Occipital Ctx | 0.0 |
| Control (Path) 3 Hippo | 25.0 | Control 1 Occipital Ctx | 0.0 |
| AD 1 Temporal Ctx | 0.0 | Control 2 Occipital Ctx | 0.0 |
| AD 2 Temporal Ctx | 6.9 | Control 3 Occipital Ctx | 0.0 |
| AD 3 Temporal Ctx | 0.0 | Control 4 Occipital Ctx | 0.0 |
| AD 4 Temporal Ctx | 0.0 | Control (Path)1 Occipital Ctx | 5.6 |
| AD 5 Inf Temporal Ctx | 0.0 | Control (Path) 2 Occipital Ctx | 0.0 |
| AD 5 Sup Temporal Ctx | 100.0 | Control (Path) 3 Occipital Ctx | 0.0 |
| AD 6 Inf Temporal Ctx | 0.0 | Control (Path) 4 Occipital Ctx | 0.0 |
| AD 6 Sup Temporal Ctx | 0.0 | Control 1 Parietal Ctx | 0.0 |
| Control 1 Temporal Ctx | 0.0 | Control 2 Parietal Ctx | 0.0 |
| Control 2 Temporal Ctx | 0.0 | Control 3 Parietal Ctx | 0.0 |
| Control 3 Temporal Ctx | 0.0 | Control (Path) 1 Parietal Ctx | 0.0 |
| Control 3 Temporal Ctx | 0.0 | Control (Path) 2 Parietal Ctx | 0.0 |
| Control (Path) 1 Temporal Ctx | 0.0 | Control (Path) 3 Parietal Ctx | 0.0 |
| Control (Path) 2 Temporal Ctx | 0.0 | Control (Path) 4 Parietal Ctx | 0.0 |

**Table BYE. Panel 1.1**

| **Tissue Name** | | **Tissue Name** | |
|---|---|---|---|
| | **109664919** | | **109664919** |
| Adrenal gland | 0.0 | Renal ca. UO-31 | 7.9 |
| Bladder | 2.3 | Renal ca. RXF 393 | 0.0 |
| Brain (amygdala) | 0.0 | Liver | 0.0 |
| Brain (cerebellum) | 0.1 | Liver (fetal) | 5.3 |
| Brain (hippocampus) | 0.0 | Liver ca.(hepatoblast) HenG2 | 0.0 |
| Brain (substantia nigra) | 6.9 | Lung | 0.1 |
| Brain (thalamus) | 7.2 | Lung (fetal) | 0.6 |
| Cerebral Cortex | 1.0 | Lung ca. (non-s.cell) HOP-62 | 18.4 |
| Brain (fetal) | 0.1 | Lung ca. (large cell)NCI-H460 | 0.0 |
| Brain (whole) | 0.1 | Lung ca. (non-s.cell) NCI-H23 | 18.2 |
| glio/astro U-118-MG | 0.0 | Lung ca.(non-s.cl)NCI-H522 | 16.4 |
| astrocytoma SF-539 | 0.7 | Lung ca. (non-sm. cell) A549 | 8.6 |
| astrocytoma SNB-75 | 0.0 | Lung ca. (s.cell var.) SHP-77 | 1.0 |
| astrocytoma SW1783 | 0.0 | Lung ca.(small cell) LX-1 | 0.0 |
| glioma U251 | 0.0 | Lung ca. (small cell) NCI-H69 | 57.4 |
| glioma SF-295 | 0.0 | Lung ca. (squam) SW 900 | 4.3 |
| glioma SNB-19 | 10.7 | Lung ca. (squam) NCI-H596 | 9.2 |
| glio/astro U87-MG | 0.0 | Lymph node | 0.0 |
| neuro*; met SK-N-AS | 0.0 | spleen | 0.0 |
| Mammary gland | 0.1 | Thymus | 0.5 |
| Breast ca. BT-549 | 1.8 | Ovary | 6.6 |
| Breast ca. MDA-N | 1.1 | Ovarian ca. IGROV-1 | 0.5 |
| Breast ca.* (pl.ef) T47D | 25.7 | Ovarian ca. OVCAR-3 | 14.2 |
| Breast ca.* (pl.ef) MCF-7 | 2.5 | Ovarian ca. OVCAR-4 | 3.2 |
| Breast ca.* (pl.ef) MDA-MB-231 | 0.0 | Ovarian ca. OVCAR-5 | 51.1 |
| Small intestine | 0.0 | Ovarian ca. OVCAR-8 | 6.3 |
| Colorectal | 4.0 | Ovarian ca.* (ascites) SK-OV-3 | 62.4 |
| Colon ca. HT29 | 5.7 | Pancreas | 4.5 |
| Colon ca. CaCo-2 | 2.3 | Pancreatic ca. CAPAN 2 | 0.0 |
| Colon ca. HCT-15 | 12.2 | Pituitary gland | 3.7 |
| Colon ca. HCR-116 | 0.0 | Placenta | 0.0 |
| Colon ca.HCC-2998 | 1.3 | prostate | 0.0 |
| Colon ca. SW480 | 0.8 | Prostate ca.* (bone met) PC-3 | 0.5 |
| Colon ca.*SW620 (SW480 met) | 0.0 | Salivary gland | 0.6 |
| Stomach | 0.0 | Trachea | 0.0 |
| Gastnc ca. (liver met) NCI-N87 | 8.2 | Spinal cord | 10.4 |
| Heart | 0.0 | Testis | 100.0 |
| Skeletal muscle (Fetal) | 0.3 | Thyroid | 0.0 |
| Skeletal muscle | 0.4 | Uterus | 0.0 |
| Endothilial cells | 0.1 | MelanomaM14 | 8.4 |
| Heart (Fetal) | 0.3 | Melanoma LOX IMVI | 0.0 |
| Kidney | 0.3 | Melanoma UACC-62 | 0.0 |
| Kidney (fetal) | 0.0 | Melanoma SK-MEL-28 | 1.0 |
| Renal ca. 786-0 | 2.3 | Melanoma*(met) SK-MEL-5 | 0.0 |
| Renal ca. A498 | 1.1 | Melanoma Hs688 (A). T | 0.0 |
| Renal ca. ACHN | 0.0 | Melanoma*(met) Hs688(B).T | 0.5 |
| Renal ca. TK-10 | 0.2 | | |

**CNS_neurodegeneration_v1.0 Summary:** Ag2497 A low level of expression of the CG57678-01 gene is detected with samples derived from one of the Alzheimer's patient hippocampus (CT=34.3) and sup temporal cortex (CT=34.1). Therefore, expression of this gene may be used to distinguish these samples from the other samples on this panel. This gene encodes a putative GPCR; therefore, therapeutic modulation of this gene or its protein product may be beneficial in the treatment of one or more of CNS disorders, as may stimulation and/or blockade of the receptor coded for by the gene.

Ag3306 Expression of this gene is low/undetectable (CTs> 35) across all of the samples on this panel (data not shown).

**General_screening_panel_v1.4 Summary:** Ag3306 Expression of the CG57678-01 gene is low/undetectable (CTs> 35) across all of the samples on this panel (data not shown).

**Panel 1.1** Summary: Gpcr22 Expression of the CG57678-01 gene is highest in testis (CT = 29.2). In addition, this gene is expressed at significant levels in some regions of the central nervous system, including spinal cord, substantia nigra and thalamus. The CG57678-01 gene encodes a putative GPCR. Several neurotransmitter receptors are GPCRs, including the dopamine receptor family, the serotonin receptor family, the GABAB receptor, muscarinic acetylcholine receptors, and others; thus this GPCR may represent a novel neurotransmitter receptor. Targeting various neurotransmitter receptors (dopamine, serotonin) has proven to be an effective therapy in psychiatric illnesses such as schizophrenia, bipolar disorder, and depression. Furthermore, the cerebral cortex and hippocampus are regions of the brain that are known to be involved in Alzheimer's disease, seizure disorders, and in the normal process of memory formation. Therefore, therapeutic modulation of this gene or its protein product may be beneficial in the treatment of one or more of these diseases, as may stimulation and/or blockade of the receptor coded for by the gene.

In addition, expression of this gene is upregulated in a number of lung cancer cell lines compared to normal lung. Therefore, therapeutic modulation of the activity of this gene or its protein product, using small molecule drugs, antibodies or protein therapeutics, may be of benefit in the treatment of lung cancer.

**Panel 1.3D Summary:** Ag2497 Expression of the CG57678-01 gene is low/undetectable (CTs > 35) across all of the samples on this panel (data not shown).

**Panel 2.2 Summary:** Ag2497 Expression of the CG57678-01 gene is low/undetectable (CTs > 34.5) across all of the samples on this panel (data not shown).

**Panel 4D Summary:** Ag2497 Expression of the CG57678-01 gene is low/undetectable (CTs > 34.5) across all of the samples on this panel (data not shown).

### Example 3. SNP analysis of NOVX clones

SeqCallingTM Technology: cDNA was derived from various human samples representing multiple tissue types, normal and diseased states, physiological states, and developmental states from different donors. Samples were obtained as whole tissue, cell lines, primary cells or tissue cultured primary cells and cell lines. Cells and cell lines may have been treated with biological or chemical agents that regulate gene expression for example, growth factors, chemokines, steroids. The cDNA thus derived was then sequenced using CuraGen's proprietary SeqCalling technology. Sequence traces were evaluated manually and edited for corrections if appropriate. cDNA sequences from all samples were assembled with themselves and with public ESTs using bioinformatics programs to generate CuraGen's human SeqCalling database of SeqCalling assemblies. Each assembly contains one or more overlapping cDNA sequences derived from one or more human samples. Fragments and ESTs were included as components for an assembly when the extent of identity with another component of the assembly was at least 95% over 50 bp. Each assembly can represent a gene and/or its variants such as splice forms and/or single nucleotide polymorphisms (SNPs) and their combinations.

Variant sequences are included in this application. A variant sequence can include a single nucleotide polymorphism (SNP). A SNP can, in some instances, be referred to as a "cSNP" to denote that the nucleotide sequence containing the SNP originates as a cDNA. A SNP can arise in several ways. For example, a SNP may be due to a substitution of one nucleotide for another at the polymorphic site. Such a substitution can be either a transition or a transversion. A SNP can also arise from a deletion of a nucleotide or an insertion of a nucleotide, relative to a reference allele. In this case, the polymorphic site is a site at which one allele bears a gap with respect to a particular nucleotide in another allele. SNPs occurring within genes may result in an alteration of the amino acid encoded by the gene at the position of the SNP. Intragenic SNPs may also be silent, however, in the case that a codon including a SNP encodes the same amino acid as a result of the redundancy of the genetic code. SNPs occurring outside the region of a gene, or in an intron within a gene, do not result in changes in any amino acid sequence of a protein but may result in altered regulation of the expression pattern for example, alteration in temporal expression, physiological response regulation, cell type expression regulation, intensity of expression, stability of transcribed message.

Method of novel SNP Identification: SNPs are identified by analyzing sequence assemblies using CuraGen's proprietary SNPTool algorithm. SNPTool identifies variation in assemblies with the following criteria: SNPs are not analyzed within 10 base pairs on both ends of an alignment; Window size (number of bases in a view) is 10; The allowed number of mismatches in a window is 2; Minimum SNP base quality (PHRED score) is 23; Minimum number of changes to score an SNP is 2/assembly position. SNPTool analyzes the assembly and displays SNP positions, associated individual variant sequences in the assembly, the depth of the assembly at that given position, the putative assembly allele frequency, and the SNP sequence variation. Sequence traces are then selected and brought into view for manual validation. The consensus assembly sequence is imported into CuraTools along with variant sequence changes to identify potential amino acid changes resulting from the SNP sequence variation. Comprehensive SNP data analysis is then exported into the SNPCalling database.

**Method of novel SNP Confirmation:** SNPs are confirmed employing a validated method know as Pyrosequencing (Pyrosequencing, Westborough, MA). Detailed protocols for Pyrosequencing can be found in: Alderborn et al. Determination of Single Nucleotide Polymorphisms by Real-time Pyrophosphate DNA Sequencing. (2000). *Genome Research.* 10, Issue 8, August. 1249-1265. In brief, Pyrosequencing is a real time primer extension process of genotyping. This protocol takes double-stranded, biotinylated PCR products from genomic DNA samples and binds them to streptavidin beads. These beads are then denatured producing single stranded bound DNA. SNPs are characterized utilizing a technique based on an indirect bioluminometric assay of pyrophosphate (PPi) that is released from each dNTP upon DNA chain elongation. Following Klenow polymerase-mediated base incorporation, PPi is released and used as a substrate, together with adenosine 5'-phosphosulfate (APS), for ATP sulfurylase, which results in the formation of ATP. Subsequently, the ATP accomplishes the conversion of luciferin to its oxi-derivative by the action of luciferase. The ensuing light output becomes proportional to the number of added bases, up to about four bases. To allow processivity of the method dNTP excess is degraded by apyrase, which is also present in the starting reaction mixture, so that only dNTPs are added to the template during the sequencing. The process has been fully automated and adapted to a 96-well format, which allows rapid screening of large SNP panels. The DNA and protein sequences for the novel single nucleotide polymorphic variants are reported. Variants are reported individually but any combination of all or a select subset of variants are also included. In addition, the positions of the variant bases and the variant amino acid residues are underlined.

### Results

Variants are reported individually but any combination of all or a select subset of variants are also included as contemplated NOVX embodiments of the invention.

### NOV36a SNP data:

NOV36a has 4 SNP variants, whose variant positions for their nucleotide and amino acid sequences is numbered according to SEQ ID NOs:81 and 82, respectively. The nucleotide sequence of the NOV36a variants differs as shown in Table SNP1.

| **Table SNP1. cSNP and Coding Variants for NOV36a** | | | | |
|---|---|---|---|---|
| **NT Position of cSNP** | **Wild Type NT** | **Variant NT** | **Amino Acid position** | **Amino Acid Change** |
| 284 | T | C | 87 | Phe to Ser |
| 307 | G | A | 95 | Gly to Ser |
| 354 | A | G | 110 | Ala to Ala |
| 379 | T | C | 119 | Ser to Pro |

### NOV55 SNP data:

NOV55 has 5 SNP variants, whose variant positions for their nucleotide and amino acid sequences is numbered according to SEQ ID NOs:127 and 128, respectively. The nucleotide sequence of the NOV55 variants differs as shown in Table SNP2.

| **Table SNP2. cSNP and Coding Variants for NOV55** | | | | |
|---|---|---|---|---|
| **NT Position of cSNP** | **Wild Type NT** | **Variant NT** | **Amino Acid position** | **Amino Acid Change** |
| 149 | A | T | 45 | Ser to Cys |
| 227 | G | A | 71 | Glu to Lys |
| 304 | C | T | 96 | His to His |
| 2542 | T | C | 842 | His to His |
| 2838 | T | C | 941 | Ile to Thr |

### NOV SNP19 data:

NOV19 has one SNP variant, whose variant positions for their nucleotide and amino acid sequences is numbered according to SEQ ID NOs:47 and 48, respectively. The nucleotide sequence of the NOV19 variants differs as shown in Table SNP3.

| **Table SNP3. cSNP and Coding Variants for NOV19** | | | | |
|---|---|---|---|---|
| **NT Position of cSNP** | **Wild Type NT** | **Variant NT** | **Amino Acid position** | **Amino Acid Change** |
| 893 | C | T | 289 | Ala to Val |

### NOV8a SNP data:

NOV8a has two SNP variants, whose variant positions for their nucleotide and amino acid sequences is numbered according to SEQ ID NOs:17 and 18, respectively. The nucleotide sequence of the NOV8a variants differs as shown in Table SNP4.

| **Table SNP4. cSNP and Coding Variants for NOV8a** | | | | |
|---|---|---|---|---|
| **NT Position of cSNP** | **Wild Type NT** | **Variant NT** | **Amino Acid position** | **Amino Acid Change** |
| 1199 | T | C | 380 | Leu to Pro |
| 1213 | G | A | 385 | Gly to Ser |

### NOV62a SNP data:

NOV62a has three SNP variants, whose variant positions for their nucleotide and amino acid sequences is numbered according to SEQ ID NOs:145 and 156, respectively. The nucleotide sequence of the NOV62a variants differs as shown in Table SNP5.

| **Table SNP5. cSNP and Coding Variants for NOV62a** | | | | |
|---|---|---|---|---|
| **NT Position of cSNP** | **Wild Type NT** | **Variant NT** | **Amino Acid position** | **Amino Acid Change** |
| 1197 | G | A | 366 | Ala to Thr |
| 1237 | A | G | 379 | Asp to Gly |
| 1285 | C | T | 395 | Ala to Val |

### NOV SNPlla data:

NOV11a has four SNP variants, whose variant positions for their nucleotide and amino acid sequences is numbered according to SEQ ID NOs:25 and 26, respectively. The nucleotide sequence of the NOV11a variants differs as shown in Table SNP6.

| **Table SNP6. cSNP and Coding Variants for NOV11a** | | | | |
|---|---|---|---|---|
| **NT Position of cSRP** | **Wild Type NT** | **Variant NT** | **Amino Acid position** | **Amino Acid Change** |
| 636 | C | T | 212 | No change |
| 1089 | G | A | 363 | Met to Ile |
| 5654 | A | G | 1885 | Gln to Art |
| 5914 | C | A | 0 | Silent |

### NOV25 SNP data:

NOV25 has one SNP variant, whose variant positions for their nucleotide and amino acid sequences is numbered according to SEQ ID NOs:59 and 60, respectively. The nucleotide sequence of the NOV25 variants differs as shown in Table SNP7.

| **Table SNP7. cSNP and Coding Variants for NOV25** | | | | |
|---|---|---|---|---|
| **NT Position of cSNP** | **Wild Type NT** | **Variant NT** | **Amino Acid position** | **Amino Acid Change** |
| 1836 | C | T | 612 | No change |

### NOV10 SNP data:

NOV10 has one SNP variant, whose variant positions for their nucleotide and amino acid sequences is numbered according to SEQ ID NOs:23 and 24, respectively. The nucleotide sequence of the NOV10 variant differs as shown in Table SNP8.

| **Table SNP8. cSNP and Coding Variants for NOV10** | | | | |
|---|---|---|---|---|
| **NT Position of cSNP** | **Wild Type NT** | **Variant NT** | **Amino Acid position** | **Amino Acid Change** |
| 715 | T | C | 239 | Ser to Pro |

### NOV3 SNP data:

NOV3 has two SNP variants, whose variant positions for their nucleotide and amino acid sequences is numbered according to SEQ ID NOs:5 and 6, respectively. The nucleotide sequence of the NOV3 variants differs as shown in Table SNP9.

| **Table SNP9. cSNP and Coding Variants for NOV3** | | | | |
|---|---|---|---|---|
| **NT Position of cSNP** | **Wild Type NT** | **Variant NT** | **Amino Acid position** | **Amino Acid Change** |
| 127 | C | T | 9 | No change |
| 158 | G | A | 20 | Val to Met |

### NOV44a SNP data:

NOV44a has four SNP variants, whose variant positions for their nucleotide and amino acid sequences is numbered according to SEQ ID NOs:101 and 102, respectively. The nucleotide sequence of the NOV44a variants differs as shown in Table SNP10.

| **Table SNP10. cSNP and Coding Variants for NOV44a** | | | | |
|---|---|---|---|---|
| **NT Position of cSRP** | **Wild Type NT** | **Variant NT** | **Amino Acid position** | **Amino Acid Change** |
| 56 | G | T | 7 | Gly to Val |
| 164 | C | A | 43 | Pro to His |
| 422 | G | A | 129 | Gly to Asp |
| 500 | T | C | 155 | Leu to Pro |

### NOV57 SNP data:

NOV57 has one SNP variant, whose variant positions for its nucleotide and amino acid sequences is numbered according to SEQ ID NOs:131 and 132, respectively. The nucleotide sequence of the NOV57 variant differs as shown in Table SNP11.

| **Table SNP11. cSNP and Coding Variants for NOV57** | | | | |
|---|---|---|---|---|
| **NT Position of cSNP** | **Wild Type NT** | **Variant NT** | **Amino Acid position** | **Amino Acid Change** |
| 1214 | C | T | 362 | No change |

### NOV15 SNP data:

NOV15 has 14 SNP variants, whose variant positions for their nucleotide and amino acid sequences is numbered according to SEQ ID NOs:39 and 40, respectively. The nucleotide sequence of the NOV15 variants differs as shown in Table SNP12.

| **Table SNP12. cSNP and Coding Variants for NOV15** | | | | |
|---|---|---|---|---|
| **NT Position of cSNP** | **Wild Type NT** | **Variant NT** | **Amino Acid position** | **Amino Acid Change** |
| 136 | T | C | 11 | No change |
| 187 | T | C | 28 | No change |
| 189 | A | G | 29 | Asp to Gly |
| 259 | A | G | 52 | No change |
| 266 | C | T | 55 | Gln to stop |
| 434 | C | T | 111 | Pro to Ser |
| 998 | A | G | 299 | Ile to Val |
| 1252 | G | A | 383 | No change |
| 1409 | A | G | 436 | Thr to Ala |
| 1428 | T | C | 442 | Leu to Ser |
| 1429 | G | A | 442 | No change |
| 1935 | A | G | 611 | His to Arg |
| 1967 | G | T | 622 | Gly to Cys |
| 2063 | G | C | 654 | Ala to Pro |

### NOV21 SNP data:

NOV21 has one SNP variant, whose variant positions for its nucleotide and amino acid sequences is numbered according to SEQ ID NOs:51 and 52, respectively. The nucleotide sequence of the NOV21 variants differs as shown in Table SNP13.

| **Table SNP13. cSNP and Coding Variants for NOV21** | | | | |
|---|---|---|---|---|
| **NT Position of cSNP** | **Wild Type NT** | **Variant NT** | **Amino Acid position** | **Amino Acid Change** |
| 814 | T | C | 196 | No change |

### NOV45a SNP data:

NOV45a has one SNP variant, whose variant positions for its nucleotide and amino acid sequences is numbered according to SEQ ID NOs:105 and 106, respectively. The nucleotide sequence of the NOV45a variants differs as shown in Table SNP14.

| **Table SNP14. cSNP and Coding Variants for NOV45a** | | | | |
|---|---|---|---|---|
| **NT Position of cSNP** | **Wild Type NT** | **Variant NT** | **Amino Acid position** | **Amino Acid Change** |
| 4091 | A | G | 1353 | Thr to Ala |

### NOV54 SNP data:

NOV54 has one SNP variant, whose variant positions for its nucleotide and amino acid sequences is numbered according to SEQ ID NOs:125 and 126, respectively. The nucleotide sequence of the NOV54 variants differs as shown in Table SNP15.

| **Table SNP15. cSNP and Coding Variants for NOV54** | | | | |
|---|---|---|---|---|
| **NT Position of cSNP** | **Wild Type NT** | **Variant NT** | **Amino Acid position** | **Amino Acid Change** |
| 3208 | C | T | Silent | No change |

### NOV69a SNP data:

NOV69a has four SNP variants, whose variant positions for their nucleotide and amino acid sequences is numbered according to SEQ ID NOs:163 and 164, respectively. The nucleotide sequence of the NOV69a variants differs as shown in Table SNP16.

| **Table SNP16. cSNP and Coding Variants for NOV69a** | | | | |
|---|---|---|---|---|
| **NT Position of cSNP** | **Wild Type NT** | **Variant NT** | **Amino Acid position** | **Amino Acid Change** |
| 1052 | G | A | 327 | No change |
| 1171 | A | G | 367 | Lys to Arg |
| 1186 | A | G | 372 | Glu to Gly |
| 1316 | C | A | 415 | No change |

### NOV58 SNP data:

NOV58 has three SNP variants, whose variant positions for their nucleotide and amino acid sequences is numbered according to SEQ ID NOs: 133 and 134, respectively. The nucleotide sequence of the NOV58 variants differs as shown in Table SNP17.

| **Table SNP17. cSNP and Coding Variants for NOV58** | | | | |
|---|---|---|---|---|
| **NT Position of cSNP** | **Wild Type NT** | **Variant NT** | **Amino Acid position** | **Amino Acid Change** |
| 579 | C | T | 174 | Gln to Stop |
| 598 | C | T | 180 | Ala to Val |
| 637 | A | G | 193 | Asp to Gly |

### Example 4. In-frame Cloning

### NOV45b

For NOV45b, the cDNA coding for the DOMAIN of NOV45a (CG50718-02) from residues 511 to 705 was targeted for "in-frame" cloning by PCR. The PCR template was based on the previously identified plasmid, when available, or on human cDNA(s).

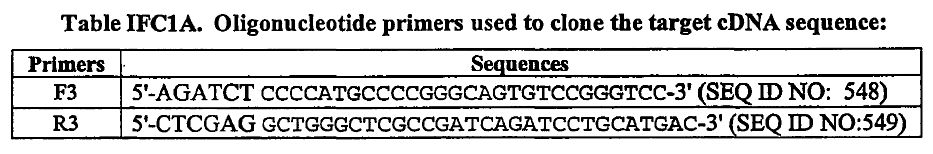

For downstream cloning purposes, the forward primer includes an in-frame Bg1II restriction site and the reverse primer contains an in-frame Xho I restriction site.

An amplified product was detected by agarose gel electrophoresis. The fragment was gel-purified and ligated into the pCR2.1 vector (Invitrogen, Carlsbad, CA) following the manufacturer's recommendation. Twelve clones per PCR reaction were picked and sequenced. The inserts were sequenced using vector-specific M13 Forward and M13 Reverse primers and the following gene-specific primers:

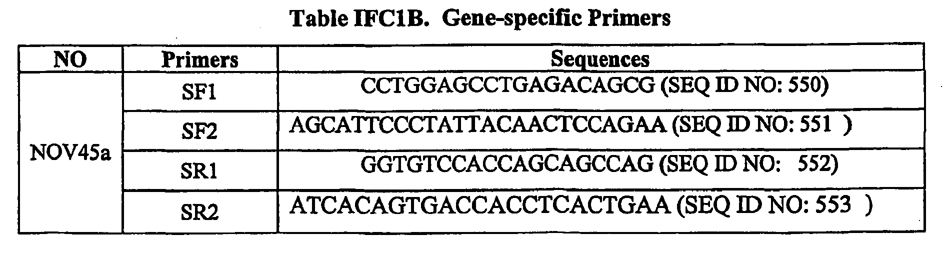

### NOV7

For NOV7, the cDNA coding for a domain of CG57595-01 from residue 2191 to 2450 was targeted for "in-frame" cloning by PCR. The PCR template is based on human cDNA(s).

The following oligonucleotide primers were used to clone the target cDNA sequence:

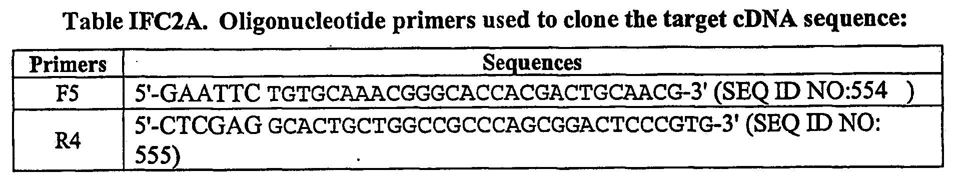

For downstream cloning purposes, the forward primer includes an in-frame EcoRI restriction site and the reverse primer contains an in-frame XhoI restriction site.

An amplified product was detected by agarose gel electrophoresis. The fragment was gel-purified and ligated into the pCR2.1 vector (Invitrogen, Carlsbad, CA) following the manufacturer's recommendation. Twelve clones per PCR reaction were picked and sequenced. The inserts were sequenced using vector-specific M13 Forward and M13 Reverse primers and the following gene-specific primers:

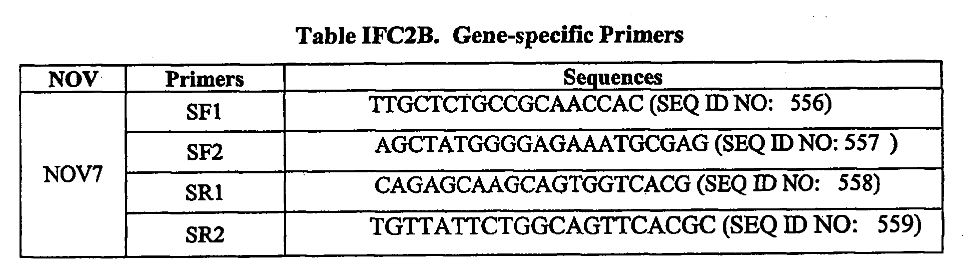

The cDNA coding for a domain of the full length form onf CG57595-01 between residues 1885 and 2450 was targeted for "in-frame" cloning by PCR. The PCR template is based on human cDNA(s).

The following oligonucleotide primers were used to clone the target cDNA sequence:

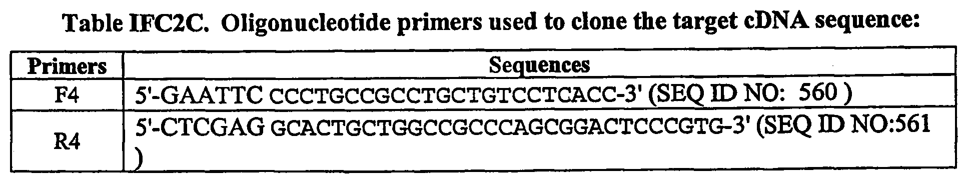

For downstream cloning purposes, the forward primer includes an in-frame EcoRI restriction site and the reverse primer contains an in-frame XhoI restriction site.

An amplified product was detected by agarose gel electrophoresis. The fragment was gel-purified and ligated into the pCR2.1 vector (Invitrogen, Carlsbad, CA) following the manufacturer's recommendation. Twelve clones per PCR reaction were picked and sequenced. The inserts were sequenced using vector-specific M13 Forward and M13 Reverse primers and the following gene-specific primers:

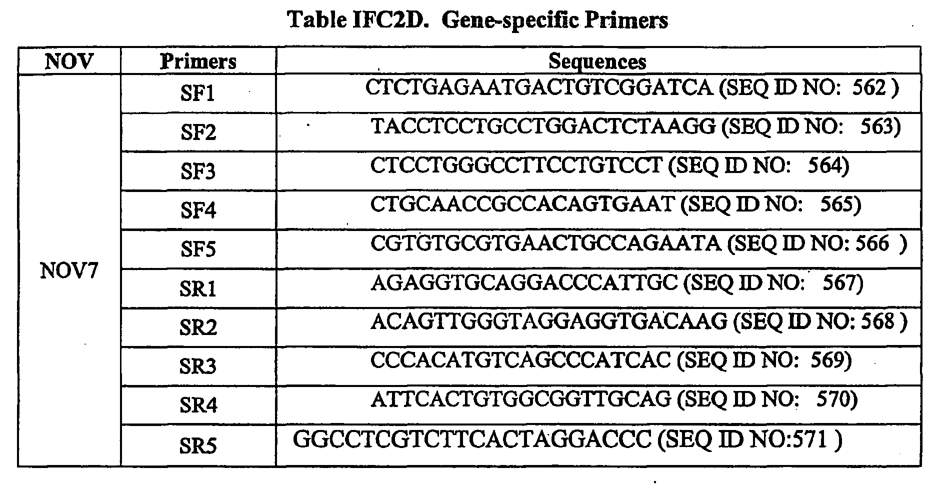

### NOV8

The cDNA coding for a domain of CG57452-01 from residue 156 to 815 was targeted for "in-frame" cloning by PCR. The PCR template is based on human cDNA(s).

The following oligonucleotide primers were used to clone the target cDNA sequence:

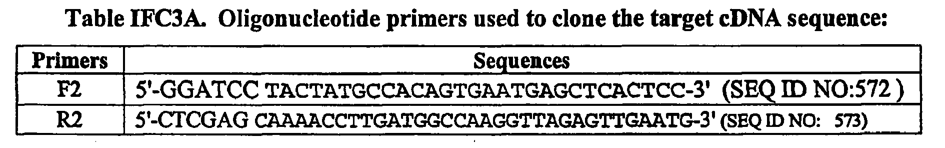

For downstream cloning purposes, the forward primer includes an in-frame BamHI restriction site and the reverse primer contains an in-frame XhoI restriction site.

An amplified product was detected by agarose gel electrophoresis. The fragment was gel-purified and ligated into the pCR2.1 vector (Invitrogen, Carlsbad, CA) following the manufacturer's recommendation. Twelve clones per PCR reaction were picked and sequenced. The inserts were sequenced using vector-specific M13 Forward and M13 Reverse primers and the following gene-specific primers:
9. The nucleic acid molecule of claim 5, wherein said nucleic acid molecule comprises a nucleotide sequence selected from the group consisting of:
   (a) a nucleotide sequence selected from the group consisting of SEQ ID NO:2*n*-1, wherein *n* is any integer 1-107;
   (b) a nucleotide sequence differing by one or more nucleotides from a nucleotide sequence selected from the group consisting of SEQ ID NO:2*n*-1, wherein n is any integer 1-107, provided that no more than 20% of the nucleotides differ from said nucleotide sequence;
   (c) a nucleic acid fragment of (a); and
   (d) a nucleic acid fragment of (b).
10. The nucleic acid molecule of claim 5, wherein said nucleic acid molecule hybridizes under stringent conditions to a nucleotide sequence chosen from the group consisting SEQ ID NO:2*n*-1, wherein n is any integer 1-107, or a complement of said nucleotide sequence.
11. The nucleic acid molecule of claim 5, wherein the nucleic acid molecule comprises a nucleotide sequence selected from the group consisting of:
   (a) a first nucleotide sequence comprising a coding sequence differing by one or more nucleotide sequences from a coding sequence encoding said amino acid sequence, provided that no more than 20% of the nucleotides in the coding sequence in said first nucleotide sequence differ from said coding sequence;
   (b) an isolated second polynucleotide that is a complement of the first polynucleotide; and
   (c) a nucleic acid fragment of (a) or (b).
12. A vector comprising the nucleic acid molecule of claim 11.
13. The vector of claim 12, further comprising a promoter operably-linked to said nucleic acid molecule.
14. A cell comprising the vector of claim 12.
15. An antibody that binds immunospecifically to the polypeptide or claim 1.
16. The antibody of claim 15, wherein said antibody is a monoclonal antibody.
17. The antibody of claim 15, wherein the antibody is a humanized antibody.
18. A method for determining the presence or amount of the polypeptide of claim 1 in a sample, the method comprising:
   (a) providing the sample;
   (b) contacting the sample with an antibody that binds immunospecifically to the polypeptide; and
   (c) determining the presence or amount of antibody bound to said polypeptide, thereby determining the presence or amount of polypeptide in said sample.
19. A method for determining the presence or amount of the nucleic acid molecule of claim 5 in a sample, the method comprising:
   (a) providing the sample;
   (b) contacting the sample with a probe that binds to said nucleic acid molecule; and
   (c) determining the presence or amount of the probe bound to said nucleic acid molecule,
   thereby determining the presence or amount of the nucleic acid molecule in said sample.
20. The method of claim 19 wherein presence or amount of the nucleic acid molecule is used as a marker for cell or tissue type.
21. The method of claim 20 wherein the cell or tissue type is cancerous.
22. A method of identifying an agent that binds to a polypeptide of claim 1, the method comprising:
   (a) contacting said polypeptide with said agent; and
   (b) determining whether said agent binds to said polypeptide.
23. The method of claim 22 wherein the agent is a cellular receptor or a downstream effector.
24. A method for identifying an agent that modulates the expression or activity of the polypeptide of claim 1, the method comprising:
   (a) providing a cell expressing said polypeptide;
   (b) contacting the cell with said agent, and
   (c) determining whether the agent modulates expression or activity of said polypeptide,
   whereby an alteration in expression or activity of said peptide indicates said agent modulates expression or activity of said polypeptide.
25. A method for modulating the activity of the polypeptide of claim 1, the method comprising contacting a cell sample expressing the polypeptide of said claim with a compound that binds to said polypeptide in an amount sufficient to modulate the activity of the polypeptide.
26. A method of treating or preventing a NOVX-associated disorder, said method comprising administering to a subject in which such treatment or prevention is desired the polypeptide of claim 1 in an amount sufficient to treat or prevent said NOVX-associated disorder in said subject.
27. The method of claim 26 wherein the disorder is selected from the group consisting of cardiomyopathy and atherosclerosis.
28. The method of claim 26 wherein the disorder is related to cell signal processing and metabolic pathway modulation.
29. The method of claim 26, wherein said subject is a human.
30. A method of treating or preventing a NOVX-associated disorder, said method comprising administering to a subject in which such treatment or prevention is desired the nucleic acid of claim 5 in an amount sufficient to treat or prevent said NOVX-associated disorder in said subject.
31. The method of claim 30 wherein the disorder is selected from the group consisting of cardiomyopathy and atherosclerosis.
32. The method of claim 30 wherein the disorder is related to cell signal processing and metabolic pathway modulation.
33. The method of claim 30, wherein said subject is a human.
34. A method of treating or preventing a NOVX-associated disorder, said method comprising administering to a subject in which such treatment or prevention is desired the antibody of claim 15 in an amount sufficient to treat or prevent said NOVX-associated disorder in said subject.
35. The method of claim 34 wherein the disorder is diabetes.
36. The method of claim 34 wherein the disorder is related to cell signal processing and metabolic pathway modulation.
37. The method of claim 34, wherein the subject is a human.
38. A pharmaceutical composition comprising the polypeptide of claim 1 and a pharmaceutically-acceptable carrier.
39. A pharmaceutical composition comprising the nucleic acid molecule of claim 5 and a pharmaceutically-acceptable carrier.
40. A pharmaceutical composition comprising the antibody of claim 15 and a pharmaceutically-acceptable carrier.
41. A kit comprising in one or more containers, the pharmaceutical composition of claim 38.
42. A kit comprising in one or more containers, the pharmaceutical composition of claim 39.
43. A kit comprising in one or more containers, the pharmaceutical composition of claim 40.
44. A method for determining the presence of or predisposition to a disease associated with altered levels of the polypeptide of claim 1 in a first mammalian subject, the method comprising:
   (a) measuring the level of expression of the polypeptide in a sample from the first mammalian subject; and
   (b) comparing the amount of said polypeptide in the sample of step (a) to the amount of the polypeptide present in a control sample from a second mammalian subject known not to have, or not to be predisposed to, said disease;
   wherein an alteration in the expression level of the polypeptide in the first subject as compared to the control sample indicates the presence of or predisposition to said disease.
45. The method of claim 44 wherein the predisposition is to a cancer.
46. A method for determining the presence of or predisposition to a disease associated with altered levels of the nucleic acid molecule of claim 5 in a first mammalian subject, the method comprising:
   (a) measuring the amount of the nucleic acid in a sample from the first mammalian subject; and
   (b) comparing the amount of said nucleic acid in the sample of step (a) to the amount of the nucleic acid present in a control sample from a second mammalian subject known not to have or not be predisposed to, the disease;
   wherein an alteration in the level of the nucleic acid in the first subject as compared to the control sample indicates the presence of or predisposition to the disease.
47. The method of claim 46 wherein the predisposition is to a cancer.
48. A method of treating a pathological state in a mammal, the method comprising administering to the mammal a polypeptide in an amount that is sufficient to alleviate the pathological state, wherein the polypeptide is a polypeptide having an amino acid sequence at least 95% identical to a polypeptide comprising an amino acid sequence of at least one of SEQ ID NO:2*n*, wherein *n* is any integer 1-107, or a biologically active fragment thereof.
49. A method of treating a pathological state in a mammal, the method comprising administering to the mammal the antibody of claim 15 in an amount sufficient to alleviate the pathological state.

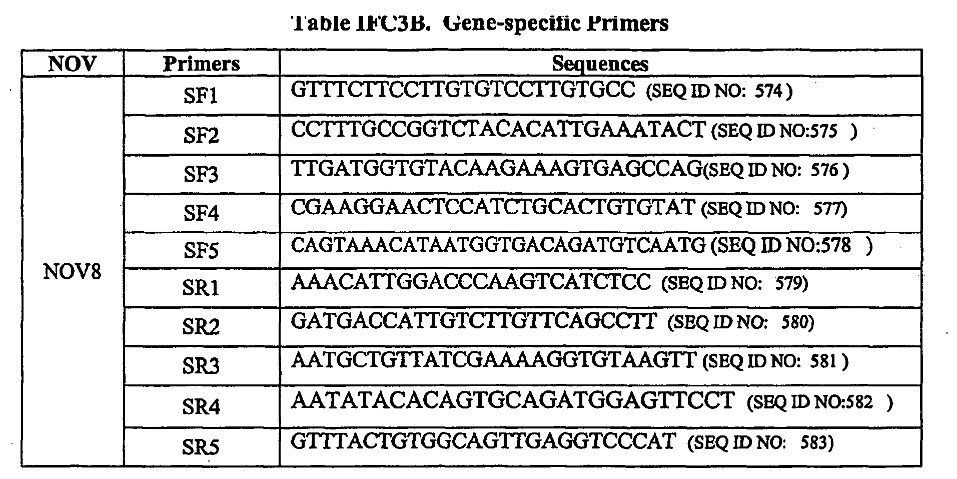

The cDNA coding for a domain of the full length CG57452-01 from residue 728 to 1382 was targeted for "in-frame" cloning by PCR. The PCR template is based human cDNA(s).

The following oligonucleotide primers were used to clone the target cDNA sequence:

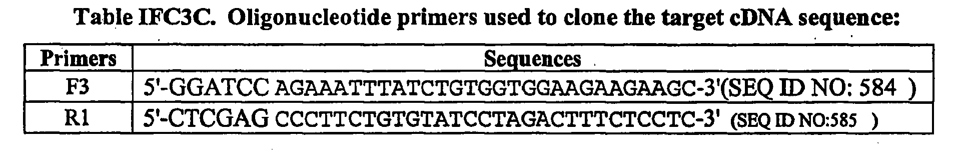

For downstream cloning purposes, the forward primer includes an in-frame BamHI restriction site and the reverse primer contains an in-frame XhoI restriction site.

An amplified product was detected by agarose gel electrophoresis. The fragment was gel-purified and ligated into the pCR2.1 vector (Invitrogen, Carlsbad, CA) following the manufacturer's recommendation. Twelve clones per PCR reaction were picked and sequenced. The inserts were sequenced using vector-specific M13 Forward and M13 Reverse primers and the following gene-specific primers:

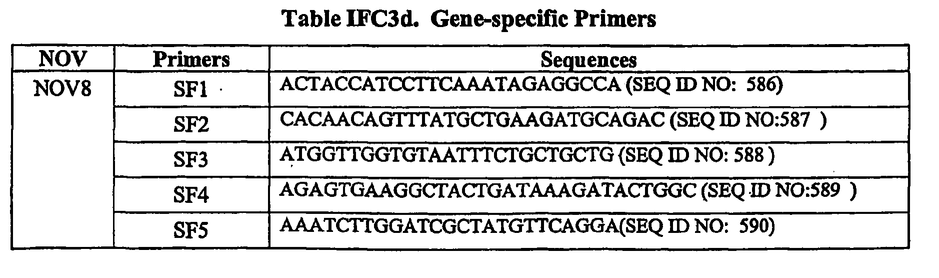

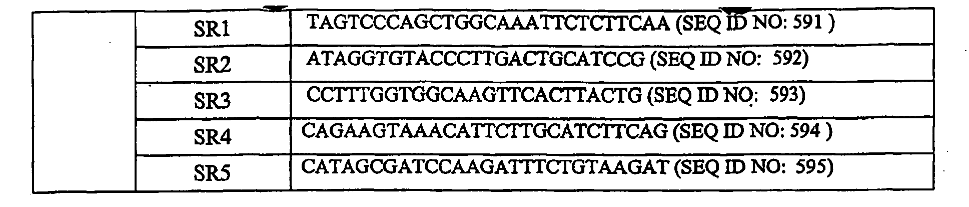

### NOV11

The cDNA coding for a domain of CG57488-03 from residue 121 to 741 was targeted for "in-frame" cloning by PCR. The PCR template is based on human cDNA(s).

The following oligonucleotide primers were used to clone the target cDNA sequence:
**Table IFC4A. Oligonucleotide primers used to clone the target cDNA sequence:**

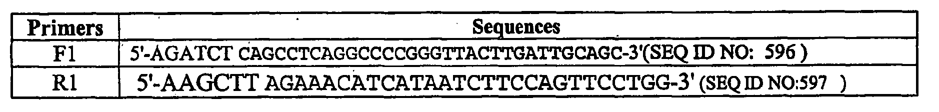

For downstream cloning purposes, the forward primer includes an in-frame.Bglll restriction site and the reverse primer contains an in-frame HindIII restriction site.

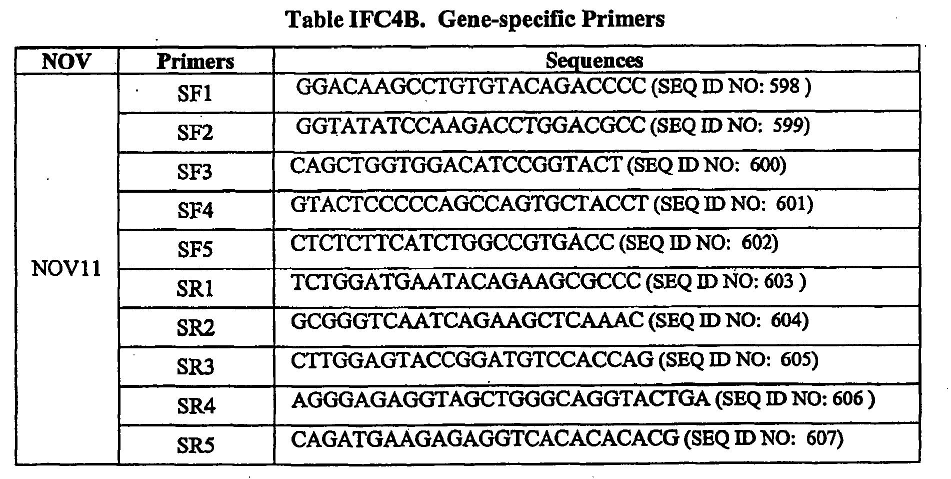

The cDNA coding for a domain of the full-length protein CG57488-03 residue 119 to 741. was targeted for "in-frame" cloning by PCR The PCR template is human cDNA(s).

The following oligonucleotide primers were used to clone the target cDNA sequence:

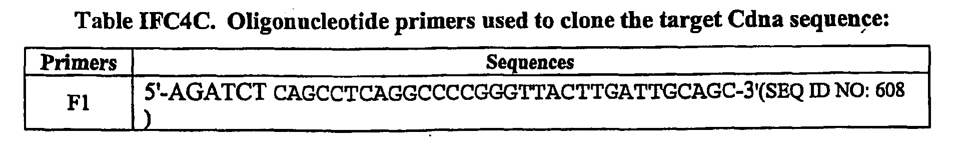

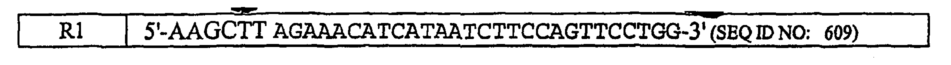

For downstream cloning purposes, the forward primer includes an in-frame BglII restriction site and the reverse primer contains an in-frame HindIII restriction site.

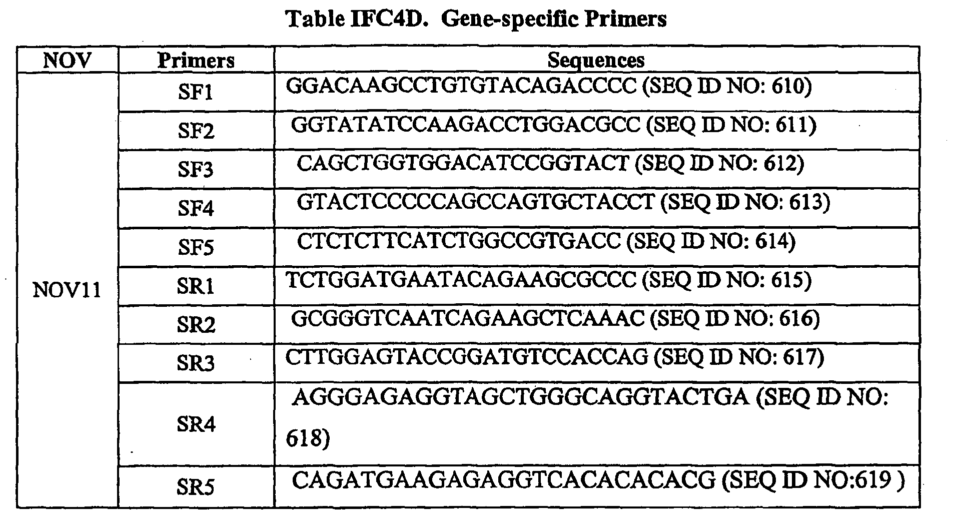

### NOV52

For NOV52, the cDNA coding for the full-length NOV52 (CG57748-01) was targeted for "in-frame" cloning by PCR. The PCR template was based on the previously identified plasmid, when available, or on human cDNA(s).

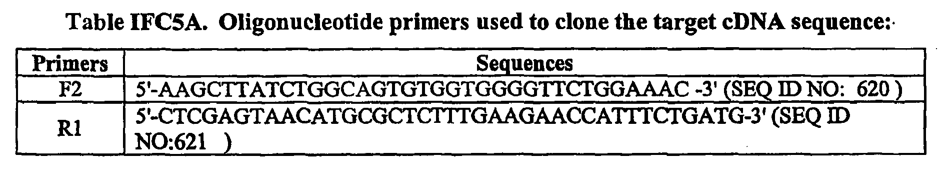

For downstream cloning purposes, the forward primer includes an in-frame HindIII restriction site and the reverse primer contains an in-frame Xho I restriction site.

An amplified product was detected by agarose gel electrophoresis. The fragment was gel-purified and ligated into the pCR2.1 vector (Invitrogen, Carlsbad, CA) following the manufacturer's recommendation. Twelve clones per PCR reaction were picked and sequenced. The inserts were sequenced using vector-specific M13 Forward and M13 Reverse primers and the following gene-specific primers:

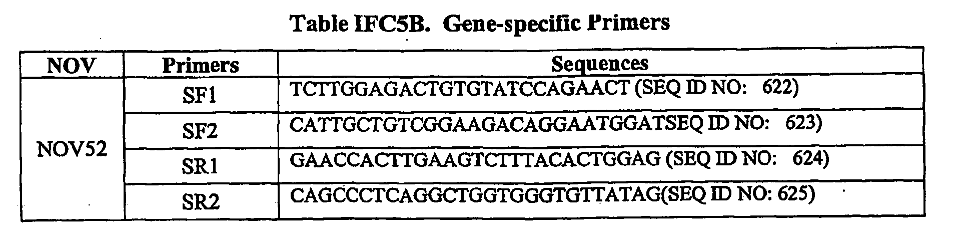

For NOV52, the cDNA coding for the domain of NOV52 (CG57748-01) from residue 225 to 412 was targeted for "in-frame" cloning by PCR. The PCR template was based on the previously identified plasmid, when available, or on human cDNA(s).

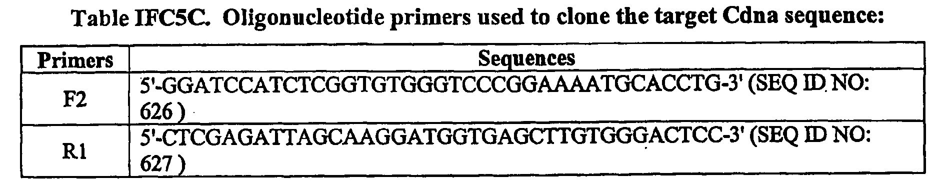

For downstream cloning purposes, the forward primer includes an in-frame BamHI restriction site and the reverse primer contains an in-frame Xho I restriction site.

An amplified product was detected by agarose gel electrophoresis. The fragment was gel-purified and ligated into the pCR2.1 vector (Invitrogen, Carlsbad, CA) following the manufacturer's recommendation. Twelve clones per PCR reaction were picked and sequenced. The inserts were sequenced using vector-specific M13 Forward and M13 Reverse primers and the following gene-specific primers:

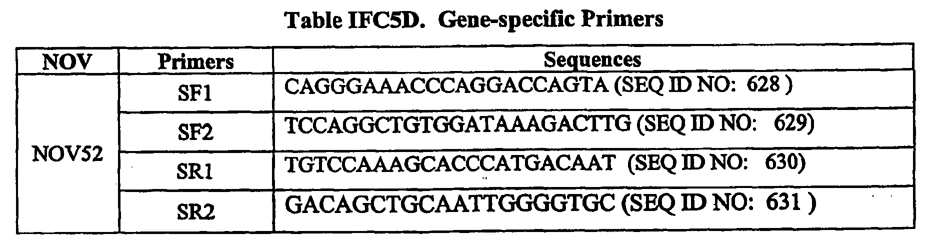

### NOV54

The cDNA coding for the mature form of the full length CG57707-01 from residue 23 to 543 was targeted for "in-frame" cloning by PCR. The PCR template is based on human cDNA(s).

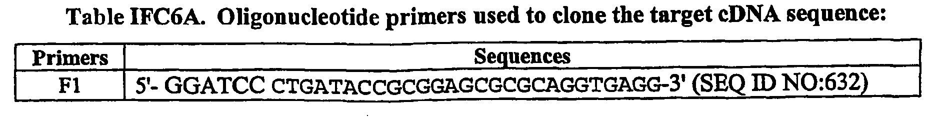

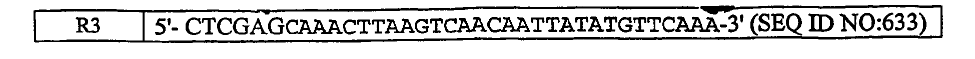

For downstream cloning purposes, the forward primer includes an in-frame BamHI restriction site and the reverse primer contains an in-frame Xho I restriction site.

An amplified product was detected by agarose gel electrophoresis. The fragment was gel-purified and ligated into the pCR2.1 vector (Invitrogen, Carlsbad, CA) following the manufacturer's recommendation. Twelve clones per PCR reaction were picked and sequenced. The inserts were sequenced using vector-specific M13 Forward and M13 Reverse primers and the following gene-specific primers:

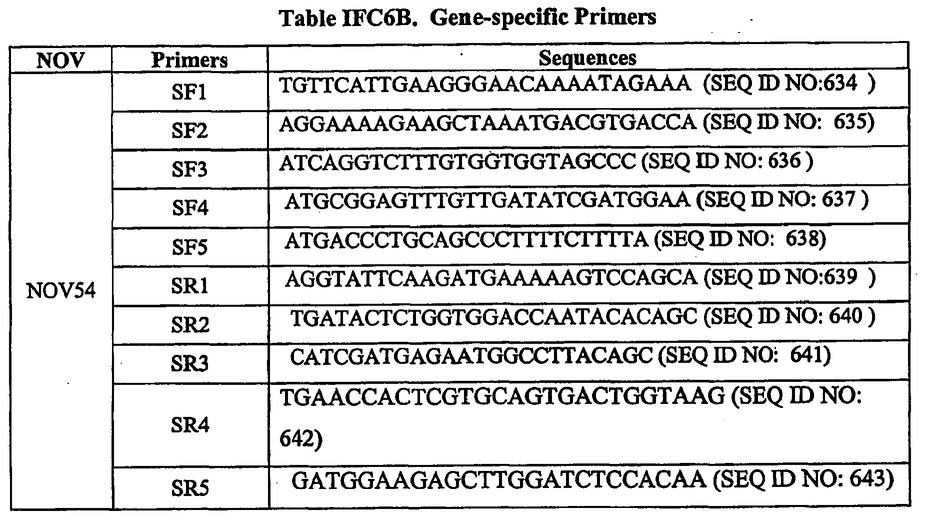

### NOV56

The cDNA coding for a domain of the full length CG57348-01 from residues 202 to 343 was targeted for "in-frame" cloning by PCR. The PCR template is based on human cDNA(s).

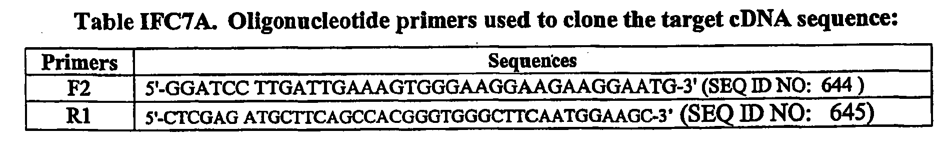

For downstream cloning purposes, the forward primer includes an in-frame BamHI restriction site and the reverse primer contains an in-frame Xho I restriction site.

An amplified product was detected by agarose gel electrophoresis. The fragment was gel-purified and ligated into the pCR2.1 vector (Invitrogen, Carlsbad, CA) following the manufacturer's recommendation, Twelve clones per PCR reaction were picked and sequenced. The inserts were sequenced using vector-specific M13 Forward and M13 Reverse primers and the following gene-specific primers:

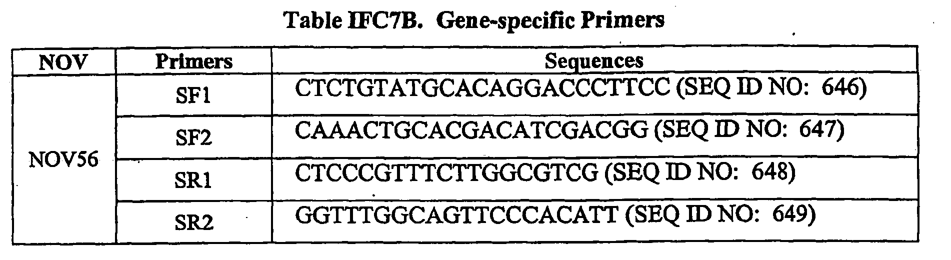

### NOV60

The cDNA coding for a domain fo the full length CG57574-01 from residue 18 to 275 was targeted for "in-frame" cloning by PCR. The PCR template is based on human cDNA(s).

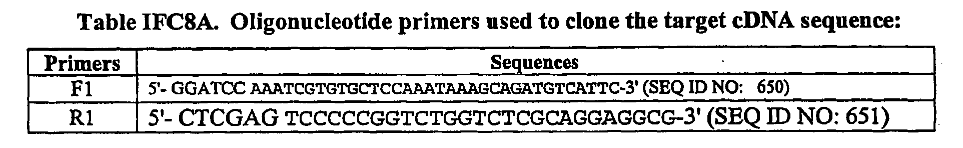

For downstream cloning purposes, the forward primer includes an in-frame BamHI restriction site and the reverse primer contains an in-frame Xho I restriction site.

An amplified product was detected by agarose gel electrophoresis. The fragment was gel-purified and ligated into the pCR2.1 vector (Invitrogen, Carlsbad, CA) following the manufacturer's recommendation. Twelve clones per PCR reaction were picked and sequenced. The inserts were sequenced using vector-specific M13 Forward and M13 Reverse primers and the following gene-specific primers:

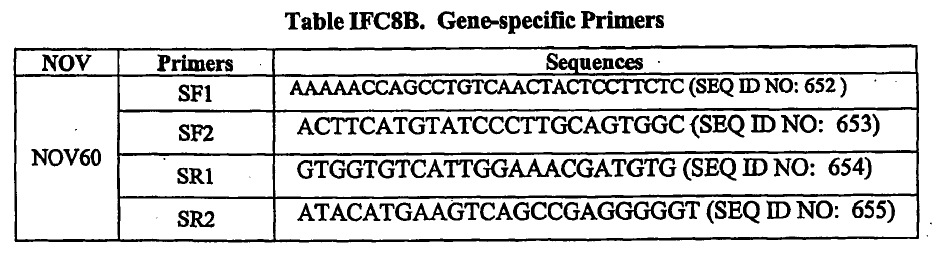

Two parallel PCR reactions were set up using a total of 0.5-1.0 ng human pooled cDNAs as template for each reaction. The pool is composed of 5 micrograms of each of the following human tissue cDNAs: adrenal gland, whole brain, amygdala, cerebellum, thalamus, bone marrow, fetal brain, fetal kidney, fetal liver, fetal lung, heart, kidney, liver, lymphoma, Burkitt's Raji cell line, mammary gland, pancreas, pituitary giana, placenta, prostate, sauvary gland, skeletal muscle, small Intestine, spleen, stomach, thyroid, trachea, uterus.

When the tissue of expression is known and available, the second PCR was performed using the above primers and 0.5ng-1.0 ng of one of the following human tissue cDNAs:
skeleton muscle, testis, mammary gland, adrenal gland, ovary, colon, normal cerebellum, normal adipose, normal skin, bone marrow, brain amygdala, brain hippocampus, brain substantia nigra, brain thalamus, thyroid, fetal lung, fetal liver, fetal brain, kidney, heart, spleen, uterus, pituitary gland, lymph, node, salivary gland, small intestine, prostate, placenta, spinal cord, peripheral blood, trachea, stomach, pancreas, hypothalamus.

The reaction mixtures contained 2 microliters of each of the primers (original concentration: 5 pmol/ul), 1 microliter of 10mM dNTP (Clontech Laboratories, Palo Alto CA) and 1 microliter of 50xAdvantage-HF 2 polymerase (Clontech Laboratories) in 50 microliter-reaction volume. The following reaction conditions were used:
PCR condition 1:
   a) 96°C 3 minutes
   b) 96°C 30 seconds denaturation
   c) 60°C 30 seconds, primer annealing
   d) 72°C 6 minutes extension
Repeat steps b-d 15 times
   e) 96°C 15 seconds denaturation
   f) 60°C 30 seconds, primer annealing
   g) 72°C 6 minutes extension
Repeat steps e-g 29 times
   e) 72°C 10 minutes final extension
PCR condition 2:
   a) 96°C 3 minutes
   b) 96°C 15 seconds denaturation
   c) 76°C 30 seconds, primer annealing, reducing the temperature by 1°C per cycle
   d) 72°C 4 minutes extension
Repeat steps b-d 34 times
   e) 72°C 10 minutes final extension

### OTHER EMBODIMENTS

Although particular embodiments have been disclosed herein in detail, this has been done by way of example for purposes of illustration only, and is not intended to be limiting with respect to the scope of the appended claims, which follow. In particular, it is contemplated by the inventors that various substitutions, alterations, and modifications may be made to the invention without departing from the spirit and scope of the invention as defined by the claims. The choice of nucleic acid starting material, clone of interest, or library type is believed to be a matter of routine for a person of ordinary skill in the art with knowledge of the embodiments described herein. Other aspects, advantages, and modifications considered to be within the scope of the following claims.

## Claims

1. An isolated polypeptide comprising an amino acid sequence selected from the group consisting of:
(a) a mature form of an amino acid sequence selected from the group consisting of SEQ ID NO: 48, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO: 132, SEQ ID NO:134, SEQ ID NO:136, SEQ ID NO:138, SEQ ID NO:140, SEQ ID NO:142, SEQ ID NO:144, SEQ ID NO:146, SEQ ID NO:148, SEQ ID NO:150, SEQ ID NO:152, SEQ ID NO:154, SEQ ID NO:156, SEQ ID NO:158, SEQ ID NO:160, SEQ ID NO:162, SEQ ID NO:164, SEQ ID NO:166, SEQ ID NO:168, SEQ ID NO:170, SEQ ID NO: 172, SEQ ID NO: 174, SEQ ID NO:176, SEQ ID NO:178, SEQ ID NO:180, SEQ ID NO:182, SEQ ID NO:184, SEQ ID NO:186, SEQ ID NO:188, SEQ ID NO:190, SEQ ID NO:192, SEQ ID NO:194, SEQ ID NO:196, SEQ ID NO:198, SEQ ID NO:200, SEQ ID NO:202, SEQ ID NO:204, SEQ ID NO:206, SEQ ID NO:208, SEQ ID NO:210, SEQ ID NO:212, and SEQ ID NO:214;
(b) a variant of a mature form of an amino acid sequence selected from the group consisting of SEQ ID NO:48, SEQ ID NO:2, SEQ ID NO:4, SEQ ID NO:6, SEQ ID NO:8, SEQ ID NO:10, SEQ ID NO:12, SEQ ID NO: 14, SEQ ID NO:16, SEQ ID NO:18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:144, SEQ ID NO:146, SEQ ID NO:148, SEQ ID NO:150, SEQ ID NO:152, SEQ ID NO:154, SEQ ID NO:156, SEQ ID NO:158, SEQ ID NO:160, SEQ ID NO:162, SEQ ID NO:164, SEQ ID NO:166, SEQ ID NO:168, SEQ ID NO:170, SEQ ID NO: 172, SEQ ID NO: 174, SEQ ID NO:176, SEQ ID NO:178, SEQ ID NO:180, SEQ ID NO:182, SEQ ID NO:184, SEQ ID NO:186, SEQ ID NO:188, SEQ ID NO:190, SEQ ID NO:192, SEQ ID NO:194, SEQ ID NO:196, SEQ ID NO:198, SEQ ID NO:200, SEQ ID NO:202, SEQ ID NO:204, SEQ ID NO:206, SEQ ID NO:208, SEQ ID NO:210, SEQ ID NO:212, and SEQ ID NO:214, wherein one or more amino acid residues in said variant differs from the amino acid sequence of said mature form, provided that said variant differs in no more than 15% of the amino acid residues from the amino acid sequence of said mature form;
(c) an amino acid sequence selected from the group consisting of SEQ ID NO: 48, SEQ ID NO:2, SEQ ID NO:4, SEQ ID NO:6, SEQ ID N0:8, SEQ ID NO:10, SEQ ID N0:12, SEQ ID NO: 14, SEQ ID NO: 16, SEQ ID NO: 18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO: 110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:144, SEQ ID NO:146, SEQ ID NO:148, SEQ ID NO:150, SEQ ID NO:152, SEQ ID NO:154, SEQ ID NO:156, SEQ ID NO:158, SEQ ID NO:160, SEQ ID NO:162, SEQ ID NO:164, SEQ ID NO:166, SEQ ID NO:168, SEQ ID NO:170, SEQ ID NO: 172, SEQ ID NO: 174, SEQ ID NO:176, SEQ ID NO:178, SEQ ID NO:180, SEQ ID NO:182, SEQ ID NO:184, SEQ ID NO:186, SEQ ID NO:188, SEQ ID NO:190, SEQ ID NO:192, SEQ ID NO:194, SEQ ID NO:196, SEQ ID NO:198, SEQ ID NO:200, SEQ ID NO:202, SEQ ID NO:204, SEQ ID NO:206, SEQ ID NO:208, SEQ ID NO:210, SEQ ID NO:212, and SEQ ID NO:214, and
(d) a variant of an amino acid sequence selected from the group consisting of SEQ ID NO:48, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID N0:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:144, SEQ ID NO:146, SEQ ID NO:148, SEQ ID NO:150, SEQ ID NO:152, SEQ ID NO:154, SEQ ID NO:156, SEQ ID NO:158, SEQ ID NO:160, SEQ ID NO:162, SEQ ID NO:164, SEQ ID NO:166, SEQ ID NO:168, SEQ ID NO:170, SEQ ID NO: 172, SEQ ID NO: 174, SEQ ID NO:176, SEQ ID NO:178, SEQ ID NO:180, SEQ ID NO:182, SEQ ID NO:184, SEQ ID NO:186, SEQ ID NO:188, SEQ ID NO:190, SEQ ID NO:192, SEQ ID NO:194, SEQ ID NO:196, SEQ ID NO:198, SEQ ID NO:200, SEQ ID NO:202, SEQ ID NO:204, SEQ ID NO:206, SEQ ID NO:208, SEQ ID NO:2 10, SEQ ID NO:212, and SEQ ID NO:214, wherein one or more amino acid residues in said variant differs from the amino acid sequence of said mature form, provided that said variant differs in no more than 15% of amino acid residues from said amino acid sequence.

2. The polypeptide of claim 1, wherein said polypeptide comprises the ammo acid sequence of a naturally-occurring allelic variant of an amino acid sequence selected from the group consisting SEQ ID NO: 48, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO: 18, SEQ ID NO:20, SEQ ID NO:22, SEQ ID NO:24, SEQ ID NO:26, SEQ NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO: 108, SEQ ID NO: *110,* SEQ ID NO: 112, SEQ ID NO: 114, SEQ ID NO: 116, SEQ ID NO:118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, SEQ ID NO: 136, SEQ ID NO: 138, SEQ ID NO: 140, SEQ ID NO: 142, SEQ ID NO: 144, SEQ ID NO: 146, SEQ ID NO: 148, SEQ ID NO:150, SEQ ID NO: 152, SEQ ID NO: 154, SEQ ID NO: 156, SEQ ID NO:158, SEQ ID NO: 160, SEQ ID NO:162, SEQ ID NO: 164, SEQ ID NO: 166, SEQ ID NO: 168, SEQ ID NO: 170, SEQ ID NO: 172, SEQ ID NO: 174, SEQ ID NO: 176, SEQ ID NO:178, SEQ ID NO:180, SEQ ID NO:182, SEQ ID NO:184, SEQ ID NO:186, SEQ ID NO:188, SEQ ID NO:190, SEQ ID NO:192, SEQ ID NO:194, SEQ ID NO:196, SEQ ID NO:198, SEQ ID NO:200, SEQ ID NO:202, SEQ ID NO:204, SEQ ID NO:206, SEQ ID NO:208, SEQ ID NO:210, SEQ ID NO:212, and SEQ ID NO:214.

3. The polypeptide of claim 2, wherein said allelic variant comprises an amino acid sequence that is the translation of a nucleic acid sequence differing by a single nucleotide from a nucleic acid sequence selected from the group consisting of SEQ ID NO: 47, SEQ ID NO:1, SEQ ID NO:3, SEQ ID N0:5, SEQ ID N0:7, SEQ ID N0:9, SEQ ID NO:11, SEQ ID N0:13, SEQ ID N0:15, SEQ ID N0:17, SEQ ID N0:19, SEQ ID N0:21, SEQ ID N0:23, SEQ ID N0:25, SEQ ID N0:27, SEQ ID N0:29, SEQ ID N0:31, SEQ ID NO:33, SEQ ID NO:35, SEQ ID N0:37, SEQ ID N0:39, SEQ ID NO:41, SEQ ID N0:43, SEQ ID N0:45, SEQ ID N0:49, SEQ ID NO:51, SEQ ID N0:53, SEQ ID N0:55, SEQ ID N0:57, SEQ ID N0:59, SEQ ID N0:61, SEQ ID NO:63, SEQ ID NO:65, SEQ ID NO:67, SEQ ID N0:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID N0:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:l27, SEQ ID NO:129, SEQ ID NO:131, SEQ ID NO:133, SEQ ID NO:135, SEQ ID NO:137, SEQ ID NO:139, SEQ ID NO:141, SEQ ID NO:143, SEQ ID NO:145, SEQ ID NO:147, SEQ ID NO:149, SEQ ID NO:151, SEQ ID NO:153, SEQ ID NO:155, SEQ ID NO:157, SEQ ID NO:159, SEQ ID NO:161, SEQ ID NO:163, SEQ ID NO:165, SEQ ID NO:167, SEQ ID NO:169, SEQ ID NO:171, SEQ ID NO:173, SEQ ID NO:175, SEQ ID NO: 177, SEQ ID NO:179, SEQ ID NO: 181, SEQ ID NO:183, SEQ ID NO:185, SEQ ID NO:187, SEQ ID NO:189, SEQ ID NO:191, SEQ ID NO:193, SEQ ID NO:195, SEQ ID NO:197, SEQ ID NO:199, SEQ ID NO:201, SEQ ID NO:203, SEQ ID NO:205, SEQ ID NO:207, SEQ ID NO:209, SEQ ID NO:21 1, and SEQ ID NO:213.

4. The polypeptide of claim 1, wherein the amino acid sequence of said variant comprises a conservative amino acid substitution.

5. An isolated nucleic acid molecule comprising a nucleic acid sequence encoding a polypeptide comprising an amino acid sequence selected from the group consisting of:
(a) a mature form of an amino acid sequence selected from the group consisting of SEQ ID NO: 48, SEQ ID NO:2, SEQ ID NO:4, SEQ ID NO:6, SEQ ID NO:8, SEQ ID NO: 10, SEQ ID NO:12, SEQ ID NO:14, SEQ ID NO:16, SEQ ID NO:18, SEQ ID NO:20, SEQ ID NO:22, SEQ ID NO:24, SEQ ID NO:26, SEQ NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO: 112, SEQ ID NO:114, SEQ ID NO:116, SEQ ID NO:118, SEQ ID NO:120, SEQ ID NO: 122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:144, SEQ ID NO:146, SEQ ID NO:148, SEQ ID NO:150, SEQ ID NO:152, SEQ ID NO:154, SEQ ID NO:156, SEQ ID NO:158, SEQ ID NO:160, SEQ ID NO:162, SEQ ID NO:164, SEQ ID NO:166, SEQ ID NO:168, SEQ ID NO:170, SEQ ID NO: 172, SEQ ID NO: 174, SEQ ID NO:176, SEQ ID NO:178, SEQ ID NO:180, SEQ ID NO:182, SEQ ID NO:184, SEQ ID NO:186, SEQ ID NO:188, SEQ ID NO:190, SEQ ID NO:192, SEQ ID NO:194, SEQ ID NO:196, SEQ ID NO:198, SEQ ID NO:200, SEQ ID NO:202, SEQ ID NO:204, SEQ ID NO:206, SEQ ID NO:208, SEQ ID NO:210, SEQ ID NO:212 and SEQ ID NO:214.
(b) a variant of a mature form of an amino acid sequence selected from the group consisting of SEQ ID NO: 48, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO: 18, SEQ ID N0:20, SEQ ID NO:22, SEQ ID NO:24, SEQ ID NO:26, SEQ NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO: 130, SEQ ID NO: 132, SEQ ID NO: 134, SEQ ID NO:136, SEQ ID NO: 138, SEQ ID NO:140, SEQ ID NO:142, SEQ ID NO:144, SEQ ID NO:146, SEQ ID NO:148, SEQ ID NO:150, SEQ ID NO: 152, SEQ ID NO: 154, SEQ ID NO: 156, SEQ ID NO:158, SEQ ID NO:160, SEQ ID NO:162, SEQ ID NO:164, SEQ ID NO: 166, SEQ ID NO:168, SEQ ID NO:170, SEQ ID NO: 172, SEQ ID NO: 174, SEQ ID NO: 176, SEQ ID NO: 178, SEQ ID NO: 180, SEQ ID NO:182, SEQ ID NO:184, SEQ ID NO:186, SEQ ID NO:188, SEQ ID NO:190, SEQ ID NO:192, SEQ ID NO:194, SEQ ID NO:196, SEQ ID NO:198, SEQ ID NO:200, SEQ ID NO:202, SEQ ID NO:204, SEQ ID NO:206, SEQ ID NO:208, SEQ ID NO:210, SEQ ID NO:212, and SEQ ID NO:214, wherein one or more amino acid residues in said variant differs from the amino acid sequence of said mature form, provided that said variant differs in no more than 15% of the amino acid residues from the ammo acid sequence of said mature form;
(c) an amino acid sequence selected from the group consisting of SEQ ID NO: 48, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ NO:26, SEQ NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO: 102, SEQ ID NO: 104, SEQ ID NO: 106, SEQ ID NO:108, SEQ ID NO:110, SEQ ID NO:112, SEQ ID NO:114, SEQ ID NO: 116, SEQ ID NO:118, SEQ ID NO: 120, SEQ ID NO: 122, SEQ ID NO: 124, SEQ ID NO: 126, SEQ ID NO:128, SEQ ID NO:130, SEQ ID NO: 132, SEQ ID NO: 134, SEQ ID NO:136, SEQ ID NO: 138, SEQ ID NO: 140, SEQ ID NO: 142, SEQ ID NO: 144, SEQ ID NO:146, SEQ ID NO: 148, SEQ ID NO:150, SEQ ID NO: 152, SEQ ID NO: 154, SEQ ID NO: 156, SEQ ID NO: 15 8, SEQ ID NO: 160, SEQ ID NO:162, SEQ ID NO:164, SEQ ID NO:166, SEQ ID NO:168, SEQ ID NO:170, SEQ ID NO: 172, SEQ ID NO: 174, SEQ ID NO:176, SEQ ID NO:178, SEQ ID NO: 180, SEQ ID N0:182, SEQ ID NO: 184, SEQ ID NO: 186, SEQ ID NO: 188, SEQ ID NO: 190, SEQ ID NO:192, SEQ ID NO:194, SEQ ID NO:196, SEQ ID NO:198, SEQ ID NO:200, SEQ ID NO:202, SEQ ID NO:204, SEQ ID NO:206, SEQ ID NO:208, SEQ ID NO:210, SEQ ID NO:212, and SEQ ID NO:214.
(d) a variant of an amino acid sequence selected from the group consisting SEQ ID NO: 48, SEQ ID NO:2, SEQ ID NO:4, SEQ ID NO:6, SEQ ID NO:8, SEQ ID NO:10, SEQ ID NO:12, SEQ ID NO:14, SEQ ID NO:16, SEQ ID NO:18, SEQ ID NO:20, SEQ ID NO:22, SEQ ID NO:24, SEQ NO:26, SEQ NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO: 106, SEQ ID NO: 108, SEQ ID NO:110, SEQ ID NO:112, SEQ ID NO:114, SEQ ID NO: 116, SEQ ID NO: 118, SEQ ID NO:120, SEQ ID NO:122, SEQ ID NO:124, SEQ ID NO: 126, SEQ ID NO: 12 8, SEQ ID NO: 130, SEQ ID NO: 132, SEQ ID NO:134, SEQ ID NO: 136, SEQ ID NO: 13 8, SEQ ID NO: 140, SEQ ID NO: 142, SEQ ID NO:144, SEQ ID NO: 146, SEQ ID NO: 148, SEQ ID NO: 150, SEQ ID NO:152, SEQ ID NO:154, SEQ ID NO:156, SEQ ID NO:158, SEQ ID NO:160, SEQ ID NO:162, SEQ ID NO:164, SEQ ID NO: 166, SEQ ID NO: 168, SEQ ID NO:170, SEQ ID NO: 172, SEQ ID NO: 174, SEQ ID NO:176, SEQ ID NO:178, SEQ ID NO:180, SEQ ID NO:182, SEQ ID NO:184, SEQ ID NO:186, SEQ ID NO:188, SEQ ID NO:190, SEQ ID NO:192, SEQ ID NO:194, SEQ ID NO:196, SEQ ID NO:198, SEQ ID NO:200, SEQ ID NO:202, SEQ ID NO:204, SEQ ID NO:206, SEQ ID NO:208, SEQ ID NO:210, SEQ ID NO:212 and SEQ ID NO:214, wherein one or more amino acid
residues in said variant differs from the amino acid sequence of said mature form, provided that said variant differs in no more than 15% of amino acid residues from said amino acid sequence;
(e) a nucleic acid fragment encoding at least a portion of a polypeptide comprising an amino acid sequence chosen from the group consisting of SEQ ID NO: 48, SEQ ID NO:2, SEQ ID NO:4, SEQ ID NO:6, SEQ ID NO:8, SEQ ID NO:10, SEQ ID NO: 12, SEQ ID NO:14, SEQ ID NO:16, SEQ ID NO: 18, SEQ ID NO: 20, SEQ ID NO: 22, SEQ ID NO:24, SEQ NO:26, SEQ NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:44, SEQ ID NO:46, SEQ ID NO:50, SEQ ID NO:52, SEQ ID NO:54, SEQ ID NO:56, SEQ ID NO:58, SEQ ID NO:60, SEQ ID NO:62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID NO:80, SEQ ID NO:82, SEQ ID NO:84, SEQ ID NO:86, SEQ ID NO:88, SEQ ID NO:90, SEQ ID NO:92, SEQ ID NO:94, SEQ ID NO:96, SEQ ID NO:98, SEQ ID NO:100, SEQ ID NO:102, SEQ ID NO:104, SEQ ID NO:106, SEQ ID NO:108, SEQ ID NO:110, SEQ ID NO:112, SEQ ID NO:114, SEQ ID NO:116, SEQ ID NO:118, SEQ ID NO:120, SEQ ID NO:122, SEQ ID NO:124, SEQ ID NO:126, SEQ ID NO:128, SEQ ID NO:130, SEQ ID NO:132, SEQ ID NO:134, SEQ ID NO:136, SEQ ID NO:138, SEQ ID NO:140, SEQ ID NO:142, SEQ ID NO:144, SEQ ID NO:146, SEQ ID NO:148, SEQ ID NO:150, SEQ ID NO:152, SEQ ID NO:154, SEQ ID NO:156, SEQ ID NO:158, SEQ ID NO:160, SEQ ID NO:162, SEQ ID NO:164, SEQ ID NO:166, SEQ ID NO:168, SEQ ID NO:170, SEQ ID NO:172, SEQ ID NO:174, SEQ ID NO:176, SEQ ID NO:178, SEQ ID NO:180, SEQ ID NO:182, SEQ ID NO:184, SEQ ID NO:186, SEQ ID NO:188, SEQ ID NO:190, SEQ ID NO:192, SEQ ID NO:194, SEQ ID NO:196, SEQ ID NO:198, SEQ ID NO:200, SEQ ID NO:202, SEQ ID NO:204, SEQ ID NO:206, SEQ ID NO:208, SEQ ID NO:210, SEQ ID NO:212, and SEQ ID NO:214, or a variant of said polypeptide, wherein one or more amino acid residues in said variant differs from the amino acid sequence of said mature form, provided that said variant differs in no more than 15% of amino acid residues from said amino acid sequence; and
(f) a nucleic acid molecule comprising the complement of (a), (b), (c), (d) or (e).

6. The nucleic acid molecule of claim 5, wherein the nucleic acid molecule comprises the nucleotide sequence of a naturally-occurring allelic nucleic acid variant.

7. The nucleic acid molecule of claim 5, wherein the nucleic acid molecule encodes a polypeptide comprising the amino acid sequence of a naturally-occurring polypeptide variant.

8. The nucleic acid molecule of claim 5, wherein the nucleic acid molecule differs by a single nucleotide from a nucleic acid sequence selected from the group consisting of SEQ ID NO: 47, SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO: 117, SEQ ID NO: 119, SEQ ID NO:121, SEQ ID NO:123, SEQ ID NO:125, SEQ ID NO: 127, SEQ ID NO:129, SEQ ID NO:131, SEQ ID NO: 133, SEQ ID NO: 135, SEQ ID NO: 137, SEQ ID NO:139, SEQ ID NO: 14 1, SEQ ID NO:143, SEQ ID NO: 145, SEQ ID NO: 147, SEQ ID NO: 149, SEQ ID NO:151, SEQ ID NO: 153, SEQ ID NO:155, SEQ ID NO:157, SEQ ID NO: 159, SEQ ID NO:161, SEQ ID NO: 163, SEQ ID NO: 165, SEQ ID NO: 167, SEQ ID NO: 169, SEQ ID NO:171, SEQ ID NO:173, SEQ ID NO: 175, SEQ ID NO:177, SEQ ID NO: 179, SEQ ID NO: 18 1, SEQ ID NO:183, SEQ ID NO:185, SEQ ID NO: 187, SEQ ID NO:189, SEQ ID NO:191, SEQ ID NO: 193, SEQ ID NO:195, SEQ ID NO: 197, SEQ ID NO: 199, SEQ ID NO:201, SEQ ID NO:203, SEQ ID NO:205, SEQ ID NO:207, SEQ ID NO:209, SEQ ID NO:211, and SEQ ID NO:213,

9. The nucleic acid molecule of claim 5, wherein said nucleic acid molecule comprises a nucleotide sequence selected from the group consisting of:
(a) a nucleotide sequence selected from the group consisting of SEQ ID NO: 47, SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:4 SEQ ID NO:43, SEQ ID NO:45, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO: 109, SEQ ID NO:111, SEQ ID NO: 113, SEQ ID NO: 115, SEQ ID NO: 117, SEQ ID NO: 119, SEQ ID NO: 121, SEQ ID NO: 123, SEQ ID NO: 125, SEQ ID NO: 127, SEQ ID NO: 129, SEQ ID NO: 131, SEQ ID NO: 133, SEQ ID NO: 135, SEQ ID NO: 137, SEQ ID NO:139, SEQ ID NO: 141, SEQ ID NO: 143, SEQ ID NO: 145, SEQ ID NO: 147, SEQ ID NO:149, SEQ ID NO:151, SEQ ID NO: 153, SEQ ID NO: 155, SEQ ID NO: 157, SEQ ID NO: 15 9, SEQ ID NO: 161, SEQ ID NO: 163, SEQ ID NO: 165, SEQ ID NO: 167, SEQ ID NO: 169, SEQ ID NO: 171, SEQ ID NO:173, SEQ ID NO:175, SEQ ID NO:177, SEQ ID NO:179, SEQ ID NO:181, SEQ ID NO:183, SEQ ID NO:185, SEQ ID NO:187, SEQ ID NO:189, SEQ ID NO:191, SEQ ID NO:193, SEQ ID NO:195, SEQ ID NO:197, SEQ ID NO:199, SEQ ID NO:201, SEQ ID NO:203, SEQ ID NO:205, SEQ ID NO:207, SEQ ID NO:209, SEQ ID NO:211, and SEQ ID NO:213.
(b) a nucleotide sequence differing by one or more nucleotides from a nucleotide sequence selected from the group consisting of SEQ ID NO: 47, SEQ ID NO:1, SEQ ID NO:3, SEQ ID NO:5, SEQ ID NO:7, SEQ ID NO: 9, SEQ ID NO:11, SEQ ID NO: 13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:45, SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO:115, SEQ ID NO:117, SEQ ID NO:119, SEQ ID NO:121, SEQ ID NO:123, SEQ ID NO:125, SEQ ID NO:127, SEQ ID NO:129, SEQ ID NO:131, SEQ ID NO:133, SEQ ID NO:135, SEQ ID NO:137, SEQ ID NO:139, SEQ ID NO:141, SEQ ID NO:143, SEQ ID NO:145, SEQ ID NO:147, SEQ ID NO:149, SEQ ID NO:151, SEQ ID NO:153, SEQ ID NO:155, SEQ ID NO:157, SEQ ID NO:159, SEQ ID NO:161, SEQ ID NO:163, SEQ ID NO:165, SEQ ID NO:167, SEQ ID NO:169, SEQ ID NO:171, SEQ ID NO:173, SEQ ID NO:175, SEQ ID NO:177, SEQ ID NO:179, SEQ ID NO:181, SEQ ID NO:183, SEQ ID NO:185, SEQ ID NO:187, SEQ ID NO:189, SEQ ID NO:191, SEQ ID NO:193, SEQ ID NO:195, SEQ ID NO:197, SEQ ID NO:199, SEQ ID N0:201, SEQ ID N0:203, SEQ ID NO:205, SEQ ID NO:207, SEQ ID NO:209, SEQ ID NO:211, and SEQ ID NO:213, provided that no more than 20% of the nucleotides differ from said nucleotide sequence;
(c) a nucleic acid fragment of (a); and
(d) a nucleic acid fragment of (b).

10. The nucleic acid molecule of claim 5, wherein said nucleic acid molecule hybridizes under stringent conditions to a nucleotide sequence chosen from the group consisting SEQ ID NO: 47, SEQ ID NO:1, SEQ ID NO:3, SEQ ID NO:5, SEQ ID NO:7, SEQ ID NO:9, SEQ ID NO:11, SEQ ID NO:13, SEQ ID NO:15, SEQ ID NO:17, SEQ ID NO:19, SEQ ID NO:21, SEQ ID NO:23, SEQ ID NO:25, SEQ ID NO:27, SEQ ID NO:29, SEQ ID NO:31, SEQ ID NO:33, SEQ ID NO:35, SEQ ID NO:37, SEQ ID NO:39, SEQ ID NO:41, SEQ ID NO:43, SEQ ID NO:4 SEQ ID NO:49, SEQ ID NO:51, SEQ ID NO:53, SEQ ID NO:55, SEQ ID NO:57, SEQ ID NO:59, SEQ ID NO:61, SEQ ID NO:63, SEQ ID NO:65, SEQ ID NO:67, SEQ ID NO:69, SEQ ID NO:71, SEQ ID NO:73, SEQ ID NO:75, SEQ ID NO:77, SEQ ID NO:79, SEQ ID NO:81, SEQ ID NO:83, SEQ ID NO:85, SEQ ID NO:87, SEQ ID NO:89, SEQ ID NO:91, SEQ ID NO:93, SEQ ID NO:95, SEQ ID NO:97, SEQ ID NO:99, SEQ ID NO:101, SEQ ID NO:103, SEQ ID NO:105, SEQ ID NO:107, SEQ ID NO:109, SEQ ID NO:111, SEQ ID NO:113, SEQ ID NO: 115, SEQ ID NO: 117, SEQ ID N0:119, SEQ ID NO:121, SEQ ID NO:123, SEQ ID N0:125, SEQ ID NO:127, SEQ ID N0:129, SEQ ID N0:131, SEQ ID N0:133, SEQ ID N0:135, SEQ ID NO:137, SEQ ID N0:139, SEQ ID N0:141, SEQ ID NO:143, SEQ ID N0:145, SEQ ID N0:147, SEQ ID N0:149, SEQ ID N0:151, SEQ ID NO:153, SEQ ID N0:155, SEQ ID NO:157, SEQ ID NO:159, SEQ ID NO:161, SEQ ID N0:163, SEQ ID NO:165, SEQ ID N0:167, SEQ ID N0:169, SEQ ID NO:171, SEQ ID N0:173, SEQ ID N0:175, SEQ ID N0:177, SEQ ID NO:179, SEQ ID NO:181, SEQ ID NO:183, SEQ ID NO:185, SEQ ID NO:187, SEQ ID N0:189, SEQ ID NO:191, SEQ ID N0:193, SEQ ID NO:195, SEQ ID N0:197, SEQ ID N0:199, SEQ ID NO:201, SEQ ID N0:203, SEQ ID NO:205, SEQ ID NO:207, SEQ ID N0:209, SEQ ID N0:211, and SEQ ID N0:213, or a complement of said nucleotide sequence.

11. The nucleic acid molecule of claim 5, wherein the nucleic acid molecule comprises a nucleotide sequence selected from the group consisting of:
(a) a first nucleotide sequence comprising a coding sequence differing by one or more nucleotide sequences from a coding sequence encoding said amino acid sequence, provided that no more than 20% of the nucleotides in the coding sequence in said first nucleotide sequence differ from said coding sequence;
(b) an isolated second polynucleotide that is a complement of the first polynucleotide; and
(c) a nucleic acid fragment of (a) or (b).

12. A vector comprising the nucleic acid molecule of claim 11.

13. The vector of claim 12, further comprising a promoter operably-linked to said nucleic acid molecule.

14. A cell comprising the vector of claim 12.

15. An antibody that binds immunospecifically to the polypeptide of claim 1.

16. The antibody of claim 15, wherein said antibody is a monoclonal antibody.

17. The antibody of claim 15, wherein the antibody is a humanized antibody.

18. A method for determining the presence or amount of the polypeptide of claim 1 in a sample, the method comprising:
(a) providing the sample;
(b) contacting the sample with an antibody that binds immunospecifically to the polypeptide; and
(c) determining the presence or amount of antibody bound to said polypeptide, thereby determining the presence or amount of polypeptide in said sample.

19. A method for determining the presence or amount of the nucleic acid molecule of claim 5 in a sample, the method comprising:
(a) providing the sample;
(b) contacting the sample with a probe that binds to said nucleic acid molecule; and
(c) determining the presence or amount of the probe bound to said nucleic acid molecule,
thereby determining the presence or amount of the nucleic acid molecule in said sample.

20. The method of claim 19 wherein presence or amount of the nucleic acid molecule is used as a marker for cell or tissue type.

21. The method of claim 20 wherein the cell or tissue type is cancerous.

22. A method of identifying an agent that binds to a polypeptide of claim 1, the method comprising:
(a) contacting said polypeptide with said agent; and
(b) determining whether said agent binds to said polypeptide.

23. The method of claim 22 wherein the agent is a cellular receptor or a downstream effector.

24. A method for identifying an agent that modulates the expression or activity of the polypeptide of claim 1, the method comprising:
(a) providing a cell expressing said polypeptide;
(b) contacting the cell with said agent, and
(c) determining whether the agent modulates expression or activity of said polypeptide,
whereby an alteration in expression or activity of said peptide indicates said agent modulates expression or activity of said polypeptide.

25. A method for modulating the activity of the polypeptide of claim 1, the method comprising contacting a cell sample expressing the polypeptide of said claim with a compound that binds to said polypeptide in an amount sufficient to modulate the activity of the polypeptide.

26. A method of treating or preventing a NOVX-associated disorder, said method comprising administering to a subject in which such treatment or prevention is desired the polypeptide of claim 1 in an amount sufficient to treat or prevent said NOVX-associated disorder in said subject.

27. The method of claim 26 wherein the disorder is selected from the group consisting of cardiomyopathy and atherosclerosis.

28. The method of claim 26 wherein the disorder is related to cell signal processing and metabolic pathway modulation.

29. The method of claim 26, wherein said subject is a human.

30. A method of treating or preventing a NOVX-associated disorder, said method comprising administering to a subject in which such treatment or prevention is desired the nucleic acid of claim 5 in an amount sufficient to treat or prevent said NOVX-associated disorder in said subject.

31. The method of claim 30 wherein the disorder is selected from the group consisting of cardiomyopathy and atherosclerosis.

32. The method of claim 30 wherein the disorder is related to cell signal processing and metabolic pathway modulation.

33. The method of claim 30, wherein said subject is a human.

34. A method of treating or preventing a NOVX-associated disorder, said method comprising administering to a subject in which such treatment or prevention is desired the antibody of claim 15 in an amount sufficient to treat or prevent said NOVX-associated disorder in said subject.

35. The method of claim 34 wherein the disorder is diabetes.

36. The method of claim 34 wherein the disorder is related to cell signal processing and metabolic pathway modulation.

37. The method of claim 34, wherein the subject is a human.

38. A pharmaceutical composition comprising the polypeptide of claim 1 and a pharmaceutically-acceptable carrier.

39. A pharmaceutical composition comprising the nucleic acid molecule of claim 5 and a pharmaceutically-acceptable carrier.

40. A pharmaceutical composition comprising the antibody of claim 15 and a pharmaceutically-acceptable carrier.

41. A kit comprising in one or more containers, the pharmaceutical composition of claim 38.

42. A kit comprising in one or more containers, the pharmaceutical composition of claim 39.

43. A kit comprising in one or more containers, the pharmaceutical composition of claim 40.

44. A method for determining the presence of or predisposition to a disease associated with altered levels of the polypeptide of claim 1 in a first mammalian subject, the method comprising:
(a) measuring the level of expression of the polypeptide in a sample from the first mammalian subject; and
(b) comparing the amount of said polypeptide in the sample of step (a) to the amount of the polypeptide present in a control sample from a second mammalian subject known not to have, or not to be predisposed to, said disease;
wherein an alteration in the expression level of the polypeptide in the first subject as compared to the control sample indicates the presence of or predisposition to said disease.

45. The method of claim 44 wherein the predisposition is to a cancer.

46. A method for determining the presence of or predisposition to a disease associated with altered levels of the nucleic acid molecule of claim 5 in a first mammalian subject, the method comprising:
(a) measuring the amount of the nucleic acid in a sample from the first mammalian subject; and
(b) comparing the amount of said nucleic acid in the sample of step (a) to the amount of the nucleic acid present in a control sample from a second mammalian subject known not to have or not be predisposed to, the disease;
wherein an alteration in the level of the nucleic acid in the first subject as compared to the control sample indicates the presence of or predisposition to the disease.

47. The method of claim 46 wherein the predisposition is to a cancer.

48. A method of treating a pathological state in a mammal, the method comprising administering to the mammal a polypeptide in an amount that is sufficient to alleviate the pathological state, wherein the polypeptide is a polypeptide having an amino acid sequence at least 95% identical to a polypeptide comprising an amino acid sequence of at least one of SEQ ID NO: 48, SEQ ID NO: 2, SEQ ID NO:4, SEQ ID NO:6, SEQ ID NO:8, SEQ ID NO: 10, SEQ ID NO: 12, SEQ ID NO: 14, SEQ ID NO:16, SEQ ID NO: 18, SEQ ID NO:20, SEQ ID NO:22, SEQ ID NO:24, SEQ ID NO: 26, SEQ ID NO:28, SEQ ID NO:30, SEQ ID NO:32, SEQ ID NO:34, SEQ ID NO:36, SEQ ID NO:38, SEQ ID NO:40, SEQ ID NO:42, SEQ ID NO:4 SEQ ID NO:46, SEQ ID NO:50, SEQ ID N0:52, SEQ ID NO:54, SEQ ID NO:56, SEQ ID NO:58, SEQ ID N0:60, SEQ ID NOs62, SEQ ID NO:64, SEQ ID NO:66, SEQ ID NO:68, SEQ ID NO:70, SEQ ID NO:72, SEQ ID NO:74, SEQ ID NO:76, SEQ ID NO:78, SEQ ID N0:80, SEQ ID N0:82, SEQ ID NO:84, SEQ ID NO:86, SEQ ID N0:88, SEQ ID N0:90, SEQ ID N0:92, SEQ ID NO:94, SEQ ID N0:96, SEQ ID N0:98, SEQ ID NO:100, SEQ ID NO:102, SEQ ID NO:104, SEQ ID NO:106, SEQ ID NO:108, SEQ ID NO:110, SEQ ID N0:112, SEQ ID NO:114, SEQ ID N0:116, SEQ ID N0:118, SEQ ID NO:120, SEQ ID N0:122, SEQ ID NO:124, SEQ ID NO:126, SEQ ID NO:128, SEQ ID N0:130, SEQ ID N0:132, SEQ ID N0:134, SEQ ID NO:136, SEQ ID N0:138, SEQ ID N0:140, SEQ ID N0:142, SEQ ID N0:144, SEQ ID N0:146, SEQ ID NO:148, SEQ ID N0:150, SEQ ID N0:152, SEQ ID N0:154, SEQ ID N0:156, SEQ ID N0:158, SEQ ID N0:160, SEQ ID N0:162, SEQ ID NO:164, SEQ ID N0:166, SEQ ID N0:168, SEQ ID N0:170, SEQ ID NO: 172, SEQ ID NO: 174, SEQ ID N0:176, SEQ N0:188, SEQ ID N0:190, SEQ ID N0:192, SEQ ID N0:194, SEQ ID N0:196, SEQ ID NO:198, SEQ ID N0:200, SEQ ID N0:202, SEQ ID N0:204, SEQ ID N0:206, SEQ ID N0:208, SEQ ID N0:210, SEQ ID NO:212, and SEQ ID N0:214, or a biologically active fragment thereof.

49. A method of treating a pathological state in a mammal, the method comprising administering to the mammal the antibody of claim 15 in an amount sufficient to alleviate the pathological state.
